# EUROPÄISCHE PATENTANMELDUNG

(11) **EP 1 260 592 A1**
(43) Veröffentlichungstag der Anmeldung: **27.11.2002**
(21) Anmeldenummer: 01112179.5
(22) Anmeldetag: 17.05.2001
(51) Int. Cl.: C12Q 1/68

(54) **Biochip**

(71) Anmelder: MWG -Biotech AG, 85560 Ebersberg (DE)
(72) Erfinder: Donner, Horst, 85570 Markt Schwaben (DE); Drescher, Bernd, 69221 Dossenheim (DE); Huber, Andrea, 83026 Rosenheim (DE); Weber, Jaqueline, 85661 Fortinning (DE)
(74) Vertreter: Ganahl, Bernhard

(57) **Zusammenfassung**

1. Biochip für den möglichst umfassenden Nachweis des Genoms von *Escherichia Coli* K12.

2.1 Es sollen Vorrichtungen zur Verfügung gestellt werden, mit deren Hilfe eine möglichst umfassende Analyse des Genoms des *Escherichia Coli* -Stammes *Escherichia Coli* K12 ermöglicht wird.

2.2 Es wird ein Biochip mit einer Vielzahl von Sondenpunkten bereitgestellt, bei dem in jedem Sondenpunkt eine Sonde aufgebracht ist, jede Sonde eine Vielzahl von Sondenmolekülen aufweist, und alle Sondenmoleküle einer Sonde eine identische Sondensequenz besitzen, die Sondensequenzen Nukleotidsequenzen sind, und zumindest eine der Sonden Sondensequenzen mit einer Länge von mindestens 20 Basen aufweist, die identisch mit oder komplementär zu einem Abschnitt eines Open Reading Frames von *Escherichia Coli* K12 ist.

2.3 Ein erfindungsgemässer Biochip eignet sich für den spezifischen Nachweis von Expressionen von Genen von *Escherichia Coli* K12 und die Erstellung von Genexpressionsmustern von *Escherichia Coli* K12.

<110> MWG Biotech AG
<120> Oligonukleotidchip
<130> EP-3015
<160> 12812
<210> 1
<211> 100
<212> DNA
<213> Escherichia coli K-12 MG1655
<220> b1360 b1361
<222> 648-747
<223> b1360 b1361 U00096 1420007..1420753
<313> 1: 719-738
<400> 1
GCGGATTATG GATCGCATGA CCATGAACGG CGGGCGATGG GTGAATTTTA 50
ACTGGGAGAG CTGGCGTCCG AATGTCGTCC AGCCAGGAAT TGCGAAGTAA 100
<210> 2
<211> 100
<212> DNA
<213> Escherichia coli K-12 MG1655
<220> b1360 b1361
<222> 635-734
<223> b1360 b1361 U00096 1420007..1420753
<313> 2: 676-695
<400> 2
CATTGCTCGG CGAGCGGATT ATGGATCGCA TGACCATGAA CGGCGGGCGA 50
TGGGTGAATT TTAACTGGGA GAGCTGGCGT CCGAATGTCG TCCAGCCAGG 100
<210> 3
<211> 100
<212> DNA
<213> Escherichia coli K-12 MG1655
<220> b1360 b1361
<222> 594-693
<223> b1360 b1361 U00096 1420007..1420753
<313> 3: 608-627
<400> 3
CGTGGGGATG CTGACAAACC TGAACTATGA GGCCATGAAA ACATTGCTCG 50
GCGAGCGGAT TATGGATCGC ATGACCATGA ACGGCGGGCG ATGGGTGAAT 100
<210> 4
<211> 100
<212> DNA
<213> Escherichia coli K-12 MG1655
<220> mlta b2814
<222> 958-1057
<223> mlta b2814 U00096 complement(2944103..2945200)
<313> 4: 1011-1030
<400> 4
GGTGCAATCA AAGGCCAACA CTTCGATATC TATCAAGGGA TCGGGCCGGA 50
AGCCGGACAC CGCGCAGGTT GGTACAACCA CTATGGACGT GTCTGGGTGC 100
<210> 5
<211> 100
<212> DNA
<213> Escherichia coli K-12 MG1655
<220> mlta b2814
<222> 927-1026
<223> mlta b2814 U00096 complement(2944103..2945200)
<313> 5: 967-986
<400> 5
ACTGCGTCTG ATGGTGGCGC TGGATGTCGG TGGTGCAATC AAAGGCCAAC 50
ACTTCGATAT CTATCAAGGG ATCGGGCCGG AAGCCGGACA CCGCGCAGGT 100
<210> 6
<211> 100
<212> DNA
<213> Escherichia coli K-12 MG1655
<220> mlta b2814
<222> 890-989
<223> mlta b2814 U00096 complement(2944103..2945200)
<313> 6: 911-930
<400> 6
TGCTGGATAA TAACGGCAAA TTTAATGGTC AGTACGAACT GCGTCTGATG 50
GTGGCGCTGG ATGTCGGTGG TGCAATCAAA GGCCAACACT TCGATATCTA 100
<210> 7

<211> 100
<212> DNA
<213> Escherichia coli K-12 MG1655
<220> b1362 b1363
<222> 207-306
<223> b1362 b1363 U00096 1421363..1421668
<313> 7: 267-286
<400> 7
CGCTGAACGG GATTATTTCA CCCTCCGGGA ACGACTGGTA ATGATGCAGG  50
CCCAACTTGA AGGTGCTCAG CAATACATAA CCGAGCAGTG TTTAAAGTAA  100
<210> 8
<211> 100
<212> DNA
<213> Escherichia coli K-12 MG1655
<220> b1362 b1363
<222> 174-273
<223> b1362 b1363 U00096 1421363..1421668
<313> 8: 227-246
<400> 8
GGATAATGCA GCCTCCCCCC GACTGGCAGA CACCGCTGAA CGGGATTATT  50
TCACCCTCCG GGAACGACTG GTAATGATGC AGGCCCAACT TGAAGGTGCT  100
<210> 9
<211> 100
<212> DNA
<213> Escherichia coli K-12 MG1655
<220> b1362 b1363
<222> 119-218
<223> b1362 b1363 U00096 1421363..1421668
<313> 9: 177-196
<400> 9
TGCACATCAA AGCAGTCTGT CAGTCAGTGC GTGAAGCCAC CACCGCCTCC  50
GGCGTGGATA ATGCAGCCTC CCCCCGACTG GCAGACACCG CTGAACGGGA  100
<210> 10
<211> 100
<212> DNA
<213> Escherichia coli K-12 MG1655
<220> mltb b2702
<222> 987-1086
<223> mltb b2702 U00096 complement(2822514..2823599)
<313> 10: 1033-1052
<400> 10
GCCGAACTTC TACACCATCA CCCGTTACAA CCACAGCACC CATTACGCAA  50
TGGCGGTCTG GCAGTTAGGA CAAGCCGTGG CGCTGGCGCG AGTACAGTAG  100
<210> 11
<211> 100
<212> DNA
<213> Escherichia coli K-12 MG1655
<220> mltb b2702
<222> 938-1037
<223> mltb b2702 U00096 complement(2822514..2823599)
<313> 11: 1001-1020
<400> 11
GCCTGCTGCG TCTGGATGTT GGCACCGGCT ACCAGTACTG GTACGGTCTG  50
CCGAACTTCT ACACCATCAC CCGTTACAAC CACAGCACCC ATTACGCAAT  100
<210> 12
<211> 100
<212> DNA
<213> Escherichia coli K-12 MG1655
<220> mltb b2702
<222> 900-999
<223> mltb b2702 U00096 complement(2822514..2823599)
<313> 12: 912-931
<400> 12
AACGCCACAG CAGCCGCTGG GCAACCATCA ACAAGCCAGC CTGCTGCGTC  50
TGGATGTTGG CACCGGCTAC CAGTACTGGT ACGGTCTGCC GAACTTCTAC  100
<210> 13
<211> 100
<212> DNA
<213> Escherichia coli K-12 MG1655
<220> mltc b2964
<222> 984-1083

<223> mltc b2964 U00096 3102448..3103530
<313> 13: 1060-1079
<400> 13
CGATGTTTAT CAGACGCTGA CGACCCGCCA TCCCTCTGCG GAATCTCGCC  50
GTTATCTTTA TAAAGTGAAT ACCGCGCAAA AATCCTACCG CCGCCGATAA  100
<210> 14
<211> 100
<212> DNA
<213> Escherichia coli K-12 MG1655
<220> mltc b2964
<222> 964-1063
<223> mltc b2964 U00096 3102448..3103530
<313> 14: 995-1014
<400> 14
ATTAACACCA TGACGCCGGG CGATGTTTAT CAGACGCTGA CGACCCGCCA  50
TCCCTCTGCG GAATCTCGCC GTTATCTTTA TAAAGTGAAT ACCGCGCAAA  100
<210> 15
<211> 100
<212> DNA
<213> Escherichia coli K-12 MG1655
<220> mltc b2964
<222> 796-895
<223> mltc b2964 U00096 3102448..3103530
<313> 15: 847-866
<400> 15
AGCAATATTG ATACCGGCAC CGCGTATCTG GCGATGCTGA ACAATGTTTA  50
TCTCGGCGGA ATTGATAACC CAACATCGCG GCGTTATGCC GTCATCACCG  100
<210> 16
<211> 100
<212> DNA
<213> Escherichia coli K-12 MG1655
<220> b1364 b1364
<222> 160-259
<223> b1364 b1364 U00096 1423202..1423483
<313> 16: 208-227
<400> 16
CTGGCTGCTG GGGTGCGTTT TATTCATAAA GCAAGGCTGT ATGAGCGAGA  50
AATTAAAGAT AGTCTATCGC CCATTACAAG AATTGTCACC GTATGCGCAC  100
<210> 17
<211> 100
<212> DNA
<213> Escherichia coli K-12 MG1655
<220> b1364 b1364
<222> 162-261
<223> b1364 b1364 U00096 1423202..1423483
<313> 17: 208-227
<400> 17
GGCTGCTGGG GTGCGTTTTA TTCATAAAGC AAGGCTGTAT GAGCGAGAAA  50
TTAAAGATAG TCTATCGCCC ATTACAAGAA TTGTCACCGT ATGCGCACAA  100
<210> 18
<211> 100
<212> DNA
<213> Escherichia coli K-12 MG1655
<220> b1364 b1364
<222> 159-258
<223> b1364 b1364 U00096 1423202..1423483
<313> 18: 185-204
<400> 18
ACTGGCTGCT GGGGTGCGTT TTATTCATAA AGCAAGGCTG TATGAGCGAG  50
AAATTAAAGA TAGTCTATCG CCCATTACAA GAATTGTCAC CGTATGCGCA  100
<210> 19
<211> 100
<212> DNA
<213> Escherichia coli K-12 MG1655
<220> b1365 b1366
<222> 165-264
<223> b1365 b1366 U00096 1423401..1423664
<313> 19: 239-258
<400> 19
TGCGGGTCAC GGTCGTGTTA TGGCGGCTGA AATGCTCAAA ATGGATTCTG  50
TTCCGGTCAT TGTTCTGTCT GGCCTGACGG ATGAGCAGAA GCAGCGATAA  100

<210> 20
<211> 100
<212> DNA
<213> Escherichia coli K-12 MG1655
<220> b1365 b1366
<222> 143-242
<223> b1365 b1366 U00096 1423401..1423664
<313> 20: 190-209
<400> 20
TTGACGAAAA GGGCGAAATT ATTGCGGGTC ACGGTCGTGT TATGGCGGCT 50
GAAATGCTCA AAATGGATTC TGTTCCGGTC ATTGTTCTGT CTGGCCTGAC 100
<210> 21
<211> 100
<212> DNA
<213> Escherichia coli K-12 MG1655
<220> b1365 b1366
<222> 116-215
<223> b1365 b1366 U00096 1423401..1423664
<313> 21: 144-163
<400> 21
AATTCGGCTG GACTAATCCG GTGCTGATTG ACGAAAAGGG CGAAATTATT 50
GCGGGTCACG GTCGTGTTAT GGCGGCTGAA ATGCTCAAAA TGGATTCTGT 100
<210> 22
<211> 100
<212> DNA
<213> Escherichia coli K-12 MG1655
<220> mlte b1194
<222> 627-726
<223> mlte b1194 U00096 1242289..1243014
<313> 22: 697-716
<400> 22
GGATGCTGAC GAGTTCCTCG AACACGTAGC GCGAAATCAC CCTGCGCCGC 50
AGGCTCCGCG CTATATCTAC AAACTTGAGC AGGCACTGGA CGCGATGTAA 100
<210> 23
<211> 100
<212> DNA
<213> Escherichia coli K-12 MG1655
<220> mlte b1194
<222> 591-690
<223> mlte b1194 U00096 1242289..1243014
<313> 23: 646-665
<400> 23
AGATCGGAAA AAGGCGATCA GCAAAATCAA CGATTTGGAT GCTGACGAGT 50
TCCTCGAACA CGTAGCGCGA AATCACCCTG CGCCGCAGGC TCCGCGCTAT 100
<210> 24
<211> 100
<212> DNA
<213> Escherichia coli K-12 MG1655
<220> mlte b1194
<222> 562-661
<223> mlte b1194 U00096 1242289..1243014
<313> 24: 606-625
<400> 24
GCAGGTGCGC TGCTACGGAC TTTCTCGTCA GATCGGAAAA AGGCGATCAG 50
CAAAATCAAC GATTTGGATG CTGACGAGTT CCTCGAACAC GTAGCGCGAA 100
<210> 25
<211> 100
<212> DNA
<213> Escherichia coli K-12 MG1655
<220> b1367 b1367
<222> 135-234
<223> b1367 b1367 U00096 1424079..1424312
<313> 25: 193-212
<400> 25
TGAATATTTA GCCACTCATC CGGTATTGAT TGAGCGCGAT GAATTTGAAG 50
CAGTGATCTT TGCACTGGAT GACCAGTATT TTCAGGAGCA GTGTGTGTAG 100
<210> 26
<211> 100
<212> DNA
<213> Escherichia coli K-12 MG1655
<220> b1367 b1367

<222> 101-200
<223> b1367 b1367 U00096 1424079..1424312
<313> 26: 152-171
<400> 26
GGGTGCCTTT GCCTTTATCT CTGACAGAGA TAAATGAATA TTTAGCCACT  50
CATCCGGTAT TGATTGAGCG CGATGAATTT GAAGCAGTGA TCTTTGCACT  100
<210> 27
<211> 100
<212> DNA
<213> Escherichia coli K-12 MG1655
<220> b1367 b1367
<222> 18-117
<223> b1367 b1367 U00096 1424079..1424312
<313> 27: 74-93
<400> 27
ACCAGAAATA GACCCGGTGC TTAAGGAGTT GTTGTACGCC TATTCGGTAA  50
TATCCCGTGC CCGACGTTAT GCTGGAATGG CTGGGGTGCC TTTGCCTTTA  100
<210> 28
<211> 100
<212> DNA
<213> Escherichia coli K-12 MG1655
<220> b2097 b2098
<222> 1026-1125
<223> b2097 b2098 U00096 complement(2175532..2176656)
<313> 28: 1095-1114
<400> 28
TCTTGGACGT AAAGCGTTCA AGAAATCGAT GGCTGACGGC GTGAAACTGA  50
TTAACGCCGT GCAGGACGTT TATCTCGATA GCAAAATTAC TATCGCCTGA  100
<210> 29
<211> 100
<212> DNA
<213> Escherichia coli K-12 MG1655
<220> b2097 b2098
<222> 1024-1123
<223> b2097 b2098 U00096 complement(2175532..2176656)
<313> 29: 1056-1075
<400> 29
ATTCTTGGAC GTAAAGCGTT CAAGAAATCG ATGGCTGACG GCGTGAAACT  50
GATTAACGCC GTGCAGGACG TTTATCTCGA TAGCAAAATT ACTATCGCCT  100
<210> 30
<211> 100
<212> DNA
<213> Escherichia coli K-12 MG1655
<220> b2097 b2098
<222> 959-1058
<223> b2097 b2098 U00096 complement(2175532..2176656)
<313> 30: 1006-1025
<400> 30
AAACTGACCT CAGCGATGCA GTGCGTACTG CGGTTATCAA CAAACGCGCA  50
GGCGGAATGG GGCTGATTCT TGGACGTAAA GCGTTCAAGA AATCGATGGC  100
<210> 31
<211> 100
<212> DNA
<213> Escherichia coli K-12 MG1655
<220> b1368 b1368
<222> 930-1029
<223> b1368 b1368 U00096 1424478..1425506
<313> 31: 984-1003
<400> 31
CGGCCATTCA GCTATGCAGG AGACAAGAAT CGCCAGCTTA CCCGTTACAG  50
CGATACCCGC TGGCATGAAG ATTCCGTGCG TAACCGCTGG TTCAGCGTGA  100
<210> 32
<211> 100
<212> DNA
<213> Escherichia coli K-12 MG1655
<220> b1368 b1368
<222> 919-1018
<223> b1368 b1368 U00096 1424478..1425506
<313> 32: 950-969
<400> 32
ATGCAGCAGG GCGGCCATTC AGCTATGCAG GAGACAAGAA TCGCCAGCTT  50

ACCCGTTACA GCGATACCCG CTGGCATGAA GATTCCGTGC GTAACCGCTG 100
<210> 33
<211> 100
<212> DNA
<213> Escherichia coli K-12 MG1655
<220> b1368 b1368
<222> 810-909
<223> b1368 b1368 U00096 1424478..1425506
<313> 33: 864-883
<400> 33
AACCGGAATG TCGGCAAAAC AAACGGCTTT TGCTATGCGC ATGTTGCCTG 50
CACAAATGAC GGATATTGTT GTTGGGTTGT CCACTGGTCA GTCGCCATTT 100
<210> 34
<211> 100
<212> DNA
<213> Escherichia coli K-12 MG1655
<220> b1369 b1370
<222> 57-156
<223> b1369 b1370 U00096 1425482..1425637
<313> 34: 132-151
<400> 34
GTTCAGCGCG TATGCGATGG CGGGTATGGC TTACAGCCGT GTGTCGACTT 50
TCTCCGGGGA TTATCTCCGC GTAACTGACA ACAAGGGAAG GTGCGAATAA 100
<210> 35
<211> 100
<212> DNA
<213> Escherichia coli K-12 MG1655
<220> b2099 b2099
<222> 904-1003
<223> b2099 b2099 U00096 2178115..2179119
<313> 35: 934-953
<400> 35
GATCCTGCGT TAAAGGCGGA ACTGGATGCG GTAAATCAGC TTGATTTCAA 50
CCGCTATGCC ACAGCGCTGG CGAAATATCG TCAACAACGG GAGGCGGTAT 100
<210> 36
<211> 100
<212> DNA
<213> Escherichia coli K-12 MG1655
<220> b2099 b2099
<222> 766-865
<223> b2099 b2099 U00096 2178115..2179119
<313> 36: 819-838
<400> 36
GCCATTGCGC TGGTTGAACT GGCACAAACC GACCCGAATC GCTGCGCCGT 50
CCTGTGCGCT AACCTTGGCG GCGACACAGA CACCATCGGT GCTATGGCGA 100
<210> 37
<211> 100
<212> DNA
<213> Escherichia coli K-12 MG1655
<220> b2099 b2099
<222> 708-807
<223> b2099 b2099 U00096 2178115..2179119
<313> 37: 755-774
<400> 37
CGAACAGCTT TACCAGGTCG TTGGCGCAGG TACCAGCACT ATTGAGTCCG 50
TTCCGTGCGC CATTGCGCTG GTTGAACTGG CACAAACCGA CCCGAATCGC 100
<210> 38
<211> 100
<212> DNA
<213> Escherichia coli K-12 MG1655
<220> yojh b2211
<222> 1548-1647
<223> yojh b2211 U00096 complement(2303128..2304774)
<313> 38: 1613-1632
<400> 38
GGGGTTGAAC TACGACAAGC CGCAGGCAGC AGATAGTACG CCGAAACCGC 50
AGTTGAAACC GCAACCCGTT CAAAAAGAAG TGGCGGATAT TGCGTTGTAA 100
<210> 39
<211> 100
<212> DNA
<213> Escherichia coli K-12 MG1655

<220> yojh b2211
<222> 1520-1619
<223> yojh b2211 U00096 complement(2303128..2304774)
<313> 39: 1587-1606
<400> 39
GCGAGTTGCA GTACACCAGC GAAGTGCTGG GGTTGAACTA CGACAAGCCG  50
CAGGCAGCAG ATAGTACGCC GAAACCGCAG TTGAAACCGC AACCCGTTCA  100
<210> 40
<211> 100
<212> DNA
<213> Escherichia coli K-12 MG1655
<220> yojh b2211
<222> 1482-1581
<223> yojh b2211 U00096 complement(2303128..2304774)
<313> 40: 1517-1536
<400> 40
TGGACGCAAG CTGAACGGTG ATGTAGCGGC AACAGAACGC GAGTTGCAGT  50
ACACCAGCGA AGTGCTGGGG TTGAACTACG ACAAGCCGCA GGCAGCAGAT  100
<210> 41
<211> 100
<212> DNA
<213> Escherichia coli K-12 MG1655
<220> yoji b2212
<222> 1545-1644
<223> yoji b2212 U00096 complement(2304992..2306635)
<313> 41: 1591-1610
<400> 41
CGCCGACCGC CTGCTGGAAA TGCGCAATGG GCAACTTAGC GAGCTGACGG  50
GCGAAGAGCG CGATGCCGCT TCGCGTGATG CCGTTGCCCG GACGGCATAA  100
<210> 42
<211> 100
<212> DNA
<213> Escherichia coli K-12 MG1655
<220> yoji b2212
<222> 1490-1589
<223> yoji b2212 U00096 complement(2304992..2306635)
<313> 42: 1534-1553
<400> 42
AGGAGATGGG TAAAACTATT TTCGCTATCA GTCATGATGA TCATTACTTT  50
ATCCACGCCG ACCGCCTGCT GGAAATGCGC AATGGGCAAC TTAGCGAGCT  100
<210> 43
<211> 100
<212> DNA
<213> Escherichia coli K-12 MG1655
<220> yoji b2212
<222> 1437-1536
<223> yoji b2212 U00096 complement(2304992..2306635)
<313> 43: 1478-1497
<400> 43
TCAGGATCCA CACTTCCGTC GTGAGTTTTA TCAGGTGTTG CTGCCGCTGA  50
TGCAGGAGAT GGGTAAAACT ATTTTCGCTA TCAGTCATGA TGATCATTAC  100
<210> 44
<211> 100
<212> DNA
<213> Escherichia coli K-12 MG1655
<220> yojl b2215
<222> 957-1056
<223> yojl b2215 U00096 complement(2308499..2309554)
<313> 44: 1029-1048
<400> 44
GGAAGGGCTG GCGGTTTATA TGATCACCAA AGAAGGCGAT AGCTTTAAAA  50
CCTGGATGTC ACCACAGTTT AAAAGCTTCC TTGTCAGCGA AAAAAATTAA  100
<210> 45
<211> 100
<212> DNA
<213> Escherichia coli K-12 MG1655
<220> yojl b2215
<222> 946-1045
<223> yojl b2215 U00096 complement(2308499..2309554)
<313> 45: 970-989
<400> 45

GTTGTTCGCC GGGAAGGGCT GGCGGTTTAT ATGATCACCA AAGAAGGCGA 50
TAGCTTTAAA ACCTGGATGT CACCACAGTT TAAAAGCTTC CTTGTCAGCG 100
<210> 46
<211> 100
<212> DNA
<213> Escherichia coli K-12 MG1655
<220> yojl b2215
<222> 898-997
<223> yojl b2215 U00096 complement(2308499..2309554)
<313> 46: 924-943
<400> 46
GCCTGGGATA CTGGCTTGAT GGTACTCGGG CCGGAGAAAG CTAAAGAGGT 50
TGTTCGCCGG GAAGGGCTGG CGGTTTATAT GATCACCAAA GAAGGCGATA 100
<210> 47
<211> 100
<212> DNA
<213> Escherichia coli K-12 MG1655
<220> goag b1303
<222> 1149-1248
<223> goag b1303 U00096 1363574..1364839
<313> 47: 1187-1206
<400> 47
GCTCCTGCTG ACCTGTGGCG CATACGGCAA CGTGATTCGC TTCCTGTATC 50
CGCTGACCAT CCCGGATGCG CAATTCGATG CGGCAATGAA AATTTTGCAG 100
<210> 48
<211> 100
<212> DNA
<213> Escherichia coli K-12 MG1655
<220> goag b1303
<222> 1048-1147
<223> goag b1303 U00096 1363574..1364839
<313> 48: 1079-1098
<400> 48
CTGGGGTCGA TGATTGCGGT AGAGTTTAAC GATCCGCAAA CGGGCGAGCC 50
GTCAGCGGCG ATTGCACAGA AAATCCAGCA ACGCGCGCTG GCGCAGGGGC 100
<210> 49
<211> 100
<212> DNA
<213> Escherichia coli K-12 MG1655
<220> goag b1303
<222> 970-1069
<223> goag b1303 U00096 1363574..1364839
<313> 49: 1019-1038
<400> 49
AATCAACTGG GCCAGCGTCT CAAAAACACG TTGATTGATG CCAAAGAAAG 50
CGTTCCGGCC ATTGCTGCGG TACGCGGCCT GGGGTCGATG ATTGCGGTAG 100
<210> 50
<211> 100
<212> DNA
<213> Escherichia coli K-12 MG1655
<220> yojn b2217
<222> 2541-2640
<223> yojn b2217 U00096 2311508..2314180
<313> 50: 2586-2605
<400> 50
ATTTGCCATG CTAAATCTGG TACCCGGCAA GCAGTTATGT GAAACGCTGG 50
AACATCTGAT TCGTGAGAAG GATGTTCCAG GAATAGAAAA ATACATCAGC 100
<210> 51
<211> 100
<212> DNA
<213> Escherichia coli K-12 MG1655
<220> yojn b2217
<222> 2498-2597
<223> yojn b2217 U00096 2311508..2314180
<313> 51: 2538-2557
<400> 51
ACTTTGCTGC GTTAGCCCAA ACGGCTCATC GTCTTAAAGG CGTATTTGCC 50
ATGCTAAATC TGGTACCCGG CAAGCAGTTA TGTGAAACGC TGGAACATCT 100
<210> 52
<211> 100
<212> DNA

<213> Escherichia coli K-12 MG1655
<220> yojn b2217
<222> 2437-2536
<223> yojn b2217 U00096 2311508..2314180
<313> 52: 2458-2477
<400> 52
CTCTTTGTAG ACACAGTACC GGATGATGTT AAGAGGCTGT ATACTGAAGC 50
AGCAACCAGT GACTTTGCTG CGTTAGCCCA AACGGCTCAT CGTCTTAAAG 100
<210> 53
<211> 100
<212> DNA
<213> Escherichia coli K-12 MG1655
<220> b1371 b1371
<222> 363-462
<223> b1371 b1371 U00096 1426547..1427008
<313> 53: 412-431
<400> 53
CCCGACCGAA TCCGTGGCCA TTGATATTGC TTATGAAGGC CCCGGCAGTG 50
GCGACTGGCG CACTGACGGT TTCATCGTGG GTGTCGGTTA TAAGTTCTGA 100
<210> 54
<211> 100
<212> DNA
<213> Escherichia coli K-12 MG1655
<220> b1371 b1371
<222> 315-414
<223> b1371 b1371 U00096 1426547..1427008
<313> 54: 351-370
<400> 54
TCGCCACAGC AACACGTCTC TGGCGTGGGG AGCTGGCGTG CAGTTTAACC 50
CGACCGAATC CGTGGCCATT GATATTGCTT ATGAAGGCCC CGGCAGTGGC 100
<210> 55
<211> 100
<212> DNA
<213> Escherichia coli K-12 MG1655
<220> b1371 b1371
<222> 243-342
<223> b1371 b1371 U00096 1426547..1427008
<313> 55: 278-297
<400> 55
TCTCATATCA GGAACTTGTT CGCACCTTCC CAAGGGGAAA ACGCACGACG 50
TGCTGACCGG AAGTGATGAC GGTCGCCACA GCAACACGTC TCTGGCGTGG 100
<210> 56
<211> 100
<212> DNA
<213> Escherichia coli K-12 MG1655
<220> b1372 b1373
<222> 3163-3262
<223> b1372 b1373 U00096 1427067..1430435
<313> 56: 3197-3216
<400> 56
GCAACATCAT CTGCTGGCGC ACATACCCAC TCACTGTCCG GCACTGCTGC 50
AAGCGCAGGT GCACACGCGC ATACTGTCGG TATTGGTGCT CATACGCACT 100
<210> 57
<211> 100
<212> DNA
<213> Escherichia coli K-12 MG1655
<220> b1372 b1373
<222> 3015-3114
<223> b1372 b1373 U00096 1427067..1430435
<313> 57: 3069-3088
<400> 57
CACCGGGGCA CATACACACA GTGTGAGCGG CTCTACAAAC TCGGCTGGAG 50
CACACACACA CTCACTAGCC AACGTGAACA CGGCTAGTGC TAACTCCGGT 100
<210> 58
<211> 100
<212> DNA
<213> Escherichia coli K-12 MG1655
<220> b1372 b1373
<222> 3006-3105
<223> b1372 b1373 U00096 1427067..1430435
<313> 58: 3029-3048

<400> 58
CACGAATAAC ACCGGGGCAC ATACACACAG TGTGAGCGGC TCTACAAACT 50
CGGCTGGAGC ACACACACAC TCACTAGCCA ACGTGAACAC GGCTAGTGCT 100
<210> 59
<211> 100
<212> DNA
<213> Escherichia coli K-12 MG1655
<220> b1374 b1375
<222> 449-548
<223> b1374 b1375 U00096 complement(1431108..1431698)
<313> 59: 488-507
<400> 59
CTGTGTTAAA TGAAGAACAG AAACAGGTGG TATTCGAACG AATTAAGTCA 50
GGTGTAAGTA TAAGTGCCAT TGCCCGGGAA TTCAAAACCT CGCGGCAAAC 100
<210> 60
<211> 100
<212> DNA
<213> Escherichia coli K-12 MG1655
<220> b1374 b1375
<222> 429-528
<223> b1374 b1375 U00096 complement(1431108..1431698)
<313> 60: 474-493
<400> 60
GAAACGTTTT GGTCGACCTC CTGTGTTAAA TGAAGAACAG AAACAGGTGG 50
TATTCGAACG AATTAAGTCA GGTGTAAGTA TAAGTGCCAT TGCCCGGGAA 100
<210> 61
<211> 100
<212> DNA
<213> Escherichia coli K-12 MG1655
<220> b1374 b1375
<222> 445-544
<223> b1374 b1375 U00096 complement(1431108..1431698)
<313> 61: 471-490
<400> 61
CCTCCTGTGT TAAATGAAGA ACAGAAACAG GTGGTATTCG AACGAATTAA 50
GTCAGGTGTA AGTATAAGTG CCATTGCCCG GGAATTCAAA ACCTCGCGGC 100
<210> 62
<211> 100
<212> DNA
<213> Escherichia coli K-12 MG1655
<220> b1377 b1378
<222> 1035-1134
<223> b1377 b1378 U00096 complement(1433784..1434917)
<313> 62: 1075-1094
<400> 62
CTATAAAATC AACCTGTTGG ATGAAGATGA CAGCTTCTAC GCTGCCAATG 50
GCATCTCTAC CGATGATATT GTCGCTTTAG GTCTGGTTTA TCAGTTCTAA 100
<210> 63
<211> 100
<212> DNA
<213> Escherichia coli K-12 MG1655
<220> b1377 b1378
<222> 903-1002
<223> b1377 b1378 U00096 complement(1433784..1434917)
<313> 63: 950-969
<400> 63
GTCTAAAGGC CGTGACCTGC ACGCTGCGGG TGGTGCAGAC AACCCGGCAG 50
GTGTTGATGA TAAAGATCTG GTTAAATACG CCGATATTGG CGCGACTTAC 100
<210> 64
<211> 100
<212> DNA
<213> Escherichia coli K-12 MG1655
<220> b1377 b1378
<222> 850-949
<223> b1377 b1378 U00096 complement(1433784..1434917)
<313> 64: 899-918
<400> 64
ACTGCACAGT ACCAGTTTGA TTTTGGTCTG CGTCCGGCAG TCTCTTTCCT 50
GATGTCTAAA GGCCGTGACC TGCACGCTGC GGGTGGTGCA GACAACCCGG 100
<210> 65
<211> 100

<212> DNA
<213> Escherichia coli K-12 MG1655
<220> flu flu
<222> 3177-3276
<223> flu flu U00096 2069405..2072680
<313> 65: 3245-3264
<400> 65
AAATATCACC CTGGGCGTTC AGGCCGGTTA TGCCCACAGC GTCAGCGGCA 50
GCAGCGCTGA AGGGTATAAC GGTCAGGCCA CACTGAATGT GACCTTCTGA 100
<210> 66
<211> 100
<212> DNA
<213> Escherichia coli K-12 MG1655
<220> flu flu
<222> 3075-3174
<223> flu flu U00096 2069405..2072680
<313> 66: 3125-3144
<400> 66
GCGTGTGGGG ACTTCCACTG CAGGCAGCGG GATGACGTTC TCTCCCTCAC 50
AGAATGGCAC ATCACTGGAC CTGCAGGCCG GACTGGAAGC CCGTGTCCGG 100
<210> 67
<211> 100
<212> DNA
<213> Escherichia coli K-12 MG1655
<220> flu flu
<222> 2937-3036
<223> flu flu U00096 2069405..2072680
<313> 67: 2999-3018
<400> 67
CAACGATATG ACCTTTGGCG AAGGCACCTC ATCCCGTGCC CCCCTGCGTG 50
ACAGTGCAAA ACACAGTGTG AGTGAATTAC CGGTGAACTG GTGGGTACAG 100
<210> 68
<211> 100
<212> DNA
<213> Escherichia coli K-12 MG1655
<220> ung b2581
<222> 591-690
<223> ung b2581 U00096 2714774..2715463
<313> 68: 666-685
<400> 68
CTTTGGCTGC AACCATTTTG TGCTGGCAAA TCAGTGGCTG GAACAACGTG 50
GCGAGACGCC GATTGACTGG ATGCCAGTAT TACCGGCAGA GAGTGAGTAA 100
<210> 69
<211> 100
<212> DNA
<213> Escherichia coli K-12 MG1655
<220> ung b2581
<222> 559-658
<223> ung b2581 U00096 2714774..2715463
<313> 69: 596-615
<400> 69
CATCCGTCGC CGCTTTCGGC GCATCGTGGA TTCTTTGGCT GCAACCATTT 50
TGTGCTGGCA AATCAGTGGC TGGAACAACG TGGCGAGACG CCGATTGACT 100
<210> 70
<211> 100
<212> DNA
<213> Escherichia coli K-12 MG1655
<220> ung b2581
<222> 487-586
<223> ung b2581 U00096 2714774..2715463
<313> 70: 550-569
<400> 70
TTGTGGGGAT CGCATGCGCA AAAGAAAGGG GCGATTATAG ATAAGCAACG 50
CCATCATGTA CTGAAAGCAC CGCATCCGTC GCCGCTTTCG GCGCATCGTG 100
<210> 71
<211> 100
<212> DNA
<213> Escherichia coli K-12 MG1655
<220> uspa b3496
<222> 336-435
<223> uspa b3496 U00096 3637741..3638175

<313> 71: 406-425
<400> 71
CCAGGACTTC TGGAGCAAAC TGATGTCTTC CGCACGTCAG CTGATCAACA 50
CCGTTCACGT TGATATGCTG ATTGTTCCGC TGCGCGACGA AGAAGAATAA 100
<210> 72
<211> 100
<212> DNA
<213> Escherichia coli K-12 MG1655
<220> uspa b3496
<222> 317-416
<223> uspa b3496 U00096 3637741..3638175
<313> 72: 345-364
<400> 72
TGGTGGTTTG TGGTCACCAC CAGGACTTCT GGAGCAAACT GATGTCTTCC 50
GCACGTCAGC TGATCAACAC CGTTCACGTT GATATGCTGA TTGTTCCGCT 100
<210> 73
<211> 100
<212> DNA
<213> Escherichia coli K-12 MG1655
<220> uspa b3496
<222> 241-340
<223> uspa b3496 U00096 3637741..3638175
<313> 73: 285-304
<400> 73
ATCACTGAAA CCCTGAGCGG CAGCGGCGAC CTGGGCCAGG TTCTGGTCGA 50
TGCAATCAAG AAATACGATA TGGATTTGGT GGTTTGTGGT CACCACCAGG 100
<210> 74
<211> 100
<212> DNA
<213> Escherichia coli K-12 MG1655
<220> aqpz b0876
<222> 543-642
<223> aqpz b0876 U00096 complement(914575..915270)
<313> 74: 593-612
<400> 74
TAACACTTCT GTTAACCCGG CGCGCAGCAC CGCGGTTGCT ATCTTCCAGG 50
GCGGCTGGGC ATTAGAACAA CTGTGGTTCT TCTGGGTGGT GCCAATTGTC 100
<210> 75
<211> 100
<212> DNA
<213> Escherichia coli K-12 MG1655
<220> aqpz b0876
<222> 387-486
<223> aqpz b0876 U00096 complement(914575..915270)
<313> 75: 429-448
<400> 75
TTCCATGCTT TCCGCGCTGG TAGTTGAACT GGTATTGAGT GCAGGTTTCC 50
TGTTGGTGAT CCACGGCGCA ACCGACAAAT TCGCGCCGGC AGGTTTTGCG 100
<210> 76
<211> 100
<212> DNA
<213> Escherichia coli K-12 MG1655
<220> aqpz b0876
<222> 353-452
<223> aqpz b0876 U00096 complement(914575..915270)
<313> 76: 395-414
<400> 76
CTAACGGTTA TGGCGAGCAT TCACCAGGCG GTTATTCCAT GCTTTCCGCG 50
CTGGTAGTTG AACTGGTATT GAGTGCAGGT TTCCTGTTGG TGATCCACGG 100
<210> 77
<211> 100
<212> DNA
<213> Escherichia coli K-12 MG1655
<220> csga b1043
<222> 346-445
<223> csga b1043 U00096 1103670..1104125
<313> 77: 392-411
<400> 77
AAACAGTTCG GTGGTGGCAA CGGTGCTGCA GTTGACCAGA CTGCATCTAA 50
CTCCTCCGTC AACGTGACTC AGGTTGGCTT TGGTAACAAC GCGACCGCTC 100
<210> 78

<211> 100
<212> DNA
<213> Escherichia coli K-12 MG1655
<220> csga b1043
<222> 286-385
<223> csga b1043 U00096 1103670..1104125
<313> 78: 351-370
<400> 78
GGCTTCGGTA ACAGCGCTAC TCTTGATCAG TGGAACGGCA AAAATTCTGA 50
AATGACGGTT AAACAGTTCG GTGGTGGCAA CGGTGCTGCA GTTGACCAGA 100
<210> 79
<211> 100
<212> DNA
<213> Escherichia coli K-12 MG1655
<220> csga b1043
<222> 214-313
<223> csga b1043 U00096 1103670..1104125
<313> 79: 267-286
<400> 79
CAGCATGGCG GCGGTAATGG TGCAGATGTT GGTCAGGGCT CAGATGACAG 50
CTCAATCGAT CTGACCCAAC GTGGCTTCGG TAACAGCGCT ACTCTTGATC 100
<210> 80
<211> 100
<212> DNA
<213> Escherichia coli K-12 MG1655
<220> csgb b1042
<222> 266-365
<223> csgb b1042 U00096 1103174..1103629
<313> 80: 304-323
<400> 80
ACCTTGCATA TATTGATCAG GCGGGCAGTG CCAACGATGC CAGTATTTCG 50
CAAGGTGCTT ATGGTAATAC TGCGATGATT ATCCAGAAAG GTTCTGGTAA 100
<210> 81
<211> 100
<212> DNA
<213> Escherichia coli K-12 MG1655
<220> csgb b1042
<222> 208-307
<223> csgb b1042 U00096 1103174..1103629
<313> 81: 245-264
<400> 81
GTTGTTGCGC AAGAAGGTAG TAGCAACCGG GCAAAGATTG ACCAGACAGG 50
AGATTATAAC CTTGCATATA TTGATCAGGC GGGCAGTGCC AACGATGCCA 100
<210> 82
<211> 100
<212> DNA
<213> Escherichia coli K-12 MG1655
<220> csgb b1042
<222> 123-222
<223> csgb b1042 U00096 1103174..1103629
<313> 82: 179-198
<400> 82
TTCATTTAAT CAGGCAGCCA TAATTGGTCA AGCTGGGACT AATAATAGTG 50
CTCAGTTACG GCAGGGAGGC TCAAAACTTT TGGCGGTTGT TGCGCAAGAA 100
<210> 83
<211> 100
<212> DNA
<213> Escherichia coli K-12 MG1655
<220> csgc csgC
<222> 234-333
<223> csgc csgC U00096 1104184..1104516
<313> 83: 309-328
<400> 83
TATTTCCCCG GACGATCGGG TGAAAATAGT TGTTACTGTT TCTGATGGAC 50
AGTCACTTCA TTTATCACAA CAATGGCCGC CCTCTTCAGA AAAGTCTTAA 100
<210> 84
<211> 100
<212> DNA
<213> Escherichia coli K-12 MG1655
<220> csgc csgC
<222> 177-276

<223> csgc csgC U00096 1104184..1104516
<313> 84: 192-211
<400> 84
AAAGACCCTT TCATTGCCTG CTAATCAACC CATTGCTTTG ACGAAGTTGA 50
GTTTAAATAT TTCCCCGGAC GATCGGGTGA AAATAGTTGT TACTGTTTCT 100
<210> 85
<211> 100
<212> DNA
<213> Escherichia coli K-12 MG1655
<220> csgc csgC
<222> 107-206
<223> csgc csgC U00096 1104184..1104516
<313> 85: 171-190
<400> 85
CTTGTCTGTG CAGAGTACAA ATATTGTCCC TGCGCGAAGG CAGTTCAGGG 50
CAAAGTCAGA CGAAGCAAGA AAAGACCCTT TCATTGCCTG CTAATCAACC 100
<210> 86
<211> 100
<212> DNA
<213> Escherichia coli K-12 MG1655
<220> csgd b1041
<222> 437-536
<223> csgd b1041 U00096 complement(1101769..1102419)
<313> 86: 476-495
<400> 86
ATCGTTATAA CAGCACGGAA TCAGCCCTCC TTACTCATCG GGAAAAAGAG 50
ATCCTGAATA AGCTGCGTAT CGGCGCGTCT AATAACGAGA TCGCTCGTTC 100
<210> 87
<211> 100
<212> DNA
<213> Escherichia coli K-12 MG1655
<220> csgd b1041
<222> 360-459
<223> csgd b1041 U00096 complement(1101769..1102419)
<313> 87: 404-423
<400> 87
GTTGCAAGGC GTCCTGCGCG GCGAATGCTA CTTTACGCAA AAGCTTGCCA 50
GCTACCTGAT TACGCATTCA GGTAACTATC GTTATAACAG CACGGAATCA 100
<210> 88
<211> 100
<212> DNA
<213> Escherichia coli K-12 MG1655
<220> csgd b1041
<222> 237-336
<223> csgd b1041 U00096 complement(1101769..1102419)
<313> 88: 294-313
<400> 88
CAACAATATC AAAATATTGT TGCTAAATAC GCCTGAAGAT TACCCGTACC 50
GCGACATTGA AAACTGGCCT CATATCAACG GCGTTTTTTA TTCCATGGAG 100
<210> 89
<211> 100
<212> DNA
<213> Escherichia coli K-12 MG1655
<220> csge b1040
<222> 291-390
<223> csge b1040 U00096 complement(1101375..1101764)
<313> 89: 350-369
<400> 89
TGTCGTCTTT GCACTGATTC AAACTGAAGA AGCACTAAAT CGTCGCCAGA 50
TAAATCAGGC GTTATTAAGT ACGGGCGATT TGGCGCATGA TGAATTCTAA 100
<210> 90
<211> 100
<212> DNA
<213> Escherichia coli K-12 MG1655
<220> csge b1040
<222> 257-356
<223> csge b1040 U00096 complement(1101375..1101764)
<313> 90: 313-332
<400> 90
TTTTATTTCC GTTGAAAAGA GACTTCGAGA AAACTGTCGT CTTTGCACTG 50
ATTCAAACTG AAGAAGCACT AAATCGTCGC CAGATAAATC AGGCGTTATT 100

<210> 91
<211> 100
<212> DNA
<213> Escherichia coli K-12 MG1655
<220> csge b1040
<222> 157-256
<223> csge b1040 U00096 complement(1101375..1101764)
<313> 91: 213-232
<400> 91
AGTGACTATA CGGGTAACTT AACGATTAAT GAAAGGCCCA GTGCACGATG  50
GGGAAGCTGG ATCACTATAA CGGTCAATCA GGACGTTATT TTCCAGACTT  100
<210> 92
<211> 100
<212> DNA
<213> Escherichia coli K-12 MG1655
<220> csgf b1039
<222> 318-417
<223> csgf b1039 U00096 complement(1100934..1101350)
<313> 92: 363-382
<400> 92
CCGCGATGGT CAATTGCAGT TGAACGTGAC AGATCGTAAA ACCGGACAAA  50
CCTCGACCAT CCAGGTTTCG GGTTTACAAA ATAACTCAAC CGATTTTTAA  100
<210> 93
<211> 100
<212> DNA
<213> Escherichia coli K-12 MG1655
<220> csgf b1039
<222> 207-306
<223> csgf b1039 U00096 complement(1100934..1101350)
<313> 93: 224-243
<400> 93
TAACTTTACT CAGGCCATCC AGTCACAAAT TTTAGGTGGG CTACTGTCGA  50
ATATTAATAC CGGTAAACCG GGCCGCATGG TGACCAACGA TTATATTGTC  100
<210> 94
<211> 100
<212> DNA
<213> Escherichia coli K-12 MG1655
<220> csgf b1039
<222> 44-143
<223> csgf b1039 U00096 complement(1100934..1101350)
<313> 94: 70-89
<400> 94
CATTAAGTTG GGCTGGAACC ATGACTTTCC AGTTCCGTAA TCCAAACTTT  50
GGTGGTAACC CAAATAATGG CGCTTTTTTA TTAAATAGCG CTCAGGCCCA  100
<210> 95
<211> 100
<212> DNA
<213> Escherichia coli K-12 MG1655
<220> csgg b1038
<222> 735-834
<223> csgg b1038 U00096 complement(1100074..1100907)
<313> 95: 795-814
<400> 95
TGGTATCGAC CGTGGTCTGT GGGATTTGCA AAATAAAGCA GAACGGCAGA  50
ATGACATTCT GGTGAAATAC CGCCATATGT CGGTTCCACC GGAATCCTGA  100
<210> 96
<211> 100
<212> DNA
<213> Escherichia coli K-12 MG1655
<220> csgg b1038
<222> 631-730
<223> csgg b1038 U00096 complement(1100074..1100907)
<313> 96: 659-678
<400> 96
TACCAGCGCT TGCTTGAAGG GGAAGTGGGT TACACCTCGA ACGAACCTGT  50
TATGCTGTGC CTGATGTCGG CTATCGAAAC AGGGGTCATT TTCCTGATTA  100
<210> 97
<211> 100
<212> DNA
<213> Escherichia coli K-12 MG1655
<220> csgg b1038

<222> 561-660
<223> csgg b1038 U00096 complement(1100074..1100907)
<313> 97: 609-628
<400> 97
TTCTTCGGTG AACACCAGTA AGACGATACT TTCCTATGAA GTTCAGGCCG  50
GGGTTTTCCG CTTTATTGAC TACCAGCGCT TGCTTGAAGG GGAAGTGGGT  100
<210> 98
<211> 100
<212> DNA
<213> Escherichia coli K-12 MG1655
<220> fmt b3289
<222> 849-948
<223> fmt b3289 U00096 3431851..3432798
<313> 98: 920-939
<400> 98
CCTGCTCTCG TTACAACCTG CGGGTAAGAA AGCGATGAGC GCGCAAGACC  50
TCCTGAACTC TCGTCGGGAA TGGTTTGTTC CGGGCAACCG TCTGGTCTGA  100
<210> 99
<211> 100
<212> DNA
<213> Escherichia coli K-12 MG1655
<220> fmt b3289
<222> 796-895
<223> fmt b3289 U00096 3431851..3432798
<313> 99: 851-870
<400> 99
CTTGAAGCCA ACAAACAAGG CATTCAGGTT GCGACTGGTG ATGGCATCCT  50
GAACCTGCTC TCGTTACAAC CTGCGGGTAA GAAAGCGATG AGCGCGCAAG  100
<210> 100
<211> 100
<212> DNA
<213> Escherichia coli K-12 MG1655
<220> fmt b3289
<222> 765-864
<223> fmt b3289 U00096 3431851..3432798
<313> 100: 796-815
<400> 100
TACGGCAACC AACGCTGCAC CAGGAACGAT CCTTGAAGCC AACAAACAAG  50
GCATTCAGGT TGCGACTGGT GATGGCATCC TGAACCTGCT CTCGTTACAA  100
<210> 101
<211> 100
<212> DNA
<213> Escherichia coli K-12 MG1655
<220> mtga b3209
<222> 587-686
<223> mtga b3209 U00096 complement(3346722..3347450)
<313> 101: 621-640
<400> 101
TACTGGCAGC TGTATTACCT AATCCACTTC GTTTCAAAGT CTCCTCGCCA  50
TCGGGCTACG TGCGTAGCCG TCAGGCGTGG ATTTTACGGC AGATGTATCA  100
<210> 102
<211> 100
<212> DNA
<213> Escherichia coli K-12 MG1655
<220> mtga b3209
<222> 517-616
<223> mtga b3209 U00096 complement(3346722..3347450)
<313> 102: 554-573
<400> 102
GGCGTCGAAG CTGCGGCACA ACGTTATTTC CACAAACCCG CGAGCAAACT  50
TACCCGGTCG GAAGCTGCAT TACTGGCAGC TGTATTACCT AATCCACTTC  100
<210> 103
<211> 100
<212> DNA
<213> Escherichia coli K-12 MG1655
<220> mtga b3209
<222> 433-532
<223> mtga b3209 U00096 complement(3346722..3347450)
<313> 103: 463-482
<400> 103
ACGCTGGGGA TAGAAACGGT CTGGAGCAAA AAGCGTATCC TGACGGTTTA  50

CCTGAATATC GCCGAATTTG GCGACGGTGT GTTTGGCGTC GAAGCTGCGG 100
<210> 104
<211> 100
<212> DNA
<213> Escherichia coli K-12 MG1655
<220> b1391 b1391
<222> 405-504
<223> b1391 b1391 U00096 1453943..1454446
<313> 104: 475-494
<400> 104
CCATACCACA CTTATCAGTG AATTTGGTTC CACGGCCTGC AAAGCATTGT 50
ACCGCTGCGA TAGTTGCCGC GAACCTTTCG ATTATTTCAA ATGTATTTGA 100
<210> 105
<211> 100
<212> DNA
<213> Escherichia coli K-12 MG1655
<220> b1391 b1391
<222> 380-479
<223> b1391 b1391 U00096 1453943..1454446
<313> 105: 435-454
<400> 105
TACGTTGCCC GCGCTGCGCC AGCGTCCATA CCACACTTAT CAGTGAATTT 50
GGTTCCACGG CCTGCAAAGC ATTGTACCGC TGCGATAGTT GCCGCGAACC 100
<210> 106
<211> 100
<212> DNA
<213> Escherichia coli K-12 MG1655
<220> b1391 b1391
<222> 329-428
<223> b1391 b1391 U00096 1453943..1454446
<313> 106: 371-390
<400> 106
GCATTAGCCC GCCCGCCGGA CACAGTTGCC ATGCCCATTT GCCGCCAGAA 50
GTACGTTGCC CGCGCTGCGC CAGCGTCCAT ACCACACTTA TCAGTGAATT 100
<210> 107
<211> 100
<212> DNA
<213> Escherichia coli K-12 MG1655
<220> b1392 b1393
<222> 972-1071
<223> b1392 b1393 U00096 1454454..1455524
<313> 107: 1035-1054
<400> 107
GGAACCGGAT GAACTGGCCG CAGGTTATGT GTTGAGTTGC CAGGCACTGC 50
CGCTGACCAG CGATGTGGTG GTTGACTTTG ACGCGAAGGG GATGGCATGA 100
<210> 108
<211> 100
<212> DNA
<213> Escherichia coli K-12 MG1655
<220> b1392 b1393
<222> 953-1052
<223> b1392 b1393 U00096 1454454..1455524
<313> 108: 1002-1021
<400> 108
TGGAAACCAA TTACAGTCTG GAACCGGATG AACTGGCCGC AGGTTATGTG 50
TTGAGTTGCC AGGCACTGCC GCTGACCAGC GATGTGGTGG TTGACTTTGA 100
<210> 109
<211> 100
<212> DNA
<213> Escherichia coli K-12 MG1655
<220> b1392 b1393
<222> 913-1012
<223> b1392 b1393 U00096 1454454..1455524
<313> 109: 964-983
<400> 109
GCGACCTGCA AATGCAAAGT GCTGCGTGGC AAAGTGGCGA TGGAAACCAA 50
TTACAGTCTG GAACCGGATG AACTGGCCGC AGGTTATGTG TTGAGTTGCC 100
<210> 110
<211> 100
<212> DNA
<213> Escherichia coli K-12 MG1655

<220> b0663 b0664
<222> 215-314
<223> b0663 b0664 U00096 695581..695916
<313> 110: 234-253
<400> 110
ATGCCGGAAT CAGAATCCGG TGCCTTACCG CTTGGCGATA CCCCAACAAA 50
TTGGTTTTGA ATTTGCCGAA CATATTCGAT ACATTCAGAA TTTGGTGGCT 100
<210> 111
<211> 100
<212> DNA
<213> Escherichia coli K-12 MG1655
<220> b0663 b0664
<222> 125-224
<223> b0663 b0664 U00096 695581..695916
<313> 111: 185-204
<400> 111
CCTTACCGCT TGGCGATACC CCAACTGGGT GCACTTACAA GGTAAGCGTC 50
TTGAATAAAT TGGCTGGGGT ACGAGGATTC GAACCTCGGA ATGCCGGAAT 100
<210> 112
<211> 100
<212> DNA
<213> Escherichia coli K-12 MG1655
<220> b0663 b0664
<222> 76-175
<223> b0663 b0664 U00096 695581..695916
<313> 112: 128-147
<400> 112
CTGGGGTACG AGGATTCGAA CCTCGGAATG CCGGAATCAG AATCCGGTGC 50
CTTACCGCTT GGCGATACCC CAACTGGGTG CACTTACAAG GTAAGCGTCT 100
<210> 113
<211> 100
<212> DNA
<213> Escherichia coli K-12 MG1655
<220> b1394 b1395
<222> 690-789
<223> b1394 b1395 U00096 1456288..1457076
<313> 113: 760-779
<400> 113
GGAACGTGAC TATCAGCGAC TTGCCGGACG GAGCGCAGAT TATCGTGAAG 50
GTGTCAGTGC GTTCCTGGCT AAACGCTCAC CGCAGTTCAC GGGGAAATAG 100
<210> 114
<211> 100
<212> DNA
<213> Escherichia coli K-12 MG1655
<220> b1394 b1395
<222> 681-780
<223> b1394 b1395 U00096 1456288..1457076
<313> 114: 729-748
<400> 114
ACTGGATCTG GAACGTGACT ATCAGCGACT TGCCGGACGG AGCGCAGATT 50
ATCGTGAAGG TGTCAGTGCG TTCCTGGCTA AACGCTCACC GCAGTTCACG 100
<210> 115
<211> 100
<212> DNA
<213> Escherichia coli K-12 MG1655
<220> b1394 b1395
<222> 581-680
<223> b1394 b1395 U00096 1456288..1457076
<313> 115: 625-644
<400> 115
CGCAACAGCT GGCACGGCAT CTGGCGACAC AACCGACATT TGGTCTTGGA 50
CTTATCAAGC AAGCGATAAA TAGCGCTGAA ACCAATACGC TCGATACGCA 100
<210> 116
<211> 100
<212> DNA
<213> Escherichia coli K-12 MG1655
<220> b1396 b1396
<222> 324-423
<223> b1396 b1396 U00096 1458495..1458917
<313> 116: 385-404
<400> 116

TGTTTACGAC ATCGAAATTG TTAACCAACA ACAAAAAACG GTTGCGCTGT 50
TTCGCGGTAA ATCTCACCGC ATCGGCGGCA CCATTACAGG AGAAGCCTGA 100
<210> 117
<211> 100
<212> DNA
<213> Escherichia coli K-12 MG1655
<220> b1396 b1396
<222> 240-339
<223> b1396 b1396 U00096 1458495..1458917
<313> 117: 264-283
<400> 117
CACGATTGAT TTTTTGCGTC CAGGCTTTGC CGGAGACACC TTAACTGCTA 50
CTGCGCAGGT ACGTCATCAG GGCAAGCAAA CCGGTGTTTA CGACATCGAA 100
<210> 118
<211> 100
<212> DNA
<213> Escherichia coli K-12 MG1655
<220> b1396 b1396
<222> 172-271
<223> b1396 b1396 U00096 1458495..1458917
<313> 118: 225-244
<400> 118
TCACTGGCTG ATACTGCCTT TGCCTACGCC TGCAATAGCC AGGGGCTGGC 50
AGCCGTCGCT TCTGCCTGCA CGATTGATTT TTTGCGTCCA GGCTTTGCCG 100
<210> 119
<211> 100
<212> DNA
<213> Escherichia coli K-12 MG1655
<220> b0667 b0668
<222> 16-115
<223> b0667 b0668 U00096 695931..696068
<313> 119: 35-54
<400> 119
CAACTGAGCT ACGTAGCCAG ATTGTTTCTT CGATGGCTGG GGTACCTGGA 50
TTCGAACCAG GGAATGCCGG TATCAAAAAC CGGTGCCTTA CCGCTTGGCG 100
<210> 120
<211> 100
<212> DNA
<213> Escherichia coli K-12 MG1655
<220> b1397 b1397
<222> 973-1072
<223> b1397 b1397 U00096 1458917..1460122
<313> 120: 1023-1042
<400> 120
GCGTTCGCGG CCCAGGCGTT GGGTGTACTA CGCGAATTGG GGCTGCCTGA 50
TGATGCCCCA CATGTTAACC CCAACGGAGG CGCTATCGCC TTAGGCCATC 100
<210> 121
<211> 100
<212> DNA
<213> Escherichia coli K-12 MG1655
<220> b1397 b1397
<222> 930-1029
<223> b1397 b1397 U00096 1458917..1460122
<313> 121: 970-989
<400> 121
AGGGCTGAGT ATTCACGATA TGGACGTGAT TGAACTGAAC GAAGCGTTCG 50
CGGCCCAGGC GTTGGGTGTA CTACGCGAAT TGGGGCTGCC TGATGATGCC 100
<210> 122
<211> 100
<212> DNA
<213> Escherichia coli K-12 MG1655
<220> b1397 b1397
<222> 882-981
<223> b1397 b1397 U00096 1458917..1460122
<313> 122: 917-936
<400> 122
GATGGGGCTT GGTCCGGTGC CTGCAACTCG CCGGGTGCTG GAACGCGCAG 50
GGCTGAGTAT TCACGATATG GACGTGATTG AACTGAACGA AGCGTTCGCG 100
<210> 123
<211> 100
<212> DNA

<213> Escherichia coli K-12 MG1655
<220> b1398 b1398
<222> 1215-1314
<223> b1398 b1398 U00096 1460149..1461462
<313> 123: 1236-1255
<400> 123
GATCTCTACC GATGTGATGA TCGTTAACTG TGGCAGTATC CCGCGTTCAG 50
AAGGCAAGGC GTGTCGGGTG TTTGATCTGC GCAATATTGT TGGTGCCTGA 100
<210> 124
<211> 100
<212> DNA
<213> Escherichia coli K-12 MG1655
<220> b1398 b1398
<222> 1107-1206
<223> b1398 b1398 U00096 1460149..1461462
<313> 124: 1132-1151
<400> 124
TTCACTTTCT GTGAAAGTGG AGTTGAAAGA AAGTAGCCTG ACATTGACGC 50
ATGAGCAGCG TTGCCAGGTA TGCCACCAGC TGCGCCATCG GATTAAGTCG 100
<210> 125
<211> 100
<212> DNA
<213> Escherichia coli K-12 MG1655
<220> b1398 b1398
<222> 1058-1157
<223> b1398 b1398 U00096 1460149..1461462
<313> 125: 1082-1101
<400> 125
ATTTATCGCC GCATTACCAA CTGGAGGTGA ACCGCCGGGG GCATCTTGAT 50
TCACTTTCTG TGAAAGTGGA GTTGAAAGAA AGTAGCCTGA CATTGACGCA 100
<210> 126
<211> 100
<212> DNA
<213> Escherichia coli K-12 MG1655
<220> b0669 b0670
<222> 21-120
<223> b0669 b0670 U00096 696065..696184
<313> 126: 90-109
<400> 126
AGATGGCTGG GGTACCTGGA TTCGAACCAG GGAATGCCGG TATCAAAAAC 50
CGGTGCCTTA CCGCTTGGCG ATACCCCATC CGTACAACGC TTTCTGGTGA 100
<210> 127
<211> 100
<212> DNA
<213> Escherichia coli K-12 MG1655
<220> b0669 b0670
<222> 21-120
<223> b0669 b0670 U00096 696065..696184
<313> 127: 82-101
<400> 127
AGATGGCTGG GGTACCTGGA TTCGAACCAG GGAATGCCGG TATCAAAAAC 50
CGGTGCCTTA CCGCTTGGCG ATACCCCATC CGTACAACGC TTTCTGGTGA 100
<210> 128
<211> 100
<212> DNA
<213> Escherichia coli K-12 MG1655
<220> b1399 b1399
<222> 852-951
<223> b1399 b1399 U00096 1461563..1462513
<313> 128: 908-927
<400> 128
AACCTCGGTC GGTGAACTGC CTGCGCCGGG AAGCCTGTAT TTTCAACGTT 50
TTGGCGGCTT GAATATTGAA CAGGAGGCGT TATGCCAATT TATCAGATAG 100
<210> 129
<211> 100
<212> DNA
<213> Escherichia coli K-12 MG1655
<220> b1399 b1399
<222> 827-926
<223> b1399 b1399 U00096 1461563..1462513
<313> 129: 865-884

<400> 129
TAGCGTTCGT TAGTGAAAAA GGTGAAACCT CGGTCGGTGA ACTGCCTGCG  50
CCGGGAAGCC TGTATTTTCA ACGTTTTGGC GGCTTGAATA TTGAACAGGA  100
<210> 130
<211> 100
<212> DNA
<213> Escherichia coli K-12 MG1655
<220> b1399 b1399
<222> 775-874
<223> b1399 b1399 U00096 1461563..1462513
<313> 130: 804-823
<400> 130
ACGGCGCGTC AGCTGTGTAT CAACATTTAT CAGCGCGTAG CGCCTGCTGC  50
TTTAGCGTTC GTTAGTGAAA AAGGTGAAAC CTCGGTCGGT GAACTGCCTG  100
<210> 131
<211> 100
<212> DNA
<213> Escherichia coli K-12 MG1655
<220> fnr b1335
<222> 654-753
<223> fnr b1335 U00096 complement(1396798..1397550)
<313> 131: 703-722
<400> 131
CCAGAAAAGC GGCATGCTGG CAGTCAAAGG TAAATACATC ACCATCGAAA  50
ATAACGATGC GCTGGCCCAG CTTGCTGGTC ATACGCGTAA CGTTGCCTGA  100
<210> 132
<211> 100
<212> DNA
<213> Escherichia coli K-12 MG1655
<220> fnr b1335
<222> 581-680
<223> fnr b1335 U00096 complement(1396798..1397550)
<313> 132: 636-655
<400> 132
CGATGACTCG TGGCGATATC GGTAACTATC TGGGCCTGAC GGTAGAAACC  50
ATCAGCCGTC TGCTGGGTCG CTTCCAGAAA AGCGGCATGC TGGCAGTCAA  100
<210> 133
<211> 100
<212> DNA
<213> Escherichia coli K-12 MG1655
<220> fnr b1335
<222> 549-648
<223> fnr b1335 U00096 complement(1396798..1397550)
<313> 133: 561-580
<400> 133
ACGCGGCTTC TCCCCTCGTG AATTCCGCCT GACGATGACT CGTGGCGATA  50
TCGGTAACTA TCTGGGCCTG ACGGTAGAAA CCATCAGCCG TCTGCTGGGT  100
<210> 134
<211> 100
<212> DNA
<213> Escherichia coli K-12 MG1655
<220> b0671 b0672
<222> 54-153
<223> b0671 b0672 U00096 696185..696337
<313> 134: 122-141
<400> 134
ATCTGGTGCG TCTACCAATT TCGCCACTCC CGCAAAAAAA GATGGTGGCT  50
ACGACGGGAT TCGAACCTGT GACCCCATCA TTATGAGTGA TGTGCTCTAA  100
<210> 135
<211> 100
<212> DNA
<213> Escherichia coli K-12 MG1655
<220> b0671 b0672
<222> 33-132
<223> b0671 b0672 U00096 696185..696337
<313> 135: 72-91
<400> 135
CCTTGCGGCG CCAGAACCTA AATCTGGTGC GTCTACCAAT TTCGCCACTC  50
CCGCAAAAAA AGATGGTGGC TACGACGGGA TTCGAACCTG TGACCCCATC  100
<210> 136
<211> 100

<212> DNA
<213> Escherichia coli K-12 MG1655
<220> upp b2499
<222> 555-654
<223> upp b2499 U00096 complement(2618266..2618919)
<313> 136: 597-616
<400> 136
CGAACTGTAT ACCGCATCGA TTGATCAGGG ACTGAACGAG CACGGATACA 50
TTATTCCGGG CCTCGGCGAT GCCGGTGACA AAATCTTTGG TACGAAATAA 100
<210> 137
<211> 100
<212> DNA
<213> Escherichia coli K-12 MG1655
<220> upp b2499
<222> 191-290
<223> upp b2499 U00096 complement(2618266..2618919)
<313> 137: 204-223
<400> 137
AAGGCTGGAA CGGCCCGGTA GAAATCGACC AGATCAAAGG TAAGAAAATT 50
ACCGTTGTGC CAATTCTGCG TGCGGGTCTT GGTATGATGG ACGGTGTGCT 100
<210> 138
<211> 100
<212> DNA
<213> Escherichia coli K-12 MG1655
<220> upp b2499
<222> 123-222
<223> upp b2499 U00096 complement(2618266..2618919)
<313> 138: 172-191
<400> 138
TTCCGAAGTG GGTAGCCTGC TGACTTACGA AGCGACCGCC GACCTCGAAA 50
CGGAAAAAGT AACTATCGAA GGCTGGAACG GCCCGGTAGA AATCGACCAG 100
<210> 139
<211> 100
<212> DNA
<213> Escherichia coli K-12 MG1655
<220> csie b2536
<222> 1203-1302
<223> csie b2536 U00096 2663434..2664735
<313> 139: 1279-1298
<400> 139
CATGCCATTA CCGCTCTTTT CACCACCGCT GATCTATACG GACCTGACGC 50
TGACAACACA TCAACAGGAG CAGATCCGCA AAATGCTCGA ATCAGCATGA 100
<210> 140
<211> 100
<212> DNA
<213> Escherichia coli K-12 MG1655
<220> csie b2536
<222> 1198-1297
<223> csie b2536 U00096 2663434..2664735
<313> 140: 1240-1259
<400> 140
TATACCATGC CATTACCGCT CTTTTCACCA CCGCTGATCT ATACGGACCT 50
GACGCTGACA ACACATCAAC AGGAGCAGAT CCGCAAAATG CTCGAATCAG 100
<210> 141
<211> 100
<212> DNA
<213> Escherichia coli K-12 MG1655
<220> csie b2536
<222> 1165-1264
<223> csie b2536 U00096 2663434..2664735
<313> 141: 1186-1205
<400> 141
GCTCCGCGTG GCGCGGCACT GATTATTGCG CCTTATACCA TGCCATTACC 50
GCTCTTTTCA CCACCGCTGA TCTATACGGA CCTGACGCTG ACAACACATC 100
<210> 142
<211> 100
<212> DNA
<213> Escherichia coli K-12 MG1655
<220> abrb b0716
<222> 967-1066
<223> abrb b0716 U00096 complement(745946..747037)

<313> 142: 1019-1038
<400> 142
GCAGGGAGCA ATGCCGATAT GGCGCTCATT ATGGCGATGC AAACCCTACG 50
CCTGTTCAGT ATTTTGCTGA CAGGGCCTGC CATTGCGCGG TTTATTTCAA 100
<210> 143
<211> 100
<212> DNA
<213> Escherichia coli K-12 MG1655
<220> abrb b0716
<222> 938-1037
<223> abrb b0716 U00096 complement(745946..747037)
<313> 143: 992-1011
<400> 143
GCGGGCTTGA TACCGTGGCG GTCATCGCCG CAGGGAGCAA TGCCGATATG 50
GCGCTCATTA TGGCGATGCA AACCCTACGC CTGTTCAGTA TTTTGCTGAC 100
<210> 144
<211> 100
<212> DNA
<213> Escherichia coli K-12 MG1655
<220> abrb b0716
<222> 857-956
<223> abrb b0716 U00096 complement(745946..747037)
<313> 144: 909-928
<400> 144
CTATTTGTGC GGGTATGGCG TGGGGGCTGA CCCGGTTTAT GCACATTGAT 50
TTTATGACTG CCTACCTCGC CACCAGCCCC GGCGGGCTTG ATACCGTGGC 100
<210> 145
<211> 100
<212> DNA
<213> Escherichia coli K-12 MG1655
<220> fpr b3925
<222> 648-747
<223> fpr b3925 U00096 complement(4111306..4112052)
<313> 145: 714-733
<400> 145
GATGGTGCGC GATACACAAC AGTTGCTGAA AGAGACCCGG CAGATGACGA 50
AACATTTACG TCGCCGACCG GGCCATATGA CAGCGGAGCA TTACTGGTAA 100
<210> 146
<211> 100
<212> DNA
<213> Escherichia coli K-12 MG1655
<220> fpr b3925
<222> 645-744
<223> fpr b3925 U00096 complement(4111306..4112052)
<313> 146: 667-686
<400> 146
ACAGATGGTG CGCGATACAC AACAGTTGCT GAAAGAGACC CGGCAGATGA 50
CGAAACATTT ACGTCGCCGA CCGGGCCATA TGACAGCGGA GCATTACTGG 100
<210> 147
<211> 100
<212> DNA
<213> Escherichia coli K-12 MG1655
<220> fpr b3925
<222> 555-654
<223> fpr b3925 U00096 complement(4111306..4112052)
<313> 147: 611-630
<400> 147
ACCGGCATTA ATTGAAAGTG GGGAACTGGA AAGCACGATT GGCCTGCCGA 50
TGAATAAAGA AACCAGCCAT GTGATGCTGT GCGGCAATCC ACAGATGGTG 100
<210> 148
<211> 100
<212> DNA
<213> Escherichia coli K-12 MG1655
<220> b4250 b4251
<222> 66-165
<223> b4250 b4251 U00096 4471801..4471965
<313> 148: 136-155
<400> 148
TTTGGCAGTA AAGCTGGGTT ATTTAGCCGT GTACTCAATG AATACGTCGG 50
TACGGAAGCT ATTCCGCTTG CCGATATTCT TCGTGATGAT CGTCCGGTAG 100
<210> 149

<211> 100
<212> DNA
<213> Escherichia coli K-12 MG1655
<220> b4250 b4251
<222> 66-165
<223> b4250 b4251 U00096 4471801..4471965
<313> 149: 104-123
<400> 149
TTTGGCAGTA AAGCTGGGTT ATTTAGCCGT GTACTCAATG AATACGTCGG 50
TACGGAAGCT ATTCCGCTTG CCGATATTCT TCGTGATGAT CGTCCGGTAG 100
<210> 150
<211> 100
<212> DNA
<213> Escherichia coli K-12 MG1655
<220> b4250 b4251
<222> 62-161
<223> b4250 b4251 U00096 4471801..4471965
<313> 150: 96-115
<400> 150
GGCTTTTGGC AGTAAAGCTG GGTTATTTAG CCGTGTACTC AATGAATACG 50
TCGGTACGGA AGCTATTCCG CTTGCCGATA TTCTTCGTGA TGATCGTCCG 100
<210> 151
<211> 100
<212> DNA
<213> Escherichia coli K-12 MG1655
<220> nac b1989
<222> 819-918
<223> nac b1989 U00096 complement(2059038..2059955)
<313> 151: 888-907
<400> 151
AGCGAACTTA TCGCCACAGG CACAGGCAGT GAAAGAGTTG TTAATGTCAG 50
TGATTAGTTC TCCAGTGATG GAAAAAAGGC AGTGGCAATT GGTGAGCTAA 100
<210> 152
<211> 100
<212> DNA
<213> Escherichia coli K-12 MG1655
<220> nac b1989
<222> 800-899
<223> nac b1989 U00096 complement(2059038..2059955)
<313> 152: 844-863
<400> 152
CATTAAATTT ACCCGCCAGA GCGAACTTAT CGCCACAGGC ACAGGCAGTG 50
AAAGAGTTGT TAATGTCAGT GATTAGTTCT CCAGTGATGG AAAAAAGGCA 100
<210> 153
<211> 100
<212> DNA
<213> Escherichia coli K-12 MG1655
<220> nac b1989
<222> 717-816
<223> nac b1989 U00096 complement(2059038..2059955)
<313> 153: 782-801
<400> 153
CGAATCGGCC GCGCGTTCGT TATGTGGCGC AGTAAATGGG TGGATGTCAC 50
GCATTACCAC TCCTTCCATG AGTCTCTCTT TGTCATTAAA TTTACCCGCC 100
<210> 154
<211> 100
<212> DNA
<213> Escherichia coli K-12 MG1655
<220> b4256 b4257
<222> 195-294
<223> b4256 b4257 U00096 complement(4476597..4476890)
<313> 154: 253-272
<400> 154
CATTGCGCTT TATGAGCATT TGGGCTTTGA GCATATCGAC TATGCGCTTG 50
GCTGCACGGG CCATGTCGAT TGTGAAGTGC GGATGTTGCG CGAACTCTAA 100
<210> 155
<211> 100
<212> DNA
<213> Escherichia coli K-12 MG1655
<220> b4256 b4257
<222> 182-281

<223> b4256 b4257 U00096 complement(4476597..4476890)
<313> 155: 200-219
<400> 155
TTTTAAAGGA AGCCATTGCG CTTTATGAGC ATTTGGGCTT TGAGCATATC  50
GACTATGCGC TTGGCTGCAC GGGCCATGTC GATTGTGAAG TGCGGATGTT  100
<210> 156
<211> 100
<212> DNA
<213> Escherichia coli K-12 MG1655
<220> b4256 b4257
<222> 110-209
<223> b4256 b4257 U00096 complement(4476597..4476890)
<313> 156: 168-187
<400> 156
AACTGGCCTT AATGGCGATG GAGCAGGCGC GAGAGATGGG TTTCAAACGC  50
TGCTATCTGG AAACGACCGC TTTTTTAAAG GAAGCCATTG CGCTTTATGA  100
<210> 157
<211> 100
<212> DNA
<213> Escherichia coli K-12 MG1655
<220> frda b4155
<222> 1710-1809
<223> frda b4155 U00096 complement(4378088..4379896)
<313> 157: 1769-1788
<400> 157
GATTACTACG CTGCCGCCAG CTAAACGCGT TTACGGTGGC GAAGCGGATG  50
CAGCCGATAA GGCGGAAGCA GCCAATAAGA AGGAGAAGGC GAATGGCTGA  100
<210> 158
<211> 100
<212> DNA
<213> Escherichia coli K-12 MG1655
<220> frda b4155
<222> 1672-1771
<223> frda b4155 U00096 complement(4378088..4379896)
<313> 158: 1713-1732
<400> 158
GCTGATGGCA CGACTCGCCT GGAGTACAGC GACGTGAAGA TTACTACGCT  50
GCCGCCAGCT AAACGCGTTT ACGGTGGCGA AGCGGATGCA GCCGATAAGG  100
<210> 159
<211> 100
<212> DNA
<213> Escherichia coli K-12 MG1655
<220> frda b4155
<222> 1626-1725
<223> frda b4155 U00096 complement(4378088..4379896)
<313> 159: 1658-1677
<400> 159
GCGTGACGAC GTCAACTTCC TCAAACACAC CCTCGCCTTC CGCGATGCTG  50
ATGGCACGAC TCGCCTGGAG TACAGCGACG TGAAGATTAC TACGCTGCCG  100
<210> 160
<211> 100
<212> DNA
<213> Escherichia coli K-12 MG1655
<220> frdb b4154
<222> 623-722
<223> frdb b4154 U00096 complement(4377361..4378095)
<313> 160: 683-702
<400> 160
TGGGCTACTG CTCCGAAGTC TGCCCGAAAC ACGTCGATCC GGCTGCGGCC  50
ATTCAGCAGG GCAAAGTAGA AAGTTCGAAA GACTTTCTTA TCGCGACCCT  100
<210> 161
<211> 100
<212> DNA
<213> Escherichia coli K-12 MG1655
<220> frdb b4154
<222> 589-688
<223> frdb b4154 U00096 complement(4377361..4378095)
<313> 161: 633-652
<400> 161
AACAGCCAGA ACGGCGTATG GAGCTGTACT TTCGTGGGCT ACTGCTCCGA  50
AGTCTGCCCG AAACACGTCG ATCCGGCTGC GGCCATTCAG CAGGGCAAAG  100

<210> 162
<211> 100
<212> DNA
<213> Escherichia coli K-12 MG1655
<220> frdb b4154
<222> 545-644
<223> frdb b4154 U00096 complement(4377361..4378095)
<313> 162: 592-611
<400> 162
AAGATAGCCG CGACCACGGT AAGAAGGAGC GTATGGCGCA GTTGAACAGC 50
CAGAACGGCG TATGGAGCTG TACTTTCGTG GGCTACTGCT CCGAAGTCTG 100
<210> 163
<211> 100
<212> DNA
<213> Escherichia coli K-12 MG1655
<220> frdc b4153
<222> 297-396
<223> frdc b4153 U00096 complement(4376955..4377350)
<313> 163: 359-378
<400> 163
AAAAGACGAA AAAATGGGAC CAGAGCCAAT TATCAAAAGT CTCTGGGCGG 50
TAACTGTGGT TGCCACCATC GTAATCCTGT TTGTTGCCCT GTACTGGTAA 100
<210> 164
<211> 100
<212> DNA
<213> Escherichia coli K-12 MG1655
<220> frdc b4153
<222> 259-358
<223> frdc b4153 U00096 complement(4376955..4377350)
<313> 164: 271-290
<400> 164
TGGTTTGAAC TGGCACCGAA AGCGGCCAAT ATCATTGTAA AAGACGAAAA 50
AATGGGACCA GAGCCAATTA TCAAAAGTCT CTGGGCGGTA ACTGTGGTTG 100
<210> 165
<211> 100
<212> DNA
<213> Escherichia coli K-12 MG1655
<220> frdc b4153
<222> 32-131
<223> frdc b4153 U00096 complement(4376955..4377350)
<313> 165: 87-106
<400> 165
CAATGACGTC CACCTGGTGG AAAAAATTGC CGTTTTATCG CTTTTACATG 50
CTGCGCGAAG GCACGGCGGT TCCGGCTGTG TGGTTCAGCA TTGAACTGAT 100
<210> 166
<211> 100
<212> DNA
<213> Escherichia coli K-12 MG1655
<220> frdd b4152
<222> 261-360
<223> frdd b4152 U00096 complement(4376585..4376944)
<313> 166: 306-325
<400> 166
GCACGATCTG AAAATCCACG TACCTGCGGG CAAATGGGTT TTCTACGGTC 50
TGGCTGCTAT CCTGACAGTT GTCACGCTGA TTGGTGTCGT TACAATCTAA 100
<210> 167
<211> 100
<212> DNA
<213> Escherichia coli K-12 MG1655
<220> frdd b4152
<222> 217-316
<223> frdd b4152 U00096 complement(4376585..4376944)
<313> 167: 251-270
<400> 167
GTTCTGCCGC TGTGGTGTGG TTTACACCGT ATGCACCACG CGATGCACGA 50
TCTGAAAATC CACGTACCTG CGGGCAAATG GGTTTTCTAC GGTCTGGCTG 100
<210> 168
<211> 100
<212> DNA
<213> Escherichia coli K-12 MG1655
<220> frdd b4152

<222> 129-228
<223> frdd b4152 U00096 complement(4376585..4376944)
<313> 168: 192-211
<400> 168
GTTGTTTCCG GGTGATGCGC TGAGCTACGA GCGCGTTCTG GCGTTCGCGC 50
AGAGCTTCAT TGGTCGCGTA TTCCTGTTCC TGATGATCGT TCTGCCGCTG 100
<210> 169
<211> 100
<212> DNA
<213> Escherichia coli K-12 MG1655
<220> usg b2320
<222> 915-1014
<223> usg b2320 U00096 complement(2433656..2434669)
<313> 169: 988-1007
<400> 169
GCAAGTCCAG TTCTGGTCGG TGGCCGATAA CGTTCGCTTT GGCGGCGCGC 50
TGATGGCAGT AAAAATCGCC GAGAAACTGG TGCAGGAGTA TCTGTACTAA 100
<210> 170
<211> 100
<212> DNA
<213> Escherichia coli K-12 MG1655
<220> usg b2320
<222> 814-913
<223> usg b2320 U00096 complement(2433656..2434669)
<313> 170: 863-882
<400> 170
GTGCTCTCTG AAGAGAACGA ATTCCCAACT CAGGTAGGTG ATGCTTCGGG 50
TACGCCGCAT CTTTCTGTTG GCTGCGTGCG TAATGACTAC GGTATGCCGG 100
<210> 171
<211> 100
<212> DNA
<213> Escherichia coli K-12 MG1655
<220> usg b2320
<222> 786-885
<223> usg b2320 U00096 complement(2433656..2434669)
<313> 171: 811-830
<400> 171
TGATGCGTTT GTTCAAGGCG AAGATATTGT GCTCTCTGAA GAGAACGAAT 50
TCCCAACTCA GGTAGGTGAT GCTTCGGGTA CGCCGCATCT TTCTGTTGGC 100
<210> 172
<211> 100
<212> DNA
<213> Escherichia coli K-12 MG1655
<220> b2809 b2809
<222> 132-231
<223> b2809 b2809 U00096 complement(2940940..2941170)
<313> 172: 206-225
<400> 172
TGATAACGAT ACCGGTATGA TTTCGTATAA AGACGCTAAT GGCAACAAAC 50
AGCAGATCAA CCGTACTGAC GTGAAAGAGA TGGTCGAACT GGATCAGTAA 100
<210> 173
<211> 100
<212> DNA
<213> Escherichia coli K-12 MG1655
<220> b2809 b2809
<222> 69-168
<223> b2809 b2809 U00096 complement(2940940..2941170)
<313> 173: 123-142
<400> 173
TTCGAACTAT GTGATGCACA CCAATGACGG ACGTACCATC GTCTCTGACG 50
GCAAACCACA GACTGATAAC GATACCGGTA TGATTTCGTA TAAAGACGCT 100
<210> 174
<211> 100
<212> DNA
<213> Escherichia coli K-12 MG1655
<220> b2809 b2809
<222> 17-116
<223> b2809 b2809 U00096 complement(2940940..2941170)
<313> 174: 36-55
<400> 174
CCGCAATTAT TTCTGCCTGT ATGCTGACTT TTGCCCTGAG CGCCTGTTCC 50

GGTTCGAACT ATGTGATGCA CACCAATGAC GGACGTACCA TCGTCTCTGA 100
<210> 175
<211> 100
<212> DNA
<213> Escherichia coli K-12 MG1655
<220> emra b2686
<222> 1074-1173
<223> emra b2686 U00096 2809448..2810620
<313> 175: 1130-1149
<400> 175
AGTACGTTCC ACTCCGGTAG CGGTAAGCAC CGCGCGTGAA ATCAGCCTGG 50
CACCTGTCAA TAAACTGATC GACGATATCG TAAAAGCTAA CGCTGGCTAA 100
<210> 176
<211> 100
<212> DNA
<213> Escherichia coli K-12 MG1655
<220> emra b2686
<222> 1039-1138
<223> emra b2686 U00096 2809448..2810620
<313> 176: 1084-1103
<400> 176
ACCACTAACC GTGACGGTCA GGTACTGGCA AATAAAGTAC GTTCCACTCC 50
GGTAGCGGTA AGCACCGCGC GTGAAATCAG CCTGGCACCT GTCAATAAAC 100
<210> 177
<211> 100
<212> DNA
<213> Escherichia coli K-12 MG1655
<220> emra b2686
<222> 941-1040
<223> emra b2686 U00096 2809448..2810620
<313> 177: 996-1015
<400> 177
AAGTCGTTCA GCGTCTGCCT GTGCGTATCG AACTGGACCA GAAACAGCTG 50
GAGCAATATC CGCTGCGTAT CGGTTTGTCC ACGCTGGTGA GCGTCAATAC 100
<210> 178
<211> 100
<212> DNA
<213> Escherichia coli K-12 MG1655
<220> emrb b2687
<222> 1440-1539
<223> emrb b2687 U00096 2810637..2812175
<313> 178: 1474-1493
<400> 178
CTTCTGGATG TCAGCCGGGA TATTCCTCGT CCTGCTGGGG CTGGTGTGGT 50
TTGCTAAACC GCCATTTGGC GCAGGTGGCG GCGGAGGCGG TGCGCACTAA 100
<210> 179
<211> 100
<212> DNA
<213> Escherichia coli K-12 MG1655
<220> emrb b2687
<222> 1363-1462
<223> emrb b2687 U00096 2810637..2812175
<313> 179: 1414-1433
<400> 179
ATGACGCAAC AGCAGGCATC AGGCTGGATT GCCCAGCAGA TCACCAATCA 50
GGGGCTGATT ATTTCCGCCA ATGAGATCTT CTGGATGTCA GCCGGGATAT 100
<210> 180
<211> 100
<212> DNA
<213> Escherichia coli K-12 MG1655
<220> emrb b2687
<222> 1331-1430
<223> emrb b2687 U00096 2810637..2812175
<313> 180: 1366-1385
<400> 180
AGGCGATGTA CAGTCAACTG GAAGGGCTTG GGATGACGCA ACAGCAGGCA 50
TCAGGCTGGA TTGCCCAGCA GATCACCAAT CAGGGGCTGA TTATTTCCGC 100
<210> 181
<211> 100
<212> DNA
<213> Escherichia coli K-12 MG1655

<220> emrd b3674
<222> 1092-1191
<223> emrd b3674 U00096 3851543..3852733
<313> 181: 1133-1152
<400> 181
GGGCAGCCTG GGGTTGTTGA TGACCTTAAT GGGATTGTTG ATCGTGCTGT 50
GCTGGCTGCC GCTGGCGACG CGGATGTCGC ATCAGGGGCA GCCCGTTTAA 100
<210> 182
<211> 100
<212> DNA
<213> Escherichia coli K-12 MG1655
<220> emrd b3674
<222> 664-763
<223> emrd b3674 U00096 3851543..3852733
<313> 182: 731-750
<400> 182
CTGGCCGGGA TTGCCGCCTT TGAAGCCTGC TCCGGCGTGC TGATGGGCGC 50
GGTGTTAGGG CTGAGCAGTA TGACGGTCAG TATTTTGTTT ATTCTGCCGA 100
<210> 183
<211> 100
<212> DNA
<213> Escherichia coli K-12 MG1655
<220> emrd b3674
<222> 556-655
<223> emrd b3674 U00096 3851543..3852733
<313> 183: 602-621
<400> 183
CCGGAAACGC GTCCGGTCGA TGCACCGCGC ACGCGCCTGC TTACCAGTTA 50
TAAAACGCTT TTCGGTAACA GCGGTTTTAA CTGTTATTTG CTGATGCTGA 100
<210> 184
<211> 100
<212> DNA
<213> Escherichia coli K-12 MG1655
<220> emre b0544
<222> 219-318
<223> emre b0544 U00096 567538..567870
<313> 184: 258-277
<400> 184
ACTGTCATGG GGATTTTTCG GCCAACGGCT GGACCTGCCA GCCATTATAG 50
GCATGATGTT GATTTGTGCC GGTGTGTTGA TTATTAATTT ATTGTCACGA 100
<210> 185
<211> 100
<212> DNA
<213> Escherichia coli K-12 MG1655
<220> emre b0544
<222> 116-215
<223> emre b0544 U00096 567538..567870
<313> 185: 159-178
<400> 185
GTTATTGTGC ATCATTCTGG TTATTAGCTC AGACGCTGGC TTATATTCCT 50
ACAGGGATTG CTTATGCTAT CTGGTCAGGA GTCGGTATTG TCCTGATTAG 100
<210> 186
<211> 100
<212> DNA
<213> Escherichia coli K-12 MG1655
<220> emre b0544
<222> 1-100
<223> emre b0544 U00096 567538..567870
<313> 186: 33-52
<400> 186
ATGAACCCTT ATATTTATCT TGGTGGTGCA ATACTTGCAG AGGTCATTGG 50
TACAACCTTA ATGAAGTTTT CAGAAGGTTT TACACGGTTA TGGCCATCTG 100
<210> 187
<211> 100
<212> DNA
<213> Escherichia coli K-12 MG1655
<220> frr b0173
<222> 434-533
<223> frr b0173 U00096 192872..193429
<313> 187: 472-491
<400> 187

ATAAAGAGAT CAGCGAAGAC GACGATCGCC GTTCTCAGGA CGATGTACAG 50
AAACTGACTG ATGCTGCAAT CAAGAAAATT GAAGCGGCGC TGGCAGACAA 100
<210> 188
<211> 100
<212> DNA
<213> Escherichia coli K-12 MG1655
<220> frr b0173
<222> 358-457
<223> frr b0173 U00096 192872..193429
<313> 188: 410-429
<400> 188
GAAGCAGAAC AAGCGCGTGT TGCAGTACGT AACGTGCGTC GTGACGCGAA 50
CGACAAAGTG AAAGCACTGT TGAAAGATAA AGAGATCAGC GAAGACGACG 100
<210> 189
<211> 100
<212> DNA
<213> Escherichia coli K-12 MG1655
<220> frr b0173
<222> 312-411
<223> frr b0173 U00096 192872..193429
<313> 189: 339-358
<400> 189
GCTGACGGAA GAACGTCGTA AAGATCTGAC CAAAATCGTT CGTGGTGAAG 50
CAGAACAAGC GCGTGTTGCA GTACGTAACG TGCGTCGTGA CGCGAACGAC 100
<210> 190
<211> 100
<212> DNA
<213> Escherichia coli K-12 MG1655
<220> damx b3389
<222> 1188-1287
<223> damx b3389 U00096 complement(3513658..3514944)
<313> 190: 1255-1274
<400> 190
AGAAGAGGCG AAAAAAGCGG TATCTACATT GCCAGCAGAT GTCCAGGCCA 50
AAAACCCGTG GGCGAAACCG CTGCGTCAGG TACAGGCCGA TCTGAAGTAA 100
<210> 191
<211> 100
<212> DNA
<213> Escherichia coli K-12 MG1655
<220> damx b3389
<222> 1162-1261
<223> damx b3389 U00096 complement(3513658..3514944)
<313> 191: 1224-1243
<400> 191
CTGGTTTCTG GCGTGTACGC TTCGAAAGAA GAGGCGAAAA AAGCGGTATC 50
TACATTGCCA GCAGATGTCC AGGCCAAAAA CCCGTGGGCG AAACCGCTGC 100
<210> 192
<211> 100
<212> DNA
<213> Escherichia coli K-12 MG1655
<220> damx b3389
<222> 1052-1151
<223> damx b3389 U00096 complement(3513658..3514944)
<313> 192: 1100-1119
<400> 192
AGCTGAGCAG TTCCTCTAAC TACGACAACC TGAACGGTTG GGCGAAGAAA 50
GAGAATCTGA AAAACTACGT TGTCTATGAA ACGACGCGTA ATGGTCAGCC 100
<210> 193
<211> 100
<212> DNA
<213> Escherichia coli K-12 MG1655
<220> b2810 b2811
<222> 1079-1178
<223> b2810 b2811 U00096 2941359..2942564
<313> 193: 1133-1152
<400> 193
AGCCGCTACT GGCAGAATTA GGCGTAACCG GCACACTGCG CGCCTCTTTT 50
GCGCCATATA ATACAAAGAG TGATGTGGAT GCGCTGGTGA ATGCCGTTGA 100
<210> 194
<211> 100
<212> DNA

<213> Escherichia coli K-12 MG1655
<220> b2810 b2811
<222> 1062-1161
<223> b2810 b2811 U00096 2941359..2942564
<313> 194: 1084-1103
<400> 194
CGGGCAGCAT TGCGCTCAGC CGCTACTGGC AGAATTAGGC GTAACCGGCA 50
CACTGCGCGC CTCTTTTGCG CCATATAATA CAAAGAGTGA TGTGGATGCG 100
<210> 195
<211> 100
<212> DNA
<213> Escherichia coli K-12 MG1655
<220> b2810 b2811
<222> 923-1022
<223> b2810 b2811 U00096 2941359..2942564
<313> 195: 968-987
<400> 195
CGGAAGATGC GCTGGCGAAA CGTCCCGGCT TTCGTTCATT CCGCTGCCAG 50
GATTCCAGCC TGCTGGCCTT TGATTTTGCT GGCGTTCATC ATAGCGATAT 100
<210> 196
<211> 100
<212> DNA
<213> Escherichia coli K-12 MG1655
<220> mtla b3600
<222> 1807-1906
<223> mtla b3600 U00096 3769908..3771821
<313> 196: 1854-1873
<400> 196
GTTATCACCA GCCTGACCAA TGCACTGGAT GATGAGTCCG TCATCGAGCG 50
TCTGGCACAC ACCACCAGCG TGGATGAAGT GCTGGAACTG CTGGCAGGTC 100
<210> 197
<211> 100
<212> DNA
<213> Escherichia coli K-12 MG1655
<220> mtla b3600
<222> 1794-1893
<223> mtla b3600 U00096 3769908..3771821
<313> 197: 1828-1847
<400> 197
CGAGCACATT CAGGTTATCA CCAGCCTGAC CAATGCACTG GATGATGAGT 50
CCGTCATCGA GCGTCTGGCA CACACCACCA GCGTGGATGA AGTGCTGGAA 100
<210> 198
<211> 100
<212> DNA
<213> Escherichia coli K-12 MG1655
<220> mtla b3600
<222> 1740-1839
<223> mtla b3600 U00096 3769908..3771821
<313> 198: 1769-1788
<400> 198
TGAAGAAGAA GATGACATTG CCCGTCTGGT GATTGGTATT GCTGCCCGTA 50
ACAACGAGCA CATTCAGGTT ATCACCAGCC TGACCAATGC ACTGGATGAT 100
<210> 199
<211> 100
<212> DNA
<213> Escherichia coli K-12 MG1655
<220> emrk b2369
<222> 995-1094
<223> emrk b2369 U00096 complement(2480196..2481359)
<313> 199: 1032-1051
<400> 199
TATCGATGAC AGCAACTATT GATACGAAGA ACGAAGACAT TGCCGAGATG 50
CCTGAGCTGG CTTCAACCGT GACCTCCATG CCGGCTTATA CCAGTAAGGC 100
<210> 200
<211> 100
<212> DNA
<213> Escherichia coli K-12 MG1655
<220> emrk b2369
<222> 938-1037
<223> emrk b2369 U00096 complement(2480196..2481359)
<313> 200: 960-979

<400> 200
CGGTTGAAGT TTCTCTTGAT CCAAAAGAAC TCATGGAACA CCCCTTGCGT 50
ATTGGTTTAT CGATGACAGC AACTATTGAT ACGAAGAACG AAGACATTGC 100
<210> 201
<211> 100
<212> DNA
<213> Escherichia coli K-12 MG1655
<220> emrk b2369
<222> 867-966
<223> emrk b2369 U00096 complement(2480196..2481359)
<313> 201: 911-930
<400> 201
CGGCAATGCG TTCTCCTTAT TACCTGCACA AAATGCGACA GGGAACTGGA 50
TCAAAATCGT TCAGCGTGTA CCGGTTGAAG TTTCTCTTGA TCCAAAAGAA 100
<210> 202
<211> 100
<212> DNA
<213> Escherichia coli K-12 MG1655
<220> mtld b3601
<222> 1050-1149
<223> mtld b3601 U00096 3772051..3773199
<313> 202: 1105-1124
<400> 202
TGACAAAGGT CCGCAGGCGG CGCTGGCACA GATTTCCGGT CTTGATGCCA 50
ACAGCGAGGT TGTATCCGAG GCGGTAACCG CTTATAAAGC AATGCAATAA 100
<210> 203
<211> 100
<212> DNA
<213> Escherichia coli K-12 MG1655
<220> mtld b3601
<222> 984-1083
<223> mtld b3601 U00096 3772051..3773199
<313> 203: 1038-1057
<400> 203
TGCCGCTGCA ATGCACTTCC GCAGTGAAGA TGATCCGCAG GCTCAGGAAC 50
TGGCAGCACT GATCGCTGAC AAAGGTCCGC AGGCGGCGCT GGCACAGATT 100
<210> 204
<211> 100
<212> DNA
<213> Escherichia coli K-12 MG1655
<220> mtld b3601
<222> 958-1057
<223> mtld b3601 U00096 3772051..3773199
<313> 204: 994-1013
<400> 204
CCACATAAAA ACCTGATTGA AGGTATTGCC GCTGCAATGC ACTTCCGCAG 50
TGAAGATGAT CCGCAGGCTC AGGAACTGGC AGCACTGATC GCTGACAAAG 100
<210> 205
<211> 100
<212> DNA
<213> Escherichia coli K-12 MG1655
<220> b2817 b2818
<222> 1245-1344
<223> b2817 b2818 U00096 complement(2945779..2947122)
<313> 205: 1292-1311
<400> 205
ACTGAAAACG GCGACTTTCC AGCAGGAAGT TGCGGAGTCT ATTCTTGCGG 50
GGATTAAAGC GTATTTTGCC GATGGGGCGA CGCTGGCGAG AAGGGGATGA 100
<210> 206
<211> 100
<212> DNA
<213> Escherichia coli K-12 MG1655
<220> b2817 b2818
<222> 1188-1287
<223> b2817 b2818 U00096 complement(2945779..2947122)
<313> 206: 1252-1271
<400> 206
TATTCCCTCC ATTCTGGTCG AAACGGCGTT TATCAGTAAC GTTGAGGAAG 50
AGCGTAAACT GAAAACGGCG ACTTTCCAGC AGGAAGTTGC GGAGTCTATT 100
<210> 207
<211> 100

<212> DNA
<213> Escherichia coli K-12 MG1655
<220> b2817 b2818
<222> 1167-1266
<223> b2817 b2818 U00096 complement(2945779..2947122)
<313> 207: 1194-1213
<400> 207
TGCCGTACTA AAGGCACCAG ATATTCCCTC CATTCTGGTC GAAACGGCGT 50
TTATCAGTAA CGTTGAGGAA GAGCGTAAAC TGAAAACGGC GACTTTCCAG 100
<210> 208
<211> 100
<212> DNA
<213> Escherichia coli K-12 MG1655
<220> emrr b2685
<222> 432-531
<223> emrr b2685 U00096 2808791..2809321
<313> 208: 509-528
<400> 208
CACAACAGAA AAAGATCAGC TCGAGCAAAT CACCCGCAAA TTGCTCTCCC 50
GTCTCGACCA GATGGAACAA GACGGTGTGG TTCTCGAAGC GATGAGCTAA 100
<210> 209
<211> 100
<212> DNA
<213> Escherichia coli K-12 MG1655
<220> emrr b2685
<222> 399-498
<223> emrr b2685 U00096 2808791..2809321
<313> 209: 431-450
<400> 209
TAACTGCCTG CATCAACTCT GGTCCGCGCT CAGCACAACA GAAAAAGATC 50
AGCTCGAGCA AATCACCCGC AAATTGCTCT CCCGTCTCGA CCAGATGGAA 100
<210> 210
<211> 100
<212> DNA
<213> Escherichia coli K-12 MG1655
<220> emrr b2685
<222> 357-456
<223> emrr b2685 U00096 2808791..2809321
<313> 210: 384-403
<400> 210
AAAAGGTCAC GAGTTTTTGC GCGAGGTTTT ACCACCGCAG CATAACTGCC 50
TGCATCAACT CTGGTCCGCG CTCAGCACAA CAGAAAAAGA TCAGCTCGAG 100
<210> 211
<211> 100
<212> DNA
<213> Escherichia coli K-12 MG1655
<220> emry b2368
<222> 1245-1344
<223> emry b2368 U00096 complement(2478658..2480196)
<313> 211: 1275-1294
<400> 211
AGTTGGTACG TCGTTGACAA TGACGCTGTG GGGACGACGC GAATCGTTAC 50
ACCATAGTCA GTTGACAGCA ACCATCGATC AATTTAACCC CGTGTTTAAT 100
<210> 212
<211> 100
<212> DNA
<213> Escherichia coli K-12 MG1655
<220> emry b2368
<222> 1104-1203
<223> emry b2368 U00096 complement(2478658..2480196)
<313> 212: 1143-1162
<400> 212
CATTTTGCCG CAGTTTTTTC AGGGATTCGC CGTTGCCTGT TTCTTTTTAC 50
CCTTAACAAC GATTTCGTTT TCAGGCTTGC CAGATAATAA ATTTGCCAAT 100
<210> 213
<211> 100
<212> DNA
<213> Escherichia coli K-12 MG1655
<220> emry b2368
<222> 1077-1176
<223> emry b2368 U00096 complement(2478658..2480196)

<313> 213: 1103-1122
<400> 213
TATGCCAACG ATTGATTTTA CAGGCATCAT TTTGCCGCAG TTTTTTCAGG 50
GATTCGCCGT TGCCTGTTTC TTTTTACCCT TAACAACGAT TTCGTTTTCA 100
<210> 214
<211> 100
<212> DNA
<213> Escherichia coli K-12 MG1655
<220> mtlr b3602
<222> 489-588
<223> mtlr b3602 U00096 3773199..3773786
<313> 214: 535-554
<400> 214
GCAAATTCAG CGCTATCAAC AGGCTGTGCG ATCAACAATG GTCCTTTCAC 50
TGACTGAGCT GATTTCCAAA ATCAGCTTAA AAAAAGCCTT TCAAAAGTAA 100
<210> 215
<211> 100
<212> DNA
<213> Escherichia coli K-12 MG1655
<220> mtlr b3602
<222> 359-458
<223> mtlr b3602 U00096 3773199..3773786
<313> 215: 399-418
<400> 215
TAAATCACGA CGGCAACGAG TACGCCTTTA CCGACGACGA AATCCTTGGA 50
CCCTTTGGTG AACTGCACTG CGTGGCGGCG TTACCACCGC CGCCACAGTT 100
<210> 216
<211> 100
<212> DNA
<213> Escherichia coli K-12 MG1655
<220> mtlr b3602
<222> 319-418
<223> mtlr b3602 U00096 3773199..3773786
<313> 216: 361-380
<400> 216
TACGAAGATG CGGAACTGCT GATGGCATTG CGTGAAGAGC TAAATCACGA 50
CGGCAACGAG TACGCCTTTA CCGACGACGA AATCCTTGGA CCCTTTGGTG 100
<210> 217
<211> 100
<212> DNA
<213> Escherichia coli K-12 MG1655
<220> yhab b3121
<222> 428-527
<223> yhab b3121 U00096 3265474..3266034
<313> 217: 444-463
<400> 217
AGTATTGTGC AAAATTCACC CGAAAAGAAG CCATCTTAAC CCCCCTTGTG 50
CACTTTAATA AAACCAGCAT TACTTCAGAT TGTTGGCAAA TTCTCTTTTT 100
<210> 218
<211> 100
<212> DNA
<213> Escherichia coli K-12 MG1655
<220> yhab b3121
<222> 304-403
<223> yhab b3121 U00096 3265474..3266034
<313> 218: 358-377
<400> 218
GGAGAACATG ATGATTATAT CATACAAACA TTAGATGTAG GCACTTTTGC 50
ACCTTTTGGT GAACAATGTA CTTGCTCAGC CGTCAATAAA AAAGAGCTGG 100
<210> 219
<211> 100
<212> DNA
<213> Escherichia coli K-12 MG1655
<220> yhab b3121
<222> 51-150
<223> yhab b3121 U00096 3265474..3266034
<313> 219: 77-96
<400> 219
TCACCCTTCA ATCCCGCCAA TGCACGCAGT GGTTAACAAT CACAATAGAA 50
ATATTGATTA TTGGACGGTA AAAAGAAAGT TTGCAGAAAT TGTCTCCACC 100
<210> 220

<211> 100
<212> DNA
<213> Escherichia coli K-12 MG1655
<220> yhac yhaC
<222> 1088-1187
<223> yhac yhaC U00096 3266056..3267243
<313> 220: 1121-1140
<400> 220
AGGTCGGGGA ATATAACACA TATGAAGCAT ATAACTCAGA GAAGCAATAT 50
TTTACCAGAG AGGACTATAC GTATGATTAC GACCTTTTAA ATGCAATATA 100
<210> 221
<211> 100
<212> DNA
<213> Escherichia coli K-12 MG1655
<220> yhac yhaC
<222> 1064-1163
<223> yhac yhaC U00096 3266056..3267243
<313> 221: 1098-1117
<400> 221
AAAATTCTCC GGACAATGGC TTTGAGGTCG GGGAATATAA CACATATGAA 50
GCATATAACT CAGAGAAGCA ATATTTTACC AGAGAGGACT ATACGTATGA 100
<210> 222
<211> 100
<212> DNA
<213> Escherichia coli K-12 MG1655
<220> yhac yhaC
<222> 1049-1148
<223> yhac yhaC U00096 3266056..3267243
<313> 222: 1094-1113
<400> 222
CCCTCGAGAA AGACGAAAAT TCTCCGGACA ATGGCTTTGA GGTCGGGGAA 50
TATAACACAT ATGAAGCATA TAACTCAGAG AAGCAATATT TTACCAGAGA 100
<210> 223
<211> 100
<212> DNA
<213> Escherichia coli K-12 MG1655
<220> dppa b3545
<222> 1453-1552
<223> dppa b3545 U00096 complement(3703727..3705334)
<313> 223: 1504-1523
<400> 223
GAACTGTACA AACAAGCGCA GGTGGTGATG CACGATCAGG CTCCGGCACT 50
GATCATCGCT CACTCCACCG TGTTTGAACC GGTACGTAAA GAAGTTAAAG 100
<210> 224
<211> 100
<212> DNA
<213> Escherichia coli K-12 MG1655
<220> dppa b3545
<222> 1420-1519
<223> dppa b3545 U00096 complement(3703727..3705334)
<313> 224: 1464-1483
<400> 224
GCGCGTGCTA CCGACGACCA CAATAAACGC GTTGAACTGT ACAAACAAGC 50
GCAGGTGGTG ATGCACGATC AGGCTCCGGC ACTGATCATC GCTCACTCCA 100
<210> 225
<211> 100
<212> DNA
<213> Escherichia coli K-12 MG1655
<220> dppa b3545
<222> 1359-1458
<223> dppa b3545 U00096 complement(3703727..3705334)
<313> 225: 1403-1422
<400> 225
TGAACAAGGC TCCAACTACT CAAAATGGTG CTACAAACCG TTTGAAGATC 50
TGATTCAACC GGCGCGTGCT ACCGACGACC ACAATAAACG CGTTGAACTG 100
<210> 226
<211> 100
<212> DNA
<213> Escherichia coli K-12 MG1655
<220> yhad b3125
<222> 1128-1227

<223> yhad b3125 U00096 complement(3268266..3269492)
<313> 226: 1182-1201
<400> 226
TACGTTGGAC GAAGCATTCC GCGGGGCTTA TGACAATATC TGCCGTGCTT 50
CACGTAATAT CGCCGCGACA CTGGCGATTG GAATGCGCAA CGCGGGGTGA 100
<210> 227
<211> 100
<212> DNA
<213> Escherichia coli K-12 MG1655
<220> yhad b3125
<222> 1068-1167
<223> yhad b3125 U00096 complement(3268266..3269492)
<313> 227: 1125-1144
<400> 227
TGTTGGCGTT GTACATCAGC ATGGCATTGA TGCGGTCTTC AGCGTATTGA 50
CCAGCATAGG TACGTTGGAC GAAGCATTCC GCGGGGCTTA TGACAATATC 100
<210> 228
<211> 100
<212> DNA
<213> Escherichia coli K-12 MG1655
<220> yhad b3125
<222> 970-1069
<223> yhad b3125 U00096 complement(3268266..3269492)
<313> 228: 1010-1029
<400> 228
AGCCAGAGTA TTCACGGGAA GGTACCGATT GGTGTCGCAA ACGTGGCGAA 50
GAAGTACCAT AAACCGGTGA TTGGCATTGC GGGTAGCCTG ACCGATGATG 100
<210> 229
<211> 100
<212> DNA
<213> Escherichia coli K-12 MG1655
<220> dppb b3544
<222> 874-973
<223> dppb b3544 U00096 complement(3702400..3703419)
<313> 229: 928-947
<400> 229
CGCTGGTTGA TTGACGCACT GCAACGCCGC GACTATCCGG TAGTGCAGGG 50
CGGCGTATTG CTGGTGGCGA CGATGATTAT CCTCGTCAAC TTGCTGGTCG 100
<210> 230
<211> 100
<212> DNA
<213> Escherichia coli K-12 MG1655
<220> dppb b3544
<222> 815-914
<223> dppb b3544 U00096 complement(3702400..3703419)
<313> 230: 853-872
<400> 230
GAACATTGCT GGCGGGGGCG ATTCTGACCG AAACCATCTT CTCGTGGCCC 50
GGTCTGGGAC GCTGGTTGAT TGACGCACTG CAACGCCGCG ACTATCCGGT 100
<210> 231
<211> 100
<212> DNA
<213> Escherichia coli K-12 MG1655
<220> dppb b3544
<222> 650-749
<223> dppb b3544 U00096 complement(3702400..3703419)
<313> 231: 688-707
<400> 231
TGGCGGTCAT TGTGCGTATG ACACGCTCCT CGATGCTGGA AGTGCTGGGC 50
GAGGATTACA TCCGCACCGC GCGCGCCAAA GGGCTAACCC GCATGCGGGT 100
<210> 232
<211> 100
<212> DNA
<213> Escherichia coli K-12 MG1655
<220> yhae b3126
<222> 801-900
<223> yhae b3126 U00096 complement(3269508..3270407)
<313> 232: 868-887
<400> 232
GATGATGCAG GCACTGCGAG CAGATGGTTT AGGAACGGCG GATCATAGCG 50
CCCTGGCGTG CTACTACGAA AAACTGGCGA AAGTCGAAGT TACTCGTTAA 100

<210> 233
<211> 100
<212> DNA
<213> Escherichia coli K-12 MG1655
<220> yhae b3126
<222> 797-896
<223> yhae b3126 U00096 complement(3269508..3270407)
<313> 233: 823-842
<400> 233
TGGAGATGAT GCAGGCACTG CGAGCAGATG GTTTAGGAAC GGCGGATCAT 50
AGCGCCCTGG CGTGCTACTA CGAAAAACTG GCGAAAGTCG AAGTTACTCG 100
<210> 234
<211> 100
<212> DNA
<213> Escherichia coli K-12 MG1655
<220> yhae b3126
<222> 690-789
<223> yhae b3126 U00096 complement(3269508..3270407)
<313> 234: 716-735
<400> 234
CAACTTCAAG CCGGGCTTCC GTATTGATCT GCATATTAAG GATCTGGCGA 50
ATGCGCTGGA TACTTCTCAC GGCGTCGGCG CACAACTGCC GCTCACAGCT 100
<210> 235
<211> 100
<212> DNA
<213> Escherichia coli K-12 MG1655
<220> dppc b3543
<222> 804-903
<223> dppc b3543 U00096 complement(3701488..3702390)
<313> 235: 883-902
<400> 235
CGTGACCTTC CCGGGTCTGG CGATCCTGCT GACGGTGCTG GCATTTAACC 50
TGATGGGTGA CGGTCTGCGT GACGCGCTCG ATCCCAAACT GAAGCAGTAA 100
<210> 236
<211> 100
<212> DNA
<213> Escherichia coli K-12 MG1655
<220> dppc b3543
<222> 708-807
<223> dppc b3543 U00096 complement(3701488..3702390)
<313> 236: 738-757
<400> 236
TCTTGGTTTC CTCGGCATGG GGGCACAGCC GCCAACGCCT GAGTGGGGCA 50
CCATGCTCTC CGACGTGTTG CAGTTCGCGC AAAGCGCCTG GTGGGTCGTG 100
<210> 237
<211> 100
<212> DNA
<213> Escherichia coli K-12 MG1655
<220> dppc b3543
<222> 678-777
<223> dppc b3543 U00096 complement(3701488..3702390)
<313> 237: 700-719
<400> 237
TTTCTCTAAC GCCATTCTCG ATATGGCTGC TCTTGGTTTC CTCGGCATGG 50
GGGCACAGCC GCCAACGCCT GAGTGGGGCA CCATGCTCTC CGACGTGTTG 100
<210> 238
<211> 100
<212> DNA
<213> Escherichia coli K-12 MG1655
<220> yhaf b3127
<222> 672-771
<223> yhaf b3127 U00096 complement(3270428..3271198)
<313> 238: 744-763
<400> 238
TCGTTATCTG GAATGGGGCG CGACGTTTGT GGCTGTCGGC AGCGATCTCG 50
GCGTCTTCCG CTCTGCCACT CAGAAACTGG CTGATACCTT TAAAAAATAA 100
<210> 239
<211> 100
<212> DNA
<213> Escherichia coli K-12 MG1655
<220> yhaf b3127

<222> 637-736
<223> yhaf b3127 U00096 complement(3270428..3271198)
<313> 239: 682-701
<400> 239
AGCGGTATCC TCGCGCCGGT CGAAGCCGAT GCGCGTCGTT ATCTGGAATG 50
GGGCGCGACG TTTGTGGCTG TCGGCAGCGA TCTCGGCGTC TTCCGCTCTG 100
<210> 240
<211> 100
<212> DNA
<213> Escherichia coli K-12 MG1655
<220> yhaf b3127
<222> 593-692
<223> yhaf b3127 U00096 complement(3270428..3271198)
<313> 240: 642-661
<400> 240
CAATTCAGCA CATTTTTAAC CGTGCCAGCG CGCACGGCAA ACCCAGCGGT 50
ATCCTCGCGC CGGTCGAAGC CGATGCGCGT CGTTATCTGG AATGGGGCGC 100
<210> 241
<211> 100
<212> DNA
<213> Escherichia coli K-12 MG1655
<220> dppd b3542
<222> 885-984
<223> dppd b3542 U00096 complement(3700494..3701477)
<313> 241: 958-977
<400> 241
CAGATGTCGC GCTGAAGAAC CGGCGCTGAA TATGCTCGCT GACGGGCGTC 50
AGTCCAAATG CCATTACCCA CTTGATGATG CCGGGAGGCC GACACTATGA 100
<210> 242
<211> 100
<212> DNA
<213> Escherichia coli K-12 MG1655
<220> dppd b3542
<222> 839-938
<223> dppd b3542 U00096 complement(3700494..3701477)
<313> 242: 868-887
<400> 242
GCCCGAACGG CTGCCTGCTT AACCCGCGCT GCCCCTATGC CACTGACAGA 50
TGTCGCGCTG AAGAACCGGC GCTGAATATG CTCGCTGACG GGCGTCAGTC 100
<210> 243
<211> 100
<212> DNA
<213> Escherichia coli K-12 MG1655
<220> dppd b3542
<222> 787-886
<223> dppd b3542 U00096 complement(3700494..3701477)
<313> 243: 831-850
<400> 243
GACAAAGAAC GTCTGGCGTC GTTGCCAGGT GTCGTTCCCG GCAAGTACGA 50
CCGCCCGAAC GGCTGCCTGC TTAACCCGCG CTGCCCCTAT GCCACTGACA 100
<210> 244
<211> 100
<212> DNA
<213> Escherichia coli K-12 MG1655
<220> yhag b3129
<222> 1461-1560
<223> yhag b3129 U00096 3272923..3274494
<313> 244: 1520-1539
<400> 244
GAAGTTGTTC CACTTTATTC TCGACGTCGC CAGCGGGAAG AAGAAAACCT 50
TCTCGGATCA ATGGGGGCTG CATAACCAGC TGGCGGTGTT TAACCCGGCA 100
<210> 245
<211> 100
<212> DNA
<213> Escherichia coli K-12 MG1655
<220> yhag b3129
<222> 1389-1488
<223> yhag b3129 U00096 3272923..3274494
<313> 245: 1451-1470
<400> 245
CTGGTTTGAT TTAATGGATA TTAATGCGGG CACCATCGCT ACCGGCGAAG 50

AAACTATTGA AGAGGTGGGC TGGAAGTTGT TCCACTTTAT TCTCGACGTC 100
<210> 246
<211> 100
<212> DNA
<213> Escherichia coli K-12 MG1655
<220> yhag b3129
<222> 1363-1462
<223> yhag b3129 U00096 3272923..3274494
<313> 246: 1388-1407
<400> 246
GCAACCCGCA CCGAGCTGGC GAACCGCTGG TTTGATTTAA TGGATATTAA 50
TGCGGGCACC ATCGCTACCG GCGAAGAAAC TATTGAAGAG GTGGGCTGGA 100
<210> 247
<211> 100
<212> DNA
<213> Escherichia coli K-12 MG1655
<220> yhah b3104
<222> 267-366
<223> yhah b3104 U00096 3249944..3250309
<313> 247: 338-357
<400> 247
AATCCCGTTT ATCGGCTGGC TTATTATCAT CGTCTTTAAC TGCCAGGCGG 50
GTACGCCAGG CGAAAACCGC TTTGGACCAG ATCCAAAACT GGAACCTTAA 100
<210> 248
<211> 100
<212> DNA
<213> Escherichia coli K-12 MG1655
<220> yhah b3104
<222> 253-352
<223> yhah b3104 U00096 3249944..3250309
<313> 248: 288-307
<400> 248
GCACTGCTAT TCTTAATCCC GTTTATCGGC TGGCTTATTA TCATCGTCTT 50
TAACTGCCAG GCGGGTACGC CAGGCGAAAA CCGCTTTGGA CCAGATCCAA 100
<210> 249
<211> 100
<212> DNA
<213> Escherichia coli K-12 MG1655
<220> yhah b3104
<222> 202-301
<223> yhah b3104 U00096 3249944..3250309
<313> 249: 263-282
<400> 249
TGGGCGGTTC AGTTCCGCCG CCTGCACGAC ACCGACCGCT CGGCGTGGTG 50
GGCACTGCTA TTCTTAATCC CGTTTATCGG CTGGCTTATT ATCATCGTCT 100
<210> 250
<211> 100
<212> DNA
<213> Escherichia coli K-12 MG1655
<220> dppf b3541
<222> 906-1005
<223> dppf b3541 U00096 complement(3699493..3700497)
<313> 250: 947-966
<400> 250
CTTCGGCCCC TGCACCCAGT TGCAGCCGCA GCTAAAAGAC TACGGCGGTC 50
AACTGGTAGC TTGTTTTGCT GTTGATCAGG ATGAAAATCC GCAGCGTTAA 100
<210> 251
<211> 100
<212> DNA
<213> Escherichia coli K-12 MG1655
<220> dppf b3541
<222> 814-913
<223> dppf b3541 U00096 complement(3699493..3700497)
<313> 251: 876-895
<400> 251
GATCGCCGCG AGCGCATCAA GCTCAGCGGT GAACTACCAA GCCCACTGAA 50
TCCACCGCCG GGTTGCGCCT TCAACGCCCG CTGTCGTCGG CGCTTCGGCC 100
<210> 252
<211> 100
<212> DNA
<213> Escherichia coli K-12 MG1655

<220> dppf b3541
<222> 782-881
<223> dppf b3541 U00096 complement(3699493..3700497)
<313> 252: 837-856
<400> 252
TACTTTCCGC GACGCCGCGC CTGAACCCGG ACGATCGCCG CGAGCGCATC 50
AAGCTCAGCG GTGAACTACC AAGCCCACTG AATCCACCGC CGGGTTGCGC 100
<210> 253
<211> 100
<212> DNA
<213> Escherichia coli K-12 MG1655
<220> yhai b3105
<222> 258-357
<223> yhai b3105 U00096 3250551..3250907
<313> 253: 321-340
<400> 253
TCCGTTTATT GGCTGGCTCG TGCTTCTGGT TTTTTTCTGC ACAGAAGGTA 50
CTTCTGGCAG CAATCGTTAC GGAAACGATC CGAAGTTTGG TTCAAATTAA 100
<210> 254
<211> 100
<212> DNA
<213> Escherichia coli K-12 MG1655
<220> yhai b3105
<222> 149-248
<223> yhai b3105 U00096 3250551..3250907
<313> 254: 207-226
<400> 254
ATCTGTCTAT GCTTTATTTG CTGGCAACCT TCCTTCCCGT CCTTGCGCTT 50
GCTATCCGTC GCTTACACGA TACCGACCGT TCAGGTGCCT GGGCCTTGCT 100
<210> 255
<211> 100
<212> DNA
<213> Escherichia coli K-12 MG1655
<220> yhai b3105
<222> 34-133
<223> yhai b3105 U00096 3250551..3250907
<313> 255: 66-85
<400> 255
GTTGGTTTCT CTGGTCGCGC ACGTCGTAAA GAGTACTGGA TGTTTACTCT 50
GATTAACGCC ATCGTCGGCG CTATTATCAA TGTCATTCAA TTGATTTTAG 100
<210> 256
<211> 100
<212> DNA
<213> Escherichia coli K-12 MG1655
<220> fsr b0480
<222> 1122-1221
<223> fsr b0480 U00096 complement(502700..503920)
<313> 256: 1196-1215
<400> 256
CGATCACACC AGCATCGAGT TAGTCTATAA AATCTGTGCT TTCCTGCCAC 50
TATTGGGGAT GTTGACCATA TTCCTGCCTG ATAACCGGCA TAAAGACTGA 100
<210> 257
<211> 100
<212> DNA
<213> Escherichia coli K-12 MG1655
<220> fsr b0480
<222> 1092-1191
<223> fsr b0480 U00096 complement(502700..503920)
<313> 257: 1114-1133
<400> 257
TCTGGGAGCG GCAGTTCTGG GGCTTATCGC CGATCACACC AGCATCGAGT 50
TAGTCTATAA AATCTGTGCT TTCCTGCCAC TATTGGGGAT GTTGACCATA 100
<210> 258
<211> 100
<212> DNA
<213> Escherichia coli K-12 MG1655
<220> fsr b0480
<222> 1011-1110
<223> fsr b0480 U00096 complement(502700..503920)
<313> 258: 1059-1078
<400> 258

GGTCTACGCT CAGGAGCTGC TTCCAGGACG TATCGGTATG GTTTCTGGAC 50
TCTTTTTCGG TTTTGCTTTT GGCATGGGAG GTCTGGGAGC GGCAGTTCTG 100
<210> 259
<211> 100
<212> DNA
<213> Escherichia coli K-12 MG1655
<220> yhaj b3106
<222> 758-857
<223> yhaj b3106 U00096 complement(3250958..3251854)
<313> 259: 805-824
<400> 259
TTGCGGAAGG GCGGTTGCGT GTCGTCAGCC CGGAATCGAC CAGCGAGATC 50
GATATTATTA TGGCCTGGCG TCGTGACAGT ATGGGGGAAG CGAAATCCTG 100
<210> 260
<211> 100
<212> DNA
<213> Escherichia coli K-12 MG1655
<220> yhaj b3106
<222> 693-792
<223> yhaj b3106 U00096 complement(3250958..3251854)
<313> 260: 733-752
<400> 260
ACGTCAGGCA TTACTGGCGG GGCTTGGCGT GGCGACGATG CCGTATCCTA 50
TGGTCGAAAA AGATATTGCG GAAGGGCGGT TGCGTGTCGT CAGCCCGGAA 100
<210> 261
<211> 100
<212> DNA
<213> Escherichia coli K-12 MG1655
<220> yhaj b3106
<222> 641-740
<223> yhaj b3106 U00096 complement(3250958..3251854)
<313> 261: 670-689
<400> 261
TACAGCTGCT GGACAAACAG CCGCGCTTAA CGGTGAGCAC GATTGAAGAT 50
AAACGTCAGG CATTACTGGC GGGGCTTGGC GTGGCGACGA TGCCGTATCC 100
<210> 262
<211> 100
<212> DNA
<213> Escherichia coli K-12 MG1655
<220> yhak b3107
<222> 603-702
<223> yhak b3107 U00096 3251959..3252660
<313> 262: 642-661
<400> 262
GCTGACCTGC GGTGATGGGG CGTTTATTCG TGACGAGGCT AACATTACGC 50
TGGTTGCCGA TTCCCCACTG CGCGCTTTGC TGATAGATTT GCCTGTCTAG 100
<210> 263
<211> 100
<212> DNA
<213> Escherichia coli K-12 MG1655
<220> yhak b3107
<222> 467-566
<223> yhak b3107 U00096 3251959..3252660
<313> 263: 505-524
<400> 263
GCCAGCAAGT GTGGCTGCAC CATATCGTGC TCGACAAAGG CGAAAGTGCG 50
AATTTCCAGT TGCATGGGCC ACGCGCGTAT TTGCAATCGA TTCACGGGAA 100
<210> 264
<211> 100
<212> DNA
<213> Escherichia coli K-12 MG1655
<220> yhak b3107
<222> 393-492
<223> yhak b3107 U00096 3251959..3252660
<313> 264: 449-468
<400> 264
AAAGCTGGCG CTTAATATGG GCAAGCAGCA ATTAATCGCC TCGCCAGAGG 50
GTGCGATGGG AAGCCTGCAA TTACGCCAGC AAGTGTGGCT GCACCATATC 100
<210> 265
<211> 100
<212> DNA

<213> Escherichia coli K-12 MG1655
<220> yhal b3108
<222> 60-159
<223> yhal b3108 U00096 3252677..3252847
<313> 265: 115-134
<400> 265
AGAACCTGCT CGCACCGCCA AAAATTTTGG CTATGAAGAG ATGTTGAGCG 50
AGCTGGAAGC TATCGTCGCG GATGCTGAAA CGCGTTTAGC CGAGGATGAA 100
<210> 266
<211> 100
<212> DNA
<213> Escherichia coli K-12 MG1655
<220> yhal b3108
<222> 72-171
<223> yhal b3108 U00096 3252677..3252847
<313> 266: 101-120
<400> 266
CACCGCCAAA AATTTTGGCT ATGAAGAGAT GTTGAGCGAG CTGGAAGCTA 50
TCGTCGCGGA TGCTGAAACG CGTTTAGCCG AGGATGAAGC TACCGCGTAA 100
<210> 267
<211> 100
<212> DNA
<213> Escherichia coli K-12 MG1655
<220> yhal b3108
<222> 23-122
<223> yhal b3108 U00096 3252677..3252847
<313> 267: 40-59
<400> 267
CCAAAAAGCG CCAGCCGGTG AAGCCCGTGG TGGCGAAAGA ACCTGCTCGC 50
ACCGCCAAAA ATTTTGGCTA TGAAGAGATG TTGAGCGAGC TGGAAGCTAT 100
<210> 268
<211> 100
<212> DNA
<213> Escherichia coli K-12 MG1655
<220> yham b3109
<222> 468-567
<223> yham b3109 U00096 complement(3252981..3253547)
<313> 268: 542-561
<400> 268
TGTTGAGCAG TCGATTGCCA ACCTGTGTGC GTTAGCAAGC CATTCGATGC 50
AGCAAACGGA TCGGCAGATT ATCGAGATTA TGGCGAGCAA GGCCAGATAA 100
<210> 269
<211> 100
<212> DNA
<213> Escherichia coli K-12 MG1655
<220> yham b3109
<222> 458-557
<223> yham b3109 U00096 complement(3252981..3253547)
<313> 269: 498-517
<400> 269
TGGCGCATGA TGTTGAGCAG TCGATTGCCA ACCTGTGTGC GTTAGCAAGC 50
CATTCGATGC AGCAAACGGA TCGGCAGATT ATCGAGATTA TGGCGAGCAA 100
<210> 270
<211> 100
<212> DNA
<213> Escherichia coli K-12 MG1655
<220> yham b3109
<222> 411-510
<223> yham b3109 U00096 complement(3252981..3253547)
<313> 270: 444-463
<400> 270
GTTAATGGCG CTGGATGATA CCGCCGTGAC CGGCAATGAA GGGATTGTGG 50
CGCATGATGT TGAGCAGTCG ATTGCCAACC TGTGTGCGTT AGCAAGCCAT 100
<210> 271
<211> 100
<212> DNA
<213> Escherichia coli K-12 MG1655
<220> yhan b3110
<222> 465-564
<223> yhan b3110 U00096 complement(3253729..3254292)
<313> 271: 543-562

<400> 271
CGAGACGCAC GATGGTGTGG TGTTTACCCA GCAGGCGTGT GTGGCAGAGG 50
GCGAGCAAGA GTCTCCGCTG ACGGTGCTTT CCAGAACCGA CGCTGGCTGA 100
<210> 272
<211> 100
<212> DNA
<213> Escherichia coli K-12 MG1655
<220> yhan b3110
<222> 449-548
<223> yhan b3110 U00096 complement(3253729..3254292)
<313> 272: 493-512
<400> 272
CCAACATTGT GCATATCGAG ACGCACGATG GTGTGGTGTT TACCCAGCAG 50
GCGTGTGTGG CAGAGGGCGA GCAAGAGTCT CCGCTGACGG TGCTTTCCAG 100
<210> 273
<211> 100
<212> DNA
<213> Escherichia coli K-12 MG1655
<220> yhan b3110
<222> 327-426
<223> yhan b3110 U00096 complement(3253729..3254292)
<313> 273: 380-399
<400> 273
ACTGCTGGCG GCGGGGAAAG TCTCCGTTAA GATCCAGGAA CCTTGCGATG 50
AAATCCTCTT CTCACGCGCC AAAGTCTGGA ACGGTGAGAA GTGGGCGTGT 100
<210> 274
<211> 100
<212> DNA
<213> Escherichia coli K-12 MG1655
<220> yhao b3111
<222> 1179-1278
<223> yhao b3111 U00096 complement(3254320..3255597)
<313> 274: 1240-1259
<400> 274
CAAAGTACCA GCATTGCACA AATACAAAGG GATGTCTCTG TACCTGATTA 50
TCGTCACTGG TTTGTTGCTG TGTGTTTCTC CGTTCCTGGC ATTTTCTTGA 100
<210> 275
<211> 100
<212> DNA
<213> Escherichia coli K-12 MG1655
<220> yhao b3111
<222> 1075-1174
<223> yhao b3111 U00096 complement(3254320..3255597)
<313> 275: 1128-1147
<400> 275
GCCTGGAGCG CCATCGTACT GAACGCACCG GTGTTGAGCT TCACCTCTAT 50
CTGTAGCCCG ATTTTCGGCA TGGTAGGGTG CCTGATCCCG GCGTGGCTGG 100
<210> 276
<211> 100
<212> DNA
<213> Escherichia coli K-12 MG1655
<220> yhao b3111
<222> 1045-1144
<223> yhao b3111 U00096 complement(3254320..3255597)
<313> 276: 1073-1092
<400> 276
CGCGGCATCA TGATTTTCGC CATTTTGCTG GCCTGGAGCG CCATCGTACT 50
GAACGCACCG GTGTTGAGCT TCACCTCTAT CTGTAGCCCG ATTTTCGGCA 100
<210> 277
<211> 100
<212> DNA
<213> Escherichia coli K-12 MG1655
<220> yhap b3112
<222> 716-815
<223> yhap b3112 U00096 complement(3255928..3256755)
<313> 277: 764-783
<400> 277
GTGTTTCACT CGATAAAGTG ATCGAGACGA TGTATGAAAC CGGCAAAGAT 50
ATGAACGATA AATACCGCGA AACATCACGC GGAGGACTGG CCATTAAAGT 100
<210> 278
<211> 100

<212> DNA
<213> Escherichia coli K-12 MG1655
<220> yhap b3112
<222> 675-774
<223> yhap b3112 U00096 complement(3255928..3256755)
<313> 278: 709-728
<400> 278
AAACGCCGCG CGGATGGCGA TGCGCCGCAC CTCGGCACCG CGTGTTTCAC  50
TCGATAAAGT GATCGAGACG ATGTATGAAA CCGGCAAAGA TATGAACGAT  100
<210> 279
<211> 100
<212> DNA
<213> Escherichia coli K-12 MG1655
<220> yhap b3112
<222> 586-685
<223> yhap b3112 U00096 complement(3255928..3256755)
<313> 279: 620-639
<400> 279
CATAACCTTG GGCTGACCTG CGATCCGGTT GCCGGACAGG TACAAATCCC  50
GTGCATTGAA CGTAATGCCA TTAATGCCGT GAAAGCAGTA AACGCCGCGC  100
<210> 280
<211> 100
<212> DNA
<213> Escherichia coli K-12 MG1655
<220> yhaq b3113
<222> 324-423
<223> yhaq b3113 U00096 complement(3256875..3257297)
<313> 280: 394-413
<400> 280
TTTTCCTGTA GCAAAGAACA TTATCTTCCA TCCCGAAATG TTGCCTCGCC  50
ATGAGAACGG AATGCGGATC ACTGCCTGGA AGGACAGGAA GAGCTATTAA  100
<210> 281
<211> 100
<212> DNA
<213> Escherichia coli K-12 MG1655
<220> yhaq b3113
<222> 314-413
<223> yhaq b3113 U00096 complement(3256875..3257297)
<313> 281: 357-376
<400> 281
ATATTGTTGA TTTTCCTGTA GCAAAGAACA TTATCTTCCA TCCCGAAATG  50
TTGCCTCGCC ATGAGAACGG AATGCGGATC ACTGCCTGGA AGGACAGGAA  100
<210> 282
<211> 100
<212> DNA
<213> Escherichia coli K-12 MG1655
<220> yhaq b3113
<222> 231-330
<223> yhaq b3113 U00096 complement(3256875..3257297)
<313> 282: 260-279
<400> 282
GCAGGATGTT GTCATTGATG AGATCCCTGC ATTTATAGAG TTAGTAACGC  50
GCAGCGGGCG GCTGCCAGTG GCATCTGGTG CGCATATTGT TGATTTTCCT  100
<210> 283
<211> 100
<212> DNA
<213> Escherichia coli K-12 MG1655
<220> yhar b3114
<222> 354-453
<223> yhar b3114 U00096 complement(3257363..3257815)
<313> 283: 420-439
<400> 283
GCATCAGGCG ACCTATCCGA CCCGGAGCTG TGTGCAGGTC GCGCGTTTGC  50
CGAAAGATGT GAAGCTGGAA ATTGAAGCCA TCGCAGTACG TAGTGCGTAA  100
<210> 284
<211> 100
<212> DNA
<213> Escherichia coli K-12 MG1655
<220> yhar b3114
<222> 320-419
<223> yhar b3114 U00096 complement(3257363..3257815)

<313> 284: 365-384
<400> 284
CCATCAACGA AGTCTATAAG CAGTTCTTCG ATGAGCATCA GGCGACCTAT 50
CCGACCCGGA GCTGTGTGCA GGTCGCGCGT TTGCCGAAAG ATGTGAAGCT 100
<210> 285
<211> 100
<212> DNA
<213> Escherichia coli K-12 MG1655
<220> yhar b3114
<222> 249-348
<223> yhar b3114 U00096 complement(3257363..3257815)
<313> 285: 312-331
<400> 285
TGCTGCCGGG CTGAGCGTGG GCGATATCAT CAAGATGACC GTGTTTATCA 50
CCGATCTGAA TGATTTTGCC ACCATCAACG AAGTCTATAA GCAGTTCTTC 100
<210> 286
<211> 100
<212> DNA
<213> Escherichia coli K-12 MG1655
<220> b4285 b4285
<222> 498-597
<223> b4285 b4285 U00096 4506526..4507122
<313> 286: 573-592
<400> 286
TAAGCCCGTC GGGGTGATGT TCCAAGCGAT CAAGGCAGTC ATTATACGAG 50
CAGGCAGTTC CGGCAGTTAC TGTGGCGATA CCGGATCAGG CAGAGTATGA 100
<210> 287
<211> 100
<212> DNA
<213> Escherichia coli K-12 MG1655
<220> b4285 b4285
<222> 470-569
<223> b4285 b4285 U00096 4506526..4507122
<313> 287: 531-550
<400> 287
CACTGGAAAT GGCATGGGAA ACCCGTGGTA AGCCCGTCGG GGTGATGTTC 50
CAAGCGATCA AGGCAGTCAT TATACGAGCA GGCAGTTCCG GCAGTTACTG 100
<210> 288
<211> 100
<212> DNA
<213> Escherichia coli K-12 MG1655
<220> b4285 b4285
<222> 437-536
<223> b4285 b4285 U00096 4506526..4507122
<313> 288: 472-491
<400> 288
CGTTCTCGCC GGACAGCAGG CTTACCATGA AAGCACTGGA AATGGCATGG 50
GAAACCCGTG GTAAGCCCGT CGGGGTGATG TTCCAAGCGA TCAAGGCAGT 100
<210> 289
<211> 100
<212> DNA
<213> Escherichia coli K-12 MG1655
<220> b4286 b4287
<222> 314-413
<223> b4286 b4287 U00096 4507288..4507701
<313> 289: 360-379
<400> 289
CCCGTCTGCC TTCACTTTAT GCCAGCTTCG GTAAGCTGGG TGAGACACCG 50
ACTCATGAAG ATATCATTGA TAACAATCGA TCCATCAACT GGCCTGTATA 100
<210> 290
<211> 100
<212> DNA
<213> Escherichia coli K-12 MG1655
<220> b4286 b4287
<222> 220-319
<223> b4286 b4287 U00096 4507288..4507701
<313> 290: 282-301
<400> 290
CGTCAGCAGC CGTGCACACC ATCCTATTGT TGTGACTGGG AGGCTGCCAC 50
CATGCTTCGC GCTTTCCCTC TGGCGAAAAA GGGAGAAGGA CGGGCCCGTC 100
<210> 291

<211> 100
<212> DNA
<213> Escherichia coli K-12 MG1655
<220> b4286 b4287
<222> 163-262
<223> b4286 b4287 U00096 4507288..4507701
<313> 291: 185-204
<400> 291
CTGGTTAATG ATACCGGCTT TGACCCCGTA TTTTCCGGCT CTATCGCTGA 50
ATCATGGCGT CAGCAGCCGT GCACACCATC CTATTGTTGT GACTGGGAGG 100
<210> 292
<211> 100
<212> DNA
<213> Escherichia coli K-12 MG1655
<220> yhau b3128
<222> 1236-1335
<223> yhau b3128 U00096 complement(3271214..3272548)
<313> 292: 1282-1301
<400> 292
CAATGCAGCA CTGGTTTTCG TGGGATGTTC AGCGCTGATG GCGATGGTCT 50
GCTACCTCTT CGTAGTTGGC GACATTAAAC GTATGGAATT GCAGAAATAA 100
<210> 293
<211> 100
<212> DNA
<213> Escherichia coli K-12 MG1655
<220> yhau b3128
<222> 1190-1289
<223> yhau b3128 U00096 complement(3271214..3272548)
<313> 293: 1234-1253
<400> 293
TCACTCCACT GGTGATTGGC TACCTGGTAA GTGAACTGCA CTCCTTCAAT 50
GCAGCACTGG TTTTCGTGGG ATGTTCAGCG CTGATGGCGA TGGTCTGCTA 100
<210> 294
<211> 100
<212> DNA
<213> Escherichia coli K-12 MG1655
<220> yhau b3128
<222> 1093-1192
<223> yhau b3128 U00096 complement(3271214..3272548)
<313> 294: 1157-1176
<400> 294
GCGCTGGGCT GGCCGGTGAT TTCTGACACC GCGCCGAAAG AGATTGTTGG 50
CCTCTGCGGC GGCGTCTTTA ACGTCTTTGG CAATGTTGCC TCCATTGTCA 100
<210> 295
<211> 100
<212> DNA
<213> Escherichia coli K-12 MG1655
<220> yhav b3131
<222> 366-465
<223> yhav b3131 U00096 3274978..3275442
<313> 295: 427-446
<400> 295
AAAAACAGAT GCCTATACCG TATTCAGCAA AATGTTAAAA AGAGGACATC 50
CTCCTGCCGA CTGGGAAACC CTCACCCGAG AAACAGAAGA AACCCATTGA 100
<210> 296
<211> 100
<212> DNA
<213> Escherichia coli K-12 MG1655
<220> yhav b3131
<222> 226-325
<223> yhav b3131 U00096 3274978..3275442
<313> 296: 255-274
<400> 296
GGCTCTGGGA AAAATAAAGA CTGGTCACGG GTAAAATTTG GTGCTGGTCG 50
TTATCGTCTC TTCTTTCGTT ATAGTGAAAA AGAGAAAGTC ATCATTCTGG 100
<210> 297
<211> 100
<212> DNA
<213> Escherichia coli K-12 MG1655
<220> yhav b3131
<222> 187-286

<223> yhav b3131 U00096 3274978..3275442
<313> 297: 229-248
<400> 297
AATCCATCAT CACCGGCATT CCGTCATGGC AAGTCGTTAG GCTCTGGGAA  50
AAATAAAGAC TGGTCACGGG TAAAATTTGG TGCTGGTCGT TATCGTCTCT  100
<210> 298
<211> 100
<212> DNA
<213> Escherichia coli K-12 MG1655
<220> uup b0950
<222> 1809-1908
<223> uup b0950 U00096 1009187..1011094
<313> 298: 1880-1899
<400> 298
GACGCAAAAA GTGCTTGCTG ATATGGCTGC TGCAGAGCAG GAGCTGGAGC  50
AAGCCTTTGA ACGCTGGGAG TATCTTGAAG CGTTAAAAAA TGGTGGCTGA  100
<210> 299
<211> 100
<212> DNA
<213> Escherichia coli K-12 MG1655
<220> uup b0950
<222> 1733-1832
<223> uup b0950 U00096 1009187..1011094
<313> 299: 1790-1809
<400> 299
AAGATCTGGA GGCGAAGCTG GAAGCCCTAC AGACGCAAGT GGCGGATGCT  50
TCCTTCTTCA GTCAGCCGCA TGAGCAGACG CAAAAAGTGC TTGCTGATAT  100
<210> 300
<211> 100
<212> DNA
<213> Escherichia coli K-12 MG1655
<220> uup b0950
<222> 1720-1819
<223> uup b0950 U00096 1009187..1011094
<313> 300: 1755-1774
<400> 300
CCGCAATTGC TCGAAGATCT GGAGGCGAAG CTGGAAGCCC TACAGACGCA  50
AGTGGCGGAT GCTTCCTTCT TCAGTCAGCC GCATGAGCAG ACGCAAAAAG  100
<210> 301
<211> 100
<212> DNA
<213> Escherichia coli K-12 MG1655
<220> ndh b1110
<222> 1206-1305
<223> ndh b1110 U00096 1165308..1166612
<313> 301: 1269-1288
<400> 301
AATGCATCAG ATTGCGCTGC ATGGTTACTT TAAAACCGGA TTAATGATGC  50
TGGTGGGGAG TATTAACCGC GTTATCCGTC CGCGTTTGAA GTTGCATTAA  100
<210> 302
<211> 100
<212> DNA
<213> Escherichia coli K-12 MG1655
<220> ndh b1110
<222> 1202-1301
<223> ndh b1110 U00096 1165308..1166612
<313> 302: 1237-1256
<400> 302
ACCGAATGCA TCAGATTGCG CTGCATGGTT ACTTTAAAAC CGGATTAATG  50
ATGCTGGTGG GGAGTATTAA CCGCGTTATC CGTCCGCGTT TGAAGTTGCA  100
<210> 303
<211> 100
<212> DNA
<213> Escherichia coli K-12 MG1655
<220> ndh b1110
<222> 1061-1160
<223> ndh b1110 U00096 1165308..1166612
<313> 303: 1097-1116
<400> 303
TGAAAAATTA TCAGTATAAA GATCATGGTT CGCTGGTATC GCTGTCGAAC  50
TTCTCCACCG TCGGTAGCCT GATGGGTAAC CTGACGCGCG GCTCAATGAT  100

<210> 304
<211> 100
<212> DNA
<213> Escherichia coli K-12 MG1655
<220> ndk b2519
<222> 333-432
<223> ndk b2519 U00096 complement(2642453..2642884)
<313> 304: 386-405
<400> 304
GACCGAAAAC GGTACCCACG GTTCTGATTC CGTCGAATCT GCCGCTCGCG 50
AAATCGCTTA TTTCTTTGGC GAAGGCGAAG TGTGCCCGCG CACCCGTTAA 100
<210> 305
<211> 100
<212> DNA
<213> Escherichia coli K-12 MG1655
<220> ndk b2519
<222> 219-318
<223> ndk b2519 U00096 complement(2642453..2642884)
<313> 305: 230-249
<400> 305
GGTTTCCGTG CTGGAAGGTG AAAACGCCGT TCAGCGTCAC CGCGATCTGC 50
TGGGCGCGAC CAATCCGGCA AACGCACTGG CTGGTACTCT GCGCGCTGAT 100
<210> 306
<211> 100
<212> DNA
<213> Escherichia coli K-12 MG1655
<220> ndk b2519
<222> 115-214
<223> ndk b2519 U00096 complement(2642453..2642884)
<313> 306: 164-183
<400> 306
ATGCTGCACC TGACCGTTGA ACAGGCACGT GGCTTTTATG CTGAACACGA 50
TGGAAAACCG TTCTTTGATG GTCTGGTTGA ATTCATGACC TCTGGCCCGA 100
<210> 307
<211> 100
<212> DNA
<213> Escherichia coli K-12 MG1655
<220> mela b4120
<222> 1236-1335
<223> mela b4120 U00096 4339489..4340844
<313> 307: 1263-1282
<400> 307
CCACGCCGCG ATGATGGACC CGCATACTGC CGCCGTGCTG GGCATTGACG 50
AAATATATGC TCTTGTTGAC GACCTGATTG CCGCCCACGG CGACTGGCTG 100
<210> 308
<211> 100
<212> DNA
<213> Escherichia coli K-12 MG1655
<220> mela b4120
<222> 1189-1288
<223> mela b4120 U00096 4339489..4340844
<313> 308: 1240-1259
<400> 308
ACGCTGCTGA CCGAAGCTAT TCTTACGGAA AATCGCGACC GTGTTTACCA 50
CGCCGCGATG ATGGACCCGC ATACTGCCGC CGTGCTGGGC ATTGACGAAA 100
<210> 309
<211> 100
<212> DNA
<213> Escherichia coli K-12 MG1655
<220> mela b4120
<222> 1154-1253
<223> mela b4120 U00096 4339489..4340844
<313> 309: 1187-1206
<400> 309
TGGCCGCCCT GATGCAAACC AACATCAACG TACAGACGCT GCTGACCGAA 50
GCTATTCTTA CGGAAAATCG CGACCGTGTT TACCACGCCG CGATGATGGA 100
<210> 310
<211> 100
<212> DNA
<213> Escherichia coli K-12 MG1655
<220> melb b4121

<222> 1305-1404
<223> melb b4121 U00096 4340959..4342368
<313> 310: 1347-1366
<400> 310
GCTGCGCAGG ATCCAGATCC ATCTGCTGGA TAAATATCGC AAAGTACCGC 50
CCGAGCCTGT TCATGCTGAT ATTCCGGTCG GTGCAGTGAG TGATGTGAAA 100
<210> 311
<211> 100
<212> DNA
<213> Escherichia coli K-12 MG1655
<220> melb b4121
<222> 1247-1346
<223> melb b4121 U00096 4340959..4342368
<313> 311: 1284-1303
<400> 311
TGTTTTTCAT GGTAACGCTG ATTCTCTACT TCCGTTTCTA TCGCCTCAAT 50
GGTGACACGC TGCGCAGGAT CCAGATCCAT CTGCTGGATA AATATCGCAA 100
<210> 312
<211> 100
<212> DNA
<213> Escherichia coli K-12 MG1655
<220> melb b4121
<222> 1146-1245
<223> melb b4121 U00096 4340959..4342368
<313> 312: 1184-1203
<400> 312
CATTGCGGTT GTGTTAGGGA TGATTGGCTA TGTACCGAAT GTTGAACAGT 50
CTACGCAAGC CCTATTAGGT ATGCAGTTTA TTATGATTGC TCTACCAACT 100
<210> 313
<211> 100
<212> DNA
<213> Escherichia coli K-12 MG1655
<220> yhbc b3171
<222> 360-459
<223> yhbc b3171 U00096 complement(3315195..3315653)
<313> 313: 436-455
<400> 313
AGCGGTAGAC GGTGAAATGA TCACAGTTAC CGTCGAAGGT AAAGATGAAG 50
TGTTCGCGCT GAGTAATATC CAGAAGGCGA ACCTGGTTCC CCACTTTTAA 100
<210> 314
<211> 100
<212> DNA
<213> Escherichia coli K-12 MG1655
<220> yhbc b3171
<222> 341-440
<223> yhbc b3171 U00096 complement(3315195..3315653)
<313> 314: 393-412
<400> 314
AATGGCAGGG CGTTATCAAA GCGGTAGACG GTGAAATGAT CACAGTTACC 50
GTCGAAGGTA AAGATGAAGT GTTCGCGCTG AGTAATATCC AGAAGGCGAA 100
<210> 315
<211> 100
<212> DNA
<213> Escherichia coli K-12 MG1655
<220> yhbc b3171
<222> 293-392
<223> yhbc b3171 U00096 complement(3315195..3315653)
<313> 315: 342-361
<400> 315
GAGAAGAGGT GACTCTGGTT CTCCGTATGG CGGTACAAAA CCGTCGTAAA 50
TGGCAGGGCG TTATCAAAGC GGTAGACGGT GAAATGATCA CAGTTACCGT 100
<210> 316
<211> 100
<212> DNA
<213> Escherichia coli K-12 MG1655
<220> yhbe b3185
<222> 846-945
<223> yhbe b3185 U00096 complement(3329411..3330376)
<313> 316: 885-904
<400> 316
AGGTTATCTC GGTGCGTTTG TCGTGGTTGC GGGCGCGATG TATTCCGCCA 50

TTGGTCATCG TATTTGGGGC GGATTACGTA AGCATACAAC GGTGGTATCG 100
<210> 317
<211> 100
<212> DNA
<213> Escherichia coli K-12 MG1655
<220> yhbe b3185
<222> 806-905
<223> yhbe b3185 U00096 complement(3329411..3330376)
<313> 317: 825-844
<400> 317
CCTGGCCCGA TTTCTTCGCC AGACCGATGT TAAACCTTTT AGGTTATCTC 50
GGTGCGTTTG TCGTGGTTGC GGGCGCGATG TATTCCGCCA TTGGTCATCG 100
<210> 318
<211> 100
<212> DNA
<213> Escherichia coli K-12 MG1655
<220> yhbe b3185
<222> 749-848
<223> yhbe b3185 U00096 complement(3329411..3330376)
<313> 318: 783-802
<400> 318
CGATCATCAC GCTCACCCCA CTGTTTACGC TGTTTTTTTC AGATCTTTTA 50
TCACTGGCCT GGCCCGATTT CTTCGCCAGA CCGATGTTAA ACCTTTTAGG 100
<210> 319
<211> 100
<212> DNA
<213> Escherichia coli K-12 MG1655
<220> ftn b1906
<222> 399-498
<223> ftn b1906 U00096 1986739..1987236
<313> 319: 435-454
<400> 319
GTTCAAATCG ATTATTGATA AATTAAGCCT GGCAGGCAAA AGCGGCGAAG 50
GTCTGTATTT TATCGACAAA GAACTCTCTA CCCTCGACAC ACAAAACTAA 100
<210> 320
<211> 100
<212> DNA
<213> Escherichia coli K-12 MG1655
<220> ftn b1906
<222> 266-365
<223> ftn b1906 U00096 1986739..1987236
<313> 320: 331-350
<400> 320
AAACCTATAA ACACGAACAA TTAATCACCC AGAAAATTAA CGAACTGGCT 50
CATGCTGCAA TGACCAATCA GGACTACCCA ACATTTAATT TCCTGCAATG 100
<210> 321
<211> 100
<212> DNA
<213> Escherichia coli K-12 MG1655
<220> ftn b1906
<222> 154-253
<223> ftn b1906 U00096 1986739..1987236
<313> 321: 176-195
<400> 321
ACGCATATGC AGCGTCTGTT TGATTACCTG ACTGATACCG GCAATTTACC 50
GCGTATTAAT ACCGTTGAAT CTCCGTTTGC TGAATATTCC TCACTTGATG 100
<210> 322
<211> 100
<212> DNA
<213> Escherichia coli K-12 MG1655
<220> yhbg b3202
<222> 609-708
<223> yhbg b3202 U00096 3341585..3342310
<313> 322: 661-680
<400> 322
TTGTGAACGC GCTTATATCG TCAGTCAGGG GCATTTGATC GCCCACGGCA 50
CGCCTACAGA AATCTTACAA GACGAACACG TTAAGCGTGT ATACCTTGGG 100
<210> 323
<211> 100
<212> DNA
<213> Escherichia coli K-12 MG1655

<220> yhbg b3202
<222> 572-671
<223> yhbg b3202 U00096 3341585..3342310
<313> 323: 627-646
<400> 323
TGATCACTGA CCACAACGTG CGTGAAACAC TGGCGGTTTG TGAACGCGCT 50
TATATCGTCA GTCAGGGGCA TTTGATCGCC CACGGCACGC CTACAGAAAT 100
<210> 324
<211> 100
<212> DNA
<213> Escherichia coli K-12 MG1655
<220> yhbg b3202
<222> 485-584
<223> yhbg b3202 U00096 3341585..3342310
<313> 324: 528-547
<400> 324
ACGAACCGTT TGCCGGGGTT GACCCGATCT CGGTTATCGA CATTAAACGC 50
ATCATTGAGC ACCTGCGCGA CAGCGGCCTG GGCGTGCTGA TCACTGACCA 100
<210> 325
<211> 100
<212> DNA
<213> Escherichia coli K-12 MG1655
<220> yhbh b3204
<222> 141-240
<223> yhbh b3204 U00096 3343814..3344101
<313> 325: 191-210
<400> 325
CCACACCTCA GATGCAACAC TGCATGTAAA CGGTGGTGAA ATTCATGCCA 50
GCGCGGAAGG TCAGGACATG TACGCTGCCA TTGATGGCTT AATTGATAAG 100
<210> 326
<211> 100
<212> DNA
<213> Escherichia coli K-12 MG1655
<220> yhbh b3204
<222> 80-179
<223> yhbh b3204 U00096 3343814..3344101
<313> 326: 130-149
<400> 326
TTGAGCAATA TTTTGACCGA ATCAACCAGG TCTATGTTGT TCTGAAAGTG 50
GAGAAAGTCA CCCACACCTC AGATGCAACA CTGCATGTAA ACGGTGGTGA 100
<210> 327
<211> 100
<212> DNA
<213> Escherichia coli K-12 MG1655
<220> yhbh b3204
<222> 1-100
<223> yhbh b3204 U00096 3343814..3344101
<313> 327: 19-38
<400> 327
ATGCAGCTCA ACATTACCGG AAATAACGTC GAGATCACCG AGGCACTGCG 50
CGAATTTGTT ACAGCCAAAT TTGCCAAACT TGAGCAATAT TTTGACCGAA 100
<210> 328
<211> 100
<212> DNA
<213> Escherichia coli K-12 MG1655
<220> yhbj b3206
<222> 750-849
<223> yhbj b3206 U00096 3344756..3345610
<313> 328: 779-798
<400> 328
GAAGCACCGT TCGGTGTATA TTGCAGAGCA ACTGGCAGAC TACTTCCGCT 50
CGCGCGGTAA AAACGTCCAG TCACGCCATC GTACGCTGGA AAAACGTAAA 100
<210> 329
<211> 100
<212> DNA
<213> Escherichia coli K-12 MG1655
<220> yhbj b3206
<222> 704-803
<223> yhbj b3206 U00096 3344756..3345610
<313> 329: 731-750
<400> 329

CCAACAACCG TAGCTACTTG ACGGTCGCCA TTGGTTGTAC CGGCGGGAAG 50
CACCGTTCGG TGTATATTGC AGAGCAACTG GCAGACTACT TCCGCTCGCG 100
<210> 330
<211> 100
<212> DNA
<213> Escherichia coli K-12 MG1655
<220> yhbj b3206
<222> 620-719
<223> yhbj b3206 U00096 3344756..3345610
<313> 330: 633-652
<400> 330
CGTTCCTCGA CCGCCACACA GAAGTACACA ATTTTATCTA CCAGACGCGA 50
AGCTATCTTG AGCTATGGTT ACCTATGCTG GAAACCAACA ACCGTAGCTA 100
<210> 331
<211> 100
<212> DNA
<213> Escherichia coli K-12 MG1655
<220> yhbl b3210
<222> 547-646
<223> yhbl b3210 U00096 complement(3347447..3348109)
<313> 331: 610-629
<400> 331
GTTGATGAAG ACAATAAGAT TGTCACCACC CCAGCATATA TGCTGGCGCA 50
GAACATTGCA GAAGCGGCGA GCGGCATTGA TAAGCTGGTT TCCCGCGTGC 100
<210> 332
<211> 100
<212> DNA
<213> Escherichia coli K-12 MG1655
<220> yhbl b3210
<222> 506-605
<223> yhbl b3210 U00096 complement(3347447..3348109)
<313> 332: 563-582
<400> 332
TGGGCGCGGA GCATGTGCCG TGTCCTGTCG ATGATATCGT GGTTGATGAA 50
GACAATAAGA TTGTCACCAC CCCAGCATAT ATGCTGGCGC AGAACATTGC 100
<210> 333
<211> 100
<212> DNA
<213> Escherichia coli K-12 MG1655
<220> yhbl b3210
<222> 461-560
<223> yhbl b3210 U00096 complement(3347447..3348109)
<313> 333: 485-504
<400> 333
TGACCATCGG TACTGATATC GATACCGCAG AAGTGCTGGA AGAGATGGGC 50
GCGGAGCATG TGCCGTGTCC TGTCGATGAT ATCGTGGTTG ATGAAGACAA 100
<210> 334
<211> 100
<212> DNA
<213> Escherichia coli K-12 MG1655
<220> yhbm b3164
<222> 786-885
<223> yhbm b3164 U00096 complement(3305681..3306565)
<313> 334: 856-875
<400> 334
CAACAACGTT CATAACTTTG TTGAGCACCG ATACGCATTG TTGGAATTAT 50
CGCTCCTGGG CCAGGACCAA GATGACCTGG CAGAATCGGA CCAGCAATAG 100
<210> 335
<211> 100
<212> DNA
<213> Escherichia coli K-12 MG1655
<220> yhbm b3164
<222> 786-885
<223> yhbm b3164 U00096 complement(3305681..3306565)
<313> 335: 815-834
<400> 335
CAACAACGTT CATAACTTTG TTGAGCACCG ATACGCATTG TTGGAATTAT 50
CGCTCCTGGG CCAGGACCAA GATGACCTGG CAGAATCGGA CCAGCAATAG 100
<210> 336
<211> 100
<212> DNA

<213> Escherichia coli K-12 MG1655
<220> yhbm b3164
<222> 650-749
<223> yhbm b3164 U00096 complement(3305681..3306565)
<313> 336: 664-683
<400> 336
AAAGGCTCAA GGCGGACGCA ACGGATAACA CCTCGCTCGC TGAGCATCTC 50
AGTGAAACCA ACTTCTATTT AGGTAAGTAC TACCTAAGTC TGGGGGATTT 100
<210> 337
<211> 100
<212> DNA
<213> Escherichia coli K-12 MG1655
<220> melr b4119
<222> 810-909
<223> melr b4119 U00096 complement(4338298..4339206)
<313> 337: 861-880
<400> 337
AAGTAGTCGT TTTTACAGCA CGTTCGGCAA ATATGTCGGC ATGTCGCCGC 50
AACAATACCG CAAACTTAGC CAACAGCGCC GCCAGACGTT TCCCGGCTAA 100
<210> 338
<211> 100
<212> DNA
<213> Escherichia coli K-12 MG1655
<220> melr b4119
<222> 757-856
<223> melr b4119 U00096 complement(4338298..4339206)
<313> 338: 816-835
<400> 338
AGCGATACCG ATAAAAGTAT TCTCGATATT GCCCTGACGG CAGGCTTTCG 50
TTCAAGTAGT CGTTTTTACA GCACGTTCGG CAAATATGTC GGCATGTCGC 100
<210> 339
<211> 100
<212> DNA
<213> Escherichia coli K-12 MG1655
<220> melr b4119
<222> 676-775
<223> melr b4119 U00096 complement(4338298..4339206)
<313> 339: 703-722
<400> 339
GGGATATTCC AGCGGGTCAT GCAATTGACG ATGAAACAGT ACATTACCGC 50
GATGCGCATC AACCACGTTC GCGCGTTACT GAGCGATACC GATAAAAGTA 100
<210> 340
<211> 100
<212> DNA
<213> Escherichia coli K-12 MG1655
<220> yhbn b3201
<222> 459-558
<223> yhbn b3201 U00096 3341021..3341578
<313> 340: 539-558
<400> 340
CAGCGACAAA GGCAAGCGCG TAACAACCGT TCTGGTGCCG TCGCAGCTGC 50
AGGACAAAAA CAACAAAGGC CAGACCCCGG CACAGAAGAA GGGTAATTAA 100
<210> 341
<211> 100
<212> DNA
<213> Escherichia coli K-12 MG1655
<220> yhbn b3201
<222> 441-540
<223> yhbn b3201 U00096 3341021..3341578
<313> 341: 457-476
<400> 341
GCAGAAAATG CAGGCTTTCA GCGACAAAGG CAAGCGCGTA ACAACCGTTC 50
TGGTGCCGTC GCAGCTGCAG GACAAAAACA ACAAAGGCCA GACCCCGGCA 100
<210> 342
<211> 100
<212> DNA
<213> Escherichia coli K-12 MG1655
<220> yhbn b3201
<222> 341-440
<223> yhbn b3201 U00096 3341021..3341578
<313> 342: 405-424

<400> 342
ACGAACTGGC AAAAGATTTT GTCGTTCTGA CGGGTAATGC TTATCTGCAG 50
CAGGTCGATA GCAACATTAA GGGCGATAAG ATCACTTACC TGGTGAAAGA 100
<210> 343
<211> 100
<212> DNA
<213> Escherichia coli K-12 MG1655
<220> b2832 b2832
<222> 615-714
<223> b2832 b2832 U00096 2968442..2969155
<313> 343: 679-698
<400> 343
CGACATCCAC GTACCGAAAG GTTACATCTA CTTCGCGATG TTCTTCTCTA 50
TTGCGGTTGA AAGCCTCAAC TTGATTCGCA ACAAAAAGAA TCCGCTCTGA 100
<210> 344
<211> 100
<212> DNA
<213> Escherichia coli K-12 MG1655
<220> b2832 b2832
<222> 567-666
<223> b2832 b2832 U00096 2968442..2969155
<313> 344: 622-641
<400> 344
GCTCTCTTTC CTGATTCTGG TGGGCTTTAC CCTGATTCTG GAAAGTTTCG 50
ACATCCACGT ACCGAAAGGT TACATCTACT TCGCGATGTT CTTCTCTATT 100
<210> 345
<211> 100
<212> DNA
<213> Escherichia coli K-12 MG1655
<220> b2832 b2832
<222> 529-628
<223> b2832 b2832 U00096 2968442..2969155
<313> 345: 573-592
<400> 345
GATTTTGTCG AACGCCATCC TTCGGTAAAA ATGCTGGCGC TCTCTTTCCT 50
GATTCTGGTG GGCTTTACCC TGATTCTGGA AAGTTTCGAC ATCCACGTAC 100
<210> 346
<211> 100
<212> DNA
<213> Escherichia coli K-12 MG1655
<220> yhbo b3154
<222> 462-561
<223> yhbo b3154 U00096 3296573..3297133
<313> 346: 523-542
<400> 346
TCAGGAAGTC GTGGTTGATA AAGATCAGCT GGTTACCAGC CGGACACCGG 50
ACGATCTGCC AGCGTTTAAC CGCGAGGCGT TACGCCTGCT CGGTGCCTGA 100
<210> 347
<211> 100
<212> DNA
<213> Escherichia coli K-12 MG1655
<220> yhbo b3154
<222> 393-492
<223> yhbo b3154 U00096 3296573..3297133
<313> 347: 448-467
<400> 347
GGGGCGCAAA CTGACCGCAG TTAAACCGAT CATTATTGAT GTTAAAAATG 50
CGGGCGCGGA ATTTTACGAT CAGGAAGTCG TGGTTGATAA AGATCAGCTG 100
<210> 348
<211> 100
<212> DNA
<213> Escherichia coli K-12 MG1655
<220> yhbo b3154
<222> 348-447
<223> yhbo b3154 U00096 3296573..3297133
<313> 348: 410-429
<400> 348
CATCTGTCAC GGCCCGCAGT TGCTGATCAG CGCCGATGTG ATTCGGGGGC 50
GCAAACTGAC CGCAGTTAAA CCGATCATTA TTGATGTTAA AAATGCGGGC 100
<210> 349
<211> 100

<212> DNA
<213> Escherichia coli K-12 MG1655
<220> b2833 b2833
<222> 120-219
<223> b2833 b2833 U00096 2969293..2969511
<313> 349: 175-194
<400> 349
TGAAATTGAT GATGATACCG GGCTGGTGAG TTATCACGAT CAGCAAGGTA 50
ACGCGATGCA AATTAACCGT GATGATGTTT CGCAAATTAT TGAACGTTAA 100
<210> 350
<211> 100
<212> DNA
<213> Escherichia coli K-12 MG1655
<220> b2833 b2833
<222> 77-176
<223> b2833 b2833 U00096 2969293..2969511
<313> 350: 140-159
<400> 350
TGGCGACCAA AGATGGCCGT ATGATTTTGA CCGATGGAAA ACCTGAAATT 50
GATGATGATA CCGGGCTGGT GAGTTATCAC GATCAGCAAG GTAACGCGAT 100
<210> 351
<211> 100
<212> DNA
<213> Escherichia coli K-12 MG1655
<220> b2833 b2833
<222> 4-103
<223> b2833 b2833 U00096 2969293..2969511
<313> 351: 39-58
<400> 351
AAAAAATGGG CAGTAATAAT TTCTGCAGTC GGACTGGCGT TTGCTGTTTC 50
CGGGTGTTCC AGTGATTACG TCATGGCGAC CAAAGATGGC CGTATGATTT 100
<210> 352
<211> 100
<212> DNA
<213> Escherichia coli K-12 MG1655
<220> yhbp b3155
<222> 345-444
<223> yhbp b3155 U00096 complement(3297113..3297556)
<313> 352: 413-432
<400> 352
GTGGGAAATC CGGCTCGATG AAATCAAATT CACCGACAAC ACGCTGGGCT 50
TTGGTAAAAA AATGATTTGG CTACGCGACT CAGGCACCGA GCAGGCGTAA 100
<210> 353
<211> 100
<212> DNA
<213> Escherichia coli K-12 MG1655
<220> yhbp b3155
<222> 345-444
<223> yhbp b3155 U00096 complement(3297113..3297556)
<313> 353: 373-392
<400> 353
GTGGGAAATC CGGCTCGATG AAATCAAATT CACCGACAAC ACGCTGGGCT 50
TTGGTAAAAA AATGATTTGG CTACGCGACT CAGGCACCGA GCAGGCGTAA 100
<210> 354
<211> 100
<212> DNA
<213> Escherichia coli K-12 MG1655
<220> yhbp b3155
<222> 283-382
<223> yhbp b3155 U00096 complement(3297113..3297556)
<313> 354: 328-347
<400> 354
GACCTCGCGC GCAAGGCGTA CAATCGTCGC TTTCCGGTTG CCAGAATGCT 50
GTCGGCCCCG GTGTGGGAAA TCCGGCTCGA TGAAATCAAA TTCACCGACA 100
<210> 355
<211> 100
<212> DNA
<213> Escherichia coli K-12 MG1655
<220> b2834 b2835
<222> 845-944
<223> b2834 b2835 U00096 2969619..2970659

<313> 355: 900-919
<400> 355
GACGTCATGG CCTGGACCCT GCTCAGATGG CGCTCGCGTT TGTACGCCGT 50
CAACCGTTTG TTGCCAGCAC TCTGCTGGGC GCAACCACGA TGGATCAGCT 100
<210> 356
<211> 100
<212> DNA
<213> Escherichia coli K-12 MG1655
<220> b2834 b2835
<222> 717-816
<223> b2834 b2835 U00096 2969619..2970659
<313> 356: 769-788
<400> 356
CACGCTGACC GGGAAATATC TCAATGGTGC AAAACCCGCT GGCGCACGTA 50
ATACGCTCTT TAGTCGGTTC ACCCGCTATA GCGGTGAGCA AACGCAAAAA 100
<210> 357
<211> 100
<212> DNA
<213> Escherichia coli K-12 MG1655
<220> b2834 b2835
<222> 669-768
<223> b2834 b2835 U00096 2969619..2970659
<313> 357: 721-740
<400> 357
CCAGTATGAA GGGGTCGAAC TGCTGGCCTA TTCGTGCCTG GGTTTCGGCA 50
CGCTGACCGG GAAATATCTC AATGGTGCAA AACCCGCTGG CGCACGTAAT 100
<210> 358
<211> 100
<212> DNA
<213> Escherichia coli K-12 MG1655
<220> yhbq b3156
<222> 204-303
<223> yhbq b3156 U00096 3297607..3297909
<313> 358: 276-295
<400> 358
GCAACTGACC AAACGGCAAA AAGAGCGTCT GGTAGCGGAA GGCGCAGGGT 50
TTGCAGAGCT GTTAAGCAGT CTGCAAACCC CGGAGATTAA AAGCGATTGA 100
<210> 359
<211> 100
<212> DNA
<213> Escherichia coli K-12 MG1655
<220> yhbq b3156
<222> 160-259
<223> yhbq b3156 U00096 3297607..3297909
<313> 359: 209-228
<400> 359
GTGGGCGATC GTTCGCTGGC GTTACGGGCG GAATATCGCG TTAAGCAACT 50
GACCAAACGG CAAAAAGAGC GTCTGGTAGC GGAAGGCGCA GGGTTTGCAG 100
<210> 360
<211> 100
<212> DNA
<213> Escherichia coli K-12 MG1655
<220> yhbq b3156
<222> 64-163
<223> yhbq b3156 U00096 3297607..3297909
<313> 360: 94-113
<400> 360
ACGGATGTCG AACGCCGCTA TCAGCAGCAC CAAAGCGGCA AAGGGGCGAA 50
AGCACTGCGC GGGAAAGGAG AACTAACGCT GGTGTTTTCC GCGCCAGTGG 100
<210> 361
<211> 100
<212> DNA
<213> Escherichia coli K-12 MG1655
<220> yhbs b3157
<222> 405-504
<223> yhbs b3157 U00096 complement(3297896..3298399)
<313> 361: 467-486
<400> 361
GGGCACTGAA AGCGCCTTCC AGGTACATCG TTTAGCGGAT GACGCGCTGA 50
ATGGCGTTAC CGGCCTGGTT GAGTATCACG AGCATTTCAA TCGCTTTTAA 100
<210> 362

<211> 100
<212> DNA
<213> Escherichia coli K-12 MG1655
<220> yhbs b3157
<222> 385-484
<223> yhbs b3157 U00096 complement(3297896..3298399)
<313> 362: 423-442
<400> 362
GATCTGCGTT GCCGCTGGCC GGGCACTGAA AGCGCCTTCC AGGTACATCG 50
TTTAGCGGAT GACGCGCTGA ATGGCGTTAC CGGCCTGGTT GAGTATCACG 100
<210> 363
<211> 100
<212> DNA
<213> Escherichia coli K-12 MG1655
<220> yhbs b3157
<222> 325-424
<223> yhbs b3157 U00096 complement(3297896..3298399)
<313> 363: 358-377
<400> 363
GTGACGCTGG GCGATCCGGC GCTGTACAGT CGTTTCGGCT TTGAACTGGC 50
GGCGCATCAC GATCTGCGTT GCCGCTGGCC GGGCACTGAA AGCGCCTTCC 100
<210> 364
<211> 100
<212> DNA
<213> Escherichia coli K-12 MG1655
<220> dapa b2479
<222> 762-861
<223> dapa b2479 U00096 complement(2596902..2597780)
<313> 364: 813-832
<400> 364
GGCATGTAAG GAACTGGGTC TTGTGGCGAC CGATACGCTG CGCCTGCCAA 50
TGACACCAAT CACCGACAGT GGTCGTGAGA CGGTCAGAGC GGCGCTTAAG 100
<210> 365
<211> 100
<212> DNA
<213> Escherichia coli K-12 MG1655
<220> dapa b2479
<222> 748-847
<223> dapa b2479 U00096 complement(2596902..2597780)
<313> 365: 760-779
<400> 365
ATCCCGGTGA AATGGGCATG TAAGGAACTG GGTCTTGTGG CGACCGATAC 50
GCTGCGCCTG CCAATGACAC CAATCACCGA CAGTGGTCGT GAGACGGTCA 100
<210> 366
<211> 100
<212> DNA
<213> Escherichia coli K-12 MG1655
<220> dapa b2479
<222> 687-786
<223> dapa b2479 U00096 complement(2596902..2597780)
<313> 366: 721-740
<400> 366
ACGCGTTATT AATCAGCGTC TGATGCCATT ACACAACAAA CTATTTGTCG 50
AACCCAATCC AATCCCGGTG AAATGGGCAT GTAAGGAACT GGGTCTTGTG 100
<210> 367
<211> 100
<212> DNA
<213> Escherichia coli K-12 MG1655
<220> yhbt b3158
<222> 426-525
<223> yhbt b3158 U00096 complement(3298393..3298917)
<313> 367: 496-515
<400> 367
AGCCTTACGC ATGATGCTGC TGCAACTGGC GGATTTTGTT GAGGCGGGAA 50
TGAAAACCGC GCCTGAAACC AAACAGACAT CGGTAGGTGA ACCATGCTAA 100
<210> 368
<211> 100
<212> DNA
<213> Escherichia coli K-12 MG1655
<220> yhbt b3158
<222> 402-501

<223> yhbt b3158 U00096 complement(3298393..3298917)
<313> 368: 456-475
<400> 368
CATTGAACTG GAGCAAATGC CGAAAGCCTT ACGCATGATG CTGCTGCAAC  50
TGGCGGATTT TGTTGAGGCG GGAATGAAAA CCGCGCCTGA AACCAAACAG  100
<210> 369
<211> 100
<212> DNA
<213> Escherichia coli K-12 MG1655
<220> yhbt b3158
<222> 368-467
<223> yhbt b3158 U00096 complement(3298393..3298917)
<313> 369: 414-433
<400> 369
AGCTGGGGCT GTATGTGAAA AACCTGATGG ACGCCATTGA ACTGGAGCAA  50
ATGCCGAAAG CCTTACGCAT GATGCTGCTG CAACTGGCGG ATTTTGTTGA  100
<210> 370
<211> 100
<212> DNA
<213> Escherichia coli K-12 MG1655
<220> dapb b0032
<222> 723-822
<223> dapb b0032 U00096 28374..29195
<313> 370: 775-794
<400> 370
GACATTTGCT AACGGCGCGG TAAGATCGGC TTTGTGGTTG AGTGGTAAGG  50
AAAGCGGTCT TTTTGATATG CGAGATGTAC TTGATCTCAA TAATTTGTAA  100
<210> 371
<211> 100
<212> DNA
<213> Escherichia coli K-12 MG1655
<220> dapb b0032
<222> 690-789
<223> dapb b0032 U00096 28374..29195
<313> 371: 734-753
<400> 371
TCTGGAGATC ACCCATAAGG CGTCCAGCCG TATGACATTT GCTAACGGCG  50
CGGTAAGATC GGCTTTGTGG TTGAGTGGTA AGGAAAGCGG TCTTTTTGAT  100
<210> 372
<211> 100
<212> DNA
<213> Escherichia coli K-12 MG1655
<220> dapb b0032
<222> 574-673
<223> dapb b0032 U00096 28374..29195
<313> 372: 610-629
<400> 372
TACAGTCGTG AAGGCCACAC CGGTGAACGT GTGCCTGGCA CCATTGGTTT  50
TGCCACCGTG CGTGCAGGTG ACATCGTTGG TGAACATACC GCGATGTTTG  100
<210> 373
<211> 100
<212> DNA
<213> Escherichia coli K-12 MG1655
<220> yhbu b3159
<222> 897-996
<223> yhbu b3159 U00096 3299126..3300121
<313> 373: 956-975
<400> 373
GCAAAACTTT GTACCGCAAA GCGCGTGGAT GGAGACGCTC GGGTCGATGT  50
CCGAAGGCAC GCAAACCACT CTTGGCGCAT ATCACCGTAA ATGGCAGTGA  100
<210> 374
<211> 100
<212> DNA
<213> Escherichia coli K-12 MG1655
<220> yhbu b3159
<222> 861-960
<223> yhbu b3159 U00096 3299126..3300121
<313> 374: 908-927
<400> 374
CTGGCGTCAG GCTATCGACC GTTGTAAGGC CGATCCGCAA AACTTTGTAC  50
CGCAAAGCGC GTGGATGGAG ACGCTCGGGT CGATGTCCGA AGGCACGCAA  100

<210> 375
<211> 100
<212> DNA
<213> Escherichia coli K-12 MG1655
<220> yhbu b3159
<222> 752-851
<223> yhbu b3159 U00096 3299126..3300121
<313> 375: 804-823
<400> 375
GTCTCAATAC CCTGGAACTG CTGCCGGAGT TAATGGCGGC GAATATTGCT  50
TCGGTGAAAA TTGAAGGCCG CCAGCGTAGC CCGGCGTATG TCAGCCAGGT  100
<210> 376
<211> 100
<212> DNA
<213> Escherichia coli K-12 MG1655
<220> yhbv b3160
<222> 798-897
<223> yhbv b3160 U00096 3300112..3301008
<313> 376: 851-870
<400> 376
CCGCGCTAAT GAAAATGGCG CTGCACCACT GCCGTTGACG GCGAACAGCG  50
ACTGTAACGG CTACTGGCGG CGGCTGGCAG GACTGGAGCT GCAAGCCTAA  100
<210> 377
<211> 100
<212> DNA
<213> Escherichia coli K-12 MG1655
<220> yhbv b3160
<222> 725-824
<223> yhbv b3160 U00096 3300112..3301008
<313> 377: 749-768
<400> 377
CCATGCAGGG CCTGGTTGAT GTGGTCCGCC TGTCACCGCA GGGTACTGAC  50
ACTTTCGCGA TGCTCGACGC CTTCCGCGCT AATGAAAATG GCGCTGCACC  100
<210> 378
<211> 100
<212> DNA
<213> Escherichia coli K-12 MG1655
<220> yhbv b3160
<222> 670-769
<223> yhbv b3160 U00096 3300112..3301008
<313> 378: 720-739
<400> 378
AATGGCATTC AGACCATGAG CGGCTACGTT TACAACCTCG GTAACGAGCT  50
GGCATCCATG CAGGGCCTGG TTGATGTGGT CCGCCTGTCA CCGCAGGGTA  100
<210> 379
<211> 100
<212> DNA
<213> Escherichia coli K-12 MG1655
<220> dapd b0167
<222> 726-825
<223> dapd b0167 U00096 complement(185123..185947)
<313> 379: 798-817
<400> 379
TGGCAAATAC AGCCTCTACT GTGCGGTTAT CGTTAAGAAA GTTGACGCGA  50
AAACTCGCGG CAAAGTCGGC ATTAACGAAC TGCTGCGTAC CATCGACTAA  100
<210> 380
<211> 100
<212> DNA
<213> Escherichia coli K-12 MG1655
<220> dapd b0167
<222> 661-760
<223> dapd b0167 U00096 complement(185123..185947)
<313> 380: 693-712
<400> 380
GAAATCCACT ACGGTCGCGT TCCGGCGGGG TCTGTGGTTG TTTCAGGTAA  50
TCTGCCGTCA AAAGATGGCA AATACAGCCT CTACTGTGCG GTTATCGTTA  100
<210> 381
<211> 100
<212> DNA
<213> Escherichia coli K-12 MG1655
<220> dapd b0167

<222> 590-689
<223> dapd b0167 U00096 complement(185123..185947)
<313> 381: 637-656
<400> 381
AAGAAGGTTC CGTCATTTCC ATGGGCGTAT ACATTGGTCA GAGCACCCGT 50
ATTTACGACC GTGAAACCGG CGAAATCCAC TACGGTCGCG TTCCGGCGGG 100
<210> 382
<211> 100
<212> DNA
<213> Escherichia coli K-12 MG1655
<220> yhbw b3161
<222> 909-1008
<223> yhbw b3161 U00096 3301089..3302096
<313> 382: 988-1007
<400> 382
CGCCGATGAG ATTATGGTCA ACGGGCAGAT TTTCGACCAC CAGGCGCGGC 50
TGCATTCGTT TGAGCTGGCG ATGGATGTTA AGGAAGAGTT GTTGGGATAG 100
<210> 383
<211> 100
<212> DNA
<213> Escherichia coli K-12 MG1655
<220> yhbw b3161
<222> 903-1002
<223> yhbw b3161 U00096 3301089..3302096
<313> 383: 931-950
<400> 383
AACCGACGCC GATGAGATTA TGGTCAACGG GCAGATTTTC GACCACCAGG 50
CGCGGCTGCA TTCGTTTGAG CTGGCGATGG ATGTTAAGGA AGAGTTGTTG 100
<210> 384
<211> 100
<212> DNA
<213> Escherichia coli K-12 MG1655
<220> yhbw b3161
<222> 849-948
<223> yhbw b3161 U00096 3301089..3302096
<313> 384: 893-912
<400> 384
GTTGGTAGGT GATAAAGCGA AAGTGCGTCA TGGCTTGCAG TCGATCCTGC 50
GCGAAACCGA CGCCGATGAG ATTATGGTCA ACGGGCAGAT TTTCGACCAC 100
<210> 385
<211> 100
<212> DNA
<213> Escherichia coli K-12 MG1655
<220> dape b2473
<222> 1029-1128
<223> dape b2473 U00096 2589627..2590754
<313> 385: 1108-1127
<400> 385
CAATGCCACT ATTCATAAAA TTAATGAATG TGTGAACGCT GCCGACCTGC 50
AGCTACTTGC CCGTATGTAT CAACGTATCA TGGAACAGCT CGTCGCCTGA 100
<210> 386
<211> 100
<212> DNA
<213> Escherichia coli K-12 MG1655
<220> dape b2473
<222> 919-1018
<223> dape b2473 U00096 2589627..2590754
<313> 386: 953-972
<400> 386
GCGGTTGAGC ACTATAATGA AATTAAACCG CAGCTACTGA CCACAGGCGG 50
AACGTCCGAC GGGCGCTTTA TTGCCCGCAT GGGGGCGCAG GTGGTGGAAC 100
<210> 387
<211> 100
<212> DNA
<213> Escherichia coli K-12 MG1655
<220> dape b2473
<222> 807-906
<223> dape b2473 U00096 2589627..2590754
<313> 387: 841-860
<400> 387
GCAGGTGCTT GCCCTGCTTG AAAAACATCA ACTGCGCTAT ACGGTGGATT 50

GGTGGCTTTC CGGGCAGCCA TTTTTGACCG CGCGCGGTAA ACTGGTGGAT 100
<210> 388
<211> 100
<212> DNA
<213> Escherichia coli K-12 MG1655
<220> yhbx b3174
<222> 1545-1644
<223> yhbx b3174 U00096 complement(3317629..3319272)
<313> 388: 1613-1632
<400> 388
GGTTGTGGAT GCAAACCATG ATGTTTTCGA TTATGTGATG CTCAGAAAGG 50
AGTTTACAGA GGATAAGCAA GGTAACCCCA CCCCTGAAGG GCAGGGTTGA 100
<210> 389
<211> 100
<212> DNA
<213> Escherichia coli K-12 MG1655
<220> yhbx b3174
<222> 1508-1607
<223> yhbx b3174 U00096 complement(3317629..3319272)
<313> 389: 1552-1571
<400> 389
TTGAGGAAGT GATTGCACAC TATAAAGGAG ACTCACGGGT TGTGGATGCA 50
AACCATGATG TTTTCGATTA TGTGATGCTC AGAAAGGAGT TTACAGAGGA 100
<210> 390
<211> 100
<212> DNA
<213> Escherichia coli K-12 MG1655
<220> yhbx b3174
<222> 1480-1579
<223> yhbx b3174 U00096 complement(3317629..3319272)
<313> 390: 1501-1520
<400> 390
GTAACAAAGC CGGAACAGCC GCAAACGCTT GAGGAAGTGA TTGCACACTA 50
TAAAGGAGAC TCACGGGTTG TGGATGCAAA CCATGATGTT TTCGATTATG 100
<210> 391
<211> 100
<212> DNA
<213> Escherichia coli K-12 MG1655
<220> dapf b3810
<222> 729-828
<223> dapf b3810 U00096 3992372..3993199
<313> 391: 798-817
<400> 391
CCCCGGCGGT CGTCTTGATA TCGCCTGGAA AGGTCCGGGT CACCCGTTAT 50
ATATGACTGG CCCGGCGGTA CATGTCTACG ACGGATTTAT TCATCTATGA 100
<210> 392
<211> 100
<212> DNA
<213> Escherichia coli K-12 MG1655
<220> dapf b3810
<222> 527-626
<223> dapf b3810 U00096 3992372..3993199
<313> 392: 584-603
<400> 392
TTGGTCCTGT TCTGGAAAGC CACGAGCGTT TTCCGGAGCG CGCCAATATC 50
GGTTTTATGC AAGTGGTTAA GCGCGAGCAT ATTCGTTTAC GCGTTTATGA 100
<210> 393
<211> 100
<212> DNA
<213> Escherichia coli K-12 MG1655
<220> dapf b3810
<222> 413-512
<223> dapf b3810 U00096 3992372..3993199
<313> 393: 451-470
<400> 393
AGACCTATAT TATGCGCGCC GCCGAGCAGA CAATCTTATG CGGCGTGGTG 50
TCGATGGGAA ATCCGCATTG CGTGATTCAG GTCGATGATG TCGATACCGC 100
<210> 394
<211> 100
<212> DNA
<213> Escherichia coli K-12 MG1655

<220> yhby b3181
<222> 195-294
<223> yhby b3181 U00096 3325431..3325724
<313> 394: 267-286
<400> 394
TATCGTGCGC GAAACCGGCG CCTGTAATGT ACAGGTCATC GGTAAAACGC 50
TGGTGCTTTA TCGCCCAACT AAAGAACGTA AAATCTCGCT GCCACGCTAA 100
<210> 395
<211> 100
<212> DNA
<213> Escherichia coli K-12 MG1655
<220> yhby b3181
<222> 105-204
<223> yhby b3181 U00096 3325431..3325724
<313> 395: 141-160
<400> 395
CGAGATTGAA CAAGCGTTAG AGCACCATGA ACTCATCAAG GTGAAAATCG 50
CCACCGAAGA TCGCGAAACT AAAACCTTGA TCGTGGAAGC TATCGTGCGC 100
<210> 396
<211> 100
<212> DNA
<213> Escherichia coli K-12 MG1655
<220> yhby b3181
<222> 58-157
<223> yhby b3181 U00096 3325431..3325724
<313> 396: 94-113
<400> 396
CCAGTTGTTC TGCTTGGCAG TAATGGTTTG ACCGAAGGGG TGCTGGCCGA 50
GATTGAACAA GCGTTAGAGC ACCATGAACT CATCAAGGTG AAAATCGCCA 100
<210> 397
<211> 100
<212> DNA
<213> Escherichia coli K-12 MG1655
<220> nei b0715
<222> 693-792
<223> nei b0715 U00096 745158..745949
<313> 397: 760-779
<400> 397
TCATCGAGAT GGCGAACCGT GCGAACGTTG TGGCAGCATC ATTGAGAAAA 50
CCACGCTGTC ATCTCGCCCG TTTTACTGGT GCCCTGGCTG CCAGCACTAG 100
<210> 398
<211> 100
<212> DNA
<213> Escherichia coli K-12 MG1655
<220> nei b0715
<222> 657-756
<223> nei b0715 U00096 745158..745949
<313> 398: 724-743
<400> 398
TAAGCATCAT GGGGCGCTGT TTCGCTTTAA GGTTTTTCAT CGAGATGGCG 50
AACCGTGCGA ACGTTGTGGC AGCATCATTG AGAAAACCAC GCTGTCATCT 100
<210> 399
<211> 100
<212> DNA
<213> Escherichia coli K-12 MG1655
<220> nei b0715
<222> 631-730
<223> nei b0715 U00096 745158..745949
<313> 399: 655-674
<400> 399
GCTACGCGGG GGCAGGTGGA TGAGAATAAG CATCATGGGG CGCTGTTTCG 50
CTTTAAGGTT TTTCATCGAG ATGGCGAACC GTGCGAACGT TGTGGCAGCA 100
<210> 400
<211> 100
<212> DNA
<213> Escherichia coli K-12 MG1655
<220> yhbz b3184
<222> 1074-1173
<223> yhbz b3184 U00096 complement(3328223..3329395)
<313> 400: 1124-1143
<400> 400

CCAGCAGCTT GAAGAGATTG CTGAAGAGGA TGATGAAGAC TGGGATGACG 50
ACTGGGACGA AGACGACGAA GAAGGCGTTG AGTTCATTTA CAAGCGTTAA 100
<210> 401
<211> 100
<212> DNA
<213> Escherichia coli K-12 MG1655
<220> yhbz b3184
<222> 1042-1141
<223> yhbz b3184 U00096 complement(3328223..3329395)
<313> 401: 1079-1098
<400> 401
AAAGTCGAAT TCATGTGGGA TGATTATCAT CGCCAGCAGC TTGAAGAGAT 50
TGCTGAAGAG GATGATGAAG ACTGGGATGA CGACTGGGAC GAAGACGACG 100
<210> 402
<211> 100
<212> DNA
<213> Escherichia coli K-12 MG1655
<220> yhbz b3184
<222> 975-1074
<223> yhbz b3184 U00096 complement(3328223..3329395)
<313> 402: 1025-1044
<400> 402
CTGGGATGTG ATGACCTTTA TCATTGAAAA CCCGGTCGTG CAGGCTGAAG 50
AAGCGAAACA GCCAGAGAAA GTCGAATTCA TGTGGGATGA TTATCATCGC 100
<210> 403
<211> 100
<212> DNA
<213> Escherichia coli K-12 MG1655
<220> yhca b3216
<222> 576-675
<223> yhca b3216 U00096 3359747..3360421
<313> 403: 618-637
<400> 403
AACTACTGCA TTAACAAACG CTACGGATAC AAAAGTCGAG TTTTACCCAG 50
CCAGCCGATA TGGTTATCGC GGTAATAAAT ATGTCACTGA CTTAAAATAA 100
<210> 404
<211> 100
<212> DNA
<213> Escherichia coli K-12 MG1655
<220> yhca b3216
<222> 545-644
<223> yhca b3216 U00096 3359747..3360421
<313> 404: 581-600
<400> 404
ATAAGACCTA TTTTCTACCA CACACCAGTA CAACTACTGC ATTAACAAAC 50
GCTACGGATA CAAAAGTCGA GTTTTACCCA GCCAGCCGAT ATGGTTATCG 100
<210> 405
<211> 100
<212> DNA
<213> Escherichia coli K-12 MG1655
<220> yhca b3216
<222> 420-519
<223> yhca b3216 U00096 3359747..3360421
<313> 405: 460-479
<400> 405
GACCTGGAAA TTCCTGGAAT GGCGTAAAAA TGGCGACCAA ATTGAGATTA 50
GCAACCCCAG TAATTATGTC GTTCGTATGA CCTTACAATT TAAAACGCTC 100
<210> 406
<211> 100
<212> DNA
<213> Escherichia coli K-12 MG1655
<220> yhcb b3234
<222> 306-405
<223> yhcb b3234 U00096 3377820..3378224
<313> 406: 369-388
<400> 406
AGAGTCTGAA GCCAGCAACG ATCAGGCACC GGTGCAGATG CCTCGCGACT 50
ATTCTGAAGG CGCATCCGGC CTGCTGCGTA CTGGCGCGAA GCGCGACTAA 100
<210> 407
<211> 100
<212> DNA

<213> Escherichia coli K-12 MG1655
<220> yhcb b3234
<222> 264-363
<223> yhcb b3234 U00096 3377820..3378224
<313> 407: 309-328
<400> 407
GGAACTGTCT GCTGAAGCAA ACCCGTTCCG TAATCGTCTG GCAGAGTCTG 50
AAGCCAGCAA CGATCAGGCA CCGGTGCAGA TGCCTCGCGA CTATTCTGAA 100
<210> 408
<211> 100
<212> DNA
<213> Escherichia coli K-12 MG1655
<220> yhcb b3234
<222> 203-302
<223> yhcb b3234 U00096 3377820..3378224
<313> 408: 267-286
<400> 408
TGGCGCACGA CTATCGCCAG CTGTATCAGC ACATGGCAAA AAGCTCCAGC 50
AGCCTGCTGC CGGAACTGTC TGCTGAAGCA AACCCGTTCC GTAATCGTCT 100
<210> 409
<211> 100
<212> DNA
<213> Escherichia coli K-12 MG1655
<220> yhcc b3212
<222> 831-930
<223> yhcc b3212 U00096 complement(3350756..3351685)
<313> 409: 874-893
<400> 409
AAATCGATGG ACGGGAATGG TCGAGCTGGA TCGCTATCTG AATGAACATG 50
GCGTACAGGG ATCGGCGCTG GGACGTCCGT GGCTACCTCC AACGGAGTAG 100
<210> 410
<211> 100
<212> DNA
<213> Escherichia coli K-12 MG1655
<220> yhcc b3212
<222> 757-856
<223> yhcc b3212 U00096 complement(3350756..3351685)
<313> 410: 799-818
<400> 410
CCACCGGAAG TGATCTACCA CCGCATTTCC GCCAGCGCCC GTCGCCCGAC 50
GCTGCTTGCT CCGCTGTGGT GCGAAAATCG ATGGACGGGA ATGGTCGAGC 100
<210> 411
<211> 100
<212> DNA
<213> Escherichia coli K-12 MG1655
<220> yhcc b3212
<222> 697-796
<223> yhcc b3212 U00096 complement(3350756..3351685)
<313> 411: 761-780
<400> 411
TTGAACGGTA TTGAACTGGA GGATTACACG CTCACTGCCG GAGAAATGAT 50
TCGCCATACG CCACCGGAAG TGATCTACCA CCGCATTTCC GCCAGCGCCC 100
<210> 412
<211> 100
<212> DNA
<213> Escherichia coli K-12 MG1655
<220> yhcd b3217
<222> 2283-2382
<223> yhcd b3217 U00096 3360442..3362823
<313> 412: 2359-2378
<400> 412
TATAAAAGAT GAGCAGCAAT CATGCCGTAT TCACCTTACA TTCCCAGAAG 50
ATGCACCAAA AGATGTGTTC TATGAGACAG CAACAGGAGA GTGCCAATGA 100
<210> 413
<211> 100
<212> DNA
<213> Escherichia coli K-12 MG1655
<220> yhcd b3217
<222> 2281-2380
<223> yhcd b3217 U00096 3360442..3362823
<313> 413: 2312-2331

<400> 413
GATATAAAAG ATGAGCAGCA ATCATGCCGT ATTCACCTTA CATTCCCAGA 50
AGATGCACCA AAAGATGTGT TCTATGAGAC AGCAACAGGA GAGTGCCAAT 100
<210> 414
<211> 100
<212> DNA
<213> Escherichia coli K-12 MG1655
<220> yhcd b3217
<222> 2175-2274
<223> yhcd b3217 U00096 3360442..3362823
<313> 414: 2215-2234
<400> 414
ACTGCCCAGA GGCGTTGCGA TTGAAGATAG TGAAGGCAAC TATCTGACAA 50
CATCAGTGGA TGACGGTGTT GTATTCCTCA ATAACATCAA ACCGGACATG 100
<210> 415
<211> 100
<212> DNA
<213> Escherichia coli K-12 MG1655
<220> yhce b3218
<222> 331-430
<223> yhce b3218 U00096 3362820..3363299
<313> 415: 379-398
<400> 415
AAAAATGACA ATCCGGGCAG CACAGGAACA GCATCAATTA GGGAAGGTGC 50
GAATAAGCGG GGAAATTCTT CTCGGCTGAC TCAGTCATTT CATTTCTTCA 100
<210> 416
<211> 100
<212> DNA
<213> Escherichia coli K-12 MG1655
<220> yhce b3218
<222> 298-397
<223> yhce b3218 U00096 3362820..3363299
<313> 416: 324-343
<400> 416
AGTAACAATC TCGACCTGGC GTTAGCCAGT GGCAAAAATG ACAATCCGGG 50
CAGCACAGGA ACAGCATCAA TTAGGGAAGG TGCGAATAAG CGGGGAAATT 100
<210> 417
<211> 100
<212> DNA
<213> Escherichia coli K-12 MG1655
<220> yhce b3218
<222> 220-319
<223> yhce b3218 U00096 3362820..3363299
<313> 417: 252-271
<400> 417
GCTAATATTT CCAACAACGG ATTTGGATTT GGCCCGAATG GTTCATTAAA 50
TCTGATCGCC AGCGATGCAT TTTCAGGTAG TAACAATCTC GACCTGGCGT 100
<210> 418
<211> 100
<212> DNA
<213> Escherichia coli K-12 MG1655
<220> yhcf b3220
<222> 618-717
<223> yhcf b3220 U00096 3364561..3365277
<313> 418: 673-692
<400> 418
TATACCGCTT CAGGTGAGCG CATCTATCGC GAATGATTTA GGCGGTTCAG 50
AAAAAATCGA ACTTGATGGG AAAGCTGTGA TAAGCGTAGT CTACTTGTGA 100
<210> 419
<211> 100
<212> DNA
<213> Escherichia coli K-12 MG1655
<220> yhcf b3220
<222> 569-668
<223> yhcf b3220 U00096 3364561..3365277
<313> 419: 609-628
<400> 419
CCTTTGTAAA ACCAGGTGAA ACAACCCCAA CTCCTGTAAC GAACGCCATT 50
ATACCGCTTC AGGTGAGCGC ATCTATCGCG AATGATTTAG GCGGTTCAGA 100
<210> 420
<211> 100

<212> DNA
<213> Escherichia coli K-12 MG1655
<220> yhcf b3220
<222> 535-634
<223> yhcf b3220 U00096 3364561..3365277
<313> 420: 583-602
<400> 420
TATCCGCTTG CAGTCGATCA AAGCTATTAT TACACCTTTG TAAAACCAGG 50
TGAAACAACC CCAACTCCTG TAACGAACGC CATTATACCG CTTCAGGTGA 100
<210> 421
<211> 100
<212> DNA
<213> Escherichia coli K-12 MG1655
<220> yhcg b3221
<222> 1026-1125
<223> yhcg b3221 U00096 3365462..3366589
<313> 421: 1054-1073
<400> 421
TCTGGCAAGC GAATATAAGA TGCAACTACC TGATGAGAAA CGACTCGCAG 50
ATGAACTCGT TCGAACACAG GCGGTGCTAG AGGAAGGCTA TAGACGCCGT 100
<210> 422
<211> 100
<212> DNA
<213> Escherichia coli K-12 MG1655
<220> yhcg b3221
<222> 896-995
<223> yhcg b3221 U00096 3365462..3366589
<313> 422: 935-954
<400> 422
ATATGTATCT CAACTACGCC AAAGAGCACT GGACGCTACC GGATGAAAAT 50
CCGCCCATCG GTCTGGTTCT CTGTGCAGAG AAAGGAGCCG GAGAAGCGCA 100
<210> 423
<211> 100
<212> DNA
<213> Escherichia coli K-12 MG1655
<220> yhcg b3221
<222> 831-930
<223> yhcg b3221 U00096 3365462..3366589
<313> 423: 897-916
<400> 423
ACGCTGCCTG CTAATCGTCG ATCTAAAAGT GGGCAAATTC AGCTATAGCG 50
ATGCCGGACA GATGAATATG TATCTCAACT ACGCCAAAGA GCACTGGACG 100
<210> 424
<211> 100
<212> DNA
<213> Escherichia coli K-12 MG1655
<220> yhch b3222
<222> 363-462
<223> yhch b3222 U00096 complement(3366649..3367113)
<313> 424: 428-447
<400> 424
CGTGTTTATG CCAGGTGAAC CGCATAAACC AGGATGCGTT GTCGGCGAGC 50
CTGGAGAGAT TAAAAAGGTT GTGGTGAAGG TTAAGGCTGA TTTAATGGCT 100
<210> 425
<211> 100
<212> DNA
<213> Escherichia coli K-12 MG1655
<220> yhch b3222
<222> 268-367
<223> yhch b3222 U00096 complement(3366649..3367113)
<313> 425: 322-341
<400> 425
CGTCAGTGTG AAGAGTTCCA CCATGAGGAT GATTATCAGC TTTGCAGCAC 50
CATTGATAAC GAGCAAGCCA TCATCTTAAA ACCGGGAATG TTCGCCGTGT 100
<210> 426
<211> 100
<212> DNA
<213> Escherichia coli K-12 MG1655
<220> yhch b3222
<222> 230-329
<223> yhch b3222 U00096 complement(3366649..3367113)

<313> 426: 267-286
<400> 426
GTGAGGAACG GATTCTGTTT GGCATGGCAG GCACTGCGCG TCAGTGTGAA 50
GAGTTCCACC ATGAGGATGA TTATCAGCTT TGCAGCACCA TTGATAACGA 100
<210> 427
<211> 100
<212> DNA
<213> Escherichia coli K-12 MG1655
<220> yhci b3223
<222> 810-909
<223> yhci b3223 U00096 complement(3367110..3368018)
<313> 427: 862-881
<400> 427
GGAGCCAGCG GCATTTCATG TTGATTTACT GGCGGCGCAT TACCGCCATG 50
ATGCAGGTTT ACTTGGGGCT GCGCTGTTGG CCCAGGGAGA AAAATTATGA 100
<210> 428
<211> 100
<212> DNA
<213> Escherichia coli K-12 MG1655
<220> yhci b3223
<222> 789-888
<223> yhci b3223 U00096 complement(3367110..3368018)
<313> 428: 814-833
<400> 428
GGTGGAAACG TATCTGGCGC AGGAGCCAGC GGCATTTCAT GTTGATTTAC 50
TGGCGGCGCA TTACCGCCAT GATGCAGGTT TACTTGGGGC TGCGCTGTTG 100
<210> 429
<211> 100
<212> DNA
<213> Escherichia coli K-12 MG1655
<220> yhci b3223
<222> 665-764
<223> yhci b3223 U00096 complement(3367110..3368018)
<313> 429: 716-735
<400> 429
AGCAGCTGAT TCACCGCTCC GCACGTACGC TTGCAAGGCT GATCGCTGAT 50
ATTAAAGCCA CAACTGATTG CCAGTGCGTG GTGGTCGGTG GCAGCGTTGG 100
<210> 430
<211> 100
<212> DNA
<213> Escherichia coli K-12 MG1655
<220> fur b0684
<222> 324-423
<223> fur b0684 U00096 complement(709423..709869)
<313> 430: 371-390
<400> 430
AGCGCGTCAG CGTGAAATTG CCGCAAAACA TGGCATTCGC CTGACTAACC 50
ACAGTCTCTA TCTTTACGGT CACTGTGCCG AAGGCGATTG CCGCGAAGAT 100
<210> 431
<211> 100
<212> DNA
<213> Escherichia coli K-12 MG1655
<220> fur b0684
<222> 272-371
<223> fur b0684 U00096 complement(709423..709869)
<313> 431: 320-339
<400> 431
TGATCTGCCT CGACTGCGGC AAGGTTATCG AATTTAGTGA TGATTCCATC 50
GAAGCGCGTC AGCGTGAAAT TGCCGCAAAA CATGGCATTC GCCTGACTAA 100
<210> 432
<211> 100
<212> DNA
<213> Escherichia coli K-12 MG1655
<220> fur b0684
<222> 240-339
<223> fur b0684 U00096 complement(709423..709869)
<313> 432: 288-307
<400> 432
TGAACTGACA CAGCAACATC ACCACGATCA CCTGATCTGC CTCGACTGCG 50
GCAAGGTTAT CGAATTTAGT GATGATTCCA TCGAAGCGCG TCAGCGTGAA 100
<210> 433

<211> 100
<212> DNA
<213> Escherichia coli K-12 MG1655
<220> yhcj b3224
<222> 591-690
<223> yhcj b3224 U00096 complement(3367982..3368671)
<313> 433: 657-676
<400> 433
GCGCCACGGC GCGTGGGCGG TGACGGTCGG TTCTGCAATC ACGCGTCTTG 50
AGCACATTTG TCAGTGGTAC AACACAGCGA TGAAAAAGGC GGTGCTATGA 100
<210> 434
<211> 100
<212> DNA
<213> Escherichia coli K-12 MG1655
<220> yhcj b3224
<222> 554-653
<223> yhcj b3224 U00096 complement(3367982..3368671)
<313> 434: 594-613
<400> 434
GGCGTTACAA CACGCCTGCT CAGGCGGCGG ATGCGATGCG CCACGGCGCG 50
TGGGCGGTGA CGGTCGGTTC TGCAATCACG CGTCTTGAGC ACATTTGTCA 100
<210> 435
<211> 100
<212> DNA
<213> Escherichia coli K-12 MG1655
<220> yhcj b3224
<222> 474-573
<223> yhcj b3224 U00096 complement(3367982..3368671)
<313> 435: 513-532
<400> 435
CACGCCTGAA ACGCCAGAAG AGCCGGATCT GGCGCTGGTG AAAACGTTGA 50
GCGACGCCGG ATGTCGGGTG ATTGCCGAAG GGCGTTACAA CACGCCTGCT 100
<210> 436
<211> 100
<212> DNA
<213> Escherichia coli K-12 MG1655
<220> yhck b3227
<222> 693-792
<223> yhck b3227 U00096 complement(3371333..3372124)
<313> 436: 759-778
<400> 436
TCATGATCCT GACGAAGCCG ATCGTGCGTT GCAATCGCAT CTCAACAGCG 50
TCTCTGCTAC CTGGCACGCT TTCGGTCAGA CCACCAACAA AAAGAAATAA 100
<210> 437
<211> 100
<212> DNA
<213> Escherichia coli K-12 MG1655
<220> yhck b3227
<222> 595-694
<223> yhck b3227 U00096 complement(3371333..3372124)
<313> 437: 643-662
<400> 437
CTTATTGCCG CACGCCCAAC GGTTACCGAT CAGGCACTGC ACGAACATAA 50
CAACGTTAGT TATCAACAGC ATATTGCGAT CGTTGATGCG ATCCGCCGTC 100
<210> 438
<211> 100
<212> DNA
<213> Escherichia coli K-12 MG1655
<220> yhck b3227
<222> 541-640
<223> yhck b3227 U00096 complement(3371333..3372124)
<313> 438: 594-613
<400> 438
GAGATCCCCG GTAACCCAAT CTTCATGGCG ATCCACGTTG CCCTGCTCGA 50
CTGGCTTATT GCCGCACGCC CAACGGTTAC CGATCAGGCA CTGCACGAAC 100
<210> 439
<211> 100
<212> DNA
<213> Escherichia coli K-12 MG1655
<220> yhcl b3228
<222> 1214-1313

<223> yhcl b3228 U00096 3372504..3373871
<313> 439: 1258-1277
<400> 439
CGCTAGCGCG CGCGGTTTCG CCGATTACTG CGGTGGTGGT CGTTACGTCA 50
GGAATTGCAG GCGTTTCGCC TTTTGCGGTG GTGAAGCGGA CAGCGATCCC 100
<210> 440
<211> 100
<212> DNA
<213> Escherichia coli K-12 MG1655
<220> yhcl b3228
<222> 1108-1207
<223> yhcl b3228 U00096 3372504..3373871
<313> 440: 1132-1151
<400> 440
TCTGGCAATG CGCCGTTTAT GTCATTTGCC AGTCTTATTC CGAATATCGC 50
AGCCGGACTA CATGTACCAG CGGTTGTAAT GATTATGCCG ATGCATTTTG 100
<210> 441
<211> 100
<212> DNA
<213> Escherichia coli K-12 MG1655
<220> yhcl b3228
<222> 982-1081
<223> yhcl b3228 U00096 3372504..3373871
<313> 441: 1009-1028
<400> 441
AAAGGCTTAA CGACGATTGG CACTGTCGAT GCGGTTATCA GGGGGGCGGA 50
GCATTCTGGT CTGGGCGGTA TTGGCGTGAT GATTATTATG GCGCTGGTCA 100
<210> 442
<211> 100
<212> DNA
<213> Escherichia coli K-12 MG1655
<220> yhcm b3233
<222> 1029-1128
<223> yhcm b3233 U00096 complement(3376505..3377632)
<313> 442: 1101-1120
<400> 442
TCAGGGCGAT CGGCTGAAGT TTGAGTTCCA GCGTTGCCTG TCACGTCTGC 50
AAGAGATGCA AAGCGAAGAG TATCTGAAGC GCGAGCATTT GGCGGGTTAA 100
<210> 443
<211> 100
<212> DNA
<213> Escherichia coli K-12 MG1655
<220> yhcm b3233
<222> 981-1080
<223> yhcm b3233 U00096 complement(3376505..3377632)
<313> 443: 1005-1024
<400> 443
CCATGTCAAA TTAGTGGTGA GTGCAGAAGT GCCGCTGTAT GAAATTTATC 50
AGGGCGATCG GCTGAAGTTT GAGTTCCAGC GTTGCCTGTC ACGTCTGCAA 100
<210> 444
<211> 100
<212> DNA
<213> Escherichia coli K-12 MG1655
<220> yhcm b3233
<222> 912-1011
<223> yhcm b3233 U00096 complement(3376505..3377632)
<313> 444: 971-990
<400> 444
AGTTATGACG CGGTTGATGG AGAGCGAAGC GCGGCGCTTT ATTGCGCTGG 50
TGGATGAGTT TTACGAGCGC CATGTCAAAT TAGTGGTGAG TGCAGAAGTG 100
<210> 445
<211> 100
<212> DNA
<213> Escherichia coli K-12 MG1655
<220> yhcn b3239
<222> 216-315
<223> yhcn b3239 U00096 3383122..3383436
<313> 445: 291-310
<400> 445
GCTGAACAAA AAAGCGGAAG AGAAAGGCGC AACGGCCTAC CAGATTACTG 50
AAGCTCGTAG CGGTGACACC TGGCACGCTA CGGCTGAACT GTACAAATAA 100

<210> 446
<211> 100
<212> DNA
<213> Escherichia coli K-12 MG1655
<220> yhcn b3239
<222> 195-294
<223> yhcn b3239 U00096 3383122..3383436
<313> 446: 251-270
<400> 446
GCCAATGGAT ATGCGTGAAA TGCTGAACAA AAAAGCGGAA GAGAAAGGCG 50
CAACGGCCTA CCAGATTACT GAAGCTCGTA GCGGTGACAC CTGGCACGCT 100
<210> 447
<211> 100
<212> DNA
<213> Escherichia coli K-12 MG1655
<220> yhcn b3239
<222> 161-260
<223> yhcn b3239 U00096 3383122..3383436
<313> 447: 205-224
<400> 447
TCGGGACCGT ATCCGTAAGT GGTGTGGCGT CTTCGCCAAT GGATATGCGT 50
GAAATGCTGA ACAAAAAAGC GGAAGAGAAA GGCGCAACGG CCTACCAGAT 100
<210> 448
<211> 100
<212> DNA
<213> Escherichia coli K-12 MG1655
<220> yhco b3240
<222> 174-273
<223> yhco b3240 U00096 complement(3383492..3383764)
<313> 448: 236-255
<400> 448
GGGAGAGAAA ACGCGTCGCC GTTTTGGCGC GTTAATATTG CTGTTTGATG 50
AGGCAGAGGA AGAGCTGGAA GGGCATTTGC GTTTTAATGT TCGTCATTAG 100
<210> 449
<211> 100
<212> DNA
<213> Escherichia coli K-12 MG1655
<220> yhco b3240
<222> 106-205
<223> yhco b3240 U00096 complement(3383492..3383764)
<313> 449: 158-177
<400> 449
GACTCACTAT GGGATGTGTT AATGAACGAT GTCCTGCCGC TACCACTTGA 50
GATTGAATTT GTTCATCTGG GAGAGAAAAC GCGTCGCCGT TTTGGCGCGT 100
<210> 450
<211> 100
<212> DNA
<213> Escherichia coli K-12 MG1655
<220> yhco b3240
<222> 83-182
<223> yhco b3240 U00096 complement(3383492..3383764)
<313> 450: 99-118
<400> 450
CGAAAGATAA GGTACGCGAT CTCGACTCAC TATGGGATGT GTTAATGAAC 50
GATGTCCTGC CGCTACCACT TGAGATTGAA TTTGTTCATC TGGGAGAGAA 100
<210> 451
<211> 100
<212> DNA
<213> Escherichia coli K-12 MG1655
<220> yhcp b3241
<222> 1869-1968
<223> yhcp b3241 U00096 complement(3383856..3385823)
<313> 451: 1937-1956
<400> 451
AAAGCTACGC ATCTGGCAAG CGCCACCGCA GGTGACGGAA CCGGTAAATC 50
GGCTGGCGGG GATGCTCCAT AAGTATCAAC ATGCGTTGAC CGATAGTTAA 100
<210> 452
<211> 100
<212> DNA
<213> Escherichia coli K-12 MG1655
<220> yhcp b3241

<222> 1843-1942
<223> yhcp b3241 U00096 complement(3383856..3385823)
<313> 452: 1883-1902
<400> 452
CTGGAAGAAC TGGAAATCTA CCAGGAAAAG CTACGCATCT GGCAAGCGCC  50
ACCGCAGGTG ACGGAACCGG TAAATCGGCT GGCGGGGATG CTCCATAAGT  100
<210> 453
<211> 100
<212> DNA
<213> Escherichia coli K-12 MG1655
<220> yhcp b3241
<222> 1746-1845
<223> yhcp b3241 U00096 complement(3383856..3385823)
<313> 453: 1786-1805
<400> 453
GGATTTATCG GCGTTTCACC GACAAATGCG CCGCACAGCA GACCATGTGA  50
TATCTGCCCG TAGCGATGAT AAACGTCGTC GGTACTTTGG CCAGTTGCTG  100
<210> 454
<211> 100
<212> DNA
<213> Escherichia coli K-12 MG1655
<220> yhcq b3242
<222> 834-933
<223> yhcq b3242 U00096 complement(3385829..3386761)
<313> 454: 893-912
<400> 454
CACCACTGCT ACAGTGGTGG TCACTGGCAA ACAAGATCGC GACGAAAGCC  50
AGGATTCGTT CTTCCGTAAA ATGGCCCATC GCCTGCGTGA GTTTGGTTAA  100
<210> 455
<211> 100
<212> DNA
<213> Escherichia coli K-12 MG1655
<220> yhcq b3242
<222> 781-880
<223> yhcq b3242 U00096 complement(3385829..3386761)
<313> 455: 807-826
<400> 455
GTTCCGGTTC GTATTCGTCT CGACAACCAG CAAGAGAACA TCTGGCCTGC  50
GGGCACCACT GCTACAGTGG TGGTCACTGG CAAACAAGAT CGCGACGAAA  100
<210> 456
<211> 100
<212> DNA
<213> Escherichia coli K-12 MG1655
<220> yhcq b3242
<222> 688-787
<223> yhcq b3242 U00096 complement(3385829..3386761)
<313> 456: 747-766
<400> 456
GCCGCAGGGG TCACCAACGC CAGCAGCACG CGTGACGACA AAGGGATGGC  50
GACTATAGAC TCTAACCTTG AATGGGTGCG TCTTGCGCAA CGTGTTCCGG  100
<210> 457
<211> 100
<212> DNA
<213> Escherichia coli K-12 MG1655
<220> b2845 b2846
<222> 1123-1222
<223> b2845 b2846 U00096 2983869..2985098
<313> 457: 1170-1189
<400> 457
ACGCTGTCAA CGTATCTCAT CCCGGCGCTT AAACCCTGGC GTTCCATCGG  50
AAATCTGATT ACGCTGATCG TGGGTATCCT GTGCGTATCG GTAATGTTCT  100
<210> 458
<211> 100
<212> DNA
<213> Escherichia coli K-12 MG1655
<220> b2845 b2846
<222> 1101-1200
<223> b2845 b2846 U00096 2983869..2985098
<313> 458: 1135-1154
<400> 458
CATGATACTT TTCATCATGC CTACGCTGTC AACGTATCTC ATCCCGGCGC  50

TTAAACCCTG GCGTTCCATC GGAAATCTGA TTACGCTGAT CGTGGGTATC 100
<210> 459
<211> 100
<212> DNA
<213> Escherichia coli K-12 MG1655
<220> b2845 b2846
<222> 1030-1129
<223> b2845 b2846 U00096 2983869..2985098
<313> 459: 1090-1109
<400> 459
ACCTTCATTG TTTGTTGCAT TAACCCGAAC GCGATTTCGA TGATTTACGC 50
GATCAGCGGC CCGCTCATTG CCATGATACT TTTCATCATG CCTACGCTGT 100
<210> 460
<211> 100
<212> DNA
<213> Escherichia coli K-12 MG1655
<220> yhcr b3243
<222> 174-273
<223> yhcr b3243 U00096 complement(3386769..3387041)
<313> 460: 222-241
<400> 460
TGTGCCAACA GGTATCTACG ACTTTGTCTG GCATCCGGCG TTGTTCAACA 50
CCGCGCTCTA TTGCTGCTTG TTTTATTTGA TATCGCGACT GTTCGTTTGA 100
<210> 461
<211> 100
<212> DNA
<213> Escherichia coli K-12 MG1655
<220> yhcr b3243
<222> 86-185
<223> yhcr b3243 U00096 complement(3386769..3387041)
<313> 461: 111-130
<400> 461
TTATCGTGGT GTTTGGGCTG TCCTTCCCAC CGATATTTTT TGAATTGCTT 50
TTATCACTGG CGATTTTCTG GCTGGTGCGC CGGGTACTTG TGCCAACAGG 100
<210> 462
<211> 100
<212> DNA
<213> Escherichia coli K-12 MG1655
<220> yhcr b3243
<222> 21-120
<223> yhcr b3243 U00096 complement(3386769..3387041)
<313> 462: 57-76
<400> 462
AGATGCTGAA AATAATAACG CCTGCTCTCT CTTTACAACC AAGGTCAACA 50
TGAGTCTGTT TCCCGTTATC GTGGTGTTTG GGCTGTCCTT CCCACCGATA 100
<210> 463
<211> 100
<212> DNA
<213> Escherichia coli K-12 MG1655
<220> yhcs b3244
<222> 831-930
<223> yhcs b3244 U00096 3387155..3388084
<313> 463: 904-923
<400> 463
GCCGCTGAAG GTACAGGTCG TGATCAACTC GCTGACGGAT TATTTTGTTG 50
AGGTCGGTAA ATTGTTTCAG GAGATGCACG GGCGCGGGAA AGAGAAGTAA 100
<210> 464
<211> 100
<212> DNA
<213> Escherichia coli K-12 MG1655
<220> yhcs b3244
<222> 803-902
<223> yhcs b3244 U00096 3387155..3388084
<313> 464: 829-848
<400> 464
ATGCGTTATA TACCGAAAAA GATAAGCTGC CGCTGAAGGT ACAGGTCGTG 50
ATCAACTCGC TGACGGATTA TTTTGTTGAG GTCGGTAAAT TGTTTCAGGA 100
<210> 465
<211> 100
<212> DNA
<213> Escherichia coli K-12 MG1655

<220> yhcs b3244
<222> 733-832
<223> yhcs b3244 U00096 3387155..3388084
<313> 465: 792-811
<400> 465
ATCAACGAGA TCAATCGTGG GGAGCTGGAG ATCCTGCTGC CGCGTTACCA  50
GTCAGATCCA CGCCCGGTTT ATGCGTTATA TACCGAAAAA GATAAGCTGC  100
<210> 466
<211> 100
<212> DNA
<213> Escherichia coli K-12 MG1655
<220> pshm b3335
<222> 387-486
<223> pshm b3335 U00096 3462695..3463180
<313> 466: 460-479
<400> 466
ACAGGTAACA TACGGTATTG TTATTGAAGA CCTTCAGTTT ACTCTTGCCG  50
GAAACGAAGA GATCACCCTC AGGCATTTGT CCTTCAGGGA GCAACAATAA  100
<210> 467
<211> 100
<212> DNA
<213> Escherichia coli K-12 MG1655
<220> pshm b3335
<222> 349-448
<223> pshm b3335 U00096 3462695..3463180
<313> 467: 378-397
<400> 467
GCTCCCCTCG AAAACGTCTC CCGCTGGCTC ACTACGGCAC AGGTAACATA  50
CGGTATTGTT ATTGAAGACC TTCAGTTTAC TCTTGCCGGA AACGAAGAGA  100
<210> 468
<211> 100
<212> DNA
<213> Escherichia coli K-12 MG1655
<220> pshm b3335
<222> 312-411
<223> pshm b3335 U00096 3462695..3463180
<313> 468: 338-357
<400> 468
CGGCCCGGAC AATACTCTGA CAATTCATCC TGTCACAGCT CCCCTCGAAA  50
ACGTCTCCCG CTGGCTCACT ACGGCACAGG TAACATACGG TATTGTTATT  100
<210> 469
<211> 100
<212> DNA
<213> Escherichia coli K-12 MG1655
<220> nfi b3999
<222> 579-678
<223> nfi b3999 U00096 4196362..4197039
<313> 469: 650-669
<400> 469
CTGCATGAAA GGCTATCGTC TGCCGGAGCC AACGCGCTGG GCGGACGCGG  50
TGGCCTCGGA ACGTCCGGCG TTCGTGCGCT ATACAGCAAA TCAGCCCTAA  100
<210> 470
<211> 100
<212> DNA
<213> Escherichia coli K-12 MG1655
<220> nfi b3999
<222> 573-672
<223> nfi b3999 U00096 4196362..4197039
<313> 470: 612-631
<400> 470
ACAACGCTGC ATGAAAGGCT ATCGTCTGCC GGAGCCAACG CGCTGGGCGG  50
ACGCGGTGGC CTCGGAACGT CCGGCGTTCG TGCGCTATAC AGCAAATCAG  100
<210> 471
<211> 100
<212> DNA
<213> Escherichia coli K-12 MG1655
<220> nfi b3999
<222> 495-594
<223> nfi b3999 U00096 4196362..4197039
<313> 471: 527-546
<400> 471

GCGCAGCAAA GCGCGCTGTA ACCCGTTGTT TATCGCTACC GGCCATCGGG 50
TCAGCGTGGA CAGCGCGCTG GCGTGGGTAC AACGCTGCAT GAAAGGCTAT 100
<210> 472
<211> 100
<212> DNA
<213> Escherichia coli K-12 MG1655
<220> mena b3931
<222> 818-917
<223> mena b3931 U00096 complement(4117003..4117929)
<313> 472: 867-886
<400> 472
AAATGGACCC GGTGGCGATG CGACCAATGC TGGAACGTAC TGTCAAGGGA 50
GCGTTACTGA CTAACCTGCT GTTTGTTTTA GGGATATTCC TAAGCCAGTG 100
<210> 473
<211> 100
<212> DNA
<213> Escherichia coli K-12 MG1655
<220> mena b3931
<222> 780-879
<223> mena b3931 U00096 complement(4117003..4117929)
<313> 473: 829-848
<400> 473
ACCATTACTG GTGAAGCAAG CCCGTTATGT GATGCGGGAA ATGGACCCGG 50
TGGCGATGCG ACCAATGCTG GAACGTACTG TCAAGGGAGC GTTACTGACT 100
<210> 474
<211> 100
<212> DNA
<213> Escherichia coli K-12 MG1655
<220> mena b3931
<222> 581-680
<223> mena b3931 U00096 complement(4117003..4117929)
<313> 474: 623-642
<400> 474
CGGCAGTACT GAATATTAAT AACCTGCGTG ATATCAATAG CGACCGCGAA 50
AATGGCAAAA ACACGCTGGT GGTGCGCTTA GGTGAAGTGA ACGCGCGTCG 100
<210> 475
<211> 100
<212> DNA
<213> Escherichia coli K-12 MG1655
<220> menb b2263
<222> 759-858
<223> menb b2263 U00096 complement(2373982..2374839)
<313> 475: 823-842
<400> 475
CACCATGCTG TTCTACATGA CGGAAGAAGG TCAGGAAGGT CGCAACGCCT 50
TCAACCAGAA ACGTCAGCCT GACTTCAGCA AATTCAAACG GAATCCGTAA 100
<210> 476
<211> 100
<212> DNA
<213> Escherichia coli K-12 MG1655
<220> menb b2263
<222> 741-840
<223> menb b2263 U00096 complement(2373982..2374839)
<313> 476: 780-799
<400> 476
GGAGCTGGCG GGCAACGCCA CCATGCTGTT CTACATGACG GAAGAAGGTC 50
AGGAAGGTCG CAACGCCTTC AACCAGAAAC GTCAGCCTGA CTTCAGCAAA 100
<210> 477
<211> 100
<212> DNA
<213> Escherichia coli K-12 MG1655
<220> menb b2263
<222> 716-815
<223> menb b2263 U00096 complement(2373982..2374839)
<313> 477: 744-763
<400> 477
ACTGTGACGG GCAGGCGGGG CTGCAGGAGC TGGCGGGCAA CGCCACCATG 50
CTGTTCTACA TGACGGAAGA AGGTCAGGAA GGTCGCAACG CCTTCAACCA 100
<210> 478
<211> 100
<212> DNA

<213> Escherichia coli K-12 MG1655
<220> nfo b2160
<222> 759-858
<223> nfo b2160 U00096 2248860..2249717
<313> 478: 837-856
<400> 478
CGACGGCATT CCGCTGATCC TCGAAACCAT CAACCCGGAT ATCTGGGCAG 50
AAGAGATCGC CTGGCTGAAA GCGCAACAAA CTGAAAAAGC GGTAGCCTGA 100
<210> 479
<211> 100
<212> DNA
<213> Escherichia coli K-12 MG1655
<220> nfo b2160
<222> 738-837
<223> nfo b2160 U00096 2248860..2249717
<313> 479: 772-791
<400> 479
GATCATGCAG GACGACCGTT TCGACGGCAT TCCGCTGATC CTCGAAACCA 50
TCAACCCGGA TATCTGGGCA GAAGAGATCG CCTGGCTGAA AGCGCAACAA 100
<210> 480
<211> 100
<212> DNA
<213> Escherichia coli K-12 MG1655
<220> nfo b2160
<222> 688-787
<223> nfo b2160 U00096 2248860..2249717
<313> 480: 729-748
<400> 480
CGCCATCATA GCCTCGGTGA AGGCAATATC GGTCATGATG CGTTCCGCTG 50
GATCATGCAG GACGACCGTT TCGACGGCAT TCCGCTGATC CTCGAAACCA 100
<210> 481
<211> 100
<212> DNA
<213> Escherichia coli K-12 MG1655
<220> menc b2262
<222> 864-963
<223> menc b2262 U00096 complement(2373020..2373982)
<313> 481: 921-940
<400> 481
GCTGGACACG CTGGATCTGA TGCAGGCGCA GCAGGTACGT CGCTGGCCGG 50
GTAGCACGCT GCCTGTCGTG GAAGTTGATG CACTGGAGCG GTTGTTATGA 100
<210> 482
<211> 100
<212> DNA
<213> Escherichia coli K-12 MG1655
<220> menc b2262
<222> 823-922
<223> menc b2262 U00096 complement(2373020..2373982)
<313> 482: 890-909
<400> 482
GCGCGGATTG CCGCCTGGTT AACGCCGGAC ACCATTCCAG GGCTGGACAC 50
GCTGGATCTG ATGCAGGCGC AGCAGGTACG TCGCTGGCCG GGTAGCACGC 100
<210> 483
<211> 100
<212> DNA
<213> Escherichia coli K-12 MG1655
<220> menc b2262
<222> 809-908
<223> menc b2262 U00096 complement(2373020..2373982)
<313> 483: 831-850
<400> 483
GCTTAACGCA ACTGGCGCGG ATTGCCGCCT GGTTAACGCC GGACACCATT 50
CCAGGGCTGG ACACGCTGGA TCTGATGCAG GCGCAGCAGG TACGTCGCTG 100
<210> 484
<211> 100
<212> DNA
<213> Escherichia coli K-12 MG1655
<220> mend b2265
<222> 1572-1671
<223> mend b2265 U00096 complement(2375609..2377279)
<313> 484: 1635-1654

<400> 484
CTGGCGCACG CCAACCACCA CGGTGATTGA AATGGTGGTT AACGACACCG 50
ATGGTGCGCA AACGCTCCAG CAACTTCTGG CGCAGGTAAG CCATTTATGA 100
<210> 485
<211> 100
<212> DNA
<213> Escherichia coli K-12 MG1655
<220> mend b2265
<222> 1571-1670
<223> mend b2265 U00096 complement(2375609..2377279)
<313> 485: 1593-1612
<400> 485
CCTGGCGCAC GCCAACCACC ACGGTGATTG AAATGGTGGT TAACGACACC 50
GATGGTGCGC AAACGCTCCA GCAACTTCTG GCGCAGGTAA GCCATTTATG 100
<210> 486
<211> 100
<212> DNA
<213> Escherichia coli K-12 MG1655
<220> mend b2265
<222> 1514-1613
<223> mend b2265 U00096 complement(2375609..2377279)
<313> 486: 1565-1584
<400> 486
AGCTGAAATA TCATCGTCCG CAAAACTGGC AGGAACTTGA AACGGCATTT 50
GCCGACGCCT GGCGCACGCC AACCACCACG GTGATTGAAA TGGTGGTTAA 100
<210> 487
<211> 100
<212> DNA
<213> Escherichia coli K-12 MG1655
<220> mene b2261
<222> 1257-1356
<223> mene b2261 U00096 complement(2371668..2373023)
<313> 487: 1329-1348
<400> 487
ACAACCGGTG CGCTGGCTAA CTCTGCCGCC GGAGCTGAAA AACGGCGGTA 50
TTAAAATTTC ACGTCAGGCG CTAAAAGAGT GGGTGCAACG TCAGCAATAA 100
<210> 488
<211> 100
<212> DNA
<213> Escherichia coli K-12 MG1655
<220> mene b2261
<222> 1167-1266
<223> mene b2261 U00096 complement(2371668..2373023)
<313> 488: 1199-1218
<400> 488
GTTTGGTCAT CGACCGGTGG CGGTGATGGA GTATGACCAC GAGAGCGTTG 50
ATCTTAGTGA ATGGGTGAAA GATAAGCTGG CCCGTTTTCA ACAACCGGTG 100
<210> 489
<211> 100
<212> DNA
<213> Escherichia coli K-12 MG1655
<220> mene b2261
<222> 1110-1209
<223> mene b2261 U00096 complement(2371668..2373023)
<313> 489: 1158-1177
<400> 489
AATTGCTGCA CATCCTGCGG TTTTGCAGGT GTTTATCGTC CCCGTTGCCG 50
ACAAGGAGTT TGGTCATCGA CCGGTGGCGG TGATGGAGTA TGACCACGAG 100
<210> 490
<211> 100
<212> DNA
<213> Escherichia coli K-12 MG1655
<220> yhda b3253
<222> 1834-1933
<223> yhda b3253 U00096 complement(3399029..3400969)
<313> 490: 1885-1904
<400> 490
CAGACCCTGA TTCAGCGCGG TGTTACAGGC GGGCAAGGGG ATTTTTTCGC 50
GTCCTCACAG CCACTTGATA CTAACGTGAA AAAATATTCA CAAAGATACT 100
<210> 491
<211> 100

<212> DNA
<213> Escherichia coli K-12 MG1655
<220> yhda b3253
<222> 1818-1917
<223> yhda b3253 U00096 complement(3399029..3400969)
<313> 491: 1845-1864
<400> 491
TTCGCGAAGC GAGTGGCAGA CCCTGATTCA GCGCGGTGTT ACAGGCGGGC 50
AAGGGGATTT TTTCGCGTCC TCACAGCCAC TTGATACTAA CGTGAAAAAA 100
<210> 492
<211> 100
<212> DNA
<213> Escherichia coli K-12 MG1655
<220> yhda b3253
<222> 1743-1842
<223> yhda b3253 U00096 complement(3399029..3400969)
<313> 492: 1766-1785
<400> 492
GAACCAGCTG CTGGTTCAAA GCCTGGTGGA AGCCTGCTCC GGGACCAGCA 50
CCCAGGTTTA CGCCACCGGC GTGCGTTCGC GAAGCGAGTG GCAGACCCTG 100
<210> 493
<211> 100
<212> DNA
<213> Escherichia coli K-12 MG1655
<220> menf b2266
<222> 972-1071
<223> menf b2266 U00096 complement(2377368..2378438)
<313> 493: 1041-1060
<400> 493
TGCTGGCGCG GGCATTGTCC GTGGTTCCGA CCCCGAGCAA GAGTGGCAGG 50
AAATCGACAA CAAAGCGGCA GGGCTGCGTA CTTTATTACA AATGGAATAG 100
<210> 494
<211> 100
<212> DNA
<213> Escherichia coli K-12 MG1655
<220> menf b2266
<222> 946-1045
<223> menf b2266 U00096 complement(2377368..2378438)
<313> 494: 994-1013
<400> 494
ATTAGCGGCA ATGTCGTGCG ATTATATGCT GGCGCGGGCA TTGTCCGTGG 50
TTCCGACCCC GAGCAAGAGT GGCAGGAAAT CGACAACAAA GCGGCAGGGC 100
<210> 495
<211> 100
<212> DNA
<213> Escherichia coli K-12 MG1655
<220> menf b2266
<222> 911-1010
<223> menf b2266 U00096 complement(2377368..2378438)
<313> 495: 926-945
<400> 495
AAAGCGAATT CTGCGTTTCC CTGCGCTCAG CAAAAATTAG CGGCAATGTC 50
GTGCGATTAT ATGCTGGCGC GGGCATTGTC CGTGGTTCCG ACCCCGAGCA 100
<210> 496
<211> 100
<212> DNA
<213> Escherichia coli K-12 MG1655
<220> meng b3930
<222> 387-486
<223> meng b3930 U00096 complement(4116425..4116910)
<313> 496: 452-471
<400> 496
CCGCGTCAAT TTTGGCGGTG TCACCTTCTT CTCCGGCGAC CATCTTTATG 50
CCGACAATAC CGGGATTATT CTTTCAGAAG ATCCGCTGGA TATTGAATGA 100
<210> 497
<211> 100
<212> DNA
<213> Escherichia coli K-12 MG1655
<220> meng b3930
<222> 370-469
<223> meng b3930 U00096 complement(4116425..4116910)

<313> 497: 414-433
<400> 497
ATTGGCGAAA GCGATGTCCG CGTCAATTTT GGCGGTGTCA CCTTCTTCTC 50
CGGCGACCAT CTTTATGCCG ACAATACCGG GATTATTCTT TCAGAAGATC 100
<210> 498
<211> 100
<212> DNA
<213> Escherichia coli K-12 MG1655
<220> meng b3930
<222> 277-376
<223> meng b3930 U00096 complement(4116425..4116910)
<313> 498: 330-349
<400> 498
TACGGCGCGG TGCGTCAGGT AGATGACCTG GAAGAGTTGG ATATCGGCAT 50
CCAGGCGATG GCGGCAATTC CGGTTGGTGC CGCTGGCGAA GGCATTGGCG 100
<210> 499
<211> 100
<212> DNA
<213> Escherichia coli K-12 MG1655
<220> cspa b3557
<222> 114-213
<223> cspa b3557 U00096 3717678..3717890
<313> 499: 172-191
<400> 499
GAACGATGGT TACAAATCTC TGGACGAAGG TCAGAAAGTG TCCTTCACCA 50
TCGAAAGCGG CGCTAAAGGC CCGGCAGCTG GTAACGTAAC CAGCCTGTAA 100
<210> 500
<211> 100
<212> DNA
<213> Escherichia coli K-12 MG1655
<220> cspa b3557
<222> 35-134
<223> cspa b3557 U00096 3717678..3717890
<313> 500: 88-107
<400> 500
TCAACGCTGA CAAAGGCTTC GGCTTCATCA CTCCTGACGA TGGCTCTAAA 50
GATGTGTTCG TACACTTCTC TGCTATCCAG AACGATGGTT ACAAATCTCT 100
<210> 501
<211> 100
<212> DNA
<213> Escherichia coli K-12 MG1655
<220> cspa b3557
<222> 1-100
<223> cspa b3557 U00096 3717678..3717890
<313> 501: 31-50
<400> 501
ATGTCCGGTA AAATGACTGG TATCGTAAAA TGGTTCAACG CTGACAAAGG 50
CTTCGGCTTC ATCACTCCTG ACGATGGCTC TAAAGATGTG TTCGTACACT 100
<210> 502
<211> 100
<212> DNA
<213> Escherichia coli K-12 MG1655
<220> cspb b1558
<222> 40-139
<223> cspb b1558 U00096 complement(1639363..1639578)
<313> 502: 91-110
<400> 502
GCTGATAAAG GTTTCGGCTT TATTTCTCCT GTTGATGGTA GTAAAGATGT 50
GTTTGTGCAT TTTTCTGCGA TTCAGAATGA TAATTATCGA ACCTTATTTG 100
<210> 503
<211> 100
<212> DNA
<213> Escherichia coli K-12 MG1655
<220> cspb b1558
<222> 23-122
<223> cspb b1558 U00096 complement(1639363..1639578)
<313> 503: 50-69
<400> 503
TAGTAAAATG GTTTAACGCT GATAAAGGTT TCGGCTTTAT TTCTCCTGTT 50
GATGGTAGTA AAGATGTGTT TGTGCATTTT TCTGCGATTC AGAATGATAA 100
<210> 504

<211> 100
<212> DNA
<213> Escherichia coli K-12 MG1655
<220> cspb b1558
<222> 6-105
<223> cspb b1558 U00096 complement(1639363..1639578)
<313> 504: 27-46
<400> 504
AAATAAAATG ACTGGTTTAG TAAAATGGTT TAACGCTGAT AAAGGTTTCG 50
GCTTTATTTC TCCTGTTGAT GGTAGTAAAG ATGTGTTTGT GCATTTTTCT 100
<210> 505
<211> 100
<212> DNA
<213> Escherichia coli K-12 MG1655
<220> cspc cspC
<222> 41-140
<223> cspc cspC U00096 complement(1905250..1905459)
<313> 505: 103-122
<400> 505
CTAAAGGTTT TGGCTTCATT ACTCCGGCTG ATGGCAGCAA AGATGTGTTC 50
GTACACTTCT CCGCTATCCA GGGTAATGGC TTCAAAACTC TGGCTGAAGG 100
<210> 506
<211> 100
<212> DNA
<213> Escherichia coli K-12 MG1655
<220> cspc cspC
<222> 34-133
<223> cspc cspC U00096 complement(1905250..1905459)
<313> 506: 99-118
<400> 506
AACGAGTCTA AAGGTTTTGG CTTCATTACT CCGGCTGATG GCAGCAAAGA 50
TGTGTTCGTA CACTTCTCCG CTATCCAGGG TAATGGCTTC AAAACTCTGG 100
<210> 507
<211> 100
<212> DNA
<213> Escherichia coli K-12 MG1655
<220> cspc cspC
<222> 52-151
<223> cspc cspC U00096 complement(1905250..1905459)
<313> 507: 73-92
<400> 507
GGCTTCATTA CTCCGGCTGA TGGCAGCAAA GATGTGTTCG TACACTTCTC 50
CGCTATCCAG GGTAATGGCT TCAAAACTCT GGCTGAAGGT CAGAACGTTG 100
<210> 508
<211> 100
<212> DNA
<213> Escherichia coli K-12 MG1655
<220> yhde b3249
<222> 495-594
<223> yhde b3249 U00096 complement(3395422..3396015)
<313> 508: 559-578
<400> 508
CAGCTATCAC GCCGTAGTCG GCTTACCGCT GGTTGAAACG TATGAATTAT 50
TAAGTAATTT TAACGCACTG CGTGAGAAAA GGGATAAACA TGACGGCTGA 100
<210> 509
<211> 100
<212> DNA
<213> Escherichia coli K-12 MG1655
<220> yhde b3249
<222> 432-531
<223> yhde b3249 U00096 complement(3395422..3396015)
<313> 509: 483-502
<400> 509
AGATAAAGCA GGTGCATACG GTATTCAGGG GCTGGGTGGC TGTTTTGTCA 50
GGAAGATAAA TGGCAGCTAT CACGCCGTAG TCGGCTTACC GCTGGTTGAA 100
<210> 510
<211> 100
<212> DNA
<213> Escherichia coli K-12 MG1655
<220> yhde b3249
<222> 388-487

<223> yhde b3249 U00096 complement(3395422..3396015)
<313> 510: 434-453
<400> 510
ACAGACGAAG ACATCGCGGG CTATGTCGCC AGCGATGAAC CGTTAGATAA 50
AGCAGGTGCA TACGGTATTC AGGGGCTGGG TGGCTGTTTT GTCAGGAAGA 100
<210> 511
<211> 100
<212> DNA
<213> Escherichia coli K-12 MG1655
<220> cspd b0881
<222> 126-225
<223> cspd b0881 U00096 complement(921589..921813)
<313> 511: 200-219
<400> 511
AAAAGCTGGA CAATCCGTTC AGTTTGATGT CCACCAGGGG CCAAAAGGCA 50
ATCACGCCAG TGTTATTGTG CCCGTCGAAG TAGAAGCGGC AGTCGCATAG 100
<210> 512
<211> 100
<212> DNA
<213> Escherichia coli K-12 MG1655
<220> cspd b0881
<222> 126-225
<223> cspd b0881 U00096 complement(921589..921813)
<313> 512: 155-174
<400> 512
AAAAGCTGGA CAATCCGTTC AGTTTGATGT CCACCAGGGG CCAAAAGGCA 50
ATCACGCCAG TGTTATTGTG CCCGTCGAAG TAGAAGCGGC AGTCGCATAG 100
<210> 513
<211> 100
<212> DNA
<213> Escherichia coli K-12 MG1655
<220> cspd b0881
<222> 20-119
<223> cspd b0881 U00096 complement(921589..921813)
<313> 513: 61-80
<400> 513
AGTGGTTCAA CAATGCCAAA GGGTTTGGTT TCATCTGCCC TGAAGGCGGC 50
GGCGAAGATA TTTTCGCTCA TTATTCCACC ATTCAGATGG ATGGTTACAG 100
<210> 514
<211> 100
<212> DNA
<213> Escherichia coli K-12 MG1655
<220> cspe b0624
<222> 111-210
<223> cspe b0624 U00096 656515..656724
<313> 514: 176-195
<400> 514
GACTAATGGT TTTAAAACTC TTGCTGAAGG TCAGCGCGTA GAGTTCGAAA 50
TCACTAACGG TGCCAAAGGC CCTTCTGCTG CAAACGTAAT CGCTCTGTAA 100
<210> 515
<211> 100
<212> DNA
<213> Escherichia coli K-12 MG1655
<220> cspe b0624
<222> 92-191
<223> cspe b0624 U00096 656515..656724
<313> 515: 151-170
<400> 515
TACACTTCTC TGCAATCCAG ACTAATGGTT TTAAAACTCT TGCTGAAGGT 50
CAGCGCGTAG AGTTCGAAAT CACTAACGGT GCCAAAGGCC CTTCTGCTGC 100
<210> 516
<211> 100
<212> DNA
<213> Escherichia coli K-12 MG1655
<220> cspe b0624
<222> 41-140
<223> cspe b0624 U00096 656515..656724
<313> 516: 88-107
<400> 516
CCAAAGGATT CGGTTTCATT ACTCCGGAAG ACGGCAGCAA AGACGTGTTC 50
GTACACTTCT CTGCAATCCA GACTAATGGT TTTAAAACTC TTGCTGAAGG 100

<210> 517
<211> 100
<212> DNA
<213> Escherichia coli K-12 MG1655
<220> yhdg b3261
<222> 843-942
<223> yhdg b3261 U00096 3407917..3408882
<313> 517: 890-909
<400> 517
ACACGTTTCC TGGTATCTCC AGGAACACGC TCCAAATGAC CAGTTTCGGC 50
GCACATTCAA CGCCATTGAG GATGCCAGCG AACAGCTGGA GGCGTTGGAG 100
<210> 518
<211> 100
<212> DNA
<213> Escherichia coli K-12 MG1655
<220> yhdg b3261
<222> 756-855
<223> yhdg b3261 U00096 3407917..3408882
<313> 518: 796-815
<400> 518
GGCAGAGGTT AAGCGCTTGC TTTGCGCGCA CGTTCGGGAA CTGCATGACT 50
TTTATGGTCC GGCAAAAGGG TACCGAATTG CACGTAAACA CGTTTCCTGG 100
<210> 519
<211> 100
<212> DNA
<213> Escherichia coli K-12 MG1655
<220> yhdg b3261
<222> 677-776
<223> yhdg b3261 U00096 3407917..3408882
<313> 519: 740-759
<400> 519
CAGCTCAGGG AAGACCCTGG ATCTTTCGGG AAATCCAGCA TTATCTGGAC 50
ACTGGGGAGT TGCTGCCCCC GCTGCCTTTG GCAGAGGTTA AGCGCTTGCT 100
<210> 520
<211> 100
<212> DNA
<213> Escherichia coli K-12 MG1655
<220> cspf cspF
<222> 100-199
<223> cspf cspF U00096 1639879..1640091
<313> 520: 129-148
<400> 520
GTTTCAGCGC TCAATCTCCG CGATGCAGAA GAAATTACCA CCGGATTACG 50
CGTGGAATTT TGCCGGATAA ATGGTCTGCG TGGCCCTTCA GCTGCCAATG 100
<210> 521
<211> 100
<212> DNA
<213> Escherichia coli K-12 MG1655
<220> cspf cspF
<222> 5-104
<223> cspf cspF U00096 1639879..1640091
<313> 521: 59-78
<400> 521
CCCGTAAAAT GACAGGAATT GTCAAAACCT TTGACGGCAA AAGCGGCAAG 50
GGTCTTATCA CCCCATCCGA TGGTCGTATC GATGTCCAGC TTCATGTTTC 100
<210> 522
<211> 100
<212> DNA
<213> Escherichia coli K-12 MG1655
<220> cspf cspF
<222> 1-100
<223> cspf cspF U00096 1639879..1640091
<313> 522: 42-61
<400> 522
TTGTCCCGTA AAATGACAGG AATTGTCAAA ACCTTTGACG GCAAAAGCGG 50
CAAGGGTCTT ATCACCCCAT CCGATGGTCG TATCGATGTC CAGCTTCATG 100
<210> 523
<211> 100
<212> DNA
<213> Escherichia coli K-12 MG1655
<220> yhdh yhdH

<222> 876-975
<223> yhdh yhdH U00096 3401121..3402095
<313> 523: 928-947
<400> 523
CCAGGCGGCA AAAGAGATAT CTCTGTCAGA GGCACCGAAC TTTGCCGAGG  50
CCATCATTAA TAACCAGATC CAGGGTCGCA CGCTGGTGAA GGTTAACTAA  100
<210> 524
<211> 100
<212> DNA
<213> Escherichia coli K-12 MG1655
<220> yhdh yhdH
<222> 820-919
<223> yhdh yhdH U00096 3401121..3402095
<313> 524: 866-885
<400> 524
CGCCGCGCAC AAGCCTGGCA GCGACTGGTC GCCGATTTAC CGGAATCATT  50
CTATACCCAG GCGGCAAAAG AGATATCTCT GTCAGAGGCA CCGAACTTTG  100
<210> 525
<211> 100
<212> DNA
<213> Escherichia coli K-12 MG1655
<220> yhdh yhdH
<222> 793-892
<223> yhdh yhdH U00096 3401121..3402095
<313> 525: 813-832
<400> 525
GTGGATTCAG TAATGACGCC ACCAGAACGC CGCGCACAAG CCTGGCAGCG  50
ACTGGTCGCC GATTTACCGG AATCATTCTA TACCCAGGCG GCAAAAGAGA  100
<210> 526
<211> 100
<212> DNA
<213> Escherichia coli K-12 MG1655
<220> cspg b0991
<222> 81-180
<223> cspg b0991 U00096 1050684..1050896
<313> 526: 129-148
<400> 526
CAAAGACGTT TTCGTCCATT TCACCGCCAT CCAGAGCAAT GAATTCCGCA  50
CGCTGAACGA AAATCAGAAA GTTGAATTTT CTATTGAGCA GGGGCAACGT  100
<210> 527
<211> 100
<212> DNA
<213> Escherichia coli K-12 MG1655
<220> cspg b0991
<222> 85-184
<223> cspg b0991 U00096 1050684..1050896
<313> 527: 124-143
<400> 527
GACGTTTTCG TCCATTTCAC CGCCATCCAG AGCAATGAAT TCCGCACGCT  50
GAACGAAAAT CAGAAAGTTG AATTTTCTAT TGAGCAGGGG CAACGTGGCC  100
<210> 528
<211> 100
<212> DNA
<213> Escherichia coli K-12 MG1655
<220> cspg b0991
<222> 69-168
<223> cspg b0991 U00096 1050684..1050896
<313> 528: 112-131
<400> 528
TGATGATGGC AGCAAAGACG TTTTCGTCCA TTTCACCGCC ATCCAGAGCA  50
ATGAATTCCG CACGCTGAAC GAAAATCAGA AAGTTGAATT TTCTATTGAG  100
<210> 529
<211> 100
<212> DNA
<213> Escherichia coli K-12 MG1655
<220> csph b0990
<222> 114-213
<223> csph b0990 U00096 complement(1050186..1050398)
<313> 529: 190-209
<400> 529
TCCCCGCGAC GCAGAAGTGC TTATACCAGG ATTACGTGTG GAATTTTGCC  50

GCGTAAATGG CCTGCGAGGA CCAACAGCGG CAAATGTTTA TCTCTCTTAA 100
<210> 530
<211> 100
<212> DNA
<213> Escherichia coli K-12 MG1655
<220> csph b0990
<222> 53-152
<223> csph b0990 U00096 complement(1050186..1050398)
<313> 530: 99-118
<400> 530
AAGGATTCAT TATCCCCTCC GACGGTCGTA AAGAAGTTCA GGTCCATATT 50
TCCGCATTCA CTCCCCGCGA CGCAGAAGTG CTTATACCAG GATTACGTGT 100
<210> 531
<211> 100
<212> DNA
<213> Escherichia coli K-12 MG1655
<220> csph b0990
<222> 20-119
<223> csph b0990 U00096 complement(1050186..1050398)
<313> 531: 45-64
<400> 531
GAATTGTCAA AACCTTTGAT CGCAAAAGCG GCAAAGGATT CATTATCCCC 50
TCCGACGGTC GTAAAGAAGT TCAGGTCCAT ATTTCCGCAT TCACTCCCCG 100
<210> 532
<211> 100
<212> DNA
<213> Escherichia coli K-12 MG1655
<220> yhdj b3263
<222> 792-891
<223> yhdj b3263 U00096 3409284..3410174
<313> 532: 867-886
<400> 532
CTCTGCGGAA GAACTGGCGA AAGTGAAAAA AAGAAAGACC GGCAACCTGT 50
CAAAACGAAG CCGGTTAAGC GAAGTTGACC CCGATCTCAT TACAAAGTAA 100
<210> 533
<211> 100
<212> DNA
<213> Escherichia coli K-12 MG1655
<220> yhdj b3263
<222> 762-861
<223> yhdj b3263 U00096 3409284..3410174
<313> 533: 813-832
<400> 533
GCTTCGACGG CTGGATGTCG CGTCGCATTA CTCTGCGGAA GAACTGGCGA 50
AAGTGAAAAA AAGAAAGACC GGCAACCTGT CAAAACGAAG CCGGTTAAGC 100
<210> 534
<211> 100
<212> DNA
<213> Escherichia coli K-12 MG1655
<220> yhdj b3263
<222> 674-773
<223> yhdj b3263 U00096 3409284..3410174
<313> 534: 722-741
<400> 534
CTGGTAGCTT TACTACCGGT GCCGTAGCCA TCGCCAGCGG ACGAAAATTC 50
ATTGGTATTG AGATCAACAG CGAGTACATC AAAATGGGGC TTCGACGGCT 100
<210> 535
<211> 100
<212> DNA
<213> Escherichia coli K-12 MG1655
<220> cspi cspI
<222> 102-201
<223> cspi cspI U00096 complement(1636479..1636691)
<313> 535: 119-138
<400> 535
CTCAGCAATT CAGAGCAACG ATTTCAAAAC ATTAACTGAG AATCAGGAAG 50
TTGAATTTGG TATTGAGAAC GGACCTAAAG GTCCTGCCGC TGTTCATGTA 100
<210> 536
<211> 100
<212> DNA
<213> Escherichia coli K-12 MG1655

<220> cspi cspI
<222> 69-168
<223> cspi cspI U00096 complement(1636479..1636691)
<313> 536: 117-136
<400> 536
GAAAGATGGC AGCAAAGATG TGTTTGTCCA TTTCTCAGCA ATTCAGAGCA 50
ACGATTTCAA AACATTAACT GAGAATCAGG AAGTTGAATT TGGTATTGAG 100
<210> 537
<211> 100
<212> DNA
<213> Escherichia coli K-12 MG1655
<220> cspi cspI
<222> 56-155
<223> cspi cspI U00096 complement(1636479..1636691)
<313> 537: 106-125
<400> 537
GTTTCATCAC GCCGAAAGAT GGCAGCAAAG ATGTGTTTGT CCATTTCTCA 50
GCAATTCAGA GCAACGATTT CAAAACATTA ACTGAGAATC AGGAAGTTGA 100
<210> 538
<211> 100
<212> DNA
<213> Escherichia coli K-12 MG1655
<220> yhdm b3293
<222> 327-426
<223> yhdm b3293 U00096 complement(3436342..3436767)
<313> 538: 397-416
<400> 538
CCTTAACGAT GCCTGTTGTG GGACTGCTCA TAGCAGTGTT TATTGTTCGA 50
TTCTTGAAGC TCTTGAACAA GGGGCGAGTG GCGTTAAGAG TGGTTGTTGA 100
<210> 539
<211> 100
<212> DNA
<213> Escherichia coli K-12 MG1655
<220> yhdm b3293
<222> 259-358
<223> yhdm b3293 U00096 complement(3436342..3436767)
<313> 539: 312-331
<400> 539
CAGGAAAGAT TGCAGGAAGT CGAAGCACGG ATAGCCGAGT TGCAGAGTAT 50
GCAGCGTTCC TTGCAACGCC TTAACGATGC CTGTTGTGGG ACTGCTCATA 100
<210> 540
<211> 100
<212> DNA
<213> Escherichia coli K-12 MG1655
<220> yhdm b3293
<222> 214-313
<223> yhdm b3293 U00096 complement(3436342..3436767)
<313> 540: 264-283
<400> 540
ATCGATCCTG AACACCATAC CTGTCAGGAG TCAAAAGGCA TTGTGCAGGA 50
AAGATTGCAG GAAGTCGAAG CACGGATAGC CGAGTTGCAG AGTATGCAGC 100
<210> 541
<211> 100
<212> DNA
<213> Escherichia coli K-12 MG1655
<220> psif b0385
<222> 217-316
<223> psif b0385 U00096 402487..402825
<313> 541: 253-272
<400> 541
GAAAAAAGTT TGACGCCACA GCAGCAAAAG ATGCGCGAAT GCAATAATCA 50
AGCAACACAA CAATCTCTGA AAGGTGATGA TCGTAATAAG TTTATGAGTG 100
<210> 542
<211> 100
<212> DNA
<213> Escherichia coli K-12 MG1655
<220> psif b0385
<222> 197-296
<223> psif b0385 U00096 402487..402825
<313> 542: 225-244
<400> 542

ACAGCAAGTC TGCGCCTGGC GAAAAAAGTT TGACGCCACA GCAGCAAAAG  50
ATGCGCGAAT GCAATAATCA AGCAACACAA CAATCTCTGA AAGGTGATGA  100
<210> 543
<211> 100
<212> DNA
<213> Escherichia coli K-12 MG1655
<220> psif b0385
<222> 143-242
<223> psif b0385 U00096 402487..402825
<313> 543: 169-188
<400> 543
CGCAGGCGTT GAAAGGGGAT GCTCGTAAGA CCTACATGAG TGATTGCCTG  50
AAGAACAGCA AGTCTGCGCC TGGCGAAAAA AGTTTGACGC CACAGCAGCA  100
<210> 544
<211> 100
<212> DNA
<213> Escherichia coli K-12 MG1655
<220> yhdn b3294
<222> 270-369
<223> yhdn b3294 U00096 complement(3436778..3437146)
<313> 544: 321-340
<400> 544
GTCATTACTG GAATTGAAGC TGCGACAAGC TGGATTGAGT ACCGATTTTT  50
TACGCGGCGA TTATGCTGAC AAGTTGTTGG ACAAAATCAA CGATAACTAG  100
<210> 545
<211> 100
<212> DNA
<213> Escherichia coli K-12 MG1655
<220> yhdn b3294
<222> 231-330
<223> yhdn b3294 U00096 complement(3436778..3437146)
<313> 545: 284-303
<400> 545
TCACGATGAT CCGCAATATC AAGAGGTTAG CCGTCGATTG TCATTACTGG  50
AATTGAAGCT GCGACAAGCT GGATTGAGTA CCGATTTTTT ACGCGGCGAT  100
<210> 546
<211> 100
<212> DNA
<213> Escherichia coli K-12 MG1655
<220> yhdn b3294
<222> 187-286
<223> yhdn b3294 U00096 complement(3436778..3437146)
<313> 546: 238-257
<400> 546
CGGAGAGAAG CAATTCAGCT TCTGGATATT CTCAAAGGTA TCCGTCACGA  50
TGATCCGCAA TATCAAGAGG TTAGCCGTCG ATTGTCATTA CTGGAATTGA  100
<210> 547
<211> 100
<212> DNA
<213> Escherichia coli K-12 MG1655
<220> b2853 b2853
<222> 65-164
<223> b2853 b2853 U00096 2991660..2991878
<313> 547: 129-148
<400> 547
ATGAAGTCGT ATCATTAGAT AAACACATCA ACACCAGTGC TACTGATACA  50
GATCAAATAC AAGCATTTAT AGTTTCAACA TGGATGGCGC CTTTTCAAAA  100
<210> 548
<211> 100
<212> DNA
<213> Escherichia coli K-12 MG1655
<220> b2853 b2853
<222> 75-174
<223> b2853 b2853 U00096 2991660..2991878
<313> 548: 104-123
<400> 548
ATCATTAGAT AAACACATCA ACACCAGTGC TACTGATACA GATCAAATAC  50
AAGCATTTAT AGTTTCAACA TGGATGGCGC CTTTTCAAAA TGATATGTAT  100
<210> 549
<211> 100
<212> DNA

<213> Escherichia coli K-12 MG1655
<220> b2853 b2853
<222> 75-174
<223> b2853 b2853 U00096 2991660..2991878
<313> 549: 95-114
<400> 549
ATCATTAGAT AAACACATCA ACACCAGTGC TACTGATACA GATCAAATAC 50
AAGCATTTAT AGTTTCAACA TGGATGGCGC CTTTTCAAAA TGATATGTAT 100
<210> 550
<211> 100
<212> DNA
<213> Escherichia coli K-12 MG1655
<220> yhdp b3246
<222> 2862-2961
<223> yhdp b3246 U00096 complement(3390094..3393054)
<313> 550: 2932-2951
<400> 550
GAGCAAAGTC TCCATTTTGC GCTATCACAT TTCGGGTCCG CTGGACGATC 50
CGCAAATCAA CGAAGTGTTG CGCCAACCGC GTAAAGAAAA AGCGCAATGA 100
<210> 551
<211> 100
<212> DNA
<213> Escherichia coli K-12 MG1655
<220> yhdp b3246
<222> 2789-2888
<223> yhdp b3246 U00096 complement(3390094..3393054)
<313> 551: 2844-2863
<400> 551
CTGCGGCTTT TGCGGTTAAC CCCATTGTTG GCGCGGCAGT GTTTGCCGCC 50
AGTAAAGTGC TGGGGCCGCT GTGGAGCAAA GTCTCCATTT TGCGCTATCA 100
<210> 552
<211> 100
<212> DNA
<213> Escherichia coli K-12 MG1655
<220> yhdp b3246
<222> 2657-2756
<223> yhdp b3246 U00096 complement(3390094..3393054)
<313> 552: 2702-2721
<400> 552
TGCACACCGA CGACACGCTG GTGGATGGCC TGGAGGCGGA TATCGCCATG 50
AAAGGGTCGG TAAATCTGGT ACGTCGCGAC CTGAATATGG AAGCGGTTGT 100
<210> 553
<211> 100
<212> DNA
<213> Escherichia coli K-12 MG1655
<220> b2854 b2855
<222> 318-417
<223> b2854 b2855 U00096 2992021..2992437
<313> 553: 356-375
<400> 553
CCTGGGGACT TATAATGCGG GTTTTGCAAT GGATAACCAA AAAAAGAGAC 50
AGCAATATGC CCCAAAATAT ATATTGTATA TACCAGGCTT AATGAACTAG 100
<210> 554
<211> 100
<212> DNA
<213> Escherichia coli K-12 MG1655
<220> b2854 b2855
<222> 297-396
<223> b2854 b2855 U00096 2992021..2992437
<313> 554: 341-360
<400> 554
TTGTGGAGTA ACGTGGCAAT GCCTGGGGAC TTATAATGCG GGTTTTGCAA 50
TGGATAACCA AAAAAAGAGA CAGCAATATG CCCCAAAATA TATATTGTAT 100
<210> 555
<211> 100
<212> DNA
<213> Escherichia coli K-12 MG1655
<220> b2854 b2855
<222> 284-383
<223> b2854 b2855 U00096 2992021..2992437
<313> 555: 336-355

<400> 555
ACCATTTTTC ACGTTGTGGA GTAACGTGGC AATGCCTGGG GACTTATAAT 50
GCGGGTTTTG CAATGGATAA CCAAAAAAAG AGACAGCAAT ATGCCCCAAA 100
<210> 556
<211> 100
<212> DNA
<213> Escherichia coli K-12 MG1655
<220> bgla b2902
<222> 1268-1367
<223> bgla b2902 U00096 3041682..3043121
<313> 556: 1321-1340
<400> 556
CACCGTGGGG CTGCATCGAC TGCGTGTCGT TCACCACCGG GCAGTACAGC 50
AAACGCTACG GCTTTATCTA TGTGAATAAA CATGACGACG GTACTGGCGA 100
<210> 557
<211> 100
<212> DNA
<213> Escherichia coli K-12 MG1655
<220> bgla b2902
<222> 1240-1339
<223> bgla b2902 U00096 3041682..3043121
<313> 557: 1284-1303
<400> 557
TACGATGGCG TGGATCTGAT GGGCTACACA CCGTGGGGCT GCATCGACTG 50
CGTGTCGTTC ACCACCGGGC AGTACAGCAA ACGCTACGGC TTTATCTATG 100
<210> 558
<211> 100
<212> DNA
<213> Escherichia coli K-12 MG1655
<220> bgla b2902
<222> 1205-1304
<223> bgla b2902 U00096 3041682..3043121
<313> 558: 1233-1252
<400> 558
GCGCCCATAT CGAAGAGATG AAAAAAGCGG TGACTTACGA TGGCGTGGAT 50
CTGATGGGCT ACACACCGTG GGGCTGCATC GACTGCGTGT CGTTCACCAC 100
<210> 559
<211> 100
<212> DNA
<213> Escherichia coli K-12 MG1655
<220> yhdr b3247
<222> 673-772
<223> yhdr b3247 U00096 complement(3393047..3393895)
<313> 559: 716-735
<400> 559
AAGCCGTGGC TCGGTAAATG GATGCAGGAC AATATTGCGC TGGAAACGGC 50
ACAGTTCTCC CTTGAAGGCT GGATGACGAT CGACAAAGGC GATGTAACCG 100
<210> 560
<211> 100
<212> DNA
<213> Escherichia coli K-12 MG1655
<220> yhdr b3247
<222> 568-667
<223> yhdr b3247 U00096 complement(3393047..3393895)
<313> 560: 631-650
<400> 560
CTTACCGGAC AGCACGGCGT GATGCAGGTG CGCATGGATT TGCGCGATGA 50
TGAGGGGTTG TTAAGCAATG GTCGCGTCTG GCTCCAGGCG GATGACATCG 100
<210> 561
<211> 100
<212> DNA
<213> Escherichia coli K-12 MG1655
<220> yhdr b3247
<222> 521-620
<223> yhdr b3247 U00096 complement(3393047..3393895)
<313> 561: 566-585
<400> 561
ACGATCCACG TCGACACCGT GCGGAAGGCC TGGTAAGCCT CTCCAGCCTT 50
ACCGGACAGC ACGGCGTGAT GCAGGTGCGC ATGGATTTGC GCGATGATGA 100
<210> 562
<211> 100

<212> DNA
<213> Escherichia coli K-12 MG1655
<220> bglb b3722
<222> 1314-1413
<223> bglb b3722 U00096 complement(3899917..3901329)
<313> 562: 1361-1380
<400> 562
AAGCCTCACA AGAACACGCA AGAAAAGCTT CGGATGGTAT GCAGAGGTGA  50
TCAAGACGCG GGGGCTGTCA TTAAAAAAAA TAACCATTAA AGCACCTTAA  100
<210> 563
<211> 100
<212> DNA
<213> Escherichia coli K-12 MG1655
<220> bglb b3722
<222> 1292-1391
<223> bglb b3722 U00096 complement(3899917..3901329)
<313> 563: 1333-1352
<400> 563
ATCGTGATGA TAATGGCGAA GGAAGCCTCA CAAGAACACG CAAGAAAAGC  50
TTCGGATGGT ATGCAGAGGT GATCAAGACG CGGGGGCTGT CATTAAAAAA  100
<210> 564
<211> 100
<212> DNA
<213> Escherichia coli K-12 MG1655
<220> bglb b3722
<222> 1209-1308
<223> bglb b3722 U00096 complement(3899917..3901329)
<313> 564: 1227-1246
<400> 564
GGGGTACACC AGTTGGGGGC CAATTGATTT AGTCAGTGCA TCTCATTCAC  50
AAATGTCTAA GCGCTACGGC TTTATTTATG TGGATCGTGA TGATAATGGC  100
<210> 565
<211> 100
<212> DNA
<213> Escherichia coli K-12 MG1655
<220> b2856 b2856
<222> 53-152
<223> b2856 b2856 U00096 complement(2992959..2993114)
<313> 565: 93-112
<400> 565
GGATAATTGG ATTTCTCGGA CATTATCCTG ATTTATATGA GGTTGTTGGG  50
GGCTATAAAG ATCTGAAGAA AGCTATAGCG GAGTGTAACA AATCTACAGC  100
<210> 566
<211> 100
<212> DNA
<213> Escherichia coli K-12 MG1655
<220> b2856 b2856
<222> 9-108
<223> b2856 b2856 U00096 complement(2992959..2993114)
<313> 566: 43-62
<400> 566
AATTAAAATT GTAGTTTCAG ATCAGCAGCC GTTTATGATT GATGGGATAA  50
TTGGATTTCT CGGACATTAT CCTGATTTAT ATGAGGTTGT TGGGGGCTAT  100
<210> 567
<211> 100
<212> DNA
<213> Escherichia coli K-12 MG1655
<220> b2856 b2856
<222> 12-111
<223> b2856 b2856 U00096 complement(2992959..2993114)
<313> 567: 28-47
<400> 567
TAAAATTGTA GTTTCAGATC AGCAGCCGTT TATGATTGAT GGGATAATTG  50
GATTTCTCGG ACATTATCCT GATTTATATG AGGTTGTTGG GGGCTATAAA  100
<210> 568
<211> 100
<212> DNA
<213> Escherichia coli K-12 MG1655
<220> b2857 b2857
<222> 333-432
<223> b2857 b2857 U00096 complement(2993336..2993767)

<313> 568: 397-416
<400> 568
AATCTGCGAA CAAGCCAGAC ATTATTTAGT TGAAAAAATG TTTGAAACAC 50
ATTCTTTGGA TATGAATAAT TCTGTTTTGG CAAGCCCTGA GGACTTATAG 100
<210> 569
<211> 100
<212> DNA
<213> Escherichia coli K-12 MG1655
<220> b2857 b2857
<222> 333-432
<223> b2857 b2857 U00096 complement(2993336..2993767)
<313> 569: 388-407
<400> 569
AATCTGCGAA CAAGCCAGAC ATTATTTAGT TGAAAAAATG TTTGAAACAC 50
ATTCTTTGGA TATGAATAAT TCTGTTTTGG CAAGCCCTGA GGACTTATAG 100
<210> 570
<211> 100
<212> DNA
<213> Escherichia coli K-12 MG1655
<220> b2857 b2857
<222> 291-390
<223> b2857 b2857 U00096 complement(2993336..2993767)
<313> 570: 336-355
<400> 570
TTTAATTAAT AATCATTGTT TCTCCCCTGA CAAGGTTAAC GAAATCTGCG 50
AACAAGCCAG ACATTATTTA GTTGAAAAAA TGTTTGAAAC ACATTCTTTG 100
<210> 571
<211> 100
<212> DNA
<213> Escherichia coli K-12 MG1655
<220> yhdt b3258
<222> 123-222
<223> yhdt b3258 U00096 3405012..3405254
<313> 571: 178-197
<400> 571
CTTTCCGCGC TGGTTTGAGA TGGCCTGCAT CCTGACGCCG CTGCTGTTTA 50
TTGGACTGTG CTGGGCGATG GTGAAATTTA TCTATCGCGA TATCCCACTG 100
<210> 572
<211> 100
<212> DNA
<213> Escherichia coli K-12 MG1655
<220> yhdt b3258
<222> 104-203
<223> yhdt b3258 U00096 3405012..3405254
<313> 572: 128-147
<400> 572
TTGCCCCCGG TTTTACCGGC TTTCCGCGCT GGTTTGAGAT GGCCTGCATC 50
CTGACGCCGC TGCTGTTTAT TGGACTGTGC TGGGCGATGG TGAAATTTAT 100
<210> 573
<211> 100
<212> DNA
<213> Escherichia coli K-12 MG1655
<220> yhdt b3258
<222> 63-162
<223> yhdt b3258 U00096 3405012..3405254
<313> 573: 89-108
<400> 573
GTATCTGGCA GTTTGGTTAG TAGCCGCTTA CTTATCTGGC GTTGCCCCCG 50
GTTTTACCGG CTTTCCGCGC TGGTTTGAGA TGGCCTGCAT CCTGACGCCG 100
<210> 574
<211> 100
<212> DNA
<213> Escherichia coli K-12 MG1655
<220> b2858 b2858
<222> 61-160
<223> b2858 b2858 U00096 complement(2993770..2993991)
<313> 574: 79-98
<400> 574
CTTCTTGAAA CCACCGCAAA AATTCAGAGT ATTGAGGAAA AGAGAGAAGA 50
AAAATTTATT CAAGGTTATT ATGATGGCTA TACAAAAGGC ATTATTGATG 100
<210> 575

<211> 100
<212> DNA
<213> Escherichia coli K-12 MG1655
<220> b2858 b2858
<222> 35-134
<223> b2858 b2858 U00096 complement(2993770..2993991)
<313> 575: 71-90
<400> 575
AAATAGTTTC TCTTAAAGAA AAAATCCTTC TTGAAACCAC CGCAAAAATT 50
CAGAGTATTG AGGAAAAGAG AGAAGAAAAA TTTATTCAAG GTTATTATGA 100
<210> 576
<211> 100
<212> DNA
<213> Escherichia coli K-12 MG1655
<220> b2858 b2858
<222> 18-117
<223> b2858 b2858 U00096 complement(2993770..2993991)
<313> 576: 60-79
<400> 576
TTCTGATGGA TTGAAAGAAA TAGTTTCTCT TAAAGAAAAA ATCCTTCTTG 50
AAACCACCGC AAAAATTCAG AGTATTGAGG AAAAGAGAGA AGAAAAATTT 100
<210> 577
<211> 100
<212> DNA
<213> Escherichia coli K-12 MG1655
<220> yhdu b3264
<222> 68-167
<223> yhdu b3264 U00096 3410258..3410437
<313> 577: 105-124
<400> 577
TGCAGTGGAT TGAACTGCTG GCAACAGAAA CAGACAAATG CCGCAATATG 50
AACTCAGTTA ATCCACTAAA ACTGGTTAAC TGTGACGAAC TGAATTTTCA 100
<210> 578
<211> 100
<212> DNA
<213> Escherichia coli K-12 MG1655
<220> yhdu b3264
<222> 47-146
<223> yhdu b3264 U00096 3410258..3410437
<313> 578: 109-128
<400> 578
TTCTGTTTGA TACTTTACTG ATGCAGTGGA TTGAACTGCT GGCAACAGAA 50
ACAGACAAAT GCCGCAATAT GAACTCAGTT AATCCACTAA AACTGGTTAA 100
<210> 579
<211> 100
<212> DNA
<213> Escherichia coli K-12 MG1655
<220> yhdu b3264
<222> 4-103
<223> yhdu b3264 U00096 3410258..3410437
<313> 579: 57-76
<400> 579
ATTCGCAAGT ATTGGTGGCT CGTCGTTTTC GCTGTCTTCG TTTTTCTGTT 50
TGATACTTTA CTGATGCAGT GGATTGAACT GCTGGCAACA GAAACAGACA 100
<210> 580
<211> 100
<212> DNA
<213> Escherichia coli K-12 MG1655
<220> b2859 b2860
<222> 271-370
<223> b2859 b2860 U00096 complement(2993984..2994409)
<313> 580: 322-341
<400> 580
ATGCAGCAAC GATTTAATTT ATTATTTCCT GATTTTGTTG ACCACATACA 50
ATCGCCGTTA CCACTTGCAT CAACACTTTT AGAGAGAATT ACATTTTATG 100
<210> 581
<211> 100
<212> DNA
<213> Escherichia coli K-12 MG1655
<220> b2859 b2860
<222> 215-314

<223> b2859 b2860 U00096 complement(2993984..2994409)
<313> 581: 251-270
<400> 581
TCATCACATG TGGTTTTTCA ACGTTGTTAC CCTATATAAG GCAACAACCA 50
TTGGCAATGC AGCAACGATT TAATTTATTA TTTCCTGATT TTGTTGACCA 100
<210> 582
<211> 100
<212> DNA
<213> Escherichia coli K-12 MG1655
<220> b2859 b2860
<222> 137-236
<223> b2859 b2860 U00096 complement(2993984..2994409)
<313> 582: 189-208
<400> 582
GGGATTATTT ATCAGCTATA CCGCTGGAAA TAAATGAAAA GGCACGATAC 50
AAACCCGGCA TCGCTAACTA TCACAATATC ATCACATGTG GTTTTTCAAC 100
<210> 583
<211> 100
<212> DNA
<213> Escherichia coli K-12 MG1655
<220> yhdv b3268
<222> 94-193
<223> yhdv b3268 U00096 3416027..3416248
<313> 583: 128-147
<400> 583
CGTCTGGTTC ACACCAATAC CTGTATGAAA GGCACGACTC AGGATAACTG 50
GGAAACGGCT GGGGCTATTG CCGGCGGGGC AGCTGCTGTT GCTGGCCTGA 100
<210> 584
<211> 100
<212> DNA
<213> Escherichia coli K-12 MG1655
<220> yhdv b3268
<222> 59-158
<223> yhdv b3268 U00096 3416027..3416248
<313> 584: 104-123
<400> 584
ATTCGCCCTG CGTTCCGGTT TATGACGATC AGGGTCGTCT GGTTCACACC 50
AATACCTGTA TGAAAGGCAC GACTCAGGAT AACTGGGAAA CGGCTGGGGC 100
<210> 585
<211> 100
<212> DNA
<213> Escherichia coli K-12 MG1655
<220> yhdv b3268
<222> 30-129
<223> yhdv b3268 U00096 3416027..3416248
<313> 585: 47-66
<400> 585
CACCGCATTG CTGGCGGGCT GTGCTCACGA TTCGCCCTGC GTTCCGGTTT 50
ATGACGATCA GGGTCGTCTG GTTCACACCA ATACCTGTAT GAAAGGCACG 100
<210> 586
<211> 100
<212> DNA
<213> Escherichia coli K-12 MG1655
<220> bglf b3723
<222> 1779-1878
<223> bglf b3723 U00096 complement(3901348..3903225)
<313> 586: 1844-1863
<400> 586
GCCGGTATTA ATCAGTAATA GCGATGATTT TACGGACGTA TTACCCCACG 50
GCACGGCGCA GATAAGCGCA GGTGAACCGC TGTTATCCAT CATTCGCTAA 100
<210> 587
<211> 100
<212> DNA
<213> Escherichia coli K-12 MG1655
<220> bglf b3723
<222> 1753-1852
<223> bglf b3723 U00096 complement(3901348..3903225)
<313> 587: 1809-1828
<400> 587
CGCGAGGCCG GATTTGATCT GACGACGCCG GTATTAATCA GTAATAGCGA 50
TGATTTTACG GACGTATTAC CCCACGGCAC GGCGCAGATA AGCGCAGGTG 100

<210> 588
<211> 100
<212> DNA
<213> Escherichia coli K-12 MG1655
<220> bglf b3723
<222> 1726-1825
<223> bglf b3723 U00096 complement(3901348..3903225)
<313> 588: 1775-1794
<400> 588
CTGATTTCTT TTGATATCCC TGCTATTCGC GAGGCCGGAT TTGATCTGAC 50
GACGCCGGTA TTAATCAGTA ATAGCGATGA TTTTACGGAC GTATTACCCC 100
<210> 589
<211> 100
<212> DNA
<213> Escherichia coli K-12 MG1655
<220> yhdw b3269
<222> 819-918
<223> yhdw b3269 U00096 3416786..3417703
<313> 589: 872-891
<400> 589
TGAGCGTAAC GTAGGTTCAG AAAGCCCGCT GAAAATTAAA CGTGGGCAAA 50
ATAATCTCTG GAATAACGGC GGTATTCAGT ACGCACCGCC CGTGCGTTAA 100
<210> 590
<211> 100
<212> DNA
<213> Escherichia coli K-12 MG1655
<220> yhdw b3269
<222> 774-873
<223> yhdw b3269 U00096 3416786..3417703
<313> 590: 827-846
<400> 590
GGCCTATAAC ATCATCAAAC AGGTGGGTAA CTACTCGGAA ATTTTTGAGC 50
GTAACGTAGG TTCAGAAAGC CCGCTGAAAA TTAAACGTGG GCAAAATAAT 100
<210> 591
<211> 100
<212> DNA
<213> Escherichia coli K-12 MG1655
<220> yhdw b3269
<222> 763-862
<223> yhdw b3269 U00096 3416786..3417703
<313> 591: 787-806
<400> 591
GATAATAAAT GGGCCTATAA CATCATCAAA CAGGTGGGTA ACTACTCGGA 50
AATTTTTGAG CGTAACGTAG GTTCAGAAAG CCCGCTGAAA ATTAAACGTG 100
<210> 592
<211> 100
<212> DNA
<213> Escherichia coli K-12 MG1655
<220> bglg b3724
<222> 694-793
<223> bglg b3724 U00096 complement(3903359..3904195)
<313> 592: 733-752
<400> 592
GCAGTAAAAC AAAATTACCC GCAAGCATGG CAATGTGCGG AACGGATCGC 50
CATTTTTATT GGTTTGCAGT ATCAACGTAA AATTTCACCC GCAGAGATTA 100
<210> 593
<211> 100
<212> DNA
<213> Escherichia coli K-12 MG1655
<220> bglg b3724
<222> 644-743
<223> bglg b3724 U00096 complement(3903359..3904195)
<313> 593: 700-719
<400> 593
GTATTCTTGA ACATGCTTCA ATTAACGATA GTGATGAATC ATTACAACAA 50
GCAGTAAAAC AAAATTACCC GCAAGCATGG CAATGTGCGG AACGGATCGC 100
<210> 594
<211> 100
<212> DNA
<213> Escherichia coli K-12 MG1655
<220> bglg b3724

<222> 561-660
<223> bglg b3724 U00096 complement(3903359..3904195)
<313> 594: 617-636
<400> 594
AAAATTTCAG TTCAGCCTTA ATTACCAGGA AGAAAGCTTG AGTTATCAGC 50
GACTGGTTAC ACATCTGAAG TTTTTATCCT GGCGTATTCT TGAACATGCT 100
<210> 595
<211> 100
<212> DNA
<213> Escherichia coli K-12 MG1655
<220> yhdx b3270
<222> 1008-1107
<223> yhdx b3270 U00096 3417753..3418859
<313> 595: 1072-1091
<400> 595
CAAAAACTCC TCTCTTGCCG CCGCTATTGG CTATCCCGAT ATGGTTTCGC 50
TGTTTGCCGG CACCGTGCTG AATCAGACGG GCAAGCCATC GAGACGATAG 100
<210> 596
<211> 100
<212> DNA
<213> Escherichia coli K-12 MG1655
<220> yhdx b3270
<222> 990-1089
<223> yhdx b3270 U00096 3417753..3418859
<313> 596: 1043-1062
<400> 596
CCAGTATCTC AACATCGTCA AAAACTCCTC TCTTGCCGCC GCTATTGGCT 50
ATCCCGATAT GGTTTCGCTG TTTGCCGGCA CCGTGCTGAA TCAGACGGGC 100
<210> 597
<211> 100
<212> DNA
<213> Escherichia coli K-12 MG1655
<220> yhdx b3270
<222> 953-1052
<223> yhdx b3270 U00096 3417753..3418859
<313> 597: 989-1008
<400> 597
CCCAGGCATT GCGGGTGATT ATTCCACCGT TAACCAGCCA GTATCTCAAC 50
ATCGTCAAAA ACTCCTCTCT TGCCGCCGCT ATTGGCTATC CCGATATGGT 100
<210> 598
<211> 100
<212> DNA
<213> Escherichia coli K-12 MG1655
<220> yhdy b3271
<222> 983-1082
<223> yhdy b3271 U00096 3418958..3420064
<313> 598: 1030-1049
<400> 598
GGTTGGGTAT GTCGACGGAA GGGTATGTTT TCGCCGCGCT GATCTACTGG 50
ATCTTCTGTT TCAGCATGTC GCGCTATAGC CAGTATCTGG AAAAACGTTT 100
<210> 599
<211> 100
<212> DNA
<213> Escherichia coli K-12 MG1655
<220> yhdy b3271
<222> 926-1025
<223> yhdy b3271 U00096 3418958..3420064
<313> 599: 960-979
<400> 599
TTATCGGGTT GTTCGATCTT TTCAGTAGCG TTCAGCAGGC AACCGTTGAT 50
CCCGCCTGGT TGGGTATGTC GACGGAAGGG TATGTTTTCG CCGCGCTGAT 100
<210> 600
<211> 100
<212> DNA
<213> Escherichia coli K-12 MG1655
<220> yhdy b3271
<222> 830-929
<223> yhdy b3271 U00096 3418958..3420064
<313> 600: 885-904
<400> 600
AGGGGCTGGT TATTCTGCCA CAGGCGTTGA AGCTGGTAAT TCCTGGGCTG 50

GTAAATACCA TCATCGCACT CTTCAAAGAT ACCAGCCTGG TGATCATTAT 100
<210> 601
<211> 100
<212> DNA
<213> Escherichia coli K-12 MG1655
<220> yhdz b3272
<222> 647-746
<223> yhdz b3272 U00096 3420072..3420830
<313> 601: 668-687
<400> 601
GGGTAATTTT TATGGATCGT GGGGAAATAG TGGAGCAAGC TGCACCTGAT 50
GAATTTTTTG CGCATCCTAA ATCAGAGCGT ACGAGGGCAT TTTTATCGCA 100
<210> 602
<211> 100
<212> DNA
<213> Escherichia coli K-12 MG1655
<220> yhdz b3272
<222> 611-710
<223> yhdz b3272 U00096 3420072..3420830
<313> 602: 627-646
<400> 602
CACATGAGAT GGGGTTTGCA CGAACCGTCG CTGACCGGGT AATTTTTATG 50
GATCGTGGGG AAATAGTGGA GCAAGCTGCA CCTGATGAAT TTTTTGCGCA 100
<210> 603
<211> 100
<212> DNA
<213> Escherichia coli K-12 MG1655
<220> yhdz b3272
<222> 546-645
<223> yhdz b3272 U00096 3420072..3420830
<313> 603: 567-586
<400> 603
GGTGAAAGAG GTGCTGGATA CGATGATTGG GCTGGCGCAG TCGGGTATGA 50
CAATGTTGTG TGTAACACAT GAGATGGGGT TTGCACGAAC CGTCGCTGAC 100
<210> 604
<211> 100
<212> DNA
<213> Escherichia coli K-12 MG1655
<220> bglj b4367
<222> 579-678
<223> bglj b4367 U00096 4601729..4602406
<313> 604: 633-652
<400> 604
GAAACTGGGC GTCAGTTCTG ACGCAGGGTT GTTGGAGGCC GCAGATATTC 50
TGTTATGTAT GCGGCATTGC GAAACAAGTA ATGTGTTGCA TCCCTATTAA 100
<210> 605
<211> 100
<212> DNA
<213> Escherichia coli K-12 MG1655
<220> bglj b4367
<222> 508-607
<223> bglj b4367 U00096 4601729..4602406
<313> 605: 537-556
<400> 605
ATGACACAAA TTGCCGAGCA GCTTAAACGC AATATCAAAA CGATCCGTGC 50
ACATAAATTT AATGTGATGT CGAAACTGGG CGTCAGTTCT GACGCAGGGT 100
<210> 606
<211> 100
<212> DNA
<213> Escherichia coli K-12 MG1655
<220> bglj b4367
<222> 425-524
<223> bglj b4367 U00096 4601729..4602406
<313> 606: 467-486
<400> 606
ACAATCAGTG GTACATTAAC CAAAGCCGGA CGTTAAGCCC GACGGAGAGA 50
GAAATATTGC GCTTTATGTC GCGTGGCTAC TCAATGACAC AAATTGCCGA 100
<210> 607
<211> 100
<212> DNA
<213> Escherichia coli K-12 MG1655

<220> hlpa b0179
<222> 387-486
<223> hlpa b0179 U00096 200482..200967
<313> 607: 449-468
<400> 607
CAGCCAGGAT ATCGATCTGG TTGTTGATGC AAACGCCGTT GCTTACAACA 50
GCAGCGATGT AAAAGACATC ACTGCCGACG TACTGAAACA GGTTAAATAA 100
<210> 608
<211> 100
<212> DNA
<213> Escherichia coli K-12 MG1655
<220> hlpa b0179
<222> 349-448
<223> hlpa b0179 U00096 200482..200967
<313> 608: 396-415
<400> 608
GTTACTCGTA TCCAGACTGC TGTGAAATCC GTTGCCAACA GCCAGGATAT 50
CGATCTGGTT GTTGATGCAA ACGCCGTTGC TTACAACAGC AGCGATGTAA 100
<210> 609
<211> 100
<212> DNA
<213> Escherichia coli K-12 MG1655
<220> hlpa b0179
<222> 338-437
<223> hlpa b0179 U00096 200482..200967
<313> 609: 368-387
<400> 609
GCGGCAAACT GGTTACTCGT ATCCAGACTG CTGTGAAATC CGTTGCCAAC 50
AGCCAGGATA TCGATCTGGT TGTTGATGCA AACGCCGTTG CTTACAACAG 100
<210> 610
<211> 100
<212> DNA
<213> Escherichia coli K-12 MG1655
<220> yhea b3338
<222> 18-117
<223> yhea b3338 U00096 complement(3464434..3464628)
<313> 610: 76-95
<400> 610
TAATGGTATC AGCGATAAAA AAATTCGTCA GGCTGTGCGC CAGTTTTCCC 50
CTCACTCGTT CCAGCAATTA AAAAAATTTA TTCCGGTCGG CAATCAGTGC 100
<210> 611
<211> 100
<212> DNA
<213> Escherichia coli K-12 MG1655
<220> yhea b3338
<222> 15-114
<223> yhea b3338 U00096 complement(3464434..3464628)
<313> 611: 65-84
<400> 611
TTGTAATGGT ATCAGCGATA AAAAAATTCG TCAGGCTGTG CGCCAGTTTT 50
CCCCTCACTC GTTCCAGCAA TTAAAAAAAT TTATTCCGGT CGGCAATCAG 100
<210> 612
<211> 100
<212> DNA
<213> Escherichia coli K-12 MG1655
<220> yhea b3338
<222> 34-133
<223> yhea b3338 U00096 complement(3464434..3464628)
<313> 612: 64-83
<400> 612
AAAAAAATTC GTCAGGCTGT GCGCCAGTTT TCCCCTCACT CGTTCCAGCA 50
ATTAAAAAAA TTTATTCCGG TCGGCAATCA GTGCGGTAAA TGTGTCCGGG 100
<210> 613
<211> 100
<212> DNA
<213> Escherichia coli K-12 MG1655
<220> yheb b3339
<222> 2595-2694
<223> yheb b3339 U00096 complement(3464797..3467490)
<313> 613: 2674-2693
<400> 613

GGGCGGCACT AACCTGTCTC CGGAATTCCA CGGCCTGACT AAAGAACAGG 50
CACCGAAGTA CGCATTTAGC GAAATCTTCG CGCCGTTTAC TAAGCAATAA 100
<210> 614
<211> 100
<212> DNA
<213> Escherichia coli K-12 MG1655
<220> yheb b3339
<222> 2461-2560
<223> yheb b3339 U00096 complement(3464797..3467490)
<313> 614: 2508-2527
<400> 614
ACCACGCCGA TGGTTGGCGT GAACGACGTT CAGGGCGAGG TGTTCTATCT 50
CTCTGATGCT CGTCTGGTCA TGCAGGATGC GCAGAAGCGT AATCTCGGTA 100
<210> 615
<211> 100
<212> DNA
<213> Escherichia coli K-12 MG1655
<220> yheb b3339
<222> 2392-2491
<223> yheb b3339 U00096 complement(3464797..3467490)
<313> 615: 2426-2445
<400> 615
AACCTGCATT CACAATTGAA AGGCCTCCAT CCCAATAAGA GCGATGCAGA 50
AATTGACGCT ATGATGGGTA CCACGCCGAT GGTTGGCGTG AACGACGTTC 100
<210> 616
<211> 100
<212> DNA
<213> Escherichia coli K-12 MG1655
<220> yhed b3324
<222> 1371-1470
<223> yhed b3324 U00096 complement(3451566..3453035)
<313> 616: 1414-1433
<400> 616
TTCAACGTTA GTACATCTCT GGCAGGTGGC AGGGGAGAGT GCCTACTTAT 50
ATCGGGATGA AGCAAATATT TCCCCGGAAA CAACAGTGAA GGGGAAATGA 100
<210> 617
<211> 100
<212> DNA
<213> Escherichia coli K-12 MG1655
<220> yhed b3324
<222> 1329-1428
<223> yhed b3324 U00096 complement(3451566..3453035)
<313> 617: 1371-1390
<400> 617
TCAAAAAAGC CACCACTTGA AAACTGACGG TGTTGTTGGC TTTTCAACGT 50
TAGTACATCT CTGGCAGGTG GCAGGGGAGA GTGCCTACTT ATATCGGGAT 100
<210> 618
<211> 100
<212> DNA
<213> Escherichia coli K-12 MG1655
<220> yhed b3324
<222> 1275-1374
<223> yhed b3324 U00096 complement(3451566..3453035)
<313> 618: 1292-1311
<400> 618
GACAGAACCT TCGGCGGAGT GGCGTCCCTT GTTGGTAGAA AAAATAAAGC 50
AATTTCAAAA AAGCCACCAC TTGAAAACTG ACGGTGTTGT TGGCTTTTCA 100
<210> 619
<211> 100
<212> DNA
<213> Escherichia coli K-12 MG1655
<220> yhee b3325
<222> 717-816
<223> yhee b3325 U00096 3453215..3454030
<313> 619: 779-798
<400> 619
TCAGTTTACA ATTCGTCGCA ACGGCGTACC CCGCTTGATA AATGTTTCCG 50
TCGGGGAACT TACAGGAATG AATGGACTGC GTCATGAAAG GACTCAATAA 100
<210> 620
<211> 100
<212> DNA

<213> Escherichia coli K-12 MG1655
<220> yhee b3325
<222> 698-797
<223> yhee b3325 U00096 3453215..3454030
<313> 620: 739-758
<400> 620
TGCTAACTCA ACAAAGTGCT CAGTTTACAA TTCGTCGCAA CGGCGTACCC  50
CGCTTGATAA ATGTTTCCGT CGGGGAACTT ACAGGAATGA ATGGACTGCG  100
<210> 621
<211> 100
<212> DNA
<213> Escherichia coli K-12 MG1655
<220> yhee b3325
<222> 611-710
<223> yhee b3325 U00096 3453215..3454030
<313> 621: 647-666
<400> 621
GCTTGCTACA ACCAGGGGAT ATCGCCCTCA GGATCAATAA TCTTTCGCTG  50
ACCCACCCTG ATGAGGTTTC ACAGGCATTA AGTTTACTGC TAACTCAACA  100
<210> 622
<211> 100
<212> DNA
<213> Escherichia coli K-12 MG1655
<220> yhef b3326
<222> 1866-1965
<223> yhef b3326 U00096 3454002..3455966
<313> 622: 1940-1959
<400> 622
GATCGACGGG AAATCAAAAG CGCTGGTTGG CTCGGAAGAT TTGCCGGTGC  50
TGGATGAAAA CACGTTCAAC AGTCACGCCC CTGCGCCATC GTCACGGTGA  100
<210> 623
<211> 100
<212> DNA
<213> Escherichia coli K-12 MG1655
<220> yhef b3326
<222> 1835-1934
<223> yhef b3326 U00096 3454002..3455966
<313> 623: 1860-1879
<400> 623
ACACCCGTTA CCGTCAGGAG CAACAACAGC GGATCGACGG GAAATCAAAA  50
GCGCTGGTTG GCTCGGAAGA TTTGCCGGTG CTGGATGAAA ACACGTTCAA  100
<210> 624
<211> 100
<212> DNA
<213> Escherichia coli K-12 MG1655
<220> yhef b3326
<222> 1763-1862
<223> yhef b3326 U00096 3454002..3455966
<313> 624: 1800-1819
<400> 624
ACCTGATGGT ATTTATCCGT CCGACGATTA TCCGTGACGA TGATGTTTAT  50
CGCTCACTGT CAAAAGAGAA ATACACCCGT TACCGTCAGG AGCAACAACA  100
<210> 625
<211> 100
<212> DNA
<213> Escherichia coli K-12 MG1655
<220> yheg b3327
<222> 1379-1478
<223> yheg b3327 U00096 3455976..3457457
<313> 625: 1412-1431
<400> 625
ACAAGGCCTT AATCAAAAAT GGCCTGCAAA AAGTGATAAG CGGTGACACC  50
TCCTGGGATG AGGTTATGCG CGTCGCCAGT GCCACGCTGG AGAGCGAAGC  100
<210> 626
<211> 100
<212> DNA
<213> Escherichia coli K-12 MG1655
<220> yheg b3327
<222> 1269-1368
<223> yheg b3327 U00096 3455976..3457457
<313> 626: 1292-1311

<400> 626
TCAGGGGCGC ATGGCGATCC ACGAAATGAT GGTGGTGACG CCGGAATTAC  50
GGGCCGCTAT TCATGAAAAT GTGGATGAAC AAGCACTGGA GCGACTAGTC  100
<210> 627
<211> 100
<212> DNA
<213> Escherichia coli K-12 MG1655
<220> yheg b3327
<222> 1206-1305
<223> yheg b3327 U00096 3455976..3457457
<313> 627: 1242-1261
<400> 627
TCAGCTCGCG GTGACAACAA TTGGCACTCC CGTAGGCTGC CCTCATTGCC  50
ATCAATCCGG CTATCAGGGG CGCATGGCGA TCCACGAAAT GATGGTGGTG  100
<210> 628
<211> 100
<212> DNA
<213> Escherichia coli K-12 MG1655
<220> bglx b2133
<222> 2182-2281
<223> bglx b2133 U00096 complement(2217712..2220009)
<313> 628: 2245-2264
<400> 628
GAGGCGCTGA AGTTCTGGAA TCAACAGATG AAATATGACG CCGAGCCTGG  50
CAAGTTCAAT GTCTTTATCG GCACTGATTC CGCACGCGTT AAGAAAGGCG  100
<210> 629
<211> 100
<212> DNA
<213> Escherichia coli K-12 MG1655
<220> bglx b2133
<222> 2097-2196
<223> bglx b2133 U00096 complement(2217712..2220009)
<313> 629: 2144-2163
<400> 629
TCGCCCTGTG AAACAGCTGA AAGGCTTTGA GAAAATCACC CTGAAACCGG  50
GCGAAACTCA GACTGTCAGC TTCCCGATCG ATATTGAGGC GCTGAAGTTC  100
<210> 630
<211> 100
<212> DNA
<213> Escherichia coli K-12 MG1655
<220> bglx b2133
<222> 2058-2157
<223> bglx b2133 U00096 complement(2217712..2220009)
<313> 630: 2090-2109
<400> 630
AGTGCAGATG TACTTGCAGG ATGTGACGGC TTCCATGAGT CGCCCTGTGA  50
AACAGCTGAA AGGCTTTGAG AAAATCACCC TGAAACCGGG CGAAACTCAG  100
<210> 631
<211> 100
<212> DNA
<213> Escherichia coli K-12 MG1655
<220> yheh b3331
<222> 318-417
<223> yheh b3331 U00096 3459572..3459988
<313> 631: 385-404
<400> 631
GGAACGCACC ATAACGGTGA CATTACCCAG CGGTCAGACA ACCTCGCTTA  50
CGCGTTATCA AAGCATCGAT AATAAATCCG GGCAGGCACA AGATGATTAA  100
<210> 632
<211> 100
<212> DNA
<213> Escherichia coli K-12 MG1655
<220> yheh b3331
<222> 302-401
<223> yheh b3331 U00096 3459572..3459988
<313> 632: 348-367
<400> 632
AAGACGGCAC GCTTCTGGAA CGCACCATAA CGGTGACATT ACCCAGCGGT  50
CAGACAACCT CGCTTACGCG TTATCAAAGC ATCGATAATA AATCCGGGCA  100
<210> 633
<211> 100

<212> DNA
<213> Escherichia coli K-12 MG1655
<220> yheh b3331
<222> 262-361
<223> yheh b3331 U00096 3459572..3459988
<313> 633: 287-306
<400> 633
GGCGAAGAGT GGAACTGGCG CAGCGATATC CACTCAAGCA AAGACGGCAC 50
GCTTCTGGAA CGCACCATAA CGGTGACATT ACCCAGCGGT CAGACAACCT 100
<210> 634
<211> 100
<212> DNA
<213> Escherichia coli K-12 MG1655
<220> pdhr b0114
<222> 664-763
<223> pdhr b0114 U00096 122092..122856
<313> 634: 698-717
<400> 634
CATCGCCATC TGGCCTTTAT CGAAGAAATT TTGCTCGACA GAAGTCGTGA 50
AGAGAGCCGC CGTGAGCGTT CTCTGCGTCG TCTGGAGCAA CGAAAGAATT 100
<210> 635
<211> 100
<212> DNA
<213> Escherichia coli K-12 MG1655
<220> pdhr b0114
<222> 615-714
<223> pdhr b0114 U00096 122092..122856
<313> 635: 668-687
<400> 635
ATTTGAAGCG ATTATGGCCG GTAAGCCGGA AGAAGCGCGC GAAGCATCGC 50
ATCGCCATCT GGCCTTTATC GAAGAAATTT TGCTCGACAG AAGTCGTGAA 100
<210> 636
<211> 100
<212> DNA
<213> Escherichia coli K-12 MG1655
<220> pdhr b0114
<222> 573-672
<223> pdhr b0114 U00096 122092..122856
<313> 636: 628-647
<400> 636
TCGCGAGATG CTGCCGCTGG TGAGTAGTCA CCGCACCCGC ATATTTGAAG 50
CGATTATGGC CGGTAAGCCG GAAGAAGCGC GCGAAGCATC GCATCGCCAT 100
<210> 637
<211> 100
<212> DNA
<213> Escherichia coli K-12 MG1655
<220> yhei b3332
<222> 489-588
<223> yhei b3332 U00096 3459981..3460568
<313> 637: 556-575
<400> 637
GCAATATGGG GGGTTACAGC GCCGTTTTGC ATTACCTGAG CAGTTAGCGA 50
GAGAAGAGTC GCCAGCTCAG ACGCAGGCCG GGAATAATAA CCATGAATAA 100
<210> 638
<211> 100
<212> DNA
<213> Escherichia coli K-12 MG1655
<220> yhei b3332
<222> 489-588
<223> yhei b3332 U00096 3459981..3460568
<313> 638: 516-535
<400> 638
GCAATATGGG GGGTTACAGC GCCGTTTTGC ATTACCTGAG CAGTTAGCGA 50
GAGAAGAGTC GCCAGCTCAG ACGCAGGCCG GGAATAATAA CCATGAATAA 100
<210> 639
<211> 100
<212> DNA
<213> Escherichia coli K-12 MG1655
<220> yhei b3332
<222> 411-510
<223> yhei b3332 U00096 3459981..3460568

<313> 639: 453-472
<400> 639
CGAGAGCTTT CTTCTGGAAA GTAACAGCGG GGAAAGCCAG GAACTTCCTC  50
TCTCAGTCAC GTTACATCTG CAGACTCAGC AATATGGGGG GTTACAGCGC  100
<210> 640
<211> 100
<212> DNA
<213> Escherichia coli K-12 MG1655
<220> yhej b3333
<222> 885-984
<223> yhej b3333 U00096 3460561..3461544
<313> 640: 943-962
<400> 640
TATCAGCGAC GAACAATCCC AGGGATGGCA AAGCCATATT TTTTTCAACC  50
GTAAACAGCA GAGCGCCCAA ATCTATCGGC GTACGCTGCA ACTCTACTAA  100
<210> 641
<211> 100
<212> DNA
<213> Escherichia coli K-12 MG1655
<220> yhej b3333
<222> 838-937
<223> yhej b3333 U00096 3460561..3461544
<313> 641: 898-917
<400> 641
AGGCATCTCT TTCCCTATAG CCGCTATTTT ACGCTGAGTA CGGAAAGTAT  50
CAGCGACGAA CAATCCCAGG GATGGCAAAG CCATATTTTT TTCAACCGTA  100
<210> 642
<211> 100
<212> DNA
<213> Escherichia coli K-12 MG1655
<220> yhej b3333
<222> 792-891
<223> yhej b3333 U00096 3460561..3461544
<313> 642: 844-863
<400> 642
GCAGCAGGAT TTCTCCGGTG TGAAGCCACT GGTTGCTCAG GTGAAAAGGC  50
ATCTCTTTCC CTATAGCCGC TATTTTACGC TGAGTACGGA AAGTATCAGC  100
<210> 643
<211> 100
<212> DNA
<213> Escherichia coli K-12 MG1655
<220> yhek b3334
<222> 1068-1167
<223> yhek b3334 U00096 3461556..3462722
<313> 643: 1135-1154
<400> 643
CGATCGTTTC TGTGAGTTAA CACAGTCCTG GCTTCCGATG GAAAAAACAG  50
AAAAAGATCC GGTCAGTGGT GTATGGACAG TAAGGAACTC AGGGAAATGA  100
<210> 644
<211> 100
<212> DNA
<213> Escherichia coli K-12 MG1655
<220> yhek b3334
<222> 1058-1157
<223> yhek b3334 U00096 3461556..3462722
<313> 644: 1092-1111
<400> 644
AAGCTAATAT CGATCGTTTC TGTGAGTTAA CACAGTCCTG GCTTCCGATG  50
GAAAAAACAG AAAAAGATCC GGTCAGTGGT GTATGGACAG TAAGGAACTC  100
<210> 645
<211> 100
<212> DNA
<213> Escherichia coli K-12 MG1655
<220> yhek b3334
<222> 987-1086
<223> yhek b3334 U00096 3461556..3462722
<313> 645: 1019-1038
<400> 645
ACTGCAGTTA ATGGAGGCGA ACTATAGCCA GAAGCAAAAG TCGCTGACCT  50
TAAAAATGAG TGCTAAGAGT GAAGCTAATA TCGATCGTTT CTGTGAGTTA  100
<210> 646

<211> 100
<212> DNA
<213> Escherichia coli K-12 MG1655
<220> dica b1571
<222> 263-362
<223> dica b1571 U00096 1645958..1646365
<313> 646: 310-329
<400> 646
AAGAACTAGA GCTCCTTGAG CTGTTTAATG CACTGCCAGA ATCAGAACAG 50
GATACCCAGC TCGCCGAAAT GCGAGCTCGA GTAAAAAACT TCAATAAACT 100
<210> 647
<211> 100
<212> DNA
<213> Escherichia coli K-12 MG1655
<220> dica b1571
<222> 215-314
<223> dica b1571 U00096 1645958..1646365
<313> 647: 249-268
<400> 647
ACAAGCAACC AACACCACCT GTTGAGAAGC CAGTTGCCTT ATCCCCCAAA 50
GAACTAGAGC TCCTTGAGCT GTTTAATGCA CTGCCAGAAT CAGAACAGGA 100
<210> 648
<211> 100
<212> DNA
<213> Escherichia coli K-12 MG1655
<220> dica b1571
<222> 154-253
<223> dica b1571 U00096 1645958..1646365
<313> 648: 197-216
<400> 648
CTTTTTGCCC TCAGTAAAGT ATTGCAATGC TCACCAACAT GGATTCTATT 50
TGGCGATGAA GACAAGCAAC CAACACCACC TGTTGAGAAG CCAGTTGCCT 100
<210> 649
<211> 100
<212> DNA
<213> Escherichia coli K-12 MG1655
<220> yhel b3344
<222> 189-288
<223> yhel b3344 U00096 complement(3472315..3472602)
<313> 649: 268-287
<400> 649
CCGCGGTTTG ACTGGTCAAA TTTCGAACGA CATCATTCTC ATTGACTATA 50
CTGATTTCGT CAGACTTACG GTTAAGCACC CCAGCCAGAT GGCCTGGTGA 100
<210> 650
<211> 100
<212> DNA
<213> Escherichia coli K-12 MG1655
<220> yhel b3344
<222> 117-216
<223> yhel b3344 U00096 complement(3472315..3472602)
<313> 650: 143-162
<400> 650
CGGTAACCGC TACCTTGAAA GTCTGCGTAA TGCCCCCATT AAGGTCTATG 50
CCCTGAACGA AGACCTTATT GCCCGCGGTT TGACTGGTCA AATTTCGAAC 100
<210> 651
<211> 100
<212> DNA
<213> Escherichia coli K-12 MG1655
<220> yhel b3344
<222> 29-128
<223> yhel b3344 U00096 complement(3472315..3472602)
<313> 651: 87-106
<400> 651
GGCTGACGGA TTTTGCTGCG CTGCTGCGTC TGCTCAGTGA AGGAGACGAA 50
CTGCTATTAT TGCAAGATGG CGTAACTGCC GCAGTTGACG GTAACCGCTA 100
<210> 652
<211> 100
<212> DNA
<213> Escherichia coli K-12 MG1655
<220> dicb b1576
<222> 90-189

<223> dicb b1576 U00096 1647633..1647821
<313> 652: 150-169
<400> 652
CATTAACAAG AGAAAACAAG AACGGGAGCT ATTAAATAAA ATATGCATTG 50
TTTCAATGCT GGCTCGTTTA CGTCTGATGC CAAAAGGATG TGCACAATGA 100
<210> 653
<211> 100
<212> DNA
<213> Escherichia coli K-12 MG1655
<220> dicb b1576
<222> 47-146
<223> dicb b1576 U00096 1647633..1647821
<313> 653: 93-112
<400> 653
AAATTGGTGT CACTATCAGT AACCCAGTAT TTACTGAAGA TGCCATTAAC 50
AAGAGAAAAC AAGAACGGGA GCTATTAAAT AAAATATGCA TTGTTTCAAT 100
<210> 654
<211> 100
<212> DNA
<213> Escherichia coli K-12 MG1655
<220> dicb b1576
<222> 45-144
<223> dicb b1576 U00096 1647633..1647821
<313> 654: 91-110
<400> 654
TGAAATTGGT GTCACTATCA GTAACCCAGT ATTTACTGAA GATGCCATTA 50
ACAAGAGAAA ACAAGAACGG GAGCTATTAA ATAAAATATG CATTGTTTCA 100
<210> 655
<211> 100
<212> DNA
<213> Escherichia coli K-12 MG1655
<220> yhem b3345
<222> 261-360
<223> yhem b3345 U00096 complement(3472610..3472969)
<313> 655: 328-347
<400> 655
GTTAGATCCG CAGACACCCT TTGTTGTCGA AGCCACGCCG CTCGAAGCAG 50
ATGCCTTACG CCGCGAACTC GCCAACTACG ATGTTATTTT GAGGTTTTGA 100
<210> 656
<211> 100
<212> DNA
<213> Escherichia coli K-12 MG1655
<220> yhem b3345
<222> 243-342
<223> yhem b3345 U00096 complement(3472610..3472969)
<313> 656: 297-316
<400> 656
TTCACTGCGC GAACGCGGGT TAGATCCGCA GACACCCTTT GTTGTCGAAG 50
CCACGCCGCT CGAAGCAGAT GCCTTACGCC GCGAACTCGC CAACTACGAT 100
<210> 657
<211> 100
<212> DNA
<213> Escherichia coli K-12 MG1655
<220> yhem b3345
<222> 49-148
<223> yhem b3345 U00096 complement(3472610..3472969)
<313> 657: 99-118
<400> 657
GCAGGCCGGG AAGGTTTAGA TGCTTTACTG GCAACTTCCG CATTAACTGA 50
CGATCTGGCT GTCTTCTTTA TAGCTGATGG CGTTTTTCAG CTGCTGCCAG 100
<210> 658
<211> 100
<212> DNA
<213> Escherichia coli K-12 MG1655
<220> dicc b1570
<222> 132-231
<223> dicc b1570 U00096 complement(1645644..1645874)
<313> 658: 205-224
<400> 658
GGAGGCATCT GGCGGGGAGC TTCAGTATGA TCCCAAAGTT TATGATGAAT 50
ATCGTAAGAC GAAGCGGGCG GGGCGGTTGA ACAATGAAAA TCACTCCTGA 100

<210> 659
<211> 100
<212> DNA
<213> Escherichia coli K-12 MG1655
<220> dicc b1570
<222> 132-231
<223> dicc b1570 U00096 complement(1645644..1645874)
<313> 659: 185-204
<400> 659
GGAGGCATCT GGCGGGGAGC TTCAGTATGA TCCCAAAGTT TATGATGAAT  50
ATCGTAAGAC GAAGCGGGCG GGGCGGTTGA ACAATGAAAA TCACTCCTGA  100
<210> 660
<211> 100
<212> DNA
<213> Escherichia coli K-12 MG1655
<220> dicc b1570
<222> 132-231
<223> dicc b1570 U00096 complement(1645644..1645874)
<313> 660: 182-201
<400> 660
GGAGGCATCT GGCGGGGAGC TTCAGTATGA TCCCAAAGTT TATGATGAAT  50
ATCGTAAGAC GAAGCGGGCG GGGCGGTTGA ACAATGAAAA TCACTCCTGA  100
<210> 661
<211> 100
<212> DNA
<213> Escherichia coli K-12 MG1655
<220> yhen b3346
<222> 288-387
<223> yhen b3346 U00096 complement(3472969..3473355)
<313> 661: 362-381
<400> 661
GGGGCTGGCT TCGTCAAACC TTCAGCAGGG ATTTACCTTA AGCGGACTTG  50
GGGCGCTGGC GGAAGCCTCG CTGACCTGTG ACAGGGTGGT ACAGTTCTGA  100
<210> 662
<211> 100
<212> DNA
<213> Escherichia coli K-12 MG1655
<220> yhen b3346
<222> 279-378
<223> yhen b3346 U00096 complement(3472969..3473355)
<313> 662: 306-325
<400> 662
CGGAAGACTG GGGCTGGCTT CGTCAAACCT TCAGCAGGGA TTTACCTTAA  50
GCGGACTTGG GGCGCTGGCG GAAGCCTCGC TGACCTGTGA CAGGGTGGTA  100
<210> 663
<211> 100
<212> DNA
<213> Escherichia coli K-12 MG1655
<220> yhen b3346
<222> 211-310
<223> yhen b3346 U00096 complement(3472969..3473355)
<313> 663: 269-288
<400> 663
CATGGTGTGG CGCTGAATAT CTGCGTAGCG GCAGCATTAC GCCGTGGCGT  50
TGTTGATGAA ACGGAGGCCG GAAGACTGGG GCTGGCTTCG TCAAACCTTC  100
<210> 664
<211> 100
<212> DNA
<213> Escherichia coli K-12 MG1655
<220> b2862 b2862
<222> 201-300
<223> b2862 b2862 U00096 complement(2995711..2996010)
<313> 664: 277-296
<400> 664
AAAAGATAAA CATTACAGAG AAGCCGATGC GGCTTTACTT AATGTTATTT  50
ATCCTACAAA TCTTTCAAAG ATAAACACCA GAAGAAAAGA GCAAGTTTAG  100
<210> 665
<211> 100
<212> DNA
<213> Escherichia coli K-12 MG1655
<220> b2862 b2862

<222> 201-300
<223> b2862 b2862 U00096 complement(2995711..2996010)
<313> 665: 244-263
<400> 665
AAAAGATAAA CATTACAGAG AAGCCGATGC GGCTTTACTT AATGTTATTT 50
ATCCTACAAA TCTTTCAAAG ATAAACACCA GAAGAAAAGA GCAAGTTTAG 100
<210> 666
<211> 100
<212> DNA
<213> Escherichia coli K-12 MG1655
<220> b2862 b2862
<222> 180-279
<223> b2862 b2862 U00096 complement(2995711..2996010)
<313> 666: 194-213
<400> 666
AGAATCCCCA GGTTATCTGG AAAAAGATAA ACATTACAGA GAAGCCGATG 50
CGGCTTTACT TAATGTTATT TATCCTACAA ATCTTTCAAA GATAAACACC 100
<210> 667
<211> 100
<212> DNA
<213> Escherichia coli K-12 MG1655
<220> yheo b3347
<222> 636-735
<223> yheo b3347 U00096 complement(3473355..3474089)
<313> 667: 703-722
<400> 667
GATCAACCAG GTTGCTGACC GCCTGAACAT CTCCAAACAC ACTGTCTATC 50
TCTACATCCG CCAGTTCAAG AGCGGTGATT TCCAGGGGCA AGATAAGTAA 100
<210> 668
<211> 100
<212> DNA
<213> Escherichia coli K-12 MG1655
<220> yheo b3347
<222> 607-706
<223> yheo b3347 U00096 complement(3473355..3474089)
<313> 668: 660-679
<400> 668
GAGAAAGGGA TCTTCGATAT TAAAGATGCG ATCAACCAGG TTGCTGACCG 50
CCTGAACATC TCCAAACACA CTGTCTATCT CTACATCCGC CAGTTCAAGA 100
<210> 669
<211> 100
<212> DNA
<213> Escherichia coli K-12 MG1655
<220> yheo b3347
<222> 543-642
<223> yheo b3347 U00096 complement(3473355..3474089)
<313> 669: 596-615
<400> 669
AGTGAATGCC GATCGCAATG TTTCTAATAA CGCCAAAAAT CGTCAGATCG 50
TGCTGAATCT CTACGAGAAA GGGATCTTCG ATATTAAAGA TGCGATCAAC 100
<210> 670
<211> 100
<212> DNA
<213> Escherichia coli K-12 MG1655
<220> b2863 b2864
<222> 591-690
<223> b2863 b2864 U00096 complement(2996056..2996892)
<313> 670: 636-655
<400> 670
TGCTGTGCAT ATCCGATTTG ACAGCAACTC CCCCGTGATG GCAGATGTGC 50
AGGAAGAAGG CAAGCCGCAG CTGTGGAACC ATAAACAAAA TGATGCTTTA 100
<210> 671
<211> 100
<212> DNA
<213> Escherichia coli K-12 MG1655
<220> b2863 b2864
<222> 478-577
<223> b2863 b2864 U00096 complement(2996056..2996892)
<313> 671: 505-524
<400> 671
AAAAAGGTTC TGCTCACCCT GTGGGATTAT ATGTCTACGC CCGACCAGAT 50

TAAGCGGATG CAGGACGTCA TTAGCAACCC AAAACGACTG GATGGAATTC 100
<210> 672
<211> 100
<212> DNA
<213> Escherichia coli K-12 MG1655
<220> b2863 b2864
<222> 421-520
<223> b2863 b2864 U00096 complement(2996056..2996892)
<313> 672: 475-494
<400> 672
GATAGTTTAT GGGGCTATAT GGCATTAGTA TCAGATCAGG GAAATAGTGT 50
AGCCGCAAAA AAGGTTCTGC TCACCCTGTG GGATTATATG TCTACGCCCG 100
<210> 673
<211> 100
<212> DNA
<213> Escherichia coli K-12 MG1655
<220> b2865 b2865
<222> 681-780
<223> b2865 b2865 U00096 complement(2997158..2997937)
<313> 673: 739-758
<400> 673
TGCGGCATCT GTTCGCCTGC ATTTCCAGAT TCGTTACCGT GCAACGGCAA 50
TTGATCCGCT ACGTTACTTG CCGCCTCAGG GCAGCAAGCC AAAATGCTGA 100
<210> 674
<211> 100
<212> DNA
<213> Escherichia coli K-12 MG1655
<220> b2865 b2865
<222> 674-773
<223> b2865 b2865 U00096 complement(2997158..2997937)
<313> 674: 703-722
<400> 674
GCACGGATGC GGCATCTGTT CGCCTGCATT TCCAGATTCG TTACCGTGCA 50
ACGGCAATTG ATCCGCTACG TTACTTGCCG CCTCAGGGCA GCAAGCCAAA 100
<210> 675
<211> 100
<212> DNA
<213> Escherichia coli K-12 MG1655
<220> b2865 b2865
<222> 628-727
<223> b2865 b2865 U00096 complement(2997158..2997937)
<313> 675: 654-673
<400> 675
AATGGGCAAA GCGTGAAGGC TGGGCAAAAA ATCGCCACTA TGGGGAGCAC 50
GGATGCGGCA TCTGTTCGCC TGCATTTCCA GATTCGTTAC CGTGCAACGG 100
<210> 676
<211> 100
<212> DNA
<213> Escherichia coli K-12 MG1655
<220> yher b3352
<222> 447-546
<223> yher b3352 U00096 complement(3478244..3478798)
<313> 676: 471-490
<400> 676
TCCCATCATT ATTTACTGGG CGAGACGGCA AAGCGCACAG GAGCTGGCGA 50
GCCACGCCAG AGCCTACGGT GACTGGCTGG CAAATCCGCT GTCTCCAGGA 100
<210> 677
<211> 100
<212> DNA
<213> Escherichia coli K-12 MG1655
<220> yher b3352
<222> 363-462
<223> yher b3352 U00096 complement(3478244..3478798)
<313> 677: 396-415
<400> 677
TGACGCGCTG AATCGCTACC CGATGAGCGA TGTGCTGCGC CCCTTTGAAC 50
TGGCGGCGGG CATGTGCCGG ATGCATTGGT TAAGTCCCAT CATTATTTAC 100
<210> 678
<211> 100
<212> DNA
<213> Escherichia coli K-12 MG1655

<220> yher b3352
<222> 249-348
<223> yher b3352 U00096 complement(3478244..3478798)
<313> 678: 302-321
<400> 678
GCTGGACCGG GTATTAAGTC GTGGTTTTGC CAGCGGGCCG GGAGGAAACC  50
AACTGGCGGG AAAGTACTGG CGTAGCGTGA TTACCACCGG CGAGCCGGAA  100
<210> 679
<211> 100
<212> DNA
<213> Escherichia coli K-12 MG1655
<220> b2866 b2867
<222> 2160-2259
<223> b2866 b2867 U00096 2998367..3000625
<313> 679: 2221-2240
<400> 679
TCGTAACGCG GTGAAGATGG CTACCGGTGT TGCAATCAAT ACACTGCCGC  50
TGACGCCAAA ACGGTTATAT GAAGAGTTCC ATCTGGCAGG ATTGATTTGA  100
<210> 680
<211> 100
<212> DNA
<213> Escherichia coli K-12 MG1655
<220> b2866 b2867
<222> 2091-2190
<223> b2866 b2867 U00096 2998367..3000625
<313> 680: 2132-2151
<400> 680
TGAGCCGCAA TCCGCATACG GACATAAGTC ACTGGGTGAG CCACCAATAA  50
TTCCTGTTGC CGCTGCTATT CGTAACGCGG TGAAGATGGC TACCGGTGTT  100
<210> 681
<211> 100
<212> DNA
<213> Escherichia coli K-12 MG1655
<220> b2866 b2867
<222> 2077-2176
<223> b2866 b2867 U00096 2998367..3000625
<313> 681: 2100-2119
<400> 681
TTCGTCGAAA TCAATGAGCC GCAATCCGCA TACGGACATA AGTCACTGGG  50
TGAGCCACCA ATAATTCCTG TTGCCGCTGC TATTCGTAAC GCGGTGAAGA  100
<210> 682
<211> 100
<212> DNA
<213> Escherichia coli K-12 MG1655
<220> yhes b3353
<222> 1807-1906
<223> yhes b3353 U00096 3478926..3480839
<313> 682: 1858-1877
<400> 682
CTGCAACAGC AAGCCAGCGC CAAATCCGGC CTGGAAGAGT GCGAAATGGC  50
ATGGCTGGAA GCCCAGGAGC AGCTTGAGCA GATGCTGCTG GAAGGCCAAA  100
<210> 683
<211> 100
<212> DNA
<213> Escherichia coli K-12 MG1655
<220> yhes b3353
<222> 1791-1890
<223> yhes b3353 U00096 3478926..3480839
<313> 683: 1812-1831
<400> 683
GGAGTTGACC GCCTGCCTGC AACAGCAAGC CAGCGCCAAA TCCGGCCTGG  50
AAGAGTGCGA AATGGCATGG CTGGAAGCCC AGGAGCAGCT TGAGCAGATG  100
<210> 684
<211> 100
<212> DNA
<213> Escherichia coli K-12 MG1655
<220> yhes b3353
<222> 1724-1823
<223> yhes b3353 U00096 3478926..3480839
<313> 684: 1778-1797
<400> 684

ACGCGCAACT GGCGCAGGCG GAAGAGAAAC TCGGCGACAG CGAACTGTAT 50
GACCAGAGCC GTAAAGCGGA GTTGACCGCC TGCCTGCAAC AGCAAGCCAG 100
<210> 685
<211> 100
<212> DNA
<213> Escherichia coli K-12 MG1655
<220> yhet b3354
<222> 916-1015
<223> yhet b3354 U00096 3480839..3481861
<313> 685: 932-951
<400> 685
GGCGGTCATG TTGGCTTTAT TGGCGGTACA TTACTTCATC CGCAAATGTG 50
GCTGGAGTCA CGCATTCCTG ACTGGTTAAC AACGTATCTG GAGGCGAAAT 100
<210> 686
<211> 100
<212> DNA
<213> Escherichia coli K-12 MG1655
<220> yhet b3354
<222> 848-947
<223> yhet b3354 U00096 3480839..3481861
<313> 686: 897-916
<400> 686
TGGATCATCA GGTGATCCCG AAACCGGAAA GTCTCCCCCC GCAGGTGGAG 50
TATCAACTGA CTGAACATGG CGGTCATGTT GGCTTTATTG GCGGTACATT 100
<210> 687
<211> 100
<212> DNA
<213> Escherichia coli K-12 MG1655
<220> yhet b3354
<222> 787-886
<223> yhet b3354 U00096 3480839..3481861
<313> 687: 824-843
<400> 687
CCGATGCTGA ACCGGATCGC CAAACCGACG CTGATTATTC ACGCCAAAGA 50
CGATCCGTTT ATGGATCATC AGGTGATCCC GAAACCGGAA AGTCTCCCCC 100
<210> 688
<211> 100
<212> DNA
<213> Escherichia coli K-12 MG1655
<220> b2868 b2869
<222> 381-480
<223> b2868 b2869 U00096 3001511..3001990
<313> 688: 436-455
<400> 688
CATTACGGAA ATTCGTCGCG GACTGGCGGG AAATCTTTGT CGCTGCACGG 50
GGTATCAGAT GATTGTAAAT ACAGTTCTGG ATTGCGAGAA AACGAAGTAA 100
<210> 689
<211> 100
<212> DNA
<213> Escherichia coli K-12 MG1655
<220> b2868 b2869
<222> 351-450
<223> b2868 b2869 U00096 3001511..3001990
<313> 689: 398-417
<400> 689
GCTGGCGAAA CCACGCGAGA AGCCATTAAC CATTACGGAA ATTCGTCGCG 50
GACTGGCGGG AAATCTTTGT CGCTGCACGG GGTATCAGAT GATTGTAAAT 100
<210> 690
<211> 100
<212> DNA
<213> Escherichia coli K-12 MG1655
<220> b2868 b2869
<222> 268-367
<223> b2868 b2869 U00096 3001511..3001990
<313> 690: 330-349
<400> 690
CATGTTCAGC AGGCTTATGC GAAATCCGGC GCAGTGCAGT GCGGGTTTTG 50
TACGCCTGGC CTGATTATGG CTACCACGGC AATGCTGGCG AAACCACGCG 100
<210> 691
<211> 100
<212> DNA

<213> Escherichia coli K-12 MG1655
<220> yheu b3355
<222> 120-219
<223> yheu b3355 U00096 3481855..3482073
<313> 691: 183-202
<400> 691
CGACGTCAAA CGCCAGCTAC AGTGCGGAGA AGCGGTGCTG GTATGGTCGG  50
AGCTGCACGA AACGGTCAAT ATTATGCCGC GCAGTCAGTT TCGCGAATAA  100
<210> 692
<211> 100
<212> DNA
<213> Escherichia coli K-12 MG1655
<220> yheu b3355
<222> 53-152
<223> yheu b3355 U00096 3481855..3482073
<313> 692: 87-106
<400> 692
AAAGCTTTGT GTTACGTGAA GGCACCGATT ATGGTGAACA TGAGCGTACA  50
CTTGAACAGA AAGTCGCCGA CGTCAAACGC CAGCTACAGT GCGGAGAAGC  100
<210> 693
<211> 100
<212> DNA
<213> Escherichia coli K-12 MG1655
<220> yheu b3355
<222> 0-99
<223> yheu b3355 U00096 3481855..3482073
<313> 693: 20-39
<400> 693
ATGTTGATTC CGTGGCAAGA CCTCTCCCCC GAAACGCTGG AAAATTTAAT  50
TGAAAGCTTT GTGTTACGTG AAGGCACCGA TTATGGTGAA CATGAGCGT  100
<210> 694
<211> 100
<212> DNA
<213> Escherichia coli K-12 MG1655
<220> mepa b2329
<222> 726-825
<223> mepa b2329 U00096 complement(2443580..2444404)
<313> 694: 779-798
<400> 694
GTTTGAACCT CCAAAACCGG GAACAACAAA GCCTGAGAAG AAGACACCGC  50
CTCCGTTGCC GCCTTCCTGC CAGGCGCTAC TGGATGAGCA CGTGATCTAA  100
<210> 695
<211> 100
<212> DNA
<213> Escherichia coli K-12 MG1655
<220> mepa b2329
<222> 711-810
<223> mepa b2329 U00096 complement(2443580..2444404)
<313> 695: 740-759
<400> 695
AGAACTGCAA AGCTGGTTTG AACCTCCAAA ACCGGGAACA ACAAAGCCTG  50
AGAAGAAGAC ACCGCCTCCG TTGCCGCCTT CCTGCCAGGC GCTACTGGAT  100
<210> 696
<211> 100
<212> DNA
<213> Escherichia coli K-12 MG1655
<220> mepa b2329
<222> 655-754
<223> mepa b2329 U00096 complement(2443580..2444404)
<313> 696: 703-722
<400> 696
GATAGTCTGG AGTGTGAAGA TCAACCTTTA CCGCCATCAG GCGATGGTTG  50
CGGGGCAGAA CTGCAAAGCT GGTTTGAACC TCCAAAACCG GGAACAACAA  100
<210> 697
<211> 100
<212> DNA
<213> Escherichia coli K-12 MG1655
<220> yhfa b3357
<222> 268-367
<223> yhfa b3357 U00096 complement(3483051..3483455)
<313> 697: 320-339

<400> 697
ATCGTCACCG GTCGCGACCT GAAAGACGCA GCGGTTGCGC GTGCGGTTGA 50
TCTCTCTGCC GAGAAATATT GTTCAGTGGC GTTGATGCTG GAAAAAGCGG 100
<210> 698
<211> 100
<212> DNA
<213> Escherichia coli K-12 MG1655
<220> yhfa b3357
<222> 199-298
<223> yhfa b3357 U00096 complement(3483051..3483455)
<313> 698: 255-274
<400> 698
GAAGTAAAAT TGACCTCTGA ACGCCGCGAA GAGGCACCAC GCCTGTTTAC 50
GCACATTAAT CTGCATTTTA TCGTCACCGG TCGCGACCTG AAAGACGCAG 100
<210> 699
<211> 100
<212> DNA
<213> Escherichia coli K-12 MG1655
<220> yhfa b3357
<222> 159-258
<223> yhfa b3357 U00096 complement(3483051..3483455)
<313> 699: 215-234
<400> 699
TTCGATCCTG CAAAAAGGGC GTCAGGATGT GGTCGATTGT GAAGTAAAAT 50
TGACCTCTGA ACGCCGCGAA GAGGCACCAC GCCTGTTTAC GCACATTAAT 100
<210> 700
<211> 100
<212> DNA
<213> Escherichia coli K-12 MG1655
<220> csra b2697
<222> 87-186
<223> csra b2697 U00096 complement(2816983..2817168)
<313> 700: 166-185
<400> 700
GGTACGTATT GGCGTAAATG CCCCGAAGGA AGTTTCTGTT CACCGTGAAG 50
AGATCTACCA GCGTATCCAG GCTGAAAAAT CCCAGCAGTC CAGTTACTAA 100
<210> 701
<211> 100
<212> DNA
<213> Escherichia coli K-12 MG1655
<220> csra b2697
<222> 87-186
<223> csra b2697 U00096 complement(2816983..2817168)
<313> 701: 146-165
<400> 701
GGTACGTATT GGCGTAAATG CCCCGAAGGA AGTTTCTGTT CACCGTGAAG 50
AGATCTACCA GCGTATCCAG GCTGAAAAAT CCCAGCAGTC CAGTTACTAA 100
<210> 702
<211> 100
<212> DNA
<213> Escherichia coli K-12 MG1655
<220> csra b2697
<222> 87-186
<223> csra b2697 U00096 complement(2816983..2817168)
<313> 702: 142-161
<400> 702
GGTACGTATT GGCGTAAATG CCCCGAAGGA AGTTTCTGTT CACCGTGAAG 50
AGATCTACCA GCGTATCCAG GCTGAAAAAT CCCAGCAGTC CAGTTACTAA 100
<210> 703
<211> 100
<212> DNA
<213> Escherichia coli K-12 MG1655
<220> yhfc b3365
<222> 1083-1182
<223> yhfc b3365 U00096 3490205..3491386
<313> 703: 1162-1181
<400> 703
GCTTACAGCA AACGGTCTGT ACGCTGTCGT CTTTGTGATG TGCTTCCTGT 50
TAGGTTTCGT CAGCCGTCAC CGTCAGCATA ACACCCTGAC CTCTCATTAA 100
<210> 704
<211> 100

<212> DNA
<213> Escherichia coli K-12 MG1655
<220> yhfc b3365
<222> 1080-1179
<223> yhfc b3365 U00096 3490205..3491386
<313> 704: 1108-1127
<400> 704
ACTGCTTACA GCAAACGGTC TGTACGCTGT CGTCTTTGTG ATGTGCTTCC 50
TGTTAGGTTT CGTCAGCCGT CACCGTCAGC ATAACACCCT GACCTCTCAT 100
<210> 705
<211> 100
<212> DNA
<213> Escherichia coli K-12 MG1655
<220> yhfc b3365
<222> 1049-1148
<223> yhfc b3365 U00096 3490205..3491386
<313> 705: 1078-1097
<400> 705
CGATCGTTGA ACATAGCGGT CCGCAGGCGG CACTGCTTAC AGCAAACGGT 50
CTGTACGCTG TCGTCTTTGT GATGTGCTTC CTGTTAGGTT TCGTCAGCCG 100
<210> 706
<211> 100
<212> DNA
<213> Escherichia coli K-12 MG1655
<220> yhfg b3363
<222> 69-168
<223> yhfg b3363 U00096 complement(3489090..3489257)
<313> 706: 102-121
<400> 706
CAGTCGCCGT CTTGAAGGCG TCGAGATGCC TTTAGTCACT CTTACTGCCG 50
CAGAGGCTTT AGCGCGCCTT GAAGAGCTGA GGAGTCACTA TGAGCGATAA 100
<210> 707
<211> 100
<212> DNA
<213> Escherichia coli K-12 MG1655
<220> yhfg b3363
<222> 29-128
<223> yhfg b3363 U00096 complement(3489090..3489257)
<313> 707: 53-72
<400> 707
CCCGTCTCTG GGAGCTTCAG CGTAATCGTA ATTTTCAGGC CAGTCGCCGT 50
CTTGAAGGCG TCGAGATGCC TTTAGTCACT CTTACTGCCG CAGAGGCTTT 100
<210> 708
<211> 100
<212> DNA
<213> Escherichia coli K-12 MG1655
<220> yhfg b3363
<222> 1-100
<223> yhfg b3363 U00096 complement(3489090..3489257)
<313> 708: 8-27
<400> 708
GTGAAGAAAC TCACCGATAA GCAAAAGTCC CGTCTCTGGG AGCTTCAGCG 50
TAATCGTAAT TTTCAGGCCA GTCGCCGTCT TGAAGGCGTC GAGATGCCTT 100
<210> 709
<211> 100
<212> DNA
<213> Escherichia coli K-12 MG1655
<220> yhfk b3359
<222> 1949-2048
<223> yhfk b3359 U00096 3484440..3486530
<313> 709: 1997-2016
<400> 709
GCGCGGCAGG TACGCTGGAG CAGCATTTAC AGCGGGTTAT TGGTCATCTG 50
AACACCATGC ACACCATTTC GTCGATGGCA TGGCGTCAGC GACCGCATCA 100
<210> 710
<211> 100
<212> DNA
<213> Escherichia coli K-12 MG1655
<220> yhfk b3359
<222> 1909-2008
<223> yhfk b3359 U00096 3484440..3486530

<313> 710: 1946-1965
<400> 710
GCCAATATCA TGGATGCGCC GGAGATGCAG CCGCACGAAG GCGCGGCAGG 50
TACGCTGGAG CAGCATTTAC AGCGGGTTAT TGGTCATCTG AACACCATGC 100
<210> 711
<211> 100
<212> DNA
<213> Escherichia coli K-12 MG1655
<220> yhfk b3359
<222> 1865-1964
<223> yhfk b3359 U00096 3484440..3486530
<313> 711: 1902-1921
<400> 711
GTCAGCAGCG ACTGGAGTAT GACGAACCGG GTAGTTCTGG CGATGCCAAT 50
ATCATGGATG CGCCGGAGAT GCAGCCGCAC GAAGGCGCGG CAGGTACGCT 100
<210> 712
<211> 100
<212> DNA
<213> Escherichia coli K-12 MG1655
<220> yhfl b3370
<222> 69-168
<223> yhfl b3370 U00096 3497085..3497252
<313> 712: 115-134
<400> 712
TATTGAAAAC CGTGATAAGA ACTGCTCTTA CGACTACCTG CTGCACCCGG 50
CAATTTCTAT TTCTAAAATC ATTGGCGGTT GCGGTCCTAC TGCACAGTAA 100
<210> 713
<211> 100
<212> DNA
<213> Escherichia coli K-12 MG1655
<220> yhfl b3370
<222> 69-168
<223> yhfl b3370 U00096 3497085..3497252
<313> 713: 116-135
<400> 713
TATTGAAAAC CGTGATAAGA ACTGCTCTTA CGACTACCTG CTGCACCCGG 50
CAATTTCTAT TTCTAAAATC ATTGGCGGTT GCGGTCCTAC TGCACAGTAA 100
<210> 714
<211> 100
<212> DNA
<213> Escherichia coli K-12 MG1655
<220> yhfl b3370
<222> 68-167
<223> yhfl b3370 U00096 3497085..3497252
<313> 714: 107-126
<400> 714
ATATTGAAAA CCGTGATAAG AACTGCTCTT ACGACTACCT GCTGCACCCG 50
GCAATTTCTA TTTCTAAAAT CATTGGCGGT TGCGGTCCTA CTGCACAGTA 100
<210> 715
<211> 100
<212> DNA
<213> Escherichia coli K-12 MG1655
<220> yhfm b3371
<222> 1290-1389
<223> yhfm b3371 U00096 3497496..3498884
<313> 715: 1354-1373
<400> 715
GATTCCCGGC CTTATCTGCG CCGTCATCGT TATTGCTACT GGTCTGCCTG 50
CTTACGCCTT CTGGGCGAAG CGTAGCCGCC AGCTCAACGC TTTGTCGTAA 100
<210> 716
<211> 100
<212> DNA
<213> Escherichia coli K-12 MG1655
<220> yhfm b3371
<222> 1270-1369
<223> yhfm b3371 U00096 3497496..3498884
<313> 716: 1315-1334
<400> 716
TCAACCTTTG TCTGGGCACC GATTCCCGGC CTTATCTGCG CCGTCATCGT 50
TATTGCTACT GGTCTGCCTG CTTACGCCTT CTGGGCGAAG CGTAGCCGCC 100
<210> 717

<211> 100
<212> DNA
<213> Escherichia coli K-12 MG1655
<220> yhfm b3371
<222> 1220-1319
<223> yhfm b3371 U00096 3497496..3498884
<313> 717: 1254-1273
<400> 717
TCGGGCTGAT GACCACCCTC GCCATTGCGT CAAGCCTCAT TCTGGTCGCC 50
TCAACCTTTG TCTGGGCACC GATTCCCGGC CTTATCTGCG CCGTCATCGT 100
<210> 718
<211> 100
<212> DNA
<213> Escherichia coli K-12 MG1655
<220> yhfn b3372
<222> 945-1044
<223> yhfn b3372 U00096 3498884..3499927
<313> 718: 1016-1035
<400> 718
GATGTTCGTG CCAATGGAGT GGCTCTGCTA CTACCTGTCT ATTTACAAAG 50
ATCACAACCC GGATGAACGC CGCTATTACG GTGGTCTGGT GGAATATTAA 100
<210> 719
<211> 100
<212> DNA
<213> Escherichia coli K-12 MG1655
<220> yhfn b3372
<222> 941-1040
<223> yhfn b3372 U00096 3498884..3499927
<313> 719: 965-984
<400> 719
TCCTGATGTT CGTGCCAATG GAGTGGCTCT GCTACTACCT GTCTATTTAC 50
AAAGATCACA ACCCGGATGA ACGCCGCTAT TACGGTGGTC TGGTGGAATA 100
<210> 720
<211> 100
<212> DNA
<213> Escherichia coli K-12 MG1655
<220> yhfn b3372
<222> 898-997
<223> yhfn b3372 U00096 3498884..3499927
<313> 720: 922-941
<400> 720
TACGCCGAAA TTTCGCAAGG GCTGCACCCG TGGCTGGCAC CGTTCCTGAT 50
GTTCGTGCCA ATGGAGTGGC TCTGCTACTA CCTGTCTATT TACAAAGATC 100
<210> 721
<211> 100
<212> DNA
<213> Escherichia coli K-12 MG1655
<220> yhfo b3373
<222> 351-450
<223> yhfo b3373 U00096 3499890..3500339
<313> 721: 383-402
<400> 721
CCTCGACATG ATCAAGCTGG CGATGGATAT GGCAAAAGAG ATGAACGCGG 50
GTTATACGCT GATTTCCGCC GGCCCACGCG GGCTATCTCA CGCCACCTAA 100
<210> 722
<211> 100
<212> DNA
<213> Escherichia coli K-12 MG1655
<220> yhfo b3373
<222> 267-366
<223> yhfo b3373 U00096 3499890..3500339
<313> 722: 312-331
<400> 722
GATGCCGATT ATCGGCTATA CGCCAGAAAC CAACGGCTAT CCGTATAACA 50
TGATGCTGGG CGATGAACAT ATGCGTCGCG AAAGCCTCGA CATGATCAAG 100
<210> 723
<211> 100
<212> DNA
<213> Escherichia coli K-12 MG1655
<220> yhfo b3373
<222> 171-270

<223> yhfo b3373 U00096 3499890..3500339
<313> 723: 229-248
<400> 723
CGGCATCGAA ATTTGGGGCG GTCGCCCGCA CGCGTTCGCG CCGGACTTAA 50
AAGCGGGCGG CATCAAACAA ATCAAGGCGC TGGCGCAGAC GTATCAGATG 100
<210> 724
<211> 100
<212> DNA
<213> Escherichia coli K-12 MG1655
<220> b2873 b2874
<222> 1299-1398
<223> b2873 b2874 U00096 3008037..3009434
<313> 724: 1362-1381
<400> 724
ATCCCGTGGT GAAACGATTT TCTGTGACGG CACCTTTACA GGCAAAGCCG 50
GGCGAGGTCG TTTCCTGCGA CGCAAACCGT TTGTCCCTCC CGTGCTCTAA 100
<210> 725
<211> 100
<212> DNA
<213> Escherichia coli K-12 MG1655
<220> b2873 b2874
<222> 1273-1372
<223> b2873 b2874 U00096 3008037..3009434
<313> 725: 1324-1343
<400> 725
TGTCAGGGCG CGATTGTCAG AACCTTATCC CGTGGTGAAA CGATTTTCTG 50
TGACGGCACC TTTACAGGCA AAGCCGGGCG AGGTCGTTTC CTGCGACGCA 100
<210> 726
<211> 100
<212> DNA
<213> Escherichia coli K-12 MG1655
<220> b2873 b2874
<222> 1233-1332
<223> b2873 b2874 U00096 3008037..3009434
<313> 726: 1292-1311
<400> 726
CCACGACAAC GCCGACTACT CGCCATGGGA GGGTTTTACC TGTCAGGGCG 50
CGATTGTCAG AACCTTATCC CGTGGTGAAA CGATTTTCTG TGACGGCACC 100
<210> 727
<211> 100
<212> DNA
<213> Escherichia coli K-12 MG1655
<220> yhfp b3374
<222> 306-405
<223> yhfp b3374 U00096 3500404..3500808
<313> 727: 351-370
<400> 727
TACGGTGGAG CTGGTGACGA TGTATATGAA CGAGCCCAGA CTCTATGCCC 50
GCCAGGCGCT GGAACGCTTT CGCGCGCTGC TGCCGGAGGA TGAGAGATGA 100
<210> 728
<211> 100
<212> DNA
<213> Escherichia coli K-12 MG1655
<220> yhfp b3374
<222> 233-332
<223> yhfp b3374 U00096 3500404..3500808
<313> 728: 281-300
<400> 728
TTCCTGGAGA AGGCAAAATG CCGCTGCGGG AACTGATGCG CGATATTATT 50
GAGCGGGGCT ATGAGGGTTA CTGTACGGTG GAGCTGGTGA CGATGTATAT 100
<210> 729
<211> 100
<212> DNA
<213> Escherichia coli K-12 MG1655
<220> yhfp b3374
<222> 206-305
<223> yhfp b3374 U00096 3500404..3500808
<313> 729: 240-259
<400> 729
GCGACGGGGC CAGCGACACG CATTACATTC CTGGAGAAGG CAAAATGCCG 50
CTGCGGGAAC TGATGCGCGA TATTATTGAG CGGGGCTATG AGGGTTACTG 100

<210> 730
<211> 100
<212> DNA
<213> Escherichia coli K-12 MG1655
<220> yhfq b3375
<222> 687-786
<223> yhfq b3375 U00096 3500805..3501590
<313> 730: 756-775
<400> 730
ATTCCTTTGC GGCTGGTCTG CGGGGATGAC ATTACCGCAG GCGATAGCGC 50
AGGGAACGGC GTGCGCGGCG AAAACCATTC AGTACCACGG TGCCTGGTAG 100
<210> 731
<211> 100
<212> DNA
<213> Escherichia coli K-12 MG1655
<220> yhfq b3375
<222> 687-786
<223> yhfq b3375 U00096 3500805..3501590
<313> 731: 715-734
<400> 731
ATTCCTTTGC GGCTGGTCTG CGGGGATGAC ATTACCGCAG GCGATAGCGC 50
AGGGAACGGC GTGCGCGGCG AAAACCATTC AGTACCACGG TGCCTGGTAG 100
<210> 732
<211> 100
<212> DNA
<213> Escherichia coli K-12 MG1655
<220> yhfq b3375
<222> 633-732
<223> yhfq b3375 U00096 3500805..3501590
<313> 732: 672-691
<400> 732
TGAACCGGTG ACGGTTATCG ACACCATGGG TGCCGGAGAT TCGTTCATTG 50
CCGGATTCCT TTGCGGCTGG TCTGCGGGGA TGACATTACC GCAGGCGATA 100
<210> 733
<211> 100
<212> DNA
<213> Escherichia coli K-12 MG1655
<220> b2875 b2875
<222> 1527-1626
<223> b2875 b2875 U00096 complement(3010635..3012260)
<313> 733: 1607-1626
<400> 733
TGATAAAGCC CGGGCGATTG GCGGCGGCGT ACTTGAGGCG TTAATGATGT 50
TGATGCATCA GGGCGTGAAA GCGACAAAAG AAGTGCTGGA AGTGGCTTAA 100
<210> 734
<211> 100
<212> DNA
<213> Escherichia coli K-12 MG1655
<220> b2875 b2875
<222> 1456-1555
<223> b2875 b2875 U00096 complement(3010635..3012260)
<313> 734: 1505-1524
<400> 734
GCAGTGGTTG GTGGTTTTAA AATTGGTGAT ATCGATCCTC GTGGTGAAAC 50
CGCTGATTTC ACCAGCGTTT CTGATAAAGC CCGGGCGATT GGCGGCGGCG 100
<210> 735
<211> 100
<212> DNA
<213> Escherichia coli K-12 MG1655
<220> b2875 b2875
<222> 1421-1520
<223> b2875 b2875 U00096 complement(3010635..3012260)
<313> 735: 1445-1464
<400> 735
CGGGGATGGT GCGTGGCTTG TTGAACGACG GCCTGGCAGT GGTTGGTGGT 50
TTTAAAATTG GTGATATCGA TCCTCGTGGT GAAACCGCTG ATTTCACCAG 100
<210> 736
<211> 100
<212> DNA
<213> Escherichia coli K-12 MG1655
<220> yhfr b3376

<222> 699-798
<223> yhfr b3376 U00096 3501624..3502421
<313> 736: 768-787
<400> 736
CAAAATCGCC TTTGATCAGA ATGACAAACC GGTGCACGTC TCCGAACTCT  50
TCTGCCGCGC CAATCGCATC ACCTTAACTA TTGATAATAA AAGACATTAA  100
<210> 737
<211> 100
<212> DNA
<213> Escherichia coli K-12 MG1655
<220> yhfr b3376
<222> 623-722
<223> yhfr b3376 U00096 3501624..3502421
<313> 737: 656-675
<400> 737
TCGATATCTT TGCCGCCACC CGCCCGCAGG CAAAATGGCT GAAATGCGAA  50
CTGGGCGAAC CGTTGTTTCG CATCAGCAAA ATCGCCTTTG ATCAGAATGA  100
<210> 738
<211> 100
<212> DNA
<213> Escherichia coli K-12 MG1655
<220> yhfr b3376
<222> 564-663
<223> yhfr b3376 U00096 3501624..3502421
<313> 738: 611-630
<400> 738
CACCTATCAG TTATTTCAGG AGCGTTTTGA CACGCGAGTG GTCAGCGACA  50
AAAAGACCAT CGATATCTTT GCCGCCACCC GCCCGCAGGC AAAATGGCTG  100
<210> 739
<211> 100
<212> DNA
<213> Escherichia coli K-12 MG1655
<220> b2876 b2877
<222> 599-698
<223> b2876 b2877 U00096 complement(3012308..3013015)
<313> 739: 632-651
<400> 739
CGATTGCACA AAGCGAGCTC CTTCAACCGC TGCAACAACA CGACGTAGAG  50
GCAATCTGGC TGGGCGATAT ACAAGAACAT CCTGCAATCG CGCGCAGATT  100
<210> 740
<211> 100
<212> DNA
<213> Escherichia coli K-12 MG1655
<220> b2876 b2877
<222> 539-638
<223> b2876 b2877 U00096 complement(3012308..3013015)
<313> 740: 560-579
<400> 740
ACGTACCGCA AGGTTGTCGG CGAGTCTGGT TCATTAACCG TTTTTCTCAA  50
TGTGAGAATG CGATTGCACA AAGCGAGCTC CTTCAACCGC TGCAACAACA  100
<210> 741
<211> 100
<212> DNA
<213> Escherichia coli K-12 MG1655
<220> b2876 b2877
<222> 491-590
<223> b2876 b2877 U00096 complement(3012308..3013015)
<313> 741: 534-553
<400> 741
TGAGCGATCT CGTTGCGCTG GTTCGCCACC CTCAGGGGGC GTTTAAAAAC  50
GTACCGCAAG GTTGTCGGCG AGTCTGGTTC ATTAACCGTT TTTCTCAATG  100
<210> 742
<211> 100
<212> DNA
<213> Escherichia coli K-12 MG1655
<220> yhfs b3377
<222> 906-1005
<223> yhfs b3377 U00096 complement(3502573..3503658)
<313> 742: 946-965
<400> 742
CTTGCCTTAC CCGGTGGGTG CAGAGTCGAA ATATGAAATC CCGCCGCTCT  50

TTTATCGCCT TTCCGGAACG TTTCGCCAGG CGAATCCACA ATCAGAACAT 100
<210> 743
<211> 100
<212> DNA
<213> Escherichia coli K-12 MG1655
<220> yhfs b3377
<222> 845-944
<223> yhfs b3377 U00096 complement(3502573..3503658)
<313> 743: 897-916
<400> 743
TTGTCGAGTT TCATCAGCCG ATTGCCGCCA GAGTGCTGGA AGAGGCGCAA 50
AAGCGCGGTG CCTTGCCTTA CCCGGTGGGT GCAGAGTCGA AATATGAAAT 100
<210> 744
<211> 100
<212> DNA
<213> Escherichia coli K-12 MG1655
<220> yhfs b3377
<222> 828-927
<223> yhfs b3377 U00096 complement(3502573..3503658)
<313> 744: 864-883
<400> 744
GCAGTCGAAG GTGTTGATTG TCGAGTTTCA TCAGCCGATT GCCGCCAGAG 50
TGCTGGAAGA GGCGCAAAAG CGCGGTGCCT TGCCTTACCC GGTGGGTGCA 100
<210> 745
<211> 100
<212> DNA
<213> Escherichia coli K-12 MG1655
<220> yhft b3378
<222> 1152-1251
<223> yhft b3378 U00096 complement(3503670..3504974)
<313> 745: 1189-1208
<400> 745
TTATACCGGA TTCTCTATCG CGGTTGCCAT TTACTTCCTC AACGAATCCC 50
TGGGCCGTCC GGTACAGAAA ATGGCGGCAC CGGTCGTGGC AGTAATGATC 100
<210> 746
<211> 100
<212> DNA
<213> Escherichia coli K-12 MG1655
<220> yhft b3378
<222> 1092-1191
<223> yhft b3378 U00096 complement(3503670..3504974)
<313> 746: 1140-1159
<400> 746
TATGCTGATG GAAGTGGCGC TGCTGGTCGG TTCGATTTTC GCAGCAATTA 50
AGATGGCGGG TTATACCGGA TTCTCTATCG CGGTTGCCAT TTACTTCCTC 100
<210> 747
<211> 100
<212> DNA
<213> Escherichia coli K-12 MG1655
<220> yhft b3378
<222> 1053-1152
<223> yhft b3378 U00096 complement(3503670..3504974)
<313> 747: 1085-1104
<400> 747
GGTGCGTAAT GCGTCGGATA ACATCCGTAA CGCCATGAAT ATGCTGATGG 50
AAGTGGCGCT GCTGGTCGGT TCGATTTTCG CAGCAATTAA GATGGCGGGT 100
<210> 748
<211> 100
<212> DNA
<213> Escherichia coli K-12 MG1655
<220> b2878 b2878
<222> 2986-3085
<223> b2878 b2878 U00096 3014081..3017179
<313> 748: 3043-3062
<400> 748
ATCGACAGCA AAGGTCAGTT TAACAACGTA CCACCGGAGC TGAACGATAT 50
GTGCCGCATC ATCAGCCATG TCCACCAGCA TCATCATTAT CTGCTGGGCC 100
<210> 749
<211> 100
<212> DNA
<213> Escherichia coli K-12 MG1655

<220> b2878 b2878
<222> 2978-3077
<223> b2878 b2878 U00096 3014081..3017179
<313> 749: 3013-3032
<400> 749
TGTTAAACAT CGACAGCAAA GGTCAGTTTA ACAACGTACC ACCGGAGCTG 50
AACGATATGT GCCGCATCAT CAGCCATGTC CACCAGCATC ATCATTATCT 100
<210> 750
<211> 100
<212> DNA
<213> Escherichia coli K-12 MG1655
<220> b2878 b2878
<222> 2905-3004
<223> b2878 b2878 U00096 3014081..3017179
<313> 750: 2960-2979
<400> 750
GATAACAGCA GCAACCCAGG CTTCCTTGTG GAAGATTGCC GGGTACGAGT 50
ACGTCTGAAT AACCAAAGCT GGGTGTTAAA CATCGACAGC AAAGGTCAGT 100
<210> 751
<211> 100
<212> DNA
<213> Escherichia coli K-12 MG1655
<220> yhfu b3379
<222> 294-393
<223> yhfu b3379 U00096 complement(3504986..3505378)
<313> 751: 363-382
<400> 751
GCATATCGCC AAAATGATCG CTGAAGGAAA AGTGGCGTTT GGCCTTTCCG 50
TTGAGCACGT CGAACACGCG ATTCCGATGC TGATTAACCA TCTGAAATAA 100
<210> 752
<211> 100
<212> DNA
<213> Escherichia coli K-12 MG1655
<220> yhfu b3379
<222> 273-372
<223> yhfu b3379 U00096 complement(3504986..3505378)
<313> 752: 333-352
<400> 752
AGGCATTAAA GCGAAAGACG AGCATATCGC CAAAATGATC GCTGAAGGAA 50
AAGTGGCGTT TGGCCTTTCC GTTGAGCACG TCGAACACGC GATTCCGATG 100
<210> 753
<211> 100
<212> DNA
<213> Escherichia coli K-12 MG1655
<220> yhfu b3379
<222> 261-360
<223> yhfu b3379 U00096 complement(3504986..3505378)
<313> 753: 288-307
<400> 753
CATTGCCAAA CCAGGCATTA AAGCGAAAGA CGAGCATATC GCCAAAATGA 50
TCGCTGAAGG AAAAGTGGCG TTTGGCCTTT CCGTTGAGCA CGTCGAACAC 100
<210> 754
<211> 100
<212> DNA
<213> Escherichia coli K-12 MG1655
<220> b2879 b2879
<222> 1296-1395
<223> b2879 b2879 U00096 3017116..3018510
<313> 754: 1350-1369
<400> 754
CTATGAAGAC CGTCAGTTTA ACTTCGATTG CGATTCCATT TATGCACAAG 50
CCAGAAAAGC CGCTGCCAGT ATGTGGCGTC GGATGGATGC GCTGGCATAA 100
<210> 755
<211> 100
<212> DNA
<213> Escherichia coli K-12 MG1655
<220> b2879 b2879
<222> 1241-1340
<223> b2879 b2879 U00096 3017116..3018510
<313> 755: 1274-1293
<400> 755

CTTTCGGTAT GGGCTCAGGC AGCGTTCACA GCGTGATGGT CAATGGTGTG 50
ATGGTCTATG AAGACCGTCA GTTTAACTTC GATTGCGATT CCATTTATGC 100
<210> 756
<211> 100
<212> DNA
<213> Escherichia coli K-12 MG1655
<220> b2879 b2879
<222> 1153-1252
<223> b2879 b2879 U00096 3017116..3018510
<313> 756: 1192-1211
<400> 756
CTGGAAGCCG GTTACAAAGC TGATTTAACC ATTTGCGATT ACAACTCGCC 50
GACGCCGCTG CTGGCAGACA ATATCGCCGG GCATATCGCT TTCGGTATGG 100
<210> 757
<211> 100
<212> DNA
<213> Escherichia coli K-12 MG1655
<220> yhfv b3380
<222> 769-868
<223> yhfv b3380 U00096 complement(3505350..3506228)
<313> 757: 818-837
<400> 757
GGCTATGACT ATTTACTAAC CACCTTTATT CCGCAATTGC GCCAGTCAGG 50
ATTCAGTCAG GCCGATGTGG ATGTGATGTT ACGTGAAAAT CCCTCTCAAT 100
<210> 758
<211> 100
<212> DNA
<213> Escherichia coli K-12 MG1655
<220> yhfv b3380
<222> 697-796
<223> yhfv b3380 U00096 complement(3505350..3506228)
<313> 758: 737-756
<400> 758
GGGTTGCTGA ACCGCGTCAT GCTGTCGATG GATATTACGC GCCGCTCCCA 50
TTTAAAAGCC AACGGTGGTT ATGGCTATGA CTATTTACTA ACCACCTTTA 100
<210> 759
<211> 100
<212> DNA
<213> Escherichia coli K-12 MG1655
<220> yhfv b3380
<222> 612-711
<223> yhfv b3380 U00096 complement(3505350..3506228)
<313> 759: 651-670
<400> 759
GTACGTGCAG TTCGACACCA TCGGCAAGAA CAGTTACTAC CCGGACGAAA 50
AGCGTATTGC GATGCTTCAT GCGCTACGCG ACCGTGGGTT GCTGAACCGC 100
<210> 760
<211> 100
<212> DNA
<213> Escherichia coli K-12 MG1655
<220> yhfw b3381
<222> 1128-1227
<223> yhfw b3381 U00096 complement(3506225..3507451)
<313> 760: 1191-1210
<400> 760
TGATGTGGGG GCTACCGTGT GTGAATTTTT CCGCGCGCCA CCGCCACAAA 50
ATGGTCGCTC TTTTCTTTCC TCCCTCCGGT TTGCAGGAGA CACCCTATGA 100
<210> 761
<211> 100
<212> DNA
<213> Escherichia coli K-12 MG1655
<220> yhfw b3381
<222> 1107-1206
<223> yhfw b3381 U00096 complement(3506225..3507451)
<313> 761: 1134-1153
<400> 761
CGGTGTGCGC ACCACGCTTT CTGATGTGGG GGCTACCGTG TGTGAATTTT 50
TCCGCGCGCC ACCGCCACAA AATGGTCGCT CTTTTCTTTC CTCCCTCCGG 100
<210> 762
<211> 100
<212> DNA

<213> Escherichia coli K-12 MG1655
<220> yhfw b3381
<222> 1044-1143
<223> yhfw b3381 U00096 complement(3506225..3507451)
<313> 762: 1075-1094
<400> 762
CCACCATACC CGCGAAGTGG TGCCAGTGCT GGTTTATCAG CAAGGGATGA 50
TCGCTACGCA GCTCGGTGTG CGCACCACGC TTTCTGATGT GGGGGCTACC 100
<210> 763
<211> 100
<212> DNA
<213> Escherichia coli K-12 MG1655
<220> yhfx b3382
<222> 1015-1114
<223> yhfx b3382 U00096 complement(3507451..3508614)
<313> 763: 1065-1084
<400> 763
GAGTTTCCGG TAAGCAGTGC AGTGGTGCTC TGTTTTCGCA CGCAGATTTT 50
TGTCACCCGT AGCGATGTGG TGCTGGTGTC CGGTATTCAT CGTGGCGAAC 100
<210> 764
<211> 100
<212> DNA
<213> Escherichia coli K-12 MG1655
<220> yhfx b3382
<222> 975-1074
<223> yhfx b3382 U00096 complement(3507451..3508614)
<313> 764: 1021-1040
<400> 764
GGATGACAGC AGTATCGACT ACACCCTGCC GCTGGCAGGC GAGTTTCCGG 50
TAAGCAGTGC AGTGGTGCTC TGTTTTCGCA CGCAGATTTT TGTCACCCGT 100
<210> 765
<211> 100
<212> DNA
<213> Escherichia coli K-12 MG1655
<220> yhfx b3382
<222> 931-1030
<223> yhfx b3382 U00096 complement(3507451..3508614)
<313> 765: 980-999
<400> 765
ACGCCAGAAA ATCAAAAGAT TACTGAAACC AATCTCAAAA CTGTGGATGA 50
CAGCAGTATC GACTACACCC TGCCGCTGGC AGGCGAGTTT CCGGTAAGCA 100
<210> 766
<211> 100
<212> DNA
<213> Escherichia coli K-12 MG1655
<220> yhfy b3383
<222> 306-405
<223> yhfy b3383 U00096 complement(3508698..3509102)
<313> 766: 361-380
<400> 766
GTTGTTGAAG GAATTCGCAC TGGAAGTTAA CCCGTGTGAA GAAGGCTATT 50
TGCTGGCCAA CCTTTATGGA TTATGGATGG CTGCTAACGA AGAGGTTTGA 100
<210> 767
<211> 100
<212> DNA
<213> Escherichia coli K-12 MG1655
<220> yhfy b3383
<222> 306-405
<223> yhfy b3383 U00096 complement(3508698..3509102)
<313> 767: 332-351
<400> 767
GTTGTTGAAG GAATTCGCAC TGGAAGTTAA CCCGTGTGAA GAAGGCTATT 50
TGCTGGCCAA CCTTTATGGA TTATGGATGG CTGCTAACGA AGAGGTTTGA 100
<210> 768
<211> 100
<212> DNA
<213> Escherichia coli K-12 MG1655
<220> yhfy b3383
<222> 236-335
<223> yhfy b3383 U00096 complement(3508698..3509102)
<313> 768: 266-285

<400> 768
TGGATAACGA GTTGCTGGCA GAACTGGCGC AATCCAGCCA CTGGCAAGCC 50
GTTGTGCAAT TGCATCAGGT GTTGTTGAAG GAATTCGCAC TGGAAGTTAA 100
<210> 769
<211> 100
<212> DNA
<213> Escherichia coli K-12 MG1655
<220> yhfz b3384
<222> 588-687
<223> yhfz b3384 U00096 complement(3509077..3509763)
<313> 769: 650-669
<400> 769
TTACCCAATG CAACAACTGC TGCGTGCCGT TGTAGATAAA CACGCCCTGC 50
TCGCCCATCA ACAACGGGTA GTGAGTGGGG AACAGGAACC GAGTTATTAA 100
<210> 770
<211> 100
<212> DNA
<213> Escherichia coli K-12 MG1655
<220> yhfz b3384
<222> 576-675
<223> yhfz b3384 U00096 complement(3509077..3509763)
<313> 770: 625-644
<400> 770
GCGAGTCGAT GATTACCCAA TGCAACAACT GCTGCGTGCC GTTGTAGATA 50
AACACGCCCT GCTCGCCCAT CAACAACGGG TAGTGAGTGG GGAACAGGAA 100
<210> 771
<211> 100
<212> DNA
<213> Escherichia coli K-12 MG1655
<220> yhfz b3384
<222> 531-630
<223> yhfz b3384 U00096 complement(3509077..3509763)
<313> 771: 581-600
<400> 771
AGACGATCCG CGATTTTTAC AGGCCACCGA AGCCGTGGTC CTGACGCGAG 50
TCGATGATTA CCCAATGCAA CAACTGCTGC GTGCCGTTGT AGATAAACAC 100
<210> 772
<211> 100
<212> DNA
<213> Escherichia coli K-12 MG1655
<220> yhga b3412
<222> 780-879
<223> yhga b3412 U00096 3540803..3541681
<313> 772: 837-856
<400> 772
GCATATAGCC AAAATAATGC TTGAATCCGG AGTTCCTCTT GCAGACATCA 50
TGCGCTTTAC CGGGCTGTCA GAAGAAGAGT TGGCTGCGGC GAGTCAGTAA 100
<210> 773
<211> 100
<212> DNA
<213> Escherichia coli K-12 MG1655
<220> yhga b3412
<222> 741-840
<223> yhga b3412 U00096 3540803..3541681
<313> 773: 773-792
<400> 773
GGAAAGATTG CGGCAGGAGG GGGAACAATC CAAAGCCCTG CATATAGCCA 50
AAATAATGCT TGAATCCGGA GTTCCTCTTG CAGACATCAT GCGCTTTACC 100
<210> 774
<211> 100
<212> DNA
<213> Escherichia coli K-12 MG1655
<220> yhga b3412
<222> 691-790
<223> yhga b3412 U00096 3540803..3541681
<313> 774: 724-743
<400> 774
GGCGTTGCCG AACGTTCACC GAAACACAAG GAGAGTTTAA TGACTATTGC 50
GGAAAGATTG CGGCAGGAGG GGGAACAATC CAAAGCCCTG CATATAGCCA 100
<210> 775
<211> 100

<212> DNA
<213> Escherichia coli K-12 MG1655
<220> yi91a b0256
<222> 295-394
<223> yi91a b0256 U00096 269466..269870
<313> 775: 351-370
<400> 775
GCTACTGTAG ATTCAATTGG TCAACGCAAC AGTTATGTGA AAACATGGGG 50
TTGCGGAGGT TTTTTGAATG AGACGAACAT TTACAGCAGA GGAAAAAGCC 100
<210> 776
<211> 100
<212> DNA
<213> Escherichia coli K-12 MG1655
<220> yi91a b0256
<222> 186-285
<223> yi91a b0256 U00096 269466..269870
<313> 776: 219-238
<400> 776
GCAGGGCAAA ACACCAAAAG CCTCCCCCAT TACCCCGGAA CAAATTGAAA 50
TCCGTGAGCT CAGGAAAAAG CTACAACGTA TTGAAATGGA AAATGAAATA 100
<210> 777
<211> 100
<212> DNA
<213> Escherichia coli K-12 MG1655
<220> yi91a b0256
<222> 101-200
<223> yi91a b0256 U00096 269466..269870
<313> 777: 154-173
<400> 777
ACTACACCGT GGCAGATGCA GCCAGCGCTA TGGATGTCGG CCTTTCCACA 50
ATGACGCGAT GGGTGAAACA ATTACGTGAT GAACGGCAGG GCAAAACACC 100
<210> 778
<211> 100
<212> DNA
<213> Escherichia coli K-12 MG1655
<220> yhge b3403
<222> 1626-1725
<223> yhge b3403 U00096 complement(3528353..3530077)
<313> 778: 1682-1701
<400> 778
ACTCACAGCT GCGAAGGATG GAAAACTGGG GACGAAGCCT AAAGCGTGGC 50
GCGATCTTGT AGTGGATGGT GAAAGGCTGA ATGTGAATCT GAATGAGTGA 100
<210> 779
<211> 100
<212> DNA
<213> Escherichia coli K-12 MG1655
<220> yhge b3403
<222> 1613-1712
<223> yhge b3403 U00096 complement(3528353..3530077)
<313> 779: 1640-1659
<400> 779
CGAAAGAAAA ATTACTCACA GCTGCGAAGG ATGGAAAACT GGGGACGAAG 50
CCTAAAGCGT GGCGCGATCT TGTAGTGGAT GGTGAAAGGC TGAATGTGAA 100
<210> 780
<211> 100
<212> DNA
<213> Escherichia coli K-12 MG1655
<220> yhge b3403
<222> 1554-1653
<223> yhge b3403 U00096 complement(3528353..3530077)
<313> 780: 1585-1604
<400> 780
TGACTCAGAC AGAAAAGAAT GGGACGCGCT TGATATGAGT TTACTTCCGA 50
ACGAAATAAC GAAAGAAAAA TTACTCACAG CTGCGAAGGA TGGAAAACTG 100
<210> 781
<211> 100
<212> DNA
<213> Escherichia coli K-12 MG1655
<220> yi91b b4284
<222> 166-265
<223> yi91b b4284 U00096 4504729..4505031

<313> 781: 211-230
<400> 781
AAACAACTGC GTGATGAGCG TCAGGGCAAA ACACCAAAAG CCTCTCCGAT 50
AACACCAGAA CAAATCGAAA TACGTAAGCT GAGGAAAAAG CTACAACGGA 100
<210> 782
<211> 100
<212> DNA
<213> Escherichia coli K-12 MG1655
<220> yi91b b4284
<222> 112-211
<223> yi91b b4284 U00096 4504729..4505031
<313> 782: 165-184
<400> 782
GCAGATGCCG CGAAAGCTAT GGATGTTGGC CTTTCCACAA TGACAAGATG 50
GGTCAAACAA CTGCGTGATG AGCGTCAGGG CAAAACACCA AAAGCCTCTC 100
<210> 783
<211> 100
<212> DNA
<213> Escherichia coli K-12 MG1655
<220> yi91b b4284
<222> 70-169
<223> yi91b b4284 U00096 4504729..4505031
<313> 783: 135-154
<400> 783
CGCGAATCCG CTCAACTGGT TGTTGACCAG AAATACACGG TGGCAGATGC 50
CGCGAAAGCT ATGGATGTTG GCCTTTCCAC AATGACAAGA TGGGTCAAAC 100
<210> 784
<211> 100
<212> DNA
<213> Escherichia coli K-12 MG1655
<220> yhgf b3408
<222> 2098-2197
<223> yhgf b3408 U00096 3535122..3537344
<313> 784: 2123-2142
<400> 784
GGCGGCGGTA ATGAACGCCC GCAAAACAAC CGCCCGGCAG CCAAACCACG 50
CGGTCGTGAA GCGCAGCCTG CCGGTAATAG CGCGATGATG GATGCGCTGG 100
<210> 785
<211> 100
<212> DNA
<213> Escherichia coli K-12 MG1655
<220> yhgf b3408
<222> 2010-2109
<223> yhgf b3408 U00096 3535122..3537344
<313> 785: 2053-2072
<400> 785
GAAAGTGCTG GAAGTGGATC TTCAGCGTAA ACGTATCGCC CTGACTATGC 50
GCCTGGATGA GCAGCCTGGC GAAACCAACG CTCGTCGCGG CGGCGGTAAT 100
<210> 786
<211> 100
<212> DNA
<213> Escherichia coli K-12 MG1655
<220> yhgf b3408
<222> 1969-2068
<223> yhgf b3408 U00096 3535122..3537344
<313> 786: 1997-2016
<400> 786
GATCCGCATA CCGTGGTGAA AGCGGGCGAC ATTGTGAAGG TGAAAGTGCT 50
GGAAGTGGAT CTTCAGCGTA AACGTATCGC CCTGACTATG CGCCTGGATG 100
<210> 787
<211> 100
<212> DNA
<213> Escherichia coli K-12 MG1655
<220> yhgg b3411
<222> 138-237
<223> yhgg b3411 U00096 3540364..3540600
<313> 787: 199-218
<400> 787
CGTGCGGATT CAGGAAGAAC CTGACGGCTG CCTCTCTGGC AGTTGTAAAA 50
GCTGCCCGGA AGGAAAAGCC TGTCTGCGCG AGTGGTGGGC GCTGCGTTAA 100
<210> 788

<211> 100
<212> DNA
<213> Escherichia coli K-12 MG1655
<220> yhgg b3411
<222> 68-167
<223> yhgg b3411 U00096 3540364..3540600
<313> 788: 96-115
<400> 788
GCCAGACATT GAACACTCCA CAGCCAATGA TTAACGCCAT GCTGCAACAA 50
CTGGAAAGTA TGGGCAAAGC CGTGCGGATT CAGGAAGAAC CTGACGGCTG 100
<210> 789
<211> 100
<212> DNA
<213> Escherichia coli K-12 MG1655
<220> yhgg b3411
<222> 3-102
<223> yhgg b3411 U00096 3540364..3540600
<313> 789: 60-79
<400> 789
GGCTTCACTT ATTCAGGTGC GCGATTTGCT GGCGTTACGG GGCCGTATGG 50
AAGCGGCCCA GATAAGCCAG ACATTGAACA CTCCACAGCC AATGATTAAC 100
<210> 790
<211> 100
<212> DNA
<213> Escherichia coli K-12 MG1655
<220> yhgh b3414
<222> 559-658
<223> yhgh b3414 U00096 3542470..3543201
<313> 790: 596-615
<400> 790
CGCAAGCGCA ACCTGAAAAA TGCCTTTCGT CTTGAATTGC CCGTGCAAGG 50
TCGCCATATG GTGATTGTGG ATGATGTCGT TACCACCGGA AGTACCGTCG 100
<210> 791
<211> 100
<212> DNA
<213> Escherichia coli K-12 MG1655
<220> yhgh b3414
<222> 499-598
<223> yhgh b3414 U00096 3542470..3543201
<313> 791: 522-541
<400> 791
AGCGAAGCCG TCACACGTAC ACGGGCCACT GCGACCCAGC ATTTTCTCAG 50
TGCCCGGCTG CGCAAGCGCA ACCTGAAAAA TGCCTTTCGT CTTGAATTGC 100
<210> 792
<211> 100
<212> DNA
<213> Escherichia coli K-12 MG1655
<220> yhgh b3414
<222> 470-569
<223> yhgh b3414 U00096 3542470..3543201
<313> 792: 495-514
<400> 792
TATCACGCTG GTTGCACTGC CAATGGGATA GCGAAGCCGT CACACGTACA 50
CGGGCCACTG CGACCCAGCA TTTTCTCAGT GCCCGGCTGC GCAAGCGCAA 100
<210> 793
<211> 100
<212> DNA
<213> Escherichia coli K-12 MG1655
<220> yhgi b3415
<222> 477-576
<223> yhgi b3415 U00096 3543260..3543835
<313> 793: 553-572
<400> 793
GAAAGAAGGG ATCGAGAAGC AGCTGCTGAA CGAATTCCCG GAGCTGAAAG 50
GTGTGCGCGA TCTCACCGAA CACCAGCGCG GCGAACACTC CTACTACTAA 100
<210> 794
<211> 100
<212> DNA
<213> Escherichia coli K-12 MG1655
<220> yhgi b3415
<222> 417-516

<223> yhgi b3415 U00096 3543260..3543835
<313> 794: 476-495
<400> 794
TTACGCCATT CTGCAATTTG GCGGCGGCTG TAACGGTTGT TCCATGGTCG  50
ATGTGACGCT GAAAGAAGGG ATCGAGAAGC AGCTGCTGAA CGAATTCCCG  100
<210> 795
<211> 100
<212> DNA
<213> Escherichia coli K-12 MG1655
<220> yhgi b3415
<222> 380-479
<223> yhgi b3415 U00096 3543260..3543835
<313> 795: 426-445
<400> 795
GTGGTCGCGT TTCGCTGATG GAAATCACCG AAGACGGTTA CGCCATTCTG  50
CAATTTGGCG GCGGCTGTAA CGGTTGTTCC ATGGTCGATG TGACGCTGAA  100
<210> 796
<211> 100
<212> DNA
<213> Escherichia coli K-12 MG1655
<220> yhgj b3420
<222> 507-606
<223> yhgj b3420 U00096 complement(3553466..3554071)
<313> 796: 583-602
<400> 796
ATGCCATCTG CTGACCAATA TCGCGGTGGT GGAGCGTTTC TTGCCGGTGC  50
GGTTTAGTTT GATAGAAACA GATGGCGTAA CGCGGGTGAG CATTGAATGA  100
<210> 797
<211> 100
<212> DNA
<213> Escherichia coli K-12 MG1655
<220> yhgj b3420
<222> 442-541
<223> yhgj b3420 U00096 complement(3553466..3554071)
<313> 797: 494-513
<400> 797
GCTGACCAAC TGGTGCTACC GATGGCGCTG GCGGGCGCGG GGGAATTTAC  50
GGTCGCCCAT CCCTCATGCC ATCTGCTGAC CAATATCGCG GTGGTGGAGC  100
<210> 798
<211> 100
<212> DNA
<213> Escherichia coli K-12 MG1655
<220> yhgj b3420
<222> 415-514
<223> yhgj b3420 U00096 complement(3553466..3554071)
<313> 798: 440-459
<400> 798
AGCACGGCGG CGGTGGGGGA ATATCTTGCT GACCAACTGG TGCTACCGAT  50
GGCGCTGGCG GGCGCGGGGG AATTTACGGT CGCCCATCCC TCATGCCATC  100
<210> 799
<211> 100
<212> DNA
<213> Escherichia coli K-12 MG1655
<220> b2880 b2880
<222> 681-780
<223> b2880 b2880 U00096 3018561..3019340
<313> 799: 756-775
<400> 799
AGATTTGCGG GGCAGCGTGG CCTATAAACG CTATATCACG GGAGTTCTGG  50
TAGCCGACCT GTATGCCGAC TGCCAACAGG CTGGGGAGGA AGCCGTATGA  100
<210> 800
<211> 100
<212> DNA
<213> Escherichia coli K-12 MG1655
<220> b2880 b2880
<222> 681-780
<223> b2880 b2880 U00096 3018561..3019340
<313> 800: 716-735
<400> 800
AGATTTGCGG GGCAGCGTGG CCTATAAACG CTATATCACG GGAGTTCTGG  50
TAGCCGACCT GTATGCCGAC TGCCAACAGG CTGGGGAGGA AGCCGTATGA  100

<210> 801
<211> 100
<212> DNA
<213> Escherichia coli K-12 MG1655
<220> b2880 b2880
<222> 615-714
<223> b2880 b2880 U00096 3018561..3019340
<313> 801: 651-670
<400> 801
CGAAAAACAA AATCTGGAAG GCAATGCACT TGAACAGGCT GTCGCCAACG 50
CCATTTTCCC GCAGGAAGAT TTGCGGGGCA GCGTGGCCTA TAAACGCTAT 100
<210> 802
<211> 100
<212> DNA
<213> Escherichia coli K-12 MG1655
<220> b2881 b2882
<222> 2772-2871
<223> b2881 b2882 U00096 3019337..3022207
<313> 802: 2844-2863
<400> 802
TCCGGCGATT GCTACCGCAA TTCACGATGC ATGCGGCATC TGGTTACGCG 50
AATGGCATTT CACACCGGAG AAAATACTCA CTGCGCTGGA AAAAATATAA 100
<210> 803
<211> 100
<212> DNA
<213> Escherichia coli K-12 MG1655
<220> b2881 b2882
<222> 2706-2805
<223> b2881 b2882 U00096 3019337..3022207
<313> 803: 2744-2763
<400> 803
GAGCGACGAT AAAGTCGGCC CGTTCGGGGC GAAATCGATC TCGGAAATCG 50
GTGTAAATGG CGCAGCTCCG GCGATTGCTA CCGCAATTCA CGATGCATGC 100
<210> 804
<211> 100
<212> DNA
<213> Escherichia coli K-12 MG1655
<220> b2881 b2882
<222> 2640-2739
<223> b2881 b2882 U00096 3019337..3022207
<313> 804: 2665-2684
<400> 804
TTTACGCAGT TACGGCGCAC CGAAAATTGG TGACATTCCG CGTGATTTCC 50
GCGCTGTGCT GGTGCCGAGC GACGATAAAG TCGGCCCGTT CGGGGCGAAA 100
<210> 805
<211> 100
<212> DNA
<213> Escherichia coli K-12 MG1655
<220> yhgn b3435
<222> 495-594
<223> yhgn b3435 U00096 3572704..3573297
<313> 805: 545-564
<400> 805
GAAAGGGGTG AACGCACTTG AACGCCTGAT GGGATTGATT CTGGTGATGA 50
TGGCAACCCA GATGTTCCTC GACGGCATTC GAATGTGGAT GAAGGGGTAA 100
<210> 806
<211> 100
<212> DNA
<213> Escherichia coli K-12 MG1655
<220> yhgn b3435
<222> 479-578
<223> yhgn b3435 U00096 3572704..3573297
<313> 806: 497-516
<400> 806
TACGTCTGCT GGGCGAGAAA GGGGTGAACG CACTTGAACG CCTGATGGGA 50
TTGATTCTGG TGATGATGGC AACCCAGATG TTCCTCGACG GCATTCGAAT 100
<210> 807
<211> 100
<212> DNA
<213> Escherichia coli K-12 MG1655
<220> yhgn b3435

<222> 429-528
<223> yhgn b3435 U00096 3572704..3573297
<313> 807: 468-487
<400> 807
GCTGGCCTGG GGCGGCACCT TTGTCATCCT GCTACAGTCT TCGCTATTTT 50
TACGTCTGCT GGGCGAGAAA GGGGTGAACG CACTTGAACG CCTGATGGGA 100
<210> 808
<211> 100
<212> DNA
<213> Escherichia coli K-12 MG1655
<220> rfab b3629
<222> 730-829
<223> rfab b3629 U00096 complement(3800685..3801794)
<313> 808: 755-774
<400> 808
GATCGAATTG TCTGGTATGG TTGGCAACAA TATCCCTGGG AATTGGTCCA 50
GCAAGATATT GAAAAAGTGA GTGCGTTATT GCTCACGTCA TCTTTTGAAG 100
<210> 809
<211> 100
<212> DNA
<213> Escherichia coli K-12 MG1655
<220> rfab b3629
<222> 612-711
<223> rfab b3629 U00096 complement(3800685..3801794)
<313> 809: 657-676
<400> 809
AGTTAAAGAC CTGCTTGATG GCTTATCACA AGCGAAAGGT AACTGGAAAC 50
TCCATGTATT GGGTGATGGC TCCGATTTCG AAAAATGCCA GGCTTATGGT 100
<210> 810
<211> 100
<212> DNA
<213> Escherichia coli K-12 MG1655
<220> rfab b3629
<222> 525-624
<223> rfab b3629 U00096 complement(3800685..3801794)
<313> 810: 555-574
<400> 810
TAAAGACTCA GTCATTCCCG CGCCCGAGGA AGGTGAAACG GCAACGTTTA 50
TTTATGTTGG GCGTATGAAA TTTGAAGGCC AGAAAAGAGT TAAAGACCTG 100
<210> 811
<211> 100
<212> DNA
<213> Escherichia coli K-12 MG1655
<220> rfac b3622
<222> 852-951
<223> rfac b3622 U00096 3793606..3794565
<313> 811: 879-898
<400> 811
GGGATTAATT GGTGGGTATG GGAAGAATCA GATGGTATGT AGGGCTCCAA 50
GAGAAAATTT AATTAACCTC AACAGTCAAG CAGTTTTGGA AAAGTTATCA 100
<210> 812
<211> 100
<212> DNA
<213> Escherichia coli K-12 MG1655
<220> rfac b3622
<222> 763-862
<223> rfac b3622 U00096 3793606..3794565
<313> 812: 800-819
<400> 812
AAATTTGTAG TGTCGGTGGA TACGGGGTTA AGCCATTTAA CGGCGGCACT 50
GGATAGACCC AATATCACGG TTTATGGACC AACCGATCCG GGATTAATTG 100
<210> 813
<211> 100
<212> DNA
<213> Escherichia coli K-12 MG1655
<220> rfac b3622
<222> 709-808
<223> rfac b3622 U00096 3793606..3794565
<313> 813: 770-789
<400> 813
GAAGTATTGC CGAAGATGAG TCTGGAAGGC GTTGCCCGCG TGCTGGCCGG 50

GGCTAAATTT GTAGTGTCGG TGGATACGGG GTTAAGCCAT TTAACGGCGG 100
<210> 814
<211> 100
<212> DNA
<213> Escherichia coli K-12 MG1655
<220> rfad b3620
<222> 834-933
<223> rfad b3620 U00096 3791614..3792546
<313> 814: 901-920
<400> 814
AGATCTGACA AATCTGCGCG CGGCGGGTTA CGACAAACCG TTCAAAACCG 50
TTGCTGAAGG TGTAACGGAA TACATGGCCT GGCTGAATCG CGACGCATAA 100
<210> 815
<211> 100
<212> DNA
<213> Escherichia coli K-12 MG1655
<220> rfad b3620
<222> 764-863
<223> rfad b3620 U00096 3791614..3792546
<313> 815: 802-821
<400> 815
AGAAAGGCCA GATCGAATAC ATTCCGTTCC CGGATAAACT GAAAGGCCGC 50
TATCAGGCGT TCACTCAGGC AGATCTGACA AATCTGCGCG CGGCGGGTTA 100
<210> 816
<211> 100
<212> DNA
<213> Escherichia coli K-12 MG1655
<220> rfad b3620
<222> 718-817
<223> rfad b3620 U00096 3791614..3792546
<313> 816: 761-780
<400> 816
GCGGAATCCT TCCAGGCGGT AGCTGATGCT ACGCTGGCTT ATCACAAGAA 50
AGGCCAGATC GAATACATTC CGTTCCCGGA TAAACTGAAA GGCCGCTATC 100
<210> 817
<211> 100
<212> DNA
<213> Escherichia coli K-12 MG1655
<220> b2889 b2890
<222> 450-549
<223> b2889 b2890 U00096 3031085..3031633
<313> 817: 510-529
<400> 817
TATTGATGCC ACGCCGTGGG CGTTCAGTCC GTGGATGGTG ATGCAGGCGA 50
CAAATCGCGA AGCCAGAAAA CGATTATCTG CATTTACCCA GCTTAAATAA 100
<210> 818
<211> 100
<212> DNA
<213> Escherichia coli K-12 MG1655
<220> b2889 b2890
<222> 317-416
<223> b2889 b2890 U00096 3031085..3031633
<313> 818: 355-374
<400> 818
CCACCGATCC GAGTGGCATT GTGGAAAATG AAGTGTGTCC GGTATTTGCC 50
GCACGCACCA CTAGTGCGTT ACAGATCAAT GATGATGAAG TGATGGATTA 100
<210> 819
<211> 100
<212> DNA
<213> Escherichia coli K-12 MG1655
<220> b2889 b2890
<222> 273-372
<223> b2889 b2890 U00096 3031085..3031633
<313> 819: 316-335
<400> 819
AATTACGCCT CCTGAATCTA TCTATCCTGA CTTTCGCTAC CGCGCCACCG 50
ATCCGAGTGG CATTGTGGAA AATGAAGTGT GTCCGGTATT TGCCGCACGC 100
<210> 820
<211> 100
<212> DNA
<213> Escherichia coli K-12 MG1655

<220> rfaf b3621
<222> 948-1047
<223> rfaf b3621 U00096 3792556..3793602
<313> 820: 1013-1032
<400> 820
AGGTGACGCT GCGGAGGGTT ATCACCAGAG CTTAATCGAC ATTACTCCCC  50
AGCGCGTACT GGAAGAACTC AACGCGCTAT TGTTACAAGA GGAAGCCTGA  100
<210> 821
<211> 100
<212> DNA
<213> Escherichia coli K-12 MG1655
<220> rfaf b3621
<222> 926-1025
<223> rfaf b3621 U00096 3792556..3793602
<313> 821: 969-988
<400> 821
CCGGCTATCA CAAAGTGCGT AAAGGTGACG CTGCGGAGGG TTATCACCAG  50
AGCTTAATCG ACATTACTCC CCAGCGCGTA CTGGAAGAAC TCAACGCGCT  100
<210> 822
<211> 100
<212> DNA
<213> Escherichia coli K-12 MG1655
<220> rfaf b3621
<222> 895-994
<223> rfaf b3621 U00096 3792556..3793602
<313> 822: 929-948
<400> 822
TCCCATAAAG CGCGCGTGAT CCGTTTGATT ACCGGCTATC ACAAAGTGCG  50
TAAAGGTGAC GCTGCGGAGG GTTATCACCA GAGCTTAATC GACATTACTC  100
<210> 823
<211> 100
<212> DNA
<213> Escherichia coli K-12 MG1655
<220> rfag b3632
<222> 1011-1110
<223> rfag b3632 U00096 complement(3803570..3804694)
<313> 823: 1063-1082
<400> 823
GTCGCCATTG CGAATGGCCT GGGCGGAGAA TGCTCGCCAT TATGCCGATA  50
CTCAGGATTT GTATAGCTTG CCAGAAAAAG CCGCGGATAT CATTACAGGT  100
<210> 824
<211> 100
<212> DNA
<213> Escherichia coli K-12 MG1655
<220> rfag b3632
<222> 932-1031
<223> rfag b3632 U00096 complement(3803570..3804694)
<313> 824: 962-981
<400> 824
ATGCCAATTG TGGAACGGTC ATCGCTGAAC CTTTCTCTCA GGAACAATTA  50
AATGAAGTTT TACGTAAAGC GTTAACTCAG TCGCCATTGC GAATGGCCTG  100
<210> 825
<211> 100
<212> DNA
<213> Escherichia coli K-12 MG1655
<220> rfag b3632
<222> 874-973
<223> rfag b3632 U00096 complement(3803570..3804694)
<313> 825: 904-923
<400> 825
ACTGCTGGGT TACCTGTTTT AACAACAGCG GTATGTGGGT ACGCGCATTA  50
TATTGCGGAT GCCAATTGTG GAACGGTCAT CGCTGAACCT TTCTCTCAGG  100
<210> 826
<211> 100
<212> DNA
<213> Escherichia coli K-12 MG1655
<220> rfah b3843
<222> 353-452
<223> rfah b3843 U00096 complement(4021923..4022411)
<313> 826: 415-434
<400> 826

TTACCGAAGG CGCGTTCGAA GGCTTTCAGG CCATTTTCAC CGAACCCGAT 50
GGTGAGGCTC GCTCCATGCT ATTGCTTAAT CTTATTAATA AAGAGATTAA 100
<210> 827
<211> 100
<212> DNA
<213> Escherichia coli K-12 MG1655
<220> rfah b3843
<222> 279-378
<223> rfah b3843 U00096 complement(4021923..4022411)
<313> 827: 316-335
<400> 827
TCATCAGCTA TCGGTATATA AACCGAAAGA CATTGTCGAT CCGGCAACCC 50
CTTATCCGGG TGATAAGGTG ATTATTACCG AAGGCGCGTT CGAAGGCTTT 100
<210> 828
<211> 100
<212> DNA
<213> Escherichia coli K-12 MG1655
<220> rfah b3843
<222> 207-306
<223> rfah b3843 U00096 complement(4021923..4022411)
<313> 828: 266-285
<400> 828
CAACGCGACC CGCGGTGTCA GCCACTTCGT GCGCTTTGGC GCGTCGCCAG 50
CGATAGTCCC ATCGGCGGTT ATTCATCAGC TATCGGTATA TAAACCGAAA 100
<210> 829
<211> 100
<212> DNA
<213> Escherichia coli K-12 MG1655
<220> rfai b3628
<222> 772-871
<223> rfai b3628 U00096 complement(3799666..3800685)
<313> 829: 799-818
<400> 829
ACCAACGATA CTATTTTTAT CCATTATATC GGGCCAACCA AGCCCTGGCA 50
TGATTGGGCC TGGGATTATC CTGTATCACA AGCCTTTATG GAGGCAAAAA 100
<210> 830
<211> 100
<212> DNA
<213> Escherichia coli K-12 MG1655
<220> rfai b3628
<222> 489-588
<223> rfai b3628 U00096 complement(3799666..3800685)
<313> 830: 520-539
<400> 830
CTGGTGGGAA AAACGCGCCC ATTCGTTAGG TGTTGCGGGA ATTGCTAAAG 50
GTTACTTTAA CTCCGGTTTT TTATTGATTA ATACTGCCCA ATGGGCGGCT 100
<210> 831
<211> 100
<212> DNA
<213> Escherichia coli K-12 MG1655
<220> rfai b3628
<222> 462-561
<223> rfai b3628 U00096 complement(3799666..3800685)
<313> 831: 490-509
<400> 831
AATGGTTGTT ACAGAAGGGC AAGCTGACTG GTGGGAAAAA CGCGCCCATT 50
CGTTAGGTGT TGCGGGAATT GCTAAAGGTT ACTTTAACTC CGGTTTTTTA 100
<210> 832
<211> 100
<212> DNA
<213> Escherichia coli K-12 MG1655
<220> rfaj b3627
<222> 824-923
<223> rfaj b3627 U00096 complement(3798610..3799626)
<313> 832: 885-904
<400> 832
GGGCAATTTA TCCATCGGTA AAATACTATA AAATAGCTTT AGAAAATTCC 50
CCCTGGAAAG ATGACTCTCC ACGAGATGCG AAATCAATTA TTGAATTTAA 100
<210> 833
<211> 100
<212> DNA

<213> Escherichia coli K-12 MG1655
<220> rfaj b3627
<222> 791-890
<223> rfaj b3627 U00096 complement(3798610..3799626)
<313> 833: 811-830
<400> 833
ATTATACAGG TGCAACTAAA CCGTGGCACA AATGGGCAAT TTATCCATCG 50
GTAAAATACT ATAAAATAGC TTTAGAAAAT TCCCCCTGGA AAGATGACTC 100
<210> 834
<211> 100
<212> DNA
<213> Escherichia coli K-12 MG1655
<220> rfaj b3627
<222> 550-649
<223> rfaj b3627 U00096 complement(3798610..3799626)
<313> 834: 589-608
<400> 834
GTTTATTTAG ACTTAAAAAA ATGGGCTGAC GCAAAACTAA CAGAAAAAGC 50
GTTGTCTATT CTTATGAGTA AAGATAATGT TTATAAATAC CCTGATCAGG 100
<210> 835
<211> 100
<212> DNA
<213> Escherichia coli K-12 MG1655
<220> rfak b3624
<222> 910-1009
<223> rfak b3624 U00096 complement(3795866..3796939)
<313> 835: 964-983
<400> 835
TTTTACCCTA ATTCACGTAC TCCGGAATAT CCCTCGCATT TAATTTGGTC 50
CCCGAATCAT CATAAATCTA TACAAATAGT ATCCCCAACA TATACGGTAA 100
<210> 836
<211> 100
<212> DNA
<213> Escherichia coli K-12 MG1655
<220> rfak b3624
<222> 903-1002
<223> rfak b3624 U00096 complement(3795866..3796939)
<313> 836: 945-964
<400> 836
GCTTGCATTT TACCCTAATT CACGTACTCC GGAATATCCC TCGCATTTAA 50
TTTGGTCCCC GAATCATCAT AAATCTATAC AAATAGTATC CCCAACATAT 100
<210> 837
<211> 100
<212> DNA
<213> Escherichia coli K-12 MG1655
<220> rfak b3624
<222> 921-1020
<223> rfak b3624 U00096 complement(3795866..3796939)
<313> 837: 953-972
<400> 837
TTCACGTACT CCGGAATATC CCTCGCATTT AATTTGGTCC CCGAATCATC 50
ATAAATCTAT ACAAATAGTA TCCCCAACAT ATACGGTAAA AGATATCGAT 100
<210> 838
<211> 100
<212> DNA
<213> Escherichia coli K-12 MG1655
<220> rfal b3623
<222> 1096-1195
<223> rfal b3623 U00096 3794575..3795834
<313> 838: 1143-1162
<400> 838
ATAGCATATA AAAAACGAGC TTTGGGTTTG TTGATATTAA CGCTTGGCAT 50
TGTGGGGATT GGACTCAGTG ATGTGATCAT ATGGGCACGC AGCATTCCAA 100
<210> 839
<211> 100
<212> DNA
<213> Escherichia coli K-12 MG1655
<220> rfal b3623
<222> 1003-1102
<223> rfal b3623 U00096 3794575..3795834
<313> 839: 1048-1067

<400> 839
GTACATCTAC ATAATGAGAT AATTGAAGCA GGGTCACTGA AAGGTCTGAT 50
GGGAATTTTT TCCACACTTT TCCTCTATTT TTCACTATTT TATATAGCAT 100
<210> 840
<211> 100
<212> DNA
<213> Escherichia coli K-12 MG1655
<220> rfal b3623
<222> 948-1047
<223> rfal b3623 U00096 3794575..3795834
<313> 840: 965-984
<400> 840
GAATTTGTTA GTTGCAGAAC ACAATAGGCT AAGAGGGGCA TTGGAGTTTT 50
CTAACGTACA TCTACATAAT GAGATAATTG AAGCAGGGTC ACTGAAAGGT 100
<210> 841
<211> 100
<212> DNA
<213> Escherichia coli K-12 MG1655
<220> rfap b3631
<222> 699-798
<223> rfap b3631 U00096 complement(3802780..3803577)
<313> 841: 774-793
<400> 841
GCCTTTACGC AAAATATTGT CTCTTGAACA GAATTTATTG AACATGGCAA 50
GCGTTAAGGC CGAGCGTATT AAAGAGCGAA CACAACGCAA AGGATTATAA 100
<210> 842
<211> 100
<212> DNA
<213> Escherichia coli K-12 MG1655
<220> rfap b3631
<222> 539-638
<223> rfap b3631 U00096 complement(3802780..3803577)
<313> 842: 567-586
<400> 842
AATTAAAAAT TTCAGTTATC GATCTGCATC GGGCACAGAT ACGTGCAAAA 50
GTACCGCGCC GCTGGCGCGA TAAAGACCTG ATTGGTTTAT ATTTCTCATC 100
<210> 843
<211> 100
<212> DNA
<213> Escherichia coli K-12 MG1655
<220> rfap b3631
<222> 456-555
<223> rfap b3631 U00096 complement(3802780..3803577)
<313> 843: 498-517
<400> 843
AATGCATACT GCAGGGATAA ATCACCGCGA CTGTTACATT TGCCACTTTT 50
TGCTTCATTT GCCATTTACT GGCCGGGAAG ATGAATTAAA AATTTCAGTT 100
<210> 844
<211> 100
<212> DNA
<213> Escherichia coli K-12 MG1655
<220> yhha b3449
<222> 342-441
<223> yhha b3449 U00096 3584574..3585014
<313> 844: 414-433
<400> 844
GAATCAGCAG CATATGCTGC CGGAGAGGAG AAACGGCGAC ATGCTGAATC 50
AGCCCAGCAC GCCGCAGCCT GACATTCCGT TGAAAACTAT TGGGCCGTAA 100
<210> 845
<211> 100
<212> DNA
<213> Escherichia coli K-12 MG1655
<220> yhha b3449
<222> 342-441
<223> yhha b3449 U00096 3584574..3585014
<313> 845: 372-391
<400> 845
GAATCAGCAG CATATGCTGC CGGAGAGGAG AAACGGCGAC ATGCTGAATC 50
AGCCCAGCAC GCCGCAGCCT GACATTCCGT TGAAAACTAT TGGGCCGTAA 100
<210> 846
<211> 100

<212> DNA
<213> Escherichia coli K-12 MG1655
<220> yhha b3449
<222> 249-348
<223> yhha b3449 U00096 3584574..3585014
<313> 846: 302-321
<400> 846
ACAGCCGGGG GAGCGAAATC CCGCCCGGCA GCAAATGCTG CCCAACACCA 50
ACGGCGGGAT GTTAAACAGC AACCGTAATC CGGATAGTTC GTTGAATCAG 100
<210> 847
<211> 100
<212> DNA
<213> Escherichia coli K-12 MG1655
<220> rfaq b3633
<222> 936-1035
<223> rfaq b3633 U00096 complement(3804691..3805725)
<313> 847: 990-1009
<400> 847
AAAATATCTT TCTGTTATCC CAGCGGAGGA TGTGATCGCC GCTACGGAAA 50
AGCTGTTGCC AGAAGATGCC CCTTCAGCTG ACAGGAATGC ACAATTATGA 100
<210> 848
<211> 100
<212> DNA
<213> Escherichia coli K-12 MG1655
<220> rfaq b3633
<222> 882-981
<223> rfaq b3633 U00096 complement(3804691..3805725)
<313> 848: 915-934
<400> 848
CTGGGCGGGG AATTATCAGA AAATGCCGAC CCGGCATGAA CTTGACCGCA 50
ACAAAAAATA TCTTTCTGTT ATCCCAGCGG AGGATGTGAT CGCCGCTACG 100
<210> 849
<211> 100
<212> DNA
<213> Escherichia coli K-12 MG1655
<220> rfaq b3633
<222> 811-910
<223> rfaq b3633 U00096 complement(3804691..3805725)
<313> 849: 852-871
<400> 849
CCAGTCATTA GTCTATTTGG TGCAACGGAT CACGTATTCT GGCGTCCCTG 50
GACCGAGAAT ATTATTCAAT TCTGGGCGGG GAATTATCAG AAAATGCCGA 100
<210> 850
<211> 100
<212> DNA
<213> Escherichia coli K-12 MG1655
<220> csta b0599
<222> 2007-2106
<223> csta b0599 U00096 629117..631222
<313> 850: 2079-2098
<400> 850
GGCATTGAAA GATCCGAAGC CAACGGCGAA AGAAACGCCG TATGAACCAA 50
TGCCGGAAAA TGTCGAGGAG ATCGTGGCGC AGGCAAAAGG CGCACACTAA 100
<210> 851
<211> 100
<212> DNA
<213> Escherichia coli K-12 MG1655
<220> csta b0599
<222> 1967-2066
<223> csta b0599 U00096 629117..631222
<313> 851: 2017-2036
<400> 851
TGGTGGTTCT GGCACTGTTC TCGATTAAGA CGGCACTTGC GGCATTGAAA 50
GATCCGAAGC CAACGGCGAA AGAAACGCCG TATGAACCAA TGCCGGAAAA 100
<210> 852
<211> 100
<212> DNA
<213> Escherichia coli K-12 MG1655
<220> csta b0599
<222> 1922-2021
<223> csta b0599 U00096 629117..631222

<313> 852: 1979-1998
<400> 852
ACAACCGTCT GGATGCCGGG TTAACCATCT TCTTTATGGT GGTCGTGGTG  50
GTTCTGGCAC TGTTCTCGAT TAAGACGGCA CTTGCGGCAT TGAAAGATCC  100
<210> 853
<211> 100
<212> DNA
<213> Escherichia coli K-12 MG1655
<220> rfas b3630
<222> 658-757
<223> rfas b3630 U00096 complement(3801808..3802743)
<313> 853: 702-721
<400> 853
AATATAATTG TTGATATAAC CAAAGAAAAC CAATCTGGCT GGACTCTTCG  50
TATACTTGAG GCACTATTTT TCAATAAAAA ATTAATTACT AATAATATAA  100
<210> 854
<211> 100
<212> DNA
<213> Escherichia coli K-12 MG1655
<220> rfas b3630
<222> 510-609
<223> rfas b3630 U00096 complement(3801808..3802743)
<313> 854: 540-559
<400> 854
AATTAATGAG TTAGCCGAGA GATTAACAAC ACTAGGATGT AAGCTAGATT  50
TTAATGTTGT CAAAGATAAG ACATCATCTA CGACTTCAAA GTATTTGATT  100
<210> 855
<211> 100
<212> DNA
<213> Escherichia coli K-12 MG1655
<220> rfas b3630
<222> 479-578
<223> rfas b3630 U00096 complement(3801808..3802743)
<313> 855: 511-530
<400> 855
TCCTTGGTCG TGATAAAGGG CGTCTTCAAA TAATTAATGA GTTAGCCGAG  50
AGATTAACAA CACTAGGATG TAAGCTAGAT TTTAATGTTG TCAAAGATAA  100
<210> 856
<211> 100
<212> DNA
<213> Escherichia coli K-12 MG1655
<220> cstc b1749
<222> 1112-1211
<223> cstc b1749 U00096 complement(1828786..1830006)
<313> 856: 1149-1168
<400> 856
ACGTGGTGCG TTTTGCGCCT GCGCTCAATG TCAGCGAAGA AGAGGTGACG  50
ACCGGACTGG ATCGCTTTGC AGCTGCTTGC GAACACTTTG TTAGCCGAGG  100
<210> 857
<211> 100
<212> DNA
<213> Escherichia coli K-12 MG1655
<220> cstc b1749
<222> 1075-1174
<223> cstc b1749 U00096 complement(1828786..1830006)
<313> 857: 1096-1115
<400> 857
GCGAAAGCAG GCGTGATGGT ACTGATTGCG GGTGGCAACG TGGTGCGTTT  50
TGCGCCTGCG CTCAATGTCA GCGAAGAAGA GGTGACGACC GGACTGGATC  100
<210> 858
<211> 100
<212> DNA
<213> Escherichia coli K-12 MG1655
<220> cstc b1749
<222> 1001-1100
<223> cstc b1749 U00096 complement(1828786..1830006)
<313> 858: 1051-1070
<400> 858
GCTTAGGTTT GCTGATTGGC TGTGTACTGA ATGCCGATTA CGCCGGGCAA  50
GCGAAACAGA TCTCTCAGGA AGCGGCGAAA GCAGGCGTGA TGGTACTGAT  100
<210> 859

<211> 100
<212> DNA
<213> Escherichia coli K-12 MG1655
<220> yhhf b3466
<222> 498-597
<223> yhhf b3466 U00096 3602024..3602620
<313> 859: 575-594
<400> 859
TGTTCCAGCA AACTGGTCAT TACATCGGGA AAAAGTGGCG GGTCAGGTGG 50
CTTATCGGCT GTATCAACGC GAAGCACAAG GAGAAAGTGA TGCTGATTAA 100
<210> 860
<211> 100
<212> DNA
<213> Escherichia coli K-12 MG1655
<220> yhhf b3466
<222> 482-581
<223> yhhf b3466 U00096 3602024..3602620
<313> 860: 529-548
<400> 860
AAAACGGTCT GCCCACTGTT CCAGCAAACT GGTCATTACA TCGGGAAAAA 50
GTGGCGGGTC AGGTGGCTTA TCGGCTGTAT CAACGCGAAG CACAAGGAGA 100
<210> 861
<211> 100
<212> DNA
<213> Escherichia coli K-12 MG1655
<220> yhhf b3466
<222> 445-544
<223> yhhf b3466 U00096 3602024..3602620
<313> 861: 490-509
<400> 861
GCTGACGAAG CCCTGATTTA TGTCGAAAGC GAAGTCGAAA ACGGTCTGCC 50
CACTGTTCCA GCAAACTGGT CATTACATCG GGAAAAAGTG GCGGGTCAGG 100
<210> 862
<211> 100
<212> DNA
<213> Escherichia coli K-12 MG1655
<220> yhhg b3482
<222> 303-402
<223> yhhg b3482 U00096 3616219..3616620
<313> 862: 347-366
<400> 862
GAAAGGTGAC ATGGGTGACG TGCAGCATTT TGCCGATGAC GTTATCGCCC 50
AGCGCGGCGT GCGGCACGGG CATTTGCAGT GCTTGCCGAA GGAAGATTGA 100
<210> 863
<211> 100
<212> DNA
<213> Escherichia coli K-12 MG1655
<220> yhhg b3482
<222> 286-385
<223> yhhg b3482 U00096 3616219..3616620
<313> 863: 305-324
<400> 863
CTGGAAATCG CCGTGTTGAA AGGTGACATG GGTGACGTGC AGCATTTTGC 50
CGATGACGTT ATCGCCCAGC GCGGCGTGCG GCACGGGCAT TTGCAGTGCT 100
<210> 864
<211> 100
<212> DNA
<213> Escherichia coli K-12 MG1655
<220> yhhg b3482
<222> 227-326
<223> yhhg b3482 U00096 3616219..3616620
<313> 864: 267-286
<400> 864
ATCATCACCA CGACCTCTCC GTCGCCACGC TGCATGTGCA CATCAACCAC 50
GACGACTGTC TGGAAATCGC CGTGTTGAAA GGTGACATGG GTGACGTGCA 100
<210> 865
<211> 100
<212> DNA
<213> Escherichia coli K-12 MG1655
<220> yhhh b3484
<222> 270-369

<223> yhhh b3484 U00096 3621045..3621413
<313> 865: 303-322
<400> 865
TGAAGGTACC ATTCACTCAG ACCAAATTGC TGGTGGGGTT TTTATTATAG  50
AAATAGGCAA AAATGATGGG AGAATTCTGA ATTTTGGCCA CGGAAAATAA  100
<210> 866
<211> 100
<212> DNA
<213> Escherichia coli K-12 MG1655
<220> yhhh b3484
<222> 240-339
<223> yhhh b3484 U00096 3621045..3621413
<313> 866: 290-309
<400> 866
AATCACTGAG TTAACAACAA GTTGGGTTGT TGAAGGTACC ATTCACTCAG  50
ACCAAATTGC TGGTGGGGTT TTTATTATAG AAATAGGCAA AAATGATGGG  100
<210> 867
<211> 100
<212> DNA
<213> Escherichia coli K-12 MG1655
<220> yhhh b3484
<222> 226-325
<223> yhhh b3484 U00096 3621045..3621413
<313> 867: 289-308
<400> 867
GAAAAACCAT ACGAAATCAC TGAGTTAACA ACAAGTTGGG TTGTTGAAGG  50
TACCATTCAC TCAGACCAAA TTGCTGGTGG GGTTTTTATT ATAGAAATAG  100
<210> 868
<211> 100
<212> DNA
<213> Escherichia coli K-12 MG1655
<220> yhhi b3485
<222> 861-960
<223> yhhi b3485 U00096 3622009..3623145
<313> 868: 884-903
<400> 868
TGCTGATTTA ACCGCTGAGA AATTCGCCAC AGCGATCCGA AATCACTGGC  50
ACGTGGAGAA TAAGCTGCAC TGGCGTCTGG ACGTGGTAAT GAATGAAGAC  100
<210> 869
<211> 100
<212> DNA
<213> Escherichia coli K-12 MG1655
<220> yhhi b3485
<222> 645-744
<223> yhhi b3485 U00096 3622009..3623145
<313> 869: 686-705
<400> 869
AAATAATCCA GAGCATGACA GTTACGCAAT CAGTGAAAAG AGTCACGGCA  50
GAGAAGAAAT CCGTCTTCAT ATTGTTTGCG ATGTCCCTGA TGAACTTATT  100
<210> 870
<211> 100
<212> DNA
<213> Escherichia coli K-12 MG1655
<220> yhhi b3485
<222> 539-638
<223> yhhi b3485 U00096 3622009..3623145
<313> 870: 580-599
<400> 870
TTGCAGAGAA GATACAAAAA CAGGGAGGTG ATTATTTATT CGCGGTAAAA  50
GGAACCCAGG GGCGGCTAAA TAAAGCCTTT GAGGAAAAAT TTCCGCTGAA  100
<210> 871
<211> 100
<212> DNA
<213> Escherichia coli K-12 MG1655
<220> rfay b3626
<222> 569-668
<223> rfay b3626 U00096 complement(3797894..3798592)
<313> 871: 593-612
<400> 871
AAGATCGTAT TGACTTAGAG CGTCATTACG GTATTAAAAA TGAGATTAGA  50
GATCTAGGCT ATTATCTTTT AGTATATCGT AAAAAAATGC GCAATTTTAT  100

<210> 872
<211> 100
<212> DNA
<213> Escherichia coli K-12 MG1655
<220> rfay b3626
<222> 557-656
<223> rfay b3626 U00096 complement(3797894..3798592)
<313> 872: 581-600
<400> 872
AGCGTAAAGC GAAAGATCGT ATTGACTTAG AGCGTCATTA CGGTATTAAA 50
AATGAGATTA GAGATCTAGG CTATTATCTT TTAGTATATC GTAAAAAAAT 100
<210> 873
<211> 100
<212> DNA
<213> Escherichia coli K-12 MG1655
<220> rfay b3626
<222> 519-618
<223> rfay b3626 U00096 complement(3797894..3798592)
<313> 873: 583-602
<400> 873
TCGAATTATC GATCTCTCCG GAAAGCGTGC TTCAGCGCAG CGTAAAGCGA 50
AAGATCGTAT TGACTTAGAG CGTCATTACG GTATTAAAAA TGAGATTAGA 100
<210> 874
<211> 100
<212> DNA
<213> Escherichia coli K-12 MG1655
<220> yhhj b3486
<222> 1029-1128
<223> yhhj b3486 U00096 complement(3623310..3624437)
<313> 874: 1105-1124
<400> 874
AATCGTCTGG CCGCAGTTTC TGACGCTGAT GGCAATTGGC GGCGCATTTT 50
TCACCATTGC GCTGCTGCGA TTCAGGAAGA CGATTGGGAC AATGGCGTAA 100
<210> 875
<211> 100
<212> DNA
<213> Escherichia coli K-12 MG1655
<220> yhhj b3486
<222> 986-1085
<223> yhhj b3486 U00096 complement(3623310..3624437)
<313> 875: 1035-1054
<400> 875
TTAGCCTCGC GCAGGCCATC CTCTACCGGG GTGCCGGATT CGAAATCGTC 50
TGGCCGCAGT TTCTGACGCT GATGGCAATT GGCGGCGCAT TTTTCACCAT 100
<210> 876
<211> 100
<212> DNA
<213> Escherichia coli K-12 MG1655
<220> yhhj b3486
<222> 946-1045
<223> yhhj b3486 U00096 complement(3623310..3624437)
<313> 876: 988-1007
<400> 876
GTGCAGGACA TTATGCTGAC CATGCCGACG ACACACTTTG TTAGCCTCGC 50
GCAGGCCATC CTCTACCGGG GTGCCGGATT CGAAATCGTC TGGCCGCAGT 100
<210> 877
<211> 100
<212> DNA
<213> Escherichia coli K-12 MG1655
<220> rfaz b3625
<222> 489-588
<223> rfaz b3625 U00096 complement(3796972..3797823)
<313> 877: 512-531
<400> 877
TCTTGGTTAT TGCTCATGCC ATACTATTGC CTTTGCTGCA ATTCAAATCG 50
CATATTCACT TAAGTATGCG CGGATTATTT GTTCTGGTCT TGATTTAACG 100
<210> 878
<211> 100
<212> DNA
<213> Escherichia coli K-12 MG1655
<220> rfaz b3625

<222> 401-500
<223> rfaz b3625 U00096 complement(3796972..3797823)
<313> 878: 432-451
<400> 878
ATATTTTGAG ACAGATTCAC AAAGAACTGC TGATCTCTGT ACCGTTGTCT  50
AAAAAAGGTC GTCTGGTTGG ATTTTGCAAG GACATTAGTC TTGGTTATTG  100
<210> 879
<211> 100
<212> DNA
<213> Escherichia coli K-12 MG1655
<220> rfaz b3625
<222> 324-423
<223> rfaz b3625 U00096 complement(3796972..3797823)
<313> 879: 343-362
<400> 879
TATCCTCCAG AACTGCCTCG TATTACGGTC CTTTTACCGT CGGGAAAAAG  50
GTGGTTTTAT AAAGAAGATT AAATTTAATA TTTTGAGACA GATTCACAAA  100
<210> 880
<211> 100
<212> DNA
<213> Escherichia coli K-12 MG1655
<220> yhhk b3460
<222> 285-384
<223> yhhk b3460 U00096 3595615..3595998
<313> 880: 359-378
<400> 880
GTGGATGGCG GATGCAGGCG TGGAAGATCG CGGTGTGATG ACGGCGTTTA  50
TGCAGGCGCT GGGGTTTACG GCACAACAGG GCGGCTGGGA GAAGTGTTAA  100
<210> 881
<211> 100
<212> DNA
<213> Escherichia coli K-12 MG1655
<220> yhhk b3460
<222> 250-349
<223> yhhk b3460 U00096 3595615..3595998
<313> 881: 268-287
<400> 881
GAGGTTTTGC GTAACAATCC TGGCGTTTCA TGCTGGTGGA TGGCGGATGC  50
AGGCGTGGAA GATCGCGGTG TGATGACGGC GTTTATGCAG GCGCTGGGGT  100
<210> 882
<211> 100
<212> DNA
<213> Escherichia coli K-12 MG1655
<220> yhhk b3460
<222> 140-239
<223> yhhk b3460 U00096 3595615..3595998
<313> 882: 178-197
<400> 882
GCCTGCTCGC TGCCGTGCGG GTAACCTTAA GCGGCACCGA GGGAGCACTG  50
GATTCCCTGC GCGTGCGGGA AGTCACCCGC CGTCGCGGTG TGGGGCAATA  100
<210> 883
<211> 100
<212> DNA
<213> Escherichia coli K-12 MG1655
<220> yhhl b3467
<222> 166-265
<223> yhhl b3467 U00096 3602610..3602879
<313> 883: 187-206
<400> 883
CCGAAAGATG GCCCGCAGAT GACCACCGCC GAAAAGGTAC GGATTTTCCT  50
TTTCGGCGTG TTTGAACTGC TGGCCTGGCA GAAGAAATTT AAAGTTAAAA  100
<210> 884
<211> 100
<212> DNA
<213> Escherichia coli K-12 MG1655
<220> yhhl b3467
<222> 154-253
<223> yhhl b3467 U00096 3602610..3602879
<313> 884: 168-187
<400> 884
AAATCCACTT TACCGAAAGA TGGCCCGCAG ATGACCACCG CCGAAAAGGT  50

ACGGATTTTC CTTTTCGGCG TGTTTGAACT GCTGGCCTGG CAGAAGAAAT 100
<210> 885
<211> 100
<212> DNA
<213> Escherichia coli K-12 MG1655
<220> yhhl b3467
<222> 137-236
<223> yhhl b3467 U00096 3602610..3602879
<313> 885: 166-185
<400> 885
TGCAGCTGGC GCTATTGAAA TCCACTTTAC CGAAAGATGG CCCGCAGATG 50
ACCACCGCCG AAAAGGTACG GATTTTCCTT TTCGGCGTGT TTGAACTGCT 100
<210> 886
<211> 100
<212> DNA
<213> Escherichia coli K-12 MG1655
<220> yhhm b3468
<222> 261-360
<223> yhhm b3468 U00096 complement(3602882..3603241)
<313> 886: 329-348
<400> 886
TCGCCTCGAC GCCCAGCAGT ACCACGCCCT GACAGTGGGC GATAAAGGTA 50
CGCTGAGCTA TAAAGGAACG CGCTTTGTCA GCTTTGTAGG CGAACAATAA 100
<210> 887
<211> 100
<212> DNA
<213> Escherichia coli K-12 MG1655
<220> yhhm b3468
<222> 228-327
<223> yhhm b3468 U00096 complement(3602882..3603241)
<313> 887: 269-288
<400> 887
CAAACCGCAA AGCGGAGGAA TGGAGCAGAC GTTTCGCCTC GACGCCCAGC 50
AGTACCACGC CCTGACAGTG GGCGATAAAG GTACGCTGAG CTATAAAGGA 100
<210> 888
<211> 100
<212> DNA
<213> Escherichia coli K-12 MG1655
<220> yhhm b3468
<222> 197-296
<223> yhhm b3468 U00096 complement(3602882..3603241)
<313> 888: 222-241
<400> 888
CAGGCACCAG TATACGCTAT GAGGCAAGCT TCAAACCGCA AAGCGGAGGA 50
ATGGAGCAGA CGTTTCGCCT CGACGCCCAG CAGTACCACG CCCTGACAGT 100
<210> 889
<211> 100
<212> DNA
<213> Escherichia coli K-12 MG1655
<220> yhhn b3469
<222> 528-627
<223> yhhn b3469 U00096 3603382..3604008
<313> 889: 594-613
<400> 889
GGGGAGCCAC TATCGCCGAC GCTTCCGTGC GGATAACGCG ATTGCTGCGG 50
CCTGCTACTT TGCCGGTCAC TTCCTGATCG TCCGCTCGCT GTATCTCTGA 100
<210> 890
<211> 100
<212> DNA
<213> Escherichia coli K-12 MG1655
<220> yhhn b3469
<222> 425-524
<223> yhhn b3469 U00096 3603382..3604008
<313> 890: 493-512
<400> 890
TGGTGTGGCT GGCAGGTGAA CTGTGGTTCT TCCGTCCGAC CGCTCCGGCG 50
CTCTCTGCGT TTGTTGGCGC TTCGTTGCTG TTTATCAGTA ACTTTGTCTG 100
<210> 891
<211> 100
<212> DNA
<213> Escherichia coli K-12 MG1655

<220> yhhn b3469
<222> 365-464
<223> yhhn b3469 U00096 3603382..3604008
<313> 891: 389-408
<400> 891
GGACGCGCCT GGAAGAGTAC CGTTGGCCTA TCTGCACGTT TATCGGCATG 50
ACGCTGGTGA TGGTGTGGCT GGCAGGTGAA CTGTGGTTCT TCCGTCCGAC 100
<210> 892
<211> 100
<212> DNA
<213> Escherichia coli K-12 MG1655
<220> yhhp b3471
<222> 147-246
<223> yhhp b3471 U00096 complement(3606382..3606627)
<313> 892: 199-218
<400> 892
CCGCGATATT CCTGGGTTTT GTACCTTTAT GGAACACGAA CTGGTTGCTA 50
AAGAGACGGA TGGACTGCCT TATCGTTATT TGATTCGTAA AGGCGGTTGA 100
<210> 893
<211> 100
<212> DNA
<213> Escherichia coli K-12 MG1655
<220> yhhp b3471
<222> 101-200
<223> yhhp b3471 U00096 complement(3606382..3606627)
<313> 893: 149-168
<400> 893
AGCCTGGCGA AACGTTGCTG ATTATCGCCG ACGATCCGGC CACTACCCGC 50
GATATTCCTG GGTTTTGTAC CTTTATGGAA CACGAACTGG TTGCTAAAGA 100
<210> 894
<211> 100
<212> DNA
<213> Escherichia coli K-12 MG1655
<220> yhhp b3471
<222> 94-193
<223> yhhp b3471 U00096 complement(3606382..3606627)
<313> 894: 118-137
<400> 894
AATATGCAGC CTGGCGAAAC GTTGCTGATT ATCGCCGACG ATCCGGCCAC 50
TACCCGCGAT ATTCCTGGGT TTTGTACCTT TATGGAACAC GAACTGGTTG 100
<210> 895
<211> 100
<212> DNA
<213> Escherichia coli K-12 MG1655
<220> yhhq yhhQ
<222> 567-666
<223> yhhq yhhQ U00096 3606848..3607513
<313> 895: 638-657
<400> 895
CAGTATCGTT TTCTTCCTGC CAATGTATGG CGTATTACTC AATATGCTGT 50
TGAAAAGACT GGCAGATAAA TCCGAAATCA ACGCTTTGCA GGCGAGTTAA 100
<210> 896
<211> 100
<212> DNA
<213> Escherichia coli K-12 MG1655
<220> yhhq yhhQ
<222> 455-554
<223> yhhq yhhQ U00096 3606848..3607513
<313> 896: 513-532
<400> 896
TCAGCGACAC GCTGGCCTTT TTCTTCATTG CCTTCTGGCG TAGCCCGGAT 50
GCCTTTATGG CTGAACACTG GATGGAAATC GCGCTGGTCG ATTACTGTTT 100
<210> 897
<211> 100
<212> DNA
<213> Escherichia coli K-12 MG1655
<220> yhhq yhhQ
<222> 405-504
<223> yhhq yhhQ U00096 3606848..3607513
<313> 897: 446-465
<400> 897

GAGTCGCCGC TGGTGGCTGG CACCGACAGC GTCCACACTG TTCGGTAACG 50
TCAGCGACAC GCTGGCCTTT TTCTTCATTG CCTTCTGGCG TAGCCCGGAT 100
<210> 898
<211> 100
<212> DNA
<213> Escherichia coli K-12 MG1655
<220> rfba b2040
<222> 712-811
<223> rfba b2040 U00096 complement(2108160..2109041)
<313> 898: 753-772
<400> 898
AATTTTATTG CGACAATTGA AGAGCGCCAG GGATTGAAGG TTTCCTGTCC 50
TGAAGAGATT GCATTTCGTA AAGGTTTTAT TGATGTTGAG CAAGTAAGAA 100
<210> 899
<211> 100
<212> DNA
<213> Escherichia coli K-12 MG1655
<220> rfba b2040
<222> 640-739
<223> rfba b2040 U00096 complement(2108160..2109041)
<313> 899: 681-700
<400> 899
TCTGTCGCGA TGATGGGGCG TGGCTACGCG TGGCTGGACA CGGGGACTCA 50
TCAGAGTCTG ATAGAAGCAA GTAATTTTAT TGCGACAATT GAAGAGCGCC 100
<210> 900
<211> 100
<212> DNA
<213> Escherichia coli K-12 MG1655
<220> rfba b2040
<222> 456-555
<223> rfba b2040 U00096 complement(2108160..2109041)
<313> 900: 474-493
<400> 900
TAAAAACGGT ACGGCAATCA GTCTGGAAGA AAAACCGTTA GAACCAAAGA 50
GTAATTACGC CGTTACAGGT CTGTACTTTT ATGATAACGA CGTGGTTCAG 100
<210> 901
<211> 100
<212> DNA
<213> Escherichia coli K-12 MG1655
<220> cdsa b0176
<222> 612-711
<223> cdsa b0176 U00096 195785..196534
<313> 901: 650-669
<400> 901
GTTTAAGCGT GAAGCAGGAA TTAAGGACAG CGGTCATTTA ATTCCAGGAC 50
ACGGTGGTAT TTTAGATCGT ATTGATAGCC TGACGGCTGC GGTACCGGTC 100
<210> 902
<211> 100
<212> DNA
<213> Escherichia coli K-12 MG1655
<220> cdsa b0176
<222> 564-663
<223> cdsa b0176 U00096 195785..196534
<313> 902: 595-614
<400> 902
TATTGTCGCA GCGTTAGCCT CAGTGCTCGG CGATCTGACC GAGAGTATGT 50
TTAAGCGTGA AGCAGGAATT AAGGACAGCG GTCATTTAAT TCCAGGACAC 100
<210> 903
<211> 100
<212> DNA
<213> Escherichia coli K-12 MG1655
<220> cdsa b0176
<222> 525-624
<223> cdsa b0176 U00096 195785..196534
<313> 903: 543-562
<400> 903
GAATCTCGAC GTCGCTCCCG TCACCTTACT CATTTGCTCT ATTGTCGCAG 50
CGTTAGCCTC AGTGCTCGGC GATCTGACCG AGAGTATGTT TAAGCGTGAA 100
<210> 904
<211> 100
<212> DNA

<213> Escherichia coli K-12 MG1655
<220> b2896 b2897
<222> 309-408
<223> b2896 b2897 U00096 complement(3038436..3038843)
<313> 904: 346-365
<400> 904
CGGTAAACGG CAACATTTAT GGCTGGCAGC CGACAGCATG GACGAAGCTG  50
AATGGCGGGA TTTACGGCGG ATTTTGTTGC AACAAGAGAC GCAAAGATAA  100
<210> 905
<211> 100
<212> DNA
<213> Escherichia coli K-12 MG1655
<220> b2896 b2897
<222> 271-370
<223> b2896 b2897 U00096 complement(3038436..3038843)
<313> 905: 314-333
<400> 905
ATTAAGAGCG GCATGATGCT GCGTTTACGT TCTGATGGCG GTAAACGGCA  50
ACATTTATGG CTGGCAGCCG ACAGCATGGA CGAAGCTGAA TGGCGGGATT  100
<210> 906
<211> 100
<212> DNA
<213> Escherichia coli K-12 MG1655
<220> b2896 b2897
<222> 205-304
<223> b2896 b2897 U00096 complement(3038436..3038843)
<313> 906: 251-270
<400> 906
TTGTTGATGG ACGGGCGTTT GCGTTGGCAA GGGCAGGAGT GGAGCATCGT  50
CAAAGCACCG TGGATGATTA AGAGCGGCAT GATGCTGCGT TTACGTTCTG  100
<210> 907
<211> 100
<212> DNA
<213> Escherichia coli K-12 MG1655
<220> yhhs b3474
<222> 1011-1110
<223> yhhs b3474 U00096 complement(3608147..3609406)
<313> 907: 1028-1047
<400> 907
GGCATTGGGT GTAGTGGCGG TAAAAGCGGT TCCGCAGCAA AATCAGGGGG  50
CGGCGCTGGC AACTTACACC GTATTTATGG ATTTATCGCT TGGCGTGACT  100
<210> 908
<211> 100
<212> DNA
<213> Escherichia coli K-12 MG1655
<220> yhhs b3474
<222> 945-1044
<223> yhhs b3474 U00096 complement(3608147..3609406)
<313> 908: 957-976
<400> 908
GACTATGCCG TGGATGGCGA AAATCGGCGT CTTACTGGCG GGGGCCGGGT  50
TTTCGCTGGT GTTCCCGGCA TTGGGTGTAG TGGCGGTAAA AGCGGTTCCG  100
<210> 909
<211> 100
<212> DNA
<213> Escherichia coli K-12 MG1655
<220> yhhs b3474
<222> 884-983
<223> yhhs b3474 U00096 complement(3608147..3609406)
<313> 909: 915-934
<400> 909
TAAACGTAGC GATGATTTGC TTTAGCGTTG AGATAATCGG CCTGCTACTG  50
GTTGGCGTGG CGACTATGCC GTGGATGGCG AAAATCGGCG TCTTACTGGC  100
<210> 910
<211> 100
<212> DNA
<213> Escherichia coli K-12 MG1655
<220> rfbb b2042
<222> 987-1086
<223> rfbb b2042 U00096 complement(2109998..2111083)
<313> 910: 1055-1074

<400> 910
AACGGTGGAA TGGTACCTGT CCAATACAAA ATGGGTTGAT AATGTGAAAA 50
GTGGTGCCTA TCAATCGTGG ATTGAACAGA ACTATGAGGG CCGCCAGTAA 100
<210> 911
<211> 100
<212> DNA
<213> Escherichia coli K-12 MG1655
<220> rfbb b2042
<222> 891-990
<223> rfbb b2042 U00096 complement(2109998..2111083)
<313> 911: 936-955
<400> 911
TCCGGGACAC GATCGCCGCT ATGCTATTGA TGCTGAGAAG ATTGGTCGCG 50
CATTGGGATG GAAACCACAG GAAACGTTTG AGAGCGGGAT TCGTAAAACG 100
<210> 912
<211> 100
<212> DNA
<213> Escherichia coli K-12 MG1655
<220> rfbb b2042
<222> 847-946
<223> rfbb b2042 U00096 complement(2109998..2111083)
<313> 912: 873-892
<400> 912
AAAGAGAAAT CTTATCGTGA GCAAATCACT TATGTTGCTG ATCGTCCGGG 50
ACACGATCGC CGCTATGCTA TTGATGCTGA GAAGATTGGT CGCGCATTGG 100
<210> 913
<211> 100
<212> DNA
<213> Escherichia coli K-12 MG1655
<220> yhht b3475
<222> 1032-1131
<223> yhht b3475 U00096 3609415..3610545
<313> 913: 1102-1121
<400> 913
AACCAGCGTG TGTAAAATCT GGATGGAAAC CACCAAAGGC GGTAGCAAAC 50
TGGCGATTTT ACTGGGGCCG GGCAGACCGA AAAGTCGGTT ACCGGGATGA 100
<210> 914
<211> 100
<212> DNA
<213> Escherichia coli K-12 MG1655
<220> yhht b3475
<222> 1032-1131
<223> yhht b3475 U00096 3609415..3610545
<313> 914: 1053-1072
<400> 914
AACCAGCGTG TGTAAAATCT GGATGGAAAC CACCAAAGGC GGTAGCAAAC 50
TGGCGATTTT ACTGGGGCCG GGCAGACCGA AAAGTCGGTT ACCGGGATGA 100
<210> 915
<211> 100
<212> DNA
<213> Escherichia coli K-12 MG1655
<220> yhht b3475
<222> 981-1080
<223> yhht b3475 U00096 3609415..3610545
<313> 915: 1018-1037
<400> 915
AATTTGGGGA TGGCTGCTCG GCCCGGTAGG GATGCTACTT TCGGTGCCAT 50
TAACCAGCGT GTGTAAAATC TGGATGGAAA CCACCAAAGG CGGTAGCAAA 100
<210> 916
<211> 100
<212> DNA
<213> Escherichia coli K-12 MG1655
<220> rfbc b2039
<222> 295-394
<223> rfbc b2039 U00096 complement(2107603..2108160)
<313> 916: 340-359
<400> 916
GTTGGTGTTC TGCTTTCAGC TGATAATAAG CAGCAGTTGT GGATACCAAA 50
AGGGTTTGCT CATGGCTTTT TGGTTCTGTC TGATATCGCT GAATTTCAAT 100
<210> 917
<211> 100

<212> DNA
<213> Escherichia coli K-12 MG1655
<220> rfbc b2039
<222> 247-346
<223> rfbc b2039 U00096 complement(2107603..2108160)
<313> 917: 285-304
<400> 917
GATGTTGCTG TTGATATTCG ACCCAATTCG GTATCCTTTG GTAAATGGGT 50
TGGTGTTCTG CTTTCAGCTG ATAATAAGCA GCAGTTGTGG ATACCAAAAG 100
<210> 918
<211> 100
<212> DNA
<213> Escherichia coli K-12 MG1655
<220> rfbc b2039
<222> 180-279
<223> rfbc b2039 U00096 complement(2107603..2108160)
<313> 918: 230-249
<400> 918
CCTTCACTTT CAACGCGGCG AGTACGCACA AGATAAACTT GTACGCTGCA 50
CTCATGGAGC AGTTTTTGAT GTTGCTGTTG ATATTCGACC CAATTCGGTA 100
<210> 919
<211> 100
<212> DNA
<213> Escherichia coli K-12 MG1655
<220> yhhu b3476
<222> 489-588
<223> yhhu b3476 U00096 3610600..3611187
<313> 919: 552-571
<400> 919
CAGCCATTGT CAGCTTGAAA ATTTAAGCCT TGCGATCTGC ACCCCTACTC 50
CCTTTACGCT CACCGCCGAC AGTGTGCAAT GGATCGATTC AGTTAACTGA 100
<210> 920
<211> 100
<212> DNA
<213> Escherichia coli K-12 MG1655
<220> yhhu b3476
<222> 456-555
<223> yhhu b3476 U00096 3610600..3611187
<313> 920: 479-498
<400> 920
CGTAGACAGC ACCTATCACT CCTCGCTGTC AGTCAGCCAT TGTCAGCTTG 50
AAAATTTAAG CCTTGCGATC TGCACCCCTA CTCCCTTTAC GCTCACCGCC 100
<210> 921
<211> 100
<212> DNA
<213> Escherichia coli K-12 MG1655
<220> yhhu b3476
<222> 390-489
<223> yhhu b3476 U00096 3610600..3611187
<313> 921: 444-463
<400> 921
GCGCATCTGG ACGCGCAAAG AAGCCATCGT TAAACAGCGT GGCGGCAGCG 50
CCTGGCAAAT CGTCAGCGTA GACAGCACCT ATCACTCCTC GCTGTCAGTC 100
<210> 922
<211> 100
<212> DNA
<213> Escherichia coli K-12 MG1655
<220> rfbd b2041
<222> 801-900
<223> rfbd b2041 U00096 complement(2109099..2109998)
<313> 922: 847-866
<400> 922
TACAGAAAAA TTTCAGCAGA ACTTTGCGCT TGTCTTGCCT GACTGGCAGG 50
TTGGCGTGAA ACGAATGCTC AATGAATTAT TTACGACTAC AGCAATTTAA 100
<210> 923
<211> 100
<212> DNA
<213> Escherichia coli K-12 MG1655
<220> rfbd b2041
<222> 782-881
<223> rfbd b2041 U00096 complement(2109099..2109998)

<313> 923: 818-837
<400> 923
CACATAACTC TCGCCTTAAT ACAGAAAAAT TTCAGCAGAA CTTTGCGCTT 50
GTCTTGCCTG ACTGGCAGGT TGGCGTGAAA CGAATGCTCA ATGAATTATT 100
<210> 924
<211> 100
<212> DNA
<213> Escherichia coli K-12 MG1655
<220> rfbd b2041
<222> 713-812
<223> rfbd b2041 U00096 complement(2109099..2109998)
<313> 924: 777-796
<400> 924
GCATTCCCCT TGCACTCAAC AAGCTCAACG CAGTACCAAC AACAGCCTAT 50
CCTACACCAG CTCGTCGTCC ACATAACTCT CGCCTTAATA CAGAAAAATT 100
<210> 925
<211> 100
<212> DNA
<213> Escherichia coli K-12 MG1655
<220> b2899 b2900
<222> 561-660
<223> b2899 b2900 U00096 complement(3040509..3041168)
<313> 925: 641-660
<400> 925
CAAACGCATT CCATACAACC ATGCCATCTG GCACGGCTTC GTGCTCGGCG 50
GTAGTGTGTG CCACTTTCTG GCGATCTATT TGTATATTGG GCAGGCGTAA 100
<210> 926
<211> 100
<212> DNA
<213> Escherichia coli K-12 MG1655
<220> b2899 b2900
<222> 524-623
<223> b2899 b2900 U00096 complement(3040509..3041168)
<313> 926: 563-582
<400> 926
GCGTGGTTTA TTCGCTCGGG GTGATTTTCT ACGTCTGCAA ACGCATTCCA 50
TACAACCATG CCATCTGGCA CGGCTTCGTG CTCGGCGGTA GTGTGTGCCA 100
<210> 927
<211> 100
<212> DNA
<213> Escherichia coli K-12 MG1655
<220> b2899 b2900
<222> 500-599
<223> b2899 b2900 U00096 complement(3040509..3041168)
<313> 927: 527-546
<400> 927
GCGTTACCTT ACTGGCGGTA GGCGGCGTGG TTTATTCGCT CGGGGTGATT 50
TTCTACGTCT GCAAACGCAT TCCATACAAC CATGCCATCT GGCACGGCTT 100
<210> 928
<211> 100
<212> DNA
<213> Escherichia coli K-12 MG1655
<220> yhhw b3440
<222> 597-696
<223> yhhw b3440 U00096 complement(3576581..3577276)
<313> 928: 664-683
<400> 928
CTCGACCAGC GATGGTCTGG CAATCTGGGA TGAGCAGGCA ATCTCCATCC 50
ATGCGGATAG CGACAGCGAA GTGTTACTGT TCGATCTGCC GCCGGTTTAA 100
<210> 929
<211> 100
<212> DNA
<213> Escherichia coli K-12 MG1655
<220> yhhw b3440
<222> 576-675
<223> yhhw b3440 U00096 complement(3576581..3577276)
<313> 929: 618-637
<400> 929
CACCATTAAC GGCGTGAAAG CCTCGACCAG CGATGGTCTG GCAATCTGGG 50
ATGAGCAGGC AATCTCCATC CATGCGGATA GCGACAGCGA AGTGTTACTG 100
<210> 930

<211> 100
<212> DNA
<213> Escherichia coli K-12 MG1655
<220> yhhw b3440
<222> 541-640
<223> yhhw b3440 U00096 complement(3576581..3577276)
<313> 930: 579-598
<400> 930
CGCCGCGTCT GGATCCAGGT GGTGAAAGGC AATGTCACCA TTAACGGCGT  50
GAAAGCCTCG ACCAGCGATG GTCTGGCAAT CTGGGATGAG CAGGCAATCT  100
<210> 931
<211> 100
<212> DNA
<213> Escherichia coli K-12 MG1655
<220> yhhx b3441
<222> 939-1038
<223> yhhx b3441 U00096 complement(3577399..3578436)
<313> 931: 1015-1034
<400> 931
GCCAAATTAC GTCAAGGAAT CTGAAGTTCT TACCAATCTG GAAATCCTTG  50
AACGCGGATT TGAGCAAGCC TCTCCCTCCA CAGTAACTCT CGCTAAGTAA  100
<210> 932
<211> 100
<212> DNA
<213> Escherichia coli K-12 MG1655
<220> yhhx b3441
<222> 816-915
<223> yhhx b3441 U00096 complement(3577399..3578436)
<313> 932: 879-898
<400> 932
TTCGGTCGGT GTGCTGGAGT ATGTCAATGA CGAGGGCGTG ACGGTCAGAG  50
AAGAGATGAA GCCGGAGATG GGCGATTACG GGCGCGTTTA TGATGCGTTG  100
<210> 933
<211> 100
<212> DNA
<213> Escherichia coli K-12 MG1655
<220> yhhx b3441
<222> 762-861
<223> yhhx b3441 U00096 complement(3577399..3578436)
<313> 933: 788-807
<400> 933
AACCAGCCTG AAGGCTAATA TTATGCCGGG CGAACCGGGA TTCGCAGCGG  50
ATGATTCGGT CGGTGTGCTG GAGTATGTCA ATGACGAGGG CGTGACGGTC  100
<210> 934
<211> 100
<212> DNA
<213> Escherichia coli K-12 MG1655
<220> yhhy b3442
<222> 390-489
<223> yhhy b3442 U00096 3578769..3579257
<313> 934: 452-471
<400> 934
CTATAAAAAA TACGGCTTTG AAATTGAAGG GACTGGTAAG AAGTACGCAT  50
TGCGTAATGG TGAATATGTC GATGCATATT ATATGGCGCG GGTGAAGTAA  100
<210> 935
<211> 100
<212> DNA
<213> Escherichia coli K-12 MG1655
<220> yhhy b3442
<222> 357-456
<223> yhhy b3442 U00096 3578769..3579257
<313> 935: 417-436
<400> 935
CGTGTTTGTC GATAACGCCC CGGCAATTAA GGTCTATAAA AAATACGGCT  50
TTGAAATTGA AGGGACTGGT AAGAAGTACG CATTGCGTAA TGGTGAATAT  100
<210> 936
<211> 100
<212> DNA
<213> Escherichia coli K-12 MG1655
<220> yhhy b3442
<222> 310-409

<223> yhhy b3442 U00096 3578769..3579257
<313> 936: 340-359
<400> 936
ATTGAAATGT GCGACAACTG GTTGCGGGTA GATCGCATTG AACTAACCGT  50
GTTTGTCGAT AACGCCCCGG CAATTAAGGT CTATAAAAAA TACGGCTTTG  100
<210> 937
<211> 100
<212> DNA
<213> Escherichia coli K-12 MG1655
<220> yhhz b3443
<222> 1054-1153
<223> yhhz b3443 U00096 3579494..3580672
<313> 937: 1109-1128
<400> 937
TTTACTAACA TGCAATCACG AATAGCTAAG GGAATATTAG TAGGTGAAAG  50
CAAAATCACT CCCTGGGCCA TTCCATCTGG CTCAATTTAT CCTCCCATGA  100
<210> 938
<211> 100
<212> DNA
<213> Escherichia coli K-12 MG1655
<220> yhhz b3443
<222> 902-1001
<223> yhhz b3443 U00096 3579494..3580672
<313> 938: 915-934
<400> 938
CTGGAAGATT CAACGCCGAA GACCTTTTAA GAATGAGAAA GGGCAATGTT  50
CCTCTAAATT ATAATGTTCA CCATAAACTA TCTCTAGATG ATGGTGGTAC  100
<210> 939
<211> 100
<212> DNA
<213> Escherichia coli K-12 MG1655
<220> yhhz b3443
<222> 610-709
<223> yhhz b3443 U00096 3579494..3580672
<313> 939: 673-692
<400> 939
GGTGGAGATG CAATAAAAGG CTATGATCAA ATACTAAATG AAAAAACAGC  50
GGGCATAGCG ATAGCAACAG CATCTATTCT TTTAACAAAG CGTTCTAATG  100
<210> 940
<211> 100
<212> DNA
<213> Escherichia coli K-12 MG1655
<220> yhid b3509
<222> 549-648
<223> yhid b3509 U00096 complement(3652885..3653532)
<313> 940: 599-618
<400> 940
GGTCGCCATT GATATCCAGC TTCATGCCAC AACGTCGATA GAAGACCTGT  50
TGCGATTATT AAAAGGTATG GCAGGGGTGA AAGGCGTTTC AATTAGCTAA  100
<210> 941
<211> 100
<212> DNA
<213> Escherichia coli K-12 MG1655
<220> yhid b3509
<222> 509-608
<223> yhid b3509 U00096 complement(3652885..3653532)
<313> 941: 559-578
<400> 941
CTGATTTAGT TTCGTTGCAG GAAAATGAAG ATCATGAAGT GGTCGCCATT  50
GATATCCAGC TTCATGCCAC AACGTCGATA GAAGACCTGT TGCGATTATT  100
<210> 942
<211> 100
<212> DNA
<213> Escherichia coli K-12 MG1655
<220> yhid b3509
<222> 337-436
<223> yhid b3509 U00096 complement(3652885..3653532)
<313> 942: 362-381
<400> 942
AGCGGTATGT ACGAACTGGG TATTTATGGT TCAGTGATGA CCTTGTTGGT  50
GCTGGAAGTC TTCCATCAAT TAACCTTCCG CCTGATGAAC AAAAATTACC  100

<210> 943
<211> 100
<212> DNA
<213> Escherichia coli K-12 MG1655
<220> yhie b3513
<222> 429-528
<223> yhie b3513 U00096 3655996..3656523
<313> 943: 492-511
<400> 943
GCAACAAACA TTAAAGATTC AGAAATTCAA CATTATGTAC AAGCTGAAAC  50
TAAGACGTAT GAGCGACATC GTCACCCTGG GTATCACATC TTATTTTTAG  100
<210> 944
<211> 100
<212> DNA
<213> Escherichia coli K-12 MG1655
<220> yhie b3513
<222> 429-528
<223> yhie b3513 U00096 3655996..3656523
<313> 944: 485-504
<400> 944
GCAACAAACA TTAAAGATTC AGAAATTCAA CATTATGTAC AAGCTGAAAC  50
TAAGACGTAT GAGCGACATC GTCACCCTGG GTATCACATC TTATTTTTAG  100
<210> 945
<211> 100
<212> DNA
<213> Escherichia coli K-12 MG1655
<220> yhie b3513
<222> 429-528
<223> yhie b3513 U00096 3655996..3656523
<313> 945: 480-499
<400> 945
GCAACAAACA TTAAAGATTC AGAAATTCAA CATTATGTAC AAGCTGAAAC  50
TAAGACGTAT GAGCGACATC GTCACCCTGG GTATCACATC TTATTTTTAG  100
<210> 946
<211> 100
<212> DNA
<213> Escherichia coli K-12 MG1655
<220> mesj b0189
<222> 1200-1299
<223> mesj b0189 U00096 212331..213629
<313> 946: 1260-1279
<400> 946
CGAAACGCTG ATTGCGGCGG CAGGGGTATT TGTGACGCAA GAAGGTGTGG  50
CTGAAGGTGA GAATGGCGTA AGTTTTGTCT GGCAGAAAAC GCTTAGTTAA  100
<210> 947
<211> 100
<212> DNA
<213> Escherichia coli K-12 MG1655
<220> mesj b0189
<222> 1174-1273
<223> mesj b0189 U00096 212331..213629
<313> 947: 1222-1241
<400> 947
GACACCACGC CACTGCTGTT TTATGGCGAA ACGCTGATTG CGGCGGCAGG  50
GGTATTTGTG ACGCAAGAAG GTGTGGCTGA AGGTGAGAAT GGCGTAAGTT  100
<210> 948
<211> 100
<212> DNA
<213> Escherichia coli K-12 MG1655
<220> mesj b0189
<222> 1138-1237
<223> mesj b0189 U00096 212331..213629
<313> 948: 1194-1213
<400> 948
ATCTGGCAAG AGCTGGGCGT GCCGCCGTGG CTACGTGACA CCACGCCACT  50
GCTGTTTTAT GGCGAAACGC TGATTGCGGC GGCAGGGGTA TTTGTGACGC  100
<210> 949
<211> 100
<212> DNA
<213> Escherichia coli K-12 MG1655
<220> yhif b3508

<222> 432-531
<223> yhif b3508 U00096 3652313..3652843
<313> 949: 486-505
<400> 949
AAAAACGTTC TACTGCCATA AACACAATAT CATGATGATC CTCAATCTTA 50
AGCGGATCAA TGAGCTGGTA CGCCATCAGC ATATTGATTA TCTGGTGTGA 100
<210> 950
<211> 100
<212> DNA
<213> Escherichia coli K-12 MG1655
<220> yhif b3508
<222> 342-441
<223> yhif b3508 U00096 3652313..3652843
<313> 950: 380-399
<400> 950
GTCACTTTCG CGCAAGCAAC ATCAGGTTCT GAGCTGCATT GCGAATCAAA 50
TGACAACGGA AGATATTCTG GAGAAACTGA AAATATCGCT AAAAACGTTC 100
<210> 951
<211> 100
<212> DNA
<213> Escherichia coli K-12 MG1655
<220> yhif b3508
<222> 270-369
<223> yhif b3508 U00096 3652313..3652843
<313> 951: 310-329
<400> 951
GCGGACTATC ACTATCATTG ATTTTGTCGC ACTCATCAAT GGCAGTTACT 50
GCTCTGTGCC TGAAGCGGCT GTGTCACTTT CGCGCAAGCA ACATCAGGTT 100
<210> 952
<211> 100
<212> DNA
<213> Escherichia coli K-12 MG1655
<220> yhih b3487
<222> 2586-2685
<223> yhih b3487 U00096 complement(3624434..3627118)
<313> 952: 2603-2622
<400> 952
GGATCTGACT GATTTGTGGC AACTTTTTAT CCCGTTACTG ATAGCCATCC 50
CGCTGGTGAT GGGCTTAAGT ATCCTGCTGC TGAAAAAACA GGAGGGATGA 100
<210> 953
<211> 100
<212> DNA
<213> Escherichia coli K-12 MG1655
<220> yhih b3487
<222> 2478-2577
<223> yhih b3487 U00096 complement(3624434..3627118)
<313> 953: 2534-2553
<400> 953
GATGATCGAT CCGGTAGCTT CGCTGGAAGG GCCTGGACGT TGGATCGGCG 50
AGGTTTACCC GACCAGTCAT TTTCTGACTA TCGCCCGCGG GACGTTCTCG 100
<210> 954
<211> 100
<212> DNA
<213> Escherichia coli K-12 MG1655
<220> yhih b3487
<222> 2387-2486
<223> yhih b3487 U00096 complement(3624434..3627118)
<313> 954: 2439-2458
<400> 954
GGCTGCTGAT CTCCACCTTT ATGAAAAGCC AGATTGCCGC CATTTTCGGA 50
ACGGCGATTA TCACGTTGAT CCCGGCGACA CAGTTTTCCG GGATGATCGA 100
<210> 955
<211> 100
<212> DNA
<213> Escherichia coli K-12 MG1655
<220> rfbx b2038
<222> 1149-1248
<223> rfbx b2038 U00096 complement(2106359..2107606)
<313> 955: 1195-1214
<400> 955
TAAAGAGATT GGTGCAGCAA TAACATTGCT TGCAACAGAG TGCTTAGTTA 50

CGTCACTCAT GCTGATGTTC GTAAGAAATA ATAAATTACT GGTTTGCTGA 100
<210> 956
<211> 100
<212> DNA
<213> Escherichia coli K-12 MG1655
<220> rfbx b2038
<222> 1073-1172
<223> rfbx b2038 U00096 complement(2106359..2107606)
<313> 956: 1107-1126
<400> 956
AGAAAGAATT CAGTAAGATT TTAATCGCTG CGGGTTTGTT GAGTTTGTTG 50
TTGATTTTTC CGCTAACAAC TCTTTTTAAA GAGATTGGTG CAGCAATAAC 100
<210> 957
<211> 100
<212> DNA
<213> Escherichia coli K-12 MG1655
<220> rfbx b2038
<222> 728-827
<223> rfbx b2038 U00096 complement(2106359..2107606)
<313> 957: 789-808
<400> 957
GACCAACGTC CGTAGGGAAT TTTAATGCGG CCAATACTAT AAGAAACGCG 50
CTTCAAGGGC TATTAAATCC TATCACCCAA GCAATATACC CAAGAATATC 100
<210> 958
<211> 100
<212> DNA
<213> Escherichia coli K-12 MG1655
<220> yhii b3488
<222> 969-1068
<223> yhii b3488 U00096 complement(3627165..3628232)
<313> 958: 1027-1046
<400> 958
TCTGGAATAT GTCAAAACCG GTTTGCCGGG CGTAGCGTGG GTGCGGGTGA 50
ATGAAGAACT TCCGTGGCCT GACGACCTCG TGGTGAGGTT GCCGCAATGA 100
<210> 959
<211> 100
<212> DNA
<213> Escherichia coli K-12 MG1655
<220> yhii b3488
<222> 899-998
<223> yhii b3488 U00096 complement(3627165..3628232)
<313> 959: 951-970
<400> 959
CCAGCGATGA ACGGCTGAAA CTGATGTTCC GCGTCAAAGC GCGTATCCCA 50
CCGGAATTAC TCCAGCAGCA TCTGGAATAT GTCAAAACCG GTTTGCCGGG 100
<210> 960
<211> 100
<212> DNA
<213> Escherichia coli K-12 MG1655
<220> yhii b3488
<222> 866-965
<223> yhii b3488 U00096 complement(3627165..3628232)
<313> 960: 923-942
<400> 960
GTGTCGCCCA GTTCACGCCA AAAACCGTCG AAACCAGCGA TGAACGGCTG 50
AAACTGATGT TCCGCGTCAA AGCGCGTATC CCACCGGAAT TACTCCAGCA 100
<210> 961
<211> 100
<212> DNA
<213> Escherichia coli K-12 MG1655
<220> yhij b3489
<222> 1514-1613
<223> yhij b3489 U00096 complement(3628598..3630220)
<313> 961: 1537-1556
<400> 961
ACCCTGGTGC AGGGCCTGAG ATAACCACTC CCTGGAAGTC TTACCAGCAT 50
GAAAAAAACA GAAATGGACT TATGCCTTTG ATTAGAAGGC GCACAATGTT 100
<210> 962
<211> 100
<212> DNA
<213> Escherichia coli K-12 MG1655

<220> yhij b3489
<222> 1427-1526
<223> yhij b3489 U00096 complement(3628598..3630220)
<313> 962: 1462-1481
<400> 962
TAGGCGAAAC GGGGACAGTT TTAGGTCTTA ACGCCCGTGA CAGTAAACAT  50
GCTGGAAGAA TGTTCAGCAT GCATAATTGC CAAATTAACC CTGGTGCAGG  100
<210> 963
<211> 100
<212> DNA
<213> Escherichia coli K-12 MG1655
<220> yhij b3489
<222> 1376-1475
<223> yhij b3489 U00096 complement(3628598..3630220)
<313> 963: 1411-1430
<400> 963
TAGGTATAAA TCAAGTTGTT GCACATAACG GAAATGGTGG AAGAGAACGC  50
ATAGGCGAAA CGGGGACAGT TTTAGGTCTT AACGCCCGTG ACAGTAAACA  100
<210> 964
<211> 100
<212> DNA
<213> Escherichia coli K-12 MG1655
<220> yhik b3490
<222> 182-281
<223> yhik b3490 U00096 complement(3630482..3630853)
<313> 964: 209-228
<400> 964
GTGGGGAGAC ACTCAAAGGT GAAGACCCCA GCAAATACAT TAGACAAAAA  50
TATGGGCATC ATGGGCCCGG GGTAGATCAT AATCAACAAT TTGATAATGG  100
<210> 965
<211> 100
<212> DNA
<213> Escherichia coli K-12 MG1655
<220> yhik b3490
<222> 186-285
<223> yhik b3490 U00096 complement(3630482..3630853)
<313> 965: 205-224
<400> 965
GGAGACACTC AAAGGTGAAG ACCCCAGCAA ATACATTAGA CAAAAATATG  50
GGCATCATGG GCCCGGGGTA GATCATAATC AACAATTTGA TAATGGTATT  100
<210> 966
<211> 100
<212> DNA
<213> Escherichia coli K-12 MG1655
<220> yhik b3490
<222> 165-264
<223> yhik b3490 U00096 complement(3630482..3630853)
<313> 966: 201-220
<400> 966
TGAACGTATC TTCATATGTG GGGAGACACT CAAAGGTGAA GACCCCAGCA  50
AATACATTAG ACAAAAATAT GGGCATCATG GGCCCGGGGT AGATCATAAT  100
<210> 967
<211> 100
<212> DNA
<213> Escherichia coli K-12 MG1655
<220> yhil b3491
<222> 681-780
<223> yhil b3491 U00096 complement(3630850..3632088)
<313> 967: 712-731
<400> 967
TTATGCATTG CTCGTTTCAG GGCATATAGG CATCAGGGAA AAGGGTTGGA  50
GTTTATTATG TGAATTATTA AAACACGAAG AGATGGCTTC ATCTGCATAC  100
<210> 968
<211> 100
<212> DNA
<213> Escherichia coli K-12 MG1655
<220> yhil b3491
<222> 633-732
<223> yhil b3491 U00096 complement(3630850..3632088)
<313> 968: 660-679
<400> 968

CAACACGGCT TATTTTGGCG ATACAGATGG AAGAGTAGGC GCTGTACTTT 50
ATGCATTGCT CGTTTCAGGG CATATAGGCA TCAGGGAAAA GGGTTGGAGT 100
<210> 969
<211> 100
<212> DNA
<213> Escherichia coli K-12 MG1655
<220> yhil b3491
<222> 603-702
<223> yhil b3491 U00096 complement(3630850..3632088)
<313> 969: 627-646
<400> 969
ACAGTGTGGT GTGATTAACC CACAAAATGC CAACACGGCT TATTTTGGCG 50
ATACAGATGG AAGAGTAGGC GCTGTACTTT ATGCATTGCT CGTTTCAGGG 100
<210> 970
<211> 100
<212> DNA
<213> Escherichia coli K-12 MG1655
<220> yhim b3492
<222> 953-1052
<223> yhim b3492 U00096 3632372..3633523
<313> 970: 997-1016
<400> 970
GTTCGTTAGC CAACAGAATA CCGATGATTC CCGTCTTCAC CTGCCTGTTT 50
TGCCTTTTCC TGGCATCGTT TCTTGCGGAA ATGGCGCAGA CCGACATGGG 100
<210> 971
<211> 100
<212> DNA
<213> Escherichia coli K-12 MG1655
<220> yhim b3492
<222> 933-1032
<223> yhim b3492 U00096 3632372..3633523
<313> 971: 969-988
<400> 971
ACTGGTATGG AGACGTACCT GTTCGTTAGC CAACAGAATA CCGATGATTC 50
CCGTCTTCAC CTGCCTGTTT TGCCTTTTCC TGGCATCGTT TCTTGCGGAA 100
<210> 972
<211> 100
<212> DNA
<213> Escherichia coli K-12 MG1655
<220> yhim b3492
<222> 863-962
<223> yhim b3492 U00096 3632372..3633523
<313> 972: 922-941
<400> 972
CTCCCGGCAT TATTCTTATT TGCCTCGGAA TGATCTGTTA CAGCATATTC 50
TCAAAAGTCT GGCTACTGGC ACTGGTATGG AGACGTACCT GTTCGTTAGC 100
<210> 973
<211> 100
<212> DNA
<213> Escherichia coli K-12 MG1655
<220> yhin b3493
<222> 1104-1203
<223> yhin b3493 U00096 complement(3633838..3635040)
<313> 973: 1132-1151
<400> 973
GATGGACGTC ACCGGCTGGC TGGGGGGCTA TAACTTCCAG TGGGCGTGGT 50
CGAGTGCGTG GGCTTGTGCG CAGGATTTGA TTGCAGCAAA GTCGTCCTGA 100
<210> 974
<211> 100
<212> DNA
<213> Escherichia coli K-12 MG1655
<220> yhin b3493
<222> 1011-1110
<223> yhin b3493 U00096 complement(3633838..3635040)
<313> 974: 1030-1049
<400> 974
CGAAGTGACG CTCGGCGGCG TGGACACCAA CGAACTCTCT TCACGGACGA 50
TGGAAGCGCG CAAAGTGCCT GGGCTGTACT TCATCGGCGA AGTGATGGAC 100
<210> 975
<211> 100
<212> DNA

<213> Escherichia coli K-12 MG1655
<220> yhin b3493
<222> 973-1072
<223> yhin b3493 U00096 complement(3633838..3635040)
<313> 975: 1002-1021
<400> 975
CGCGTACAAC CCAACGGCAC TGAAGGCTAT CGCACTGCCG AAGTGACGCT 50
CGGCGGCGTG GACACCAACG AACTCTCTTC ACGGACGATG GAAGCGCGCA 100
<210> 976
<211> 100
<212> DNA
<213> Escherichia coli K-12 MG1655
<220> yhio b3495
<222> 220-319
<223> yhio b3495 U00096 complement(3637015..3637350)
<313> 976: 280-299
<400> 976
CATCACGATG ATGAATTTAT TCGCCGCTGT GAGCGGGTGC GTCGGCAGTT 50
TATTCTGACC AGCGCATTGT GTGGTCTGGT GGTGGTCAGC CTGATTGCAT 100
<210> 977
<211> 100
<212> DNA
<213> Escherichia coli K-12 MG1655
<220> yhio b3495
<222> 1-100
<223> yhio b3495 U00096 complement(3637015..3637350)
<313> 977: 31-50
<400> 977
ATGATAAGCA CCGTCGCATT ATTTTGGGCT TTATGTGTCG TTTGCATTGT 50
TAACATGGCG CGCTATTTCT CATCACTACG CGCGTTGTTA GTGGTACTGC 100
<210> 978
<211> 100
<212> DNA
<213> Escherichia coli K-12 MG1655
<220> yhio b3495
<222> 1-100
<223> yhio b3495 U00096 complement(3637015..3637350)
<313> 978: 16-35
<400> 978
ATGATAAGCA CCGTCGCATT ATTTTGGGCT TTATGTGTCG TTTGCATTGT 50
TAACATGGCG CGCTATTTCT CATCACTACG CGCGTTGTTA GTGGTACTGC 100
<210> 979
<211> 100
<212> DNA
<213> Escherichia coli K-12 MG1655
<220> yhip b3497
<222> 1371-1470
<223> yhip b3497 U00096 3638492..3639961
<313> 979: 1436-1455
<400> 979
GTTTGGTAAG ATTGGTCTGG TCACGCTGGG CGTTGCAGTA GTGATGCTGT 50
TGATGGTGCC GTGGCTGAAA CGCATGATTG CGACGCCGGA AAGCCATTAA 100
<210> 980
<211> 100
<212> DNA
<213> Escherichia coli K-12 MG1655
<220> yhip b3497
<222> 1292-1391
<223> yhip b3497 U00096 3638492..3639961
<313> 980: 1331-1350
<400> 980
GCGGCTATGT GGCAACATTT ACCGCGGTGC CGGACAACAT TACCGATCCG 50
CTTGAGACGT TGCCCGTCTA TACCAACGTG TTTGGTAAGA TTGGTCTGGT 100
<210> 981
<211> 100
<212> DNA
<213> Escherichia coli K-12 MG1655
<220> yhip b3497
<222> 1171-1270
<223> yhip b3497 U00096 3638492..3639961
<313> 981: 1201-1220

<400> 981
TTAGGTGAAC TGTTTATTAG CGCCCTTGGC CTGGCGATGA TTGCTGCCCT 50
GGTGCCGCAG CATTTGATGG GCTTTATTCT CGGGATGTGG TTCCTGACGC 100
<210> 982
<211> 100
<212> DNA
<213> Escherichia coli K-12 MG1655
<220> yhiq b3498
<222> 654-753
<223> yhiq b3498 U00096 complement(3640010..3640762)
<313> 982: 722-741
<400> 982
GCGCCCGGAC TACGCGCCAC CGCTGGCGAA TGTCGCCACG CCAAACGCGG 50
TAGTCACTAA AGGGCATCGC TTTGATATCT ATGCAGGTAC GCCAGTGTAA 100
<210> 983
<211> 100
<212> DNA
<213> Escherichia coli K-12 MG1655
<220> yhiq b3498
<222> 569-668
<223> yhiq b3498 U00096 complement(3640010..3640762)
<313> 983: 603-622
<400> 983
TTCAGTCGCT GGTGGGACCG GATCTTGATG CCGATGGATT ACTGGAGCCT 50
GCACGCTTGC TGGCGACCAA ACGCGTGGTG GTAAAGCGCC CGGACTACGC 100
<210> 984
<211> 100
<212> DNA
<213> Escherichia coli K-12 MG1655
<220> yhiq b3498
<222> 460-559
<223> yhiq b3498 U00096 complement(3640010..3640762)
<313> 984: 518-537
<400> 984
AGCCTGACGG CGCTGACTGA TATTACCCCG CGCCCGCAGG TGGTTTATCT 50
CGACCCGATG TTCCCGCATA AGCAGAAAAG CGCGCTAGTG AAAAAAGAGA 100
<210> 985
<211> 100
<212> DNA
<213> Escherichia coli K-12 MG1655
<220> yhir b3500
<222> 702-801
<223> yhir b3500 U00096 3643015..3643857
<313> 985: 754-773
<400> 985
CATGACCGCT TCCGGCATGA TTGTGATTAA CCCGCCGTGG AAACTGGAAC 50
AACAGATGAA TAACGTGCTG CCGTGGCTGC ACAGCAAACT GGTTCCGGCA 100
<210> 986
<211> 100
<212> DNA
<213> Escherichia coli K-12 MG1655
<220> yhir b3500
<222> 584-683
<223> yhir b3500 U00096 3643015..3643857
<313> 986: 618-637
<400> 986
GGTATCCGGT GGTGCTGCGT CAGCAAATTA AGCGCATGAT CCACGATCTG 50
GAAGCGACCG GTATTCGCAA AATTCTGCAA ATTGAACTGG CGGTACTGCC 100
<210> 987
<211> 100
<212> DNA
<213> Escherichia coli K-12 MG1655
<220> yhir b3500
<222> 494-593
<223> yhir b3500 U00096 3643015..3643857
<313> 987: 524-543
<400> 987
CGCCGTATGA AATGAAAACT GACTATCAAG CGGTGGTCAG CGGGATAGCA 50
GAAGGTTACA AACGTTTCGC CACTGGTATT TACGCACTGT GGTATCCGGT 100
<210> 988
<211> 100

<212> DNA
<213> Escherichia coli K-12 MG1655
<220> yhis b3505
<222> 684-783
<223> yhis b3505 U00096 3648921..3649703
<313> 988: 746-765
<400> 988
AACCTTGTCA AGGTTTTATT TCGAAGATCT ACTTAATATA AATTTCACTG 50
ACGCAAATTT AGGGAAGGTG CGAATAAGCG GGGAAATTCT TCTCGGCTGA 100
<210> 989
<211> 100
<212> DNA
<213> Escherichia coli K-12 MG1655
<220> yhis b3505
<222> 179-278
<223> yhis b3505 U00096 3648921..3649703
<313> 989: 223-242
<400> 989
ATGATTTAGC GCAACACCAA CTTCAACAAA TTAATGCGTG CCACACGAAC 50
ACTACATATA CAAATGCAGA CTACTCAAAA GTTGTGGCTC AACTTGTAAG 100
<210> 990
<211> 100
<212> DNA
<213> Escherichia coli K-12 MG1655
<220> yhis b3505
<222> 74-173
<223> yhis b3505 U00096 3648921..3649703
<313> 990: 114-133
<400> 990
ATAATGCTAA AACGCCAGAC ACACCGAAGT GGCCTTGCGA TAATTGGGAA 50
GAACAACAAC CCATTAATTC CACATTCTCT GGAGAAGGAT ATATCTCTGA 100
<210> 991
<211> 100
<212> DNA
<213> Escherichia coli K-12 MG1655
<220> yhiu b3514
<222> 1059-1158
<223> yhiu b3514 U00096 3656862..3658019
<313> 991: 1130-1149
<400> 991
TCGGGTGATC GTTTCCGGTT TGCAACGCAT TCGTCCGGGT ATCAAAGCAC 50
GAGCAATTTC CTCCAGCCAG GAAAACGCCA GCACCGAATC GAAACAATAA 100
<210> 992
<211> 100
<212> DNA
<213> Escherichia coli K-12 MG1655
<220> yhiu b3514
<222> 968-1067
<223> yhiu b3514 U00096 3656862..3658019
<313> 992: 998-1017
<400> 992
ATAAAGACGA TGTCGTGCAG CTACGCGAAA TTGAAGCCAG CAAAGCCATC 50
GGCGACCAGT GGGTCGTCAC CTCTGGCTTG CAGGCTGGCG ATCGGGTGAT 100
<210> 993
<211> 100
<212> DNA
<213> Escherichia coli K-12 MG1655
<220> yhiu b3514
<222> 925-1024
<223> yhiu b3514 U00096 3656862..3658019
<313> 993: 948-967
<400> 993
GTCACCCACA ACGCCCAGGG TAAAGCAACG GCGCTCATTC TGGATAAAGA 50
CGATGTCGTG CAGCTACGCG AAATTGAAGC CAGCAAAGCC ATCGGCGACC 100
<210> 994
<211> 100
<212> DNA
<213> Escherichia coli K-12 MG1655
<220> yhiv b3515
<222> 2901-3000
<223> yhiv b3515 U00096 3658044..3661157

<313> 994: 2919-2938
<400> 994
GATGCGTTTA CGCCCAATCC TGATGACCTC TCTGGCCTTT ATTCTCGGCG  50
TGCTGCCGCT GGTTATCAGT CATGGTGCCG GTTCTGGCGC GCAAAACGCG  100
<210> 995
<211> 100
<212> DNA
<213> Escherichia coli K-12 MG1655
<220> yhiv b3515
<222> 2806-2905
<223> yhiv b3515 U00096 3658044..3661157
<313> 995: 2858-2877
<400> 995
TCCGCCAAAA ACGCCATCCT GATTGTCGAA TTTGCCGTTG AGATGATGCA  50
GAAAGAAGGG AAAACGCCGA TAGAGGCAAT CATCGAAGCG GCGCGGATGC  100
<210> 996
<211> 100
<212> DNA
<213> Escherichia coli K-12 MG1655
<220> yhiv b3515
<222> 2788-2887
<223> yhiv b3515 U00096 3658044..3661157
<313> 996: 2818-2837
<400> 996
CTGACCACCA TCGGGCTTTC CGCCAAAAAC GCCATCCTGA TTGTCGAATT  50
TGCCGTTGAG ATGATGCAGA AAGAAGGGAA AACGCCGATA GAGGCAATCA  100
<210> 997
<211> 100
<212> DNA
<213> Escherichia coli K-12 MG1655
<220> yhiw b3516
<222> 383-482
<223> yhiw b3516 U00096 complement(3661520..3662248)
<313> 997: 403-422
<400> 997
CCTTACTTTT CAATAGCATC AGCACTGTTT CAGGAAAAGT TGAACGCCTT  50
ATTAGCTTTG ATATCGCCAA ACGTTGGTAT CTGCGCGATA TCGCGGAAAG  100
<210> 998
<211> 100
<212> DNA
<213> Escherichia coli K-12 MG1655
<220> yhiw b3516
<222> 252-351
<223> yhiw b3516 U00096 complement(3661520..3662248)
<313> 998: 296-315
<400> 998
GTCACCCCCC ATGCCGCTGG TGACGGCGAT TTTTGATAGC TTCAATGAAT  50
CCGGGGTAAA TTCACCGATA CTGAGCAATA TGCTCTACCT TTCCTGTTTA  100
<210> 999
<211> 100
<212> DNA
<213> Escherichia coli K-12 MG1655
<220> yhiw b3516
<222> 170-269
<223> yhiw b3516 U00096 complement(3661520..3662248)
<313> 999: 212-231
<400> 999
CAGTTTGCCA TTACTTACAA AATATTCGTC AGCTACCACG CAATTTAGGG  50
TTACACAGCA AAGACCGTTT GTTAATTAAC CAGTCACCCC CCATGCCGCT  100
<210> 1000
<211> 100
<212> DNA
<213> Escherichia coli K-12 MG1655
<220> yhix b3517
<222> 716-815
<223> yhix b3517 U00096 complement(3662616..3663440)
<313> 1000: 734-753
<400> 1000
ATCAGGAGCG ATCGGCGCAG AGATTGTCGA ACCGTGACTC GGCGGCAAGT  50
ATTGTTGCGC AAGGGAATTT TTACGGCACT GACCGTTCTG CGGAAGGAAT  100
<210> 1001

<211> 100
<212> DNA
<213> Escherichia coli K-12 MG1655
<220> yhix b3517
<222> 657-756
<223> yhix b3517 U00096 complement(3662616..3663440)
<313> 1001: 693-712
<400> 1001
CAGCGTGTCG TATTTCATTT ACGTCTTTCG AAATTATTAT GGGATGACGC 50
CCACAGAGTA TCAGGAGCGA TCGGCGCAGA GATTGTCGAA CCGTGACTCG 100
<210> 1002
<211> 100
<212> DNA
<213> Escherichia coli K-12 MG1655
<220> yhix b3517
<222> 611-710
<223> yhix b3517 U00096 complement(3662616..3663440)
<313> 1002: 648-667
<400> 1002
TACATGGTTT TTCAATTAAG CGAGTTGCAG TATCCTGTGG ATATCACAGC 50
GTGTCGTATT TCATTTACGT CTTTCGAAAT TATTATGGGA TGACGCCCAC 100
<210> 1003
<211> 100
<212> DNA
<213> Escherichia coli K-12 MG1655
<220> meta b4014
<222> 831-930
<223> meta b4014 U00096 4211859..4212788
<313> 1003: 900-919
<400> 1003
GCGTAGTCAC GGTAATTTAC TGTTTACCAA CTGGCTCAAC TATTACGTCT 50
ACCAGATCAC GCCATACGAT CTACGGCACA TGAATCCAAC GCTGGATTAA 100
<210> 1004
<211> 100
<212> DNA
<213> Escherichia coli K-12 MG1655
<220> meta b4014
<222> 831-930
<223> meta b4014 U00096 4211859..4212788
<313> 1004: 883-902
<400> 1004
GCGTAGTCAC GGTAATTTAC TGTTTACCAA CTGGCTCAAC TATTACGTCT 50
ACCAGATCAC GCCATACGAT CTACGGCACA TGAATCCAAC GCTGGATTAA 100
<210> 1005
<211> 100
<212> DNA
<213> Escherichia coli K-12 MG1655
<220> meta b4014
<222> 831-930
<223> meta b4014 U00096 4211859..4212788
<313> 1005: 884-903
<400> 1005
GCGTAGTCAC GGTAATTTAC TGTTTACCAA CTGGCTCAAC TATTACGTCT 50
ACCAGATCAC GCCATACGAT CTACGGCACA TGAATCCAAC GCTGGATTAA 100
<210> 1006
<211> 100
<212> DNA
<213> Escherichia coli K-12 MG1655
<220> metb b3940
<222> 1062-1161
<223> metb b3940 U00096 4126252..4127412
<313> 1006: 1112-1131
<400> 1006
GATCTCCGAG ACGCTGCTGC GTATCTCCAC CGGTATTGAA GATGGCGAAG 50
ATTTAATTGC CGACCTGGAA AATGGCTTCC GGGCTGCAAA CAAGGGGTAA 100
<210> 1007
<211> 100
<212> DNA
<213> Escherichia coli K-12 MG1655
<220> metb b3940
<222> 975-1074

<223> metb b3940 U00096 4126252..4127412
<313> 1007: 1021-1040
<400> 1007
ATCATTAGGG GGAGTGGAAA GTTTAATCTC TCACGCCGCA ACCATGACAC 50
ATGCAGGCAT GGCACCAGAA GCGCGTGCTG CCGCCGGGAT CTCCGAGACG 100
<210> 1008
<211> 100
<212> DNA
<213> Escherichia coli K-12 MG1655
<220> metb b3940
<222> 882-981
<223> metb b3940 U00096 4126252..4127412
<313> 1008: 935-954
<400> 1008
AGGCTTTGGC GCAATGTTGA GTTTTGAACT GGATGGCGAT GAGCAGACGC 50
TGCGTCGTTT CCTGGGCGGG CTGTCGTTGT TTACGCTGGC GGAATCATTA 100
<210> 1009
<211> 100
<212> DNA
<213> Escherichia coli K-12 MG1655
<220> metc b3009
<222> 1071-1170
<223> metc b3009 U00096 3150251..3151438
<313> 1009: 1108-1127
<400> 1009
CATTCGCCCA CAAGGCGAGA TCGATTTTAG CGGGACCTTG ATTCGCCTGC 50
ATATTGGTCT GGAAGATGTC GACGATCTGA TTGCCGATCT GGACGCCGGT 100
<210> 1010
<211> 100
<212> DNA
<213> Escherichia coli K-12 MG1655
<220> metc b3009
<222> 1024-1123
<223> metc b3009 U00096 3150251..3151438
<313> 1010: 1054-1073
<400> 1010
GGGTATGAAT CGTTGATCCT GGCAAATCAA CCAGAACATA TCGCCGCCAT 50
TCGCCCACAA GGCGAGATCG ATTTTAGCGG GACCTTGATT CGCCTGCATA 100
<210> 1011
<211> 100
<212> DNA
<213> Escherichia coli K-12 MG1655
<220> metc b3009
<222> 879-978
<223> metc b3009 U00096 3150251..3151438
<313> 1011: 930-949
<400> 1011
CAGTAAAGGT CACGAATTCT GGAAACGAGA CTTTACAGGC AGCAGCGGGC 50
TATTTTCCTT TGTGCTTAAG AAAAAACTCA ATAATGAAGA GCTGGCGAAC 100
<210> 1012
<211> 100
<212> DNA
<213> Escherichia coli K-12 MG1655
<220> nlp b3189
<222> 144-243
<223> nlp b3189 U00096 3332550..3332828
<313> 1012: 162-181
<400> 1012
AGGAGAGATG ATTATTGCGA AAGCCCTGGG AACTGACCCC TGGGTTATCT 50
GGCCATCACG CTACCATGAT CCGCAGACCC ATGAGTTTAT CGACAGAACG 100
<210> 1013
<211> 100
<212> DNA
<213> Escherichia coli K-12 MG1655
<220> nlp b3189
<222> 59-158
<223> nlp b3189 U00096 3332550..3332828
<313> 1013: 78-97
<400> 1013
AGGGAACGTC AATGGCGGCG GAATCTCGCA GAAATGGTTT GAGTTCCTCA 50
ACGCTGGCGA ATGCATTATC GCGCCCATGG CCGAAAGGAG AGATGATTAT 100

<210> 1014
<211> 100
<212> DNA
<213> Escherichia coli K-12 MG1655
<220> nlp b3189
<222> 1-100
<223> nlp b3189 U00096 3332550..3332828
<313> 1014: 7-26
<400> 1014
ATGGAAAGTA ATTTCATTGA CTGGCATCCC GCTGACATCA TTGCGGGTTT 50
GCGCAAGAAG GGAACGTCAA TGGCGGCGGA ATCTCGCAGA AATGGTTTGA 100
<210> 1015
<211> 100
<212> DNA
<213> Escherichia coli K-12 MG1655
<220> mete b3830
<222> 2163-2262
<223> mete b3830 U00096 4010643..4012904
<313> 1015: 2220-2239
<400> 1015
GGTCAACCCG GACTGTGGCC TGAAAACGCG CGGCTGGCCA GAAACCCGCG 50
CGGCACTGGC GAACATGGTG CAGGCGGCGC AGAACTTGCG TCGGGGGTAA 100
<210> 1016
<211> 100
<212> DNA
<213> Escherichia coli K-12 MG1655
<220> mete b3830
<222> 2106-2205
<223> mete b3830 U00096 4010643..4012904
<313> 1016: 2130-2149
<400> 1016
ATGGATTGAA GCCTTGCTGA AGAAAGCGGC AAAACGCATT CCGGCAGAGC 50
GCCTGTGGGT CAACCCGGAC TGTGGCCTGA AAACGCGCGG CTGGCCAGAA 100
<210> 1017
<211> 100
<212> DNA
<213> Escherichia coli K-12 MG1655
<220> mete b3830
<222> 2070-2169
<223> mete b3830 U00096 4010643..4012904
<313> 1017: 2087-2106
<400> 1017
CTATGACATT CACTCGCCAA ACGTACCGAG CGTGGAATGG ATTGAAGCCT 50
TGCTGAAGAA AGCGGCAAAA CGCATTCCGG CAGAGCGCCT GTGGGTCAAC 100
<210> 1018
<211> 100
<212> DNA
<213> Escherichia coli K-12 MG1655
<220> yhja b3519
<222> 1285-1384
<223> yhja b3519 U00096 complement(3665421..3666818)
<313> 1018: 1302-1321
<400> 1018
ATGCTGCGCT ATCAGGTAGG CAAAGAGCTG CCGCAGGAGG ATGTGGATGA 50
TATCGTAGCT TTCCTGCACA GTCTGAACGG GGTGTACACG CCGTATATGC 100
<210> 1019
<211> 100
<212> DNA
<213> Escherichia coli K-12 MG1655
<220> yhja b3519
<222> 1236-1335
<223> yhja b3519 U00096 complement(3665421..3666818)
<313> 1019: 1265-1284
<400> 1019
GTACTTCCAT CGCGGTGACG TGCCGACGCT GGACGGGGCG GTGAAACTGA 50
TGCTGCGCTA TCAGGTAGGC AAAGAGCTGC CGCAGGAGGA TGTGGATGAT 100
<210> 1020
<211> 100
<212> DNA
<213> Escherichia coli K-12 MG1655
<220> yhja b3519

<222> 1085-1184
<223> yhja b3519 U00096 complement(3665421..3666818)
<313> 1020: 1135-1154
<400> 1020
GACGTTCCTT TGAACCGTTG GGGCTGAAAA AAGACTTTAA CTTTGGGGAA 50
ATTACGGCGG CGGATATTGG TCGTATGAAT GTGACTAAAG AAGAGCGTGA 100
<210> 1021
<211> 100
<212> DNA
<213> Escherichia coli K-12 MG1655
<220> metf b3942
<222> 792-891
<223> metf b3942 U00096 4130196..4131086
<313> 1021: 865-884
<400> 1021
AAGCCGTGAA GGAGTGAAAG ATTTCCACTT CTATACGCTT AACCGTGCTG 50
AAATGAGTTA CGCGATTTGC CATACGCTGG GGGTTCGACC TGGTTTATAA 100
<210> 1022
<211> 100
<212> DNA
<213> Escherichia coli K-12 MG1655
<220> metf b3942
<222> 668-767
<223> metf b3942 U00096 4130196..4131086
<313> 1022: 723-742
<400> 1022
TTGCCGATAT GACCAACGTG CGTATTCCGG CGTGGATGGC GCAAATGTTC 50
GACGGTCTGG ATGATGATGC CGAAACCCGC AAACTGGTTG GCGCGAATAT 100
<210> 1023
<211> 100
<212> DNA
<213> Escherichia coli K-12 MG1655
<220> metf b3942
<222> 623-722
<223> metf b3942 U00096 4130196..4131086
<313> 1023: 664-683
<400> 1023
TTCCGGGAAT TTTGCCGGTA TCTAACTTTA AACAGGCGAA GAAATTTGCC 50
GATATGACCA ACGTGCGTAT TCCGGCGTGG ATGGCGCAAA TGTTCGACGG 100
<210> 1024
<211> 100
<212> DNA
<213> Escherichia coli K-12 MG1655
<220> yhjb b3521
<222> 504-603
<223> yhjb b3521 U00096 complement(3668922..3669524)
<313> 1024: 568-587
<400> 1024
CAGTACCGGA ACGGTAAAAG CGCATCTGGA ATCCCTGTAT CGCCGTCTGG 50
AAGTGAAAAA TCGCACCCAG GCGGCAATGA TGTTAAATAT CTCCTCCTGA 100
<210> 1025
<211> 100
<212> DNA
<213> Escherichia coli K-12 MG1655
<220> yhjb b3521
<222> 504-603
<223> yhjb b3521 U00096 complement(3668922..3669524)
<313> 1025: 535-554
<400> 1025
CAGTACCGGA ACGGTAAAAG CGCATCTGGA ATCCCTGTAT CGCCGTCTGG 50
AAGTGAAAAA TCGCACCCAG GCGGCAATGA TGTTAAATAT CTCCTCCTGA 100
<210> 1026
<211> 100
<212> DNA
<213> Escherichia coli K-12 MG1655
<220> yhjb b3521
<222> 411-510
<223> yhjb b3521 U00096 complement(3668922..3669524)
<313> 1026: 473-492
<400> 1026
AGATCTCAAA TCGTTAAGCG CCCGCCAGCG GGAGATTTTA ACCATGTTAG 50

CGGCGGGCGA ATCAAATAAG GAGATCGGCA GAGCGCTGAA TATCAGTACC 100
<210> 1027
<211> 100
<212> DNA
<213> Escherichia coli K-12 MG1655
<220> metg b2115
<222> 1905-2004
<223> metg b2115 U00096 2192320..2194353
<313> 1027: 1944-1963
<400> 1027
TAACCTGGCA CCACGTAAAA TGCGCTTCGG TATCTCTGAA GGCATGGTGA 50
TGGCTGCCGG TCCTGGCGGG AAAGATATTT TCCTGCTAAG CCCGGATGCC 100
<210> 1028
<211> 100
<212> DNA
<213> Escherichia coli K-12 MG1655
<220> metg b2115
<222> 1797-1896
<223> metg b2115 U00096 2192320..2194353
<313> 1028: 1836-1855
<400> 1028
GCGCCTGACG CTGGATCTCG GCGGTGAAAA ACGCAATGTC TTCTCCGGTA 50
TTCGTTCTGC TTACCCGGAT CCGCAGGCAC TGATTGGTCG TCACACCATT 100
<210> 1029
<211> 100
<212> DNA
<213> Escherichia coli K-12 MG1655
<220> metg b2115
<222> 1763-1862
<223> metg b2115 U00096 2192320..2194353
<313> 1029: 1786-1805
<400> 1029
ACGCAGAGTT TGTTGAAGGT TCTGACAAAC TGCTGCGCCT GACGCTGGAT 50
CTCGGCGGTG AAAAACGCAA TGTCTTCTCC GGTATTCGTT CTGCTTACCC 100
<210> 1030
<211> 100
<212> DNA
<213> Escherichia coli K-12 MG1655
<220> yhjc b3522
<222> 817-916
<223> yhjc b3522 U00096 3669972..3670943
<313> 1030: 871-890
<400> 1030
GCCCTGCGTG CCGGGCGGCT TATTGAAGTA TTACCTGGCT ACCGTGCCGA 50
GCCGCTCTCC CTTTCGCTGG TTTATCCGCA GCGTCGGGAG CTTTCCCGGC 100
<210> 1031
<211> 100
<212> DNA
<213> Escherichia coli K-12 MG1655
<220> yhjc b3522
<222> 772-871
<223> yhjc b3522 U00096 3669972..3670943
<313> 1031: 828-847
<400> 1031
GCCGGTCTGG GGATTATTCA GATCCCGCGC ATTGCCGTGC GCGAAGCCCT 50
GCGTGCCGGG CGGCTTATTG AAGTATTACC TGGCTACCGT GCCGAGCCGC 100
<210> 1032
<211> 100
<212> DNA
<213> Escherichia coli K-12 MG1655
<220> yhjc b3522
<222> 720-819
<223> yhjc b3522 U00096 3669972..3670943
<313> 1032: 767-786
<400> 1032
TGGCGGCATG TTGACGGTAA ACAGTAGCGA AAACTATCTC ACCGCCGGTC 50
TTGCCGGTCT GGGGATTATT CAGATCCCGC GCATTGCCGT GCGCGAAGCC 100
<210> 1033
<211> 100
<212> DNA
<213> Escherichia coli K-12 MG1655

<220> meth b4020
<222> 3576-3675
<223> meth b4020 U00096 4221407..4225090
<313> 1033: 3610-3629
<400> 1033
AGCACAAATT CAGCGCGATC AGGTTGAAGA TTATGCCCGC CGTAAAGGTA 50
TGAGCGTTAC CGAAGTTGAG CGCTGGCTGG CACCGAATCT GGGGTATGAC 100
<210> 1034
<211> 100
<212> DNA
<213> Escherichia coli K-12 MG1655
<220> meth b4020
<222> 3454-3553
<223> meth b4020 U00096 4221407..4225090
<313> 1034: 3475-3494
<400> 1034
TGGGAGCTGC TGGAAGTGGA AAAACACACT GGCATGAAAC TCACAGAATC 50
TTTCGCCATG TGGCCCGGTG CATCGGTTTC GGGTTGGTAC TTCAGCCACC 100
<210> 1035
<211> 100
<212> DNA
<213> Escherichia coli K-12 MG1655
<220> meth b4020
<222> 3406-3505
<223> meth b4020 U00096 4221407..4225090
<313> 1035: 3447-3466
<400> 1035
GCACCGGGCT ATCCGGCCTG CCCGGAACAT ACGGAAAAAG CCACCATCTG 50
GGAGCTGCTG GAAGTGGAAA AACACACTGG CATGAAACTC ACAGAATCTT 100
<210> 1036
<211> 100
<212> DNA
<213> Escherichia coli K-12 MG1655
<220> yhjd b3523
<222> 915-1014
<223> yhjd b3523 U00096 3670992..3672005
<313> 1036: 991-1010
<400> 1036
TTTCTACTTC TTCGCCCGTT TGACGCTGTT TTGCGCGGCG TGGATTGCCA 50
CCGCCGAATA TAAAGACGAC CCGCGAATGC CGGGGAAAAC GCAGCCTTAA 100
<210> 1037
<211> 100
<212> DNA
<213> Escherichia coli K-12 MG1655
<220> yhjd b3523
<222> 873-972
<223> yhjd b3523 U00096 3670992..3672005
<313> 1037: 929-948
<400> 1037
CTCTGGCGCA GCATTTGGTT CCGTGCTGGG GCTGATGGCA TTTTTCTACT 50
TCTTCGCCCG TTTGACGCTG TTTTGCGCGG CGTGGATTGC CACCGCCGAA 100
<210> 1038
<211> 100
<212> DNA
<213> Escherichia coli K-12 MG1655
<220> yhjd b3523
<222> 845-944
<223> yhjd b3523 U00096 3670992..3672005
<313> 1038: 876-895
<400> 1038
ACACCCTGCC ATCGTTGATG AAATCCCCCT CTGGCGCAGC ATTTGGTTCC 50
GTGCTGGGGC TGATGGCATT TTTCTACTTC TTCGCCCGTT TGACGCTGTT 100
<210> 1039
<211> 100
<212> DNA
<213> Escherichia coli K-12 MG1655
<220> yhje b3524
<222> 1224-1323
<223> yhje b3524 U00096 3672416..3673738
<313> 1039: 1289-1308
<400> 1039

CTACGGGCTA GGTGCGGTGG GGTTATATCT GGCGGCGATG GCTGGCTTGA 50
CGTTAATCGC CCTGCTGCTG ACCCATGAGA CGCGACATCA GTCGTTGTAA 100
<210> 1040
<211> 100
<212> DNA
<213> Escherichia coli K-12 MG1655
<220> yhje b3524
<222> 1176-1275
<223> yhje b3524 U00096 3672416..3673738
<313> 1040: 1226-1245
<400> 1040
TCTCGGGGCT TCCGTTGCGC CATATATCGC AGCCTGGTTG CAGACTAACT 50
ACGGGCTAGG TGCGGTGGGG TTATATCTGG CGGCGATGGC TGGCTTGACG 100
<210> 1041
<211> 100
<212> DNA
<213> Escherichia coli K-12 MG1655
<220> yhje b3524
<222> 1123-1222
<223> yhje b3524 U00096 3672416..3673738
<313> 1041: 1151-1170
<400> 1041
CCGACAGAAG TGCGTTACAC CGGAGCATCG TTCTCTTACA ACGTAGCGTC 50
GATTCTCGGG GCTTCCGTTG CGCCATATAT CGCAGCCTGG TTGCAGACTA 100
<210> 1042
<211> 100
<212> DNA
<213> Escherichia coli K-12 MG1655
<220> metj b3939
<222> 219-318
<223> metj b3939 U00096 complement(4125658..4125975)
<313> 1042: 299-318
<400> 1042
TGATGCCGAT CTGCGTAAAG AGCGCAGCGA CGAAATCCCG GAAGCGGCAA 50
AAGAGATCAT GCGTGAGATG GGGATTAACC CGGAGACGTG GGAATACTAA 100
<210> 1043
<211> 100
<212> DNA
<213> Escherichia coli K-12 MG1655
<220> metj b3939
<222> 191-290
<223> metj b3939 U00096 complement(4125658..4125975)
<313> 1043: 245-264
<400> 1043
ATGCCTTTAC CGGGCAACCT TTGCCGGATG ATGCCGATCT GCGTAAAGAG 50
CGCAGCGACG AAATCCCGGA AGCGGCAAAA GAGATCATGC GTGAGATGGG 100
<210> 1044
<211> 100
<212> DNA
<213> Escherichia coli K-12 MG1655
<220> metj b3939
<222> 153-252
<223> metj b3939 U00096 complement(4125658..4125975)
<313> 1044: 198-217
<400> 1044
CGCTACCAAC AGCGAGCTGC TGTGCGAAGC GTTTCTGCAT GCCTTTACCG 50
GGCAACCTTT GCCGGATGAT GCCGATCTGC GTAAAGAGCG CAGCGACGAA 100
<210> 1045
<211> 100
<212> DNA
<213> Escherichia coli K-12 MG1655
<220> metk b2943
<222> 1010-1109
<223> metk b2943 U00096 3084725..3085879
<313> 1045: 1058-1077
<400> 1045
GCCCATACGG TCTGATTCAG ATGCTGGATC TGCTGCACCC GATCTACAAA 50
GAAACCGCAG CATACGGTCA CTTTGGTCGT GAACATTTCC CGTGGGAAAA 100
<210> 1046
<211> 100
<212> DNA

<213> Escherichia coli K-12 MG1655
<220> metk b2943
<222> 983-1082
<223> metk b2943 U00096 3084725..3085879
<313> 1046: 1002-1021
<400> 1046
TGCTGGTACG TGAGTTCTTC GACCTGCGCC CATACGGTCT GATTCAGATG 50
CTGGATCTGC TGCACCCGAT CTACAAAGAA ACCGCAGCAT ACGGTCACTT 100
<210> 1047
<211> 100
<212> DNA
<213> Escherichia coli K-12 MG1655
<220> metk b2943
<222> 928-1027
<223> metk b2943 U00096 3084725..3085879
<313> 1047: 963-982
<400> 1047
TCCATCATGG TAGAAACTTT CGGTACTGAG AAAGTGCCTT CTGAACAACT 50
GACCCTGCTG GTACGTGAGT TCTTCGACCT GCGCCCATAC GGTCTGATTC 100
<210> 1048
<211> 100
<212> DNA
<213> Escherichia coli K-12 MG1655
<220> yhjg b3525
<222> 1977-2076
<223> yhjg b3525 U00096 complement(3673920..3675995)
<313> 1048: 2036-2055
<400> 1048
AACGCTGGTA ACACCGGCGG CGGCGTTACT GGCACTGATC TCACCTTCCG 50
AAGGGGAGGC TAATCAGTGT CGGACGATTT TGTCGCAGAT GAAGAAGTGA 100
<210> 1049
<211> 100
<212> DNA
<213> Escherichia coli K-12 MG1655
<220> yhjg b3525
<222> 1949-2048
<223> yhjg b3525 U00096 complement(3673920..3675995)
<313> 1049: 2004-2023
<400> 1049
CCCGTGGTGC TGTTGCTGCG GCACTGGCAA CGCTGGTAAC ACCGGCGGCG 50
GCGTTACTGG CACTGATCTC ACCTTCCGAA GGGGAGGCTA ATCAGTGTCG 100
<210> 1050
<211> 100
<212> DNA
<213> Escherichia coli K-12 MG1655
<220> yhjg b3525
<222> 1885-1984
<223> yhjg b3525 U00096 complement(3673920..3675995)
<313> 1050: 1936-1955
<400> 1050
CCGCTGTATG TGCGGGGGAC GTTTAAAAAT CCGCAGGCTG GGGTGAAAGC 50
CGGACCGCTG ATTGCCCGTG GTGCTGTTGC TGCGGCACTG GCAACGCTGG 100
<210> 1051
<211> 100
<212> DNA
<213> Escherichia coli K-12 MG1655
<220> metl b3941
<222> 2273-2372
<223> metl b3941 U00096 4127415..4129847
<313> 1051: 2296-2315
<400> 1051
ATCATCCGTT GGCATCACTG CTGCCGTGCG ATAACGTCTT TGCCATCGAA 50
AGCCGCTGGT ATCGCGATAA CCCTCTGGTG ATCCGCGGAC CTGGCGCTGG 100
<210> 1052
<211> 100
<212> DNA
<213> Escherichia coli K-12 MG1655
<220> metl b3941
<222> 2228-2327
<223> metl b3941 U00096 4127415..4129847
<313> 1052: 2264-2283

<400> 1052
ATGCCAACGG TAAAGCGCGT GTAGGCGTGG AAGCGGTGCG TGAAGATCAT  50
CCGTTGGCAT CACTGCTGCC GTGCGATAAC GTCTTTGCCA TCGAAAGCCG  100
<210> 1053
<211> 100
<212> DNA
<213> Escherichia coli K-12 MG1655
<220> metl b3941
<222> 2141-2240
<223> metl b3941 U00096 4127415..4129847
<313> 1053: 2169-2188
<400> 1053
ATGGCGATGA ACTGAACGAG CAGATGGTGC AACGGCTGGA AGCGGCCCGC  50
GAAATGGGGC TGGTGCTGCG CTACGTGGCG CGTTTCGATG CCAACGGTAA  100
<210> 1054
<211> 100
<212> DNA
<213> Escherichia coli K-12 MG1655
<220> yhjh b3526
<222> 672-771
<223> yhjh b3526 U00096 complement(3676050..3676820)
<313> 1054: 727-746
<400> 1054
GCGTGATGTT CAGAACTCGC CCGCATTCGC CGCACAAGGC TGGTTTCTTT  50
CACGCCCGGC ACCGATAGAA ACGCTGAATA CGGCGGTTCT GGCGCTATAA  100
<210> 1055
<211> 100
<212> DNA
<213> Escherichia coli K-12 MG1655
<220> yhjh b3526
<222> 663-762
<223> yhjh b3526 U00096 complement(3676050..3676820)
<313> 1055: 676-695
<400> 1055
GGAAGAGTGG CGTGATGTTC AGAACTCGCC CGCATTCGCC GCACAAGGCT  50
GGTTTCTTTC ACGCCCGGCA CCGATAGAAA CGCTGAATAC GGCGGTTCTG  100
<210> 1056
<211> 100
<212> DNA
<213> Escherichia coli K-12 MG1655
<220> yhjh b3526
<222> 573-672
<223> yhjh b3526 U00096 complement(3676050..3676820)
<313> 1056: 600-619
<400> 1056
GTCGCCGGAA GGACGCACAC TCTTTTCTCA GCTTTTACAT CTAATGAATC  50
GCTATTGTCG CGGGGTGATT GTCGAGGGCG TAGAAACGCC GGAAGAGTGG  100
<210> 1057
<211> 100
<212> DNA
<213> Escherichia coli K-12 MG1655
<220> yhjj b3528
<222> 1398-1497
<223> yhjj b3528 U00096 complement(3678074..3679570)
<313> 1057: 1438-1457
<400> 1057
CTGGGATCAA ATCATGGCCC CATCTACCGC CGCTGCGACC ACCTCTGTCG  50
CCACGGATGA CGTACATCCT GAAGTGACGG ATATTCCACC TGCACAGTAA  100
<210> 1058
<211> 100
<212> DNA
<213> Escherichia coli K-12 MG1655
<220> yhjj b3528
<222> 1372-1471
<223> yhjj b3528 U00096 complement(3678074..3679570)
<313> 1058: 1404-1423
<400> 1058
TTTAACATGA AAGCGTTGCA GGCGGTCTGG GATCAAATCA TGGCCCCATC  50
TACCGCCGCT GCGACCACCT CTGTCGCCAC GGATGACGTA CATCCTGAAG  100
<210> 1059
<211> 100

<212> DNA
<213> Escherichia coli K-12 MG1655
<220> yhjj b3528
<222> 1340-1439
<223> yhjj b3528 U00096 complement(3678074..3679570)
<313> 1059: 1363-1382
<400> 1059
TAATACTGCT GCAGCCGAAA GGCGAGCCGG AATTTAACAT GAAAGCGTTG 50
CAGGCGGTCT GGGATCAAAT CATGGCCCCA TCTACCGCCG CTGCGACCAC 100
<210> 1060
<211> 100
<212> DNA
<213> Escherichia coli K-12 MG1655
<220> yhjk b3530
<222> 1845-1944
<223> yhjk b3530 U00096 complement(3681260..3683215)
<313> 1060: 1896-1915
<400> 1060
AGCACAACGC GACTGGCTGG CAAAAGCGGG CGTTGGTATT GCCCAGGGCT 50
TCCTTTTTGC TCGCCCACTC CCTATTGAAA TCTTCGAAGA GAGTTACCTG 100
<210> 1061
<211> 100
<212> DNA
<213> Escherichia coli K-12 MG1655
<220> yhjk b3530
<222> 1786-1885
<223> yhjk b3530 U00096 complement(3681260..3683215)
<313> 1061: 1839-1858
<400> 1061
ATTATCATGC TGGCGCAGAG CCTGAACTTA CAAATGATTG CCGAAGGCGT 50
GGAGACTGAA GCACAACGCG ACTGGCTGGC AAAAGCGGGC GTTGGTATTG 100
<210> 1062
<211> 100
<212> DNA
<213> Escherichia coli K-12 MG1655
<220> yhjk b3530
<222> 1712-1811
<223> yhjk b3530 U00096 complement(3681260..3683215)
<313> 1062: 1759-1778
<400> 1062
TGCCAATCGA CGTACTGAAA ATCGACAAAA TGTTTGTTGA AGGCTTGCCG 50
GGAGATAGCA GCATGATTGC TGCAATTATC ATGCTGGCGC AGAGCCTGAA 100
<210> 1063
<211> 100
<212> DNA
<213> Escherichia coli K-12 MG1655
<220> yhjl b3531
<222> 3402-3501
<223> yhjl b3531 U00096 complement(3683330..3686830)
<313> 1063: 3470-3489
<400> 1063
ACCCAGCCAT TTCCTGCTCT ACGTACGTTA TTCCGCCGCC GGATGGCAGG 50
GTGACATGGA TTTACCGCCG CAGCCGCTGA TACCTTACGC CGACTGGTAA 100
<210> 1064
<211> 100
<212> DNA
<213> Escherichia coli K-12 MG1655
<220> yhjl b3531
<222> 3339-3438
<223> yhjl b3531 U00096 complement(3683330..3686830)
<313> 1064: 3387-3406
<400> 1064
ACGTGTTACT TCCAACTGGT TTGTTGGCAC GGCAATTGAT ATCCAGCAGG 50
CGAAAGATTA CGCACCCAGC CATTTCCTGC TCTACGTACG TTATTCCGCC 100
<210> 1065
<211> 100
<212> DNA
<213> Escherichia coli K-12 MG1655
<220> yhjl b3531
<222> 3307-3406
<223> yhjl b3531 U00096 complement(3683330..3686830)

<313> 1065: 3334-3353
<400> 1065
TTCGGCTACA CGGCGCGGGC ATTACTTGAA CGACGTGTTA CTTCCAACTG 50
GTTTGTTGGC ACGGCAATTG ATATCCAGCA GGCGAAAGAT TACGCACCCA 100
<210> 1066
<211> 100
<212> DNA
<213> Escherichia coli K-12 MG1655
<220> yhjm b3532
<222> 1008-1107
<223> yhjm b3532 U00096 complement(3686784..3687890)
<313> 1066: 1066-1085
<400> 1066
GACCCTGTTT GGACAAGGCT GGGATCAACA CCGTTTCCGC TTCTCGACAA 50
AAGGTGAGTT ATTACCTGAC TGGGGCCAGG AATGCGCAAA TTCACACTAA 100
<210> 1067
<211> 100
<212> DNA
<213> Escherichia coli K-12 MG1655
<220> yhjm b3532
<222> 942-1041
<223> yhjm b3532 U00096 complement(3686784..3687890)
<313> 1067: 983-1002
<400> 1067
GGCCGTTCAG CGCCAGCGCG TGGCCGATAA CTTTCCCGGC AGCGATGCCT 50
ATTACAACTA TGTGCTGACC CTGTTTGGAC AAGGCTGGGA TCAACACCGT 100
<210> 1068
<211> 100
<212> DNA
<213> Escherichia coli K-12 MG1655
<220> yhjm b3532
<222> 903-1002
<223> yhjm b3532 U00096 complement(3686784..3687890)
<313> 1068: 949-968
<400> 1068
TGCCGCCATG CTGCCCTTTT TACAAAACCG CGATGCGCAG GCCGTTCAGC 50
GCCAGCGCGT GGCCGATAAC TTTCCCGGCA GCGATGCCTA TTACAACTAT 100
<210> 1069
<211> 100
<212> DNA
<213> Escherichia coli K-12 MG1655
<220> metr b3829
<222> 855-954
<223> metr b3829 U00096 complement(4009453..4010406)
<313> 1069: 925-944
<400> 1069
GCGCAATCAC GCCTGCGATC ATCTGCCGTT TGTGAAGAGC GCGGAGCGAC 50
CCACTTACGA TGCACCCACA GTGAGGCCAG GATCACCAGC GCGCCTGTAA 100
<210> 1070
<211> 100
<212> DNA
<213> Escherichia coli K-12 MG1655
<220> metr b3829
<222> 824-923
<223> metr b3829 U00096 complement(4009453..4010406)
<313> 1070: 869-888
<400> 1070
AGCCAGTTAC GGAAGCGTTT ATTCGCTCAG CGCGCAATCA CGCCTGCGAT 50
CATCTGCCGT TTGTGAAGAG CGCGGAGCGA CCCACTTACG ATGCACCCAC 100
<210> 1071
<211> 100
<212> DNA
<213> Escherichia coli K-12 MG1655
<220> metr b3829
<222> 719-818
<223> metr b3829 U00096 complement(4009453..4010406)
<313> 1071: 781-800
<400> 1071
GGGTAGTAGA GAGTTTTGAG CGCCAGGGTC TGGTGGTGAC CAAAACCCTG 50
GGCGAAGGCT TGTGGAGCCG ACTGTACGCC GCCGTGCGCG ATGGCGAGCA 100
<210> 1072

<211> 100
<212> DNA
<213> Escherichia coli K-12 MG1655
<220> yhjn b3533
<222> 2241-2340
<223> yhjn b3533 U00096 complement(3687897..3690236)
<313> 1072: 2307-2326
<400> 1072
GGCGGTGCTG GCGGCTATCA GTGTGATATT GCTGGCATGG GTACTGTGGC 50
GTCTGCTGCG AATTATTAGT CGTCGTCGTC TTAACCCGGA TAACGAGTAA 100
<210> 1073
<211> 100
<212> DNA
<213> Escherichia coli K-12 MG1655
<220> yhjn b3533
<222> 2118-2217
<223> yhjn b3533 U00096 complement(3687897..3690236)
<313> 1073: 2146-2165
<400> 1073
TTCGGTCGCG GTGATCCGCG AGTCCGGTAT CAACAGCCTA CGTGTTGGCG 50
ACGTTTATTA CGTAGGTCAT CTGCCGTGGT TCGAGCGCGT GTGGTATGCG 100
<210> 1074
<211> 100
<212> DNA
<213> Escherichia coli K-12 MG1655
<220> yhjn b3533
<222> 2049-2148
<223> yhjn b3533 U00096 complement(3687897..3690236)
<313> 1074: 2093-2112
<400> 1074
TAGCCCACGC GGTTATGAAA TGCTTAACGA TGCGGTGAAC GATAGCGGCA 50
AACGCGCCAC CATGTTCGGT TCGGTCGCGG TGATCCGCGA GTCCGGTATC 100
<210> 1075
<211> 100
<212> DNA
<213> Escherichia coli K-12 MG1655
<220> yhjo b3534
<222> 2568-2667
<223> yhjo b3534 U00096 complement(3690247..3692913)
<313> 1075: 2637-2656
<400> 1075
GCTGACTTCT CTGGTTTCCT GGGTTGTATC GTTTATTCCG CGCCGCCCGG 50
AGCGGAGCGA AACGGCACAA CCATCGGATC AGGCTTTGGC TCAACAATGA 100
<210> 1076
<211> 100
<212> DNA
<213> Escherichia coli K-12 MG1655
<220> yhjo b3534
<222> 2545-2644
<223> yhjo b3534 U00096 complement(3690247..3692913)
<313> 1076: 2590-2609
<400> 1076
TCGGTGAAGG GCATATTCCG TGTGCTGACT TCTCTGGTTT CCTGGGTTGT 50
ATCGTTTATT CCGCGCCGCC CGGAGCGGAG CGAAACGGCA CAACCATCGG 100
<210> 1077
<211> 100
<212> DNA
<213> Escherichia coli K-12 MG1655
<220> yhjo b3534
<222> 2493-2592
<223> yhjo b3534 U00096 complement(3690247..3692913)
<313> 1077: 2555-2574
<400> 1077
TCTGAAGCTC GGCTTCCGTG GCTACCGCCA TCTGGCGGAG TTTGCGCCTT 50
CTTCGGTGAA GGGCATATTC CGTGTGCTGA CTTCTCTGGT TTCCTGGGTT 100
<210> 1078
<211> 100
<212> DNA
<213> Escherichia coli K-12 MG1655
<220> yhjq b3535
<222> 630-729

<223> yhjq b3535 U00096 complement(3692862..3693590)
<313> 1078: 682-701
<400> 1078
TCGCAGTGAT GCGCTGGCGG CTGAAGAGAT ACTGACGCTG GCGAACTGGT  50
GCCTGTTGAA CTACTCCGGG CTGAAAACGC CAGTCGGGAG TGCATCATGA  100
<210> 1079
<211> 100
<212> DNA
<213> Escherichia coli K-12 MG1655
<220> yhjq b3535
<222> 571-670
<223> yhjq b3535 U00096 complement(3692862..3693590)
<313> 1079: 615-634
<400> 1079
ATTCATCGTG ATGAAGCGAT GGCTGAATGC CTGGCGGCTA AGCAACCAGT  50
AGGTGAATAT CGCAGTGATG CGCTGGCGGC TGAAGAGATA CTGACGCTGG  100
<210> 1080
<211> 100
<212> DNA
<213> Escherichia coli K-12 MG1655
<220> yhjq b3535
<222> 509-608
<223> yhjq b3535 U00096 complement(3692862..3693590)
<313> 1080: 550-569
<400> 1080
AGGTTCAGGA CGATATTTAC CAGCTTTGGT TGCAAAGCCA GCGCCGATTA  50
CTGCCGATGC TCATTCATCG TGATGAAGCG ATGGCTGAAT GCCTGGCGGC  100
<210> 1081
<211> 100
<212> DNA
<213> Escherichia coli K-12 MG1655
<220> yhjr b3536
<222> 72-171
<223> yhjr b3536 U00096 complement(3693626..3693814)
<313> 1081: 113-132
<400> 1081
GTTAAAGCAG GCATTTTCAC TGCCTGATAT TGATTATGCC GATATTTCCC  50
AACGCGAACA GTTGGCCGCG GCATTAAAAC GCTGGCCGTT GCTGGCAGAG  100
<210> 1082
<211> 100
<212> DNA
<213> Escherichia coli K-12 MG1655
<220> yhjr b3536
<222> 61-160
<223> yhjr b3536 U00096 complement(3693626..3693814)
<313> 1082: 103-122
<400> 1082
GATATTGTGG CGTTAAAGCA GGCATTTTCA CTGCCTGATA TTGATTATGC  50
CGATATTTCC CAACGCGAAC AGTTGGCCGC GGCATTAAAA CGCTGGCCGT  100
<210> 1083
<211> 100
<212> DNA
<213> Escherichia coli K-12 MG1655
<220> yhjr b3536
<222> 12-111
<223> yhjr b3536 U00096 complement(3693626..3693814)
<313> 1083: 27-46
<400> 1083
TGAACCAGAT ACTCTGCCTG ATCCCGCGAT AGGCTATATC TTCCAGAATG  50
ATATTGTGGC GTTAAAGCAG GCATTTTCAC TGCCTGATAT TGATTATGCC  100
<210> 1084
<211> 100
<212> DNA
<213> Escherichia coli K-12 MG1655
<220> yhjs b3537
<222> 1473-1572
<223> yhjs b3537 U00096 3694087..3695658
<313> 1084: 1542-1561
<400> 1084
TTCTAAACCG GTCATCAATG CCGAGCACGA TGGTCGCCAC TGGCGACGAA  50
TACCAGAACC CATGCGACTG TTAGATGATG CTGTGGAGCG CTCATCATGA  100

<210> 1085
<211> 100
<212> DNA
<213> Escherichia coli K-12 MG1655
<220> yhjs b3537
<222> 1396-1495
<223> yhjs b3537 U00096 3694087..3695658
<313> 1085: 1417-1436
<400> 1085
CAAATCAGTG CCGAGCTGGT GCAGATGCGC TTGCTTGCCC CAGAACAATG 50
GGGCATGCCG CTGCCTTTAA CGCAAAGTTC TAAACCGGTC ATCAATGCCG 100
<210> 1086
<211> 100
<212> DNA
<213> Escherichia coli K-12 MG1655
<220> yhjs b3537
<222> 1309-1408
<223> yhjs b3537 U00096 3694087..3695658
<313> 1086: 1344-1363
<400> 1086
AACGATCTGG ATACCGCGTT GAATCATATT TTCCCATTGC CTACTGGCGA 50
CATTTTCTCA AACCGTATGG TCTGGTTTGA AGATGATCAA ATCAGTGCCG 100
<210> 1087
<211> 100
<212> DNA
<213> Escherichia coli K-12 MG1655
<220> yhjt b3538
<222> 90-189
<223> yhjt b3538 U00096 3695658..3695846
<313> 1087: 160-179
<400> 1087
CATTCGCGAC ACCTTACGTT TGTTCTTTGC TAAACCTCGT TATGTTAAAC 50
CGGCCGGGAC GTTACGCCGC ACGGAAAAAG CCAGGGCAAC CAAAAAATGA 100
<210> 1088
<211> 100
<212> DNA
<213> Escherichia coli K-12 MG1655
<220> yhjt b3538
<222> 59-158
<223> yhjt b3538 U00096 3695658..3695846
<313> 1088: 123-142
<400> 1088
TGGGCTATCT GGCGCGGCAC TCTTTGCGAC GCATTCGCGA CACCTTACGT 50
TTGTTCTTTG CTAAACCTCG TTATGTTAAA CCGGCCGGGA CGTTACGCCG 100
<210> 1089
<211> 100
<212> DNA
<213> Escherichia coli K-12 MG1655
<220> yhjt b3538
<222> 45-144
<223> yhjt b3538 U00096 3695658..3695846
<313> 1089: 97-116
<400> 1089
GATATTTTTC CCGCTGGGCT ATCTGGCGCG GCACTCTTTG CGACGCATTC 50
GCGACACCTT ACGTTTGTTC TTTGCTAAAC CTCGTTATGT TAAACCGGCC 100
<210> 1090
<211> 100
<212> DNA
<213> Escherichia coli K-12 MG1655
<220> yhju b3539
<222> 1481-1580
<223> yhju b3539 U00096 3695843..3697522
<313> 1090: 1524-1543
<400> 1090
TCCTGGCTAT CTCCGATCTG GTGGTTCGCG TTCTCGATGG CAAGATTTTC 50
ACCGAAGACA ATGTTGACTG GAAAAAACTC ACCAGTGGGT TGCCACAAAC 100
<210> 1091
<211> 100
<212> DNA
<213> Escherichia coli K-12 MG1655
<220> yhju b3539

<222> 1415-1514
<223> yhju b3539 U00096 3695843..3697522
<313> 1091: 1474-1493
<400> 1091
TGAAATTCTT CGGCATGAAG GCACCGCATC AGGGGGCACC GATTGTCATC 50
GAACAACCGA GCAGCTTCCT GGCTATCTCC GATCTGGTGG TTCGCGTTCT 100
<210> 1092
<211> 100
<212> DNA
<213> Escherichia coli K-12 MG1655
<220> yhju b3539
<222> 1399-1498
<223> yhju b3539 U00096 3695843..3697522
<313> 1092: 1436-1455
<400> 1092
GACGTCCCCG TTGGGGTGAA ATTCTTCGGC ATGAAGGCAC CGCATCAGGG 50
GGCACCGATT GTCATCGAAC AACCGAGCAG CTTCCTGGCT ATCTCCGATC 100
<210> 1093
<211> 100
<212> DNA
<213> Escherichia coli K-12 MG1655
<220> yhjv b3540
<222> 1173-1272
<223> yhjv b3540 U00096 3698192..3699463
<313> 1093: 1238-1257
<400> 1093
AGTCGCACAA CTGGAAAAAC TTCGCGGATT TAAAGCCTGG CTGATTCTGC 50
TGTACGGCAT TTTGCTATGC TTGTCGCCAC TGTTGAAGCT GATTGAGTAA 100
<210> 1094
<211> 100
<212> DNA
<213> Escherichia coli K-12 MG1655
<220> yhjv b3540
<222> 1111-1210
<223> yhjv b3540 U00096 3698192..3699463
<313> 1094: 1146-1165
<400> 1094
CAGTTGGGAA GCCCGTTATA TGGTATTGTG TCGTGCCTCA TTCCGTTTTT 50
CCTGATCTAT AAAGTCGCAC AACTGGAAAA ACTTCGCGGA TTTAAAGCCT 100
<210> 1095
<211> 100
<212> DNA
<213> Escherichia coli K-12 MG1655
<220> yhjv b3540
<222> 911-1010
<223> yhjv b3540 U00096 3698192..3699463
<313> 1095: 939-958
<400> 1095
TCTTTGCCGT ACTGACCGCA TTCTTTGGCA TTTATCTCGG TTTCCACGAG 50
GCCATTAAAG GCATTATTCT CAATCTGTTA AGCCGAATTA TTGATACCAA 100
<210> 1096
<211> 100
<212> DNA
<213> Escherichia coli K-12 MG1655
<220> yhjw b3547
<222> 1626-1725
<223> yhjw b3547 U00096 complement(3706413..3708137)
<313> 1096: 1699-1718
<400> 1096
CATCATGGGT TGTCTTGGCT ATACTTCACC GGATGGTGGA ATTAACGAAA 50
ACAACAACTG GTGTCACATC CCGCAGGCAA AAGAGGCAGC GGCTAACTAA 100
<210> 1097
<211> 100
<212> DNA
<213> Escherichia coli K-12 MG1655
<220> yhjw b3547
<222> 1594-1693
<223> yhjw b3547 U00096 complement(3706413..3708137)
<313> 1097: 1628-1647
<400> 1097
GTGCCACGCC GTCACGTAGA GCTGTACGAT ACCATCATGG GTTGTCTTGG 50

CTATACTTCA CCGGATGGTG GAATTAACGA AAACAACAAC TGGTGTCACA 100
<210> 1098
<211> 100
<212> DNA
<213> Escherichia coli K-12 MG1655
<220> yhjw b3547
<222> 1557-1656
<223> yhjw b3547 U00096 complement(3706413..3708137)
<313> 1098: 1597-1616
<400> 1098
GGCGTTTGCG CAGCTGAAAA AAGAAGCCGA CATGAAAGTG CCACGCCGTC 50
ACGTAGAGCT GTACGATACC ATCATGGGTT GTCTTGGCTA TACTTCACCG 100
<210> 1099
<211> 100
<212> DNA
<213> Escherichia coli K-12 MG1655
<220> yhjx b3548
<222> 1110-1209
<223> yhjx b3548 U00096 complement(3708428..3709636)
<313> 1099: 1182-1201
<400> 1099
CTACGTGATT TTCGCCCTGC TGATTCTGTC ATTGGCGCTT TCTACGACGA 50
TTCGTCAGCC AGAGCAGAAA ATGTTGCGTG AGGCGCATGG CTCCCTTTAA 100
<210> 1100
<211> 100
<212> DNA
<213> Escherichia coli K-12 MG1655
<220> yhjx b3548
<222> 1090-1189
<223> yhjx b3548 U00096 complement(3708428..3709636)
<313> 1100: 1133-1152
<400> 1100
GGCGGCTTCT ATGTGACCTT CTACGTGATT TTCGCCCTGC TGATTCTGTC 50
ATTGGCGCTT TCTACGACGA TTCGTCAGCC AGAGCAGAAA ATGTTGCGTG 100
<210> 1101
<211> 100
<212> DNA
<213> Escherichia coli K-12 MG1655
<220> yhjx b3548
<222> 1052-1151
<223> yhjx b3548 U00096 complement(3708428..3709636)
<313> 1101: 1108-1127
<400> 1101
TCGGTAGCAT TTGTGGTTCG ATTATCGCCT CACTGTTTGG CGGCTTCTAT 50
GTGACCTTCT ACGTGATTTT CGCCCTGCTG ATTCTGTCAT TGGCGCTTTC 100
<210> 1102
<211> 100
<212> DNA
<213> Escherichia coli K-12 MG1655
<220> yhjy b3549
<222> 577-676
<223> yhjy b3549 U00096 complement(3709865..3710569)
<313> 1102: 626-645
<400> 1102
GATGTCACCG TAGGCGCTGA TATGTTGCTC AATCAAAATA TTGCCGCCTA 50
TGCCGCGCTA ACTCAGGCAG AAAATACCAC TAATAATAGC GACTATCTGT 100
<210> 1103
<211> 100
<212> DNA
<213> Escherichia coli K-12 MG1655
<220> yhjy b3549
<222> 556-655
<223> yhjy b3549 U00096 complement(3709865..3710569)
<313> 1103: 595-614
<400> 1103
AACCAGAACG GCAACTGGCT GGATGTCACC GTAGGCGCTG ATATGTTGCT 50
CAATCAAAAT ATTGCCGCCT ATGCCGCGCT AACTCAGGCA GAAAATACCA 100
<210> 1104
<211> 100
<212> DNA
<213> Escherichia coli K-12 MG1655

<220> yhjy b3549
<222> 506-605
<223> yhjy b3549 U00096 complement(3709865..3710569)
<313> 1104: 542-561
<400> 1104
GCGAGAATAT CTGGAAGGCG CAATCAGGCC TGAGCCGGAT GACGGCGACA  50
AACCAGAACG GCAACTGGCT GGATGTCACC GTAGGCGCTG ATATGTTGCT  100
<210> 1105
<211> 100
<212> DNA
<213> Escherichia coli K-12 MG1655
<220> tola b0740
<222> 1167-1266
<223> tola b0740 U00096 775565..776830
<313> 1105: 1235-1254
<400> 1105
GGCTGCGTTG GCAGCAGCTA AACTTGCGAA GATCCCGAAA CCACCAAGCC  50
AGGCAGTATA TGAAGTGTTC AAAAACGCGC CATTGGACTT CAAACCGTAA  100
<210> 1106
<211> 100
<212> DNA
<213> Escherichia coli K-12 MG1655
<220> tola b0740
<222> 1160-1259
<223> tola b0740 U00096 775565..776830
<313> 1106: 1188-1207
<400> 1106
TTTGTCAGGC TGCGTTGGCA GCAGCTAAAC TTGCGAAGAT CCCGAAACCA  50
CCAAGCCAGG CAGTATATGA AGTGTTCAAA AACGCGCCAT TGGACTTCAA  100
<210> 1107
<211> 100
<212> DNA
<213> Escherichia coli K-12 MG1655
<220> tola b0740
<222> 1132-1231
<223> tola b0740 U00096 775565..776830
<313> 1107: 1163-1182
<400> 1107
ATCAAACCTG AAGGTGGCGA TCCCGCACTT TGTCAGGCTG CGTTGGCAGC  50
AGCTAAACTT GCGAAGATCC CGAAACCACC AAGCCAGGCA GTATATGAAG  100
<210> 1108
<211> 100
<212> DNA
<213> Escherichia coli K-12 MG1655
<220> tolb b0741
<222> 1194-1293
<223> tolb b0741 U00096 776963..778255
<313> 1108: 1265-1284
<400> 1108
ATCCGTGCTG AATTTGGTTT CTACAGATGG GCGTTTCAAA GCGCGTCTTC  50
CGGCAACTGA TGGACAGGTC AAATTCCCTG CCTGGTCGCC GTATCTGTGA  100
<210> 1109
<211> 100
<212> DNA
<213> Escherichia coli K-12 MG1655
<220> tolb b0741
<222> 1194-1293
<223> tolb b0741 U00096 776963..778255
<313> 1109: 1223-1242
<400> 1109
ATCCGTGCTG AATTTGGTTT CTACAGATGG GCGTTTCAAA GCGCGTCTTC  50
CGGCAACTGA TGGACAGGTC AAATTCCCTG CCTGGTCGCC GTATCTGTGA  100
<210> 1110
<211> 100
<212> DNA
<213> Escherichia coli K-12 MG1655
<220> tolb b0741
<222> 1081-1180
<223> tolb b0741 U00096 776963..778255
<313> 1110: 1133-1152
<400> 1110

CAAGATCTGG CAACGGGAGG CGTACAAGTT CTGTCGTCCA CGTTCCTGGA 50
TGAAACGCCA AGTCTGGCAC CTAACGGCAC TATGGTAATC TACAGCTCTT 100
<210> 1111
<211> 100
<212> DNA
<213> Escherichia coli K-12 MG1655
<220> tolc b3036
<222> 1389-1488
<223> tolc b3036 U00096 3176125..3177612
<313> 1111: 1458-1477
<400> 1111
TATTGCTGAT GGTTATGCGC CTGATAGCCC GGCACCAGTC GTTCAGCAAA 50
CATCCGCACG CACTACCACC AGTAACGGTC ATAACCCTTT CCGTAACTGA 100
<210> 1112
<211> 100
<212> DNA
<213> Escherichia coli K-12 MG1655
<220> tolc b3036
<222> 1386-1485
<223> tolc b3036 U00096 3176125..3177612
<313> 1112: 1427-1446
<400> 1112
TGCTATTGCT GATGGTTATG CGCCTGATAG CCCGGCACCA GTCGTTCAGC 50
AAACATCCGC ACGCACTACC ACCAGTAACG GTCATAACCC TTTCCGTAAC 100
<210> 1113
<211> 100
<212> DNA
<213> Escherichia coli K-12 MG1655
<220> tolc b3036
<222> 1353-1452
<223> tolc b3036 U00096 3176125..3177612
<313> 1113: 1374-1393
<400> 1113
GGAAAACGTT GCACCGCAAA CGCCGGAACA GAATGCTATT GCTGATGGTT 50
ATGCGCCTGA TAGCCCGGCA CCAGTCGTTC AGCAAACATC CGCACGCACT 100
<210> 1114
<211> 100
<212> DNA
<213> Escherichia coli K-12 MG1655
<220> pspa b1305
<222> 570-669
<223> pspa b1305 U00096 1366103..1366771
<313> 1114: 623-642
<400> 1114
AAAATCGCTG GACGATCAGT TTGCCGAACT GAAAGCCGAT GATGCAATCA 50
GCGAACAACT GGCACAATTA AAAGCCAAAA TGAAGCAAGA CAATCAATAA 100
<210> 1115
<211> 100
<212> DNA
<213> Escherichia coli K-12 MG1655
<220> pspa b1305
<222> 509-608
<223> pspa b1305 U00096 1366103..1366771
<313> 1115: 567-586
<400> 1115
CTTTCGAACG TCGTATTGAC CAGATGGAAG CGGAAGCAGA AAGCCACAGC 50
TTCGGTAAAC AAAAATCGCT GGACGATCAG TTTGCCGAAC TGAAAGCCGA 100
<210> 1116
<211> 100
<212> DNA
<213> Escherichia coli K-12 MG1655
<220> pspa b1305
<222> 499-598
<223> pspa b1305 U00096 1366103..1366771
<313> 1116: 524-543
<400> 1116
CGTTTCGAAT CTTTCGAACG TCGTATTGAC CAGATGGAAG CGGAAGCAGA 50
AAGCCACAGC TTCGGTAAAC AAAAATCGCT GGACGATCAG TTTGCCGAAC 100
<210> 1117
<211> 100
<212> DNA

<213> Escherichia coli K-12 MG1655
<220> pspb b1306
<222> 126-225
<223> pspb b1306 U00096 1366825..1367049
<313> 1117: 199-218
<400> 1117
ATTAGCGCAA CTGGCTGATG AAGCAAAACG GATGCGCGAA CGTATTCAGG 50
CGCTGGAATC TATTCTTGAT GCAGAACATC CGAACTGGAG GGATCGCTAA 100
<210> 1118
<211> 100
<212> DNA
<213> Escherichia coli K-12 MG1655
<220> pspb b1306
<222> 82-181
<223> pspb b1306 U00096 1366825..1367049
<313> 1118: 132-151
<400> 1118
AATCGTTCTG GTCGCAGTGA ATTGTCGCAA AGTGAGCAGC AGCGATTAGC 50
GCAACTGGCT GATGAAGCAA AACGGATGCG CGAACGTATT CAGGCGCTGG 100
<210> 1119
<211> 100
<212> DNA
<213> Escherichia coli K-12 MG1655
<220> pspb b1306
<222> 54-153
<223> pspb b1306 U00096 1366825..1367049
<313> 1119: 92-111
<400> 1119
ACCGATCTGG TTATGGCTGC ATTACAGCAA TCGTTCTGGT CGCAGTGAAT 50
TGTCGCAAAG TGAGCAGCAG CGATTAGCGC AACTGGCTGA TGAAGCAAAA 100
<210> 1120
<211> 100
<212> DNA
<213> Escherichia coli K-12 MG1655
<220> pspc b1307
<222> 261-360
<223> pspc b1307 U00096 1367049..1367408
<313> 1120: 306-325
<400> 1120
CGACCGTGAA CTGGCGGCAA GTGAAACGCG TTTACGCGAG ATGGAACGTT 50
ATGTCACTTC CGATACTTTC ACGTTACGTA GCCGTTTCCG TCAACTGTGA 100
<210> 1121
<211> 100
<212> DNA
<213> Escherichia coli K-12 MG1655
<220> pspc b1307
<222> 165-264
<223> pspc b1307 U00096 1367049..1367408
<313> 1121: 221-240
<400> 1121
TGCTTACATC ATTTTGTCAT TTGCGCTTGA TCCAATGCCG GACAACATGG 50
CCTTTGGTGA GCAGCTACCT TCCAGCAGCG AATTGCTGGA TGAAGTCGAC 100
<210> 1122
<211> 100
<212> DNA
<213> Escherichia coli K-12 MG1655
<220> pspc b1307
<222> 138-237
<223> pspc b1307 U00096 1367049..1367408
<313> 1122: 180-199
<400> 1122
CTTCGGTCTG GCGCTGTTTA CCCTGGTTGC TTACATCATT TTGTCATTTG 50
CGCTTGATCC AATGCCGGAC AACATGGCCT TTGGTGAGCA GCTACCTTCC 100
<210> 1123
<211> 100
<212> DNA
<213> Escherichia coli K-12 MG1655
<220> pspd b1308
<222> 123-222
<223> pspd b1308 U00096 1367417..1367638
<313> 1123: 191-210

<400> 1123
ATCAGTTGCT CGCCGACCGC TGAAAATGTT GCTGGCTGTG GCGCTGGAAC 50
CGCTGTTAAG TCGGGCTGCT AATAAACTGG CACAGCGTTA TAAAAGGTGA 100
<210> 1124
<211> 100
<212> DNA
<213> Escherichia coli K-12 MG1655
<220> pspd b1308
<222> 113-212
<223> pspd b1308 U00096 1367417..1367638
<313> 1124: 127-146
<400> 1124
GGGCGATAAA ATCAGTTGCT CGCCGACCGC TGAAAATGTT GCTGGCTGTG 50
GCGCTGGAAC CGCTGTTAAG TCGGGCTGCT AATAAACTGG CACAGCGTTA 100
<210> 1125
<211> 100
<212> DNA
<213> Escherichia coli K-12 MG1655
<220> pspd b1308
<222> 32-131
<223> pspd b1308 U00096 1367417..1367638
<313> 1125: 64-83
<400> 1125
AGGTAAAGCC TGGTTTCAAA TTAGCAGGCA AGCTGGTACT TCTTACCGCA 50
CTGCGCTATG GCCCGGCGGG TGTGGCGGGC TGGGCGATAA AATCAGTTGC 100
<210> 1126
<211> 100
<212> DNA
<213> Escherichia coli K-12 MG1655
<220> pspe b1309
<222> 216-315
<223> pspe b1309 U00096 1367713..1368027
<313> 1126: 281-300
<400> 1126
GTCAGGGCAA GCAAAAGAGA TCCTTAGCGA GATGGGATAT ACCCACGTTG 50
AGAACGCCGG TGGCCTGAAA GACATCGCAA TGCCGAAGGT CAAAGGTTAA 100
<210> 1127
<211> 100
<212> DNA
<213> Escherichia coli K-12 MG1655
<220> pspe b1309
<222> 207-306
<223> pspe b1309 U00096 1367713..1368027
<313> 1127: 227-246
<400> 1127
CGGACGCCAG TCAGGGCAAG CAAAAGAGAT CCTTAGCGAG ATGGGATATA 50
CCCACGTTGA GAACGCCGGT GGCCTGAAAG ACATCGCAAT GCCGAAGGTC 100
<210> 1128
<211> 100
<212> DNA
<213> Escherichia coli K-12 MG1655
<220> pspe b1309
<222> 108-207
<223> pspe b1309 U00096 1367713..1368027
<313> 1128: 141-160
<400> 1128
CGTTCAGGGG GCCATCAATA TTCCCCTGAA GGAAGTGAAA GAGCGCATTG 50
CCACCGCCGT TCCGGATAAA AACGACACCG TGAAAGTGTA TTGCAATGCC 100
<210> 1129
<211> 100
<212> DNA
<213> Escherichia coli K-12 MG1655
<220> pspf b1304
<222> 780-879
<223> pspf b1304 U00096 complement(1364959..1365951)
<313> 1129: 825-844
<400> 1129
TAAACGGCGT CCGCCTGAAG ACGCTATCGC CGTTTCAGAA ACCACCTCGC 50
TTCCAACACT GCCGCTGGAT TTACGTGAGT TTCAGATGCA GCAGGAAAAA 100
<210> 1130
<211> 100

<212> DNA
<213> Escherichia coli K-12 MG1655
<220> pspf b1304
<222> 732-831
<223> pspf b1304 U00096 complement(1364959..1365951)
<313> 1130: 773-792
<400> 1130
CCACGGCACC AGCGATTATC CGCTTGATGA CATCATTATT GATCCCTTTA 50
AACGGCGTCC GCCTGAAGAC GCTATCGCCG TTTCAGAAAC CACCTCGCTT 100
<210> 1131
<211> 100
<212> DNA
<213> Escherichia coli K-12 MG1655
<220> pspf b1304
<222> 673-772
<223> pspf b1304 U00096 complement(1364959..1365951)
<313> 1131: 727-746
<400> 1131
TATCGTTGGC CGGGAAATAT TCGTGAATTG AAAAACGTGG TGGAACGTTC 50
AGTGTATCGC CACGGCACCA GCGATTATCC GCTTGATGAC ATCATTATTG 100
<210> 1132
<211> 100
<212> DNA
<213> Escherichia coli K-12 MG1655
<220> tolq b0738
<222> 594-693
<223> tolq b0738 U00096 774376..775068
<313> 1132: 650-669
<400> 1132
CGTAAACAAA CTGGAACTGA ATTACGACAA CTTTATGGAA GAGTTTACCG 50
CGATTCTGCA CCGCCAGGCG TTTACCGTTA GCGAGAGCAA CAAGGGGTAA 100
<210> 1133
<211> 100
<212> DNA
<213> Escherichia coli K-12 MG1655
<220> tolq b0738
<222> 549-648
<223> tolq b0738 U00096 774376..775068
<313> 1133: 601-620
<400> 1133
TGCCGCTATC CCGGCAGTTA TGGCCTACAA CCGCCTCAAC CAGCGCGTAA 50
ACAAACTGGA ACTGAATTAC GACAACTTTA TGGAAGAGTT TACCGCGATT 100
<210> 1134
<211> 100
<212> DNA
<213> Escherichia coli K-12 MG1655
<220> tolq b0738
<222> 425-524
<223> tolq b0738 U00096 774376..775068
<313> 1134: 479-498
<400> 1134
TGTTTGGTAC GGTCTGGGGG ATCATGCACG CCTTTATCGC CCTCGGGGCG 50
GTAAAACAAG CAACACTGCA AATGGTTGCG CCCGGTATCG CAGAAGCGTT 100
<210> 1135
<211> 100
<212> DNA
<213> Escherichia coli K-12 MG1655
<220> tolr b0739
<222> 225-324
<223> tolr b0739 U00096 775072..775500
<313> 1135: 290-309
<400> 1135
CGTGGTGGTT GAGAAAGATC GCCTGGAGCG TTTACCACCA GAGCAGGTGG 50
TGGCGGAAGT GTCCAGCCGT TTCAAGGCCA ACCCGAAAAC GGTCTTTCTG 100
<210> 1136
<211> 100
<212> DNA
<213> Escherichia coli K-12 MG1655
<220> tolr b0739
<222> 184-283
<223> tolr b0739 U00096 775072..775500

<313> 1136: 232-251
<400> 1136
CCGCCAGTGA TTGTTGAAGT GTCTGGTATT GGTCAGTACA CCGTGGTGGT  50
TGAGAAAGAT CGCCTGGAGC GTTTACCACC AGAGCAGGTG GTGGCGGAAG  100
<210> 1137
<211> 100
<212> DNA
<213> Escherichia coli K-12 MG1655
<220> tolr b0739
<222> 119-218
<223> tolr b0739 U00096 775072..775500
<313> 1137: 158-177
<400> 1137
CCCAGAGCGT GGAGGTCGAT CTGCCAGACG CTACTGAATC ACAGGCGGTG  50
AGCAGTAACG ATAATCCGCC AGTGATTGTT GAAGTGTCTG GTATTGGTCA  100
<210> 1138
<211> 100
<212> DNA
<213> Escherichia coli K-12 MG1655
<220> htga b0013
<222> 492-591
<223> htga b0013 U00096 10725..11315
<313> 1138: 570-589
<400> 1138
TTCGTTGCGC ATCAGTACGC TGGAGAGGCG AGTTTTGCCT TTTTCATTAT  50
GGGTGAGCAA TCGGGCGAAA TTTGCCAACT GTTCCTCACT ACAATGCTGA  100
<210> 1139
<211> 100
<212> DNA
<213> Escherichia coli K-12 MG1655
<220> htga b0013
<222> 428-527
<223> htga b0013 U00096 10725..11315
<313> 1139: 466-485
<400> 1139
CTCCGGCAAT CAGCTGCCAG TTGCGGCGAT GTTGCTCGGG ATGCCCTTCC  50
ATCGATTTAA ACAGTTCGTT GCGCATCAGT ACGCTGGAGA GGCGAGTTTT  100
<210> 1140
<211> 100
<212> DNA
<213> Escherichia coli K-12 MG1655
<220> htga b0013
<222> 371-470
<223> htga b0013 U00096 10725..11315
<313> 1140: 417-436
<400> 1140
ATTTACCGTG TCCGCGCAGT TTGTTGGCGA TACTATCGCC ACCAAAATGC  50
TGTAATTCTC CGGCAATCAG CTGCCAGTTG CGGCGATGTT GCTCGGGATG  100
<210> 1141
<211> 100
<212> DNA
<213> Escherichia coli K-12 MG1655
<220> visc b2907
<222> 1104-1203
<223> visc b2907 U00096 complement(3049135..3050337)
<313> 1141: 1174-1193
<400> 1141
TGATATTGGT TTGAAACTGG CCGACACGCT TCCTGGCGTT AAGCCGCAAC  50
TTATCCGCCA GGCAATGGGA TTAAACGATT TGCCTGAATG GCTGCGTTAA  100
<210> 1142
<211> 100
<212> DNA
<213> Escherichia coli K-12 MG1655
<220> visc b2907
<222> 1104-1203
<223> visc b2907 U00096 complement(3049135..3050337)
<313> 1142: 1126-1145
<400> 1142
TGATATTGGT TTGAAACTGG CCGACACGCT TCCTGGCGTT AAGCCGCAAC  50
TTATCCGCCA GGCAATGGGA TTAAACGATT TGCCTGAATG GCTGCGTTAA  100
<210> 1143

<211> 100
<212> DNA
<213> Escherichia coli K-12 MG1655
<220> visc b2907
<222> 1066-1165
<223> visc b2907 U00096 complement(3049135..3050337)
<313> 1143: 1084-1103
<400> 1143
CTGTTTTCCG GTACCAATCC GGCGAAAAAA CTGCTGCGTG ATATTGGTTT 50
GAAACTGGCC GACACGCTTC CTGGCGTTAA GCCGCAACTT ATCCGCCAGG 100
<210> 1144
<211> 100
<212> DNA
<213> Escherichia coli K-12 MG1655
<220> araa b0063
<222> 1404-1503
<223> araa b0063 U00096 complement(66835..68337)
<313> 1144: 1467-1486
<400> 1144
GCACGACATT GAAATCACGG TGATTGATAA CGACACACGC CTGCCAGCGT 50
TTAAAGACGC GCTGCGCTGG AACGAAGTGT ATTACGGGTT TCGTCGCTAA 100
<210> 1145
<211> 100
<212> DNA
<213> Escherichia coli K-12 MG1655
<220> araa b0063
<222> 1396-1495
<223> araa b0063 U00096 complement(66835..68337)
<313> 1145: 1426-1445
<400> 1145
GCCGAGATGC ACGACATTGA AATCACGGTG ATTGATAACG ACACACGCCT 50
GCCAGCGTTT AAAGACGCGC TGCGCTGGAA CGAAGTGTAT TACGGGTTTC 100
<210> 1146
<211> 100
<212> DNA
<213> Escherichia coli K-12 MG1655
<220> araa b0063
<222> 1334-1433
<223> araa b0063 U00096 complement(66835..68337)
<313> 1146: 1384-1403
<400> 1146
CTGGTGGCGC GCACCATACC GTCTTCAGCC ATGCACTGAA CCTCAACGAT 50
ATGCGCCAAT TCGCCGAGAT GCACGACATT GAAATCACGG TGATTGATAA 100
<210> 1147
<211> 100
<212> DNA
<213> Escherichia coli K-12 MG1655
<220> arab b0064
<222> 1602-1701
<223> arab b0064 U00096 complement(68348..70048)
<313> 1147: 1667-1686
<400> 1147
GCTTTATCGC CGCTATCAGC AATGGGCGAT GAGCGCCGAA CAACACTATC 50
TTCCAACTTC CGCCCCGGCA CAGGCTGCCC AGGCCGTTGC GACTCTATAA 100
<210> 1148
<211> 100
<212> DNA
<213> Escherichia coli K-12 MG1655
<220> arab b0064
<222> 1563-1662
<223> arab b0064 U00096 complement(68348..70048)
<313> 1148: 1631-1650
<400> 1148
CCTGCAACCG TGCAGCGAGC AGGCACAACG CTTTGAACAG CTTTATCGCC 50
GCTATCAGCA ATGGGCGATG AGCGCCGAAC AACACTATCT TCCAACTTCC 100
<210> 1149
<211> 100
<212> DNA
<213> Escherichia coli K-12 MG1655
<220> arab b0064
<222> 1555-1654

<223> arab b0064 U00096 complement(68348..70048)
<313> 1149: 1582-1601
<400> 1149
GAGAAAACCC TGCAACCGTG CAGCGAGCAG GCACAACGCT TTGAACAGCT 50
TTATCGCCGC TATCAGCAAT GGGCGATGAG CGCCGAACAA CACTATCTTC 100
<210> 1150
<211> 100
<212> DNA
<213> Escherichia coli K-12 MG1655
<220> arac b0065
<222> 775-874
<223> arac b0065 U00096 70387..71265
<313> 1150: 837-856
<400> 1150
CTCTATTTCT CGCGAGTATT TAAAAAATGC ACCGGGGCCA GCCCGAGCGA 50
GTTTCGTGCC GGTTGTGAAG AAAAAGTGAA TGATGTAGCC GTCAAGTTGT 100
<210> 1151
<211> 100
<212> DNA
<213> Escherichia coli K-12 MG1655
<220> arac b0065
<222> 690-789
<223> arac b0065 U00096 70387..71265
<313> 1151: 730-749
<400> 1151
ACGCATTAGT CAGGCGAAGC TGCTTTTGAG CACTACCCGG ATGCCTATCG 50
CCACCGTCGG TCGCAATGTT GGTTTTGACG ATCAACTCTA TTTCTCGCGA 100
<210> 1152
<211> 100
<212> DNA
<213> Escherichia coli K-12 MG1655
<220> arac b0065
<222> 633-732
<223> arac b0065 U00096 70387..71265
<313> 1152: 679-698
<400> 1152
GTCACATCTT TTCCGCCAGC AGTTAGGGAT TAGCGTCTTA AGCTGGCGCG 50
AGGACCAACG CATTAGTCAG GCGAAGCTGC TTTTGAGCAC TACCCGGATG 100
<210> 1153
<211> 100
<212> DNA
<213> Escherichia coli K-12 MG1655
<220> arad b0062
<222> 597-696
<223> arad b0062 U00096 complement(65855..66550)
<313> 1153: 659-678
<400> 1153
CTGCCGTCAG TTAGCGCCGC AGTTACCGGA TATGCAGCAA ACGCTGCTGG 50
ATAAACACTA TCTGCGTAAG CATGGCGCGA AGGCATATTA CGGGCAGTAA 100
<210> 1154
<211> 100
<212> DNA
<213> Escherichia coli K-12 MG1655
<220> arad b0062
<222> 573-672
<223> arad b0062 U00096 complement(65855..66550)
<313> 1154: 635-654
<400> 1154
AGAGGTCGCT TATATGGGGA TATTCTGCCG TCAGTTAGCG CCGCAGTTAC 50
CGGATATGCA GCAAACGCTG CTGGATAAAC ACTATCTGCG TAAGCATGGC 100
<210> 1155
<211> 100
<212> DNA
<213> Escherichia coli K-12 MG1655
<220> arad b0062
<222> 535-634
<223> arad b0062 U00096 complement(65855..66550)
<313> 1155: 552-571
<400> 1155
AATGCCGAAG ATGCGGTGCA TAACGCCATC GTGCTGGAAG AGGTCGCTTA 50
TATGGGGATA TTCTGCCGTC AGTTAGCGCC GCAGTTACCG GATATGCAGC 100

<210> 1156
<211> 100
<212> DNA
<213> Escherichia coli K-12 MG1655
<220> arae b2842
<222> 1320-1419
<223> arae b2842 U00096 complement(2978786..2980204)
<313> 1156: 1389-1408
<400> 1156
CATTACTTTC TGGCTCATTC CGGAAACCAA AAATGTCACG CTGGAACATA 50
TCGAACGCAA ACTGATGGCA GGCGAGAAGT TGAGAAATAT CGGCGTCTGA 100
<210> 1157
<211> 100
<212> DNA
<213> Escherichia coli K-12 MG1655
<220> arae b2842
<222> 1271-1370
<223> arae b2842 U00096 complement(2978786..2980204)
<313> 1157: 1312-1331
<400> 1157
CTGCCGGTAC GTTCTGGCTC TACACTGCGC TGAACATTGC GTTTGTGGGC 50
ATTACTTTCT GGCTCATTCC GGAAACCAAA AATGTCACGC TGGAACATAT 100
<210> 1158
<211> 100
<212> DNA
<213> Escherichia coli K-12 MG1655
<220> arae b2842
<222> 1185-1284
<223> arae b2842 U00096 complement(2978786..2980204)
<313> 1158: 1239-1258
<400> 1158
CGGTATTACC TGTTCGACCA CCACGAACTG GGTGTCGAAT ATGATTATCG 50
GCGCGACCTT CCTGACACTG CTTGATAGCA TTGGCGCTGC CGGTACGTTC 100
<210> 1159
<211> 100
<212> DNA
<213> Escherichia coli K-12 MG1655
<220> araf b1902
<222> 884-983
<223> araf b1902 U00096 complement(1983162..1984151)
<313> 1159: 904-923
<400> 1159
CAAAAGACGT TGAACCGCCA AAATTTACCG AAGTTACCGA CGTGGTACTG 50
ATCACGCGTG ACAACTTTAA AGAAGAACTG GAGAAAAAAG GTTTAGGCGG 100
<210> 1160
<211> 100
<212> DNA
<213> Escherichia coli K-12 MG1655
<220> araf b1902
<222> 824-923
<223> araf b1902 U00096 complement(1983162..1984151)
<313> 1160: 880-899
<400> 1160
TGCTGCCAAG CCCGGACGTA CATGGCTATA AATCCAGCGA AATGCTTTAC 50
AACTGGGTAG CAAAAGACGT TGAACCGCCA AAATTTACCG AAGTTACCGA 100
<210> 1161
<211> 100
<212> DNA
<213> Escherichia coli K-12 MG1655
<220> araf b1902
<222> 793-892
<223> araf b1902 U00096 complement(1983162..1984151)
<313> 1161: 814-833
<400> 1161
AAAGCACAGG CAACCGGCTT CTACGGTTCC CTGCTGCCAA GCCCGGACGT 50
ACATGGCTAT AAATCCAGCG AAATGCTTTA CAACTGGGTA GCAAAAGACG 100
<210> 1162
<211> 100
<212> DNA
<213> Escherichia coli K-12 MG1655
<220> arag b1901

<222> 1416-1515
<223> arag b1901 U00096 complement(1981578..1983092)
<313> 1162: 1487-1506
<400> 1162
GCGGGAAGGT GAAATCGCCG GTGAATTGTT ACACGAGCAG GCAGATGAGC 50
GTCAGGCACT GAGCCTTGCG ATGCCTAAAG TCAGCCAGGC TGTTGCCTGA 100
<210> 1163
<211> 100
<212> DNA
<213> Escherichia coli K-12 MG1655
<220> arag b1901
<222> 1376-1475
<223> arag b1901 U00096 complement(1981578..1983092)
<313> 1163: 1409-1428
<400> 1163
TACCTGAAGT CCTCGGCGTT GCCGACCGGA TTGTGGTGAT GCGGGAAGGT 50
GAAATCGCCG GTGAATTGTT ACACGAGCAG GCAGATGAGC GTCAGGCACT 100
<210> 1164
<211> 100
<212> DNA
<213> Escherichia coli K-12 MG1655
<220> arag b1901
<222> 1218-1317
<223> arag b1901 U00096 complement(1981578..1983092)
<313> 1164: 1250-1269
<400> 1164
AAAAGCCATT CTGGGCCGCT GGTTATCGGA AGAGATGAAG GTCATTTTGC 50
TGGATGAACC TACGCGCGGC ATTGATGTTG GCGCTAAGCA CGAAATATAT 100
<210> 1165
<211> 100
<212> DNA
<213> Escherichia coli K-12 MG1655
<220> araj b0397
<222> 1012-1111
<223> araj b0397 U00096 complement(410521..411705)
<313> 1165: 1043-1062
<400> 1165
GCCGTCGGCG CATATTGCGG AGGTATGATG CTGACGCTGG GGCTGGCATA 50
TAATTACGTG GCGCTGCCTG CCGCCCTGCT TTCGTTTGCT GCGATGTCGT 100
<210> 1166
<211> 100
<212> DNA
<213> Escherichia coli K-12 MG1655
<220> araj b0397
<222> 959-1058
<223> araj b0397 U00096 complement(410521..411705)
<313> 1166: 984-1003
<400> 1166
GCGGAGAGTT ATTAGGTGCC GCAGGTGGGC AAATAGCGTT TAACCTCGGT 50
AGCGCCGTCG GCGCATATTG CGGAGGTATG ATGCTGACGC TGGGGCTGGC 100
<210> 1167
<211> 100
<212> DNA
<213> Escherichia coli K-12 MG1655
<220> araj b0397
<222> 904-1003
<223> araj b0397 U00096 complement(410521..411705)
<313> 1167: 958-977
<400> 1167
GGATTATTTG CCCTTTCAGC ACCGCTACAA ATATTGTTAC TACAAAACGC 50
CAAAGGCGGA GAGTTATTAG GTGCCGCAGG TGGGCAAATA GCGTTTAACC 100
<210> 1168
<211> 100
<212> DNA
<213> Escherichia coli K-12 MG1655
<220> lpda b0117
<222> 1326-1425
<223> lpda b0117 U00096 127912..129336
<313> 1168: 1387-1406
<400> 1168
CCACGCGCAC CCGACTCTGC ACGAGTCTGT GGGCCTGGCG GCAGAAGTGT 50

TCGAAGGTAG CATTACCGAC CTGCCGAACC CGAAAGCGAA GAAGAAGTAA 100
<210> 1169
<211> 100
<212> DNA
<213> Escherichia coli K-12 MG1655
<220> lpda b0117
<222> 1302-1401
<223> lpda b0117 U00096 127912..129336
<313> 1169: 1325-1344
<400> 1169
TGCTGAAGAC ATCGCACTGA CCATCCACGC GCACCCGACT CTGCACGAGT 50
CTGTGGGCCT GGCGGCAGAA GTGTTCGAAG GTAGCATTAC CGACCTGCCG 100
<210> 1170
<211> 100
<212> DNA
<213> Escherichia coli K-12 MG1655
<220> lpda b0117
<222> 1226-1325
<223> lpda b0117 U00096 127912..129336
<313> 1170: 1286-1305
<400> 1170
TCGGTGGTGC GATTGTCGGT ACTAACGGCG GCGAGCTGCT GGGTGAAATC 50
GGCCTGGCAA TCGAAATGGG TTGTGATGCT GAAGACATCG CACTGACCAT 100
<210> 1171
<211> 100
<212> DNA
<213> Escherichia coli K-12 MG1655
<220> rffg b3789
<222> 969-1068
<223> rffg b3789 U00096 3970134..3971201
<313> 1171: 1019-1038
<400> 1171
AATGCGTAAA ACGGTTCAGT GGTATCTGGC TAATGAAAGC TGGTGGAAGC 50
AGGTGCAGGA CGGCAGCTAT CAGGGCGAGC GTTTAGGTCT GAAAGGCTAA 100
<210> 1172
<211> 100
<212> DNA
<213> Escherichia coli K-12 MG1655
<220> rffg b3789
<222> 953-1052
<223> rffg b3789 U00096 3970134..3971201
<313> 1172: 996-1015
<400> 1172
AAACCTTTGA AAGTGGAATG CGTAAAACGG TTCAGTGGTA TCTGGCTAAT 50
GAAAGCTGGT GGAAGCAGGT GCAGGACGGC AGCTATCAGG GCGAGCGTTT 100
<210> 1173
<211> 100
<212> DNA
<213> Escherichia coli K-12 MG1655
<220> rffg b3789
<222> 912-1011
<223> rffg b3789 U00096 3970134..3971201
<313> 1173: 950-969
<400> 1173
TGCTTCGAAA ATTGCCCGTG AACTTGGCTG CGTGCCGCAG GAAACCTTTG 50
AAAGTGGAAT GCGTAAAACG GTTCAGTGGT ATCTGGCTAA TGAAAGCTGG 100
<210> 1174
<211> 100
<212> DNA
<213> Escherichia coli K-12 MG1655
<220> rffh b3790
<222> 783-882
<223> rffh b3790 U00096 3971220..3972101
<313> 1174: 860-879
<400> 1174
CGATGACGAG GGTGTGAAGC GTGCTGCCAG TTCATTAGCG AAAACTGGCT 50
ACGGCCAATA TCTGCTGGAG TTACTTCGTG CCCGTCCGCG CCAGTATTGA 100
<210> 1175
<211> 100
<212> DNA
<213> Escherichia coli K-12 MG1655

<220> rffh b3790
<222> 714-813
<223> rffh b3790 U00096 3971220..3972101
<313> 1175: 759-778
<400> 1175
GACGGTGGAA AAACGCCAGG GCTTTAAGAT TGCCTGCCTG GAAGAGATTG  50
CCTGGCGTAA CGGCTGGCTC GATGACGAGG GTGTGAAGCG TGCTGCCAGT  100
<210> 1176
<211> 100
<212> DNA
<213> Escherichia coli K-12 MG1655
<220> rffh b3790
<222> 659-758
<223> rffh b3790 U00096 3971220..3972101
<313> 1176: 682-701
<400> 1176
CCTGGCTGGA TACCGGCACT CACGACAGCC TGATTGAAGC CAGCACCTTT  50
GTACAGACGG TGGAAAAACG CCAGGGCTTT AAGATTGCCT GCCTGGAAGA  100
<210> 1177
<211> 100
<212> DNA
<213> Escherichia coli K-12 MG1655
<220> yigw_1 b3841
<222> 409-508
<223> yigw_1 b3841 U00096 4021131..4021751
<313> 1177: 459-478
<400> 1177
GAGCGGTTTA TGACATTGCT GGAGCCGTGG CTGGATAAAC TGCCTGGTGC  50
GGTTCTTCAT TGCTTTACCG GCACACGCGA AGAGATGCAG GCGTGCGTGG  100
<210> 1178
<211> 100
<212> DNA
<213> Escherichia coli K-12 MG1655
<220> yigw_1 b3841
<222> 320-419
<223> yigw_1 b3841 U00096 4021131..4021751
<313> 1178: 343-362
<400> 1178
CGGAAGAGCA GGAACGCGCT TTTGTTGCCC AGCTACGCAT TGCCGCAGAT  50
TTAAACATGC CGGTATTTAT GCACTGTCGC GATGCCCACG AGCGGTTTAT  100
<210> 1179
<211> 100
<212> DNA
<213> Escherichia coli K-12 MG1655
<220> yigw_1 b3841
<222> 263-362
<223> yigw_1 b3841 U00096 4021131..4021751
<313> 1179: 304-323
<400> 1179
CAGAAGTGGT GGCGATTGGT GAATGTGGTC TCGACTTTAA CCGCAACTTT  50
TCGACGCCGG AAGAGCAGGA ACGCGCTTTT GTTGCCCAGC TACGCATTGC  100
<210> 1180
<211> 100
<212> DNA
<213> Escherichia coli K-12 MG1655
<220> yigw_2 b3842
<222> 243-342
<223> yigw_2 b3842 U00096 4021585..4021926
<313> 1180: 316-335
<400> 1180
TATTTTGCAA CGTATTGCGC ACTGGCGTGG AGAAGATGCC GCATGGCTGG  50
CTGCCACCAC GGATGCTAAT GTCAAAACAC TGTTTGGGAT TGCGTTTTAG  100
<210> 1181
<211> 100
<212> DNA
<213> Escherichia coli K-12 MG1655
<220> yigw_2 b3842
<222> 194-293
<223> yigw_2 b3842 U00096 4021585..4021926
<313> 1181: 224-243
<400> 1181

TCACGCCAAA GCCATCATCC CGGCGCAACG AGCCAGCCCA TCTGCCCCAT 50
ATTTTGCAAC GTATTGCGCA CTGGCGTGGA GAAGATGCCG CATGGCTGGC 100
<210> 1182
<211> 100
<212> DNA
<213> Escherichia coli K-12 MG1655
<220> yigw_2 b3842
<222> 102-201
<223> yigw_2 b3842 U00096 4021585..4021926
<313> 1182: 163-182
<400> 1182
ACGCGGACTG GAGCTGCGGG AACTTTTGCC GTTGATTCCG GCGGAAAAAT 50
TACTGATCGA AACTGATGCG CCGTATCTGC TCCCTCGCGA TCTCACGCCA 100
<210> 1183
<211> 100
<212> DNA
<213> Escherichia coli K-12 MG1655
<220> laca b0343
<222> 440-539
<223> laca b0343 U00096 complement(360473..361084)
<313> 1183: 490-509
<400> 1183
ATCCAGGCGT CACCATCGGG GATAATTCTG TTATTGGCGC GGGTAGTATC 50
GTCACAAAAG ACATTCCACC AAACGTCGTG GCGGCTGGCG TTCCTTGTCG 100
<210> 1184
<211> 100
<212> DNA
<213> Escherichia coli K-12 MG1655
<220> laca b0343
<222> 407-506
<223> laca b0343 U00096 complement(360473..361084)
<313> 1184: 433-452
<400> 1184
ATAACGTCTG GATCGGAAGT CATGTGGTTA TTAATCCAGG CGTCACCATC 50
GGGGATAATT CTGTTATTGG CGCGGGTAGT ATCGTCACAA AAGACATTCC 100
<210> 1185
<211> 100
<212> DNA
<213> Escherichia coli K-12 MG1655
<220> laca b0343
<222> 347-446
<223> laca b0343 U00096 complement(360473..361084)
<313> 1185: 373-392
<400> 1185
CTGTACACCA TGAATTGAGA AAAAACGGCG AGATGTACTC TTTTCCGATA 50
ACGATTGGCA ATAACGTCTG GATCGGAAGT CATGTGGTTA TTAATCCAGG 100
<210> 1186
<211> 100
<212> DNA
<213> Escherichia coli K-12 MG1655
<220> tonb b1253
<222> 602-701
<223> tonb b1253 U00096 1309113..1309832
<313> 1186: 651-670
<400> 1186
TGTTTGAGCG TGAGGTGAAA AATGCGATGC GCAGATGGCG TTATGAGCCG 50
GGTAAGCCAG GCAGTGGGAT TGTGGTGAAT ATCCTGTTTA AAATTAACGG 100
<210> 1187
<211> 100
<212> DNA
<213> Escherichia coli K-12 MG1655
<220> tonb b1253
<222> 533-632
<223> tonb b1253 U00096 1309113..1309832
<313> 1187: 575-594
<400> 1187
TTAAATTTGA CGTTACGCCG GATGGTCGCG TGGATAACGT ACAAATCCTC 50
TCAGCCAAGC CTGCGAACAT GTTTGAGCGT GAGGTGAAAA ATGCGATGCG 100
<210> 1188
<211> 100
<212> DNA

<213> Escherichia coli K-12 MG1655
<220> tonb b1253
<222> 436-535
<223> tonb b1253 U00096 1309113..1309832
<313> 1188: 479-498
<400> 1188
GTTACCAGTG TGGCTTCAGG ACCACGCGCA TTAAGCCGTA ATCAGCCGCA 50
GTATCCGGCA CGAGCACAGG CATTGCGCAT TGAAGGGCAG GTTAAAGTTA 100
<210> 1189
<211> 100
<212> DNA
<213> Escherichia coli K-12 MG1655
<220> laci b0346
<222> 984-1083
<223> laci b0346 U00096 complement(365652..366734)
<313> 1189: 1052-1071
<400> 1189
CACCCTGGCG CCCAATACGC AAACCGCCTC TCCCCGCGCG TTGGCCGATT 50
CATTAATGCA GCTGGCACGA CAGGTTTCCC GACTGGAAAG CGGGCAGTGA 100
<210> 1190
<211> 100
<212> DNA
<213> Escherichia coli K-12 MG1655
<220> laci b0346
<222> 871-970
<223> laci b0346 U00096 complement(365652..366734)
<313> 1190: 926-945
<400> 1190
CAGGATTTTC GCCTGCTGGG GCAAACCAGC GTGGACCGCT TGCTGCAACT 50
CTCTCAGGGC CAGGCGGTGA AGGGCAATCA GCTGTTGCCC GTCTCACTGG 100
<210> 1191
<211> 100
<212> DNA
<213> Escherichia coli K-12 MG1655
<220> laci b0346
<222> 830-929
<223> laci b0346 U00096 complement(365652..366734)
<313> 1191: 873-892
<400> 1191
AAGACAGCTC ATGTTATATC CCGCCGTTAA CCACCATCAA ACAGGATTTT 50
CGCCTGCTGG GGCAAACCAG CGTGGACCGC TTGCTGCAAC TCTCTCAGGG 100
<210> 1192
<211> 100
<212> DNA
<213> Escherichia coli K-12 MG1655
<220> lacy b0344
<222> 1155-1254
<223> lacy b0344 U00096 complement(361150..362403)
<313> 1192: 1220-1239
<400> 1192
GGGTCTGGTG GCGCTGGGCT TCACCTTAAT TTCCGTGTTC ACGCTTAGCG 50
GCCCCGGCCC GCTTTCCCTG CTGCGTCGTC AGGTGAATGA AGTCGCTTAA 100
<210> 1193
<211> 100
<212> DNA
<213> Escherichia coli K-12 MG1655
<220> lacy b0344
<222> 1129-1228
<223> lacy b0344 U00096 complement(361150..362403)
<313> 1193: 1181-1200
<400> 1193
GGTTTCCAGG GCGCTTATCT GGTGCTGGGT CTGGTGGCGC TGGGCTTCAC 50
CTTAATTTCC GTGTTCACGC TTAGCGGCCC CGGCCCGCTT TCCCTGCTGC 100
<210> 1194
<211> 100
<212> DNA
<213> Escherichia coli K-12 MG1655
<220> lacy b0344
<222> 1065-1164
<223> lacy b0344 U00096 complement(361150..362403)
<313> 1194: 1108-1127

<400> 1194
CTTCTTTAAG CAACTGGCGA TGATTTTTAT GTCTGTACTG GCGGGCAATA 50
TGTATGAAAG CATCGGTTTC CAGGGCGCTT ATCTGGTGCT GGGTCTGGTG 100
<210> 1195
<211> 100
<212> DNA
<213> Escherichia coli K-12 MG1655
<220> lacz b0345
<222> 2948-3047
<223> lacz b0345 U00096 complement(362455..365529)
<313> 1195: 3002-3021
<400> 1195
CATGGCTGAA TATCGACGGT TTCCATATGG GGATTGGTGG CGACGACTCC 50
TGGAGCCCGT CAGTATCGGC GGAATTCCAG CTGAGCGCCG GTCGCTACCA 100
<210> 1196
<211> 100
<212> DNA
<213> Escherichia coli K-12 MG1655
<220> lacz b0345
<222> 2913-3012
<223> lacz b0345 U00096 complement(362455..365529)
<313> 1196: 2948-2967
<400> 1196
CAGCCATCGC CATCTGCTGC ACGCGGAAGA AGGCACATGG CTGAATATCG 50
ACGGTTTCCA TATGGGGATT GGTGGCGACG ACTCCTGGAG CCCGTCAGTA 100
<210> 1197
<211> 100
<212> DNA
<213> Escherichia coli K-12 MG1655
<220> lacz b0345
<222> 2849-2948
<223> lacz b0345 U00096 complement(362455..365529)
<313> 1197: 2899-2918
<400> 1197
ACCAGTGGCG CGGCGACTTC CAGTTCAACA TCAGCCGCTA CAGTCAACAG 50
CAACTGATGG AAACCAGCCA TCGCCATCTG CTGCACGCGG AAGAAGGCAC 100
<210> 1198
<211> 100
<212> DNA
<213> Escherichia coli K-12 MG1655
<220> srla b2703
<222> 465-564
<223> srla b2703 U00096 2823855..2824418
<313> 1198: 530-549
<400> 1198
CTTCCGCGGC TGGGTGACCG ATCTGACCAC CGCCATTTTT GAGAAAAAGA 50
TGGGCATTCA ACTTGAACAA AAAGTTCACC TGGCAGGAGC AACATCATGA 100
<210> 1199
<211> 100
<212> DNA
<213> Escherichia coli K-12 MG1655
<220> srla b2703
<222> 417-516
<223> srla b2703 U00096 2823855..2824418
<313> 1199: 446-465
<400> 1199
TGGCCCACTG GCGGTGAGTT ATCTGCTGGT TGGTCTGGTC ACCAATTTCT 50
TCCGCGGCTG GGTGACCGAT CTGACCACCG CCATTTTTGA GAAAAAGATG 100
<210> 1200
<211> 100
<212> DNA
<213> Escherichia coli K-12 MG1655
<220> srla b2703
<222> 374-473
<223> srla b2703 U00096 2823855..2824418
<313> 1200: 408-427
<400> 1200
ATCTTGGCAT TGCCAGCGGT CTGACAACGC TGAACCTGCC ACTTGGCCCA 50
CTGGCGGTGA GTTATCTGCT GGTTGGTCTG GTCACCAATT TCTTCCGCGG 100
<210> 1201
<211> 100

<212> DNA
<213> Escherichia coli K-12 MG1655
<220> hlye b1183
<222> 690-789
<223> hlye b1183 U00096 complement(1228706..1229623)
<313> 1201: 737-756
<400> 1201
GGTTAAACAA GCGAATAAAG ATATCGATGC CGCCAAATTG AAATTAACCA 50
CCGAAATAGC CGCCATCGGT GAGATAAAAA CGGAAACTGA AACAACCAGA 100
<210> 1202
<211> 100
<212> DNA
<213> Escherichia coli K-12 MG1655
<220> hlye b1183
<222> 564-663
<223> hlye b1183 U00096 complement(1228706..1229623)
<313> 1202: 622-641
<400> 1202
CGCCGGTCCA TTTGGATTAA TCATTTCCTA TTCTATTGCT GCGGGCGTAG 50
TTGAAGGAAA ACTGATTCCA GAATTGAAGA ACAAGTTAAA ATCTGTGCAG 100
<210> 1203
<211> 100
<212> DNA
<213> Escherichia coli K-12 MG1655
<220> hlye b1183
<222> 389-488
<223> hlye b1183 U00096 complement(1228706..1229623)
<313> 1203: 418-437
<400> 1203
ATGAAGCGCA AAAATCCCTG CTGGTAAGCT CACAAAGTTT CAACAACGCT 50
TCCGGGAAAC TGCTGGCGTT AGATAGCCAG TTAACCAATG ATTTTTCAGA 100
<210> 1204
<211> 100
<212> DNA
<213> Escherichia coli K-12 MG1655
<220> srlb b2705
<222> 209-308
<223> srlb b2705 U00096 2825385..2825756
<313> 1204: 254-273
<400> 1204
TTGGCAGCGT GGCGGAAGAC AACCTTCGCG AACTGGGTCA TGTCACCCTG 50
CGCTTCGATG GTTTAAACGA AGCGGAATTT CCGGGCACTG TCCATGTGGC 100
<210> 1205
<211> 100
<212> DNA
<213> Escherichia coli K-12 MG1655
<220> srlb b2705
<222> 145-244
<223> srlb b2705 U00096 2825385..2825756
<313> 1205: 188-207
<400> 1205
AAAGGTGCAC TCCATCCCGG TTTGCAATTT TCACTCGGGC AGCATCGCTA 50
TCCGGTGACC GCTGTTGGCA GCGTGGCGGA AGACAACCTT CGCGAACTGG 100
<210> 1206
<211> 100
<212> DNA
<213> Escherichia coli K-12 MG1655
<220> srlb b2705
<222> 64-163
<223> srlb b2705 U00096 2825385..2825756
<313> 1206: 120-139
<400> 1206
GACCAGATGC TCATCACCTT TCGTGAAGGC GCGCCTGCGG ACCTCGAAGA 50
GTATTGCTTC ATTCATTGCC ACGGCGAGTT GAAAGGTGCA CTCCATCCCG 100
<210> 1207
<211> 100
<212> DNA
<213> Escherichia coli K-12 MG1655
<220> srld b2706
<222> 681-780
<223> srld b2706 U00096 2825760..2826539

<313> 1207: 737-756
<400> 1207
TCAAGATGTG CTGAATATGC TGCTGTTCTA CGCCAGTCCT AAGGCGTCGT 50
ACTGCACCGG ACAGTCGATC AATGTCACCG GCGGTCAGGT GATGTTCTGA 100
<210> 1208
<211> 100
<212> DNA
<213> Escherichia coli K-12 MG1655
<220> srld b2706
<222> 650-749
<223> srld b2706 U00096 2825760..2826539
<313> 1208: 703-722
<400> 1208
ACAAAGTACC GCTCAAACGC GGCTGCGATT ATCAAGATGT GCTGAATATG 50
CTGCTGTTCT ACGCCAGTCC TAAGGCGTCG TACTGCACCG GACAGTCGAT 100
<210> 1209
<211> 100
<212> DNA
<213> Escherichia coli K-12 MG1655
<220> srld b2706
<222> 594-693
<223> srld b2706 U00096 2825760..2826539
<313> 1209: 654-673
<400> 1209
ACAATACGCG ACCAAGCTGG GTATCAAACC GGATCAAGTC GAGCAGTATT 50
ACATCGACAA AGTACCGCTC AAACGCGGCT GCGATTATCA AGATGTGCTG 100
<210> 1210
<211> 100
<212> DNA
<213> Escherichia coli K-12 MG1655
<220> srle b2704
<222> 861-960
<223> srle b2704 U00096 2824415..2825374
<313> 1210: 925-944
<400> 1210
GGTTCGCGTC GGTGTCCCTT CTGTACTGGT GAGCCGCTTT TTAACCGGCG 50
CGCCGACTGT ACTGATCGCC TGGTTTGTCT CCGGTTTTAT CTATCAATAG 100
<210> 1211
<211> 100
<212> DNA
<213> Escherichia coli K-12 MG1655
<220> srle b2704
<222> 725-824
<223> srle b2704 U00096 2824415..2825374
<313> 1211: 781-800
<400> 1211
TATTGATTGG CGTGCAGATT GGTCTCGGCA ATATTCCGCC GCATCTGGCT 50
TTACCGGCAC TGTTTGCCAT CAACGCGCAG GCGGCCTGCG ACTTCATCCC 100
<210> 1212
<211> 100
<212> DNA
<213> Escherichia coli K-12 MG1655
<220> srle b2704
<222> 664-763
<223> srle b2704 U00096 2824415..2825374
<313> 1212: 708-727
<400> 1212
TCCTTCCCAC TGCTTTCACC TTTCTTCGGC CCAGGCGCAG TTATCGCACA 50
GGTTATCGGC GTATTGATTG GCGTGCAGAT TGGTCTCGGC AATATTCCGC 100
<210> 1213
<211> 100
<212> DNA
<213> Escherichia coli K-12 MG1655
<220> pssa b2586
<222> 1260-1359
<223> pssa b2586 U00096 2720744..2722102
<313> 1213: 1325-1344
<400> 1213
CGATCTGCAA AGTATTGCCG ATTATCCGGT GAAGGTTCGT AAACTCATCC 50
GCCGTTTGCG CCGTATCCGC ATCGACCGAT TAATTAGCCG CATCCTGTAA 100
<210> 1214

<211> 100
<212> DNA
<213> Escherichia coli K-12 MG1655
<220> pssa b2586
<222> 1230-1329
<223> pssa b2586 U00096 2720744..2722102
<313> 1214: 1295-1314
<400> 1214
CGAGCATACC ACCATCGTTA AGCACTATCG CGATCTGCAA AGTATTGCCG  50
ATTATCCGGT GAAGGTTCGT AAACTCATCC GCCGTTTGCG CCGTATCCGC  100
<210> 1215
<211> 100
<212> DNA
<213> Escherichia coli K-12 MG1655
<220> pssa b2586
<222> 1184-1283
<223> pssa b2586 U00096 2720744..2722102
<313> 1215: 1223-1242
<400> 1215
AACTTGAGCT GGCGCCACAG CGAGAGAAAG AACTGGAGCT GATCCGCGAG  50
CATACCACCA TCGTTAAGCA CTATCGCGAT CTGCAAAGTA TTGCCGATTA  100
<210> 1216
<211> 100
<212> DNA
<213> Escherichia coli K-12 MG1655
<220> arca b4402
<222> 618-717
<223> arca b4402 U00096 complement(4637159..4637875)
<313> 1216: 674-693
<400> 1216
CCGTATTCGT AAACATTTCG AATCTACGCC GGATACGCCG GAAATCATCG  50
CCACCATTCA CGGTGAAGGT TATCGCTTCT GCGGTGATCT GGAAGATTAA  100
<210> 1217
<211> 100
<212> DNA
<213> Escherichia coli K-12 MG1655
<220> arca b4402
<222> 564-663
<223> arca b4402 U00096 complement(4637159..4637875)
<313> 1217: 620-639
<400> 1217
AATGACCGGC CGTGAGCTGA AACCGCACGA CCGTACTGTA GACGTGACGA  50
TCCGCCGTAT TCGTAAACAT TTCGAATCTA CGCCGGATAC GCCGGAAATC  100
<210> 1218
<211> 100
<212> DNA
<213> Escherichia coli K-12 MG1655
<220> arca b4402
<222> 501-600
<223> arca b4402 U00096 complement(4637159..4637875)
<313> 1218: 541-560
<400> 1218
CGCCATGCTT CACTTCTGTG AAAACCCAGG CAAAATTCAG TCCCGTGCTG  50
AACTGCTGAA GAAAATGACC GGCCGTGAGC TGAAACCGCA CGACCGTACT  100
<210> 1219
<211> 100
<212> DNA
<213> Escherichia coli K-12 MG1655
<220> arcb b3211
<222> 2232-2331
<223> arcb b3211 U00096 complement(3348330..3350660)
<313> 1219: 2290-2309
<400> 1219
GGAAGATAAC GTCGGTGAAT GGATTGAAGA GATGAAAGAA GAGTGGCGTC  50
ACGACGTAGA AGTGCTGAAA GCGTGGGTGG CAAAAGCCAC TAAAAAATGA  100
<210> 1220
<211> 100
<212> DNA
<213> Escherichia coli K-12 MG1655
<220> arcb b3211
<222> 2167-2266

<223> arcb b3211 U00096 complement(3348330..3350660)
<313> 1220: 2209-2228
<400> 1220
TCAGTGGGGT TACGCCATCT GCAACAGCTG GGTCAGCAAA TTCAGTCTCC 50
TGACCTTCCG GCCTGGGAAG ATAACGTCGG TGAATGGATT GAAGAGATGA 100
<210> 1221
<211> 100
<212> DNA
<213> Escherichia coli K-12 MG1655
<220> arcb b3211
<222> 2109-2208
<223> arcb b3211 U00096 complement(3348330..3350660)
<313> 1221: 2160-2179
<400> 1221
GGCGCAGGAT AAAAAAGGCA TTGTTGAGGA AGGACATAAA ATTAAAGGTG 50
CGGCGGGGTC AGTGGGGTTA CGCCATCTGC AACAGCTGGG TCAGCAAATT 100
<210> 1222
<211> 100
<212> DNA
<213> Escherichia coli K-12 MG1655
<220> arcc b0522
<222> 744-843
<223> arcc b0522 U00096 549662..550555
<313> 1222: 783-802
<400> 1222
GCCATTTGCC AAAGCCGATG GTTCGATGGG GCCGAATGTA ACGGCGGTGA 50
GTGGTTATGT CAGAAGCCGT GGTAAACCCG CGTGGATTGG GGCGTTATCG 100
<210> 1223
<211> 100
<212> DNA
<213> Escherichia coli K-12 MG1655
<220> arcc b0522
<222> 708-807
<223> arcc b0522 U00096 549662..550555
<313> 1223: 736-755
<400> 1223
ACGTGCCATT CGCCATGCCA CACCGGATGA GTTAGCGCCA TTTGCCAAAG 50
CCGATGGTTC GATGGGGCCG AATGTAACGG CGGTGAGTGG TTATGTCAGA 100
<210> 1224
<211> 100
<212> DNA
<213> Escherichia coli K-12 MG1655
<220> arcc b0522
<222> 668-767
<223> arcc b0522 U00096 549662..550555
<313> 1224: 709-728
<400> 1224
ATGCTGATGC GGTATATGAA AACTGGGGAA CGCCGCAGCA ACGTGCCATT 50
CGCCATGCCA CACCGGATGA GTTAGCGCCA TTTGCCAAAG CCGATGGTTC 100
<210> 1225
<211> 100
<212> DNA
<213> Escherichia coli K-12 MG1655
<220> srlr b2708
<222> 675-774
<223> srlr b2708 U00096 2827070..2827843
<313> 1225: 724-743
<400> 1225
CGATAAGCTG ATTACCGACG CAGGTATCGA TCCGGCGTTT CGTCAGGCGC 50
TGGAAGAGAA AGGGATCGAT GTGATCATAA CCGGAGAGAG CAATGAGTGA 100
<210> 1226
<211> 100
<212> DNA
<213> Escherichia coli K-12 MG1655
<220> srlr b2708
<222> 619-718
<223> srlr b2708 U00096 2827070..2827843
<313> 1226: 648-667
<400> 1226
GACTCATCAA AGTTTGGCCG TAAAAGCCCC AACGTAGTTT GCAGTCTTGA 50
AAGCGTCGAT AAGCTGATTA CCGACGCAGG TATCGATCCG GCGTTTCGTC 100

<210> 1227
<211> 100
<212> DNA
<213> Escherichia coli K-12 MG1655
<220> srlr b2708
<222> 534-633
<223> srlr b2708 U00096 2827070..2827843
<313> 1227: 560-579
<400> 1227
CAATGCGGGC GTAACCACCT TTAACGAGGT TTATACCGTC AGTAAGGCAA 50
TGTGCAATGC CGCGCGCGAA GTGATTTTGA TGGCGGACTC ATCAAAGTTT 100
<210> 1228
<211> 100
<212> DNA
<213> Escherichia coli K-12 MG1655
<220> pssr b3764
<222> 215-314
<223> pssr b3764 U00096 complement(3945189..3945590)
<313> 1228: 256-275
<400> 1228
TGAGCACCTG GCAGGCCGCC CGTAAGAGGT GGCGCATACC TCACGACATA 50
ACGAGTTTTC TATCGGTGCC AGCGCCTCGT TGTGGGAATG TATGCTTAAT 100
<210> 1229
<211> 100
<212> DNA
<213> Escherichia coli K-12 MG1655
<220> pssr b3764
<222> 109-208
<223> pssr b3764 U00096 complement(3945189..3945590)
<313> 1229: 143-162
<400> 1229
AGACAACTGG AAAATCAACT GGGTGTGAAC CTTTTCACCC GCCACAGAAA 50
CAATATCCGT TTAACCGCTG CCGGTGAAAA ACTACTGCCT TATGCAGAAA 100
<210> 1230
<211> 100
<212> DNA
<213> Escherichia coli K-12 MG1655
<220> pssr b3764
<222> 59-158
<223> pssr b3764 U00096 complement(3945189..3945590)
<313> 1230: 110-129
<400> 1230
GAGCGGCTGA ATCGCTCTAT CTGACCCAGT CAGCAGTGAG CTTTCGAATC 50
AGACAACTGG AAAATCAACT GGGTGTGAAC CTTTTCACCC GCCACAGAAA 100
<210> 1231
<211> 100
<212> DNA
<213> Escherichia coli K-12 MG1655
<220> topa b1275
<222> 2498-2597
<223> topa b1275 U00096 1329072..1331669
<313> 1231: 2523-2542
<400> 1231
GTAAAACCAA ACAGCAATAT GTCTCTTCGG AAAAAGACGG AAAGGCGACT 50
GGCTGGTCAG CATTTTATGT TGATGGCAAA TGGGTTGAAG GAAAAAAATA 100
<210> 1232
<211> 100
<212> DNA
<213> Escherichia coli K-12 MG1655
<220> topa b1275
<222> 2404-2503
<223> topa b1275 U00096 1329072..1331669
<313> 1232: 2438-2457
<400> 1232
CGCTTCCGCG ACCGTCTGCC GGAAAAACTG CGTTATCTGG CCGATGCGCC 50
ACAGCAGGAT CCGGAAGGTA ATAAGACCAT GGTTCGCTTT AGCCGTAAAA 100
<210> 1233
<211> 100
<212> DNA
<213> Escherichia coli K-12 MG1655
<220> topa b1275

<222> 2318-2417
<223> topa b1275 U00096 1329072..1331669
<313> 1233: 2341-2360
<400> 1233
GTGACGGTGC TGCCGGTGTG TTCCTGGCTG CCAACACTTT CCCGAAATCG 50
CGTGAAACGC GTGCGCCACT GGTGGAAGAG CTTTATCGCT TCCGCGACCG 100
<210> 1234
<211> 100
<212> DNA
<213> Escherichia coli K-12 MG1655
<220> srmb b2577
<222> 1236-1335
<223> srmb b2577 U00096 2710916..2712250
<313> 1234: 1279-1298
<400> 1234
GAAGCCGCGG GTGAAAAAAC GCCATCGCGA CACCAAAAAT ATTGGTAAGC 50
GCCGTAAACC AAGCGGAACG GGCGTGCCAC CGCAAACGAC AGAAGAGTAA 100
<210> 1235
<211> 100
<212> DNA
<213> Escherichia coli K-12 MG1655
<220> srmb b2577
<222> 1162-1261
<223> srmb b2577 U00096 2710916..2712250
<313> 1235: 1207-1226
<400> 1235
GAAAAGCAGA CCGGCAAGCC ATCGAAGAAA GTACTGGCTA AACGTGCTGA 50
GAAGAAAAAA GCTAAAGAGA AAGAGAAGCC GCGGGTGAAA AAACGCCATC 100
<210> 1236
<211> 100
<212> DNA
<213> Escherichia coli K-12 MG1655
<220> srmb b2577
<222> 1129-1228
<223> srmb b2577 U00096 2710916..2712250
<313> 1236: 1175-1194
<400> 1236
GATGAGTTAC GCCCGAAAAC GCGTGCGCCA AGCGAAAAGC AGACCGGCAA 50
GCCATCGAAG AAAGTACTGG CTAAACGTGC TGAGAAGAAA AAAGCTAAAG 100
<210> 1237
<211> 100
<212> DNA
<213> Escherichia coli K-12 MG1655
<220> topb b1764
<222> 1863-1962
<223> topb b1764 U00096 complement(1843023..1844984)
<313> 1237: 1932-1951
<400> 1237
CAGTGGTGGC AGTGCTGATA AGAAAAAGGC TGCACCGCGT AAACGTAGTG 50
CGAAAAAAAG TCCGCCAGCA GATGAAGTCG GAAGCGGGGC GATAGCGTAA 100
<210> 1238
<211> 100
<212> DNA
<213> Escherichia coli K-12 MG1655
<220> topb b1764
<222> 1842-1941
<223> topb b1764 U00096 complement(1843023..1844984)
<313> 1238: 1891-1910
<400> 1238
TCGCGGCATT GTGGCTCCGG GCAGTGGTGG CAGTGCTGAT AAGAAAAAGG 50
CTGCACCGCG TAAACGTAGT GCGAAAAAAA GTCCGCCAGC AGATGAAGTC 100
<210> 1239
<211> 100
<212> DNA
<213> Escherichia coli K-12 MG1655
<220> topb b1764
<222> 1795-1894
<223> topb b1764 U00096 complement(1843023..1844984)
<313> 1239: 1838-1857
<400> 1239
CTATATCAGC TTATTGATCA AGCCAAACGT ACGCCGGTGC GGCAGTTTCG 50

CGGCATTGTG GCTCCGGGCA GTGGTGGCAG TGCTGATAAG AAAAAGGCTG 100
<210> 1240
<211> 100
<212> DNA
<213> Escherichia coli K-12 MG1655
<220> psta b3727
<222> 744-843
<223> psta b3727 U00096 complement(3906177..3907067)
<313> 1240: 784-803
<400> 1240
CCTGCCGGTG ACGATCTTTA AGTTTGCGAT GAGCCCGTTT GCGGAATGGC 50
AGCAATTGGC CTGGGCCGGG GTATTGATCA TTACCCTGTG CGTACTGCTG 100
<210> 1241
<211> 100
<212> DNA
<213> Escherichia coli K-12 MG1655
<220> psta b3727
<222> 636-735
<223> psta b3727 U00096 complement(3906177..3907067)
<313> 1241: 678-697
<400> 1241
CGGTATCCTG CTGGCGATTG CCCGTATTGC TGGTGAAACC GCGCCGCTGC 50
TGTTTACCGC GCTCTCCAAC CAGTTCTGGA GCACGGACAT GATGCAGCCG 100
<210> 1242
<211> 100
<212> DNA
<213> Escherichia coli K-12 MG1655
<220> psta b3727
<222> 543-642
<223> psta b3727 U00096 complement(3906177..3907067)
<313> 1242: 583-602
<400> 1242
CAGCCTGCGT GAAGCGGCTT ATGCGCTGGG TACACCGAAG TGGAAGATGA 50
TCTCTGCGAT TACGCTGAAA GCGTCGGTGT CCGGGATTAT GACCGGTATC 100
<210> 1243
<211> 100
<212> DNA
<213> Escherichia coli K-12 MG1655
<220> pstb b3726
<222> 675-774
<223> pstb b3726 U00096 complement(3905221..3905994)
<313> 1243: 739-758
<400> 1243
GTACCTGGGC GAATTGATTG AGTTCAGCAA CACGGACGAT CTGTTCACCA 50
AGCCAGCGAA GAAACAAACA GAAGACTACA TCACCGGTCG TTACGGTTGA 100
<210> 1244
<211> 100
<212> DNA
<213> Escherichia coli K-12 MG1655
<220> pstb b3726
<222> 625-724
<223> pstb b3726 U00096 complement(3905221..3905994)
<313> 1244: 663-682
<400> 1244
ACCCACAACA TGCAGCAGGC TGCGCGTTGT TCCGACCACA CGGCGTTTAT 50
GTACCTGGGC GAATTGATTG AGTTCAGCAA CACGGACGAT CTGTTCACCA 100
<210> 1245
<211> 100
<212> DNA
<213> Escherichia coli K-12 MG1655
<220> pstb b3726
<222> 562-661
<223> pstb b3726 U00096 complement(3905221..3905994)
<313> 1245: 589-608
<400> 1245
TCTACCGGGC GTATTGAAGA GCTGATCACC GAACTGAAGC AGGATTACAC 50
CGTGGTGATC GTCACCCACA ACATGCAGCA GGCTGCGCGT TGTTCCGACC 100
<210> 1246
<211> 100
<212> DNA
<213> Escherichia coli K-12 MG1655

<220> pstc b3728
<222> 861-960
<223> pstc b3728 U00096 complement(3907067..3908026)
<313> 1246: 889-908
<400> 1246
GATGGAACTG GGCCTGATCC TGTTTGTGAT TACCTTCATC GTCCTCGCCG  50
CATCGAAGTT TATGATTATG CGCCTGGCTA AGAATGAGGG GGCACGCTAA  100
<210> 1247
<211> 100
<212> DNA
<213> Escherichia coli K-12 MG1655
<220> pstc b3728
<222> 781-880
<223> pstc b3728 U00096 complement(3907067..3908026)
<313> 1247: 846-865
<400> 1247
TATATGCCGG GCAACAGTAT CACCTCTGCG CTGGCGAACG AATTTGCGGA  50
AGCGGAATCC GGTCTGCACG TTGCCGCACT GATGGAACTG GGCCTGATCC  100
<210> 1248
<211> 100
<212> DNA
<213> Escherichia coli K-12 MG1655
<220> pstc b3728
<222> 726-825
<223> pstc b3728 U00096 complement(3907067..3908026)
<313> 1248: 748-767
<400> 1248
CATGGCGGTG ACCTTTATCA TCGGTAACAC CTACCAGCTC GACAGCGCCT  50
CGCTGTATAT GCCGGGCAAC AGTATCACCT CTGCGCTGGC GAACGAATTT  100
<210> 1249
<211> 100
<212> DNA
<213> Escherichia coli K-12 MG1655
<220> acca b0186
<222> 839-938
<223> acca b0186 U00096 208621..209580
<313> 1249: 882-901
<400> 1249
AAGCGATGGC GGCATCGTTG AAAGCGCAAC TGCTGGCGGA TCTGGCCGAT  50
CTCGACGTGT TAAGCACTGA AGATTTAAAA AATCGTCGTT ATCAGCGCCT  100
<210> 1250
<211> 100
<212> DNA
<213> Escherichia coli K-12 MG1655
<220> acca b0186
<222> 751-850
<223> acca b0186 U00096 208621..209580
<313> 1250: 787-806
<400> 1250
ATGGGTATCA TTGCTCCGCG TCTGAAAGAA CTGAAACTGA TCGACTCCAT  50
CATCCCGGAA CCACTGGGTG GTGCTCACCG TAACCCGGAA GCGATGGCGG  100
<210> 1251
<211> 100
<212> DNA
<213> Escherichia coli K-12 MG1655
<220> acca b0186
<222> 653-752
<223> acca b0186 U00096 208621..209580
<313> 1251: 689-708
<400> 1251
ATATGCTGCA ATACAGCACC TATTCCGTTA TCTCGCCGGA AGGTTGTGCG  50
TCCATTCTGT GGAAGAGCGC CGACAAAGCG CCGCTGGCGG CTGAAGCGAT  100
<210> 1252
<211> 100
<212> DNA
<213> Escherichia coli K-12 MG1655
<220> accb b3256
<222> 343-442
<223> accb b3256 U00096 3403073..3403543
<313> 1252: 369-388
<400> 1252

CTGTGCATCG TTGAAGCCAT GAAAATGATG AACCAGATCG AAGCGGACAA 50
ATCCGGTACC GTGAAAGCAA TTCTGGTCGA AAGTGGACAA CCGGTAGAAT 100
<210> 1253
<211> 100
<212> DNA
<213> Escherichia coli K-12 MG1655
<220> accb b3256
<222> 291-390
<223> accb b3256 U00096 3403073..3403543
<313> 1253: 322-341
<400> 1253
GGACGCAAAA GCGTTCATCG AAGTGGGTCA GAAAGTCAAC GTGGGCGATA 50
CCCTGTGCAT CGTTGAAGCC ATGAAAATGA TGAACCAGAT CGAAGCGGAC 100
<210> 1254
<211> 100
<212> DNA
<213> Escherichia coli K-12 MG1655
<220> accb b3256
<222> 229-328
<223> accb b3256 U00096 3403073..3403543
<313> 1254: 272-291
<400> 1254
GAAATCAGTG GTCACATCGT ACGTTCCCCG ATGGTTGGTA CTTTCTACCG 50
CACCCCAAGC CCGGACGCAA AAGCGTTCAT CGAAGTGGGT CAGAAAGTCA 100
<210> 1255
<211> 100
<212> DNA
<213> Escherichia coli K-12 MG1655
<220> psts b3729
<222> 904-1003
<223> psts b3729 U00096 complement(3908113..3909153)
<313> 1255: 952-971
<400> 1255
TTCGACTGGG CGTACAAAAC CGGGGCTAAA CAGGCGAACG ACCTGGATTA 50
CGCCAGCCTG CCGGATAGTG TAGTTGAACA GGTTCGCGCT GCGTGGAAGA 100
<210> 1256
<211> 100
<212> DNA
<213> Escherichia coli K-12 MG1655
<220> psts b3729
<222> 864-963
<223> psts b3729 U00096 complement(3908113..3909153)
<313> 1256: 922-941
<400> 1256
TCAGAAGAAA CCAGAACAAG GCACAGAAGT GCTGAAATTC TTCGACTGGG 50
CGTACAAAAC CGGGGCTAAA CAGGCGAACG ACCTGGATTA CGCCAGCCTG 100
<210> 1257
<211> 100
<212> DNA
<213> Escherichia coli K-12 MG1655
<220> psts b3729
<222> 848-947
<223> psts b3729 U00096 complement(3908113..3909153)
<313> 1257: 872-891
<400> 1257
TTCTGATCCA CAAAGATCAG AAGAAACCAG AACAAGGCAC AGAAGTGCTG 50
AAATTCTTCG ACTGGGCGTA CAAAACCGGG GCTAAACAGG CGAACGACCT 100
<210> 1258
<211> 100
<212> DNA
<213> Escherichia coli K-12 MG1655
<220> accc b3257
<222> 1251-1350
<223> accc b3257 U00096 3403554..3404903
<313> 1258: 1310-1329
<400> 1258
CGTTGATCTG CAGATCCGCA TCATGAATGA CGAGAACTTC CAGCATGGTG 50
GCACTAACAT CCACTATCTG GAGAAAAAAC TCGGTCTTCA GGAAAAATAA 100
<210> 1259
<211> 100
<212> DNA

<213> Escherichia coli K-12 MG1655
<220> accc b3257
<222> 1112-1211
<223> accc b3257 U00096 3403554..3404903
<313> 1259: 1159-1178
<400> 1259
TCTACGCGGG CTACACCGTA CCGCCGTACT ATGACTCAAT GATCGGTAAG 50
CTGATTTGCT ACGGTGAAAA CCGTGACGTG GCGATTGCCC GCATGAAGAA 100
<210> 1260
<211> 100
<212> DNA
<213> Escherichia coli K-12 MG1655
<220> accc b3257
<222> 1099-1198
<223> accc b3257 U00096 3403554..3404903
<313> 1260: 1120-1139
<400> 1260
TGGGAGTCTC ATATCTACGC GGGCTACACC GTACCGCCGT ACTATGACTC 50
AATGATCGGT AAGCTGATTT GCTACGGTGA AAACCGTGAC GTGGCGATTG 100
<210> 1261
<211> 100
<212> DNA
<213> Escherichia coli K-12 MG1655
<220> accd b2317
<222> 802-901
<223> accd b2317 U00096 complement(2431032..2431946)
<313> 1261: 861-880
<400> 1261
CTGAAACTGG CGAGCATTCT GGCGAAGTTG ATGAATCTGC CAGCGCCGAA 50
TCCTGAAGCG CCGCGTGAAG GCGTAGTGGT ACCCCCGGTA CCGGATCAGG 100
<210> 1262
<211> 100
<212> DNA
<213> Escherichia coli K-12 MG1655
<220> accd b2317
<222> 791-890
<223> accd b2317 U00096 complement(2431032..2431946)
<313> 1262: 815-834
<400> 1262
CGGAAATGCG CCTGAAACTG GCGAGCATTC TGGCGAAGTT GATGAATCTG 50
CCAGCGCCGA ATCCTGAAGC GCCGCGTGAA GGCGTAGTGG TACCCCCGGT 100
<210> 1263
<211> 100
<212> DNA
<213> Escherichia coli K-12 MG1655
<220> accd b2317
<222> 697-796
<223> accd b2317 U00096 complement(2431032..2431946)
<313> 1263: 745-764
<400> 1263
ATCGAACAGA CCGTTCGCGA AAAACTGCCG CCTGGATTCC AGCGCAGTGA 50
ATTCCTGATC GAGAAAGGCG CGATCGACAT GATCGTCCGT CGTCCGGAAA 100
<210> 1264
<211> 100
<212> DNA
<213> Escherichia coli K-12 MG1655
<220> frua b2168
<222> 1489-1588
<223> frua b2168 U00096 complement(2257739..2259430)
<313> 1264: 1545-1564
<400> 1264
GGGGCGCTGA CTGGCGCAAT CTCAATGGCG ATTGGTGCGA AACTGATGGC 50
ACCGCACGGT GGTCTGTTTG TTCTGCTGAT CCCTGGCGCT ATTACGCCGG 100
<210> 1265
<211> 100
<212> DNA
<213> Escherichia coli K-12 MG1655
<220> frua b2168
<222> 1448-1547
<223> frua b2168 U00096 complement(2257739..2259430)
<313> 1265: 1502-1521

<400> 1265
CTCGTGATCC GATGCGTGTG CTGCCGTGCT GTATCGTGGG TGGGGCGCTG  50
ACTGGCGCAA TCTCAATGGC GATTGGTGCG AAACTGATGG CACCGCACGG  100
<210> 1266
<211> 100
<212> DNA
<213> Escherichia coli K-12 MG1655
<220> frua b2168
<222> 1382-1481
<223> frua b2168 U00096 complement(2257739..2259430)
<313> 1266: 1443-1462
<400> 1266
AAGGTGGCAA AGCCGCTCTG GTATTGGGAC TGTGCTTCAT TTCGGAAGGT  50
GCAATTCCGT TTGCTGCTCG TGATCCGATG CGTGTGCTGC CGTGCTGTAT  100
<210> 1267
<211> 100
<212> DNA
<213> Escherichia coli K-12 MG1655
<220> frub b2170
<222> 1014-1113
<223> frub b2170 U00096 complement(2260385..2261515)
<313> 1267: 1067-1086
<400> 1267
GGCACTTGGC GTTAAGAAAG GTCATCGCCT ACGCTTTACC GCCCAGGGTG  50
CAGATGCTGA ACAGGCGCTG AAAGCAATCG GCGACGCTAT CGCTGCTGGT  100
<210> 1268
<211> 100
<212> DNA
<213> Escherichia coli K-12 MG1655
<220> frub b2170
<222> 952-1051
<223> frub b2170 U00096 complement(2260385..2261515)
<313> 1268: 1001-1020
<400> 1268
ACCGTGACAA ACCTTGATGG TACCGGCAAA CCGGCAAACG GACGTAGTCT  50
GATGAAAGTT GTGGCACTTG GCGTTAAGAA AGGTCATCGC CTACGCTTTA  100
<210> 1269
<211> 100
<212> DNA
<213> Escherichia coli K-12 MG1655
<220> frub b2170
<222> 815-914
<223> frub b2170 U00096 complement(2260385..2261515)
<313> 1269: 836-855
<400> 1269
TGGCGCTGCT GACCAGCGAT GATGCGCCGA CCGACGACGT GTTAAGCGCG  50
GAGTTTGTGG TGCGCAATGA ACACGGCCTG CATGCTCGTC CAGGTACCAT  100
<210> 1270
<211> 100
<212> DNA
<213> Escherichia coli K-12 MG1655
<220> fruk b2169
<222> 773-872
<223> fruk b2169 U00096 complement(2259447..2260385)
<313> 1270: 818-837
<400> 1270
TTGGTGGCCT GATTTATGGC TTGCTGATGC GTGAATCCAG TGAACACACA  50
CTGCGTCTGG CGACAGCTGT TGCAGCCCTG GCGGTAAGTC AAAGCAATGT  100
<210> 1271
<211> 100
<212> DNA
<213> Escherichia coli K-12 MG1655
<220> fruk b2169
<222> 723-822
<223> fruk b2169 U00096 complement(2259447..2260385)
<313> 1271: 761-780
<400> 1271
ACCACCGTCA GTCGATGTCG TAAGCACCGT TGGCGCAGGG GATTCTATGG  50
TTGGTGGCCT GATTTATGGC TTGCTGATGC GTGAATCCAG TGAACACACA  100
<210> 1272
<211> 100

<212> DNA
<213> Escherichia coli K-12 MG1655
<220> fruk b2169
<222> 676-775
<223> fruk b2169 U00096 complement(2259447..2260385)
<313> 1272: 717-736
<400> 1272
GCCGAAGGCG CGCTTTGGGT TAATGCCTCC GGCGAATGGA TCGCCAAACC  50
ACCGTCAGTC GATGTCGTAA GCACCGTTGG CGCAGGGGAT TCTATGGTTG  100
<210> 1273
<211> 100
<212> DNA
<213> Escherichia coli K-12 MG1655
<220> frul b0080
<222> 1-87
<223> frul b0080 U00096 87860..87946
<313> 1273: 47-66
<400> 1273
ATGCGCAATT TACAGCCCAA CATGTCACGT TGGGCTTTTT TTGCGAAATC  50
AGTGGGAACC TGGAATAAAA GCAGTTGCCG CAGTTAA  100
<210> 1274
<211> 100
<212> DNA
<213> Escherichia coli K-12 MG1655
<220> frur b0081
<222> 905-1004
<223> frur b0081 U00096 88028..89032
<313> 1274: 945-964
<400> 1274
AGATTGTCCT GGCAAGCCTG GACGAACCGC GTAAGCCAAA ACCTGGTTTA  50
ACGCGCATTA AACGTAATCT CTATCGCCGC GGCGTGCTCA GCCGTAGCTA  100
<210> 1275
<211> 100
<212> DNA
<213> Escherichia coli K-12 MG1655
<220> frur b0081
<222> 887-986
<223> frur b0081 U00096 88028..89032
<313> 1275: 906-925
<400> 1275
TCGCAGAGCG TGTGCTGGAG ATTGTCCTGG CAAGCCTGGA CGAACCGCGT  50
AAGCCAAAAC CTGGTTTAAC GCGCATTAAA CGTAATCTCT ATCGCCGCGG  100
<210> 1276
<211> 100
<212> DNA
<213> Escherichia coli K-12 MG1655
<220> frur b0081
<222> 796-895
<223> frur b0081 U00096 88028..89032
<313> 1276: 836-855
<400> 1276
TCTGACCTGG CAATTGCCAC CTTTGGCGAT AACGAACTGC TCGACTTCTT  50
ACAGTGTCCG GTGCTGGCAG TGGCTCAACG TCACCGCGAT GTCGCAGAGC  100
<210> 1277
<211> 100
<212> DNA
<213> Escherichia coli K-12 MG1655
<220> dind b3646
<222> 738-837
<223> dind b3646 U00096 3815375..3816211
<313> 1277: 783-802
<400> 1277
CATTCAGGAA CTTGGCGGAA CCATGCCTGA GGAGTTGCCG ACCCCGCAGG  50
TCAGCATCAA GCAGTTGGAA AACAGTGTAA AAATTACAGA GAAGAAATAG  100
<210> 1278
<211> 100
<212> DNA
<213> Escherichia coli K-12 MG1655
<220> dind b3646
<222> 727-826
<223> dind b3646 U00096 3815375..3816211

<313> 1278: 745-764
<400> 1278
GTGAGGCAAA CCATTCAGGA ACTTGGCGGA ACCATGCCTG AGGAGTTGCC 50
GACCCCGCAG GTCAGCATCA AGCAGTTGGA AAACAGTGTA AAAATTACAG 100
<210> 1279
<211> 100
<212> DNA
<213> Escherichia coli K-12 MG1655
<220> dind b3646
<222> 638-737
<223> dind b3646 U00096 3815375..3816211
<313> 1279: 695-714
<400> 1279
GAGCTACCCA AACAGAAGAA AAACTCAAGC GGGATGGCGT GAATTCAAAA 50
CAGCAAGCAA ACACCACACA CTTTGACGTG GGTCGCAAGG TGAGGCAAAC 100
<210> 1280
<211> 100
<212> DNA
<213> Escherichia coli K-12 MG1655
<220> plda b3822
<222> 771-870
<223> plda b3822 U00096 4002473..4003342
<313> 1280: 812-831
<400> 1280
TTATACTCAG GTTTACAGCG GCTATGGCGA ATCGCTCATC GACTATAACT 50
TCAACCAGAC CCGTGTCGGT GTGGGGGTTA TGCTAAACGA TTTGTTTTGA 100
<210> 1281
<211> 100
<212> DNA
<213> Escherichia coli K-12 MG1655
<220> plda b3822
<222> 747-846
<223> plda b3822 U00096 4002473..4003342
<313> 1281: 762-781
<400> 1281
CCCGATCACC AAACATGTGC GCCTTTATAC TCAGGTTTAC AGCGGCTATG 50
GCGAATCGCT CATCGACTAT AACTTCAACC AGACCCGTGT CGGTGTGGGG 100
<210> 1282
<211> 100
<212> DNA
<213> Escherichia coli K-12 MG1655
<220> plda b3822
<222> 670-769
<223> plda b3822 U00096 4002473..4003342
<313> 1282: 706-725
<400> 1282
GGTGATGCGG TGCTCAGTGC GAAAGGACAG TACAACTGGA ACACCGGCTA 50
CGGCGGCGCG GAGTTAGGCT TAAGTTACCC GATCACCAAA CATGTGCGCC 100
<210> 1283
<211> 100
<212> DNA
<213> Escherichia coli K-12 MG1655
<220> dinf b4045
<222> 1267-1366
<223> dinf b4045 U00096 4255321..4256700
<313> 1283: 1315-1334
<400> 1283
GGTAATCATG CTTTGTGGCT GGCATTAACC GTCTTTCTGG CGTTGCGCGG 50
GCTTTCTCTG GCGGCTATCT GGCGGCGTCA CTGGCGCAAT GGTACCTGGT 100
<210> 1284
<211> 100
<212> DNA
<213> Escherichia coli K-12 MG1655
<220> dinf b4045
<222> 1221-1320
<223> dinf b4045 U00096 4255321..4256700
<313> 1284: 1283-1302
<400> 1284
GGCCGCCGCA GGTTTTGCGC TGACGCTCCT TACGCTGCCG TGGCTGGGTA 50
ATCATGCTTT GTGGCTGGCA TTAACCGTCT TTCTGGCGTT GCGCGGGCTT 100
<210> 1285

<211> 100
<212> DNA
<213> Escherichia coli K-12 MG1655
<220> dinf b4045
<222> 1182-1281
<223> dinf b4045 U00096 4255321..4256700
<313> 1285: 1224-1243
<400> 1285
CGCAACGCGT GCCACCGAAA TGCGTAACAG TATGGCGGTG GCCGCCGCAG 50
GTTTTGCGCT GACGCTCCTT ACGCTGCCGT GGCTGGGTAA TCATGCTTTG 100
<210> 1286
<211> 100
<212> DNA
<213> Escherichia coli K-12 MG1655
<220> pldb b3826
<222> 924-1023
<223> pldb b3826 U00096 4006776..4007798
<313> 1286: 991-1010
<400> 1286
TTTTGAAAAG GACGCAATGG CCTCAGTCGC GCTCCACGCC ATCGTTGATT 50
TTTTCAACAG GCATAACTCA CCCAGCGGAA ACCGCTCTAC AGAGGTTTAA 100
<210> 1287
<211> 100
<212> DNA
<213> Escherichia coli K-12 MG1655
<220> pldb b3826
<222> 918-1017
<223> pldb b3826 U00096 4006776..4007798
<313> 1287: 963-982
<400> 1287
GATCCTTTTT GAAAAGGACG CAATGGCCTC AGTCGCGCTC CACGCCATCG 50
TTGATTTTTT CAACAGGCAT AACTCACCCA GCGGAAACCG CTCTACAGAG 100
<210> 1288
<211> 100
<212> DNA
<213> Escherichia coli K-12 MG1655
<220> pldb b3826
<222> 707-806
<223> pldb b3826 U00096 4006776..4007798
<313> 1288: 758-777
<400> 1288
CGACCTACCA TTGGGTACGC GAAAGTATTC TGGCTGGCGA ACAGGTGTTA 50
GCCGGTGCGG GTGATGACGC CACGCCAACG CTTCTCTTGC AGGCTGAAGA 100
<210> 1289
<211> 100
<212> DNA
<213> Escherichia coli K-12 MG1655
<220> ding b0800
<222> 1969-2068
<223> ding b0800 U00096 832293..834443
<313> 1289: 2005-2024
<400> 1289
CTGATTCGAA GCCACGGTTG CTGGGGCGAA GTGGTTATCT ACGACAAACG 50
CTTGCTGACC AAAAATTATG GCAAGCGACT ACTGGATGCA TTACCGGTAT 100
<210> 1290
<211> 100
<212> DNA
<213> Escherichia coli K-12 MG1655
<220> ding b0800
<222> 1909-2008
<223> ding b0800 U00096 832293..834443
<313> 1290: 1951-1970
<400> 1290
CCGTTTGAGG TGCAAAGCCT GCCGAGCGCC TCGTTTAACC TGATTCAGCA 50
GGTTGGGCGA CTGATTCGAA GCCACGGTTG CTGGGGCGAA GTGGTTATCT 100
<210> 1291
<211> 100
<212> DNA
<213> Escherichia coli K-12 MG1655
<220> ding b0800
<222> 1838-1937

<223> ding b0800 U00096 832293..834443
<313> 1291: 1882-1901
<400> 1291
TCGCTTTTCC GCCCATCGAC AGCCCGGTGG TGATCACCGA AGGGGAATGG 50
CTGAAAAGCC TCAACCGCTA TCCGTTTGAG GTGCAAAGCC TGCCGAGCGC 100
<210> 1292
<211> 100
<212> DNA
<213> Escherichia coli K-12 MG1655
<220> dini b1062
<222> 147-246
<223> dini b1062 U00096 complement(1120465..1120710)
<313> 1292: 208-227
<400> 1292
TTTATCGGTT ATTGGCGCGA CAAAAGAAGA TAAACAGCGC ATTAGTGAAA 50
TTCTCCAGGA AACGTGGGAA AGCGCCGATG ACTGGTTTGT CAGCGAATAA 100
<210> 1293
<211> 100
<212> DNA
<213> Escherichia coli K-12 MG1655
<220> dini b1062
<222> 121-220
<223> dini b1062 U00096 complement(1120465..1120710)
<313> 1293: 153-172
<400> 1293
TCGGTACGTT ATGCCGCAGC GAATAATTTA TCGGTTATTG GCGCGACAAA 50
AGAAGATAAA CAGCGCATTA GTGAAATTCT CCAGGAAACG TGGGAAAGCG 100
<210> 1294
<211> 100
<212> DNA
<213> Escherichia coli K-12 MG1655
<220> dini b1062
<222> 58-157
<223> dini b1062 U00096 complement(1120465..1120710)
<313> 1294: 97-116
<400> 1294
GCCCTGGCTG GCGAACTTTC CCGCCGTATT CAGTATGCGT TTCCTGATAA 50
TGAAGGCCAC GTATCGGTAC GTTATGCCGC AGCGAATAAT TTATCGGTTA 100
<210> 1295
<211> 100
<212> DNA
<213> Escherichia coli K-12 MG1655
<220> dinj b0227
<222> 124-223
<223> dinj b0227 U00096 complement(246242..246502)
<313> 1295: 153-172
<400> 1295
GCGCGTGAAA AGGCATTGCC GTTTGATTTA CGCGAGCCTA ATCAATTAAC 50
CATTCAATCA ATCAAAAACA GCGAAGCTGG CATTGATGTT CATAAGGCCA 100
<210> 1296
<211> 100
<212> DNA
<213> Escherichia coli K-12 MG1655
<220> dinj b0227
<222> 71-170
<223> dinj b0227 U00096 complement(246242..246502)
<313> 1296: 91-110
<400> 1296
CCGGGATGGG GCTGACCATC TCTGACCTGG TTCGCATAAC CCTCACAAAG 50
GTCGCGCGTG AAAAGGCATT GCCGTTTGAT TTACGCGAGC CTAATCAATT 100
<210> 1297
<211> 100
<212> DNA
<213> Escherichia coli K-12 MG1655
<220> dinj b0227
<222> 1-100
<223> dinj b0227 U00096 complement(246242..246502)
<313> 1297: 48-67
<400> 1297
ATGGCTGCTA ACGCGTTTGT TCGCGCCCGA ATCGATGAAG ATCTGAAGAA 50
TCAGGCAGCG GACGTACTGG CCGGGATGGG GCTGACCATC TCTGACCTGG 100

<210> 1298
<211> 100
<212> DNA
<213> Escherichia coli K-12 MG1655
<220> b2100 b2101
<222> 867-966
<223> b2100 b2101 U00096 2179116..2180081
<313> 1298: 895-914
<400> 1298
ATTACTGGGC AATGCAGTGG CGTCGTGGGT TGTCGGGCAT CGGGGCGGTG 50
ATTGTGCGCC AACGCGCGAG GAACTACTCC TCGCACACAA AAACGTATAG 100
<210> 1299
<211> 100
<212> DNA
<213> Escherichia coli K-12 MG1655
<220> b2100 b2101
<222> 735-834
<223> b2100 b2101 U00096 2179116..2180081
<313> 1299: 766-785
<400> 1299
TTTCAGCAAC GACGCTTCTG GCTGCATTCC GGCATTTCCA ACGCAAGTTG 50
TCGACACCAT TGGGGCGGGC GACAGTCATG CCGGTGGCGT ACTTGCCGGG 100
<210> 1300
<211> 100
<212> DNA
<213> Escherichia coli K-12 MG1655
<220> b2100 b2101
<222> 682-781
<223> b2100 b2101 U00096 2179116..2180081
<313> 1300: 711-730
<400> 1300
GAGAAATTTG CCGCGCCGTT GATCGTTCGT CTCGATAAAG AAGGCGCATG 50
GTATTTCAGC AACGACGCTT CTGGCTGCAT TCCGGCATTT CCAACGCAAG 100
<210> 1301
<211> 100
<212> DNA
<213> Escherichia coli K-12 MG1655
<220> dinp b0232
<222> 866-965
<223> dinp b0232 U00096 250898..251953
<313> 1301: 886-905
<400> 1301
AATTAAAGTT CGACGATTTT CAGCAAACCA CCCAGGAGCA CGTCTGGCCG 50
CGGCTGAATA AAGCTGATCT AATCGCCACC GCGCGTAAAA CCTGGGATGA 100
<210> 1302
<211> 100
<212> DNA
<213> Escherichia coli K-12 MG1655
<220> dinp b0232
<222> 815-914
<223> dinp b0232 U00096 250898..251953
<313> 1302: 853-872
<400> 1302
GCCGTCTGGC AAAGGTAAAA CCTGATTTAC TGATTGCTCG CCAGGGGGTG 50
AAATTAAAGT TCGACGATTT TCAGCAAACC ACCCAGGAGC ACGTCTGGCC 100
<210> 1303
<211> 100
<212> DNA
<213> Escherichia coli K-12 MG1655
<220> dinp b0232
<222> 732-831
<223> dinp b0232 U00096 250898..251953
<313> 1303: 761-780
<400> 1303
CGTGGAACGC ACGATGGCGG AAGATATTCA TCACTGGTCT GAATGTGAAG 50
CGATTATCGA GCGGCTGTAT CCGGAACTTG AACGCCGTCT GGCAAAGGTA 100
<210> 1304
<211> 100
<212> DNA
<213> Escherichia coli K-12 MG1655
<220> b2107 b2108

<222> 420-519
<223> b2107 b2108 U00096 2184800..2185318
<313> 1304: 482-501
<400> 1304
GACGCACTGG ACCTATATGG GTGGCGCGTA CGTGTTGATC AGCGACACCG  50
ACGGTAAAAT CATTAAAGCC TACGACGGTG AGATTTTTTA TCATCGCTAA  100
<210> 1305
<211> 100
<212> DNA
<213> Escherichia coli K-12 MG1655
<220> b2107 b2108
<222> 386-485
<223> b2107 b2108 U00096 2184800..2185318
<313> 1305: 400-419
<400> 1305
GGCAGTTGCG TAACCTGCCC GCGCCTGATG CCGGGACGCA CTGGACCTAT  50
ATGGGTGGCG CGTACGTGTT GATCAGCGAC ACCGACGGTA AAATCATTAA  100
<210> 1306
<211> 100
<212> DNA
<213> Escherichia coli K-12 MG1655
<220> b2107 b2108
<222> 304-403
<223> b2107 b2108 U00096 2184800..2185318
<313> 1306: 359-378
<400> 1306
TTCATTGCTG ACTTTAAGCA TTTCAAACCA GGGGACACCG TACCAGAAAT  50
GTACCGTACC GATGAGTACA ACATTAAGCA GTGGCAGTTG CGTAACCTGC  100
<210> 1307
<211> 100
<212> DNA
<213> Escherichia coli K-12 MG1655
<220> frva b3901
<222> 345-444
<223> frva b3901 U00096 complement(4089958..4090404)
<313> 1307: 388-407
<400> 1307
CACACTGTGT CGCAAAATTA TTCACAAGGA ATTTATTCAT CAACTGCAAC  50
AGGGCGATAC CGACCAGGTG CTTGCCTTGT TAAATCAAAC CCTCAGCTCA  100
<210> 1308
<211> 100
<212> DNA
<213> Escherichia coli K-12 MG1655
<220> frva b3901
<222> 295-394
<223> frva b3901 U00096 complement(4089958..4090404)
<313> 1308: 331-350
<400> 1308
TGCCTCGGCG TGCCGCAAAG CGGCGAAGAG GATCAGGTCA AAATCATCGG  50
CACACTGTGT CGCAAAATTA TTCACAAGGA ATTTATTCAT CAACTGCAAC  100
<210> 1309
<211> 100
<212> DNA
<213> Escherichia coli K-12 MG1655
<220> frva b3901
<222> 213-312
<223> frva b3901 U00096 complement(4089958..4090404)
<313> 1309: 239-258
<400> 1309
ACAACCGTTC GTATTATTCG CCCGCAAAGC GCAGGCTATT GACTGGAAAG  50
CCAGCGATGG CGAAGACGTC AATTGCTGGA TCTGCCTCGG CGTGCCGCAA  100
<210> 1310
<211> 100
<212> DNA
<213> Escherichia coli K-12 MG1655
<220> frvb b3900
<222> 1359-1458
<223> frvb b3900 U00096 complement(4088496..4089953)
<313> 1310: 1432-1451
<400> 1310
TGGCCTCGCG CTGAACATTT CATTCATTAT CGTCCTGAAA GGATTGTGGT  50

TACGCCGCAA AGCGAAAGCC GCGCAACAGG AGCTTGTCCA TGAACATTGA 100
<210> 1311
<211> 100
<212> DNA
<213> Escherichia coli K-12 MG1655
<220> frvb b3900
<222> 1333-1432
<223> frvb b3900 U00096 complement(4088496..4089953)
<313> 1311: 1367-1386
<400> 1311
GGTTCGTTTT ATCTGGTGCT GGCGATTGGC CTCGCGCTGA ACATTTCATT 50
CATTATCGTC CTGAAAGGAT TGTGGTTACG CCGCAAAGCG AAAGCCGCGC 100
<210> 1312
<211> 100
<212> DNA
<213> Escherichia coli K-12 MG1655
<220> frvb b3900
<222> 1262-1361
<223> frvb b3900 U00096 complement(4088496..4089953)
<313> 1312: 1292-1311
<400> 1312
CTTTCGGAAT CAAACGTCTG GCACCTGGTT TGGGTATCTT TGACCCGCTG 50
ATCGGCCTGA TGTCGCCGGT GGGTTCGTTT TATCTGGTGC TGGCGATTGG 100
<210> 1313
<211> 100
<212> DNA
<213> Escherichia coli K-12 MG1655
<220> tora b0998
<222> 2446-2545
<223> tora b0998 U00096 1058479..1061025
<313> 1313: 2494-2513
<400> 1313
GAGAAGTACA ACGGAACAGT GGAGCAGGTG ACGGCGTTTA ACGGCCCCGT 50
GGAGATGGTG GCGCAGTGCG AATATGTTCC CGCGTCGCAG GTGAAATCAT 100
<210> 1314
<211> 100
<212> DNA
<213> Escherichia coli K-12 MG1655
<220> tora b0998
<222> 2418-2517
<223> tora b0998 U00096 1058479..1061025
<313> 1314: 2461-2480
<400> 1314
CAGTGCGCAC ACTACGCTGG TGGAAATTGA GAAGTACAAC GGAACAGTGG 50
AGCAGGTGAC GGCGTTTAAC GGCCCCGTGG AGATGGTGGC GCAGTGCGAA 100
<210> 1315
<211> 100
<212> DNA
<213> Escherichia coli K-12 MG1655
<220> tora b0998
<222> 2281-2380
<223> tora b0998 U00096 1058479..1061025
<313> 1315: 2321-2340
<400> 1315
GCACCCGGCG TGGCACGAAT TCACGAAGGG GCATGGTACG ATCCAGATAA 50
AGGCGGCGAG CCTGGTGCGC TGTGCAAATA CGGTAACCCC AACGTGTTGA 100
<210> 1316
<211> 100
<212> DNA
<213> Escherichia coli K-12 MG1655
<220> torc b0997
<222> 1074-1173
<223> torc b0997 U00096 1057307..1058479
<313> 1316: 1149-1168
<400> 1316
TCTCGATAAA CGTGAAGAAC GCACCTTGTT GAAATATCTG CAAATGAATG 50
CGTCTGACAC CGCAGGTAAG GCTCACGGCG ATAAGAAGGA AGAAAAATAA 100
<210> 1317
<211> 100
<212> DNA
<213> Escherichia coli K-12 MG1655

<220> torc b0997
<222> 1053-1152
<223> torc b0997 U00096 1057307..1058479
<313> 1317: 1101-1120
<400> 1317
CGGCATGATT GGCTTTACCA GTCTCGATAA ACGTGAAGAA CGCACCTTGT  50
TGAAATATCT GCAAATGAAT GCGTCTGACA CCGCAGGTAA GGCTCACGGC  100
<210> 1318
<211> 100
<212> DNA
<213> Escherichia coli K-12 MG1655
<220> torc b0997
<222> 1014-1113
<223> torc b0997 U00096 1057307..1058479
<313> 1318: 1071-1090
<400> 1318
CGCCCACTTT GACGCTAACG GTTGGATCGG CACGCTCAAC GGCATGATTG  50
GCTTTACCAG TCTCGATAAA CGTGAAGAAC GCACCTTGTT GAAATATCTG  100
<210> 1319
<211> 100
<212> DNA
<213> Escherichia coli K-12 MG1655
<220> tord b0999
<222> 487-586
<223> tord b0999 U00096 1061022..1061621
<313> 1319: 536-555
<400> 1319
CAATGGTTAC CAGAGTTTGT TGCGCGTTGT CGTCAGTATG ACAGCTTTGG  50
TTTTTACGCG GCACTAAGCC AGTTATTGCT GGTGTTAGTG GAGTGCGACC  100
<210> 1320
<211> 100
<212> DNA
<213> Escherichia coli K-12 MG1655
<220> tord b0999
<222> 442-541
<223> tord b0999 U00096 1061022..1061621
<313> 1320: 495-514
<400> 1320
CGAAGAATCG ACAGTTTGCG GCAAAAAACA CTGACGGCGC TGTGGCAATG  50
GTTACCAGAG TTTGTTGCGC GTTGTCGTCA GTATGACAGC TTTGGTTTTT  100
<210> 1321
<211> 100
<212> DNA
<213> Escherichia coli K-12 MG1655
<220> tord b0999
<222> 414-513
<223> tord b0999 U00096 1061022..1061621
<313> 1321: 456-475
<400> 1321
TTCGCTGGGA GAGGGGACCG TTCCTGCGCG AAGAATCGAC AGTTTGCGGC  50
AAAAAACACT GACGGCGCTG TGGCAATGGT TACCAGAGTT TGTTGCGCGT  100
<210> 1322
<211> 100
<212> DNA
<213> Escherichia coli K-12 MG1655
<220> frvr b3898
<222> 1642-1741
<223> frvr b3898 U00096 complement(4085688..4087436)
<313> 1322: 1696-1715
<400> 1322
CTAAAAATAT TTAGCTATCT GGCAAGCATA TTGTGTCAGC ATCCGGCGGA  50
GATTATTGCC GGGTTAACAG GATATGAGGC ATTTATGGAG TTACTTCACA  100
<210> 1323
<211> 100
<212> DNA
<213> Escherichia coli K-12 MG1655
<220> frvr b3898
<222> 1550-1649
<223> frvr b3898 U00096 complement(4085688..4087436)
<313> 1323: 1605-1624
<400> 1323

TTTTTATTAC CCTCGCCCAA CCAGTTGAGG TGAATAACGA AGTCATTAAC 50
CATGTCTTGA TCGCCTGCGC CGCCGCCGAT GCGCGTCATG AGCTAAAAAT 100
<210> 1324
<211> 100
<212> DNA
<213> Escherichia coli K-12 MG1655
<220> frvr b3898
<222> 1523-1622
<223> frvr b3898 U00096 complement(4085688..4087436)
<313> 1324: 1542-1561
<400> 1324
GCGAACAGGA GCGACGTTTT CGTGGATTTT TTATTACCCT CGCCCAACCA 50
GTTGAGGTGA ATAACGAAGT CATTAACCAT GTCTTGATCG CCTGCGCCGC 100
<210> 1325
<211> 100
<212> DNA
<213> Escherichia coli K-12 MG1655
<220> frvx b3899
<222> 967-1066
<223> frvx b3899 U00096 complement(4087436..4088506)
<313> 1325: 994-1013
<400> 1325
TCAAAAGCCG ATTACGAAGC TCTGCTCACG CTGATACGGG GTTTTCTGAC 50
GACCTTAACT GCGGAGAAAG TCAACGCGTT TAGCCAGTTC CGTCAGGTGG 100
<210> 1326
<211> 100
<212> DNA
<213> Escherichia coli K-12 MG1655
<220> frvx b3899
<222> 928-1027
<223> frvx b3899 U00096 complement(4087436..4088506)
<313> 1326: 958-977
<400> 1326
CTGCCAACTC GTTATCTGCA CGCCAACAGC GGTATGATTT CAAAAGCCGA 50
TTACGAAGCT CTGCTCACGC TGATACGGGG TTTTCTGACG ACCTTAACTG 100
<210> 1327
<211> 100
<212> DNA
<213> Escherichia coli K-12 MG1655
<220> frvx b3899
<222> 874-973
<223> frvx b3899 U00096 complement(4087436..4088506)
<313> 1327: 908-927
<400> 1327
GATGGCGGGC GCTACAACGT GATGGGCGGC GGGCGTCCGG TTGTCGCGCT 50
GTGTCTGCCA ACTCGTTATC TGCACGCCAA CAGCGGTATG ATTTCAAAAG 100
<210> 1328
<211> 100
<212> DNA
<213> Escherichia coli K-12 MG1655
<220> torr b0996
<222> 594-693
<223> torr b0996 U00096 complement(1056485..1057177)
<313> 1328: 660-679
<400> 1328
CGTCGATGTG TTAATTCGTC GTTTACGTCA TAAACTCAGC GCGGATTTAC 50
TGGTGACGCA ACATGGTGAA GGTTATTTCT TAGCCGCTGA TGTGTGCTGA 100
<210> 1329
<211> 100
<212> DNA
<213> Escherichia coli K-12 MG1655
<220> torr b0996
<222> 577-676
<223> torr b0996 U00096 complement(1056485..1057177)
<313> 1329: 614-633
<400> 1329
AACCCTGACC TGCGCACCGT CGATGTGTTA ATTCGTCGTT TACGTCATAA 50
ACTCAGCGCG GATTTACTGG TGACGCAACA TGGTGAAGGT TATTTCTTAG 100
<210> 1330
<211> 100
<212> DNA

<213> Escherichia coli K-12 MG1655
<220> torr b0996
<222> 494-593
<223> torr b0996 U00096 complement(1056485..1057177)
<313> 1330: 544-563
<400> 1330
TGTTGGTGGC ATTTGTGACG AATCCGGGCG AAATTCTCAG CCGTGAACGT 50
CTGCTACGTA TGCTTTCTGC GCGTCGGGTG GAAAACCCTG ACCTGCGCAC 100
<210> 1331
<211> 100
<212> DNA
<213> Escherichia coli K-12 MG1655
<220> tors b0994
<222> 2616-2715
<223> tors b0994 U00096 complement(1052657..1055371)
<313> 1331: 2687-2706
<400> 1331
GCAGCAGCCA TTATCTGCCC CCCTTCCACA CGAAGAAATT ACACGCAGTG 50
TTGCCGCTCT GGAAGCATGG TTGCATAAGA AAGACCTGAA CGCGATTTGA 100
<210> 1332
<211> 100
<212> DNA
<213> Escherichia coli K-12 MG1655
<220> tors b0994
<222> 2600-2699
<223> tors b0994 U00096 complement(1052657..1055371)
<313> 1332: 2646-2665
<400> 1332
TATGCGCACA ACTGGAGCAG CAGCCATTAT CTGCCCCCCT TCCACACGAA 50
GAAATTACAC GCAGTGTTGC CGCTCTGGAA GCATGGTTGC ATAAGAAAGA 100
<210> 1333
<211> 100
<212> DNA
<213> Escherichia coli K-12 MG1655
<220> tors b0994
<222> 2490-2589
<223> tors b0994 U00096 complement(1052657..1055371)
<313> 1333: 2546-2565
<400> 1333
CGATGAAATC GACATTGCCC GCGCCAGTCA GGATAGCGAG AAAATAAAGC 50
GTGCCGCGCA TCAGCTAAAA AGCAGTTGCT CGAGTCTGGG AATGCATATC 100
<210> 1334
<211> 100
<212> DNA
<213> Escherichia coli K-12 MG1655
<220> tort b0995
<222> 930-1029
<223> tort b0995 U00096 1055484..1056512
<313> 1334: 987-1006
<400> 1334
GCCGAAAAAT GCCGACCGTG AACATATTCG CCGCTCGCTG TCACCAGGGG 50
GATTTCGTCC GGTCTATTTT TATCAGCACA CATCAGCGGC TAAGAAATAA 100
<210> 1335
<211> 100
<212> DNA
<213> Escherichia coli K-12 MG1655
<220> tort b0995
<222> 927-1026
<223> tort b0995 U00096 1055484..1056512
<313> 1335: 960-979
<400> 1335
GACGCCGAAA AATGCCGACC GTGAACATAT TCGCCGCTCG CTGTCACCAG 50
GGGGATTTCG TCCGGTCTAT TTTTATCAGC ACACATCAGC GGCTAAGAAA 100
<210> 1336
<211> 100
<212> DNA
<213> Escherichia coli K-12 MG1655
<220> tort b0995
<222> 861-960
<223> tort b0995 U00096 1055484..1056512
<313> 1336: 921-940

<400> 1336
GCAGGCCATC AGGCAATTAC AGGGGCAATC GGTTTCTGAT AATGTCAGCC 50
CACCGATTTT AGTTCTGACG CCGAAAAATG CCGACCGTGA ACATATTCGC 100
<210> 1337
<211> 100
<212> DNA
<213> Escherichia coli K-12 MG1655
<220> nth b1634
<222> 504-603
<223> nth b1634 U00096 1709547..1710182
<313> 1337: 547-566
<400> 1337
AGCAGAGTTT AAAGTCGACT GCCACCATTG GTTGATCCTG CACGGGCGTT 50
ATACCTGCAT TGCCCGCAAG CCCCGCTGTG GCTCTTGTAT TATTGAAGAT 100
<210> 1338
<211> 100
<212> DNA
<213> Escherichia coli K-12 MG1655
<220> nth b1634
<222> 451-550
<223> nth b1634 U00096 1709547..1710182
<313> 1338: 477-496
<400> 1338
GCGCCGGGGA AAAACGTCGA ACAGGTAGAA GAAAAGCTAC TGAAAGTGGT 50
TCCAGCAGAG TTTAAAGTCG ACTGCCACCA TTGGTTGATC CTGCACGGGC 100
<210> 1339
<211> 100
<212> DNA
<213> Escherichia coli K-12 MG1655
<220> nth b1634
<222> 399-498
<223> nth b1634 U00096 1709547..1710182
<313> 1339: 428-447
<400> 1339
GACTATTGCT GTCGACACGC ACATTTTCCG CGTTTGTAAT CGTACTCAAT 50
TTGCGCCGGG GAAAAACGTC GAACAGGTAG AAGAAAAGCT ACTGAAAGTG 100
<210> 1340
<211> 100
<212> DNA
<213> Escherichia coli K-12 MG1655
<220> acea b4016
<222> 1206-1305
<223> acea b4016 U00096 4214688..4215992
<313> 1340: 1283-1302
<400> 1340
AGTGGGTACA GGTTACTTCG ATAAAGTGAC GACTATTATT CAGGGCGGCA 50
CGTCTTCAGT CACCGCGCTG ACCGGCTCCA CTGAAGAATC GCAGTTCTAA 100
<210> 1341
<211> 100
<212> DNA
<213> Escherichia coli K-12 MG1655
<220> acea b4016
<222> 1182-1281
<223> acea b4016 U00096 4214688..4215992
<313> 1341: 1233-1252
<400> 1341
TACCTTCGTA TCTCACCAGC AGGAAGTGGG TACAGGTTAC TTCGATAAAG 50
TGACGACTAT TATTCAGGGC GGCACGTCTT CAGTCACCGC GCTGACCGGC 100
<210> 1342
<211> 100
<212> DNA
<213> Escherichia coli K-12 MG1655
<220> acea b4016
<222> 1169-1268
<223> acea b4016 U00096 4214688..4215992
<313> 1342: 1199-1218
<400> 1342
CGAAAGATGG CTATACCTTC GTATCTCACC AGCAGGAAGT GGGTACAGGT 50
TACTTCGATA AAGTGACGAC TATTATTCAG GGCGGCACGT CTTCAGTCAC 100
<210> 1343
<211> 100

<212> DNA
<213> Escherichia coli K-12 MG1655
<220> aceb b4015
<222> 1503-1602
<223> aceb b4015 U00096 4213057..4214658
<313> 1343: 1572-1591
<400> 1343
GGGGCGTTTT GACGATGCCG CACGCTTGAT GGAACAGATC ACCACTTCCG 50
ATGAGTTAAT TGATTTCCTG ACCCTGCCAG GCTACCGCCT GTTAGCGTAA 100
<210> 1344
<211> 100
<212> DNA
<213> Escherichia coli K-12 MG1655
<220> aceb b4015
<222> 1499-1598
<223> aceb b4015 U00096 4213057..4214658
<313> 1344: 1527-1546
<400> 1344
CCCAGGGGCG TTTTGACGAT GCCGCACGCT TGATGGAACA GATCACCACT 50
TCCGATGAGT TAATTGATTT CCTGACCCTG CCAGGCTACC GCCTGTTAGC 100
<210> 1345
<211> 100
<212> DNA
<213> Escherichia coli K-12 MG1655
<220> aceb b4015
<222> 1406-1505
<223> aceb b4015 U00096 4213057..4214658
<313> 1345: 1456-1475
<400> 1345
GCAATGGCAA ACCGGTGACC AAAGCCTTGT TCCGCCAGAT GCTGGGCGAA 50
GAGATGAAAG TCATTGCCAG CGAACTGGGC GAAGAACGTT TCTCCCAGGG 100
<210> 1346
<211> 100
<212> DNA
<213> Escherichia coli K-12 MG1655
<220> gcd b0125
<222> 2292-2391
<223> gcd b0125 U00096 complement(138835..141225)
<313> 1346: 2358-2377
<400> 1346
TAAGCAGTAT GTGGTGATCT CCGCAGGCGG TCACGGTTCA TTTGGTACGA 50
AGATGGGCGA CTATATTGTG GCTTATGCGC TGCCGGATGA TGTGAAGTAA 100
<210> 1347
<211> 100
<212> DNA
<213> Escherichia coli K-12 MG1655
<220> gcd b0125
<222> 2292-2391
<223> gcd b0125 U00096 complement(138835..141225)
<313> 1347: 2321-2340
<400> 1347
TAAGCAGTAT GTGGTGATCT CCGCAGGCGG TCACGGTTCA TTTGGTACGA 50
AGATGGGCGA CTATATTGTG GCTTATGCGC TGCCGGATGA TGTGAAGTAA 100
<210> 1348
<211> 100
<212> DNA
<213> Escherichia coli K-12 MG1655
<220> gcd b0125
<222> 2226-2325
<223> gcd b0125 U00096 complement(138835..141225)
<313> 1348: 2262-2281
<400> 1348
AAAACTGTGG CAGGGTCGTT TACCAGCGGG TGGTCAGGCT ACGCCAATGA 50
CCTATGAAGT GAATGGTAAG CAGTATGTGG TGATCTCCGC AGGCGGTCAC 100
<210> 1349
<211> 100
<212> DNA
<213> Escherichia coli K-12 MG1655
<220> acee b0115
<222> 2551-2650
<223> acee b0115 U00096 123017..125680

<313> 1349: 2573-2592
<400> 1349
GTTGCGGCGC TGGGCGAACT GGCTAAACGT GGCGAAATCG ATAAGAAAGT 50
GGTTGCTGAC GCAATCGCCA AATTCAACAT CGATGCAGAT AAAGTTAACC 100
<210> 1350
<211> 100
<212> DNA
<213> Escherichia coli K-12 MG1655
<220> acee b0115
<222> 2463-2562
<223> acee b0115 U00096 123017..125680
<313> 1350: 2500-2519
<400> 1350
CCGCGTACTG GGTACTGATG GCTTCGGTCG TTCCGACAGC CGTGAGAACC 50
TGCGTCACCA CTTCGAAGTT GATGCTTCTT ATGTCGTGGT TGCGGCGCTG 100
<210> 1351
<211> 100
<212> DNA
<213> Escherichia coli K-12 MG1655
<220> acee b0115
<222> 2424-2523
<223> acee b0115 U00096 123017..125680
<313> 1351: 2468-2487
<400> 1351
CGCTGAGCAG GTCCGTACTT ACGTACCGGC TGACGACTAC CGCGTACTGG 50
GTACTGATGG CTTCGGTCGT TCCGACAGCC GTGAGAACCT GCGTCACCAC 100
<210> 1352
<211> 100
<212> DNA
<213> Escherichia coli K-12 MG1655
<220> acef b0116
<222> 1794-1893
<223> acef b0116 U00096 125695..127587
<313> 1352: 1855-1874
<400> 1352
TCTCTCCTTC GACCACCGCG TGATCGACGG TGCTGATGGT GCCCGTTTCA 50
TTACCATCAT TAACAACACG CTGTCTGACA TTCGCCGTCT GGTGATGTAA 100
<210> 1353
<211> 100
<212> DNA
<213> Escherichia coli K-12 MG1655
<220> acef b0116
<222> 1742-1841
<223> acef b0116 U00096 125695..127587
<313> 1353: 1803-1822
<400> 1353
AGCCGGTGTG GAATGGTAAA GAGTTCGTGC CGCGTCTGAT GCTGCCGATT 50
TCTCTCTCCT TCGACCACCG CGTGATCGAC GGTGCTGATG GTGCCCGTTT 100
<210> 1354
<211> 100
<212> DNA
<213> Escherichia coli K-12 MG1655
<220> acef b0116
<222> 1731-1830
<223> acef b0116 U00096 125695..127587
<313> 1354: 1753-1772
<400> 1354
GTCCGCGATG GAGCCGGTGT GGAATGGTAA AGAGTTCGTG CCGCGTCTGA 50
TGCTGCCGAT TTCTCTCTCC TTCGACCACC GCGTGATCGA CGGTGCTGAT 100
<210> 1355
<211> 100
<212> DNA
<213> Escherichia coli K-12 MG1655
<220> eutb eutB
<222> 1305-1404
<223> eutb eutB U00096 complement(2555338..2556741)
<313> 1355: 1384-1403
<400> 1355
GCGCCCGTCA CCGGAGTTTG AACGCTGGCT GGAAAGCATG GGCATTATGG 50
CAAACGGTCG CCTGACCAAA CGGGCGGGCG ATCCGTCACT GTTCTTCTGA 100
<210> 1356

<211> 100
<212> DNA
<213> Escherichia coli K-12 MG1655
<220> eutb eutB
<222> 1273-1372
<223> eutb eutB U00096 complement(2555338..2556741)
<313> 1356: 1299-1318
<400> 1356
GATACCGCCA CTGTGCGTCA GTTACTCAAC CTGCGCCCGT CACCGGAGTT 50
TGAACGCTGG CTGGAAAGCA TGGGCATTAT GGCAAACGGT CGCCTGACCA 100
<210> 1357
<211> 100
<212> DNA
<213> Escherichia coli K-12 MG1655
<220> eutb eutB
<222> 1230-1329
<223> eutb eutB U00096 complement(2555338..2556741)
<313> 1357: 1263-1282
<400> 1357
GGGTGATGAC ATCATGCTCA ACTACCAGAC CACCGCATTC CACGATACCG 50
CCACTGTGCG TCAGTTACTC AACCTGCGCC CGTCACCGGA GTTTGAACGC 100
<210> 1358
<211> 100
<212> DNA
<213> Escherichia coli K-12 MG1655
<220> frwb b3951
<222> 222-321
<223> frwb b3951 U00096 4141203..4141523
<313> 1358: 283-302
<400> 1358
TGCTGGCAAG ACCATCGTGC GCGTCAACAT CAGCGATGCA GTAAAACGTG 50
CTGACGCCAT TATGAGCAAA ATTGAAGCTC ACCTGGCGCA AACCGCTTAA 100
<210> 1359
<211> 100
<212> DNA
<213> Escherichia coli K-12 MG1655
<220> frwb b3951
<222> 92-191
<223> frwb b3951 U00096 4141203..4141523
<313> 1359: 150-169
<400> 1359
AAGGCTGGGA AGTGAAAGTA GAAACCCAGG GGTCAATTGG TCTGGAAAAT 50
GAACTGACTG CGGAAGATGT GGCGAGCGCT GATATGGTGA TTCTGACCAA 100
<210> 1360
<211> 100
<212> DNA
<213> Escherichia coli K-12 MG1655
<220> frwb b3951
<222> 60-159
<223> frwb b3951 U00096 4141203..4141523
<313> 1360: 107-126
<400> 1360
AGCAGAGGCG CTGGAAAGTG CCGCGAAAGC GAAAGGCTGG GAAGTGAAAG 50
TAGAAACCCA GGGGTCAATT GGTCTGGAAA ATGAACTGAC TGCGGAAGAT 100
<210> 1361
<211> 100
<212> DNA
<213> Escherichia coli K-12 MG1655
<220> eutc b2441
<222> 789-888
<223> eutc b2441 U00096 complement(2554430..2555317)
<313> 1361: 845-864
<400> 1361
CCAGGGCGGC ACGCCGCCGG TTGAAGCCGC AGCCGTCATT GTGGATTTGG 50
CGAAACGTAT GCTGGAGCAG AAAGCATCCG GCATCAACAT GACCCGATAA 100
<210> 1362
<211> 100
<212> DNA
<213> Escherichia coli K-12 MG1655
<220> eutc b2441
<222> 659-758

<223> eutc b2441 U00096 complement(2554430..2555317)
<313> 1362: 722-741
<400> 1362
AAGTAGTGAT CCTGCTGGTG GGCGAACGTC CGGGGTTGGG TCAGTCAGAA 50
AGCCTCTCCT GCTACGCCGT TTACTCGCCG CGTATGGCGA CCACCGTTGA 100
<210> 1363
<211> 100
<212> DNA
<213> Escherichia coli K-12 MG1655
<220> eutc b2441
<222> 604-703
<223> eutc b2441 U00096 complement(2554430..2555317)
<313> 1363: 637-656
<400> 1363
GTTCGTTATG GTCGCGTGAA GATTGAAGAT CAGATCGGTG AGATCCTCGG 50
CGCGAAAGTA GTGATCCTGC TGGTGGGCGA ACGTCCGGGG TTGGGTCAGT 100
<210> 1364
<211> 100
<212> DNA
<213> Escherichia coli K-12 MG1655
<220> frwc b3950
<222> 960-1059
<223> frwc b3950 U00096 4140109..4141188
<313> 1364: 1016-1035
<400> 1364
GGCTGTCGGA GCAGTGGTGA CGGCTGTTTG TGTGAACGTG CTGAAAAGTC 50
TGGCGCGTAA AAATGGGTCT TCGACTGATG AAAAAGAAGA CGACCTGGAT 100
<210> 1365
<211> 100
<212> DNA
<213> Escherichia coli K-12 MG1655
<220> frwc b3950
<222> 914-1013
<223> frwc b3950 U00096 4140109..4141188
<313> 1365: 939-958
<400> 1365
TTGTGCTGCC GGTGGTTGAA GGCAAGCTGG GTTATATCGC AGCAGTGGCT 50
GTCGGAGCAG TGGTGACGGC TGTTTGTGTG AACGTGCTGA AAAGTCTGGC 100
<210> 1366
<211> 100
<212> DNA
<213> Escherichia coli K-12 MG1655
<220> frwc b3950
<222> 781-880
<223> frwc b3950 U00096 4140109..4141188
<313> 1366: 815-834
<400> 1366
ACTGAAGGGG CGATTCCTTT CGCCGCTGCC GATCCGCTGC GTGTCATTCC 50
TTCCATCATG GTCGGTTCAG TTTGTGGTGC AGTAACTGCG GCGCTGGTCG 100
<210> 1367
<211> 100
<212> DNA
<213> Escherichia coli K-12 MG1655
<220> ogrk ogrK
<222> 120-219
<223> ogrk ogrK U00096 complement(2165324..2165542)
<313> 1367: 160-179
<400> 1367
CACTTATGAG TCGGTACAGC GATACATTGT GAAGCCGGGA GAAGTCCACG 50
TCGTAAGGCC GCACCCGTTG CCGTCAGGGC AGCAAATTAT GTGGATGTAA 100
<210> 1368
<211> 100
<212> DNA
<213> Escherichia coli K-12 MG1655
<220> ogrk ogrK
<222> 99-198
<223> ogrk ogrK U00096 complement(2165324..2165542)
<313> 1368: 165-184
<400> 1368
GAATTGCAGC GCCACGTTCA TCACTTATGA GTCGGTACAG CGATACATTG 50
TGAAGCCGGG AGAAGTCCAC GTCGTAAGGC CGCACCCGTT GCCGTCAGGG 100

<210> 1369
<211> 100
<212> DNA
<213> Escherichia coli K-12 MG1655
<220> ogrk ogrK
<222> 110-209
<223> ogrk ogrK U00096 complement(2165324..2165542)
<313> 1369: 155-174
<400> 1369
CCACGTTCAT CACTTATGAG TCGGTACAGC GATACATTGT GAAGCCGGGA 50
GAAGTCCACG TCGTAAGGCC GCACCCGTTG CCGTCAGGGC AGCAAATTAT 100
<210> 1370
<211> 100
<212> DNA
<213> Escherichia coli K-12 MG1655
<220> frwd b3954
<222> 217-316
<223> frwd b3954 U00096 4144717..4145058
<313> 1370: 263-282
<400> 1370
CGATTTGAAC ATTGCCGCTA TGTGCAATGC AGCATCTACG CATTCCTGCG 50
TGAGCCGCAG CGGGTAATGA GCGCGGTGCG CAAAGTGCTT TCTGCGCCGC 100
<210> 1371
<211> 100
<212> DNA
<213> Escherichia coli K-12 MG1655
<220> frwd b3954
<222> 73-172
<223> frwd b3954 U00096 4144717..4145058
<313> 1371: 104-123
<400> 1371
GAAAAGTTGT GCCTGTTAGA GAAGTGGGGA GTCAGCATTG AAACTCAGGG 50
CGCGCTGGGA ACGGAGAATC GTTTAGCGGA TGAGGATATC CGCCGGGCGG 100
<210> 1372
<211> 100
<212> DNA
<213> Escherichia coli K-12 MG1655
<220> frwd b3954
<222> 9-108
<223> frwd b3954 U00096 4144717..4145058
<313> 1372: 54-73
<400> 1372
CCTGGTGGCA GTAACCGCCT GCGTAAGCGG CGTGGCGCAT ACTTATATGG 50
CGGCGGAACG GCTGGAAAAG TTGTGCCTGT TAGAGAAGTG GGGAGTCAGC 100
<210> 1373
<211> 100
<212> DNA
<213> Escherichia coli K-12 MG1655
<220> eute b2456
<222> 1249-1348
<223> eute b2456 U00096 complement(2568368..2569771)
<313> 1373: 1305-1324
<400> 1373
GTTAAGAACG GACCGTGCAT TGCCGGGCTG GGGCTGGGCG GGGAAGGCTG 50
GACCACCATG ACCATCACCA CGCCAACCGG TGAAGGGGTA ACCAGCGCGC 100
<210> 1374
<211> 100
<212> DNA
<213> Escherichia coli K-12 MG1655
<220> eute b2456
<222> 1204-1303
<223> eute b2456 U00096 complement(2568368..2569771)
<313> 1374: 1261-1280
<400> 1374
GAAAACATGA ACCAGATGGC GAATGCTATT GATACCAGCA TTTTCGTTAA 50
GAACGGACCG TGCATTGCCG GGCTGGGGCT GGGCGGGGAA GGCTGGACCA 100
<210> 1375
<211> 100
<212> DNA
<213> Escherichia coli K-12 MG1655
<220> eute b2456

<222> 1175-1274
<223> eute b2456 U00096 complement(2568368..2569771)
<313> 1375: 1208-1227
<400> 1375
ACACGGCGGC AATGCACTCG CGCAACATCG AAAACATGAA CCAGATGGCG  50
AATGCTATTG ATACCAGCAT TTTCGTTAAG AACGGACCGT GCATTGCCGG  100
<210> 1376
<211> 100
<212> DNA
<213> Escherichia coli K-12 MG1655
<220> acek b4017
<222> 1623-1722
<223> acek b4017 U00096 4216175..4217911
<313> 1376: 1651-1670
<400> 1376
CGCTGATTAC TGGCGCGCAC TACAAAACCG CATACGTGAA GGGCATGTGG  50
AAGATGTTTA TGCGTATCGG CGCAGGCAAA GATTTAGCGT ACGGTATGGG  100
<210> 1377
<211> 100
<212> DNA
<213> Escherichia coli K-12 MG1655
<220> acek b4017
<222> 1558-1657
<223> acek b4017 U00096 4216175..4217911
<313> 1377: 1597-1616
<400> 1377
CACTGGCTAT GCGCCGACCC GCGTATTGGT CCGCTGTTTG AAGAGATGCA  50
CGCCGACCTG TTCCGCGCTG ATTACTGGCG CGCACTACAA AACCGCATAC  100
<210> 1378
<211> 100
<212> DNA
<213> Escherichia coli K-12 MG1655
<220> acek b4017
<222> 1512-1611
<223> acek b4017 U00096 4216175..4217911
<313> 1378: 1565-1584
<400> 1378
GTGGTACAGC GTCTCGCCGG GCGATGTTTT CCCGGAAGAG TTTCGCCACT  50
GGCTATGCGC CGACCCGCGT ATTGGTCCGC TGTTTGAAGA GATGCACGCC  100
<210> 1379
<211> 100
<212> DNA
<213> Escherichia coli K-12 MG1655
<220> eutg b2454
<222> 1116-1215
<223> eutg b2454 U00096 complement(2566344..2567558)
<313> 1379: 1188-1207
<400> 1379
CGCATGGGCG CAGGCCGCGC TGGAAGATAT TTGTCTGCGC AGTAACCCGC  50
GTACCGCCAG CCTGGAGCAG ATTGTCGGCC TGTACGCAGC GGCGCAATAA  100
<210> 1380
<211> 100
<212> DNA
<213> Escherichia coli K-12 MG1655
<220> eutg b2454
<222> 1020-1119
<223> eutg b2454 U00096 complement(2566344..2567558)
<313> 1380: 1080-1099
<400> 1380
CGATCGTGAC GCTATTAACG CGGTAAGTGA GCTGATTGCG GAAGTTGGGA  50
TTGGTAAACG ACTGGGCGAT GTTGGTGCGA CATCTGCGCA TTACGGCGCA  100
<210> 1381
<211> 100
<212> DNA
<213> Escherichia coli K-12 MG1655
<220> eutg b2454
<222> 959-1058
<223> eutg b2454 U00096 complement(2566344..2567558)
<313> 1381: 988-1007
<400> 1381
GGATGGTTTG TCGTGAACGC TTTAGTCAGA TTGGTCGGGC ACTGCGAACT  50

AAAAAATCCG ACGATCGTGA CGCTATTAAC GCGGTAAGTG AGCTGATTGC 100
<210> 1382
<211> 100
<212> DNA
<213> Escherichia coli K-12 MG1655
<220> gcl b0508
<222> 1683-1782
<223> gcl b0508 U00096 533140..534921
<313> 1382: 1750-1769
<400> 1382
TTCGATGGGC AGCGAACTGG ATAACGTCAT GGAATTTGAA GATATCGCCG 50
ATAACGCAGC GGACGCACCG ACTGAAACCT GCTTCATGCA CTATGAATAA 100
<210> 1383
<211> 100
<212> DNA
<213> Escherichia coli K-12 MG1655
<220> gcl b0508
<222> 1678-1777
<223> gcl b0508 U00096 533140..534921
<313> 1383: 1698-1717
<400> 1383
AATATTTCGA TGGGCAGCGA ACTGGATAAC GTCATGGAAT TTGAAGATAT 50
CGCCGATAAC GCAGCGGACG CACCGACTGA AACCTGCTTC ATGCACTATG 100
<210> 1384
<211> 100
<212> DNA
<213> Escherichia coli K-12 MG1655
<220> gcl b0508
<222> 1592-1691
<223> gcl b0508 U00096 533140..534921
<313> 1384: 1621-1640
<400> 1384
TTGCGCCAGC CTTTGAACAG GCGAAAGCCT TAATGGCGCA ATATCGGGTA 50
CCGGTAGTCG TGGAAGTTAT TCTCGAGCGT GTGACCAATA TTTCGATGGG 100
<210> 1385
<211> 100
<212> DNA
<213> Escherichia coli K-12 MG1655
<220> euth b2453
<222> 1128-1227
<223> euth b2453 U00096 complement(2564901..2566127)
<313> 1385: 1191-1210
<400> 1385
CAAGTTGATC GGCGGCGTAA CGGCGATTGG CGTGGCGATG ATGCTGGTGC 50
CAAAAGAAGA CGCGACCGCG ACTAAAACCG AAGCGGAGGC ACAATCGTGA 100
<210> 1386
<211> 100
<212> DNA
<213> Escherichia coli K-12 MG1655
<220> euth b2453
<222> 1120-1219
<223> euth b2453 U00096 complement(2564901..2566127)
<313> 1386: 1147-1166
<400> 1386
ATTGTCGGCA AGTTGATCGG CGGCGTAACG GCGATTGGCG TGGCGATGAT 50
GCTGGTGCCA AAAGAAGACG CGACCGCGAC TAAAACCGAA GCGGAGGCAC 100
<210> 1387
<211> 100
<212> DNA
<213> Escherichia coli K-12 MG1655
<220> euth b2453
<222> 1043-1142
<223> euth b2453 U00096 complement(2564901..2566127)
<313> 1387: 1086-1105
<400> 1387
CCGTTTCCGC TGCTTTCGCC CTGGGCGACC ACTTAGGCTT CGCCGCTGCC 50
AACATGAACG CCATGATCTT CCCGATGATT GTCGGCAAGT TGATCGGCGG 100
<210> 1388
<211> 100
<212> DNA
<213> Escherichia coli K-12 MG1655

<220> euti eutI
<222> 918-1017
<223> euti eutI U00096 complement(2570509..2571525)
<313> 1388: 992-1011
<400> 1388
TTGTAGCGTG CAGGAAATTA TCGAGCTGGC GCTGGTGGCA GCTGTGCCGC 50
GTCAGACAGA AGTGAACCGC GAAAGCAGTT TACAAACACT GGTTGAATGA 100
<210> 1389
<211> 100
<212> DNA
<213> Escherichia coli K-12 MG1655
<220> euti eutI
<222> 879-978
<223> euti eutI U00096 complement(2570509..2571525)
<313> 1389: 923-942
<400> 1389
ACAAGGACTT GCCGCGCCGA TGCACGATCT CTCTCGTGGT TGTAGCGTGC 50
AGGAAATTAT CGAGCTGGCG CTGGTGGCAG CTGTGCCGCG TCAGACAGAA 100
<210> 1390
<211> 100
<212> DNA
<213> Escherichia coli K-12 MG1655
<220> euti eutI
<222> 825-924
<223> euti eutI U00096 complement(2570509..2571525)
<313> 1390: 870-889
<400> 1390
TGGTTACAAA ATCGCACAAC GACTCGGCGG ATATCGTGCC GTCGGGCCAC 50
TGATACAAGG ACTTGCCGCG CCGATGCACG ATCTCTCTCG TGGTTGTAGC 100
<210> 1391
<211> 100
<212> DNA
<213> Escherichia coli K-12 MG1655
<220> eutj b2455
<222> 738-837
<223> eutj b2455 U00096 complement(2567521..2568357)
<313> 1391: 815-834
<400> 1391
GGCGTTACAG GTGCATTTAC CGCAGCACAG TTTGTTTATG ACCCCGCTGG 50
CGATCGCCAG TAGTGGGCGA GAGAAAGCGG AGGGGCTATA TGCAAAATGA 100
<210> 1392
<211> 100
<212> DNA
<213> Escherichia coli K-12 MG1655
<220> eutj b2455
<222> 732-831
<223> eutj b2455 U00096 complement(2567521..2568357)
<313> 1392: 766-785
<400> 1392
ATTCCCGGCG TTACAGGTGC ATTTACCGCA GCACAGTTTG TTTATGACCC 50
CGCTGGCGAT CGCCAGTAGT GGGCGAGAGA AAGCGGAGGG GCTATATGCA 100
<210> 1393
<211> 100
<212> DNA
<213> Escherichia coli K-12 MG1655
<220> eutj b2455
<222> 646-745
<223> eutj b2455 U00096 complement(2567521..2568357)
<313> 1393: 662-681
<400> 1393
CATATTGAAG GGCAGGGCAT TACTGATTTA TGGCTGGCGG GCGGCTCTTG 50
TATGCAACCG GGCGTGGCGG AACTGTTTCG CAAACAATTC CCGGCGTTAC 100
<210> 1394
<211> 100
<212> DNA
<213> Escherichia coli K-12 MG1655
<220> arga b2819
<222> 1233-1332
<223> arga b2819 U00096 2947264..2948595
<313> 1394: 1299-1318
<400> 1394

TGGATTTACC CCAGTGGATA TTGATTTACT GCCCGAGAGC AAAAAGCAGT 50
TGTACAACTA CCAGCGTAAA TCCAAAGTGT TGATGGCGGA TTTAGGGTAA 100
<210> 1395
<211> 100
<212> DNA
<213> Escherichia coli K-12 MG1655
<220> arga b2819
<222> 1177-1276
<223> arga b2819 U00096 2947264..2948595
<313> 1395: 1204-1223
<400> 1395
GGCTTAAGCA AATTGTTTGT GCTGACCACG CGCAGTATTC ACTGGTTCCA 50
GGAACGTGGA TTTACCCCAG TGGATATTGA TTTACTGCCC GAGAGCAAAA 100
<210> 1396
<211> 100
<212> DNA
<213> Escherichia coli K-12 MG1655
<220> arga b2819
<222> 1107-1206
<223> arga b2819 U00096 2947264..2948595
<313> 1396: 1148-1167
<400> 1396
CCCGGATTAC CGCAGTTCAT CAAGGGGTGA AGTTCTGCTG GAACGCATTG 50
CCGCTCAGGC GAAGCAGAGC GGCTTAAGCA AATTGTTTGT GCTGACCACG 100
<210> 1397
<211> 100
<212> DNA
<213> Escherichia coli K-12 MG1655
<220> yaaa b0007
<222> 678-777
<223> yaaa b0007 U00096 complement(5683..6459)
<313> 1397: 722-741
<400> 1397
ACCAGAGCAA CTGACTGGTT TTAATAGCGA AGGTTACTTC TTTGATGAAG 50
ATTCCTCCAG CAATGGCGAA CTGGTGTTTA AACGCTACGA GCAGCGTTAA 100
<210> 1398
<211> 100
<212> DNA
<213> Escherichia coli K-12 MG1655
<220> yaaa b0007
<222> 589-688
<223> yaaa b0007 U00096 complement(5683..6459)
<313> 1398: 630-649
<400> 1398
AAGAACGGCA AATTTAAGAT CATCAGCTTC TACGCTAAGA AAGCACGCGG 50
TCTGATGAGT CGTTTCATTA TTGAAAATCG GCTGACCAAA CCAGAGCAAC 100
<210> 1399
<211> 100
<212> DNA
<213> Escherichia coli K-12 MG1655
<220> yaaa b0007
<222> 525-624
<223> yaaa b0007 U00096 complement(5683..6459)
<313> 1399: 582-601
<400> 1399
TTTTAAATCG GTGAAGCCGA AGAAATTGAA TGCCGAGATT ATCAAGCCGG 50
TGTTCCTCGA TGAGAAGAAC GGCAAATTTA AGATCATCAG CTTCTACGCT 100
<210> 1400
<211> 100
<212> DNA
<213> Escherichia coli K-12 MG1655
<220> argb b3960
<222> 678-777
<223> argb b3960 U00096 4153592..4154368
<313> 1400: 725-744
<400> 1400
CACGCTGGGC CGTCCGGTAG ATATCGCCTC CTGGCGTCAT GCGGAGCAGC 50
TTCCGGCACT GTTTAACGGT ATGCCGATGG GTACGCGGAT TTTAGCTTAA 100
<210> 1401
<211> 100
<212> DNA

<213> Escherichia coli K-12 MG1655
<220> argb b3960
<222> 570-669
<223> argb b3960 U00096 4153592..4154368
<313> 1401: 624-643
<400> 1401
GCAACGCATT GCCGAAATGA CCGCCGCGAA AGCAGAACAA CTGATTGAGC 50
AGGGCATTAT TACTGACGGC ATGATAGTGA AAGTGAACGC GGCGCTGGAT 100
<210> 1402
<211> 100
<212> DNA
<213> Escherichia coli K-12 MG1655
<220> argb b3960
<222> 534-633
<223> argb b3960 U00096 4153592..4154368
<313> 1402: 574-593
<400> 1402
GCTCTCCGAC GTCAGCGGCA TTCTCGACGG CAAAGGGCAA CGCATTGCCG 50
AAATGACCGC CGCGAAAGCA GAACAACTGA TTGAGCAGGG CATTATTACT 100
<210> 1403
<211> 100
<212> DNA
<213> Escherichia coli K-12 MG1655
<220> held b0963
<222> 1956-2055
<223> held b0963 U00096 1023694..1025748
<313> 1403: 1988-2007
<400> 1403
GGCACGCCAT CGGGTATGGG CACTGTTTAA CAAAGAGAAT CCCTCTCCCT 50
TTGTGGAAAT ACTGAAAAAT CTGGATGTGC CGGTGGCGAG AAAACCGTAA 100
<210> 1404
<211> 100
<212> DNA
<213> Escherichia coli K-12 MG1655
<220> held b0963
<222> 1857-1956
<223> held b0963 U00096 1023694..1025748
<313> 1404: 1915-1934
<400> 1404
TGCGGCGCGG GAGTCGATTA TGGAAGAGGC GCTACTGCCA CCGGTTGAGG 50
ATTTCCCGGA CGCTGAAGAA CGGCGGTTAA TGTACGTGGC GCTGACCCGG 100
<210> 1405
<211> 100
<212> DNA
<213> Escherichia coli K-12 MG1655
<220> held b0963
<222> 1756-1855
<223> held b0963 U00096 1023694..1025748
<313> 1405: 1800-1819
<400> 1405
AAGTTGCAAA TCGACTTTAT GACCATTCAT GCCAGCAAAG GGCAACAGGC 50
GGATTACGTC ATCATCGTTG GCTTGCAGGA GGGAAGTGAT GGTTTTCCGG 100
<210> 1406
<211> 100
<212> DNA
<213> Escherichia coli K-12 MG1655
<220> argc b3959
<222> 906-1005
<223> argc b3959 U00096 4152580..4153584
<313> 1406: 963-982
<400> 1406
TGTGGCGACC GAAGACAACT TACTGAAAGG CGCGGCGGCA CAAGCGGTAC 50
AGTGCGCCAA TATTCGTTTC GGCTATGCGG AAACGCAGTC TCTTATTTAA 100
<210> 1407
<211> 100
<212> DNA
<213> Escherichia coli K-12 MG1655
<220> argc b3959
<222> 895-994
<223> argc b3959 U00096 4152580..4153584
<313> 1407: 918-937

<400> 1407
CATCTGATTA TTGTGGCGAC CGAAGACAAC TTACTGAAAG GCGCGGCGGC  50
ACAAGCGGTA CAGTGCGCCA ATATTCGTTT CGGCTATGCG GAAACGCAGT  100
<210> 1408
<211> 100
<212> DNA
<213> Escherichia coli K-12 MG1655
<220> argc b3959
<222> 718-817
<223> argc b3959 U00096 4152580..4153584
<313> 1408: 761-780
<400> 1408
CTCGAAACCA TTACCTGCCG CCTGAAATCG GGTGTGACCC AGGCGCAAGT  50
CGCGCAAGTG TTACAGCAGG CGTATGCCCA TAAACCGCTG GTGCGGCTGT  100
<210> 1409
<211> 100
<212> DNA
<213> Escherichia coli K-12 MG1655
<220> argd b3360
<222> 1088-1187
<223> argd b3360 U00096 complement(3486597..3487817)
<313> 1409: 1110-1129
<400> 1409
AGGCTGGCGT AATGGTGCTG AATGCCGGAC CGGATGTGAT GCGTTTTGCA  50
CCGTCGCTGG TGGTGGAAGA TGCGGATATC GATGAAGGGA TGCAACGTTT  100
<210> 1410
<211> 100
<212> DNA
<213> Escherichia coli K-12 MG1655
<220> argd b3360
<222> 1028-1127
<223> argd b3360 U00096 complement(3486597..3487817)
<313> 1410: 1044-1063
<400> 1410
GCGCAGAGCT GAAACCACAG TACAAAGGTC GGGCGCGTGA TTTCCTGTAT  50
GCGGGCGCAG AGGCTGGCGT AATGGTGCTG AATGCCGGAC CGGATGTGAT  100
<210> 1411
<211> 100
<212> DNA
<213> Escherichia coli K-12 MG1655
<220> argd b3360
<222> 945-1044
<223> argd b3360 U00096 complement(3486597..3487817)
<313> 1411: 988-1007
<400> 1411
CCAGCGTTTT GTTGACCATC TGCAGAAGAT CGATCAGCAG TACGATGTAT  50
TTAGCGATAT TCGCGGTATG GGGCTGTTGA TTGGCGCAGA GCTGAAACCA  100
<210> 1412
<211> 100
<212> DNA
<213> Escherichia coli K-12 MG1655
<220> arge b3958
<222> 1053-1152
<223> arge b3958 U00096 complement(4151275..4152426)
<313> 1412: 1115-1134
<400> 1412
TAATCAGGCT CATCAACCTG ATGAATATCT GGAAACACGG TTTATCAAGC  50
CCACCCGCGA ACTGATAACC CAGGTAATTC ACCATTTTTG CTGGCATTAA  100
<210> 1413
<211> 100
<212> DNA
<213> Escherichia coli K-12 MG1655
<220> arge b3958
<222> 948-1047
<223> arge b3958 U00096 complement(4151275..4152426)
<313> 1413: 1009-1028
<400> 1413
GGTTGAGAAA TTGCTCGGAG CAAAAACCGA AGTGGTGAAC TACTGTACCG  50
AAGCGCCGTT TATTCAAACG TTATGCCCGA CGCTGGTGTT GGGGCCTGGC  100
<210> 1414
<211> 100

<212> DNA
<213> Escherichia coli K-12 MG1655
<220> arge b3958
<222> 857-956
<223> arge b3958 U00096 complement(4151275..4152426)
<313> 1414: 911-930
<400> 1414
GCGAACGCTG GCCGGGTCGT CTGACGGTCG ACGAGCTGCA TCCGCCGATC 50
CCTGGCTATG AATGCCCACC GAATCATCAA CTGGTTGAAG TGGTTGAGAA 100
<210> 1415
<211> 100
<212> DNA
<213> Escherichia coli K-12 MG1655
<220> argf b0274
<222> 906-1005
<223> argf b0274 U00096 complement(288525..289529)
<313> 1415: 974-993
<400> 1415
GGACGAGGTG TTTGAGTCGG CGGCGAGCAT CGTGTTCGAC CAGGCGGAAA 50
ACCGGATGCA TACGATTAAG GCGGTGATGA TGGCAACGCT TGGGGAGTGA 100
<210> 1416
<211> 100
<212> DNA
<213> Escherichia coli K-12 MG1655
<220> argf b0274
<222> 861-960
<223> argf b0274 U00096 complement(288525..289529)
<313> 1416: 905-924
<400> 1416
GCAGATGGCG AAGGAGTTCG ATCTGCACGG CGGGATGGAG GTGACGGACG 50
AGGTGTTTGA GTCGGCGGCG AGCATCGTGT TCGACCAGGC GGAAAACCGG 100
<210> 1417
<211> 100
<212> DNA
<213> Escherichia coli K-12 MG1655
<220> argf b0274
<222> 819-918
<223> argf b0274 U00096 complement(288525..289529)
<313> 1417: 850-869
<400> 1417
CTGTCTGCCG GCGTTCCATG ACGACCAGAC TACGCTCGGC AAGCAGATGG 50
CGAAGGAGTT CGATCTGCAC GGCGGGATGG AGGTGACGGA CGAGGTGTTT 100
<210> 1418
<211> 100
<212> DNA
<213> Escherichia coli K-12 MG1655
<220> yaaf b0031
<222> 816-915
<223> yaaf b0031 U00096 27293..28207
<313> 1418: 878-897
<400> 1418
CGGTTGCCTG GGCAAGCCAG CCAATGTACA GGTGGCATTG GATCTGGATG 50
TGAAAGGCTT CCAGCAGTGG GTGGCTGAGG TGCTGGCTCT GGCGTCGTAA 100
<210> 1419
<211> 100
<212> DNA
<213> Escherichia coli K-12 MG1655
<220> yaaf b0031
<222> 762-861
<223> yaaf b0031 U00096 27293..28207
<313> 1419: 788-807
<400> 1419
GGCAGTGGAA ACTCAGGGCG AATTTACCTC AGGCACGACG GTGGTTGATA 50
TCGACGGTTG CCTGGGCAAG CCAGCCAATG TACAGGTGGC ATTGGATCTG 100
<210> 1420
<211> 100
<212> DNA
<213> Escherichia coli K-12 MG1655
<220> yaaf b0031
<222> 703-802
<223> yaaf b0031 U00096 27293..28207

<313> 1420: 738-757
<400> 1420
CTCTGCGCCA TCGCCTGGCT GGTGCGCCCG GACCTGTTCA CTCTCAAACC 50
CTGTTTTGTG GCAGTGGAAA CTCAGGGCGA ATTTACCTCA GGCACGACGG 100
<210> 1421
<211> 100
<212> DNA
<213> Escherichia coli K-12 MG1655
<220> argg b3173
<222> 1245-1344
<223> argg b3173 U00096 3316278..3317621
<313> 1421: 1306-1325
<400> 1421
CCGCGAGAAA CTTTTCGGTT ATGCCAAAAC TGGCCTGCTT TCCTCCTCTG 50
CCGCTTCAGG CGTGCCGCAG GTGGAGAATC TGGAAAACAA AGGCCAGTAA 100
<210> 1422
<211> 100
<212> DNA
<213> Escherichia coli K-12 MG1655
<220> argg b3173
<222> 1240-1339
<223> argg b3173 U00096 3316278..3317621
<313> 1422: 1267-1286
<400> 1422
GATACCCGCG AGAAACTTTT CGGTTATGCC AAAACTGGCC TGCTTTCCTC 50
CTCTGCCGCT TCAGGCGTGC CGCAGGTGGA GAATCTGGAA AACAAAGGCC 100
<210> 1423
<211> 100
<212> DNA
<213> Escherichia coli K-12 MG1655
<220> argg b3173
<222> 1197-1296
<223> argg b3173 U00096 3316278..3317621
<313> 1423: 1225-1244
<400> 1423
TGATCGTATT GGTCAATTGA CCATGCGTAA CCTGGATATC ACTGATACCC 50
GCGAGAAACT TTTCGGTTAT GCCAAAACTG GCCTGCTTTC CTCCTCTGCC 100
<210> 1424
<211> 100
<212> DNA
<213> Escherichia coli K-12 MG1655
<220> argh b3961
<222> 1275-1374
<223> argh b3961 U00096 4154429..4155802
<313> 1424: 1318-1337
<400> 1424
GTCGCTGCAA TCGTGCCTCG ACAAGCGTGC GGCAAAAGGC GGCGTCTCAC 50
CGCAGCAGGT GGCGCAGGCG ATTGCTTTTG CGCAGGCTCG GTTAGGGTAA 100
<210> 1425
<211> 100
<212> DNA
<213> Escherichia coli K-12 MG1655
<220> argh b3961
<222> 1217-1316
<223> argh b3961 U00096 4154429..4155802
<313> 1425: 1280-1299
<400> 1425
CGCTCAGTGA GTTGCAGAAA TTCAGTCAGG TGATTGACGA AGATGTCTAT 50
CCGATTCTGT CGCTGCAATC GTGCCTCGAC AAGCGTGCGG CAAAAGGCGG 100
<210> 1426
<211> 100
<212> DNA
<213> Escherichia coli K-12 MG1655
<220> argh b3961
<222> 1197-1296
<223> argh b3961 U00096 4154429..4155802
<313> 1426: 1216-1235
<400> 1426
CAAACCGCTG GAAGATCTGC CGCTCAGTGA GTTGCAGAAA TTCAGTCAGG 50
TGATTGACGA AGATGTCTAT CCGATTCTGT CGCTGCAATC GTGCCTCGAC 100
<210> 1427

<211> 100
<212> DNA
<213> Escherichia coli K-12 MG1655
<220> yaah yaaH
<222> 463-562
<223> yaah yaaH U00096 complement(9928..10494)
<313> 1427: 519-538
<400> 1427
TGGATTGGGC TGATCTGCGG TGCCAGCGCA ATCTATCTGG CGATGGGTGA 50
AGTACTGAAC GAGCAGTTTG GTCGCACCGT TCTGCCGATT GGTGAATCCC 100
<210> 1428
<211> 100
<212> DNA
<213> Escherichia coli K-12 MG1655
<220> yaah yaaH
<222> 331-430
<223> yaah yaaH U00096 complement(9928..10494)
<313> 1428: 387-406
<400> 1428
CTGTTTATGT TCTTCGGCAC GCTGAAAGGC GCACGCGTTC TGCAATTCGT 50
TTTCTTTAGC CTGACCGTGC TGTTTGCCCT GCTGGCGATC GGTAACATTG 100
<210> 1429
<211> 100
<212> DNA
<213> Escherichia coli K-12 MG1655
<220> yaah yaaH
<222> 291-390
<223> yaah yaaH U00096 complement(9928..10494)
<313> 1429: 341-360
<400> 1429
GTTCCTTGGT GTCTACCTGG GTCTGTGGGG CGTATTTACG CTGTTTATGT 50
TCTTCGGCAC GCTGAAAGGC GCACGCGTTC TGCAATTCGT TTTCTTTAGC 100
<210> 1430
<211> 100
<212> DNA
<213> Escherichia coli K-12 MG1655
<220> argi b4255
<222> 882-981
<223> argi b4255 U00096 complement(4474870..4475874)
<313> 1430: 912-931
<400> 1430
CCTACATGGC GGTATGGAAG TCACTGATGA GGTCTTCGAA TCTGCCGCCA 50
GCATTGTTTT TGATCAGGCG GAAAACCGTA TGCATACTAT CAAAGCGGTG 100
<210> 1431
<211> 100
<212> DNA
<213> Escherichia coli K-12 MG1655
<220> argi b4255
<222> 830-929
<223> argi b4255 U00096 complement(4474870..4475874)
<313> 1431: 870-889
<400> 1431
CGTTTCATGA CGACCAAACG ACGCTTGGCA AGAAAATGGC GGAAGAATTT 50
GGCCTACATG GCGGTATGGA AGTCACTGAT GAGGTCTTCG AATCTGCCGC 100
<210> 1432
<211> 100
<212> DNA
<213> Escherichia coli K-12 MG1655
<220> argi b4255
<222> 812-911
<223> argi b4255 U00096 complement(4474870..4475874)
<313> 1432: 840-859
<400> 1432
TCCTCCACTG CCTGCCCGCG TTTCATGACG ACCAAACGAC GCTTGGCAAG 50
AAAATGGCGG AAGAATTTGG CCTACATGGC GGTATGGAAG TCACTGATGA 100
<210> 1433
<211> 100
<212> DNA
<213> Escherichia coli K-12 MG1655
<220> yaai b0014
<222> 303-402

<223> yaai b0014 U00096 complement(11382..11786)
<313> 1433: 338-357
<400> 1433
CAGCGATAAC GACAATAAAC GCTGCGTCTC AAAAATCACC TTTTCGGGTC 50
ATACGGTGAA CTCATCGGAT ATGGCCACGC TGAAAATTAT CGGCGACGAC 100
<210> 1434
<211> 100
<212> DNA
<213> Escherichia coli K-12 MG1655
<220> yaai b0014
<222> 228-327
<223> yaai b0014 U00096 complement(11382..11786)
<313> 1434: 287-306
<400> 1434
CCGTAGCGCC AGTCAGAGCC TGAATATTCC TTCAGAAATA AAAGAAGGGC 50
AAACCACTGA CTGGATCAAC ATTAACAGCG ATAACGACAA TAAACGCTGC 100
<210> 1435
<211> 100
<212> DNA
<213> Escherichia coli K-12 MG1655
<220> yaai b0014
<222> 167-266
<223> yaai b0014 U00096 complement(11382..11786)
<313> 1435: 227-246
<400> 1435
AACTCTCTGC CGACCATGGC GATTTACAGT TAAGCGGTGC ATCGGTTTAT 50
TTCAAAGCCG CCCGTAGCGC CAGTCAGAGC CTGAATATTC CTTCAGAAAT 100
<210> 1436
<211> 100
<212> DNA
<213> Escherichia coli K-12 MG1655
<220> yaaj b0008
<222> 1293-1392
<223> yaaj b0008 U00096 complement(6529..7959)
<313> 1436: 1323-1342
<400> 1436
GCCTGTGGTT CATACCATTG CCAGTGATTA TCTACGCCAG CGTAAACTCG 50
GCGTGCGCCC GGTGTTTGAT CCATTGCGTT ATCCGGATAT CGGTCGCCAG 100
<210> 1437
<211> 100
<212> DNA
<213> Escherichia coli K-12 MG1655
<220> yaaj b0008
<222> 1227-1326
<223> yaaj b0008 U00096 complement(6529..7959)
<313> 1437: 1273-1292
<400> 1437
GCAACTGGCA GATATCATAA TGGCCTGCAT GGCTATTACC AATTTGACCG 50
CCATTTTACT GCTCTCGCCT GTGGTTCATA CCATTGCCAG TGATTATCTA 100
<210> 1438
<211> 100
<212> DNA
<213> Escherichia coli K-12 MG1655
<220> yaaj b0008
<222> 1200-1299
<223> yaaj b0008 U00096 complement(6529..7959)
<313> 1438: 1230-1249
<400> 1438
CACCTTGCTA AGCCTTCCGC TGATGTGGCA ACTGGCAGAT ATCATAATGG 50
CCTGCATGGC TATTACCAAT TTGACCGCCA TTTTACTGCT CTCGCCTGTG 100
<210> 1439
<211> 100
<212> DNA
<213> Escherichia coli K-12 MG1655
<220> ksga b0052
<222> 723-822
<223> ksga b0052 U00096 complement(51609..52430)
<313> 1439: 799-818
<400> 1439
GGGGATCGAC CCGGCGATGC GAGCGGAAAA TATCTCTGTC GCGCAATATT 50
GCCAGATGGC GAACTATCTG GCGGAGAACG CGCCTTTGCA GGAGAGTTAA 100

<210> 1440
<211> 100
<212> DNA
<213> Escherichia coli K-12 MG1655
<220> ksga b0052
<222> 698-797
<223> ksga b0052 U00096 complement(51609..52430)
<313> 1440: 750-769
<400> 1440
TTAGCGTCGA GGTGTTAACG GGAATGGGGA TCGACCCGGC GATGCGAGCG  50
GAAAATATCT CTGTCGCGCA ATATTGCCAG ATGGCGAACT ATCTGGCGGA  100
<210> 1441
<211> 100
<212> DNA
<213> Escherichia coli K-12 MG1655
<220> ksga b0052
<222> 643-742
<223> ksga b0052 U00096 complement(51609..52430)
<313> 1441: 678-697
<400> 1441
ACCGAAGCCT TTAACCAGCG TCGTAAAACC ATTCGTAACA GCCTCGGCAA  50
CCTGTTTAGC GTCGAGGTGT TAACGGGAAT GGGGATCGAC CCGGCGATGC  100
<210> 1442
<211> 100
<212> DNA
<213> Escherichia coli K-12 MG1655
<220> argr b3238
<222> 372-471
<223> argr b3238 U00096 3382338..3382808
<313> 1442: 442-461
<400> 1442
CATCGCTGGC GATGACACCA TCTTTACCAC CCCTGCTAAC GGTTTCACAG  50
TCAAAGACCT GTACGAAGCG ATTTTAGAGC TGTTCGACCA GGAGCTTTAA  100
<210> 1443
<211> 100
<212> DNA
<213> Escherichia coli K-12 MG1655
<220> argr b3238
<222> 369-468
<223> argr b3238 U00096 3382338..3382808
<313> 1443: 400-419
<400> 1443
CACCATCGCT GGCGATGACA CCATCTTTAC CACCCCTGCT AACGGTTTCA  50
CAGTCAAAGA CCTGTACGAA GCGATTTTAG AGCTGTTCGA CCAGGAGCTT  100
<210> 1444
<211> 100
<212> DNA
<213> Escherichia coli K-12 MG1655
<220> argr b3238
<222> 311-410
<223> argr b3238 U00096 3382338..3382808
<313> 1444: 355-374
<400> 1444
CGGCGCAGTT AATTGCTCGC CTGCTGGACT CACTGGGCAA AGCAGAAGGT  50
ATTCTGGGCA CCATCGCTGG CGATGACACC ATCTTTACCA CCCCTGCTAA  100
<210> 1445
<211> 100
<212> DNA
<213> Escherichia coli K-12 MG1655
<220> args b1877
<222> 1622-1721
<223> args b1877 U00096 1958086..1959819
<313> 1445: 1664-1683
<400> 1445
GCGCAGAAAA CGAAGAAGTG CGTAACAGCC GTCTAAAACT GGCACAACTG  50
ACGGCGAAGA CGCTGAAGCT GGGTCTGGAT ACGCTGGGTA TTGAGACTGT  100
<210> 1446
<211> 100
<212> DNA
<213> Escherichia coli K-12 MG1655
<220> args b1877

<222> 1594-1693
<223> args b1877 U00096 1958086..1959819
<313> 1446: 1637-1656
<400> 1446
GGCTTCTACG AGCACTGCCC GATCCTCAGC GCAGAAAACG AAGAAGTGCG 50
TAACAGCCGT CTAAAACTGG CACAACTGAC GGCGAAGACG CTGAAGCTGG 100
<210> 1447
<211> 100
<212> DNA
<213> Escherichia coli K-12 MG1655
<220> args b1877
<222> 1433-1532
<223> args b1877 U00096 1958086..1959819
<313> 1447: 1469-1488
<400> 1447
AAGAGCAACT GGCTGCAGCT CCGGTTATCA TCCGTGAAGA TCGTGAAGCG 50
CAACTGGCAG CTCGCCTGCT GCAGTTTGAA GAAACCCTCA CCGTGGTTGC 100
<210> 1448
<211> 100
<212> DNA
<213> Escherichia coli K-12 MG1655
<220> argt b2311
<222> 684-783
<223> argt b2311 U00096 complement(2425029..2425811)
<313> 1448: 726-745
<400> 1448
GACGGCTGCC TTCAATAAGG CGCTTGGCGA GCTGCGTCAG GACGGCACCT 50
ACGACAAGAT GGCGAAAAAG TATTTCGACT TTAATGTCTA CGGTGACTGA 100
<210> 1449
<211> 100
<212> DNA
<213> Escherichia coli K-12 MG1655
<220> argt b2311
<222> 624-723
<223> argt b2311 U00096 complement(2425029..2425811)
<313> 1449: 675-694
<400> 1449
AGACAAAAAA TACTTCGGTG ATGGCACCGG TGTAGGGCTA CGTAAAGATG 50
ATGCTGAACT GACGGCTGCC TTCAATAAGG CGCTTGGCGA GCTGCGTCAG 100
<210> 1450
<211> 100
<212> DNA
<213> Escherichia coli K-12 MG1655
<220> argt b2311
<222> 535-634
<223> argt b2311 U00096 complement(2425029..2425811)
<313> 1450: 574-593
<400> 1450
GCTGCGTTAC AAGATGAAGT TGCTGCCAGC GAAGGATTCC TCAAGCAACC 50
TGCTGGTAAA GATTTCGCCT TTGCTGGCTC ATCAGTAAAA GACAAAAAAT 100
<210> 1451
<211> 100
<212> DNA
<213> Escherichia coli K-12 MG1655
<220> yaau b0046
<222> 1233-1332
<223> yaau b0046 U00096 45807..47138
<313> 1451: 1287-1306
<400> 1451
GTTTGGCTTG TTGATTTCCG TAGCGTTTGC CCCGGAGACT CGAGGGATGT 50
CACTGGCGCA GACCAGCAAT ATGACGATCC GCGGGCAGAG AATGGGGTAA 100
<210> 1452
<211> 100
<212> DNA
<213> Escherichia coli K-12 MG1655
<220> yaau b0046
<222> 1208-1307
<223> yaau b0046 U00096 45807..47138
<313> 1452: 1235-1254
<400> 1452
TGCTAATGGG GGCGGGTATC TCGCTGTTTG GCTTGTTGAT TTCCGTAGCG 50

TTTGCCCCGG AGACTCGAGG GATGTCACTG GCGCAGACCA GCAATATGAC 100
<210> 1453
<211> 100
<212> DNA
<213> Escherichia coli K-12 MG1655
<220> yaau b0046
<222> 1165-1264
<223> yaau b0046 U00096 45807..47138
<313> 1453: 1203-1222
<400> 1453
GCACTACCGA TCTTTATCAA TAATTACGGT ATCAGTAACA CGATGCTAAT 50
GGGGGCGGGT ATCTCGCTGT TTGGCTTGTT GATTTCCGTA GCGTTTGCCC 100
<210> 1454
<211> 100
<212> DNA
<213> Escherichia coli K-12 MG1655
<220> enda b2946
<222> 609-708
<223> enda b2946 U00096 3088366..3089073
<313> 1454: 669-688
<400> 1454
GGTTACCGAC TGGGAGTGCG AGCGCGATGA ACGCATCGCG AAGGTGCAGG 50
GCAATCATAA CCCGTATGTG CAACGCGCTT GCCAGGCGCG AAAGAGCTAA 100
<210> 1455
<211> 100
<212> DNA
<213> Escherichia coli K-12 MG1655
<220> enda b2946
<222> 576-675
<223> enda b2946 U00096 3088366..3089073
<313> 1455: 608-627
<400> 1455
GCAGCTGTTC AACGCATGGA ACAAGATGTA TCCGGTTACC GACTGGGAGT 50
GCGAGCGCGA TGAACGCATC GCGAAGGTGC AGGGCAATCA TAACCCGTAT 100
<210> 1456
<211> 100
<212> DNA
<213> Escherichia coli K-12 MG1655
<220> enda b2946
<222> 519-618
<223> enda b2946 U00096 3088366..3089073
<313> 1456: 561-580
<400> 1456
GCGCACCTAC TTCTATATGC GCGACCAATA CAACCTGACA CTCTCTCGCC 50
AGCAAACGCA GCTGTTCAAC GCATGGAACA AGATGTATCC GGTTACCGAC 100
<210> 1457
<211> 100
<212> DNA
<213> Escherichia coli K-12 MG1655
<220> hema b1211
<222> 1144-1243
<223> hema b1211 U00096 1262937..1264193
<313> 1457: 1194-1213
<400> 1457
TGGAAACTGA CTAACCGCTT GATCCATGCG CCAACGAAAT CACTTCAACA 50
GGCCGCCCGT GACGGGGATA ACGAACGCCT GAATATTCTG CGCGACAGCC 100
<210> 1458
<211> 100
<212> DNA
<213> Escherichia coli K-12 MG1655
<220> hema b1211
<222> 1121-1220
<223> hema b1211 U00096 1262937..1264193
<313> 1458: 1153-1172
<400> 1458
AAGCCATTAT GCAGGATCTG GCATGGAAAC TGACTAACCG CTTGATCCAT 50
GCGCCAACGA AATCACTTCA ACAGGCCGCC CGTGACGGGG ATAACGAACG 100
<210> 1459
<211> 100
<212> DNA
<213> Escherichia coli K-12 MG1655

<220> hema b1211
<222> 1067-1166
<223> hema b1211 U00096 1262937..1264193
<313> 1459: 1115-1134
<400> 1459
GCGATGAGTT AACCGCCAAA GCGTTAGCGG CCCTTGAGCA GGGCGGCGAC 50
GCGCAAGCCA TTATGCAGGA TCTGGCATGG AAACTGACTA ACCGCTTGAT 100
<210> 1460
<211> 100
<212> DNA
<213> Escherichia coli K-12 MG1655
<220> ibpa b3688
<222> 274-373
<223> ibpa b3688 U00096 complement(3864636..3865049)
<313> 1460: 335-354
<400> 1460
GAACGCAACT TTGAACGCAA ATTCCAGTTA GCTGAGAACA TTCATGTTCG 50
TGGTGCTAAC CTGGTAAATG GTTTGCTGTA TATCGATCTC GAACGCGTGA 100
<210> 1461
<211> 100
<212> DNA
<213> Escherichia coli K-12 MG1655
<220> ibpa b3688
<222> 254-353
<223> ibpa b3688 U00096 complement(3864636..3865049)
<313> 1461: 267-286
<400> 1461
ATCTGTACCA GGGCATCGCT GAACGCAACT TTGAACGCAA ATTCCAGTTA 50
GCTGAGAACA TTCATGTTCG TGGTGCTAAC CTGGTAAATG GTTTGCTGTA 100
<210> 1462
<211> 100
<212> DNA
<213> Escherichia coli K-12 MG1655
<220> ibpa b3688
<222> 195-294
<223> ibpa b3688 U00096 complement(3864636..3865049)
<313> 1462: 226-245
<400> 1462
CCAGGATAAT CTGCTGGTGG TGAAAGGTGC TCACGCCGAC GAACAAAAAG 50
AGCGCACCTA TCTGTACCAG GGCATCGCTG AACGCAACTT TGAACGCAAA 100
<210> 1463
<211> 100
<212> DNA
<213> Escherichia coli K-12 MG1655
<220> hemb hemB
<222> 750-849
<223> hemb hemB U00096 complement(387977..388984)
<313> 1463: 773-792
<400> 1463
GGGCGCAGAC TGCCTGATGG TTAAACCTGC TGGAGCGTAC CTCGACATCG 50
TGCGTGAGCT GCGTGAACGT ACTGAATTGC CGATTGGCGC GTATCAGGTG 100
<210> 1464
<211> 100
<212> DNA
<213> Escherichia coli K-12 MG1655
<220> hemb hemB
<222> 642-741
<223> hemb hemB U00096 complement(387977..388984)
<313> 1464: 697-716
<400> 1464
GTTCCGTGAA GCTGCCGGAA GCGCATTAAA AGGCGACCGC AAAAGCTATC 50
AGATGAACCC AATGAACCGT CGTGAGGCGA TTCGTGAATC ACTGCTGGAT 100
<210> 1465
<211> 100
<212> DNA
<213> Escherichia coli K-12 MG1655
<220> hemb hemB
<222> 628-727
<223> hemb hemB U00096 complement(387977..388984)
<313> 1465: 652-671
<400> 1465

TCCTTTTATG GCCCGTTCCG TGAAGCTGCC GGAAGCGCAT TAAAAGGCGA 50
CCGCAAAAGC TATCAGATGA ACCCAATGAA CCGTCGTGAG GCGATTCGTG 100
<210> 1466
<211> 100
<212> DNA
<213> Escherichia coli K-12 MG1655
<220> ibpb b3687
<222> 336-435
<223> ibpb b3687 U00096 complement(3864096..3864530)
<313> 1466: 416-435
<400> 1466
CGTAAACGGT TTACTGCATA TTGATTTAAT TCGTAATGAG CCTGAACCCA 50
TCGCAGCGCA GCGTATCGCT ATCAGCGAAC GTCCCGCGTT AAATAGCTAA 100
<210> 1467
<211> 100
<212> DNA
<213> Escherichia coli K-12 MG1655
<220> ibpb b3687
<222> 336-435
<223> ibpb b3687 U00096 complement(3864096..3864530)
<313> 1467: 364-383
<400> 1467
CGTAAACGGT TTACTGCATA TTGATTTAAT TCGTAATGAG CCTGAACCCA 50
TCGCAGCGCA GCGTATCGCT ATCAGCGAAC GTCCCGCGTT AAATAGCTAA 100
<210> 1468
<211> 100
<212> DNA
<213> Escherichia coli K-12 MG1655
<220> ibpb b3687
<222> 279-378
<223> ibpb b3687 U00096 complement(3864096..3864530)
<313> 1468: 333-352
<400> 1468
GCCATTTAGC CTGAGCTTTA CGCTGGCTGA AAATATGGAA GTCTCTGGCG 50
CAACCTTCGT AAACGGTTTA CTGCATATTG ATTTAATTCG TAATGAGCCT 100
<210> 1469
<211> 100
<212> DNA
<213> Escherichia coli K-12 MG1655
<220> hemc b3806
<222> 864-963
<223> hemc b3806 U00096 complement(3987438..3988400)
<313> 1469: 926-945
<400> 1469
AGATGCCGAA CAAATGGGGA TTTCGCTGGC AGAAGAGCTA CTGAATAACG 50
GCGCGCGCGA GATCCTCGCT GAAGTCTATA ACGGAGACGC CCCGGCATGA 100
<210> 1470
<211> 100
<212> DNA
<213> Escherichia coli K-12 MG1655
<220> hemc b3806
<222> 851-950
<223> hemc b3806 U00096 complement(3987438..3988400)
<313> 1470: 894-913
<400> 1470
GCGGTGCGCC GCAAGATGCC GAACAAATGG GGATTTCGCT GGCAGAAGAG 50
CTACTGAATA ACGGCGCGCG CGAGATCCTC GCTGAAGTCT ATAACGGAGA 100
<210> 1471
<211> 100
<212> DNA
<213> Escherichia coli K-12 MG1655
<220> hemc b3806
<222> 678-777
<223> hemc b3806 U00096 complement(3987438..3988400)
<313> 1471: 718-737
<400> 1471
GAATCACCAC GAAACTGCAC TGCGCGTTAC CGCAGAACGC GCCATGAATA 50
CCCGTCTCGA AGGCGGATGT CAGGTGCCAA TTGGTAGCTA CGCCGAGCTT 100
<210> 1472
<211> 100
<212> DNA

<213> Escherichia coli K-12 MG1655
<220> yabb b0082
<222> 351-450
<223> yabb b0082 U00096 89634..90092
<313> 1472: 401-420
<400> 1472
TGAGCTGTGG GATGAAACAA CCTGGCATCA ACAGGTCAAG GAAGATATCG 50
ACGCAGAGCA GTTGGCTACC GGAGACTTAT CGGAGCGACT GCAGGACTTG 100
<210> 1473
<211> 100
<212> DNA
<213> Escherichia coli K-12 MG1655
<220> yabb b0082
<222> 336-435
<223> yabb b0082 U00096 89634..90092
<313> 1473: 363-382
<400> 1473
ACAGTTCAAC AAGTTTGAGC TGTGGGATGA AACAACCTGG CATCAACAGG 50
TCAAGGAAGA TATCGACGCA GAGCAGTTGG CTACCGGAGA CTTATCGGAG 100
<210> 1474
<211> 100
<212> DNA
<213> Escherichia coli K-12 MG1655
<220> yabb b0082
<222> 258-357
<223> yabb b0082 U00096 89634..90092
<313> 1474: 318-337
<400> 1474
GGATGGCGCA GGTCGATTGT TAATCGCGCC AGTACTGCGG CAACATGCCG 50
GGCTGACAAA AGAAGTGATG CTGGTTGGAC AGTTCAACAA GTTTGAGCTG 100
<210> 1475
<211> 100
<212> DNA
<213> Escherichia coli K-12 MG1655
<220> hemd b3805
<222> 642-741
<223> hemd b3805 U00096 complement(3986701..3987441)
<313> 1475: 714-733
<400> 1475
CAGTGAGCGT TTGGCGAAAC TCGCCCGGGA ACTGGGCTGG CAAGACATTA 50
AGGTCGCCGA TAACGCTGAC AACGATGCGC TTTTACGGGC ATTACAATAA 100
<210> 1476
<211> 100
<212> DNA
<213> Escherichia coli K-12 MG1655
<220> hemd b3805
<222> 488-587
<223> hemd b3805 U00096 complement(3986701..3987441)
<313> 1476: 519-538
<400> 1476
TCCATTACGA TGGTGCAGAA GAAGCGATGC GCTGGCAAGC CCGCGAGGTG 50
ACGATGGTCG TTGTTACCAG CGGTGAAATG TTGCAGCAAC TCTGGTCGCT 100
<210> 1477
<211> 100
<212> DNA
<213> Escherichia coli K-12 MG1655
<220> hemd b3805
<222> 426-525
<223> hemd b3805 U00096 complement(3986701..3987441)
<313> 1477: 452-471
<400> 1477
GGATACCCTG ACGGCGCGCG GTGCTGAGGT CACTTTTTGT GAATGTTATC 50
AACGATGCGC AATCCATTAC GATGGTGCAG AAGAAGCGAT GCGCTGGCAA 100
<210> 1478
<211> 100
<212> DNA
<213> Escherichia coli K-12 MG1655
<220> yabc b0083
<222> 843-942
<223> yabc b0083 U00096 90094..91035
<313> 1478: 889-908

<400> 1478
GCGAGCACTA GGCAAGTTAA TGCCGGGCGA AGAAGAGGTG GCTGAGAACC 50
CTCGTGCCCG TAGTTCAGTT CTGCGTATTG CAGAGAGGAC GAATGCATGA 100
<210> 1479
<211> 100
<212> DNA
<213> Escherichia coli K-12 MG1655
<220> yabc b0083
<222> 821-920
<223> yabc b0083 U00096 90094..91035
<313> 1479: 849-868
<400> 1479
AAAAACTGGG TGGCCGTCAG CTGCGAGCAC TAGGCAAGTT AATGCCGGGC 50
GAAGAAGAGG TGGCTGAGAA CCCTCGTGCC CGTAGTTCAG TTCTGCGTAT 100
<210> 1480
<211> 100
<212> DNA
<213> Escherichia coli K-12 MG1655
<220> yabc b0083
<222> 769-868
<223> yabc b0083 U00096 90094..91035
<313> 1480: 807-826
<400> 1480
AGCCGCGGTC CGCAAGTTCC GGCAGGGTTA CCGATGACTG AAGAGCAGCT 50
CAAAAAACTG GGTGGCCGTC AGCTGCGAGC ACTAGGCAAG TTAATGCCGG 100
<210> 1481
<211> 100
<212> DNA
<213> Escherichia coli K-12 MG1655
<220> heme b3998
<222> 935-1034
<223> heme b3998 U00096 4195294..4196358
<313> 1481: 959-978
<400> 1481
TTGCAGGTTT CGGTCACGGC GAAGGTCATG TCTTTAACCT TGGTCACGGC 50
ATTCATCAGG ATGTGCCGCC AGAACATGCT GGCGTGTTCG TGGAGGCAGT 100
<210> 1482
<211> 100
<212> DNA
<213> Escherichia coli K-12 MG1655
<220> heme b3998
<222> 865-964
<223> heme b3998 U00096 4195294..4196358
<313> 1482: 918-937
<400> 1482
CAGGGTAATA TGGATCCGTC GATGCTGTAC GCGCCGCCTG CCCGCATTGA 50
AGAAGAAGTA GCGACTATAC TTGCAGGTTT CGGTCACGGC GAAGGTCATG 100
<210> 1483
<211> 100
<212> DNA
<213> Escherichia coli K-12 MG1655
<220> heme b3998
<222> 762-861
<223> heme b3998 U00096 4195294..4196358
<313> 1483: 801-820
<400> 1483
GTGGCTGGAA GCCATGGCAG AAACCGGTTG CGATGCGCTG GGCCTCGACT 50
GGACAACGGA CATCGCCGAT GCGCGCCGCC GTGTGGGCAA TAAAGTCGCG 100
<210> 1484
<211> 100
<212> DNA
<213> Escherichia coli K-12 MG1655
<220> hemf b2437
<222> 793-892
<223> hemf b2437 U00096 2551245..2552144
<313> 1484: 843-862
<400> 1484
CTGATGTCAA TGCCGCCACT GGTACGCTGG GAATATGATT ATCAGCCAAA 50
AGATGGCAGC CCAGAAGCGG CGTTAAGTGA GTTTATTAAG GTCAGGGATT 100
<210> 1485
<211> 100

<212> DNA
<213> Escherichia coli K-12 MG1655
<220> hemf b2437
<222> 770-869
<223> hemf b2437 U00096 2551245..2552144
<313> 1485: 814-833
<400> 1485
CTGGCGGGCG CACCGAGTCT ATCCTGATGT CAATGCCGCC ACTGGTACGC 50
TGGGAATATG ATTATCAGCC AAAAGATGGC AGCCCAGAAG CGGCGTTAAG 100
<210> 1486
<211> 100
<212> DNA
<213> Escherichia coli K-12 MG1655
<220> hemf b2437
<222> 716-815
<223> hemf b2437 U00096 2551245..2552144
<313> 1486: 745-764
<400> 1486
GTTATGTCGA GTTCAATCTG GTCTGGGATC GCGGCACGCT GTTTGGCCTG 50
CAAACTGGCG GGCGCACCGA GTCTATCCTG ATGTCAATGC CGCCACTGGT 100
<210> 1487
<211> 100
<212> DNA
<213> Escherichia coli K-12 MG1655
<220> hemg b3851
<222> 447-546
<223> hemg b3851 U00096 4032197..4032742
<313> 1487: 521-540
<400> 1487
AACGGATACG CGCAAAGAAG TTGTCTATAC CGATTGGGAG CAGGTGGCGA 50
ATTTCGCCCG AGAAATCGCC CATTTAACCG ACAAACCGAC GCTGAAATAA 100
<210> 1488
<211> 100
<212> DNA
<213> Escherichia coli K-12 MG1655
<220> hemg b3851
<222> 420-519
<223> hemg b3851 U00096 4032197..4032742
<313> 1488: 447-466
<400> 1488
GCTGATTATG AAGATGTCAG GCGGTGAAAC GGATACGCGC AAAGAAGTTG 50
TCTATACCGA TTGGGAGCAG GTGGCGAATT TCGCCCGAGA AATCGCCCAT 100
<210> 1489
<211> 100
<212> DNA
<213> Escherichia coli K-12 MG1655
<220> hemg b3851
<222> 350-449
<223> hemg b3851 U00096 4032197..4032742
<313> 1489: 397-416
<400> 1489
ATCGCTGCGC GGTCATTGCC GGGGCGCTGC GTTACCCACG TTATCGCTGG 50
TACGACCGTT TTATGATCAA GCTGATTATG AAGATGTCAG GCGGTGAAAC 100
<210> 1490
<211> 100
<212> DNA
<213> Escherichia coli K-12 MG1655
<220> yabf b0047
<222> 432-531
<223> yabf b0047 U00096 47246..47776
<313> 1490: 493-512
<400> 1490
CATGCACTGC ACCTTTATTT GTGACGACGA AACCCTCGAA GGGCAGGCGC 50
GTCACTATAA GCAACGTCTG CTGGAATGGC AGGAGGCCCA TCATGGATAG 100
<210> 1491
<211> 100
<212> DNA
<213> Escherichia coli K-12 MG1655
<220> yabf b0047
<222> 425-524
<223> yabf b0047 U00096 47246..47776

<313> 1491: 449-468
<400> 1491
CGTTTGCCAT GCACTGCACC TTTATTTGTG ACGACGAAAC CCTCGAAGGG 50
CAGGCGCGTC ACTATAAGCA ACGTCTGCTG GAATGGCAGG AGGCCCATCA 100
<210> 1492
<211> 100
<212> DNA
<213> Escherichia coli K-12 MG1655
<220> yabf b0047
<222> 377-476
<223> yabf b0047 U00096 47246..47776
<313> 1492: 410-429
<400> 1492
AGCCGCTACA GGCGACGGCA ATCTACTGCG GGCTGAACTG GCTGCCACCG 50
TTTGCCATGC ACTGCACCTT TATTTGTGAC GACGAAACCC TCGAAGGGCA 100
<210> 1493
<211> 100
<212> DNA
<213> Escherichia coli K-12 MG1655
<220> hemh b0476
<222> 864-963
<223> hemh b0476 U00096 497279..498241
<313> 1493: 941-960
<400> 1493
CCTCGGTGCC GGCGGGAAAA AATATGAATA TATTCCGGCG CTTAATGCCA 50
CGCCGGAACA TATCGAAATG ATGGCTAATC TTGTTGCCGC GTATCGCTAA 100
<210> 1494
<211> 100
<212> DNA
<213> Escherichia coli K-12 MG1655
<220> hemh b0476
<222> 800-899
<223> hemh b0476 U00096 497279..498241
<313> 1494: 839-858
<400> 1494
CGGGCTTTGC TGCGGATTGT CTGGAGACGC TGGAAGAGAT TGCCGAGCAA 50
AACCGTGAGG TCTTCCTCGG TGCCGGCGGG AAAAAATATG AATATATTCC 100
<210> 1495
<211> 100
<212> DNA
<213> Escherichia coli K-12 MG1655
<220> hemh b0476
<222> 721-820
<223> hemh b0476 U00096 497279..498241
<313> 1495: 773-792
<400> 1495
CCCTGGCTGA TGCCTTATAC CGACGAAACG CTGAAAATGC TCGGAGAAAA 50
AGGCGTAGGT CATATTCAGG TGATGTGCCC GGGCTTTGCT GCGGATTGTC 100
<210> 1496
<211> 100
<212> DNA
<213> Escherichia coli K-12 MG1655
<220> yabh b0056
<222> 717-816
<223> yabh b0056 U00096 57364..58179
<313> 1496: 783-802
<400> 1496
GAAAGGTTTG CCGCCTGAGA TGATGGAGAT GGCGAAGCAG AAAGCGCAGG 50
AAATTCAGCA GGCATATGAG CTGATAAAGC AGCAGAAAGG GTTTAAATGA 100
<210> 1497
<211> 100
<212> DNA
<213> Escherichia coli K-12 MG1655
<220> yabh b0056
<222> 701-800
<223> yabh b0056 U00096 57364..58179
<313> 1497: 745-764
<400> 1497
CCGATAAGCT GGTGGCGAAA GGTTTGCCGC CTGAGATGAT GGAGATGGCG 50
AAGCAGAAAG CGCAGGAAAT TCAGCAGGCA TATGAGCTGA TAAAGCAGCA 100
<210> 1498

<211> 100
<212> DNA
<213> Escherichia coli K-12 MG1655
<220> yabh b0056
<222> 645-744
<223> yabh b0056 U00096 57364..58179
<313> 1498: 697-716
<400> 1498
GGATGATGCG ACCACCATCA AACGTGCCTA CCGTAAGCTG ATGAGTGAAC 50
ACCATCCCGA TAAGCTGGTG GCGAAAGGTT TGCCGCCTGA GATGATGGAG 100
<210> 1499
<211> 100
<212> DNA
<213> Escherichia coli K-12 MG1655
<220> yabi b0066
<222> 630-729
<223> yabi b0066 U00096 71351..72115
<313> 1499: 648-667
<400> 1499
TTATTTGTCC CGCGGTCGTT TGTTGTGGCT GACGCCGTTG ATTTCTGCCA 50
TCGGCGTGGT GGCGCTGGTG GTGTTAATTC GCCACCCGTT GATGCCGGTG 100
<210> 1500
<211> 100
<212> DNA
<213> Escherichia coli K-12 MG1655
<220> yabi b0066
<222> 591-690
<223> yabi b0066 U00096 71351..72115
<313> 1500: 608-627
<400> 1500
GTTATGGCGC AGCGGTAAAG CGACTGACCG TTTGAGTCAT TATTTGTCCC 50
GCGGTCGTTT GTTGTGGCTG ACGCCGTTGA TTTCTGCCAT CGGCGTGGTG 100
<210> 1501
<211> 100
<212> DNA
<213> Escherichia coli K-12 MG1655
<220> yabi b0066
<222> 487-586
<223> yabi b0066 U00096 71351..72115
<313> 1501: 513-532
<400> 1501
GGCGCGGCGA TCGATATTCC TGCCGGAATG CAGAGCGGTG AGTTTAAATG 50
GTTGCTGCTG GCAACAGCGG TGTTTTTGTG GGTTGGTGGC TGGCTGTGCT 100
<210> 1502
<211> 100
<212> DNA
<213> Escherichia coli K-12 MG1655
<220> hemk hemK
<222> 686-785
<223> hemk hemK U00096 1265317..1266150
<313> 1502: 728-747
<400> 1502
TATCCGGCGG CTTTCTGCTT CTGGAACATG GCTGGCAGCA GGGCGAAGCG 50
GTGCGACAAG CATTTATCCT CGCGGGGTAT CATGACGTCG AAACCTGCCG 100
<210> 1503
<211> 100
<212> DNA
<213> Escherichia coli K-12 MG1655
<220> hemk hemK
<222> 639-738
<223> hemk hemK U00096 1265317..1266150
<313> 1503: 655-674
<400> 1503
AATGGCAGAC ATCGTGCATA TCATCGAACA GTCGCGTAAC GCGCTGGTAT 50
CCGGCGGCTT TCTGCTTCTG GAACATGGCT GGCAGCAGGG CGAAGCGGTG 100
<210> 1504
<211> 100
<212> DNA
<213> Escherichia coli K-12 MG1655
<220> hemk hemK
<222> 513-612

<223> hemk hemK U00096 1265317..1266150
<313> 1504: 571-590
<400> 1504
GCTAGCCGGG CAGCAGTTTG CGATGATTGT CAGCAATCCG CCGTATATTG 50
ACGAGCAGGA CCCTCATCTT CAACAAGGCG ATGTCCGCTT TGAGCCGCTC 100
<210> 1505
<211> 100
<212> DNA
<213> Escherichia coli K-12 MG1655
<220> yabj b0067
<222> 600-699
<223> yabj b0067 U00096 complement(72229..72927)
<313> 1505: 663-682
<400> 1505
GGTAGTAGTC GCCGACGGGC GCATCGCCTG GCAGGGTATG ACCAATGAGT 50
TGTTGAGCGG TAAGGCGAGT GCTTCGGCAC TATTGGGGAT TACGGGTTAG 100
<210> 1506
<211> 100
<212> DNA
<213> Escherichia coli K-12 MG1655
<220> yabj b0067
<222> 569-668
<223> yabj b0067 U00096 complement(72229..72927)
<313> 1506: 634-653
<400> 1506
TGGAAGATGC GGCGCGGATC GCCACGCGCT CGGTAGTAGT CGCCGACGGG 50
CGCATCGCCT GGCAGGGTAT GACCAATGAG TTGTTGAGCG GTAAGGCGAG 100
<210> 1507
<211> 100
<212> DNA
<213> Escherichia coli K-12 MG1655
<220> yabj b0067
<222> 495-594
<223> yabj b0067 U00096 complement(72229..72927)
<313> 1507: 522-541
<400> 1507
TCAGGAGATG TTGACGCTGG TGAGCACGAG CTGCCAGCAG CAAAAAATGA 50
CGCTATTGAT GGTGTCGCAC AGCGTGGAAG ATGCGGCGCG GATCGCCACG 100
<210> 1508
<211> 100
<212> DNA
<213> Escherichia coli K-12 MG1655
<220> heml b0155
<222> 1170-1269
<223> heml b0155 U00096 complement(173602..174882)
<313> 1508: 1218-1237
<400> 1508
TGTTTACCTG GCACCGTCAG CGTTTGAAGC GGGCTTTATG TCCGTGGCGC 50
ACAGCATGGA AGATATCAAT AACACCATCG ATGCTGCACG TCGGGTGTTT 100
<210> 1509
<211> 100
<212> DNA
<213> Escherichia coli K-12 MG1655
<220> heml b0155
<222> 1156-1255
<223> heml b0155 U00096 complement(173602..174882)
<313> 1509: 1173-1192
<400> 1509
ATGCTGGACG AAGGTGTTTA CCTGGCACCG TCAGCGTTTG AAGCGGGCTT 50
TATGTCCGTG GCGCACAGCA TGGAAGATAT CAATAACACC ATCGATGCTG 100
<210> 1510
<211> 100
<212> DNA
<213> Escherichia coli K-12 MG1655
<220> heml b0155
<222> 1060-1159
<223> heml b0155 U00096 complement(173602..174882)
<313> 1510: 1080-1099
<400> 1510
ATGTTCGGTA TTTTCTTTAC CGACGCCGAG TCCGTGACGT GCTATCAGGA 50
TGTGATGGCC TGTGACGTGG AACGCTTTAA GCGTTTCTTC CATATGATGC 100

<210> 1511
<211> 100
<212> DNA
<213> Escherichia coli K-12 MG1655
<220> yabk b0068
<222> 1512-1611
<223> yabk b0068 U00096 complement(72911..74521)
<313> 1511: 1570-1589
<400> 1511
CCAGGACGGT GCGGTCACCG CGTTAATTCT GCTGCTGCTC TGTTTTCTGC 50
TGTTTACCGT GATTGAAAAA CTACCGGGGC GAAATGTTAA AACTGACTGA 100
<210> 1512
<211> 100
<212> DNA
<213> Escherichia coli K-12 MG1655
<220> yabk b0068
<222> 1489-1588
<223> yabk b0068 U00096 complement(72911..74521)
<313> 1512: 1546-1565
<400> 1512
CAGCAAATTG GCTCCTATCG CAGCCAGGAC GGTGCGGTCA CCGCGTTAAT 50
TCTGCTGCTG CTCTGTTTTC TGCTGTTTAC CGTGATTGAA AAACTACCGG 100
<210> 1513
<211> 100
<212> DNA
<213> Escherichia coli K-12 MG1655
<220> yabk b0068
<222> 1436-1535
<223> yabk b0068 U00096 complement(72911..74521)
<313> 1513: 1496-1515
<400> 1513
TGGTGGCGTT GTTCGGTAAC GATGATTTCC GCACCCTGCC GTTTTATCTC 50
TACCAGCAAA TTGGCTCCTA TCGCAGCCAG GACGGTGCGG TCACCGCGTT 100
<210> 1514
<211> 100
<212> DNA
<213> Escherichia coli K-12 MG1655
<220> hemm b1210
<222> 524-623
<223> hemm b1210 U00096 complement(1262100..1262723)
<313> 1514: 578-597
<400> 1514
GTTATGACAC CAAAACGCAA CCTGCGATGC CAGCCAATAT GGAACTCACC 50
GACGGTGGTC AACGCATCAA GTTAAAAATG GATAACTGGA TAGTGAAATA 100
<210> 1515
<211> 100
<212> DNA
<213> Escherichia coli K-12 MG1655
<220> hemm b1210
<222> 514-613
<223> hemm b1210 U00096 complement(1262100..1262723)
<313> 1515: 549-568
<400> 1515
GTTTATGGTG GTTATGACAC CAAAACGCAA CCTGCGATGC CAGCCAATAT 50
GGAACTCACC GACGGTGGTC AACGCATCAA GTTAAAAATG GATAACTGGA 100
<210> 1516
<211> 100
<212> DNA
<213> Escherichia coli K-12 MG1655
<220> hemm b1210
<222> 465-564
<223> hemm b1210 U00096 complement(1262100..1262723)
<313> 1516: 499-518
<400> 1516
CCGCCTGAGC GAAATTACCT ACAGCCAGAA TGGCAAAAAC TGGAAGGTTG 50
TTTATGGTGG TTATGACACC AAAACGCAAC CTGCGATGCC AGCCAATATG 100
<210> 1517
<211> 100
<212> DNA
<213> Escherichia coli K-12 MG1655
<220> b1400 b1401

<222> 492-591
<223> b1400 b1401 U00096 1462495..1463085
<313> 1517: 563-582
<400> 1517
ACGCTGTAAG CAGACGTTAC ATCAAGTCGA GCCATTGCGG GAAATTGAAC  50
CTGGCAGGAA ACGCCTGGTA TTTGATGAGA ATCTGCGACC GAAACAGTAA  100
<210> 1518
<211> 100
<212> DNA
<213> Escherichia coli K-12 MG1655
<220> b1400 b1401
<222> 482-581
<223> b1400 b1401 U00096 1462495..1463085
<313> 1518: 529-548
<400> 1518
TGCTGGTGAC ACGCTGTAAG CAGACGTTAC ATCAAGTCGA GCCATTGCGG  50
GAAATTGAAC CTGGCAGGAA ACGCCTGGTA TTTGATGAGA ATCTGCGACC  100
<210> 1519
<211> 100
<212> DNA
<213> Escherichia coli K-12 MG1655
<220> b1400 b1401
<222> 431-530
<223> b1400 b1401 U00096 1462495..1463085
<313> 1519: 483-502
<400> 1519
TCAGTGAGCA GGAGTTGGCA TGGAAAAAGC AGGGTACGCA TGAGTACCAG  50
GTGCTGGTGA CACGCTGTAA GCAGACGTTA CATCAAGTCG AGCCATTGCG  100
<210> 1520
<211> 100
<212> DNA
<213> Escherichia coli K-12 MG1655
<220> hemn b3868
<222> 1281-1380
<223> hemn b3868 U00096 4049619..4050998
<313> 1520: 1356-1375
<400> 1520
GACGGCGAAA GGTCGCTTGC TGATCCGCAA CATTTGCATG TGCTTTGATA  50
CCTATCTGCG CCAGAAAGCG CGGATGCAGC AGTTCTCTCG GGTGATTTAA  100
<210> 1521
<211> 100
<212> DNA
<213> Escherichia coli K-12 MG1655
<220> hemn b3868
<222> 1264-1363
<223> hemn b3868 U00096 4049619..4050998
<313> 1521: 1281-1300
<400> 1521
GAGAAGGGAA TACAGGTGAC GGCGAAAGGT CGCTTGCTGA TCCGCAACAT  50
TTGCATGTGC TTTGATACCT ATCTGCGCCA GAAAGCGCGG ATGCAGCAGT  100
<210> 1522
<211> 100
<212> DNA
<213> Escherichia coli K-12 MG1655
<220> hemn b3868
<222> 1229-1328
<223> hemn b3868 U00096 4049619..4050998
<313> 1522: 1257-1276
<400> 1522
CCCCGTTAGC AAAAGATGGG CTGGTGGATG TGGATGAGAA GGGAATACAG  50
GTGACGGCGA AAGGTCGCTT GCTGATCCGC AACATTTGCA TGTGCTTTGA  100
<210> 1523
<211> 100
<212> DNA
<213> Escherichia coli K-12 MG1655
<220> yabm b0071
<222> 1057-1156
<223> yabm b0071 U00096 77621..78799
<313> 1523: 1093-1112
<400> 1523
ATTCTGGCTG GCGTTATTCA GGGAGCAATT GCACAAAGTT GGGGGCACTT  50

TGCTGTCTAC TGGGTAATTG CGGTTATTTC TGTTGTCGCA TTATTTTTAA 100
<210> 1524
<211> 100
<212> DNA
<213> Escherichia coli K-12 MG1655
<220> yabm b0071
<222> 996-1095
<223> yabm b0071 U00096 77621..78799
<313> 1524: 1010-1029
<400> 1524
AATGCCTGGA AGAGCGGGGG CAGCTACCAC CTTATTTACT AACAGTATTT 50
CTACCGGGGT AATTCTGGCT GGCGTTATTC AGGGAGCAAT TGCACAAAGT 100
<210> 1525
<211> 100
<212> DNA
<213> Escherichia coli K-12 MG1655
<220> yabm b0071
<222> 907-1006
<223> yabm b0071 U00096 77621..78799
<313> 1525: 960-979
<400> 1525
AGCCGTATGG CGTTGATGAC GCTGCAACTT TTTAACGCTG TATTTATCGG 50
CATTGTTGCG GGTATTGGGA TGCTATGGTT TCAGGATTTA ATGCCTGGAA 100
<210> 1526
<211> 100
<212> DNA
<213> Escherichia coli K-12 MG1655
<220> yabn b0070
<222> 1517-1616
<223> yabn b0070 U00096 complement(75644..77299)
<313> 1526: 1557-1576
<400> 1526
TGGTCGCCAG CAAAGCGATG GTGCCATTAT TGCACCACTG GCTGATCATT 50
CAGGGGCAAC GCAGTATGCG CGGCCTGCGC ATGAATACCC TCGGCTGGTT 100
<210> 1527
<211> 100
<212> DNA
<213> Escherichia coli K-12 MG1655
<220> yabn b0070
<222> 1389-1488
<223> yabn b0070 U00096 complement(75644..77299)
<313> 1527: 1454-1473
<400> 1527
GGACTTCTCT GTTTTCGCAC ATTTATGCGA AGTGCCACTG CTACAACATT 50
GCATTCCCAT TGACTGGCAA GCCGACGCTG CTCGCTGGCG CAATGGCGAG 100
<210> 1528
<211> 100
<212> DNA
<213> Escherichia coli K-12 MG1655
<220> yabn b0070
<222> 1360-1459
<223> yabn b0070 U00096 complement(75644..77299)
<313> 1528: 1384-1403
<400> 1528
CTAAACAGCG CCAACTTTAC CCTGCCGCTG GACTTCTCTG TTTTCGCACA 50
TTTATGCGAA GTGCCACTGC TACAACATTG CATTCCCATT GACTGGCAAG 100
<210> 1529
<211> 100
<212> DNA
<213> Escherichia coli K-12 MG1655
<220> yabp b0057
<222> 552-651
<223> yabp b0057 U00096 58474..59124
<313> 1529: 626-645
<400> 1529
GAGACCTGGG TGGAATGCTA ACCTGGCGGT GCTACCCCGG ACGGTGCTAC 50
CCCGGACGGT GCTAACCCGG ACGGTGCTAA CCTGGACGGT GCTACCGTGA 100
<210> 1530
<211> 100
<212> DNA
<213> Escherichia coli K-12 MG1655

<220> yabp b0057
<222> 420-519
<223> yabp b0057 U00096 58474..59124
<313> 1530: 462-481
<400> 1530
GACGTTGCAG CTGCATAAGG GCCAAGGTAA CTTGGGCGTT GATCTTAGAA 50
AGGCTTACCT TCCCGGCGTT GACATGAGGG ATTGTTACCT TGGTAAAAAA 100
<210> 1531
<211> 100
<212> DNA
<213> Escherichia coli K-12 MG1655
<220> yabp b0057
<222> 171-270
<223> yabp b0057 U00096 58474..59124
<313> 1531: 211-230
<400> 1531
ACTTCTGTTC AATTTCGTGA ATAACCAGAC TGGCAACATC AACGCCTCAG 50
AATACTTTAC TGGGGCTATC AACGAGAATG AGAGAGAAAA GTTTATCAAT 100
<210> 1532
<211> 100
<212> DNA
<213> Escherichia coli K-12 MG1655
<220> yabq b0058
<222> 60-159
<223> yabq b0058 U00096 59121..59279
<313> 1532: 137-156
<400> 1532
AAAAATTGAT ACTAAAGGAG TTGCTACTGA AGAAGTTGCT ACTAAAAAAG 50
TACTGCTGAA CAAATTACTG ACAACGCAAT TATTGAATGA GCCAGAATAA 100
<210> 1533
<211> 100
<212> DNA
<213> Escherichia coli K-12 MG1655
<220> yabq b0058
<222> 60-159
<223> yabq b0058 U00096 59121..59279
<313> 1533: 118-137
<400> 1533
AAAAATTGAT ACTAAAGGAG TTGCTACTGA AGAAGTTGCT ACTAAAAAAG 50
TACTGCTGAA CAAATTACTG ACAACGCAAT TATTGAATGA GCCAGAATAA 100
<210> 1534
<211> 100
<212> DNA
<213> Escherichia coli K-12 MG1655
<220> yabq b0058
<222> 59-158
<223> yabq b0058 U00096 59121..59279
<313> 1534: 104-123
<400> 1534
AAAAAATTGA TACTAAAGGA GTTGCTACTG AAGAAGTTGC TACTAAAAAA 50
GTACTGCTGA ACAAATTACT GACAACGCAA TTATTGAATG AGCCAGAATA 100
<210> 1535
<211> 100
<212> DNA
<213> Escherichia coli K-12 MG1655
<220> b1408 b1408
<222> 491-590
<223> b1408 b1408 U00096 1475639..1476250
<313> 1535: 544-563
<400> 1535
TTGCCATTTA TCCACAGTGG ATGCAGTGGA ATAATCTTTT ATGGAGCATT 50
GCTTCAATTC TGCTTCTCTG GACTGCGATT AATCGTTGTC GGAGTGTGCT 100
<210> 1536
<211> 100
<212> DNA
<213> Escherichia coli K-12 MG1655
<220> b1408 b1408
<222> 444-543
<223> b1408 b1408 U00096 1475639..1476250
<313> 1536: 472-491
<400> 1536

TGGCAAAAGC GATCGCGCGT TAATATTTGG TCTGTGGGGT CTGGCCGTTG 50
CCATTTATCC ACAGTGGATG CAGTGGAATA ATCTTTTATG GAGCATTGCT 100
<210> 1537
<211> 100
<212> DNA
<213> Escherichia coli K-12 MG1655
<220> b1408 b1408
<222> 376-475
<223> b1408 b1408 U00096 1475639..1476250
<313> 1537: 421-440
<400> 1537
ATATTGACCG AGTTTTGCGG TTTACTCGCG CAGACGATTA ACGGTGTTCG 50
CAGTTATGCC GGTCCATTTG GCAAAAGCGA TCGCGCGTTA ATATTTGGTC 100
<210> 1538
<211> 100
<212> DNA
<213> Escherichia coli K-12 MG1655
<220> b1409 b1409
<222> 796-895
<223> b1409 b1409 U00096 1476250..1477146
<313> 1538: 828-847
<400> 1538
GGAAAACTAT TGCCAGGACA TGGCGGACTC CTTGACAGGA TTGACTCATT 50
AATTTTCACC GCTCCGGTAT TTTTTTATTT TATACGCTAC TGCTGTTACT 100
<210> 1539
<211> 100
<212> DNA
<213> Escherichia coli K-12 MG1655
<220> b1409 b1409
<222> 773-872
<223> b1409 b1409 U00096 1476250..1477146
<313> 1539: 788-807
<400> 1539
GAGATATTGG TGTTAAAGAT AGCGGAAAAC TATTGCCAGG ACATGGCGGA 50
CTCCTTGACA GGATTGACTC ATTAATTTTC ACCGCTCCGG TATTTTTTTA 100
<210> 1540
<211> 100
<212> DNA
<213> Escherichia coli K-12 MG1655
<220> b1409 b1409
<222> 623-722
<223> b1409 b1409 U00096 1476250..1477146
<313> 1540: 688-707
<400> 1540
GTCTGATGGG CGGCGTCATC ACCATCATGA TTGCGTCACT GATTATCGGA 50
CCGTTACTGA CACCGCTAAA TACATTACAG GCATTATTAG CGGGTTTGTT 100
<210> 1541
<211> 100
<212> DNA
<213> Escherichia coli K-12 MG1655
<220> hemx b3804
<222> 1083-1182
<223> hemx b3804 U00096 complement(3985498..3986679)
<313> 1541: 1157-1176
<400> 1541
GCGTAACCTG CTGGCACAAC CGGCAGCGGG GACAACGGAA GCTAAACCTG 50
CACCTGCACC GCAAGCTGAT ACTCCGGCAG CCGCGCCGCA AGGAGAATAA 100
<210> 1542
<211> 100
<212> DNA
<213> Escherichia coli K-12 MG1655
<220> hemx b3804
<222> 1083-1182
<223> hemx b3804 U00096 complement(3985498..3986679)
<313> 1542: 1109-1128
<400> 1542
GCGTAACCTG CTGGCACAAC CGGCAGCGGG GACAACGGAA GCTAAACCTG 50
CACCTGCACC GCAAGCTGAT ACTCCGGCAG CCGCGCCGCA AGGAGAATAA 100
<210> 1543
<211> 100
<212> DNA

<213> Escherichia coli K-12 MG1655
<220> hemx b3804
<222> 999-1098
<223> hemx b3804 U00096 complement(3985498..3986679)
<313> 1543: 1060-1079
<400> 1543
GTTAAGCCAG CAAAATATCT CGATGGATCT TCCGGAAACC CTGCAAAGCC  50
AGGCGATGCT GGAAAAACTG ATGCAGACTC GCGTGCGTAA CCTGCTGGCA  100
<210> 1544
<211> 100
<212> DNA
<213> Escherichia coli K-12 MG1655
<220> gef b0019
<222> 111-210
<223> gef b0019 U00096 complement(16751..16960)
<313> 1544: 186-205
<400> 1544
CGTAGTGGCG GCGCTGGTAA CGAGAAAAGA CCTCTGTGAG GTTCACATCC  50
GAACTGGCCA GACGGAGGTT GCTGTTTTCA CGGCTTACGA ATCCGAGTAA  100
<210> 1545
<211> 100
<212> DNA
<213> Escherichia coli K-12 MG1655
<220> gef b0019
<222> 111-210
<223> gef b0019 U00096 complement(16751..16960)
<313> 1545: 131-150
<400> 1545
CGTAGTGGCG GCGCTGGTAA CGAGAAAAGA CCTCTGTGAG GTTCACATCC  50
GAACTGGCCA GACGGAGGTT GCTGTTTTCA CGGCTTACGA ATCCGAGTAA  100
<210> 1546
<211> 100
<212> DNA
<213> Escherichia coli K-12 MG1655
<220> gef b0019
<222> 36-135
<223> gef b0019 U00096 complement(16751..16960)
<313> 1546: 84-103
<400> 1546
TAAGGTCAAG GAGAAGAGAG CAATGAAGCA GCATAAGGCG ATGATTGTCG  50
CCCTGATCGT CATCTGTATC ACCGCCGTAG TGGCGGCGCT GGTAACGAGA  100
<210> 1547
<211> 100
<212> DNA
<213> Escherichia coli K-12 MG1655
<220> hemy b3803
<222> 1080-1179
<223> hemy b3803 U00096 complement(3984299..3985495)
<313> 1547: 1113-1132
<400> 1547
CGCCTACGAT TACGCATGGC TTGCCGACGC GCTGGACAGA CTGCACAAGC  50
CGGAAGAAGC TGCAGCTATG CGTCGCGACG GTTTGATGTT AACGTTGCAG  100
<210> 1548
<211> 100
<212> DNA
<213> Escherichia coli K-12 MG1655
<220> hemy b3803
<222> 992-1091
<223> hemy b3803 U00096 complement(3984299..3985495)
<313> 1548: 1030-1049
<400> 1548
GGAGCACACT GGGCCAGTCA CTGATGAAGC ACGGAGAATG GCAGGAAGCA  50
TCGCTCGCCT TCCGCGCAGC GCTGAAACAA CGTCCGGACG CCTACGATTA  100
<210> 1549
<211> 100
<212> DNA
<213> Escherichia coli K-12 MG1655
<220> hemy b3803
<222> 967-1066
<223> hemy b3803 U00096 complement(3984299..3985495)
<313> 1549: 999-1018

<400> 1549
AACGTCGGCG ATCGCCCGCT GTTGTGGAGC ACACTGGGCC AGTCACTGAT 50
GAAGCACGGA GAATGGCAGG AAGCATCGCT CGCCTTCCGC GCAGCGCTGA 100
<210> 1550
<211> 100
<212> DNA
<213> Escherichia coli K-12 MG1655
<220> yaca b0098
<222> 489-588
<223> yaca b0098 U00096 107630..108217
<313> 1550: 559-578
<400> 1550
CATTGATTAT GCGCATTTTA CCCCACAAGC AAAATTCAGC ACGCCCGTCT 50
GGATAAGCCA GGCGCAAGGC ATCCGTGCTG GCCCTCAACG CCTCACCTAA 100
<210> 1551
<211> 100
<212> DNA
<213> Escherichia coli K-12 MG1655
<220> yaca b0098
<222> 476-575
<223> yaca b0098 U00096 107630..108217
<313> 1551: 520-539
<400> 1551
AAAAGGGTTA TCGCATTGAT TATGCGCATT TTACCCCACA AGCAAAATTC 50
AGCACGCCCG TCTGGATAAG CCAGGCGCAA GGCATCCGTG CTGGCCCTCA 100
<210> 1552
<211> 100
<212> DNA
<213> Escherichia coli K-12 MG1655
<220> yaca b0098
<222> 432-531
<223> yaca b0098 U00096 107630..108217
<313> 1552: 465-484
<400> 1552
GGATACGCTC AGCGCGCTGC TGACCCAGGA AGGCACGCCG TCTGAAAAGG 50
GTTATCGCAT TGATTATGCG CATTTTACCC CACAAGCAAA ATTCAGCACG 100
<210> 1553
<211> 100
<212> DNA
<213> Escherichia coli K-12 MG1655
<220> bola b0436
<222> 252-351
<223> bola b0436 U00096 453663..454013
<313> 1553: 313-332
<400> 1553
TGCGCTGGCT CTGCATACTT ACACTATTAA GGAGTGGGAA GGGTTGCAGG 50
ACACCGTCTT TGCCTCTCCT CCCTGTCGTG GAGCAGGAAG CATCGCGTAA 100
<210> 1554
<211> 100
<212> DNA
<213> Escherichia coli K-12 MG1655
<220> bola b0436
<222> 249-348
<223> bola b0436 U00096 453663..454013
<313> 1554: 271-290
<400> 1554
TCATGCGCTG GCTCTGCATA CTTACACTAT TAAGGAGTGG GAAGGGTTGC 50
AGGACACCGT CTTTGCCTCT CCTCCCTGTC GTGGAGCAGG AAGCATCGCG 100
<210> 1555
<211> 100
<212> DNA
<213> Escherichia coli K-12 MG1655
<220> bola b0436
<222> 184-283
<223> bola b0436 U00096 453663..454013
<313> 1555: 246-265
<400> 1555
GAACGTTTTC TGAATCGTCA TCGAATGATT TACAGTACTT TAGCGGAGGA 50
ACTCTCTACT ACCGTTCATG CGCTGGCTCT GCATACTTAC ACTATTAAGG 100
<210> 1556
<211> 100

<212> DNA
<213> Escherichia coli K-12 MG1655
<220> yacc b0123
<222> 372-471
<223> yacc b0123 U00096 complement(136570..137040)
<313> 1556: 441-460
<400> 1556
AGACAGCTAC CGCGATCTCA GCGGCATTGG CATTCCCGTC GCTAAAAAAT 50
GCAAAGCCCT GGCCCGCGAT TCCTTAAGCC TGCTTGCCTA CGTCAAATAA 100
<210> 1557
<211> 100
<212> DNA
<213> Escherichia coli K-12 MG1655
<220> yacc b0123
<222> 371-470
<223> yacc b0123 U00096 complement(136570..137040)
<313> 1557: 409-428
<400> 1557
AAGACAGCTA CCGCGATCTC AGCGGCATTG GCATTCCCGT CGCTAAAAAA 50
TGCAAAGCCC TGGCCCGCGA TTCCTTAAGC CTGCTTGCCT ACGTCAAATA 100
<210> 1558
<211> 100
<212> DNA
<213> Escherichia coli K-12 MG1655
<220> yacc b0123
<222> 315-414
<223> yacc b0123 U00096 complement(136570..137040)
<313> 1558: 365-384
<400> 1558
GCAAAACCAG TGGGACCTCA GTAATTACGA CACCTTCGAC ATGAAAGCCC 50
TCGGTGAAGA CAGCTACCGC GATCTCAGCG GCATTGGCAT TCCCGTCGCT 100
<210> 1559
<211> 100
<212> DNA
<213> Escherichia coli K-12 MG1655
<220> kdgk b3527
<222> 1050-1149
<223> kdgk b3527 U00096 3676830..3677978
<313> 1559: 1087-1106
<400> 1559
CGGCAGCGCG GAAGACGCGG CGAAACGTGG GCACCTGACC GCAAGTACCG 50
TTATTCAGTA TCGCGGCGCG ATTATCCCGC GTGAGGCGAT GCCAGCGTAA 100
<210> 1560
<211> 100
<212> DNA
<213> Escherichia coli K-12 MG1655
<220> kdgk b3527
<222> 935-1034
<223> kdgk b3527 U00096 3676830..3677978
<313> 1560: 971-990
<400> 1560
CTGGCGAAGG GTTAGTGGAT GTTCCGGCGG TGAAACTGCC GAAAGAAAAA 50
GTGATCGATA CCACCGCAGC TGGCGACTCT TTCAGTGCCG GTTATCTGGC 100
<210> 1561
<211> 100
<212> DNA
<213> Escherichia coli K-12 MG1655
<220> kdgk b3527
<222> 878-977
<223> kdgk b3527 U00096 3676830..3677978
<313> 1561: 908-927
<400> 1561
CGGGCGTGAA AGAAGTGGTG GTGAAACGCG GGGCGGATTC TTGCCTGGTG 50
TCCATTGCTG GCGAAGGGTT AGTGGATGTT CCGGCGGTGA AACTGCCGAA 100
<210> 1562
<211> 100
<212> DNA
<213> Escherichia coli K-12 MG1655
<220> yace b0104
<222> 522-621
<223> yace b0104 U00096 complement(112599..113219)

<313> 1562: 586-605
<400> 1562
TAATAACGGC GCACCGGATG CTATCGCATC GGATGTTGCC CGCCTGCACG 50
CACACTATTT GCAGCTTGCG TCGCAGTTTG TCTCACAGGA AAAACCGTAA 100
<210> 1563
<211> 100
<212> DNA
<213> Escherichia coli K-12 MG1655
<220> yace b0104
<222> 522-621
<223> yace b0104 U00096 complement(112599..113219)
<313> 1563: 551-570
<400> 1563
TAATAACGGC GCACCGGATG CTATCGCATC GGATGTTGCC CGCCTGCACG 50
CACACTATTT GCAGCTTGCG TCGCAGTTTG TCTCACAGGA AAAACCGTAA 100
<210> 1564
<211> 100
<212> DNA
<213> Escherichia coli K-12 MG1655
<220> yace b0104
<222> 361-460
<223> yace b0104 U00096 complement(112599..113219)
<313> 1564: 415-434
<400> 1564
TATAAAAAAG CGAATCGAGT GCTTGTGGTG GATGTCAGCC CAGAAACGCA 50
ACTTAAGCGC ACCATGCAGC GCGATGATGT AACTCGCGAG CATGTCGAAC 100
<210> 1565
<211> 100
<212> DNA
<213> Escherichia coli K-12 MG1655
<220> yacf b0103
<222> 645-744
<223> yacf b0103 U00096 complement(111856..112599)
<313> 1565: 674-693
<400> 1565
TTCCGGACAT AAGAGCCGTT TTGCCATTCG TTTTATGCCG CTGGACACTG 50
AAAACGGACA GGTACCGGAA CGTCTGGATT TCGAACTGGC CTGTTGCTAA 100
<210> 1566
<211> 100
<212> DNA
<213> Escherichia coli K-12 MG1655
<220> yacf b0103
<222> 582-681
<223> yacf b0103 U00096 complement(111856..112599)
<313> 1566: 626-645
<400> 1566
CGGTGGCGAT GCCGACTTGC TGCGCCTGAA TCTGTCGCTC GATTCACAGC 50
TTTATCCGCA AATTTCCGGA CATAAGAGCC GTTTTGCCAT TCGTTTTATG 100
<210> 1567
<211> 100
<212> DNA
<213> Escherichia coli K-12 MG1655
<220> yacf b0103
<222> 549-648
<223> yacf b0103 U00096 complement(111856..112599)
<313> 1567: 577-596
<400> 1567
ACAAACCAGC CTGAATGGTT TTTATCAGGA TAACGGTGGC GATGCCGACT 50
TGCTGCGCCT GAATCTGTCG CTCGATTCAC AGCTTTATCC GCAAATTTCC 100
<210> 1568
<211> 100
<212> DNA
<213> Escherichia coli K-12 MG1655
<220> yacg b0102
<222> 99-198
<223> yacg b0102 U00096 complement(111649..111846)
<313> 1568: 156-175
<400> 1568
GCTGATCGAC CTCGGAGAAT GGGCTGCTGA AGAAAAACGA ATCCCCAGCA 50
GTGGCGATCT TTCCGAAAGC GATGACTGGA GCGAAGAACC AAAGCAGTGA 100
<210> 1569

<211> 100
<212> DNA
<213> Escherichia coli K-12 MG1655
<220> yacg b0102
<222> 82-181
<223> yacg b0102 U00096 complement(111649..111846)
<313> 1569: 120-139
<400> 1569
TGCTCCAAAC GTTGTCAGCT GATCGACCTC GGAGAATGGG CTGCTGAAGA 50
AAAACGAATC CCCAGCAGTG GCGATCTTTC CGAAAGCGAT GACTGGAGCG 100
<210> 1570
<211> 100
<212> DNA
<213> Escherichia coli K-12 MG1655
<220> yacg b0102
<222> 15-114
<223> yacg b0102 U00096 complement(111649..111846)
<313> 1570: 65-84
<400> 1570
TACGGTGAAT TGCCCAACCT GCGGGAAAAC GGTGGTGTGG GGTGAAATCA 50
GCCCGTTTCG GCCATTTTGC TCCAAACGTT GTCAGCTGAT CGACCTCGGA 100
<210> 1571
<211> 100
<212> DNA
<213> Escherichia coli K-12 MG1655
<220> yach b0118
<222> 1755-1854
<223> yach b0118 U00096 complement(129407..131260)
<313> 1571: 1823-1842
<400> 1571
TCTAAACGAC AGTAACCATA CTGTCAGGCT GAATAACGAG CAACGGTCAG 50
CAGTATGCGA ACGTCTCTCT GAACGTGGAG CAAGAAGACT GGAAAGGTAA 100
<210> 1572
<211> 100
<212> DNA
<213> Escherichia coli K-12 MG1655
<220> yach b0118
<222> 1729-1828
<223> yach b0118 U00096 complement(129407..131260)
<313> 1572: 1788-1807
<400> 1572
AAAGTTCAGG AGAGCCGCCC ACAACGTCTA AACGACAGTA ACCATACTGT 50
CAGGCTGAAT AACGAGCAAC GGTCAGCAGT ATGCGAACGT CTCTCTGAAC 100
<210> 1573
<211> 100
<212> DNA
<213> Escherichia coli K-12 MG1655
<220> yach b0118
<222> 1701-1800
<223> yach b0118 U00096 complement(129407..131260)
<313> 1573: 1744-1763
<400> 1573
ACCTGAACAA CGTCAGGTTT TTCGGGAGAA AGTTCAGGAG AGCCGCCCAC 50
AACGTCTAAA CGACAGTAAC CATACTGTCA GGCTGAATAA CGAGCAACGG 100
<210> 1574
<211> 100
<212> DNA
<213> Escherichia coli K-12 MG1655
<220> thdf b3707
<222> 1258-1357
<223> thdf b3707 U00096 3884457..3885821
<313> 1574: 1306-1325
<400> 1574
GCGGAAGAGT TGCGTCTGGC ACAGCAGAAC TTAAGCGAAA TCACCGGGGA 50
ATTTACTTCA GACGACCTGC TGGGGCGGAT TTTCTCCAGC TTCTGTATTG 100
<210> 1575
<211> 100
<212> DNA
<213> Escherichia coli K-12 MG1655
<220> thdf b3707
<222> 1218-1317

<223> thdf b3707 U00096 3884457..3885821
<313> 1575: 1273-1292
<400> 1575
CAAAGCGCAA CTGTTGGGAG CCTGGGCAGG TGAACTGCTG GCGGAAGAGT 50
TGCGTCTGGC ACAGCAGAAC TTAAGCGAAA TCACCGGGGA ATTTACTTCA 100
<210> 1576
<211> 100
<212> DNA
<213> Escherichia coli K-12 MG1655
<220> thdf b3707
<222> 1180-1279
<223> thdf b3707 U00096 3884457..3885821
<313> 1576: 1201-1220
<400> 1576
CAGGCGCTGG AACAGGCAGC GGAACATCTA CAACAGGGCA AAGCGCAACT 50
GTTGGGAGCC TGGGCAGGTG AACTGCTGGC GGAAGAGTTG CGTCTGGCAC 100
<210> 1577
<211> 100
<212> DNA
<213> Escherichia coli K-12 MG1655
<220> yack b0124
<222> 1452-1551
<223> yack b0124 U00096 137083..138633
<313> 1577: 1513-1532
<400> 1577
GAAGTTTAAT CACGATGCAC CGAAAGAACA TGCTTATATG GCGCACTGCC 50
ATCTGCTGGA GCATGAAGAT ACGGGGATGA TGTTAGGGTT TACGGTATAA 100
<210> 1578
<211> 100
<212> DNA
<213> Escherichia coli K-12 MG1655
<220> yack b0124
<222> 1431-1530
<223> yack b0124 U00096 137083..138633
<313> 1578: 1483-1502
<400> 1578
TAATGTCAGC GAAGTGCTGG TGAAGTTTAA TCACGATGCA CCGAAAGAAC 50
ATGCTTATAT GGCGCACTGC CATCTGCTGG AGCATGAAGA TACGGGGATG 100
<210> 1579
<211> 100
<212> DNA
<213> Escherichia coli K-12 MG1655
<220> yack b0124
<222> 1378-1477
<223> yack b0124 U00096 137083..138633
<313> 1579: 1405-1424
<400> 1579
AAACCGCCAG CGGCTCATCG CGCGGGCTGG AAAGATACCG TTAAGGTAGA 50
AGGTAATGTC AGCGAAGTGC TGGTGAAGTT TAATCACGAT GCACCGAAAG 100
<210> 1580
<211> 100
<212> DNA
<213> Escherichia coli K-12 MG1655
<220> b1410 b1411
<222> 1659-1758
<223> b1410 b1411 U00096 1477162..1478919
<313> 1580: 1708-1727
<400> 1580
GGATTCACTC GTGCGTGATG CCGGATTTGA TAAATGCACA CAACGGATTG 50
ATGAGTGGGG TATTTTTACG GTTTCGATGG CGGTGCGTCG TGATAACTGA 100
<210> 1581
<211> 100
<212> DNA
<213> Escherichia coli K-12 MG1655
<220> b1410 b1411
<222> 1571-1670
<223> b1410 b1411 U00096 1477162..1478919
<313> 1581: 1608-1627
<400> 1581
ACCCTCAACT GGAGATGATT GCCGGGGTGT TAACCAGTCA TAAAGATGGT 50
AAACCGTGGG TAATGCGCGT GCGTTCGCAA GGGGAGATGG ATTCACTCGT 100

<210> 1582
<211> 100
<212> DNA
<213> Escherichia coli K-12 MG1655
<220> b1410 b1411
<222> 1484-1583
<223> b1410 b1411 U00096 1477162..1478919
<313> 1582: 1543-1562
<400> 1582
AAAATGAGCA GGTAAAAAAC TCACTCGCAG GTCTTGCCAA TGCCATCGAA 50
CCGGGCGGCA TTCTCATCTA CACCGGGCAG CCGTGGCACC CTCAACTGGA 100
<210> 1583
<211> 100
<212> DNA
<213> Escherichia coli K-12 MG1655
<220> yacl b0120
<222> 273-372
<223> yacl b0120 U00096 134340..134750
<313> 1583: 329-348
<400> 1583
CGCCAATCAA CTGGAATTCG CTGGCGATGA AATGGAAGAG GGGATGAACT 50
ACTACGACGA AGAAAGCCTG TCGCTATGCG GCGTTGAGGA TTTTCTGCAG 100
<210> 1584
<211> 100
<212> DNA
<213> Escherichia coli K-12 MG1655
<220> yacl b0120
<222> 241-340
<223> yacl b0120 U00096 134340..134750
<313> 1584: 261-280
<400> 1584
CTGTGGATGG ACGGTGAAGA GGTGATGGTT CGCGCCAATC AACTGGAATT 50
CGCTGGCGAT GAAATGGAAG AGGGGATGAA CTACTACGAC GAAGAAAGCC 100
<210> 1585
<211> 100
<212> DNA
<213> Escherichia coli K-12 MG1655
<220> yacl b0120
<222> 161-260
<223> yacl b0120 U00096 134340..134750
<313> 1585: 199-218
<400> 1585
TGCTTGATGA AGTGGAACAA GCAGCGCACG CACTGAAAGG TAGCGAACGG 50
TCCTGGCAAC GGGCAGGGCA TGAATACACC CTGTGGATGG ACGGTGAAGA 100
<210> 1586
<211> 100
<212> DNA
<213> Escherichia coli K-12 MG1655
<220> lpla b4387
<222> 918-1017
<223> lpla b4387 U00096 complement(4620670..4621686)
<313> 1586: 990-1009
<400> 1586
TATGCTGCAA CAGGAGTGCG AAGCGCTGTT GGTTGACTTC CCGGAACAGG 50
AAAAAGAGCT ACGGGAGTTA TCGGCATGGA TGGCGGGGGC TGTAAGGTAG 100
<210> 1587
<211> 100
<212> DNA
<213> Escherichia coli K-12 MG1655
<220> lpla b4387
<222> 890-989
<223> lpla b4387 U00096 complement(4620670..4621686)
<313> 1587: 905-924
<400> 1587
GACTGCAAGG CTGCCTGTAC CGCGCAGATA TGCTGCAACA GGAGTGCGAA 50
GCGCTGTTGG TTGACTTCCC GGAACAGGAA AAAGAGCTAC GGGAGTTATC 100
<210> 1588
<211> 100
<212> DNA
<213> Escherichia coli K-12 MG1655
<220> lpla b4387

<222> 829-928
<223> lpla b4387 U00096 complement(4620670..4621686)
<313> 1588: 872-891
<400> 1588
ACCCGCGCCC AGGTGTTTAC CGACAGCCTC AACCCCGCGC CGCTGGAAGC  50
CCTCGCCGGA CGACTGCAAG GCTGCCTGTA CCGCGCAGAT ATGCTGCAAC  100
<210> 1589
<211> 100
<212> DNA
<213> Escherichia coli K-12 MG1655
<220> kdgt b3910
<222> 894-993
<223> kdgt b3910 U00096 4099262..4100254
<313> 1589: 968-987
<400> 1589
CATTGTGACT TCGATTCTGG TGCCAATTCT TACCTCTATC TGGTCACGTA  50
AAGTCAAAGC CAGAGCAGCG AAAATCGAAA TTTTAGGTAC GGTGAAATAA  100
<210> 1590
<211> 100
<212> DNA
<213> Escherichia coli K-12 MG1655
<220> kdgt b3910
<222> 850-949
<223> kdgt b3910 U00096 4099262..4100254
<313> 1590: 887-906
<400> 1590
AAACCGATGG CTCCGGCAGC AACTTCGCTG GTAGCGACGG CGGTCATTGT  50
GACTTCGATT CTGGTGCCAA TTCTTACCTC TATCTGGTCA CGTAAAGTCA  100
<210> 1591
<211> 100
<212> DNA
<213> Escherichia coli K-12 MG1655
<220> kdgt b3910
<222> 771-870
<223> kdgt b3910 U00096 4099262..4100254
<313> 1591: 830-849
<400> 1591
TGCCGGAATT GCCGCTTCCA GTTCCGCAGG GGCCGCGGTA GCGACACCTG  50
TGCTGATTGC AGAAATGGTG CCTGCGTTTA AACCGATGGC TCCGGCAGCA  100
<210> 1592
<211> 100
<212> DNA
<213> Escherichia coli K-12 MG1655
<220> pdxa b0053
<222> 886-985
<223> pdxa b0053 U00096 complement(52427..53416)
<313> 1592: 935-954
<400> 1592
GACCACGGCA CCGCGCTTGA ACTGGCGGGA CGTGGCAAAG CCGATGTCGG  50
CAGTTTTATT ACGGCGCTTA ATCTCGCCAT CAAAATGATT GTTAACACCC  100
<210> 1593
<211> 100
<212> DNA
<213> Escherichia coli K-12 MG1655
<220> pdxa b0053
<222> 863-962
<223> pdxa b0053 U00096 complement(52427..53416)
<313> 1593: 898-917
<400> 1593
TGCCCTTTAT TCGCACATCA GTGGACCACG GCACCGCGCT TGAACTGGCG  50
GGACGTGGCA AAGCCGATGT CGGCAGTTTT ATTACGGCGC TTAATCTCGC  100
<210> 1594
<211> 100
<212> DNA
<213> Escherichia coli K-12 MG1655
<220> pdxa b0053
<222> 794-893
<223> pdxa b0053 U00096 complement(52427..53416)
<313> 1594: 827-846
<400> 1594
ACCACGATCA GGGTCTTCCC GTGCTAAAAT ACCAGGGCTT CGGGCGCGGT  50

GTGAACATTA CGCTGGGCCT GCCCTTTATT CGCACATCAG TGGACCACGG 100
<210> 1595
<211> 100
<212> DNA
<213> Escherichia coli K-12 MG1655
<220> pdxb b2321
<222> 1038-1137
<223> pdxb b2321 U00096 complement(2434735..2435871)
<313> 1595: 1111-1130
<400> 1595
CCGTGAATGG TCATCTCTGT ATGTAATTTG TGATGACGCC AGTGCGGCAT 50
CATTGCTGTG TAAACTGGGT TTTAACGCCG TTCATCATCC GGCACGTTAA 100
<210> 1596
<211> 100
<212> DNA
<213> Escherichia coli K-12 MG1655
<220> pdxb b2321
<222> 956-1055
<223> pdxb b2321 U00096 complement(2434735..2435871)
<313> 1596: 986-1005
<400> 1596
TGCGCCGCGA TGACGCACCG CTGCGTAAAG TCGCCGGGAT ACCGGGTGAG 50
TTCGATAAAC TGCGCAAAAA CTATCTTGAG CGCCGTGAAT GGTCATCTCT 100
<210> 1597
<211> 100
<212> DNA
<213> Escherichia coli K-12 MG1655
<220> pdxb b2321
<222> 873-972
<223> pdxb b2321 U00096 complement(2434735..2435871)
<313> 1597: 926-945
<400> 1597
TGCGCCAGAG TTTGGTCGCA TTACGCTGCA TGGCCCGCTC GATCAACCGA 50
CGCTGAAAAG GCTGGTGCAT TTGGTGTATG ATGTGCGCCG CGATGACGCA 100
<210> 1598
<211> 100
<212> DNA
<213> Escherichia coli K-12 MG1655
<220> b2145 b2145
<222> 119-218
<223> b2145 b2145 U00096 2231620..2231859
<313> 1598: 154-173
<400> 1598
CGGAACCGGT GTATGTCTGG CGAGCAAAAA ATCCCGGACT CTTTTTCGCT 50
TATATGGTGG CATATATCGG CTTCGGAATT TTATCTATCG GCATGATTGT 100
<210> 1599
<211> 100
<212> DNA
<213> Escherichia coli K-12 MG1655
<220> b2145 b2145
<222> 47-146
<223> b2145 b2145 U00096 2231620..2231859
<313> 1599: 99-118
<400> 1599
CGATATTTTG TTTCCGCGAA GCATGGAAAG GCTGGCGCGC AGGCGCGATT 50
GATAAACGGG TTAAAAATGC ACCGGAACCG GTGTATGTCT GGCGAGCAAA 100
<210> 1600
<211> 100
<212> DNA
<213> Escherichia coli K-12 MG1655
<220> b2145 b2145
<222> 18-117
<223> b2145 b2145 U00096 2231620..2231859
<313> 1600: 53-72
<400> 1600
TTTTGTCGTT GCCGTTTTTT TCCTTATCCC GATATTTTGT TTCCGCGAAG 50
CATGGAAAGG CTGGCGCGCA GGCGCGATTG ATAAACGGGT TAAAAATGCA 100
<210> 1601
<211> 100
<212> DNA
<213> Escherichia coli K-12 MG1655

<220> b2146 b2147
<222> 1140-1239
<223> b2146 b2147 U00096 2232053..2233291
<313> 1601: 1200-1219
<400> 1601
TATTGTTGAG GGTGACAAAA CCGTGGTCTA TGCCGTGAAA ACCGGGAAAG  50
AAGCCGCCGA GGCGATTCAT CACTATTTAG AGGGAGCTTG CTCATGTTAA  100
<210> 1602
<211> 100
<212> DNA
<213> Escherichia coli K-12 MG1655
<220> b2146 b2147
<222> 1079-1178
<223> b2146 b2147 U00096 2232053..2233291
<313> 1602: 1111-1130
<400> 1602
GTAACACCAT CAAAACACAA AATTACCAGA CCCGCGACCC GCAAGTCTTT  50
GCTGCTGGCG ATATTGTTGA GGGTGACAAA ACCGTGGTCT ATGCCGTGAA  100
<210> 1603
<211> 100
<212> DNA
<213> Escherichia coli K-12 MG1655
<220> b2146 b2147
<222> 973-1072
<223> b2146 b2147 U00096 2232053..2233291
<313> 1603: 993-1012
<400> 1603
AAGCATGTAC GGTTATCGGG CGAACTGACG ATGGCGGCAG ATAAAATTAT  50
TCTCGCCGTC GGTCAGCATG CCAGACTAGA TGCCTTTGCG GAGTTAGAGC  100
<210> 1604
<211> 100
<212> DNA
<213> Escherichia coli K-12 MG1655
<220> htpg b0474
<222> 1776-1875
<223> htpg b0474 U00096 494344..496218
<313> 1604: 1818-1837
<400> 1604
GGTAGAACTG CTGCTGGATC AGGCGCTGCT GGCAGAACGC GGCACGCTGG  50
AAGATCCGAA CCTGTTTATT CGTCGTATGA ACCAGCTGCT GGTTTCCTGA  100
<210> 1605
<211> 100
<212> DNA
<213> Escherichia coli K-12 MG1655
<220> htpg b0474
<222> 1716-1815
<223> htpg b0474 U00096 494344..496218
<313> 1605: 1751-1770
<400> 1605
GGATCACGTA CTGGTGAAAC GTGCGGCAGA TACTGAAGAT GAAGCGAAGT  50
TCAGCGAGTG GGTAGAACTG CTGCTGGATC AGGCGCTGCT GGCAGAACGC  100
<210> 1606
<211> 100
<212> DNA
<213> Escherichia coli K-12 MG1655
<220> htpg b0474
<222> 1689-1788
<223> htpg b0474 U00096 494344..496218
<313> 1606: 1722-1741
<400> 1606
AGTGAAATAC ATCTTCGAAC TGAACCCGGA TCACGTACTG GTGAAACGTG  50
CGGCAGATAC TGAAGATGAA GCGAAGTTCA GCGAGTGGGT AGAACTGCTG  100
<210> 1607
<211> 100
<212> DNA
<213> Escherichia coli K-12 MG1655
<220> pdxh b1639
<222> 558-657
<223> pdxh b1639 U00096 complement(1715375..1716031)
<313> 1607: 612-631
<400> 1607

ACAGATTGAG TTCTGGCAGG GTGGTGAGCA TCGCCTGCAT GACCGCTTTT 50
TGTACCAGCG TGAAAATGAT GCGTGGAAGA TTGATCGTCT TGCACCCTGA 100
<210> 1608
<211> 100
<212> DNA
<213> Escherichia coli K-12 MG1655
<220> pdxh b1639
<222> 534-633
<223> pdxh b1639 U00096 complement(1715375..1716031)
<313> 1608: 560-579
<400> 1608
GGGCGGTTTT CGCGTCAGCC TTGAACAGAT TGAGTTCTGG CAGGGTGGTG 50
AGCATCGCCT GCATGACCGC TTTTTGTACC AGCGTGAAAA TGATGCGTGG 100
<210> 1609
<211> 100
<212> DNA
<213> Escherichia coli K-12 MG1655
<220> pdxh b1639
<222> 475-574
<223> pdxh b1639 U00096 complement(1715375..1716031)
<313> 1609: 511-530
<400> 1609
AAATTCCTGG AGCTGAAGCA GAAGTTTCAA CAGGGCGAAG TGCCATTGCC 50
GAGCTTTTGG GGCGGTTTTC GCGTCAGCCT TGAACAGATT GAGTTCTGGC 100
<210> 1610
<211> 100
<212> DNA
<213> Escherichia coli K-12 MG1655
<220> pdxj b2565
<222> 633-732
<223> pdxj b2565 U00096 complement(2699018..2699749)
<313> 1610: 704-723
<400> 1610
ACTGAATATC GGTCATGCCA TTATTGGTCG TGCAGTGATG ACCGGACTGA 50
AAGATGCGGT GGCAGAAATG AAGCGTCTGA TGCTGGAAGC GCGTGGCTAA 100
<210> 1611
<211> 100
<212> DNA
<213> Escherichia coli K-12 MG1655
<220> pdxj b2565
<222> 594-693
<223> pdxj b2565 U00096 complement(2699018..2699749)
<313> 1611: 624-643
<400> 1611
CAACGTGAAA GCCATTGCCG CCATCCCTGA GATGCATGAA CTGAATATCG 50
GTCATGCCAT TATTGGTCGT GCAGTGATGA CCGGACTGAA AGATGCGGTG 100
<210> 1612
<211> 100
<212> DNA
<213> Escherichia coli K-12 MG1655
<220> pdxj b2565
<222> 523-622
<223> pdxj b2565 U00096 complement(2699018..2699749)
<313> 1612: 588-607
<400> 1612
ATCGCCAAAG CCGCGACCTT TGCCGCAAGC CTCGGTCTGA AAGTTAACGC 50
CGGACACGGT CTGACCTATC ACAACGTGAA AGCCATTGCC GCCATCCCTG 100
<210> 1613
<211> 100
<212> DNA
<213> Escherichia coli K-12 MG1655
<220> pdxk b2419
<222> 753-852
<223> pdxk b2419 U00096 complement(2534406..2535257)
<313> 1613: 795-814
<400> 1613
TGCAGTGCAC CGAGCGGGGT TGCGCGTACT GGAAGTGATG CGCTACACCC 50
AGCAGCATGA GAGCGATGAA TTGATTTTGC CGCCGCTGGC GGAAGCATAA 100
<210> 1614
<211> 100
<212> DNA

<213> Escherichia coli K-12 MG1655
<220> pdxk b2419
<222> 670-769
<223> pdxk b2419 U00096 complement(2534406..2535257)
<313> 1614: 719-738
<400> 1614
GTAAAAACCG ACCTGAAAGG GACTGGCGAC CTGTTTTGTG CTCAGCTCAT 50
CAGTGGCTTG CTGAAAGGGA AGGCGTTAAC CGATGCAGTG CACCGAGCGG 100
<210> 1615
<211> 100
<212> DNA
<213> Escherichia coli K-12 MG1655
<220> pdxk b2419
<222> 636-735
<223> pdxk b2419 U00096 complement(2534406..2535257)
<313> 1615: 694-713
<400> 1615
TGCCGACAGC GTGAATGTCA TTTCCCATTC ACGGGTAAAA ACCGACCTGA 50
AAGGGACTGG CGACCTGTTT TGTGCTCAGC TCATCAGTGG CTTGCTGAAA 100
<210> 1616
<211> 100
<212> DNA
<213> Escherichia coli K-12 MG1655
<220> yadb b0145
<222> 785-884
<223> yadb b0145 U00096 complement(159186..160112)
<313> 1616: 831-850
<400> 1616
AATTTCTGGG GCAGCAGGCA GAAGCACACT GGCAGGATTT CAGCGTCGAG 50
CAAATCCTTC AGTCAGCCGT CAAAAACTGG CGGCTTACCG CCGTGCCCGA 100
<210> 1617
<211> 100
<212> DNA
<213> Escherichia coli K-12 MG1655
<220> yadb b0145
<222> 743-842
<223> yadb b0145 U00096 complement(159186..160112)
<313> 1617: 786-805
<400> 1617
TGCCGAAAGG CGATCCACGC CCGGTACTAA TCGCGGCACT TCAATTTCTG 50
GGGCAGCAGG CAGAAGCACA CTGGCAGGAT TTCAGCGTCG AGCAAATCCT 100
<210> 1618
<211> 100
<212> DNA
<213> Escherichia coli K-12 MG1655
<220> yadb b0145
<222> 713-812
<223> yadb b0145 U00096 complement(159186..160112)
<313> 1618: 746-765
<400> 1618
AACTTTCCAA GCAGAATCAT GCGCCTGCGT TGCCGAAAGG CGATCCACGC 50
CCGGTACTAA TCGCGGCACT TCAATTTCTG GGGCAGCAGG CAGAAGCACA 100
<210> 1619
<211> 100
<212> DNA
<213> Escherichia coli K-12 MG1655
<220> clpa b0883
<222> 2149-2248
<223> clpa b0883 U00096 922487..924763
<313> 1619: 2203-2222
<400> 1619
GCCAACGAAC TGCTGTTTGG TTCGCTGGTG GACGGCGGTC AGGTCACCGT 50
CGCGCTGGAT AAAGAGAAAA ATGAGCTGAC TTACGGATTC CAGAGTGCAC 100
<210> 1620
<211> 100
<212> DNA
<213> Escherichia coli K-12 MG1655
<220> clpa b0883
<222> 2079-2178
<223> clpa b0883 U00096 922487..924763
<313> 1620: 2129-2148

<400> 1620
AGGTTACGAC CGGGCAATGG GCGCTCGTCC GATGGCGCGT GTCATCCAGG 50
ACAACCTGAA AAAACCGCTC GCCAACGAAC TGCTGTTTGG TTCGCTGGTG 100
<210> 1621
<211> 100
<212> DNA
<213> Escherichia coli K-12 MG1655
<220> clpa b0883
<222> 1979-2078
<223> clpa b0883 U00096 922487..924763
<313> 1621: 2017-2036
<400> 1621
AGGTGGTGGA TAAATTCATC GTCGAGTTGC AGGTTCAGCT GGATCAGAAA 50
GGTGTTTCTC TGGAAGTGAG CCAGGAAGCG CGTAACTGGC TGGCCGAGAA 100
<210> 1622
<211> 100
<212> DNA
<213> Escherichia coli K-12 MG1655
<220> yadc b0136
<222> 1140-1239
<223> yadc b0136 U00096 complement(149715..150953)
<313> 1622: 1195-1214
<400> 1622
GCCTCTTCAT TTCCAGGCAA CATTAAAGCA AGACGGGAAT ATTGCTATCG 50
AACCCGGCGA ATTTAAAGCC ACCAGTACTT TCCAGGTAAC CTATCCCTGA 100
<210> 1623
<211> 100
<212> DNA
<213> Escherichia coli K-12 MG1655
<220> yadc b0136
<222> 1115-1214
<223> yadc b0136 U00096 complement(149715..150953)
<313> 1623: 1138-1157
<400> 1623
CTAATCAAGG TAACGGCACA TCACAGCCTC TTCATTTCCA GGCAACATTA 50
AAGCAAGACG GGAATATTGC TATCGAACCC GGCGAATTTA AAGCCACCAG 100
<210> 1624
<211> 100
<212> DNA
<213> Escherichia coli K-12 MG1655
<220> yadc b0136
<222> 1065-1164
<223> yadc b0136 U00096 complement(149715..150953)
<313> 1624: 1098-1117
<400> 1624
TGTTTATATA GATTATGAAA CCGAAGATGA CACCTCCGAT GGGGTTTACC 50
CTAATCAAGG TAACGGCACA TCACAGCCTC TTCATTTCCA GGCAACATTA 100
<210> 1625
<211> 100
<212> DNA
<213> Escherichia coli K-12 MG1655
<220> clpb b2593
<222> 2475-2574
<223> clpb b2593 U00096 complement(2729620..2732193)
<313> 1625: 2553-2572
<400> 1625
CGAAAACCCG CTGGCACAGC AAATACTGTC TGGTGAATTG GTTCCGGGTA 50
AAGTGATTCG CCTGGAAGTT AATGAAGACC GGATTGTCGC CGTCCAGTAA 100
<210> 1626
<211> 100
<212> DNA
<213> Escherichia coli K-12 MG1655
<220> clpb b2593
<222> 2437-2536
<223> clpb b2593 U00096 complement(2729620..2732193)
<313> 1626: 2486-2505
<400> 1626
GGTGCACGTC CTCTGAAACG TGCAATTCAG CAGCAGATCG AAAACCCGCT 50
GGCACAGCAA ATACTGTCTG GTGAATTGGT TCCGGGTAAA GTGATTCGCC 100
<210> 1627
<211> 100

<212> DNA
<213> Escherichia coli K-12 MG1655
<220> clpb b2593
<222> 2357-2456
<223> clpb b2593 U00096 complement(2729620..2732193)
<313> 1627: 2407-2426
<400> 1627
TGGAAGAACG TGGTTATGAA ATCCACATTT CTGACGAGGC GCTGAAACTG 50
CTGAGCGAGA ACGGTTACGA TCCGGTCTAT GGTGCACGTC CTCTGAAACG 100
<210> 1628
<211> 100
<212> DNA
<213> Escherichia coli K-12 MG1655
<220> yadd b0133
<222> 804-903
<223> yadd b0133 U00096 146968..147870
<313> 1628: 856-875
<400> 1628
CGCCCAGCGT CTTCTGAGTA AAGGAATGTC TCGGGAAGAC GTTGCAGAGA 50
TGGCAAATTT ACCTCTTGCT GAGATTGATA AGGTAATTAA CCTTATTTAA 100
<210> 1629
<211> 100
<212> DNA
<213> Escherichia coli K-12 MG1655
<220> yadd b0133
<222> 771-870
<223> yadd b0133 U00096 146968..147870
<313> 1629: 802-821
<400> 1629
TCAGCAGGGA AGACAGGAAG TAAGTCAGGA ATTCGCCCAG CGTCTTCTGA 50
GTAAAGGAAT GTCTCGGGAA GACGTTGCAG AGATGGCAAA TTTACCTCTT 100
<210> 1630
<211> 100
<212> DNA
<213> Escherichia coli K-12 MG1655
<220> yadd b0133
<222> 723-822
<223> yadd b0133 U00096 146968..147870
<313> 1630: 771-790
<400> 1630
GATGACGCTG GCGCAGTGGT TTGAAGAGAA AGGGATTGAG AAGGGGATTC 50
AGCAGGGAAG ACAGGAAGTA AGTCAGGAAT TCGCCCAGCG TCTTCTGAGT 100
<210> 1631
<211> 100
<212> DNA
<213> Escherichia coli K-12 MG1655
<220> yade b0131
<222> 1062-1161
<223> yade b0131 U00096 145081..146310
<313> 1631: 1127-1146
<400> 1631
CAACGCCGTG AAGGCAGCAA ACGATGCCGG ATTTCACCTG GCGGTGACAA 50
CCATGAAAGG CAAAGTAAAA CCGGGGGATA ATCCGTTGTT ACTAAAACGA 100
<210> 1632
<211> 100
<212> DNA
<213> Escherichia coli K-12 MG1655
<220> yade b0131
<222> 1056-1155
<223> yade b0131 U00096 145081..146310
<313> 1632: 1081-1100
<400> 1632
TAATGACAAC GCCGTGAAGG CAGCAAACGA TGCCGGATTT CACCTGGCGG 50
TGACAACCAT GAAAGGCAAA GTAAAACCGG GGGATAATCC GTTGTTACTA 100
<210> 1633
<211> 100
<212> DNA
<213> Escherichia coli K-12 MG1655
<220> yade b0131
<222> 963-1062
<223> yade b0131 U00096 145081..146310

<313> 1633: 1026-1045
<400> 1633
GCACAATATT CTGTTTGATT TTGCACGTTC ACGCCGCGCT CTGGCGCAAT 50
TTAATCCGCA TGTCTGGTAT CTTTCGTATC CGTTTGGCGG ATTTAATGAC 100
<210> 1634
<211> 100
<212> DNA
<213> Escherichia coli K-12 MG1655
<220> yadf b0127
<222> 564-663
<223> yadf b0127 U00096 complement(142008..142670)
<313> 1634: 642-661
<400> 1634
GCGTGATCTG GATGTTACCG CCACCAACCG CGAAACCCTT GAGCAACGTT 50
ACCGTCACGG GATTTCCAAC CTCAAGCTGA AACACGCCAA CCACAAATAA 100
<210> 1635
<211> 100
<212> DNA
<213> Escherichia coli K-12 MG1655
<220> yadf b0127
<222> 553-652
<223> yadf b0127 U00096 complement(142008..142670)
<313> 1635: 591-610
<400> 1635
GACGGCTTGC TGCGTGATCT GGATGTTACC GCCACCAACC GCGAAACCCT 50
TGAGCAACGT TACCGTCACG GGATTTCCAA CCTCAAGCTG AAACACGCCA 100
<210> 1636
<211> 100
<212> DNA
<213> Escherichia coli K-12 MG1655
<220> yadf b0127
<222> 477-576
<223> yadf b0127 U00096 complement(142008..142670)
<313> 1636: 520-539
<400> 1636
CCACTCCACC ATTATGCAAT CAGCGTGGAA ACGCGGGCAG AAAGTTACCA 50
TTCACGGCTG GGCCTACGGC ATTCACGACG GCTTGCTGCG TGATCTGGAT 100
<210> 1637
<211> 100
<212> DNA
<213> Escherichia coli K-12 MG1655
<220> htpx b1830
<222> 783-882
<223> htpx b1830 U00096 complement(1909719..1910600)
<313> 1637: 834-853
<400> 1637
CATTAACGGT AAGTCGAAAT CGCTCAGTGA GTTGTTCATG ACCCACCCGC 50
CGCTGGATAA ACGAATTGAA GCTCTGCGTA CGGGTGAATA CCTGAAGTAA 100
<210> 1638
<211> 100
<212> DNA
<213> Escherichia coli K-12 MG1655
<220> htpx b1830
<222> 732-831
<223> htpx b1830 U00096 complement(1909719..1910600)
<313> 1638: 789-808
<400> 1638
CCTGAAAACC AGCTATGAAC CGCAAGAAGC AACCAGCATG ATGGCTCTCT 50
GCATTAACGG TAAGTCGAAA TCGCTCAGTG AGTTGTTCAT GACCCACCCG 100
<210> 1639
<211> 100
<212> DNA
<213> Escherichia coli K-12 MG1655
<220> htpx b1830
<222> 713-812
<223> htpx b1830 U00096 complement(1909719..1910600)
<313> 1639: 740-759
<400> 1639
TGATTGCCGC GCTGCAGCGC CTGAAAACCA GCTATGAACC GCAAGAAGCA 50
ACCAGCATGA TGGCTCTCTG CATTAACGGT AAGTCGAAAT CGCTCAGTGA 100
<210> 1640

<211> 100
<212> DNA
<213> Escherichia coli K-12 MG1655
<220> yadg b0128
<222> 828-927
<223> yadg b0128 U00096 142779..143705
<313> 1640: 877-896
<400> 1640
TGAGCAGGGC ATTCAGGTAT TAAGTATGCG TAACAAAGCT AACCGTCTGG 50
AAGAGCTGTT TGTTTCACTG GTTAATGAAA AACAAGGAGA TCGCGCATGA 100
<210> 1641
<211> 100
<212> DNA
<213> Escherichia coli K-12 MG1655
<220> yadg b0128
<222> 793-892
<223> yadg b0128 U00096 142779..143705
<313> 1641: 816-835
<400> 1641
GAGCAGGGGA TCAACAGCGT ATTTACGCAG TTAAGTGAGC AGGGCATTCA 50
GGTATTAAGT ATGCGTAACA AAGCTAACCG TCTGGAAGAG CTGTTTGTTT 100
<210> 1642
<211> 100
<212> DNA
<213> Escherichia coli K-12 MG1655
<220> yadg b0128
<222> 724-823
<223> yadg b0128 U00096 142779..143705
<313> 1642: 779-798
<400> 1642
TTACCGAAGC TCGATGGCTA TCAGTATCGA CTGGTCGATA CCGCGACGCT 50
GGAAGTTGAA GTGCTGCGTG AGCAGGGGAT CAACAGCGTA TTTACGCAGT 100
<210> 1643
<211> 100
<212> DNA
<213> Escherichia coli K-12 MG1655
<220> pdxy b1637
<222> 765-864
<223> pdxy b1637 U00096 complement(1713050..1713913)
<313> 1643: 840-859
<400> 1643
GGTGACCACC AAAGCAATGC AGGAATATGA GCTGCAAGTG GTGGCTGCTC 50
AGGATCGTAT TGCCAAACCA GAACATTACT TCAGCGCAAC AAAGCTCTGA 100
<210> 1644
<211> 100
<212> DNA
<213> Escherichia coli K-12 MG1655
<220> pdxy b1637
<222> 742-841
<223> pdxy b1637 U00096 complement(1713050..1713913)
<313> 1644: 782-801
<400> 1644
ACCGCTGCAG TCTACGAAAT CATGGTGACC ACCAAAGCAA TGCAGGAATA 50
TGAGCTGCAA GTGGTGGCTG CTCAGGATCG TATTGCCAAA CCAGAACATT 100
<210> 1645
<211> 100
<212> DNA
<213> Escherichia coli K-12 MG1655
<220> pdxy b1637
<222> 657-756
<223> pdxy b1637 U00096 complement(1713050..1713913)
<313> 1645: 696-715
<400> 1645
GGTAGGTGTT GGTGATGTGA CGAGCGGTTT ACTGCTGGTG AAACTGCTTC 50
AGGGGGCAAC GCTGCAGGAG GCGCTGGAAC ATGTGACCGC TGCAGTCTAC 100
<210> 1646
<211> 100
<212> DNA
<213> Escherichia coli K-12 MG1655
<220> yadh b0129
<222> 672-771

<223> yadh b0129 U00096 143702..144472
<313> 1646: 733-752
<400> 1646
TCCGCTGGTC ACTACCTTTG GCGTACTGGT GGTCTTTATT GTGGCGTTTT 50
ATTTGATCTG TTGGTCGCTG ATCCAACGTG GACGTGGTTT GCGTAGCTAA 100
<210> 1647
<211> 100
<212> DNA
<213> Escherichia coli K-12 MG1655
<220> yadh b0129
<222> 649-748
<223> yadh b0129 U00096 143702..144472
<313> 1647: 688-707
<400> 1647
GGCTTCCTCG GTATCAATGA TGTTCCGCTG GTCACTACCT TTGGCGTACT 50
GGTGGTCTTT ATTGTGGCGT TTTATTTGAT CTGTTGGTCG CTGATCCAAC 100
<210> 1648
<211> 100
<212> DNA
<213> Escherichia coli K-12 MG1655
<220> yadh b0129
<222> 607-706
<223> yadh b0129 U00096 143702..144472
<313> 1648: 649-668
<400> 1648
CACCTGAACC CAATCGTTTA TATGATCAGT GGTTTCCGCT ACGGCTTCCT 50
CGGTATCAAT GATGTTCCGC TGGTCACTAC CTTTGGCGTA CTGGTGGTCT 100
<210> 1649
<211> 100
<212> DNA
<213> Escherichia coli K-12 MG1655
<220> yadi b0130
<222> 342-441
<223> yadi b0130 U00096 144577..145017
<313> 1649: 398-417
<400> 1649
GCGTATTGTC GAACTGGGTG CGCCGGAGGT GAGTAGTCTT TGGCACCAAC 50
AACAAAAAAA TCCGCCTTTC GTCCTCAAAC ATAATTTGTA TGAGTATTAA 100
<210> 1650
<211> 100
<212> DNA
<213> Escherichia coli K-12 MG1655
<220> yadi b0130
<222> 265-364
<223> yadi b0130 U00096 144577..145017
<313> 1650: 304-323
<400> 1650
ATTTCTGGTG TCACGTTACC GTTAATTGAA CAGATGATGG CTTGCCGTGA 50
AACCATGACC AGTTCAGAGT TTCGCGAGCG TATTGTCGAA CTGGGTGCGC 100
<210> 1651
<211> 100
<212> DNA
<213> Escherichia coli K-12 MG1655
<220> yadi b0130
<222> 230-329
<223> yadi b0130 U00096 144577..145017
<313> 1651: 260-279
<400> 1651
CATTATTAAG CCACAAACAC TCCCGTTGCG AAGTGATTTC TGGTGTCACG 50
TTACCGTTAA TTGAACAGAT GATGGCTTGC CGTGAAACCA TGACCAGTTC 100
<210> 1652
<211> 100
<212> DNA
<213> Escherichia coli K-12 MG1655
<220> yadk b0137
<222> 473-572
<223> yadk b0137 U00096 complement(151003..151599)
<313> 1652: 512-531
<400> 1652
TGTCGGATGA CATGAATGGT TCATCCTGGA AATTTAGCAC CCGAATGCAA 50
AAAATCGATC CTGCATTGAG TGTTACATCT GGTCAACTTA TGAGCCATGT 100

<210> 1653
<211> 100
<212> DNA
<213> Escherichia coli K-12 MG1655
<220> yadk b0137
<222> 382-481
<223> yadk b0137 U00096 complement(151003..151599)
<313> 1653: 424-443
<400> 1653
GTAGGAGTAG TTATATTTTC TACTCAGGAT TCGGCGAATA CATTCAATGT 50
TCTTAATGCT TCAGGCGGAT CTCGTTCCGT TTATCCAGTA ATGTCGGATG 100
<210> 1654
<211> 100
<212> DNA
<213> Escherichia coli K-12 MG1655
<220> yadk b0137
<222> 221-320
<223> yadk b0137 U00096 complement(151003..151599)
<313> 1654: 275-294
<400> 1654
CAGGTGGAAA AGAGTTCACT ATCACTGTAT CCGACTGTGC ATTACAGGGA 50
ACTGGCGATG TGCTAAATCA GTTACATATT GATTTTAGAG CCCTTAGCGG 100
<210> 1655
<211> 100
<212> DNA
<213> Escherichia coli K-12 MG1655
<220> b1420 b1421
<222> 67-166
<223> b1420 b1421 U00096 complement(1489986..1490153)
<313> 1655: 125-144
<400> 1655
CAACCCTCTG GTGGTGTGTC TGCTCATTAT CTGCATTACG ATTCTGACAT 50
TCACACTCCT GACCCGACAA ACGCTCTACG AACTGCGGTT CCGGGACGGT 100
<210> 1656
<211> 100
<212> DNA
<213> Escherichia coli K-12 MG1655
<220> b1420 b1421
<222> 22-121
<223> b1420 b1421 U00096 complement(1489986..1490153)
<313> 1656: 90-109
<400> 1656
TTAGCCTCTT ACCCGCCATC CGGCAAGGAG AAAGGCTATG AAGCACAACC 50
CTCTGGTGGT GTGTCTGCTC ATTATCTGCA TTACGATTCT GACATTCACA 100
<210> 1657
<211> 100
<212> DNA
<213> Escherichia coli K-12 MG1655
<220> b1420 b1421
<222> 13-112
<223> b1420 b1421 U00096 complement(1489986..1490153)
<313> 1657: 47-66
<400> 1657
ATTCAGATCT TAGCCTCTTA CCCGCCATCC GGCAAGGAGA AAGGCTATGA 50
AGCACAACCC TCTGGTGGTG TGTCTGCTCA TTATCTGCAT TACGATTCTG 100
<210> 1658
<211> 100
<212> DNA
<213> Escherichia coli K-12 MG1655
<220> yadl b0138
<222> 507-606
<223> yadl b0138 U00096 complement(151626..152231)
<313> 1658: 565-584
<400> 1658
GATGAGTGCA CGCCTGGTCG TGGAAAAAAA TAAAACCGTA AACAATGTCA 50
CTGCGGGTAA GTTTTCTGCA CCAGCCACAT TTACAGTAAC CTATAACTAA 100
<210> 1659
<211> 100
<212> DNA
<213> Escherichia coli K-12 MG1655
<220> yadl b0138

<222> 474-573
<223> yadl b0138 U00096 complement(151626..152231)
<313> 1659: 517-536
<400> 1659
CTCCACTGCA GCCAATGCGG TCGTTTATTA TCCGATGAGT GCACGCCTGG  50
TCGTGGAAAA AAATAAAACC GTAAACAATG TCACTGCGGG TAAGTTTTCT  100
<210> 1660
<211> 100
<212> DNA
<213> Escherichia coli K-12 MG1655
<220> yadl b0138
<222> 396-495
<223> yadl b0138 U00096 complement(151626..152231)
<313> 1660: 460-479
<400> 1660
CTGGAGCACT GTAACTCCGG CAACAGGGAG TGCAACACAA TTTAGCTGTG  50
TAACACCAGC ATCACAAGAG GTAACAATCT CCACTGCAGC CAATGCGGTC  100
<210> 1661
<211> 100
<212> DNA
<213> Escherichia coli K-12 MG1655
<220> yadm b0139
<222> 513-612
<223> yadm b0139 U00096 complement(152243..152854)
<313> 1661: 538-557
<400> 1661
GGGTCTTGAA ATGACCGTTG CGCTACGTGA AACAGATGCA GGGCAAGGCG  50
TACCGGGGAA TTTTCGTGCA CTGGCGACGT TTAATTTCAT CTATCAATAA  100
<210> 1662
<211> 100
<212> DNA
<213> Escherichia coli K-12 MG1655
<220> yadm b0139
<222> 464-563
<223> yadm b0139 U00096 complement(152243..152854)
<313> 1662: 506-525
<400> 1662
ACAGTGCGGA AAAGATACGC TGGAGCACAG ACGAGATGCA GCCCGATAAG  50
GGTCTTGAAA TGACCGTTGC GCTACGTGAA ACAGATGCAG GGCAAGGCGT  100
<210> 1663
<211> 100
<212> DNA
<213> Escherichia coli K-12 MG1655
<220> yadm b0139
<222> 427-526
<223> yadm b0139 U00096 complement(152243..152854)
<313> 1663: 463-482
<400> 1663
AAACGGACTA CTGATGATGC CACTTTCCTT AAACCTAACA GTGCGGAAAA  50
GATACGCTGG AGCACAGACG AGATGCAGCC CGATAAGGGT CTTGAAATGA  100
<210> 1664
<211> 100
<212> DNA
<213> Escherichia coli K-12 MG1655
<220> b1422 b1422
<222> 966-1065
<223> b1422 b1422 U00096 complement(1492172..1493236)
<313> 1664: 1011-1030
<400> 1664
TGCAGGCGAA CCGATTGGAA TACTGACCCG CGTAGATGCG ACGTTCTCTT  50
CTGGTTGCCA GTTGATGATT AACGCTATTC GAAAATCAAT GCCGTTCTGA  100
<210> 1665
<211> 100
<212> DNA
<213> Escherichia coli K-12 MG1655
<220> b1422 b1422
<222> 798-897
<223> b1422 b1422 U00096 complement(1492172..1493236)
<313> 1665: 859-878
<400> 1665
GCAGAGCCAG GGCTGTAAAA TTCCTTCGGG TTGTATCGAA ACGCTGTCTG  50

CTTCGCTATC TCGTCAACTT ACGGTTGAAT ACGATTACGT GTGGTTTGTC 100
<210> 1666
<211> 100
<212> DNA
<213> Escherichia coli K-12 MG1655
<220> b1422 b1422
<222> 790-889
<223> b1422 b1422 U00096 complement(1492172..1493236)
<313> 1666: 810-829
<400> 1666
GCATTAGTGC AGAGCCAGGG CTGTAAAATT CCTTCGGGTT GTATCGAAAC 50
GCTGTCTGCT TCGCTATCTC GTCAACTTAC GGTTGAATAC GATTACGTGT 100
<210> 1667
<211> 100
<212> DNA
<213> Escherichia coli K-12 MG1655
<220> yadn b0142
<222> 475-574
<223> yadn b0142 U00096 complement(156299..156883)
<313> 1667: 532-551
<400> 1667
AAATCTGCTG TTTATGATTT TAAAGCGTCT TACGTTCGTG CCGTCGCAGA 50
CCAAACAGCA ACTGCTGGTT ATGTAAAAAC TAACACTGCA TACACCATTA 100
<210> 1668
<211> 100
<212> DNA
<213> Escherichia coli K-12 MG1655
<220> yadn b0142
<222> 239-338
<223> yadn b0142 U00096 complement(156299..156883)
<313> 1668: 299-318
<400> 1668
TCAGCATCAC CGTTGACTGC AGCAAAGCAA ATCCGAATCC AGGTAGCACG 50
GCTAAAATGA CCTTCGGTTC TGTTTTCTTT GGTAACAGCA AAGGGACTTT 100
<210> 1669
<211> 100
<212> DNA
<213> Escherichia coli K-12 MG1655
<220> yadn b0142
<222> 132-231
<223> yadn b0142 U00096 complement(156299..156883)
<313> 1669: 169-188
<400> 1669
CGTTGACGGC GGTAACAAGG ATGGTCTGAT CCTGCTGCAA ACCGCAACCG 50
TTGGTGAAAT CGATGCTGGC GTACTGAATG ACACCGTTGG CGCTAAAGCT 100
<210> 1670
<211> 100
<212> DNA
<213> Escherichia coli K-12 MG1655
<220> b1423 b1424
<222> 1245-1344
<223> b1423 b1424 U00096 1493312..1494655
<313> 1670: 1298-1317
<400> 1670
CGAAGCATTT GAGCAGGCGT TGGGTTGTCC GGTTTTGGAT GAGTTCCAGC 50
TTTACCAGGA AGCGGAAGAA CGCAGTAAAC GTCGCTGTGG TTTGCTTTAA 100
<210> 1671
<211> 100
<212> DNA
<213> Escherichia coli K-12 MG1655
<220> b1423 b1424
<222> 1234-1333
<223> b1423 b1424 U00096 1493312..1494655
<313> 1671: 1253-1272
<400> 1671
AATGCCAGTC GCGAAGCATT TGAGCAGGCG TTGGGTTGTC CGGTTTTGGA 50
TGAGTTCCAG CTTTACCAGG AAGCGGAAGA ACGCAGTAAA CGTCGCTGTG 100
<210> 1672
<211> 100
<212> DNA
<213> Escherichia coli K-12 MG1655

<220> b1423 b1424
<222> 1196-1295
<223> b1423 b1424 U00096 1493312..1494655
<313> 1672: 1208-1227
<400> 1672
CAAATCTGGG TAATGAAACG CAGACACGCA GTCACGGTAA TGCCAGTCGC  50
GAAGCATTTG AGCAGGCGTT GGGTTGTCCG GTTTTGGATG AGTTCCAGCT  100
<210> 1673
<211> 100
<212> DNA
<213> Escherichia coli K-12 MG1655
<220> yadp b0148
<222> 441-540
<223> yadp b0148 U00096 complement(161501..162040)
<313> 1673: 503-522
<400> 1673
CTGGTCGTAT GCGGTGACGG AGTTCACCCT TTACGCCTCC TCGTTTGCCC  50
GTGGACGCAC ACGCTACACG CCGCTAAAAC GCTGGGCGCT AACGCAATAA  100
<210> 1674
<211> 100
<212> DNA
<213> Escherichia coli K-12 MG1655
<220> yadp b0148
<222> 415-514
<223> yadp b0148 U00096 complement(161501..162040)
<313> 1674: 474-493
<400> 1674
GTGACAATCC CGCCGCCAGG TTTTAACTGG TCGTATGCGG TGACGGAGTT  50
CACCCTTTAC GCCTCCTCGT TTGCCCGTGG ACGCACACGC TACACGCCGC  100
<210> 1675
<211> 100
<212> DNA
<213> Escherichia coli K-12 MG1655
<220> yadp b0148
<222> 183-282
<223> yadp b0148 U00096 complement(161501..162040)
<313> 1675: 239-258
<400> 1675
GGCGCTTTCT CTTTTAGCCG GACGGATTCG TCAACCTGGT TTCACACTCA  50
CGCTTGATGA CGCCGGACAA TGGCTGCGTT CGCGTGTGGT GTGGTTAGGG  100
<210> 1676
<211> 100
<212> DNA
<213> Escherichia coli K-12 MG1655
<220> b1425 b1426
<222> 79-178
<223> b1425 b1426 U00096 complement(1496456..1496659)
<313> 1676: 119-138
<400> 1676
TTTAACCAGA CGGCACCTCC CGAAGAAGGT GCCGAAGAAA AAACGATTAA  50
CTCATCACGC GGTCGTCAAC ATACTGACGT TTATCCGGCG CTGGCGGGAA  100
<210> 1677
<211> 100
<212> DNA
<213> Escherichia coli K-12 MG1655
<220> b1425 b1426
<222> 53-152
<223> b1425 b1426 U00096 complement(1496456..1496659)
<313> 1677: 111-130
<400> 1677
TAGGGAAAAA TGCGAGCGGG ATCGTGTTTA ACCAGACGGC ACCTCCCGAA  50
GAAGGTGCCG AAGAAAAAAC GATTAACTCA TCACGCGGTC GTCAACATAC  100
<210> 1678
<211> 100
<212> DNA
<213> Escherichia coli K-12 MG1655
<220> b1425 b1426
<222> 22-121
<223> b1425 b1426 U00096 complement(1496456..1496659)
<313> 1678: 72-91
<400> 1678

C CATCAGATTA CTCATTTAAC TTAGGGAAAA ATGCGAGCGG 50
T AACCAGACGG CACCTCCCGA AGAAGGTGCC GAAGAAAAAA 100
9

<212> DNA
<213> Escherichia coli K-12 MG1655
<220> yadq b0156
<222> 1323-1422
<223> yadq b0156 U00096 175107..176528
<313> 1679: 1387-1406
<400> 1679
GAAACCGCTA TACTCGGCGA TTCTTGCGCG CACGCTGGCA AAACAGGAAG 50
CTGAGCAACT GGCGCGAAGC AAGGCCGCAT CAGCCAGCGA GAATACTTGA 100
<210> 1680
<211> 100
<212> DNA
<213> Escherichia coli K-12 MG1655
<220> yadq b0156
<222> 1262-1361
<223> yadq b0156 U00096 175107..176528
<313> 1680: 1324-1343
<400> 1680
TCATTTTGCC AATGATTATT ACCGGTCTTG GCGCAACACT ATTAGCGCAA 50
TTTACCGGCG GGAAACCGCT ATACTCGGCG ATTCTTGCGC GCACGCTGGC 100
<210> 1681
<211> 100
<212> DNA
<213> Escherichia coli K-12 MG1655
<220> yadq b0156
<222> 1216-1315
<223> yadq b0156 U00096 175107..176528
<313> 1681: 1249-1268
<400> 1681
TTAACGGGGA TCATTCTGGT TCTGGAGATG ACCGATAACT ACCAGCTCAT 50
TTTGCCAATG ATTATTACCG GTCTTGGCGC AACACTATTA GCGCAATTTA 100
<210> 1682
<211> 100
<212> DNA
<213> Escherichia coli K-12 MG1655
<220> clpp b0438
<222> 525-624
<223> clpp b0438 U00096 455901..456524
<313> 1682: 583-602
<400> 1682
AGAACAGATT GAACGTGATA CCGAGCGCGA TCGCTTCCTT TCCGCCCCTG 50
AAGCGGTGGA ATACGGTCTG GTCGATTCGA TTCTGACCCA TCGTAATTGA 100
<210> 1683
<211> 100
<212> DNA
<213> Escherichia coli K-12 MG1655
<220> clpp b0438
<222> 501-600
<223> clpp b0438 U00096 455901..456524
<313> 1683: 524-543
<400> 1683
GGCGCTTCAT ACGGGTCAAT CATTAGAACA GATTGAACGT GATACCGAGC 50
GCGATCGCTT CCTTTCCGCC CCTGAAGCGG TGGAATACGG TCTGGTCGAT 100
<210> 1684
<211> 100
<212> DNA
<213> Escherichia coli K-12 MG1655
<220> clpp b0438
<222> 434-533
<223> clpp b0438 U00096 455901..456524
<313> 1684: 477-496
<400> 1684
AGGCGACCGA TATCGAAATT CATGCCCGTG AAATTCTGAA AGTTAAAGGG 50
CGCATGAATG AACTTATGGC GCTTCATACG GGTCAATCAT TAGAACAGAT 100
<210> 1685
<211> 100
<212> DNA

<213> Escherichia coli K-12 MG1655
<220> yadr b0157
<222> 246-345
<223> yadr b0157 U00096 176610..176954
<313> 1685: 280-299
<400> 1685
TTCCGTTGAT TATACCGAAG GTCTGGAAGG TTCTCGTTTC ATCGTGACCA 50
ACCCGAACGC GAAAAGCACC TGCGGTTGCG GTTCTTCCTT TAGTATCTAA 100
<210> 1686
<211> 100
<212> DNA
<213> Escherichia coli K-12 MG1655
<220> yadr b0157
<222> 178-277
<223> yadr b0157 U00096 176610..176954
<313> 1686: 206-225
<400> 1686
ATGACCATCG AAAAACAGGG CGTTGGCCTG GTGGTTGATC CGATGAGCCT 50
GCAATATCTG GTCGGCGGTT CCGTTGATTA TACCGAAGGT CTGGAAGGTT 100
<210> 1687
<211> 100
<212> DNA
<213> Escherichia coli K-12 MG1655
<220> yadr b0157
<222> 140-239
<223> yadr b0157 U00096 176610..176954
<313> 1687: 169-188
<400> 1687
ATGGTTTCAC CTTTGATGAT CAGGTGAACG AAGGCGATAT GACCATCGAA 50
AAACAGGGCG TTGGCCTGGT GGTTGATCCG ATGAGCCTGC AATATCTGGT 100
<210> 1688
<211> 100
<212> DNA
<213> Escherichia coli K-12 MG1655
<220> yads b0158
<222> 525-624
<223> yads b0158 U00096 complement(177001..177624)
<313> 1688: 599-618
<400> 1688
CAGTATGATG GGCATGGTCG TGACGCTATT GATTCGGCTG GCGGCTATTC 50
GTTGGCATCT TAAGCTACCG ACGTTTGCGC TGGATGAGAA TGGGCGTTGA 100
<210> 1689
<211> 100
<212> DNA
<213> Escherichia coli K-12 MG1655
<220> yads b0158
<222> 472-571
<223> yads b0158 U00096 complement(177001..177624)
<313> 1689: 520-539
<400> 1689
GGTATTGTCC ACGCTACGGC TTATTACACA TTTTCCGTAC CACTGGAAAC 50
AGCCAGTATG ATGGGCATGG TCGTGACGCT ATTGATTCGG CTGGCGGCTA 100
<210> 1690
<211> 100
<212> DNA
<213> Escherichia coli K-12 MG1655
<220> yads b0158
<222> 403-502
<223> yads b0158 U00096 complement(177001..177624)
<313> 1690: 439-458
<400> 1690
GATGTTCTGG CCCGCGAAAT CCCCATGATT TTACGTACAG AAATCTACGC 50
AACTGCCTGT ATTATCGGCG GTATTGTCCA CGCTACGGCT TATTACACAT 100
<210> 1691
<211> 100
<212> DNA
<213> Escherichia coli K-12 MG1655
<220> b1428 b1429
<222> 882-981
<223> b1428 b1429 U00096 complement(1497493..1498473)
<313> 1691: 959-978

<400> 1691
CGTTGAAGCC TTTGATGGCG ACACGGTTCA TGTCCGTGGT CCAAAAGTCA 50
TTAACGGCGA GGAGCGCATT ACGCGAACGC CGCTGGCAGG GTTATTGTGA 100
<210> 1692
<211> 100
<212> DNA
<213> Escherichia coli K-12 MG1655
<220> b1428 b1429
<222> 882-981
<223> b1428 b1429 U00096 complement(1497493..1498473)
<313> 1692: 914-933
<400> 1692
CGTTGAAGCC TTTGATGGCG ACACGGTTCA TGTCCGTGGT CCAAAAGTCA 50
TTAACGGCGA GGAGCGCATT ACGCGAACGC CGCTGGCAGG GTTATTGTGA 100
<210> 1693
<211> 100
<212> DNA
<213> Escherichia coli K-12 MG1655
<220> b1428 b1429
<222> 778-877
<223> b1428 b1429 U00096 complement(1497493..1498473)
<313> 1693: 812-831
<400> 1693
GGCGGACCGA TTCTGCTCGA CGAGCACGTT GTCATACAAG GTGAGAGCCG 50
TATTACAGGT GCGGTGATAA TTGAAAACCA TGTGGAGTTA ACCGACCACG 100
<210> 1694
<211> 100
<212> DNA
<213> Escherichia coli K-12 MG1655
<220> yadt b0159
<222> 702-801
<223> yadt b0159 U00096 complement(177662..178462)
<313> 1694: 776-795
<400> 1694
TCCCGTTATT CCTCTCACGA GTGACTGGTT TGAACGTGCA AGCCCACGTA 50
TTATCCTCGC TGCACAACAG CTCTGTAATG CGCTTTCACA GGTAGATTAG 100
<210> 1695
<211> 100
<212> DNA
<213> Escherichia coli K-12 MG1655
<220> yadt b0159
<222> 660-759
<223> yadt b0159 U00096 complement(177662..178462)
<313> 1695: 703-722
<400> 1695
AATTCCTAAA ATCAAACAAT ACTGGGGTGA ACAGCTCAAA ATTCCCGTTA 50
TTCCTCTCAC GAGTGACTGG TTTGAACGTG CAAGCCCACG TATTATCCTC 100
<210> 1696
<211> 100
<212> DNA
<213> Escherichia coli K-12 MG1655
<220> yadt b0159
<222> 602-701
<223> yadt b0159 U00096 complement(177662..178462)
<313> 1696: 660-679
<400> 1696
GCGAACAGGT GTTAGCACGC TCGCCACAGG CGATTGTCAT TACAGGCGGA 50
CCGGACCAAA TTCCTAAAAT CAAACAATAC TGGGGTGAAC AGCTCAAAAT 100
<210> 1697
<211> 100
<212> DNA
<213> Escherichia coli K-12 MG1655
<220> ypfh b2474
<222> 624-723
<223> ypfh b2474 U00096 complement(2591092..2591814)
<313> 1697: 685-704
<400> 1697
TGCCCTGGAT CACTTGCGTT ATACCATTCC GAAACATTAT TTCGACGAAG 50
CGCTAAGTGG CGGTAAGCCT GGCGATGATG ATGTGATTGA AATGATGTAA 100
<210> 1698
<211> 100

<212> DNA
<213> Escherichia coli K-12 MG1655
<220> ypfh b2474
<222> 608-707
<223> ypfh b2474 U00096 complement(2591092..2591814)
<313> 1698: 635-654
<400> 1698
ATCGCAGTAT GCAATTTGCC CTGGATCACT TGCGTTATAC CATTCCGAAA 50
CATTATTTCG ACGAAGCGCT AAGTGGCGGT AAGCCTGGCG ATGATGATGT 100
<210> 1699
<211> 100
<212> DNA
<213> Escherichia coli K-12 MG1655
<220> ypfh b2474
<222> 540-639
<223> ypfh b2474 U00096 complement(2591092..2591814)
<313> 1699: 562-581
<400> 1699
AGCGTTAATC AGTGCCGGTG GTGACGTGAC GCTGGATATC GTGGAGGATT 50
TAGGCCATGC GATCGACAAT CGCAGTATGC AATTTGCCCT GGATCACTTG 100
<210> 1700
<211> 100
<212> DNA
<213> Escherichia coli K-12 MG1655
<220> clpx b0439
<222> 1176-1275
<223> clpx b0439 U00096 456650..457924
<313> 1700: 1255-1274
<400> 1700
CGTCGAAAAA GTGGTTATCG ACGAGTCGGT AATTGATGGT CAAAGCAAAC 50
CGTTGCTGAT TTATGGCAAG CCGGAAGCGC AACAGGCATC TGGTGAATAA 100
<210> 1701
<211> 100
<212> DNA
<213> Escherichia coli K-12 MG1655
<220> clpx b0439
<222> 1089-1188
<223> clpx b0439 U00096 456650..457924
<313> 1701: 1132-1151
<400> 1701
GGCGCGTAAA ACCGGTGCCC GTGGCCTGCG TTCCATCGTA GAAGCCGCAC 50
TGCTCGATAC CATGTACGAT CTGCCGTCCA TGGAAGACGT CGAAAAAGTG 100
<210> 1702
<211> 100
<212> DNA
<213> Escherichia coli K-12 MG1655
<220> clpx b0439
<222> 1029-1128
<223> clpx b0439 U00096 456650..457924
<313> 1702: 1050-1069
<400> 1702
GGAAGGCGTG GATCTGGAAT TCCGTGACGA GGCGCTGGAT GCTATCGCTA 50
AGAAAGCGAT GGCGCGTAAA ACCGGTGCCC GTGGCCTGCG TTCCATCGTA 100
<210> 1703
<211> 100
<212> DNA
<213> Escherichia coli K-12 MG1655
<220> ypfi ypfI
<222> 1892-1991
<223> ypfi ypfI U00096 complement(2591864..2593879)
<313> 1703: 1951-1970
<400> 1703
TTTCAGGCCG CAAGATGTTA CTGGTCCGTC AGCGGGAAGA GGCCGCGCAG 50
GCGCTGTTCG CACTTAATGA TGTTCGCACT GAGCGTCTGC GCGATCGCAT 100
<210> 1704
<211> 100
<212> DNA
<213> Escherichia coli K-12 MG1655
<220> ypfi ypfI
<222> 1884-1983
<223> ypfi ypfI U00096 complement(2591864..2593879)

<313> 1704: 1896-1915
<400> 1704
ACTTAAACTT TCAGGCCGCA AGATGTTACT GGTCCGTCAG CGGGAAGAGG 50
CCGCGCAGGC GCTGTTCGCA CTTAATGATG TTCGCACTGA GCGTCTGCGC 100
<210> 1705
<211> 100
<212> DNA
<213> Escherichia coli K-12 MG1655
<220> ypfi ypfI
<222> 1778-1877
<223> ypfi ypfI U00096 complement(2591864..2593879)
<313> 1705: 1812-1831
<400> 1705
CGTCGTTAGG TTGCTTATTG CGTCTGTTAC AAACCAGTGA ACTGGCATTA 50
CCGGCGCTGC GTGGGCGTTT ACAGAAAAAC GCCAGTGATG CGCAGTTATG 100
<210> 1706
<211> 100
<212> DNA
<213> Escherichia coli K-12 MG1655
<220> hepa b0060
<222> 2783-2882
<223> hepa b0060 U00096 complement(60358..63264)
<313> 1706: 2826-2845
<400> 1706
ACCCGAACAT TCGTGACGAC GAACTGACCG CCATTGAGAG CAACCGTCAG 50
CAGGTAATGG AAAGCCTGGA TCAGGCAGGT TGGCGTCTGG ATGCCCTGCG 100
<210> 1707
<211> 100
<212> DNA
<213> Escherichia coli K-12 MG1655
<220> hepa b0060
<222> 2731-2830
<223> hepa b0060 U00096 complement(60358..63264)
<313> 1707: 2777-2796
<400> 1707
GACGAAAAAC TGTCTGCCGA GCTGTCTCGT CTGGAAGCTC TGCGTGCAGT 50
GAACCCGAAC ATTCGTGACG ACGAACTGAC CGCCATTGAG AGCAACCGTC 100
<210> 1708
<211> 100
<212> DNA
<213> Escherichia coli K-12 MG1655
<220> hepa b0060
<222> 2712-2811
<223> hepa b0060 U00096 complement(60358..63264)
<313> 1708: 2746-2765
<400> 1708
TGCAGCGCGT AACGAAGCCG ACGAAAAACT GTCTGCCGAG CTGTCTCGTC 50
TGGAAGCTCT GCGTGCAGTG AACCCGAACA TTCGTGACGA CGAACTGACC 100
<210> 1709
<211> 100
<212> DNA
<213> Escherichia coli K-12 MG1655
<220> yaeb b0196
<222> 602-701
<223> yaeb b0196 U00096 complement(218887..219594)
<313> 1709: 637-656
<400> 1709
AAGGTGAGGA AACGGGCAAA ACCTATGCCG TCTGGCTACA TGATTTTAAC 50
GTTCGCTGGC GCGTCACCGA CGCAGGTTTT GAAGTCTTTG CGCTGGAACC 100
<210> 1710
<211> 100
<212> DNA
<213> Escherichia coli K-12 MG1655
<220> yaeb b0196
<222> 530-629
<223> yaeb b0196 U00096 complement(218887..219594)
<313> 1710: 588-607
<400> 1710
AGAAGCGTTA TCCGCAGTTA ACGCTGTTTA TCCGCGAAGT ACTGGCGCAG 50
GACCCGCGCC CGGCCTATCG TAAAGGTGAG GAAACGGGCA AAACCTATGC 100
<210> 1711

<211> 100
<212> DNA
<213> Escherichia coli K-12 MG1655
<220> yaeb b0196
<222> 501-600
<223> yaeb b0196 U00096 complement(218887..219594)
<313> 1711: 556-575
<400> 1711
AGAGGTCGAA AAGCAGCTTT TGACGCTGGA GAAGCGTTAT CCGCAGTTAA 50
CGCTGTTTAT CCGCGAAGTA CTGGCGCAGG ACCCGCGCCC GGCCTATCGT 100
<210> 1712
<211> 100
<212> DNA
<213> Escherichia coli K-12 MG1655
<220> yaec b0198
<222> 717-816
<223> yaec b0198 U00096 complement(220113..220928)
<313> 1712: 767-786
<400> 1712
CGCCGAGAAC GTGAAGAAAT TCGTCCAGGC TTATCAGTCT GACGAAGTTT 50
ACGAAGCAGC AAACAAAGTG TTTAACGGCG GAGCTGTTAA AGGCTGGTAA 100
<210> 1713
<211> 100
<212> DNA
<213> Escherichia coli K-12 MG1655
<220> yaec b0198
<222> 672-771
<223> yaec b0198 U00096 complement(220113..220928)
<313> 1713: 726-745
<400> 1713
GTCCCCGTAC GTAAACCTGA TCGTGACGCG TGAAGATAAC AAAGACGCCG 50
AGAACGTGAA GAAATTCGTC CAGGCTTATC AGTCTGACGA AGTTTACGAA 100
<210> 1714
<211> 100
<212> DNA
<213> Escherichia coli K-12 MG1655
<220> yaec b0198
<222> 586-685
<223> yaec b0198 U00096 complement(220113..220928)
<313> 1714: 642-661
<400> 1714
ATCGCTCTGG CAGTTATCAA TACCACCTAT GCCAGCCAGA TTGGCCTGAC 50
TCCGGCGAAA GACGGTATCT TTGTTGAAGA TAAAGAGTCC CCGTACGTAA 100
<210> 1715
<211> 100
<212> DNA
<213> Escherichia coli K-12 MG1655
<220> ggt b3448
<222> 1644-1743
<223> ggt b3448 U00096 complement(3582712..3584454)
<313> 1715: 1708-1727
<400> 1715
GGCGATGGGC AGTACACAAA GCATTATGGT TGGGCCGGAC GGTGAGTTGT 50
ACGGCGCATC CGACCCGCGC TCGGTGGATG ATTTAACGGC GGGGTACTAA 100
<210> 1716
<211> 100
<212> DNA
<213> Escherichia coli K-12 MG1655
<220> ggt b3448
<222> 1610-1709
<223> ggt b3448 U00096 complement(3582712..3584454)
<313> 1716: 1656-1675
<400> 1716
TGGAAGCAAA AGGTCAGAAA GTGGCGCTGA AAGAGGCGAT GGGCAGTACA 50
CAAAGCATTA TGGTTGGGCC GGACGGTGAG TTGTACGGCG CATCCGACCC 100
<210> 1717
<211> 100
<212> DNA
<213> Escherichia coli K-12 MG1655
<220> ggt b3448
<222> 1577-1676

<223> ggt b3448 U00096 complement(3582712..3584454)
<313> 1717: 1606-1625
<400> 1717
AAAAAGGGTT TAGCCCGGAT ACGCTCAAGC TGCTGGAAGC AAAAGGTCAG 50
AAAGTGGCGC TGAAAGAGGC GATGGGCAGT ACACAAAGCA TTATGGTTGG 100
<210> 1718
<211> 100
<212> DNA
<213> Escherichia coli K-12 MG1655
<220> yaed b0201
<222> 477-576
<223> yaed b0201 U00096 222833..223408
<313> 1718: 554-573
<400> 1718
ACCTATTACG CCTGAAGCAG AAAACGCGGC GGATTGGGTG TTAAATAGCC 50
TGGCAGACCT GCCGCAAGCG ATAAAAAAGC AGCAAAAACC GGCACAATGA 100
<210> 1719
<211> 100
<212> DNA
<213> Escherichia coli K-12 MG1655
<220> yaed b0201
<222> 466-565
<223> yaed b0201 U00096 222833..223408
<313> 1719: 500-519
<400> 1719
CGTACGGGTA AACCTATTAC GCCTGAAGCA GAAAACGCGG CGGATTGGGT 50
GTTAAATAGC CTGGCAGACC TGCCGCAAGC GATAAAAAAG CAGCAAAAAC 100
<210> 1720
<211> 100
<212> DNA
<213> Escherichia coli K-12 MG1655
<220> yaed b0201
<222> 378-477
<223> yaed b0201 U00096 222833..223408
<313> 1720: 414-433
<400> 1720
TGATATGGCC GCTTCTTATA TGGTGGGCGA TAAATTAGAA GATATGCAGG 50
CAGCGGTTGC GGCGAACGTG GGAACAAAAG TGCTGGTGCG TACGGGTAAA 100
<210> 1721
<211> 100
<212> DNA
<213> Escherichia coli K-12 MG1655
<220> yaee b0199
<222> 555-654
<223> yaee b0199 U00096 complement(220968..221621)
<313> 1721: 601-620
<400> 1721
CGCGACGGTG ATGAATACGG TACTGGTATT GCTGGTCATT CTGGTTTATT 50
TAATTCAGTT CGCAGGCGAC CGCATCGTCC GGGCTGTCAC TCGCAAGTAA 100
<210> 1722
<211> 100
<212> DNA
<213> Escherichia coli K-12 MG1655
<220> yaee b0199
<222> 506-605
<223> yaee b0199 U00096 complement(220968..221621)
<313> 1722: 548-567
<400> 1722
CCGGTGGTTT AGGTCAGATT GGCTATCAGT ATGGCTACAT CGGCTATAAC 50
GCGACGGTGA TGAATACGGT ACTGGTATTG CTGGTCATTC TGGTTTATTT 100
<210> 1723
<211> 100
<212> DNA
<213> Escherichia coli K-12 MG1655
<220> yaee b0199
<222> 399-498
<223> yaee b0199 U00096 complement(220968..221621)
<313> 1723: 439-458
<400> 1723
CGTCCGTAAG GTGCTGTTAC CGGAAGCGCT GCCGGGTCTG GTGAATGCGG 50
CAACTATCAC CCTGATTACC CTGGTCGGTT ATTCCGCGAT GGGTGGTGCA 100

<210> 1724
<211> 100
<212> DNA
<213> Escherichia coli K-12 MG1655
<220> yaef b0194
<222> 770-869
<223> yaef b0194 U00096 complement(216179..217057)
<313> 1724: 834-853
<400> 1724
GTATTGGTGA TGTCTCTGCG TTTAAGCCAG AAACGCAGTT ACAGTATGTT 50
GGGCACCTGA AGCCTGGGAT TTATATCAAG GCGGGTCGCT TTGTTGGTTT 100
<210> 1725
<211> 100
<212> DNA
<213> Escherichia coli K-12 MG1655
<220> yaef b0194
<222> 684-783
<223> yaef b0194 U00096 complement(216179..217057)
<313> 1725: 714-733
<400> 1725
TGTCACGGAT GCCTTTGCCA AAGCCGGGCA TCCACTTACA CTGGCGCAAT 50
CGGGCTGGAT TAGCCCTGCC GATCTGATGC ATATGCGTAT TGGTGATGTC 100
<210> 1726
<211> 100
<212> DNA
<213> Escherichia coli K-12 MG1655
<220> yaef b0194
<222> 616-715
<223> yaef b0194 U00096 complement(216179..217057)
<313> 1726: 676-695
<400> 1726
GGCCTGGCGA AAGCGCAGTT AAGCAGTGTG GGCGAAGGAG ACAAAAAGTC 50
GTGGTTTTGT TCGGAATTTG TCACGGATGC CTTTGCCAAA GCCGGGCATC 100
<210> 1727
<211> 100
<212> DNA
<213> Escherichia coli K-12 MG1655
<220> nrda b2235
<222> 2187-2286
<223> nrda b2235 U00096 2342885..2345170
<313> 1727: 2254-2273
<400> 1727
GTATTATCAG AACACCCGTG ACGGCGCTGA AGACGCACAA GACGATCTGG 50
TGCCGTCAAT CCAGGACGAT GGCTGCGAAA GCGGCGCATG TAAGATCTGA 100
<210> 1728
<211> 100
<212> DNA
<213> Escherichia coli K-12 MG1655
<220> nrda b2235
<222> 2137-2236
<223> nrda b2235 U00096 2342885..2345170
<313> 1728: 2193-2212
<400> 1728
CAGTTGCTGA AAGACCTGCT CACCGCCTAC AAATTCGGGG TCAAAACACT 50
GTATTATCAG AACACCCGTG ACGGCGCTGA AGACGCACAA GACGATCTGG 100
<210> 1729
<211> 100
<212> DNA
<213> Escherichia coli K-12 MG1655
<220> nrda b2235
<222> 2074-2173
<223> nrda b2235 U00096 2342885..2345170
<313> 1729: 2109-2128
<400> 1729
TCGATCTCTG CCAACACCAA CTACGATCCG TCACGCTTCC CGTCAGGAAA 50
AGTGCCGATG CAGCAGTTGC TGAAAGACCT GCTCACCGCC TACAAATTCG 100
<210> 1730
<211> 100
<212> DNA
<213> Escherichia coli K-12 MG1655
<220> yaeg b0163

<222> 1052-1151
<223> yaeg b0163 U00096 182445..183620
<313> 1730: 1071-1090
<400> 1730
TTATTCATCG TAATACCCTG GAGTATCGGC TTAATCGTAT ATCGGAACTG 50
ACCGGGCTTG ATTTGGGCAA TTTTGATGAC AGGTTGCTGC TGTATGTGGC 100
<210> 1731
<211> 100
<212> DNA
<213> Escherichia coli K-12 MG1655
<220> yaeg b0163
<222> 963-1062
<223> yaeg b0163 U00096 182445..183620
<313> 1731: 1012-1031
<400> 1731
AACGATGGAC AATAACGGCT TGCTGCGACG AACGCTGGCG GCGTGGTTTC 50
GCCACAATGT GCAACCGCTG GCAACGTCAA AGGCGTTGTT TATTCATCGT 100
<210> 1732
<211> 100
<212> DNA
<213> Escherichia coli K-12 MG1655
<220> yaeg b0163
<222> 913-1012
<223> yaeg b0163 U00096 182445..183620
<313> 1732: 966-985
<400> 1732
CGTGGCGACT GGCAGGCCAA CGAACTGGCG CGACCGCTGG CGCGGCTGAA 50
AACGATGGAC AATAACGGCT TGCTGCGACG AACGCTGGCG GCGTGGTTTC 100
<210> 1733
<211> 100
<212> DNA
<213> Escherichia coli K-12 MG1655
<220> nrdb b2236
<222> 1032-1131
<223> nrdb b2236 U00096 2345404..2346534
<313> 1733: 1093-1112
<400> 1733
CGTGCAGGTT GCTCCGCAGG AAGTGGAAGT CAGTTCTTAT CTGGTCGGGC 50
AGATTGACTC GGAAGTGGAC ACCGACGATT TGAGTAACTT CCAGCTCTGA 100
<210> 1734
<211> 100
<212> DNA
<213> Escherichia coli K-12 MG1655
<220> nrdb b2236
<222> 1013-1112
<223> nrdb b2236 U00096 2345404..2346534
<313> 1734: 1047-1066
<400> 1734
CTTGGCTGGT GTCTGATAAC GTGCAGGTTG CTCCGCAGGA AGTGGAAGTC 50
AGTTCTTATC TGGTCGGGCA GATTGACTCG GAAGTGGACA CCGACGATTT 100
<210> 1735
<211> 100
<212> DNA
<213> Escherichia coli K-12 MG1655
<220> nrdb b2236
<222> 962-1061
<223> nrdb b2236 U00096 2345404..2346534
<313> 1735: 1013-1032
<400> 1735
GTTTGGATCT GCCGTTCCAG ACGCGCTCCA ACCCGATCCC GTGGATCAAC 50
ACTTGGCTGG TGTCTGATAA CGTGCAGGTT GCTCCGCAGG AAGTGGAAGT 100
<210> 1736
<211> 100
<212> DNA
<213> Escherichia coli K-12 MG1655
<220> yaeh b0164
<222> 288-387
<223> yaeh b0164 U00096 complement(183709..184095)
<313> 1736: 333-352
<400> 1736
TAAGCGTAAG ATCCTCGACG ACTTACGTCA CCTGGAGTCA GTCGTAACCA 50

ATAAGATCAG CGAGATTGAA GCCGATCTGG AAAAACTAAC GCGTAAATAA 100
<210> 1737
<211> 100
<212> DNA
<213> Escherichia coli K-12 MG1655
<220> yaeh b0164
<222> 209-308
<223> yaeh b0164 U00096 complement(183709..184095)
<313> 1737: 277-296
<400> 1737
AGGAAATCAG CCCGAATCTA CGGTATATCA TTGATGAGCT TGATCAAATC 50
TGCCAGCGTG ACCGCAGCGA AGTTGATCTT AAGCGTAAGA TCCTCGACGA 100
<210> 1738
<211> 100
<212> DNA
<213> Escherichia coli K-12 MG1655
<220> yaeh b0164
<222> 173-272
<223> yaeh b0164 U00096 complement(183709..184095)
<313> 1738: 192-211
<400> 1738
TCGCTGATGG TGTGGGTCAG GGTTATAAAG AAGTCCAGGA AATCAGCCCG 50
AATCTACGGT ATATCATTGA TGAGCTTGAT CAAATCTGCC AGCGTGACCG 100
<210> 1739
<211> 100
<212> DNA
<213> Escherichia coli K-12 MG1655
<220> yaei yaeI
<222> 645-744
<223> yaei yaeI U00096 complement(184257..185000)
<313> 1739: 716-735
<400> 1739
CTTTGGCGAA AGACACATCT ACACAACCCG TGGCGTGGGC AGTTTGTATG 50
GTTTGCGTCT GAACTGCCGC CCGGAAGTAA CGATGCTGGA ACTGGTGTAG 100
<210> 1740
<211> 100
<212> DNA
<213> Escherichia coli K-12 MG1655
<220> yaei yaeI
<222> 638-737
<223> yaei yaeI U00096 complement(184257..185000)
<313> 1740: 664-683
<400> 1740
TAAATGCCTT TGGCGAAAGA CACATCTACA CAACCCGTGG CGTGGGCAGT 50
TTGTATGGTT TGCGTCTGAA CTGCCGCCCG GAAGTAACGA TGCTGGAACT 100
<210> 1741
<211> 100
<212> DNA
<213> Escherichia coli K-12 MG1655
<220> yaei yaeI
<222> 570-669
<223> yaei yaeI U00096 complement(184257..185000)
<313> 1741: 625-644
<400> 1741
GCAACTGCGC GTACCGTTGG TTGGTGAACC TTTTGCCCCT GTCGAAGATA 50
AACGTTACGT CGCCGGATTA AATGCCTTTG GCGAAAGACA CATCTACACA 100
<210> 1742
<211> 100
<212> DNA
<213> Escherichia coli K-12 MG1655
<220> nrdd b4239
<222> 2040-2139
<223> nrdd b4239 U00096 complement(4458096..4460234)
<313> 1742: 2111-2130
<400> 1742
CGGATATTTA GGTAGCCCGG ATGCACGTCC GTTTAACGCT GGTAAGCAGG 50
AAGAAGTTAA GCGCCGCGTT AAACATTTGG GGAATGGGCA GATAGGTTAA 100
<210> 1743
<211> 100
<212> DNA
<213> Escherichia coli K-12 MG1655

<220> nrdd b4239
<222> 2034-2133
<223> nrdd b4239 U00096 complement(4458096..4460234)
<313> 1743: 2064-2083
<400> 1743
CGTGTGCGGA TATTTAGGTA GCCCGGATGC ACGTCCGTTT AACGCTGGTA  50
AGCAGGAAGA AGTTAAGCGC CGCGTTAAAC ATTTGGGGAA TGGGCAGATA  100
<210> 1744
<211> 100
<212> DNA
<213> Escherichia coli K-12 MG1655
<220> nrdd b4239
<222> 1948-2047
<223> nrdd b4239 U00096 complement(4458096..4460234)
<313> 1744: 2006-2025
<400> 1744
ACCGGTGAGT TCGAGTGCAC CAGCAAAGGC TTCACTTGCC CGAAATGTGG  50
TAACCATGAC GCCTCCCGTG TGTCGGTAAC TCGCCGCGTG TGCGGATATT  100
<210> 1745
<211> 100
<212> DNA
<213> Escherichia coli K-12 MG1655
<220> yaej b0192
<222> 318-417
<223> yaej b0192 U00096 214833..215255
<313> 1745: 383-402
<400> 1745
ACCCACGCGG CCCACCCGTG CATCGAAAGA GCGCAGGCTG GCATCGAAAG  50
CACAAAAATC AAGCGTGAAG GCGATGCGCG GCAAAGTGCG CAGCGGTCGG  100
<210> 1746
<211> 100
<212> DNA
<213> Escherichia coli K-12 MG1655
<220> yaej b0192
<222> 280-379
<223> yaej b0192 U00096 214833..215255
<313> 1746: 337-356
<400> 1746
ATGATTAAAG AATTAACAAC AGAAAAAAAA GCCCGACGAC CCACGCGGCC  50
CACCCGTGCA TCGAAAGAGC GCAGGCTGGC ATCGAAAGCA CAAAAATCAA  100
<210> 1747
<211> 100
<212> DNA
<213> Escherichia coli K-12 MG1655
<220> yaej b0192
<222> 274-373
<223> yaej b0192 U00096 214833..215255
<313> 1747: 287-306
<400> 1747
GTGGCTATGA TTAAAGAATT AACAACAGAA AAAAAAGCCC GACGACCCAC  50
GCGGCCCACC CGTGCATCGA AAGAGCGCAG GCTGGCATCG AAAGCACAAA  100
<210> 1748
<211> 100
<212> DNA
<213> Escherichia coli K-12 MG1655
<220> nrde b2676
<222> 2046-2145
<223> nrde b2676 U00096 2799369..2801513
<313> 1748: 2114-2133
<400> 1748
CGCCTGGCGC AAGGGTATCA AAACGCTCTA TTACATCCGC CTGCGTCAGA  50
TGGCGCTGGA AGGCACTGAA ATTGAAGGCT GCGTCTCCTG TGCACTTTAA  100
<210> 1749
<211> 100
<212> DNA
<213> Escherichia coli K-12 MG1655
<220> nrde b2676
<222> 2021-2120
<223> nrde b2676 U00096 2799369..2801513
<313> 1749: 2061-2080
<400> 1749

GCGATATCAA CAAAGCGCAG ATTTACGCCT GGCGCAAGGG TATCAAAACG 50
CTCTATTACA TCCGCCTGCG TCAGATGGCG CTGGAAGGCA CTGAAATTGA 100
<210> 1750
<211> 100
<212> DNA
<213> Escherichia coli K-12 MG1655
<220> nrde b2676
<222> 1939-2038
<223> nrde b2676 U00096 2799369..2801513
<313> 1750: 1997-2016
<400> 1750
ATCGACACCT ACGCGGAAGC GACTCGCCAT GTCGATCAGG GGCTGTCGCT 50
GACGCTTTTT TTCCCCGATA CCGCCACCAC TCGCGATATC AACAAAGCGC 100
<210> 1751
<211> 100
<212> DNA
<213> Escherichia coli K-12 MG1655
<220> nrdf b2677
<222> 861-960
<223> nrdf b2677 U00096 2801523..2802482
<313> 1751: 933-952
<400> 1751
GCCGAATGCC GATGAAAATC ACGATTTCTT TTCCGGTTCA GGCTCCTCTT 50
ATGTGATGGG GAAAGCGGTT GAAACAGAAG ATGAAGACTG GAATTTCTGA 100
<210> 1752
<211> 100
<212> DNA
<213> Escherichia coli K-12 MG1655
<220> nrdf b2677
<222> 831-930
<223> nrdf b2677 U00096 2801523..2802482
<313> 1752: 890-909
<400> 1752
AGTGAATCCG GCAATCCTCG CCGCGCTTTC GCCGAATGCC GATGAAAATC 50
ACGATTTCTT TTCCGGTTCA GGCTCCTCTT ATGTGATGGG GAAAGCGGTT 100
<210> 1753
<211> 100
<212> DNA
<213> Escherichia coli K-12 MG1655
<220> nrdf b2677
<222> 812-911
<223> nrdf b2677 U00096 2801523..2802482
<313> 1753: 831-850
<400> 1753
TTCCCGCAGA AATGGCGGAA GTGAATCCGG CAATCCTCGC CGCGCTTTCG 50
CCGAATGCCG ATGAAAATCA CGATTTCTTT TCCGGTTCAG GCTCCTCTTA 100
<210> 1754
<211> 100
<212> DNA
<213> Escherichia coli K-12 MG1655
<220> b0701 b0702
<222> 123-222
<223> b0701 b0702 U00096 732593..732814
<313> 1754: 180-199
<400> 1754
GGTCTATGGG GGCCAGGTAA AGCAAGTAAT GATACAAAAG AAGCTGCTGG 50
CCGTTGTGAC CTCGTTGAAA CCGATAATAG TTGTGTGGAG AACTGTTTAA 100
<210> 1755
<211> 100
<212> DNA
<213> Escherichia coli K-12 MG1655
<220> b0701 b0702
<222> 98-197
<223> b0701 b0702 U00096 732593..732814
<313> 1755: 136-155
<400> 1755
CCAACGGGGA GAATCAAAAG GAGATGGTCT ATGGGGGCCA GGTAAAGCAA 50
GTAATGATAC AAAAGAAGCT GCTGGCCGTT GTGACCTCGT TGAAACCGAT 100
<210> 1756
<211> 100
<212> DNA

<213> Escherichia coli K-12 MG1655
<220> b0701 b0702
<222> 4-103
<223> b0701 b0702 U00096 732593..732814
<313> 1756: 54-73
<400> 1756
AAAGAAGCGG TCCAGATATA TGGGGGAATC CGTTCTATCA TCAATATCTT 50
TGTGTCCCAG ATGGTAAAGG GGACTATACT TGTGGTGGCC AAGACCAACG 100
<210> 1757
<211> 100
<212> DNA
<213> Escherichia coli K-12 MG1655
<220> yael b0177
<222> 1229-1328
<223> yael b0177 U00096 196546..197898
<313> 1757: 1272-1291
<400> 1757
CGATCGAAAA GATCAAGGGC GGACCGGTAT CCGAGCGGGT TCAAGACTTT 50
TGTTATCGCA TTGGCTCGAT TCTGCTGGTG CTGTTAATGG GGCTTGCACT 100
<210> 1758
<211> 100
<212> DNA
<213> Escherichia coli K-12 MG1655
<220> yael b0177
<222> 1147-1246
<223> yael b0177 U00096 196546..197898
<313> 1758: 1178-1197
<400> 1758
CTTGCGCTTA TTAGCGTGAA CTTAGGGATA ATTAACCTGT TTCCGTTGCC 50
CGTACTTGAC GGGGGGCATC TGCTGTTCCT TGCGATCGAA AAGATCAAGG 100
<210> 1759
<211> 100
<212> DNA
<213> Escherichia coli K-12 MG1655
<220> yael b0177
<222> 1098-1197
<223> yael b0177 U00096 196546..197898
<313> 1759: 1133-1152
<400> 1759
GGGGGCTGGG ATGACAGCGG AACTCGGGGT TGTTTATTAC CTGCCGTTTC 50
TTGCGCTTAT TAGCGTGAAC TTAGGGATAA TTAACCTGTT TCCGTTGCCC 100
<210> 1760
<211> 100
<212> DNA
<213> Escherichia coli K-12 MG1655
<220> nrdg b4238
<222> 366-465
<223> nrdg b4238 U00096 complement(4457474..4457938)
<313> 1760: 413-432
<400> 1760
GATTAACGTG CTGGTCGACG GCAAATTTGT GCAGGATTTA AAAGACCCTT 50
CCCTGATCTG GCGCGGCAGC AGCAATCAGG TGGTGCATCA TTTGCGATGA 100
<210> 1761
<211> 100
<212> DNA
<213> Escherichia coli K-12 MG1655
<220> nrdg b4238
<222> 287-386
<223> nrdg b4238 U00096 complement(4457474..4457938)
<313> 1761: 323-342
<400> 1761
GTCCGGGTAA AGACATCTGG GTGTGGACAG GCTATAAACT CGACGAACTC 50
AACGCTGCGC AAATGCAGGT TGTTGATTTG ATTAACGTGC TGGTCGACGG 100
<210> 1762
<211> 100
<212> DNA
<213> Escherichia coli K-12 MG1655
<220> nrdg b4238
<222> 213-312
<223> nrdg b4238 U00096 complement(4457474..4457938)
<313> 1762: 255-274

<400> 1762
CTCCGGCGGC GATCCGCTGC ATCCGCAAAA CGTGCCGGAT ATTCTGAAAC 50
TGGTACAACG CATCCGCGCC GAGTGTCCGG GTAAAGACAT CTGGGTGTGG 100
<210> 1763
<211> 100
<212> DNA
<213> Escherichia coli K-12 MG1655
<220> b1431 b1431
<222> 516-615
<223> b1431 b1431 U00096 1500481..1501149
<313> 1763: 560-579
<400> 1763
TGCAGCGACT AATAAACCGG TTATCAAAGT CGTTCGTCAG GGCGAAGGTA 50
AAGACCTGAA TAACGAAAGT ACGCCAATGG CTTTCGAAAA TATTAAACAA 100
<210> 1764
<211> 100
<212> DNA
<213> Escherichia coli K-12 MG1655
<220> b1431 b1431
<222> 453-552
<223> b1431 b1431 U00096 1500481..1501149
<313> 1764: 490-509
<400> 1764
GCAAATGGCT ACAGGCCACC GTACCATGGA TACTCGCCTC TATTTTGAAG 50
GTGAGCTGAT TGATGCAGCG ACTAATAAAC CGGTTATCAA AGTCGTTCGT 100
<210> 1765
<211> 100
<212> DNA
<213> Escherichia coli K-12 MG1655
<220> b1431 b1431
<222> 408-507
<223> b1431 b1431 U00096 1500481..1501149
<313> 1765: 445-464
<400> 1765
GCAATTCTAT GAAGTGGTTC CTGTTGCATT AGTGGTTGCG GGGACGCAAA 50
TGGCTACAGG CCACCGTACC ATGGATACTC GCCTCTATTT TGAAGGTGAG 100
<210> 1766
<211> 100
<212> DNA
<213> Escherichia coli K-12 MG1655
<220> yaem b0174
<222> 1095-1194
<223> yaem b0174 U00096 193521..194717
<313> 1766: 1127-1146
<400> 1766
AAAAATGGAT ATGCGCGAAC CACAATGTGT GGACGATGTG TTATCTGTTG 50
ATGCGAACGC GCGTGAAGTC GCCAGAAAAG AGGTGATGCG TCTCGCAAGC 100
<210> 1767
<211> 100
<212> DNA
<213> Escherichia coli K-12 MG1655
<220> yaem b0174
<222> 1011-1110
<223> yaem b0174 U00096 193521..194717
<313> 1767: 1056-1075
<400> 1767
CGCAAACGAA ATCACCGTTG CTGCTTTTCT TGCGCAACAA ATCCGCTTTA 50
CGGATATCGC TGCGTTGAAT TTATCCGTAC TGGAAAAAAT GGATATGCGC 100
<210> 1768
<211> 100
<212> DNA
<213> Escherichia coli K-12 MG1655
<220> yaem b0174
<222> 973-1072
<223> yaem b0174 U00096 193521..194717
<313> 1768: 1007-1026
<400> 1768
TTCGAACAAG GCCAGGCAGC GACGACAGCA TTGAATGCCG CAAACGAAAT 50
CACCGTTGCT GCTTTTCTTG CGCAACAAAT CCGCTTTACG GATATCGCTG 100
<210> 1769
<211> 100

<212> DNA
<213> Escherichia coli K-12 MG1655
<220> nrdh b2674
<222> 147-246
<223> nrdh b2674 U00096 2798744..2798989
<313> 1769: 225-244
<400> 1769
TCAGTTGCCG GTAGTGATTG CTGGCGATCT TAGCTGGTCT GGTTTCCGTC 50
CGGACATGAT TAACCGTCTG CATCCAGCGC CACACGCGGC CAGTGCATGA 100
<210> 1770
<211> 100
<212> DNA
<213> Escherichia coli K-12 MG1655
<220> nrdh b2674
<222> 107-206
<223> nrdh b2674 U00096 2798744..2798989
<313> 1770: 128-147
<400> 1770
TTCCTGAAGC GGCAGAAGCG TTGCGTGCTC AGGGCTTTCG TCAGTTGCCG 50
GTAGTGATTG CTGGCGATCT TAGCTGGTCT GGTTTCCGTC CGGACATGAT 100
<210> 1771
<211> 100
<212> DNA
<213> Escherichia coli K-12 MG1655
<220> nrdh b2674
<222> 1-100
<223> nrdh b2674 U00096 2798744..2798989
<313> 1771: 49-68
<400> 1771
ATGCGCATTA CTATTTACAC TCGTAACGAT TGCGTTCAGT GCCACGCCAC 50
CAAACGGGCG ATGGAAAACC GGGGCTTTGA TTTTGAAATG ATTAATGTCG 100
<210> 1772
<211> 100
<212> DNA
<213> Escherichia coli K-12 MG1655
<220> b0703 b0704
<222> 1316-1415
<223> b0703 b0704 U00096 733443..734876
<313> 1772: 1361-1380
<400> 1772
AACCCTATTT ATTCCCAGAC ATTAATAAAC CCGATGGGCC AGCAACAGCA 50
GGGATAGATG ATTTGGGTGA AATTTTAGCT AAGATGAAAC AGAGAACATC 100
<210> 1773
<211> 100
<212> DNA
<213> Escherichia coli K-12 MG1655
<220> b0703 b0704
<222> 1235-1334
<223> b0703 b0704 U00096 733443..734876
<313> 1773: 1289-1308
<400> 1773
TGGATACAAA GACAGCAAGT GATTACCTGC CACAGACAAA TTGGCCAACA 50
ACTCAATTAC CACCAGGATA TACTTGTGCA GAACCCTATT TATTCCCAGA 100
<210> 1774
<211> 100
<212> DNA
<213> Escherichia coli K-12 MG1655
<220> b0703 b0704
<222> 1154-1253
<223> b0703 b0704 U00096 733443..734876
<313> 1774: 1204-1223
<400> 1774
ACGACTACAC ACTGTATTGC AGGAAGTGGG TAAAACCAAA TCTGACTTGT 50
ACGCCACAGG ATGATTCCCA GTATCCAGGG ATGGATACAA AGACAGCAAG 100
<210> 1775
<211> 100
<212> DNA
<213> Escherichia coli K-12 MG1655
<220> b1432 b1432
<222> 1110-1209
<223> b1432 b1432 U00096 1501681..1502889

<313> 1775: 1170-1189
<400> 1775
TTTAGCGGCG GGGCACGCCG TTCTTGCCTG TGGAGAGATG GTGCAGTCAG 50
GCCGCCCGTT GAAGCAGGAA CCCACCGAAA TGATTCAGGC GACAGCCTGA 100
<210> 1776
<211> 100
<212> DNA
<213> Escherichia coli K-12 MG1655
<220> b1432 b1432
<222> 1108-1207
<223> b1432 b1432 U00096 1501681..1502889
<313> 1776: 1125-1144
<400> 1776
ATTTTAGCGG CGGGGCACGC CGTTCTTGCC TGTGGAGAGA TGGTGCAGTC 50
AGGCCGCCCG TTGAAGCAGG AACCCACCGA AATGATTCAG GCGACAGCCT 100
<210> 1777
<211> 100
<212> DNA
<213> Escherichia coli K-12 MG1655
<220> b1432 b1432
<222> 1055-1154
<223> b1432 b1432 U00096 1501681..1502889
<313> 1777: 1083-1102
<400> 1777
GATGCCAGGT ATGTGGATAT ACAGCGAACG CCGATGTAAA TGGCGCTCGC 50
AACATTTTAG CGGCGGGGCA CGCCGTTCTT GCCTGTGGAG AGATGGTGCA 100
<210> 1778
<211> 100
<212> DNA
<213> Escherichia coli K-12 MG1655
<220> nrdi b2675
<222> 312-411
<223> nrdi b2675 U00096 2798986..2799396
<313> 1778: 391-410
<400> 1778
GTGGCTGTAC CGTTTTGAAC TCATGGGTAC GCAAAGCGAT ATCGAAAACG 50
TTCGTAAAGG AGTAACCGAA TTTTGGCAAC GACAACCGCA GAATGCCTGA 100
<210> 1779
<211> 100
<212> DNA
<213> Escherichia coli K-12 MG1655
<220> nrdi b2675
<222> 217-316
<223> nrdi b2675 U00096 2798986..2799396
<313> 1779: 278-297
<400> 1779
GCGTTGCTTC GCGGCGTTAT TGCTTCTGGT AATCGCAACT TTGGTGAGGC 50
GTATGGCCGC GCCGGAGATG TGATTGCCCG GAAATGCGGC GTGCCGTGGC 100
<210> 1780
<211> 100
<212> DNA
<213> Escherichia coli K-12 MG1655
<220> nrdi b2675
<222> 197-296
<223> nrdi b2675 U00096 2798986..2799396
<313> 1780: 227-246
<400> 1780
TAAACGACGA GCACAACCGG GCGTTGCTTC GCGGCGTTAT TGCTTCTGGT 50
AATCGCAACT TTGGTGAGGC GTATGGCCGC GCCGGAGATG TGATTGCCCG 100
<210> 1781
<211> 100
<212> DNA
<213> Escherichia coli K-12 MG1655
<220> b1433 b1434
<222> 1337-1436
<223> b1433 b1434 U00096 complement(1502929..1504365)
<313> 1781: 1373-1392
<400> 1781
GATTGACGCT GGTCGGGATT GGTTCTGCGT TTTGGGGATT AATTGCCGGA 50
GGCGTTTGTT ACGTGGTGTT GAATTTAATC GCTGACAGAA ACCGATATTG 100
<210> 1782

<211> 100
<212> DNA
<213> Escherichia coli K-12 MG1655
<220> b1433 b1434
<222> 1265-1364
<223> b1433 b1434 U00096 complement(1502929..1504365)
<313> 1782: 1322-1341
<400> 1782
GTTTGTATCA GGCGCTGCAT AATGAGCGTG AGCGAGACGC GGCGGTGGTG 50
GCATTTCTGG TAACGGCAAG TGGATTGACG CTGGTCGGGA TTGGTTCTGC 100
<210> 1783
<211> 100
<212> DNA
<213> Escherichia coli K-12 MG1655
<220> b1433 b1434
<222> 1218-1317
<223> b1433 b1434 U00096 complement(1502929..1504365)
<313> 1783: 1282-1301
<400> 1783
GATCCAGATG CTGGCAGGTC TGGCGCTGTT AAGTACCATC GGCGGCAGTT 50
TGTATCAGGC GCTGCATAAT GAGCGTGAGC GAGACGCGGC GGTGGTGGCA 100
<210> 1784
<211> 100
<212> DNA
<213> Escherichia coli K-12 MG1655
<220> yaeo b0190
<222> 162-261
<223> yaeo b0190 U00096 complement(213678..213938)
<313> 1784: 221-240
<400> 1784
GGTCGTCGAG GCCGCTGGCG AAACCCGCGA GCTGCGTCTG GATAAAATTA 50
CCAGCTTTAG CCACCCGGAA ATCGGTACGG TGGTGGTAAG CGAATCCTGA 100
<210> 1785
<211> 100
<212> DNA
<213> Escherichia coli K-12 MG1655
<220> yaeo b0190
<222> 115-214
<223> yaeo b0190 U00096 complement(213678..213938)
<313> 1785: 151-170
<400> 1785
CAGGCAAAAG CCAGTGATTT AGTCTCCCGC AAAAATGTGG AGTACCTGGT 50
CGTCGAGGCC GCTGGCGAAA CCCGCGAGCT GCGTCTGGAT AAAATTACCA 100
<210> 1786
<211> 100
<212> DNA
<213> Escherichia coli K-12 MG1655
<220> yaeo b0190
<222> 82-181
<223> yaeo b0190 U00096 complement(213678..213938)
<313> 1786: 106-125
<400> 1786
CTGACACTTG AGCTGAAAGA TGGCGAAAAA TTGCAGGCAA AAGCCAGTGA 50
TTTAGTCTCC CGCAAAAATG TGGAGTACCT GGTCGTCGAG GCCGCTGGCG 100
<210> 1787
<211> 100
<212> DNA
<213> Escherichia coli K-12 MG1655
<220> htra b0162
<222> 1326-1425
<223> htra b0162 U00096 180884..182308
<313> 1787: 1402-1421
<400> 1787
AAACATCGCT GAACTGCGTA AAGTTCTCGA CAGCAAACCG TCTGTGCTGG 50
CACTCAACAT TCAGCGCGGC GACAGCACCA TCTACCTGTT AATGCAGTAA 100
<210> 1788
<211> 100
<212> DNA
<213> Escherichia coli K-12 MG1655
<220> htra b0162
<222> 1297-1396

<223> htra b0162 U00096 180884..182308
<313> 1788: 1354-1373
<400> 1788
ATTATTGGCG CGAACCAGCA GGCAGTGAAA AACATCGCTG AACTGCGTAA 50
AGTTCTCGAC AGCAAACCGT CTGTGCTGGC ACTCAACATT CAGCGCGGCG 100
<210> 1789
<211> 100
<212> DNA
<213> Escherichia coli K-12 MG1655
<220> htra b0162
<222> 1284-1383
<223> htra b0162 U00096 180884..182308
<313> 1789: 1314-1333
<400> 1789
GAAAGGTGAT GTGATTATTG GCGCGAACCA GCAGGCAGTG AAAAACATCG 50
CTGAACTGCG TAAAGTTCTC GACAGCAAAC CGTCTGTGCT GGCACTCAAC 100
<210> 1790
<211> 100
<212> DNA
<213> Escherichia coli K-12 MG1655
<220> yaeq b0191
<222> 447-546
<223> yaeq b0191 U00096 214291..214836
<313> 1790: 517-536
<400> 1790
CATGACGCTG CAGGCAACGA TTCAGGATGG CGTGATCTGG TTATCGGATG 50
ATAAGAATAA TCTGGAAGTG AACTTAACCG CCTGGCAACA ACCTTCATGA 100
<210> 1791
<211> 100
<212> DNA
<213> Escherichia coli K-12 MG1655
<220> yaeq b0191
<222> 413-512
<223> yaeq b0191 U00096 214291..214836
<313> 1791: 461-480
<400> 1791
AACTGGCGAA AGTAAGCGCC TTTGCCGATC GTACCATGAC GCTGCAGGCA 50
ACGATTCAGG ATGGCGTGAT CTGGTTATCG GATGATAAGA ATAATCTGGA 100
<210> 1792
<211> 100
<212> DNA
<213> Escherichia coli K-12 MG1655
<220> yaeq b0191
<222> 363-462
<223> yaeq b0191 U00096 214291..214836
<313> 1792: 406-425
<400> 1792
CAAATGTGTG CAGTTTGCCA ATCTTTCCGT CTGGTATCTG GACGATGAAC 50
AACTGGCGAA AGTAAGCGCC TTTGCCGATC GTACCATGAC GCTGCAGGCA 100
<210> 1793
<211> 100
<212> DNA
<213> Escherichia coli K-12 MG1655
<220> htrb b1055
<222> 822-921
<223> htrb b1055 U00096 complement(1114885..1115805)
<313> 1793: 892-911
<400> 1793
CAAAGTGGTC GAAAAATGCA TCATGATGGC ACCAGAGCAG TATATGTGGT 50
TACACCGTCG CTTTAAAACA CGCCCGGAAG GCGTTCCTTC ACGCTATTAA 100
<210> 1794
<211> 100
<212> DNA
<213> Escherichia coli K-12 MG1655
<220> htrb b1055
<222> 732-831
<223> htrb b1055 U00096 complement(1114885..1115805)
<313> 1794: 782-801
<400> 1794
GCCAGATGGC AAAGGGTATC AATTGATTAT GCTGCCGCCA GAGTGTTCTC 50
CGCCACTGGA TGATGCCGAA ACTACCGCCG CGTGGATGAA CAAAGTGGTC 100

<210> 1795
<211> 100
<212> DNA
<213> Escherichia coli K-12 MG1655
<220> htrb b1055
<222> 705-804
<223> htrb b1055 U00096 complement(1114885..1115805)
<313> 1795: 726-745
<400> 1795
TCTGGTGCCC TTCGTTCCAC GCCGTAAGCC AGATGGCAAA GGGTATCAAT 50
TGATTATGCT GCCGCCAGAG TGTTCTCCGC CACTGGATGA TGCCGAAACT 100
<210> 1796
<211> 100
<212> DNA
<213> Escherichia coli K-12 MG1655
<220> b1436 b1436
<222> 132-231
<223> b1436 b1436 U00096 complement(1506858..1507088)
<313> 1796: 192-211
<400> 1796
CAGCGATGAA TTGCGTGCTG TGGCGGAAGA GTCGGCGGAA GGTTTGCGCG 50
AGCATTTTTA CTGGCAAAAA TCGCGCAAAC CAGAAGCGGG ACAACGTTGA 100
<210> 1797
<211> 100
<212> DNA
<213> Escherichia coli K-12 MG1655
<220> b1436 b1436
<222> 99-198
<223> b1436 b1436 U00096 complement(1506858..1507088)
<313> 1797: 119-138
<400> 1797
GAATGTGGAT CATCAGCTGC GTCATGAAGC GGACAGCGAT GAATTGCGTG 50
CTGTGGCGGA AGAGTCGGCG GAAGGTTTGC GCGAGCATTT TTACTGGCAA 100
<210> 1798
<211> 100
<212> DNA
<213> Escherichia coli K-12 MG1655
<220> b1436 b1436
<222> 5-104
<223> b1436 b1436 U00096 complement(1506858..1507088)
<313> 1798: 51-70
<400> 1798
TTACGAAGGC GTTATCGGTT GTCTTATTAA CGTGTGCTCT GTTTTCAGGA 50
CAACTCATGG CAGGGCACAA AGGACATGAA TTTGTGTGGG TAAAGAATGT 100
<210> 1799
<211> 100
<212> DNA
<213> Escherichia coli K-12 MG1655
<220> yaer b0188
<222> 298-397
<223> yaer b0188 U00096 211850..212266
<313> 1799: 363-382
<400> 1799
GCGCACCTTG AAAGCCATAA CGTGAAGTGT GAAACCATCC GTGTCGATCC 50
ATACACGCAA AAACGCTTCA CCTTCTTTAA CGATCCGGAC GGGCTGCCGT 100
<210> 1800
<211> 100
<212> DNA
<213> Escherichia coli K-12 MG1655
<220> yaer b0188
<222> 288-387
<223> yaer b0188 U00096 211850..212266
<313> 1800: 320-339
<400> 1800
TGCGGCAGTG GCGCACCTTG AAAGCCATAA CGTGAAGTGT GAAACCATCC 50
GTGTCGATCC ATACACGCAA AAACGCTTCA CCTTCTTTAA CGATCCGGAC 100
<210> 1801
<211> 100
<212> DNA
<213> Escherichia coli K-12 MG1655
<220> yaer b0188

<222> 188-287
<223> yaer b0188 U00096 211850..212266
<313> 1801: 251-270
<400> 1801
ATGTGATTGA GCTTTTCTCA TTTCCGTTCC CGCCGGAACG ACCCAGCCGA 50
CCGGAAGCTT GCGGTCTGCG TCATCTGGCT TTTAGCGTTG ATGACATCGA 100
<210> 1802
<211> 100
<212> DNA
<213> Escherichia coli K-12 MG1655
<220> htrc b3990
<222> 441-540
<223> htrc b3990 U00096 4187364..4187903
<313> 1802: 499-518
<400> 1802
TTATCCCATT TCTGAAAAAC AGAAAGGTAA ATCAGTGAAA TTTTTCACCA 50
TTGCCTACAA CTTATTGAGA AATAAGCAGC TTCCTCAGCC CTCAAAATAA 100
<210> 1803
<211> 100
<212> DNA
<213> Escherichia coli K-12 MG1655
<220> htrc b3990
<222> 371-470
<223> htrc b3990 U00096 4187364..4187903
<313> 1803: 399-418
<400> 1803
AGGCATTTTA CTTTGTTGCG TTCAGGGCTT TCAGGGAAAA GAAAAAACTC 50
CGTTTGCATG TAACTAGCGC TTATCCCATT TCTGAAAAAC AGAAAGGTAA 100
<210> 1804
<211> 100
<212> DNA
<213> Escherichia coli K-12 MG1655
<220> htrc b3990
<222> 324-423
<223> htrc b3990 U00096 4187364..4187903
<313> 1804: 355-374
<400> 1804
GTATGGTAGC TATGCCGTGA TTGAGGTGGA CTTAGACGGA GGAGATAAGG 50
CATTTTACTT TGTTGCGTTC AGGGCTTTCA GGGAAAAGAA AAAACTCCGT 100
<210> 1805
<211> 100
<212> DNA
<213> Escherichia coli K-12 MG1655
<220> b1437 b1437
<222> 99-198
<223> b1437 b1437 U00096 complement(1507274..1507471)
<313> 1805: 169-188
<400> 1805
TGCTGCCATT CGCTACATCG ACGCCCTGAG ATTCGAGCCA ACGTCTGAAC 50
TCGCTTTGTT TCACATCGCC TCCCTGCCGC TGAGTATATC AACAAAGTAA 100
<210> 1806
<211> 100
<212> DNA
<213> Escherichia coli K-12 MG1655
<220> b1437 b1437
<222> 81-180
<223> b1437 b1437 U00096 complement(1507274..1507471)
<313> 1806: 132-151
<400> 1806
ACCTGAGTTT CAAATGGTTG CTGCCATTCG CTACATCGAC GCCCTGAGAT 50
TCGAGCCAAC GTCTGAACTC GCTTTGTTTC ACATCGCCTC CCTGCCGCTG 100
<210> 1807
<211> 100
<212> DNA
<213> Escherichia coli K-12 MG1655
<220> b1437 b1437
<222> 53-152
<223> b1437 b1437 U00096 complement(1507274..1507471)
<313> 1807: 89-108
<400> 1807
CGCGGCATGA CACTGCGCCT CCCATGAAAC CTGAGTTTCA AATGGTTGCT 50

GCCATTCGCT ACATCGACGC CCTGAGATTC GAGCCAACGT CTGAACTCGC 100
<210> 1808
<211> 100
<212> DNA
<213> Escherichia coli K-12 MG1655
<220> yaes b0175
<222> 663-762
<223> yaes b0175 U00096 194903..195664
<313> 1808: 735-754
<400> 1808
GCCCGATTTC GATGAACAAG ACTTTGAAGG GGCGTTAAAT GCCTTTGCTA 50
ATCGAGAGCG TCGTTTCGGC GGCACCGAGC CCGGTGATGA AACAGCCTGA 100
<210> 1809
<211> 100
<212> DNA
<213> Escherichia coli K-12 MG1655
<220> yaes b0175
<222> 621-720
<223> yaes b0175 U00096 194903..195664
<313> 1809: 656-675
<400> 1809
GCAAATTGCC TATGCCGAAC TTTACTTTAC AGATGTTCTC TGGCCCGATT 50
TCGATGAACA AGACTTTGAA GGGGCGTTAA ATGCCTTTGC TAATCGAGAG 100
<210> 1810
<211> 100
<212> DNA
<213> Escherichia coli K-12 MG1655
<220> yaes b0175
<222> 562-661
<223> yaes b0175 U00096 194903..195664
<313> 1810: 599-618
<400> 1810
CCTGTAGATT TAGTAATTAG GACTGGGGGG GAGCATCGCA TTAGTAACTT 50
TTTGCTTTGG CAAATTGCCT ATGCCGAACT TTACTTTACA GATGTTCTCT 100
<210> 1811
<211> 100
<212> DNA
<213> Escherichia coli K-12 MG1655
<220> b1438 b1438
<222> 329-428
<223> b1438 b1438 U00096 1507511..1507948
<313> 1811: 379-398
<400> 1811
CTAAACAGGA GATTACTCGC CTATTTAACT TGCATCATGC GACAAAAATC 50
GACGCCGTCC AGCTCGCGGC AAAGGCGCTT GGCAAAGAGT TATCGCTGGT 100
<210> 1812
<211> 100
<212> DNA
<213> Escherichia coli K-12 MG1655
<220> b1438 b1438
<222> 283-382
<223> b1438 b1438 U00096 1507511..1507948
<313> 1812: 332-351
<400> 1812
CAGTCAGAAA TCACTCAGCA AGAGTTAGCC AGGCGAATTG GCAAACCTAA 50
ACAGGAGATT ACTCGCCTAT TTAACTTGCA TCATGCGACA AAAATCGACG 100
<210> 1813
<211> 100
<212> DNA
<213> Escherichia coli K-12 MG1655
<220> b1438 b1438
<222> 195-294
<223> b1438 b1438 U00096 1507511..1507948
<313> 1813: 233-252
<400> 1813
TTTACCTTCG CCATTAAATA GTCACGATCA CTTTATTGAA GTACCTTTGA 50
GCGTCGCCTC TAAGGTATTG CTGTTAAATG CTTTTTTACA GTCAGAAATC 100
<210> 1814
<211> 100
<212> DNA
<213> Escherichia coli K-12 MG1655

<220> yaet b0178
<222> 2334-2433
<223> yaet b0178 U00096 197928..200360
<313> 1814: 2393-2412
<400> 1814
CCCATTGGGG CCGTTGGTGT TCTCCTACGC CCAGCCGTTC AAAAAGTACG 50
ATGGAGACAA GGCAGAACAG TTCCAGTTTA ACATCGGTAA AACCTGGTAA 100
<210> 1815
<211> 100
<212> DNA
<213> Escherichia coli K-12 MG1655
<220> yaet b0178
<222> 2297-2396
<223> yaet b0178 U00096 197928..200360
<313> 1815: 2349-2368
<400> 1815
TCCGTATGTC TGCGGGTATC GCATTACAAT GGATGTCCCC ATTGGGGCCG 50
TTGGTGTTCT CCTACGCCCA GCCGTTCAAA AAGTACGATG GAGACAAGGC 100
<210> 1816
<211> 100
<212> DNA
<213> Escherichia coli K-12 MG1655
<220> yaet b0178
<222> 2113-2212
<223> yaet b0178 U00096 197928..200360
<313> 1816: 2152-2171
<400> 1816
GCTGTAGGCG GTAACGCCAT GGCGGTTGCC AGCCTCGAGT TCATCACCCC 50
GACGCCGTTT ATTAGCGATA AGTATGCTAA CTCGGTTCGT ACTTCCTTCT 100
<210> 1817
<211> 100
<212> DNA
<213> Escherichia coli K-12 MG1655
<220> htre b0140
<222> 2427-2526
<223> htre b0140 U00096 complement(152829..155426)
<313> 1817: 2470-2489
<400> 1817
ACAGGGTGGA CAAGCATTTG TTCGTGGTAT TGAGCAGCAG GGAAATATCA 50
GCATTAAATG GCTCGAACAA AGTAAACCCG TAAGTTGTCT TGCGCATTAT 100
<210> 1818
<211> 100
<212> DNA
<213> Escherichia coli K-12 MG1655
<220> htre b0140
<222> 2360-2459
<223> htre b0140 U00096 complement(152829..155426)
<313> 1818: 2414-2433
<400> 1818
ATGGTAAAAA TATTCCATTT GCTGCAGATA TTTATGATGA GCAAGGCAAT 50
GTCATTGGTA ATGTTGGACA GGGTGGACAA GCATTTGTTC GTGGTATTGA 100
<210> 1819
<211> 100
<212> DNA
<213> Escherichia coli K-12 MG1655
<220> htre b0140
<222> 2308-2407
<223> htre b0140 U00096 complement(152829..155426)
<313> 1819: 2340-2359
<400> 1819
GATTTTGAAA CCGTGCAAGG GCAATCAGCC ATTATGAACA TCACACGAAG 50
TGATGGTAAA AATATTCCAT TTGCTGCAGA TATTTATGAT GAGCAAGGCA 100
<210> 1820
<211> 100
<212> DNA
<213> Escherichia coli K-12 MG1655
<220> b1439 b1439
<222> 1308-1407
<223> b1439 b1439 U00096 1508027..1509433
<313> 1820: 1355-1374
<400> 1820

CTGGTCACGT TTTTTCCGTT TTAATACCGC GTGGCAGTGG GGAGAGCGTG 50
AAGAACAGGC GGTAAAACAA TTAGGCAAAC TTATTCAAGA ACGGCTGTAA 100
<210> 1821
<211> 100
<212> DNA
<213> Escherichia coli K-12 MG1655
<220> b1439 b1439
<222> 1264-1363
<223> b1439 b1439 U00096 1508027..1509433
<313> 1821: 1310-1329
<400> 1821
CATATCAGTA TTGCGCCGGG TAAAATGTTT TCTACCGGTG AAAACTGGTC 50
ACGTTTTTTC CGTTTTAATA CCGCGTGGCA GTGGGGAGAG CGTGAAGAAC 100
<210> 1822
<211> 100
<212> DNA
<213> Escherichia coli K-12 MG1655
<220> b1439 b1439
<222> 1197-1296
<223> b1439 b1439 U00096 1508027..1509433
<313> 1822: 1248-1267
<400> 1822
CTTTCTCTGG TTGGAGCTCC CCGAGCCGTT AGATGCCGGC GAATTAAGCC 50
TGGCGGCACT GACGCATCAT ATCAGTATTG CGCCGGGTAA AATGTTTTCT 100
<210> 1823
<211> 100
<212> DNA
<213> Escherichia coli K-12 MG1655
<220> htrl b3619
<222> 774-873
<223> htrl b3619 U00096 complement(3790453..3791325)
<313> 1823: 824-843
<400> 1823
TCTGGGGAGA GGAAAATGGT TCGATTTGTT TCGCTGCTTC AGGAGTAACA 50
CTTTAGGGGC AAAAATGCAG GCGCTGAGAA TTTTCCTTTC AAGAAAATAG 100
<210> 1824
<211> 100
<212> DNA
<213> Escherichia coli K-12 MG1655
<220> htrl b3619
<222> 739-838
<223> htrl b3619 U00096 complement(3790453..3791325)
<313> 1824: 786-805
<400> 1824
TATTACAAAC GCCCCGATCT CTTCAACCTA AACTATCTGG GGAGAGGAAA 50
ATGGTTCGAT TTGTTTCGCT GCTTCAGGAG TAACACTTTA GGGGCAAAAA 100
<210> 1825
<211> 100
<212> DNA
<213> Escherichia coli K-12 MG1655
<220> htrl b3619
<222> 714-813
<223> htrl b3619 U00096 complement(3790453..3791325)
<313> 1825: 737-756
<400> 1825
TGACCAGGGC ATTTTTGTCA TGTGCTATTA CAAACGCCCC GATCTCTTCA 50
ACCTAAACTA TCTGGGGAGA GGAAAATGGT TCGATTTGTT TCGCTGCTTC 100
<210> 1826
<211> 100
<212> DNA
<213> Escherichia coli K-12 MG1655
<220> lamb b4037
<222> 1242-1341
<223> lamb b4037 U00096 4245550..4246890
<313> 1826: 1299-1318
<400> 1826
CTTCGGCAAA GCCGTTCCTG CTGATTTCAA CGGCGGCAGC TTCGGTCGTG 50
GCGACAGCGA CGAGTGGACC TTCGGTGCCC AGATGGAAAT CTGGTGGTAA 100
<210> 1827
<211> 100
<212> DNA

<213> Escherichia coli K-12 MG1655
<220> lamb b4037
<222> 1188-1287
<223> lamb b4037 U00096 4245550..4246890
<313> 1827: 1235-1254
<400> 1827
GTGGGATGAG AAATGGGGTT ACGACTACAC CGGTAACGCT GATAACAACG 50
CGAACTTCGG CAAAGCCGTT CCTGCTGATT TCAACGGCGG CAGCTTCGGT 100
<210> 1828
<211> 100
<212> DNA
<213> Escherichia coli K-12 MG1655
<220> lamb b4037
<222> 1159-1258
<223> lamb b4037 U00096 4245550..4246890
<313> 1828: 1199-1218
<400> 1828
GCTATTCGTG TCTTCGCAAC CTACGCCAAG TGGGATGAGA AATGGGGTTA 50
CGACTACACC GGTAACGCTG ATAACAACGC GAACTTCGGC AAAGCCGTTC 100
<210> 1829
<211> 100
<212> DNA
<213> Escherichia coli K-12 MG1655
<220> yafa b0240
<222> 1146-1245
<223> yafa b0240 U00096 256527..257771
<313> 1829: 1221-1240
<400> 1829
TGACGGTAAA TTATTAGAGA TCCCATTTAA CCCGGTGTAT CGGAATTTTG 50
ACAAAGGTCT TCAGGAAATC ACCGACTGGA TCGAAAAACG CTTGTGTTAA 100
<210> 1830
<211> 100
<212> DNA
<213> Escherichia coli K-12 MG1655
<220> yafa b0240
<222> 1079-1178
<223> yafa b0240 U00096 256527..257771
<313> 1830: 1118-1137
<400> 1830
CAGGCTACTG GAAGAACGAT CCGTTCAGCC CGGAAGAGGA CTCACGCTTA 50
ATCACCTCAT CATCTGCTGA CGGTAAATTA TTAGAGATCC CATTTAACCC 100
<210> 1831
<211> 100
<212> DNA
<213> Escherichia coli K-12 MG1655
<220> yafa b0240
<222> 1037-1136
<223> yafa b0240 U00096 256527..257771
<313> 1831: 1078-1097
<400> 1831
TGCAAGGATT GCTGGGACGT CGCTGCCCAA CGCCAATGTT ATCAGGCTAC 50
TGGAAGAACG ATCCGTTCAG CCCGGAAGAG GACTCACGCT TAATCACCTC 100
<210> 1832
<211> 100
<212> DNA
<213> Escherichia coli K-12 MG1655
<220> yafb b0208
<222> 592-691
<223> yafb b0208 U00096 229167..229970
<313> 1832: 624-643
<400> 1832
AAACACAATG CGACTCCGGC ACAAGTGATT CTGGCGTGGG CTATGGGGGA 50
AGGTTACTCA GTAATTCCTT CTTCTACTAA ACGTAAAAAC CTGGAAAGTA 100
<210> 1833
<211> 100
<212> DNA
<213> Escherichia coli K-12 MG1655
<220> yafb b0208
<222> 549-648
<223> yafb b0208 U00096 229167..229970
<313> 1833: 582-601

<400> 1833
TGGTAAGGCC CTGAAAGATG AGGTTATTGC TCGTATCGCA GCTAAACACA 50
ATGCGACTCC GGCACAAGTG ATTCTGGCGT GGGCTATGGG GGAAGGTTAC 100
<210> 1834
<211> 100
<212> DNA
<213> Escherichia coli K-12 MG1655
<220> yafb b0208
<222> 484-583
<223> yafb b0208 U00096 229167..229970
<313> 1834: 512-531
<400> 1834
CGTAAAGTGG TTGCCTGGGC TAAACAGCAC GGCATCCATA TTACTTCCTA 50
TATGACGCTG GCGTATGGTA AGGCCCTGAA AGATGAGGTT ATTGCTCGTA 100
<210> 1835
<211> 100
<212> DNA
<213> Escherichia coli K-12 MG1655
<220> yafc b0209
<222> 816-915
<223> yafc b0209 U00096 complement(229967..230881)
<313> 1835: 858-877
<400> 1835
TTACAGCGAC CGTGCGGTAA GTACGCGCAT CCGGGCTTTT ATCGATTTCC 50
TTAGCGAGCA TGTAAAAACA GCTCCCGGAG GAGCTGTCAG AGAGGCTTAA 100
<210> 1836
<211> 100
<212> DNA
<213> Escherichia coli K-12 MG1655
<220> yafc b0209
<222> 767-866
<223> yafc b0209 U00096 complement(229967..230881)
<313> 1836: 804-823
<400> 1836
TAATGGCAGA TAAAGTGTTG CCAGTGGAAA TGCCATTCAG TGCCGTCTAT 50
TACAGCGACC GTGCGGTAAG TACGCGCATC CGGGCTTTTA TCGATTTCCT 100
<210> 1837
<211> 100
<212> DNA
<213> Escherichia coli K-12 MG1655
<220> yafc b0209
<222> 730-829
<223> yafc b0209 U00096 complement(229967..230881)
<313> 1837: 754-773
<400> 1837
ATCGACAAAG AAATCGCTCG CGGAGAATTG GTGGAGTTAA TGGCAGATAA 50
AGTGTTGCCA GTGGAAATGC CATTCAGTGC CGTCTATTAC AGCGACCGTG 100
<210> 1838
<211> 100
<212> DNA
<213> Escherichia coli K-12 MG1655
<220> yafd b0210
<222> 498-597
<223> yafd b0210 U00096 231122..231922
<313> 1838: 550-569
<400> 1838
CTATAGTAAG CAGTTACTTC CTATTGGCGA TCAGATAGCT CACCACAGCG 50
GCCCGGTCAT TATGGCGGGA GATTTCAATG CCTGGAGCCG TAGAAGGATG 100
<210> 1839
<211> 100
<212> DNA
<213> Escherichia coli K-12 MG1655
<220> yafd b0210
<222> 392-491
<223> yafd b0210 U00096 231122..231922
<313> 1839: 417-436
<400> 1839
TGCGTCTGGC GAAGTCGGCA CTGGTGACGG TCTATCCATT ACCTGACACC 50
CGCCTGTTGA TGGTGGTTAA TATACACGCC GTCAACTTCA GTCTGGGCGT 100
<210> 1840
<211> 100

<212> DNA
<213> Escherichia coli K-12 MG1655
<220> yafd b0210
<222> 324-423
<223> yafd b0210 U00096 231122..231922
<313> 1840: 387-406
<400> 1840
TGGCGTAATG ACCCTTTCGG CGGCACATCC AGTGTATTGC TGCCCGTTAC 50
GTGAACGAGA ACCCATTTTG CGTCTGGCGA AGTCGGCACT GGTGACGGTC 100
<210> 1841
<211> 100
<212> DNA
<213> Escherichia coli K-12 MG1655
<220> yafe b0211
<222> 483-582
<223> yafe b0211 U00096 231926..232549
<313> 1841: 534-553
<400> 1841
GCGTACGCCA GAAGCGTTAG TAGACGCTAT TCGCATTTAC CAACAGAGCG 50
CATCGACAGA GGTGAGAACG TATTTTGCCT TGCAGAATGA TGGCTTTTTC 100
<210> 1842
<211> 100
<212> DNA
<213> Escherichia coli K-12 MG1655
<220> yafe b0211
<222> 290-389
<223> yafe b0211 U00096 231926..232549
<313> 1842: 334-353
<400> 1842
TGGACGTAAT GTCTCCGGGT CACCCAGTGC GCGACATCTG GTTACAGACG 50
GTAGAAGCAT TACGCGATAC CTCTCACGTA CGAAACTACG CCAGCGGTGA 100
<210> 1843
<211> 100
<212> DNA
<213> Escherichia coli K-12 MG1655
<220> yafe b0211
<222> 243-342
<223> yafe b0211 U00096 231926..232549
<313> 1843: 290-309
<400> 1843
GCGAGAAGTG AATAGGATAT TGAAACCTGG CGGTAGGCTG ATTGTGATGG 50
ACGTAATGTC TCCGGGTCAC CCAGTGCGCG ACATCTGGTT ACAGACGGTA 100
<210> 1844
<211> 100
<212> DNA
<213> Escherichia coli K-12 MG1655
<220> yafh b0222
<222> 2382-2481
<223> yafh b0222 U00096 complement(240859..243339)
<313> 1844: 2460-2479
<400> 1844
CCGTCTGCGC AGTATTAACG TTGATGACTT TGATCCGGAA GAGCTGGCGA 50
CGAAGCCGGT AAAGTTGCCG GAGAAAGTGC GGAAAGTTGA AGCCGCGTAA 100
<210> 1845
<211> 100
<212> DNA
<213> Escherichia coli K-12 MG1655
<220> yafh b0222
<222> 2335-2434
<223> yafh b0222 U00096 complement(240859..243339)
<313> 1845: 2368-2387
<400> 1845
CTGATTGATA AAGATGAAGC CGCTATTCTG GTGAAAGCTG AAGAAAGCCG 50
TCTGCGCAGT ATTAACGTTG ATGACTTTGA TCCGGAAGAG CTGGCGACGA 100
<210> 1846
<211> 100
<212> DNA
<213> Escherichia coli K-12 MG1655
<220> yafh b0222
<222> 2146-2245
<223> yafh b0222 U00096 complement(240859..243339)

<313> 1846: 2171-2190
<400> 1846
ACCCGTTCCC GCATTGGTCG CGGTCAGTAC CTGACGCCGA GCGAGCATAA 50
TCCGGTTGGC TTGCTGGAAG AGGCGCTGGT GGATGTGATT GCCGCCGACC 100
<210> 1847
<211> 100
<212> DNA
<213> Escherichia coli K-12 MG1655
<220> yafj b0224
<222> 661-760
<223> yafj b0224 U00096 244327..245094
<313> 1847: 702-721
<400> 1847
GATGTGGTCA CGGTGATTGC AACACAGCCG CTGACGGGCA ATGAAACCTG 50
GCAAAAGATT ATGCCAGGCG AATGGCGCTT ATTTTGCCTC GGGGAGCGTG 100
<210> 1848
<211> 100
<212> DNA
<213> Escherichia coli K-12 MG1655
<220> yafj b0224
<222> 584-683
<223> yafj b0224 U00096 244327..245094
<313> 1848: 631-650
<400> 1848
GCCGCGCGCC GTTTGGCGTG GCAACGTTGC TGGATCAGGA TGTGGAAATC 50
GACTTCAGCT CGCAGACCAC ACCGAATGAT GTGGTCACGG TGATTGCAAC 100
<210> 1849
<211> 100
<212> DNA
<213> Escherichia coli K-12 MG1655
<220> yafj b0224
<222> 469-568
<223> yafj b0224 U00096 244327..245094
<313> 1849: 525-544
<400> 1849
AAATATATCG CCTCACTGGC GGATGAACTG CGGCAGAAGG GCGTTTTCAA 50
CATGCTGCTT TCGGACGGGC GCTATGTAAT GGCGTATTGC TCGACTAATT 100
<210> 1850
<211> 100
<212> DNA
<213> Escherichia coli K-12 MG1655
<220> yafk b0225
<222> 642-741
<223> yafk b0225 U00096 complement(245065..245805)
<313> 1850: 709-728
<400> 1850
TTCTGTGGTC AATGGTCGTT ACGTGGTCAG CAAGCCGTTA AGCCACGAAG 50
TTGTGCAGCC ACAACTGGCA TCAAACTACA CGCTCCCCGA GGCAAAATAA 100
<210> 1851
<211> 100
<212> DNA
<213> Escherichia coli K-12 MG1655
<220> yafk b0225
<222> 585-684
<223> yafk b0225 U00096 complement(245065..245805)
<313> 1851: 628-647
<400> 1851
GGAGCAACTG AAGCCGGGTT ACGACTACTT TGAGCAAACC CGTAAGCCAC 50
CAACCGTTTC TGTGGTCAAT GGTCGTTACG TGGTCAGCAA GCCGTTAAGC 100
<210> 1852
<211> 100
<212> DNA
<213> Escherichia coli K-12 MG1655
<220> yafk b0225
<222> 496-595
<223> yafk b0225 U00096 complement(245065..245805)
<313> 1852: 544-563
<400> 1852
CAGCCGAGCG TGCAAGTGAG TATTTACCCG TTCCGCATGA CCGACGCCAA 50
TATGAAGCGC CATAAATATT CCAACTTTAA GGACTTCTGG GAGCAACTGA 100
<210> 1853

<211> 100
<212> DNA
<213> Escherichia coli K-12 MG1655
<220> b1440 b1440
<222> 1047-1146
<223> b1440 b1440 U00096 1509678..1510823
<313> 1853: 1113-1132
<400> 1853
CAAAATCGCC TTCTGGAAAA CGCCTATAGC AGAAGGGGGC AAGTTTGTTC 50
CCTACAGTCG CTGGACGCAG GATTACATTG CCATTATGGG CGGTCGCTAA 100
<210> 1854
<211> 100
<212> DNA
<213> Escherichia coli K-12 MG1655
<220> b1440 b1440
<222> 957-1056
<223> b1440 b1440 U00096 1509678..1510823
<313> 1854: 1007-1026
<400> 1854
TGGCTCGTTA CCGGTAGTGC CGGAAGGGTG TAAAGCCAGT CCGTTATTAG 50
GCGAAAAAGG TTGTGAAACC AACGGTTTTA ACTATTTCGA CAAAATCGCC 100
<210> 1855
<211> 100
<212> DNA
<213> Escherichia coli K-12 MG1655
<220> b1440 b1440
<222> 910-1009
<223> b1440 b1440 U00096 1509678..1510823
<313> 1855: 933-952
<400> 1855
AACTGGTCAT TAACGCCAAA AGTGCAGGGC GATGTGGCGG CCTGGTTTGG 50
CTCGTTACCG GTAGTGCCGG AAGGGTGTAA AGCCAGTCCG TTATTAGGCG 100
<210> 1856
<211> 100
<212> DNA
<213> Escherichia coli K-12 MG1655
<220> yafl b0228
<222> 628-727
<223> yafl b0228 U00096 246712..247461
<313> 1856: 692-711
<400> 1856
GGGCAATTTA TCGAGTCGCC ACGTACCGGC GAAACCATTC GGATAAGCCG 50
ATTAGCCGAA CCTTTCTGGC AGGACCATTT TTTGGGCGCG CGCAGGATTT 100
<210> 1857
<211> 100
<212> DNA
<213> Escherichia coli K-12 MG1655
<220> yafl b0228
<222> 599-698
<223> yafl b0228 U00096 246712..247461
<313> 1857: 632-651
<400> 1857
CCGATCATAT GGGCGTGTAT TTGGGCGATG GGCAATTTAT CGAGTCGCCA 50
CGTACCGGCG AAACCATTCG GATAAGCCGA TTAGCCGAAC CTTTCTGGCA 100
<210> 1858
<211> 100
<212> DNA
<213> Escherichia coli K-12 MG1655
<220> yafl b0228
<222> 520-619
<223> yafl b0228 U00096 246712..247461
<313> 1858: 576-595
<400> 1858
CGCCGGGCAA CGATTGTGGC GAACAACGAC CTGCGCCGGG GAGATTTGCT 50
GTTTTTCCAT ATCCACAGCC GCGAGATAGC CGATCATATG GGCGTGTATT 100
<210> 1859
<211> 100
<212> DNA
<213> Escherichia coli K-12 MG1655
<220> b1441 b1441
<222> 915-1014

<223> b1441 b1441 U00096 1510841..1511854
<313> 1859: 958-977
<400> 1859
TATGACAGGC GAAGAACTGC CTGCCACGCT CACGCCCGGA CAACAGGTGA 50
TGGTTTCCTG GTCGCGTGAT GTGATGGTGC CGCTGGTTGA GGAGAGGTGA 100
<210> 1860
<211> 100
<212> DNA
<213> Escherichia coli K-12 MG1655
<220> b1441 b1441
<222> 842-941
<223> b1441 b1441 U00096 1510841..1511854
<313> 1860: 868-887
<400> 1860
AATATCAGGG CGCGGCAACT CGTTTTGAAC TGAAATTGAA CGGCGGTGAA 50
AAACTGCTTG TGAGTCAGGC CAATATGACA GGCGAAGAAC TGCCTGCCAC 100
<210> 1861
<211> 100
<212> DNA
<213> Escherichia coli K-12 MG1655
<220> b1441 b1441
<222> 779-878
<223> b1441 b1441 U00096 1510841..1511854
<313> 1861: 839-858
<400> 1861
CGGAACATAT CCGCCTCAAC ACCCCTGGTG AACTGCAGGC CAATGGCACG 50
ATCCAGGCGG TGCAATATCA GGGCGCGGCA ACTCGTTTTG AACTGAAATT 100
<210> 1862
<211> 100
<212> DNA
<213> Escherichia coli K-12 MG1655
<220> yafm b0229
<222> 399-498
<223> yafm b0229 U00096 247637..248134
<313> 1862: 472-491
<400> 1862
ATTCTCAACG TTCCATCGCG ATGTCGCGCG AGGGTTATAT CCCATCGATT 50
GGGCGGGGGA CGTAACGGAT TTTAGTGCCG GGGAGCGTAT CATTTCATAA 100
<210> 1863
<211> 100
<212> DNA
<213> Escherichia coli K-12 MG1655
<220> yafm b0229
<222> 335-434
<223> yafm b0229 U00096 247637..248134
<313> 1863: 370-389
<400> 1863
ATGTTGATTA TATTTATATA AATCCAGTAA AGCATGGTTG GGTAAAGCAA 50
GTGAGTGATT GGCCATTCTC AACGTTCCAT CGCGATGTCG CGCGAGGGTT 100
<210> 1864
<211> 100
<212> DNA
<213> Escherichia coli K-12 MG1655
<220> yafm b0229
<222> 285-384
<223> yafm b0229 U00096 247637..248134
<313> 1864: 304-323
<400> 1864
ACCACGTTTT TGGGAGCACG CCATCCGCAA TACCAAAGAT TATAGGCATC 50
ATGTTGATTA TATTTATATA AATCCAGTAA AGCATGGTTG GGTAAAGCAA 100
<210> 1865
<211> 100
<212> DNA
<213> Escherichia coli K-12 MG1655
<220> b1442 b1442
<222> 843-942
<223> b1442 b1442 U00096 1511855..1512796
<313> 1865: 878-897
<400> 1865
CAATATGCCG ATGGCGGCGG CATTCACCCT GGTGCCGATT ATTCTCATCG 50
CACTGTACCT GGCGTTCGTG AAACGTCTGG GAGCGTTCGA TGCACTCTGA 100

<210> 1866
<211> 100
<212> DNA
<213> Escherichia coli K-12 MG1655
<220> b1442 b1442
<222> 785-884
<223> b1442 b1442 U00096 1511855..1512796
<313> 1866: 823-842
<400> 1866
TTGGTCCTCC AGGATATTTT ATCGGCAATA TGGTTTATTC CCAGCAGGGG 50
GCGATTGGCA ATATGCCGAT GGCGGCGGCA TTCACCCTGG TGCCGATTAT 100
<210> 1867
<211> 100
<212> DNA
<213> Escherichia coli K-12 MG1655
<220> b1442 b1442
<222> 689-788
<223> b1442 b1442 U00096 1511855..1512796
<313> 1867: 732-751
<400> 1867
GCTATGTGGT GCTGCCGCTG GCAATCCCGG GTATTGCCGC TGGCTCTATC 50
TTTACCTTCT CACTCACACT GGGCGATTTT ATCGTCCCGC AGCTGGTTGG 100
<210> 1868
<211> 100
<212> DNA
<213> Escherichia coli K-12 MG1655
<220> yafn b0233
<222> 195-294
<223> yafn b0233 U00096 252005..252298
<313> 1868: 236-255
<400> 1868
CTTCCGTCCA AGTGCTGCAA GATTAGAGGA AATTACACGC CGCGCTGAAC 50
AATATCTTAA TGATATGACG GATGATGATT TCAATGACTT TAAGGAATAA 100
<210> 1869
<211> 100
<212> DNA
<213> Escherichia coli K-12 MG1655
<220> yafn b0233
<222> 142-241
<223> yafn b0233 U00096 252005..252298
<313> 1869: 200-219
<400> 1869
GCGTTAATGG ACATGCTTGC TGAACAAGAG GAGAAAAAGC CCATAAAGGC 50
GCGCTTCCGT CCAAGTGCTG CAAGATTAGA GGAAATTACA CGCCGCGCTG 100
<210> 1870
<211> 100
<212> DNA
<213> Escherichia coli K-12 MG1655
<220> yafn b0233
<222> 110-209
<223> yafn b0233 U00096 252005..252298
<313> 1870: 165-184
<400> 1870
CAGGATATCT CTTAAGTGCC AGCGCATTCG AAGCGTTAAT GGACATGCTT 50
GCTGAACAAG AGGAGAAAAA GCCCATAAAG GCGCGCTTCC GTCCAAGTGC 100
<210> 1871
<211> 100
<212> DNA
<213> Escherichia coli K-12 MG1655
<220> b1443 b1443
<222> 696-795
<223> b1443 b1443 U00096 1512786..1513580
<313> 1871: 775-794
<400> 1871
ACCGGTAACT AACGTGGTGG CACTGCTGGT TATGTTGGTA ACAACCTTGC 50
CGATCCTGGG GGCCTGGTGG CTAACCCGCG AAGGCGACAA TGGTCAATAA 100
<210> 1872
<211> 100
<212> DNA
<213> Escherichia coli K-12 MG1655
<220> b1443 b1443

<222> 695-794
<223> b1443 b1443 U00096 1512786..1513580
<313> 1872: 709-728
<400> 1872
TACCGGTAAC TAACGTGGTG GCACTGCTGG TTATGTTGGT AACAACCTTG  50
CCGATCCTGG GGGCCTGGTG GCTAACCCGC GAAGGCGACA ATGGTCAATA  100
<210> 1873
<211> 100
<212> DNA
<213> Escherichia coli K-12 MG1655
<220> b1443 b1443
<222> 618-717
<223> b1443 b1443 U00096 1512786..1513580
<313> 1873: 643-662
<400> 1873
CGTTACGACC TTTACGGCAG GTCATGAACG AACGTTACCG TTGTGGTTGC  50
TCAATCAGCT TGGGCGACCG CGTGATGTAC CGGTAACTAA CGTGGTGGCA  100
<210> 1874
<211> 100
<212> DNA
<213> Escherichia coli K-12 MG1655
<220> yafo b0234
<222> 280-379
<223> yafo b0234 U00096 252301..252699
<313> 1874: 313-332
<400> 1874
GATGAGCAAG CATGGTTGCT CATTGCCATT CTGAAACCTG AACCTCATAA  50
ACTGGCTCGA GATAACAACC AAATGCATAA AATTGGGAAA ATGGCAGAAG  100
<210> 1875
<211> 100
<212> DNA
<213> Escherichia coli K-12 MG1655
<220> yafo b0234
<222> 226-325
<223> yafo b0234 U00096 252301..252699
<313> 1875: 262-281
<400> 1875
CAATTCAGCA GAACGAATGA CGAAGCGCAT TTGGTATATT GTCAGGGGGC  50
GTTTGATGAG CAAGCATGGT TGCTCATTGC CATTCTGAAA CCTGAACCTC  100
<210> 1876
<211> 100
<212> DNA
<213> Escherichia coli K-12 MG1655
<220> yafo b0234
<222> 198-297
<223> yafo b0234 U00096 252301..252699
<313> 1876: 233-252
<400> 1876
GAATAATCCA TTTCCGCCAC AGTTGCGCCA ATTCAGCAGA ACGAATGACG  50
AAGCGCATTT GGTATATTGT CAGGGGGCGT TTGATGAGCA AGCATGGTTG  100
<210> 1877
<211> 100
<212> DNA
<213> Escherichia coli K-12 MG1655
<220> b1444 b1444
<222> 1326-1425
<223> b1444 b1444 U00096 1513602..1515026
<313> 1877: 1396-1415
<400> 1877
GCCGCACGGT GGGCAGAAAC TTTCTGGTTA CGGCAAGGAT ATGTCACTTT  50
ATGGGCTGGA GGATTACACC GTCGTCCGCC ACGTCATGGT TAAACATTAA  100
<210> 1878
<211> 100
<212> DNA
<213> Escherichia coli K-12 MG1655
<220> b1444 b1444
<222> 1296-1395
<223> b1444 b1444 U00096 1513602..1515026
<313> 1878: 1334-1353
<400> 1878
CAATACCCAT TTCATGCTGG TAAGTGAAAT GCCGCACGGT GGGCAGAAAC  50

TTTCTGGTTA CGGCAAGGAT ATGTCACTTT ATGGGCTGGA GGATTACACC 100
<210> 1879
<211> 100
<212> DNA
<213> Escherichia coli K-12 MG1655
<220> b1444 b1444
<222> 1241-1340
<223> b1444 b1444 U00096 1513602..1515026
<313> 1879: 1259-1278
<400> 1879
ATGTGGGCAG GGCGCATCGC GTCAGCGCAC GGCTGCAATA TGGTTGTACC 50
TGGGTCAATA CCCATTTCAT GCTGGTAAGT GAAATGCCGC ACGGTGGGCA 100
<210> 1880
<211> 100
<212> DNA
<213> Escherichia coli K-12 MG1655
<220> yafp yafP
<222> 354-453
<223> yafp yafP U00096 252709..253161
<313> 1880: 407-426
<400> 1880
TTTTGAACGT TATGGTTTTC AGACAGTTAA GCAGCAGCGC GTTGAATGCC 50
GGGGAGCGTG GTTTACTAAT TTTTATATGC GATATAAACC GCAACATTAA 100
<210> 1881
<211> 100
<212> DNA
<213> Escherichia coli K-12 MG1655
<220> yafp yafP
<222> 280-379
<223> yafp yafP U00096 252709..253161
<313> 1881: 339-358
<400> 1881
AGCGCTTTGT TAAAACCTTT GATTAAGTCT GAATCCGAAC TTACGGTGGA 50
CGCAAGCATA ACCGCAAAAC CCTTTTTTGA ACGTTATGGT TTTCAGACAG 100
<210> 1882
<211> 100
<212> DNA
<213> Escherichia coli K-12 MG1655
<220> yafp yafP
<222> 214-313
<223> yafp yafP U00096 252709..253161
<313> 1882: 242-261
<400> 1882
TCCCGCATTG AACATTATAT CGATATGTTA TTTGTTGACC CTGAATACAC 50
CCGCCGTGGG GTTGCCAGCG CTTTGTTAAA ACCTTTGATT AAGTCTGAAT 100
<210> 1883
<211> 100
<212> DNA
<213> Escherichia coli K-12 MG1655
<220> acka b2297
<222> 1104-1203
<223> acka b2297 U00096 2411490..2412692
<313> 1883: 1179-1198
<400> 1883
ATCTGGTTTC ATCAACAAAG AAGGTACCCG TCCTGCGGTG GTTATCCCAA 50
CCAACGAAGA ACTGGTTATC GCGCAAGACG CGAGCCGCCT GACTGCCTGA 100
<210> 1884
<211> 100
<212> DNA
<213> Escherichia coli K-12 MG1655
<220> acka b2297
<222> 1051-1150
<223> acka b2297 U00096 2411490..2412692
<313> 1884: 1106-1125
<400> 1884
GTGCTGGGCT TTGAAGTTGA TCATGAACGC AACCTGGCTG CACGTTTCGG 50
CAAATCTGGT TTCATCAACA AAGAAGGTAC CCGTCCTGCG GTGGTTATCC 100
<210> 1885
<211> 100
<212> DNA
<213> Escherichia coli K-12 MG1655

<220> acka b2297
<222> 849-948
<223> acka b2297 U00096 2411490..2412692
<313> 1885: 882-901
<400> 1885
CAGCGACTGC CGCTATGTTG AAGACAACTA CGCGACGAAA GAAGACGCGA  50
AGCGCGCAAT GGACGTTTAC TGCCACCGCC TGGCGAAATA CATCGGTGCC  100
<210> 1886
<211> 100
<212> DNA
<213> Escherichia coli K-12 MG1655
<220> b1445 b1445
<222> 150-249
<223> b1445 b1445 U00096 1515338..1515586
<313> 1886: 195-214
<400> 1886
TTCTCACGAT AAGAAACGCA TCCGTTTCTT AAGCGCCTTT CTGGTGGGGG  50
CAACATGGCC GATGAGTTTC CCGGTGGCGC TGTTGTTTTC ACTGTTTTAA  100
<210> 1887
<211> 100
<212> DNA
<213> Escherichia coli K-12 MG1655
<220> b1445 b1445
<222> 102-201
<223> b1445 b1445 U00096 1515338..1515586
<313> 1887: 138-157
<400> 1887
TTTTTATGCG GTTATGGCAG CAATCGCATT TCTTATCACC TGGTTTCTTT  50
CTCACGATAA GAAACGCATC CGTTTCTTAA GCGCCTTTCT GGTGGGGGCA  100
<210> 1888
<211> 100
<212> DNA
<213> Escherichia coli K-12 MG1655
<220> b1445 b1445
<222> 29-128
<223> b1445 b1445 U00096 1515338..1515586
<313> 1888: 85-104
<400> 1888
TGACGCATAT TTGCGCGCGT TTTATTCATC TGGCTGGACG CCCGTACATG  50
TCTCTCTATC AACACATGCT TGTTTTTTAT GCGGTTATGG CAGCAATCGC  100
<210> 1889
<211> 100
<212> DNA
<213> Escherichia coli K-12 MG1655
<220> b2174 b2175
<222> 625-724
<223> b2174 b2175 U00096 2266838..2267587
<313> 1889: 652-671
<400> 1889
GTCGGTTCGA TGACCGTGAT ATTGATCGGT TTGCCCTGGG TGTTGCTGAC  50
GCCATTAAGT GATCGATTAA TCACCTTTTT TGACAAATCA CTACCAGGAA  100
<210> 1890
<211> 100
<212> DNA
<213> Escherichia coli K-12 MG1655
<220> b2174 b2175
<222> 547-646
<223> b2174 b2175 U00096 2266838..2267587
<313> 1890: 600-619
<400> 1890
CTTATCGCCC TTATTATTTT TGTGGTTTTT GCATTTCCCA GAGTAATGAT  50
TGGCGCACAC TGGTTTACTG ACATCATTGT CGGTTCGATG ACCGTGATAT  100
<210> 1891
<211> 100
<212> DNA
<213> Escherichia coli K-12 MG1655
<220> b2174 b2175
<222> 451-550
<223> b2174 b2175 U00096 2266838..2267587
<313> 1891: 486-505
<400> 1891

AAAGATGCCT CACGAGATAG CTTTCCCGGC GATCACGGCA TGATGCTGCT 50
TATTTTTTCG GCATTCATGT GGCGTTATTT CGGCAAAGTT GCAGGCCTTA 100
<210> 1892
<211> 100
<212> DNA
<213> Escherichia coli K-12 MG1655
<220> yafq b0226
<222> 180-279
<223> yafq b0226 U00096 complement(245961..246239)
<313> 1892: 242-261
<400> 1892
CGATGCTCAT GTCGAACCGG ACTGGATCCT GATTTACAAA CTTACCGATA 50
AACTTTTACG ATTTGAGAGA ACTGGAACTC ACGCGGCGCT CTTTGGGTAA 100
<210> 1893
<211> 100
<212> DNA
<213> Escherichia coli K-12 MG1655
<220> yafq b0226
<222> 180-279
<223> yafq b0226 U00096 complement(245961..246239)
<313> 1893: 242-261
<400> 1893
CGATGCTCAT GTCGAACCGG ACTGGATCCT GATTTACAAA CTTACCGATA 50
AACTTTTACG ATTTGAGAGA ACTGGAACTC ACGCGGCGCT CTTTGGGTAA 100
<210> 1894
<211> 100
<212> DNA
<213> Escherichia coli K-12 MG1655
<220> yafq b0226
<222> 180-279
<223> yafq b0226 U00096 complement(245961..246239)
<313> 1894: 234-253
<400> 1894
CGATGCTCAT GTCGAACCGG ACTGGATCCT GATTTACAAA CTTACCGATA 50
AACTTTTACG ATTTGAGAGA ACTGGAACTC ACGCGGCGCT CTTTGGGTAA 100
<210> 1895
<211> 100
<212> DNA
<213> Escherichia coli K-12 MG1655
<220> xsea xseA
<222> 1253-1352
<223> xsea xseA U00096 2632252..2633622
<313> 1895: 1292-1311
<400> 1895
AAAAAGTGAA GCAAGTTAAA GCGGGTGAAA TGCTAACCAC ACGTCTGGAA 50
GACGGCTGGA TAGAAAGTGA AGTAAAAAAC ATCCAGCCAG TAAAAAAATC 100
<210> 1896
<211> 100
<212> DNA
<213> Escherichia coli K-12 MG1655
<220> xsea xseA
<222> 1198-1297
<223> xsea xseA U00096 2632252..2633622
<313> 1896: 1245-1264
<400> 1896
TCAACGCTGG CGCGTGGATA CAGCGTTACT ACTGCTACTG ACGGCAATGT 50
ACTGAAAAAA GTGAAGCAAG TTAAAGCGGG TGAAATGCTA ACCACACGTC 100
<210> 1897
<211> 100
<212> DNA
<213> Escherichia coli K-12 MG1655
<220> xsea xseA
<222> 1122-1221
<223> xsea xseA U00096 2632252..2633622
<313> 1897: 1181-1200
<400> 1897
CCTGCGCGCA CAGCTTAGCG CCACGCGTGA ACGTTTCGGT AATGCAGTAA 50
CGCACCTCGA AGCCGTAAGC CCACTGTCAA CGCTGGCGCG TGGATACAGC 100
<210> 1898
<211> 100
<212> DNA

<213> Escherichia coli K-12 MG1655
<220> b1446 b1446
<222> 135-234
<223> b1446 b1446 U00096 1515672..1515905
<313> 1898: 209-228
<400> 1898
GCAACGTCTG CGCACAGCGA TCGCCCATCA CGGTCGCAAG CATAAAGAAG 50
ATATGGAAGC GCGCCACGAA CAGTTAACCA AAGGCGGCAC CATCCTCTGA 100
<210> 1899
<211> 100
<212> DNA
<213> Escherichia coli K-12 MG1655
<220> b1446 b1446
<222> 129-228
<223> b1446 b1446 U00096 1515672..1515905
<313> 1899: 164-183
<400> 1899
CCAGCAGCAA CGTCTGCGCA CAGCGATCGC CCATCACGGT CGCAAGCATA 50
AAGAAGATAT GGAAGCGCGC CACGAACAGT TAACCAAAGG CGGCACCATC 100
<210> 1900
<211> 100
<212> DNA
<213> Escherichia coli K-12 MG1655
<220> b1446 b1446
<222> 92-191
<223> b1446 b1446 U00096 1515672..1515905
<313> 1900: 110-129
<400> 1900
GCGATGACGC GGAGTTAAGT CGGGAAGATC GCTACACCCA GCAGCAACGT 50
CTGCGCACAG CGATCGCCCA TCACGGTCGC AAGCATAAAG AAGATATGGA 100
<210> 1901
<211> 100
<212> DNA
<213> Escherichia coli K-12 MG1655
<220> ypha b2544
<222> 396-495
<223> ypha b2544 U00096 2671294..2671788
<313> 1901: 453-472
<400> 1901
GCCAAATATG ATTAATTTCT GGAAGAATGT CAGTATCGCT GGCGCGTTCT 50
TGCTATTGGC AATTACCGGG CCGGGGGCAA TTTCTCTCGA TCGGCGTTAG 100
<210> 1902
<211> 100
<212> DNA
<213> Escherichia coli K-12 MG1655
<220> ypha b2544
<222> 336-435
<223> ypha b2544 U00096 2671294..2671788
<313> 1902: 390-409
<400> 1902
CACGCTGGGT ACGGCGGTGA TTGGTCACCA TTACTGGGAT ATGACCGGCG 50
ATGCGGTTGG GCCAAATATG ATTAATTTCT GGAAGAATGT CAGTATCGCT 100
<210> 1903
<211> 100
<212> DNA
<213> Escherichia coli K-12 MG1655
<220> ypha b2544
<222> 295-394
<223> ypha b2544 U00096 2671294..2671788
<313> 1903: 340-359
<400> 1903
GGCTTTTTCA CCCGTCCGCT GGCGGTGCTG TTTATTTTCT ACACGCTGGG 50
TACGGCGGTG ATTGGTCACC ATTACTGGGA TATGACCGGC GATGCGGTTG 100
<210> 1904
<211> 100
<212> DNA
<213> Escherichia coli K-12 MG1655
<220> xseb b0423
<222> 144-243
<223> xseb b0423 U00096 complement(440325..440567)
<313> 1904: 209-228

<400> 1904
GGGGCAGGCC AAATTACAAC AAGCCGAACA GCGCGTACAA ATTCTGCTGT  50
CTGACAATGA AGACGCCTCT CTAACCCCTT TTACACCGGA CAATGAGTAA  100
<210> 1905
<211> 100
<212> DNA
<213> Escherichia coli K-12 MG1655
<220> xseb b0423
<222> 129-228
<223> xseb b0423 U00096 complement(440325..440567)
<313> 1905: 171-190
<400> 1905
GCAGCTGGCA CGTCAGGGGC AGGCCAAATT ACAACAAGCC GAACAGCGCG  50
TACAAATTCT GCTGTCTGAC AATGAAGACG CCTCTCTAAC CCCTTTTACA  100
<210> 1906
<211> 100
<212> DNA
<213> Escherichia coli K-12 MG1655
<220> xseb b0423
<222> 11-110
<223> xseb b0423 U00096 complement(440325..440567)
<313> 1906: 68-87
<400> 1906
AAAATGAGGC GCCCGCCAGC TTTGAAAAGG CGCTGAGCGA GCTGGAACAG  50
ATTGTAACCC GTCTGGAAAG TGGCGACCTG CCGCTGGAAG AGGCGCTGAA  100
<210> 1907
<211> 100
<212> DNA
<213> Escherichia coli K-12 MG1655
<220> b1447 b1447
<222> 351-450
<223> b1447 b1447 U00096 complement(1515906..1516355)
<313> 1907: 395-414
<400> 1907
CCGCAGTCAT GGCGTACCAC TACGGGCGTT GTTCGGCCCA ATTTGTGGCG  50
CAATTTTACT TGTGGTTGGC GCCTGGTTGG TAGCCAGACG CTCATTTTAA  100
<210> 1908
<211> 100
<212> DNA
<213> Escherichia coli K-12 MG1655
<220> b1447 b1447
<222> 249-348
<223> b1447 b1447 U00096 complement(1515906..1516355)
<313> 1908: 288-307
<400> 1908
TGTCTTCGCC AGTATCAGCG GATATCAGAA TGTGGGAGCG GCGACAACTA  50
TTGCTGTACT GGTCGCCAGT CAGCTGATTG GCGGTCTGAT GCTGGATATC  100
<210> 1909
<211> 100
<212> DNA
<213> Escherichia coli K-12 MG1655
<220> b1447 b1447
<222> 200-299
<223> b1447 b1447 U00096 complement(1515906..1516355)
<313> 1909: 248-267
<400> 1909
CCAGCGTGCG CTGGTGGACA CTTATACCTG GCTTGCTGGG ATCGTTTTTT  50
GTCTTCGCCA GTATCAGCGG ATATCAGAAT GTGGGAGCGG CGACAACTAT  100
<210> 1910
<211> 100
<212> DNA
<213> Escherichia coli K-12 MG1655
<220> yphb b2545
<222> 774-873
<223> yphb b2545 U00096 complement(2671836..2672708)
<313> 1910: 844-863
<400> 1910
GCCGGATGAT CATCATCGCC CGGAAGGGGG TGATCTCATT GCGCTAGCAC  50
CAGGGGAATC AACAACTTCA GAGATGTCGT TGCGGGTAGA GTGGCTGTAA  100
<210> 1911
<211> 100

<212> DNA
<213> Escherichia coli K-12 MG1655
<220> yphb b2545
<222> 730-829
<223> yphb b2545 U00096 complement(2671836..2672708)
<313> 1911: 749-768
<400> 1911
GGATATGCGT TTGATTTCTT CTGTCTGGAA CCGATGAGCC ATGCGCCGGA 50
TGATCATCAT CGCCCGGAAG GGGGTGATCT CATTGCGCTA GCACCAGGGG 100
<210> 1912
<211> 100
<212> DNA
<213> Escherichia coli K-12 MG1655
<220> yphb b2545
<222> 656-755
<223> yphb b2545 U00096 complement(2671836..2672708)
<313> 1912: 720-739
<400> 1912
ATGCCATCAT CATGGAAACG ACGCCACCTG CACCGTGTTA TTTCATCTTT 50
GTTTCCGACC CTGCGTTTGA TAAAGGATAT GCGTTTGATT TCTTCTGTCT 100
<210> 1913
<211> 100
<212> DNA
<213> Escherichia coli K-12 MG1655
<220> yafs b0214
<222> 642-741
<223> yafs b0214 U00096 234798..235538
<313> 1913: 712-731
<400> 1913
TATTCCTTTA ACGCTAAATC CGATGAAACA GAGTAAAAAC AAGCCACGAA 50
TTCGCCAGGC GGTTGGAGCC ACCCGGCAAT GTCGTAAACC ACAGGCTTAA 100
<210> 1914
<211> 100
<212> DNA
<213> Escherichia coli K-12 MG1655
<220> yafs b0214
<222> 609-708
<223> yafs b0214 U00096 234798..235538
<313> 1914: 636-655
<400> 1914
TTGCTTACAA CTTATTGTTG CCCGGAAACG GACTATTCCT TTAACGCTAA 50
ATCCGATGAA ACAGAGTAAA AACAAGCCAC GAATTCGCCA GGCGGTTGGA 100
<210> 1915
<211> 100
<212> DNA
<213> Escherichia coli K-12 MG1655
<220> yafs b0214
<222> 539-638
<223> yafs b0214 U00096 234798..235538
<313> 1915: 557-576
<400> 1915
GCCGTTTCCA CGTTCTCCCG TGGAACAAAC ACGGAGGAAA ACTATTGAAT 50
GCGCATATTC CTGCGCTTGG TTGCTTACAA CTTATTGTTG CCCGGAAACG 100
<210> 1916
<211> 100
<212> DNA
<213> Escherichia coli K-12 MG1655
<220> b1448 b1449
<222> 420-519
<223> b1448 b1449 U00096 complement(1516352..1516870)
<313> 1916: 488-507
<400> 1916
GCAAATGCCG CAGGTAGGCA CTAAATTTGG TCGTTGGCTG GATCTGACAT 50
TTATGCAGTT GCAACTCGAC GAGCGCACTG AACCGGACGC GATTGGATGA 100
<210> 1917
<211> 100
<212> DNA
<213> Escherichia coli K-12 MG1655
<220> b1448 b1449
<222> 358-457
<223> b1448 b1449 U00096 complement(1516352..1516870)

<313> 1917: 415-434
<400> 1917
ATCGAATCGC AAAATCAGGC CTCGCTGCAT CTCCACCAGT CGCTGGGATT 50
TGTCGTCACC GCGCAAATGC CGCAGGTAGG CACTAAATTT GGTCGTTGGC 100
<210> 1918
<211> 100
<212> DNA
<213> Escherichia coli K-12 MG1655
<220> b1448 b1449
<222> 323-422
<223> b1448 b1449 U00096 complement(1516352..1516870)
<313> 1918: 349-368
<400> 1918
CGCGGGATTG CGGGAAGCAT GTCATGGTCG CCGGGATCGA ATCGCAAAAT 50
CAGGCCTCGC TGCATCTCCA CCAGTCGCTG GGATTTGTCG TCACCGCGCA 100
<210> 1919
<211> 100
<212> DNA
<213> Escherichia coli K-12 MG1655
<220> yphc b2546
<222> 996-1095
<223> yphc b2546 U00096 complement(2672720..2673814)
<313> 1919: 1060-1079
<400> 1919
CGCCATTACC CATCGCTTCT CGCTGGAACA GGCAGGTGAT GCCTATGCGC 50
TGATGGCGAG CGGCAAATGC GGGAAAGTTG TGATTAACTT CCCGGATTAA 100
<210> 1920
<211> 100
<212> DNA
<213> Escherichia coli K-12 MG1655
<220> yphc b2546
<222> 974-1073
<223> yphc b2546 U00096 complement(2672720..2673814)
<313> 1920: 1009-1028
<400> 1920
ACTGGAAGCT GTGGCCGCGT AACGCCATTA CCCATCGCTT CTCGCTGGAA 50
CAGGCAGGTG ATGCCTATGC GCTGATGGCG AGCGGCAAAT GCGGGAAAGT 100
<210> 1921
<211> 100
<212> DNA
<213> Escherichia coli K-12 MG1655
<220> yphc b2546
<222> 901-1000
<223> yphc b2546 U00096 complement(2672720..2673814)
<313> 1921: 955-974
<400> 1921
CATCAACGGC GGATTATCGG CTCCTGGGTG ACCAGTCTGT TCCATATGGA 50
AAAATGCGCC CATGATCTGA CGGACTGGAA GCTGTGGCCG CGTAACGCCA 100
<210> 1922
<211> 100
<212> DNA
<213> Escherichia coli K-12 MG1655
<220> yaft b0218
<222> 647-746
<223> yaft b0218 U00096 237335..238120
<313> 1922: 704-723
<400> 1922
ATTATGAATA TAAAGTGCTT CCACCATTGC CCGACAAGAA TGAAGCCAGT 50
ACGACTGATA TGACAGAGCA GCAAATCATG CAACTTAAGC AAAAGACCTA 100
<210> 1923
<211> 100
<212> DNA
<213> Escherichia coli K-12 MG1655
<220> yaft b0218
<222> 515-614
<223> yaft b0218 U00096 237335..238120
<313> 1923: 574-593
<400> 1923
CTGCGCTGAA ATCAACAGTA TGGTCCGACT ATAAAAATGA CAAACTCACT 50
GACGCTATCT CCCTGCGCTA CCTGGTACGT TTCACGCTGG TGGATGTGGC 100
<210> 1924

<211> 100
<212> DNA
<213> Escherichia coli K-12 MG1655
<220> yaft b0218
<222> 459-558
<223> yaft b0218 U00096 237335..238120
<313> 1924: 497-516
<400> 1924
ACATCAGAAA GCGATTATTG TTTACCAGGA TATGTTGCAG ACAGGAAAAT 50
ATGACTCTGC GCTGAAATCA ACAGTATGGT CCGACTATAA AAATGACAAA 100
<210> 1925
<211> 100
<212> DNA
<213> Escherichia coli K-12 MG1655
<220> yphd b2547
<222> 900-999
<223> yphd b2547 U00096 complement(2673847..2674845)
<313> 1925: 972-991
<400> 1925
TAACTCCTTT TTCCAGCAGG TGGTACGCGG CGTCATCATC GTGGTGGCGG 50
TGCTGGCGAA TATCTTGCTG ACCCAGCGAA GCAGTAAAGC GAAACGCTAA 100
<210> 1926
<211> 100
<212> DNA
<213> Escherichia coli K-12 MG1655
<220> yphd b2547
<222> 873-972
<223> yphd b2547 U00096 complement(2673847..2674845)
<313> 1926: 904-923
<400> 1926
CGGTAACGGT CTGGTGCTGC TCGGTATTAA CTCCTTTTTC CAGCAGGTGG 50
TACGCGGCGT CATCATCGTG GTGGCGGTGC TGGCGAATAT CTTGCTGACC 100
<210> 1927
<211> 100
<212> DNA
<213> Escherichia coli K-12 MG1655
<220> yphd b2547
<222> 725-824
<223> yphd b2547 U00096 complement(2673847..2674845)
<313> 1927: 759-778
<400> 1927
CGGCGCGCCT CGGTTCTGGT AACGCAGGTG CCGCAAACGG TCTGGAGTTT 50
GACGTCATCG CCGCGGTCGT GGTCGGCGGT ACGGCACTTT CCGGCGGTCG 100
<210> 1928
<211> 100
<212> DNA
<213> Escherichia coli K-12 MG1655
<220> yafu b0219
<222> 240-339
<223> yafu b0219 U00096 complement(238746..239084)
<313> 1928: 306-325
<400> 1928
AATTAGAGAT GGACTAAAAC GGCAACAGGA ACTTGATAAA TGGAAAAGGG 50
AAAACATGAC TCCATTTATG TATGTTCTTA ACACTCCACC CGTGATATGA 100
<210> 1929
<211> 100
<212> DNA
<213> Escherichia coli K-12 MG1655
<220> yafu b0219
<222> 240-339
<223> yafu b0219 U00096 complement(238746..239084)
<313> 1929: 297-316
<400> 1929
AATTAGAGAT GGACTAAAAC GGCAACAGGA ACTTGATAAA TGGAAAAGGG 50
AAAACATGAC TCCATTTATG TATGTTCTTA ACACTCCACC CGTGATATGA 100
<210> 1930
<211> 100
<212> DNA
<213> Escherichia coli K-12 MG1655
<220> yafu b0219
<222> 235-334

<223> yafu b0219 U00096 complement(238746..239084)
<313> 1930: 294-313
<400> 1930
TATGCAATTA GAGATGGACT AAAACGGCAA CAGGAACTTG ATAAATGGAA 50
AAGGGAAAAC ATGACTCCAT TTATGTATGT TCTTAACACT CCACCCGTGA 100
<210> 1931
<211> 100
<212> DNA
<213> Escherichia coli K-12 MG1655
<220> yphe b2548
<222> 1413-1512
<223> yphe b2548 U00096 complement(2674870..2676381)
<313> 1931: 1476-1495
<400> 1931
TGACCGCATC CTGTTATTAC AGCACGGCAC GTTCTCGCAG GAGTTTCACG 50
CTCCGGTCAA TGTGGATGAG CTGATGTCCG CCATTCTGTC TGTGCACTGA 100
<210> 1932
<211> 100
<212> DNA
<213> Escherichia coli K-12 MG1655
<220> yphe b2548
<222> 1377-1476
<223> yphe b2548 U00096 complement(2674870..2676381)
<313> 1932: 1444-1463
<400> 1932
TATCTCCAGT GAAGTGGAGG AGTTGCCGCT GGTGTGTGAC CGCATCCTGT 50
TATTACAGCA CGGCACGTTC TCGCAGGAGT TTCACGCTCC GGTCAATGTG 100
<210> 1933
<211> 100
<212> DNA
<213> Escherichia coli K-12 MG1655
<220> yphe b2548
<222> 1341-1440
<223> yphe b2548 U00096 complement(2674870..2676381)
<313> 1933: 1365-1384
<400> 1933
CCGTGAGCTG GCTGCCGAAG GAAAAAGCGT GGTGTTTATC TCCAGTGAAG 50
TGGAGGAGTT GCCGCTGGTG TGTGACCGCA TCCTGTTATT ACAGCACGGC 100
<210> 1934
<211> 100
<212> DNA
<213> Escherichia coli K-12 MG1655
<220> yafv b0220
<222> 672-771
<223> yafv b0220 U00096 complement(239419..240189)
<313> 1934: 737-756
<400> 1934
GGCAACGCGC ATTGATGCGG AGCTGTCGAT GGCAGCATTG CGGGAATATA 50
GAGAAAAGTT TCCGGCATGG CAGGATGCGG ATGAGTTTAG GTTGTGGTGA 100
<210> 1935
<211> 100
<212> DNA
<213> Escherichia coli K-12 MG1655
<220> yafv b0220
<222> 628-727
<223> yafv b0220 U00096 complement(239419..240189)
<313> 1935: 664-683
<400> 1935
ATTAATCCGC AAGGAGAGAT TATCGCTACT GCCGACGCGC ATCAGGCAAC 50
GCGCATTGAT GCGGAGCTGT CGATGGCAGC ATTGCGGGAA TATAGAGAAA 100
<210> 1936
<211> 100
<212> DNA
<213> Escherichia coli K-12 MG1655
<220> yafv b0220
<222> 515-614
<223> yafv b0220 U00096 complement(239419..240189)
<313> 1936: 564-583
<400> 1936
ACTGGCAGGC ATTGCTGACG GCCCGCGCGA TTGAGAATCA GGCGTATGTG 50
GCGGGATGCA ATCGCGTCGG CAGCGATGGC AACGGCTGCC ATTATCGCGG 100

<210> 1937
<211> 100
<212> DNA
<213> Escherichia coli K-12 MG1655
<220> yphf b2549
<222> 885-984
<223> yphf b2549 U00096 complement(2676404..2677387)
<313> 1937: 947-966
<400> 1937
AGCCGACGGT GAAAAAGTGA TTCAGGTGCC TATCGATCTC TATACCAAAA 50
CGGAAGACGG TAAACAGTGG CTGGCAACGC ACGTTGATGG TCTGCCCTAA 100
<210> 1938
<211> 100
<212> DNA
<213> Escherichia coli K-12 MG1655
<220> yphf b2549
<222> 840-939
<223> yphf b2549 U00096 complement(2676404..2677387)
<313> 1938: 893-912
<400> 1938
GGGCAATGCT GTTTTCGCGC AAACATTGAA GGTTATCAAT AAACAAGCCG 50
ACGGTGAAAA AGTGATTCAG GTGCCTATCG ATCTCTATAC CAAAACGGAA 100
<210> 1939
<211> 100
<212> DNA
<213> Escherichia coli K-12 MG1655
<220> yphf b2549
<222> 775-874
<223> yphf b2549 U00096 complement(2676404..2677387)
<313> 1939: 810-829
<400> 1939
ATTGCTCAGG AGCTGGAAAA CAATCAGGTG CTGAAAGCGG TAGTGGATAT 50
TTCCGGTAAG AAAATGGGCA ATGCTGTTTT CGCGCAAACA TTGAAGGTTA 100
<210> 1940
<211> 100
<212> DNA
<213> Escherichia coli K-12 MG1655
<220> yafw b0247
<222> 219-318
<223> yafw b0247 U00096 complement(262914..263231)
<313> 1940: 284-303
<400> 1940
CCACTGCGTT ACCCTGTACC ACAACGATTT AACCTGCGAA GCCGACACCC 50
TTGGCAGTTG CGGCTACGTA TACATCGCCA TTTACCCCAC TCAGCGTTAA 100
<210> 1941
<211> 100
<212> DNA
<213> Escherichia coli K-12 MG1655
<220> yafw b0247
<222> 193-292
<223> yafw b0247 U00096 complement(262914..263231)
<313> 1941: 249-268
<400> 1941
ACCGGTGAAC TCAGCCCCCA CCATGCCCAC TGCGTTACCC TGTACCACAA 50
CGATTTAACC TGCGAAGCCG ACACCCTTGG CAGTTGCGGC TACGTATACA 100
<210> 1942
<211> 100
<212> DNA
<213> Escherichia coli K-12 MG1655
<220> yafw b0247
<222> 185-284
<223> yafw b0247 U00096 complement(262914..263231)
<313> 1942: 216-235
<400> 1942
TGCTGACCAC CGGTGAACTC AGCCCCCACC ATGCCCACTG CGTTACCCTG 50
TACCACAACG ATTTAACCTG CGAAGCCGAC ACCCTTGGCA GTTGCGGCTA 100
<210> 1943
<211> 100
<212> DNA
<213> Escherichia coli K-12 MG1655
<220> yphg b2550

<222> 3276-3375
<223> yphg b2550 U00096 complement(2677484..2680858)
<313> 1943: 3351-3370
<400> 1943
TGAGTCTGCT CGCGCTGAAT TGACGGCGAT TAATCCGGCT TGGCCGAAAG 50
CGGCATTATT CACCACCGTG ATGCCTTTTA TTTTTAACCG CGTTCACTAA 100
<210> 1944
<211> 100
<212> DNA
<213> Escherichia coli K-12 MG1655
<220> yphg b2550
<222> 3232-3331
<223> yphg b2550 U00096 complement(2677484..2680858)
<313> 1944: 3273-3292
<400> 1944
GTGGCGATGC TGGCGTCCGC GGGACTCGGG GAGGTTGCGC AATATGAGTC 50
TGCTCGCGCT GAATTGACGG CGATTAATCC GGCTTGGCCG AAAGCGGCAT 100
<210> 1945
<211> 100
<212> DNA
<213> Escherichia coli K-12 MG1655
<220> yphg b2550
<222> 3184-3283
<223> yphg b2550 U00096 complement(2677484..2680858)
<313> 1945: 3211-3230
<400> 1945
TCGCTGTATG GCGATTTACA ACAGCAGCAT AAAGAAAAAT GCCTGATGGT 50
GGCGATGCTG GCGTCCGCGG GACTCGGGGA GGTTGCGCAA TATGAGTCTG 100
<210> 1946
<211> 100
<212> DNA
<213> Escherichia coli K-12 MG1655
<220> yafx b0249
<222> 360-459
<223> yafx b0249 U00096 complement(263972..264430)
<313> 1946: 437-456
<400> 1946
CCGTACAGAA TGTGACGCGA TGACCGCACA CTATTACCGC CTGCGGGAGT 50
ATGCCATGCA ACATCCAGAG GCTCACGCCA TTCTGCGTAT TATCGACTGA 100
<210> 1947
<211> 100
<212> DNA
<213> Escherichia coli K-12 MG1655
<220> yafx b0249
<222> 351-450
<223> yafx b0249 U00096 complement(263972..264430)
<313> 1947: 369-388
<400> 1947
CCATGCCTGC CGTACAGAAT GTGACGCGAT GACCGCACAC TATTACCGCC 50
TGCGGGAGTA TGCCATGCAA CATCCAGAGG CTCACGCCAT TCTGCGTATT 100
<210> 1948
<211> 100
<212> DNA
<213> Escherichia coli K-12 MG1655
<220> yafx b0249
<222> 303-402
<223> yafx b0249 U00096 complement(263972..264430)
<313> 1948: 330-349
<400> 1948
GAGTGCAGAA GCAGCAGGTA TTGCTGTCTG CCTGATTGCG TATAGCCACC 50
ATGCCTGCCG TACAGAATGT GACGCGATGA CCGCACACTA TTACCGCCTG 100
<210> 1949
<211> 100
<212> DNA
<213> Escherichia coli K-12 MG1655
<220> yphh b2551
<222> 1101-1200
<223> yphh b2551 U00096 2680877..2682076
<313> 1949: 1173-1192
<400> 1949
CGTGAAAGCG ACGCAAATTG GCTTTGGGCA ATTAAGCCGC GCACAACAGG 50

TGCTGGGAAT TGGCTATTTG TATGTTGAGG CGCAGTTACG ACAGATTTGA 100
<210> 1950
<211> 100
<212> DNA
<213> Escherichia coli K-12 MG1655
<220> yphh b2551
<222> 1064-1163
<223> yphh b2551 U00096 2680877..2682076
<313> 1950: 1115-1134
<400> 1950
CAGGATTTAA CCCGTTCGAC CGCGACGAAG GACCGAGCGT GAAAGCGACG 50
CAAATTGGCT TTGGGCAATT AAGCCGCGCA CAACAGGTGC TGGGAATTGG 100
<210> 1951
<211> 100
<212> DNA
<213> Escherichia coli K-12 MG1655
<220> yphh b2551
<222> 1018-1117
<223> yphh b2551 U00096 2680877..2682076
<313> 1951: 1071-1090
<400> 1951
AGTTGTGCCT TTGGTGAGAA CTGGCTTAAT ACTATTATTC GCCAGACAGG 50
ATTTAACCCG TTCGACCGCG ACGAAGGACC GAGCGTGAAA GCGACGCAAA 100
<210> 1952
<211> 100
<212> DNA
<213> Escherichia coli K-12 MG1655
<220> yafy b0252
<222> 759-858
<223> yafy b0252 U00096 complement(265334..266191)
<313> 1952: 836-855
<400> 1952
GACGGACCCG TATACCAAAG AAAAATACTT TGAGTACGCC CATGACGGCA 50
AACTGATGGG GCAGCTACTG ATACCCTCGA ACCCTGACAA TCAGGAATAA 100
<210> 1953
<211> 100
<212> DNA
<213> Escherichia coli K-12 MG1655
<220> yafy b0252
<222> 757-856
<223> yafy b0252 U00096 complement(265334..266191)
<313> 1953: 787-806
<400> 1953
GTGACGGACC CGTATACCAA AGAAAAATAC TTTGAGTACG CCCATGACGG 50
CAAACTGATG GGGCAGCTAC TGATACCCTC GAACCCTGAC AATCAGGAAT 100
<210> 1954
<211> 100
<212> DNA
<213> Escherichia coli K-12 MG1655
<220> yafy b0252
<222> 713-812
<223> yafy b0252 U00096 complement(265334..266191)
<313> 1954: 745-764
<400> 1954
ATGCGCTGTG TATTGCCGAT AACATCAAAC TGGAATACCA GGAGGTGACG 50
GACCCGTATA CCAAAGAAAA ATACTTTGAG TACGCCCATG ACGGCAAACT 100
<210> 1955
<211> 100
<212> DNA
<213> Escherichia coli K-12 MG1655
<220> yafz b0253
<222> 738-837
<223> yafz b0253 U00096 complement(266408..267244)
<313> 1955: 795-814
<400> 1955
ACGGTCACAT ACCCGTGCCG TTCGCGGTAT CGACGGGGAC GTGAAACTTA 50
ACCGGGCACT GTGGGTGATG GCGGAAGCCC TGCTCACGCA ACTGCAGTGA 100
<210> 1956
<211> 100
<212> DNA
<213> Escherichia coli K-12 MG1655

<220> yafz b0253
<222> 652-751
<223> yafz b0253 U00096 complement(266408..267244)
<313> 1956: 682-701
<400> 1956
GAGAGCAATG ACCTGTGGAC CACGTACCAG CGTATTCAGG AGAACCTGAT 50
TAAGGGCGGG CTCAGTGGCC GTAATGCCAA AGGAGGACGG TCACATACCC 100
<210> 1957
<211> 100
<212> DNA
<213> Escherichia coli K-12 MG1655
<220> yafz b0253
<222> 620-719
<223> yafz b0253 U00096 complement(266408..267244)
<313> 1957: 655-674
<400> 1957
CGCAGATCCT CTCCCCTCGC CGCTGGCAGG ATGAGAGCAA TGACCTGTGG 50
ACCACGTACC AGCGTATTCA GGAGAACCTG ATTAAGGGCG GGCTCAGTGG 100
<210> 1958
<211> 100
<212> DNA
<213> Escherichia coli K-12 MG1655
<220> fklb b4208
<222> 681-780
<223> fklb b4208 U00096 4426354..4427133
<313> 1958: 747-766
<400> 1958
TATCCCGCAG GAACTGGCAT ATGGCGAGCG CGGCGCAGGC GCATCCATCC 50
CTCCGTTCAG CACCCTGGTG TTTGAAGTCG AACTGCTGGA AATCCTCTAA 100
<210> 1959
<211> 100
<212> DNA
<213> Escherichia coli K-12 MG1655
<220> fklb b4208
<222> 667-766
<223> fklb b4208 U00096 4426354..4427133
<313> 1959: 720-739
<400> 1959
AAATGGGAAC TGACTATCCC GCAGGAACTG GCATATGGCG AGCGCGGCGC 50
AGGCGCATCC ATCCCTCCGT TCAGCACCCT GGTGTTTGAA GTCGAACTGC 100
<210> 1960
<211> 100
<212> DNA
<213> Escherichia coli K-12 MG1655
<220> fklb b4208
<222> 635-734
<223> fklb b4208 U00096 4426354..4427133
<313> 1960: 668-687
<400> 1960
TTGAAGCACT GACTCTGATG CCGGTAGGTT CTAAATGGGA ACTGACTATC 50
CCGCAGGAAC TGGCATATGG CGAGCGCGGC GCAGGCGCAT CCATCCCTCC 100
<210> 1961
<211> 100
<212> DNA
<213> Escherichia coli K-12 MG1655
<220> gip b0509
<222> 678-777
<223> gip b0509 U00096 534934..535710
<313> 1961: 713-732
<400> 1961
GGTAATCGAA AATTCTGACT ACAACGGTTG GGTTGGGTGT GAATATAAAC 50
CCCAAACCAC CACGGAAGCC GGTTTACGCT GGATGGATCC GTACCGTTAA 100
<210> 1962
<211> 100
<212> DNA
<213> Escherichia coli K-12 MG1655
<220> gip b0509
<222> 574-673
<223> gip b0509 U00096 534934..535710
<313> 1962: 630-649
<400> 1962

ACCATGACTC AGTGGGCTGA TAAAATTGGT CACCTGCAAA TTGCCGATAA 50
TCCGCATCGC GGCGAACCGG GAACCGGAGA AATTAATTAT GATTATCTCT 100
<210> 1963
<211> 100
<212> DNA
<213> Escherichia coli K-12 MG1655
<220> gip b0509
<222> 557-656
<223> gip b0509 U00096 534934..535710
<313> 1963: 581-600
<400> 1963
AAGGTGAATT AACCAACACC ATGACTCAGT GGGCTGATAA AATTGGTCAC 50
CTGCAAATTG CCGATAATCC GCATCGCGGC GAACCGGGAA CCGGAGAAAT 100
<210> 1964
<211> 100
<212> DNA
<213> Escherichia coli K-12 MG1655
<220> yaga b0268
<222> 1056-1155
<223> yaga b0268 U00096 complement(280053..281207)
<313> 1964: 1110-1129
<400> 1964
GATGCAGGAA GACGGCAGCT ACGAGGTGTG GTGGTACAGC ACGAAAGTGG 50
GGGTGATCGA CCTGAAGAAA AAGTCGATCA CCATGGGTAA AGGATGTTAA 100
<210> 1965
<211> 100
<212> DNA
<213> Escherichia coli K-12 MG1655
<220> yaga b0268
<222> 1024-1123
<223> yaga b0268 U00096 complement(280053..281207)
<313> 1965: 1063-1082
<400> 1965
GCGTTCAGGG GAGAACGGGT CGGGCTGAAG GAGATGCAGG AAGACGGCAG 50
CTACGAGGTG TGGTGGTACA GCACGAAAGT GGGGGTGATC GACCTGAAGA 100
<210> 1966
<211> 100
<212> DNA
<213> Escherichia coli K-12 MG1655
<220> yaga b0268
<222> 972-1071
<223> yaga b0268 U00096 complement(280053..281207)
<313> 1966: 1003-1022
<400> 1966
GGATATCAGC GGAAAGCTGA GCGTGAAAGG GGTAAGTCTG AGCGCAGGCA 50
AGGCGTTCAG GGGAGAACGG GTCGGGCTGA AGGAGATGCA GGAAGACGGC 100
<210> 1967
<211> 100
<212> DNA
<213> Escherichia coli K-12 MG1655
<220> yagb b0267
<222> 279-378
<223> yagb b0267 U00096 complement(279609..279986)
<313> 1967: 354-373
<400> 1967
CGGGCTGACT GGTGAAGCCG ATACGCTGGG CAGTCATGGG TATGTATATA 50
TCGCAATTTT GAATCGCCAC GGGTTTAACA GACACCTCCG AGTCATTTAA 100
<210> 1968
<211> 100
<212> DNA
<213> Escherichia coli K-12 MG1655
<220> yagb b0267
<222> 254-353
<223> yagb b0267 U00096 complement(279609..279986)
<313> 1968: 289-308
<400> 1968
CGCACTGTGT CACACTGTAC CGTAACGGGC TGACTGGTGA AGCCGATACG 50
CTGGGCAGTC ATGGGTATGT ATATATCGCA ATTTTGAATC GCCACGGGTT 100
<210> 1969
<211> 100
<212> DNA

<213> Escherichia coli K-12 MG1655
<220> yagb b0267
<222> 197-296
<223> yagb b0267 U00096 complement(279609..279986)
<313> 1969: 237-256
<400> 1969
ATTTTGTCGC ACATCTGGAG CTGATGCTGC TCTCCTGTGA GCTGAATCCC  50
AGGTACGCGC ACTGTGTCAC ACTGTACCGT AACGGGCTGA CTGGTGAAGC  100
<210> 1970
<211> 100
<212> DNA
<213> Escherichia coli K-12 MG1655
<220> yagd b0262
<222> 834-933
<223> yagd b0262 U00096 275939..276871
<313> 1970: 888-907
<400> 1970
GGATTATCTG CCGCAGTGGC AGGCCGCTGG CGCACGGTTG ATTGGCGGGT  50
GCTGTCGCAC CACGCCTGCG GATATCGCCG CGTTAAAAGC GCGAAGCTGA  100
<210> 1971
<211> 100
<212> DNA
<213> Escherichia coli K-12 MG1655
<220> yagd b0262
<222> 726-825
<223> yagd b0262 U00096 275939..276871
<313> 1971: 764-783
<400> 1971
ACACGGTTTA ACGGTGCTGC CGCTGGTGGT GTATCCGAAC TCGGGCGAGC  50
ATTACGATGC CGTGAGCAAA ACCTGGCATC ATCACGGTGA ACATTGCGCG  100
<210> 1972
<211> 100
<212> DNA
<213> Escherichia coli K-12 MG1655
<220> yagd b0262
<222> 648-747
<223> yagd b0262 U00096 275939..276871
<313> 1972: 686-705
<400> 1972
GGCGGGTTAT CCGCAGGTGG TGGCGCTAGG CATTAACTGT ATTGCGCTGG  50
AAAACACCAC CGCTGCGTTG CAGCATTTAC ACGGTTTAAC GGTGCTGCCG  100
<210> 1973
<211> 100
<212> DNA
<213> Escherichia coli K-12 MG1655
<220> yage b0269
<222> 821-920
<223> yage b0269 U00096 281481..282410
<313> 1973: 872-891
<400> 1973
TGCTCTGCGG TCGTCCTGTC TCCACGCACG TGCTGCCGCC CGCCTCGCCG  50
CTGGACGAGC CGCGCAAGGC GCAGCTGAAA ACCCTGCTGC AACAGCTCAA  100
<210> 1974
<211> 100
<212> DNA
<213> Escherichia coli K-12 MG1655
<220> yage b0269
<222> 716-815
<223> yage b0269 U00096 281481..282410
<313> 1974: 750-769
<400> 1974
ACGGGGACGT GGCGAAAGCG GCCGGGTATC ATCAGACCTT GCTGCAAATT  50
CCGCAGATGT ATCAGCTGGA TACGCCGTTT GTGAACGTGA TTAAAGAGGC  100
<210> 1975
<211> 100
<212> DNA
<213> Escherichia coli K-12 MG1655
<220> yage b0269
<222> 670-769
<223> yage b0269 U00096 281481..282410
<313> 1975: 702-721

<400> 1975
AACTTTGCCC CGCAGGTGTC GGTGAATCTT CTGAAAGCCT GGCGCGACGG 50
GGACGTGGCG AAAGCGGCCG GGTATCATCA GACCTTGCTG CAAATTCCGC 100
<210> 1976
<211> 100
<212> DNA
<213> Escherichia coli K-12 MG1655
<220> yagf b0270
<222> 1842-1941
<223> yagf b0270 U00096 282425..284392
<313> 1976: 1898-1917
<400> 1976
GCCGGACGAC ACCCGGCTGT GGGCGGCACT GCAGTCGGTG AGCGGCGGCA 50
CCTGGAAAGG CTGTATTTAT GACACCGATA AAATTATCGA GGTAATTAAC 100
<210> 1977
<211> 100
<212> DNA
<213> Escherichia coli K-12 MG1655
<220> yagf b0270
<222> 1803-1902
<223> yagf b0270 U00096 282425..284392
<313> 1977: 1834-1853
<400> 1977
GCGGCAGACG CACCCGGACC TGCACGCCCA CGACTTTTTG CCGGACGACA 50
CCCGGCTGTG GGCGGCACTG CAGTCGGTGA GCGGCGGCAC CTGGAAAGGC 100
<210> 1978
<211> 100
<212> DNA
<213> Escherichia coli K-12 MG1655
<220> yagf b0270
<222> 1637-1736
<223> yagf b0270 U00096 282425..284392
<313> 1978: 1690-1709
<400> 1978
GCCACGTGTC GCCGGAGGCG CTGGCGGGCG GGCCGATTGG CAAGCTGCGC 50
GATAACGACA TCATCGAGAT TGCCGTGGAT CGTCTGACGT TAACTGGCAG 100
<210> 1979
<211> 100
<212> DNA
<213> Escherichia coli K-12 MG1655
<220> nrfa b4071
<222> 1338-1437
<223> nrfa b4071 U00096 4285343..4286779
<313> 1979: 1367-1386
<400> 1979
GAACATGGAA CAAATCAAGG CCGAGAAGCA GGACTTCATC AAAACGGTGA 50
TCCCGCAGTG GGAAGAACAG GCACGTAAAA ACGGTCTGTT AAGCCAATAA 100
<210> 1980
<211> 100
<212> DNA
<213> Escherichia coli K-12 MG1655
<220> nrfa b4071
<222> 1226-1325
<223> nrfa b4071 U00096 4285343..4286779
<313> 1980: 1281-1300
<400> 1980
CGGCGGATGC ACGCACCAAA CTGGCGCGCC TGCTGGCGAC CAAAGGCATC 50
ACCCATGAAA TCCAGATCCC GGATATCTCA ACCAAAGAGA AAGCCCAGCA 100
<210> 1981
<211> 100
<212> DNA
<213> Escherichia coli K-12 MG1655
<220> nrfa b4071
<222> 1188-1287
<223> nrfa b4071 U00096 4285343..4286779
<313> 1981: 1230-1249
<400> 1981
AGAAGGTTTA CGGATGCTCG GTACGGCGAT GGATAAAGCG GCGGATGCAC 50
GCACCAAACT GGCGCGCCTG CTGGCGACCA AAGGCATCAC CCATGAAATC 100
<210> 1982
<211> 100

<212> DNA
<213> Escherichia coli K-12 MG1655
<220> yagg b0271
<222> 1284-1383
<223> yagg b0271 U00096 284619..286001
<313> 1982: 1361-1380
<400> 1982
CACCGATGCC CGCGTGGAGG CCATCAGCCG GCAGCTGATT AAGCACCGCG 50
CGGCGCAGGG CGAGGCCGTT CCCGACGCCG CGACAGCCGC ATCCCATTAA 100
<210> 1983
<211> 100
<212> DNA
<213> Escherichia coli K-12 MG1655
<220> yagg b0271
<222> 1222-1321
<223> yagg b0271 U00096 284619..286001
<313> 1983: 1269-1288
<400> 1983
TTCTGCGTGG TGCCGGTGGT GCTCTACGCG GGCATGTTCA TCATGCTGTC 50
GCTCTACAAG CTCACCGATG CCCGCGTGGA GGCCATCAGC CGGCAGCTGA 100
<210> 1984
<211> 100
<212> DNA
<213> Escherichia coli K-12 MG1655
<220> yagg b0271
<222> 1164-1263
<223> yagg b0271 U00096 284619..286001
<313> 1984: 1197-1216
<400> 1984
CTATTCCGCC AGCAGCAGCG TGCAGCCGGT TGAGGTGCTC ACCACCATCA 50
AAATTCTGTT CTGCGTGGTG CCGGTGGTGC TCTACGCGGG CATGTTCATC 100
<210> 1985
<211> 100
<212> DNA
<213> Escherichia coli K-12 MG1655
<220> nrfb b4072
<222> 474-573
<223> nrfb b4072 U00096 4286818..4287390
<313> 1985: 525-544
<400> 1985
CAAAGGACGG ATTAAGATTT GCGTCGATTG CCACAGCGAT CAGCGCACCA 50
ATCCGAACTT TAACCCGGCG TCTGTTCCGT TGCTTAAGGA GCAGCCATGA 100
<210> 1986
<211> 100
<212> DNA
<213> Escherichia coli K-12 MG1655
<220> nrfb b4072
<222> 449-548
<223> nrfb b4072 U00096 4286818..4287390
<313> 1986: 503-522
<400> 1986
AAGATACGAT GCAGACGTTA AGTGACAAAG GACGGATTAA GATTTGCGTC 50
GATTGCCACA GCGATCAGCG CACCAATCCG AACTTTAACC CGGCGTCTGT 100
<210> 1987
<211> 100
<212> DNA
<213> Escherichia coli K-12 MG1655
<220> nrfb b4072
<222> 423-522
<223> nrfb b4072 U00096 4286818..4287390
<313> 1987: 458-477
<400> 1987
CAGCTGCCAT TCCCTGCATC CACAGCAAGA TACGATGCAG ACGTTAAGTG 50
ACAAAGGACG GATTAAGATT TGCGTCGATT GCCACAGCGA TCAGCGCACC 100
<210> 1988
<211> 100
<212> DNA
<213> Escherichia coli K-12 MG1655
<220> yagh b0272
<222> 1512-1611
<223> yagh b0272 U00096 286013..287623

<313> 1988: 1576-1595
<400> 1988
CGGCTTCTTC ACCGGCGCGT TTGTGGGCCT GCACTGCGAG GACATCAGCG 50
GCGACGGCTG CTACGCGGAC TTCGACTACT TCACCTACGA GCCGGTCTAA 100
<210> 1989
<211> 100
<212> DNA
<213> Escherichia coli K-12 MG1655
<220> yagh b0272
<222> 1447-1546
<223> yagh b0272 U00096 286013..287623
<313> 1989: 1468-1487
<400> 1989
ACGTGGCACA CCGTGCCGGT GACGTATGAG GCGTGGAAGC TGTCGGACGA 50
CTACATCGGC GGGCGCGGCT TCTTCACCGG CGCGTTTGTG GGCCTGCACT 100
<210> 1990
<211> 100
<212> DNA
<213> Escherichia coli K-12 MG1655
<220> yagh b0272
<222> 1410-1509
<223> yagh b0272 U00096 286013..287623
<313> 1990: 1436-1455
<400> 1990
GCTGGTCTAC CGCTACAGCT ACTCGTTTGA TGGCGAGACG TGGCACACCG 50
TGCCGGTGAC GTATGAGGCG TGGAAGCTGT CGGACGACTA CATCGGCGGG 100
<210> 1991
<211> 100
<212> DNA
<213> Escherichia coli K-12 MG1655
<220> nrfc b4073
<222> 573-672
<223> nrfc b4073 U00096 4287387..4288058
<313> 1991: 639-658
<400> 1991
ACTGCTGCGC CAGAAGCCCA CTTACCGCTA CAAGCTGGCG CTGGGAACCA 50
AACCGAAGCT GTACCGCGTA CCGTTTAAAT ACGGGGAGGT GAGCCAATGA 100
<210> 1992
<211> 100
<212> DNA
<213> Escherichia coli K-12 MG1655
<220> nrfc b4073
<222> 534-633
<223> nrfc b4073 U00096 4287387..4288058
<313> 1992: 584-603
<400> 1992
GTTTGGCAAT CTGGACGATC CCAACAGTGA GATTTCGCAA CTGCTGCGCC 50
AGAAGCCCAC TTACCGCTAC AAGCTGGCGC TGGGAACCAA ACCGAAGCTG 100
<210> 1993
<211> 100
<212> DNA
<213> Escherichia coli K-12 MG1655
<220> nrfc b4073
<222> 502-601
<223> nrfc b4073 U00096 4287387..4288058
<313> 1993: 531-550
<400> 1993
TGCGTTGAAG CTTGCCCGAC CAAGGCGCTG ACGTTTGGCA ATCTGGACGA 50
TCCCAACAGT GAGATTTCGC AACTGCTGCG CCAGAAGCCC ACTTACCGCT 100
<210> 1994
<211> 100
<212> DNA
<213> Escherichia coli K-12 MG1655
<220> yagi b0273
<222> 571-670
<223> yagi b0273 U00096 complement(287628..288386)
<313> 1994: 635-654
<400> 1994
AGCGAAGAGA ACGAGCAGGG CGTGCGCTGC GTGGCGGTGC CGGTGTGGAA 50
CCACGAGTCC CGCGTCATCG CCGCCCTGAG CCTGTCGACG CTGACCTCCC 100
<210> 1995

<211> 100
<212> DNA
<213> Escherichia coli K-12 MG1655
<220> yagi b0273
<222> 536-635
<223> yagi b0273 U00096 complement(287628..288386)
<313> 1995: 566-585
<400> 1995
TGGCGCAGAC CCGCGAGCAA GGCTACGCCC TGGACAGCGA AGAGAACGAG 50
CAGGGCGTGC GCTGCGTGGC GGTGCCGGTG TGGAACCACG AGTCCCGCGT 100
<210> 1996
<211> 100
<212> DNA
<213> Escherichia coli K-12 MG1655
<220> yagi b0273
<222> 437-536
<223> yagi b0273 U00096 complement(287628..288386)
<313> 1996: 485-504
<400> 1996
GGCTGGGCGA GGCCGAGCTG AACGCCCTGC TGGAGGGCTA TCAGTACACT 50
ACCTTTACGC CCGCCACCCT CGCGTCTCGC GAAGCCTTAA TGAGCGCCCT 100
<210> 1997
<211> 100
<212> DNA
<213> Escherichia coli K-12 MG1655
<220> nrfd b4074
<222> 858-957
<223> nrfd b4074 U00096 4288055..4289011
<313> 1997: 924-943
<400> 1997
CGGCATTCCT GCCGTGCTGG CGGCGTGTGG GGCCAGTCTG GTCGGCGTGT 50
TGATGCTGCG CTTTTTCATT CTCTACGCCG GACAGTTGAC GGTGGCGTAA 100
<210> 1998
<211> 100
<212> DNA
<213> Escherichia coli K-12 MG1655
<220> nrfd b4074
<222> 816-915
<223> nrfd b4074 U00096 4288055..4289011
<313> 1998: 840-859
<400> 1998
GATTGTGCCA ATGCTGCTCA AACCGTGGGT CAATCGCAGT TCCGGCATTC 50
CTGCCGTGCT GGCGGCGTGT GGGGCCAGTC TGGTCGGCGT GTTGATGCTG 100
<210> 1999
<211> 100
<212> DNA
<213> Escherichia coli K-12 MG1655
<220> nrfd b4074
<222> 729-828
<223> nrfd b4074 U00096 4288055..4289011
<313> 1999: 768-787
<400> 1999
TGACGGTAAA GTTCGTGCGC TGGTGGCGGC ATTAGGTGGC GGTTTCTGGA 50
CGTGGTGGTT CTGGCTTGGT GTCGCCGGGC TGGGGCTGAT TGTGCCAATG 100
<210> 2000
<211> 100
<212> DNA
<213> Escherichia coli K-12 MG1655
<220> yagj b0277
<222> 633-732
<223> yagj b0277 U00096 290724..291455
<313> 2000: 672-691
<400> 2000
AGTGTTAGAT TTGAAACGGC AGGATGTGCA GGATGCTGTT GCTACCGCTG 50
AAAATGCAGA AACGTTATTT AGTAGCGGAC TTGCTGATGA CTATCAGTAA 100
<210> 2001
<211> 100
<212> DNA
<213> Escherichia coli K-12 MG1655
<220> yagj b0277
<222> 583-682

<223> yagj b0277 U00096 290724..291455
<313> 2001: 600-619
<400> 2001
GATGCTTACA GGCGAATCGA ATCTGTCGCC GAAGTAAAGG GTAAATTACG 50
AGTGTTAGAT TTGAAACGGC AGGATGTGCA GGATGCTGTT GCTACCGCTG 100
<210> 2002
<211> 100
<212> DNA
<213> Escherichia coli K-12 MG1655
<220> yagj b0277
<222> 519-618
<223> yagj b0277 U00096 290724..291455
<313> 2002: 572-591
<400> 2002
CCTACATCTG GCTGATGCTT TGCCCAAACT GGAAGGACTT GCGCTATATG 50
CCGAACATCA TTCTGATGCT TACAGGCGAA TCGAATCTGT CGCCGAAGTA 100
<210> 2003
<211> 100
<212> DNA
<213> Escherichia coli K-12 MG1655
<220> nrfe b4075
<222> 1560-1659
<223> nrfe b4075 U00096 4289091..4290749
<313> 2003: 1620-1639
<400> 2003
GTATGTACAA AGCGGTGTGC GCTGGATCTG GGGGGGAGGA TTGTTGATGA 50
TTGCGGGCGC ATTGTTAAGC GGATGGCGGG GGAAGAAGCG CGATGAATAA 100
<210> 2004
<211> 100
<212> DNA
<213> Escherichia coli K-12 MG1655
<220> nrfe b4075
<222> 1511-1610
<223> nrfe b4075 U00096 4289091..4290749
<313> 2004: 1538-1557
<400> 2004
ATGCGGTCAT GGGGGAGAAA ACTGGGCCGG ATCGTTACGC TTTTCGTTTG 50
TATGTACAAA GCGGTGTGCG CTGGATCTGG GGGGGAGGAT TGTTGATGAT 100
<210> 2005
<211> 100
<212> DNA
<213> Escherichia coli K-12 MG1655
<220> nrfe b4075
<222> 1429-1528
<223> nrfe b4075 U00096 4289091..4290749
<313> 2005: 1482-1501
<400> 2005
ACGCCGGAGC GGCGTTTTTA TGAAGCACGC CGTCAACAAA TGATGGAACC 50
GTCAATTCGC TGGAACGGCA TCCATGACTG GTATGCGGTC ATGGGGGAGA 100
<210> 2006
<211> 100
<212> DNA
<213> Escherichia coli K-12 MG1655
<220> yagk b0278
<222> 528-627
<223> yagk b0278 U00096 complement(291546..292172)
<313> 2006: 591-610
<400> 2006
CCAGGCGCTG TTAACCCGAC TGGACTACCT GACCAAAGTT GAAAGCAAAG 50
TGTTTGGTGA AGGAGACCGT AACTTCGGCT GTAGCCAAAT CGAACTGTAA 100
<210> 2007
<211> 100
<212> DNA
<213> Escherichia coli K-12 MG1655
<220> yagk b0278
<222> 501-600
<223> yagk b0278 U00096 complement(291546..292172)
<313> 2007: 548-567
<400> 2007
CAATAGTACT GACTTCCAGC AGAACTACCA GGCGCTGTTA ACCCGACTGG 50
ACTACCTGAC CAAAGTTGAA AGCAAAGTGT TTGGTGAAGG AGACCGTAAC 100

<210> 2008
<211> 100
<212> DNA
<213> Escherichia coli K-12 MG1655
<220> yagk b0278
<222> 416-515
<223> yagk b0278 U00096 complement(291546..292172)
<313> 2008: 451-470
<400> 2008
CCTGGTACAG CGCCCTGAGA CTGGAGAGAG ACGATCACCC TGGTCTGGTC 50
CATTTTCCTG AAAACTGTAA GTATGTGCTA GACACCAATA GTACTGACTT 100
<210> 2009
<211> 100
<212> DNA
<213> Escherichia coli K-12 MG1655
<220> nrff b4076
<222> 285-384
<223> nrff b4076 U00096 4290742..4291125
<313> 2009: 357-376
<400> 2009
CCCACCGTTA ACGGGTCAGA CGCTGGTGTT ATGGGCGCTG CCAGTGGTGT 50
TGTTACTGCT GATGGCACTG ATCCTCTGGC GAGTGAGGGC GAAGCGATGA 100
<210> 2010
<211> 100
<212> DNA
<213> Escherichia coli K-12 MG1655
<220> nrff b4076
<222> 209-308
<223> nrff b4076 U00096 4290742..4291125
<313> 2010: 264-283
<400> 2010
TGGTGGCGGA GGGGAAGAAC GAAGTCGAAA TCATTGGCTG GATGACCGAA 50
CGCTACGGAG ATTTTGTTCG CTATAACCCA CCGTTAACGG GTCAGACGCT 100
<210> 2011
<211> 100
<212> DNA
<213> Escherichia coli K-12 MG1655
<220> nrff b4076
<222> 200-299
<223> nrff b4076 U00096 4290742..4291125
<313> 2011: 217-236
<400> 2011
TTTACAGCAT GGTGGCGGAG GGGAAGAACG AAGTCGAAAT CATTGGCTGG 50
ATGACCGAAC GCTACGGAGA TTTTGTTCGC TATAACCCAC CGTTAACGGG 100
<210> 2012
<211> 100
<212> DNA
<213> Escherichia coli K-12 MG1655
<220> b1450 b1450
<222> 586-685
<223> b1450 b1450 U00096 1518229..1518951
<313> 2012: 621-640
<400> 2012
GAATTACGGG AGCATATAGA AAACTATCAT CTGTTACTCG CTGCGTTAAA 50
AGCAAAAGAC AAAGAGGGAT GCAGACATTG TCTTGCTGAA ATTATGCAAC 100
<210> 2013
<211> 100
<212> DNA
<213> Escherichia coli K-12 MG1655
<220> b1450 b1450
<222> 495-594
<223> b1450 b1450 U00096 1518229..1518951
<313> 2013: 550-569
<400> 2013
AAATATGCCC ATCCTGTGTG AGATGATTGA GCAACTGTGG GTCAGGATGG 50
GGCCTGGTTT ACATTATCTC TATGAAGCGA TTAATCCAGC GGAATTACGG 100
<210> 2014
<211> 100
<212> DNA
<213> Escherichia coli K-12 MG1655
<220> b1450 b1450

<222> 432-531
<223> b1450 b1450 U00096 1518229..1518951
<313> 2014: 485-504
<400> 2014
GGGCGACATG GAACAAATCA TTAATGTAAA CAGGCTATTT CGCTTAGCGA 50
TTTATCATCG CTCAAATATG CCCATCCTGT GTGAGATGAT TGAGCAACTG 100
<210> 2015
<211> 100
<212> DNA
<213> Escherichia coli K-12 MG1655
<220> yagl b0279
<222> 600-699
<223> yagl b0279 U00096 complement(292444..293142)
<313> 2015: 665-684
<400> 2015
ACTTAAGAAA GGTGGATTTA CTCAGGCTGA GATTGCAAAG AAATTGAGTA 50
TCAGTTTATC GACTGTGAAG CGGCACTGGA ATAACGGAAT CATCGGTTAA 100
<210> 2016
<211> 100
<212> DNA
<213> Escherichia coli K-12 MG1655
<220> yagl b0279
<222> 429-528
<223> yagl b0279 U00096 complement(292444..293142)
<313> 2016: 455-474
<400> 2016
CCATCCAGCT TCAAGGATAG AGCCTTCTGC TGAAAGTTGC TTGTCTTTTT 50
TGATTGCTCG TCAGGATACG ATTGACATTC ACAATCTGAA ATCAACTAAA 100
<210> 2017
<211> 100
<212> DNA
<213> Escherichia coli K-12 MG1655
<220> yagl b0279
<222> 328-427
<223> yagl b0279 U00096 complement(292444..293142)
<313> 2017: 357-376
<400> 2017
ACGCTTGTCA TTCCAGATCT AAGCTGTCTT GGCAGAACTG TGGAGGACTT 50
ACAGAATATT TTATTTTTTT GCCTTCAAAA AGAAATGTTT ATCTACAGCT 100
<210> 2018
<211> 100
<212> DNA
<213> Escherichia coli K-12 MG1655
<220> nrfg b4077
<222> 470-569
<223> nrfg b4077 U00096 4291122..4291718
<313> 2018: 519-538
<400> 2018
AGGCGAACTA CGCGCAAGCC ATCGAACTAT GGCAAAAAGT GATGGATCTC 50
AACTCACCGC GAGTTAACCG AACACAGCTG GTTGAGTCGA TTAATATGGC 100
<210> 2019
<211> 100
<212> DNA
<213> Escherichia coli K-12 MG1655
<220> nrfg b4077
<222> 458-557
<223> nrfg b4077 U00096 4291122..4291718
<313> 2019: 485-504
<400> 2019
ATGCGTTTAT GCAGGCGAAC TACGCGCAAG CCATCGAACT ATGGCAAAAA 50
GTGATGGATC TCAACTCACC GCGAGTTAAC CGAACACAGC TGGTTGAGTC 100
<210> 2020
<211> 100
<212> DNA
<213> Escherichia coli K-12 MG1655
<220> nrfg b4077
<222> 384-483
<223> nrfg b4077 U00096 4291122..4291718
<313> 2020: 436-455
<400> 2020
GACTCGCGCA ATGATCGACA AAGCCCTCGC GCTGGACAGT AATGAAATCA 50

CCGCCCTGAT GCTGCTGGCT TCCGATGCGT TTATGCAGGC GAACTACGCG 100
<210> 2021
<211> 100
<212> DNA
<213> Escherichia coli K-12 MG1655
<220> b1451 b1451
<222> 1972-2071
<223> b1451 b1451 U00096 complement(1518987..1521089)
<313> 2021: 2004-2023
<400> 2021
CGTGTCGATA ATTTATTCGA TAAAGAATAC GTTGGTTCTG TCATTGTCAA 50
TGAGTCAAAC GGGCGATATT ACGAACCTTC GCCCGGACGA AATTATGGTG 100
<210> 2022
<211> 100
<212> DNA
<213> Escherichia coli K-12 MG1655
<220> b1451 b1451
<222> 1840-1939
<223> b1451 b1451 U00096 complement(1518987..1521089)
<313> 2022: 1900-1919
<400> 2022
ACGGAAGCGC GTTATATGGG CGATATTATG GCAGATGATG AAAATACGGC 50
AAAAGCGCCG TCTTATACTC TCGTCGGCTT ATTCACCGGG TATAAATACA 100
<210> 2023
<211> 100
<212> DNA
<213> Escherichia coli K-12 MG1655
<220> b1451 b1451
<222> 1767-1866
<223> b1451 b1451 U00096 complement(1518987..1521089)
<313> 2023: 1825-1844
<400> 2023
GATGCCAGGG ATCGCCCGTA ATATGGGCTT TGCGTCGATA GGTTATGTAC 50
CGGAAGATGG TTGGTATGCA GGCACGGAAG CGCGTTATAT GGGCGATATT 100
<210> 2024
<211> 100
<212> DNA
<213> Escherichia coli K-12 MG1655
<220> yagm b0280
<222> 756-855
<223> yagm b0280 U00096 complement(293169..294023)
<313> 2024: 784-803
<400> 2024
GGTTATGCTG CCAGTGCTCC GCAAAACAGA TGTTTCCAAC CGTGTTAATG 50
TACCGGATGA CATCATTATA AACCCATACT CACAAGAGAG CAGAACCTAA 100
<210> 2025
<211> 100
<212> DNA
<213> Escherichia coli K-12 MG1655
<220> yagm b0280
<222> 696-795
<223> yagm b0280 U00096 complement(293169..294023)
<313> 2025: 754-773
<400> 2025
GATTGTTATG CCTTATCCGG TTGTTTGTTG GATTGATATT TACCATGTCA 50
ATGAAATGGT GGTTATGCTG CCAGTGCTCC GCAAAACAGA TGTTTCCAAC 100
<210> 2026
<211> 100
<212> DNA
<213> Escherichia coli K-12 MG1655
<220> yagm b0280
<222> 660-759
<223> yagm b0280 U00096 complement(293169..294023)
<313> 2026: 701-720
<400> 2026
TATATTCGGG ATAAATGAAG TTCTTAGAAG AAAGGGGATT GTTATGCCTT 50
ATCCGGTTGT TTGTTGGATT GATATTTACC ATGTCAATGA AATGGTGGTT 100
<210> 2027
<211> 100
<212> DNA
<213> Escherichia coli K-12 MG1655

<220> b1452 b1453
<222> 963-1062
<223> b1452 b1453 U00096 1521331..1522392
<313> 2027: 1025-1044
<400> 2027
GTCTGCCGAT GGCAAAACGC TGTATGTCAG TGTGAAACAA AAATCCACTA 50
AACAGCAGGA AGCTACCCAG CCAGACGATG TGATTCGTAT TGCGCTGTAA 100
<210> 2028
<211> 100
<212> DNA
<213> Escherichia coli K-12 MG1655
<220> b1452 b1453
<222> 957-1056
<223> b1452 b1453 U00096 1521331..1522392
<313> 2028: 972-991
<400> 2028
GGCGCTGTCT GCCGATGGCA AAACGCTGTA TGTCAGTGTG AAACAAAAAT 50
CCACTAAACA GCAGGAAGCT ACCCAGCCAG ACGATGTGAT TCGTATTGCG 100
<210> 2029
<211> 100
<212> DNA
<213> Escherichia coli K-12 MG1655
<220> b1452 b1453
<222> 900-999
<223> b1452 b1453 U00096 1521331..1522392
<313> 2029: 921-940
<400> 2029
CGCGAAAAGC TATAAAGTGG TGAAAACGTT CGATACGCCG ACTCATCCAA 50
ACAGCCTGGC GCTGTCTGCC GATGGCAAAA CGCTGTATGT CAGTGTGAAA 100
<210> 2030
<211> 100
<212> DNA
<213> Escherichia coli K-12 MG1655
<220> yagn b0281
<222> 299-398
<223> yagn b0281 U00096 complement(294363..294803)
<313> 2030: 331-350
<400> 2030
CCTTATCATC AGATTCAGGT GATGGTAGTG ACCCCGACCC CGAGCCCGAA 50
ACGTTTCCTT CTGCCTTCAT TACTACCCCT ACTAATTCAA TAATGCTTAA 100
<210> 2031
<211> 100
<212> DNA
<213> Escherichia coli K-12 MG1655
<220> yagn b0281
<222> 228-327
<223> yagn b0281 U00096 complement(294363..294803)
<313> 2031: 278-297
<400> 2031
CGCGCGTCTG ATGTCATGTA AAAATCAGGC ATCCAGATCG CATCACAGTA 50
CGGCTAAATC AGCTAAAAGT GCCTTATCAT CAGATTCAGG TGATGGTAGT 100
<210> 2032
<211> 100
<212> DNA
<213> Escherichia coli K-12 MG1655
<220> yagn b0281
<222> 105-204
<223> yagn b0281 U00096 complement(294363..294803)
<313> 2032: 138-157
<400> 2032
GTCCATGGAA GAGGTTGTCG AGTATCTCAA AGGAACATAC TCAGCTCTTC 50
GCAAGTCTGT CGCATGCGCC AAACTGAAAA TTTTACATCT TAAACAAAGA 100
<210> 2033
<211> 100
<212> DNA
<213> Escherichia coli K-12 MG1655
<220> b0725 b0726
<222> 162-261
<223> b0725 b0726 U00096 757687..757947
<313> 2033: 219-238
<400> 2033

AGTGAACCCC GGCACGCACA TCACTGTGCG TGGTAGTATC CACGGCGAAG 50
TAAGCATAAA AAAGATGCTT AAGGGATCAC GATGCAGAAC AGCGCTTTGA 100
<210> 2034
<211> 100
<212> DNA
<213> Escherichia coli K-12 MG1655
<220> b0725 b0726
<222> 146-245
<223> b0725 b0726 U00096 757687..757947
<313> 2034: 195-214
<400> 2034
TAGACAAGAG CTCGCAAGTG AACCCCGGCA CGCACATCAC TGTGCGTGGT 50
AGTATCCACG GCGAAGTAAG CATAAAAAAG ATGCTTAAGG GATCACGATG 100
<210> 2035
<211> 100
<212> DNA
<213> Escherichia coli K-12 MG1655
<220> b0725 b0726
<222> 65-164
<223> b0725 b0726 U00096 757687..757947
<313> 2035: 95-114
<400> 2035
TAACAAAGAA ATGCAGGAAA TCTTTAAAAA CTGCCCCTGA CACTAAGACA 50
GTTTTTAAAG GTTCCTTCGC GAGCCACTAC GTAGACAAGA GCTCGCAAGT 100
<210> 2036
<211> 100
<212> DNA
<213> Escherichia coli K-12 MG1655
<220> b1454 b1454
<222> 477-576
<223> b1454 b1454 U00096 1524271..1524888
<313> 2036: 516-535
<400> 2036
CTGTTATCTT TGCACTATGC GCACATGGGG GCCTGGGCAT GAATGGTTTC 50
AGGATAATGC CACGAATATC AGTGCGATTG CTGATGCTGT ATGTCAGTTA 100
<210> 2037
<211> 100
<212> DNA
<213> Escherichia coli K-12 MG1655
<220> b1454 b1454
<222> 409-508
<223> b1454 b1454 U00096 1524271..1524888
<313> 2037: 439-458
<400> 2037
TATATCTGGT TGAATTCGCA GCTCACTGCT GAACCTTATG CGTTTGGTGA 50
GCAACTAACG CTGGTGGACT GTTATCTTTG CACTATGCGC ACATGGGGGC 100
<210> 2038
<211> 100
<212> DNA
<213> Escherichia coli K-12 MG1655
<220> b1454 b1454
<222> 325-424
<223> b1454 b1454 U00096 1524271..1524888
<313> 2038: 362-381
<400> 2038
ACTTTCGCCG ATTACCCTGA GCGTTGGGCT CCTGACGCAC CTGAACAGTT 50
AAAGAAGAAT GTTATTGAAT ATCGAAAATC GCTTTATATC TGGTTGAATT 100
<210> 2039
<211> 100
<212> DNA
<213> Escherichia coli K-12 MG1655
<220> yagp b0283
<222> 312-411
<223> yagp b0283 U00096 complement(296605..297015)
<313> 2039: 373-392
<400> 2039
TCCGGGCTTC TATCTGTATT TCCCACAGCG TCGCAATATT GCACCAAAGC 50
TCCGCGCCCT GATTGACTAC GTCAAAGAAT GGCGGCAGCA ATTGGCTTAA 100
<210> 2040
<211> 100
<212> DNA

<213> Escherichia coli K-12 MG1655
<220> yagp b0283
<222> 285-384
<223> yagp b0283 U00096 complement(296605..297015)
<313> 2040: 325-344
<400> 2040
GCTGCTTGAT GACTTTCTTC CACAATTTCC GGGCTTCTAT CTGTATTTCC 50
CACAGCGTCG CAATATTGCA CCAAAGCTCC GCGCCCTGAT TGACTACGTC 100
<210> 2041
<211> 100
<212> DNA
<213> Escherichia coli K-12 MG1655
<220> yagp b0283
<222> 173-272
<223> yagp b0283 U00096 complement(296605..297015)
<313> 2041: 238-257
<400> 2041
TTACGACTAA TGATCTGCGT CTGATGTTGA GGCTGGCTCT TGCCGGCGGA 50
GGAATAACAA TTGCCACTCA GGAAACTTTC AGGCCATATA TTGAAAGCGG 100
<210> 2042
<211> 100
<212> DNA
<213> Escherichia coli K-12 MG1655
<220> b1455 b1456
<222> 91-190
<223> b1455 b1456 U00096 1524964..1525176
<313> 2042: 155-174
<400> 2042
CAGATGTTTC AGTTGTTATC ACAGGCATTC TATAATCTCA AAATGTTTTT 50
AATGATGGAT ATGCTCGGAG TTGGAGATGC AATTAATATT AATACAAATA 100
<210> 2043
<211> 100
<212> DNA
<213> Escherichia coli K-12 MG1655
<220> b1455 b1456
<222> 74-173
<223> b1455 b1456 U00096 1524964..1525176
<313> 2043: 107-126
<400> 2043
TAACTATTCA CATCGTGCAG ATGTTTCAGT TGTTATCACA GGCATTCTAT 50
AATCTCAAAA TGTTTTTAAT GATGGATATG CTCGGAGTTG GAGATGCAAT 100
<210> 2044
<211> 100
<212> DNA
<213> Escherichia coli K-12 MG1655
<220> b1455 b1456
<222> 53-152
<223> b1455 b1456 U00096 1524964..1525176
<313> 2044: 97-116
<400> 2044
TCATTTCCTC TGAAAAGAAA CTAACTATTC ACATCGTGCA GATGTTTCAG 50
TTGTTATCAC AGGCATTCTA TAATCTCAAA ATGTTTTTAA TGATGGATAT 100
<210> 2045
<211> 100
<212> DNA
<213> Escherichia coli K-12 MG1655
<220> yagq b0284
<222> 858-957
<223> yagq b0284 U00096 complement(296994..297950)
<313> 2045: 928-947
<400> 2045
AGCCCGGGAT GCGCATACTC TGGCACTCTC CGTGCTGGCA GAAGTCGCCT 50
CTGTACGTCT CCATCAGGAG GAGGATTCAT GCCTGCCCCC GTCGTCCTGA 100
<210> 2046
<211> 100
<212> DNA
<213> Escherichia coli K-12 MG1655
<220> yagq b0284
<222> 787-886
<223> yagq b0284 U00096 complement(296994..297950)
<313> 2046: 824-843

<400> 2046
AAGCTCCACG AGCTGGGATG GTCCAGGGAG GAAACAACGC AAATCCGGGC  50
ACCCGTCGGG ATATTCCCCA AAGCCCGGGA TGCGCATACT CTGGCACTCT  100
<210> 2047
<211> 100
<212> DNA
<213> Escherichia coli K-12 MG1655
<220> yagq b0284
<222> 754-853
<223> yagq b0284 U00096 complement(296994..297950)
<313> 2047: 777-796
<400> 2047
GGCAGCTATC GAACCCACAC GTTACGTCTG CAAAAGCTCC ACGAGCTGGG  50
ATGGTCCAGG GAGGAAACAA CGCAAATCCG GGCACCCGTC GGGATATTCC  100
<210> 2048
<211> 100
<212> DNA
<213> Escherichia coli K-12 MG1655
<220> yagr b0285
<222> 2100-2199
<223> yagr b0285 U00096 complement(297960..300158)
<313> 2048: 2167-2186
<400> 2048
GGCTATCGCC AACGCGGTGT ATAACGCCAC CGGTATTCGG GTACGGGATT  50
ATCCCATCAC TCTGGATAAG CTGCTCGATA AGCTGCCGGA TGTGGTTTAA  100
<210> 2049
<211> 100
<212> DNA
<213> Escherichia coli K-12 MG1655
<220> yagr b0285
<222> 2074-2173
<223> yagr b0285 U00096 complement(297960..300158)
<313> 2049: 2091-2110
<400> 2049
GAGCTGGGCC TGTGCGGCGT GAGCGCGGCT ATCGCCAACG CGGTGTATAA  50
CGCCACCGGT ATTCGGGTAC GGGATTATCC CATCACTCTG GATAAGCTGC  100
<210> 2050
<211> 100
<212> DNA
<213> Escherichia coli K-12 MG1655
<220> yagr b0285
<222> 2014-2113
<223> yagr b0285 U00096 complement(297960..300158)
<313> 2050: 2044-2063
<400> 2050
GTGATTTTCC TGGATGATAC CGACCCCATA TCCTCCCCGA TGAAGGCCAA  50
AGGTGTCGGT GAGCTGGGCC TGTGCGGCGT GAGCGCGGCT ATCGCCAACG  100
<210> 2051
<211> 100
<212> DNA
<213> Escherichia coli K-12 MG1655
<220> yags b0286
<222> 858-957
<223> yags b0286 U00096 complement(300155..301111)
<313> 2051: 923-942
<400> 2051
GTTCGCCAGC GCCCATCCCA CCGCTGAAAA CACCTTTAAA CTCCTGTTGG  50
CGAAGCGAAC GCTTGCCTCC GTACTGGCTG AAGCGAGGGC ACAGGCATGA  100
<210> 2052
<211> 100
<212> DNA
<213> Escherichia coli K-12 MG1655
<220> yags b0286
<222> 858-957
<223> yags b0286 U00096 complement(300155..301111)
<313> 2052: 887-906
<400> 2052
GTTCGCCAGC GCCCATCCCA CCGCTGAAAA CACCTTTAAA CTCCTGTTGG  50
CGAAGCGAAC GCTTGCCTCC GTACTGGCTG AAGCGAGGGC ACAGGCATGA  100
<210> 2053
<211> 100

<212> DNA
<213> Escherichia coli K-12 MG1655
<220> yags b0286
<222> 764-863
<223> yags b0286 U00096 complement(300155..301111)
<313> 2053: 824-843
<400> 2053
GCGTCGCGCT GGGCGGAGTA GCACATAAGC CCTGGCGCAT TGAGGCTGCG 50
GATGCTCAGC TATCCCAGGG GGCGCAGGCC GTATATGACA CGCTGTTCGC 100
<210> 2054
<211> 100
<212> DNA
<213> Escherichia coli K-12 MG1655
<220> b1458 b1458
<222> 639-738
<223> b1458 b1458 U00096 1528610..1529356
<313> 2054: 659-678
<400> 2054
TGTGTTTTCT AATTATAAAG GCGATTCGTT TAGTACGCAG GAGATAAGTC 50
ATGGAAGAAA AGAAACACGT TTGCATATTG TCAGTAACGT AACGCCTGAA 100
<210> 2055
<211> 100
<212> DNA
<213> Escherichia coli K-12 MG1655
<220> b1458 b1458
<222> 379-478
<223> b1458 b1458 U00096 1528610..1529356
<313> 2055: 436-455
<400> 2055
CATATGGTGA GTGCATTCTC GAACGAAAAT GGTGTTGTAC TGGGGCAGGT 50
GAAAACGGAA GCCAAAAGTA ATGAGATTAC AGCCATTCCA GAGTTGCTTA 100
<210> 2056
<211> 100
<212> DNA
<213> Escherichia coli K-12 MG1655
<220> b1458 b1458
<222> 232-331
<223> b1458 b1458 U00096 1528610..1529356
<313> 2056: 280-299
<400> 2056
GTGAGTAACA TTGACAGTTT GGCCTTTGAA AAGATGTTTA TTGAATGGAT 50
GCAGGAGTGC CATGAAATCA CTGATGGTGA AATTATAGCA ATAGATGGAA 100
<210> 2057
<211> 100
<212> DNA
<213> Escherichia coli K-12 MG1655
<220> yagt b0287
<222> 591-690
<223> yagt b0287 U00096 complement(301108..301797)
<313> 2057: 660-679
<400> 2057
TGAGATCCGT GAACGTATGA GCGGCAACAT CTGTCGCTGT GGTGCATACG 50
CTAACATCCT TGCCGCCATT GAAGATGCTG CGGGGGAGAT AAAATCATGA 100
<210> 2058
<211> 100
<212> DNA
<213> Escherichia coli K-12 MG1655
<220> yagt b0287
<222> 540-639
<223> yagt b0287 U00096 complement(301108..301797)
<313> 2058: 594-613
<400> 2058
TCCCAGTCAC GTCACGGTCG ATTTGGTTTC CGCTCCAGAA ACAACTGCCG 50
ATGAGATCCG TGAACGTATG AGCGGCAACA TCTGTCGCTG TGGTGCATAC 100
<210> 2059
<211> 100
<212> DNA
<213> Escherichia coli K-12 MG1655
<220> yagt b0287
<222> 518-617
<223> yagt b0287 U00096 complement(301108..301797)

<313> 2059: 549-568
<400> 2059
TAAAAGAGAT TCAGGACGGC ATTCCCAGTC ACGTCACGGT CGATTTGGTT 50
TCCGCTCCAG AAACAACTGC CGATGAGATC CGTGAACGTA TGAGCGGCAA 100
<210> 2060
<211> 100
<212> DNA
<213> Escherichia coli K-12 MG1655
<220> b1459 b1460
<222> 102-201
<223> b1459 b1460 U00096 1529400..1529600
<313> 2060: 170-189
<400> 2060
TATCAGAGCG CACTGGCTGA TCGAGCACAG TCTTCATTGG GTGTTAGATG 50
TAAAAATGAA TGAAGATGCC AGCCGGATAA GAAGAGGAAA CGCAGCCTAA 100
<210> 2061
<211> 100
<212> DNA
<213> Escherichia coli K-12 MG1655
<220> b1459 b1460
<222> 85-184
<223> b1459 b1460 U00096 1529400..1529600
<313> 2061: 135-154
<400> 2061
AAAGAATTTG CACATGCTAT CAGAGCGCAC TGGCTGATCG AGCACAGTCT 50
TCATTGGGTG TTAGATGTAA AAATGAATGA AGATGCCAGC CGGATAAGAA 100
<210> 2062
<211> 100
<212> DNA
<213> Escherichia coli K-12 MG1655
<220> b1459 b1460
<222> 29-128
<223> b1459 b1460 U00096 1529400..1529600
<313> 2062: 84-103
<400> 2062
AATCAGCAGA AGGTGTAAGC ATCCGATATT ATATTTCATC AAAGGATATG 50
GATGCTAAAG AATTTGCACA TGCTATCAGA GCGCACTGGC TGATCGAGCA 100
<210> 2063
<211> 100
<212> DNA
<213> Escherichia coli K-12 MG1655
<220> yagu b0288
<222> 432-531
<223> yagu b0288 U00096 302215..302829
<313> 2063: 475-494
<400> 2063
TATGATTTCA TTCCCTCTCA TGGGACTGAC GCCACCTCTG TTTGATCTCC 50
CGTGGTATGA GAATGTTTCT GAAATTTTTG GACATTTAGT CTGGTTCTGG 100
<210> 2064
<211> 100
<212> DNA
<213> Escherichia coli K-12 MG1655
<220> yagu b0288
<222> 354-453
<223> yagu b0288 U00096 302215..302829
<313> 2064: 403-422
<400> 2064
TGTGGTCGCT GAAGTATTTC CAAAAATTAA ACTCTGGCAG GGCTTACTGG 50
CAGGTGCTTT AGCCCAACTT TTTGTTCATA TGATTTCATT CCCTCTCATG 100
<210> 2065
<211> 100
<212> DNA
<213> Escherichia coli K-12 MG1655
<220> yagu b0288
<222> 175-274
<223> yagu b0288 U00096 302215..302829
<313> 2065: 232-251
<400> 2065
AGGGCTGCAG GCCAAATTGA TTGCTCGCGT AATTTTCTCA ATCCACCGTA 50
TATTTTTCTT CGAGACTGGT TGGGGCTGAC AGATCCCAAT GCGGCTGTTT 100
<210> 2066

<211> 100
<212> DNA
<213> Escherichia coli K-12 MG1655
<220> yagv b0290
<222> 657-756
<223> yagv b0290 U00096 complement(303719..304474)
<313> 2066: 729-748
<400> 2066
GCCTGAGTTA CGTCAGCCGG GGAATCACTA TCTGGTCTAT AACGACAAAT 50
TCATTAAGAT TAGCGATTCC TGTCCGGCTA AGCCCCCTTC GGCGGACTAA 100
<210> 2067
<211> 100
<212> DNA
<213> Escherichia coli K-12 MG1655
<220> yagv b0290
<222> 651-750
<223> yagv b0290 U00096 complement(303719..304474)
<313> 2067: 683-702
<400> 2067
TCATCAGCCT GAGTTACGTC AGCCGGGGAA TCACTATCTG GTCTATAACG 50
ACAAATTCAT TAAGATTAGC GATTCCTGTC CGGCTAAGCC CCCTTCGGCG 100
<210> 2068
<211> 100
<212> DNA
<213> Escherichia coli K-12 MG1655
<220> yagv b0290
<222> 590-689
<223> yagv b0290 U00096 complement(303719..304474)
<313> 2068: 617-636
<400> 2068
CAGGATGTGA TTCGACCGAA GAGGAAGGCG ATGCCTGGTA TCTACGTCCG 50
GGAGACGTGG TTCATCAGCC TGAGTTACGT CAGCCGGGGA ATCACTATCT 100
<210> 2069
<211> 100
<212> DNA
<213> Escherichia coli K-12 MG1655
<220> yagw b0291
<222> 1485-1584
<223> yagw b0291 U00096 complement(304398..306041)
<313> 2069: 1506-1525
<400> 2069
CTCTGGCGAA GTGGGGCAGA TGGATAAGAC CACAGTCAAA TTTTCGATTC 50
CAATGGATAA CGCCATTTCG CTGCGTACGG TAGATGATAA CGGCTGGTTT 100
<210> 2070
<211> 100
<212> DNA
<213> Escherichia coli K-12 MG1655
<220> yagw b0291
<222> 1400-1499
<223> yagw b0291 U00096 complement(304398..306041)
<313> 2070: 1440-1459
<400> 2070
CTACAAAAAC CTACGATGCC GGGTGCGATG ATAGCTGGCG GGATATGACC 50
GATGCGCTGT GGTTGACCAC ACCGTGGACT GATATCTCTG GCGAAGTGGG 100
<210> 2071
<211> 100
<212> DNA
<213> Escherichia coli K-12 MG1655
<220> yagw b0291
<222> 1357-1456
<223> yagw b0291 U00096 complement(304398..306041)
<313> 2071: 1397-1416
<400> 2071
GTACCGTTCC CGGCGACACT TTCCTTTATT ACCCGCAACG GAGCTACAAA 50
AACCTACGAT GCCGGGTGCG ATGATAGCTG GCGGGATATG ACCGATGCGC 100
<210> 2072
<211> 100
<212> DNA
<213> Escherichia coli K-12 MG1655
<220> yagx b0292
<222> 2427-2526

<223> yagx b0292 U00096 complement(306031..308556)
<313> 2072: 2498-2517
<400> 2072
CAACCAGGCG CGCGGTGCCG TCTGGGTCGG TGATGTGGTC TGCAGCGGCC 50
TCTCATCGTG GGCGGCGGTG ACGCAGACAG GAGAAGAGAA TGAGAGTTAA 100
<210> 2073
<211> 100
<212> DNA
<213> Escherichia coli K-12 MG1655
<220> yagx b0292
<222> 2357-2456
<223> yagx b0292 U00096 complement(306031..308556)
<313> 2073: 2411-2430
<400> 2073
TGGATAAGAA ATACCCCACT ATCGATTTTC GCTACAGTGG CAATAAAACC 50
TGCGAAGTGG CTCTGGAACT CAACCAGGCG CGCGGTGCCG TCTGGGTCGG 100
<210> 2074
<211> 100
<212> DNA
<213> Escherichia coli K-12 MG1655
<220> yagx b0292
<222> 2246-2345
<223> yagx b0292 U00096 complement(306031..308556)
<313> 2074: 2277-2296
<400> 2074
CCGTCTCCGG TCGTATCCGT GCGGAAGACG GCACACTGCT GGCTAACGCA 50
CGGATTAACA ACCATATCGG CCGAACCCGA ACCGATGAAA ACGGCGAGTT 100
<210> 2075
<211> 100
<212> DNA
<213> Escherichia coli K-12 MG1655
<220> yagy b0293
<222> 570-669
<223> yagy b0293 U00096 complement(308582..309250)
<313> 2075: 623-642
<400> 2075
GATGCCGGGC AAGTCGCGTC GTTTTACCCG CGTGGACACT GCGGATAACA 50
AAGGACGGGT TGCACTTTGG CAGGGTGATA AGTTCATTCC CGTGAAATAG 100
<210> 2076
<211> 100
<212> DNA
<213> Escherichia coli K-12 MG1655
<220> yagy b0293
<222> 519-618
<223> yagy b0293 U00096 complement(308582..309250)
<313> 2076: 565-584
<400> 2076
ACCTTGCCTG AAAGCCGCCA ACGGTAAGGA GTGTAAAGAG AATTACTACC 50
TGATGCCGGG CAAGTCGCGT CGTTTTACCC GCGTGGACAC TGCGGATAAC 100
<210> 2077
<211> 100
<212> DNA
<213> Escherichia coli K-12 MG1655
<220> yagy b0293
<222> 457-556
<223> yagy b0293 U00096 complement(308582..309250)
<313> 2077: 485-504
<400> 2077
CAGTACGCCA ACGGCTCCCT GACAAATACA GGAAATGCGA CGCTGCGGAT 50
CCTCGCCTAC GGACCTTGCC TGAAAGCCGC CAACGGTAAG GAGTGTAAAG 100
<210> 2078
<211> 100
<212> DNA
<213> Escherichia coli K-12 MG1655
<220> yagz b0294
<222> 487-586
<223> yagz b0294 U00096 complement(309308..309895)
<313> 2078: 525-544
<400> 2078
ACCACCAATG GTACCACCGC AGTAACAGAT TACAGCACTC TACCGGAAGG 50
CATCTGGAGC GGCGACGTTA GCGTACAGTT CGACGCGACC TGGACCAGTT 100

<210> 2079
<211> 100
<212> DNA
<213> Escherichia coli K-12 MG1655
<220> yagz b0294
<222> 401-500
<223> yagz b0294 U00096 complement(309308..309895)
<313> 2079: 454-473
<400> 2079
GCAACCTTAG CCCGCTGGCT AACGGTTACA ATGCCAGCAA TCGTACCACC 50
GCACAGGATG GTTTCACCTT CTCCATCATC AGCGGTACCA CCAATGGTAC 100
<210> 2080
<211> 100
<212> DNA
<213> Escherichia coli K-12 MG1655
<220> yagz b0294
<222> 323-422
<223> yagz b0294 U00096 complement(309308..309895)
<313> 2080: 363-382
<400> 2080
GCGTGGATTA TAACGGCGCG GCAGTCGAAA AAACTGGCGA TACCGTGATG 50
ATCGATACCG CCAACGGCGT ACTGGGCGGC AACCTTAGCC CGCTGGCTAA 100
<210> 2081
<211> 100
<212> DNA
<213> Escherichia coli K-12 MG1655
<220> yaha b0316
<222> 967-1066
<223> yaha b0316 U00096 331595..332683
<313> 2081: 1030-1049
<400> 2081
ACCCAGGAGC AGGCGGATTT AATGATCGGT AAAGGCGTTC ACTTTTTGCA 50
GGGCTATTTG TACTCTCCGC CAGTACCGGG TAATAAATTT ATCTCTGAAT 100
<210> 2082
<211> 100
<212> DNA
<213> Escherichia coli K-12 MG1655
<220> yaha b0316
<222> 953-1052
<223> yaha b0316 U00096 331595..332683
<313> 2082: 978-997
<400> 2082
CGGAAGGGGT AGAAACCCAG GAGCAGGCGG ATTTAATGAT CGGTAAAGGC 50
GTTCACTTTT TGCAGGGCTA TTTGTACTCT CCGCCAGTAC CGGGTAATAA 100
<210> 2083
<211> 100
<212> DNA
<213> Escherichia coli K-12 MG1655
<220> yaha b0316
<222> 886-985
<223> yaha b0316 U00096 331595..332683
<313> 2083: 944-963
<400> 2083
GAAATCTCCG GTCATATTGT GGACAATATT GTCGAACTAG CGCGTAAGCC 50
TGGTCTGAGT ATCGTGGCGG AAGGGGTAGA AACCCAGGAG CAGGCGGATT 100
<210> 2084
<211> 100
<212> DNA
<213> Escherichia coli K-12 MG1655
<220> yahb b0317
<222> 834-933
<223> yahb b0317 U00096 complement(332725..333657)
<313> 2084: 886-905
<400> 2084
TTCTGCTACG GGTCAGGTGA CGCAGTGGAT TAAAAAACAA TTTGCGCCAA 50
ATGGCATATT AACGGGGATC TATCAGGATT TACTGCATCG GGAGAATTAA 100
<210> 2085
<211> 100
<212> DNA
<213> Escherichia coli K-12 MG1655
<220> yahb b0317

<222> 790-889
<223> yahb b0317 U00096 complement(332725..333657)
<313> 2085: 841-860
<400> 2085
CATAATCCGC GCCAGGATTC GCGCATGTTA CTGGCGACTC AGCATTCTGC  50
TACGGGTCAG GTGACGCAGT GGATTAAAAA ACAATTTGCG CCAAATGGCA  100
<210> 2086
<211> 100
<212> DNA
<213> Escherichia coli K-12 MG1655
<220> yahb b0317
<222> 756-855
<223> yahb b0317 U00096 complement(332725..333657)
<313> 2086: 787-806
<400> 2086
GATGACGCAA TCCCTGCTGG TTACCCGACA AATCCATAAT CCGCGCCAGG  50
ATTCGCGCAT GTTACTGGCG ACTCAGCATT CTGCTACGGG TCAGGTGACG  100
<210> 2087
<211> 100
<212> DNA
<213> Escherichia coli K-12 MG1655
<220> yahc b0318
<222> 399-498
<223> yahc b0318 U00096 complement(333749..334246)
<313> 2087: 474-493
<400> 2087
GACGTTACTG GGTTTTCTGG CGGGTAACTG CGTGGGGGTA ATTTCAGAAT  50
ATGGTGGACA AAAATTAAGC GAAGCGACGA CTAAACGCGA CGGGTATTAA  100
<210> 2088
<211> 100
<212> DNA
<213> Escherichia coli K-12 MG1655
<220> yahc b0318
<222> 345-444
<223> yahc b0318 U00096 complement(333749..334246)
<313> 2088: 371-390
<400> 2088
CGGTGGCTTT CTCGGCATGA CGCTGTTTTT TGCCAGCGGG ATGAACTGGA  50
CGGTGACGTT ACTGGGTTTT CTGGCGGGTA ACTGCGTGGG GGTAATTTCA  100
<210> 2089
<211> 100
<212> DNA
<213> Escherichia coli K-12 MG1655
<220> yahc b0318
<222> 274-373
<223> yahc b0318 U00096 complement(333749..334246)
<313> 2089: 304-323
<400> 2089
GTAATTACTA CCGTACCCTT CTCGGCGGTA GTGGTCTGGC AAGGGCGTTT  50
CTGGTTACTG TCCTTTATTC CCGGTGGCTT TCTCGGCATG ACGCTGTTTT  100
<210> 2090
<211> 100
<212> DNA
<213> Escherichia coli K-12 MG1655
<220> yahd b0319
<222> 493-592
<223> yahd b0319 U00096 334504..335109
<313> 2090: 534-553
<400> 2090
GAACACGGTG CCAGCCCGCA TCTGACCGAT AAATATGGCA AAACGCCACT  50
GGAACTGGCG CGGGAACGGG GCTTTGAAGA GATTGCGCAG TTACTGATTG  100
<210> 2091
<211> 100
<212> DNA
<213> Escherichia coli K-12 MG1655
<220> yahd b0319
<222> 457-556
<223> yahd b0319 U00096 334504..335109
<313> 2091: 489-508
<400> 2091
GGTATTAAAC AGCAGGCGAT TGTGCAGTTA TTGCTGGAAC ACGGTGCCAG  50

CCCGCATCTG ACCGATAAAT ATGGCAAAAC GCCACTGGAA CTGGCGCGGG 100
<210> 2092
<211> 100
<212> DNA
<213> Escherichia coli K-12 MG1655
<220> yahd b0319
<222> 393-492
<223> yahd b0319 U00096 334504..335109
<313> 2092: 456-475
<400> 2092
CGTTAACCAG ACCAACCATG TAGGCTGGAC GCCGCTACTG GAAGCGATTG 50
TGCTTAATGA TGGTGGTATT AAACAGCAGG CGATTGTGCA GTTATTGCTG 100
<210> 2093
<211> 100
<212> DNA
<213> Escherichia coli K-12 MG1655
<220> yahe b0320
<222> 765-864
<223> yahe b0320 U00096 335149..336012
<313> 2093: 829-848
<400> 2093
AATTAAACAT ATTGGCTCCA GTTCCGGCTG CGACATGCTG TATGGCATGG 50
CCGATGGTTG TGCGCTGAGC CAAACCTACG GAGGGAATTA TGTCAGTTAA 100
<210> 2094
<211> 100
<212> DNA
<213> Escherichia coli K-12 MG1655
<220> yahe b0320
<222> 740-839
<223> yahe b0320 U00096 335149..336012
<313> 2094: 769-788
<400> 2094
GGAACGCAAC TCAGGCAATA GAAAAAATTA AACATATTGG CTCCAGTTCC 50
GGCTGCGACA TGCTGTATGG CATGGCCGAT GGTTGTGCGC TGAGCCAAAC 100
<210> 2095
<211> 100
<212> DNA
<213> Escherichia coli K-12 MG1655
<220> yahe b0320
<222> 651-750
<223> yahe b0320 U00096 335149..336012
<313> 2095: 673-692
<400> 2095
AAGTGCCATA ACGCTGGAAG CCGCATTACA ACAACGCTGC CGGGAAAATA 50
TTCATCGTTT TATTCACAAC ATTATTTATG ACATCCCTGG GAACGCAACT 100
<210> 2096
<211> 100
<212> DNA
<213> Escherichia coli K-12 MG1655
<220> yahf b0321
<222> 1399-1498
<223> yahf b0321 U00096 336002..337549
<313> 2096: 1442-1461
<400> 2096
CTCGCCTATG TGCTGGGTAC CGATCTTGAT ACGCCATCGT TAGAACAACA 50
AAGCCAGATG CTGCTTGATG CCGGAGTGAT TCTGGCGAGC AGCAGCACCA 100
<210> 2097
<211> 100
<212> DNA
<213> Escherichia coli K-12 MG1655
<220> yahf b0321
<222> 1367-1466
<223> yahf b0321 U00096 336002..337549
<313> 2097: 1382-1401
<400> 2097
TCGCCGCTGC CGAAGGACGC GAGTTGATCA TTCTCGCCTA TGTGCTGGGT 50
ACCGATCTTG ATACGCCATC GTTAGAACAA CAAAGCCAGA TGCTGCTTGA 100
<210> 2098
<211> 100
<212> DNA
<213> Escherichia coli K-12 MG1655

<220> yahf b0321
<222> 1314-1413
<223> yahf b0321 U00096 336002..337549
<313> 2098: 1346-1365
<400> 2098
GCATGAAGAT CCGGTCGGCT CCACCATCGA GACGATCAAA GAAGCGAAAG 50
CGATCGCCGC TGCCGAAGGA CGCGAGTTGA TCATTCTCGC CTATGTGCTG 100
<210> 2099
<211> 100
<212> DNA
<213> Escherichia coli K-12 MG1655
<220> thic b3995
<222> 1797-1896
<223> thic b3995 U00096 complement(4191782..4193677)
<313> 2099: 1874-1893
<400> 2099
TTACGCCGCC ACGCAAACTA TTGAAATGGG AATGGCGGAT ATGTCGGAGA 50
ACTTCCGTGC CAGAGGCGGA GAAATCTACC TGCGTAAGGA GGAAGCGTGA 100
<210> 2100
<211> 100
<212> DNA
<213> Escherichia coli K-12 MG1655
<220> thic b3995
<222> 1740-1839
<223> thic b3995 U00096 complement(4191782..4193677)
<313> 2100: 1773-1792
<400> 2100
TTGCTCCATG TGTGGGCCGA AATTCTGCTC GATGAAAATC AGCCAGGAAG 50
TGCGTGATTA CGCCGCCACG CAAACTATTG AAATGGGAAT GGCGGATATG 100
<210> 2101
<211> 100
<212> DNA
<213> Escherichia coli K-12 MG1655
<220> thic b3995
<222> 1663-1762
<223> thic b3995 U00096 complement(4191782..4193677)
<313> 2101: 1718-1737
<400> 2101
CTGGCCCTCG ACCCGTTTAC CGCCCGCGCT TATCACGATG AAACCCTGCC 50
GCAAGAGTCA GGTAAAGTCG CCCATTTTTG CTCCATGTGT GGGCCGAAAT 100
<210> 2102
<211> 100
<212> DNA
<213> Escherichia coli K-12 MG1655
<220> yahg b0322
<222> 1320-1419
<223> yahg b0322 U00096 337549..338967
<313> 2102: 1397-1416
<400> 2102
AGATGCGGGC GTCGGCATGA TTGGCGCGGG CATTGTGCAT CCACCTTTTG 50
CCTGCTTCGA GAAAGCCATT CTTGGCTGGT GCGAACGTTA CGGCGTCTGA 100
<210> 2103
<211> 100
<212> DNA
<213> Escherichia coli K-12 MG1655
<220> yahg b0322
<222> 1285-1384
<223> yahg b0322 U00096 337549..338967
<313> 2103: 1345-1364
<400> 2103
ATTCTGCCGG TGATCAACAC CGCCATCGCC CATAAAGATG CGGGCGTCGG 50
CATGATTGGC GCGGGCATTG TGCATCCACC TTTTGCCTGC TTCGAGAAAG 100
<210> 2104
<211> 100
<212> DNA
<213> Escherichia coli K-12 MG1655
<220> yahg b0322
<222> 1178-1277
<223> yahg b0322 U00096 337549..338967
<313> 2104: 1212-1231
<400> 2104

AGATGCGCGA AATCACCCTC GGTGAAAACC CCAACGTCAC CATTCCGCTG 50
CTCGGTTTTA TGGGCGTGCC GTCGGCAATC GACATCACCC GCGTGGGTAG 100
<210> 2105
<211> 100
<212> DNA
<213> Escherichia coli K-12 MG1655
<220> thid b2104
<222> 702-801
<223> thid b2104 U00096 complement(2181736..2182536)
<313> 2105: 762-781
<400> 2105
GGAGGCAAAA AGCTGGCTTT CATCGGCGTT AGCCCAGGCC GACACGCTGG 50
AAGTTGGTCA CGGTATTGGT CCGGTTCACC ACTTCCACGC CTGGTGGTGA 100
<210> 2106
<211> 100
<212> DNA
<213> Escherichia coli K-12 MG1655
<220> thid b2104
<222> 652-751
<223> thid b2104 U00096 complement(2181736..2182536)
<313> 2106: 691-710
<400> 2106
GCGTTGGCTG CACTACGCCC GCGCCATACA AACTGGGCTG ACACCGTACA 50
GGAGGCAAAA AGCTGGCTTT CATCGGCGTT AGCCCAGGCC GACACGCTGG 100
<210> 2107
<211> 100
<212> DNA
<213> Escherichia coli K-12 MG1655
<220> thid b2104
<222> 595-694
<223> thid b2104 U00096 complement(2181736..2182536)
<313> 2107: 618-637
<400> 2107
ACCGCACCGC GCATTATGAC CAAAAACACC CACGGCACTG GTTGTACACT 50
CTCTGCGGCG TTGGCTGCAC TACGCCCGCG CCATACAAAC TGGGCTGACA 100
<210> 2108
<211> 100
<212> DNA
<213> Escherichia coli K-12 MG1655
<220> yahh b0323
<222> 222-321
<223> yahh b0323 U00096 338993..339313
<313> 2108: 289-308
<400> 2108
ATCTGTAGGC CTGATAAGCG TAGCGCATCA GGCATTTGTC ACCATTGCCG 50
GATGCGGCGT GAACGCCTTA TCCGGCCTAC GAGTGGCGCG AGAATTGTAG 100
<210> 2109
<211> 100
<212> DNA
<213> Escherichia coli K-12 MG1655
<220> yahh b0323
<222> 148-247
<223> yahh b0323 U00096 338993..339313
<313> 2109: 208-227
<400> 2109
GTAGCGCATC AGGCATTTGC CACCACTGCC GGATGCGGCG TGGACGCCTT 50
ATCCGGCCTA CGAGTGGCGC GAGAATCTGT AGGCCTGATA AGCGTAGCGC 100
<210> 2110
<211> 100
<212> DNA
<213> Escherichia coli K-12 MG1655
<220> yahh b0323
<222> 114-213
<223> yahh b0323 U00096 338993..339313
<313> 2110: 149-168
<400> 2110
ACGAATGGCG CAAGAATCTG TAGGCCTGAT AAGCGTAGCG CATCAGGCAT 50
TTGCCACCAC TGCCGGATGC GGCGTGGACG CCTTATCCGG CCTACGAGTG 100
<210> 2111
<211> 100
<212> DNA

<213> Escherichia coli K-12 MG1655
<220> thie b3994
<222> 537-636
<223> thie b3994 U00096 complement(4191147..4191782)
<313> 2111: 616-635
<400> 2111
TGTCGGCAGT ATCGCCGTCG TCAGCGCCAT TACTCAAGCC GCAGACTGGC  50
GTTTGGCAAC GGCACAGTTG CTGGAAATTG CAGGAGTTGG CGATGAATGA  100
<210> 2112
<211> 100
<212> DNA
<213> Escherichia coli K-12 MG1655
<220> thie b3994
<222> 530-629
<223> thie b3994 U00096 complement(4191147..4191782)
<313> 2112: 546-565
<400> 2112
CAACGGGTGT CGGCAGTATC GCCGTCGTCA GCGCCATTAC TCAAGCCGCA  50
GACTGGCGTT TGGCAACGGC ACAGTTGCTG GAAATTGCAG GAGTTGGCGA  100
<210> 2113
<211> 100
<212> DNA
<213> Escherichia coli K-12 MG1655
<220> thie b3994
<222> 462-561
<223> thie b3994 U00096 complement(4191147..4191782)
<313> 2113: 478-497
<400> 2113
GCGACTGGCG GATTATCCCA CCGTGGCGAT TGGCGGTATC AGTCTGGCAC  50
GCGCGCCTGC GGTGATAGCA ACGGGTGTCG GCAGTATCGC CGTCGTCAGC  100
<210> 2114
<211> 100
<212> DNA
<213> Escherichia coli K-12 MG1655
<220> yahi b0324
<222> 846-945
<223> yahi b0324 U00096 339389..340339
<313> 2114: 873-892
<400> 2114
CCTGACATTT TTAGAACAAG GCGGCAAAGA AGTGATTATC ACCACGCCGG  50
AATGCCTGCC TGCGGCGCTG CGCGGCGAAA CGGGCACTCA TATTATTAAA  100
<210> 2115
<211> 100
<212> DNA
<213> Escherichia coli K-12 MG1655
<220> yahi b0324
<222> 808-907
<223> yahi b0324 U00096 339389..340339
<313> 2115: 844-863
<400> 2115
TTCCCGCCCG GCAGCATGTT GCCAAAAATC ATCGCCAGCC TGACATTTTT  50
AGAACAAGGC GGCAAAGAAG TGATTATCAC CACGCCGGAA TGCCTGCCTG  100
<210> 2116
<211> 100
<212> DNA
<213> Escherichia coli K-12 MG1655
<220> yahi b0324
<222> 745-844
<223> yahi b0324 U00096 339389..340339
<313> 2116: 801-820
<400> 2116
CAGCAGCAGG CGCTCGATCG GGTGGATATT GCCACCATGA CCCGCTATAT  50
GCAGGAAGGG CATTTCCCGC CCGGCAGCAT GTTGCCAAAA ATCATCGCCA  100
<210> 2117
<211> 100
<212> DNA
<213> Escherichia coli K-12 MG1655
<220> thif b3993
<222> 639-738
<223> thif b3993 U00096 complement(4190399..4191136)
<313> 2117: 719-738

<400> 2117
CCGACTGTTC GACGGTAAAT CGAGCCAGTG GCGCAGCCTG GCGTTGCGCC 50
GCGCCAGTGG TTGCCCGGTA TGCGGAGGAA GCAATGCAGA TCCTGTTTAA 100
<210> 2118
<211> 100
<212> DNA
<213> Escherichia coli K-12 MG1655
<220> thif b3993
<222> 602-701
<223> thif b3993 U00096 complement(4190399..4191136)
<313> 2118: 645-664
<400> 2118
AGTTATTAAG CGGTATAGAG ACACCTGCGG GAGAACTCCG ACTGTTCGAC 50
GGTAAATCGA GCCAGTGGCG CAGCCTGGCG TTGCGCCGCG CCAGTGGTTG 100
<210> 2119
<211> 100
<212> DNA
<213> Escherichia coli K-12 MG1655
<220> thif b3993
<222> 553-652
<223> thif b3993 U00096 complement(4190399..4191136)
<313> 2119: 583-602
<400> 2119
GGCCCGGTGG TCGGGGTTAT GGGCACTTTG CAGGCACTGG AAGCCATTAA 50
GTTATTAAGC GGTATAGAGA CACCTGCGGG AGAACTCCGA CTGTTCGACG 100
<210> 2120
<211> 100
<212> DNA
<213> Escherichia coli K-12 MG1655
<220> yahj b0325
<222> 1284-1383
<223> yahj b0325 U00096 340349..341731
<313> 2120: 1358-1377
<400> 2120
GGTGGACGCC TCCTGTTCCG CCGAGGCGGT GGCGCGTATC TCGCCGAGAA 50
CCGCAACGTT CCATAAAGGG CAACTGGTGT GGGGGAGTGT GGCAGGTTGA 100
<210> 2121
<211> 100
<212> DNA
<213> Escherichia coli K-12 MG1655
<220> yahj b0325
<222> 1266-1365
<223> yahj b0325 U00096 340349..341731
<313> 2121: 1308-1327
<400> 2121
TGACGCCAGC TTTGTGCTGG TGGACGCCTC CTGTTCCGCC GAGGCGGTGG 50
CGCGTATCTC GCCGAGAACC GCAACGTTCC ATAAAGGGCA ACTGGTGTGG 100
<210> 2122
<211> 100
<212> DNA
<213> Escherichia coli K-12 MG1655
<220> yahj b0325
<222> 1137-1236
<223> yahj b0325 U00096 340349..341731
<313> 2122: 1187-1206
<400> 2122
AGCCAATCTG TACGCGCAGC TCTATATTCG TCCTAACGAA CAGAACCTCT 50
CCCGCTCGCT GTTTTTAGCC ACTGGCGATG TATTGCCGCT GAATGAAAAA 100
<210> 2123
<211> 100
<212> DNA
<213> Escherichia coli K-12 MG1655
<220> thig b3992
<222> 745-844
<223> thig b3992 U00096 complement(4189443..4190288)
<313> 2123: 792-811
<400> 2123
GTAGAAGCAG GCCTACTGGC ACGTCAGTCC GGACCGGGCA GCCGCAGTTA 50
TTTTGCTCAT GCCACCAGCC CGCTGACCGG ATTTCTGGAG GCATCGGCAT 100
<210> 2124
<211> 100

<212> DNA
<213> Escherichia coli K-12 MG1655
<220> thig b3992
<222> 687-786
<223> thig b3992 U00096 complement(4189443..4190288)
<313> 2124: 703-722
<400> 2124
TACGGCGATT GCCGTCGCGG ACGATCCCGT CAACATGGCG AAGGCATTTC 50
GTCTGGCGGT AGAAGCAGGC CTACTGGCAC GTCAGTCCGG ACCGGGCAGC 100
<210> 2125
<211> 100
<212> DNA
<213> Escherichia coli K-12 MG1655
<220> thig b3992
<222> 631-730
<223> thig b3992 U00096 complement(4189443..4190288)
<313> 2125: 658-677
<400> 2125
GTTCCCAGCC ATGCCGCGCA GGCGCTGGAA ATGGGGGCCG ACGCGGTGTT 50
AGTGAATACG GCGATTGCCG TCGCGGACGA TCCCGTCAAC ATGGCGAAGG 100
<210> 2126
<211> 100
<212> DNA
<213> Escherichia coli K-12 MG1655
<220> yahk b0326
<222> 915-1014
<223> yahk b0326 U00096 342108..343157
<313> 2126: 972-991
<400> 2126
TTTTTGCGCC GAACATGGCA TCGTGGCTGA TATAGAGATG ATTCGGGCCG 50
ATCAAATTAA TGAAGCCTAT GAGCGAATGC TGCGCGGTGA TGTGAAATAT 100
<210> 2127
<211> 100
<212> DNA
<213> Escherichia coli K-12 MG1655
<220> yahk b0326
<222> 894-993
<223> yahk b0326 U00096 342108..343157
<313> 2127: 936-955
<400> 2127
AGAAACTCAG GAGATGCTCG ATTTTTGCGC CGAACATGGC ATCGTGGCTG 50
ATATAGAGAT GATTCGGGCC GATCAAATTA ATGAAGCCTA TGAGCGAATG 100
<210> 2128
<211> 100
<212> DNA
<213> Escherichia coli K-12 MG1655
<220> yahk b0326
<222> 849-948
<223> yahk b0326 U00096 342108..343157
<313> 2128: 880-899
<400> 2128
CATGAAACGC CGTGCGATAG CCGGTTCTAT GATTGGCGGC ATTCCAGAAA 50
CTCAGGAGAT GCTCGATTTT TGCGCCGAAC ATGGCATCGT GGCTGATATA 100
<210> 2129
<211> 100
<212> DNA
<213> Escherichia coli K-12 MG1655
<220> thih b3991
<222> 1035-1134
<223> thih b3991 U00096 complement(4188313..4189446)
<313> 2129: 1110-1129
<400> 2129
CAGACCGGAA GCGGTTGCTG CCGCGTTAAC CGCTCAGGGT TTGCAGCCGG 50
TATGGAAAGA CTGGGACAGC TATCTGGGAC GCGCCTCGCA AAGACTATGA 100
<210> 2130
<211> 100
<212> DNA
<213> Escherichia coli K-12 MG1655
<220> thih b3991
<222> 1035-1134
<223> thih b3991 U00096 complement(4188313..4189446)

<313> 2130: 1069-1088
<400> 2130
CAGACCGGAA GCGGTTGCTG CCGCGTTAAC CGCTCAGGGT TTGCAGCCGG 50
TATGGAAAGA CTGGGACAGC TATCTGGGAC GCGCCTCGCA AAGACTATGA 100
<210> 2131
<211> 100
<212> DNA
<213> Escherichia coli K-12 MG1655
<220> thih b3991
<222> 976-1075
<223> thih b3991 U00096 complement(4188313..4189446)
<313> 2131: 1013-1032
<400> 2131
CCAGGTGGCT ATGCCGATAA TCACCCCGAG TTGGAACAGT TCTCACCGCA 50
CGACGATCGC AGACCGGAAG CGGTTGCTGC CGCGTTAACC GCTCAGGGTT 100
<210> 2132
<211> 100
<212> DNA
<213> Escherichia coli K-12 MG1655
<220> yahl b0327
<222> 374-473
<223> yahl b0327 U00096 343400..344215
<313> 2132: 424-443
<400> 2132
ATGACCAGGA TGATGCGTTT CTTGATATTA AAGCAAACGT GCAGGCTGGA 50
CGCTTTACCG GGCTTAAGAA ATTAGTGCAG TTTATCGATT CAGTGCGAAC 100
<210> 2133
<211> 100
<212> DNA
<213> Escherichia coli K-12 MG1655
<220> yahl b0327
<222> 327-426
<223> yahl b0327 U00096 343400..344215
<313> 2133: 356-375
<400> 2133
GTTTGTTTTT CACATACTTG AAAATCAAAA AGGGACAGTT TGGGTATATG 50
ACCAGGATGA TGCGTTTCTT GATATTAAAG CAAACGTGCA GGCTGGACGC 100
<210> 2134
<211> 100
<212> DNA
<213> Escherichia coli K-12 MG1655
<220> yahl b0327
<222> 250-349
<223> yahl b0327 U00096 343400..344215
<313> 2134: 289-308
<400> 2134
GCACTCACCA TAAATGTGCA GTTTAATGAT TTCCCTGGAT TTTATGACTG 50
GATGGATTAC CCTGATCACC CGGTTAAGTT TGTTTTTCAC ATACTTGAAA 100
<210> 2135
<211> 100
<212> DNA
<213> Escherichia coli K-12 MG1655
<220> yahm b0328
<222> 157-256
<223> yahm b0328 U00096 344598..344873
<313> 2135: 219-238
<400> 2135
GAGAGTGGGC TGAAATGGGA AGCTAAAGAC TCAAGTAAAC TTATCGGAAA 50
TAAGGACCAC GCATTACGGG GGCTATCATC GCCGATGGCC GTGATTCGGC 100
<210> 2136
<211> 100
<212> DNA
<213> Escherichia coli K-12 MG1655
<220> yahm b0328
<222> 129-228
<223> yahm b0328 U00096 344598..344873
<313> 2136: 161-180
<400> 2136
CAGACCAATG AAAAAAGCCC ACACAGGGGA GAGTGGGCTG AAATGGGAAG 50
CTAAAGACTC AAGTAAACTT ATCGGAAATA AGGACCACGC ATTACGGGGG 100
<210> 2137

<211> 100
<212> DNA
<213> Escherichia coli K-12 MG1655
<220> yahm b0328
<222> 98-197
<223> yahm b0328 U00096 344598..344873
<313> 2137: 129-148
<400> 2137
CATTGCCATT GTTTGGCTAT TCAGGATGGG GCAGACCAAT GAAAAAAGCC 50
CACACAGGGG AGAGTGGGCT GAAATGGGAA GCTAAAGACT CAAGTAAACT 100
<210> 2138
<211> 100
<212> DNA
<213> Escherichia coli K-12 MG1655
<220> thij b0425
<222> 498-597
<223> thij b0425 U00096 complement(442275..442871)
<313> 2138: 532-551
<400> 2138
TGGTCTGAAA ATTATCGACC TGTTGGTTGG GCGTGAAAAA GCCCATGAAG 50
TGGCATCACA ACTGGTGATG GCGGCAGGGA TTTATAATTA TTACGAGTAG 100
<210> 2139
<211> 100
<212> DNA
<213> Escherichia coli K-12 MG1655
<220> thij b0425
<222> 455-554
<223> thij b0425 U00096 complement(442275..442871)
<313> 2139: 487-506
<400> 2139
TAAAATTGCT GACCAGCCAG GGGCCGGGTA CAGCTATCGA CTTTGGTCTG 50
AAAATTATCG ACCTGTTGGT TGGGCGTGAA AAAGCCCATG AAGTGGCATC 100
<210> 2140
<211> 100
<212> DNA
<213> Escherichia coli K-12 MG1655
<220> thij b0425
<222> 412-511
<223> thij b0425 U00096 complement(442275..442871)
<313> 2140: 456-475
<400> 2140
GCCGAACAAT GGCTGGACAA GCGCGTCGTC TGGGATGCAC GGGTAAAATT 50
GCTGACCAGC CAGGGGCCGG GTACAGCTAT CGACTTTGGT CTGAAAATTA 100
<210> 2141
<211> 100
<212> DNA
<213> Escherichia coli K-12 MG1655
<220> b1462 b1462
<222> 482-581
<223> b1462 b1462 U00096 complement(1531306..1531923)
<313> 2141: 543-562
<400> 2141
AAGTAGTATC AGCAGCGGCA GACGCACGGG TATTTGTCGA AGGCCGCTGG 50
CAGTTTGATG ATGATAAGCT CAATACGTTG CATCATTTAG GTGCTGGGAC 100
<210> 2142
<211> 100
<212> DNA
<213> Escherichia coli K-12 MG1655
<220> b1462 b1462
<222> 444-543
<223> b1462 b1462 U00096 complement(1531306..1531923)
<313> 2142: 481-500
<400> 2142
GACTTCTGCG CAAGAAGAAT ACGACACGCT GTTTGGCGAA GTAGTATCAG 50
CAGCGGCAGA CGCACGGGTA TTTGTCGAAG GCCGCTGGCA GTTTGATGAT 100
<210> 2143
<211> 100
<212> DNA
<213> Escherichia coli K-12 MG1655
<220> b1462 b1462
<222> 366-465

<223> b1462 b1462 U00096 complement(1531306..1531923)
<313> 2143: 428-447
<400> 2143
TGTGAGAGGC CCGGTATTTG GTTTGCCTCT GGTCGAGGAA AAATGTCTGG 50
CGTGGATGGA GTGTCGATTG CTACCTGCGA CTTCTGCGCA AGAAGAATAC 100
<210> 2144
<211> 100
<212> DNA
<213> Escherichia coli K-12 MG1655
<220> b2191 b2192
<222> 1-100
<223> b2191 b2192 U00096 2286925..2287047
<313> 2144: 26-45
<400> 2144
ATGGGTGAAT ATAAGGAAGG TGCGAATAAG CGGGGAAATT CTTCTCGGCT 50
GACTCAGTCA TTTCATTTCT TCATGTTTGA GCCGATTTTT TCTCCCGTAA 100
<210> 2145
<211> 100
<212> DNA
<213> Escherichia coli K-12 MG1655
<220> b2191 b2192
<222> 1-100
<223> b2191 b2192 U00096 2286925..2287047
<313> 2145: 2-21
<400> 2145
ATGGGTGAAT ATAAGGAAGG TGCGAATAAG CGGGGAAATT CTTCTCGGCT 50
GACTCAGTCA TTTCATTTCT TCATGTTTGA GCCGATTTTT TCTCCCGTAA 100
<210> 2146
<211> 100
<212> DNA
<213> Escherichia coli K-12 MG1655
<220> yahn b0329
<222> 573-672
<223> yahn b0329 U00096 complement(344890..345561)
<313> 2146: 642-661
<400> 2146
TGCTTATGGG CGTATGCAAC GCGTTGCCAG TCGGGTTATT GGTGCAATTA 50
TTGGTGTATT CGCGCTACGC CTGATTTACG AAGGGGTGAC GCAGCGGTGA 100
<210> 2147
<211> 100
<212> DNA
<213> Escherichia coli K-12 MG1655
<220> yahn b0329
<222> 526-625
<223> yahn b0329 U00096 complement(344890..345561)
<313> 2147: 556-575
<400> 2147
TGGCGAGTTT TTCTTAGTCA GGCGTTTTCT TTGCCCGCTG TGCGTCGTGC 50
TTATGGGCGT ATGCAACGCG TTGCCAGTCG GGTTATTGGT GCAATTATTG 100
<210> 2148
<211> 100
<212> DNA
<213> Escherichia coli K-12 MG1655
<220> yahn b0329
<222> 448-547
<223> yahn b0329 U00096 complement(344890..345561)
<313> 2148: 466-485
<400> 2148
TCAGTAACAT TAAATGCCGA AACACCAACA TGGGCACGTT TAATGGCCTG 50
GGCGGGGATT GTGCTCGCAT CAATTATCTG GCGAGTTTTT CTTAGTCAGG 100
<210> 2149
<211> 100
<212> DNA
<213> Escherichia coli K-12 MG1655
<220> b1463 b1464
<222> 747-846
<223> b1463 b1464 U00096 1532048..1532893
<313> 2149: 826-845
<400> 2149
GTTTGGTCTG GGCGTGGATG ATGCGAAACA TGGCTTTACC GTGGATGAGT 50
TAGCGCTGGT GATGGCGGCG TTTGATACGC ACCCGGAGGC GGGAAAATAA 100

<210> 2150
<211> 100
<212> DNA
<213> Escherichia coli K-12 MG1655
<220> b1463 b1464
<222> 730-829
<223> b1463 b1464 U00096 1532048..1532893
<313> 2150: 768-787
<400> 2150
GCTGTGATGC AAGAACAGTT TGGTCTGGGC GTGGATGATG CGAAACATGG 50
CTTTACCGTG GATGAGTTAG CGCTGGTGAT GGCGGCGTTT GATACGCACC 100
<210> 2151
<211> 100
<212> DNA
<213> Escherichia coli K-12 MG1655
<220> b1463 b1464
<222> 654-753
<223> b1463 b1464 U00096 1532048..1532893
<313> 2151: 670-689
<400> 2151
CAATTTTCAT TTTACCCATT ATGAAAATGG GCACGCGGTG GAGCAGCGAA 50
ATCTACCGGA TGTGGCGTCA TTATATGCTG TGATGCAAGA ACAGTTTGGT 100
<210> 2152
<211> 100
<212> DNA
<213> Escherichia coli K-12 MG1655
<220> yaho b0330
<222> 176-275
<223> yaho b0330 U00096 345708..345983
<313> 2152: 224-243
<400> 2152
TCAAAAAAGC GGATGAAAAA GGGGCTGATG TGTTGGTACT GACCTCCGGT 50
CAAACTGACA ATAAGATCCA CGGCACGGCA AATATTTATA AGAAGAAGTA 100
<210> 2153
<211> 100
<212> DNA
<213> Escherichia coli K-12 MG1655
<220> yaho b0330
<222> 177-276
<223> yaho b0330 U00096 345708..345983
<313> 2153: 215-234
<400> 2153
CAAAAAAGCG GATGAAAAAG GGGCTGATGT GTTGGTACTG ACCTCCGGTC 50
AAACTGACAA TAAGATCCAC GGCACGGCAA ATATTTATAA GAAGAAGTAA 100
<210> 2154
<211> 100
<212> DNA
<213> Escherichia coli K-12 MG1655
<220> yaho b0330
<222> 87-186
<223> yaho b0330 U00096 345708..345983
<313> 2154: 106-125
<400> 2154
ATTTGAAAAA GTTGAATCGC AGTATGAAAA AATAGGTGAT ATTTCAACCA 50
GCAATGAAAT GTCGACTGCA GATGCAAAAG AAGATTTGAT CAAAAAAGCG 100
<210> 2155
<211> 100
<212> DNA
<213> Escherichia coli K-12 MG1655
<220> thil b0418
<222> 879-978
<223> thil b0418 U00096 434858..435835
<313> 2155: 956-975
<400> 2155
CGGGCAAATG ACCGCCGATA TCGAAGGGCT TGTTTTTATT CGTGACGGCG 50
AACCTGTTAC ATTAGACTGG AAAGGATATG ACCATTTTGC CACGCCATAA 100
<210> 2156
<211> 100
<212> DNA
<213> Escherichia coli K-12 MG1655
<220> thil b0418

<222> 866-965
<223> thil b0418 U00096 434858..435835
<313> 2156: 913-932
<400> 2156
CGTTTACCTG TATCGGGCAA ATGACCGCCG ATATCGAAGG GCTTTGTTTT  50
ATTCGTGACG GCGAACCTGT TACATTAGAC TGGAAAGGAT ATGACCATTT  100
<210> 2157
<211> 100
<212> DNA
<213> Escherichia coli K-12 MG1655
<220> thil b0418
<222> 823-922
<223> thil b0418 U00096 434858..435835
<313> 2157: 860-879
<400> 2157
AACCGTGGCG CGCTGGATGT GGCTCTCGGA CACCTGGGCG TACCGTTTAC  50
CTGTATCGGG CAAATGACCG CCGATATCGA AGGGCTTTGT TTTATTCGTG  100
<210> 2158
<211> 100
<212> DNA
<213> Escherichia coli K-12 MG1655
<220> thim b2105
<222> 690-789
<223> thim b2105 U00096 complement(2182533..2183321)
<313> 2158: 760-779
<400> 2158
AGCCGGAGAA CGCGCAGTCG CCAGAAGCGA GGGGCCAGGC AGTTTTGTTC  50
CACATTTCCT TGATGCGCTC TGGCAATTGA CGCAGGAGGT GCAGGCATGA  100
<210> 2159
<211> 100
<212> DNA
<213> Escherichia coli K-12 MG1655
<220> thim b2105
<222> 663-762
<223> thim b2105 U00096 complement(2182533..2183321)
<313> 2159: 729-748
<400> 2159
CGCATCTGCC TGTCACTGGA TGAAACAAGC CGGAGAACGC GCAGTCGCCA  50
GAAGCGAGGG GCCAGGCAGT TTTGTTCCAC ATTTCCTTGA TGCGCTCTGG  100
<210> 2160
<211> 100
<212> DNA
<213> Escherichia coli K-12 MG1655
<220> thim b2105
<222> 645-744
<223> thim b2105 U00096 complement(2182533..2183321)
<313> 2160: 685-704
<400> 2160
CGATACGCTG GAAAATGTCG CATCTGCCTG TCACTGGATG AAACAAGCCG  50
GAGAACGCGC AGTCGCCAGA AGCGAGGGGC CAGGCAGTTT TGTTCCACAT  100
<210> 2161
<211> 100
<212> DNA
<213> Escherichia coli K-12 MG1655
<220> ypja ypjA
<222> 4611-4710
<223> ypja ypjA U00096 complement(2776167..2780876)
<313> 2161: 4668-4687
<400> 2161
CAGCAGTACG TTAAGCGGGC ATCTTGGGGT GGGGTATAGC CATAGTGCCG  50
GTGTGGAATC CCCGTGGAAC GCGGTAGCTG GTGTGAACTG GTCGTTCTGA  100
<210> 2162
<211> 100
<212> DNA
<213> Escherichia coli K-12 MG1655
<220> ypja ypjA
<222> 4574-4673
<223> ypja ypjA U00096 complement(2776167..2780876)
<313> 2162: 4615-4634
<400> 2162
GACGTGGAAT ATACCAGGCA GGTATTAAAG CCTCATTCAG CAGTACGTTA  50

AGCGGGCATC TTGGGGTGGG GTATAGCCAT AGTGCCGGTG TGGAATCCCC 100
<210> 2163
<211> 100
<212> DNA
<213> Escherichia coli K-12 MG1655
<220> ypja ypjA
<222> 4512-4611
<223> ypja ypjA U00096 complement(2776167..2780876)
<313> 2163: 4563-4582
<400> 2163
TGTCGATGAT AACCGGGTGA AAGTGAATAG TGACGGTAAT TTCGTCAATT 50
ATTTGTCGGG CAGACGTGGA ATATACCAGG CAGGTATTAA AGCCTCATTC 100
<210> 2164
<211> 100
<212> DNA
<213> Escherichia coli K-12 MG1655
<220> ypje b2613
<222> 492-591
<223> ypje b2613 U00096 2746818..2747408
<313> 2164: 565-584
<400> 2164
AGAGTTGCGC ACTATCGTGC ACGAATCACG CTCACAAATT TCCCGTCGCA 50
ATCAGGATAT GCTGCTGTCG GTGCTCGATC CTGGAAAAAA TGACCGTTGA 100
<210> 2165
<211> 100
<212> DNA
<213> Escherichia coli K-12 MG1655
<220> ypje b2613
<222> 471-570
<223> ypje b2613 U00096 2746818..2747408
<313> 2165: 526-545
<400> 2165
TAGCGGCTCT TTGAGCAAAG AAGAGTTGCG CACTATCGTG CACGAATCAC 50
GCTCACAAAT TTCCCGTCGC AATCAGGATA TGCTGCTGTC GGTGCTCGAT 100
<210> 2166
<211> 100
<212> DNA
<213> Escherichia coli K-12 MG1655
<220> ypje b2613
<222> 356-455
<223> ypje b2613 U00096 2746818..2747408
<313> 2166: 415-434
<400> 2166
CGAGTAGTTT TCTGCTGGCT CCGCTGCAAA TTTTGATGAT GCCGCTGGTC 50
TGGTTGCTGA ATGCTATCAC CCGTATGCTG ATGCGCATGA TGGGTATCAA 100
<210> 2167
<211> 100
<212> DNA
<213> Escherichia coli K-12 MG1655
<220> ypjf b2647
<222> 231-330
<223> ypjf b2647 U00096 2775474..2775803
<313> 2167: 301-320
<400> 2167
CAGAGGATTT TCGTGGCAAC AACAGTCTCC ATATATTTCC GTAGTAGATA 50
TTCTGCGAGC AAGGCGCTCT ACCGGCTTGC TAAAAACTAA TGTGAAATAA 100
<210> 2168
<211> 100
<212> DNA
<213> Escherichia coli K-12 MG1655
<220> ypjf b2647
<222> 128-227
<223> ypjf b2647 U00096 2775474..2775803
<313> 2168: 181-200
<400> 2168
TCAGTGATGA AACTGTTATT AAGGAACATA TCGATGCTGG TATCACTCTG 50
GCCGATGCAG TCAATTTTCT GGTGGAAAAG TACGAACTGG TACGTATCGA 100
<210> 2169
<211> 100
<212> DNA
<213> Escherichia coli K-12 MG1655

<220> ypjf b2647
<222> 50-149
<223> ypjf b2647 U00096 2775474..2775803
<313> 2169: 88-107
<400> 2169
CGCCAGTGGC TGTCTGGCAA ATGCTACTGA CACGCCTGCT GGAACAACAC 50
TATGGCCTGA CACTGAACGA CACGCCGTTC AGTGATGAAA CTGTTATTAA 100
<210> 2170
<211> 100
<212> DNA
<213> Escherichia coli K-12 MG1655
<220> aroa b0909
<222> 1174-1273
<223> aroa b0909 U00096 958035..959318
<313> 2170: 1218-1237
<400> 2170
TCGCTGGTGG CGTTGTCAGA TACACCAGTG ACGATTCTTG ATCCCAAATG 50
CACGGCCAAA ACATTTCCGG ATTATTTCGA GCAGCTGGCG CGGATTAGCC 100
<210> 2171
<211> 100
<212> DNA
<213> Escherichia coli K-12 MG1655
<220> aroa b0909
<222> 1123-1222
<223> aroa b0909 U00096 958035..959318
<313> 2171: 1155-1174
<400> 2171
AACTTTGCCG AGATCGCGAC ATACAATGAT CACCGGATGG CGATGTGTTT 50
CTCGCTGGTG GCGTTGTCAG ATACACCAGT GACGATTCTT GATCCCAAAT 100
<210> 2172
<211> 100
<212> DNA
<213> Escherichia coli K-12 MG1655
<220> aroa b0909
<222> 1034-1133
<223> aroa b0909 U00096 958035..959318
<313> 2172: 1077-1096
<400> 2172
TGTTTGCGAT GGCAACAGAA CTGCGTAAAG TCGGCGCGGA AGTGGAAGAG 50
GGGCACGATT ACATTCGTAT CACTCCTCCG GAAAAACTGA ACTTTGCCGA 100
<210> 2173
<211> 100
<212> DNA
<213> Escherichia coli K-12 MG1655
<220> yaia b0390
<222> 93-192
<223> yaia b0390 U00096 406203..406394
<313> 2173: 158-177
<400> 2173
AGCCGATCAT CCTAAACCAG ACTCGTTGAT CAGTGAACAT CCGACCGCTC 50
AGGAAGCGAT GGATGCGAAA AAACGCTATG AGGACCCTGA CAAAGAGTGA 100
<210> 2174
<211> 100
<212> DNA
<213> Escherichia coli K-12 MG1655
<220> yaia b0390
<222> 44-143
<223> yaia b0390 U00096 406203..406394
<313> 2174: 88-107
<400> 2174
CGATTGAAAA AGGAAAGCCA GGACAGACGG TAACCTGGTA CCAACTCAGA 50
GCCGATCATC CTAAACCAGA CTCGTTGATC AGTGAACATC CGACCGCTCA 100
<210> 2175
<211> 100
<212> DNA
<213> Escherichia coli K-12 MG1655
<220> yaia b0390
<222> 22-121
<223> yaia b0390 U00096 406203..406394
<313> 2175: 54-73
<400> 2175

CCTCGTGAAG CATATATAGT GACGATTGAA AAAGGAAAGC CAGGACAGAC 50
GGTAACCTGG TACCAACTCA GAGCCGATCA TCCTAAACCA GACTCGTTGA 100
<210> 2176
<211> 100
<212> DNA
<213> Escherichia coli K-12 MG1655
<220> arob b3390
<222> 990-1089
<223> arob b3390 U00096 complement(3515036..3516124)
<313> 2176: 1061-1080
<400> 2176
GCGCTTAATT CTTCCGTTGG CAATTGGTAA GAGTGAAGTT CGCAGCGGCG 50
TTTCGCACGA GCTTGTTCTT AACGCCATTG CCGATTGTCA ATCAGCGTAA 100
<210> 2177
<211> 100
<212> DNA
<213> Escherichia coli K-12 MG1655
<220> arob b3390
<222> 985-1084
<223> arob b3390 U00096 complement(3515036..3516124)
<313> 2177: 1025-1044
<400> 2177
GAGATGCGCT TAATTCTTCC GTTGGCAATT GGTAAGAGTG AAGTTCGCAG 50
CGGCGTTTCG CACGAGCTTG TTCTTAACGC CATTGCCGAT TGTCAATCAG 100
<210> 2178
<211> 100
<212> DNA
<213> Escherichia coli K-12 MG1655
<220> arob b3390
<222> 825-924
<223> arob b3390 U00096 complement(3515036..3516124)
<313> 2178: 870-889
<400> 2178
GGCGCGGACG TCGGAACGTC TCGGGCAGTT TAGTTCTGCC GAAACGCAGC 50
GTATTATAAC CCTGCTCAAG CGGGCTGGGT TACCGGTCAA TGGGCCGCGC 100
<210> 2179
<211> 100
<212> DNA
<213> Escherichia coli K-12 MG1655
<220> yaib b0383
<222> 161-260
<223> yaib b0383 U00096 400610..400870
<313> 2179: 193-212
<400> 2179
GGGCGCTGTA CGAGGTAAAG CCCGATGCCA GCATTCCTGA CGACGATACG 50
GAGCTGCTGC GCGATTACGT AAAGAAGTTA TTGAAGCATC CTCGTCAGTA 100
<210> 2180
<211> 100
<212> DNA
<213> Escherichia coli K-12 MG1655
<220> yaib b0383
<222> 104-203
<223> yaib b0383 U00096 400610..400870
<313> 2180: 169-188
<400> 2180
CAATGCTTCG CAATATGGCG CAAAATGACC AACAGCGGTT GATTGATCAG 50
GTAGAGGGGG CGCTGTACGA GGTAAAGCCC GATGCCAGCA TTCCTGACGA 100
<210> 2181
<211> 100
<212> DNA
<213> Escherichia coli K-12 MG1655
<220> yaib b0383
<222> 89-188
<223> yaib b0383 U00096 400610..400870
<313> 2181: 111-130
<400> 2181
AGATTATCGT CACTGCAATG CTTCGCAATA TGGCGCAAAA TGACCAACAG 50
CGGTTGATTG ATCAGGTAGA GGGGGCGCTG TACGAGGTAA AGCCCGATGC 100
<210> 2182
<211> 100
<212> DNA

<213> Escherichia coli K-12 MG1655
<220> aroc b2330
<222> 987-1086
<223> aroc b2330 U00096 complement(2444408..2445493)
<313> 2182: 1059-1078
<400> 2182
GCCGATCGCA GAAGCGATGC TGGCGATCGT TTTAATGGAT CACCTGTTAC 50
GGCAACGGGC GCAAAATGCC GATGTGAAGA CTGATATTCC ACGCTGGTAA 100
<210> 2183
<211> 100
<212> DNA
<213> Escherichia coli K-12 MG1655
<220> aroc b2330
<222> 912-1011
<223> aroc b2330 U00096 complement(2444408..2445493)
<313> 2183: 939-958
<400> 2183
CATTAACCGC TTTGGCGAAG AAGTTGAGAT GATCACCAAA GGCCGTCACG 50
ATCCCTGTGT CGGGATCCGC GCAGTGCCGA TCGCAGAAGC GATGCTGGCG 100
<210> 2184
<211> 100
<212> DNA
<213> Escherichia coli K-12 MG1655
<220> aroc b2330
<222> 854-953
<223> aroc b2330 U00096 complement(2444408..2445493)
<313> 2184: 903-922
<400> 2184
AAATCATTGC CCATATGGCG CTGAAACCGA CCTCCAGCAT TACCGTGCCG 50
GGTCGTACCA TTAACCGCTT TGGCGAAGAA GTTGAGATGA TCACCAAAGG 100
<210> 2185
<211> 100
<212> DNA
<213> Escherichia coli K-12 MG1655
<220> yaic b0386
<222> 1017-1116
<223> yaic b0386 U00096 402927..404042
<313> 2185: 1086-1105
<400> 2185
CCCACAAATG AGTCACTATC GTGAGTGGTT GAAATCGGCA GATTTGGCGC 50
TTTACAAAGC AAAGAAAGCC GGACGTAACC GCACCGAAGT GGCGGCCTGA 100
<210> 2186
<211> 100
<212> DNA
<213> Escherichia coli K-12 MG1655
<220> yaic b0386
<222> 975-1074
<223> yaic b0386 U00096 402927..404042
<313> 2186: 1030-1049
<400> 2186
ACAGGTAACT TTACGGATTA GTGTGGGGGT TGCGCCGCTG AACCCACAAA 50
TGAGTCACTA TCGTGAGTGG TTGAAATCGG CAGATTTGGC GCTTTACAAA 100
<210> 2187
<211> 100
<212> DNA
<213> Escherichia coli K-12 MG1655
<220> yaic b0386
<222> 955-1054
<223> yaic b0386 U00096 402927..404042
<313> 2187: 981-1000
<400> 2187
TTACGTTTGC CGAATACGCC ACAGGTAACT TTACGGATTA GTGTGGGGGT 50
TGCGCCGCTG AACCCACAAA TGAGTCACTA TCGTGAGTGG TTGAAATCGG 100
<210> 2188
<211> 100
<212> DNA
<213> Escherichia coli K-12 MG1655
<220> arod b1694
<222> 660-759
<223> arod b1694 U00096 1772710..1773468
<313> 2188: 698-717

<400> 2188
CTCGGCGGCA ACTTTTGGTG CGGTAAAAAA AGCGTCTGCG CCAGGGCAAA 50
TCTCGGTAAA TGATTTGCGC ACGGTATTAA CTATTTTACA CCAGGCATAA 100
<210> 2189
<211> 100
<212> DNA
<213> Escherichia coli K-12 MG1655
<220> arod b1694
<222> 629-728
<223> arod b1694 U00096 1772710..1773468
<313> 2189: 655-674
<400> 2189
TAATTTCTCG TCTGGCTGGT GAAGTATTTG GCTCGGCGGC AACTTTTGGT 50
GCGGTAAAAA AAGCGTCTGC GCCAGGGCAA ATCTCGGTAA ATGATTTGCG 100
<210> 2190
<211> 100
<212> DNA
<213> Escherichia coli K-12 MG1655
<220> arod b1694
<222> 518-617
<223> arod b1694 U00096 1772710..1773468
<313> 2190: 560-579
<400> 2190
TGATGCCGCA AAGTACCAGC GATGTGCTGA CGTTGCTTGC CGCGACCCTG 50
GAGATGCAGG AGCAGTATGC CGATCGTCCA ATTATCACGA TGTCGATGGC 100
<210> 2191
<211> 100
<212> DNA
<213> Escherichia coli K-12 MG1655
<220> yaid b0394
<222> 813-912
<223> yaid b0394 U00096 complement(408332..409243)
<313> 2191: 853-872
<400> 2191
CGCCCAGCGT TTTGATGCCG ATTTCATCCT GATGACTGGT GAACTGGCAG 50
CGTTAATTCA AAACCTGATT GAAGGATTAG GTGGCGAAGC ACAACGTTAA 100
<210> 2192
<211> 100
<212> DNA
<213> Escherichia coli K-12 MG1655
<220> yaid b0394
<222> 786-885
<223> yaid b0394 U00096 complement(408332..409243)
<313> 2192: 814-833
<400> 2192
AAACGAAGAT ATCGACCGTG AAGATTTCGC CCAGCGTTTT GATGCCGATT 50
TCATCCTGAT GACTGGTGAA CTGGCAGCGT TAATTCAAAA CCTGATTGAA 100
<210> 2193
<211> 100
<212> DNA
<213> Escherichia coli K-12 MG1655
<220> yaid b0394
<222> 747-846
<223> yaid b0394 U00096 complement(408332..409243)
<313> 2193: 788-807
<400> 2193
GCTCAAGCGT CTGAAGTTCT GCGACGAGCT GCGCGATCAA AACGAAGATA 50
TCGACCGTGA AGATTTCGCC CAGCGTTTTG ATGCCGATTT CATCCTGATG 100
<210> 2194
<211> 100
<212> DNA
<213> Escherichia coli K-12 MG1655
<220> aroe b3282
<222> 691-790
<223> aroe b3282 U00096 complement(3427657..3428475)
<313> 2194: 757-776
<400> 2194
TCAAAGCGTA ATGCTGATGG TTTAGGAATG CTGGTGGCAC AGGCGGCTCA 50
TGCCTTTCTT CTCTGGCACG GTGTTCTGCC TGACGTAGAA CCAGTTATAA 100
<210> 2195
<211> 100

<212> DNA
<213> Escherichia coli K-12 MG1655
<220> aroe b3282
<222> 671-770
<223> aroe b3282 U00096 complement(3427657..3428475)
<313> 2195: 703-722
<400> 2195
CATGGTGTGA GCAGCGAGGC TCAAAGCGTA ATGCTGATGG TTTAGGAATG 50
CTGGTGGCAC AGGCGGCTCA TGCCTTTCTT CTCTGGCACG GTGTTCTGCC 100
<210> 2196
<211> 100
<212> DNA
<213> Escherichia coli K-12 MG1655
<220> aroe b3282
<222> 635-734
<223> aroe b3282 U00096 complement(3427657..3428475)
<313> 2196: 658-677
<400> 2196
ACATGTTCTA TCAGAAAGGA AAAACTCCTT TTCTGGCATG GTGTGAGCAG 50
CGAGGCTCAA AGCGTAATGC TGATGGTTTA GGAATGCTGG TGGCACAGGC 100
<210> 2197
<211> 100
<212> DNA
<213> Escherichia coli K-12 MG1655
<220> yaie yaiE
<222> 186-285
<223> yaie yaiE U00096 407401..407685
<313> 2197: 248-267
<400> 2197
GCAGGTGTAT GAAGCCGGTT CGGTGTTTAA TGTTCCCGGT CACAGTGAGT 50
TTCATCTGCA AGTTGCCGAA CCCACCTCTT ATCTGTGCCG CTATCTGTAA 100
<210> 2198
<211> 100
<212> DNA
<213> Escherichia coli K-12 MG1655
<220> yaie yaiE
<222> 173-272
<223> yaie yaiE U00096 407401..407685
<313> 2198: 210-229
<400> 2198
ACGCGACCGA CTGGCAGGTG TATGAAGCCG GTTCGGTGTT TAATGTTCCC 50
GGTCACAGTG AGTTTCATCT GCAAGTTGCC GAACCCACCT CTTATCTGTG 100
<210> 2199
<211> 100
<212> DNA
<213> Escherichia coli K-12 MG1655
<220> yaie yaiE
<222> 134-233
<223> yaie yaiE U00096 407401..407685
<313> 2199: 162-181
<400> 2199
TGACGGTAAT CAGTGGCGCG CTGAATGTGT TACTGCCTGA CGCGACCGAC 50
TGGCAGGTGT ATGAAGCCGG TTCGGTGTTT AATGTTCCCG GTCACAGTGA 100
<210> 2200
<211> 100
<212> DNA
<213> Escherichia coli K-12 MG1655
<220> arof b2602
<222> 972-1071
<223> arof b2602 U00096 complement(2738100..2739170)
<313> 2200: 1018-1037
<400> 2200
ATCCGTAACC GATGCCTGCA TTAGCTGGGA AATGACCGAT GCCTTGCTGC 50
GTGAAATTCA TCAGGATCTG AACGGGCAGC TGACGGCTCG CGTGGCTTAA 100
<210> 2201
<211> 100
<212> DNA
<213> Escherichia coli K-12 MG1655
<220> arof b2602
<222> 944-1043
<223> arof b2602 U00096 complement(2738100..2739170)

<313> 2201: 979-998
<400> 2201
AACCGCGCAG TGAAATGAAA TACGGTGTAT CCGTAACCGA TGCCTGCATT 50
AGCTGGGAAA TGACCGATGC CTTGCTGCGT GAAATTCATC AGGATCTGAA 100
<210> 2202
<211> 100
<212> DNA
<213> Escherichia coli K-12 MG1655
<220> arof b2602
<222> 884-983
<223> arof b2602 U00096 complement(2738100..2739170)
<313> 2202: 939-958
<400> 2202
GCTCAATTAT TGGTCTGATG ATCGAAAGTA ATATCCACGA GGGCAATCAG 50
TCTTCCGAGC AACCGCGCAG TGAAATGAAA TACGGTGTAT CCGTAACCGA 100
<210> 2203
<211> 100
<212> DNA
<213> Escherichia coli K-12 MG1655
<220> arog b0755
<222> 954-1053
<223> arog b0755 U00096 784856..785908
<313> 2203: 1024-1043
<400> 2203
GGCCTACGGT AAGAGCATCA CCGATGCCTG CATCGGCTGG GAAGATACCG 50
ATGCTCTGTT ACGTCAACTG GCGAATGCAG TAAAAGCGCG TCGCGGGTAA 100
<210> 2204
<211> 100
<212> DNA
<213> Escherichia coli K-12 MG1655
<220> arog b0755
<222> 900-999
<223> arog b0755 U00096 784856..785908
<313> 2204: 959-978
<400> 2204
GGTGGAAAGC CATCTGGTGG AAGGCAATCA GAGCCTCGAG AGCGGGGAGC 50
CGCTGGCCTA CGGTAAGAGC ATCACCGATG CCTGCATCGG CTGGGAAGAT 100
<210> 2205
<211> 100
<212> DNA
<213> Escherichia coli K-12 MG1655
<220> arog b0755
<222> 876-975
<223> arog b0755 U00096 784856..785908
<313> 2205: 899-918
<400> 2205
CGAAAAGGCC ATTATTGGCG TGATGGTGGA AAGCCATCTG GTGGAAGGCA 50
ATCAGAGCCT CGAGAGCGGG GAGCCGCTGG CCTACGGTAA GAGCATCACC 100
<210> 2206
<211> 100
<212> DNA
<213> Escherichia coli K-12 MG1655
<220> aroh b1705
<222> 948-1047
<223> aroh b1705 U00096 1786459..1787505
<313> 2206: 1023-1042
<400> 2206
GCTCACTTAC GGTCAATCCA TTACCGACCC GTGTCTGGGC TGGGAGGATA 50
CCGAACGCCT GGTCGAAAAA CTCGCCTCTG CGGTAGATAC CCGCTTCTGA 100
<210> 2207
<211> 100
<212> DNA
<213> Escherichia coli K-12 MG1655
<220> aroh b1705
<222> 901-1000
<223> aroh b1705 U00096 1786459..1787505
<313> 2207: 966-985
<400> 2207
GAAAGTTTCC TGCGCGAAGG AACGCAAAAA ATCGTCGGCA GTCAGCCGCT 50
CACTTACGGT CAATCCATTA CCGACCCGTG TCTGGGCTGG GAGGATACCG 100
<210> 2208

<211> 100
<212> DNA
<213> Escherichia coli K-12 MG1655
<220> aroh b1705
<222> 896-995
<223> aroh b1705 U00096 1786459..1787505
<313> 2208: 925-944
<400> 2208
TGGCGGAAAG TTTCCTGCGC GAAGGAACGC AAAAAATCGT CGGCAGTCAG 50
CCGCTCACTT ACGGTCAATC CATTACCGAC CCGTGTCTGG GCTGGGAGGA 100
<210> 2209
<211> 100
<212> DNA
<213> Escherichia coli K-12 MG1655
<220> yaih b0377
<222> 1053-1152
<223> yaih b0377 U00096 complement(394354..395511)
<313> 2209: 1108-1127
<400> 2209
GGTCACTCGT TCGCCGTTGA CGCGCTTTAA AAATATGAGC GATGGCATCA 50
ATGACCTGGT GACCGAGCTA AGCGGGAATA AACCGTTGGT TATCCCCGCG 100
<210> 2210
<211> 100
<212> DNA
<213> Escherichia coli K-12 MG1655
<220> yaih b0377
<222> 947-1046
<223> yaih b0377 U00096 complement(394354..395511)
<313> 2210: 969-988
<400> 2210
CCCCGAAAGA GGGTCGTCCG GGGATTATTC AGAAGACAGG CGGTGGTGGC 50
GGTTTCATTA CCTATATGGC GATGATCCCG CAGAAGAATA TTGGCGCGTT 100
<210> 2211
<211> 100
<212> DNA
<213> Escherichia coli K-12 MG1655
<220> yaih b0377
<222> 777-876
<223> yaih b0377 U00096 complement(394354..395511)
<313> 2211: 825-844
<400> 2211
CGATATGATG CGCTGGATGC AGCAGTATTT GTCATCTGAT TTTTATCAGC 50
GCAGTAACCA GGCAGATCGC ATGCAGACGT TGATTTATCA GCGCGCGCAG 100
<210> 2212
<211> 100
<212> DNA
<213> Escherichia coli K-12 MG1655
<220> yaii b0388
<222> 480-579
<223> yaii b0388 U00096 404868..405446
<313> 2212: 536-555
<400> 2212
CCAGACCGGC GGACCAGATA GCCTTTCACA ACGTGACCGC CAGGCCTTTG 50
CCGCGGAGCT GGAGAAGTGG TGGCTGGAAG TGCAACGTAG TCGTGGCTAA 100
<210> 2213
<211> 100
<212> DNA
<213> Escherichia coli K-12 MG1655
<220> yaii b0388
<222> 464-563
<223> yaii b0388 U00096 404868..405446
<313> 2213: 481-500
<400> 2213
TACGTGCCAG TGGGATCCAG ACCGGCGGAC CAGATAGCCT TTCACAACGT 50
GACCGCCAGG CCTTTGCCGC GGAGCTGGAG AAGTGGTGGC TGGAAGTGCA 100
<210> 2214
<211> 100
<212> DNA
<213> Escherichia coli K-12 MG1655
<220> yaii b0388
<222> 390-489

<223> yaii b0388 U00096 404868..405446
<313> 2214: 441-460
<400> 2214
TAATCCGCGC GGCGAACGTT ACACGCCAGC GACCATTCGT GAGCGCCTGA 50
CGATGCGCGA TTTTATGGAT ACCTTACGTG CCAGTGGGAT CCAGACCGGC 100
<210> 2215
<211> 100
<212> DNA
<213> Escherichia coli K-12 MG1655
<220> arok b3391
<222> 624-723
<223> arok b3391 U00096 complement(3516181..3516903)
<313> 2215: 691-710
<400> 2215
TCCGCTGTAT GAAGAGATTG CCGACGTGAC CATTCGTACT GATGATCAAA 50
GCGCTAAAGT GGTTGCAAAC CAGATTATTC ACATGCTGGA AAGCAACTAA 100
<210> 2216
<211> 100
<212> DNA
<213> Escherichia coli K-12 MG1655
<220> arok b3391
<222> 603-702
<223> arok b3391 U00096 complement(3516181..3516903)
<313> 2216: 649-668
<400> 2216
AGCGTTGGCC AATGAACGCA ATCCGCTGTA TGAAGAGATT GCCGACGTGA 50
CCATTCGTAC TGATGATCAA AGCGCTAAAG TGGTTGCAAA CCAGATTATT 100
<210> 2217
<211> 100
<212> DNA
<213> Escherichia coli K-12 MG1655
<220> arok b3391
<222> 522-621
<223> arok b3391 U00096 complement(3516181..3516903)
<313> 2217: 574-593
<400> 2217
CGAAAAGCAA CTTGCACGCA CGCAGCGTGA TAAAAAACGC CCGTTGCTGC 50
ACGTTGAAAC ACCGCCGCGT GAAGTTCTGG AAGCGTTGGC CAATGAACGC 100
<210> 2218
<211> 100
<212> DNA
<213> Escherichia coli K-12 MG1655
<220> arol b0389
<222> 426-525
<223> arol b0389 U00096 405629..406153
<313> 2218: 488-507
<400> 2218
ATATCGCGAA GTTGCGCATA TTATCATCGA CGCAACAAAC GAACCCAGCC 50
AGGTGATTTC TGAAATTCGC AGCGCCCTGG CACAGACGAT CAATTGTTGA 100
<210> 2219
<211> 100
<212> DNA
<213> Escherichia coli K-12 MG1655
<220> arol b0389
<222> 401-500
<223> arol b0389 U00096 405629..406153
<313> 2219: 455-474
<400> 2219
AAGTGCTGGA AGAACGCGAT GCGCTATATC GCGAAGTTGC GCATATTATC 50
ATCGACGCAA CAAACGAACC CAGCCAGGTG ATTTCTGAAA TTCGCAGCGC 100
<210> 2220
<211> 100
<212> DNA
<213> Escherichia coli K-12 MG1655
<220> arol b0389
<222> 346-445
<223> arol b0389 U00096 405629..406153
<313> 2220: 398-417
<400> 2220
GAAGAAGATT TACGGCCAAC CTTAACGGGA AAACCGCTGA GCGAAGAAGT 50
TCAGGAAGTG CTGGAAGAAC GCGATGCGCT ATATCGCGAA GTTGCGCATA 100

<210> 2221
<211> 100
<212> DNA
<213> Escherichia coli K-12 MG1655
<220> b1470 b1470
<222> 237-336
<223> b1470 b1470 U00096 complement(1542408..1542743)
<313> 2221: 300-319
<400> 2221
CAATACTTAC TGTAATAACG ATTATTACAA TCAGGCAATA AGAGAGGAAC 50
CGATAGACCT TCTGGACAGA TCGTTTTCCT CCAGTTCATG GCCTTTTTAG 100
<210> 2222
<211> 100
<212> DNA
<213> Escherichia coli K-12 MG1655
<220> b1470 b1470
<222> 237-336
<223> b1470 b1470 U00096 complement(1542408..1542743)
<313> 2222: 299-318
<400> 2222
CAATACTTAC TGTAATAACG ATTATTACAA TCAGGCAATA AGAGAGGAAC 50
CGATAGACCT TCTGGACAGA TCGTTTTCCT CCAGTTCATG GCCTTTTTAG 100
<210> 2223
<211> 100
<212> DNA
<213> Escherichia coli K-12 MG1655
<220> b1470 b1470
<222> 229-328
<223> b1470 b1470 U00096 complement(1542408..1542743)
<313> 2223: 285-304
<400> 2223
GAAATAAACA ATACTTACTG TAATAACGAT TATTACAATC AGGCAATAAG 50
AGAGGAACCG ATAGACCTTC TGGACAGATC GTTTTCCTCC AGTTCATGGC 100
<210> 2224
<211> 100
<212> DNA
<213> Escherichia coli K-12 MG1655
<220> yaiI b0355
<222> 558-657
<223> yaiI b0355 U00096 375879..376535
<313> 2224: 630-649
<400> 2224
TGCCAGCAGT ATAGTGTTGC ACAGCGCGCT CAGCGCCGAA GAGCAGGATG 50
AAGACGACCC GTATGCCGAC TTCAAAGTGC CTGATGATTT GATGTGGTAA 100
<210> 2225
<211> 100
<212> DNA
<213> Escherichia coli K-12 MG1655
<220> yaiI b0355
<222> 494-593
<223> yaiI b0355 U00096 375879..376535
<313> 2225: 543-562
<400> 2225
ACAATAGCGA AGGTGAATAC GCCATTATTC CCGCCAGCGT TGCCGATAAA 50
ATTGCTCAGC GCGATGCCAG CAGTATAGTG TTGCACAGCG CGCTCAGCGC 100
<210> 2226
<211> 100
<212> DNA
<213> Escherichia coli K-12 MG1655
<220> yaiI b0355
<222> 454-553
<223> yaiI b0355 U00096 375879..376535
<313> 2226: 511-530
<400> 2226
ATTAATGGTC GTCTGGCGAT TGCCCGCTTG TTGGTCGATA ACAATAGCGA 50
AGGTGAATAC GCCATTATTC CCGCCAGCGT TGCCGATAAA ATTGCTCAGC 100
<210> 2227
<211> 100
<212> DNA
<213> Escherichia coli K-12 MG1655
<220> aroM b0391

<222> 579-678
<223> arom b0391 U00096 406652..407329
<313> 2227: 632-651
<400> 2227
TCACCAACGT CATCGCGATT TACTGCAAAA ACAGCTCGAT GTTCCTGTCT 50
TGCTGTCTAA CGTATTGATT GCACGGCTGG CTGCGGAATT ACTGGTGTAA 100
<210> 2228
<211> 100
<212> DNA
<213> Escherichia coli K-12 MG1655
<220> arom b0391
<222> 511-610
<223> arom b0391 U00096 406652..407329
<313> 2228: 568-587
<400> 2228
ATCATTGATG CCGGGAAAGA ATTACTGGCA AAAGGGGCTG ATGTCATCAT 50
GCTGGATTGT TTAGGATTTC ACCAACGTCA TCGCGATTTA CTGCAAAAAC 100
<210> 2229
<211> 100
<212> DNA
<213> Escherichia coli K-12 MG1655
<220> arom b0391
<222> 435-534
<223> arom b0391 U00096 406652..407329
<313> 2229: 473-492
<400> 2229
GGCGCAAAAA TGGCAAATTT TGCAGAAATC GCCGGTATTT TCATTGGGTA 50
ACCCCATTCA TGATTCAGAA CAAAAAATCA TTGATGCCGG GAAAGAATTA 100
<210> 2230
<211> 100
<212> DNA
<213> Escherichia coli K-12 MG1655
<220> b1471 b1471
<222> 750-849
<223> b1471 b1471 U00096 complement(1542782..1543738)
<313> 2230: 791-810
<400> 2230
AATTAAAATA TTCTATAGCA ACTTCTGCAA TGTTCAGACA TTAAACGCTG 50
CTAACAACCA GTTGAAATAT ATAAATCTTG ATTTCCTGAC TTATCTTCCA 100
<210> 2231
<211> 100
<212> DNA
<213> Escherichia coli K-12 MG1655
<220> b1471 b1471
<222> 729-828
<223> b1471 b1471 U00096 complement(1542782..1543738)
<313> 2231: 786-805
<400> 2231
TAACAACAAT TTGCTTTCTG AAATTAAAAT ATTCTATAGC AACTTCTGCA 50
ATGTTCAGAC ATTAAACGCT GCTAACAACC AGTTGAAATA TATAAATCTT 100
<210> 2232
<211> 100
<212> DNA
<213> Escherichia coli K-12 MG1655
<220> b1471 b1471
<222> 720-819
<223> b1471 b1471 U00096 complement(1542782..1543738)
<313> 2232: 775-794
<400> 2232
GTTACTTATT AACAACAATT TGCTTTCTGA AATTAAAATA TTCTATAGCA 50
ACTTCTGCAA TGTTCAGACA TTAAACGCTG CTAACAACCA GTTGAAATAT 100
<210> 2233
<211> 100
<212> DNA
<213> Escherichia coli K-12 MG1655
<220> yaim b0356
<222> 735-834
<223> yaim b0356 U00096 complement(376759..377592)
<313> 2233: 778-797
<400> 2233
AATCCGTTAT CACGAGGGTT ATGATCACAG CTATTATTTT GTCTCCAGTT 50

TTATTGGCGA GCATATTGCC TACCACGCCA ATAAACTGAA TATGCGTTGA 100
<210> 2234
<211> 100
<212> DNA
<213> Escherichia coli K-12 MG1655
<220> yaim b0356
<222> 666-765
<223> yaim b0356 U00096 complement(376759..377592)
<313> 2234: 704-723
<400> 2234
TTTTTACGCA GAACAGCTGC GGACTCCAAA TCTTGAAAAG ATCTGCCAGG 50
AGATGAATAT CAAGACGTTA ATCCGTTATC ACGAGGGTTA TGATCACAGC 100
<210> 2235
<211> 100
<212> DNA
<213> Escherichia coli K-12 MG1655
<220> yaim b0356
<222> 562-661
<223> yaim b0356 U00096 complement(376759..377592)
<313> 2235: 607-626
<400> 2235
CTTGCTGAAA ATAAAGATGC CTGGTTGGAT TACGACCCGG TGAGTCTTAT 50
TTCACAAGGT CAACGCGTTG CGGAAATCAT GGTTGATCAG GGGTTGAGTG 100
<210> 2236
<211> 100
<212> DNA
<213> Escherichia coli K-12 MG1655
<220> b1472 b1473
<222> 192-291
<223> b1472 b1473 U00096 complement(1543762..1544052)
<313> 2236: 241-260
<400> 2236
AACGCAAATC AATGATCAAA TGATTGGTTT TGGTCACTGG GAATATGATT 50
TTAAAGGCTA TAACGATGAA GCCAACGGCT CGCGCGGCAA GAATCTCTGA 100
<210> 2237
<211> 100
<212> DNA
<213> Escherichia coli K-12 MG1655
<220> b1472 b1473
<222> 186-285
<223> b1472 b1473 U00096 complement(1543762..1544052)
<313> 2237: 243-262
<400> 2237
AGGAGAAACG CAAATCAATG ATCAAATGAT TGGTTTTGGT CACTGGGAAT 50
ATGATTTTAA AGGCTATAAC GATGAAGCCA ACGGCTCGCG CGGCAAGAAT 100
<210> 2238
<211> 100
<212> DNA
<213> Escherichia coli K-12 MG1655
<220> b1472 b1473
<222> 173-272
<223> b1472 b1473 U00096 complement(1543762..1544052)
<313> 2238: 229-248
<400> 2238
GTCTCGGCTT TAAAGGAGAA ACGCAAATCA ATGATCAAAT GATTGGTTTT 50
GGTCACTGGG AATATGATTT TAAAGGCTAT AACGATGAAG CCAACGGCTC 100
<210> 2239
<211> 100
<212> DNA
<213> Escherichia coli K-12 MG1655
<220> yain b0358
<222> 198-297
<223> yain b0358 U00096 complement(378830..379126)
<313> 2239: 232-251
<400> 2239
CCATATCCGG GAAACGTTTG ACCGAAATGA CTGCTACAGC CGCGAAGTCA 50
GCCAATCCGT TGACGACACT ATTGAACTGG TTCGTGCCTA TCTTAAATAG 100
<210> 2240
<211> 100
<212> DNA
<213> Escherichia coli K-12 MG1655

<220> yain b0358
<222> 122-221
<223> yain b0358 U00096 complement(378830..379126)
<313> 2240: 145-164
<400> 2240
AATGCCGTGC CATACTCCAA CAGATCGCTG CCGTTCGGGG CGCGGCTAAT  50
GGGCTGATGG CAGAAGTGCT TGAAAGCCAT ATCCGGGAAA CGTTTGACCG  100
<210> 2241
<211> 100
<212> DNA
<213> Escherichia coli K-12 MG1655
<220> yain b0358
<222> 57-156
<223> yain b0358 U00096 complement(378830..379126)
<313> 2241: 104-123
<400> 2241
TACTCGAGTT CGTCGTATTC GGGGGCAGAT TGATGCTCTG GAACGGTCGC  50
TGGAGGGTGA TGCCGAATGC CGTGCCATAC TCCAACAGAT CGCTGCCGTT  100
<210> 2242
<211> 100
<212> DNA
<213> Escherichia coli K-12 MG1655
<220> yaio yaiO
<222> 675-774
<223> yaio yaiO U00096 complement(379293..380066)
<313> 2242: 719-738
<400> 2242
ACTTAATCTC GGGCTGACGG CAGGTAAAGT GTGGTACGAC ACCCCAACCG  50
ATGATTTTAA CGGTCTGCAA CTTGCAGCCC GTCTGACCTG GAAATTCTGA  100
<210> 2243
<211> 100
<212> DNA
<213> Escherichia coli K-12 MG1655
<220> yaio yaiO
<222> 651-750
<223> yaio yaiO U00096 complement(379293..380066)
<313> 2243: 685-704
<400> 2243
ACGTATTCAA CCGCTGACTG AGCAACTTAA TCTCGGGCTG ACGGCAGGTA  50
AAGTGTGGTA CGACACCCCA ACCGATGATT TTAACGGTCT GCAACTTGCA  100
<210> 2244
<211> 100
<212> DNA
<213> Escherichia coli K-12 MG1655
<220> yaio yaiO
<222> 612-711
<223> yaio yaiO U00096 complement(379293..380066)
<313> 2244: 631-650
<400> 2244
AGAAACACGC TACGGCAGCA TGAAGAGCGT CAGTCTGCAA CGTATTCAAC  50
CGCTGACTGA GCAACTTAAT CTCGGGCTGA CGGCAGGTAA AGTGTGGTAC  100
<210> 2245
<211> 100
<212> DNA
<213> Escherichia coli K-12 MG1655
<220> arop b0113
<222> 1275-1374
<223> arop b0113 U00096 complement(120178..121551)
<313> 2245: 1350-1369
<400> 2245
AATGGCGATT TCGGTATACC TGATCCCGGT ATGGCTGATC GTGTTAGGTA  50
TCGGCTATCT GTTTAAAGAG AAAACCGCCA AAGCCGTAAA AGCGCATTAA  100
<210> 2246
<211> 100
<212> DNA
<213> Escherichia coli K-12 MG1655
<220> arop b0113
<222> 1234-1333
<223> arop b0113 U00096 complement(120178..121551)
<313> 2246: 1257-1276
<400> 2246

TTTATGGCGG CGGTACTGGT GATTATGCTG ATGACCCCAG GAATGGCGAT 50
TTCGGTATAC CTGATCCCGG TATGGCTGAT CGTGTTAGGT ATCGGCTATC 100
<210> 2247
<211> 100
<212> DNA
<213> Escherichia coli K-12 MG1655
<220> arop b0113
<222> 1154-1253
<223> arop b0113 U00096 complement(120178..121551)
<313> 2247: 1219-1238
<400> 2247
GTCGCGCCAA GCAGGAACAA GGCGTGGTAA CTCGCTTCCC TGCTCTGCTT 50
TATCCGCTGG GTAACTGGAT CTGCCTGCTG TTTATGGCGG CGGTACTGGT 100
<210> 2248
<211> 100
<212> DNA
<213> Escherichia coli K-12 MG1655
<220> yaip b0364
<222> 997-1096
<223> yaip b0364 U00096 complement(381963..383159)
<313> 2248: 1022-1041
<400> 2248
TTGTTGGTGC CTTTAGCGCC GCTTTCCGTT GTGTATGTAT TATTAGCTTA 50
TGCCATCTGG ATTATTTATG GACTTATTGC CTTTTTTACT GGACGCGAAC 100
<210> 2249
<211> 100
<212> DNA
<213> Escherichia coli K-12 MG1655
<220> yaip b0364
<222> 943-1042
<223> yaip b0364 U00096 complement(381963..383159)
<313> 2249: 989-1008
<400> 2249
GTCGGCGTAG TTTGTGTTAT TGGTGCTTTT AGCGCCTGGT TTCATCGTTG 50
CTGGTTGTTG GTGCCTTTAG CGCCGCTTTC CGTTGTGTAT GTATTATTAG 100
<210> 2250
<211> 100
<212> DNA
<213> Escherichia coli K-12 MG1655
<220> yaip b0364
<222> 916-1015
<223> yaip b0364 U00096 complement(381963..383159)
<313> 2250: 945-964
<400> 2250
GTGTTGGCAA TGTTTCCGCT TATCTGGGTC GGCGTAGTTT GTGTTATTGG 50
TGCTTTTAGC GCCTGGTTTC ATCGTTGCTG GTTGTTGGTG CCTTTAGCGC 100
<210> 2251
<211> 100
<212> DNA
<213> Escherichia coli K-12 MG1655
<220> acna b1277
<222> 2577-2676
<223> acna b1277 U00096 1333855..1336530
<313> 2251: 2616-2635
<400> 2251
AGTCGTACCC TGCCGTTGTC GTATCGACAC CGCGACGGAG TTGACCTACT 50
ACCAGAACGA CGGCATTTTG CATTATGTCA TTCGTAATAT GTTGAAGTAA 100
<210> 2252
<211> 100
<212> DNA
<213> Escherichia coli K-12 MG1655
<220> acna b1277
<222> 2554-2653
<223> acna b1277 U00096 1333855..1336530
<313> 2252: 2572-2591
<400> 2252
ACGCGCGCGG ATGGTAGCCA GGAAGTCGTA CCCTGCCGTT GTCGTATCGA 50
CACCGCGACG GAGTTGACCT ACTACCAGAA CGACGGCATT TTGCATTATG 100
<210> 2253
<211> 100
<212> DNA

<213> Escherichia coli K-12 MG1655
<220> acna b1277
<222> 2476-2575
<223> acna b1277 U00096 1333855..1336530
<313> 2253: 2532-2551
<400> 2253
GGGCTAACCG GGGAAGAGAA GATTGATATT GGCGATCTGC AAAACCTACA 50
ACCCGGCGCG ACGGTTCCGG TGACGCTTAC GCGCGCGGAT GGTAGCCAGG 100
<210> 2254
<211> 100
<212> DNA
<213> Escherichia coli K-12 MG1655
<220> acnb b0119
<222> 2499-2598
<223> acnb b0119 U00096 131615..134212
<313> 2254: 2573-2592
<400> 2254
AGATAAAACA GCCGTTGATA CTTACCGTTA TCTGAACTTC AACCAGCTTT 50
CTCAGTACAC CGAGAAAGCC GATGGGGTGA TTTTCCAGAC TGCGGTTTAA 100
<210> 2255
<211> 100
<212> DNA
<213> Escherichia coli K-12 MG1655
<220> acnb b0119
<222> 2447-2546
<223> acnb b0119 U00096 131615..134212
<313> 2255: 2507-2526
<400> 2255
TGATTGGCAA ACTGCCGACG CCGGAAGAGT ACCAGACCTA CGTGGCGCAG 50
GTAGATAAAA CAGCCGTTGA TACTTACCGT TATCTGAACT TCAACCAGCT 100
<210> 2256
<211> 100
<212> DNA
<213> Escherichia coli K-12 MG1655
<220> acnb b0119
<222> 2435-2534
<223> acnb b0119 U00096 131615..134212
<313> 2256: 2451-2470
<400> 2256
CTGTTGCGGC GCTGATTGGC AAACTGCCGA CGCCGGAAGA GTACCAGACC 50
TACGTGGCGC AGGTAGATAA AACAGCCGTT GATACTTACC GTTATCTGAA 100
<210> 2257
<211> 100
<212> DNA
<213> Escherichia coli K-12 MG1655
<220> yais b0365
<222> 266-365
<223> yais b0365 U00096 complement(383283..383693)
<313> 2257: 329-348
<400> 2257
CAATGGTTGC CTGCCGAACT ATTCCGCAAA TTCTCGGCTA CGAAACCCCA 50
AGTACCTGGC TTTCATTTAT GCCTCAGGTT TTTGAATCCG TTAAAGAAGA 100
<210> 2258
<211> 100
<212> DNA
<213> Escherichia coli K-12 MG1655
<220> yais b0365
<222> 222-321
<223> yais b0365 U00096 complement(383283..383693)
<313> 2258: 275-294
<400> 2258
TGATGCCGAC CGCCATCAGG ATCATCTTGC TGTTTATCAA GCTTCAATGG 50
TTGCCTGCCG AACTATTCCG CAAATTCTCG GCTACGAAAC CCCAAGTACC 100
<210> 2259
<211> 100
<212> DNA
<213> Escherichia coli K-12 MG1655
<220> yais b0365
<222> 197-296
<223> yais b0365 U00096 complement(383283..383693)
<313> 2259: 238-257

<400> 2259
AAATCATGCG GGTATATACC ATGCATGATG CCGACCGCCA TCAGGATCAT 50
CTTGCTGTTT ATCAAGCTTC AATGGTTGCC TGCCGAACTA TTCCGCAAAT 100
<210> 2260
<211> 100
<212> DNA
<213> Escherichia coli K-12 MG1655
<220> yait b0372
<222> 1362-1461
<223> yait b0372 U00096 389475..390935
<313> 2260: 1440-1459
<400> 2260
GAACATTGGT GAAGATGGCT ACGTTGATAC CGATCATCTG ACTATTAACT 50
CCTACAGTAC TGTTGCGTTG ACCGAATCTA CTGGGTGGGG GGCTGATTGA 100
<210> 2261
<211> 100
<212> DNA
<213> Escherichia coli K-12 MG1655
<220> yait b0372
<222> 1325-1424
<223> yait b0372 U00096 389475..390935
<313> 2261: 1369-1388
<400> 2261
CTTCAGATAC TATCGATTTA ACTAACGGTT CTTCGCTGAA CATTGGTGAA 50
GATGGCTACG TTGATACCGA TCATCTGACT ATTAACTCCT ACAGTACTGT 100
<210> 2262
<211> 100
<212> DNA
<213> Escherichia coli K-12 MG1655
<220> yait b0372
<222> 1232-1331
<223> yait b0372 U00096 389475..390935
<313> 2262: 1257-1276
<400> 2262
ACGGTTCTGA ATGGGATACT ACAGGTCGTT CCATCGTTGA TACCTTGACA 50
GTTAATAATG GTTCTCAGGT TAATGTTTCG GAATCTAAAT TAACTTCAGA 100
<210> 2263
<211> 100
<212> DNA
<213> Escherichia coli K-12 MG1655
<220> yaiu b0375
<222> 1305-1404
<223> yaiu b0375 U00096 392239..393642
<313> 2263: 1343-1362
<400> 2263
CACCAAGAAC TTCAGCGCCT ATACCGATGC TAACTACCTC GGTGGTGGTG 50
ACGTAGATCA AGACTGGTCC GCGAACGTGG GTGTTAAATA TACCTGGTAA 100
<210> 2264
<211> 100
<212> DNA
<213> Escherichia coli K-12 MG1655
<220> yaiu b0375
<222> 1178-1277
<223> yaiu b0375 U00096 392239..393642
<313> 2264: 1223-1242
<400> 2264
CTCCGTACTT CAAACTGGCT TACGTCTACG ACGACTCTAA CAACGATAAC 50
GATGTGAACG GCGATTCCAT CGATAACGGT ACTGAAGGGT CTGCGGTACG 100
<210> 2265
<211> 100
<212> DNA
<213> Escherichia coli K-12 MG1655
<220> yaiu b0375
<222> 1137-1236
<223> yaiu b0375 U00096 392239..393642
<313> 2265: 1183-1202
<400> 2265
TGCAGGTTAT ACCTTCACCT ACAGCGAAGA TCAGGCTCTG ACTCCGTACT 50
TCAAACTGGC TTACGTCTAC GACGACTCTA ACAACGATAA CGATGTGAAC 100
<210> 2266
<211> 100

<212> DNA
<213> Escherichia coli K-12 MG1655
<220> yaiv b0376
<222> 516-615
<223> yaiv b0376 U00096 393685..394353
<313> 2266: 581-600
<400> 2266
ATCACGTATT GGTGTGATGA ATTATATCCA TCAACGTACA CGCATATCGC 50
GTTCTGTCGT CGCAGAAGTT CTCGCTGCTT TGCGTAAAGG CGGCTATATC 100
<210> 2267
<211> 100
<212> DNA
<213> Escherichia coli K-12 MG1655
<220> yaiv b0376
<222> 400-499
<223> yaiv b0376 U00096 393685..394353
<313> 2267: 455-474
<400> 2267
TTAGCCTGGC AAAATAGAAT TCTGGAATTA CGCGACGTGC AGCTCATTGG 50
GCATAATTCC TACGAACAAA TCCGCGCAAC ATTATTATCA ATGATTGACT 100
<210> 2268
<211> 100
<212> DNA
<213> Escherichia coli K-12 MG1655
<220> yaiv b0376
<222> 323-422
<223> yaiv b0376 U00096 393685..394353
<313> 2268: 358-377
<400> 2268
CGGGATATCA TCTACCAGCC AAACAAACCA TTACGCTTAT TGAACAAAAT 50
CAACTCTGGC GAGACGCTTT TTACTGGTTA GCCTGGCAAA ATAGAATTCT 100
<210> 2269
<211> 100
<212> DNA
<213> Escherichia coli K-12 MG1655
<220> glf b2037
<222> 879-978
<223> glf b2037 U00096 complement(2105248..2106351)
<313> 2269: 922-941
<400> 2269
GGTTGTTACA AAAGAATATC CATTAGAGTG GAAAGTTGGC GACGAACCCT 50
ACTATCCAGT TAATGATAAT AAAAACATGG AGCTTTTTAA GAAATATAGA 100
<210> 2270
<211> 100
<212> DNA
<213> Escherichia coli K-12 MG1655
<220> glf b2037
<222> 747-846
<223> glf b2037 U00096 complement(2105248..2106351)
<313> 2270: 775-794
<400> 2270
AAAATTTGAG ACGGAACGCC ATGAATTTCC AAACTTCCAA GGGAATGCAG 50
TAATAAATTT CACTGATGCT AATGTACCAT ATACCAGAAT AATTGAGCAT 100
<210> 2271
<211> 100
<212> DNA
<213> Escherichia coli K-12 MG1655
<220> glf b2037
<222> 647-746
<223> glf b2037 U00096 complement(2105248..2106351)
<313> 2271: 680-699
<400> 2271
ACAAAGATTC TCTAGCGAGT AAAGCCCATA GAATCATCTA CACTGGACCC 50
ATTGATCAGT ACTTCGACTA TAGGTTTGGA GCGTTAGAAT ATCGCTCTTT 100
<210> 2272
<211> 100
<212> DNA
<213> Escherichia coli K-12 MG1655
<220> yaiw yaiW
<222> 972-1071
<223> yaiw yaiW U00096 397096..398190

<313> 2272: 1029-1048
<400> 2272
ATCACTCCCC CGCGAAATGT TGCCTGGCAT TCAACTGGAA AGCCCGAAAA 50
TCACCCGCAA CCTGACTACG GCCTGGTTTG CGAAGCGCGT AGACGAACGG 100
<210> 2273
<211> 100
<212> DNA
<213> Escherichia coli K-12 MG1655
<220> yaiw yaiW
<222> 924-1023
<223> yaiw yaiW U00096 397096..398190
<313> 2273: 975-994
<400> 2273
GGCGCTGTAC AAGAAAGTTT ATCAACTTGC CGAAACGAAA ACGGGTAAAT 50
CACTCCCCCG CGAAATGTTG CCTGGCATTC AACTGGAAAG CCCGAAAATC 100
<210> 2274
<211> 100
<212> DNA
<213> Escherichia coli K-12 MG1655
<220> yaiw yaiW
<222> 879-978
<223> yaiw yaiW U00096 397096..398190
<313> 2274: 944-963
<400> 2274
TCGCCGTCAG TTAGAGAAAG GTGACAGCTT CTCTTTTGAG GAGACGGCGC 50
TGTACAAGAA AGTTTATCAA CTTGCCGAAA CGAAAACGGG TAAATCACTC 100
<210> 2275
<211> 100
<212> DNA
<213> Escherichia coli K-12 MG1655
<220> ruva b1862
<222> 505-604
<223> ruva b1862 U00096 complement(1943389..1944000)
<313> 2275: 561-580
<400> 2275
GCGCTGGGCT ATAAACCACA AGAAGCAAGC CGCATGGTGA GCAAAATCGC 50
TCGCCCTGAC GCCAGCAGTG AAACTTTAAT TCGCGAAGCC CTACGCGCCG 100
<210> 2276
<211> 100
<212> DNA
<213> Escherichia coli K-12 MG1655
<220> ruva b1862
<222> 482-581
<223> ruva b1862 U00096 complement(1943389..1944000)
<313> 2276: 515-534
<400> 2276
AAGAAGCGGT TGCCGCGCTG GTGGCGCTGG GCTATAAACC ACAAGAAGCA 50
AGCCGCATGG TGAGCAAAAT CGCTCGCCCT GACGCCAGCA GTGAAACTTT 100
<210> 2277
<211> 100
<212> DNA
<213> Escherichia coli K-12 MG1655
<220> ruva b1862
<222> 438-537
<223> ruva b1862 U00096 complement(1943389..1944000)
<313> 2277: 459-478
<400> 2277
GGTACTCACG TCTCCTGCCA GCCCGGCGAC CGACGATGCT GAACAAGAAG 50
CGGTTGCCGC GCTGGTGGCG CTGGGCTATA AACCACAAGA AGCAAGCCGC 100
<210> 2278
<211> 100
<212> DNA
<213> Escherichia coli K-12 MG1655
<220> glk glk
<222> 867-966
<223> glk glk U00096 complement(2506481..2507446)
<313> 2278: 898-917
<400> 2278
ATATGTCCAT GATATTCCGG TGTATCTCAT CGTCCATGAC AATCCGGGCC 50
TTCTCGGTTC CGGTGCACAT TTACGCCAGA CCTTAGGTCA CATTCTGTAA 100
<210> 2279

<211> 100
<212> DNA
<213> Escherichia coli K-12 MG1655
<220> glk glk
<222> 777-876
<223> glk glk U00096 complement(2506481..2507446)
<313> 2279: 822-841
<400> 2279
TATTGCGGGC GGTATCGTGC CGCGCTTCCT TGAGTTCTTC AAAGCCTCCG 50
GTTTCCGTGC CGCATTTGAA GATAAAGGGC GCTTTAAAGA ATATGTCCAT 100
<210> 2280
<211> 100
<212> DNA
<213> Escherichia coli K-12 MG1655
<220> glk glk
<222> 672-771
<223> glk glk U00096 complement(2506481..2507446)
<313> 2280: 731-750
<400> 2280
TGACAGCTGC ACCGATTGCC GCCGCGCATT GTCGCTGTTT TGCGTCATTA 50
TGGGCCGTTT TGGCGGCAAT CTGGCGCTCA ATCTCGGGAC ATTTGGCGGC 100
<210> 2281
<211> 100
<212> DNA
<213> Escherichia coli K-12 MG1655
<220> ruvb b1861
<222> 912-1011
<223> ruvb b1861 U00096 complement(1942370..1943380)
<313> 2281: 987-1006
<400> 2281
TTTGATTCAG CAAGGCTTTT TGCAGCGTAC ACCGCGTGGG CGTATGGCGA 50
CGACGCGGGC GTGGAATCAC TTTGGCATAA CGCCGCCAGA AATGCCGTAA 100
<210> 2282
<211> 100
<212> DNA
<213> Escherichia coli K-12 MG1655
<220> ruvb b1861
<222> 841-940
<223> ruvb b1861 U00096 complement(1942370..1943380)
<313> 2282: 893-912
<400> 2282
GTAGGTCTGG ATAACCTGGC GGCAGCCATT GGCGAAGAAC GTGAAACCAT 50
TGAGGATGTG CTGGAACCTT ATTTGATTCA GCAAGGCTTT TTGCAGCGTA 100
<210> 2283
<211> 100
<212> DNA
<213> Escherichia coli K-12 MG1655
<220> ruvb b1861
<222> 730-829
<223> ruvb b1861 U00096 complement(1942370..1943380)
<313> 2283: 778-797
<400> 2283
GCAGATATCG CTGCTCAGGC GCTGGATATG TTGAATGTCG ATGCTGAAGG 50
TTTCGATTAT ATGGACCGCA AATTGTTGCT GGCGGTAATC GATAAGTTCT 100
<210> 2284
<211> 100
<212> DNA
<213> Escherichia coli K-12 MG1655
<220> ruvc b1864
<222> 423-522
<223> ruvc b1864 U00096 complement(1944879..1945400)
<313> 2284: 495-514
<400> 2284
CGATGCGCTG GCGATTGCTA TCACCCACTG CCACGTTAGT CAGAATGCGA 50
TGCAGATGAG CGAATCGCGG CTGAACCTGG CGAGAGGGCG ACTGCGTTAA 100
<210> 2285
<211> 100
<212> DNA
<213> Escherichia coli K-12 MG1655
<220> ruvc b1864
<222> 423-522

<223> ruvc b1864 U00096 complement(1944879..1945400)
<313> 2285: 463-482
<400> 2285
CGATGCGCTG GCGATTGCTA TCACCCACTG CCACGTTAGT CAGAATGCGA  50
TGCAGATGAG CGAATCGCGG CTGAACCTGG CGAGAGGGCG ACTGCGTTAA  100
<210> 2286
<211> 100
<212> DNA
<213> Escherichia coli K-12 MG1655
<220> ruvc b1864
<222> 363-462
<223> ruvc b1864 U00096 complement(1944879..1945400)
<313> 2286: 395-414
<400> 2286
GGTGCAGCAT ATGGTCCGCA CCTTGCTGAA ACTGCCCGCT AATCCACAGG  50
CGGATGCCGC CGATGCGCTG GCGATTGCTA TCACCCACTG CCACGTTAGT  100
<210> 2287
<211> 100
<212> DNA
<213> Escherichia coli K-12 MG1655
<220> yajb b0405
<222> 483-582
<223> yajb b0405 U00096 complement(423561..424142)
<313> 2287: 509-528
<400> 2287
CTCCTGGTAC GATTTAGACG CTCATTATGA CGCCCTCGAA ACCCGCTTCT  50
GGCAGTTTTA TCCGCGGATG ATGGCGCAGG CGTCACGCAA GGCGTTATAA  100
<210> 2288
<211> 100
<212> DNA
<213> Escherichia coli K-12 MG1655
<220> yajb b0405
<222> 443-542
<223> yajb b0405 U00096 complement(423561..424142)
<313> 2288: 484-503
<400> 2288
GCATGGCAAG CCGCCGCCCA CGTCTGGATG CCCTGCGTGA CTCCTGGTAC  50
GATTTAGACG CTCATTATGA CGCCCTCGAA ACCCGCTTCT GGCAGTTTTA  100
<210> 2289
<211> 100
<212> DNA
<213> Escherichia coli K-12 MG1655
<220> yajb b0405
<222> 350-449
<223> yajb b0405 U00096 complement(423561..424142)
<313> 2289: 378-397
<400> 2289
CGCCACGTTT TATCAATCTG AACAATTACT TGTGGTCAGA GCAGTGGCTG  50
GTGCGCTATC GCGATATGGA TTTCATCCAG AACGTGTTAA ACGGCATGGC  100
<210> 2290
<211> 100
<212> DNA
<213> Escherichia coli K-12 MG1655
<220> yajc b0408
<222> 234-333
<223> yajc b0408 U00096 426511..426843
<313> 2290: 312-331
<400> 2290
CGGCTACATT GCTATCGCGC TGAATGACAC CACTGAAGTA GTTATTAAAC  50
GTGACTTCGT AGCTGCCGTC CTGCCGAAAG GCACCATGAA GGCGCTGTAA  100
<210> 2291
<211> 100
<212> DNA
<213> Escherichia coli K-12 MG1655
<220> yajc b0408
<222> 194-293
<223> yajc b0408 U00096 426511..426843
<313> 2291: 255-274
<400> 2291
ACGGTGGCCT GGTTGGTCGC GTAACCAAAG TAGCGGAAAA CGGCTACATT  50
GCTATCGCGC TGAATGACAC CACTGAAGTA GTTATTAAAC GTGACTTCGT  100

<210> 2292
<211> 100
<212> DNA
<213> Escherichia coli K-12 MG1655
<220> yajc b0408
<222> 155-254
<223> yajc b0408 U00096 426511..426843
<313> 2292: 216-235
<400> 2292
TGATGGACTC CATTGCCAAA GGTGATGAAG TTCTGACGAA CGGTGGCCTG 50
GTTGGTCGCG TAACCAAAGT AGCGGAAAAC GGCTACATTG CTATCGCGCT 100
<210> 2293
<211> 100
<212> DNA
<213> Escherichia coli K-12 MG1655
<220> yajd b0411
<222> 249-348
<223> yajd b0411 U00096 429829..430176
<313> 2293: 314-333
<400> 2293
TACGACCGTT ATCGCAGGGG AAGATGCGCA GAAAGATGTC GGTGAAGCGA 50
AGTACAACCC ATTCGCTGAC CTGAAAGCGA TGATGAACAA GAAGAAGTGA 100
<210> 2294
<211> 100
<212> DNA
<213> Escherichia coli K-12 MG1655
<220> yajd b0411
<222> 226-325
<223> yajd b0411 U00096 429829..430176
<313> 2294: 245-264
<400> 2294
TATACCGAAG CGGATCAGTA TGGTACGACC GTTATCGCAG GGGAAGATGC 50
GCAGAAAGAT GTCGGTGAAG CGAAGTACAA CCCATTCGCT GACCTGAAAG 100
<210> 2295
<211> 100
<212> DNA
<213> Escherichia coli K-12 MG1655
<220> yajd b0411
<222> 132-231
<223> yajd b0411 U00096 429829..430176
<313> 2295: 188-207
<400> 2295
TCACCACATT GATCACGACC ATACCAATAA CCCGGAAGAT GGCAGTAACT 50
GGGAATTGTT GTGTCTCTAT TGCCACGATC ATGAGCATTC GAAATATACC 100
<210> 2296
<211> 100
<212> DNA
<213> Escherichia coli K-12 MG1655
<220> yajf yajF
<222> 894-993
<223> yajf yajF U00096 409230..410276
<313> 2296: 949-968
<400> 2296
CGGGATGAGC AATGTAGACC GTTTATATCA AACGGTTGGG CAGTTGATTA 50
AACAATTTGT CTTCGGCGGC GAATGTGAAA CGCCGGTGCG TAAGGCGAAG 100
<210> 2297
<211> 100
<212> DNA
<213> Escherichia coli K-12 MG1655
<220> yajf yajF
<222> 751-850
<223> yajf yajF U00096 409230..410276
<313> 2297: 791-810
<400> 2297
CTGAAAGGCA GTGAAATTAT CCGCCTGGTT GAAGAAAGCG ATCCGGTAGC 50
GGAACTGGCA TTGCGTCGCT ACGAGCTGCG GCTGGCAAAA TCGCTGGCAC 100
<210> 2298
<211> 100
<212> DNA
<213> Escherichia coli K-12 MG1655
<220> yajf yajF

<222> 696-795
<223> yajf yajF U00096 409230..410276
<313> 2298: 757-776
<400> 2298
CTTTATTTCG GGCACGGGAT TCGCGATGGA TTATCGTCGT TTGAGCGGAC 50
ATGCGCTGAA AGGCAGTGAA ATTATCCGCC TGGTTGAAGA AAGCGATCCG 100
<210> 2299
<211> 100
<212> DNA
<213> Escherichia coli K-12 MG1655
<220> yajg b0435
<222> 582-681
<223> yajg b0435 U00096 complement(452813..453493)
<313> 2299: 653-672
<400> 2299
AAATATCGCC GATGCGGTTA ACAGTGTGCT GACCGATACC ATCGCTGATA 50
TGTCTCAGGA CACCAGCATC CACGAATTCA TCAAGCAGAA CGCGCGTTAA 100
<210> 2300
<211> 100
<212> DNA
<213> Escherichia coli K-12 MG1655
<220> yajg b0435
<222> 526-625
<223> yajg b0435 U00096 complement(452813..453493)
<313> 2300: 583-602
<400> 2300
ACCAAAAACT ATCGTGCCAG CTACAACGTT GAAGGTGCGT TCCAGGCCTC 50
CAACAAAAAT ATCGCCGATG CGGTTAACAG TGTGCTGACC GATACCATCG 100
<210> 2301
<211> 100
<212> DNA
<213> Escherichia coli K-12 MG1655
<220> yajg b0435
<222> 498-597
<223> yajg b0435 U00096 complement(452813..453493)
<313> 2301: 523-542
<400> 2301
CGCTACCGCG CAGAATGGCA ACAAAATGAC CAAAAACTAT CGTGCCAGCT 50
ACAACGTTGA AGGTGCGTTC CAGGCCTCCA ACAAAAATAT CGCCGATGCG 100
<210> 2302
<211> 100
<212> DNA
<213> Escherichia coli K-12 MG1655
<220> yaji b0413
<222> 352-451
<223> yaji b0413 U00096 complement(431536..432135)
<313> 2302: 393-412
<400> 2302
ACCCTTACAC TACGTATTCA GGGTGAGTCG AATGATCCCT TGCCCGCATT 50
CAGCGGAACT GTTGAATATG GGCAAATTCA GGGAACAATA GACAACTTTC 100
<210> 2303
<211> 100
<212> DNA
<213> Escherichia coli K-12 MG1655
<220> yaji b0413
<222> 295-394
<223> yaji b0413 U00096 complement(431536..432135)
<313> 2303: 352-371
<400> 2303
CAGATCGGTA CTTTACGTAT GTCGCTGGTG AATATTACGC CTGATGCAGA 50
TGGCACCACC CTTACACTAC GTATTCAGGG TGAGTCGAAT GATCCCTTGC 100
<210> 2304
<211> 100
<212> DNA
<213> Escherichia coli K-12 MG1655
<220> yaji b0413
<222> 254-353
<223> yaji b0413 U00096 complement(431536..432135)
<313> 2304: 277-296
<400> 2304
ACCTTCTGCC TGGAGCGAAA ACACCGGCAA GACTGGAAAG CCAGATCGGT 50

ACTTTACGTA TGTCGCTGGT GAATATTACG CCTGATGCAG ATGGCACCAC 100
<210> 2305
<211> 100
<212> DNA
<213> Escherichia coli K-12 MG1655
<220> yajk b0424
<222> 1350-1449
<223> yajk b0424 U00096 440773..442221
<313> 2305: 1418-1437
<400> 2305
AACCTGGCTG CTGTGGTGTG AGCGCGGGGT GATGAGCCGT CTGCAGGCGC 50
TCTATCTGCG CGAGCAGGGC TTTAACAATG TGAAGGTATA TCGCCCGTAA 100
<210> 2306
<211> 100
<212> DNA
<213> Escherichia coli K-12 MG1655
<220> yajk b0424
<222> 1328-1427
<223> yajk b0424 U00096 440773..442221
<313> 2306: 1361-1380
<400> 2306
TTGGCGATCT CGACCAGAAC AAAACCTGGC TGCTGTGGTG TGAGCGCGGG 50
GTGATGAGCC GTCTGCAGGC GCTCTATCTG CGCGAGCAGG GCTTTAACAA 100
<210> 2307
<211> 100
<212> DNA
<213> Escherichia coli K-12 MG1655
<220> yajk b0424
<222> 1262-1361
<223> yajk b0424 U00096 440773..442221
<313> 2307: 1292-1311
<400> 2307
ATAAGCCACT GAAAGTCGAA GGGATTGATG TGGTTTCTCT GCCGTTCTAT 50
AAACTGAGCA CCAAATTTGG CGATCTCGAC CAGAACAAAA CCTGGCTGCT 100
<210> 2308
<211> 100
<212> DNA
<213> Escherichia coli K-12 MG1655
<220> b1481 b1482
<222> 129-228
<223> b1481 b1482 U00096 complement(1554089..1554367)
<313> 2308: 175-194
<400> 2308
AGGCCTTCGG GCACAGCATG GTGTTGTCAC TGAAGATGAC ATTCTCATGG 50
AGCTGACCAA ATGGGTTGAA GCTTCTGATA ACGACATCCT CAGTGATATC 100
<210> 2309
<211> 100
<212> DNA
<213> Escherichia coli K-12 MG1655
<220> b1481 b1482
<222> 69-168
<223> b1481 b1482 U00096 complement(1554089..1554367)
<313> 2309: 96-115
<400> 2309
TACTTATTAT CAGGCAGAAA ATTCAACAGC AGAACCCGCT CTGGTTAATG 50
CCATTGAGCA AGGCCTTCGG GCACAGCATG GTGTTGTCAC TGAAGATGAC 100
<210> 2310
<211> 100
<212> DNA
<213> Escherichia coli K-12 MG1655
<220> b1481 b1482
<222> 0-99
<223> b1481 b1482 U00096 complement(1554089..1554367)
<313> 2310: 43-62
<400> 2310
ATGTGCTTTA TAAATCTGCG ATCCGTAGCA GACACCATAA ATACACAGAC 50
ACGGAGAATC ACTATGTTTA CTTATTATCA GGCAGAAAAT TCAACAGCA 100
<210> 2311
<211> 100
<212> DNA
<213> Escherichia coli K-12 MG1655

<220> b0753 b0754
<222> 233-332
<223> b0753 b0754 U00096 complement(784160..784540)
<313> 2311: 270-289
<400> 2311
CAATGAACAA TGACGGAATG ACCAAAGATG AAGAGCACAA GAACACCATG  50
TGCAAAGATG GTCGCTGTCC GGATATCAAT AAAAAGGTAC AAACCGGTGA  100
<210> 2312
<211> 100
<212> DNA
<213> Escherichia coli K-12 MG1655
<220> b0753 b0754
<222> 95-194
<223> b0753 b0754 U00096 complement(784160..784540)
<313> 2312: 149-168
<400> 2312
ATAACGGCCA GGCAAACGCC GCAGCTGATG CGGGCCAGGT AGCCCCTGAC  50
GCCCGTGAAA ATGTCGCGCC AAATAACGTC GACAATAACG GGGTAAATAC  100
<210> 2313
<211> 100
<212> DNA
<213> Escherichia coli K-12 MG1655
<220> b0753 b0754
<222> 54-153
<223> b0753 b0754 U00096 complement(784160..784540)
<313> 2313: 89-108
<400> 2313
CAGCGGTGCC GCACTGGCTG CCGATAGCGG AGCGCAAACT AATAACGGCC  50
AGGCAAACGC CGCAGCTGAT GCGGGCCAGG TAGCCCCTGA CGCCCGTGAA  100
<210> 2314
<211> 100
<212> DNA
<213> Escherichia coli K-12 MG1655
<220> b1483 b1483
<222> 822-921
<223> b1483 b1483 U00096 complement(1555136..1556062)
<313> 2314: 876-895
<400> 2314
TTTTTTCTAC GAACGTTGCC CGCTGGCAAC CCACGGATGT GAAGTCCGGC  50
AATCATTAGC AATAAGGGAG GACGGACGTG AGCTCCGGTG CTGGCGGGCG  100
<210> 2315
<211> 100
<212> DNA
<213> Escherichia coli K-12 MG1655
<220> b1483 b1483
<222> 762-861
<223> b1483 b1483 U00096 complement(1555136..1556062)
<313> 2315: 822-841
<400> 2315
GGAAGAATGG GCATTACGTA AAACGGATCT GCCGGGAAAC CGCACGTTGC  50
CGCAAGGCTG TTTTTTCTAC GAACGTTGCC CGCTGGCAAC CCACGGATGT  100
<210> 2316
<211> 100
<212> DNA
<213> Escherichia coli K-12 MG1655
<220> b1483 b1483
<222> 728-827
<223> b1483 b1483 U00096 complement(1555136..1556062)
<313> 2316: 742-761
<400> 2316
TGGATTCCCT CCCCGCCATT GATAAACCGC TGGAGGAAGA ATGGGCATTA  50
CGTAAAACGG ATCTGCCGGG AAACCGCACG TTGCCGCAAG GCTGTTTTTT  100
<210> 2317
<211> 100
<212> DNA
<213> Escherichia coli K-12 MG1655
<220> yajo b0420
<222> 948-1047
<223> yajo b0420 U00096 complement(436385..437431)
<313> 2317: 1008-1027
<400> 2317

TGATGAGCTA TTGAACGCGG TGGATATCAC TTTGAAGCCG GAACAGATTG 50
CCGAACTGGA AACGCCGTAT AAACCGCATC CTGTCGTAGG ATTTAAATAA 100
<210> 2318
<211> 100
<212> DNA
<213> Escherichia coli K-12 MG1655
<220> yajo b0420
<222> 928-1027
<223> yajo b0420 U00096 complement(436385..437431)
<313> 2318: 957-976
<400> 2318
ACTTCGCGCG AAGAACAGCT TGATGAGCTA TTGAACGCGG TGGATATCAC 50
TTTGAAGCCG GAACAGATTG CCGAACTGGA AACGCCGTAT AAACCGCATC 100
<210> 2319
<211> 100
<212> DNA
<213> Escherichia coli K-12 MG1655
<220> yajo b0420
<222> 847-946
<223> yajo b0420 U00096 complement(436385..437431)
<313> 2319: 914-933
<400> 2319
GAAGAACTGG GGGCGACACG AGCACAAGTT GCGCTGGCCT GGTTGTTGAG 50
TAAACCGGGC ATTGCCGCAC CGATTATCGG AACTTCGCGC GAAGAACAGC 100
<210> 2320
<211> 100
<212> DNA
<213> Escherichia coli K-12 MG1655
<220> b1484 b1484
<222> 790-889
<223> b1484 b1484 U00096 complement(1556055..1557041)
<313> 2320: 844-863
<400> 2320
GGAGTACCAC GCCAGCTATT ACCCGCCATT CCAGGGACGG TACCAAACCT 50
CACCCATTTG CCTGACGGCT GCGCTTTTCG CGATCGTTGC TATGCGGCAG 100
<210> 2321
<211> 100
<212> DNA
<213> Escherichia coli K-12 MG1655
<220> b1484 b1484
<222> 766-865
<223> b1484 b1484 U00096 complement(1556055..1557041)
<313> 2321: 803-822
<400> 2321
TTGCTGCAAT GCGCACCAGA ACATGGAGTA CCACGCCAGC TATTACCCGC 50
CATTCCAGGG ACGGTACCAA ACCTCACCCA TTTGCCTGAC GGCTGCGCTT 100
<210> 2322
<211> 100
<212> DNA
<213> Escherichia coli K-12 MG1655
<220> b1484 b1484
<222> 711-810
<223> b1484 b1484 U00096 complement(1556055..1557041)
<313> 2322: 740-759
<400> 2322
AAGCGGCGTG ACGGCAGACG TTATCCATCA TCCCCGGCAT CCGTATACCA 50
TTGGTTTGCT GCAATGCGCA CCAGAACATG GAGTACCACG CCAGCTATTA 100
<210> 2323
<211> 100
<212> DNA
<213> Escherichia coli K-12 MG1655
<220> b1485 b1485
<222> 788-887
<223> b1485 b1485 U00096 complement(1557038..1557934)
<313> 2323: 836-855
<400> 2323
ATTGCGCATT TCCGGGGGCA GCGATTTTGC TTACCGCCGT CGGGTTTAAT 50
CTCTTTGGTG ATGGTATTCG CGATCTGCTT GACCCGAAAG CAGGAGGAAA 100
<210> 2324
<211> 100
<212> DNA

<213> Escherichia coli K-12 MG1655
<220> b1485 b1485
<222> 709-808
<223> b1485 b1485 U00096 complement(1557038..1557934)
<313> 2324: 759-778
<400> 2324
CTGGGTGCTC AACAACCGAG TGCTGAATGG GGCGCGATGG TAGCAAATGG 50
TCGCAACTAT GTACTCGATC AGTGGTGGTA TTGCGCATTT CCGGGGGCAG 100
<210> 2325
<211> 100
<212> DNA
<213> Escherichia coli K-12 MG1655
<220> b1485 b1485
<222> 596-695
<223> b1485 b1485 U00096 complement(1557038..1557934)
<313> 2325: 644-663
<400> 2325
GGCATCTGAT CAACTGGCAT ATTTTACGTA ACTCCCTACC GCCGCTGATC 50
GTGCAGGCAT CGCTGGATAT CGGTAGCGCG ATTTTAATGG CCGCCACGTT 100
<210> 2326
<211> 100
<212> DNA
<213> Escherichia coli K-12 MG1655
<220> yajq b0427
<222> 411-510
<223> yajq b0427 U00096 443889..444398
<313> 2326: 490-509
<400> 2326
CGTAACGGGC AAATCTCGTG ATGATTTGCA GGCTGTCATG GCGATGGTAC 50
GTGGTGGCGA TCTCGGTCAG CCGTTCCAGT TCAAAAACTT CCGCGATTAA 100
<210> 2327
<211> 100
<212> DNA
<213> Escherichia coli K-12 MG1655
<220> yajq b0427
<222> 361-460
<223> yajq b0427 U00096 443889..444398
<313> 2327: 412-431
<400> 2327
AAAGACAGCA AACTGAAAGT GCAGGCGCAA ATTCAGGGCG ATGAAATCCG 50
CGTAACGGGC AAATCTCGTG ATGATTTGCA GGCTGTCATG GCGATGGTAC 100
<210> 2328
<211> 100
<212> DNA
<213> Escherichia coli K-12 MG1655
<220> yajq b0427
<222> 306-405
<223> yajq b0427 U00096 443889..444398
<313> 2328: 364-383
<400> 2328
GAAACTGAAA CAGGGCATTG AGAGCGCGAC CCAGAAGAAA ATCGTCAAGA 50
TGATCAAAGA CAGCAAACTG AAAGTGCAGG CGCAAATTCA GGGCGATGAA 100
<210> 2329
<211> 100
<212> DNA
<213> Escherichia coli K-12 MG1655
<220> b1486 b1486
<222> 923-1022
<223> b1486 b1486 U00096 complement(1557931..1558953)
<313> 2329: 945-964
<400> 2329
TTGCCGTCGT GGTTTCATTT GCTTATGTGC TGGTCAACCT GGTGGTGGAT 50
TTGCTCTATT TGTGGATTGA TCCGCGTATC GGACGTGGAG GTGGTGAATG 100
<210> 2330
<211> 100
<212> DNA
<213> Escherichia coli K-12 MG1655
<220> b1486 b1486
<222> 862-961
<223> b1486 b1486 U00096 complement(1557931..1558953)
<313> 2330: 900-919

<400> 2330
GGAATGGGTG CATGGGTAGT AACATCAATA CAGGCGCTCG ACTTCCCGGC  50
AGTGATGGGC TTTGCCGTCG TGGTTTCATT TGCTTATGTG CTGGTCAACC  100
<210> 2331
<211> 100
<212> DNA
<213> Escherichia coli K-12 MG1655
<220> b1486 b1486
<222> 798-897
<223> b1486 b1486 U00096 complement(1557931..1558953)
<313> 2331: 852-871
<400> 2331
GGGTTTGGCG CTGGGCGATT TGTTGTATGG CGCAGTGCTC ACCGAAACCG  50
TTTTTGCCTG GCCCGGAATG GGTGCATGGG TAGTAACATC AATACAGGCG  100
<210> 2332
<211> 100
<212> DNA
<213> Escherichia coli K-12 MG1655
<220> yajr b0428
<222> 1272-1371
<223> yajr b0428 U00096 complement(444526..445896)
<313> 2332: 1346-1365
<400> 2332
AGAAGTGTTG ATTGCAGAAG AAGAACATTC AGCTTATGTG AAAATCGACA  50
GCAAAGTGAC GAATCGCTTT GAGATAGAAC AGGCAATTCG TCAGGCATAA  100
<210> 2333
<211> 100
<212> DNA
<213> Escherichia coli K-12 MG1655
<220> yajr b0428
<222> 1141-1240
<223> yajr b0428 U00096 complement(444526..445896)
<313> 2333: 1172-1191
<400> 2333
GCAGTGTGGC TGACAGTCGC CAGTACCATG AAAGAACCGC CGTATGTCAG  50
CAGTTTGCGC ATTGAAATCC CGGCGAACAT TGCCGCAAAC GAGGCGTTAA  100
<210> 2334
<211> 100
<212> DNA
<213> Escherichia coli K-12 MG1655
<220> yajr b0428
<222> 1032-1131
<223> yajr b0428 U00096 complement(444526..445896)
<313> 2334: 1094-1113
<400> 2334
TTACTCCACC AGCCAGTTTC TTGGCGTGGC GATTGGCGGT TCGCTGGGCG  50
GCTGGATTAA CGGCATGTTT GACGGTCAGG GGGTATTTCT CGCTGGCGCA  100
<210> 2335
<211> 100
<212> DNA
<213> Escherichia coli K-12 MG1655
<220> b1487 b1487
<222> 1450-1549
<223> b1487 b1487 U00096 complement(1558955..1560505)
<313> 2335: 1493-1512
<400> 2335
TTCCAGAAAA ACTACCAACT GGCGATGAAC AAAGAGGTGA AAGGCTTTGT  50
GTTCAATCCC ATGCTGGAAC AGGTCTTCAA TATCAATACC ATGAGTAAAT  100
<210> 2336
<211> 100
<212> DNA
<213> Escherichia coli K-12 MG1655
<220> b1487 b1487
<222> 1438-1537
<223> b1487 b1487 U00096 complement(1558955..1560505)
<313> 2336: 1461-1480
<400> 2336
TATGTGTACC TGTTCCAGAA AAACTACCAA CTGGCGATGA ACAAAGAGGT  50
GAAAGGCTTT GTGTTCAATC CCATGCTGGA ACAGGTCTTC AATATCAATA  100
<210> 2337
<211> 100

<212> DNA
<213> Escherichia coli K-12 MG1655
<220> b1487 b1487
<222> 1310-1409
<223> b1487 b1487 U00096 complement(1558955..1560505)
<313> 2337: 1369-1388
<400> 2337
GTAACCGCTC GTTCTATGAA AACAGTGAGG TCGATAAGTT ACTGCGCAAT 50
GCGCTTGCGA CCACCGACCA GACGCAGCGT ACCCGGGACT ACCAGCAGGC 100
<210> 2338
<211> 100
<212> DNA
<213> Escherichia coli K-12 MG1655
<220> b1488 b1488
<222> 483-582
<223> b1488 b1488 U00096 complement(1560519..1561100)
<313> 2338: 561-580
<400> 2338
CGAATGGTGG CACTTCGAAT TACCCCAGGC AGCGAGTTAC CCTCTGCTCG 50
CTGATCAATT CTCTTGTTTT ATATCCCCTG GCACACAGCA CGTCAGTTAA 100
<210> 2339
<211> 100
<212> DNA
<213> Escherichia coli K-12 MG1655
<220> b1488 b1488
<222> 447-546
<223> b1488 b1488 U00096 complement(1560519..1561100)
<313> 2339: 506-525
<400> 2339
GATAATGACT GGTGGCGGTT TTGTCGGTAT CTCCAGCGAA TGGTGGCACT 50
TCGAATTACC CCAGGCAGCG AGTTACCCTC TGCTCGCTGA TCAATTCTCT 100
<210> 2340
<211> 100
<212> DNA
<213> Escherichia coli K-12 MG1655
<220> b1488 b1488
<222> 407-506
<223> b1488 b1488 U00096 complement(1560519..1561100)
<313> 2340: 463-482
<400> 2340
TCCCGCCCGC CGCTCAGCGC AATCGGCTGT TGCTGAATGC GATAATGACT 50
GGTGGCGGTT TTGTCGGTAT CTCCAGCGAA TGGTGGCACT TCGAATTACC 100
<210> 2341
<211> 100
<212> DNA
<213> Escherichia coli K-12 MG1655
<220> gmd b2054
<222> 1023-1122
<223> gmd b2054 U00096 complement(2125215..2126336)
<313> 2341: 1068-1087
<400> 2341
GATGGTGTCT GAAATGGTGG CTAATGACCT CGAAGCGGCG AAAAAACACT 50
CTCTGCTGAA ATCTCACGGC TACGACGTGG CGATCGCGCT GGAGTCATAA 100
<210> 2342
<211> 100
<212> DNA
<213> Escherichia coli K-12 MG1655
<220> gmd b2054
<222> 1009-1108
<223> gmd b2054 U00096 complement(2125215..2126336)
<313> 2342: 1033-1052
<400> 2342
ATCACCCTCA GAGAGATGGT GTCTGAAATG GTGGCTAATG ACCTCGAAGC 50
GGCGAAAAAA CACTCTCTGC TGAAATCTCA CGGCTACGAC GTGGCGATCG 100
<210> 2343
<211> 100
<212> DNA
<213> Escherichia coli K-12 MG1655
<220> gmd b2054
<222> 956-1055
<223> gmd b2054 U00096 complement(2125215..2126336)

<313> 2343: 990-1009
<400> 2343
CGCTGCTCGG CGACCCGACC AAAGCGCACG AAAAACTGGG CTGGAAACCG 50
GAAATCACCC TCAGAGAGAT GGTGTCTGAA ATGGTGGCTA ATGACCTCGA 100
<210> 2344
<211> 100
<212> DNA
<213> Escherichia coli K-12 MG1655
<220> b1489 b1489
<222> 2325-2424
<223> b1489 b1489 U00096 complement(1561358..1563781)
<313> 2344: 2402-2421
<400> 2344
GATCCACTGT CGCGTTATTC AGGGATATTT CTTTTCCCGC CCCCTACCCG 50
CCGAAGAAAT TCCAGGCTGG ATGAGCAGCG TGTTACCGCT GAAAATCTGA 100
<210> 2345
<211> 100
<212> DNA
<213> Escherichia coli K-12 MG1655
<220> b1489 b1489
<222> 2265-2364
<223> b1489 b1489 U00096 complement(1561358..1563781)
<313> 2345: 2325-2344
<400> 2345
CAATTTAACC GTCGTGGCGG AAGGCGTCGA AACCAAAGAG CAATTTGAGA 50
TGCTACGCAA GATCCACTGT CGCGTTATTC AGGGATATTT CTTTTCCCGC 100
<210> 2346
<211> 100
<212> DNA
<213> Escherichia coli K-12 MG1655
<220> b1489 b1489
<222> 2206-2305
<223> b1489 b1489 U00096 complement(1561358..1563781)
<313> 2346: 2258-2277
<400> 2346
CTGACCGAAA AACGCATCCT TGCCTTACTT GAAGCCATTA CCAGCATTGG 50
GCAAAGCCTC AATTTAACCG TCGTGGCGGA AGGCGTCGAA ACCAAAGAGC 100
<210> 2347
<211> 100
<212> DNA
<213> Escherichia coli K-12 MG1655
<220> gmk b3649
<222> 508-607
<223> gmk b3649 U00096 3819055..3819678
<313> 2347: 532-551
<400> 2347
GATGACTTCG ATACCGCGTT GACCGATTTG AAGACCATTA TTCGCGCCGA 50
ACGTCTGCGC ATGAGCCGCC AAAAGCAGCG TCATGACGCT TTAATCAGCA 100
<210> 2348
<211> 100
<212> DNA
<213> Escherichia coli K-12 MG1655
<220> gmk b3649
<222> 405-504
<223> gmk b3649 U00096 3819055..3819678
<313> 2348: 459-478
<400> 2348
ACGCGGTCGC GGTCAGGACA GCGAAGAGGT CATTGCAAAG CGTATGGCGC 50
AAGCTGTTGC AGAAATGAGC CATTACGCCG AATATGATTA TCTGATTGTG 100
<210> 2349
<211> 100
<212> DNA
<213> Escherichia coli K-12 MG1655
<220> gmk b3649
<222> 384-483
<223> gmk b3649 U00096 3819055..3819678
<313> 2349: 417-436
<400> 2349
AATTGAACTG GACCGCCGTC TACGCGGTCG CGGTCAGGAC AGCGAAGAGG 50
TCATTGCAAA GCGTATGGCG CAAGCTGTTG CAGAAATGAG CCATTACGCC 100
<210> 2350

<211> 100
<212> DNA
<213> Escherichia coli K-12 MG1655
<220> fkpa b3348
<222> 714-813
<223> fkpa b3348 U00096 complement(3474244..3475056)
<313> 2350: 777-796
<400> 2350
CCTGGTGTTT GACGTAGAGC TGCTGGATGT GAAACCAGCG CCGAAGGCTG  50
ATGCAAAGCC GGAAGCTGAT GCGAAAGCCG CAGATTCTGC TAAAAAATAA  100
<210> 2351
<211> 100
<212> DNA
<213> Escherichia coli K-12 MG1655
<220> fkpa b3348
<222> 674-773
<223> fkpa b3348 U00096 complement(3474244..3475056)
<313> 2351: 722-741
<400> 2351
ACGGCAAAGC GGGTGTTCCG GGGATCCCAC CGAATTCTAC CCTGGTGTTT  50
GACGTAGAGC TGCTGGATGT GAAACCAGCG CCGAAGGCTG ATGCAAAGCC  100
<210> 2352
<211> 100
<212> DNA
<213> Escherichia coli K-12 MG1655
<220> fkpa b3348
<222> 617-716
<223> fkpa b3348 U00096 complement(3474244..3475056)
<313> 2352: 656-675
<400> 2352
TGAAGAACAT CAAGAAAGGC GGTAAGATCA AACTGGTTAT TCCACCAGAA  50
CTGGCTTACG GCAAAGCGGG TGTTCCGGGG ATCCCACCGA ATTCTACCCT  100
<210> 2353
<211> 100
<212> DNA
<213> Escherichia coli K-12 MG1655
<220> gabd b2662
<222> 1347-1446
<223> gabd b2662 U00096 2789294..2790742
<313> 2353: 1378-1397
<400> 2353
GGCCCCGTTC GGCGGCATCA AAGCCTCGGG TCTGGGTCGT GAAGGTTCGA  50
AGTATGGCAT CGAAGATTAC TTAGAAATCA AATATATGTG CATCGGTCTT  100
<210> 2354
<211> 100
<212> DNA
<213> Escherichia coli K-12 MG1655
<220> gabd b2662
<222> 1306-1405
<223> gabd b2662 U00096 2789294..2790742
<313> 2354: 1346-1365
<400> 2354
GGCATCGTCG GCATCAATAC CGGCATTATT TCCAATGAAG TGGCCCCGTT  50
CGGCGGCATC AAAGCCTCGG GTCTGGGTCG TGAAGGTTCG AAGTATGGCA  100
<210> 2355
<211> 100
<212> DNA
<213> Escherichia coli K-12 MG1655
<220> gabd b2662
<222> 1280-1379
<223> gabd b2662 U00096 2789294..2790742
<313> 2355: 1314-1333
<400> 2355
TCCGCGTGGG CGAAGCGCTG GAGTACGGCA TCGTCGGCAT CAATACCGGC  50
ATTATTTCCA ATGAAGTGGC CCCGTTCGGC GGCATCAAAG CCTCGGGTCT  100
<210> 2356
<211> 100
<212> DNA
<213> Escherichia coli K-12 MG1655
<220> b1490 b1490
<222> 1284-1383

<223> b1490 b1490 U00096 complement(1563782..1565164)
<313> 2356: 1346-1365
<400> 2356
TCCTGACTAT GAGCGCCTCA TTCAAATAGC CGATGAAGCT CTGTATATCG 50
CCAAAAGACG AGGTAGAAAC CGTGTTGAAC TCTGGAAAGC CAGTCTTTAG 100
<210> 2357
<211> 100
<212> DNA
<213> Escherichia coli K-12 MG1655
<220> b1490 b1490
<222> 1284-1383
<223> b1490 b1490 U00096 complement(1563782..1565164)
<313> 2357: 1316-1335
<400> 2357
TCCTGACTAT GAGCGCCTCA TTCAAATAGC CGATGAAGCT CTGTATATCG 50
CCAAAAGACG AGGTAGAAAC CGTGTTGAAC TCTGGAAAGC CAGTCTTTAG 100
<210> 2358
<211> 100
<212> DNA
<213> Escherichia coli K-12 MG1655
<220> b1490 b1490
<222> 1175-1274
<223> b1490 b1490 U00096 complement(1563782..1565164)
<313> 2358: 1239-1258
<400> 2358
GTACCGCAGA ACGTATTCGC AGTCGGGTGG AGAAAACCAA ACTGAAAGCC 50
GCAAACGGCG AAGATATTGC CCTCTCACTT TCCATCGGTG CCGCCATGTT 100
<210> 2359
<211> 100
<212> DNA
<213> Escherichia coli K-12 MG1655
<220> b0762 b0763
<222> 51-150
<223> b0762 b0763 U00096 793996..794145
<313> 2359: 122-141
<400> 2359
CGTGATGGCC ATCATCCTGG GTCTGATTTA CGGTCTTGGT GAAGTATTCA 50
ACATCTTTTC TGGTGTTGGT AAAAAAGACC AGCCCGGACA AAATCATTGA 100
<210> 2360
<211> 100
<212> DNA
<213> Escherichia coli K-12 MG1655
<220> b0762 b0763
<222> 51-150
<223> b0762 b0763 U00096 793996..794145
<313> 2360: 105-124
<400> 2360
CGTGATGGCC ATCATCCTGG GTCTGATTTA CGGTCTTGGT GAAGTATTCA 50
ACATCTTTTC TGGTGTTGGT AAAAAAGACC AGCCCGGACA AAATCATTGA 100
<210> 2361
<211> 100
<212> DNA
<213> Escherichia coli K-12 MG1655
<220> b0762 b0763
<222> 29-128
<223> b0762 b0763 U00096 793996..794145
<313> 2361: 78-97
<400> 2361
TCGCCGTAAT CATGGTACCT GTCGTGATGG CCATCATCCT GGGTCTGATT 50
TACGGTCTTG GTGAAGTATT CAACATCTTT TCTGGTGTTG GTAAAAAAGA 100
<210> 2362
<211> 100
<212> DNA
<213> Escherichia coli K-12 MG1655
<220> b1491 b1492
<222> 1221-1320
<223> b1491 b1492 U00096 complement(1565528..1566847)
<313> 2362: 1293-1312
<400> 2362
GCCCGAAATT AGCGGCACCA TCTTGTTCCG TGAGGACTAT CTGAATAAAC 50
CGCAGACTCA ACAAGCGGTC AGCTATCTGC AAAGTCGCTG GGGCAGTTAA 100

<210> 2363
<211> 100
<212> DNA
<213> Escherichia coli K-12 MG1655
<220> b1491 b1492
<222> 1207-1306
<223> b1491 b1492 U00096 complement(1565528..1566847)
<313> 2363: 1246-1265
<400> 2363
CTTAACGATG CTGTGCCCGA AATTAGCGGC ACCATCTTGT TCCGTGAGGA 50
CTATCTGAAT AAACCGCAGA CTCAACAAGC GGTCAGCTAT CTGCAAAGTC 100
<210> 2364
<211> 100
<212> DNA
<213> Escherichia coli K-12 MG1655
<220> b1491 b1492
<222> 1175-1274
<223> b1491 b1492 U00096 complement(1565528..1566847)
<313> 2364: 1212-1231
<400> 2364
GCGGCGTACC GGAACTGAAA AAGCAGCTCG ATCTTAACGA TGCTGTGCCC 50
GAAATTAGCG GCACCATCTT GTTCCGTGAG GACTATCTGA ATAAACCGCA 100
<210> 2365
<211> 100
<212> DNA
<213> Escherichia coli K-12 MG1655
<220> b1497 b1497
<222> 912-1011
<223> b1497 b1497 U00096 complement(1577657..1578829)
<313> 2365: 937-956
<400> 2365
GAACAGTGTA CAACTGACAG CGCAAAAAAA ACGGATTCCA GCGAAATGTC 50
AGCAATGTGC ATATAAACCT ATCTGCAATG GCGGTTGTCC TAAGCATCGT 100
<210> 2366
<211> 100
<212> DNA
<213> Escherichia coli K-12 MG1655
<220> b1497 b1497
<222> 723-822
<223> b1497 b1497 U00096 complement(1577657..1578829)
<313> 2366: 774-793
<400> 2366
AATTTTCATC CGTCAGTTTG AAAGCTTTGT CAGCCGTTTT TTGGGGAATG 50
GGCATACCAG TTGTATTTTC CAGGAGTCCT GCAAGGATAA TCTGGTTGTT 100
<210> 2367
<211> 100
<212> DNA
<213> Escherichia coli K-12 MG1655
<220> b1497 b1497
<222> 614-713
<223> b1497 b1497 U00096 complement(1577657..1578829)
<313> 2367: 674-693
<400> 2367
GTCATAGTGA GAACACATTC CGTATCATTG ATTTTTCTGT GCCTCCCACG 50
GCTTATGGCA AGTTTATGTC AACCATTTTT ATGCAATGGG TTAAAAACGA 100
<210> 2368
<211> 100
<212> DNA
<213> Escherichia coli K-12 MG1655
<220> acpd b1413
<222> 507-606
<223> acpd b1413 U00096 complement(1480279..1480884)
<313> 2368: 572-591
<400> 2368
GAAATTTGTC TTCGCCGAAG GGATCGCATA CGGTCCGGAA ATGGCAGCGA 50
AAGCACAGTC TGACGCAAAA GCAGCCATCG ACAGCATTGT TTCTGCATAA 100
<210> 2369
<211> 100
<212> DNA
<213> Escherichia coli K-12 MG1655
<220> acpd b1413

<222> 460-559
<223> acpd b1413 U00096 complement(1480279..1480884)
<313> 2369: 507-526
<400> 2369
ACGCCGTATC TGTCCACGTT CCTCGGCTTT ATCGGCATTA CCGATGTGAA 50
ATTTGTCTTC GCCGAAGGGA TCGCATACGG TCCGGAAATG GCAGCGAAAG 100
<210> 2370
<211> 100
<212> DNA
<213> Escherichia coli K-12 MG1655
<220> acpd b1413
<222> 438-537
<223> acpd b1413 U00096 complement(1480279..1480884)
<313> 2370: 463-482
<400> 2370
AGATGGACCA ACGGACCTGG TGACGCCGTA TCTGTCCACG TTCCTCGGCT 50
TTATCGGCAT TACCGATGTG AAATTTGTCT TCGCCGAAGG GATCGCATAC 100
<210> 2371
<211> 100
<212> DNA
<213> Escherichia coli K-12 MG1655
<220> b1498 b1498
<222> 1617-1716
<223> b1498 b1498 U00096 complement(1578866..1580581)
<313> 2371: 1665-1684
<400> 2371
CGTGGTAAGA GAGTTTATCG ACAGCAGCCA GCCACCGCTT AGCGAGGTAA 50
ATCAGGAGAA GTTTAACAAT ATCAAGAAAG CACTAAGCGA AGCGAAATAA 100
<210> 2372
<211> 100
<212> DNA
<213> Escherichia coli K-12 MG1655
<220> b1498 b1498
<222> 1561-1660
<223> b1498 b1498 U00096 complement(1578866..1580581)
<313> 2372: 1611-1630
<400> 2372
CAGCAAAAAG ATAACCTTGC CGCCGCCAAT CCGCAGGTCG TTAAAGAGAT 50
GCAAGGCGTG GTAAGAGAGT TTATCGACAG CAGCCAGCCA CCGCTTAGCG 100
<210> 2373
<211> 100
<212> DNA
<213> Escherichia coli K-12 MG1655
<220> b1498 b1498
<222> 1536-1635
<223> b1498 b1498 U00096 complement(1578866..1580581)
<313> 2373: 1583-1602
<400> 2373
AGGTCTCTAC AAACTGACGG ATCTACAGCA AAAAGATAAC CTTGCCGCCG 50
CCAATCCGCA GGTCGTTAAA GAGATGCAAG GCGTGGTAAG AGAGTTTATC 100
<210> 2374
<211> 100
<212> DNA
<213> Escherichia coli K-12 MG1655
<220> gnd b2030
<222> 1285-1384
<223> gnd b2030 U00096 complement(2097884..2099290)
<313> 2374: 1324-1343
<400> 2374
GACAGCTACC GTGCTGCTGT TCTGCCTGCG AACCTGATCC AGGCACAGCG 50
TGACTATTTT GGTGCGCATA CTTATAAGCG TATTGATAAA GAAGGTGTGT 100
<210> 2375
<211> 100
<212> DNA
<213> Escherichia coli K-12 MG1655
<220> gnd b2030
<222> 1246-1345
<223> gnd b2030 U00096 complement(2097884..2099290)
<313> 2375: 1283-1302
<400> 2375
ATTCCGGTTC CGACCTTCTC CGCAGCGGTT GCCTATTACG ACAGCTACCG 50

TGCTGCTGTT CTGCCTGCGA ACCTGATCCA GGCACAGCGT GACTATTTTG 100
<210> 2376
<211> 100
<212> DNA
<213> Escherichia coli K-12 MG1655
<220> gnd b2030
<222> 1206-1305
<223> gnd b2030 U00096 complement(2097884..2099290)
<313> 2376: 1239-1258
<400> 2376
GGCGCTGCGT GATGTCGTTG CTTATGCAGT ACAGAACGGT ATTCCGGTTC 50
CGACCTTCTC CGCAGCGGTT GCCTATTACG ACAGCTACCG TGCTGCTGTT 100
<210> 2377
<211> 100
<212> DNA
<213> Escherichia coli K-12 MG1655
<220> b1499 b1499
<222> 663-762
<223> b1499 b1499 U00096 complement(1580950..1581711)
<313> 2377: 724-743
<400> 2377
ACATTTCGGC AACAGTCCGA AGAGAGTTTC TAAGGAGTAC CGTTGTCAAA 50
GTCACACGGG TATGAATACG GGCAACACGA TGAATGCTTT AGCTATTTGA 100
<210> 2378
<211> 100
<212> DNA
<213> Escherichia coli K-12 MG1655
<220> b1499 b1499
<222> 658-757
<223> b1499 b1499 U00096 complement(1580950..1581711)
<313> 2378: 691-710
<400> 2378
CGCAAACATT TCGGCAACAG TCCGAAGAGA GTTTCTAAGG AGTACCGTTG 50
TCAAAGTCAC ACGGGTATGA ATACGGGCAA CACGATGAAT GCTTTAGCTA 100
<210> 2379
<211> 100
<212> DNA
<213> Escherichia coli K-12 MG1655
<220> b1499 b1499
<222> 434-533
<223> b1499 b1499 U00096 complement(1580950..1581711)
<313> 2379: 479-498
<400> 2379
TGAAGCCGGC GCACCCATGG AAGCTGAAAG ATATTTGTGA CTGCCTGTAC 50
ATCAGTGAAA GCCTGTTGAA GAAAAAACTT AAGCAAGAGC AAACGACATT 100
<210> 2380
<211> 100
<212> DNA
<213> Escherichia coli K-12 MG1655
<220> gabp b2664
<222> 1302-1401
<223> gabp b2664 U00096 2792274..2793674
<313> 2380: 1355-1374
<400> 2380
CGGCTTATTA GCGATAGGGA TTATCTGTAC CGTGCCAATT ATGGCTCGCT 50
GGAAAAAGCT GGTATTGTGG CAAAAAACAC CCGTTCATAA TACGCGCTGA 100
<210> 2381
<211> 100
<212> DNA
<213> Escherichia coli K-12 MG1655
<220> gabp b2664
<222> 1269-1368
<223> gabp b2664 U00096 2792274..2793674
<313> 2381: 1314-1333
<400> 2381
CCGTCCGGCG CAACAGTTAG AAGTGATCTC CACCGGCTTA TTAGCGATAG 50
GGATTATCTG TACCGTGCCA ATTATGGCTC GCTGGAAAAA GCTGGTATTG 100
<210> 2382
<211> 100
<212> DNA
<213> Escherichia coli K-12 MG1655

<220> gabp b2664
<222> 1252-1351
<223> gabp b2664 U00096 2792274..2793674
<313> 2382: 1286-1305
<400> 2382
TTGGTAGTGA TGCTATTCCG TCCGGCGCAA CAGTTAGAAG TGATCTCCAC 50
CGGCTTATTA GCGATAGGGA TTATCTGTAC CGTGCCAATT ATGGCTCGCT 100
<210> 2383
<211> 100
<212> DNA
<213> Escherichia coli K-12 MG1655
<220> gabt b2663
<222> 1182-1281
<223> gabt b2663 U00096 2790756..2792036
<313> 2383: 1213-1232
<400> 2383
CAACGTGCTG CGCATCCTTG TACCGCTCAC CATTGAAGAC GCTCAGATCC 50
GTCAGGGTCT GGAGATCATC AGCCAGTGTT TTGATGAGGC GAAGCAGTAG 100
<210> 2384
<211> 100
<212> DNA
<213> Escherichia coli K-12 MG1655
<220> gabt b2663
<222> 1136-1235
<223> gabt b2663 U00096 2790756..2792036
<313> 2384: 1153-1172
<400> 2384
GCGCCCGCGA TAAAGGCCTG ATTCTTCTCT CCTGCGGCCC GTATTACAAC 50
GTGCTGCGCA TCCTTGTACC GCTCACCATT GAAGACGCTC AGATCCGTCA 100
<210> 2385
<211> 100
<212> DNA
<213> Escherichia coli K-12 MG1655
<220> gabt b2663
<222> 1082-1181
<223> gabt b2663 U00096 2790756..2792036
<313> 2385: 1105-1124
<400> 2385
AAGACGGCGA TCACAACAAG CCGGACGCCA AACTCACCGC CGAGATCGTG 50
GCTCGCGCCC GCGATAAAGG CCTGATTCTT CTCTCCTGCG GCCCGTATTA 100
<210> 2386
<211> 100
<212> DNA
<213> Escherichia coli K-12 MG1655
<220> kdpa b0699
<222> 1575-1674
<223> kdpa b0699 U00096 complement(726282..727955)
<313> 2386: 1636-1655
<400> 2386
GCTGTTTGTT GGCCTGTTAA TCGGCACCGT GTTGCTGGTT GGCGCACTGA 50
CCTTTATCCC TGCCCTGGCG CTTGGTCCGG TGGCGGAATA TCTCTCCTGA 100
<210> 2387
<211> 100
<212> DNA
<213> Escherichia coli K-12 MG1655
<220> kdpa b0699
<222> 1525-1624
<223> kdpa b0699 U00096 complement(726282..727955)
<313> 2387: 1583-1602
<400> 2387
AGTAAAAAGA GCCAAGCCGC CAGCTCCGGC ACGCTGCCAA CGCACGGCCC 50
GCTGTTTGTT GGCCTGTTAA TCGGCACCGT GTTGCTGGTT GGCGCACTGA 100
<210> 2388
<211> 100
<212> DNA
<213> Escherichia coli K-12 MG1655
<220> kdpa b0699
<222> 1498-1597
<223> kdpa b0699 U00096 complement(726282..727955)
<313> 2388: 1533-1552
<400> 2388

GTGATGGCAA TTGCCGGTTC GCTGGTGAGT AAAAAGAGCC AAGCCGCCAG 50
CTCCGGCACG CTGCCAACGC ACGGCCCGCT GTTTGTTGGC CTGTTAATCG 100
<210> 2389
<211> 100
<212> DNA
<213> Escherichia coli K-12 MG1655
<220> kdpb b0698
<222> 1915-2014
<223> kdpb b0698 U00096 complement(724211..726259)
<313> 2389: 1963-1982
<400> 2389
GTGAGTTATA AACCGCTTAC CGCTTCTGCC ATGTTGCGCC GTAACTTATG 50
GATTTACGGT CTGGGTGGGC TGCTGGTGCC GTTTATCGGT ATCAAAGTCA 100
<210> 2390
<211> 100
<212> DNA
<213> Escherichia coli K-12 MG1655
<220> kdpb b0698
<222> 1900-1999
<223> kdpb b0698 U00096 complement(724211..726259)
<313> 2390: 1919-1938
<400> 2390
CTGGCGTTAA AAGGCGTGAG TTATAAACCG CTTACCGCTT CTGCCATGTT 50
GCGCCGTAAC TTATGGATTT ACGGTCTGGG TGGGCTGCTG GTGCCGTTTA 100
<210> 2391
<211> 100
<212> DNA
<213> Escherichia coli K-12 MG1655
<220> kdpb b0698
<222> 1799-1898
<223> kdpb b0698 U00096 complement(724211..726259)
<313> 2391: 1854-1873
<400> 2391
CGCAGTTAAA TGCGCTGAAC ATCATGTGCC TGCATTCGCC CGACTCCGCA 50
ATCCTCAGTG CGGTGATTTT CAACGCCTTG ATTATCGTCT TTTTGATTCC 100
<210> 2392
<211> 100
<212> DNA
<213> Escherichia coli K-12 MG1655
<220> kdpc b0697
<222> 424-523
<223> kdpc b0697 U00096 complement(723630..724202)
<313> 2392: 468-487
<400> 2392
CCACGCGTGG CGAAAGCGCG TAATCTCAGC GTTGAACAGC TCACGCAACT 50
GATCGCAAAA TACAGCCAAC AACCGCTGGT GAAATATATC GGCCAGCCGG 100
<210> 2393
<211> 100
<212> DNA
<213> Escherichia coli K-12 MG1655
<220> kdpc b0697
<222> 354-453
<223> kdpc b0697 U00096 complement(723630..724202)
<313> 2393: 398-417
<400> 2393
TGAACTGGTG ACGGCATCGG CAAGCGGGCT GGACAATAAT ATCACCCCGC 50
AAGCGGCGGC CTGGCAAATC CCACGCGTGG CGAAAGCGCG TAATCTCAGC 100
<210> 2394
<211> 100
<212> DNA
<213> Escherichia coli K-12 MG1655
<220> kdpc b0697
<222> 323-422
<223> kdpc b0697 U00096 complement(723630..724202)
<313> 2394: 357-376
<400> 2394
CTAACCCGGA TGCCAGCGCG AGCGTTCCGG TTGAACTGGT GACGGCATCG 50
GCAAGCGGGC TGGACAATAA TATCACCCCG CAAGCGGCGG CCTGGCAAAT 100
<210> 2395
<211> 100
<212> DNA

<213> Escherichia coli K-12 MG1655
<220> kdpd b0696
<222> 2586-2685
<223> kdpd b0696 U00096 complement(720953..723637)
<313> 2395: 2643-2662
<400> 2395
TATTACCGCG TTCAACCGAC CGGAAGGTGG TGCCTGTTTT CGTGTTACAC 50
TTCCCCAGCA AACTGCCCCT GAACTTGAAG AATTTCATGA GGATATGTGA 100
<210> 2396
<211> 100
<212> DNA
<213> Escherichia coli K-12 MG1655
<220> kdpd b0696
<222> 2578-2677
<223> kdpd b0696 U00096 complement(720953..723637)
<313> 2396: 2596-2615
<400> 2396
GGGGGCACTA TTACCGCGTT CAACCGACCG GAAGGTGGTG CCTGTTTTCG 50
TGTTACACTT CCCCAGCAAA CTGCCCCTGA ACTTGAAGAA TTTCATGAGG 100
<210> 2397
<211> 100
<212> DNA
<213> Escherichia coli K-12 MG1655
<220> kdpd b0696
<222> 2511-2610
<223> kdpd b0696 U00096 complement(720953..723637)
<313> 2397: 2547-2566
<400> 2397
TAAAGAGTCG GCAGTACCGG GGGTAGGGCT TGGACTGGCA ATTTGTCGGG 50
CGATAGTGGA TGTACACGGG GGCACTATTA CCGCGTTCAA CCGACCGGAA 100
<210> 2398
<211> 100
<212> DNA
<213> Escherichia coli K-12 MG1655
<220> kdpe b0695
<222> 514-613
<223> kdpe b0695 U00096 complement(720279..720956)
<313> 2398: 571-590
<400> 2398
ACCCAGCGCC AGCTCCTTAA CCAGGTGTGG GGGCCAAACG CGGTCGAACA 50
CAGTCACTAT TTGCGTATTT ATATGGGACA TCTGCGACAA AAACTGGAAC 100
<210> 2399
<211> 100
<212> DNA
<213> Escherichia coli K-12 MG1655
<220> kdpe b0695
<222> 491-590
<223> kdpe b0695 U00096 complement(720279..720956)
<313> 2399: 517-536
<400> 2399
TCAACAATGC CGGAAAAGTA CTCACCCAGC GCCAGCTCCT TAACCAGGTG 50
TGGGGGCCAA ACGCGGTCGA ACACAGTCAC TATTTGCGTA TTTATATGGG 100
<210> 2400
<211> 100
<212> DNA
<213> Escherichia coli K-12 MG1655
<220> kdpe b0695
<222> 367-466
<223> kdpe b0695 U00096 complement(720279..720956)
<313> 2400: 431-450
<400> 2400
ACCGCGCCCG ATCCGCTGGT AAAATTTTCC GATGTTACCG TCGATTTAGC 50
CGCCCGCGTG ATTCACCGGG GTGAGGAAGA GGTGCATCTC ACACCAATTG 100
<210> 2401
<211> 100
<212> DNA
<213> Escherichia coli K-12 MG1655
<220> acpp b1095
<222> 138-237
<223> acpp b1095 U00096 1150838..1151074
<313> 2401: 201-220

<400> 2401
TCTGGAAGAA GAGTTTGATA CTGAGATTCC GGACGAAGAA GCTGAGAAAA  50
TCACCACCGT TCAGGCTGCC ATTGATTACA TCAACGGCCA CCAGGCGTAA  100
<210> 2402
<211> 100
<212> DNA
<213> Escherichia coli K-12 MG1655
<220> acpp b1095
<222> 100-199
<223> acpp b1095 U00096 1150838..1151074
<313> 2402: 130-149
<400> 2402
GGCGCGGATT CTCTTGACAC CGTTGAGCTG GTAATGGCTC TGGAAGAAGA  50
GTTTGATACT GAGATTCCGG ACGAAGAAGC TGAGAAAATC ACCACCGTTC  100
<210> 2403
<211> 100
<212> DNA
<213> Escherichia coli K-12 MG1655
<220> acpp b1095
<222> 62-161
<223> acpp b1095 U00096 1150838..1151074
<313> 2403: 82-101
<400> 2403
AAGAAGTTAC CAACAATGCT TCTTTCGTTG AAGACCTGGG CGCGGATTCT  50
CTTGACACCG TTGAGCTGGT AATGGCTCTG GAAGAAGAGT TTGATACTGA  100
<210> 2404
<211> 100
<212> DNA
<213> Escherichia coli K-12 MG1655
<220> acps b2564
<222> 282-381
<223> acps b2564 U00096 complement(2698638..2699018)
<313> 2404: 345-364
<400> 2404
GGCATTAAAA CTGGCGGAAA AGCTGGGCGT TGCAAATATG CATGTAACGC  50
TGGCAGATGA GCGGCACTAT GCTTGTGCCA CGGTAATTAT TGAAAGTTAA  100
<210> 2405
<211> 100
<212> DNA
<213> Escherichia coli K-12 MG1655
<220> acps b2564
<222> 249-348
<223> acps b2564 U00096 complement(2698638..2699018)
<313> 2405: 286-305
<400> 2405
GCTCGGCAAA CCACGGCTAC GGCTATGGGG CGAGGCATTA AAACTGGCGG  50
AAAAGCTGGG CGTTGCAAAT ATGCATGTAA CGCTGGCAGA TGAGCGGCAC  100
<210> 2406
<211> 100
<212> DNA
<213> Escherichia coli K-12 MG1655
<220> acps b2564
<222> 88-187
<223> acps b2564 U00096 complement(2698638..2699018)
<313> 2406: 115-134
<400> 2406
CGCGTATTAA GCGATAACGA ATGGGCTATC TGGAAAACGC ACCACCAGCC  50
GGTGCGTTTT CTGGCGAAGC GTTTTGCTGT GAAAGAAGCC GCAGCAAAAG  100
<210> 2407
<211> 100
<212> DNA
<213> Escherichia coli K-12 MG1655
<220> last lasT
<222> 565-664
<223> last lasT U00096 4638511..4639197
<313> 2407: 623-642
<400> 2407
GCAGTGGCAG ATGACATAAA ACTGGTCGAC TGGTTACAAC AACGCCTGGG  50
GCTTTTAGAG CAACGAGACA CGGCAATGTT GCACCGTTTG CTGCATGATA  100
<210> 2408
<211> 100

<212> DNA
<213> Escherichia coli K-12 MG1655
<220> last lasT
<222> 523-622
<223> last lasT U00096 4638511..4639197
<313> 2408: 558-577
<400> 2408
CAAGCTTTAC GCGAACGAGC CATGACATTG CTGACGACTC TGGCAGTGGC 50
AGATGACATA AAACTGGTCG ACTGGTTACA ACAACGCCTG GGGCTTTTAG 100
<210> 2409
<211> 100
<212> DNA
<213> Escherichia coli K-12 MG1655
<220> last lasT
<222> 482-581
<223> last lasT U00096 4638511..4639197
<313> 2409: 530-549
<400> 2409
AACCGGCGAA AAGTGATGCA ACGGCAGACC AACATCAACT GCAAGCTTTA 50
CGCGAACGAG CCATGACATT GCTGACGACT CTGGCAGTGG CAGATGACAT 100
<210> 2410
<211> 100
<212> DNA
<213> Escherichia coli K-12 MG1655
<220> gor b3501
<222> 1254-1353
<223> gor b3501 U00096 3643929..3645281
<313> 2410: 1322-1341
<400> 2410
CTTCGCGGTG GCGCTGAAGA TGGGGGCAAC CAAAAAAGAC TTCGACAATA 50
CCGTCGCCAT TCACCCAACG GCGGCAGAAG AGTTCGTGAC AATGCGTTAA 100
<210> 2411
<211> 100
<212> DNA
<213> Escherichia coli K-12 MG1655
<220> gor b3501
<222> 1230-1329
<223> gor b3501 U00096 3643929..3645281
<313> 2411: 1273-1292
<400> 2411
TGGTATGGAC GAAATGTTGC AGGGCTTCGC GGTGGCGCTG AAGATGGGGG 50
CAACCAAAAA AGACTTCGAC AATACCGTCG CCATTCACCC AACGGCGGCA 100
<210> 2412
<211> 100
<212> DNA
<213> Escherichia coli K-12 MG1655
<220> gor b3501
<222> 1197-1296
<223> gor b3501 U00096 3643929..3645281
<313> 2412: 1241-1260
<400> 2412
AGAGAAGATT GTCGGTATTC ACGGCATTGG CTTTGGTATG GACGAAATGT 50
TGCAGGGCTT CGCGGTGGCG CTGAAGATGG GGGCAACCAA AAAAGACTTC 100
<210> 2413
<211> 100
<212> DNA
<213> Escherichia coli K-12 MG1655
<220> arsb b3503
<222> 1208-1307
<223> arsb b3503 U00096 3646544..3647854
<313> 2413: 1248-1267
<400> 2413
CTATCAGCTG GGGATATTAC TTCCGTACAG GGATTATCAT GACCCTGCCT 50
GTGCTGTTTG TGACGCTGGC TGCGCTGGCG CTACGTCTCT CTTTCACTTT 100
<210> 2414
<211> 100
<212> DNA
<213> Escherichia coli K-12 MG1655
<220> arsb b3503
<222> 1151-1250
<223> arsb b3503 U00096 3646544..3647854

<313> 2414: 1171-1190
<400> 2414
CAATTGGTAG CCTGGCTACG CTACTCTGGC TGCACGTACT TTCGCAGAAG 50
AATATGACTA TCAGCTGGGG ATATTACTTC CGTACAGGGA TTATCATGAC 100
<210> 2415
<211> 100
<212> DNA
<213> Escherichia coli K-12 MG1655
<220> arsb b3503
<222> 1068-1167
<223> arsb b3503 U00096 3646544..3647854
<313> 2415: 1088-1107
<400> 2415
TGGCAGCACG GCATCTGGCG TTATCAAAGA AGCGATGGTT TATGCCAATG 50
TGATTGGCTG CGATTTGGGA CCGAAAATTA CCCCAATTGG TAGCCTGGCT 100
<210> 2416
<211> 100
<212> DNA
<213> Escherichia coli K-12 MG1655
<220> rnha b0215
<222> 369-468
<223> rnha b0215 U00096 complement(235535..236002)
<313> 2416: 442-461
<400> 2416
CCATGCCGGA CACCCGGAAA ACGAACGCTG TGATGAACTG GCTCGTGCCG 50
CGGCGATGAA TCCCACACTG GAAGATACAG GCTACCAAGT TGAAGTTTAA 100
<210> 2417
<211> 100
<212> DNA
<213> Escherichia coli K-12 MG1655
<220> rnha b0215
<222> 347-446
<223> rnha b0215 U00096 complement(235535..236002)
<313> 2417: 381-400
<400> 2417
TCAAATGGGA ATGGGTTAAA GGCCATGCCG GACACCCGGA AAACGAACGC 50
TGTGATGAAC TGGCTCGTGC CGCGGCGATG AATCCCACAC TGGAAGATAC 100
<210> 2418
<211> 100
<212> DNA
<213> Escherichia coli K-12 MG1655
<220> rnha b0215
<222> 325-424
<223> rnha b0215 U00096 complement(235535..236002)
<313> 2418: 354-373
<400> 2418
GCTGCATTGG GGCAGCATCA AATCAAATGG GAATGGGTTA AAGGCCATGC 50
CGGACACCCG GAAAACGAAC GCTGTGATGA ACTGGCTCGT GCCGCGGCGA 100
<210> 2419
<211> 100
<212> DNA
<213> Escherichia coli K-12 MG1655
<220> arsc b3504
<222> 327-426
<223> arsc b3504 U00096 3647867..3648292
<313> 2419: 396-415
<400> 2419
AGAAGTGGTG CTGGAAATTC TGCCAGATGC GCAAAAAGGC GCATTCTCCA 50
AGGAAGATGG CGAGAAAGTG GTTGATGAAG CGGGTAAGCG CCTGAAATAA 100
<210> 2420
<211> 100
<212> DNA
<213> Escherichia coli K-12 MG1655
<220> arsc b3504
<222> 271-370
<223> arsc b3504 U00096 3647867..3648292
<313> 2420: 319-338
<400> 2420
CTGATTAATC GCCCGATTGT GGTGACGCCG CTGGGAACTC GCCTGTGCCG 50
CCCTTCAGAA GTGGTGCTGG AAATTCTGCC AGATGCGCAA AAAGGCGCAT 100
<210> 2421

<211> 100
<212> DNA
<213> Escherichia coli K-12 MG1655
<220> arsc b3504
<222> 266-365
<223> arsc b3504 U00096 3647867..3648292
<313> 2421: 287-306
<400> 2421
CGATTCTGAT TAATCGCCCG ATTGTGGTGA CGCCGCTGGG AACTCGCCTG 50
TGCCGCCCTT CAGAAGTGGT GCTGGAAATT CTGCCAGATG CGCAAAAAGG 100
<210> 2422
<211> 100
<212> DNA
<213> Escherichia coli K-12 MG1655
<220> rnhb b0184
<222> 479-578
<223> rnhb b0184 U00096 204493..205089
<313> 2422: 528-547
<400> 2422
CCCAACACAA AGGGTACCCA ACCGCTTTTC ATCTGGAAAA ACTGGCTGAA 50
CACGGCGCGA CCGAACACCA TCGGCGCAGC TTTGGGCCTG TCAAACGCGC 100
<210> 2423
<211> 100
<212> DNA
<213> Escherichia coli K-12 MG1655
<220> rnhb b0184
<222> 433-532
<223> rnhb b0184 U00096 204493..205089
<313> 2423: 460-479
<400> 2423
GCCGAAATGG CGGCGCTGGA TATTGTTTTC CCGCAATATG GTTTTGCCCA 50
ACACAAAGGG TACCCAACCG CTTTTCATCT GGAAAAACTG GCTGAACACG 100
<210> 2424
<211> 100
<212> DNA
<213> Escherichia coli K-12 MG1655
<220> rnhb b0184
<222> 375-474
<223> rnhb b0184 U00096 204493..205089
<313> 2424: 393-412
<400> 2424
CGATAGCCGC GTACCGGAAA TCAGTGCCGC GTCTATCCTG GCGAAAGTGA 50
CGCGTGACGC CGAAATGGCG GCGCTGGATA TTGTTTTCCC GCAATATGGT 100
<210> 2425
<211> 100
<212> DNA
<213> Escherichia coli K-12 MG1655
<220> gada b3518
<222> 1302-1401
<223> gada b3518 U00096 complement(3663810..3665210)
<313> 2425: 1377-1396
<400> 2425
TGAACTGTTG CTGGAAGACT ACAAAGCCTC CCTGAAATAT CTCAGCGATC 50
ACCCGAAACT GCAGGGTATT GCCCAGCAGA ACAGCTTTAA ACACACCTGA 100
<210> 2426
<211> 100
<212> DNA
<213> Escherichia coli K-12 MG1655
<220> gada b3518
<222> 1149-1248
<223> gada b3518 U00096 complement(3663810..3665210)
<313> 2426: 1178-1197
<400> 2426
AGATGGTGAA GATCCGGGAT ACACCCTGTA CGACCTCTCT GAACGTCTGC 50
GTCTGCGCGG CTGGCAGGTT CCGGCCTTCA CTCTCGGCGG TGAAGCCACC 100
<210> 2427
<211> 100
<212> DNA
<213> Escherichia coli K-12 MG1655
<220> gada b3518
<222> 619-718

<223> gada b3518 U00096 complement(3663810..3665210)
<313> 2427: 667-686
<400> 2427
CCGACTTTCG GCGTGACCTA CACCGGTAAC TATGAGTTCC CACAACCGCT 50
GCACGATGCG CTGGATAAAT TCCAGGCCGA CACCGGTATC GACATCGACA 100
<210> 2428
<211> 100
<212> DNA
<213> Escherichia coli K-12 MG1655
<220> gadb b1494
<222> 1302-1401
<223> gadb b1494 U00096 complement(1568669..1570069)
<313> 2428: 1379-1398
<400> 2428
TGAACTGTTG CTGGAAGACT ACAAAGCCTC CCTGAAATAT CTCAGCGATC 50
ACCCGAAACT GCAGGGTATT GCCCAACAGA ACAGCTTTAA ACATACCTGA 100
<210> 2429
<211> 100
<212> DNA
<213> Escherichia coli K-12 MG1655
<220> gadb b1494
<222> 1154-1253
<223> gadb b1494 U00096 complement(1568669..1570069)
<313> 2429: 1180-1199
<400> 2429
GTGAAGATCC GGGATACACC CTGTATGACC TCTCTGAACG TCTGCGTCTG 50
CGCGGCTGGC AGGTTCCGGC CTTCACTCTC GGCGGTGAAG CCACCGACAT 100
<210> 2430
<211> 100
<212> DNA
<213> Escherichia coli K-12 MG1655
<220> gadb b1494
<222> 644-743
<223> gadb b1494 U00096 complement(1568669..1570069)
<313> 2430: 682-701
<400> 2430
GTAACTATGA GTTCCCACAA CCGCTGCACG ATGCGCTGGA TAAATTCCAG 50
GCCGATACCG GTATCGACAT CGACATGCAC ATCGACGCTG CCAGCGGTGG 100
<210> 2431
<211> 100
<212> DNA
<213> Escherichia coli K-12 MG1655
<220> acra b0464
<222> 1095-1194
<223> acra b0464 U00096 complement(483650..484843)
<313> 2431: 1170-1189
<400> 2431
GAAAGTGCGT CCTGGTGTCC AGGTAAAAGC ACAAGAAGTT ACCGCTGATA 50
ATAACCAGCA AGCCGCAAGC GGTGCTCAGC CTGAACAGTC CAAGTCTTAA 100
<210> 2432
<211> 100
<212> DNA
<213> Escherichia coli K-12 MG1655
<220> acra b0464
<222> 1062-1161
<223> acra b0464 U00096 complement(483650..484843)
<313> 2432: 1120-1139
<400> 2432
AGCAGGCGAT CGCGTAGTAA TAAGTGGGCT GCAGAAAGTG CGTCCTGGTG 50
TCCAGGTAAA AGCACAAGAA GTTACCGCTG ATAATAACCA GCAAGCCGCA 100
<210> 2433
<211> 100
<212> DNA
<213> Escherichia coli K-12 MG1655
<220> acra b0464
<222> 1010-1109
<223> acra b0464 U00096 complement(483650..484843)
<313> 2433: 1049-1068
<400> 2433
TCGTTGCAAG CCAGGCTATT GGCGATAAGT GGCTGGTGAC AGAAGGTCTG 50
AAAGCAGGCG ATCGCGTAGT AATAAGTGGG CTGCAGAAAG TGCGTCCTGG 100

<210> 2434
<211> 100
<212> DNA
<213> Escherichia coli K-12 MG1655
<220> arsr b3502
<222> 207-306
<223> arsr b3502 U00096 3646158..3646511
<313> 2434: 232-251
<400> 2434
TCATTACCGC TTATCACCGC ATATTCCAGC ATGGGCGGCG AAAATTATTG 50
ATGAGGCCTG GCGATGTGAA CAGGAAAAGG TTCAGGCGAT TGTCCGCAAC 100
<210> 2435
<211> 100
<212> DNA
<213> Escherichia coli K-12 MG1655
<220> arsr b3502
<222> 149-248
<223> arsr b3502 U00096 3646158..3646511
<313> 2435: 181-200
<400> 2435
ACCTGGCATT GCTGCGTGAA AGCGGGCTAT TGCTGGACCG CAAGCAAGGT 50
AAGTGGGTTC ATTACCGCTT ATCACCGCAT ATTCCAGCAT GGGCGGCGAA 100
<210> 2436
<211> 100
<212> DNA
<213> Escherichia coli K-12 MG1655
<220> arsr b3502
<222> 96-195
<223> arsr b3502 U00096 3646158..3646511
<313> 2436: 138-157
<400> 2436
CGTCTGCGAT CTCTGCACTG CTCTCGACCA GTCGCAGCCC AAGATCTCCC 50
GCCACCTGGC ATTGCTGCGT GAAAGCGGGC TATTGCTGGA CCGCAAGCAA 100
<210> 2437
<211> 100
<212> DNA
<213> Escherichia coli K-12 MG1655
<220> acrb b0463
<222> 3051-3150
<223> acrb b0463 U00096 complement(480478..483627)
<313> 2437: 3105-3124
<400> 2437
GGCAATCTTC TTCGTTCCGG TATTCTTTGT GGTGGTTCGC CGCCGCTTTA 50
GCCGCAAGAA TGAAGATATC GAGCACAGCC ATACTGTCGA TCATCATTGA 100
<210> 2438
<211> 100
<212> DNA
<213> Escherichia coli K-12 MG1655
<220> acrb b0463
<222> 3002-3101
<223> acrb b0463 U00096 complement(480478..483627)
<313> 2438: 3064-3083
<400> 2438
ACGCAGTAGG TACCGGTGTA ATGGGCGGGA TGGTGACCGC AACGGTACTG 50
GCAATCTTCT TCGTTCCGGT ATTCTTTGTG GTGGTTCGCC GCCGCTTTAG 100
<210> 2439
<211> 100
<212> DNA
<213> Escherichia coli K-12 MG1655
<220> acrb b0463
<222> 2833-2932
<223> acrb b0463 U00096 complement(480478..483627)
<313> 2439: 2892-2911
<400> 2439
ATCGTCGAAT TCGCCAAAGA CTTGATGGAT AAAGAAGGTA AAGGTCTGAT 50
TGAAGCGACG CTTGATGCGG TGCGGATGCG TTTACGTCCG ATCCTGATGA 100
<210> 2440
<211> 100
<212> DNA
<213> Escherichia coli K-12 MG1655
<220> acrd b2471

<222> 2987-3086
<223> acrd b2471 U00096 2585615..2588728
<313> 2440: 3044-3063
<400> 2440
GTCAGCATGC GGTGGGTACT GGCGTAATGG GCGGGATGAT TTCGGCCACT 50
ATTCTGGCTA TTTACTTCGT GCCGCTGTTC TTTGTGCTGG TGCGCCGCCG 100
<210> 2441
<211> 100
<212> DNA
<213> Escherichia coli K-12 MG1655
<220> acrd b2471
<222> 2951-3050
<223> acrd b2471 U00096 2585615..2588728
<313> 2441: 2994-3013
<400> 2441
TGCCAATGGC AACCAGCACG GGTGCCGGTT CCGGTGGTCA GCATGCGGTG 50
GGTACTGGCG TAATGGGCGG GATGATTTCG GCCACTATTC TGGCTATTTA 100
<210> 2442
<211> 100
<212> DNA
<213> Escherichia coli K-12 MG1655
<220> acrd b2471
<222> 2904-3003
<223> acrd b2471 U00096 2585615..2588728
<313> 2442: 2969-2988
<400> 2442
TTTACGCCCG ATTCTGATGA CCTCGCTGGC ATTTATCTTC GGCGTATTGC 50
CAATGGCAAC CAGCACGGGT GCCGGTTCCG GTGGTCAGCA TGCGGTGGGT 100
<210> 2443
<211> 100
<212> DNA
<213> Escherichia coli K-12 MG1655
<220> acre b3266
<222> 1059-1158
<223> acre b3266 U00096 3411501..3412658
<313> 2443: 1123-1142
<400> 2443
ATCTGGCGAT CAAGTCATTG TCAGCGGCCT GCAAAAAGCG CGTCCGGGAG 50
AGCAGGTTAA AGCCACTACC GATACCCCCG CAGATACTGC ATCGAAGTAA 100
<210> 2444
<211> 100
<212> DNA
<213> Escherichia coli K-12 MG1655
<220> acre b3266
<222> 1049-1148
<223> acre b3266 U00096 3411501..3412658
<313> 2444: 1074-1093
<400> 2444
AAGGACTGAA ATCTGGCGAT CAAGTCATTG TCAGCGGCCT GCAAAAAGCG 50
CGTCCGGGAG AGCAGGTTAA AGCCACTACC GATACCCCCG CAGATACTGC 100
<210> 2445
<211> 100
<212> DNA
<213> Escherichia coli K-12 MG1655
<220> acre b3266
<222> 1000-1099
<223> acre b3266 U00096 3411501..3412658
<313> 2445: 1023-1042
<400> 2445
CGCCCTGTCG TTGCCAGTCA GGCGATTGGC GATAAATGGT TGATTAGTGA 50
AGGACTGAAA TCTGGCGATC AAGTCATTGT CAGCGGCCTG CAAAAAGCGC 100
<210> 2446
<211> 100
<212> DNA
<213> Escherichia coli K-12 MG1655
<220> acrf b3267
<222> 2966-3065
<223> acrf b3267 U00096 3412670..3415774
<313> 2446: 3016-3035
<400> 2446
CTATCAGTAA CGGTGCCGGC AGTGGCGCGC AGAACGCTGT GGGTATCGGG 50

GTAATGGGAG GAATGGTCTC TGCAACGTTG CTGGCAATCT TCTTCGTACC 100
<210> 2447
<211> 100
<212> DNA
<213> Escherichia coli K-12 MG1655
<220> acrf b3267
<222> 2902-3001
<223> acrf b3267 U00096 3412670..3415774
<313> 2447: 2930-2949
<400> 2447
CGTATGCGTC TGCGTCCTAT CCTGATGACC TCTCTCGCCT TTATTCTCGG 50
CGTATTACCG CTAGCTATCA GTAACGGTGC CGGCAGTGGC GCGCAGAACG 100
<210> 2448
<211> 100
<212> DNA
<213> Escherichia coli K-12 MG1655
<220> acrf b3267
<222> 2795-2894
<223> acrf b3267 U00096 3412670..3415774
<313> 2448: 2816-2835
<400> 2448
CGACAATTGG CTTGTCGGCC AAAAACGCTA TTTTGATCGT TGAGTTCGCT 50
AAAGATCTCA TGGAGAAAGA GGGTAAAGGT GTTGTTGAAG CGACACTGAT 100
<210> 2449
<211> 100
<212> DNA
<213> Escherichia coli K-12 MG1655
<220> gph b3386
<222> 660-759
<223> gph b3386 U00096 complement(3511269..3512027)
<313> 2449: 723-742
<400> 2449
CGATCTCAGC CAGCCTGATG TAATTTATCA GTCTATAAAT GACCTTCTGC 50
CCGCATTAGG GCTTCCGCAT AGCGAAAATC AGGAATCGAA AAATGACTAA 100
<210> 2450
<211> 100
<212> DNA
<213> Escherichia coli K-12 MG1655
<220> gph b3386
<222> 613-712
<223> gph b3386 U00096 complement(3511269..3512027)
<313> 2450: 651-670
<400> 2450
TGCCCATCAG TTGGCTTAAC CTACGGATAT AACTACGGCG AGGCTATCGA 50
TCTCAGCCAG CCTGATGTAA TTTATCAGTC TATAAATGAC CTTCTGCCCG 100
<210> 2451
<211> 100
<212> DNA
<213> Escherichia coli K-12 MG1655
<220> gph b3386
<222> 575-674
<223> gph b3386 U00096 complement(3511269..3512027)
<313> 2451: 609-628
<400> 2451
ACTCACGCAA TGATATTCAG GCGGCAAAAG CGGCAGGTTG CCCATCAGTT 50
GGCTTAACCT ACGGATATAA CTACGGCGAG GCTATCGATC TCAGCCAGCC 100
<210> 2452
<211> 100
<212> DNA
<213> Escherichia coli K-12 MG1655
<220> acrr b0465
<222> 549-648
<223> acrr b0465 U00096 484985..485632
<313> 2452: 623-642
<400> 2452
ATCTTTTGAT CTTAAAAAAG AAGCCCGCGA TTACGTTGCC ATCTTACTGG 50
AGATGTATCT CCTGTGCCCC ACGCTTCGTA ATCCTGCCAC TAACGAATAA 100
<210> 2453
<211> 100
<212> DNA
<213> Escherichia coli K-12 MG1655

<220> acrr b0465
<222> 546-645
<223> acrr b0465 U00096 484985..485632
<313> 2453: 579-598
<400> 2453
GCAATCTTTT GATCTTAAAA AAGAAGCCCG CGATTACGTT GCCATCTTAC  50
TGGAGATGTA TCTCCTGTGC CCCACGCTTC GTAATCCTGC CACTAACGAA  100
<210> 2454
<211> 100
<212> DNA
<213> Escherichia coli K-12 MG1655
<220> acrr b0465
<222> 478-577
<223> acrr b0465 U00096 484985..485632
<313> 2454: 526-545
<400> 2454
ACGCGTCGCG CAGCAATTAT TATGCGCGGC TATATTTCCG GCCTGATGGA  50
AAACTGGCTC TTTGCCCCGC AATCTTTTGA TCTTAAAAAA GAAGCCCGCG  100
<210> 2455
<211> 100
<212> DNA
<213> Escherichia coli K-12 MG1655
<220> gpt b0239
<222> 360-459
<223> gpt b0239 U00096 255977..256435
<313> 2455: 429-448
<400> 2455
GCTGGTTGAT GACTATGTTG TTGATATCCC GCAAGATACC TGGATTGAAC  50
AGCCGTGGGA TATGGGCGTC GTATTCGTCC CGCCAATCTC CGGTCGCTAA  100
<210> 2456
<211> 100
<212> DNA
<213> Escherichia coli K-12 MG1655
<220> gpt b0239
<222> 331-430
<223> gpt b0239 U00096 255977..256435
<313> 2456: 392-411
<400> 2456
ACCATCTTCG CAAAACCGGC TGGTCGTCCG CTGGTTGATG ACTATGTTGT  50
TGATATCCCG CAAGATACCT GGATTGAACA GCCGTGGGAT ATGGGCGTCG  100
<210> 2457
<211> 100
<212> DNA
<213> Escherichia coli K-12 MG1655
<220> gpt b0239
<222> 301-400
<223> gpt b0239 U00096 255977..256435
<313> 2457: 330-349
<400> 2457
CGTGAAATGT ATCCAAAAGC GCACTTTGTC ACCATCTTCG CAAAACCGGC  50
TGGTCGTCCG CTGGTTGATG ACTATGTTGT TGATATCCCG CAAGATACCT  100
<210> 2458
<211> 100
<212> DNA
<213> Escherichia coli K-12 MG1655
<220> arti b0864
<222> 633-732
<223> arti b0864 U00096 complement(901480..902211)
<313> 2458: 686-705
<400> 2458
GGGCAACACT GAGCTGCAGC AGAAACTCAA CACTGCGCTG GAAAAAGTGA  50
AGAAAGATGG CACTTACGAA ACCATCTACA ACAAATGGTT CCAGAAGTAA  100
<210> 2459
<211> 100
<212> DNA
<213> Escherichia coli K-12 MG1655
<220> arti b0864
<222> 551-650
<223> arti b0864 U00096 complement(901480..902211)
<313> 2459: 598-617
<400> 2459

ATAACCCGAA ACTGGCGGCG GTGGGCGACA AAGTGACCGA TAAAGATTAC 50
TTCGGCACTG GCCTCGGCAT CGCGGTACGT CAGGGCAACA CTGAGCTGCA 100
<210> 2460
<211> 100
<212> DNA
<213> Escherichia coli K-12 MG1655
<220> arti b0864
<222> 504-603
<223> arti b0864 U00096 complement(901480..902211)
<313> 2460: 562-581
<400> 2460
CGACGGCGTC TTCGGTGACA CCGCAGTGGT CACTGAGTGG CTGAAAGATA 50
ACCCGAAACT GGCGGCGGTG GGCGACAAAG TGACCGATAA AGATTACTTC 100
<210> 2461
<211> 100
<212> DNA
<213> Escherichia coli K-12 MG1655
<220> plsb b4042
<222> 2385-2484
<223> plsb b4042 U00096 complement(4251622..4254105)
<313> 2461: 2450-2469
<400> 2461
CGAACCGGCA GAAACGATGA AGGTTTATCA GTTGCTGGCG GAGTTGATTA 50
CATCAGACGT GCGTTTGACG ATTGAGAGTG CGACGCAGGG CGAAGGGTAA 100
<210> 2462
<211> 100
<212> DNA
<213> Escherichia coli K-12 MG1655
<220> plsb b4042
<222> 2368-2467
<223> plsb b4042 U00096 complement(4251622..4254105)
<313> 2462: 2392-2411
<400> 2462
AGCGATAGCG GCGATGCCGA ACCGGCAGAA ACGATGAAGG TTTATCAGTT 50
GCTGGCGGAG TTGATTACAT CAGACGTGCG TTTGACGATT GAGAGTGCGA 100
<210> 2463
<211> 100
<212> DNA
<213> Escherichia coli K-12 MG1655
<220> plsb b4042
<222> 2283-2382
<223> plsb b4042 U00096 complement(4251622..4254105)
<313> 2463: 2342-2361
<400> 2463
GCTGCACGGC ATCAACGCGC CGGAGTTCTT CGACAAGGCG GTGTTCAGTT 50
CTCTGGTGCT GACACTGCGT GATGAAGGGT ATATCAGCGA TAGCGGCGAT 100
<210> 2464
<211> 100
<212> DNA
<213> Escherichia coli K-12 MG1655
<220> artj b0861
<222> 633-732
<223> artj b0861 U00096 complement(899067..899798)
<313> 2464: 671-690
<400> 2464
GGATAACAAA GCCCTGCTGG AAAAACTGAA TAACGCGCTG GCAGCAATTA 50
AAGCTGACGG TACTTATCAA AAAATCAGTG ACCAGTGGTT CCCACAGTAA 100
<210> 2465
<211> 100
<212> DNA
<213> Escherichia coli K-12 MG1655
<220> artj b0861
<222> 560-659
<223> artj b0861 U00096 complement(899067..899798)
<313> 2465: 587-606
<400> 2465
AACTTGGTGT TGCTACTGAG AAAGTGACCG ATCCGCAATA TTTTGGCACC 50
GGCCTGGGCA TCGCTGTACG TCCGGATAAC AAAGCCCTGC TGGAAAAACT 100
<210> 2466
<211> 100
<212> DNA

<213> Escherichia coli K-12 MG1655
<220> artj b0861
<222> 518-617
<223> artj b0861 U00096 complement(899067..899798)
<313> 2466: 553-572
<400> 2466
GTGACACAGC GGTGGTAAAC GAATGGCTGA AAACCAATCC ACAACTTGGT 50
GTTGCTACTG AGAAAGTGAC CGATCCGCAA TATTTTGGCA CCGGCCTGGG 100
<210> 2467
<211> 100
<212> DNA
<213> Escherichia coli K-12 MG1655
<220> plsc b3019
<222> 639-738
<223> plsc b3019 U00096 complement(3160760..3161497)
<313> 2467: 702-721
<400> 2467
GGTTCGTGAG CTGGCTGCCC ATTGTCGTTC GATAATGGAA CAAAAAATCG 50
CCGAGCTCGA TAAAGAAGTC GCAGAACGCG AAGCCGCCGG AAAAGTTTAA 100
<210> 2468
<211> 100
<212> DNA
<213> Escherichia coli K-12 MG1655
<220> plsc b3019
<222> 422-521
<223> plsc b3019 U00096 complement(3160760..3161497)
<313> 2468: 485-504
<400> 2468
CCATCTGGAT GTTCCCGGAA GGAACCCGCA GCCGTGGTCG CGGCCTGCTA 50
CCGTTCAAGA CTGGAGCATT TCACGCGGCA ATTGCGGCGG GCGTCCCGAT 100
<210> 2469
<211> 100
<212> DNA
<213> Escherichia coli K-12 MG1655
<220> plsc b3019
<222> 355-454
<223> plsc b3019 U00096 complement(3160760..3161497)
<313> 2469: 383-402
<400> 2469
AACAATCGCA CTAAAGCTCA CGGCACCATT GCGGAAGTAG TGAATCACTT 50
CAAAAAACGC CGTATTTCCA TCTGGATGTT CCCGGAAGGA ACCCGCAGCC 100
<210> 2470
<211> 100
<212> DNA
<213> Escherichia coli K-12 MG1655
<220> artm b0862
<222> 520-619
<223> artm b0862 U00096 complement(900089..900757)
<313> 2470: 582-601
<400> 2470
ATGGGATACA GCCAGTTGTT GTACGGACGC ACCTACGATG TAATGGTGTT 50
CGGTGCGGCA GGGATTATTT ACCTGGTCGT TAACGGCCTG CTGACGCTGA 100
<210> 2471
<211> 100
<212> DNA
<213> Escherichia coli K-12 MG1655
<220> artm b0862
<222> 501-600
<223> artm b0862 U00096 complement(900089..900757)
<313> 2471: 532-551
<400> 2471
CATTACGCTG ATGGAAGTGA TGGGATACAG CCAGTTGTTG TACGGACGCA 50
CCTACGATGT AATGGTGTTC GGTGCGGCAG GGATTATTTA CCTGGTCGTT 100
<210> 2472
<211> 100
<212> DNA
<213> Escherichia coli K-12 MG1655
<220> artm b0862
<222> 456-555
<223> artm b0862 U00096 complement(900089..900757)
<313> 2472: 489-508

<400> 2472
CAACGAAGTG GTGCTGGTAT TCAAAAGTAC CTCTCTGGCA TACACCATTA 50
CGCTGATGGA AGTGATGGGA TACAGCCAGT TGTTGTACGG ACGCACCTAC 100
<210> 2473
<211> 100
<212> DNA
<213> Escherichia coli K-12 MG1655
<220> b0795 b0796
<222> 900-999
<223> b0795 b0796 U00096 complement(828197..829195)
<313> 2473: 979-998
<400> 2473
CGTCTATCGC CTGCGTATTG TGGTGACCGA CGCCGATGAT GCGTTACGCC 50
AGGGAATGCC AGTGACGGTA CAATTCGGTG ACGAGGCAGG ACATGAATGA 100
<210> 2474
<211> 100
<212> DNA
<213> Escherichia coli K-12 MG1655
<220> b0795 b0796
<222> 881-980
<223> b0795 b0796 U00096 complement(828197..829195)
<313> 2474: 903-922
<400> 2474
CGGATCTGCG TACCGACCTC GTCTATCGCC TGCGTATTGT GGTGACCGAC 50
GCCGATGATG CGTTACGCCA GGGAATGCCA GTGACGGTAC AATTCGGTGA 100
<210> 2475
<211> 100
<212> DNA
<213> Escherichia coli K-12 MG1655
<220> b0795 b0796
<222> 836-935
<223> b0795 b0796 U00096 complement(828197..829195)
<313> 2475: 848-867
<400> 2475
TCGTTTCGCC GACTGCTGAA TTTACCCCGA AAACCGTCGA AACGCCGGAT 50
CTGCGTACCG ACCTCGTCTA TCGCCTGCGT ATTGTGGTGA CCGACGCCGA 100
<210> 2476
<211> 100
<212> DNA
<213> Escherichia coli K-12 MG1655
<220> artp b0865
<222> 630-729
<223> artp b0865 U00096 complement(902229..902957)
<313> 2476: 701-720
<400> 2476
AGTGGTGTAT ATGGAAAATG GTCATATCGT AGAACAAGGC GACGCGAGCT 50
GCTTTACCGA GCCGCAAACC GAAGCATTTA AAAACTATCT CTCTCACTAA 100
<210> 2477
<211> 100
<212> DNA
<213> Escherichia coli K-12 MG1655
<220> artp b0865
<222> 571-670
<223> artp b0865 U00096 complement(902229..902957)
<313> 2477: 621-640
<400> 2477
AATATTACCC AGGTGATCGT CACCCACGAA GTTGAAGTGG CGCGTAAAAC 50
CGCCAGCCGA GTGGTGTATA TGGAAAATGG TCATATCGTA GAACAAGGCG 100
<210> 2478
<211> 100
<212> DNA
<213> Escherichia coli K-12 MG1655
<220> artp b0865
<222> 525-624
<223> artp b0865 U00096 complement(902229..902957)
<313> 2478: 562-581
<400> 2478
AATTACGGCA CAAATCGTCA GCATCATTCG TGAGCTGGCA GAAACGAATA 50
TTACCCAGGT GATCGTCACC CACGAAGTTG AAGTGGCGCG TAAAACCGCC 100
<210> 2479
<211> 100

<212> DNA
<213> Escherichia coli K-12 MG1655
<220> artq b0863
<222> 602-701
<223> artq b0863 U00096 complement(900757..901473)
<313> 2479: 656-675
<400> 2479
CATTTACCTG GTACATTGTG GCGGCGGCGA TTTACCTGGT GATCACCCTG 50
CTCAGTCAGT ACATTCTCAA ACGCATTGAC CTGCGCGCGA CACGTTTTGA 100
<210> 2480
<211> 100
<212> DNA
<213> Escherichia coli K-12 MG1655
<220> artq b0863
<222> 589-688
<223> artq b0863 U00096 complement(900757..901473)
<313> 2480: 622-641
<400> 2480
CGTACCCAGG AACCATTTAC CTGGTACATT GTGGCGGCGG CGATTTACCT 50
GGTGATCACC CTGCTCAGTC AGTACATTCT CAAACGCATT GACCTGCGCG 100
<210> 2481
<211> 100
<212> DNA
<213> Escherichia coli K-12 MG1655
<220> artq b0863
<222> 520-619
<223> artq b0863 U00096 complement(900757..901473)
<313> 2481: 578-597
<400> 2481
AAAGATACCG CGCTGGTCAG TTTGATTAGT GTGAATGATT TAATGCTGCA 50
AACAAAAAGC ATCGCTACTC GTACCCAGGA ACCATTTACC TGGTACATTG 100
<210> 2482
<211> 100
<212> DNA
<213> Escherichia coli K-12 MG1655
<220> kdsa b1216
<222> 756-855
<223> kdsa b1216 U00096 1267388..1268242
<313> 2482: 793-812
<400> 2482
ATCCGCGCTG CCGCTGGCTA AACTGGAACC GTTCCTCAAG CAGATGAAAG 50
CGATTGATGA TCTGGTGAAA GGTTTCGAAG AACTGGATAC CAGCAAGTAA 100
<210> 2483
<211> 100
<212> DNA
<213> Escherichia coli K-12 MG1655
<220> kdsa b1216
<222> 734-833
<223> kdsa b1216 U00096 1267388..1268242
<313> 2483: 751-770
<400> 2483
AACATGCGAA ATGTGATGGT CCATCCGCGC TGCCGCTGGC TAAACTGGAA 50
CCGTTCCTCA AGCAGATGAA AGCGATTGAT GATCTGGTGA AAGGTTTCGA 100
<210> 2484
<211> 100
<212> DNA
<213> Escherichia coli K-12 MG1655
<220> kdsa b1216
<222> 643-742
<223> kdsa b1216 U00096 1267388..1268242
<313> 2484: 706-725
<400> 2484
GGTGGTCGTC GTGCTCAGGT GGCTGAGCTG GCACGAGCCG GTATGGCGGT 50
AGGTCTGGCG GGGCTGTTTA TTGAAGCGCA TCCGGATCCG GAACATGCGA 100
<210> 2485
<211> 100
<212> DNA
<213> Escherichia coli K-12 MG1655
<220> kdsb kdsB
<222> 591-690
<223> kdsb kdsB U00096 970075..970821

<313> 2485: 642-661
<400> 2485
CTGGCAGCCA AGTCCGTTAG AACACATCGA AATGTTAGAG CAGCTTCGTG 50
TTCTGTGGTA CGGCGAAAAA ATCCATGTTG CTGTTGCTCA GGAAGTTCCT 100
<210> 2486
<211> 100
<212> DNA
<213> Escherichia coli K-12 MG1655
<220> kdsb kdsB
<222> 553-652
<223> kdsb kdsB U00096 970075..970821
<313> 2486: 598-617
<400> 2486
TATGGCTACC GTGCAGGCTT TATCCGTCGT TACGTCAACT GGCAGCCAAG 50
TCCGTTAGAA CACATCGAAA TGTTAGAGCA GCTTCGTGTT CTGTGGTACG 100
<210> 2487
<211> 100
<212> DNA
<213> Escherichia coli K-12 MG1655
<220> kdsb kdsB
<222> 514-613
<223> kdsb kdsB U00096 970075..970821
<313> 2487: 562-581
<400> 2487
GAAACCGTTG GCGATAACTT CCTGCGTCAT CTTGGTATTT ATGGCTACCG 50
TGCAGGCTTT ATCCGTCGTT ACGTCAACTG GCAGCCAAGT CCGTTAGAAC 100
<210> 2488
<211> 100
<212> DNA
<213> Escherichia coli K-12 MG1655
<220> plsx b1091
<222> 942-1041
<223> plsx b1091 U00096 1146874..1147914
<313> 2488: 960-979
<400> 2488
GCAGCGACAA GTTCCTCAGC GAATTGCCGC TCGCCTGGAA TCTGTATACC 50
CAGCTGGTTT TGAGCTGCTG GACGGTGGCA AAAGCGGAAC TCTGCGGTAG 100
<210> 2489
<211> 100
<212> DNA
<213> Escherichia coli K-12 MG1655
<220> plsx b1091
<222> 882-981
<223> plsx b1091 U00096 1146874..1147914
<313> 2489: 921-940
<400> 2489
TCATGGTGCA GCCAATCAGC GAGCTTTTGC GGTCGCGATT GAACAGGCAG 50
TGCAGGCGGT GCAGCGACAA GTTCCTCAGC GAATTGCCGC TCGCCTGGAA 100
<210> 2490
<211> 100
<212> DNA
<213> Escherichia coli K-12 MG1655
<220> plsx b1091
<222> 856-955
<223> plsx b1091 U00096 1146874..1147914
<313> 2490: 891-910
<400> 2490
GGATTGCGCG GCACGGTGAT AAAAAGTCAT GGTGCAGCCA ATCAGCGAGC 50
TTTTGCGGTC GCGATTGAAC AGGCAGTGCA GGCGGTGCAG CGACAAGTTC 100
<210> 2491
<211> 100
<212> DNA
<213> Escherichia coli K-12 MG1655
<220> mukb b0925
<222> 4362-4461
<223> mukb b0925 U00096 975549..980009
<313> 2491: 4439-4458
<400> 2491
GAATACCGAA CACGTTCATG TCGTCGGCCT GCGAGGATTT GCGCCGCAAC 50
TCCCTGAAAC GCTTCCAGGA ACTGACGAAG CGCCTTCTCA GGCGAGTTAA 100
<210> 2492

<211> 100
<212> DNA
<213> Escherichia coli K-12 MG1655
<220> mukb b0925
<222> 4358-4457
<223> mukb b0925 U00096 975549..980009
<313> 2492: 4398-4417
<400> 2492
TCCAGAATAC CGAACACGTT CATGTCGTCG GCCTGCGAGG ATTTGCGCCG 50
CAACTCCCTG AAACGCTTCC AGGAACTGAC GAAGCGCCTT CTCAGGCGAG 100
<210> 2493
<211> 100
<212> DNA
<213> Escherichia coli K-12 MG1655
<220> mukb b0925
<222> 4310-4409
<223> mukb b0925 U00096 975549..980009
<313> 2493: 4346-4365
<400> 2493
ATATCAGCCC GGAGAAAGGC ACCACCTATA AACTGGTGCG TAAAGTCTTC 50
CAGAATACCG AACACGTTCA TGTCGTCGGC CTGCGAGGAT TTGCGCCGCA 100
<210> 2494
<211> 100
<212> DNA
<213> Escherichia coli K-12 MG1655
<220> muke b0924
<222> 579-678
<223> muke b0924 U00096 974872..975549
<313> 2494: 643-662
<400> 2494
TCGCCTGATT CGTGATGGCG AAGCAATGCC GATTGAAAAT CATCTGCAAC 50
TCAACGATGA AACCGAAGAG AATCAGCCAG ATAGCGGAGA GGAAGAATAA 100
<210> 2495
<211> 100
<212> DNA
<213> Escherichia coli K-12 MG1655
<220> muke b0924
<222> 553-652
<223> muke b0924 U00096 974872..975549
<313> 2495: 605-624
<400> 2495
GGCGACGATC CCCGTGAAGC ACAGCGTCGC CTGATTCGTG ATGGCGAAGC 50
AATGCCGATT GAAAATCATC TGCAACTCAA CGATGAAACC GAAGAGAATC 100
<210> 2496
<211> 100
<212> DNA
<213> Escherichia coli K-12 MG1655
<220> muke b0924
<222> 480-579
<223> muke b0924 U00096 974872..975549
<313> 2496: 505-524
<400> 2496
GTTTATGGGC CACGACAGCA GCAAGTTTCG CATTACCGAA TCGGTGTTCC 50
GCTTCGGAGC CGATGTGCGT GCTGGCGACG ATCCCCGTGA AGCACAGCGT 100
<210> 2497
<211> 100
<212> DNA
<213> Escherichia coli K-12 MG1655
<220> mukf b0923
<222> 1224-1323
<223> mukf b0923 U00096 973542..974864
<313> 2497: 1285-1304
<400> 2497
TGGCGTAGCG CAAGCAGATT TCACCGGACT GCCAGCGAAA TGGCAGCCGA 50
TTAATGATTA CGGAGCCAAG GTACAGGCGC ATGTCATCGA CAAATATTGA 100
<210> 2498
<211> 100
<212> DNA
<213> Escherichia coli K-12 MG1655
<220> mukf b0923
<222> 1186-1285

<223> mukf b0923 U00096 973542..974864
<313> 2498: 1241-1260
<400> 2498
GACGTTGCGC GTATTGTTAT TGATCAGGCG GTACGTCTTG GCGTAGCGCA  50
AGCAGATTTC ACCGGACTGC CAGCGAAATG GCAGCCGATT AATGATTACG  100
<210> 2499
<211> 100
<212> DNA
<213> Escherichia coli K-12 MG1655
<220> mukf b0923
<222> 1094-1193
<223> mukf b0923 U00096 973542..974864
<313> 2499: 1142-1161
<400> 2499
AACTTGCCGT GTACAAAACC AGACAAGTGC CGCTGGATCT TGGTCTGGTG  50
GTACGCGAAT ATCTGTCACA GTATCCGCGT GCACGTCACT TTGACGTTGC  100
<210> 2500
<211> 100
<212> DNA
<213> Escherichia coli K-12 MG1655
<220> lpxa b0182
<222> 690-789
<223> lpxa b0182 U00096 202560..203348
<313> 2500: 751-770
<400> 2500
TGAAGTGAAA CCGGAAATTG CTGAACTGGC GGAAACATAT CCGGAAGTGA  50
AAGCCTTTAC CGATTTCTTT GCACGCTCAA CGCGCGGTCT GATTCGTTAA  100
<210> 2501
<211> 100
<212> DNA
<213> Escherichia coli K-12 MG1655
<220> lpxa b0182
<222> 681-780
<223> lpxa b0182 U00096 202560..203348
<313> 2501: 706-725
<400> 2501
AACGCTCGAT GAAGTGAAAC CGGAAATTGC TGAACTGGCG GAAACATATC  50
CGGAAGTGAA AGCCTTTACC GATTTCTTTG CACGCTCAAC GCGCGGTCTG  100
<210> 2502
<211> 100
<212> DNA
<213> Escherichia coli K-12 MG1655
<220> lpxa b0182
<222> 420-519
<223> lpxa b0182 U00096 202560..203348
<313> 2502: 462-481
<400> 2502
GCTGGCGGGT CACGTATCGG TTGACGACTT CGCGATCATC GGCGGCATGA  50
CCGCAGTCCA TCAGTTCTGC ATCATTGGTG CGCACGTGAT GGTTGGCGGC  100
<210> 2503
<211> 100
<212> DNA
<213> Escherichia coli K-12 MG1655
<220> lpxb b0183
<222> 1050-1149
<223> lpxb b0183 U00096 203348..204496
<313> 2503: 1109-1128
<400> 2503
CAGCCACGCG ATGCACGATA CCTTCCGTGA ACTGCATCAG CAGATCCGCT  50
GCAATGCCGA TGAGCAGGCG GCACAAGCCG TTCTGGAGTT AGCACAATGA  100
<210> 2504
<211> 100
<212> DNA
<213> Escherichia coli K-12 MG1655
<220> lpxb b0183
<222> 1017-1116
<223> lpxb b0183 U00096 203348..204496
<313> 2504: 1062-1081
<400> 2504
GGCGCTGTTA CCGCTGTTGG CGAACGGGAA AACCAGCCAC GCGATGCACG  50
ATACCTTCCG TGAACTGCAT CAGCAGATCC GCTGCAATGC CGATGAGCAG  100

<210> 2505
<211> 100
<212> DNA
<213> Escherichia coli K-12 MG1655
<220> lpxb b0183
<222> 951-1050
<223> lpxb b0183 U00096 203348..204496
<313> 2505: 990-1009
<400> 2505
GCTGGCGGGC AGAGAGTTAG TCAAAGAATT ATTGCAGGAA GAGTGTGAGC 50
CGCAAAAACT GGCTGCGGCG CTGTTACCGC TGTTGGCGAA CGGGAAAACC 100
<210> 2506
<211> 100
<212> DNA
<213> Escherichia coli K-12 MG1655
<220> lpxc b0097
<222> 819-918
<223> lpxc b0097 U00096 106557..107474
<313> 2506: 880-899
<400> 2506
GGCTGTCCTG GCGAAACAGG AAGCCTGGGA ATATGTGACC TTCCAGGACG 50
ACGCAGAACT GCCGTTGGCC TTCAAAGCGC CTTCAGCTGT ACTGGCATAA 100
<210> 2507
<211> 100
<212> DNA
<213> Escherichia coli K-12 MG1655
<220> lpxc b0097
<222> 781-880
<223> lpxc b0097 U00096 106557..107474
<313> 2507: 831-850
<400> 2507
TATAAATCCG GTCATGCACT GAATAACAAA CTGCTGCAGG CTGTCCTGGC 50
GAAACAGGAA GCCTGGGAAT ATGTGACCTT CCAGGACGAC GCAGAACTGC 100
<210> 2508
<211> 100
<212> DNA
<213> Escherichia coli K-12 MG1655
<220> lpxc b0097
<222> 632-731
<223> lpxc b0097 U00096 106557..107474
<313> 2508: 667-686
<400> 2508
GCTTCGATTG TGCCATCGTT GTTGACGATT ATCGCGTACT GAACGAAGAC 50
GGCCTGCGTT TTGAAGACGA ATTTGTGCGT CACAAAATGC TCGATGCGAT 100
<210> 2509
<211> 100
<212> DNA
<213> Escherichia coli K-12 MG1655
<220> lpxd b0180
<222> 888-987
<223> lpxd b0180 U00096 200971..201996
<313> 2509: 935-954
<400> 2509
TGAACCAGGC GTCTATTCCT CAGGCATTCC GCTGCAACCC AACAAAGTCT 50
GGCGCAAAAC CGCTGCACTG GTGATGAACA TTGATGACAT GAGCAAGCGT 100
<210> 2510
<211> 100
<212> DNA
<213> Escherichia coli K-12 MG1655
<220> lpxd b0180
<222> 822-921
<223> lpxd b0180 U00096 200971..201996
<313> 2510: 848-867
<400> 2510
CGGGCATATG GAAATATGCG ACAAAGTGAC GGTTACGGGC ATGGGTATGG 50
TGATGCGTCC CATCACTGAA CCAGGCGTCT ATTCCTCAGG CATTCCGCTG 100
<210> 2511
<211> 100
<212> DNA
<213> Escherichia coli K-12 MG1655
<220> lpxd b0180

<222> 784-883
<223> lpxd b0180 U00096 200971..201996
<313> 2511: 815-834
<400> 2511
GGTCGTTACT GCATGATCGG CGGAGCCAGC GTAATCAACG GGCATATGGA  50
AATATGCGAC AAAGTGACGG TTACGGGCAT GGGTATGGTG ATGCGTCCCA  100
<210> 2512
<211> 100
<212> DNA
<213> Escherichia coli K-12 MG1655
<220> vsr b1961
<222> 372-471
<223> vsr b1961 U00096 complement(2028470..2028940)
<313> 2512: 439-458
<400> 2512
GCTGACGGAT GAAGCGCTTA CCGAGCGTCT GGAAGAGTGG ATCTGCGGCG  50
AAGGTGCCAG CGCGCAGATC GACACGCAGG GGATTCATTT ACTCGCTTGA  100
<210> 2513
<211> 100
<212> DNA
<213> Escherichia coli K-12 MG1655
<220> vsr b1961
<222> 194-293
<223> vsr b1961 U00096 complement(2028470..2028940)
<313> 2513: 237-256
<400> 2513
GCTGCTTCTG GCATCATCAT CACTGCTATC TGTTTAAAGT GCCTGCGACT  50
CGAACCGAGT TCTGGCTGGA GAAGATAGGT AAAAATGTTG AGCGCGATCG  100
<210> 2514
<211> 100
<212> DNA
<213> Escherichia coli K-12 MG1655
<220> vsr b1961
<222> 21-120
<223> vsr b1961 U00096 complement(2028470..2028940)
<313> 2514: 76-95
<400> 2514
GGCCACTCGC AGCAAAAATA TGCGCGCGAT TGCCACGCGT GATACGGCGA  50
TAGAGAAGCG CCTCGCCAGT CTGTTAACCG GGCAGGGCCT GGCATTTCGC  100
<210> 2515
<211> 100
<212> DNA
<213> Escherichia coli K-12 MG1655
<220> kdta b3634
<222> 1179-1278
<223> kdta b3634 U00096 3806167..3807444
<313> 2515: 1240-1259
<400> 2515
CTATGGCCGT CATGCCGTTG AAGTACTGTA TCAAAACCAG GGCGCGCTAC  50
AGCGTCTGCT TCAACTGCTG GAACCTTACC TGCCACCGAA AACGCATTGA  100
<210> 2516
<211> 100
<212> DNA
<213> Escherichia coli K-12 MG1655
<220> kdta b3634
<222> 1133-1232
<223> kdta b3634 U00096 3806167..3807444
<313> 2516: 1155-1174
<400> 2516
CAAAAGAGGT TTCCTCTTTA CTCACCGACG CCGATTACCG TAGTTTCTAT  50
GGCCGTCATG CCGTTGAAGT ACTGTATCAA AACCAGGGCG CGCTACAGCG  100
<210> 2517
<211> 100
<212> DNA
<213> Escherichia coli K-12 MG1655
<220> kdta b3634
<222> 1059-1158
<223> kdta b3634 U00096 3806167..3807444
<313> 2517: 1113-1132
<400> 2517
TAACTTTAAA GACATTTGCG CGCGGCTGGA GCAGGCAAGC GGGCTGATTA  50

CCGTTACCGA TGCCACTACG CTTGCAAAAG AGGTTTCCTC TTTACTCACC 100
<210> 2518
<211> 100
<212> DNA
<213> Escherichia coli K-12 MG1655
<220> kdtb b3635
<222> 381-480
<223> kdtb b3635 U00096 3807452..3807931
<313> 2518: 439-458
<400> 2518
CTCTTCATCG TTGGTGAAAG AGGTGGCGCG CCATCAGGGC GATGTCACCC 50
ATTTCCTGCC GGAGAATGTC CATCAGGCGC TGATGGCGAA GTTAGCGTAG 100
<210> 2519
<211> 100
<212> DNA
<213> Escherichia coli K-12 MG1655
<220> kdtb b3635
<222> 301-400
<223> kdtb b3635 U00096 3807452..3807931
<313> 2519: 340-359
<400> 2519
CAGCTGGCGC ATATGAATCG CCACTTAATG CCGGAACTGG AAAGTGTGTT 50
TCTGATGCCG TCGAAAGAGT GGTCGTTTAT CTCTTCATCG TTGGTGAAAG 100
<210> 2520
<211> 100
<212> DNA
<213> Escherichia coli K-12 MG1655
<220> kdtb b3635
<222> 163-262
<223> kdtb b3635 U00096 3807452..3807931
<313> 2520: 207-226
<400> 2520
GCACAGCAGG CAACCGCGCA TCTGGGGAAC GTGGAAGTGG TCGGGTTTAG 50
TGATTTAATG GCGAACTTCG CCCGTAATCA ACACGCTACG GTGCTGATTC 100
<210> 2521
<211> 100
<212> DNA
<213> Escherichia coli K-12 MG1655
<220> yiaa b3563
<222> 342-441
<223> yiaa b3563 U00096 complement(3724553..3724993)
<313> 2521: 415-434
<400> 2521
AAGCTTGTTT GGTGCAGTAG CGGTGCAGAA AAATATTCGT GATGCCGGAA 50
TAAACCCACC AAAAGAAACA CAGGTTACCC AGGAAGAATA CAGCGAATAA 100
<210> 2522
<211> 100
<212> DNA
<213> Escherichia coli K-12 MG1655
<220> yiaa b3563
<222> 317-416
<223> yiaa b3563 U00096 complement(3724553..3724993)
<313> 2522: 359-378
<400> 2522
GTTTTTATGG ACTGGCTTTC TTCTTAAGCT TGTTTGGTGC AGTAGCGGTG 50
CAGAAAAATA TTCGTGATGC CGGAATAAAC CCACCAAAAG AAACACAGGT 100
<210> 2523
<211> 100
<212> DNA
<213> Escherichia coli K-12 MG1655
<220> yiaa b3563
<222> 152-251
<223> yiaa b3563 U00096 complement(3724553..3724993)
<313> 2523: 202-221
<400> 2523
TAGGACTGTT TTCTGCGGCG TCTTATCAAA AGACCGTTCG GGACAAGTAT 50
GAAGGCATAC CGACCACTTC CATTTATTAT ATGACCTGCC TGACTGTCTT 100
<210> 2524
<211> 100
<212> DNA
<213> Escherichia coli K-12 MG1655

<220> yiab b3564
<222> 255-354
<223> yiab b3564 U00096 complement(3725036..3725389)
<313> 2524: 298-317
<400> 2524
TGTTTTAAAC GCACCTATCA ATACTTATGA AATGGTGATC TATCCCATCG  50
CCTTTTTTGT CTGCTTGTTT GGTCAAATGC GTTTGTTTCG CTCGGCATGA  100
<210> 2525
<211> 100
<212> DNA
<213> Escherichia coli K-12 MG1655
<220> yiab b3564
<222> 142-241
<223> yiab b3564 U00096 complement(3725036..3725389)
<313> 2525: 191-210
<400> 2525
ACTTTTGGCA ACTATGCATA TCTTCGCGCA GAAAAACTCG GGCAACTGGA  50
TGATTTTTTT ACCCATATCT GCCAGTTAGT TGCGTTAATC ACTATCGGTC  100
<210> 2526
<211> 100
<212> DNA
<213> Escherichia coli K-12 MG1655
<220> yiab b3564
<222> 59-158
<223> yiab b3564 U00096 complement(3725036..3725389)
<313> 2526: 95-114
<400> 2526
CGCTGGTTTA TCTGGTTGGG CTATGGATCT CATGCCCATT GTTAAGTGGA  50
AAAGGCTATT TTCTTGGCGT GTTAATGACA GCAACTTTTG GCAACTATGC  100
<210> 2527
<211> 100
<212> DNA
<213> Escherichia coli K-12 MG1655
<220> gsk b0478
<222> 1206-1305
<223> gsk b0478 U00096 499349..500653
<313> 2527: 1284-1303
<400> 2527
CCGTGTGAGC TATCAGGTAC TGAACCAGCA TTCACCTCGT TTAACGCGCG  50
GCTTGCCGGA GCGTGAAGAC AGCCTGGAAG AGTCTTACTG GGATCGTTAA  100
<210> 2528
<211> 100
<212> DNA
<213> Escherichia coli K-12 MG1655
<220> gsk b0478
<222> 1186-1285
<223> gsk b0478 U00096 499349..500653
<313> 2528: 1220-1239
<400> 2528
CAGGTGTGTA AATATGCTAA CCGTGTGAGC TATCAGGTAC TGAACCAGCA  50
TTCACCTCGT TTAACGCGCG GCTTGCCGGA GCGTGAAGAC AGCCTGGAAG  100
<210> 2529
<211> 100
<212> DNA
<213> Escherichia coli K-12 MG1655
<220> gsk b0478
<222> 1095-1194
<223> gsk b0478 U00096 499349..500653
<313> 2529: 1140-1159
<400> 2529
GCATGACATT ACCGCCAACA GCTACCATCG TAGCAACGTA CCAAACTCCA  50
GCAAACATAA ATTCACCTGG TTAACTTATT CATCGTTAGC GCAGGTGTGT  100
<210> 2530
<211> 100
<212> DNA
<213> Escherichia coli K-12 MG1655
<220> yiac b3551
<222> 342-441
<223> yiac b3551 U00096 3711281..3711721
<313> 2530: 392-411
<400> 2530

TTACCAGGCA CAGGGTTTTC ACATTGTCGA TTGCGCATGG CAGGATGAAA 50
CCCAACTACC CACATGGATT ATGAGCTGGC CGGTGGTTCA AACGCTGTAA 100
<210> 2531
<211> 100
<212> DNA
<213> Escherichia coli K-12 MG1655
<220> yiac b3551
<222> 315-414
<223> yiac b3551 U00096 3711281..3711721
<313> 2531: 347-366
<400> 2531
TCAAAAAAAT CAACCGGCGA TAAATTTTTA CCAGGCACAG GGTTTTCACA 50
TTGTCGATTG CGCATGGCAG GATGAAACCC AACTACCCAC ATGGATTATG 100
<210> 2532
<211> 100
<212> DNA
<213> Escherichia coli K-12 MG1655
<220> yiac b3551
<222> 274-373
<223> yiac b3551 U00096 3711281..3711721
<313> 2532: 315-334
<400> 2532
TATGTGCAGC AGCGCCATCC CCACCTGATG CTGGAGGTTT ATCAAAAAAA 50
TCAACCGGCG ATAAATTTTT ACCAGGCACA GGGTTTTCAC ATTGTCGATT 100
<210> 2533
<211> 100
<212> DNA
<213> Escherichia coli K-12 MG1655
<220> yiad b3553
<222> 561-660
<223> yiad b3553 U00096 3714176..3714835
<313> 2533: 628-647
<400> 2533
TACTCAGGGC CTTGGCCCGG CTAACCCAAT CGCCAGCAAC AGCACCGCAG 50
AAGGTAAGGC GCAAAACCGC CGTGTAGAAA TTACCTTAAG CCCGCTGTAA 100
<210> 2534
<211> 100
<212> DNA
<213> Escherichia coli K-12 MG1655
<220> yiad b3553
<222> 561-660
<223> yiad b3553 U00096 3714176..3714835
<313> 2534: 588-607
<400> 2534
TACTCAGGGC CTTGGCCCGG CTAACCCAAT CGCCAGCAAC AGCACCGCAG 50
AAGGTAAGGC GCAAAACCGC CGTGTAGAAA TTACCTTAAG CCCGCTGTAA 100
<210> 2535
<211> 100
<212> DNA
<213> Escherichia coli K-12 MG1655
<220> yiad b3553
<222> 508-607
<223> yiad b3553 U00096 3714176..3714835
<313> 2535: 535-554
<400> 2535
GATTCCGTTG CCAGCGCGTT GATCACCCAG GGCGTGGACG CCAGCCGCAT 50
CCGTACTCAG GGCCTTGGCC CGGCTAACCC AATCGCCAGC AACAGCACCG 100
<210> 2536
<211> 100
<212> DNA
<213> Escherichia coli K-12 MG1655
<220> yiae b3554
<222> 888-987
<223> yiae b3554 U00096 3714927..3715913
<313> 2536: 964-983
<400> 2536
GACGCGTTAT GGCATGGCCG CCTGTGCCGT GGATAATTTG ATTGATGCGT 50
TACAAGGAAA GGTTGAGAAG AACTGTGTGA ATCCGCACGT CGCGGACTAA 100
<210> 2537
<211> 100
<212> DNA

<213> Escherichia coli K-12 MG1655
<220> yiae b3554
<222> 808-907
<223> yiae b3554 U00096 3714927..3715913
<313> 2537: 859-878
<400> 2537
GAGCCACTGT CCGTAGATTC GCCGTTGCTC TCAATGGCCA ACGTCGTCGC 50
AGTACCGCAT ATTGGATCTG CCACCCATGA GACGCGTTAT GGCATGGCCG 100
<210> 2538
<211> 100
<212> DNA
<213> Escherichia coli K-12 MG1655
<220> yiae b3554
<222> 791-890
<223> yiae b3554 U00096 3714927..3715913
<313> 2538: 808-827
<400> 2538
TGGATGTCTT CGAACAAGAG CCACTGTCCG TAGATTCGCC GTTGCTCTCA 50
ATGGCCAACG TCGTCGCAGT ACCGCATATT GGATCTGCCA CCCATGAGAC 100
<210> 2539
<211> 100
<212> DNA
<213> Escherichia coli K-12 MG1655
<220> kdud b2843
<222> 652-751
<223> kdud b2843 U00096 complement(2980519..2981280)
<313> 2539: 707-726
<400> 2539
TGGGGACTGC CGAGTGACCT GATGGGGCCG ATAGTGTTCC TTGCCTCCAG 50
CGCTTCAGAT TATGTGAATG GTTATACCAT TGCCGTGGAT GGCGGTTGGC 100
<210> 2540
<211> 100
<212> DNA
<213> Escherichia coli K-12 MG1655
<220> kdud b2843
<222> 636-735
<223> kdud b2843 U00096 complement(2980519..2981280)
<313> 2540: 671-690
<400> 2540
CATTCCAGCT GGTCGTTGGG GACTGCCGAG TGACCTGATG GGGCCGATAG 50
TGTTCCTTGC CTCCAGCGCT TCAGATTATG TGAATGGTTA TACCATTGCC 100
<210> 2541
<211> 100
<212> DNA
<213> Escherichia coli K-12 MG1655
<220> kdud b2843
<222> 560-659
<223> kdud b2843 U00096 complement(2980519..2981280)
<313> 2541: 611-630
<400> 2541
CCCCGGGTTA CATGGCGACC AACAATACTC AACAACTACG GGCAGATGAA 50
CAACGTAGCG CGGAAATTCT CGACCGCATT CCAGCTGGTC GTTGGGGACT 100
<210> 2542
<211> 100
<212> DNA
<213> Escherichia coli K-12 MG1655
<220> yiaf b3555
<222> 732-831
<223> yiaf b3555 U00096 complement(3715963..3716793)
<313> 2542: 776-795
<400> 2542
ACAGCAGGCA AGTGAATATA ACAAACTGAT TGCGCCATTA CCGGCACAGC 50
ATCAGGCGTT TAATCAGGCC TGGACTACAG CAGTTACTGC AACCCAATAA 100
<210> 2543
<211> 100
<212> DNA
<213> Escherichia coli K-12 MG1655
<220> yiaf b3555
<222> 698-797
<223> yiaf b3555 U00096 complement(3715963..3716793)
<313> 2543: 721-740

<400> 2543
GCTTTGTCGC AAATGGCATC CAGTTCCCGA CTTCACAGCA GGCAAGTGAA  50
TATAACAAAC TGATTGCGCC ATTACCGGCA CAGCATCAGG CGTTTAATCA  100
<210> 2544
<211> 100
<212> DNA
<213> Escherichia coli K-12 MG1655
<220> yiaf b3555
<222> 661-760
<223> yiaf b3555 U00096 complement(3715963..3716793)
<313> 2544: 682-701
<400> 2544
GTTGGAGACT ACATTGCTCA GCAAGGTACT CAGGTGAGCT TTGTCGCAAA  50
TGGCATCCAG TTCCCGACTT CACAGCAGGC AAGTGAATAT AACAAACTGA  100
<210> 2545
<211> 100
<212> DNA
<213> Escherichia coli K-12 MG1655
<220> yiag b3556
<222> 192-291
<223> yiag b3556 U00096 3717107..3717397
<313> 2545: 267-286
<400> 2545
GGAATGGGAA TCCAGACGCG TGAAGCCTTC AAGTGCCGAA CTAAAATTGA  50
TGCGTTTGAT TCAAGCCAAC CCGGCATTAA GTAAGCAGTT GATGGAATAG  100
<210> 2546
<211> 100
<212> DNA
<213> Escherichia coli K-12 MG1655
<220> yiag b3556
<222> 63-162
<223> yiag b3556 U00096 3717107..3717397
<313> 2546: 116-135
<400> 2546
TGAAACGCAG AAAATTACCC TGACGCACAG AACAACGTCC TGTACCGAAA  50
TTGAGCAGTT ACGAAAAGGG ACAGGATTAA AAATCGATGA TTTCGCCCGG  100
<210> 2547
<211> 100
<212> DNA
<213> Escherichia coli K-12 MG1655
<220> yiag b3556
<222> 37-136
<223> yiag b3556 U00096 3717107..3717397
<313> 2547: 71-90
<400> 2547
AGCCTGGAAC AGATTGTTTT TAAAGATGAA ACGCAGAAAA TTACCCTGAC  50
GCACAGAACA ACGTCCTGTA CCGAAATTGA GCAGTTACGA AAAGGGACAG  100
<210> 2548
<211> 100
<212> DNA
<213> Escherichia coli K-12 MG1655
<220> gsp gsp
<222> 1585-1684
<223> gsp gsp U00096 complement(3134678..3136537)
<313> 2548: 1641-1660
<400> 2548
GGTTACGCAG TGAAACCGAT CGCCGGTCGC TGTGGCAGCA ATATCGACCT  50
CGTCAGCCAT CATGAAGAGG TGCTGGACAA AACCAGCGGT AAATTTGCCG  100
<210> 2549
<211> 100
<212> DNA
<213> Escherichia coli K-12 MG1655
<220> gsp gsp
<222> 1548-1647
<223> gsp gsp U00096 complement(3134678..3136537)
<313> 2549: 1577-1596
<400> 2549
TACCGATTTC ACTGTTAATG ATGAACTGGT GAAAACAGGT TACGCAGTGA  50
AACCGATCGC CGGTCGCTGT GGCAGCAATA TCGACCTCGT CAGCCATCAT  100
<210> 2550
<211> 100

<212> DNA
<213> Escherichia coli K-12 MG1655
<220> gsp gsp
<222> 1484-1583
<223> gsp gsp U00096 complement(3134678..3136537)
<313> 2550: 1526-1545
<400> 2550
CCGGCAACAA AGCGATTCTG CCGATCCTCT GGTCGCTGTT CCCGCACCAT 50
CGTTACCTGC TGGATACCGA TTTCACTGTT AATGATGAAC TGGTGAAAAC 100
<210> 2551
<211> 100
<212> DNA
<213> Escherichia coli K-12 MG1655
<220> yiah b3562
<222> 779-878
<223> yiah b3562 U00096 3723516..3724511
<313> 2551: 808-827
<400> 2551
ATACGCGTAC CATTCGCGGA CTTGGCTTAA TCTCCCGCCA TTCATTGGGT 50
ATATACGGAT TCCACGCCTT GATTATCCAT GCGCTGCGCA CCCGGGGAAT 100
<210> 2552
<211> 100
<212> DNA
<213> Escherichia coli K-12 MG1655
<220> yiah b3562
<222> 704-803
<223> yiah b3562 U00096 3723516..3724511
<313> 2552: 758-777
<400> 2552
CCTGGTATCT TTACTGTGGG CCGATGGTTT TTATCTGCGC AATCGCGCTA 50
TTGACTCTGG TTAAAAACAC GCTGGATACG CGTACCATTC GCGGACTTGG 100
<210> 2553
<211> 100
<212> DNA
<213> Escherichia coli K-12 MG1655
<220> yiah b3562
<222> 574-673
<223> yiah b3562 U00096 3723516..3724511
<313> 2553: 632-651
<400> 2553
GCTATAGGGA TGATGGACAC ACAGCATAAA GCACTGTCGT GGGTGAGCGC 50
CGCGCTGTTT GCGACGGGGG TTTTTATTAT CTCTCGCGGG ACATTATATG 100
<210> 2554
<211> 100
<212> DNA
<213> Escherichia coli K-12 MG1655
<220> yiai b3574
<222> 375-474
<223> yiai b3574 U00096 complement(3738738..3739211)
<313> 2554: 436-455
<400> 2554
GACGGGACCG GCCTGTGTTG AAGCCTGCCC GACAAAGGCG TTGCAGTGCA 50
TGGATGTCGA GAAAGTGCAG CGGCATCGGC TACGGCAGCA GCCTGTTTGA 100
<210> 2555
<211> 100
<212> DNA
<213> Escherichia coli K-12 MG1655
<220> yiai b3574
<222> 331-430
<223> yiai b3574 U00096 complement(3738738..3739211)
<313> 2555: 380-399
<400> 2555
AAAAAGGCGA GGGCGATTAA GTGCGATCTG TGCTGGCATC GGGAGACGGG 50
ACCGGCCTGT GTTGAAGCCT GCCCGACAAA GGCGTTGCAG TGCATGGATG 100
<210> 2556
<211> 100
<212> DNA
<213> Escherichia coli K-12 MG1655
<220> yiai b3574
<222> 280-379
<223> yiai b3574 U00096 complement(3738738..3739211)

<313> 2556: 335-354
<400> 2556
AGCTGTATGC TGGCTTGCCC GTTTGGTGCG ATGGAGGTCG TTTCTTCGCG 50
CAAAAAGGCG AGGGCGATTA AGTGCGATCT GTGCTGGCAT CGGGAGACGG 100
<210> 2557
<211> 100
<212> DNA
<213> Escherichia coli K-12 MG1655
<220> yiaj b3575
<222> 750-849
<223> yiaj b3575 U00096 complement(3739313..3740161)
<313> 2557: 817-836
<400> 2557
GGGAGAGAAA AATCTCCTGA AACCACTGCG TGAAACCGCG CAGGCTATTT 50
CTAATGAACT GGGATTTACT GTCAGGGATG ACCTGGGCGC GATCACATAA 100
<210> 2558
<211> 100
<212> DNA
<213> Escherichia coli K-12 MG1655
<220> yiaj b3575
<222> 686-785
<223> yiaj b3575 U00096 complement(3739313..3740161)
<313> 2558: 735-754
<400> 2558
ATATTCATGG GCGGGTGCCG TACGCCGTGT CGATTTCGCT TTCGACATCA 50
CGTCTGAAAC AGGTGGGAGA GAAAAATCTC CTGAAACCAC TGCGTGAAAC 100
<210> 2559
<211> 100
<212> DNA
<213> Escherichia coli K-12 MG1655
<220> yiaj b3575
<222> 635-734
<223> yiaj b3575 U00096 complement(3739313..3740161)
<313> 2559: 667-686
<400> 2559
ACAGAGAAGA AAACGAACTC GGCGTCTCCT GTATTGCTGT TCCGGTGTTT 50
GATATTCATG GGCGGGTGCC GTACGCCGTG TCGATTTCGC TTTCGACATC 100
<210> 2560
<211> 100
<212> DNA
<213> Escherichia coli K-12 MG1655
<220> kdui b2844
<222> 736-835
<223> kdui b2844 U00096 complement(2981310..2982146)
<313> 2560: 785-804
<400> 2560
TCCGGTGTCG GAACCAAAGC TTATACCTTT ATCTGGGGCA TGGTCGGTGA 50
AAACCAGGTC TTTGATGATA TGGACCATGT GGCCGTTAAA GATTTGCGCT 100
<210> 2561
<211> 100
<212> DNA
<213> Escherichia coli K-12 MG1655
<220> kdui b2844
<222> 668-767
<223> kdui b2844 U00096 complement(2981310..2982146)
<313> 2561: 707-726
<400> 2561
CGCAAGAAAC GCGTCATATT GTGATGCATA ACGAGCAGGC GGTGATCTCC 50
CCGAGCTGGT CGATCCATTC CGGTGTCGGA ACCAAAGCTT ATACCTTTAT 100
<210> 2562
<211> 100
<212> DNA
<213> Escherichia coli K-12 MG1655
<220> kdui b2844
<222> 600-699
<223> kdui b2844 U00096 complement(2981310..2982146)
<313> 2562: 656-675
<400> 2562
CCGGATGGAA GTTTATTTCT ATTTCAATAT GGATGATGAC GCCTGCGTTT 50
TCCACATGAT GGGGCAGCCG CAAGAAACGC GTCATATTGT GATGCATAAC 100
<210> 2563

<211> 100
<212> DNA
<213> Escherichia coli K-12 MG1655
<220> yiak b3576
<222> 900-999
<223> yiak b3576 U00096 3740362..3741360
<313> 2563: 962-981
<400> 2563
TCGCTTACCC GGCCATGAAT TTACTACCCT GCTGGCCGAA AACCGCCGTA 50
ACGGCATCAC TGTTGATGAC AGCGTGTGGG CCAAAATCCA GGCGTTATGA 100
<210> 2564
<211> 100
<212> DNA
<213> Escherichia coli K-12 MG1655
<220> yiak b3576
<222> 894-993
<223> yiak b3576 U00096 3740362..3741360
<313> 2564: 938-957
<400> 2564
GGCCATTCGC TTACCCGGCC ATGAATTTAC TACCCTGCTG GCCGAAAACC 50
GCCGTAACGG CATCACTGTT GATGACAGCG TGTGGGCCAA AATCCAGGCG 100
<210> 2565
<211> 100
<212> DNA
<213> Escherichia coli K-12 MG1655
<220> yiak b3576
<222> 850-949
<223> yiak b3576 U00096 3740362..3741360
<313> 2565: 892-911
<400> 2565
ATCATGGATT ACGTTACTAG TGCCGAGCGT GCTGACGAAA ATCAGGCCAT 50
TCGCTTACCC GGCCATGAAT TTACTACCCT GCTGGCCGAA AACCGCCGTA 100
<210> 2566
<211> 100
<212> DNA
<213> Escherichia coli K-12 MG1655
<220> gst b1636
<222> 507-606
<223> gst b1636 U00096 1712401..1713006
<313> 2566: 584-603
<400> 2566
ACTGAATCTG GAAGGGTTAG AGCACATTGC AGCATTTATG CAACGTATGG 50
CTGAACGTCC GGAAGTACAA GACGCGCTGT CAGCGGAAGG CTTAAAGTAA 100
<210> 2567
<211> 100
<212> DNA
<213> Escherichia coli K-12 MG1655
<220> gst b1636
<222> 507-606
<223> gst b1636 U00096 1712401..1713006
<313> 2567: 542-561
<400> 2567
ACTGAATCTG GAAGGGTTAG AGCACATTGC AGCATTTATG CAACGTATGG 50
CTGAACGTCC GGAAGTACAA GACGCGCTGT CAGCGGAAGG CTTAAAGTAA 100
<210> 2568
<211> 100
<212> DNA
<213> Escherichia coli K-12 MG1655
<220> gst b1636
<222> 451-550
<223> gst b1636 U00096 1712401..1713006
<313> 2568: 502-521
<400> 2568
TTTACAATTG CTGATGCCTA TCTGTTTACG GTTCTGCGCT GGGCATACGC 50
GGTGAAACTG AATCTGGAAG GGTTAGAGCA CATTGCAGCA TTTATGCAAC 100
<210> 2569
<211> 100
<212> DNA
<213> Escherichia coli K-12 MG1655
<220> yial b3577
<222> 364-463

<223> yial b3577 U00096 3741372..3741839
<313> 2569: 386-405
<400> 2569
GCAATTTTCT TTCCGCAGGA TGTTCACCGA CCAGGTTGTA TTATGCAAAC  50
AGCCTCTGAG ATACGAAAAA TTGTGGTTAA AGTCGCATTA ACGGCGCTGA  100
<210> 2570
<211> 100
<212> DNA
<213> Escherichia coli K-12 MG1655
<220> yial b3577
<222> 202-301
<223> yial b3577 U00096 3741372..3741839
<313> 2570: 242-261
<400> 2570
TATATCGATA TTCAGTTTCT GGCGTGGGGC GAAGAGAAAA TCGGCATTGC  50
TATTGATACG GGAAATAATA AAGTCAGCGA ATCACTATTA GAGCAGCGCA  100
<210> 2571
<211> 100
<212> DNA
<213> Escherichia coli K-12 MG1655
<220> yial b3577
<222> 109-208
<223> yial b3577 U00096 3741372..3741839
<313> 2571: 166-185
<400> 2571
GTTGTCGAAA TCGACGGCAA AAATATTTAT ACGCAAATTA TCGATTTAAC  50
CACACGCGAA GCGGTGGTAA ACCGTCCGGA AGTGCATCGT CGGTATATCG  100
<210> 2572
<211> 100
<212> DNA
<213> Escherichia coli K-12 MG1655
<220> thra b0003
<222> 2364-2463
<223> thra b0003 U00096 337..2799
<313> 2572: 2440-2459
<400> 2572
GCCGTTGGTA CTGCGCGGAT ATGGTGCGGG CAATGACGTT ACAGCTGCCG  50
GTGTCTTTGC TGATCTGCTA CGTACCCTCT CATGGAAGTT AGGAGTCTGA  100
<210> 2573
<211> 100
<212> DNA
<213> Escherichia coli K-12 MG1655
<220> thra b0003
<222> 2294-2393
<223> thra b0003 U00096 337..2799
<313> 2573: 2342-2361
<400> 2573
ATGATCCGCT GTTCAAAGTG AAAAATGGCG AAAACGCCCT GGCCTTCTAT  50
AGCCACTATT ATCAGCCGCT GCCGTTGGTA CTGCGCGGAT ATGGTGCGGG  100
<210> 2574
<211> 100
<212> DNA
<213> Escherichia coli K-12 MG1655
<220> thra b0003
<222> 2235-2334
<223> thra b0003 U00096 337..2799
<313> 2574: 2271-2290
<400> 2574
TGTTGGCAAT ATTGATGAAG ATGGCGTCTG CCGCGTGAAG ATTGCCGAAG  50
TGGATGGTAA TGATCCGCTG TTCAAAGTGA AAAATGGCGA AAACGCCCTG  100
<210> 2575
<211> 100
<212> DNA
<213> Escherichia coli K-12 MG1655
<220> yiam b3578
<222> 375-474
<223> yiam b3578 U00096 3741957..3742430
<313> 2575: 444-463
<400> 2575
TCTGCCCACC AGCCTCGTTA TCGCATTCTT TGAACTGCGT CATTTATATC  50
AACTCATCAC GCGTAGCAAT TCTCTAACGT CGCCACCGCA AGGAGCTTAA  100

<210> 2576
<211> 100
<212> DNA
<213> Escherichia coli K-12 MG1655
<220> yiam b3578
<222> 348-447
<223> yiam b3578 U00096 3741957..3742430
<313> 2576: 389-408
<400> 2576
ACCCATCGGC CTGATGTATG CCGCTTGTCT GCCCACCAGC CTCGTTATCG  50
CATTCTTTGA ACTGCGTCAT TTATATCAAC TCATCACGCG TAGCAATTCT  100
<210> 2577
<211> 100
<212> DNA
<213> Escherichia coli K-12 MG1655
<220> yiam b3578
<222> 302-401
<223> yiam b3578 U00096 3741957..3742430
<313> 2577: 359-378
<400> 2577
TAAAAACAAT TCAGGACTGG AGCGATTATT CACCGATCCT CGGTTTACCC  50
ATCGGCCTGA TGTATGCCGC TTGTCTGCCC ACCAGCCTCG TTATCGCATT  100
<210> 2578
<211> 100
<212> DNA
<213> Escherichia coli K-12 MG1655
<220> thrb b0004
<222> 779-878
<223> thrb b0004 U00096 2801..3733
<313> 2578: 807-826
<400> 2578
CCGGCTCCGG CCCGACCTTG TTCGCTCTGT GTGACAAGCC GGAAACCGCC  50
CAGCGCGTTG CCGACTGGTT GGGTAAGAAC TACCTGCAAA ATCAGGAAGG  100
<210> 2579
<211> 100
<212> DNA
<213> Escherichia coli K-12 MG1655
<220> thrb b0004
<222> 716-815
<223> thrb b0004 U00096 2801..3733
<313> 2579: 757-776
<400> 2579
TGCCAGGCTT CCGGCAGGCG CGGCAGGCGG TCGCGGAAAT CGGCGCGGTA  50
GCGAGCGGTA TCTCCGGCTC CGGCCCGACC TTGTTCGCTC TGTGTGACAA  100
<210> 2580
<211> 100
<212> DNA
<213> Escherichia coli K-12 MG1655
<220> thrb b0004
<222> 674-773
<223> thrb b0004 U00096 2801..3733
<313> 2580: 712-731
<400> 2580
TGATGAAAGA TGTTATCGCT GAACCCTACC GTGAACGGTT ACTGCCAGGC  50
TTCCGGCAGG CGCGGCAGGC GGTCGCGGAA ATCGGCGCGG TAGCGAGCGG  100
<210> 2581
<211> 100
<212> DNA
<213> Escherichia coli K-12 MG1655
<220> yian b3579
<222> 879-978
<223> yian b3579 U00096 3742433..3743707
<313> 2581: 936-955
<400> 2581
CATTGCTGAA CTGCCGATGA TGGTTTCTGA TTTACTGCAA CCGTTGGTCG  50
ATTCACCGCG TCTGCTGTTT ATCGTCATTA TGGTGGCTAT CCTGATTGTC  100
<210> 2582
<211> 100
<212> DNA
<213> Escherichia coli K-12 MG1655
<220> yian b3579

<222> 858-957
<223> yian b3579 U00096 3742433..3743707
<313> 2582: 885-904
<400> 2582
AGTTTCCGCC TGGCTGATTA CCATTGCTGA ACTGCCGATG ATGGTTTCTG  50
ATTTACTGCA ACCGTTGGTC GATTCACCGC GTCTGCTGTT TATCGTCATT  100
<210> 2583
<211> 100
<212> DNA
<213> Escherichia coli K-12 MG1655
<220> yian b3579
<222> 797-896
<223> yian b3579 U00096 3742433..3743707
<313> 2583: 843-862
<400> 2583
ATGTACTCAT TGGCGCGGCG AAAACCACCT CAGTGGTTAT GTTTCTGGTG  50
GCCTCGGCAC AAGTTTCCGC CTGGCTGATT ACCATTGCTG AACTGCCGAT  100
<210> 2584
<211> 100
<212> DNA
<213> Escherichia coli K-12 MG1655
<220> thrc thrC
<222> 1188-1287
<223> thrc thrC U00096 3734..5020
<313> 2584: 1240-1259
<400> 2584
GCCAAAAGAG CTGGCAGAAC GTGCTGATTT ACCCTTGCTT TCACATAATC  50
TGCCCGCCGA TTTTGCTGCG TTGCGTAAAT TGATGATGAA TCATCAGTAA  100
<210> 2585
<211> 100
<212> DNA
<213> Escherichia coli K-12 MG1655
<220> thrc thrC
<222> 1136-1235
<223> thrc thrC U00096 3734..5020
<313> 2585: 1164-1183
<400> 2585
CGGCGAAATT TAAAGAGAGC GTGGAAGCGA TTCTCGGTGA AACGTTGGAT  50
CTGCCAAAAG AGCTGGCAGA ACGTGCTGAT TTACCCTTGC TTTCACATAA  100
<210> 2586
<211> 100
<212> DNA
<213> Escherichia coli K-12 MG1655
<220> thrc thrC
<222> 1090-1189
<223> thrc thrC U00096 3734..5020
<313> 2586: 1127-1146
<400> 2586
CAGTTGAATC CAGGCGAATA TGGCTTGTTC CTCGGCACCG CGCATCCGGC  50
GAAATTTAAA GAGAGCGTGG AAGCGATTCT CGGTGAAACG TTGGATCTGC  100
<210> 2587
<211> 100
<212> DNA
<213> Escherichia coli K-12 MG1655
<220> yiao b3580
<222> 888-987
<223> yiao b3580 U00096 3743724..3744710
<313> 2587: 951-970
<400> 2587
AGCCTTTAGC GACGCACTGG GGAATCAGGT TCGCGACATG TTTGTTAAAG  50
ATGTGCCGCA GGGAGCTGAT CTGCTGAAAG CCGTGGATGA GGTGCAATAA  100
<210> 2588
<211> 100
<212> DNA
<213> Escherichia coli K-12 MG1655
<220> yiao b3580
<222> 841-940
<223> yiao b3580 U00096 3743724..3744710
<313> 2588: 891-910
<400> 2588
GGCATGAAAG AAGCGGGCGT GGAAGTCATC ACCGATCTCG ACCGCAAAGC  50

CTTTAGCGAC GCACTGGGGA ATCAGGTTCG CGACATGTTT GTTAAAGATG 100
<210> 2589
<211> 100
<212> DNA
<213> Escherichia coli K-12 MG1655
<220> yiao b3580
<222> 824-923
<223> yiao b3580 U00096 3743724..3744710
<313> 2589: 860-879
<400> 2589
AGCAAAAAAT CATCGACGGC ATGAAAGAAG CGGGCGTGGA AGTCATCACC 50
GATCTCGACC GCAAAGCCTT TAGCGACGCA CTGGGGAATC AGGTTCGCGA 100
<210> 2590
<211> 100
<212> DNA
<213> Escherichia coli K-12 MG1655
<220> cela b1739
<222> 192-291
<223> cela b1739 U00096 complement(1819323..1819643)
<313> 2590: 256-275
<400> 2590
GCCCGAAATC CAGCGTTTGT TACCCAACAA ACCGGTTGAA GTAATTGACT 50
CGCTGCTTTA TGGCAAAGTC GATGGTTTAG GCGTGCTTAA GGCTGCGGTT 100
<210> 2591
<211> 100
<212> DNA
<213> Escherichia coli K-12 MG1655
<220> cela b1739
<222> 167-266
<223> cela b1739 U00096 complement(1819323..1819643)
<313> 2591: 193-212
<400> 2591
TAGGGCCGCA GATTGCTTAT ATGTTGCCCG AAATCCAGCG TTTGTTACCC 50
AACAAACCGG TTGAAGTAAT TGACTCGCTG CTTTATGGCA AAGTCGATGG 100
<210> 2592
<211> 100
<212> DNA
<213> Escherichia coli K-12 MG1655
<220> cela b1739
<222> 22-121
<223> cela b1739 U00096 complement(1819323..1819643)
<313> 2592: 78-97
<400> 2592
CTGTTTTGTT CTGCGGGCAT GTCTACCTCT TTACTGGTAT CAAAAATGCG 50
CGCACAGGCA GAAAAATATG AAGTTCCGGT CATTATTGAA GCATTTCCGG 100
<210> 2593
<211> 100
<212> DNA
<213> Escherichia coli K-12 MG1655
<220> celb b1738
<222> 1199-1298
<223> celb b1738 U00096 complement(1817880..1819238)
<313> 2593: 1250-1269
<400> 2593
TTAACACCAA CGGTAGCGTC GCCGCATTGC TGGTCGCACT CTTCAACCTT 50
GGCATCGCAA CGTTAATTTA TCTGCCCTTT GTTGTGGTGG CTAACAAAGC 100
<210> 2594
<211> 100
<212> DNA
<213> Escherichia coli K-12 MG1655
<220> celb b1738
<222> 1172-1271
<223> celb b1738 U00096 complement(1817880..1819238)
<313> 2594: 1210-1229
<400> 2594
CCATGCCAAC CGGTCTGGGA GCCTTCTTTA ACACCAACGG TAGCGTCGCC 50
GCATTGCTGG TCGCACTCTT CAACCTTGGC ATCGCAACGT TAATTTATCT 100
<210> 2595
<211> 100
<212> DNA
<213> Escherichia coli K-12 MG1655

<220> celb b1738
<222> 1089-1188
<223> celb b1738 U00096 complement(1817880..1819238)
<313> 2595: 1141-1160
<400> 2595
GGTACAACCG ATTCTGGCGG CAATCACCCT CGCAGCGTAC TACATGGGCA  50
TTATTCCTCC GGTGACCAAT ATTGCACCGT GGACCATGCC AACCGGTCTG  100
<210> 2596
<211> 100
<212> DNA
<213> Escherichia coli K-12 MG1655
<220> yiat b3585
<222> 642-741
<223> yiat b3585 U00096 complement(3748758..3749498)
<313> 2596: 706-725
<400> 2596
CGCAGGATAC AGCGAGCTGC CGGAAGAGAT TACCGACAGC CCGATGATTG  50
ATCGTAATGA GAGTTTTACC TTTGTCACCG GGGTGAGCTG GCGTTTTTAA  100
<210> 2597
<211> 100
<212> DNA
<213> Escherichia coli K-12 MG1655
<220> yiat b3585
<222> 629-728
<223> yiat b3585 U00096 complement(3748758..3749498)
<313> 2597: 667-686
<400> 2597
TATTGATGGC GAGCGCAGGA TACAGCGAGC TGCCGGAAGA GATTACCGAC  50
AGCCCGATGA TTGATCGTAA TGAGAGTTTT ACCTTTGTCA CCGGGGTGAG  100
<210> 2598
<211> 100
<212> DNA
<213> Escherichia coli K-12 MG1655
<220> yiat b3585
<222> 584-683
<223> yiat b3585 U00096 complement(3748758..3749498)
<313> 2598: 623-642
<400> 2598
CCTATGTCAG CCTGACGGCA AAATACCCGA TAGGAGAGCA CGTCGTATTG  50
ATGGCGAGCG CAGGATACAG CGAGCTGCCG GAAGAGATTA CCGACAGCCC  100
<210> 2599
<211> 100
<212> DNA
<213> Escherichia coli K-12 MG1655
<220> celc b1737
<222> 173-272
<223> celc b1737 U00096 complement(1817479..1817829)
<313> 2599: 234-253
<400> 2599
GGATGGCATT GAATGAAGCG CATCTGGTAC AGACGAAACT GATTGAAGGC  50
GATGCGGGCG AAGGTAAGAT GAAAGTGAGT CTGGTGCTGG TCCACGCTCA  100
<210> 2600
<211> 100
<212> DNA
<213> Escherichia coli K-12 MG1655
<220> celc b1737
<222> 168-267
<223> celc b1737 U00096 complement(1817479..1817829)
<313> 2600: 192-211
<400> 2600
GTCACGGATG GCATTGAATG AAGCGCATCT GGTACAGACG AAACTGATTG  50
AAGGCGATGC GGGCGAAGGT AAGATGAAAG TGAGTCTGGT GCTGGTCCAC  100
<210> 2601
<211> 100
<212> DNA
<213> Escherichia coli K-12 MG1655
<220> celc b1737
<222> 96-195
<223> celc b1737 U00096 complement(1817479..1817829)
<313> 2601: 139-158
<400> 2601

CAGCCTGGCG TATGCGGCAC TGAAACAGGC GAAGCAGGGC GATTTTGCCG 50
CAGCAAAAGC CATGATGGAT CAGTCACGGA TGGCATTGAA TGAAGCGCAT 100
<210> 2602
<211> 100
<212> DNA
<213> Escherichia coli K-12 MG1655
<220> yiau b3586
<222> 876-975
<223> yiau b3586 U00096 3749622..3750596
<313> 2602: 950-969
<400> 2602
GGTCCAGTAT GTGCTGGATG AAATGCTGCG TGAAATTCGC CACCAGCTTG 50
CTCAGTCGCA GCAACTGCGG CCTGAACAAG CGGCGGAAAG CGAAGATTAA 100
<210> 2603
<211> 100
<212> DNA
<213> Escherichia coli K-12 MG1655
<220> yiau b3586
<222> 872-971
<223> yiau b3586 U00096 3749622..3750596
<313> 2603: 905-924
<400> 2603
GGCGGGTCCA GTATGTGCTG GATGAAATGC TGCGTGAAAT TCGCCACCAG 50
CTTGCTCAGT CGCAGCAACT GCGGCCTGAA CAAGCGGCGG AAAGCGAAGA 100
<210> 2604
<211> 100
<212> DNA
<213> Escherichia coli K-12 MG1655
<220> yiau b3586
<222> 825-924
<223> yiau b3586 U00096 3749622..3750596
<313> 2604: 855-874
<400> 2604
GTGGCTGGAT CTGGTGGCTA TCTTCCGTAA ACGGGAATAC CAGCCCTGGC 50
GGGTCCAGTA TGTGCTGGAT GAAATGCTGC GTGAAATTCG CCACCAGCTT 100
<210> 2605
<211> 100
<212> DNA
<213> Escherichia coli K-12 MG1655
<220> celd b1736
<222> 578-677
<223> celd b1736 U00096 complement(1816629..1817471)
<313> 2605: 631-650
<400> 2605
AGAATATGGT GGCGTTGTCA GCCAAATCAC AGGAATATTT GACGCGAGCG 50
ACTCAACGAT ATTATGGCAA AACGCCAATG CAGATTATTA ATGAAATCCG 100
<210> 2606
<211> 100
<212> DNA
<213> Escherichia coli K-12 MG1655
<220> celd b1736
<222> 481-580
<223> celd b1736 U00096 complement(1816629..1817471)
<313> 2606: 515-534
<400> 2606
TACCGCGAAG AACAGGTGAT TGATGATGTA CCGCAGTGGC TGAAAAGTAC 50
GGTAGAAAAG ATGCATGATA AAGAGCAGTT TAGTGAATCG GCGCTGGAGA 100
<210> 2607
<211> 100
<212> DNA
<213> Escherichia coli K-12 MG1655
<220> celd b1736
<222> 434-533
<223> celd b1736 U00096 complement(1816629..1817471)
<313> 2607: 477-496
<400> 2607
AGTTTGTTGA GATGGTTACT TTTTATGTCA TTAACCGTTT ACGTCATTAC 50
CGCGAAGAAC AGGTGATTGA TGATGTACCG CAGTGGCTGA AAAGTACGGT 100
<210> 2608
<211> 100
<212> DNA

<213> Escherichia coli K-12 MG1655
<220> yiav b3587
<222> 1013-1112
<223> yiav b3587 U00096 complement(3750593..3751729)
<313> 2608: 1045-1064
<400> 2608
CTTTACCAGA CGGTATTTAC GCCCAGGTGG CGGTTTACTC TGATCATTTC 50
AGCCATGTCT CGGTGATGCG CAAAGTGCTA TTACGCATGA CCAGCTGGGT 100
<210> 2609
<211> 100
<212> DNA
<213> Escherichia coli K-12 MG1655
<220> yiav b3587
<222> 914-1013
<223> yiav b3587 U00096 complement(3750593..3751729)
<313> 2609: 933-952
<400> 2609
CTTATCAGTC GACCGGCACC TTACAGACGT TAAACACAGC GCCGGGTTCA 50
GATGGCGTTA TCGCGACCAT TGAACTGGAT GAGCACACTG ATTTGAGCGC 100
<210> 2610
<211> 100
<212> DNA
<213> Escherichia coli K-12 MG1655
<220> yiav b3587
<222> 839-938
<223> yiav b3587 U00096 complement(3750593..3751729)
<313> 2610: 888-907
<400> 2610
TGGTGTTTAA TGCTCTGCCA GGTAAGGTAT TCAGCGGTAA GCTGGCAGCC 50
ATTAGTCCAG CCGTTCCCGG CGGAGCTTAT CAGTCGACCG GCACCTTACA 100
<210> 2611
<211> 100
<212> DNA
<213> Escherichia coli K-12 MG1655
<220> yiaw b3588
<222> 225-324
<223> yiaw b3588 U00096 complement(3751735..3752058)
<313> 2611: 295-314
<400> 2611
GGGAATGCAA AGCCATGATT CATCCGTTAT GCAACTGCAA CAGCGCATTG 50
CCGGGCTGGA GAAACAGCTC GCCGACATCA AATCCTCTTC TGCCGAGTAA 100
<210> 2612
<211> 100
<212> DNA
<213> Escherichia coli K-12 MG1655
<220> yiaw b3588
<222> 217-316
<223> yiaw b3588 U00096 complement(3751735..3752058)
<313> 2612: 248-267
<400> 2612
CGGGGATGGG GAATGCAAAG CCATGATTCA TCCGTTATGC AACTGCAACA 50
GCGCATTGCC GGGCTGGAGA AACAGCTCGC CGACATCAAA TCCTCTTCTG 100
<210> 2613
<211> 100
<212> DNA
<213> Escherichia coli K-12 MG1655
<220> yiaw b3588
<222> 173-272
<223> yiaw b3588 U00096 complement(3751735..3752058)
<313> 2613: 219-238
<400> 2613
TCTGGCCGTT TTTGTGGATT TGGGCCACGC TTTACCGCCC GGAGCGGGGA 50
TGGGGAATGC AAAGCCATGA TTCATCCGTT ATGCAACTGC AACAGCGCAT 100
<210> 2614
<211> 100
<212> DNA
<213> Escherichia coli K-12 MG1655
<220> thrl b0002
<222> 1-66
<223> thrl b0002 U00096 190..255
<313> 2614: 35-54

<400> 2614
ATGAAACGCA TTAGCACCAC CATTACCACC ACCATCACCA TTACCACAGG  50
TAACGGTGCG GGCTGA  100
<210> 2615
<211> 100
<212> DNA
<213> Escherichia coli K-12 MG1655
<220> thrl b0002
<222> 1-66
<223> thrl b0002 U00096 190..255
<313> 2615: 23-42
<400> 2615
ATGAAACGCA TTAGCACCAC CATTACCACC ACCATCACCA TTACCACAGG  50
TAACGGTGCG GGCTGA  100
<210> 2616
<211> 100
<212> DNA
<213> Escherichia coli K-12 MG1655
<220> thrl b0002
<222> 1-66
<223> thrl b0002 U00096 190..255
<313> 2616: 27-46
<400> 2616
ATGAAACGCA TTAGCACCAC CATTACCACC ACCATCACCA TTACCACAGG  50
TAACGGTGCG GGCTGA  100
<210> 2617
<211> 100
<212> DNA
<213> Escherichia coli K-12 MG1655
<220> celf b1735
<222> 1254-1353
<223> celf b1735 U00096 complement(1815172..1816524)
<313> 2617: 1317-1336
<400> 2617
TCGCGATGCT GAGCTGCTGG CACGCGAGAT GATTCTGGCG CACGAGAAAT  50
GGCTGCCAAA CTTTGCCGAC TGCATCGCAG AGCTTAAAAA AGCACATTAA  100
<210> 2618
<211> 100
<212> DNA
<213> Escherichia coli K-12 MG1655
<220> celf b1735
<222> 1235-1334
<223> celf b1735 U00096 complement(1815172..1816524)
<313> 2618: 1276-1295
<400> 2618
GTCCGTTGGT GCATTCCGAT CGCGATGCTG AGCTGCTGGC ACGCGAGATG  50
ATTCTGGCGC ACGAGAAATG GCTGCCAAAC TTTGCCGACT GCATCGCAGA  100
<210> 2619
<211> 100
<212> DNA
<213> Escherichia coli K-12 MG1655
<220> celf b1735
<222> 1163-1262
<223> celf b1735 U00096 complement(1815172..1816524)
<313> 2619: 1195-1214
<400> 2619
GCTTCGAGAT TGCTGCCAGT AACGCCGCAC TTAGCGGAGA ATTTAACGAT  50
GTGTTACTGG CGCTAAACCT TAGTCCGTTG GTGCATTCCG ATCGCGATGC  100
<210> 2620
<211> 100
<212> DNA
<213> Escherichia coli K-12 MG1655
<220> yiay b3590
<222> 1050-1149
<223> yiay b3590 U00096 complement(3754306..3755454)
<313> 2620: 1114-1133
<400> 2620
CGCGGTATTG GCGACTAATG CCCTGAAAGA TGCCTGTGGC TTTACTAACC  50
CGATCCAGGC AACTCACGAA GAAATTGTGG CGATTTATGC AGCGAGGTAA  100
<210> 2621
<211> 100

<212> DNA
<213> Escherichia coli K-12 MG1655
<220> yiay b3590
<222> 1032-1131
<223> yiay b3590 U00096 complement(3754306..3755454)
<313> 2621: 1075-1094
<400> 2621
CGTGAAAGAA GAAGATTTCG CGGTATTGGC GACTAATGCC CTGAAAGATG 50
CCTGTGGCTT TACTAACCCG ATCCAGGCAA CTCACGAAGA AATTGTGGCG 100
<210> 2622
<211> 100
<212> DNA
<213> Escherichia coli K-12 MG1655
<220> yiay b3590
<222> 956-1055
<223> yiay b3590 U00096 complement(3754306..3755454)
<313> 2622: 973-992
<400> 2622
GTGCTGAAGC CTGCATTAAC GCCATCCGTG AACTGGCGAA GAAAGTGGAT 50
ATCCCGGCAG GCCTACGCGA CCTGAACGTG AAAGAAGAAG ATTTCGCGGT 100
<210> 2623
<211> 100
<212> DNA
<213> Escherichia coli K-12 MG1655
<220> thrs thrS
<222> 1830-1929
<223> thrs thrS U00096 complement(1798666..1800594)
<313> 2623: 1895-1914
<400> 2623
CCGCCGTGGT AAAGACCTGG GAAGCATGGA CGTAAATGAA GTGATCGAGA 50
AGCTGCAACA AGAGATTCGC AGCCGCAGTC TTAAACAATT GGAGGAATAA 100
<210> 2624
<211> 100
<212> DNA
<213> Escherichia coli K-12 MG1655
<220> thrs thrS
<222> 1830-1929
<223> thrs thrS U00096 complement(1798666..1800594)
<313> 2624: 1858-1877
<400> 2624
CCGCCGTGGT AAAGACCTGG GAAGCATGGA CGTAAATGAA GTGATCGAGA 50
AGCTGCAACA AGAGATTCGC AGCCGCAGTC TTAAACAATT GGAGGAATAA 100
<210> 2625
<211> 100
<212> DNA
<213> Escherichia coli K-12 MG1655
<220> thrs thrS
<222> 1763-1862
<223> thrs thrS U00096 complement(1798666..1800594)
<313> 2625: 1810-1829
<400> 2625
GTCGCGTCCC ATATATGCTG GTCTGTGGTG ATAAAGAGGT GGAATCAGGC 50
AAAGTTGCCG TTCGCACCCG CCGTGGTAAA GACCTGGGAA GCATGGACGT 100
<210> 2626
<211> 100
<212> DNA
<213> Escherichia coli K-12 MG1655
<220> yiba b3595
<222> 687-786
<223> yiba b3595 U00096 3763964..3764806
<313> 2626: 737-756
<400> 2626
CGATGAGTTA AAAAAAAATA CTGTTTATGA TGATATCATT GAAGCTGCGG 50
GTGAATTAGG TGATAAAACG CTACTTCCTG TTTTAGATAC TATGTTGTAC 100
<210> 2627
<211> 100
<212> DNA
<213> Escherichia coli K-12 MG1655
<220> yiba b3595
<222> 486-585
<223> yiba b3595 U00096 3763964..3764806

<313> 2627: 514-533
<400> 2627
ACTATTGATT AATCTGTTAA AAGATCCAAA TGGAGATGTC AGAAACTGGG 50
CCGCATTTGC AATAAATATC AATAAATATG ATAATAGTGA TATTAGGGAT 100
<210> 2628
<211> 100
<212> DNA
<213> Escherichia coli K-12 MG1655
<220> yiba b3595
<222> 305-404
<223> yiba b3595 U00096 3763964..3764806
<313> 2628: 354-373
<400> 2628
ATAAGAGCGC TTGCGTTCGA GCTACGGCAA TCGAGTCAAC GGCTCAGCGA 50
TGCAAGAAAA ACCCAATTTA TTCACCTAAA ATAGTAGAAC AATCTCAAAT 100
<210> 2629
<211> 100
<212> DNA
<213> Escherichia coli K-12 MG1655
<220> yibd b3616
<222> 918-1017
<223> yibd b3616 U00096 complement(3786674..3787708)
<313> 2629: 966-985
<400> 2629
TGTGCGCAGC GTGAAGGTCG GTTACCAGGC GTTACTGTGG TCTTTCCGCT 50
TATGGCAATG GCGCGACAAA ACGCGGTCGC ACCATCGCAT TACGCGTAGC 100
<210> 2630
<211> 100
<212> DNA
<213> Escherichia coli K-12 MG1655
<220> yibd b3616
<222> 846-945
<223> yibd b3616 U00096 complement(3786674..3787708)
<313> 2630: 873-892
<400> 2630
GGATATTCTT ACCCGCCAAC GGATGATTGC CGAGATATTT ACTTCCGGTA 50
TGTATAAGCG CCTGATTACC AATGTGCGCA GCGTGAAGGT CGGTTACCAG 100
<210> 2631
<211> 100
<212> DNA
<213> Escherichia coli K-12 MG1655
<220> yibd b3616
<222> 701-800
<223> yibd b3616 U00096 complement(3786674..3787708)
<313> 2631: 739-758
<400> 2631
ATTATCAACG TCACTATATT AAGATTACCC GCCTGCTGGA GAAATTAAAT 50
CGAAATTATG CCGACAAAAT TATGATTTAT CCGGAATTTC ATCAGCAAAT 100
<210> 2632
<211> 100
<212> DNA
<213> Escherichia coli K-12 MG1655
<220> enta b0597
<222> 648-747
<223> enta b0597 U00096 627774..628520
<313> 2632: 710-729
<400> 2632
AGAGATCGCC AACACGATTT TGTTCCTCGC CTCTGACCTC GCCAGCCATA 50
TTACCCTACA GGATATTGTG GTCGATGGCG GCTCAACGCT GGGGGCATAA 100
<210> 2633
<211> 100
<212> DNA
<213> Escherichia coli K-12 MG1655
<220> enta b0597
<222> 635-734
<223> enta b0597 U00096 627774..628520
<313> 2633: 687-706
<400> 2633
TCGCCCGTCC ACAAGAGATC GCCAACACGA TTTTGTTCCT CGCCTCTGAC 50
CTCGCCAGCC ATATTACCCT ACAGGATATT GTGGTCGATG GCGGCTCAAC 100
<210> 2634

<211> 100
<212> DNA
<213> Escherichia coli K-12 MG1655
<220> enta b0597
<222> 607-706
<223> enta b0597 U00096 627774..628520
<313> 2634: 632-651
<400> 2634
TTTAAACTCG GCATTCCGCT GGGGAAAATC GCCCGTCCAC AAGAGATCGC 50
CAACACGATT TTGTTCCTCG CCTCTGACCT CGCCAGCCAT ATTACCCTAC 100
<210> 2635
<211> 100
<212> DNA
<213> Escherichia coli K-12 MG1655
<220> entb b0596
<222> 759-858
<223> entb b0596 U00096 626917..627774
<313> 2635: 828-847
<400> 2635
GCGCTGGCGC AAAGTGCATG GTGATATCGA CTTTGTCATG CTGGCGAAAA 50
ACCCGACCAT CGACGCCTGG TGGAAGCTAC TCTCCCGCGA GGTGAAATAA 100
<210> 2636
<211> 100
<212> DNA
<213> Escherichia coli K-12 MG1655
<220> entb b0596
<222> 677-776
<223> entb b0596 U00096 626917..627774
<313> 2636: 692-711
<400> 2636
TGGACGAGTC CGATGAACCG TTCGATGACG ACAACCTGAT CGACTACGGT 50
CTGGATTCGG TGCGCATGAT GGCGCTGGCG GCGCGCTGGC GCAAAGTGCA 100
<210> 2637
<211> 100
<212> DNA
<213> Escherichia coli K-12 MG1655
<220> entb b0596
<222> 635-734
<223> entb b0596 U00096 626917..627774
<313> 2637: 670-689
<400> 2637
CCGCCAGCAA AGCGGCGCTG CGTGAGGTGA TCCTGCCGTT GCTGGACGAG 50
TCCGATGAAC CGTTCGATGA CGACAACCTG ATCGACTACG GTCTGGATTC 100
<210> 2638
<211> 100
<212> DNA
<213> Escherichia coli K-12 MG1655
<220> yibf b3593
<222> 510-609
<223> yibf b3593 U00096 complement(3758974..3759582)
<313> 2638: 577-596
<400> 2638
TGCGCCAGGT TGGTGTGTCG ACCGCCCACA CTTAGTCAAA CTGGTCGAAA 50
ACCTGTTTAG CCGCGAAAGT TTTGCTCGCA CCGAACCGCC AAAGGCTTGA 100
<210> 2639
<211> 100
<212> DNA
<213> Escherichia coli K-12 MG1655
<220> yibf b3593
<222> 510-609
<223> yibf b3593 U00096 complement(3758974..3759582)
<313> 2639: 539-558
<400> 2639
TGCGCCAGGT TGGTGTGTCG ACCGCCCACA CTTAGTCAAA CTGGTCGAAA 50
ACCTGTTTAG CCGCGAAAGT TTTGCTCGCA CCGAACCGCC AAAGGCTTGA 100
<210> 2640
<211> 100
<212> DNA
<213> Escherichia coli K-12 MG1655
<220> yibf b3593
<222> 384-483

<223> yibf b3593 U00096 complement(3758974..3759582)
<313> 2640: 434-453
<400> 2640
GAAAATCAAC CGCAGTCTGG ATGTGCTGGA AGGATATCTG GTCGATGGCA  50
CACTCAAAAC CGATACGGTC AATCTGGCGA CTATCGCCAT TGCCTGTGCC  100
<210> 2641
<211> 100
<212> DNA
<213> Escherichia coli K-12 MG1655
<220> entc b0594
<222> 1050-1149
<223> entc b0594 U00096 624108..625283
<313> 2641: 1088-1107
<400> 2641
GCTGCGGGAA AATCAGGTGC GTCTGTTTGC CGGAGCGGGG ATTGTGCCTG  50
CGTCGTCACC GTTGGGTGAG TGGCGCGAAA CAGGCGTCAA ACTTTCTACC  100
<210> 2642
<211> 100
<212> DNA
<213> Escherichia coli K-12 MG1655
<220> entc b0594
<222> 1005-1104
<223> entc b0594 U00096 624108..625283
<313> 2642: 1043-1062
<400> 2642
CAGCGAAGGT AACGGCGAAT GGGTGGTGAC CATCCGCTGC GCGAAGCTGC  50
GGGAAAATCA GGTGCGTCTG TTTGCCGGAG CGGGGATTGT GCCTGCGTCG  100
<210> 2643
<211> 100
<212> DNA
<213> Escherichia coli K-12 MG1655
<220> entc b0594
<222> 952-1051
<223> entc b0594 U00096 624108..625283
<313> 2643: 992-1011
<400> 2643
GAACTGGAAC CGTTCGACCG CGAACTGTTT GGCGGCATTG TGGGTTGGTG  50
TGACAGCGAA GGTAACGGCG AATGGGTGGT GACCATCCGC TGCGCGAAGC  100
<210> 2644
<211> 100
<212> DNA
<213> Escherichia coli K-12 MG1655
<220> yibg b3597
<222> 363-462
<223> yibg b3597 U00096 3765804..3766265
<313> 2644: 437-456
<400> 2644
ATTCGCTGTA AATAACGGTA ATATTGATGC AAAGAGCAAA CTTCAAGAGC  50
TCAAAAAGCT AAAGCGAATG GATAGAAGGA AGAATAAGGA GAATCCGTAA  100
<210> 2645
<211> 100
<212> DNA
<213> Escherichia coli K-12 MG1655
<220> yibg b3597
<222> 363-462
<223> yibg b3597 U00096 3765804..3766265
<313> 2645: 433-452
<400> 2645
ATTCGCTGTA AATAACGGTA ATATTGATGC AAAGAGCAAA CTTCAAGAGC  50
TCAAAAAGCT AAAGCGAATG GATAGAAGGA AGAATAAGGA GAATCCGTAA  100
<210> 2646
<211> 100
<212> DNA
<213> Escherichia coli K-12 MG1655
<220> yibg b3597
<222> 359-458
<223> yibg b3597 U00096 3765804..3766265
<313> 2646: 379-398
<400> 2646
TGGAATTCGC TGTAAATAAC GGTAATATTG ATGCAAAGAG CAAACTTCAA  50
GAGCTCAAAA AGCTAAAGCG AATGGATAGA AGGAAGAATA AGGAGAATCC  100

<210> 2647
<211> 100
<212> DNA
<213> Escherichia coli K-12 MG1655
<220> entd b0584
<222> 435-534
<223> entd b0584 U00096 complement(608682..609311)
<313> 2647: 493-512
<400> 2647
GGCGCTGACA CTGGCATTTT CCGCCAAAGA GAGCGCATTT AAGGCAAGTG 50
AGATCCAAAC TGATGCAGGT TTTCTGGACT ATCAGATAAT TAGCTGGAAT 100
<210> 2648
<211> 100
<212> DNA
<213> Escherichia coli K-12 MG1655
<220> entd b0584
<222> 389-488
<223> entd b0584 U00096 complement(608682..609311)
<313> 2648: 447-466
<400> 2648
CAGCGGAACA CGAGCGACTC GCAGACTGCG GTTTAGCCTT TTCTCTGGCG 50
CTGACACTGG CATTTTCCGC CAAAGAGAGC GCATTTAAGG CAAGTGAGAT 100
<210> 2649
<211> 100
<212> DNA
<213> Escherichia coli K-12 MG1655
<220> entd b0584
<222> 352-451
<223> entd b0584 U00096 complement(608682..609311)
<313> 2649: 392-411
<400> 2649
CAAACCGCAA GAGAATTGAC AGACAACATT ATTACACCAG CGGAACACGA 50
GCGACTCGCA GACTGCGGTT TAGCCTTTTC TCTGGCGCTG ACACTGGCAT 100
<210> 2650
<211> 100
<212> DNA
<213> Escherichia coli K-12 MG1655
<220> yibh b3598
<222> 1038-1137
<223> yibh b3598 U00096 complement(3767870..3769006)
<313> 2650: 1074-1093
<400> 2650
GGTGGCGGTT TACTCCGACC ATTTCAGCCA TGTTTCGGTG ATGCGGAAAG 50
TGCTGCTAAG AATGACCAGC TGGATGCATT ATCTTTATTT GGATCATTGA 100
<210> 2651
<211> 100
<212> DNA
<213> Escherichia coli K-12 MG1655
<220> yibh b3598
<222> 1010-1109
<223> yibh b3598 U00096 complement(3767870..3769006)
<313> 2651: 1039-1058
<400> 2651
ATGCCTTACC CGACGGCATC TACGCCCAGG TGGCGGTTTA CTCCGACCAT 50
TTCAGCCATG TTTCGGTGAT GCGGAAAGTG CTGCTAAGAA TGACCAGCTG 100
<210> 2652
<211> 100
<212> DNA
<213> Escherichia coli K-12 MG1655
<220> yibh b3598
<222> 922-1021
<223> yibh b3598 U00096 complement(3767870..3769006)
<313> 2652: 977-996
<400> 2652
GCGCAGGGGG TATTGCAATC ATTAACGGTC GTGCCCGGCA CGGACGGTGT 50
GCTGGGAACC ATTGAACTGG ACCCTAACGA TGATATCGAT GCCTTACCCG 100
<210> 2653
<211> 100
<212> DNA
<213> Escherichia coli K-12 MG1655
<220> ente b0595

<222> 1512-1611
<223> ente b0595 U00096 625293..626903
<313> 2653: 1556-1575
<400> 2653
GGATCGCGTG GAGTGTGTGG ATTCACTTCC GCTGACGGCG GTCGGGAAAG 50
TCGATAAAAA ACAATTACGT CAGTGGCTGG CGTCACGCGC ATCAGCCTGA 100
<210> 2654
<211> 100
<212> DNA
<213> Escherichia coli K-12 MG1655
<220> ente b0595
<222> 1428-1527
<223> ente b0595 U00096 625293..626903
<313> 2654: 1467-1486
<400> 2654
TTATCTGGTG GTAAAAGAGC CGCTGCGCGC GGTGCAGGTG CGTCGTTTCC 50
TGCGTGAACA GGGTATTGCC GAATTTAAAT TACCGGATCG CGTGGAGTGT 100
<210> 2655
<211> 100
<212> DNA
<213> Escherichia coli K-12 MG1655
<220> ente b0595
<222> 1389-1488
<223> ente b0595 U00096 625293..626903
<313> 2655: 1417-1436
<400> 2655
GAGCATGGAA GATGAGCTGA TGGGCGAAAA AAGCTGCGCT TATCTGGTGG 50
TAAAAGAGCC GCTGCGCGCG GTGCAGGTGC GTCGTTTCCT GCGTGAACAG 100
<210> 2656
<211> 100
<212> DNA
<213> Escherichia coli K-12 MG1655
<220> yibi b3599
<222> 264-363
<223> yibi b3599 U00096 complement(3769009..3769371)
<313> 2656: 332-351
<400> 2656
TGACCCGGAA ATCGCCGCAC TTTCTGACCG AATTTCCCGG CTGGAGCATC 50
AACTCGCCGC CGAGAAAAAG ACTGACTATT CCACGTTCCC GGAGATCTAA 100
<210> 2657
<211> 100
<212> DNA
<213> Escherichia coli K-12 MG1655
<220> yibi b3599
<222> 238-337
<223> yibi b3599 U00096 complement(3769009..3769371)
<313> 2657: 281-300
<400> 2657
CATGTTGCGT CGCAGGAGAA AGCGACTGAC CCGGAAATCG CCGCACTTTC 50
TGACCGAATT TCCCGGCTGG AGCATCAACT CGCCGCCGAG AAAAAGACTG 100
<210> 2658
<211> 100
<212> DNA
<213> Escherichia coli K-12 MG1655
<220> yibi b3599
<222> 208-307
<223> yibi b3599 U00096 complement(3769009..3769371)
<313> 2658: 226-245
<400> 2658
CAACCGGAGC GTGGCTGGGG TATGCAGTCA CATGTTGCGT CGCAGGAGAA 50
AGCGACTGAC CCGGAAATCG CCGCACTTTC TGACCGAATT TCCCGGCTGG 100
<210> 2659
<211> 100
<212> DNA
<213> Escherichia coli K-12 MG1655
<220> entf b0587
<222> 3783-3882
<223> entf b0587 U00096 613380..617261
<313> 2659: 3846-3865
<400> 2659
GCTGGATATC TATCGTCAGG ATTGTGCGCA TGTGGATATT ATCTCTCCAG 50

GGACGTTTGA AAAAATTGGG CCGATTATTC GCGCAACGCT AAACAGGTAA 100
<210> 2660
<211> 100
<212> DNA
<213> Escherichia coli K-12 MG1655
<220> entf b0587
<222> 3740-3839
<223> entf b0587 U00096 613380..617261
<313> 2660: 3758-3777
<400> 2660
AAGGTATGAG TCCCGAACGC GCCTGGTCGC CGTGGATAGC GGAGCTGGAT 50
ATCTATCGTC AGGATTGTGC GCATGTGGAT ATTATCTCTC CAGGGACGTT 100
<210> 2661
<211> 100
<212> DNA
<213> Escherichia coli K-12 MG1655
<220> entf b0587
<222> 3667-3766
<223> entf b0587 U00096 613380..617261
<313> 2661: 3723-3742
<400> 2661
CTGCTGACGA CTGCTCATAG CGTACCGTTT GACGGTAAAG CGACGCTGTT 50
TGTTGCTGAA CGCACACTTC AGGAAGGTAT GAGTCCCGAA CGCGCCTGGT 100
<210> 2662
<211> 100
<212> DNA
<213> Escherichia coli K-12 MG1655
<220> yibj b3596
<222> 537-636
<223> yibj b3596 U00096 3764848..3765549
<313> 2662: 579-598
<400> 2662
CGGGGGGCAT GGTACCCCCA CATCTATTGA ATCCGCAACG CGCAGTATCA 50
TGACAGCTAA AGATCTAGCA TATCTAATTA AATTTGATGG GAATTATAAA 100
<210> 2663
<211> 100
<212> DNA
<213> Escherichia coli K-12 MG1655
<220> yibj b3596
<222> 482-581
<223> yibj b3596 U00096 3764848..3765549
<313> 2663: 527-546
<400> 2663
GTGATCTTAT CCATTCTGTA GCTGATGAGA TTAATATCCC AGGCGTTTTC 50
ACAATCGGGG GGCATGGTAC CCCCACATCT ATTGAATCCG CAACGCGCAG 100
<210> 2664
<211> 100
<212> DNA
<213> Escherichia coli K-12 MG1655
<220> yibj b3596
<222> 364-463
<223> yibj b3596 U00096 3764848..3765549
<313> 2664: 396-415
<400> 2664
TATATCACTC AGGATCCGAT TGGGCTGAAG GGGGGATGGA ATTTTTATCA 50
GTATCCGTTG AATCCGGTCA TAAATGTAGA TCCGCAAGGT TTGGTTGATA 100
<210> 2665
<211> 100
<212> DNA
<213> Escherichia coli K-12 MG1655
<220> yibk b3607
<222> 375-474
<223> yibk b3607 U00096 3778842..3779315
<313> 2665: 412-431
<400> 2665
GCCGGACAGC CGCAGCATGA ATCTGTCCAA TGCGGTGTCG GTAGTGGTGT 50
ATGAAGCCTG GCGGCAGTTG GGGTATCCGG GAGCGGTATT GAGAGATTAG 100
<210> 2666
<211> 100
<212> DNA
<213> Escherichia coli K-12 MG1655

<220> yibk b3607
<222> 308-407
<223> yibk b3607 U00096 3778842..3779315
<313> 2666: 341-360
<400> 2666
CACGCGGCCT GCCAGCGAGC ATTCTTGATG CCCTGCCCGC TGAACAAAAA 50
ATTCGCATTC CGATGGTGCC GGACAGCCGC AGCATGAATC TGTCCAATGC 100
<210> 2667
<211> 100
<212> DNA
<213> Escherichia coli K-12 MG1655
<220> yibk b3607
<222> 252-351
<223> yibk b3607 U00096 3778842..3779315
<313> 2667: 306-325
<400> 2667
TGCTCACAGC GCCGTAAGCT ATCAGGATGG CGATTATCTG ATGTTCGGCC 50
CGGAAACACG CGGCCTGCCA GCGAGCATTC TTGATGCCCT GCCCGCTGAA 100
<210> 2668
<211> 100
<212> DNA
<213> Escherichia coli K-12 MG1655
<220> yibl b3603
<222> 224-323
<223> yibl b3603 U00096 3774292..3774654
<313> 2668: 255-274
<400> 2668
CAATTACCAA AAAAGAGCAG GCTGATATGG GCAAGCTGAA GAAAAGTGTT 50
CGCGGACTGG TCGTTGTGCA CCCAATGACC GCACTGGGCC GCGAAATGGG 100
<210> 2669
<211> 100
<212> DNA
<213> Escherichia coli K-12 MG1655
<220> yibl b3603
<222> 133-232
<223> yibl b3603 U00096 3774292..3774654
<313> 2669: 199-218
<400> 2669
CTGGAAGCAG AAATTGCTCG CCTGCGTGAA GTTCATAGCC AAAAACTGAG 50
TAAAGAAGCA CAGAAGCTGA TGAAGATGCC ATTCCAGCGC GCAATTACCA 100
<210> 2670
<211> 100
<212> DNA
<213> Escherichia coli K-12 MG1655
<220> yibl b3603
<222> 131-230
<223> yibl b3603 U00096 3774292..3774654
<313> 2670: 148-167
<400> 2670
CGCTGGAAGC AGAAATTGCT CGCCTGCGTG AAGTTCATAG CCAAAAACTG 50
AGTAAAGAAG CACAGAAGCT GATGAAGATG CCATTCCAGC GCGCAATTAC 100
<210> 2671
<211> 100
<212> DNA
<213> Escherichia coli K-12 MG1655
<220> b2931 b2932
<222> 866-965
<223> b2931 b2932 U00096 complement(3074199..3075188)
<313> 2671: 914-933
<400> 2671
AAACCGATCC GCTGTTTAAA GAGTTGGCGC GGCTGGTTGA GGAAACGCAC 50
GGCATCTGGA ATGAACAGGC CGAGAAATAT CTGCTGGCAC AATTTGGCGT 100
<210> 2672
<211> 100
<212> DNA
<213> Escherichia coli K-12 MG1655
<220> b2931 b2932
<222> 837-936
<223> b2931 b2932 U00096 complement(3074199..3075188)
<313> 2672: 860-879
<400> 2672

TGCCATTGCC GATTTTGCCG AAAAAGGCAA AACCGATCCG CTGTTTAAAG 50
AGTTGGCGCG GCTGGTTGAG GAAACGCACG GCATCTGGAA TGAACAGGCC 100
<210> 2673
<211> 100
<212> DNA
<213> Escherichia coli K-12 MG1655
<220> b2931 b2932
<222> 773-872
<223> b2931 b2932 U00096 complement(3074199..3075188)
<313> 2673: 789-808
<400> 2673
TCAATCTGCC GGATATCCCT GGCGGTAAAA AACTCATTTA TAACGGCGTG 50
ACCATGCCGC TCACTGCCAT TGCCGATTTT GCCGAAAAAG GCAAAACCGA 100
<210> 2674
<211> 100
<212> DNA
<213> Escherichia coli K-12 MG1655
<220> yibn b3612
<222> 333-432
<223> yibn b3612 U00096 complement(3782211..3782642)
<313> 2674: 399-418
<400> 2674
AAACGCACTG ACGAAAGCTG GTTTTGCGCA AGTATTCGTG CTGAAAGAAG 50
GCGTCGCTGG CTGGGCTGGC GAAAACTTGC CTTTGGTGCG CGGCAAATAA 100
<210> 2675
<211> 100
<212> DNA
<213> Escherichia coli K-12 MG1655
<220> yibn b3612
<222> 200-299
<223> yibn b3612 U00096 complement(3782211..3782642)
<313> 2675: 223-242
<400> 2675
AAGGCCATAT CGCAGGTTCT ATTAATCTGT TGCCGAGCGA AATCAAAGCC 50
AACAATGTTG GTGAGCTTGA GAAGCACAAA GACAAACCGG TTATCGTGGT 100
<210> 2676
<211> 100
<212> DNA
<213> Escherichia coli K-12 MG1655
<220> yibn b3612
<222> 129-228
<223> yibn b3612 U00096 complement(3782211..3782642)
<313> 2676: 186-205
<400> 2676
TGAAGCTACG CGTCTGATCA ACAAAGAAGA CGCTGTGGTT GTGGATTTAC 50
GTCAGCGTGA TGACTTCCGT AAAGGCCATA TCGCAGGTTC TATTAATCTG 100
<210> 2677
<211> 100
<212> DNA
<213> Escherichia coli K-12 MG1655
<220> yibo b3613
<222> 1446-1545
<223> yibo b3613 U00096 3782887..3784431
<313> 2677: 1507-1526
<400> 2677
AGGCGGCAAA CTTTCTGACA TCGCGCCGAC CATGTTGTCG CTGATGGGTA 50
TGGAAATCCC GCAAGAGATG ACTGGTAAGC CGCTGTTCAT CGTGGAATAA 100
<210> 2678
<211> 100
<212> DNA
<213> Escherichia coli K-12 MG1655
<220> yibo b3613
<222> 1427-1526
<223> yibo b3613 U00096 3782887..3784431
<313> 2678: 1479-1498
<400> 2678
AGAACGTGAA AGCGGTTGAA GGCGGCAAAC TTTCTGACAT CGCGCCGACC 50
ATGTTGTCGC TGATGGGTAT GGAAATCCCG CAAGAGATGA CTGGTAAGCC 100
<210> 2679
<211> 100
<212> DNA

<213> Escherichia coli K-12 MG1655
<220> yibo b3613
<222> 1375-1474
<223> yibo b3613 U00096 3782887..3784431
<313> 2679: 1430-1449
<400> 2679
GCACACACGG CACACACCAA CCTGCCAGTT CCGCTGATTT ACGTTGGTGA 50
TAAGAACGTG AAAGCGGTTG AAGGCGGCAA ACTTTCTGAC ATCGCGCCGA 100
<210> 2680
<211> 100
<212> DNA
<213> Escherichia coli K-12 MG1655
<220> yibp b3614
<222> 1090-1189
<223> yibp b3614 U00096 3784441..3785724
<313> 2680: 1106-1125
<400> 2680
GTTGAGCATG GTAAAGGCGA CATGAGTCTT TACGGCTATA ATCAGAGCGC 50
ACTGGTGAGC GTTGGTTCGC AGGTTCGCGC GGGCCAGCCG ATTGCACTGG 100
<210> 2681
<211> 100
<212> DNA
<213> Escherichia coli K-12 MG1655
<220> yibp b3614
<222> 1028-1127
<223> yibp b3614 U00096 3784441..3785724
<313> 2681: 1069-1088
<400> 2681
CGATTGCCGA CGGTCGGGTG ATTCTGGCTG ACTGGCTGCA AGGCTACGGT 50
CTGGTGGTGG TGGTTGAGCA TGGTAAAGGC GACATGAGTC TTTACGGCTA 100
<210> 2682
<211> 100
<212> DNA
<213> Escherichia coli K-12 MG1655
<220> yibp b3614
<222> 946-1045
<223> yibp b3614 U00096 3784441..3785724
<313> 2682: 989-1008
<400> 2682
CATCGCTATG GCGAACAGCT ACAGGGTGAA TTACGCTGGA AAGGTATGGT 50
TATCGGTGCT TCTGAAGGTA CTGAAGTTAA AGCGATTGCC GACGGTCGGG 100
<210> 2683
<211> 100
<212> DNA
<213> Escherichia coli K-12 MG1655
<220> yibq b3615
<222> 735-834
<223> yibq b3615 U00096 3785854..3786687
<313> 2683: 804-823
<400> 2683
AAGCATCAGC CAGAGCACGC TGATCGTTTA CTTCCAGCAT CAGTGGCAAG 50
GCTGGGGCAA ACAGCCCGAA GCGGCGAAGT TTAACGCTAG CGCAAATTAA 100
<210> 2684
<211> 100
<212> DNA
<213> Escherichia coli K-12 MG1655
<220> yibq b3615
<222> 658-757
<223> yibq b3615 U00096 3785854..3786687
<313> 2684: 683-702
<400> 2684
CGTGGCGTGA AGCTGTGCAA ACCGAAGAAA CCGATAGAAC CTGTTTATGC 50
TAATCGCTTC TTTGAAGTAT TAAGCGAAAG CATCAGCCAG AGCACGCTGA 100
<210> 2685
<211> 100
<212> DNA
<213> Escherichia coli K-12 MG1655
<220> yibq b3615
<222> 606-705
<223> yibq b3615 U00096 3785854..3786687
<313> 2685: 644-663

<400> 2685
TACTTCTACA CCGCCGAAAA ACGCTGTGCC TGATGCACCG CGTAATCCGT 50
TCCGTGGCGT GAAGCTGTGC AAACCGAAGA AACCGATAGA ACCTGTTTAT 100
<210> 2686
<211> 100
<212> DNA
<213> Escherichia coli K-12 MG1655
<220> smf_1 b3287
<222> 621-720
<223> smf_1 b3287 U00096 complement(3430436..3431197)
<313> 2686: 668-687
<400> 2686
TTTCCCACGA AGAAATCGCA TTATCAGTGG TCTAAGTAAA GGTGTACTGG 50
TGGTGGAAGC GGCTTTGCGT AGTGGTTCGC TGGTGACAGC ACGTTGTGCG 100
<210> 2687
<211> 100
<212> DNA
<213> Escherichia coli K-12 MG1655
<220> smf_1 b3287
<222> 555-654
<223> smf_1 b3287 U00096 complement(3430436..3431197)
<313> 2687: 620-639
<400> 2687
TCTGCTTGAA CAGGGGGGCG CTCTCGTCTC GGAATTTCCC CTCGATGTTC 50
CACCCCTTGC TTACAATTTC CCACGAAGAA ATCGCATTAT CAGTGGTCTA 100
<210> 2688
<211> 100
<212> DNA
<213> Escherichia coli K-12 MG1655
<220> smf_1 b3287
<222> 474-573
<223> smf_1 b3287 U00096 complement(3430436..3431197)
<313> 2688: 524-543
<400> 2688
GGTAAATGGC GTCAGCATTG CTGTATTGGG GAATGGACTT AATACCATTC 50
ATCCCCGCCG TCATGCCCGA CTGGCTGCCA GTCTGCTTGA ACAGGGGGGC 100
<210> 2689
<211> 100
<212> DNA
<213> Escherichia coli K-12 MG1655
<220> smf_2 b3286
<222> 167-266
<223> smf_2 b3286 U00096 complement(3430074..3430382)
<313> 2689: 217-236
<400> 2689
CTGAACGTGC CGGCCAACCT GTGCCAGAGG TAGTTACTCA ACTACTCGAA 50
CTGGAGTTAG CAGGATGGAT CGCAGCTGTA CCCGGCGGCT ATGTCCGATT 100
<210> 2690
<211> 100
<212> DNA
<213> Escherichia coli K-12 MG1655
<220> smf_2 b3286
<222> 95-194
<223> smf_2 b3286 U00096 complement(3430074..3430382)
<313> 2690: 132-151
<400> 2690
AAGACGTGGC ATTGCCATTT CCTGAGCTCC TGGCTAACGT AGGAGATGAG 50
GTAACACCTG TTGACGTCGT CGCTGAACGT GCCGGCCAAC CTGTGCCAGA 100
<210> 2691
<211> 100
<212> DNA
<213> Escherichia coli K-12 MG1655
<220> smf_2 b3286
<222> 62-161
<223> smf_2 b3286 U00096 complement(3430074..3430382)
<313> 2691: 83-102
<400> 2691
CCCCTGAAAA TTCATTTTAT TCACCAGATC AGCAAGACGT GGCATTGCCA 50
TTTCCTGAGC TCCTGGCTAA CGTAGGAGAT GAGGTAACAC CTGTTGACGT 100
<210> 2692
<211> 100

<212> DNA
<213> Escherichia coli K-12 MG1655
<220> dbpa b1344
<222> 1275-1374
<223> dbpa b1344 U00096 1407535..1408908
<313> 2692: 1336-1355
<400> 2692
CTATGTCGCG GTCCGTCAGG CTGTTGCTCA TAAAGCATGG AAACAGTTAC 50
AGGGCGGGAA GATTAAAGGA AAAACGTGCC GGGTGCGGTT ATTAAAATAA 100
<210> 2693
<211> 100
<212> DNA
<213> Escherichia coli K-12 MG1655
<220> dbpa b1344
<222> 1249-1348
<223> dbpa b1344 U00096 1407535..1408908
<313> 2693: 1304-1323
<400> 2693
AAAATCGCCG TGCATCCGGC GCATGTCTAT GTCGCGGTCC GTCAGGCTGT 50
TGCTCATAAA GCATGGAAAC AGTTACAGGG CGGGAAGATT AAAGGAAAAA 100
<210> 2694
<211> 100
<212> DNA
<213> Escherichia coli K-12 MG1655
<220> dbpa b1344
<222> 1101-1200
<223> dbpa b1344 U00096 1407535..1408908
<313> 2694: 1115-1134
<400> 2694
AACGCCGCCA GCTAATAGTT CCATTGCGAC GCTGGAAGCA GAAATGGCAA 50
CGTTGTGTAT CGATGGCGGG AAAAAAGCCA AAATGCGCCC GGGTGATGTA 100
<210> 2695
<211> 100
<212> DNA
<213> Escherichia coli K-12 MG1655
<220> yicc b3645
<222> 765-864
<223> yicc b3645 U00096 3814303..3815166
<313> 2695: 845-864
<400> 2695
TCTTGCGTCG AAGTCTATCA ATGCCGAAGT GACAAACTCC GCCATCGAGC 50
TGAAAGTGTT GATTGAGCAG ATGCGCGAGC AGATTCAGAA CATCGAATAA 100
<210> 2696
<211> 100
<212> DNA
<213> Escherichia coli K-12 MG1655
<220> yicc b3645
<222> 741-840
<223> yicc b3645 U00096 3814303..3815166
<313> 2696: 788-807
<400> 2696
GGAGTTCAAC CGCGAGTCGA ACACTCTTGC GTCGAAGTCT ATCAATGCCG 50
AAGTGACAAA CTCCGCCATC GAGCTGAAAG TGTTGATTGA GCAGATGCGC 100
<210> 2697
<211> 100
<212> DNA
<213> Escherichia coli K-12 MG1655
<220> yicc b3645
<222> 703-802
<223> yicc b3645 U00096 3814303..3815166
<313> 2697: 731-750
<400> 2697
AAAGAAGCGG TTGGTCGTCG TCTGGATTTT ATGATGCAGG AGTTCAACCG 50
CGAGTCGAAC ACTCTTGCGT CGAAGTCTAT CAATGCCGAA GTGACAAACT 100
<210> 2698
<211> 100
<212> DNA
<213> Escherichia coli K-12 MG1655
<220> yice b3655
<222> 1293-1392
<223> yice b3655 U00096 3826572..3827963

<313> 2698: 1361-1380
<400> 2698
TGCCCCTGAA TGGCTGAAAA ACCTGCTCTC CTCCGGGATC GCCGCGGGCG 50
GTATTACTGC CATCGTGCTG AATCTGATTT TCCCACCAGA AAAACAGTAA 100
<210> 2699
<211> 100
<212> DNA
<213> Escherichia coli K-12 MG1655
<220> yice b3655
<222> 1284-1383
<223> yice b3655 U00096 3826572..3827963
<313> 2699: 1308-1327
<400> 2699
TTTGCAGTTT GCCCCTGAAT GGCTGAAAAA CCTGCTCTCC TCCGGGATCG 50
CCGCGGGCGG TATTACTGCC ATCGTGCTGA ATCTGATTTT CCCACCAGAA 100
<210> 2700
<211> 100
<212> DNA
<213> Escherichia coli K-12 MG1655
<220> yice b3655
<222> 1193-1292
<223> yice b3655 U00096 3826572..3827963
<313> 2700: 1211-1230
<400> 2700
TCGTTTCTCG TGAGCCGCTG AACCGTCGGG CGATTCTGAT TATCGCGCTG 50
TCGCTGGCGG TTGGTCTGGG CGTGTCTCAG CAGCCGCTGA TTTTGCAGTT 100
<210> 2701
<211> 100
<212> DNA
<213> Escherichia coli K-12 MG1655
<220> yicf b3648
<222> 1561-1660
<223> yicf b3648 U00096 complement(3817115..3818803)
<313> 2701: 1596-1615
<400> 2701
TTCTGGCAGC AGCTGCCGGG GACTGGATCG GGACGCGCCA GACAGGTTAT 50
TGAATGGAAG GAAAATGCGC AAATCAAGAA GCTGGGCAGT TGGCTGGCTG 100
<210> 2702
<211> 100
<212> DNA
<213> Escherichia coli K-12 MG1655
<220> yicf b3648
<222> 1513-1612
<223> yicf b3648 U00096 complement(3817115..3818803)
<313> 2702: 1553-1572
<400> 2702
GCCAGTGATG AACGGTCCTG GTCGCAACTT TTATTTAGCA CGGAGCAGTT 50
CTGGCAGCAG CTGCCGGGGA CTGGATCGGG ACGCGCCAGA CAGGTTATTG 100
<210> 2703
<211> 100
<212> DNA
<213> Escherichia coli K-12 MG1655
<220> yicf b3648
<222> 1445-1544
<223> yicf b3648 U00096 complement(3817115..3818803)
<313> 2703: 1478-1497
<400> 2703
CTCGTAAGCA GCCTTTTACT CGCTGGGTGA TGGCAATGGG AATACCGCTA 50
ACCCGGGCGG CGCTTAATGC CAGTGATGAA CGGTCCTGGT CGCAACTTTT 100
<210> 2704
<211> 100
<212> DNA
<213> Escherichia coli K-12 MG1655
<220> yicg b3647
<222> 553-652
<223> yicg b3647 U00096 3816447..3817118
<313> 2704: 580-599
<400> 2704
GTTTCTAACC ATGATGTGGT AATTATCTCC ACCCTGGTAT TCGGCTTTTT 50
TGCCCGCTTA CTGGCGCTAC GCCTTAAGCT GGGATTACCG GTCTTTTACT 100
<210> 2705

<211> 100
<212> DNA
<213> Escherichia coli K-12 MG1655
<220> yicg b3647
<222> 509-608
<223> yicg b3647 U00096 3816447..3817118
<313> 2705: 555-574
<400> 2705
TCTCATTTGC CTCCGCCGTG CTGTACATCG CGCTGCAACA CTATGTTTCT 50
AACCATGATG TGGTAATTAT CTCCACCCTG GTATTCGGCT TTTTTGCCCG 100
<210> 2706
<211> 100
<212> DNA
<213> Escherichia coli K-12 MG1655
<220> yicg b3647
<222> 336-435
<223> yicg b3647 U00096 3816447..3817118
<313> 2706: 385-404
<400> 2706
GCTCGGACTG GTGGTCTTTT CTATCATCGG GGCACAGGTT GCACTGGATA 50
TGGGGCACGG CCCAATTATT GCCGTTGTCG CGGCGGTAAC CACCGGCGTG 100
<210> 2707
<211> 100
<212> DNA
<213> Escherichia coli K-12 MG1655
<220> yich b3656
<222> 1611-1710
<223> yich b3656 U00096 3828084..3829793
<313> 2707: 1682-1701
<400> 2707
ATTGCATGCC GTGAACGCCG CAAAGCAGCA AGTGACTCAA ACCATGAATG 50
CTGGCATCGT TTCCAGCGGT GAAGTTACAT CGACGGAGCC GGTGCGGTAA 100
<210> 2708
<211> 100
<212> DNA
<213> Escherichia coli K-12 MG1655
<220> yich b3656
<222> 1580-1679
<223> yich b3656 U00096 3828084..3829793
<313> 2708: 1645-1664
<400> 2708
ATGTCCCGTT GAAACCTACG GTTAGCGGGC AATTGCATGC CGTGAACGCC 50
GCAAAGCAGC AAGTGACTCA AACCATGAAT GCTGGCATCG TTTCCAGCGG 100
<210> 2709
<211> 100
<212> DNA
<213> Escherichia coli K-12 MG1655
<220> yich b3656
<222> 1527-1626
<223> yich b3656 U00096 3828084..3829793
<313> 2709: 1580-1599
<400> 2709
GTTGACTGGC GACGGCAATA TTCAGCTTAC CGCCAGTGGC GATATTCAGG 50
CCAATGTCCC GTTGAAACCT ACGGTTAGCG GGCAATTGCA TGCCGTGAAC 100
<210> 2710
<211> 100
<212> DNA
<213> Escherichia coli K-12 MG1655
<220> yici b3657
<222> 2220-2319
<223> yici b3657 U00096 complement(3829846..3832164)
<313> 2710: 2296-2315
<400> 2710
TGTTGTGAAA GTAAATGGTC TGCAAGACGG TTCGCAGGCT GAAAGTGAGC 50
AGGGGCTGGT GGTGAAGCCT CAAGGGAATG CGCTGACAAT TACGTTGTAA 100
<210> 2711
<211> 100
<212> DNA
<213> Escherichia coli K-12 MG1655
<220> yici b3657
<222> 2191-2290

<223> yici b3657 U00096 complement(3829846..3832164)
<313> 2711: 2256-2275
<400> 2711
GCGAAGAACT GGACACTGTG CCTGCGCAAT GTTGTGAAAG TAAATGGTCT 50
GCAAGACGGT TCGCAGGCTG AAAGTGAGCA GGGGCTGGTG GTGAAGCCTC 100
<210> 2712
<211> 100
<212> DNA
<213> Escherichia coli K-12 MG1655
<220> yici b3657
<222> 2130-2229
<223> yici b3657 U00096 complement(3829846..3832164)
<313> 2712: 2190-2209
<400> 2712
GATCTTTACT TTAAAAGCAG CACGTACTGG CAACACGATT ACTGTGACTG 50
GTGCGGGCGA GGCGAAGAAC TGGACACTGT GCCTGCGCAA TGTTGTGAAA 100
<210> 2713
<211> 100
<212> DNA
<213> Escherichia coli K-12 MG1655
<220> yicj b3658
<222> 1341-1440
<223> yicj b3658 U00096 complement(3832174..3833613)
<313> 2713: 1413-1432
<400> 2713
CTCACTCACG ACGCACAATC TGAAAACCGT TATGGAACAG CTGGCTCAGG 50
GTAAACGCCG TTGCCAGCAA CAATTCACCT CTCAAGAAGT GCAGAACTAA 100
<210> 2714
<211> 100
<212> DNA
<213> Escherichia coli K-12 MG1655
<220> yicj b3658
<222> 1326-1425
<223> yicj b3658 U00096 complement(3832174..3833613)
<313> 2714: 1344-1363
<400> 2714
CGCTAAACGC TACTACTCAC TCACGACGCA CAATCTGAAA ACCGTTATGG 50
AACAGCTGGC TCAGGGTAAA CGCCGTTGCC AGCAACAATT CACCTCTCAA 100
<210> 2715
<211> 100
<212> DNA
<213> Escherichia coli K-12 MG1655
<220> yicj b3658
<222> 1286-1385
<223> yicj b3658 U00096 complement(3832174..3833613)
<313> 2715: 1318-1337
<400> 2715
CGATTGTTCC GGCGATCTGT TATTTGCTGA GCGCGATTAT CGCTAAACGC 50
TACTACTCAC TCACGACGCA CAATCTGAAA ACCGTTATGG AACAGCTGGC 100
<210> 2716
<211> 100
<212> DNA
<213> Escherichia coli K-12 MG1655
<220> yick b3660
<222> 945-1044
<223> yick b3660 U00096 3834580..3835764
<313> 2716: 1011-1030
<400> 2716
TAATGCCATC TTCCTTGGTA TTCTCTGTGG TATCGGCATG CTTTATTTTC 50
AGGACTTGAT GCCTGAAAAA ATAGGCTCTG CGACAACGTT ATATGCAAAT 100
<210> 2717
<211> 100
<212> DNA
<213> Escherichia coli K-12 MG1655
<220> yick b3660
<222> 888-987
<223> yick b3660 U00096 3834580..3835764
<313> 2717: 926-945
<400> 2717
TTACGCCAGC GTACTCATGG CGACGACTCC GGCGGTTGAG CTGGAATTGC 50
AAATTCTTAA TGCCATCTTC CTTGGTATTC TCTGTGGTAT CGGCATGCTT 100

<210> 2718
<211> 100
<212> DNA
<213> Escherichia coli K-12 MG1655
<220> yick b3660
<222> 843-942
<223> yick b3660 U00096 3834580..3835764
<313> 2718: 896-915
<400> 2718
CAAGCGACTA TTAATGCTCA TTGCTATCGT GAGTGGAATG TGTTTTTACG 50
CCAGCGTACT CATGGCGACG ACTCCGGCGG TTGAGCTGGA ATTGCAAATT 100
<210> 2719
<211> 100
<212> DNA
<213> Escherichia coli K-12 MG1655
<220> yicl b3661
<222> 825-924
<223> yicl b3661 U00096 3835875..3836798
<313> 2719: 869-888
<400> 2719
CTGGCTGGGA ACGCTGCTGA TTCTGTCATC GGTGATTTTG ATTTCAATGG 50
ATTCCCGTCG CCGCGCCAGA AAAATAAATC GTCCGGCGCG GCATAAGTGA 100
<210> 2720
<211> 100
<212> DNA
<213> Escherichia coli K-12 MG1655
<220> yicl b3661
<222> 742-841
<223> yicl b3661 U00096 3835875..3836798
<313> 2720: 801-820
<400> 2720
GCCAGCATTT TGAGCTGTGC AGAACCATTA AGTAGCGCGC TACTCTCTTT 50
GCTGTTGCTG GGGATCACGT TCACATTACC GGACTGGCTG GGAACGCTGC 100
<210> 2721
<211> 100
<212> DNA
<213> Escherichia coli K-12 MG1655
<220> yicl b3661
<222> 675-774
<223> yicl b3661 U00096 3835875..3836798
<313> 2721: 698-717
<400> 2721
GGTCATTGGT ACGTCCCTGA CATTTAGTCT GTACCTGAAA GGAGCACAAT 50
TAATTGGCGG TCCAAAAGCC AGCATTTTGA GCTGTGCAGA ACCATTAAGT 100
<210> 2722
<211> 100
<212> DNA
<213> Escherichia coli K-12 MG1655
<220> yicm b3663
<222> 1257-1356
<223> yicm b3663 U00096 complement(3838176..3839531)
<313> 2722: 1322-1341
<400> 2722
GGATAATATT GGTCTGACTT CGCCGTTGAT GTTGTCCGGC ACATTGATGT 50
TGCTGACTGC ATTGTTGGTT ACTGCAAAGG TGAAAATGAA GAAATCCTGA 100
<210> 2723
<211> 100
<212> DNA
<213> Escherichia coli K-12 MG1655
<220> yicm b3663
<222> 1236-1335
<223> yicm b3663 U00096 complement(3838176..3839531)
<313> 2723: 1275-1294
<400> 2723
GGCAATCGGC GGTTATGCGC TGGATAATAT TGGTCTGACT TCGCCGTTGA 50
TGTTGTCCGG CACATTGATG TTGCTGACTG CATTGTTGGT TACTGCAAAG 100
<210> 2724
<211> 100
<212> DNA
<213> Escherichia coli K-12 MG1655
<220> yicm b3663

<222> 1199-1298
<223> yicm b3663 U00096 complement(3838176..3839531)
<313> 2724: 1230-1249
<400> 2724
AGGTGGCGGT TATTCAGCTT GCTAATACCT GTGGCGCGGC AATCGGCGGT 50
TATGCGCTGG ATAATATTGG TCTGACTTCG CCGTTGATGT TGTCCGGCAC 100
<210> 2725
<211> 100
<212> DNA
<213> Escherichia coli K-12 MG1655
<220> yicn b3664
<222> 346-445
<223> yicn b3664 U00096 complement(3839577..3840056)
<313> 2725: 394-413
<400> 2725
ATTACCGACG CTCAGGTCAG GCTGGCGCTA GGTTTTCTGC GCGAAACCGA 50
ACCTGAAATG CAGGATATTA ATGCTTTTCA GATGCGCTAT AACGCGTTCT 100
<210> 2726
<211> 100
<212> DNA
<213> Escherichia coli K-12 MG1655
<220> yicn b3664
<222> 317-416
<223> yicn b3664 U00096 complement(3839577..3840056)
<313> 2726: 342-361
<400> 2726
TTTTACAAAA AGCTCGCCTC GCCGCGCCGA TTACCGACGC TCAGGTCAGG 50
CTGGCGCTAG GTTTTCTGCG CGAAACCGAA CCTGAAATGC AGGATATTAA 100
<210> 2727
<211> 100
<212> DNA
<213> Escherichia coli K-12 MG1655
<220> yicn b3664
<222> 270-369
<223> yicn b3664 U00096 complement(3839577..3840056)
<313> 2727: 306-325
<400> 2727
TGTCATTGTC GATGGTAGCG AACAGAACCT GCTGCAATGG CATCGGATTT 50
TACAAAAAGC TCGCCTCGCC GCGCCGATTA CCGACGCTCA GGTCAGGCTG 100
<210> 2728
<211> 100
<212> DNA
<213> Escherichia coli K-12 MG1655
<220> yico b3665
<222> 1314-1413
<223> yico b3665 U00096 complement(3840082..3841494)
<313> 2728: 1376-1395
<400> 2728
CTGCATCATG AAAGTATGCA CCGGGCGCTG GCGCGATCTG AACCTGTGTG 50
TGGTGGTGGT CGCAGCTCTG TTTGCACTGA AGATTATTCT GGTGGATTAG 100
<210> 2729
<211> 100
<212> DNA
<213> Escherichia coli K-12 MG1655
<220> yico b3665
<222> 1266-1365
<223> yico b3665 U00096 complement(3840082..3841494)
<313> 2729: 1310-1329
<400> 2729
CTTTACTTTC TCGATCACCG AAGGGATTGC ACTCGGCTTT ATGTCGTACT 50
GCATCATGAA AGTATGCACC GGGCGCTGGC GCGATCTGAA CCTGTGTGTG 100
<210> 2730
<211> 100
<212> DNA
<213> Escherichia coli K-12 MG1655
<220> yico b3665
<222> 1244-1343
<223> yico b3665 U00096 complement(3840082..3841494)
<313> 2730: 1280-1299
<400> 2730
TTATTACCAC GGTGATGATG CCCTTTACTT TCTCGATCAC CGAAGGGATT 50

GCACTCGGCT TTATGTCGTA CTGCATCATG AAAGTATGCA CCGGGCGCTG 100
<210> 2731
<211> 100
<212> DNA
<213> Escherichia coli K-12 MG1655
<220> yicp b3666
<222> 1668-1767
<223> yicp b3666 U00096 3841591..3843357
<313> 2731: 1730-1749
<400> 2731
GTTTCTTTCT CTGCCAGTGA TCCCCGCGCT AAAACTAACC AGTCAGGGGC 50
TATTTGATGG CGAGAAGTTT GCCTTCACTA CGCTGGAAGT CACGGAATAA 100
<210> 2732
<211> 100
<212> DNA
<213> Escherichia coli K-12 MG1655
<220> yicp b3666
<222> 1643-1742
<223> yicp b3666 U00096 3841591..3843357
<313> 2732: 1690-1709
<400> 2732
CCGATGAGCC GTTTATTCAG ATGGCGTTTC TTTCTCTGCC AGTGATCCCC 50
GCGCTAAAAC TAACCAGTCA GGGGCTATTT GATGGCGAGA AGTTTGCCTT 100
<210> 2733
<211> 100
<212> DNA
<213> Escherichia coli K-12 MG1655
<220> yicp b3666
<222> 1614-1713
<223> yicp b3666 U00096 3841591..3843357
<313> 2733: 1631-1650
<400> 2733
AGCCGCCGCC CGTGAATGCG GTCCGTTACC CGATGAGCCG TTTATTCAGA 50
TGGCGTTTCT TTCTCTGCCA GTGATCCCCG CGCTAAAACT AACCAGTCAG 100
<210> 2734
<211> 100
<212> DNA
<213> Escherichia coli K-12 MG1655
<220> yida b3698
<222> 714-813
<223> yida b3698 U00096 complement(3873768..3874580)
<313> 2734: 775-794
<400> 2734
TAACGCTATT CCTTCAGTGA AAGAAGTGGC GAACTTTGTC ACCAAATCTA 50
ACCTTGAAGA TGGCGTGGCG TTTGCTATTG AGAAGTATGT GCTGAATTAA 100
<210> 2735
<211> 100
<212> DNA
<213> Escherichia coli K-12 MG1655
<220> yida b3698
<222> 661-760
<223> yida b3698 U00096 complement(3873768..3874580)
<313> 2735: 711-730
<400> 2735
CAGGAAAACG ATATCGCAAT GATTGAATAT GCAGGCGTCG GTGTGGCGAT 50
GGATAACGCT ATTCCTTCAG TGAAAGAAGT GGCGAACTTT GTCACCAAAT 100
<210> 2736
<211> 100
<212> DNA
<213> Escherichia coli K-12 MG1655
<220> yida b3698
<222> 590-689
<223> yida b3698 U00096 complement(3873768..3874580)
<313> 2736: 640-659
<400> 2736
AAGGTACGGG GGTGAAATCA CTGGCCGACG TGTTAGGTAT TAAACCGGAA 50
GAAATCATGG CGATTGGCGA TCAGGAAAAC GATATCGCAA TGATTGAATA 100
<210> 2737
<211> 100
<212> DNA
<213> Escherichia coli K-12 MG1655

<220> yidb b3699
<222> 309-408
<223> yidb b3699 U00096 complement(3874695..3875102)
<313> 2737: 372-391
<400> 2737
TATTGATGCG CTCTCACCGC AAGGTGAAGT GTCACCACAA GCCAATAACG 50
ATCTGCTTTC CGCAGGCATG GAACTGCTGA AAGGGAAACT CTTCCGCTAA 100
<210> 2738
<211> 100
<212> DNA
<213> Escherichia coli K-12 MG1655
<220> yidb b3699
<222> 288-387
<223> yidb b3699 U00096 complement(3874695..3875102)
<313> 2738: 348-367
<400> 2738
GGCAGAACAA TTGCCGAAGA TTATTGATGC GCTCTCACCG CAAGGTGAAG 50
TGTCACCACA AGCCAATAAC GATCTGCTTT CCGCAGGCAT GGAACTGCTG 100
<210> 2739
<211> 100
<212> DNA
<213> Escherichia coli K-12 MG1655
<220> yidb b3699
<222> 276-375
<223> yidb b3699 U00096 complement(3874695..3875102)
<313> 2739: 299-318
<400> 2739
TTCCAGTTTA CTGGCAGAAC AATTGCCGAA GATTATTGAT GCGCTCTCAC 50
CGCAAGGTGA AGTGTCACCA CAAGCCAATA ACGATCTGCT TTCCGCAGGC 100
<210> 2740
<211> 100
<212> DNA
<213> Escherichia coli K-12 MG1655
<220> yidc b3706
<222> 1520-1619
<223> yidc b3706 U00096 3882705..3884351
<313> 2740: 1559-1578
<400> 2740
TGTGGTTCCC GTCAGGTCTG GTGCTGTACT ATATCGTCAG CAACCTGGTA 50
ACCATTATTC AGCAGCAGCT GATTTACCGT GGTCTGGAAA AACGTGGCCT 100
<210> 2741
<211> 100
<212> DNA
<213> Escherichia coli K-12 MG1655
<220> yidc b3706
<222> 1473-1572
<223> yidc b3706 U00096 3882705..3884351
<313> 2741: 1504-1523
<400> 2741
GCAGAAGATC ATGACCTTTA TGCCGGTCAT CTTCACCGTG TTCTTCCTGT 50
GGTTCCCGTC AGGTCTGGTG CTGTACTATA TCGTCAGCAA CCTGGTAACC 100
<210> 2742
<211> 100
<212> DNA
<213> Escherichia coli K-12 MG1655
<220> yidc b3706
<222> 1419-1518
<223> yidc b3706 U00096 3882705..3884351
<313> 2742: 1465-1484
<400> 2742
AACGATGTTC TTCATTCAGA AGATGTCGCC GACCACAGTG ACCGACCCGA 50
TGCAGCAGAA GATCATGACC TTTATGCCGG TCATCTTCAC CGTGTTCTTC 100
<210> 2743
<211> 100
<212> DNA
<213> Escherichia coli K-12 MG1655
<220> yide b3686
<222> 1587-1686
<223> yide b3686 U00096 complement(3862239..3863924)
<313> 2743: 1654-1673
<400> 2743

TGCGGCGGCG CTCTCTTACG CCACTGTCTA TCCGTTGGTA ATGTTCCTGC 50
GCATTATCAC CCCCCAATTA CTGGCGGTGC TCTTCTGGAG TATCGGTTAA 100
<210> 2744
<211> 100
<212> DNA
<213> Escherichia coli K-12 MG1655
<220> yide b3686
<222> 1565-1664
<223> yide b3686 U00096 complement(3862239..3863924)
<313> 2744: 1596-1615
<400> 2744
ATAATCTTCA TCCAACCAGC GGTGCGGCGG CGCTCTCTTA CGCCACTGTC 50
TATCCGTTGG TAATGTTCCT GCGCATTATC ACCCCCCAAT TACTGGCGGT 100
<210> 2745
<211> 100
<212> DNA
<213> Escherichia coli K-12 MG1655
<220> yide b3686
<222> 1532-1631
<223> yide b3686 U00096 complement(3862239..3863924)
<313> 2745: 1555-1574
<400> 2745
CCATGACCGA TCCTCCGGCG CTGGCGTTTG CTAATAATCT TCATCCAACC 50
AGCGGTGCGG CGGCGCTCTC TTACGCCACT GTCTATCCGT TGGTAATGTT 100
<210> 2746
<211> 100
<212> DNA
<213> Escherichia coli K-12 MG1655
<220> yidf b3675
<222> 380-479
<223> yidf b3675 U00096 complement(3852741..3853238)
<313> 2746: 407-426
<400> 2746
GCGTGCTTTG TGCGGGCTAT CAGGCGTTTT ATTCTTACTC CGCACCGCAT 50
ATGCGGGTGA TGCGTGATCT GATAAAGCAG CATCGTTCTC CCATGGAGCT 100
<210> 2747
<211> 100
<212> DNA
<213> Escherichia coli K-12 MG1655
<220> yidf b3675
<222> 329-428
<223> yidf b3675 U00096 complement(3852741..3853238)
<313> 2747: 343-362
<400> 2747
CGTGCGACTG GCATCGCCTG TGCCCGCACG CTCGGCAGGA GACGCCAGAC 50
AGCGTGCTTT GTGCGGGCTA TCAGGCGTTT TATTCTTACT CCGCACCGCA 100
<210> 2748
<211> 100
<212> DNA
<213> Escherichia coli K-12 MG1655
<220> yidf b3675
<222> 275-374
<223> yidf b3675 U00096 complement(3852741..3853238)
<313> 2748: 309-328
<400> 2748
CCTTACAAAA ATGGTGTGGC AGCGAAAACC CGCAACCGCG CTGTGACTGC 50
CAGGCGTGCG ACTGGCATCG CCTGTGCCCG CACGCTCGGC AGGAGACGCC 100
<210> 2749
<211> 100
<212> DNA
<213> Escherichia coli K-12 MG1655
<220> yidg b3676
<222> 226-325
<223> yidg b3676 U00096 complement(3853235..3853597)
<313> 2749: 285-304
<400> 2749
GTCACGAATA GCGATTTTTC CCAATTTCAC GTAGTCCGTG ACAAATTTTT 50
GATCTCCCTC GCGGTGTTAT CTCTCGCAAT ACTGTTTGCT GTAACGCATA 100
<210> 2750
<211> 100
<212> DNA

<213> Escherichia coli K-12 MG1655
<220> yidg b3676
<222> 202-301
<223> yidg b3676 U00096 complement(3853235..3853597)
<313> 2750: 234-253
<400> 2750
ACCCGTAATC GCAATTTAAT GGATGTCACG AATAGCGATT TTTCCCAATT 50
TCACGTAGTC CGTGACAAAT TTTTGATCTC CCTCGCGGTG TTATCTCTCG 100
<210> 2751
<211> 100
<212> DNA
<213> Escherichia coli K-12 MG1655
<220> yidg b3676
<222> 156-255
<223> yidg b3676 U00096 complement(3853235..3853597)
<313> 2751: 204-223
<400> 2751
TTCGATTGGC ATTCTCGCCA TCGTGGCGCT GATCCTCTGG CACTACACCC 50
GTAATCGCAA TTTAATGGAT GTCACGAATA GCGATTTTTC CCAATTTCAC 100
<210> 2752
<211> 100
<212> DNA
<213> Escherichia coli K-12 MG1655
<220> yidh b3677
<222> 230-329
<223> yidh b3677 U00096 complement(3853587..3853934)
<313> 2752: 250-269
<400> 2752
ATGAAAAAGC GATGCGTCTG AAAGAGGACC TGCCCTACAC CAACAGCTTA 50
TTAATCATTA GCTTAATATT GATGGTCGTG GCGGTGATCG TTATGGGACT 100
<210> 2753
<211> 100
<212> DNA
<213> Escherichia coli K-12 MG1655
<220> yidh b3677
<222> 161-260
<223> yidh b3677 U00096 complement(3853587..3853934)
<313> 2753: 211-230
<400> 2753
TGCTGGCGTT GTTGTTGTGT CTGTTTTCCG GCGGGTTAGC GATGTATGGC 50
TACTTGCGCT GGTTACGCAA TGAAAAAGCG ATGCGTCTGA AAGAGGACCT 100
<210> 2754
<211> 100
<212> DNA
<213> Escherichia coli K-12 MG1655
<220> yidh b3677
<222> 48-147
<223> yidh b3677 U00096 complement(3853587..3853934)
<313> 2754: 90-109
<400> 2754
GGCTAATGAG CGTACTTTTC TGGCGTGGAT CCGTACTGCA CTAGGATTTC 50
TGGCGGCGGG GGTTGGGCTT GATCAACTTG CGCCAGATTT CGCCACGCCC 100
<210> 2755
<211> 100
<212> DNA
<213> Escherichia coli K-12 MG1655
<220> yidi b3678
<222> 351-450
<223> yidi b3678 U00096 3854042..3854491
<313> 2755: 431-450
<400> 2755
GCTGGCTTTC CATACCTTGC TTTTTGGTAT CGGCATCGTG TGCAGCATTT 50
TGCTGATATT CCTTATCTTT TCCTTCCTGA CCGGCGGTTC ACTGGTTTAA 100
<210> 2756
<211> 100
<212> DNA
<213> Escherichia coli K-12 MG1655
<220> yidi b3678
<222> 291-390
<223> yidi b3678 U00096 3854042..3854491
<313> 2756: 352-371

<400> 2756
CGGTATGCGT AAGGAAAATC GCTGGGGCAT TCGCGGCGCG CTGGTTTTTG  50
GCGGCGGGAC GCTGGCTTTC CATACCTTGC TTTTTGGTAT CGGCATCGTG  100
<210> 2757
<211> 100
<212> DNA
<213> Escherichia coli K-12 MG1655
<220> yidi b3678
<222> 146-245
<223> yidi b3678 U00096 3854042..3854491
<313> 2757: 196-215
<400> 2757
CTGAAATTAA AAGAGGTCTT CTCGCCGGAA TAAAAGGCGA AAAAATTACC  50
ACCGTGGAGA AAAAAGAGGA CGTAGATGTC GATAAAATCC TCAATCAAAG  100
<210> 2758
<211> 100
<212> DNA
<213> Escherichia coli K-12 MG1655
<220> yidj b3679
<222> 1395-1494
<223> yidj b3679 U00096 complement(3854538..3856031)
<313> 2758: 1462-1481
<400> 2758
TCCACGTCCA CAAGATGGCT ATTCGCCAGT TGTACGCGAC TATGACACCG  50
GCCTACCGAC ACAAGGGGTG AAGGTGGAGG AGAAAAAACA GAAGTTCTGA  100
<210> 2759
<211> 100
<212> DNA
<213> Escherichia coli K-12 MG1655
<220> yidj b3679
<222> 1392-1491
<223> yidj b3679 U00096 complement(3854538..3856031)
<313> 2759: 1423-1442
<400> 2759
TCGTCCACGT CCACAAGATG GCTATTCGCC AGTTGTACGC GACTATGACA  50
CCGGCCTACC GACACAAGGG GTGAAGGTGG AGGAGAAAAA ACAGAAGTTC  100
<210> 2760
<211> 100
<212> DNA
<213> Escherichia coli K-12 MG1655
<220> yidj b3679
<222> 1298-1397
<223> yidj b3679 U00096 complement(3854538..3856031)
<313> 2760: 1348-1367
<400> 2760
ATTACATGGA CAAAATTCGC GATCCGTTCC GCAGTTACCA ATGGAGTCTG  50
CGTCCGTGGC GTAAAGATGC ACGGCCGCGC TGGATGGGGG CGTTTCGTCC  100
<210> 2761
<211> 100
<212> DNA
<213> Escherichia coli K-12 MG1655
<220> yidk b3680
<222> 1617-1716
<223> yidk b3680 U00096 complement(3856028..3857743)
<313> 2761: 1681-1700
<400> 2761
CATTGCCGCC GTAGTGATTG TCTACCTGAT TTTTGACAGC TGGCGGCATC  50
GTCACGACCC AGCCGTAACC TTTACTCCCG ACGGGAAGGA TAGCCTATGA  100
<210> 2762
<211> 100
<212> DNA
<213> Escherichia coli K-12 MG1655
<220> yidk b3680
<222> 1581-1680
<223> yidk b3680 U00096 complement(3856028..3857743)
<313> 2762: 1620-1639
<400> 2762
CTACGGTACG CGCTGGTTAG CGATGATCAG TTATTTCATT GCCGCCGTAG  50
TGATTGTCTA CCTGATTTTT GACAGCTGGC GGCATCGTCA CGACCCAGCC  100
<210> 2763
<211> 100

<212> DNA
<213> Escherichia coli K-12 MG1655
<220> yidk b3680
<222> 1473-1572
<223> yidk b3680 U00096 complement(3856028..3857743)
<313> 2763: 1504-1523
<400> 2763
AGATGCGTTT GCGGTGGACA TGAAACCGTG GAAAAACGTC AAGATCGCGT 50
CAATTGGCAT CCTGTTCGCG ATGATTGGCG TCTATGCCGG GCTGGCTGAA 100
<210> 2764
<211> 100
<212> DNA
<213> Escherichia coli K-12 MG1655
<220> yidl b3681
<222> 825-924
<223> yidl b3681 U00096 3857880..3858803
<313> 2764: 894-913
<400> 2764
TCATTTCAGC AAAGCCTTTA AACATAAATT TGGTTATGCG CCGTCAGCCG 50
TGCTAAAGAA TACGGACCAG CACCCAACGG ATGCCAGTCC ACACAATTAA 100
<210> 2765
<211> 100
<212> DNA
<213> Escherichia coli K-12 MG1655
<220> yidl b3681
<222> 805-904
<223> yidl b3681 U00096 3857880..3858803
<313> 2765: 863-882
<400> 2765
CTCAACTTCT TCGACTCCTT TCATTTCAGC AAAGCCTTTA AACATAAATT 50
TGGTTATGCG CCGTCAGCCG TGCTAAAGAA TACGGACCAG CACCCAACGG 100
<210> 2766
<211> 100
<212> DNA
<213> Escherichia coli K-12 MG1655
<220> yidl b3681
<222> 781-880
<223> yidl b3681 U00096 3857880..3858803
<313> 2766: 818-837
<400> 2766
TCGGTTGGCG AAGTCGCTGA TACGCTCAAC TTCTTCGACT CCTTTCATTT 50
CAGCAAAGCC TTTAAACATA AATTTGGTTA TGCGCCGTCA GCCGTGCTAA 100
<210> 2767
<211> 100
<212> DNA
<213> Escherichia coli K-12 MG1655
<220> yidp b3685
<222> 618-717
<223> yidp b3685 U00096 3861526..3862242
<313> 2767: 681-700
<400> 2767
CGAGAGCGGG GAGTTTTTGA ATTATTCAGT GATGTTCAGA AATGCCAGCG 50
AATACCAGGT GGAGTACCAT TTACGGCGAC TCCACCCGGA AAAGAGTTAA 100
<210> 2768
<211> 100
<212> DNA
<213> Escherichia coli K-12 MG1655
<220> yidp b3685
<222> 535-634
<223> yidp b3685 U00096 3861526..3862242
<313> 2768: 584-603
<400> 2768
GTGCTCGCCG ATAGACTGCT GGCGCGGCAA ATGAAGGTAG CGGAACACAC 50
GCCACTGCTG CGGATCACCT CGTTGTCATA TAGCGAGAGC GGGGAGTTTT 100
<210> 2769
<211> 100
<212> DNA
<213> Escherichia coli K-12 MG1655
<220> yidp b3685
<222> 475-574
<223> yidp b3685 U00096 3861526..3862242

<313> 2769: 534-553
<400> 2769
GAATATATTG AACAAGAGTG CGGGATTTTG ATTGGCGGTA ATTATGAAAG 50
CCTGACGCCA GTGCTCGCCG ATAGACTGCT GGCGCGGCAA ATGAAGGTAG 100
<210> 2770
<211> 100
<212> DNA
<213> Escherichia coli K-12 MG1655
<220> fdhd b3896
<222> 735-834
<223> fdhd b3896 U00096 4083596..4084429
<313> 2770: 804-823
<400> 2770
TGCTGTAGAA GTGGCTGAGC GCTGTAATCT GACACTGGTA GGTTTTTGTA 50
AACCGGGTCG GGCAACGGTT TATACCCATC CGCAGCGTTT GAGCAATTAA 100
<210> 2771
<211> 100
<212> DNA
<213> Escherichia coli K-12 MG1655
<220> fdhd b3896
<222> 729-828
<223> fdhd b3896 U00096 4083596..4084429
<313> 2771: 758-777
<400> 2771
CACGCTTGCT GTAGAAGTGG CTGAGCGCTG TAATCTGACA CTGGTAGGTT 50
TTTGTAAACC GGGTCGGGCA ACGGTTTATA CCCATCCGCA GCGTTTGAGC 100
<210> 2772
<211> 100
<212> DNA
<213> Escherichia coli K-12 MG1655
<220> fdhd b3896
<222> 680-779
<223> fdhd b3896 U00096 4083596..4084429
<313> 2772: 730-749
<400> 2772
AGTCGGCGAT GTGCGGCGTA GAGATTTTGT TTGCGGTGTC TGCCGCGACC 50
ACGCTTGCTG TAGAAGTGGC TGAGCGCTGT AATCTGACAC TGGTAGGTTT 100
<210> 2773
<211> 100
<212> DNA
<213> Escherichia coli K-12 MG1655
<220> yidq b3689
<222> 309-408
<223> yidq b3689 U00096 3865280..3865687
<313> 2773: 371-390
<400> 2773
CCGCAAAGAC AGCTCGGTGC GCTCGCGCGT TGAAAAAAGC GAAGCCAACG 50
CGCAAGCCAC CAATGCGGTT ATTCCACCGG CCAGAATGCC TGATAACTGA 100
<210> 2774
<211> 100
<212> DNA
<213> Escherichia coli K-12 MG1655
<220> yidq b3689
<222> 241-340
<223> yidq b3689 U00096 3865280..3865687
<313> 2774: 272-291
<400> 2774
TCGCTGGCGA TTCTCGATAT GCCGTTTACC GCTGTGATGG ACACGCTTTT 50
ACTGCCGTGG GATGTGTTCC GCAAAGACAG CTCGGTGCGC TCGCGCGTTG 100
<210> 2775
<211> 100
<212> DNA
<213> Escherichia coli K-12 MG1655
<220> yidq b3689
<222> 173-272
<223> yidq b3689 U00096 3865280..3865687
<313> 2775: 216-235
<400> 2775
GAACGTATCC CGGCACCCGC GCCAGCGCAA CCATGATTGG CGACGATGAA 50
ACCAACTGGG GCACTAAGTC GCTGGCGATT CTCGATATGC CGTTTACCGC 100
<210> 2776

<211> 100
<212> DNA
<213> Escherichia coli K-12 MG1655
<220> fdhe b3892
<222> 831-930
<223> fdhe b3892 U00096 complement(4077879..4078808)
<313> 2776: 899-918
<400> 2776
AGATGACCTC GCCTCTCTGG TACTGGACGC ACGAATGGAG CAAGAAGGCT 50
ATGCCCGCAG TTCCATCAAC CCGTTCCTGT TTCCGGGTGA AGGAGAGTAA 100
<210> 2777
<211> 100
<212> DNA
<213> Escherichia coli K-12 MG1655
<220> fdhe b3892
<222> 820-919
<223> fdhe b3892 U00096 complement(4077879..4078808)
<313> 2777: 862-881
<400> 2777
GAAGCCGTAG CAGATGACCT CGCCTCTCTG GTACTGGACG CACGAATGGA 50
GCAAGAAGGC TATGCCCGCA GTTCCATCAA CCCGTTCCTG TTTCCGGGTG 100
<210> 2778
<211> 100
<212> DNA
<213> Escherichia coli K-12 MG1655
<220> fdhe b3892
<222> 777-876
<223> fdhe b3892 U00096 complement(4077879..4078808)
<313> 2778: 823-842
<400> 2778
CACTTACCTG AAAATTCTCT ATCAGGAAAA AGATCCAAAA ATCGAAGCCG 50
TAGCAGATGA CCTCGCCTCT CTGGTACTGG ACGCACGAAT GGAGCAAGAA 100
<210> 2779
<211> 100
<212> DNA
<213> Escherichia coli K-12 MG1655
<220> yidr b3690
<222> 1042-1141
<223> yidr b3690 U00096 complement(3865689..3866939)
<313> 2779: 1086-1105
<400> 2779
GCATTTAACT GGCATCCGTC AGGAGAATGG TTGGGCTTTG TGCTGGATAA 50
TCGAATTGCT TGTGCCCATG CGCAAAGCGG CGAGGTTGAG TATTTAACCG 100
<210> 2780
<211> 100
<212> DNA
<213> Escherichia coli K-12 MG1655
<220> yidr b3690
<222> 1008-1107
<223> yidr b3690 U00096 complement(3865689..3866939)
<313> 2780: 1041-1060
<400> 2780
CCAGTTAACC CATAACAAAA CGGATATTCA GTCTGCATTT AACTGGCATC 50
CGTCAGGAGA ATGGTTGGGC TTTGTGCTGG ATAATCGAAT TGCTTGTGCC 100
<210> 2781
<211> 100
<212> DNA
<213> Escherichia coli K-12 MG1655
<220> yidr b3690
<222> 886-985
<223> yidr b3690 U00096 complement(3865689..3866939)
<313> 2781: 917-936
<400> 2781
GTCAACGTCC CGCGCCACTG GGTGCGCTGT AATCCGCAGG GTACACAAAT 50
CGCGTTTTTA ATGCGAGATG ATAACGGCAT TGTGCAACTG TGGCTTATCT 100
<210> 2782
<211> 100
<212> DNA
<213> Escherichia coli K-12 MG1655
<220> fdhf b4080
<222> 2049-2148

<223> fdhf b4080 U00096 complement(4294798..4296945)
<313> 2782: 2124-2143
<400> 2782
TCGCGTCGAG CCGATCGCCG ATCAGCGCGC CGCCGAGCAG TACGTGATTG 50
ACGAGTACAA CAAGTTGAAA ACTCGCCTGC GCGAAGCGGC ACTGGCGTAA 100
<210> 2783
<211> 100
<212> DNA
<213> Escherichia coli K-12 MG1655
<220> fdhf b4080
<222> 2000-2099
<223> fdhf b4080 U00096 complement(4294798..4296945)
<313> 2783: 2042-2061
<400> 2783
AAAACTTAAG CCCGATTACG AAAACGCCGG AGTACAAATA CTGCGCCGTT 50
CGCGTCGAGC CGATCGCCGA TCAGCGCGCC GCCGAGCAGT ACGTGATTGA 100
<210> 2784
<211> 100
<212> DNA
<213> Escherichia coli K-12 MG1655
<220> fdhf b4080
<222> 1961-2060
<223> fdhf b4080 U00096 complement(4294798..4296945)
<313> 2784: 1995-2014
<400> 2784
ACCAGTGGTG GATTGGTGCC TGTAACGAGC TGGTTACCGA AAACTTAAGC 50
CCGATTACGA AAACGCCGGA GTACAAATAC TGCGCCGTTC GCGTCGAGCC 100
<210> 2785
<211> 100
<212> DNA
<213> Escherichia coli K-12 MG1655
<220> yids b3691
<222> 987-1086
<223> yids b3691 U00096 3866983..3868068
<313> 2785: 1067-1086
<400> 2785
CTTCGGCAAG ATAGTAAAAA GCCGATGCCT GACCGCACCG GCTTTAAGAA 50
AGTGGATTAT GCGCAGTGGT GTGGCACATA TTCCACAGTT GAAGGATTAG 100
<210> 2786
<211> 100
<212> DNA
<213> Escherichia coli K-12 MG1655
<220> yids b3691
<222> 964-1063
<223> yids b3691 U00096 3866983..3868068
<313> 2786: 1009-1028
<400> 2786
ACCCGCAAAC TGCGTTTAAA ACTCTTCGGC AAGATAGTAA AAAGCCGATG 50
CCTGACCGCA CCGGCTTTAA GAAAGTGGAT TATGCGCAGT GGTGTGGCAC 100
<210> 2787
<211> 100
<212> DNA
<213> Escherichia coli K-12 MG1655
<220> yids b3691
<222> 939-1038
<223> yids b3691 U00096 3866983..3868068
<313> 2787: 973-992
<400> 2787
GCTCAATACC GCTTACTGGC GCGCCACCCG CAAACTGCGT TTAAAACTCT 50
TCGGCAAGAT AGTAAAAAGC CGATGCCTGA CCGCACCGGC TTTAAGAAAG 100
<210> 2788
<211> 100
<212> DNA
<213> Escherichia coli K-12 MG1655
<220> sdaa sdaA
<222> 1266-1365
<223> sdaa sdaA U00096 1894956..1896320
<313> 2788: 1330-1349
<400> 2788
TAAGGTCATC GAAACGATGT ACGAAACCGG TAAGGACATG AACGCCAAAT 50
ACCGCGAAAC CTCACGCGGT GGTCTGGCAA TCAAAGTCCA GTGTGACTAA 100

<210> 2789
<211> 100
<212> DNA
<213> Escherichia coli K-12 MG1655
<220> sdaa sdaA
<222> 1211-1310
<223> sdaa sdaA U00096 1894956..1896320
<313> 2789: 1274-1293
<400> 2789
TTAACGCCGC GCGGATGGCT CTGCGCCGCA CCAGTGCACC GCGCGTCTCG 50
CTGGATAAGG TCATCGAAAC GATGTACGAA ACCGGTAAGG ACATGAACGC 100
<210> 2790
<211> 100
<212> DNA
<213> Escherichia coli K-12 MG1655
<220> sdaa sdaA
<222> 1135-1234
<223> sdaa sdaA U00096 1894956..1896320
<313> 2790: 1173-1192
<400> 2790
TTAACCTGCG ACCCGGTTGC AGGGCAGGTT CAGGTGCCGT GCATTGAGCG 50
TAATGCCATT GCCTCTGTGA AGGCGATTAA CGCCGCGCGG ATGGCTCTGC 100
<210> 2791
<211> 100
<212> DNA
<213> Escherichia coli K-12 MG1655
<220> sdab b2798
<222> 1263-1362
<223> sdab b2798 U00096 2927598..2928965
<313> 2791: 1308-1327
<400> 2791
CGATAAAGTT ATCGAAACCA TGTACGAAAC AGGTAAAGAT ATGAACGCCA 50
AGTACCGCGA AACCTCTCGC GGCGGCCTGG CAATGAAGAT CGTTGCCTGC 100
<210> 2792
<211> 100
<212> DNA
<213> Escherichia coli K-12 MG1655
<220> sdab b2798
<222> 1157-1256
<223> sdab b2798 U00096 2927598..2928965
<313> 2792: 1194-1213
<400> 2792
GACAGGTACA GGTGCCATGC ATCGAGCGTA ACGCCATTGC GGCAGTAAAA 50
GCGGTGAACG CCGCACGTAT GGCGCTGCGC CGTACCAGCG AGCCGCGCGT 100
<210> 2793
<211> 100
<212> DNA
<213> Escherichia coli K-12 MG1655
<220> sdab b2798
<222> 1019-1118
<223> sdab b2798 U00096 2927598..2928965
<313> 2793: 1069-1088
<400> 2793
AGGGTGAAGT TGGCGTGGCG TGCTCAATGG CGGCGGCTGG TCTGGCAGAA 50
CTATTAGGCG CAAGCCCGGC GCAGGTGTGC ATCGCGGCGG AAATCGCCAT 100
<210> 2794
<211> 100
<212> DNA
<213> Escherichia coli K-12 MG1655
<220> sdac b2797
<222> 1178-1277
<223> sdac b2797 U00096 2926251..2927540
<313> 2794: 1191-1210
<400> 2794
CAATTCAGAA AGTACCGGCA ATGCGTAAGT ACAGCGGTCA CATCAGCAAC 50
GTATTCGTTG TCGTGATGGG TCTGATTGCA ATCTCCGCAA TCTTCTACTC 100
<210> 2795
<211> 100
<212> DNA
<213> Escherichia coli K-12 MG1655
<220> sdac b2797

<222> 1127-1226
<223> sdac b2797 U00096 2926251..2927540
<313> 2795: 1162-1181
<400> 2795
CCCTGGGCGG TCCAATCATC GCGATGATCC TGTTCCTGAT GCCGATGTAC 50
GCAATTCAGA AAGTACCGGC AATGCGTAAG TACAGCGGTC ACATCAGCAA 100
<210> 2796
<211> 100
<212> DNA
<213> Escherichia coli K-12 MG1655
<220> sdac b2797
<222> 1090-1189
<223> sdac b2797 U00096 2926251..2927540
<313> 2796: 1114-1133
<400> 2796
GCCACCCTGA ACCCGAGCAT CCTGGGTATG ATTGAAACCC TGGGCGGTCC 50
AATCATCGCG ATGATCCTGT TCCTGATGCC GATGTACGCA ATTCAGAAAG 100
<210> 2797
<211> 100
<212> DNA
<213> Escherichia coli K-12 MG1655
<220> envr b3265
<222> 548-647
<223> envr b3265 U00096 complement(3410440..3411102)
<313> 2797: 564-583
<400> 2797
GTTATGATCT TTATAAACAA GCCCCCGCTC TGGTCGATAA CGTATTAAGA 50
ATGTTCATGC CAGATGAAAA CATAACGAAA TTAATTCATC AAACGAATGA 100
<210> 2798
<211> 100
<212> DNA
<213> Escherichia coli K-12 MG1655
<220> envr b3265
<222> 351-450
<223> envr b3265 U00096 complement(3410440..3411102)
<313> 2798: 401-420
<400> 2798
TGAATTTAAT GATGAGATGC TGGCCGAGGG AGTGATACGC GAAAAGATGG 50
GCTTTAATCC GCAGACTCTC CGCGAAGTAT TGCAGGCGTG TCAGCAACAA 100
<210> 2799
<211> 100
<212> DNA
<213> Escherichia coli K-12 MG1655
<220> envr b3265
<222> 339-438
<223> envr b3265 U00096 complement(3410440..3411102)
<313> 2799: 364-383
<400> 2799
ATATCACAAA TGTGAATTTA ATGATGAGAT GCTGGCCGAG GGAGTGATAC 50
GCGAAAAGAT GGGCTTTAAT CCGCAGACTC TCCGCGAAGT ATTGCAGGCG 100
<210> 2800
<211> 100
<212> DNA
<213> Escherichia coli K-12 MG1655
<220> yidw b3696
<222> 251-350
<223> yidw b3696 U00096 complement(3872401..3872787)
<313> 2800: 301-320
<400> 2800
GGGTGCTGGA AAATGACTAC GACCCACGGC TTATCAGTGC CATGAGCGAA 50
GTGCGAAATC TGGTGGAACC GGCGATTGCC CGTTGGGAGC AGAGCGCGCG 100
<210> 2801
<211> 100
<212> DNA
<213> Escherichia coli K-12 MG1655
<220> yidw b3696
<222> 218-317
<223> yidw b3696 U00096 complement(3872401..3872787)
<313> 2801: 255-274
<400> 2801
AGTGGAATTA CCTCGACACT GACGTACTGC AATGGGTGCT GGAAAATGAC 50

TACGACCCAC GGCTTATCAG TGCCATGAGC GAAGTGCGAA ATCTGGTGGA 100
<210> 2802
<211> 100
<212> DNA
<213> Escherichia coli K-12 MG1655
<220> yidw b3696
<222> 165-264
<223> yidw b3696 U00096 complement(3872401..3872787)
<313> 2802: 223-242
<400> 2802
GCGGCTGATT GAAATGAAAC GTTATCGCGG GGCGTTTGTG GCACCGCGTA 50
ACCAGTGGAA TTACCTCGAC ACTGACGTAC TGCAATGGGT GCTGGAAAAT 100
<210> 2803
<211> 100
<212> DNA
<213> Escherichia coli K-12 MG1655
<220> yidx b3697
<222> 573-672
<223> yidx b3697 U00096 3873122..3873793
<313> 2803: 653-672
<400> 2803
TTCTGGTTGT ATATGCAGTA AACCAATAAT CAGTAAGCGG GCAAACGCGT 50
TTATGCTGTT TGCCCGCCCA CAGATTAATT CAGCACATAC TTCTCAATAG 100
<210> 2804
<211> 100
<212> DNA
<213> Escherichia coli K-12 MG1655
<220> yidx b3697
<222> 502-601
<223> yidx b3697 U00096 3873122..3873793
<313> 2804: 565-584
<400> 2804
CCGGGATGGT GTTTCCGCAG CATTACTGCC AGTGACTGTT ACGCTGGACA 50
TCATTACTGC ACCGCTGCAA TTTCTGGTTG TATATGCAGT AAACCAATAA 100
<210> 2805
<211> 100
<212> DNA
<213> Escherichia coli K-12 MG1655
<220> yidx b3697
<222> 492-591
<223> yidx b3697 U00096 3873122..3873793
<313> 2805: 508-527
<400> 2805
CAGAGGCATT CCGGGATGGT GTTTCCGCAG CATTACTGCC AGTGACTGTT 50
ACGCTGGACA TCATTACTGC ACCGCTGCAA TTTCTGGTTG TATATGCAGT 100
<210> 2806
<211> 100
<212> DNA
<213> Escherichia coli K-12 MG1655
<220> yidy b3711
<222> 1077-1176
<223> yidy b3711 U00096 3889244..3890419
<313> 2806: 1147-1166
<400> 2806
GATTCTGATT GCCTGTAGCA TAGTGAGCCT GTTGCTGATT ATGTTCGTCG 50
CGCCTGGACG CCCCGTTGCC GCTCATGAAG AAATCCATCA CCACGCTTGA 100
<210> 2807
<211> 100
<212> DNA
<213> Escherichia coli K-12 MG1655
<220> yidy b3711
<222> 1049-1148
<223> yidy b3711 U00096 3889244..3890419
<313> 2807: 1104-1123
<400> 2807
GTATCGGCGC ATGGAATATG CTGATCGGGA TTCTGATTGC CTGTAGCATA 50
GTGAGCCTGT TGCTGATTAT GTTCGTCGCG CCTGGACGCC CCGTTGCCGC 100
<210> 2808
<211> 100
<212> DNA
<213> Escherichia coli K-12 MG1655

<220> yidy b3711
<222> 910-1009
<223> yidy b3711 U00096 3889244..3890419
<313> 2808: 947-966
<400> 2808
GGTTTCTCGG TAGGTTTTGG TGTGGCGATG AGTCAGGCGT TAGGGCCGTT  50
TTCATTACGC GCGGGCGTAG CCAGCTCGAC CTTAGGTATT GCGCAGGTTT  100
<210> 2809
<211> 100
<212> DNA
<213> Escherichia coli K-12 MG1655
<220> yidz b3712
<222> 861-960
<223> yidz b3712 U00096 3890394..3891353
<313> 2809: 904-923
<400> 2809
AGTTCCTTTT ACCCTGCTGT GGCATAAACG GAACAGCCAT AATCCGAAGA  50
TCGTCTGGTT ACGGGAAACC ATTAAAAACC TTTACGCGTC GATGGCATAA  100
<210> 2810
<211> 100
<212> DNA
<213> Escherichia coli K-12 MG1655
<220> yidz b3712
<222> 807-906
<223> yidz b3712 U00096 3890394..3891353
<313> 2810: 862-881
<400> 2810
GCCGTTGGTT GCTCTTCCTC TCCCGTTTGA CGAAAGCCAG CAAAAAAAGC  50
TGGAAGTTCC TTTTACCCTG CTGTGGCATA AACGGAACAG CCATAATCCG  100
<210> 2811
<211> 100
<212> DNA
<213> Escherichia coli K-12 MG1655
<220> yidz b3712
<222> 785-884
<223> yidz b3712 U00096 3890394..3891353
<313> 2811: 809-828
<400> 2811
ACTACAATCA ACTCCATCAA CTGCCGTTGG TTGCTCTTCC TCTCCCGTTT  50
GACGAAAGCC AGCAAAAAAA GCTGGAAGTT CCTTTTACCC TGCTGTGGCA  100
<210> 2812
<211> 100
<212> DNA
<213> Escherichia coli K-12 MG1655
<220> envy b0567
<222> 663-762
<223> envy b0567 U00096 complement(585370..586131)
<313> 2812: 720-739
<400> 2812
CTATAGCAGC ACGTCGTACT TTATCTCTGT TTTTAAGGCG TTTTACGGCC  50
TGACACCGTT GAATTATCTC GCCAAACAGC GACAAAAAGT GATGTGGTGA  100
<210> 2813
<211> 100
<212> DNA
<213> Escherichia coli K-12 MG1655
<220> envy b0567
<222> 420-519
<223> envy b0567 U00096 complement(585370..586131)
<313> 2813: 475-494
<400> 2813
GTATATCTTA CGCAGCAGCG TCCGCGACAG CGTCTGCCGC ATTATTCAAA  50
GCGATATTCA GCATTACTGG AATCTGCGAA TTGTCGCCAG TTCGCTATGT  100
<210> 2814
<211> 100
<212> DNA
<213> Escherichia coli K-12 MG1655
<220> envy b0567
<222> 399-498
<223> envy b0567 U00096 complement(585370..586131)
<313> 2814: 443-462
<400> 2814

GCGGTTTATT GCCCTACTGA TGTATATCTT ACGCAGCAGC GTCCGCGACA 50
GCGTCTGCCG CATTATTCAA AGCGATATTC AGCATTACTG GAATCTGCGA 100
<210> 2815
<211> 100
<212> DNA
<213> Escherichia coli K-12 MG1655
<220> evga b2370
<222> 378-477
<223> evga b2370 U00096 2481775..2482389
<313> 2815: 424-443
<400> 2815
TCTCAACCGG TTTGTTGGAA GTTTAACGTC CGACCAGCAA AAACTCGACT 50
CCTTATCGAA ACAAGAAATT AGTGTCATGC GGTATATTCT TGATGGCAAG 100
<210> 2816
<211> 100
<212> DNA
<213> Escherichia coli K-12 MG1655
<220> evga b2370
<222> 263-362
<223> evga b2370 U00096 2481775..2482389
<313> 2816: 295-314
<400> 2816
GGAAACATTG TGCTGATGCT GGCGCTAATG GTTTCGTGAG TAAAAAAGAA 50
GGCATGAACA ATATCATTGC GGCTATTGAA GCTGCAAAAA ATGGCTACTG 100
<210> 2817
<211> 100
<212> DNA
<213> Escherichia coli K-12 MG1655
<220> evga b2370
<222> 150-249
<223> evga b2370 U00096 2481775..2482389
<313> 2817: 195-214
<400> 2817
CATTGATGTC GATATCCCCG GAGTTAACGG TATCCAGGTG TTAGAAACGC 50
TGAGGAAGCG CCAATATAGC GGAATTATTA TTATCGTCTC CGCTAAAAAT 100
<210> 2818
<211> 100
<212> DNA
<213> Escherichia coli K-12 MG1655
<220> envz b3405
<222> 1254-1353
<223> envz b3405 U00096 complement(3532154..3533506)
<313> 2818: 1315-1334
<400> 2818
GATGCTGGAG CTTGGCACCA GCGAGCGGGG CGGGCTTTCC ATTCGCGCCT 50
GGCTGCCAGT GCCGGTAACG CGGGCGCAGG GCACGACAAA AGAAGGGTAA 100
<210> 2819
<211> 100
<212> DNA
<213> Escherichia coli K-12 MG1655
<220> envz b3405
<222> 1239-1338
<223> envz b3405 U00096 complement(3532154..3533506)
<313> 2819: 1272-1291
<400> 2819
GGATAACCAT AACGGGATGC TGGAGCTTGG CACCAGCGAG CGGGGCGGGC 50
TTTCCATTCG CGCCTGGCTG CCAGTGCCGG TAACGCGGGC GCAGGGCACG 100
<210> 2820
<211> 100
<212> DNA
<213> Escherichia coli K-12 MG1655
<220> envz b3405
<222> 1192-1291
<223> envz b3405 U00096 complement(3532154..3533506)
<313> 2820: 1233-1252
<400> 2820
ACCATTAGCG GCACGGGATT AGGGCTGGCA ATTGTGCAGC GTATCGTGGA 50
TAACCATAAC GGGATGCTGG AGCTTGGCAC CAGCGAGCGG GGCGGGCTTT 100
<210> 2821
<211> 100
<212> DNA

<213> Escherichia coli K-12 MG1655
<220> gpma b0756
<222> 621-720
<223> gpma b0756 U00096 complement(786066..786818)
<313> 2821: 664-683
<400> 2821
TATCCCGACT GGCGTGCCGC TGGTGTATGA GTTCGACGAG AATTTCAAAC 50
CGCTGAAACG CTATTATCTG GGTAATGCTG ACGAGATCGC AGCGAAAGCA 100
<210> 2822
<211> 100
<212> DNA
<213> Escherichia coli K-12 MG1655
<220> gpma b0756
<222> 461-560
<223> gpma b0756 U00096 complement(786066..786818)
<313> 2822: 514-533
<400> 2822
TGGCGCTGAC CATTGACCGC GTGATCCCTT ACTGGAATGA AACTATTCTG 50
CCGCGTATGA AGAGCGGTGA GCGCGTGATC ATCGCTGCAC ACGGTAACTC 100
<210> 2823
<211> 100
<212> DNA
<213> Escherichia coli K-12 MG1655
<220> gpma b0756
<222> 405-504
<223> gpma b0756 U00096 complement(786066..786818)
<313> 2823: 426-445
<400> 2823
TCACGATCCG CGTTACGCGA AACTGAGCGA GAAAGAACTG CCGCTGACGG 50
AAAGCCTGGC GCTGACCATT GACCGCGTGA TCCCTTACTG GAATGAAACT 100
<210> 2824
<211> 100
<212> DNA
<213> Escherichia coli K-12 MG1655
<220> hmpa b2553
<222> 1092-1191
<223> hmpa b2553 U00096 2683855..2685045
<313> 2824: 1171-1190
<400> 2824
GGTTGGCTTC ATGCAGTTTA CCGCGAAACA GTTAGTGGAT CTGGGCGTGA 50
AGCAGGAAAA CATTCATTAC GAATGCTTTG GCCCGCATAA GGTGCTGTAA 100
<210> 2825
<211> 100
<212> DNA
<213> Escherichia coli K-12 MG1655
<220> hmpa b2553
<222> 1073-1172
<223> hmpa b2553 U00096 2683855..2685045
<313> 2825: 1125-1144
<400> 2825
AGTTCTATCT CTGCGGCCCG GTTGGCTTCA TGCAGTTTAC CGCGAAACAG 50
TTAGTGGATC TGGGCGTGAA GCAGGAAAAC ATTCATTACG AATGCTTTGG 100
<210> 2826
<211> 100
<212> DNA
<213> Escherichia coli K-12 MG1655
<220> hmpa b2553
<222> 1023-1122
<223> hmpa b2553 U00096 2683855..2685045
<313> 2826: 1073-1092
<400> 2826
TCTGATGGAT TTGAGCAAAC TGGAAGGTGC GTTCAGCGAT CCGACAATGC 50
AGTTCTATCT CTGCGGCCCG GTTGGCTTCA TGCAGTTTAC CGCGAAACAG 100
<210> 2827
<211> 100
<212> DNA
<213> Escherichia coli K-12 MG1655
<220> gpmb b4396
<222> 549-648
<223> gpmb b4396 U00096 4631366..4632013
<313> 2827: 602-621

<400> 2827
TTATCAGGAA AGCCTGTGGC TGGCGTCTGG CTGGGTCGTT GAAACCGCAG  50
GGGACATCTC GCATCTGGAC GCCCCTGCAT TAGATGAGCT GCAGCGTTAA  100
<210> 2828
<211> 100
<212> DNA
<213> Escherichia coli K-12 MG1655
<220> gpmb b4396
<222> 454-553
<223> gpmb b4396 U00096 4631366..4632013
<313> 2828: 518-537
<400> 2828
ATTGCACTGG GATGCCTGGT GAGTACGATT CTCGGATTAC CAGCATGGGC  50
AGAGCGCCGC TTACGTCTGC GTAACTGTTC TATTTCGCGC GTGGATTATC  100
<210> 2829
<211> 100
<212> DNA
<213> Escherichia coli K-12 MG1655
<220> gpmb b4396
<222> 418-517
<223> gpmb b4396 U00096 4631366..4632013
<313> 2829: 433-452
<400> 2829
CCGCAGGGAA GCCGACCGCT GCTGGTGAGT CATGGTATTG CACTGGGATG  50
CCTGGTGAGT ACGATTCTCG GATTACCAGC ATGGGCAGAG CGCCGCTTAC  100
<210> 2830
<211> 100
<212> DNA
<213> Escherichia coli K-12 MG1655
<220> yiec b3721
<222> 1518-1617
<223> yiec b3721 U00096 complement(3898232..3899848)
<313> 2830: 1562-1581
<400> 2830
GTATATCAAA GCCCTGGAAA ACGAACTGGA TGGCTTCACC TTTGAAGACA  50
ATAAAGACGA CCAGTTTGCT GTCGGTGCCC AGGCTGAAAT CTGGTGGTAA  100
<210> 2831
<211> 100
<212> DNA
<213> Escherichia coli K-12 MG1655
<220> yiec b3721
<222> 1482-1581
<223> yiec b3721 U00096 complement(3898232..3899848)
<313> 2831: 1524-1543
<400> 2831
GTTGACCTCG CGTCCGGAAA TTCGTTTCTA CGCCACGTAT ATCAAAGCCC  50
TGGAAAACGA ACTGGATGGC TTCACCTTTG AAGACAATAA AGACGACCAG  100
<210> 2832
<211> 100
<212> DNA
<213> Escherichia coli K-12 MG1655
<220> yiec b3721
<222> 1350-1449
<223> yiec b3721 U00096 complement(3898232..3899848)
<313> 2832: 1382-1401
<400> 2832
GGACCAATAT AACCAGACAG GTGTTGAACT GGGCTATTTC ACCCAGCAAA  50
ACAAAGATGC GAATAGTAAT AAATTTAATG AGTCTGGTTA TAAAACCACG  100
<210> 2833
<211> 100
<212> DNA
<213> Escherichia coli K-12 MG1655
<220> ogt b1336
<222> 417-516
<223> ogt b1336 U00096 complement(1397745..1398260)
<313> 2833: 487-506
<400> 2833
CCATCGGGTT ATTGGCCGAA ACGGCACCAT GACCGGATAT GCAGGCGGAG  50
TTCAGCGAAA AGAGTGGTTA TTGCGCCATG AAGGTTATCT TTTGCTGTAA  100
<210> 2834
<211> 100

<212> DNA
<213> Escherichia coli K-12 MG1655
<220> ogt b1336
<222> 351-450
<223> ogt b1336 U00096 complement(1397745..1398260)
<313> 2834: 405-424
<400> 2834
TCCTGGCGCG GCGCGTGCCG TTGGTGCGGC AAACGGATCG AATCCCATCA 50
GCATCGTCGT ACCTTGCCAT CGGGTTATTG GCCGAAACGG CACCATGACC 100
<210> 2835
<211> 100
<212> DNA
<213> Escherichia coli K-12 MG1655
<220> ogt b1336
<222> 232-331
<223> ogt b1336 U00096 complement(1397745..1398260)
<313> 2835: 280-299
<400> 2835
GATACGCTTC CCACTGCTAC GGGGGGGACG CCATTTCAGC GCGAAGTCTG 50
GAAAACACTA CGCACTATCC CCTGCGGGCA GGTAATGCAT TACGGCCAAC 100
<210> 2836
<211> 100
<212> DNA
<213> Escherichia coli K-12 MG1655
<220> yiee b3713
<222> 663-762
<223> yiee b3713 U00096 3891498..3892259
<313> 2836: 722-741
<400> 2836
GAAAAGCCTG CCGGATATTC ACAGCCGCGC CAACAATCCT ACCAGCCGGA 50
TGATGCGTTT TGCCCAACTC TCTACCGTGA AGGCTTTTTC GCCAAATTGA 100
<210> 2837
<211> 100
<212> DNA
<213> Escherichia coli K-12 MG1655
<220> yiee b3713
<222> 596-695
<223> yiee b3713 U00096 3891498..3892259
<313> 2837: 647-666
<400> 2837
GGTCCGTGGC GGTCACGCCC ACGATTGAAA AGCTCAGTGT CTGGGAGTTA 50
GATGGCAAAC ACGGCTGGAA AAGCCTGCCG GATATTCACA GCCGCGCCAA 100
<210> 2838
<211> 100
<212> DNA
<213> Escherichia coli K-12 MG1655
<220> yiee b3713
<222> 540-639
<223> yiee b3713 U00096 3891498..3892259
<313> 2838: 573-592
<400> 2838
ATGTGCTCAT GTAAATCATG TAGAAGCGTT ATGCGACGCG GAAGACGTGC 50
TGGTGTGGTC CGTGGCGGTC ACGCCCACGA TTGAAAAGCT CAGTGTCTGG 100
<210> 2839
<211> 100
<212> DNA
<213> Escherichia coli K-12 MG1655
<220> yief b3714
<222> 468-567
<223> yief b3714 U00096 3892281..3892847
<313> 2839: 514-533
<400> 2839
TGATCCGCAA ACCGGAGAAG TGATTGATCA GGGTACGCTG GACCACCTGA 50
CCGGGCAATT GACCGCATTT GGTGAGTTTA TTCAGCGAGT TAAGATCTAA 100
<210> 2840
<211> 100
<212> DNA
<213> Escherichia coli K-12 MG1655
<220> yief b3714
<222> 405-504
<223> yief b3714 U00096 3892281..3892847

<313> 2840: 466-485
<400> 2840
TTTCCTCGAT GCAATGGTGA TGAACAAGCC GGAATTTATG GGCGGCGTGA 50
TTCAGAACAA AGTTGATCCG CAAACCGGAG AAGTGATTGA TCAGGGTACG 100
<210> 2841
<211> 100
<212> DNA
<213> Escherichia coli K-12 MG1655
<220> yief b3714
<222> 373-472
<223> yief b3714 U00096 3892281..3892847
<313> 2841: 424-443
<400> 2841
CGCTGTCAGT ATCACCTGCG CCAGATTCTG GTTTTCCTCG ATGCAATGGT 50
GATGAACAAG CCGGAATTTA TGGGCGGCGT GATTCAGAAC AAAGTTGATC 100
<210> 2842
<211> 100
<212> DNA
<213> Escherichia coli K-12 MG1655
<220> yieg b3715
<222> 1239-1338
<223> yieg b3715 U00096 complement(3892901..3894238)
<313> 2842: 1309-1328
<400> 2842
GATGAAAATC GGTACCGGAC GTCTGCGTGA TCTAAGCCCG TGCGTAATCA 50
TCGTTGCGCT GCTGTTTATC CTGAAGATTG TCTTTATCGA CGCTCACTAA 100
<210> 2843
<211> 100
<212> DNA
<213> Escherichia coli K-12 MG1655
<220> yieg b3715
<222> 1177-1276
<223> yieg b3715 U00096 complement(3892901..3894238)
<313> 2843: 1205-1224
<400> 2843
ATGATGCCGT TCAGCTTCTC GATTACCGAA GGTATCGCGC TGGGCTTTAT 50
CTCCTACTGC GTGATGAAAA TCGGTACCGG ACGTCTGCGT GATCTAAGCC 100
<210> 2844
<211> 100
<212> DNA
<213> Escherichia coli K-12 MG1655
<220> yieg b3715
<222> 978-1077
<223> yieg b3715 U00096 complement(3892901..3894238)
<313> 2844: 1018-1037
<400> 2844
CGGTCGTACC GGTCTGACGG CAGTGGTTGT TGGTCTGCTG TTCCTGCTGG 50
TTATCTTTCT GTCGCCGCTG GCGGGGATGG TGCCAGGCTA CGCTGCAGCT 100
<210> 2845
<211> 100
<212> DNA
<213> Escherichia coli K-12 MG1655
<220> yieh b3716
<222> 567-666
<223> yieh b3716 U00096 3894403..3895068
<313> 2845: 642-661
<400> 2845
GCACAATAAG CCGATCGTTC ACCCGAAAGT CACCACCTTT ACCCATCTTT 50
CGCAGTTACC TGAACTGTGG AAAGCGCGTG GTTGGGATAT TACGGCATAG 100
<210> 2846
<211> 100
<212> DNA
<213> Escherichia coli K-12 MG1655
<220> yieh b3716
<222> 517-616
<223> yieh b3716 U00096 3894403..3895068
<313> 2846: 560-579
<400> 2846
CAATCTGGTA TCGACGCAGG TATGGAAGTG TTCTACTTCT GCGCCGACCC 50
GCACAATAAG CCGATCGTTC ACCCGAAAGT CACCACCTTT ACCCATCTTT 100
<210> 2847

<211> 100
<212> DNA
<213> Escherichia coli K-12 MG1655
<220> yieh b3716
<222> 344-443
<223> yieh b3716 U00096 3894403..3895068
<313> 2847: 384-403
<400> 2847
TGCAGCATTC TATGGGCAAG CTGAATATGT TGCACTACTT CCCGGATAAA 50
CTGTTCAGCG GCTACGATAT TCAGCGCTGG AAGCCAGACC CGGCGTTAAT 100
<210> 2848
<211> 100
<212> DNA
<213> Escherichia coli K-12 MG1655
<220> yiei b3717
<222> 369-468
<223> yiei b3717 U00096 3895135..3895602
<313> 2848: 434-453
<400> 2848
CATCATTCCT GCATTGCTGG CAGGTGTGGT GATGAGCAGA ATCATTACCC 50
GTCTACCCGG ATTGGATATT TCATGTCCTG AAACAGACTC TTTAAGTTAA 100
<210> 2849
<211> 100
<212> DNA
<213> Escherichia coli K-12 MG1655
<220> yiei b3717
<222> 306-405
<223> yiei b3717 U00096 3895135..3895602
<313> 2849: 364-383
<400> 2849
TAAGGGCATG GCTTCCTCGG AGTTGTTTGA AAACATTCTT TCTGGTGACA 50
GTCTCGTTGT CCGCATCATT CCTGCATTGC TGGCAGGTGT GGTGATGAGC 100
<210> 2850
<211> 100
<212> DNA
<213> Escherichia coli K-12 MG1655
<220> yiei b3717
<222> 287-386
<223> yiei b3717 U00096 3895135..3895602
<313> 2850: 323-342
<400> 2850
GTGCAGTTAT TGTACATATT AAGGGCATGG CTTCCTCGGA GTTGTTTGAA 50
AACATTCTTT CTGGTGACAG TCTCGTTGTC CGCATCATTC CTGCATTGCT 100
<210> 2851
<211> 100
<212> DNA
<213> Escherichia coli K-12 MG1655
<220> yiej b3718
<222> 489-588
<223> yiej b3718 U00096 3895651..3896238
<313> 2851: 550-569
<400> 2851
TGATCTAGCT AAATGCCTGC AAAAGGGTGG TCATTGTCAG GGTTATCTCT 50
TCCGCTGTCT CCACTGCGGC AAGCTGAGAC TGTGGGGTGA TTTTTCGTAG 100
<210> 2852
<211> 100
<212> DNA
<213> Escherichia coli K-12 MG1655
<220> yiej b3718
<222> 395-494
<223> yiej b3718 U00096 3895651..3896238
<313> 2852: 432-451
<400> 2852
TTATCGGCTA TGTGGGCTGG AATGATATAA AAGATCGCCT CGATGAATTT 50
GCCAACCTTG AAGAAGATTG TGAGAATTTC GGTATTAGAA ATTCTGATCT 100
<210> 2853
<211> 100
<212> DNA
<213> Escherichia coli K-12 MG1655
<220> yiej b3718
<222> 355-454

<223> yiej b3718 U00096 3895651..3896238
<313> 2853: 386-405
<400> 2853
CAGGAATTCT GGCTCGCGCA TTGTGGCGAT TTCTGTGTTT TTATCGGCTA 50
TGTGGGCTGG AATGATATAA AAGATCGCCT CGATGAATTT GCCAACCTTG 100
<210> 2854
<211> 100
<212> DNA
<213> Escherichia coli K-12 MG1655
<220> rnpa b3705
<222> 249-348
<223> rnpa b3705 U00096 3882122..3882481
<313> 2854: 303-322
<400> 2854
GGATTTCGTG GTGGTGGCGA AAAAAGGGGT TGCCGACCTC GATAACCGTG 50
CTCTCTCGGA AGCGTTGGAA AAATTATGGC GCCGCCACTG TCGCCTGGCT 100
<210> 2855
<211> 100
<212> DNA
<213> Escherichia coli K-12 MG1655
<220> rnpa b3705
<222> 218-317
<223> rnpa b3705 U00096 3882122..3882481
<313> 2855: 246-265
<400> 2855
TCCGTCTGCG CCAACATGAA CTCCCGGCTA TGGATTTCGT GGTGGTGGCG 50
AAAAAAGGGG TTGCCGACCT CGATAACCGT GCTCTCTCGG AAGCGTTGGA 100
<210> 2856
<211> 100
<212> DNA
<213> Escherichia coli K-12 MG1655
<220> rnpa b3705
<222> 145-244
<223> rnpa b3705 U00096 3882122..3882481
<313> 2856: 172-191
<400> 2856
CTTACAGTCG CCAAGAAAAA CGTTCGACGC GCCCATGAAC GCAATCGGAT 50
TAAACGTCTG ACGCGTGAAA GCTTCCGTCT GCGCCAACAT GAACTCCCGG 100
<210> 2857
<211> 100
<212> DNA
<213> Escherichia coli K-12 MG1655
<220> yiek b3719
<222> 543-642
<223> yiek b3719 U00096 complement(3896311..3896952)
<313> 2857: 622-641
<400> 2857
TGTTCTGCAA GGTCCGGTTA CCGAAGATGT TCCAGCTTCC GTGTTGCAGC 50
TGCATCCTTC GCTGATGGTA ATCGCTGATA AAGCCGTGCC GCAGAATTAG 100
<210> 2858
<211> 100
<212> DNA
<213> Escherichia coli K-12 MG1655
<220> yiek b3719
<222> 504-603
<223> yiek b3719 U00096 complement(3896311..3896952)
<313> 2858: 569-588
<400> 2858
AATTGTCAGC GGCGCGGGTA AAGCGCAGGC ACTGAAAAAT GTTCTGCAAG 50
GTCCGGTTAC CGAAGATGTT CCAGCTTCCG TGTTGCAGCT GCATCCTTCG 100
<210> 2859
<211> 100
<212> DNA
<213> Escherichia coli K-12 MG1655
<220> yiek b3719
<222> 465-564
<223> yiek b3719 U00096 complement(3896311..3896952)
<313> 2859: 529-548
<400> 2859
GGGGCCGAAA AGCATCATGG CGGCGAAAAA CTTGCTGATA ATTGTCAGCG 50
GCGCGGGTAA AGCGCAGGCA CTGAAAAATG TTCTGCAAGG TCCGGTTACC 100

<210> 2860
<211> 100
<212> DNA
<213> Escherichia coli K-12 MG1655
<220> yiel b3720
<222> 1024-1123
<223> yiel b3720 U00096 complement(3897036..3898238)
<313> 2860: 1053-1072
<400> 2860
AATTTTACTG TGGTTGTGGG GGATAAAGAT GTCGTAACCG GCAAGGATAT 50
CGCCGGGCTG AAAACTGAGC TTGAGCAGAA AAAAATTAAC TTTGATTACC 100
<210> 2861
<211> 100
<212> DNA
<213> Escherichia coli K-12 MG1655
<220> yiel b3720
<222> 977-1076
<223> yiel b3720 U00096 complement(3897036..3898238)
<313> 2861: 1009-1028
<400> 2861
TCGCGGCCCG ACTGAACGAT CCGGCAGCTA TCAACCAGCA ACTACGTAAT 50
TTTACTGTGG TTGTGGGGGA TAAAGATGTC GTAACCGGCA AGGATATCGC 100
<210> 2862
<211> 100
<212> DNA
<213> Escherichia coli K-12 MG1655
<220> yiel b3720
<222> 702-801
<223> yiel b3720 U00096 complement(3897036..3898238)
<313> 2862: 745-764
<400> 2862
GAAGGGCATT ATTCCCGAAG ATTTCGTGCC TCAGGAAAGA CGTAAAGTCT 50
TTTATCCGCT GAATGCTAAA GCGGCAGATC GCGAACTGAT GAACGATATT 100
<210> 2863
<211> 100
<212> DNA
<213> Escherichia coli K-12 MG1655
<220> yiem b3746
<222> 1185-1284
<223> yiem b3746 U00096 complement(3925780..3927063)
<313> 2863: 1241-1260
<400> 2863
GTCGGCACAC GGCAAACCCG GCATCATGCG CATTTTCGAT CATATCTGGC 50
GCTTTGATAC CGGGATGCGA AGCCGCCTGC TCAGACGCTG GCGGCGATAA 100
<210> 2864
<211> 100
<212> DNA
<213> Escherichia coli K-12 MG1655
<220> yiem b3746
<222> 1083-1182
<223> yiem b3746 U00096 complement(3925780..3927063)
<313> 2864: 1123-1142
<400> 2864
GGTGATTTCT GATTTTATCG CTCAGCGGTT GCCTGACGAC GTGACGAGTA 50
AAGTGAAAGA GCTGCAGCGG GTACATCAGC ATCGCTTTCA TGCCGTGGCG 100
<210> 2865
<211> 100
<212> DNA
<213> Escherichia coli K-12 MG1655
<220> yiem b3746
<222> 1023-1122
<223> yiem b3746 U00096 complement(3925780..3927063)
<313> 2865: 1082-1101
<400> 2865
TTGTTTTCGC GCCATTATGG AACGCTTGCA AAGCAGGGAA TGGTTTGATG 50
CCGATGCGGT GGTGATTTCT GATTTTATCG CTCAGCGGTT GCCTGACGAC 100
<210> 2866
<211> 100
<212> DNA
<213> Escherichia coli K-12 MG1655
<220> yien b3747

<222> 1422-1521
<223> yien b3747 U00096 complement(3927224..3928744)
<313> 2866: 1466-1485
<400> 2866
ACAGCAAAAA TGTCTGTTTA TCCCTGGCGA CTGGTTAGGT CGCATTGAAG 50
CCAGCCTACA GGATGTCGGT GCACAGATTC GCCAGGCACA ACAATGCTAA 100
<210> 2867
<211> 100
<212> DNA
<213> Escherichia coli K-12 MG1655
<220> yien b3747
<222> 1378-1477
<223> yien b3747 U00096 complement(3927224..3928744)
<313> 2867: 1410-1429
<400> 2867
GAGCTTGAAA GCGACTGGCG CAAGCAACAC GCTTTATTCA GCGAACAGCA 50
AAAATGTCTG TTTATCCCTG GCGACTGGTT AGGTCGCATT GAAGCCAGCC 100
<210> 2868
<211> 100
<212> DNA
<213> Escherichia coli K-12 MG1655
<220> yien b3747
<222> 1339-1438
<223> yien b3747 U00096 complement(3927224..3928744)
<313> 2868: 1377-1396
<400> 2868
GCTGAAGGTC TGCCTGGTGA AATAAAACAA CAACTGGAAG AGCTTGAAAG 50
CGACTGGCGC AAGCAACACG CTTTATTCAG CGAACAGCAA AAATGTCTGT 100
<210> 2869
<211> 100
<212> DNA
<213> Escherichia coli K-12 MG1655
<220> evgs b2371
<222> 3495-3594
<223> evgs b2371 U00096 2482394..2485987
<313> 2869: 3552-3571
<400> 2869
TGACAGTAAG CCTGAAATTC TTCAGTTGCT GAACTCTGTA AAAGAACACA 50
TTGCAGAGCT GGACCAGGAG ATTGCTGTTT TCTGTCAGAA AAATGACTAA 100
<210> 2870
<211> 100
<212> DNA
<213> Escherichia coli K-12 MG1655
<220> evgs b2371
<222> 3355-3454
<223> evgs b2371 U00096 2482394..2485987
<313> 2870: 3386-3405
<400> 2870
GCTGCGTTTC AAGCACTAGA AGCTGGCGAT AACAGAACTT TCCATCAGTG 50
TATTCATCGC ATCCACGGTG CGGCTAACAT CCTGAATTTG CAAAAGTTGA 100
<210> 2871
<211> 100
<212> DNA
<213> Escherichia coli K-12 MG1655
<220> evgs b2371
<222> 3303-3402
<223> evgs b2371 U00096 2482394..2485987
<313> 2871: 3339-3358
<400> 2871
GATGCAGGAG ATTCTCATGA CTTTCCAGCA TGAAACGCAT AAAGATCTAC 50
CCGCTGCGTT TCAAGCACTA GAAGCTGGCG ATAACAGAAC TTTCCATCAG 100
<210> 2872
<211> 100
<212> DNA
<213> Escherichia coli K-12 MG1655
<220> yieo b3755
<222> 1329-1428
<223> yieo b3755 U00096 complement(3936811..3938238)
<313> 2872: 1402-1421
<400> 2872
AGCAGCAATG TTCATGCTTC TGAAAACAAC CGATGGTAAT AATTTGATCA 50

AAAGACAGCG TAAATCTAAG CCGAACCGCG TTCCATCAGA ATCGGAGTAA 100
<210> 2873
<211> 100
<212> DNA
<213> Escherichia coli K-12 MG1655
<220> yieo b3755
<222> 1294-1393
<223> yieo b3755 U00096 complement(3936811..3938238)
<313> 2873: 1335-1354
<400> 2873
ACGTTTATCA CAATGGGCAT TATTACTGTT GCTTCAGCAG CAATGTTCAT 50
GCTTCTGAAA ACAACCGATG GTAATAATTT GATCAAAAGA CAGCGTAAAT 100
<210> 2874
<211> 100
<212> DNA
<213> Escherichia coli K-12 MG1655
<220> yieo b3755
<222> 1237-1336
<223> yieo b3755 U00096 complement(3936811..3938238)
<313> 2874: 1264-1283
<400> 2874
GTCCTTCGCG TTTATGAAGG AATGGAAGGC ACAACGACTG TCGAACAATT 50
CCACTATACG TTTATCACAA TGGGCATTAT TACTGTTGCT TCAGCAGCAA 100
<210> 2875
<211> 100
<212> DNA
<213> Escherichia coli K-12 MG1655
<220> yiep b3756
<222> 447-546
<223> yiep b3756 U00096 complement(3938261..3938806)
<313> 2875: 510-529
<400> 2875
GCTGGATCTG CATCAGGCGA TTGTCGATGC GATTATCCAA AGCGATGGCG 50
ACGCGGCATT TAAAGCTTGT CAGGCGCTGC TACGCTCACC TGATAAGTGA 100
<210> 2876
<211> 100
<212> DNA
<213> Escherichia coli K-12 MG1655
<220> yiep b3756
<222> 401-500
<223> yiep b3756 U00096 complement(3938261..3938806)
<313> 2876: 431-450
<400> 2876
TTTATCACAC TTACTTCACG TCAATTACCA GCGACACAGT GATAAAGCTG 50
GATCTGCATC AGGCGATTGT CGATGCGATT ATCCAAAGCG ATGGCGACGC 100
<210> 2877
<211> 100
<212> DNA
<213> Escherichia coli K-12 MG1655
<220> yiep b3756
<222> 332-431
<223> yiep b3756 U00096 complement(3938261..3938806)
<313> 2877: 388-407
<400> 2877
GGCATGAGCA TATCTATGAA ATGAGCGCCA ATCCGTTTTT GACCTCATTC 50
GCCTCGCTAT TCCATTCGGT TTATCACACT TACTTCACGT CAATTACCAG 100
<210> 2878
<211> 100
<212> DNA
<213> Escherichia coli K-12 MG1655
<220> b3694 b3695
<222> 198-297
<223> b3694 b3695 U00096 complement(3872099..3872395)
<313> 2878: 265-284
<400> 2878
GCTGTTCGAT GCGATACGAC ATCAGGACGG CGATGCGGCA GAGCAGGCGG 50
CGCTTACCAT GATCGCCAGC TCGACACGAA GGTTAAAGGA AATCACATGA 100
<210> 2879
<211> 100
<212> DNA
<213> Escherichia coli K-12 MG1655

<220> b3694 b3695
<222> 198-297
<223> b3694 b3695 U00096 complement(3872099..3872395)
<313> 2879: 234-253
<400> 2879
GCTGTTCGAT GCGATACGAC ATCAGGACGG CGATGCGGCA GAGCAGGCGG 50
CGCTTACCAT GATCGCCAGC TCGACACGAA GGTTAAAGGA AATCACATGA 100
<210> 2880
<211> 100
<212> DNA
<213> Escherichia coli K-12 MG1655
<220> b3694 b3695
<222> 166-265
<223> b3694 b3695 U00096 complement(3872099..3872395)
<313> 2880: 189-208
<400> 2880
AACATGCCGC AAACGCTCCA GGAACATAAG GCGCTGTTCG ATGCGATACG 50
ACATCAGGAC GGCGATGCGG CAGAGCAGGC GGCGCTTACC ATGATCGCCA 100
<210> 2881
<211> 100
<212> DNA
<213> Escherichia coli K-12 MG1655
<220> gale b0760
<222> 918-1017
<223> gale b0760 U00096 complement(790262..791278)
<313> 2881: 984-1003
<400> 2881
CGACCGTGAA CTGAACTGGC GCGTAACGCG CACACTCGAT GAAATGGCGC 50
AGGACACCTG GCACTGGCAG TCACGCCATC CACAGGGATA TCCCGATTAA 100
<210> 2882
<211> 100
<212> DNA
<213> Escherichia coli K-12 MG1655
<220> gale b0760
<222> 850-949
<223> gale b0760 U00096 complement(790262..791278)
<313> 2882: 912-931
<400> 2882
GTTAATTATC ATTTTGCACC GCGTCGCGAG GGCGACCTTC CGGCCTACTG 50
GGCGGACGCC AGCAAAGCCG ACCGTGAACT GAACTGGCGC GTAACGCGCA 100
<210> 2883
<211> 100
<212> DNA
<213> Escherichia coli K-12 MG1655
<220> gale b0760
<222> 779-878
<223> gale b0760 U00096 complement(790262..791278)
<313> 2883: 819-838
<400> 2883
ACCTCGGCGC TGGCGTAGGC AACAGCGTGC TGGACGTGGT TAATGCCTTC 50
AGCAAAGCCT GCGGCAAACC GGTTAATTAT CATTTTGCAC CGCGTCGCGA 100
<210> 2884
<211> 100
<212> DNA
<213> Escherichia coli K-12 MG1655
<220> galf b2043
<222> 795-894
<223> galf b2043 U00096 complement(2111456..2112349)
<313> 2884: 864-883
<400> 2884
AAAAATGGGC TATATGCAGG CGTTTGTGAA GTATGGCCTA CGCAACCTGA 50
AAGAAGGGGC GAAGTTCCGT AAAGGTATTG AGAAGCTGTT AAGCGAATAA 100
<210> 2885
<211> 100
<212> DNA
<213> Escherichia coli K-12 MG1655
<220> galf b2043
<222> 767-866
<223> galf b2043 U00096 complement(2111456..2112349)
<313> 2885: 829-848
<400> 2885

TGACCGGCGA CAGTTACGAC TGCGGCAAAA AAATGGGCTA TATGCAGGCG 50
TTTGTGAAGT ATGGCCTACG CAACCTGAAA GAAGGGGCGA AGTTCCGTAA 100
<210> 2886
<211> 100
<212> DNA
<213> Escherichia coli K-12 MG1655
<220> galf b2043
<222> 733-832
<223> galf b2043 U00096 complement(2111456..2112349)
<313> 2886: 783-802
<400> 2886
CTGGCGAAAA AACAATCCGT TGATGCAATG CTGATGACCG GCGACAGTTA 50
CGACTGCGGC AAAAAAATGG GCTATATGCA GGCGTTTGTG AAGTATGGCC 100
<210> 2887
<211> 100
<212> DNA
<213> Escherichia coli K-12 MG1655
<220> galk b0758
<222> 1026-1125
<223> galk b0758 U00096 complement(788054..789202)
<313> 2887: 1077-1096
<400> 2887
GCTGATCCCG GAAGAGCTGG TGCCTGCCGT ACAGCAAGCT GTCGCTGAAC 50
AATATGAAGC AAAAACAGGT ATTAAAGAGA CTTTTTACGT TTGTAAACCA 100
<210> 2888
<211> 100
<212> DNA
<213> Escherichia coli K-12 MG1655
<220> galk b0758
<222> 969-1068
<223> galk b0758 U00096 complement(788054..789202)
<313> 2888: 1037-1056
<400> 2888
TGGCGACAAA GGTGGCGTAC GCATGACCGG CGGCGGATTT GGCGGCTGTA 50
TCGTCGCGCT GATCCCGGAA GAGCTGGTGC CTGCCGTACA GCAAGCTGTC 100
<210> 2889
<211> 100
<212> DNA
<213> Escherichia coli K-12 MG1655
<220> galk b0758
<222> 904-1003
<223> galk b0758 U00096 complement(788054..789202)
<313> 2889: 942-961
<400> 2889
CGCGATGATT TCGAAATCAC CGTGCCGCAA ATTGACACTC TGGTAGAAAT 50
CGTCAAAGCT GTGATTGGCG ACAAAGGTGG CGTACGCATG ACCGGCGGCG 100
<210> 2890
<211> 100
<212> DNA
<213> Escherichia coli K-12 MG1655
<220> galm b0757
<222> 942-1041
<223> galm b0757 U00096 complement(787020..788060)
<313> 2890: 976-995
<400> 2890
GGACAGCCCG AACCACCCTG AATGGCCGCA ACCGGACTGC TTCCTGCGTC 50
CTGGCGAAGA GTATTCCAGC CTGACGGAAT ATCAGTTTAT TGCTGAGTAA 100
<210> 2891
<211> 100
<212> DNA
<213> Escherichia coli K-12 MG1655
<220> galm b0757
<222> 890-989
<223> galm b0757 U00096 complement(787020..788060)
<313> 2891: 945-964
<400> 2891
CCGAACCTTA CGCCGACTGG CAAGGGCTGG CTCTGGAAAG CGAGTTTCTA 50
CCGGACAGCC CGAACCACCC TGAATGGCCG CAACCGGACT GCTTCCTGCG 100
<210> 2892
<211> 100
<212> DNA

<213> Escherichia coli K-12 MG1655
<220> galm b0757
<222> 853-952
<223> galm b0757 U00096 complement(787020..788060)
<313> 2892: 893-912
<400> 2892
TCCGGCAACT TCCTCGGCGG CACACCGTCG CGGGGAACCG AACCTTACGC 50
CGACTGGCAA GGGCTGGCTC TGGAAAGCGA GTTTCTACCG GACAGCCCGA 100
<210> 2893
<211> 100
<212> DNA
<213> Escherichia coli K-12 MG1655
<220> galp b2944
<222> 1296-1395
<223> galp b2944 U00096 3086303..3087697
<313> 2893: 1360-1379
<400> 2893
ATTGTGGCTG GTACCGGAAA CCAAACACGT TTCGCTGGAA CATATTGAAC 50
GTAATCTGAT GAAAGGTCGT AAACTGCGCG AAATAGGCGC TCACGATTAA 100
<210> 2894
<211> 100
<212> DNA
<213> Escherichia coli K-12 MG1655
<220> galp b2944
<222> 1272-1371
<223> galp b2944 U00096 3086303..3087697
<313> 2894: 1313-1332
<400> 2894
GAACGTACTG TTTATCCTGC TGACATTGTG GCTGGTACCG GAAACCAAAC 50
ACGTTTCGCT GGAACATATT GAACGTAATC TGATGAAAGG TCGTAAACTG 100
<210> 2895
<211> 100
<212> DNA
<213> Escherichia coli K-12 MG1655
<220> galp b2944
<222> 1257-1356
<223> galp b2944 U00096 3086303..3087697
<313> 2895: 1271-1290
<400> 2895
GGTGTATGCG GCTCTGAACG TACTGTTTAT CCTGCTGACA TTGTGGCTGG 50
TACCGGAAAC CAAACACGTT TCGCTGGAAC ATATTGAACG TAATCTGATG 100
<210> 2896
<211> 100
<212> DNA
<213> Escherichia coli K-12 MG1655
<220> galr b2838
<222> 933-1032
<223> galr b2838 U00096 2974621..2975652
<313> 2896: 999-1018
<400> 2896
TCTCCCGGAA ATCACTAATG TCTTTAGTCC GACGCTGGTA CGTCGTCATT 50
CAGTGTCAAC TCCGTCGCTG GAGGCAAGTC ATCATGCAAC CAGCGACTAA 100
<210> 2897
<211> 100
<212> DNA
<213> Escherichia coli K-12 MG1655
<220> galr b2838
<222> 930-1029
<223> galr b2838 U00096 2974621..2975652
<313> 2897: 947-966
<400> 2897
CCCTCTCCCG GAAATCACTA ATGTCTTTAG TCCGACGCTG GTACGTCGTC 50
ATTCAGTGTC AACTCCGTCG CTGGAGGCAA GTCATCATGC AACCAGCGAC 100
<210> 2898
<211> 100
<212> DNA
<213> Escherichia coli K-12 MG1655
<220> galr b2838
<222> 837-936
<223> galr b2838 U00096 2974621..2975652
<313> 2898: 890-909

<400> 2898
CTATGTGCGT CCGCGCCTGA CCACCGTGCG TTACCCAATC GTGACGATGG 50
CGACCCAGGC TGCCGAACTG GCTTTGGCGC TGGCGGATAA TCGCCCTCTC 100
<210> 2899
<211> 100
<212> DNA
<213> Escherichia coli K-12 MG1655
<220> gals b2152
<222> 942-1041
<223> gals b2152 U00096 complement(2238648..2239688)
<313> 2899: 1014-1033
<400> 2899
TGCCAGCCAC TGTTTTATGC CGACGTTAGT GCGTCGCCAT TCTGTCGCAA 50
CACGCCAGAA TGCGGCGGCG ATCACTAACT CAACAAATCA GGCGATGTAA 100
<210> 2900
<211> 100
<212> DNA
<213> Escherichia coli K-12 MG1655
<220> gals b2152
<222> 924-1023
<223> gals b2152 U00096 complement(2238648..2239688)
<313> 2900: 939-958
<400> 2900
AGGCAATATT GATCCTCGTG CCAGCCACTG TTTTATGCCG ACGTTAGTGC 50
GTCGCCATTC TGTCGCAACA CGCCAGAATG CGGCGGCGAT CACTAACTCA 100
<210> 2901
<211> 100
<212> DNA
<213> Escherichia coli K-12 MG1655
<220> gals b2152
<222> 843-942
<223> gals b2152 U00096 complement(2238648..2239688)
<313> 2901: 892-911
<400> 2901
CGACCCGCAA TTAACGACCG TGCGTTATCC CATTGCTTCA ATGGCGAAAT 50
TAGCCACCGA ACTGGCCTTG CAGGGGGCAG CAGGCAATAT TGATCCTCGT 100
<210> 2902
<211> 100
<212> DNA
<213> Escherichia coli K-12 MG1655
<220> galt b0759
<222> 926-1025
<223> galt b0759 U00096 complement(789206..790252)
<313> 2902: 967-986
<400> 2902
TACGTAAATT TATGGTTGGT TATGAAATGC TGGCAGAGAC CCAGCGAGAC 50
CTGACCGCAG AACAGGCAGC AGAGCGTTTG CGCGCAGTCA GCGATATCCA 100
<210> 2903
<211> 100
<212> DNA
<213> Escherichia coli K-12 MG1655
<220> galt b0759
<222> 842-941
<223> galt b0759 U00096 complement(789206..790252)
<313> 2903: 891-910
<400> 2903
ACGGCGCGCC ATTTAATGGC GAAGAGAATC AACACTGGCA GCTGCACGCG 50
CACTTTTATC CGCCTCTGCT GCGCTCCGCC ACCGTACGTA AATTTATGGT 100
<210> 2904
<211> 100
<212> DNA
<213> Escherichia coli K-12 MG1655
<220> galt b0759
<222> 795-894
<223> galt b0759 U00096 complement(789206..790252)
<313> 2904: 828-847
<400> 2904
TTATGACAAC CTCTTCCAGT GCTCCTTCCC CTACTCTATG GGCTGGCACG 50
GCGCGCCATT TAATGGCGAA GAGAATCAAC ACTGGCAGCT GCACGCGCAC 100
<210> 2905
<211> 100

<212> DNA
<213> Escherichia coli K-12 MG1655
<220> yifa b3763
<222> 332-431
<223> yifa b3763 U00096 complement(3944752..3945348)
<313> 2905: 369-388
<400> 2905
AACAGCATGA GGCAGGTTTG ATCGGTGCTG ACGAAGTGCC CATTCTGACA 50
ACCAGTTCTG CTGAACTGGC ACAGCAACAG ATTGCGATGC TTAATGGTTG 100
<210> 2906
<211> 100
<212> DNA
<213> Escherichia coli K-12 MG1655
<220> yifa b3763
<222> 293-392
<223> yifa b3763 U00096 complement(3944752..3945348)
<313> 2906: 319-338
<400> 2906
ATCTTAATTA TCTGCGACTT GAGTGGGGGC CAGATTTTCA ACAGCATGAG 50
GCAGGTTTGA TCGGTGCTGA CGAAGTGCCC ATTCTGACAA CCAGTTCTGC 100
<210> 2907
<211> 100
<212> DNA
<213> Escherichia coli K-12 MG1655
<220> yifa b3763
<222> 158-257
<223> yifa b3763 U00096 complement(3944752..3945348)
<313> 2907: 226-245
<400> 2907
TAAAACAGCT GCATGAACGC CAGCTTGATC TTCTTATTAC CACTGAAGCG 50
CCCAAAATGG ACGAATTTAG TAGTCAGTTG CTGGGATATT TCACTTTAGC 100
<210> 2908
<211> 100
<212> DNA
<213> Escherichia coli K-12 MG1655
<220> galu b1237
<222> 810-909
<223> galu b1237 U00096 1290680..1291588
<313> 2908: 877-896
<400> 2908
AGGTTACATG CAGGCCTTCG TTGAATACGG TATTCGTCAT AACACCCTTG 50
GCACGGAATT TAAAGCCTGG CTTGAAGAAG AGATGGGCAT TAAGAAGTAA 100
<210> 2909
<211> 100
<212> DNA
<213> Escherichia coli K-12 MG1655
<220> galu b1237
<222> 706-805
<223> galu b1237 U00096 1290680..1291588
<313> 2909: 731-750
<400> 2909
GGTGATGAAA TTCAGCTCAC CGACGCAATT GATATGCTGA TCGAAAAAGA 50
AACGGTGGAA GCCTATCATA TGAAAGGGAA GAGCCATGAC TGCGGTAATA 100
<210> 2910
<211> 100
<212> DNA
<213> Escherichia coli K-12 MG1655
<220> galu b1237
<222> 602-701
<223> galu b1237 U00096 1290680..1291588
<313> 2910: 645-664
<400> 2910
AAAAACCGAA AGCGGATGTT GCGCCGTCTA ATCTCGCTAT TGTGGGTCGT 50
TACGTACTTA GCGCGGATAT TTGGCCGTTG CTGGCAAAAA CCCCTCCGGG 100
<210> 2911
<211> 100
<212> DNA
<213> Escherichia coli K-12 MG1655
<220> yifb b3766
<222> 1449-1548
<223> yifb b3766 U00096 complement(3946072..3947622)

<313> 2911: 1496-1515
<400> 2911
TATTGATCAG TCTGACATTA TCACACGTCA GCATTTGCAG GAGGCAGTTA 50
GCTATCGAGC GATTGACCGT TTGCTCATCC ATCTGCAGAA ACTACTGACA 100
<210> 2912
<211> 100
<212> DNA
<213> Escherichia coli K-12 MG1655
<220> yifb b3766
<222> 1411-1510
<223> yifb b3766 U00096 complement(3946072..3947622)
<313> 2912: 1461-1480
<400> 2912
TGGCAGCGGT TATTGAAAGT TGCACGAACC ATTGCTGATA TTGATCAGTC 50
TGACATTATC ACACGTCAGC ATTTGCAGGA GGCAGTTAGC TATCGAGCGA 100
<210> 2913
<211> 100
<212> DNA
<213> Escherichia coli K-12 MG1655
<220> yifb b3766
<222> 1389-1488
<223> yifb b3766 U00096 complement(3946072..3947622)
<313> 2913: 1420-1439
<400> 2913
TCTGGGGTTA TCGATTCGTG CCTGGCAGCG GTTATTGAAA GTTGCACGAA 50
CCATTGCTGA TATTGATCAG TCTGACATTA TCACACGTCA GCATTTGCAG 100
<210> 2914
<211> 100
<212> DNA
<213> Escherichia coli K-12 MG1655
<220> arah_1 b1900
<222> 553-652
<223> arah_1 b1900 U00096 complement(1980862..1981566)
<313> 2914: 599-618
<400> 2914
CTGATTATCT TTGGTTTGCT GCTGAATAAA ACCACCTTTG GTCGTAACAC 50
CCTGGCGATT GGCGGGAACG AAGAGCCGCG CGTCTGGCGG GTGTACCGGT 100
<210> 2915
<211> 100
<212> DNA
<213> Escherichia coli K-12 MG1655
<220> arah_1 b1900
<222> 508-607
<223> arah_1 b1900 U00096 complement(1980862..1981566)
<313> 2915: 557-576
<400> 2915
AACTGGTTCG GTCTGCCTGC GCCAATCTGG CTCACCGTCG CGTGTCTGAT 50
TATCTTTGGT TTGCTGCTGA ATAAAACCAC CTTTGGTCGT AACACCCTGG 100
<210> 2916
<211> 100
<212> DNA
<213> Escherichia coli K-12 MG1655
<220> arah_1 b1900
<222> 429-528
<223> arah_1 b1900 U00096 complement(1980862..1981566)
<313> 2916: 492-511
<400> 2916
TCGAGGTCTG GCGTACATCA TTTCAGACGG TAAAGCGGTC GGTATCGAAG 50
ATGAAAGCTT CTTTGCCCTT GGTTACGCCA ACTGGTTCGG TCTGCCTGCG 100
<210> 2917
<211> 100
<212> DNA
<213> Escherichia coli K-12 MG1655
<220> yife b3765
<222> 240-339
<223> yife b3765 U00096 3945709..3946047
<313> 2917: 320-339
<400> 2917
GTATATGACG CGTATTAAGC GTCCAAAACG TTTTCACACC CTTTCCGGCG 50
GTAAACCGCA GGTTGAAGGT GCTGAAGACT ACACCGATTC TGACGATTAA 100
<210> 2918

<211> 100
<212> DNA
<213> Escherichia coli K-12 MG1655
<220> yife b3765
<222> 240-339
<223> yife b3765 U00096 3945709..3946047
<313> 2918: 294-313
<400> 2918
GTATATGACG CGTATTAAGC GTCCAAAACG TTTTCACACC CTTTCCGGCG 50
GTAAACCGCA GGTTGAAGGT GCTGAAGACT ACACCGATTC TGACGATTAA 100
<210> 2919
<211> 100
<212> DNA
<213> Escherichia coli K-12 MG1655
<220> yife b3765
<222> 240-339
<223> yife b3765 U00096 3945709..3946047
<313> 2919: 293-312
<400> 2919
GTATATGACG CGTATTAAGC GTCCAAAACG TTTTCACACC CTTTCCGGCG 50
GTAAACCGCA GGTTGAAGGT GCTGAAGACT ACACCGATTC TGACGATTAA 100
<210> 2920
<211> 100
<212> DNA
<213> Escherichia coli K-12 MG1655
<220> arah_2 b1899
<222> 162-261
<223> arah_2 b1899 U00096 complement(1980578..1980838)
<313> 2920: 228-247
<400> 2920
TAATATTTCT CCTTTCGCGC AGTACGTGGT TCGCGGCTTA ATCCTGCTGG 50
CAGCGGTGAT CTTCGACCGT TACAAGCAAA AAGCGAAACG CACTGTCTGA 100
<210> 2921
<211> 100
<212> DNA
<213> Escherichia coli K-12 MG1655
<220> arah_2 b1899
<222> 147-246
<223> arah_2 b1899 U00096 complement(1980578..1980838)
<313> 2921: 179-198
<400> 2921
CGCCATGAAC CTGCTTAATA TTTCTCCTTT CGCGCAGTAC GTGGTTCGCG 50
GCTTAATCCT GCTGGCAGCG GTGATCTTCG ACCGTTACAA GCAAAAAGCG 100
<210> 2922
<211> 100
<212> DNA
<213> Escherichia coli K-12 MG1655
<220> arah_2 b1899
<222> 110-209
<223> arah_2 b1899 U00096 complement(1980578..1980838)
<313> 2922: 123-142
<400> 2922
TGGTGGCGGG TATCTTAATT TTAGGCACCG TGGAAAACGC CATGAACCTG 50
CTTAATATTT CTCCTTTCGC GCAGTACGTG GTTCGCGGCT TAATCCTGCT 100
<210> 2923
<211> 100
<212> DNA
<213> Escherichia coli K-12 MG1655
<220> yifk b3796
<222> 1287-1386
<223> yifk b3796 U00096 3978500..3979885
<313> 2923: 1363-1382
<400> 2923
GTCGCTGTTT GTTGGCATCA TCTTTATGCT GGCGGTGACG GCGATTTATA 50
AAGTTTTTGG CCTTAATCGC CACGGGAAAG CGCATAAACT GGAGGAATAA 100
<210> 2924
<211> 100
<212> DNA
<213> Escherichia coli K-12 MG1655
<220> yifk b3796
<222> 1208-1307

<223> yifk b3796 U00096 3978500..3979885
<313> 2924: 1235-1254
<400> 2924
TCCCGTGGGC CAATTACGTA ACAATGGCAT TCCTGATTTG CGTTTTGATC 50
GGCATGTACT TTAATGAAGA TACGCGTATG TCGCTGTTTG TTGGCATCAT 100
<210> 2925
<211> 100
<212> DNA
<213> Escherichia coli K-12 MG1655
<220> yifk b3796
<222> 1149-1248
<223> yifk b3796 U00096 3978500..3979885
<313> 2925: 1184-1203
<400> 2925
GCGTTTTCGG CGTGCACATA AAGCGGCGAT TGCCAGCCAT CCGTTCCGCT 50
CAATCCTGTT CCCGTGGGCC AATTACGTAA CAATGGCATT CCTGATTTGC 100
<210> 2926
<211> 100
<212> DNA
<213> Escherichia coli K-12 MG1655
<220> b2970 b2970
<222> 768-867
<223> b2970 b2970 U00096 complement(3110071..3110937)
<313> 2926: 834-853
<400> 2926
TTCCATTCAA CTTACGGTTT TACGCAAGGG TGCGCGCCAC GACATTTCCA 50
TCGCGCTGCG CTACCGACTA TTCTGCCCGC CTTACTGGAA GAAGAGCTGA 100
<210> 2927
<211> 100
<212> DNA
<213> Escherichia coli K-12 MG1655
<220> b2970 b2970
<222> 733-832
<223> b2970 b2970 U00096 complement(3110071..3110937)
<313> 2927: 762-781
<400> 2927
ATGATTGCTC TGATGCGGCA GTTACCTTCA ATGGATTCCA TTCAACTTAC 50
GGTTTTACGC AAGGGTGCGC GCCACGACAT TTCCATCGCG CTGCGCTACC 100
<210> 2928
<211> 100
<212> DNA
<213> Escherichia coli K-12 MG1655
<220> b2970 b2970
<222> 651-750
<223> b2970 b2970 U00096 complement(3110071..3110937)
<313> 2928: 707-726
<400> 2928
TTCTCTGTTC GATGCCAGCG GTTTCAAGGA AGGCGATATC GCCATTGCGC 50
TAAATCAGCA GGATTTCACT GATCCACGAG CAATGATTGC TCTGATGCGG 100
<210> 2929
<211> 100
<212> DNA
<213> Escherichia coli K-12 MG1655
<220> mura b3190
<222> 1161-1260
<223> mura b3190 U00096 complement(3332876..3334135)
<313> 2929: 1223-1242
<400> 2929
GGTGGTTGAT CGTATTTATC ACATCGATCG TGGCTACGAA CGCATTGAAG 50
ACAAACTGCG CGCTTTAGGT GCAAATATTG AGCGTGTGAA AGGCGAATAA 100
<210> 2930
<211> 100
<212> DNA
<213> Escherichia coli K-12 MG1655
<220> mura b3190
<222> 1040-1139
<223> mura b3190 U00096 complement(3332876..3334135)
<313> 2930: 1085-1104
<400> 2930
TCGAAAGCAA TACCGTTATT TGTCACGGTG TTGAAAAACT TTCTGGCGCA 50
CAGGTTATGG CAACCGATCT GCGTGCATCA GCAAGCCTGG TGCTGGCTGG 100

<210> 2931
<211> 100
<212> DNA
<213> Escherichia coli K-12 MG1655
<220> mura b3190
<222> 979-1078
<223> mura b3190 U00096 complement(3332876..3334135)
<313> 2931: 1028-1047
<400> 2931
GTCTTTGAAA ACCGCTTTAT GCATGTGCCA GAGCTGAGCC GTATGGGCGC 50
GCACGCCGAA ATCGAAAGCA ATACCGTTAT TTGTCACGGT GTTGAAAAAC 100
<210> 2932
<211> 100
<212> DNA
<213> Escherichia coli K-12 MG1655
<220> b2971 b2971
<222> 312-411
<223> b2971 b2971 U00096 complement(3111084..3111494)
<313> 2932: 385-404
<400> 2932
TGAGGGAATT AATTACAGCA TAACGATTAA TGATACACGT ACAGGTAACC 50
AGTATCAGCG GAAACTGGAT CGTACCACCT GTGGAATAGT CAAAGCATAA 100
<210> 2933
<211> 100
<212> DNA
<213> Escherichia coli K-12 MG1655
<220> b2971 b2971
<222> 269-368
<223> b2971 b2971 U00096 complement(3111084..3111494)
<313> 2933: 288-307
<400> 2933
AACGACAAAT GTGCGCTGAC CCGACGGTGA AATTAATGAT CACTGAGGGA 50
ATTAATTACA GCATAACGAT TAATGATACA CGTACAGGTA ACCAGTATCA 100
<210> 2934
<211> 100
<212> DNA
<213> Escherichia coli K-12 MG1655
<220> b2971 b2971
<222> 230-329
<223> b2971 b2971 U00096 complement(3111084..3111494)
<313> 2934: 256-275
<400> 2934
AAACCACGCA GACGCCTGAC GCCTTTTTAA CCAGCTATCA ACGACAAATG 50
TGCGCTGACC CGACGGTGAA ATTAATGATC ACTGAGGGAA TTAATTACAG 100
<210> 2935
<211> 100
<212> DNA
<213> Escherichia coli K-12 MG1655
<220> yifn b3778
<222> 130-229
<223> yifn b3778 U00096 complement(3957857..3958117)
<313> 2935: 153-172
<400> 2935
ACGACGTATT ACGATGGCCG CATTCCTCCT GAAATGATAA AAAACAGGTT 50
AGTTGTCGTG CTCAACGGAA AAATTAATGG CAACGCATGC ATCGTCGTTC 100
<210> 2936
<211> 100
<212> DNA
<213> Escherichia coli K-12 MG1655
<220> yifn b3778
<222> 80-179
<223> yifn b3778 U00096 complement(3957857..3958117)
<313> 2936: 109-128
<400> 2936
TGGAATGCAA CTTTGGTAAC TATCCTGTTA GCCAAAATGG ACCGTTCAGC 50
ACGACGTATT ACGATGGCCG CATTCCTCCT GAAATGATAA AAAACAGGTT 100
<210> 2937
<211> 100
<212> DNA
<213> Escherichia coli K-12 MG1655
<220> yifn b3778

<222> 13-112
<223> yifn b3778 U00096 complement(3957857..3958117)
<313> 2937: 49-68
<400> 2937
TTTCAGAGTT TACTAAGGAG AGGTTTACTC GTGGCGATTA ATTTCAGCCC 50
TAAGGTTGGT GAAATACTGG AATGCAACTT TGGTAACTAT CCTGTTAGCC 100
<210> 2938
<211> 100
<212> DNA
<213> Escherichia coli K-12 MG1655
<220> murb b3973
<222> 787-886
<223> murb b3973 U00096 4169636..4170664
<313> 2938: 813-832
<400> 2938
CTGGCAGCAG GTTGGCTTAT CGATCAGTGC CAGCTAAAAG GGATGCAAAT 50
AGGTGGGGCT GCGGTGCACC GTCAACAGGC GTTAGTTCTC ATTAATGAAG 100
<210> 2939
<211> 100
<212> DNA
<213> Escherichia coli K-12 MG1655
<220> murb b3973
<222> 727-826
<223> murb b3973 U00096 4169636..4170664
<313> 2939: 769-788
<400> 2939
GCATTACTGT CACAATTTCC AACAGCACCA AATTACCCCC AGGCGGATGG 50
TTCAGTAAAA CTGGCAGCAG GTTGGCTTAT CGATCAGTGC CAGCTAAAAG 100
<210> 2940
<211> 100
<212> DNA
<213> Escherichia coli K-12 MG1655
<220> murb b3973
<222> 709-808
<223> murb b3973 U00096 4169636..4170664
<313> 2940: 739-758
<400> 2940
TCTGCCGAAA CGGCTAAAGC ATTACTGTCA CAATTTCCAA CAGCACCAAA 50
TTACCCCCAG GCGGATGGTT CAGTAAAACT GGCAGCAGGT TGGCTTATCG 100
<210> 2941
<211> 100
<212> DNA
<213> Escherichia coli K-12 MG1655
<220> b2972 b2972
<222> 812-911
<223> b2972 b2972 U00096 complement(3111560..3112492)
<313> 2941: 861-880
<400> 2941
TCGCCTCATG CTGCGGCCTG ATATATGCCG TTATTACAAA AAGAGGATCA 50
ACCACACTGC CTTTTGGACC GTGTTTAAGT CTGGGCGGTA TAGCAACACT 100
<210> 2942
<211> 100
<212> DNA
<213> Escherichia coli K-12 MG1655
<220> b2972 b2972
<222> 740-839
<223> b2972 b2972 U00096 complement(3111560..3112492)
<313> 2942: 772-791
<400> 2942
TGGGCGATGT ATTACTTTTC GCCGCGTTAG GTGGCTGGGT GGGGGCATTG 50
TCGCTGCCCA ATGTTGCTTT AATCGCCTCA TGCTGCGGCC TGATATATGC 100
<210> 2943
<211> 100
<212> DNA
<213> Escherichia coli K-12 MG1655
<220> b2972 b2972
<222> 699-798
<223> b2972 b2972 U00096 complement(3111560..3112492)
<313> 2943: 726-745
<400> 2943
TTGGATAGCC GGAATAGTTC TGCGTAAAGA AGCATTAGGC ATGGGCGATG 50

TATTACTTTT CGCCGCGTTA GGTGGCTGGG TGGGGGCATT GTCGCTGCCC  100
<210> 2944
<211> 100
<212> DNA
<213> Escherichia coli K-12 MG1655
<220> murc b0092
<222> 1361-1460
<223> murc b0092 U00096 100765..102240
<313> 2944: 1390-1409
<400> 2944
TAACCGGTAA CGACCTGATT CTCGTTCAGG GGGCTGGTAA TATTGGAAAA  50
ATTGCCCGTT CTTTAGCTGA AATCAAACTG AAGCCGCAAA CTCCGGAGGA  100
<210> 2945
<211> 100
<212> DNA
<213> Escherichia coli K-12 MG1655
<220> murc b0092
<222> 1254-1353
<223> murc b0092 U00096 100765..102240
<313> 2945: 1290-1309
<400> 2945
GGGAGCGGAC AGCCGTTCGC TGTGTCGCAC AATTCGTGGA CGTGGGAAAA  50
TTGATCCCAT TCTGGTGCCG GATCCGGCGC GGGTAGCCGA GATGCTGGCA  100
<210> 2946
<211> 100
<212> DNA
<213> Escherichia coli K-12 MG1655
<220> murc b0092
<222> 1162-1261
<223> murc b0092 U00096 100765..102240
<313> 2946: 1204-1223
<400> 2946
CGCGACCTGT ATGATGATTT CGCCAATGTG CTGACGCAGG TTGATACCCT  50
GTTGATGCTG GAAGTGTATC CGGCTGGCGA AGCGCCAATT CCGGGAGCGG  100
<210> 2947
<211> 100
<212> DNA
<213> Escherichia coli K-12 MG1655
<220> b2973 b2973
<222> 2448-2547
<223> b2973 b2973 U00096 complement(3112567..3115113)
<313> 2947: 2508-2527
<400> 2947
GTCGGCTTAC AACACGCTCG CGTCACTCGA TCTGCCGAAA CCGGAACAGG  50
GACCGGAAAC CATTAATCAG GTTACCGAGC ATAAGATGTC TGCCGAGTAA  100
<210> 2948
<211> 100
<212> DNA
<213> Escherichia coli K-12 MG1655
<220> b2973 b2973
<222> 2440-2539
<223> b2973 b2973 U00096 complement(3112567..3115113)
<313> 2948: 2460-2479
<400> 2948
GTGAGCCAGT CGGCTTACAA CACGCTCGCG TCACTCGATC TGCCGAAACC  50
GGAACAGGGA CCGGAAACCA TTAATCAGGT TACCGAGCAT AAGATGTCTG  100
<210> 2949
<211> 100
<212> DNA
<213> Escherichia coli K-12 MG1655
<220> b2973 b2973
<222> 2343-2442
<223> b2973 b2973 U00096 complement(3112567..3115113)
<313> 2949: 2380-2399
<400> 2949
CGCCCAGACG GATCTTTCGG AGTTCTTTAA GAAATGGAAT CCGGGCGCGA  50
ATGCTTACCA GCTGCCGGGG GCGAGCGAGA TGAGCTTCGA GGGCGGTGTG  100
<210> 2950
<211> 100
<212> DNA
<213> Escherichia coli K-12 MG1655

<220> murd b0089
<222> 1218-1317
<223> murd b0089 U00096 97087..98403
<313> 2950: 1280-1299
<400> 2950
TATGGTTCTG CTCTCCCCAG CCTGTGCCAG CCTTGATCAG TTCAAGAACT 50
TTGAACAACG AGGCAATGAG TTTGCCCGTC TGGCGAAGGA GTTAGGTTGA 100
<210> 2951
<211> 100
<212> DNA
<213> Escherichia coli K-12 MG1655
<220> murd b0089
<222> 1187-1286
<223> murd b0089 U00096 97087..98403
<313> 2951: 1222-1241
<400> 2951
GCTTGCTGGC TCCGCGTGTT CAGCCGGGCG ATATGGTTCT GCTCTCCCCA 50
GCCTGTGCCA GCCTTGATCA GTTCAAGAAC TTTGAACAAC GAGGCAATGA 100
<210> 2952
<211> 100
<212> DNA
<213> Escherichia coli K-12 MG1655
<220> murd b0089
<222> 1125-1224
<223> murd b0089 U00096 97087..98403
<313> 2952: 1162-1181
<400> 2952
GCAGCTGGCG GCGCTACGCC CGGAAGTGGC AGAACAAACC GAAACTATGG 50
AACAGGCGAT GCGCTTGCTG GCTCCGCGTG TTCAGCCGGG CGATATGGTT 100
<210> 2953
<211> 100
<212> DNA
<213> Escherichia coli K-12 MG1655
<220> b2974 b2975
<222> 1845-1944
<223> b2974 b2975 U00096 complement(3115101..3117128)
<313> 2953: 1870-1889
<400> 2953
ATGGAAGCCG GACGCGAAAG CCAGCATGAC CGTAGGCACC AACCTGGATA 50
CTGTCTATTT TAAACGTCAT GGTCAGGTTA CAGGAAACAG CGCTGCGTTC 100
<210> 2954
<211> 100
<212> DNA
<213> Escherichia coli K-12 MG1655
<220> b2974 b2975
<222> 1782-1881
<223> b2974 b2975 U00096 complement(3115101..3117128)
<313> 2954: 1833-1852
<400> 2954
CAGCGACCCG GATGACATGA AGAACTTCAT GGAGAACGTG CTGCGCTATC 50
TGTCCGACGA TAAATGGAAG CCGGACGCGA AAGCCAGCAT GACCGTAGGC 100
<210> 2955
<211> 100
<212> DNA
<213> Escherichia coli K-12 MG1655
<220> b2974 b2975
<222> 1663-1762
<223> b2974 b2975 U00096 complement(3115101..3117128)
<313> 2955: 1702-1721
<400> 2955
CAAGTCGGTG AGGGCAAACT GATGGTTATC GGTAACCCAC ACTACAACAG 50
CATTTTGCGT TGCCCGAACG GTTACAGCTG GAACGGGGGC GTTAATAAAG 100
<210> 2956
<211> 100
<212> DNA
<213> Escherichia coli K-12 MG1655
<220> mure b0086
<222> 1344-1443
<223> mure b0086 U00096 93166..94653
<313> 2956: 1397-1416
<400> 2956

GACTTGCGCC GTTATGCAGG CTAAAGAGAA TGATGTGGTA CTGGTCGCGG 50
GCAAAGGCCA TGAAGATTAC CAGATTGTTG GCAATCAGCG TCTGGACTAC 100
<210> 2957
<211> 100
<212> DNA
<213> Escherichia coli K-12 MG1655
<220> mure b0086
<222> 1297-1396
<223> mure b0086 U00096 93166..94653
<313> 2957: 1358-1377
<400> 2957
TTAGATGCCG GACATGCCAA AGTGATGGAA GGCCGTGCTG AAGCGGTGAC 50
TTGCGCCGTT ATGCAGGCTA AAGAGAATGA TGTGGTACTG GTCGCGGGCA 100
<210> 2958
<211> 100
<212> DNA
<213> Escherichia coli K-12 MG1655
<220> mure b0086
<222> 1277-1376
<223> mure b0086 U00096 93166..94653
<313> 2958: 1308-1327
<400> 2958
ACGATATTCT GGCGGGAATG TTAGATGCCG GACATGCCAA AGTGATGGAA 50
GGCCGTGCTG AAGCGGTGAC TTGCGCCGTT ATGCAGGCTA AAGAGAATGA 100
<210> 2959
<211> 100
<212> DNA
<213> Escherichia coli K-12 MG1655
<220> murf b0087
<222> 1260-1359
<223> murf b0087 U00096 94650..96008
<313> 2959: 1323-1342
<400> 2959
GATTGCTGAG CAACAGGTAA TTACGATTTT AGTTAAGGGT TCACGTAGTG 50
CCGCCATGGA AGAGGTAGTA CGCGCTTTAC AGGAGAATGG GACATGTTAG 100
<210> 2960
<211> 100
<212> DNA
<213> Escherichia coli K-12 MG1655
<220> murf b0087
<222> 1100-1199
<223> murf b0087 U00096 94650..96008
<313> 2960: 1147-1166
<400> 2960
AAAGCGAAGC CTGCCATGTA CAGGTGGGCG AGGCGGCAAA AGCTGCTGGT 50
ATTGACCGCG TGTTAAGCGT GGGTAAACAA AGCCATGCTA TCAGCACCGC 100
<210> 2961
<211> 100
<212> DNA
<213> Escherichia coli K-12 MG1655
<220> murf b0087
<222> 1069-1168
<223> murf b0087 U00096 94650..96008
<313> 2961: 1091-1110
<400> 2961
GTGGTGGGCG ATATGGCGGA ACTGGGCGCT GAAAGCGAAG CCTGCCATGT 50
ACAGGTGGGC GAGGCGGCAA AAGCTGCTGG TATTGACCGC GTGTTAAGCG 100
<210> 2962
<211> 100
<212> DNA
<213> Escherichia coli K-12 MG1655
<220> murg b0091
<222> 895-994
<223> murg b0091 U00096 99644..100711
<313> 2962: 932-951
<400> 2962
GGCGCAGCCA AAATTATCGA GCAGCCACAG CTTAGCGTGG ATGCTGTCGC 50
CAACACCCTG GCCGGGTGGT CGCGAGAAAC CTTATTAACC ATGGCAGAAC 100
<210> 2963
<211> 100
<212> DNA

<213> Escherichia coli K-12 MG1655
<220> murg b0091
<222> 837-936
<223> murg b0091 U00096 99644..100711
<313> 2963: 890-909
<400> 2963
GCCGTTTCAA CATAAAGACC GCCAGCAATA CTGGAATGCG CTACCGCTGG 50
AAAAAGCGGG CGCAGCCAAA ATTATCGAGC AGCCACAGCT TAGCGTGGAT 100
<210> 2964
<211> 100
<212> DNA
<213> Escherichia coli K-12 MG1655
<220> murg b0091
<222> 807-906
<223> murg b0091 U00096 99644..100711
<313> 2964: 856-875
<400> 2964
CGCCGCGGCA GGACTACCGG CGTTGTTTGT GCCGTTTCAA CATAAAGACC 50
GCCAGCAATA CTGGAATGCG CTACCGCTGG AAAAAGCGGG CGCAGCCAAA 100
<210> 2965
<211> 100
<212> DNA
<213> Escherichia coli K-12 MG1655
<220> muri b3968
<222> 771-870
<223> muri b3968 U00096 4162995..4163864
<313> 2965: 817-836
<400> 2965
TGCCTTTTGT ATGGCAATGA CGCCAGGAGC TGAACAATTA TTGCCCGTTT 50
TACAGCGTTA CGGCTTCGAA ACGCTCGAAA AACTGGCAGT TTTAGGCTGA 100
<210> 2966
<211> 100
<212> DNA
<213> Escherichia coli K-12 MG1655
<220> muri b3968
<222> 697-796
<223> muri b3968 U00096 4162995..4163864
<313> 2966: 742-761
<400> 2966
GCAGCGATTG CTCGCCGAAC GGCCTGGTTG TTAGAACATG AAGCCCCGGA 50
TGCAAAATCT GCCGATGCGA ATATTGCCTT TTGTATGGCA ATGACGCCAG 100
<210> 2967
<211> 100
<212> DNA
<213> Escherichia coli K-12 MG1655
<220> muri b3968
<222> 626-725
<223> muri b3968 U00096 4162995..4163864
<313> 2967: 650-669
<400> 2967
CCCATTTCCC TCTACTACAA GAAGAACTGT TACAAGTGCT GCCAGAGGGA 50
ACCCGGCTGG TGGATTCTGG CGCAGCGATT GCTCGCCGAA CGGCCTGGTT 100
<210> 2968
<211> 100
<212> DNA
<213> Escherichia coli K-12 MG1655
<220> b0005 b0006
<222> 178-277
<223> b0005 b0006 U00096 5234..5530
<313> 2968: 225-244
<400> 2968
CATTATGAAT GGCGAGGCAA TCGCTGGCAC CTACACGGAC CGCCGCCACC 50
GCCGCGCCAC CATAAGAAAG CTCCTCATGA TCATCACGGC GGTCATGGTC 100
<210> 2969
<211> 100
<212> DNA
<213> Escherichia coli K-12 MG1655
<220> b0005 b0006
<222> 130-229
<223> b0005 b0006 U00096 5234..5530
<313> 2969: 190-209

<400> 2969
TATTACTGGG ATGGAGGTCA CTGGCGCGAC CACGGCTGGT GGAAACAACA 50
TTATGAATGG CGAGGCAATC GCTGGCACCT ACACGGACCG CCGCCACCGC 100
<210> 2970
<211> 100
<212> DNA
<213> Escherichia coli K-12 MG1655
<220> b0005 b0006
<222> 85-184
<223> b0005 b0006 U00096 5234..5530
<313> 2970: 145-164
<400> 2970
GTCCCGTCAG TAAAATTACA GATAGGCGAT CGTGATAATC GTGGCTATTA 50
CTGGGATGGA GGTCACTGGC GCGACCACGG CTGGTGGAAA CAACATTATG 100
<210> 2971
<211> 100
<212> DNA
<213> Escherichia coli K-12 MG1655
<220> yiga b3811
<222> 586-685
<223> yiga b3811 U00096 3993196..3993903
<313> 2971: 616-635
<400> 2971
GGTGTCGTGC TGTTTACCAG TCGCGATGCC AGTCACTATC AACAAGGGCA 50
AGGAACGCAG TTACTTCATG AAATTGCGCT GATGTTGCCG GAGCTTCTGG 100
<210> 2972
<211> 100
<212> DNA
<213> Escherichia coli K-12 MG1655
<220> yiga b3811
<222> 446-545
<223> yiga b3811 U00096 3993196..3993903
<313> 2972: 489-508
<400> 2972
AACCGCTGCG TATTCAGCGT TTGGGGCAGG AACAGCACTA TCTTGGGCCG 50
CTTAACGGAC CAGAGCTGCT GGTGGTGCTA CCGGAAGCGA AAGCGGTGGG 100
<210> 2973
<211> 100
<212> DNA
<213> Escherichia coli K-12 MG1655
<220> yiga b3811
<222> 402-501
<223> yiga b3811 U00096 3993196..3993903
<313> 2973: 445-464
<400> 2973
GCCGTCGAAC CACACTCATC TGGCATTAAG CCGTCAGTCT TTCGAACCGC 50
TGCGTATTCA GCGTTTGGGG CAGGAACAGC ACTATCTTGG GCCGCTTAAC 100
<210> 2974
<211> 100
<212> DNA
<213> Escherichia coli K-12 MG1655
<220> yigb b3813
<222> 618-717
<223> yigb b3813 U00096 3994796..3995512
<313> 2974: 652-671
<400> 2974
GATCAGACCG GAAAATGGCG ATCTGATGCA AACCTGGGAC AGCCGTTTAC 50
TGCCGCATCT GGAAATTTCC CGGTTGGCAT CTCTGACCTC GCTGATATAA 100
<210> 2975
<211> 100
<212> DNA
<213> Escherichia coli K-12 MG1655
<220> yigb b3813
<222> 552-651
<223> yigb b3813 U00096 3994796..3995512
<313> 2975: 586-605
<400> 2975
ACATGTTGGG GACGATCTCA CCACTGACGT GGGTGGGGCA ATTCGCAGCG 50
GAATGCAGGC TTGTTGGATC AGACCGGAAA ATGGCGATCT GATGCAAACC 100
<210> 2976
<211> 100

<212> DNA
<213> Escherichia coli K-12 MG1655
<220> yigb b3813
<222> 454-553
<223> yigb b3813 U00096 3994796..3995512
<313> 2976: 518-537
<400> 2976
TTTGTGCTGC GCGCTGGCCC GCACGGGCGC TCAAAACCGT TCAGCGATAT 50
GTACTTTTTG GCTGCGGAAA AACTCAACGT GCCGATCGGC GAGATCTTAC 100
<210> 2977
<211> 100
<212> DNA
<213> Escherichia coli K-12 MG1655
<220> yigc b3844
<222> 1395-1494
<223> yigc b3844 U00096 4022578..4024071
<313> 2977: 1473-1492
<400> 2977
TGAATGGGGA CGTCCCATCA AAAAAGATCC AGATGTTGTC GCGCATATTG 50
ACGCCATCTG GGATGAACTG GCTATTTTTA ACAACGGTAA AAGCGCCTGA 100
<210> 2978
<211> 100
<212> DNA
<213> Escherichia coli K-12 MG1655
<220> yigc b3844
<222> 1395-1494
<223> yigc b3844 U00096 4022578..4024071
<313> 2978: 1417-1436
<400> 2978
TGAATGGGGA CGTCCCATCA AAAAAGATCC AGATGTTGTC GCGCATATTG 50
ACGCCATCTG GGATGAACTG GCTATTTTTA ACAACGGTAA AAGCGCCTGA 100
<210> 2979
<211> 100
<212> DNA
<213> Escherichia coli K-12 MG1655
<220> yigc b3844
<222> 1345-1444
<223> yigc b3844 U00096 4022578..4024071
<313> 2979: 1381-1400
<400> 2979
TCAAAAATGG GGCTGGATGC CACGAATAAA TGGCCGGGGG AAACCCAGCG 50
TGAATGGGGA CGTCCCATCA AAAAAGATCC AGATGTTGTC GCGCATATTG 100
<210> 2980
<211> 100
<212> DNA
<213> Escherichia coli K-12 MG1655
<220> ssea b2522
<222> 900-999
<223> ssea b2522 U00096 2650355..2651359
<313> 2980: 940-959
<400> 2980
TTTGTTAGCA CTCGCGACGC TGGATGTGCC AAACGTGAAA CTGTACGACG 50
GCGCATGGAG TGAATGGGGC GCGCGGGCAG ATTTACCGGT TGAGCCAGTG 100
<210> 2981
<211> 100
<212> DNA
<213> Escherichia coli K-12 MG1655
<220> ssea b2522
<222> 760-859
<223> ssea b2522 U00096 2650355..2651359
<313> 2981: 798-817
<400> 2981
AATGTTCCGT GGACGGAACT GGTGCGCGAA GGCGAACTAA AAACGACCGA 50
TGAACTGGAT GCGATATTTT TTGGTCGCGG CGTCAGCTAC GACAAACCAA 100
<210> 2982
<211> 100
<212> DNA
<213> Escherichia coli K-12 MG1655
<220> ssea b2522
<222> 686-785
<223> ssea b2522 U00096 2650355..2651359

<313> 2982: 724-743
<400> 2982
CGGCTGCACG TTTTAACGCA GAAGTTGATG AACCTCGCCC AGGTTTACGT  50
CGCGGACATA TTCCCGGAGC ACTGAATGTT CCGTGGACGG AACTGGTGCG  100
<210> 2983
<211> 100
<212> DNA
<213> Escherichia coli K-12 MG1655
<220> sseb b2523
<222> 687-786
<223> sseb b2523 U00096 complement(2652177..2652962)
<313> 2983: 757-776
<400> 2983
AAAAGGAATT AGCCACTTTA TTACCGAACA TATTGCGCCA TTCTATGAAC  50
GTCGCTGGGG TGGTTTTTTG CGTGACTTTA AACAGAATCG GATAATCTAA  100
<210> 2984
<211> 100
<212> DNA
<213> Escherichia coli K-12 MG1655
<220> sseb b2523
<222> 626-725
<223> sseb b2523 U00096 complement(2652177..2652962)
<313> 2984: 650-669
<400> 2984
TAGCGACCGA TACATTACCT GGCGATGAAC CAATCGATAT TTGTCAGGTG  50
AAAAAAGGGG AAAAAGGAAT TAGCCACTTT ATTACCGAAC ATATTGCGCC  100
<210> 2985
<211> 100
<212> DNA
<213> Escherichia coli K-12 MG1655
<220> sseb b2523
<222> 593-692
<223> sseb b2523 U00096 complement(2652177..2652962)
<313> 2985: 618-637
<400> 2985
ATATCGAAGA AATTATTCAG GCGACGGGAA GTGTAGCGAC CGATACATTA  50
CCTGGCGATG AACCAATCGA TATTTGTCAG GTGAAAAAAG GGGAAAAAGG  100
<210> 2986
<211> 100
<212> DNA
<213> Escherichia coli K-12 MG1655
<220> yige b3816
<222> 387-486
<223> yige b3816 U00096 complement(3998183..3998668)
<313> 2986: 452-471
<400> 2986
GTGTTTTATG TCGCGGGAGA TAAAGTCGGC ATCGTTCGTC TGGATGCCTT  50
CAAAACCAGT AAAGAGATTC AGTTTGCGGT GCAGTCAGGG CCAATGTTGA  100
<210> 2987
<211> 100
<212> DNA
<213> Escherichia coli K-12 MG1655
<220> yige b3816
<222> 367-466
<223> yige b3816 U00096 complement(3998183..3998668)
<313> 2987: 404-423
<400> 2987
TTCTTTATCC GTCTGGCGGC GTGTTTTATG TCGCGGGAGA TAAAGTCGGC  50
ATCGTTCGTC TGGATGCCTT CAAAACCAGT AAAGAGATTC AGTTTGCGGT  100
<210> 2988
<211> 100
<212> DNA
<213> Escherichia coli K-12 MG1655
<220> yige b3816
<222> 312-411
<223> yige b3816 U00096 complement(3998183..3998668)
<313> 2988: 339-358
<400> 2988
CATCGAAAAC GGTCAGCAGA AGGTGGCGTT AAATCTCGCT TCAGGTGAAG  50
GGAATTTCTT TATCCGTCTG GCGGCGTGTT TTATGTCGCG GGAGATAAAG  100
<210> 2989

<211> 100
<212> DNA
<213> Escherichia coli K-12 MG1655
<220> yigf b3818
<222> 282-381
<223> yigf b3818 U00096 complement(4000031..4000411)
<313> 2989: 357-376
<400> 2989
TAACATTCTG GGAATTGCCG TAGCAGCCTA TGTTGCTGTT AACACGAACA 50
AATGGTTCTA TGAGAAAGAG GTAAAAGGGT TAAATACCTG GAGCCTTTGA 100
<210> 2990
<211> 100
<212> DNA
<213> Escherichia coli K-12 MG1655
<220> yigf b3818
<222> 278-377
<223> yigf b3818 U00096 complement(4000031..4000411)
<313> 2990: 338-357
<400> 2990
GCGTTAACAT TCTGGGAATT GCCGTAGCAG CCTATGTTGC TGTTAACACG 50
AACAAATGGT TCTATGAGAA AGAGGTAAAA GGGTTAAATA CCTGGAGCCT 100
<210> 2991
<211> 100
<212> DNA
<213> Escherichia coli K-12 MG1655
<220> yigf b3818
<222> 117-216
<223> yigf b3818 U00096 complement(4000031..4000411)
<313> 2991: 161-180
<400> 2991
AGCATTTAAA GCATTAAAAG TAAGACAACG CTTAACTATT CAAATGAATT 50
TCATTGCTTT CTTCTGTTCC TGGATATATT TATTTGTCCT GGGGTTATGG 100
<210> 2992
<211> 100
<212> DNA
<213> Escherichia coli K-12 MG1655
<220> yigg b3819
<222> 318-417
<223> yigg b3819 U00096 complement(4000425..4000841)
<313> 2992: 397-416
<400> 2992
TTTTGCCTGT TCAGGCAATG GTTCGATGAT TTTTTTGATT TGTTATTTTT 50
CCACTTTCAG TATGCTAATG CTTGCACCTA CTGATTCATC AAAACAATAG 100
<210> 2993
<211> 100
<212> DNA
<213> Escherichia coli K-12 MG1655
<220> yigg b3819
<222> 298-397
<223> yigg b3819 U00096 complement(4000425..4000841)
<313> 2993: 340-359
<400> 2993
ATCACAATGA TAGCCGAAGA TTTTGCCTGT TCAGGCAATG GTTCGATGAT 50
TTTTTTGATT TGTTATTTTT CCACTTTCAG TATGCTAATG CTTGCACCTA 100
<210> 2994
<211> 100
<212> DNA
<213> Escherichia coli K-12 MG1655
<220> yigg b3819
<222> 187-286
<223> yigg b3819 U00096 complement(4000425..4000841)
<313> 2994: 225-244
<400> 2994
GTACTCCATT ATCTCGTCAT CAACATGAAT TGCCAGCGAC TCCGTGATAG 50
TGGTTTCATC TATATAAAAA CTTATGTTTT TGGTACATTA GCAGTATATA 100
<210> 2995
<211> 100
<212> DNA
<213> Escherichia coli K-12 MG1655
<220> icda b1137
<222> 1131-1230

<223> icda b1137 U00096 1194346..1195596
<313> 2995: 1178-1197
<400> 2995
TAAAGGTATG GAAGGCGCAA TCAACGCGAA AACCGTAACC TATGACTTCG 50
AGCGTCTGAT GGATGGCGCT AAACTGCTGA AATGTTCAGA GTTTGGTGAC 100
<210> 2996
<211> 100
<212> DNA
<213> Escherichia coli K-12 MG1655
<220> icda b1137
<222> 1074-1173
<223> icda b1137 U00096 1194346..1195596
<313> 2996: 1115-1134
<400> 2996
CTCCGCTGAG ATGATGCTGC GCCACATGGG TTGGACCGAA GCGGCTGACT 50
TAATTGTTAA AGGTATGGAA GGCGCAATCA ACGCGAAAAC CGTAACCTAT 100
<210> 2997
<211> 100
<212> DNA
<213> Escherichia coli K-12 MG1655
<220> icda b1137
<222> 1048-1147
<223> icda b1137 U00096 1194346..1195596
<313> 2997: 1083-1102
<400> 2997
AAAGTAAATC CTGGCTCTAT TATTCTCTCC GCTGAGATGA TGCTGCGCCA 50
CATGGGTTGG ACCGAAGCGG CTGACTTAAT TGTTAAAGGT ATGGAAGGCG 100
<210> 2998
<211> 100
<212> DNA
<213> Escherichia coli K-12 MG1655
<220> yigi b3821
<222> 387-486
<223> yigi b3821 U00096 complement(4001841..4002326)
<313> 2998: 443-462
<400> 2998
GTTGCGTGCA GGCAATAAAG TCGCCGTCGC CCGCGTTGAA TTACACAATG 50
AAGAACAGCT TTATATTGCC AGTGCCACCG CCACCTATAT GGTAGGTTGA 100
<210> 2999
<211> 100
<212> DNA
<213> Escherichia coli K-12 MG1655
<220> yigi b3821
<222> 360-459
<223> yigi b3821 U00096 complement(4001841..4002326)
<313> 2999: 409-428
<400> 2999
CGAGCGTTTT ACTGCTACTA GTAGCCTGTT GCGTGCAGGC AATAAAGTCG 50
CCGTCGCCCG CGTTGAATTA CACAATGAAG AACAGCTTTA TATTGCCAGT 100
<210> 3000
<211> 100
<212> DNA
<213> Escherichia coli K-12 MG1655
<220> yigi b3821
<222> 294-393
<223> yigi b3821 U00096 complement(4001841..4002326)
<313> 3000: 321-340
<400> 3000
ACGCCAGCGG CTATCGCGGA TGGGGACCAT TGATCTTCGC GTTGATTATC 50
TGCGCCCAGG CAGGGGCGAG CGTTTTACTG CTACTAGTAG CCTGTTGCGT 100
<210> 3001
<211> 100
<212> DNA
<213> Escherichia coli K-12 MG1655
<220> yigj b3824
<222> 266-365
<223> yigj b3824 U00096 4005616..4005984
<313> 3001: 312-331
<400> 3001
TGCCGCAAAT GCGCCGTGGT TATCAACGTC TGGCGAAGTG GATTGATGGT 50
TTTGCCGGGG CGTTATTTGC CGGATTTGGC ATTCATTTGA TTATTTCGCG 100

<210> 3002
<211> 100
<212> DNA
<213> Escherichia coli K-12 MG1655
<220> yigj b3824
<222> 207-306
<223> yigj b3824 U00096 4005616..4005984
<313> 3002: 260-279
<400> 3002
TGCGCTGATC ATTGTCGAAA CGCTGGCGTG GTTTACCGTC GTTGCCAGCC 50
TGTTTGCCCT GCCGCAAATG CGCCGTGGTT ATCAACGTCT GGCGAAGTGG 100
<210> 3003
<211> 100
<212> DNA
<213> Escherichia coli K-12 MG1655
<220> yigj b3824
<222> 168-267
<223> yigj b3824 U00096 4005616..4005984
<313> 3003: 205-224
<400> 3003
CGGTGATAAC GTTGGCACTA CCGCGCGCTG GGGCATTTTT GCGCTGATCA 50
TTGTCGAAAC GCTGGCGTGG TTTACCGTCG TTGCCAGCCT GTTTGCCCTG 100
<210> 3004
<211> 100
<212> DNA
<213> Escherichia coli K-12 MG1655
<220> yigk b3825
<222> 307-406
<223> yigk b3825 U00096 complement(4006046..4006462)
<313> 3004: 355-374
<400> 3004
CGGATTGCTC TATGGATTAA AGGACCAAAG CAGATGAAGG CGCTGAATAA 50
GATTTTCGGC TCGTTGTTTA TGCTGGTGGG AGCGCTGTTA GCATCGGCGA 100
<210> 3005
<211> 100
<212> DNA
<213> Escherichia coli K-12 MG1655
<220> yigk b3825
<222> 213-312
<223> yigk b3825 U00096 complement(4006046..4006462)
<313> 3005: 260-279
<400> 3005
GCAACAGCCG CAACTGATGC AGTATATCGT GCTCGGCGTC ACCACTATTG 50
TGGTCGATAT TATTGTGATG ATCGGTTACG CCACCCTTGC TCAACGGATT 100
<210> 3006
<211> 100
<212> DNA
<213> Escherichia coli K-12 MG1655
<220> yigk b3825
<222> 153-252
<223> yigk b3825 U00096 complement(4006046..4006462)
<313> 3006: 205-224
<400> 3006
TCTCACCAAT CCCAAAAGTA TTGTGTTTCT GGCGGCGCTA TTTCCGCAAT 50
TCATCATGCC GCAACAGCCG CAACTGATGC AGTATATCGT GCTCGGCGTC 100
<210> 3007
<211> 100
<212> DNA
<213> Escherichia coli K-12 MG1655
<220> yigl b3827
<222> 417-516
<223> yigl b3827 U00096 4007918..4008433
<313> 3007: 459-478
<400> 3007
TACCTTAACC TGTCTGGAAG TGATGGCGGG CGGCGTTTCA AAGGCCATGC 50
GCTGGAAGGC TGGCGAACTG GCTACAGCTG AAGGATTGAT TGCGTTGTGA 100
<210> 3008
<211> 100
<212> DNA
<213> Escherichia coli K-12 MG1655
<220> yigl b3827

<222> 380-479
<223> yigl b3827 U00096 4007918..4008433
<313> 3008: 408-427
<400> 3008
ACGCTCGTTG GGGCGATCGC GTCAACGTCA GTTTCTCTAC CTTAACCTGT  50
CTGGAAGTGA TGGCGGGCGG CGTTTCAAAG GCCATGCGCT GGAAGGCTGG  100
<210> 3009
<211> 100
<212> DNA
<213> Escherichia coli K-12 MG1655
<220> yigl b3827
<222> 328-427
<223> yigl b3827 U00096 4007918..4008433
<313> 3009: 357-376
<400> 3009
TTCTTCACCT GCGATTCCCA TGAACAACTG CTGCCGCTGG AGCAGGCGAT  50
TAACGCTCGT TGGGGCGATC GCGTCAACGT CAGTTTCTCT ACCTTAACCT  100
<210> 3010
<211> 100
<212> DNA
<213> Escherichia coli K-12 MG1655
<220> yigm b3828
<222> 801-900
<223> yigm b3828 U00096 4008666..4009565
<313> 3010: 843-862
<400> 3010
GGTGATCCTG GCCTCACTGT GGGTGCATCG TAAGTGGGTC GCTCCGCGCT  50
CTTCACAAAC GGCAGATGAT CGCAGGCGTG ATTGCGCGCT GAGCGAATAA  100
<210> 3011
<211> 100
<212> DNA
<213> Escherichia coli K-12 MG1655
<220> yigm b3828
<222> 724-823
<223> yigm b3828 U00096 4008666..4009565
<313> 3011: 739-758
<400> 3011
GTTCCGGCAG GGCTGCTGGT AAACCTGGCT ATCTGGCACC AACAGCCGCA  50
CTGGCCAACG TTTATTACAG GCGCGCTGGT GATCCTGGCC TCACTGTGGG  100
<210> 3012
<211> 100
<212> DNA
<213> Escherichia coli K-12 MG1655
<220> yigm b3828
<222> 658-757
<223> yigm b3828 U00096 4008666..4009565
<313> 3012: 711-730
<400> 3012
TTTATGTGGA ACTACGGCGC GACGCAGGTG GACGCCGGAA CGCTGGGCAT  50
TATGAATAAT ATGCACGTTC CGGCAGGGCT GCTGGTAAAC CTGGCTATCT  100
<210> 3013
<211> 100
<212> DNA
<213> Escherichia coli K-12 MG1655
<220> b2981 b2982
<222> 1074-1173
<223> b2981 b2982 U00096 complement(3127058..3128230)
<313> 3013: 1117-1136
<400> 3013
GTCATGGATC CTCGAAACCA ATACAGGCAT GAATAACATG CTGGAACGCA  50
TTGGGGCGGA GCCGTACAAG CGTTACCGAT TGTATGAAAA GCAGATTTAA  100
<210> 3014
<211> 100
<212> DNA
<213> Escherichia coli K-12 MG1655
<220> b2981 b2982
<222> 1024-1123
<223> b2981 b2982 U00096 complement(3127058..3128230)
<313> 3014: 1056-1075
<400> 3014
GAAGCCTTAC GCGATCCGTT TGCCCGCCGG AAGATCGATG CGCTGGAGAT  50

GTCATGGATC CTCGAAACCA ATACAGGCAT GAATAACATG CTGGAACGCA 100
<210> 3015
<211> 100
<212> DNA
<213> Escherichia coli K-12 MG1655
<220> b2981 b2982
<222> 975-1074
<223> b2981 b2982 U00096 complement(3127058. 3128230)
<313> 3015: 1003-1022
<400> 3015
CGAGTATCAG TTCAGCCGCA TCGGCCCGGT GATTGCGCTG TTATTGATTG 50
AAGCCTTACG CGATCCGTTT GCCCGCCGGA AGATCGATGC GCTGGAGATG 100
<210> 3016
<211> 100
<212> DNA
<213> Escherichia coli K-12 MG1655
<220> yign b3833
<222> 1329-1428
<223> yign b3833 U00096 4014920..4016347
<313> 3016: 1375-1394
<400> 3016
GGTGAGCCAG GATGAAGAGT ATCGACTTCG GTCGGTTCCG GAGCAGCCGA 50
ATGATGAAGC TTATCAACGC GATGATGAAT ATAATCAGCA GTCGCGCTAG 100
<210> 3017
<211> 100
<212> DNA
<213> Escherichia coli K-12 MG1655
<220> yign b3833
<222> 1302-1401
<223> yign b3833 U00096 4014920..4016347
<313> 3017: 1335-1354
<400> 3017
GATTAATCCG GATTTGGCTG AACAGGCGGT GAGCCAGGAT GAAGAGTATC 50
GACTTCGGTC GGTTCCGGAG CAGCCGAATG ATGAAGCTTA TCAACGCGAT 100
<210> 3018
<211> 100
<212> DNA
<213> Escherichia coli K-12 MG1655
<220> yign b3833
<222> 1168-1267
<223> yign b3833 U00096 4014920..4016347
<313> 3018: 1231-1250
<400> 3018
TCCGCGATTG GTCAAAGTCT CGACAAAGCG CAGGATAATT ATCGGCAGGC 50
AATGAAAAAA CTCTCTTCAG GGCGCGGAAA TGTGCTGGCG CAGGCAGAAG 100
<210> 3019
<211> 100
<212> DNA
<213> Escherichia coli K-12 MG1655
<220> yigp b3835
<222> 507-606
<223> yigp b3835 U00096 4017211..4017816
<313> 3019: 571-590
<400> 3019
CGGTCCGCTT GAAGTGGCCT GGTTTGCGGA AGAGACGGCT GCCGTCGAGC 50
GTGCTGTTGA TGCCCTGACC AAACGGCTGG AAAAACTGGA GGCTAAATGA 100
<210> 3020
<211> 100
<212> DNA
<213> Escherichia coli K-12 MG1655
<220> yigp b3835
<222> 478-577
<223> yigp b3835 U00096 4017211..4017816
<313> 3020: 508-527
<400> 3020
GCCATTACTG AAGAGTGGCG TATGGCACCC GGTCCGCTTG AAGTGGCCTG 50
GTTTGCGGAA GAGACGGCTG CCGTCGAGCG TGCTGTTGAT GCCCTGACCA 100
<210> 3021
<211> 100
<212> DNA
<213> Escherichia coli K-12 MG1655

<220> yigp b3835
<222> 402-501
<223> yigp b3835 U00096 4017211..4017816
<313> 3021: 432-451
<400> 3021
AATCAGCAAA GCCATGCGCG GAGGCGCAAA GTTCCTGCAT CACGGCATTA 50
AGCGCCAGCA ACGTTATGTG GCGGAAGCCA TTACTGAAGA GTGGCGTATG 100
<210> 3022
<211> 100
<212> DNA
<213> Escherichia coli K-12 MG1655
<220> yigr yigR
<222> 1426-1525
<223> yigr yigR U00096 4017813..4019453
<313> 3022: 1461-1480
<400> 3022
AAGTATTTAC AGCACAGTGT TGATAAGATT GCCCGCGAGC TTCAGTCAAA 50
TCATGTACGT CAGGGACAAT CGCGTTATTT TCTCGGAATT GGCGCTACGT 100
<210> 3023
<211> 100
<212> DNA
<213> Escherichia coli K-12 MG1655
<220> yigr yigR
<222> 1357-1456
<223> yigr yigR U00096 4017813..4019453
<313> 3023: 1423-1442
<400> 3023
AAAGCGCCGT TCTGGGTCGA AAAAATGCCA GAACTGCCTG AATTGGTTTA 50
CGACAGTTTG CGCCAGGGCA AGTATTTACA GCACAGTGTT GATAAGATTG 100
<210> 3024
<211> 100
<212> DNA
<213> Escherichia coli K-12 MG1655
<220> yigr yigR
<222> 1311-1410
<223> yigr yigR U00096 4017813..4019453
<313> 3024: 1364-1383
<400> 3024
TAAAGATCAG GTCGGTATTC CTGCGCTGGT GAGAGCATTT AAAGAAAAAG 50
CGCCGTTCTG GGTCGAAAAA ATGCCAGAAC TGCCTGAATT GGTTTACGAC 100
<210> 3025
<211> 100
<212> DNA
<213> Escherichia coli K-12 MG1655
<220> yigu b3840
<222> 678-777
<223> yigu b3840 U00096 4020325..4021101
<313> 3025: 740-759
<400> 3025
TGAAATCGGT GTCTTCTTCT CACGCTTTTA CGTTGGTAAA GGGCGAAATC 50
GGGAAGAGGA AAACGACGCT GAAGCAGAAA GCGAAAAAAC TGAAGAATAA 100
<210> 3026
<211> 100
<212> DNA
<213> Escherichia coli K-12 MG1655
<220> yigu b3840
<222> 613-712
<223> yigu b3840 U00096 4020325..4021101
<313> 3026: 661-680
<400> 3026
ATGTTGCTGA CGCCGCCGGA TGTCTTCTCG CAAACGCTGT TGGCGATCCC 50
GATGTACTGT CTGTTTGAAA TCGGTGTCTT CTTCTCACGC TTTTACGTTG 100
<210> 3027
<211> 100
<212> DNA
<213> Escherichia coli K-12 MG1655
<220> yigu b3840
<222> 600-699
<223> yigu b3840 U00096 4020325..4021101
<313> 3027: 629-648
<400> 3027

ATTCGTTGTC GGGATGTTGC TGACGCCGCC GGATGTCTTC TCGCAAACGC 50
TGTTGGCGAT CCCGATGTAC TGTCTGTTTG AAATCGGTGT CTTCTTCTCA 100
<210> 3028
<211> 100
<212> DNA
<213> Escherichia coli K-12 MG1655
<220> b2989 b2990
<222> 766-865
<223> b2989 b2990 U00096 3136694..3137608
<313> 3028: 824-843
<400> 3028
CAACGCTGGG CGAAAGAAGT AGGCGAACGT CCGGCGGTGA AACGTGGGCG 50
TATTGTTAAC CGCACCAACG GACCGCTGAA TGAGCAGTTG CATGAGCGCC 100
<210> 3029
<211> 100
<212> DNA
<213> Escherichia coli K-12 MG1655
<220> b2989 b2990
<222> 728-827
<223> b2989 b2990 U00096 3136694..3137608
<313> 3029: 777-796
<400> 3029
CCGCTGAGTT TCTTGATGCG GGCAGTTATA AGCATGTACA ACGCTGGGCG 50
AAAGAAGTAG GCGAACGTCC GGCGGTGAAA CGTGGGCGTA TTGTTAACCG 100
<210> 3030
<211> 100
<212> DNA
<213> Escherichia coli K-12 MG1655
<220> b2989 b2990
<222> 719-818
<223> b2989 b2990 U00096 3136694..3137608
<313> 3030: 738-757
<400> 3030
TGTATGATGC CGCTGAGTTT CTTGATGCGG GCAGTTATAA GCATGTACAA 50
CGCTGGGCGA AAGAAGTAGG CGAACGTCCG GCGGTGAAAC GTGGGCGTAT 100
<210> 3031
<211> 100
<212> DNA
<213> Escherichia coli K-12 MG1655
<220> yigz b3849
<222> 519-618
<223> yigz b3849 U00096 4030079..4030696
<313> 3031: 557-576
<400> 3031
GCTGCGGGTG GCGCTTCCGG CGGCGAAAGT GGCTGAATTT TCCGCAAAGC 50
TGGCGGATTT TAGCCGTGGT TCATTGCAAT TGTTAGCGAT TGAAGAATAA 100
<210> 3032
<211> 100
<212> DNA
<213> Escherichia coli K-12 MG1655
<220> yigz b3849
<222> 450-549
<223> yigz b3849 U00096 4030079..4030696
<313> 3032: 510-529
<400> 3032
CGGCATTGAA GCGTTGCTGG GGCAGTGTGA CGGCAAAATT ATCAACAGTG 50
ATTATCAGGC ATTCGTTCTG CTGCGGGTGG CGCTTCCGGC GGCGAAAGTG 100
<210> 3033
<211> 100
<212> DNA
<213> Escherichia coli K-12 MG1655
<220> yigz b3849
<222> 381-480
<223> yigz b3849 U00096 4030079..4030696
<313> 3033: 400-419
<400> 3033
CCAGCTAACG ACCCAACGCA AGACGCCATT AACCGAATAT ACTTTGCAAT 50
GTGAATATCA TCAGTTAACC GGCATTGAAG CGTTGCTGGG GCAGTGTGAC 100
<210> 3034
<211> 100
<212> DNA

<213> Escherichia coli K-12 MG1655
<220> b0011 b0012
<222> 615-714
<223> b0011 b0012 U00096 complement(10643..11356)
<313> 3034: 684-703
<400> 3034
TGGGGTTAAA GCGGTCAGCG GCAGCGCCTA TCGCGTGACG ATTCCAGCCG  50
TACTGCAAAT CGCCTGCCTG CGCCGGATGG TTAGCGCCAC TCAGGTCTGA  100
<210> 3035
<211> 100
<212> DNA
<213> Escherichia coli K-12 MG1655
<220> b0011 b0012
<222> 576-675
<223> b0011 b0012 U00096 complement(10643..11356)
<313> 3035: 611-630
<400> 3035
CGGCGCGGGG CTGGGAGGCC CTGTAGGTGC GGCACTAAAT GGGGTTAAAG  50
CGGTCAGCGG CAGCGCCTAT CGCGTGACGA TTCCAGCCGT ACTGCAAATC  100
<210> 3036
<211> 100
<212> DNA
<213> Escherichia coli K-12 MG1655
<220> b0011 b0012
<222> 517-616
<223> b0011 b0012 U00096 complement(10643..11356)
<313> 3036: 549-568
<400> 3036
CTGACCCGCA TTTTACGCAC CCACGCAGCA ATGAGCGTAC TTGGGCATGG  50
TTTGCTGCGC GGCGCGGGGC TGGGAGGCCC TGTAGGTGCG GCACTAAATG  100
<210> 3037
<211> 100
<212> DNA
<213> Escherichia coli K-12 MG1655
<220> gppa b3780
<222> 1386-1485
<223> gppa b3780 U00096 complement(3960360..3961844)
<313> 3037: 1439-1458
<400> 3037
TCCGCAAGGT TGGCTAACCC AACATCCGCT GGGTAAAGAG ATTATTGCTC  50
AGGAAAGCCA GTGGCAGAGC TATGTCCACT GGCCGCTGGA AGTGCATTAA  100
<210> 3038
<211> 100
<212> DNA
<213> Escherichia coli K-12 MG1655
<220> gppa b3780
<222> 1327-1426
<223> gppa b3780 U00096 complement(3960360..3961844)
<313> 3038: 1357-1376
<400> 3038
GACGATCTCG TGCCAGAGAT GACATTACAG GCTAACCATG AACTGTTGAC  50
CTTGACGCTT CCGCAAGGTT GGCTAACCCA ACATCCGCTG GGTAAAGAGA  100
<210> 3039
<211> 100
<212> DNA
<213> Escherichia coli K-12 MG1655
<220> gppa b3780
<222> 1211-1310
<223> gppa b3780 U00096 complement(3960360..3961844)
<313> 3039: 1227-1246
<400> 3039
AGACTAATCC GGTCGATCTC TCATCGCTGC ATCAGCAAAA TGCCGTACCG  50
CCGCGGCTCG CAGAACAACT CTGCCGTTTA CTACGCCTGG CCATCATTTT  100
<210> 3040
<211> 100
<212> DNA
<213> Escherichia coli K-12 MG1655
<220> yiha b3866
<222> 501-600
<223> yiha b3866 U00096 complement(4047713..4048312)
<313> 3040: 577-596

<400> 3040
TTCTTCGTTG AAGAAACAAG GCGTGGACAA GCTGCGGCAG AAACTGGATA  50
CCTGGTTTAG CGAGATGCAG CCTGTAGAAG AAACGCAGGA CGGCGAATAA  100
<210> 3041
<211> 100
<212> DNA
<213> Escherichia coli K-12 MG1655
<220> yiha b3866
<222> 441-540
<223> yiha b3866 U00096 complement(4047713..4048312)
<313> 3041: 463-482
<400> 3041
ATTGAATATG GTGCGTGAAG CTGTACTGGC GTTTAACGGT GATGTGCAGG  50
TTGAAACGTT TTCTTCGTTG AAGAAACAAG GCGTGGACAA GCTGCGGCAG  100
<210> 3042
<211> 100
<212> DNA
<213> Escherichia coli K-12 MG1655
<220> yiha b3866
<222> 379-478
<223> yiha b3866 U00096 complement(4047713..4048312)
<313> 3042: 429-448
<400> 3042
ATCGCCGTTC TGGTGCTGCT GACCAAAGCG GACAAACTGG CAAGCGGCGC  50
ACGTAAAGCG CAATTGAATA TGGTGCGTGA AGCTGTACTG GCGTTTAACG  100
<210> 3043
<211> 100
<212> DNA
<213> Escherichia coli K-12 MG1655
<220> yihd b3859
<222> 144-243
<223> yihd b3859 U00096 4039649..4039918
<313> 3043: 167-186
<400> 3043
CATTCTGATC TATCACCTGA AAATGCGTGA TTCCGCGAAA GATGCGGTGA  50
TCCCGGGTTT GCAGAAAGAT TATGAAGAAG ACTTCAAAAC GGCGCTGTTA  100
<210> 3044
<211> 100
<212> DNA
<213> Escherichia coli K-12 MG1655
<220> yihd b3859
<222> 105-204
<223> yihd b3859 U00096 4039649..4039918
<313> 3044: 120-139
<400> 3044
GTCAGGTTTT GACGGCGAAC TGGCGGATTT GACGGATGAC ATTCTGATCT  50
ATCACCTGAA AATGCGTGAT TCCGCGAAAG ATGCGGTGAT CCCGGGTTTG  100
<210> 3045
<211> 100
<212> DNA
<213> Escherichia coli K-12 MG1655
<220> yihd b3859
<222> 31-130
<223> yihd b3859 U00096 4039649..4039918
<313> 3045: 47-66
<400> 3045
GAACTCCTCC AGCCAGCCTG GCAAAAAGAG CCAGACCTTA ACCTGCTGCA  50
ATTTTTGCAG AAACTGGCGA AAGAGTCAGG TTTTGACGGC GAACTGGCGG  100
<210> 3046
<211> 100
<212> DNA
<213> Escherichia coli K-12 MG1655
<220> tpia b3920
<222> 668-767
<223> tpia b3920 U00096 complement(4108320..4109087)
<313> 3046: 717-736
<400> 3046
CTCAGCCGGA TATCGACGGC GCGCTGGTTG GTGGTGCTTC TCTGAAAGCT  50
GACGCCTTCG CAGTAATCGT TAAAGCTGCA GAAGCGGCTA AACAGGCTTA  100
<210> 3047
<211> 100

<212> DNA
<213> Escherichia coli K-12 MG1655
<220> tpia b3920
<222> 552-651
<223> tpia b3920 U00096 complement(4108320..4109087)
<313> 3047: 591-610
<400> 3047
GGCTGTTCAC AAATTCATCC GTGACCACAT CGCTAAAGTT GACGCTAACA 50
TCGCTGAACA AGTGATCATT CAGTACGGCG GCTCTGTAAA CGCGTCTAAC 100
<210> 3048
<211> 100
<212> DNA
<213> Escherichia coli K-12 MG1655
<220> tpia b3920
<222> 489-588
<223> tpia b3920 U00096 complement(4108320..4109087)
<313> 3048: 553-572
<400> 3048
TATCGCTTAC GAACCTGTAT GGGCAATCGG TACTGGCAAA TCTGCAACTC 50
CGGCTCAGGC ACAGGCTGTT CACAAATTCA TCCGTGACCA CATCGCTAAA 100
<210> 3049
<211> 100
<212> DNA
<213> Escherichia coli K-12 MG1655
<220> yihe b3860
<222> 869-968
<223> yihe b3860 U00096 4039996..4040982
<313> 3049: 907-926
<400> 3049
TCCCGAAAAA TTTCCCGTGG TTAACCGGGG AAGATTACTG GCTGCGACAG 50
ACGGCGACTT TTATAGAACA GGCAAAAGTT CTACAAGAAC CCCCTTTGCA 100
<210> 3050
<211> 100
<212> DNA
<213> Escherichia coli K-12 MG1655
<220> yihe b3860
<222> 823-922
<223> yihe b3860 U00096 4039996..4040982
<313> 3050: 866-885
<400> 3050
GTTTATTATC TTGCCTGGCT AATGCGGCGT TGGGCTGATC CCGCGTTCCC 50
GAAAAATTTC CCGTGGTTAA CCGGGGAAGA TTACTGGCTG CGACAGACGG 100
<210> 3051
<211> 100
<212> DNA
<213> Escherichia coli K-12 MG1655
<220> yihe b3860
<222> 768-867
<223> yihe b3860 U00096 4039996..4040982
<313> 3051: 819-838
<400> 3051
CGAGTTCGAC ACCGCTGAAA TCGGACTGAT TGAACCTTTA CGCGCCATGC 50
GTTTGGTTTA TTATCTTGCC TGGCTAATGC GGCGTTGGGC TGATCCCGCG 100
<210> 3052
<211> 100
<212> DNA
<213> Escherichia coli K-12 MG1655
<220> yihf b3862
<222> 1374-1473
<223> yihf b3862 U00096 4041737..4043209
<313> 3052: 1434-1453
<400> 3052
CTCGCTGCAG CTGCGTTATA CACCGGGTAA AGTTGTTTTT AACGGACAGG 50
AGATGAGCGA AGAAGAATTT ATGTCTCGTG CCGGACGTTT TGTTCATTAA 100
<210> 3053
<211> 100
<212> DNA
<213> Escherichia coli K-12 MG1655
<220> yihf b3862
<222> 1337-1436
<223> yihf b3862 U00096 4041737..4043209

<313> 3053: 1390-1409
<400> 3053
AGATGTTTCA GTTAATCACG ATTGACAACA ATACCGCCTC GCTGCAGCTG 50
CGTTATACAC CGGGTAAAGT TGTTTTTAAC GGACAGGAGA TGAGCGAAGA 100
<210> 3054
<211> 100
<212> DNA
<213> Escherichia coli K-12 MG1655
<220> yihf b3862
<222> 1237-1336
<223> yihf b3862 U00096 4041737..4043209
<313> 3054: 1284-1303
<400> 3054
ACAGAAACCG CAAAACAGCT TAATTTATCT GAAGGAATGG ATGCGGAAAA 50
AGCGCAAAAG CAGGCTGATA AACAAATCAG CGGGATGATG ACATTAGGTC 100
<210> 3055
<211> 100
<212> DNA
<213> Escherichia coli K-12 MG1655
<220> yihg b3863
<222> 830-929
<223> yihg b3863 U00096 complement(4043250..4044182)
<313> 3055: 880-899
<400> 3055
ACAAAAGCTT TAAGCGCCAT TTTCAGCAAT GGCTGAACTC ATTGTGGCAG 50
GAAAAAGACC GGCTTCTGAC GTCTCTCATG TCATCACAGC GTCAGAATAA 100
<210> 3056
<211> 100
<212> DNA
<213> Escherichia coli K-12 MG1655
<220> yihg b3863
<222> 751-850
<223> yihg b3863 U00096 complement(4043250..4044182)
<313> 3056: 802-821
<400> 3056
AGCGGTAAAC TGACGCGGAT TGTCGTACAT GTGGATTTAC AGCCTATTGC 50
CGATGAGTTA CATGGCGACT ACATCAATGA CAAAAGCTTT AAGCGCCATT 100
<210> 3057
<211> 100
<212> DNA
<213> Escherichia coli K-12 MG1655
<220> yihg b3863
<222> 692-791
<223> yihg b3863 U00096 complement(4043250..4044182)
<313> 3057: 753-772
<400> 3057
TGCTTAATGT TACGCTGTGT TATCCGGACA ATAACCGTCA GCCGTTCTTC 50
GATATGTTAA GCGGTAAACT GACGCGGATT GTCGTACATG TGGATTTACA 100
<210> 3058
<211> 100
<212> DNA
<213> Escherichia coli K-12 MG1655
<220> yihi b3867
<222> 408-507
<223> yihi b3867 U00096 4048927..4049436
<313> 3058: 447-466
<400> 3058
TGATGAGTTG ATGCAGAAAC TCGGCCTCTC TTATGACGAT GACGAAGAAG 50
AGGAAGAAGA CGAGAAGCAA GAAGACATGA TGCGTCTGCT GCGGGGCAAC 100
<210> 3059
<211> 100
<212> DNA
<213> Escherichia coli K-12 MG1655
<220> yihi b3867
<222> 372-471
<223> yihi b3867 U00096 4048927..4049436
<313> 3059: 402-421
<400> 3059
AGAGCAATCC TGGGTGGATG CTAAACTGGA TCGTATTGAT GAGTTGATGC 50
AGAAACTCGG CCTCTCTTAT GACGATGACG AAGAAGAGGA AGAAGACGAG 100
<210> 3060

<211> 100
<212> DNA
<213> Escherichia coli K-12 MG1655
<220> yihi b3867
<222> 322-421
<223> yihi b3867 U00096 4048927..4049436
<313> 3060: 365-384
<400> 3060
CTGGATGCGC TGCTGGAACG TCTGGAAGCA GGCGAAACCC TGAGTGCCGA 50
AGAGCAATCC TGGGTGGATG CTAAACTGGA TCGTATTGAT GAGTTGATGC 100
<210> 3061
<211> 100
<212> DNA
<213> Escherichia coli K-12 MG1655
<220> yihk b3872
<222> 1677-1776
<223> yihk b3872 U00096 4055987..4057762
<313> 3061: 1743-1762
<400> 3061
GCCTATCCGC ATGACTCTGG AACAAGCTCT GGAGTTCATC GATGATGACG 50
AACTGGTAGA AGTGACTCCG ACCTCTATCC GTATTCGTAA ACGTCACTGA 100
<210> 3062
<211> 100
<212> DNA
<213> Escherichia coli K-12 MG1655
<220> yihk b3872
<222> 1625-1724
<223> yihk b3872 U00096 4055987..4057762
<313> 3062: 1682-1701
<400> 3062
AACTGACCAA CATGCGTGCT TCCGGTACTG ACGAAGCCGT TGTTCTGGTT 50
CCGCCTATCC GCATGACTCT GGAACAAGCT CTGGAGTTCA TCGATGATGA 100
<210> 3063
<211> 100
<212> DNA
<213> Escherichia coli K-12 MG1655
<220> yihk b3872
<222> 1553-1652
<223> yihk b3872 U00096 4055987..4057762
<313> 3063: 1584-1603
<400> 3063
TTTACGAAGG TCAGATTATC GGTATTCATA GCCGCTCTAA CGACCTGACT 50
GTAAACTGCC TGACCGGTAA GAAACTGACC AACATGCGTG CTTCCGGTAC 100
<210> 3064
<211> 100
<212> DNA
<213> Escherichia coli K-12 MG1655
<220> yihl b3873
<222> 590-689
<223> yihl b3873 U00096 4058026..4058736
<313> 3064: 615-634
<400> 3064
GTTCTGCGGG TATGCCAGCG ATCTTAATTG AAAAGCATCG CGCCGACCAG 50
CAAGGCAATA TTGTCCAAAT AGATATTGAA TATTGGCGAT TTGAGGCCGT 100
<210> 3065
<211> 100
<212> DNA
<213> Escherichia coli K-12 MG1655
<220> yihl b3873
<222> 524-623
<223> yihl b3873 U00096 4058026..4058736
<313> 3065: 556-575
<400> 3065
CGGTAGTAAA AAAATTGATT TTCAAACCCG TCAGAATGCC GGGCGATATC 50
AGCAAGTATC TTGGCGGTTC TGCGGGTATG CCAGCGATCT TAATTGAAAA 100
<210> 3066
<211> 100
<212> DNA
<213> Escherichia coli K-12 MG1655
<220> yihl b3873
<222> 413-512

<223> yihl b3873 U00096 4058026..4058736
<313> 3066: 463-482
<400> 3066
AAGTTTTCTA TCACGAAACA TATATTAATC CTGAAGTTGC TCCGGGCTTT  50
ATTGAACAAC TTGAAAACCA CTCATTTTCT GCAGTCTGGG AAAAGTGCTA  100
<210> 3067
<211> 100
<212> DNA
<213> Escherichia coli K-12 MG1655
<220> thya b2828
<222> 696-795
<223> thya b2828 U00096 complement(2962383..2963177)
<313> 3067: 754-773
<400> 3067
CAAACGTAAA CCCGAATCCA TCTTCGACTA CCGTTTCGAA GACTTTGAGA  50
TTGAAGGCTA CGATCCGCAT CCGGGCATTA AAGCGCCGGT GGCTATCTAA  100
<210> 3068
<211> 100
<212> DNA
<213> Escherichia coli K-12 MG1655
<220> thya b2828
<222> 663-762
<223> thya b2828 U00096 complement(2962383..2963177)
<313> 3068: 693-712
<400> 3068
CCGCGAACCG CGTCCGCTGC CGAAGTTGAT TATCAAACGT AAACCCGAAT  50
CCATCTTCGA CTACCGTTTC GAAGACTTTG AGATTGAAGG CTACGATCCG  100
<210> 3069
<211> 100
<212> DNA
<213> Escherichia coli K-12 MG1655
<220> thya b2828
<222> 592-691
<223> thya b2828 U00096 complement(2962383..2963177)
<313> 3069: 646-665
<400> 3069
GATTTTGTCT GGACCGGTGG CGACACGCAT CTGTACAGCA ACCATATGGA  50
TCAAACTCAT CTGCAATTAA GCCGCGAACC GCGTCCGCTG CCGAAGTTGA  100
<210> 3070
<211> 100
<212> DNA
<213> Escherichia coli K-12 MG1655
<220> yihm b3874
<222> 807-906
<223> yihm b3874 U00096 4058744..4059724
<313> 3070: 851-870
<400> 3070
AGATGATAAT CCGTGGATCC CTTATCGCCA GATGAGTGAA ACACCATTGC  50
CAGAAAATCA TTTACTGGAT GCGCGGTTAC GTAAAGAAAA AGAAGATGCA  100
<210> 3071
<211> 100
<212> DNA
<213> Escherichia coli K-12 MG1655
<220> yihm b3874
<222> 706-805
<223> yihm b3874 U00096 4058744..4059724
<313> 3071: 746-765
<400> 3071
ACGCACCTTA TCTGCCTGGG TGATGATGAG CCGCAGGTGA CGGCATATGG  50
CCTGGAAGAA GAGGTTGATT ACTATGCGCC GGCGTTCCGC TTTGAAGACG  100
<210> 3072
<211> 100
<212> DNA
<213> Escherichia coli K-12 MG1655
<220> yihm b3874
<222> 644-743
<223> yihm b3874 U00096 4058744..4059724
<313> 3072: 677-696
<400> 3072
GTGGCCTGGG TCATAAAGCC TGTATTTCAG GTCAGGGTGA TATGCCGTTC  50
AAAGCGTTAT TAACGCACCT TATCTGCCTG GGTGATGATG AGCCGCAGGT  100

<210> 3073
<211> 100
<212> DNA
<213> Escherichia coli K-12 MG1655
<220> yihn b3875
<222> 1167-1266
<223> yihn b3875 U00096 4059826..4061091
<313> 3073: 1185-1204
<400> 3073
CATTATGGCC TGCTTCGCAT TCAGTGGTGC GGTGGTTTCC GTAATGCTGG 50
TTAAGCGTAT TAGCCAACGT AAGAAAGAGA TGCTGGCGGC TGAAGCTTAA 100
<210> 3074
<211> 100
<212> DNA
<213> Escherichia coli K-12 MG1655
<220> yihn b3875
<222> 1124-1223
<223> yihn b3875 U00096 4059826..4061091
<313> 3074: 1154-1173
<400> 3074
TGGATTTAAA TCCGGGGATT ATTGGCTACA AAATCGTGTT TGGCATTATG 50
GCCTGCTTCG CATTCAGTGG TGCGGTGGTT TCCGTAATGC TGGTTAAGCG 100
<210> 3075
<211> 100
<212> DNA
<213> Escherichia coli K-12 MG1655
<220> yihn b3875
<222> 1071-1170
<223> yihn b3875 U00096 4059826..4061091
<313> 3075: 1104-1123
<400> 3075
CTTTATTGGT TACGCTCCGG CGATGTTCTG CTTCAGTCTG TATGGCTACA 50
TTCTGGATTT AAATCCGGGG ATTATTGGCT ACAAAATCGT GTTTGGCATT 100
<210> 3076
<211> 100
<212> DNA
<213> Escherichia coli K-12 MG1655
<220> yiho b3877
<222> 1341-1440
<223> yiho b3877 U00096 complement(4061889..4063352)
<313> 3076: 1377-1396
<400> 3076
TGAGGAAATC AACCAACGCA AAAATAAAGA GATGGCGACA GAAGAAAAAA 50
CGGGCTTCCG TAACGTTATA AATGTCAATA GCCGCTATTT CCATCCTGGT 100
<210> 3077
<211> 100
<212> DNA
<213> Escherichia coli K-12 MG1655
<220> yiho b3877
<222> 1223-1322
<223> yiho b3877 U00096 complement(4061889..4063352)
<313> 3077: 1273-1292
<400> 3077
CGACATTACA GGGTTTGCGT CAGCTCATTT TTATCTGGCC TTGTGCACTG 50
GCAATTATTG CTGCATTGAC GATGGGATTC TTTTACACAC TCAATGAAAA 100
<210> 3078
<211> 100
<212> DNA
<213> Escherichia coli K-12 MG1655
<220> yiho b3877
<222> 1169-1268
<223> yiho b3877 U00096 complement(4061889..4063352)
<313> 3078: 1190-1209
<400> 3078
CAGGCATTAT GCTGACGCAA ATTGGTTATG TTCCCAACAT CGCGCAAAGC 50
GATGCGACAT TACAGGGTTT GCGTCAGCTC ATTTTTATCT GGCCTTGTGC 100
<210> 3079
<211> 100
<212> DNA
<213> Escherichia coli K-12 MG1655
<220> yihp b3878

<222> 1308-1407
<223> yihp b3878 U00096 complement(4063389..4064795)
<313> 3079: 1372-1391
<400> 3079
GGTAGTCACC ATTGTGGCGA TGGGTTGCTT CTACAGCCTG AACGAGAAGA 50
TGTATGTCCG CATTGTGGAA GAGATAGAAG CCCGTAAACG CACGGCGTAA 100
<210> 3080
<211> 100
<212> DNA
<213> Escherichia coli K-12 MG1655
<220> yihp b3878
<222> 1281-1380
<223> yihp b3878 U00096 complement(4063389..4064795)
<313> 3080: 1336-1355
<400> 3080
GATCTTCATC TACCCAAGCG CACTGGCGGT AGTCACCATT GTGGCGATGG 50
GTTGCTTCTA CAGCCTGAAC GAGAAGATGT ATGTCCGCAT TGTGGAAGAG 100
<210> 3081
<211> 100
<212> DNA
<213> Escherichia coli K-12 MG1655
<220> yihp b3878
<222> 1261-1360
<223> yihp b3878 U00096 complement(4063389..4064795)
<313> 3081: 1303-1322
<400> 3081
ATTGAAGGGT TACGCCAGTT GATCTTCATC TACCCAAGCG CACTGGCGGT 50
AGTCACCATT GTGGCGATGG GTTGCTTCTA CAGCCTGAAC GAGAAGATGT 100
<210> 3082
<211> 100
<212> DNA
<213> Escherichia coli K-12 MG1655
<220> yihq b3879
<222> 1938-2037
<223> yihq b3879 U00096 complement(4064820..4066856)
<313> 3082: 1976-1995
<400> 3082
CGGGGAAGTT ACCGTTAATG CGCCCATCGG CAAGCCGCCG GTCTTTTATC 50
GCGCCGATAG CGAATGGGCG GCACTGTTCG CGTCGTTAAA AAGCATCTAA 100
<210> 3083
<211> 100
<212> DNA
<213> Escherichia coli K-12 MG1655
<220> yihq b3879
<222> 1856-1955
<223> yihq b3879 U00096 complement(4064820..4066856)
<313> 3083: 1886-1905
<400> 3083
ATGAAGAAGG CCGTAGCGAC TGGACGCTCT ATCTGCCGGA GGATAACTGG 50
GTCCACGCCT GGACGGGTGA AGCGTTCCGG GGCGGGGAAG TTACCGTTAA 100
<210> 3084
<211> 100
<212> DNA
<213> Escherichia coli K-12 MG1655
<220> yihq b3879
<222> 1754-1853
<223> yihq b3879 U00096 complement(4064820..4066856)
<313> 3084: 1811-1830
<400> 3084
CGGTTATGCG CCCGCTGTTC CTGCATTACG AAGACGATGC GCACACTTAC 50
ACCCTGAAAT ATCAGTACCT GTTAGGTCGC GACATTCTGG TCGCTCCGGT 100
<210> 3085
<211> 100
<212> DNA
<213> Escherichia coli K-12 MG1655
<220> yihr b3880
<222> 828-927
<223> yihr b3880 U00096 complement(4067055..4067981)
<313> 3085: 874-893
<400> 3085
TTGCCCGCCT AACGCCTTTA ATTCAGGTAT CGACTTATTA TTACTGGAAT 50

CAGGAAAACC GCACCGCCTG TTTTTTAATA TTTACGGACA GAGAAAATAA 100
<210> 3086
<211> 100
<212> DNA
<213> Escherichia coli K-12 MG1655
<220> yihr b3880
<222> 779-878
<223> yihr b3880 U00096 complement(4067055..4067981)
<313> 3086: 822-841
<400> 3086
ATAGCGGTGA AAAATTACAG CGTCAGGGAC TGGCAGTTGA ACCTATGAGT 50
TGCCCGCCTA ACGCCTTTAA TTCAGGTATC GACTTATTAT TACTGGAATC 100
<210> 3087
<211> 100
<212> DNA
<213> Escherichia coli K-12 MG1655
<220> yihr b3880
<222> 769-868
<223> yihr b3880 U00096 complement(4067055..4067981)
<313> 3087: 782-801
<400> 3087
GTGCAGGTTT ATAGCGGTGA AAAATTACAG CGTCAGGGAC TGGCAGTTGA 50
ACCTATGAGT TGCCCGCCTA ACGCCTTTAA TTCAGGTATC GACTTATTAT 100
<210> 3088
<211> 100
<212> DNA
<213> Escherichia coli K-12 MG1655
<220> yihs b3881
<222> 1112-1211
<223> yihs b3881 U00096 complement(4068095..4069351)
<313> 3088: 1178-1197
<400> 3088
CGGACAATAA GGTCACCACC AAAGTCTGGG ACGGCAAACA GGATATTTAT 50
CACCTGCTGC ATTGCCTGGT GATCCCGCGT ATCCCGTTAG CGCCCGGCAT 100
<210> 3089
<211> 100
<212> DNA
<213> Escherichia coli K-12 MG1655
<220> yihs b3881
<222> 1088-1187
<223> yihs b3881 U00096 complement(4068095..4069351)
<313> 3089: 1123-1142
<400> 3089
GTTCCTGGTG GCAGGAGCTG GATGCGGACA ATAAGGTCAC CACCAAAGTC 50
TGGGACGGCA AACAGGATAT TTATCACCTG CTGCATTGCC TGGTGATCCC 100
<210> 3090
<211> 100
<212> DNA
<213> Escherichia coli K-12 MG1655
<220> yihs b3881
<222> 954-1053
<223> yihs b3881 U00096 complement(4068095..4069351)
<313> 3090: 982-1001
<400> 3090
TGTACGTTGG CCTATCGTCG AAGCAATGGG TACGGCCTAC GCGCTCTACA 50
CCGTCACTGG CGATCGCCAG TATGAAACCT GGTATCAAAC ATGGTGGGAG 100
<210> 3091
<211> 100
<212> DNA
<213> Escherichia coli K-12 MG1655
<220> b2997 b2997
<222> 1020-1119
<223> b2997 b2997 U00096 complement(3143158..3144276)
<313> 3091: 1066-1085
<400> 3091
CGGCGGTGTG GTTGGGTTGG TTGCCGGTGT CAGCGTGATG GCGGTGCGTG 50
AACTGGGTCG TCAGCAAAAG AAAGATAACG CTGACTCACG GGGAGAATAA 100
<210> 3092
<211> 100
<212> DNA
<213> Escherichia coli K-12 MG1655

<220> b2997 b2997
<222> 957-1056
<223> b2997 b2997 U00096 complement(3143158..3144276)
<313> 3092: 1007-1026
<400> 3092
ACAGAAACCG GATGTTAACG CTAAAGAGGG CGGCAACGTC TCTGCAGGCG 50
CTATTGGTTT GCTCGGCGGT GTGGTTGGGT TGGTTGCCGG TGTCAGCGTG 100
<210> 3093
<211> 100
<212> DNA
<213> Escherichia coli K-12 MG1655
<220> b2997 b2997
<222> 898-997
<223> b2997 b2997 U00096 complement(3143158..3144276)
<313> 3093: 945-964
<400> 3093
ATCGGCTTCC ATAAAGGCAT CCATCAGCTT GCCAACGTCG AAAATCAAAC 50
TCCGCGTTCA CAGAAACCGG ATGTTAACGC TAAAGAGGGC GGCAACGTCT 100
<210> 3094
<211> 100
<212> DNA
<213> Escherichia coli K-12 MG1655
<220> yiht b3882
<222> 762-861
<223> yiht b3882 U00096 complement(4069353..4070231)
<313> 3094: 795-814
<400> 3094
AGTCTGGGCA TCGGTCGTCG GTTTACCAGA CAACGAGCTG ATGCTGCGTG 50
ACGTTTGCGC ACCGAAATTA CAACAACTTG GCGATATCGT CGACGAAATG 100
<210> 3095
<211> 100
<212> DNA
<213> Escherichia coli K-12 MG1655
<220> yiht b3882
<222> 582-681
<223> yiht b3882 U00096 complement(4069353..4070231)
<313> 3095: 632-651
<400> 3095
TGAAATGCCG CTTTATGGCA AAGGTCCGCA ACAAGAGCTT CTCTGTGCTT 50
CACAACGTCT GAATGACCAT ATCAATATGC CATGGGTGAT CCTCTCTTCC 100
<210> 3096
<211> 100
<212> DNA
<213> Escherichia coli K-12 MG1655
<220> yiht b3882
<222> 546-645
<223> yiht b3882 U00096 complement(4069353..4070231)
<313> 3096: 575-594
<400> 3096
AGAGCTGGGC GACAGTGGCG CTGACCTCTA CAAAGTTGAA ATGCCGCTTT 50
ATGGCAAAGG TCCGCAACAA GAGCTTCTCT GTGCTTCACA ACGTCTGAAT 100
<210> 3097
<211> 100
<212> DNA
<213> Escherichia coli K-12 MG1655
<220> muth mutH
<222> 591-690
<223> muth mutH U00096 2967684..2968373
<313> 3097: 637-656
<400> 3097
TGGTGCCCGG GGCGAACGGA TTCTGACGCT GCCACGCGGC TTTTATTTGA 50
AGAAGAATTT CACCAGTGCA CTACTGGCCC GTCATTTTCT GATCCAGTAG 100
<210> 3098
<211> 100
<212> DNA
<213> Escherichia coli K-12 MG1655
<220> muth mutH
<222> 489-588
<223> muth mutH U00096 2967684..2968373
<313> 3098: 538-557
<400> 3098

TATGATTGTT CTCGGTCAGG TTGAGCGGAT TACCGCTCGT CACGGGGAGT 50
ATTTACAGAT ACGACCGAAA GCAGCGAATG CGAAAGCGCT TACCGAAGCC 100
<210> 3099
<211> 100
<212> DNA
<213> Escherichia coli K-12 MG1655
<220> muth mutH
<222> 408-507
<223> muth mutH U00096 2967684..2968373
<313> 3099: 458-477
<400> 3099
TCGCGTTGGA TCACCGTTGC TGTGGAGCCC GAATGAAGAG GAAGACCGGC 50
AGCTACGCGA AGACTGGGAA GAATTAATGG ATATGATTGT TCTCGGTCAG 100
<210> 3100
<211> 100
<212> DNA
<213> Escherichia coli K-12 MG1655
<220> b2998 b2998
<222> 171-270
<223> b2998 b2998 U00096 complement(3144465..3144752)
<313> 3100: 199-218
<400> 3100
GGGTATTCTG ACGCGCCGCT ATGGACTGGA TAAAGAGATG GTAATGGATT 50
TCTTTCGTGA GAATAATTCC TGTTCTACGT TGCGCTTTTT TATGGCCGGT 100
<210> 3101
<211> 100
<212> DNA
<213> Escherichia coli K-12 MG1655
<220> b2998 b2998
<222> 112-211
<223> b2998 b2998 U00096 complement(3144465..3144752)
<313> 3101: 143-162
<400> 3101
GGCTACTCAC ACCGGATGTA CGAAAAGACC GGAGATCGCC AGCTTAGCGC 50
CTGGGAAGCG GGTATTCTGA CGCGCCGCTA TGGACTGGAT AAAGAGATGG 100
<210> 3102
<211> 100
<212> DNA
<213> Escherichia coli K-12 MG1655
<220> b2998 b2998
<222> 25-124
<223> b2998 b2998 U00096 complement(3144465..3144752)
<313> 3102: 65-84
<400> 3102
TTAATGGCGG GATTAAAAGC AACGCATGCC GACAGTGCGG TTAATGTGAC 50
AAAATTCTGT GCCGATTATA AACGCGGTTT TGTATTAGGC TACTCACACC 100
<210> 3103
<211> 100
<212> DNA
<213> Escherichia coli K-12 MG1655
<220> yihu b3883
<222> 798-897
<223> yihu b3883 U00096 complement(4070255..4071151)
<313> 3103: 871-890
<400> 3103
TAGCCAGGCG CGCGCAGCGG GTCGCGGTCG CCAGGACTGG TCCGCCATTC 50
TGGAACAGGT CCGTGTCAGT GCCGGGATGA CTGCCAAAGT AAAAATGTAA 100
<210> 3104
<211> 100
<212> DNA
<213> Escherichia coli K-12 MG1655
<220> yihu b3883
<222> 755-854
<223> yihu b3883 U00096 complement(4070255..4071151)
<313> 3104: 786-805
<400> 3104
TGCATGTGCC AATGCCGCTG GGGGCCGCCT CACGGGAGGT TTATAGCCAG 50
GCGCGCGCAG CGGGTCGCGG TCGCCAGGAC TGGTCCGCCA TTCTGGAACA 100
<210> 3105
<211> 100
<212> DNA

<213> Escherichia coli K-12 MG1655
<220> yihu b3883
<222> 537-636
<223> yihu b3883 U00096 complement(4070255..4071151)
<313> 3105: 597-616
<400> 3105
CGCGCTCAAT GCGCTTTCGG CAGAAGCCGC CGTTTTGTGC GAAGCCCTGA  50
ATCTTCCCTT CGATGTTGCC GTCAAAGTGA TGAGCGGTAC CGCCGCCGGT  100
<210> 3106
<211> 100
<212> DNA
<213> Escherichia coli K-12 MG1655
<220> b2999 b2999
<222> 312-411
<223> b2999 b2999 U00096 complement(3144871..3145281)
<313> 3106: 377-396
<400> 3106
TCACGGATTC CATAATGATT CCACGCCCCG TTATGACAAA TCTGCCGCCG  50
ATCTTGCCTG GCAAAGGACA CTGAAATGGT TCGATAAATA TCTCTCCTGA  100
<210> 3107
<211> 100
<212> DNA
<213> Escherichia coli K-12 MG1655
<220> b2999 b2999
<222> 130-229
<223> b2999 b2999 U00096 complement(3144871..3145281)
<313> 3107: 182-201
<400> 3107
GCCTGCGCGG TGCCGTTTTA TGGTCGTCAG GCACCCACTG CCGATGTGGC  50
GAAGATTGAA GCGCCTTTAC TACTCCACTT CGCGGAACTG GACACCCGAA  100
<210> 3108
<211> 100
<212> DNA
<213> Escherichia coli K-12 MG1655
<220> b2999 b2999
<222> 1-100
<223> b2999 b2999 U00096 complement(3144871..3145281)
<313> 3108: 30-49
<400> 3108
ATGAATGATT TCTTTGCCGC AATTGAGTTT ATGCAACGCT ATCCGCAAGC  50
GACAGGCAAA GTGGGTATTA CCGGATTTTG CTATGGCGGT GGCGTATCGA  100
<210> 3109
<211> 100
<212> DNA
<213> Escherichia coli K-12 MG1655
<220> yihv b3884
<222> 794-893
<223> yihv b3884 U00096 4071313..4072215
<313> 3109: 818-837
<400> 3109
CAGTCCGCTT CGCCAGCGGT GTAGCGGCGT TAAAATGCAC ACGTCCCGGT  50
GGACGAGCCG GGATCCCTGA CTGTGATCAA ACCCGATCTT TTTTGTCACT  100
<210> 3110
<211> 100
<212> DNA
<213> Escherichia coli K-12 MG1655
<220> yihv b3884
<222> 680-779
<223> yihv b3884 U00096 4071313..4072215
<313> 3110: 729-748
<400> 3110
GGCGTCAGCA TCAACCGGCC TTCAAAGTTG ATGTGGTAGA TACCACAGGT  50
GCGGGTGATG TTTTTCACGG CGCTTTGGCG GTGGCGCTGG CAACAAGTGG  100
<210> 3111
<211> 100
<212> DNA
<213> Escherichia coli K-12 MG1655
<220> yihv b3884
<222> 625-724
<223> yihv b3884 U00096 4071313..4072215
<313> 3111: 681-700

<400> 3111
GGTCATGTCT ATGTGACCCA GGGTAGCGCA GGCTGCGACT GGCTGGAAAA 50
TGGTGGGCGT CAGCATCAAC CGGCCTTCAA AGTTGATGTG GTAGATACCA 100
<210> 3112
<211> 100
<212> DNA
<213> Escherichia coli K-12 MG1655
<220> yihw b3885
<222> 701-800
<223> yihw b3885 U00096 4072225..4073034
<313> 3112: 754-773
<400> 3112
ACGTGGCACA GGTCACTGCG CTCTTTACCG ACGAGCTGCC GCCCGCTGCG 50
CTAAAATCAC GCTTACAAGA CAGCCAAATT GAAATCATTC TTCCCCAGGA 100
<210> 3113
<211> 100
<212> DNA
<213> Escherichia coli K-12 MG1655
<220> yihw b3885
<222> 663-762
<223> yihw b3885 U00096 4072225..4073034
<313> 3113: 714-733
<400> 3113
CACTAAGTAT CATGCTTCGG CGGCGGTTGA AATTGGTAAC GTGGCACAGG 50
TCACTGCGCT CTTTACCGAC GAGCTGCCGC CCGCTGCGCT AAAATCACGC 100
<210> 3114
<211> 100
<212> DNA
<213> Escherichia coli K-12 MG1655
<220> yihw b3885
<222> 648-747
<223> yihw b3885 U00096 4072225..4073034
<313> 3114: 676-695
<400> 3114
GCTGGTCGCC GATCACACTA AGTATCATGC TTCGGCGGCG GTTGAAATTG 50
GTAACGTGGC ACAGGTCACT GCGCTCTTTA CCGACGAGCT GCCGCCCGCT 100
<210> 3115
<211> 100
<212> DNA
<213> Escherichia coli K-12 MG1655
<220> yihx b3886
<222> 517-616
<223> yihx b3886 U00096 4073112..4073732
<313> 3115: 571-590
<400> 3115
GATAACGCCG ATAATATAGA AGGAGCCAAT CAGCTGGGCA TTACCAGTAT 50
TCTGGTGAAA GATAAAACCA CCATCCCGGA CTATTTCGCG AAGGTGTTAT 100
<210> 3116
<211> 100
<212> DNA
<213> Escherichia coli K-12 MG1655
<220> yihx b3886
<222> 477-576
<223> yihx b3886 U00096 4073112..4073732
<313> 3116: 510-529
<400> 3116
GGCGGAAGGT TTTTCACCCA GCGATACGGT CTTTTTCGAC GATAACGCCG 50
ATAATATAGA AGGAGCCAAT CAGCTGGGCA TTACCAGTAT TCTGGTGAAA 100
<210> 3117
<211> 100
<212> DNA
<213> Escherichia coli K-12 MG1655
<220> yihx b3886
<222> 375-474
<223> yihx b3886 U00096 4073112..4073732
<313> 3117: 403-422
<400> 3117
GGAAGAATAC CCGGAAATTC GTGATGCTGC TGACCATATC TATCTGTCGC 50
AAGATCTGGG GATGCGCAAA CCTGAAGCAC GAATTTACCA GCATGTTTTG 100
<210> 3118
<211> 100

<212> DNA
<213> Escherichia coli K-12 MG1655
<220> mutl b4171
<222> 1749-1848
<223> mutl b4171 U00096 4394990..4396837
<313> 3118: 1782-1801
<400> 3118
CGTGGAACGG TTATGTCCGC AACTTGTGAA AACGCCGCCG GGTGGTCTGT 50
TACAATCTGT TGATTTACAT CCGGCGATAA AAGCCCTGAA AGATGAGTGA 100
<210> 3119
<211> 100
<212> DNA
<213> Escherichia coli K-12 MG1655
<220> mutl b4171
<222> 1602-1701
<223> mutl b4171 U00096 4394990..4396837
<313> 3119: 1640-1659
<400> 3119
TTTACAAATC TTGATTCCTG AACTGATAGG CTACCTGGCG AAGCAGTCCG 50
TATTCGAACC TGGCAATATT GCGCAGTGGA TTGCACGAAA TCTGATGAGC 100
<210> 3120
<211> 100
<212> DNA
<213> Escherichia coli K-12 MG1655
<220> mutl b4171
<222> 1551-1650
<223> mutl b4171 U00096 4394990..4396837
<313> 3120: 1576-1595
<400> 3120
TGCACAGCAT GTGACCATCA GGGCAGTGCC TTTACCCTTA CGCCAACAAA 50
ATTTACAAAT CTTGATTCCT GAACTGATAG GCTACCTGGC GAAGCAGTCC 100
<210> 3121
<211> 100
<212> DNA
<213> Escherichia coli K-12 MG1655
<220> mutm b3636
<222> 637-736
<223> mutm b3636 U00096 complement(3807970..3808779)
<313> 3121: 687-706
<400> 3121
GGTGGTACAA CGCTGAAAGA TTTTCTGCAA AGTGATGGTA AACCGGGCTA 50
TTTCGCTCAG GAATTGCAGG TTTACGGGCG AAAAGGTGAG CCGTGTCGGG 100
<210> 3122
<211> 100
<212> DNA
<213> Escherichia coli K-12 MG1655
<220> mutm b3636
<222> 595-694
<223> mutm b3636 U00096 complement(3807970..3808779)
<313> 3122: 644-663
<400> 3122
GCTCGGGTGA TTAAAGCGGT GTTGCTGCGT TCGATTGAGC AGGGTGGTAC 50
AACGCTGAAA GATTTTCTGC AAAGTGATGG TAAACCGGGC TATTTCGCTC 100
<210> 3123
<211> 100
<212> DNA
<213> Escherichia coli K-12 MG1655
<220> mutm b3636
<222> 465-564
<223> mutm b3636 U00096 complement(3807970..3808779)
<313> 3123: 506-525
<400> 3123
ACCGTGGCTG ATGGATAACA AGCTGGTGGT AGGGGTAGGG AATATCTATG 50
CCAGCGAATC ACTGTTTGCG GCGGGGATCC ATCCGGATCG GCTGGCGTCA 100
<210> 3124
<211> 100
<212> DNA
<213> Escherichia coli K-12 MG1655
<220> yihz b3888
<222> 339-438
<223> yihz b3888 U00096 4074595..4075032

<313> 3124: 402-421
<400> 3124
CCGTCAGCAA GAGATGAACA CGCAAACAGG ACGCTTCGCT GCGGATATGC 50
AGGTATCGCT GGTCAATGAT GGCCCCGTGA CATTCTGGTT GCAGGTATGA 100
<210> 3125
<211> 100
<212> DNA
<213> Escherichia coli K-12 MG1655
<220> yihz b3888
<222> 319-418
<223> yihz b3888 U00096 4074595..4075032
<313> 3125: 360-379
<400> 3125
GACTATTTCG TCGAACGCTG CCGTCAGCAA GAGATGAACA CGCAAACAGG 50
ACGCTTCGCT GCGGATATGC AGGTATCGCT GGTCAATGAT GGCCCCGTGA 100
<210> 3126
<211> 100
<212> DNA
<213> Escherichia coli K-12 MG1655
<220> yihz b3888
<222> 306-405
<223> yihz b3888 U00096 4074595..4075032
<313> 3126: 322-341
<400> 3126
AGAGGCGTTA TATGACTATT TCGTCGAACG CTGCCGTCAG CAAGAGATGA 50
ACACGCAAAC AGGACGCTTC GCTGCGGATA TGCAGGTATC GCTGGTCAAT 100
<210> 3127
<211> 100
<212> DNA
<213> Escherichia coli K-12 MG1655
<220> lipa b0629
<222> 867-966
<223> lipa b0629 U00096 complement(658474..659439)
<313> 3127: 937-956
<400> 3127
CGAAGCGCTG GCGATGGGCT TTACCCATGC TGCATGCGGT CCGTTTGTCC 50
GCTCTTCTTA CCACGCCGAT TTGCAGGCGA AAGGGATGGA AGTTAAGTAA 100
<210> 3128
<211> 100
<212> DNA
<213> Escherichia coli K-12 MG1655
<220> lipa b0629
<222> 823-922
<223> lipa b0629 U00096 complement(658474..659439)
<313> 3128: 837-856
<400> 3128
GTTCAACGTT ACGTTAGCCC GGATGAGTTC GACGAAATGA AAGCCGAAGC 50
GCTGGCGATG GGCTTTACCC ATGCTGCATG CGGTCCGTTT GTCCGCTCTT 100
<210> 3129
<211> 100
<212> DNA
<213> Escherichia coli K-12 MG1655
<220> lipa b0629
<222> 754-853
<223> lipa b0629 U00096 complement(658474..659439)
<313> 3129: 806-825
<400> 3129
CTGCGCCGTC ATGGTGTGAC GATGTTAACG CTGGGGCAAT ATTTGCAGCC 50
AAGCCGCCAT CACCTGCCGG TTCAACGTTA CGTTAGCCCG GATGAGTTCG 100
<210> 3130
<211> 100
<212> DNA
<213> Escherichia coli K-12 MG1655
<220> lipb b0631
<222> 464-563
<223> lipb b0631 U00096 complement(660860..661435)
<313> 3130: 502-521
<400> 3130
CTAAAATATC ACAATGGAAA CCCGAAGCGA CGACTAATAA TATTGCTCCA 50
CGTTTACTGG AAAATATTTT AGCGCTACTA AACAATCCGG ACTTCGAATA 100
<210> 3131

<211> 100
<212> DNA
<213> Escherichia coli K-12 MG1655
<220> lipb b0631
<222> 368-467
<223> lipb b0631 U00096 complement(660860..661435)
<313> 3131: 389-408
<400> 3131
GCGGTTGTTC ATTCCACGGT CTGGCATTAA ACGTCAATAT GGATCTTTCA 50
CCATTTTTAC GTATTAATCC TTGTGGGTAT GCCGGAATGG AAATGGCTAA 100
<210> 3132
<211> 100
<212> DNA
<213> Escherichia coli K-12 MG1655
<220> lipb b0631
<222> 313-412
<223> lipb b0631 U00096 complement(660860..661435)
<313> 3132: 354-373
<400> 3132
CCAGGTGTCT ATGTTGGGGA AAAGAAAATT TGCTCACTGG GTTTACGTAT 50
TCGACGCGGT TGTTCATTCC ACGGTCTGGC ATTAAACGTC AATATGGATC 100
<210> 3133
<211> 100
<212> DNA
<213> Escherichia coli K-12 MG1655
<220> b0024 b0025
<222> 115-214
<223> b0024 b0025 U00096 21181..21399
<313> 3133: 137-156
<400> 3133
TGCGCCATGG CAATGAAAAG CCACTTCTTT CTGATTTCGG TACTCAATCG 50
CCGGTTAACC TTGACCGCTG TACAAGGTAT ACTCGGACGA TTTTCACTGT 100
<210> 3134
<211> 100
<212> DNA
<213> Escherichia coli K-12 MG1655
<220> b0024 b0025
<222> 96-195
<223> b0024 b0025 U00096 21181..21399
<313> 3134: 136-155
<400> 3134
TCAGTTTCTT CCCGTGATTT GCGCCATGGC AATGAAAAGC CACTTCTTTC 50
TGATTTCGGT ACTCAATCGC CGGTTAACCT TGACCGCTGT ACAAGGTATA 100
<210> 3135
<211> 100
<212> DNA
<213> Escherichia coli K-12 MG1655
<220> b0024 b0025
<222> 66-165
<223> b0024 b0025 U00096 21181..21399
<313> 3135: 95-114
<400> 3135
CTTTTCCGCG ATAAAAATTG CAGCAGGCGG TCAGTTTCTT CCCGTGATTT 50
GCGCCATGGC AATGAAAAGC CACTTCTTTC TGATTTCGGT ACTCAATCGC 100
<210> 3136
<211> 100
<212> DNA
<213> Escherichia coli K-12 MG1655
<220> muts b2734
<222> 2463-2562
<223> muts b2734 U00096 2855116..2857677
<313> 3136: 2537-2556
<400> 3136
AACTTCGCCT GCGGTCGAAG CTCTGGAAAA TCTTGATCCG GATTCACTCA 50
CCCCGCGTCA GGCGCTGGAG TGGATTTATC GCTTGAAGAG CCTGGTGTAA 100
<210> 3137
<211> 100
<212> DNA
<213> Escherichia coli K-12 MG1655
<220> muts b2734
<222> 2413-2512

<223> muts b2734 U00096 2855116..2857677
<313> 3137: 2482-2501
<400> 3137
GCTACGCAAG TGGATGGTAC GCAAATGTCT TTGCTGTCAG TACCAGAAGA 50
AACTTCGCCT GCGGTCGAAG CTCTGGAAAA TCTTGATCCG GATTCACTCA 100
<210> 3138
<211> 100
<212> DNA
<213> Escherichia coli K-12 MG1655
<220> muts b2734
<222> 2379-2478
<223> muts b2734 U00096 2855116..2857677
<313> 3138: 2420-2439
<400> 3138
GCGTGAGCTG GAAAGCATTT CGCCGAACGC CGCCGCTACG CAAGTGGATG 50
GTACGCAAAT GTCTTTGCTG TCAGTACCAG AAGAAACTTC GCCTGCGGTC 100
<210> 3139
<211> 100
<212> DNA
<213> Escherichia coli K-12 MG1655
<220> mutt mutT
<222> 290-389
<223> mutt mutT U00096 111044..111433
<313> 3139: 325-344
<400> 3139
AAGAAGGGCA ACCCGGTGAG TGGATGTCGC TGGTCGGTCT TAATGCCGAT 50
GATTTTCCGC CAGCCAATGA ACCGGTAATT GCGAAGCTTA AACGTCTGTA 100
<210> 3140
<211> 100
<212> DNA
<213> Escherichia coli K-12 MG1655
<220> mutt mutT
<222> 247-346
<223> mutt mutT U00096 111044..111433
<313> 3140: 284-303
<400> 3140
TGGTTTTGGC TGGTCGAACG CTGGGAAGGG GAGCCGTGGG GTAAAGAAGG 50
GCAACCCGGT GAGTGGATGT CGCTGGTCGG TCTTAATGCC GATGATTTTC 100
<210> 3141
<211> 100
<212> DNA
<213> Escherichia coli K-12 MG1655
<220> mutt mutT
<222> 208-307
<223> mutt mutT U00096 111044..111433
<313> 3141: 235-254
<400> 3141
AAACTGGAAT ATGAATTCCC GGACAGGCAT ATAACACTGT GGTTTTGGCT 50
GGTCGAACGC TGGGAAGGGG AGCCGTGGGG TAAAGAAGGG CAACCCGGTG 100
<210> 3142
<211> 100
<212> DNA
<213> Escherichia coli K-12 MG1655
<220> yiid b3889
<222> 891-990
<223> yiid b3889 U00096 4075029..4076018
<313> 3142: 953-972
<400> 3142
TGGCGACGAG ACGCCGGGTG CAGTGTTTGA AGGCACGTAT ATCGTTCTGC 50
CCGCGAAGCC ATTTGGCCCG TATGAAGAGG GCGGGAACGA AGAAGAGTAG 100
<210> 3143
<211> 100
<212> DNA
<213> Escherichia coli K-12 MG1655
<220> yiid b3889
<222> 875-974
<223> yiid b3889 U00096 4075029..4076018
<313> 3143: 912-931
<400> 3143
TGCAGGTCGA AATCTTTGGC GACGAGACGC CGGGTGCAGT GTTTGAAGGC 50
ACGTATATCG TTCTGCCCGC GAAGCCATTT GGCCCGTATG AAGAGGGCGG 100

<210> 3144
<211> 100
<212> DNA
<213> Escherichia coli K-12 MG1655
<220> yiid b3889
<222> 823-922
<223> yiid b3889 U00096 4075029..4076018
<313> 3144: 882-901
<400> 3144
TTAAGCGGCG ATCTCGACCG TCTGGCGCGC GGACGAAAAG CACGGGTGCA 50
GATGCAGGTC GAAATCTTTG GCGACGAGAC GCCGGGTGCA GTGTTTGAAG 100
<210> 3145
<211> 100
<212> DNA
<213> Escherichia coli K-12 MG1655
<220> muty b2962
<222> 954-1053
<223> muty b2962 U00096 3101031..3102083
<313> 3145: 1007-1026
<400> 3145
CAATGCGCTC TGGTATAACT TAGCGCAACC GCCGTCAGTT GGCCTAGCGG 50
CTCCCGTGGA GCGTTTGTTA CAGCAGTTAC GCACTGGCGC GCCGGTTTAG 100
<210> 3146
<211> 100
<212> DNA
<213> Escherichia coli K-12 MG1655
<220> muty b2962
<222> 926-1025
<223> muty b2962 U00096 3101031..3102083
<313> 3146: 945-964
<400> 3146
CGTCATTCAC CGGCTGCATG GATGAAGGCA ATGCGCTCTG GTATAACTTA 50
GCGCAACCGC CGTCAGTTGG CCTAGCGGCT CCCGTGGAGC GTTTGTTACA 100
<210> 3147
<211> 100
<212> DNA
<213> Escherichia coli K-12 MG1655
<220> muty b2962
<222> 849-948
<223> muty b2962 U00096 3101031..3102083
<313> 3147: 916-935
<400> 3147
CCTGACGCAA CTGACCGCGT TTCGGCATAC CTTCAGCCAT TTCCACTTAG 50
ATATTGTGCC TATGTGGCTT CCCGTGTCGT CATTCACCGG CTGCATGGAT 100
<210> 3148
<211> 100
<212> DNA
<213> Escherichia coli K-12 MG1655
<220> yiie b3890
<222> 147-246
<223> yiie b3890 U00096 4076844..4077089
<313> 3148: 204-223
<400> 3148
TCTGGAACGT CAGCAATTCC CGGTGCGTAA AGCAAAGGGA GGCAGGCAAA 50
AGGGGGAGGT AGTTGGTGTT GATGATCAGT GTAAGGAGCA TAAGGAATGA 100
<210> 3149
<211> 100
<212> DNA
<213> Escherichia coli K-12 MG1655
<220> yiie b3890
<222> 117-216
<223> yiie b3890 U00096 4076844..4077089
<313> 3149: 179-198
<400> 3149
TGCAATTTTA CGCGAAGCGG TAGAGCAATA TCTGGAACGT CAGCAATTCC 50
CGGTGCGTAA AGCAAAGGGA GGCAGGCAAA AGGGGGAGGT AGTTGGTGTT 100
<210> 3150
<211> 100
<212> DNA
<213> Escherichia coli K-12 MG1655
<220> yiie b3890

<222> 76-175
<223> yiie b3890 U00096 4076844..4077089
<313> 3150: 125-144
<400> 3150
CGGCTGAACA AGCTTCGGGG CTGGCGTAAG GTTTCACGAT CTGCAATTTT  50
ACGCGAAGCG GTAGAGCAAT ATCTGGAACG TCAGCAATTC CCGGTGCGTA  100
<210> 3151
<211> 100
<212> DNA
<213> Escherichia coli K-12 MG1655
<220> yiif b3891
<222> 144-243
<223> yiif b3891 U00096 4077307..4077549
<313> 3151: 222-241
<400> 3151
AAATCAATCG CAAACAGCAA GAACCAGTGT TCCTGGCATC TGGCAAGGGT  50
GTGAGGAAGA TGGTGTCGAA TATCAGCGTA AGCTGCGCGA GGAATGGTAA  100
<210> 3152
<211> 100
<212> DNA
<213> Escherichia coli K-12 MG1655
<220> yiif b3891
<222> 125-224
<223> yiif b3891 U00096 4077307..4077549
<313> 3152: 168-187
<400> 3152
CGGTAGATCA ATACCTGATA AATCAATCGC AAACAGCAAG AACCAGTGTT  50
CCTGGCATCT GGCAAGGGTG TGAGGAAGAT GGTGTCGAAT ATCAGCGTAA  100
<210> 3153
<211> 100
<212> DNA
<213> Escherichia coli K-12 MG1655
<220> yiif b3891
<222> 55-154
<223> yiif b3891 U00096 4077307..4077549
<313> 3153: 117-136
<400> 3153
GAGGTGATTA AGCAACTTGA TGATCTCGAG GTGCAGCGTA ATCTTCCTCG  50
TGCGGACCTA TTAAGGGAAG CGGTAGATCA ATACCTGATA AATCAATCGC  100
<210> 3154
<211> 100
<212> DNA
<213> Escherichia coli K-12 MG1655
<220> yiig b3897
<222> 957-1056
<223> yiig b3897 U00096 4084582..4085637
<313> 3154: 1006-1025
<400> 3154
ACTTCAGGAC GCAAACTCAA GCTGGATGGT CGAAGACTCT TTCCCGAGAG  50
CATTACGCGA GTACAACGAA ATGGTTGATG ACTATAATAG CCTGCGTTAA  100
<210> 3155
<211> 100
<212> DNA
<213> Escherichia coli K-12 MG1655
<220> yiig b3897
<222> 935-1034
<223> yiig b3897 U00096 4084582..4085637
<313> 3155: 977-996
<400> 3155
ACTCTGACTG GGATAAAGAG CAACTTCAGG ACGCAAACTC AAGCTGGATG  50
GTCGAAGACT CTTTCCCGAG AGCATTACGC GAGTACAACG AAATGGTTGA  100
<210> 3156
<211> 100
<212> DNA
<213> Escherichia coli K-12 MG1655
<220> yiig b3897
<222> 863-962
<223> yiig b3897 U00096 4084582..4085637
<313> 3156: 914-933
<400> 3156
TTATTAATTC GGCAGGCCAG TATCAACTCG AGGCTAAAAA ATACGTTCGC  50

CGCATCAGAG ATAAAGTCCC GTACTCTGAC TGGGATAAAG AGCAACTTCA 100
<210> 3157
<211> 100
<212> DNA
<213> Escherichia coli K-12 MG1655
<220> yiil b3902
<222> 216-315
<223> yiil b3902 U00096 complement(4090705..4091019)
<313> 3157: 281-300
<400> 3157
TTGCCAACGT TGGTGGAAAT ATATGACCGA TGTTATGCCC GCTAACCCGG 50
ATAACAGCCC GGTGAGTAGC GAGCTGCAAG AAGTGTTTTA CCTGCCGTAA 100
<210> 3158
<211> 100
<212> DNA
<213> Escherichia coli K-12 MG1655
<220> yiil b3902
<222> 194-293
<223> yiil b3902 U00096 complement(4090705..4091019)
<313> 3158: 254-273
<400> 3158
ATGCGGTTGC CAGCACTGAT GTTTGCCAAC GTTGGTGGAA ATATATGACC 50
GATGTTATGC CCGCTAACCC GGATAACAGC CCGGTGAGTA GCGAGCTGCA 100
<210> 3159
<211> 100
<212> DNA
<213> Escherichia coli K-12 MG1655
<220> yiil b3902
<222> 150-249
<223> yiil b3902 U00096 complement(4090705..4091019)
<313> 3159: 180-199
<400> 3159
TCTGCTGTTT GCCATGGTAG AGATTGAATC TGAAGAACGC TGGAATGCGG 50
TTGCCAGCAC TGATGTTTGC CAACGTTGGT GGAAATATAT GACCGATGTT 100
<210> 3160
<211> 100
<212> DNA
<213> Escherichia coli K-12 MG1655
<220> yiim b3911
<222> 606-705
<223> yiim b3911 U00096 4100373..4101077
<313> 3160: 655-674
<400> 3160
CTCCGCTGCC GGGTTATCGA AAAGCTGGAC GCGGACGATG CAAAAGCGCC 50
GACTGAGCGG CAAGATCGAA GATTTTTCCC GCCGGTTGTG GGGAAAATAA 100
<210> 3161
<211> 100
<212> DNA
<213> Escherichia coli K-12 MG1655
<220> yiim b3911
<222> 561-660
<223> yiim b3911 U00096 4100373..4101077
<313> 3161: 599-618
<400> 3161
CGCATGGCAT ATGCCGTTTG ATGACGATCA ATATCACCGT TTACTCTCCG 50
CTGCCGGGTT ATCGAAAAGC TGGACGCGGA CGATGCAAAA GCGCCGACTG 100
<210> 3162
<211> 100
<212> DNA
<213> Escherichia coli K-12 MG1655
<220> yiim b3911
<222> 477-576
<223> yiim b3911 U00096 4100373..4101077
<313> 3162: 523-542
<400> 3162
AGCGCCAGGG AAAGTGTCTG CGGACGCGCC GCTGGAGTTG GTTTCCCGTG 50
TCAGCGATGT GACCGTGCAG GAGGCTGCCG CCATCGCATG GCATATGCCG 100
<210> 3163
<211> 100
<212> DNA
<213> Escherichia coli K-12 MG1655

<220> gapa b1780
<222> 897-996
<223> gapa b1780 U00096 1860795..1861790
<313> 3163: 967-986
<400> 3163
CGCTCTGAAC GACAACTTCG TGAAACTGGT ATCCTGGTAC GACAACGAAA 50
CCGGTTACTC CAACAAAGTT CTGGACCTGA TCGCTCACAT CTCCAAATAA 100
<210> 3164
<211> 100
<212> DNA
<213> Escherichia coli K-12 MG1655
<220> gapa b1780
<222> 819-918
<223> gapa b1780 U00096 1860795..1861790
<313> 3164: 857-876
<400> 3164
CTACACCGAA GATGACGTAG TATCTACCGA TTTCAACGGC GAAGTTTGCA 50
CTTCCGTGTT CGATGCTAAA GCTGGTATCG CTCTGAACGA CAACTTCGTG 100
<210> 3165
<211> 100
<212> DNA
<213> Escherichia coli K-12 MG1655
<220> gapa b1780
<222> 794-893
<223> gapa b1780 U00096 1860795..1861790
<313> 3165: 832-851
<400> 3165
AAGGCGAAAT GAAAGGCGTT CTGGGCTACA CCGAAGATGA CGTAGTATCT 50
ACCGATTTCA ACGGCGAAGT TTGCACTTCC GTGTTCGATG CTAAAGCTGG 100
<210> 3166
<211> 100
<212> DNA
<213> Escherichia coli K-12 MG1655
<220> yiip b3916
<222> 804-903
<223> yiip b3916 U00096 4104049..4104951
<313> 3166: 880-899
<400> 3166
GCAGGCTATT TTACGGCGTT TTCCGGGATC GGATGTAATT ATCCATCAGG 50
ACCCCTGTTC CGTCGTACCC AGGGAGGGTA AACGGTCTAT GCTTTCATAA 100
<210> 3167
<211> 100
<212> DNA
<213> Escherichia coli K-12 MG1655
<220> yiip b3916
<222> 774-873
<223> yiip b3916 U00096 4104049..4104951
<313> 3167: 796-815
<400> 3167
TCAGGCACAT ATGGTGGCGG ATCAGGTAGA GCAGGCTATT TTACGGCGTT 50
TTCCGGGATC GGATGTAATT ATCCATCAGG ACCCCTGTTC CGTCGTACCC 100
<210> 3168
<211> 100
<212> DNA
<213> Escherichia coli K-12 MG1655
<220> yiip b3916
<222> 703-802
<223> yiip b3916 U00096 4104049..4104951
<313> 3168: 758-777
<400> 3168
CGCACGCGGC AGTCAGGGCC GACCCGCTTT ATTCAGATTC ATTTGGAAAT 50
GGAAGACTCT CTGCCTTTGG TTCAGGCACA TATGGTGGCG GATCAGGTAG 100
<210> 3169
<211> 100
<212> DNA
<213> Escherichia coli K-12 MG1655
<220> yiiq b3921
<222> 501-600
<223> yiiq b3921 U00096 complement(4109195..4109794)
<313> 3169: 528-547
<400> 3169

GCGTTATGTT GTCGCGGACA ACGGCGAAAA GGGGCTGACC TTCGCTGTTG 50
AACCGATTAA GCTGGCGCTA TCTGAATCGC TTGAAGGTTT GAATAAATGA 100
<210> 3170
<211> 100
<212> DNA
<213> Escherichia coli K-12 MG1655
<220> yiiq b3921
<222> 412-511
<223> yiiq b3921 U00096 complement(4109195..4109794)
<313> 3170: 464-483
<400> 3170
ATGACCAAAA CACAAAGCCA GAAAAAACTG CAGTCGCTAC TAACGGCGGG 50
GAAAAACAAA CGTTATTACA CCGAGACAGA AGGTGCACTG CGTTATGTTG 100
<210> 3171
<211> 100
<212> DNA
<213> Escherichia coli K-12 MG1655
<220> yiiq b3921
<222> 363-462
<223> yiiq b3921 U00096 complement(4109195..4109794)
<313> 3171: 391-410
<400> 3171
GAAAGCTCAG GAATACATGG CAGCGGTGAT CCGCACACTC ACCCCATTAA 50
TGACCAAAAC ACAAAGCCAG AAAAAACTGC AGTCGCTACT AACGGCGGGG 100
<210> 3172
<211> 100
<212> DNA
<213> Escherichia coli K-12 MG1655
<220> yiir b3922
<222> 325-424
<223> yiir b3922 U00096 4109895..4110335
<313> 3172: 369-388
<400> 3172
ATTCTGGTGA TGATGCTTAT CGATCTTGGT GCGTTTGTTG GCACACAAGG 50
CGAAAATAAA TATGGTAAAG ATACTCAGGA TGTGAAGTAT AAAGCCGACA 100
<210> 3173
<211> 100
<212> DNA
<213> Escherichia coli K-12 MG1655
<220> yiir b3922
<222> 183-282
<223> yiir b3922 U00096 4109895..4110335
<313> 3173: 230-249
<400> 3173
AACTGTTAAG CGCTTGCATG ATCGCGGGCG TTCCGGCGCA TGGGCATTTC 50
TGATGATTGT GGCGTGGATG CTGCTGGCGG GTAACTGGGC GATTTTACCG 100
<210> 3174
<211> 100
<212> DNA
<213> Escherichia coli K-12 MG1655
<220> yiir b3922
<222> 43-142
<223> yiir b3922 U00096 4109895..4110335
<313> 3174: 93-112
<400> 3174
GGACGCCGTG ATTTCTGGAT TTGGATAGGC CTGTGGTTCG CAGGCATGCT 50
GGTGCTTTTC TCACTGGCGG GTAAGAATCT ACTCGATATT CAGACCGCGG 100
<210> 3175
<211> 100
<212> DNA
<213> Escherichia coli K-12 MG1655
<220> yiis b3923
<222> 160-259
<223> yiis b3923 U00096 4110547..4110846
<313> 3175: 197-216
<400> 3175
ACATCCTCTG CAGATGAAGG GGCCAGCGTG GCCTATAAGA TTAAGGATCT 50
GGAGGGGCAA GTTGAGCTTG ATGCGGCCTT CACTTTCTCA TGCCAGGCCG 100
<210> 3176
<211> 100
<212> DNA

<213> Escherichia coli K-12 MG1655
<220> yiis b3923
<222> 121-220
<223> yiis b3923 U00096 4110547..4110846
<313> 3176: 150-169
<400> 3176
GAGTCTTTTA TGACCAAACT GAAAGAGCTT GCCGCCGCTA CATCCTCTGC 50
AGATGAAGGG GCCAGCGTGG CCTATAAGAT TAAGGATCTG GAGGGGCAAG 100
<210> 3177
<211> 100
<212> DNA
<213> Escherichia coli K-12 MG1655
<220> yiis b3923
<222> 27-126
<223> yiis b3923 U00096 4110547..4110846
<313> 3177: 89-108
<400> 3177
TACTAAAGGA TGTGCGATTG ATATCGGTAC GGTGATTGAT AACGACAACT 50
GTACCAGTAA GTTTTCGCGC TTTTTTGCCA CCCGCGAAGA AGCAGAGTCT 100
<210> 3178
<211> 100
<212> DNA
<213> Escherichia coli K-12 MG1655
<220> yiit b3924
<222> 328-427
<223> yiit b3924 U00096 4110873..4111301
<313> 3178: 364-383
<400> 3178
GGTCATCATC ACTCATTTAT CAACCGTTTG ATGCCGGCAT ATCGCGGGAT 50
GATCAATAAG ATGAGTGCGG ATTTGCTCAT CGTGCCGTTT ATCGATAAGT 100
<210> 3179
<211> 100
<212> DNA
<213> Escherichia coli K-12 MG1655
<220> yiit b3924
<222> 298-397
<223> yiit b3924 U00096 4110873..4111301
<313> 3179: 328-347
<400> 3179
CAAAAAGAGC AGTGCGACCT CCTTGTCTGT GGTCATCATC ACTCATTTAT 50
CAACCGTTTG ATGCCGGCAT ATCGCGGGAT GATCAATAAG ATGAGTGCGG 100
<210> 3180
<211> 100
<212> DNA
<213> Escherichia coli K-12 MG1655
<220> yiit b3924
<222> 248-347
<223> yiit b3924 U00096 4110873..4111301
<313> 3180: 281-300
<400> 3180
AACTGCGTAT TGAACGCGGA GAAATGCCGG AAACACTGCT GGAAATTATG 50
CAAAAAGAGC AGTGCGACCT CCTTGTCTGT GGTCATCATC ACTCATTTAT 100
<210> 3181
<211> 100
<212> DNA
<213> Escherichia coli K-12 MG1655
<220> yiiu b3929
<222> 127-226
<223> yiiu b3929 U00096 4116095..4116340
<313> 3181: 192-211
<400> 3181
GTTCAAAATG CCCAGCATCA GCGCGAAGAG CTGGAGCGTG AGAACAACCA 50
TCTGAAAGAA CAGCAGAACG GCTGGCAGGA ACGTCTGCAG GCCCTGCTGG 100
<210> 3182
<211> 100
<212> DNA
<213> Escherichia coli K-12 MG1655
<220> yiiu b3929
<222> 95-194
<223> yiiu b3929 U00096 4116095..4116340
<313> 3182: 152-171

<400> 3182
TGAAAGAAAA AAACAACTCA CTGTCGCAGG AAGTTCAAAA TGCCCAGCAT 50
CAGCGCGAAG AGCTGGAGCG TGAGAACAAC CATCTGAAAG AACAGCAGAA 100
<210> 3183
<211> 100
<212> DNA
<213> Escherichia coli K-12 MG1655
<220> yiiu b3929
<222> 50-149
<223> yiiu b3929 U00096 4116095..4116340
<313> 3183: 93-112
<400> 3183
AGGCGATTGA TACCATCACT CTGTTGCAGA TGGAAATCGA AGAGCTGAAA 50
GAAAAAAACA ACTCACTGTC GCAGGAAGTT CAAAATGCCC AGCATCAGCG 100
<210> 3184
<211> 100
<212> DNA
<213> Escherichia coli K-12 MG1655
<220> yiix b3938
<222> 510-609
<223> yiix b3938 U00096 complement(4124866..4125474)
<313> 3184: 561-580
<400> 3184
GAATATTCCG CTTGAGGAAA CGGTGGTCTC GCCACAAGCC GTTTTCGATG 50
CGCCACAACT CACCACGGTC GCCAAAGAAT GGCCGCTGTT TTCGTGGTAA 100
<210> 3185
<211> 100
<212> DNA
<213> Escherichia coli K-12 MG1655
<220> yiix b3938
<222> 450-549
<223> yiix b3938 U00096 complement(4124866..4125474)
<313> 3185: 480-499
<400> 3185
GAAAGAGTTC GACCTGAGCA GCCCACAAGT TCAGGCAAAG CTCAAAGAAC 50
GTTACGGCAA GAATATTCCG CTTGAGGAAA CGGTGGTCTC GCCACAAGCC 100
<210> 3186
<211> 100
<212> DNA
<213> Escherichia coli K-12 MG1655
<220> yiix b3938
<222> 418-517
<223> yiix b3938 U00096 complement(4124866..4125474)
<313> 3186: 449-468
<400> 3186
CTGGGAATGC GCGTGGGCGA GCAGCAGAAG TTGAAAGAGT TCGACCTGAG 50
CAGCCCACAA GTTCAGGCAA AGCTCAAAGA ACGTTACGGC AAGAATATTC 100
<210> 3187
<211> 100
<212> DNA
<213> Escherichia coli K-12 MG1655
<220> yijc b3964
<222> 606-705
<223> yijc b3964 U00096 4158646..4159350
<313> 3187: 659-678
<400> 3187
GGTACTGCAA CTGCGAATGA TTTCGAAAGG GGCTTATTAC TGGTATCGCC 50
GTGAACAAGA GAAAACCGCA ATTATTCCGG GAAATGTGAA GGACGAGTAA 100
<210> 3188
<211> 100
<212> DNA
<213> Escherichia coli K-12 MG1655
<220> yijc b3964
<222> 547-646
<223> yijc b3964 U00096 4158646..4159350
<313> 3188: 589-608
<400> 3188
GCGGGTGCCG AGGCGTTGGA CGTCGGCGTC GAACAACGTC GGCAATTAGA 50
AGAGCGACTG GTACTGCAAC TGCGAATGAT TTCGAAAGGG GCTTATTACT 100
<210> 3189
<211> 100

<212> DNA
<213> Escherichia coli K-12 MG1655
<220> yijc b3964
<222> 413-512
<223> yijc b3964 U00096 4158646..4159350
<313> 3189: 462-481
<400> 3189
CGTTTCGTGC CGCCGTTGCG CGTGAAATTC AGCACTTCAT TGCGGAACTT 50
GCGGACTATC TGGAACTCGA AAACCATATG CCGCGTGCGT TTACTGAAGC 100
<210> 3190
<211> 100
<212> DNA
<213> Escherichia coli K-12 MG1655
<220> yijd b3965
<222> 261-360
<223> yijd b3965 U00096 4159350..4159709
<313> 3190: 338-357
<400> 3190
GGATATCGGC TCTAACTTCT TCCCGGCAGT ACTCTCCGTC ATTATGGTGT 50
TCTGGATTGG CGCGAAGATG CGTAACCGTA AGCAGGAAGT TGCTGAGTAA 100
<210> 3191
<211> 100
<212> DNA
<213> Escherichia coli K-12 MG1655
<220> yijd b3965
<222> 247-346
<223> yijd b3965 U00096 4159350..4159709
<313> 3191: 280-299
<400> 3191
CGTGCGGAAT ATCCGGATAT CGGCTCTAAC TTCTTCCCGG CAGTACTCTC 50
CGTCATTATG GTGTTCTGGA TTGGCGCGAA GATGCGTAAC CGTAAGCAGG 100
<210> 3192
<211> 100
<212> DNA
<213> Escherichia coli K-12 MG1655
<220> yijd b3965
<222> 114-213
<223> yijd b3965 U00096 4159350..4159709
<313> 3192: 150-169
<400> 3192
CCCGATTATT TCCCTGGTGC TGACGGTTTA CTGCCTGCAT CAACGTTATC 50
TTAATCGCAC TATGCCGGTA GGCTTGCCGG GTCTGGCAGC TGCCTGTTTT 100
<210> 3193
<211> 100
<212> DNA
<213> Escherichia coli K-12 MG1655
<220> yije b3944
<222> 840-939
<223> yije b3944 U00096 4133655..4134593
<313> 3193: 897-916
<400> 3193
TCCGGGGGCC GTTGAAGGTA GCGGTATTGT GCTGATTGTG CTGGCACTGG 50
CGCTGGTGAG CCGTAAGAAA AAAGAAGCCG TCAGTGTAAA AAGGATCTGA 100
<210> 3194
<211> 100
<212> DNA
<213> Escherichia coli K-12 MG1655
<220> yije b3944
<222> 790-889
<223> yije b3944 U00096 4133655..4134593
<313> 3194: 848-867
<400> 3194
GCCGTTCCCG TTTGCGGCGT ACTCTTTTCC TGGTGGCTGC TCGGCGAGAA 50
TCCGGGGGCC GTTGAAGGTA GCGGTATTGT GCTGATTGTG CTGGCACTGG 100
<210> 3195
<211> 100
<212> DNA
<213> Escherichia coli K-12 MG1655
<220> yije b3944
<222> 753-852
<223> yije b3944 U00096 4133655..4134593

<313> 3195: 792-811
<400> 3195
AAACTTGCCT GCCAGTATTG CCAGCTTAAG CACACTGGCC GTTCCCGTTT 50
GCGGCGTACT CTTTTCCTGG TGGCTGCTCG GCGAGAATCC GGGGGCCGTT 100
<210> 3196
<211> 100
<212> DNA
<213> Escherichia coli K-12 MG1655
<220> yijf b3945
<222> 519-618
<223> yijf b3945 U00096 complement(4134620..4135237)
<313> 3196: 586-605
<400> 3196
TACGCCGCTG GTGATACATA ACATTGGCGC AGGCGCGCAG GAAGAAGATG 50
TGCTGTTTAA CTGGCGGATG GTGGGGCATT ATCGTTATTT TGTTAAATAA 100
<210> 3197
<211> 100
<212> DNA
<213> Escherichia coli K-12 MG1655
<220> yijf b3945
<222> 463-562
<223> yijf b3945 U00096 complement(4134620..4135237)
<313> 3197: 503-522
<400> 3197
CTGGATAACG GGCTGGCACA TATTGGGGTG GTTTCAGATG GCTTCGCCCG 50
CGACGGTACG CCGCTGGTGA TACATAACAT TGGCGCAGGC GCGCAGGAAG 100
<210> 3198
<211> 100
<212> DNA
<213> Escherichia coli K-12 MG1655
<220> yijf b3945
<222> 419-518
<223> yijf b3945 U00096 complement(4134620..4135237)
<313> 3198: 450-469
<400> 3198
AGAACCCCAG TGACTATCAA GCGGGCGATA TTGTCTCCTG GCGACTGGAT 50
AACGGGCTGG CACATATTGG GGTGGTTTCA GATGGCTTCG CCCGCGACGG 100
<210> 3199
<211> 100
<212> DNA
<213> Escherichia coli K-12 MG1655
<220> yiji b3949
<222> 231-330
<223> yiji b3949 U00096 4139437..4139766
<313> 3199: 310-329
<400> 3199
GCCTTGCGGT CAGTGCGCAG GTTATGCCAC TCAATTTGTG ATGAAAAAGT 50
ATTACACAGC GTTTCAACGT GGCTTGCCGG ACGCGCATGT ACGCCGTTAG 100
<210> 3200
<211> 100
<212> DNA
<213> Escherichia coli K-12 MG1655
<220> yiji b3949
<222> 92-191
<223> yiji b3949 U00096 4139437..4139766
<313> 3200: 148-167
<400> 3200
CGCAGTGGGG GAATTCATCG CGCAGCCATT GGCTTAAACG CTGGTGCGCT 50
TCCTGTTCGT CGGCCCCGGA AATCAGTAAC TGGCAGTTAT CGCCCGCCAG 100
<210> 3201
<211> 100
<212> DNA
<213> Escherichia coli K-12 MG1655
<220> yiji b3949
<222> 56-155
<223> yiji b3949 U00096 4139437..4139766
<313> 3201: 109-128
<400> 3201
CCAGTTCGTC AGATTTAACT TCCGCCAGCG GCGCATCGCA GTGGGGGAAT 50
TCATCGCGCA GCCATTGGCT TAAACGCTGG TGCGCTTCCT GTTCGTCGGC 100
<210> 3202

<211> 100
<212> DNA
<213> Escherichia coli K-12 MG1655
<220> yijo b3955
<222> 753-852
<223> yijo b3955 U00096 complement(4145045..4145896)
<313> 3202: 820-839
<400> 3202
CTTCTGCCGT CTGTTTCGTA AAAACACCGA ACGCTCGCCG TCGGAATATC 50
GCCGGCAGTA CCACAGCCAA CTGACAGAAA AACCGACTAC TCCAGAATAA 100
<210> 3203
<211> 100
<212> DNA
<213> Escherichia coli K-12 MG1655
<220> yijo b3955
<222> 719-818
<223> yijo b3955 U00096 complement(4145045..4145896)
<313> 3203: 785-804
<400> 3203
TGGCGCACGC CTGCGGCTTT GTCGACAGCA ACTACTTCTG CCGTCTGTTT 50
CGTAAAAACA CCGAACGCTC GCCGTCGGAA TATCGCCGGC AGTACCACAG 100
<210> 3204
<211> 100
<212> DNA
<213> Escherichia coli K-12 MG1655
<220> yijo b3955
<222> 671-770
<223> yijo b3955 U00096 complement(4145045..4145896)
<313> 3204: 714-733
<400> 3204
AGCACGCTAA GACGTTATTG AAAGGCTATG ACCTGAAGGT AAAAGAAGTG 50
GCGCACGCCT GCGGCTTTGT CGACAGCAAC TACTTCTGCC GTCTGTTTCG 100
<210> 3205
<211> 100
<212> DNA
<213> Escherichia coli K-12 MG1655
<220> yijp b3956
<222> 1635-1734
<223> yijp b3956 U00096 complement(4146111..4147844)
<313> 3205: 1698-1717
<400> 3205
GTTCAAAGAA ACTACCCGCT GGATTGGTAA CCCGTATAAG AAAAACGCAC 50
TGATCGATTA CGACACACTG CCCTATGGCG ATCAGGTGGG TAATCAGTAA 100
<210> 3206
<211> 100
<212> DNA
<213> Escherichia coli K-12 MG1655
<220> yijp b3956
<222> 1603-1702
<223> yijp b3956 U00096 complement(4146111..4147844)
<313> 3206: 1658-1677
<400> 3206
TACGATCCAA CCCGTTCAGT GGTGAATCCG CAGTTCAAAG AAACTACCCG 50
CTGGATTGGT AACCCGTATA AGAAAAACGC ACTGATCGAT TACGACACAC 100
<210> 3207
<211> 100
<212> DNA
<213> Escherichia coli K-12 MG1655
<220> yijp b3956
<222> 1569-1668
<223> yijp b3956 U00096 complement(4146111..4147844)
<313> 3207: 1609-1628
<400> 3207
CTGGTCAGAT TTGGCGGGCT TATCTTACGA CGGTTACGAT CCAACCCGTT 50
CAGTGGTGAA TCCGCAGTTC AAAGAAACTA CCCGCTGGAT TGGTAACCCG 100
<210> 3208
<211> 100
<212> DNA
<213> Escherichia coli K-12 MG1655
<220> fdng b1475
<222> 2949-3048

<223> fdng b1475 U00096 1545425..1548472
<313> 3208: 3008-3027
<400> 3208
TAAAGGTTAT ATCGCTAACA CTCTGACGCC GAATGTCGGT GATGCAAACT 50
CGCAAACGCC GGAATATAAA GCGTTCTTAG TCAACATCGA GAAGGCGTAA 100
<210> 3209
<211> 100
<212> DNA
<213> Escherichia coli K-12 MG1655
<220> fdng b1475
<222> 2904-3003
<223> fdng b1475 U00096 1545425..1548472
<313> 3209: 2957-2976
<400> 3209
AACGGTGGGT ATTCCAATCC ACTGGGGCTT TGAGGGTGTC GCGCGTAAAG 50
GTTATATCGC TAACACTCTG ACGCCGAATG TCGGTGATGC AAACTCGCAA 100
<210> 3210
<211> 100
<212> DNA
<213> Escherichia coli K-12 MG1655
<220> fdng b1475
<222> 2870-2969
<223> fdng b1475 U00096 1545425..1548472
<313> 3210: 2896-2915
<400> 3210
TGAAACCGCT GAATGTAAAT GGTCAGCAGG TTGAAACGGT GGGTATTCCA 50
ATCCACTGGG GCTTTGAGGG TGTCGCGCGT AAAGGTTATA TCGCTAACAC 100
<210> 3211
<211> 100
<212> DNA
<213> Escherichia coli K-12 MG1655
<220> fdnh b1476
<222> 786-885
<223> fdnh b1476 U00096 1548485..1549369
<313> 3211: 858-877
<400> 3211
GGCTGGCTTT ATTGCCACTT TTGCCGGGTT GATTTTCCAC TACATCGGTA 50
TTGGCCCGAA TAAGGAAGTG GACGATGACG AGGAGGATCA TCATGAGTAA 100
<210> 3212
<211> 100
<212> DNA
<213> Escherichia coli K-12 MG1655
<220> fdnh b1476
<222> 786-885
<223> fdnh b1476 U00096 1548485..1549369
<313> 3212: 815-834
<400> 3212
GGCTGGCTTT ATTGCCACTT TTGCCGGGTT GATTTTCCAC TACATCGGTA 50
TTGGCCCGAA TAAGGAAGTG GACGATGACG AGGAGGATCA TCATGAGTAA 100
<210> 3213
<211> 100
<212> DNA
<213> Escherichia coli K-12 MG1655
<220> fdnh b1476
<222> 690-789
<223> fdnh b1476 U00096 1548485..1549369
<313> 3213: 734-753
<400> 3213
CGCCGATCAG CCGGAGCTGT ATCACGGTCT GCCGAAAGAT CCGAAGATCG 50
ACACCTCGGT AAGCCTGTGG AAAGGCGCGT TGAAACCGCT GGCAGCGGCT 100
<210> 3214
<211> 100
<212> DNA
<213> Escherichia coli K-12 MG1655
<220> fdni b1477
<222> 555-654
<223> fdni b1477 U00096 1549362..1550015
<313> 3214: 627-646
<400> 3214
AGGGAAGGTA AGTCGTCGCT GGGCGAAGAA ACACCATCCG CGCTGGTATC 50
GTGAAATCGA GAAGGCAGAA GCGAAAAAAG AGAGTGAAGA AGGGATATAA 100

<210> 3215
<211> 100
<212> DNA
<213> Escherichia coli K-12 MG1655
<220> fdni b1477
<222> 555-654
<223> fdni b1477 U00096 1549362..1550015
<313> 3215: 582-601
<400> 3215
AGGGAAGGTA AGTCGTCGCT GGGCGAAGAA ACACCATCCG CGCTGGTATC 50
GTGAAATCGA GAAGGCAGAA GCGAAAAAAG AGAGTGAAGA AGGGATATAA 100
<210> 3216
<211> 100
<212> DNA
<213> Escherichia coli K-12 MG1655
<220> fdni b1477
<222> 428-527
<223> fdni b1477 U00096 1549362..1550015
<313> 3216: 489-508
<400> 3216
TCCCGATGCA GGTTGTTCGC TACAGCCTGC TGATCCACGC GGCTGCGGGT 50
ATCATCCTGA TCCACGCCAT CCTGATCCAT ATGTATATGG CATTTTGGGT 100
<210> 3217
<211> 100
<212> DNA
<213> Escherichia coli K-12 MG1655
<220> gpsa b3609
<222> 836-935
<223> gpsa b3609 U00096 complement(3780269..3781288)
<313> 3217: 883-902
<400> 3217
AGATTGGTCA GGTGGTGGAA GGCTACCGCA ATACGAAAGA AGTCCGCGAA 50
CTGGCGCATC GCTTCGGCGT TGAAATGCCA ATAACCGAGG AAATTTATCA 100
<210> 3218
<211> 100
<212> DNA
<213> Escherichia coli K-12 MG1655
<220> gpsa b3609
<222> 769-868
<223> gpsa b3609 U00096 complement(3780269..3781288)
<313> 3218: 792-811
<400> 3218
CAGTCGCGTA ACCGCCGTTT TGGCATGATG CTCGGTCAGG GCATGGATGT 50
ACAAAGCGCG CAGGAGAAGA TTGGTCAGGT GGTGGAAGGC TACCGCAATA 100
<210> 3219
<211> 100
<212> DNA
<213> Escherichia coli K-12 MG1655
<220> gpsa b3609
<222> 700-799
<223> gpsa b3609 U00096 complement(3780269..3781288)
<313> 3219: 751-770
<400> 3219
GGTGCCGACC CTGCCACCTT TATGGGCATG GCGGGGCTTG GCGATCTGGT 50
GCTTACCTGT ACCGACAACC AGTCGCGTAA CCGCCGTTTT GGCATGATGC 100
<210> 3220
<211> 100
<212> DNA
<213> Escherichia coli K-12 MG1655
<220> sdha b0724
<222> 1668-1767
<223> sdha b0724 U00096 755130..756896
<313> 3220: 1742-1761
<400> 3220
CTCCCTGTAT CTGCCAGAGT CGGAATCCAT GACGCGCCGA AGCGTCAACA 50
TGGAACCGAA ACTGCGCCCG GCATTCCCGC CGAAGATTCG TACTTACTAA 100
<210> 3221
<211> 100
<212> DNA
<213> Escherichia coli K-12 MG1655
<220> sdha b0724

<222> 1638-1737
<223> sdha b0724 U00096 755130..756896
<313> 3221: 1689-1708
<400> 3221
GGATCGTGAT GATGAAAACT GGCTGTGCCA CTCCCTGTAT CTGCCAGAGT 50
CGGAATCCAT GACGCGCCGA AGCGTCAACA TGGAACCGAA ACTGCGCCCG 100
<210> 3222
<211> 100
<212> DNA
<213> Escherichia coli K-12 MG1655
<220> sdha b0724
<222> 1506-1605
<223> sdha b0724 U00096 755130..756896
<313> 3222: 1536-1555
<400> 3222
TTCCAGCGAG TTCAACACCC AGCGCGTTGA GTGCCTGGAA CTGGATAACC 50
TGATGGAAAC GGCGTATGCA ACGGCTGTTT CTGCCAACTT CCGTACCGAA 100
<210> 3223
<211> 100
<212> DNA
<213> Escherichia coli K-12 MG1655
<220> fdog b3895
<222> 2952-3051
<223> fdog b3895 U00096 complement(4080352..4083402)
<313> 3223: 3025-3044
<400> 3223
AAAAGGCTTT ATTGCCAATA CTTTAACGCC ATTCGTTGGT GATGCGAACA 50
CGCAGACGCC GGAGTTTAAG TCCTTCCTTG TGAATGTGGA AAAGGTGTAA 100
<210> 3224
<211> 100
<212> DNA
<213> Escherichia coli K-12 MG1655
<220> fdog b3895
<222> 2858-2957
<223> fdog b3895 U00096 complement(4080352..4083402)
<313> 3224: 2918-2937
<400> 3224
TGGTGACCAA ACGTATTCGC ACGCTGAAAG CGAACGGCAA AGATATCGAT 50
ACCATCGGTA TTCCGATTCA CTGGGGCTAT GAAGGCGTAG CTAAAAAAGG 100
<210> 3225
<211> 100
<212> DNA
<213> Escherichia coli K-12 MG1655
<220> fdog b3895
<222> 2846-2945
<223> fdog b3895 U00096 complement(4080352..4083402)
<313> 3225: 2883-2902
<400> 3225
AAGCCAAAGC GGTGGTGACC AAACGTATTC GCACGCTGAA AGCGAACGGC 50
AAAGATATCG ATACCATCGG TATTCCGATT CACTGGGGCT ATGAAGGCGT 100
<210> 3226
<211> 100
<212> DNA
<213> Escherichia coli K-12 MG1655
<220> sdhb sdhB
<222> 613-712
<223> sdhb sdhB U00096 756912..757628
<313> 3226: 639-658
<400> 3226
CGCTGTCACA GCATCATGAA CTGCGTCAGT GTATGTCCGA AGGGGCTGAA 50
CCCGACGCGC GCCATCGGCC ATATCAAGTC GATGTTGTTG CAACGTAATG 100
<210> 3227
<211> 100
<212> DNA
<213> Escherichia coli K-12 MG1655
<220> sdhb sdhB
<222> 522-621
<223> sdhb sdhB U00096 756912..757628
<313> 3227: 563-582
<400> 3227
CTTGTTAGCG GCATATCGTT TCCTGATTGA TAGCCGTGAT ACCGAGACTG 50

ACAGCCGCCT CGACGGTTTG AGTGATGCAT TCAGCGTATT CCGCTGTCAC 100
<210> 3228
<211> 100
<212> DNA
<213> Escherichia coli K-12 MG1655
<220> sdhb sdhB
<222> 446-545
<223> sdhb sdhB U00096 756912..757628
<313> 3228: 473-492
<400> 3228
GTATTCTCTG CGCATGTTGT TCAACCTCTT GTCCGTCTTT CTGGTGGAAT 50
CCCGATAAGT TTATCGGCCC GGCAGGCTTG TTAGCGGCAT ATCGTTTCCT 100
<210> 3229
<211> 100
<212> DNA
<213> Escherichia coli K-12 MG1655
<220> fdoh b3894
<222> 804-903
<223> fdoh b3894 U00096 complement(4079437..4080339)
<313> 3229: 878-897
<400> 3229
CTTTGCGGCC AGTATCTTCC ACTACGTGGG TGTCGGTCCG AACCGTGCGG 50
ATGAGGAAGA GAATAATCTG CACGAAGAGA AAGACGAGGA GCGCAAATGA 100
<210> 3230
<211> 100
<212> DNA
<213> Escherichia coli K-12 MG1655
<220> fdoh b3894
<222> 779-878
<223> fdoh b3894 U00096 complement(4079437..4080339)
<313> 3230: 791-810
<400> 3230
TCGCGGCTGT TGGCTTTGCG GCTACCTTTG CGGCCAGTAT CTTCCACTAC 50
GTGGGTGTCG GTCCGAACCG TGCGGATGAG GAAGAGAATA ATCTGCACGA 100
<210> 3231
<211> 100
<212> DNA
<213> Escherichia coli K-12 MG1655
<220> fdoh b3894
<222> 674-773
<223> fdoh b3894 U00096 complement(4079437..4080339)
<313> 3231: 729-748
<400> 3231
TGTACGTGCT GCACCATGCT GACAAGCCAA ATCTGTATCA TGGCTTGCCG 50
GAGAACCCGG AAATCAGCGA AACCGTGAAA TTCTGGAAAG GCATCTGGAA 100
<210> 3232
<211> 100
<212> DNA
<213> Escherichia coli K-12 MG1655
<220> sdhc b0722
<222> 291-390
<223> sdhc b0722 U00096 754400..754789
<313> 3232: 360-379
<400> 3232
CTATCTGGAA GAAACATTCG AAGCGGGTAA ACGCTCCGCC AAAATCTCCT 50
TTGTTATTAC TGTCGTGCTT TCACTTCTCG CAGGAGTCCT CGTATGGTAA 100
<210> 3233
<211> 100
<212> DNA
<213> Escherichia coli K-12 MG1655
<220> sdhc b0722
<222> 285-384
<223> sdhc b0722 U00096 754400..754789
<313> 3233: 318-337
<400> 3233
TTTTGGCTAT CTGGAAGAAA CATTCGAAGC GGGTAAACGC TCCGCCAAAA 50
TCTCCTTTGT TATTACTGTC GTGCTTTCAC TTCTCGCAGG AGTCCTCGTA 100
<210> 3234
<211> 100
<212> DNA
<213> Escherichia coli K-12 MG1655

<220> sdhc b0722
<222> 222-321
<223> sdhc b0722 U00096 754400..754789
<313> 3234: 285-304
<400> 3234
GTGGGGCATC CTTACCGCTC TGGCGTATCA CGTCGTCGTA GGTATTCGCC  50
ACATGATGAT GGATTTTGGC TATCTGGAAG AAACATTCGA AGCGGGTAAA  100
<210> 3235
<211> 100
<212> DNA
<213> Escherichia coli K-12 MG1655
<220> fdoi b3893
<222> 537-636
<223> fdoi b3893 U00096 complement(4078805..4079440)
<313> 3235: 616-635
<400> 3235
CGCCATGGTG GAAGGATGGG TTACCAGCGC CTGGGCGAAG AAACATCACC  50
CGCGCTGGTA CCGTGAGGTC CGCAAGACAA CGGAAAAGAA AGCTGAATGA  100
<210> 3236
<211> 100
<212> DNA
<213> Escherichia coli K-12 MG1655
<220> fdoi b3893
<222> 454-553
<223> fdoi b3893 U00096 complement(4078805..4079440)
<313> 3236: 505-524
<400> 3236
CTGCATTCAT TTGCCGCAGT GGCGTTAATT GTGGTTATCA TGGTGCATAT  50
CTACGCCGCC CTTTGGGTGA AAGGCACGAT TACCGCCATG GTGGAAGGAT  100
<210> 3237
<211> 100
<212> DNA
<213> Escherichia coli K-12 MG1655
<220> fdoi b3893
<222> 378-477
<223> fdoi b3893 U00096 complement(4078805..4079440)
<313> 3237: 416-435
<400> 3237
GGTGAGCGGC GTGATTATCT GGCGTCCTTA TTTTGCGCCT GCTTTCTCAA  50
TCCCGGTGAT CCGATTCGCG TTAATGCTGC ATTCATTTGC CGCAGTGGCG  100
<210> 3238
<211> 100
<212> DNA
<213> Escherichia coli K-12 MG1655
<220> sdhd b0723
<222> 220-319
<223> sdhd b0723 U00096 754783..755130
<313> 3238: 262-281
<400> 3238
ATCGGCATGT GGCAGGTGTT GACCGACTAC GTTAAACCGC TGGCTTTGCG  50
CCTGATGCTG CAACTGGTGA TTGTCGTTGC ACTGGTGGTT TACGTGATTT  100
<210> 3239
<211> 100
<212> DNA
<213> Escherichia coli K-12 MG1655
<220> sdhd b0723
<222> 190-289
<223> sdhd b0723 U00096 754783..755130
<313> 3239: 226-245
<400> 3239
GCGCTGTTTT CTATCTTGAT CCATGCCTGG ATCGGCATGT GGCAGGTGTT  50
GACCGACTAC GTTAAACCGC TGGCTTTGCG CCTGATGCTG CAACTGGTGA  100
<210> 3240
<211> 100
<212> DNA
<213> Escherichia coli K-12 MG1655
<220> sdhd b0723
<222> 130-229
<223> sdhd b0723 U00096 754783..755130
<313> 3240: 159-178
<400> 3240

ACATATGAAG TCTGGATCGG TTTCTTCGCC TCTGCGTTCA CCAAAGTGTT 50
CACCCTGCTG GCGCTGTTTT CTATCTTGAT CCATGCCTGG ATCGGCATGT 100
<210> 3241
<211> 100
<212> DNA
<213> Escherichia coli K-12 MG1655
<220> icia b2917
<222> 795-894
<223> icia b2917 U00096 3057773..3058666
<313> 3241: 833-852
<400> 3241
GATGCTCTAC TGGCACCGCT TTGCTCCTGA AAGCCGCATG ATGCGTAAAG 50
TCACTGATGC GTTACTCGAT TATGGTCACA AAGTCCTTCG TCAGGATTAA 100
<210> 3242
<211> 100
<212> DNA
<213> Escherichia coli K-12 MG1655
<220> icia b2917
<222> 699-798
<223> icia b2917 U00096 3057773..3058666
<313> 3242: 727-746
<400> 3242
GGGCACCACC TGCTGTATGA TCCCGCACCT GCAAATCGAG AAAGAGCTGG 50
CCAGCGGTGA ACTGATTGAC TTAACGCCTG GGCTATTTCA ACGACGGATG 100
<210> 3243
<211> 100
<212> DNA
<213> Escherichia coli K-12 MG1655
<220> icia b2917
<222> 645-744
<223> icia b2917 U00096 3057773..3058666
<313> 3243: 689-708
<400> 3243
CGTGCCCTGC CATATCGTTA ATTCTTCAGA AGCGTTCGTA CAACTTGCTC 50
GCCAGGGCAC CACCTGCTGT ATGATCCCGC ACCTGCAAAT CGAGAAAGAG 100
<210> 3244
<211> 100
<212> DNA
<213> Escherichia coli K-12 MG1655
<220> sdia b1917
<222> 624-723
<223> sdia b1917 U00096 complement(1994133..1994855)
<313> 3244: 683-702
<400> 3244
GAATACGGTC AATTTCCACC AGAAAAACAT GCAGAAAAAA ATTAATGCAC 50
CAAATAAGAC CCAGGTTGCC TGTTACGCGG CCGCTACTGG CTTAATTTGA 100
<210> 3245
<211> 100
<212> DNA
<213> Escherichia coli K-12 MG1655
<220> sdia b1917
<222> 539-638
<223> sdia b1917 U00096 complement(1994133..1994855)
<313> 3245: 573-592
<400> 3245
TCAGCAAGCG CGAAAAAGAA ATTCTGAGGT GGACGGCGGA AGGGAAAACA 50
TCAGCAGAGA TAGCGATGAT TTTGTCAATC TCTGAGAATA CGGTCAATTT 100
<210> 3246
<211> 100
<212> DNA
<213> Escherichia coli K-12 MG1655
<220> sdia b1917
<222> 499-598
<223> sdia b1917 U00096 complement(1994133..1994855)
<313> 3246: 522-541
<400> 3246
CGTTTAAATG ATGAAATAGT GATGACGCCA GAGATGAATT TCAGCAAGCG 50
CGAAAAAGAA ATTCTGAGGT GGACGGCGGA AGGGAAAACA TCAGCAGAGA 100
<210> 3247
<211> 100
<212> DNA

<213> Escherichia coli K-12 MG1655
<220> tala b2465
<222> 848-947
<223> tala b2465 U00096 2576686..2577636
<313> 3247: 886-905
<400> 3247
AGCACAATCA GGATGCGATG GCGGTAGAAA AACTGTCTGA AGGCATTCGT 50
CTGTTCGCCG TTGATCAACG CAAACTGGAA GATCTTCTTG CCGCCAAACT 100
<210> 3248
<211> 100
<212> DNA
<213> Escherichia coli K-12 MG1655
<220> tala b2465
<222> 752-851
<223> tala b2465 U00096 2576686..2577636
<313> 3248: 816-835
<400> 3248
TGCAGGAAAA AGTTTCGCCA GTGGTACGTA AATTAATCCC ACCTTCTCAG 50
ACGTTCCCAC GCCCAGCTCC CATGAGCGAA GCGGAGTTCC GTTGGGAGCA 100
<210> 3249
<211> 100
<212> DNA
<213> Escherichia coli K-12 MG1655
<220> tala b2465
<222> 690-789
<223> tala b2465 U00096 2576686..2577636
<313> 3249: 727-746
<400> 3249
ACAAATCCTC GCCTTAACCG GCTGCGATCG ACTGACTATC GCACCGAATT 50
TACTGAAGGA GCTGCAGGAA AAAGTTTCGC CAGTGGTACG TAAATTAATC 100
<210> 3250
<211> 100
<212> DNA
<213> Escherichia coli K-12 MG1655
<220> talb b0009
<222> 855-954
<223> talb b0009 U00096 8238..9191
<313> 3250: 933-952
<400> 3250
CAACCAGGAT CCAATGGCAG TAGATAAACT GGCGGAAGGT ATCCGTAAGT 50
TTGCTATTGA CCAGGAAAAA CTGGAAAAAA TGATCGGCGA TCTGCTGTAA 100
<210> 3251
<211> 100
<212> DNA
<213> Escherichia coli K-12 MG1655
<220> talb b0009
<222> 846-945
<223> talb b0009 U00096 8238..9191
<313> 3251: 874-893
<400> 3251
GTGGCAGCAC AACCAGGATC CAATGGCAGT AGATAAACTG GCGGAAGGTA 50
TCCGTAAGTT TGCTATTGAC CAGGAAAAAC TGGAAAAAAT GATCGGCGAT 100
<210> 3252
<211> 100
<212> DNA
<213> Escherichia coli K-12 MG1655
<220> talb b0009
<222> 784-883
<223> talb b0009 U00096 8238..9191
<313> 3252: 835-854
<400> 3252
AAACTGTCTT ACACCGGCGA AGTGAAAGCG CGTCCGGCGC GTATCACTGA 50
GTCCGAGTTC CTGTGGCAGC ACAACCAGGA TCCAATGGCA GTAGATAAAC 100
<210> 3253
<211> 100
<212> DNA
<213> Escherichia coli K-12 MG1655
<220> talc b3947
<222> 564-663
<223> talc b3947 U00096 complement(4136626..4137288)
<313> 3253: 628-647

<400> 3253
AGATGTAGCG CAACAAATGC TCAACACCCC TGCGGTAGAG TCAGCTATAG 50
AGAAGTTCGA ACACGACTGG AATGCCGCAT TTGGCACTAC TCATCTCTAA 100
<210> 3254
<211> 100
<212> DNA
<213> Escherichia coli K-12 MG1655
<220> talc b3947
<222> 550-649
<223> talc b3947 U00096 complement(4136626..4137288)
<313> 3254: 590-609
<400> 3254
ATCACCCTGC CCTTAGATGT AGCGCAACAA ATGCTCAACA CCCCTGCGGT 50
AGAGTCAGCT ATAGAGAAGT TCGAACACGA CTGGAATGCC GCATTTGGCA 100
<210> 3255
<211> 100
<212> DNA
<213> Escherichia coli K-12 MG1655
<220> talc b3947
<222> 498-597
<223> talc b3947 U00096 complement(4136626..4137288)
<313> 3255: 559-578
<400> 3255
CTTTAAAACG CCGCGTCAGG CGCTGGACTG TTTACTGGCA GGATGTGAAT 50
CCATCACCCT GCCCTTAGAT GTAGCGCAAC AAATGCTCAA CACCCCTGCG 100
<210> 3256
<211> 100
<212> DNA
<213> Escherichia coli K-12 MG1655
<220> pepa b4261
<222> 1343-1442
<223> pepa b4261 U00096 complement(4482008..4483519)
<313> 3256: 1387-1406
<400> 3256
TTACCGCAGG TTGCTTCCTG TCACGCTTTA CCCGTAAGTA CAACTGGGCG 50
CACCTGGATA TCGCCGGTAC CGCCTGGCGT TCTGGTAAAG CAAAAGGCGC 100
<210> 3257
<211> 100
<212> DNA
<213> Escherichia coli K-12 MG1655
<220> pepa b4261
<222> 1231-1330
<223> pepa b4261 U00096 complement(4482008..4483519)
<313> 3257: 1281-1300
<400> 3257
GAACAATCCG GTGACCGCGC ATGGCGCTTA CCGCTGGGTG ACGAGTATCA 50
GGAACAACTG GAGTCCAATT TTGCCGATAT GGCGAACATT GGCGGTCGTC 100
<210> 3258
<211> 100
<212> DNA
<213> Escherichia coli K-12 MG1655
<220> pepa b4261
<222> 1207-1306
<223> pepa b4261 U00096 complement(4482008..4483519)
<313> 3258: 1240-1259
<400> 3258
GCCCATGAAC TGATTGCCGC GTCTGAACAA TCCGGTGACC GCGCATGGCG 50
CTTACCGCTG GGTGACGAGT ATCAGGAACA ACTGGAGTCC AATTTTGCCG 100
<210> 3259
<211> 100
<212> DNA
<213> Escherichia coli K-12 MG1655
<220> pepb b2524
<222> 1223-1322
<223> pepb b2524 U00096 complement(2653095..2654465)
<313> 3259: 1268-1287
<400> 3259
GCTTCCTGTC GCACTTTGTT GAGAACTATC AGCAAGGCTG GCTGCATATC 50
GACTGCTCGG CGACTTACCG TAAAGCGCCG GTTGAACAGT GGTCTGCGGG 100
<210> 3260
<211> 100

<212> DNA
<213> Escherichia coli K-12 MG1655
<220> pepb b2524
<222> 1155-1254
<223> pepb b2524 U00096 complement(2653095..2654465)
<313> 3260: 1198-1217
<400> 3260
GTCTAACTTT GCCGAACTGA ACAATACCGG AAGCGCGGCG TATCCGGCAG 50
GCGCGAGCAC GGCGGCGGGC TTCCTGTCGC ACTTTGTTGA GAACTATCAG 100
<210> 3261
<211> 100
<212> DNA
<213> Escherichia coli K-12 MG1655
<220> pepb b2524
<222> 1121-1220
<223> pepb b2524 U00096 complement(2653095..2654465)
<313> 3261: 1158-1177
<400> 3261
TGCCGCTGGC GGAGTTCCAC CGCAGCCAGC TGCCGTCTAA CTTTGCCGAA 50
CTGAACAATA CCGGAAGCGC GGCGTATCCG GCAGGCGCGA GCACGGCGGC 100
<210> 3262
<211> 100
<212> DNA
<213> Escherichia coli K-12 MG1655
<220> baca b3058
<222> 715-814
<223> baca b3058 U00096 complement(3200951..3201772)
<313> 3262: 765-784
<400> 3262
CTGATAGCGA TTAAAACCTT CCTGCAATTG ATTAAGCGCA TTTCGTTTAT 50
CCCGTTCGCC ATTTATCGCT TTATTGTGGC GGCTGCGGTG TATGTCGTGT 100
<210> 3263
<211> 100
<212> DNA
<213> Escherichia coli K-12 MG1655
<220> baca b3058
<222> 523-622
<223> baca b3058 U00096 complement(3200951..3201772)
<313> 3263: 565-584
<400> 3263
TCCGGGGCGA CCATTTCAGG TGGGATGCTG ATGGGGGTGA GCCGTTACGC 50
TGCTTCCGAG TTTTCGTTCC TGCTGGCGGT GCCGATGATG ATGGGCGCAA 100
<210> 3264
<211> 100
<212> DNA
<213> Escherichia coli K-12 MG1655
<220> baca b3058
<222> 435-534
<223> baca b3058 U00096 complement(3200951..3201772)
<313> 3264: 461-480
<400> 3264
TGCGCCGGGT CTTGATGATA TGACCTATCG TCAGGCATTT ATGATTGGCT 50
GTTTCCAGTG TCTGGCGCTG TGGCCGGGTT TCTCCCGTTC CGGGGCGACC 100
<210> 3265
<211> 100
<212> DNA
<213> Escherichia coli K-12 MG1655
<220> pepd b0238
<222> 1359-1458
<223> pepd b0238 U00096 complement(254259..255716)
<313> 3265: 1430-1449
<400> 3265
CACCGGTCCA CACTCTCCGG ATGAGCAAGT TCACATCGAA AGCGTAGGTC 50
ATTACTGGAC ACTGCTGACT GAACTGCTGA AAGAAATTCC GGCGAAGTAA 100
<210> 3266
<211> 100
<212> DNA
<213> Escherichia coli K-12 MG1655
<220> pepd b0238
<222> 1359-1458
<223> pepd b0238 U00096 complement(254259..255716)

<313> 3266: 1388-1407
<400> 3266
CACCGGTCCA CACTCTCCGG ATGAGCAAGT TCACATCGAA AGCGTAGGTC 50
ATTACTGGAC ACTGCTGACT GAACTGCTGA AAGAAATTCC GGCGAAGTAA 100
<210> 3267
<211> 100
<212> DNA
<213> Escherichia coli K-12 MG1655
<220> pepd b0238
<222> 1241-1340
<223> pepd b0238 U00096 complement(254259..255716)
<313> 3267: 1271-1290
<400> 3267
CCTATCAGCG CCTGTTCAAC AAGACGCCGA ACATCCAGAT TATCCACGCG 50
GGCCTGGAAT GTGGTCTGTT CAAAAAACCG TATCCGGAAA TGGACATGGT 100
<210> 3268
<211> 100
<212> DNA
<213> Escherichia coli K-12 MG1655
<220> pepe b4022
<222> 591-690
<223> pepe b4022 U00096 complement(4227032..4227721)
<313> 3268: 655-674
<400> 3268
AGTGAGTAAA GGTCACGCTA CGCTGGGTGG CCCGAACACC ACTTATGTGT 50
TTAAGGCTGG TGAAGAAGCG GTTCCGCTGG AAGCTGGTCA CCGTTTTTAA 100
<210> 3269
<211> 100
<212> DNA
<213> Escherichia coli K-12 MG1655
<220> pepe b4022
<222> 566-665
<223> pepe b4022 U00096 complement(4227032..4227721)
<313> 3269: 592-611
<400> 3269
GTCTACCGGA AGGTAACTGG ATCACAGTGA GTAAAGGTCA CGCTACGCTG 50
GGTGGCCCGA ACACCACTTA TGTGTTTAAG GCTGGTGAAG AAGCGGTTCC 100
<210> 3270
<211> 100
<212> DNA
<213> Escherichia coli K-12 MG1655
<220> pepe b4022
<222> 522-621
<223> pepe b4022 U00096 complement(4227032..4227721)
<313> 3270: 544-563
<400> 3270
TATTCGCGAA CTGCTGGTCG TCGCGCCAGA ACTGACGATT ATTGGTCTAC 50
CGGAAGGTAA CTGGATCACA GTGAGTAAAG GTCACGCTAC GCTGGGTGGC 100
<210> 3271
<211> 100
<212> DNA
<213> Escherichia coli K-12 MG1655
<220> pepn b0933
<222> 2514-2613
<223> pepn b0933 U00096 989845..992457
<313> 3271: 2583-2602
<400> 3271
CAAACGTCAG GAGAAAATGC GCGCGGCGCT GGAACAGTTG AAAGGGCTGG 50
AAAATCTCTC TGGCGATCTG TACGAGAAGA TAACTAAAGC ACTGGCTTGA 100
<210> 3272
<211> 100
<212> DNA
<213> Escherichia coli K-12 MG1655
<220> pepn b0933
<222> 2464-2563
<223> pepn b0933 U00096 989845..992457
<313> 3272: 2501-2520
<400> 3272
GTGGCTTCAC GTCTGATTGA ACCGCTGATT CGCCTGAAAC GTTACGATGC 50
CAAACGTCAG GAGAAAATGC GCGCGGCGCT GGAACAGTTG AAAGGGCTGG 100
<210> 3273

<211> 100
<212> DNA
<213> Escherichia coli K-12 MG1655
<220> pepn b0933
<222> 2441-2540
<223> pepn b0933 U00096 989845..992457
<313> 3273: 2456-2475
<400> 3273
ACCTCAACAG CCGTAACCCG CAGGTGGCTT CACGTCTGAT TGAACCGCTG 50
ATTCGCCTGA AACGTTACGA TGCCAAACGT CAGGAGAAAA TGCGCGCGGC 100
<210> 3274
<211> 100
<212> DNA
<213> Escherichia coli K-12 MG1655
<220> pepp b2909
<222> 1227-1326
<223> pepp b2909 U00096 complement(3051535..3052860)
<313> 3274: 1302-1321
<400> 3274
CATTGTGATT ACCGAAACCG GTAACGAAAA CCTCACCGCC AGCGTGGTGA 50
AAAAGCCGGA AGAAATCGAA GCGTTGATGG TTGCTGCGAG AAAGCAATGA 100
<210> 3275
<211> 100
<212> DNA
<213> Escherichia coli K-12 MG1655
<220> pepp b2909
<222> 1150-1249
<223> pepp b2909 U00096 complement(3051535..3052860)
<313> 3275: 1172-1191
<400> 3275
GAGCCAGGGC TGTATATTGC GCCGGATGCA GAAGTGCCAG AACAATATCG 50
CGGTATCGGC ATTCGTATTG AAGACGACAT TGTGATTACC GAAACCGGTA 100
<210> 3276
<211> 100
<212> DNA
<213> Escherichia coli K-12 MG1655
<220> pepp b2909
<222> 970-1069
<223> pepp b2909 U00096 complement(3051535..3052860)
<313> 3276: 1017-1036
<400> 3276
AGCGGCCTGG TAAAACTCGG CATCCTGAAA GGTGATGTTG ATGAACTGAT 50
CGCTCAGAAC GCCCATCGTC CTTTCTTTAT GCATGGCCTT AGCCACTGGT 100
<210> 3277
<211> 100
<212> DNA
<213> Escherichia coli K-12 MG1655
<220> gida b3742
<222> 1791-1890
<223> gida b3742 U00096 complement(3921372..3923261)
<313> 3277: 1838-1857
<400> 3277
TATCGGCCAA GCTTCGCGTA TTTCTGGCGT CACGCCTGCG GCCATCTCCA 50
TTCTGCTGGT GTGGCTGAAA AAACAGGGTA TGCTGCGTCG TAGCGCATAA 100
<210> 3278
<211> 100
<212> DNA
<213> Escherichia coli K-12 MG1655
<220> gida b3742
<222> 1739-1838
<223> gida b3742 U00096 complement(3921372..3923261)
<313> 3278: 1798-1817
<400> 3278
CCGGTCTTTC TAACGAAGTG ATCGCCAAAC TTAACGATCA CAAACCAGCC 50
TCTATCGGCC AAGCTTCGCG TATTTCTGGC GTCACGCCTG CGGCCATCTC 100
<210> 3279
<211> 100
<212> DNA
<213> Escherichia coli K-12 MG1655
<220> gida b3742
<222> 1696-1795

<223> gida b3742 U00096 complement(3921372..3923261)
<313> 3279: 1725-1744
<400> 3279
GAGAACACCC TGCTACCCGC GACACTGGAT TACCGCCAGG TATCCGGTCT 50
TTCTAACGAA GTGATCGCCA AACTTAACGA TCACAAACCA GCCTCTATCG 100
<210> 3280
<211> 100
<212> DNA
<213> Escherichia coli K-12 MG1655
<220> pepq b3848
<222> 1233-1332
<223> pepq b3848 U00096 4028751..4030082
<313> 3280: 1260-1279
<400> 3280
TGAAGCACTG AAACCGTTCG GCGGCATTCG TATCGAAGAC AACGTGGTGA 50
TCCACGAAAA CAACGTGGAA AACATGACCC GGGATCTGAA ACTGGCGTGA 100
<210> 3281
<211> 100
<212> DNA
<213> Escherichia coli K-12 MG1655
<220> pepq b3848
<222> 1183-1282
<223> pepq b3848 U00096 4028751..4030082
<313> 3281: 1234-1253
<400> 3281
GCACCGTGGC GTGAAGGGCA GTTCAGCAAG CACTTCAACT GGCAGAAAAT 50
TGAAGCACTG AAACCGTTCG GCGGCATTCG TATCGAAGAC AACGTGGTGA 100
<210> 3282
<211> 100
<212> DNA
<213> Escherichia coli K-12 MG1655
<220> pepq b3848
<222> 1140-1239
<223> pepq b3848 U00096 4028751..4030082
<313> 3282: 1180-1199
<400> 3282
GTTAACCATC GAACCGGGTA TCTACTTCAT CGAATCGCTA CTGGCACCGT 50
GGCGTGAAGG GCAGTTCAGC AAGCACTTCA ACTGGCAGAA AATTGAAGCA 100
<210> 3283
<211> 100
<212> DNA
<213> Escherichia coli K-12 MG1655
<220> gidb b3741
<222> 524-623
<223> gidb b3741 U00096 complement(3920685..3921308)
<313> 3283: 562-581
<400> 3283
TGTTGCCCGA AGAATATCAG GTCGAATCAG TGGTTAAACT TCAGGTTCCA 50
GCCCTGGATG GCGAACGTCA TCTGGTGGTG ATTAAAGCAA ATAAAATTTA 100
<210> 3284
<211> 100
<212> DNA
<213> Escherichia coli K-12 MG1655
<220> gidb b3741
<222> 495-594
<223> gidb b3741 U00096 complement(3920685..3921308)
<313> 3284: 521-540
<400> 3284
AGGGCAAATG CCGGAAGATG AAATCGCTTT GTTGCCCGAA GAATATCAGG 50
TCGAATCAGT GGTTAAACTT CAGGTTCCAG CCCTGGATGG CGAACGTCAT 100
<210> 3285
<211> 100
<212> DNA
<213> Escherichia coli K-12 MG1655
<220> gidb b3741
<222> 433-532
<223> gidb b3741 U00096 complement(3920685..3921308)
<313> 3285: 486-505
<400> 3285
AACGATATGG TGAGCTGGTG CCACCATCTT CCTGGTGAGC AAGGCCGTTT 50
CTACGCGCTG AAAGGGCAAA TGCCGGAAGA TGAAATCGCT TTGTTGCCCG 100

<210> 3286
<211> 100
<212> DNA
<213> Escherichia coli K-12 MG1655
<220> ptrb b1846
<222> 1873-1972
<223> ptrb b1846 U00096 complement(1924803..1926863)
<313> 3286: 1929-1948
<400> 3286
CCGGCAAAAT GGGTCGCTAA ATTGCGCGAG CTGAAAACCG ATGACCATCT 50
TTTATTGCTC TGTACCGACA TGGACTCAGG CCATGGCGGT AAATCTGGTC 100
<210> 3287
<211> 100
<212> DNA
<213> Escherichia coli K-12 MG1655
<220> ptrb b1846
<222> 1778-1877
<223> ptrb b1846 U00096 complement(1924803..1926863)
<313> 3287: 1808-1827
<400> 3287
AAAGCTACAG CCCGTATGAC AACGTCACCG CACAGGCTTA TCCGCATTTA 50
CTGGTAACGA CCGGTTTGCA CGATTCTCAG GTGCAATATT GGGAACCGGC 100
<210> 3288
<211> 100
<212> DNA
<213> Escherichia coli K-12 MG1655
<220> ptrb b1846
<222> 1540-1639
<223> ptrb b1846 U00096 complement(1924803..1926863)
<313> 3288: 1596-1615
<400> 3288
GCATTGTTAA AACTGGGCTA TGGCTCTCCT TCGCTTTGTT ATGCGATGGG 50
CGGGAGTGCG GGGGGCATGT TGATGGGCGT TGCAATTAAT CAACGCCCGG 100
<210> 3289
<211> 100
<212> DNA
<213> Escherichia coli K-12 MG1655
<220> pept b1128
<222> 1128-1227
<223> pept b1128 U00096 1185067..1186293
<313> 3289: 1192-1211
<400> 3289
CAACTATCAT GGTAAGCATG AGTTTGTGAC TCTGGAAGGT ATGGAAAAAG 50
CGGTGCAGGT GATCGTCCGT ATTGCCGAGT TAACGGCGCA ACGGAAGTAA 100
<210> 3290
<211> 100
<212> DNA
<213> Escherichia coli K-12 MG1655
<220> pept b1128
<222> 1097-1196
<223> pept b1128 U00096 1185067..1186293
<313> 3290: 1147-1166
<400> 3290
TACCGTGCCC GAACCTGTTC ACTGGCGGTT ACAACTATCA TGGTAAGCAT 50
GAGTTTGTGA CTCTGGAAGG TATGGAAAAA GCGGTGCAGG TGATCGTCCG 100
<210> 3291
<211> 100
<212> DNA
<213> Escherichia coli K-12 MG1655
<220> pept b1128
<222> 1063-1162
<223> pept b1128 U00096 1185067..1186293
<313> 3291: 1109-1128
<400> 3291
GGTACCGACG GCGCGCAGTT GTCGTTTATG GGATTACCGT GCCCGAACCT 50
GTTCACTGGC GGTTACAACT ATCATGGTAA GCATGAGTTT GTGACTCTGG 100
<210> 3292
<211> 100
<212> DNA
<213> Escherichia coli K-12 MG1655
<220> gata b2095

<222> 291-390
<223> gata b2095 U00096 complement(2172617..2173069)
<313> 3292: 335-354
<400> 3292
GGTTATTGCG TTGATTGTGG AAAATCCGCA GCAGCAATTG AAACTTTTAC 50
GCTGTTTATT TGGCAAGTTA CAACAGCCCG ATATCGTCGA GACACTAATC 100
<210> 3293
<211> 100
<212> DNA
<213> Escherichia coli K-12 MG1655
<220> gata b2095
<222> 220-319
<223> gata b2095 U00096 complement(2172617..2173069)
<313> 3293: 264-283
<400> 3293
TATCTGTTAA GGCCAACAAA TAAAGTTCAT TTTCAGCAAG CGGATGATGA 50
TAACGACGTG GCGGTATCGT TGGTTATTGC GTTGATTGTG GAAAATCCGC 100
<210> 3294
<211> 100
<212> DNA
<213> Escherichia coli K-12 MG1655
<220> gata b2095
<222> 80-179
<223> gata b2095 U00096 complement(2172617..2173069)
<313> 3294: 136-155
<400> 3294
TCGCCAAAGG TGTGGTTCAT GATACCTGGC CACAGGCATT AATTGCCAGA 50
GAAGCAGAAT TCCCTACCGG GATAATGCTT GAGCAGCACG CTATTGCAAT 100
<210> 3295
<211> 100
<212> DNA
<213> Escherichia coli K-12 MG1655
<220> gatb b2094
<222> 168-267
<223> gatb b2094 U00096 complement(2172302..2172586)
<313> 3295: 187-206
<400> 3295
CACTGCCAAA GTGGATCGTA GTTTTGGCGA TATTCCGTTA GTTCACGGCA 50
TGCCTTTTAT TTCTGGTATC GGTATCGAAG CATTACAAAA TAAAATTCTG 100
<210> 3296
<211> 100
<212> DNA
<213> Escherichia coli K-12 MG1655
<220> gatb b2094
<222> 119-218
<223> gatb b2094 U00096 complement(2172302..2172586)
<313> 3296: 169-188
<400> 3296
GGGTTAATGA AATAGAAACC TATATGGATG GTGTGCATTT GATATGCACC 50
ACTGCCAAAG TGGATCGTAG TTTTGGCGAT ATTCCGTTAG TTCACGGCAT 100
<210> 3297
<211> 100
<212> DNA
<213> Escherichia coli K-12 MG1655
<220> gatb b2094
<222> 64-163
<223> gatb b2094 U00096 complement(2172302..2172586)
<313> 3297: 91-110
<400> 3297
GAAATTAAAG AGTTGTGTCA GAATCATAAT ATTCCTGTTG AATTAATCCA 50
GTGTCGGGTT AATGAAATAG AAACCTATAT GGATGGTGTG CATTTGATAT 100
<210> 3298
<211> 100
<212> DNA
<213> Escherichia coli K-12 MG1655
<220> gatc b2093
<222> 1257-1356
<223> gatc b2093 U00096 complement(2170943..2172298)
<313> 3298: 1308-1327
<400> 3298
TTTCATTATT ATCGGTGCAA TTTATCTGAC CGGTATTTTC ATGACCTGGC 50

GTAGAGCGCG TGGCTTTATT AAACAAGAGA AAGTCGTTCT CGCAGAATAA 100
<210> 3299
<211> 100
<212> DNA
<213> Escherichia coli K-12 MG1655
<220> gatc b2093
<222> 1209-1308
<223> gatc b2093 U00096 complement(2170943..2172298)
<313> 3299: 1246-1265
<400> 3299
CATTACCTGG TTACTGATTC AGGTTTTCTC CCCGCAAAAT ATTCCCGGTT 50
TCATTATTAT CGGTGCAATT TATCTGACCG GTATTTTCAT GACCTGGCGT 100
<210> 3300
<211> 100
<212> DNA
<213> Escherichia coli K-12 MG1655
<220> gatc b2093
<222> 1148-1247
<223> gatc b2093 U00096 complement(2170943..2172298)
<313> 3300: 1210-1229
<400> 3300
CTAATGCTGG GGCGTTAAAA GCCGGGGGTA TGGTGGCTTC AATGGATCAG 50
GGCGGTTCTC CCATTACCTG GTTACTGATT CAGGTTTTCT CCCCGCAAAA 100
<210> 3301
<211> 100
<212> DNA
<213> Escherichia coli K-12 MG1655
<220> gatd b2092
<222> 937-1036
<223> gatd b2092 U00096 complement(2169855..2170895)
<313> 3301: 1001-1020
<400> 3301
CTGGAGCCAT TAATCGCTCA CCGTGGAAGC TTTGAAAGCT TCGCCCAGGC 50
GGTGCGTGAC ATCGCTCGTA ATGCTATGCC GGGCAAAGTG TTGCTCATTC 100
<210> 3302
<211> 100
<212> DNA
<213> Escherichia coli K-12 MG1655
<220> gatd b2092
<222> 904-1003
<223> gatd b2092 U00096 complement(2169855..2170895)
<313> 3302: 941-960
<400> 3302
GCGAGCCGGT TGCTGACAGA ACGTAAGTTA AGCCTGGAGC CATTAATCGC 50
TCACCGTGGA AGCTTTGAAA GCTTCGCCCA GGCGGTGCGT GACATCGCTC 100
<210> 3303
<211> 100
<212> DNA
<213> Escherichia coli K-12 MG1655
<220> gatd b2092
<222> 799-898
<223> gatd b2092 U00096 complement(2169855..2170895)
<313> 3303: 846-865
<400> 3303
CATTTAACAT CGGCAACGTT TGGCAAAATA TTGCGTAAAG AGCTGACGGT 50
TATCGGCAGT TGGATGAACT ACTCCAGCCC TTGGCCGGGG CAGGAGTGGG 100
<210> 3304
<211> 100
<212> DNA
<213> Escherichia coli K-12 MG1655
<220> gaty gatY
<222> 762-861
<223> gaty gatY U00096 complement(2174370..2175230)
<313> 3304: 822-841
<400> 3304
AGCGACCGAT CCCCGGGATT ATTTGCAGTC GGCTAAATCC GCAATGCGCG 50
ATGTGGTGAG CAAAGTGATT GCCGATTGTG GCTGCGAGGG CAGGGCATAA 100
<210> 3305
<211> 100
<212> DNA
<213> Escherichia coli K-12 MG1655

<220> gaty gatY
<222> 691-790
<223> gaty gatY U00096 complement(2174370..2175230)
<313> 3305: 747-766
<400> 3305
ATCAACGTTG CAACGGAGCT GAAAAATGCC TTCTCGCAGG CGTTAAAAAA 50
TTACCTGACC GAGCACCCTG AAGCGACCGA TCCCCGGGAT TATTTGCAGT 100
<210> 3306
<211> 100
<212> DNA
<213> Escherichia coli K-12 MG1655
<220> gaty gatY
<222> 551-650
<223> gaty gatY U00096 complement(2174370..2175230)
<313> 3306: 596-615
<400> 3306
TGTATGCCAG CGCACCGGCG CTTGATTTTT CTAGACTGGA GAACATTCGC 50
CAGTGGGTGA ACTTACCGCT GGTGCTGCAT GGCGCGTCAG GGTTATCGAC 100
<210> 3307
<211> 100
<212> DNA
<213> Escherichia coli K-12 MG1655
<220> gatz b2096
<222> 1137-1236
<223> gatz b2096 U00096 complement(2173079..2174341)
<313> 3307: 1177-1196
<400> 3307
TAGTCAGTAT CTTCCCAAAC AATTTGAACG CATTCAGTCC GGGGAATTAT 50
CAGCAATACC GCATCAGCTG ATTATGGATA AAATTTATGA TGTTTTGCGC 100
<210> 3308
<211> 100
<212> DNA
<213> Escherichia coli K-12 MG1655
<220> gatz b2096
<222> 1090-1189
<223> gatz b2096 U00096 complement(2173079..2174341)
<313> 3308: 1111-1130
<400> 3308
ACGATGATGG TGAATCTTGA AGGCGTGGAC ATCCCACTGG GCATGATTAG 50
TCAGTATCTT CCCAAACAAT TTGAACGCAT TCAGTCCGGG GAATTATCAG 100
<210> 3309
<211> 100
<212> DNA
<213> Escherichia coli K-12 MG1655
<220> gatz b2096
<222> 1032-1131
<223> gatz b2096 U00096 complement(2173079..2174341)
<313> 3309: 1075-1094
<400> 3309
CCTGTCGGAT CGTATTCGTT ATTACTGGCC GCATAGTCGG ATTAAAAATA 50
GCGTCGAAAC GATGATGGTG AATCTTGAAG GCGTGGACAT CCCACTGGGC 100
<210> 3310
<211> 100
<212> DNA
<213> Escherichia coli K-12 MG1655
<220> ptsa b3948
<222> 2037-2136
<223> ptsa b3948 U00096 complement(4137300..4139435)
<313> 3310: 2099-2118
<400> 3310
CGCTCGCCGC ATCATGCACG AAGAATTCCG TAACGCGCTG GTTAACGCCG 50
CCTCTGCCGA CGCTATCGCC AGCCTGCTGC AACATGAACT GGAACTGTAA 100
<210> 3311
<211> 100
<212> DNA
<213> Escherichia coli K-12 MG1655
<220> ptsa b3948
<222> 2010-2109
<223> ptsa b3948 U00096 complement(4137300..4139435)
<313> 3311: 2068-2087
<400> 3311

TCAGCATATG CGCATTTTCT CGCGCCTCGC TCGCCGCATC ATGCACGAAG 50
AATTCCGTAA CGCGCTGGTT AACGCCGCCT CTGCCGACGC TATCGCCAGC 100
<210> 3312
<211> 100
<212> DNA
<213> Escherichia coli K-12 MG1655
<220> ptsa b3948
<222> 1930-2029
<223> ptsa b3948 U00096 complement(4137300..4139435)
<313> 3312: 1994-2013
<400> 3312
CTGCAAGCGC CGGTGCGCTG GGGCGATGAT GAAGCGCAAT TCATCATTAT 50
GTTAACCCTG AACAAACACG CTGCGGGCGA TCAGCATATG CGCATTTTCT 100
<210> 3313
<211> 100
<212> DNA
<213> Escherichia coli K-12 MG1655
<220> ascb b2717
<222> 1299-1398
<223> ascb b2717 U00096 2839013..2840437
<313> 3313: 1332-1351
<400> 3313
CAAACGCTAC GGCTTTGTCT TTGTTGACCG TGACGACGCA GGCAACGGTA 50
CGCTGACGCG CACGCGTAAG AAATCATTCT GGTGGTATAA AAAAGTGATT 100
<210> 3314
<211> 100
<212> DNA
<213> Escherichia coli K-12 MG1655
<220> ascb b2717
<222> 1262-1361
<223> ascb b2717 U00096 2839013..2840437
<313> 3314: 1296-1315
<400> 3314
GTATTGATTT AGTTTCCGCC TCTACGGGTG AAATGAGCAA ACGCTACGGC 50
TTTGTCTTTG TTGACCGTGA CGACGCAGGC AACGGTACGC TGACGCGCAC 100
<210> 3315
<211> 100
<212> DNA
<213> Escherichia coli K-12 MG1655
<220> ascb b2717
<222> 1231-1330
<223> ascb b2717 U00096 2839013..2840437
<313> 3315: 1242-1261
<400> 3315
ATTCCGCTGA TGGGCTACAC CACATGGGGC TGTATTGATT TAGTTTCCGC 50
CTCTACGGGT GAAATGAGCA AACGCTACGG CTTTGTCTTT GTTGACCGTG 100
<210> 3316
<211> 100
<212> DNA
<213> Escherichia coli K-12 MG1655
<220> fdra b0519
<222> 1512-1611
<223> fdra b0519 U00096 545904..547571
<313> 3316: 1548-1567
<400> 3316
ACCGTCATTA CTGGAAAACG TCGCCGTGAT TAACATCGGA TTACGCAGCT 50
TTGCGCTGGA GCTACAAAGC GCCAGCAAAC CGGTTGTGCA TTACCAATGG 100
<210> 3317
<211> 100
<212> DNA
<213> Escherichia coli K-12 MG1655
<220> fdra b0519
<222> 1458-1557
<223> fdra b0519 U00096 545904..547571
<313> 3317: 1518-1537
<400> 3317
CTTGCTGGCG GCAGCGTTAA TTCATCCGCT CTCGCCTGCC GCACAGCAAC 50
ACACACCGTC ATTACTGGAA AACGTCGCCG TGATTAACAT CGGATTACGC 100
<210> 3318
<211> 100
<212> DNA

<213> Escherichia coli K-12 MG1655
<220> fdra b0519
<222> 1369-1468
<223> fdra b0519 U00096 545904..547571
<313> 3318: 1396-1415
<400> 3318
ACTGAACGTG ACCCGCAATG CCGCTCGCAG CAAATCGCCA CGCTGGAAGA 50
TGCGGGGATT GCGGTCGTGA GTTCGCTACC GGAAGCCACC TTGCTGGCGG 100
<210> 3319
<211> 100
<212> DNA
<213> Escherichia coli K-12 MG1655
<220> ascf b2716
<222> 1359-1458
<223> ascf b2716 U00096 2837547..2839004
<313> 3319: 1421-1440
<400> 3319
GCTCGGCTTT GAGGATATTC CTGTTGAGGA AGCGGCTGCC CAGGCGCGAA 50
AGTATCAGAG CGTACAACCG ACCGTCGCCA AAGAAGTAAG TCTTAATTGA 100
<210> 3320
<211> 100
<212> DNA
<213> Escherichia coli K-12 MG1655
<220> ascf b2716
<222> 1288-1387
<223> ascf b2716 U00096 2837547..2839004
<313> 3320: 1343-1362
<400> 3320
CCAATGAGCA TCGTCTGGGT GTTCGCGGTC ATGGCGCTGG CGGTGGTGCT 50
GTCGTTTATC CTCACACTGT TGCTCGGCTT TGAGGATATT CCTGTTGAGG 100
<210> 3321
<211> 100
<212> DNA
<213> Escherichia coli K-12 MG1655
<220> ascf b2716
<222> 1239-1338
<223> ascf b2716 U00096 2837547..2839004
<313> 3321: 1285-1304
<400> 3321
GGCAGCGCCG GGGCTATTTA CCAGCGTGCA GTTCTTCGAT CCGGCGAATC 50
CAATGAGCAT CGTCTGGGTG TTCGCGGTCA TGGCGCTGGC GGTGGTGCTG 100
<210> 3322
<211> 100
<212> DNA
<213> Escherichia coli K-12 MG1655
<220> ptsg b1102
<222> 1317-1416
<223> ptsg b1102 U00096 1157092..1158525
<313> 3322: 1360-1379
<400> 3322
CCTGAAGAAA CTGGGCGCAG CGGGCGTAGT GGTTGCTGGT TCTGGTGTTC 50
AGGCGATTTT CGGTACTAAA TCCGATAACC TGAAAACCGA GATGGATGAG 100
<210> 3323
<211> 100
<212> DNA
<213> Escherichia coli K-12 MG1655
<220> ptsg b1102
<222> 1223-1322
<223> ptsg b1102 U00096 1157092..1158525
<313> 3323: 1247-1266
<400> 3323
CATTTGGTGG TAAAGAAAAC ATTACTAACC TCGACGCATG TATTACCCGT 50
CTGCGCGTCA GCGTTGCTGA TGTGTCTAAA GTGGATCAGG CCGGCCTGAA 100
<210> 3324
<211> 100
<212> DNA
<213> Escherichia coli K-12 MG1655
<220> ptsg b1102
<222> 1185-1284
<223> ptsg b1102 U00096 1157092..1158525
<313> 3324: 1211-1230

<400> 3324
GACAGGTACC AGCGAAATGG CACCGGCTCT GGTTGCTGCA TTTGGTGGTA 50
AAGAAAACAT TACTAACCTC GACGCATGTA TTACCCGTCT GCGCGTCAGC 100
<210> 3325
<211> 100
<212> DNA
<213> Escherichia coli K-12 MG1655
<220> ascg b2715
<222> 915-1014
<223> ascg b2715 U00096 complement(2836277..2837290)
<313> 3325: 981-1000
<400> 3325
ACGGCTGATT TTTATGCTCG ATGGCGGGGA TTTCTCACCG CCGAAAACCT 50
TCAGCGGAAA ACTGATCCGC CGCGACTCAC TCATTGCTCC TTCGCGATAA 100
<210> 3326
<211> 100
<212> DNA
<213> Escherichia coli K-12 MG1655
<220> ascg b2715
<222> 879-978
<223> ascg b2715 U00096 complement(2836277..2837290)
<313> 3326: 928-947
<400> 3326
AATTCCGGTG ACTGAGATGA TTCAGGAAAT TATTGGACGG CTGATTTTTA 50
TGCTCGATGG CGGGGATTTC TCACCGCCGA AAACCTTCAG CGGAAAACTG 100
<210> 3327
<211> 100
<212> DNA
<213> Escherichia coli K-12 MG1655
<220> ascg b2715
<222> 848-947
<223> ascg b2715 U00096 complement(2836277..2837290)
<313> 3327: 882-901
<400> 3327
CCTACACCGT TCCGGCGCTC TCCAGCGTAA AAATTCCGGT GACTGAGATG 50
ATTCAGGAAA TTATTGGACG GCTGATTTTT ATGCTCGATG GCGGGGATTT 100
<210> 3328
<211> 100
<212> DNA
<213> Escherichia coli K-12 MG1655
<220> ptsh b2416
<222> 159-258
<223> ptsh b2416 U00096 2531784..2532041
<313> 3328: 215-234
<400> 3328
GGGCCTGACT CAAGGTACCG TTGTGACTAT CTCCGCAGAA GGCGAAGACG 50
AGCAGAAAGC GGTTGAACAT CTGGTTAAAC TGATGGCGGA ACTCGAGTAA 100
<210> 3329
<211> 100
<212> DNA
<213> Escherichia coli K-12 MG1655
<220> ptsh b2416
<222> 149-248
<223> ptsh b2416 U00096 2531784..2532041
<313> 3329: 175-194
<400> 3329
TGCAGACTCT GGGCCTGACT CAAGGTACCG TTGTGACTAT CTCCGCAGAA 50
GGCGAAGACG AGCAGAAAGC GGTTGAACAT CTGGTTAAAC TGATGGCGGA 100
<210> 3330
<211> 100
<212> DNA
<213> Escherichia coli K-12 MG1655
<220> ptsh b2416
<222> 78-177
<223> ptsh b2416 U00096 2531784..2532041
<313> 3330: 117-136
<400> 3330
TAAGGGCTTC ACTTCTGAAA TTACTGTGAC TTCCAACGGC AAAAGCGCCA 50
GCGCGAAAAG CCTGTTTAAA CTGCAGACTC TGGGCCTGAC TCAAGGTACC 100
<210> 3331
<211> 100

<212> DNA
<213> Escherichia coli K-12 MG1655
<220> ptsi b2417
<222> 1628-1727
<223> ptsi b2417 U00096 2532086..2533813
<313> 3331: 1679-1698
<400> 3331
AAGATGCGAA GGTGTTAGCA GAGCAGGCTC TTGCTCAACC GACAACGGAC 50
GAGTTAATGA CGCTGGTTAA CAAGTTCATT GAAGAAAAAA CAATCTGCTA 100
<210> 3332
<211> 100
<212> DNA
<213> Escherichia coli K-12 MG1655
<220> ptsi b2417
<222> 1544-1643
<223> ptsi b2417 U00096 2532086..2533813
<313> 3332: 1595-1614
<400> 3332
TGCTGGGGAT GGGTCTGGAC GAATTCTCTA TGAGCGCCAT TTCTATCCCG 50
CGCATTAAGA AGATTATCCG TAACACGAAC TTCGAAGATG CGAAGGTGTT 100
<210> 3333
<211> 100
<212> DNA
<213> Escherichia coli K-12 MG1655
<220> ptsi b2417
<222> 1505-1604
<223> ptsi b2417 U00096 2532086..2533813
<313> 3333: 1531-1550
<400> 3333
GTGGTGAGCT TGCTGGCGAT GAACGTGCTA CACTTCTGTT GCTGGGGATG 50
GGTCTGGACG AATTCTCTAT GAGCGCCATT TCTATCCCGC GCATTAAGAA 100
<210> 3334
<211> 100
<212> DNA
<213> Escherichia coli K-12 MG1655
<220> pmba b4236
<222> 1254-1353
<223> pmba b4236 U00096 4455533..4456885
<313> 3334: 1321-1340
<400> 3334
GTGGCGCAAT ATTGTCACCG TCGGTAACGA TATTGAAACA CGCAGTAATA 50
TACAGTGTGG TTCTGTGCTG TTGCCGGAGA TGAAAATCGC CGGACAGTAA 100
<210> 3335
<211> 100
<212> DNA
<213> Escherichia coli K-12 MG1655
<220> pmba b4236
<222> 1202-1301
<223> pmba b4236 U00096 4455533..4456885
<313> 3335: 1218-1237
<400> 3335
AAATTCAGTA TCCGGTGAGC GAAATCACCA TCGCAGGTAA TTTAAAAGAT 50
ATGTGGCGCA ATATTGTCAC CGTCGGTAAC GATATTGAAA CACGCAGTAA 100
<210> 3336
<211> 100
<212> DNA
<213> Escherichia coli K-12 MG1655
<220> pmba b4236
<222> 1159-1258
<223> pmba b4236 U00096 4455533..4456885
<313> 3336: 1183-1202
<400> 3336
GATTATTCCC GTGGTGCAGC GGGCTTCTGG GTAGAGAATG GCGAAATTCA 50
GTATCCGGTG AGCGAAATCA CCATCGCAGG TAATTTAAAA GATATGTGGC 100
<210> 3337
<211> 100
<212> DNA
<213> Escherichia coli K-12 MG1655
<220> ptsn b3205
<222> 382-481
<223> ptsn b3205 U00096 3344219..3344710

<313> 3337: 434-453
<400> 3337
GCGAAACGTC TGGCGGACAA AACCATCTGC CGCCGTTTAC GCGCAGCCCA 50
GAGCGATGAA GAGCTGTATC AAATCATTAC GGATACCGAA GGTACTCCGG 100
<210> 3338
<211> 100
<212> DNA
<213> Escherichia coli K-12 MG1655
<220> ptsn b3205
<222> 300-399
<223> ptsn b3205 U00096 3344219..3344710
<313> 3338: 345-364
<400> 3338
CAACCAACCG GTGGACTTAC TTTTTGCCCT GCTGGTGCCG GCAGACCAAA 50
CTAAAACGCA CCTGCATACT CTGTCGCTGG TGGCGAAACG TCTGGCGGAC 100
<210> 3339
<211> 100
<212> DNA
<213> Escherichia coli K-12 MG1655
<220> ptsn b3205
<222> 220-319
<223> ptsn b3205 U00096 3344219..3344710
<313> 3339: 268-287
<400> 3339
GGCAAACTGG AAGAAGATAC TCTGCGCGCC GTTGGCGTTT TCGTTCAGCT 50
CGAAACGCCT ATAGCTTTCG ATGCCATCGA CAACCAACCG GTGGACTTAC 100
<210> 3340
<211> 100
<212> DNA
<213> Escherichia coli K-12 MG1655
<220> ptso b3207
<222> 174-273
<223> ptso b3207 U00096 3345607..3345879
<313> 3340: 248-267
<400> 3340
CAAAGGACGG CAGATTGAAG TTGAAGCGAC CGGTCCACAG GAAGAGGAAG 50
CACTGGCCGC CGTTATCGCC CTCTTTAATT CTGGTTTTGA TGAAGATTAA 100
<210> 3341
<211> 100
<212> DNA
<213> Escherichia coli K-12 MG1655
<220> ptso b3207
<222> 72-171
<223> ptso b3207 U00096 3345607..3345879
<313> 3341: 116-135
<400> 3341
TGAATTAATG CAGGGTTTTG ACGCTGAAGT GCTCTTACGT AATGACGAAG 50
GCACCGAGGC TGAAGCCAAC AGCGTTATTG CGCTGCTGAT GTTGGATTCT 100
<210> 3342
<211> 100
<212> DNA
<213> Escherichia coli K-12 MG1655
<220> ptso b3207
<222> 1-100
<223> ptso b3207 U00096 3345607..3345879
<313> 3342: 20-39
<400> 3342
ATGACCGTCA AGCAAACTGT TGAAATCACA AACAAGCTGG GCATGCATGC 50
CCGGCCTGCA ATGAAGCTGT TTGAATTAAT GCAGGGTTTT GACGCTGAAG 100
<210> 3343
<211> 100
<212> DNA
<213> Escherichia coli K-12 MG1655
<220> ptsp b2830
<222> 2107-2206
<223> ptsp b2830 U00096 complement(2964210..2966456)
<313> 3343: 2147-2166
<400> 3343
CTGCGGCGCA TTGATTATGC CGAAGCAGAA AATCTTGCGC AGCGTAGTCT 50
GGAAGCGCAA CTGGCGACCG AAGTTCGCCA TCAGGTTGCA GCCTTTATGG 100
<210> 3344

<211> 100
<212> DNA
<213> Escherichia coli K-12 MG1655
<220> ptsp b2830
<222> 2009-2108
<223> ptsp b2830 U00096 complement(2964210..2966456)
<313> 3344: 2053-2072
<400> 3344
AAATGGCGGG CGATCCCATG TGCGTGGCAA TCCTCATTGG GCTTGGGTAT 50
CGCCATCTGT CTATGAACGG ACGTTCTGTA GCGCGGGCAA AATACCTGCT 100
<210> 3345
<211> 100
<212> DNA
<213> Escherichia coli K-12 MG1655
<220> ptsp b2830
<222> 1941-2040
<223> ptsp b2830 U00096 complement(2964210..2966456)
<313> 3345: 1993-2012
<400> 3345
GTTACGAGCT CTGGCGATGA TCGCCCGGGA AGCGGAAATA CATGGAATCG 50
ATCTCCGTTT GTGCGGTGAA ATGGCGGGCG ATCCCATGTG CGTGGCAATC 100
<210> 3346
<211> 100
<212> DNA
<213> Escherichia coli K-12 MG1655
<220> livf b3455
<222> 599-698
<223> livf b3455 U00096 complement(3590355..3591080)
<313> 3346: 651-670
<400> 3346
ACGCCAACCA GGCGCTAAAG CTGGCGGATC GCGGCTACGT GCTGGAAAAC 50
GGCCATGTAG TGCTTTCCGA TACTGGTGAT GCGCTGCTGG CGAATGAAGC 100
<210> 3347
<211> 100
<212> DNA
<213> Escherichia coli K-12 MG1655
<220> livf b3455
<222> 587-686
<223> livf b3455 U00096 complement(3590355..3591080)
<313> 3347: 615-634
<400> 3347
TCGTCGAGCA GAACGCCAAC CAGGCGCTAA AGCTGGCGGA TCGCGGCTAC 50
GTGCTGGAAA ACGGCCATGT AGTGCTTTCC GATACTGGTG ATGCGCTGCT 100
<210> 3348
<211> 100
<212> DNA
<213> Escherichia coli K-12 MG1655
<220> livf b3455
<222> 521-620
<223> livf b3455 U00096 complement(3590355..3591080)
<313> 3348: 574-593
<400> 3348
CGATTATCAT CCAGCAAATT TTCGACACCA TCGAGCAGCT GCGCGAGCAG 50
GGGATGACTA TCTTTCTCGT CGAGCAGAAC GCCAACCAGG CGCTAAAGCT 100
<210> 3349
<211> 100
<212> DNA
<213> Escherichia coli K-12 MG1655
<220> livg b3456
<222> 669-768
<223> livg b3456 U00096 complement(3591070..3591837)
<313> 3349: 736-755
<400> 3349
AATTTACGTG GTCAATCAGG GGACGCCGCT GGCAAACGGT ACGCCGGAGC 50
AGATCCGTAA TAACCCGGAC GTGATCCGTG CCTATTTAGG TGAGGCATAA 100
<210> 3350
<211> 100
<212> DNA
<213> Escherichia coli K-12 MG1655
<220> livg b3456
<222> 655-754

<223> livg b3456 U00096 complement(3591070..3591837)
<313> 3350: 703-722
<400> 3350
GGAATTTCGG ACCGAATTTA CGTGGTCAAT CAGGGGACGC CGCTGGCAAA 50
CGGTACGCCG GAGCAGATCC GTAATAACCC GGACGTGATC CGTGCCTATT 100
<210> 3351
<211> 100
<212> DNA
<213> Escherichia coli K-12 MG1655
<220> livg b3456
<222> 544-643
<223> livg b3456 U00096 complement(3591070..3591837)
<313> 3351: 562-581
<400> 3351
GCAGGTCTTA ACCCGAAAGA GACGAAAGAG CTGGATGAGC TGATTGCCGA 50
ACTGCGCAAT CATCACAACA CCACTATCTT GTTGATTGAA CACGATATGA 100
<210> 3352
<211> 100
<212> DNA
<213> Escherichia coli K-12 MG1655
<220> livh b3458
<222> 820-919
<223> livh b3458 U00096 complement(3593108..3594034)
<313> 3352: 872-891
<400> 3352
TATCTGAGTA CGGAATATAA AGATGTGGTC TCATTCGCCC TGCTGATTCT 50
GGTGCTGCTG GTGATGCCGA CCGGTATTCT GGGTCGCCCG GAGGTAGAGA 100
<210> 3353
<211> 100
<212> DNA
<213> Escherichia coli K-12 MG1655
<220> livh b3458
<222> 770-869
<223> livh b3458 U00096 complement(3593108..3594034)
<313> 3353: 820-839
<400> 3353
CGATGATTGG CGGCCTGATT CTGGGGATTG CGGAGGCGCT CTCTTCTGCC 50
TATCTGAGTA CGGAATATAA AGATGTGGTC TCATTCGCCC TGCTGATTCT 100
<210> 3354
<211> 100
<212> DNA
<213> Escherichia coli K-12 MG1655
<220> livh b3458
<222> 654-753
<223> livh b3458 U00096 complement(3593108..3594034)
<313> 3354: 696-715
<400> 3354
GGCGGGTGTG CTGCTCGGTC AGTTCTACGG CGTCATTAAC CCCTACATCG 50
GCTTTATGGC CGGGATGAAA GCCTTTACCG CGGCGGTGCT CGGTGGGATT 100
<210> 3355
<211> 100
<212> DNA
<213> Escherichia coli K-12 MG1655
<220> perm perM
<222> 963-1062
<223> perm perM U00096 complement(2612840..2613901)
<313> 3355: 1031-1050
<400> 3355
TTTGTGGGGA TTCTGGGGCG TATTCTTCGC CATTCCGTTG GCGACGCTGA 50
TCAAAGCCGT GATTCATGCC TGGCCCGATG GGCAAATCGC GCAAGAATAA 100
<210> 3356
<211> 100
<212> DNA
<213> Escherichia coli K-12 MG1655
<220> perm perM
<222> 940-1039
<223> perm perM U00096 complement(2612840..2613901)
<313> 3356: 974-993
<400> 3356
TTATCGGTGG TGATCTTCGG TGGTTTGTGG GGATTCTGGG GCGTATTCTT 50
CGCCATTCCG TTGGCGACGC TGATCAAAGC CGTGATTCAT GCCTGGCCCG 100

<210> 3357
<211> 100
<212> DNA
<213> Escherichia coli K-12 MG1655
<220> perm perM
<222> 807-906
<223> perm perM U00096 complement(2612840..2613901)
<313> 3357: 867-886
<400> 3357
ATTCCAGTTT GGCGCAGGTA CTGAATTCTG GAGCTGCTTC GCGGTGTATC  50
TGATTATTCA GGCGCTGGAC GGCAACCTGT TAGTGCCGGT GTTGTTCTCC  100
<210> 3358
<211> 100
<212> DNA
<213> Escherichia coli K-12 MG1655
<220> livj b3461
<222> 1062-1161
<223> livj b3461 U00096 complement(3596186..3597346)
<313> 3358: 1123-1142
<400> 3358
AATGGGACCG CTGACCTGGG ATGAGAAAGG CGATCTGAAA GGCTTTGAGT  50
TCGGCGTATT TGACTGGCAC GCCAACGGCA CGGCGACCGA TGCGAAGTAA  100
<210> 3359
<211> 100
<212> DNA
<213> Escherichia coli K-12 MG1655
<220> livj b3461
<222> 1017-1116
<223> livj b3461 U00096 complement(3596186..3597346)
<313> 3359: 1085-1104
<400> 3359
GGCTGAAATC GCCAAATACC TGAAAGCGAA CTCCGTGGAT ACCGTAATGG  50
GACCGCTGAC CTGGGATGAG AAAGGCGATC TGAAAGGCTT TGAGTTCGGC  100
<210> 3360
<211> 100
<212> DNA
<213> Escherichia coli K-12 MG1655
<220> livj b3461
<222> 995-1094
<223> livj b3461 U00096 complement(3596186..3597346)
<313> 3360: 1027-1046
<400> 3360
GCCTGAATCA GTCTGACGAT CCGGCTGAAA TCGCCAAATA CCTGAAAGCG  50
AACTCCGTGG ATACCGTAAT GGGACCGCTG ACCTGGGATG AGAAAGGCGA  100
<210> 3361
<211> 100
<212> DNA
<213> Escherichia coli K-12 MG1655
<220> livk b3459
<222> 1011-1110
<223> livk b3459 U00096 complement(3594082..3595191)
<313> 3361: 1078-1097
<400> 3361
GATTGGGCCG CTGAACTGGG ATGAAAAAGG CGATCTTAAG GGATTTGATT  50
TTGGTGTCTT CCAGTGGCAC GCCGACGGTT CATCCACGGC AGCCAAGTGA  100
<210> 3362
<211> 100
<212> DNA
<213> Escherichia coli K-12 MG1655
<220> livk b3459
<222> 988-1087
<223> livk b3459 U00096 complement(3594082..3595191)
<313> 3362: 1023-1042
<400> 3362
AAAGCTAACG GTGCAAACAC CGTGATTGGG CCGCTGAACT GGGATGAAAA  50
AGGCGATCTT AAGGGATTTG ATTTTGGTGT CTTCCAGTGG CACGCCGACG  100
<210> 3363
<211> 100
<212> DNA
<213> Escherichia coli K-12 MG1655
<220> livk b3459

<222> 909-1008
<223> livk b3459 U00096 complement(3594082..3595191)
<313> 3363: 927-946
<400> 3363
CTACGCGGCG GTGCAATCTC TGGCGACTGC CCTTGAGCGT ACCGGCAGCG 50
ATGAGCCGCT GGCGCTGGTG AAAGATTTAA AAGCTAACGG TGCAAACACC 100
<210> 3364
<211> 100
<212> DNA
<213> Escherichia coli K-12 MG1655
<220> livm b3457
<222> 1179-1278
<223> livm b3457 U00096 complement(3591834..3593111)
<313> 3364: 1240-1259
<400> 3364
GATGGTGCTG ATGATGATCT GGCGTCCGCA GGGCTTGCTG CCCATGACGC 50
GCCCGCAACT GAAGCTGAAA AACGGCGCAG CGAAAGGAGA GCAGGCATGA 100
<210> 3365
<211> 100
<212> DNA
<213> Escherichia coli K-12 MG1655
<220> livm b3457
<222> 1155-1254
<223> livm b3457 U00096 complement(3591834..3593111)
<313> 3365: 1211-1230
<400> 3365
CAGCATGTTA ATGCTCGGTG GTTTGATGGT GCTGATGATG ATCTGGCGTC 50
CGCAGGGCTT GCTGCCCATG ACGCGCCCGC AACTGAAGCT GAAAAACGGC 100
<210> 3366
<211> 100
<212> DNA
<213> Escherichia coli K-12 MG1655
<220> livm b3457
<222> 1115-1214
<223> livm b3457 U00096 complement(3591834..3593111)
<313> 3366: 1161-1180
<400> 3366
TGGTGGTGTC GCGCGAGTTG ATGCGTGATT TCAACGAATA CAGCATGTTA 50
ATGCTCGGTG GTTTGATGGT GCTGATGATG ATCTGGCGTC CGCAGGGCTT 100
<210> 3367
<211> 100
<212> DNA
<213> Escherichia coli K-12 MG1655
<220> perr b0255
<222> 795-894
<223> perr b0255 U00096 complement(268513..269406)
<313> 3367: 843-862
<400> 3367
TCATTATTTG GTTTTCCCCC AGCACCAACA CATGCATTCT GCGCTTGATG 50
TGTTCAAAAC CTGGCTACTG AATGAACTCA ATCTGGGTAA AATTCGTTGA 100
<210> 3368
<211> 100
<212> DNA
<213> Escherichia coli K-12 MG1655
<220> perr b0255
<222> 760-859
<223> perr b0255 U00096 complement(268513..269406)
<313> 3368: 802-821
<400> 3368
CTTGTAAACA GTACCAGCGA AATACATTAC ATCGGTCATT ATTTGGTTTT 50
CCCCCAGCAC CAACACATGC ATTCTGCGCT TGATGTGTTC AAAACCTGGC 100
<210> 3369
<211> 100
<212> DNA
<213> Escherichia coli K-12 MG1655
<220> perr b0255
<222> 652-751
<223> perr b0255 U00096 complement(268513..269406)
<313> 3369: 708-727
<400> 3369
GACCGTAGCT TTATGGCGAT TGCTGCCGCA GTTGACGGCT TGGGAGTTGT 50

GCTTGAATCA AAACTACTGG CTGAACGTGA AATCGCTAGC GGTAAACTGG 100
<210> 3370
<211> 100
<212> DNA
<213> Escherichia coli K-12 MG1655
<220> baer baeR
<222> 624-723
<223> baer baeR U00096 2162298..2163020
<313> 3370: 697-716
<400> 3370
GCGCCGCAAG CTGGAATCTC TCGACGCCGA ACAGTCATTT ATCCGCGCCG 50
TTTATGGCGT CGGTTACCGC TGGGAAGCCG ACGCCTGCCG CATCGTTTAG 100
<210> 3371
<211> 100
<212> DNA
<213> Escherichia coli K-12 MG1655
<220> baer baeR
<222> 624-723
<223> baer baeR U00096 2162298..2163020
<313> 3371: 647-666
<400> 3371
GCGCCGCAAG CTGGAATCTC TCGACGCCGA ACAGTCATTT ATCCGCGCCG 50
TTTATGGCGT CGGTTACCGC TGGGAAGCCG ACGCCTGCCG CATCGTTTAG 100
<210> 3372
<211> 100
<212> DNA
<213> Escherichia coli K-12 MG1655
<220> baer baeR
<222> 564-663
<223> baer baeR U00096 2162298..2163020
<313> 3372: 606-625
<400> 3372
TCTTTATGAC GACTACCGCG TAGTAACCGA CCGCACCATC GACAGCCACA 50
TTAAAAACCT GCGCCGCAAG CTGGAATCTC TCGACGCCGA ACAGTCATTT 100
<210> 3373
<211> 100
<212> DNA
<213> Escherichia coli K-12 MG1655
<220> baes b2079
<222> 1305-1404
<223> baes b2079 U00096 2160898..2162301
<313> 3373: 1378-1397
<400> 3373
AGCACATAAT GGTCGCATTA TTGCTGCCCA TTCGCCTTTT GGCGGGGTAA 50
GCATTACAGT AGAGTTACCG CTGGAACGGG ATTTACAGAG AGAAGTATGA 100
<210> 3374
<211> 100
<212> DNA
<213> Escherichia coli K-12 MG1655
<220> baes b2079
<222> 1289-1388
<223> baes b2079 U00096 2160898..2162301
<313> 3374: 1322-1341
<400> 3374
GCCTGAACAT TGTTGAAGCA CATAATGGTC GCATTATTGC TGCCCATTCG 50
CCTTTTGGCG GGGTAAGCAT TACAGTAGAG TTACCGCTGG AACGGGATTT 100
<210> 3375
<211> 100
<212> DNA
<213> Escherichia coli K-12 MG1655
<220> baes b2079
<222> 1247-1346
<223> baes b2079 U00096 2160898..2162301
<313> 3375: 1285-1304
<400> 3375
GCAACCGTGC CAGCGGCGGT TCCGGGCTGG GGCTGGCGAT TTGCCTGAAC 50
ATTGTTGAAG CACATAATGG TCGCATTATT GCTGCCCATT CGCCTTTTGG 100
<210> 3376
<211> 100
<212> DNA
<213> Escherichia coli K-12 MG1655

<220> hcaa1 b2539
<222> 1263-1362
<223> hcaa1 b2539 U00096 2667052..2668413
<313> 3376: 1333-1352
<400> 3376
TCGTGGAATG TACCAACGCT GGGCCGATCT TCTGAGTAGC GAAAGCTGGC 50
AGGAAGTGCT CGATAAAACC GCCGCTTACC AGCAGGAGGT GATGAAATGA 100
<210> 3377
<211> 100
<212> DNA
<213> Escherichia coli K-12 MG1655
<220> hcaa1 b2539
<222> 1245-1344
<223> hcaa1 b2539 U00096 2667052..2668413
<313> 3377: 1298-1317
<400> 3377
CTTTTCAGAA ACTGCCGCTC GTGGAATGTA CCAACGCTGG GCCGATCTTC 50
TGAGTAGCGA AAGCTGGCAG GAAGTGCTCG ATAAAACCGC CGCTTACCAG 100
<210> 3378
<211> 100
<212> DNA
<213> Escherichia coli K-12 MG1655
<220> hcaa1 b2539
<222> 1223-1322
<223> hcaa1 b2539 U00096 2667052..2668413
<313> 3378: 1243-1262
<400> 3378
TTCCTGGCAT TACTAACTAT ATCTTTTCAG AAACTGCCGC TCGTGGAATG 50
TACCAACGCT GGGCCGATCT TCTGAGTAGC GAAAGCTGGC AGGAAGTGCT 100
<210> 3379
<211> 100
<212> DNA
<213> Escherichia coli K-12 MG1655
<220> hcaa2 b2540
<222> 420-519
<223> hcaa2 b2540 U00096 2668410..2668928
<313> 3379: 475-494
<400> 3379
AGTTCGCCGT CTGGAAGATG ACAACTGGCG CTTGCTGGAA CGGGATATCG 50
TCCTGGATCA AGCGGTAATC ACTTCCCATA ACCTGAGTGT ACTGTTCTGA 100
<210> 3380
<211> 100
<212> DNA
<213> Escherichia coli K-12 MG1655
<220> hcaa2 b2540
<222> 407-506
<223> hcaa2 b2540 U00096 2668410..2668928
<313> 3380: 449-468
<400> 3380
CGCGTTTCGA CAAAGTTCGC CGTCTGGAAG ATGACAACTG GCGCTTGCTG 50
GAACGGGATA TCGTCCTGGA TCAAGCGGTA ATCACTTCCC ATAACCTGAG 100
<210> 3381
<211> 100
<212> DNA
<213> Escherichia coli K-12 MG1655
<220> hcaa2 b2540
<222> 330-429
<223> hcaa2 b2540 U00096 2668410..2668928
<313> 3381: 372-391
<400> 3381
AAACGTATTT GCTGTGCGGG TAAATTATCT GCTTTATCGG GCACAAAAAG 50
AGCGCGATGA AACATTCTAT GTTGGAACGC GTTTCGACAA AGTTCGCCGT 100
<210> 3382
<211> 100
<212> DNA
<213> Escherichia coli K-12 MG1655
<220> iclr b4019
<222> 765-864
<223> iclr b4019 U00096 complement(4220383..4221246)
<313> 3382: 811-830
<400> 3382

ACCGATTTCA CGTATTACCG ATGACCGCGT GACCGAGTTT GGCGCGATGG 50
TGATTAAAGC GGCGAAGGAA GTGACGCTGG CGTACGGTGG AATGCGCTGA 100
<210> 3383
<211> 100
<212> DNA
<213> Escherichia coli K-12 MG1655
<220> iclr b4019
<222> 722-821
<223> iclr b4019 U00096 complement(4220383..4221246)
<313> 3383: 785-804
<400> 3383
TCGATGAGCA CCGTGAACCG TTTGCCGCAA TTTCTATTTC CGGACCGATT 50
TCACGTATTA CCGATGACCG CGTGACCGAG TTTGGCGCGA TGGTGATTAA 100
<210> 3384
<211> 100
<212> DNA
<213> Escherichia coli K-12 MG1655
<220> iclr b4019
<222> 689-788
<223> iclr b4019 U00096 complement(4220383..4221246)
<313> 3384: 737-756
<400> 3384
CACTGGGGCT ACGTTGCCTT GCAGCGTGTA TTTTCGATGA GCACCGTGAA 50
CCGTTTGCCG CAATTTCTAT TTCCGGACCG ATTTCACGTA TTACCGATGA 100
<210> 3385
<211> 100
<212> DNA
<213> Escherichia coli K-12 MG1655
<220> flda b0685
<222> 403-502
<223> flda b0685 U00096 complement(710158..710688)
<313> 3385: 456-475
<400> 3385
GATGACGACC ACTTTGTCGG TCTGGCTATC GACGAAGACC GTCAGCCGGA 50
ACTGACCGCT GAACGTGTAG AAAAATGGGT TAAACAGATT TCTGAAGAGT 100
<210> 3386
<211> 100
<212> DNA
<213> Escherichia coli K-12 MG1655
<220> flda b0685
<222> 266-365
<223> flda b0685 U00096 complement(710158..710688)
<313> 3386: 297-316
<400> 3386
GTGACCAGGA AGATTACGCC GAATATTTCT GCGACGCATT GGGCACCATC 50
CGCGACATCA TTGAACCGCG CGGTGCAACC ATCGTTGGTC ACTGGCCAAC 100
<210> 3387
<211> 100
<212> DNA
<213> Escherichia coli K-12 MG1655
<220> flda b0685
<222> 172-271
<223> flda b0685 U00096 complement(710158..710688)
<313> 3387: 193-212
<400> 3387
TATTACGGCG AAGCGCAGTG TGACTGGGAT GACTTCTTCC CGACTCTCGA 50
AGAGATTGAT TTCAACGGCA AACTGGTTGC GCTGTTTGGT TGTGGTGACC 100
<210> 3388
<211> 100
<212> DNA
<213> Escherichia coli K-12 MG1655
<220> fldb fldB
<222> 423-522
<223> fldb fldB U00096 3037875..3038396
<313> 3388: 502-521
<400> 3388
TCTGGCGCTG GATGAAACTA ACCAGTATGA CCTTAGCGAC GAGCGTATTC 50
AGAGCTGGTG CGAGCAAATC CTCAACGAAA TGGCAGAGCA TTACGCCTGA 100
<210> 3389
<211> 100
<212> DNA

<213> Escherichia coli K-12 MG1655
<220> fldb fldB
<222> 418-517
<223> fldb fldB U00096 3037875..3038396
<313> 3389: 461-480
<400> 3389
GTGGGTCTGG CGCTGGATGA AACTAACCAG TATGACCTTA GCGACGAGCG 50
TATTCAGAGC TGGTGCGAGC AAATCCTCAA CGAAATGGCA GAGCATTACG 100
<210> 3390
<211> 100
<212> DNA
<213> Escherichia coli K-12 MG1655
<220> fldb fldB
<222> 282-381
<223> fldb fldB U00096 3037875..3038396
<313> 3390: 319-338
<400> 3390
CGAGTGGTTC CTCGATGCGC TCGGTATGCT GCATGACAAA CTCTCGACCA 50
AAGGCGTGAA GTTCGTCGGC TACTGGCCAA CGGAAGGATA TGAATTTACC 100
<210> 3391
<211> 100
<212> DNA
<213> Escherichia coli K-12 MG1655
<220> yqab b2691
<222> 423-522
<223> yqab b2691 U00096 complement(2814959..2815525)
<313> 3391: 471-490
<400> 3391
TAAACCCGCG CCAGACACAT TTTTGTTGTG CGCGCAGCGT ATGGGCGTGC 50
AACCGACGCA GTGTGTGGTC TTTGAAGATG CCGATTTCGG TATTCAGGCG 100
<210> 3392
<211> 100
<212> DNA
<213> Escherichia coli K-12 MG1655
<220> yqab b2691
<222> 381-480
<223> yqab b2691 U00096 complement(2814959..2815525)
<313> 3392: 428-447
<400> 3392
TTATTTTGAC GCCGTCGTCG CTGCCGATCA CGTCAAACAC CATAAACCCG 50
CGCCAGACAC ATTTTTGTTG TGCGCGCAGC GTATGGGCGT GCAACCGACG 100
<210> 3393
<211> 100
<212> DNA
<213> Escherichia coli K-12 MG1655
<220> yqab b2691
<222> 345-444
<223> yqab b2691 U00096 complement(2814959..2815525)
<313> 3393: 377-396
<400> 3393
CGCTGAGGCA TTGCTGGCGC ACCTGGGATT ACGCCATTAT TTTGACGCCG 50
TCGTCGCTGC CGATCACGTC AAACACCATA AACCCGCGCC AGACACATTT 100
<210> 3394
<211> 100
<212> DNA
<213> Escherichia coli K-12 MG1655
<220> umuc b1185
<222> 1170-1269
<223> umuc b1185 U00096 1230409..1231677
<313> 3394: 1225-1244
<400> 3394
TTTTGCCGGG CAGGGGATCC AGCAACAATG GCAGATGAAG CGAGCCATGC 50
TTTCACCACG TTATACAACG CGAAGTTCTG ATTTACTGAG GGTCAAATAA 100
<210> 3395
<211> 100
<212> DNA
<213> Escherichia coli K-12 MG1655
<220> umuc b1185
<222> 1087-1186
<223> umuc b1185 U00096 1230409..1231677
<313> 3395: 1141-1160

<400> 3395
GACAACGCAC CGCGCCCCGG GAGTGAGCAA TTGATGACGG TAATGGATAC 50
ACTGAATGCT AAAGAGGGCA GAGGAACACT CTATTTTGCC GGGCAGGGGA 100
<210> 3396
<211> 100
<212> DNA
<213> Escherichia coli K-12 MG1655
<220> umuc b1185
<222> 1037-1136
<223> umuc b1185 U00096 1230409..1231677
<313> 3396: 1084-1103
<400> 3396
TGCTGGGGGA TTTCTTCAGT CAGGGAGTCG CGCAGCTCAA TTTATTCGAT 50
GACAACGCAC CGCGCCCCGG GAGTGAGCAA TTGATGACGG TAATGGATAC 100
<210> 3397
<211> 100
<212> DNA
<213> Escherichia coli K-12 MG1655
<220> umud b1184
<222> 321-420
<223> umud b1184 U00096 1229990..1230409
<313> 3397: 353-372
<400> 3397
TATTCCCATG AACAGCGCGT ACTCGCCCAT TACCATCAGT AGTGAAGATA 50
CGCTGGATGT CTTTGGTGTG GTGATCCACG TCGTTAAGGC GATGCGCTGA 100
<210> 3398
<211> 100
<212> DNA
<213> Escherichia coli K-12 MG1655
<220> umud b1184
<222> 250-349
<223> umud b1184 U00096 1229990..1230409
<313> 3398: 304-323
<400> 3398
GATATTGTCA TCGCTGCTGT TGACGGCGAG TTTACGGTGA AAAAATTGCA 50
ACTACGCCCG ACGGTACAGC TTATTCCCAT GAACAGCGCG TACTCGCCCA 100
<210> 3399
<211> 100
<212> DNA
<213> Escherichia coli K-12 MG1655
<220> umud b1184
<222> 106-205
<223> umud b1184 U00096 1229990..1230409
<313> 3399: 162-181
<400> 3399
CAGCGCATCG ATCTGAATCA ACTGTTGATC CAGCATCCCA GCGCGACTTA 50
CTTCGTCAAA GCAAGTGGTG ATTCTATGAT TGATGGTGGA ATTAGTGACG 100
<210> 3400
<211> 100
<212> DNA
<213> Escherichia coli K-12 MG1655
<220> sspa b3230
<222> 517-616
<223> sspa b3230 U00096 complement(3374417..3375055)
<313> 3400: 583-602
<400> 3400
ATCGAGTTCA GCGGCCCGGG TGCGAAAGAG CTGAAAGGCT ATATGACCCG 50
CGTCTTTGAG CGTGACTCTT TCCTTGCTTC TTTAACTGAA GCAGAACGTG 100
<210> 3401
<211> 100
<212> DNA
<213> Escherichia coli K-12 MG1655
<220> sspa b3230
<222> 390-489
<223> sspa b3230 U00096 complement(3374417..3375055)
<313> 3401: 435-454
<400> 3401
GCAACTGCGC GAAGAACTGC TGGCGATTGC GCCGGTCTTC GGTCAGAAGC 50
CGTACTTCCT GAGCGATGAG TTCAGCCTGG TCGATTGCTA TCTTGCTCCG 100
<210> 3402
<211> 100

<212> DNA
<213> Escherichia coli K-12 MG1655
<220> sspa b3230
<222> 334-433
<223> sspa b3230 U00096 complement(3374417..3375055)
<313> 3402: 375-394
<400> 3402
ACGCTGATGA ACACCATCAT CAACGGTTCA GCTTCTGAAG CAGATGCCGC 50
ACGTAAGCAA CTGCGCGAAG AACTGCTGGC GATTGCGCCG GTCTTCGGTC 100
<210> 3403
<211> 100
<212> DNA
<213> Escherichia coli K-12 MG1655
<220> sspb b3229
<222> 386-485
<223> sspb b3229 U00096 complement(3373914..3374411)
<313> 3403: 438-457
<400> 3403
TTATGTCGGT TATTGATGGC GACAAGCCAG ATCACGATGA TGACACTCAT 50
CCTGACGATG AACCTCCGCA GCCACCACGC GGTGGTCGAC CGGCATTACG 100
<210> 3404
<211> 100
<212> DNA
<213> Escherichia coli K-12 MG1655
<220> sspb b3229
<222> 324-423
<223> sspb b3229 U00096 complement(3373914..3374411)
<313> 3404: 365-384
<400> 3404
TGCCTACGAT GAAGATACCA GCATCATGAA TGATGAAGAG GCATCGGCAG 50
ACAACGAAAC CGTTATGTCG GTTATTGATG GCGACAAGCC AGATCACGAT 100
<210> 3405
<211> 100
<212> DNA
<213> Escherichia coli K-12 MG1655
<220> sspb b3229
<222> 295-394
<223> sspb b3229 U00096 complement(3373914..3374411)
<313> 3405: 317-336
<400> 3405
GGCGCAGGCA CGATGTTTGA GCCTGAAGCT GCCTACGATG AAGATACCAG 50
CATCATGAAT GATGAAGAGG CATCGGCAGA CAACGAAACC GTTATGTCGG 100
<210> 3406
<211> 100
<212> DNA
<213> Escherichia coli K-12 MG1655
<220> hflb b3179
<222> 1831-1930
<223> hflb b3179 U00096 complement(3322642..3324576)
<313> 3406: 1865-1884
<400> 3406
AACAATTCTG GCGACAATGG TAGTCCAAAG GCTCCTCGTC CGGTTGATGA 50
ACCGCGTACG CCGAACCCGG GTAACACCAT GTCAGAGCAG TTAGGCGACA 100
<210> 3407
<211> 100
<212> DNA
<213> Escherichia coli K-12 MG1655
<220> hflb b3179
<222> 1732-1831
<223> hflb b3179 U00096 complement(3322642..3324576)
<313> 3407: 1759-1778
<400> 3407
CTCATGAAAT ATGAGACTAT CGACGCACCG CAGATTGATG ACCTGATGGC 50
ACGTCGCGAT GTACGTCCGC CAGCGGGCTG GGAAGAACCA GGCGCTTCTA 100
<210> 3408
<211> 100
<212> DNA
<213> Escherichia coli K-12 MG1655
<220> hflb b3179
<222> 1587-1686
<223> hflb b3179 U00096 complement(3322642..3324576)

<313> 3408: 1650-1669
<400> 3408
TAGCGTAGCG AAAGCGAAAC ATATGTCCGA TGAAACTGCA CGTATCATCG 50
ACCAGGAAGT GAAAGCACTG ATTGAGCGTA ACTATAATCG TGCGCGTCAG 100
<210> 3409
<211> 100
<212> DNA
<213> Escherichia coli K-12 MG1655
<220> ybaa b0457
<222> 255-354
<223> ybaa b0457 U00096 475896..476249
<313> 3409: 286-305
<400> 3409
GATGATGTCG GACCCACGGA TGAAAGAGTT CGGCGAGTCC ATGCCGTTTG 50
ACGGCAAGCG AATGATCTAT GGCGGATTCG AGTCAATCAT CGACGAATAG 100
<210> 3410
<211> 100
<212> DNA
<213> Escherichia coli K-12 MG1655
<220> ybaa b0457
<222> 185-284
<223> ybaa b0457 U00096 475896..476249
<313> 3410: 225-244
<400> 3410
AGAATGAAGA GGTTGTCTTT AGCTGGATTG AATACCCTTC AAAAGAGGTC 50
CGCGACGCTG CTAATCAAAA GATGATGTCG GACCCACGGA TGAAAGAGTT 100
<210> 3411
<211> 100
<212> DNA
<213> Escherichia coli K-12 MG1655
<220> ybaa b0457
<222> 144-243
<223> ybaa b0457 U00096 475896..476249
<313> 3411: 188-207
<400> 3411
TGGCAAAGTG ACCGATTTTC GTATGGCGGT GAAAGCGGAA GAGAATGAAG 50
AGGTTGTCTT TAGCTGGATT GAATACCCTT CAAAAGAGGT CCGCGACGCT 100
<210> 3412
<211> 100
<212> DNA
<213> Escherichia coli K-12 MG1655
<220> hflc b4176
<222> 906-1005
<223> hflc b4176 U00096 4400878..4401882
<313> 3412: 965-984
<400> 3412
TTATGAGAAC AGCTTCTCTG GCAATCAGGA CGTGATGGTC ATGAGCCCGG 50
ATAGCGATTT CTTCCGCTAC ATGAAGACGC CGACTTCCGC AACGCGTTAA 100
<210> 3413
<211> 100
<212> DNA
<213> Escherichia coli K-12 MG1655
<220> hflc b4176
<222> 883-982
<223> hflc b4176 U00096 4400878..4401882
<313> 3413: 907-926
<400> 3413
GCATTCATCC GTAGCCTGCG TGCTTATGAG AACAGCTTCT CTGGCAATCA 50
GGACGTGATG GTCATGAGCC CGGATAGCGA TTTCTTCCGC TACATGAAGA 100
<210> 3414
<211> 100
<212> DNA
<213> Escherichia coli K-12 MG1655
<220> hflc b4176
<222> 792-891
<223> hflc b4176 U00096 4400878..4401882
<313> 3414: 831-850
<400> 3414
TGAGCGTCAG GGCCGCATCA TGCGTGGTGA AGGCGATGCC GAAGCAGCCA 50
AACTGTTTGC TGATGCATTC AGTAAAGATC CGGACTTCTA CGCATTCATC 100
<210> 3415

<211> 100
<212> DNA
<213> Escherichia coli K-12 MG1655
<220> ybab b0472
<222> 220-319
<223> ybab b0472 U00096 493300..493629
<313> 3415: 259-278
<400> 3415
GCTGCAGCAT TCAACGACGC AGCACGTCGT ATTGAAGAAA CGCAGAAAGA 50
AAAAATGGCC TCTGTATCCT CCGGAATGCA GCTGCCGCCT GGCTTTAAGA 100
<210> 3416
<211> 100
<212> DNA
<213> Escherichia coli K-12 MG1655
<220> ybab b0472
<222> 169-268
<223> ybab b0472 U00096 493300..493629
<313> 3416: 198-217
<400> 3416
ATCGACCCGA GCCTGCTGGA AGACGACAAA GAGATGCTGG AAGACCTGGT 50
GGCTGCAGCA TTCAACGACG CAGCACGTCG TATTGAAGAA ACGCAGAAAG 100
<210> 3417
<211> 100
<212> DNA
<213> Escherichia coli K-12 MG1655
<220> ybab b0472
<222> 32-131
<223> ybab b0472 U00096 493300..493629
<313> 3417: 53-72
<400> 3417
TGAAGCAAGC CCAGCAGATG CAAGAAAAAA TGCAGAAAAT GCAGGAAGAG 50
ATCGCGCAGC TGGAAGTCAC CGGCGAATCT GGCGCAGGTC TGGTAAAAGT 100
<210> 3418
<211> 100
<212> DNA
<213> Escherichia coli K-12 MG1655
<220> ybac b0477
<222> 844-943
<223> ybac b0477 U00096 complement(498238..499197)
<313> 3418: 906-925
<400> 3418
TGTGAGTTCA AACTCTACCC AGGCACGCTG CACGCCTTTT TGCATTATTC 50
ACGGATGATG AAAACCGCCG ACGAGGCTCT TCGCGACGGC GCTCAGTTCT 100
<210> 3419
<211> 100
<212> DNA
<213> Escherichia coli K-12 MG1655
<220> ybac b0477
<222> 822-921
<223> ybac b0477 U00096 complement(498238..499197)
<313> 3419: 855-874
<400> 3419
GTTAGCGGCG CATCAGCAGC CCTGTGAGTT CAAACTCTAC CCAGGCACGC 50
TGCACGCCTT TTTGCATTAT TCACGGATGA TGAAAACCGC CGACGAGGCT 100
<210> 3420
<211> 100
<212> DNA
<213> Escherichia coli K-12 MG1655
<220> ybac b0477
<222> 747-846
<223> ybac b0477 U00096 complement(498238..499197)
<313> 3420: 790-809
<400> 3420
CGAAGTTCCG CCCTGTTTTA TTGCCGGGGC GGAGTTCGAT CCGCTGCTGG 50
ATGACAGCCG TCTGCTTTAC CAGACGTTAG CGGCGCATCA GCAGCCCTGT 100
<210> 3421
<211> 100
<212> DNA
<213> Escherichia coli K-12 MG1655
<220> ybad b0414
<222> 345-444

<223> ybad b0414 U00096 432226..432675
<313> 3421: 384-403
<400> 3421
ATTGAAAAAG CTCGATAAAG TCGCCTATAT CCGTTTTGCC TCTGTCTACC 50
GCAGTTTCGA AGATATCAAA GAATTTGGCG AAGAGATCGC GCGCCTGGAG 100
<210> 3422
<211> 100
<212> DNA
<213> Escherichia coli K-12 MG1655
<220> ybad b0414
<222> 287-386
<223> ybad b0414 U00096 432226..432675
<313> 3422: 327-346
<400> 3422
GCGCCACCGG TGAGCGCGAA GTGCCGAGCA AGATGATTGG CAATCTGGTG 50
ATGGAGCAAT TGAAAAAGCT CGATAAAGTC GCCTATATCC GTTTTGCCTC 100
<210> 3423
<211> 100
<212> DNA
<213> Escherichia coli K-12 MG1655
<220> ybad b0414
<222> 122-221
<223> ybad b0414 U00096 432226..432675
<313> 3423: 178-197
<400> 3423
TTGAAGTGGC GGAGCTGGTT ATGCCGCGTG TTGTAAAAAG CAACGACGTG 50
CGTGAACCGT TTAATGAAGA GAAATTGCGT AGCGGAATGC TGCGGGCGCT 100
<210> 3424
<211> 100
<212> DNA
<213> Escherichia coli K-12 MG1655
<220> ybae b0446
<222> 1540-1639
<223> ybae b0446 U00096 complement(464836..466536)
<313> 3424: 1575-1594
<400> 3424
CTGAAAGCGG TTTTTAGCCA GTTAATGACA GATGCGACGC TGACGCCGCT 50
GTTCAACTAT CACTATCGCA TTAGTGCCCC TCCCGGCGTG AACGGTGTGC 100
<210> 3425
<211> 100
<212> DNA
<213> Escherichia coli K-12 MG1655
<220> ybae b0446
<222> 1482-1581
<223> ybae b0446 U00096 complement(464836..466536)
<313> 3425: 1527-1546
<400> 3425
ACAATCGACA CTGGATGCCG TGCAAATAAT GCCTGATGAA GAAAACCGAT 50
TTAATGCCCT GAAAGCGGTT TTTAGCCAGT TAATGACAGA TGCGACGCTG 100
<210> 3426
<211> 100
<212> DNA
<213> Escherichia coli K-12 MG1655
<220> ybae b0446
<222> 1424-1523
<223> ybae b0446 U00096 complement(464836..466536)
<313> 3426: 1482-1501
<400> 3426
GGCTGCGCTG CGATCCGCTG TGGCCACATG TTTTCGACGC TCCAGCATAC 50
GCACATCTAC AATCGACACT GGATGCCGTG CAAATAATGC CTGATGAAGA 100
<210> 3427
<211> 100
<212> DNA
<213> Escherichia coli K-12 MG1655
<220> hflk b4175
<222> 1161-1260
<223> hflk b4175 U00096 4399616..4400875
<313> 3427: 1232-1251
<400> 3427
CAGTGGAGCA AGCAACACGT CGTCCACCAG TCAGGGCGAT ATTATGGACC 50
AACGCCGCGC CAACGCGCAG CGTAACGACT ACCAGCGTCA GGGGGAATAA 100

<210> 3428
<211> 100
<212> DNA
<213> Escherichia coli K-12 MG1655
<220> hflk b4175
<222> 1112-1211
<223> hflk b4175 U00096 4399616..4400875
<313> 3428: 1174-1193
<400> 3428
ACGGTGCCAG CAATCTGCTG CGTCTGCCGC CAGCCTCTTC CTCCACAACC 50
AGTGGAGCAA GCAACACGTC GTCCACCAGT CAGGGCGATA TTATGGACCA 100
<210> 3429
<211> 100
<212> DNA
<213> Escherichia coli K-12 MG1655
<220> hflk b4175
<222> 1099-1198
<223> hflk b4175 U00096 4399616..4400875
<313> 3429: 1119-1138
<400> 3429
GCGAAGAGCG ATAACGGTGC CAGCAATCTG CTGCGTCTGC CGCCAGCCTC 50
TTCCTCCACA ACCAGTGGAG CAAGCAACAC GTCGTCCACC AGTCAGGGCG 100
<210> 3430
<211> 100
<212> DNA
<213> Escherichia coli K-12 MG1655
<220> ybaj b0462
<222> 97-196
<223> ybaj b0462 U00096 complement(479558..479932)
<313> 3430: 160-179
<400> 3430
AATCATGGCT GGGTAAACGA CCCAACCTCG GCGATCAACC TCCAGTTGAA 50
TGAACTGATT GAGCATATTG CGACCTTCGC ACTTAATTAC AAAATTAAGT 100
<210> 3431
<211> 100
<212> DNA
<213> Escherichia coli K-12 MG1655
<220> ybaj b0462
<222> 65-164
<223> ybaj b0462 U00096 complement(479558..479932)
<313> 3431: 106-125
<400> 3431
ATCATGACTG CCTTGCAAAC CTTGAAGAAA GCAATCATGG CTGGGTAAAC 50
GACCCAACCT CGGCGATCAA CCTCCAGTTG AATGAACTGA TTGAGCATAT 100
<210> 3432
<211> 100
<212> DNA
<213> Escherichia coli K-12 MG1655
<220> ybaj b0462
<222> 28-127
<223> ybaj b0462 U00096 complement(479558..479932)
<313> 3432: 78-97
<400> 3432
GATATCGCAC AGCTTAAGTT TCTCTGTGAA ACCCTGTATC ATGACTGCCT 50
TGCAAACCTT GAAGAAAGCA ATCATGGCTG GGTAAACGAC CCAACCTCGG 100
<210> 3433
<211> 100
<212> DNA
<213> Escherichia coli K-12 MG1655
<220> ybak b0482
<222> 380-479
<223> ybak b0482 U00096 complement(505827..506306)
<313> 3433: 431-450
<400> 3433
ATGTTTCCGG CGGCAAGCGC GGACTGGATA TCGAACTGGC GGCAGGCGAT 50
CTGGCAAAGA TCCTCGATGC CAAATTTGCT GATATCGCCC GCCGCGATTA 100
<210> 3434
<211> 100
<212> DNA
<213> Escherichia coli K-12 MG1655
<220> ybak b0482

<222> 359-458
<223> ybak b0482 U00096 complement(505827..506306)
<313> 3434: 381-400
<400> 3434
CACAAGAATT TGCCACTATT TATGTTTCCG GCGGCAAGCG CGGACTGGAT 50
ATCGAACTGG CGGCAGGCGA TCTGGCAAAG ATCCTCGATG CCAAATTTGC 100
<210> 3435
<211> 100
<212> DNA
<213> Escherichia coli K-12 MG1655
<220> ybak b0482
<222> 319-418
<223> ybak b0482 U00096 complement(505827..506306)
<313> 3435: 354-373
<400> 3435
GGGCAGAAAA AACGTCTGCC AACGATTATC GACGCCCCCG CACAAGAATT 50
TGCCACTATT TATGTTTCCG GCGGCAAGCG CGGACTGGAT ATCGAACTGG 100
<210> 3436
<211> 100
<212> DNA
<213> Escherichia coli K-12 MG1655
<220> ybal b0479
<222> 1578-1677
<223> ybal b0479 U00096 complement(500786..502462)
<313> 3436: 1628-1647
<400> 3436
ACGTGGTGCG AATCAGGTAG TGATGGGCGA GCGTGAAATC GCCCGTACCA 50
TGCTGGAACT GCTGGAAACG CCACCGGCGG GTGAGGTGGT GACGGGGTAA 100
<210> 3437
<211> 100
<212> DNA
<213> Escherichia coli K-12 MG1655
<220> ybal b0479
<222> 1543-1642
<223> ybal b0479 U00096 complement(500786..502462)
<313> 3437: 1582-1601
<400> 3437
GCCCATTATG ACGATGAAGT GGCGTATATC ACCGAACGTG GTGCGAATCA 50
GGTAGTGATG GGCGAGCGTG AAATCGCCCG TACCATGCTG GAACTGCTGG 100
<210> 3438
<211> 100
<212> DNA
<213> Escherichia coli K-12 MG1655
<220> ybal b0479
<222> 1499-1598
<223> ybal b0479 U00096 complement(500786..502462)
<313> 3438: 1540-1559
<400> 3438
CATCTGCCCG CGCGAAAAAT CCGGATATTG AGATTATTGC CCGCGCCCAT 50
TATGACGATG AAGTGGCGTA TATCACCGAA CGTGGTGCGA ATCAGGTAGT 100
<210> 3439
<211> 100
<212> DNA
<213> Escherichia coli K-12 MG1655
<220> ybam b0467
<222> 25-124
<223> ybam b0467 U00096 complement(489334..489495)
<313> 3439: 76-95
<400> 3439
GATGTCAAAC TGGCTGTCGA TTTGATTGTG CTGCTGGAGG AAAATCAGAT 50
CCCTGCCAGT ACCGTGCTGC GCGCGCTGGA TATTGTAAAG CGTGATTATG 100
<210> 3440
<211> 100
<212> DNA
<213> Escherichia coli K-12 MG1655
<220> ybam b0467
<222> 12-111
<223> ybam b0467 U00096 complement(489334..489495)
<313> 3440: 37-56
<400> 3440
AAATGCCCCT GACGATGTCA AACTGGCTGT CGATTTGATT GTGCTGCTGG 50

AGGAAAATCA GATCCCTGCC AGTACCGTGC TGCGCGCGCT GGATATTGTA 100
<210> 3441
<211> 100
<212> DNA
<213> Escherichia coli K-12 MG1655
<220> ybam b0467
<222> 10-109
<223> ybam b0467 U00096 complement(489334..489495)
<313> 3441: 31-50
<400> 3441
GAAAATGCCC CTGACGATGT CAAACTGGCT GTCGATTTGA TTGTGCTGCT 50
GGAGGAAAAT CAGATCCCTG CCAGTACCGT GCTGCGCGCG CTGGATATTG 100
<210> 3442
<211> 100
<212> DNA
<213> Escherichia coli K-12 MG1655
<220> yban b0469
<222> 279-378
<223> yban b0469 U00096 490106..490483
<313> 3442: 351-370
<400> 3442
GATGCCATGG GTGCGCATCA TGTTGCTGGT AATTCTCGCC TGTTTGCTTT 50
TCTATATGTG GCGAATTCCG GTGATTGATG AAAAGCAAGA AAAGCACTGA 100
<210> 3443
<211> 100
<212> DNA
<213> Escherichia coli K-12 MG1655
<220> yban b0469
<222> 221-320
<223> yban b0469 U00096 490106..490483
<313> 3443: 257-276
<400> 3443
AACCGCGGGC GATTTTGCTT ATTTTGCTCA CGTTTGCCAT TTCTCTGTGG 50
TTCGTCCAGA TGCCATGGGT GCGCATCATG TTGCTGGTAA TTCTCGCCTG 100
<210> 3444
<211> 100
<212> DNA
<213> Escherichia coli K-12 MG1655
<220> yban b0469
<222> 110-209
<223> yban b0469 U00096 490106..490483
<313> 3444: 168-187
<400> 3444
GGTGCTTTGC CCGTTCTTCC CCGCGCTTTC ACGCCTGGTT GCTGTACCGC 50
TCATGGTTTG GCAGCTATCT ACGTTTCTGG CAGAAACATC ATGCGATGCC 100
<210> 3445
<211> 100
<212> DNA
<213> Escherichia coli K-12 MG1655
<220> ybao b0448
<222> 407-506
<223> ybao b0448 U00096 467520..468065
<313> 3445: 447-466
<400> 3445
TGCGCGTCCA GGTTGCCGAC ATGAAACGCT ACGACGAGTT TTATAAGCGT 50
CTGGTAAACA GCGTGCCGGG GCTGTCGGAC GTCACTTCCA GCTTCGCGAT 100
<210> 3446
<211> 100
<212> DNA
<213> Escherichia coli K-12 MG1655
<220> ybao b0448
<222> 326-425
<223> ybao b0448 U00096 467520..468065
<313> 3446: 378-397
<400> 3446
ATTGCCGCTT TGTCACGGTG GTTACCGAAA TGCCAGAAGT GCTGGGGTTC 50
TGGCGCATGG CTGGTGAATA CGATTATCTG ATGCGCGTCC AGGTTGCCGA 100
<210> 3447
<211> 100
<212> DNA
<213> Escherichia coli K-12 MG1655

<220> ybao b0448
<222> 272-371
<223> ybao b0448 U00096 467520..468065
<313> 3447: 316-335
<400> 3447
TCGGCCTGAC CGCTTTTGTG CTGATAAAAA CGCAACATCA CAGCAGCGAA  50
TGGTATTGCC GCTTTGTCAC GGTGGTTACC GAAATGCCAG AAGTGCTGGG  100
<210> 3448
<211> 100
<212> DNA
<213> Escherichia coli K-12 MG1655
<220> ybap b0483
<222> 696-795
<223> ybap b0483 U00096 complement(506510..507304)
<313> 3448: 757-776
<400> 3448
GCGGGATAAA TTACGCGCCA TGCCGCCGGG GCGATATGTG GTCGCGGTCG  50
GTGCACTACA CCTGTATGGA GAAGGGAATT TGCCGCAAAT GTTGCGCTAA  100
<210> 3449
<211> 100
<212> DNA
<213> Escherichia coli K-12 MG1655
<220> ybap b0483
<222> 660-759
<223> ybap b0483 U00096 complement(506510..507304)
<313> 3449: 709-728
<400> 3449
GTACGATGTG CTGATGCATC AGCGAAATCT CGCCTGGCGG GATAAATTAC  50
GCGCCATGCC GCCGGGGCGA TATGTGGTCG CGGTCGGTGC ACTACACCTG  100
<210> 3450
<211> 100
<212> DNA
<213> Escherichia coli K-12 MG1655
<220> ybap b0483
<222> 602-701
<223> ybap b0483 U00096 complement(506510..507304)
<313> 3450: 640-659
<400> 3450
TGAATGCACC GCCGCAAAAT AATGATATAA CGCTGCCCAA TACGTTCAGT  50
CAGTCGCTGT ACGATGTGCT GATGCATCAG CGAAATCTCG CCTGGCGGGA  100
<210> 3451
<211> 100
<212> DNA
<213> Escherichia coli K-12 MG1655
<220> ybaq b0484
<222> 297-396
<223> ybaq b0484 U00096 507388..507783
<313> 3451: 319-338
<400> 3451
AGTTGAAAAC AACATGCGCA CCCAGGAAGA ATTGGGACGG ATTGAAACAG  50
TGGCTGAATA TTTGGCACGC CGTGAAGAGC GTGCAAAAAA GGTCGCGTAA  100
<210> 3452
<211> 100
<212> DNA
<213> Escherichia coli K-12 MG1655
<220> ybaq b0484
<222> 245-344
<223> ybaq b0484 U00096 507388..507783
<313> 3452: 292-311
<400> 3452
ATACCACAGT CGATTTTTGG CTAAACCTCC AGGCGGCGGT TGATCTTTGG  50
GAAGTTGAAA ACAACATGCG CACCCAGGAA GAATTGGGAC GGATTGAAAC  100
<210> 3453
<211> 100
<212> DNA
<213> Escherichia coli K-12 MG1655
<220> ybaq b0484
<222> 176-275
<223> ybaq b0484 U00096 507388..507783
<313> 3453: 206-225
<400> 3453

TCAGTGCACT GATCAATAAC AATCGTAAAC TCACTACTGA GATGGCATTT 50
CGTCTGGCGA AAGTTTTTGA TACCACAGTC GATTTTTGGC TAAACCTCCA 100
<210> 3454
<211> 100
<212> DNA
<213> Escherichia coli K-12 MG1655
<220> b2225 b2225
<222> 613-712
<223> b2225 b2225 U00096 complement(2325387..2326163)
<313> 3454: 660-679
<400> 3454
CAGGTGTATA TCAACTACTA TGGTGGGCGC AGCGAAACGG AATTAACCAC 50
CGCCCAACTG ACGCTGATCA CCGATGAAGG ATCGGTCAAT GAGAAACAGG 100
<210> 3455
<211> 100
<212> DNA
<213> Escherichia coli K-12 MG1655
<220> b2225 b2225
<222> 546-645
<223> b2225 b2225 U00096 complement(2325387..2326163)
<313> 3455: 566-585
<400> 3455
TATGGATGTC ACGACGGGGT ACGGACCCGA GATTTTCGCC ATGCCCGCGC 50
CAATTCACGG GCGCTATCAG GTGTATATCA ACTACTATGG TGGGCGCAGC 100
<210> 3456
<211> 100
<212> DNA
<213> Escherichia coli K-12 MG1655
<220> b2225 b2225
<222> 462-561
<223> b2225 b2225 U00096 complement(2325387..2326163)
<313> 3456: 492-511
<400> 3456
CGACCTCGAC CTTCACGTTG TTACGCCCGA TGGCGAACAC GCCTGGTACG 50
GTAACACCGT GCTGAAAAAC AGTGGTGCAC TGGATATGGA TGTCACGACG 100
<210> 3457
<211> 100
<212> DNA
<213> Escherichia coli K-12 MG1655
<220> ybar b0485
<222> 2406-2505
<223> ybar b0485 U00096 complement(508099..510603)
<313> 3457: 2439-2458
<400> 3457
ACTGCTTAAC CCGGTAGTTG CCGGAGCGGC AATGGCGCTC TCGTCGATTA 50
CCGTAGTGAG TAACGCCAAC CGGTTGCTGC GGTTTAAACC GAAGGAATAA 100
<210> 3458
<211> 100
<212> DNA
<213> Escherichia coli K-12 MG1655
<220> ybar b0485
<222> 2299-2398
<223> ybar b0485 U00096 complement(508099..510603)
<313> 3458: 2324-2343
<400> 3458
TCCCGCGCAA CGCTGCACAA CATGAAGCAG AACCTGCTCG GTGCGTTTAT 50
CTACAACAGT ATCGGTATTC CGGTCGCCGC CGGTATTTTG TGGCCGTTCA 100
<210> 3459
<211> 100
<212> DNA
<213> Escherichia coli K-12 MG1655
<220> ybar b0485
<222> 2244-2343
<223> ybar b0485 U00096 complement(508099..510603)
<313> 3459: 2286-2305
<400> 3459
GGCGATTACC CTGATGCGCC ATAGCCTGAT GGGCGTTGCG GATGCGCTCG 50
CTATTTCCCG CGCAACGCTG CACAACATGA AGCAGAACCT GCTCGGTGCG 100
<210> 3460
<211> 100
<212> DNA

<213> Escherichia coli K-12 MG1655
<220> b2226 b2226
<222> 1551-1650
<223> b2226 b2226 U00096 complement(2326168..2327817)
<313> 3460: 1611-1630
<400> 3460
TTTAACCCAT GAGTATCTGC ACCTGGCCTT TGATGGTTAT CCCACCGGGC 50
TTGATGAGAA CTATATCGAA ACGCTGACCC GACAATTATT AATGGACTAA 100
<210> 3461
<211> 100
<212> DNA
<213> Escherichia coli K-12 MG1655
<220> b2226 b2226
<222> 1536-1635
<223> b2226 b2226 U00096 complement(2326168..2327817)
<313> 3461: 1549-1568
<400> 3461
TCAGGATCGG CTGGATTTAA CCCATGAGTA TCTGCACCTG GCCTTTGATG 50
GTTATCCCAC CGGGCTTGAT GAGAACTATA TCGAAACGCT GACCCGACAA 100
<210> 3462
<211> 100
<212> DNA
<213> Escherichia coli K-12 MG1655
<220> b2226 b2226
<222> 1421-1520
<223> b2226 b2226 U00096 complement(2326168..2327817)
<313> 3462: 1464-1483
<400> 3462
AAGCTGAGAC GGGGTACAAC GAACCAGACG TGTTTGCGGT TTGCCGTTTA 50
GTCTCAGGTT TCCCCTATAC CGATCGGCAG CAGAAACGGC TGTTTATTCG 100
<210> 3463
<211> 100
<212> DNA
<213> Escherichia coli K-12 MG1655
<220> yqcb yqcB
<222> 684-783
<223> yqcb yqcB U00096 complement(2921024..2921806)
<313> 3463: 748-767
<400> 3463
GCAGGCGTTA TCACAATTTG GCTGGCGCGG TCTGCTTCCT GAAAATGAAA 50
GGGTTGAGTT CAGTGCGCCA TCGGGTCAGG ATGGAGAGAT AAGTTCCTAA 100
<210> 3464
<211> 100
<212> DNA
<213> Escherichia coli K-12 MG1655
<220> yqcb yqcB
<222> 684-783
<223> yqcb yqcB U00096 complement(2921024..2921806)
<313> 3464: 710-729
<400> 3464
GCAGGCGTTA TCACAATTTG GCTGGCGCGG TCTGCTTCCT GAAAATGAAA 50
GGGTTGAGTT CAGTGCGCCA TCGGGTCAGG ATGGAGAGAT AAGTTCCTAA 100
<210> 3465
<211> 100
<212> DNA
<213> Escherichia coli K-12 MG1655
<220> yqcb yqcB
<222> 627-726
<223> yqcb yqcB U00096 complement(2921024..2921806)
<313> 3465: 679-698
<400> 3465
GCATCCTTTT ACTGGCGAGC CGCTGACTAT TCACGCGGGC CTGGACGACA 50
CCTGGATGCA GGCGTTATCA CAATTTGGCT GGCGCGGTCT GCTTCCTGAA 100
<210> 3466
<211> 100
<212> DNA
<213> Escherichia coli K-12 MG1655
<220> ybas b0486
<222> 834-933
<223> ybas b0486 U00096 510865..511797
<313> 3466: 884-903

<400> 3466
GTTCTCACCA CCGCTGGACG AAGATGGCAA CAGTGTTCGC GGTCAAAAAA 50
TGGTGGCATC GGTCGCTAAG CAACTCGGCT ATAACGTGTT TAAGGGCTGA 100
<210> 3467
<211> 100
<212> DNA
<213> Escherichia coli K-12 MG1655
<220> ybas b0486
<222> 825-924
<223> ybas b0486 U00096 510865..511797
<313> 3467: 843-862
<400> 3467
AATTGCCGCG TTCTCACCAC CGCTGGACGA AGATGGCAAC AGTGTTCGCG 50
GTCAAAAAAT GGTGGCATCG GTCGCTAAGC AACTCGGCTA TAACGTGTTT 100
<210> 3468
<211> 100
<212> DNA
<213> Escherichia coli K-12 MG1655
<220> ybas b0486
<222> 754-853
<223> ybas b0486 U00096 510865..511797
<313> 3468: 782-801
<400> 3468
TATCGTGTTG GTTTACCGGG CAAAAGCGGT GTAGGTGGCG GTATTCTGGC 50
GGTCGTCCCT GGAGTGATGG GAATTGCCGC GTTCTCACCA CCGCTGGACG 100
<210> 3469
<211> 100
<212> DNA
<213> Escherichia coli K-12 MG1655
<220> b2227 b2227
<222> 387-486
<223> b2227 b2227 U00096 complement(2327818..2328303)
<313> 3469: 453-472
<400> 3469
GCAATTCGTT CTGCCTCCTG CTCGTTATGT GCGTTCCTAT GCACCTGCGC 50
AGCAAAGTGT TGCGGCAGGG AGTGAATGGA CCGGGATGCA GGTGAAATAA 100
<210> 3470
<211> 100
<212> DNA
<213> Escherichia coli K-12 MG1655
<220> b2227 b2227
<222> 383-482
<223> b2227 b2227 U00096 complement(2327818..2328303)
<313> 3470: 401-420
<400> 3470
AAGGGCAATT CGTTCTGCCT CCTGCTCGTT ATGTGCGTTC CTATGCACCT 50
GCGCAGCAAA GTGTTGCGGC AGGGAGTGAA TGGACCGGGA TGCAGGTGAA 100
<210> 3471
<211> 100
<212> DNA
<213> Escherichia coli K-12 MG1655
<220> b2227 b2227
<222> 339-438
<223> b2227 b2227 U00096 complement(2327818..2328303)
<313> 3471: 363-382
<400> 3471
GACGGGAACG GTCACTTTCC GCCATTTGCT GCGCTTCTCG CAAAAAGGGC 50
AATTCGTTCT GCCTCCTGCT CGTTATGTGC GTTCCTATGC ACCTGCGCAG 100
<210> 3472
<211> 100
<212> DNA
<213> Escherichia coli K-12 MG1655
<220> ybat b0487
<222> 1194-1293
<223> ybat b0487 U00096 511800..513092
<313> 3472: 1224-1243
<400> 3472
TCTGTGGTCC CAGGGTAGCC GTGCGTTGAT ATGGATTATT GGCTCACTCT 50
TACTCAGCCT TATTGTGGCA ATGGTCATGA AGCGCAATAA AACCGTATAA 100
<210> 3473
<211> 100

<212> DNA
<213> Escherichia coli K-12 MG1655
<220> ybat b0487
<222> 1099-1198
<223> ybat b0487 U00096 511800..513092
<313> 3473: 1121-1140
<400> 3473
GCGATCCGCC TGCGTCATGA TATTCACGCC TCGTTGCCGA TTCTTATCGT 50
TGGTACGTTG GTGATGTTGT TGGTGATCGT TGGCTTTATC TACAGTCTGT 100
<210> 3474
<211> 100
<212> DNA
<213> Escherichia coli K-12 MG1655
<220> ybat b0487
<222> 1028-1127
<223> ybat b0487 U00096 511800..513092
<313> 3474: 1075-1094
<400> 3474
TGAATTTAGG CTCACTCGCC AGCGTTGCCA GCGCCACCTT TTTGATTTGC 50
TACCTGGCGG TGTTTGTGGT GGCGATCCGC CTGCGTCATG ATATTCACGC 100
<210> 3475
<211> 100
<212> DNA
<213> Escherichia coli K-12 MG1655
<220> b2228 b2228
<222> 3915-4014
<223> b2228 b2228 U00096 complement(2328319..2332332)
<313> 3475: 3990-4009
<400> 3475
AACTGGAGGG GGCGAAGACT GGCGTTGGGT TGGTCAGGGC GTGCCGGACA 50
TTCTCTCTTT TGGTGACGAA TTATCGCCGC AAAATGTGCA GGTCCGCTGA 100
<210> 3476
<211> 100
<212> DNA
<213> Escherichia coli K-12 MG1655
<220> b2228 b2228
<222> 3832-3931
<223> b2228 b2228 U00096 complement(2328319..2332332)
<313> 3476: 3853-3872
<400> 3476
ATGAACTGGC TGGCGAAATA TATGGCGACA ATGCCTCCAG TTGTTTTGCC 50
TGCGCCTGCG GGCGCATGGG CTAAACATAA GTTAACTGGA GGGGGCGAAG 100
<210> 3477
<211> 100
<212> DNA
<213> Escherichia coli K-12 MG1655
<220> b2228 b2228
<222> 3636-3735
<223> b2228 b2228 U00096 complement(2328319..2332332)
<313> 3477: 3686-3705
<400> 3477
GTTGCTGAAA AAAGCGCAGT TGAAGTCCAC GGTGATGCCA CAGGTTCAGC 50
AATATGCGTG GGATAAAGCG GCAAACAGCA ATCAGCCGCT GGCGCACACG 100
<210> 3478
<211> 100
<212> DNA
<213> Escherichia coli K-12 MG1655
<220> yqcd b2795
<222> 750-849
<223> yqcd b2795 U00096 2923370..2924218
<313> 3478: 826-845
<400> 3478
CGTTTACGCA CGTTATACCC GTCGTGGCGG TCTGGACATT AACCCGTGGC 50
GCAGTAATAG CGATTTTGTC CCATCGACCA CAAGACTGGT TCGGCAATAA 100
<210> 3479
<211> 100
<212> DNA
<213> Escherichia coli K-12 MG1655
<220> yqcd b2795
<222> 665-764
<223> yqcd b2795 U00096 2923370..2924218

<313> 3479: 710-729
<400> 3479
TCCGTCATCA CAACGAGTTC CACGAACAGT GCGTGGAACG CATCTTTAAT 50
GACCTGTTAC GCTTCTGCCA GCCAGAAAAA TTGAGCGTTT ACGCACGTTA 100
<210> 3480
<211> 100
<212> DNA
<213> Escherichia coli K-12 MG1655
<220> yqcd b2795
<222> 635-734
<223> yqcd b2795 U00096 2923370..2924218
<313> 3480: 675-694
<400> 3480
GAGAAAAACT GCTGCGTTAC CTGGTCTCAT TCCGTCATCA CAACGAGTTC 50
CACGAACAGT GCGTGGAACG CATCTTTAAT GACCTGTTAC GCTTCTGCCA 100
<210> 3481
<211> 100
<212> DNA
<213> Escherichia coli K-12 MG1655
<220> ybau b0442
<222> 1773-1872
<223> ybau b0442 U00096 461139..463010
<313> 3481: 1842-1861
<400> 3481
TATCACCCAG AACAACGCAC AAATCGTCTT TGAAGCTCTG ATGAGTAACC 50
TGCGTAAAGA GGCGAAAATC AAAATTGGCG ATGCGCTGGA ACAGCAATAA 100
<210> 3482
<211> 100
<212> DNA
<213> Escherichia coli K-12 MG1655
<220> ybau b0442
<222> 1750-1849
<223> ybau b0442 U00096 461139..463010
<313> 3482: 1781-1800
<400> 3482
CAGAAAAAAG CGATGGTGCA GGGTATCACC CAGAACAACG CACAAATCGT 50
CTTTGAAGCT CTGATGAGTA ACCTGCGTAA AGAGGCGAAA ATCAAAATTG 100
<210> 3483
<211> 100
<212> DNA
<213> Escherichia coli K-12 MG1655
<220> ybau b0442
<222> 1686-1785
<223> ybau b0442 U00096 461139..463010
<313> 3483: 1731-1750
<400> 3483
TATGCAAGGT AATGTGGTTC TGCTGGCGCT GGATGAAGTG AAACAAGGTT 50
CAATGCCGGA AGATCAGAAA AAAGCGATGG TGCAGGGTAT CACCCAGAAC 100
<210> 3484
<211> 100
<212> DNA
<213> Escherichia coli K-12 MG1655
<220> b2229 b2230
<222> 552-651
<223> b2229 b2230 U00096 complement(2332356..2333006)
<313> 3484: 597-616
<400> 3484
TCTGCAACAA CTTATGACAT GGAAGGACAC CCGATGGATA CCCAACGATT 50
CCAATCCCAA TTTCATTGGC ATTTATCGTT TAAATTTTCT GGCGCGATAG 100
<210> 3485
<211> 100
<212> DNA
<213> Escherichia coli K-12 MG1655
<220> b2229 b2230
<222> 512-611
<223> b2229 b2230 U00096 complement(2332356..2333006)
<313> 3485: 559-578
<400> 3485
GAAGCGCCAC GAAAACTGAC AACGGAATGC GCGCAGTCAG TCTGCAACAA 50
CTTATGACAT GGAAGGACAC CCGATGGATA CCCAACGATT CCAATCCCAA 100
<210> 3486

<211> 100
<212> DNA
<213> Escherichia coli K-12 MG1655
<220> b2229 b2230
<222> 459-558
<223> b2229 b2230 U00096 complement(2332356..2333006)
<313> 3486: 503-522
<400> 3486
CGATGCCCAG CACTTAATGG TCTGGATGGG GCGTTACGTC ATCTACCACA 50
CCGGAAGCGC CACGAAAACT GACAACGGAA TGCGCGCAGT CAGTCTGCAA 100
<210> 3487
<211> 100
<212> DNA
<213> Escherichia coli K-12 MG1655
<220> yqce b2776
<222> 1179-1278
<223> yqce b2776 U00096 2898614..2899891
<313> 3487: 1246-1265
<400> 3487
GGGCATGGCG GCGCTTGGCA TGGTGATTGT CTTTACCTTT TTGCTGTTCC 50
AAAAAATTCG TACTGCTGAT AGCGCCCCCG CAATGGCTAG CAGCAAGTAA 100
<210> 3488
<211> 100
<212> DNA
<213> Escherichia coli K-12 MG1655
<220> yqce b2776
<222> 1135-1234
<223> yqce b2776 U00096 2898614..2899891
<313> 3488: 1169-1188
<400> 3488
GACACCCTGC CCGCTGCAGA AGCCTACCGC AATATGTGGC TGATGGGCAT 50
GGCGGCGCTT GGCATGGTGA TTGTCTTTAC CTTTTTGCTG TTCCAAAAAA 100
<210> 3489
<211> 100
<212> DNA
<213> Escherichia coli K-12 MG1655
<220> yqce b2776
<222> 1065-1164
<223> yqce b2776 U00096 2898614..2899891
<313> 3489: 1132-1151
<400> 3489
GGTAGGTATT TGTTCGGTGA TTGGATTCCT GCCGGATGTC TTCGTTTACC 50
CAATTATCGG CCACTGGCAA GACACCCTGC CCGCTGCAGA AGCCTACCGC 100
<210> 3490
<211> 100
<212> DNA
<213> Escherichia coli K-12 MG1655
<220> ybav b0443
<222> 263-362
<223> ybav b0443 U00096 463161..463532
<313> 3490: 317-336
<400> 3490
TCAGTTATCG CGAAGAGTAC GGTCCGTTTA AAACTGTGGA GGATCTAAAG 50
CAGGTGCCGG GGATGGGCAA TTCGCTGGTG GAACGTAATC TGGCGGTATT 100
<210> 3491
<211> 100
<212> DNA
<213> Escherichia coli K-12 MG1655
<220> ybav b0443
<222> 197-296
<223> ybav b0443 U00096 463161..463532
<313> 3491: 232-251
<400> 3491
ATGCCAGCGC GGAAGAGCTA GCCCGCGCGA TGAATGGCGT TGGCCTGAAG 50
AAAGCGCAGG CGATTGTCAG TTATCGCGAA GAGTACGGTC CGTTTAAAAC 100
<210> 3492
<211> 100
<212> DNA
<213> Escherichia coli K-12 MG1655
<220> ybav b0443
<222> 110-209

<223> ybav b0443 U00096 463161..463532
<313> 3492: 154-173
<400> 3492
CCAAAGCGGA AGCTCCTGCA GCACAAAGTA AAGCAGCAGT ACCGGCGAAA 50
GCCAGTGACG AAGAAGGCAC CCGGGTCAGC ATTAATAATG CCAGCGCGGA 100
<210> 3493
<211> 100
<212> DNA
<213> Escherichia coli K-12 MG1655
<220> hflx b4174
<222> 1172-1271
<223> hflx b4174 U00096 4398250..4399530
<313> 3493: 1200-1219
<400> 3493
AAGAGTGGAT GGAGGAGGAC GGCAGCGTAA GTCTGCAAGT TCGTATGCCG 50
ATCGTTGACT GGCGTCGCCT CTGTAAACAA GAACCGGCGT TGATCGATTA 100
<210> 3494
<211> 100
<212> DNA
<213> Escherichia coli K-12 MG1655
<220> hflx b4174
<222> 1027-1126
<223> hflx b4174 U00096 4398250..4399530
<313> 3494: 1084-1103
<400> 3494
TCCGCACAGA CCGGAGCGGG GATACCACAG CTTTTTCAGG CTTTGACGGA 50
GCGGCTTTCC GGCGAGGTGG CGCAGCATAC ATTGCGTCTG CCACCGCAGG 100
<210> 3495
<211> 100
<212> DNA
<213> Escherichia coli K-12 MG1655
<220> hflx b4174
<222> 998-1097
<223> hflx b4174 U00096 4398250..4399530
<313> 3495: 1031-1050
<400> 3495
AAGAGAACAA ACCGAACCGT GTCTGGCTTT CCGCACAGAC CGGAGCGGGG 50
ATACCACAGC TTTTTCAGGC TTTGACGGAG CGGCTTTCCG GCGAGGTGGC 100
<210> 3496
<211> 100
<212> DNA
<213> Escherichia coli K-12 MG1655
<220> ybaw b0444
<222> 279-378
<223> ybaw b0444 U00096 463626..464024
<313> 3496: 305-324
<400> 3496
GCCGGAAGGG CAGGTGGTAG CGGATGCGCT TATTACGTTT GTTTGTATTG 50
ATCTTAAAAC GCAGAAAGCA TTAGCTCTGG AAGGGGAATT GCGCGAAAAG 100
<210> 3497
<211> 100
<212> DNA
<213> Escherichia coli K-12 MG1655
<220> ybaw b0444
<222> 216-315
<223> ybaw b0444 U00096 463626..464024
<313> 3497: 255-274
<400> 3497
TACCAGTCAG TTGCAGCAAT TAAACGGTAA AAGCGGCATC TTAAGCCAGG 50
TCATTACACT GGAGCCGGAA GGGCAGGTGG TAGCGGATGC GCTTATTACG 100
<210> 3498
<211> 100
<212> DNA
<213> Escherichia coli K-12 MG1655
<220> ybaw b0444
<222> 144-243
<223> ybaw b0444 U00096 463626..464024
<313> 3498: 189-208
<400> 3498
CATCGCCTTC GTCGTGGTCA ATATCAATAT TAACTATCGT CGCCCAGCGG 50
TATTAAGTGA CCTGTTAACT ATTACCAGTC AGTTGCAGCA ATTAAACGGT 100

<210> 3499
<211> 100
<212> DNA
<213> Escherichia coli K-12 MG1655
<220> ybax b0445
<222> 597-696
<223> ybax b0445 U00096 complement(464076..464771)
<313> 3499: 676-695
<400> 3499
TTGTGCGGCA TGTAATTTAC GCGCCAACGG TTTGAATCAT TATCTGGCCG 50
ATAAACCGAC GGTGATGGCA GCGATGAAGC AGAAAACCGG GTTGAGGTAA 100
<210> 3500
<211> 100
<212> DNA
<213> Escherichia coli K-12 MG1655
<220> ybax b0445
<222> 569-668
<223> ybax b0445 U00096 complement(464076..464771)
<313> 3500: 598-617
<400> 3500
ACGGCTTTAA AGGCGACGGT TGCGGTCATT GTGCGGCATG TAATTTACGC 50
GCCAACGGTT TGAATCATTA TCTGGCCGAT AAACCGACGG TGATGGCAGC 100
<210> 3501
<211> 100
<212> DNA
<213> Escherichia coli K-12 MG1655
<220> ybax b0445
<222> 491-590
<223> ybax b0445 U00096 complement(464076..464771)
<313> 3501: 556-575
<400> 3501
ATAAAGCGGA AACCTGGGCG CTGGCAGATT ATTACGGCAA ACTGGATTTA 50
GTCCGTAACG AAACGTTGAC CTGCTATAAC GGCTTTAAAG GCGACGGTTG 100
<210> 3502
<211> 100
<212> DNA
<213> Escherichia coli K-12 MG1655
<220> ybay b0454
<222> 474-573
<223> ybay b0454 U00096 474603..475175
<313> 3502: 537-556
<400> 3502
GCCGGTTCAG GCCAGCGGTG GCGCAACGAC TACCGTACCT TCGACTTCAC 50
CAACTCAGGT GAATCCGTCT TCGGCAGTTC CAGCTCCTAC GCAATATTAA 100
<210> 3503
<211> 100
<212> DNA
<213> Escherichia coli K-12 MG1655
<220> ybay b0454
<222> 474-573
<223> ybay b0454 U00096 474603..475175
<313> 3503: 510-529
<400> 3503
GCCGGTTCAG GCCAGCGGTG GCGCAACGAC TACCGTACCT TCGACTTCAC 50
CAACTCAGGT GAATCCGTCT TCGGCAGTTC CAGCTCCTAC GCAATATTAA 100
<210> 3504
<211> 100
<212> DNA
<213> Escherichia coli K-12 MG1655
<220> ybay b0454
<222> 400-499
<223> ybay b0454 U00096 474603..475175
<313> 3504: 420-439
<400> 3504
ACCGTTCAGC CGGTGATCAA CCAGGGCGGA ACTAAAGCCG ACCTGACATT 50
GGTGCCGGTA CAGCAAACCG CCGTGCCGGT TCAGGCCAGC GGTGGCGCAA 100
<210> 3505
<211> 100
<212> DNA
<213> Escherichia coli K-12 MG1655
<220> ybaz b0455

<222> 291-390
<223> ybaz b0455 U00096 complement(475206..475595)
<313> 3505: 363-382
<400> 3505
ACCGGATTTA CAGCGTCAGC GACAGGCATT ACTGGCAGAA GGTGTGATGG 50
TATCGGGAAG CGGGCAAATC GACTTGCAGC GTTATCGCTG GAACTACTAA 100
<210> 3506
<211> 100
<212> DNA
<213> Escherichia coli K-12 MG1655
<220> ybaz b0455
<222> 270-369
<223> ybaz b0455 U00096 complement(475206..475595)
<313> 3506: 314-333
<400> 3506
CGGCACAATT TCGCTAACCG GACCGGATTT ACAGCGTCAG CGACAGGCAT 50
TACTGGCAGA AGGTGTGATG GTATCGGGAA GCGGGCAAAT CGACTTGCAG 100
<210> 3507
<211> 100
<212> DNA
<213> Escherichia coli K-12 MG1655
<220> ybaz b0455
<222> 242-341
<223> ybaz b0455 U00096 complement(475206..475595)
<313> 3507: 288-307
<400> 3507
TACCCTGGCA CCGGGTGGTT AATCGCCACG GCACAATTTC GCTAACCGGA 50
CCGGATTTAC AGCGTCAGCG ACAGGCATTA CTGGCAGAAG GTGTGATGGT 100
<210> 3508
<211> 100
<212> DNA
<213> Escherichia coli K-12 MG1655
<220> kefb b3351
<222> 1707-1806
<223> kefb b3351 U00096 complement(3476439..3478244)
<313> 3508: 1748-1767
<400> 3508
TTCTCGCGCC AGGGAAGCCC GACGCGAACT GGAAGAGATT TTCCAGCGTG 50
AAATGCAACA AGAACGACGC CAGCTGGACG GCTGGGATGA ATTTGAGTAG 100
<210> 3509
<211> 100
<212> DNA
<213> Escherichia coli K-12 MG1655
<220> kefb b3351
<222> 1666-1765
<223> kefb b3351 U00096 complement(3476439..3478244)
<313> 3509: 1709-1728
<400> 3509
CTGCGAGAGC TCATCCCAAT GCATGCCGAT ACCGTACAAA TTTCTCGCGC 50
CAGGGAAGCC CGACGCGAAC TGGAAGAGAT TTTCCAGCGT GAAATGCAAC 100
<210> 3510
<211> 100
<212> DNA
<213> Escherichia coli K-12 MG1655
<220> kefb b3351
<222> 1605-1704
<223> kefb b3351 U00096 complement(3476439..3478244)
<313> 3510: 1637-1656
<400> 3510
CATGCATCCG CATCAGGCAC AGCGAGCGCA ACTGCATTTT CGCCGCCTGG 50
ATATGCGGAT GCTGCGAGAG CTCATCCCAA TGCATGCCGA TACCGTACAA 100
<210> 3511
<211> 100
<212> DNA
<213> Escherichia coli K-12 MG1655
<220> kefc b0048
<222> 1764-1863
<223> kefc b0048 U00096 47769..49631
<313> 3511: 1825-1844
<400> 3511
ACATCTGTCA TTAATTCAAC GACATGGCTG GCAGGGAACC GAAGAAGGTA 50

AACATACCGG CAACATGGCG GATGAACCGG AAACGAAACC CTCATCCTAA 100
<210> 3512
<211> 100
<212> DNA
<213> Escherichia coli K-12 MG1655
<220> kefc b0048
<222> 1737-1836
<223> kefc b0048 U00096 47769..49631
<313> 3512: 1796-1815
<400> 3512
AAGTGAGATC ATTACCGAGG ACCGCGAACA TCTGTCATTA ATTCAACGAC 50
ATGGCTGGCA GGGAACCGAA GAAGGTAAAC ATACCGGCAA CATGGCGGAT 100
<210> 3513
<211> 100
<212> DNA
<213> Escherichia coli K-12 MG1655
<220> kefc b0048
<222> 1713-1812
<223> kefc b0048 U00096 47769..49631
<313> 3513: 1746-1765
<400> 3513
CTATAAACGC ACCAGCGCGA TGTTAAGTGA GATCATTACC GAGGACCGCG 50
AACATCTGTC ATTAATTCAA CGACATGGCT GGCAGGGAAC CGAAGAAGGT 100
<210> 3514
<211> 100
<212> DNA
<213> Escherichia coli K-12 MG1655
<220> flga b1073
<222> 528-627
<223> flga b1073 U00096 complement(1129427..1130086)
<313> 3514: 554-573
<400> 3514
GTTTAGCGCC AACGCAGAAG GTCAGGCGCT GAACAATGCA GCCGTCGCAC 50
AGAATGCGCG GGTGCGCATG GTATCGGGAC AGGTAGTCAG CGGCGTTGTT 100
<210> 3515
<211> 100
<212> DNA
<213> Escherichia coli K-12 MG1655
<220> flga b1073
<222> 466-565
<223> flga b1073 U00096 complement(1129427..1130086)
<313> 3515: 526-545
<400> 3515
TTTCGCCAGG CATGGCGGGT AAAAGCGGGA CAACGCGTCA ATGTGATCGC 50
CAGCGGTGAT GGGTTTAGCG CCAACGCAGA AGGTCAGGCG CTGAACAATG 100
<210> 3516
<211> 100
<212> DNA
<213> Escherichia coli K-12 MG1655
<220> flga b1073
<222> 394-493
<223> flga b1073 U00096 complement(1129427..1130086)
<313> 3516: 453-472
<400> 3516
GATATCAATC AACTTGTTGA TGCCATTAGC CTGCGCGATC TATCACCCGA 50
TCAACCTATC CAGTTAACCC AGTTTCGCCA GGCATGGCGG GTAAAAGCGG 100
<210> 3517
<211> 100
<212> DNA
<213> Escherichia coli K-12 MG1655
<220> flgb b1074
<222> 276-375
<223> flgb b1074 U00096 1130241..1130657
<313> 3517: 328-347
<400> 3517
GCCTTCGCTT GACGGTAATA CCGTCGATAT GGATCGCGAA CGCACCCAGT 50
TTGCCGATAA CAGCCTGCAA TACCAGATGA GCCTTAGCGC GTTGAGCGGG 100
<210> 3518
<211> 100
<212> DNA
<213> Escherichia coli K-12 MG1655

<220> flgb b1074
<222> 198-297
<223> flgb b1074 U00096 1130241..1130657
<313> 3518: 248-267
<400> 3518
GATGACCTCA ACGCAACACA TTCCGGCGCA GGCGCTGACG CCTCCTACCG 50
CAGAACTGCA ATACCGTATT CCGGACCAGC CTTCGCTTGA CGGTAATACC 100
<210> 3519
<211> 100
<212> DNA
<213> Escherichia coli K-12 MG1655
<220> flgb b1074
<222> 172-271
<223> flgb b1074 U00096 1130241..1130657
<313> 3519: 193-212
<400> 3519
GATGCAACCA GTGTGGTTGC ACTGACGATG ACCTCAACGC AACACATTCC 50
GGCGCAGGCG CTGACGCCTC CTACCGCAGA ACTGCAATAC CGTATTCCGG 100
<210> 3520
<211> 100
<212> DNA
<213> Escherichia coli K-12 MG1655
<220> flgc b1075
<222> 306-405
<223> flgc b1075 U00096 1130661..1131065
<313> 3520: 376-395
<400> 3520
GGTTAACACC ATGTCGGCGT CACGCAGCTA TCAGGCCAAT GTTGAAGTGC 50
TCAACACGGT GAAAAGCATG ATGCTGAAAA CCCTTACGCT CGGTCAATAA 100
<210> 3521
<211> 100
<212> DNA
<213> Escherichia coli K-12 MG1655
<220> flgc b1075
<222> 220-319
<223> flgc b1075 U00096 1130661..1131065
<313> 3521: 261-280
<400> 3521
GACAAACTGG TTTATGAACC GGGTAATCCG CTGGCAGATG CAAAGGGCTA 50
CGTAAAAATG CCGAACGTTG ATGTTGTCGG AGAGATGGTT AACACCATGT 100
<210> 3522
<211> 100
<212> DNA
<213> Escherichia coli K-12 MG1655
<220> flgc b1075
<222> 179-278
<223> flgc b1075 U00096 1130661..1131065
<313> 3522: 199-218
<400> 3522
GCGGCGTAAA GGTTGCCGAT GTTATAGAAA GTCAGGCCCC GGACAAACTG 50
GTTTATGAAC CGGGTAATCC GCTGGCAGAT GCAAAGGGCT ACGTAAAAAT 100
<210> 3523
<211> 100
<212> DNA
<213> Escherichia coli K-12 MG1655
<220> ptxa b4196
<222> 366-465
<223> ptxa b4196 U00096 4419286..4419750
<313> 3523: 434-453
<400> 3523
CAACCTGTTT GAAGATGAAG AGAATTTTGA CCGTTTACGC GCCTGCCGTA 50
CCGAGCAGGA AGTACTGGAT CTCATTGACC GCACCAACGC GGCAGCTTAA 100
<210> 3524
<211> 100
<212> DNA
<213> Escherichia coli K-12 MG1655
<220> ptxa b4196
<222> 336-435
<223> ptxa b4196 U00096 4419286..4419750
<313> 3524: 394-413
<400> 3524

TCACCAGGAA GTGGGCATCA TGCAGATCGT CAACCTGTTT GAAGATGAAG 50
AGAATTTTGA CCGTTTACGC GCCTGCCGTA CCGAGCAGGA AGTACTGGAT 100
<210> 3525
<211> 100
<212> DNA
<213> Escherichia coli K-12 MG1655
<220> ptxa b4196
<222> 331-430
<223> ptxa b4196 U00096 4419286..4419750
<313> 3525: 348-367
<400> 3525
AATACTCACC AGGAAGTGGG CATCATGCAG ATCGTCAACC TGTTTGAAGA 50
TGAAGAGAAT TTTGACCGTT TACGCGCCTG CCGTACCGAG CAGGAAGTAC 100
<210> 3526
<211> 100
<212> DNA
<213> Escherichia coli K-12 MG1655
<220> flgd b1076
<222> 597-696
<223> flgd b1076 U00096 1131077..1131772
<313> 3526: 676-695
<400> 3526
TGCTCTGGTG CAGGGTGTGA TCCGCGGCAA CAGCGGTAAT ACGCTGGATC 50
TCGGCACTTA CGGCACCACC ACCCTCGACG AAGTACGGCA GATAATTTAA 100
<210> 3527
<211> 100
<212> DNA
<213> Escherichia coli K-12 MG1655
<220> flgd b1076
<222> 496-595
<223> flgd b1076 U00096 1131077..1131772
<313> 3527: 547-566
<400> 3527
GGTACGTTGA CTGATGGCAG CACTGCGCCG AACGGTTCTT ACAATGTAGC 50
GATTAGCGCC AGTAACGGTG GTACACAACT GGTTGCCCAG CCGCTGCAGT 100
<210> 3528
<211> 100
<212> DNA
<213> Escherichia coli K-12 MG1655
<220> flgd b1076
<222> 476-575
<223> flgd b1076 U00096 1131077..1131772
<313> 3528: 514-533
<400> 3528
TTCACAGTTT CACCTGGGAC GGTACGTTGA CTGATGGCAG CACTGCGCCG 50
AACGGTTCTT ACAATGTAGC GATTAGCGCC AGTAACGGTG GTACACAACT 100
<210> 3529
<211> 100
<212> DNA
<213> Escherichia coli K-12 MG1655
<220> ybba b0496
<222> 586-685
<223> ybba b0496 U00096 518957..519643
<313> 3529: 638-657
<400> 3529
CATGGCACCA CGTTGATTAT GGTGACCCAC GACCTGCAAC TGGCGGCACG 50
CTGCGACCGC TGCTTACGGC TGGTGAACGG GCAGTTGCAG GAGGAAGCAT 100
<210> 3530
<211> 100
<212> DNA
<213> Escherichia coli K-12 MG1655
<220> ybba b0496
<222> 554-653
<223> ybba b0496 U00096 518957..519643
<313> 3530: 607-626
<400> 3530
TTGCCGACCT GCTGTTTTCC CTCAACCGTG AACATGGCAC CACGTTGATT 50
ATGGTGACCC ACGACCTGCA ACTGGCGGCA CGCTGCGACC GCTGCTTACG 100
<210> 3531
<211> 100
<212> DNA

<213> Escherichia coli K-12 MG1655
<220> ybba b0496
<222> 517-616
<223> ybba b0496 U00096 518957..519643
<313> 3531: 567-586
<400> 3531
CCCACCGGCA ACCTTGACCG CCAGACGGGC GATAAAATTG CCGACCTGCT 50
GTTTTCCCTC AACCGTGAAC ATGGCACCAC GTTGATTATG GTGACCCACG 100
<210> 3532
<211> 100
<212> DNA
<213> Escherichia coli K-12 MG1655
<220> flge b1077
<222> 1110-1209
<223> flge b1077 U00096 1131797..1133005
<313> 3532: 1174-1193
<400> 3532
ACTGGTCAAT ATGATCGTTG CCCAGCGTAA CTATCAGTCT AACGCCCAGA 50
CCATCAAAAC CCAGGACCAG ATCCTCAACA CGCTGGTTAA CTTACGCTAA 100
<210> 3533
<211> 100
<212> DNA
<213> Escherichia coli K-12 MG1655
<220> flge b1077
<222> 1057-1156
<223> flge b1077 U00096 1131797..1133005
<313> 3533: 1090-1109
<400> 3533
GGCACCCTGA CCAACGGTGC GCTGGAAGCG TCCAACGTCG ATCTCAGTAA 50
AGAACTGGTC AATATGATCG TTGCCCAGCG TAACTATCAG TCTAACGCCC 100
<210> 3534
<211> 100
<212> DNA
<213> Escherichia coli K-12 MG1655
<220> flge b1077
<222> 1022-1121
<223> flge b1077 U00096 1131797..1133005
<313> 3534: 1050-1069
<400> 3534
TGGCGCTGTT GGGGACAGCC GGGACGGGAA ACTTTGGCAC CCTGACCAAC 50
GGTGCGCTGG AAGCGTCCAA CGTCGATCTC AGTAAAGAAC TGGTCAATAT 100
<210> 3535
<211> 100
<212> DNA
<213> Escherichia coli K-12 MG1655
<220> ybbb b0504
<222> 996-1095
<223> ybbb b0504 U00096 complement(529356..530450)
<313> 3535: 1076-1095
<400> 3535
TTACGACCCG ATGTATCGCT ATCAGCTTGA GAAAAAAGCG GAAAAAGTTG 50
TCTTTCGCGG TGAGTGGGCG GAAGTGGCGG AATGGGTTAA GGCGCGGTAA 100
<210> 3536
<211> 100
<212> DNA
<213> Escherichia coli K-12 MG1655
<220> ybbb b0504
<222> 915-1014
<223> ybbb b0504 U00096 complement(529356..530450)
<313> 3536: 952-971
<400> 3536
GGATGCAGCA CTGACAACGC AACTCACCAC CGGCAGCACC GACGGTCATC 50
TGGCCTGGCT GGTGCCGTTA CTTGAAGAAT ATTACGACCC GATGTATCGC 100
<210> 3537
<211> 100
<212> DNA
<213> Escherichia coli K-12 MG1655
<220> ybbb b0504
<222> 821-920
<223> ybbb b0504 U00096 complement(529356..530450)
<313> 3537: 850-869

<400> 3537
GCTGGCAGGA GTATTGCGAA TACCTGCATC ACGGACTTTC GGCGATTAAG 50
CGTCGGCTGG GGCTACAGCG CTATAACGAA CTGGCTGCAA GGCTGGATGC 100
<210> 3538
<211> 100
<212> DNA
<213> Escherichia coli K-12 MG1655
<220> flgf b1078
<222> 657-756
<223> flgf b1078 U00096 1133025..1133780
<313> 3538: 732-751
<400> 3538
CATGATTGCC AGCGCGCGGC GTTTTGAAAT GCAGATGAAG GTGATCAGCA 50
GCGTCGATGA TAACGCAGGC CGTGCCAACC AACTGCTGTC GATGAGTTAA 100
<210> 3539
<211> 100
<212> DNA
<213> Escherichia coli K-12 MG1655
<220> flgf b1078
<222> 483-582
<223> flgf b1078 U00096 1133025..1133780
<313> 3539: 520-539
<400> 3539
TAAACTGGTG AAAGCCACGG GCAGCGAAGT GCAGCGCGGT GACGACGGCA 50
TTTTTCGTTT AAGCGCAGAA ACCCAGGCCA CGCGTGGGCC GGTACTGCAG 100
<210> 3540
<211> 100
<212> DNA
<213> Escherichia coli K-12 MG1655
<220> flgf b1078
<222> 426-525
<223> flgf b1078 U00096 1133025..1133780
<313> 3540: 440-459
<400> 3540
AATCTCGGCG CTCAATCCGG GCGATCCGGC AAATACGGTT GCGCCAGTAG 50
GGCGTCTTAA ACTGGTGAAA GCCACGGGCA GCGAAGTGCA GCGCGGTGAC 100
<210> 3541
<211> 100
<212> DNA
<213> Escherichia coli K-12 MG1655
<220> ybbc ybbC
<222> 233-332
<223> ybbc ybbC U00096 526805..527173
<313> 3541: 289-308
<400> 3541
CATATTTAAT TACGGAACTA CCAGATAGTT GGGTTGTTGA GGGAGCAAAG 50
TTACCTTATG AAGTTGCGGG TGGTGTATTT ATTATAGAAA TTAATAAGAA 100
<210> 3542
<211> 100
<212> DNA
<213> Escherichia coli K-12 MG1655
<220> ybbc ybbC
<222> 237-336
<223> ybbc ybbC U00096 526805..527173
<313> 3542: 280-299
<400> 3542
TTTAATTACG GAACTACCAG ATAGTTGGGT TGTTGAGGGA GCAAAGTTAC 50
CTTATGAAGT TGCGGGTGGT GTATTTATTA TAGAAATTAA TAAGAAAAAT 100
<210> 3543
<211> 100
<212> DNA
<213> Escherichia coli K-12 MG1655
<220> ybbc ybbC
<222> 221-320
<223> ybbc ybbC U00096 526805..527173
<313> 3543: 272-291
<400> 3543
AAGAAGAAAA ACCATATTTA ATTACGGAAC TACCAGATAG TTGGGTTGTT 50
GAGGGAGCAA AGTTACCTTA TGAAGTTGCG GGTGGTGTAT TTATTATAGA 100
<210> 3544
<211> 100

<212> DNA
<213> Escherichia coli K-12 MG1655
<220> flgg b1079
<222> 684-783
<223> flgg b1079 U00096 1133952..1134734
<313> 3544: 752-771
<400> 3544
AGAACTGGTC AATATGATTC AGGTGCAACG CGCTTACGAA ATCAACAGTA 50
AAGCGGTGTC CACCACCGAT CAGATGCTGC AAAAACTGAC GCAACTCTAA 100
<210> 3545
<211> 100
<212> DNA
<213> Escherichia coli K-12 MG1655
<220> flgg b1079
<222> 674-773
<223> flgg b1079 U00096 1133952..1134734
<313> 3545: 704-723
<400> 3545
ACGTGGCGGA AGAACTGGTC AATATGATTC AGGTGCAACG CGCTTACGAA 50
ATCAACAGTA AAGCGGTGTC CACCACCGAT CAGATGCTGC AAAAACTGAC 100
<210> 3546
<211> 100
<212> DNA
<213> Escherichia coli K-12 MG1655
<220> flgg b1079
<222> 629-728
<223> flgg b1079 U00096 1133952..1134734
<313> 3546: 682-701
<400> 3546
GCGCGGGACT GCTGTATCAA GGGTATGTTG AAACGTCTAA CGTCAACGTG 50
GCGGAAGAAC TGGTCAATAT GATTCAGGTG CAACGCGCTT ACGAAATCAA 100
<210> 3547
<211> 100
<212> DNA
<213> Escherichia coli K-12 MG1655
<220> ybbd ybbD
<222> 129-228
<223> ybbd ybbD U00096 527864..528124
<313> 3547: 182-201
<400> 3547
CATAAACTAT ATGGCAATAC CAGAAAATGA TGGAGTTTTT ACATGGCTCC 50
CAGATTTTTT TCCGCACGTA GCGGTGGATA TATCAATATA CACAAATGTA 100
<210> 3548
<211> 100
<212> DNA
<213> Escherichia coli K-12 MG1655
<220> ybbd ybbD
<222> 141-240
<223> ybbd ybbD U00096 527864..528124
<313> 3548: 171-190
<400> 3548
GGCAATACCA GAAAATGATG GAGTTTTTAC ATGGCTCCCA GATTTTTTTC 50
CGCACGTAGC GGTGGATATA TCAATATACA CAAATGTAGA AGATGATTAT 100
<210> 3549
<211> 100
<212> DNA
<213> Escherichia coli K-12 MG1655
<220> ybbd ybbD
<222> 104-203
<223> ybbd ybbD U00096 527864..528124
<313> 3549: 159-178
<400> 3549
ATGAATCTCT TGTAAGTAAA CATTACATAA ACTATATGGC AATACCAGAA 50
AATGATGGAG TTTTTACATG GCTCCCAGAT TTTTTTCCGC ACGTAGCGGT 100
<210> 3550
<211> 100
<212> DNA
<213> Escherichia coli K-12 MG1655
<220> flgh b1080
<222> 593-692
<223> flgh b1080 U00096 1134787..1135485

<313> 3550: 629-648
<400> 3550
CTCAGGTGGC GGATGCGCGC ATTGAATACG TAGGCAATGG CTACATTAAC 50
GAAGCGCAAA ATATGGGCTG GTTGCAGCGT TTCTTCCTTA ACCTGTCGCC 100
<210> 3551
<211> 100
<212> DNA
<213> Escherichia coli K-12 MG1655
<220> flgh b1080
<222> 558-657
<223> flgh b1080 U00096 1134787..1135485
<313> 3551: 585-604
<400> 3551
ACGCACTATC AGCGGCAGCA ATACCGTACC GTCTACTCAG GTGGCGGATG 50
CGCGCATTGA ATACGTAGGC AATGGCTACA TTAACGAAGC GCAAAATATG 100
<210> 3552
<211> 100
<212> DNA
<213> Escherichia coli K-12 MG1655
<220> flgh b1080
<222> 471-570
<223> flgh b1080 U00096 1134787..1135485
<313> 3552: 496-515
<400> 3552
CAACGGCAAC CTGCATGTGG TGGGTGAAAA ACAGATTGCC ATTAATCAGG 50
GTACCGAATT TATTCGCTTC TCTGGCGTGG TTAATCCACG CACTATCAGC 100
<210> 3553
<211> 100
<212> DNA
<213> Escherichia coli K-12 MG1655
<220> flgi b1081
<222> 999-1098
<223> flgi b1081 U00096 1135497..1136594
<313> 3553: 1042-1061
<400> 3553
CGCGCTCAAT GCGCTGGGCG CTACGCCGAT GGATCTGATG TCCATACTGC 50
AATCAATGCA AAGTGCGGGA TGTCTGCGGG CAAAACTGGA AATCATCTGA 100
<210> 3554
<211> 100
<212> DNA
<213> Escherichia coli K-12 MG1655
<220> flgi b1081
<222> 828-927
<223> flgi b1081 U00096 1135497..1136594
<313> 3554: 875-894
<400> 3554
GCAGGGGAAT CTCTCAGTAA CAGTTAATCG TCAGGCCAAT GTCAGCCAGC 50
CAGATACACC GTTTGGTGGT GGACAGACTG TGGTTACTCC ACAAACGCAG 100
<210> 3555
<211> 100
<212> DNA
<213> Escherichia coli K-12 MG1655
<220> flgi b1081
<222> 803-902
<223> flgi b1081 U00096 1135497..1136594
<313> 3555: 841-860
<400> 3555
TGACCCTCGA CAGCTGCGCG GTAGCGCAGG GGAATCTCTC AGTAACAGTT 50
AATCGTCAGG CCAATGTCAG CCAGCCAGAT ACACCGTTTG GTGGTGGACA 100
<210> 3556
<211> 100
<212> DNA
<213> Escherichia coli K-12 MG1655
<220> ybbf b0525
<222> 624-723
<223> ybbf b0525 U00096 complement(552441..553163)
<313> 3556: 701-720
<400> 3556
GCAACCTGCT TTTCGCGTGG TACTGGGTGC CTGGCATACG GAAGGTTCAA 50
TGGTGAAAGT CACGGCGGAT GACGTTGAGC TGATTCATTT TCCGTTTTAA 100
<210> 3557

<211> 100
<212> DNA
<213> Escherichia coli K-12 MG1655
<220> ybbf b0525
<222> 576-675
<223> ybbf b0525 U00096 complement(552441..553163)
<313> 3557: 622-641
<400> 3557
CCACGGGCAT ACCCATCGCC CGGCGGTGCA TGAACTTATC GCCAATCAGC 50
AACCTGCTTT TCGCGTGGTA CTGGGTGCCT GGCATACGGA AGGTTCAATG 100
<210> 3558
<211> 100
<212> DNA
<213> Escherichia coli K-12 MG1655
<220> ybbf b0525
<222> 522-621
<223> ybbf b0525 U00096 complement(552441..553163)
<313> 3558: 562-581
<400> 3558
TAACCAAAAC GCGGTGGTCA GTGCGATGGA AAAACATCAG GTGCAATGGC 50
TGATCCACGG GCATACCCAT CGCCCGGCGG TGCATGAACT TATCGCCAAT 100
<210> 3559
<211> 100
<212> DNA
<213> Escherichia coli K-12 MG1655
<220> flgj b1082
<222> 826-925
<223> flgj b1082 U00096 1136594..1137535
<313> 3559: 878-897
<400> 3559
GGCTATGCCA CCGATCCTCA CTATGCCCGC AAACTCACCA ACATGATTCA 50
GCAGATGAAA TCGATAAGCG ACAAGGTGAG CAAAACCTAC AGTATGAACA 100
<210> 3560
<211> 100
<212> DNA
<213> Escherichia coli K-12 MG1655
<220> flgj b1082
<222> 821-920
<223> flgj b1082 U00096 1136594..1137535
<313> 3560: 850-869
<400> 3560
ACGCGGGCTA TGCCACCGAT CCTCACTATG CCCGCAAACT CACCAACATG 50
ATTCAGCAGA TGAAATCGAT AAGCGACAAG GTGAGCAAAA CCTACAGTAT 100
<210> 3561
<211> 100
<212> DNA
<213> Escherichia coli K-12 MG1655
<220> flgj b1082
<222> 774-873
<223> flgj b1082 U00096 1136594..1137535
<313> 3561: 795-814
<400> 3561
CGTGACGACC GCCGCGAGTG CGGAACAGGG GGCGCAGGCC CTACAGGACG 50
CGGGCTATGC CACCGATCCT CACTATGCCC GCAAACTCAC CAACATGATT 100
<210> 3562
<211> 100
<212> DNA
<213> Escherichia coli K-12 MG1655
<220> flgk b1083
<222> 1545-1644
<223> flgk b1083 U00096 1137601..1139244
<313> 3562: 1618-1637
<400> 3562
GTACGGAAAT CTGCAACGTT TTCAGCAGTA TTACCTGGCG AATGCGCAGG 50
TTCTGCAGAC GGCAAACGCG ATTTTTGATG CGCTGATTAA CATTCGCTAA 100
<210> 3563
<211> 100
<212> DNA
<213> Escherichia coli K-12 MG1655
<220> flgk b1083
<222> 1527-1626

<223> flgk b1083 U00096 1137601..1139244
<313> 3563: 1551-1570
<400> 3563
TGTCAATCTC GATGAGGAGT ACGGAAATCT GCAACGTTTT CAGCAGTATT 50
ACCTGGCGAA TGCGCAGGTT CTGCAGACGG CAAACGCGAT TTTTGATGCG 100
<210> 3564
<211> 100
<212> DNA
<213> Escherichia coli K-12 MG1655
<220> flgk b1083
<222> 1462-1561
<223> flgk b1083 U00096 1137601..1139244
<313> 3564: 1491-1510
<400> 3564
ACCAGTAGCG CCACGCAAGG TAATGTGGTG ACGCAGCTTT CCAATCAGCA 50
GCAGTCGATT TCCGGTGTCA ATCTCGATGA GGAGTACGGA AATCTGCAAC 100
<210> 3565
<211> 100
<212> DNA
<213> Escherichia coli K-12 MG1655
<220> flgl b1084
<222> 855-954
<223> flgl b1084 U00096 1139256..1140209
<313> 3565: 933-952
<400> 3565
AACTATTTCA TCTTACATCA TGCAGCAAAC GGCATTGCAG GCATCGTATA 50
AAGCATTTAC CGATATGCAG GGATTGTCGC TCTTCCAGCT CAGCAAATAA 100
<210> 3566
<211> 100
<212> DNA
<213> Escherichia coli K-12 MG1655
<220> flgl b1084
<222> 834-933
<223> flgl b1084 U00096 1139256..1140209
<313> 3566: 894-913
<400> 3566
GGTTGATGTG GACTGGAATG CAACTATTTC ATCTTACATC ATGCAGCAAA 50
CGGCATTGCA GGCATCGTAT AAAGCATTTA CCGATATGCA GGGATTGTCG 100
<210> 3567
<211> 100
<212> DNA
<213> Escherichia coli K-12 MG1655
<220> flgl b1084
<222> 792-891
<223> flgl b1084 U00096 1139256..1140209
<313> 3567: 843-862
<400> 3567
TAGCGATCGC GCTTTAGGGC AAACGCAGCA GATGAGCGAT CTGGTTGATG 50
TGGACTGGAA TGCAACTATT TCATCTTACA TCATGCAGCA AACGGCATTG 100
<210> 3568
<211> 100
<212> DNA
<213> Escherichia coli K-12 MG1655
<220> ybbi b0488
<222> 309-408
<223> ybbi b0488 U00096 513217..513624
<313> 3568: 373-392
<400> 3568
CCAGCTGCTG GCACTGGCGA ATGCCTGCCC TGGCGATGAC AGCGCCGACT 50
GCCCGATTAT CGAAAATCTC TCCGGCTGCT GTCATCATCG GGCAGGGTGA 100
<210> 3569
<211> 100
<212> DNA
<213> Escherichia coli K-12 MG1655
<220> ybbi b0488
<222> 296-395
<223> ybbi b0488 U00096 513217..513624
<313> 3569: 345-364
<400> 3569
AATCCATGCG CGACCAGCTG CTGGCACTGG CGAATGCCTG CCCTGGCGAT 50
GACAGCGCCG ACTGCCCGAT TATCGAAAAT CTCTCCGGCT GCTGTCATCA 100

<210> 3570
<211> 100
<212> DNA
<213> Escherichia coli K-12 MG1655
<220> ybbi b0488
<222> 238-337
<223> ybbi b0488 U00096 513217..513624
<313> 3570: 285-304
<400> 3570
GTCAAACGGC GCACGCTGGA GAAGGTGGCG GAGATCGAAC GACACATTGA 50
GGAGCTGCAA TCCATGCGCG ACCAGCTGCT GGCACTGGCG AATGCCTGCC 100
<210> 3571
<211> 100
<212> DNA
<213> Escherichia coli K-12 MG1655
<220> flgm b1072
<222> 195-294
<223> flgm b1072 U00096 complement(1129058..1129351)
<313> 3571: 269-288
<400> 3571
GTTAAAACTG GCGATTCGTA ACGGTGAACT AAAAATGGAC ACCGGCAAAA 50
TTGCCGATGC GCTGATCAAC GAAGCGCAGC AAGACTTGCA GAGTAACTGA 100
<210> 3572
<211> 100
<212> DNA
<213> Escherichia coli K-12 MG1655
<220> flgm b1072
<222> 193-292
<223> flgm b1072 U00096 complement(1129058..1129351)
<313> 3572: 214-233
<400> 3572
GCGTTAAAAC TGGCGATTCG TAACGGTGAA CTAAAAATGG ACACCGGCAA 50
AATTGCCGAT GCGCTGATCA ACGAAGCGCA GCAAGACTTG CAGAGTAACT 100
<210> 3573
<211> 100
<212> DNA
<213> Escherichia coli K-12 MG1655
<220> flgm b1072
<222> 100-199
<223> flgm b1072 U00096 complement(1129058..1129351)
<313> 3573: 140-159
<400> 3573
ACCGCCTCCA CCAGCACCAG TGTGACGTTA AGCGACGCGC AAGCAAAACT 50
GATGCAACCC GGCAGCAGTG ATATCAATCT TGAACGTGTC GAAGCGTTAA 100
<210> 3574
<211> 100
<212> DNA
<213> Escherichia coli K-12 MG1655
<220> ybbj b0489
<222> 357-456
<223> ybbj b0489 U00096 complement(513625..514080)
<313> 3574: 427-446
<400> 3574
ATGGCCTGTC AGCGCCAGCG AGGATCTCGG TGCAGGTACG CATGTTGAAG 50
TCATTGCGAT AGAAGGGATA ACCCTACATA TCCGAGCCGT CTCGTCTTAA 100
<210> 3575
<211> 100
<212> DNA
<213> Escherichia coli K-12 MG1655
<220> ybbj b0489
<222> 339-438
<223> ybbj b0489 U00096 complement(513625..514080)
<313> 3575: 379-398
<400> 3575
GCGCGTCGGT GACAGTTCAT GGCCTGTCAG CGCCAGCGAG GATCTCGGTG 50
CAGGTACGCA TGTTGAAGTC ATTGCGATAG AAGGGATAAC CCTACATATC 100
<210> 3576
<211> 100
<212> DNA
<213> Escherichia coli K-12 MG1655
<220> ybbj b0489

<222> 253-352
<223> ybbj b0489 U00096 complement(513625..514080)
<313> 3576: 304-323
<400> 3576
GATAGTCATT TAAACCAGCG CGGGCAGCAG CTGATTGGCC GACGTTTTGT 50
GCTGGAATCT CCGCTGGTCA ACGGGCGCGG TCATATGCGC GTCGGTGACA 100
<210> 3577
<211> 100
<212> DNA
<213> Escherichia coli K-12 MG1655
<220> flgn b1071
<222> 318-417
<223> flgn b1071 U00096 complement(1128637..1129053)
<313> 3577: 397-416
<400> 3577
GGCGCTGGAA ATGTTGAAAC CGCATCAGGA ACCGACGCTA TATGGGGCGA 50
ACGGTCAGAC CTCAACAACC CATCGCGGCG GTAAAAAGAT TTCGATCTGA 100
<210> 3578
<211> 100
<212> DNA
<213> Escherichia coli K-12 MG1655
<220> flgn b1071
<222> 282-381
<223> flgn b1071 U00096 complement(1128637..1129053)
<313> 3578: 337-356
<400> 3578
CTGGTTACTG GAAGGACAGA TTGAGCGCAA TCAACAGGCG CTGGAAATGT 50
TGAAACCGCA TCAGGAACCG ACGCTATATG GGGCGAACGG TCAGACCTCA 100
<210> 3579
<211> 100
<212> DNA
<213> Escherichia coli K-12 MG1655
<220> flgn b1071
<222> 263-362
<223> flgn b1071 U00096 complement(1128637..1129053)
<313> 3579: 294-313
<400> 3579
AAATGAATCA ACATAACGGC TGGTTACTGG AAGGACAGAT TGAGCGCAAT 50
CAACAGGCGC TGGAAATGTT GAAACCGCAT CAGGAACCGA CGCTATATGG 100
<210> 3580
<211> 100
<212> DNA
<213> Escherichia coli K-12 MG1655
<220> ybbk b0490
<222> 819-918
<223> ybbk b0490 U00096 complement(514080..514997)
<313> 3580: 894-913
<400> 3580
AGTAGTGATG ATGCCATTAG AGGCCAGCAG CCTGATGGGG TCGATTGCCG 50
GGATTGCCGA GCTGGTGAAA GACAGCGCCA ACAAACGGAC TCAGCCATGA 100
<210> 3581
<211> 100
<212> DNA
<213> Escherichia coli K-12 MG1655
<220> ybbk b0490
<222> 800-899
<223> ybbk b0490 U00096 complement(514080..514997)
<313> 3581: 858-877
<400> 3581
GTTCCTCCAG TAACAGCAAA GTAGTGATGA TGCCATTAGA GGCCAGCAGC 50
CTGATGGGGT CGATTGCCGG GATTGCCGAG CTGGTGAAAG ACAGCGCCAA 100
<210> 3582
<211> 100
<212> DNA
<213> Escherichia coli K-12 MG1655
<220> ybbk b0490
<222> 763-862
<223> ybbk b0490 U00096 complement(514080..514997)
<313> 3582: 801-820
<400> 3582
GTAGCGCAGA AATACACCGA AGCGTTACAG CAGATCGGTT CCTCCAGTAA 50

CAGCAAAGTA GTGATGATGC CATTAGAGGC CAGCAGCCTG ATGGGGTCGA 100
<210> 3583
<211> 100
<212> DNA
<213> Escherichia coli K-12 MG1655
<220> b1500 b1500
<222> 36-135
<223> b1500 b1500 U00096 complement(1581786..1581983)
<313> 3583: 103-122
<400> 3583
AAGAGACAAC TATAAAGAAA TCATGTCTGC AATTGTGGTT GTCTTATTAC 50
TGACACTTAC GTTGATAGCC ATTTTTTCGG CAATTGATCA GCTGAGTATT 100
<210> 3584
<211> 100
<212> DNA
<213> Escherichia coli K-12 MG1655
<220> b1500 b1500
<222> 16-115
<223> b1500 b1500 U00096 complement(1581786..1581983)
<313> 3584: 42-61
<400> 3584
GTGAAAAACA AAATCACGCA AAGAGACAAC TATAAAGAAA TCATGTCTGC 50
AATTGTGGTT GTCTTATTAC TGACACTTAC GTTGATAGCC ATTTTTTCGG 100
<210> 3585
<211> 100
<212> DNA
<213> Escherichia coli K-12 MG1655
<220> b1500 b1500
<222> 1-100
<223> b1500 b1500 U00096 complement(1581786..1581983)
<313> 3585: 20-39
<400> 3585
ATGCATGCGA CCACAGTGAA AAACAAAATC ACGCAAAGAG ACAACTATAA 50
AGAAATCATG TCTGCAATTG TGGTTGTCTT ATTACTGACA CTTACGTTGA 100
<210> 3586
<211> 100
<212> DNA
<213> Escherichia coli K-12 MG1655
<220> ybbl b0491
<222> 561-660
<223> ybbl b0491 U00096 515143..515820
<313> 3586: 592-611
<400> 3586
AAATATTGCC GTGCTGTGGG TGACACACGA TAAAGACGAA ATTAATCATG 50
CGGATAAAGT GATTACACTG CAACCGCATG CCGGAGAAAT GCAGGAAGCA 100
<210> 3587
<211> 100
<212> DNA
<213> Escherichia coli K-12 MG1655
<220> ybbl b0491
<222> 385-484
<223> ybbl b0491 U00096 515143..515820
<313> 3587: 434-453
<400> 3587
AGCATTTTGA CGAAGAATAT CGCCGAGCTA TCTGGTGGTG AAAAACAACG 50
CATCTCATTG ATTCGTAACC TGCAATTTAT GCCGAAGGTT TTATTGCTGG 100
<210> 3588
<211> 100
<212> DNA
<213> Escherichia coli K-12 MG1655
<220> ybbl b0491
<222> 361-460
<223> ybbl b0491 U00096 515143..515820
<313> 3588: 386-405
<400> 3588
CTCGAACGCT TCGCCTTGCC GGACAGCATT TTGACGAAGA ATATCGCCGA 50
GCTATCTGGT GGTGAAAAAC AACGCATCTC ATTGATTCGT AACCTGCAAT 100
<210> 3589
<211> 100
<212> DNA
<213> Escherichia coli K-12 MG1655

<220> b1501 b1501
<222> 2088-2187
<223> b1501 b1501 U00096 complement(1582231..1584510)
<313> 3589: 2117-2136
<400> 3589
GCTTACGCCA GACGGTAAGC GCAGCTCACG CCGCATGGAT AGATTAAAAG 50
TGGTCATTTA CCCTATGGCT GACCGCTCAC TGGTGACCTA TTTTCCAGAA 100
<210> 3590
<211> 100
<212> DNA
<213> Escherichia coli K-12 MG1655
<220> b1501 b1501
<222> 2021-2120
<223> b1501 b1501 U00096 complement(1582231..1584510)
<313> 3590: 2063-2082
<400> 3590
TCTTTATGAG TGCTAAACAA GCTAAAATTT GCCGTGTAAA AAACGGCGAA 50
AGAGTTAATC TTATTGCGCT TACGCCAGAC GGTAAGCGCA GCTCACGCCG 100
<210> 3591
<211> 100
<212> DNA
<213> Escherichia coli K-12 MG1655
<220> b1501 b1501
<222> 1972-2071
<223> b1501 b1501 U00096 complement(1582231..1584510)
<313> 3591: 2006-2025
<400> 3591
TATGGTATGG ATGATCGCTA TCGAGGGGTA TTCGGTCAAC GAGATGTGGT 50
CTTTATGAGT GCTAAACAAG CTAAAATTTG CCGTGTAAAA AACGGCGAAA 100
<210> 3592
<211> 100
<212> DNA
<213> Escherichia coli K-12 MG1655
<220> ybbm b0492
<222> 656-755
<223> ybbm b0492 U00096 515780..516586
<313> 3592: 698-717
<400> 3592
ATCCGGTGAA GGCGATTAAA TATCAGATTA TGGTGACCTT TATGCTGCTC 50
TCAACCGCCA GCTTGTCGAC CATTATTGCC TGCTATTTAA CCTATCGTAA 100
<210> 3593
<211> 100
<212> DNA
<213> Escherichia coli K-12 MG1655
<220> ybbm b0492
<222> 550-649
<223> ybbm b0492 U00096 515780..516586
<313> 3593: 594-613
<400> 3593
CGCGACAGTA TTCGCGCGGC TTTAATTCCG ACGGTCGATT CAGCAAAAAC 50
GGTTGGCTTA GTGAGTTTAC CAGGAATGAT GTCCGGGCTG ATATTTGCCG 100
<210> 3594
<211> 100
<212> DNA
<213> Escherichia coli K-12 MG1655
<220> ybbm b0492
<222> 464-563
<223> ybbm b0492 U00096 515780..516586
<313> 3594: 516-535
<400> 3594
GGCAACGGGT CATTAGCGAA CAGCAACAGA TTCAGGAGAA ACTGAGTCTT 50
GGTGCGACGC CGAAGCAGGC TTCAGCGATA TTGATTCGCG ACAGTATTCG 100
<210> 3595
<211> 100
<212> DNA
<213> Escherichia coli K-12 MG1655
<220> b1502 b1502
<222> 816-915
<223> b1502 b1502 U00096 complement(1584844..1585758)
<313> 3595: 887-906
<400> 3595

AGTACCTCTG GGTTTAAGCG CAACCTATGG ACAAACAGGA AACAAGGTTT 50
CAGCCGGAAC CGTCCAGTCA GTCATTGGTG TCACTTTTAT CTATGAATAA 100
<210> 3596
<211> 100
<212> DNA
<213> Escherichia coli K-12 MG1655
<220> b1502 b1502
<222> 645-744
<223> b1502 b1502 U00096 complement(1584844..1585758)
<313> 3596: 665-684
<400> 3596
AAAGCTCTCA TTTTATCTTT CGGGTGCCAC CACTGACAGT AGCCGACAAG 50
TGTTTGCTAA CACAGCACCT GATGCAACAA AAGCATCGGG AGTGGGTGTC 100
<210> 3597
<211> 100
<212> DNA
<213> Escherichia coli K-12 MG1655
<220> b1502 b1502
<222> 597-696
<223> b1502 b1502 U00096 complement(1584844..1585758)
<313> 3597: 624-643
<400> 3597
TCCTGGAAGC GCAGAAATAC CCCTTGGCGT ATATTGCTCA AGCGAACAAA 50
AGCTCTCATT TTATCTTTCG GGTGCCACCA CTGACAGTAG CCGACAAGTG 100
<210> 3598
<211> 100
<212> DNA
<213> Escherichia coli K-12 MG1655
<220> ybbn b0493
<222> 792-891
<223> ybbn b0493 U00096 complement(516649..517539)
<313> 3598: 830-849
<400> 3598
TCAGACGCGT AAAACGTTCC AGGAGATCCT CGCTGCGCTG GGTACGGGTG 50
ATGCACTGGC GTCGAAGTAT CGCCGCCAGC TGTATGCATT GTTGTATTGA 100
<210> 3599
<211> 100
<212> DNA
<213> Escherichia coli K-12 MG1655
<220> ybbn b0493
<222> 682-781
<223> ybbn b0493 U00096 complement(516649..517539)
<313> 3599: 726-745
<400> 3599
GCCGCACTGG CGACGCAACT GGCGCTGCAA CTGCATCAGG TTGGGCGCAA 50
TGAAGAGGCG CTGGAGTTGC TGTTCGGGCA TCTGCGTAAA GATCTCACCG 100
<210> 3600
<211> 100
<212> DNA
<213> Escherichia coli K-12 MG1655
<220> ybbn b0493
<222> 618-717
<223> ybbn b0493 U00096 complement(516649..517539)
<313> 3600: 664-683
<400> 3600
GCAGGCGGCT GATACGCCGG AAATTCAACA GTTGCAACAG CAGGTGGCGG 50
AGAATCCAGA AGATGCCGCA CTGGCGACGC AACTGGCGCT GCAACTGCAT 100
<210> 3601
<211> 100
<212> DNA
<213> Escherichia coli K-12 MG1655
<220> b1503 b1503
<222> 403-502
<223> b1503 b1503 U00096 complement(1585817..1586320)
<313> 3601: 432-451
<400> 3601
AATGCACAGT TTCCACTTAA GGCAAGAGCT ATCACGGTGA ATGGAAACGC 50
AAGCCAGGGA ACGATCGAGG CGCTAATCAA TGTGATCTAC ACCTGGCAAT 100
<210> 3602
<211> 100
<212> DNA

<213> Escherichia coli K-12 MG1655
<220> b1503 b1503
<222> 326-425
<223> b1503 b1503 U00096 complement(1585817..1586320)
<313> 3602: 348-367
<400> 3602
TAGAGCTGAG GGATGACCAG GATGCTGCGT TAAAAAATGG CGATAGCAAA 50
ACGGTTATTG TTGATGAGAT CACTCGTAAT GCACAGTTTC CACTTAAGGC 100
<210> 3603
<211> 100
<212> DNA
<213> Escherichia coli K-12 MG1655
<220> b1503 b1503
<222> 226-325
<223> b1503 b1503 U00096 complement(1585817..1586320)
<313> 3603: 266-285
<400> 3603
GATTGTCCGG TTGAAACAAG TGCAGTGACG GCAATCGTGA CAGGTTCAAC 50
TGACAATACG GGTTATTACA AAAATGAAGG TACTGCCGAA AATATTCAGA 100
<210> 3604
<211> 100
<212> DNA
<213> Escherichia coli K-12 MG1655
<220> ybbo b0494
<222> 711-810
<223> ybbo b0494 U00096 complement(517564..518373)
<313> 3604: 786-805
<400> 3604
GCCGAAGATG CGCTATCCGG TGACGCTGGT GACCTGGGCG GTAATGGTGC 50
TTAAGCGCCT GCTGCCGGGG CGCGTGATGG ACAAAATATT GCAGGGGTGA 100
<210> 3605
<211> 100
<212> DNA
<213> Escherichia coli K-12 MG1655
<220> ybbo b0494
<222> 662-761
<223> ybbo b0494 U00096 complement(517564..518373)
<313> 3605: 713-732
<400> 3605
GACCGGAAGC GGTGGTGGAC AAAGTACGCC ATGCTTTTAT TAGCGAGAAG 50
CCGAAGATGC GCTATCCGGT GACGCTGGTG ACCTGGGCGG TAATGGTGCT 100
<210> 3606
<211> 100
<212> DNA
<213> Escherichia coli K-12 MG1655
<220> ybbo b0494
<222> 634-733
<223> ybbo b0494 U00096 complement(517564..518373)
<313> 3606: 672-691
<400> 3606
CCCGGCATCG CCGCCCGCTT TACGTTGGGA CCGGAAGCGG TGGTGGACAA 50
AGTACGCCAT GCTTTTATTA GCGAGAAGCC GAAGATGCGC TATCCGGTGA 100
<210> 3607
<211> 100
<212> DNA
<213> Escherichia coli K-12 MG1655
<220> b1504 b1504
<222> 432-531
<223> b1504 b1504 U00096 complement(1586333..1586863)
<313> 3607: 487-506
<400> 3607
CAATATTTTG CCTTACTCCG CTCGTCTGAA GTCAACTCAG AAGTCCGTCA 50
ATCCGGGACT GGTGAGGGCT TCGGCAACCT TTACCCTTGA ATTTCAATAA 100
<210> 3608
<211> 100
<212> DNA
<213> Escherichia coli K-12 MG1655
<220> b1504 b1504
<222> 396-495
<223> b1504 b1504 U00096 complement(1586333..1586863)
<313> 3608: 430-449

<400> 3608
CGGGATGCAG TGGATCCCAC TGGTACCAGA ACAGAACAAT ATTTTGCCTT 50
ACTCCGCTCG TCTGAAGTCA ACTCAGAAGT CCGTCAATCC GGGACTGGTG 100
<210> 3609
<211> 100
<212> DNA
<213> Escherichia coli K-12 MG1655
<220> b1504 b1504
<222> 323-422
<223> b1504 b1504 U00096 complement(1586333..1586863)
<313> 3609: 388-407
<400> 3609
ATACGGCCTC CGGTTTGGGT ATAGAAATAT TGGACGCAAA TATGCGTCCG 50
GTGAAACTGA ATGATCTTCA TGCCGGGATG CAGTGGATCC CACTGGTACC 100
<210> 3610
<211> 100
<212> DNA
<213> Escherichia coli K-12 MG1655
<220> ybbp b0497
<222> 2316-2415
<223> ybbp b0497 U00096 519640..522054
<313> 3610: 2383-2402
<400> 3610
TCTGCCGTGC AGCGGAGCGC TGCTGCTGTC GCTTTTCGGC GGCTGGCTGG 50
GTGCGCGACT GGTTAAGGGT AAGGCGCTGT TCAGGCAGTT TGCGGGGTGA 100
<210> 3611
<211> 100
<212> DNA
<213> Escherichia coli K-12 MG1655
<220> ybbp b0497
<222> 2268-2367
<223> ybbp b0497 U00096 519640..522054
<313> 3611: 2292-2311
<400> 3611
GAAAGTGTTT GATTTTCCGT GGGAGCCAGA CTGGCGATTG TGGATTGTTC 50
TGCCGTGCAG CGGAGCGCTG CTGCTGTCGC TTTTCGGCGG CTGGCTGGGT 100
<210> 3612
<211> 100
<212> DNA
<213> Escherichia coli K-12 MG1655
<220> ybbp b0497
<222> 2099-2198
<223> ybbp b0497 U00096 519640..522054
<313> 3612: 2137-2156
<400> 3612
TGCAGGTGGG AATGCGTCAG CGTCATCAGG AGCTGGTGGT GTGGCGCACA 50
CTCGGTGCGG GGAAAAAACT GCTGCGTACC ACGTTGTGGT GTGAGTTCGC 100
<210> 3613
<211> 100
<212> DNA
<213> Escherichia coli K-12 MG1655
<220> b1505 b1505
<222> 963-1062
<223> b1505 b1505 U00096 complement(1586877..1588025)
<313> 3613: 975-994
<400> 3613
CGGAGAGAAT AAAAATGGCA GCATTGTCGC GGAAAATGGT CAGGTTTATC 50
TGACTGGACT TCCACAGTCA GGGCAATTAC AGGTTTCATG GGGCAAAGAT 100
<210> 3614
<211> 100
<212> DNA
<213> Escherichia coli K-12 MG1655
<220> b1505 b1505
<222> 864-963
<223> b1505 b1505 U00096 complement(1586877..1588025)
<313> 3614: 892-911
<400> 3614
TATTGCCAGA GCAACATTTA ATGCACAAAT CGGCGGGAAA GTATTAATGA 50
CGTTGAAGTA CGGTAATAAG AGCGTTCCAT TCGGTGCAAT TGTCACACAC 100
<210> 3615
<211> 100

<212> DNA
<213> Escherichia coli K-12 MG1655
<220> b1505 b1505
<222> 809-908
<223> b1505 b1505 U00096 complement(1586877..1588025)
<313> 3615: 848-867
<400> 3615
CAGATAATGT TGAACTGGAT GAAACCGTGG TCACTGTCAT CCCAACTCAC 50
GGTGCTATTG CCAGAGCAAC ATTTAATGCA CAAATCGGCG GGAAAGTATT 100
<210> 3616
<211> 100
<212> DNA
<213> Escherichia coli K-12 MG1655
<220> ybbq b0510
<222> 780-879
<223> ybbq b0510 U00096 535810..536688
<313> 3616: 859-878
<400> 3616
GGAGTTATTT AATACCTGTG CGGCAAACGG TGGCAGCCAG TTGGATCACT 50
CTGCGTTAGT GCAGGCGCTG GAATTAATGG CTAACCATAA ACTGGCCTGA 100
<210> 3617
<211> 100
<212> DNA
<213> Escherichia coli K-12 MG1655
<220> ybbq b0510
<222> 743-842
<223> ybbq b0510 U00096 535810..536688
<313> 3617: 775-794
<400> 3617
CACTTGCGCT GAACCTGCCA AACACTGCGA CCTGCCAGGA GTTATTTAAT 50
ACCTGTGCGG CAAACGGTGG CAGCCAGTTG GATCACTCTG CGTTAGTGCA 100
<210> 3618
<211> 100
<212> DNA
<213> Escherichia coli K-12 MG1655
<220> ybbq b0510
<222> 680-779
<223> ybbq b0510 U00096 535810..536688
<313> 3618: 716-735
<400> 3618
ATCCGGGCTT CAAAATCGCT CTGCACCAGA AAGATCTCAA CCTGGCACTG 50
CAAAGTGCGA AAGCACTTGC GCTGAACCTG CCAAACACTG CGACCTGCCA 100
<210> 3619
<211> 100
<212> DNA
<213> Escherichia coli K-12 MG1655
<220> b1506 b1507
<222> 66-165
<223> b1506 b1507 U00096 complement(1588381..1588560)
<313> 3619: 103-122
<400> 3619
ACATTACGAT GTCACCACAG CAGCCTATGC TGTCGGTTAC GAAAGCTATC 50
CCATTTCAGT CGGGAATATT CGCGGATGTT TGGAGAGTCA CCCAAGAGAG 100
<210> 3620
<211> 100
<212> DNA
<213> Escherichia coli K-12 MG1655
<220> b1506 b1507
<222> 32-131
<223> b1506 b1507 U00096 complement(1588381..1588560)
<313> 3620: 56-75
<400> 3620
GTCTCAATGA AGTCAGGCGA CAGATGCTGA ATGAACATTA CGATGTCACC 50
ACAGCAGCCT ATGCTGTCGG TTACGAAAGC TATCCCATTT CAGTCGGGAA 100
<210> 3621
<211> 100
<212> DNA
<213> Escherichia coli K-12 MG1655
<220> b1506 b1507
<222> 1-100
<223> b1506 b1507 U00096 complement(1588381..1588560)

<313> 3621: 18-37
<400> 3621
ATGAGTCCAC TCCGGTATCA GAAATGGCTA CGTCTCAATG AAGTCAGGCG 50
ACAGATGCTG AATGAACATT ACGATGTCAC CACAGCAGCC TATGCTGTCG 100
<210> 3622
<211> 100
<212> DNA
<213> Escherichia coli K-12 MG1655
<220> ybbs b0505
<222> 825-924
<223> ybbs b0505 U00096 complement(530519..531445)
<313> 3622: 869-888
<400> 3622
ATTTGGCAGC GGCAAAGCGG TAGAAGATAT TGTGACCTTG TTTACCCAGC 50
GCAGGCCGGA AATCAGCGGA TTTTTAGAAA TTTTCGGCAA CCCACGCAGT 100
<210> 3623
<211> 100
<212> DNA
<213> Escherichia coli K-12 MG1655
<220> ybbs b0505
<222> 752-851
<223> ybbs b0505 U00096 complement(530519..531445)
<313> 3623: 785-804
<400> 3623
ATAATCAACA ACTGGTCAGC CGGGTAATCC CAACGATGCG CCCTCCTTCG 50
CCATTGAGTC TGGCATGGCG CAAATTTGGC AGCGGCAAAG CGGTAGAAGA 100
<210> 3624
<211> 100
<212> DNA
<213> Escherichia coli K-12 MG1655
<220> ybbs b0505
<222> 627-726
<223> ybbs b0505 U00096 complement(530519..531445)
<313> 3624: 680-699
<400> 3624
CGTCGCCTGG CGATTGCCAG GGCAAAAAGA GATTATTGTT CCCGATATGG 50
AAACGAAAAT CGCCGCCCAT CTGGCGGGCG TTGGCATTGG TTTTTTGCCA 100
<210> 3625
<211> 100
<212> DNA
<213> Escherichia coli K-12 MG1655
<220> ybbt b0506
<222> 384-483
<223> ybbt b0506 U00096 531675..532157
<313> 3625: 442-461
<400> 3625
CGCCTGGCAG CGCGTCACCG ATTTTCTGAC CATCGATCGC GGCGGCAGTG 50
ACAACTGTGA TGTTGAAAGT ATTCCTGAAC AGGAACTCTG TTTTGCGTGA 100
<210> 3626
<211> 100
<212> DNA
<213> Escherichia coli K-12 MG1655
<220> ybbt b0506
<222> 334-433
<223> ybbt b0506 U00096 531675..532157
<313> 3626: 381-400
<400> 3626
GGCGAACAGG GAGTGAATTA CCATCGTAAC GTCTGGCATC ACCCACTTTT 50
CGCCTGGCAG CGCGTCACCG ATTTTCTGAC CATCGATCGC GGCGGCAGTG 100
<210> 3627
<211> 100
<212> DNA
<213> Escherichia coli K-12 MG1655
<220> ybbt b0506
<222> 291-390
<223> ybbt b0506 U00096 531675..532157
<313> 3627: 341-360
<400> 3627
CGACAAACCA GACCTGTCAA CGCTGCGGGC GTTTATCACC AACGGCGAAC 50
AGGGAGTGAA TTACCATCGT AACGTCTGGC ATCACCCACT TTTCGCCTGG 100
<210> 3628

<211> 100
<212> DNA
<213> Escherichia coli K-12 MG1655
<220> b1509 b1510
<222> 1191-1290
<223> b1509 b1510 U00096 complement(1590689..1592089)
<313> 3628: 1226-1245
<400> 3628
GAACTGGATC AACAATAGCA AAGTCTACGC CGTGAAGATG AATGGTCAAA 50
CCGTAGGCCG TGAAGGTGCG CGTAATCTCG GTGAAGTACG TACCGGGGTT 100
<210> 3629
<211> 100
<212> DNA
<213> Escherichia coli K-12 MG1655
<220> b1509 b1510
<222> 1152-1251
<223> b1509 b1510 U00096 complement(1590689..1592089)
<313> 3629: 1186-1205
<400> 3629
CGATGGTAAA CAGCGTGAGT TCCAGCCTTA CATTGAAGCG AACTGGATCA 50
ACAATAGCAA AGTCTACGCC GTGAAGATGA ATGGTCAAAC CGTAGGCCGT 100
<210> 3630
<211> 100
<212> DNA
<213> Escherichia coli K-12 MG1655
<220> b1509 b1510
<222> 1116-1215
<223> b1509 b1510 U00096 complement(1590689..1592089)
<313> 3630: 1150-1169
<400> 3630
GGTGAAAACC TACCTGAACA GCCATCACCA GCGTGACGAT GGTAAACAGC 50
GTGAGTTCCA GCCTTACATT GAAGCGAACT GGATCAACAA TAGCAAAGTC 100
<210> 3631
<211> 100
<212> DNA
<213> Escherichia coli K-12 MG1655
<220> ybbu b0507
<222> 687-786
<223> ybbu b0507 U00096 532235..533050
<313> 3631: 714-733
<400> 3631
TGTTGCCGCT ATCTCCATCT CCGGGCCTTC ATCAAGACTG ACAGAAGATC 50
GTTTTGTCAG TCAGGGTGAG CTGGTCAGAG ACACCGCCCG CGATATCAGC 100
<210> 3632
<211> 100
<212> DNA
<213> Escherichia coli K-12 MG1655
<220> ybbu b0507
<222> 584-683
<223> ybbu b0507 U00096 532235..533050
<313> 3632: 605-624
<400> 3632
ACCTGGAACA AGCGCGTGAA CTGGGCTATA CCGTAGATAA AGAAGAGCAT 50
GTTGTAGGTC TGAATTGCAT AGCTTCAGCA ATTTACGATG ATGTCGGTAG 100
<210> 3633
<211> 100
<212> DNA
<213> Escherichia coli K-12 MG1655
<220> ybbu b0507
<222> 513-612
<223> ybbu b0507 U00096 532235..533050
<313> 3633: 562-581
<400> 3633
CATCATTCTG CAAACCGGTT TGCAGCAGTT TACGCCAACT ACGCTTGTGG 50
ATATGCCCAC CTTGCTGAAG GACCTGGAAC AAGCGCGTGA ACTGGGCTAT 100
<210> 3634
<211> 100
<212> DNA
<213> Escherichia coli K-12 MG1655
<220> ybbv b0511
<222> 179-278

<223> ybbv b0511 U00096 536720..536998
<313> 3634: 230-249
<400> 3634
CGAAGATCTA TTGCCGAAAA CGCAAAGCCA GCGGACCTGG AAAACATTTA 50
ACTATTTTAC CTTATGGATG GGTTCGGTTC ATAACGTTCC CAATTATGTG 100
<210> 3635
<211> 100
<212> DNA
<213> Escherichia coli K-12 MG1655
<220> ybbv b0511
<222> 179-278
<223> ybbv b0511 U00096 536720..536998
<313> 3635: 222-241
<400> 3635
CGAAGATCTA TTGCCGAAAA CGCAAAGCCA GCGGACCTGG AAAACATTTA 50
ACTATTTTAC CTTATGGATG GGTTCGGTTC ATAACGTTCC CAATTATGTG 100
<210> 3636
<211> 100
<212> DNA
<213> Escherichia coli K-12 MG1655
<220> ybbv b0511
<222> 162-261
<223> ybbv b0511 U00096 536720..536998
<313> 3636: 203-222
<400> 3636
CAGCAACGCG GCTATAGCGA AGATCTATTG CCGAAAACGC AAAGCCAGCG 50
GACCTGGAAA ACATTTAACT ATTTTACCTT ATGGATGGGT TCGGTTCATA 100
<210> 3637
<211> 100
<212> DNA
<213> Escherichia coli K-12 MG1655
<220> ybbw b0512
<222> 1212-1311
<223> ybbw b0512 U00096 536998..538311
<313> 3637: 1251-1270
<400> 3637
TGTTTCATGG TTTGTCGGCG TCATCGTCGC CTTTGCGGCC TACGCCTTAT 50
TAAAGAAACG TACAACAGCA GAAAAAACAG GAGAGCAAAA AACCATAGGT 100
<210> 3638
<211> 100
<212> DNA
<213> Escherichia coli K-12 MG1655
<220> ybbw b0512
<222> 1073-1172
<223> ybbw b0512 U00096 536998..538311
<313> 3638: 1119-1138
<400> 3638
TTGATGAACT GTATACCGCA CCTGGCGATT ATAAATATTA CGATAACGGT 50
TTTAACCTCA CTGCGTTTTC AGTAACTCTG GTGGCCGTTA TTTTATCTCT 100
<210> 3639
<211> 100
<212> DNA
<213> Escherichia coli K-12 MG1655
<220> ybbw b0512
<222> 1042-1141
<223> ybbw b0512 U00096 536998..538311
<313> 3639: 1063-1082
<400> 3639
TATTTTGTGG TGATGCGCGG ACAAATTAAT CTTGATGAAC TGTATACCGC 50
ACCTGGCGAT TATAAATATT ACGATAACGG TTTTAACCTC ACTGCGTTTT 100
<210> 3640
<211> 100
<212> DNA
<213> Escherichia coli K-12 MG1655
<220> ybbx b0513
<222> 1263-1362
<223> ybbx b0513 U00096 538371..539732
<313> 3640: 1335-1354
<400> 3640
TATCACGAAA ACCATCTTAC GTGGTGATGT GATTTACGAC ATTGAACAGG 50
GCTTCCCTGT TGCGCCGAAA GGTCAATTTA TCCTTAAACA TCAGCAGTAA 100

<210> 3641
<211> 100
<212> DNA
<213> Escherichia coli K-12 MG1655
<220> ybbx b0513
<222> 1196-1295
<223> ybbx b0513 U00096 538371..539732
<313> 3641: 1219-1238
<400> 3641
TTACCAATGA CGATCTGGAA TATCGCCACA AAGTCAGCCC GTATGTTGGC 50
CGTACCATTG GCGCGCGTAT CACGAAAACC ATCTTACGTG GTGATGTGAT 100
<210> 3642
<211> 100
<212> DNA
<213> Escherichia coli K-12 MG1655
<220> ybbx b0513
<222> 1127-1226
<223> ybbx b0513 U00096 538371..539732
<313> 3642: 1175-1194
<400> 3642
AAAAAGGCCG TATCGCCCCA GGAAAAGATG CCGACTTCGT CTTCATTCAG 50
CCGAATAGCA GCTATGTTCT TACCAATGAC GATCTGGAAT ATCGCCACAA 100
<210> 3643
<211> 100
<212> DNA
<213> Escherichia coli K-12 MG1655
<220> ybby b0514
<222> 1196-1295
<223> ybby b0514 U00096 539783..541090
<313> 3643: 1251-1270
<400> 3643
ATCTGCAAGA CCTTCCATTA ACGCTTCGTC CTCTGCTCAG TAACGGCTTA 50
TTGGTCGGGA TTTTACTGGC TGTTCTTATG GATAACCTTA TTCCGTGGGA 100
<210> 3644
<211> 100
<212> DNA
<213> Escherichia coli K-12 MG1655
<220> ybby b0514
<222> 1178-1277
<223> ybby b0514 U00096 539783..541090
<313> 3644: 1216-1235
<400> 3644
TGGCATTACC GCCTGTGTAT CTGCAAGACC TTCCATTAAC GCTTCGTCCT 50
CTGCTCAGTA ACGGCTTATT GGTCGGGATT TTACTGGCTG TTCTTATGGA 100
<210> 3645
<211> 100
<212> DNA
<213> Escherichia coli K-12 MG1655
<220> ybby b0514
<222> 1098-1197
<223> ybby b0514 U00096 539783..541090
<313> 3645: 1135-1154
<400> 3645
GGTGTTTAGC CAGCAAATAA CGTTTACCGC TCGCAATATT TATCGACTCG 50
CATTGCCGTT ATTTGTCGGC ATATTTTTAA TGGCATTACC GCCTGTGTAT 100
<210> 3646
<211> 100
<212> DNA
<213> Escherichia coli K-12 MG1655
<220> ybbz b0515
<222> 1047-1146
<223> ybbz b0515 U00096 541112..542257
<313> 3646: 1109-1128
<400> 3646
ACCTTTAGCC GAAGTGCTCG CCAGCGGTGA AACCAATCTC TTCAACAGCG 50
CGCGAAATAT TGCCTGCGCC ATTAAAATAG GTCAGGGAAT TAAAAACTAA 100
<210> 3647
<211> 100
<212> DNA
<213> Escherichia coli K-12 MG1655
<220> ybbz b0515

<222> 1023-1122
<223> ybbz b0515 U00096 541112..542257
<313> 3647: 1076-1095
<400> 3647
ATTCAGCATT TTGCCTCGTC TGGCACCTTT AGCCGAAGTG CTCGCCAGCG 50
GTGAAACCAA TCTCTTCAAC AGCGCGCGAA ATATTGCCTG CGCCATTAAA 100
<210> 3648
<211> 100
<212> DNA
<213> Escherichia coli K-12 MG1655
<220> ybbz b0515
<222> 1001-1100
<223> ybbz b0515 U00096 541112..542257
<313> 3648: 1033-1052
<400> 3648
ACCAGTACGG CATTGACGCG GTATTCAGCA TTTTGCCTCG TCTGGCACCT 50
TTAGCCGAAG TGCTCGCCAG CGGTGAAACC AATCTCTTCA ACAGCGCGCG 100
<210> 3649
<211> 100
<212> DNA
<213> Escherichia coli K-12 MG1655
<220> flha b1880
<222> 1980-2079
<223> flha b1880 U00096 complement(1960996..1963074)
<313> 3649: 2051-2070
<400> 3649
CTTCCTGCGC CGCAGCTTGC CGCAGTTAGT GGTCCTGTCG AATCTGGAAC 50
TGTCTGATAA CCGACATATC CGCATGACGG CGACAATTGG CGGCAAATAA 100
<210> 3650
<211> 100
<212> DNA
<213> Escherichia coli K-12 MG1655
<220> flha b1880
<222> 1962-2061
<223> flha b1880 U00096 complement(1960996..1963074)
<313> 3650: 2001-2020
<400> 3650
GCGACCATTA TTGTCTCGCT TCCTGCGCCG CAGCTTGCCG CAGTTAGTGG 50
TCCTGTCGAA TCTGGAACTG TCTGATAACC GACATATCCG CATGACGGCG 100
<210> 3651
<211> 100
<212> DNA
<213> Escherichia coli K-12 MG1655
<220> flha b1880
<222> 1912-2011
<223> flha b1880 U00096 complement(1960996..1963074)
<313> 3651: 1969-1988
<400> 3651
CGTCAGGAGA TGCTGGGTGC GCCGCCAGTA TTGTTGGTGA ACCACGCGCT 50
GCGACCATTA TTGTCTCGCT TCCTGCGCCG CAGCTTGCCG CAGTTAGTGG 100
<210> 3652
<211> 100
<212> DNA
<213> Escherichia coli K-12 MG1655
<220> flhb b1881
<222> 1050-1149
<223> flhb b1881 U00096 complement(1963067..1964215)
<313> 3652: 1113-1132
<400> 3652
ACGCTGGCGT CTGGCTGGTG GACAGCGCCC TGTACAACCT ACTCATCTTC 50
CGGTGCCGGA AGCCCTGGAT TTTATTAACG AGAAACCGAC CCATGAGTAA 100
<210> 3653
<211> 100
<212> DNA
<213> Escherichia coli K-12 MG1655
<220> flhb b1881
<222> 1050-1149
<223> flhb b1881 U00096 complement(1963067..1964215)
<313> 3653: 1065-1084
<400> 3653
ACGCTGGCGT CTGGCTGGTG GACAGCGCCC TGTACAACCT ACTCATCTTC 50

CGGTGCCGGA AGCCCTGGAT TTTATTAACG AGAAACCGAC CCATGAGTAA 100
<210> 3654
<211> 100
<212> DNA
<213> Escherichia coli K-12 MG1655
<220> flhb b1881
<222> 884-983
<223> flhb b1881 U00096 complement(1963067..1964215)
<313> 3654: 920-939
<400> 3654
TGGTCGCGCT GCGCATTCGT GAAATTGGCG CTGAAAATAA CGTCCCGACG 50
CTTGAAGCGC CGCCGCTGGC GCGAGCGCTG TATCGACATG CGGAGATTGG 100
<210> 3655
<211> 100
<212> DNA
<213> Escherichia coli K-12 MG1655
<220> flhc b1892
<222> 480-579
<223> flhc b1892 U00096 complement(1975290..1975868)
<313> 3655: 537-556
<400> 3655
TCAACCGCCA TCCCGGGCAG TAAAAAGACG TAAACTTTCC CAGAATCCTG 50
CCGATATTAT CCCACAACTG CTGGATGAAC AGAGAGTACA GGCTGTTTAA 100
<210> 3656
<211> 100
<212> DNA
<213> Escherichia coli K-12 MG1655
<220> flhc b1892
<222> 465-564
<223> flhc b1892 U00096 complement(1975290..1975868)
<313> 3656: 512-531
<400> 3656
TGCCTGCAGC TTATGTCAAC CGCCATCCCG GGCAGTAAAA AGACGTAAAC 50
TTTCCCAGAA TCCTGCCGAT ATTATCCCAC AACTGCTGGA TGAACAGAGA 100
<210> 3657
<211> 100
<212> DNA
<213> Escherichia coli K-12 MG1655
<220> flhc b1892
<222> 319-418
<223> flhc b1892 U00096 complement(1975290..1975868)
<313> 3657: 387-406
<400> 3657
CCACAAGCAG AAGAAGGACC ACTGCTGGCA TTAACCCGTG CCTGGACATT 50
GGTGCGGTTT GTTGAAAGTG GATTACTGCA ACTTTCCAGC TGCAACTGCT 100
<210> 3658
<211> 100
<212> DNA
<213> Escherichia coli K-12 MG1655
<220> flhd b1893
<222> 261-360
<223> flhd b1893 U00096 complement(1975871..1976230)
<313> 3658: 308-327
<400> 3658
TGACGATCTC CAGCAAATTC ATACCGGCAT CATGCTCTCA ACACGCTTGC 50
TGAATGATGT TAATCAGCCT GAAGAAGCGC TGCGCAAGAA AAGGGCCTGA 100
<210> 3659
<211> 100
<212> DNA
<213> Escherichia coli K-12 MG1655
<220> flhd b1893
<222> 185-284
<223> flhd b1893 U00096 complement(1975871..1976230)
<313> 3659: 214-233
<400> 3659
AAACCAATCA ACTGGTTTGT CACTTCCGTT TTGACAGCCA CCAGACGATT 50
ACTCAGTTGA CGCAAGATTC CCGCGTTGAC GATCTCCAGC AAATTCATAC 100
<210> 3660
<211> 100
<212> DNA
<213> Escherichia coli K-12 MG1655

<220> flhd b1893
<222> 142-241
<223> flhd b1893 U00096 complement(1975871..1976230)
<313> 3660: 178-197
<400> 3660
ACGTTAGCGG CACTGACTCT TCCGCAAATG GTTAAGCTGG CAGAAACCAA 50
TCAACTGGTT TGTCACTTCC GTTTTGACAG CCACCAGACG ATTACTCAGT 100
<210> 3661
<211> 100
<212> DNA
<213> Escherichia coli K-12 MG1655
<220> flhe b1879
<222> 281-380
<223> flhe b1879 U00096 complement(1960604..1960996)
<313> 3661: 311-330
<400> 3661
CCGGTATAGC GGCAGCAGAA CCGTTGCGAT TTATCTGGGA AGTGCCAGGC 50
GGTGGGCGGT TAATTCCACC GCTAAAGGTA CAACGTAATG AAGTGATTGT 100
<210> 3662
<211> 100
<212> DNA
<213> Escherichia coli K-12 MG1655
<220> flhe b1879
<222> 220-319
<223> flhe b1879 U00096 complement(1960604..1960996)
<313> 3662: 275-294
<400> 3662
TTGTGTTCGC AATCTCGTTG TGTCGAATTA GAGGGGCAGA GCGGAACCAC 50
CGTGGCCTTT TCCGGTATAG CGGCAGCAGA ACCGTTGCGA TTTATCTGGG 100
<210> 3663
<211> 100
<212> DNA
<213> Escherichia coli K-12 MG1655
<220> flhe b1879
<222> 188-287
<223> flhe b1879 U00096 complement(1960604..1960996)
<313> 3663: 223-242
<400> 3663
TCGGCCCGAC ACCTTCAGGA CTGCGGGTTC GCTTGTGTTC GCAATCTCGT 50
TGTGTCGAAT TAGAGGGGCA GAGCGGAACC ACCGTGGCCT TTTCCGGTAT 100
<210> 3664
<211> 100
<212> DNA
<213> Escherichia coli K-12 MG1655
<220> ybch b0568
<222> 792-891
<223> ybch b0568 U00096 complement(586314..587204)
<313> 3664: 859-878
<400> 3664
AATTGCCTCG CTGTTAGCGA AACGTTCCTC TTGCGGTAAA GACACTCTCT 50
ATTTCTCTCT GCGCTATTTG CCCGTCGCGC ACGGCATTCT CGAGTATTAA 100
<210> 3665
<211> 100
<212> DNA
<213> Escherichia coli K-12 MG1655
<220> ybch b0568
<222> 780-879
<223> ybch b0568 U00096 complement(586314..587204)
<313> 3665: 822-841
<400> 3665
ACCGGACGCA GAAATTGCCT CGCTGTTAGC GAAACGTTCC TCTTGCGGTA 50
AAGACACTCT CTATTTCTCT CTGCGCTATT TGCCCGTCGC GCACGGCATT 100
<210> 3666
<211> 100
<212> DNA
<213> Escherichia coli K-12 MG1655
<220> ybch b0568
<222> 735-834
<223> ybch b0568 U00096 complement(586314..587204)
<313> 3666: 768-787
<400> 3666

ACTTTTTGTC GCCGGCAAAG GCAAAACCTT TACAGCGAAA CCGAAACCGG 50
ACGCAGAAAT TGCCTCGCTG TTAGCGAAAC GTTCCTCTTG CGGTAAAGAC 100
<210> 3667
<211> 100
<212> DNA
<213> Escherichia coli K-12 MG1655
<220> ybci b0528
<222> 423-522
<223> ybci b0528 U00096 complement(555255..555776)
<313> 3667: 468-487
<400> 3667
CACCACGCCG TACGGGCATC AGGTGATTAT TTCTGAATTG ATGTGGGTGT 50
GGTTGCCGGG GATGCTACTG ATGGGAATGT TGTGGTGGCG CAGACGATAA 100
<210> 3668
<211> 100
<212> DNA
<213> Escherichia coli K-12 MG1655
<220> ybci b0528
<222> 356-455
<223> ybci b0528 U00096 complement(555255..555776)
<313> 3668: 407-426
<400> 3668
GGTCAGATGA ACGCTTTTTC GCTCCCTGGC AGGTGATAAA AGTCGCGCCG 50
TTTGCATTGT CTCGTTACAC CACGCCGTAC GGGCATCAGG TGATTATTTC 100
<210> 3669
<211> 100
<212> DNA
<213> Escherichia coli K-12 MG1655
<220> ybci b0528
<222> 315-414
<223> ybci b0528 U00096 complement(555255..555776)
<313> 3669: 358-377
<400> 3669
GGTAACCACT GGCGGTAAAG GCGTTGGCTG GCTGTGGCCG TGGTCAGATG 50
AACGCTTTTT CGCTCCCTGG CAGGTGATAA AAGTCGCGCC GTTTGCATTG 100
<210> 3670
<211> 100
<212> DNA
<213> Escherichia coli K-12 MG1655
<220> ybcj b0529
<222> 135-234
<223> ybcj b0529 U00096 complement(555884..556117)
<313> 3670: 201-220
<400> 3670
GGTGAAAGTC GACGGTGCGG TTGAAACGCG CAAACGCTGC AAAATCGTCG 50
CCGGTCAGAC AGTGAGTTTT GCAGGTCACA GCGTACAGGT TGTTGCCTGA 100
<210> 3671
<211> 100
<212> DNA
<213> Escherichia coli K-12 MG1655
<220> ybcj b0529
<222> 120-219
<223> ybcj b0529 U00096 complement(555884..556117)
<313> 3671: 158-177
<400> 3671
GATTGCCGAA GGCCAGGTGA AAGTCGACGG TGCGGTTGAA ACGCGCAAAC 50
GCTGCAAAAT CGTCGCCGGT CAGACAGTGA GTTTTGCAGG TCACAGCGTA 100
<210> 3672
<211> 100
<212> DNA
<213> Escherichia coli K-12 MG1655
<220> ybcj b0529
<222> 11-110
<223> ybcj b0529 U00096 complement(555884..556117)
<313> 3672: 80-99
<400> 3672
GGATGAGTAA CATGGCGACA TTTTCTTTAG GTAAACATCC GCACGTTGAG 50
CTGTGCGACT TGCTGAAACT GGAAGGCTGG AGCGAAAGCG GCGCGCAGGC 100
<210> 3673
<211> 100
<212> DNA

<213> Escherichia coli K-12 MG1655
<220> ybck b0545
<222> 1428-1527
<223> ybck b0545 U00096 568125..569651
<313> 3673: 1485-1504
<400> 3673
TGGCATTGTT TTTAAACACT ATCCTTTAAT GAAAGTAATA TCCGCCCAGC 50
AGGCGATAAG TGCTCTCAAA TATATGGTTG ATGGTGAGAT TTATTTCTAA 100
<210> 3674
<211> 100
<212> DNA
<213> Escherichia coli K-12 MG1655
<220> ybck b0545
<222> 1303-1402
<223> ybck b0545 U00096 568125..569651
<313> 3674: 1338-1357
<400> 3674
GATTTTTATG CTATTGACTT AACCAGTAAA AATGGACGAG AGTTATGCCG 50
TACACTTGCC TATAAAACAT TCGAAAAAAT CATAATTAAT ACGGATAATA 100
<210> 3675
<211> 100
<212> DNA
<213> Escherichia coli K-12 MG1655
<220> ybck b0545
<222> 1163-1262
<223> ybck b0545 U00096 568125..569651
<313> 3675: 1181-1200
<400> 3675
TTAATAATTT AATCGTTGCA TTGTCTGTCG CGCCTGAAGT TACCGCTATA 50
GCAGAGAAAA TAAGACTATT AGATAAGGAA TTACGAAGGG CTTCGGTATC 100
<210> 3676
<211> 100
<212> DNA
<213> Escherichia coli K-12 MG1655
<220> ybcl b0546
<222> 409-508
<223> ybcl b0546 U00096 570116..570667
<313> 3676: 443-462
<400> 3676
CATCATTACC AGTTTAAAGT ATGGGCTCTA AAAACTGAAA AGATTCCTGT 50
AGATTCTAAC TCCAGCGGAG CGTTAGTTGG TTATATGCTT AATGCTAATA 100
<210> 3677
<211> 100
<212> DNA
<213> Escherichia coli K-12 MG1655
<220> ybcl b0546
<222> 360-459
<223> ybcl b0546 U00096 570116..570667
<313> 3677: 385-404
<400> 3677
TGGCTATGCT GGGTTTGGTG GCGCATGTCC TCCTAAAGGA GATAAACCAC 50
ATCATTACCA GTTTAAAGTA TGGGCTCTAA AAACTGAAAA GATTCCTGTA 100
<210> 3678
<211> 100
<212> DNA
<213> Escherichia coli K-12 MG1655
<220> ybcl b0546
<222> 278-377
<223> ybcl b0546 U00096 570116..570667
<313> 3678: 331-350
<400> 3678
CAGTAACATA TTTGCCCGTT GATGCAGGGA GACGTGATGG AACAAAACTG 50
CCGACTGGTG CTGTTCAAGG CCGAAATGAT TTTGGCTATG CTGGGTTTGG 100
<210> 3679
<211> 100
<212> DNA
<213> Escherichia coli K-12 MG1655
<220> ybcm b0547
<222> 562-661
<223> ybcm b0547 U00096 570677..571474
<313> 3679: 608-627

<400> 3679
CTCTACATTT CTGTTTCTAC ATTACATCGC CGTCTAGCAA GCGAGGGGGT 50
AAGTTTCCAA AGTATACTGG ACGATGTGAG GTTAAATAAT GCGTTGTCTG 100
<210> 3680
<211> 100
<212> DNA
<213> Escherichia coli K-12 MG1655
<220> ybcm b0547
<222> 515-614
<223> ybcm b0547 U00096 570677..571474
<313> 3680: 546-565
<400> 3680
TATCCGAACC CGGCACAAAA TGGACAGCAA ACAAGGTTGC TCGATATCTC 50
TACATTTCTG TTTCTACATT ACATCGCCGT CTAGCAAGCG AGGGGGTAAG 100
<210> 3681
<211> 100
<212> DNA
<213> Escherichia coli K-12 MG1655
<220> ybcm b0547
<222> 375-474
<223> ybcm b0547 U00096 570677..571474
<313> 3681: 388-407
<400> 3681
TGTTTCCCGA GGCTTCTCAA CTAAAATTCA GGAGCACTTA GCAATGGGCG 50
TTCTACTTTC GTTAGGAGTG AATCATGTTA ATCATTTACT TTTATCATAT 100
<210> 3682
<211> 100
<212> DNA
<213> Escherichia coli K-12 MG1655
<220> ybcn b0548
<222> 357-456
<223> ybcn b0548 U00096 571689..572144
<313> 3682: 401-420
<400> 3682
CCTGTGTAAG GTTGAAGCCT GGGCGATGAA TATTGGTTGC CACCTGACTA 50
TTCCACAGAG CTGCGAGTTC CAGCTGCTGC GCGACAAGCA GGAGGCGTAA 100
<210> 3683
<211> 100
<212> DNA
<213> Escherichia coli K-12 MG1655
<220> ybcn b0548
<222> 292-391
<223> ybcn b0548 U00096 571689..572144
<313> 3683: 327-346
<400> 3683
GATATCACCA CTATCCAGTC GTTACGCCAT ACCTCCGATC TTGATACCGG 50
AGAGATGTAT GTCTTCCTGT GTAAGGTTGA AGCCTGGGCG ATGAATATTG 100
<210> 3684
<211> 100
<212> DNA
<213> Escherichia coli K-12 MG1655
<220> ybcn b0548
<222> 193-292
<223> ybcn b0548 U00096 571689..572144
<313> 3684: 229-248
<400> 3684
AGGGGTAAAA CGTTCGCCAC TCCAGCTTGG GTAAAAGATG CTCTCAAACA 50
CACATATCTC GGTTATGAAA CCAAAGACCT GGTTGATGTC GTAACCGGTG 100
<210> 3685
<211> 100
<212> DNA
<213> Escherichia coli K-12 MG1655
<220> b1513 b1514
<222> 1395-1494
<223> b1513 b1514 U00096 1599514..1601049
<313> 3685: 1452-1471
<400> 3685
GATCGAACTG ATGGCAGATC GTGTGTATGT GATGCATCAG GGCGAAATTA 50
CCCACTCTGC ACTGACCGAG CGCGATATTA ATGTCGAGAC TATTATGCGC 100
<210> 3686
<211> 100

<212> DNA
<213> Escherichia coli K-12 MG1655
<220> b1513 b1514
<222> 1342-1441
<223> b1513 b1514 U00096 1599514..1601049
<313> 3686: 1383-1402
<400> 3686
AGCATCGCCG CACAAAATGT GGCTGTGCTG CTTATCTCCT CCGACCTGGA 50
AGAGATCGAA CTGATGGCAG ATCGTGTGTA TGTGATGCAT CAGGGCGAAA 100
<210> 3687
<211> 100
<212> DNA
<213> Escherichia coli K-12 MG1655
<220> b1513 b1514
<222> 1171-1270
<223> b1513 b1514 U00096 1599514..1601049
<313> 3687: 1221-1240
<400> 3687
AATATTAAAT TCAACCAACC GGAACAAGCT GCACGGACAT TATCCGGTGG 50
CAACCAGCAA AAAATCCTCA TTGCCAAATG CTTGGAAGCT TCGCCGCAAG 100
<210> 3688
<211> 100
<212> DNA
<213> Escherichia coli K-12 MG1655
<220> ybco b0550
<222> 192-291
<223> ybco b0550 U00096 572307..572597
<313> 3688: 262-281
<400> 3688
CACGCATTTT GTTGACGCTG GATATGCAAA AGAATGCGCG CTGGAAGGTA 50
TGGCGAGAAC ACAGGTTATC TGGCTGAAAG AGGGGGTTAT TAAGGCGTGA 100
<210> 3689
<211> 100
<212> DNA
<213> Escherichia coli K-12 MG1655
<220> ybco b0550
<222> 144-243
<223> ybco b0550 U00096 572307..572597
<313> 3689: 202-221
<400> 3689
GATTGCCACC ATTGCATGTT CTGCCTGCCA CGACGAAATC GACCGCCGCA 50
CGCATTTTGT TGACGCTGGA TATGCAAAAG AATGCGCGCT GGAAGGTATG 100
<210> 3690
<211> 100
<212> DNA
<213> Escherichia coli K-12 MG1655
<220> ybco b0550
<222> 106-205
<223> ybco b0550 U00096 572307..572597
<313> 3690: 155-174
<400> 3690
ACTGGATTGT GCGGCACCGG TACGAAACCG CCAGACCTGA TTGCCACCAT 50
TGCATGTTCT GCCTGCCACG ACGAAATCGA CCGCCGCACG CATTTTGTTG 100
<210> 3691
<211> 100
<212> DNA
<213> Escherichia coli K-12 MG1655
<220> hupa b4001
<222> 174-273
<223> hupa b4001 U00096 4197859..4198131
<313> 3691: 248-267
<400> 3691
TACTGGCCGC AACCCGCAGA CCGGTAAAGA AATCAAAATT GCCGCAGCTA 50
ACGTACCGGC ATTTGTTTCT GGCAAGGCAC TGAAAGACGC AGTTAAGTAA 100
<210> 3692
<211> 100
<212> DNA
<213> Escherichia coli K-12 MG1655
<220> hupa b4001
<222> 119-218
<223> hupa b4001 U00096 4197859..4198131

<313> 3692: 173-192
<400> 3692
ATGCTGTACA ACTGGTTGGT TTCGGTACCT TCAAAGTGAA CCACCGCGCT 50
GAGCGTACTG GCCGCAACCC GCAGACCGGT AAAGAAATCA AAATTGCCGC 100
<210> 3693
<211> 100
<212> DNA
<213> Escherichia coli K-12 MG1655
<220> hupa b4001
<222> 66-165
<223> hupa b4001 U00096 4197859..4198131
<313> 3693: 102-121
<400> 3693
AGCTGCTCTG GAGTCCACTC TGGCTGCAAT TACTGAGTCT CTGAAAGAAG 50
GCGATGCTGT ACAACTGGTT GGTTTCGGTA CCTTCAAAGT GAACCACCGC 100
<210> 3694
<211> 100
<212> DNA
<213> Escherichia coli K-12 MG1655
<220> ybcq b0552
<222> 240-339
<223> ybcq b0552 U00096 573179..573562
<313> 3694: 293-312
<400> 3694
ATTCATGTCA CTGGCAGGTA AGCATTGCTG CTCTGATGGT TATATCGGGA 50
AAAGGTTACA GAAGGCTGAG GGCATAATTG AAGGGATGTT AATGGCATTA 100
<210> 3695
<211> 100
<212> DNA
<213> Escherichia coli K-12 MG1655
<220> ybcq b0552
<222> 233-332
<223> ybcq b0552 U00096 573179..573562
<313> 3695: 265-284
<400> 3695
GTATGACATT CATGTCACTG GCAGGTAAGC ATTGCTGCTC TGATGGTTAT 50
ATCGGGAAAA GGTTACAGAA GGCTGAGGGC ATAATTGAAG GGATGTTAAT 100
<210> 3696
<211> 100
<212> DNA
<213> Escherichia coli K-12 MG1655
<220> ybcq b0552
<222> 132-231
<223> ybcq b0552 U00096 573179..573562
<313> 3696: 173-192
<400> 3696
ATGTTGTGAC GATGACGCGA TGATCATTTG CGGGTGCATG GCCCGTCTGA 50
AAAAGAACAA CAGCGATTTG CACGATTTAT TAGTAGATTA TTATGTAGTC 100
<210> 3697
<211> 100
<212> DNA
<213> Escherichia coli K-12 MG1655
<220> hupb b0441
<222> 174-273
<223> hupb b0441 U00096 460675..460947
<313> 3697: 252-271
<400> 3697
TACTGGCCGC AACCCGCAGA CCGGTAAAGA GATCACCATC GCTGCTGCTA 50
AAGTACCGAG CTTCCGTGCA GGTAAAGCAC TGAAAGACGC GGTAAACTAA 100
<210> 3698
<211> 100
<212> DNA
<213> Escherichia coli K-12 MG1655
<220> hupb b0441
<222> 174-273
<223> hupb b0441 U00096 460675..460947
<313> 3698: 197-216
<400> 3698
TACTGGCCGC AACCCGCAGA CCGGTAAAGA GATCACCATC GCTGCTGCTA 50
AAGTACCGAG CTTCCGTGCA GGTAAAGCAC TGAAAGACGC GGTAAACTAA 100
<210> 3699

<211> 100
<212> DNA
<213> Escherichia coli K-12 MG1655
<220> hupb b0441
<222> 83-182
<223> hupb b0441 U00096 460675..460947
<313> 3699: 142-161
<400> 3699
TTATTGCTTC CGTAACTGAA TCTCTGAAAG AAGGGGATGA TGTAGCACTG 50
GTAGGTTTTG GTACTTTTGC CGTTAAAGAG CGTGCTGCCC GTACTGGCCG 100
<210> 3700
<211> 100
<212> DNA
<213> Escherichia coli K-12 MG1655
<220> b1516 b1517
<222> 924-1023
<223> b1516 b1517 U00096 1603075..1604097
<313> 3700: 977-996
<400> 3700
CGTTCAGGGC TATGACTACG AAGCGGATGG TAATGGCATC GTACTGTTAC 50
CGGAGCGCGT GATATTCAAC AAAGAGAATA TCGGCAAATA CGATTTCTGA 100
<210> 3701
<211> 100
<212> DNA
<213> Escherichia coli K-12 MG1655
<220> b1516 b1517
<222> 885-984
<223> b1516 b1517 U00096 1603075..1604097
<313> 3701: 937-956
<400> 3701
TATCAAGGGC GTAGGTCAGG TTGAAGTCTC GCCAAACAGC GTTCAGGGCT 50
ATGACTACGA AGCGGATGGT AATGGCATCG TACTGTTACC GGAGCGCGTG 100
<210> 3702
<211> 100
<212> DNA
<213> Escherichia coli K-12 MG1655
<220> b1516 b1517
<222> 865-964
<223> b1516 b1517 U00096 1603075..1604097
<313> 3702: 887-906
<400> 3702
AAAACGGGCG ACAAGCTGGA TATCAAGGGC GTAGGTCAGG TTGAAGTCTC 50
GCCAAACAGC GTTCAGGGCT ATGACTACGA AGCGGATGGT AATGGCATCG 100
<210> 3703
<211> 100
<212> DNA
<213> Escherichia coli K-12 MG1655
<220> b2245 b2245
<222> 705-804
<223> b2245 b2245 U00096 complement(2356062..2356865)
<313> 3703: 767-786
<400> 3703
TGTTGGCGTT GACACGATGC TCTACAGCGA TGCCCTGGAT CAACGACTGG 50
CGATGTTTAA ATCAGGCAAA AATGGGCCAC GCATAAAAGG TAGTTATTGA 100
<210> 3704
<211> 100
<212> DNA
<213> Escherichia coli K-12 MG1655
<220> b2245 b2245
<222> 669-768
<223> b2245 b2245 U00096 complement(2356062..2356865)
<313> 3704: 720-739
<400> 3704
GCAGCAATGC CTGGCGTGGG GAGCGAACTT TGTCGCTGTT GGCGTTGACA 50
CGATGCTCTA CAGCGATGCC CTGGATCAAC GACTGGCGAT GTTTAAATCA 100
<210> 3705
<211> 100
<212> DNA
<213> Escherichia coli K-12 MG1655
<220> b2245 b2245
<222> 637-736

<223> b2245 b2245 U00096 complement(2356062..2356865)
<313> 3705: 684-703
<400> 3705
GCTGGTTTTC TGGCTGTGGC TCCTGATATG GCGCAGCAAT GCCTGGCGTG 50
GGGAGCGAAC TTTGTCGCTG TTGGCGTTGA CACGATGCTC TACAGCGATG 100
<210> 3706
<211> 100
<212> DNA
<213> Escherichia coli K-12 MG1655
<220> yqea yqeA
<222> 834-933
<223> yqea yqeA U00096 3009482..3010414
<313> 3706: 907-926
<400> 3706
GTTTGCTCGC TCTCGCGCAG GTCGTGAAGC ACTGATTACC GTATTGAGCA 50
AAGCGAAAGA AGGGATTGAA GGCAAAACAG GAACGGTGAT TTGTCAGTAA 100
<210> 3707
<211> 100
<212> DNA
<213> Escherichia coli K-12 MG1655
<220> yqea yqeA
<222> 822-921
<223> yqea yqeA U00096 3009482..3010414
<313> 3707: 859-878
<400> 3707
AGCTGCCGCC TCGTTTGCTC GCTCTCGCGC AGGTCGTGAA GCACTGATTA 50
CCGTATTGAG CAAAGCGAAA GAAGGGATTG AAGGCAAAAC AGGAACGGTG 100
<210> 3708
<211> 100
<212> DNA
<213> Escherichia coli K-12 MG1655
<220> yqea yqeA
<222> 802-901
<223> yqea yqeA U00096 3009482..3010414
<313> 3708: 821-840
<400> 3708
TCTATGCTGC CTAAAGTGGA AGCTGCCGCC TCGTTTGCTC GCTCTCGCGC 50
AGGTCGTGAA GCACTGATTA CCGTATTGAG CAAAGCGAAA GAAGGGATTG 100
<210> 3709
<211> 100
<212> DNA
<213> Escherichia coli K-12 MG1655
<220> ybcr b0555
<222> 117-216
<223> ybcr b0555 U00096 576621..576836
<313> 3709: 177-196
<400> 3709
AGGTGTGCTG GGTAGCCTGG TATTTGGCCT GCTGACGTAC CTGACAAACC 50
TTTATTTCAA GATTAAAGAA GATAAGCGCA AGGCTGCGAG AGGTGAATAA 100
<210> 3710
<211> 100
<212> DNA
<213> Escherichia coli K-12 MG1655
<220> ybcr b0555
<222> 117-216
<223> ybcr b0555 U00096 576621..576836
<313> 3710: 140-159
<400> 3710
AGGTGTGCTG GGTAGCCTGG TATTTGGCCT GCTGACGTAC CTGACAAACC 50
TTTATTTCAA GATTAAAGAA GATAAGCGCA AGGCTGCGAG AGGTGAATAA 100
<210> 3711
<211> 100
<212> DNA
<213> Escherichia coli K-12 MG1655
<220> ybcr b0555
<222> 64-163
<223> ybcr b0555 U00096 576621..576836
<313> 3711: 95-114
<400> 3711
TACTGGTTTT TACAGCTGCT AGATAAAGTC ACTCCCTCAC AGTGGGCAGC 50
AATAGGTGTG CTGGGTAGCC TGGTATTTGG CCTGCTGACG TACCTGACAA 100

<210> 3712
<211> 100
<212> DNA
<213> Escherichia coli K-12 MG1655
<220> adhc b0357
<222> 1011-1110
<223> adhc b0357 U00096 complement(377686..378795)
<313> 3712: 1074-1093
<400> 3712
GGAACCGTTT GTCACGCATA CCATGAGCCT GGATGAAATT AATGACGCCT 50
TCGACCTGAT GCATGAAGGC AAATCCATTC GAACCGTAAT TCGTTACTGA 100
<210> 3713
<211> 100
<212> DNA
<213> Escherichia coli K-12 MG1655
<220> adhc b0357
<222> 1000-1099
<223> adhc b0357 U00096 complement(377686..378795)
<313> 3713: 1028-1047
<400> 3713
GATATCGATC TGGAACCGTT TGTCACGCAT ACCATGAGCC TGGATGAAAT 50
TAATGACGCC TTCGACCTGA TGCATGAAGG CAAATCCATT CGAACCGTAA 100
<210> 3714
<211> 100
<212> DNA
<213> Escherichia coli K-12 MG1655
<220> adhc b0357
<222> 917-1016
<223> adhc b0357 U00096 complement(377686..378795)
<313> 3714: 982-1001
<400> 3714
GTCGCGTATG GAAAGGTTCC GCGTTTGGCG GCGTGAAAGG TCGTTCCCAG 50
TTACCGGGCA TGGTTGAAGA TGCGATGAAA GGTGATATCG ATCTGGAACC 100
<210> 3715
<211> 100
<212> DNA
<213> Escherichia coli K-12 MG1655
<220> b2246 b2246
<222> 1228-1327
<223> b2246 b2246 U00096 complement(2356883..2358211)
<313> 3715: 1274-1293
<400> 3715
GGATTACTGA CGCTGGCAGC GGTGGCGGTC ATCGGTTCGC TGATTATTTT 50
CACTCTGCGT GTAAATCGCA CTGTTGCGCA GACCGACGTG GCACATCATT 100
<210> 3716
<211> 100
<212> DNA
<213> Escherichia coli K-12 MG1655
<220> b2246 b2246
<222> 1205-1304
<223> b2246 b2246 U00096 complement(2356883..2358211)
<313> 3716: 1220-1239
<400> 3716
CGTTATTTGC CAGCGATGCG GCGGGATTAC TGACGCTGGC AGCGGTGGCG 50
GTCATCGGTT CGCTGATTAT TTTCACTCTG CGTGTAAATC GCACTGTTGC 100
<210> 3717
<211> 100
<212> DNA
<213> Escherichia coli K-12 MG1655
<220> b2246 b2246
<222> 1061-1160
<223> b2246 b2246 U00096 complement(2356883..2358211)
<313> 3717: 1087-1106
<400> 3717
GGTTTATTGC CGTGCAGCCA GTGTTCTGGA CGATGCCGAC ACAACTGCTT 50
TCCGGTACGG CGCTGGCTGC GGGAATTGGT TTTGTAAACC TGTTTGGTGC 100
<210> 3718
<211> 100
<212> DNA
<213> Escherichia coli K-12 MG1655
<220> ybcs b0556

<222> 399-498
<223> ybcs b0556 U00096 576836..577333
<313> 3718: 476-495
<400> 3718
TCAGCTGCGT CGCTGGACAT ACGCTGGCGG TAAGCAATGG AAAGGCCTGA 50
TGACTCGTCG TGAGATTGAG CGTGAAGTCT GTTTGTGGGG GCAACAGTGA 100
<210> 3719
<211> 100
<212> DNA
<213> Escherichia coli K-12 MG1655
<220> ybcs b0556
<222> 344-443
<223> ybcs b0556 U00096 576836..577333
<313> 3719: 367-386
<400> 3719
GAACATCGAC GCTTCTTCGC AAAATAAACC AGGGCGATAT CAAAGGCGCA 50
TGTGATCAGC TGCGTCGCTG GACATACGCT GGCGGTAAGC AATGGAAAGG 100
<210> 3720
<211> 100
<212> DNA
<213> Escherichia coli K-12 MG1655
<220> ybcs b0556
<222> 215-314
<223> ybcs b0556 U00096 576836..577333
<313> 3720: 244-263
<400> 3720
AAGCACTCTT GAATAAAGAC CTTGCCACTG TCGCCAGACA AATTAACCCG 50
TATATCAAAG TCGATATACC GGAAACAACG CGCGGCGCTC TTTACTCATT 100
<210> 3721
<211> 100
<212> DNA
<213> Escherichia coli K-12 MG1655
<220> b1518 b1518
<222> 192-291
<223> b1518 b1518 U00096 1605023..1605313
<313> 3721: 261-280
<400> 3721
CCATAAAACC ACGCCCCACT ACAAAACCTG TGTCGCGAAA CTGGAATCTT 50
TAATGACCGG GCCGCGTAAA AAACGTCTGT TCAATGGTTT GATGCCGTGA 100
<210> 3722
<211> 100
<212> DNA
<213> Escherichia coli K-12 MG1655
<220> b1518 b1518
<222> 166-265
<223> b1518 b1518 U00096 1605023..1605313
<313> 3722: 208-227
<400> 3722
TATAAAGATG AAGACGCAGT GGCGTTCCAT AAAACCACGC CCCACTACAA 50
AACCTGTGTC GCGAAACTGG AATCTTTAAT GACCGGGCCG CGTAAAAAAC 100
<210> 3723
<211> 100
<212> DNA
<213> Escherichia coli K-12 MG1655
<220> b1518 b1518
<222> 86-185
<223> b1518 b1518 U00096 1605023..1605313
<313> 3723: 109-128
<400> 3723
CTGTACAGGA AGAAGGCAAT TTGCGCTTCG ATGTCTTACA GGACCCGGAA 50
GTGAATTCGC GCTTTTATAT CTACGAAGCC TATAAAGATG AAGACGCAGT 100
<210> 3724
<211> 100
<212> DNA
<213> Escherichia coli K-12 MG1655
<220> b2247 b2247
<222> 1119-1218
<223> b2247 b2247 U00096 complement(2358229..2359446)
<313> 3724: 1182-1201
<400> 3724
TCCGGTGAAT GGTCGTATTC ATAAATCAGT GCTTGATAAA CCCGGTTTTG 50

GCGTCGAACT CAATCGTGAC TGCAATTTGA AACGCCCCTA CAGCCACTAA 100
<210> 3725
<211> 100
<212> DNA
<213> Escherichia coli K-12 MG1655
<220> b2247 b2247
<222> 1072-1171
<223> b2247 b2247 U00096 complement(2358229..2359446)
<313> 3725: 1126-1145
<400> 3725
TCAACGATGC GTCCGCAGTT TGACCCGATT CTGCTCAATG AGCCGGTTCC 50
GGTGAATGGT CGTATTCATA AATCAGTGCT TGATAAACCC GGTTTTGGCG 100
<210> 3726
<211> 100
<212> DNA
<213> Escherichia coli K-12 MG1655
<220> b2247 b2247
<222> 1008-1107
<223> b2247 b2247 U00096 complement(2358229..2359446)
<313> 3726: 1062-1081
<400> 3726
CCATGCGGTG ATCACCTTCA CCAATACGCC ATTCAGCGAA TTCCTGATGA 50
CCAGCCCGGA TTGTTCAACG ATGCGTCCGC AGTTTGACCC GATTCTGCTC 100
<210> 3727
<211> 100
<212> DNA
<213> Escherichia coli K-12 MG1655
<220> ybct b0557
<222> 363-462
<223> ybct b0557 U00096 577330..577791
<313> 3727: 416-435
<400> 3727
CACCGCTGAA CGGGATTATT TCACCCTCAG AGAGAGGCTG ATCACTATGC 50
AAAAACAACT GGAAGGAACC CAGAAGTATA TTAATGAGCA GTGCAGATAG 100
<210> 3728
<211> 100
<212> DNA
<213> Escherichia coli K-12 MG1655
<220> ybct b0557
<222> 314-413
<223> ybct b0557 U00096 577330..577791
<313> 3728: 363-382
<400> 3728
CCACCACGGC CTCCGGCGTG GATAATGCAG CCTCCCCCCG ACTGGCAGAC 50
ACCGCTGAAC GGGATTATTT CACCCTCAGA GAGAGGCTGA TCACTATGCA 100
<210> 3729
<211> 100
<212> DNA
<213> Escherichia coli K-12 MG1655
<220> ybct b0557
<222> 287-386
<223> ybct b0557 U00096 577330..577791
<313> 3729: 333-352
<400> 3729
AAGCAGTCTG TCAGTCAGTG CGTGAAGCCA CCACGGCCTC CGGCGTGGAT 50
AATGCAGCCT CCCCCCGACT GGCAGACACC GCTGAACGGG ATTATTTCAC 100
<210> 3730
<211> 100
<212> DNA
<213> Escherichia coli K-12 MG1655
<220> b1519 b1519
<222> 660-759
<223> b1519 b1519 U00096 1605370..1606128
<313> 3730: 733-752
<400> 3730
CTACCATCAG ATGCTGGAAG AGCAGTATCC ACTGCAAGAG AACGGACAGA 50
TACTGCTGGC ATTTCCGCGT CTGTTTATTG TTGCCCGGCG TATGGAGTAA 100
<210> 3731
<211> 100
<212> DNA
<213> Escherichia coli K-12 MG1655

<220> b1519 b1519
<222> 625-724
<223> b1519 b1519 U00096 1605370..1606128
<313> 3731: 685-704
<400> 3731
CTGACCGAGA GCGAACAGCA GCTTTTTCTT AAGCGCTACC ATCAGATGCT 50
GGAAGAGCAG TATCCACTGC AAGAGAACGG ACAGATACTG CTGGCATTTC 100
<210> 3732
<211> 100
<212> DNA
<213> Escherichia coli K-12 MG1655
<220> b1519 b1519
<222> 595-694
<223> b1519 b1519 U00096 1605370..1606128
<313> 3732: 625-644
<400> 3732
GCCACTGGAT TACGTCCGTG GTTACAGGAT CTGACCGAGA GCGAACAGCA 50
GCTTTTTCTT AAGCGCTACC ATCAGATGCT GGAAGAGCAG TATCCACTGC 100
<210> 3733
<211> 100
<212> DNA
<213> Escherichia coli K-12 MG1655
<220> adhe b1242
<222> 2577-2676
<223> adhe b1242 U00096 complement(1294669..1297344)
<313> 3733: 2649-2668
<400> 3733
GGATACCTAC TACGGTCGTG ATTATGTAGA AGGTGAAACT GCAGCGAAGA 50
AAGAAGCTGC TCCGGCTAAA GCTGAGAAAA AAGCGAAAAA ATCCGCTTAA 100
<210> 3734
<211> 100
<212> DNA
<213> Escherichia coli K-12 MG1655
<220> adhe b1242
<222> 2542-2641
<223> adhe b1242 U00096 complement(1294669..1297344)
<313> 3734: 2557-2576
<400> 3734
TACCCGCTGA TCTCCGAGCT GAAACAGATT CTGCTGGATA CCTACTACGG 50
TCGTGATTAT GTAGAAGGTG AAACTGCAGC GAAGAAAGAA GCTGCTCCGG 100
<210> 3735
<211> 100
<212> DNA
<213> Escherichia coli K-12 MG1655
<220> adhe b1242
<222> 2419-2518
<223> adhe b1242 U00096 complement(1294669..1297344)
<313> 3735: 2485-2504
<400> 3735
GCTGAACTGG GTATTCCGAA ATCTATCCGT GAAGCTGGCG TTCAGGAAGC 50
AGACTTCCTG GCGAACGTGG ATAAACTGTC TGAAGATGCA TTCGATGACC 100
<210> 3736
<211> 100
<212> DNA
<213> Escherichia coli K-12 MG1655
<220> b2248 b2248
<222> 684-783
<223> b2248 b2248 U00096 complement(2359449..2360231)
<313> 3736: 745-764
<400> 3736
AGAATACCGT GATTATCTGG CGGGCAAGGC CATTGCCTGC GCCAGGGATA 50
TTTCACGTCT GTTGGGATGG AAAAGTCCCT TTGACTTACA AGCCTCATAA 100
<210> 3737
<211> 100
<212> DNA
<213> Escherichia coli K-12 MG1655
<220> b2248 b2248
<222> 654-753
<223> b2248 b2248 U00096 complement(2359449..2360231)
<313> 3737: 687-706
<400> 3737

GGTGGTCGGT ACCCGCTTAC AAATCAATGA AGAATACCGT GATTATCTGG 50
CGGGCAAGGC CATTGCCTGC GCCAGGGATA TTTCACGTCT GTTGGGATGG 100
<210> 3738
<211> 100
<212> DNA
<213> Escherichia coli K-12 MG1655
<220> b2248 b2248
<222> 571-670
<223> b2248 b2248 U00096 complement(2359449..2360231)
<313> 3738: 629-648
<400> 3738
GACAACGGCG AAGATTACGC CGATGTCCGT TGTGTCGCTG CGCCGGTGTT 50
TAACGCCAAT AACGAGCTTA CCGCTGCGAT CTCGGTGGTC GGTACCCGCT 100
<210> 3739
<211> 100
<212> DNA
<213> Escherichia coli K-12 MG1655
<220> ybcu b0558
<222> 161-260
<223> ybcu b0558 U00096 complement(577823..578116)
<313> 3739: 204-223
<400> 3739
CAGCTAAAAT TTGTGGCGGC GCAGAAAATG TTGTTAAAAC AGAAACCCAG 50
CAAACATTCG TAAATGGATT GCTCGGTTTT ATTACTTTAG GCATTTATAC 100
<210> 3740
<211> 100
<212> DNA
<213> Escherichia coli K-12 MG1655
<220> ybcu b0558
<222> 115-214
<223> ybcu b0558 U00096 complement(577823..578116)
<313> 3740: 177-196
<400> 3740
CATCATTTCT TCGTTTCTGG AATTGGGCAG AAGAAAACTG TCGATGCAGC 50
TAAAATTTGT GGCGGCGCAG AAAATGTTGT TAAAACAGAA ACCCAGCAAA 100
<210> 3741
<211> 100
<212> DNA
<213> Escherichia coli K-12 MG1655
<220> ybcu b0558
<222> 66-165
<223> ybcu b0558 U00096 complement(577823..578116)
<313> 3741: 112-131
<400> 3741
TACTGTTCAA AACAAACAGA CAGCAGTAGC ACCAAAGGAA ACCATCACCC 50
ATCATTTCTT CGTTTCTGGA ATTGGGCAGA AGAAAACTGT CGATGCAGCT 100
<210> 3742
<211> 100
<212> DNA
<213> Escherichia coli K-12 MG1655
<220> b2249 b2249
<222> 1104-1203
<223> b2249 b2249 U00096 complement(2360451..2361653)
<313> 3742: 1156-1175
<400> 3742
GATGGCACTG AATATGCTGC GCCGTTGGTT AAACGGCCAG GATATCGCCA 50
GTGAGCATGG CTGGATTGAG GTTGTTGAGT CCATGACCTT ATCTGTCTGA 100
<210> 3743
<211> 100
<212> DNA
<213> Escherichia coli K-12 MG1655
<220> b2249 b2249
<222> 1069-1168
<223> b2249 b2249 U00096 complement(2360451..2361653)
<313> 3743: 1112-1131
<400> 3743
TACAGCCTGG CTATCCGTCA GGAAGTGTGC GCAATGATGG CACTGAATAT 50
GCTGCGCCGT TGGTTAAACG GCCAGGATAT CGCCAGTGAG CATGGCTGGA 100
<210> 3744
<211> 100
<212> DNA

<213> Escherichia coli K-12 MG1655
<220> b2249 b2249
<222> 817-916
<223> b2249 b2249 U00096 complement(2360451..2361653)
<313> 3744: 855-874
<400> 3744
AGCGAGCAAT TCACCGGTGG TTTATTGGCT TTGCAACTTT CTCGCGCAGG 50
TGCTCCATTG CTGGCGTGTG AAGTGGTTCC TTCACAGGAG GAAACCCTGG 100
<210> 3745
<211> 100
<212> DNA
<213> Escherichia coli K-12 MG1655
<220> ybcv b0559
<222> 354-453
<223> ybcv b0559 U00096 complement(578407..578859)
<313> 3745: 429-448
<400> 3745
TACAAATATC CACGAACATA AAAGATATTA CTATACCTTT GATAATTCAT 50
TACTATTTAC TGAGAGCATT CAGAACACTA CACAAATCTT TCCACGCTAA 100
<210> 3746
<211> 100
<212> DNA
<213> Escherichia coli K-12 MG1655
<220> ybcv b0559
<222> 312-411
<223> ybcv b0559 U00096 complement(578407..578859)
<313> 3746: 342-361
<400> 3746
ATACAGGGAA AATCTTGCTA AAGCAGGAGT TTTCCGATGG ATTACAAATA 50
TCCACGAACA TAAAAGATAT TACTATACCT TTGATAATTC ATTACTATTT 100
<210> 3747
<211> 100
<212> DNA
<213> Escherichia coli K-12 MG1655
<220> ybcv b0559
<222> 299-398
<223> ybcv b0559 U00096 complement(578407..578859)
<313> 3747: 336-355
<400> 3747
TCACATTTCA GCAATACAGG GAAAATCTTG CTAAAGCAGG AGTTTTCCGA 50
TGGATTACAA ATATCCACGA ACATAAAAGA TATTACTATA CCTTTGATAA 100
<210> 3748
<211> 100
<212> DNA
<213> Escherichia coli K-12 MG1655
<220> yqef yqeF
<222> 1086-1185
<223> yqef yqeF U00096 complement(2982433..2983617)
<313> 3748: 1166-1185
<400> 3748
GGTTCATGAA ATGGTGAAAC GTAATGCCCG CAAAGGACTG GCAACGCTTT 50
GTATCGGCGG GGGCCAGGGT GTGGCATTGA CCATTGAACG TGACGAATAG 100
<210> 3749
<211> 100
<212> DNA
<213> Escherichia coli K-12 MG1655
<220> yqef yqeF
<222> 1060-1159
<223> yqef yqeF U00096 complement(2982433..2983617)
<313> 3749: 1106-1125
<400> 3749
TCCGGTTGCC GAATCCTGGT TTCTCTGGTT CATGAAATGG TGAAACGTAA 50
TGCCCGCAAA GGACTGGCAA CGCTTTGTAT CGGCGGGGGC CAGGGTGTGG 100
<210> 3750
<211> 100
<212> DNA
<213> Escherichia coli K-12 MG1655
<220> yqef yqeF
<222> 966-1065
<223> yqef yqeF U00096 complement(2982433..2983617)
<313> 3750: 1008-1027

<400> 3750
ACAGGCGCTT TCGGTTGGCA AGATGCTTGA GTGGGATGAG CGTCGGGTCA 50
ATGTCAATGG TGGCGCGATC GCACTCGGTC ACCCGATAGG CGCTTCCGGT 100
<210> 3751
<211> 100
<212> DNA
<213> Escherichia coli K-12 MG1655
<220> ybcw b0560
<222> 108-207
<223> ybcw b0560 U00096 579103..579309
<313> 3751: 173-192
<400> 3751
TATAGAACAT AAGGTGTCTC TGGAAGCATT CAGGGCAATT GAGGCAGCGT 50
TGGTGAAGCA CGATAATAAT ATGAAGGATT ATTCCCTGGT GGTTGACTGA 100
<210> 3752
<211> 100
<212> DNA
<213> Escherichia coli K-12 MG1655
<220> ybcw b0560
<222> 108-207
<223> ybcw b0560 U00096 579103..579309
<313> 3752: 161-180
<400> 3752
TATAGAACAT AAGGTGTCTC TGGAAGCATT CAGGGCAATT GAGGCAGCGT 50
TGGTGAAGCA CGATAATAAT ATGAAGGATT ATTCCCTGGT GGTTGACTGA 100
<210> 3753
<211> 100
<212> DNA
<213> Escherichia coli K-12 MG1655
<220> ybcw b0560
<222> 21-120
<223> ybcw b0560 U00096 579103..579309
<313> 3753: 73-92
<400> 3753
TGATGATCCC TCCGTGGATC TGATTCGTGT AAAAAATATG CTTAATAGCA 50
CCATTTCTAT GAGTTACCCT GATGTTGTAA TTGCATGTAT AGAACATAAG 100
<210> 3754
<211> 100
<212> DNA
<213> Escherichia coli K-12 MG1655
<220> ybcx b0562
<222> 645-744
<223> ybcx b0562 U00096 580577..581320
<313> 3754: 708-727
<400> 3754
ATTGCTGGAA GCCTGGAAAA AATATCGGGT GTTGCTGAAC CGTGTTGATA 50
CATCAACTGC ACCTGATATT GAGTGGCCTA CGAACCCTGT CAGGGAGTAA 100
<210> 3755
<211> 100
<212> DNA
<213> Escherichia coli K-12 MG1655
<220> ybcx b0562
<222> 638-737
<223> ybcx b0562 U00096 580577..581320
<313> 3755: 664-683
<400> 3755
AAACCTCATT GCTGGAAGCC TGGAAAAAAT ATCGGGTGTT GCTGAACCGT 50
GTTGATACAT CAACTGCACC TGATATTGAG TGGCCTACGA ACCCTGTCAG 100
<210> 3756
<211> 100
<212> DNA
<213> Escherichia coli K-12 MG1655
<220> ybcx b0562
<222> 594-693
<223> ybcx b0562 U00096 580577..581320
<313> 3756: 626-645
<400> 3756
GCCACTTCAG GATGCTGTAG ATCTGGAAAT CGCAACGGAG GAAGAAACCT 50
CATTGCTGGA AGCCTGGAAA AAATATCGGG TGTTGCTGAA CCGTGTTGAT 100
<210> 3757
<211> 100

<212> DNA
<213> Escherichia coli K-12 MG1655
<220> yqeh b2847
<222> 580-679
<223> yqeh b2847 U00096 2985498..2986190
<313> 3757: 622-641
<400> 3757
GAAGTTCTGG ATATGGATAT CCGTAGCATC TACGCGTACA AGCAACGAAT 50
CGAAAAGAGA ATGGGTGGTA AAATAAACGA ATTATTTATT CGTTCACATT 100
<210> 3758
<211> 100
<212> DNA
<213> Escherichia coli K-12 MG1655
<220> yqeh b2847
<222> 306-405
<223> yqeh b2847 U00096 2985498..2986190
<313> 3758: 355-374
<400> 3758
AAAAGACTGG ATTGACTATC TTGCAAGTAC TGACATGGGG ATTGTTTTAG 50
TCAGCGACCG CAATATGCAA TCGTTAGCTA ATTACTGGCG AAAACACAAC 100
<210> 3759
<211> 100
<212> DNA
<213> Escherichia coli K-12 MG1655
<220> yqeh b2847
<222> 118-217
<223> yqeh b2847 U00096 2985498..2986190
<313> 3759: 159-178
<400> 3759
CAAATAATGC CAGAAGAGTC ACCGCGTTTG CAGTATTGCG CAAACTCGTG 50
TTTTTGTATG TGGCCCGAAG AAAGTTCATA TTTTAATCGT GGTGTGGTAG 100
<210> 3760
<211> 100
<212> DNA
<213> Escherichia coli K-12 MG1655
<220> ybcy b0563
<222> 169-268
<223> ybcy b0563 U00096 complement(581375..581806)
<313> 3760: 231-250
<400> 3760
TCTACAAAAA GCTGTGTAAT ACGCAATGCG GCGCAGGCCT TAACTGACGA 50
TGGAACTCTA TACGGAGCCA CAATTCTTGG CGATGGAGTT GTGCACAATA 100
<210> 3761
<211> 100
<212> DNA
<213> Escherichia coli K-12 MG1655
<220> ybcy b0563
<222> 124-223
<223> ybcy b0563 U00096 complement(581375..581806)
<313> 3761: 176-195
<400> 3761
TCCATTTCCA TGTCTTACCT TCTTCACTGC CTGCCTGGAA ATATATCTAC 50
AAAAAGCTGT GTAATACGCA ATGCGGCGCA GGCCTTAACT GACGATGGAA 100
<210> 3762
<211> 100
<212> DNA
<213> Escherichia coli K-12 MG1655
<220> ybcy b0563
<222> 107-206
<223> ybcy b0563 U00096 complement(581375..581806)
<313> 3762: 140-159
<400> 3762
TACATGGTCA ATTTGATTCC ATTTCCATGT CTTACCTTCT TCACTGCCTG 50
CCTGGAAATA TATCTACAAA AAGCTGTGTA ATACGCAATG CGGCGCAGGC 100
<210> 3763
<211> 100
<212> DNA
<213> Escherichia coli K-12 MG1655
<220> yqei b2848
<222> 711-810
<223> yqei b2848 U00096 2986524..2987333

<313> 3763: 761-780
<400> 3763
CTCACCGGGG ATGATTGTTA TGGCATGCAA TAAAAACTAT AACCAACATG 50
AAGTGGCAAA CTGTTTAACC CTGTCTTACC GTGAGGTTAA TCGTGATTGA 100
<210> 3764
<211> 100
<212> DNA
<213> Escherichia coli K-12 MG1655
<220> yqei b2848
<222> 664-763
<223> yqei b2848 U00096 2986524..2987333
<313> 3764: 697-716
<400> 3764
ATTAACTGTA AAAAACATCC GTGGTTATAC TTCCCTCTTG CAAAATCCTC 50
ACCGGGGATG ATTGTTATGG CATGCAATAA AAACTATAAC CAACATGAAG 100
<210> 3765
<211> 100
<212> DNA
<213> Escherichia coli K-12 MG1655
<220> yqei b2848
<222> 492-591
<223> yqei b2848 U00096 2986524..2987333
<313> 3765: 537-556
<400> 3765
CATAGGCGCA TATTCTGCAT ATTGGCTATG GAATAATAAT CAACCGAAGC 50
CATTTTTCAA GGATTACAAG ACTGTTGCGG AAATTAATGG CTGCCATTTT 100
<210> 3766
<211> 100
<212> DNA
<213> Escherichia coli K-12 MG1655
<220> ybcz b0571
<222> 1344-1443
<223> ybcz b0571 U00096 complement(592551..593993)
<313> 3766: 1408-1427
<400> 3766
CCTGGCGATA GTGAAATCGA TTGTTGTCGC GCATAAAGGC ACGGTTGCGG 50
TAACGTCGGA TGCGCGGGGG ACAAGGTTTG TTATCACATT ACCCGCTTAA 100
<210> 3767
<211> 100
<212> DNA
<213> Escherichia coli K-12 MG1655
<220> ybcz b0571
<222> 1286-1385
<223> ybcz b0571 U00096 complement(592551..593993)
<313> 3767: 1329-1348
<400> 3767
ACCGTTTCTA TCGCGTTGAC CCCTCCCGCC AGCGAAAAGG TGAAGGTAGC 50
GGTATTGGCC TGGCGATAGT GAAATCGATT GTTGTCGCGC ATAAAGGCAC 100
<210> 3768
<211> 100
<212> DNA
<213> Escherichia coli K-12 MG1655
<220> ybcz b0571
<222> 1254-1353
<223> ybcz b0571 U00096 complement(592551..593993)
<313> 3768: 1287-1306
<400> 3768
CATTGCGCCC GAGCACTTAC CGCGATTGTT TGACCGTTTC TATCGCGTTG 50
ACCCCTCCCG CCAGCGAAAA GGTGAAGGTA GCGGTATTGG CCTGGCGATA 100
<210> 3769
<211> 100
<212> DNA
<213> Escherichia coli K-12 MG1655
<220> yqej b2849
<222> 327-426
<223> yqej b2849 U00096 2987314..2987808
<313> 3769: 383-402
<400> 3769
TAATAAGTTT GAGATTATGG ATCAAGTTGA TGATGATAGA CTTGCACAAC 50
TTATTCCTGA TTTTTATGTC TTTCCAGAAA AAAGTGTAAG CTATAATATA 100
<210> 3770

<211> 100
<212> DNA
<213> Escherichia coli K-12 MG1655
<220> yqej b2849
<222> 327-426
<223> yqej b2849 U00096 2987314..2987808
<313> 3770: 359-378
<400> 3770
TAATAAGTTT GAGATTATGG ATCAAGTTGA TGATGATAGA CTTGCACAAC 50
TTATTCCTGA TTTTTATGTC TTTCCAGAAA AAAGTGTAAG CTATAATATA 100
<210> 3771
<211> 100
<212> DNA
<213> Escherichia coli K-12 MG1655
<220> yqej b2849
<222> 321-420
<223> yqej b2849 U00096 2987314..2987808
<313> 3771: 341-360
<400> 3771
GAAAGTTAAT AAGTTTGAGA TTATGGATCA AGTTGATGAT GATAGACTTG 50
CACAACTTAT TCCTGATTTT TATGTCTTTC CAGAAAAAAG TGTAAGCTAT 100
<210> 3772
<211> 100
<212> DNA
<213> Escherichia coli K-12 MG1655
<220> yqek b2850
<222> 333-432
<223> yqek b2850 U00096 complement(2987957..2988394)
<313> 3772: 388-407
<400> 3772
TAATAATCCT CTTTCTGTAT TGTTTACATC GACTGTACGA AGGAATAATA 50
ATCGAGCTAA GATGAATGTC GAGTTCGACT CATGGATTTG CACCAGATGT 100
<210> 3773
<211> 100
<212> DNA
<213> Escherichia coli K-12 MG1655
<220> yqek b2850
<222> 313-412
<223> yqek b2850 U00096 complement(2987957..2988394)
<313> 3773: 364-383
<400> 3773
TCACTGAGTA AACTGGTTCT TAATAATCCT CTTTCTGTAT TGTTTACATC 50
GACTGTACGA AGGAATAATA ATCGAGCTAA GATGAATGTC GAGTTCGACT 100
<210> 3774
<211> 100
<212> DNA
<213> Escherichia coli K-12 MG1655
<220> yqek b2850
<222> 302-401
<223> yqek b2850 U00096 complement(2987957..2988394)
<313> 3774: 352-371
<400> 3774
CCTCTATTGA TTCACTGAGT AAACTGGTTC TTAATAATCC TCTTTCTGTA 50
TTGTTTACAT CGACTGTACG AAGGAATAAT AATCGAGCTA AGATGAATGT 100
<210> 3775
<211> 100
<212> DNA
<213> Escherichia coli K-12 MG1655
<220> flia b1923
<222> 621-720
<223> flia b1923 U00096 complement(1999093..1999812)
<313> 3775: 689-708
<400> 3775
CAAAGAGATT GGCGCGGTGC TGGAGGTCGG GGAATCGCGG GTCAGTCAGT 50
TACACAGCCA GGCTATTAAA CGGTTACGCA CTAAACTGGG TAAGTTATAA 100
<210> 3776
<211> 100
<212> DNA
<213> Escherichia coli K-12 MG1655
<220> flia b1923
<222> 598-697

<223> flia b1923 U00096 complement(1999093..1999812)
<313> 3776: 640-659
<400> 3776
TATTACCAGG AAGAGCTGAA TCTCAAAGAG ATTGGCGCGG TGCTGGAGGT  50
CGGGGAATCG CGGGTCAGTC AGTTACACAG CCAGGCTATT AAACGGTTAC  100
<210> 3777
<211> 100
<212> DNA
<213> Escherichia coli K-12 MG1655
<220> flia b1923
<222> 548-647
<223> flia b1923 U00096 complement(1999093..1999812)
<313> 3777: 581-600
<400> 3777
TGGAAGCCAT CGAAACGTTG CCGGAGCGCG AAAAACTGGT ATTAACCCTC  50
TATTACCAGG AAGAGCTGAA TCTCAAAGAG ATTGGCGCGG TGCTGGAGGT  100
<210> 3778
<211> 100
<212> DNA
<213> Escherichia coli K-12 MG1655
<220> adhp b1479
<222> 891-990
<223> adhp b1479 U00096 complement(1550852..1551892)
<313> 3778: 904-923
<400> 3778
CCAGGATTTA ACTGAAGCCT TCCAGTTTGC CGCCGAAGGT AAAGTGGTGC  50
CGAAAGTCGC CCTGCGTCCG TTAGCGGACA TCAACACCAT CTTTACTGAG  100
<210> 3779
<211> 100
<212> DNA
<213> Escherichia coli K-12 MG1655
<220> adhp b1479
<222> 810-909
<223> adhp b1479 U00096 complement(1550852..1551892)
<313> 3779: 851-870
<400> 3779
ACCGCCGGAG TCTATGAGCC TGGATATCCC ACGTCTTGTG CTGGATGGTA  50
TTGAAGTGGT CGGTTCGCTG GTCGGCACGC GCCAGGATTT AACTGAAGCC  100
<210> 3780
<211> 100
<212> DNA
<213> Escherichia coli K-12 MG1655
<220> adhp b1479
<222> 774-873
<223> adhp b1479 U00096 complement(1550852..1551892)
<313> 3780: 805-824
<400> 3780
TGTCCGTGCA GGCGGTCGTG TTGTGGCTGT CGGTCTACCG CCGGAGTCTA  50
TGAGCCTGGA TATCCCACGT CTTGTGCTGG ATGGTATTGA AGTGGTCGGT  100
<210> 3781
<211> 100
<212> DNA
<213> Escherichia coli K-12 MG1655
<220> flic b1924
<222> 1398-1497
<223> flic b1924 U00096 complement(2000133..2001629)
<313> 3781: 1429-1448
<400> 3781
CAATATGTCG AAAGCGCAGA TCATCCAGCA GGCCGGTAAC TCCGTGTTGG  50
CAAAAGCTAA CCAGGTACCG CAGCAGGTTC TGTCTCTGCT GCAGGGTTAA  100
<210> 3782
<211> 100
<212> DNA
<213> Escherichia coli K-12 MG1655
<220> flic b1924
<222> 1352-1451
<223> flic b1924 U00096 complement(2000133..2001629)
<313> 3782: 1404-1423
<400> 3782
AAGCGCAGTC CCGTATTCAG GACGCCGACT ATGCGACCGA AGTGTCCAAT  50
ATGTCGAAAG CGCAGATCAT CCAGCAGGCC GGTAACTCCG TGTTGGCAAA  100

<210> 3783
<211> 100
<212> DNA
<213> Escherichia coli K-12 MG1655
<220> flic b1924
<222> 1324-1423
<223> flic b1924 U00096 complement(2000133..2001629)
<313> 3783: 1361-1380
<400> 3783
CTGAACAACA CCACTACCAA CCTGTCTGAA GCGCAGTCCC GTATTCAGGA 50
CGCCGACTAT GCGACCGAAG TGTCCAATAT GTCGAAAGCG CAGATCATCC 100
<210> 3784
<211> 100
<212> DNA
<213> Escherichia coli K-12 MG1655
<220> hfq b4173
<222> 210-309
<223> hfq b4173 U00096 4397866..4398174
<313> 3784: 272-291
<400> 3784
TCACAGTAAC AACGCCGGTG GCGGTACCAG CAGTAACTAC CATCATGGTA 50
GCAGCGCGCA GAATACTTCC GCGCAACAGG ACAGCGAAGA AACCGAATAA 100
<210> 3785
<211> 100
<212> DNA
<213> Escherichia coli K-12 MG1655
<220> hfq b4173
<222> 168-267
<223> hfq b4173 U00096 4397866..4398174
<313> 3785: 194-213
<400> 3785
GCACGCGATT TCTACTGTTG TCCCGTCTCG CCCGGTTTCT CATCACAGTA 50
ACAACGCCGG TGGCGGTACC AGCAGTAACT ACCATCATGG TAGCAGCGCG 100
<210> 3786
<211> 100
<212> DNA
<213> Escherichia coli K-12 MG1655
<220> hfq b4173
<222> 96-195
<223> hfq b4173 U00096 4397866..4398174
<313> 3786: 153-172
<400> 3786
GCAAGGGCAA ATCGAGTCTT TTGATCAGTT CGTGATCCTG TTGAAAAACA 50
CGGTCAGCCA GATGGTTTAC AAGCACGCGA TTTCTACTGT TGTCCCGTCT 100
<210> 3787
<211> 100
<212> DNA
<213> Escherichia coli K-12 MG1655
<220> flid b1925
<222> 1308-1407
<223> flid b1925 U00096 2001895..2003301
<313> 3787: 1367-1386
<400> 3787
CAAAGAACAA TTTACCCAAC TGGACGTTTT AATGACCTCG TTAAACAGCA 50
CCAGCAGCTA CTTAACGCAG CAGTTCGAAA ACAACAGTAA TTCCAAGTAA 100
<210> 3788
<211> 100
<212> DNA
<213> Escherichia coli K-12 MG1655
<220> flid b1925
<222> 1261-1360
<223> flid b1925 U00096 2001895..2003301
<313> 3788: 1305-1324
<400> 3788
GACTACAACG CCGCCAGCGA TCGCATTGAT GCGCAGGTCG CTCGCTACAA 50
AGAACAATTT ACCCAACTGG ACGTTTTAAT GACCTCGTTA AACAGCACCA 100
<210> 3789
<211> 100
<212> DNA
<213> Escherichia coli K-12 MG1655
<220> flid b1925

<222> 1212-1311
<223> flid b1925 U00096 2001895..2003301
<313> 3789: 1257-1276
<400> 3789
TAAAGCCGCT ACCGATGGCG TTAGTAAGAC CCTGAATAAA TTAACTAAAG  50
ACTACAACGC CGCCAGCGAT CGCATTGATG CGCAGGTCGC TCGCTACAAA  100
<210> 3790
<211> 100
<212> DNA
<213> Escherichia coli K-12 MG1655
<220> ybda b0592
<222> 1152-1251
<223> ybda b0592 U00096 621523..622773
<313> 3790: 1216-1235
<400> 3790
TTTTGGTTTG TTGATTATCG GCGTGTTGTT ATTGCTGGTG CTGGTGGAGT  50
TGCGACATTT TCGCCAGACG CCGCCGCAGG TGACAGCGTC CGACAGTTAA  100
<210> 3791
<211> 100
<212> DNA
<213> Escherichia coli K-12 MG1655
<220> ybda b0592
<222> 1104-1203
<223> ybda b0592 U00096 621523..622773
<313> 3791: 1134-1153
<400> 3791
TGGTTTGGGC GCGATGATGA CACCGGTTGC TTCCGCAAGC GCGAGCGGTT  50
TTGGTTTGTT GATTATCGGC GTGTTGTTAT TGCTGGTGCT GGTGGAGTTG  100
<210> 3792
<211> 100
<212> DNA
<213> Escherichia coli K-12 MG1655
<220> ybda b0592
<222> 1009-1108
<223> ybda b0592 U00096 621523..622773
<313> 3792: 1066-1085
<400> 3792
CAAACGCAAA CCCCGGAAGC GATGTTAGGG CGGATTAACG GTTTGTGGAC  50
GGCGCAGAAC GTGACGGGCG ATGCCATAGG CGCGGCGCTG CTGGGTGGTT  100
<210> 3793
<211> 100
<212> DNA
<213> Escherichia coli K-12 MG1655
<220> flie b1938
<222> 216-315
<223> flie b1938 U00096 complement(2010722..2011036)
<313> 3793: 277-296
<400> 3793
GACCGATATG CAAAAAGCCT CAGTTTCTAT GCAAATGGGG ATTCAGGTGC  50
GTAATAAGCT GGTGGCGGCG TATCAGGAAG TGATGAGCAT GCAGGTGTAG  100
<210> 3794
<211> 100
<212> DNA
<213> Escherichia coli K-12 MG1655
<220> flie b1938
<222> 172-271
<223> flie b1938 U00096 complement(2010722..2011036)
<313> 3794: 204-223
<400> 3794
AAATTCACTC TCGGTGAACC CGGCGTGGCG TTAAACGATG TGATGACCGA  50
TATGCAAAAA GCCTCAGTTT CTATGCAAAT GGGGATTCAG GTGCGTAATA  100
<210> 3795
<211> 100
<212> DNA
<213> Escherichia coli K-12 MG1655
<220> flie b1938
<222> 116-215
<223> flie b1938 U00096 complement(2010722..2011036)
<313> 3795: 162-181
<400> 3795
ACGCCGCGCT CGATCGCATT AGTGATACAC AAACAGCTGC CCGCACGCAG  50

GCAGAAAAAT TCACTCTCGG TGAACCCGGC GTGGCGTTAA ACGATGTGAT 100
<210> 3796
<211> 100
<212> DNA
<213> Escherichia coli K-12 MG1655
<220> ybdb b0598
<222> 292-391
<223> ybdb b0598 U00096 628523..628936
<313> 3796: 333-352
<400> 3796
GTACGCGGCG TCTGCCAGCC GCTGCATCTT GGTCGGCAAA ATCAGAGCTG 50
GGAAATCGTC GTTTTCGATG AACAGGGGCG GCGTTGCTGC ACTTGTCGGC 100
<210> 3797
<211> 100
<212> DNA
<213> Escherichia coli K-12 MG1655
<220> ybdb b0598
<222> 230-329
<223> ybdb b0598 U00096 628523..628936
<313> 3797: 287-306
<400> 3797
GACAGTGTGT GGTAGGCACA GAACTTAATG CAACACACCA TCGCCCGGTG 50
TCTGAGGGAA AGGTACGCGG CGTCTGCCAG CCGCTGCATC TTGGTCGGCA 100
<210> 3798
<211> 100
<212> DNA
<213> Escherichia coli K-12 MG1655
<220> ybdb b0598
<222> 198-297
<223> ybdb b0598 U00096 628523..628936
<313> 3798: 238-257
<400> 3798
ATCGATGGCC GGATTTATGA TGACCCGCGA CGGACAGTGT GTGGTAGGCA 50
CAGAACTTAA TGCAACACAC CATCGCCCGG TGTCTGAGGG AAAGGTACGC 100
<210> 3799
<211> 100
<212> DNA
<213> Escherichia coli K-12 MG1655
<220> flif b1939
<222> 1559-1658
<223> flif b1939 U00096 2011251..2012909
<313> 3799: 1571-1590
<400> 3799
GGGCAGAAGT CATGAGCCAG CGTATCCGTG AAATGTCTGA TAACGATCCG 50
CGCGTGGTGG CGCTGGTCAT TCGCCAGTGG ATAAATAACG ATCATGAGTA 100
<210> 3800
<211> 100
<212> DNA
<213> Escherichia coli K-12 MG1655
<220> flif b1939
<222> 1456-1555
<223> flif b1939 U00096 2011251..2012909
<313> 3800: 1517-1536
<400> 3800
GTACAGCAAC AGGCGCAGGC CCGCGAGGAA GTGGAAGATG CGGTGGAAGT 50
CCGCCTGAGC AAAGACGAAC AACTACAACA ACGGCGCGCT AACCAACGTC 100
<210> 3801
<211> 100
<212> DNA
<213> Escherichia coli K-12 MG1655
<220> flif b1939
<222> 1435-1534
<223> flif b1939 U00096 2011251..2012909
<313> 3801: 1458-1477
<400> 3801
CGCGCTGAGG CGATGAAAGC TGTACAGCAA CAGGCGCAGG CCCGCGAGGA 50
AGTGGAAGAT GCGGTGGAAG TCCGCCTGAG CAAAGACGAA CAACTACAAC 100
<210> 3802
<211> 100
<212> DNA
<213> Escherichia coli K-12 MG1655

<220> flig b1940
<222> 897-996
<223> flig b1940 U00096 2012902..2013897
<313> 3802: 965-984
<400> 3802
GCAGGTGGAA AACGAACAGA AAGCGATTCT GCTGATTGTG CGCCGCCTTG  50
CCGAAACTGG CGAGATGGTA ATTGGCAGCG GCGAGGATAC CTATGTCTGA  100
<210> 3803
<211> 100
<212> DNA
<213> Escherichia coli K-12 MG1655
<220> flig b1940
<222> 897-996
<223> flig b1940 U00096 2012902..2013897
<313> 3803: 923-942
<400> 3803
GCAGGTGGAA AACGAACAGA AAGCGATTCT GCTGATTGTG CGCCGCCTTG  50
CCGAAACTGG CGAGATGGTA ATTGGCAGCG GCGAGGATAC CTATGTCTGA  100
<210> 3804
<211> 100
<212> DNA
<213> Escherichia coli K-12 MG1655
<220> flig b1940
<222> 804-903
<223> flig b1940 U00096 2012902..2013897
<313> 3804: 852-871
<400> 3804
GCCACTGCGC GAGAAATTCT TGCGCAATAT GTCGCAGCGT GCCGCCGATA  50
TTCTGCGCGA CGATCTCGCC AACCGTGGTC CGGTGCGTCT GTCGCAGGTG  100
<210> 3805
<211> 100
<212> DNA
<213> Escherichia coli K-12 MG1655
<220> flih b1941
<222> 578-677
<223> flih b1941 U00096 2013869..2014576
<313> 3805: 621-640
<400> 3805
GGCGCTTGCG GGGCGATCCC ACCCTCCATC CTGGCGGCTG TAAAGTCTCC  50
GCCGATGAAG GCGATCTCGA CGCCAGTGTC GCCACTCGCT GGCAAGAACT  100
<210> 3806
<211> 100
<212> DNA
<213> Escherichia coli K-12 MG1655
<220> flih b1941
<222> 506-605
<223> flih b1941 U00096 2013869..2014576
<313> 3806: 553-572
<400> 3806
AGCTGCGCGT GCACCCGGAT GATCTGCAAC GTGTGGATGA TATGCTCGGC  50
GCTACCTTAA GTTTGCATGG CTGGCGCTTG CGGGGCGATC CCACCCTCCA  100
<210> 3807
<211> 100
<212> DNA
<213> Escherichia coli K-12 MG1655
<220> flih b1941
<222> 412-511
<223> flih b1941 U00096 2013869..2014576
<313> 3807: 437-456
<400> 3807
GTCATCGGTC AGACGCCAAC GGTGGATAAC TCGGCACTGA TCAAACAGAT  50
CCAACAGTTG TTGCAGCAAG AACCGTTATT CAGCGGTAAA CCACAGCTGC  100
<210> 3808
<211> 100
<212> DNA
<213> Escherichia coli K-12 MG1655
<220> ybde b0576
<222> 3020-3119
<223> ybde b0576 U00096 597937..601080
<313> 3808: 3064-3083
<400> 3808

AGGTGATGAG CCGGATTGCC GCGCCGATGA TTGGCGGCAT GATCACCGCA 50
CCTTTGCTGT CGCTGTTTAT TATCCCGGCG GCGTATAAGC TGATGTGGCT 100
<210> 3809
<211> 100
<212> DNA
<213> Escherichia coli K-12 MG1655
<220> ybde b0576
<222> 2977-3076
<223> ybde b0576 U00096 597937..601080
<313> 3809: 3026-3045
<400> 3809
GCCGGTCTGC TGCCGATTCT GTGGGGAACG GGGGCTGGTT CAGAGGTGAT 50
GAGCCGGATT GCCGCGCCGA TGATTGGCGG CATGATCACC GCACCTTTGC 100
<210> 3810
<211> 100
<212> DNA
<213> Escherichia coli K-12 MG1655
<220> ybde b0576
<222> 2953-3052
<223> ybde b0576 U00096 597937..601080
<313> 3810: 2972-2991
<400> 3810
GCGATGACGG TGGCGGTGAT TATCGCCGGT CTGCTGCCGA TTCTGTGGGG 50
AACGGGGGCT GGTTCAGAGG TGATGAGCCG GATTGCCGCG CCGATGATTG 100
<210> 3811
<211> 100
<212> DNA
<213> Escherichia coli K-12 MG1655
<220> flii b1942
<222> 1224-1323
<223> flii b1942 U00096 2014576..2015949
<313> 3811: 1249-1268
<400> 3811
CGCGTATGCC AAAGGCAGCG ATCCGATGCT CGATAAAGCC ATCGCCCTGT 50
GGCCGCAGCT GGAGGGCTAT TTGCAACAAG GCATTTTTGA ACGCGCGGAC 100
<210> 3812
<211> 100
<212> DNA
<213> Escherichia coli K-12 MG1655
<220> flii b1942
<222> 1140-1239
<223> flii b1942 U00096 2014576..2015949
<313> 3812: 1195-1214
<400> 3812
CAGTGAGCAA CATTACGCGC GAGTGCGCAC CTTCAAACAG CTGTTGTCGA 50
GTTTTCAGCG TAACCGCGAT CTGGTTAGCG TCGGCGCGTA TGCCAAAGGC 100
<210> 3813
<211> 100
<212> DNA
<213> Escherichia coli K-12 MG1655
<220> flii b1942
<222> 1026-1125
<223> flii b1942 U00096 2014576..2015949
<313> 3813: 1069-1088
<400> 3813
CGCGCGGGCG ATCCTCGACG GTCACATTGT GCTGTCTCGC CGACTGGCGG 50
AAGCCGGGCA CTATCCGGCT ATCGATATTG AAGCGTCGAT CAGCCGCGCA 100
<210> 3814
<211> 100
<212> DNA
<213> Escherichia coli K-12 MG1655
<220> ybdf b0580
<222> 270-369
<223> ybdf b0580 U00096 complement(604741..605109)
<313> 3814: 341-360
<400> 3814
AATCTCCGAA GCGTTACTTC GCGATCTGAT CAACGATTCG TGGAATCTGG 50
TGGTTGATGG TCTGGCTAAA CGCGATCAAA AAAGAGTGCG TCCAGGCTAA 100
<210> 3815
<211> 100
<212> DNA

<213> Escherichia coli K-12 MG1655
<220> ybdf b0580
<222> 250-349
<223> ybdf b0580 U00096 complement(604741..605109)
<313> 3815: 301-320
<400> 3815
TCAGTGTATC CCGGCGAAGA AATCTCCGAA GCGTTACTTC GCGATCTGAT 50
CAACGATTCG TGGAATCTGG TGGTTGATGG TCTGGCTAAA CGCGATCAAA 100
<210> 3816
<211> 100
<212> DNA
<213> Escherichia coli K-12 MG1655
<220> ybdf b0580
<222> 212-311
<223> ybdf b0580 U00096 complement(604741..605109)
<313> 3816: 261-280
<400> 3816
TTAAGCCAGG GTATCACATG AATAAAAAGC ACTGGATTTC AGTGTATCCC 50
GGCGAAGAAA TCTCCGAAGC GTTACTTCGC GATCTGATCA ACGATTCGTG 100
<210> 3817
<211> 100
<212> DNA
<213> Escherichia coli K-12 MG1655
<220> flij b1943
<222> 345-444
<223> flij b1943 U00096 2015968..2016411
<313> 3817: 386-405
<400> 3817
GGAACGGCAA TCCACGGCGG CACTGCTTGC AGAAAACCGC CTCGATCAGA 50
AAAAGATGGA TGAGTTCGCC CAGCGCGCCG CCATGAGGAA ACCTGAATGA 100
<210> 3818
<211> 100
<212> DNA
<213> Escherichia coli K-12 MG1655
<220> flij b1943
<222> 238-337
<223> flij b1943 U00096 2015968..2016411
<313> 3818: 284-303
<400> 3818
ATTACTCAGC ATCGCCAGCA ACTTAATCAG TGGACGCAGA AAGTTGACAT 50
TGCCCTGAAC AGTTGGCGAG AAAAAAAACA ACGTTTGCAG GCCTGGCAGA 100
<210> 3819
<211> 100
<212> DNA
<213> Escherichia coli K-12 MG1655
<220> flij b1943
<222> 221-320
<223> flij b1943 U00096 2015968..2016411
<313> 3819: 244-263
<400> 3819
AGACGCTGGA AAAAGCCATT ACTCAGCATC GCCAGCAACT TAATCAGTGG 50
ACGCAGAAAG TTGACATTGC CCTGAACAGT TGGCGAGAAA AAAAACAACG 100
<210> 3820
<211> 100
<212> DNA
<213> Escherichia coli K-12 MG1655
<220> ybdg b0578
<222> 1149-1248
<223> ybdg b0578 U00096 complement(602639..603886)
<313> 3820: 1197-1216
<400> 3820
CGATCACATA TTTGCGATTG TCGAAGAGTT TGGTCTGCGA CTTCATCAGT 50
CGCCAACCGG CAATGATATT CGCTCTCTGG CGGGTGCATT TAAGCAGTAA 100
<210> 3821
<211> 100
<212> DNA
<213> Escherichia coli K-12 MG1655
<220> ybdg b0578
<222> 1134-1233
<223> ybdg b0578 U00096 complement(602639..603886)
<313> 3821: 1155-1174

<400> 3821
TCAGGCTGAT ATATTCGATC ACATATTTGC GATTGTCGAA GAGTTTGGTC 50
TGCGACTTCA TCAGTCGCCA ACCGGCAATG ATATTCGCTC TCTGGCGGGT 100
<210> 3822
<211> 100
<212> DNA
<213> Escherichia coli K-12 MG1655
<220> ybdg b0578
<222> 1092-1191
<223> ybdg b0578 U00096 complement(602639..603886)
<313> 3822: 1126-1145
<400> 3822
CGCGTTTACT AACACCGTGG TGTGGCTGGA ATATGAAAGC ATTCAGGCTG 50
ATATATTCGA TCACATATTT GCGATTGTCG AAGAGTTTGG TCTGCGACTT 100
<210> 3823
<211> 100
<212> DNA
<213> Escherichia coli K-12 MG1655
<220> flik b1944
<222> 1029-1128
<223> flik b1944 U00096 2016408..2017535
<313> 3823: 1078-1097
<400> 3823
CCATGAACCT CTGGCGGGGG AAGACGACGA TACGCTTCCG GTTCCCGTCT 50
CTTTACAAGG GCGTGTAACA GGCAACAGCG GCGTTGATAT TTTCGCCTAA 100
<210> 3824
<211> 100
<212> DNA
<213> Escherichia coli K-12 MG1655
<220> flik b1944
<222> 1009-1108
<223> flik b1944 U00096 2016408..2017535
<313> 3824: 1049-1068
<400> 3824
CAAAGCCAAC GCACAGCAAA CCATGAACCT CTGGCGGGGG AAGACGACGA 50
TACGCTTCCG GTTCCCGTCT CTTTACAAGG GCGTGTAACA GGCAACAGCG 100
<210> 3825
<211> 100
<212> DNA
<213> Escherichia coli K-12 MG1655
<220> flik b1944
<222> 956-1055
<223> flik b1944 U00096 2016408..2017535
<313> 3825: 1010-1029
<400> 3825
ACATCAGTGG CGAAAGCTTT AGTGGTCAGC AGCAGGCCGC TTCCCAGCAA 50
CAGCAAAGCC AACGCACAGC AAACCATGAA CCTCTGGCGG GGGAAGACGA 100
<210> 3826
<211> 100
<212> DNA
<213> Escherichia coli K-12 MG1655
<220> ybdh b0600
<222> 990-1089
<223> ybdh b0600 U00096 complement(631612..632700)
<313> 3826: 1064-1083
<400> 3826
CACCCTGCGT CCGGTGGAGT CCATTCATTA CCTGCCAGTC ACGCTGACAC 50
CAGATACGTT GCGTGCAGCG TTCAAAAAAG TGGAATCGTT TAAAGCCTGA 100
<210> 3827
<211> 100
<212> DNA
<213> Escherichia coli K-12 MG1655
<220> ybdh b0600
<222> 990-1089
<223> ybdh b0600 U00096 complement(631612..632700)
<313> 3827: 1012-1031
<400> 3827
CACCCTGCGT CCGGTGGAGT CCATTCATTA CCTGCCAGTC ACGCTGACAC 50
CAGATACGTT GCGTGCAGCG TTCAAAAAAG TGGAATCGTT TAAAGCCTGA 100
<210> 3828
<211> 100

<212> DNA
<213> Escherichia coli K-12 MG1655
<220> ybdh b0600
<222> 935-1034
<223> ybdh b0600 U00096 complement(631612..632700)
<313> 3828: 973-992
<400> 3828
CGGAGCTGGA AGTGGATATC AATAATCAGG CGGAGATCGA CAAAGTGATT 50
GCCCACACCC TGCGTCCGGT GGAGTCCATT CATTACCTGC CAGTCACGCT 100
<210> 3829
<211> 100
<212> DNA
<213> Escherichia coli K-12 MG1655
<220> flil b1945
<222> 366-465
<223> flil b1945 U00096 2017640..2018104
<313> 3829: 443-462
<400> 3829
GATTGCCGAG ATTAAAACCA CACTTTCCAC CCCGCTTGTT GCCGGGCAAC 50
CGAAACAGGA TGTCACCGAC GTGCTGTATA CCGCTTTTAT TCTGCGATAA 100
<210> 3830
<211> 100
<212> DNA
<213> Escherichia coli K-12 MG1655
<220> flil b1945
<222> 358-457
<223> flil b1945 U00096 2017640..2018104
<313> 3830: 372-391
<400> 3830
AAAAACCTGA TTGCCGAGAT TAAAACCACA CTTTCCACCC CGCTTGTTGC 50
CGGGCAACCG AAACAGGATG TCACCGACGT GCTGTATACC GCTTTTATTC 100
<210> 3831
<211> 100
<212> DNA
<213> Escherichia coli K-12 MG1655
<220> flil b1945
<222> 312-411
<223> flil b1945 U00096 2017640..2018104
<313> 3831: 343-362
<400> 3831
GTTTTCGCGT CAGGATGCTG CCGTACTGGC GACAGAAGAA GGCAAGAAAA 50
ACCTGATTGC CGAGATTAAA ACCACACTTT CCACCCCGCT TGTTGCCGGG 100
<210> 3832
<211> 100
<212> DNA
<213> Escherichia coli K-12 MG1655
<220> flim b1946
<222> 886-985
<223> flim b1946 U00096 2018109..2019113
<313> 3832: 937-956
<400> 3832
GTTGACGGCG TCCCGGTGCT GACCAGTCAG TATGGCACCC TCAACGGTCA 50
GTATGCGTTA CGGATAGAAC ATTTGATTAA CCCGATTTTA AATTCTCTGA 100
<210> 3833
<211> 100
<212> DNA
<213> Escherichia coli K-12 MG1655
<220> flim b1946
<222> 768-867
<223> flim b1946 U00096 2018109..2019113
<313> 3833: 797-816
<400> 3833
TTCACAGCTG GAGCTGGTCG CCAACTTTGC CGATATCTCG CTACGCCTGT 50
CGCAGATTTT AAAACTGAAC CCCGGCGACG TCCTGCCGAT AGAAAAACCC 100
<210> 3834
<211> 100
<212> DNA
<213> Escherichia coli K-12 MG1655
<220> flim b1946
<222> 654-753
<223> flim b1946 U00096 2018109..2019113

<313> 3834: 700-719
<400> 3834
CCTGCCATTC AGCATGATCG AGCCGCTACG GGAATTGTTG GTTAACCCGC 50
CGCTGGAAAA CTCGCGTAAT GAAGATCAGA ACTGGCGCGA TAACCTGGTG 100
<210> 3835
<211> 100
<212> DNA
<213> Escherichia coli K-12 MG1655
<220> ybdj b0581
<222> 150-249
<223> ybdj b0581 U00096 complement(605174..605422)
<313> 3835: 203-222
<400> 3835
TTACACTTTA TTGTTTTGTC TGTGGTTTTT AGTGCTGGGC GCTATTGAGT 50
ATTTTGTTCT GCGCTTTATC TGGCGACGCT GGTTCTCGCT GGCGGATTAA 100
<210> 3836
<211> 100
<212> DNA
<213> Escherichia coli K-12 MG1655
<220> ybdj b0581
<222> 21-120
<223> ybdj b0581 U00096 complement(605174..605422)
<313> 3836: 56-75
<400> 3836
CTTGACCACC GCAGCAGGCA TTTTGCTGAT GGCTTTCCTC TCTTGCCTGC 50
TGCTGCCCGC CCCCGCACTG GGGCTGGCGC TGGCACAAAA ACTGGTGACC 100
<210> 3837
<211> 100
<212> DNA
<213> Escherichia coli K-12 MG1655
<220> ybdj b0581
<222> 1-100
<223> ybdj b0581 U00096 complement(605174..605422)
<313> 3837: 14-33
<400> 3837
ATGAAACACC CTTTAGAAAC CTTGACCACC GCAGCAGGCA TTTTGCTGAT 50
GGCTTTCCTC TCTTGCCTGC TGCTGCCCGC CCCCGCACTG GGGCTGGCGC 100
<210> 3838
<211> 100
<212> DNA
<213> Escherichia coli K-12 MG1655
<220> flin b1947
<222> 315-414
<223> flin b1947 U00096 2019110..2019523
<313> 3838: 391-410
<400> 3838
AATCGCCCAG GGCGAAGTGG TGGTCGTTGC CGATAAATAT GGCGTGCGGA 50
TCACCGATAT CATTACTCCG TCTGAGCGAA TGCGCCGCCT GAGCCGTTAG 100
<210> 3839
<211> 100
<212> DNA
<213> Escherichia coli K-12 MG1655
<220> flin b1947
<222> 289-388
<223> flin b1947 U00096 2019110..2019523
<313> 3839: 350-369
<400> 3839
CTGGATATTC TGATCAACGG TTATTTAATC GCCCAGGGCG AAGTGGTGGT 50
CGTTGCCGAT AAATATGGCG TGCGGATCAC CGATATCATT ACTCCGTCTG 100
<210> 3840
<211> 100
<212> DNA
<213> Escherichia coli K-12 MG1655
<220> flin b1947
<222> 126-225
<223> flin b1947 U00096 2019110..2019523
<313> 3840: 145-164
<400> 3840
TGGCGGTGGT GATGTCAGCG GAACGTTGCA GGATATCGAC CTGATTATGG 50
ATATTCCGGT CAAGCTGACC GTCGAGCTGG GCCGTACGCG GATGACCATC 100
<210> 3841

<211> 100
<212> DNA
<213> Escherichia coli K-12 MG1655
<220> ybdk b0582
<222> 1020-1119
<223> ybdk b0582 U00096 complement(605488..606606)
<313> 3841: 1096-1115
<400> 3841
CGGTCTGAAT GAAGCGCAGC TAATGCGCGA TTTCGTCGCC GATGGCGGCT 50
CGCTGATTGG GCTGGTGAAA AAGCATTGTG AGATCTGGGC CGGTGACTAA 100
<210> 3842
<211> 100
<212> DNA
<213> Escherichia coli K-12 MG1655
<220> ybdk b0582
<222> 1011-1110
<223> ybdk b0582 U00096 complement(605488..606606)
<313> 3842: 1035-1054
<400> 3842
GGTCGTCAGC GGTCTGAATG AAGCGCAGCT AATGCGCGAT TTCGTCGCCG 50
ATGGCGGCTC GCTGATTGGG CTGGTGAAAA AGCATTGTGA GATCTGGGCC 100
<210> 3843
<211> 100
<212> DNA
<213> Escherichia coli K-12 MG1655
<220> ybdk b0582
<222> 932-1031
<223> ybdk b0582 U00096 complement(605488..606606)
<313> 3843: 998-1017
<400> 3843
CCTTGCGATT GCTGGAAAAA ATCGCCCCTT CCGCACATAA AATTGGTGCA 50
TCGAGCGCGA TTGAGGCCCT GCATCGCCAG GTCGTCAGCG GTCTGAATGA 100
<210> 3844
<211> 100
<212> DNA
<213> Escherichia coli K-12 MG1655
<220> b1520 b1521
<222> 867-966
<223> b1520 b1521 U00096 complement(1606132..1607097)
<313> 3844: 918-937
<400> 3844
TGCCATCTGC AACGCTATTG AAATTGACCT GTTACAGATG AACGATGAAG 50
CCGAAATTCC AGCAAAAATT CTTCCCGATC GTCATTACCA GCTGACGTGA 100
<210> 3845
<211> 100
<212> DNA
<213> Escherichia coli K-12 MG1655
<220> b1520 b1521
<222> 825-924
<223> b1520 b1521 U00096 complement(1606132..1607097)
<313> 3845: 883-902
<400> 3845
GGAAGATCCG TTCGGTACTG AAAACAATGA TTTACCACTG GATGCCATCT 50
GCAACGCTAT TGAAATTGAC CTGTTACAGA TGAACGATGA AGCCGAAATT 100
<210> 3846
<211> 100
<212> DNA
<213> Escherichia coli K-12 MG1655
<220> b1520 b1521
<222> 785-884
<223> b1520 b1521 U00096 complement(1606132..1607097)
<313> 3846: 821-840
<400> 3846
CCTACACTTT TATTTCGCTG GATTGTCTGG CGGAAGAACT GGAAGATCCG 50
TTCGGTACTG AAAACAATGA TTTACCACTG GATGCCATCT GCAACGCTAT 100
<210> 3847
<211> 100
<212> DNA
<213> Escherichia coli K-12 MG1655
<220> flio b1948
<222> 183-282

<223> flio b1948 U00096 2019586..2019891
<313> 3847: 245-264
<400> 3847
CGTTACCGCA GGTCAAATCA ATCTGCTGCA TAAACTTCCC CCTTCTGCAC 50
CAACGGAAGA GATACCGCAG ACCGATTTTC AGTCGGTCAT GAAAAATTTG 100
<210> 3848
<211> 100
<212> DNA
<213> Escherichia coli K-12 MG1655
<220> flio b1948
<222> 142-241
<223> flio b1948 U00096 2019586..2019891
<313> 3848: 193-212
<400> 3848
GTTGTGGTGG TCGATGTGGA AGATGCACGG CTGGTGCTCG GCGTTACCGC 50
AGGTCAAATC AATCTGCTGC ATAAACTTCC CCCTTCTGCA CCAACGGAAG 100
<210> 3849
<211> 100
<212> DNA
<213> Escherichia coli K-12 MG1655
<220> flio b1948
<222> 100-199
<223> flio b1948 U00096 2019586..2019891
<313> 3849: 158-177
<400> 3849
CTGAAAATTA GCGCCAGTGC TTCACTGGGC GCGCGTGAAA GGGTTGTGGT 50
GGTCGATGTG GAAGATGCAC GGCTGGTGCT CGGCGTTACC GCAGGTCAAA 100
<210> 3850
<211> 100
<212> DNA
<213> Escherichia coli K-12 MG1655
<220> ybdl b0601
<222> 1049-1148
<223> ybdl b0601 U00096 632809..633969
<313> 3850: 1090-1109
<400> 3850
TTCCGCTGTC GGTGTTTTGC GCCGATCCCT TCCCACATAA ACTGATTCGT 50
CTCTGTTTTG CCAAGAAGGA ATCGACGTTG CTGGCAGCAG CTGAACGCCT 100
<210> 3851
<211> 100
<212> DNA
<213> Escherichia coli K-12 MG1655
<220> ybdl b0601
<222> 970-1069
<223> ybdl b0601 U00096 632809..633969
<313> 3851: 1014-1033
<400> 3851
GTGGATTACA GCGCGGTTTC TACCCTGGAT GATGTTGAGT TTTGCCAGTG 50
GCTGACGCAG GAGCACGGCG TAGCGGCGAT TCCGCTGTCG GTGTTTTGCG 100
<210> 3852
<211> 100
<212> DNA
<213> Escherichia coli K-12 MG1655
<220> ybdl b0601
<222> 936-1035
<223> ybdl b0601 U00096 632809..633969
<313> 3852: 985-1004
<400> 3852
GATTTTACCG TGTGAAGGTA CATACTTTTT GCTGGTGGAT TACAGCGCGG 50
TTTCTACCCT GGATGATGTT GAGTTTTGCC AGTGGCTGAC GCAGGAGCAC 100
<210> 3853
<211> 100
<212> DNA
<213> Escherichia coli K-12 MG1655
<220> taua b0366
<222> 921-1020
<223> taua b0366 U00096 384399..385418
<313> 3853: 988-1007
<400> 3853
AGCGATCATC GACACCGCGC AGTTTTTGAA AGAGCAGGGC AAGGTCCCGG 50
CTGTAGCGAA TGATTACAGC CAGTACGTTA CCTCGCGCTT CGTGCAATAA 100

<210> 3854
<211> 100
<212> DNA
<213> Escherichia coli K-12 MG1655
<220> taua b0366
<222> 883-982
<223> taua b0366 U00096 384399..385418
<313> 3854: 924-943
<400> 3854
CAGCAACAAA CGGCAGAACT GACCGGACCG GTGAACAAAG CGATCATCGA  50
CACCGCGCAG TTTTTGAAAG AGCAGGGCAA GGTCCCGGCT GTAGCGAATG  100
<210> 3855
<211> 100
<212> DNA
<213> Escherichia coli K-12 MG1655
<220> taua b0366
<222> 824-923
<223> taua b0366 U00096 384399..385418
<313> 3855: 887-906
<400> 3855
GCGGCGTGCC TGAAGGTGAC GTTCCGGGGC TGGTGAAGGG GAATACCTAT  50
CTGACGCCGC AGCAACAAAC GGCAGAACTG ACCGGACCGG TGAACAAAGC  100
<210> 3856
<211> 100
<212> DNA
<213> Escherichia coli K-12 MG1655
<220> b2250 b2251
<222> 465-564
<223> b2250 b2251 U00096 complement(2361753..2362316)
<313> 3856: 539-558
<400> 3856
TGAGGCGGGT TATCGCTACC TGAATCTGTC GGGTAAAGAC GGTAACCGCG  50
ACAACGCCGT GGCTGACGGC CCGTATGTTG GGGTTAACGC CAGTTTCTGA  100
<210> 3857
<211> 100
<212> DNA
<213> Escherichia coli K-12 MG1655
<220> b2250 b2251
<222> 442-541
<223> b2250 b2251 U00096 complement(2361753..2362316)
<313> 3857: 487-506
<400> 3857
ACCATTATGC GTCCAGTCAG TATTGAGGCG GGTTATCGCT ACCTGAATCT  50
GTCGGGTAAA GACGGTAACC GCGACAACGC CGTGGCTGAC GGCCCGTATG  100
<210> 3858
<211> 100
<212> DNA
<213> Escherichia coli K-12 MG1655
<220> b2250 b2251
<222> 318-417
<223> b2250 b2251 U00096 complement(2361753..2362316)
<313> 3858: 359-378
<400> 3858
AGGAGGTGGT TTGCAGTGGA AAATTGGCAA CAGCTTCCGT TTGTTTGGCG  50
AGTATTACTA CTCTCCGGAT TCGCTCTCCA GCGGTATTCA AAGTTATGAA  100
<210> 3859
<211> 100
<212> DNA
<213> Escherichia coli K-12 MG1655
<220> flip b1949
<222> 624-723
<223> flip b1949 U00096 2019891..2020628
<313> 3859: 672-691
<400> 3859
GATGATGATG GTTCCCCCAG CCACCATTGC TCTGCCCTTT AAACTGATGC  50
TGTTTGTACT GGTGGATGGC TGGCAATTGC TGGTCGGTTC GCTGGCGCAG  100
<210> 3860
<211> 100
<212> DNA
<213> Escherichia coli K-12 MG1655
<220> flip b1949

<222> 604-703
<223> flip b1949 U00096 2019891..2020628
<313> 3860: 639-658
<400> 3860
AGCGTGTTGA TGGCATTGGG GATGATGATG GTTCCCCCAG CCACCATTGC 50
TCTGCCCTTT AAACTGATGC TGTTTGTACT GGTGGATGGC TGGCAATTGC 100
<210> 3861
<211> 100
<212> DNA
<213> Escherichia coli K-12 MG1655
<220> flip b1949
<222> 533-632
<223> flip b1949 U00096 2019891..2020628
<313> 3861: 590-609
<400> 3861
AGTTGAAAAC CGCATTTCAG ATAGGCTTCA CGATTTTCAT CCCTTTTTTG 50
ATTATCGACC TGGTGATAGC CAGCGTGTTG ATGGCATTGG GGATGATGAT 100
<210> 3862
<211> 100
<212> DNA
<213> Escherichia coli K-12 MG1655
<220> ybdm b0602
<222> 531-630
<223> ybdm b0602 U00096 complement(633970..634599)
<313> 3862: 583-602
<400> 3862
GGGAATGGAC AGCGACGAAG TGCTGCGCCT GAAGCAAATT AACGGCCTGC 50
AAGAGTTGTT TGCCGACCGT CAATATTCCC GCGCCTGGAC GGTTAAATAG 100
<210> 3863
<211> 100
<212> DNA
<213> Escherichia coli K-12 MG1655
<220> ybdm b0602
<222> 494-593
<223> ybdm b0602 U00096 complement(633970..634599)
<313> 3863: 561-580
<400> 3863
AGTTGGGATG GGACGATAAT AAAATTGGCA AAGAGCTGGG AATGGACAGC 50
GACGAAGTGC TGCGCCTGAA GCAAATTAAC GGCCTGCAAG AGTTGTTTGC 100
<210> 3864
<211> 100
<212> DNA
<213> Escherichia coli K-12 MG1655
<220> ybdm b0602
<222> 452-551
<223> ybdm b0602 U00096 complement(633970..634599)
<313> 3864: 503-522
<400> 3864
ATCAAATCAC CGCCATGTCG GAAATCGTCC GTGAACTTAG CCAGTTGGGA 50
TGGGACGATA ATAAAATTGG CAAAGAGCTG GGAATGGACA GCGACGAAGT 100
<210> 3865
<211> 100
<212> DNA
<213> Escherichia coli K-12 MG1655
<220> taub b0367
<222> 669-768
<223> taub b0367 U00096 385431..386198
<313> 3865: 738-757
<400> 3865
GGGAGAGTCG AGCCGCAGCA TCAAGTCCGA TCCACAATTC ATCGCCATGC 50
GCGAATATGT TTTAAGCCGC GTATTTGAGC AACGGGAGGC GTTCTCATGA 100
<210> 3866
<211> 100
<212> DNA
<213> Escherichia coli K-12 MG1655
<220> taub b0367
<222> 669-768
<223> taub b0367 U00096 385431..386198
<313> 3866: 694-713
<400> 3866
GGGAGAGTCG AGCCGCAGCA TCAAGTCCGA TCCACAATTC ATCGCCATGC 50

GCGAATATGT TTTAAGCCGC GTATTTGAGC AACGGGAGGC GTTCTCATGA 100
<210> 3867
<211> 100
<212> DNA
<213> Escherichia coli K-12 MG1655
<220> taub b0367
<222> 633-732
<223> taub b0367 U00096 385431..386198
<313> 3867: 647-666
<400> 3867
GCGGCTGCCG CTCAACTTTG CTCGCCGCTT TGTTGCGGGA GAGTCGAGCC 50
GCAGCATCAA GTCCGATCCA CAATTCATCG CCATGCGCGA ATATGTTTTA 100
<210> 3868
<211> 100
<212> DNA
<213> Escherichia coli K-12 MG1655
<220> b1522 b1522
<222> 849-948
<223> b1522 b1522 U00096 complement(1608931..1609878)
<313> 3868: 922-941
<400> 3868
TAATAAATTG ATGGTCGAAG CCGATACGTG TCTGTATCGC TCGAAGAAAG 50
ATGGGCGCAA CCGTACCAGC ACTATGCGTT ACGGTGAGGA AGTTGTCTGA 100
<210> 3869
<211> 100
<212> DNA
<213> Escherichia coli K-12 MG1655
<220> b1522 b1522
<222> 784-883
<223> b1522 b1522 U00096 complement(1608931..1609878)
<313> 3869: 848-867
<400> 3869
TATCTCACGG TAAGTATTGG CGTCGGTAGT GGTCGCGCAT CGTACCTAAC 50
GCTGACCGAT GACTTTAATA AATTGATGGT CGAAGCCGAT ACGTGTCTGT 100
<210> 3870
<211> 100
<212> DNA
<213> Escherichia coli K-12 MG1655
<220> b1522 b1522
<222> 743-842
<223> b1522 b1522 U00096 complement(1608931..1609878)
<313> 3870: 762-781
<400> 3870
GCGTTGAACT GCAACCATTC ACCTGGCAAC AAAAAACGCT TTATCTCACG 50
GTAAGTATTG GCGTCGGTAG TGGTCGCGCA TCGTACCTAA CGCTGACCGA 100
<210> 3871
<211> 100
<212> DNA
<213> Escherichia coli K-12 MG1655
<220> fliq b1950
<222> 171-270
<223> fliq b1950 U00096 2020638..2020907
<313> 3871: 230-249
<400> 3871
CGTATTTATC GCCATTATTA TTGCCGGACC GTGGATGCTC AATCTGTTGC 50
TGGATTACGT CCGCACCTTG TTCACTAACC TGCCGTATAT CATCGGGTAG 100
<210> 3872
<211> 100
<212> DNA
<213> Escherichia coli K-12 MG1655
<220> fliq b1950
<222> 171-270
<223> fliq b1950 U00096 2020638..2020907
<313> 3872: 202-221
<400> 3872
CGTATTTATC GCCATTATTA TTGCCGGACC GTGGATGCTC AATCTGTTGC 50
TGGATTACGT CCGCACCTTG TTCACTAACC TGCCGTATAT CATCGGGTAG 100
<210> 3873
<211> 100
<212> DNA
<213> Escherichia coli K-12 MG1655

<220> fliq b1950
<222> 116-215
<223> fliq b1950 U00096 2020638..2020907
<313> 3873: 157-176
<400> 3873
AGGCCGCCAC GCAGATTAAC GAAATGACGC TGTCGTTTAT TCCGAAAATC 50
ATCGCCGTAT TTATCGCCAT TATTATTGCC GGACCGTGGA TGCTCAATCT 100
<210> 3874
<211> 100
<212> DNA
<213> Escherichia coli K-12 MG1655
<220> ybdn b0603
<222> 1122-1221
<223> ybdn b0603 U00096 complement(634572..635792)
<313> 3874: 1182-1201
<400> 3874
ATTATCATTT AGCCCCACAA AATCGAAGAA CTATCAGCGT TATAACGAAC 50
GGATAAAAGG AAAACGTCAG GAATGGGGGA TACTATGCAA CAACGATTAA 100
<210> 3875
<211> 100
<212> DNA
<213> Escherichia coli K-12 MG1655
<220> ybdn b0603
<222> 1079-1178
<223> ybdn b0603 U00096 complement(634572..635792)
<313> 3875: 1125-1144
<400> 3875
GGATATGCAA AGTTTTACTC AATAACGATT ACTGGTGTCG GGCATTATCA 50
TTTAGCCCCA CAAAATCGAA GAACTATCAG CGTTATAACG AACGGATAAA 100
<210> 3876
<211> 100
<212> DNA
<213> Escherichia coli K-12 MG1655
<220> ybdn b0603
<222> 1045-1144
<223> ybdn b0603 U00096 complement(634572..635792)
<313> 3876: 1081-1100
<400> 3876
GACATTGGCG CGAAAGATAT CCCCTCCTGG CGGCGGATAT GCAAAGTTTT 50
ACTCAATAAC GATTACTGGT GTCGGGCATT ATCATTTAGC CCCACAAAAT 100
<210> 3877
<211> 100
<212> DNA
<213> Escherichia coli K-12 MG1655
<220> tauc b0368
<222> 729-828
<223> tauc b0368 U00096 386195..387022
<313> 3877: 792-811
<400> 3877
GGCGGGGATC GCGGTGATTG CGATTATCGC CTTTCTTTTA GAACTGGGTC 50
TGCGCGCGTT ACAGCGCCGC CTGACGCCCT GGCATGGAGA AGTACAATGA 100
<210> 3878
<211> 100
<212> DNA
<213> Escherichia coli K-12 MG1655
<220> tauc b0368
<222> 665-764
<223> tauc b0368 U00096 386195..387022
<313> 3878: 708-727
<400> 3878
CGACGCGCGG TTTAGGATTT ATGGTTCAGT CAGCGGGTGA ATTTCTCGCA 50
ACTGACGTGG TGCTGGCGGG GATCGCGGTG ATTGCGATTA TCGCCTTTCT 100
<210> 3879
<211> 100
<212> DNA
<213> Escherichia coli K-12 MG1655
<220> tauc b0368
<222> 466-565
<223> tauc b0368 U00096 386195..387022
<313> 3879: 508-527
<400> 3879

CCGGTGGCGA TGTCGGCGCT GGCGGGGGTG AAAAGCGTGC AGCAGGTTCG 50
CATTCGTGCC GCCCAGTCGC TGGGTGCCAG CCGTGCGCAG GTGCTGTGGT 100
<210> 3880
<211> 100
<212> DNA
<213> Escherichia coli K-12 MG1655
<220> b1523 b1524
<222> 240-339
<223> b1523 b1524 U00096 complement(1609990..1610349)
<313> 3880: 288-307
<400> 3880
TCGCGGTTGT CTGGCTAAGT GGCATAACAT TCCGCAGGGT GTTTCGTTAA 50
GCGAAGAACA ACAACGTTAT ATCGTGGCGG TAATCTATCA CTGGCTGGTT 100
<210> 3881
<211> 100
<212> DNA
<213> Escherichia coli K-12 MG1655
<220> b1523 b1524
<222> 212-311
<223> b1523 b1524 U00096 complement(1609990..1610349)
<313> 3881: 257-276
<400> 3881
TCGCTCAGCA CGCCACCGCG ACCTGCTGTC GCGGTTGTCT GGCTAAGTGG 50
CATAACATTC CGCAGGGTGT TTCGTTAAGC GAAGAACAAC AACGTTATAT 100
<210> 3882
<211> 100
<212> DNA
<213> Escherichia coli K-12 MG1655
<220> b1523 b1524
<222> 176-275
<223> b1523 b1524 U00096 complement(1609990..1610349)
<313> 3882: 218-237
<400> 3882
GTAAGCAAAC CCCCATGCGC GGGCATCCGG TGTTTATCGC TCAGCACGCC 50
ACCGCGACCT GCTGTCGCGG TTGTCTGGCT AAGTGGCATA ACATTCCGCA 100
<210> 3883
<211> 100
<212> DNA
<213> Escherichia coli K-12 MG1655
<220> flir b1951
<222> 578-677
<223> flir b1951 U00096 2020915..2021700
<313> 3883: 596-615
<400> 3883
TGCTGCTGAC ACTGAATCTG GCATTAGGTT TACTTAATCG TATGGCCCCG 50
CAATTATCCA TTTTTGTTAT TGGATTTCCA TTAACTCTGA CTGTCGGCAT 100
<210> 3884
<211> 100
<212> DNA
<213> Escherichia coli K-12 MG1655
<220> flir b1951
<222> 483-582
<223> flir b1951 U00096 2020915..2021700
<313> 3884: 535-554
<400> 3884
CGAACCGTTG AACAGCAATG CGTTTCTGGC ACTCACCAAA GCAGGGAGTT 50
TGATTTTCCT TAACGGGCTG ATGCTGGCGT TACCGCTCAT TACTCTGCTG 100
<210> 3885
<211> 100
<212> DNA
<213> Escherichia coli K-12 MG1655
<220> flir b1951
<222> 388-487
<223> flir b1951 U00096 2020915..2021700
<313> 3885: 450-469
<400> 3885
GATATGCTGG CGTTACTGCT GTTCCTGACA TTTAACGGTC ATTTATGGTT 50
GATTTCACTG CTGGTCGATA CCTTTCACAC CCTGCCGATT GGTGGCGAAC 100
<210> 3886
<211> 100
<212> DNA

<213> Escherichia coli K-12 MG1655
<220> ybdo b0604
<222> 374-473
<223> ybdo b0604 U00096 complement(635939..636841)
<313> 3886: 433-452
<400> 3886
CTGTCGAGAT TGAGTGCCAT GATATCCTCA TGTCAGCAGA GAATGCTGAA 50
GAACTGTTGG TTCACCGCAA AGCCGATCTG GTGATCACCC AAATGCCAGT 100
<210> 3887
<211> 100
<212> DNA
<213> Escherichia coli K-12 MG1655
<220> ybdo b0604
<222> 364-463
<223> ybdo b0604 U00096 complement(635939..636841)
<313> 3887: 388-407
<400> 3887
CAAGATTCCT CTGTCGAGAT TGAGTGCCAT GATATCCTCA TGTCAGCAGA 50
GAATGCTGAA GAACTGTTGG TTCACCGCAA AGCCGATCTG GTGATCACCC 100
<210> 3888
<211> 100
<212> DNA
<213> Escherichia coli K-12 MG1655
<220> ybdo b0604
<222> 148-247
<223> ybdo b0604 U00096 complement(635939..636841)
<313> 3888: 202-221
<400> 3888
GCGCAATTTA ATGACCCTTT ATTCATTCGC TCAGGAAAAG GTATCGCGCC 50
GACGACAACA GGGTTAAATC TGCATCACCA TCTTGAGAAA AATCTCAGAG 100
<210> 3889
<211> 100
<212> DNA
<213> Escherichia coli K-12 MG1655
<220> taud b0369
<222> 753-852
<223> taud b0369 U00096 387019..387870
<313> 3889: 790-809
<400> 3889
CGTGACCCAG CACTATGCCA ATGCCGATTA CCTGCCACAG CGACGGATAA 50
TGCATCGGGC GACGATCCTT GGGGATAAAC CGTTTTATCG GGCGGGGTAA 100
<210> 3890
<211> 100
<212> DNA
<213> Escherichia coli K-12 MG1655
<220> taud b0369
<222> 726-825
<223> taud b0369 U00096 387019..387870
<313> 3890: 750-769
<400> 3890
AAATGATATT GCGATTTGGG ATAACCGCGT GACCCAGCAC TATGCCAATG 50
CCGATTACCT GCCACAGCGA CGGATAATGC ATCGGGCGAC GATCCTTGGG 100
<210> 3891
<211> 100
<212> DNA
<213> Escherichia coli K-12 MG1655
<220> taud b0369
<222> 631-730
<223> taud b0369 U00096 387019..387870
<313> 3891: 687-706
<400> 3891
GTTGATGTGA GCGAGAAAGA GAGCGAAGCC TTGTTAAGTT TTTTGTTTGC 50
CCATATCACC AAACCGGAGT TTCAGGTGCG CTGGCGCTGG CAACCAAATG 100
<210> 3892
<211> 100
<212> DNA
<213> Escherichia coli K-12 MG1655
<220> b2253 b2253
<222> 1074-1173
<223> b2253 b2253 U00096 2363915..2365087
<313> 3892: 1140-1159

<400> 3892
GAATAGCGAA CGCATCTGTT CTTTGCCGCT GTTCCCGGAT ATGACTACCG 50
CCGATGCCGA CCATGTCATC ACAGCCCTTC AGCAACTCGC AGGACAATAA 100
<210> 3893
<211> 100
<212> DNA
<213> Escherichia coli K-12 MG1655
<220> b2253 b2253
<222> 1069-1168
<223> b2253 b2253 U00096 2363915..2365087
<313> 3893: 1102-1121
<400> 3893
GAATGGAATA GCGAACGCAT CTGTTCTTTG CCGCTGTTCC CGGATATGAC 50
TACCGCCGAT GCCGACCATG TCATCACAGC CCTTCAGCAA CTCGCAGGAC 100
<210> 3894
<211> 100
<212> DNA
<213> Escherichia coli K-12 MG1655
<220> b2253 b2253
<222> 1006-1105
<223> b2253 b2253 U00096 2363915..2365087
<313> 3894: 1058-1077
<400> 3894
CGCGCCGCTC ACACACAAAA ATATTATCGC GAGCGTTTTC CCACGCTGTC 50
GTTACCGAAT ACCGAATGGA ATAGCGAACG CATCTGTTCT TTGCCGCTGT 100
<210> 3895
<211> 100
<212> DNA
<213> Escherichia coli K-12 MG1655
<220> flis b1926
<222> 312-411
<223> flis b1926 U00096 2003326..2003736
<313> 3895: 383-402
<400> 3895
TGTCTCCGCA GTCGAAGAAG TGGAAGCATT AATGCGCAAT ATTGCCGATG 50
CCTGGAAAGA GTCGTTACTC TCCCCTTCTT TGATTCAGGA CCCAGTCTGA 100
<210> 3896
<211> 100
<212> DNA
<213> Escherichia coli K-12 MG1655
<220> flis b1926
<222> 286-385
<223> flis b1926 U00096 2003326..2003736
<313> 3896: 307-326
<400> 3896
TTGCTGCAAG CCAATTTACG CAACGATGTC TCCGCAGTCG AAGAAGTGGA 50
AGCATTAATG CGCAATATTG CCGATGCCTG GAAAGAGTCG TTACTCTCCC 100
<210> 3897
<211> 100
<212> DNA
<213> Escherichia coli K-12 MG1655
<220> flis b1926
<222> 166-265
<223> flis b1926 U00096 2003326..2003736
<313> 3897: 210-229
<400> 3897
GTCTCTTTGT CAAAAGCGAT CAACATCATT GAGAACGGAC TGCGGGTGAG 50
TCTTGATGAA GAGAGCAAAG ACGAACTAAC CCAAAACTTG ATTGCTCTTT 100
<210> 3898
<211> 100
<212> DNA
<213> Escherichia coli K-12 MG1655
<220> adia b4118
<222> 2151-2250
<223> adia b4118 U00096 complement(4335832..4338102)
<313> 3898: 2200-2219
<400> 3898
GCAAGTAAGT TATTTACGCT CGCTGCAATC CTGGGACCAC CATTTCCCTG 50
GATTTGAACA CGAAACTGAA GGGACTGAAA TTATTGACGG TATTTACCAC 100
<210> 3899
<211> 100

<212> DNA
<213> Escherichia coli K-12 MG1655
<220> adia b4118
<222> 2099-2198
<223> adia b4118 U00096 complement(4335832..4338102)
<313> 3899: 2164-2183
<400> 3899
CAGGAATCCC GATGCTGCTG TCTGGTGAAA ACTTCGGCGA TAAAAACAGT 50
CCGCAAGTAA GTTATTTACG CTCGCTGCAA TCCTGGGACC ACCATTTCCC 100
<210> 3900
<211> 100
<212> DNA
<213> Escherichia coli K-12 MG1655
<220> adia b4118
<222> 2091-2190
<223> adia b4118 U00096 complement(4335832..4338102)
<313> 3900: 2114-2133
<400> 3900
GTATCCGCCA GGAATCCCGA TGCTGCTGTC TGGTGAAAAC TTCGGCGATA 50
AAAACAGTCC GCAAGTAAGT TATTTACGCT CGCTGCAATC CTGGGACCAC 100
<210> 3901
<211> 100
<212> DNA
<213> Escherichia coli K-12 MG1655
<220> b1525 b1526
<222> 1314-1413
<223> b1525 b1526 U00096 complement(1611339..1612751)
<313> 3901: 1380-1399
<400> 3901
CTTTGGTGGC GTGAAAAAGA GTGGCTTTGG TCGTGAGCTT TCCCATTTCG 50
GCTTACACGA ATTCTGTAAT ATCCAGACGG TGTGGAAAGA CCGGATCTGA 100
<210> 3902
<211> 100
<212> DNA
<213> Escherichia coli K-12 MG1655
<220> b1525 b1526
<222> 1303-1402
<223> b1525 b1526 U00096 complement(1611339..1612751)
<313> 3902: 1324-1343
<400> 3902
GCGCGAGTGG CCTTTGGTGG CGTGAAAAAG AGTGGCTTTG GTCGTGAGCT 50
TTCCCATTTC GGCTTACACG AATTCTGTAA TATCCAGACG GTGTGGAAAG 100
<210> 3903
<211> 100
<212> DNA
<213> Escherichia coli K-12 MG1655
<220> b1525 b1526
<222> 1240-1339
<223> b1525 b1526 U00096 complement(1611339..1612751)
<313> 3903: 1261-1280
<400> 3903
AGACAGATGG CGGCACGTCT GGAATGCGGT GGGGTGTTTA TCAATGGTTA 50
TTGTGCCAGC GACGCGCGAG TGGCCTTTGG TGGCGTGAAA AAGAGTGGCT 100
<210> 3904
<211> 100
<212> DNA
<213> Escherichia coli K-12 MG1655
<220> b2254 b2254
<222> 870-969
<223> b2254 b2254 U00096 2365091..2366059
<313> 3904: 944-963
<400> 3904
ACTCGGTGAA TATATCGGCA GGATCTACAC CGATGTCCGC GCCCGCCCCC 50
GCTATTTTGT TCAGCAAGTT ATCCGTCCAT CCAGCAAGGA AAATGAATAA 100
<210> 3905
<211> 100
<212> DNA
<213> Escherichia coli K-12 MG1655
<220> b2254 b2254
<222> 840-939
<223> b2254 b2254 U00096 2365091..2366059

<313> 3905: 903-922
<400> 3905
TATTGGCGCT CAGTTTATCG GCATGGGATT ACTCGGTGAA TATATCGGCA 50
GGATCTACAC CGATGTCCGC GCCCGCCCCC GCTATTTTGT TCAGCAAGTT 100
<210> 3906
<211> 100
<212> DNA
<213> Escherichia coli K-12 MG1655
<220> b2254 b2254
<222> 822-921
<223> b2254 b2254 U00096 2365091..2366059
<313> 3906: 839-858
<400> 3906
TGCCGTGCTG TTTACTTTTA TTGGCGCTCA GTTTATCGGC ATGGGATTAC 50
TCGGTGAATA TATCGGCAGG ATCTACACCG ATGTCCGCGC CCGCCCCCGC 100
<210> 3907
<211> 100
<212> DNA
<213> Escherichia coli K-12 MG1655
<220> flit b1927
<222> 267-366
<223> flit b1927 U00096 2003736..2004101
<313> 3907: 346-365
<400> 3907
GGCGAAACTG GTCGGTCAGT CATCGGTGCA AAAATCGGTG TTAAGTGCCT 50
ATGGCGATCA GGGCGGCTTT GTGCTGGCTC CGCAGGATAA CCTCTTTTGA 100
<210> 3908
<211> 100
<212> DNA
<213> Escherichia coli K-12 MG1655
<220> flit b1927
<222> 173-272
<223> flit b1927 U00096 2003736..2004101
<313> 3908: 212-231
<400> 3908
CCACGATGCA GGAGCAGCTC CGCCCGATGC TGCGCCTGAT TCTCGACAAC 50
GAAAGCAAGG TAAAGCAGTT ATTACAGATT CGGATGGATG AACTGGCGAA 100
<210> 3909
<211> 100
<212> DNA
<213> Escherichia coli K-12 MG1655
<220> flit b1927
<222> 150-249
<223> flit b1927 U00096 2003736..2004101
<313> 3909: 167-186
<400> 3909
GACTGAAGAG GTTGACCCGT CCACCACGAT GCAGGAGCAG CTCCGCCCGA 50
TGCTGCGCCT GATTCTCGAC AACGAAAGCA AGGTAAAGCA GTTATTACAG 100
<210> 3910
<211> 100
<212> DNA
<213> Escherichia coli K-12 MG1655
<220> ybdq b0608
<222> 330-429
<223> ybdq b0608 U00096 complement(640662..641090)
<313> 3910: 347-366
<400> 3910
TATTGGTTCT CGCAACCCAT CGATTTCGAC CCATCTGTTA GGTTCTAACG 50
CCTCGAGCGT GATCCGCCAC GCCAATCTGC CGGTGCTGGT TGTGCGTTAA 100
<210> 3911
<211> 100
<212> DNA
<213> Escherichia coli K-12 MG1655
<220> ybdq b0608
<222> 284-383
<223> ybdq b0608 U00096 complement(640662..641090)
<313> 3911: 296-315
<400> 3911
ATGAAGTCAA TGAGTTGGCA GAAGAACTGG GGGCTGATGT TGTAGTTATT 50
GGTTCTCGCA ACCCATCGAT TTCGACCCAT CTGTTAGGTT CTAACGCCTC 100
<210> 3912

<211> 100
<212> DNA
<213> Escherichia coli K-12 MG1655
<220> ybdq b0608
<222> 230-329
<223> ybdq b0608 U00096 complement(640662..641090)
<313> 3912: 274-293
<400> 3912
TCACCATCGA TCCTTCCCGC ATTAAACAAC ATGTCCGTTT TGGTAGCGTG 50
CGGGATGAAG TCAATGAGTT GGCAGAAGAA CTGGGGGCTG ATGTTGTAGT 100
<210> 3913
<211> 100
<212> DNA
<213> Escherichia coli K-12 MG1655
<220> b2255 b2255
<222> 1874-1973
<223> b2255 b2255 U00096 2366059..2368041
<313> 3913: 1911-1930
<400> 3913
ATGCCCACCG CTGCCTGGAC TGGGAGCCGA AAATTGATAT GCAGGAAACC 50
ATCGACGAAA CGCTGGATTT CTTCCTGCGC ACCGTTGATC TTACGGATAA 100
<210> 3914
<211> 100
<212> DNA
<213> Escherichia coli K-12 MG1655
<220> b2255 b2255
<222> 1829-1928
<223> b2255 b2255 U00096 2366059..2368041
<313> 3914: 1885-1904
<400> 3914
GCAAAGGATA TCAGGACGTA GAGCATCGTA AACCGAGCAT CCGCAATGCC 50
CACCGCTGCC TGGACTGGGA GCCGAAAATT GATATGCAGG AAACCATCGA 100
<210> 3915
<211> 100
<212> DNA
<213> Escherichia coli K-12 MG1655
<220> b2255 b2255
<222> 1813-1912
<223> b2255 b2255 U00096 2366059..2368041
<313> 3915: 1830-1849
<400> 3915
AGTAGCAGCT ACTACGGCAA AGGATATCAG GACGTAGAGC ATCGTAAACC 50
GAGCATCCGC AATGCCCACC GCTGCCTGGA CTGGGAGCCG AAAATTGATA 100
<210> 3916
<211> 100
<212> DNA
<213> Escherichia coli K-12 MG1655
<220> ybdr ybdR
<222> 1139-1238
<223> ybdr ybdR U00096 641311..642549
<313> 3916: 1188-1207
<400> 3916
AGTGCCGTAA GGTGATTCTG GTACCCGGTG CACAAAGCGC AGAGGCGGCG 50
CAGAAGGCGG TTTCAGGTCT GGTGAATGCG ATGCCGGGGG GAACAATATG 100
<210> 3917
<211> 100
<212> DNA
<213> Escherichia coli K-12 MG1655
<220> ybdr ybdR
<222> 1084-1183
<223> ybdr ybdR U00096 641311..642549
<313> 3917: 1134-1153
<400> 3917
ATGCCGTTTG AAGAGGCCGC CCGGGGATAT GAGATTTTCG AAAAACGTGA 50
AGAGGAGTGC CGTAAGGTGA TTCTGGTACC CGGTGCACAA AGCGCAGAGG 100
<210> 3918
<211> 100
<212> DNA
<213> Escherichia coli K-12 MG1655
<220> ybdr ybdR
<222> 1029-1128

<223> ybdr ybdR U00096 641311..642549
<313> 3918: 1082-1101
<400> 3918
ATTACCGTTA ATTGAGAAAG GATTACTGAA ACCAGAAGAA ATTGTTACCC 50
ACTATATGCC GTTTGAAGAG GCCGCCCGGG GATATGAGAT TTTCGAAAAA 100
<210> 3919
<211> 100
<212> DNA
<213> Escherichia coli K-12 MG1655
<220> b1527 b1528
<222> 1017-1116
<223> b1527 b1528 U00096 1613787..1614902
<313> 3919: 1089-1108
<400> 3919
AGTTACTCAC GAAGACCTGG TGAAAAACAG GCCTCGCTCC CCTGTCAGAC 50
CACCTATACC CGCAACCGCC AAAACGCCAG ACCTCCCTGA ACGTCATTAA 100
<210> 3920
<211> 100
<212> DNA
<213> Escherichia coli K-12 MG1655
<220> b1527 b1528
<222> 975-1074
<223> b1527 b1528 U00096 1613787..1614902
<313> 3920: 1023-1042
<400> 3920
CAGGACCCTC AAACTGTTTG TAAAAAATGA AACACCGCCA AAAGTTACTC 50
ACGAAGACCT GGTGAAAAAC AGGCCTCGCT CCCCTGTCAG ACCACCTATA 100
<210> 3921
<211> 100
<212> DNA
<213> Escherichia coli K-12 MG1655
<220> b1527 b1528
<222> 648-747
<223> b1527 b1528 U00096 1613787..1614902
<313> 3921: 707-726
<400> 3921
CTCTGAGGTT CACAGATGGT TCGTCAATAA AGATCCTAAC TTGATCACCG 50
CCATTCTCGA CATAACGGAC AGCGAATTAA AAGAGGATTT GTTGCAAAGC 100
<210> 3922
<211> 100
<212> DNA
<213> Escherichia coli K-12 MG1655
<220> b2256 b2256
<222> 765-864
<223> b2256 b2256 U00096 2368038..2368928
<313> 3922: 805-824
<400> 3922
AGGCGTGACA TTTTGCCCTT TAAGCGAACT GTTATCAGAG ACGTTGCCGC 50
TCGGACAAGT TGTTCGCGGA AATATTGCCG GACGTGAAGG CTGGCTGGGT 100
<210> 3923
<211> 100
<212> DNA
<213> Escherichia coli K-12 MG1655
<220> b2256 b2256
<222> 717-816
<223> b2256 b2256 U00096 2368038..2368928
<313> 3923: 765-784
<400> 3923
CGCTTATCAG CATAATTTTG TGGATCTCCT CAAACGCGCA GCTCAGGAAG 50
GCGTGACATT TTGCCCTTTA AGCGAACTGT TATCAGAGAC GTTGCCGCTC 100
<210> 3924
<211> 100
<212> DNA
<213> Escherichia coli K-12 MG1655
<220> b2256 b2256
<222> 690-789
<223> b2256 b2256 U00096 2368038..2368928
<313> 3924: 727-746
<400> 3924
TACCATTCAT GCAGAAGTTG AAGGCTGCGC TTATCAGCAT AATTTTGTGG 50
ATCTCCTCAA ACGCGCAGCT CAGGAAGGCG TGACATTTTG CCCTTTAAGC 100

<210> 3925
<211> 100
<212> DNA
<213> Escherichia coli K-12 MG1655
<220> yqfb b2901
<222> 176-275
<223> yqfb b2901 U00096 complement(3041332..3041643)
<313> 3925: 208-227
<400> 3925
CAACCGTAAC GCTGGATACG CTGACTGAAA AACATGCAGA GCAGGAAAAT 50
ATGACCCTGA CTGAACTGAA AAAAGTCATT GCTGACATCT ATCCCGGTCA 100
<210> 3926
<211> 100
<212> DNA
<213> Escherichia coli K-12 MG1655
<220> yqfb b2901
<222> 133-232
<223> yqfb b2901 U00096 complement(3041332..3041643)
<313> 3926: 171-190
<400> 3926
GAAGATGACG GTTATTTTTG CACGATTGAA GTCACCGCAA CCTCAACCGT 50
AACGCTGGAT ACGCTGACTG AAAAACATGC AGAGCAGGAA AATATGACCC 100
<210> 3927
<211> 100
<212> DNA
<213> Escherichia coli K-12 MG1655
<220> yqfb b2901
<222> 96-195
<223> yqfb b2901 U00096 complement(3041332..3041643)
<313> 3927: 122-141
<400> 3927
CTTCAAAACG GGTGATGTGC TTCGTGTCGG ACGTTTTGAA GATGACGGTT 50
ATTTTTGCAC GATTGAAGTC ACCGCAACCT CAACCGTAAC GCTGGATACG 100
<210> 3928
<211> 100
<212> DNA
<213> Escherichia coli K-12 MG1655
<220> ybds b0613
<222> 1323-1422
<223> ybds b0613 U00096 complement(644340..645803)
<313> 3928: 1374-1393
<400> 3928
TGCAACCGGT CCTGGGGTGA TTATTTACGG CTGTGGCTAT GTGAAATCAA 50
AAGATTACTG GCGTCTTGGC GCAATCTTCG GGGTGATTTA CATCTCTATG 100
<210> 3929
<211> 100
<212> DNA
<213> Escherichia coli K-12 MG1655
<220> ybds b0613
<222> 1239-1338
<223> ybds b0613 U00096 complement(644340..645803)
<313> 3929: 1273-1292
<400> 3929
TAAAGGTATT CCGGGCGTAC CAATGGAACA ACTGTGTATC CTGCTGGTGC 50
TGTCTATCGG TATCATGGGC TGTCTGACGC CGTATGCAAC CGGTCCTGGG 100
<210> 3930
<211> 100
<212> DNA
<213> Escherichia coli K-12 MG1655
<220> ybds b0613
<222> 1204-1303
<223> ybds b0613 U00096 complement(644340..645803)
<313> 3930: 1227-1246
<400> 3930
ACCGCAACCA TGCTGCCGGT TATTCTGGCC GTCGGTAAAG GTATTCCGGG 50
CGTACCAATG GAACAACTGT GTATCCTGCT GGTGCTGTCT ATCGGTATCA 100
<210> 3931
<211> 100
<212> DNA
<213> Escherichia coli K-12 MG1655
<220> b2257 b2257

<222> 1552-1651
<223> b2257 b2257 U00096 2368928..2370580
<313> 3931: 1583-1602
<400> 3931
TCGGTTGACC GCGATGAAGA TATCAACAGT CTCGCCATTC CGCCCGCAGA 50
TGCCATCGAT CGTCAGGAGC GTCTGGTGCT GATTCAGTAT CGTCCCAAAT 100
<210> 3932
<211> 100
<212> DNA
<213> Escherichia coli K-12 MG1655
<220> b2257 b2257
<222> 1483-1582
<223> b2257 b2257 U00096 2368928..2370580
<313> 3932: 1530-1549
<400> 3932
GTCAGCGGTG ATGAGTTCGC AAACTGGCTT AATCAACATC GTCAGGAGGG 50
GATTATTACT CTCGTGCTTT CGGTTGACCG CGATGAAGAT ATCAACAGTC 100
<210> 3933
<211> 100
<212> DNA
<213> Escherichia coli K-12 MG1655
<220> b2257 b2257
<222> 1398-1497
<223> b2257 b2257 U00096 2368928..2370580
<313> 3933: 1437-1456
<400> 3933
CCTGCAACGC GATGACATCA TCATGTATCG CCAGACAGGT GAGTTGAAAT 50
ACGGCCTTAA TTATCCGGAT GCGAAAGGGA GATTTGTCAG CGGTGATGAG 100
<210> 3934
<211> 100
<212> DNA
<213> Escherichia coli K-12 MG1655
<220> b2258 b2259
<222> 570-669
<223> b2258 b2259 U00096 2370630..2371298
<313> 3934: 634-653
<400> 3934
CTGGGAAGGG ACTTTTTCGC TGAAAGCACT ACTGGGAGTA GCCTGTATTA 50
TGAGCGGGTT GATGCTGATT TTCCTGCCCA CGACAAAACA ACGTTACTGA 100
<210> 3935
<211> 100
<212> DNA
<213> Escherichia coli K-12 MG1655
<220> b2258 b2259
<222> 559-658
<223> b2258 b2259 U00096 2370630..2371298
<313> 3935: 593-612
<400> 3935
GTACTTCCCG GCTGGGAAGG GACTTTTTCG CTGAAAGCAC TACTGGGAGT 50
AGCCTGTATT ATGAGCGGGT TGATGCTGAT TTTCCTGCCC ACGACAAAAC 100
<210> 3936
<211> 100
<212> DNA
<213> Escherichia coli K-12 MG1655
<220> b2258 b2259
<222> 480-579
<223> b2258 b2259 U00096 2370630..2371298
<313> 3936: 501-520
<400> 3936
AACGCTTCAT AAACTTGCGC TAAGCAAAGC CTATGCCTTG CTCAGTATGA 50
GTTATGTGCT GGTATGGATT GCTTCAATGG TACTTCCCGG CTGGGAAGGG 100
<210> 3937
<211> 100
<212> DNA
<213> Escherichia coli K-12 MG1655
<220> ybdu b0615
<222> 443-542
<223> ybdu b0615 U00096 complement(646707..647258)
<313> 3937: 505-524
<400> 3937
GCGAACAAAG CGCAGCCGTC TGCGCGCGTG GAAAAACCCA TCAACTGACC 50

GATTTACTCA ACCGCATGGA GGCACTGCTG AACGATGTCG ATGCCTGCAA 100
<210> 3938
<211> 100
<212> DNA
<213> Escherichia coli K-12 MG1655
<220> ybdu b0615
<222> 367-466
<223> ybdu b0615 U00096 complement(646707..647258)
<313> 3938: 415-434
<400> 3938
ATCGATGTCC TGACGCCCGA AGGCGAAATT CTCTCCCGCC GCGACTATTC 50
ACTGCCGCCT CGCCGCTGCC TGTTGTGCGA ACAAAGCGCA GCCGTCTGCG 100
<210> 3939
<211> 100
<212> DNA
<213> Escherichia coli K-12 MG1655
<220> ybdu b0615
<222> 363-462
<223> ybdu b0615 U00096 complement(646707..647258)
<313> 3939: 386-405
<400> 3939
GGATATCGAT GTCCTGACGC CCGAAGGCGA AATTCTCTCC CGCCGCGACT 50
ATTCACTGCC GCCTCGCCGC TGCCTGTTGT GCGAACAAAG CGCAGCCGTC 100
<210> 3940
<211> 100
<212> DNA
<213> Escherichia coli K-12 MG1655
<220> fliy b1921
<222> 702-801
<223> fliy b1921 U00096 complement(1997608..1998408)
<313> 3940: 749-768
<400> 3940
CCTGCTGAAA GCAGTGAATG ATGCAATTGC GGAAATGCAA AAAGATGGCA 50
CTCTGCAAGC CCTTTCCGAA AAATGGTTTG GTGCTGATGT GACCAAATAA 100
<210> 3941
<211> 100
<212> DNA
<213> Escherichia coli K-12 MG1655
<220> fliy b1921
<222> 662-761
<223> fliy b1921 U00096 complement(1997608..1998408)
<313> 3941: 701-720
<400> 3941
GTCAGGAGTC TGGCGTGGCG CTGCGTAAAG GAAATGAGGA CCTGCTGAAA 50
GCAGTGAATG ATGCAATTGC GGAAATGCAA AAAGATGGCA CTCTGCAAGC 100
<210> 3942
<211> 100
<212> DNA
<213> Escherichia coli K-12 MG1655
<220> fliy b1921
<222> 565-664
<223> fliy b1921 U00096 complement(1997608..1998408)
<313> 3942: 611-630
<400> 3942
GGGCGTATCG ATGCGATCCT CGTTGATCGT CTGGCGGCGC TGGATCTGGT 50
GAAGAAAACC AACGATACGC TGGCAGTAAC CGGTGAAGCA TTCTCCCGTC 100
<210> 3943
<211> 100
<212> DNA
<213> Escherichia coli K-12 MG1655
<220> yqfe b2916
<222> 132-231
<223> yqfe b2916 U00096 complement(3057401..3057631)
<313> 3943: 209-228
<400> 3943
CAGACCGTTG TATGCTTTAT TTGCGGGGCG AAAGGGGATG CCTGCCATTG 50
CGCGATATTT TATGGATGAG TTAACCACGC GGCTTGCCAA CGGGGTCTGA 100
<210> 3944
<211> 100
<212> DNA
<213> Escherichia coli K-12 MG1655

<220> yqfe b2916
<222> 97-196
<223> yqfe b2916 U00096 complement(3057401..3057631)
<313> 3944: 151-170
<400> 3944
CTGGTGCGCC TGTTTGCACC GTGGAGCGGC ATACCCAGAC CGTTGTATGC 50
TTTATTTGCG GGGCGAAAGG GGATGCCTGC CATTGCGCGA TATTTTATGG 100
<210> 3945
<211> 100
<212> DNA
<213> Escherichia coli K-12 MG1655
<220> yqfe b2916
<222> 80-179
<223> yqfe b2916 U00096 complement(3057401..3057631)
<313> 3945: 104-123
<400> 3945
GGCTTGCGAA CGGTGAACTG GTGCGCCTGT TTGCACCGTG GAGCGGCATA 50
CCCAGACCGT TGTATGCTTT ATTTGCGGGG CGAAAGGGGA TGCCTGCCAT 100
<210> 3946
<211> 100
<212> DNA
<213> Escherichia coli K-12 MG1655
<220> fliz b1922
<222> 489-588
<223> fliz b1922 U00096 complement(1998496..1999083)
<313> 3946: 562-581
<400> 3946
TTACCGCATC GCGTTACGGA AGTATCAACA CTATCAGCGC CAAACCTGTA 50
CCAGACTTGT GCAGAAATCC TCCAGCCTGC CTTCTTCTGA TATATATTAA 100
<210> 3947
<211> 100
<212> DNA
<213> Escherichia coli K-12 MG1655
<220> fliz b1922
<222> 478-577
<223> fliz b1922 U00096 complement(1998496..1999083)
<313> 3947: 515-534
<400> 3947
AGCACCAACA ATTACCGCAT CGCGTTACGG AAGTATCAAC ACTATCAGCG 50
CCAAACCTGT ACCAGACTTG TGCAGAAATC CTCCAGCCTG CCTTCTTCTG 100
<210> 3948
<211> 100
<212> DNA
<213> Escherichia coli K-12 MG1655
<220> fliz b1922
<222> 437-536
<223> fliz b1922 U00096 complement(1998496..1999083)
<313> 3948: 465-484
<400> 3948
GTTTCCTTGA TGAAATCCTC GCCCCGTGGC TGCCCACCAC CAGCACCAAC 50
AATTACCGCA TCGCGTTACG GAAGTATCAA CACTATCAGC GCCAAACCTG 100
<210> 3949
<211> 100
<212> DNA
<213> Escherichia coli K-12 MG1655
<220> ybea b0637
<222> 369-468
<223> ybea b0637 U00096 complement(667471..667938)
<313> 3949: 429-448
<400> 3949
GGCGCTTACC CTCCCCCATC CGCTGGTTCG CGTGCTGGTC GCAGAGAGTC 50
TGTACCGGGC GTGGAGCATC ACCACCAACC ATCCTTATCA CCGTGAGTGA 100
<210> 3950
<211> 100
<212> DNA
<213> Escherichia coli K-12 MG1655
<220> ybea b0637
<222> 355-454
<223> ybea b0637 U00096 complement(667471..667938)
<313> 3950: 390-409
<400> 3950

AGCTGGTCGC TGTCGGCGCT TACCCTCCCC CATCCGCTGG TTCGCGTGCT 50
GGTCGCAGAG AGTCTGTACC GGGCGTGGAG CATCACCACC AACCATCCTT 100
<210> 3951
<211> 100
<212> DNA
<213> Escherichia coli K-12 MG1655
<220> ybea b0637
<222> 227-326
<223> ybea b0637 U00096 complement(667471..667938)
<313> 3951: 254-273
<400> 3951
GCAAGCCCTG GGATACGCCG CAGTTAGCCG CTGAGCTGGA ACGCTGGAAG 50
CTGGATGGTC GCGACGTCAG TCTACTGATT GGCGGGCCTG AAGGGTTGTC 100
<210> 3952
<211> 100
<212> DNA
<213> Escherichia coli K-12 MG1655
<220> ybeb b0638
<222> 111-210
<223> ybeb b0638 U00096 complement(667942..668151)
<313> 3952: 170-189
<400> 3952
CAGCGCCGAC TGGATTGTCG TGGATTTGGG CGATGTGATT GTCCATGTCA 50
TGCAGGAAGA GAGCCGTCGC CTGTATGAAC TGGAAAAACT CTGGAGTTAA 100
<210> 3953
<211> 100
<212> DNA
<213> Escherichia coli K-12 MG1655
<220> ybeb b0638
<222> 84-183
<223> ybeb b0638 U00096 complement(667942..668151)
<313> 3953: 120-139
<400> 3953
GTTACCGCTC GGCGTAGAAG GTGAAAACAG CGCCGACTGG ATTGTCGTGG 50
ATTTGGGCGA TGTGATTGTC CATGTCATGC AGGAAGAGAG CCGTCGCCTG 100
<210> 3954
<211> 100
<212> DNA
<213> Escherichia coli K-12 MG1655
<220> ybeb b0638
<222> 1-100
<223> ybeb b0638 U00096 complement(667942..668151)
<313> 3954: 43-62
<400> 3954
ATGATCATCT GTACGGGTAC GTCCAGCCGT CATGTTATGT CCATTGCTGA 50
CCACGTTGTG CAGGAGTCTC GCGCAGCGGG CCTGTTACCG CTCGGCGTAG 100
<210> 3955
<211> 100
<212> DNA
<213> Escherichia coli K-12 MG1655
<220> ybec b0628
<222> 105-204
<223> ybec b0628 U00096 658170..658373
<313> 3955: 168-187
<400> 3955
CATTAAAGGG TTCAAGAAGG CGATGAATGA TGACGATGCT GCGGCGAAAA 50
AAGGCGCAGA CGTTGATCTT CAGGCTGAAA AGCTCTCTCA TAAAGAGTGA 100
<210> 3956
<211> 100
<212> DNA
<213> Escherichia coli K-12 MG1655
<220> ybec b0628
<222> 105-204
<223> ybec b0628 U00096 658170..658373
<313> 3956: 156-175
<400> 3956
CATTAAAGGG TTCAAGAAGG CGATGAATGA TGACGATGCT GCGGCGAAAA 50
AAGGCGCAGA CGTTGATCTT CAGGCTGAAA AGCTCTCTCA TAAAGAGTGA 100
<210> 3957
<211> 100
<212> DNA

<213> Escherichia coli K-12 MG1655
<220> ybec b0628
<222> 103-202
<223> ybec b0628 U00096 658170..658373
<313> 3957: 147-166
<400> 3957
GCCATTAAAG GGTTCAAGAA GGCGATGAAT GATGACGATG CTGCGGCGAA 50
AAAAGGCGCA GACGTTGATC TTCAGGCTGA AAAGCTCTCT CATAAAGAGT 100
<210> 3958
<211> 100
<212> DNA
<213> Escherichia coli K-12 MG1655
<220> ybed b0632
<222> 165-264
<223> ybed b0632 U00096 complement(661602..661865)
<313> 3958: 210-229
<400> 3958
CCACTCGGTA TCTATCACTA TCAACGCCAC TCATATCGAG CAGGTTGAAA 50
CACTGTATGA AGAACTGGGC AAAATCGATA TTGTCCGCAT GGTTCTGTAA 100
<210> 3959
<211> 100
<212> DNA
<213> Escherichia coli K-12 MG1655
<220> ybed b0632
<222> 139-238
<223> ybed b0632 U00096 complement(661602..661865)
<313> 3959: 171-190
<400> 3959
GTAAAACCAA GCAGCAAAGG CAACTACCAC TCGGTATCTA TCACTATCAA 50
CGCCACTCAT ATCGAGCAGG TTGAAACACT GTATGAAGAA CTGGGCAAAA 100
<210> 3960
<211> 100
<212> DNA
<213> Escherichia coli K-12 MG1655
<220> ybed b0632
<222> 101-200
<223> ybed b0632 U00096 complement(661602..661865)
<313> 3960: 122-141
<400> 3960
TGGTACAGCG CCATGCGCCA GGTGACTACA CCCCAACGGT AAAACCAAGC 50
AGCAAAGGCA ACTACCACTC GGTATCTATC ACTATCAACG CCACTCATAT 100
<210> 3961
<211> 100
<212> DNA
<213> Escherichia coli K-12 MG1655
<220> ybef b0630
<222> 702-801
<223> ybef b0630 U00096 complement(659648..660448)
<313> 3961: 771-790
<400> 3961
AAATGAGGAG CAAATAGATT TCTCTATCCA CTACAACAAA TTCAGCCTGC 50
GTGATCCCAT CCTGCACGGA GTAATAGATG TTATCCGTAA TGCATTTTGA 100
<210> 3962
<211> 100
<212> DNA
<213> Escherichia coli K-12 MG1655
<220> ybef b0630
<222> 639-738
<223> ybef b0630 U00096 complement(659648..660448)
<313> 3962: 664-683
<400> 3962
CAGCCGTTTT TATAACCTGT TTAGCCGCTG CTGGCCGCTG GAAAAACTGC 50
CTTTTCCGTC CTTAAATGAG GAGCAAATAG ATTTCTCTAT CCACTACAAC 100
<210> 3963
<211> 100
<212> DNA
<213> Escherichia coli K-12 MG1655
<220> ybef b0630
<222> 548-647
<223> ybef b0630 U00096 complement(659648..660448)
<313> 3963: 604-623

<400> 3963
TGTTTCCGGA CCGGCAAATC AATTTCACCA GCTACAACAT TTTGACAATC 50
GCTGCACTGG TTGCCAACAG TGACATGTTA GCGATTATTC CCAGCCGTTT 100
<210> 3964
<211> 100
<212> DNA
<213> Escherichia coli K-12 MG1655
<220> adiy b4117
<222> 663-762
<223> adiy b4117 U00096 complement(4334746..4335507)
<313> 3964: 730-749
<400> 3964
CTACAACAGT ACGTCGTACT TTATTTCTGT CTTTAAAGAC TTCTACGGTA 50
TGACGCCGCT GCATTATGTT AGTCAGCACA GAGAACGCAC TGTCGCCTGA 100
<210> 3965
<211> 100
<212> DNA
<213> Escherichia coli K-12 MG1655
<220> adiy b4117
<222> 617-716
<223> adiy b4117 U00096 complement(4334746..4335507)
<313> 3965: 642-661
<400> 3965
TAATGATGGA CGGTAAAAAT ATCTCTCAGG TATCACAGTC CTGCGGCTAC 50
AACAGTACGT CGTACTTTAT TTCTGTCTTT AAAGACTTCT ACGGTATGAC 100
<210> 3966
<211> 100
<212> DNA
<213> Escherichia coli K-12 MG1655
<220> adiy b4117
<222> 339-438
<223> adiy b4117 U00096 complement(4334746..4335507)
<313> 3966: 361-380
<400> 3966
AGAAAAGGAA CGAACACGCG CATTATTATT CACGGTGCTA TCCCGTTTTC 50
TCGACAGTAA AAAATTCGTT TCATTAATGA TGTATATGTT ACGTAATTGT 100
<210> 3967
<211> 100
<212> DNA
<213> Escherichia coli K-12 MG1655
<220> ybeh b0626
<222> 129-228
<223> ybeh b0626 U00096 657254..657481
<313> 3967: 191-210
<400> 3967
GGCGCGCGAT GATCATGATG CAGATCTATC GGTTAAATCA GCACAGCTGC 50
TGGAAGGCGA ATTCCTCGGA CTTTACGGCG AGAAAGTAAA CGTAACATGA 100
<210> 3968
<211> 100
<212> DNA
<213> Escherichia coli K-12 MG1655
<220> ybeh b0626
<222> 68-167
<223> ybeh b0626 U00096 657254..657481
<313> 3968: 108-127
<400> 3968
CGTTGATGGC GCAGGCGGCG GAAAACGACG CATCGCTGTT TGCCCTGCCG 50
GAAGCATTGC TGGCGCGCGA TGATCATGAT GCAGATCTAT CGGTTAAATC 100
<210> 3969
<211> 100
<212> DNA
<213> Escherichia coli K-12 MG1655
<220> ybeh b0626
<222> 1-100
<223> ybeh b0626 U00096 657254..657481
<313> 3969: 53-72
<400> 3969
ATGCTGGTTG CCGCAGGACA GTTTGCTGTT ACATCTGTGT GGGAAAAGAA 50
CGCTGAGATT TGTGCCTCGT TGATGGCGCA GGCGGCGGAA AACGACGCAT 100
<210> 3970
<211> 100

<212> DNA
<213> Escherichia coli K-12 MG1655
<220> ybej b0656
<222> 810-909
<223> ybej b0656 U00096 complement(686062..686970)
<313> 3970: 880-899
<400> 3970
CAAAAATCCA ATTCCGCCGA AAAACCTGAA CATGAATTTC GAACTGTCAG 50
ACGAAATGAA AGCACTGTTC AAAGAACCGA ATGACAAGGC ACTGAACTAA 100
<210> 3971
<211> 100
<212> DNA
<213> Escherichia coli K-12 MG1655
<220> ybej b0656
<222> 776-875
<223> ybej b0656 U00096 complement(686062..686970)
<313> 3971: 836-855
<400> 3971
CCGGTGAAGC GGAAAAATGG TTTGATAAGT GGTTCAAAAA TCCAATTCCG 50
CCGAAAAACC TGAACATGAA TTTCGAACTG TCAGACGAAA TGAAAGCACT 100
<210> 3972
<211> 100
<212> DNA
<213> Escherichia coli K-12 MG1655
<220> ybej b0656
<222> 749-848
<223> ybej b0656 U00096 complement(686062..686970)
<313> 3972: 774-793
<400> 3972
ATGACACCAT CGCTCAGGTG CAGACCTCCG GTGAAGCGGA AAAATGGTTT 50
GATAAGTGGT TCAAAAATCC AATTCCGCCG AAAAACCTGA ACATGAATTT 100
<210> 3973
<211> 100
<212> DNA
<213> Escherichia coli K-12 MG1655
<220> ybek b0652
<222> 810-909
<223> ybek b0652 U00096 complement(682700..683635)
<313> 3973: 846-865
<400> 3973
CCAGGGTATG ACGGTTGTTG ATTATTATTA TCTGACAGGC AATAAACCGA 50
ATGCCACCGT AATGGTCGAT GTTGATCGTC AGGGCTTTGT TGATTTACTG 100
<210> 3974
<211> 100
<212> DNA
<213> Escherichia coli K-12 MG1655
<220> ybek b0652
<222> 698-797
<223> ybek b0652 U00096 complement(682700..683635)
<313> 3974: 752-771
<400> 3974
GCTTTGTCGG CGCACCACTG CATGACCCAT GCACCATCGC CTGGCTGTTG 50
AAACCGGAGT TATTTACCTC TGTTGAGCGC TGGGTTGGCG TGGAAACACA 100
<210> 3975
<211> 100
<212> DNA
<213> Escherichia coli K-12 MG1655
<220> ybek b0652
<222> 627-726
<223> ybek b0652 U00096 complement(682700..683635)
<313> 3975: 649-668
<400> 3975
TAACCCTGTT TCAACCATTG TTGCCGAACT GCTGGATTTC TTCCTCGAAT 50
ATCATAAAGA CGAAAAATGG GGCTTTGTCG GCGCACCACT GCATGACCCA 100
<210> 3976
<211> 100
<212> DNA
<213> Escherichia coli K-12 MG1655
<220> ybel b0644
<222> 384-483
<223> ybel b0644 U00096 674241..674723

<313> 3976: 412-431
<400> 3976
GAAATGTCAC TTCCATCTCC CGATCTACAC ACCGGAAGTG CTGACGCTAT 50
GCCCGAAATG TGGTCATGAC CAGTTCCAGA GACGCCCGTT TGAGCCGTAA 100
<210> 3977
<211> 100
<212> DNA
<213> Escherichia coli K-12 MG1655
<220> ybel b0644
<222> 321-420
<223> ybel b0644 U00096 674241..674723
<313> 3977: 385-404
<400> 3977
CCTCAATCAT CATGGGGTTT ATCACAGCGG AGAAGTGGTC GGGCTGGGAA 50
ATCTGGTCTG CGAGAAATGT CACTTCCATC TCCCGATCTA CACACCGGAA 100
<210> 3978
<211> 100
<212> DNA
<213> Escherichia coli K-12 MG1655
<220> ybel b0644
<222> 314-413
<223> ybel b0644 U00096 674241..674723
<313> 3978: 336-355
<400> 3978
TCCAGGACCT CAATCATCAT GGGGTTTATC ACAGCGGAGA AGTGGTCGGG 50
CTGGGAAATC TGGTCTGCGA GAAATGTCAC TTCCATCTCC CGATCTACAC 100
<210> 3979
<211> 100
<212> DNA
<213> Escherichia coli K-12 MG1655
<220> ybem b0627
<222> 465-564
<223> ybem b0627 U00096 657478..658041
<313> 3979: 527-546
<400> 3979
ACTCATTATG GCGGAAGTGA CGCCCGAACG TGTGCGTCAG GTGCGCGCGC 50
AACTGCCCGT CTTAAACAAC CGTCGCTTTG CGCCGCCGCA ATTATTATGA 100
<210> 3980
<211> 100
<212> DNA
<213> Escherichia coli K-12 MG1655
<220> ybem b0627
<222> 429-528
<223> ybem b0627 U00096 657478..658041
<313> 3980: 461-480
<400> 3980
TGGCGTCACC ATTGCGGCAG CGTCAGAAAT GCCTGCACTC ATTATGGCGG 50
AAGTGACGCC CGAACGTGTG CGTCAGGTGC GCGCGCAACT GCCCGTCTTA 100
<210> 3981
<211> 100
<212> DNA
<213> Escherichia coli K-12 MG1655
<220> ybem b0627
<222> 362-461
<223> ybem b0627 U00096 657478..658041
<313> 3981: 383-402
<400> 3981
GTTATATGGT GGCGGCGGGG GAGTGCGGGA ACAAAAATAT CGGTCAAAGC 50
CGGATTATAG ATCCCTTTGG CGTCACCATT GCGGCAGCGT CAGAAATGCC 100
<210> 3982
<211> 100
<212> DNA
<213> Escherichia coli K-12 MG1655
<220> yben b0640
<222> 402-501
<223> yben b0640 U00096 complement(669154..669795)
<313> 3982: 453-472
<400> 3982
TCCAGGTTAC CCACTTGAAA TGGCGCAACC GCAATACCAG CAATGGCTGG 50
AAGATCATTT GACACATAAC CCGGAAGATC TTCACCTTCA GCCTGCCGGT 100
<210> 3983

<211> 100
<212> DNA
<213> Escherichia coli K-12 MG1655
<220> yben b0640
<222> 356-455
<223> yben b0640 U00096 complement(669154..669795)
<313> 3983: 404-423
<400> 3983
AATACGAAAC GATACTCGAC AATGCACATT TGATCGTCTG TCGGCGTCCA 50
GGTTACCCAC TTGAAATGGC GCAACCGCAA TACCAGCAAT GGCTGGAAGA 100
<210> 3984
<211> 100
<212> DNA
<213> Escherichia coli K-12 MG1655
<220> yben b0640
<222> 297-396
<223> yben b0640 U00096 complement(669154..669795)
<313> 3984: 361-380
<400> 3984
CGTGCCGCTG GCGTTTATTA TTGGTCAGGA TTCACTGCTG ACCTTTCCGA 50
CCTGGTACGA ATACGAAACG ATACTCGACA ATGCACATTT GATCGTCTGT 100
<210> 3985
<211> 100
<212> DNA
<213> Escherichia coli K-12 MG1655
<220> b0805 b0806
<222> 2184-2283
<223> b0805 b0806 U00096 complement(838472..840754)
<313> 3985: 2262-2281
<400> 3985
GTTTGATACC GATTACGTCG CCTCAATCAA CAAGAGCGGC TACCGTTATC 50
ACCCGGGCGA GCCAAGAACC TTCTTGCTCA CAGCCAATAT GCATTTCTGA 100
<210> 3986
<211> 100
<212> DNA
<213> Escherichia coli K-12 MG1655
<220> b0805 b0806
<222> 2180-2279
<223> b0805 b0806 U00096 complement(838472..840754)
<313> 3986: 2210-2229
<400> 3986
ACCTGTTTGA TACCGATTAC GTCGCCTCAA TCAACAAGAG CGGCTACCGT 50
TATCACCCGG GCGAGCCAAG AACCTTCTTG CTCACAGCCA ATATGCATTT 100
<210> 3987
<211> 100
<212> DNA
<213> Escherichia coli K-12 MG1655
<220> b0805 b0806
<222> 2087-2186
<223> b0805 b0806 U00096 complement(838472..840754)
<313> 3987: 2140-2159
<400> 3987
GAACGCCAGC GTTTACCGAA GGTTACTGGG TCGCCGATGC CAAACTGGGG 50
TATCGAGTTA ATCGCAATCT CGACTTCCAG CTAAACGTTT ACAACCTGTT 100
<210> 3988
<211> 100
<212> DNA
<213> Escherichia coli K-12 MG1655
<220> ybeq b0645
<222> 883-982
<223> ybeq b0645 U00096 complement(674793..675776)
<313> 3988: 896-915
<400> 3988
CCTGTCGATC TCCGACAGGC TCTCGACCTG TATCGTAAAG TTCAGTCATC 50
CGGAACCAGA GATGTCAGTC AGGAAATTCG TGAGACTGAA GATTTACTGT 100
<210> 3989
<211> 100
<212> DNA
<213> Escherichia coli K-12 MG1655
<220> ybeq b0645
<222> 791-890

<223> ybeq b0645 U00096 complement(674793..675776)
<313> 3989: 819-838
<400> 3989
CTGCCTTTGA ATGGTTTACG AAAGCCGCTG AATGCAATGA CGCCACTGCC 50
TGGTATAACC TGGCCATTAT GTATCACTAC GGAGAAGGAA GACCTGTCGA 100
<210> 3990
<211> 100
<212> DNA
<213> Escherichia coli K-12 MG1655
<220> ybeq b0645
<222> 754-853
<223> ybeq b0645 U00096 complement(674793..675776)
<313> 3990: 769-788
<400> 3990
TATAAACACG GACAAGGCGT TGAGAAAGAT TATCAGGCTG CCTTTGAATG 50
GTTTACGAAA GCCGCTGAAT GCAATGACGC CACTGCCTGG TATAACCTGG 100
<210> 3991
<211> 100
<212> DNA
<213> Escherichia coli K-12 MG1655
<220> hha b0461
<222> 120-219
<223> hha b0461 U00096 complement(479314..479532)
<313> 3991: 163-182
<400> 3991
GGCGGTATTT TACTCAGCCG CAGATCACCG CCTCGCCGAA TTGACCATGA 50
ATAAACTGTA CGACAAGATC CCTTCCTCAG TATGGAAATT TATTCGCTAA 100
<210> 3992
<211> 100
<212> DNA
<213> Escherichia coli K-12 MG1655
<220> hha b0461
<222> 78-177
<223> hha b0461 U00096 complement(479314..479532)
<313> 3992: 118-137
<400> 3992
TGTTATCGAG AAAAATAAAT ACGAATTATC AGATAATGAA CTGGCGGTAT 50
TTTACTCAGC CGCAGATCAC CGCCTCGCCG AATTGACCAT GAATAAACTG 100
<210> 3993
<211> 100
<212> DNA
<213> Escherichia coli K-12 MG1655
<220> hha b0461
<222> 18-117
<223> hha b0461 U00096 complement(479314..479532)
<313> 3993: 50-69
<400> 3993
AACGAAAACC GATTATTTAA TGCGTTTACG TCGTTGCCAG ACAATTGACA 50
CGCTGGAGCG TGTTATCGAG AAAAATAAAT ACGAATTATC AGATAATGAA 100
<210> 3994
<211> 100
<212> DNA
<213> Escherichia coli K-12 MG1655
<220> yqga b2967
<222> 609-708
<223> yqga b2967 U00096 3107570..3108277
<313> 3994: 679-698
<400> 3994
ACGCATCTGT GGCATTAAAA TGTTCCCGGT GGTCAACATG CTTCCAGCAC 50
TCCTTCTGGC AATGCCGCTT TCCGCCGCCT GGACCGCCTG GTTTGCCTGA 100
<210> 3995
<211> 100
<212> DNA
<213> Escherichia coli K-12 MG1655
<220> yqga b2967
<222> 600-699
<223> yqga b2967 U00096 3107570..3108277
<313> 3995: 633-652
<400> 3995
AACCGGATTA CGCATCTGTG GCATTAAAAT GTTCCCGGTG GTCAACATGC 50
TTCCAGCACT CCTTCTGGCA ATGCCGCTTT CCGCCGCCTG GACCGCCTGG 100

<210> 3996
<211> 100
<212> DNA
<213> Escherichia coli K-12 MG1655
<220> yqga b2967
<222> 483-582
<223> yqga b2967 U00096 3107570..3108277
<313> 3996: 534-553
<400> 3996
TATCCCATTA CTGATCATCC AGTTAACGCT GGCGTGGGCT GCCGCGCTGA 50
TTTTACCGCT GACCACACCG TCGATGATGG CAGACTTCAG CGCCGTAGGC 100
<210> 3997
<211> 100
<212> DNA
<213> Escherichia coli K-12 MG1655
<220> yber b0646
<222> 597-696
<223> yber b0646 U00096 675934..676641
<313> 3997: 654-673
<400> 3997
GTTTTATCAA TTATTTGCCA GTGATGATGC GTATTACCCT ACCTTGTCCT 50
GGCAACCATT GGCTTACTAT TCTGCATGCC CGGAAACGCT TAAGGATATG 100
<210> 3998
<211> 100
<212> DNA
<213> Escherichia coli K-12 MG1655
<220> yber b0646
<222> 527-626
<223> yber b0646 U00096 675934..676641
<313> 3998: 577-596
<400> 3998
AAGATGATCA TCTGCCTTTA TTACGCGAAT TCTTAGCGCG TAAAGAATAC 50
GACGATTCCG GCAATGATAT GTTTTATCAA TTATTTGCCA GTGATGATGC 100
<210> 3999
<211> 100
<212> DNA
<213> Escherichia coli K-12 MG1655
<220> yber b0646
<222> 393-492
<223> yber b0646 U00096 675934..676641
<313> 3999: 444-463
<400> 3999
TAATAACTGG TTGAGCGAAG AAGAAAGTCT ATGGATCCAG TCGCGCATTC 50
ATCTACGGGC TCTACGTTAT TATAGTAACT GGCGGCAATA CTTTGCCGGT 100
<210> 4000
<211> 100
<212> DNA
<213> Escherichia coli K-12 MG1655
<220> yqgb b2940
<222> 48-147
<223> yqgb b2940 U00096 complement(3083939..3084085)
<313> 4000: 111-130
<400> 4000
TCAGCATTCA CTGCTGGAAA ATCCATGTGC TTATGGGTTG TTATCGCAGT 50
TCCAGGCTGC GATAGTCGTT AACTGTTTTA CACTTAATAA AATAATTTGA 100
<210> 4001
<211> 100
<212> DNA
<213> Escherichia coli K-12 MG1655
<220> yqgb b2940
<222> 7-106
<223> yqgb b2940 U00096 complement(3083939..3084085)
<313> 4001: 57-76
<400> 4001
AATAATGTTA TGAAAAAGAA ACCGGTTGCG CAGTTGGAGC GTCAGCATTC 50
ACTGCTGGAA AATCCATGTG CTTATGGGTT GTTATCGCAG TTCCAGGCTG 100
<210> 4002
<211> 100
<212> DNA
<213> Escherichia coli K-12 MG1655
<220> ybes b0647

<222> 1329-1428
<223> ybes b0647 U00096 676638..678065
<313> 4002: 1404-1423
<400> 4002
TGACGATGGT GGGCCAGGCT GTCTGGGCAT CATTAAAATT ATTTTCTATA 50
TTTTTATCTT TGCTGGTTTG ATAGGGAAAA TACTCCATCT GTTCGGGTGA 100
<210> 4003
<211> 100
<212> DNA
<213> Escherichia coli K-12 MG1655
<220> ybes b0647
<222> 1243-1342
<223> ybes b0647 U00096 676638..678065
<313> 4003: 1302-1321
<400> 4003
ATTATTCGTC GATTATTAAT TCAGGCGGCG AATTTTACCG AGAAGCAAAC 50
TGTTGAACCG GAGCCGCAAC CGCAGCCAAT GCCCGTTGAC GATGGTGGGC 100
<210> 4004
<211> 100
<212> DNA
<213> Escherichia coli K-12 MG1655
<220> ybes b0647
<222> 1202-1301
<223> ybes b0647 U00096 676638..678065
<313> 4004: 1254-1273
<400> 4004
GCCGCCTTGA TGCAGAAAAT CGTTGTCCAC TGGAAGCATT AATTATTCGT 50
CGATTATTAA TTCAGGCGGC GAATTTTACC GAGAAGCAAA CTGTTGAACC 100
<210> 4005
<211> 100
<212> DNA
<213> Escherichia coli K-12 MG1655
<220> yqgc b2941
<222> 85-184
<223> yqgc b2941 U00096 3084206..3084421
<313> 4005: 140-159
<400> 4005
CAGCAGACAA CGGTTCATTA TTTCGTATCA CCTCCACGGC CGCCTGTTAA 50
GACGAACCCA CAAGCCAAAA CTCTGATTTC AACCCGGCTG GAAGTGGCAA 100
<210> 4006
<211> 100
<212> DNA
<213> Escherichia coli K-12 MG1655
<220> yqgc b2941
<222> 58-157
<223> yqgc b2941 U00096 3084206..3084421
<313> 4006: 97-116
<400> 4006
TTCACCAGAA CGGTGAGACA GGTTAAGCAG CAGACAACGG TTCATTATTT 50
CGTATCACCT CCACGGCCGC CTGTTAAGAC GAACCCACAA GCCAAAACTC 100
<210> 4007
<211> 100
<212> DNA
<213> Escherichia coli K-12 MG1655
<220> yqgc b2941
<222> 1-100
<223> yqgc b2941 U00096 3084206..3084421
<313> 4007: 53-72
<400> 4007
ATGGGTATAA CATCGGCAGG TATGCAAAGC AGAGATGCAG AGTGCGGGGA 50
ACGAATCTTC ACCAGAACGG TGAGACAGGT TAAGCAGCAG ACAACGGTTC 100
<210> 4008
<211> 100
<212> DNA
<213> Escherichia coli K-12 MG1655
<220> ybet b0648
<222> 456-555
<223> ybet b0648 U00096 complement(678075..678629)
<313> 4008: 518-537
<400> 4008
TAATGTTAAC CATTATGAAT CTCAAAAATG GTTAAAACTG ACCGCCAAAC 50

AACATTACAA AAATGCGGAA AGATTACTTG CCGGGCTTCC CGCACATTAA 100
<210> 4009
<211> 100
<212> DNA
<213> Escherichia coli K-12 MG1655
<220> ybet b0648
<222> 325-424
<223> ybet b0648 U00096 complement(678075..678629)
<313> 4009: 373-392
<400> 4009
ATCTACCTCA AAGGTCTGGG GGTCAAACCC GACACCCGTA AAGCAATTCT 50
CTGGTATAAG GAAGCAGCTG AACAAGGGTA TGCTCATGCT CAATATACTT 100
<210> 4010
<211> 100
<212> DNA
<213> Escherichia coli K-12 MG1655
<220> ybet b0648
<222> 186-285
<223> ybet b0648 U00096 complement(678075..678629)
<313> 4010: 247-266
<400> 4010
CTCAACGTCG CAATATCGCC TTGGAGAATT TTATCTTCAT GGTAGCGACG 50
GTAAACCACT GGATTATACA CAGGCGAGAT ACTGGTATGA GCAATCAGCG 100
<210> 4011
<211> 100
<212> DNA
<213> Escherichia coli K-12 MG1655
<220> yqgd b2942
<222> 153-252
<223> yqgd b2942 U00096 complement(3084418..3084669)
<313> 4011: 214-233
<400> 4011
AACGTGGCTG GAAATTTTTC CTGACAATGC CGGCATTCTG CGTATTTATC 50
TTTTGCAATT TTCTGCCATT GTGGGGTATA AAACGCGGCG CGCGGCTTAA 100
<210> 4012
<211> 100
<212> DNA
<213> Escherichia coli K-12 MG1655
<220> yqgd b2942
<222> 134-233
<223> yqgd b2942 U00096 complement(3084418..3084669)
<313> 4012: 203-222
<400> 4012
CGTGTCTCAT TCTGTTAAAA ACGTGGCTGG AAATTTTTCC TGACAATGCC 50
GGCATTCTGC GTATTTATCT TTTGCAATTT TCTGCCATTG TGGGGTATAA 100
<210> 4013
<211> 100
<212> DNA
<213> Escherichia coli K-12 MG1655
<220> yqgd b2942
<222> 96-195
<223> yqgd b2942 U00096 complement(3084418..3084669)
<313> 4013: 144-163
<400> 4013
AGGTCAATTC TTCACCCTAT TTCCACTTTT TTTTGAATCG TGTCTCATTC 50
TGTTAAAAAC GTGGCTGGAA ATTTTTCCTG ACAATGCCGG CATTCTGCGT 100
<210> 4014
<211> 100
<212> DNA
<213> Escherichia coli K-12 MG1655
<220> ybeu b0649
<222> 609-708
<223> ybeu b0649 U00096 678731..679438
<313> 4014: 679-698
<400> 4014
GGCAGCGGGA AGCGATCGTT TCTATGCCAC GCTTCCCTGG CAGCCATTAA 50
CTGTCCAGTC TGAATGCCCG GTAACGCTTA AGGATGTGAG CGACCTATGA 100
<210> 4015
<211> 100
<212> DNA
<213> Escherichia coli K-12 MG1655

<220> ybeu b0649
<222> 608-707
<223> ybeu b0649 U00096 678731..679438
<313> 4015: 638-657
<400> 4015
TGGCAGCGGG AAGCGATCGT TTCTATGCCA CGCTTCCCTG GCAGCCATTA  50
ACTGTCCAGT CTGAATGCCC GGTAACGCTT AAGGATGTGA GCGACCTATG  100
<210> 4016
<211> 100
<212> DNA
<213> Escherichia coli K-12 MG1655
<220> ybeu b0649
<222> 566-665
<223> ybeu b0649 U00096 678731..679438
<313> 4016: 597-616
<400> 4016
TGTACAAATA CGATAGCGCC GGGAGTCGTA TGTTCGAAGA GCTGGCAGCG  50
GGAAGCGATC GTTTCTATGC CACGCTTCCC TGGCAGCCAT TAACTGTCCA  100
<210> 4017
<211> 100
<212> DNA
<213> Escherichia coli K-12 MG1655
<220> yqge b2949
<222> 402-501
<223> yqge b2949 U00096 3090884..3091519
<313> 4017: 434-453
<400> 4017
TGTGCTGGAA ACGCTCGGCA CCGATAAACA ACCGTCTGAC GTATTGGTGG  50
CTCTGGGTTA TGCCTCCTGG GAGAAAGGTC AACTGGAACA AGAAATTCTC  100
<210> 4018
<211> 100
<212> DNA
<213> Escherichia coli K-12 MG1655
<220> yqge b2949
<222> 369-468
<223> yqge b2949 U00096 3090884..3091519
<313> 4018: 382-401
<400> 4018
TTCAGACAAC ACGGTAATGA CCACTTCCCG CGATGTGCTG GAAACGCTCG  50
GCACCGATAA ACAACCGTCT GACGTATTGG TGGCTCTGGG TTATGCCTCC  100
<210> 4019
<211> 100
<212> DNA
<213> Escherichia coli K-12 MG1655
<220> yqge b2949
<222> 316-415
<223> yqge b2949 U00096 3090884..3091519
<313> 4019: 353-372
<400> 4019
CGCGGGTTTA TTTTGCATAC TCCGCCCTCC AATTTTGCTT CCAGCATTCG  50
CATTTCAGAC AACACGGTAA TGACCACTTC CCGCGATGTG CTGGAAACGC  100
<210> 4020
<211> 100
<212> DNA
<213> Escherichia coli K-12 MG1655
<220> ybev b0650
<222> 1270-1369
<223> ybev b0650 U00096 679435..680886
<313> 4020: 1296-1315
<400> 4020
CTTTTGACGA AAGTGAAACT CGAGCCGGAG GATGAAGATG TCGATGAGCC  50
AACACCTGAA AATCATGAAG AAAAAAATGA TGAGGGTGAA AAACCACAGA  100
<210> 4021
<211> 100
<212> DNA
<213> Escherichia coli K-12 MG1655
<220> ybev b0650
<222> 1206-1305
<223> ybev b0650 U00096 679435..680886
<313> 4021: 1240-1259
<400> 4021

CCGTCTTAAT ACAAGGCTGG AAGGCGGAGA TGCTCATCCA TTAGAGGCCA 50
TTGTCACCCG GATGCTTTTG ACGAAAGTGA AACTCGAGCC GGAGGATGAA 100
<210> 4022
<211> 100
<212> DNA
<213> Escherichia coli K-12 MG1655
<220> ybev b0650
<222> 1155-1254
<223> ybev b0650 U00096 679435..680886
<313> 4022: 1204-1223
<400> 4022
GGTAGCGGAA CAGGTAGTAC TTGAAGCGTT TCCGAAACAA GAGATGCTGG 50
CCCGTCTTAA TACAAGGCTG GAAGGCGGAG ATGCTCATCC ATTAGAGGCC 100
<210> 4023
<211> 100
<212> DNA
<213> Escherichia coli K-12 MG1655
<220> yqgf b2950
<222> 268-367
<223> yqgf b2950 U00096 3091519..3091935
<313> 4023: 302-321
<400> 4023
GAAGTAAAGC TCCATGACGA GCGTCTTAGC ACTGTGGAAG CCCGTTCCGG 50
TCTGTTTGAA CAGGGCGGCT ATCGGGCGCT CAACAAAGGC AAAGTTGACT 100
<210> 4024
<211> 100
<212> DNA
<213> Escherichia coli K-12 MG1655
<220> yqgf b2950
<222> 171-270
<223> yqgf b2950 U00096 3091519..3091935
<313> 4024: 217-236
<400> 4024
CATCGTCGGT TTGCCGCTGA ATATGGACGG CACCGAGCAA CCATTGACTG 50
CCAGAGCGCG TAAATTTGCC AACCGTATTC ATGGCCGTTT CGGTGTTGAA 100
<210> 4025
<211> 100
<212> DNA
<213> Escherichia coli K-12 MG1655
<220> yqgf b2950
<222> 119-218
<223> yqgf b2950 U00096 3091519..3091935
<313> 4025: 158-177
<400> 4025
CGGACTGGAA CATTATCGAG CGTTTACTGA AAGAGTGGCA GCCGGACGAA 50
ATCATCGTCG GTTTGCCGCT GAATATGGAC GGCACCGAGC AACCATTGAC 100
<210> 4026
<211> 100
<212> DNA
<213> Escherichia coli K-12 MG1655
<220> ybew b0651
<222> 1572-1671
<223> ybew b0651 U00096 complement(680946..682616)
<313> 4026: 1648-1667
<400> 4026
GATCACCGAT TTTGATGCGG CGTTGCAGTC AAACGATATG GCCCGCGTGG 50
ATGAAGTTCG GCGGCGGGCG AGCGATTATT TAGCCATTGA GATCCCATAA 100
<210> 4027
<211> 100
<212> DNA
<213> Escherichia coli K-12 MG1655
<220> ybew b0651
<222> 1504-1603
<223> ybew b0651 U00096 complement(680946..682616)
<313> 4027: 1529-1548
<400> 4027
TTTAAAGCCA AACTGGAAGA GTTGTGGGCG CGGGCGCTGG GTGACGAGCG 50
AGAAGAGATC GGCCGGGTGA TCACCGATTT TGATGCGGCG TTGCAGTCAA 100
<210> 4028
<211> 100
<212> DNA

<213> Escherichia coli K-12 MG1655
<220> ybew b0651
<222> 1398-1497
<223> ybew b0651 U00096 complement(680946..682616)
<313> 4028: 1451-1470
<400> 4028
GATTAACCAC AGCCCGGTAA CATTGAGCGC GCAGCAGATT GAAGAGAGTC 50
GGACGCGGTT ATCCGCATTG AAAATTTATC CGCGCGATAT GCTCATCAAT 100
<210> 4029
<211> 100
<212> DNA
<213> Escherichia coli K-12 MG1655
<220> ybex b0659
<222> 780-879
<223> ybex b0659 U00096 complement(690129..691007)
<313> 4029: 855-874
<400> 4029
CGGTTACCAG TTCAAAGTGG CGATGGCCGA CAGTCGGCGT ATTATTCAGG 50
TTCATGTCAA AATCCCGGAT GACTCACCCC AGCCGAAGCT GGATGAATAA 100
<210> 4030
<211> 100
<212> DNA
<213> Escherichia coli K-12 MG1655
<220> ybex b0659
<222> 765-864
<223> ybex b0659 U00096 complement(690129..691007)
<313> 4030: 794-813
<400> 4030
AACTATCGAC ATCGACGGTT ACCAGTTCAA AGTGGCGATG GCCGACAGTC 50
GGCGTATTAT TCAGGTTCAT GTCAAAATCC CGGATGACTC ACCCCAGCCG 100
<210> 4031
<211> 100
<212> DNA
<213> Escherichia coli K-12 MG1655
<220> ybex b0659
<222> 711-810
<223> ybex b0659 U00096 complement(690129..691007)
<313> 4031: 755-774
<400> 4031
CACTATCGGT GGTCTGGTGA TGCAGGCATT TGGGCATCTT CCGGCGCGTG 50
GCGAAACTAT CGACATCGAC GGTTACCAGT TCAAAGTGGC GATGGCCGAC 100
<210> 4032
<211> 100
<212> DNA
<213> Escherichia coli K-12 MG1655
<220> ybey b0660
<222> 369-468
<223> ybey b0660 U00096 complement(691097..691564)
<313> 4032: 413-432
<400> 4032
TCACATCGAA GATGACGAAG CAGAAGAAAT GGAAGCCCTC GAAACAGAGA 50
TTATGCTTGC TCTGGGCTAT GAGGATCCGT ACATTGCCGA GAAAGAATAA 100
<210> 4033
<211> 100
<212> DNA
<213> Escherichia coli K-12 MG1655
<220> ybey b0660
<222> 337-436
<223> ybey b0660 U00096 complement(691097..691564)
<313> 4033: 382-401
<400> 4033
GTGCACGGCA GTCTGCATTT GTTAGGTTAC GATCACATCG AAGATGACGA 50
AGCAGAAGAA ATGGAAGCCC TCGAAACAGA GATTATGCTT GCTCTGGGCT 100
<210> 4034
<211> 100
<212> DNA
<213> Escherichia coli K-12 MG1655
<220> ybey b0660
<222> 281-380
<223> ybey b0660 U00096 complement(691097..691564)
<313> 4034: 304-323

<400> 4034
AGAAGGAAGC TCAGGAGCAA GGCAAACCAC TGGAGGCGCA CTGGGCGCAT 50
ATGGTGGTGC ACGGCAGTCT GCATTTGTTA GGTTACGATC ACATCGAAGA 100
<210> 4035
<211> 100
<212> DNA
<213> Escherichia coli K-12 MG1655
<220> ybez b0661
<222> 981-1080
<223> ybez b0661 U00096 complement(691561..692640)
<313> 4035: 1049-1068
<400> 4035
GCGTATCGTT AACGCCTATG AAGCCTGGGA AGAAGCCGAA CAAAAACGAA 50
AAGCGGCGCT GGCAGCAGAA CGCAAGCGCG AAGAACAGGA ACAAAAATGA 100
<210> 4036
<211> 100
<212> DNA
<213> Escherichia coli K-12 MG1655
<220> ybez b0661
<222> 951-1050
<223> ybez b0661 U00096 complement(691561..692640)
<313> 4036: 1009-1028
<400> 4036
CGAAGACGTG GTTCGTCACC CTGTGGTGGC GCGTATCGTT AACGCCTATG 50
AAGCCTGGGA AGAAGCCGAA CAAAAACGAA AAGCGGCGCT GGCAGCAGAA 100
<210> 4037
<211> 100
<212> DNA
<213> Escherichia coli K-12 MG1655
<220> ybez b0661
<222> 915-1014
<223> ybez b0661 U00096 complement(691561..692640)
<313> 4037: 941-960
<400> 4037
CGATGTCGAA GAGATCAGCT TTAACTTCTT CCACAGCGAA GACGTGGTTC 50
GTCACCCTGT GGTGGCGCGT ATCGTTAACG CCTATGAAGC CTGGGAAGAA 100
<210> 4038
<211> 100
<212> DNA
<213> Escherichia coli K-12 MG1655
<220> asla b3802
<222> 1470-1569
<223> asla b3802 U00096 complement(3981965..3983620)
<313> 4038: 1522-1541
<400> 4038
TGGATTCACC GGCACAGTAA TGCAAACGGC GGGATCGTCG GTGTTTAACC 50
TCTACACCGA TCCGCAGGAA AGCGACTCCA TCGGCGTGCG CCATATTCCG 100
<210> 4039
<211> 100
<212> DNA
<213> Escherichia coli K-12 MG1655
<220> asla b3802
<222> 1414-1513
<223> asla b3802 U00096 complement(3981965..3983620)
<313> 4039: 1439-1458
<400> 4039
AAGTATCACG TCCTGATTCA GCAACCTTAC GCTTATACCC AGAGCGGATA 50
TCAGGGTGGA TTCACCGGCA CAGTAATGCA AACGGCGGGA TCGTCGGTGT 100
<210> 4040
<211> 100
<212> DNA
<213> Escherichia coli K-12 MG1655
<220> asla b3802
<222> 1339-1438
<223> asla b3802 U00096 complement(3981965..3983620)
<313> 4040: 1376-1395
<400> 4040
AATGGTCAGT CTAACCGTAA GGCCGAGCAC TACTTCCTCA ACGGTAAACT 50
CGCTGCTGTG CGTATGGATG AGTTCAAGTA TCACGTCCTG ATTCAGCAAC 100
<210> 4041
<211> 100

<212> DNA
<213> Escherichia coli K-12 MG1655
<220> ybfa b0700
<222> 108-207
<223> ybfa b0700 U00096 728357..728563
<313> 4041: 181-200
<400> 4041
CGCCCGCTTT TACAGCTACC TGCATCGCGT CTGGTCGAAA ACCAGCGATA 50
AACCGGTGTG GATGGATCAG GCCGAAAAGG CAACTGGTGA TTTTTATTGA 100
<210> 4042
<211> 100
<212> DNA
<213> Escherichia coli K-12 MG1655
<220> ybfa b0700
<222> 84-183
<223> ybfa b0700 U00096 728357..728563
<313> 4042: 126-145
<400> 4042
TTTCATGCTT TTCTGGAAAG ACCGCGCCCG CTTTTACAGC TACCTGCATC 50
GCGTCTGGTC GAAAACCAGC GATAAACCGG TGTGGATGGA TCAGGCCGAA 100
<210> 4043
<211> 100
<212> DNA
<213> Escherichia coli K-12 MG1655
<220> ybfa b0700
<222> 77-176
<223> ybfa b0700 U00096 728357..728563
<313> 4043: 129-148
<400> 4043
CGCTGCCTTT CATGCTTTTC TGGAAAGACC GCGCCCGCTT TTACAGCTAC 50
CTGCATCGCG TCTGGTCGAA AACCAGCGAT AAACCGGTGT GGATGGATCA 100
<210> 4044
<211> 100
<212> DNA
<213> Escherichia coli K-12 MG1655
<220> aslb b3801
<222> 1085-1184
<223> aslb b3801 U00096 3980571..3981806
<313> 4044: 1148-1167
<400> 4044
GCGGCAAACC GGGACTGAAT TATTTGTGTG CCGGGTATCA GCGTTATTTC 50
CGCCATCTAC CGCCATATCT TAAAGCAATG GCTGATTTGC TGGCGCACGG 100
<210> 4045
<211> 100
<212> DNA
<213> Escherichia coli K-12 MG1655
<220> aslb b3801
<222> 1079-1178
<223> aslb b3801 U00096 3980571..3981806
<313> 4045: 1097-1116
<400> 4045
ATGCCAGCGG CAAACCGGGA CTGAATTATT TGTGTGCCGG GTATCAGCGT 50
TATTTCCGCC ATCTACCGCC ATATCTTAAA GCAATGGCTG ATTTGCTGGC 100
<210> 4046
<211> 100
<212> DNA
<213> Escherichia coli K-12 MG1655
<220> aslb b3801
<222> 1038-1137
<223> aslb b3801 U00096 3980571..3981806
<313> 4046: 1066-1085
<400> 4046
AGCGTGCTGG GGAGGCTGCC CGAAACACCG CTTCATGCTC GATGCCAGCG 50
GCAAACCGGG ACTGAATTAT TTGTGTGCCG GGTATCAGCG TTATTTCCGC 100
<210> 4047
<211> 100
<212> DNA
<213> Escherichia coli K-12 MG1655
<220> ybfb ybfB
<222> 228-327
<223> ybfb ybfB U00096 732999..733325

<313> 4047: 262-281
<400> 4047
TGATAGTAAG TATATTATGG CGATAATAAT ATGGCTTTAT TTTCTGTCAG 50
GAGGGTATAT TCAATGGTTT GTATTAAGTA AGCGCATTAT AAACAAATAA 100
<210> 4048
<211> 100
<212> DNA
<213> Escherichia coli K-12 MG1655
<220> ybfb ybfB
<222> 180-279
<223> ybfb ybfB U00096 732999..733325
<313> 4048: 215-234
<400> 4048
TGCTCTAATA TTTATAGGGA CTGTATCAGA CATTATTGGC GTAAGGATTG 50
ATAGTAAGTA TATTATGGCG ATAATAATAT GGCTTTATTT TCTGTCAGGA 100
<210> 4049
<211> 100
<212> DNA
<213> Escherichia coli K-12 MG1655
<220> ybfb ybfB
<222> 157-256
<223> ybfb ybfB U00096 732999..733325
<313> 4049: 204-223
<400> 4049
CCAACAGGCA TAGTATTCTT TATTGCTCTA ATATTTATAG GGACTGTATC 50
AGACATTATT GGCGTAAGGA TTGATAGTAA GTATATTATG GCGATAATAA 100
<210> 4050
<211> 100
<212> DNA
<213> Escherichia coli K-12 MG1655
<220> ybfc b0705
<222> 458-557
<223> ybfc b0705 U00096 734873..735442
<313> 4050: 491-510
<400> 4050
AAGATGAGAA ACCAACTTAT GCTCGAATGA CAGCACATCT CCCGAACAAA 50
AATGAGTCTT ACGAATGTTT AATAAAGATA CAGCATGAAC TAATTCGTAG 100
<210> 4051
<211> 100
<212> DNA
<213> Escherichia coli K-12 MG1655
<220> ybfc b0705
<222> 274-373
<223> ybfc b0705 U00096 734873..735442
<313> 4051: 320-339
<400> 4051
CGCCTTAATG AAAATCATGC CATCAAAGGA ACGTTGCAGG AGTTGATAAA 50
TACAGCAGAA AAAACGGCGT TTCTCGGCGA TCGGTTTAGA AGTTATATAA 100
<210> 4052
<211> 100
<212> DNA
<213> Escherichia coli K-12 MG1655
<220> ybfc b0705
<222> 257-356
<223> ybfc b0705 U00096 734873..735442
<313> 4052: 287-306
<400> 4052
TTCGCGTCGG GTCACTACGC CTTAATGAAA ATCATGCCAT CAAAGGAACG 50
TTGCAGGAGT TGATAAATAC AGCAGAAAAA ACGGCGTTTC TCGGCGATCG 100
<210> 4053
<211> 100
<212> DNA
<213> Escherichia coli K-12 MG1655
<220> ybfd b0707
<222> 663-762
<223> ybfd b0707 U00096 737315..738076
<313> 4053: 703-722
<400> 4053
CAGTTACGCA ATTAGTGAAA AGAGTCACGG CAGAGAAGAA ACCCGTCTTC 50
ATATTGTTTG CGATGTCCCT GATGAGCTTA TTGATTTCAC GTTTGAATAG 100
<210> 4054

<211> 100
<212> DNA
<213> Escherichia coli K-12 MG1655
<220> ybfd b0707
<222> 637-736
<223> ybfd b0707 U00096 737315..738076
<313> 4054: 662-681
<400> 4054
AAAGAATTAA ATAATCCCAA GCATGACAGT TACGCAATTA GTGAAAAGAG 50
TCACGGCAGA GAAGAAACCC GTCTTCATAT TGTTTGCGAT GTCCCTGATG 100
<210> 4055
<211> 100
<212> DNA
<213> Escherichia coli K-12 MG1655
<220> ybfd b0707
<222> 537-636
<223> ybfd b0707 U00096 737315..738076
<313> 4055: 589-608
<400> 4055
TATTGCAGAG AAGATACAAA AACAGGGAGG TGATTATTTA TTCGCTGTAA 50
AAGGAAACCA GGGGCGGCTA AATAAAGCCT TCGAGGAAAA ATTTCCGCTG 100
<210> 4056
<211> 100
<212> DNA
<213> Escherichia coli K-12 MG1655
<220> ybfe b0686
<222> 252-351
<223> ybfe b0686 U00096 complement(710828..711190)
<313> 4056: 304-323
<400> 4056
TGTCGAGGCG CTGAACGAGC TGGCGGAGTC GCGCAATATG AGCCGTAGCG 50
AACTGATCGA AGAGATGTTA ATGCAGCAAC TGGCGGCGCT GCGTAGTCAG 100
<210> 4057
<211> 100
<212> DNA
<213> Escherichia coli K-12 MG1655
<220> ybfe b0686
<222> 212-311
<223> ybfe b0686 U00096 complement(710828..711190)
<313> 4057: 262-281
<400> 4057
GTGGCCTTAA GCGTGTCGAA CTGAAGCTGA ACGCGGAAGC TGTCGAGGCG 50
CTGAACGAGC TGGCGGAGTC GCGCAATATG AGCCGTAGCG AACTGATCGA 100
<210> 4058
<211> 100
<212> DNA
<213> Escherichia coli K-12 MG1655
<220> ybfe b0686
<222> 190-289
<223> ybfe b0686 U00096 complement(710828..711190)
<313> 4058: 225-244
<400> 4058
CAGCTAAAAC GCGACAAAGT ACGTGGCCTT AAGCGTGTCG AACTGAAGCT 50
GAACGCGGAA GCTGTCGAGG CGCTGAACGA GCTGGCGGAG TCGCGCAATA 100
<210> 4059
<211> 100
<212> DNA
<213> Escherichia coli K-12 MG1655
<220> ybff b0687
<222> 666-765
<223> ybff b0687 U00096 complement(711261..712025)
<313> 4059: 742-761
<400> 4059
ACAGGCACGG GCGCATGTGA TTGCAGGCGC GGGTCACTGG GTCCATGCTG 50
AAAAACCGGA TGCGGTATTA CGCGCTATCC GTCGCTATCT CAATGATTAA 100
<210> 4060
<211> 100
<212> DNA
<213> Escherichia coli K-12 MG1655
<220> ybff b0687
<222> 608-707

<223> ybff b0687 U00096 complement(711261..712025)
<313> 4060: 648-667
<400> 4060
GCGGCAATTC TCCGTATGTT AGCGAGCAGT ACCGTGATGA TTTACTGGCT 50
CAATTTCCAC AGGCACGGGC GCATGTGATT GCAGGCGCGG GTCACTGGGT 100
<210> 4061
<211> 100
<212> DNA
<213> Escherichia coli K-12 MG1655
<220> ybff b0687
<222> 585-684
<223> ybff b0687 U00096 complement(711261..712025)
<313> 4061: 612-631
<400> 4061
TCACCCTGCC CTGTTTATCC CTGGCGGCAA TTCTCCGTAT GTTAGCGAGC 50
AGTACCGTGA TGATTTACTG GCTCAATTTC CACAGGCACG GGCGCATGTG 100
<210> 4062
<211> 100
<212> DNA
<213> Escherichia coli K-12 MG1655
<220> ybfg b0691
<222> 192-291
<223> ybfg b0691 U00096 complement(715170..715532)
<313> 4062: 244-263
<400> 4062
TGTACGATGG CAGCAATGGC GAGAAGACGT AATAAAATTA TCGGCAACGG 50
AAGCCTTCAC GTTCTACCCT TTCTTATGGG TACAAAGTGA AGAAGCAAGA 100
<210> 4063
<211> 100
<212> DNA
<213> Escherichia coli K-12 MG1655
<220> ybfg b0691
<222> 172-271
<223> ybfg b0691 U00096 complement(715170..715532)
<313> 4063: 200-219
<400> 4063
TTAGATACCT TCTATGAAAA TGTACGATGG CAGCAATGGC GAGAAGACGT 50
AATAAAATTA TCGGCAACGG AAGCCTTCAC GTTCTACCCT TTCTTATGGG 100
<210> 4064
<211> 100
<212> DNA
<213> Escherichia coli K-12 MG1655
<220> ybfg b0691
<222> 67-166
<223> ybfg b0691 U00096 complement(715170..715532)
<313> 4064: 117-136
<400> 4064
GGGGAAGATA CTGGCTGCGT TTATTATTTT GCGCCAGATA CGCTGAATTG 50
GGAAAGTCTG GAAGTTGGAT ATTCTGAGTT TCTTCAGTGG GCATTATCCG 100
<210> 4065
<211> 100
<212> DNA
<213> Escherichia coli K-12 MG1655
<220> ybfh b0692
<222> 219-318
<223> ybfh b0692 U00096 complement(715611..715928)
<313> 4065: 248-267
<400> 4065
TTTAGCCGAA ACCGCACTTT ATCAATTACA GGTGACAACA AAATCACCGA 50
TGGGTGCGCT TGTCTATGGC TCCGGAGGTC TGCTGATTGA TAATGGTTAG 100
<210> 4066
<211> 100
<212> DNA
<213> Escherichia coli K-12 MG1655
<220> ybfh b0692
<222> 183-282
<223> ybfh b0692 U00096 complement(715611..715928)
<313> 4066: 244-263
<400> 4066
CACAAACCAT ACCGAACTCT TACCGGTTAA TAAAGATTTA GCCGAAACCG 50
CACTTTATCA ATTACAGGTG ACAACAAAAT CACCGATGGG TGCGCTTGTC 100

<210> 4067
<211> 100
<212> DNA
<213> Escherichia coli K-12 MG1655
<220> ybfh b0692
<222> 194-293
<223> ybfh b0692 U00096 complement(715611..715928)
<313> 4067: 233-252
<400> 4067
CCGAACTCTT ACCGGTTAAT AAAGATTTAG CCGAAACCGC ACTTTATCAA 50
TTACAGGTGA CAACAAAATC ACCGATGGGT GCGCTTGTCT ATGGCTCCGG 100
<210> 4068
<211> 100
<212> DNA
<213> Escherichia coli K-12 MG1655
<220> ybfl b0706
<222> 668-767
<223> ybfl b0706 U00096 736327..737184
<313> 4068: 709-728
<400> 4068
TAATGAATGA AGACGACTGC AAAATAAGAA GAGGAAACGC CGCAGAATTA 50
TTTTCAGGGA TACGGCACAT CGCTATTAAT ATTTTAACGA ATGATAAGGT 100
<210> 4069
<211> 100
<212> DNA
<213> Escherichia coli K-12 MG1655
<220> ybfl b0706
<222> 546-645
<223> ybfl b0706 U00096 736327..737184
<313> 4069: 600-619
<400> 4069
AGAGCCAGAA ATGACGGTCA GATATTATAT CAGTTCTGCT GATTTAACCG 50
CAGAAAAGTT CGCCACAGCA ATCCGAAACC ACTGGCACGT GGAGAATAAG 100
<210> 4070
<211> 100
<212> DNA
<213> Escherichia coli K-12 MG1655
<220> ybfl b0706
<222> 457-556
<223> ybfl b0706 U00096 736327..737184
<313> 4070: 511-530
<400> 4070
GAACTTATTG ATTTCACGTT TGAATGGAAA GGACTGAAGA AATTATGCGT 50
GGCAGTCTCC TTTCGGTCAA TAATAGCAGA ACAAAAGAAA GAGCCAGAAA 100
<210> 4071
<211> 100
<212> DNA
<213> Escherichia coli K-12 MG1655
<220> b1541 b1542
<222> 105-204
<223> b1541 b1542 U00096 1627239..1627442
<313> 4071: 174-193
<400> 4071
TCTGACCGCT GAGCAAATCC GCCGCGGAAA AACCGTAGTT GTTGAAGGTT 50
GTGAAGAGAA ACTGGCACCA CTGGACCTGA TTCGTCTCGG CATGAACTAA 100
<210> 4072
<211> 100
<212> DNA
<213> Escherichia coli K-12 MG1655
<220> b1541 b1542
<222> 71-170
<223> b1541 b1542 U00096 1627239..1627442
<313> 4072: 98-117
<400> 4072
ACACTGGCAA GATCCTGTCG ATTGATACTG AAGGTCTGAC CGCTGAGCAA 50
ATCCGCCGCG GAAAAACCGT AGTTGTTGAA GGTTGTGAAG AGAAACTGGC 100
<210> 4073
<211> 100
<212> DNA
<213> Escherichia coli K-12 MG1655
<220> b1541 b1542

<222> 1-100
<223> b1541 b1542 U00096 1627239..1627442
<313> 4073: 35-54
<400> 4073
ATGACGACCT ACGATCGTAA CCGTAACGCA ATCACCACTG GCAGCCGTGT  50
TATGGTTAGC GGCACCGGTC ACACTGGCAA GATCCTGTCG ATTGATACTG  100
<210> 4074
<211> 100
<212> DNA
<213> Escherichia coli K-12 MG1655
<220> ybfm b0682
<222> 1308-1407
<223> ybfm b0682 U00096 707557..708963
<313> 4074: 1368-1387
<400> 4074
CCACTCCGAC ATCCCAAGCT GGGGCGGTGG TTACGGCAAC ATCTTCCAGG  50
ATGAGCGTGA CGTGAAATTT ATGGTAATCG CACCATTCAC CATCTTCTGA  100
<210> 4075
<211> 100
<212> DNA
<213> Escherichia coli K-12 MG1655
<220> ybfm b0682
<222> 1296-1395
<223> ybfm b0682 U00096 707557..708963
<313> 4075: 1332-1351
<400> 4075
CGAATACGAC AACCACTCCG ACATCCCAAG CTGGGGCGGT GGTTACGGCA  50
ACATCTTCCA GGATGAGCGT GACGTGAAAT TTATGGTAAT CGCACCATTC  100
<210> 4076
<211> 100
<212> DNA
<213> Escherichia coli K-12 MG1655
<220> ybfm b0682
<222> 1240-1339
<223> ybfm b0682 U00096 707557..708963
<313> 4076: 1283-1302
<400> 4076
GTCTATACCA TTCAGGACGG TCGCGCCAAA GGCACGATGT TCAAACTGCA  50
TTTCACCGAA TACGACAACC ACTCCGACAT CCCAAGCTGG GGCGGTGGTT  100
<210> 4077
<211> 100
<212> DNA
<213> Escherichia coli K-12 MG1655
<220> ybfn b0683
<222> 228-327
<223> ybfn b0683 U00096 709013..709339
<313> 4077: 282-301
<400> 4077
TTATCAGCAG TGTCTGCGTG CAACGCAAAC CGGAAATGAT CAGGCGGTGA  50
AAGCCGATTG TGATAAAGTC TGGCAGGAAA TACGCAGTAA TAACAAATAA  100
<210> 4078
<211> 100
<212> DNA
<213> Escherichia coli K-12 MG1655
<220> ybfn b0683
<222> 153-252
<223> ybfn b0683 U00096 709013..709339
<313> 4078: 181-200
<400> 4078
GAGCGTGTTA AACGTGCTGA AGAAAGAGAA ACAACATCAG CAGTTTGCCG  50
ACCAGGAAAG TGTACGCGTA CTGGATTATC AGCAGTGTCT GCGTGCAACG  100
<210> 4079
<211> 100
<212> DNA
<213> Escherichia coli K-12 MG1655
<220> ybfn b0683
<222> 108-207
<223> ybfn b0683 U00096 709013..709339
<313> 4079: 133-152
<400> 4079
TATCAACACC GCACAAGGTT CGCCAGAAAA AATTGAAGCC TGCCAGAGCG  50

TGTTAAACGT GCTGAAGAAA GAGAAACAAC ATCAGCAGTT TGCCGACCAG 100
<210> 4080
<211> 100
<212> DNA
<213> Escherichia coli K-12 MG1655
<220> b1543 b1544
<222> 1185-1284
<223> b1543 b1544 U00096 complement(1629026..1630309)
<313> 4080: 1233-1252
<400> 4080
AATGCCAGAG GTGAAAGACC GTGATTTAAG TGCGCTGGAA GACGCTGCGG 50
AAGATCAACC GCGTGTTGTA AGAGCTGCGC AACCTTCCAG AAGTCTGTAA 100
<210> 4081
<211> 100
<212> DNA
<213> Escherichia coli K-12 MG1655
<220> b1543 b1544
<222> 1145-1244
<223> b1543 b1544 U00096 complement(1629026..1630309)
<313> 4081: 1191-1210
<400> 4081
TCATGGCATA TTCAGTGATT GGTTTAATCT CTGCGCTGAA AATGCCAGAG 50
GTGAAAGACC GTGATTTAAG TGCGCTGGAA GACGCTGCGG AAGATCAACC 100
<210> 4082
<211> 100
<212> DNA
<213> Escherichia coli K-12 MG1655
<220> b1543 b1544
<222> 1052-1151
<223> b1543 b1544 U00096 complement(1629026..1630309)
<313> 4082: 1109-1128
<400> 4082
AAATTGGTGG TCTTATCGCT TCCGGTTTTG GTCCTATCCT GGCGGGTATT 50
TTCTGCACCA TGACGGAATC CTGGTATCCG ATCGCCATTA TGATCATGGC 100
<210> 4083
<211> 100
<212> DNA
<213> Escherichia coli K-12 MG1655
<220> ybfp b0690
<222> 396-495
<223> ybfp b0690 U00096 714635..715129
<313> 4083: 449-468
<400> 4083
CGGATTTTTT GGCTCGATGA TGCATGTCGG GATTATTGAG TTACGACGCT 50
GCCAGTCAGA AAATTGCCAG CAAGAATTGA AAGCGATAAA TACACATTAA 100
<210> 4084
<211> 100
<212> DNA
<213> Escherichia coli K-12 MG1655
<220> ybfp b0690
<222> 378-477
<223> ybfp b0690 U00096 714635..715129
<313> 4084: 407-426
<400> 4084
GGATTATGTC AGCGAAAGCG GATTTTTTGG CTCGATGATG CATGTCGGGA 50
TTATTGAGTT ACGACGCTGC CAGTCAGAAA ATTGCCAGCA AGAATTGAAA 100
<210> 4085
<211> 100
<212> DNA
<213> Escherichia coli K-12 MG1655
<220> ybfp b0690
<222> 336-435
<223> ybfp b0690 U00096 714635..715129
<313> 4085: 366-385
<400> 4085
GGTTTGCCAG GGCAAGCCCG GCACTCGACC GATAGTCAAT GAGGATTATG 50
TCAGCGAAAG CGGATTTTTT GGCTCGATGA TGCATGTCGG GATTATTGAG 100
<210> 4086
<211> 100
<212> DNA
<213> Escherichia coli K-12 MG1655

<220> b0816 b0816
<222> 171-270
<223> b0816 b0816 U00096 851894..852163
<313> 4086: 234-253
<400> 4086
AGGAGTGAAG CGGCGTACCG CGACGCCGCT ATTTATCGGA AGGTTTATCT 50
TGCTGCGGTT TGTTGTTGAC CATATCGCAC AACATAGAGA GCAGCATTAA 100
<210> 4087
<211> 100
<212> DNA
<213> Escherichia coli K-12 MG1655
<220> b0816 b0816
<222> 151-250
<223> b0816 b0816 U00096 851894..852163
<313> 4087: 185-204
<400> 4087
CGTTATGCCT GCATGAAAGC AGGAGTGAAG CGGCGTACCG CGACGCCGCT 50
ATTTATCGGA AGGTTTATCT TGCTGCGGTT TGTTGTTGAC CATATCGCAC 100
<210> 4088
<211> 100
<212> DNA
<213> Escherichia coli K-12 MG1655
<220> b0816 b0816
<222> 71-170
<223> b0816 b0816 U00096 851894..852163
<313> 4088: 126-145
<400> 4088
CAGGTCAACT ACTGACGAAC AAATCTTTTG CGAAGGGGAT TGCAAATCCG 50
ACAAAAAGTG GGGTTTTTCA GACCAAAACG CGTTATGCCT GCATGAAAGC 100
<210> 4089
<211> 100
<212> DNA
<213> Escherichia coli K-12 MG1655
<220> b1545 b1546
<222> 461-560
<223> b1545 b1546 U00096 1631646..1632236
<313> 4089: 478-497
<400> 4089
AAGAACAGAA ACAGGCGGTA TTCGAACGAA TTAAGTCAGG TGTAAGTATA 50
AGTGCCATTG CCCGGGAATT CAAAACCTCG CGGCAAACCA TTTTAAGAGC 100
<210> 4090
<211> 100
<212> DNA
<213> Escherichia coli K-12 MG1655
<220> b1545 b1546
<222> 454-553
<223> b1545 b1546 U00096 1631646..1632236
<313> 4090: 469-488
<400> 4090
TTAAATGAAG AACAGAAACA GGCGGTATTC GAACGAATTA AGTCAGGTGT 50
AAGTATAAGT GCCATTGCCC GGGAATTCAA AACCTCGCGG CAAACCATTT 100
<210> 4091
<211> 100
<212> DNA
<213> Escherichia coli K-12 MG1655
<220> b1545 b1546
<222> 419-518
<223> b1545 b1546 U00096 1631646..1632236
<313> 4091: 474-493
<400> 4091
GCGGCGCAGG GAAACGTTTT GGTCGACCAC CTGTGTTAAA TGAAGAACAG 50
AAACAGGCGG TATTCGAACG AATTAAGTCA GGTGTAAGTA TAAGTGCCAT 100
<210> 4092
<211> 100
<212> DNA
<213> Escherichia coli K-12 MG1655
<220> b2274 b2274
<222> 365-464
<223> b2274 b2274 U00096 2386601..2387077
<313> 4092: 413-432
<400> 4092

TTGTAGAAGA AATTGCCCCT GATGAGTGCT GGGAAGGATG GTCAGAAGAT 50
ACGGAGCATG GATGGGTTGA AGTCCCTGTG GGTATGCATC CGGTTACTTG 100
<210> 4093
<211> 100
<212> DNA
<213> Escherichia coli K-12 MG1655
<220> b2274 b2274
<222> 344-443
<223> b2274 b2274 U00096 2386601..2387077
<313> 4093: 382-401
<400> 4093
CAGAAGAAGT TCAGAGCTGG TTTGTAGAAG AAATTGCCCC TGATGAGTGC 50
TGGGAAGGAT GGTCAGAAGA TACGGAGCAT GGATGGGTTG AAGTCCCTGT 100
<210> 4094
<211> 100
<212> DNA
<213> Escherichia coli K-12 MG1655
<220> b2274 b2274
<222> 263-362
<223> b2274 b2274 U00096 2386601..2387077
<313> 4094: 298-317
<400> 4094
ATGTTTTGGT AGAAATTACG ATGTTCGATG ACCCAGACTG GCCCTTTAGT 50
GAAAGTATTT TAGTTATCAC CACTGCTTCA CCAGAAGAAG TTCAGAGCTG 100
<210> 4095
<211> 100
<212> DNA
<213> Escherichia coli K-12 MG1655
<220> b0817 b0818
<222> 369-468
<223> b0817 b0818 U00096 852406..852873
<313> 4095: 445-464
<400> 4095
GGAAATCGCC CGTCGCGACG CGGAAGGCAT GGAGCACCAT GTTAGTGAAG 50
AGACGCTCGA CGCTTTTCGT TTGTTTACCC AGAAACACGG TGCCAAATGA 100
<210> 4096
<211> 100
<212> DNA
<213> Escherichia coli K-12 MG1655
<220> b0817 b0818
<222> 292-391
<223> b0817 b0818 U00096 852406..852873
<313> 4096: 320-339
<400> 4096
GGAGAGAAGC TGGCGCAGGA AAGCCGCGAG CGACATCAGA TAGTCGAAAA 50
TTTCTTGTTG GTGTTGGGCG TCAGTCCGGA AATCGCCCGT CGCGACGCGG 100
<210> 4097
<211> 100
<212> DNA
<213> Escherichia coli K-12 MG1655
<220> b0817 b0818
<222> 226-325
<223> b0817 b0818 U00096 852406..852873
<313> 4097: 286-305
<400> 4097
AAGCGGCTGG CAACCATGGG GCTGATTGAA ATGATCCCCT GGCGTGGCGT 50
GTTTTTAACG GCAGAAGGAG AGAAGCTGGC GCAGGAAAGC CGCGAGCGAC 100
<210> 4098
<211> 100
<212> DNA
<213> Escherichia coli K-12 MG1655
<220> b2275 b2276
<222> 753-852
<223> b2275 b2276 U00096 2387133..2387984
<313> 4098: 797-816
<400> 4098
ATTTCAGGAA CGCTATGATT TTAATATCGA TGAATGGCGG GCAAAATTCA 50
GGCAGTTATG TTTAGAGTAT TTTGGCGATA GTCGTACACA GTTCACTTAA 100
<210> 4099
<211> 100
<212> DNA

<213> Escherichia coli K-12 MG1655
<220> b2275 b2276
<222> 695-794
<223> b2275 b2276 U00096 2387133..2387984
<313> 4099: 736-755
<400> 4099
TTCAGGCATT AACCCTGTTA CATAATTATG CCTGGATAAT GTCCAGTGAA 50
ACGACTGCAT TTCAGGAACG CTATGATTTT AATATCGATG AATGGCGGGC 100
<210> 4100
<211> 100
<212> DNA
<213> Escherichia coli K-12 MG1655
<220> b2275 b2276
<222> 632-731
<223> b2275 b2276 U00096 2387133..2387984
<313> 4100: 700-719
<400> 4100
AAAATAATAA TTACCCCTTG ATGCAGATGT GTGTTGATAC TTATTTTGCG 50
CAGAGAAAAC CGCTTCAGGC ATTAACCCTG TTACATAATT ATGCCTGGAT 100
<210> 4101
<211> 100
<212> DNA
<213> Escherichia coli K-12 MG1655
<220> yqha b3003
<222> 396-495
<223> yqha b3003 U00096 complement(3146992..3147486)
<313> 4101: 459-478
<400> 4101
GGATAACAAA CTGATGTGGT ACGTCATTAT CCATCTGACG TTTGTGCTTT 50
CTGCATTTGT GATGGGCTAT CTTGACCGAC TGACTCGTCA TAATCACTGA 100
<210> 4102
<211> 100
<212> DNA
<213> Escherichia coli K-12 MG1655
<220> yqha b3003
<222> 354-453
<223> yqha b3003 U00096 complement(3146992..3147486)
<313> 4102: 391-410
<400> 4102
CATTCACTTA CTGCGCGTCT TTATGGATGC GAAAAATGTC CCGGATAACA 50
AACTGATGTG GTACGTCATT ATCCATCTGA CGTTTGTGCT TTCTGCATTT 100
<210> 4103
<211> 100
<212> DNA
<213> Escherichia coli K-12 MG1655
<220> yqha b3003
<222> 289-388
<223> yqha b3003 U00096 complement(3146992..3147486)
<313> 4103: 346-365
<400> 4103
CTGGGGAAAA TGGACGCAAC GTCGCTGAAA AACAAAGTAG CAGCGTCGAT 50
TGTGGCAATT TCTTCCATTC ACTTACTGCG CGTCTTTATG GATGCGAAAA 100
<210> 4104
<211> 100
<212> DNA
<213> Escherichia coli K-12 MG1655
<220> b1547 b1548
<222> 750-849
<223> b1547 b1548 U00096 complement(1632909..1633871)
<313> 4104: 780-799
<400> 4104
TGCAGGGAAG ACATCATCAG ATGGTGCTCA TACCCACTCG CTGTCCGGCA 50
CTGCTGCAAG CGCAGGCGCG CATGCACATA CTGTCGGTAT TGGTGCTCAT 100
<210> 4105
<211> 100
<212> DNA
<213> Escherichia coli K-12 MG1655
<220> b1547 b1548
<222> 676-775
<223> b1547 b1548 U00096 complement(1632909..1633871)
<313> 4105: 708-727

<400> 4105
AATGGTTATA CCGCGATTTC AAATCTAAGC GCGGGGATTA TGAGCACAAC 50
AAGCGGTAGT GGCCAGACTC GTAATGCAGG GAAGACATCA TCAGATGGTG 100
<210> 4106
<211> 100
<212> DNA
<213> Escherichia coli K-12 MG1655
<220> b1547 b1548
<222> 621-720
<223> b1547 b1548 U00096 complement(1632909..1633871)
<313> 4106: 657-676
<400> 4106
GCACCAACAC AAGAGTTCCG GTGCATTTGG TGGCACGAAC ACGAGCATTT 50
TCCCTAATGG TTATACCGCG ATTTCAAATC TAAGCGCGGG GATTATGAGC 100
<210> 4107
<211> 100
<212> DNA
<213> Escherichia coli K-12 MG1655
<220> yqhc b3011
<222> 1029-1128
<223> yqhc b3011 U00096 complement(3152276..3153403)
<313> 4107: 1090-1109
<400> 4107
CTATGAAAGC GCATCGCAAT TTAGCCGTGA GTTTAAACGT TACTTCGGTG 50
TGACGCCGGG GGAAGATGCG GCAAGAATGC GGGCGATGCA GGGGAATTAA 100
<210> 4108
<211> 100
<212> DNA
<213> Escherichia coli K-12 MG1655
<220> yqhc b3011
<222> 953-1052
<223> yqhc b3011 U00096 complement(3152276..3153403)
<313> 4108: 983-1002
<400> 4108
ATTACCGTCT GCATAAGGCG CGGATGATGA TCATCCATGA CGGCATGAAG 50
GCCAGCGCAG CAGCGATGCG CGTCGGCTAT GAAAGCGCAT CGCAATTTAG 100
<210> 4109
<211> 100
<212> DNA
<213> Escherichia coli K-12 MG1655
<220> yqhc b3011
<222> 907-1006
<223> yqhc b3011 U00096 complement(3152276..3153403)
<313> 4109: 935-954
<400> 4109
AATTTTAAGT CTGTCACCAG TACCTCGCCG TTGCAGTATT TGAAGAATTA 50
CCGTCTGCAT AAGGCGCGGA TGATGATCAT CCATGACGGC ATGAAGGCCA 100
<210> 4110
<211> 100
<212> DNA
<213> Escherichia coli K-12 MG1655
<220> yqhd b3012
<222> 1057-1156
<223> yqhd b3012 U00096 3153369..3154532
<313> 4110: 1094-1113
<400> 4110
ATCCCGGCTT TGCTGAAAAA ACTGGAAGAG CACGGCATGA CCCAACTGGG 50
CGAAAATCAT GACATTACGT TGGATGTCAG CCGCCGTATA TACGAAGCCG 100
<210> 4111
<211> 100
<212> DNA
<213> Escherichia coli K-12 MG1655
<220> yqhd b3012
<222> 1027-1126
<223> yqhd b3012 U00096 3153369..3154532
<313> 4111: 1065-1084
<400> 4111
CTCTCCGACT ACGGTCTGGA CGGCAGCTCC ATCCCGGCTT TGCTGAAAAA 50
ACTGGAAGAG CACGGCATGA CCCAACTGGG CGAAAATCAT GACATTACGT 100
<210> 4112
<211> 100

<212> DNA
<213> Escherichia coli K-12 MG1655
<220> yqhd b3012
<222> 955-1054
<223> yqhd b3012 U00096 3153369..3154532
<313> 4112: 995-1014
<400> 4112
GATGATGAGC GTATTGACGC CGCGATTGCC GCAACCCGCA ATTTCTTTGA 50
GCAATTAGGC GTGCCGACCC ACCTCTCCGA CTACGGTCTG GACGGCAGCT 100
<210> 4113
<211> 100
<212> DNA
<213> Escherichia coli K-12 MG1655
<220> yqhe b3013
<222> 612-711
<223> yqhe b3013 U00096 3154754..3155464
<313> 4113: 676-695
<400> 4113
TGTCTGGGAT TTCCGTCTCG ACAAAGACGA ACTCGGCGAA ATTGCAAAAC 50
TCGATCAGGG CAAGCGTCTC GGTCCCGATC CTGACCAGTT CGGCGGCTAA 100
<210> 4114
<211> 100
<212> DNA
<213> Escherichia coli K-12 MG1655
<220> yqhe b3013
<222> 599-698
<223> yqhe b3013 U00096 3154754..3155464
<313> 4114: 648-667
<400> 4114
CCGAAAACTT TGATGTCTGG GATTTCCGTC TCGACAAAGA CGAACTCGGC 50
GAAATTGCAA AACTCGATCA GGGCAAGCGT CTCGGTCCCG ATCCTGACCA 100
<210> 4115
<211> 100
<212> DNA
<213> Escherichia coli K-12 MG1655
<220> yqhe b3013
<222> 550-649
<223> yqhe b3013 U00096 3154754..3155464
<313> 4115: 600-619
<400> 4115
GATAGCGGCC TGGTGGTGAT CCCGAAATCG GTCACACCTT CACGTATTGC 50
CGAAAACTTT GATGTCTGGG ATTTCCGTCT CGACAAAGAC GAACTCGGCG 100
<210> 4116
<211> 100
<212> DNA
<213> Escherichia coli K-12 MG1655
<220> yqhg b3014
<222> 831-930
<223> yqhg b3014 U00096 3155664..3156593
<313> 4116: 895-914
<400> 4116
GCGCGAAGGC AAGTGGGAAA TCGCTGATTT TATCCGCCCG AACAGCGGCA 50
GCCTACTTAA GCAGATTGAG GCAAAAACTG CCGCCAGATT AAAGCAATGA 100
<210> 4117
<211> 100
<212> DNA
<213> Escherichia coli K-12 MG1655
<220> yqhg b3014
<222> 804-903
<223> yqhg b3014 U00096 3155664..3156593
<313> 4117: 852-871
<400> 4117
CGAAGAGCAA GAACTGGTTT TACAGCGGCG CGAAGGCAAG TGGGAAATCG 50
CTGATTTTAT CCGCCCGAAC AGCGGCAGCC TACTTAAGCA GATTGAGGCA 100
<210> 4118
<211> 100
<212> DNA
<213> Escherichia coli K-12 MG1655
<220> yqhg b3014
<222> 750-849
<223> yqhg b3014 U00096 3155664..3156593

<313> 4118: 792-811
<400> 4118
AACCGCCGAT TCCGCCCGCA TTCATGTTCG TTTTACGCTG ACCAATGGCA 50
ACAACGAAGA GCAAGAACTG GTTTTACAGC GGCGCGAAGG CAAGTGGGAA 100
<210> 4119
<211> 100
<212> DNA
<213> Escherichia coli K-12 MG1655
<220> yqhh yqhH
<222> 159-258
<223> yqhh yqhH U00096 3156644..3156901
<313> 4119: 221-240
<400> 4119
AATCTATGCT GCCAAATCCG AAGCTAACAG AGCCAATACG CGTCTTGATG 50
CTCAGGACTA TTTTGATTGC CTGCGCTGCT TGCGTATGTA CGCAGAATGA 100
<210> 4120
<211> 100
<212> DNA
<213> Escherichia coli K-12 MG1655
<220> yqhh yqhH
<222> 111-210
<223> yqhh yqhH U00096 3156644..3156901
<313> 4120: 176-195
<400> 4120
TGCCAAAATC GCCCGGCTTG AGCAGGATAT GAAAGCACTA CGCCCACAAA 50
TCTATGCTGC CAAATCCGAA GCTAACAGAG CCAATACGCG TCTTGATGCT 100
<210> 4121
<211> 100
<212> DNA
<213> Escherichia coli K-12 MG1655
<220> yqhh yqhH
<222> 46-145
<223> yqhh yqhH U00096 3156644..3156901
<313> 4121: 93-112
<400> 4121
TTGCTCAGTG GCTGCGTCAA TGAGCAAAAG GTCAATCAGC TGGCGAGCAA 50
TGTGCAAACA TTAAATGCCA AAATCGCCCG GCTTGAGCAG GATATGAAAG 100
<210> 4122
<211> 100
<212> DNA
<213> Escherichia coli K-12 MG1655
<220> asma b2065
<222> 1755-1854
<223> asma b2065 U00096 complement(2137781..2139634)
<313> 4122: 1822-1841
<400> 4122
TCTACAGGAC GAAGCGAAAC GTCGCCTGAA TGACTGGGCC GAGCGGAATA 50
AAGATTCCCG TAATGGCAAA GATGTGAAGA AGTTGCTGGA GAAGATGTAG 100
<210> 4123
<211> 100
<212> DNA
<213> Escherichia coli K-12 MG1655
<220> asma b2065
<222> 1755-1854
<223> asma b2065 U00096 complement(2137781..2139634)
<313> 4123: 1787-1806
<400> 4123
TCTACAGGAC GAAGCGAAAC GTCGCCTGAA TGACTGGGCC GAGCGGAATA 50
AAGATTCCCG TAATGGCAAA GATGTGAAGA AGTTGCTGGA GAAGATGTAG 100
<210> 4124
<211> 100
<212> DNA
<213> Escherichia coli K-12 MG1655
<220> asma b2065
<222> 1690-1789
<223> asma b2065 U00096 complement(2137781..2139634)
<313> 4124: 1712-1731
<400> 4124
CGGGTTTATG GCAACTGGCA GCAACTCAAT TACAGCCTGC AAGTGGATCA 50
GTTACTGCGC AAACATCTAC AGGACGAAGC GAAACGTCGC CTGAATGACT 100
<210> 4125

<211> 100
<212> DNA
<213> Escherichia coli K-12 MG1655
<220> ybga b0708
<222> 400-499
<223> ybga b0708 U00096 738224..738733
<313> 4125: 458-477
<400> 4125
ATGCGTATCA AACACTATAT GGCGCTTTAT CCTGACGCCT GGCTTTCAGG 50
GCAGCGTTAT TTCGAACTTT GGCCGCGTGT GATTAACTTG CGCCATTCAG 100
<210> 4126
<211> 100
<212> DNA
<213> Escherichia coli K-12 MG1655
<220> ybga b0708
<222> 278-377
<223> ybga b0708 U00096 738224..738733
<313> 4126: 305-324
<400> 4126
CCAATGTTTT GATGCACGTT CAGGGTTATT TTCGCCCGCA TATTGATTCC 50
ACAGAACGCC AGCAGCTGGC TGCGCTTATC GACAGTTATC GCCGTGGCGA 100
<210> 4127
<211> 100
<212> DNA
<213> Escherichia coli K-12 MG1655
<220> ybga b0708
<222> 148-247
<223> ybga b0708 U00096 738224..738733
<313> 4127: 168-187
<400> 4127
CCGGAGTACC GCAAACTTGG CCCGTTCGTG GCTGATATTC ACCAGTGGCA 50
AAATCTGGAT GACTATTACA ACCAGTACCG CCAACGCGTA GTTGTTTTGC 100
<210> 4128
<211> 100
<212> DNA
<213> Escherichia coli K-12 MG1655
<220> ybgc b0737
<222> 306-405
<223> ybgc b0737 U00096 773975..774379
<313> 4128: 366-385
<400> 4128
TTTGCTGAAT GAAGCAGAGG TTCTGGTTGT TTGCGTTGAC CCACTCAAAA 50
TGAAGCCTCG TGCGCTTCCC AAGTCTATTG TCGCGGAGTT TAAGCAGTGA 100
<210> 4129
<211> 100
<212> DNA
<213> Escherichia coli K-12 MG1655
<220> ybgc b0737
<222> 278-377
<223> ybgc b0737 U00096 773975..774379
<313> 4129: 323-342
<400> 4129
CGCAACGTAT TGTCAACGCC GAGAATACTT TGCTGAATGA AGCAGAGGTT 50
CTGGTTGTTT GCGTTGACCC ACTCAAAATG AAGCCTCGTG CGCTTCCCAA 100
<210> 4130
<211> 100
<212> DNA
<213> Escherichia coli K-12 MG1655
<220> ybgc b0737
<222> 267-366
<223> ybgc b0737 U00096 773975..774379
<313> 4130: 287-306
<400> 4130
TTTGGTTTTC ACGCAACGTA TTGTCAACGC CGAGAATACT TTGCTGAATG 50
AAGCAGAGGT TCTGGTTGTT TGCGTTGACC CACTCAAAAT GAAGCCTCGT 100
<210> 4131
<211> 100
<212> DNA
<213> Escherichia coli K-12 MG1655
<220> ybgd b0720
<222> 468-567

<223> ybgd b0720 U00096 complement(751452..752018)
<313> 4131: 533-552
<400> 4131
GCTGAACTTT TTCGCCTGGA TGGAACAAAT TGATAATGCA GTCGATGTCA 50
CGGCAGGTGA AGTAACCGCT AACGCAACCT ACGTGCTGGA TTATAAATAA 100
<210> 4132
<211> 100
<212> DNA
<213> Escherichia coli K-12 MG1655
<220> ybgd b0720
<222> 468-567
<223> ybgd b0720 U00096 complement(751452..752018)
<313> 4132: 490-509
<400> 4132
GCTGAACTTT TTCGCCTGGA TGGAACAAAT TGATAATGCA GTCGATGTCA 50
CGGCAGGTGA AGTAACCGCT AACGCAACCT ACGTGCTGGA TTATAAATAA 100
<210> 4133
<211> 100
<212> DNA
<213> Escherichia coli K-12 MG1655
<220> ybgd b0720
<222> 401-500
<223> ybgd b0720 U00096 complement(751452..752018)
<313> 4133: 450-469
<400> 4133
ACGGTAACAT CGTATTAGGT TCAGCCGCGC CAGATCTTGA CCTGGATGCA 50
AGCTCATCAG AACAGACGCT GAACTTTTTC GCCTGGATGG AACAAATTGA 100
<210> 4134
<211> 100
<212> DNA
<213> Escherichia coli K-12 MG1655
<220> ybge b0736
<222> 195-294
<223> ybge b0736 U00096 773532..773825
<313> 4134: 274-293
<400> 4134
CGTGGGCTTT CGTCCGCAGA AGGTTCTTTG GCAAGGGATT TTTTGCCCAT 50
TGCTTGCCGA TATTGTTCTC ATTGTCGGGC TGATTTTCTT CTTCTTTTAA 100
<210> 4135
<211> 100
<212> DNA
<213> Escherichia coli K-12 MG1655
<220> ybge b0736
<222> 148-247
<223> ybge b0736 U00096 773532..773825
<313> 4135: 179-198
<400> 4135
CATGGTTTAT TGCTGATGTG GGCCGTCTGT GCTGGTGTGA TTCACGGCGT 50
GGGCTTTCGT CCGCAGAAGG TTCTTTGGCA AGGGATTTTT TGCCCATTGC 100
<210> 4136
<211> 100
<212> DNA
<213> Escherichia coli K-12 MG1655
<220> ybge b0736
<222> 92-191
<223> ybge b0736 U00096 773532..773825
<313> 4136: 118-137
<400> 4136
GTATGTTTTG GGACCCATCT CGTTTTGCCG CGAAGACCAG TGAACTGGAA 50
ATCTGGCATG GTTTATTGCT GATGTGGGCC GTCTGTGCTG GTGTGATTCA 100
<210> 4137
<211> 100
<212> DNA
<213> Escherichia coli K-12 MG1655
<220> ybgf b0743
<222> 661-760
<223> ybgf b0743 U00096 778821..779612
<313> 4137: 712-731
<400> 4137
TTTAAAGTCG GCGTCATCAT GCAGGACAAA GGTGACACCG CAAAAGCGAA 50
AGCCGTATAC CAGCAGGTTA TCAGTAAATA CCCTGGTACT GATGGCGCTA 100

<210> 4138
<211> 100
<212> DNA
<213> Escherichia coli K-12 MG1655
<220> ybgf b0743
<222> 636-735
<223> ybgf b0743 U00096 778821..779612
<313> 4138: 671-690
<400> 4138
GTCACCAAAG GCTGCAGATG CGATGTTTAA AGTCGGCGTC ATCATGCAGG 50
ACAAAGGTGA CACCGCAAAA GCGAAAGCCG TATACCAGCA GGTTATCAGT 100
<210> 4139
<211> 100
<212> DNA
<213> Escherichia coli K-12 MG1655
<220> ybgf b0743
<222> 541-640
<223> ybgf b0743 U00096 778821..779612
<313> 4139: 587-606
<400> 4139
AACGCCAATT ATTGGCTGGG TCAGTTAAAC TACAACAAGG GTAAAAAAGA 50
TGATGCGGCG TACTATTTTG CTTCGGTAGT GAAAAACTAT CCGAAGTCAC 100
<210> 4140
<211> 100
<212> DNA
<213> Escherichia coli K-12 MG1655
<220> ybgg b0733
<222> 2528-2627
<223> ybgg b0733 U00096 767201..769834
<313> 4140: 2563-2582
<400> 4140
AAACGATGAT GGATGAACAC ATTACCACCG AGGAAAATCA AGGTTCAAAT 50
CTATCGGGGC CTTTTTTACC CGGCCAGTCA CGGACGTTCA GTTACCGGCT 100
<210> 4141
<211> 100
<212> DNA
<213> Escherichia coli K-12 MG1655
<220> ybgg b0733
<222> 2476-2575
<223> ybgg b0733 U00096 767201..769834
<313> 4141: 2525-2544
<400> 4141
GCAACCTGTG ATGCGACTGT TGCTTTCAGT CGCGAGGTGA TTTCTTGCTC 50
AGAAACGATG ATGGATGAAC ACATTACCAC CGAGGAAAAT CAAGGTTCAA 100
<210> 4142
<211> 100
<212> DNA
<213> Escherichia coli K-12 MG1655
<220> ybgg b0733
<222> 2418-2517
<223> ybgg b0733 U00096 767201..769834
<313> 4142: 2466-2485
<400> 4142
TAAGAAAGCT GAAGACCGAC AAGAAGTGAT TTTACGGCTG TTTAATCCGG 50
CTGAATCAGC AACCTGTGAT GCGACTGTTG CTTTCAGTCG CGAGGTGATT 100
<210> 4143
<211> 100
<212> DNA
<213> Escherichia coli K-12 MG1655
<220> ybgh b0710
<222> 1383-1482
<223> ybgh b0710 U00096 complement(740298..741779)
<313> 4143: 1422-1441
<400> 4143
AATTACCTGG GGCGCACTGG CGTGTGTAGG ACTGGTACTG ATGATTTGGC 50
TGTATCAGGC GCTGAAATTC AGAAACCGCG CGCTGGCGCT GGAGTCTTAA 100
<210> 4144
<211> 100
<212> DNA
<213> Escherichia coli K-12 MG1655
<220> ybgh b0710

<222> 1336-1435
<223> ybgh b0710 U00096 complement(740298..741779)
<313> 4144: 1398-1417
<400> 4144
GGGGCGATCA ACTACTCCAT CAATGCATAT ATTGAAGTAT TTGATCAAAT 50
TACCTGGGGC GCACTGGCGT GTGTAGGACT GGTACTGATG ATTTGGCTGT 100
<210> 4145
<211> 100
<212> DNA
<213> Escherichia coli K-12 MG1655
<220> ybgh b0710
<222> 1260-1359
<223> ybgh b0710 U00096 complement(740298..741779)
<313> 4145: 1295-1314
<400> 4145
GCTTTCTGGC GCGATCGCGA ACTATCTGGC GGGCGTGATT GCCGATCAGA 50
CATCGCAGGC TTCGTTTGAT GCTTCCGGGG CGATCAACTA CTCCATCAAT 100
<210> 4146
<211> 100
<212> DNA
<213> Escherichia coli K-12 MG1655
<220> ybgi b0711
<222> 642-741
<223> ybgi b0711 U00096 742050..742793
<313> 4146: 698-717
<400> 4146
TCACCATGCC ACTGAACGTG GTGGTATTCG CGCATTGAGC GAGTGGCTGA 50
ATGAAAATAC CGATCTTGAT GTGACCTTTA TTGATATTCC TAATCCTGCA 100
<210> 4147
<211> 100
<212> DNA
<213> Escherichia coli K-12 MG1655
<220> ybgi b0711
<222> 546-645
<223> ybgi b0711 U00096 742050..742793
<313> 4147: 602-621
<400> 4147
CGCGCGTTTT GGCGTGGATG CTTTTATTAC TGGCGAAGTT TCTGAACAGA 50
CCATTCATTC AGCCCGCGAG CAGGGATTGC ATTTTTATGC TGCAGGTCAC 100
<210> 4148
<211> 100
<212> DNA
<213> Escherichia coli K-12 MG1655
<220> ybgi b0711
<222> 352-451
<223> ybgi b0711 U00096 742050..742793
<313> 4148: 404-423
<400> 4148
ATCACGGTCA TGGGCGAAAT TGAGCCGTTG GTGCCGTGGG GCGAACTGAC 50
CATGCCTGTG CCGGGACTGG AGCTGGCTTC CTGGATTGAA GCGCGTCTGG 100
<210> 4149
<211> 100
<212> DNA
<213> Escherichia coli K-12 MG1655
<220> ybgj b0712
<222> 558-657
<223> ybgj b0712 U00096 742816..743472
<313> 4149: 592-611
<400> 4149
TACCTCACTC AGCCTGTTTG ATCCGGCGCG TGACGAACCC ATCTTATTAC 50
GTCCGGGAGA CAGCGTGCGC TTTGTACCGC AGAAGGAGGG AGTATGCTGA 100
<210> 4150
<211> 100
<212> DNA
<213> Escherichia coli K-12 MG1655
<220> ybgj b0712
<222> 489-588
<223> ybgj b0712 U00096 742816..743472
<313> 4150: 514-533
<400> 4150
CGGGATCGGC GGGCCGCAGA CTGGTGTTTA TCCGCTGGCA ACGCCGGGTG 50

GCTGGCAGTT GATTGGTCAT ACCTCACTCA GCCTGTTTGA TCCGGCGCGT 100
<210> 4151
<211> 100
<212> DNA
<213> Escherichia coli K-12 MG1655
<220> ybgj b0712
<222> 300-399
<223> ybgj b0712 U00096 742816..743472
<313> 4151: 348-367
<400> 4151
GGATTTGGCG GTGGTCGCGG CGCATTGCGG GTTGAGCGAA AAACAGGTTG 50
TTGAATTGCA CTCCTCCGTG GAATACGTGG TCTGGTTTTT AGGTTTTCAA 100
<210> 4152
<211> 100
<212> DNA
<213> Escherichia coli K-12 MG1655
<220> ybgk b0713
<222> 834-933
<223> ybgk b0713 U00096 743466..744398
<313> 4152: 883-902
<400> 4152
TCATTTTGTC CAGTGTTCAC TGGAAGAGGC ACTAAAAGCA CGGCAAGATC 50
AGCAACGTTA TTTTGAACAA TTAGCGTGGC GGCTGCACAA TGAAAATTGA 100
<210> 4153
<211> 100
<212> DNA
<213> Escherichia coli K-12 MG1655
<220> ybgk b0713
<222> 784-883
<223> ybgk b0713 U00096 743466..744398
<313> 4153: 818-837
<400> 4153
ATTGAGGCTG ATATGTACCA TCTGGCGCAA ATTCCGCTCG GTCAGCCGAT 50
TCATTTTGTC CAGTGTTCAC TGGAAGAGGC ACTAAAAGCA CGGCAAGATC 100
<210> 4154
<211> 100
<212> DNA
<213> Escherichia coli K-12 MG1655
<220> ybgk b0713
<222> 731-830
<223> ybgk b0713 U00096 743466..744398
<313> 4154: 785-804
<400> 4154
TGTTGATGAA CGACGCACAG ACCACCGGTG GTTACCCGCG TATTGCCTGT 50
ATCATTGAGG CTGATATGTA CCATCTGGCG CAAATTCCGC TCGGTCAGCC 100
<210> 4155
<211> 100
<212> DNA
<213> Escherichia coli K-12 MG1655
<220> b1550 b1550
<222> 78-177
<223> b1550 b1550 U00096 complement(1635633..1635809)
<313> 4155: 122-141
<400> 4155
TAAGAAAAAG ACCGGGAAAG AAGTTACCAG TATTCAGTTT ACCGCACGGG 50
AAAAAATGAC GGGTCTTGAA AGCTATGATG TCAAGATTAA TTTAATCTGA 100
<210> 4156
<211> 100
<212> DNA
<213> Escherichia coli K-12 MG1655
<220> b1550 b1550
<222> 66-165
<223> b1550 b1550 U00096 complement(1635633..1635809)
<313> 4156: 124-143
<400> 4156
AATTATTGAA CTTAAGAAAA AGACCGGGAA AGAAGTTACC AGTATTCAGT 50
TTACCGCACG GGAAAAAATG ACGGGTCTTG AAAGCTATGA TGTCAAGATT 100
<210> 4157
<211> 100
<212> DNA
<213> Escherichia coli K-12 MG1655

<220> b1550 b1550
<222> 75-174
<223> b1550 b1550 U00096 complement(1635633..1635809)
<313> 4157: 103-122
<400> 4157
ACTTAAGAAA AAGACCGGGA AAGAAGTTAC CAGTATTCAG TTTACCGCAC  50
GGGAAAAAAT GACGGGTCTT GAAAGCTATG ATGTCAAGAT TAATTTAATC  100
<210> 4158
<211> 100
<212> DNA
<213> Escherichia coli K-12 MG1655
<220> ybgl b0714
<222> 636-735
<223> ybgl b0714 U00096 744388..745122
<313> 4158: 698-717
<400> 4158
GCAAACGGTC TGCCTGCATG GCGACGGCGA GCACGCACTG GCGTTCGCCC  50
GCCGACTACG CTCTGCATTT GCCGAAAAGG GGATTGTTGT CGCAGCATAA  100
<210> 4159
<211> 100
<212> DNA
<213> Escherichia coli K-12 MG1655
<220> ybgl b0714
<222> 590-689
<223> ybgl b0714 U00096 744388..745122
<313> 4159: 645-664
<400> 4159
ACGGCAGAGT CAAAAGTATC ACCGGCGAAT GGGCAACGGT CGCGGCGCAA  50
ACGGTCTGCC TGCATGGCGA CGGCGAGCAC GCACTGGCGT TCGCCCGCCG  100
<210> 4160
<211> 100
<212> DNA
<213> Escherichia coli K-12 MG1655
<220> ybgl b0714
<222> 580-679
<223> ybgl b0714 U00096 744388..745122
<313> 4160: 610-629
<400> 4160
ATGGTGCAAC ACGGCAGAGT CAAAAGTATC ACCGGCGAAT GGGCAACGGT  50
CGCGGCGCAA ACGGTCTGCC TGCATGGCGA CGGCGAGCAC GCACTGGCGT  100
<210> 4161
<211> 100
<212> DNA
<213> Escherichia coli K-12 MG1655
<220> b0822 b0823
<222> 661-760
<223> b0822 b0823 U00096 complement(858436..859251)
<313> 4161: 711-730
<400> 4161
GAGATGCTGA AAATGGCGCG TTATTCCTTT GCGATGGGCA ATGCTGCGGA  50
AAACATTAAA CAAATCGCCC GTTACGCTAC CGATGATAAT AATCATGAAG  100
<210> 4162
<211> 100
<212> DNA
<213> Escherichia coli K-12 MG1655
<220> b0822 b0823
<222> 624-723
<223> b0822 b0823 U00096 complement(858436..859251)
<313> 4162: 647-666
<400> 4162
AAATGTGGTA GCGATTGGCG ACAGCGGTAA CGATGCGGAG ATGCTGAAAA  50
TGGCGCGTTA TTCCTTTGCG ATGGGCAATG CTGCGGAAAA CATTAAACAA  100
<210> 4163
<211> 100
<212> DNA
<213> Escherichia coli K-12 MG1655
<220> b0822 b0823
<222> 541-640
<223> b0822 b0823 U00096 complement(858436..859251)
<313> 4163: 596-615
<400> 4163

GGCTTTATCG ACCTGATTAT TCCCGGTCTA CATAAAGCAA ACGGTATTTC 50
GCGGTTACTG AAACGCTGGG ATCTGTCACC GCAAAATGTG GTAGCGATTG 100
<210> 4164
<211> 100
<212> DNA
<213> Escherichia coli K-12 MG1655
<220> b1551 b1552
<222> 30-129
<223> b1551 b1552 U00096 complement(1635978..1636169)
<313> 4164: 71-90
<400> 4164
CACCATCATG CGTGAATATC CAAATGGCGA AAAAACACAC CTTACTGTAA 50
TGGCCGCAGG GTTTCCATCT CTGACCGGAG ATCATAAAGT CATTTATGTA 100
<210> 4165
<211> 100
<212> DNA
<213> Escherichia coli K-12 MG1655
<220> b1551 b1552
<222> 47-146
<223> b1551 b1552 U00096 complement(1635978..1636169)
<313> 4165: 58-77
<400> 4165
ATCCAAATGG CGAAAAAACA CACCTTACTG TAATGGCCGC AGGGTTTCCA 50
TCTCTGACCG GAGATCATAA AGTCATTTAT GTAGCCGCGG ATCGACATGT 100
<210> 4166
<211> 100
<212> DNA
<213> Escherichia coli K-12 MG1655
<220> b1551 b1552
<222> 14-113
<223> b1551 b1552 U00096 complement(1635978..1636169)
<313> 4166: 56-75
<400> 4166
CGATGACTGG AGTTAACACC ATCATGCGTG AATATCCAAA TGGCGAAAAA 50
ACACACCTTA CTGTAATGGC CGCAGGGTTT CCATCTCTGA CCGGAGATCA 100
<210> 4167
<211> 100
<212> DNA
<213> Escherichia coli K-12 MG1655
<220> b1553 b1553
<222> 399-498
<223> b1553 b1553 U00096 complement(1637054..1637551)
<313> 4167: 440-459
<400> 4167
AGCTCAGCTT TATGCTGAAA TTGCTGACGA ACGCTACATC GCAGGAGTGA 50
CTTGTCAACA GATCTATGAA TCTTTAAGAG ATAAAAAGCA TCAAATGTAG 100
<210> 4168
<211> 100
<212> DNA
<213> Escherichia coli K-12 MG1655
<220> b1553 b1553
<222> 355-454
<223> b1553 b1553 U00096 complement(1637054..1637551)
<313> 4168: 392-411
<400> 4168
GCCGAAGGAA TGCTCACCAA CATGCTCGGA GATATTGCAG CAGAAGCTCA 50
GCTTTATGCT GAAATTGCTG ACGAACGCTA CATCGCAGGA GTGACTTGTC 100
<210> 4169
<211> 100
<212> DNA
<213> Escherichia coli K-12 MG1655
<220> b1553 b1553
<222> 320-419
<223> b1553 b1553 U00096 complement(1637054..1637551)
<313> 4169: 353-372
<400> 4169
TTGCCGCTGC CGTCAGAGGC AAAACAACCA AAACCGCCGA AGGAATGCTC 50
ACCAACATGC TCGGAGATAT TGCAGCAGAA GCTCAGCTTT ATGCTGAAAT 100
<210> 4170
<211> 100
<212> DNA

<213> Escherichia coli K-12 MG1655
<220> ybgo b0717
<222> 990-1089
<223> ybgo b0717 U00096 complement(747144..748232)
<313> 4170: 1060-1079
<400> 4170
CGAAAAAACC TATACGGCTG AACTTTCGTC CATTCCAGGC ACCCCCATCG 50
AGGCTGGCCC CTTCGATACA GTGGTGCTTT TTAAGATTAA CTATAACTGA 100
<210> 4171
<211> 100
<212> DNA
<213> Escherichia coli K-12 MG1655
<220> ybgo b0717
<222> 967-1066
<223> ybgo b0717 U00096 complement(747144..748232)
<313> 4171: 1004-1023
<400> 4171
TTCACCAGTG ACATGTTGGT CTACGAAAAA ACCTATACGG CTGAACTTTC 50
GTCCATTCCA GGCACCCCCA TCGAGGCTGG CCCCTTCGAT ACAGTGGTGC 100
<210> 4172
<211> 100
<212> DNA
<213> Escherichia coli K-12 MG1655
<220> ybgo b0717
<222> 887-986
<223> ybgo b0717 U00096 complement(747144..748232)
<313> 4172: 926-945
<400> 4172
TTGGTAATGG TCTGAAGCTA CGTTTACTGG ATGAAAACGC CTCGCCTTAT 50
ACCTTCAATA AATATGCTGA ATACGCCGAT TTCACCAGTG ACATGTTGGT 100
<210> 4173
<211> 100
<212> DNA
<213> Escherichia coli K-12 MG1655
<220> b1554 b1554
<222> 435-534
<223> b1554 b1554 U00096 complement(1637548..1638081)
<313> 4173: 508-527
<400> 4173
TGGCGGACGC GATTGCCGCA TTCGTTCAAA TAACTGTTAC GGTCAGGTTA 50
TTCGTCGTGA CCAGGAGAGC GCATTAACCT GCTGGGGGAT AGAACAGTGA 100
<210> 4174
<211> 100
<212> DNA
<213> Escherichia coli K-12 MG1655
<220> b1554 b1554
<222> 406-505
<223> b1554 b1554 U00096 complement(1637548..1638081)
<313> 4174: 453-472
<400> 4174
TGCGAAGCGA TTCGCTGGTG GATTAAGGAT GGCGGACGCG ATTGCCGCAT 50
TCGTTCAAAT AACTGTTACG GTCAGGTTAT TCGTCGTGAC CAGGAGAGCG 100
<210> 4175
<211> 100
<212> DNA
<213> Escherichia coli K-12 MG1655
<220> b1554 b1554
<222> 353-452
<223> b1554 b1554 U00096 complement(1637548..1638081)
<313> 4175: 366-385
<400> 4175
GTTTCCCGTC GACGTTTTAT AAGCGGCTGA ATGCTGGTGA TCGTAAAGGT 50
GCATGCGAAG CGATTCGCTG GTGGATTAAG GATGGCGGAC GCGATTGCCG 100
<210> 4176
<211> 100
<212> DNA
<213> Escherichia coli K-12 MG1655
<220> ybgp b0718
<222> 630-729
<223> ybgp b0718 U00096 complement(748202..748930)
<313> 4176: 710-729

<400> 4176
CCAGTTTTAT CTCGGTTACA TGGATGATTA CGGCGCATTA CGCATGACGA 50
CGCTCAACTG CAGCGGACAA TGCCGTTTAC AAGCAGTGGA GGCGAAATGA 100
<210> 4177
<211> 100
<212> DNA
<213> Escherichia coli K-12 MG1655
<220> ybgp b0718
<222> 613-712
<223> ybgp b0718 U00096 complement(748202..748930)
<313> 4177: 652-671
<400> 4177
GGAAGTTATA GCGGCAGCCA GTTTTATCTC GGTTACATGG ATGATTACGG 50
CGCATTACGC ATGACGACGC TCAACTGCAG CGGACAATGC CGTTTACAAG 100
<210> 4178
<211> 100
<212> DNA
<213> Escherichia coli K-12 MG1655
<220> ybgp b0718
<222> 559-658
<223> ybgp b0718 U00096 complement(748202..748930)
<313> 4178: 599-618
<400> 4178
CCAGGCTTCA AAACCGTGAT GGTTGCACCT TTTAGTACGG TCAACACAAA 50
CACCGGAAGT TATAGCGGCA GCCAGTTTTA TCTCGGTTAC ATGGATGATT 100
<210> 4179
<211> 100
<212> DNA
<213> Escherichia coli K-12 MG1655
<220> b1555 b1555
<222> 213-312
<223> b1555 b1555 U00096 complement(1638078..1638389)
<313> 4179: 277-296
<400> 4179
ATTGTCAGAA AAAGTTGAGT CTTATCTGCA GCATCAGTAC TCCTTTAAGA 50
TTGAAAATCC TGCCAATAAG CACGAGCGTC CTCATCATAA ATATCTATGA 100
<210> 4180
<211> 100
<212> DNA
<213> Escherichia coli K-12 MG1655
<220> b1555 b1555
<222> 136-235
<223> b1555 b1555 U00096 complement(1638078..1638389)
<313> 4180: 190-209
<400> 4180
GAAGGAGCTG CGCTGGAGTT TTATGAGGCA GCAGCCAGAA GGAGCGTCCG 50
GCAAGTCTTC CTGGAAGTAG CAGAAAAATT GTCAGAAAAA GTTGAGTCTT 100
<210> 4181
<211> 100
<212> DNA
<213> Escherichia coli K-12 MG1655
<220> b1555 b1555
<222> 50-149
<223> b1555 b1555 U00096 complement(1638078..1638389)
<313> 4181: 98-117
<400> 4181
ATAAAGTTAC GGTAACTGTA GCCTTACAGG ACAAAGAACG CTTTGACGGT 50
GAAATTTTTG ACCTGGATGT CGCCATGGAC CGTGTTGAAG GAGCTGCGCT 100
<210> 4182
<211> 100
<212> DNA
<213> Escherichia coli K-12 MG1655
<220> ybgq b0719
<222> 2358-2457
<223> ybgq b0719 U00096 complement(748945..751401)
<313> 4182: 2423-2442
<400> 4182
TCAACTGTTT ACCGTGGTCT GGGGTGAGCA AAGCTGCATT ATTCATCTGC 50
CAGAACGTCT GGAAGACACG ACCAAACGCC TGATTTTACC TTGTCATTAA 100
<210> 4183
<211> 100

<212> DNA
<213> Escherichia coli K-12 MG1655
<220> ybgq b0719
<222> 2346-2445
<223> ybgq b0719 U00096 complement(748945..751401)
<313> 4183: 2389-2408
<400> 4183
TGTCGCTGAA AATCAACTGT TTACCGTGGT CTGGGGTGAG CAAAGCTGCA 50
TTATTCATCT GCCAGAACGT CTGGAAGACA CGACCAAACG CCTGATTTTA 100
<210> 4184
<211> 100
<212> DNA
<213> Escherichia coli K-12 MG1655
<220> ybgq b0719
<222> 2278-2377
<223> ybgq b0719 U00096 complement(748945..751401)
<313> 4184: 2314-2333
<400> 4184
ATCCGCCAGG ATGACAGCGG CATTAGCGTG GGTATGGTTG GCGAGGAAGG 50
ACATGCCTGG TTAAGCGGTG TCGCTGAAAA TCAACTGTTT ACCGTGGTCT 100
<210> 4185
<211> 100
<212> DNA
<213> Escherichia coli K-12 MG1655
<220> b1556 b1557
<222> 192-291
<223> b1556 b1557 U00096 complement(1638394..1638684)
<313> 4185: 261-280
<400> 4185
CGGTGTGCTG GGTAGCCTGG TTTTTGGCCT GCTGACGTAT CTGACAAATC 50
TTTATTTCAA GATTAAAGAA GACAGGCGTA AGGCTGCGAG AGGAGAGTAA 100
<210> 4186
<211> 100
<212> DNA
<213> Escherichia coli K-12 MG1655
<220> b1556 b1557
<222> 181-280
<223> b1556 b1557 U00096 complement(1638394..1638684)
<313> 4186: 209-228
<400> 4186
TGGGCTGCAA TCGGTGTGCT GGGTAGCCTG GTTTTTGGCC TGCTGACGTA 50
TCTGACAAAT CTTTATTTCA AGATTAAAGA AGACAGGCGT AAGGCTGCGA 100
<210> 4187
<211> 100
<212> DNA
<213> Escherichia coli K-12 MG1655
<220> b1556 b1557
<222> 148-247
<223> b1556 b1557 U00096 complement(1638394..1638684)
<313> 4187: 160-179
<400> 4187
TTGCAGTTAC TCGATAAAGT AACTCCGTCA CAGTGGGCTG CAATCGGTGT 50
GCTGGGTAGC CTGGTTTTTG GCCTGCTGAC GTATCTGACA AATCTTTATT 100
<210> 4188
<211> 100
<212> DNA
<213> Escherichia coli K-12 MG1655
<220> yqia b3032
<222> 454-553
<223> yqia b3032 U00096 complement(3173441..3174022)
<313> 4188: 496-515
<400> 4188
TACCGCCAGG CGGTGGCGTA CTACGCTTCC TGCCGCCAGA CTGTCATAGA 50
AGGCGGCAAC CACGCATTCA CGGGCTTCGA AGATTATTTC AACCCGATCG 100
<210> 4189
<211> 100
<212> DNA
<213> Escherichia coli K-12 MG1655
<220> yqia b3032
<222> 399-498
<223> yqia b3032 U00096 complement(3173441..3174022)

<313> 4189: 438-457
<400> 4189
GGAAGCGCCG GATTTGATCT GGCTGCTGCA ACAGACGGGA GATGAAGTGC 50
TGGATTACCG CCAGGCGGTG GCGTACTACG CTTCCTGCCG CCAGACTGTC 100
<210> 4190
<211> 100
<212> DNA
<213> Escherichia coli K-12 MG1655
<220> yqia b3032
<222> 337-436
<223> yqia b3032 U00096 complement(3173441..3174022)
<313> 4190: 385-404
<400> 4190
CAGCAATATG TGCTAGAGTC ACGCCATATT TACGATCTTA AAGTCATGCA 50
GATTGACCCG CTGGAAGCGC CGGATTTGAT CTGGCTGCTG CAACAGACGG 100
<210> 4191
<211> 100
<212> DNA
<213> Escherichia coli K-12 MG1655
<220> ybgr ybgR
<222> 843-942
<223> ybgr ybgR U00096 complement(783105..784046)
<313> 4191: 880-899
<400> 4191
GATTGAGCAC GCCACCATTC AGATGGAATA TCAACCTTGT CATGGGCCAG 50
ACTGCCACCT GAATGAAGGG GTGTCTGGCC ATTCACATCA TCACCATTAA 100
<210> 4192
<211> 100
<212> DNA
<213> Escherichia coli K-12 MG1655
<220> ybgr ybgR
<222> 783-882
<223> ybgr ybgR U00096 complement(783105..784046)
<313> 4192: 848-867
<400> 4192
GCCACACGAT CACGATGCCT TGTTGGATCA GATCCAACAC TACCTGATGG 50
ATCACTATCA GATTGAGCAC GCCACCATTC AGATGGAATA TCAACCTTGT 100
<210> 4193
<211> 100
<212> DNA
<213> Escherichia coli K-12 MG1655
<220> ybgr ybgR
<222> 712-811
<223> ybgr ybgR U00096 complement(783105..784046)
<313> 4193: 743-762
<400> 4193
AATGTGCACC ATGTACATGT ATGGATGGTA GGCGAGAAGC CGGTGATGAC 50
GTTGCATGTG CAGGTGATCC CGCCACACGA TCACGATGCC TTGTTGGATC 100
<210> 4194
<211> 100
<212> DNA
<213> Escherichia coli K-12 MG1655
<220> yqib b3034
<222> 324-423
<223> yqib b3034 U00096 complement(3174874..3175296)
<313> 4194: 386-405
<400> 4194
GTTGCATCAA CGCGACGAAA AGCATCAAAT TAATCAGTTT TTAGCGGACT 50
GGTTGCGATA CTGTTTAGCA CATGGAGCGA TGGCGATTCC GGTTTATTAG 100
<210> 4195
<211> 100
<212> DNA
<213> Escherichia coli K-12 MG1655
<220> yqib b3034
<222> 228-327
<223> yqib b3034 U00096 complement(3174874..3175296)
<313> 4195: 295-314
<400> 4195
GACGGTGCGT CTGTATCATG ACGCGATGGT GGCTGAAGTG TGTTCAAGCC 50
AGCAGATTTT TCGCTTCAAA GCGCGGTATG ATTATCCTAA TAAAAAGTTG 100
<210> 4196

<211> 100
<212> DNA
<213> Escherichia coli K-12 MG1655
<220> yqib b3034
<222> 217-316
<223> yqib b3034 U00096 complement(3174874..3175296)
<313> 4196: 250-269
<400> 4196
CTTCCGTCAA TGACGGTGCG TCTGTATCAT GACGCGATGG TGGCTGAAGT 50
GTGTTCAAGC CAGCAGATTT TTCGCTTCAA AGCGCGGTAT GATTATCCTA 100
<210> 4197
<211> 100
<212> DNA
<213> Escherichia coli K-12 MG1655
<220> b0829 b0829
<222> 1740-1839
<223> b0829 b0829 U00096 866776..868614
<313> 4197: 1798-1817
<400> 4197
TCTGCGTGGC GAAGAGGTGG CAGCCGTCTC GTTGCAATGC GTCGGGCCGG 50
GGCATTACGT CGCACAACCA CAATCAGAAT ACGCATTCAT GCGTAGATAA 100
<210> 4198
<211> 100
<212> DNA
<213> Escherichia coli K-12 MG1655
<220> b0829 b0829
<222> 1661-1760
<223> b0829 b0829 U00096 866776..868614
<313> 4198: 1717-1736
<400> 4198
CGGCAGTTCC GGTCGCTGAA CCGTCCCGAC AACGACCGCA GCGTGTACTG 50
CTGTCGGACG ATCTTCCCAG CAATATTCAT CTGCGTGGCG AAGAGGTGGC 100
<210> 4199
<211> 100
<212> DNA
<213> Escherichia coli K-12 MG1655
<220> b0829 b0829
<222> 1507-1606
<223> b0829 b0829 U00096 866776..868614
<313> 4199: 1532-1551
<400> 4199
GGCATTGCGT ATCTGTTTAT CTCCCACGAT ATGGCGGTGG TAGAGCGGAT 50
TAGTCATCGT GTGGCGGTGA TGTATCTCGG GCAAATTGTT GAAATTGGTC 100
<210> 4200
<211> 100
<212> DNA
<213> Escherichia coli K-12 MG1655
<220> b1559 b1559
<222> 684-783
<223> b1559 b1559 U00096 complement(1640513..1641295)
<313> 4200: 760-779
<400> 4200
GTGGTATAAG GGGTATGCGG ACGTCATTGG TAAACTGGTA ACAAAGTGCT 50
GGCAGGAAGA AGCATACGCG GAAGCGCAAT TGAGGAAGGT GACGAGATAA 100
<210> 4201
<211> 100
<212> DNA
<213> Escherichia coli K-12 MG1655
<220> b1559 b1559
<222> 652-751
<223> b1559 b1559 U00096 complement(1640513..1641295)
<313> 4201: 680-699
<400> 4201
CAGAAGCTGG TACCAGACCT GACCGATTAT CAGTGGTATA AGGGGTATGC 50
GGACGTCATT GGTAAACTGG TAACAAAGTG CTGGCAGGAA GAAGCATACG 100
<210> 4202
<211> 100
<212> DNA
<213> Escherichia coli K-12 MG1655
<220> b1559 b1559
<222> 494-593

<223> b1559 b1559 U00096 complement(1640513..1641295)
<313> 4202: 547-566
<400> 4202
GCGAAGCCAG CACAGCGTGC AGAGGGTGTA AGGGTAAAGG GATTGTTCTG 50
GATGAAAAAA GAACCCGGTT TCATGGCGTA CCGGTATATA AGATTTGTGG 100
<210> 4203
<211> 100
<212> DNA
<213> Escherichia coli K-12 MG1655
<220> yqie b3035
<222> 520-619
<223> yqie b3035 U00096 complement(3175297..3175926)
<313> 4203: 555-574
<400> 4203
GAACAGGCAT ACCAGTGGGT AGAAGAGGGG AAAATCGACA ACGCAGCGTC 50
GGTCATCGCT TTGCAATGGC TGCAGCTGCA TCATCAAGCG TTAAAAAATG 100
<210> 4204
<211> 100
<212> DNA
<213> Escherichia coli K-12 MG1655
<220> yqie b3035
<222> 470-569
<223> yqie b3035 U00096 complement(3175297..3175926)
<313> 4204: 522-541
<400> 4204
ACGGTCTGGC TGATGAAAAC GAAGATATTC GCGTTCATGT GGTAAGCCGG 50
GAACAGGCAT ACCAGTGGGT AGAAGAGGGG AAAATCGACA ACGCAGCGTC 100
<210> 4205
<211> 100
<212> DNA
<213> Escherichia coli K-12 MG1655
<220> yqie b3035
<222> 454-553
<223> yqie b3035 U00096 complement(3175297..3175926)
<313> 4205: 471-490
<400> 4205
ACCGCAAGCG GTATTCACGG TCTGGCTGAT GAAAACGAAG ATATTCGCGT 50
TCATGTGGTA AGCCGGGAAC AGGCATACCA GTGGGTAGAA GAGGGGAAAA 100
<210> 4206
<211> 100
<212> DNA
<213> Escherichia coli K-12 MG1655
<220> yqig b3047
<222> 2367-2466
<223> yqig b3047 U00096 3185416..3187881
<313> 4206: 2437-2456
<400> 4206
TGCTTTACGC TGGGGTGACA AATCTTGTTT TATTCAACCA CCCAATAGCA 50
GCAACTTAAC CACCGGAACG GCTATTTTAC CGTGTATTAG CCAAAATTAA 100
<210> 4207
<211> 100
<212> DNA
<213> Escherichia coli K-12 MG1655
<220> yqig b3047
<222> 2355-2454
<223> yqig b3047 U00096 3185416..3187881
<313> 4207: 2390-2409
<400> 4207
TGATAACAAA CTTGCTTTAC GCTGGGGTGA CAAATCTTGT TTTATTCAAC 50
CACCCAATAG CAGCAACTTA ACCACCGGAA CGGCTATTTT ACCGTGTATT 100
<210> 4208
<211> 100
<212> DNA
<213> Escherichia coli K-12 MG1655
<220> yqig b3047
<222> 2288-2387
<223> yqig b3047 U00096 3185416..3187881
<313> 4208: 2325-2344
<400> 4208
AAACCAGCCA CAAAGAATTA GGACTGGTTG CTGATGGCGG CTTTGTATAC 50
CTCAACGGCA TTCAGGATGA TAACAAACTT GCTTTACGCT GGGGTGACAA 100

<210> 4209
<211> 100
<212> DNA
<213> Escherichia coli K-12 MG1655
<220> yqih b3048
<222> 508-607
<223> yqih b3048 U00096 3187888..3188646
<313> 4209: 546-565
<400> 4209
ATCCGCTTTA ACAATAAGAT GCCTTATCAC GTTACGCTGG GTTATATCGG 50
AACAAATGGG GTAACGATGT TACCACAAAC GCAAAGCTTA ATGGTAACCC 100
<210> 4210
<211> 100
<212> DNA
<213> Escherichia coli K-12 MG1655
<220> yqih b3048
<222> 408-507
<223> yqih b3048 U00096 3187888..3188646
<313> 4210: 439-458
<400> 4210
CGCATTGAAA CTATTCTGGC GGCCAAAAGC CATTGAATTA GAAGACGATG 50
GGGTAATGAC TTACGAAAAA GTTGAAATCA TTCGTAGAAA TGACGGTTCG 100
<210> 4211
<211> 100
<212> DNA
<213> Escherichia coli K-12 MG1655
<220> yqih b3048
<222> 361-460
<223> yqih b3048 U00096 3187888..3188646
<313> 4211: 402-421
<400> 4211
CCGCCCAAAA CAGATAAAAA AAATGTGATG CAAGTCACTA TGCAGCACGC 50
ATTGAAACTA TTCTGGCGGC CAAAAGCCAT TGAATTAGAA GACGATGGGG 100
<210> 4212
<211> 100
<212> DNA
<213> Escherichia coli K-12 MG1655
<220> yqii b3049
<222> 966-1065
<223> yqii b3049 U00096 3188648..3189712
<313> 4212: 1023-1042
<400> 4212
CACCGCACCA TTCAGGGCAG AACTGAGTGC GAACCCGGCA GAAACGGTTG 50
TTGAGGGACC GTTTAGTAAA GACGTAATCC TGAAAATCAC CTATAACTAG 100
<210> 4213
<211> 100
<212> DNA
<213> Escherichia coli K-12 MG1655
<220> yqii b3049
<222> 937-1036
<223> yqii b3049 U00096 3188648..3189712
<313> 4213: 954-973
<400> 4213
TCTTTTTCAT CGTCTAGTGC GCCTTCGGTC ACCGCACCAT TCAGGGCAGA 50
ACTGAGTGCG AACCCGGCAG AAACGGTTGT TGAGGGACCG TTTAGTAAAG 100
<210> 4214
<211> 100
<212> DNA
<213> Escherichia coli K-12 MG1655
<220> yqii b3049
<222> 856-955
<223> yqii b3049 U00096 3188648..3189712
<313> 4214: 917-936
<400> 4214
GATATAGGTA ATGGTCTGCT ATTGAAGATA TTTCATAACG ATGGAACAGC 50
AACGGAATTT AACCGCTTCT CACAATTTGC TTCTTTTTCA TCGTCTAGTG 100
<210> 4215
<211> 100
<212> DNA
<213> Escherichia coli K-12 MG1655
<220> asna b3745

<222> 893-992
<223> asna b3745 U00096 3924783..3925775
<313> 4215: 922-941
<400> 4215
CTCGTTTGAC TATGCTGCTG CTGCAACTGC CGCATATCGG CCAGGTTCAG 50
TGTGGAGTAT GGCCAGCTGC TGTTCGCGAG AGCGTCCCTT CTCTGCTGTA 100
<210> 4216
<211> 100
<212> DNA
<213> Escherichia coli K-12 MG1655
<220> asna b3745
<222> 620-719
<223> asna b3745 U00096 3924783..3925775
<313> 4216: 665-684
<400> 4216
ATGGTCATCG CCACGACGTG CGCGCACCGG ATTATGATGA CTGGAGCACC 50
CCGTCAGAGC TGGGCCATGC GGGTCTGAAC GGCGATATTC TGGTGTGGAA 100
<210> 4217
<211> 100
<212> DNA
<213> Escherichia coli K-12 MG1655
<220> asna b3745
<222> 510-609
<223> asna b3745 U00096 3924783..3925775
<313> 4217: 540-559
<400> 4217
CCAGGAGTTA CTGTCTCGTT ATCCGGATCT TGATGCCAAA GGGCGTGAGC 50
GGGCGATAGC GAAAGATCTT GGCGCGGTAT TCCTTGTCGG GATTGGCGGC 100
<210> 4218
<211> 100
<212> DNA
<213> Escherichia coli K-12 MG1655
<220> ybha b0767
<222> 720-819
<223> ybha b0767 U00096 complement(796836..797654)
<313> 4218: 792-811
<400> 4218
GGGCAACGCC GATGACGCGG TAAAAGCGCG CGCCAACATT GTGATTGGTG 50
ATAACACCAC CGACAGCATT GCCCAGTTCA TTTATAGCCA CCTGATTTAA 100
<210> 4219
<211> 100
<212> DNA
<213> Escherichia coli K-12 MG1655
<220> ybha b0767
<222> 678-777
<223> ybha b0767 U00096 complement(796836..797654)
<313> 4219: 715-734
<400> 4219
TGATATCAGT ATGCTGGAAG CCGCTGGTAC AGGCGTGGCG ATGGGCAACG 50
CCGATGACGC GGTAAAAGCG CGCGCCAACA TTGTGATTGG TGATAACACC 100
<210> 4220
<211> 100
<212> DNA
<213> Escherichia coli K-12 MG1655
<220> ybha b0767
<222> 536-635
<223> ybha b0767 U00096 complement(796836..797654)
<313> 4220: 586-605
<400> 4220
TGGGACTGGA GTGTGAATGG TCCTGGCACG ATCAGGTTGA TATTGCACGC 50
GGCGGCAACA GCAAAGGTAA ACGTTTGACG AAATGGGTTG AGGCGCAAGG 100
<210> 4221
<211> 100
<212> DNA
<213> Escherichia coli K-12 MG1655
<220> asnb b0675
<222> 1557-1656
<223> asnb b0675 U00096 complement(696736..698400)
<313> 4221: 1609-1628
<400> 4221
TTCCGTCGCT TGTTCTTCCG CTAAAGCGAT CGAATGGGAT GAAGCGTTCA 50

AGAAAATGGA CGATCCGTCT GGTCGCGCGG TTGGTGTTCA CCAGTCGGCG 100
<210> 4222
<211> 100
<212> DNA
<213> Escherichia coli K-12 MG1655
<220> asnb b0675
<222> 1440-1539
<223> asnb b0675 U00096 complement(696736..698400)
<313> 4222: 1464-1483
<400> 4222
TGCCCGCTTC CGCTTCCCGT ACAACACGCC AACCTCTAAA GAAGCGTACT 50
TGTATCGGGA GATCTTTGAA GAACTATTCC CGCTTCCGAG CGCCGCTGAG 100
<210> 4223
<211> 100
<212> DNA
<213> Escherichia coli K-12 MG1655
<220> asnb b0675
<222> 1409-1508
<223> asnb b0675 U00096 complement(696736..698400)
<313> 4223: 1430-1449
<400> 4223
CGCAGCAGGT TTCTGATCAG CAACTGGAAA CTGCCCGCTT CCGCTTCCCG 50
TACAACACGC CAACCTCTAA AGAAGCGTAC TTGTATCGGG AGATCTTTGA 100
<210> 4224
<211> 100
<212> DNA
<213> Escherichia coli K-12 MG1655
<220> ybhb b0774
<222> 378-477
<223> ybhb b0774 U00096 complement(806656..807132)
<313> 4224: 439-458
<400> 4224
ACGTATTGAT GTCGATGAAG GTGCCAGCGG CGCGATGGTC GGGTTTAACG 50
TTCATTTCCA CTCTCTGGCA AGCGCCTCGA TTACTGCGAT GTTTAGTTAA 100
<210> 4225
<211> 100
<212> DNA
<213> Escherichia coli K-12 MG1655
<220> ybhb b0774
<222> 350-449
<223> ybhb b0774 U00096 complement(806656..807132)
<313> 4225: 405-424
<400> 4225
TTTTTACCGT TCACGCGCTG GATATAGAAC GTATTGATGT CGATGAAGGT 50
GCCAGCGGCG CGATGGTCGG GTTTAACGTT CATTTCCACT CTCTGGCAAG 100
<210> 4226
<211> 100
<212> DNA
<213> Escherichia coli K-12 MG1655
<220> ybhb b0774
<222> 304-403
<223> ybhb b0774 U00096 complement(806656..807132)
<313> 4226: 344-363
<400> 4226
GGGTACGATG GCGCAGCACC GCCGAAAGGC GAAACTCATC GCTACATTTT 50
TACCGTTCAC GCGCTGGATA TAGAACGTAT TGATGTCGAT GAAGGTGCCA 100
<210> 4227
<211> 100
<212> DNA
<213> Escherichia coli K-12 MG1655
<220> asnc b3744
<222> 360-459
<223> asnc b3744 U00096 complement(3924173..3924631)
<313> 4227: 434-453
<400> 4227
GCATGTACTT ATCAACAAGA TCCAAACAAT TGATGAAATT CAGTCCACCG 50
AGACATTGAT CGTCCTGCAA AACCCGATCA TGCGTACCAT CAAGCCCTGA 100
<210> 4228
<211> 100
<212> DNA
<213> Escherichia coli K-12 MG1655

<220> asnc b3744
<222> 319-418
<223> asnc b3744 U00096 complement(3924173..3924631)
<313> 4228: 361-380
<400> 4228
ATCTTTATAA AAGTGATGTG CCGTTCGATC GACGCTCTCC AGCATGTACT 50
TATCAACAAG ATCCAAACAA TTGATGAAAT TCAGTCCACC GAGACATTGA 100
<210> 4229
<211> 100
<212> DNA
<213> Escherichia coli K-12 MG1655
<220> asnc b3744
<222> 269-368
<223> asnc b3744 U00096 complement(3924173..3924631)
<313> 4229: 293-312
<400> 4229
AAAGCCTTGA TGAAGTCACT GAAGCCTATT ACACAACCGG CCACTACAGC 50
ATCTTTATAA AAGTGATGTG CCGTTCGATC GACGCTCTCC AGCATGTACT 100
<210> 4230
<211> 100
<212> DNA
<213> Escherichia coli K-12 MG1655
<220> cmta b2934
<222> 1266-1365
<223> cmta b2934 U00096 complement(3075490..3076878)
<313> 4230: 1284-1303
<400> 4230
TGCCAGTCTG GAAGGGCGCG TGAAACGTGT GACGGATAAA CCACTGATAT 50
TGATTAATAA CTATATTGGC GATCCAAAAC TCGACACTTT ATTTAATCAA 100
<210> 4231
<211> 100
<212> DNA
<213> Escherichia coli K-12 MG1655
<220> cmta b2934
<222> 1213-1312
<223> cmta b2934 U00096 complement(3075490..3076878)
<313> 4231: 1252-1271
<400> 4231
AAACATTACG CCATAGAAAA CGTGCCTGCG GATGCGGATA TCGTCGTTAC 50
TCATGCCAGT CTGGAAGGGC GCGTGAAACG TGTGACGGAT AAACCACTGA 100
<210> 4232
<211> 100
<212> DNA
<213> Escherichia coli K-12 MG1655
<220> cmta b2934
<222> 1155-1254
<223> cmta b2934 U00096 complement(3075490..3076878)
<313> 4232: 1208-1227
<400> 4232
GATGGGCGCG ACCACCTTCC GTAAACGCCT GGAAAAAGCG GGCTGGCAA 50
TTGAAGTAAA ACATTACGCC ATAGAAAACG TGCCTGCGGA TGCGGATATC 100
<210> 4233
<211> 100
<212> DNA
<213> Escherichia coli K-12 MG1655
<220> ybhc b0773
<222> 1185-1284
<223> ybhc b0773 U00096 complement(805221..806504)
<313> 4233: 1262-1281
<400> 4233
CGAAATACAG CGCAATCTGA ATGACACTAA CTACAACCGC ATGTGGGAAT 50
ACAATAACCG CGGCGTGGGT AGTAAAGTGG TTGCAGAGGC GAAGAAGTAA 100
<210> 4234
<211> 100
<212> DNA
<213> Escherichia coli K-12 MG1655
<220> ybhc b0773
<222> 1156-1255
<223> ybhc b0773 U00096 complement(805221..806504)
<313> 4234: 1173-1192
<400> 4234

GGTAATACCG GCAGCGTAGA TGATAACGAC GAAATACAGC GCAATCTGAA 50
TGACACTAAC TACAACCGCA TGTGGGAATA CAATAACCGC GGCGTGGGTA 100
<210> 4235
<211> 100
<212> DNA
<213> Escherichia coli K-12 MG1655
<220> ybhc b0773
<222> 1102-1201
<223> ybhc b0773 U00096 complement(805221..806504)
<313> 4235: 1126-1145
<400> 4235
TTTAACACGG CTAAACCGTG GGCCGATGCG GTGATCTCTA ATCGTCCGTT 50
TGCGGGTAAT ACCGGCAGCG TAGATGATAA CGACGAAATA CAGCGCAATC 100
<210> 4236
<211> 100
<212> DNA
<213> Escherichia coli K-12 MG1655
<220> cmtb b2935
<222> 299-398
<223> cmtb b2935 U00096 complement(3076906..3077349)
<313> 4236: 355-374
<400> 4236
TCATCGGACT CTCTGCTGCC GATGCCGATT CGCACATTGG CGCTATTCAG 50
GCGTTAAGTG AATTACTGTG CGAAGAAGAA ATACTCGAAC AACTCTTAAC 100
<210> 4237
<211> 100
<212> DNA
<213> Escherichia coli K-12 MG1655
<220> cmtb b2935
<222> 233-332
<223> cmtb b2935 U00096 complement(3076906..3077349)
<313> 4237: 298-317
<400> 4237
GGATGTCTTT GACATTACTT GAACAAGGTG TTTATTTTCC GGGGAATGAC 50
GAACCAATTA AATTACTCAT CGGACTCTCT GCTGCCGATG CCGATTCGCA 100
<210> 4238
<211> 100
<212> DNA
<213> Escherichia coli K-12 MG1655
<220> cmtb b2935
<222> 156-255
<223> cmtb b2935 U00096 complement(3076906..3077349)
<313> 4238: 187-206
<400> 4238
CAATGGCCCT TATTATATTC TCGCACCAGG CGTGGCAATG CCTCATGCGC 50
GACCGGAATG TGGGGCGCTT AAAACCGGGA TGTCTTTGAC ATTACTTGAA 100
<210> 4239
<211> 100
<212> DNA
<213> Escherichia coli K-12 MG1655
<220> ybhd b0769
<222> 918-1017
<223> ybhd b0769 U00096 complement(798845..799861)
<313> 4239: 960-979
<400> 4239
CTTTTTTGCC TTGCTGATTA ATTTATGGAC TGACGATAAA GACAATTTGT 50
GGACCAATAT CGTCGAACGC CAGAGACACG CGTTACAGGA GATAGGTTAA 100
<210> 4240
<211> 100
<212> DNA
<213> Escherichia coli K-12 MG1655
<220> ybhd b0769
<222> 876-975
<223> ybhd b0769 U00096 complement(798845..799861)
<313> 4240: 908-927
<400> 4240
AATTCGCCGT AAAGATGCCG TACTTTCTCC AGCAGCAGAA CGCTTTTTTG 50
CCTTGCTGAT TAATTTATGG ACTGACGATA AAGACAATTT GTGGACCAAT 100
<210> 4241
<211> 100
<212> DNA

<213> Escherichia coli K-12 MG1655
<220> ybhd b0769
<222> 829-928
<223> ybhd b0769 U00096 complement(798845..799861)
<313> 4241: 870-889
<400> 4241
ATTGGTATCC CATTGGTGGA ACCCGTTATA CGTCGAACAT TGGGAATAAT 50
TCGCCGTAAA GATGCCGTAC TTTCTCCAGC AGCAGAACGC TTTTTTGCCT 100
<210> 4242
<211> 100
<212> DNA
<213> Escherichia coli K-12 MG1655
<220> ybhe b0768
<222> 897-996
<223> ybhe b0768 U00096 797809..798804
<313> 4242: 969-988
<400> 4242
CTCGGTATAC GAAATTGTTG GCGAGCAGGG GCTACTGCAT GAAAAAGGCC 50
GCTATGCGGT CGGGCAGGGA CCAATGTGGG TGGTGGTTAA CGCACACTAA 100
<210> 4243
<211> 100
<212> DNA
<213> Escherichia coli K-12 MG1655
<220> ybhe b0768
<222> 892-991
<223> ybhe b0768 U00096 797809..798804
<313> 4243: 922-941
<400> 4243
CACATCTCGG TATACGAAAT TGTTGGCGAG CAGGGGCTAC TGCATGAAAA 50
AGGCCGCTAT GCGGTCGGGC AGGGACCAAT GTGGGTGGTG GTTAACGCAC 100
<210> 4244
<211> 100
<212> DNA
<213> Escherichia coli K-12 MG1655
<220> ybhe b0768
<222> 853-952
<223> ybhe b0768 U00096 797809..798804
<313> 4244: 885-904
<400> 4244
AGCGGCAAGT ATCTGATTGC CGCCGGGCAA AAATCTCACC ACATCTCGGT 50
ATACGAAATT GTTGGCGAGC AGGGGCTACT GCATGAAAAA GGCCGCTATG 100
<210> 4245
<211> 100
<212> DNA
<213> Escherichia coli K-12 MG1655
<220> ybhf ybhF
<222> 1653-1752
<223> ybhf ybhF U00096 complement(826468..828219)
<313> 4245: 1731-1750
<400> 4245
GAAAGCACAG TCGGCTAACG ATGAGCAACC CGATCCCACC ATGGAGCAAG 50
CCTTTATTCA GTTGATCCAC GACTGGGATA AGGAGCATAG CAATGAGTAA 100
<210> 4246
<211> 100
<212> DNA
<213> Escherichia coli K-12 MG1655
<220> ybhf ybhF
<222> 1582-1681
<223> ybhf ybhF U00096 complement(826468..828219)
<313> 4246: 1641-1660
<400> 4246
GCGGAATATT GCGACCGCAT CGGCCTGGTG TACCGCGGGA AATTAATCGC 50
CAGCGGCACG CCGGACGATT TGAAAGCACA GTCGGCTAAC GATGAGCAAC 100
<210> 4247
<211> 100
<212> DNA
<213> Escherichia coli K-12 MG1655
<220> ybhf ybhF
<222> 1544-1643
<223> ybhf ybhF U00096 complement(826468..828219)
<313> 4247: 1577-1596

<400> 4247
GCGTCACGGT GATGGTCACC ACCCACTTTA TGGATGAAGC GGAATATTGC 50
GACCGCATCG GCCTGGTGTA CCGCGGGAAA TTAATCGCCA GCGGCACGCC 100
<210> 4248
<211> 100
<212> DNA
<213> Escherichia coli K-12 MG1655
<220> ybhh b0770
<222> 954-1053
<223> ybhh b0770 U00096 799982..801034
<313> 4248: 1003-1022
<400> 4248
CGTTCATTTA AGTAATGAAG GTCAGGATGC CACGACGTTA CGCGCATCTG 50
TTATTCGGAC GACCAGAAAA ATATTTTCCG GTGAAGTTTA TCTTCCCTGA 100
<210> 4249
<211> 100
<212> DNA
<213> Escherichia coli K-12 MG1655
<220> ybhh b0770
<222> 914-1013
<223> ybhh b0770 U00096 799982..801034
<313> 4249: 952-971
<400> 4249
ACGGCAATAT CAATATTGAA CACCCCAGTG GTGCGCTCGA CGTTCATTTA 50
AGTAATGAAG GTCAGGATGC CACGACGTTA CGCGCATCTG TTATTCGGAC 100
<210> 4250
<211> 100
<212> DNA
<213> Escherichia coli K-12 MG1655
<220> ybhh b0770
<222> 846-945
<223> ybhh b0770 U00096 799982..801034
<313> 4250: 882-901
<400> 4250
TATTGCTATT TCCAGTAGTT GTGCATTGGA AGGCACCGTC ACCCGACAAA 50
TCGTCCCTTC TGTAGGATAC GGCAATATCA ATATTGAACA CCCCAGTGGT 100
<210> 4251
<211> 100
<212> DNA
<213> Escherichia coli K-12 MG1655
<220> ybhi b0771
<222> 1335-1434
<223> ybhi b0771 U00096 801110..802543
<313> 4251: 1361-1380
<400> 4251
GTGGTTGGTC GGTGCGGTAC TGACGATATT AACCTTCCTG GTGCATATCA 50
CCCTCGGCGT GTGGTGGTGG AATATGCTGA TCGGCTGGAA CATGCTGTAA 100
<210> 4252
<211> 100
<212> DNA
<213> Escherichia coli K-12 MG1655
<220> ybhi b0771
<222> 1246-1345
<223> ybhi b0771 U00096 801110..802543
<313> 4252: 1294-1313
<400> 4252
TCCAACTCCT ATGGCGGCAT GGTTACTCAC TATGGCGGCG CGGCAGGTCC 50
GGTCATCTTT GGCGTGGGTT ATAACGATAT TAAATCCTGG TGGTTGGTCG 100
<210> 4253
<211> 100
<212> DNA
<213> Escherichia coli K-12 MG1655
<220> ybhi b0771
<222> 1221-1320
<223> ybhi b0771 U00096 801110..802543
<313> 4253: 1240-1259
<400> 4253
GTTAACCGCG CTGGCACTGT TGTTCTCCAA CTCCTATGGC GGCATGGTTA 50
CTCACTATGG CGGCGCGGCA GGTCCGGTCA TCTTTGGCGT GGGTTATAAC 100
<210> 4254
<211> 100

<212> DNA
<213> Escherichia coli K-12 MG1655
<220> ybhj b0772
<222> 2187-2286
<223> ybhj b0772 U00096 802702..804987
<313> 4254: 2238-2257
<400> 4254
AACGGAAATT ACGCTCTATA TGGAAAGTCT GACTGCTGAA GAGCGCGAGA 50
TTATCAAGGC GGGTAGTTTG ATTAACTTCA ATAAAAACCG TCAGATGTAA 100
<210> 4255
<211> 100
<212> DNA
<213> Escherichia coli K-12 MG1655
<220> ybhj b0772
<222> 2135-2234
<223> ybhj b0772 U00096 802702..804987
<313> 4255: 2175-2194
<400> 4255
ATAATCCGGG TACGACGTTT AAAGGTTATG TGATCCATGA AGATGCGCCG 50
GTAACGGAAA TTACGCTCTA TATGGAAAGT CTGACTGCTG AAGAGCGCGA 100
<210> 4256
<211> 100
<212> DNA
<213> Escherichia coli K-12 MG1655
<220> ybhj b0772
<222> 2054-2153
<223> ybhj b0772 U00096 802702..804987
<313> 4256: 2101-2120
<400> 4256
TGTTACCGCT GCAAATGGCG GAAGTACCAA CCTTTGAAGT GGGGGATTAC 50
ATTTACATCC CTGGCATTAA AGCGGCGCTG GATAATCCGG GTACGACGTT 100
<210> 4257
<211> 100
<212> DNA
<213> Escherichia coli K-12 MG1655
<220> b0830 b0830
<222> 1436-1535
<223> b0830 b0830 U00096 868634..870172
<313> 4257: 1496-1515
<400> 4257
CGCTGGTGGT AGAAAAACTG GTGTCGGCAC ACAGTAAAAA CCTGACCGGT 50
TTTTGGATCA TGCCAGACAC CGGCTTCAGC TTTGAAGACG CGGATTTGCA 100
<210> 4258
<211> 100
<212> DNA
<213> Escherichia coli K-12 MG1655
<220> b0830 b0830
<222> 1310-1409
<223> b0830 b0830 U00096 868634..870172
<313> 4258: 1365-1384
<400> 4258
CGTTTTACAG CAATAAACAG GTGGATGACT TCCTGGCTCA GGCACTGAAA 50
ACTAATGATC CGGCGGAAAA GACCCGCTTA TATAAGGCGG CGCAGGATAT 100
<210> 4259
<211> 100
<212> DNA
<213> Escherichia coli K-12 MG1655
<220> b0830 b0830
<222> 1266-1365
<223> b0830 b0830 U00096 868634..870172
<313> 4259: 1329-1348
<400> 4259
GCTGTTTGCC TCGCAGAACT GGCCACCGAC GCTGTTTAAT ACCGCGTTTT 50
ACAGCAATAA ACAGGTGGAT GACTTCCTGG CTCAGGCACT GAAAACTAAT 100
<210> 4260
<211> 100
<212> DNA
<213> Escherichia coli K-12 MG1655
<220> ybhk b0781
<222> 776-875
<223> ybhk b0781 U00096 complement(814962..815870)

<313> 4260: 825-844
<400> 4260
CAAAAGTGGA TGTCTCGGCG GTGAAAGAGC GGATTGTGAT CCAGGAGGTA 50
CTGGAGGCCA GCGATATTCC GTATCGTCAT GACCGCCAGT TGTTACATAA 100
<210> 4261
<211> 100
<212> DNA
<213> Escherichia coli K-12 MG1655
<220> ybhk b0781
<222> 753-852
<223> ybhk b0781 U00096 complement(814962..815870)
<313> 4261: 785-804
<400> 4261
CATTGATGCG GTCATCGTCG GGCCAAAAGT GGATGTCTCG GCGGTGAAAG 50
AGCGGATTGT GATCCAGGAG GTACTGGAGG CCAGCGATAT TCCGTATCGT 100
<210> 4262
<211> 100
<212> DNA
<213> Escherichia coli K-12 MG1655
<220> ybhk b0781
<222> 662-761
<223> ybhk b0781 U00096 complement(814962..815870)
<313> 4262: 709-728
<400> 4262
GCAATCTGGG GCGTGAGTTG AGTTTACCTG CGGCTAATTT GAAGCTGGAA 50
AGCAAGCTGG CAATTATGGA GCAGTATGTT GGTAAAAAGG TCATTGATGC 100
<210> 4263
<211> 100
<212> DNA
<213> Escherichia coli K-12 MG1655
<220> b0831 b0831
<222> 822-921
<223> b0831 b0831 U00096 870190..871110
<313> 4263: 882-901
<400> 4263
TCAGGCGGAA ATTCTGCTTT TCTCGCTGGA ATTTATTCTT ATCAACTTAG 50
TGGTGGATGT GCTTTACGCC GCCATTAACC CGGCTATCAG GTACAAGTAA 100
<210> 4264
<211> 100
<212> DNA
<213> Escherichia coli K-12 MG1655
<220> b0831 b0831
<222> 736-835
<223> b0831 b0831 U00096 870190..871110
<313> 4264: 774-793
<400> 4264
TCCATCGTTG TGGAGAAAGT TTTCAACTGG CCGGGACTTG GACGCTTACT 50
CGTTGACTCC GTAGAAATGC GTGATTACCC GGTGATTCAG GCGGAAATTC 100
<210> 4265
<211> 100
<212> DNA
<213> Escherichia coli K-12 MG1655
<220> b0831 b0831
<222> 658-757
<223> b0831 b0831 U00096 870190..871110
<313> 4265: 705-724
<400> 4265
CTCAAACACG GGCTACGTAA CGCGATGATC CCGGTAGTGA CCATGATGGG 50
CTTACAGTTT GGCTTTTTGC TCGGTGGTTC CATCGTTGTG GAGAAAGTTT 100
<210> 4266
<211> 100
<212> DNA
<213> Escherichia coli K-12 MG1655
<220> b1560 b1561
<222> 958-1057
<223> b1560 b1561 U00096 complement(1641279..1642367)
<313> 4266: 998-1017
<400> 4266
TTGCCTTTGT GCAGAAAGCA TCACAACGAG CTGCATACGG ATACAGTGGC 50
ATTTGAAGAT AAGTATGGCT CCCAACTGGA GCTGATATTT CGTTTTATCG 100
<210> 4267

<211> 100
<212> DNA
<213> Escherichia coli K-12 MG1655
<220> b1560 b1561
<222> 893-992
<223> b1560 b1561 U00096 complement(1641279..1642367)
<313> 4267: 938-957
<400> 4267
ATCCGCATCA TCTGATTGGT CACGGGCAGG GCGGAATGGG AACAAAAGCA 50
CATGATCTCT TTGTGTTGCC TTTGTGCAGA AAGCATCACA ACGAGCTGCA 100
<210> 4268
<211> 100
<212> DNA
<213> Escherichia coli K-12 MG1655
<220> b1560 b1561
<222> 791-890
<223> b1560 b1561 U00096 complement(1641279..1642367)
<313> 4268: 833-852
<400> 4268
AATCTTTCAT GAAGCTGCCT AAACGTCGCC GCTGGGTTAA GGAGAAATAC 50
ACACGTTGGG TTAAGACACA GCCGTGTGCT TGCTGCGGTA TGCCAGCCGA 100
<210> 4269
<211> 100
<212> DNA
<213> Escherichia coli K-12 MG1655
<220> ybhl b0787
<222> 606-705
<223> ybhl b0787 U00096 819107..819811
<313> 4269: 667-686
<400> 4269
GAACCTGCGC AAATATTCCA TTCTTGGCGC GTTAACCTTG TATCTGGACT 50
TCATCAACCT GTTCCTGATG TTGTTGCGGA TCTTCGGCAA CCGCCGTTAA 100
<210> 4270
<211> 100
<212> DNA
<213> Escherichia coli K-12 MG1655
<220> ybhl b0787
<222> 536-635
<223> ybhl b0787 U00096 819107..819811
<313> 4270: 589-608
<400> 4270
TCGGATTGAC GGCGTATGAC ACGCAGAAAC TGAAAAATAT GGGTGAGCAG 50
ATTGATACCC GCGACACGTC GAACCTGCGC AAATATTCCA TTCTTGGCGC 100
<210> 4271
<211> 100
<212> DNA
<213> Escherichia coli K-12 MG1655
<220> ybhl b0787
<222> 491-590
<223> ybhl b0787 U00096 819107..819811
<313> 4271: 548-567
<400> 4271
AAGCATTGAT GTGGGCAGTT ACCTACATCG GCGTGATTGT CTTTGTCGGA 50
TTGACGGCGT ATGACACGCA GAAACTGAAA AATATGGGTG AGCAGATTGA 100
<210> 4272
<211> 100
<212> DNA
<213> Escherichia coli K-12 MG1655
<220> b0832 b0832
<222> 780-879
<223> b0832 b0832 U00096 871113..872024
<313> 4272: 844-863
<400> 4272
GGCTCGAGCG GATATGGTTA TCGCGCCGCA TGTCGCTGTT TTTCCGGCCC 50
TGGCTATTTT TCTGACCGTA CTGGCGTTCA ATTTGTTGGG CGATGGTTTA 100
<210> 4273
<211> 100
<212> DNA
<213> Escherichia coli K-12 MG1655
<220> b0832 b0832
<222> 745-844

<223> b0832 b0832 U00096 871113..872024
<313> 4273: 799-818
<400> 4273
CCGCCGACAC CAGAGTGGGG AGCAATGCTC AATGAGGCTC GAGCGGATAT 50
GGTTATCGCG CCGCATGTCG CTGTTTTTCC GGCCCTGGCT ATTTTTCTGA 100
<210> 4274
<211> 100
<212> DNA
<213> Escherichia coli K-12 MG1655
<220> b0832 b0832
<222> 718-817
<223> b0832 b0832 U00096 871113..872024
<313> 4274: 762-781
<400> 4274
CTCTCATTTC TCGGCCTCGG TGCGCAGCCG CCGACACCAG AGTGGGGAGC 50
AATGCTCAAT GAGGCTCGAG CGGATATGGT TATCGCGCCG CATGTCGCTG 100
<210> 4275
<211> 100
<212> DNA
<213> Escherichia coli K-12 MG1655
<220> b2290 b2290
<222> 1071-1170
<223> b2290 b2290 U00096 2405581..2406798
<313> 4275: 1119-1138
<400> 4275
GCAGGAAAAA GTTCTGTTGG TGCAAGGGAC GGCATTCAAC TGGCCGTGGC 50
CGGATCACTT CCGCATTGTC ACGCTACCGC GTGTCGATGA TATCGAGCTG 100
<210> 4276
<211> 100
<212> DNA
<213> Escherichia coli K-12 MG1655
<220> b2290 b2290
<222> 1028-1127
<223> b2290 b2290 U00096 2405581..2406798
<313> 4276: 1094-1113
<400> 4276
TTAACATTCA CGACGATCAG AAAATGGTGT TGGATTTCCT GTTGCAGGAA 50
AAAGTTCTGT TGGTGCAAGG GACGGCATTC AACTGGCCGT GGCCGGATCA 100
<210> 4277
<211> 100
<212> DNA
<213> Escherichia coli K-12 MG1655
<220> b2290 b2290
<222> 991-1090
<223> b2290 b2290 U00096 2405581..2406798
<313> 4277: 1018-1037
<400> 4277
GCGCTGTATA TGTTCCCGAA AATCGACGCC AAACGCTTTA ACATTCACGA 50
CGATCAGAAA ATGGTGTTGG ATTTCCTGTT GCAGGAAAAA GTTCTGTTGG 100
<210> 4278
<211> 100
<212> DNA
<213> Escherichia coli K-12 MG1655
<220> ybhm b0788
<222> 615-714
<223> ybhm b0788 U00096 820016..820729
<313> 4278: 650-669
<400> 4278
TGCATTAACA CTCTATTACT ATTTTATCGG CTTCTTTGGG ATACTTGCCG 50
CGATAGCTAT AACGCTTGTC TGGCAAAGGC ATACGCGTTT TTTTCATTAG 100
<210> 4279
<211> 100
<212> DNA
<213> Escherichia coli K-12 MG1655
<220> ybhm b0788
<222> 556-655
<223> ybhm b0788 U00096 820016..820729
<313> 4279: 587-606
<400> 4279
TTGGCGGGGA AATGCCATAG TGAAGAGTTG TGGAGTCCGG TTCGTTGCGC 50
TTTTACAGGT GCATTAACAC TCTATTACTA TTTTATCGGC TTCTTTGGGA 100

<210> 4280
<211> 100
<212> DNA
<213> Escherichia coli K-12 MG1655
<220> ybhm b0788
<222> 500-599
<223> ybhm b0788 U00096 820016..820729
<313> 4280: 539-558
<400> 4280
TAATGATTGT GTTATGGTCA GGCATTATCT CGCATGGACG AAATAAGCTC 50
CTTGAATTGG CGGGGAAATG CCATAGTGAA GAGTTGTGGA GTCCGGTTCG 100
<210> 4281
<211> 100
<212> DNA
<213> Escherichia coli K-12 MG1655
<220> b0833 b0833
<222> 2250-2349
<223> b0833 b0833 U00096 872202..874550
<313> 4281: 2317-2336
<400> 4281
ATTAATCAAA TTAGGATGTG TTCAGGCTCA GGGGTATCTG TACCAGAAAC 50
CCATGCCATT CTCCGCCTGG GATAAAAGTG GAAAATTAGT AAAAGAGTAG 100
<210> 4282
<211> 100
<212> DNA
<213> Escherichia coli K-12 MG1655
<220> b0833 b0833
<222> 2071-2170
<223> b0833 b0833 U00096 872202..874550
<313> 4282: 2118-2137
<400> 4282
TTGTCAAACC TGAAACGTTT TTATGAAATT AATCCAGACA GCATAAAGGT 50
GGACTCGCAA TTCACCGGCG ATATTTTCGG TACTGCGGGA AAAATTGTGC 100
<210> 4283
<211> 100
<212> DNA
<213> Escherichia coli K-12 MG1655
<220> b0833 b0833
<222> 1912-2011
<223> b0833 b0833 U00096 872202..874550
<313> 4283: 1948-1967
<400> 4283
TTTACCACCA TCATGAAAGA AAATGGCCTG AAGGCGAACA GCCTCTGTGT 50
TGAGATCACT GAAACCGTGA TCGAGCGAAT TAATGAACAT TTTTATCTCA 100
<210> 4284
<211> 100
<212> DNA
<213> Escherichia coli K-12 MG1655
<220> b2291 b2292
<222> 501-600
<223> b2291 b2292 U00096 2406882..2407481
<313> 4284: 568-587
<400> 4284
TGAAGCGCGT CGCAGCCAGG AGATGGACTA CTTCATGGAA ATATTTGTTC 50
CCAGCTTCCA TCTTTCGCTC GATGAGATTA GCCAGGATTC ACCGCTGTAA 100
<210> 4285
<211> 100
<212> DNA
<213> Escherichia coli K-12 MG1655
<220> b2291 b2292
<222> 240-339
<223> b2291 b2292 U00096 2406882..2407481
<313> 4285: 300-319
<400> 4285
TCCGGTGAAA TACTTCAATT CGCAAATCGC TCAGGAGTAC AAGGCTATTG 50
AAAAAATCGC TCAGCAAAAA CTGGTCGATA TGGTTCCGGA AGAGCTGCGG 100
<210> 4286
<211> 100
<212> DNA
<213> Escherichia coli K-12 MG1655
<220> b2291 b2292

<222> 211-310
<223> b2291 b2292 U00096 2406882..2407481
<313> 4286: 261-280
<400> 4286
TCAGAAGTGC TCACCGGCGA TCTCCCTACT CCGGTGAAAT ACTTCAATTC  50
GCAAATCGCT CAGGAGTACA AGGCTATTGA AAAAATCGCT CAGCAAAAAC  100
<210> 4287
<211> 100
<212> DNA
<213> Escherichia coli K-12 MG1655
<220> ybhn b0789
<222> 858-957
<223> ybhn b0789 U00096 complement(820765..821721)
<313> 4287: 920-939
<400> 4287
GCTGTATTAC TTTATCCCAC TGCTGCTGGC GCTGATCTGT TACCTGTTGC  50
TGGAAAGCCA GGCGAAGAAG CTACGGGCGA AAAATGAAGC GGCGATGTGA  100
<210> 4288
<211> 100
<212> DNA
<213> Escherichia coli K-12 MG1655
<220> ybhn b0789
<222> 821-920
<223> ybhn b0789 U00096 complement(820765..821721)
<313> 4288: 867-886
<400> 4288
AAGGTACAAT TATCGCCGCC CTACTCGCTT ACCGTGTGCT GTATTACTTT  50
ATCCCACTGC TGCTGGCGCT GATCTGTTAC CTGTTGCTGG AAAGCCAGGC  100
<210> 4289
<211> 100
<212> DNA
<213> Escherichia coli K-12 MG1655
<220> ybhn b0789
<222> 781-880
<223> ybhn b0789 U00096 complement(820765..821721)
<313> 4289: 814-833
<400> 4289
GCGGTGTTTA TCGCACTACT GGCTGGGGAG CATACCTCCA AAGGTACAAT  50
TATCGCCGCC CTACTCGCTT ACCGTGTGCT GTATTACTTT ATCCCACTGC  100
<210> 4290
<211> 100
<212> DNA
<213> Escherichia coli K-12 MG1655
<220> b0834 b0835
<222> 1230-1329
<223> b0834 b0835 U00096 874558..875886
<313> 4290: 1300-1319
<400> 4290
TTCAAATGAA TGTCCTACCG TCGAACGCTT GAAAATGCTG GCGGATGAGC  50
GGCTGTATAA GAGTAAGAAA AACGGCAGGG CACAGATTAG CTGGCAGTAA  100
<210> 4291
<211> 100
<212> DNA
<213> Escherichia coli K-12 MG1655
<220> b0834 b0835
<222> 1183-1282
<223> b0834 b0835 U00096 874558..875886
<313> 4291: 1250-1269
<400> 4291
TTCCAGGTGG GAAGCATTAA TTTAAGTACC AGTATCGGTG TAGCACATTC  50
AAATGAATGT CCTACCGTCG AACGCTTGAA AATGCTGGCG GATGAGCGGC  100
<210> 4292
<211> 100
<212> DNA
<213> Escherichia coli K-12 MG1655
<220> b0834 b0835
<222> 1091-1190
<223> b0834 b0835 U00096 874558..875886
<313> 4292: 1151-1170
<400> 4292
AAGTCTACCG CTTCGGCGGT GACGAATTTG CGGTGATTTA TACGGGTGGA  50

ACGCTGGAAG AGTTGCTATC GATTCTAAAA GAAATCGTTC ATTTCCAGGT 100
<210> 4293
<211> 100
<212> DNA
<213> Escherichia coli K-12 MG1655
<220> ybho b0790
<222> 1143-1242
<223> ybho b0790 U00096 complement(821721..822962)
<313> 4293: 1212-1231
<400> 4293
GGCACACACG CCACGTCTGG CGCAGGTTGA TCCGCCCGCA CAACCGACAA 50
TGGAAACGCA GGATCGGGTA GAAACTGAAA ACACGGGGGT AAAACCCTGA 100
<210> 4294
<211> 100
<212> DNA
<213> Escherichia coli K-12 MG1655
<220> ybho b0790
<222> 1117-1216
<223> ybho b0790 U00096 complement(821721..822962)
<313> 4294: 1172-1191
<400> 4294
TTCCCGGCGC TGGTTGGCTG GCTTCCGGCA CACACGCCAC GTCTGGCGCA 50
GGTTGATCCG CCCGCACAAC CGACAATGGA AACGCAGGAT CGGGTAGAAA 100
<210> 4295
<211> 100
<212> DNA
<213> Escherichia coli K-12 MG1655
<220> ybho b0790
<222> 1008-1107
<223> ybho b0790 U00096 complement(821721..822962)
<313> 4295: 1065-1084
<400> 4295
GAACGGCATT ATTGCCGCAG ATTGTCAGCA GGTGGATGAA ACCATGCTGC 50
CCAAACGCAC CTGGTGGAAC CTGACCAAAA GCGTGCTGGC GTTCCACTTT 100
<210> 4296
<211> 100
<212> DNA
<213> Escherichia coli K-12 MG1655
<220> ybhp b0791
<222> 663-762
<223> ybhp b0791 U00096 complement(822959..823720)
<313> 4296: 722-741
<400> 4296
CGTCAAAAAT GCCAGCGCCA GCGCGCCAAC CGCGTTGCCG CTGCGGACAT 50
GGCGACACCT TTCTGATCAT GCCCCTTTAA GTGCGGAGAT TCATTTATGA 100
<210> 4297
<211> 100
<212> DNA
<213> Escherichia coli K-12 MG1655
<220> ybhp b0791
<222> 628-727
<223> ybhp b0791 U00096 complement(822959..823720)
<313> 4297: 650-669
<400> 4297
GTGCAATTTC CTCTACTACG ACTGGACAGG ATCTACGTCA AAAATGCCAG 50
CGCCAGCGCG CCAACCGCGT TGCCGCTGCG GACATGGCGA CACCTTTCTG 100
<210> 4298
<211> 100
<212> DNA
<213> Escherichia coli K-12 MG1655
<220> ybhp b0791
<222> 531-630
<223> ybhp b0791 U00096 complement(822959..823720)
<313> 4298: 578-597
<400> 4298
TGACTGGCGG CAAAAAGCTA ATCATCCGTT AAAAGTGCAG GCCGGACTGG 50
ATGAGATTTT TACCCGCGCC CACGGACGCC CGGCGCGCAC GTTTCCGGTG 100
<210> 4299
<211> 100
<212> DNA
<213> Escherichia coli K-12 MG1655

<220> b0836 b0837
<222> 253-352
<223> b0836 b0837 U00096 877471..877854
<313> 4299: 279-298
<400> 4299
TGTTTGATGA GTGGGCACCA TGATTGCCCG ACTGTTATTA CCGTCAACGC 50
CGATAAGCTT GAAAATTGTC TGATGAGCTT AACGCTGAGT ATCCAGAGCC 100
<210> 4300
<211> 100
<212> DNA
<213> Escherichia coli K-12 MG1655
<220> b0836 b0837
<222> 208-307
<223> b0836 b0837 U00096 877471..877854
<313> 4300: 236-255
<400> 4300
AGTGCTCTTC ATCATGCTGA AGAAGCATTA GCGACAGCCG CAGTATGTTT 50
GATGAGTGGG CACCATGATT GCCCGACTGT TATTACCGTC AACGCCGATA 100
<210> 4301
<211> 100
<212> DNA
<213> Escherichia coli K-12 MG1655
<220> b0836 b0837
<222> 73-172
<223> b0836 b0837 U00096 877471..877854
<313> 4301: 103-122
<400> 4301
GTGCAACTGA GGAAGGCAAA AGGATACATG TCCGTCAGCG AAAGCAATCA 50
TCTACGAGAT AACTTTTTTA AACTGAATCG CGAACTGCAC GATAAATCGC 100
<210> 4302
<211> 100
<212> DNA
<213> Escherichia coli K-12 MG1655
<220> b1565 b1566
<222> 133-232
<223> b1565 b1566 U00096 1643921..1644226
<313> 4302: 191-210
<400> 4302
GCAGGTCAGC TCGTTTTTAA AGAGTCCGGC CATCATCTTA CTGGTACAGA 50
CACCATATAC TTTGTGACGG TCAGGCTACA TATGCACAAC TCAACTTATT 100
<210> 4303
<211> 100
<212> DNA
<213> Escherichia coli K-12 MG1655
<220> b1565 b1566
<222> 56-155
<223> b1565 b1566 U00096 1643921..1644226
<313> 4303: 112-131
<400> 4303
AGAAAGTAGT CGTAAGAACG GTGATCACTG TCCGCTTTGT GCCAGGAGCA 50
GCCATTGCTA AGTCCATCCT GTATTGTGCA GGTCAGCTCG TTTTTAAAGA 100
<210> 4304
<211> 100
<212> DNA
<213> Escherichia coli K-12 MG1655
<220> b1565 b1566
<222> 17-116
<223> b1565 b1566 U00096 1643921..1644226
<313> 4304: 50-69
<400> 4304
CGCACTACAA CATGCTTAGG GCAAGTCACG AAGGAAGTCA GAAAGTAGTC 50
GTAAGAACGG TGATCACTGT CCGCTTTGTG CCAGGAGCAG CCATTGCTAA 100
<210> 4305
<211> 100
<212> DNA
<213> Escherichia coli K-12 MG1655
<220> b2294 b2294
<222> 414-513
<223> b2294 b2294 U00096 complement(2410120..2410632)
<313> 4305: 461-480
<400> 4305

AATGTGGGAA AAATATCAGC GCATGCTTAA TGTGTGGCAT GCCTGCCCGC 50
GTCAGTACCA TTTGAGCGCC AACGAAATTA ATCAAATTAT CAATGCCTGA 100
<210> 4306
<211> 100
<212> DNA
<213> Escherichia coli K-12 MG1655
<220> b2294 b2294
<222> 356-455
<223> b2294 b2294 U00096 complement(2410120..2410632)
<313> 4306: 412-431
<400> 4306
TGGAAGGGCG CTATACCCAT TTTGACGCCG GAACTCACGG TTTTAACGCC 50
CAGACGCCAA TGTGGGAAAA ATATCAGCGC ATGCTTAATG TGTGGCATGC 100
<210> 4307
<211> 100
<212> DNA
<213> Escherichia coli K-12 MG1655
<220> b2294 b2294
<222> 329-428
<223> b2294 b2294 U00096 complement(2410120..2410632)
<313> 4307: 364-383
<400> 4307
TCGGTTATGT CCGCTTTATG GTTAATGTGG AAGGGCGCTA TACCCATTTT 50
GACGCCGGAA CTCACGGTTT TAACGCCCAG ACGCCAATGT GGGAAAAATA 100
<210> 4308
<211> 100
<212> DNA
<213> Escherichia coli K-12 MG1655
<220> ybhq b0792
<222> 312-411
<223> ybhq b0792 U00096 823853..824263
<313> 4308: 371-390
<400> 4308
TGCAGGGCTG GCAATCCTTG CCGGCCCGGC GGTAGTGTCT TTGCTGGCGA 50
AAGATAAGAA GTTACATCAC CTTACGTCTA AACATCGCGT ACGCCGCTGA 100
<210> 4309
<211> 100
<212> DNA
<213> Escherichia coli K-12 MG1655
<220> ybhq b0792
<222> 160-259
<223> ybhq b0792 U00096 823853..824263
<313> 4309: 216-235
<400> 4309
ATGATGCTGG TGTTTCAGCG CCACCCCGAG CAACGCTGGC GTGAGGTGGC 50
AGACGTGCTG GAAGAGCTGA CCACGACCTG GTATTTTGGC GCAGCGCTGA 100
<210> 4310
<211> 100
<212> DNA
<213> Escherichia coli K-12 MG1655
<220> ybhq b0792
<222> 183-282
<223> ybhq b0792 U00096 823853..824263
<313> 4310: 204-223
<400> 4310
CCCCGAGCAA CGCTGGCGTG AGGTGGCAGA CGTGCTGGAA GAGCTGACCA 50
CGACCTGGTA TTTTGGCGCA GCGCTGATTG TGCTGTGGCT GTTGTCCCGC 100
<210> 4311
<211> 100
<212> DNA
<213> Escherichia coli K-12 MG1655
<220> b2295 b2296
<222> 338-437
<223> b2295 b2296 U00096 complement(2410697..2411152)
<313> 4311: 368-387
<400> 4311
TGCAGGAGTC TGGACAGGTG TTGGCACCCG TTGAAGGCAA GCCTGATTAC 50
CAGGCATTAG CTGACACGCT TAAGCGCGCC TTCAAACAAC TGGATAAAAC 100
<210> 4312
<211> 100
<212> DNA

<213> Escherichia coli K-12 MG1655
<220> b2295 b2296
<222> 246-345
<223> b2295 b2296 U00096 complement(2410697..2411152)
<313> 4312: 280-299
<400> 4312
ACCCATGCAG GGATTGTGGT GGCTGGGCAA GCGTTCTGTC ACGCCATTAC  50
CCCCTGCAAT CCTTAACTGG TTTTATGAAG TTCGCGGTAA ATTGCAGGAG  100
<210> 4313
<211> 100
<212> DNA
<213> Escherichia coli K-12 MG1655
<220> b2295 b2296
<222> 212-311
<223> b2295 b2296 U00096 complement(2410697..2411152)
<313> 4313: 240-259
<400> 4313
CGGCAGTTGC CACTGCCCTG TTCGCCTTAA GTTTACCCAT GCAGGGATTG  50
TGGTGGCTGG GCAAGCGTTC TGTCACGCCA TTACCCCCTG CAATCCTTAA  100
<210> 4314
<211> 100
<212> DNA
<213> Escherichia coli K-12 MG1655
<220> yqja b3096
<222> 564-663
<223> yqja b3096 U00096 3245413..3246075
<313> 4314: 619-638
<400> 4314
GATGTCATGC CTGATGCTGC TCCCGGTGGT GCTGCTGGTG TTTGGCCTGG  50
CAGGTTCTCT GGTCGTGTTA TGGAAAAAGA AATATGGAAA TCGGGGGTAA  100
<210> 4315
<211> 100
<212> DNA
<213> Escherichia coli K-12 MG1655
<220> yqja b3096
<222> 475-574
<223> yqja b3096 U00096 3245413..3246075
<313> 4315: 542-561
<400> 4315
ATGAGCGGTC TGCTGTGGGT ATTGATCCTG ACAACTCTGG GTTACATGCT  50
CGGCAAAACG CCGGTATTTT TAAAGTACGA GGACCAGCTG ATGTCATGCC  100
<210> 4316
<211> 100
<212> DNA
<213> Escherichia coli K-12 MG1655
<220> yqja b3096
<222> 467-566
<223> yqja b3096 U00096 3245413..3246075
<313> 4316: 493-512
<400> 4316
TCAACTGGAT GAGCGGTCTG CTGTGGGTAT TGATCCTGAC AACTCTGGGT  50
TACATGCTCG GCAAAACGCC GGTATTTTTA AAGTACGAGG ACCAGCTGAT  100
<210> 4317
<211> 100
<212> DNA
<213> Escherichia coli K-12 MG1655
<220> ybhr b0793
<222> 1008-1107
<223> ybhr b0793 U00096 complement(824225..825331)
<313> 4317: 1052-1071
<400> 4317
GAGTCTGGAT ATTGTGTGGA ATAGTTTGTG GCCGCTACTG GTGATAACGG  50
CCACGACAGG GTCAGCGGCG TACGCGATGT TTAGACGTAA GGTGATGTAA  100
<210> 4318
<211> 100
<212> DNA
<213> Escherichia coli K-12 MG1655
<220> ybhr b0793
<222> 968-1067
<223> ybhr b0793 U00096 complement(824225..825331)
<313> 4318: 1017-1036

<400> 4318
ACTTTACGGA CATTACCAAG CAGATTTATT TGAAGGATGC GAGTCTGGAT 50
ATTGTGTGGA ATAGTTTGTG GCCGCTACTG GTGATAACGG CCACGACAGG 100
<210> 4319
<211> 100
<212> DNA
<213> Escherichia coli K-12 MG1655
<220> ybhr b0793
<222> 909-1008
<223> ybhr b0793 U00096 complement(824225..825331)
<313> 4319: 968-987
<400> 4319
TCCGGTGGAA AACATGCCGG TATGGCTGCA AAACCTGACG TGGATTAACC 50
CTATTCGCCA CTTTACGGAC ATTACCAAGC AGATTTATTT GAAGGATGCG 100
<210> 4320
<211> 100
<212> DNA
<213> Escherichia coli K-12 MG1655
<220> asns b0931
<222> 1302-1401
<223> asns b0931 U00096 complement(986808..988208)
<313> 4320: 1369-1388
<400> 4320
CGGTCTTGGT TTTGAACGTC TGATTGCTTA CGTAACTGGC GTGCAAAACG 50
TACGTGATGT GATTCCGTTC CCACGTACTC CGCGTAACGC CAGCTTCTAA 100
<210> 4321
<211> 100
<212> DNA
<213> Escherichia coli K-12 MG1655
<220> asns b0931
<222> 1270-1369
<223> asns b0931 U00096 complement(986808..988208)
<313> 4321: 1302-1321
<400> 4321
CGTCGCTACG GTACTGTTCC GCATTCAGGT TTCGGTCTTG GTTTTGAACG 50
TCTGATTGCT TACGTAACTG GCGTGCAAAA CGTACGTGAT GTGATTCCGT 100
<210> 4322
<211> 100
<212> DNA
<213> Escherichia coli K-12 MG1655
<220> asns b0931
<222> 1060-1159
<223> asns b0931 U00096 complement(986808..988208)
<313> 4322: 1112-1131
<400> 4322
GTAGTGGTTA AAAACTATCC GAAAGATATT AAAGCGTTCT ATATGCGCCT 50
TAACGAAGAC GGTAAAACCG TTGCGGCTAT GGACGTTCTG GCTCCGGGCA 100
<210> 4323
<211> 100
<212> DNA
<213> Escherichia coli K-12 MG1655
<220> b1567 b1567
<222> 51-150
<223> b1567 b1567 U00096 complement(1645198..1645347)
<313> 4323: 115-134
<400> 4323
AGCTGACCAT CCCTGGCTTA CGCGCCGAAC GCAAAGTCAT CAGCAAGTTA 50
AGCCCCCGAA GTTACCTAAA AAGAAGCCTG ATCCCGATAA AAAAGATTGA 100
<210> 4324
<211> 100
<212> DNA
<213> Escherichia coli K-12 MG1655
<220> b1567 b1567
<222> 51-150
<223> b1567 b1567 U00096 complement(1645198..1645347)
<313> 4324: 106-125
<400> 4324
AGCTGACCAT CCCTGGCTTA CGCGCCGAAC GCAAAGTCAT CAGCAAGTTA 50
AGCCCCCGAA GTTACCTAAA AAGAAGCCTG ATCCCGATAA AAAAGATTGA 100
<210> 4325
<211> 100

<212> DNA
<213> Escherichia coli K-12 MG1655
<220> b1567 b1567
<222> 51-150
<223> b1567 b1567 U00096 complement(1645198..1645347)
<313> 4325: 96-115
<400> 4325
AGCTGACCAT CCCTGGCTTA CGCGCCGAAC GCAAAGTCAT CAGCAAGTTA 50
AGCCCCCGAA GTTACCTAAA AAGAAGCCTG ATCCCGATAA AAAAGATTGA 100
<210> 4326
<211> 100
<212> DNA
<213> Escherichia coli K-12 MG1655
<220> yqjb b3097
<222> 285-384
<223> yqjb b3097 U00096 3246079..3246462
<313> 4326: 363-382
<400> 4326
AACATTGCCC CATGGCTACA TCATTGCGCA GCAGGACAAT AACAGTCAGG 50
CTATGCAGTG GCTGACCCGG TTACGGGATA ACTCTCATCG CTTCGGATAA 100
<210> 4327
<211> 100
<212> DNA
<213> Escherichia coli K-12 MG1655
<220> yqjb b3097
<222> 238-337
<223> yqjb b3097 U00096 3246079..3246462
<313> 4327: 274-293
<400> 4327
TTTGACTCCA GCGACCAGAG CGCCGCCGCA AAAGCGGTCC TCGACAGAAC 50
ATTGCCCCAT GGCTACATCA TTGCGCAGCA GGACAATAAC AGTCAGGCTA 100
<210> 4328
<211> 100
<212> DNA
<213> Escherichia coli K-12 MG1655
<220> yqjb b3097
<222> 147-246
<223> yqjb b3097 U00096 3246079..3246462
<313> 4328: 189-208
<400> 4328
GCCAGACGGT TTTTCAATCT GGCATCACCT TGACGCTCAT GGCATTCCTT 50
TCAAAAGTAT CACCCCCAAA AACGACACTC TGTTAATTAC ATTTGACTCC 100
<210> 4329
<211> 100
<212> DNA
<213> Escherichia coli K-12 MG1655
<220> ybhs b0794
<222> 1035-1134
<223> ybhs b0794 U00096 complement(825342..826475)
<313> 4329: 1073-1092
<400> 4329
TATTCCAGTG GTGCTGGTGG TAAACGTGCT GTTTTTGATC GCTTCGGCGG 50
TGATGTTTAT CGGCCTGACG TGGCTGAAAA CCAAACGTCG GCTGGATTAG 100
<210> 4330
<211> 100
<212> DNA
<213> Escherichia coli K-12 MG1655
<220> ybhs b0794
<222> 983-1082
<223> ybhs b0794 U00096 complement(825342..826475)
<313> 4330: 1000-1019
<400> 4330
TTCCCGCTCG TTATTTCGTC AGCACCCTGC AAAGCCTGTT CCTCGCCGGG 50
AATATTCCAG TGGTGCTGGT GGTAAACGTG CTGTTTTTGA TCGCTTCGGC 100
<210> 4331
<211> 100
<212> DNA
<213> Escherichia coli K-12 MG1655
<220> ybhs b0794
<222> 879-978
<223> ybhs b0794 U00096 complement(825342..826475)

<313> 4331: 922-941
<400> 4331
TCAGGTCGCC CTGAACGCCG CTTTTCTGCC GTCGATTATG CTTTCCGGCT 50
TTATTTTTCA GATCGACAGT ATGCCCGCGG TGATCCGCGC GGTGACGTAC 100
<210> 4332
<211> 100
<212> DNA
<213> Escherichia coli K-12 MG1655
<220> b1568 b1569
<222> 188-287
<223> b1568 b1569 U00096 complement(1645370..1645660)
<313> 4332: 217-236
<400> 4332
ATCGAGTGAA GATATTTGAA CGCTGGAAGG CTATCGACAC CAGGGATAAG 50
CGTGAAAAGT TCACGGCGCT AGTGCCTGCA ATTATGGAGG CTACCACTGG 100
<210> 4333
<211> 100
<212> DNA
<213> Escherichia coli K-12 MG1655
<220> b1568 b1569
<222> 71-170
<223> b1568 b1569 U00096 complement(1645370..1645660)
<313> 4333: 101-120
<400> 4333
AGCAGGCGAC GATATTAATC ACTGAAGCAT TCTGGGCTTT GAAAGAGCGC 50
CCGAACATCG ATGTTCAGCG TGTCACATAT GAAGGTGGCG CGATTGATCA 100
<210> 4334
<211> 100
<212> DNA
<213> Escherichia coli K-12 MG1655
<220> b1568 b1569
<222> 23-122
<223> b1568 b1569 U00096 complement(1645370..1645660)
<313> 4334: 64-83
<400> 4334
CTCGTGAGGC TCTGGATGCC TGGATATGTC GACCAGGAAT GACACAGGAG 50
CAGGCGACGA TATTAATCAC TGAAGCATTC TGGGCTTTGA AAGAGCGCCC 100
<210> 4335
<211> 100
<212> DNA
<213> Escherichia coli K-12 MG1655
<220> yqjc b3098
<222> 285-384
<223> yqjc b3098 U00096 3246594..3246977
<313> 4335: 336-355
<400> 4335
GGCGAAGCAA AAAGGCGATG CCGATAAGAT TGCCAAACGC GAACGGAAAC 50
TGGCAGAAGC GCAGGAAGAG CTGAAAAAGC TGGAAGCGCG CGACTACTAA 100
<210> 4336
<211> 100
<212> DNA
<213> Escherichia coli K-12 MG1655
<220> yqjc b3098
<222> 254-353
<223> yqjc b3098 U00096 3246594..3246977
<313> 4336: 297-316
<400> 4336
AGGTGGCGGA ACGCCAGCAA GATTTAGCCG AGGCGAAGCA AAAAGGCGAT 50
GCCGATAAGA TTGCCAAACG CGAACGGAAA CTGGCAGAAG CGCAGGAAGA 100
<210> 4337
<211> 100
<212> DNA
<213> Escherichia coli K-12 MG1655
<220> yqjc b3098
<222> 225-324
<223> yqjc b3098 U00096 3246594..3246977
<313> 4337: 268-287
<400> 4337
TCAGAAGAAA ATCGCAAAGC AGAAAGATGA GGTGGCGGAA CGCCAGCAAG 50
ATTTAGCCGA GGCGAAGCAA AAAGGCGATG CCGATAAGAT TGCCAAACGC 100
<210> 4338

<211> 100
<212> DNA
<213> Escherichia coli K-12 MG1655
<220> yqjd b3099
<222> 207-306
<223> yqjd b3099 U00096 3247015..3247320
<313> 4338: 279-298
<400> 4338
GGCGCGTGCC GATGAGTATG TGCGCGAAAA TCCGTGGACG GGCGTGGGCA 50
TTGGCGCTGC AATCGGTGTA GTGCTCGGCG TTCTGCTGTC GCGTCGTTAA 100
<210> 4339
<211> 100
<212> DNA
<213> Escherichia coli K-12 MG1655
<220> yqjd b3099
<222> 105-204
<223> yqjd b3099 U00096 3247015..3247320
<313> 4339: 126-145
<400> 4339
GTTGAGTAAG ATTCGTAGCA AAGCGGAGCA GGCACTGAAA CAGAGCCGTT 50
ATCGCCTGGG TGAAACCGGT GATGCCATTG CCAAACAAAC CCGTGTCGCG 100
<210> 4340
<211> 100
<212> DNA
<213> Escherichia coli K-12 MG1655
<220> yqjd b3099
<222> 49-148
<223> yqjd b3099 U00096 3247015..3247320
<313> 4340: 81-100
<400> 4340
CTTTCCGATA CGCTGGAAGA GGTGCTTAGC TCATCTGGCG AGAAGTCGAA 50
AGAAGAGTTG AGTAAGATTC GTAGCAAAGC GGAGCAGGCA CTGAAACAGA 100
<210> 4341
<211> 100
<212> DNA
<213> Escherichia coli K-12 MG1655
<220> b2299 b2300
<222> 444-543
<223> b2299 b2300 U00096 complement(2416654..2417196)
<313> 4341: 521-540
<400> 4341
ACGCTGCGAT GAGTTCACTC CAGACTCGCT GAAAGCGCTA GCGTTGTGGA 50
TGAAGCGCAA TGCCAAAAAT GAAGCCGTAG AGACTGAAAC GGCAGAATGA 100
<210> 4342
<211> 100
<212> DNA
<213> Escherichia coli K-12 MG1655
<220> b2299 b2300
<222> 383-482
<223> b2299 b2300 U00096 complement(2416654..2417196)
<313> 4342: 433-452
<400> 4342
TCGCACTACA GGAAGATGAA GTCAGTGAAG TTTGCTGGCT GACGCCGGAA 50
GAAATCACCG CACGCTGCGA TGAGTTCACT CCAGACTCGC TGAAAGCGCT 100
<210> 4343
<211> 100
<212> DNA
<213> Escherichia coli K-12 MG1655
<220> b2299 b2300
<222> 343-442
<223> b2299 b2300 U00096 complement(2416654..2417196)
<313> 4343: 391-410
<400> 4343
GTCTGGGGCG CATTGTTCAG CTGCGTCTCT CACGGTCCCT TCGCACTACA 50
GGAAGATGAA GTCAGTGAAG TTTGCTGGCT GACGCCGGAA GAAATCACCG 100
<210> 4344
<211> 100
<212> DNA
<213> Escherichia coli K-12 MG1655
<220> yqje yqjE
<222> 300-399

<223> yqje yqjE U00096 3247323..3247727
<313> 4344: 343-362
<400> 4344
TATCTGGACG CTACGTAAAT CGCGTAAGTC TACGTTGCTG CGCCATACAC 50
GCCATGAGTT AGCAAACGAT CGGCAGCTGC TCGAGGAGGA GTCCCGTGAG 100
<210> 4345
<211> 100
<212> DNA
<213> Escherichia coli K-12 MG1655
<220> yqje yqjE
<222> 247-346
<223> yqje yqjE U00096 3247323..3247727
<313> 4345: 298-317
<400> 4345
AATGCGATGA TTGCCACCAC CGTGGTGTTG CTGCTACTGG CACTGATTGG 50
CGGTATCTGG ACGCTACGTA AATCGCGTAA GTCTACGTTG CTGCGCCATA 100
<210> 4346
<211> 100
<212> DNA
<213> Escherichia coli K-12 MG1655
<220> yqje yqjE
<222> 211-310
<223> yqje yqjE U00096 3247323..3247727
<313> 4346: 235-254
<400> 4346
CTAATTATTT GGGCGGTTGA CCCGCAATAT CGCCTGAATG CGATGATTGC 50
CACCACCGTG GTGTTGCTGC TACTGGCACT GATTGGCGGT ATCTGGACGC 100
<210> 4347
<211> 100
<212> DNA
<213> Escherichia coli K-12 MG1655
<220> yqjf b3102
<222> 384-483
<223> yqjf b3102 U00096 3248112..3248594
<313> 4347: 459-478
<400> 4347
GTTCATGAAA AACCTGACAA TTTCTGGCGG ATTCCTGCTG CTGGCAATTA 50
CCGGTCCGGG CGCGTATAGC ATCGACCGCC TGCTGAATAA AAAGTGGTAA 100
<210> 4348
<211> 100
<212> DNA
<213> Escherichia coli K-12 MG1655
<220> yqjf b3102
<222> 289-388
<223> yqjf b3102 U00096 3248112..3248594
<313> 4348: 336-355
<400> 4348
CTGACTCGCA CCACAGCCCT GTTTACTGCG GGCTTTACGC TGCTGACGGC 50
ATTTTTATTT CACAGCAACT TTGCTGAAGG CGTCAACTCG CTGATGTTCA 100
<210> 4349
<211> 100
<212> DNA
<213> Escherichia coli K-12 MG1655
<220> yqjf b3102
<222> 249-348
<223> yqjf b3102 U00096 3248112..3248594
<313> 4349: 290-309
<400> 4349
GCTTGAGTTT GGTGGTGGTC TGGCAATCCT GTTCGGTTTC CTGACTCGCA 50
CCACAGCCCT GTTTACTGCG GGCTTTACGC TGCTGACGGC ATTTTTATTT 100
<210> 4350
<211> 100
<212> DNA
<213> Escherichia coli K-12 MG1655
<220> yqjg b3103
<222> 888-987
<223> yqjg b3103 U00096 3248664..3249650
<313> 4350: 952-971
<400> 4350
CTTCCGCAGC CATAAGACCA TCAACCCTAC GGGGATTATT TCAATTGGTC 50
CGTGGCAGGA TCTCGATGAA CCGCATGGAC GAGATGTTCG CTTCGGTTAA 100

<210> 4351
<211> 100
<212> DNA
<213> Escherichia coli K-12 MG1655
<220> yqjg b3103
<222> 861-960
<223> yqjg b3103 U00096 3248664..3249650
<313> 4351: 907-926
<400> 4351
CAATTTCGAT CATATCCGTA ATCATTACTT CCGCAGCCAT AAGACCATCA 50
ACCCTACGGG GATTATTTCA ATTGGTCCGT GGCAGGATCT CGATGAACCG 100
<210> 4352
<211> 100
<212> DNA
<213> Escherichia coli K-12 MG1655
<220> yqjg b3103
<222> 799-898
<223> yqjg b3103 U00096 3248664..3249650
<313> 4352: 855-874
<400> 4352
TACCTGAATC TGTACGGTTT TCTGCGCGAT ATCTACCAGA TGCCGGGGAT 50
CGCCGAAACA GTCAATTTCG ATCATATCCG TAATCATTAC TTCCGCAGCC 100
<210> 4353
<211> 100
<212> DNA
<213> Escherichia coli K-12 MG1655
<220> yqjh b3071
<222> 619-718
<223> yqjh b3071 U00096 complement(3213368..3214132)
<313> 4353: 683-702
<400> 4353
CTGGCGCAGA TGCAAATCCC TGCCGATGAT TACTTCATCT GGATAACCGG 50
CGAAGGTAAA GTCGTTAAGA ATTTAAGCCG CCGCTTTGAA GCGGAACAGT 100
<210> 4354
<211> 100
<212> DNA
<213> Escherichia coli K-12 MG1655
<220> yqjh b3071
<222> 571-670
<223> yqjh b3071 U00096 complement(3213368..3214132)
<313> 4354: 626-645
<400> 4354
TTTAATATCG AATGGCTGGC ACATGATGAG CAGGCGGTAG ATGCGCGTCT 50
GGCGCAGATG CAAATCCCTG CCGATGATTA CTTCATCTGG ATAACCGGCG 100
<210> 4355
<211> 100
<212> DNA
<213> Escherichia coli K-12 MG1655
<220> yqjh b3071
<222> 556-655
<223> yqjh b3071 U00096 complement(3213368..3214132)
<313> 4355: 591-610
<400> 4355
GCGCACCTTG ATGGTTTTAA TATCGAATGG CTGGCACATG ATGAGCAGGC 50
GGTAGATGCG CGTCTGGCGC AGATGCAAAT CCCTGCCGAT GATTACTTCA 100
<210> 4356
<211> 100
<212> DNA
<213> Escherichia coli K-12 MG1655
<220> yqji b3072
<222> 525-624
<223> yqji b3072 U00096 3214420..3215043
<313> 4356: 592-611
<400> 4356
GGATTTACGC GTCAACCAGA GCGATATCAG TGATGCACAA ATAAAAAAGA 50
TCATTGCGGT GATCGACCGC GCCGCTTTTG ATATTACGCA ACTGGATTAA 100
<210> 4357
<211> 100
<212> DNA
<213> Escherichia coli K-12 MG1655
<220> yqji b3072

<222> 400-499
<223> yqji b3072 U00096 3214420..3215043
<313> 4357: 418-437
<400> 4357
TGGCTGGAAG AAAACCGCGA ACAGGTGGAG ATGATTGAAG AACGCATCAA 50
AGCGCGTTGC GTTGGCGCGG CGCTGCGCCA GAACCCGCAA ATGAAGCGGG 100
<210> 4358
<211> 100
<212> DNA
<213> Escherichia coli K-12 MG1655
<220> yqji b3072
<222> 336-435
<223> yqji b3072 U00096 3214420..3215043
<313> 4358: 363-382
<400> 4358
GATTACCATC CGCGAAGAGG AAGGAGGTAA GAAGCAGATT GCGCTGACCG 50
AACAAGGCGC GCAGTGGCTG GAAGAAAACC GCGAACAGGT GGAGATGATT 100
<210> 4359
<211> 100
<212> DNA
<213> Escherichia coli K-12 MG1655
<220> ybia b0799
<222> 378-477
<223> ybia b0799 U00096 complement(831691..832173)
<313> 4359: 438-457
<400> 4359
TACGGAAAAC GATGCTTACT GGGGAGACGG TGGTCATGGT AAGGGCAAGA 50
ATCGACTGGG CTACCTTTTA ATGGAGTTGC GCGAACAATT GGCTATAGAG 100
<210> 4360
<211> 100
<212> DNA
<213> Escherichia coli K-12 MG1655
<220> ybia b0799
<222> 360-459
<223> ybia b0799 U00096 complement(831691..832173)
<313> 4360: 380-399
<400> 4360
CGCAAAACTG GTTGAGCATA CGGAAAACGA TGCTTACTGG GGAGACGGTG 50
GTCATGGTAA GGGCAAGAAT CGACTGGGCT ACCTTTTAAT GGAGTTGCGC 100
<210> 4361
<211> 100
<212> DNA
<213> Escherichia coli K-12 MG1655
<220> ybia b0799
<222> 244-343
<223> ybia b0799 U00096 complement(831691..832173)
<313> 4361: 291-310
<400> 4361
TCTAAGCCTC TGCGTAAAAA CTGGGAGTCG GTCAAAGAAC AGGTGATGCG 50
AAAAGCACTT CGCGCTAAAT TCGAACAGCA TGCAGAACTG CGTGCGCTCT 100
<210> 4362
<211> 100
<212> DNA
<213> Escherichia coli K-12 MG1655
<220> ybib b0801
<222> 864-963
<223> ybib b0801 U00096 834471..835433
<313> 4362: 912-931
<400> 4362
TCCCGAATCG CTGAAAATCC AGATGGCTTG CTGCCTGGTG GCTACGGGTG 50
AAGCGGCAAC TATCAGCGAC GGCCTGGCGC GCGTTAATCA GGCATTTTAA 100
<210> 4363
<211> 100
<212> DNA
<213> Escherichia coli K-12 MG1655
<220> ybib b0801
<222> 852-951
<223> ybib b0801 U00096 834471..835433
<313> 4363: 878-897
<400> 4363
CAGCGAACCG ATTCCCGAAT CGCTGAAAAT CCAGATGGCT TGCTGCCTGG 50

TGGCTACGGG TGAAGCGGCA ACTATCAGCG ACGGCCTGGC GCGCGTTAAT 100
<210> 4364
<211> 100
<212> DNA
<213> Escherichia coli K-12 MG1655
<220> ybib b0801
<222> 784-883
<223> ybib b0801 U00096 834471..835433
<313> 4364: 827-846
<400> 4364
AGCGAGTTAC TGCCACAAGC AAAAGATCCG GAAACCACGG CGCAGTGGAT 50
TGAGCGTTGC CTTGCTGGCA GCGAACCGAT TCCCGAATCG CTGAAAATCC 100
<210> 4365
<211> 100
<212> DNA
<213> Escherichia coli K-12 MG1655
<220> ybic b0802
<222> 987-1086
<223> ybic b0802 U00096 835574..836659
<313> 4365: 1059-1078
<400> 4365
ACTGGATGCG GGAAGCTGGC AGGCCATTTG TGATGCAGCG CGGCAGATTG 50
GTATGCCGGA AGAGACGTTG CAGGCTTTCT GTCAGCAGTT AGCCAGCTAA 100
<210> 4366
<211> 100
<212> DNA
<213> Escherichia coli K-12 MG1655
<220> ybic b0802
<222> 958-1057
<223> ybic b0802 U00096 835574..836659
<313> 4366: 1021-1040
<400> 4366
CGTCGCGAAC GGCAGAAGCA GGGGATTCCA CTGGATGCGG GAAGCTGGCA 50
GGCCATTTGT GATGCAGCGC GGCAGATTGG TATGCCGGAA GAGACGTTGC 100
<210> 4367
<211> 100
<212> DNA
<213> Escherichia coli K-12 MG1655
<220> ybic b0802
<222> 900-999
<223> ybic b0802 U00096 835574..836659
<313> 4367: 930-949
<400> 4367
CTCGCCGCAT GATGATGATA AGCCGATTTT GCTACCGGGC GAGTGGGAAG 50
TGAACACGCG TCGCGAACGG CAGAAGCAGG GGATTCCACT GGATGCGGGA 100
<210> 4368
<211> 100
<212> DNA
<213> Escherichia coli K-12 MG1655
<220> ybif b0814
<222> 789-888
<223> ybif b0814 U00096 complement(848433..849320)
<313> 4368: 838-857
<400> 4368
CATACAGCTA CTGGCGCTCG GCGCTATCAT CGCCGCTTCA ATGGGGTCTA 50
CGCTGACAGT ACGCAAAGAG AGCAAAATAA AAGAATTAGA CATTAATTAA 100
<210> 4369
<211> 100
<212> DNA
<213> Escherichia coli K-12 MG1655
<220> ybif b0814
<222> 738-837
<223> ybif b0814 U00096 complement(848433..849320)
<313> 4369: 774-793
<400> 4369
GGCGCTGGCT GCCGTTTCCG GGATGATTTT CCTCGGAGAA ACACTGACAC 50
CCATACAGCT ACTGGCGCTC GGCGCTATCA TCGCCGCTTC AATGGGGTCT 100
<210> 4370
<211> 100
<212> DNA
<213> Escherichia coli K-12 MG1655

<220> ybif b0814
<222> 664-763
<223> ybif b0814 U00096 complement(848433..849320)
<313> 4370: 707-726
<400> 4370
CCTTATTCGC TGGAAATGAT TGCCCTCACC CGTTTGCCAA CACGGACATT 50
TGGTACGCTG ATGAGCATGG AACCGGCGCT GGCTGCCGTT TCCGGGATGA 100
<210> 4371
<211> 100
<212> DNA
<213> Escherichia coli K-12 MG1655
<220> ybih b0797
<222> 584-683
<223> ybih b0797 U00096 complement(829195..829878)
<313> 4371: 631-650
<400> 4371
GCTGGACCGC GTTCGATGAA GAAAAGACCG AACTGATCAA CCAGACGGTG 50
ACCTGTCATA TCGACCTGAT TTTGCAAGGA TTATCGCAAA GGAGTTTGTA 100
<210> 4372
<211> 100
<212> DNA
<213> Escherichia coli K-12 MG1655
<220> ybih b0797
<222> 502-601
<223> ybih b0797 U00096 complement(829195..829878)
<313> 4372: 561-580
<400> 4372
CGCATGATCC TTCATACCCA TGCGTTGATT GGTGAGATTC TGGCGTTCCG 50
TCTTGGCAAA GAAACAATTC TGTTACGTAC CGGCTGGACC GCGTTCGATG 100
<210> 4373
<211> 100
<212> DNA
<213> Escherichia coli K-12 MG1655
<220> ybih b0797
<222> 467-566
<223> ybih b0797 U00096 complement(829195..829878)
<313> 4373: 487-506
<400> 4373
TTGCCGCCTG GACCGGCTGC GACGCCAATG ACACCCGCAT GATCCTTCAT 50
ACCCATGCGT TGATTGGTGA GATTCTGGCG TTCCGTCTTG GCAAAGAAAC 100
<210> 4374
<211> 100
<212> DNA
<213> Escherichia coli K-12 MG1655
<220> ybii b0804
<222> 168-267
<223> ybii b0804 U00096 complement(837413..837679)
<313> 4374: 228-247
<400> 4374
CGTGCGCTTA TGTATTCATT GTCAGCAGGA GAAAGATTTA CAAAAACCAG 50
CTTATACAGG ATATAATCGC AGAGGTTCGA AAGACAGCCA GTTACGTTAA 100
<210> 4375
<211> 100
<212> DNA
<213> Escherichia coli K-12 MG1655
<220> ybii b0804
<222> 113-212
<223> ybii b0804 U00096 complement(837413..837679)
<313> 4375: 125-144
<400> 4375
AATGTGAAGA GTGCGGTGCC CCCATCCCGC AGGCCCGTCG GGAAGCCATT 50
CCTGGCGTGC GCTTATGTAT TCATTGTCAG CAGGAGAAAG ATTTACAAAA 100
<210> 4376
<211> 100
<212> DNA
<213> Escherichia coli K-12 MG1655
<220> ybii b0804
<222> 1-100
<223> ybii b0804 U00096 complement(837413..837679)
<313> 4376: 42-61
<400> 4376

ATGGCATCCG GTTGGGCTAA CGATGACGCC GTCAACGAAC AGATCAACAG 50
TACAATTGAA GATGCGATTG CCCGCGCTCG GGGTGAAATT CCGCGCGGCG 100
<210> 4377
<211> 100
<212> DNA
<213> Escherichia coli K-12 MG1655
<220> ybij b0803
<222> 162-261
<223> ybij b0803 U00096 complement(836888..837148)
<313> 4377: 210-229
<400> 4377
ACTGGCTGAG AAAGCCGCGG CAGCCGGTGC CAGTGGATAC AGCATCACTT 50
CCGCCACCAA CAACAATAAA TTAAGCGGTA CTGCGGTAAT TTATAAGTAA 100
<210> 4378
<211> 100
<212> DNA
<213> Escherichia coli K-12 MG1655
<220> ybij b0803
<222> 111-210
<223> ybij b0803 U00096 complement(836888..837148)
<313> 4378: 156-175
<400> 4378
CGGCGTGGTT TCTGCCGATG GCGCATCCAC CCTCGATGCC CTGGAAGCGA 50
AACTGGCTGA GAAAGCCGCG GCAGCCGGTG CCAGTGGATA CAGCATCACT 100
<210> 4379
<211> 100
<212> DNA
<213> Escherichia coli K-12 MG1655
<220> ybij b0803
<222> 57-156
<223> ybij b0803 U00096 complement(836888..837148)
<313> 4379: 104-123
<400> 4379
CGTGTTCGCG GCGGAACCGG TAACGGCATC CCAGGCACAG AACATGAATA 50
AAATCGGCGT GGTTTCTGCC GATGGCGCAT CCACCCTCGA TGCCCTGGAA 100
<210> 4380
<211> 100
<212> DNA
<213> Escherichia coli K-12 MG1655
<220> ybik ybiK
<222> 867-966
<223> ybik ybiK U00096 865791..866756
<313> 4380: 928-947
<400> 4380
GTTTAACACC GAAGGAATGT ATCGCGCCTG GGGCTACGCA GGCGATACGC 50
CAACCACCGG TATCTACCGT GAAAAAGGGG ACACCGTTGC CACACAGTGA 100
<210> 4381
<211> 100
<212> DNA
<213> Escherichia coli K-12 MG1655
<220> ybik ybiK
<222> 848-947
<223> ybik ybiK U00096 865791..866756
<313> 4381: 881-900
<400> 4381
AAGGGAATGT CGCGCTACCG TTTAACACCG AAGGAATGTA TCGCGCCTGG 50
GGCTACGCAG GCGATACGCC AACCACCGGT ATCTACCGTG AAAAAGGGGA 100
<210> 4382
<211> 100
<212> DNA
<213> Escherichia coli K-12 MG1655
<220> ybik ybiK
<222> 806-905
<223> ybik ybiK U00096 865791..866756
<313> 4382: 840-859
<400> 4382
CTGCGCTTGG CGGTAGCGGT GGCTTAATCG CTATCGACCA TGAAGGGAAT 50
GTCGCGCTAC CGTTTAACAC CGAAGGAATG TATCGCGCCT GGGGCTACGC 100
<210> 4383
<211> 100
<212> DNA

<213> Escherichia coli K-12 MG1655
<220> ybim b0807
<222> 275-374
<223> ybim b0807 U00096 complement(841019..841423)
<313> 4383: 343-362
<400> 4383
GCGCATCAAA CCTAAGCGAT CTGGAGGATA AACTGGCAGA AAAAGCGCGC 50
GAACAAGGCG CGAAAGGTTA TGTCATTAAT TCCGCAGGCG GAAATGACCA 100
<210> 4384
<211> 100
<212> DNA
<213> Escherichia coli K-12 MG1655
<220> ybim b0807
<222> 251-350
<223> ybim b0807 U00096 complement(841019..841423)
<313> 4384: 290-309
<400> 4384
CCGCCGGTAC CGTTTCGGCA ACTGGCGCAT CAAACCTAAG CGATCTGGAG 50
GATAAACTGG CAGAAAAAGC GCGCGAACAA GGCGCGAAAG GTTATGTCAT 100
<210> 4385
<211> 100
<212> DNA
<213> Escherichia coli K-12 MG1655
<220> ybim b0807
<222> 205-304
<223> ybim b0807 U00096 complement(841019..841423)
<313> 4385: 231-250
<400> 4385
TATGCCGCGC AACCGATGAG TAACCTGGAC AGCGGTCAGC TACGGCCCGC 50
CGGTACCGTT TCGGCAACTG GCGCATCAAA CCTAAGCGAT CTGGAGGATA 100
<210> 4386
<211> 100
<212> DNA
<213> Escherichia coli K-12 MG1655
<220> ybin b0808
<222> 909-1008
<223> ybin b0808 U00096 841474..842481
<313> 4386: 982-1001
<400> 4386
TAAAAAAGAG ATGGCCCAGG GGCAAAAGCA GAGTCGCTTT ATTGCCTGGA 50
CCTTTATGAA CGACGAGCAG CGCCGCCGTT TTGTCAATCG CCAGCGTTAA 100
<210> 4387
<211> 100
<212> DNA
<213> Escherichia coli K-12 MG1655
<220> ybin b0808
<222> 893-992
<223> ybin b0808 U00096 841474..842481
<313> 4387: 932-951
<400> 4387
GCGCGGTGAA GGTGGTTAAA AAAGAGATGG CCCAGGGGCA AAAGCAGAGT 50
CGCTTTATTG CCTGGACCTT TATGAACGAC GAGCAGCGCC GCCGTTTTGT 100
<210> 4388
<211> 100
<212> DNA
<213> Escherichia coli K-12 MG1655
<220> ybin b0808
<222> 864-963
<223> ybin b0808 U00096 841474..842481
<313> 4388: 898-917
<400> 4388
GCCGTTGTAT CGTGCCCTGA CGGACGTGGG CGCGGTGAAG GTGGTTAAAA 50
AAGAGATGGC CCAGGGGCAA AAGCAGAGTC GCTTTATTGC CTGGACCTTT 100
<210> 4389
<211> 100
<212> DNA
<213> Escherichia coli K-12 MG1655
<220> b0844 b0844
<222> 690-789
<223> b0844 b0844 U00096 complement(884539..885327)
<313> 4389: 739-758

<400> 4389
CGTCGCAGCG GCAAAATACC GCGCAGGCTC CAATAACCGT GAAGGCGTAC 50
TGGATGTGAT CGATAAAGTT CTTAAACACG AAGCGCCATT TGACCAATAA 100
<210> 4390
<211> 100
<212> DNA
<213> Escherichia coli K-12 MG1655
<220> b0844 b0844
<222> 678-777
<223> b0844 b0844 U00096 complement(884539..885327)
<313> 4390: 702-721
<400> 4390
CGGTAGCGCG GTCGTCGCAG CGGCAAAATA CCGCGCAGGC TCCAATAACC 50
GTGAAGGCGT ACTGGATGTG ATCGATAAAG TTCTTAAACA CGAAGCGCCA 100
<210> 4391
<211> 100
<212> DNA
<213> Escherichia coli K-12 MG1655
<220> b0844 b0844
<222> 616-715
<223> b0844 b0844 U00096 complement(884539..885327)
<313> 4391: 656-675
<400> 4391
GATGGCGGTA ACGATATTGA GATGCTGCGT CAGGCAGGCT TTAGTTTTGC 50
AATGGAAAAT GCCGGTAGCG CGGTCGTCGC AGCGGCAAAA TACCGCGCAG 100
<210> 4392
<211> 100
<212> DNA
<213> Escherichia coli K-12 MG1655
<220> ybio b0809
<222> 2235-2334
<223> ybio b0809 U00096 complement(842478..844838)
<313> 4392: 2302-2321
<400> 4392
CGACAGCCAG GTGAAAAAAC ATTTCGACCT GGCGGGCGTT CGCGCGCCAG 50
TGCAGACTTA TCAGGTGCTG CCTGCTCCGG GCGCGACCCC GGCTGAACCG 100
<210> 4393
<211> 100
<212> DNA
<213> Escherichia coli K-12 MG1655
<220> ybio b0809
<222> 2200-2299
<223> ybio b0809 U00096 complement(842478..844838)
<313> 4393: 2230-2249
<400> 4393
CTGCCACTCA AACAGTGGAC GGTACGCTTT GCCCTCGACA GCCAGGTGAA 50
AAAACATTTC GACCTGGCGG GCGTTCGCGC GCCAGTGCAG ACTTATCAGG 100
<210> 4394
<211> 100
<212> DNA
<213> Escherichia coli K-12 MG1655
<220> ybio b0809
<222> 2149-2248
<223> ybio b0809 U00096 complement(842478..844838)
<313> 4394: 2194-2213
<400> 4394
ATTGTCGGCT TAAGCAATAC CGCGTTTACA CTGCGTGTTT CGTTCACCAC 50
GCTGCCACTC AAACAGTGGA CGGTACGCTT TGCCCTCGAC AGCCAGGTGA 100
<210> 4395
<211> 100
<212> DNA
<213> Escherichia coli K-12 MG1655
<220> b0845 b0845
<222> 1110-1209
<223> b0845 b0845 U00096 complement(885354..886562)
<313> 4395: 1186-1205
<400> 4395
AATGCTGGTT GTACTGGCGC TGGTTATTCT CGCGGCTATT GTCGCGAAAG 50
CCGTCGCCAA ACCCGATACC AAAACGCAGA CGGCGATGGA GAATAGTTGA 100
<210> 4396
<211> 100

<212> DNA
<213> Escherichia coli K-12 MG1655
<220> b0845 b0845
<222> 1026-1125
<223> b0845 b0845 U00096 complement(885354..886562)
<313> 4396: 1048-1067
<400> 4396
GGTAGCAACG ACCGGTTATC TGGCTTTCCT CGTCGGGCCG CCGCTGCTGG 50
GCTATCTCGG CGAACATTAT GGATTACGTA GTGCAATGCT GGTTGTACTG 100
<210> 4397
<211> 100
<212> DNA
<213> Escherichia coli K-12 MG1655
<220> b0845 b0845
<222> 951-1050
<223> b0845 b0845 U00096 complement(885354..886562)
<313> 4397: 1005-1024
<400> 4397
TGCCTCGCTG GGCTTCCCGC TGACCATTTC TGCCGCCAGC GATACCGGCC 50
CCGATGCACC AACCCGCGTC AGTGTGGTAG CAACGACCGG TTATCTGGCT 100
<210> 4398
<211> 100
<212> DNA
<213> Escherichia coli K-12 MG1655
<220> ybip ybiP
<222> 1485-1584
<223> ybip ybiP U00096 complement(850237..851820)
<313> 4398: 1548-1567
<400> 4398
ATCTGAGAAG AAAGCCGGGC CGATATACAT CACCAACTTC CAGTTACAGA 50
AGGTGGATTA CAACCATCTC GGAACCGATA TTTTCGACCC GAAACCTTAA 100
<210> 4399
<211> 100
<212> DNA
<213> Escherichia coli K-12 MG1655
<220> ybip ybiP
<222> 1478-1577
<223> ybip ybiP U00096 complement(850237..851820)
<313> 4399: 1502-1521
<400> 4399
CGTTTATATC TGAGAAGAAA GCCGGGCCGA TATACATCAC CAACTTCCAG 50
TTACAGAAGG TGGATTACAA CCATCTCGGA ACCGATATTT TCGACCCGAA 100
<210> 4400
<211> 100
<212> DNA
<213> Escherichia coli K-12 MG1655
<220> ybip ybiP
<222> 1394-1493
<223> ybip ybiP U00096 complement(850237..851820)
<313> 4400: 1442-1461
<400> 4400
AAGCCCGCCG CTCAGCCAAT GACTTCTTAG GCTTTTTCTC CCAGTGGACG 50
GGGATTAAAG CGAAGGAAAT TAACATCAAA TACCCGTTTA TATCTGAGAA 100
<210> 4401
<211> 100
<212> DNA
<213> Escherichia coli K-12 MG1655
<220> b0846 b0846
<222> 438-537
<223> b0846 b0846 U00096 886646..887182
<313> 4401: 500-519
<400> 4401
TGATGCGTTT ATTGAGGGGA TGACGCTGCA TTTTGTCACC GACCGTAAGC 50
CGCTATCGCG CGAGGAGATT TTGAGGATGG TTGAGAGGGT TGCAGGGTAG 100
<210> 4402
<211> 100
<212> DNA
<213> Escherichia coli K-12 MG1655
<220> b0846 b0846
<222> 438-537
<223> b0846 b0846 U00096 886646..887182

<313> 4402: 467-486
<400> 4402
TGATGCGTTT ATTGAGGGGA TGACGCTGCA TTTTGTCACC GACCGTAAGC 50
CGCTATCGCG CGAGGAGATT TTGAGGATGG TTGAGAGGGT TGCAGGGTAG 100
<210> 4403
<211> 100
<212> DNA
<213> Escherichia coli K-12 MG1655
<220> b0846 b0846
<222> 357-456
<223> b0846 b0846 U00096 886646..887182
<313> 4403: 381-400
<400> 4403
AAAAACGGTA ATGCAAAACT GGATGCAGCG CAGTCAGCAA ACGCTCGAAC 50
AATGGTTTGA ACCCGGAACC GCCCGCGCGC TTGATGCGTT TATTGAGGGG 100
<210> 4404
<211> 100
<212> DNA
<213> Escherichia coli K-12 MG1655
<220> xtha b1750
<222> 708-807
<223> xtha b1750 U00096 1830452..1831258
<313> 4404: 769-788
<400> 4404
GCTGGCAGAA TGTTGCGTAG AAACCGGCAT CGACTATGAA ATCCGCAGCA 50
TGGAAAAACC GTCCGATCAC GCCCCCGTCT GGGCGACCTT CCGCCGCTAA 100
<210> 4405
<211> 100
<212> DNA
<213> Escherichia coli K-12 MG1655
<220> xtha b1750
<222> 678-777
<223> xtha b1750 U00096 1830452..1831258
<313> 4405: 724-743
<400> 4405
GCGCATCGAC CTGCTGCTCG CCAGCCAACC GCTGGCAGAA TGTTGCGTAG 50
AAACCGGCAT CGACTATGAA ATCCGCAGCA TGGAAAAACC GTCCGATCAC 100
<210> 4406
<211> 100
<212> DNA
<213> Escherichia coli K-12 MG1655
<220> xtha b1750
<222> 579-678
<223> xtha b1750 U00096 1830452..1831258
<313> 4406: 604-623
<400> 4406
GGGGTTGGTC GATACCTTCC GCCATGCGAA TCCGCAAACA GCAGATCGTT 50
TCTCATGGTT TGATTACCGC TCAAAAGGTT TTGACGATAA CCGTGGTCTG 100
<210> 4407
<211> 100
<212> DNA
<213> Escherichia coli K-12 MG1655
<220> b0847 b0848
<222> 1571-1670
<223> b0847 b0848 U00096 complement(887357..889042)
<313> 4407: 1607-1626
<400> 4407
ATACAGCTCG CAGTAACATC CCGGCGCTGG GCTATGCGGG CACCTATGCA 50
ATCGCCAACG TCCTGCTGAC GCTGGCAGGG ACAATCATCG TCATGGTATG 100
<210> 4408
<211> 100
<212> DNA
<213> Escherichia coli K-12 MG1655
<220> b0847 b0848
<222> 1508-1607
<223> b0847 b0848 U00096 complement(887357..889042)
<313> 4408: 1560-1579
<400> 4408
CGCTGTTGTT CGGCGCAATG ATGGGCGCAC GTACCTGCGC GCCGGCAATG 50
GAGATCATCA GTGATACAGC TCGCAGTAAC ATCCCGGCGC TGGGCTATGC 100
<210> 4409

<211> 100
<212> DNA
<213> Escherichia coli K-12 MG1655
<220> b0847 b0848
<222> 1430-1529
<223> b0847 b0848 U00096 complement(887357..889042)
<313> 4409: 1465-1484
<400> 4409
TTGCCGGATT GATTGTCAGT CTGGTGCCCG TGGTTATCTG TTTCTTGTTC 50
GGTGCTTATG TATTGCGAAT GAACCGCGCG CTGTTGTTCG GCGCAATGAT 100
<210> 4410
<211> 100
<212> DNA
<213> Escherichia coli K-12 MG1655
<220> ybir b0819
<222> 1015-1114
<223> ybir b0819 U00096 852870..853988
<313> 4410: 1038-1057
<400> 4410
AACGATCGCC GCATCTGGTG GCGTTTCCAT CTCTATTCAA TACCGATGCT 50
GTTGTGGGCG GCGTTGGTGG GATATGTTTT GTTAGTTATA CTCCCGGCCA 100
<210> 4411
<211> 100
<212> DNA
<213> Escherichia coli K-12 MG1655
<220> ybir b0819
<222> 907-1006
<223> ybir b0819 U00096 852870..853988
<313> 4411: 939-958
<400> 4411
TTGCTGAACT ATGTGCCGCC GTCTTTATTA CTGGTATGGG CGGTAAACGT 50
AGGTGGCTTT GGGTTATTAC CCGGATCGCT GGCAAATTTG ATTGCGCTAC 100
<210> 4412
<211> 100
<212> DNA
<213> Escherichia coli K-12 MG1655
<220> ybir b0819
<222> 864-963
<223> ybir b0819 U00096 852870..853988
<313> 4412: 908-927
<400> 4412
CGGTTTATCG CAGGTGATCA GTAACGTGCC GAGTACCATA TTGTTGCTGA 50
ACTATGTGCC GCCGTCTTTA TTACTGGTAT GGGCGGTAAA CGTAGGTGGC 100
<210> 4413
<211> 100
<212> DNA
<213> Escherichia coli K-12 MG1655
<220> ybis b0820
<222> 822-921
<223> ybis b0820 U00096 complement(854047..854967)
<313> 4413: 889-908
<400> 4413
GAAGAGCGTG CAGACAGTGA CCGGTCAGCC AGATGTTGAC CAGGTTGTTC 50
TTGATGAAGC GATTAAAAAC CGCTCCGGGA TGCCGGTTCG TCTGAATTAA 100
<210> 4414
<211> 100
<212> DNA
<213> Escherichia coli K-12 MG1655
<220> ybis b0820
<222> 779-878
<223> ybis b0820 U00096 complement(854047..854967)
<313> 4414: 806-825
<400> 4414
AAGCCCAGTT TGAAGGTCAG GAAATTGTGC CAATTACCCT GACGAAGAGC 50
GTGCAGACAG TGACCGGTCA GCCAGATGTT GACCAGGTTG TTCTTGATGA 100
<210> 4415
<211> 100
<212> DNA
<213> Escherichia coli K-12 MG1655
<220> ybis b0820
<222> 741-840

<223> ybis b0820 U00096 complement(854047..854967)
<313> 4415: 774-793
<400> 4415
CCGTTATATT GAAGTCCATA ACCCGCTGTC TACCACCGAA GCCCAGTTTG 50
AAGGTCAGGA AATTGTGCCA ATTACCCTGA CGAAGAGCGT GCAGACAGTG 100
<210> 4416
<211> 100
<212> DNA
<213> Escherichia coli K-12 MG1655
<220> b1578 b1578
<222> 549-648
<223> b1578 b1578 U00096 1648905..1649561
<313> 4416: 591-610
<400> 4416
TTATAACGAA TGGCATCCGC ATAGTGCGCT GGGTTATCGC TCGCCACGGG 50
AATATCTGCG GCAGCGGGCT TGTAATGGGT TAAGTGATAA CAGATGTCTG 100
<210> 4417
<211> 100
<212> DNA
<213> Escherichia coli K-12 MG1655
<220> b1578 b1578
<222> 488-587
<223> b1578 b1578 U00096 1648905..1649561
<313> 4417: 514-533
<400> 4417
GTATCATGCC CAAACCAGAC GGGTTAACGG CAGCAAAGAA CCTTGCAGAG 50
GCGTTCGAGC ATTATAACGA ATGGCATCCG CATAGTGCGC TGGGTTATCG 100
<210> 4418
<211> 100
<212> DNA
<213> Escherichia coli K-12 MG1655
<220> b1578 b1578
<222> 353-452
<223> b1578 b1578 U00096 1648905..1649561
<313> 4418: 409-428
<400> 4418
CATGCTACCG GGCTAATGAA ACACGCCAGT TCGCCCGGAT GTTGGGACTT 50
GAACCGAAGA ACACGGCGGT GCGGAGTCCG GAGAGTAACG GAATAGCAGA 100
<210> 4419
<211> 100
<212> DNA
<213> Escherichia coli K-12 MG1655
<220> ybit b0821
<222> 1438-1537
<223> ybit b0821 U00096 855186..856778
<313> 4419: 1467-1486
<400> 4419
GCACTGGAAC TGTATCAGGG CACGCTGATC TTTGTTTCAC ACGACCGTGA 50
GTTCGTAAGC TCCCTGGCGA CCCGCATTCT GGAAATCACC CCGGAACGCG 100
<210> 4420
<211> 100
<212> DNA
<213> Escherichia coli K-12 MG1655
<220> ybit b0821
<222> 1382-1481
<223> ybit b0821 U00096 855186..856778
<313> 4420: 1428-1447
<400> 4420
TCATGGACGA ACCGACCAAC CACCTGGATA TGGAATCCAT TGAGTCGCTG 50
AACATGGCAC TGGAACTGTA TCAGGGCACG CTGATCTTTG TTTCACACGA 100
<210> 4421
<211> 100
<212> DNA
<213> Escherichia coli K-12 MG1655
<220> ybit b0821
<222> 1326-1425
<223> ybit b0821 U00096 855186..856778
<313> 4421: 1383-1402
<400> 4421
GGAAAAAGGG CGGATGCTGT TTGGTAAGTT AATGATGCAG AAGCCGAACA 50
TTCTGATCAT GGACGAACCG ACCAACCACC TGGATATGGA ATCCATTGAG 100

<210> 4422
<211> 100
<212> DNA
<213> Escherichia coli K-12 MG1655
<220> b1579 b1580
<222> 1098-1197
<223> b1579 b1580 U00096 1649536..1650732
<313> 4422: 1174-1193
<400> 4422
ATGGATGTCT GAAAACAATA ATGCACAGGT AGCTTTGTTA AATACACAGT 50
TAAGCGAGTT TGCCCCAACC ATGCCCCATA ACGAAGCAAT GAAAAATTAA 100
<210> 4423
<211> 100
<212> DNA
<213> Escherichia coli K-12 MG1655
<220> b1579 b1580
<222> 1002-1101
<223> b1579 b1580 U00096 1649536..1650732
<313> 4423: 1043-1062
<400> 4423
TGCCTGCTGG TCGCTGACAG CTGGTGCTAA CCCGGCATTT ATAGCAAACC 50
AGATGGGCCA TGCAGATGCG CAAATGGTAT TTCAGGTATA CGGAAAATGG 100
<210> 4424
<211> 100
<212> DNA
<213> Escherichia coli K-12 MG1655
<220> b1579 b1580
<222> 964-1063
<223> b1579 b1580 U00096 1649536..1650732
<313> 4424: 989-1008
<400> 4424
CTCCGCCATC GAAAATCATA TCAGTCGAGA CATACTTATG CCTGCTGGTC 50
GCTGACAGCT GGTGCTAACC CGGCATTTAT AGCAAACCAG ATGGGCCATG 100
<210> 4425
<211> 100
<212> DNA
<213> Escherichia coli K-12 MG1655
<220> ybiu ybiU
<222> 1167-1266
<223> ybiu ybiU U00096 complement(857019..858284)
<313> 4425: 1223-1242
<400> 4425
CGACTTCCCG CGCGAGGACT ATGAAACAAA CTGGGAAGGA CGCTTTACGC 50
TTGCCGACCT CAACATTCAC GGTAAGCGAG CGTTGGGCAT GGATGTTTGA 100
<210> 4426
<211> 100
<212> DNA
<213> Escherichia coli K-12 MG1655
<220> ybiu ybiU
<222> 1164-1263
<223> ybiu ybiU U00096 complement(857019..858284)
<313> 4426: 1194-1213
<400> 4426
GGGCGACTTC CCGCGCGAGG ACTATGAAAC AAACTGGGAA GGACGCTTTA 50
CGCTTGCCGA CCTCAACATT CACGGTAAGC GAGCGTTGGG CATGGATGTT 100
<210> 4427
<211> 100
<212> DNA
<213> Escherichia coli K-12 MG1655
<220> ybiu ybiU
<222> 1100-1199
<223> ybiu ybiU U00096 complement(857019..858284)
<313> 4427: 1150-1169
<400> 4427
TGTGCGAGAA AAATCTTGCC TACGCGCACA AGGTGAAGGC CGCACTGGAA 50
AAAGGCGCAT CGCCGGGCGA CTTCCCGCGC GAGGACTATG AAACAAACTG 100
<210> 4428
<211> 100
<212> DNA
<213> Escherichia coli K-12 MG1655
<220> ybiw b0824

<222> 2334-2433
<223> ybiw b0824 U00096 complement(859397..861829)
<313> 4428: 2411-2430
<400> 4428
CGATCTGGTA GTGCGTGTCG CGGGCTATTC CGCGTTCTTC ACCGCGCTCT 50
CTCCAGACGC TCAGGACGAT ATCATCGCCC GTACTGAACA TATGCTGTAA 100
<210> 4429
<211> 100
<212> DNA
<213> Escherichia coli K-12 MG1655
<220> ybiw b0824
<222> 2276-2375
<223> ybiw b0824 U00096 complement(859397..861829)
<313> 4429: 2296-2315
<400> 4429
ACAACATCGT TTCCCGCGAA ACGCTGCTGG ATGCGAAAAA ACATCCCGAT 50
CAGTATCGCG ATCTGGTAGT GCGTGTCGCG GGCTATTCCG CGTTCTTCAC 100
<210> 4430
<211> 100
<212> DNA
<213> Escherichia coli K-12 MG1655
<220> ybiw b0824
<222> 2235-2334
<223> ybiw b0824 U00096 complement(859397..861829)
<313> 4430: 2272-2291
<400> 4430
GCGTACCTTC TTTGAAGTGC ATAAAGGCTG GCATATTCAG TACAACATCG 50
TTTCCCGCGA AACGCTGCTG GATGCGAAAA AACATCCCGA TCAGTATCGC 100
<210> 4431
<211> 100
<212> DNA
<213> Escherichia coli K-12 MG1655
<220> ybix ybiX
<222> 615-714
<223> ybix ybiX U00096 complement(837753..838466)
<313> 4431: 670-689
<400> 4431
CAATATTCAG TCGCTGAAAA GCCGCTACGG CGAAAGTGAA GAGATCCTGT 50
CGCTGCTTAA TCTTTATCAT AATCTGCTGC GGGAATGGTC GGAGATCTGA 100
<210> 4432
<211> 100
<212> DNA
<213> Escherichia coli K-12 MG1655
<220> ybix ybiX
<222> 564-663
<223> ybix ybiX U00096 complement(837753..838466)
<313> 4432: 611-630
<400> 4432
GTCGATGATC CGCGATGATA AAAAGCGCGC CATGCTGTTT GAACTGGACA 50
ACAATATTCA GTCGCTGAAA AGCCGCTACG GCGAAAGTGA AGAGATCCTG 100
<210> 4433
<211> 100
<212> DNA
<213> Escherichia coli K-12 MG1655
<220> ybix ybiX
<222> 452-551
<223> ybix ybiX U00096 complement(837753..838466)
<313> 4433: 496-515
<400> 4433
AACATCGGGT AAAACTCCCG GCAGGCGATC TCGTGTTGTA TCCCTCCAGC 50
AGCCTGCATT GCGTGACACC CGTAACCCGC GGCGTACGAG TGGCATCATT 100
<210> 4434
<211> 100
<212> DNA
<213> Escherichia coli K-12 MG1655
<220> ybiy b0825
<222> 828-927
<223> ybiy b0825 U00096 complement(861835..862761)
<313> 4434: 871-890
<400> 4434
CTATGACGCC CCGGAAAAAC CGCTTGATGC GCCAGAACTG CTCGACTTTG 50

CCCAGCAGTA TGCCTGCCAG AAAGGGTTAA CCGCGACCTT ACGAGGATAA 100
<210> 4435
<211> 100
<212> DNA
<213> Escherichia coli K-12 MG1655
<220> ybiy b0825
<222> 782-881
<223> ybiy b0825 U00096 complement(861835..862761)
<313> 4435: 834-853
<400> 4435
CCTACCACAC GCTGGGCATC AACAAATATC ACTTACTTAA TCTGCCCTAT 50
GACGCCCCGG AAAAACCGCT TGATGCGCCA GAACTGCTCG ACTTTGCCCA 100
<210> 4436
<211> 100
<212> DNA
<213> Escherichia coli K-12 MG1655
<220> ybiy b0825
<222> 733-832
<223> ybiy b0825 U00096 complement(861835..862761)
<313> 4436: 792-811
<400> 4436
ACCGATTTTG CCGCCGACGA GCTGCACGTT GGCGAAATTC ATTTTCTGCC 50
CTACCACACG CTGGGCATCA ACAAATATCA CTTACTTAAT CTGCCCTATG 100
<210> 4437
<211> 100
<212> DNA
<213> Escherichia coli K-12 MG1655
<220> aspa aspA
<222> 1383-1482
<223> aspa aspA U00096 complement(4364469..4365950)
<313> 4437: 1457-1476
<400> 4437
TCTGTTGACT GAAGCGGAAC TTGACGATAT TTTCTCCGTA CAGAATCTGA 50
TGCACCCGGC TTACAAAGCA AAACGCTATA CTGATGAAAG CGAACAGTAA 100
<210> 4438
<211> 100
<212> DNA
<213> Escherichia coli K-12 MG1655
<220> aspa aspA
<222> 1360-1459
<223> aspa aspA U00096 complement(4364469..4365950)
<313> 4438: 1394-1413
<400> 4438
CGTGAAGTCG TTCTGGAACG CGGTCTGTTG ACTGAAGCGG AACTTGACGA 50
TATTTTCTCC GTACAGAATC TGATGCACCC GGCTTACAAA GCAAAACGCT 100
<210> 4439
<211> 100
<212> DNA
<213> Escherichia coli K-12 MG1655
<220> aspa aspA
<222> 1294-1393
<223> aspa aspA U00096 complement(4364469..4365950)
<313> 4439: 1323-1342
<400> 4439
CCGTTCATCG GTCACCACAA CGGTGACATC GTGGGTAAAA TCTGTGCCGA 50
AACCGGTAAG AGTGTACGTG AAGTCGTTCT GGAACGCGGT CTGTTGACTG 100
<210> 4440
<211> 100
<212> DNA
<213> Escherichia coli K-12 MG1655
<220> aspc b0929
<222> 1092-1191
<223> aspc b0929 U00096 complement(983742..984932)
<313> 4440: 1153-1172
<400> 4440
GTTTGGCGTA TATGCGGTTG CTTCTGGTCG CGTAAATGTG GCCGGGATGA 50
CACCAGATAA CATGGCTCCG CTGTGCGAAG CGATTGTGGC AGTGCTGTAA 100
<210> 4441
<211> 100
<212> DNA
<213> Escherichia coli K-12 MG1655

<220> aspc b0929
<222> 1042-1141
<223> aspc b0929 U00096 complement(983742..984932)
<313> 4441: 1078-1097
<400> 4441
TTCTCCTTCA GTGGCCTGAC AAAAGAACAA GTGCTGCGTC TGCGCGAAGA  50
GTTTGGCGTA TATGCGGTTG CTTCTGGTCG CGTAAATGTG GCCGGGATGA  100
<210> 4442
<211> 100
<212> DNA
<213> Escherichia coli K-12 MG1655
<220> aspc b0929
<222> 1012-1111
<223> aspc b0929 U00096 complement(983742..984932)
<313> 4442: 1041-1060
<400> 4442
TTCAGCTTTA TCATCAAACA GAACGGCATG TTCTCCTTCA GTGGCCTGAC  50
AAAAGAACAA GTGCTGCGTC TGCGCGAAGA GTTTGGCGTA TATGCGGTTG  100
<210> 4443
<211> 100
<212> DNA
<213> Escherichia coli K-12 MG1655
<220> ybjc b0851
<222> 189-288
<223> ybjc b0851 U00096 890136..890423
<313> 4443: 245-264
<400> 4443
TATTCTTCAG GTGGCAAAAC GTCTTGCCCC ACAGCTGATG AACCGTCCAC  50
CGCAATATTC ACGTTCAGAA AGAGAAAAAG ATAATGACGC CAACCATTGA  100
<210> 4444
<211> 100
<212> DNA
<213> Escherichia coli K-12 MG1655
<220> ybjc b0851
<222> 138-237
<223> ybjc b0851 U00096 890136..890423
<313> 4444: 200-219
<400> 4444
TCTGATGATT TTTCTCGGTG TTTTGCTCAT GCTTCCCGCT GCGGTGGTGG  50
TTATTCTTCA GGTGGCAAAA CGTCTTGCCC CACAGCTGAT GAACCGTCCA  100
<210> 4445
<211> 100
<212> DNA
<213> Escherichia coli K-12 MG1655
<220> ybjc b0851
<222> 109-208
<223> ybjc b0851 U00096 890136..890423
<313> 4445: 154-173
<400> 4445
GAGCCATTTT CTCGGCCAGA AGTGCAGATT CTGATGATTT TTCTCGGTGT  50
TTTGCTCATG CTTCCCGCTG CGGTGGTGGT TATTCTTCAG GTGGCAAAAC  100
<210> 4446
<211> 100
<212> DNA
<213> Escherichia coli K-12 MG1655
<220> ybjd b0877
<222> 1560-1659
<223> ybjd b0877 U00096 915696..917354
<313> 4446: 1589-1608
<400> 4446
GGCAATGGAG GCAGGACGTC GTGGTGTGGA CTCCGTACCG ACGCTGCTGA  50
AAAAAATGTT CTCACGCGTG CTGTGGCTGG CGCGCGGTCG CGCGGATTAA  100
<210> 4447
<211> 100
<212> DNA
<213> Escherichia coli K-12 MG1655
<220> ybjd b0877
<222> 1493-1592
<223> ybjd b0877 U00096 915696..917354
<313> 4447: 1534-1553
<400> 4447

TGAATCTACG CAAAATTATC TCGAAAGCGA TCCATCGCTC TTCCAAACCC 50
GATCTTGCCA TTGAAGTGGC AATGGAGGCA GGACGTCGTG GTGTGGACTC 100
<210> 4448
<211> 100
<212> DNA
<213> Escherichia coli K-12 MG1655
<220> ybjd b0877
<222> 1440-1539
<223> ybjd b0877 U00096 915696..917354
<313> 4448: 1485-1504
<400> 4448
GGGATTTTCC GATGTGTTCC ACCGCATGGC GCAAATCCCG GAAAATGTAC 50
CGATGAATCT ACGCAAAATT ATCTCGAAAG CGATCCATCG CTCTTCCAAA 100
<210> 4449
<211> 100
<212> DNA
<213> Escherichia coli K-12 MG1655
<220> ybje b0875
<222> 849-948
<223> ybje b0875 U00096 complement(913181..914128)
<313> 4449: 911-930
<400> 4449
TCAACGTACT GGCGGGCTGG ATATGGTCCC GGCGGCAATT GTTCACGGTT 50
TTATTCTTAG CCTGTTAGTG CCGATCCTCA TCGCCTTTTT CTCTGCGTAA 100
<210> 4450
<211> 100
<212> DNA
<213> Escherichia coli K-12 MG1655
<220> ybje b0875
<222> 808-907
<223> ybje b0875 U00096 complement(913181..914128)
<313> 4450: 863-882
<400> 4450
TTATGCGGTG CCACATCAAT GGATTTCACC CTGCCCGTTC TTCAACGTAC 50
TGGCGGGCTG GATATGGTCC CGGCGGCAAT TGTTCACGGT TTTATTCTTA 100
<210> 4451
<211> 100
<212> DNA
<213> Escherichia coli K-12 MG1655
<220> ybje b0875
<222> 799-898
<223> ybje b0875 U00096 complement(913181..914128)
<313> 4451: 827-846
<400> 4451
GCACTGGGCT TATGCGGTGC CACATCAATG GATTTCACCC TGCCCGTTCT 50
TCAACGTACT GGCGGGCTGG ATATGGTCCC GGCGGCAATT GTTCACGGTT 100
<210> 4452
<211> 100
<212> DNA
<213> Escherichia coli K-12 MG1655
<220> ybjf b0860
<222> 1029-1128
<223> ybjf b0860 U00096 897741..898868
<313> 4452: 1094-1113
<400> 4452
CCGCGAACTG CCTGGGTTTC GTATTGAACG GGTGCAGCTT TTCGATATGT 50
TCCCGCATAC CGCGCACTAT GAAGTGCTGA CGCTGCTGGT GAAGCAATAA 100
<210> 4453
<211> 100
<212> DNA
<213> Escherichia coli K-12 MG1655
<220> ybjf b0860
<222> 994-1093
<223> ybjf b0860 U00096 897741..898868
<313> 4453: 1010-1029
<400> 4453
TCCAGCTGTA ACGCCCAAAC TATGGCGAAA GATATCCGCG AACTGCCTGG 50
GTTTCGTATT GAACGGGTGC AGCTTTTCGA TATGTTCCCG CATACCGCGC 100
<210> 4454
<211> 100
<212> DNA

<213> Escherichia coli K-12 MG1655
<220> ybjf b0860
<222> 945-1044
<223> ybjf b0860 U00096 897741..898868
<313> 4454: 978-997
<400> 4454
ACCGCTGTGT GATTATCTCT CAACGATGGC ACCGCGTTTT ATCATCTACT 50
CCAGCTGTAA CGCCCAAACT ATGGCGAAAG ATATCCGCGA ACTGCCTGGG 100
<210> 4455
<211> 100
<212> DNA
<213> Escherichia coli K-12 MG1655
<220> ybjg b0842
<222> 498-597
<223> ybjg b0842 U00096 complement(882015..882611)
<313> 4455: 565-584
<400> 4455
TATCTGGCAA GCGATGGGGC ATAAACTCTA TCAACGTCTG CAATCGTGGT 50
ATCGCGTCTG TTTTGCATTA CCGATCCGCA AAGGCTGGGT GCGTGACTGA 100
<210> 4456
<211> 100
<212> DNA
<213> Escherichia coli K-12 MG1655
<220> ybjg b0842
<222> 445-544
<223> ybjg b0842 U00096 complement(882015..882611)
<313> 4456: 475-494
<400> 4456
GATATGCTCG GTGGATTGCT GGCAGGTATG ATTGGCTGCC TTAGTGCCCA 50
GATTATCTGG CAAGCGATGG GGCATAAACT CTATCAACGT CTGCAATCGT 100
<210> 4457
<211> 100
<212> DNA
<213> Escherichia coli K-12 MG1655
<220> ybjg b0842
<222> 394-493
<223> ybjg b0842 U00096 complement(882015..882611)
<313> 4457: 425-444
<400> 4457
GCCGTCGTCA TTGCCTGGTC GCGCGTTTAT CTTGGCGTCC ACTGGCCGCT 50
GGATATGCTC GGTGGATTGC TGGCAGGTAT GATTGGCTGC CTTAGTGCCC 100
<210> 4458
<211> 100
<212> DNA
<213> Escherichia coli K-12 MG1655
<220> ybjh ybjH
<222> 173-272
<223> ybjh ybjH U00096 complement(884169..884453)
<313> 4458: 201-220
<400> 4458
ATGTGGCTCC TGCGCCAGCT CCGGCTTACC GCCCGCATCC TTATGTTCGC 50
CATCTTGCCA GCGTAGGTGA AGGGATGGTT ATCGAACACC AGATTGACGA 100
<210> 4459
<211> 100
<212> DNA
<213> Escherichia coli K-12 MG1655
<220> ybjh ybjH
<222> 93-192
<223> ybjh ybjH U00096 complement(884169..884453)
<313> 4459: 129-148
<400> 4459
TGGATACAAA GTGGTTGTGG TTCCTGATTC AGTCAGCGTT CCACAAGCGG 50
TCAGCGTCGC GACTGTGCCG CAAACCGTCT ATGTGGCTCC TGCGCCAGCT 100
<210> 4460
<211> 100
<212> DNA
<213> Escherichia coli K-12 MG1655
<220> ybjh ybjH
<222> 1-100
<223> ybjh ybjH U00096 complement(884169..884453)
<313> 4460: 44-63

<400> 4460
ATGATTATGA AAAATTGTCT ACTGTTGGGC GCGCTTTTAA TGGGCTTTAC  50
TGGTGTGGCG ATGGCGCAAA GTGTCACCGT CGATGTGCCG AGTGGATACA  100
<210> 4461
<211> 100
<212> DNA
<213> Escherichia coli K-12 MG1655
<220> ybjm b0849
<222> 257-356
<223> ybjm b0849 U00096 889312..889689
<313> 4461: 279-298
<400> 4461
CGCGCTCATT CTGGCAAGAG CTGGCATGGT TACTAAGCGC GGTGTTCTGG  50
TGTGCGCTGG GGGCACTGTG TTTCTTATTT ATCAGTAGTT TGTTTAAACC  100
<210> 4462
<211> 100
<212> DNA
<213> Escherichia coli K-12 MG1655
<220> ybjm b0849
<222> 157-256
<223> ybjm b0849 U00096 889312..889689
<313> 4462: 183-202
<400> 4462
AGCTTCTTTT CACAGCGTAG AGAAGTCCTG AAACCTCTGT TTGGCGCAAT  50
GCTGGCGGCA CCCTGTTCGA TGCTCATTAT GCGGCTGTTT TTTTCACCGA  100
<210> 4463
<211> 100
<212> DNA
<213> Escherichia coli K-12 MG1655
<220> ybjm b0849
<222> 84-183
<223> ybjm b0849 U00096 889312..889689
<313> 4463: 142-161
<400> 4463
GAAAGGCGCT TTTCGGGCTG CCGGGCATCC TGAAATCGGC TTGCTATTTT  50
TCATTCTTCC TGGAGCAGTC GCCAGCTTCT TTTCACAGCG TAGAGAAGTC  100
<210> 4464
<211> 100
<212> DNA
<213> Escherichia coli K-12 MG1655
<220> b1582 b1582
<222> 205-304
<223> b1582 b1582 U00096 complement(1653371..1653697)
<313> 4464: 261-280
<400> 4464
TGCACGGCGT TGATGTGGCT GCGCGTTGTG GATGGCGTGA AACTGACTCT  50
TTATGACTGG ACGGGTGCGT TGATTGCGCT TTGCGGCATG TTGATCATTG  100
<210> 4465
<211> 100
<212> DNA
<213> Escherichia coli K-12 MG1655
<220> b1582 b1582
<222> 148-247
<223> b1582 b1582 U00096 complement(1653371..1653697)
<313> 4465: 185-204
<400> 4465
ACGTTGCATC CAGCGGCGAG TGGGCGTGTT TACGCGGCTT ATGGTGGCGT  50
TTATGTCTGC ACGGCGTTGA TGTGGCTGCG CGTTGTGGAT GGCGTGAAAC  100
<210> 4466
<211> 100
<212> DNA
<213> Escherichia coli K-12 MG1655
<220> b1582 b1582
<222> 73-172
<223> b1582 b1582 U00096 complement(1653371..1653697)
<313> 4466: 98-117
<400> 4466
TGGTTAAAAC GAAACGCCAG TATCTGGCTG TTGCTTCCGG CGGGGATTTC  50
ACTGGCGCTG TTTGTCTGGT TGTTAACGTT GCATCCAGCG GCGAGTGGGC  100
<210> 4467
<211> 100

<212> DNA
<213> Escherichia coli K-12 MG1655
<220> ybjn b0854
<222> 378-477
<223> ybjn b0854 U00096 892180..892656
<313> 4467: 451-470
<400> 4467
GATTTCGATG GTCATTCTTG AAGCTAATGC CCATCAACTG CTGTTACCGA 50
CTGATGATGA AGGGCAAAAC AACGTTACCG AAAACTATTT CCTCCACTGA 100
<210> 4468
<211> 100
<212> DNA
<213> Escherichia coli K-12 MG1655
<220> ybjn b0854
<222> 378-477
<223> ybjn b0854 U00096 892180..892656
<313> 4468: 412-431
<400> 4468
GATTTCGATG GTCATTCTTG AAGCTAATGC CCATCAACTG CTGTTACCGA 50
CTGATGATGA AGGGCAAAAC AACGTTACCG AAAACTATTT CCTCCACTGA 100
<210> 4469
<211> 100
<212> DNA
<213> Escherichia coli K-12 MG1655
<220> ybjn b0854
<222> 321-420
<223> ybjn b0854 U00096 892180..892656
<313> 4469: 352-371
<400> 4469
TATGCAGGGC GTAACCTATG AGCAGTTTGC ATGGTTCGTG CGTCAGAGCG 50
AAGAGCAGAT TTCGATGGTC ATTCTTGAAG CTAATGCCCA TCAACTGCTG 100
<210> 4470
<211> 100
<212> DNA
<213> Escherichia coli K-12 MG1655
<220> b1583 b1584
<222> 243-342
<223> b1583 b1584 U00096 1653832..1654173
<313> 4470: 313-332
<400> 4470
CCGCGATAAA TGTGAGCAAA GCGCCAACAT CAATGCCTAC TGGGAGCCCA 50
ATACTTTGCG CTGCCTGGAC CGTCGAACTG GCCGCGTTAT TACCCCCTAA 100
<210> 4471
<211> 100
<212> DNA
<213> Escherichia coli K-12 MG1655
<220> b1583 b1584
<222> 229-328
<223> b1583 b1584 U00096 1653832..1654173
<313> 4471: 275-294
<400> 4471
GCCGCTTTTG ATAACCGCGA TAAATGTGAG CAAAGCGCCA ACATCAATGC 50
CTACTGGGAG CCCAATACTT TGCGCTGCCT GGACCGTCGA ACTGGCCGCG 100
<210> 4472
<211> 100
<212> DNA
<213> Escherichia coli K-12 MG1655
<220> b1583 b1584
<222> 177-276
<223> b1583 b1584 U00096 1653832..1654173
<313> 4472: 239-258
<400> 4472
TCTGCGCCAG AAAGTGAATA AACGCACTGA AAAAGAGTGG GATAAAGCCG 50
ACGCCGCTTT TGATAACCGC GATAAATGTG AGCAAAGCGC CAACATCAAT 100
<210> 4473
<211> 100
<212> DNA
<213> Escherichia coli K-12 MG1655
<220> ybjo b0859
<222> 390-489
<223> ybjo b0859 U00096 897212..897700

<313> 4473: 448-467
<400> 4473
AATCTTCCAT AGCCTGATGC TGCAGAAGCT GCCGGATATG CTCATCCTGA 50
TGCTGCTGTT CGTTCCCTCG ACCAGTCGGC GGTTCTTCCA GTTGCAATAA 100
<210> 4474
<211> 100
<212> DNA
<213> Escherichia coli K-12 MG1655
<220> ybjo b0859
<222> 330-429
<223> ybjo b0859 U00096 897212..897700
<313> 4474: 376-395
<400> 4474
GGCTTCGCTG GGGTACGGTT ATCCGGAGCT GTTCAGCATT CCCGGTGAAT 50
CAAAACGTGA AATCTTCCAT AGCCTGATGC TGCAGAAGCT GCCGGATATG 100
<210> 4475
<211> 100
<212> DNA
<213> Escherichia coli K-12 MG1655
<220> ybjo b0859
<222> 302-401
<223> ybjo b0859 U00096 897212..897700
<313> 4475: 320-339
<400> 4475
AAATCACCGC CGCAAGTTAC TTGTGGGCGG CTTCGCTGGG GTACGGTTAT 50
CCGGAGCTGT TCAGCATTCC CGGTGAATCA AAACGTGAAA TCTTCCATAG 100
<210> 4476
<211> 100
<212> DNA
<213> Escherichia coli K-12 MG1655
<220> yi21_1 b0361
<222> 312-411
<223> yi21_1 b0361 U00096 380530..380940
<313> 4476: 368-387
<400> 4476
GAAAACGATG GAAAATGAAC TCCTCAAAGA AGCCGTTGAA TATGGACGGG 50
CAAAAAAGTG GATAGCGCAC GCGCCCTTAT TGCCCGGGGA TGGGGAGTAA 100
<210> 4477
<211> 100
<212> DNA
<213> Escherichia coli K-12 MG1655
<220> yi21_1 b0361
<222> 295-394
<223> yi21_1 b0361 U00096 380530..380940
<313> 4477: 313-332
<400> 4477
CAGCGCCTGC TCGGCAAGAA AACGATGGAA AATGAACTCC TCAAAGAAGC 50
CGTTGAATAT GGACGGGCAA AAAAGTGGAT AGCGCACGCG CCCTTATTGC 100
<210> 4478
<211> 100
<212> DNA
<213> Escherichia coli K-12 MG1655
<220> yi21_1 b0361
<222> 243-342
<223> yi21_1 b0361 U00096 380530..380940
<313> 4478: 282-301
<400> 4478
GGTTGTTCCT GCCTCTGAAC TTGCTGCCGC CATGAAGCAG ATTAAAGAAC 50
TCCAGCGCCT GCTCGGCAAG AAAACGATGG AAAATGAACT CCTCAAAGAA 100
<210> 4479
<211> 100
<212> DNA
<213> Escherichia coli K-12 MG1655
<220> ybjp ybjP
<222> 417-516
<223> ybjp ybjP U00096 complement(903175..903690)
<313> 4479: 487-506
<400> 4479
TCAGGAAGGC CAGTGTTGGG TCATCGACGA TGTGCGTTAC TTAGGCGGTA 50
GCGTTCATGC CACGGCGGGT ACGCTCCGTC AGTCCATCGA GAATCGCTAA 100
<210> 4480

<211> 100
<212> DNA
<213> Escherichia coli K-12 MG1655
<220> ybjp ybjP
<222> 356-455
<223> ybjp ybjP U00096 complement(903175..903690)
<313> 4480: 396-415
<400> 4480
TTCCATTGCG CGTGGATCTC AAACAGGGTG ACCAGGGTTG GCAGGATGAA 50
GTGTTAATGA TTCAGGAAGG CCAGTGTTGG GTCATCGACG ATGTGCGTTA 100
<210> 4481
<211> 100
<212> DNA
<213> Escherichia coli K-12 MG1655
<220> ybjp ybjP
<222> 282-381
<223> ybjp ybjP U00096 complement(903175..903690)
<313> 4481: 319-338
<400> 4481
CAGTCGAACC ACCTTGCCAG ATAGCGCCCA CGTTGCCAGT GCATCGACTA 50
TCCCTAATCG TGATGCCCGT AACATTCCAT TGCGCGTGGA TCTCAAACAG 100
<210> 4482
<211> 100
<212> DNA
<213> Escherichia coli K-12 MG1655
<220> yi21_2 b1404
<222> 310-409
<223> yi21_2 b1404 U00096 complement(1466808..1467218)
<313> 4482: 366-385
<400> 4482
AAGAAAACGA TGGAAAATGA ACTCCTCAAA GAAGCCGTTG AATATGGACG 50
GGCAAAAAAG TGGATAGCGC ACGCGCCCTT ATTGCCCGGG GATGGGGAGT 100
<210> 4483
<211> 100
<212> DNA
<213> Escherichia coli K-12 MG1655
<220> yi21_2 b1404
<222> 288-387
<223> yi21_2 b1404 U00096 complement(1466808..1467218)
<313> 4483: 321-340
<400> 4483
AGAACTCCAG CGCCTGCTCG GCAAGAAAAC GATGGAAAAT GAACTCCTCA 50
AAGAAGCCGT TGAATATGGA CGGGCAAAAA AGTGGATAGC GCACGCGCCC 100
<210> 4484
<211> 100
<212> DNA
<213> Escherichia coli K-12 MG1655
<220> yi21_2 b1404
<222> 232-331
<223> yi21_2 b1404 U00096 complement(1466808..1467218)
<313> 4484: 281-300
<400> 4484
GCCGGAGAAC AGGTTGTTCC TGCCTCTGAA CTTGCTGCCG CCATGAAGCA 50
GATTAAAGAA CTCCAGCGCC TGCTCGGCAA GAAAACGATG GAAAATGAAC 100
<210> 4485
<211> 100
<212> DNA
<213> Escherichia coli K-12 MG1655
<220> b1586 b1586
<222> 240-339
<223> b1586 b1586 U00096 1655547..1655894
<313> 4485: 289-308
<400> 4485
CCATTGCGCT AATGATACGC ATAAAATTCT CTATACCCGC ACCACCAGCG 50
GTAACGTCTC TGCTCCCGCG CAGTCTAGCC AGGATGGCGC GCCTGCCGAA 100
<210> 4486
<211> 100
<212> DNA
<213> Escherichia coli K-12 MG1655
<220> b1586 b1586
<222> 194-293

<223> b1586 b1586 U00096 1655547..1655894
<313> 4486: 253-272
<400> 4486
TTGTACCCAA TGACCAGGTT GATCAGCCTG ATTCCCAGGT CGTCGGCCAT 50
TGCGCTAATG ATACGCATAA AATTCTCTAT ACCCGCACCA CCAGCGGTAA 100
<210> 4487
<211> 100
<212> DNA
<213> Escherichia coli K-12 MG1655
<220> b1586 b1586
<222> 164-263
<223> b1586 b1586 U00096 1655547..1655894
<313> 4487: 177-196
<400> 4487
GCGTACCCGA AAGCAGCTTC ACGTTAAGTA TTGTACCCAA TGACCAGGTT 50
GATCAGCCTG ATTCCCAGGT CGTCGGCCAT TGCGCTAATG ATACGCATAA 100
<210> 4488
<211> 100
<212> DNA
<213> Escherichia coli K-12 MG1655
<220> bara b2787
<222> 2649-2748
<223> bara b2787 U00096 2913079..2915835
<313> 4488: 2695-2714
<400> 4488
ACAACAGCTA CGTAGCGGTA CTAAAGAAGA AGATTTGGAA CCGGAGCTGC 50
TGGAACTGTT GGACGAGATG GATAATGTCG CGCGCGAAGC CAGCAAAATT 100
<210> 4489
<211> 100
<212> DNA
<213> Escherichia coli K-12 MG1655
<220> bara b2787
<222> 2606-2705
<223> bara b2787 U00096 2913079..2915835
<313> 4489: 2626-2645
<400> 4489
ATAGCGGTGT GCCACGTATG AAGAATCTCT GCCAACTGAT CGAACAACAG 50
CTACGTAGCG GTACTAAAGA AGAAGATTTG GAACCGGAGC TGCTGGAACT 100
<210> 4490
<211> 100
<212> DNA
<213> Escherichia coli K-12 MG1655
<220> bara b2787
<222> 2491-2590
<223> bara b2787 U00096 2913079..2915835
<313> 4490: 2519-2538
<400> 4490
CAAATGTTAC TCGATTTCCT GCCTGAAGTT CGCAACAAAG TTGAGGAACA 50
GCTGGTTGGA GAAAACCCGG AAGGCCTGGT TGATTTGATT CATAAACTGC 100
<210> 4491
<211> 100
<212> DNA
<213> Escherichia coli K-12 MG1655
<220> yi21_3 yi21_3
<222> 312-411
<223> yi21_3 yi21_3 U00096 complement(2067837..2068247)
<313> 4491: 361-380
<400> 4491
GAAAACGATG GAAAATGAAC TCCTCAAAGA AGCCGTTGAA TATGGACGGG 50
CAAAAAAGTG GATAGCGCAC GCGCCCTTAT TGCCCGGGGA TGGGGAGTAA 100
<210> 4492
<211> 100
<212> DNA
<213> Escherichia coli K-12 MG1655
<220> yi21_3 yi21_3
<222> 267-366
<223> yi21_3 yi21_3 U00096 complement(2067837..2068247)
<313> 4492: 310-329
<400> 4492
TGCCGCCATG AAGCAGATTA AAGAACTCCA GCGCCTGCTC GGCAAGAAAA 50
CGATGGAAAA TGAACTCCTC AAAGAAGCCG TTGAATATGG ACGGGCAAAA 100

<210> 4493
<211> 100
<212> DNA
<213> Escherichia coli K-12 MG1655
<220> yi21_3 yi21_3
<222> 226-325
<223> yi21_3 yi21_3 U00096 complement(2067837..2068247)
<313> 4493: 269-288
<400> 4493
GTCGCCGCCG GAGAACAGGT TGTTCCTGCC TCTGAACTTG CTGCCGCCAT 50
GAAGCAGATT AAAGAACTCC AGCGCCTGCT CGGCAAGAAA ACGATGGAAA 100
<210> 4494
<211> 100
<212> DNA
<213> Escherichia coli K-12 MG1655
<220> asps b1867
<222> 1674-1773
<223> asps b1867 U00096 complement(1946774..1948546)
<313> 4494: 1737-1756
<400> 4494
GGCGGCAGCG TGTCTGATGA CTGAAGCACC GAGCTTTGCT AACCCGACTG 50
CACTGGCTGA GCTGAGCATT CAGGTTGTGA AGAAGGCTGA GAATAACTGA 100
<210> 4495
<211> 100
<212> DNA
<213> Escherichia coli K-12 MG1655
<220> asps b1867
<222> 1649-1748
<223> asps b1867 U00096 complement(1946774..1948546)
<313> 4495: 1703-1722
<400> 4495
ACGTTATCGC CTTCCCGAAA ACCACGGCGG CAGCGTGTCT GATGACTGAA 50
GCACCGAGCT TTGCTAACCC GACTGCACTG GCTGAGCTGA GCATTCAGGT 100
<210> 4496
<211> 100
<212> DNA
<213> Escherichia coli K-12 MG1655
<220> asps b1867
<222> 1629-1728
<223> asps b1867 U00096 complement(1946774..1948546)
<313> 4496: 1662-1681
<400> 4496
CGGCACCGAC AATATCCGTG ACGTTATCGC CTTCCCGAAA ACCACGGCGG 50
CAGCGTGTCT GATGACTGAA GCACCGAGCT TTGCTAACCC GACTGCACTG 100
<210> 4497
<211> 100
<212> DNA
<213> Escherichia coli K-12 MG1655
<220> b1587 b1587
<222> 2328-2427
<223> b1587 b1587 U00096 1656093..1658519
<313> 4497: 2397-2416
<400> 4497
CCATGGCGGC TGTGTGAATA CGCTAACCAC TCTGCGCCCT TCACCACTGG 50
CGAAGGGAAA CCCGCAGCAC ACTAATCTGG TCGAGATCGA AAAAATATAA 100
<210> 4498
<211> 100
<212> DNA
<213> Escherichia coli K-12 MG1655
<220> b1587 b1587
<222> 2312-2411
<223> b1587 b1587 U00096 1656093..1658519
<313> 4498: 2357-2376
<400> 4498
CTGGCGATAA AATCGACCAT GGCGGCTGTG TGAATACGCT AACCACTCTG 50
CGCCCTTCAC CACTGGCGAA GGGAAACCCG CAGCACACTA ATCTGGTCGA 100
<210> 4499
<211> 100
<212> DNA
<213> Escherichia coli K-12 MG1655
<220> b1587 b1587

<222> 2244-2343
<223> b1587 b1587 U00096 1656093..1658519
<313> 4499: 2297-2316
<400> 4499
AGTAACACCA CGTATTCTCC CTGGAGTTAG CGCTATGGGC CAGGGAGCCT 50
GGCACGAGGC CAATATGTCT GGCGATAAAA TCGACCATGG CGGCTGTGTG 100
<210> 4500
<211> 100
<212> DNA
<213> Escherichia coli K-12 MG1655
<220> yi21_4 yi21_4
<222> 267-366
<223> yi21_4 yi21_4 U00096 complement(2995257..2995622)
<313> 4500: 326-345
<400> 4500
GAAAACGATG GAAAATGAAC TCCTCAAAGA AGCCGTTGAA TATGGACGGG 50
CAAAAAAGTG GATAGCGCAC GCGCCCTTAT TGCCCGGGGA TGGGGAGTAA 100
<210> 4501
<211> 100
<212> DNA
<213> Escherichia coli K-12 MG1655
<220> yi21_4 yi21_4
<222> 219-318
<223> yi21_4 yi21_4 U00096 complement(2995257..2995622)
<313> 4501: 279-298
<400> 4501
TGCTGCCGCC ATGAAGCAGA TTAAAGAACT CCAGCGCCTG CTCGGCAAGA 50
AAACGATGGA AAATGAACTC CTCAAAGAAG CCGTTGAATA TGGACGGGCA 100
<210> 4502
<211> 100
<212> DNA
<213> Escherichia coli K-12 MG1655
<220> yi21_4 yi21_4
<222> 197-296
<223> yi21_4 yi21_4 U00096 complement(2995257..2995622)
<313> 4502: 224-243
<400> 4502
AGGTTGTTCC TGCCTCTGAA CTTGCTGCCG CCATGAAGCA GATTAAAGAA 50
CTCCAGCGCC TGCTCGGCAA GAAAACGATG GAAAATGAAC TCCTCAAAGA 100
<210> 4503
<211> 100
<212> DNA
<213> Escherichia coli K-12 MG1655
<220> b1588 b1588
<222> 2328-2427
<223> b1588 b1588 U00096 1658577..1661003
<313> 4503: 2405-2424
<400> 4503
TCACGGCGGC AGTATCAATA TTCTGACCTC TCACCGCCCT TCACCGCTGG 50
CAAAGGGAAA CCCGTCGCAC AGCAATCTTG TCCAGATCGA AAAGGTTTAA 100
<210> 4504
<211> 100
<212> DNA
<213> Escherichia coli K-12 MG1655
<220> b1588 b1588
<222> 2323-2422
<223> b1588 b1588 U00096 1658577..1661003
<313> 4504: 2349-2368
<400> 4504
GTCGATCACG GCGGCAGTAT CAATATTCTG ACCTCTCACC GCCCTTCACC 50
GCTGGCAAAG GGAAACCCGT CGCACAGCAA TCTTGTCCAG ATCGAAAAGG 100
<210> 4505
<211> 100
<212> DNA
<213> Escherichia coli K-12 MG1655
<220> b1588 b1588
<222> 2241-2340
<223> b1588 b1588 U00096 1658577..1661003
<313> 4505: 2272-2291
<400> 4505
AAAAGTGACT CCGCGTATTC TGCCTGGCGT TACCGCCATC GGGCAAGGTG 50

CGTGGCTTAA AGCGGATATG TTTGGTGACC GGGTCGATCA CGGCGGCAGT 100
<210> 4506
<211> 100
<212> DNA
<213> Escherichia coli K-12 MG1655
<220> yi21_5 b3045
<222> 263-362
<223> yi21_5 b3045 U00096 3184203..3184568
<313> 4506: 310-329
<400> 4506
GCAAGAAAAC GATGGAAAAT GAACTCCTCA AAGAAGCCGT TGAATATGGA 50
CGGGCAAAAA AGTGGATAGC GCACGCGCCC TTATTGCCCG GGGATGGGGA 100
<210> 4507
<211> 100
<212> DNA
<213> Escherichia coli K-12 MG1655
<220> yi21_5 b3045
<222> 232-331
<223> yi21_5 b3045 U00096 3184203..3184568
<313> 4507: 265-284
<400> 4507
AAGCAGATTA AAGAACTCCA GCGCCTGCTC GGCAAGAAAA CGATGGAAAA 50
TGAACTCCTC AAAGAAGCCG TTGAATATGG ACGGGCAAAA AAGTGGATAG 100
<210> 4508
<211> 100
<212> DNA
<213> Escherichia coli K-12 MG1655
<220> yi21_5 b3045
<222> 176-275
<223> yi21_5 b3045 U00096 3184203..3184568
<313> 4508: 229-248
<400> 4508
CTGCTGTCGC CGCCGGAGAA CAGGTTGTTC CTGCCTCTGA ACTTGCTGCC 50
GCCATGAAGC AGATTAAAGA ACTCCAGCGC CTGCTCGGCA AGAAAACGAT 100
<210> 4509
<211> 100
<212> DNA
<213> Escherichia coli K-12 MG1655
<220> ybjt b0870
<222> 1362-1461
<223> ybjt b0870 U00096 complement(906075..907535)
<313> 4509: 1424-1443
<400> 4509
GGGGCTGTTT TACTGGTTAT TGATGATCCC CGCGCATCTG TTTATTTTTC 50
GCGGAATGGC AAAACAAATC GCCAGACTGG CAGAACAAAG CACAGATTAA 100
<210> 4510
<211> 100
<212> DNA
<213> Escherichia coli K-12 MG1655
<220> ybjt b0870
<222> 1245-1344
<223> ybjt b0870 U00096 complement(906075..907535)
<313> 4510: 1292-1311
<400> 4510
TACGTTGTTA TTTGGCATGA AAGCGCCGGG GCTGGGACGA CTGTGTTTTA 50
GCCTGGAAGA TAAAGGCGAC TATCGTACTA TCGATGTCCG CGCTTTCTGG 100
<210> 4511
<211> 100
<212> DNA
<213> Escherichia coli K-12 MG1655
<220> ybjt b0870
<222> 1212-1311
<223> ybjt b0870 U00096 complement(906075..907535)
<313> 4511: 1244-1263
<400> 4511
GAAAGTGATT GTCGTTGAAC CGGAAAAACA ACTTACGTTG TTATTTGGCA 50
TGAAAGCGCC GGGGCTGGGA CGACTGTGTT TTAGCCTGGA AGATAAAGGC 100
<210> 4512
<211> 100
<212> DNA
<213> Escherichia coli K-12 MG1655

<220> b1589 b1589
<222> 519-618
<223> b1589 b1589 U00096 1661014..1661631
<313> 4512: 576-595
<400> 4512
CGCGCATTTC ACAAAACCCA ATATCGTTAT CAAACCTAAC GCCAACAGCC 50
GCCCGACCGG CGATACCACT GGTTATCTGG CTAATCCGGA GGAAGTGTAA 100
<210> 4513
<211> 100
<212> DNA
<213> Escherichia coli K-12 MG1655
<220> b1589 b1589
<222> 426-525
<223> b1589 b1589 U00096 1661014..1661631
<313> 4513: 481-500
<400> 4513
CGAATCCTGC CCGCTGCGTG CACTTGAGTT CGGTCCAATT GAAGAGCTGC 50
GCCAGAAGCA CGGCACTCTT GCTGCCGTCG CGCCACTGCC GCGCGCGCAT 100
<210> 4514
<211> 100
<212> DNA
<213> Escherichia coli K-12 MG1655
<220> b1589 b1589
<222> 327-426
<223> b1589 b1589 U00096 1661014..1661631
<313> 4514: 370-389
<400> 4514
TCCGTACGGC GCGCCACAGT ACAATGCTGA AAAAGGGCAC ATGACGAAGT 50
GCGATGGTTG TTATTCGCGC GTCGCCGAGG GGAAACAACC CATATGTGTC 100
<210> 4515
<211> 100
<212> DNA
<213> Escherichia coli K-12 MG1655
<220> yi21_6 b4273
<222> 312-411
<223> yi21_6 b4273 U00096 4495795..4496205
<313> 4515: 369-388
<400> 4515
GAAAACGATG GAAAATGAAC TCCTCAAAGA AGCCGTTGAA TATGGACGGG 50
CAAAAAAGTG GATAGCGCAC GCGCCCTTAT TGCCCGGGGA TGGGGAGTAA 100
<210> 4516
<211> 100
<212> DNA
<213> Escherichia coli K-12 MG1655
<220> yi21_6 b4273
<222> 271-370
<223> yi21_6 b4273 U00096 4495795..4496205
<313> 4516: 318-337
<400> 4516
GCCATGAAGC AGATTAAAGA ACTCCAGCGC CTGCTCGGCA AGAAAACGAT 50
GGAAAATGAA CTCCTCAAAG AAGCCGTTGA ATATGGACGG GCAAAAAAGT 100
<210> 4517
<211> 100
<212> DNA
<213> Escherichia coli K-12 MG1655
<220> yi21_6 b4273
<222> 255-354
<223> yi21_6 b4273 U00096 4495795..4496205
<313> 4517: 275-294
<400> 4517
CTCTGAACTT GCTGCCGCCA TGAAGCAGAT TAAAGAACTC CAGCGCCTGC 50
TCGGCAAGAA AACGATGGAA AATGAACTCC TCAAAGAAGC CGTTGAATAT 100
<210> 4518
<211> 100
<212> DNA
<213> Escherichia coli K-12 MG1655
<220> ybju b0871
<222> 878-977
<223> ybju b0871 U00096 complement(907516..908517)
<313> 4518: 923-942
<400> 4518

ACATGAAAGC GAGAAACGTG CTGATTAACG CCTCGCCGAT TGTCCGCCTG 50
GTGACGCATC TTGACGTCTC GCGCGAACAA CTGGCGGAAG TCGCCGCCCA 100
<210> 4519
<211> 100
<212> DNA
<213> Escherichia coli K-12 MG1655
<220> ybju b0871
<222> 814-913
<223> ybju b0871 U00096 complement(907516..908517)
<313> 4519: 873-892
<400> 4519
CGTCAGGACA CCAATATGCT GTTTGTTCGC GTCGGGGAAG AAAATGCTGC 50
CGCGTTAGGC GAATACATGA AAGCGAGAAA CGTGCTGATT AACGCCTCGC 100
<210> 4520
<211> 100
<212> DNA
<213> Escherichia coli K-12 MG1655
<220> ybju b0871
<222> 800-899
<223> ybju b0871 U00096 complement(907516..908517)
<313> 4520: 827-846
<400> 4520
GCGCGGATGT GATGCGTCAG GACACCAATA TGCTGTTTGT TCGCGTCGGG 50
GAAGAAAATG CTGCCGCGTT AGGCGAATAC ATGAAAGCGA GAAACGTGCT 100
<210> 4521
<211> 100
<212> DNA
<213> Escherichia coli K-12 MG1655
<220> ybjw b0874
<222> 1560-1659
<223> ybjw b0874 U00096 complement(911385..913043)
<313> 4521: 1633-1652
<400> 4521
TTTCCTGACA CCAGACCTGC TGGCGGTGCT GAACGAGAAA TTCGGCCTGC 50
GTTCTATCAC CACTGTTGAA GAAGACATGA AGCAACTGTT GAGCGCGTAA 100
<210> 4522
<211> 100
<212> DNA
<213> Escherichia coli K-12 MG1655
<220> ybjw b0874
<222> 1493-1592
<223> ybjw b0874 U00096 complement(911385..913043)
<313> 4522: 1559-1578
<400> 4522
TCGTCATTCT GCTGACGCTG CTTTCTCTGG GCGTGAAAAA TATCGTCACC 50
GGCCCGACTG CACCTGGTTT CCTGACACCA GACCTGCTGG CGGTGCTGAA 100
<210> 4523
<211> 100
<212> DNA
<213> Escherichia coli K-12 MG1655
<220> ybjw b0874
<222> 1484-1583
<223> ybjw b0874 U00096 complement(911385..913043)
<313> 4523: 1503-1522
<400> 4523
AGAAAGCGAT CGTCATTCTG CTGACGCTGC TTTCTCTGGG CGTGAAAAAT 50
ATCGTCACCG GCCCGACTGC ACCTGGTTTC CTGACACCAG ACCTGCTGGC 100
<210> 4524
<211> 100
<212> DNA
<213> Escherichia coli K-12 MG1655
<220> ybjx ybjX
<222> 894-993
<223> ybjx ybjX U00096 complement(917351..918343)
<313> 4524: 946-965
<400> 4524
GATTGCCGAA ATCGCCAGTA AAAAACGGGC TGAATACCGT CGGCGCTATG 50
AGATGCTCGA CGCTATTCAG CCACAAATGG CCACGATGTT TCGCGGTTAA 100
<210> 4525
<211> 100
<212> DNA

<213> Escherichia coli K-12 MG1655
<220> ybjx ybjX
<222> 855-954
<223> ybjx ybjX U00096 complement(917351..918343)
<313> 4525: 896-915
<400> 4525
ACGCCATTAC CGCCTTCCAG CACAGATAGC ACGAAAAGAG ATTGCCGAAA 50
TCGCCAGTAA AAAACGGGCT GAATACCGTC GGCGCTATGA GATGCTCGAC 100
<210> 4526
<211> 100
<212> DNA
<213> Escherichia coli K-12 MG1655
<220> ybjx ybjX
<222> 840-939
<223> ybjx ybjX U00096 complement(917351..918343)
<313> 4526: 866-885
<400> 4526
CGTATGTGAT GCTGAACGCC ATTACCGCCT TCCAGCACAG ATAGCACGAA 50
AAGAGATTGC CGAAATCGCC AGTAAAAAAC GGGCTGAATA CCGTCGGCGC 100
<210> 4527
<211> 100
<212> DNA
<213> Escherichia coli K-12 MG1655
<220> ybjz b0880
<222> 1830-1929
<223> ybjz b0880 U00096 919570..921516
<313> 4527: 1852-1871
<400> 4527
ACCGTTGGCG CTGCTGCTGG CGTTTCTCTG CTCGACGGTC ACCGGGATTT 50
TATTTGGCTG GTTACCCGCA CGAAATGCGG CACGACTGGA TCCAGTAGAT 100
<210> 4528
<211> 100
<212> DNA
<213> Escherichia coli K-12 MG1655
<220> ybjz b0880
<222> 1753-1852
<223> ybjz b0880 U00096 919570..921516
<313> 4528: 1778-1797
<400> 4528
GCGTTGGGAA TAACACTGTC ACTGTTAATT GCTTTCACCT TGCAGCTTTT 50
CTTACCCGGC TGGGAGATTG GTTTTTCACC GTTGGCGCTG CTGCTGGCGT 100
<210> 4529
<211> 100
<212> DNA
<213> Escherichia coli K-12 MG1655
<220> ybjz b0880
<222> 1669-1768
<223> ybjz b0880 U00096 919570..921516
<313> 4529: 1707-1726
<400> 4529
ATTCGCATGG CTGTAGGTGC GCGAGCAAGC GATGTTTTGC AACAGTTCCT 50
GATCGAAGCC GTACTGGTTT GCCTGGTCGG TGGCGCGTTG GGAATAACAC 100
<210> 4530
<211> 100
<212> DNA
<213> Escherichia coli K-12 MG1655
<220> b1590 b1590
<222> 744-843
<223> b1590 b1590 U00096 1661633..1662487
<313> 4530: 768-787
<400> 4530
TCACGTTGCC GGACTTATTC TGGGGCTAAT CCTCATACTT GGCGGTGAGA 50
TGATCGGGCG TGTGCTCTTT TATGGCCTGC ACATGACAGT AGGAATGGCT 100
<210> 4531
<211> 100
<212> DNA
<213> Escherichia coli K-12 MG1655
<220> b1590 b1590
<222> 678-777
<223> b1590 b1590 U00096 1661633..1662487
<313> 4531: 736-755

<400> 4531
GCGTGTGGTA TTGTTATGCG CAGGTCTTGG ATGCTGGCTT TGCCCGCTCA 50
TTCGTCGCCG TGAACCTCAC GTTGCCGGAC TTATTCTGGG GCTAATCCTC 100
<210> 4532
<211> 100
<212> DNA
<213> Escherichia coli K-12 MG1655
<220> b1590 b1590
<222> 598-697
<223> b1590 b1590 U00096 1661633..1662487
<313> 4532: 613-632
<400> 4532
GGTTTGTCTC TGGCGTCTAT TCACTCATCC GTGCAACAAG CCAGCGCACT 50
GGTACCAGAT TACGCCTCCT TACAGGTCTG GCGTGTGGTA TTGTTATGCG 100
<210> 4533
<211> 100
<212> DNA
<213> Escherichia coli K-12 MG1655
<220> b1591 b1591
<222> 429-528
<223> b1591 b1591 U00096 1662521..1663144
<313> 4533: 476-495
<400> 4533
GAATGGTCGC CAGACAGAAT GCGAAGAATT GCTGGCATGG CACCTTTTTC 50
CGTGGTCAAC ACGTTTTCTT GATGTTTTTA TCGAAAAAGC AGAACACCCT 100
<210> 4534
<211> 100
<212> DNA
<213> Escherichia coli K-12 MG1655
<220> b1591 b1591
<222> 392-491
<223> b1591 b1591 U00096 1662521..1663144
<313> 4534: 426-445
<400> 4534
TTGGTTCACT GCTGTTGATG GCTGCGTGGT TGGCGGAGAA TGGTCGCCAG 50
ACAGAATGCG AAGAATTGCT GGCATGGCAC CTTTTTCCGT GGTCAACACG 100
<210> 4535
<211> 100
<212> DNA
<213> Escherichia coli K-12 MG1655
<220> b1591 b1591
<222> 301-400
<223> b1591 b1591 U00096 1662521..1663144
<313> 4535: 356-375
<400> 4535
CTGTTTGGCG ATTCAACATT GGCACTTCGT CAGTGGATGC GCGAGAAAGG 50
CATTCAGTTT GAAATGAAGC AAAACGAACC GGAAGATCAT TTTGGTTCAC 100
<210> 4536
<211> 100
<212> DNA
<213> Escherichia coli K-12 MG1655
<220> b1592 b1592
<222> 1218-1317
<223> b1592 b1592 U00096 1663279..1664595
<313> 4536: 1278-1297
<400> 4536
ACTCCCCGGT TTATTGATTG CCTGCGTAAT TGCGTCGGTA ATTTCGCGGA 50
CGTTACACCG TGACTCTATC TACCGCCAGC ACACTGCGCA GCATAGCTAA 100
<210> 4537
<211> 100
<212> DNA
<213> Escherichia coli K-12 MG1655
<220> b1592 b1592
<222> 1171-1270
<223> b1592 b1592 U00096 1663279..1664595
<313> 4537: 1216-1235
<400> 4537
ATTATGTCGA CGTTGATGAT ATGTGAAATG ACCGGGGAGT ATCAGCTACT 50
CCCCGGTTTA TTGATTGCCT GCGTAATTGC GTCGGTAATT TCGCGGACGT 100
<210> 4538
<211> 100

<212> DNA
<213> Escherichia coli K-12 MG1655
<220> b1592 b1592
<222> 1093-1192
<223> b1592 b1592 U00096 1663279..1664595
<313> 4538: 1128-1147
<400> 4538
CCTGATGGCG AAGAAATTAC ACTTTTACTC GGATTGACCG GGATGGCGAC 50
ACTGTTGGCG GCGACCACGC ACGCGCCGAT TATGTCGACG TTGATGATAT 100
<210> 4539
<211> 100
<212> DNA
<213> Escherichia coli K-12 MG1655
<220> b1593 b1594
<222> 596-695
<223> b1593 b1594 U00096 complement(1664548..1665255)
<313> 4539: 649-668
<400> 4539
CGCTCATTGG TGAACTGCCT TATCTGCCGC GCGCCGAACA GCGTGAACTG 50
GGGCAATACA TCCGCTTAGC TATGCTGCGC AGTGTGCTGG CGGTAGATAG 100
<210> 4540
<211> 100
<212> DNA
<213> Escherichia coli K-12 MG1655
<220> b1593 b1594
<222> 504-603
<223> b1593 b1594 U00096 complement(1664548..1665255)
<313> 4540: 534-553
<400> 4540
GCCGCTCATT GGCTGGGTGG CTAACCGAAT CAACCCAGGA CTGGCGCATT 50
ATGCGGAAAT CATTGATGTG CTGGGTAAAA AACTTCCGGC ACCGCTCATT 100
<210> 4541
<211> 100
<212> DNA
<213> Escherichia coli K-12 MG1655
<220> b1593 b1594
<222> 432-531
<223> b1593 b1594 U00096 complement(1664548..1665255)
<313> 4541: 463-482
<400> 4541
GGTTGTCGGT ATTCAGGAAG GTTGCATTAA CCATGCACTG CTAACAGCTC 50
AGGCGATCGC CAACGACGGG CTGCCGCTCA TTGGCTGGGT GGCTAACCGA 100
<210> 4542
<211> 100
<212> DNA
<213> Escherichia coli K-12 MG1655
<220> b0866 b0866
<222> 159-258
<223> b0866 b0866 U00096 903816..904139
<313> 4542: 190-209
<400> 4542
ACTGCGTAAA GCACGGGAGA TCGCCTTTGA GGAATTAGGC TCCCAGGCGC 50
GGGCGCTGGG GGCCGATGCC GTCGTCGGTA TTGATATCGA CTACGAAACG 100
<210> 4543
<211> 100
<212> DNA
<213> Escherichia coli K-12 MG1655
<220> b0866 b0866
<222> 108-207
<223> b0866 b0866 U00096 903816..904139
<313> 4543: 153-172
<400> 4543
TGCCGGTATC CGCGATATCG TTGGCGGACG TTCCGGTGCG TATGAAAAAG 50
AACTGCGTAA AGCACGGGAG ATCGCCTTTG AGGAATTAGG CTCCCAGGCG 100
<210> 4544
<211> 100
<212> DNA
<213> Escherichia coli K-12 MG1655
<220> b0866 b0866
<222> 22-121
<223> b0866 b0866 U00096 903816..904139

<313> 4544: 69-88
<400> 4544
ACTCTGGAAG GCCAGACCAT CGTTGAATAT TGCGGTGTGG TGACCGGCGA 50
AGCGATTTTA GGTGCCAATA TTTTCCGTGA TTTCTTTGCC GGTATCCGCG 100
<210> 4545
<211> 100
<212> DNA
<213> Escherichia coli K-12 MG1655
<220> b0867 b0867
<222> 732-831
<223> b0867 b0867 U00096 904136..904966
<313> 4545: 799-818
<400> 4545
ATTCCAGATG CATTTCCGCC CGACGTTATA TAACGGCGAA GCGGATGCAG 50
AAACTCAGGC GATTGCCGAA GCATTGCTGG AGAAATACGG GCAGGATTAG 100
<210> 4546
<211> 100
<212> DNA
<213> Escherichia coli K-12 MG1655
<220> b0867 b0867
<222> 476-575
<223> b0867 b0867 U00096 904136..904966
<313> 4546: 510-529
<400> 4546
AGCCGGAAAA CGTAGTGGCA CATGCGGATA TCGCACCGCA GCGCAAAGAC 50
GATCCGGGGC CATTATTTCC CTGGCAGCAA CTGGCGCAGC AGGGGATTGG 100
<210> 4547
<211> 100
<212> DNA
<213> Escherichia coli K-12 MG1655
<220> b0867 b0867
<222> 437-536
<223> b0867 b0867 U00096 904136..904966
<313> 4547: 468-487
<400> 4547
TTCCACTGGC GAAAGATATT ATTGCCCGTT ATCACATCAA GCCGGAAAAC 50
GTAGTGGCAC ATGCGGATAT CGCACCGCAG CGCAAAGACG ATCCGGGGCC 100
<210> 4548
<211> 100
<212> DNA
<213> Escherichia coli K-12 MG1655
<220> b0868 b0869
<222> 951-1050
<223> b0868 b0869 U00096 complement(904963..906012)
<313> 4548: 1022-1041
<400> 4548
TGCGCAGGAA GAGTTAGGTT ATCAGCCGGT CATCACCCTG GATGAAGGTA 50
TCGAGAAAAC TGCCGCCTGG CTACGCGACC ACGGAAAACT GCCGCGCTAA 100
<210> 4549
<211> 100
<212> DNA
<213> Escherichia coli K-12 MG1655
<220> b0868 b0869
<222> 951-1050
<223> b0868 b0869 U00096 complement(904963..906012)
<313> 4549: 975-994
<400> 4549
TGCGCAGGAA GAGTTAGGTT ATCAGCCGGT CATCACCCTG GATGAAGGTA 50
TCGAGAAAAC TGCCGCCTGG CTACGCGACC ACGGAAAACT GCCGCGCTAA 100
<210> 4550
<211> 100
<212> DNA
<213> Escherichia coli K-12 MG1655
<220> b0868 b0869
<222> 840-939
<223> b0868 b0869 U00096 complement(904963..906012)
<313> 4550: 862-881
<400> 4550
TATGATCGCC CGCAGCATGG AGCGTTTAGG CCGCAAGTCA GCAAAAGAGC 50
CGCCGCTGAC CCACTACGGC GTCTCCAAGT TGAATTTTGA CTTTACGCTG 100
<210> 4551

<211> 100
<212> DNA
<213> Escherichia coli K-12 MG1655
<220> b1598 b1598
<222> 664-763
<223> b1598 b1598 U00096 1669984..1670805
<313> 4551: 715-734
<400> 4551
GACAGCGGTT CGCCTCTGAT GTTGCATACC GATGACGGCT GGCAATTAAT 50
TGGGGTGCAA AGTTCGGCTC CTGCCGCGAA AGATCGCTGG CGCGCCGATA 100
<210> 4552
<211> 100
<212> DNA
<213> Escherichia coli K-12 MG1655
<220> b1598 b1598
<222> 635-734
<223> b1598 b1598 U00096 1669984..1670805
<313> 4552: 673-692
<400> 4552
TGTCACATCA GTGCGATACC TTGCCGGGTG ACAGCGGTTC GCCTCTGATG 50
TTGCATACCG ATGACGGCTG GCAATTAATT GGGGTGCAAA GTTCGGCTCC 100
<210> 4553
<211> 100
<212> DNA
<213> Escherichia coli K-12 MG1655
<220> b1598 b1598
<222> 509-608
<223> b1598 b1598 U00096 1669984..1670805
<313> 4553: 553-572
<400> 4553
CGCTTACCGC CGCATTAAAA GCGGCAGGTC GTAAAGTGAC TCAGGCAGGC 50
TACCCTGAAG ATCATCTCGA TACGTTGTAC AGTCATCAAA ACTGTGAAGT 100
<210> 4554
<211> 100
<212> DNA
<213> Escherichia coli K-12 MG1655
<220> b1599 b1599
<222> 196-295
<223> b1599 b1599 U00096 complement(1670844..1671173)
<313> 4554: 227-246
<400> 4554
GCATTGTGGG GCGGGTTTGG TATTGCCGCC ACGTTAGCCG CAGGTTGGAT 50
CTTGTTTGGT CAACGGTTAA ATCGTAAAGG CTGGATTGGC CTGGTCTTGC 100
<210> 4555
<211> 100
<212> DNA
<213> Escherichia coli K-12 MG1655
<220> b1599 b1599
<222> 124-223
<223> b1599 b1599 U00096 complement(1670844..1671173)
<313> 4555: 186-205
<400> 4555
CTGGCGGCGG TGCTGGCTGC CTTTAGTGCG CTTTCTCAAG CCGTTAAAGG 50
GATCGACTTG TCTGTCGCTT ATGCATTGTG GGGCGGGTTT GGTATTGCCG 100
<210> 4556
<211> 100
<212> DNA
<213> Escherichia coli K-12 MG1655
<220> b1599 b1599
<222> 26-125
<223> b1599 b1599 U00096 complement(1670844..1671173)
<313> 4556: 68-87
<400> 4556
CCGCCTGGCT GGCATTGGCA ATCGTGCTGG AAATCGTTGC TAACGTCTTT 50
TTGAAATTTT CTGACGGCTT TCGTCGCAAA ATATTTGGCT TGCTCTCCCT 100
<210> 4557
<211> 100
<212> DNA
<213> Escherichia coli K-12 MG1655
<220> basr b4114
<222> 544-643

<223> basr b4114 U00096 complement(4330860..4331528)
<313> 4557: 607-626
<400> 4557
GACAATGAAC CCTCGACCAA CACCCTGGAA GTGCATATCC ACAATCTGCG 50
CGACAAAGTG GGCAAAGCCC GTATCCGCAC CGTGCGCGGC TTTGGCTATA 100
<210> 4558
<211> 100
<212> DNA
<213> Escherichia coli K-12 MG1655
<220> basr b4114
<222> 531-630
<223> basr b4114 U00096 complement(4330860..4331528)
<313> 4558: 566-585
<400> 4558
CATCTATAAC TGGGACAATG AACCCTCGAC CAACACCCTG GAAGTGCATA 50
TCCACAATCT GCGCGACAAA GTGGGCAAAG CCCGTATCCG CACCGTGCGC 100
<210> 4559
<211> 100
<212> DNA
<213> Escherichia coli K-12 MG1655
<220> basr b4114
<222> 442-541
<223> basr b4114 U00096 complement(4330860..4331528)
<313> 4559: 476-495
<400> 4559
TTGATTCTGA CGCCCAAAGA ATATGCTCTG CTGTCACGGT TAATGCTCAA 50
AGCAGGCAGT CCGGTGCATC GGGAAATTCT CTACAACGAC ATCTATAACT 100
<210> 4560
<211> 100
<212> DNA
<213> Escherichia coli K-12 MG1655
<220> hnr b1236
<222> 897-996
<223> hnr b1236 U00096 1289465..1290478
<313> 4560: 945-964
<400> 4560
TCCGTTAGGC ACTTTAGGTA ACGCTTATTT GAATCAATTG AGCCAGCGAT 50
GCGATGCCTG GCAATGCCAA ATATGGGGAA CCGGTGGTCG ACTGCGCTTG 100
<210> 4561
<211> 100
<212> DNA
<213> Escherichia coli K-12 MG1655
<220> hnr b1236
<222> 830-929
<223> hnr b1236 U00096 1289465..1290478
<313> 4561: 892-911
<400> 4561
TGGTTTCTGC GGGTCTGAAT GCGACGTTAA ATACCGGCGA ACACCAGGTG 50
CAAATCAGTA ATGGTGTTCC GTTAGGCACT TTAGGTAACG CTTATTTGAA 100
<210> 4562
<211> 100
<212> DNA
<213> Escherichia coli K-12 MG1655
<220> hnr b1236
<222> 714-813
<223> hnr b1236 U00096 1289465..1290478
<313> 4562: 776-795
<400> 4562
GCCAGAGTTG GGCGCGTTAT TGAAGCAGGT AAACCATTTA CTTCGTCAGG 50
CCAATCTGCC GGGGCAGTTT CCGCTATTAG TTGGCTATTA TCATCGCGAA 100
<210> 4563
<211> 100
<212> DNA
<213> Escherichia coli K-12 MG1655
<220> bass b4113
<222> 993-1092
<223> bass b4113 U00096 complement(4329759..4330850)
<313> 4563: 1055-1074
<400> 4563
GCATCACGGG CAGTTTTTCC TGCAAAACCG GCAAGAGACT TCCGGCACGC 50
GGGCCTGGGT ACGGCTGAAG AAAGATCAGT ACGTGGCAAA CCAGATATAA 100

<210> 4564
<211> 100
<212> DNA
<213> Escherichia coli K-12 MG1655
<220> bass b4113
<222> 978-1077
<223> bass b4113 U00096 complement(4329759..4330850)
<313> 4564: 1026-1045
<400> 4564
CCGCATTACA CAGTTGCATC ACGGGCAGTT TTTCCTGCAA AACCGGCAAG 50
AGACTTCCGG CACGCGGGCC TGGGTACGGC TGAAGAAAGA TCAGTACGTG 100
<210> 4565
<211> 100
<212> DNA
<213> Escherichia coli K-12 MG1655
<220> bass b4113
<222> 941-1040
<223> bass b4113 U00096 complement(4329759..4330850)
<313> 4565: 973-992
<400> 4565
GTTATGGCGG GATTGGTCTG GGGTTAAGTA TTGTCAGCCG CATTACACAG 50
TTGCATCACG GGCAGTTTTT CCTGCAAAAC CGGCAAGAGA CTTCCGGCAC 100
<210> 4566
<211> 100
<212> DNA
<213> Escherichia coli K-12 MG1655
<220> hns b1238
<222> 315-414
<223> hns b1238 U00096 complement(1291732..1292145)
<313> 4566: 389-408
<400> 4566
AACTAAAACC TGGACTGGCC AAGGCCGTAC TCCAGCTGTA ATCAAAAAAG 50
CAATGGATGA GCAAGGTAAA TCCCTCGACG ATTTCCTGAT CAAGCAATAA 100
<210> 4567
<211> 100
<212> DNA
<213> Escherichia coli K-12 MG1655
<220> hns b1238
<222> 239-338
<223> hns b1238 U00096 complement(1291732..1292145)
<313> 4567: 268-287
<400> 4567
CTGCCGTTAA ATCTGGCACC AAAGCTAAAC GTGCTCAGCG TCCGGCAAAA 50
TATAGCTACG TTGACGAAAA CGGCGAAACT AAAACCTGGA CTGGCCAAGG 100
<210> 4568
<211> 100
<212> DNA
<213> Escherichia coli K-12 MG1655
<220> hns b1238
<222> 190-289
<223> hns b1238 U00096 complement(1291732..1292145)
<313> 4568: 238-257
<400> 4568
ATGCTGATCG CTGACGGTAT TGACCCGAAC GAACTGCTGA ATAGCCTTGC 50
TGCCGTTAAA TCTGGCACCA AAGCTAAACG TGCTCAGCGT CCGGCAAAAT 100
<210> 4569
<211> 100
<212> DNA
<213> Escherichia coli K-12 MG1655
<220> b0872 b0873
<222> 870-969
<223> b0872 b0873 U00096 complement(910405..911373)
<313> 4569: 915-934
<400> 4569
TACGGTGAGC AGCACAATGA CGCTGACCGA CGCCGAAATC GCTGAAGGTT 50
ACGTACTGGC CTGCTCCTGC CATCCGCAGG GGGATTTGGT TCTCGCATAA 100
<210> 4570
<211> 100
<212> DNA
<213> Escherichia coli K-12 MG1655
<220> b0872 b0873

<222> 853-952
<223> b0872 b0873 U00096 complement(910405..911373)
<313> 4570: 875-894
<400> 4570
GTGGTTTCCG GTGAATATAC GGTGAGCAGC ACAATGACGC TGACCGACGC 50
CGAAATCGCT GAAGGTTACG TACTGGCCTG CTCCTGCCAT CCGCAGGGGG 100
<210> 4571
<211> 100
<212> DNA
<213> Escherichia coli K-12 MG1655
<220> b0872 b0873
<222> 754-853
<223> b0872 b0873 U00096 complement(910405..911373)
<313> 4571: 802-821
<400> 4571
GCCCCGGTTG GCACCACGCT ACTGGAGGCG CTGGAAAGCA ATAACGTTCC 50
GGTTGTCGCT GCCTGCCGTG CGGGTGTTTG CGGCTGCTGT AAGACAAAAG 100
<210> 4572
<211> 100
<212> DNA
<213> Escherichia coli K-12 MG1655
<220> b0878 b0879
<222> 1044-1143
<223> b0878 b0879 U00096 918431..919573
<313> 4572: 1110-1129
<400> 4572
GATTGGCGCA CGTAACGATA CCGATGTTGA GATTGTCAAA GGGCTTGAAG 50
CGGGCGATGA AGTGGTGATT GGTGAGGCCA AACCAGGAGC TGCACAATGA 100
<210> 4573
<211> 100
<212> DNA
<213> Escherichia coli K-12 MG1655
<220> b0878 b0879
<222> 1026-1125
<223> b0878 b0879 U00096 918431..919573
<313> 4573: 1071-1090
<400> 4573
ACGCGAGCGT GAAGTGACGA TTGGCGCACG TAACGATACC GATGTTGAGA 50
TTGTCAAAGG GCTTGAAGCG GGCGATGAAG TGGTGATTGG TGAGGCCAAA 100
<210> 4574
<211> 100
<212> DNA
<213> Escherichia coli K-12 MG1655
<220> b0878 b0879
<222> 992-1091
<223> b0878 b0879 U00096 918431..919573
<313> 4574: 1032-1051
<400> 4574
GTTATAAAGT CAAATTGTTG CGTAATGGTG AAACACGCGA GCGTGAAGTG 50
ACGATTGGCG CACGTAACGA TACCGATGTT GAGATTGTCA AAGGGCTTGA 100
<210> 4575
<211> 100
<212> DNA
<213> Escherichia coli K-12 MG1655
<220> alas b2698
<222> 2532-2631
<223> alas b2698 U00096 complement(2817403..2820033)
<313> 4575: 2604-2623
<400> 4575
TGGACGTCCT GACATGGCGC AAGCCGGTGG TACGGATGCT GCGGCCTTAC 50
CTGCAGCGTT AGCCAGTGTG AAAGGCTGGG TCAGCGCGAA ATTGCAATAA 100
<210> 4576
<211> 100
<212> DNA
<213> Escherichia coli K-12 MG1655
<220> alas b2698
<222> 2381-2480
<223> alas b2698 U00096 complement(2817403..2820033)
<313> 4576: 2435-2454
<400> 4576
AAAATCAGCT GGGGTCGACA ATTATCGTGC TGGCAACGGT AGTCGAAGGT 50

AAGGTTTCTC TGATTGCAGG CGTATCTAAG GACGTCACAG ATCGTGTGAA 100
<210> 4577
<211> 100
<212> DNA
<213> Escherichia coli K-12 MG1655
<220> alas b2698
<222> 2239-2338
<223> alas b2698 U00096 complement(2817403..2820033)
<313> 4577: 2268-2287
<400> 4577
CAACAGCTTA AAGAACAAGC TGCCGCACAG GAGAGCGCAA ATCTTTCCAG 50
TAAGGCAATT GATGTTAATG GTGTTAAGCT GTTGGTTAGC GAGCTTAGCG 100
<210> 4578
<211> 100
<212> DNA
<213> Escherichia coli K-12 MG1655
<220> feab b1386
<222> 1373-1472
<223> feab b1386 U00096 1445540..1447042
<313> 4578: 1425-1444
<400> 4578
TAAACAGCCA TACCTTAATT GACGCTAACT TACCGTTTGG TGGGATGAAG 50
CAGTCAGGAA CGGGCCGTGA TTTTGGCCCC GACTGGCTGG ACGGTTGGTG 100
<210> 4579
<211> 100
<212> DNA
<213> Escherichia coli K-12 MG1655
<220> feab b1386
<222> 1272-1371
<223> feab b1386 U00096 1445540..1447042
<313> 4579: 1318-1337
<400> 4579
GGCAAACGAC ACGGAATATG GCTTAACTGC CAGTGTCTGG ACGCAAAATC 50
TCTCCCAGGC TCTGGAATAT AGCGATCGCT TACAGGCAGG GACGGTGTGG 100
<210> 4580
<211> 100
<212> DNA
<213> Escherichia coli K-12 MG1655
<220> feab b1386
<222> 1231-1330
<223> feab b1386 U00096 1445540..1447042
<313> 4580: 1263-1282
<400> 4580
AACCTGGTGC GAGTAGCGGA TGGAGAAGAG GCGTTACAAC TGGCAAACGA 50
CACGGAATAT GGCTTAACTG CCAGTGTCTG GACGCAAAAT CTCTCCCAGG 100
<210> 4581
<211> 100
<212> DNA
<213> Escherichia coli K-12 MG1655
<220> pmrd b2260
<222> 198-297
<223> pmrd b2260 U00096 complement(2371292..2371588)
<313> 4581: 275-294
<400> 4581
TAATCGTGAA AAGCTGCATA CGGTGAAAGT CCTCTCGGCA AGCAGTTATA 50
GCCCCGATGA GTGGGAACGG CAGTGTAAAG TGGCAGGGAA AACTCAGTAA 100
<210> 4582
<211> 100
<212> DNA
<213> Escherichia coli K-12 MG1655
<220> pmrd b2260
<222> 198-297
<223> pmrd b2260 U00096 complement(2371292..2371588)
<313> 4582: 267-286
<400> 4582
TAATCGTGAA AAGCTGCATA CGGTGAAAGT CCTCTCGGCA AGCAGTTATA 50
GCCCCGATGA GTGGGAACGG CAGTGTAAAG TGGCAGGGAA AACTCAGTAA 100
<210> 4583
<211> 100
<212> DNA
<213> Escherichia coli K-12 MG1655

<220> pmrd b2260
<222> 159-258
<223> pmrd b2260 U00096 complement(2371292..2371588)
<313> 4583: 220-239
<400> 4583
GGATTTACTC TCGCCTTTGC AGAATGCGCT TTATTGTATT AATCGTGAAA 50
AGCTGCATAC GGTGAAAGTC CTCTCGGCAA GCAGTTATAG CCCCGATGAG 100
<210> 4584
<211> 100
<212> DNA
<213> Escherichia coli K-12 MG1655
<220> mvim b1069
<222> 825-924
<223> mvim b1069 U00096 1126029..1126952
<313> 4584: 901-920
<400> 4584
ATGTGTGCAA AACCAGACAG TTCCGCAAAC CGCCGGCGAA CAGGCCGTGC 50
TGGCGCAACG TATCGTTGAC AAGATCTGGC GCGATGCGAT GAGTGAATAA 100
<210> 4585
<211> 100
<212> DNA
<213> Escherichia coli K-12 MG1655
<220> mvim b1069
<222> 808-907
<223> mvim b1069 U00096 1126029..1126952
<313> 4585: 836-855
<400> 4585
GCGCGGCACT TCATTGAATG TGTGCAAAAC CAGACAGTTC CGCAAACCGC 50
CGGCGAACAG GCCGTGCTGG CGCAACGTAT CGTTGACAAG ATCTGGCGCG 100
<210> 4586
<211> 100
<212> DNA
<213> Escherichia coli K-12 MG1655
<220> mvim b1069
<222> 716-815
<223> mvim b1069 U00096 1126029..1126952
<313> 4586: 757-776
<400> 4586
GCGAATGGCG TGAGGAGCGC GGGCAGGGCG TAGTGCATAA ACCGATTCCT 50
GGTTGGCAGA GTACGCTTGA GCAACGTGGG TTTGTCGGCT GTGCGCGGCA 100
<210> 4587
<211> 100
<212> DNA
<213> Escherichia coli K-12 MG1655
<220> mvin b1070
<222> 1437-1536
<223> mvin b1070 U00096 1127062..1128597
<313> 4587: 1506-1525
<400> 4587
GCGTTTAATG GCGGTCGTGC TGGCGGGGAT TGCCGCGTAC TTCGCTGCAC 50
TGGCGGTACT GGGCTTCAAA GTTAAAGAAT TTGCCCGCCG GACGGTGTAA 100
<210> 4588
<211> 100
<212> DNA
<213> Escherichia coli K-12 MG1655
<220> mvin b1070
<222> 1117-1216
<223> mvin b1070 U00096 1127062..1128597
<313> 4588: 1172-1191
<400> 4588
GGCTTTTATT CCCGCCAGGA CATTAAAACG CCAGTGAAAA TTGCCATCGT 50
TACGCTGATT TTAACGCAAT TGATGAACCT GGCGTTTATT GGTCCGTTGA 100
<210> 4589
<211> 100
<212> DNA
<213> Escherichia coli K-12 MG1655
<220> mvin b1070
<222> 1085-1184
<223> mvin b1070 U00096 1127062..1128597
<313> 4589: 1129-1148
<400> 4589

TTGGCCTGAT TGTAGTGAAA GTGTTGGCTC CTGGCTTTTA TTCCCGCCAG 50
GACATTAAAA CGCCAGTGAA AATTGCCATC GTTACGCTGA TTTTAACGCA 100
<210> 4590
<211> 100
<212> DNA
<213> Escherichia coli K-12 MG1655
<220> fear b1385
<222> 807-906
<223> fear b1385 U00096 complement(1444402..1445307)
<313> 4590: 872-891
<400> 4590
CGGCTTTCAT TGGGGATTTT CTGACCAGAG TCATTTTTCA ACGGTATTTA 50
AGCAACGCTT TGGGATGACG CCAGGCGAGT ATCGACGTAA ATTCCGCTAA 100
<210> 4591
<211> 100
<212> DNA
<213> Escherichia coli K-12 MG1655
<220> fear b1385
<222> 738-837
<223> fear b1385 U00096 complement(1444402..1445307)
<313> 4591: 793-812
<400> 4591
TAACCGTCGT CTCGATTTTT GTGCAGATGC GATTCGCCAT GCCGCAGATG 50
ATGAAAAACT GGCAGGCATC GGCTTTCATT GGGGATTTTC TGACCAGAGT 100
<210> 4592
<211> 100
<212> DNA
<213> Escherichia coli K-12 MG1655
<220> fear b1385
<222> 627-726
<223> fear b1385 U00096 complement(1444402..1445307)
<313> 4592: 677-696
<400> 4592
TCGCGAAGAG ATATTACGCC CGGAGTGGAT AGCCGGAGAG ACAGGTATGT 50
CAGTACGTAG TTTGTACCGA ATGTTTGCCG ATAAAGGTTT GGTAGTCGCG 100
<210> 4593
<211> 100
<212> DNA
<213> Escherichia coli K-12 MG1655
<220> hpt b0126
<222> 450-549
<223> hpt b0126 U00096 141419..141967
<313> 4593: 523-542
<400> 4593
CTCGATCCCG GATGAGTTTG TGGTGGGTTA CGGCATTGAT TACGCACAGC 50
GTTACCGTCA TCTGCCGTAT ATCGGCAAAG TGATTCTGCT GGACGAGTAA 100
<210> 4594
<211> 100
<212> DNA
<213> Escherichia coli K-12 MG1655
<220> hpt b0126
<222> 422-521
<223> hpt b0126 U00096 141419..141967
<313> 4594: 472-491
<400> 4594
TGAACGTCCC GGTAGAATTT ATCGGTTTCT CGATCCCGGA TGAGTTTGTG 50
GTGGGTTACG GCATTGATTA CGCACAGCGT TACCGTCATC TGCCGTATAT 100
<210> 4595
<211> 100
<212> DNA
<213> Escherichia coli K-12 MG1655
<220> hpt b0126
<222> 384-483
<223> hpt b0126 U00096 141419..141967
<313> 4595: 419-438
<400> 4595
GATTTGTACG CTGCTGGATA AACCGTCCCG TCGTGAAGTG AACGTCCCGG 50
TAGAATTTAT CGGTTTCTCG ATCCCGGATG AGTTTGTGGT GGGTTACGGC 100
<210> 4596
<211> 100
<212> DNA

<213> Escherichia coli K-12 MG1655
<220> oppa b1244
<222> 1533-1632
<223> oppa b1244 U00096 1299206..1300837
<313> 4596: 1585-1604
<400> 4596
CGTGAATGCG CGTCTGGTGA AACCGTGGGT TGGTGGCTAT ACCGGCAAAG 50
ATCCGCTGGA TAATACCTAT ACCCGGAATA TGTACATTGT GAAGCACTAA 100
<210> 4597
<211> 100
<212> DNA
<213> Escherichia coli K-12 MG1655
<220> oppa b1244
<222> 1438-1537
<223> oppa b1244 U00096 1299206..1300837
<313> 4597: 1461-1480
<400> 4597
CTGAAAGTGA CTGACGAGGC GCAGCGCACA GCTCTGTACA CTAAAGCAGA 50
ACAACAGCTG GATAAGGATT CGGCCATTGT TCCTGTTTAT TACTACGTGA 100
<210> 4598
<211> 100
<212> DNA
<213> Escherichia coli K-12 MG1655
<220> oppa b1244
<222> 1378-1477
<223> oppa b1244 U00096 1299206..1300837
<313> 4598: 1429-1448
<400> 4598
AGCTCGATGA ATACCGCGCA TTATAAGAGC CCGGCCTTTG ACAGCATTAT 50
GGCGGAAACG CTGAAAGTGA CTGACGAGGC GCAGCGCACA GCTCTGTACA 100
<210> 4599
<211> 100
<212> DNA
<213> Escherichia coli K-12 MG1655
<220> oppb b1245
<222> 822-921
<223> oppb b1245 U00096 1300923..1301843
<313> 4599: 892-911
<400> 4599
AGTGTTAAGC CTGACCATCC TGGTTGGTGC TTTAACCATT TTGTTTAATG 50
CCATTGTCGA TGTGCTATAT GCGGTTATCG ACCCGAAAAT CCGTTACTGA 100
<210> 4600
<211> 100
<212> DNA
<213> Escherichia coli K-12 MG1655
<220> oppb b1245
<222> 714-813
<223> oppb b1245 U00096 1300923..1301843
<313> 4600: 769-788
<400> 4600
CGCATTTGTC GGCATTATTA CCGGTTCTAT GGTTATCGAA ACCATTTATG 50
GTTTGCCGGG GATTGGACAA CTGTTCGTTA ATGGCGCATT GAACCGTGAC 100
<210> 4601
<211> 100
<212> DNA
<213> Escherichia coli K-12 MG1655
<220> oppb b1245
<222> 640-739
<223> oppb b1245 U00096 1300923..1301843
<313> 4601: 669-688
<400> 4601
TTACCTATGC GGCGGATCAT TTTACGTCAC GCATTAAAAC CTGCTCTGTT 50
ACCCGTGCTC TCTTATATGG GCCCCGCATT TGTCGGCATT ATTACCGGTT 100
<210> 4602
<211> 100
<212> DNA
<213> Escherichia coli K-12 MG1655
<220> oppc b1246
<222> 810-909
<223> oppc b1246 U00096 1301858..1302766
<313> 4602: 890-909

<400> 4602
GTTATTGTTG TTCCCAGCGG GATTCCTCGT GGTGACGCTG TTTTGTTTCA 50
ACTTTATCGG CGATGGCTTG CGTGATGCCC TCGACCCGAA AGATCGTTAA 100
<210> 4603
<211> 100
<212> DNA
<213> Escherichia coli K-12 MG1655
<220> oppc b1246
<222> 810-909
<223> oppc b1246 U00096 1301858..1302766
<313> 4603: 842-861
<400> 4603
GTTATTGTTG TTCCCAGCGG GATTCCTCGT GGTGACGCTG TTTTGTTTCA 50
ACTTTATCGG CGATGGCTTG CGTGATGCCC TCGACCCGAA AGATCGTTAA 100
<210> 4604
<211> 100
<212> DNA
<213> Escherichia coli K-12 MG1655
<220> oppc b1246
<222> 755-854
<223> oppc b1246 U00096 1301858..1302766
<313> 4604: 786-805
<400> 4604
GCAGCTGGGG TGCATTGCTG AGTGATGGCG CGAACTCGAT GGAAGTCTCT 50
CCATGGTTAT TGTTGTTCCC AGCGGGATTC CTCGTGGTGA CGCTGTTTTG 100
<210> 4605
<211> 100
<212> DNA
<213> Escherichia coli K-12 MG1655
<220> oppd b1247
<222> 915-1014
<223> oppd b1247 U00096 1302778..1303791
<313> 4605: 964-983
<400> 4605
TCCGCATGCG ATGGAAATTT GTAGTAGCGC ACCGCCGCTG GAAGAGTTTA 50
CGCCTGGCCG TCTGCGTGCT TGCTTTAAAC CGGTGGAGGA ACTGTTATGA 100
<210> 4606
<211> 100
<212> DNA
<213> Escherichia coli K-12 MG1655
<220> oppd b1247
<222> 850-949
<223> oppd b1247 U00096 1302778..1303791
<313> 4606: 902-921
<400> 4606
ACCATCCCGG GTAATCCGCC AAACCTGCTG CGATTACCGA AAGGTTGCCC 50
GTTCCAGCCA CGTTGTCCGC ATGCGATGGA AATTTGTAGT AGCGCACCGC 100
<210> 4607
<211> 100
<212> DNA
<213> Escherichia coli K-12 MG1655
<220> oppd b1247
<222> 759-858
<223> oppd b1247 U00096 1302778..1303791
<313> 4607: 798-817
<400> 4607
GCGCGATGTC TTTTATCAAC CCGTTCATCC TTATTCTATC GGTTTGCTCA 50
ACGCGGTGCC GCGTCTCGAT GCGGAAGGTG AAACAATGTT GACCATCCCG 100
<210> 4608
<211> 100
<212> DNA
<213> Escherichia coli K-12 MG1655
<220> oppf oppF
<222> 906-1005
<223> oppf oppF U00096 1303788..1304792
<313> 4608: 967-986
<400> 4608
CCGTTGCCCG ATTGCCGGTC CAGAGTGCGC CAAAACACGT CCTGTTCTGG 50
AGGGGAGTTT CAGACACTCC GTTTCTTGCC TGAAAGTCGA TCCGCTTTAA 100
<210> 4609
<211> 100

<212> DNA
<213> Escherichia coli K-12 MG1655
<220> oppf oppF
<222> 865-964
<223> oppf oppF U00096 1303788..1304792
<313> 4609: 915-934
<400> 4609
CCGTCGCCGA TCAACCCGCC TTCCGGTTGT GTTTTCCGTA CCCGTTGCCC 50
GATTGCCGGT CCAGAGTGCG CCAAAACACG TCCTGTTCTG GAGGGGAGTT 100
<210> 4610
<211> 100
<212> DNA
<213> Escherichia coli K-12 MG1655
<220> oppf oppF
<222> 832-931
<223> oppf oppF U00096 1303788..1304792
<313> 4610: 885-904
<400> 4610
AACAAAACCA TCCAGTTACT GGAAGGGGAA TTACCGTCGC CGATCAACCC 50
GCCTTCCGGT TGTGTTTTCC GTACCCGTTG CCCGATTGCC GGTCCAGAGT 100
<210> 4611
<211> 100
<212> DNA
<213> Escherichia coli K-12 MG1655
<220> b0899 b0900
<222> 1524-1623
<223> b0899 b0900 U00096 946260..947882
<313> 4611: 1574-1593
<400> 4611
GTGGACCTTC CAGTTGGCGC TGAATGTTGC AACGCCGTTT GTGCTGGTAG 50
GATTAGGACT GATATTCCCG CTGCTGGCGC GTAAAGCGAA TAGTAAATAA 100
<210> 4612
<211> 100
<212> DNA
<213> Escherichia coli K-12 MG1655
<220> b0899 b0900
<222> 1424-1523
<223> b0899 b0900 U00096 946260..947882
<313> 4612: 1472-1491
<400> 4612
ATAAGCCTCT GGCAATGACC GTCGGGATTT GGTGTTTTGC CTTTACCGCC 50
TTTGCCTGTT TGACGGGGAT CTTCCCGAAA ATGGAAGCCT TCACTGCAGA 100
<210> 4613
<211> 100
<212> DNA
<213> Escherichia coli K-12 MG1655
<220> b0899 b0900
<222> 1370-1469
<223> b0899 b0900 U00096 946260..947882
<313> 4613: 1419-1438
<400> 4613
TTGCAGTCGT TCGCTTGGCG CAGAAATATA AACCAGAATA TGTCTTTATT 50
CGTAATAAGC CTCTGGCAAT GACCGTCGGG ATTTGGTGTT TTGCCTTTAC 100
<210> 4614
<211> 100
<212> DNA
<213> Escherichia coli K-12 MG1655
<220> pal b0742
<222> 263-362
<223> pal b0742 U00096 778290..778811
<313> 4614: 308-327
<400> 4614
TGGATGCACA TGCAAACTTC CTGCGTAGCA ACCCGTCTTA CAAAGTCACC 50
GTAGAAGGTC ACGCGGACGA ACGTGGTACT CCGGAATACA ACATCTCCCT 100
<210> 4615
<211> 100
<212> DNA
<213> Escherichia coli K-12 MG1655
<220> pal b0742
<222> 221-320
<223> pal b0742 U00096 778290..778811

<313> 4615: 251-270
<400> 4615
ATCTGGACAA GTACGATATC CGTTCTGACT TCGCTCAAAT GCTGGATGCA 50
CATGCAAACT TCCTGCGTAG CAACCCGTCT TACAAAGTCA CCGTAGAAGG 100
<210> 4616
<211> 100
<212> DNA
<213> Escherichia coli K-12 MG1655
<220> pal b0742
<222> 93-192
<223> pal b0742 U00096 778290..778811
<313> 4616: 154-173
<400> 4616
CGGCAGCGAA GGCATGCTGG GTGCCGGCAC TGGTATGGAT GCGAACGGCG 50
GCAACGGCAA CATGTCTTCC GAAGAGCAGG CTCGTCTGCA AATGCAACAG 100
<210> 4617
<211> 100
<212> DNA
<213> Escherichia coli K-12 MG1655
<220> feca b4292
<222> 2226-2325
<223> feca b4292 U00096 complement(4511921..4514245)
<313> 4617: 2267-2286
<400> 4617
CGACCAGGAC TACTTCATCC GCTCTTATGA CGACAACAAC AAAGGCATCT 50
ATGCAGGCCA GCCGCGCACG CTGTATATGC AGGGGTCGTT GAAGTTCTGA 100
<210> 4618
<211> 100
<212> DNA
<213> Escherichia coli K-12 MG1655
<220> feca b4292
<222> 2172-2271
<223> feca b4292 U00096 complement(4511921..4514245)
<313> 4618: 2235-2254
<400> 4618
CTTTGGCCCG CAGATGGCAG ATCTGAACCT GGCATTCGGT GTGAAAAACA 50
TCTTCGACCA GGACTACTTC ATCCGCTCTT ATGACGACAA CAACAAAGGC 100
<210> 4619
<211> 100
<212> DNA
<213> Escherichia coli K-12 MG1655
<220> feca b4292
<222> 2069-2168
<223> feca b4292 U00096 complement(4511921..4514245)
<313> 4619: 2088-2107
<400> 4619
CCAGCCAGTT TGCGGATAAC GCCAATACGG TGAAAGAGAG CGCCGACGGC 50
AGTACCGGCC GCATTCCCGG CTTCATGCTC TGGGGCGCAC GCGTGGCGTA 100
<210> 4620
<211> 100
<212> DNA
<213> Escherichia coli K-12 MG1655
<220> fecb b4291
<222> 810-909
<223> fecb b4291 U00096 complement(4510974..4511882)
<313> 4620: 880-899
<400> 4620
TAACACCTGG GCGCGGATGC GCGGTATTTT TGCTGCAGAG CGTATTGCCG 50
CTGACACGGT AAAAATCTTC CACCATCAGC CGCTTACCGT TGTGAAATGA 100
<210> 4621
<211> 100
<212> DNA
<213> Escherichia coli K-12 MG1655
<220> fecb b4291
<222> 731-830
<223> fecb b4291 U00096 complement(4510974..4511882)
<313> 4621: 760-779
<400> 4621
TTGTTAAACG CTGGCAACAA GATCCGCTCT GGCAGATGTT AACCGCCGCG 50
CAGAAGCAGC AGGTTGCTTC GGTCGACAGT AACACCTGGG CGCGGATGCG 100
<210> 4622

<211> 100
<212> DNA
<213> Escherichia coli K-12 MG1655
<220> fecb b4291
<222> 551-650
<223> fecb b4291 U00096 complement(4510974..4511882)
<313> 4622: 573-592
<400> 4622
CACGCGAACA GCAATTCAAC CTGCATACTC AGGAGACCTG GACCGGCAGC 50
GTGCTGGCCT CTCTGGGGCT GAACGTTCCC GCTGCGATGG CGGGTGCGTC 100
<210> 4623
<211> 100
<212> DNA
<213> Escherichia coli K-12 MG1655
<220> fecc b4290
<222> 798-897
<223> fecc b4290 U00096 complement(4509979..4510977)
<313> 4623: 835-854
<400> 4623
GGTGCCACAT CTGGCGCGCT TCTGGGCAGG CTTCGATCAG CGCAACGTAC 50
TGCCAGTGAG CATGCTGCTG GGGGCCACGC TGATGCTGCT GGCAGATGTA 100
<210> 4624
<211> 100
<212> DNA
<213> Escherichia coli K-12 MG1655
<220> fecc b4290
<222> 695-794
<223> fecc b4290 U00096 complement(4509979..4510977)
<313> 4624: 723-742
<400> 4624
GAGTGAACCT GACGAGGCTA CGTTTGGTCA TCAATATGTT AGTGCTGCTT 50
CTGGTTGGTG CGTGCGTCAG CGTAGCAGGT CCGGTGGCGT TTATCGGTCT 100
<210> 4625
<211> 100
<212> DNA
<213> Escherichia coli K-12 MG1655
<220> fecc b4290
<222> 629-728
<223> fecc b4290 U00096 complement(4509979..4510977)
<313> 4625: 678-697
<400> 4625
TCGTGTTGCT GCTGGCGAAT CAACTGAACC TGCTCAACCT CAGCGACAGC 50
ACCGCCCATA CGCTGGGAGT GAACCTGACG AGGCTACGTT TGGTCATCAA 100
<210> 4626
<211> 100
<212> DNA
<213> Escherichia coli K-12 MG1655
<220> alda b1416
<222> 1338-1437
<223> alda b1416 U00096 1486256..1487695
<313> 4626: 1385-1404
<400> 4626
AGGCTTCCAC GCCGGATGGC GTAAATCCGG TATTGGCGGC GCAGATGGTA 50
AACATGGCTT GCATGAATAT CTGCAGACCC AGGTGGTTTA TTTACAGTCT 100
<210> 4627
<211> 100
<212> DNA
<213> Escherichia coli K-12 MG1655
<220> alda b1416
<222> 1206-1305
<223> alda b1416 U00096 1486256..1487695
<313> 4627: 1246-1265
<400> 4627
AATGGCTAAT GACAGTGATT ACGGCCTGAC CTCATCAATC TATACCCAAA 50
ATCTGAACGT CGCGATGAAA GCCATTAAAG GGCTGAAGTT TGGTGAAACT 100
<210> 4628
<211> 100
<212> DNA
<213> Escherichia coli K-12 MG1655
<220> alda b1416
<222> 1144-1243

<223> alda b1416 U00096 1486256..1487695
<313> 4628: 1192-1211
<400> 4628
GAGGAAACCT TTGGCCCGGT GCTGCCAGTT GTCGCATTTG ACACGCTGGA  50
AGATGCTATC TCAATGGCTA ATGACAGTGA TTACGGCCTG ACCTCATCAA  100
<210> 4629
<211> 100
<212> DNA
<213> Escherichia coli K-12 MG1655
<220> fecd b4289
<222> 648-747
<223> fecd b4289 U00096 complement(4509026..4509982)
<313> 4629: 686-705
<400> 4629
GCTCGGTGTG TCGGTGCCCC ATACCCGATT CTGGGCTTTG TTACTAGCTG  50
TCGCCATGAC ATCTACCGGC GTGGCCGCCT GCGGCCCGAT TAGCTTTATT  100
<210> 4630
<211> 100
<212> DNA
<213> Escherichia coli K-12 MG1655
<220> fecd b4289
<222> 542-641
<223> fecd b4289 U00096 complement(4509026..4509982)
<313> 4630: 606-625
<400> 4630
GCTTTGTGAA GATTGCCATC CCGCTGATGA TTTTATTTCT GCCGCTGAGC  50
CTGAGTTTTT GCCGCGATCT CGACCTCCTT GCACTCGGCG ATGCGCGCGC  100
<210> 4631
<211> 100
<212> DNA
<213> Escherichia coli K-12 MG1655
<220> fecd b4289
<222> 505-604
<223> fecd b4289 U00096 complement(4509026..4509982)
<313> 4631: 565-584
<400> 4631
CTGTGGCTGA CCGGCAGCTT ATGGGGCCGT GACTGGAGCT TTGTGAAGAT  50
TGCCATCCCG CTGATGATTT TATTTCTGCC GCTGAGCCTG AGTTTTTGCC  100
<210> 4632
<211> 100
<212> DNA
<213> Escherichia coli K-12 MG1655
<220> aldb b3589
<222> 1530-1629
<223> aldb b3589 U00096 complement(3752603..3754231)
<313> 4632: 1589-1608
<400> 4632
AGGTATCGGT CGCGAAACCC ACAAGATGAT GCTGGAGCAT TACCAGCAAA  50
CCAAGTGCCT GCTGGTGAGC TACTCGGATA AACCGTTGGG GCTGTTCTGA  100
<210> 4633
<211> 100
<212> DNA
<213> Escherichia coli K-12 MG1655
<220> aldb b3589
<222> 1431-1530
<223> aldb b3589 U00096 complement(3752603..3754231)
<313> 4633: 1491-1510
<400> 4633
GGCCTATAAG ATGGGGCGCG GCATACAGGC TGGGCGCGTG TGGACCAACT  50
GTTATCACGC TTACCCGGCA CATGCGGCGT TTGGTGGCTA CAAACAATCA  100
<210> 4634
<211> 100
<212> DNA
<213> Escherichia coli K-12 MG1655
<220> aldb b3589
<222> 1376-1475
<223> aldb b3589 U00096 complement(3752603..3754231)
<313> 4634: 1431-1450
<400> 4634
CGAACGATAC GCAATATGGC CTGGGCGCGG GCGTCTGGAG CCGCAACGGT  50
AATCTGGCCT ATAAGATGGG GCGCGGCATA CAGGCTGGGC GCGTGTGGAC  100

<210> 4635
<211> 100
<212> DNA
<213> Escherichia coli K-12 MG1655
<220> fece b4288
<222> 669-768
<223> fece b4288 U00096 complement(4508258..4509025)
<313> 4635: 737-756
<400> 4635
AGAGGTGATG ACCCCAGGAT TGCTGAGAAC AGTATTCAGC GTGGAAGCGG 50
AAATACACCC CGAGCCGGTA TCTGGCAGGC CGATGTGCCT AATGAGGTAG 100
<210> 4636
<211> 100
<212> DNA
<213> Escherichia coli K-12 MG1655
<220> fece b4288
<222> 652-751
<223> fece b4288 U00096 complement(4508258..4509025)
<313> 4636: 670-689
<400> 4636
GCGCAAGGCA CACCAGAAGA GGTGATGACC CCAGGATTGC TGAGAACAGT 50
ATTCAGCGTG GAAGCGGAAA TACACCCCGA GCCGGTATCT GGCAGGCCGA 100
<210> 4637
<211> 100
<212> DNA
<213> Escherichia coli K-12 MG1655
<220> fece b4288
<222> 481-580
<223> fece b4288 U00096 complement(4508258..4509025)
<313> 4637: 531-550
<400> 4637
TTACTTGATG AGCCAACCAC CTATCTTGAT ATCAATCACC AGGTGGACCT 50
GATGCGGTTG ATGGGCGAAC TCCGGACTCA GGGGAAAACG GTGGTCGCTG 100
<210> 4638
<211> 100
<212> DNA
<213> Escherichia coli K-12 MG1655
<220> feci b4294
<222> 423-522
<223> feci b4294 U00096 complement(4515282..4515803)
<313> 4638: 500-519
<400> 4638
GATTGCGCAC AAACTCGGTG TTTCCATCAG CTCCGTGAAA AAATACGTGG 50
CGAAAGCCGT CGAGCACTGC CTGCTGTTCC GTCTGGAGTA TGGGTTATGA 100
<210> 4639
<211> 100
<212> DNA
<213> Escherichia coli K-12 MG1655
<220> feci b4294
<222> 423-522
<223> feci b4294 U00096 complement(4515282..4515803)
<313> 4639: 454-473
<400> 4639
GATTGCGCAC AAACTCGGTG TTTCCATCAG CTCCGTGAAA AAATACGTGG 50
CGAAAGCCGT CGAGCACTGC CTGCTGTTCC GTCTGGAGTA TGGGTTATGA 100
<210> 4640
<211> 100
<212> DNA
<213> Escherichia coli K-12 MG1655
<220> feci b4294
<222> 337-436
<223> feci b4294 U00096 complement(4515282..4515803)
<313> 4640: 388-407
<400> 4640
CTCCTCGACA GCATGCTGGA CGGGCTAAAC GGCAAAACAC GTGAAGCGTT 50
TCTGCTTTCG CAACTGGATG GTCTGACATA CAGCGAGATT GCGCACAAAC 100
<210> 4641
<211> 100
<212> DNA
<213> Escherichia coli K-12 MG1655
<220> aldh b1301

<222> 1336-1435
<223> aldh b1301 U00096 1360767..1362254
<313> 4641: 1378-1397
<400> 4641
GCCGGTTCCG TCTTCGTCAA TAACTACAAC GACGGCGATA TGACCGTGCC  50
GTTTGGCGGC TATAAGCAGA GCGGCAACGG TCGCGACAAA TCCCTGCATG  100
<210> 4642
<211> 100
<212> DNA
<213> Escherichia coli K-12 MG1655
<220> aldh b1301
<222> 1224-1323
<223> aldh b1301 U00096 1360767..1362254
<313> 4642: 1266-1285
<400> 4642
CACATCAGAA GAACAGGCGC TACAGCTTGC CAACGACAGC CAGTACGGCC  50
TTGGCGCGGC GGTATGGACG CGCGACCTCT CCCGCGCGCA CCGCATGAGC  100
<210> 4643
<211> 100
<212> DNA
<213> Escherichia coli K-12 MG1655
<220> aldh b1301
<222> 1118-1217
<223> aldh b1301 U00096 1360767..1362254
<313> 4643: 1161-1180
<400> 4643
CCGGGCTGGC TGCCGCCATC GGCCCGACCA TCTTTGTGGA TGTGGACCCG  50
AATGCGTCCT TAAGTCGCGA AGAGATTTTC GGTCCGGTGC TGGTGGTCAC  100
<210> 4644
<211> 100
<212> DNA
<213> Escherichia coli K-12 MG1655
<220> znta b3470
<222> 2067-2166
<223> znta b3470 U00096 3604082..3606280
<313> 4644: 2087-2106
<400> 4644
GCTGGGGCTG AAAGGGATCT TCCTCGTCAC CACGCTGTTA GGGATGACCG  50
GGTTGTGGCT GGCAGTGCTG GCAGATACGG GGGCGACGGT GCTGGTGACA  100
<210> 4645
<211> 100
<212> DNA
<213> Escherichia coli K-12 MG1655
<220> znta b3470
<222> 2011-2110
<223> znta b3470 U00096 3604082..3606280
<313> 4645: 2060-2079
<400> 4645
ATGATTGAAC TGGCACGCGC CACTCACGCC AATATCCGCC AGAACATCAC  50
TATTGCGCTG GGGCTGAAAG GGATCTTCCT CGTCACCACG CTGTTAGGGA  100
<210> 4646
<211> 100
<212> DNA
<213> Escherichia coli K-12 MG1655
<220> znta b3470
<222> 1930-2029
<223> znta b3470 U00096 3604082..3606280
<313> 4646: 1975-1994
<400> 4646
GCAATGGGTA GCGGCACAGA CGTGGCGCTG GAAACCGCCG ACGCAGCATT  50
AACCCATAAC CACCTGCGCG GCCTGGTGCA AATGATTGAA CTGGCACGCG  100
<210> 4647
<211> 100
<212> DNA
<213> Escherichia coli K-12 MG1655
<220> fecr b4293
<222> 855-954
<223> fecr b4293 U00096 complement(4514332..4515285)
<313> 4647: 904-923
<400> 4647
GCTGAAAAAT ACCGATGCGA TCCTGAACGT TATCGCGCAA ACGCTTCCCG  50

TTAAAATTCA GTCTATTACG CGGTACTGGA TAAACATTTC ACCACTGTAA 100
<210> 4648
<211> 100
<212> DNA
<213> Escherichia coli K-12 MG1655
<220> fecr b4293
<222> 805-904
<223> fecr b4293 U00096 complement(4514332..4515285)
<313> 4648: 855-874
<400> 4648
CTGCGCTGCG ATCCCGCCGT TGCCGGGCTG CGCCTGAGCG GGACGTTCCC 50
GCTGAAAAAT ACCGATGCGA TCCTGAACGT TATCGCGCAA ACGCTTCCCG 100
<210> 4649
<211> 100
<212> DNA
<213> Escherichia coli K-12 MG1655
<220> fecr b4293
<222> 757-856
<223> fecr b4293 U00096 complement(4514332..4515285)
<313> 4649: 820-839
<400> 4649
CCGCTGGGTG AGGTGATAGC CACGCTAACC CGTTACCGCA ACGGCGTGCT 50
GCGCTGCGAT CCCGCCGTTG CCGGGCTGCG CCTGAGCGGG ACGTTCCCGC 100
<210> 4650
<211> 100
<212> DNA
<213> Escherichia coli K-12 MG1655
<220> aas b2837
<222> 2061-2160
<223> aas b2837 U00096 complement(2971877..2974036)
<313> 4650: 2122-2141
<400> 4650
TATTCGCTAT CTGAAACAGA TGCCATTACT TGGCAGCGGC AAACCTGACT 50
TTGTCACGTT GAAAAGCTGG GTAGACGAAG CGGAACAACA CGATGAGTGA 100
<210> 4651
<211> 100
<212> DNA
<213> Escherichia coli K-12 MG1655
<220> aas b2837
<222> 2061-2160
<223> aas b2837 U00096 complement(2971877..2974036)
<313> 4651: 2084-2103
<400> 4651
TATTCGCTAT CTGAAACAGA TGCCATTACT TGGCAGCGGC AAACCTGACT 50
TTGTCACGTT GAAAAGCTGG GTAGACGAAG CGGAACAACA CGATGAGTGA 100
<210> 4652
<211> 100
<212> DNA
<213> Escherichia coli K-12 MG1655
<220> aas b2837
<222> 1899-1998
<223> aas b2837 U00096 complement(2971877..2974036)
<313> 4652: 1932-1951
<400> 4652
ACTTGGTGTT TCGCCAGATA AAGTCCATGC CACTGCGATT AAGAGCGATG 50
CCAGCAAAGG CGAGGCACTG GTGCTTTTCA CCACAGATAA CGAACTGACG 100
<210> 4653
<211> 100
<212> DNA
<213> Escherichia coli K-12 MG1655
<220> aat b0886
<222> 606-705
<223> aat b0886 U00096 complement(925951..926655)
<313> 4653: 652-671
<400> 4653
TCCCCGCCGG GATTACCTTA ATTATCTCAA TCAAATGCGC CTCGGACGAT 50
TGCCGAATAA TTTCTGGGTA CCACGATGCT TGTTTTCACC ACAAGAATGA 100
<210> 4654
<211> 100
<212> DNA
<213> Escherichia coli K-12 MG1655

<220> aat b0886
<222> 553-652
<223> aat b0886 U00096 complement(925951..926655)
<313> 4654: 611-630
<400> 4654
ATCGACTGCC AGGTCCTTAA CGATCACACA GCATCGCTTG GTGCCTGCGA 50
AATTCCCCGC CGGGATTACC TTAATTATCT CAATCAAATG CGCCTCGGAC 100
<210> 4655
<211> 100
<212> DNA
<213> Escherichia coli K-12 MG1655
<220> aat b0886
<222> 521-620
<223> aat b0886 U00096 complement(925951..926655)
<313> 4655: 567-586
<400> 4655
GTGAGGAATT TATCGGTCAT GGCGGTAAGC TTATCGACTG CCAGGTCCTT 50
AACGATCACA CAGCATCGCT TGGTGCCTGC GAAATTCCCC GCCGGGATTA 100
<210> 4656
<211> 100
<212> DNA
<213> Escherichia coli K-12 MG1655
<220> pcm b2744
<222> 528-627
<223> pcm b2744 U00096 complement(2866916..2867542)
<313> 4656: 585-604
<400> 4656
CCAGTATTTG AAACGGGTGC GTCGTCGGGG AGGCGAATTT ATTATCGATA 50
CCGTGGAGGC CGTGCGCTTT GTCCCTTTAG TGAAGGGTGA GCTGGCTTAA 100
<210> 4657
<211> 100
<212> DNA
<213> Escherichia coli K-12 MG1655
<220> pcm b2744
<222> 477-576
<223> pcm b2744 U00096 complement(2866916..2867542)
<313> 4657: 504-523
<400> 4657
GACGCAGCTG GACGAAGGCG GGATTCTCGT CTTACCCGTA GGGGAGGAGC 50
ACCAGTATTT GAAACGGGTG CGTCGTCGGG GAGGCGAATT TATTATCGAT 100
<210> 4658
<211> 100
<212> DNA
<213> Escherichia coli K-12 MG1655
<220> pcm b2744
<222> 404-503
<223> pcm b2744 U00096 complement(2866916..2867542)
<313> 4658: 447-466
<400> 4658
GTTGGCAGGC ACGTGCGCCG TTTGACGCTA TCATTGTTAC GGCGGCACCG 50
CCGGAAATTC CAACTGCGCT AATGACGCAG CTGGACGAAG GCGGGATTCT 100
<210> 4659
<211> 100
<212> DNA
<213> Escherichia coli K-12 MG1655
<220> yjaa b4012
<222> 225-324
<223> yjaa b4012 U00096 4210813..4211196
<313> 4659: 264-283
<400> 4659
ACTGGAAATG AATGAGGGCG GTTTGTCACA GGTTGAGGAA CGCATTCTCC 50
ATGAAGTGGT CGCTGGGGCA ACGTTAATGA AATATCGCCA GTTCCACATC 100
<210> 4660
<211> 100
<212> DNA
<213> Escherichia coli K-12 MG1655
<220> yjaa b4012
<222> 187-286
<223> yjaa b4012 U00096 4210813..4211196
<313> 4660: 220-239
<400> 4660

TTTTTGCCAG CAAAACGTAT GGATATTGTT GTGAGCGCAC TGGAAATGAA 50
TGAGGGCGGT TTGTCACAGG TTGAGGAACG CATTCTCCAT GAAGTGGTCG 100
<210> 4661
<211> 100
<212> DNA
<213> Escherichia coli K-12 MG1655
<220> yjaa b4012
<222> 104-203
<223> yjaa b4012 U00096 4210813..4211196
<313> 4661: 170-189
<400> 4661
TGTTAATCGC CAATTTCTTT GTTGCAGAAA AAGTTCTGCA AGATCTTGTT 50
CTGCAACTCC ACCCACGTTC AACCTGGCAT TCTTTTTTGC CAGCAAAACG 100
<210> 4662
<211> 100
<212> DNA
<213> Escherichia coli K-12 MG1655
<220> yjab b4013
<222> 345-444
<223> yjab b4013 U00096 complement(4211259..4211702)
<313> 4662: 412-431
<400> 4662
GTTCTATAAG AAGGTGGGTT TTAAGGTTAC GGGACGCTCT GAGGTGGACG 50
ATTTGGGGAA ACCGTATCCG TTGCTGAATC TGGCGTATGT GGGGGCGTAG 100
<210> 4663
<211> 100
<212> DNA
<213> Escherichia coli K-12 MG1655
<220> yjab b4013
<222> 326-425
<223> yjab b4013 U00096 complement(4211259..4211702)
<313> 4663: 367-386
<400> 4663
AAAATGAGCA GGCGGTTGGG TTCTATAAGA AGGTGGGTTT TAAGGTTACG 50
GGACGCTCTG AGGTGGACGA TTTGGGGAAA CCGTATCCGT TGCTGAATCT 100
<210> 4664
<211> 100
<212> DNA
<213> Escherichia coli K-12 MG1655
<220> yjab b4013
<222> 295-394
<223> yjab b4013 U00096 complement(4211259..4211702)
<313> 4664: 345-364
<400> 4664
GCACCGGAAC TGACAACCAA CGTTAATGAG CAAAATGAGC AGGCGGTTGG 50
GTTCTATAAG AAGGTGGGTT TTAAGGTTAC GGGACGCTCT GAGGTGGACG 100
<210> 4665
<211> 100
<212> DNA
<213> Escherichia coli K-12 MG1655
<220> abc b0200
<222> 928-1027
<223> abc b0200 U00096 complement(221614..222645)
<313> 4665: 953-972
<400> 4665
GGCATCATGC TGACTGAAAT GCACGGCACA CAACAAGATA CGCAAGCCGC 50
CATTGCCTGG CTGCAGGAAC ACCATGTAAA AGTAGAGGTA CTGGGTTATG 100
<210> 4666
<211> 100
<212> DNA
<213> Escherichia coli K-12 MG1655
<220> abc b0200
<222> 738-837
<223> abc b0200 U00096 complement(221614..222645)
<313> 4666: 793-812
<400> 4666
TCTGGATATC CCGGAAGATT ACCAGGAACG TCTGCAAGCG GAGCCATTTA 50
CTGACTGCGT GCCGATGCTG CGTCTGGAGT TTACCGGTCA ATCGGTCGAT 100
<210> 4667
<211> 100
<212> DNA

<213> Escherichia coli K-12 MG1655
<220> abc b0200
<222> 695-794
<223> abc b0200 U00096 complement(221614..222645)
<313> 4667: 764-783
<400> 4667
CGAAAACGCC GCTGGCGCAG AAGTTTATTC AGTCGACCCT GCATCTGGAT 50
ATCCCGGAAG ATTACCAGGA ACGTCTGCAA GCGGAGCCAT TTACTGACTG 100
<210> 4668
<211> 100
<212> DNA
<213> Escherichia coli K-12 MG1655
<220> yjad b3997
<222> 668-767
<223> yjad b3997 U00096 4194481..4195254
<313> 4668: 689-708
<400> 4668
CGAACTGGTA TCGCTATGAC GATTTGCCGT TACTCCCGCC GCCCGGCACC 50
GTAGCGCGCC GTCTGATAGA AGATACGGTG GCGATGTGTC GGGCAGAGTA 100
<210> 4669
<211> 100
<212> DNA
<213> Escherichia coli K-12 MG1655
<220> yjad b3997
<222> 554-653
<223> yjad b3997 U00096 4194481..4195254
<313> 4669: 608-627
<400> 4669
ACTTGCGTTA CGTGACTTCT CAGCCGTGGC CGTTTCCTCA GTCTTTAATG 50
ACCGCGTTTA TGGCGGAATA TGACAGCGGC GACATCGTGA TCGACCCGAA 100
<210> 4670
<211> 100
<212> DNA
<213> Escherichia coli K-12 MG1655
<220> yjad b3997
<222> 502-601
<223> yjad b3997 U00096 4194481..4195254
<313> 4670: 517-536
<400> 4670
GAGCAGGCAG TCGCGCGGGA AGTGATGGAA GAGAGCGGAA TTAAAGTTAA 50
AAACTTGCGT TACGTGACTT CTCAGCCGTG GCCGTTTCCT CAGTCTTTAA 100
<210> 4671
<211> 100
<212> DNA
<213> Escherichia coli K-12 MG1655
<220> yjae b3996
<222> 378-477
<223> yjae b3996 U00096 complement(4193910..4194386)
<313> 4671: 437-456
<400> 4671
TTTATCTGAC ATCGGCGAGG CGCTGGAAGC GCGCTTTGTG CTGGAAGATA 50
AGCTGATTCT GCTGGTGCTT GACGCCGCCC GCGTCAAACA TCCTGCTTGA 100
<210> 4672
<211> 100
<212> DNA
<213> Escherichia coli K-12 MG1655
<220> yjae b3996
<222> 349-448
<223> yjae b3996 U00096 complement(4193910..4194386)
<313> 4672: 385-404
<400> 4672
CATGATAATT ACCTTGAGTT TCAACAGGTT TTATCTGACA TCGGCGAGGC 50
GCTGGAAGCG CGCTTTGTGC TGGAAGATAA GCTGATTCTG CTGGTGCTTG 100
<210> 4673
<211> 100
<212> DNA
<213> Escherichia coli K-12 MG1655
<220> yjae b3996
<222> 249-348
<223> yjae b3996 U00096 complement(4193910..4194386)
<313> 4673: 298-317

<400> 4673
CGGGCAACTC GCGCGCGCCG CGAAGATTTG GCCGCAACTC GAAGCCAATA 50
CTCAACAGAT TATGGATTAC TACGATTCCA GTCTGGAAAC CGCCATCGAT 100
<210> 4674
<211> 100
<212> DNA
<213> Escherichia coli K-12 MG1655
<220> yjag b4000
<222> 296-395
<223> yjag b4000 U00096 4197082..4197672
<313> 4674: 351-370
<400> 4674
CGTTAAGTGA ACTGGTCCAT TCGCGTTTGA GTGGTGAAAC GCTCGAACAC 50
GCGGTGGAAG TGAGTAAGAC CTCCATCACG ACCGTTGCGA TGCTGGAAAT 100
<210> 4675
<211> 100
<212> DNA
<213> Escherichia coli K-12 MG1655
<220> yjag b4000
<222> 206-305
<223> yjag b4000 U00096 4197082..4197672
<313> 4675: 233-252
<400> 4675
ACAGCCAACT GGAGAAATTT GAAGAAGCGA TTCCTTCAGC CGACGATTTC 50
GATCTGTACG GCGTTTATCC GGCAATCGAT GCCTGCGTGG CGTTAAGTGA 100
<210> 4676
<211> 100
<212> DNA
<213> Escherichia coli K-12 MG1655
<220> yjag b4000
<222> 169-268
<223> yjag b4000 U00096 4197082..4197672
<313> 4676: 185-204
<400> 4676
GAAACGCTGA CCGTTAAAGA CGCAAAAGTA AATTTCGACA GCCAACTGGA 50
GAAATTTGAA GAAGCGATTC CTTCAGCCGA CGATTTCGAT CTGTACGGCG 100
<210> 4677
<211> 100
<212> DNA
<213> Escherichia coli K-12 MG1655
<220> yjah b4002
<222> 597-696
<223> yjah b4002 U00096 4198144..4198839
<313> 4677: 674-693
<400> 4677
TGTCAGCGTG GCATGGCTGG AAGCGCCCGA AGGTTCGCAA TTACTGTTAG 50
TGGCAAACTC TGATTTCTGT CGCTGGCAAC CCAACGAGAA AACGTTCTGA 100
<210> 4678
<211> 100
<212> DNA
<213> Escherichia coli K-12 MG1655
<220> yjah b4002
<222> 582-681
<223> yjah b4002 U00096 4198144..4198839
<313> 4678: 623-642
<400> 4678
CCGCTCTTCT GTTGATGTCA GCGTGGCATG GCTGGAAGCG CCCGAAGGTT 50
CGCAATTACT GTTAGTGGCA AACTCTGATT TCTGTCGCTG GCAACCCAAC 100
<210> 4679
<211> 100
<212> DNA
<213> Escherichia coli K-12 MG1655
<220> yjah b4002
<222> 522-621
<223> yjah b4002 U00096 4198144..4198839
<313> 4679: 565-584
<400> 4679
GCAAACCATT AAACCTGATT TAGATTCGCA GGCGATAGCG CATATCGAGC 50
GCCGCCAGAG CCGCTCTTCT GTTGATGTCA GCGTGGCATG GCTGGAAGCG 100
<210> 4680
<211> 100

<212> DNA
<213> Escherichia coli K-12 MG1655
<220> yjai b4003
<222> 416-515
<223> yjai b4003 U00096 complement(4198841..4199407)
<313> 4680: 444-463
<400> 4680
AAGAGATGGA GAATTTGCGT CAGTCGTTAG ATGAGTTACG GGTGAAACGA 50
GATATCGCGA TGGCTGAAGC GGGTATTCCG CGCGGAGCCG GAATGGGCAT 100
<210> 4681
<211> 100
<212> DNA
<213> Escherichia coli K-12 MG1655
<220> yjai b4003
<222> 322-421
<223> yjai b4003 U00096 complement(4198841..4199407)
<313> 4681: 384-403
<400> 4681
GCACTGCAAC AGCAACTGGT GACGAAGCGT TATGAATACA ATGCCCTGTT 50
AGCCGCGAAC CCACCGGATA GCAGCAAAAT TAACGCGGTC GCCAAAGAGA 100
<210> 4682
<211> 100
<212> DNA
<213> Escherichia coli K-12 MG1655
<220> yjai b4003
<222> 309-408
<223> yjai b4003 U00096 complement(4198841..4199407)
<313> 4682: 331-350
<400> 4682
CGCTCAAAGC AGCGCACTGC AACAGCAACT GGTGACGAAG CGTTATGAAT 50
ACAATGCCCT GTTAGCCGCG AACCCACCGG ATAGCAGCAA AATTAACGCG 100
<210> 4683
<211> 100
<212> DNA
<213> Escherichia coli K-12 MG1655
<220> mgla b2150
<222> 1422-1521
<223> mgla b2150 U00096 complement(2235789..2237309)
<313> 4683: 1502-1521
<400> 4683
CCGTATTCTG GTCATGAGCA ATGGTCTCGT TTCCGGAATT GTCGATACAA 50
AAACAACAAC GCAAAACGAA ATTCTGCGTC TTGCGTCTTT GCACCTTTAA 100
<210> 4684
<211> 100
<212> DNA
<213> Escherichia coli K-12 MG1655
<220> mgla b2150
<222> 1379-1478
<223> mgla b2150 U00096 complement(2235789..2237309)
<313> 4684: 1409-1428
<400> 4684
TTATTATCTC CTCTGAAATG CCTGAGTTGT TAGGGATAAC AGACCGTATT 50
CTGGTCATGA GCAATGGTCT CGTTTCCGGA ATTGTCGATA CAAAAACAAC 100
<210> 4685
<211> 100
<212> DNA
<213> Escherichia coli K-12 MG1655
<220> mgla b2150
<222> 1323-1422
<223> mgla b2150 U00096 complement(2235789..2237309)
<313> 4685: 1354-1373
<400> 4685
GAAGTTTGAA ATTTATCAGT TAATTGCCGA ACTGGCGAAG AAAGGCAAGG 50
GGATTATTAT TATCTCCTCT GAAATGCCTG AGTTGTTAGG GATAACAGAC 100
<210> 4686
<211> 100
<212> DNA
<213> Escherichia coli K-12 MG1655
<220> mglb b2151
<222> 874-973
<223> mglb b2151 U00096 complement(2237370..2238368)

<313> 4686: 918-937
<400> 4686
GCGAAAAACC TGGCCGATGG TAAAGGTGCG GCTGATGGCA CCAACTGGAA 50
AATCGACAAC AAAGTGGTCC GCGTACCTTA TGTTGGCGTA GATAAAGACA 100
<210> 4687
<211> 100
<212> DNA
<213> Escherichia coli K-12 MG1655
<220> mglb b2151
<222> 834-933
<223> mglb b2151 U00096 complement(2237370..2238368)
<313> 4687: 891-910
<400> 4687
GAACGATGCT AACAACCAGG CGAAAGCGAC CTTTGATCTG GCGAAAAACC 50
TGGCCGATGG TAAAGGTGCG GCTGATGGCA CCAACTGGAA AATCGACAAC 100
<210> 4688
<211> 100
<212> DNA
<213> Escherichia coli K-12 MG1655
<220> mglb b2151
<222> 801-900
<223> mglb b2151 U00096 complement(2237370..2238368)
<313> 4688: 850-869
<400> 4688
GGTGAAATCC GGTGCACTGG CGGGCACCGT ACTGAACGAT GCTAACAACC 50
AGGCGAAAGC GACCTTTGAT CTGGCGAAAA ACCTGGCCGA TGGTAAAGGT 100
<210> 4689
<211> 100
<212> DNA
<213> Escherichia coli K-12 MG1655
<220> mglc b2149
<222> 912-1011
<223> mglc b2149 U00096 complement(2234763..2235773)
<313> 4689: 939-958
<400> 4689
GTATATCGGC GTAAACCCAT ACTGGCAGTA CATCATCAAA GGGGCGATTA 50
TTATCTTCGC CGTAGCGCTG GATTCACTGA AATACGCGCG TAAGAAATGA 100
<210> 4690
<211> 100
<212> DNA
<213> Escherichia coli K-12 MG1655
<220> mglc b2149
<222> 854-953
<223> mglc b2149 U00096 complement(2234763..2235773)
<313> 4690: 913-932
<400> 4690
CGGTGATTGG CGTGGTGACC GGGGTAATTA TTTTTACCGT CATCAACTAT 50
GGCCTGACGT ATATCGGCGT AAACCCATAC TGGCAGTACA TCATCAAAGG 100
<210> 4691
<211> 100
<212> DNA
<213> Escherichia coli K-12 MG1655
<220> mglc b2149
<222> 824-923
<223> mglc b2149 U00096 complement(2234763..2235773)
<313> 4691: 859-878
<400> 4691
GCGGTGTATC GTTCAGCGGC GGTGTGGGGA CGGTGATTGG CGTGGTGACC 50
GGGGTAATTA TTTTTACCGT CATCAACTAT GGCCTGACGT ATATCGGCGT 100
<210> 4692
<211> 100
<212> DNA
<213> Escherichia coli K-12 MG1655
<220> yjba b4031
<222> 312-411
<223> yjba b4031 U00096 4237904..4238314
<313> 4692: 389-408
<400> 4692
TCATAAATCG CCACTGGATG TGTTGATCTA CTCGGCTGCG ATCCTGTTAC 50
TGGTGATCAC CCTGTGGCTG TGTAATTCGA AACGGCTGAA GCGGGAGTAA 100
<210> 4693

<211> 100
<212> DNA
<213> Escherichia coli K-12 MG1655
<220> yjba b4031
<222> 291-390
<223> yjba b4031 U00096 4237904..4238314
<313> 4693: 337-356
<400> 4693
TGTGCGCTTG ATCATCGTCG ATCATAAATC GCCACTGGAT GTGTTGATCT 50
ACTCGGCTGC GATCCTGTTA CTGGTGATCA CCCTGTGGCT GTGTAATTCG 100
<210> 4694
<211> 100
<212> DNA
<213> Escherichia coli K-12 MG1655
<220> yjba b4031
<222> 253-352
<223> yjba b4031 U00096 4237904..4238314
<313> 4694: 305-324
<400> 4694
CCGCTACGTT ACTTTGTCTA TATTGGGATC ACCGCCATTG TGCGCTTGAT 50
CATCGTCGAT CATAAATCGC CACTGGATGT GTTGATCTAC TCGGCTGCGA 100
<210> 4695
<211> 100
<212> DNA
<213> Escherichia coli K-12 MG1655
<220> yjbb b4021
<222> 1533-1632
<223> yjbb b4021 U00096 4225310..4226941
<313> 4695: 1597-1616
<400> 4695
CTTACTGGGA GATATGCAGC GCCTGAACTC GCTGTTTTGT TCGGTGGCTT 50
ACAGTGTGCT GGAACAGCCG GATGAAGATG AAGGACGGGA CGAGTATTAA 100
<210> 4696
<211> 100
<212> DNA
<213> Escherichia coli K-12 MG1655
<220> yjbb b4021
<222> 1515-1614
<223> yjbb b4021 U00096 4225310..4226941
<313> 4696: 1563-1582
<400> 4696
CAGTTCGCTA CATTTAGGCT TACTGGGAGA TATGCAGCGC CTGAACTCGC 50
TGTTTTGTTC GGTGGCTTAC AGTGTGCTGG AACAGCCGGA TGAAGATGAA 100
<210> 4697
<211> 100
<212> DNA
<213> Escherichia coli K-12 MG1655
<220> yjbb b4021
<222> 1475-1574
<223> yjbb b4021 U00096 4225310..4226941
<313> 4697: 1506-1525
<400> 4697
TCGATCGCCT GCATCAGCAA AACGTGCAGA GCATTGAAAC CAGTTCGCTA 50
CATTTAGGCT TACTGGGAGA TATGCAGCGC CTGAACTCGC TGTTTTGTTC 100
<210> 4698
<211> 100
<212> DNA
<213> Escherichia coli K-12 MG1655
<220> yjbc b4023
<222> 774-873
<223> yjbc b4023 U00096 4227933..4228805
<313> 4698: 844-863
<400> 4698
TCCAGTCGTT AAGATGGAAA AAACGGCGGA AAAAGGCGGT CGCCCGGCGT 50
CCAACGGTAA GCGTTTTACC TCGCCGGGGC GTAAAAAGAA GGGGCGCTGA 100
<210> 4699
<211> 100
<212> DNA
<213> Escherichia coli K-12 MG1655
<220> yjbc b4023
<222> 748-847

<223> yjbc b4023 U00096 4227933..4228805
<313> 4699: 787-806
<400> 4699
AAACCGAAAA CAGCGGGCAT CAAACGTCCA GTCGTTAAGA TGGAAAAAAC  50
GGCGGAAAAA GGCGGTCGCC CGGCGTCCAA CGGTAAGCGT TTTACCTCGC  100
<210> 4700
<211> 100
<212> DNA
<213> Escherichia coli K-12 MG1655
<220> yjbc b4023
<222> 719-818
<223> yjbc b4023 U00096 4227933..4228805
<313> 4700: 744-763
<400> 4700
CTTCCGAGGT AAAACCTAAA GCGAAGGCCA AACCGAAAAC AGCGGGCATC  50
AAACGTCCAG TCGTTAAGAT GGAAAAAACG GCGGAAAAAG GCGGTCGCCC  100
<210> 4701
<211> 100
<212> DNA
<213> Escherichia coli K-12 MG1655
<220> yjbd b4024
<222> 142-241
<223> yjbd b4024 U00096 complement(4228938..4229210)
<313> 4701: 200-219
<400> 4701
GATAAATTAA TGGTTGAGCG TGAGGCTGAA GCGAAAAAAG CCCGCCAGTT  50
GAAGAAAAAG CAGGCTTATA AACCGGATCC TGAAGCGTCA TTTAGCTGGT  100
<210> 4702
<211> 100
<212> DNA
<213> Escherichia coli K-12 MG1655
<220> yjbd b4024
<222> 90-189
<223> yjbd b4024 U00096 complement(4228938..4229210)
<313> 4702: 143-162
<400> 4702
TGGTCGCGTA TTGACCAACT CCGAAACCAA CAGCATTAAG AAAGATTACA  50
TCGATAAATT AATGGTTGAG CGTGAGGCTG AAGCGAAAAA AGCCCGCCAG  100
<210> 4703
<211> 100
<212> DNA
<213> Escherichia coli K-12 MG1655
<220> yjbd b4024
<222> 71-170
<223> yjbd b4024 U00096 complement(4228938..4229210)
<313> 4703: 111-130
<400> 4703
ATCGGGCGCG GATTGCGCAT GGTCGCGTAT TGACCAACTC CGAAACCAAC  50
AGCATTAAGA AAGATTACAT CGATAAATTA ATGGTTGAGC GTGAGGCTGA  100
<210> 4704
<211> 100
<212> DNA
<213> Escherichia coli K-12 MG1655
<220> yjbe b4027
<222> 144-243
<223> yjbe b4027 U00096 4233485..4233727
<313> 4704: 206-225
<400> 4704
CACCGTAAGC TCGGCGGTGG GTGTCGCGCT TGCGGCAACC GGTGGCGGTG  50
ATGGTTCTAA TACCGGGACC ACCACAACCA CGACCACCAG TACCCAGTAA  100
<210> 4705
<211> 100
<212> DNA
<213> Escherichia coli K-12 MG1655
<220> yjbe b4027
<222> 52-151
<223> yjbe b4027 U00096 4233485..4233727
<313> 4705: 102-121
<400> 4705
TCTGCGTGGG CTGCACCTGT ACAGGTGGGC GAAGCGGCAG GGTCGGCAGC  50
AACGTCGGTT TCGGCGGGGA GTTCCTCCGC GACCAGCGTC AGCACCGTAA  100

<210> 4706
<211> 100
<212> DNA
<213> Escherichia coli K-12 MG1655
<220> yjbf b4028
<222> 570-669
<223> yjbf b4028 U00096 4233811..4234479
<313> 4706: 646-665
<400> 4706
TTGGATCGAC AGCGAAGGGC AAATTCGCCA GTCGGAACAG TATCTCGGCG 50
CGGATTATTT TCCGGTGAAA ACCACTCTCA TCAAGGCGGC AAAACAATGA 100
<210> 4707
<211> 100
<212> DNA
<213> Escherichia coli K-12 MG1655
<220> yjbf b4028
<222> 561-660
<223> yjbf b4028 U00096 4233811..4234479
<313> 4707: 585-604
<400> 4707
TAACCGCTAT TGGATCGACA GCGAAGGGCA AATTCGCCAG TCGGAACAGT 50
ATCTCGGCGC GGATTATTTT CCGGTGAAAA CCACTCTCAT CAAGGCGGCA 100
<210> 4708
<211> 100
<212> DNA
<213> Escherichia coli K-12 MG1655
<220> yjbf b4028
<222> 475-574
<223> yjbf b4028 U00096 4233811..4234479
<313> 4708: 531-550
<400> 4708
GGCACCGATA CCGTCAAAGT CGGCAGCGAT GAAACCCCGG TTCGCGTGCT 50
GGACGAAGAA GTCTCCACCG ACCAGGCGCG CTGGCATAAC CGCTATTGGA 100
<210> 4709
<211> 100
<212> DNA
<213> Escherichia coli K-12 MG1655
<220> yjbg b4029
<222> 639-738
<223> yjbg b4029 U00096 4234476..4235213
<313> 4709: 705-724
<400> 4709
CATTATTTAT GTTGGCCTCG CGGACTCCGT CTGGAGTGAG ACGCCTGATG 50
CCCTTAACGC CGACATTCTT CAGACTCTGA CGCAGCGGAT ACCTCAATAA 100
<210> 4710
<211> 100
<212> DNA
<213> Escherichia coli K-12 MG1655
<220> yjbg b4029
<222> 632-731
<223> yjbg b4029 U00096 4234476..4235213
<313> 4710: 667-686
<400> 4710
CCGGCAGCAT TATTTATGTT GGCCTCGCGG ACTCCGTCTG GAGTGAGACG 50
CCTGATGCCC TTAACGCCGA CATTCTTCAG ACTCTGACGC AGCGGATACC 100
<210> 4711
<211> 100
<212> DNA
<213> Escherichia coli K-12 MG1655
<220> yjbg b4029
<222> 579-678
<223> yjbg b4029 U00096 4234476..4235213
<313> 4711: 620-639
<400> 4711
CACGCAAAAA GCGCCGGTGG CTTACTGGAA CAAGCGTCAC GTAGAGCCGA 50
TGCCCGGCAG CATTATTTAT GTTGGCCTCG CGGACTCCGT CTGGAGTGAG 100
<210> 4712
<211> 100
<212> DNA
<213> Escherichia coli K-12 MG1655
<220> yjbh b4030

<222> 1998-2097
<223> yjbh b4030 U00096 4235213..4237309
<313> 4712: 2076-2095
<400> 4712
GATTGGCTGG ACGCCGCTGA CGCGTGACGG TGGTCAGCAA CTTGGGCGTA  50
AGTTCCAGTT GTATGACATG ACCAGCGACC GTAGCGTCAA TTTCCGCTAA  100
<210> 4713
<211> 100
<212> DNA
<213> Escherichia coli K-12 MG1655
<220> yjbh b4030
<222> 1913-2012
<223> yjbh b4030 U00096 4235213..4237309
<313> 4713: 1966-1985
<400> 4713
ACGGCGAAGG GGACTTCACC AAAGGCGTGT ATGTCTCGGT ACCGTTGGAT  50
CTCTTCTCGT CTGGCCCGAC ACGCAGCCGT GCGGCGATTG GCTGGACGCC  100
<210> 4714
<211> 100
<212> DNA
<213> Escherichia coli K-12 MG1655
<220> yjbh b4030
<222> 1862-1961
<223> yjbh b4030 U00096 4235213..4237309
<313> 4714: 1908-1927
<400> 4714
GCGTGGTGGT GGGTGGCTAT GCCACGATCA CTAATGTTTC GAAAGAGGAG  50
TACGGCGAAG GGGACTTCAC CAAAGGCGTG TATGTCTCGG TACCGTTGGA  100
<210> 4715
<211> 100
<212> DNA
<213> Escherichia coli K-12 MG1655
<220> yjbi b4039
<222> 1225-1324
<223> yjbi b4039 U00096 4248534..4249862
<313> 4715: 1268-1287
<400> 4715
ACTCAGGAAA CACTTTTCAG CTCCAAATCT CATGGTAAAT ATGCGCTTTT  50
CCCCATTTTT ACTGCGAGTT GGCGAGCTCA TCGTATAATG AATAAGGGTG  100
<210> 4716
<211> 100
<212> DNA
<213> Escherichia coli K-12 MG1655
<220> yjbi b4039
<222> 1075-1174
<223> yjbi b4039 U00096 4248534..4249862
<313> 4716: 1104-1123
<400> 4716
ATCTTTATCC TGTTCTCCTC AGTCGATGAT GGGCCAGTTA TGGTGGTAAG  50
CTCCCAGCGC TTACATGATA TGTTGAATCC TACAAAAGAT ACCAATTGGA  100
<210> 4717
<211> 100
<212> DNA
<213> Escherichia coli K-12 MG1655
<220> yjbi b4039
<222> 778-877
<223> yjbi b4039 U00096 4248534..4249862
<313> 4717: 807-826
<400> 4717
AACAAGTTCG ATGATTTTAT GCGTCAACAT CGATTCAGTG AGGTGAATTT  50
CGATACCCAA CAGATGATCG ATTTTATTAA CAGATTTAAT ACGAATAAAT  100
<210> 4718
<211> 100
<212> DNA
<213> Escherichia coli K-12 MG1655
<220> yjbj b4046
<222> 111-210
<223> yjbj b4046 U00096 4256816..4257025
<313> 4718: 183-202
<400> 4718
TCAACTGGTC GGTAAAATCC AGGAACGTTA TGGTTATCAG AAAGATCAGG  50

CAGAAAAAGA GGTCGTGGAT TGGGAAACCC GCAATGAATA TCGCTGGTAA 100
<210> 4719
<211> 100
<212> DNA
<213> Escherichia coli K-12 MG1655
<220> yjbj b4046
<222> 111-210
<223> yjbj b4046 U00096 4256816..4257025
<313> 4719: 162-181
<400> 4719
TCAACTGGTC GGTAAAATCC AGGAACGTTA TGGTTATCAG AAAGATCAGG 50
CAGAAAAAGA GGTCGTGGAT TGGGAAACCC GCAATGAATA TCGCTGGTAA 100
<210> 4720
<211> 100
<212> DNA
<213> Escherichia coli K-12 MG1655
<220> yjbj b4046
<222> 111-210
<223> yjbj b4046 U00096 4256816..4257025
<313> 4720: 156-175
<400> 4720
TCAACTGGTC GGTAAAATCC AGGAACGTTA TGGTTATCAG AAAGATCAGG 50
CAGAAAAAGA GGTCGTGGAT TGGGAAACCC GCAATGAATA TCGCTGGTAA 100
<210> 4721
<211> 100
<212> DNA
<213> Escherichia coli K-12 MG1655
<220> yjbk b4047
<222> 467-566
<223> yjbk b4047 U00096 complement(4257067..4257642)
<313> 4721: 519-538
<400> 4721
TGATTGAAGC ACATGGGCTC TGTGCGGCAT GTGTAGAAGT GGAAGCGTGT 50
CGTCATCCTG AACAGTGCCA GCATGATCAC TCTGTGCAGG TGAAAAAGAA 100
<210> 4722
<211> 100
<212> DNA
<213> Escherichia coli K-12 MG1655
<220> yjbk b4047
<222> 425-524
<223> yjbk b4047 U00096 complement(4257067..4257642)
<313> 4722: 470-489
<400> 4722
ATACGCTGGC GGCAAAAATG GGGTTTGCCC TGCGGCATAA TGTGATTGAA 50
GCACATGGGC TCTGTGCGGC ATGTGTAGAA GTGGAAGCGT GTCGTCATCC 100
<210> 4723
<211> 100
<212> DNA
<213> Escherichia coli K-12 MG1655
<220> yjbk b4047
<222> 345-444
<223> yjbk b4047 U00096 complement(4257067..4257642)
<313> 4723: 399-418
<400> 4723
CCATACGTCA GCCATGTTTA TTTGCGATCG CTGCGGCGCA GTGAAAGAAG 50
AGTGTGCAGA AGGCGTGGAA GACATTATGC ATACGCTGGC GGCAAAAATG 100
<210> 4724
<211> 100
<212> DNA
<213> Escherichia coli K-12 MG1655
<220> slpa b0029
<222> 331-430
<223> slpa b0029 U00096 25826..26275
<313> 4724: 350-369
<400> 4724
GGCGTGATCC GCGAAATTAA CGGCGACTCC ATTACCGTTG ATTTCAACCA 50
TCCGCTGGCC GGGCAGACCG TTCATTTTGA TATTGAAGTG CTGGAAATCG 100
<210> 4725
<211> 100
<212> DNA
<213> Escherichia coli K-12 MG1655

<220> slpa b0029
<222> 297-396
<223> slpa b0029 U00096 25826..26275
<313> 4725: 323-342
<400> 4725
GCTTTTTACC GCAATGGATG GCAGTGAGAT GCCTGGCGTG ATCCGCGAAA 50
TTAACGGCGA CTCCATTACC GTTGATTTCA ACCATCCGCT GGCCGGGCAG 100
<210> 4726
<211> 100
<212> DNA
<213> Escherichia coli K-12 MG1655
<220> slpa b0029
<222> 249-348
<223> slpa b0029 U00096 25826..26275
<313> 4726: 278-297
<400> 4726
CCGCCGTGAA TTTATGGATG CAGGCGAGCC AGAAATTGGC GCAATCATGC 50
TTTTTACCGC AATGGATGGC AGTGAGATGC CTGGCGTGAT CCGCGAAATT 100
<210> 4727
<211> 100
<212> DNA
<213> Escherichia coli K-12 MG1655
<220> yjbl b4048
<222> 5-104
<223> yjbl b4048 U00096 4257900..4258154
<313> 4727: 31-50
<400> 4727
TGAAGATAAT ACCCGGTGCA ACTGGATACT TTAATAAAAC ACTCAATTCA 50
AATCAATTCG ATAATGAAGA TGCAATCAAA GATAAATTAG ATAATAGAGG 100
<210> 4728
<211> 100
<212> DNA
<213> Escherichia coli K-12 MG1655
<220> yjbl b4048
<222> 1-100
<223> yjbl b4048 U00096 4257900..4258154
<313> 4728: 19-38
<400> 4728
ATGTTGAAGA TAATACCCGG TGCAACTGGA TACTTTAATA AAACACTCAA 50
TTCAAATCAA TTCGATAATG AAGATGCAAT CAAAGATAAA TTAGATAATA 100
<210> 4729
<211> 100
<212> DNA
<213> Escherichia coli K-12 MG1655
<220> yjbm b4049
<222> 505-604
<223> yjbm b4049 U00096 4258178..4258885
<313> 4729: 557-576
<400> 4729
TTTGATGGCT ATCTGGATAA GATGTCACAT CTGGTGAATG AAGGAACGCT 50
CGATCCTGAT ATTTTAAGCA AAATGCGAGA TGCTGCACCA CAATTAATTA 100
<210> 4730
<211> 100
<212> DNA
<213> Escherichia coli K-12 MG1655
<220> yjbm b4049
<222> 445-544
<223> yjbm b4049 U00096 4258178..4258885
<313> 4730: 475-494
<400> 4730
CGTGATATGA AGAAGTACCT GGGAGAGATG AAGCGTCTGG CTGATGGGGG 50
AGAAATGAAT TTTGATGGCT ATCTGGATAA GATGTCACAT CTGGTGAATG 100
<210> 4731
<211> 100
<212> DNA
<213> Escherichia coli K-12 MG1655
<220> yjbm b4049
<222> 256-355
<223> yjbm b4049 U00096 4258178..4258885
<313> 4731: 296-315
<400> 4731

GCAATAGGTT CTGGTTTTTT AACGTTTGTT AATGCCTGCC AGGAACATGA 50
TGACAACAGC TTAAAATTAT TTGCTTCGCT GTTAATTTCA CTTTCATATT 100
<210> 4732
<211> 100
<212> DNA
<213> Escherichia coli K-12 MG1655
<220> yjbn b4050
<222> 824-923
<223> yjbn b4050 U00096 4259248..4260285
<313> 4732: 888-907
<400> 4732
CGGTGGCGGT AGTGCGCGCC ATGTATCCGT ACATTGAGCG TGAACTCAGC 50
CAGGGGACGT ATCTCGGCCA TATTACCCGG CATATGTTGG GCTTGTTCCA 100
<210> 4733
<211> 100
<212> DNA
<213> Escherichia coli K-12 MG1655
<220> yjbn b4050
<222> 763-862
<223> yjbn b4050 U00096 4259248..4260285
<313> 4733: 805-824
<400> 4733
CCGGGTATTC TGGCGGCGGT AGACCGGGAG ATCTTTGGTT CCTCGGATAC 50
CGATGCCGAT CCGGTGGCGG TAGTGCGCGC CATGTATCCG TACATTGAGC 100
<210> 4734
<211> 100
<212> DNA
<213> Escherichia coli K-12 MG1655
<220> yjbn b4050
<222> 700-799
<223> yjbn b4050 U00096 4259248..4260285
<313> 4734: 731-750
<400> 4734
GAGGCCAAAG CACACCTGCA ACATATGGAT GGCGTGATGG TCGGGCGCGA 50
GGCGTATCAG AATCCGGGTA TTCTGGCGGC GGTAGACCGG GAGATCTTTG 100
<210> 4735
<211> 100
<212> DNA
<213> Escherichia coli K-12 MG1655
<220> yjbo b4051
<222> 354-453
<223> yjbo b4051 U00096 4260209..4260661
<313> 4735: 409-428
<400> 4735
GCCGTGGTTA CTACTGGCGA TTGCGGTGGT GTGGGTTATT AAGGCGATTA 50
AAGCACCAAA AGTGCCGAAA TATCAGCGTT ATGACCGCTG GCGTTACTAA 100
<210> 4736
<211> 100
<212> DNA
<213> Escherichia coli K-12 MG1655
<220> yjbo b4051
<222> 230-329
<223> yjbo b4051 U00096 4260209..4260661
<313> 4736: 261-280
<400> 4736
TGATTGGCTT TTTTGTCATG CTGATGGTCA CCGGCGTTTC GTTGCTGGGC 50
ATTATCGCCG CGCTGGTTGT GGCGACGGCC ATTATGTTCC TCGGCGGTAT 100
<210> 4737
<211> 100
<212> DNA
<213> Escherichia coli K-12 MG1655
<220> yjbo b4051
<222> 184-283
<223> yjbo b4051 U00096 4260209..4260661
<313> 4737: 218-237
<400> 4737
AAGAGATTTC ATATTTGGGA GAGCATCATG CTGGAACTAC TTTTTGTGAT 50
TGGCTTTTTT GTCATGCTGA TGGTCACCGG CGTTTCGTTG CTGGGCATTA 100
<210> 4738
<211> 100
<212> DNA

<213> Escherichia coli K-12 MG1655
<220> yjbq b4057
<222> 316-415
<223> yjbq b4057 U00096 4267817..4268233
<313> 4738: 378-397
<400> 4738
AAAGGGCGTA TTCAGACCGG CACCTGGCAA GGCATTTGGC TGGGGGAACA 50
TCGCATCCAC GGCGGATCGC GTCGCATCAT CGCGACACTA CAAGGGGAGT 100
<210> 4739
<211> 100
<212> DNA
<213> Escherichia coli K-12 MG1655
<220> yjbq b4057
<222> 297-396
<223> yjbq b4057 U00096 4267817..4268233
<313> 4739: 329-348
<400> 4739
GCTTGTATTG CCGGTGCATA AAGGGCGTAT TCAGACCGGC ACCTGGCAAG 50
GCATTTGGCT GGGGGAACAT CGCATCCACG GCGGATCGCG TCGCATCATC 100
<210> 4740
<211> 100
<212> DNA
<213> Escherichia coli K-12 MG1655
<220> yjbq b4057
<222> 230-329
<223> yjbq b4057 U00096 4267817..4268233
<313> 4740: 276-295
<400> 4740
ATGAGCATGA CTATGAGGGA GCAGACGATA TGCCTTCTCA TATCAAATCC 50
TCAATGCTGG GAACATCGCT TGTATTGCCG GTGCATAAAG GGCGTATTCA 100
<210> 4741
<211> 100
<212> DNA
<213> Escherichia coli K-12 MG1655
<220> yjbr b4058
<222> 258-357
<223> yjbr b4058 U00096 4268237..4268593
<313> 4741: 323-342
<400> 4741
GCTGCCAGAT TCGCAAATCT ATTATCTGGT GGATGCGTCT TATCAGCAGG 50
CGGTGAATTT ACTGCCGGAA GAAAAACGTA AATTGCTGGT GCAACTCTGA 100
<210> 4742
<211> 100
<212> DNA
<213> Escherichia coli K-12 MG1655
<220> yjbr b4058
<222> 211-310
<223> yjbr b4058 U00096 4268237..4268593
<313> 4742: 251-270
<400> 4742
CGCCATCTGA ATAAAGCGCA CTGGAGCACC GTGTATCTCG ACGGTTCGCT 50
GCCAGATTCG CAAATCTATT ATCTGGTGGA TGCGTCTTAT CAGCAGGCGG 100
<210> 4743
<211> 100
<212> DNA
<213> Escherichia coli K-12 MG1655
<220> yjbr b4058
<222> 157-256
<223> yjbr b4058 U00096 4268237..4268593
<313> 4743: 221-240
<400> 4743
AGCCCGGAGC TGGCGGAGCT GCTACGTCAG CAGCACAGCG ATGTGCGTCC 50
AAGCCGCCAT CTGAATAAAG CGCACTGGAG CACCGTGTAT CTCGACGGTT 100
<210> 4744
<211> 100
<212> DNA
<213> Escherichia coli K-12 MG1655
<220> acs b4070
<222> 1860-1959
<223> acs b4070 U00096 complement(4282992..4284950)
<313> 4744: 1907-1926

<400> 4744
GGGCGATACC AGCAACCTGG GCGATACCTC GACGCTTGCC GATCCTGGCG 50
TAGTCGAGAA GCTGCTTGAA GAGAAGCAGG CTATCGCGAT GCCATCGTAA 100
<210> 4745
<211> 100
<212> DNA
<213> Escherichia coli K-12 MG1655
<220> acs b4070
<222> 1833-1932
<223> acs b4070 U00096 complement(4282992..4284950)
<313> 4745: 1868-1887
<400> 4745
GCGCCGTATT CTGCGCAAAA TTGCGGCGGG CGATACCAGC AACCTGGGCG 50
ATACCTCGAC GCTTGCCGAT CCTGGCGTAG TCGAGAAGCT GCTTGAAGAG 100
<210> 4746
<211> 100
<212> DNA
<213> Escherichia coli K-12 MG1655
<220> acs b4070
<222> 1791-1890
<223> acs b4070 U00096 complement(4282992..4284950)
<313> 4746: 1824-1843
<400> 4746
CTGGACCGAC TCCCTGCCTA AAACCCGCTC CGGCAAAATT ATGCGCCGTA 50
TTCTGCGCAA AATTGCGGCG GGCGATACCA GCAACCTGGG CGATACCTCG 100
<210> 4747
<211> 100
<212> DNA
<213> Escherichia coli K-12 MG1655
<220> ada b2214
<222> 966-1065
<223> ada b2214 U00096 complement(2307361..2308425)
<313> 4747: 1043-1062
<400> 4747
TCGGGTGGTC CGTGGTGATG GCACACTTTC CGGTTACCGC TGGGGCGTGT 50
CGCGTAAAGC GCAACTGCTG CGCCGCGAAG CTGAAAATGA GGAGAGGTAA 100
<210> 4748
<211> 100
<212> DNA
<213> Escherichia coli K-12 MG1655
<220> ada b2214
<222> 854-953
<223> ada b2214 U00096 complement(2307361..2308425)
<313> 4748: 885-904
<400> 4748
GTGAAACCGT CAGTTATCAG CAACTGGCTA ACGCCATCGG CAAACCGAAA 50
GCGGTACGGG CCGTTGCCAG CGCCTGTGCC GCCAACAAGC TGGCTATCAT 100
<210> 4749
<211> 100
<212> DNA
<213> Escherichia coli K-12 MG1655
<220> ada b2214
<222> 793-892
<223> ada b2214 U00096 complement(2307361..2308425)
<313> 4749: 849-868
<400> 4749
GACATTCGCG GCACTGCTTT TCAGCAACAA GTCTGGCAGG CACTGCGCAC 50
GATACCTTGC GGTGAAACCG TCAGTTATCA GCAACTGGCT AACGCCATCG 100
<210> 4750
<211> 100
<212> DNA
<213> Escherichia coli K-12 MG1655
<220> yjcb b4061
<222> 252-351
<223> yjcb b4061 U00096 complement(4272339..4272689)
<313> 4750: 296-315
<400> 4750
CACAATGGCG GACATCACCG CTATCTGGCA CAACATCGAG TCGGTGATGA 50
TAGAAGAGAT GAATCAGACA CCGCCACAAT GGCCAATGAT TTTGACCTGA 100
<210> 4751
<211> 100

<212> DNA
<213> Escherichia coli K-12 MG1655
<220> yjcb b4061
<222> 183-282
<223> yjcb b4061 U00096 complement(4272339..4272689)
<313> 4751: 251-270
<400> 4751
GCTTGCGGTG ATCAGCAGCG GTCTGGGCGT TGTTTCCGCC TGTTGGTTCG 50
CAATGGGGTT GCTTGGCATC ACAATGGCGG ACATCACCGC TATCTGGCAC 100
<210> 4752
<211> 100
<212> DNA
<213> Escherichia coli K-12 MG1655
<220> yjcb b4061
<222> 111-210
<223> yjcb b4061 U00096 complement(4272339..4272689)
<313> 4752: 159-178
<400> 4752
ACTTCTTAGT GCCGTAATGA TGTTTCTGGC CAGCACACTC AACATCCGTT 50
TTCGTCGGTC TGATTATGTC GGGCTTGCGG TGATCAGCAG CGGTCTGGGC 100
<210> 4753
<211> 100
<212> DNA
<213> Escherichia coli K-12 MG1655
<220> yjcc b4062
<222> 1488-1587
<223> yjcc b4062 U00096 4273050..4274636
<313> 4753: 1524-1543
<400> 4753
CTATTGCCAG GGATGGTTCT TTGCGAAGGC GATGCCGCCG CAGGTGTTTA 50
TGCAATGGAT GGAGCAATTA CCCGCGCGGG AGTTAACGCG CGGGCAATAA 100
<210> 4754
<211> 100
<212> DNA
<213> Escherichia coli K-12 MG1655
<220> yjcc b4062
<222> 1350-1449
<223> yjcc b4062 U00096 4273050..4274636
<313> 4754: 1410-1429
<400> 4754
AACGCTGACC ACCCACAAAA CCAGTCATTT GATTGCGGAA CACATCATCG 50
AGCTGGCGCA CAGCCTGGGG TTAAAAACGA TCGCTGAAGG CGTCGAAACT 100
<210> 4755
<211> 100
<212> DNA
<213> Escherichia coli K-12 MG1655
<220> yjcc b4062
<222> 1303-1402
<223> yjcc b4062 U00096 4273050..4274636
<313> 4755: 1364-1383
<400> 4755
AAATCATTGA ATGTCGATAT TTTGAAAATC GACAAATCGT TTGTTGAAAC 50
GCTGACCACC CACAAAACCA GTCATTTGAT TGCGGAACAC ATCATCGAGC 100
<210> 4756
<211> 100
<212> DNA
<213> Escherichia coli K-12 MG1655
<220> add add
<222> 903-1002
<223> add add U00096 1700257..1701258
<313> 4756: 979-998
<400> 4756
CCGCGAGCAA ATCCGCCAGG CACAGATTAA TGGTCTGGAA ATGGCTTTCC 50
TCAGCGCTGA GGAAAAACGC GCACTGCGAG AAAAAGTCGC CGCGAAGTAA 100
<210> 4757
<211> 100
<212> DNA
<213> Escherichia coli K-12 MG1655
<220> add add
<222> 891-990
<223> add add U00096 1700257..1701258

<313> 4757: 937-956
<400> 4757
TGCTGGGTTA TCCCGCGAGC AAATCCGCCA GGCACAGATT AATGGTCTGG 50
AAATGGCTTT CCTCAGCGCT GAGGAAAAAC GCGCACTGCG AGAAAAAGTC 100
<210> 4758
<211> 100
<212> DNA
<213> Escherichia coli K-12 MG1655
<220> add add
<222> 812-911
<223> add add U00096 1700257..1701258
<313> 4758: 852-871
<400> 4758
TTCGTGCCAG CATTAACACT GACGATCCCG GCGTACAGGG AGTGGATATC 50
ATTCACGAAT ATACCGTTGC CGCGCCAGCT GCTGGGTTAT CCCGCGAGCA 100
<210> 4759
<211> 100
<212> DNA
<213> Escherichia coli K-12 MG1655
<220> yjcd b4065
<222> 1237-1336
<223> yjcd b4065 U00096 4276058..4277407
<313> 4759: 1271-1290
<400> 4759
GCGACTCTGG TGATTGGTCG TCTGGTTTCC GGCGAATGGC GCAAGTTGAA 50
CATCGGTACG GTCGTTATCG CCGTGGCGCT GGTGACCTTC TATGCGGGTG 100
<210> 4760
<211> 100
<212> DNA
<213> Escherichia coli K-12 MG1655
<220> yjcd b4065
<222> 1154-1253
<223> yjcd b4065 U00096 4276058..4277407
<313> 4760: 1193-1212
<400> 4760
TTGTTGATGC GATGGCGGGT CTGGTTACGG CTGTATTCAT CGTGCTGACC 50
TGTAACATCG TAACAGGCAT CATGATCGGC TTCGCGACTC TGGTGATTGG 100
<210> 4761
<211> 100
<212> DNA
<213> Escherichia coli K-12 MG1655
<220> yjcd b4065
<222> 1086-1185
<223> yjcd b4065 U00096 4276058..4277407
<313> 4761: 1139-1158
<400> 4761
GGCTCCGGCG CTGATGTACG TTGGCCTGCT GATGCTGAGC AACGTGGCGA 50
AAATCGACTT TGCTGATTTT GTTGATGCGA TGGCGGGTCT GGTTACGGCT 100
<210> 4762
<211> 100
<212> DNA
<213> Escherichia coli K-12 MG1655
<220> yjce b4066
<222> 1551-1650
<223> yjce b4066 U00096 4277559..4279208
<313> 4762: 1609-1628
<400> 4762
CCACCTGCGC GCCACACGGG AGATCAGCAA CGAAACGCTG CAAAAATTAC 50
TGCACGATCT CGATTTGCTT GAAGCGTTGC TAATTGAGGA AAATCAGTAA 100
<210> 4763
<211> 100
<212> DNA
<213> Escherichia coli K-12 MG1655
<220> yjce b4066
<222> 1454-1553
<223> yjce b4066 U00096 4277559..4279208
<313> 4763: 1498-1517
<400> 4763
CCGATGGACG TAACGACGTT GAAAGTTCCG TGCAGGAAGA GAACCTTGAG 50
CGTCGCTTCC GTCTGGCGGC ATTGCGTTCT GAACGTGCTG AACTTTACCA 100
<210> 4764

<211> 100
<212> DNA
<213> Escherichia coli K-12 MG1655
<220> yjce b4066
<222> 1385-1484
<223> yjce b4066 U00096 4277559..4279208
<313> 4764: 1433-1452
<400> 4764
AAGAGAATAT CGATAACCAG CTGCTTACGG AGGTCAGTTC TCGCGTCATT  50
GGTAACCTGC GTCGTCGCGC CGATGGACGT AACGACGTTG AAAGTTCCGT  100
<210> 4765
<211> 100
<212> DNA
<213> Escherichia coli K-12 MG1655
<220> flxa flxA
<222> 221-320
<223> flxa flxA U00096 1644429..1644761
<313> 4765: 239-258
<400> 4765
TGTTAAGGCA GCAGGCAGAG AAAAAGAATG AAGACGCGAC AGTTCAGCCT  50
GATAAAAAAG AAGAGAAAAA AGACGATACA AATACCGCTG GCACCATTGA  100
<210> 4766
<211> 100
<212> DNA
<213> Escherichia coli K-12 MG1655
<220> flxa flxA
<222> 125-224
<223> flxa flxA U00096 1644429..1644761
<313> 4766: 183-202
<400> 4766
TCTCATCGGA AGAGGGGATG ACGACACAGC AGAAAAAAGA AATGGCTGCA  50
TTGGTACAGA AGCAAATTGA AAGCCTCTGG GCTCAACTGG AGCAGTTGTT  100
<210> 4767
<211> 100
<212> DNA
<213> Escherichia coli K-12 MG1655
<220> flxa flxA
<222> 109-208
<223> flxa flxA U00096 1644429..1644761
<313> 4767: 136-155
<400> 4767
GAAAAGCTTG GGAAAATCTC ATCGGAAGAG GGGATGACGA CACAGCAGAA  50
AAAAGAAATG GCTGCATTGG TACAGAAGCA AATTGAAAGC CTCTGGGCTC  100
<210> 4768
<211> 100
<212> DNA
<213> Escherichia coli K-12 MG1655
<220> yjcf b4067
<222> 1091-1190
<223> yjcf b4067 U00096 complement(4279362..4280654)
<313> 4768: 1145-1164
<400> 4768
TCGATGAGGC TAGCGTCCAG GGAATGGGTA TTACCATTCT CCGTGGTGAG  50
GAAAATATCT CCATTAATAG TGATATCCTG GTAACACTAC AGAAATTCTT  100
<210> 4769
<211> 100
<212> DNA
<213> Escherichia coli K-12 MG1655
<220> yjcf b4067
<222> 1074-1173
<223> yjcf b4067 U00096 complement(4279362..4280654)
<313> 4769: 1130-1149
<400> 4769
TCTCTATCGT GTTAATTTCG ATGAGGCTAG CGTCCAGGGA ATGGGTATTA  50
CCATTCTCCG TGGTGAGGAA AATATCTCCA TTAATAGTGA TATCCTGGTA  100
<210> 4770
<211> 100
<212> DNA
<213> Escherichia coli K-12 MG1655
<220> yjcf b4067
<222> 1014-1113

<223> yjcf b4067 U00096 complement(4279362..4280654)
<313> 4770: 1070-1089
<400> 4770
TTTAAGCAAT TGCCATTTCA ACTGTTCTGT TTTAACAAAA GCCTGGATGT 50
TTAATATCCG TCTCTATCGT GTTAATTTCG ATGAGGCTAG CGTCCAGGGA 100
<210> 4771
<211> 100
<212> DNA
<213> Escherichia coli K-12 MG1655
<220> yjcg b4068
<222> 1493-1592
<223> yjcg b4068 U00096 complement(4280832..4282481)
<313> 4771: 1547-1566
<400> 4771
ACCCGGCGCT GTTCTCTATC ACCGTGGCAT TCCTCGGCAT CTGGTTCTTC 50
TCGGCAACCG ATAACTCAGC GGAAGGCGCG CGTGAGCGTG AACTGTTCCG 100
<210> 4772
<211> 100
<212> DNA
<213> Escherichia coli K-12 MG1655
<220> yjcg b4068
<222> 1426-1525
<223> yjcg b4068 U00096 complement(4280832..4282481)
<313> 4772: 1471-1490
<400> 4772
ATGATCCTCG GCCCGACGAT TTGGGTACAG ATCCTTGGTC ACGAAAAAGC 50
CATCTTCCCG TATGAATACC CGGCGCTGTT CTCTATCACC GTGGCATTCC 100
<210> 4773
<211> 100
<212> DNA
<213> Escherichia coli K-12 MG1655
<220> yjcg b4068
<222> 1407-1506
<223> yjcg b4068 U00096 complement(4280832..4282481)
<313> 4773: 1424-1443
<400> 4773
GATTACCGCA GTAGTACTGA TGATCCTCGG CCCGACGATT TGGGTACAGA 50
TCCTTGGTCA CGAAAAAGCC ATCTTCCCGT ATGAATACCC GGCGCTGTTC 100
<210> 4774
<211> 100
<212> DNA
<213> Escherichia coli K-12 MG1655
<220> yjch b4069
<222> 137-236
<223> yjch b4069 U00096 complement(4282478..4282792)
<313> 4774: 172-191
<400> 4774
TCGCGCCCGG CTGGCTGGGC ACGCCGCTGA ATCCGAACAC CAGCGTCACA 50
CGCGGTATTC CAATTGGTGT TGGAGTGATT GTGATCTCCT TTGTTCTCAC 100
<210> 4775
<211> 100
<212> DNA
<213> Escherichia coli K-12 MG1655
<220> yjch b4069
<222> 96-195
<223> yjch b4069 U00096 complement(4282478..4282792)
<313> 4775: 123-142
<400> 4775
TATGCTGGCA GTTTATATCG GCTTTATTTT ACTGATCGCC TTCGCGCCCG 50
GCTGGCTGGG CACGCCGCTG AATCCGAACA CCAGCGTCAC ACGCGGTATT 100
<210> 4776
<211> 100
<212> DNA
<213> Escherichia coli K-12 MG1655
<220> yjch b4069
<222> 26-125
<223> yjch b4069 U00096 complement(4282478..4282792)
<313> 4776: 76-95
<400> 4776
TAGAAGACAA TGCGCATTTC AGGGAGTTAG TCGAAAAACG GCAACGGTTT 50
GCCACCATCC TGTCGATTAT TATGCTGGCA GTTTATATCG GCTTTATTTT 100

<210> 4777
<211> 100
<212> DNA
<213> Escherichia coli K-12 MG1655
<220> adk b0475
<222> 546-645
<223> adk b0475 U00096 496399..497043
<313> 4777: 626-645
<400> 4777
CTCCAAAGAA GCAGAAGCGG GTAATACCAA ATACGCGAAA GTTGACGGCA 50
CCAAGCCGGT TGCTGAAGTT CGCGCTGATC TGGAAAAAAT CCTCGGCTAA 100
<210> 4778
<211> 100
<212> DNA
<213> Escherichia coli K-12 MG1655
<220> adk b0475
<222> 518-617
<223> adk b0475 U00096 496399..497043
<313> 4778: 578-597
<400> 4778
AGATGACAGC ACCGCTGATC GGCTACTACT CCAAAGAAGC AGAAGCGGGT 50
AATACCAAAT ACGCGAAAGT TGACGGCACC AAGCCGGTTG CTGAAGTTCG 100
<210> 4779
<211> 100
<212> DNA
<213> Escherichia coli K-12 MG1655
<220> adk b0475
<222> 411-510
<223> adk b0475 U00096 496399..497043
<313> 4779: 454-473
<400> 4779
CAATCCGCCG AAAGTAGAAG GCAAAGACGA CGTTACCGGT GAAGAACTGA 50
CTACCCGTAA AGATGATCAG GAAGAGACCG TACGTAAACG TCTGGTTGAA 100
<210> 4780
<211> 100
<212> DNA
<213> Escherichia coli K-12 MG1655
<220> yjco b4079
<222> 591-690
<223> yjco b4079 U00096 complement(4294015..4294704)
<313> 4780: 652-671
<400> 4780
CGAGAAGAAC AAGCAAAAGG CGCTGCACTG GTTGAACCTG AGCTGTATGG 50
AAGGGTTTGA TACCGGGTGT GAAGAGTTTG AAAAATTAAC CAACGGTTAA 100
<210> 4781
<211> 100
<212> DNA
<213> Escherichia coli K-12 MG1655
<220> yjco b4079
<222> 551-650
<223> yjco b4079 U00096 complement(4294015..4294704)
<313> 4781: 602-621
<400> 4781
GGGCGGGAAT GATGTTCTTA AACGGTGAAG AAGGTTTTAT CGAGAAGAAC 50
AAGCAAAAGG CGCTGCACTG GTTGAACCTG AGCTGTATGG AAGGGTTTGA 100
<210> 4782
<211> 100
<212> DNA
<213> Escherichia coli K-12 MG1655
<220> yjco b4079
<222> 536-635
<223> yjco b4079 U00096 complement(4294015..4294704)
<313> 4782: 569-588
<400> 4782
GTTATTCCGA GTACTGGGCG GGAATGATGT TCTTAAACGG TGAAGAAGGT 50
TTTATCGAGA AGAACAAGCA AAAGGCGCTG CACTGGTTGA ACCTGAGCTG 100
<210> 4783
<211> 100
<212> DNA
<213> Escherichia coli K-12 MG1655
<220> yjcp b4081

<222> 1368-1467
<223> yjcp b4081 U00096 complement(4297143..4298609)
<313> 4783: 1425-1444
<400> 4783
ATTACTGATG CTGGACAGCC GCCGGGTGAT CCAAAGCATT CAGTTGATGA 50
AATCGCTGGG CGGCGGGTAT CAGGCAGGTC CCGTCGTCGA GAAAAAATAA 100
<210> 4784
<211> 100
<212> DNA
<213> Escherichia coli K-12 MG1655
<220> yjcp b4081
<222> 1353-1452
<223> yjcp b4081 U00096 complement(4297143..4298609)
<313> 4784: 1384-1403
<400> 4784
TGCCGAAGAG ATGTCATTAC TGATGCTGGA CAGCCGCCGG GTGATCCAAA 50
GCATTCAGTT GATGAAATCG CTGGGCGGCG GGTATCAGGC AGGTCCCGTC 100
<210> 4785
<211> 100
<212> DNA
<213> Escherichia coli K-12 MG1655
<220> yjcp b4081
<222> 1308-1407
<223> yjcp b4081 U00096 complement(4297143..4298609)
<313> 4785: 1332-1351
<400> 4785
CTTAACCAGC CGCTTACAGG CCACCGAAGC CCGGTTGCCA GTGCTTGCCG 50
AAGAGATGTC ATTACTGATG CTGGACAGCC GCCGGGTGAT CCAAAGCATT 100
<210> 4786
<211> 100
<212> DNA
<213> Escherichia coli K-12 MG1655
<220> yjcq b4082
<222> 1812-1911
<223> yjcq b4082 U00096 complement(4298606..4300516)
<313> 4786: 1889-1908
<400> 4786
ACAGGAGCAA CACGTCTGCC AGCTTTTCGC CCGCTTGCCA GACTGGACAG 50
CCCCGGCATT AACGCCCGCC ACGGAACAGG CACAAGGAGC CACGCAATGA 100
<210> 4787
<211> 100
<212> DNA
<213> Escherichia coli K-12 MG1655
<220> yjcq b4082
<222> 1807-1906
<223> yjcq b4082 U00096 complement(4298606..4300516)
<313> 4787: 1851-1870
<400> 4787
TTAAAACAGG AGCAACACGT CTGCCAGCTT TTCGCCCGCT TGCCAGACTG 50
GACAGCCCCG GCATTAACGC CCGCCACGGA ACAGGCACAA GGAGCCACGC 100
<210> 4788
<211> 100
<212> DNA
<213> Escherichia coli K-12 MG1655
<220> yjcq b4082
<222> 1745-1844
<223> yjcq b4082 U00096 complement(4298606..4300516)
<313> 4788: 1796-1815
<400> 4788
GCCGTTCTCC TCAAATAACG CTTGAAGAGA CACCCGCCTC TCAGGCCATC 50
CTGCCCACCT TATTAAAACA GGAGCAACAC GTCTGCCAGC TTTTCGCCCG 100
<210> 4789
<211> 100
<212> DNA
<213> Escherichia coli K-12 MG1655
<220> yjcr b4083
<222> 933-1032
<223> yjcr b4083 U00096 complement(4300657..4301688)
<313> 4789: 998-1017
<400> 4789
CGTTGCCCAG CGTTTTCCGG TCAAAATCAT GGTCGATAAA CCTGACCCGG 50

AAATGTTCCG CATCGGCGCT TCGGCAGTCG CTAATCTTGA GCCGCAATAA 100
<210> 4790
<211> 100
<212> DNA
<213> Escherichia coli K-12 MG1655
<220> yjcr b4083
<222> 845-944
<223> yjcr b4083 U00096 complement(4300657..4301688)
<313> 4790: 898-917
<400> 4790
ATTCGATTGG CTACGGCGTG CTACCGGATG ACGGCGGCCT GGTGCTGGGC 50
GGCCTGCCGA AAGTGTCTCG TTCTATTAAC TGGGTCCGCG TTGCCCAGCG 100
<210> 4791
<211> 100
<212> DNA
<213> Escherichia coli K-12 MG1655
<220> yjcr b4083
<222> 755-854
<223> yjcr b4083 U00096 complement(4300657..4301688)
<313> 4791: 801-820
<400> 4791
GCGAAACCGA TCTGAAAAAT ATTCGCTCAG GTACACCCGC AACGATTCGC 50
CTGATGAGTG ACAGCGGCAA AACCTTCGAG GGTAAAGTGG ATTCGATTGG 100
<210> 4792
<211> 100
<212> DNA
<213> Escherichia coli K-12 MG1655
<220> yjcs b4084
<222> 1899-1998
<223> yjcs b4084 U00096 complement(4302191..4304188)
<313> 4792: 1928-1947
<400> 4792
GAAGAAAGCC AAAATTATTG GCAATGGCGC GAAACTGGAA GAAATTATCG 50
CCTGTCTGGA TAATTTCGAT TTGTGGGTGA ATATCGTAAC CCCAAATTAA 100
<210> 4793
<211> 100
<212> DNA
<213> Escherichia coli K-12 MG1655
<220> yjcs b4084
<222> 1809-1908
<223> yjcs b4084 U00096 complement(4302191..4304188)
<313> 4793: 1856-1875
<400> 4793
ACCGCAAGCC GACGCCTCTT TCTACATCAG CCGTGAAGAT CTGCACGCCG 50
TGCTGACCGG ACAAGCCAAA ATGGCGGATC TGGTAAAAGC GAAGAAAGCC 100
<210> 4794
<211> 100
<212> DNA
<213> Escherichia coli K-12 MG1655
<220> yjcs b4084
<222> 1775-1874
<223> yjcs b4084 U00096 complement(4302191..4304188)
<313> 4794: 1812-1831
<400> 4794
ACGATAGCGT GCTTAACTAC CGGAAAACGC TGCAACCGCA AGCCGACGCC 50
TCTTTCTACA TCAGCCGTGA AGATCTGCAC GCCGTGCTGA CCGGACAAGC 100
<210> 4795
<211> 100
<212> DNA
<213> Escherichia coli K-12 MG1655
<220> b3776 b3777
<222> 177-276
<223> b3776 b3777 U00096 complement(3957627..3957902)
<313> 4795: 239-258
<400> 4795
TTACAGAGGG TATCTTAATC AATGTTTGCC ACATGAGTTG GTTGCAGACA 50
TACAGAGGGC GGTCATCAAG TCAATCAATG CCATATCGCT CATAAATTGA 100
<210> 4796
<211> 100
<212> DNA
<213> Escherichia coli K-12 MG1655

<220> b3776 b3777
<222> 85-184
<223> b3776 b3777 U00096 complement(3957627..3957902)
<313> 4796: 134-153
<400> 4796
AATGACCTGC AATTCTTTGA TCAGCAGATT CGTTGGGCCA AAGCCGACCT 50
GGTACAACAA GTCAGTCGAA ACAGATTAAA CAGAGCAAGG ACTTACAGAG 100
<210> 4797
<211> 100
<212> DNA
<213> Escherichia coli K-12 MG1655
<220> b3776 b3777
<222> 1-100
<223> b3776 b3777 U00096 complement(3957627..3957902)
<313> 4797: 37-56
<400> 4797
ATGCATCGTC GTTCCATTAT CAACCACTCG CGACCAGACA AGTTAAAACG 50
TGGAATGCAT GTTGAAATAG CCAGTAATGT TATCAATGAC CTGCAATTCT 100
<210> 4798
<211> 100
<212> DNA
<213> Escherichia coli K-12 MG1655
<220> yjct b4085
<222> 831-930
<223> yjct b4085 U00096 complement(4304449..4305378)
<313> 4798: 910-929
<400> 4798
CTTTATTGCC GCCTCATCTT CTGACTTTAA TGGCGCTCAG GGTGCAGCAA 50
TATTGGCGCA TCAACGTTTT TTGCCACAGT TCTGTGCTAA AGCCCCATGA 100
<210> 4799
<211> 100
<212> DNA
<213> Escherichia coli K-12 MG1655
<220> yjct b4085
<222> 797-896
<223> yjct b4085 U00096 complement(4304449..4305378)
<313> 4799: 855-874
<400> 4799
ACCTGCGCCG TCCACTGCCG CATCAGGTCG TGCGCTTTAT TGCCGCCTCA 50
TCTTCTGACT TTAATGGCGC TCAGGGTGCA GCAATATTGG CGCATCAACG 100
<210> 4800
<211> 100
<212> DNA
<213> Escherichia coli K-12 MG1655
<220> yjct b4085
<222> 777-876
<223> yjct b4085 U00096 complement(4304449..4305378)
<313> 4800: 818-837
<400> 4800
CGTTGCCATG ACCCAAAAGT ACCTGCGCCG TCCACTGCCG CATCAGGTCG 50
TGCGCTTTAT TGCCGCCTCA TCTTCTGACT TTAATGGCGC TCAGGGTGCA 100
<210> 4801
<211> 100
<212> DNA
<213> Escherichia coli K-12 MG1655
<220> yjcu b4086
<222> 597-696
<223> yjcu b4086 U00096 complement(4305362..4306057)
<313> 4801: 675-694
<400> 4801
GTTTAATCAT GCGGAAAATA TCGACGAAGC ATGGAGAATT ATGACCGCGC 50
AGATTCTGGC TGCAAAAAGC GAGGTACAGC CTCATGCAAA AACAGCATAA 100
<210> 4802
<211> 100
<212> DNA
<213> Escherichia coli K-12 MG1655
<220> yjcu b4086
<222> 574-673
<223> yjcu b4086 U00096 complement(4305362..4306057)
<313> 4802: 629-648
<400> 4802

TTTATCGTCG GCACTTCCGG CCTGTTTAAT CATGCGGAAA ATATCGACGA 50
AGCATGGAGA ATTATGACCG CGCAGATTCT GGCTGCAAAA AGCGAGGTAC 100
<210> 4803
<211> 100
<212> DNA
<213> Escherichia coli K-12 MG1655
<220> yjcu b4086
<222> 549-648
<223> yjcu b4086 U00096 complement(4305362..4306057)
<313> 4803: 577-596
<400> 4803
ACTGATGGCG GCAGGGGCGG ATGTCTTTAT CGTCGGCACT TCCGGCCTGT 50
TTAATCATGC GGAAAATATC GACGAAGCAT GGAGAATTAT GACCGCGCAG 100
<210> 4804
<211> 100
<212> DNA
<213> Escherichia coli K-12 MG1655
<220> yjcv b4087
<222> 853-952
<223> yjcv b4087 U00096 complement(4306068..4307048)
<313> 4804: 876-895
<400> 4804
GGCGGGTTGA TCATCGGCAC CATCAACAAC GGTCTGAATA TTTTGCAGGT 50
ACAAACCTAT TACCAACTGG TGGTGATGGG CGGATTAATT ATCGCGGCTG 100
<210> 4805
<211> 100
<212> DNA
<213> Escherichia coli K-12 MG1655
<220> yjcv b4087
<222> 797-896
<223> yjcv b4087 U00096 complement(4306068..4307048)
<313> 4805: 842-861
<400> 4805
TCATTGGCGG CACCAGTTTC TTCGGCGGCA AGGGGCGCAT TTTCTCTGTG 50
GTGATTGGCG GGTTGATCAT CGGCACCATC AACAACGGTC TGAATATTTT 100
<210> 4806
<211> 100
<212> DNA
<213> Escherichia coli K-12 MG1655
<220> yjcv b4087
<222> 735-834
<223> yjcv b4087 U00096 complement(4306068..4307048)
<313> 4806: 788-807
<400> 4806
ACTCGGTGCC GCAGAACCGC TTGCCGGTAT GGGTTTTGAA ACCTATGCCA 50
TTGCCAGCGC CATCATTGGC GGCACCAGTT TCTTCGGCGG CAAGGGGCGC 100
<210> 4807
<211> 100
<212> DNA
<213> Escherichia coli K-12 MG1655
<220> yjcw b4088
<222> 1434-1533
<223> yjcw b4088 U00096 complement(4307027..4308559)
<313> 4807: 1509-1528
<400> 4807
CATCGCCGTG TTCTGCGAAG GACGACTGAC GCAAATCCTG ACGAATCGCG 50
ATGACATGAG CGAAGAGGAG ATTATGGCAT GGGCTTTACC ACAAGAGTAA 100
<210> 4808
<211> 100
<212> DNA
<213> Escherichia coli K-12 MG1655
<220> yjcw b4088
<222> 1405-1504
<223> yjcw b4088 U00096 complement(4307027..4308559)
<313> 4808: 1453-1472
<400> 4808
CTACCTGAAA TTATCACCGT CTGCGACCGC ATCGCCGTGT TCTGCGAAGG 50
ACGACTGACG CAAATCCTGA CGAATCGCGA TGACATGAGC GAAGAGGAGA 100
<210> 4809
<211> 100
<212> DNA

<213> Escherichia coli K-12 MG1655
<220> yjcw b4088
<222> 1392-1491
<223> yjcw b4088 U00096 complement(4307027..4308559)
<313> 4809: 1414-1433
<400> 4809
GGTGTCATCT GAACTACCTG AAATTATCAC CGTCTGCGAC CGCATCGCCG 50
TGTTCTGCGA AGGACGACTG ACGCAAATCC TGACGAATCG CGATGACATG 100
<210> 4810
<211> 100
<212> DNA
<213> Escherichia coli K-12 MG1655
<220> yjcx b4089
<222> 837-936
<223> yjcx b4089 U00096 complement(4308686..4309621)
<313> 4810: 897-916
<400> 4810
TCTGAAGCTG ATGGTTGACG CTGAGAAATC CGGCAAGGTT ATCCCGCTGG 50
ATAAAGCACC GGAATTTAAA CTGGTCGATT CAATCCTGGT CACTCAATAA 100
<210> 4811
<211> 100
<212> DNA
<213> Escherichia coli K-12 MG1655
<220> yjcx b4089
<222> 815-914
<223> yjcx b4089 U00096 complement(4308686..4309621)
<313> 4811: 854-873
<400> 4811
CGGCGGATAT CGGCGCAACG GGTCTGAAGC TGATGGTTGA CGCTGAGAAA 50
TCCGGCAAGG TTATCCCGCT GGATAAAGCA CCGGAATTTA AACTGGTCGA 100
<210> 4812
<211> 100
<212> DNA
<213> Escherichia coli K-12 MG1655
<220> yjcx b4089
<222> 787-886
<223> yjcx b4089 U00096 complement(4308686..4309621)
<313> 4812: 808-827
<400> 4812
CAAATGACCG CGACGGTTGC CCAGAACCCG GCGGATATCG GCGCAACGGG 50
TCTGAAGCTG ATGGTTGACG CTGAGAAATC CGGCAAGGTT ATCCCGCTGG 100
<210> 4813
<211> 100
<212> DNA
<213> Escherichia coli K-12 MG1655
<220> yjcz b4111
<222> 728-827
<223> yjcz b4111 U00096 4326971..4327816
<313> 4813: 784-803
<400> 4813
TTTGCTGGTC AGTGACCCAA CGCTGGCAAA CTGTCGCGCT GGGGATGCCA 50
CGACTCTGTT CAGCGCAGCG TCTGGCGGGG GCAATGCTCG AGGAAATCTT 100
<210> 4814
<211> 100
<212> DNA
<213> Escherichia coli K-12 MG1655
<220> yjcz b4111
<222> 665-764
<223> yjcz b4111 U00096 4326971..4327816
<313> 4814: 703-722
<400> 4814
GCGGGGAGCA TGCTGATTTT TTAAGTTGGC TGGGAAAGTG GACCACGGCA 50
GATAAACAAC CCGTTTGCTG GTCAGTGACC CAACGCTGGC AAACTGTCGC 100
<210> 4815
<211> 100
<212> DNA
<213> Escherichia coli K-12 MG1655
<220> yjcz b4111
<222> 605-704
<223> yjcz b4111 U00096 4326971..4327816
<313> 4815: 629-648

<400> 4815
CGGCACCGAC CACGCGTCAC TATTACCATC AGTGGTTTGC CTCTTCGACC 50
GTTCCGACGG GCGGGGAGCA TGCTGATTTT TTAAGTTGGC TGGGAAAGTG 100
<210> 4816
<211> 100
<212> DNA
<213> Escherichia coli K-12 MG1655
<220> yjda b4110
<222> 2130-2229
<223> yjda b4110 U00096 4324713..4326941
<313> 4816: 2192-2211
<400> 4816
GCAATTGCAG CAGACTGTGA TGACTCACGG CTGGATTTAC ACCGACGCCC 50
AGCTGTTACG CGATGATATT CAAACACTTT TCACGGCAGA ACGATATTGA 100
<210> 4817
<211> 100
<212> DNA
<213> Escherichia coli K-12 MG1655
<220> yjda b4110
<222> 2123-2222
<223> yjda b4110 U00096 4324713..4326941
<313> 4817: 2155-2174
<400> 4817
TGCAGCAGCA ATTGCAGCAG ACTGTGATGA CTCACGGCTG GATTTACACC 50
GACGCCCAGC TGTTACGCGA TGATATTCAA ACACTTTTCA CGGCAGAACG 100
<210> 4818
<211> 100
<212> DNA
<213> Escherichia coli K-12 MG1655
<220> yjda b4110
<222> 2064-2163
<223> yjda b4110 U00096 4324713..4326941
<313> 4818: 2097-2116
<400> 4818
GACGCGTTTA CGCGAAAGTC TGGCTCTGCG TCAACAAAAT GAATCGGTGG 50
TCAGGCTGAT GCAGCAGCAA TTGCAGCAGA CTGTGATGAC TCACGGCTGG 100
<210> 4819
<211> 100
<212> DNA
<213> Escherichia coli K-12 MG1655
<220> yjdb b4115
<222> 1575-1674
<223> yjdb b4115 U00096 complement(4331525..4333198)
<313> 4819: 1620-1639
<400> 4819
CAATTTATTC TCCACGCTAT TGGGATTAAC TGGCGTTGAG ACGAAGTATT 50
ACCAGGCTGC GGATGATATT CTGCAAACTT GCAGGAGAGT GAGTGAATGA 100
<210> 4820
<211> 100
<212> DNA
<213> Escherichia coli K-12 MG1655
<220> yjdb b4115
<222> 1519-1618
<223> yjdb b4115 U00096 complement(4331525..4333198)
<313> 4820: 1555-1574
<400> 4820
CAGGTTGACC AGAACTGCCT GCAAAAACAG GCGCAAACGC AACACTATTC 50
ACAAGACAAT TTATTCTCCA CGCTATTGGG ATTAACTGGC GTTGAGACGA 100
<210> 4821
<211> 100
<212> DNA
<213> Escherichia coli K-12 MG1655
<220> yjdb b4115
<222> 1470-1569
<223> yjdb b4115 U00096 complement(4331525..4333198)
<313> 4821: 1497-1516
<400> 4821
ACAGGTGCCG ATGCTGCTGT GGCTGTCGGA GGATTATCAA AAACGGTATC 50
AGGTTGACCA GAACTGCCTG CAAAAACAGG CGCAAACGCA ACACTATTCA 100
<210> 4822
<211> 100

<212> DNA
<213> Escherichia coli K-12 MG1655
<220> pfs b0160
<222> 587-686
<223> pfs b0160 U00096 complement(178455..179153)
<313> 4822: 648-667
<400> 4822
CCGACGTGGC CGATCAACAG TCTCATCTTA GCTTCGATGA GTTCCTGGCT 50
GTTGCCGCTA AACAGTCCAG CCTGATGGTT GAGTCACTGG TGCAGAAACT 100
<210> 4823
<211> 100
<212> DNA
<213> Escherichia coli K-12 MG1655
<220> pfs b0160
<222> 543-642
<223> pfs b0160 U00096 complement(178455..179153)
<313> 4823: 589-608
<400> 4823
CTGCCACAAT TTCAACGTCC CGTTTGTTGT CGTACGCGCC ATCTCCGACG 50
TGGCCGATCA ACAGTCTCAT CTTAGCTTCG ATGAGTTCCT GGCTGTTGCC 100
<210> 4824
<211> 100
<212> DNA
<213> Escherichia coli K-12 MG1655
<220> pfs b0160
<222> 494-593
<223> pfs b0160 U00096 complement(178455..179153)
<313> 4824: 547-566
<400> 4824
TCCCACAGGC CATTGCTGTA GAGATGGAAG CGACGGCAAT CGCCCATGTC 50
TGCCACAATT TCAACGTCCC GTTTGTTGTC GTACGCGCCA TCTCCGACGT 100
<210> 4825
<211> 100
<212> DNA
<213> Escherichia coli K-12 MG1655
<220> yjdc b4136
<222> 459-558
<223> yjdc b4136 U00096 complement(4360311..4360910)
<313> 4825: 502-521
<400> 4825
AAAGCAGATG GAACTGGTGC TGGAAGGCTG TTTAAGCCGA ATGCTGGTGA 50
ATCGTAGCCA GGCGGATGTC GACACTGCAC ATCGGCTGGC GGAAGATATC 100
<210> 4826
<211> 100
<212> DNA
<213> Escherichia coli K-12 MG1655
<220> yjdc b4136
<222> 417-516
<223> yjdc b4136 U00096 complement(4360311..4360910)
<313> 4826: 451-470
<400> 4826
ACTGTTAACC ACACTGGAAG TTGACGATCC GGCGATGGTA GCAAAGCAGA 50
TGGAACTGGT GCTGGAAGGC TGTTTAAGCC GAATGCTGGT GAATCGTAGC 100
<210> 4827
<211> 100
<212> DNA
<213> Escherichia coli K-12 MG1655
<220> yjdc b4136
<222> 379-478
<223> yjdc b4136 U00096 complement(4360311..4360910)
<313> 4827: 406-425
<400> 4827
GATCAGCAAA AAAGCGCGGC CTACGATTTC ACCCACGAAC TGTTAACCAC 50
ACTGGAAGTT GACGATCCGG CGATGGTAGC AAAGCAGATG GAACTGGTGC 100
<210> 4828
<211> 100
<212> DNA
<213> Escherichia coli K-12 MG1655
<220> yjde b4116
<222> 1239-1338
<223> yjde b4116 U00096 complement(4333272..4334609)

<313> 4828: 1299-1318
<400> 4828
ATTTGTCACC CTGATGGTCA TCACCGCCAT GTATGCGCTG AATTACAACC 50
GGCTACATAA AAACCCGTAT CCCTTAGATG CACCAATAAG CAAAGATTAA 100
<210> 4829
<211> 100
<212> DNA
<213> Escherichia coli K-12 MG1655
<220> yjde b4116
<222> 1127-1226
<223> yjde b4116 U00096 complement(4333272..4334609)
<313> 4829: 1185-1204
<400> 4829
GACACGGTCA CTTTGGTAAA GCACGCCCGG CATATCTGGC AGTTACTACC 50
ATTGCCTTCC TCTACTGCAT CTGGGCCGTG GTGGGGTCCG GAGCGAAAGA 100
<210> 4830
<211> 100
<212> DNA
<213> Escherichia coli K-12 MG1655
<220> yjde b4116
<222> 1082-1181
<223> yjde b4116 U00096 complement(4333272..4334609)
<313> 4830: 1144-1163
<400> 4830
CACTGGTGCC ATATCTTTAC ACCTGTGCGG CGTTACTGCT GCTCGGACAC 50
GGTCACTTTG GTAAAGCACG CCCGGCATAT CTGGCAGTTA CTACCATTGC 100
<210> 4831
<211> 100
<212> DNA
<213> Escherichia coli K-12 MG1655
<220> yjdf b4122
<222> 531-630
<223> yjdf b4122 U00096 complement(4342507..4343136)
<313> 4831: 611-630
<400> 4831
CGATATGTTC TGCGCGCTGC TTGGCGCATT AACGACGGTG ATATTCCTCG 50
CTCGCTTTCA TTGCCGCCAG CTACGGCGCT TTGGCTTGAT AACCGGATAA 100
<210> 4832
<211> 100
<212> DNA
<213> Escherichia coli K-12 MG1655
<220> yjdf b4122
<222> 531-630
<223> yjdf b4122 U00096 complement(4342507..4343136)
<313> 4832: 565-584
<400> 4832
CGATATGTTC TGCGCGCTGC TTGGCGCATT AACGACGGTG ATATTCCTCG 50
CTCGCTTTCA TTGCCGCCAG CTACGGCGCT TTGGCTTGAT AACCGGATAA 100
<210> 4833
<211> 100
<212> DNA
<213> Escherichia coli K-12 MG1655
<220> yjdf b4122
<222> 477-576
<223> yjdf b4122 U00096 complement(4342507..4343136)
<313> 4833: 514-533
<400> 4833
TCAGGGAGCG GATGATTTTC TCGGCACCCA GGGCGACCAG TGGGACACGC 50
AATCCGATAT GTTCTGCGCG CTGCTTGGCG CATTAACGAC GGTGATATTC 100
<210> 4834
<211> 100
<212> DNA
<213> Escherichia coli K-12 MG1655
<220> yjdg b4125
<222> 614-713
<223> yjdg b4125 U00096 complement(4346893..4347612)
<313> 4834: 676-695
<400> 4834
GCCACATCTT GTTCACCAGT ATCCATTATG GCGTCACGGG GCGTCCAGTT 50
TACCGTTACC GTATTCAGGC AGAACACTAC TCACTACTGA AACAATATTG 100
<210> 4835

<211> 100
<212> DNA
<213> Escherichia coli K-12 MG1655
<220> yjdg b4125
<222> 567-666
<223> yjdg b4125 U00096 complement(4346893..4347612)
<313> 4835: 625-644
<400> 4835
CATTTCGCGT GTTTCCTGCC GTAAATACCT CATCTGGCTG GTCAACTGCC 50
ACATCTTGTT CACCAGTATC CATTATGGCG TCACGGGGCG TCCAGTTTAC 100
<210> 4836
<211> 100
<212> DNA
<213> Escherichia coli K-12 MG1655
<220> yjdg b4125
<222> 538-637
<223> yjdg b4125 U00096 complement(4346893..4347612)
<313> 4836: 575-594
<400> 4836
TCAACCGACG AGCTAGCCAA CGAGGTTAAC ATTTCGCGTG TTTCCTGCCG 50
TAAATACCTC ATCTGGCTGG TCAACTGCCA CATCTTGTTC ACCAGTATCC 100
<210> 4837
<211> 100
<212> DNA
<213> Escherichia coli K-12 MG1655
<220> yjdh b4126
<222> 1533-1632
<223> yjdh b4126 U00096 complement(4347609..4349240)
<313> 4837: 1613-1632
<400> 4837
AGAAAATCTC GGCGGCAGCA TCGCCGTGGA ATCGGAACCC GGGATTTTCA 50
CACAATTTTT TGTCCAGATA CCCTGGGACG GGGAGAGGTC GAACAGATGA 100
<210> 4838
<211> 100
<212> DNA
<213> Escherichia coli K-12 MG1655
<220> yjdh b4126
<222> 1473-1572
<223> yjdh b4126 U00096 complement(4347609..4349240)
<313> 4838: 1528-1547
<400> 4838
AGGTGTCTCG ACAAAAGGAA GCGAGCGAGG CGTCGGTTTA GCACTTGTCA 50
AACAACAGGT AGAAAATCTC GGCGGCAGCA TCGCCGTGGA ATCGGAACCC 100
<210> 4839
<211> 100
<212> DNA
<213> Escherichia coli K-12 MG1655
<220> yjdh b4126
<222> 1410-1509
<223> yjdh b4126 U00096 complement(4347609..4349240)
<313> 4839: 1452-1471
<400> 4839
CTGTGAAGTT AATGATGATG GACCGGGGAT CGCACCCGAT AAAATCGATC 50
ACATTTTTGA CAAAGGTGTC TCGACAAAAG GAAGCGAGCG AGGCGTCGGT 100
<210> 4840
<211> 100
<212> DNA
<213> Escherichia coli K-12 MG1655
<220> yjdi b4127
<222> 126-225
<223> yjdi b4127 U00096 4349421..4349651
<313> 4840: 173-192
<400> 4840
TCTCAAACGA AAGCCGTGGA TCATGCCGGA TGAAGTCGAC GTCGCCACTG 50
TGGTTAAAGT GATTGATACG TGCCCGAGCG GCGCGCTGAA ATACCGTCAT 100
<210> 4841
<211> 100
<212> DNA
<213> Escherichia coli K-12 MG1655
<220> yjdi b4127
<222> 89-188

<223> yjdi b4127 U00096 4349421..4349651
<313> 4841: 126-145
<400> 4841
CTGGCAATTG CGTACGTGGC AACGGCAAGT TATTTAATCT CAAACGAAAG 50
CCGTGGATCA TGCCGGATGA AGTCGACGTC GCCACTGTGG TTAAAGTGAT 100
<210> 4842
<211> 100
<212> DNA
<213> Escherichia coli K-12 MG1655
<220> yjdi b4127
<222> 1-100
<223> yjdi b4127 U00096 4349421..4349651
<313> 4842: 16-35
<400> 4842
ATGGATCAGG CGCTACTGGA CGGGGGTTAT CGCTGTTATA CCGGCGAAAA 50
GATCGATGTC TATTTCAACA CTGCGATATG TCAGCATTCT GGCAATTGCG 100
<210> 4843
<211> 100
<212> DNA
<213> Escherichia coli K-12 MG1655
<220> yjdj b4128
<222> 122-221
<223> yjdj b4128 U00096 4349663..4349935
<313> 4843: 183-202
<400> 4843
ATGAAAGCCT GAAAGGGCAA GGGATTGGTA AACAGCTGGT TGCGAAAGTC 50
GTGGAAAAAA TGCGTCGGGA AAAACGAAAA ATTATCCCAT TATGCCCATT 100
<210> 4844
<211> 100
<212> DNA
<213> Escherichia coli K-12 MG1655
<220> yjdj b4128
<222> 81-180
<223> yjdj b4128 U00096 4349663..4349935
<313> 4844: 121-140
<400> 4844
GACCGGAGAG AATTTAGCGA TTATCGAACA TACCGATGTC GATGAAAGCC 50
TGAAAGGGCA AGGGATTGGT AAACAGCTGG TTGCGAAAGT CGTGGAAAAA 100
<210> 4845
<211> 100
<212> DNA
<213> Escherichia coli K-12 MG1655
<220> yjdj b4128
<222> 35-134
<223> yjdj b4128 U00096 4349663..4349935
<313> 4845: 84-103
<400> 4845
TTAATGACAA ACAAGGCAAG CAAATCGCTG AAATTGTCTT TGTGCCGACC 50
GGAGAGAATT TAGCGATTAT CGAACATACC GATGTCGATG AAAGCCTGAA 100
<210> 4846
<211> 100
<212> DNA
<213> Escherichia coli K-12 MG1655
<220> yjdk b4129
<222> 198-297
<223> yjdk b4129 U00096 4350162..4350458
<313> 4846: 245-264
<400> 4846
CGAAGATTTA ATCAGGCTCA AATGCTTAGA CTTACCGGAT ATTGATTTTG 50
ACCTCAACAT TATGACAGTT GATGACTATT TCCGTCAGTT TTACAAGTAG 100
<210> 4847
<211> 100
<212> DNA
<213> Escherichia coli K-12 MG1655
<220> yjdk b4129
<222> 198-297
<223> yjdk b4129 U00096 4350162..4350458
<313> 4847: 242-261
<400> 4847
CGAAGATTTA ATCAGGCTCA AATGCTTAGA CTTACCGGAT ATTGATTTTG 50
ACCTCAACAT TATGACAGTT GATGACTATT TCCGTCAGTT TTACAAGTAG 100

<210> 4848
<211> 100
<212> DNA
<213> Escherichia coli K-12 MG1655
<220> yjdk b4129
<222> 198-297
<223> yjdk b4129 U00096 4350162..4350458
<313> 4848: 236-255
<400> 4848
CGAAGATTTA ATCAGGCTCA AATGCTTAGA CTTACCGGAT ATTGATTTTG 50
ACCTCAACAT TATGACAGTT GATGACTATT TCCGTCAGTT TTACAAGTAG 100
<210> 4849
<211> 100
<212> DNA
<213> Escherichia coli K-12 MG1655
<220> yjdl b4131
<222> 1359-1458
<223> yjdl b4131 U00096 complement(4352532..4353989)
<313> 4849: 1420-1439
<400> 4849
GTGGACGTTG GCTTGTGTCG CGATCATCGT CGTATTGGCC TTTGCTACCC 50
GTTTTCTGTT TAGCACGCCG ACGAATATGA TACAGGAGAG CAACGATTAA 100
<210> 4850
<211> 100
<212> DNA
<213> Escherichia coli K-12 MG1655
<220> yjdl b4131
<222> 1359-1458
<223> yjdl b4131 U00096 complement(4352532..4353989)
<313> 4850: 1389-1408
<400> 4850
GTGGACGTTG GCTTGTGTCG CGATCATCGT CGTATTGGCC TTTGCTACCC 50
GTTTTCTGTT TAGCACGCCG ACGAATATGA TACAGGAGAG CAACGATTAA 100
<210> 4851
<211> 100
<212> DNA
<213> Escherichia coli K-12 MG1655
<220> yjdl b4131
<222> 1274-1373
<223> yjdl b4131 U00096 complement(4352532..4353989)
<313> 4851: 1286-1305
<400> 4851
GCGTCGTGGC ACAGCAGACG ACAGAGTCGC AAATTAGCGG TATGGCAATT 50
GCAGCTTACC AGCGATTCTT TTCTCAGATG GGAGAGTGGA CGTTGGCTTG 100
<210> 4852
<211> 100
<212> DNA
<213> Escherichia coli K-12 MG1655
<220> aer b3073
<222> 1420-1519
<223> aer b3073 U00096 complement(3215197..3216717)
<313> 4852: 1475-1494
<400> 4852
ACCCAGAAAA ATGCCGAGCT GGTGGAAGAG AGTGCGCAGG TGTCGGCGAT 50
GGTGAAACAC CGCGCCAGCC GACTGGAAGA CGCGGTGACG GTACTGCATT 100
<210> 4853
<211> 100
<212> DNA
<213> Escherichia coli K-12 MG1655
<220> aer b3073
<222> 1399-1498
<223> aer b3073 U00096 complement(3215197..3216717)
<313> 4853: 1434-1453
<400> 4853
GTGGATGAGC TTAACCTGAT CACCCAGAAA AATGCCGAGC TGGTGGAAGA 50
GAGTGCGCAG GTGTCGGCGA TGGTGAAACA CCGCGCCAGC CGACTGGAAG 100
<210> 4854
<211> 100
<212> DNA
<213> Escherichia coli K-12 MG1655
<220> aer b3073

<222> 1322-1421
<223> aer b3073 U00096 complement(3215197..3216717)
<313> 4854: 1377-1396
<400> 4854
ACGTCACCCA GTTGATCGCC CAGATTAGCC ATTCAACGCT GGAACAGGCC  50
GATGGGCTTT CCAGCCTGAC CCGTGCAGTG GATGAGCTTA ACCTGATCAC  100
<210> 4855
<211> 100
<212> DNA
<213> Escherichia coli K-12 MG1655
<220> insa_1 b0023
<222> 97-196
<223> insa_1 b0023 U00096 complement(20233..20508)
<313> 4855: 149-168
<400> 4855
TCTCACTGCC GTAAAACATG GCAACTGCAG TTCACTTACA CCGCTTCTCA  50
ACCCGGTACG CACCAGAAAA TCATTGATAT GGCCATGAAT GGCGTTGGAT  100
<210> 4856
<211> 100
<212> DNA
<213> Escherichia coli K-12 MG1655
<220> insa_1 b0023
<222> 52-151
<223> insa_1 b0023 U00096 complement(20233..20508)
<313> 4856: 104-123
<400> 4856
GTGCGTAACG GCAAAAGCAC CGCCGGACAT CAGCGCTATC TCTGCTCTCA  50
CTGCCGTAAA ACATGGCAAC TGCAGTTCAC TTACACCGCT TCTCAACCCG  100
<210> 4857
<211> 100
<212> DNA
<213> Escherichia coli K-12 MG1655
<220> insa_1 b0023
<222> 1-100
<223> insa_1 b0023 U00096 complement(20233..20508)
<313> 4857: 36-55
<400> 4857
GTGGCTTCTG TTTCTATCAG CTGTCCCTCC TGTTCAGCTA CTGACGGGGT  50
GGTGCGTAAC GGCAAAAGCA CCGCCGGACA TCAGCGCTAT CTCTGCTCTC  100
<210> 4858
<211> 100
<212> DNA
<213> Escherichia coli K-12 MG1655
<220> insa_2 b0266
<222> 177-276
<223> insa_2 b0266 U00096 complement(278824..279099)
<313> 4858: 250-269
<400> 4858
GGCCATGAAT GGCGTTGGAT GCCGGGCAAC CGCCCGCATT ATGGGCGTTG  50
GCCTCAACAC GATTTTCCGC CATTTAAAAA ACTCAGGCCG CAGTCGGTAA  100
<210> 4859
<211> 100
<212> DNA
<213> Escherichia coli K-12 MG1655
<220> insa_2 b0266
<222> 129-228
<223> insa_2 b0266 U00096 complement(278824..279099)
<313> 4859: 151-170
<400> 4859
CACTTACACC GCTTCTCAAC CCGGTACGCA CCAGAAAATC ATTGATATGG  50
CCATGAATGG CGTTGGATGC CGGGCAACCG CCCGCATTAT GGGCGTTGGC  100
<210> 4860
<211> 100
<212> DNA
<213> Escherichia coli K-12 MG1655
<220> insa_2 b0266
<222> 65-164
<223> insa_2 b0266 U00096 complement(278824..279099)
<313> 4860: 87-106
<400> 4860
AAAGCACTGC CGGACATCAG CGCTATCTCT GCTCTCACTG CCGTAAAACA  50

TGGCAACTGC AGTTCACTTA CACCGCTTCT CAACCCGGTA CGCACCAGAA 100
<210> 4861
<211> 100
<212> DNA
<213> Escherichia coli K-12 MG1655
<220> insa_3 b0276
<222> 177-276
<223> insa_3 b0276 U00096 complement(290295..290570)
<313> 4861: 242-261
<400> 4861
GGCCATGAAT GGCGTTGGAT GCCGGGCAAC CGCCCGCATT ATGGGCGTTG 50
GCCTCAACAC GATTTTCCGC CATTTAAAAA ACTCAGGCCG CAGTCGGTAA 100
<210> 4862
<211> 100
<212> DNA
<213> Escherichia coli K-12 MG1655
<220> insa_3 b0276
<222> 95-194
<223> insa_3 b0276 U00096 complement(290295..290570)
<313> 4862: 143-162
<400> 4862
GCTCTCACTG CCGTAAAACA TGGCAACTGC AGTTCACTTA CACCGCTTCT 50
CAACCCGGTA CGCACCAGAA AATCATTGAT ATGGCCATGA ATGGCGTTGG 100
<210> 4863
<211> 100
<212> DNA
<213> Escherichia coli K-12 MG1655
<220> insa_3 b0276
<222> 69-168
<223> insa_3 b0276 U00096 complement(290295..290570)
<313> 4863: 100-119
<400> 4863
CACTGCCGGA CATCAGCGCT ATCTCTGCTC TCACTGCCGT AAAACATGGC 50
AACTGCAGTT CACTTACACC GCTTCTCAAC CCGGTACGCA CCAGAAAATC 100
<210> 4864
<211> 100
<212> DNA
<213> Escherichia coli K-12 MG1655
<220> insa_5 b1895
<222> 92-191
<223> insa_5 b1895 U00096 complement(1976964..1977239)
<313> 4864: 154-173
<400> 4864
TCTGCTCTCA CTGCCGTAAA ACATGGCAAC TGCAGTTCAC TTACACCGCT 50
TCTCAACCCG GTACGCACCA GAAAATCATT GATATGGCCA TGAATGGCGT 100
<210> 4865
<211> 100
<212> DNA
<213> Escherichia coli K-12 MG1655
<220> insa_5 b1895
<222> 40-139
<223> insa_5 b1895 U00096 complement(1976964..1977239)
<313> 4865: 100-119
<400> 4865
ACTGACGGGG TGGTGCGTAA CGGCAAAAGC ACCGCCGGAC ATCAGCGCTA 50
TCTCTGCTCT CACTGCCGTA AAACATGGCA ACTGCAGTTC ACTTACACCG 100
<210> 4866
<211> 100
<212> DNA
<213> Escherichia coli K-12 MG1655
<220> insa_5 b1895
<222> 20-119
<223> insa_5 b1895 U00096 complement(1976964..1977239)
<313> 4866: 34-53
<400> 4866
GCTGTCCCTC CTGTTCAGCT ACTGACGGGG TGGTGCGTAA CGGCAAAAGC 50
ACCGCCGGAC ATCAGCGCTA TCTCTGCTCT CACTGCCGTA AAACATGGCA 100
<210> 4867
<211> 100
<212> DNA
<213> Escherichia coli K-12 MG1655

<220> insa_6 b3445
<222> 108-207
<223> insa_6 b3445 U00096 3581114..3581389
<313> 4867: 148-167
<400> 4867
TAAAACATGG CAACTGCAGT TCACTTACAC CGCTTCTCAA CCCGGTACGC 50
ACCAGAAAAT CATTGATATG GCCATGAATG GCGTTGGATG CCGGGCAACA 100
<210> 4868
<211> 100
<212> DNA
<213> Escherichia coli K-12 MG1655
<220> insa_6 b3445
<222> 56-155
<223> insa_6 b3445 U00096 3581114..3581389
<313> 4868: 92-111
<400> 4868
GTAACGGCAA AAGCACCGCC GGACATCAGC GCTATCTCTG CTCTCACTGC 50
CGTAAAACAT GGCAACTGCA GTTCACTTAC ACCGCTTCTC AACCCGGTAC 100
<210> 4869
<211> 100
<212> DNA
<213> Escherichia coli K-12 MG1655
<220> insa_6 b3445
<222> 11-110
<223> insa_6 b3445 U00096 3581114..3581389
<313> 4869: 37-56
<400> 4869
TTTCTATCAG CTGTCCCTCC TGTTCAGCTA CTGACGGGGT GGTGCGTAAC 50
GGCAAAAGCA CCGCCGGACA TCAGCGCTAT CTCTGCTCTC ACTGCCGTAA 100
<210> 4870
<211> 100
<212> DNA
<213> Escherichia coli K-12 MG1655
<220> pgi b4026
<222> 1551-1650
<223> pgi b4026 U00096 4231337..4232986
<313> 4870: 1612-1631
<400> 4870
GAACCGTATT CTGCCAGAGC TGAAAGATGA TAAAGAAATC AGCAGCCACG 50
ATAGCTCGAC CAATGGTCTG ATTAACCGCT ATAAAGCGTG GCGCGGTTAA 100
<210> 4871
<211> 100
<212> DNA
<213> Escherichia coli K-12 MG1655
<220> pgi b4026
<222> 1506-1605
<223> pgi b4026 U00096 4231337..4232986
<313> 4871: 1554-1573
<400> 4871
CTTCACCTTC GACCAGTGGG GCGTGGAACT GGGTAAACAG CTGGCGAACC 50
GTATTCTGCC AGAGCTGAAA GATGATAAAG AAATCAGCAG CCACGATAGC 100
<210> 4872
<211> 100
<212> DNA
<213> Escherichia coli K-12 MG1655
<220> pgi b4026
<222> 1469-1568
<223> pgi b4026 U00096 4231337..4232986
<313> 4872: 1521-1540
<400> 4872
AGCACAAAAT CTTTACTCAG GGCGTGATCC TGAACATCTT CACCTTCGAC 50
CAGTGGGGCG TGGAACTGGG TAAACAGCTG GCGAACCGTA TTCTGCCAGA 100
<210> 4873
<211> 100
<212> DNA
<213> Escherichia coli K-12 MG1655
<220> insa_7 b4295
<222> 177-276
<223> insa_7 b4295 U00096 4516095..4516370
<313> 4873: 248-267
<400> 4873

GGCCATGAAT GGCGTCGGAT GTCGCGCCAG TGCACGCATT ATGGGCGTTG 50
GCCTCAACAC GGTTTTACGT CACTTAAAAA ACTCAGGCCG CAGTCGGTAA 100
<210> 4874
<211> 100
<212> DNA
<213> Escherichia coli K-12 MG1655
<220> insa_7 b4295
<222> 91-190
<223> insa_7 b4295 U00096 4516095..4516370
<313> 4874: 144-163
<400> 4874
CTCTGCTCTC CTTGCCGTAA AACATGGCAA CTACAGTTCA CTTACACCGC 50
CTCTCAGCCC GGTAAGCACC AGAAAATCAT TGATATGGCC ATGAATGGCG 100
<210> 4875
<211> 100
<212> DNA
<213> Escherichia coli K-12 MG1655
<220> insa_7 b4295
<222> 3-102
<223> insa_7 b4295 U00096 4516095..4516370
<313> 4875: 40-59
<400> 4875
GGCTTCCATT TCCATCAGAT GTCCTTCCTG CTCCGCTACT GAAGGCGTGG 50
TGCGTAACGG CAAAAGCACT GCCGGACATC AGCGCTATCT CTGCTCTCCT 100
<210> 4876
<211> 100
<212> DNA
<213> Escherichia coli K-12 MG1655
<220> pgk b2927
<222> 1048-1147
<223> pgk b2927 U00096 complement(3069479..3070642)
<313> 4876: 1073-1092
<400> 4876
TTCGGCATTG CTGACAAAAT CTCCTACATC TCCACTGGCG GCGGCGCATT 50
CCTCGAATTC GTGGAAGGTA AAGTACTGCC TGCAGTAGCG ATGCTCGAAG 100
<210> 4877
<211> 100
<212> DNA
<213> Escherichia coli K-12 MG1655
<220> pgk b2927
<222> 988-1087
<223> pgk b2927 U00096 complement(3069479..3070642)
<313> 4877: 1026-1045
<400> 4877
GCAGACAGCG AAGCGTTCTC CATCGCTGGC GGCGGCGACA CTCTGGCAGC 50
AATCGACCTG TTCGGCATTG CTGACAAAAT CTCCTACATC TCCACTGGCG 100
<210> 4878
<211> 100
<212> DNA
<213> Escherichia coli K-12 MG1655
<220> pgk b2927
<222> 940-1039
<223> pgk b2927 U00096 complement(3069479..3070642)
<313> 4878: 973-992
<400> 4878
GAATTCCCGA ACTTCCGCAA AGGTACTGAA ATCGTGGCTA ACGCTATCGC 50
AGACAGCGAA GCGTTCTCCA TCGCTGGCGG CGGCGACACT CTGGCAGCAA 100
<210> 4879
<211> 100
<212> DNA
<213> Escherichia coli K-12 MG1655
<220> pgm b0689
<222> 1535-1634
<223> pgm b0689 U00096 712781..714421
<313> 4879: 1595-1614
<400> 4879
AAGACGCATA TAAGATCTAC TGCGAAAGCT TCCTCGGTGA AGAACATCGC 50
AAGCAGATTG AGAAAGAAGC GGTTGAGATT GTTAGCGAAG TTCTGAAAAA 100
<210> 4880
<211> 100
<212> DNA

<213> Escherichia coli K-12 MG1655
<220> pgm b0689
<222> 1522-1621
<223> pgm b0689 U00096 712781..714421
<313> 4880: 1549-1568
<400> 4880
CCGTCAGGCA CGGAAGACGC ATATAAGATC TACTGCGAAA GCTTCCTCGG 50
TGAAGAACAT CGCAAGCAGA TTGAGAAAGA AGCGGTTGAG ATTGTTAGCG 100
<210> 4881
<211> 100
<212> DNA
<213> Escherichia coli K-12 MG1655
<220> pgm b0689
<222> 1450-1549
<223> pgm b0689 U00096 712781..714421
<313> 4881: 1493-1512
<400> 4881
GCTGCTCCGG GCAACGGTGC TTCTATTGGC GGTCTGAAAG TGATGACTGA 50
CAACGGCTGG TTCGCCGCGC GTCCGTCAGG CACGGAAGAC GCATATAAGA 100
<210> 4882
<211> 100
<212> DNA
<213> Escherichia coli K-12 MG1655
<220> yjea b4156
<222> 909-1008
<223> yjea b4156 U00096 4380191..4381198
<313> 4882: 966-985
<400> 4882
CTGTTCCGGC GTGGCATTAG GTGTTGATCG TCTGGTGATG TTGGCGCTGG 50
GCGCGGAGAC ACTGGCTGAA GTCATCGCCT TTAGCGTTGA CCGGGCATAA 100
<210> 4883
<211> 100
<212> DNA
<213> Escherichia coli K-12 MG1655
<220> yjea b4156
<222> 815-914
<223> yjea b4156 U00096 4380191..4381198
<313> 4883: 862-881
<400> 4883
TTGAACAAGA TAACCGTAAG CGCGCGGCGC GCGGTTTGCC GCAGCACCCC 50
ATTGACCAGA ATCTGATTGA AGCCTTGAAA GTCGGTATGC CTGACTGTTC 100
<210> 4884
<211> 100
<212> DNA
<213> Escherichia coli K-12 MG1655
<220> yjea b4156
<222> 760-859
<223> yjea b4156 U00096 4380191..4381198
<313> 4884: 802-821
<400> 4884
GAGCTGGCGA ATGGTTTCCA TGAATTGACG GATGCCCGTG AGCAGCAACA 50
ACGCTTTGAA CAAGATAACC GTAAGCGCGC GGCGCGCGGT TTGCCGCAGC 100
<210> 4885
<211> 100
<212> DNA
<213> Escherichia coli K-12 MG1655
<220> yjeb b4179
<222> 316-415
<223> yjeb b4179 U00096 4403768..4404193
<313> 4885: 373-392
<400> 4885
CAGGCACTTT CTAAGGCCGT GCAAAGTTTT CTTACGGAAC TGGATAACTA 50
CACGCTTGCC GATTTGGTTG AAGAGAATCA ACCGCTTTAT AAATTATTGC 100
<210> 4886
<211> 100
<212> DNA
<213> Escherichia coli K-12 MG1655
<220> yjeb b4179
<222> 260-359
<223> yjeb b4179 U00096 4403768..4404193
<313> 4886: 328-347

<400> 4886
CGCTGGTGAA TTGCAGCAGT GAGTTTTGCC ACATTACACC TGCCTGTAGG 50
TTGAAACAGG CACTTTCTAA GGCCGTGCAA AGTTTTCTTA CGGAACTGGA 100
<210> 4887
<211> 100
<212> DNA
<213> Escherichia coli K-12 MG1655
<220> yjeb b4179
<222> 238-337
<223> yjeb b4179 U00096 4403768..4404193
<313> 4887: 271-290
<400> 4887
GTGCGCGAGC TGGAGCCCTT ATCGCTGGTG AATTGCAGCA GTGAGTTTTG 50
CCACATTACA CCTGCCTGTA GGTTGAAACA GGCACTTTCT AAGGCCGTGC 100
<210> 4888
<211> 100
<212> DNA
<213> Escherichia coli K-12 MG1655
<220> yjee b4169
<222> 341-440
<223> yjee b4169 U00096 4393163..4393624
<313> 4888: 368-387
<400> 4888
CAGGTGTTCT TCCTGACCCG GATGTCGAAA TACACATTGA TTATCAGGCA 50
CAAGGCCGTG AGGCGCGCGT GAGTGCGGTT TCCTCTGCGG GTGAATTGTT 100
<210> 4889
<211> 100
<212> DNA
<213> Escherichia coli K-12 MG1655
<220> yjee b4169
<222> 209-308
<223> yjee b4169 U00096 4393163..4393624
<313> 4889: 251-270
<400> 4889
CGCTCGACAA CTTAATGGTC TATCACTTTG ATTTGTACCG CCTTGCCGAT 50
CCCGAGGAGC TGGAGTTTAT GGGGATCCGC GATTATTTTG CCAACGATGC 100
<210> 4890
<211> 100
<212> DNA
<213> Escherichia coli K-12 MG1655
<220> yjee b4169
<222> 166-265
<223> yjee b4169 U00096 4393163..4393624
<313> 4890: 200-219
<400> 4890
GGTAATGTCA AAAGCCCCAC TTATACGCTG GTCGAACCCT ATACGCTCGA 50
CAACTTAATG GTCTATCACT TTGATTTGTA CCGCCTTGCC GATCCCGAGG 100
<210> 4891
<211> 100
<212> DNA
<213> Escherichia coli K-12 MG1655
<220> yjef b4168
<222> 1449-1548
<223> yjef b4168 U00096 4391644..4393191
<313> 4891: 1484-1503
<400> 4891
CGGGATGCTG GCAACCGATC TCTTTTCCAC GCTACAGCGT ATTGTTAACC 50
CGGAAGTGAC TGATAAAAAC CATGATGAAT CGAGTAATTC CGCTCCCTGA 100
<210> 4892
<211> 100
<212> DNA
<213> Escherichia coli K-12 MG1655
<220> yjef b4168
<222> 1287-1386
<223> yjef b4168 U00096 4391644..4393191
<313> 4892: 1321-1340
<400> 4892
TGCCGGAAAT GCAGGCATGG CGAGCGGCGG CATGGGCGAT GTGCTCTCTG 50
GTATTATTGG CGCATTGCTT GGGCAAAAAC TGTCGCCGTA TGATGCAGCC 100
<210> 4893
<211> 100

<212> DNA
<213> Escherichia coli K-12 MG1655
<220> yjef b4168
<222> 1141-1240
<223> yjef b4168 U00096 4391644..4393191
<313> 4893: 1188-1207
<400> 4893
CGGTTGTTAG GCTGTTCCGT CGCTGAAATT GAAAGTGACC GCTTACATTG 50
CGCCAAACGT CTGGTACAAC GTTATGGCGG CGTAGCGGTG CTGAAAGGTG 100
<210> 4894
<211> 100
<212> DNA
<213> Escherichia coli K-12 MG1655
<220> yjeh b4142
<222> 1158-1257
<223> yjeh b4142 U00096 complement(4366734..4367990)
<313> 4894: 1237-1256
<400> 4894
AATGGTCGGC TGGAAAAGTC TCTATGCGCT GATCATGCTG GCGGGGTTAT 50
GGCTGTTGCT GCCAAAACGA AAAACGCCGG AAAATGGCAT AACCACATAA 100
<210> 4895
<211> 100
<212> DNA
<213> Escherichia coli K-12 MG1655
<220> yjeh b4142
<222> 1086-1185
<223> yjeh b4142 U00096 complement(4366734..4367990)
<313> 4895: 1128-1147
<400> 4895
CTGTAAATTA TTGCAAGGAC GTTATCGACT ACTGGCGGTG GTTGGCGGGC 50
TGTTATGCGT TCTGTTACTG GCAATGGTCG GCTGGAAAAG TCTCTATGCG 100
<210> 4896
<211> 100
<212> DNA
<213> Escherichia coli K-12 MG1655
<220> yjeh b4142
<222> 1033-1132
<223> yjeh b4142 U00096 complement(4366734..4367990)
<313> 4896: 1085-1104
<400> 4896
ATTTATGCCA ATGGCATCTT TATTATGATT TATCTGTTAT GCATGCTGGC 50
AGGCTGTAAA TTATTGCAAG GACGTTATCG ACTACTGGCG GTGGTTGGCG 100
<210> 4897
<211> 100
<212> DNA
<213> Escherichia coli K-12 MG1655
<220> yjei b4145
<222> 288-387
<223> yjei b4145 U00096 4370354..4370740
<313> 4897: 335-354
<400> 4897
CGTGATTTAT GGCATCAGTA GCCCGTCGCA GGGAATGTTG TCCAGTTTTG 50
TCCCGACGGA TAGCCAGATT ATCGGTCAGG TTTATAAGTG CCCGAACTGA 100
<210> 4898
<211> 100
<212> DNA
<213> Escherichia coli K-12 MG1655
<220> yjei b4145
<222> 246-345
<223> yjei b4145 U00096 4370354..4370740
<313> 4898: 301-320
<400> 4898
AGCAAACGAT CTGCGCAACC AGGCGGCTGC AATGGGCGGT AACGTGATTT 50
ATGGCATCAG TAGCCCGTCG CAGGGAATGT TGTCCAGTTT TGTCCCGACG 100
<210> 4899
<211> 100
<212> DNA
<213> Escherichia coli K-12 MG1655
<220> yjei b4145
<222> 205-304
<223> yjei b4145 U00096 4370354..4370740

<313> 4899: 222-241
<400> 4899
TCCGGGCAAC ACGGAGAAGA AGGCGGTTCT ATGCGCGGCG CAGCAAACGA  50
TCTGCGCAAC CAGGCGGCTG CAATGGGCGG TAACGTGATT TATGGCATCA  100
<210> 4900
<211> 100
<212> DNA
<213> Escherichia coli K-12 MG1655
<220> yjej b4146
<222> 771-870
<223> yjej b4146 U00096 complement(4370943..4371812)
<313> 4900: 839-858
<400> 4900
GGCGAATAAC GCCCAACCGC TAACCCAGGA TCAATGCTTA CGCGCGCTGC  50
ATCACTTACG TGTTCAATCC ACCAGCAAAA CTGCACCACA GGCGAAATAA  100
<210> 4901
<211> 100
<212> DNA
<213> Escherichia coli K-12 MG1655
<220> yjej b4146
<222> 761-860
<223> yjej b4146 U00096 complement(4370943..4371812)
<313> 4901: 779-798
<400> 4901
GCACCGTAGC GGCGAATAAC GCCCAACCGC TAACCCAGGA TCAATGCTTA  50
CGCGCGCTGC ATCACTTACG TGTTCAATCC ACCAGCAAAA CTGCACCACA  100
<210> 4902
<211> 100
<212> DNA
<213> Escherichia coli K-12 MG1655
<220> yjej b4146
<222> 668-767
<223> yjej b4146 U00096 complement(4370943..4371812)
<313> 4902: 715-734
<400> 4902
CGGGTAAAGA AATAGAAGCG ATCACTCGCC GAATGCTTGA TTTCAGCCCA  50
CGACTGAAAA AACTCGCGGG TGTGCCATGT CAGGTCTATG TTCGCACCGT  100
<210> 4903
<211> 100
<212> DNA
<213> Escherichia coli K-12 MG1655
<220> xerc b3812
<222> 786-885
<223> xerc b3812 U00096 3993900..3994796
<313> 4903: 847-866
<400> 4903
GCTGCTGGGT CATGCCAACC TCTCCACCAC GCAAATCTAT ACTCATCTTG  50
ATTTTCAACA CCTTGCCTCG GTGTACGATG CGGCGCATCC ACGCGCCAAA  100
<210> 4904
<211> 100
<212> DNA
<213> Escherichia coli K-12 MG1655
<220> xerc b3812
<222> 740-839
<223> xerc b3812 U00096 3993900..3994796
<313> 4904: 797-816
<400> 4904
CCACGCATAT GCTGGAGTCG AGCGGCGATC TTCGTGGTGT GCAGGAGCTG  50
CTGGGTCATG CCAACCTCTC CACCACGCAA ATCTATACTC ATCTTGATTT  100
<210> 4905
<211> 100
<212> DNA
<213> Escherichia coli K-12 MG1655
<220> xerc b3812
<222> 714-813
<223> xerc b3812 U00096 3993900..3994796
<313> 4905: 763-782
<400> 4905
TCCGCATAAA TTACGTCACT CGTTCGCCAC GCATATGCTG GAGTCGAGCG  50
GCGATCTTCG TGGTGTGCAG GAGCTGCTGG GTCATGCCAA CCTCTCCACC  100
<210> 4906

<211> 100
<212> DNA
<213> Escherichia coli K-12 MG1655
<220> yjek b4147
<222> 930-1029
<223> yjek b4147 U00096 complement(4372207..4373235)
<313> 4906: 971-990
<400> 4906
GCTGACACTG GTGTCGGGAT ATCTGGTGCC GAAACTGGCG CGAGAAATTG 50
GCGGCGAACC CAGCAAAACG CCGCTGGATC TCCAGCTACG CCAGCAGTAA 100
<210> 4907
<211> 100
<212> DNA
<213> Escherichia coli K-12 MG1655
<220> yjek b4147
<222> 838-937
<223> yjek b4147 U00096 complement(4372207..4373235)
<313> 4907: 902-921
<400> 4907
ATGCCGTATT ACCTGCATGT GCTCGATAAA GTACAGGGCG CGGCGCATTT 50
TATGGTGAGT GATGACGAAG CACGGCAGAT TATGCGTGAG TTGCTGACAC 100
<210> 4908
<211> 100
<212> DNA
<213> Escherichia coli K-12 MG1655
<220> yjek b4147
<222> 762-861
<223> yjek b4147 U00096 complement(4372207..4373235)
<313> 4908: 791-810
<400> 4908
CGTTCTGTTA CGTGATGTGA ACGATAACGC ACAAACGCTG GCAAACCTGA 50
GTAATGCGTT GTTCGATGCC GGCGTAATGC CGTATTACCT GCATGTGCTC 100
<210> 4909
<211> 100
<212> DNA
<213> Escherichia coli K-12 MG1655
<220> xerd b2895
<222> 798-897
<223> xerd b2895 U00096 complement(3036867..3037763)
<313> 4909: 859-878
<400> 4909
GCTACTGGGC CACAGCGATC TCTCCACCAC GCAAATTTAT ACGCATGTCG 50
CTACCGAGCG TCTGCGGCAA CTTCATCAAC AGCATCACCC GCGGGCGTGA 100
<210> 4910
<211> 100
<212> DNA
<213> Escherichia coli K-12 MG1655
<220> xerd b2895
<222> 796-895
<223> xerd b2895 U00096 complement(3036867..3037763)
<313> 4910: 815-834
<400> 4910
ATGCTACTGG GCCACAGCGA TCTCTCCACC ACGCAAATTT ATACGCATGT 50
CGCTACCGAG CGTCTGCGGC AACTTCATCA ACAGCATCAC CCGCGGGCGT 100
<210> 4911
<211> 100
<212> DNA
<213> Escherichia coli K-12 MG1655
<220> xerd b2895
<222> 639-738
<223> xerd b2895 U00096 complement(3036867..3037763)
<313> 4911: 677-696
<400> 4911
GCGTGCGCAG CAGATGACGC GACAGACCTT CTGGCACCGT ATTAAACATT 50
ATGCTGTGCT GGCGGGTATC GACAGCGAAA AGCTGTCACC GCATGTGTTG 100
<210> 4912
<211> 100
<212> DNA
<213> Escherichia coli K-12 MG1655
<220> yjem b4157
<222> 1446-1545

<223> yjem b4157 U00096 4381375..4382919
<313> 4912: 1518-1537
<400> 4912
GATGATTGGC GGCCCTGTCT TCTTCTCGCT GTTAGCGATG GCGATTTACC 50
AGAACTATTG CAGCAGAGTG GCAAAAAATC CGCAGTGGGC GGTGGAATAA 100
<210> 4913
<211> 100
<212> DNA
<213> Escherichia coli K-12 MG1655
<220> yjem b4157
<222> 1420-1519
<223> yjem b4157 U00096 4381375..4382919
<313> 4913: 1441-1460
<400> 4913
GCCGGAGACT GGGACAGCAC ATTGTGGATG ATTGGCGGCC CTGTCTTCTT 50
CTCGCTGTTA GCGATGGCGA TTTACCAGAA CTATTGCAGC AGAGTGGCAA 100
<210> 4914
<211> 100
<212> DNA
<213> Escherichia coli K-12 MG1655
<220> yjem b4157
<222> 1274-1373
<223> yjem b4157 U00096 4381375..4382919
<313> 4914: 1311-1330
<400> 4914
CTTTCCCGTT CTTTAAAGCA CGTCAGGATC TCGACAGACC GTTTGTGATT 50
TTCAAAACGC ATTTGTCGGC AATGATTGCG ACAGTGGTTG TCGTACTGGT 100
<210> 4915
<211> 100
<212> DNA
<213> Escherichia coli K-12 MG1655
<220> yjen b4158
<222> 180-279
<223> yjen b4158 U00096 4382971..4383285
<313> 4915: 222-241
<400> 4915
TAAACGGTGG GGAAACCTGC CGGATATTTA TTGCGCTTAT CTTATCCGAT 50
GTCTGGTTTT TCGCGGGGAA TTAGTGGGAT ACGGCGATTT GTTTCGTATG 100
<210> 4916
<211> 100
<212> DNA
<213> Escherichia coli K-12 MG1655
<220> yjen b4158
<222> 73-172
<223> yjen b4158 U00096 4382971..4383285
<313> 4916: 113-132
<400> 4916
GTAGCGGAAA TAGAGCAGAC GATTCTCTCT TTTGTCGATG CCTGCCACAC 50
CCGTAAAGTG GCGATGGTGG TGGGTAGCAC CATTAACACC CTCAAAGATC 100
<210> 4917
<211> 100
<212> DNA
<213> Escherichia coli K-12 MG1655
<220> yjen b4158
<222> 62-161
<223> yjen b4158 U00096 4382971..4383285
<313> 4917: 84-103
<400> 4917
CTGCCGGGGA TGTAGCGGAA ATAGAGCAGA CGATTCTCTC TTTTGTCGAT 50
GCCTGCCACA CCCGTAAAGT GGCGATGGTG GTGGGTAGCA CCATTAACAC 100
<210> 4918
<211> 100
<212> DNA
<213> Escherichia coli K-12 MG1655
<220> yjeo b4159
<222> 216-315
<223> yjeo b4159 U00096 4383282..4383596
<313> 4918: 258-277
<400> 4918
GTGGCGAAAA AAACGCGGCT GGTTTATGTA CGCCACCGCG CTGGCTATTT 50
TCGGCTACTG GCTGTGGCAG TTTTTTCTGC GCTATCAGTT TTGTTTGTGA 100

<210> 4919
<211> 100
<212> DNA
<213> Escherichia coli K-12 MG1655
<220> yjeo b4159
<222> 161-260
<223> yjeo b4159 U00096 4383282..4383596
<313> 4919: 203-222
<400> 4919
ATGTTGGTGT GATAATGACG TTACCGCTCT TTTTCCCTTT TCTCTGGTTC  50
GCTTTGTGGC GAAAAAAACG CGGCTGGTTT ATGTACGCCA CCGCGCTGGC  100
<210> 4920
<211> 100
<212> DNA
<213> Escherichia coli K-12 MG1655
<220> yjeo b4159
<222> 97-196
<223> yjeo b4159 U00096 4383282..4383596
<313> 4920: 156-175
<400> 4920
GATGGACGCG GGATTAACAA CGTCTGCGAT GTGTTGATGT ATCTCGAAGA  50
AGACGATACC CGTGATGTTG GTGTGATAAT GACGTTACCG CTCTTTTTCC  100
<210> 4921
<211> 100
<212> DNA
<213> Escherichia coli K-12 MG1655
<220> yjep b4160
<222> 3219-3318
<223> yjep b4160 U00096 complement(4383625..4386948)
<313> 4921: 3265-3284
<400> 4921
TATGCCATTC CCGCCCTTCC AGATGCGTCT GGAAAGCCTC AACGGTAAAC  50
AAACGGGGAG AACGCTGACG TCTGCGGGCA AAGGTCGTCA GGCGGGAAGT  100
<210> 4922
<211> 100
<212> DNA
<213> Escherichia coli K-12 MG1655
<220> yjep b4160
<222> 3100-3199
<223> yjep b4160 U00096 complement(4383625..4386948)
<313> 4922: 3151-3170
<400> 4922
CAACAGGGGA TTCAGATTTT CGAGCTGCGT ATTTACGCCG CTGAGATGGG  50
TCACCGTATG CCGCTACGCC ATGAGATCCA CCAGCTGATT CTGGCTGGCT  100
<210> 4923
<211> 100
<212> DNA
<213> Escherichia coli K-12 MG1655
<220> yjep b4160
<222> 3055-3154
<223> yjep b4160 U00096 complement(4383625..4386948)
<313> 4923: 3117-3136
<400> 4923
CTGGTGATCG ACAACCCGGC ACCGGAAGTC TTCCTGGTGG ATCTGCAACA  50
GGGGATTCAG ATTTTCGAGC TGCGTATTTA CGCCGCTGAG ATGGGTCACC  100
<210> 4924
<211> 100
<212> DNA
<213> Escherichia coli K-12 MG1655
<220> yjeq b4162
<222> 858-957
<223> yjeq b4162 U00096 complement(4388035..4389048)
<313> 4924: 906-925
<400> 4924
TCGCGATTGC AAACACGATA CCGATCCGGG CTGCGCTATC CGGGAAGCGG  50
TTGAGGAAGG GAAAATCGCG GAAACCCGTT TCGAAAACTA TCACCGTATT  100
<210> 4925
<211> 100
<212> DNA
<213> Escherichia coli K-12 MG1655
<220> yjeq b4162

<222> 722-821
<223> yjeq b4162 U00096 complement(4388035..4389048)
<313> 4925: 760-779
<400> 4925
GGCTGTATCA CTTCCCGCAC GGCGGTGATG TGATTGACTC CCCAGGAGTG 50
CGTGAGTTCG GCCTCTGGCA CCTGGAGCCG GAACAAATCA CTCAGGGCTT 100
<210> 4926
<211> 100
<212> DNA
<213> Escherichia coli K-12 MG1655
<220> yjeq b4162
<222> 677-776
<223> yjeq b4162 U00096 complement(4388035..4389048)
<313> 4926: 710-729
<400> 4926
TCTCTGACAA CTCGGGGCTC GGTCAGCACA CCACGACCGC CGCTCGGCTG 50
TATCACTTCC CGCACGGCGG TGATGTGATT GACTCCCCAG GAGTGCGTGA 100
<210> 4927
<211> 100
<212> DNA
<213> Escherichia coli K-12 MG1655
<220> yjer b4163
<222> 508-607
<223> yjer b4163 U00096 4389113..4389727
<313> 4927: 552-571
<400> 4927
GAAATTCTGG ATGGTTTTAC CAAGCAGGGG ACGCATCAGG CGATGGATGA 50
TATCCGTGAA TCGGTGGCGG AGCTGGCTTA CTACCGCGAG CATTTTATCA 100
<210> 4928
<211> 100
<212> DNA
<213> Escherichia coli K-12 MG1655
<220> yjer b4163
<222> 467-566
<223> yjer b4163 U00096 4389113..4389727
<313> 4928: 520-539
<400> 4928
ATGTCAGCAC CCTGAAAGAG CTGGCGCGCC GCTGGAAGCC GGAAATTCTG 50
GATGGTTTTA CCAAGCAGGG GACGCATCAG GCGATGGATG ATATCCGTGA 100
<210> 4929
<211> 100
<212> DNA
<213> Escherichia coli K-12 MG1655
<220> yjer b4163
<222> 437-536
<223> yjer b4163 U00096 4389113..4389727
<313> 4929: 457-476
<400> 4929
TGGAAGCCTA CTTCCACTAC CGTTATCTCG ATGTCAGCAC CCTGAAAGAG 50
CTGGCGCGCC GCTGGAAGCC GGAAATTCTG GATGGTTTTA CCAAGCAGGG 100
<210> 4930
<211> 100
<212> DNA
<213> Escherichia coli K-12 MG1655
<220> yjes b4167
<222> 1041-1140
<223> yjes b4167 U00096 complement(4390506..4391645)
<313> 4930: 1104-1123
<400> 4930
GCAAATAGAG AGACGAAATG CGTGCATAGT CGAAGTGCAA TTGCCGAAAA 50
AACAGCGTCT GGTTCGGGTG ATTGAAAAAG GGTTACCGCG TGACGCCTGA 100
<210> 4931
<211> 100
<212> DNA
<213> Escherichia coli K-12 MG1655
<220> yjes b4167
<222> 974-1073
<223> yjes b4167 U00096 complement(4390506..4391645)
<313> 4931: 1002-1021
<400> 4931
CAGCGCTGGA AAGTCGTAAA GGTGAGCACC CACTTCTCGA TGAGCACATA 50

GCGTGGGCGA TTGCGCAGCA AATAGAGAGA CGAAATGCGT GCATAGTCGA 100
<210> 4932
<211> 100
<212> DNA
<213> Escherichia coli K-12 MG1655
<220> yjes b4167
<222> 919-1018
<223> yjes b4167 U00096 complement(4390506..4391645)
<313> 4932: 948-967
<400> 4932
CTGCGTAATA TCGCCGTAGC ATTAGGCAAT GCCCCTTGGG ATGAAACGAT 50
TTTGACAGCG CTGGAAAGTC GTAAAGGTGA GCACCCACTT CTCGATGAGC 100
<210> 4933
<211> 100
<212> DNA
<213> Escherichia coli K-12 MG1655
<220> yjet b4177
<222> 99-198
<223> yjet b4177 U00096 4401964..4402161
<313> 4933: 173-192
<400> 4933
GATGACCAAT TTGCCCGATA ATATTTTACG TCGTTTTGGC GGTGGACTTG 50
TGGTTGCGGG CGTTGTGGTC TACTACATGT TGAGGAAAAC GATTGGCTGA 100
<210> 4934
<211> 100
<212> DNA
<213> Escherichia coli K-12 MG1655
<220> yjet b4177
<222> 71-170
<223> yjet b4177 U00096 4401964..4402161
<313> 4934: 121-140
<400> 4934
CGAAGGCATG GAAGAAGATG ATCTCTGCGA TGACCAATTT GCCCGATAAT 50
ATTTTACGTC GTTTTGGCGG TGGACTTGTG GTTGCGGGCG TTGTGGTCTA 100
<210> 4935
<211> 100
<212> DNA
<213> Escherichia coli K-12 MG1655
<220> yjet b4177
<222> 26-125
<223> yjet b4177 U00096 4401964..4402161
<313> 4935: 86-105
<400> 4935
TTGCCCTGGT TTTGGTACTG GAAGGTTTAG GGCCGATGCT TTACCCGAAG 50
GCATGGAAGA AGATGATCTC TGCGATGACC AATTTGCCCG ATAATATTTT 100
<210> 4936
<211> 100
<212> DNA
<213> Escherichia coli K-12 MG1655
<220> yjfa b4224
<222> 150-249
<223> yjfa b4224 U00096 complement(4445553..4445822)
<313> 4936: 178-197
<400> 4936
GCATCAAGTT CGGCCAGCGT GGCGTTGTCA ATACGATAAA CTTCACCGTG 50
TACCGTTCCG TTCCCCGGAA CTGCGCCTGG ATAGTGGCCC AGGCTATACA 100
<210> 4937
<211> 100
<212> DNA
<213> Escherichia coli K-12 MG1655
<220> yjfa b4224
<222> 38-137
<223> yjfa b4224 U00096 complement(4445553..4445822)
<313> 4937: 73-92
<400> 4937
GCTTTCAATT AGCTTTAATC CATCGACGGG TCGTTGATAA ACGTACATCC 50
ATGCACTCCC GTACGGCGTC TGAATCAACT GGCGCGCGTA TTCACCGCCC 100
<210> 4938
<211> 100
<212> DNA
<213> Escherichia coli K-12 MG1655

<220> yjfa b4224
<222> 9-108
<223> yjfa b4224 U00096 complement(4445553..4445822)
<313> 4938: 34-53
<400> 4938
GGTTACTTAT CCCTGTCTAA CCAGTCGCCG CTTTCAATTA GCTTTAATCC  50
ATCGACGGGT CGTTGATAAA CGTACATCCA TGCACTCCCG TACGGCGTCT  100
<210> 4939
<211> 100
<212> DNA
<213> Escherichia coli K-12 MG1655
<220> yjfc b4187
<222> 1065-1164
<223> yjfc b4187 U00096 4410606..4411769
<313> 4939: 1138-1157
<400> 4939
GATTGTCGAT GATGAAGCGT GCGGAATGGG GATCCGTGAA GATAACACAC  50
TGATCACCAA AGACACCTCA CGTTTCGTTC CGCATTACAT TGCTGGATAA  100
<210> 4940
<211> 100
<212> DNA
<213> Escherichia coli K-12 MG1655
<220> yjfc b4187
<222> 963-1062
<223> yjfc b4187 U00096 4410606..4411769
<313> 4940: 993-1012
<400> 4940
TGTTGACCAC GCTGATGGTG ATTACGCCGA TGAACCGATG ATCTACCAGG  50
CGTTTCAACC TCTGCCGCGG TTTGGCGATA GCTACACACT CATCGGTAGC  100
<210> 4941
<211> 100
<212> DNA
<213> Escherichia coli K-12 MG1655
<220> yjfc b4187
<222> 889-988
<223> yjfc b4187 U00096 4410606..4411769
<313> 4941: 941-960
<400> 4941
GAAAGCTATG TGCGTAAACC AATCTATTCG CGCGAAGGCG GCAACGTCAC  50
CATTTTTGAC GGCAAGAATA ACGTTGTTGA CCACGCTGAT GGTGATTACG  100
<210> 4942
<211> 100
<212> DNA
<213> Escherichia coli K-12 MG1655
<220> yjff b4232
<222> 873-972
<223> yjff b4232 U00096 4451181..4452152
<313> 4942: 934-953
<400> 4942
TTTGTTGTTT ATTTTTATAG CATTACAGCG TGGATTAACG GTGCTGTGGG  50
AGAATCGTCA GAGTTCGCCA GTGACAAGAG TCAACATTGC GCAGCAATAA  100
<210> 4943
<211> 100
<212> DNA
<213> Escherichia coli K-12 MG1655
<220> yjff b4232
<222> 777-876
<223> yjff b4232 U00096 4451181..4452152
<313> 4943: 827-846
<400> 4943
GACGCTTTTT GGCGTGGCGA TTCAGGGACT GATTCAGACT TACATAAACT  50
TTGATGGCAC GCTGAGTTCC TGGTGGACGA AAATCGCCAT CGGTATTTTG  100
<210> 4944
<211> 100
<212> DNA
<213> Escherichia coli K-12 MG1655
<220> yjff b4232
<222> 754-853
<223> yjff b4232 U00096 4451181..4452152
<313> 4944: 794-813
<400> 4944

GGTGGCGTTG GAACGGTATT AGGGACGCTT TTTGGCGTGG CGATTCAGGG 50
ACTGATTCAG ACTTACATAA ACTTTGATGG CACGCTGAGT TCCTGGTGGA 100
<210> 4945
<211> 100
<212> DNA
<213> Escherichia coli K-12 MG1655
<220> yjfg b4234
<222> 1275-1374
<223> yjfg b4234 U00096 4453359..4454732
<313> 4945: 1350-1369
<400> 4945
GCCTGGCGAC CATATTCTGG TGATGAGCAA CGGCGGTTTT GGTGGGATCC 50
ATCAGAAACT GCTGGATGGG CTGGCGAAGA AGGCGGAAGC CGCGCAGTAA 100
<210> 4946
<211> 100
<212> DNA
<213> Escherichia coli K-12 MG1655
<220> yjfg b4234
<222> 1263-1362
<223> yjfg b4234 U00096 4453359..4454732
<313> 4946: 1300-1319
<400> 4946
GAAAACCGCT CAGCCTGGCG ACCATATTCT GGTGATGAGC AACGGCGGTT 50
TTGGTGGGAT CCATCAGAAA CTGCTGGATG GGCTGGCGAA GAAGGCGGAA 100
<210> 4947
<211> 100
<212> DNA
<213> Escherichia coli K-12 MG1655
<220> yjfg b4234
<222> 1207-1306
<223> yjfg b4234 U00096 4453359..4454732
<313> 4947: 1243-1262
<400> 4947
TGCGTTCAGC CTGCACACTG GAGTGGCGAT GTGGATACGC TGGCAGATAT 50
GGTGGTGAAA ACCGCTCAGC CTGGCGACCA TATTCTGGTG ATGAGCAACG 100
<210> 4948
<211> 100
<212> DNA
<213> Escherichia coli K-12 MG1655
<220> yjfh b4181
<222> 551-650
<223> yjfh b4181 U00096 4406853..4407584
<313> 4948: 592-611
<400> 4948
AGAGCAAAAT GACCGGACGC CTGGCGCTGG TGATGGGTGC GGAAGGTGAA 50
GGTATGCGTC GCCTGACTCG TGAACATTGC GATGAGTTGA TCAGCATCCC 100
<210> 4949
<211> 100
<212> DNA
<213> Escherichia coli K-12 MG1655
<220> yjfh b4181
<222> 521-620
<223> yjfh b4181 U00096 4406853..4407584
<313> 4949: 548-567
<400> 4949
CGGCAGGCGA GGCGGATCAT ACACTCTATC AGAGCAAAAT GACCGGACGC 50
CTGGCGCTGG TGATGGGTGC GGAAGGTGAA GGTATGCGTC GCCTGACTCG 100
<210> 4950
<211> 100
<212> DNA
<213> Escherichia coli K-12 MG1655
<220> yjfh b4181
<222> 408-507
<223> yjfh b4181 U00096 4406853..4407584
<313> 4950: 437-456
<400> 4950
TACGGCGAAA AAAGTAGCCT GCGGCGCGGC AGAAAGCGTT CCGCTGATTC 50
GGGTGACTAA CCTTGCGCGC ACCATGCGTA TGTTGCAGGA AGAGAATATC 100
<210> 4951
<211> 100
<212> DNA

<213> Escherichia coli K-12 MG1655
<220> yjfi b4182
<222> 300-399
<223> yjfi b4182 U00096 4407711..4408112
<313> 4951: 348-367
<400> 4951
GTTATCGCTT AAATCTTCTC TTGAAGATAT CCTGCTTGAG ATAACCTCGC 50
TGGTAGATAA CGCATTGGAT CTGGCTGAAA TTACAGAAGA ATATTCACAC 100
<210> 4952
<211> 100
<212> DNA
<213> Escherichia coli K-12 MG1655
<220> yjfi b4182
<222> 303-402
<223> yjfi b4182 U00096 4407711..4408112
<313> 4952: 322-341
<400> 4952
ATCGCTTAAA TCTTCTCTTG AAGATATCCT GCTTGAGATA ACCTCGCTGG 50
TAGATAACGC ATTGGATCTG GCTGAAATTA CAGAAGAATA TTCACACTAA 100
<210> 4953
<211> 100
<212> DNA
<213> Escherichia coli K-12 MG1655
<220> yjfi b4182
<222> 280-379
<223> yjfi b4182 U00096 4407711..4408112
<313> 4953: 328-347
<400> 4953
TATTACATTG TTTTCGGGGC GTTATCGCTT AAATCTTCTC TTGAAGATAT 50
CCTGCTTGAG ATAACCTCGC TGGTAGATAA CGCATTGGAT CTGGCTGAAA 100
<210> 4954
<211> 100
<212> DNA
<213> Escherichia coli K-12 MG1655
<220> wza b2063
<222> 1033-1132
<223> wza b2063 U00096 complement(2134126..2135265)
<313> 4954: 1072-1091
<400> 4954
ACCACCGCCC CGGTTTCCCG CTGGAACCGT CTGATCAATC AGTTGCTGCC 50
AACTATTAGC GGTGTCCGTT ACATGACGGA TACAGCCAGC GACATTCATA 100
<210> 4955
<211> 100
<212> DNA
<213> Escherichia coli K-12 MG1655
<220> wza b2063
<222> 980-1079
<223> wza b2063 U00096 complement(2134126..2135265)
<313> 4955: 1005-1024
<400> 4955
CGCTGGTGAT GGCGACAGAA TTCCGCCTGC AACCTTATGA CGTGGTGTAT 50
GTCACCACCG CCCCGGTTTC CCGCTGGAAC CGTCTGATCA ATCAGTTGCT 100
<210> 4956
<211> 100
<212> DNA
<213> Escherichia coli K-12 MG1655
<220> wza b2063
<222> 893-992
<223> wza b2063 U00096 complement(2134126..2135265)
<313> 4956: 923-942
<400> 4956
TCTTTGTCAT TCGTCCGCTG AAAGGCGAGG GCGGGCGTAA CGGCAAGATT 50
GCCAATATCT ACCAGCTGGA TATGTCCGAT GCCACGTCGC TGGTGATGGC 100
<210> 4957
<211> 100
<212> DNA
<213> Escherichia coli K-12 MG1655
<220> yjfj b4183
<222> 600-699
<223> yjfj b4183 U00096 4408131..4408829
<313> 4957: 670-689

<400> 4957
CGACCTTGAC GAAAAGCTGG CGGAAGCCGG AATTGGCGGT AGCAATAAAA 50
GTAGCGCCCA GGATGTATTA GCAAGACTGC AACGCCAACA GGGCGAGTAA 100
<210> 4958
<211> 100
<212> DNA
<213> Escherichia coli K-12 MG1655
<220> yjfj b4183
<222> 600-699
<223> yjfj b4183 U00096 4408131..4408829
<313> 4958: 640-659
<400> 4958
CGACCTTGAC GAAAAGCTGG CGGAAGCCGG AATTGGCGGT AGCAATAAAA 50
GTAGCGCCCA GGATGTATTA GCAAGACTGC AACGCCAACA GGGCGAGTAA 100
<210> 4959
<211> 100
<212> DNA
<213> Escherichia coli K-12 MG1655
<220> yjfj b4183
<222> 517-616
<223> yjfj b4183 U00096 4408131..4408829
<313> 4959: 574-593
<400> 4959
TTAAAACGCC TGCAAACGCG TCAGGCCGAA CGTCAGGCTC GCCTGGATGC 50
TGCCGCACAG TTGGAGAAAG TCGCAGACGG TCGCGACCTT GACGAAAAGC 100
<210> 4960
<211> 100
<212> DNA
<213> Escherichia coli K-12 MG1655
<220> wzb b2062
<222> 345-444
<223> wzb b2062 U00096 complement(2133677..2134120)
<313> 4960: 420-439
<400> 4960
TCCGTATCGC AAAAGCCGGG AAACGTTTGC AGCGGTGTAC ACATTACTTG 50
AACGGTCTGC CCGCCAGTGG GCGCAGGCAT TGAACGCAGA GCAGGTATAA 100
<210> 4961
<211> 100
<212> DNA
<213> Escherichia coli K-12 MG1655
<220> wzb b2062
<222> 292-391
<223> wzb b2062 U00096 complement(2133677..2134120)
<313> 4961: 333-352
<400> 4961
CGCGGCAAAG TGATGCTGTT TGGTCACTGG GATAACGAAT GTGAAATCCC 50
CGATCCGTAT CGCAAAAGCC GGGAAACGTT TGCAGCGGTG TACACATTAC 100
<210> 4962
<211> 100
<212> DNA
<213> Escherichia coli K-12 MG1655
<220> wzb b2062
<222> 262-361
<223> wzb b2062 U00096 complement(2133677..2134120)
<313> 4962: 301-320
<400> 4962
GAACGCTTAT GCGAGATGGC ACCGGAGATG CGCGGCAAAG TGATGCTGTT 50
TGGTCACTGG GATAACGAAT GTGAAATCCC CGATCCGTAT CGCAAAAGCC 100
<210> 4963
<211> 100
<212> DNA
<213> Escherichia coli K-12 MG1655
<220> yjfk b4184
<222> 531-630
<223> yjfk b4184 U00096 4408880..4409539
<313> 4963: 569-588
<400> 4963
CGAAGATACT TACGAATATT TGCTGCTAAA TGGTGAGGAA TCTTTTAACG 50
ATCTCGGCGA GCCAGAGTGG TTATTTTCGC GCGCGTTGGG TGTCGATATC 100
<210> 4964
<211> 100

<212> DNA
<213> Escherichia coli K-12 MG1655
<220> yjfk b4184
<222> 495-594
<223> yjfk b4184 U00096 4408880..4409539
<313> 4964: 526-545
<400> 4964
CCATAATTTT ACCATGGGCT ACCAACGGCA AGTAACCGAA GATACTTACG 50
AATATTTGCT GCTAAATGGT GAGGAATCTT TTAACGATCT CGGCGAGCCA 100
<210> 4965
<211> 100
<212> DNA
<213> Escherichia coli K-12 MG1655
<220> yjfk b4184
<222> 382-481
<223> yjfk b4184 U00096 4408880..4409539
<313> 4965: 433-452
<400> 4965
AACTGGCAGG AAAAACGCTG GCAGCGATTT TTTAACAGCG AAGAACCGGG 50
AAATATCGAA CCCGTCTACA TGCTGGAAAA AGTAGAAAAT CAAAACCATG 100
<210> 4966
<211> 100
<212> DNA
<213> Escherichia coli K-12 MG1655
<220> yjfl b4185
<222> 300-399
<223> yjfl b4185 U00096 4409557..4409955
<313> 4966: 380-399
<400> 4966
AAGCGAAAAA ATTATTAATC ACAATACCGC AGCAGGAATG TTTATGGGAA 50
CCGCCGCGCT GGCTGGCGGT ATTTTTAACG CAGCTTGTAT GACATGGTAA 100
<210> 4967
<211> 100
<212> DNA
<213> Escherichia coli K-12 MG1655
<220> yjfl b4185
<222> 277-376
<223> yjfl b4185 U00096 4409557..4409955
<313> 4967: 334-353
<400> 4967
GTCAGACTTT ATATGCCCGC ATTAAGCGAA AAAATTATTA ATCACAATAC 50
CGCAGCAGGA ATGTTTATGG GAACCGCCGC GCTGGCTGGC GGTATTTTTA 100
<210> 4968
<211> 100
<212> DNA
<213> Escherichia coli K-12 MG1655
<220> yjfl b4185
<222> 151-250
<223> yjfl b4185 U00096 4409557..4409955
<313> 4968: 188-207
<400> 4968
TTCAGCGGTA CATTGTTGGG TTACGTTATC CCCTTATCCA GTGCGGCAAT 50
CAATGCGGTG AGTATTCCAG ACTATTTCGC CTGGGGCGGG ATTGCACTGG 100
<210> 4969
<211> 100
<212> DNA
<213> Escherichia coli K-12 MG1655
<220> yjfm b4186
<222> 526-625
<223> yjfm b4186 U00096 4409965..4410603
<313> 4969: 579-598
<400> 4969
TCTGGCAAAA AAGAGTCTTA CTCTTCGGGC GGCTTTACCA CCAAAAAAGC 50
GTCTACCGTT TCTCGCGGCG GCTATGGTCG TTCTTCCAGC GCCCGTGGGC 100
<210> 4970
<211> 100
<212> DNA
<213> Escherichia coli K-12 MG1655
<220> yjfm b4186
<222> 498-597
<223> yjfm b4186 U00096 4409965..4410603

<313> 4970: 524-543
<400> 4970
TTCTGGTGAT TACTCCTGGC GCTCCGGTTC TGGCAAAAAA GAGTCTTACT 50
CTTCGGGCGG CTTTACCACC AAAAAAGCGT CTACCGTTTC TCGCGGCGGC 100
<210> 4971
<211> 100
<212> DNA
<213> Escherichia coli K-12 MG1655
<220> yjfm b4186
<222> 410-509
<223> yjfm b4186 U00096 4409965..4410603
<313> 4971: 464-483
<400> 4971
GCCGGGTTAT TCGTAAAGAT CGCGATGAGC CGTTTGTTTA TAACAGCGGC 50
GGTTCCTCTT TTGCTTCGCG CCCAGTCTGG CGCAGCACTT CTGGTGATTA 100
<210> 4972
<211> 100
<212> DNA
<213> Escherichia coli K-12 MG1655
<220> yjfn b4189
<222> 204-303
<223> yjfn b4189 U00096 complement(4413595..4413897)
<313> 4972: 271-290
<400> 4972
AAAGGCCGAT GAACAAGGTG CGTCATGGTA CCGCATTATT ACCATGTACG 50
AAGACCAGCA GCCTGATAAC TGGCGCGTAC AGGCGATACT GTATGCATGA 100
<210> 4973
<211> 100
<212> DNA
<213> Escherichia coli K-12 MG1655
<220> yjfn b4189
<222> 153-252
<223> yjfn b4189 U00096 complement(4413595..4413897)
<313> 4973: 192-211
<400> 4973
AGTTAGCAAT ATTTCAGGGG ATCCGCAGGA CGTGGAACGT ATCGTGGCGT 50
TAAAGGCCGA TGAACAAGGT GCGTCATGGT ACCGCATTAT TACCATGTAC 100
<210> 4974
<211> 100
<212> DNA
<213> Escherichia coli K-12 MG1655
<220> yjfn b4189
<222> 10-109
<223> yjfn b4189 U00096 complement(4413595..4413897)
<313> 4974: 62-81
<400> 4974
CGAAAAATGG AGCTGACGAT GAAACAATTA CTTGCCTCAC CCTCGCTGCA 50
ATTAGTGACT TATCCTGCGA GCGCCACGGC ACAGTCTGCA GAATTCGCCA 100
<210> 4975
<211> 100
<212> DNA
<213> Escherichia coli K-12 MG1655
<220> yjfo b4190
<222> 330-429
<223> yjfo b4190 U00096 complement(4414019..4414447)
<313> 4975: 397-416
<400> 4975
AGCTGTCGCT GCAAAAGCTG ATTATTACGT TGTCGTCATG GTGGACGAAA 50
CCATCGTGAC AGGACAGTGG TATTCACAAG CCATTTTGTA CCGTAAATAA 100
<210> 4976
<211> 100
<212> DNA
<213> Escherichia coli K-12 MG1655
<220> yjfo b4190
<222> 305-404
<223> yjfo b4190 U00096 complement(4414019..4414447)
<313> 4976: 343-362
<400> 4976
ACGCATTAGC AGAAATCAAA GCGAAAGCTG TCGCTGCAAA AGCTGATTAT 50
TACGTTGTCG TCATGGTGGA CGAAACCATC GTGACAGGAC AGTGGTATTC 100
<210> 4977

<211> 100
<212> DNA
<213> Escherichia coli K-12 MG1655
<220> yjfo b4190
<222> 218-317
<223> yjfo b4190 U00096 complement(4414019..4414447)
<313> 4977: 262-281
<400> 4977
ACCATCCGCA AGAGATTCGC CGCGACCAGA CGCAAGGGTT ACAGCGAATA 50
GGTAGCGTAA GCACGATGGT TCGGGGTTCT CCGGATGACG CATTAGCAGA 100
<210> 4978
<211> 100
<212> DNA
<213> Escherichia coli K-12 MG1655
<220> agp b1003
<222> 1139-1238
<223> agp b1003 U00096 1064808..1066049
<313> 4978: 1188-1207
<400> 4978
CTGCGCAGCG TGTGACGCTG GAATTAAGCG GTTGCCCGAT AGACGCTGAT 50
GGTTTCTGCC CGATGGATAA GTTTGATAGC GTGTTGAATG AAGCGGTGAA 100
<210> 4979
<211> 100
<212> DNA
<213> Escherichia coli K-12 MG1655
<220> agp b1003
<222> 957-1056
<223> agp b1003 U00096 1064808..1066049
<313> 4979: 976-995
<400> 4979
AACGGCGCTG GATTTCAAAC CGTATCAGTT GCATGACCAG AACGAACGCA 50
CGCCGATTGG CGGCAAAATC GTTTTCCAGC GTTGGCATGA CAGCAAAGCC 100
<210> 4980
<211> 100
<212> DNA
<213> Escherichia coli K-12 MG1655
<220> agp b1003
<222> 864-963
<223> agp b1003 U00096 1064808..1066049
<313> 4980: 914-933
<400> 4980
CAGTTATATC GACAAAGCTC TGGTCACCGA TCGCACCAGC GCACCGAAAA 50
TTACAGTGTT GGTTGGGCAC GACTCCAACA TTGCCTCTCT GTTAACGGCG 100
<210> 4981
<211> 100
<212> DNA
<213> Escherichia coli K-12 MG1655
<220> yjfp b4191
<222> 651-750
<223> yjfp b4191 U00096 4414530..4415279
<313> 4981: 705-724
<400> 4981
GGATAAACTG CTAACCTGTT CATGGCAGCC AGGCGTGCGT CACCGCATTA 50
CGCCTGAGGC GTTAGATGCT GCCGTGACAT TTTTCCGCCA GCATCTTTAA 100
<210> 4982
<211> 100
<212> DNA
<213> Escherichia coli K-12 MG1655
<220> yjfp b4191
<222> 602-701
<223> yjfp b4191 U00096 4414530..4415279
<313> 4982: 663-682
<400> 4982
CTGCCGACGA ATCACTACGT TTGCAGCAGG CCTTAAGCGA GACGGGACGG 50
GATAAACTGC TAACCTGTTC ATGGCAGCCA GGCGTGCGTC ACCGCATTAC 100
<210> 4983
<211> 100
<212> DNA
<213> Escherichia coli K-12 MG1655
<220> yjfp b4191
<222> 495-594

<223> yjfp b4191 U00096 4414530..4415279
<313> 4983: 543-562
<400> 4983
CAATAACATT GTCGCGCCAC TGGCAGAGTG GGAAGCGACA AACCACCTGG 50
AACAACTTAG TGACAGACCT CTACTGCTGT GGCATGGCCT CGACGATGAC 100
<210> 4984
<211> 100
<212> DNA
<213> Escherichia coli K-12 MG1655
<220> alka b2069
<222> 750-849
<223> alka b2069 U00096 complement(2144714..2145562)
<313> 4984: 817-836
<400> 4984
GGCGCAAATC CGCCGTTATG CCGAGCGCTG GAAGCCCTGG CGTTCTTATG 50
CGCTGTTGCA TATCTGGTAT ACGGAAGGCT GGCAACCAGA CGAAGCATGA 100
<210> 4985
<211> 100
<212> DNA
<213> Escherichia coli K-12 MG1655
<220> alka b2069
<222> 606-705
<223> alka b2069 U00096 complement(2144714..2145562)
<313> 4985: 650-669
<400> 4985
GGAGCAGGCG ATGAAAACGC TGCAAACTTT TCCGGGTATC GGGCGCTGGA 50
CGGCGAATTA TTTTGCTTTG CGTGGCTGGC AGGCGAAAGA TGTTTTCCTG 100
<210> 4986
<211> 100
<212> DNA
<213> Escherichia coli K-12 MG1655
<220> alka b2069
<222> 567-666
<223> alka b2069 U00096 complement(2144714..2145562)
<313> 4986: 610-629
<400> 4986
GCTGGAGGGC ACCTTACCAA TGACAATACC GGGCGATGTG GAGCAGGCGA 50
TGAAAACGCT GCAAACTTTT CCGGGTATCG GGCGCTGGAC GGCGAATTAT 100
<210> 4987
<211> 100
<212> DNA
<213> Escherichia coli K-12 MG1655
<220> yjfq b4192
<222> 657-756
<223> yjfq b4192 U00096 complement(4415276..4416031)
<313> 4987: 737-756
<400> 4987
CCGTGCCGAT CAAATCGATA TGCTTATCAC CGGCAAAAAT GCTAACCCGG 50
AAATCCTGCA ACAACTGGAA GCGCAAGGGG TCAGCATTCT GCGTGTTTAA 100
<210> 4988
<211> 100
<212> DNA
<213> Escherichia coli K-12 MG1655
<220> yjfq b4192
<222> 654-753
<223> yjfq b4192 U00096 complement(4415276..4416031)
<313> 4988: 690-709
<400> 4988
TAGCCGTGCC GATCAAATCG ATATGCTTAT CACCGGCAAA AATGCTAACC 50
CGGAAATCCT GCAACAACTG GAAGCGCAAG GGGTCAGCAT TCTGCGTGTT 100
<210> 4989
<211> 100
<212> DNA
<213> Escherichia coli K-12 MG1655
<220> yjfq b4192
<222> 541-640
<223> yjfq b4192 U00096 complement(4415276..4416031)
<313> 4989: 586-605
<400> 4989
TATAAAACCG ATATGCTGAC AGCAATGGCA GAGCAGAAGA TGCTGAGCGT 50
GGTAGGGAAA CTGGTGGTAC TGGTTGATAG CAGTAAGATT GGCGAACGCG 100

<210> 4990
<211> 100
<212> DNA
<213> Escherichia coli K-12 MG1655
<220> alkb b2213
<222> 552-651
<223> alkb b2213 U00096 complement(2306711..2307361)
<313> 4990: 609-628
<400> 4990
GTTTTATCAC GGTATTCAAC CGTTGAAAGC GGGGTTTCAT CCACTCACCA 50
TCGACTGCCG CTACAACCTG ACATTCCGTC AGGCAGGTAA AAAAGAATAA 100
<210> 4991
<211> 100
<212> DNA
<213> Escherichia coli K-12 MG1655
<220> alkb b2213
<222> 514-613
<223> alkb b2213 U00096 complement(2306711..2307361)
<313> 4991: 564-583
<400> 4991
CATGGCGATG TGGTGGTATG GGGCGGTGAA TCGCGGCTGT TTTATCACGG 50
TATTCAACCG TTGAAAGCGG GGTTTCATCC ACTCACCATC GACTGCCGCT 100
<210> 4992
<211> 100
<212> DNA
<213> Escherichia coli K-12 MG1655
<220> alkb b2213
<222> 481-580
<223> alkb b2213 U00096 complement(2306711..2307361)
<313> 4992: 510-529
<400> 4992
CGAAATGATC CGCTCAAACG TTTGTTGTTG GAACATGGCG ATGTGGTGGT 50
ATGGGGCGGT GAATCGCGGC TGTTTTATCA CGGTATTCAA CCGTTGAAAG 100
<210> 4993
<211> 100
<212> DNA
<213> Escherichia coli K-12 MG1655
<220> yjfr b4193
<222> 948-1047
<223> yjfr b4193 U00096 complement(4416139..4417209)
<313> 4993: 991-1010
<400> 4993
CTGGCAGGTG GGTGGCAAAT TTACCTGGCC GCTGGATAAA GACAACTTCG 50
AGTACCACTA TCCGCGCGGT TTCGATGATT GCTTCACTAT TGAACCGGAT 100
<210> 4994
<211> 100
<212> DNA
<213> Escherichia coli K-12 MG1655
<220> yjfr b4193
<222> 863-962
<223> yjfr b4193 U00096 complement(4416139..4417209)
<313> 4994: 904-923
<400> 4994
CAAACTTCCA GGCCGATCCG CAAGAGATCC GCGTGCTGTG GGAGATGAAA 50
AAAGATCGCC TGAAGTATGG CTTCAAGCCG TTTATCTGGC AGGTGGGTGG 100
<210> 4995
<211> 100
<212> DNA
<213> Escherichia coli K-12 MG1655
<220> yjfr b4193
<222> 824-923
<223> yjfr b4193 U00096 complement(4416139..4417209)
<313> 4995: 865-884
<400> 4995
ATGCGAAAGT AGTGATCCCG TTCCACCACG ATATCTGGTC AAACTTCCAG 50
GCCGATCCGC AAGAGATCCG CGTGCTGTGG GAGATGAAAA AAGATCGCCT 100
<210> 4996
<211> 100
<212> DNA
<213> Escherichia coli K-12 MG1655
<220> seca b0099

<222> 2578-2677
<223> seca b0099 U00096 108279..110984
<313> 4996: 2629-2648
<400> 4996
CAGGATGACG ACTCTGCAGC CGCAGCTGCA CTGGCGGCGC AAACCGGAGA 50
GCGCAAAGTA GGACGTAACG ATCCTTGCCC GTGCGGTTCT GGTAAAAAAT 100
<210> 4997
<211> 100
<212> DNA
<213> Escherichia coli K-12 MG1655
<220> seca b0099
<222> 2541-2640
<223> seca b0099 U00096 108279..110984
<313> 4997: 2570-2589
<400> 4997
AGCCGAGCGT TTAGCGCAAA TGCAGCAGCT TAGCCATCAG GATGACGACT 50
CTGCAGCCGC AGCTGCACTG GCGGCGCAAA CCGGAGAGCG CAAAGTAGGA 100
<210> 4998
<211> 100
<212> DNA
<213> Escherichia coli K-12 MG1655
<220> seca b0099
<222> 2476-2575
<223> seca b0099 U00096 108279..110984
<313> 4998: 2514-2533
<400> 4998
CTGAGCAAAG TTCAGGTACG TATGCCTGAA GAGGTTGAGG AGCTGGAACA 50
ACAGCGTCGT ATGGAAGCCG AGCGTTTAGC GCAAATGCAG CAGCTTAGCC 100
<210> 4999
<211> 100
<212> DNA
<213> Escherichia coli K-12 MG1655
<220> dcta b3529
<222> 1188-1287
<223> dcta b3529 U00096 complement(3679791..3681077)
<313> 4999: 1231-1250
<400> 4999
TGTCGTTGCT AAGTGGGTGA AAGAACTGGA CCACAAAAAA CTGGACGATG 50
TGCTGAATAA TCGTGCGCCG GATGGCAAAA CGCACGAATT ATCCTCTTAA 100
<210> 5000
<211> 100
<212> DNA
<213> Escherichia coli K-12 MG1655
<220> dcta b3529
<222> 1151-1250
<223> dcta b3529 U00096 complement(3679791..3681077)
<313> 5000: 1203-1222
<400> 5000
CGCTGACTAA CCTGGTCGGT AACGGCGTAG CGACCATTGT CGTTGCTAAG 50
TGGGTGAAAG AACTGGACCA CAAAAAACTG GACGATGTGC TGAATAATCG 100
<210> 5001
<211> 100
<212> DNA
<213> Escherichia coli K-12 MG1655
<220> dcta b3529
<222> 1076-1175
<223> dcta b3529 U00096 complement(3679791..3681077)
<313> 5001: 1136-1155
<400> 5001
CTGCGGTGGG CCATTTGCCG GTAGCGGGTC TGGCGCTGAT CCTCGGTATC 50
GACCGCTTTA TGTCAGAAGC TCGTGCGCTG ACTAACCTGG TCGGTAACGG 100
<210> 5002
<211> 100
<212> DNA
<213> Escherichia coli K-12 MG1655
<220> secb b3610
<222> 369-468
<223> secb b3610 U00096 complement(3781288..3781755)
<313> 5002: 433-452
<400> 5002
CCCGCAACTG AACCTTGCGC CGGTTAACTT CGATGCGCTG TTCATGAACT 50

ATTTGCAGCA GCAGGCTGGC GAAGGTACTG AAGAACATCA GGATGCCTGA 100
<210> 5003
<211> 100
<212> DNA
<213> Escherichia coli K-12 MG1655
<220> secb b3610
<222> 249-348
<223> secb b3610 U00096 complement(3781288..3781755)
<313> 5003: 292-311
<400> 5003
TTTCTCCATC GCGGGTATCG AAGGCACCCA GATGGCGCAT TGCCTGGGAG 50
CATACTGCCC GAACATTCTG TTCCCGTATG CTCGTGAGTG CATCACCAGC 100
<210> 5004
<211> 100
<212> DNA
<213> Escherichia coli K-12 MG1655
<220> secb b3610
<222> 229-328
<223> secb b3610 U00096 complement(3781288..3781755)
<313> 5004: 252-271
<400> 5004
GAAGTTCAGC AGGGCGGTAT TTTCTCCATC GCGGGTATCG AAGGCACCCA 50
GATGGCGCAT TGCCTGGGAG CATACTGCCC GAACATTCTG TTCCCGTATG 100
<210> 5005
<211> 100
<212> DNA
<213> Escherichia coli K-12 MG1655
<220> moaa b0782
<222> 891-990
<223> moaa b0782 U00096 816267..817256
<313> 5005: 922-941
<400> 5005
AGCGCGTATT TCAGCGGCGC TGCGGGAGAA GAAACAGACC CATTTCCTGC 50
ATCAAAACAA CACCGGTATT ACGCAAAACT TATCGTACAT TGGCGGCTAA 100
<210> 5006
<211> 100
<212> DNA
<213> Escherichia coli K-12 MG1655
<220> moaa b0782
<222> 849-948
<223> moaa b0782 U00096 816267..817256
<313> 5006: 865-884
<400> 5006
GCGCGATCTG CTGGAAGACG ATACCCAGCA ACAGGCGCTG GAAGCGCGTA 50
TTTCAGCGGC GCTGCGGGAG AAGAAACAGA CCCATTTCCT GCATCAAAAC 100
<210> 5007
<211> 100
<212> DNA
<213> Escherichia coli K-12 MG1655
<220> moaa b0782
<222> 777-876
<223> moaa b0782 U00096 816267..817256
<313> 5007: 843-862
<400> 5007
TTGCAACCGC CTGCGCGTTT CCTCCATTGG TAAACTCCAT CTCTGCCTGT 50
TTGGTGAAGG CGGCGTTAAC CTGCGCGATC TGCTGGAAGA CGATACCCAG 100
<210> 5008
<211> 100
<212> DNA
<213> Escherichia coli K-12 MG1655
<220> secd b0409
<222> 1659-1758
<223> secd b0409 U00096 426871..428718
<313> 5008: 1676-1695
<400> 5008
CTTCGATGCG AACATCACCA CGCTGATTAA AGTCATCATC CTGTACGCAG 50
TGGGTACCGG GGCAATTAAA GGGTTCGCGA TTACTACCGG TATCGGTGTG 100
<210> 5009
<211> 100
<212> DNA
<213> Escherichia coli K-12 MG1655

<220> secd b0409
<222> 1610-1709
<223> secd b0409 U00096 426871..428718
<313> 5009: 1635-1654
<400> 5009
CTGTTCAACA GGCAATTGAT GAAGGTTATC GTGGCGCATT CAGTTCTATC 50
TTCGATGCGA ACATCACCAC GCTGATTAAA GTCATCATCC TGTACGCAGT 100
<210> 5010
<211> 100
<212> DNA
<213> Escherichia coli K-12 MG1655
<220> secd b0409
<222> 1524-1623
<223> secd b0409 U00096 426871..428718
<313> 5010: 1558-1577
<400> 5010
GGGTATCGTC TTAACCCTTG CGGTGGCGGT CGATGCGAAC GTACTGATCA 50
ACGAACGTAT TAAAGAAGAG TTGAGCAACG GACGTACTGT TCAACAGGCA 100
<210> 5011
<211> 100
<212> DNA
<213> Escherichia coli K-12 MG1655
<220> moab b0783
<222> 406-505
<223> moab b0783 U00096 817278..817790
<313> 5011: 439-458
<400> 5011
ATGCCGGGTT CGACCAAAGC GTGCCGTACC GCATGGGAAA ATATCATCGC 50
GCCGCAGCTG GATGCCCGTA CGCGTCCGTG TAATTTCCAT CCACATTTGA 100
<210> 5012
<211> 100
<212> DNA
<213> Escherichia coli K-12 MG1655
<220> moab b0783
<222> 365-464
<223> moab b0783 U00096 817278..817790
<313> 5012: 385-404
<400> 5012
GTGCGGTAGC GGGCGTCGCC AACAAAACGC TGATTTTCGC CATGCCGGGT 50
TCGACCAAAG CGTGCCGTAC CGCATGGGAA AATATCATCG CGCCGCAGCT 100
<210> 5013
<211> 100
<212> DNA
<213> Escherichia coli K-12 MG1655
<220> moab b0783
<222> 295-394
<223> moab b0783 U00096 817278..817790
<313> 5013: 320-339
<400> 5013
GTTGAAGGTT TTGGTGAAGT GTTCCGGATG TTGTCGTTTG AAGAGATTGG 50
CACTTCCACG TTGCAATCTC GTGCGGTAGC GGGCGTCGCC AACAAAACGC 100
<210> 5014
<211> 100
<212> DNA
<213> Escherichia coli K-12 MG1655
<220> sece b3982
<222> 285-384
<223> sece b3982 U00096 4174937..4175320
<313> 5014: 361-380
<400> 5014
GATTGTGGCT GCGGTTACCG CAGTAATGTC ACTGATCCTG TGGGGACTGG 50
ATGGTATTCT GGTTCGCCTG GTATCCTTTA TCACTGGCCT GAGGTTCTGA 100
<210> 5015
<211> 100
<212> DNA
<213> Escherichia coli K-12 MG1655
<220> sece b3982
<222> 235-334
<223> sece b3982 U00096 4174937..4175320
<313> 5015: 262-281
<400> 5015

GTCCGTAAGG TCATTTGGCC GACTCGCCAG GAAACATTGC ACACCACGCT 50
GATTGTGGCT GCGGTTACCG CAGTAATGTC ACTGATCCTG TGGGGACTGG 100
<210> 5016
<211> 100
<212> DNA
<213> Escherichia coli K-12 MG1655
<220> sece b3982
<222> 166-265
<223> sece b3982 U00096 4174937..4175320
<313> 5016: 226-245
<400> 5016
GGTGGTGTCG CGCTGTTAAC GACAAAAGGT AAAGCTACCG TTGCTTTTGC 50
CCGTGAAGCG CGTACCGAAG TCCGTAAGGT CATTTGGCCG ACTCGCCAGG 100
<210> 5017
<211> 100
<212> DNA
<213> Escherichia coli K-12 MG1655
<220> moac b0784
<222> 349-448
<223> moac b0784 U00096 817793..818278
<313> 5017: 410-429
<400> 5017
ACCGCGGCCT CCGTGGCGGC GCTGACCATT TATGACATGT GCAAAGCGGT 50
GCAAAAAGAT ATGGTGATTG GTCCGGTACG TTTGCTGGCG AAGAGCGGCG 100
<210> 5018
<211> 100
<212> DNA
<213> Escherichia coli K-12 MG1655
<220> moac b0784
<222> 265-364
<223> moac b0784 U00096 817793..818278
<313> 5018: 298-317
<400> 5018
CAGGCCGAGC CGGAGCACAA TCGGGTGCGT ATAGAAACCT TATGCCGCCT 50
GACCGGGAAA ACCGGTGTCG AAATGGAAGC ATTAACCGCG GCCTCCGTGG 100
<210> 5019
<211> 100
<212> DNA
<213> Escherichia coli K-12 MG1655
<220> moac b0784
<222> 199-298
<223> moac b0784 U00096 817793..818278
<313> 5019: 266-285
<400> 5019
AAACGCACCT GGGATCTGAT CCCGCTCTGT CATCCGCTGA TGCTCAGCAA 50
AGTTGAAGTC AATTTACAGG CCGAGCCGGA GCACAATCGG GTGCGTATAG 100
<210> 5020
<211> 100
<212> DNA
<213> Escherichia coli K-12 MG1655
<220> secf b0410
<222> 873-972
<223> secf b0410 U00096 428729..429700
<313> 5020: 945-964
<400> 5020
GGCATCTGCG TTGGCTCTGA AACTGGGTAT GAAGCGCGAA CACATGTTGC 50
AGCAGAAAGT GGAAAAAGAA GGGGCGGATC AGCCGTCAAT TCTGCCGTAA 100
<210> 5021
<211> 100
<212> DNA
<213> Escherichia coli K-12 MG1655
<220> secf b0410
<222> 853-952
<223> secf b0410 U00096 428729..429700
<313> 5021: 886-905
<400> 5021
ACTGCATCTT CCATCTATGT GGCATCTGCG TTGGCTCTGA AACTGGGTAT 50
GAAGCGCGAA CACATGTTGC AGCAGAAAGT GGAAAAAGAA GGGGCGGATC 100
<210> 5022
<211> 100
<212> DNA

<213> Escherichia coli K-12 MG1655
<220> secf b0410
<222> 762-861
<223> secf b0410 U00096 428729..429700
<313> 5022: 818-837
<400> 5022
TACCTTGATG GTTATCCTGA TGCTGTACCT CTTCGGTGGT CCGGTACTGG 50
AAGGCTTCTC GCTGACCATG CTTATCGGTG TTTCCATCGG TACTGCATCT 100
<210> 5023
<211> 100
<212> DNA
<213> Escherichia coli K-12 MG1655
<220> moad b0785
<222> 147-246
<223> moad b0785 U00096 818271..818516
<313> 5023: 193-212
<400> 5023
CAAATTACTG GCTGCCGTCA ACCAGACGCT GGTGAGTTTT GACCATCCGC 50
TGACTGACGG CGACGAAGTA GCTTTCTTCC CGCCGGTAAC CGGAGGTTAA 100
<210> 5024
<211> 100
<212> DNA
<213> Escherichia coli K-12 MG1655
<220> moad b0785
<222> 50-149
<223> moad b0785 U00096 818271..818516
<313> 5024: 87-106
<400> 5024
ATGCAACCGA AGTGGCTGCG GATTTCCCAA CTGTTGAAGC GTTACGCCAG 50
CACATGGCTG CGCAGAGCGA TCGCTGGGCG CTGGCGCTGG AAGATGGCAA 100
<210> 5025
<211> 100
<212> DNA
<213> Escherichia coli K-12 MG1655
<220> moad b0785
<222> 1-100
<223> moad b0785 U00096 818271..818516
<313> 5025: 46-65
<400> 5025
ATGATTAAAG TTCTTTTTTT CGCCCAGGTG CGCGAGTTGG TGGGAACAGA 50
TGCAACCGAA GTGGCTGCGG ATTTCCCAAC TGTTGAAGCG TTACGCCAGC 100
<210> 5026
<211> 100
<212> DNA
<213> Escherichia coli K-12 MG1655
<220> yjfy b4200
<222> 177-276
<223> yjfy b4200 U00096 complement(4422094..4422369)
<313> 5026: 240-259
<400> 5026
AGAAACAGAT GCGCAAGGCG CAACGTACTA CCACGTAATT CTGATGCGGG 50
AACCGGGGAG TAACGGCAAT ATGCACGCCA GCGCGGATAT TTATCGCTAG 100
<210> 5027
<211> 100
<212> DNA
<213> Escherichia coli K-12 MG1655
<220> yjfy b4200
<222> 157-256
<223> yjfy b4200 U00096 complement(4422094..4422369)
<313> 5027: 200-219
<400> 5027
GCACGTCAGG CATTAAATGA AGAAACAGAT GCGCAAGGCG CAACGTACTA 50
CCACGTAATT CTGATGCGGG AACCGGGGAG TAACGGCAAT ATGCACGCCA 100
<210> 5028
<211> 100
<212> DNA
<213> Escherichia coli K-12 MG1655
<220> yjfy b4200
<222> 111-210
<223> yjfy b4200 U00096 complement(4422094..4422369)
<313> 5028: 158-177

<400> 5028
CTTAGGCGTG ATTTATATCA ATCATAATTT CGCCACTGAA AGCGAAGCAC  50
GTCAGGCATT AAATGAAGAA ACAGATGCGC AAGGCGCAAC GTACTACCAC  100
<210> 5029
<211> 100
<212> DNA
<213> Escherichia coli K-12 MG1655
<220> secg b3176
<222> 234-333
<223> secg b3176 U00096 complement(3319814..3320146)
<313> 5029: 308-327
<400> 5029
CAATAAAGGT AGCGAATGGG AAAATCTGAG TGCACCGGCG AAAACCGAAC  50
AAACTCAGCC AGCTGCTCCG GCTAAGCCGA CCAGCGATAT CCCGAACTAA  100
<210> 5030
<211> 100
<212> DNA
<213> Escherichia coli K-12 MG1655
<220> secg b3176
<222> 93-192
<223> secg b3176 U00096 complement(3319814..3320146)
<313> 5030: 148-167
<400> 5030
AGCCTCCTTC GGAGCAGGCG CTTCCGCTAC GCTGTTTGGT TCAAGTGGTT  50
CTGGTAACTT CATGACCCGC ATGACGGCGC TGCTGGCAAC GTTATTCTTC  100
<210> 5031
<211> 100
<212> DNA
<213> Escherichia coli K-12 MG1655
<220> secg b3176
<222> 1-100
<223> secg b3176 U00096 complement(3319814..3320146)
<313> 5031: 37-56
<400> 5031
ATGTATGAAG CTCTTTTAGT AGTTTTCCTT ATTGTGGCAA TTGGCCTTGT  50
TGGTCTGATC ATGCTGCAGC AAGGTAAAGG CGCTGATATG GGAGCCTCCT  100
<210> 5032
<211> 100
<212> DNA
<213> Escherichia coli K-12 MG1655
<220> moae b0786
<222> 354-453
<223> moae b0786 U00096 818518..818970
<313> 5032: 417-436
<400> 5032
CAAAACCCGC GCACCGTTCT GGAAGCGCGA AGCCACGCCG GAAGGCGACC  50
GCTGGGTTGA AGCTCGGGAG AGCGATCAGC AGGCGGCAAA ACGCTGGTAG  100
<210> 5033
<211> 100
<212> DNA
<213> Escherichia coli K-12 MG1655
<220> moae b0786
<222> 353-452
<223> moae b0786 U00096 818518..818970
<313> 5033: 381-400
<400> 5033
TCAAAACCCG CGCACCGTTC TGGAAGCGCG AAGCCACGCC GGAAGGCGAC  50
CGCTGGGTTG AAGCTCGGGA GAGCGATCAG CAGGCGGCAA AACGCTGGTA  100
<210> 5034
<211> 100
<212> DNA
<213> Escherichia coli K-12 MG1655
<220> moae b0786
<222> 284-383
<223> moae b0786 U00096 818518..818970
<313> 5034: 346-365
<400> 5034
TTTTTGTCGG TGTCACCAGT GCGCATCGCA GCAGTGCGTT TGAAGCCGGG  50
CAGTTTATTA TGGATTATCT CAAAACCCGC GCACCGTTCT GGAAGCGCGA  100
<210> 5035
<211> 100

<212> DNA
<213> Escherichia coli K-12 MG1655
<220> yjfz b4205
<222> 696-795
<223> yjfz b4205 U00096 complement(4424206..4425000)
<313> 5035: 734-753
<400> 5035
GGAGCGAGAC ATCAGTTGCG CTCGTATGTA TCTAACGCCA GCAAACCATC 50
CTGACTTATG GACACAAGAG AATGGTGAAT GGATCTACTT GCGAAACTAA 100
<210> 5036
<211> 100
<212> DNA
<213> Escherichia coli K-12 MG1655
<220> yjfz b4205
<222> 652-751
<223> yjfz b4205 U00096 complement(4424206..4425000)
<313> 5036: 696-715
<400> 5036
TCAGCCGCAA TCTACTCGGG TTTGGGTTTC GAGCGAGTGC CGCAGGAGCG 50
AGACATCAGT TGCGCTCGTA TGTATCTAAC GCCAGCAAAC CATCCTGACT 100
<210> 5037
<211> 100
<212> DNA
<213> Escherichia coli K-12 MG1655
<220> yjfz b4205
<222> 611-710
<223> yjfz b4205 U00096 complement(4424206..4425000)
<313> 5037: 638-657
<400> 5037
TACCCCATGA TTTGAAAATT GGTCTGCGTT CCTTATCAAG TTCAGCCGCA 50
ATCTACTCGG GTTTGGGTTT CGAGCGAGTG CCGCAGGAGC GAGACATCAG 100
<210> 5038
<211> 100
<212> DNA
<213> Escherichia coli K-12 MG1655
<220> b0100 b0101
<222> 1-100
<223> b0100 b0101 U00096 complement(111564..111698)
<313> 5038: 48-67
<400> 5038
GTGGCGATCT TTCCGAAAGC GATGACTGGA GCGAAGAACC AAAGCAGTGA 50
CATTTGTCTG ATGCCGCACG TAGGCCTGAT AAGACGCGGA CAGCGTCGCA 100
<210> 5039
<211> 100
<212> DNA
<213> Escherichia coli K-12 MG1655
<220> b0100 b0101
<222> 1-100
<223> b0100 b0101 U00096 complement(111564..111698)
<313> 5039: 35-54
<400> 5039
GTGGCGATCT TTCCGAAAGC GATGACTGGA GCGAAGAACC AAAGCAGTGA 50
CATTTGTCTG ATGCCGCACG TAGGCCTGAT AAGACGCGGA CAGCGTCGCA 100
<210> 5040
<211> 100
<212> DNA
<213> Escherichia coli K-12 MG1655
<220> b0100 b0101
<222> 1-100
<223> b0100 b0101 U00096 complement(111564..111698)
<313> 5040: 34-53
<400> 5040
GTGGCGATCT TTCCGAAAGC GATGACTGGA GCGAAGAACC AAAGCAGTGA 50
CATTTGTCTG ATGCCGCACG TAGGCCTGAT AAGACGCGGA CAGCGTCGCA 100
<210> 5041
<211> 100
<212> DNA
<213> Escherichia coli K-12 MG1655
<220> pin b1159
<222> 402-501
<223> pin b1159 U00096 1208908..1209462

<313> 5041: 432-451
<400> 5041
ACGAATTGGT GGACGTCGTC CCAAACTTAC ACCAGAACAA TGGGCACAAG 50
CTGGACGATT AATTGCAGCA GGAACTCCTC GCCAGAAGGT GGCGATTATC 100
<210> 5042
<211> 100
<212> DNA
<213> Escherichia coli K-12 MG1655
<220> pin b1159
<222> 319-418
<223> pin b1159 U00096 1208908..1209462
<313> 5042: 358-377
<400> 5042
GTGATGGGTG CCCTGGCTGA AATGGAGCGT GAACTGATTG TTGAACGAAC 50
AAAAGCTGGA CTGGAAACTG CTCGTGCACA GGGACGAATT GGTGGACGTC 100
<210> 5043
<211> 100
<212> DNA
<213> Escherichia coli K-12 MG1655
<220> pin b1159
<222> 260-359
<223> pin b1159 U00096 1208908..1209462
<313> 5043: 287-306
<400> 5043
TTCGTAGTCT GACGGATTCA ATTGATACCA GCACACCAAT GGGACGCTTT 50
TTCTTTCATG TGATGGGTGC CCTGGCTGAA ATGGAGCGTG AACTGATTGT 100
<210> 5044
<211> 100
<212> DNA
<213> Escherichia coli K-12 MG1655
<220> b0105 b0106
<222> 9-108
<223> b0105 b0106 U00096 complement(114407..114514)
<313> 5044: 51-70
<400> 5044
CCACGCCAGC GTTGGGAGAT TACAGGTTGT TGAAGATGCG GTTTTCTTGT 50
TCCTGCACAC GAATAAACGT GGTGCGCTTG GTCAGCTCTT TCAGGCGTGA 100
<210> 5045
<211> 100
<212> DNA
<213> Escherichia coli K-12 MG1655
<220> b0105 b0106
<222> 0-99
<223> b0105 b0106 U00096 complement(114407..114514)
<313> 5045: 45-64
<400> 5045
GTGTTGCTCC ACGCCAGCGT TGGGAGATTA CAGGTTGTTG AAGATGCGGT 50
TTTCTTGTTC CTGCACACGA ATAAACGTGG TGCGCTTGGT CAGCTCTTT 100
<210> 5046
<211> 100
<212> DNA
<213> Escherichia coli K-12 MG1655
<220> b0105 b0106
<222> 9-108
<223> b0105 b0106 U00096 complement(114407..114514)
<313> 5046: 39-58
<400> 5046
CCACGCCAGC GTTGGGAGAT TACAGGTTGT TGAAGATGCG GTTTTCTTGT 50
TCCTGCACAC GAATAAACGT GGTGCGCTTG GTCAGCTCTT TCAGGCGTGA 100
<210> 5047
<211> 100
<212> DNA
<213> Escherichia coli K-12 MG1655
<220> yjga b4235
<222> 453-552
<223> yjga b4235 U00096 complement(4454888..4455439)
<313> 5047: 522-541
<400> 5047
GATCCGCAAC GCGAAGAAAG AGAAAGAAGG GAATAAGCCG CCGAAATCCG 50
CACGCCAGAT TTTCCAGTAT CTGCGCGAGT TAGCGGAGAA CGAAGGGTAA 100
<210> 5048

<211> 100
<212> DNA
<213> Escherichia coli K-12 MG1655
<220> yjga b4235
<222> 453-552
<223> yjga b4235 U00096 complement(4454888..4455439)
<313> 5048: 490-509
<400> 5048
GATCCGCAAC GCGAAGAAAG AGAAAGAAGG GAATAAGCCG CCGAAATCCG 50
CACGCCAGAT TTTCCAGTAT CTGCGCGAGT TAGCGGAGAA CGAAGGGTAA 100
<210> 5049
<211> 100
<212> DNA
<213> Escherichia coli K-12 MG1655
<220> yjga b4235
<222> 416-515
<223> yjga b4235 U00096 complement(4454888..4455439)
<313> 5049: 446-465
<400> 5049
TGTGGCCCGA TGCCGATCGT CAGCAACTGC GTACTCTGAT CCGCAACGCG 50
AAGAAAGAGA AAGAAGGGAA TAAGCCGCCG AAATCCGCAC GCCAGATTTT 100
<210> 5050
<211> 100
<212> DNA
<213> Escherichia coli K-12 MG1655
<220> yjgb b4270
<222> 850-949
<223> yjgb b4270 U00096 complement(4492758..4493819)
<313> 5050: 882-901
<400> 5050
GTTCCGGCCT TTACGTTAAT TGCGGGCGAT CGCAGCGTCT CTGGTTCTGC 50
TACCGGCACG CCTTATGAGC TGCGTAAGCT GATGCGTTTT GCCGCCCGCA 100
<210> 5051
<211> 100
<212> DNA
<213> Escherichia coli K-12 MG1655
<220> yjgb b4270
<222> 791-890
<223> yjgb b4270 U00096 complement(4492758..4493819)
<313> 5051: 834-853
<400> 5051
AGGCGCTGAC CTATGGCGGT AATTTCCATA CGGTCGGTGC GGTTCTCACG 50
CCGCTGTCTG TTCCGGCCTT TACGTTAATT GCGGGCGATC GCAGCGTCTC 100
<210> 5052
<211> 100
<212> DNA
<213> Escherichia coli K-12 MG1655
<220> yjgb b4270
<222> 748-847
<223> yjgb b4270 U00096 complement(4492758..4493819)
<313> 5052: 798-817
<400> 5052
ATCAACACCG TCAACGTCAG CCTCGACTGG CAGCCCTATT TTGAGGCGCT 50
GACCTATGGC GGTAATTTCC ATACGGTCGG TGCGGTTCTC ACGCCGCTGT 100
<210> 5053
<211> 100
<212> DNA
<213> Escherichia coli K-12 MG1655
<220> yjgd yjgD
<222> 305-404
<223> yjgd yjgD U00096 4476036..4476452
<313> 5053: 349-368
<400> 5053
AGAAATTTGA CGTTGAGTAC GACGGATGGG GCACTTACTT TGAAGATCCT 50
AACGGCGAAG ATGGCGACGA TGAAGATTTT GTCGATGAAG ACGATGACGG 100
<210> 5054
<211> 100
<212> DNA
<213> Escherichia coli K-12 MG1655
<220> yjgd yjgD
<222> 261-360

<223> yjgd yjgD U00096 4476036..4476452
<313> 5054: 294-313
<400> 5054
CGATCTGATC GATGCCCAGG TTGAACAACT GATGACGCTG GCAGAGAAAT 50
TTGACGTTGA GTACGACGGA TGGGGCACTT ACTTTGAAGA TCCTAACGGC 100
<210> 5055
<211> 100
<212> DNA
<213> Escherichia coli K-12 MG1655
<220> yjgd yjgD
<222> 152-251
<223> yjgd yjgD U00096 4476036..4476452
<313> 5055: 197-216
<400> 5055
TTGAAGCGTT TAAACTCGGT TACGAAGTGA CCGATCCAGA AGAGCTGGAA 50
GTTGAAGATG GTGATATCGT GATTTGCTGC GACATCCTCA GCGAGTGCGC 100
<210> 5056
<211> 100
<212> DNA
<213> Escherichia coli K-12 MG1655
<220> yjgf b4244
<222> 327-426
<223> yjgf b4244 U00096 complement(4468101..4468526)
<313> 5056: 385-404
<400> 5056
CGAACACAAC GCCACCTTCC CGGCACGTTC TTGCGTTGAA GTTGCCCGTC 50
TGCCGAAAGA CGTGAAGATT GAGATCGAAG CGATCGCTGT TCGTCGCTAA 100
<210> 5057
<211> 100
<212> DNA
<213> Escherichia coli K-12 MG1655
<220> yjgf b4244
<222> 293-392
<223> yjgf b4244 U00096 complement(4468101..4468526)
<313> 5057: 341-360
<400> 5057
CAACCGTAAA CGCCACTTAC GAAGCCTTCT TCACCGAACA CAACGCCACC 50
TTCCCGGCAC GTTCTTGCGT TGAAGTTGCC CGTCTGCCGA AAGACGTGAA 100
<210> 5058
<211> 100
<212> DNA
<213> Escherichia coli K-12 MG1655
<220> yjgf b4244
<222> 263-362
<223> yjgf b4244 U00096 complement(4468101..4468526)
<313> 5058: 294-313
<400> 5058
CCGTGTTTGT AAAAGATCTG AACGACTTCG CAACCGTAAA CGCCACTTAC 50
GAAGCCTTCT TCACCGAACA CAACGCCACC TTCCCGGCAC GTTCTTGCGT 100
<210> 5059
<211> 100
<212> DNA
<213> Escherichia coli K-12 MG1655
<220> yjgg b4248
<222> 233-332
<223> yjgg b4248 U00096 4470087..4470419
<313> 5059: 292-311
<400> 5059
GATATGCATT TTATTCCATT CATCCGACTT ATAGGGCGGA GTGTGAAAGC 50
GAACGGCTAA CACTATTGCT TACTGCTCAG GGATGCGCGC TATCACTTTA 100
<210> 5060
<211> 100
<212> DNA
<213> Escherichia coli K-12 MG1655
<220> yjgg b4248
<222> 157-256
<223> yjgg b4248 U00096 4470087..4470419
<313> 5060: 203-222
<400> 5060
CTGCAGGGGA ACTGTCCTTC GGTATCAATA ATTGCAGACA TTCCCTGCTT 50
TTTCCTTTTT CACTTTCACG CAATCAGATA TGCATTTTAT TCCATTCATC 100

<210> 5061
<211> 100
<212> DNA
<213> Escherichia coli K-12 MG1655
<220> yjgg b4248
<222> 59-158
<223> yjgg b4248 U00096 4470087..4470419
<313> 5061: 87-106
<400> 5061
GTCAGAAGCG GCGCAAGCAT TTGATGCAAT ATTTTGTCAG CGCAAACGGT 50
TTATTTGAAT TAAAAGTCAA GGTATATGCA TTTTTATTTG ATGTGATTCT 100
<210> 5062
<211> 100
<212> DNA
<213> Escherichia coli K-12 MG1655
<220> yjgh b4249
<222> 297-396
<223> yjgh b4249 U00096 complement(4470388..4470783)
<313> 5062: 332-351
<400> 5062
TAGCGCCCCA CCTTATCCAA ACTGGACGGC GGTGGGTGTT ACATGGCTGG 50
CAGGCTTTGA TTTTGAAATT AAAGTGATAG CGCGCATCCC TGAGCAGTAA 100
<210> 5063
<211> 100
<212> DNA
<213> Escherichia coli K-12 MG1655
<220> yjgh b4249
<222> 207-306
<223> yjgh b4249 U00096 complement(4470388..4470783)
<313> 5063: 255-274
<400> 5063
CACTTTTGAC GATATCATTG ATGTTACGAG CTTCCATACC GATCCAGAAA 50
ACCAATTTGA AGACATCATG ACGGTGAAAA ATGAAATATT TAGCGCCCCA 100
<210> 5064
<211> 100
<212> DNA
<213> Escherichia coli K-12 MG1655
<220> yjgh b4249
<222> 101-200
<223> yjgh b4249 U00096 complement(4470388..4470783)
<313> 5064: 143-162
<400> 5064
CCGGGCAAGT AGGAAGTCGA GAGGACGGAA CACCAGAACC CGATTTTCAG 50
CAACAAGTCA GACTGGCATT TGATAATTTG CATGCGACCC TGGCAGCTGC 100
<210> 5065
<211> 100
<212> DNA
<213> Escherichia coli K-12 MG1655
<220> yjgi yjgI
<222> 607-706
<223> yjgi yjgI U00096 complement(4470914..4471627)
<313> 5065: 663-682
<400> 5065
CATGGGCAAC CGGAAGAGGT CGCTGGTATG GTCGCATGGT TAGCAGGGCC 50
AGAAGCCAGT TTTGTTACCG GCGCGATGCA TACCATTGAT GGCGCGTTTG 100
<210> 5066
<211> 100
<212> DNA
<213> Escherichia coli K-12 MG1655
<220> yjgi yjgI
<222> 598-697
<223> yjgi yjgI U00096 complement(4470914..4471627)
<313> 5066: 618-637
<400> 5066
ATCAAAAGAC ATGGGCAACC GGAAGAGGTC GCTGGTATGG TCGCATGGTT 50
AGCAGGGCCA GAAGCCAGTT TTGTTACCGG CGCGATGCAT ACCATTGATG 100
<210> 5067
<211> 100
<212> DNA
<213> Escherichia coli K-12 MG1655
<220> yjgi yjgI

<222> 521-620
<223> yjgi yjgI U00096 complement(4470914..4471627)
<313> 5067: 560-579
<400> 5067
TCCAGCCAGG GCCAATTGAT ACCGACGCTA ATCCCGCCAA CGGGCCAATG 50
CGCGATATGT TGCATAGTTT GATGGCTATC AAAAGACATG GGCAACCGGA 100
<210> 5068
<211> 100
<212> DNA
<213> Escherichia coli K-12 MG1655
<220> yjgj b4252
<222> 93-192
<223> yjgj b4252 U00096 4472037..4472291
<313> 5068: 121-140
<400> 5068
CATCGCCAGG CGGCATCCAC AACGCGCACA ATGTGTGACT GATTTTATGA 50
GTACCGTGAT GTCTGGGCTT TCTGCGAAGG CACGAGAGGG GCACTCTATA 100
<210> 5069
<211> 100
<212> DNA
<213> Escherichia coli K-12 MG1655
<220> yjgj b4252
<222> 80-179
<223> yjgj b4252 U00096 4472037..4472291
<313> 5069: 129-148
<400> 5069
CCATTTATGA CTACATCGCC AGGCGGCATC CACAACGCGC ACAATGTGTG 50
ACTGATTTTA TGAGTACCGT GATGTCTGGG CTTTCTGCGA AGGCACGAGA 100
<210> 5070
<211> 100
<212> DNA
<213> Escherichia coli K-12 MG1655
<220> yjgj b4252
<222> 83-182
<223> yjgj b4252 U00096 4472037..4472291
<313> 5070: 110-129
<400> 5070
TTTATGACTA CATCGCCAGG CGGCATCCAC AACGCGCACA ATGTGTGACT 50
GATTTTATGA GTACCGTGAT GTCTGGGCTT TCTGCGAAGG CACGAGAGGG 100
<210> 5071
<211> 100
<212> DNA
<213> Escherichia coli K-12 MG1655
<220> yjgk b4253
<222> 363-462
<223> yjgk b4253 U00096 4472427..4472888
<313> 5071: 436-455
<400> 5071
TTTTGTTGTG TTTTATCCGG GGGAAGTGCA TAAACCGCTG TGCGCAGTGG 50
GCGCGCCAGC CCAGGTTCGC AAAGCAGTAG TGAAGATGCT GATGGCGTGA 100
<210> 5072
<211> 100
<212> DNA
<213> Escherichia coli K-12 MG1655
<220> yjgk b4253
<222> 317-416
<223> yjgk b4253 U00096 4472427..4472888
<313> 5072: 372-391
<400> 5072
TGCCGGAAGG CGTTGATGAG AAAACAGTTA TTCTAAATGA AGGTGATTTT 50
GTTGTGTTTT ATCCGGGGGA AGTGCATAAA CCGCTGTGCG CAGTGGGCGC 100
<210> 5073
<211> 100
<212> DNA
<213> Escherichia coli K-12 MG1655
<220> yjgk b4253
<222> 290-389
<223> yjgk b4253 U00096 4472427..4472888
<313> 5073: 324-343
<400> 5073
GGTTAGCTGA TAAAGACATC GCATTTTTGC CGGAAGGCGT TGATGAGAAA 50

ACAGTTATTC TAAATGAAGG TGATTTTGTT GTGTTTTATC CGGGGGAAGT 100
<210> 5074
<211> 100
<212> DNA
<213> Escherichia coli K-12 MG1655
<220> yjgl b4254
<222> 374-473
<223> yjgl b4254 U00096 4472975..4474825
<313> 5074: 440-459
<400> 5074
ATTCACCCAA GGTGATTATA GAAATTTCAC TTTCCACTAT CACTACTATG 50
GGGACTCGTC AAGGACATAC AGCCATTATA TTTCCACAAC CTGATGGTTC 100
<210> 5075
<211> 100
<212> DNA
<213> Escherichia coli K-12 MG1655
<220> yjgl b4254
<222> 331-430
<223> yjgl b4254 U00096 4472975..4474825
<313> 5075: 374-393
<400> 5075
AAAATGAAGT TTTACATGAA TAATCCTGTA GGGGAACGAG CGGATTCACC 50
CAAGGTGATT ATAGAAATTT CACTTTCCAC TATCACTACT ATGGGGACTC 100
<210> 5076
<211> 100
<212> DNA
<213> Escherichia coli K-12 MG1655
<220> yjgl b4254
<222> 205-304
<223> yjgl b4254 U00096 4472975..4474825
<313> 5076: 250-269
<400> 5076
GAGTTTTTGG CAAAGATATT TACCCTGGGT TATGTGGATT TTAGCAAACG 50
CTCCAACGAA GCGGGTAGAA ATATGATGGC TCATATTAAG TCCTCATCTT 100
<210> 5077
<211> 100
<212> DNA
<213> Escherichia coli K-12 MG1655
<220> yjgn b4258
<222> 1083-1182
<223> yjgn b4258 U00096 4477310..4478491
<313> 5077: 1125-1144
<400> 5077
TTTGGATGAC CTTCCTTTAA TCAATAAAGA AGAACAACCA GATAAAGGCT 50
TCTTAGCCAG TATTTCACGG GGAGTTATGC CTTCTTTACC ATTTCTGTAA 100
<210> 5078
<211> 100
<212> DNA
<213> Escherichia coli K-12 MG1655
<220> yjgn b4258
<222> 1019-1118
<223> yjgn b4258 U00096 4477310..4478491
<313> 5078: 1086-1105
<400> 5078
CTTATCCACT GCTGAAAATA TGGATGATTG ACTGGCAGGC AAAAAATACG 50
TATTTGCTGG GCGATTTGGA TGACCTTCCT TTAATCAATA AAGAAGAACA 100
<210> 5079
<211> 100
<212> DNA
<213> Escherichia coli K-12 MG1655
<220> yjgn b4258
<222> 974-1073
<223> yjgn b4258 U00096 4477310..4478491
<313> 5079: 1037-1056
<400> 5079
GCATGTGTGC GTTGGTGGTG ATATCCGGGA TTACGGGCGG TCTGGCTTAT 50
CCACTGCTGA AAATATGGAT GATTGACTGG CAGGCAAAAA ATACGTATTT 100
<210> 5080
<211> 100
<212> DNA
<213> Escherichia coli K-12 MG1655

<220> yjgp b4262
<222> 995-1094
<223> yjgp b4262 U00096 4483786..4484886
<313> 5080: 1046-1065
<400> 5080
GGACCGTTAA CCTGATTTAT CTGGCTTTAG CGATTGTTCT CAACCTTTGG  50
GACACCGTGC CGGTCCGCCG CCTGCGCGCC AGTTTTTCGC GTAAAGGAGC  100
<210> 5081
<211> 100
<212> DNA
<213> Escherichia coli K-12 MG1655
<220> yjgp b4262
<222> 967-1066
<223> yjgp b4262 U00096 4483786..4484886
<313> 5081: 1006-1025
<400> 5081
GGTAAGCTGG ACCCGACGCT GTGGATGTGG ACCGTTAACC TGATTTATCT  50
GGCTTTAGCG ATTGTTCTCA ACCTTTGGGA CACCGTGCCG GTCCGCCGCC  100
<210> 5082
<211> 100
<212> DNA
<213> Escherichia coli K-12 MG1655
<220> yjgp b4262
<222> 918-1017
<223> yjgp b4262 U00096 4483786..4484886
<313> 5082: 952-971
<400> 5082
TCTACTTTTC TTCCTGATCC AGACCTCCCT GAAATCGAAC GGCGGTAAAG  50
GTAAGCTGGA CCCGACGCTG TGGATGTGGA CCGTTAACCT GATTTATCTG  100
<210> 5083
<211> 100
<212> DNA
<213> Escherichia coli K-12 MG1655
<220> yjgq b4263
<222> 987-1086
<223> yjgq b4263 U00096 4484883..4485968
<313> 5083: 1052-1071
<400> 5083
GCTGACGTTG GTTTATGGCA TCCCGCCGAT CATCGGCGCA CTGTTGCCAA  50
GCGCCAGCTT CTTCTTAATC AGCCTGTGGC TGTTAATGAG AAAATCGTAA  100
<210> 5084
<211> 100
<212> DNA
<213> Escherichia coli K-12 MG1655
<220> yjgq b4263
<222> 943-1042
<223> yjgq b4263 U00096 4484883..4485968
<313> 5084: 964-983
<400> 5084
AGTTTCGGTT TTGTCTTCTA CGTACTGGAC CAGATCTTCG GCCCGCTGAC  50
GTTGGTTTAT GGCATCCCGC CGATCATCGG CGCACTGTTG CCAAGCGCCA  100
<210> 5085
<211> 100
<212> DNA
<213> Escherichia coli K-12 MG1655
<220> yjgq b4263
<222> 793-892
<223> yjgq b4263 U00096 4484883..4485968
<313> 5085: 821-840
<400> 5085
AGCGGTCAGG ATGCCGGACG TTATCAGCTC AACATGTGGA GCAAAATCTT  50
CCAGCCGCTA TCTGTGGCGG TGATGATGCT GATGGCGCTG TCGTTCATCT  100
<210> 5086
<211> 100
<212> DNA
<213> Escherichia coli K-12 MG1655
<220> bioa b0775
<222> 1164-1263
<223> bioa b0775 U00096 complement(807191..808480)
<313> 5086: 1202-1221
<400> 5086

CTGGATCCGG CCTTTTGGCA AACTGATTTA CCTGATGCCG CCCTATATTA 50
TTCTCCCGCA ACAGTTGCAG CGTCTGACCG CAGCGGTTAA CCGCGCGGTA 100
<210> 5087
<211> 100
<212> DNA
<213> Escherichia coli K-12 MG1655
<220> bioa b0775
<222> 1064-1163
<223> bioa b0775 U00096 complement(807191..808480)
<313> 5087: 1099-1118
<400> 5087
TGGTTGCCGA TGTGCGCGTA CTGGGGGCCA TTGGCGTGGT CGAAACCACT 50
CATCCGGTGA ATATGGCGGC GCTGCAAAAA TTCTTTGTCG AACAGGGTGT 100
<210> 5088
<211> 100
<212> DNA
<213> Escherichia coli K-12 MG1655
<220> bioa b0775
<222> 995-1094
<223> bioa b0775 U00096 complement(807191..808480)
<313> 5088: 1052-1071
<400> 5088
GGCAGCAACA GGTGGCGGAT ATTGAAGTAC AGCTGCGCGA GCAACTTGCC 50
CCCGCCCGTG ATGCCGAAAT GGTTGCCGAT GTGCGCGTAC TGGGGGCCAT 100
<210> 5089
<211> 100
<212> DNA
<213> Escherichia coli K-12 MG1655
<220> yjgr b4264
<222> 1404-1503
<223> yjgr b4264 U00096 complement(4486129..4487631)
<313> 5089: 1470-1489
<400> 5089
TGGTGTGGTG CAAACAATGG CCAAAAGCGC CGCTCGCCAA GTGACGAATC 50
AGATTGTACG CGGGATGTTG GGGAGTTTGC TGGGGGGGAG AAGAAGGTAA 100
<210> 5090
<211> 100
<212> DNA
<213> Escherichia coli K-12 MG1655
<220> yjgr b4264
<222> 1403-1502
<223> yjgr b4264 U00096 complement(4486129..4487631)
<313> 5090: 1423-1442
<400> 5090
ATGGTGTGGT GCAAACAATG GCCAAAAGCG CCGCTCGCCA AGTGACGAAT 50
CAGATTGTAC GCGGGATGTT GGGGAGTTTG CTGGGGGGGA GAAGAAGGTA 100
<210> 5091
<211> 100
<212> DNA
<213> Escherichia coli K-12 MG1655
<220> yjgr b4264
<222> 1294-1393
<223> yjgr b4264 U00096 complement(4486129..4487631)
<313> 5091: 1331-1350
<400> 5091
CAAAATAATC CTCCCGCGAA AGGGAAAGAG GTAGCGGTGG ATGACGGCAT 50
TCTTGGTGGA TTGAAGGATA TTTTGTTCGG CACTACCGGA CCACGCGGCG 100
<210> 5092
<211> 100
<212> DNA
<213> Escherichia coli K-12 MG1655
<220> rhaa b3904
<222> 1154-1253
<223> rhaa b3904 U00096 complement(4092304..4093563)
<313> 5092: 1203-1222
<400> 5092
AGGCGGTCTG GGAAATGTAT TGCCAACGTC ACGATACGCC AGCAGGTAGC 50
GAATGGCTGG AGAGCGTGCG GGCTTATGAG AAAGAAATTT TGAGTCGCCG 100
<210> 5093
<211> 100
<212> DNA

<213> Escherichia coli K-12 MG1655
<220> rhaa b3904
<222> 1138-1237
<223> rhaa b3904 U00096 complement(4092304..4093563)
<313> 5093: 1164-1183
<400> 5093
AAATCGTTGC CGTGGCAGGC GGTCTGGGAA ATGTATTGCC AACGTCACGA 50
TACGCCAGCA GGTAGCGAAT GGCTGGAGAG CGTGCGGGCT TATGAGAAAG 100
<210> 5094
<211> 100
<212> DNA
<213> Escherichia coli K-12 MG1655
<220> rhaa b3904
<222> 1050-1149
<223> rhaa b3904 U00096 complement(4092304..4093563)
<313> 5094: 1104-1123
<400> 5094
GCGTGCGTTG CTGGAACCTA CCGCTGACGT GCGCAAGCTG GAAGCGGCGG 50
GCGATTACAC TGCGCGTCTG GCACTGCTGG AAGAGCAGAA ATCGTTGCCG 100
<210> 5095
<211> 100
<212> DNA
<213> Escherichia coli K-12 MG1655
<220> biob b0776
<222> 942-1041
<223> biob b0776 U00096 808567..809607
<313> 5095: 1016-1035
<400> 5095
AACTGCCGTG CTGGCAGGGG ATAACGAACA ACAGCAACGT CTTGAACAGG 50
CGCTGATGAC CCCGGACACC GACGAATATT ACAACGCGGC AGCATTATGA 100
<210> 5096
<211> 100
<212> DNA
<213> Escherichia coli K-12 MG1655
<220> biob b0776
<222> 942-1041
<223> biob b0776 U00096 808567..809607
<313> 5096: 980-999
<400> 5096
AACTGCCGTG CTGGCAGGGG ATAACGAACA ACAGCAACGT CTTGAACAGG 50
CGCTGATGAC CCCGGACACC GACGAATATT ACAACGCGGC AGCATTATGA 100
<210> 5097
<211> 100
<212> DNA
<213> Escherichia coli K-12 MG1655
<220> biob b0776
<222> 906-1005
<223> biob b0776 U00096 808567..809607
<313> 5097: 919-938
<400> 5097
GCAACTGTTC CGCAAACTGG GGCTAAATCC GCAGCAAACT GCCGTGCTGG 50
CAGGGGATAA CGAACAACAG CAACGTCTTG AACAGGCGCT GATGACCCCG 100
<210> 5098
<211> 100
<212> DNA
<213> Escherichia coli K-12 MG1655
<220> rhab b3905
<222> 1371-1470
<223> rhab b3905 U00096 complement(4093560..4095029)
<313> 5098: 1428-1447
<400> 5098
CGCGAATCTG ACCACCTTTA CCCCTAATCC TGACAGTGAA ATTGCCCACT 50
ATGTGGCGCA GATTCACTCT ACACGACAGA CAAAGGAGCT TTGCGCATGA 100
<210> 5099
<211> 100
<212> DNA
<213> Escherichia coli K-12 MG1655
<220> rhab b3905
<222> 1347-1446
<223> rhab b3905 U00096 complement(4093560..4095029)
<313> 5099: 1400-1419

<400> 5099
TTTCCGTCAG GTCGTCAGCA CCACCGCGAA TCTGACCACC TTTACCCCTA 50
ATCCTGACAG TGAAATTGCC CACTATGTGG CGCAGATTCA CTCTACACGA 100
<210> 5100
<211> 100
<212> DNA
<213> Escherichia coli K-12 MG1655
<220> rhab b3905
<222> 1288-1387
<223> rhab b3905 U00096 complement(4093560..4095029)
<313> 5100: 1333-1352
<400> 5100
ACGCTCGGCA ATATCGGCAT CCAGTTAATG ACGCTGGATG AACTCAACAA 50
TGTGGATGAT TTCCGTCAGG TCGTCAGCAC CACCGCGAAT CTGACCACCT 100
<210> 5101
<211> 100
<212> DNA
<213> Escherichia coli K-12 MG1655
<220> bioc b0778
<222> 657-756
<223> bioc b0778 U00096 810745..811500
<313> 5101: 721-740
<400> 5101
GCGTTCGCAG TTGCAGCGAT TGCAACTGGC CTGGCCGCAA CAGCAGGGGC 50
GATATCCTCT GACGTATCAT CTTTTTTTGG GAGTGATTGC TCGTGAGTAA 100
<210> 5102
<211> 100
<212> DNA
<213> Escherichia coli K-12 MG1655
<220> bioc b0778
<222> 559-658
<223> bioc b0778 U00096 810745..811500
<313> 5102: 572-591
<400> 5102
ATCACGCTGT GGTTTGATGA TGCGCTCAGT GCCATGCGTT CGCTGAAAGG 50
CATCGGTGCC ACGCATCTTC ATGAAGGGCG CGACCCGCGA ATATTAACGC 100
<210> 5103
<211> 100
<212> DNA
<213> Escherichia coli K-12 MG1655
<220> bioc b0778
<222> 483-582
<223> bioc b0778 U00096 810745..811500
<313> 5103: 540-559
<400> 5103
TGCTAATCGC TTTTTACCGC CAGATGAAAT CGAACAGTCG CTGAACGGCG 50
TGCATTATCA ACATCATATT CAGCCCATCA CGCTGTGGTT TGATGATGCG 100
<210> 5104
<211> 100
<212> DNA
<213> Escherichia coli K-12 MG1655
<220> dcua b4139
<222> 1203-1302
<223> dcua b4139 U00096 complement(4363050..4364351)
<313> 5104: 1273-1292
<400> 5104
CGGTAAATTC GTCTTCAACC ATCCGTTCTT CATCCCGGGT ACTCTGGGTG 50
TTGCCCTGGC CGTTTGCTTC GGCTTCGTGC TGGGTAGCTT CATGCTGTAA 100
<210> 5105
<211> 100
<212> DNA
<213> Escherichia coli K-12 MG1655
<220> dcua b4139
<222> 1113-1212
<223> dcua b4139 U00096 complement(4363050..4364351)
<313> 5105: 1131-1150
<400> 5105
TTTCGCTGCG GTGTCTGGTC TGTTCATTCT GCCGACCTAC CCGACGCTGG 50
TTGCTGCGGT ACAGATGGAT GACACGGGTA CTACCCGTAT CGGTAAATTC 100
<210> 5106
<211> 100

<212> DNA
<213> Escherichia coli K-12 MG1655
<220> dcua b4139
<222> 999-1098
<223> dcua b4139 U00096 complement(4363050..4364351)
<313> 5106: 1064-1083
<400> 5106
GGCCGTCATC TTCTTCTTTG CTTCTGCTCT GCTGTACTCT CAGGCTGCAA 50
CCGCAAAAGC ACTGATGCCG ATGGCTCTGG CACTGAACGT TTCACCGCTG 100
<210> 5107
<211> 100
<212> DNA
<213> Escherichia coli K-12 MG1655
<220> biod b0779
<222> 579-678
<223> biod b0779 U00096 811493..812170
<313> 5107: 638-657
<400> 5107
CCGCATGATT CCCGCGCCGC TGCTGGGAGA GATCCCCTGG CTTGCAGAAA 50
ATCCAGAAAA TGCGGCAACC GGAAAGTACA TAAACCTTGC CTTGTTGTAG 100
<210> 5108
<211> 100
<212> DNA
<213> Escherichia coli K-12 MG1655
<220> biod b0779
<222> 549-648
<223> biod b0779 U00096 811493..812170
<313> 5108: 605-624
<400> 5108
ACGTCACGCT GAATATATGA CCACGCTCAC CCGCATGATT CCCGCGCCGC 50
TGCTGGGAGA GATCCCCTGG CTTGCAGAAA ATCCAGAAAA TGCGGCAACC 100
<210> 5109
<211> 100
<212> DNA
<213> Escherichia coli K-12 MG1655
<220> biod b0779
<222> 510-609
<223> biod b0779 U00096 811493..812170
<313> 5109: 569-588
<400> 5109
GGCGGGTTGG GTGGCGAACG ATGTTACGCC TCCGGGAAAA CGTCACGCTG 50
AATATATGAC CACGCTCACC CGCATGATTC CCGCGCCGCT GCTGGGAGAG 100
<210> 5110
<211> 100
<212> DNA
<213> Escherichia coli K-12 MG1655
<220> dcub b4124
<222> 1242-1341
<223> dcub b4124 U00096 complement(4344982..4346322)
<313> 5110: 1286-1305
<400> 5110
CTTCGTCATC AACCACAGCT TTATTCTGCC GGGGTTGATT GGTGTGAGCG 50
TATCGTGCGT CTTCGGCTGG ATCTTCGCCG CGATGTACGG GTTCTTATAA 100
<210> 5111
<211> 100
<212> DNA
<213> Escherichia coli K-12 MG1655
<220> dcub b4124
<222> 1216-1315
<223> dcub b4124 U00096 complement(4344982..4346322)
<313> 5111: 1238-1257
<400> 5111
CGTTCCGGCA CCACCCACAT CGGTCGCTTC GTCATCAACC ACAGCTTTAT 50
TCTGCCGGGG TTGATTGGTG TGAGCGTATC GTGCGTCTTC GGCTGGATCT 100
<210> 5112
<211> 100
<212> DNA
<213> Escherichia coli K-12 MG1655
<220> dcub b4124
<222> 1139-1238
<223> dcub b4124 U00096 complement(4344982..4346322)

<313> 5112: 1191-1210
<400> 5112
TGGCTTCAGC ACCGGCTTGC TACGGTTATT ACATCCTGCC GACTTATCCG 50
AGCGATCTGG CAGCGATTCA GTTTGACCGT TCCGGCACCA CCCACATCGG 100
<210> 5113
<211> 100
<212> DNA
<213> Escherichia coli K-12 MG1655
<220> rhad b3903
<222> 710-809
<223> rhad b3903 U00096 complement(4091029..4091853)
<313> 5113: 751-770
<400> 5113
TATTAGTGAA GGTTTATTCG ATGGGCGGCA TGAAACAGAC CATCAGCCGT 50
GAAGAGTTGA TAGCGCTCGG CAAGCGTTTC GGCGTTACGC CACTCGCCAG 100
<210> 5114
<211> 100
<212> DNA
<213> Escherichia coli K-12 MG1655
<220> rhad b3903
<222> 665-764
<223> rhad b3903 U00096 complement(4091029..4091853)
<313> 5114: 686-705
<400> 5114
ATGAAACCTT CGGTTTAATC GACACCGCAG AAAAATCAGC ACAAGTATTA 50
GTGAAGGTTT ATTCGATGGG CGGCATGAAA CAGACCATCA GCCGTGAAGA 100
<210> 5115
<211> 100
<212> DNA
<213> Escherichia coli K-12 MG1655
<220> rhad b3903
<222> 556-655
<223> rhad b3903 U00096 complement(4091029..4091853)
<313> 5115: 588-607
<400> 5115
ATGGTGCCCG GCACGGACGA AATCGGCCAG GCGACCGCAC AAGAGATGCA 50
AAAACATTCG CTGGTGTTGT GGCCCTTCCA CGGCGTCTTC GGCAGCGGAC 100
<210> 5116
<211> 100
<212> DNA
<213> Escherichia coli K-12 MG1655
<220> dcuc b0622
<222> 1248-1347
<223> dcuc b0622 U00096 complement(653806..655191)
<313> 5116: 1266-1285
<400> 5116
GGTTGCCGGG ATGGCGAAGA TCTCGCCGTT TGAAGTCGTA AAACGCACCT 50
CGGTACCGGT GCTTGTTGGT TTGGTGATTG TTATCGTTGC TACAGAGCTG 100
<210> 5117
<211> 100
<212> DNA
<213> Escherichia coli K-12 MG1655
<220> dcuc b0622
<222> 1188-1287
<223> dcuc b0622 U00096 complement(653806..655191)
<313> 5117: 1213-1232
<400> 5117
GATGCTGCAG GCGTCAAACC TTGGCCGTAC CCTTTCGCCC GTTTCTGGCG 50
TAGTCGTTGC GGTTGCCGGG ATGGCGAAGA TCTCGCCGTT TGAAGTCGTA 100
<210> 5118
<211> 100
<212> DNA
<213> Escherichia coli K-12 MG1655
<220> dcuc b0622
<222> 1116-1215
<223> dcuc b0622 U00096 complement(653806..655191)
<313> 5118: 1167-1186
<400> 5118
TTATGCGTTT GTTGAGATGA TCCCGAAACT GGCGCACTCT TCCGGCATTA 50
ACCCGGCGTA TTTGACTATC CCGATGCTGC AGGCGTCAAA CCTTGGCCGT 100
<210> 5119

<211> 100
<212> DNA
<213> Escherichia coli K-12 MG1655
<220> moba b3858
<222> 469-568
<223> moba b3858 U00096 complement(4038995..4039579)
<313> 5119: 492-511
<400> 5119
GTAATGGTAT TTATGCGTCT GGCTGGCGGT CATGCGGTTG ATTTCAGCGA 50
TCATAAAGAT GCATTTGTTA ACGTGAATAC GCCAGAGGAG CTTGCCCGAT 100
<210> 5120
<211> 100
<212> DNA
<213> Escherichia coli K-12 MG1655
<220> moba b3858
<222> 419-518
<223> moba b3858 U00096 complement(4038995..4039579)
<313> 5120: 461-480
<400> 5120
GCGCTATTGA GCCTTTATTA CTGGAATATC TGCAAGCAGG AGAACGCCGG 50
GTAATGGTAT TTATGCGTCT GGCTGGCGGT CATGCGGTTG ATTTCAGCGA 100
<210> 5121
<211> 100
<212> DNA
<213> Escherichia coli K-12 MG1655
<220> moba b3858
<222> 357-456
<223> moba b3858 U00096 complement(4038995..4039579)
<313> 5121: 410-429
<400> 5121
TGCGCCTGTC GTGTGGGTCC ATGACGGTGA ACGCGATCAC CCGACTATTG 50
CTCTGGTAAA CCGCGCTATT GAGCCTTTAT TACTGGAATA TCTGCAAGCA 100
<210> 5122
<211> 100
<212> DNA
<213> Escherichia coli K-12 MG1655
<220> biof b0777
<222> 1056-1155
<223> biof b0777 U00096 809604..810758
<313> 5122: 1118-1137
<400> 5122
CGTACCCGCT GGTACTGCGC GACTGCGCTT AACGCTAACC GCTGCGCATG 50
AAATGCAGGA TATCGACCGT CTGCTGGAGG TGCTGCATGG CAACGGTTAA 100
<210> 5123
<211> 100
<212> DNA
<213> Escherichia coli K-12 MG1655
<220> biof b0777
<222> 1038-1137
<223> biof b0777 U00096 809604..810758
<313> 5123: 1068-1087
<400> 5123
GGCGATTCGC CCGCCAACCG TACCCGCTGG TACTGCGCGA CTGCGCTTAA 50
CGCTAACCGC TGCGCATGAA ATGCAGGATA TCGACCGTCT GCTGGAGGTG 100
<210> 5124
<211> 100
<212> DNA
<213> Escherichia coli K-12 MG1655
<220> biof b0777
<222> 974-1073
<223> biof b0777 U00096 809604..810758
<313> 5124: 1036-1055
<400> 5124
TCGGTGATAA CAGCCGTGCG TTACAACTGG CAGAAAAACT GCGTCAGCAA 50
GGCTGCTGGG TCACGGCGAT TCGCCCGCCA ACCGTACCCG CTGGTACTGC 100
<210> 5125
<211> 100
<212> DNA
<213> Escherichia coli K-12 MG1655
<220> mobb b3857
<222> 414-513

<223> mobb b3857 U00096 complement(4038486..4038998)
<313> 5125: 479-498
<400> 5125
TGATGTGCCG CTTAATCTTG ATGTCGCATT ACTGGATATT AATGATGTTG 50
AGGGGCTGGC TGATTTTGTG GTGGAGTGGA TGCAAAAGCA GAACGGATAA 100
<210> 5126
<211> 100
<212> DNA
<213> Escherichia coli K-12 MG1655
<220> mobb b3857
<222> 341-440
<223> mobb b3857 U00096 complement(4038486..4038998)
<313> 5126: 368-387
<400> 5126
TGCTGTTTCG CGATGGAGCC GGACATCGAC CTGAAGAATT AGTGATAGAC 50
AGGCATGTTA TTGCTGTAGC CAGTGATGTG CCGCTTAATC TTGATGTCGC 100
<210> 5127
<211> 100
<212> DNA
<213> Escherichia coli K-12 MG1655
<220> mobb b3857
<222> 208-307
<223> mobb b3857 U00096 complement(4038486..4038998)
<313> 5127: 263-282
<400> 5127
CGATGGGCCT TGATGACGGA AACACCAGAC GAAGAAGAGC TGGATCTACA 50
GTTTCTCGCA AGTCGAATGG ATACCTCAAA GCTGGATTTG ATTCTGGTCG 100
<210> 5128
<211> 100
<212> DNA
<213> Escherichia coli K-12 MG1655
<220> yjgw b4275
<222> 237-336
<223> yjgw b4275 U00096 4497167..4497502
<313> 5128: 313-332
<400> 5128
TGTAGCCTCC CTGCACATGA CCATAATGAT GAACAGGATT GGCACTGTCG 50
CTGACCGACA GCCCAAGGCC AGAAAAGTAA AGCATGGCGA AATGACGTGA 100
<210> 5129
<211> 100
<212> DNA
<213> Escherichia coli K-12 MG1655
<220> yjgw b4275
<222> 211-310
<223> yjgw b4275 U00096 4497167..4497502
<313> 5129: 272-291
<400> 5129
ACTGGCGGTA ATAATCAGTG GCACACTGTA GCCTCCCTGC ACATGACCAT 50
AATGATGAAC AGGATTGGCA CTGTCGCTGA CCGACAGCCC AAGGCCAGAA 100
<210> 5130
<211> 100
<212> DNA
<213> Escherichia coli K-12 MG1655
<220> yjgw b4275
<222> 180-279
<223> yjgw b4275 U00096 4497167..4497502
<313> 5130: 227-246
<400> 5130
TTCTACTGAC GGGCTGATGA GACGTTATGT CACTGGCGGT AATAATCAGT 50
GGCACACTGT AGCCTCCCTG CACATGACCA TAATGATGAA CAGGATTGGC 100
<210> 5131
<211> 100
<212> DNA
<213> Escherichia coli K-12 MG1655
<220> yjgx b4276
<222> 348-447
<223> yjgx b4276 U00096 complement(4497611..4498057)
<313> 5131: 417-436
<400> 5131
GGAAACAGAA CGTGCGCTGG CGGATACATC CTCTCCGAAG ATAATTTTCC 50
TGCACATGAT GGGTTCTCAT CCAAATCCGT GTGACAGCCT TAACTCCTAG 100

<210> 5132
<211> 100
<212> DNA
<213> Escherichia coli K-12 MG1655
<220> yjgx b4276
<222> 316-415
<223> yjgx b4276 U00096 complement(4497611..4498057)
<313> 5132: 370-389
<400> 5132
TCCCGTAAAA CAGATGACAA ACTGTTGTTA CAGGAAACAG AACGTGCGCT 50
GGCGGATACA TCCTCTCCGA AGATAATTTT CCTGCACATG ATGGGTTCTC 100
<210> 5133
<211> 100
<212> DNA
<213> Escherichia coli K-12 MG1655
<220> yjgx b4276
<222> 278-377
<223> yjgx b4276 U00096 complement(4497611..4498057)
<313> 5133: 310-329
<400> 5133
CGGAGCATGC CACTTTCCTC AAGAAAGGCA GCTTCGCTTC CCGTAAAACA 50
GATGACAAAC TGTTGTTACA GGAAACAGAA CGTGCGCTGG CGGATACATC 100
<210> 5134
<211> 100
<212> DNA
<213> Escherichia coli K-12 MG1655
<220> bioh b3413
<222> 655-754
<223> bioh b3413 U00096 complement(3541710..3542480)
<313> 5134: 690-709
<400> 5134
GATAAACTTT GGCCTCACAG CGAATCATAT ATCTTCGCCA AAGCGGCCCA 50
TGCGCCATTT ATTTCGCATC CGGCCGAGTT TTGTCACCTG CTGGTGGCGT 100
<210> 5135
<211> 100
<212> DNA
<213> Escherichia coli K-12 MG1655
<220> bioh b3413
<222> 616-715
<223> bioh b3413 U00096 complement(3541710..3542480)
<313> 5135: 655-674
<400> 5135
CTCGACGGTC TGGTGCCGCG CAAAGTGGTG CCGATGCTGG ATAAACTTTG 50
GCCTCACAGC GAATCATATA TCTTCGCCAA AGCGGCCCAT GCGCCATTTA 100
<210> 5136
<211> 100
<212> DNA
<213> Escherichia coli K-12 MG1655
<220> bioh b3413
<222> 518-617
<223> bioh b3413 U00096 complement(3541710..3542480)
<313> 5136: 568-587
<400> 5136
TTGACGTGCT TAATGGCGGG CTGGAAATCC TGAAAACGGT CGATCTCCGT 50
CAGCCGCTGC AAAACGTGTC CATGCCGTTT TTGCGATTGT ATGGCTATCT 100
<210> 5137
<211> 100
<212> DNA
<213> Escherichia coli K-12 MG1655
<220> pfka b3917
<222> 860-959
<223> pfka b3917 U00096 4105132..4106094
<313> 5137: 915-934
<400> 5137
TCCAGAACGA ACAGCTGGTT CACCACGACA TCATCGACGC TATCGAAAAC 50
ATGAAGCGTC CGTTCAAAGG TGACTGGCTG GACTGCGCGA AAAAACTGTA 100
<210> 5138
<211> 100
<212> DNA
<213> Escherichia coli K-12 MG1655
<220> pfka b3917

<222> 752-851
<223> pfka b3917 U00096 4105132..4106094
<313> 5138: 770-789
<400> 5138
TCCAGCGCGG TGGTTCTCCG GTGCCTTACG ACCGTATTCT GGCTTCCCGT  50
ATGGGCGCTT ACGCTATCGA TCTGCTGCTG GCAGGTTACG GCGGTCGTTG  100
<210> 5139
<211> 100
<212> DNA
<213> Escherichia coli K-12 MG1655
<220> pfka b3917
<222> 662-761
<223> pfka b3917 U00096 4105132..4106094
<313> 5139: 693-712
<400> 5139
TTACCGAACA TATGTGTGAT GTTGACGAAC TGGCGCATTT CATCGAGAAA  50
GAAACCGGTC GTGAAACCCG CGCAACTGTG CTGGGCCACA TCCAGCGCGG  100
<210> 5140
<211> 100
<212> DNA
<213> Escherichia coli K-12 MG1655
<220> yjgy b4277
<222> 291-390
<223> yjgy b4277 U00096 complement(4498000..4498449)
<313> 5140: 333-352
<400> 5140
AATGCAGCGT TATGAACAAC TGAGTAGCAC GGGGCTGACA ACTGGAAAAT  50
CCAGCCGGGA GCACCGTTAT ATGATACGAT TGTTATTGTC ACTGGTGAGA  100
<210> 5141
<211> 100
<212> DNA
<213> Escherichia coli K-12 MG1655
<220> yjgy b4277
<222> 164-263
<223> yjgy b4277 U00096 complement(4498000..4498449)
<313> 5141: 205-224
<400> 5141
GGCGGACACA CCATCGCGAC CACCGTAACT GGCTGGCTCT ACGGCTATTC  50
GTATTATGCT CTGTAAACCG TTGGCCATTG CGGATGGTTA AAGGAACAGT  100
<210> 5142
<211> 100
<212> DNA
<213> Escherichia coli K-12 MG1655
<220> yjgy b4277
<222> 111-210
<223> yjgy b4277 U00096 complement(4498000..4498449)
<313> 5142: 136-155
<400> 5142
TGTTTATCAT TTTCTGGTCA GTGCCGCGAT TCTGGTATTC GTGGTGATTT  50
TCTGGCGGAC ACACCATCGC GACCACCGTA ACTGGCTGGC TCTACGGCTA  100
<210> 5143
<211> 100
<212> DNA
<213> Escherichia coli K-12 MG1655
<220> pfkb pfkB
<222> 831-930
<223> pfkb pfkB U00096 1804394..1805323
<313> 5143: 892-911
<400> 5143
TTTTGGCGTA GCTGCGGGGA GTGCAGCCAC ACTCAATCAG GGAACACGTC  50
TGTGCTCCCA TGACGATACG CAAAAAATTT ACGCTTACCT TTCCCGCTAA  100
<210> 5144
<211> 100
<212> DNA
<213> Escherichia coli K-12 MG1655
<220> pfkb pfkB
<222> 825-924
<223> pfkb pfkB U00096 1804394..1805323
<313> 5144: 853-872
<400> 5144
GGTTCGTTTT GGCGTAGCTG CGGGGAGTGC AGCCACACTC AATCAGGGAA  50

CACGTCTGTG CTCCCATGAC GATACGCAAA AAATTTACGC TTACCTTTCC 100
<210> 5145
<211> 100
<212> DNA
<213> Escherichia coli K-12 MG1655
<220> pfkb pfkB
<222> 785-884
<223> pfkb pfkB U00096 1804394..1805323
<313> 5145: 807-826
<400> 5145
TGACACTGAA ACTGGCAGAA AATGCCTCTC TTGAAGAGAT GGTTCGTTTT 50
GGCGTAGCTG CGGGGAGTGC AGCCACACTC AATCAGGGAA CACGTCTGTG 100
<210> 5146
<211> 100
<212> DNA
<213> Escherichia coli K-12 MG1655
<220> yjgz b4278
<222> 231-330
<223> yjgz b4278 U00096 4498828..4499157
<313> 5146: 272-291
<400> 5146
ACATGCTTGG CTGACGGACG TCCTGACGCG TCTGCCGGAG TGGCCGGAGG 50
AGAGGTTGGC TGAGTTACTG CCTCTTGAGG GCTTTACCTT CTTCGGGTAA 100
<210> 5147
<211> 100
<212> DNA
<213> Escherichia coli K-12 MG1655
<220> yjgz b4278
<222> 169-268
<223> yjgz b4278 U00096 4498828..4499157
<313> 5147: 228-247
<400> 5147
ACTGGTGGCC GGGAACCGCG CGGCACAGAT AATGAGCCTT CTGGGAACCG 50
CAGTCTGGAG CCACATGCTT GGCTGACGGA CGTCCTGACG CGTCTGCCGG 100
<210> 5148
<211> 100
<212> DNA
<213> Escherichia coli K-12 MG1655
<220> yjgz b4278
<222> 58-157
<223> yjgz b4278 U00096 4498828..4499157
<313> 5148: 86-105
<400> 5148
GGACTTTCAC CGACGGAAAG ATCGTGCATT TCAAGGCACA CACAACAATA 50
TCTGCGAGCG TGCTATCCGT CCGGTCGTTA TGGGACGAAA GGCCTGGTTG 100
<210> 5149
<211> 100
<212> DNA
<213> Escherichia coli K-12 MG1655
<220> yjha b4312
<222> 514-613
<223> yjha b4312 U00096 complement(4536353..4537078)
<313> 5149: 561-580
<400> 5149
TGGCAAACGA CGCTATACAG CAAACAGAAC GATTATCGCT ATGCAAACCA 50
TAAGAAATGG GCGACGGAAA ATGCATTTGT TCTACAATAC CATATGACGC 100
<210> 5150
<211> 100
<212> DNA
<213> Escherichia coli K-12 MG1655
<220> yjha b4312
<222> 484-583
<223> yjha b4312 U00096 complement(4536353..4537078)
<313> 5150: 523-542
<400> 5150
TACCATATTA ATAGTGATTT CACCTTCGCA TGGCAAACGA CGCTATACAG 50
CAAACAGAAC GATTATCGCT ATGCAAACCA TAAGAAATGG GCGACGGAAA 100
<210> 5151
<211> 100
<212> DNA
<213> Escherichia coli K-12 MG1655

<220> yjha b4312
<222> 371-470
<223> yjha b4312 U00096 complement(4536353..4537078)
<313> 5151: 417-436
<400> 5151
AAGATCTTAA TTTTGGCATT CGCTATCGTT ACGACTGGAA AGCTTACCGA 50
CAACAAGACT TATCCGGTGA TATGTCTCGT GATAACGTTC ATCGTTGGGA 100
<210> 5152
<211> 100
<212> DNA
<213> Escherichia coli K-12 MG1655
<220> yjhb b4280
<222> 1105-1204
<223> yjhb b4280 U00096 4501566..4502843
<313> 5152: 1128-1147
<400> 5152
AACTTAGGGG CAACTGGAGG AATGGCCGCA CCTGTATTAG CTACATACAT 50
TTCAGGATAT TATGGCTTAG GTGTTTCATT ATTCATTGTT ACGGTTGCAT 100
<210> 5153
<211> 100
<212> DNA
<213> Escherichia coli K-12 MG1655
<220> yjhb b4280
<222> 727-826
<223> yjhb b4280 U00096 4501566..4502843
<313> 5153: 766-785
<400> 5153
CTCGTCTGTT TTTGTCTATT TGGTGCAAAC TGGCCGATAA ACGGACTACT 50
TCCTTCCTAC CTGGCAGATA ATGGAGTTAA TACAGTGGTC ATTTCAACTC 100
<210> 5154
<211> 100
<212> DNA
<213> Escherichia coli K-12 MG1655
<220> yjhb b4280
<222> 336-435
<223> yjhb b4280 U00096 4501566..4502843
<313> 5154: 379-398
<400> 5154
CGGAACAGGC CTTAGCGGTA TTGCTACAAA CTTATATATG CTCGCAGTTT 50
GCCGTTTTAT TGTTGGCTTA GGGATGTCTG GTGAATATGC ATGTGCTTCA 100
<210> 5155
<211> 100
<212> DNA
<213> Escherichia coli K-12 MG1655
<220> rhar b3907
<222> 774-873
<223> rhar b3907 U00096 4096137..4097075
<313> 5155: 818-837
<400> 5155
CAGAGTGTGT CATGCGCAAT ATCTTCTCCA GCATAGCCGC CTGTTAATCA 50
GTGATATTTC GACCGAATGT GGCTTTGAAG ATAGTAACTA TTTTTCGGTG 100
<210> 5156
<211> 100
<212> DNA
<213> Escherichia coli K-12 MG1655
<220> rhar b3907
<222> 721-820
<223> rhar b3907 U00096 4096137..4097075
<313> 5156: 767-786
<400> 5156
CAGCAATTTC GCCAGCAGAC TGGAATGACC ATCAATCAAT ATCTGCGACA 50
GGTCAGAGTG TGTCATGCGC AATATCTTCT CCAGCATAGC CGCCTGTTAA 100
<210> 5157
<211> 100
<212> DNA
<213> Escherichia coli K-12 MG1655
<220> rhar b3907
<222> 679-778
<223> rhar b3907 U00096 4096137..4097075
<313> 5157: 727-746
<400> 5157

AAATTTTGTG ATGAGGCATC GTGCAGTGAG CGCGTTTTGC GTCAGCAATT 50
TCGCCAGCAG ACTGGAATGA CCATCAATCA ATATCTGCGA CAGGTCAGAG 100
<210> 5158
<211> 100
<212> DNA
<213> Escherichia coli K-12 MG1655
<220> cuta b4138
<222> 240-339
<223> cuta b4138 U00096 complement(4362596..4362934)
<313> 5158: 308-327
<400> 5158
GAAGTCTCAT CATCCATATC AAACCCCGGA ACTTCTGGTT TTACCTGTTA 50
CACACGGAGA CACAGATTAC CTCTCATGGC TCAACGCATC TTTACGCTGA 100
<210> 5159
<211> 100
<212> DNA
<213> Escherichia coli K-12 MG1655
<220> cuta b4138
<222> 234-333
<223> cuta b4138 U00096 complement(4362596..4362934)
<313> 5159: 265-284
<400> 5159
ATGCCTGAAG TCTCATCATC CATATCAAAC CCCGGAACTT CTGGTTTTAC 50
CTGTTACACA CGGAGACACA GATTACCTCT CATGGCTCAA CGCATCTTTA 100
<210> 5160
<211> 100
<212> DNA
<213> Escherichia coli K-12 MG1655
<220> cuta b4138
<222> 132-231
<223> cuta b4138 U00096 complement(4362596..4362934)
<313> 5160: 159-178
<400> 5160
CGGCGCTACC TCTCTCTATT ACTGGGAAGG TAAGCTGGAG CAAGAATACG 50
AAGTGCAGAT GATTTTAAAA ACTACCGTAT CTCACCAGCA GGCACTGCTG 100
<210> 5161
<211> 100
<212> DNA
<213> Escherichia coli K-12 MG1655
<220> yjhc b4281
<222> 1035-1134
<223> yjhc b4281 U00096 4502840..4503973
<313> 5161: 1067-1086
<400> 5161
CATGTCGGCG ATTGCTACTG CTGATGCTGC CACTCTTTCA AGATCGCAGG 50
ACAGGAAAGT GAAAATCAGT GAGATCATTA AACATACATC AGTAATGTAA 100
<210> 5162
<211> 100
<212> DNA
<213> Escherichia coli K-12 MG1655
<220> yjhc b4281
<222> 871-970
<223> yjhc b4281 U00096 4502840..4503973
<313> 5162: 913-932
<400> 5162
ATGACCTCAG AAATGGATGG CGCTATAGCA TATGGTCATC CAGGAAAAAA 50
AACACCATTA TGGCTTGCCA GTTTAATAAG AAAGGAGACG TTATTCCTCC 100
<210> 5163
<211> 100
<212> DNA
<213> Escherichia coli K-12 MG1655
<220> yjhc b4281
<222> 837-936
<223> yjhc b4281 U00096 4502840..4503973
<313> 5163: 866-885
<400> 5163
ACAAGAAGAA GATGATGATC GTCGGAAAGG CAATATGACC TCAGAAATGG 50
ATGGCGCTAT AGCATATGGT CATCCAGGAA AAAAAACACC ATTATGGCTT 100
<210> 5164
<211> 100
<212> DNA

<213> Escherichia coli K-12 MG1655
<220> rhas b3906
<222> 738-837
<223> rhas b3906 U00096 4095317..4096153
<313> 5164: 808-827
<400> 5164
TGGATTCAGC GACAGTAACC ACTTTTCGAC GCTTTTTCGC CGAGAGTTTA 50
ACTGGTCACC GCGTGATATT CGCCAGGGAC GGGATGGCTT TCTGCAATAA 100
<210> 5165
<211> 100
<212> DNA
<213> Escherichia coli K-12 MG1655
<220> rhas b3906
<222> 733-832
<223> rhas b3906 U00096 4095317..4096153
<313> 5165: 767-786
<400> 5165
CGCTGTGGAT TCAGCGACAG TAACCACTTT TCGACGCTTT TTCGCCGAGA 50
GTTTAACTGG TCACCGCGTG ATATTCGCCA GGGACGGGAT GGCTTTCTGC 100
<210> 5166
<211> 100
<212> DNA
<213> Escherichia coli K-12 MG1655
<220> rhas b3906
<222> 679-778
<223> rhas b3906 U00096 4095317..4096153
<313> 5166: 724-743
<400> 5166
AAAGCCCGAC ATCTGCTACG CCACAGCGAG GCCAGCGTTA CTGACATCGC 50
CTATCGCTGT GGATTCAGCG ACAGTAACCA CTTTTCGACG CTTTTTCGCC 100
<210> 5167
<211> 100
<212> DNA
<213> Escherichia coli K-12 MG1655
<220> yjhd b4282
<222> 276-375
<223> yjhd b4282 U00096 complement(4504194..4504568)
<313> 5167: 336-355
<400> 5167
TGGCATCATC GTCACCAGCG TCTGTCGTAT TAAAGTCGCG ATGCTAAAAC 50
CTGAACCTCC CAGAGATCCC CATATCCCGC TGATGGGGGA TTTTAACTAA 100
<210> 5168
<211> 100
<212> DNA
<213> Escherichia coli K-12 MG1655
<220> yjhd b4282
<222> 244-343
<223> yjhd b4282 U00096 complement(4504194..4504568)
<313> 5168: 282-301
<400> 5168
CAGTGGGAAC CTTATTCGGA AGAGTCACTA CTTGGCATCA TCGTCACCAG 50
CGTCTGTCGT ATTAAAGTCG CGATGCTAAA ACCTGAACCT CCCAGAGATC 100
<210> 5169
<211> 100
<212> DNA
<213> Escherichia coli K-12 MG1655
<220> yjhd b4282
<222> 144-243
<223> yjhd b4282 U00096 complement(4504194..4504568)
<313> 5169: 210-229
<400> 5169
CGTGAACTGT GAGACGAAAC AGCGTACACA GTTTGAATGT ATCTACTTTT 50
CCCAATACTG GGCGAAGGGA GATTTTATTG CGAAGCGCGC GCCAATTGGT 100
<210> 5170
<211> 100
<212> DNA
<213> Escherichia coli K-12 MG1655
<220> rhat b3908
<222> 936-1035
<223> rhat b3908 U00096 complement(4097072..4098106)
<313> 5170: 994-1013

<400> 5170
GTGGAACAAT GCAGGACGCC GTCCGGTAAC GGTGTTGAGC CTCGGTTGTG 50
TGGTGATTAT TGTCGCCGCT AACATCGTCG GCATCGGCAT GGCGAATTAA 100
<210> 5171
<211> 100
<212> DNA
<213> Escherichia coli K-12 MG1655
<220> rhat b3908
<222> 904-1003
<223> rhat b3908 U00096 complement(4097072..4098106)
<313> 5171: 936-955
<400> 5171
TGCGGCGGTA TCGTCGGGCT GGTGCTGAAA GAGTGGAACA ATGCAGGACG 50
CCGTCCGGTA ACGGTGTTGA GCCTCGGTTG TGTGGTGATT ATTGTCGCCG 100
<210> 5172
<211> 100
<212> DNA
<213> Escherichia coli K-12 MG1655
<220> rhat b3908
<222> 812-911
<223> rhat b3908 U00096 complement(4097072..4098106)
<313> 5172: 856-875
<400> 5172
TGCAATTCTT TTTCTATGCC TGGGGCCACG CCCGCATTCC GGCGCAGTAT 50
GACTACATCA GTTGGATGCT GCATATGAGT TTCTATGTAT TGTGCGGCGG 100
<210> 5173
<211> 100
<212> DNA
<213> Escherichia coli K-12 MG1655
<220> cutc b1875
<222> 342-441
<223> cutc b1875 U00096 complement(1956544..1956984)
<313> 5173: 405-424
<400> 5173
GTCATCAGAT GAACACGCGG ACGAGTATTC GCGTTATATC GTAGACGGGG 50
CGGCGGTTGC TGAAATGAAA GGAATCATTG AACGCCATCA GGCCAAATGA 100
<210> 5174
<211> 100
<212> DNA
<213> Escherichia coli K-12 MG1655
<220> cutc b1875
<222> 317-416
<223> cutc b1875 U00096 complement(1956544..1956984)
<313> 5174: 346-365
<400> 5174
GTTATCGTAA TCAAGGATTG TCCATGTCAT CAGATGAACA CGCGGACGAG 50
TATTCGCGTT ATATCGTAGA CGGGGCGGCG GTTGCTGAAA TGAAAGGAAT 100
<210> 5175
<211> 100
<212> DNA
<213> Escherichia coli K-12 MG1655
<220> cutc b1875
<222> 249-348
<223> cutc b1875 U00096 complement(1956544..1956984)
<313> 5175: 309-328
<400> 5175
CCACTTCCTC GATGCCGGAG TGCTGGAAGT CCATAGCTCC GCGGGAGCGT 50
GGCAAGCCTC ACCGATGCGT TATCGTAATC AAGGATTGTC CATGTCATCA 100
<210> 5176
<211> 100
<212> DNA
<213> Escherichia coli K-12 MG1655
<220> yjhe b4283
<222> 105-204
<223> yjhe b4283 U00096 4504474..4504677
<313> 5176: 128-147
<400> 5176
CAAAACAGGC AACTGGAGTT TACCATTCTA CGGTTCCGTC GCATTGCTTG 50
TTATTGGTAT ATTCCTGACG TTCTTCATGC GTCCAGATAA GTCATTGTAA 100
<210> 5177
<211> 100

<212> DNA
<213> Escherichia coli K-12 MG1655
<220> yjhe b4283
<222> 40-139
<223> yjhe b4283 U00096 4504474..4504677
<313> 5177: 96-115
<400> 5177
TTGATGAACG CCGGTTCCGC TGTCGCCGAC ATTATTTCAC CTATCGCCTT 50
TGGCATTATT ATCGACAAAA CAGGCAACTG GAGTTTACCA TTCTACGGTT 100
<210> 5178
<211> 100
<212> DNA
<213> Escherichia coli K-12 MG1655
<220> yjhe b4283
<222> 25-124
<223> yjhe b4283 U00096 4504474..4504677
<313> 5178: 56-75
<400> 5178
GGAATTGCCA GCGGATTGAT GAACGCCGGT TCCGCTGTCG CCGACATTAT 50
TTCACCTATC GCCTTTGGCA TTATTATCGA CAAAACAGGC AACTGGAGTT 100
<210> 5179
<211> 100
<212> DNA
<213> Escherichia coli K-12 MG1655
<220> yjhf b4297
<222> 1251-1350
<223> yjhf b4297 U00096 complement(4518239..4519588)
<313> 5179: 1303-1322
<400> 5179
CCTGACCACC AAAGAAACGC TGTTATCCTG GACGCTGATG TCCACACTCT 50
TATCCATCAG TGGGCTGATT TTTATTCTGC TCGCCAGCTT AGTTTTATAA 100
<210> 5180
<211> 100
<212> DNA
<213> Escherichia coli K-12 MG1655
<220> yjhf b4297
<222> 1245-1344
<223> yjhf b4297 U00096 complement(4518239..4519588)
<313> 5180: 1271-1290
<400> 5180
TTTTGGCCTG ACCACCAAAG AAACGCTGTT ATCCTGGACG CTGATGTCCA 50
CACTCTTATC CATCAGTGGG CTGATTTTTA TTCTGCTCGC CAGCTTAGTT 100
<210> 5181
<211> 100
<212> DNA
<213> Escherichia coli K-12 MG1655
<220> yjhf b4297
<222> 1127-1226
<223> yjhf b4297 U00096 complement(4518239..4519588)
<313> 5181: 1164-1183
<400> 5181
TCCCATTGTT GGCAGTACAT CCCAATACCA ACCTGGCCTT GATTACGCTG 50
GCTACCGGTG CCGGATCCTG TATTTGTTCT CACGTTAACG ATGCCAGTTT 100
<210> 5182
<211> 100
<212> DNA
<213> Escherichia coli K-12 MG1655
<220> yjhg b4298
<222> 1839-1938
<223> yjhg b4298 U00096 complement(4519695..4521662)
<313> 5182: 1881-1900
<400> 5182
TCCTGAATTG CCAGATGATA CCCGGCTATG GGCAATGCTT CAGGCCGTGA 50
GTGGTGGGAC ATGGACCGGT TGTATTTATG ATGTAAACAA AATTGGCGCG 100
<210> 5183
<211> 100
<212> DNA
<213> Escherichia coli K-12 MG1655
<220> yjhg b4298
<222> 1796-1895
<223> yjhg b4298 U00096 complement(4519695..4521662)

<313> 5183: 1832-1851
<400> 5183
CAATATTAAA TGCCAGACCC AGCCATCAGG ATTTACTTCC CGATCCTGAA 50
TTGCCAGATG ATACCCGGCT ATGGGCAATG CTTCAGGCCG TGAGTGGTGG 100
<210> 5184
<211> 100
<212> DNA
<213> Escherichia coli K-12 MG1655
<220> yjhg b4298
<222> 1749-1848
<223> yjhg b4298 U00096 complement(4519695..4521662)
<313> 5184: 1802-1821
<400> 5184
CGGAACCCGT AGCGATGAAC AATTACCTTC ACAGGAGGAG GCAACTGCAA 50
TATTAAATGC CAGACCCAGC CATCAGGATT TACTTCCCGA TCCTGAATTG 100
<210> 5185
<211> 100
<212> DNA
<213> Escherichia coli K-12 MG1655
<220> cutf b0193
<222> 612-711
<223> cutf b0193 U00096 215269..215979
<313> 5185: 672-691
<400> 5185
TGAGGGTAAT CCGGATACCG GTGCGCCGAC TAAAGTATTG GCACCCGATA 50
CGGCAGGGAA ATTTTACCCC AACCAGGATT GCAGTAGTTT GGGGCAGTAA 100
<210> 5186
<211> 100
<212> DNA
<213> Escherichia coli K-12 MG1655
<220> cutf b0193
<222> 540-639
<223> cutf b0193 U00096 215269..215979
<313> 5186: 596-615
<400> 5186
GCGTAGCTAC CTGGCTGCGC GCGGTCACAG TGAAAAACCG GTGTTACTGT 50
CAGTAGAAGG TCACTTTACG CTTGAGGGTA ATCCGGATAC CGGTGCGCCG 100
<210> 5187
<211> 100
<212> DNA
<213> Escherichia coli K-12 MG1655
<220> cutf b0193
<222> 425-524
<223> cutf b0193 U00096 215269..215979
<313> 5187: 459-478
<400> 5187
TACCTATGAC GCCGATGACC CTGCGGGGCA TGTATTTTTA TATGGCTGAT 50
GCGGCGACCT TCACTGATTG CGCGACCGGA AAACGTTTCA TGGTAGCGAA 100
<210> 5188
<211> 100
<212> DNA
<213> Escherichia coli K-12 MG1655
<220> yjhh b4299
<222> 836-935
<223> yjhh b4299 U00096 complement(4521673..4522632)
<313> 5188: 862-881
<400> 5188
ACAGCATGCA GTGTGTCGGG CTGCCTGTAG AGACATATTG CTTACCACCG 50
ATTCTTGAAG CATCTGAAGA AGCAAAAGAT AAAGTCCACG TGCTGCTTAC 100
<210> 5189
<211> 100
<212> DNA
<213> Escherichia coli K-12 MG1655
<220> yjhh b4299
<222> 785-884
<223> yjhh b4299 U00096 complement(4521673..4522632)
<313> 5189: 813-832
<400> 5189
AACTGCCCGC TATTTACGCC CTCGAAACAC CGTTTGTCTC ACTGATCAAA 50
TACAGCATGC AGTGTGTCGG GCTGCCTGTA GAGACATATT GCTTACCACC 100
<210> 5190

<211> 100
<212> DNA
<213> Escherichia coli K-12 MG1655
<220> yjhh b4299
<222> 742-841
<223> yjhh b4299 U00096 complement(4521673..4522632)
<313> 5190: 775-794
<400> 5190
GGCGATCTGG CGACCGCTGC GACGCTGAAT AAAAAACTAC TACAACTGCC 50
CGCTATTTAC GCCCTCGAAA CACCGTTTGT CTCACTGATC AAATACAGCA 100
<210> 5191
<211> 100
<212> DNA
<213> Escherichia coli K-12 MG1655
<220> yjhi b4300
<222> 462-561
<223> yjhi b4300 U00096 complement(4522583..4523371)
<313> 5191: 490-509
<400> 5191
TTATTTTTTA GAAGCACTCA CGTTAACTCC ACATACGCGC AATACATTTA 50
CCGATAAAAA ATTGTTTCTG GAAGAATTAC AAAAAACACG CCTTCGGGGA 100
<210> 5192
<211> 100
<212> DNA
<213> Escherichia coli K-12 MG1655
<220> yjhi b4300
<222> 406-505
<223> yjhi b4300 U00096 complement(4522583..4523371)
<313> 5192: 428-447
<400> 5192
CATATTACCG CGTTGGGTAA GGCATTATTA GCCTGGAAGA CACGAGAAGA 50
ACTGGATTAT TTTTTAGAAG CACTCACGTT AACTCCACAT ACGCGCAATA 100
<210> 5193
<211> 100
<212> DNA
<213> Escherichia coli K-12 MG1655
<220> yjhi b4300
<222> 350-449
<223> yjhi b4300 U00096 complement(4522583..4523371)
<313> 5193: 366-385
<400> 5193
AAAGTCCGGA CTCTGTACCG ACGAGCAAAA GCTGGATTGG CAAAAAACTT 50
GAGTTGCATA TTACCGCGTT GGGTAAGGCA TTATTAGCCT GGAAGACACG 100
<210> 5194
<211> 100
<212> DNA
<213> Escherichia coli K-12 MG1655
<220> yjhp b4307
<222> 620-719
<223> yjhp b4307 U00096 complement(4530005..4530751)
<313> 5194: 677-696
<400> 5194
TGGAAGCGAA TCCTGACGAT GACTTCGCCG CCGAAGTCAG GGCCGAACTA 50
AACATCGCGC CGAAACGCTA CGTGACCTAC GCGCGGGAAT GCTTTGGCTG 100
<210> 5195
<211> 100
<212> DNA
<213> Escherichia coli K-12 MG1655
<220> yjhp b4307
<222> 500-599
<223> yjhp b4307 U00096 complement(4530005..4530751)
<313> 5195: 559-578
<400> 5195
TGCCAGGACT TGTCGGAGCG TTCGACGATC TCGGCTATGA CGTGGTGGAA 50
ATGGTACTGG CAGACCAGGA AGGCTGGGAC CGGTATGAAG CCGCAAAATG 100
<210> 5196
<211> 100
<212> DNA
<213> Escherichia coli K-12 MG1655
<220> yjhp b4307
<222> 376-475

<223> yjhp b4307 U00096 complement(4530005..4530751)
<313> 5196: 404-423
<400> 5196
CTAGCGCAAA GCCTGAAACC GGGCGGGATC ATGCTTATCG GCGAACCCTA 50
CTGGCGTCAG CTACCAGCAA CAGAAGAAAT AGCCCAGGCC TGCGGCGTCA 100
<210> 5197
<211> 100
<212> DNA
<213> Escherichia coli K-12 MG1655
<220> yjhq b4308
<222> 434-533
<223> yjhq b4308 U00096 complement(4530807..4531352)
<313> 5197: 476-495
<400> 5197
TCCCGGAGGA GCACAAAGCC TGCTGGATGA TGCAATCACT CACCGCTCAG 50
CCCATGACCC TGACCGGCCA CATCCGGTGC GCAGACCCTG ATGAAACCGG 100
<210> 5198
<211> 100
<212> DNA
<213> Escherichia coli K-12 MG1655
<220> yjhq b4308
<222> 392-491
<223> yjhq b4308 U00096 complement(4530807..4531352)
<313> 5198: 440-459
<400> 5198
AACCCTGTGC CGGAGACAAA GGCTATCCGG CACCTTATCC CATCCCGGAG 50
GAGCACAAAG CCTGCTGGAT GATGCAATCA CTCACCGCTC AGCCCATGAC 100
<210> 5199
<211> 100
<212> DNA
<213> Escherichia coli K-12 MG1655
<220> yjhq b4308
<222> 314-413
<223> yjhq b4308 U00096 complement(4530807..4531352)
<313> 5199: 358-377
<400> 5199
TCGAGCACCT GAGGTTGATG GGCTGCCAGA CGGTTTTTGT TCTCGGCCAC 50
GCCACCTATT ACCCCCGACA TGGGTTCGAA CCCTGTGCCG GAGACAAAGG 100
<210> 5200
<211> 100
<212> DNA
<213> Escherichia coli K-12 MG1655
<220> yjhr b4309
<222> 723-822
<223> yjhr b4309 U00096 4532583..4533599
<313> 5200: 763-782
<400> 5200
AAACTGCAAT ATAGCGCATG TCGATGATGA TAAGCGTCAG GAGAAACAAC 50
ATCTGCTGAA TGATGCTGTT GAAAAGCTCA ATAAGATGGG TATCGCTACG 100
<210> 5201
<211> 100
<212> DNA
<213> Escherichia coli K-12 MG1655
<220> yjhr b4309
<222> 686-785
<223> yjhr b4309 U00096 4532583..4533599
<313> 5201: 711-730
<400> 5201
GTTCCCGGGG CATTGATATT ACCGTGGTGA CGGATAAAAA CTGCAATATA 50
GCGCATGTCG ATGATGATAA GCGTCAGGAG AAACAACATC TGCTGAATGA 100
<210> 5202
<211> 100
<212> DNA
<213> Escherichia coli K-12 MG1655
<220> yjhr b4309
<222> 601-700
<223> yjhr b4309 U00096 4532583..4533599
<313> 5202: 651-670
<400> 5202
CAAAAGAAAA TAACGATTAT TTCCCCTTGG TTATCCTGGC AAAAAGTGGA 50
GCAAACGGGA TTTCTGGCAT CAATGGCTTT GGCCCGTTCC CGGGGCATTG 100

<210> 5203
<211> 100
<212> DNA
<213> Escherichia coli K-12 MG1655
<220> ais ais
<222> 469-568
<223> ais ais U00096 complement(2363038..2363640)
<313> 5203: 487-506
<400> 5203
ACCCATAATC ATTGCCTGAC ATATATTGCT AAAGATAAGC GTGACGCGAC  50
ATTTAAACCT GATTATCTGG ATGGTTTAGT CATGCATGTG GAAAAAGGCA  100
<210> 5204
<211> 100
<212> DNA
<213> Escherichia coli K-12 MG1655
<220> ais ais
<222> 309-408
<223> ais ais U00096 complement(2363038..2363640)
<313> 5204: 347-366
<400> 5204
TTATTCCAGT AATACCGTCC GGACCATTCA GTCGGCTACC TGGTTTTCAG  50
CGGGTAAAAA ATTGACGGTA GATAAACGAC TTCTTCAGTG CGGTAATGAG  100
<210> 5205
<211> 100
<212> DNA
<213> Escherichia coli K-12 MG1655
<220> ais ais
<222> 160-259
<223> ais ais U00096 complement(2363038..2363640)
<313> 5205: 197-216
<400> 5205
CCGGTTGTCG TTTTGTTTCG TCATGCTGAA CGTTGCGACC GTTCAACCAA  50
TCAATGCTTG TCAGATAAAA CAGGTATTAC GGTTAAAGGT ACCCAGGATG  100
<210> 5206
<211> 100
<212> DNA
<213> Escherichia coli K-12 MG1655
<220> yjhs b4310
<222> 882-981
<223> yjhs b4310 U00096 complement(4534182..4535162)
<313> 5206: 949-968
<400> 5206
GACGGCACTG CGAAGCAGTC ATTTCAGCAC TGCAGCCCGT CGGGGGATTA  50
TTTCTGACAG ATTTGTAGAA GCAATTTTGC AGTTTTGGCG CGAAAGGTGA  100
<210> 5207
<211> 100
<212> DNA
<213> Escherichia coli K-12 MG1655
<220> yjhs b4310
<222> 850-949
<223> yjhs b4310 U00096 complement(4534182..4535162)
<313> 5207: 892-911
<400> 5207
GGTTCAGCGT ACCGGTCACC GGAGAACTGG ACGACGGCAC TGCGAAGCAG  50
TCATTTCAGC ACTGCAGCCC GTCGGGGGAT TATTTCTGAC AGATTTGTAG  100
<210> 5208
<211> 100
<212> DNA
<213> Escherichia coli K-12 MG1655
<220> yjhs b4310
<222> 794-893
<223> yjhs b4310 U00096 complement(4534182..4535162)
<313> 5208: 841-860
<400> 5208
GAGGACTGAC GAACGCGCCT GATGAAGACC CGGACGATTT AAGCACGGGA  50
TATTACGGTT CAGCGTACCG GTCACCGGAG AACTGGACGA CGGCACTGCG  100
<210> 5209
<211> 100
<212> DNA
<213> Escherichia coli K-12 MG1655
<220> yjht b4311

<222> 1073-1172
<223> yjht b4311 U00096 complement(4535227..4536441)
<313> 5209: 1100-1119
<400> 5209
TATCGCAAGG TCGGGCCTAC GGAGTATCAT TGCCCTGGAA TAATAGTCTA 50
TTGATTATTG GCGGTGAAAC TGCAGGCGGC AAAGCGGTGA CGGATTCAGT 100
<210> 5210
<211> 100
<212> DNA
<213> Escherichia coli K-12 MG1655
<220> yjht b4311
<222> 1004-1103
<223> yjht b4311 U00096 complement(4535227..4536441)
<313> 5210: 1054-1073
<400> 5210
GCCTGAAAAA ATCATATAGC ACTGATATTC ATCTTTGGCA TAACGGGAAA 50
TGGGATAAAT CGGGTGAATT ATCGCAAGGT CGGGCCTACG GAGTATCATT 100
<210> 5211
<211> 100
<212> DNA
<213> Escherichia coli K-12 MG1655
<220> yjht b4311
<222> 924-1023
<223> yjht b4311 U00096 complement(4535227..4536441)
<313> 5211: 962-981
<400> 5211
TTCTCTTATA TTTGCCGGAG GGGCCGGATT CAAAGGTTCA CGAGAAAATT 50
ACCAGAACGG TAAGAACTAT GCGCATGAAG GCCTGAAAAA ATCATATAGC 100
<210> 5212
<211> 100
<212> DNA
<213> Escherichia coli K-12 MG1655
<220> yjhu b4296
<222> 696-795
<223> yjhu b4296 U00096 complement(4516906..4517706)
<313> 5212: 755-774
<400> 5212
AGGCTATGAC GCAACGTTGG GTGCCCTGCT GGGAGGATAT GTCACGGACC 50
TGATTGTTGA TGAAGGAACG GCAGAGTTTT TGCTGGCGTG TGAATTACCA 100
<210> 5213
<211> 100
<212> DNA
<213> Escherichia coli K-12 MG1655
<220> yjhu b4296
<222> 603-702
<223> yjhu b4296 U00096 complement(4516906..4517706)
<313> 5213: 626-645
<400> 5213
CGTCGTGGCT GATGTTGACC AGCGTATTCT GGGAATCAGT CTGGCTCAGC 50
TAAGGCAGAT TGAACGGAAG ATCTTCCTTG CTGGTGGGGA AAGAGGCTAT 100
<210> 5214
<211> 100
<212> DNA
<213> Escherichia coli K-12 MG1655
<220> yjhu b4296
<222> 513-612
<223> yjhu b4296 U00096 complement(4516906..4517706)
<313> 5214: 542-561
<400> 5214
CGTGCTGAAT AAGAAGGAAA TGCAGACGTA TCGGGATTTA GGCGCAGTAG 50
GTGTCATTTG TGGTCGCTTC TATGACAAGG AAGGGATGCC CGTCGTGGCT 100
<210> 5215
<211> 100
<212> DNA
<213> Escherichia coli K-12 MG1655
<220> pfla b0903
<222> 642-741
<223> pfla b0903 U00096 complement(949563..950303)
<313> 5215: 696-715
<400> 5215
TGAAGAGTAC AAACTCGACG GTGTTAAACC ACCGAAGAAA GAGACCATGG 50

AACGCGTGAA AGGCATTCTT GAGCAGTACG GTCATAAGGT AATGTTCTAA 100
<210> 5216
<211> 100
<212> DNA
<213> Escherichia coli K-12 MG1655
<220> pfla b0903
<222> 633-732
<223> pfla b0903 U00096 complement(949563..950303)
<313> 5216: 665-684
<400> 5216
GGCAATGGGT GAAGAGTACA AACTCGACGG TGTTAAACCA CCGAAGAAAG 50
AGACCATGGA ACGCGTGAAA GGCATTCTTG AGCAGTACGG TCATAAGGTA 100
<210> 5217
<211> 100
<212> DNA
<213> Escherichia coli K-12 MG1655
<220> pfla b0903
<222> 593-692
<223> pfla b0903 U00096 complement(949563..950303)
<313> 5217: 626-645
<400> 5217
AGCTTCTCCC CTACCACGAG CTGGGCAAAC ACAAATGGGT GGCAATGGGT 50
GAAGAGTACA AACTCGACGG TGTTAAACCA CCGAAGAAAG AGACCATGGA 100
<210> 5218
<211> 100
<212> DNA
<213> Escherichia coli K-12 MG1655
<220> pflb b0904
<222> 2184-2283
<223> pflb b0904 U00096 complement(950495..952777)
<313> 5218: 2255-2274
<400> 5218
GCTGACCATC CGTGTATCTG GCTACGCAGT ACGTTTCAAC TCGCTGACTA 50
AAGAACAGCA GCAGGACGTT ATTACTCGTA CCTTCACTCA ATCTATGTAA 100
<210> 5219
<211> 100
<212> DNA
<213> Escherichia coli K-12 MG1655
<220> pflb b0904
<222> 2150-2249
<223> pflb b0904 U00096 complement(950495..952777)
<313> 5219: 2217-2236
<400> 5219
TCGACGCGAT GGAAAACCCG GAAAAATATC CGCAGCTGAC CATCCGTGTA 50
TCTGGCTACG CAGTACGTTT CAACTCGCTG ACTAAAGAAC AGCAGCAGGA 100
<210> 5220
<211> 100
<212> DNA
<213> Escherichia coli K-12 MG1655
<220> pflb b0904
<222> 2120-2219
<223> pflb b0904 U00096 complement(950495..952777)
<313> 5220: 2163-2182
<400> 5220
ACGTTAACGT GATGAACCGT GAAATGCTGC TCGACGCGAT GGAAAACCCG 50
GAAAAATATC CGCAGCTGAC CATCCGTGTA TCTGGCTACG CAGTACGTTT 100
<210> 5221
<211> 100
<212> DNA
<213> Escherichia coli K-12 MG1655
<220> pflc b3953
<222> 780-879
<223> pflc b3953 U00096 4143837..4144715
<313> 5221: 850-869
<400> 5221
ATGGTCGATG AAAGAGGTGC CTGCGCCGTC GTCAGCTGAT GTGGCAACGA 50
TGCGCGAAAT GGCAGAACGG GCCGGACTTC AGGTTACCGT GGGAGGTTAA 100
<210> 5222
<211> 100
<212> DNA
<213> Escherichia coli K-12 MG1655

<220> pflc b3953
<222> 736-835
<223> pflc b3953 U00096 4143837..4144715
<313> 5222: 774-793
<400> 5222
TTTCATCAGT ACGGCGAACC GAAATACCGC CTGCTGGGGA AAACATGGTC  50
GATGAAAGAG GTGCCTGCGC CGTCGTCAGC TGATGTGGCA ACGATGCGCG  100
<210> 5223
<211> 100
<212> DNA
<213> Escherichia coli K-12 MG1655
<220> pflc b3953
<222> 708-807
<223> pflc b3953 U00096 4143837..4144715
<313> 5223: 726-745
<400> 5223
GAATATCAGG CAGATCCATC TGTTACCGTT TCATCAGTAC GGCGAACCGA  50
AATACCGCCT GCTGGGGAAA ACATGGTCGA TGAAAGAGGT GCCTGCGCCG  100
<210> 5224
<211> 100
<212> DNA
<213> Escherichia coli K-12 MG1655
<220> pfld b3952
<222> 2199-2298
<223> pfld b3952 U00096 4141574..4143871
<313> 5224: 2249-2268
<400> 5224
CGGGCTGGTG GTGCGCGTTG CCGGATACAG CGCCTTCTTT GTCGAACTGT  50
CGAAGGAGAT CCAGGATGAC ATCATCCGCC GGACAGCGCA TCAGCTGTAA  100
<210> 5225
<211> 100
<212> DNA
<213> Escherichia coli K-12 MG1655
<220> pfld b3952
<222> 2128-2227
<223> pfld b3952 U00096 4141574..4143871
<313> 5225: 2180-2199
<400> 5225
CAACATATTC AGTTTAACGT GGTGAACGCC GACACGTTGC GGGAAGCGCA  50
ACAGCGCCCA CAAGATTATG CCGGGCTGGT GGTGCGCGTT GCCGGATACA  100
<210> 5226
<211> 100
<212> DNA
<213> Escherichia coli K-12 MG1655
<220> pfld b3952
<222> 2044-2143
<223> pfld b3952 U00096 4141574..4143871
<313> 5226: 2094-2113
<400> 5226
AAATTCACTC CGGCGACCCT GGAAGGTGAA GCAGGATTAC GCAAACTGGC  50
CGACTTCTTA CGGGCGTTTA CCCAGCTTAA GTTACAACAT ATTCAGTTTA  100
<210> 5227
<211> 100
<212> DNA
<213> Escherichia coli K-12 MG1655
<220> mgsa mgsA
<222> 369-468
<223> mgsa mgsA U00096 complement(1025780..1026247)
<313> 5227: 430-449
<400> 5227
GGCAACGGCA GACTTCATAA TCCAGTCGCC GCATTTCAAC GACGCGGTCG  50
ATATTCTGAT CCCCGATTAT CAGCGTTATC TCGCGGACCG TCTGAAGTAA  100
<210> 5228
<211> 100
<212> DNA
<213> Escherichia coli K-12 MG1655
<220> mgsa mgsA
<222> 369-468
<223> mgsa mgsA U00096 complement(1025780..1026247)
<313> 5228: 395-414
<400> 5228

GGCAACGGCA GACTTCATAA TCCAGTCGCC GCATTTCAAC GACGCGGTCG 50
ATATTCTGAT CCCCGATTAT CAGCGTTATC TCGCGGACCG TCTGAAGTAA 100
<210> 5229
<211> 100
<212> DNA
<213> Escherichia coli K-12 MG1655
<220> mgsa mgsA
<222> 304-403
<223> mgsa mgsA U00096 complement(1025780..1026247)
<313> 5229: 355-374
<400> 5229
GATCCTGACG TGAAAGCCTT GCTGCGTCTG GCGACGGTAT GGAACATTCC 50
GGTCGCCACC AACGTGGCAA CGGCAGACTT CATAATCCAG TCGCCGCATT 100
<210> 5230
<211> 100
<212> DNA
<213> Escherichia coli K-12 MG1655
<220> yjia b4353
<222> 756-855
<223> yjia b4353 U00096 complement(4585479..4586333)
<313> 5230: 825-844
<400> 5230
GCCGTGGGGC GATGAAAAAC CGCATAGCAC GATGGTGTTT ATCGGGATCC 50
AGCTGCCGGA GGAAGAGATT CGGGCTGCGT TTGCGGGGTT GAGGAAGTAA 100
<210> 5231
<211> 100
<212> DNA
<213> Escherichia coli K-12 MG1655
<220> yjia b4353
<222> 723-822
<223> yjia b4353 U00096 complement(4585479..4586333)
<313> 5231: 767-786
<400> 5231
CGTCCAGCGC CTCTACAGCG CCGACTGGGA CAGGCCGTGG GGCGATGAAA 50
AACCGCATAG CACGATGGTG TTTATCGGGA TCCAGCTGCC GGAGGAAGAG 100
<210> 5232
<211> 100
<212> DNA
<213> Escherichia coli K-12 MG1655
<220> yjia b4353
<222> 658-757
<223> yjia b4353 U00096 complement(4585479..4586333)
<313> 5232: 698-717
<400> 5232
AAACTGCTAC GTTACAAAGG GATGCTGTGG ATTGACGGCG AACCTAACCG 50
CCTGCTGTTC CAGGGCGTCC AGCGCCTCTA CAGCGCCGAC TGGGACAGGC 100
<210> 5233
<211> 100
<212> DNA
<213> Escherichia coli K-12 MG1655
<220> yjic b4326
<222> 732-831
<223> yjic b4326 U00096 complement(4553059..4553889)
<313> 5233: 756-775
<400> 5233
TGATGAAAAT GCACCACTCA AACGTATGAC TCTGCAACCG TCATCTCTTC 50
CAGAACTGTT TGAATTAAAA AATGGTGAAT GGAATTATAT TGGTAAATAA 100
<210> 5234
<211> 100
<212> DNA
<213> Escherichia coli K-12 MG1655
<220> yjic b4326
<222> 488-587
<223> yjic b4326 U00096 complement(4553059..4553889)
<313> 5234: 529-548
<400> 5234
CGATAGGATA TATTTCAACC CTTCCAGCTT CTCAGGCAAA AAGATGGACC 50
AATGAGCAGC CAAGGATTGA TATCTATATT GATCAGATAA TGACGGTAAC 100
<210> 5235
<211> 100
<212> DNA

<213> Escherichia coli K-12 MG1655
<220> yjic b4326
<222> 417-516
<223> yjic b4326 U00096 complement(4553059..4553889)
<313> 5235: 475-494
<400> 5235
TGTTGAGGAC TATACAGTTC ATGAAATAAC ATTAGGCCCT GGTTGCAATG 50
TTCCTGGTTA TGCCGGAACC ACGATAGGAT ATATTTCAAC CCTTCCAGCT 100
<210> 5236
<211> 100
<212> DNA
<213> Escherichia coli K-12 MG1655
<220> yjid b4327
<222> 303-402
<223> yjid b4327 U00096 4554553..4554954
<313> 5236: 370-389
<400> 5236
CCTTGAGATA ATGCTGTACC ACGACGATGA ACCGCTAACG TTTGATCTGG 50
TCTGGGATAA TGGCGGTTGG CGCAGTGCGA CGCTGGAGAA TGTCAGCTAA 100
<210> 5237
<211> 100
<212> DNA
<213> Escherichia coli K-12 MG1655
<220> yjid b4327
<222> 284-383
<223> yjid b4327 U00096 4554553..4554954
<313> 5237: 322-341
<400> 5237
GCCACACTCT GGCGATCAGC CTTGAGATAA TGCTGTACCA CGACGATGAA 50
CCGCTAACGT TTGATCTGGT CTGGGATAAT GGCGGTTGGC GCAGTGCGAC 100
<210> 5238
<211> 100
<212> DNA
<213> Escherichia coli K-12 MG1655
<220> yjid b4327
<222> 259-358
<223> yjid b4327 U00096 4554553..4554954
<313> 5238: 284-303
<400> 5238
CAGGTTAATT TGAAAGAAGC GTACTGCCAC ACTCTGGCGA TCAGCCTTGA 50
GATAATGCTG TACCACGACG ATGAACCGCT AACGTTTGAT CTGGTCTGGG 100
<210> 5239
<211> 100
<212> DNA
<213> Escherichia coli K-12 MG1655
<220> yjie b4328
<222> 813-912
<223> yjie b4328 U00096 complement(4554947..4555858)
<313> 5239: 882-901
<400> 5239
CCCGATTCAG GCCTATGCAT ACCGGATGAA TACCCGCATG AATCCCGTTG 50
CCGAACGCTT CTGGCGTGAA CTGCGCGAGC TGGAGATTGT GCTTAGCTGA 100
<210> 5240
<211> 100
<212> DNA
<213> Escherichia coli K-12 MG1655
<220> yjie b4328
<222> 809-908
<223> yjie b4328 U00096 complement(4554947..4555858)
<313> 5240: 830-849
<400> 5240
TGATCCCGAT TCAGGCCTAT GCATACCGGA TGAATACCCG CATGAATCCC 50
GTTGCCGAAC GCTTCTGGCG TGAACTGCGC GAGCTGGAGA TTGTGCTTAG 100
<210> 5241
<211> 100
<212> DNA
<213> Escherichia coli K-12 MG1655
<220> yjie b4328
<222> 726-825
<223> yjie b4328 U00096 complement(4554947..4555858)
<313> 5241: 779-798

<400> 5241
TGGGATTGCC TGGCTGCCGG AGTACGCCAT CCAACAAGAA ATTCGCAGCG  50
GGAAGCTCGT TGTACTGAAT CGGGACGAAC TGGTGATCCC GATTCAGGCC  100
<210> 5242
<211> 100
<212> DNA
<213> Escherichia coli K-12 MG1655
<220> yjig b4330
<222> 363-462
<223> yjig b4330 U00096 complement(4557108..4557569)
<313> 5242: 417-436
<400> 5242
GGGGACAGCC GAAGTGAATG CCAAATATTA CCCGCATATC ATTACGGTCT  50
GCGTGATTAA CGCATTGCTG TCGATCTGGG TTATGCAGCT TATTGTTTAA  100
<210> 5243
<211> 100
<212> DNA
<213> Escherichia coli K-12 MG1655
<220> yjig b4330
<222> 352-451
<223> yjig b4330 U00096 complement(4557108..4557569)
<313> 5243: 376-395
<400> 5243
GGTCGCTGCC TGGGGACAGC CGAAGTGAAT GCCAAATATT ACCCGCATAT  50
CATTACGGTC TGCGTGATTA ACGCATTGCT GTCGATCTGG GTTATGCAGC  100
<210> 5244
<211> 100
<212> DNA
<213> Escherichia coli K-12 MG1655
<220> yjig b4330
<222> 275-374
<223> yjig b4330 U00096 complement(4557108..4557569)
<313> 5244: 337-356
<400> 5244
CTGGCGCATT AACCGGTCAC GATGTCACCG TTTTACTGCC CGCGATGTAT  50
TTAATGGGCA ACCCGGTGCA AAATGTCGGT CGCTGCCTGG GGACAGCCGA  100
<210> 5245
<211> 100
<212> DNA
<213> Escherichia coli K-12 MG1655
<220> yjih b4331
<222> 597-696
<223> yjih b4331 U00096 complement(4557566..4558261)
<313> 5245: 676-695
<400> 5245
AGCCGTCATC CTGGTGTTTA AATTTGTCGG AGCCAACATT TTGCGCGTCT  50
GGCTTAACTT CGAAGAACGT CGCAACCCAA CGCAAGGAGC GCAAGCATGA  100
<210> 5246
<211> 100
<212> DNA
<213> Escherichia coli K-12 MG1655
<220> yjih b4331
<222> 597-696
<223> yjih b4331 U00096 complement(4557566..4558261)
<313> 5246: 621-640
<400> 5246
AGCCGTCATC CTGGTGTTTA AATTTGTCGG AGCCAACATT TTGCGCGTCT  50
GGCTTAACTT CGAAGAACGT CGCAACCCAA CGCAAGGAGC GCAAGCATGA  100
<210> 5247
<211> 100
<212> DNA
<213> Escherichia coli K-12 MG1655
<220> yjih b4331
<222> 556-655
<223> yjih b4331 U00096 complement(4557566..4558261)
<313> 5247: 596-615
<400> 5247
GCGGTGTTTG CCTTTCTGGG CACGTCCGTA ATCGTACCTT TAGCCGTCAT  50
CCTGGTGTTT AAATTTGTCG GAGCCAACAT TTTGCGCGTC TGGCTTAACT  100
<210> 5248
<211> 100

<212> DNA
<213> Escherichia coli K-12 MG1655
<220> yjii b4332
<222> 558-657
<223> yjii b4332 U00096 4558397..4559053
<313> 5248: 604-623
<400> 5248
CAAAGCACAG GAAATGGCGA AACGAGGCCT CCCTTTCTGG CAAGCGGAAA 50
ACGGGGTCTG GCTGACATCA ACGGTTGCGG TTGAGTTTCT TGAGTGGTAA 100
<210> 5249
<211> 100
<212> DNA
<213> Escherichia coli K-12 MG1655
<220> yjii b4332
<222> 533-632
<223> yjii b4332 U00096 4558397..4559053
<313> 5249: 558-577
<400> 5249
ACGGATCGCC GGTTATTTTA ACCGTCAAAG CACAGGAAAT GGCGAAACGA 50
GGCCTCCCTT TCTGGCAAGC GGAAAACGGG GTCTGGCTGA CATCAACGGT 100
<210> 5250
<211> 100
<212> DNA
<213> Escherichia coli K-12 MG1655
<220> yjii b4332
<222> 484-583
<223> yjii b4332 U00096 4558397..4559053
<313> 5250: 516-535
<400> 5250
GTACATCTCT CTGCTGATGA AGCAACGGCA CGCAAAGTGG GAGCAAGACA 50
CGGATCGCCG GTTATTTTAA CCGTCAAAGC ACAGGAAATG GCGAAACGAG 100
<210> 5251
<211> 100
<212> DNA
<213> Escherichia coli K-12 MG1655
<220> yjij b4333
<222> 993-1092
<223> yjij b4333 U00096 complement(4559066..4560244)
<313> 5251: 1011-1030
<400> 5251
CAATCTTTTA GGCTTTGTGA CGCTCATTTA CGGCATTGGG CAAATCCTCG 50
GCCCGGCGCT GACCAGTATG CTCAGCAATG GAACATCGGC GCTTGCCAGC 100
<210> 5252
<211> 100
<212> DNA
<213> Escherichia coli K-12 MG1655
<220> yjij b4333
<222> 921-1020
<223> yjij b4333 U00096 complement(4559066..4560244)
<313> 5252: 985-1004
<400> 5252
TGGCGGTACC TTTATGGGCA CGACATCTCT GGTGATGACT ATCGCCCGCC 50
AACTGAGCGT ACCGGGAAAT CTCAATCTTT TAGGCTTTGT GACGCTCATT 100
<210> 5253
<211> 100
<212> DNA
<213> Escherichia coli K-12 MG1655
<220> yjij b4333
<222> 862-961
<223> yjij b4333 U00096 complement(4559066..4560244)
<313> 5253: 907-926
<400> 5253
GTGCTGCTTA CTCTCGCCAG CGACTCGCCT CTCTTGCTTA TCATCAGCAG 50
TCTTGGTTTT GGCGGTACCT TTATGGGCAC GACATCTCTG GTGATGACTA 100
<210> 5254
<211> 100
<212> DNA
<213> Escherichia coli K-12 MG1655
<220> yjik b4334
<222> 835-934
<223> yjik b4334 U00096 complement(4560312..4561283)

<313> 5254: 889-908
<400> 5254
TCACTAACCA AAGGCAGTCG AGGTTTGTCG CATAACATCA AACAGGCCGA  50
AGGGGTTGCG ATGGATGCCT CTGGTAATAT TTATATCGTT AGCGAACCAA  100
<210> 5255
<211> 100
<212> DNA
<213> Escherichia coli K-12 MG1655
<220> yjik b4334
<222> 810-909
<223> yjik b4334 U00096 complement(4560312..4561283)
<313> 5255: 852-871
<400> 5255
GGTTGGCGAG GTTATCGGTG AAATGTCACT AACCAAAGGC AGTCGAGGTT  50
TGTCGCATAA CATCAAACAG GCCGAAGGGG TTGCGATGGA TGCCTCTGGT  100
<210> 5256
<211> 100
<212> DNA
<213> Escherichia coli K-12 MG1655
<220> yjik b4334
<222> 752-851
<223> yjik b4334 U00096 complement(4560312..4561283)
<313> 5256: 798-817
<400> 5256
AAAAAAATAC GCTTCTGGTG CTGTCTCATG AATCTCGCGC ACTCCAGGAA  50
GTAACCCTGG TTGGCGAGGT TATCGGTGAA ATGTCACTAA CCAAAGGCAG  100
<210> 5257
<211> 100
<212> DNA
<213> Escherichia coli K-12 MG1655
<220> yjil b4335
<222> 637-736
<223> yjil b4335 U00096 complement(4561491..4562264)
<313> 5257: 654-673
<400> 5257
GGCGTTAGTC ATTGCCAGAA GTTTGCCCGG ATGCTGGAAT CTCACCTGCG  50
AATGCCGGTA AATACCCATC CTGATGCGCA ATTTGCTGGC GCAATTGGCG  100
<210> 5258
<211> 100
<212> DNA
<213> Escherichia coli K-12 MG1655
<220> yjil b4335
<222> 557-656
<223> yjil b4335 U00096 complement(4561491..4562264)
<313> 5258: 605-624
<400> 5258
GAGTGATTAA CGCGATGGCG CGGAGGAGTG CCAATTTCAT TGCTCGTCTC  50
TCCTGTGAAG CGCCGATTCT GTTTACTGGT GGCGTTAGTC ATTGCCAGAA  100
<210> 5259
<211> 100
<212> DNA
<213> Escherichia coli K-12 MG1655
<220> yjil b4335
<222> 531-630
<223> yjil b4335 U00096 complement(4561491..4562264)
<313> 5259: 561-580
<400> 5259
CGTCGCGCCA GAAGCGATTC TCGCAGGAGT GATTAACGCG ATGGCGCGGA  50
GGAGTGCCAA TTTCATTGCT CGTCTCTCCT GTGAAGCGCC GATTCTGTTT  100
<210> 5260
<211> 100
<212> DNA
<213> Escherichia coli K-12 MG1655
<220> yjim b4336
<222> 1074-1173
<223> yjim b4336 U00096 complement(4562268..4563440)
<313> 5260: 1126-1145
<400> 5260
GCAGCACAAC ATTCCTTATA TCGCTATTGA AACAGACTAC TCCACCTCGG  50
ATGTCGGGCA GCTCAGTACC CGTGTCGCGG CCTTTATTGA GATGCTGTAA  100
<210> 5261

<211> 100
<212> DNA
<213> Escherichia coli K-12 MG1655
<220> yjim b4336
<222> 1027-1126
<223> yjim b4336 U00096 complement(4562268..4563440)
<313> 5261: 1076-1095
<400> 5261
CATACCTACG CGGTGGAATC GCTGGCGATT AAACGTCATG TGCGCCAGCA 50
GCACAACATT CCTTATATCG CTATTGAAAC AGACTACTCC ACCTCGGATG 100
<210> 5262
<211> 100
<212> DNA
<213> Escherichia coli K-12 MG1655
<220> yjim b4336
<222> 949-1048
<223> yjim b4336 U00096 complement(4562268..4563440)
<313> 5262: 989-1008
<400> 5262
CAGCGCCTGA AAATGCTCAG CCAGATGGTG GAGGAATATC AGGTCGATGG 50
CGTAGTTGAT GTGATTTTGC AGGCGTGCCA TACCTACGCG GTGGAATCGC 100
<210> 5263
<211> 100
<212> DNA
<213> Escherichia coli K-12 MG1655
<220> yjin b4337
<222> 1182-1281
<223> yjin b4337 U00096 complement(4563535..4564815)
<313> 5263: 1256-1275
<400> 5263
GTTTATTCGT GTCAACGGTA CGCTGGTTGG CGGTTGTATT GGGCTAATTT 50
TATATTTGCT GTCGCAGCTC CCGGCCTTGT TCCCCCTCGG CAATTTTTAG 100
<210> 5264
<211> 100
<212> DNA
<213> Escherichia coli K-12 MG1655
<220> yjin b4337
<222> 1088-1187
<223> yjin b4337 U00096 complement(4563535..4564815)
<313> 5264: 1107-1126
<400> 5264
CATTCCTGAC GCGCCACATC AGCGATACGG TAAAAAGCTG GGATGCGCGG 50
GATATGTCGC GGCAAATAGA GTTAAATATC GGCAAAGATC TGCAGTTTAT 100
<210> 5265
<211> 100
<212> DNA
<213> Escherichia coli K-12 MG1655
<220> yjin b4337
<222> 1023-1122
<223> yjin b4337 U00096 complement(4563535..4564815)
<313> 5265: 1067-1086
<400> 5265
CTTGCGGGCG TCGTTAAATG GTCATCTTGA ACAAGCCGCG CACCGCGTCG 50
CGCCTGAGTT TTCCGCATTC CTGACGCGCC ACATCAGCGA TACGGTAAAA 100
<210> 5266
<211> 100
<212> DNA
<213> Escherichia coli K-12 MG1655
<220> yjio b4338
<222> 1134-1233
<223> yjio b4338 U00096 complement(4564856..4566088)
<313> 5266: 1211-1230
<400> 5266
GTTAGCCGTT GTGGCGGGCG TTATCGTCGT TTTCACCCTG GCGGGATTGC 50
TCAATCGCGT GCGCCAGCAT CAGGCAGCCG AGCTAGTGGA GGAGCAGTGA 100
<210> 5267
<211> 100
<212> DNA
<213> Escherichia coli K-12 MG1655
<220> yjio b4338
<222> 1111-1210

<223> yjio b4338 U00096 complement(4564856..4566088)
<313> 5267: 1162-1181
<400> 5267
GGCGGTCGCT TGCCGTTTCA TCTGTTAGCC GTTGTGGCGG GCGTTATCGT  50
CGTTTTCACC CTGGCGGGAT TGCTCAATCG CGTGCGCCAG CATCAGGCAG  100
<210> 5268
<211> 100
<212> DNA
<213> Escherichia coli K-12 MG1655
<220> yjio b4338
<222> 1018-1117
<223> yjio b4338 U00096 complement(4564856..4566088)
<313> 5268: 1058-1077
<400> 5268
TTACCGAAAG GGACCGTCTC CGCATCGCTA AATATGGTGA TCCTGATGGT  50
GATGTCGGTC TCGGTCGAAA TCGGCCGCTG GCTATGGTTT AACGGCGGTC  100
<210> 5269
<211> 100
<212> DNA
<213> Escherichia coli K-12 MG1655
<220> yjip b4339
<222> 200-299
<223> yjip b4339 U00096 4566567..4566878
<313> 5269: 238-257
<400> 5269
CGGTAAAGAC CCGCGAAGGC GATGGCTATA TCTATGTGGT GATTGAACAT  50
CAGAGCCGCG AGGACATTCA TATGGCCTTT CGCCTGATGC GCTATTCCAT  100
<210> 5270
<211> 100
<212> DNA
<213> Escherichia coli K-12 MG1655
<220> yjip b4339
<222> 148-247
<223> yjip b4339 U00096 4566567..4566878
<313> 5270: 189-208
<400> 5270
GCCAGCTTTG TCGATGAAAA ATTGCGGGCG CTACACTCCG ATATTTTATG  50
GTCGGTAAAG ACCCGCGAAG GCGATGGCTA TATCTATGTG GTGATTGAAC  100
<210> 5271
<211> 100
<212> DNA
<213> Escherichia coli K-12 MG1655
<220> yjip b4339
<222> 89-188
<223> yjip b4339 U00096 4566567..4566878
<313> 5271: 122-141
<400> 5271
TTCACTTACC TAAAGATTTA CGTGAACTGT GCGATCTCGA TAGCTTAAAA  50
CTGGAATCCG CCAGCTTTGT CGATGAAAAA TTGCGGGCGC TACACTCCGA  100
<210> 5272
<211> 100
<212> DNA
<213> Escherichia coli K-12 MG1655
<220> yjiq b4340
<222> 458-557
<223> yjiq b4340 U00096 4566927..4567487
<313> 5272: 504-523
<400> 5272
ATCCTGAATG GATACAAAAG ATTACCGGAC TTTCGGCAGA GCAAATGCAG  50
GCATTAAGGC AGCCCTTGCC TGAGCGTGAG CGCTATTCAT GGCTCAAGAG  100
<210> 5273
<211> 100
<212> DNA
<213> Escherichia coli K-12 MG1655
<220> yjiq b4340
<222> 394-493
<223> yjiq b4340 U00096 4566927..4567487
<313> 5273: 431-450
<400> 5273
CATAATGATG GATATATAAA AGGGGAGCAG CGCATTCTTC GATTGTTGTT  50
GCAGAATGGG GCGGATCCTG AATGGATACA AAAGATTACC GGACTTTCGG  100

<210> 5274
<211> 100
<212> DNA
<213> Escherichia coli K-12 MG1655
<220> yjiq b4340
<222> 348-447
<223> yjiq b4340 U00096 4566927..4567487
<313> 5274: 375-394
<400> 5274
AATGCCACAA CACAGGGAGC GAATAATGAC GATTGCAGAG CGAATTCATA 50
ATGATGGATA TATAAAAGGG GAGCAGCGCA TTCTTCGATT GTTGTTGCAG 100
<210> 5275
<211> 100
<212> DNA
<213> Escherichia coli K-12 MG1655
<220> yjir b4341
<222> 1314-1413
<223> yjir b4341 U00096 complement(4567731..4569143)
<313> 5275: 1388-1407
<400> 5275
CCGTAATTGT CTGCGCATCA ACTGCGCTTT GCCGCTCAGC GAAACCTATC 50
GCGAAGCACT GAAGCAAATT GGCGAGGCCG TGTATCGGGC AATGGAATAA 100
<210> 5276
<211> 100
<212> DNA
<213> Escherichia coli K-12 MG1655
<220> yjir b4341
<222> 1248-1347
<223> yjir b4341 U00096 complement(4567731..4569143)
<313> 5276: 1289-1308
<400> 5276
GCGGCAGCTG TGCCGCATGA AAATCCAGGT GGCGGCAGGC TCGATTTTCT 50
CAGCTTCCGG CAAATACCGT AATTGTCTGC GCATCAACTG CGCTTTGCCG 100
<210> 5277
<211> 100
<212> DNA
<213> Escherichia coli K-12 MG1655
<220> yjir b4341
<222> 1189-1288
<223> yjir b4341 U00096 complement(4567731..4569143)
<313> 5277: 1211-1230
<400> 5277
AAAGGCGGAT TTTTACTGTG GATAGAATTG CCTGAACAGG TCGATATGGT 50
CTGCGTCGCG CGGCAGCTGT GCCGCATGAA AATCCAGGTG GCGGCAGGCT 100
<210> 5278
<211> 100
<212> DNA
<213> Escherichia coli K-12 MG1655
<220> yjis b4342
<222> 66-165
<223> yjis b4342 U00096 4569320..4569484
<313> 5278: 131-150
<400> 5278
GAGGCGTTGG CGGCGGCAAA TGCAGACCCG ACGCGTGTTA CAGCAGATGA 50
GTGATGAGCG GTTGAAGGAT ATCGGGTTAC GCAGGGAGGA TGTGGAGTGA 100
<210> 5279
<211> 100
<212> DNA
<213> Escherichia coli K-12 MG1655
<220> yjis b4342
<222> 66-165
<223> yjis b4342 U00096 4569320..4569484
<313> 5279: 122-141
<400> 5279
GAGGCGTTGG CGGCGGCAAA TGCAGACCCG ACGCGTGTTA CAGCAGATGA 50
GTGATGAGCG GTTGAAGGAT ATCGGGTTAC GCAGGGAGGA TGTGGAGTGA 100
<210> 5280
<211> 100
<212> DNA
<213> Escherichia coli K-12 MG1655
<220> yjis b4342

<222> 66-165
<223> yjis b4342 U00096 4569320..4569484
<313> 5280: 108-127
<400> 5280
GAGGCGTTGG CGGCGGCAAA TGCAGACCCG ACGCGTGTTA CAGCAGATGA  50
GTGATGAGCG GTTGAAGGAT ATCGGGTTAC GCAGGGAGGA TGTGGAGTGA  100
<210> 5281
<211> 100
<212> DNA
<213> Escherichia coli K-12 MG1655
<220> yjit b4343
<222> 1434-1533
<223> yjit b4343 U00096 4569935..4571500
<313> 5281: 1469-1488
<400> 5281
CGGAAGGTTT GCTCTAAATG GAAAGCGTGC AAGCTGGGTT TGTCATCCAG  50
ATGAAACATT TATTGGTGTA CCAAAAGTCA TTTCTACACT ACCGGATATT  100
<210> 5282
<211> 100
<212> DNA
<213> Escherichia coli K-12 MG1655
<220> yjit b4343
<222> 1363-1462
<223> yjit b4343 U00096 4569935..4571500
<313> 5282: 1418-1437
<400> 5282
GGATGTCAGG ACGTTACAGT TACAGCAAAT GAAACTTATC GGATTCGCAC  50
CGGAAGAGAA CAAATCAGCA TCGGAAGGTT TGCTCTAAAT GGAAAGCGTG  100
<210> 5283
<211> 100
<212> DNA
<213> Escherichia coli K-12 MG1655
<220> yjit b4343
<222> 1340-1439
<223> yjit b4343 U00096 4569935..4571500
<313> 5283: 1372-1391
<400> 5283
GACTAAAAGC ACTACCTGTG AAGGGATGTC AGGACGTTAC AGTTACAGCA  50
AATGAAACTT ATCGGATTCG CACCGGAAGA GAACAAATCA GCATCGGAAG  100
<210> 5284
<211> 100
<212> DNA
<213> Escherichia coli K-12 MG1655
<220> yjiu b4344
<222> 1410-1509
<223> yjiu b4344 U00096 4571704..4573245
<313> 5284: 1458-1477
<400> 5284
GAAAGAAGCA TGGGCCCAAC TTACCGGACC GCTTGCAGTA CTGAAACCCG  50
ATGGCAACCG TTTTTATTTA CCGAAAGGAG CATATGACTT TTTCTTTAAT  100
<210> 5285
<211> 100
<212> DNA
<213> Escherichia coli K-12 MG1655
<220> yjiu b4344
<222> 1307-1406
<223> yjiu b4344 U00096 4571704..4573245
<313> 5285: 1327-1346
<400> 5285
TTCGTAATGG CAACGTATCC CATCGTTTAG CGAAGTTGCT GATGCTGGGG  50
GCTGGATTCA AAACCGTCAA TGCGGCAACT ATTGATATTA TCAATACATG  100
<210> 5286
<211> 100
<212> DNA
<213> Escherichia coli K-12 MG1655
<220> yjiu b4344
<222> 1260-1359
<223> yjiu b4344 U00096 4571704..4573245
<313> 5286: 1286-1305
<400> 5286
GGAATCAAAA GATCCTGAAG ATAATCAGAA CAGACGCTGG CCTCAAATTC  50

GTAATGGCAA CGTATCCCAT CGTTTAGCGA AGTTGCTGAT GCTGGGGGCT 100
<210> 5287
<211> 100
<212> DNA
<213> Escherichia coli K-12 MG1655
<220> moda b0764
<222> 675-774
<223> moda b0764 U00096 794312..795085
<313> 5287: 732-751
<400> 5287
GGAAGGGCAT AACAATGCGA CAGTGAAAGC ATTTTATGAT TATCTGAAGG 50
GACCGCAGGC AGCGGAAATC TTTAAACGTT ACGGATTTAC AATCAAGTAA 100
<210> 5288
<211> 100
<212> DNA
<213> Escherichia coli K-12 MG1655
<220> moda b0764
<222> 633-732
<223> moda b0764 U00096 794312..795085
<313> 5288: 687-706
<400> 5288
GGAAGATTCA CATAAAAAAG TGGAATATCC GGTTGCTGTT GTGGAAGGGC 50
ATAACAATGC GACAGTGAAA GCATTTTATG ATTATCTGAA GGGACCGCAG 100
<210> 5289
<211> 100
<212> DNA
<213> Escherichia coli K-12 MG1655
<220> moda b0764
<222> 567-666
<223> moda b0764 U00096 794312..795085
<313> 5289: 629-648
<400> 5289
TCTGGGCATT GTCTACGGTT CTGACGCAGT TGCCAGCAAA GGGGTAAAAG 50
TGGTTGCCAC CTTCCCGGAA GATTCACATA AAAAAGTGGA ATATCCGGTT 100
<210> 5290
<211> 100
<212> DNA
<213> Escherichia coli K-12 MG1655
<220> modb b0765
<222> 562-661
<223> modb b0765 U00096 795085..795774
<313> 5290: 593-612
<400> 5290
CTGATCCAGA CCCCCGGCGG GGAAAGTGGA GCGGCGAGAC TGTGCATTAT 50
TTCTATTGCG CTGGCGATGA TCTCCCTGTT GATTTCAGAA TGGCTGGCCA 100
<210> 5291
<211> 100
<212> DNA
<213> Escherichia coli K-12 MG1655
<220> modb b0765
<222> 515-614
<223> modb b0765 U00096 795085..795774
<313> 5291: 552-571
<400> 5291
CGAACATTCC TGGTGAAACG CGAACCATTC CTTCTGCCAT GTATACCCTG 50
ATCCAGACCC CCGGCGGGGA AAGTGGAGCG GCGAGACTGT GCATTATTTC 100
<210> 5292
<211> 100
<212> DNA
<213> Escherichia coli K-12 MG1655
<220> modb b0765
<222> 453-552
<223> modb b0765 U00096 795085..795774
<313> 5292: 507-526
<400> 5292
TGGTACGGTA CTGGCTTTTG CTCGTTCTCT CGGTGAGTTT GGTGCAACCA 50
TCACCTTTGT GTCGAACATT CCTGGTGAAA CGCGAACCAT TCCTTCTGCC 100
<210> 5293
<211> 100
<212> DNA
<213> Escherichia coli K-12 MG1655

<220> yjiw b4348
<222> 300-399
<223> yjiw b4348 U00096 complement(4577069..4577467)
<313> 5293: 360-379
<400> 5293
GATGCAGTCG CTACGCCAGG TGTGCAAACT GTCGGCGCGT AAACAAAAGC  50
AGGTGCAGGC GTTTATTGGA GTGATTGCCG GTAAACAGAA AGTCGCGTAA  100
<210> 5294
<211> 100
<212> DNA
<213> Escherichia coli K-12 MG1655
<220> yjiw b4348
<222> 282-381
<223> yjiw b4348 U00096 complement(4577069..4577467)
<313> 5294: 310-329
<400> 5294
CGCCGAGGAG AGCGAACTGA TGCAGTCGCT ACGCCAGGTG TGCAAACTGT  50
CGGCGCGTAA ACAAAAGCAG GTGCAGGCGT TTATTGGAGT GATTGCCGGT  100
<210> 5295
<211> 100
<212> DNA
<213> Escherichia coli K-12 MG1655
<220> yjiw b4348
<222> 207-306
<223> yjiw b4348 U00096 complement(4577069..4577467)
<313> 5295: 258-277
<400> 5295
CGGTTTTGCC ACTGGCACGG CGGTAGATGT CAAAGTGATG GAAGGCTGTA  50
TTGTCCTCAC CGCCCAACCA CCCGCCGCCG AGGAGAGCGA ACTGATGCAG  100
<210> 5296
<211> 100
<212> DNA
<213> Escherichia coli K-12 MG1655
<220> modc b0766
<222> 920-1019
<223> modc b0766 U00096 795777..796835
<313> 5296: 942-961
<400> 5296
ACGGCCAGGT GGAAGTGGAA CTGGAAGTCG GCGGTAAAAC GCTGTGGGCG  50
CGTATCAGCC CGTGGGCCAG GGATGAACTG GCGATCAAAC CTGGCCTGTG  100
<210> 5297
<211> 100
<212> DNA
<213> Escherichia coli K-12 MG1655
<220> modc b0766
<222> 853-952
<223> modc b0766 U00096 795777..796835
<313> 5297: 873-892
<400> 5297
CAACCGCCGC AGCAAACCAG CATTCGTAAC GTATTGCGGG CAAAAGTTGT  50
TAATAGTTAT GACGACAACG GCCAGGTGGA AGTGGAACTG GAAGTCGGCG  100
<210> 5298
<211> 100
<212> DNA
<213> Escherichia coli K-12 MG1655
<220> modc b0766
<222> 811-910
<223> modc b0766 U00096 795777..796835
<313> 5298: 842-861
<400> 5298
CTACGTATCC GCATTCAGGC TTCCGATGTT TCTCTGGTTT TACAACCGCC  50
GCAGCAAACC AGCATTCGTA ACGTATTGCG GGCAAAAGTT GTTAATAGTT  100
<210> 5299
<211> 100
<212> DNA
<213> Escherichia coli K-12 MG1655
<220> yjix b4354
<222> 66-165
<223> yjix b4354 U00096 complement(4586446..4586649)
<313> 5299: 115-134
<400> 5299

TCCAGACTAC GACAACTACG TCGAGCATAT GAAGACCAAC CATCCCGACA 50
AGCCGTACAT GAGCTATGAA GAATTCTTCC GCGAACGCCA GAACGCGCGC 100
<210> 5300
<211> 100
<212> DNA
<213> Escherichia coli K-12 MG1655
<220> yjix b4354
<222> 30-129
<223> yjix b4354 U00096 complement(4586446..4586649)
<313> 5300: 75-94
<400> 5300
ATATCTCGGC CAGGCGGCGA AGATGTTGAT TGGCATTCCA GACTACGACA 50
ACTACGTCGA GCATATGAAG ACCAACCATC CCGACAAGCC GTACATGAGC 100
<210> 5301
<211> 100
<212> DNA
<213> Escherichia coli K-12 MG1655
<220> yjix b4354
<222> 7-106
<223> yjix b4354 U00096 complement(4586446..4586649)
<313> 5301: 29-48
<400> 5301
GGTAACTTAG GACAGGCAAA AAAATATCTC GGCCAGGCGG CGAAGATGTT 50
GATTGGCATT CCAGACTACG ACAACTACGT CGAGCATATG AAGACCAACC 100
<210> 5302
<211> 100
<212> DNA
<213> Escherichia coli K-12 MG1655
<220> yjiy b4355
<222> 2063-2162
<223> yjiy b4355 U00096 complement(4586699..4588864)
<313> 5302: 2083-2102
<400> 5302
TCAAAACCTG GCTTGCGGTG CGTAACAGCG ACAAACGTAC TGACAAAGAA 50
ACACCGTACG TTCCAATCCC GGAAGGCGGC GTGAAGATCT CTTCGCACCA 100
<210> 5303
<211> 100
<212> DNA
<213> Escherichia coli K-12 MG1655
<220> yjiy b4355
<222> 1992-2091
<223> yjiy b4355 U00096 complement(4586699..4588864)
<313> 5303: 2058-2077
<400> 5303
GAACAACTAC ACCAACGCAG GTCTGAGTAT TCTGTTCCTG ATTGTGGTGT 50
ACAGCATCAT CTTCTACGGT TTCAAAACCT GGCTTGCGGT GCGTAACAGC 100
<210> 5304
<211> 100
<212> DNA
<213> Escherichia coli K-12 MG1655
<220> yjiy b4355
<222> 1943-2042
<223> yjiy b4355 U00096 complement(4586699..4588864)
<313> 5304: 1985-2004
<400> 5304
GTACTGACCT GACGGCGCAG CAGATTGCCA ATATGAACCA CATCGTTGTG 50
AACAACTACA CCAACGCAGG TCTGAGTATT CTGTTCCTGA TTGTGGTGTA 100
<210> 5305
<211> 100
<212> DNA
<213> Escherichia coli K-12 MG1655
<220> mode modE
<222> 690-789
<223> mode modE U00096 complement(793079..793867)
<313> 5305: 745-764
<400> 5305
CGCCACAGTG CCGGTAAATG AAGCGACTTC TCTTCAGCAA GGACAGAATG 50
TCACGGCCTA CTTTAATGCC GACAGCGTGA TTATCGCCAC GCTGTGCTAA 100
<210> 5306
<211> 100
<212> DNA

<213> Escherichia coli K-12 MG1655
<220> mode modE
<222> 681-780
<223> mode modE U00096 complement(793079..793867)
<313> 5306: 702-721
<400> 5306
AACACTGTGC GCCACAGTGC CGGTAAATGA AGCGACTTCT CTTCAGCAAG 50
GACAGAATGT CACGGCCTAC TTTAATGCCG ACAGCGTGAT TATCGCCACG 100
<210> 5307
<211> 100
<212> DNA
<213> Escherichia coli K-12 MG1655
<220> mode modE
<222> 582-681
<223> mode modE U00096 complement(793079..793867)
<313> 5307: 639-658
<400> 5307
GGTCGCGCAA AACGCTGACA ACCAATTACC GGGTATTATT AGTCATATTG 50
AGCGCGGCGC AGAGCAGTGC GAAGTATTAA TGGCGCTACC CGACGGGCAA 100
<210> 5308
<211> 100
<212> DNA
<213> Escherichia coli K-12 MG1655
<220> yjiz b4357
<222> 1263-1362
<223> yjiz b4357 U00096 complement(4590931..4592292)
<313> 5308: 1325-1344
<400> 5308
CCGTCTGGCA CTAATCATCT GCGGTTGCGT CACCGCAGCG GGGGCACTGG 50
CGTACATCTT CCTGGTTCGT CAGCCGATCA ACGACCCACG TAAAGATTAA 100
<210> 5309
<211> 100
<212> DNA
<213> Escherichia coli K-12 MG1655
<220> yjiz b4357
<222> 1234-1333
<223> yjiz b4357 U00096 complement(4590931..4592292)
<313> 5309: 1261-1280
<400> 5309
GGTTTTATTG TTGATACCAC CCACTCATTC CGTCTGGCAC TAATCATCTG 50
CGGTTGCGTC ACCGCAGCGG GGGCACTGGC GTACATCTTC CTGGTTCGTC 100
<210> 5310
<211> 100
<212> DNA
<213> Escherichia coli K-12 MG1655
<220> yjiz b4357
<222> 1182-1281
<223> yjiz b4357 U00096 complement(4590931..4592292)
<313> 5310: 1216-1235
<400> 5310
TATCCAGAAC TTTGCCAGCT TCATCTGCGC CTCTTTTGCG CCGATCATTA 50
CTGGTTTTAT TGTTGATACC ACCCACTCAT TCCGTCTGGC ACTAATCATC 100
<210> 5311
<211> 100
<212> DNA
<213> Escherichia coli K-12 MG1655
<220> modf b0761
<222> 1345-1444
<223> modf b0761 U00096 complement(791539..793011)
<313> 5311: 1393-1412
<400> 5311
AGCGAAGGTG AAACGCAATT GTTGTTTGTT TCGCACCACG CTGAAGATGC 50
GCCTGCCTGT ATTACCCATC GTCTTGAGTT CGTGCCGGAC GGTGGACTCT 100
<210> 5312
<211> 100
<212> DNA
<213> Escherichia coli K-12 MG1655
<220> modf b0761
<222> 1266-1365
<223> modf b0761 U00096 complement(791539..793011)
<313> 5312: 1310-1329

<400> 5312
GCTTATTCTC GATGAACCAC TACAGGGGCT TGATCCGCTG AATCGCCAGC 50
TTATCCGCCG TTTTGTTGAT GTGCTGATTA GCGAAGGTGA AACGCAATTG 100
<210> 5313
<211> 100
<212> DNA
<213> Escherichia coli K-12 MG1655
<220> modf b0761
<222> 1248-1347
<223> modf b0761 U00096 complement(791539..793011)
<313> 5313: 1277-1296
<400> 5313
GGTGAAACAT CCGACGTTGC TTATTCTCGA TGAACCACTA CAGGGGCTTG 50
ATCCGCTGAA TCGCCAGCTT ATCCGCCGTT TTGTTGATGT GCTGATTAGC 100
<210> 5314
<211> 100
<212> DNA
<213> Escherichia coli K-12 MG1655
<220> mgta b4243
<222> 2544-2643
<223> mgta b4243 U00096 4465199..4467895
<313> 5314: 2590-2609
<400> 5314
GATCGCATTG CCGTTTTCAC CGCTGGCCAG TTATCTGCAA TTACAGGCGC 50
TGCCGTTAAG CTATTTCCCG TGGCTGGTTG CGATTCTGGC AGGGTATATG 100
<210> 5315
<211> 100
<212> DNA
<213> Escherichia coli K-12 MG1655
<220> mgta b4243
<222> 2418-2517
<223> mgta b4243 U00096 4465199..4467895
<313> 5315: 2482-2501
<400> 5315
GTTTGTGGTG GGCTTACTGT CGCAAACGCT GATTGTGCAT ATGATCCGCA 50
CCCGCCGTGT GCCGTTTATT CAGAGCTGTG CATCGTGGCC GTTAATGATC 100
<210> 5316
<211> 100
<212> DNA
<213> Escherichia coli K-12 MG1655
<220> mgta b4243
<222> 2355-2454
<223> mgta b4243 U00096 4465199..4467895
<313> 5316: 2377-2396
<400> 5316
CCTGATGTGG TGGGTATTCC ATGCCAACAC GCCGGAAACG CAAACGCTGT 50
TCCAGTCGGG ATGGTTTGTG GTGGGCTTAC TGTCGCAAAC GCTGATTGTG 100
<210> 5317
<211> 100
<212> DNA
<213> Escherichia coli K-12 MG1655
<220> yjja b4361
<222> 399-498
<223> yjja b4361 U00096 complement(4597264..4597761)
<313> 5317: 479-498
<400> 5317
ACTCAATCTG GATAACATCG GAACAACTCC GCTGGCAGAA AAGGTGAAAA 50
CCAAAGCCTG CGATCTGGTG TTAAAACAGG GGCTGAACTT CATTTCCTGA 100
<210> 5318
<211> 100
<212> DNA
<213> Escherichia coli K-12 MG1655
<220> yjja b4361
<222> 372-471
<223> yjja b4361 U00096 complement(4597264..4597761)
<313> 5318: 436-455
<400> 5318
TTTGCTGAAA ACCAAAGATG GTCAGCAACT CAATCTGGAT AACATCGGAA 50
CAACTCCGCT GGCAGAAAAG GTGAAAACCA AAGCCTGCGA TCTGGTGTTA 100
<210> 5319
<211> 100

<212> DNA
<213> Escherichia coli K-12 MG1655
<220> yjja b4361
<222> 332-431
<223> yjja b4361 U00096 complement(4597264..4597761)
<313> 5319: 363-382
<400> 5319
AGCAAAAAGA AGACACCAAC TATCTGGACG GCATTCAGGG TTTGCTGAAA 50
ACCAAAGATG GTCAGCAACT CAATCTGGAT AACATCGGAA CAACTCCGCT 100
<210> 5320
<211> 100
<212> DNA
<213> Escherichia coli K-12 MG1655
<220> yjjb b4364
<222> 228-327
<223> yjjb b4364 U00096 complement(4599193..4599519)
<313> 5320: 289-308
<400> 5320
GTTAACCAAC TTTCTTACAG CTTCATCGAT TGTTGGTGCG TTATCCATCG 50
GTCTTTCCAT TCCTGGATTA TGGTTGTACC GCAAGCGCCC TCGCGTATAA 100
<210> 5321
<211> 100
<212> DNA
<213> Escherichia coli K-12 MG1655
<220> yjjb b4364
<222> 228-327
<223> yjjb b4364 U00096 complement(4599193..4599519)
<313> 5321: 284-303
<400> 5321
GTTAACCAAC TTTCTTACAG CTTCATCGAT TGTTGGTGCG TTATCCATCG 50
GTCTTTCCAT TCCTGGATTA TGGTTGTACC GCAAGCGCCC TCGCGTATAA 100
<210> 5322
<211> 100
<212> DNA
<213> Escherichia coli K-12 MG1655
<220> yjjb b4364
<222> 183-282
<223> yjjb b4364 U00096 complement(4599193..4599519)
<313> 5322: 228-247
<400> 5322
AAAAATCAGC CAGTTAGGTT ACAGCGAACC GTTGATGATT ACCCTGTTAA 50
CCAACTTTCT TACAGCTTCA TCGATTGTTG GTGCGTTATC CATCGGTCTT 100
<210> 5323
<211> 100
<212> DNA
<213> Escherichia coli K-12 MG1655
<220> yjjg b4375
<222> 579-678
<223> yjjg b4375 U00096 4606215..4606892
<313> 5323: 651-670
<400> 5323
CTGGCTGAAT GCACACCATC GCGAGCAACC AGAAGGCATC GCGCCCACCT 50
GGACCGTTTC TTCGTTGCAC GAACTGGAGC AGCTCCTGTG TAAACACTGA 100
<210> 5324
<211> 100
<212> DNA
<213> Escherichia coli K-12 MG1655
<220> yjjg b4375
<222> 559-658
<223> yjjg b4375 U00096 4606215..4606892
<313> 5324: 601-620
<400> 5324
AACGCCGGGC TTGCGACCTG CTGGCTGAAT GCACACCATC GCGAGCAACC 50
AGAAGGCATC GCGCCCACCT GGACCGTTTC TTCGTTGCAC GAACTGGAGC 100
<210> 5325
<211> 100
<212> DNA
<213> Escherichia coli K-12 MG1655
<220> yjjg b4375
<222> 480-579
<223> yjjg b4375 U00096 4606215..4606892

<313> 5325: 522-541
<400> 5325
GGAACAGGCG GGCAATCCTG ACCGTTCACG CGTGCTGATG GTTGGCGACA 50
CTGCCGAGTC CGATATTCTC GGTGGCATCA ACGCCGGGCT TGCGACCTGC 100
<210> 5326
<211> 100
<212> DNA
<213> Escherichia coli K-12 MG1655
<220> yjji b4381
<222> 1452-1551
<223> yjji b4381 U00096 complement(4613084..4614634)
<313> 5326: 1514-1533
<400> 5326
CACCACCTGG CTGGGCGAAG AAGCCGCACG CAACACTCGT ATTCTGGAAC 50
GCCAGCCGCG CGTGATAAGC CATGAACAGC AGATGCGCTT TAGTCAGTAA 100
<210> 5327
<211> 100
<212> DNA
<213> Escherichia coli K-12 MG1655
<220> yjji b4381
<222> 1378-1477
<223> yjji b4381 U00096 complement(4613084..4614634)
<313> 5327: 1435-1454
<400> 5327
CTGGTTCGCG TTACCGGTTA TATGGTGCGT TTGTCGGATT TAGAAAAATA 50
TCGCGCCGAA GGTTCACGCA CCAACACCAC CTGGCTGGGC GAAGAAGCCG 100
<210> 5328
<211> 100
<212> DNA
<213> Escherichia coli K-12 MG1655
<220> yjji b4381
<222> 1256-1355
<223> yjji b4381 U00096 complement(4613084..4614634)
<313> 5328: 1302-1321
<400> 5328
TCAGCGACAT TCTGACGCTC GACGAAACCA TCAAACGTAA CCCGCAGGCA 50
CTGGTACAGC TTTGCCTCGG TGCCTTTAAA GCCGGAATGC GTGAATTTAC 100
<210> 5329
<211> 100
<212> DNA
<213> Escherichia coli K-12 MG1655
<220> yjjj b4386
<222> 1233-1332
<223> yjjj b4386 U00096 4619338..4620669
<313> 5329: 1286-1305
<400> 5329
CAGAGATCCG CGTATCAGCG AGGCGTTTCG CCACATTGCG CAAGAAATGC 50
CGGAAAAAAT CCGGCAAATC GAAGAGAAAG TTGCCCGCAT GGGCGGGTAA 100
<210> 5330
<211> 100
<212> DNA
<213> Escherichia coli K-12 MG1655
<220> yjjj b4386
<222> 1170-1269
<223> yjjj b4386 U00096 4619338..4620669
<313> 5330: 1232-1251
<400> 5330
CGTCAGTAAA AGCGCTTGGT TAACGGCGAT CCCGCTGGCG CAGCAGTTCT 50
GGCAAACGGT CGCCAGAGAT CCGCGTATCA GCGAGGCGTT TCGCCACATT 100
<210> 5331
<211> 100
<212> DNA
<213> Escherichia coli K-12 MG1655
<220> yjjj b4386
<222> 1109-1208
<223> yjjj b4386 U00096 4619338..4620669
<313> 5331: 1142-1161
<400> 5331
ATGCACCAAA CAGCGCTGGA ATGCTGCGTG ATGCTGCCAT TGAGGTGAAG 50
TTTGATCTTA ACGTCAGTAA AAGCGCTTGG TTAACGGCGA TCCCGCTGGC 100
<210> 5332

<211> 100
<212> DNA
<213> Escherichia coli K-12 MG1655
<220> yjjk b4392
<222> 1569-1668
<223> yjjk b4392 U00096 complement(4626424..4628091)
<313> 5332: 1646-1665
<400> 5332
CGAAGGTAAC TTTACCGAGT ACGAAGAGTA CAAGAAACGC ACGCTGGGCG  50
CAGACGCGCT GGAGCCGAAG CGTATCAAGT ACAAGCGTAT TGCGAAGTAA  100
<210> 5333
<211> 100
<212> DNA
<213> Escherichia coli K-12 MG1655
<220> yjjk b4392
<222> 1559-1658
<223> yjjk b4392 U00096 complement(4626424..4628091)
<313> 5333: 1590-1609
<400> 5333
TTGAGTTCTT CGAAGGTAAC TTTACCGAGT ACGAAGAGTA CAAGAAACGC  50
ACGCTGGGCG CAGACGCGCT GGAGCCGAAG CGTATCAAGT ACAAGCGTAT  100
<210> 5334
<211> 100
<212> DNA
<213> Escherichia coli K-12 MG1655
<220> yjjk b4392
<222> 1458-1557
<223> yjjk b4392 U00096 complement(4626424..4628091)
<313> 5334: 1519-1538
<400> 5334
CCTGCTGGAG TTCCCGGGCT GTGCGATGGT TATCTCGCAC GACCGTTGGT  50
TCCTCGACCG TATCGCCACG CACATTCTGG ATTACCAGGA TGAAGGTAAA  100
<210> 5335
<211> 100
<212> DNA
<213> Escherichia coli K-12 MG1655
<220> lysa b2839
<222> 1164-1263
<223> lysa b2839 U00096 complement(2975659..2976921)
<313> 5335: 1225-1244
<400> 5335
CCGTCCGCTG TTACCAGAAG TTCTGTTTGA TAATGGTCAG GCGCGGTTGA  50
TTCGCCGTCG CCAGACCATC GAAGAATTAC TGGCGCTGGA ATTGCTTTAA  100
<210> 5336
<211> 100
<212> DNA
<213> Escherichia coli K-12 MG1655
<220> lysa b2839
<222> 1119-1218
<223> lysa b2839 U00096 complement(2975659..2976921)
<313> 5336: 1179-1198
<400> 5336
TGATACAGGG GCATATGGCG CATCAATGTC ATCCAACTAC AATAGCCGTC  50
CGCTGTTACC AGAAGTTCTG TTTGATAATG GTCAGGCGCG GTTGATTCGC  100
<210> 5337
<211> 100
<212> DNA
<213> Escherichia coli K-12 MG1655
<220> lysa b2839
<222> 1080-1179
<223> lysa b2839 U00096 complement(2975659..2976921)
<313> 5337: 1127-1146
<400> 5337
CTTGCCGGAA GTGAAGGCAG GTGATTATCT GGTACTGCAT GATACAGGGG  50
CATATGGCGC ATCAATGTCA TCCAACTACA ATAGCCGTCC GCTGTTACCA  100
<210> 5338
<211> 100
<212> DNA
<213> Escherichia coli K-12 MG1655
<220> yjjm b4358
<222> 684-783

<223> yjjm b4358 U00096 complement(4592507..4593313)
<313> 5338: 723-742
<400> 5338
CATTCTCAGC GCTCTGATTT GCCGCAGTGA CCTTGATGCC ACCCTTGCGC 50
TCCGTAACCA TTTGAATTCA GCTAAACAAT CAATGATTCG ATCCATTAAT 100
<210> 5339
<211> 100
<212> DNA
<213> Escherichia coli K-12 MG1655
<220> yjjm b4358
<222> 585-684
<223> yjjm b4358 U00096 complement(4592507..4593313)
<313> 5339: 644-663
<400> 5339
ATCGCTTGAG ATCATCTCAG TCATCTTTCA TTTTCACTAT CAATGGGACG 50
AAAGCGATCT TAAACAGCGC AATATCATTG CCGTCGATGA GCATATGACC 100
<210> 5340
<211> 100
<212> DNA
<213> Escherichia coli K-12 MG1655
<220> yjjm b4358
<222> 470-569
<223> yjjm b4358 U00096 complement(4592507..4593313)
<313> 5340: 529-548
<400> 5340
AACGTCATCG GCTACTGCGT GACAACATCG GCAATAGCTT TCGCATGTTC 50
TCGCAACTTG ACAGAGATTT TCACTCCTTA CTGCTTTCAG CTGCCGACAA 100
<210> 5341
<211> 100
<212> DNA
<213> Escherichia coli K-12 MG1655
<220> yjjn b4359
<222> 939-1038
<223> yjjn b4359 U00096 4593544..4594581
<313> 5341: 977-996
<400> 5341
GATGTTAACC CATCGCTATC CGTTCGCCAC GCTGGCAGAA ACCTACGAGC 50
GCGATGTGAT TAACAATCGT GAGTTAATTA AAGGCGTAAT TACTTTCTGA 100
<210> 5342
<211> 100
<212> DNA
<213> Escherichia coli K-12 MG1655
<220> yjjn b4359
<222> 912-1011
<223> yjjn b4359 U00096 4593544..4594581
<313> 5342: 938-957
<400> 5342
GGCGGAAGGA AAAATCACTG CTGACATGAT GTTAACCCAT CGCTATCCGT 50
TCGCCACGCT GGCAGAAACC TACGAGCGCG ATGTGATTAA CAATCGTGAG 100
<210> 5343
<211> 100
<212> DNA
<213> Escherichia coli K-12 MG1655
<220> yjjn b4359
<222> 841-940
<223> yjjn b4359 U00096 4593544..4594581
<313> 5343: 877-896
<400> 5343
AAAAAAGAAA CGACGATGAT GGGCAGCCGC AACGCCACGC CGGAAGATTT 50
TGCTAAAGTC GGTCGACTGA TGGCGGAAGG AAAAATCACT GCTGACATGA 100
<210> 5344
<211> 100
<212> DNA
<213> Escherichia coli K-12 MG1655
<220> lysc b4025
<222> 1251-1350
<223> lysc b4025 U00096 complement(4229463..4230812)
<313> 5344: 1310-1329
<400> 5344
GATTTGTTAT GGCGCATCCA GCCATAACCT GTGCTTCCTG GTGCCCGGCG 50
AAGATGCCGA GCAGGTGGTG CAAAAACTGC ATAGTAATTT GTTTGAGTAA 100

<210> 5345
<211> 100
<212> DNA
<213> Escherichia coli K-12 MG1655
<220> lysc b4025
<222> 1193-1292
<223> lysc b4025 U00096 complement(4229463..4230812)
<313> 5345: 1237-1256
<400> 5345
AAGCCTGCGG CGTTGGCAAA GAGGTATTCG GCGTACTGGA ACCGTTCAAC 50
ATTCGCATGA TTTGTTATGG CGCATCCAGC CATAACCTGT GCTTCCTGGT 100
<210> 5346
<211> 100
<212> DNA
<213> Escherichia coli K-12 MG1655
<220> lysc b4025
<222> 1056-1155
<223> lysc b4025 U00096 complement(4229463..4230812)
<313> 5346: 1105-1124
<400> 5346
CCTTGATACC ACCGGTTCAA CCTCCACTGG CGATACGTTG CTGACGCAAT 50
CTCTGCTGAT GGAGCTTTCC GCACTGTGTC GGGTGGAGGT GGAAGAAGGT 100
<210> 5347
<211> 100
<212> DNA
<213> Escherichia coli K-12 MG1655
<220> yjjp b4365
<222> 735-834
<223> yjjp b4365 U00096 complement(4599657..4600490)
<313> 5347: 811-830
<400> 5347
CGGACTGGCA CGCTGGGCGA TCGCCAGTCT GCTGACACTG GCTACCTGCG 50
TCGGCGTAGT GATGGCACTG ACGATTTGGG GGCTACGCGG ATGGGTGTGA 100
<210> 5348
<211> 100
<212> DNA
<213> Escherichia coli K-12 MG1655
<220> yjjp b4365
<222> 694-793
<223> yjjp b4365 U00096 complement(4599657..4600490)
<313> 5348: 721-740
<400> 5348
ATTAATGCCG TCGCCGATAT GTTTAAAGGC CACATCAATA CCGGACTGGC 50
ACGCTGGGCG ATCGCCAGTC TGCTGACACT GGCTACCTGC GTCGGCGTAG 100
<210> 5349
<211> 100
<212> DNA
<213> Escherichia coli K-12 MG1655
<220> yjjp b4365
<222> 628-727
<223> yjjp b4365 U00096 complement(4599657..4600490)
<313> 5349: 658-677
<400> 5349
TTCAGCAATA CCCCCACCAT TGCGATGGCC GCCAGCGTTC TGCTGCTAGT 50
GCCGGGCTTT CCGTTGATTA ATGCCGTCGC CGATATGTTT AAAGGCCACA 100
<210> 5350
<211> 100
<212> DNA
<213> Escherichia coli K-12 MG1655
<220> yjjq b4366
<222> 627-726
<223> yjjq b4366 U00096 4601046..4601771
<313> 5350: 694-713
<400> 5350
TAGCGCGCAG GGTGCAAGAG AACTTAACTT GCCTTCTGTT TATGGAGATG 50
CCGCAGAATG GAACACAGCC GAATTAAGAA GAGAAATGTC GCACTCATAG 100
<210> 5351
<211> 100
<212> DNA
<213> Escherichia coli K-12 MG1655
<220> yjjq b4366

<222> 622-721
<223> yjjq b4366 U00096 4601046..4601771
<313> 5351: 652-671
<400> 5351
ATAAATAGCG CGCAGGGTGC AAGAGAACTT AACTTGCCTT CTGTTTATGG 50
AGATGCCGCA GAATGGAACA CAGCCGAATT AAGAAGAGAA ATGTCGCACT 100
<210> 5352
<211> 100
<212> DNA
<213> Escherichia coli K-12 MG1655
<220> yjjq b4366
<222> 453-552
<223> yjjq b4366 U00096 4601046..4601771
<313> 5352: 496-515
<400> 5352
CGTCATTCTC ACTCTTTCAG AGCGCAAGGT ACTGCGGCTA TTAGGTAAAG 50
GATGGGGCAT CAACCAGATA GCTTCATTGC TTAAGAAAAG TAATAAAACT 100
<210> 5353
<211> 100
<212> DNA
<213> Escherichia coli K-12 MG1655
<220> bira b3974
<222> 867-966
<223> bira b3974 U00096 4170661..4171626
<313> 5353: 932-951
<400> 5353
ACGCGGAATA GACAAACAGG GGGCTTTATT ACTTGAGCAG GATGGAATAA 50
TAAAACCCTG GATGGGCGGT GAAATATCCC TGCGTAGTGC AGAAAAATAA 100
<210> 5354
<211> 100
<212> DNA
<213> Escherichia coli K-12 MG1655
<220> bira b3974
<222> 838-937
<223> bira b3974 U00096 4170661..4171626
<313> 5354: 886-905
<400> 5354
ATTGGTGATA AAGAAATATT TGGCATTTCA CGCGGAATAG ACAAACAGGG 50
GGCTTTATTA CTTGAGCAGG ATGGAATAAT AAAACCCTGG ATGGGCGGTG 100
<210> 5355
<211> 100
<212> DNA
<213> Escherichia coli K-12 MG1655
<220> bira b3974
<222> 753-852
<223> bira b3974 U00096 4170661..4171626
<313> 5355: 791-810
<400> 5355
ACTCTTCGAA CAAGAAGGAT TGGCACCTTA TCTGTCGCGC TGGGAAAAGC 50
TGGATAATTT TATTAATCGC CCAGTGAAAC TTATCATTGG TGATAAAGAA 100
<210> 5356
<211> 100
<212> DNA
<213> Escherichia coli K-12 MG1655
<220> yjjt b4372
<222> 933-1032
<223> yjjt b4372 U00096 complement(4604238..4605269)
<313> 5356: 1012-1031
<400> 5356
CGTGCTGGAT GAGACATTTG GCTTCCACGA AGTGATCGCG CAAACAGGGC 50
GCTTCAAGGT GTATCGCGCC ATTATGACCC GCCAGGCGAA GAAAGGTTAA 100
<210> 5357
<211> 100
<212> DNA
<213> Escherichia coli K-12 MG1655
<220> yjjt b4372
<222> 788-887
<223> yjjt b4372 U00096 complement(4604238..4605269)
<313> 5357: 849-868
<400> 5357
ATATGATCAT CTCCAACCCG CCGTTCCACG ATGGGATGCA AACCAGCCTG 50

GATGCGGCGC AAACGCTGAT TCGCGGTGCG GTGCGTCATC TTAATAGCGG 100
<210> 5358
<211> 100
<212> DNA
<213> Escherichia coli K-12 MG1655
<220> yjjt b4372
<222> 720-819
<223> yjjt b4372 U00096 complement(4604238..4605269)
<313> 5358: 762-781
<400> 5358
TGCGGCCAAC GGTGTTGAAG GTGAAGTCTT TGCCAGCAAC GTCTTTTCCG 50
AGGTGAAAGG TCGTTTTGAT ATGATCATCT CCAACCCGCC GTTCCACGAT 100
<210> 5359
<211> 100
<212> DNA
<213> Escherichia coli K-12 MG1655
<220> yjju b4378
<222> 975-1074
<223> yjju b4378 U00096 4609980..4611053
<313> 5359: 1055-1074
<400> 5359
CATTCCGCCT GCGCCAGTCG CCAACGATAC GCTGGTTGCC GAAGTGAGCG 50
ACGCTCCGCA GGCGAACGAC CCGACATTTA ACAATGAGGA TCTGGCTTGA 100
<210> 5360
<211> 100
<212> DNA
<213> Escherichia coli K-12 MG1655
<220> yjju b4378
<222> 963-1062
<223> yjju b4378 U00096 4609980..4611053
<313> 5360: 1002-1021
<400> 5360
GGAATCGATT GTCATTCCGC CTGCGCCAGT CGCCAACGAT ACGCTGGTTG 50
CCGAAGTGAG CGACGCTCCG CAGGCGAACG ACCCGACATT TAACAATGAG 100
<210> 5361
<211> 100
<212> DNA
<213> Escherichia coli K-12 MG1655
<220> yjju b4378
<222> 877-976
<223> yjju b4378 U00096 4609980..4611053
<313> 5361: 920-939
<400> 5361
TGCGGTCGTT ATTTCCTCGC CACGGTTGGC AAGCTATTAA CTGAAAAAGC 50
GCCGCTTACC CGCCATCTGG TGCCAGTGGT GACGCCGGAA TCGATTGTCA 100
<210> 5362
<211> 100
<212> DNA
<213> Escherichia coli K-12 MG1655
<220> moea b0828
<222> 1137-1236
<223> moea b0828 U00096 complement(864352..865587)
<313> 5362: 1184-1203
<400> 5362
CGGCAACTGC TTTATCGTGC TGGAACGCGA TCGCGGCAAT GTGGAAGTGG 50
GCGAATGGGT GGAAGTAGAA CCGTTTAACG CGTTGTTCGG AGGCCTGTAA 100
<210> 5363
<211> 100
<212> DNA
<213> Escherichia coli K-12 MG1655
<220> moea b0828
<222> 1094-1193
<223> moea b0828 U00096 complement(864352..865587)
<313> 5363: 1126-1145
<400> 5363
CCACCGGACA TCAGGGTTCA CATATATTTA GCTCCTTTAG CCTCGGCAAC 50
TGCTTTATCG TGCTGGAACG CGATCGCGGC AATGTGGAAG TGGGCGAATG 100
<210> 5364
<211> 100
<212> DNA
<213> Escherichia coli K-12 MG1655

<220> moea b0828
<222> 1041-1140
<223> moea b0828 U00096 complement(864352..865587)
<313> 5364: 1091-1110
<400> 5364
TTTCCAGCGC GGCGTGCTGC AACGCAACGC CGATGGCGAA CTGGAAGTGA 50
CGACCACCGG ACATCAGGGT TCACATATAT TTAGCTCCTT TAGCCTCGGC 100
<210> 5365
<211> 100
<212> DNA
<213> Escherichia coli K-12 MG1655
<220> yjjv b4379
<222> 523-622
<223> yjjv b4379 U00096 4611194..4611829
<313> 5365: 556-575
<400> 5365
CCGGAGCAGG CTGCCCGTGT GTTCGCCGTG CTTTGCGAGT TGCGCCGGGA 50
ACCGGCGGAT GAGATTGCGC AAGCGTTGCT TAATAACACG TATACGTTGT 100
<210> 5366
<211> 100
<212> DNA
<213> Escherichia coli K-12 MG1655
<220> yjjv b4379
<222> 453-552
<223> yjjv b4379 U00096 4611194..4611829
<313> 5366: 501-520
<400> 5366
GGCATCGTTA TTGCTGGAAA CCGACGCGCC GGATATGCCG CTCAACGGTT 50
TTCAGGGGCA GCCTAACCGC CCGGAGCAGG CTGCCCGTGT GTTCGCCGTG 100
<210> 5367
<211> 100
<212> DNA
<213> Escherichia coli K-12 MG1655
<220> yjjv b4379
<222> 404-503
<223> yjjv b4379 U00096 4611194..4611829
<313> 5367: 468-487
<400> 5367
CCTATCCACG CGCCAGTAAA ACCCGCGATG TCATCGCAAA ATTACCGCTG 50
GCATCGTTAT TGCTGGAAAC CGACGCGCCG GATATGCCGC TCAACGGTTT 100
<210> 5368
<211> 100
<212> DNA
<213> Escherichia coli K-12 MG1655
<220> moeb b0827
<222> 651-750
<223> moeb b0827 U00096 complement(863603..864352)
<313> 5368: 726-745
<400> 5368
ACCGGCCAGC GGGAAAATCG TCATGTACGA TGCGATGACC TGTCAGTTTC 50
GCGAAATGAA ACTGATGCGT AATCCGGGGT GTGAGGTGTG TGGGCAGTAA 100
<210> 5369
<211> 100
<212> DNA
<213> Escherichia coli K-12 MG1655
<220> moeb b0827
<222> 624-723
<223> moeb b0827 U00096 complement(863603..864352)
<313> 5369: 678-697
<400> 5369
GATCAAAATG CTGGCAGGTT ATGGCAAACC GGCCAGCGGG AAAATCGTCA 50
TGTACGATGC GATGACCTGT CAGTTTCGCG AAATGAAACT GATGCGTAAT 100
<210> 5370
<211> 100
<212> DNA
<213> Escherichia coli K-12 MG1655
<220> moeb b0827
<222> 478-577
<223> moeb b0827 U00096 complement(863603..864352)
<313> 5370: 522-541
<400> 5370

CAAATCACCG TCTTTACTTA TCAGGACGGT GAACCGTGCT ATCGCTGCCT 50
TAGCCGTTTG TTTGGTGAAA ATGCATTAAC CTGCGTGGAA GCAGGCGTAA 100
<210> 5371
<211> 100
<212> DNA
<213> Escherichia coli K-12 MG1655
<220> yjjw b4380
<222> 728-827
<223> yjjw b4380 U00096 complement(4612249..4613112)
<313> 5371: 781-800
<400> 5371
ACGCGTTTCA TGCCCACGGC GTGTATGGCG AGGCGCAAAG CTGGGCGAGC 50
GCCACGCCGG AAGACGTTGA GCCGTTGGCT GATGCGTTAA AGGTGCGCGG 100
<210> 5372
<211> 100
<212> DNA
<213> Escherichia coli K-12 MG1655
<220> yjjw b4380
<222> 651-750
<223> yjjw b4380 U00096 complement(4612249..4613112)
<313> 5372: 691-710
<400> 5372
CCAGGTGGAT TATTTGCAAC ACATCGAAGA ACTGGCGGCG TTTATCAAGG 50
GACTCGGCGA TGTTCCGGTA CGCCTGAACG CGTTTCATGC CCACGGCGTG 100
<210> 5373
<211> 100
<212> DNA
<213> Escherichia coli K-12 MG1655
<220> yjjw b4380
<222> 632-731
<223> yjjw b4380 U00096 complement(4612249..4613112)
<313> 5373: 656-675
<400> 5373
GTTTGCTGGT GATCCCTGGC CAGGTGGATT ATTTGCAACA CATCGAAGAA 50
CTGGCGGCGT TTATCAAGGG ACTCGGCGAT GTTCCGGTAC GCCTGAACGC 100
<210> 5374
<211> 100
<212> DNA
<213> Escherichia coli K-12 MG1655
<220> yjjx b4395
<222> 399-498
<223> yjjx b4395 U00096 complement(4630802..4631323)
<313> 5374: 456-475
<400> 5374
TGATGAGATT GGTCGTAAGG AAGGGGCGAT CGGCGTGTTT ACCGCCGGGA 50
AACTCACTCG CGCCAGCGTC TATCACCAGG CGGTGATCCT GGCGCTGAGT 100
<210> 5375
<211> 100
<212> DNA
<213> Escherichia coli K-12 MG1655
<220> yjjx b4395
<222> 266-365
<223> yjjx b4395 U00096 complement(4630802..4631323)
<313> 5375: 320-339
<400> 5375
GTTGGGTGGT GATTGAAAAC GCCAGCCAGC GCGGCGAGGC GCGTTCGGCT 50
ACTTTGCCGT TGCCTGCGGT GATTCTGGAA AAAGTGCGTG AAGGTGAGGC 100
<210> 5376
<211> 100
<212> DNA
<213> Escherichia coli K-12 MG1655
<220> yjjx b4395
<222> 240-339
<223> yjjx b4395 U00096 complement(4630802..4631323)
<313> 5376: 279-298
<400> 5376
TGGCATCGAT GGTGACAGCA CTTTCAGTTG GGTGGTGATT GAAAACGCCA 50
GCCAGCGCGG CGAGGCGCGT TCGGCTACTT TGCCGTTGCC TGCGGTGATT 100
<210> 5377
<211> 100
<212> DNA

<213> Escherichia coli K-12 MG1655
<220> yjjy b4403
<222> 41-140
<223> yjjy b4403 U00096 4637971..4638111
<313> 5377: 91-110
<400> 5377
TCAACGTCAA CAACATCATT AGCTTGGTCG TGGGTACTTT CCCTCAGGAC 50
CCGACAGTGT CAAAAACGGC TGTCATCCTA ACCATTTTAA CAGCAACATA 100
<210> 5378
<211> 100
<212> DNA
<213> Escherichia coli K-12 MG1655
<220> yjjy b4403
<222> 30-129
<223> yjjy b4403 U00096 4637971..4638111
<313> 5378: 70-89
<400> 5378
TGCACTTTCC ATCAACGTCA ACAACATCAT TAGCTTGGTC GTGGGTACTT 50
TCCCTCAGGA CCCGACAGTG TCAAAAACGG CTGTCATCCT AACCATTTTA 100
<210> 5379
<211> 100
<212> DNA
<213> Escherichia coli K-12 MG1655
<220> yjjy b4403
<222> 6-105
<223> yjjy b4403 U00096 4637971..4638111
<313> 5379: 65-84
<400> 5379
TAAAGTACGT AATTGCGTTC TTGATGCACT TTCCATCAAC GTCAACAACA 50
TCATTAGCTT GGTCGTGGGT ACTTTCCCTC AGGACCCGAC AGTGTCAAAA 100
<210> 5380
<211> 100
<212> DNA
<213> Escherichia coli K-12 MG1655
<220> lysp b2157
<222> 1371-1470
<223> lysp b2157 U00096 complement(2245083..2246552)
<313> 5380: 1435-1454
<400> 5380
CCCGCTGTTC CTGATTATTT GGTTCGGCTA CAAGCTGATT AAAGGAACTC 50
ACTTCGTACG CTACAGCGAA ATGAAGTTCC CGCAGAACGA TAAGAAATAA 100
<210> 5381
<211> 100
<212> DNA
<213> Escherichia coli K-12 MG1655
<220> lysp b2157
<222> 1368-1467
<223> lysp b2157 U00096 complement(2245083..2246552)
<313> 5381: 1392-1411
<400> 5381
TATCCCGCTG TTCCTGATTA TTTGGTTCGG CTACAAGCTG ATTAAAGGAA 50
CTCACTTCGT ACGCTACAGC GAAATGAAGT TCCCGCAGAA CGATAAGAAA 100
<210> 5382
<211> 100
<212> DNA
<213> Escherichia coli K-12 MG1655
<220> lysp b2157
<222> 1282-1381
<223> lysp b2157 U00096 complement(2245083..2246552)
<313> 5382: 1334-1353
<400> 5382
TGTCTGATTA TCACTTTGGG CCAGAACTAC GAAGCGTTCC TGAAAGATAC 50
TATTGACTGG GGCGGCGTAG CGGCAACGTA TATTGGTATC CCGCTGTTCC 100
<210> 5383
<211> 100
<212> DNA
<213> Escherichia coli K-12 MG1655
<220> lysr b2840
<222> 837-936
<223> lysr b2840 U00096 2977043..2977978
<313> 5383: 892-911

<400> 5383
ATCAGCGCTG GTTCAGGCGT TTAGTGGGCA TTTACAAGCG GGGTTACCGA  50
AACTGGTCAC TTCTCTTGAC GCTATTTTGT CGTCAGCTAC GACAGCATAA  100
<210> 5384
<211> 100
<212> DNA
<213> Escherichia coli K-12 MG1655
<220> lysr b2840
<222> 809-908
<223> lysr b2840 U00096 2977043..2977978
<313> 5384: 853-872
<400> 5384
GCCTGATCCG CCCCCTGCAC CGCCCGTCAT CAGCGCTGGT TCAGGCGTTT  50
AGTGGGCATT TACAAGCGGG GTTACCGAAA CTGGTCACTT CTCTTGACGC  100
<210> 5385
<211> 100
<212> DNA
<213> Escherichia coli K-12 MG1655
<220> lysr b2840
<222> 789-888
<223> lysr b2840 U00096 2977043..2977978
<313> 5385: 810-829
<400> 5385
TGCGGTTCCG TTCACCGTCA GCCTGATCCG CCCCCTGCAC CGCCCGTCAT  50
CAGCGCTGGT TCAGGCGTTT AGTGGGCATT TACAAGCGGG GTTACCGAAA  100
<210> 5386
<211> 100
<212> DNA
<213> Escherichia coli K-12 MG1655
<220> lyss b2891
<222> 1419-1518
<223> lyss b2891 U00096 complement(3031677..3033194)
<313> 5386: 1467-1486
<400> 5386
AGGTCTGGGA ATTGGTATCG ACCGTATGGT AATGCTGTTC ACCAACAGCC  50
ATACCATCCG CGACGTTATT CTGTTCCCGG CGATGCGTCC GGTAAAATAA  100
<210> 5387
<211> 100
<212> DNA
<213> Escherichia coli K-12 MG1655
<220> lyss b2891
<222> 1335-1434
<223> lyss b2891 U00096 complement(3031677..3033194)
<313> 5387: 1390-1409
<400> 5387
CGCGAAAGAC GCAGGTGACG ACGAAGCGAT GTTCTACGAT GAAGATTACG  50
TCACCGCACT GGAACATGGC TTACCGCCGA CAGCAGGTCT GGGAATTGGT  100
<210> 5388
<211> 100
<212> DNA
<213> Escherichia coli K-12 MG1655
<220> lyss b2891
<222> 1294-1393
<223> lyss b2891 U00096 complement(3031677..3033194)
<313> 5388: 1350-1369
<400> 5388
GACGCGGAAG ATCAGGCGCA ACGCTTCCTG GATCAGGTTG CCGCGAAAGA  50
CGCAGGTGAC GACGAAGCGA TGTTCTACGA TGAAGATTAC GTCACCGCAC  100
<210> 5389
<211> 100
<212> DNA
<213> Escherichia coli K-12 MG1655
<220> lysu b4130
<222> 1419-1518
<223> lysu b4130 U00096 complement(4350778..4352295)
<313> 5389: 1484-1503
<400> 5389
TGGTCTGGGT ATTGGTATCG ACCGAATGAT TATGCTGTTT ACTAACAGCC  50
ATACTATTCG CGACGTTATT CTCTTCCCGG CGATGCGCCC ACAGAAATAA  100
<210> 5390
<211> 100

<212> DNA
<213> Escherichia coli K-12 MG1655
<220> lysu b4130
<222> 1354-1453
<223> lysu b4130 U00096 complement(4350778..4352295)
<313> 5390: 1401-1420
<400> 5390
GACGAAGCCA TGTTCTATGA CGAAGATTAC GTGACTGCGC TGGAATATGG 50
TCTGCCGCCA ACCGCTGGTC TGGGTATTGG TATCGACCGA ATGATTATGC 100
<210> 5391
<211> 100
<212> DNA
<213> Escherichia coli K-12 MG1655
<220> lysu b4130
<222> 1313-1412
<223> lysu b4130 U00096 complement(4350778..4352295)
<313> 5391: 1348-1367
<400> 5391
AACGTTTCCA GGAACAGGTT AATGCTAAAG CTGCAGGTGA CGACGAAGCC 50
ATGTTCTATG ACGAAGATTA CGTGACTGCG CTGGAATATG GTCTGCCGCC 100
<210> 5392
<211> 100
<212> DNA
<213> Escherichia coli K-12 MG1655
<220> slya slyA
<222> 342-441
<223> slya slyA U00096 complement(1718414..1718854)
<313> 5392: 386-405
<400> 5392
GGAAATATTA CATGGCATCT CCGCAGAGGA ACTGGAGCAA CTGATTACGC 50
TCATCGCAAA ACTTGAGCAT AATATCATTG AGTTACAGGC CAAAGGGTGA 100
<210> 5393
<211> 100
<212> DNA
<213> Escherichia coli K-12 MG1655
<220> slya slyA
<222> 252-351
<223> slya slyA U00096 complement(1718414..1718854)
<313> 5393: 278-297
<400> 5393
CAGCGATCGT CGGGCTAAAC GTATTAAACT GACGGAAAAG GCAGAGCCGC 50
TGATCAGCGA AATGGAAGCT GTTATTAACA AAACCCGCGC GGAAATATTA 100
<210> 5394
<211> 100
<212> DNA
<213> Escherichia coli K-12 MG1655
<220> slya slyA
<222> 184-283
<223> slya slyA U00096 complement(1718414..1718854)
<313> 5394: 229-248
<400> 5394
CAGCCATCAC TGGTCCGTAC TCTGGACCAA CTGGAAGAAA AAGGGTTAAT 50
TTCGCGTCAA ACTTGTGCCA GCGATCGTCG GGCTAAACGT ATTAAACTGA 100
<210> 5395
<211> 100
<212> DNA
<213> Escherichia coli K-12 MG1655
<220> slyb b1642
<222> 369-468
<223> slyb b1642 U00096 1717900..1718367
<313> 5395: 435-454
<400> 5395
CATCATGGTG GTACAGAAAC AAGGCAACAC TCGTTTCTCT CCGGGCCAAC 50
GTGTCGTACT GGCCAGCAAT GGCAGTCAGG TGACCGTTTC TCCGCGCTAA 100
<210> 5396
<211> 100
<212> DNA
<213> Escherichia coli K-12 MG1655
<220> slyb b1642
<222> 360-459
<223> slyb b1642 U00096 1717900..1718367

<313> 5396: 406-425
<400> 5396
TGGTAATACC ATCATGGTGG TACAGAAACA AGGCAACACT CGTTTCTCTC 50
CGGGCCAACG TGTCGTACTG GCCAGCAATG GCAGTCAGGT GACCGTTTCT 100
<210> 5397
<211> 100
<212> DNA
<213> Escherichia coli K-12 MG1655
<220> slyb b1642
<222> 307-406
<223> slyb b1642 U00096 1717900..1718367
<313> 5397: 334-353
<400> 5397
CAGAGTGCAA TGAACAAAAC GCAGGGTGTC GAGCTGGAAA TTCGTAAAGA 50
CGATGGTAAT ACCATCATGG TGGTACAGAA ACAAGGCAAC ACTCGTTTCT 100
<210> 5398
<211> 100
<212> DNA
<213> Escherichia coli K-12 MG1655
<220> feoa b3409
<222> 129-228
<223> feoa b3409 U00096 3537799..3538026
<313> 5398: 187-206
<400> 5398
TCCACTCGGC GACCCCATTC ATATCGAAAC CCGTCGTGTG AGCCTGGTAT 50
TACGCAAAAA AGATCTGGCC TTATTAGAAG TGGAAGCGGT TTCCTGTTAA 100
<210> 5399
<211> 100
<212> DNA
<213> Escherichia coli K-12 MG1655
<220> feoa b3409
<222> 122-221
<223> feoa b3409 U00096 3537799..3538026
<313> 5399: 153-172
<400> 5399
GCGTCGCTCC ACTCGGCGAC CCCATTCATA TCGAAACCCG TCGTGTGAGC 50
CTGGTATTAC GCAAAAAAGA TCTGGCCTTA TTAGAAGTGG AAGCGGTTTC 100
<210> 5400
<211> 100
<212> DNA
<213> Escherichia coli K-12 MG1655
<220> feoa b3409
<222> 17-116
<223> feoa b3409 U00096 3537799..3538026
<313> 5400: 63-82
<400> 5400
ATACTGCGTG GAAAATCACT GGCTTTTCCC GTGAAATCAG CCCGGCATAT 50
CGCCAAAAAC TGCTTTCTCT TGGCATGTTA CCTGGCTCCT CTTTTAATGT 100
<210> 5401
<211> 100
<212> DNA
<213> Escherichia coli K-12 MG1655
<220> lytb b0030
<222> 704-803
<223> lytb b0030 U00096 26277..27227
<313> 5401: 762-781
<400> 5401
CCCAGCGTAT GGGCAAACGC GCGTTTTTGA TTGACGATGC GAAAGACATC 50
CAGGAAGAGT GGGTGAAAGA GGTTAAATGC GTCGGCGTGA CTGCGGGCGC 100
<210> 5402
<211> 100
<212> DNA
<213> Escherichia coli K-12 MG1655
<220> lytb b0030
<222> 670-769
<223> lytb b0030 U00096 26277..27227
<313> 5402: 698-717
<400> 5402
AACTCCTCCA ACTCCAACCG TCTGGCGGAG CTGGCCCAGC GTATGGGCAA 50
ACGCGCGTTT TTGATTGACG ATGCGAAAGA CATCCAGGAA GAGTGGGTGA 100
<210> 5403

<211> 100
<212> DNA
<213> Escherichia coli K-12 MG1655
<220> lytb b0030
<222> 606-705
<223> lytb b0030 U00096 26277..27227
<313> 5403: 656-675
<400> 5403
CCGTCAGGAA GCGGTACGCG CCCTGGCAGA ACAGGCGGAA GTTGTGTTGG 50
TGGTCGGTTC GAAAAACTCC TCCAACTCCA ACCGTCTGGC GGAGCTGGCC 100
<210> 5404
<211> 100
<212> DNA
<213> Escherichia coli K-12 MG1655
<220> slyd b3350
<222> 454-553
<223> slyd b3350 U00096 complement(3475544..3476134)
<313> 5404: 474-493
<400> 5404
GTTCACGGCG CGCACGATCA CCACCACGAT CACGACCACG ACGGTTGCTG 50
CGGCGGTCAT GGCCACGATC ACGGTCATGA ACACGGTGGC GAAGGCTGCT 100
<210> 5405
<211> 100
<212> DNA
<213> Escherichia coli K-12 MG1655
<220> slyd b3350
<222> 402-501
<223> slyd b3350 U00096 complement(3475544..3476134)
<313> 5405: 430-449
<400> 5405
TGAAGTTGTG GCGATTCGCG AAGCGACTGA AGAAGAACTG GCTCATGGTC 50
ACGTTCACGG CGCGCACGAT CACCACCACG ATCACGACCA CGACGGTTGC 100
<210> 5406
<211> 100
<212> DNA
<213> Escherichia coli K-12 MG1655
<220> slyd b3350
<222> 165-264
<223> slyd b3350 U00096 complement(3475544..3476134)
<313> 5406: 201-220
<400> 5406
CGACAAATTT GATGTCGCTG TTGGCGCGAA CGACGCTTAC GGTCAGTACG 50
ACGAAAACCT GGTGCAACGT GTTCCTAAAG ACGTATTTAT GGGCGTTGAT 100
<210> 5407
<211> 100
<212> DNA
<213> Escherichia coli K-12 MG1655
<220> feob b3410
<222> 2150-2249
<223> feob b3410 U00096 3538043..3540364
<313> 5407: 2194-2213
<400> 5407
ACAGTCAGCA TCCAACTTAC AGCCTGGTGT GCATTCTGGC GGTTATCCTG 50
TTTAACATCG TGGTTATCGG TCTGCTGCGC CGCGCGCGTA GCCGGGTGGA 100
<210> 5408
<211> 100
<212> DNA
<213> Escherichia coli K-12 MG1655
<220> feob b3410
<222> 2086-2185
<223> feob b3410 U00096 3538043..3540364
<313> 5408: 2149-2168
<400> 5408
TCCATCCTGT GGGGGCTGAA TATCGCTTAC TCACTGGCAA CATTGTTCTA 50
TCAGGTCGCC AGCTACAGTC AGCATCCAAC TTACAGCCTG GTGTGCATTC 100
<210> 5409
<211> 100
<212> DNA
<213> Escherichia coli K-12 MG1655
<220> feob b3410
<222> 2076-2175

<223> feob b3410 U00096 3538043..3540364
<313> 5409: 2097-2116
<400> 5409
GATGGGCTTC TCCATCCTGT GGGGGCTGAA TATCGCTTAC TCACTGGCAA 50
CATTGTTCTA TCAGGTCGCC AGCTACAGTC AGCATCCAAC TTACAGCCTG 100
<210> 5410
<211> 100
<212> DNA
<213> Escherichia coli K-12 MG1655
<220> alpa b2625
<222> 114-213
<223> alpa b2625 U00096 2756665..2756877
<313> 5410: 177-196
<400> 5410
TGCCACCTTT CCCAAAAAGC GAATGCTCGG CGTGAAATCT GTCGGATGGA 50
TTGAGGCCGA GATTGATGAG TGGTTATCAC AACGCTGTAA ACTTATTTGA 100
<210> 5411
<211> 100
<212> DNA
<213> Escherichia coli K-12 MG1655
<220> alpa b2625
<222> 107-206
<223> alpa b2625 U00096 2756665..2756877
<313> 5411: 128-147
<400> 5411
GATACGATGC CACCTTTCCC AAAAAGCGAA TGCTCGGCGT GAAATCTGTC 50
GGATGGATTG AGGCCGAGAT TGATGAGTGG TTATCACAAC GCTGTAAACT 100
<210> 5412
<211> 100
<212> DNA
<213> Escherichia coli K-12 MG1655
<220> alpa b2625
<222> 70-169
<223> alpa b2625 U00096 2756665..2756877
<313> 5412: 96-115
<400> 5412
GCCACCATCT ATGACTGGTT GAACCCCAAA TCACCACGAT ACGATGCCAC 50
CTTTCCCAAA AAGCGAATGC TCGGCGTGAA ATCTGTCGGA TGGATTGAGG 100
<210> 5413
<211> 100
<212> DNA
<213> Escherichia coli K-12 MG1655
<220> alr b4054
<222> 944-1043
<223> alr b4054 U00096 4263361..4264440
<313> 5413: 1000-1019
<400> 5413
AGGACAAAGC CGGGGATCCG GTCATTTTAT GGGGCGAAGG TTTGCCCGTA 50
GAACGTATCG CTGAAATGAC GAAAGTAAGC GCTTACGAAC TTATTACGCG 100
<210> 5414
<211> 100
<212> DNA
<213> Escherichia coli K-12 MG1655
<220> alr b4054
<222> 870-969
<223> alr b4054 U00096 4263361..4264440
<313> 5414: 885-904
<400> 5414
GAACGGTCGC GAAGTACCGA TTGTCGGGCG CGTGGCGATG GATATGATCT 50
GCGTAGACTT AGGTCCACAG GCGCAGGACA AAGCCGGGGA TCCGGTCATT 100
<210> 5415
<211> 100
<212> DNA
<213> Escherichia coli K-12 MG1655
<220> alr b4054
<222> 692-791
<223> alr b4054 U00096 4263361..4264440
<313> 5415: 754-773
<400> 5415
GTCAGCCAGT GATGTCACTA ACCTCCAGCC TGATTGCCGT GCGTGAGCAT 50
AAAGCCGGAG AGCCTGTTGG TTATGGTGGA ACCTGGGTAA GCGAACGTGA 100

<210> 5416
<211> 100
<212> DNA
<213> Escherichia coli K-12 MG1655
<220> bisc b3552
<222> 2029-2128
<223> bisc b3552 U00096 complement(3711690..3713909)
<313> 5416: 2088-2107
<400> 5416
GGGGCATGGC CGGATCTGGA TTTAACCGCT GACGGTATTT GTAAAAACGG 50
CGCAGTGAAC GTGCTGACCA AAGATCTCCC CAGCTCGCGG CTGGGGAATG 100
<210> 5417
<211> 100
<212> DNA
<213> Escherichia coli K-12 MG1655
<220> bisc b3552
<222> 1985-2084
<223> bisc b3552 U00096 complement(3711690..3713909)
<313> 5417: 2007-2026
<400> 5417
TCATTAGCGA GGGAATTAAA CCTGGCGTGA TTTGCATTCA CGAAGGGGCA 50
TGGCCGGATC TGGATTTAAC CGCTGACGGT ATTTGTAAAA ACGGCGCAGT 100
<210> 5418
<211> 100
<212> DNA
<213> Escherichia coli K-12 MG1655
<220> bisc b3552
<222> 1936-2035
<223> bisc b3552 U00096 complement(3711690..3713909)
<313> 5418: 1978-1997
<400> 5418
GATACTGTTC GGTTGTGGAA CGCACGCGGG CAAATTCTTG CCGGAGCGGT 50
CATTAGCGAG GGAATTAAAC CTGGCGTGAT TTGCATTCAC GAAGGGGCAT 100
<210> 5419
<211> 100
<212> DNA
<213> Escherichia coli K-12 MG1655
<220> pnp b3165
<222> 2106-2205
<223> pnp b3165 U00096 complement(3306674..3308878)
<313> 5419: 2179-2198
<400> 5419
TGATCGCCAG GGCCGTATCC GTCTGAGCAT TAAAGAAGCG ACTGAGCAGT 50
CTCAACCTGC TGCAGCACCG GAAGCTCCGG CTGCTGAACA GGGCGAGTAA 100
<210> 5420
<211> 100
<212> DNA
<213> Escherichia coli K-12 MG1655
<220> pnp b3165
<222> 2090-2189
<223> pnp b3165 U00096 complement(3306674..3308878)
<313> 5420: 2144-2163
<400> 5420
TGAAAGTTCT GGAAGTTGAT CGCCAGGGCC GTATCCGTCT GAGCATTAAA 50
GAAGCGACTG AGCAGTCTCA ACCTGCTGCA GCACCGGAAG CTCCGGCTGC 100
<210> 5421
<211> 100
<212> DNA
<213> Escherichia coli K-12 MG1655
<220> pnp b3165
<222> 1985-2084
<223> pnp b3165 U00096 complement(3306674..3308878)
<313> 5421: 2014-2033
<400> 5421
TTGCCATCGG CGGCGGTAAA GAAGGTCTGG TCCACATCTC TCAAATCGCT 50
GACAAACGCG TTGAGAAAGT GACCGATTAC CTGCAGATGG GTCAGGAAGT 100
<210> 5422
<211> 100
<212> DNA
<213> Escherichia coli K-12 MG1655
<220> slyx b3349

<222> 120-219
<223> slyx b3349 U00096 3475277..3475495
<313> 5422: 190-209
<400> 5422
ACTGCGCGAT CATCTGCGTC TGCTGACCGA GAAGTTAAAA GCCAGCCAGC 50
CGTCGAACAT CGCGTCGCAG GCTGAAGAAA CGCCACCGCC ACATTATTGA 100
<210> 5423
<211> 100
<212> DNA
<213> Escherichia coli K-12 MG1655
<220> slyx b3349
<222> 98-197
<223> slyx b3349 U00096 3475277..3475495
<313> 5423: 142-161
<400> 5423
CTCATGAAAT GGAGATGGCG AAACTGCGCG ATCATCTGCG TCTGCTGACC 50
GAGAAGTTAA AAGCCAGCCA GCCGTCGAAC ATCGCGTCGC AGGCTGAAGA 100
<210> 5424
<211> 100
<212> DNA
<213> Escherichia coli K-12 MG1655
<220> slyx b3349
<222> 69-168
<223> slyx b3349 U00096 3475277..3475495
<313> 5424: 116-135
<400> 5424
CATTGAAGAA CTGAACGTCA CGGTGACCGC TCATGAAATG GAGATGGCGA 50
AACTGCGCGA TCATCTGCGT CTGCTGACCG AGAAGTTAAA AGCCAGCCAG 100
<210> 5425
<211> 100
<212> DNA
<213> Escherichia coli K-12 MG1655
<220> bisz b1873
<222> 2343-2442
<223> bisz b1873 U00096 complement(1952602..1955049)
<313> 5425: 2407-2426
<400> 5425
GCTGGCAAAT GCCTGTGCCG GTAACTCAGC GTTGGTGTAT ATCGAAAAAT 50
ATACGGGCAA TGCACCGAAG TTAACGGCGT TTGATCAGCC AGCTGTTCAG 100
<210> 5426
<211> 100
<212> DNA
<213> Escherichia coli K-12 MG1655
<220> bisz b1873
<222> 2248-2347
<223> bisz b1873 U00096 complement(1952602..1955049)
<313> 5426: 2295-2314
<400> 5426
GTATGCGTGC ATGAAGGTGC ATGGCCAGAT CTGGAAAATG GCTTGTGTAA 50
AAACGGCAGT GCGAACGTGT TAACGGCGGA TATCCCCAGC TCGCAGCTGG 100
<210> 5427
<211> 100
<212> DNA
<213> Escherichia coli K-12 MG1655
<220> bisz b1873
<222> 2194-2293
<223> bisz b1873 U00096 complement(1952602..1955049)
<313> 5427: 2224-2243
<400> 5427
CGTGGTCAGA TTTTGACAGG CGCGGTGGTG ACTGACGGGA TCAAAAAAGG 50
CGTGGTATGC GTGCATGAAG GTGCATGGCC AGATCTGGAA AATGGCTTGT 100
<210> 5428
<211> 100
<212> DNA
<213> Escherichia coli K-12 MG1655
<220> amn amn
<222> 1356-1455
<223> amn amn U00096 2053083..2054537
<313> 5428: 1422-1441
<400> 5428
TCTGCAAATT GGCATTCGGG CGATCGATTT GCTGCGCGCA GAAGGCGACC 50

GACTGCATTC GCGTAAATTA CGAACCTTTA ATGAGCCGCC GTTCCGATAA 100
<210> 5429
<211> 100
<212> DNA
<213> Escherichia coli K-12 MG1655
<220> amn amn
<222> 1308-1407
<223> amn amn U00096 2053083..2054537
<313> 5429: 1349-1368
<400> 5429
ACTTCCCGGC CAGGCTAACC GTTTTTATGA AGGCGCTATT TCCGAACATC 50
TGCAAATTGG CATTCGGGCG ATCGATTTGC TGCGCGCAGA AGGCGACCGA 100
<210> 5430
<211> 100
<212> DNA
<213> Escherichia coli K-12 MG1655
<220> amn amn
<222> 1266-1365
<223> amn amn U00096 2053083..2054537
<313> 5430: 1303-1322
<400> 5430
ACTACTGTGT GTTTCAGATA AACCGTTGCA TGGCGAGATT AAACTTCCCG 50
GCCAGGCTAA CCGTTTTTAT GAAGGCGCTA TTTCCGAACA TCTGCAAATT 100
<210> 5431
<211> 100
<212> DNA
<213> Escherichia coli K-12 MG1655
<220> fepa b0585
<222> 2090-2189
<223> fepa b0585 U00096 complement(609477..611717)
<313> 5431: 2145-2164
<400> 5431
TGGACAATCT GTTCGACAAA CGTTTGTGGC GTGCGGGTAA TGCCCAGACC 50
ACGGGCGATT TGGCAGGGGC CAACTATATC GCCGGTGCCG GGGCGTATAC 100
<210> 5432
<211> 100
<212> DNA
<213> Escherichia coli K-12 MG1655
<220> fepa b0585
<222> 2025-2124
<223> fepa b0585 U00096 complement(609477..611717)
<313> 5432: 2088-2107
<400> 5432
TTACAGCATT GTTGGCCTGA GCGCGACCTG GGATGTGACG AAGAATGTCA 50
GTCTGACCGG CGGCGTGGAC AATCTGTTCG ACAAACGTTT GTGGCGTGCG 100
<210> 5433
<211> 100
<212> DNA
<213> Escherichia coli K-12 MG1655
<220> fepa b0585
<222> 2012-2111
<223> fepa b0585 U00096 complement(609477..611717)
<313> 5433: 2047-2066
<400> 5433
AAGAAATTAG TCCTTACAGC ATTGTTGGCC TGAGCGCGAC CTGGGATGTG 50
ACGAAGAATG TCAGTCTGAC CGGCGGCGTG GACAATCTGT TCGACAAACG 100
<210> 5434
<211> 100
<212> DNA
<213> Escherichia coli K-12 MG1655
<220> fepb b0593
<222> 851-950
<223> fepb b0593 U00096 complement(622777..623733)
<313> 5434: 906-925
<400> 5434
TGCCTGCAGT ACAAAACAAG CAGGTTTATG CGCTGGGAAC CGAGACGTTC 50
CGTCTGGATT ACTACAGCGC CATGCAAGTG CTGGATAGGC TTAAGGCGCT 100
<210> 5435
<211> 100
<212> DNA
<213> Escherichia coli K-12 MG1655

<220> fepb b0593
<222> 796-895
<223> fepb b0593 U00096 complement(622777..623733)
<313> 5435: 831-850
<400> 5435
GCCGGTGATC AGAAAGATGC CGATGCTATT TATGCTAATC CGCTGCTCGC  50
GCACCTGCCT GCAGTACAAA ACAAGCAGGT TTATGCGCTG GGAACCGAGA  100
<210> 5436
<211> 100
<212> DNA
<213> Escherichia coli K-12 MG1655
<220> fepb b0593
<222> 696-795
<223> fepb b0593 U00096 complement(622777..623733)
<313> 5436: 732-751
<400> 5436
GTTAAATGCC AGCCAAAGTC AGGGTAAACG CCATGACATC ATTCAGCTTG  50
GTGGGGAAAA TCTGGCTGCA GGGTTAAATG GCGAGTCACT ATTCCTGTTC  100
<210> 5437
<211> 100
<212> DNA
<213> Escherichia coli K-12 MG1655
<220> fepc b0589
<222> 717-816
<223> fepc b0589 U00096 complement(618607..619422)
<313> 5437: 789-808
<400> 5437
CATTTATGGT CTGCGCTGCA TGATCATTGA CGATCCAGTT GCCGGAACGC  50
CGCTTGTGGT GCCGCTCGGA CGAACGGCAC CTTCAACCGC AAATAGTTAA  100
<210> 5438
<211> 100
<212> DNA
<213> Escherichia coli K-12 MG1655
<220> fepc b0589
<222> 675-774
<223> fepc b0589 U00096 complement(618607..619422)
<313> 5438: 696-715
<400> 5438
GGGAGCGCCG AAGGAGATTG TCACTGCTGA ACTGATTGAG CGCATTTATG  50
GTCTGCGCTG CATGATCATT GACGATCCAG TTGCCGGAAC GCCGCTTGTG  100
<210> 5439
<211> 100
<212> DNA
<213> Escherichia coli K-12 MG1655
<220> fepc b0589
<222> 607-706
<223> fepc b0589 U00096 complement(618607..619422)
<313> 5439: 660-679
<400> 5439
GATCTTAATC AGGCCTGTCG TTACGCCAGC CATTTGATTG CATTGCGGGA  50
AGGGAAAATT GTTGCTCAGG GAGCGCCGAA GGAGATTGTC ACTGCTGAAC  100
<210> 5440
<211> 100
<212> DNA
<213> Escherichia coli K-12 MG1655
<220> fepd b0591
<222> 892-991
<223> fepd b0591 U00096 complement(620408..621412)
<313> 5440: 926-945
<400> 5440
ATCATCGGGC GGGTGATTGT GCCCGGCGAA CTGCGCGTTT CTGTGGTCAG  50
TGCGTTTATT GGTGCACCGG TGCTGATCTT CCTCGTGCGA CGTAAAACGC  100
<210> 5441
<211> 100
<212> DNA
<213> Escherichia coli K-12 MG1655
<220> fepd b0591
<222> 833-932
<223> fepd b0591 U00096 complement(620408..621412)
<313> 5441: 880-899
<400> 5441

ATCGCTGGTC GCTGCCCGTC ACGCTACTTG CTACCCCTGC CCTGCTGCTG 50
TTTGCCGATA TCATCGGGCG GGTGATTGTG CCCGGCGAAC TGCGCGTTTC 100
<210> 5442
<211> 100
<212> DNA
<213> Escherichia coli K-12 MG1655
<220> fepd b0591
<222> 790-889
<223> fepd b0591 U00096 complement(620408..621412)
<313> 5442: 846-865
<400> 5442
CTGATGATGC CGCATATGGC GCGTTGGCTG GTGGGTGCCG ATCATCGCTG 50
GTCGCTGCCC GTCACGCTAC TTGCTACCCC TGCCCTGCTG CTGTTTGCCG 100
<210> 5443
<211> 100
<212> DNA
<213> Escherichia coli K-12 MG1655
<220> fepe b0588
<222> 936-1035
<223> fepe b0588 U00096 617477..618610
<313> 5443: 979-998
<400> 5443
TGTCAACGAT GTGAATTTTA CGCCGTTTAA ATATCAGTTA AGCCCGTCAT 50
TGCCAGTGAA AAAAGACGGT CCGGGTAAGG CGATTATTGT GATCCTTTCC 100
<210> 5444
<211> 100
<212> DNA
<213> Escherichia coli K-12 MG1655
<220> fepe b0588
<222> 812-911
<223> fepe b0588 U00096 617477..618610
<313> 5444: 860-879
<400> 5444
TTTCTCTCGG TGCAGACGGT ATTGAACGCA AACTGGAAAT AGAAAAAGCG 50
GTCACTGACG TTGCGGAACT GAACGGTGAA TTACGTAATC GGCAGTATCT 100
<210> 5445
<211> 100
<212> DNA
<213> Escherichia coli K-12 MG1655
<220> fepe b0588
<222> 772-871
<223> fepe b0588 U00096 617477..618610
<313> 5445: 834-853
<400> 5445
AGTAATGGTC AGGCCGTTAA AGATGACCCC GATTTTTCTA TTTCTCTCGG 50
TGCAGACGGT ATTGAACGCA AACTGGAAAT AGAAAAAGCG GTCACTGACG 100
<210> 5446
<211> 100
<212> DNA
<213> Escherichia coli K-12 MG1655
<220> fepg b0590
<222> 848-947
<223> fepg b0590 U00096 complement(619419..620411)
<313> 5446: 898-917
<400> 5446
TATGCGGGGC GCTGTTACTG CTGGCGGCCG ATCTCTGCGC CCAACAACTG 50
TTTATGCCGT ATCAACTTCC GGTTGGCGTC GTTACCGTCA GCCTCGGCGG 100
<210> 5447
<211> 100
<212> DNA
<213> Escherichia coli K-12 MG1655
<220> fepg b0590
<222> 787-886
<223> fepg b0590 U00096 complement(619419..620411)
<313> 5447: 817-836
<400> 5447
GCACCGCACA TTGCCCGACG CATTAGCGGC ACCGCTCGCT GGGGGCTAAC 50
CCAGGCGGCG CTATGCGGGG CGCTGTTACT GCTGGCGGCC GATCTCTGCG 100
<210> 5448
<211> 100
<212> DNA

<213> Escherichia coli K-12 MG1655
<220> fepg b0590
<222> 761-860
<223> fepg b0590 U00096 complement(619419..620411)
<313> 5448: 793-812
<400> 5448
GGCCGATTTC CTTTATTGCT TTAGTCGCAC CGCACATTGC CCGACGCATT 50
AGCGGCACCG CTCGCTGGGG GCTAACCCAG GCGGCGCTAT GCGGGGCGCT 100
<210> 5449
<211> 100
<212> DNA
<213> Escherichia coli K-12 MG1655
<220> b0165 b0166
<222> 15-114
<223> b0165 b0166 U00096 complement(184987..185118)
<313> 5449: 76-95
<400> 5449
CACGACGCTG CCCGATTTTT TTGCAGGGAT GAGCGATGAT TTCACGCCGC 50
CGATTTTTGC AGGCTACTGC CGCGACGATA GCCACGAGCT CAGGTTTCGG 100
<210> 5450
<211> 100
<212> DNA
<213> Escherichia coli K-12 MG1655
<220> b0165 b0166
<222> 31-130
<223> b0165 b0166 U00096 complement(184987..185118)
<313> 5450: 70-89
<400> 5450
TTTTTTGCAG GGATGAGCGA TGATTTCACG CCGCCGATTT TTGCAGGCTA 50
CTGCCGCGAC GATAGCCACG AGCTCAGGTT TCGGTTATAT GCATTACTGT 100
<210> 5451
<211> 100
<212> DNA
<213> Escherichia coli K-12 MG1655
<220> b0165 b0166
<222> 1-100
<223> b0165 b0166 U00096 complement(184987..185118)
<313> 5451: 56-75
<400> 5451
ATGCACACGG GCAGCACGAC GCTGCCCGAT TTTTTTGCAG GGATGAGCGA 50
TGATTTCACG CCGCCGATTT TTGCAGGCTA CTGCCGCGAC GATAGCCACG 100
<210> 5452
<211> 100
<212> DNA
<213> Escherichia coli K-12 MG1655
<220> pura b4178
<222> 1200-1299
<223> pura b4178 U00096 4402265..4403563
<313> 5452: 1273-1292
<400> 5452
CAAGCGTATT GAAGAGCTGA CTGGTGTGCC GATCGATATC ATCTCTACCG 50
GTCCGGATCG TACTGAAACC ATGATTCTGC GCGACCCGTT CGACGCGTAA 100
<210> 5453
<211> 100
<212> DNA
<213> Escherichia coli K-12 MG1655
<220> pura b4178
<222> 1091-1190
<223> pura b4178 U00096 4402265..4403563
<313> 5453: 1154-1173
<400> 5453
ACGACTGGAA AGGTGTAGAG CCGATTTACG AAACCATGCC GGGCTGGTCT 50
GAATCCACCT TCGGCGTGAA AGATCGTAGC GGCCTGCCGC AGGCGGCGCT 100
<210> 5454
<211> 100
<212> DNA
<213> Escherichia coli K-12 MG1655
<220> pura b4178
<222> 1079-1178
<223> pura b4178 U00096 4402265..4403563
<313> 5454: 1105-1124

<400> 5454
CGCTGGCAGC TGACGACTGG AAAGGTGTAG AGCCGATTTA CGAAACCATG 50
CCGGGCTGGT CTGAATCCAC CTTCGGCGTG AAAGATCGTA GCGGCCTGCC 100
<210> 5455
<211> 100
<212> DNA
<213> Escherichia coli K-12 MG1655
<220> purb b1132
<222> 1238-1337
<223> purb b1132 U00096 complement(1189839..1191209)
<313> 5455: 1296-1315
<400> 5455
GCGTTGACGC CGAAGGCATG AAGCAGTTTA TCGATGGTCT GGCGTTGCCA 50
GAAGAAGAGA AAGCCCGCCT GAAAGCGATG ACGCCGGCTA ACTATATTGG 100
<210> 5456
<211> 100
<212> DNA
<213> Escherichia coli K-12 MG1655
<220> purb b1132
<222> 1109-1208
<223> purb b1132 U00096 complement(1189839..1191209)
<313> 5456: 1156-1175
<400> 5456
ACCGTGACCA TCTGCTGGAT GAACTGGATC ACAACTGGGA AGTGCTGGCT 50
GAACCAATCC AGACAGTTAT GCGTCGCTAT GGCATCGAAA AACCGTACGA 100
<210> 5457
<211> 100
<212> DNA
<213> Escherichia coli K-12 MG1655
<220> purb b1132
<222> 1085-1184
<223> purb b1132 U00096 complement(1189839..1191209)
<313> 5457: 1112-1131
<400> 5457
AAGGCGTGAG CAAACTGGAA GTGAACCGTG ACCATCTGCT GGATGAACTG 50
GATCACAACT GGGAAGTGCT GGCTGAACCA ATCCAGACAG TTATGCGTCG 100
<210> 5458
<211> 100
<212> DNA
<213> Escherichia coli K-12 MG1655
<220> purc b2477
<222> 615-714
<223> purc b2477 U00096 complement(2594925..2595638)
<313> 5458: 661-680
<400> 5458
GCTGGAGAAA ATGGACAAAG ACCGTTTCCG CCAGAGCCTC GGTGGCCTGA 50
TCGAAGCCTA TGAAGCCGTC GCCCGCCGCC TGGGTGTACA GCTGGACTGA 100
<210> 5459
<211> 100
<212> DNA
<213> Escherichia coli K-12 MG1655
<220> purc b2477
<222> 585-684
<223> purc b2477 U00096 complement(2594925..2595638)
<313> 5459: 625-644
<400> 5459
GGACGGTAGC CGCCTGTGGG ACAAAGAAAC GCTGGAGAAA ATGGACAAAG 50
ACCGTTTCCG CCAGAGCCTC GGTGGCCTGA TCGAAGCCTA TGAAGCCGTC 100
<210> 5460
<211> 100
<212> DNA
<213> Escherichia coli K-12 MG1655
<220> purc b2477
<222> 526-625
<223> purc b2477 U00096 complement(2594925..2595638)
<313> 5460: 570-589
<400> 5460
TTCAAGCTGG AATTTGGTCT GTACAAAGGC GAAGTGGTAC TGGGTGATGA 50
GTTCTCCCCG GACGGTAGCC GCCTGTGGGA CAAAGAAACG CTGGAGAAAA 100
<210> 5461
<211> 100

<212> DNA
<213> Escherichia coli K-12 MG1655
<220> purd b4006
<222> 1191-1290
<223> purd b4006 U00096 complement(4202220..4203509)
<313> 5461: 1223-1242
<400> 5461
GCAGAAACGC GCCTATGCCT TAATGACCGA TATCCACTGG GACGACTGCT 50
TCTGCCGGAA AGATATCGGC TGGCGCGCTA TCGAACGCGA GCAGAACTAA 100
<210> 5462
<211> 100
<212> DNA
<213> Escherichia coli K-12 MG1655
<220> purd b4006
<222> 1147-1246
<223> purd b4006 U00096 complement(4202220..4203509)
<313> 5462: 1190-1209
<400> 5462
CGCGTACTGT GCGTCACCGC GCTGGGTCAT ACCGTGGCAG AAGCGCAGAA 50
ACGCGCCTAT GCCTTAATGA CCGATATCCA CTGGGACGAC TGCTTCTGCC 100
<210> 5463
<211> 100
<212> DNA
<213> Escherichia coli K-12 MG1655
<220> purd b4006
<222> 1099-1198
<223> purd b4006 U00096 complement(4202220..4203509)
<313> 5463: 1123-1142
<400> 5463
GCGGGCACAA AACTGGCGGA TGACGAGCAG GTAGTGACCA ACGGCGGGCG 50
CGTACTGTGC GTCACCGCGC TGGGTCATAC CGTGGCAGAA GCGCAGAAAC 100
<210> 5464
<211> 100
<212> DNA
<213> Escherichia coli K-12 MG1655
<220> pure b0524
<222> 411-510
<223> pure b0524 U00096 complement(551814..552323)
<313> 5464: 490-509
<400> 5464
TGCGACTCAT GATAAAGAAC TGCACCAGCG TCTGAATGAC TGGCGCAAAG 50
CCCAGACCGA CGAAGTGCTG GAAAACCCGG ACCCGCGAGG TGCGGCATGA 100
<210> 5465
<211> 100
<212> DNA
<213> Escherichia coli K-12 MG1655
<220> pure b0524
<222> 389-488
<223> pure b0524 U00096 complement(551814..552323)
<313> 5465: 438-457
<400> 5465
CGTTACTGGC AGCACAAATT CTTGCGACTC ATGATAAAGA ACTGCACCAG 50
CGTCTGAATG ACTGGCGCAA AGCCCAGACC GACGAAGTGC TGGAAAACCC 100
<210> 5466
<211> 100
<212> DNA
<213> Escherichia coli K-12 MG1655
<220> pure b0524
<222> 377-476
<223> pure b0524 U00096 complement(551814..552323)
<313> 5466: 393-412
<400> 5466
CGGCAAACGC GGCGTTACTG GCAGCACAAA TTCTTGCGAC TCATGATAAA 50
GAACTGCACC AGCGTCTGAA TGACTGGCGC AAAGCCCAGA CCGACGAAGT 100
<210> 5467
<211> 100
<212> DNA
<213> Escherichia coli K-12 MG1655
<220> purf b2313
<222> 1419-1518
<223> purf b2313 U00096 complement(2426741..2428258)

<313> 5467: 1474-1493
<400> 5467
GGGCTACCTC GATTTCCTCG ATACGTTACG TAATGATGAC GCCAAAGCAG 50
TGCAACGTCA GAACGAAGTG GAAAATCTCG AAATGCATAA CGAAGGATGA 100
<210> 5468
<211> 100
<212> DNA
<213> Escherichia coli K-12 MG1655
<220> purf b2313
<222> 1303-1402
<223> purf b2313 U00096 complement(2426741..2428258)
<313> 5468: 1334-1353
<400> 5468
TTGATTTTCC AGGATCTGAA CGATCTGATC GACGCCGTTC GCGCTGAAAA 50
TCCGGATATC CAGCAGTTTG AATGCTCGGT GTTCAACGGC GTCTACGTCA 100
<210> 5469
<211> 100
<212> DNA
<213> Escherichia coli K-12 MG1655
<220> purf b2313
<222> 1144-1243
<223> purf b2313 U00096 complement(2426741..2428258)
<313> 5469: 1178-1197
<400> 5469
ATGGCACGCG AAGCCGGAGC GAAGAAAGTG TACCTCGCTT CTGCGGCACC 50
GGAAATTCGC TTCCCGAACG TTTATGGTAT TGATATGCCG AGCGCCACGG 100
<210> 5470
<211> 100
<212> DNA
<213> Escherichia coli K-12 MG1655
<220> purh b4007
<222> 1491-1590
<223> purh b4007 U00096 complement(4203521..4205110)
<313> 5470: 1570-1589
<400> 5470
CCAGCCTGGC GGTTCTATCC GTGATGACGA AGTGATTGCC GCCGCCGACG 50
AGCACGGTAT TGCGATGCTC TTCACCGACA TGCGCCACTT CCGCCATTAA 100
<210> 5471
<211> 100
<212> DNA
<213> Escherichia coli K-12 MG1655
<220> purh b4007
<222> 1472-1571
<223> purh b4007 U00096 complement(4203521..4205110)
<313> 5471: 1514-1533
<400> 5471
CGGGCGTGAC CTGCGTAATC CAGCCTGGCG GTTCTATCCG TGATGACGAA 50
GTGATTGCCG CCGCCGACGA GCACGGTATT GCGATGCTCT TCACCGACAT 100
<210> 5472
<211> 100
<212> DNA
<213> Escherichia coli K-12 MG1655
<220> purh b4007
<222> 1442-1541
<223> purh b4007 U00096 complement(4203521..4205110)
<313> 5472: 1473-1492
<400> 5472
TCCGCGACGG TATTGATGCC GCCGCCGCTG CGGGCGTGAC CTGCGTAATC 50
CAGCCTGGCG GTTCTATCCG TGATGACGAA GTGATTGCCG CCGCCGACGA 100
<210> 5473
<211> 100
<212> DNA
<213> Escherichia coli K-12 MG1655
<220> ppa b4227
<222> 427-526
<223> ppa b4227 U00096 complement(4446699..4447229)
<313> 5473: 492-511
<400> 5473
AAAGACCTCG AAAAAGGCAA GTGGGTGAAA GTTGAAGGTT GGGAAAACGC 50
AGAAGCCGCT AAAGCTGAAA TCGTTGCCTC CTTCGAGCGC GCAAAGAATA 100
<210> 5474

<211> 100
<212> DNA
<213> Escherichia coli K-12 MG1655
<220> ppa b4227
<222> 388-487
<223> ppa b4227 U00096 complement(4446699..4447229)
<313> 5474: 449-468
<400> 5474
CTGCTGAAAG CGCAAATCGC TCACTTCTTC GAGCACTACA AAGACCTCGA 50
AAAAGGCAAG TGGGTGAAAG TTGAAGGTTG GGAAAACGCA GAAGCCGCTA 100
<210> 5475
<211> 100
<212> DNA
<213> Escherichia coli K-12 MG1655
<220> ppa b4227
<222> 364-463
<223> ppa b4227 U00096 complement(4446699..4447229)
<313> 5475: 397-416
<400> 5475
AAAGACGTTA ACGATCTGCC TGAACTGCTG AAAGCGCAAA TCGCTCACTT 50
CTTCGAGCAC TACAAAGACC TCGAAAAAGG CAAGTGGGTG AAAGTTGAAG 100
<210> 5476
<211> 100
<212> DNA
<213> Escherichia coli K-12 MG1655
<220> purk b0523
<222> 969-1068
<223> purk b0523 U00096 complement(550750..551817)
<313> 5476: 1049-1068
<400> 5476
CGACACATCG CGTCTGACTG CGACGCTGGA AGCCTTAATC CCGCTGCTGC 50
CGCCGGAATA TGCCAGCGGC GTGATTTGGG CGCAGAGTAA GTTCGGTTAA 100
<210> 5477
<211> 100
<212> DNA
<213> Escherichia coli K-12 MG1655
<220> purk b0523
<222> 940-1039
<223> purk b0523 U00096 complement(550750..551817)
<313> 5477: 1004-1023
<400> 5477
AAAGTGGGGC ATCTGAATTT GACCGACAGC GACACATCGC GTCTGACTGC 50
GACGCTGGAA GCCTTAATCC CGCTGCTGCC GCCGGAATAT GCCAGCGGCG 100
<210> 5478
<211> 100
<212> DNA
<213> Escherichia coli K-12 MG1655
<220> purk b0523
<222> 901-1000
<223> purk b0523 U00096 complement(550750..551817)
<313> 5478: 959-978
<400> 5478
CATCTGCACT GGTACGACAA AGAAGTCCGT CCGGGGCGTA AAGTGGGGCA 50
TCTGAATTTG ACCGACAGCG ACACATCGCG TCTGACTGCG ACGCTGGAAG 100
<210> 5479
<211> 100
<212> DNA
<213> Escherichia coli K-12 MG1655
<220> purl b2558
<222> 3707-3806
<223> purl b2558 U00096 complement(2689676..2693563)
<313> 5479: 3752-3771
<400> 5479
ACCCGAACGG TTCACCGAAC GGTATTACGG CAGTCACGAC TGAAAGTGGT 50
CGAGTCACCA TTATGATGCC GCACCCGGAA CGTGTTTTCC GTACTGTCAG 100
<210> 5480
<211> 100
<212> DNA
<213> Escherichia coli K-12 MG1655
<220> purl b2558
<222> 3662-3761

<223> purl b2558 U00096 complement(2689676..2693563)
<313> 5480: 3699-3718
<400> 5480
GCTATGTCGA TAACTTCGGC AAAGTCACTG AAACCTACCC GGCTAACCCG  50
AACGGTTCAC CGAACGGTAT TACGGCAGTC ACGACTGAAA GTGGTCGAGT  100
<210> 5481
<211> 100
<212> DNA
<213> Escherichia coli K-12 MG1655
<220> purl b2558
<222> 3604-3703
<223> purl b2558 U00096 complement(2689676..2693563)
<313> 5481: 3634-3653
<400> 5481
GAAGTGCGTG ATGCGGCGCA TCTGGCGGCA CTGGAAAGCA AAGGGCTGGT  50
GGCACTGCGC TATGTCGATA ACTTCGGCAA AGTCACTGAA ACCTACCCGG  100
<210> 5482
<211> 100
<212> DNA
<213> Escherichia coli K-12 MG1655
<220> ppc b3957
<222> 2526-2625
<223> ppc b3957 U00096 complement(4148026..4150677)
<313> 5482: 2561-2580
<400> 5482
GCAGGCCGAG TTGCTGCACC GCTCCCGCCA GGCAGAAAAA GAAGGCCAGG  50
AACCGGATCC TCGCGTCGAA CAAGCGTTAA TGGTCACTAT TGCCGGGATT  100
<210> 5483
<211> 100
<212> DNA
<213> Escherichia coli K-12 MG1655
<220> ppc b3957
<222> 2458-2557
<223> ppc b3957 U00096 complement(4148026..4150677)
<313> 5483: 2507-2526
<400> 5483
GCCGATCTGC CGTGGATTGC AGAGTCTATT CAGCTACGGA ATATTTACAC  50
CGACCCGCTG AACGTATTGC AGGCCGAGTT GCTGCACCGC TCCCGCCAGG  100
<210> 5484
<211> 100
<212> DNA
<213> Escherichia coli K-12 MG1655
<220> ppc b3957
<222> 2398-2497
<223> ppc b3957 U00096 complement(4148026..4150677)
<313> 5484: 2433-2452
<400> 5484
AACCTGCAAG AAGAAGACAT CAAAGTGGTG CTGGCGATTG CCAACGATTC  50
CCATCTGATG GCCGATCTGC CGTGGATTGC AGAGTCTATT CAGCTACGGA  100
<210> 5485
<211> 100
<212> DNA
<213> Escherichia coli K-12 MG1655
<220> purm b2500
<222> 939-1038
<223> purm b2500 U00096 2619217..2620254
<313> 5485: 987-1006
<400> 5485
GGACAAAGCC CTCGCCCTGC TCAATGCCAA CGGTGAAAAC GCGTGGAAAA  50
TCGGTATCAT CAAAGCCTCT GATTCCGAAC AACGCGTGGT TATCGAATAA  100
<210> 5486
<211> 100
<212> DNA
<213> Escherichia coli K-12 MG1655
<220> purm b2500
<222> 914-1013
<223> purm b2500 U00096 2619217..2620254
<313> 5486: 949-968
<400> 5486
TTATTGCCCT GCCTGCTCCG GAAGTGGACA AAGCCCTCGC CCTGCTCAAT  50
GCCAACGGTG AAAACGCGTG GAAAATCGGT ATCATCAAAG CCTCTGATTC  100

<210> 5487
<211> 100
<212> DNA
<213> Escherichia coli K-12 MG1655
<220> purm b2500
<222> 821-920
<223> purm b2500 U00096 2619217..2620254
<313> 5487: 880-899
<400> 5487
GGCAGTGGCC GGAAGTGTTC AACTGGCTGC AAACGGCAGG TAACGTTGAG 50
CACCATGAAA TGTATCGCAC CTTCAACTGC GGCGTCGGGA TGATTATTGC 100
<210> 5488
<211> 100
<212> DNA
<213> Escherichia coli K-12 MG1655
<220> purn b2501
<222> 540-639
<223> purn b2501 U00096 2620254..2620892
<313> 5488: 602-621
<400> 5488
GATTAGCTGG TTTGCCGATG GTCGTCTGAA AATGCACGAA AACGCCGCGT 50
GGCTGGATGG TCAACGTCTG CCGCCGCAGG GCTACGCTGC CGACGAGTAA 100
<210> 5489
<211> 100
<212> DNA
<213> Escherichia coli K-12 MG1655
<220> purn b2501
<222> 461-560
<223> purn b2501 U00096 2620254..2620892
<313> 5489: 500-519
<400> 5489
TCCCGGTATT TGCTGGTGAT TCGGAAGATG ACATCACCGC CCGCGTGCAA 50
ACCCAGGAAC ACGCCATTTA TCCACTGGTG ATTAGCTGGT TTGCCGATGG 100
<210> 5490
<211> 100
<212> DNA
<213> Escherichia coli K-12 MG1655
<220> purn b2501
<222> 400-499
<223> purn b2501 U00096 2620254..2620892
<313> 5490: 437-456
<400> 5490
ACATCGGTGC ATTTCGTCAC CGATGAACTG GACGGTGGCC CGGTTATTTT 50
ACAGGCGAAA GTCCCGGTAT TTGCTGGTGA TTCGGAAGAT GACATCACCG 100
<210> 5491
<211> 100
<212> DNA
<213> Escherichia coli K-12 MG1655
<220> b3000 b3000
<222> 327-426
<223> b3000 b3000 U00096 complement(3145281..3145706)
<313> 5491: 399-418
<400> 5491
TATCGCCCTG GCACCTGACG GCTTAAGTTC CGTTGGAGGT TATCCGGGAA 50
ATGATGATAA AGGTCGTGAG CTGCAACAGC AGGTTGATCC AACAAACTGA 100
<210> 5492
<211> 100
<212> DNA
<213> Escherichia coli K-12 MG1655
<220> b3000 b3000
<222> 247-346
<223> b3000 b3000 U00096 complement(3145281..3145706)
<313> 5492: 290-309
<400> 5492
GCCGTAGTGG TGGTGCATGA GAATCGTGGA CTGAATCCGT ATATCGAAGA 50
TGTGGCACGG CGAGTGGCGA AGGCGGGGTA TATCGCCCTG GCACCTGACG 100
<210> 5493
<211> 100
<212> DNA
<213> Escherichia coli K-12 MG1655
<220> b3000 b3000

<222> 218-317
<223> b3000 b3000 U00096 complement(3145281..3145706)
<313> 5493: 254-273
<400> 5493
AGCCCGCAAA GATGAGCGGC AAAACGCCAG CCGTAGTGGT GGTGCATGAG  50
AATCGTGGAC TGAATCCGTA TATCGAAGAT GTGGCACGGC GAGTGGCGAA  100
<210> 5494
<211> 100
<212> DNA
<213> Escherichia coli K-12 MG1655
<220> b3001 b3002
<222> 906-1005
<223> b3001 b3002 U00096 3145912..3146952
<313> 5494: 927-946
<400> 5494
CAGCCGCGCG GAGCAACTAG AGGAGAACGT GCAGGCGCTG AATAATCTGA  50
CATTTAGCAC CAAGGAGCTG GCGCAGATTG ATCAGCATAT CGCCGATGGC  100
<210> 5495
<211> 100
<212> DNA
<213> Escherichia coli K-12 MG1655
<220> b3001 b3002
<222> 803-902
<223> b3001 b3002 U00096 3145912..3146952
<313> 5495: 858-877
<400> 5495
GCTTATTGAA TGAAATGGCA CAGCAGCGTG GACAATCAAT GGCGCAAATG  50
GCGTTAAGCT GGTTGCTGAA AGATGATCGC GTGACGTCGG TATTGATTGG  100
<210> 5496
<211> 100
<212> DNA
<213> Escherichia coli K-12 MG1655
<220> b3001 b3002
<222> 760-859
<223> b3001 b3002 U00096 3145912..3146952
<313> 5496: 815-834
<400> 5496
CTGACACCGA AAATGCTTAC CGAAGCCAAC CTCAACAGCC TGCGCTTATT  50
GAATGAAATG GCACAGCAGC GTGGACAATC AATGGCGCAA ATGGCGTTAA  100
<210> 5497
<211> 100
<212> DNA
<213> Escherichia coli K-12 MG1655
<220> purr b1659
<222> 927-1026
<223> purr b1659 U00096 1735868..1736893
<313> 5497: 981-1000
<400> 5497
CGTCAACAAA CGTGAAGAAC CGCAGTCTAT TGAAGTGCAT CCGCGCTTGA  50
TTGAACGCCG CTCCGTGGCT GACGGCCCGT TCCGCGACTA TCGTCGTTAA  100
<210> 5498
<211> 100
<212> DNA
<213> Escherichia coli K-12 MG1655
<220> purr b1659
<222> 898-997
<223> purr b1659 U00096 1735868..1736893
<313> 5498: 952-971
<400> 5498
ACAGCGTTCA ACATGCTGTT GGATCGTATC GTCAACAAAC GTGAAGAACC  50
GCAGTCTATT GAAGTGCATC CGCGCTTGAT TGAACGCCGC TCCGTGGCTG  100
<210> 5499
<211> 100
<212> DNA
<213> Escherichia coli K-12 MG1655
<220> purr b1659
<222> 844-943
<223> purr b1659 U00096 1735868..1736893
<313> 5499: 892-911
<400> 5499
TATTTTACGC CGGCGCTGAC CACGATCCAT CAGCCAAAAG ATTCGCTGGG  50

TGAAACAGCG TTCAACATGC TGTTGGATCG TATCGTCAAC AAACGTGAAG 100
<210> 5500
<211> 100
<212> DNA
<213> Escherichia coli K-12 MG1655
<220> b3004 b3005
<222> 147-246
<223> b3004 b3005 U00096 3148737..3148982
<313> 5500: 214-233
<400> 5500
CCGCCTGATG CAGCGTATCC ATTACCTTCA TCAACGTCTC GTAATCGACG 50
GTTTTGTCCG CTCGGAAGAA GATGGTGGTG TCTTTCTTGC CTTCGGTTAA 100
<210> 5501
<211> 100
<212> DNA
<213> Escherichia coli K-12 MG1655
<220> b3004 b3005
<222> 123-222
<223> b3004 b3005 U00096 3148737..3148982
<313> 5501: 171-190
<400> 5501
CGACCAGACC TATCTTCAGG TAACCCGCCT GATGCAGCGT ATCCATTACC 50
TTCATCAACG TCTCGTAATC GACGGTTTTG TCCGCTCGGA AGAAGATGGT 100
<210> 5502
<211> 100
<212> DNA
<213> Escherichia coli K-12 MG1655
<220> b3004 b3005
<222> 69-168
<223> b3004 b3005 U00096 3148737..3148982
<313> 5502: 126-145
<400> 5502
TGAGCGTAGC GCATCAGGCA ATTCTACTTT ACTTCGCTTT GGCGGTTTCT 50
TCGCCGACCA GACCTATCTT CAGGTAACCC GCCTGATGCA GCGTATCCAT 100
<210> 5503
<211> 100
<212> DNA
<213> Escherichia coli K-12 MG1655
<220> purt b1850
<222> 1080-1179
<223> purt b1850 U00096 1928905..1930083
<313> 5503: 1142-1161
<400> 5503
CAGCCGTCGT CTGGGGGTGG CACTGGCTAC TGCAGAGAGT GTTGTTGACG 50
CCATTGAACG CGCGAAGCAC GCCGCCGGAC AGGTAAAAGT ACAGGGTTAA 100
<210> 5504
<211> 100
<212> DNA
<213> Escherichia coli K-12 MG1655
<220> purt b1850
<222> 1021-1120
<223> purt b1850 U00096 1928905..1930083
<313> 5504: 1048-1067
<400> 5504
CAGAATGCCG TAGGCGCAGA TTTGCAGATT CGTTTATTTG GTAAGCCGGA 50
AATTGATGGC AGCCGTCGTC TGGGGGTGGC ACTGGCTACT GCAGAGAGTG 100
<210> 5505
<211> 100
<212> DNA
<213> Escherichia coli K-12 MG1655
<220> purt b1850
<222> 985-1084
<223> purt b1850 U00096 1928905..1930083
<313> 5505: 1005-1024
<400> 5505
CAACTGACCA GTCAGAATGT CACGTTTGAT AATGTGCAGA ATGCCGTAGG 50
CGCAGATTTG CAGATTCGTT TATTTGGTAA GCCGGAAATT GATGGCAGCC 100
<210> 5506
<211> 100
<212> DNA
<213> Escherichia coli K-12 MG1655

<220> ppk b2502
<222> 1968-2067
<223> ppk b2502 U00096 2621064..2623130
<313> 5506: 2036-2055
<400> 5506
AGAACTCAGT AATCGCTACG TTCCCCGCGG CAATCGCCGC AAAGTACGGG  50
CGCAGTTGGC GATTTATGAC TACATCAAAT CACTCGAACA ACCTGAATAA  100
<210> 5507
<211> 100
<212> DNA
<213> Escherichia coli K-12 MG1655
<220> ppk b2502
<222> 1876-1975
<223> ppk b2502 U00096 2621064..2623130
<313> 5507: 1920-1939
<400> 5507
ACGCCGCTGC TCGATCCGCG CCTGAAGCAG CGGGTACTGG ACATCATCGA  50
CATATTGTTC AGCGATACGG TCAAAGCACG TTATATCGAT AAAGAACTCA  100
<210> 5508
<211> 100
<212> DNA
<213> Escherichia coli K-12 MG1655
<220> ppk b2502
<222> 1770-1869
<223> ppk b2502 U00096 2621064..2623130
<313> 5508: 1817-1836
<400> 5508
TGAACATGAC CGGGTTTATA TTTTTGAAAA TGGCGGCGAT AAAAAGGTCT  50
ACCTTTCTTC CGCCGACTGG ATGACGCGCA ATATTGATTA TCGTATTGAA  100
<210> 5509
<211> 100
<212> DNA
<213> Escherichia coli K-12 MG1655
<220> puru b1233
<222> 742-841
<223> puru b1233 U00096 complement(1287005..1287847)
<313> 5509: 800-819
<400> 5509
CGCGCAGGTC GTGACGTCGA GAAAAACGTC TTAAGTCGTG CACTATACAA  50
AGTACTGGCA CAGCGCGTCT TTGTTTACGG TAATCGAACG ATTATTCTTT  100
<210> 5510
<211> 100
<212> DNA
<213> Escherichia coli K-12 MG1655
<220> puru b1233
<222> 654-753
<223> puru b1233 U00096 complement(1287005..1287847)
<313> 5510: 684-703
<400> 5510
CTATGTGAAT GACAATCTGG ACGAAGGCCC AATCATCATG CAGGACGTTA  50
TTCATGTCGA TCATACCTAC ACAGCTGAAG ATATGATGCG CGCAGGTCGT  100
<210> 5511
<211> 100
<212> DNA
<213> Escherichia coli K-12 MG1655
<220> puru b1233
<222> 621-720
<223> puru b1233 U00096 complement(1287005..1287847)
<313> 5511: 650-669
<400> 5511
ACGTGGTGTG AAGATTATTG GCGCAACCGC TCACTATGTG AATGACAATC  50
TGGACGAAGG CCCAATCATC ATGCAGGACG TTATTCATGT CGATCATACC  100
<210> 5512
<211> 100
<212> DNA
<213> Escherichia coli K-12 MG1655
<220> b3007 b3008
<222> 57-156
<223> b3007 b3008 U00096 complement(3149992..3150147)
<313> 5512: 123-142
<400> 5512

GTATGCAAAT AGTAATGAGA ACGACTATCA ATTCGACGTC GTTTTGATAT 50
TATTATGCGC AGATTTTGTG ACTTGCGTCC TGGAGATACA CAGTGGGTAA 100
<210> 5513
<211> 100
<212> DNA
<213> Escherichia coli K-12 MG1655
<220> b3007 b3008
<222> 13-112
<223> b3007 b3008 U00096 complement(3149992..3150147)
<313> 5513: 59-78
<400> 5513
TTTTTACTGC GTAAACGCAA GGAAAAGAAA GCAAAGGTAC GGCAGTATGC 50
AAATAGTAAT GAGAACGACT ATCAATTCGA CGTCGTTTTG ATATTATTAT 100
<210> 5514
<211> 100
<212> DNA
<213> Escherichia coli K-12 MG1655
<220> b3007 b3008
<222> 25-124
<223> b3007 b3008 U00096 complement(3149992..3150147)
<313> 5514: 44-63
<400> 5514
AAACGCAAGG AAAAGAAAGC AAAGGTACGG CAGTATGCAA ATAGTAATGA 50
GAACGACTAT CAATTCGACG TCGTTTTGAT ATTATTATGC GCAGATTTTG 100
<210> 5515
<211> 100
<212> DNA
<213> Escherichia coli K-12 MG1655
<220> ftsa b0095
<222> 1164-1263
<223> ftsa b0095 U00096 103982..105244
<313> 5515: 1224-1243
<400> 5515
GTCACATCTT AACGGTGAAG CTGAAGTAGA AAAACGTGTT ACAGCATCAG 50
TTGGCTCGTG GATCAAGCGA CTCAATAGTT GGCTGCGAAA AGAGTTTTAA 100
<210> 5516
<211> 100
<212> DNA
<213> Escherichia coli K-12 MG1655
<220> ftsa b0095
<222> 1113-1212
<223> ftsa b0095 U00096 103982..105244
<313> 5516: 1142-1161
<400> 5516
TCAGGAGCCG TATTATTCGA CGGCGGTGGG ATTGCTTCAC TATGGGAAAG 50
AGTCACATCT TAACGGTGAA GCTGAAGTAG AAAAACGTGT TACAGCATCA 100
<210> 5517
<211> 100
<212> DNA
<213> Escherichia coli K-12 MG1655
<220> ftsa b0095
<222> 929-1028
<223> ftsa b0095 U00096 103982..105244
<313> 5517: 978-997
<400> 5517
AAGAGATATT GCAGTTGCAG GAAAAGCTTC GCCAACAAGG GGTTAAACAT 50
CACCTGGCGG CAGGCATTGT ATTAACCGGT GGCGCAGCGC AGATCGAAGG 100
<210> 5518
<211> 100
<212> DNA
<213> Escherichia coli K-12 MG1655
<220> ftse b3464
<222> 570-669
<223> ftse b3464 U00096 complement(3599710..3600378)
<313> 5518: 636-655
<400> 5518
ATTGATGGCA ACGCACGACA TCAACCTGAT CTCGCGGCGT TCCTATCGCA 50
TGCTCACCCT GAGCGATGGT CACTTGCATG GAGGCGTGGG CCATGAATAA 100
<210> 5519
<211> 100
<212> DNA

<213> Escherichia coli K-12 MG1655
<220> ftse b3464
<222> 561-660
<223> ftse b3464 U00096 complement(3599710..3600378)
<313> 5519: 583-602
<400> 5519
GGTAACCGTA TTGATGGCAA CGCACGACAT CAACCTGATC TCGCGGCGTT 50
CCTATCGCAT GCTCACCCTG AGCGATGGTC ACTTGCATGG AGGCGTGGGC 100
<210> 5520
<211> 100
<212> DNA
<213> Escherichia coli K-12 MG1655
<220> ftse b3464
<222> 509-608
<223> ftse b3464 U00096 complement(3599710..3600378)
<313> 5520: 544-563
<400> 5520
ACGCGCTGTC GGAAGGCATT TTACGTCTGT TTGAAGAGTT TAACCGCGTT 50
GGGGTAACCG TATTGATGGC AACGCACGAC ATCAACCTGA TCTCGCGGCG 100
<210> 5521
<211> 100
<212> DNA
<213> Escherichia coli K-12 MG1655
<220> ftsi b0085
<222> 1664-1763
<223> ftsi b0085 U00096 91413..93179
<313> 5521: 1697-1716
<400> 5521
GCGGCGTATT GCGTACCATG AACATCGAGC CGGATGCGCT GACAACGGGC 50
GATAAAAATG AATTTGTGAT TAATCAAGGC GAGGGGACAG GTGGCAGATC 100
<210> 5522
<211> 100
<212> DNA
<213> Escherichia coli K-12 MG1655
<220> ftsi b0085
<222> 1452-1551
<223> ftsi b0085 U00096 91413..93179
<313> 5522: 1505-1524
<400> 5522
GGCGATTAAA GGCTATCGTA TCGCCATTAA AACCGGTACC GCGAAAAAGG 50
TCGGGCCGGA CGGTCGCTAC ATCAATAAAT ATATTGCTTA TACCGCAGGC 100
<210> 5523
<211> 100
<212> DNA
<213> Escherichia coli K-12 MG1655
<220> ftsi b0085
<222> 1394-1493
<223> ftsi b0085 U00096 91413..93179
<313> 5523: 1448-1467
<400> 5523
GCACTGTGGT GCATATGATG GAAAGCGTGG CGCTACCAGG CGGCGGCGGC 50
GTGAAGGCGG CGATTAAAGG CTATCGTATC GCCATTAAAA CCGGTACCGC 100
<210> 5524
<211> 100
<212> DNA
<213> Escherichia coli K-12 MG1655
<220> ftsj b3180
<222> 465-564
<223> ftsj b3180 U00096 complement(3324676..3325305)
<313> 5524: 499-518
<400> 5524
AGCGCCAGGT GGCAGTTTTG TAGTGAAGGT GTTCCAGGGC GAAGGTTTCG 50
ATGAGTATCT AAGGGAAATT CGCTCCCTGT TTACGAAGGT CAAAGTTCGT 100
<210> 5525
<211> 100
<212> DNA
<213> Escherichia coli K-12 MG1655
<220> ftsj b3180
<222> 323-422
<223> ftsj b3180 U00096 complement(3324676..3325305)
<313> 5525: 387-406

<400> 5525
CACTGCTGGA GCGCGTTGGC GACAGCAAAG TCCAGGTTGT CATGTCCGAT  50
ATGGCACCAA ACATGAGCGG AACACCGGCG GTGGATATCC CCCGTGCCAT  100
<210> 5526
<211> 100
<212> DNA
<213> Escherichia coli K-12 MG1655
<220> ftsj b3180
<222> 270-369
<223> ftsj b3180 U00096 complement(3324676..3325305)
<313> 5526: 298-317
<400> 5526
CGTTGGTGTG GACTTTCTTC AGGGCGATTT TCGTGATGAA CTGGTGATGA  50
AAGCACTGCT GGAGCGCGTT GGCGACAGCA AAGTCCAGGT TGTCATGTCC  100
<210> 5527
<211> 100
<212> DNA
<213> Escherichia coli K-12 MG1655
<220> ftsk b0891
<222> 3891-3990
<223> ftsk b0891 U00096 932447..936436
<313> 5527: 3971-3990
<400> 5527
TGCAGCGCGT ATTATCGAAC AGATGGAAGC GCAGGGGATT GTCAGCGAAC  50
AGGGGCACAA CGGTAATCGT GAAGTGCTGG CCCCACCGCC GTTTGACTAA  100
<210> 5528
<211> 100
<212> DNA
<213> Escherichia coli K-12 MG1655
<220> ftsk b0891
<222> 3891-3990
<223> ftsk b0891 U00096 932447..936436
<313> 5528: 3917-3936
<400> 5528
TGCAGCGCGT ATTATCGAAC AGATGGAAGC GCAGGGGATT GTCAGCGAAC  50
AGGGGCACAA CGGTAATCGT GAAGTGCTGG CCCCACCGCC GTTTGACTAA  100
<210> 5529
<211> 100
<212> DNA
<213> Escherichia coli K-12 MG1655
<220> ftsk b0891
<222> 3800-3899
<223> ftsk b0891 U00096 932447..936436
<313> 5529: 3839-3858
<400> 5529
CGTTGTTCGA TCAGGCGGTG CAGTTTGTCA CTGAAAAACG CAAAGCGTCA  50
ATTTCTGGCG TACAGCGTCA GTTCCGCATT GGTTATAACC GTGCAGCGCG  100
<210> 5530
<211> 100
<212> DNA
<213> Escherichia coli K-12 MG1655
<220> ppx ppx
<222> 1443-1542
<223> ppx ppx U00096 2623135..2624676
<313> 5530: 1507-1526
<400> 5530
GGTACTGCTT GATCTGGAAA AGGAGCAAGA ATACTGGGAA GGCGTGGCTG  50
GCTGGCGGTT GAAAATTGAA GAAGAAAGTA CACCAGAAAT CGCCGCTTAA  100
<210> 5531
<211> 100
<212> DNA
<213> Escherichia coli K-12 MG1655
<220> ppx ppx
<222> 1408-1507
<223> ppx ppx U00096 2623135..2624676
<313> 5531: 1461-1480
<400> 5531
CGTTTCCCGC ATGACTGGTT TAGTCAGAAT GCGCTGGTAC TGCTTGATCT  50
GGAAAAGGAG CAAGAATACT GGGAAGGCGT GGCTGGCTGG CGGTTGAAAA  100
<210> 5532
<211> 100

<212> DNA
<213> Escherichia coli K-12 MG1655
<220> ppx ppx
<222> 1389-1488
<223> ppx ppx U00096 2623135..2624676
<313> 5532: 1428-1447
<400> 5532
TGACAGTCAC TGGACACTGC GTTTCCCGCA TGACTGGTTT AGTCAGAATG 50
CGCTGGTACT GCTTGATCTG GAAAAGGAGC AAGAATACTG GGAAGGCGTG 100
<210> 5533
<211> 100
<212> DNA
<213> Escherichia coli K-12 MG1655
<220> ftsl b0084
<222> 267-366
<223> ftsl b0084 U00096 91032..91397
<313> 5533: 288-307
<400> 5533
TGCGCTCGGC GACCATAGCC GGGTGGAAAG GATCGCCACG GAAAAGCTGC 50
AAATGCAGCA TGTTGATCCG TCACAAGAAA ATATCGTAGT GCAAAAATAA 100
<210> 5534
<211> 100
<212> DNA
<213> Escherichia coli K-12 MG1655
<220> ftsl b0084
<222> 215-314
<223> ftsl b0084 U00096 91032..91397
<313> 5534: 250-269
<400> 5534
TGGAGCGAGA TGCTTTAGAC ATTGAATGGC GCAACCTGAT CCTTGAAGAG 50
AATGCGCTCG GCGACCATAG CCGGGTGGAA AGGATCGCCA CGGAAAAGCT 100
<210> 5535
<211> 100
<212> DNA
<213> Escherichia coli K-12 MG1655
<220> ftsl b0084
<222> 142-241
<223> ftsl b0084 U00096 91032..91397
<313> 5535: 185-204
<400> 5535
TTGACGGCGG TGACTGTGGT AACCACGGCG CACCATACCC GTTTACTGAC 50
CGCTCAGCGC GAACAACTGG TGCTGGAGCG AGATGCTTTA GACATTGAAT 100
<210> 5536
<211> 100
<212> DNA
<213> Escherichia coli K-12 MG1655
<220> ftsn b3934
<222> 857-956
<223> ftsn b3934 U00096 complement(4119960..4120919)
<313> 5536: 881-900
<400> 5536
TGGTCATTGG CCCGGTGAAA GGCAAAGAGA ACGCAGACAG CACCCTCAAT 50
CGGTTGAAGA TGGCGGGTCA TACAAACTGC ATTCGGCTCG CCGCCGGGGG 100
<210> 5537
<211> 100
<212> DNA
<213> Escherichia coli K-12 MG1655
<220> ftsn b3934
<222> 796-895
<223> ftsn b3934 U00096 complement(4119960..4120919)
<313> 5537: 828-847
<400> 5537
GCTCAGCTGG CGTTCGAAGG CTTTGACTCG AAAATCACCA CCAACAATGG 50
CTGGAATCGT GTGGTCATTG GCCCGGTGAA AGGCAAAGAG AACGCAGACA 100
<210> 5538
<211> 100
<212> DNA
<213> Escherichia coli K-12 MG1655
<220> ftsn b3934
<222> 729-828
<223> ftsn b3934 U00096 complement(4119960..4120919)

<313> 5538: 770-789
<400> 5538
AGACGAACGC CGCTGGATGG TGCAGTGCGG TTCGTTCAGA GGCGCGGAAC 50
AGGCAGAGAC GGTACGTGCT CAGCTGGCGT TCGAAGGCTT TGACTCGAAA 100
<210> 5539
<211> 100
<212> DNA
<213> Escherichia coli K-12 MG1655
<220> ftsq b0094
<222> 732-831
<223> ftsq b0094 U00096 103155..103985
<313> 5539: 810-829
<400> 5539
TGATTTGCGT TATGACTCTG GAGCGGCAGT AGGCTGGGCG CCCTTGCCGC 50
CAGAGGAATC TACTCAGCAA CAAAATCAGG CACAGGCAGA ACAACAATGA 100
<210> 5540
<211> 100
<212> DNA
<213> Escherichia coli K-12 MG1655
<220> ftsq b0094
<222> 676-775
<223> ftsq b0094 U00096 103155..103985
<313> 5540: 707-726
<400> 5540
CTTTATCCGG TTTTACAGCA GCAGGCGCAA ACCGATGGCA AACGGATTAG 50
CTACGTTGAT TTGCGTTATG ACTCTGGAGC GGCAGTAGGC TGGGCGCCCT 100
<210> 5541
<211> 100
<212> DNA
<213> Escherichia coli K-12 MG1655
<220> ftsq b0094
<222> 570-669
<223> ftsq b0094 U00096 103155..103985
<313> 5541: 596-615
<400> 5541
AGCGGCGATG ACCGCGCGGC GTTCCTGGCA GTTGACGCTG AATAACGATA 50
TTAAGCTCAA TCTTGGCCGG GGCGATACGA TGAAACGTTT GGCTCGCTTT 100
<210> 5542
<211> 100
<212> DNA
<213> Escherichia coli K-12 MG1655
<220> ftsw b0090
<222> 1065-1164
<223> ftsw b0090 U00096 98403..99647
<313> 5542: 1093-1112
<400> 5542
GGCGCTGGTT AACGTAGGCG CGGCGGCGGG GATGTTACCG ACCAAAGGTC 50
TGACATTGCC GCTGATCAGT TACGGTGGTT CGAGCTTACT GATTATGTCG 100
<210> 5543
<211> 100
<212> DNA
<213> Escherichia coli K-12 MG1655
<220> ftsw b0090
<222> 1011-1110
<223> ftsw b0090 U00096 98403..99647
<313> 5543: 1062-1081
<400> 5543
CCACCGTTTT TCCGGTTTTC TCGCCTGTTC TATTGGCATC TGGTTTAGCT 50
TCCAGGCGCT GGTTAACGTA GGCGCGGCGG CGGGGATGTT ACCGACCAAA 100
<210> 5544
<211> 100
<212> DNA
<213> Escherichia coli K-12 MG1655
<220> ftsw b0090
<222> 992-1091
<223> ftsw b0090 U00096 98403..99647
<313> 5544: 1027-1046
<400> 5544
GTAAAGCATT AGAAATTGAC CACCGTTTTT CCGGTTTTCT CGCCTGTTCT 50
ATTGGCATCT GGTTTAGCTT CCAGGCGCTG GTTAACGTAG GCGCGGCGGC 100
<210> 5545

<211> 100
<212> DNA
<213> Escherichia coli K-12 MG1655
<220> ftsx b3463
<222> 960-1059
<223> ftsx b3463 U00096 complement(3598659..3599717)
<313> 5545: 1021-1040
<400> 5545
CGATGAATGC CTGCTATTGC TGCTGGTATG CTCGATGATT GGCTGGGTGG 50
CAGCGTGGCT TGCCACGGTA CAACATTTAC GCCACTTTAC GCCTGAATAA 100
<210> 5546
<211> 100
<212> DNA
<213> Escherichia coli K-12 MG1655
<220> ftsx b3463
<222> 931-1030
<223> ftsx b3463 U00096 complement(3598659..3599717)
<313> 5546: 998-1017
<400> 5546
ACGAAGTTTG ATATCAATGG CTTATCATTC GATGAATGCC TGCTATTGCT 50
GCTGGTATGC TCGATGATTG GCTGGGTGGC AGCGTGGCTT GCCACGGTAC 100
<210> 5547
<211> 100
<212> DNA
<213> Escherichia coli K-12 MG1655
<220> ftsx b3463
<222> 776-875
<223> ftsx b3463 U00096 complement(3598659..3599717)
<313> 5547: 825-844
<400> 5547
AACTGATTGG TGCGACAGAT GGATTCATCC TGCGCCCGTT CCTGTATGGT 50
GGCGCACTGC TGGGATTTTC TGGCGCATTG TTGTCATTAA TTTTGTCAGA 100
<210> 5548
<211> 100
<212> DNA
<213> Escherichia coli K-12 MG1655
<220> ftsy b3465
<222> 1333-1432
<223> ftsy b3465 U00096 complement(3600381..3601874)
<313> 5548: 1392-1411
<400> 5548
CTAACGAAAC TGGACGGCAC GGCGAAAGGC GGGGTAATTT TCTCGGTGGC 50
TGACCAGTTT GGTATCCCTA TCCGCTACAT TGGTGTCGGC GAACGTATTG 100
<210> 5549
<211> 100
<212> DNA
<213> Escherichia coli K-12 MG1655
<220> ftsy b3465
<222> 1317-1416
<223> ftsy b3465 U00096 complement(3600381..3601874)
<313> 5549: 1340-1359
<400> 5549
CTTAACCGGC ATCACGCTAA CGAAACTGGA CGGCACGGCG AAAGGCGGGG 50
TAATTTTCTC GGTGGCTGAC CAGTTTGGTA TCCCTATCCG CTACATTGGT 100
<210> 5550
<211> 100
<212> DNA
<213> Escherichia coli K-12 MG1655
<220> ftsy b3465
<222> 1246-1345
<223> ftsy b3465 U00096 complement(3600381..3601874)
<313> 5550: 1302-1321
<400> 5550
ATGCTGACTA TTGATGCCAG CACCGGGCAG AACGCGGTAA GCCAGGCCAA 50
ACTGTTCCAT GAAGCCGTTG GCTTAACCGG CATCACGCTA ACGAAACTGG 100
<210> 5551
<211> 100
<212> DNA
<213> Escherichia coli K-12 MG1655
<220> ftsz b0096
<222> 1053-1152

<223> ftsz b0096 U00096 105305..106456
<313> 5551: 1095-1114
<400> 5551
GAAGCCGGTT GCTAAAGTCG TGAATGACAA TGCGCCGCAA ACTGCGAAAG 50
AGCCGGATTA TCTGGATATC CCAGCATTCC TGCGTAAGCA AGCTGATTAA 100
<210> 5552
<211> 100
<212> DNA
<213> Escherichia coli K-12 MG1655
<220> ftsz b0096
<222> 988-1087
<223> ftsz b0096 U00096 105305..106456
<313> 5552: 1035-1054
<400> 5552
CAGGTTCAGC AGCCAGTGAT GGATCGCTAC CAGCAGCATG GGATGGCTCC 50
GCTGACCCAG GAGCAGAAGC CGGTTGCTAA AGTCGTGAAT GACAATGCGC 100
<210> 5553
<211> 100
<212> DNA
<213> Escherichia coli K-12 MG1655
<220> ftsz b0096
<222> 950-1049
<223> ftsz b0096 U00096 105305..106456
<313> 5553: 980-999
<400> 5553
TGGACAAACG TCCTGAAATC ACTCTGGTGA CCAATAAGCA GGTTCAGCAG 50
CCAGTGATGG ATCGCTACCA GCAGCATGGG ATGGCTCCGC TGACCCAGGA 100
<210> 5554
<211> 100
<212> DNA
<213> Escherichia coli K-12 MG1655
<220> b3015 b3016
<222> 1143-1242
<223> b3015 b3016 U00096 complement(3156944..3158185)
<313> 5554: 1204-1223
<400> 5554
GCGTCAGACG CCTGCTACGG CAAAAAAAGC GTCGTCTACG CAATCTCGTC 50
CGGTGAATGC TGGTGCGAAG AAACGGCCTA AAGCGGCGGT TGGACGTTAA 100
<210> 5555
<211> 100
<212> DNA
<213> Escherichia coli K-12 MG1655
<220> b3015 b3016
<222> 1135-1234
<223> b3015 b3016 U00096 complement(3156944..3158185)
<313> 5555: 1164-1183
<400> 5555
GCGACCCAGC GTCAGACGCC TGCTACGGCA AAAAAAGCGT CGTCTACGCA 50
ATCTCGTCCG GTGAATGCTG GTGCGAAGAA ACGGCCTAAA GCGGCGGTTG 100
<210> 5556
<211> 100
<212> DNA
<213> Escherichia coli K-12 MG1655
<220> b3015 b3016
<222> 1100-1199
<223> b3015 b3016 U00096 complement(3156944..3158185)
<313> 5556: 1124-1143
<400> 5556
ATACCCGTCC GGCGTTGACG AAACATACGC CGATGGCGAC CCAGCGTCAG 50
ACGCCTGCTA CGGCAAAAAA AGCGTCGTCT ACGCAATCTC GTCCGGTGAA 100
<210> 5557
<211> 100
<212> DNA
<213> Escherichia coli K-12 MG1655
<220> puta b1015
<222> 3737-3836
<223> puta b1015 U00096 complement(1074143..1078105)
<313> 5557: 3763-3782
<400> 5557
AAAATATAAC CGCTCAACCG TTTGATGCGG TGATCTTCCA CGGTGATTCG 50
GATCAGCTTC GCGCATTGTG TGAAGCAGTT GCCGCGCGGG ATGGCACAAT 100

<210> 5558
<211> 100
<212> DNA
<213> Escherichia coli K-12 MG1655
<220> puta b1015
<222> 3686-3785
<223> puta b1015 U00096 complement(1074143..1078105)
<313> 5558: 3717-3736
<400> 5558
TGAAGGCATT GCCATCGGCA GTCAGCGAAC GTATTCAACT GGCGAAAGCG 50
GAAAATATAA CCGCTCAACC GTTTGATGCG GTGATCTTCC ACGGTGATTC 100
<210> 5559
<211> 100
<212> DNA
<213> Escherichia coli K-12 MG1655
<220> puta b1015
<222> 3643-3742
<223> puta b1015 U00096 complement(1074143..1078105)
<313> 5559: 3680-3699
<400> 5559
AGCCAGGTAC TGTGGCCGGA TGACGCGCTG CATCGTCAGT TAGTGAAGGC 50
ATTGCCATCG GCAGTCAGCG AACGTATTCA ACTGGCGAAA GCGGAAAATA 100
<210> 5560
<211> 100
<212> DNA
<213> Escherichia coli K-12 MG1655
<220> pncb b0932
<222> 1104-1203
<223> pncb b0932 U00096 complement(988377..989579)
<313> 5560: 1174-1193
<400> 5560
ACTTTCTGAC AGCCCTGGCA AAACTATCTG CCATGATAAA GCGTTTGTTC 50
GGGCGCTGCG CAAAGCGTTC GACCTTCCGC ATATTAAAAA AGCCAGTTAA 100
<210> 5561
<211> 100
<212> DNA
<213> Escherichia coli K-12 MG1655
<220> pncb b0932
<222> 1084-1183
<223> pncb b0932 U00096 complement(988377..989579)
<313> 5561: 1119-1138
<400> 5561
AACGGTAAAC CGGTGGCGAA ACTTTCTGAC AGCCCTGGCA AAACTATCTG 50
CCATGATAAA GCGTTTGTTC GGGCGCTGCG CAAAGCGTTC GACCTTCCGC 100
<210> 5562
<211> 100
<212> DNA
<213> Escherichia coli K-12 MG1655
<220> pncb b0932
<222> 985-1084
<223> pncb b0932 U00096 complement(988377..989579)
<313> 5562: 1029-1048
<400> 5562
TCTTCCCGCG TGCAATTAAG TTTTGGTATT GGGACTCGCC TGACCTGCGA 50
TATCCCCCAG GTAAAACCCC TGAATATTGT CATTAAGTTG GTAGAGTGTA 100
<210> 5563
<211> 100
<212> DNA
<213> Escherichia coli K-12 MG1655
<220> sela b3592
<222> 1292-1391
<223> sela b3592 U00096 complement(3757485..3758876)
<313> 5563: 1348-1367
<400> 5563
TGCCAGTGCC GGTGATTGGT CGTATTTATG ACGGACGATT GTGGCTGGAT 50
TTACGCTGCC TTGAAGATGA GCAACGGTTT TTGGAGATGT TGTTGAAATG 100
<210> 5564
<211> 100
<212> DNA
<213> Escherichia coli K-12 MG1655
<220> sela b3592

<222> 1205-1304
<223> sela b3592 U00096 complement(3757485..3758876)
<313> 5564: 1258-1277
<400> 5564
ATCGCCTGCC GAGCGCGGCA TTAACGTTTA CACCCCATGA TGGACGCGGT 50
AGCCACCTTG AGTCATTAGC CGCCCGCTGG CGTGAATTGC CAGTGCCGGT 100
<210> 5565
<211> 100
<212> DNA
<213> Escherichia coli K-12 MG1655
<220> sela b3592
<222> 1155-1254
<223> sela b3592 U00096 complement(3757485..3758876)
<313> 5565: 1208-1227
<400> 5565
ACAGGTTATG CCATGTCTTT CGCAGATTGG CAGTGGTTCG CTGCCGGTTG 50
ATCGCCTGCC GAGCGCGGCA TTAACGTTTA CACCCCATGA TGGACGCGGT 100
<210> 5566
<211> 100
<212> DNA
<213> Escherichia coli K-12 MG1655
<220> apt b0470
<222> 446-545
<223> apt b0470 U00096 490636..491187
<313> 5566: 502-521
<400> 5566
TGGCTGACGC TGCGTTCATT ATCAACCTGT TCGATCTCGG CGGCGAACAG 50
CGTCTCGAAA AACAGGGCAT TACCAGCTAC AGCCTTGTCC CGTTCCCGGG 100
<210> 5567
<211> 100
<212> DNA
<213> Escherichia coli K-12 MG1655
<220> apt b0470
<222> 418-517
<223> apt b0470 U00096 490636..491187
<313> 5567: 463-482
<400> 5567
AAACTGATCC GTCGTCTGGG TGGTGAAGTG GCTGACGCTG CGTTCATTAT 50
CAACCTGTTC GATCTCGGCG GCGAACAGCG TCTCGAAAAA CAGGGCATTA 100
<210> 5568
<211> 100
<212> DNA
<213> Escherichia coli K-12 MG1655
<220> apt b0470
<222> 369-468
<223> apt b0470 U00096 490636..491187
<313> 5568: 403-422
<400> 5568
GGTGGTGGAC GACCTGCTGG CAACCGGCGG CACTATCGAA GCGACCGTTA 50
AACTGATCCG TCGTCTGGGT GGTGAAGTGG CTGACGCTGC GTTCATTATC 100
<210> 5569
<211> 100
<212> DNA
<213> Escherichia coli K-12 MG1655
<220> prc b1831
<222> 1943-2042
<223> prc b1831 U00096 complement(1910792..1912840)
<313> 5569: 1991-2010
<400> 5569
ATTACCAGGA GCCGGATCCT TATCTGGATG AGACGGTGAA TATCGCACTC 50
GATCTGGCGA AGCTTGAAAA AGCCAGACCC GCGGAACAAC CCGCTCCCGT 100
<210> 5570
<211> 100
<212> DNA
<213> Escherichia coli K-12 MG1655
<220> prc b1831
<222> 1900-1999
<223> prc b1831 U00096 complement(1910792..1912840)
<313> 5570: 1938-1957
<400> 5570
GAAGGTAAAC CGGAGTTGAA GAAACTGGAT GATCTACCGA AAGATTACCA 50

GGAGCCGGAT CCTTATCTGG ATGAGACGGT GAATATCGCA CTCGATCTGG 100
<210> 5571
<211> 100
<212> DNA
<213> Escherichia coli K-12 MG1655
<220> prc b1831
<222> 1851-1950
<223> prc b1831 U00096 complement(1910792..1912840)
<313> 5571: 1895-1914
<400> 5571
TGAAGATGAT GCGACGCGTC TGGCGCGTTT GAACGAACGC TTTAAACGCG 50
AAGGTAAACC GGAGTTGAAG AAACTGGATG ATCTACCGAA AGATTACCAG 100
<210> 5572
<211> 100
<212> DNA
<213> Escherichia coli K-12 MG1655
<220> selb b3591
<222> 1649-1748
<223> selb b3591 U00096 complement(3755644..3757488)
<313> 5572: 1682-1701
<400> 5572
TTGTCGAGTT TGCCAATATG ATCCGCGATC TCGATCAGGA GTGTGGTTCA 50
ACCTGCGCGG CGGATTTCCG CGATCGCTTA GGCGTAGGCC GAAAGCTGGC 100
<210> 5573
<211> 100
<212> DNA
<213> Escherichia coli K-12 MG1655
<220> selb b3591
<222> 1542-1641
<223> selb b3591 U00096 complement(3755644..3757488)
<313> 5573: 1584-1603
<400> 5573
AGAGACGGGA ACCGACGAGC AGGCAATGCG CCTGACTCTA CGCCAGGCGG 50
CGCAGCAAGG AATAATTACC GCGATCGTTA AAGATCGTTA TTACCGTAAC 100
<210> 5574
<211> 100
<212> DNA
<213> Escherichia coli K-12 MG1655
<220> selb b3591
<222> 1522-1621
<223> selb b3591 U00096 complement(3755644..3757488)
<313> 5574: 1546-1565
<400> 5574
TGGGTGCGTG ACCTGGCAAA AGAGACGGGA ACCGACGAGC AGGCAATGCG 50
CCTGACTCTA CGCCAGGCGG CGCAGCAAGG AATAATTACC GCGATCGTTA 100
<210> 5575
<211> 100
<212> DNA
<213> Escherichia coli K-12 MG1655
<220> b3020 b3020
<222> 1436-1535
<223> b3020 b3020 U00096 complement(3164127..3165734)
<313> 5575: 1474-1493
<400> 5575
AGCGTAATGC GTTGTATCAG CAGGCAGAAG TGATCATCAA CCAACAGGCA 50
CCGCTGATTC CTATCTACTA TCAGCCGTTA ATCAAACTGC TTAAACCCTA 100
<210> 5576
<211> 100
<212> DNA
<213> Escherichia coli K-12 MG1655
<220> b3020 b3020
<222> 1365-1464
<223> b3020 b3020 U00096 complement(3164127..3165734)
<313> 5576: 1411-1430
<400> 5576
CGTCGGTCAC TGGAAAAATG CGCAGTATGA CGCCTTACTA AACCAGGCCA 50
CGCAGATCAC TGATGCGACA AAGCGTAATG CGTTGTATCA GCAGGCAGAA 100
<210> 5577
<211> 100
<212> DNA
<213> Escherichia coli K-12 MG1655

<220> b3020 b3020
<222> 1333-1432
<223> b3020 b3020 U00096 complement(3164127..3165734)
<313> 5577: 1367-1386
<400> 5577
CTGAACACGC TCAAAAGCGA TAGTGAAGAA AACGTCGGTC ACTGGAAAAA 50
TGCGCAGTAT GACGCCTTAC TAAACCAGGC CACGCAGATC ACTGATGCGA 100
<210> 5578
<211> 100
<212> DNA
<213> Escherichia coli K-12 MG1655
<220> seld b1765
<222> 931-1030
<223> seld b1765 U00096 complement(1844989..1846032)
<313> 5578: 981-1000
<400> 5578
CCGGAAGCAG AAAATGAGGT CAAAGCTACA GCCGCCGAGT TTGGCATTGA 50
ACTGACGGCA ATTGGCGAAC TGGTGCCAGC GCGCGGCGGT CGTGCCATGG 100
<210> 5579
<211> 100
<212> DNA
<213> Escherichia coli K-12 MG1655
<220> seld b1765
<222> 886-985
<223> seld b1765 U00096 complement(1844989..1846032)
<313> 5579: 931-950
<400> 5579
CTGTGTGATC CGCAAACTTC TGGCGGTTTG CTGCTGGCGG TCATGCCGGA 50
AGCAGAAAAT GAGGTCAAAG CTACAGCCGC CGAGTTTGGC ATTGAACTGA 100
<210> 5580
<211> 100
<212> DNA
<213> Escherichia coli K-12 MG1655
<220> seld b1765
<222> 861-960
<223> seld b1765 U00096 complement(1844989..1846032)
<313> 5580: 884-903
<400> 5580
AATGCCGCGT GAAGTGCGCG ATCTGCTGTG TGATCCGCAA ACTTCTGGCG 50
GTTTGCTGCT GGCGGTCATG CCGGAAGCAG AAAATGAGGT CAAAGCTACA 100
<210> 5581
<211> 100
<212> DNA
<213> Escherichia coli K-12 MG1655
<220> b3021 b3021
<222> 297-396
<223> b3021 b3021 U00096 complement(3165867..3166262)
<313> 5581: 324-343
<400> 5581
TTCGCGTTAC GAAAAAGGCA ATGCCCAACC TCATCCTTCC ACAATCAAAC 50
TTTTACGTGT TCTGGATAAG CATCCAGAAC TATTGAATGA AATCCGTTAA 100
<210> 5582
<211> 100
<212> DNA
<213> Escherichia coli K-12 MG1655
<220> b3021 b3021
<222> 239-338
<223> b3021 b3021 U00096 complement(3165867..3166262)
<313> 5582: 265-284
<400> 5582
AGCTCTCTCT TACCCAAAAA GAGGCAAGCG AAATTTTTGG GGGAGGTGTA 50
AATGCGTTTT CGCGTTACGA AAAAGGCAAT GCCCAACCTC ATCCTTCCAC 100
<210> 5583
<211> 100
<212> DNA
<213> Escherichia coli K-12 MG1655
<220> b3021 b3021
<222> 145-244
<223> b3021 b3021 U00096 complement(3165867..3166262)
<313> 5583: 205-224
<400> 5583

GAGTCAGATG CTTTCATGGC GCAAGTAAAG GCATTTCGGG CTTCGGTGAA 50
TGCCGAAACA GTGGCACCTG AATTTATAGT GAAGGTTCGA AAAAAGCTCT 100
<210> 5584
<211> 100
<212> DNA
<213> Escherichia coli K-12 MG1655
<220> lyxk b3581
<222> 1398-1497
<223> lyxk b3581 U00096 3744714..3746210
<313> 5584: 1458-1477
<400> 5584
GACCGCCCAT CAGCTTTACC AAAAAAAATA TCAACGTTAT CAGCATCTCA 50
TTGCCGCACT TCAGGGCTTT CACGCCCGCA TTAAGGAGCA CACATTATGA 100
<210> 5585
<211> 100
<212> DNA
<213> Escherichia coli K-12 MG1655
<220> lyxk b3581
<222> 1354-1453
<223> lyxk b3581 U00096 3744714..3746210
<313> 5585: 1398-1417
<400> 5585
CAACGTGACT TGCGACACCC GGTGCGCACC CTGCTGCCAG ATATGACCGC 50
CCATCAGCTT TACCAAAAAA AATATCAACG TTATCAGCAT CTCATTGCCG 100
<210> 5586
<211> 100
<212> DNA
<213> Escherichia coli K-12 MG1655
<220> lyxk b3581
<222> 1304-1403
<223> lyxk b3581 U00096 3744714..3746210
<313> 5586: 1345-1364
<400> 5586
CCCTTGCCGC CCGCGTCGGC ACCGGGGTTT ATCACAACTT CAGCGAAGCC 50
CAACGTGACT TGCGACACCC GGTGCGCACC CTGCTGCCAG ATATGACCGC 100
<210> 5587
<211> 100
<212> DNA
<213> Escherichia coli K-12 MG1655
<220> putp putP
<222> 1410-1509
<223> putp putP U00096 1078528..1080036
<313> 5587: 1483-1502
<400> 5587
TTTGCTGGGT AAAGCGCCGT CAGCGGCGAT GCAAAAACGC TTTGCCGAGG 50
CCGATGCGCA CTATCATTCG GCTCCGCCGT CACGGTTGCA GGAAAGCTAA 100
<210> 5588
<211> 100
<212> DNA
<213> Escherichia coli K-12 MG1655
<220> putp putP
<222> 1371-1470
<223> putp putP U00096 1078528..1080036
<313> 5588: 1394-1413
<400> 5588
GGGCTTTATC TTCGGCAGTA TTGGGATTGT AGTGTTTAGT TTGCTGGGTA 50
AAGCGCCGTC AGCGGCGATG CAAAAACGCT TTGCCGAGGC CGATGCGCAC 100
<210> 5589
<211> 100
<212> DNA
<213> Escherichia coli K-12 MG1655
<220> putp putP
<222> 1306-1405
<223> putp putP U00096 1078528..1080036
<313> 5589: 1367-1386
<400> 5589
ATCGGTGCGC TGACGGTTAT CGTCTGGAAA CAGTTCGGCT GGCTGGGACT 50
GTACGAAATT ATTCCGGGCT TTATCTTCGG CAGTATTGGG ATTGTAGTGT 100
<210> 5590
<211> 100
<212> DNA

<213> Escherichia coli K-12 MG1655
<220> b3022 b3022
<222> 137-236
<223> b3022 b3022 U00096 complement(3166264..3166560)
<313> 5590: 202-221
<400> 5590
TCATTGGATT ATCAGAGAGC GACTTTTATA AAAGCATGAC CACCTACTCT 50
GATCATACTA TCTGGCAGGA TGTTTACAGA CCCAGGCTTG TTACAGGCCA 100
<210> 5591
<211> 100
<212> DNA
<213> Escherichia coli K-12 MG1655
<220> b3022 b3022
<222> 63-162
<223> b3022 b3022 U00096 complement(3166264..3166560)
<313> 5591: 75-94
<400> 5591
AGTTCGTACA ACACGTAGTG CCCTGTTAAA TGCAGATGAG TTAGGTTTGG 50
ATTTTGATGG TATGTGTAAT GTTATCATTG GATTATCAGA GAGCGACTTT 100
<210> 5592
<211> 100
<212> DNA
<213> Escherichia coli K-12 MG1655
<220> b3022 b3022
<222> 1-100
<223> b3022 b3022 U00096 complement(3166264..3166560)
<313> 5592: 15-34
<400> 5592
ATGGAAAAAC GCACACCACA TACACGTTTG AGTCAGGTTA AAAAACTTGT 50
CAATGCCGGG CAAGTTCGTA CAACACGTAG TGCCCTGTTA AATGCAGATG 100
<210> 5593
<211> 100
<212> DNA
<213> Escherichia coli K-12 MG1655
<220> b3023 b3024
<222> 384-483
<223> b3023 b3024 U00096 complement(3166765..3167247)
<313> 5593: 452-471
<400> 5593
GAAAATGCGT AAAGCACCGA TTCTGTTTCA CTACACCAAT CTTGCCGAAG 50
GGGTGACAGA GCAGCGACTG GAAACGGATG TTTATGTGCC GTTGGCGTGA 100
<210> 5594
<211> 100
<212> DNA
<213> Escherichia coli K-12 MG1655
<220> b3023 b3024
<222> 371-470
<223> b3023 b3024 U00096 complement(3166765..3167247)
<313> 5594: 402-421
<400> 5594
CTGCAAGCGG CGAGAAAATG CGTAAAGCAC CGATTCTGTT TCACTACACC 50
AATCTTGCCG AAGGGGTGAC AGAGCAGCGA CTGGAAACGG ATGTTTATGT 100
<210> 5595
<211> 100
<212> DNA
<213> Escherichia coli K-12 MG1655
<220> b3023 b3024
<222> 315-414
<223> b3023 b3024 U00096 complement(3166765..3167247)
<313> 5595: 368-387
<400> 5595
TGGCGAGCTG GACGATATTT CACACACGGT ATGGGGCATC ATTCGCCACT 50
GGCTGCCTGC AAGCGGCGAG AAAATGCGTA AAGCACCGAT TCTGTTTCAC 100
<210> 5596
<211> 100
<212> DNA
<213> Escherichia coli K-12 MG1655
<220> b3027 b3028
<222> 234-333
<223> b3027 b3028 U00096 complement(3169895..3170227)
<313> 5596: 309-328

<400> 5596
GCGGGAAATG TTCGAGTTTT GGGTATATGT ATTCTCGCTC GGTATTTGTC 50
TGGTTTGTGG TTGGTGGCTT TTTTGGGGAC GGTTTATATT TTGCTATTAG 100
<210> 5597
<211> 100
<212> DNA
<213> Escherichia coli K-12 MG1655
<220> b3027 b3028
<222> 144-243
<223> b3027 b3028 U00096 complement(3169895..3170227)
<313> 5597: 181-200
<400> 5597
TTCAGGCGAA GATCGTATAT TGCAAGCAAT GATTGCAGCA TTTTTTCTTC 50
TTACGCCACT TATTATTCTT ATCTTAAGAA AACTCTTTAT GCGGGAAATG 100
<210> 5598
<211> 100
<212> DNA
<213> Escherichia coli K-12 MG1655
<220> b3027 b3028
<222> 50-149
<223> b3027 b3028 U00096 complement(3169895..3170227)
<313> 5598: 98-117
<400> 5598
TGCTTACTTA TTGGTTAATA GATTCTTTTT TTGCTTTCAA TAAGTATGAC 50
TGGATGCTTG AGTCGGGAGG AAATATTTGC AGTATACCTT CCGTTTCAGG 100
<210> 5599
<211> 100
<212> DNA
<213> Escherichia coli K-12 MG1655
<220> iap b2754
<222> 939-1038
<223> iap b2754 U00096 2874604..2875641
<313> 5599: 995-1014
<400> 5599
ACATATTGAT AAGGCTCTTC CTGGAAGAAT AGAACGTCGC TGCCGTGACG 50
TTATGCGGAT AATGCTACCT CTGGTGAAGG AGTTGGCGAA GGCGTCTTGA 100
<210> 5600
<211> 100
<212> DNA
<213> Escherichia coli K-12 MG1655
<220> iap b2754
<222> 862-961
<223> iap b2754 U00096 2874604..2875641
<313> 5600: 901-920
<400> 5600
GGTTATCAGC AACGCGCAAA AACACCTGCC TTCCCGGCGG GAAATAGCTG 50
GCATGACGTA AGACTGGATA ATCACCAACA TATTGATAAG GCTCTTCCTG 100
<210> 5601
<211> 100
<212> DNA
<213> Escherichia coli K-12 MG1655
<220> iap b2754
<222> 785-884
<223> iap b2754 U00096 2874604..2875641
<313> 5601: 829-848
<400> 5601
ACGCAGAAAT ATTCGACAAA GCGGGCATTG CTGTACTTTC GGTGGAAGCG 50
ACTAACTGGA ATCTTGGGAA TAAGGATGGT TATCAGCAAC GCGCAAAAAC 100
<210> 5602
<211> 100
<212> DNA
<213> Escherichia coli K-12 MG1655
<220> tbpa b0069
<222> 885-984
<223> tbpa b0069 U00096 complement(74497..75480)
<313> 5602: 949-968
<400> 5602
ATTGACCAAA CCCGCAACCA CGTTGGAGTT CACGCCAGCC GAAGTGGCGG 50
CACAACGTCA GGCATGGATT AGCGAATGGC AACGCGCCGT CAGCCGTTAA 100
<210> 5603
<211> 100

<212> DNA
<213> Escherichia coli K-12 MG1655
<220> tbpa b0069
<222> 852-951
<223> tbpa b0069 U00096 complement(74497..75480)
<313> 5603: 905-924
<400> 5603
GGCAAACGTC ACGCTGCCTG CCGGTTTTGA AAAATTGACC AAACCCGCAA 50
CCACGTTGGA GTTCACGCCA GCCGAAGTGG CGGCACAACG TCAGGCATGG 100
<210> 5604
<211> 100
<212> DNA
<213> Escherichia coli K-12 MG1655
<220> tbpa b0069
<222> 839-938
<223> tbpa b0069 U00096 complement(74497..75480)
<313> 5604: 870-889
<400> 5604
GGATGTATCC GGTGGCAAAC GTCACGCTGC CTGCCGGTTT TGAAAAATTG 50
ACCAAACCCG CAACCACGTT GGAGTTCACG CCAGCCGAAG TGGCGGCACA 100
<210> 5605
<211> 100
<212> DNA
<213> Escherichia coli K-12 MG1655
<220> psd b4161
<222> 870-969
<223> psd b4161 U00096 complement(4386970..4387938)
<313> 5605: 949-968
<400> 5605
TGTTACGCCA GACGCTGAAC CTGCCCCGCT TCCTGCTGAA GAGATCGAAG 50
CAGAACACGA CGCCAGCCCA TTGGTTGACG ACAAAAAAGA CCAGGTCTAA 100
<210> 5606
<211> 100
<212> DNA
<213> Escherichia coli K-12 MG1655
<220> psd b4161
<222> 870-969
<223> psd b4161 U00096 complement(4386970..4387938)
<313> 5606: 895-914
<400> 5606
TGTTACGCCA GACGCTGAAC CTGCCCCGCT TCCTGCTGAA GAGATCGAAG 50
CAGAACACGA CGCCAGCCCA TTGGTTGACG ACAAAAAAGA CCAGGTCTAA 100
<210> 5607
<211> 100
<212> DNA
<213> Escherichia coli K-12 MG1655
<220> psd b4161
<222> 828-927
<223> psd b4161 U00096 complement(4386970..4387938)
<313> 5607: 865-884
<400> 5607
CACGAAAATT GGCCAGCCGC TGGCAGTATC TACCGAAACC TTTGTTACGC 50
CAGACGCTGA ACCTGCCCCG CTTCCTGCTG AAGAGATCGA AGCAGAACAC 100
<210> 5608
<211> 100
<212> DNA
<213> Escherichia coli K-12 MG1655
<220> lrha lrhA
<222> 840-939
<223> lrha lrhA U00096 complement(2403723..2404661)
<313> 5608: 876-895
<400> 5608
CTATCAGGCG ATGGAGAGCT ACCATAATCC GTGGCAATAC AGCCCAATGT 50
CTGCTCCGGA AGGGGATGAT TCATTGTTGA TTGAAAGGGA TATCGAGTAA 100
<210> 5609
<211> 100
<212> DNA
<213> Escherichia coli K-12 MG1655
<220> lrha lrhA
<222> 795-894
<223> lrha lrhA U00096 complement(2403723..2404661)

<313> 5609: 829-848
<400> 5609
GCTGTGCTAT GATCCGTCCA GTAATAATGA GCTGGCCCAG GTGATCTATC 50
AGGCGATGGA GAGCTACCAT AATCCGTGGC AATACAGCCC AATGTCTGCT 100
<210> 5610
<211> 100
<212> DNA
<213> Escherichia coli K-12 MG1655
<220> lrha lrhA
<222> 709-808
<223> lrha lrhA U00096 complement(2403723..2404661)
<313> 5610: 771-790
<400> 5610
GCAAGGCCGG TTGAGATGAT GAGCCCGGAC CTGCGTGTGC TGAGTGGAGT 50
AGATGGCTTA CCGCCGCTAC CTGATACCGA ATATTTGCTG TGCTATGATC 100
<210> 5611
<211> 100
<212> DNA
<213> Escherichia coli K-12 MG1655
<220> b2304 b2305
<222> 795-894
<223> b2304 b2305 U00096 2419728..2420621
<313> 5611: 834-853
<400> 5611
ATTAGGTGGA CAACGCGCGC TGCCTAAACG GCTGGAAGAA GCGGGTTTTG 50
CGTTTCGCTG GTACGATTTA GAAGAGGCGC TGGCGGATGT CGTTCGCTGA 100
<210> 5612
<211> 100
<212> DNA
<213> Escherichia coli K-12 MG1655
<220> b2304 b2305
<222> 699-798
<223> b2304 b2305 U00096 2419728..2420621
<313> 5612: 752-771
<400> 5612
TGCCCATGCG CTCGGTCATG CGCTGCATCG CCCGGCCATT TTGCGCGTCC 50
CCGCGACCGC CATTCGACTG TTAATGGGCG AATCTTCAGT ACTGGTATTA 100
<210> 5613
<211> 100
<212> DNA
<213> Escherichia coli K-12 MG1655
<220> b2304 b2305
<222> 654-753
<223> b2304 b2305 U00096 2419728..2420621
<313> 5613: 699-718
<400> 5613
GCCATTTAAT ATGGTTTCGC CCTACCCGGT ACGCAATGAA CAATTTGCCC 50
ATGCGCTCGG TCATGCGCTG CATCGCCCGG CCATTTTGCG CGTCCCCGCG 100
<210> 5614
<211> 100
<212> DNA
<213> Escherichia coli K-12 MG1655
<220> yrah b3143
<222> 486-585
<223> yrah b3143 U00096 3285067..3285651
<313> 5614: 541-560
<400> 5614
TGTTGACCTC ATCGCTTACT ACGTATCAAC CGTAGTTCCA TCAGAAATCA 50
CCCCAGGGGA AGCAGACGCT ATCGTTAACG TAACGCTGGA TTACCGTTAA 100
<210> 5615
<211> 100
<212> DNA
<213> Escherichia coli K-12 MG1655
<220> yrah b3143
<222> 413-512
<223> yrah b3143 U00096 3285067..3285651
<313> 5615: 466-485
<400> 5615
TTATGCCACT GAATGCTATG GGCAATGATA ACACGGTTTA TCAGATCCCG 50
GCGGAATCCA ATGGTATTGT CAATGTTGAC CTCATCGCTT ACTACGTATC 100
<210> 5616

<211> 100
<212> DNA
<213> Escherichia coli K-12 MG1655
<220> yrah b3143
<222> 377-476
<223> yrah b3143 U00096 3285067..3285651
<313> 5616: 407-426
<400> 5616
TGGGTATCCG TATTGACGAT GCGGAAAGCG GAAACATTAT GCCACTGAAT 50
GCTATGGGCA ATGATAACAC GGTTTATCAG ATCCCGGCGG AATCCAATGG 100
<210> 5617
<211> 100
<212> DNA
<213> Escherichia coli K-12 MG1655
<220> yrai b3144
<222> 587-686
<223> yrai b3144 U00096 3285731..3286426
<313> 5617: 642-661
<400> 5617
GAGAACATAA ACAGTTACCA CTTCCTGCAT CTGGCGGAAA GAACGTGAAA 50
TGGGCTGCGA TCAATGATTA TGGCGGCAGT TCCGGGACAG AAACTCGTCC 100
<210> 5618
<211> 100
<212> DNA
<213> Escherichia coli K-12 MG1655
<220> yrai b3144
<222> 564-663
<223> yrai b3144 U00096 3285731..3286426
<313> 5618: 587-606
<400> 5618
CAAAATTGAA TATATCGCGC CCGGAGAACA TAAACAGTTA CCACTTCCTG 50
CATCTGGCGG AAAGAACGTG AAATGGGCTG CGATCAATGA TTATGGCGGC 100
<210> 5619
<211> 100
<212> DNA
<213> Escherichia coli K-12 MG1655
<220> yrai b3144
<222> 509-608
<223> yrai b3144 U00096 3285731..3286426
<313> 5619: 545-564
<400> 5619
CTGAATATTA CGTCGTATTT GCCGGACTGG CAGTCGATAA AACCGATCTC 50
ACAAGCAAAA TTGAATATAT CGCGCCCGGA GAACATAAAC AGTTACCACT 100
<210> 5620
<211> 100
<212> DNA
<213> Escherichia coli K-12 MG1655
<220> yraj b3145
<222> 2418-2517
<223> yraj b3145 U00096 3286455..3288971
<313> 5620: 2498-2517
<400> 5620
CTGGGGAAAT CAGGCAGATC AACAATGTCA GTTTGCTTTC AGTACACCGG 50
ATTCAGAACC TACAACCTCT GTATTACAAG GCACAGCGCA GTGCCATTAA 100
<210> 5621
<211> 100
<212> DNA
<213> Escherichia coli K-12 MG1655
<220> yraj b3145
<222> 2418-2517
<223> yraj b3145 U00096 3286455..3288971
<313> 5621: 2439-2458
<400> 5621
CTGGGGAAAT CAGGCAGATC AACAATGTCA GTTTGCTTTC AGTACACCGG 50
ATTCAGAACC TACAACCTCT GTATTACAAG GCACAGCGCA GTGCCATTAA 100
<210> 5622
<211> 100
<212> DNA
<213> Escherichia coli K-12 MG1655
<220> yraj b3145
<222> 2363-2462

<223> yraj b3145 U00096 3286455..3288971
<313> 5622: 2401-2420
<400> 5622
GCGGCATACT ATATCTCTCT GGGATATCGA GTAAATCACA AAGCTGGACT  50
GTACGCTGGG GAAATCAGGC AGATCAACAA TGTCAGTTTG CTTTCAGTAC  100
<210> 5623
<211> 100
<212> DNA
<213> Escherichia coli K-12 MG1655
<220> yrak b3146
<222> 993-1092
<223> yrak b3146 U00096 3288982..3290073
<313> 5623: 1054-1073
<400> 5623
CACCGTCAAT ATGCGCGCCT GGCCAGTTAA TCTGGTCGGT GGTGAGCTGG  50
AAACAGGAAA ATTTCAGGGC ACAGCCACCA TTACCGTCAT CGTGCGGTAA  100
<210> 5624
<211> 100
<212> DNA
<213> Escherichia coli K-12 MG1655
<220> yrak b3146
<222> 948-1047
<223> yrak b3146 U00096 3288982..3290073
<313> 5624: 975-994
<400> 5624
TCCTTTTCAG AACGGTATCC TTCCCGTTGA TCCTTCCGGG CATGGCACCG  50
TCAATATGCG CGCCTGGCCA GTTAATCTGG TCGGTGGTGA GCTGGAAACA  100
<210> 5625
<211> 100
<212> DNA
<213> Escherichia coli K-12 MG1655
<220> yrak b3146
<222> 881-980
<223> yrak b3146 U00096 3288982..3290073
<313> 5625: 933-952
<400> 5625
GAAGTTCAGA CAATGTGCCC GATGTCGGTA TTCGTATTGA AAATCTCGGC  50
GGCGGAGTTG CAAATATTCC TTTTCAGAAC GGTATCCTTC CCGTTGATCC  100
<210> 5626
<211> 100
<212> DNA
<213> Escherichia coli K-12 MG1655
<220> yral b3147
<222> 762-861
<223> yral b3147 U00096 complement(3290116..3290976)
<313> 5626: 835-854
<400> 5626
GGCAGAACTG CCGCTGAAAA AAGCGGCGGC GCTGGCCGCA GAAATTCACG  50
GCGTGAAGAA AAATGCGCTG TATAAGTATG CGCTGGAGCA GCAGGGGTAA  100
<210> 5627
<211> 100
<212> DNA
<213> Escherichia coli K-12 MG1655
<220> yral b3147
<222> 678-777
<223> yral b3147 U00096 complement(3290116..3290976)
<313> 5627: 722-741
<400> 5627
CGAAATGGTG CTGATTGTCG AAGGTCATAA AGCACAGGAA GAAGACTTAC  50
CCGCCGATGC CCTGCGCACG CTGGCGCTGC TACAGGCAGA ACTGCCGCTG  100
<210> 5628
<211> 100
<212> DNA
<213> Escherichia coli K-12 MG1655
<220> yral b3147
<222> 616-715
<223> yral b3147 U00096 complement(3290116..3290976)
<313> 5628: 683-702
<400> 5628
CACGGCGCGC CCGTTGGCGA GCTGCTGGCG TGGGTAAAGG AAGATGAAAA  50
CCGTCGCAAA GGCGAAATGG TGCTGATTGT CGAAGGTCAT AAAGCACAGG  100

<210> 5629
<211> 100
<212> DNA
<213> Escherichia coli K-12 MG1655
<220> yram b3148
<222> 1938-2037
<223> yram b3148 U00096 3291041..3293077
<313> 5629: 2005-2024
<400> 5629
CGGTAATACC GGAAGCCTGA CGGCTAACCC GGATTGCGTG ATTAACAGGA  50
ACTTATCATG GCTACAGTAC CAACAAGGTC AGGTAGTCCC CGTCAGTTAA  100
<210> 5630
<211> 100
<212> DNA
<213> Escherichia coli K-12 MG1655
<220> yram b3148
<222> 1907-2006
<223> yram b3148 U00096 3291041..3293077
<313> 5630: 1957-1976
<400> 5630
AAATGCGCCA GGTTCAGGGT TTTGAAATCA ACGGTAATAC CGGAAGCCTG  50
ACGGCTAACC CGGATTGCGT GATTAACAGG AACTTATCAT GGCTACAGTA  100
<210> 5631
<211> 100
<212> DNA
<213> Escherichia coli K-12 MG1655
<220> yram b3148
<222> 1865-1964
<223> yram b3148 U00096 3291041..3293077
<313> 5631: 1890-1909
<400> 5631
CGATGGGCGT CGATGCCTGG TCGCTGGCAA ATCATTTCTC ACAAATGCGC  50
CAGGTTCAGG GTTTTGAAAT CAACGGTAAT ACCGGAAGCC TGACGGCTAA  100
<210> 5632
<211> 100
<212> DNA
<213> Escherichia coli K-12 MG1655
<220> yran b3149
<222> 297-396
<223> yran b3149 U00096 3293035..3293430
<313> 5632: 343-362
<400> 5632
TAATGGGAGT TTTGATACTG TGGATTGCCG GTTCGATGTG GTAGCCTTCA  50
CCGGGAATGA GGTTGAGTGG ATTAAGGATG CCTTTAATGA CCACTCATAA  100
<210> 5633
<211> 100
<212> DNA
<213> Escherichia coli K-12 MG1655
<220> yran b3149
<222> 282-381
<223> yran b3149 U00096 3293035..3293430
<313> 5633: 311-330
<400> 5633
GTGGCTCGCG CGTCATAATG GGAGTTTTGA TACTGTGGAT TGCCGGTTCG  50
ATGTGGTAGC CTTCACCGGG AATGAGGTTG AGTGGATTAA GGATGCCTTT  100
<210> 5634
<211> 100
<212> DNA
<213> Escherichia coli K-12 MG1655
<220> yran b3149
<222> 212-311
<223> yran b3149 U00096 3293035..3293430
<313> 5634: 258-277
<400> 5634
CGCTTTATGG CGGCGCGGCA GCCAGTGTGA CCCGCAGCAA ACAACACAAA  50
TTATTACAGA CTGCCCGCTT GTGGCTCGCG CGTCATAATG GGAGTTTTGA  100
<210> 5635
<211> 100
<212> DNA
<213> Escherichia coli K-12 MG1655
<220> yrao b3150

<222> 492-591
<223> yrao b3150 U00096 3293450..3294040
<313> 5635: 560-579
<400> 5635
TTCGCATCGT AGTGCTCGCA TTCAGGAAAT GCATATGCTG ACGGTAAATT  50
GCCTGTGCGA TCTGATCGAT AACACGCTTT TCCCTCACCA GGATGATTAA  100
<210> 5636
<211> 100
<212> DNA
<213> Escherichia coli K-12 MG1655
<220> yrao b3150
<222> 472-571
<223> yrao b3150 U00096 3293450..3294040
<313> 5636: 505-524
<400> 5636
GATGTGGAGA TCCGCATTCC TTCGCATCGT AGTGCTCGCA TTCAGGAAAT  50
GCATATGCTG ACGGTAAATT GCCTGTGCGA TCTGATCGAT AACACGCTTT  100
<210> 5637
<211> 100
<212> DNA
<213> Escherichia coli K-12 MG1655
<220> yrao b3150
<222> 371-470
<223> yrao b3150 U00096 3293450..3294040
<313> 5637: 413-432
<400> 5637
ATATTGTTAA AGCAGTTGAA GCCGCCGTTA CGCGTGATAT GACCATTGTG  50
GCATTGACCG GCTATGACGG CGGCGAACTT GCAGGTTTGT TAGGGCCACA  100
<210> 5638
<211> 100
<212> DNA
<213> Escherichia coli K-12 MG1655
<220> yrap b3151
<222> 477-576
<223> yrap b3151 U00096 3294050..3294625
<313> 5638: 538-557
<400> 5638
GGGGCTGGTG ACTGAACGTG AAGCGAAAGC GGCGGCAGAT ATTGCCAGCC  50
GGGTGAGCGG CGTGAAGCGG GTAACTACGG CGTTTACGTT TATTAAATAG  100
<210> 5639
<211> 100
<212> DNA
<213> Escherichia coli K-12 MG1655
<220> yrap b3151
<222> 446-545
<223> yrap b3151 U00096 3294050..3294625
<313> 5639: 497-516
<400> 5639
TGACCACCGA AAACGGTGAA GTGTTCCTGA TGGGGCTGGT GACTGAACGT  50
GAAGCGAAAG CGGCGGCAGA TATTGCCAGC CGGGTGAGCG GCGTGAAGCG  100
<210> 5640
<211> 100
<212> DNA
<213> Escherichia coli K-12 MG1655
<220> yrap b3151
<222> 393-492
<223> yrap b3151 U00096 3294050..3294625
<313> 5640: 440-459
<400> 5640
AGTGCGTTCG CAGCTCTTAA CCAGCGACCT GGTGAAATCG TCCAACGTGA  50
AAGTGACCAC CGAAAACGGT GAAGTGTTCC TGATGGGGCT GGTGACTGAA  100
<210> 5641
<211> 100
<212> DNA
<213> Escherichia coli K-12 MG1655
<220> yraq b3152
<222> 942-1041
<223> yraq b3152 U00096 complement(3294739..3295779)
<313> 5641: 994-1013
<400> 5641
TATGCTGCGC AAAGCGTTCC CGGCGAAAGC CTTATGGCTG ACAGGGGCGA  50

TGGTGGCAGT GTCTGGTGTG ATTGTCGGCG GGCTGGCGCT GTTGTTCTGA 100
<210> 5642
<211> 100
<212> DNA
<213> Escherichia coli K-12 MG1655
<220> yraq b3152
<222> 775-874
<223> yraq b3152 U00096 complement(3294739..3295779)
<313> 5642: 828-847
<400> 5642
AACAGCCTGA TGTGGGTGGT GGCGATGGCG GTAGCAGGAT GCTTGTTTGT 50
CATTCCCACG GCAGCAGAAA TTCCGATTGT ACAAACGATG ATGCTGGCAG 100
<210> 5643
<211> 100
<212> DNA
<213> Escherichia coli K-12 MG1655
<220> yraq b3152
<222> 760-859
<223> yraq b3152 U00096 complement(3294739..3295779)
<313> 5643: 795-814
<400> 5643
GATGGTGCTG TCGATAACAG CCTGATGTGG GTGGTGGCGA TGGCGGTAGC 50
AGGATGCTTG TTTGTCATTC CCACGGCAGC AGAAATTCCG ATTGTACAAA 100
<210> 5644
<211> 100
<212> DNA
<213> Escherichia coli K-12 MG1655
<220> yrar b3153
<222> 582-681
<223> yrar b3153 U00096 complement(3295852..3296532)
<313> 5644: 631-650
<400> 5644
GAGAGACGTG GCGCGGGTCA TGCTGGCAGA GTCGATGCGG CCTGAGCATG 50
AAGGCGTGAC GATTTTAAGC TCTTCAGAAT TACGCAAAAG AGCTGAATAA 100
<210> 5645
<211> 100
<212> DNA
<213> Escherichia coli K-12 MG1655
<220> yrar b3153
<222> 505-604
<223> yrar b3153 U00096 complement(3295852..3296532)
<313> 5645: 540-559
<400> 5645
AGCAAACAGC GGATGAACGA AACGCTTTTT GCGCCGCTGT TCCGTTTGTT 50
ACCAGGTAAC TGGAAATCCA TTGATGCGAG AGACGTGGCG CGGGTCATGC 100
<210> 5646
<211> 100
<212> DNA
<213> Escherichia coli K-12 MG1655
<220> yrar b3153
<222> 398-497
<223> yrar b3153 U00096 complement(3295852..3296532)
<313> 5646: 450-469
<400> 5646
CGCCGTTTTT CTATAACCGC GTCAAAGGGG AGATGGAAGA AGCATTAATC 50
GCCCAGAACT GGCCGAAATT GACCATTGCT CGCCCGTCGA TGTTACTGGG 100
<210> 5647
<211> 100
<212> DNA
<213> Escherichia coli K-12 MG1655
<220> idnd b4268
<222> 933-1032
<223> idnd b4268 U00096 complement(4490943..4491974)
<313> 5647: 1011-1030
<400> 5647
GCCTTTACTG AGTGCTGAAT ATCCCTTCAC TGACCTGGAA GAGGCGCTAC 50
GTTTCGCCGG TGATAAAACC CAGGCAGCAA AAGTCCAGCT TGTTTTCTAA 100
<210> 5648
<211> 100
<212> DNA
<213> Escherichia coli K-12 MG1655

<220> idnd b4268
<222> 903-1002
<223> idnd b4268 U00096 complement(4490943..4491974)
<313> 5648: 949-968
<400> 5648
ATGGCTGGCG AATGGCGTTA TCAATCCACT GCCTTTACTG AGTGCTGAAT 50
ATCCCTTCAC TGACCTGGAA GAGGCGCTAC GTTTCGCCGG TGATAAAACC 100
<210> 5649
<211> 100
<212> DNA
<213> Escherichia coli K-12 MG1655
<220> idnd b4268
<222> 834-933
<223> idnd b4268 U00096 complement(4490943..4491974)
<313> 5649: 871-890
<400> 5649
GTTGATTGGT AAGGAGATTT CACTCAGAGG CTCTTTCCGT TTTACCAGCG 50
AATTTAATAC CGCAGTGTCA TGGCTGGCGA ATGGCGTTAT CAATCCACTG 100
<210> 5650
<211> 100
<212> DNA
<213> Escherichia coli K-12 MG1655
<220> arp b4018
<222> 2084-2183
<223> arp b4018 U00096 complement(4217880..4220066)
<313> 5650: 2105-2124
<400> 5650
AGATGGCTGA AAGCTTTGGC TTTACGGATA ACCCTCGATA CATTGCGGAG 50
AAAAATTATA TGGAAGCTTT ACTCAAAAAA GCATCTCCCC ATACCGTACG 100
<210> 5651
<211> 100
<212> DNA
<213> Escherichia coli K-12 MG1655
<220> arp b4018
<222> 1969-2068
<223> arp b4018 U00096 complement(4217880..4220066)
<313> 5651: 2010-2029
<400> 5651
GAAGATATTA TGGATTTTTG GCAACACAAA GCCCCACAAA AATACTCTGC 50
CTTTGAGTTG GCTTTTGAAT TTGGTCACCG GGTTATTGCT GAATTAATCC 100
<210> 5652
<211> 100
<212> DNA
<213> Escherichia coli K-12 MG1655
<220> arp b4018
<222> 1916-2015
<223> arp b4018 U00096 complement(4217880..4220066)
<313> 5652: 1952-1971
<400> 5652
AGACGGTTTA TCTTGCTTTA TCTGACCATG CACGCCTGTT TGGATTTACC 50
GCTGAAGATA TTATGGATTT TTGGCAACAC AAAGCCCCAC AAAAATACTC 100
<210> 5653
<211> 100
<212> DNA
<213> Escherichia coli K-12 MG1655
<220> yifm_1 b4405
<222> 23-122
<223> yifm_1 b4405 U00096 3975137..3975361
<313> 5653: 81-100
<400> 5653
TGGGATCGGA TATCCCTCAC CATAACCGAA CCGTTTTGCG GTTTTTCAAT 50
GACGCGCTGG CCGCGACGAG CGAGCACGCG CGCGAGTTTA TGGTTGTTGG 100
<210> 5654
<211> 100
<212> DNA
<213> Escherichia coli K-12 MG1655
<220> pta b2298
<222> 2046-2145
<223> pta b2298 U00096 2412767..2414911
<313> 5654: 2123-2142
<400> 5654

TATGCGCAAG CCGGTTAACG ACCTGTCCCG TGGCGCACTG GTTGACGATA 50
TCGTCTACAC CATCGCGCTG ACTGCGATTC AGTCTGCACA GCAGCAGTAA 100
<210> 5655
<211> 100
<212> DNA
<213> Escherichia coli K-12 MG1655
<220> pta b2298
<222> 2034-2133
<223> pta b2298 U00096 2412767..2414911
<313> 5655: 2070-2089
<400> 5655
GATGCTGCAG GGTATGCGCA AGCCGGTTAA CGACCTGTCC CGTGGCGCAC 50
TGGTTGACGA TATCGTCTAC ACCATCGCGC TGACTGCGAT TCAGTCTGCA 100
<210> 5656
<211> 100
<212> DNA
<213> Escherichia coli K-12 MG1655
<220> pta b2298
<222> 1948-2047
<223> pta b2298 U00096 2412767..2414911
<313> 5656: 2008-2027
<400> 5656
ACCGTGTTCA TCTTCCCGGA TCTGAACACC GGTAACACCA CCTACAAAGC 50
GGTACAGCGT TCTGCCGACC TGATCTCCAT CGGGCCGATG CTGCAGGGTA 100
<210> 5657
<211> 100
<212> DNA
<213> Escherichia coli K-12 MG1655
<220> yifm_2 b3793
<222> 516-615
<223> yifm_2 b3793 U00096 3975603..3976217
<313> 5657: 553-572
<400> 5657
GTTGGCGTCG GTGGATAAAA ACACCATTGC CTTCTTTAGC CCTAACTATC 50
TACAAGGCTG GCAGCGGGCG TTGGCGATTG CCGCCAGGGA GGTCGCATGA 100
<210> 5658
<211> 100
<212> DNA
<213> Escherichia coli K-12 MG1655
<220> yifm_2 b3793
<222> 443-542
<223> yifm_2 b3793 U00096 3975603..3976217
<313> 5658: 465-484
<400> 5658
CGGAACAACA TTTGCCGGTG CTGTTTACTA CCGACGATCT CAACGAGGAT 50
ATTGTGCGTG AAGCGCAGCG CCAGTTGGCG TCGGTGGATA AAAACACCAT 100
<210> 5659
<211> 100
<212> DNA
<213> Escherichia coli K-12 MG1655
<220> yifm_2 b3793
<222> 375-474
<223> yifm_2 b3793 U00096 3975603..3976217
<313> 5659: 419-438
<400> 5659
GTGCTTACTG ATTCAGGCGG GCATTCCTTG TGTGCTTAAC CGGGAAAATC 50
CGTTCTGGCA GGATATGACG GAACAACATT TGCCGGTGCT GTTTACTACC 100
<210> 5660
<211> 100
<212> DNA
<213> Escherichia coli K-12 MG1655
<220> idnk b4269
<222> 465-564
<223> idnk b4269 U00096 4492191..4492754
<313> 5660: 529-548
<400> 5660
TGCCAATGTC ACCGAACAAT GTCGGCAGGC TGTGCTGGCG ATACGACAAA 50
ACAGAATATG TGCGAAAGAG GGCAGCGCCT CAGATCAGCG CTGCGAATGA 100
<210> 5661
<211> 100
<212> DNA

<213> Escherichia coli K-12 MG1655
<220> idnk b4269
<222> 358-457
<223> idnk b4269 U00096 4492191..4492754
<313> 5661: 409-428
<400> 5661
GCTGGGCATT TTATGCCGGT AGCGTTACTA AAAAGTCAGT TTGAGGCACT  50
GGAGCGTCCA CAAGCAGATG AACAGGATAT TGTGCGCATT GATATCAACC  100
<210> 5662
<211> 100
<212> DNA
<213> Escherichia coli K-12 MG1655
<220> idnk b4269
<222> 256-355
<223> idnk b4269 U00096 4492191..4492754
<313> 5662: 288-307
<400> 5662
AAAAAACAGT ATCGTGATAT TTTACGCAAG GGTAGCCCCC ATGTTCATTT  50
CCTCTGGTTA GATGGCGACT ATGAAACTAT TCTCGCGCGA ATGCAGCGTC  100
<210> 5663
<211> 100
<212> DNA
<213> Escherichia coli K-12 MG1655
<220> idno b4267
<222> 666-765
<223> idno b4267 U00096 complement(4490155..4490919)
<313> 5663: 701-720
<400> 5663
GGAACTGATT GGTGCTGCGG TGTTCCTTTC TTCAAAAGCC TCTGATTTCG  50
TAAACGGCCA CCTGTTGTTT GTTGATGGCG GCATGTTAGT GGCTGTTTAA  100
<210> 5664
<211> 100
<212> DNA
<213> Escherichia coli K-12 MG1655
<220> idno b4267
<222> 620-719
<223> idno b4267 U00096 complement(4490155..4490919)
<313> 5664: 654-673
<400> 5664
CCTGGTTGTG CAAACGGACC CCCGCCGCAC GCTGGGGAGA TCCGCAGGAA  50
CTGATTGGTG CTGCGGTGTT CCTTTCTTCA AAAGCCTCTG ATTTCGTAAA  100
<210> 5665
<211> 100
<212> DNA
<213> Escherichia coli K-12 MG1655
<220> idno b4267
<222> 547-646
<223> idno b4267 U00096 complement(4490155..4490919)
<313> 5665: 606-625
<400> 5665
GTCAACGGTA TTGCGCCGGG CTATTTCAAA ACAGAAATGA CTAAAGCACT  50
GGTTGAGGAC GAAGCCTTCA CCGCCTGGTT GTGCAAACGG ACCCCCGCCG  100
<210> 5666
<211> 100
<212> DNA
<213> Escherichia coli K-12 MG1655
<220> b3042 b3043
<222> 252-351
<223> b3042 b3043 U00096 3182796..3183146
<313> 5666: 325-344
<400> 5666
AAAACTGGCG CTGCTTGAAC AAAGAATCAG CGAACTGGAA AATCGCAGTA  50
CTGAGATCAA AAAGCAGCCA GATCCAGAGA CTCTCCCCCC AACGCTGTAA  100
<210> 5667
<211> 100
<212> DNA
<213> Escherichia coli K-12 MG1655
<220> b3042 b3043
<222> 231-330
<223> b3042 b3043 U00096 3182796..3183146
<313> 5667: 260-279

<400> 5667
AGTCCTGTTA CGGACTCGTG AAAAACTGGC GCTGCTTGAA CAAAGAATCA  50
GCGAACTGGA AAATCGCAGT ACTGAGATCA AAAAGCAGCC AGATCCAGAG  100
<210> 5668
<211> 100
<212> DNA
<213> Escherichia coli K-12 MG1655
<220> b3042 b3043
<222> 200-299
<223> b3042 b3043 U00096 3182796..3183146
<313> 5668: 237-256
<400> 5668
TAAGCCGTGA AGAGTTCGAC GTCCAAACGC AAGTCCTGTT ACGGACTCGT  50
GAAAAACTGG CGCTGCTTGA ACAAAGAATC AGCGAACTGG AAAATCGCAG  100
<210> 5669
<211> 100
<212> DNA
<213> Escherichia coli K-12 MG1655
<220> pth b1205
<222> 486-585
<223> pth b1205 U00096 complement(1257152..1257736)
<313> 5669: 537-556
<400> 5669
TGATGAAGCC ATTGACGAAG CGGCGCGTTG TACTGAAATG TGGTTTACAG  50
ATGGCTTGAC CAAAGCAACG AACCGATTGC ACGCCTTTAA AGCGCAATAA  100
<210> 5670
<211> 100
<212> DNA
<213> Escherichia coli K-12 MG1655
<220> pth b1205
<222> 408-507
<223> pth b1205 U00096 complement(1257152..1257736)
<313> 5670: 447-466
<400> 5670
AATCGGTCAT CCGGGCGATA AAAATAAAGT TGTCGGTTTT GTGTTAGGCA  50
AACCGCCTGT TAGTGAACAG AAGTTAATTG ATGAAGCCAT TGACGAAGCG  100
<210> 5671
<211> 100
<212> DNA
<213> Escherichia coli K-12 MG1655
<220> pth b1205
<222> 381-480
<223> pth b1205 U00096 complement(1257152..1257736)
<313> 5671: 415-434
<400> 5671
CCCTAACTTT CACCGTTTAC GCATCGGAAT CGGTCATCCG GGCGATAAAA  50
ATAAAGTTGT CGGTTTTGTG TTAGGCAAAC CGCCTGTTAG TGAACAGAAG  100
<210> 5672
<211> 100
<212> DNA
<213> Escherichia coli K-12 MG1655
<220> idnr b4265
<222> 900-999
<223> idnr b4265 U00096 complement(4487709..4488707)
<313> 5672: 975-994
<400> 5672
GATGGCCGCC CAAATGTTGC TGAGCAAAAT TAAAAATAAC GATCACAACC  50
ACAACACTGT TGACCTCGGG TATCAGATTT ATCACGGCAA CACGCTTTAG  100
<210> 5673
<211> 100
<212> DNA
<213> Escherichia coli K-12 MG1655
<220> idnr b4265
<222> 900-999
<223> idnr b4265 U00096 complement(4487709..4488707)
<313> 5673: 964-983
<400> 5673
GATGGCCGCC CAAATGTTGC TGAGCAAAAT TAAAAATAAC GATCACAACC  50
ACAACACTGT TGACCTCGGG TATCAGATTT ATCACGGCAA CACGCTTTAG  100
<210> 5674
<211> 100

<212> DNA
<213> Escherichia coli K-12 MG1655
<220> idnr b4265
<222> 900-999
<223> idnr b4265 U00096 complement(4487709..4488707)
<313> 5674: 948-967
<400> 5674
GATGGCCGCC CAAATGTTGC TGAGCAAAAT TAAAAATAAC GATCACAACC 50
ACAACACTGT TGACCTCGGG TATCAGATTT ATCACGGCAA CACGCTTTAG 100
<210> 5675
<211> 100
<212> DNA
<213> Escherichia coli K-12 MG1655
<220> ispa b0422
<222> 785-884
<223> ispa b0422 U00096 complement(439426..440325)
<313> 5675: 842-861
<400> 5675
AAGCCCGGGA TCTGATCGAC GATGCCCGTC AGTCGCTGAA ACAACTGGCT 50
GAACAGTCAC TCGATACCTC GGCACTGGAA GCGCTAGCGG ACTACATCAT 100
<210> 5676
<211> 100
<212> DNA
<213> Escherichia coli K-12 MG1655
<220> ispa b0422
<222> 712-811
<223> ispa b0422 U00096 complement(439426..440325)
<313> 5676: 755-774
<400> 5676
CGCCAGGGTG CCGACCAGCA ACTTGGTAAA AGTACCTACC CTGCACTTCT 50
GGGTCTTGAG CAAGCCCGGA AGAAAGCCCG GGATCTGATC GACGATGCCC 100
<210> 5677
<211> 100
<212> DNA
<213> Escherichia coli K-12 MG1655
<220> ispa b0422
<222> 587-686
<223> ispa b0422 U00096 complement(439426..440325)
<313> 5677: 650-669
<400> 5677
GCGCCGGAGA TAAAGGACGT CGTGCTCTGC CGGTACTCGA CAAGTATGCA 50
GAGAGCATCG GCCTTGCCTT CCAGGTTCAG GATGACATCC TGGATGTGGT 100
<210> 5678
<211> 100
<212> DNA
<213> Escherichia coli K-12 MG1655
<220> yrba b3191
<222> 171-270
<223> yrba b3191 U00096 complement(3334190..3334459)
<313> 5678: 223-242
<400> 5678
GCTGATGGAA TATATTGCGG ATAACCGCAT TCATGCTGTG TCGATCAAAG 50
CGTATACCCC TGCGGAGTGG GCGCGCGATC GCAAACTGAA CGGCTTTTGA 100
<210> 5679
<211> 100
<212> DNA
<213> Escherichia coli K-12 MG1655
<220> yrba b3191
<222> 102-201
<223> yrba b3191 U00096 complement(3334190..3334459)
<313> 5679: 143-162
<400> 5679
GGTTATTGCC GTGGGTGAGT TGTTTGACGG CATGAGTCGG GTTAAAAAAC 50
AGCAGACGGT CTATGGTCCG CTGATGGAAT ATATTGCGGA TAACCGCATT 100
<210> 5680
<211> 100
<212> DNA
<213> Escherichia coli K-12 MG1655
<220> yrba b3191
<222> 65-164
<223> yrba b3191 U00096 complement(3334190..3334459)

<313> 5680: 97-116
<400> 5680
AGGAAGTCCA CGTTTCCGGC GATGGCAGCC ACTTTCAGGT TATTGCCGTG 50
GGTGAGTTGT TTGACGGCAT GAGTCGGGTT AAAAAACAGC AGACGGTCTA 100
<210> 5681
<211> 100
<212> DNA
<213> Escherichia coli K-12 MG1655
<220> trs5_10 b3219
<222> 809-908
<223> trs5_10 b3219 U00096 complement(3363337..3364353)
<313> 5681: 862-881
<400> 5681
ACAAAACGGC CATCAACATC GAATACATGA AAGCCAGCAT CCGGGCCAGG 50
GTGGAGCACC CATTTCGCAT CATCAAGCGA CAGTTCGGCT TCGTGAAAGC 100
<210> 5682
<211> 100
<212> DNA
<213> Escherichia coli K-12 MG1655
<220> trs5_10 b3219
<222> 833-932
<223> trs5_10 b3219 U00096 complement(3363337..3364353)
<313> 5682: 857-876
<400> 5682
ACATGAAAGC CAGCATCCGG GCCAGGGTGG AGCACCCATT TCGCATCATC 50
AAGCGACAGT TCGGCTTCGT GAAAGCCAGA TACAAGGGGT TGCTGAAAAA 100
<210> 5683
<211> 100
<212> DNA
<213> Escherichia coli K-12 MG1655
<220> trs5_10 b3219
<222> 814-913
<223> trs5_10 b3219 U00096 complement(3363337..3364353)
<313> 5683: 850-869
<400> 5683
ACGGCCATCA ACATCGAATA CATGAAAGCC AGCATCCGGG CCAGGGTGGA 50
GCACCCATTT CGCATCATCA AGCGACAGTT CGGCTTCGTG AAAGCCAGAT 100
<210> 5684
<211> 100
<212> DNA
<213> Escherichia coli K-12 MG1655
<220> ispb b3188
<222> 873-972
<223> ispb b3188 U00096 3331351..3332322
<313> 5684: 930-949
<400> 5684
AGCAGACAAA GCCATCGCAG CGTTACAGGT GCTCCCGGAC ACCCCTTGGC 50
GAGAAGCACT CATCGGCCTC GCGCACATCG CTGTTCAACG CGATCGTTAA 100
<210> 5685
<211> 100
<212> DNA
<213> Escherichia coli K-12 MG1655
<220> ispb b3188
<222> 851-950
<223> ispb b3188 U00096 3331351..3332322
<313> 5685: 896-915
<400> 5685
CGCGTCAGCG TGCCGAGGAA GAAGCAGACA AAGCCATCGC AGCGTTACAG 50
GTGCTCCCGG ACACCCCTTG GCGAGAAGCA CTCATCGGCC TCGCGCACAT 100
<210> 5686
<211> 100
<212> DNA
<213> Escherichia coli K-12 MG1655
<220> ispb b3188
<222> 789-888
<223> ispb b3188 U00096 3331351..3332322
<313> 5686: 822-841
<400> 5686
CGGTCGCCAT CTTCTGGAAC CGGTTCTGGA AGCAATGAAC GCTTGTGGAT 50
CTCTTGAATG GACGCGTCAG CGTGCCGAGG AAGAAGCAGA CAAAGCCATC 100
<210> 5687

<211> 100
<212> DNA
<213> Escherichia coli K-12 MG1655
<220> yrbb b3192
<222> 266-365
<223> yrbb b3192 U00096 complement(3334604..3334993)
<313> 5687: 312-331
<400> 5687
TGCTGCTCCA TCTTATCGAT CTGGCGAAAA AGCAGGGCAA CAATGTGACG 50
CTTCAGGGGG TAAACGACAA AGTGTATACC CTGGCGAAAT TGTATAATTT 100
<210> 5688
<211> 100
<212> DNA
<213> Escherichia coli K-12 MG1655
<220> yrbb b3192
<222> 227-326
<223> yrbb b3192 U00096 complement(3334604..3334993)
<313> 5688: 266-285
<400> 5688
TTAGCCGTGT CTCCCGCGTG GATACGGGGG GACTGGCACT GCTGCTCCAT 50
CTTATCGATC TGGCGAAAAA GCAGGGCAAC AATGTGACGC TTCAGGGGGT 100
<210> 5689
<211> 100
<212> DNA
<213> Escherichia coli K-12 MG1655
<220> yrbb b3192
<222> 162-261
<223> yrbb b3192 U00096 complement(3334604..3334993)
<313> 5689: 207-226
<400> 5689
GGACGTTTTG CTACCGCTTT GGGAAATGCG TGAGGAAGCG GTGAAGGGGA 50
TTACCTGCAT CGATCTTAGC CGTGTCTCCC GCGTGGATAC GGGGGGACTG 100
<210> 5690
<211> 100
<212> DNA
<213> Escherichia coli K-12 MG1655
<220> trs5_11 b3506
<222> 822-921
<223> trs5_11 b3506 U00096 complement(3649812..3650828)
<313> 5690: 864-883
<400> 5690
CAACATCGAA TACATGAAAG CCAGCATCCG GGCCAGGGTG GAGCACCCAT 50
TTCGCATCAT CAAGCGACAG TTCGGCTTCG TGAAAGCCAG ATACAAGGGG 100
<210> 5691
<211> 100
<212> DNA
<213> Escherichia coli K-12 MG1655
<220> trs5_11 b3506
<222> 809-908
<223> trs5_11 b3506 U00096 complement(3649812..3650828)
<313> 5691: 864-883
<400> 5691
ACAAAACGGC CATCAACATC GAATACATGA AAGCCAGCAT CCGGGCCAGG 50
GTGGAGCACC CATTTCGCAT CATCAAGCGA CAGTTCGGCT TCGTGAAAGC 100
<210> 5692
<211> 100
<212> DNA
<213> Escherichia coli K-12 MG1655
<220> trs5_11 b3506
<222> 790-889
<223> trs5_11 b3506 U00096 complement(3649812..3650828)
<313> 5692: 845-864
<400> 5692
AAACAGCATC CACGCAAGAA CAAAACGGCC ATCAACATCG AATACATGAA 50
AGCCAGCATC CGGGCCAGGG TGGAGCACCC ATTTCGCATC ATCAAGCGAC 100
<210> 5693
<211> 100
<212> DNA
<213> Escherichia coli K-12 MG1655
<220> icc b3033
<222> 729-828

<223> icc b3033 U00096 complement(3174022..3174849)
<313> 5693: 789-808
<400> 5693
GCGTACTCTC GAGTTACATG CTGATGGCAC GCTGACCACC GAGGTGCATC 50
GCCTGGCGGA CACACGTTTC CAACCTGATA CCGCTTCAGA AGGCTACTGA 100
<210> 5694
<211> 100
<212> DNA
<213> Escherichia coli K-12 MG1655
<220> icc b3033
<222> 656-755
<223> icc b3033 U00096 complement(3174022..3174849)
<313> 5694: 706-725
<400> 5694
CAACGCCGTC GACCTGTGTG CAGTTTAAGC CGCACTGTTC CAACTTTACG 50
CTGGATACCA TCGCGCCCGG CTGGCGTACT CTCGAGTTAC ATGCTGATGG 100
<210> 5695
<211> 100
<212> DNA
<213> Escherichia coli K-12 MG1655
<220> icc b3033
<222> 577-676
<223> icc b3033 U00096 complement(3174022..3174849)
<313> 5695: 633-652
<400> 5695
TTTCCGCACG TCAAATACTT GCTGTGCGGT CATATTCATC AGGAGCTGGA 50
TCTCGACTGG AATGGTCGCC GCCTGCTGGC AACGCCGTCG ACCTGTGTGC 100
<210> 5696
<211> 100
<212> DNA
<213> Escherichia coli K-12 MG1655
<220> idnt b4266
<222> 1221-1320
<223> idnt b4266 U00096 complement(4488774..4490093)
<313> 5696: 1292-1311
<400> 5696
GACGGTTGGT GAAACGTTGC GTACCTGGAC GGTGATGGAA ACCCTTATTT 50
CTATTATGGG TTTGCTGGGC GTGTTAGCCA TTAACGCCGT ATTGCACTGA 100
<210> 5697
<211> 100
<212> DNA
<213> Escherichia coli K-12 MG1655
<220> idnt b4266
<222> 1189-1288
<223> idnt b4266 U00096 complement(4488774..4490093)
<313> 5697: 1251-1270
<400> 5697
GGCTTCTGGC TATTTAAAGG GTATTTTAAT CTGACGGTTG GTGAAACGTT 50
GCGTACCTGG ACGGTGATGG AAACCCTTAT TTCTATTATG GGTTTGCTGG 100
<210> 5698
<211> 100
<212> DNA
<213> Escherichia coli K-12 MG1655
<220> idnt b4266
<222> 1155-1254
<223> idnt b4266 U00096 complement(4488774..4490093)
<313> 5698: 1177-1196
<400> 5698
GGGCAGCGTG ATCGCGTCAC ACGTAAACGA CCCTGGCTTC TGGCTATTTA 50
AAGGGTATTT TAATCTGACG GTTGGTGAAA CGTTGCGTAC CTGGACGGTG 100
<210> 5699
<211> 100
<212> DNA
<213> Escherichia coli K-12 MG1655
<220> yrbc b3193
<222> 498-597
<223> yrbc b3193 U00096 complement(3334897..3335532)
<313> 5699: 555-574
<400> 5699
GATTGCTGAA GGCGTCAGTA TGATCACCAC CAAACAAAAC GAGTGGGGAA 50
CGCTGCTGCG TACCAAAGGT ATCGACGGCC TGACTGCGCA ACTGAAATCG 100

<210> 5700
<211> 100
<212> DNA
<213> Escherichia coli K-12 MG1655
<220> yrbc b3193
<222> 476-575
<223> yrbc b3193 U00096 complement(3334897..3335532)
<313> 5700: 509-528
<400> 5700
GCAATTGGCA GGCTTACGAC ATGATTGCTG AAGGCGTCAG TATGATCACC 50
ACCAAACAAA ACGAGTGGGG AACGCTGCTG CGTACCAAAG GTATCGACGG 100
<210> 5701
<211> 100
<212> DNA
<213> Escherichia coli K-12 MG1655
<220> yrbc b3193
<222> 427-526
<223> yrbc b3193 U00096 complement(3334897..3335532)
<313> 5701: 475-494
<400> 5701
CGTCCGCCGG TGCGTCTGGA CTTCCAGTGG CGTAAAAACT CCCAGACGGG 50
CAATTGGCAG GCTTACGACA TGATTGCTGA AGGCGTCAGT ATGATCACCA 100
<210> 5702
<211> 100
<212> DNA
<213> Escherichia coli K-12 MG1655
<220> yrbd b3194
<222> 453-552
<223> yrbd b3194 U00096 complement(3335551..3336102)
<313> 5702: 514-533
<400> 5702
TTACGGTAGT AAAGGCGATG ACAATAAGAA TAGTGGCGAT GCGCCAGCTG 50
CTGCGCCAGG TAATAATGAA ACCACTGAAC CTGTGGGTAC AACGAAATAA 100
<210> 5703
<211> 100
<212> DNA
<213> Escherichia coli K-12 MG1655
<220> yrbd b3194
<222> 418-517
<223> yrbd b3194 U00096 complement(3335551..3336102)
<313> 5703: 463-482
<400> 5703
GCGATGGTGC TGGAAGATCT CATTGGTCAG TTCCTTTACG GTAGTAAAGG 50
CGATGACAAT AAGAATAGTG GCGATGCGCC AGCTGCTGCG CCAGGTAATA 100
<210> 5704
<211> 100
<212> DNA
<213> Escherichia coli K-12 MG1655
<220> yrbd b3194
<222> 378-477
<223> yrbd b3194 U00096 complement(3335551..3336102)
<313> 5704: 415-434
<400> 5704
TATCCTGAAG GATGGCGATA CAATTCAGGA CACTAAGTCT GCGATGGTGC 50
TGGAAGATCT CATTGGTCAG TTCCTTTACG GTAGTAAAGG CGATGACAAT 100
<210> 5705
<211> 100
<212> DNA
<213> Escherichia coli K-12 MG1655
<220> yrbe b3195
<222> 684-783
<223> yrbe b3195 U00096 complement(3336107..3336889)
<313> 5705: 722-741
<400> 5705
TGCCGGGATT AGCCGGGCAA CCACTCGCAC CGTTGTCCAC TCGTCTCTGG 50
CTGTTCTGGG GCTGGATTTT GTGCTGACCG CATTGATGTT TGGGAATTGA 100
<210> 5706
<211> 100
<212> DNA
<213> Escherichia coli K-12 MG1655
<220> yrbe b3195

<222> 599-698
<223> yrbe b3195 U00096 complement(3336107..3336889)
<313> 5706: 650-669
<400> 5706
TCAACTGTCT GATTAAGAGC GTGGTGTTCG CCATCACGGT GACGTGGATT  50
TCGTTGTTTA ACGGCTACGA CGCCATCCCG ACGTCTGCCG GGATTAGCCG  100
<210> 5707
<211> 100
<212> DNA
<213> Escherichia coli K-12 MG1655
<220> yrbe b3195
<222> 556-655
<223> yrbe b3195 U00096 complement(3336107..3336889)
<313> 5707: 606-625
<400> 5707
TGGTCGGCAA TGCAAAATGC CGTCGACTGG CGTATGGATC TGGTCAACTG  50
TCTGATTAAG AGCGTGGTGT TCGCCATCAC GGTGACGTGG ATTTCGTTGT  100
<210> 5708
<211> 100
<212> DNA
<213> Escherichia coli K-12 MG1655
<220> yrbf b3196
<222> 640-739
<223> yrbf b3196 U00096 complement(3336897..3337706)
<313> 5708: 654-673
<400> 5708
CACGCCTGGA TCCTGGCGGA CAAAAAAATT GTCGCTCATG GCAGTGCCCA  50
GGCGTTGCAG GCGAATCCTG ATCCGCGCGT ACGTCAGTTT CTGGACGGGA  100
<210> 5709
<211> 100
<212> DNA
<213> Escherichia coli K-12 MG1655
<220> yrbf b3196
<222> 600-699
<223> yrbf b3196 U00096 complement(3336897..3337706)
<313> 5709: 614-633
<400> 5709
GGTTTCTCAC GATGTGCCGG AAGTGTTAAG TATTGCGGAT CACGCCTGGA  50
TCCTGGCGGA CAAAAAAATT GTCGCTCATG GCAGTGCCCA GGCGTTGCAG  100
<210> 5710
<211> 100
<212> DNA
<213> Escherichia coli K-12 MG1655
<220> yrbf b3196
<222> 495-594
<223> yrbf b3196 U00096 complement(3336897..3337706)
<313> 5710: 554-573
<400> 5710
CATCATGTTT GATGAACCTT TTGTTGGGCA AGATCCCATC ACCATGGGCG  50
TACTGGTGAA GCTGATTTCT GAGCTGAACA GCGCGCTGGG CGTGACTTGT  100
<210> 5711
<211> 100
<212> DNA
<213> Escherichia coli K-12 MG1655
<220> yrbg b3197
<222> 864-963
<223> yrbg b3197 U00096 3337916..3338893
<313> 5711: 918-937
<400> 5711
GCTGTGCTGG CGGCGCTCCC CGCAACCGGG CCGTGGTGTA GGGGTATTAT  50
TAACTGGCGG ATTTATCGTA TGGCTGGCGA TGTTGTACTG GTTATCGCCA  100
<210> 5712
<211> 100
<212> DNA
<213> Escherichia coli K-12 MG1655
<220> yrbg b3197
<222> 750-849
<223> yrbg b3197 U00096 3337916..3338893
<313> 5712: 774-793
<400> 5712
TAATATTGTC ATCGTGTTGG GTTTACCCGC GCTGATAACG CCAGGAGAGA  50

TTGATCCACT GGCGTACAGT CGTGACTACA GCGTGATGTT GCTGGTGAGC 100
<210> 5713
<211> 100
<212> DNA
<213> Escherichia coli K-12 MG1655
<220> yrbg b3197
<222> 673-772
<223> yrbg b3197 U00096 3337916..3338893
<313> 5713: 726-745
<400> 5713
CTGGCAACCG CAATAGCGGG GGTTCGCAAA GGTGAAAACG ACATTGCTGT 50
CGGAAATATC ATTGGCGCAA ACATTTTTAA TATTGTCATC GTGTTGGGTT 100
<210> 5714
<211> 100
<212> DNA
<213> Escherichia coli K-12 MG1655
<220> yrbh b3198
<222> 863-962
<223> yrbh b3198 U00096 3338907..3339893
<313> 5714: 896-915
<400> 5714
TTCTGGCCGT TGAGGCACTG AACTTAATGC AGTCCCGCCA TATCACCTCC 50
GTGATGGTTG CCGATGGCGA CCATTTACTC GGTGTGTTAC ATATGCATGA 100
<210> 5715
<211> 100
<212> DNA
<213> Escherichia coli K-12 MG1655
<220> yrbh b3198
<222> 835-934
<223> yrbh b3198 U00096 3338907..3339893
<313> 5715: 857-876
<400> 5715
CCGGGGGGAA TACGTGTGCG CCCTGGCATT CTGGCCGTTG AGGCACTGAA 50
CTTAATGCAG TCCCGCCATA TCACCTCCGT GATGGTTGCC GATGGCGACC 100
<210> 5716
<211> 100
<212> DNA
<213> Escherichia coli K-12 MG1655
<220> yrbh b3198
<222> 797-896
<223> yrbh b3198 U00096 3338907..3339893
<313> 5716: 818-837
<400> 5716
TGGATGTTCG TCAGTTAAGT ATTGCCGATG TGATGACGCC GGGGGGAATA 50
CGTGTGCGCC CTGGCATTCT GGCCGTTGAG GCACTGAACT TAATGCAGTC 100
<210> 5717
<211> 100
<212> DNA
<213> Escherichia coli K-12 MG1655
<220> yrbi b3199
<222> 468-567
<223> yrbi b3199 U00096 3339914..3340480
<313> 5717: 534-553
<400> 5717
GACGCGCATT GCTGGCGGTC GTGGCGCAGT GCGCGAAGTT TGCGACTTAT 50
TACTCCTGGC GCAGGGCAAA CTGGATGAAG CCAAAGGGCA ATCGATATGA 100
<210> 5718
<211> 100
<212> DNA
<213> Escherichia coli K-12 MG1655
<220> yrbi b3199
<222> 468-567
<223> yrbi b3199 U00096 3339914..3340480
<313> 5718: 496-515
<400> 5718
GACGCGCATT GCTGGCGGTC GTGGCGCAGT GCGCGAAGTT TGCGACTTAT 50
TACTCCTGGC GCAGGGCAAA CTGGATGAAG CCAAAGGGCA ATCGATATGA 100
<210> 5719
<211> 100
<212> DNA
<213> Escherichia coli K-12 MG1655

<220> yrbi b3199
<222> 263-362
<223> yrbi b3199 U00096 3339914..3340480
<313> 5719: 299-318
<400> 5719
CCACATTGGG GATCACTCAC TTGTATCAGG GGCAGTCAAA CAAACTGATC  50
GCCTTTAGCG ATCTGCTGGA AAAACTGGCG ATTGCCCCGG AAAATGTGGC  100
<210> 5720
<211> 100
<212> DNA
<213> Escherichia coli K-12 MG1655
<220> ptr b2822
<222> 2790-2889
<223> ptr b2822 U00096 complement(2954018..2956906)
<313> 5720: 2855-2874
<400> 5720
CGGGAAAGCC GAATATGTAC ACCCTGAAGG CTGGAAAGTG TGGGAGAACG  50
TCAGCGCGTT GCAGCAAACA ATGCCCCTGA TGAGTGAAAA GAATGAGTGA  100
<210> 5721
<211> 100
<212> DNA
<213> Escherichia coli K-12 MG1655
<220> ptr b2822
<222> 2761-2860
<223> ptr b2822 U00096 complement(2954018..2956906)
<313> 5721: 2815-2834
<400> 5721
ATTCTGTCGC AGATTTCCGG CAGCCAGAAC GGGAAAGCCG AATATGTACA  50
CCCTGAAGGC TGGAAAGTGT GGGAGAACGT CAGCGCGTTG CAGCAAACAA  100
<210> 5722
<211> 100
<212> DNA
<213> Escherichia coli K-12 MG1655
<220> ptr b2822
<222> 2724-2823
<223> ptr b2822 U00096 complement(2954018..2956906)
<313> 5722: 2781-2800
<400> 5722
CTTCCATCAG GCGGTGGTCG AGCCGCAAGG CATGGCTATT CTGTCGCAGA  50
TTTCCGGCAG CCAGAACGGG AAAGCCGAAT ATGTACACCC TGAAGGCTGG  100
<210> 5723
<211> 100
<212> DNA
<213> Escherichia coli K-12 MG1655
<220> yrbk b3200
<222> 477-576
<223> yrbk b3200 U00096 3340477..3341052
<313> 5723: 510-529
<400> 5723
CGGTCTGAAA ATGCGCGGCA ACTTACGCAG CAAGAACGCC GAGCTGATTG  50
AAAAGGTTAG AACATCCTAT GAAATTCAAA ACAAACAAAC TCAGCCTTAA  100
<210> 5724
<211> 100
<212> DNA
<213> Escherichia coli K-12 MG1655
<220> yrbk b3200
<222> 432-531
<223> yrbk b3200 U00096 3340477..3341052
<313> 5724: 479-498
<400> 5724
CTCTGAAGAC CTCGTCACGT TATACGGAAC AACATTTAAC TCCAGCGGTC  50
TGAAAATGCG CGGCAACTTA CGCAGCAAGA ACGCCGAGCT GATTGAAAAG  100
<210> 5725
<211> 100
<212> DNA
<213> Escherichia coli K-12 MG1655
<220> yrbk b3200
<222> 377-476
<223> yrbk b3200 U00096 3340477..3341052
<313> 5725: 430-449
<400> 5725

TTCGCAGAAT CACGACGGAT AACGCGCAGA TCAATCTGGT GACGCAGGAT 50
GTTACCTCTG AAGACCTCGT CACGTTATAC GGAACAACAT TTAACTCCAG 100
<210> 5726
<211> 100
<212> DNA
<213> Escherichia coli K-12 MG1655
<220> stpa stpA
<222> 306-405
<223> stpa stpA U00096 complement(2796112..2796516)
<313> 5726: 369-388
<400> 5726
TAACGGTGAA ACTAAAACCT GGACCGGTCA GGGCCGTACA CCGAAGCCAA 50
TTGCTCAGGC GCTGGCAGAA GGTAAATCTC TCGACGATTT CCTGATCTAA 100
<210> 5727
<211> 100
<212> DNA
<213> Escherichia coli K-12 MG1655
<220> stpa stpA
<222> 255-354
<223> stpa stpA U00096 complement(2796112..2796516)
<313> 5727: 286-305
<400> 5727
CGCTGGTAAA AAACGCCAGC CGCGTCCGGC GAAATATAAA TTCACCGATG 50
TTAACGGTGA AACTAAAACC TGGACCGGTC AGGGCCGTAC ACCGAAGCCA 100
<210> 5728
<211> 100
<212> DNA
<213> Escherichia coli K-12 MG1655
<220> stpa stpA
<222> 201-300
<223> stpa stpA U00096 complement(2796112..2796516)
<313> 5728: 245-264
<400> 5728
TGACGGAATT AACCCGGAAG AGTTATTGGG TAATAGCTCT GCTGCTGCAC 50
CACGCGCTGG TAAAAAACGC CAGCCGCGTC CGGCGAAATA TAAATTCACC 100
<210> 5729
<211> 100
<212> DNA
<213> Escherichia coli K-12 MG1655
<220> asd b3434
<222> 1001-1100
<223> asd b3434 U00096 complement(3571408..3572511)
<313> 5729: 1018-1037
<400> 5729
AGCTGAATAT GGGACCAGAG TTCCTGTCAG CCTTTACCGT GGGCGACCAG 50
CTGCTGTGGG GGGCCGCGGA GCCGCTGCGT CGGATGCTTC GTCAACTGGC 100
<210> 5730
<211> 100
<212> DNA
<213> Escherichia coli K-12 MG1655
<220> asd b3434
<222> 794-893
<223> asd b3434 U00096 complement(3571408..3572511)
<313> 5730: 815-834
<400> 5730
GTGTGCGTGT CGGGGCATTG CGCTGCCACA GCCAGGCATT CACTATTAAA 50
TTGAAAAAAG ATGTGTCTAT TCCGACCGTG GAAGAACTGC TGGCTGCGCA 100
<210> 5731
<211> 100
<212> DNA
<213> Escherichia coli K-12 MG1655
<220> asd b3434
<222> 719-818
<223> asd b3434 U00096 complement(3571408..3572511)
<313> 5731: 754-773
<400> 5731
GCGAAGAGTG GAAAGGGCAG GCGGAAACCA ACAAGATCCT CAACACATCT 50
TCCGTAATTC CGGTAGATGG TTTATGTGTG CGTGTCGGGG CATTGCGCTG 100
<210> 5732
<211> 100
<212> DNA

<213> Escherichia coli K-12 MG1655
<220> yrbl b3208
<222> 484-583
<223> yrbl b3208 U00096 3346093..3346725
<313> 5732: 521-540
<400> 5732
CTGGCGACCT GGTCGAAGTG GTGTTGTCTG CGTAAGCAAG AGCGGTTATG  50
GAAACGATTT ATCGCCCAAC CGGCGCTGGC TATTGCCCTG CAGAAAGATC  100
<210> 5733
<211> 100
<212> DNA
<213> Escherichia coli K-12 MG1655
<220> yrbl b3208
<222> 457-556
<223> yrbl b3208 U00096 3346093..3346725
<313> 5733: 475-494
<400> 5733
AACATTGGTG AAAGCACATT GATTCCACTG GCGACCTGGT CGAAGTGGTG  50
TTGTCTGCGT AAGCAAGAGC GGTTATGGAA ACGATTTATC GCCCAACCGG  100
<210> 5734
<211> 100
<212> DNA
<213> Escherichia coli K-12 MG1655
<220> yrbl b3208
<222> 399-498
<223> yrbl b3208 U00096 3346093..3346725
<313> 5734: 442-461
<400> 5734
GCAGAATATC CTCTGTCACC GCATCAGTGA GTCAGAGGTT ATCCCGGTCG  50
TCTGCGACAA CATTGGTGAA AGCACATTGA TTCCACTGGC GACCTGGTCG  100
<210> 5735
<211> 100
<212> DNA
<213> Escherichia coli K-12 MG1655
<220> ycac b0898
<222> 394-493
<223> ycac b0898 U00096 complement(944154..944780)
<313> 5735: 451-470
<400> 5735
GACGTCTTTG TCGTGACTGA CGCGTCAGGG ACCTTTAATG AAATTACCCG  50
GCATTCGGCA TGGGATCGCT TGTCGCAAGC TGGTGCGCAA TTGATGACAT  100
<210> 5736
<211> 100
<212> DNA
<213> Escherichia coli K-12 MG1655
<220> ycac b0898
<222> 337-436
<223> ycac b0898 U00096 complement(944154..944780)
<313> 5736: 366-385
<400> 5736
GTGGTAACCG AAGTTTGCGT GGCATTCCCG GCGCTTTCAG CCATTGAAGA  50
AGGGTTTGAC GTCTTTGTCG TGACTGACGC GTCAGGGACC TTTAATGAAA  100
<210> 5737
<211> 100
<212> DNA
<213> Escherichia coli K-12 MG1655
<220> ycac b0898
<222> 196-295
<223> ycac b0898 U00096 complement(944154..944780)
<313> 5737: 222-241
<400> 5737
GGCCCATTAG TTCCAGAACT GAAAGCACAA TTCCCCGATA CACCTTACAT  50
TGCTCGCCCG GGAAATATTA ACGCCTGGGA TAACGAAGAT TTTGTAAAAG  100
<210> 5738
<211> 100
<212> DNA
<213> Escherichia coli K-12 MG1655
<220> ycad ycaD
<222> 1034-1133
<223> ycad ycaD U00096 945094..946242
<313> 5738: 1076-1095

<400> 5738
TGCAGAATTT CTCCGATAAT TTATTGTTTA TCATGATCGC CAGCGTATCG 50
TTTATCTATT TGCTGATGCT GCTGCGCAAC GCCGGTCATA CGCCGAAACC 100
<210> 5739
<211> 100
<212> DNA
<213> Escherichia coli K-12 MG1655
<220> ycad ycaD
<222> 964-1063
<223> ycad ycaD U00096 945094..946242
<313> 5739: 1018-1037
<400> 5739
ATGAACCAGG CCTTACTGTT GAGCTATACT GTGGGAAGTC TGCTTGGCCC 50
GTCATTTACC GCTATGCTAA TGCAGAATTT CTCCGATAAT TTATTGTTTA 100
<210> 5740
<211> 100
<212> DNA
<213> Escherichia coli K-12 MG1655
<220> ycad ycaD
<222> 900-999
<223> ycad ycaD U00096 945094..946242
<313> 5740: 936-955
<400> 5740
TACGCTATAT CCGGTGGCGA TGGCATGGGC TTGCGAGAAA GTTGAACATC 50
ATCAACTGGT GGCGATGAAC CAGGCCTTAC TGTTGAGCTA TACTGTGGGA 100
<210> 5741
<211> 100
<212> DNA
<213> Escherichia coli K-12 MG1655
<220> ycah b0916
<222> 888-987
<223> ycah b0916 U00096 967589..968575
<313> 5741: 959-978
<400> 5741
AGAAGAAAAT TGGTGGTATT TGCCTGTAGA CGCACAGCTT TCAGGTGATG 50
AACCAGCGAA ACTGCTTACG CAACTAACCT TGCTGGCTTC TGGCAACTAG 100
<210> 5742
<211> 100
<212> DNA
<213> Escherichia coli K-12 MG1655
<220> ycah b0916
<222> 853-952
<223> ycah b0916 U00096 967589..968575
<313> 5742: 893-912
<400> 5742
ACTGAAAAAG ATGCGGTGAA ATGCCGGGCC TTTGCAGAAG AAAATTGGTG 50
GTATTTGCCT GTAGACGCAC AGCTTTCAGG TGATGAACCA GCGAAACTGC 100
<210> 5743
<211> 100
<212> DNA
<213> Escherichia coli K-12 MG1655
<220> ycah b0916
<222> 803-902
<223> ycah b0916 U00096 967589..968575
<313> 5743: 852-871
<400> 5743
ACCATGCGGA TGTCAGTGCG TTGGTAAGCG CCGGGCAAAC GCTGGTAATG 50
ACTGAAAAAG ATGCGGTGAA ATGCCGGGCC TTTGCAGAAG AAAATTGGTG 100
<210> 5744
<211> 100
<212> DNA
<213> Escherichia coli K-12 MG1655
<220> ycai b0914
<222> 2241-2340
<223> ycai b0914 U00096 963465..965807
<313> 5744: 2270-2289
<400> 5744
GGTCAATTTT TCAGCGCAAG GCTGGCGGAT TAGCAGCCTC AGGGAGCAAA 50
TTTTACCTCG TTGGTATCAT CAGTGGTTTG GCGTGCCAGT GGATAACGGG 100
<210> 5745
<211> 100

<212> DNA
<213> Escherichia coli K-12 MG1655
<220> ycai b0914
<222> 2186-2285
<223> ycai b0914 U00096 963465..965807
<313> 5745: 2229-2248
<400> 5745
GCTATCAACT GCAAGGATAT CAATGGATTG ATACTCCACA TCAAGGTCAA 50
ACAACGGTCA ATTTTTCAGC GCAAGGCTGG CGGATTAGCA GCCTCAGGGA 100
<210> 5746
<211> 100
<212> DNA
<213> Escherichia coli K-12 MG1655
<220> ycai b0914
<222> 2120-2219
<223> ycai b0914 U00096 963465..965807
<313> 5746: 2178-2197
<400> 5746
AAGTGGCACT CGCATCGGCA TCGCGCTATA ACGCATGGCG ACTGCCCTCT 50
AACAAAGTTA AGCATCGCTA TCAACTGCAA GGATATCAAT GGATTGATAC 100
<210> 5747
<211> 100
<212> DNA
<213> Escherichia coli K-12 MG1655
<220> asr asr
<222> 237-336
<223> asr asr U00096 1669373..1669708
<313> 5747: 305-324
<400> 5747
ACAGAAAGCC CCTGAACAAA AAGCGCAGGC AGCGAAGAAA CACGCCAAGA 50
AACACAGCCA TCAGCAACCG GCAAAACCTG CTGCACAACC CGCAGCGTAA 100
<210> 5748
<211> 100
<212> DNA
<213> Escherichia coli K-12 MG1655
<220> asr asr
<222> 223-322
<223> asr asr U00096 1669373..1669708
<313> 5748: 265-284
<400> 5748
AATACGAAAG CTGAACAGAA AGCCCCTGAA CAAAAAGCGC AGGCAGCGAA 50
GAAACACGCC AAGAAACACA GCCATCAGCA ACCGGCAAAA CCTGCTGCAC 100
<210> 5749
<211> 100
<212> DNA
<213> Escherichia coli K-12 MG1655
<220> asr asr
<222> 162-261
<223> asr asr U00096 1669373..1669708
<313> 5749: 216-235
<400> 5749
TAAAAAACAG CATAAAGCAG CACCTGCCCA GAAAGCGCAG GCGGCTAAAA 50
AGCATCATAA AAATACGAAA GCTGAACAGA AAGCCCCTGA ACAAAAAGCG 100
<210> 5750
<211> 100
<212> DNA
<213> Escherichia coli K-12 MG1655
<220> ycaj b0893
<222> 1245-1344
<223> ycaj b0893 U00096 937217..938560
<313> 5750: 1312-1331
<400> 5750
TTATTTCCCG ACAAACAGGG GCCTTGAAGG CAAGATTGGC GAAAAGCTCG 50
CCTGGCTGGC TGAACAGGAT CAAAATAGCC CCATAAAACG CTACCGTTAA 100
<210> 5751
<211> 100
<212> DNA
<213> Escherichia coli K-12 MG1655
<220> ycaj b0893
<222> 1213-1312
<223> ycaj b0893 U00096 937217..938560

<313> 5751: 1233-1252
<400> 5751
TACTTCCCGC CGGAAATAGC ACAAACACGC TATTATTTCC CGACAAACAG 50
GGGCCTTGAA GGCAAGATTG GCGAAAAGCT CGCCTGGCTG GCTGAACAGG 100
<210> 5752
<211> 100
<212> DNA
<213> Escherichia coli K-12 MG1655
<220> ycaj b0893
<222> 1141-1240
<223> ycaj b0893 U00096 937217..938560
<313> 5752: 1185-1204
<400> 5752
AAGGAAATGG GCTACGGGCA GGAATATCGT TACGCTCATG ATGAAGCAAA 50
CGCTTATGCT GCCGGTGAGG TTTACTTCCC GCCGGAAATA GCACAAACAC 100
<210> 5753
<211> 100
<212> DNA
<213> Escherichia coli K-12 MG1655
<220> ycak b0902
<222> 479-578
<223> ycak b0902 U00096 948891..949481
<313> 5753: 533-552
<400> 5753
ATGCCGATGT CACTTTCTTG TGTAATACAG TGGTATTCGA TGGGGAAGAA 50
CTTCACGCGA GCTATTATCA GTCGTTATTA TCTCAGGTAC GGGATATGGT 100
<210> 5754
<211> 100
<212> DNA
<213> Escherichia coli K-12 MG1655
<220> ycak b0902
<222> 354-453
<223> ycak b0902 U00096 948891..949481
<313> 5754: 406-425
<400> 5754
ACCATTCAAT AAAGTTCGTT GGGTGGCGCT GGTTGGAGGA GACAAAGAAT 50
CATTTGTCCA GATGGGCTGG GAAAAAAATA TAAGCGATTA TTTAAAAAAT 100
<210> 5755
<211> 100
<212> DNA
<213> Escherichia coli K-12 MG1655
<220> ycak b0902
<222> 230-329
<223> ycak b0902 U00096 948891..949481
<313> 5755: 283-302
<400> 5755
TGCTTGAACA TGACACGTTA GTGGTGGTTT TTCCTCTCTG GTGGTACAGC 50
TTCCCGGCAA TGCTAAAAGG ATATATTGAC AGAGTATGGA ATAATGGGCT 100
<210> 5756
<211> 100
<212> DNA
<213> Escherichia coli K-12 MG1655
<220> rhlb b3781
<222> 1167-1266
<223> rhlb b3781 U00096 complement(3961980..3963245)
<313> 5756: 1234-1253
<400> 5756
GATGACCGAT CTGCCAAAAC CGCTGCGCCT CACGCGCCCG CGCACAGGCA 50
ATGGTCCGCG TCGTACTGGC GCTCCGCGTA ATCGTCGTCG TTCAGGTTAA 100
<210> 5757
<211> 100
<212> DNA
<213> Escherichia coli K-12 MG1655
<220> rhlb b3781
<222> 1139-1238
<223> rhlb b3781 U00096 complement(3961980..3963245)
<313> 5757: 1188-1207
<400> 5757
CGGTAAGCAA ATACAATCCG GACGCATTGA TGACCGATCT GCCAAAACCG 50
CTGCGCCTCA CGCGCCCGCG CACAGGCAAT GGTCCGCGTC GTACTGGCGC 100
<210> 5758

<211> 100
<212> DNA
<213> Escherichia coli K-12 MG1655
<220> rhlb b3781
<222> 1040-1139
<223> rhlb b3781 U00096 complement(3961980..3963245)
<313> 5758: 1090-1109
<400> 5758
CAGGTCGCGC AGGCGCAAGC GGTCACTCTA TCAGCCTGGC GTGTGAAGAG 50
TATGCATTGA ATTTGCCTGC TATTGAGACC TATATTGGTC ACTCAATTCC 100
<210> 5759
<211> 100
<212> DNA
<213> Escherichia coli K-12 MG1655
<220> ycal b0910
<222> 668-767
<223> ycal b0910 U00096 959463..960251
<313> 5759: 715-734
<400> 5759
TTGGCAGCTT TGAAACACTG GCTAGCCTGG ATGGCGGTCG CACCCAGTCC 50
ATGTTTGACT CTCACCCACC ATCAACAGAG CGTGCGCAAC ACATCCGTGA 100
<210> 5760
<211> 100
<212> DNA
<213> Escherichia coli K-12 MG1655
<220> ycal b0910
<222> 615-714
<223> ycal b0910 U00096 959463..960251
<313> 5760: 641-660
<400> 5760
TGATTTCTCC TTTGATCTGT TGAAGAAACG TGGCATCAGC ACCCAGGGGC 50
TGGTTGGCAG CTTTGAAACA CTGGCTAGCC TGGATGGCGG TCGCACCCAG 100
<210> 5761
<211> 100
<212> DNA
<213> Escherichia coli K-12 MG1655
<220> ycal b0910
<222> 527-626
<223> ycal b0910 U00096 959463..960251
<313> 5761: 575-594
<400> 5761
CTTCCCAGCT TTCCCGCTCA CAATTAGGTG ATATCGCAGA AGGCGCTATC 50
AATGCTAAAT ACTCCCGTGA TAAAGAGTCC GAAGCAGATG ATTTCTCCTT 100
<210> 5762
<211> 100
<212> DNA
<213> Escherichia coli K-12 MG1655
<220> b3050 b3050
<222> 531-630
<223> b3050 b3050 U00096 3190224..3190853
<313> 5762: 584-603
<400> 5762
AGGAAAAATT TTCACTAAAG GAGACAAAGT GTTAATTATT TGCCGACTTT 50
CTGCAACGCG ATATTTGGCG GAAAATAATC CCTGGCCCCA AATATTGTAA 100
<210> 5763
<211> 100
<212> DNA
<213> Escherichia coli K-12 MG1655
<220> b3050 b3050
<222> 414-513
<223> b3050 b3050 U00096 3190224..3190853
<313> 5763: 473-492
<400> 5763
ATATATTGGT AGCATGGCGT TAATTACCGG TCATCAAGCC ACTTCAGGTA 50
ATCCCTGCGA AGGAAAACTC ACCGATCAAT TTGGCCAAAT ACATTATTTA 100
<210> 5764
<211> 100
<212> DNA
<213> Escherichia coli K-12 MG1655
<220> b3050 b3050
<222> 328-427

<223> b3050 b3050 U00096 3190224..3190853
<313> 5764: 387-406
<400> 5764
TTTACGATTA TTGCGGTGCA TTATACCGGT AAAATAGTTG CGCCCTGGAT 50
TCCACGCGAC CATAGTTCAG CTATTACAGA AGAAGAATAT ATTGGTAGCA 100
<210> 5765
<211> 100
<212> DNA
<213> Escherichia coli K-12 MG1655
<220> ycan b0901
<222> 765-864
<223> ycan b0901 U00096 complement(947883..948791)
<313> 5765: 813-832
<400> 5765
TGTACTGGAA GACTGGAGCA CAGAGCGTCC AGGCTTCCAG ATCTATTATC 50
CAAACCGCCA GTATATGTCC TGCGGCTTGC GCGCATTTCT CGATTATGTA 100
<210> 5766
<211> 100
<212> DNA
<213> Escherichia coli K-12 MG1655
<220> ycan b0901
<222> 747-846
<223> ycan b0901 U00096 complement(947883..948791)
<313> 5766: 770-789
<400> 5766
TACTGGTCGT TTAGAATGTG TACTGGAAGA CTGGAGCACA GAGCGTCCAG 50
GCTTCCAGAT CTATTATCCA AACCGCCAGT ATATGTCCTG CGGCTTGCGC 100
<210> 5767
<211> 100
<212> DNA
<213> Escherichia coli K-12 MG1655
<220> ycan b0901
<222> 649-748
<223> ycan b0901 U00096 complement(947883..948791)
<313> 5767: 681-700
<400> 5767
AATATCATTC TGGATGATGT TGATGCTGAA CTGGAGGCCG TTTTGATGGG 50
TGCTGGTATC GGGTATCTGC TCTATGAACA GATCAAGGAG TACCTTGATA 100
<210> 5768
<211> 100
<212> DNA
<213> Escherichia coli K-12 MG1655
<220> rhle b0798
<222> 1266-1365
<223> rhle b0798 U00096 830095..831459
<313> 5768: 1336-1355
<400> 5768
GAAACCTGCA GAAAAACCGT CTCGCCGCCT CGGCGATGCC AAACCGGCAG 50
GCGAACAACA ACGTCGCCGC CGTCCGCGTA AACCTGCCGC TGCGCAGTAA 100
<210> 5769
<211> 100
<212> DNA
<213> Escherichia coli K-12 MG1655
<220> rhle b0798
<222> 1187-1286
<223> rhle b0798 U00096 830095..831459
<313> 5769: 1253-1272
<400> 5769
GCGGCGGTCG TGGGCAAGGT GGTGGTCGCG GTCAACAGCA ACCACGCCGT 50
GGGGAAGGTG GCGCAAAATC TGCAAGCGCG AAACCTGCAG AAAAACCGTC 100
<210> 5770
<211> 100
<212> DNA
<213> Escherichia coli K-12 MG1655
<220> rhle b0798
<222> 1105-1204
<223> rhle b0798 U00096 830095..831459
<313> 5770: 1148-1167
<400> 5770
CCGCGCATTG CGATTCCGGG CTATGAGCCG GACCCGTCAA TCAAAGCCGA 50
ACCGATCCAG AACGGTCGCC AGCAACGTGG CGGCGGCGGT CGTGGGCAAG 100

<210> 5771
<211> 100
<212> DNA
<213> Escherichia coli K-12 MG1655
<220> b2322 b2323
<222> 1080-1179
<223> b2322 b2323 U00096 complement(2436962..2438140)
<313> 5771: 1147-1166
<400> 5771
GCTGGCGACC ACGTTTGGTT ACTCTTCGGT ATTTCTTGCC GGGGCGATCT 50
CTGCGGTGCT GGGTATTATT GTCACGATAC TGTCATTTCG TCGGGGTTAA 100
<210> 5772
<211> 100
<212> DNA
<213> Escherichia coli K-12 MG1655
<220> b2322 b2323
<222> 981-1080
<223> b2322 b2323 U00096 complement(2436962..2438140)
<313> 5772: 1008-1027
<400> 5772
GGTTAAACGC GTCCCCTCAC AAGTTCGCGG CACCGCACTG GGCGGTTACG 50
CCGCGTTTCA GGATATCGCC CTCGGCGTCT CCGGGCCGCT GGCGGGAATG 100
<210> 5773
<211> 100
<212> DNA
<213> Escherichia coli K-12 MG1655
<220> b2322 b2323
<222> 872-971
<223> b2322 b2323 U00096 complement(2436962..2438140)
<313> 5773: 915-934
<400> 5773
TGGGCTTGTT GCTGCTCTGG CAAGCCCCAG GTGCATGGGT CGCATTAGCG 50
GGCGCGGCGT TAACCGGAGC CGGATGTTCG CTTATCTTTC CTGCGCTGGG 100
<210> 5774
<211> 100
<212> DNA
<213> Escherichia coli K-12 MG1655
<220> b3051 b3051
<222> 1563-1662
<223> b3051 b3051 U00096 3190880..3192541
<313> 5774: 1630-1649
<400> 5774
TGACTCCTTG CTCAATGAAA TTGGCGTTTC AGGCGGCTCA CTGGCGGCAT 50
TGACTTCACC CTTAACCTCA ACAACTCCCG TCGAAGAAAA AGCAGAGTAA 100
<210> 5775
<211> 100
<212> DNA
<213> Escherichia coli K-12 MG1655
<220> b3051 b3051
<222> 1546-1645
<223> b3051 b3051 U00096 3190880..3192541
<313> 5775: 1584-1603
<400> 5775
ACACAGGCAC CGCTGATTGA CTCCTTGCTC AATGAAATTG GCGTTTCAGG 50
CGGCTCACTG GCGGCATTGA CTTCACCCTT AACCTCAACA ACTCCCGTCG 100
<210> 5776
<211> 100
<212> DNA
<213> Escherichia coli K-12 MG1655
<220> b3051 b3051
<222> 1517-1616
<223> b3051 b3051 U00096 3190880..3192541
<313> 5776: 1541-1560
<400> 5776
AAGCATTATC AGCCGCTCTC TCTTACCGCA CACAGGCACC GCTGATTGAC 50
TCCTTGCTCA ATGAAATTGG CGTTTCAGGC GGCTCACTGG CGGCATTGAC 100
<210> 5777
<211> 100
<212> DNA
<213> Escherichia coli K-12 MG1655
<220> nlpa b3662

<222> 720-819
<223> nlpa b3662 U00096 complement(3836802..3837620)
<313> 5777: 792-811
<400> 5777
TGCAGAAAAC GTGAAGGAAT TTCTGCAATC TTATCAATCA CCCGAAGTCG  50
CTAAAGCGGC AGAAACCATT TTTAACGGTG GCGCGGTGCC TGGCTGGTAA  100
<210> 5778
<211> 100
<212> DNA
<213> Escherichia coli K-12 MG1655
<220> nlpa b3662
<222> 659-758
<223> nlpa b3662 U00096 complement(3836802..3837620)
<313> 5778: 700-719
<400> 5778
TTATTGAAGA TAAGAATTCG CCGTATGTGA ATATTTTGGT GGCACGGGAA  50
GATAATAAGA ATGCAGAAAA CGTGAAGGAA TTTCTGCAAT CTTATCAATC  100
<210> 5779
<211> 100
<212> DNA
<213> Escherichia coli K-12 MG1655
<220> nlpa b3662
<222> 592-691
<223> nlpa b3662 U00096 complement(3836802..3837620)
<313> 5779: 614-633
<400> 5779
GATGTAGCGA TTATCAGCAC CACTTACATT CAGCAGACCG GGCTTTCTCC  50
GGTGCACGAC AGCGTATTTA TTGAAGATAA GAATTCGCCG TATGTGAATA  100
<210> 5780
<211> 100
<212> DNA
<213> Escherichia coli K-12 MG1655
<220> ycao b0906
<222> 1604-1703
<223> ycao b0906 U00096 complement(954095..955864)
<313> 5780: 1648-1667
<400> 5780
ACGGCGCAGA TGCCGTAGAA GCCGCCAGTG CGGCAATGAG CGGCGAAGCG  50
GCGTTTTACG GCCTGCAACC GGTAGATAGC GATCTGCACG CGTTTGCTGC  100
<210> 5781
<211> 100
<212> DNA
<213> Escherichia coli K-12 MG1655
<220> ycao b0906
<222> 1515-1614
<223> ycao b0906 U00096 complement(954095..955864)
<313> 5781: 1557-1576
<400> 5781
CTATTATCGC TGCCTGCAAA CGTTGTTATT ACTGGCACAG GAAGAAGATC  50
GCCAGCCGCT GCAATATCTG AATGCGTTTG TTCGCATGTA CGGCGCAGAT  100
<210> 5782
<211> 100
<212> DNA
<213> Escherichia coli K-12 MG1655
<220> ycao b0906
<222> 1466-1565
<223> ycao b0906 U00096 complement(954095..955864)
<313> 5782: 1526-1545
<400> 5782
AATGGACGAT GGAGTTTAAC TCATCAGTAT TTAGTCCGGA ACGCGCCAAC  50
TATTATCGCT GCCTGCAAAC GTTGTTATTA CTGGCACAGG AAGAAGATCG  100
<210> 5783
<211> 100
<212> DNA
<213> Escherichia coli K-12 MG1655
<220> b3052 b3053
<222> 1335-1434
<223> b3052 b3053 U00096 complement(3192961..3194394)
<313> 5783: 1402-1421
<400> 5783
AGTCTGGGCC AACGGTGGCG AAGTGTTGGT GCTCAACTTT GAAGACGGTT  50

GCTCGACGAC CAACATCATC AAGAAGATCC AACAGGATAA AAAAGGCTAA 100
<210> 5784
<211> 100
<212> DNA
<213> Escherichia coli K-12 MG1655
<220> b3052 b3053
<222> 1236-1335
<223> b3052 b3053 U00096 complement(3192961..3194394)
<313> 5784: 1295-1314
<400> 5784
TGAAGAGGAC ACGCCGCAGC GCTTGATCGC CGGGATCTTG CCAGATCTGC 50
TGGTGAAAGG CGGCGACTAT AAACCAGAAG AGATTGCCGG GAGTAAAGAA 100
<210> 5785
<211> 100
<212> DNA
<213> Escherichia coli K-12 MG1655
<220> b3052 b3053
<222> 1108-1207
<223> b3052 b3053 U00096 complement(3192961..3194394)
<313> 5785: 1158-1177
<400> 5785
TTGATTGTTG CCGTCAACAG CGATGCCTCC ACCAAACGGC TGAAAGGGGA 50
TTCCCGCCCG GTAAACCCAC TCGAACAGCG TATGATTGTG CTGGGCGCAC 100
<210> 5786
<211> 100
<212> DNA
<213> Escherichia coli K-12 MG1655
<220> nlpb b2478
<222> 918-1017
<223> nlpb b2478 U00096 complement(2595851..2596888)
<313> 5786: 957-976
<400> 5786
GCAGGTTGGC GATTTAGATA ACCGCAGCAG CCTACAGTTC ATCGATCCGA 50
AAGGTCATAC TCTGACTCAG AGTCAGAACG ACGCGCTGGT AGCTGTCTTC 100
<210> 5787
<211> 100
<212> DNA
<213> Escherichia coli K-12 MG1655
<220> nlpb b2478
<222> 824-923
<223> nlpb b2478 U00096 complement(2595851..2596888)
<313> 5787: 859-878
<400> 5787
GCAACATGGC CGTAACTTAT AAGCCGCTGT CTGACAGCGA CTGGCAGGAA 50
CTGGGCGCGA GCGATCCAGG CCTGGCATCC GGTGACTATA AACTGCAGGT 100
<210> 5788
<211> 100
<212> DNA
<213> Escherichia coli K-12 MG1655
<220> nlpb b2478
<222> 777-876
<223> nlpb b2478 U00096 complement(2595851..2596888)
<313> 5788: 804-823
<400> 5788
GCTGGAAAAA GTGGGCATGA AAGTGACCGA CAGCACCCGT TCGCAGGGCA 50
ACATGGCCGT AACTTATAAG CCGCTGTCTG ACAGCGACTG GCAGGAACTG 100
<210> 5789
<211> 100
<212> DNA
<213> Escherichia coli K-12 MG1655
<220> ycap b0907
<222> 594-693
<223> ycap b0907 U00096 955985..956677
<313> 5789: 656-675
<400> 5789
TAGTGAAATC ATTCATATGA AAGCGGGGGA AAAACAATTA TGTCCGCGCT 50
GTGCAAATCC AGAATGGACG AAGGCAAGTC GGGCAAAACG GGTGACCTGA 100
<210> 5790
<211> 100
<212> DNA
<213> Escherichia coli K-12 MG1655

GCCCCGCTGT GTGCCGAGAT TCTGGCGGCG CAGATGAGCG ACGAACCGAT 50
TCCGATGGAT GCCAGTACGC TGGCGGCGTT AAACCCGAAT CGGTTATGGG 100
<210> 5797
<211> 100
<212> DNA
<213> Escherichia coli K-12 MG1655
<220> b2324 b2324
<222> 1878-1977
<223> b2324 b2324 U00096 2439724..2441790
<313> 5797: 1920-1939
<400> 5797
TGACGATCTC TTTATGTTTG CGGCTTTAGG TTCTCGCGGT TTGTGTTCTG 50
CCCCGCTGTG TGCCGAGATT CTGGCGGCGC AGATGAGCGA CGAACCGATT 100
<210> 5798
<211> 100
<212> DNA
<213> Escherichia coli K-12 MG1655
<220> nlpc b1709
<222> 366-465
<223> nlpc b1709 U00096 complement(1790291..1790755)
<313> 5798: 444-463
<400> 5798
CAACCAATTT ATCCACGCCT CTACCAGCAA GGGAGTGATG CGTTCCTCAC 50
TTGATAATGT CTATTGGCAG AAAAATTTCT GGCAGGCGAG ACGAATCTAG 100
<210> 5799
<211> 100
<212> DNA
<213> Escherichia coli K-12 MG1655
<220> nlpc b1709
<222> 356-455
<223> nlpc b1709 U00096 complement(1790291..1790755)
<313> 5799: 397-416
<400> 5799
ACGATACCAA CAACCAATTT ATCCACGCCT CTACCAGCAA GGGAGTGATG 50
CGTTCCTCAC TTGATAATGT CTATTGGCAG AAAAATTTCT GGCAGGCGAG 100
<210> 5800
<211> 100
<212> DNA
<213> Escherichia coli K-12 MG1655
<220> nlpc b1709
<222> 234-333
<223> nlpc b1709 U00096 complement(1790291..1790755)
<313> 5800: 283-302
<400> 5800
GCCCCGTGAA ACCAAACAAC AAGCCTCTAT TGGCACGCAA ATTGATAAAG 50
ACGAGTTGCT GCCAGGTGAC CTGGTCTTTT TCAAAACGGG GTCCGGACAA 100
<210> 5801
<211> 100
<212> DNA
<213> Escherichia coli K-12 MG1655
<220> ycaq b0917
<222> 1120-1219
<223> ycaq b0917 U00096 968612..969844
<313> 5801: 1142-1161
<400> 5801
GCGATTACTG ATTTCGCTAA CTGGCAGCAG GCAACGCGGG TGACATTAGG 50
ATGCTGCCCG CAAGGCCTCT TTACTGATTG CCGCACCGGC TGGGAAATAG 100
<210> 5802
<211> 100
<212> DNA
<213> Escherichia coli K-12 MG1655
<220> ycaq b0917
<222> 1052-1151
<223> ycaq b0917 U00096 968612..969844
<313> 5802: 1104-1123
<400> 5802
AAGTTATCTC TCTGTGGTTA CAGGAAGGTA TTAAACCAAC GACAACGCTG 50
CAAAAAGGGC TACGTCAGGC GATTACTGAT TTCGCTAACT GGCAGCAGGC 100
<210> 5803
<211> 100
<212> DNA

<213> Escherichia coli K-12 MG1655
<220> ycaq b0917
<222> 995-1094
<223> ycaq b0917 U00096 968612..969844
<313> 5803: 1045-1064
<400> 5803
GTGGGCAATT AGTTGGGCGA ATGGATGCCA AAATGCATCG CCAGACAGGC  50
ATCCTTGAAG TTATCTCTCT GTGGTTACAG GAAGGTATTA AACCAACGAC  100
<210> 5804
<211> 100
<212> DNA
<213> Escherichia coli K-12 MG1655
<220> b2325 b2325
<222> 180-279
<223> b2325 b2325 U00096 complement(2441911..2442189)
<313> 5804: 243-262
<400> 5804
TCTGGAAAAA GCCATTGGTG CTGGTGAATT GTCGCCGCGT GACCAGGCGC  50
TGGTGACCGA TATGTGGGAA AACCTGTTTC AGCAGGCGTC ACAGCAGTAA  100
<210> 5805
<211> 100
<212> DNA
<213> Escherichia coli K-12 MG1655
<220> b2325 b2325
<222> 124-223
<223> b2325 b2325 U00096 complement(2441911..2442189)
<313> 5805: 166-185
<400> 5805
CTGGAAAGTG GTGATGACCA CTCCGCTCAG GCGGTGCATA CGACCGTTAG  50
CCAGAGTCTG GAAAAAGCCA TTGGTGCTGG TGAATTGTCG CCGCGTGACC  100
<210> 5806
<211> 100
<212> DNA
<213> Escherichia coli K-12 MG1655
<220> b2325 b2325
<222> 73-172
<223> b2325 b2325 U00096 complement(2441911..2442189)
<313> 5806: 112-131
<400> 5806
TTGTTTGCCA GTGGGTATTT GCGTGGCCAC CTGACATTAG CCATCGCAGA  50
ACTGGAAAGT GGTGATGACC ACTCCGCTCA GGCGGTGCAT ACGACCGTTA  100
<210> 5807
<211> 100
<212> DNA
<213> Escherichia coli K-12 MG1655
<220> nlpd b2743
<222> 1041-1140
<223> nlpd b2743 U00096 complement(2865637..2866776)
<313> 5807: 1108-1127
<400> 5807
GACCATGGGT AGCACCGGAA CCAGTTCAAC ACGCTTGCAT TTTGAAATTC  50
GTTACAAGGG GAAATCCGTA AACCCGCTGC GTTATTTGCC GCAGCGATAA  100
<210> 5808
<211> 100
<212> DNA
<213> Escherichia coli K-12 MG1655
<220> nlpd b2743
<222> 1021-1120
<223> nlpd b2743 U00096 complement(2865637..2866776)
<313> 5808: 1058-1077
<400> 5808
AAGGCGGGGC AAAAAATAGC GACCATGGGT AGCACCGGAA CCAGTTCAAC  50
ACGCTTGCAT TTTGAAATTC GTTACAAGGG GAAATCCGTA AACCCGCTGC  100
<210> 5809
<211> 100
<212> DNA
<213> Escherichia coli K-12 MG1655
<220> nlpd b2743
<222> 976-1075
<223> nlpd b2743 U00096 complement(2865637..2866776)
<313> 5809: 1019-1038

<400> 5809
TACGCCCATA ACGACACAAT GCTGGTCCGG GAACAACAAG AAGTTAAGGC 50
GGGGCAAAAA ATAGCGACCA TGGGTAGCAC CGGAACCAGT TCAACACGCT 100
<210> 5810
<211> 100
<212> DNA
<213> Escherichia coli K-12 MG1655
<220> ycar b0918
<222> 84-183
<223> ycar b0918 U00096 969896..970078
<313> 5810: 155-174
<400> 5810
TTGCAAACTG GACAACCTCG CTTTCCCGCT GCGTGATGGC ATTCCAGTGT 50
TGCTGGAAAC GGAAGCCCGC GTGCTGACTG CTGATGAGAG TAAATCATGA 100
<210> 5811
<211> 100
<212> DNA
<213> Escherichia coli K-12 MG1655
<220> ycar b0918
<222> 53-152
<223> ycar b0918 U00096 969896..970078
<313> 5811: 98-117
<400> 5811
TTTGGTATAA CCAGGAAAAA CAAGAGCTTA TTTGCAAACT GGACAACCTC 50
GCTTTCCCGC TGCGTGATGG CATTCCAGTG TTGCTGGAAA CGGAAGCCCG 100
<210> 5812
<211> 100
<212> DNA
<213> Escherichia coli K-12 MG1655
<220> ycar b0918
<222> 1-100
<223> ycar b0918 U00096 969896..970078
<313> 5812: 44-63
<400> 5812
ATGGATCATC GTCTGCTTGA AATCATTGCC TGCCCGGTTT GCAACGGAAA 50
ACTTTGGTAT AACCAGGAAA AACAAGAGCT TATTTGCAAA CTGGACAACC 100
<210> 5813
<211> 100
<212> DNA
<213> Escherichia coli K-12 MG1655
<220> b2326 b2327
<222> 431-530
<223> b2326 b2327 U00096 complement(2442223..2442771)
<313> 5813: 465-484
<400> 5813
ATTATCTGAC AAATGGCATT CCGGAACGTC CGGCACGCTT CATTAAAGCA 50
CTACAAAATT ATTATCACAC GCCGGAACTT ACGGCGGAAC AGTTCCCGTG 100
<210> 5814
<211> 100
<212> DNA
<213> Escherichia coli K-12 MG1655
<220> b2326 b2327
<222> 263-362
<223> b2326 b2327 U00096 complement(2442223..2442771)
<313> 5814: 281-300
<400> 5814
CCCAAACGCA AAGCCAGTTT GAAGATGTTG AAGTGAAGCC GCAGGCGCTC 50
GACTGGTTGT TCTGTGTGGC GGCGGGATAT CCGTTTAACG TCAGTTGCGA 100
<210> 5815
<211> 100
<212> DNA
<213> Escherichia coli K-12 MG1655
<220> b2326 b2327
<222> 126-225
<223> b2326 b2327 U00096 complement(2442223..2442771)
<313> 5815: 140-159
<400> 5815
ACCGTATAAC CGAATCGTCT TTGCTCATGG CTTTTATGCC AGCGCAATTC 50
ATGAGATTTC GCACTGGTGT ATTGCCGGGA AAGCGCGCCG TGAGCTGGTT 100
<210> 5816
<211> 100

<212> DNA
<213> Escherichia coli K-12 MG1655
<220> ycbb b0926
<222> 1749-1848
<223> ycbb b0926 U00096 980270..982117
<313> 5816: 1826-1845
<400> 5816
AGATGGTCGT ACCCAGTATC GTACAGATAT TTACAATTAT GATCTGCCTG 50
CGCGATCCAG CTCGCAAATC GTATCGAAAG CGGAACAATT AATCAGGTAA 100
<210> 5817
<211> 100
<212> DNA
<213> Escherichia coli K-12 MG1655
<220> ycbb b0926
<222> 1691-1790
<223> ycbb b0926 U00096 980270..982117
<313> 5817: 1748-1767
<400> 5817
TCAATATTCG GCAGTCGATT CCGGTGAATC TCTACTACCT GACGGCCTTT 50
GTTGGTGCAG ATGGTCGTAC CCAGTATCGT ACAGATATTT ACAATTATGA 100
<210> 5818
<211> 100
<212> DNA
<213> Escherichia coli K-12 MG1655
<220> ycbb b0926
<222> 1648-1747
<223> ycbb b0926 U00096 980270..982117
<313> 5818: 1668-1687
<400> 5818
AAACGTATTT CTGATGCGCT GAAGCAGGGT GATACACGTT ACGTCAATAT 50
TCGGCAGTCG ATTCCGGTGA ATCTCTACTA CCTGACGGCC TTTGTTGGTG 100
<210> 5819
<211> 100
<212> DNA
<213> Escherichia coli K-12 MG1655
<220> ycbc b0921
<222> 660-759
<223> ycbc b0921 U00096 complement(971845..972624)
<313> 5819: 722-741
<400> 5819
GTGGGAACGG GCAATCCCTT CTCCGGTCTG GTTGATGCAT AGCGATCGTG 50
TTGGCTATGA AACGCTTGGC CGTATCTGGC AGTGGCTAAA AGGTTCGTCA 100
<210> 5820
<211> 100
<212> DNA
<213> Escherichia coli K-12 MG1655
<220> ycbc b0921
<222> 621-720
<223> ycbc b0921 U00096 complement(971845..972624)
<313> 5820: 676-695
<400> 5820
CCCGGCGAAT CAGTTGGCCA TCGACTCGCC GCTTAACCCG TGGGAACGGG 50
CAATCCCTTC TCCGGTCTGG TTGATGCATA GCGATCGTGT TGGCTATGAA 100
<210> 5821
<211> 100
<212> DNA
<213> Escherichia coli K-12 MG1655
<220> ycbc b0921
<222> 578-677
<223> ycbc b0921 U00096 complement(971845..972624)
<313> 5821: 602-621
<400> 5821
CGATGATCTT TTTTCAGCAG GAAGGGTTAA ATCCGCTACC AGCCCCGGCG 50
AATCAGTTGG CCATCGACTC GCCGCTTAAC CCGTGGGAAC GGGCAATCCC 100
<210> 5822
<211> 100
<212> DNA
<213> Escherichia coli K-12 MG1655
<220> ycbe b0934
<222> 669-768
<223> ycbe b0934 U00096 complement(992500..993267)

<313> 5822: 738-757
<400> 5822
TCGATTAGGG TCGGTGCGAT TAGCGGAGCT GGAAGCGGAG GTGTTGCAAC 50
GGGTGATGCA ACGTGGTGAA TCTGAAACTC GCCTGAGAAA ACAGGGGTAA 100
<210> 5823
<211> 100
<212> DNA
<213> Escherichia coli K-12 MG1655
<220> ycbe b0934
<222> 629-728
<223> ycbe b0934 U00096 complement(992500..993267)
<313> 5823: 685-704
<400> 5823
AAATTGGTCT GGATTTGACA GTGGATATCC CCCGTCCGCG TCGATTAGGG 50
TCGGTGCGAT TAGCGGAGCT GGAAGCGGAG GTGTTGCAAC GGGTGATGCA 100
<210> 5824
<211> 100
<212> DNA
<213> Escherichia coli K-12 MG1655
<220> ycbe b0934
<222> 578-677
<223> ycbe b0934 U00096 complement(992500..993267)
<313> 5824: 615-634
<400> 5824
TGAGCGAAGC GGTAGCGATG GCTGACCGGG TGCTGTTAAT TGAAGAGGGA 50
AAAATTGGTC TGGATTTGAC AGTGGATATC CCCCGTCCGC GTCGATTAGG 100
<210> 5825
<211> 100
<212> DNA
<213> Escherichia coli K-12 MG1655
<220> ycbf b0945
<222> 639-738
<223> ycbf b0945 U00096 1003143..1003880
<313> 5825: 700-719
<400> 5825
GACAAGCTGG TGTCAGGCAA TCGCACCGTG TCATGTCGCC TGGCGGGCGA 50
TAAATGATTA CGGCGGGTTG AGTGCAAAGA AGGAGCAAAA TCTGCCCTGA 100
<210> 5826
<211> 100
<212> DNA
<213> Escherichia coli K-12 MG1655
<220> ycbf b0945
<222> 561-660
<223> ycbf b0945 U00096 1003143..1003880
<313> 5826: 626-645
<400> 5826
CATTAACCTG ATTCAGGTGA GTGTAAATGG TAAAGCGTTA AGTAATGTGG 50
GAGTTGTGCC GCCTAAAAGC CAGCGTCAGA CAAGCTGGTG TCAGGCAATC 100
<210> 5827
<211> 100
<212> DNA
<213> Escherichia coli K-12 MG1655
<220> ycbf b0945
<222> 485-584
<223> ycbf b0945 U00096 1003143..1003880
<313> 5827: 521-540
<400> 5827
CGCTGGAAGC TTATCAACAA TTACGCTGGA CACGGAATTC GCAGGGTGTA 50
CAACTCACTA ACCCAACGCC TTATTACATT AACCTGATTC AGGTGAGTGT 100
<210> 5828
<211> 100
<212> DNA
<213> Escherichia coli K-12 MG1655
<220> ycbg b0957
<222> 220-319
<223> ycbg b0957 U00096 1017708..1018160
<313> 5828: 257-276
<400> 5828
ATTCGGGCAA GACGAAAGCG CCATTTTAAT GCGGAACACC AGCATACGCG 50
CAAAAAATCG ATCGATCTGG AATTTATTGT CTGGCAACGT CTGGCTGGTC 100
<210> 5829

<211> 100
<212> DNA
<213> Escherichia coli K-12 MG1655
<220> ycbg b0957
<222> 165-264
<223> ycbg b0957 U00096 1017708..1018160
<313> 5829: 217-236
<400> 5829
TGGCTGGATT GACAAGCATA TGAATCCGGA ACTGGTCAAT CGCATGAAGC 50
AAACCATTCG GGCAAGACGA AAGCGCCATT TTAATGCGGA ACACCAGCAT 100
<210> 5830
<211> 100
<212> DNA
<213> Escherichia coli K-12 MG1655
<220> ycbg b0957
<222> 108-207
<223> ycbg b0957 U00096 1017708..1018160
<313> 5830: 156-175
<400> 5830
CGCCCAGGAA GCCGTCGATG TGTTGCTCTC GCTGGAAAAT GAACCCGTGC 50
TGGTAAATGG CTGGATTGAC AAGCATATGA ATCCGGAACT GGTCAATCGC 100
<210> 5831
<211> 100
<212> DNA
<213> Escherichia coli K-12 MG1655
<220> ycbk b0927
<222> 450-549
<223> ycbk b0927 U00096 982298..982846
<313> 5831: 484-503
<400> 5831
TATTCGCAAA GCCGCGTTAT CTATGCGCGC AGGTGGTGTA GGATATTATC 50
CACGTAGTAA CTTTGTGCAT ATTGATACCG GGCCAGCACG GCACTGGTAG 100
<210> 5832
<211> 100
<212> DNA
<213> Escherichia coli K-12 MG1655
<220> ycbk b0927
<222> 417-516
<223> ycbk b0927 U00096 982298..982846
<313> 5832: 434-453
<400> 5832
GGATTTCCAT ATTGAAGGTA TCGCGTTAAG CAATATTCGC AAAGCCGCGT 50
TATCTATGCG CGCAGGTGGT GTAGGATATT ATCCACGTAG TAACTTTGTG 100
<210> 5833
<211> 100
<212> DNA
<213> Escherichia coli K-12 MG1655
<220> ycbk b0927
<222> 366-465
<223> ycbk b0927 U00096 982298..982846
<313> 5833: 402-421
<400> 5833
CCGCAGCCGT GGAGTAGCGA AGAAAAGCTA TCACACTAAA GGCCAGGCGA 50
TGGATTTCCA TATTGAAGGT ATCGCGTTAA GCAATATTCG CAAAGCCGCG 100
<210> 5834
<211> 100
<212> DNA
<213> Escherichia coli K-12 MG1655
<220> b1600 b1600
<222> 267-366
<223> b1600 b1600 U00096 complement(1671160..1671525)
<313> 5834: 324-343
<400> 5834
TGCCGGGTTA ACCACCCTGG TCGCCGGGAT TGTGTTGATA AAATCAGGTA 50
CCCGTAAAGC GCGTAAACCT GAACTGGAGG TGAACCATGG CGCAGTTTGA 100
<210> 5835
<211> 100
<212> DNA
<213> Escherichia coli K-12 MG1655
<220> b1600 b1600
<222> 132-231

<223> b1600 b1600 U00096 complement(1671160..1671525)
<313> 5835: 181-200
<400> 5835
GTATATATTT CTCTCTTTCG CCGTTAAAAA AATCGCCTTA GGCGTAGCTT 50
ATGCGCTGTG GGAAGGTATC GGTATTTTAT TTATTACCTT GTTTAGCGTT 100
<210> 5836
<211> 100
<212> DNA
<213> Escherichia coli K-12 MG1655
<220> b1600 b1600
<222> 56-155
<223> b1600 b1600 U00096 complement(1671160..1671525)
<313> 5836: 86-105
<400> 5836
CGCTGTCAAT GAAATGGGCG AGCGTCAGTG AGGGAAATGG CGGCTTTATT 50
TTAATGCTGG TGATGATTTC TCTGTCGTAT ATATTTCTCT CTTTCGCCGT 100
<210> 5837
<211> 100
<212> DNA
<213> Escherichia coli K-12 MG1655
<220> ycbl b0928
<222> 549-648
<223> ycbl b0928 U00096 982873..983520
<313> 5837: 627-646
<400> 5837
GGATGACGTG ATATTTATTC CGGGTCACGG ACCATTATCC ACACTTGGTT 50
ATGAACGCCT GCATAATCCC TTCCTGCAAG ACGAAATGCC CGTCTGGTAA 100
<210> 5838
<211> 100
<212> DNA
<213> Escherichia coli K-12 MG1655
<220> ycbl b0928
<222> 548-647
<223> ycbl b0928 U00096 982873..983520
<313> 5838: 583-602
<400> 5838
GGGATGACGT GATATTTATT CCGGGTCACG GACCATTATC CACACTTGGT 50
TATGAACGCC TGCATAATCC CTTCCTGCAA GACGAAATGC CCGTCTGGTA 100
<210> 5839
<211> 100
<212> DNA
<213> Escherichia coli K-12 MG1655
<220> ycbl b0928
<222> 478-577
<223> ycbl b0928 U00096 982873..983520
<313> 5839: 532-551
<400> 5839
CGCAGTGACT TCCCGCGTGG CGATCATAAT CAACTGATTT CTTCAATCAA 50
AGATAAATTG CTGCCACTGG GGGATGACGT GATATTTATT CCGGGTCACG 100
<210> 5840
<211> 100
<212> DNA
<213> Escherichia coli K-12 MG1655
<220> b1601 b1602
<222> 936-1035
<223> b1601 b1602 U00096 1671937..1672971
<313> 5840: 1010-1029
<400> 5840
GCTTTCCGTG CCGTTGACAA TTATTGTCAA AATTGCGCTT GAACAAACAG 50
CGGGAGGTCA AAGCATCGCC GTTCTGTTAA GCGATCTCAA TAAAGAGTGA 100
<210> 5841
<211> 100
<212> DNA
<213> Escherichia coli K-12 MG1655
<220> b1601 b1602
<222> 812-911
<223> b1601 b1602 U00096 1671937..1672971
<313> 5841: 862-881
<400> 5841
TTAATCTGGT CTTCGGCAAT ATTCTGGAGC CGCGTATCAT GGGGCGTGGG 50
CTGGGGCTTT CCACATTGGT GGTATTTTTG TCGTTGATTT TTTGGGGATG 100

<210> 5842
<211> 100
<212> DNA
<213> Escherichia coli K-12 MG1655
<220> b1601 b1602
<222> 767-866
<223> b1601 b1602 U00096 1671937..1672971
<313> 5842: 830-849
<400> 5842
ATGGCTTCTA CGAAGCGTTG CTGGTGCTGG CGGGATATCT GCTGATTAAT 50
CTGGTCTTCG GCAATATTCT GGAGCCGCGT ATCATGGGGC GTGGGCTGGG 100
<210> 5843
<211> 100
<212> DNA
<213> Escherichia coli K-12 MG1655
<220> ycbm b0935
<222> 738-837
<223> ycbm b0935 U00096 complement(993264..994100)
<313> 5843: 800-819
<400> 5843
CGCCCTGCTC GGCAAACTGG CTGACGTCAG CGCGCAGCTG TTAGAGCGCC 50
TCTGGTTGCG CTGGAACCCG GCTTATCATT TGAAGGAGGC CACGGTATGA 100
<210> 5844
<211> 100
<212> DNA
<213> Escherichia coli K-12 MG1655
<220> ycbm b0935
<222> 708-807
<223> ycbm b0935 U00096 complement(993264..994100)
<313> 5844: 740-759
<400> 5844
GGACGTGGTG GTAGTCGCTA TTATTCTTTA CGCCCTGCTC GGCAAACTGG 50
CTGACGTCAG CGCGCAGCTG TTAGAGCGCC TCTGGTTGCG CTGGAACCCG 100
<210> 5845
<211> 100
<212> DNA
<213> Escherichia coli K-12 MG1655
<220> ycbm b0935
<222> 622-721
<223> ycbm b0935 U00096 complement(993264..994100)
<313> 5845: 671-690
<400> 5845
TGGCTGACGC TGATTGTTGC CGAAACCATT TCTGCCAATT CAGGCATTGG 50
TTATCTGGCG ATGAATGCGC GGGAGTTTTT GCAAACGGAC GTGGTGGTAG 100
<210> 5846
<211> 100
<212> DNA
<213> Escherichia coli K-12 MG1655
<220> b2331 b2331
<222> 452-551
<223> b2331 b2331 U00096 2446626..2447177
<313> 5846: 470-489
<400> 5846
ATGTGATGGC CTTTCATCAG GCACCAAAAG AGTATGGCGG TGATGCGGCG 50
TTGTTGGTGT TGATTGAAGT CGAAGAGTGG CTGCCGCCGG AGTTGCCCTG 100
<210> 5847
<211> 100
<212> DNA
<213> Escherichia coli K-12 MG1655
<220> b2331 b2331
<222> 346-445
<223> b2331 b2331 U00096 2446626..2447177
<313> 5847: 412-431
<400> 5847
ATTGCCGCCT GCCGCCGTGA ACATGTGTTT TGCGCCTGCG TGATGCATGG 50
GCACGGGAAG CATATTTTGA AGCAACAAAC ACCGCTGTGG CTGGCACAAC 100
<210> 5848
<211> 100
<212> DNA
<213> Escherichia coli K-12 MG1655
<220> b2331 b2331

<222> 301-400
<223> b2331 b2331 U00096 2446626..2447177
<313> 5848: 334-353
<400> 5848
CACGGTCTGA CGCAGCTGCA GGCCAAGCAG GAACTGGGGG CGTTGATTGC 50
CGCCTGCCGC CGTGAACATG TGTTTTGCGC CTGCGTGATG CATGGGCACG 100
<210> 5849
<211> 100
<212> DNA
<213> Escherichia coli K-12 MG1655
<220> ycbn b0936
<222> 1047-1146
<223> ycbn b0936 U00096 complement(994066..995211)
<313> 5849: 1094-1113
<400> 5849
GGATGTCGCC ATCCCGGAAA TTCCCCAGCC GCAGCCGCTG AATCCGCAAG 50
GCGAAGCGGT GGCGAATGAT TTTATCCCCC GTAAAGTCGC GCAAAGCTAA 100
<210> 5850
<211> 100
<212> DNA
<213> Escherichia coli K-12 MG1655
<220> ycbn b0936
<222> 1029-1128
<223> ycbn b0936 U00096 complement(994066..995211)
<313> 5850: 1057-1076
<400> 5850
GTTGCTGTTC CCGCTTCTGG ATGTCGCCAT CCCGGAAATT CCCCAGCCGC 50
AGCCGCTGAA TCCGCAAGGC GAAGCGGTGG CGAATGATTT TATCCCCCGT 100
<210> 5851
<211> 100
<212> DNA
<213> Escherichia coli K-12 MG1655
<220> ycbn b0936
<222> 965-1064
<223> ycbn b0936 U00096 complement(994066..995211)
<313> 5851: 1030-1049
<400> 5851
TTGGCATCGA CAGTTTTGTG CTTTCGGGCT ATCCGCATCT GGAAGAAGCG 50
TATCGGGTTG GCGAGTTGCT GTTCCCGCTT CTGGATGTCG CCATCCCGGA 100
<210> 5852
<211> 100
<212> DNA
<213> Escherichia coli K-12 MG1655
<220> b2332 b2332
<222> 672-771
<223> b2332 b2332 U00096 complement(2447248..2448084)
<313> 5852: 726-745
<400> 5852
AACTTTTACC GCGTCGGGAT GTGACAAGGC GGAAGTCTCG GTCACGCCAA 50
ATCGACACCC TATAACGGAC AAACAGTTAC ACGATATGGT CGTGCGTGTT 100
<210> 5853
<211> 100
<212> DNA
<213> Escherichia coli K-12 MG1655
<220> b2332 b2332
<222> 639-738
<223> b2332 b2332 U00096 complement(2447248..2448084)
<313> 5853: 675-694
<400> 5853
AGGTGGTTAC TGCCGCTGGG TTGCACAGAT GATAACTTTT ACCGCGTCGG 50
GATGTGACAA GGCGGAAGTC TCGGTCACGC CAAATCGACA CCCTATAACG 100
<210> 5854
<211> 100
<212> DNA
<213> Escherichia coli K-12 MG1655
<220> b2332 b2332
<222> 446-545
<223> b2332 b2332 U00096 complement(2447248..2448084)
<313> 5854: 468-487
<400> 5854
AGGGGCCTGA TAGTCACGAC ACGATTTGTC CATCCGTTTC TGTTCAACCT 50

TATGACGTTT CATGGAATGA GAATTATGTC AGTAAGTCAA AACTGCTCAC 100
<210> 5855
<211> 100
<212> DNA
<213> Escherichia coli K-12 MG1655
<220> ycbo b0937
<222> 903-1002
<223> ycbo b0937 U00096 complement(995208..996209)
<313> 5855: 977-996
<400> 5855
AACGGCAGAT CTGTTTTATG AAAATCGTCT GGTGCCGAAA AAAGTCGATA 50
TTCGCCAGCG CATCTGGCAG CCCACTCAAC TGGAAGGAAA ACAATTATGA 100
<210> 5856
<211> 100
<212> DNA
<213> Escherichia coli K-12 MG1655
<220> ycbo b0937
<222> 785-884
<223> ycbo b0937 U00096 complement(995208..996209)
<313> 5856: 843-862
<400> 5856
TCGCTTTACT GGCAAAAACG ATGGGCTTAC CGGCACCGGT GATTGCCTCT 50
TACTTAGATC ATCGCCCTCC TACCACCATC AAACCGGTTA ACGCCGAGGT 100
<210> 5857
<211> 100
<212> DNA
<213> Escherichia coli K-12 MG1655
<220> ycbo b0937
<222> 738-837
<223> ycbo b0937 U00096 complement(995208..996209)
<313> 5857: 791-810
<400> 5857
CTTTAGTGAG GCCGATGCGT TAACCCGCAG CCAGCGCGAG CAAAGCATCG 50
CTTTACTGGC AAAAACGATG GGCTTACCGG CACCGGTGAT TGCCTCTTAC 100
<210> 5858
<211> 100
<212> DNA
<213> Escherichia coli K-12 MG1655
<220> b2333 b2333
<222> 402-501
<223> b2333 b2333 U00096 complement(2448071..2448610)
<313> 5858: 456-475
<400> 5858
TACAAATGGG GCGACCTTTG CGCTAACCAA TGGCAATAAC GATATCAATT 50
TCAGAGCCTG GCTACAGGCG AAAAGCGGCC GTGATGTGAC GATCGGTGAA 100
<210> 5859
<211> 100
<212> DNA
<213> Escherichia coli K-12 MG1655
<220> b2333 b2333
<222> 368-467
<223> b2333 b2333 U00096 complement(2448071..2448610)
<313> 5859: 418-437
<400> 5859
AAAAAACCGA CGGAATGCAG GTTGCCATCA ACAATACAAA TGGGGCGACC 50
TTTGCGCTAA CCAATGGCAA TAACGATATC AATTTCAGAG CCTGGCTACA 100
<210> 5860
<211> 100
<212> DNA
<213> Escherichia coli K-12 MG1655
<220> b2333 b2333
<222> 329-428
<223> b2333 b2333 U00096 complement(2448071..2448610)
<313> 5860: 371-390
<400> 5860
ATACGTCATC AACCGCTCAG GGAGTGGGAA TTGGCATGGA AAAAACCGAC 50
GGAATGCAGG TTGCCATCAA CAATACAAAT GGGGCGACCT TTGCGCTAAC 100
<210> 5861
<211> 100
<212> DNA
<213> Escherichia coli K-12 MG1655

<220> b1604 b1604
<222> 846-945
<223> b1604 b1604 U00096 1676451..1677395
<313> 5861: 903-922
<400> 5861
GAAACGTGCC GCGAAGAAAG GTGCTAAGTA TTACCACATC ACCCGCCAGT 50
GGCAGGAACG TGGTAATAAC CTGACCGTCA GCGCAGATCT GTATAAATAA 100
<210> 5862
<211> 100
<212> DNA
<213> Escherichia coli K-12 MG1655
<220> b1604 b1604
<222> 816-915
<223> b1604 b1604 U00096 1676451..1677395
<313> 5862: 861-880
<400> 5862
CAACATGACC GAAGTCTCCT ATCAGGTTGC GAAACGTGCC GCGAAGAAAG 50
GTGCTAAGTA TTACCACATC ACCCGCCAGT GGCAGGAACG TGGTAATAAC 100
<210> 5863
<211> 100
<212> DNA
<213> Escherichia coli K-12 MG1655
<220> b1604 b1604
<222> 792-891
<223> b1604 b1604 U00096 1676451..1677395
<313> 5863: 831-850
<400> 5863
CATCAAATTC TCTGGCAACT ACGGCAACAT GACCGAAGTC TCCTATCAGG 50
TTGCGAAACG TGCCGCGAAG AAAGGTGCTA AGTATTACCA CATCACCCGC 100
<210> 5864
<211> 100
<212> DNA
<213> Escherichia coli K-12 MG1655
<220> ycbp b0938
<222> 477-576
<223> ycbp b0938 U00096 complement(996160..996735)
<313> 5864: 532-551
<400> 5864
CCGTCTTGAT ACCGCGCTAG AAACTTTCTG GCAGGCATTG CACCGCCGCG 50
ATGTTCAGGT TCCTGACCTT CTGTCTCTGC GAGGTAATGC CCATGCGTAA 100
<210> 5865
<211> 100
<212> DNA
<213> Escherichia coli K-12 MG1655
<220> ycbp b0938
<222> 444-543
<223> ycbp b0938 U00096 complement(996160..996735)
<313> 5865: 482-501
<400> 5865
TCACAGACCC CAGTTCACGC CAAATCTGCA AACCCGTCTT GATACCGCGC 50
TAGAAACTTT CTGGCAGGCA TTGCACCGCC GCGATGTTCA GGTTCCTGAC 100
<210> 5866
<211> 100
<212> DNA
<213> Escherichia coli K-12 MG1655
<220> ycbp b0938
<222> 362-461
<223> ycbp b0938 U00096 complement(996160..996735)
<313> 5866: 411-430
<400> 5866
TTAAACCAGT TTTAAGCGCA CTGAAAGCTC AGGAGATCCT GCACGGCGTG 50
TTTGCCGATG ACTCACAAGT AATTGATTAC CATCACAGAC CCCAGTTCAC 100
<210> 5867
<211> 100
<212> DNA
<213> Escherichia coli K-12 MG1655
<220> b2334 b2334
<222> 390-489
<223> b2334 b2334 U00096 complement(2448607..2449095)
<313> 5867: 447-466
<400> 5867

AAAGCCGAAG CTGGAGGCCG TACCGGTAAA AGCGAGTGAT GCTGTGTTAA 50
GCGATACGAA TTTTTCGGCT TACGCAACAT TACGGGTGGA TTATCAATGA 100
<210> 5868
<211> 100
<212> DNA
<213> Escherichia coli K-12 MG1655
<220> b2334 b2334
<222> 333-432
<223> b2334 b2334 U00096 complement(2448607..2449095)
<313> 5868: 389-408
<400> 5868
TGGTCAGCGT TTTAAACTCA ATACTCCGCT TGCTATCAAC GCCACTGATT 50
TTACGCAAAA GCCGAAGCTG GAGGCCGTAC CGGTAAAAGC GAGTGATGCT 100
<210> 5869
<211> 100
<212> DNA
<213> Escherichia coli K-12 MG1655
<220> b2334 b2334
<222> 310-409
<223> b2334 b2334 U00096 complement(2448607..2449095)
<313> 5869: 332-351
<400> 5869
TTTGGTATCG AGCTTCAGCA CGATGGTCAG CGTTTTAAAC TCAATACTCC 50
GCTTGCTATC AACGCCACTG ATTTTACGCA AAAGCCGAAG CTGGAGGCCG 100
<210> 5870
<211> 100
<212> DNA
<213> Escherichia coli K-12 MG1655
<220> b1605 b1606
<222> 1284-1383
<223> b1605 b1606 U00096 1677581..1678963
<313> 5870: 1353-1372
<400> 5870
AACGCATACC CATGATAATG TACTGAACCG TCAGGAAATG GTGCTGATTG 50
GCATGCTGTT AATTGCCTCA GTTCCGGCGA CCTGGATGCT GGTGGGATAA 100
<210> 5871
<211> 100
<212> DNA
<213> Escherichia coli K-12 MG1655
<220> b1605 b1606
<222> 1240-1339
<223> b1605 b1606 U00096 1677581..1678963
<313> 5871: 1297-1316
<400> 5871
GTTCTGTATG CACCGGGGCT GCTGGTTTTC CTGTACGCGC GCAAAACGCA 50
TACCCATGAT AATGTACTGA ACCGTCAGGA AATGGTGCTG ATTGGCATGC 100
<210> 5872
<211> 100
<212> DNA
<213> Escherichia coli K-12 MG1655
<220> b1605 b1606
<222> 1199-1298
<223> b1605 b1606 U00096 1677581..1678963
<313> 5872: 1212-1231
<400> 5872
TATTGTACGC CTCTGGTCCA ATGCACTTGC TGTTATCCGT TGTTCTGTAT 50
GCACCGGGGC TGCTGGTTTT CCTGTACGCG CGCAAAACGC ATACCCATGA 100
<210> 5873
<211> 100
<212> DNA
<213> Escherichia coli K-12 MG1655
<220> ycbq b0939
<222> 448-547
<223> ycbq b0939 U00096 997082..997630
<313> 5873: 502-521
<400> 5873
GGAGATCAGA CGTTGACTTT TAAAGTTGAT TATATCGCTA CGGGGAAAGC 50
GACTTCAGGT AATGTGAATG CGGTAACAAA TTTCCATATT AACTATTATT 100
<210> 5874
<211> 100
<212> DNA

<213> Escherichia coli K-12 MG1655
<220> ycbq b0939
<222> 376-475
<223> ycbq b0939 U00096 997082..997630
<313> 5874: 411-430
<400> 5874
CAAATGTATA TGAATGATGG TACAACGGCC ATCACGCCAG ACACAGAAAC  50
CGGGAACATT TTGTTGCAGG ATGGAGATCA GACGTTGACT TTTAAAGTTG  100
<210> 5875
<211> 100
<212> DNA
<213> Escherichia coli K-12 MG1655
<220> ycbq b0939
<222> 321-420
<223> ycbq b0939 U00096 997082..997630
<313> 5875: 384-403
<400> 5875
GATTACAGCG TTTGCTAACC AGGCCTCATC TGATGCTGCT ACAAACGTGG  50
CCCTGCAAAT GTATATGAAT GATGGTACAA CGGCCATCAC GCCAGACACA  100
<210> 5876
<211> 100
<212> DNA
<213> Escherichia coli K-12 MG1655
<220> b2335 b2336
<222> 407-506
<223> b2335 b2336 U00096 complement(2449092..2449604)
<313> 5876: 441-460
<400> 5876
TCAATAACCA GCCACAGTTG AAGGCTGTAC CTGTGAAGGC AAGCGGGGCG  50
CAACTTGCTG CGGGCGAGTT TAACGCCAGT TTGACCATGG TGGTGGACTA  100
<210> 5877
<211> 100
<212> DNA
<213> Escherichia coli K-12 MG1655
<220> b2335 b2336
<222> 308-407
<223> b2335 b2336 U00096 complement(2449092..2449604)
<313> 5877: 343-362
<400> 5877
TACTCAGTAC CAATATCACC GGGCTGGGCA TTCGGATAGA AAATAGCGCC  50
GATAACTCAT TGTTTGCCGT CGGTGAAAAT AGCTGGACGC CATTTAATAT  100
<210> 5878
<211> 100
<212> DNA
<213> Escherichia coli K-12 MG1655
<220> b2335 b2336
<222> 256-355
<223> b2335 b2336 U00096 complement(2449092..2449604)
<313> 5878: 286-305
<400> 5878
GACTTGCGGA TGCAACTTAA GGGCAATACC TCAACGATTA ACGGCGAAAC  50
GGTACTCAGT ACCAATATCA CCGGGCTGGG CATTCGGATA GAAAATAGCG  100
<210> 5879
<211> 100
<212> DNA
<213> Escherichia coli K-12 MG1655
<220> yrda b3280
<222> 641-740
<223> yrda b3280 U00096 3426657..3427427
<313> 5879: 701-720
<400> 5879
ATCTCGGTAG CCCCGTCAAA CAGATCCGCC CGTTAAGTGA TGAAGAGAAG  50
GCTGGGTTAC GCTATTCCGC GAATAATTAC GTGAAATGGA AGGACGAATA  100
<210> 5880
<211> 100
<212> DNA
<213> Escherichia coli K-12 MG1655
<220> yrda b3280
<222> 624-723
<223> yrda b3280 U00096 3426657..3427427
<313> 5880: 644-663

<400> 5880
GGAGAGCGGA TATCTGTATC TCGGTAGCCC CGTCAAACAG ATCCGCCCGT 50
TAAGTGATGA AGAGAAGGCT GGGTTACGCT ATTCCGCGAA TAATTACGTG 100
<210> 5881
<211> 100
<212> DNA
<213> Escherichia coli K-12 MG1655
<220> yrda b3280
<222> 585-684
<223> yrda b3280 U00096 3426657..3427427
<313> 5881: 600-619
<400> 5881
GATTGGTGCG GGTAGTCTGG TCCCACAAAA TAAACGGCTG GAGAGCGGAT 50
ATCTGTATCT CGGTAGCCCC GTCAAACAGA TCCGCCCGTT AAGTGATGAA 100
<210> 5882
<211> 100
<212> DNA
<213> Escherichia coli K-12 MG1655
<220> ycbr b0940
<222> 603-702
<223> ycbr b0940 U00096 997713..998414
<313> 5882: 676-695
<400> 5882
CCAATTTTCT CTGCCAAAGG GGGCCAAAAA TAGCAGCGTA ACGTATCGAA 50
CCATCAATGA CTACGGGGCG GAAACGCCGC AACTCAACTG TAAATCGTAA 100
<210> 5883
<211> 100
<212> DNA
<213> Escherichia coli K-12 MG1655
<220> ycbr b0940
<222> 602-701
<223> ycbr b0940 U00096 997713..998414
<313> 5883: 628-647
<400> 5883
ACCAATTTTC TCTGCCAAAG GGGGCCAAAA ATAGCAGCGT AACGTATCGA 50
ACCATCAATG ACTACGGGGC GGAAACGCCG CAACTCAACT GTAAATCGTA 100
<210> 5884
<211> 100
<212> DNA
<213> Escherichia coli K-12 MG1655
<220> ycbr b0940
<222> 404-503
<223> ycbr b0940 U00096 997713..998414
<313> 5884: 465-484
<400> 5884
CACAAAGCGT CATCAAACTT TTCTGGCGAC CAAAAGGTTT AGCGTATCCC 50
TTAGGCGAGG CTCCGGCGAA ACTGCGTTGC ACTTCGTCAG CTGACATGGT 100
<210> 5885
<211> 100
<212> DNA
<213> Escherichia coli K-12 MG1655
<220> yrdb b3281
<222> 159-258
<223> yrdb b3281 U00096 complement(3427403..3427660)
<313> 5885: 200-219
<400> 5885
AGCGAGTTTT CGTCAGCATC GCTGGGACCT GGAAGAAGAA GCGGAAAACT 50
TAATTCAGGA ACAAAGTGAA GATGATCAAG GCTGGGTCTG GTTACCCTGA 100
<210> 5886
<211> 100
<212> DNA
<213> Escherichia coli K-12 MG1655
<220> yrdb b3281
<222> 107-206
<223> yrdb b3281 U00096 complement(3427403..3427660)
<313> 5886: 155-174
<400> 5886
CTGGCGAGAG TCTGGCGTAT CGCTTTACTG GAGATACGCC AGAACAGTGG 50
TTAGCGAGTT TTCGTCAGCA TCGCTGGGAC CTGGAAGAAG AAGCGGAAAA 100
<210> 5887
<211> 100

<212> DNA
<213> Escherichia coli K-12 MG1655
<220> yrdb b3281
<222> 79-178
<223> yrdb b3281 U00096 complement(3427403..3427660)
<313> 5887: 116-135
<400> 5887
AATGGTATGC AACTGACATG CGCGATCTCT GGCGAGAGTC TGGCGTATCG 50
CTTTACTGGA GATACGCCAG AACAGTGGTT AGCGAGTTTT CGTCAGCATC 100
<210> 5888
<211> 100
<212> DNA
<213> Escherichia coli K-12 MG1655
<220> ycbs ycbS
<222> 2502-2601
<223> ycbs ycbS U00096 998439..1001039
<313> 5888: 2573-2592
<400> 5888
CTGGGGCGAA GCTCCCGATC AAATTTGTCA TATCAATTAC GAGCTTACCG 50
AACAACAAAT TAACTCTGCG ATTACGCGAA TGGATGCCAT ATGCAGATAA 100
<210> 5889
<211> 100
<212> DNA
<213> Escherichia coli K-12 MG1655
<220> ycbs ycbS
<222> 2377-2476
<223> ycbs ycbS U00096 998439..1001039
<313> 5889: 2428-2447
<400> 5889
GGTATACCGC TGCGTTTTGG CGCGATAGCG ACGCTGGACG GCGTACAGGC 50
TAATAGCGGC ATAATTGATG ATGATGGCTC GCTCTATATG GCGGGTTTAC 100
<210> 5890
<211> 100
<212> DNA
<213> Escherichia coli K-12 MG1655
<220> ycbs ycbS
<222> 2320-2419
<223> ycbs ycbS U00096 998439..1001039
<313> 5890: 2354-2373
<400> 5890
CTGGCGGAGT TCAATGCCCA TGCGGGTGCA AGGGTATTAA TGAAAACATC 50
AAAGCAGGGT ATACCGCTGC GTTTTGGCGC GATAGCGACG CTGGACGGCG 100
<210> 5891
<211> 100
<212> DNA
<213> Escherichia coli K-12 MG1655
<220> b2337 b2338
<222> 798-897
<223> b2337 b2338 U00096 complement(2450376..2451272)
<313> 5891: 874-893
<400> 5891
CGCGCCGGTT GTTCCTGATG ACATAAAGCC TGTCATTCAG GAGCAGACGC 50
AACAGGTGAC ACCCACCGAT CCCCCTGTTT CTGTTTCAGC TAACCAATAA 100
<210> 5892
<211> 100
<212> DNA
<213> Escherichia coli K-12 MG1655
<220> b2337 b2338
<222> 767-866
<223> b2337 b2338 U00096 complement(2450376..2451272)
<313> 5892: 824-843
<400> 5892
TGTTACTGCC ATGCCAGCAT AAAGGCAACG TCGCGCCGGT TGTTCCTGAT 50
GACATAAAGC CTGTCATTCA GGAGCAGACG CAACAGGTGA CACCCACCGA 100
<210> 5893
<211> 100
<212> DNA
<213> Escherichia coli K-12 MG1655
<220> b2337 b2338
<222> 765-864
<223> b2337 b2338 U00096 complement(2450376..2451272)

<313> 5893: 794-813
<400> 5893
CTTGTTACTG CCATGCCAGC ATAAAGGCAA CGTCGCGCCG GTTGTTCCTG  50
ATGACATAAA GCCTGTCATT CAGGAGCAGA CGCAACAGGT GACACCCACC  100
<210> 5894
<211> 100
<212> DNA
<213> Escherichia coli K-12 MG1655
<220> yrdc b3283
<222> 474-573
<223> yrdc b3283 U00096 complement(3428480..3429052)
<313> 5894: 544-563
<400> 5894
TGGCGCGGCG TTCCCGGTTG TGCCTGGTGA AACGGGGGGG CGTTTAAATC  50
CTTCAGAAAT CCGCGATGCC CTGACGGGTG AACTGTTTCG ACAGGGGTAA  100
<210> 5895
<211> 100
<212> DNA
<213> Escherichia coli K-12 MG1655
<220> yrdc b3283
<222> 474-573
<223> yrdc b3283 U00096 complement(3428480..3429052)
<313> 5895: 504-523
<400> 5895
TGGCGCGGCG TTCCCGGTTG TGCCTGGTGA AACGGGGGGG CGTTTAAATC  50
CTTCAGAAAT CCGCGATGCC CTGACGGGTG AACTGTTTCG ACAGGGGTAA  100
<210> 5896
<211> 100
<212> DNA
<213> Escherichia coli K-12 MG1655
<220> yrdc b3283
<222> 420-519
<223> yrdc b3283 U00096 complement(3428480..3429052)
<313> 5896: 439-458
<400> 5896
CAACTTGAGT GGATTGCCAC CTTGTCGAAC AGTAGACGAA GTTCGCGCAC  50
AATTTGGCGC GGCGTTCCCG GTTGTGCCTG GTGAAACGGG GGGGCGTTTA  100
<210> 5897
<211> 100
<212> DNA
<213> Escherichia coli K-12 MG1655
<220> yrdd b3284
<222> 392-491
<223> yrdd b3284 U00096 complement(3429057..3429566)
<313> 5897: 437-456
<400> 5897
CCATAGCTGG AGAATGCCCT GAGTGTCATT ATCCGCTACT CATCGAAAAG  50
AAAACCGCGC AGGGTGTAAA ACACTTTTGT GCCAGTAAAC AATGTGGAAA  100
<210> 5898
<211> 100
<212> DNA
<213> Escherichia coli K-12 MG1655
<220> yrdd b3284
<222> 362-461
<223> yrdd b3284 U00096 complement(3429057..3429566)
<313> 5898: 385-404
<400> 5898
CGGAGTGTCA ATTTGCCATT AACTTCAAAC CCATAGCTGG AGAATGCCCT  50
GAGTGTCATT ATCCGCTACT CATCGAAAAG AAAACCGCGC AGGGTGTAAA  100
<210> 5899
<211> 100
<212> DNA
<213> Escherichia coli K-12 MG1655
<220> yrdd b3284
<222> 278-377
<223> yrdd b3284 U00096 complement(3429057..3429566)
<313> 5899: 320-339
<400> 5899
TTACATGCCC CCAATGTCGG ACGGGCCATC TGGTCCAGCG CCGCTCCCGT  50
TATGGCAAAA CATTTCACTC TTGTGATCGC TACCCGGAGT GTCAATTTGC  100
<210> 5900

<211> 100
<212> DNA
<213> Escherichia coli K-12 MG1655
<220> b2339 b2339
<222> 402-501
<223> b2339 b2339 U00096 complement(2453103..2453666)
<313> 5900: 456-475
<400> 5900
AAGCGGCATG GGTATTGACC AGAAAATCGT GAAAGATACT GCCACTAATA 50
AAGGCAAAGC GAAGCAGACT CTGAACTTTA AAGCCTGGCT GGTCGGCGCA 100
<210> 5901
<211> 100
<212> DNA
<213> Escherichia coli K-12 MG1655
<220> b2339 b2339
<222> 380-479
<223> b2339 b2339 U00096 complement(2453103..2453666)
<313> 5901: 405-424
<400> 5901
ACTCTCTGGG TACCTCTTAC AAAAGCGGCA TGGGTATTGA CCAGAAAATC 50
GTGAAAGATA CTGCCACTAA TAAAGGCAAA GCGAAGCAGA CTCTGAACTT 100
<210> 5902
<211> 100
<212> DNA
<213> Escherichia coli K-12 MG1655
<220> b2339 b2339
<222> 208-307
<223> b2339 b2339 U00096 complement(2453103..2453666)
<313> 5902: 237-256
<400> 5902
ACCACGCCGA AAAATTTCCA GATTCGCCTG CAAGATTGCG TATTTGATAC 50
TCAGGAAACA ATGACCACTA CCTTTACTGG TACCGTTTCT TCTGCAAATA 100
<210> 5903
<211> 100
<212> DNA
<213> Escherichia coli K-12 MG1655
<220> ycbw ycbW
<222> 474-573
<223> ycbw ycbW U00096 1005139..1005717
<313> 5903: 507-526
<400> 5903
TATTGCGGGT CGCGCGATGC AGTTGGGCGA CGCCATTAAA ATCATGAACG 50
ACAGGCTGAA ACCGCAGGTT AATGTTGACA GCTTCAGCCT CGAACAGGCA 100
<210> 5904
<211> 100
<212> DNA
<213> Escherichia coli K-12 MG1655
<220> ycbw ycbW
<222> 399-498
<223> ycbw ycbW U00096 1005139..1005717
<313> 5904: 419-438
<400> 5904
CGAGCAGGTC AATTTGCTGG TCGTTGAATC TGGCGAAAAT GCCGCACTAT 50
GCCTGCTGGC ACAACCCTGC GTTGTTATTG CGGGTCGCGC GATGCAGTTG 100
<210> 5905
<211> 100
<212> DNA
<213> Escherichia coli K-12 MG1655
<220> ycbw ycbW
<222> 241-340
<223> ycbw ycbW U00096 1005139..1005717
<313> 5905: 289-308
<400> 5905
TTATCTAAAG AACAAAAAGC CGAACTGGTG CTGAATGCAC TGGTAGCAAT 50
TCGCTATCTC AAACCGCAAA TGCCGAAAAG CTGGCATTTT GTTTCCCATG 100
<210> 5906
<211> 100
<212> DNA
<213> Escherichia coli K-12 MG1655
<220> ycby b0949
<222> 2010-2109

<223> ycby b0949 U00096 1007067..1009175
<313> 5906: 2084-2103
<400> 5906
ACTGGGACTG AAAGCACAAG AAATTACGCA AAAAACGCTC TCCCAGGATT 50
TCGCCCGTAA CCGCCAGATC CACAACTGCT GGCTGATTAC CGCAGCCTGA 100
<210> 5907
<211> 100
<212> DNA
<213> Escherichia coli K-12 MG1655
<220> ycby b0949
<222> 2010-2109
<223> ycby b0949 U00096 1007067..1009175
<313> 5907: 2045-2064
<400> 5907
ACTGGGACTG AAAGCACAAG AAATTACGCA AAAAACGCTC TCCCAGGATT 50
TCGCCCGTAA CCGCCAGATC CACAACTGCT GGCTGATTAC CGCAGCCTGA 100
<210> 5908
<211> 100
<212> DNA
<213> Escherichia coli K-12 MG1655
<220> ycby b0949
<222> 1961-2060
<223> ycby b0949 U00096 1007067..1009175
<313> 5908: 1992-2011
<400> 5908
TCTCGAACAA CAAACGTGGC TTCCGTATGG ATCTCGACGG CCTGGCAAAA 50
CTGGGACTGA AAGCACAAGA AATTACGCAA AAAACGCTCT CCCAGGATTT 100
<210> 5909
<211> 100
<212> DNA
<213> Escherichia coli K-12 MG1655
<220> dsba b3861
<222> 491-590
<223> dsba b3861 U00096 4040999..4041625
<313> 5909: 541-560
<400> 5909
TGCAATTGCG TGGCGTTCCG GCGATGTTTG TTAACGGTAA ATATCAGCTG 50
AATCCGCAGG GTATGGATAC CAGCAATATG GATGTTTTTG TTCAGCAGTA 100
<210> 5910
<211> 100
<212> DNA
<213> Escherichia coli K-12 MG1655
<220> dsba b3861
<222> 422-521
<223> dsba b3861 U00096 4040999..4041625
<313> 5910: 448-467
<400> 5910
ACGACGCGGC GTGGAACAGC TTCGTGGTGA AATCTCTGGT CGCTCAGCAG 50
GAAAAAGCTG CAGCTGACGT GCAATTGCGT GGCGTTCCGG CGATGTTTGT 100
<210> 5911
<211> 100
<212> DNA
<213> Escherichia coli K-12 MG1655
<220> dsba b3861
<222> 378-477
<223> dsba b3861 U00096 4040999..4041625
<313> 5911: 402-421
<400> 5911
TATCCGCGAT GTATTTATCA ACGCAGGTAT TAAAGGTGAA GAGTACGACG 50
CGGCGTGGAA CAGCTTCGTG GTGAAATCTC TGGTCGCTCA GCAGGAAAAA 100
<210> 5912
<211> 100
<212> DNA
<213> Escherichia coli K-12 MG1655
<220> dsbb b1186
<222> 432-531
<223> dsbb b1186 U00096 complement(1231723..1232253)
<313> 5912: 510-529
<400> 5912
GTGGCTGCTC GGTATTTTTA TCGCTTACCT GATTGTCGCA GTGCTGGTGG 50
TGATTTCCCA GCCGTTTAAA GCGAAAAAAC GTGATCTGTT CGGTCGCTAA 100

<210> 5913
<211> 100
<212> DNA
<213> Escherichia coli K-12 MG1655
<220> dsbb b1186
<222> 317-416
<223> dsbb b1186 U00096 complement(1231723..1232253)
<313> 5913: 342-361
<400> 5913
TTATGGTTCG TTTCCCGGAA TGGCTGCCGC TGGATAAGTG GGTGCCGCAA 50
GTGTTTGTCG CCTCTGGCGA TTGCGCCGAG CGTCAGTGGG ATTTTTTAGG 100
<210> 5914
<211> 100
<212> DNA
<213> Escherichia coli K-12 MG1655
<220> dsbb b1186
<222> 264-363
<223> dsbb b1186 U00096 complement(1231723..1232253)
<313> 5914: 311-330
<400> 5914
TTACGAGCAC ACCATGCTTC AGCTCTATCC TTCGCCGTTT GCCACCTGTG 50
ATTTTATGGT TCGTTTCCCG GAATGGCTGC CGCTGGATAA GTGGGTGCCG 100
<210> 5915
<211> 100
<212> DNA
<213> Escherichia coli K-12 MG1655
<220> dsbc b2894
<222> 612-711
<223> dsbc b2894 U00096 complement(3036132..3036842)
<313> 5915: 689-708
<400> 5915
AGTTGTGCTG AGCAATGGCA CACTTGTTCC GGGTTACCAG CCGCCGAAAG 50
AGATGAAAGA ATTCCTCGAC GAACACCAAA AAATGACCAG CGGTAAATAA 100
<210> 5916
<211> 100
<212> DNA
<213> Escherichia coli K-12 MG1655
<220> dsbc b2894
<222> 612-711
<223> dsbc b2894 U00096 complement(3036132..3036842)
<313> 5916: 637-656
<400> 5916
AGTTGTGCTG AGCAATGGCA CACTTGTTCC GGGTTACCAG CCGCCGAAAG 50
AGATGAAAGA ATTCCTCGAC GAACACCAAA AAATGACCAG CGGTAAATAA 100
<210> 5917
<211> 100
<212> DNA
<213> Escherichia coli K-12 MG1655
<220> dsbc b2894
<222> 539-638
<223> dsbc b2894 U00096 complement(3036132..3036842)
<313> 5917: 554-573
<400> 5917
CAGCCAGTTG CGACGTGGAT ATTGCCGACC ATTACGCACT TGGCGTCCAG 50
CTTGGCGTTA GCGGTACTCC GGCAGTTGTG CTGAGCAATG GCACACTTGT 100
<210> 5918
<211> 100
<212> DNA
<213> Escherichia coli K-12 MG1655
<220> dsbd b4137
<222> 1571-1670
<223> dsbd b4137 U00096 complement(4360923..4362620)
<313> 5918: 1615-1634
<400> 5918
TTAATGTCCT TGGCCTACCG ACAATTCTCT TTTTTGACGG ACAAGGCCAG 50
GAGCATCCAC AAGCACGCGT CACGGGCTTT ATGGATGCTG AAACCTTCAG 100
<210> 5919
<211> 100
<212> DNA
<213> Escherichia coli K-12 MG1655
<220> dsbd b4137

<222> 1531-1630
<223> dsbd b4137 U00096 complement(4360923..4362620)
<313> 5919: 1567-1586
<400> 5919
ACGGCCAACG ACGCACAAGA TGTGGCGCTG TTAAAGCATC TTAATGTCCT  50
TGGCCTACCG ACAATTCTCT TTTTTGACGG ACAAGGCCAG GAGCATCCAC  100
<210> 5920
<211> 100
<212> DNA
<213> Escherichia coli K-12 MG1655
<220> dsbd b4137
<222> 1468-1567
<223> dsbd b4137 U00096 complement(4360923..4362620)
<313> 5920: 1522-1541
<400> 5920
ACCTTCAGCG ACCCGCAGGT GCAAAAAGCG TTAGCAGACA CGGTCTTACT  50
TCAGGCCAAC GTCACGGCCA ACGACGCACA AGATGTGGCG CTGTTAAAGC  100
<210> 5921
<211> 100
<212> DNA
<213> Escherichia coli K-12 MG1655
<220> dsbe b2196
<222> 459-558
<223> dsbe b2196 U00096 complement(2290427..2290984)
<313> 5921: 520-539
<400> 5921
CATCATTCGC TATCGCCATG CGGGCGATCT GAATCCTCGC GTCTGGGAAG  50
AAGAGATCAA GCCGCTGTGG GAGAAATACA GTAAGGAGGC CGCACAATGA  100
<210> 5922
<211> 100
<212> DNA
<213> Escherichia coli K-12 MG1655
<220> dsbe b2196
<222> 459-558
<223> dsbe b2196 U00096 complement(2290427..2290984)
<313> 5922: 493-512
<400> 5922
CATCATTCGC TATCGCCATG CGGGCGATCT GAATCCTCGC GTCTGGGAAG  50
AAGAGATCAA GCCGCTGTGG GAGAAATACA GTAAGGAGGC CGCACAATGA  100
<210> 5923
<211> 100
<212> DNA
<213> Escherichia coli K-12 MG1655
<220> dsbe b2196
<222> 402-501
<223> dsbe b2196 U00096 complement(2290427..2290984)
<313> 5923: 451-470
<400> 5923
AGGGCTGGAT CTCGGTGTCT ATGGCGCGCC AGAAACGTTT CTTATTGACG  50
GCAACGGCAT CATTCGCTAT CGCCATGCGG GCGATCTGAA TCCTCGCGTC  100
<210> 5924
<211> 100
<212> DNA
<213> Escherichia coli K-12 MG1655
<220> ycca b0971
<222> 561-660
<223> ycca b0971 U00096 complement(1029982..1030641)
<313> 5924: 621-640
<400> 5924
TGAGACGAAC TATATTCGTG CCACGGTTAG CCTGTATGTT TCGCTGTACA  50
ACATCTTCGT CAGCCTGCTG AGCATTCTGG GCTTCGCTAG CCGCGATTAA  100
<210> 5925
<211> 100
<212> DNA
<213> Escherichia coli K-12 MG1655
<220> ycca b0971
<222> 512-611
<223> ycca b0971 U00096 complement(1029982..1030641)
<313> 5925: 564-583
<400> 5925
TCTCCTCTGG CGCTATCTTG TTTGAAACCA GCAACATCAT TCATGGCGGT  50

GAGACGAACT ATATTCGTGC CACGGTTAGC CTGTATGTTT CGCTGTACAA 100
<210> 5926
<211> 100
<212> DNA
<213> Escherichia coli K-12 MG1655
<220> ycca b0971
<222> 460-559
<223> ycca b0971 U00096 complement(1029982..1030641)
<313> 5926: 486-505
<400> 5926
TTCCTGCAGC TGCCTGCTCT GCATCTGGCG ATCAGCGCGG TCTTCATTCT 50
GATCTCCTCT GGCGCTATCT TGTTTGAAAC CAGCAACATC ATTCATGGCG 100
<210> 5927
<211> 100
<212> DNA
<213> Escherichia coli K-12 MG1655
<220> dsbg b0605
<222> 708-807
<223> dsbg b0605 U00096 complement(637050..637856)
<313> 5927: 775-794
<400> 5927
CACGCCGGCT ATCTATTACA TGAGTAAGGA AAATACGCTA CAACAGGCCG 50
TGGGGTTGCC CGATCAGAAA ACGCTTAATA TCATTATGGG GAATAAATAA 100
<210> 5928
<211> 100
<212> DNA
<213> Escherichia coli K-12 MG1655
<220> dsbg b0605
<222> 634-733
<223> dsbg b0605 U00096 complement(637050..637856)
<313> 5928: 687-706
<400> 5928
GCAAACGTAA GTACAGAGCA AATGAAAGTG TTAAGTGACA ATGAGAAACT 50
GATGGACGAT CTGGGGGCAA ATGTCACGCC GGCTATCTAT TACATGAGTA 100
<210> 5929
<211> 100
<212> DNA
<213> Escherichia coli K-12 MG1655
<220> dsbg b0605
<222> 608-707
<223> dsbg b0605 U00096 complement(637050..637856)
<313> 5929: 638-657
<400> 5929
GTGGCAAGCT TAAGCTAAAC GTGCCTGCAA ACGTAAGTAC AGAGCAAATG 50
AAAGTGTTAA GTGACAATGA GAAACTGATG GACGATCTGG GGGCAAATGT 100
<210> 5930
<211> 100
<212> DNA
<213> Escherichia coli K-12 MG1655
<220> yccc b0982
<222> 2082-2181
<223> yccc b0982 U00096 complement(1041253..1043433)
<313> 5930: 2130-2149
<400> 5930
TATTAAAGGC GCTATCCTCA ATGGTGTGAT TAAACGCGCC AGCACCGCTT 50
ACAGTTACGG CTATAACTAT TACGGTTATA GTTACTCCGA GAAAGAGTAA 100
<210> 5931
<211> 100
<212> DNA
<213> Escherichia coli K-12 MG1655
<220> yccc b0982
<222> 2051-2150
<223> yccc b0982 U00096 complement(1041253..1043433)
<313> 5931: 2091-2110
<400> 5931
CAATGCAGCG TCTGGAACAG GCAGGCGTCA ATATTAAAGG CGCTATCCTC 50
AATGGTGTGA TTAAACGCGC CAGCACCGCT TACAGTTACG GCTATAACTA 100
<210> 5932
<211> 100
<212> DNA
<213> Escherichia coli K-12 MG1655

<220> yccc b0982
<222> 1858-1957
<223> yccc b0982 U00096 complement(1041253..1043433)
<313> 5932: 1895-1914
<400> 5932
AACCCGTCTG AACTGCTGAT GCGCGATCGG ATGCGTCAAT TACTGGAATG  50
GGCGAACGAC CATTACGATC TGGTGATTGT CGATACGCCG CCGATGCTGG  100
<210> 5933
<211> 100
<212> DNA
<213> Escherichia coli K-12 MG1655
<220> yccd b1000
<222> 207-306
<223> yccd b1000 U00096 complement(1061773..1062078)
<313> 5933: 270-289
<400> 5933
GATCGCGGTG GCGCTGACGT TAATGGATGA TATTGCGCAC CTGAAGCAGG  50
AAAACCGCCT GCTGCGCCAG CGGCTTTCCC GGTTTGTAGC TCATCCGTGA  100
<210> 5934
<211> 100
<212> DNA
<213> Escherichia coli K-12 MG1655
<220> yccd b1000
<222> 67-166
<223> yccd b1000 U00096 complement(1061773..1062078)
<313> 5934: 92-111
<400> 5934
GAGTTGAATG AAATTGTCGG TTTGGGGGTG GTTGAACCGC GTGAGATCCA  50
GGAAACAACC TGGGTATTTG ACGACCATGC CGCCATTGTG GTGCAACGCG  100
<210> 5935
<211> 100
<212> DNA
<213> Escherichia coli K-12 MG1655
<220> yccd b1000
<222> 26-125
<223> yccd b1000 U00096 complement(1061773..1062078)
<313> 5935: 53-72
<400> 5935
CTATTACCGA ATTTTGCCTG CATACCGGCA TCTCTGAAGA GGAGTTGAAT  50
GAAATTGTCG GTTTGGGGGT GGTTGAACCG CGTGAGATCC AGGAAACAAC  100
<210> 5936
<211> 100
<212> DNA
<213> Escherichia coli K-12 MG1655
<220> ycce b1002
<222> 1136-1235
<223> ycce b1002 U00096 1063259..1064515
<313> 5936: 1169-1188
<400> 5936
TAATGAACAC TGGCAATCAT TATGACGTTT ACCTCCCTCC TGAACTTTTT  50
GCTCAGGCTT ACAAGTTAAA CAATAAGGAA ATGAATGCGC AACTCGACTA  100
<210> 5937
<211> 100
<212> DNA
<213> Escherichia coli K-12 MG1655
<220> ycce b1002
<222> 963-1062
<223> ycce b1002 U00096 1063259..1064515
<313> 5937: 996-1015
<400> 5937
GAACTTGCTT GCTTTAGATA CCGACACGTC GGATAGGATT AAAGCGGTGA  50
AGAATTTTCT TGTTGTTCTT TCAGATAGCA TTTGCTCATT ATTTATTGTT  100
<210> 5938
<211> 100
<212> DNA
<213> Escherichia coli K-12 MG1655
<220> ycce b1002
<222> 792-891
<223> ycce b1002 U00096 1063259..1064515
<313> 5938: 833-852
<400> 5938

AGATGTAAAG ACGTTATTTG GCGTTGATCT ACAATCTGAC AACCAAATTC 50
AGGGGCAGAG TAGTCTTATG TCATGGAGCT TTCTGTTTTT TAAAAAACAA 100
<210> 5939
<211> 100
<212> DNA
<213> Escherichia coli K-12 MG1655
<220> yccf b0962
<222> 319-418
<223> yccf b0962 U00096 complement(1023125..1023571)
<313> 5939: 348-367
<400> 5939
GGCATTCCTG TCGGCATTGC GAACTTTAAA ATTGCCGCTA TTGCACTATG 50
GCCGGTTGGT CGTCGCGTGG TATCGGTAGA AACAGCGCAA GCTGCGCGTG 100
<210> 5940
<211> 100
<212> DNA
<213> Escherichia coli K-12 MG1655
<220> yccf b0962
<222> 244-343
<223> yccf b0962 U00096 complement(1023125..1023571)
<313> 5940: 308-327
<400> 5940
TGGCTGATTT TCTTTGGCTG GTGGTTATGC CTGATGCACA TTGCAACGGG 50
CATCGCACAA TGTATTTCAA TCATTGGCAT TCCTGTCGGC ATTGCGAACT 100
<210> 5941
<211> 100
<212> DNA
<213> Escherichia coli K-12 MG1655
<220> yccf b0962
<222> 223-322
<223> yccf b0962 U00096 complement(1023125..1023571)
<313> 5941: 267-286
<400> 5941
GGTACGGTAT TGAATATTTT CTGGCTGATT TTCTTTGGCT GGTGGTTATG 50
CCTGATGCAC ATTGCAACGG GCATCGCACA ATGTATTTCA ATCATTGGCA 100
<210> 5942
<211> 100
<212> DNA
<213> Escherichia coli K-12 MG1655
<220> yccj b1004
<222> 129-228
<223> yccj b1004 U00096 complement(1066087..1066314)
<313> 5942: 204-223
<400> 5942
GGAAGAAGGT AGTGATGAAG TGTTAGTCAA AGCCTTTGCT AAGACCGATA 50
AAGATTCGCT TTTTTGGGGC GAACAGACCA TCGAACGTAA AAACGTTTAA 100
<210> 5943
<211> 100
<212> DNA
<213> Escherichia coli K-12 MG1655
<220> yccj b1004
<222> 126-225
<223> yccj b1004 U00096 complement(1066087..1066314)
<313> 5943: 160-179
<400> 5943
TTGGGAAGAA GGTAGTGATG AAGTGTTAGT CAAAGCCTTT GCTAAGACCG 50
ATAAAGATTC GCTTTTTTGG GGCGAACAGA CCATCGAACG TAAAAACGTT 100
<210> 5944
<211> 100
<212> DNA
<213> Escherichia coli K-12 MG1655
<220> yccj b1004
<222> 129-228
<223> yccj b1004 U00096 complement(1066087..1066314)
<313> 5944: 156-175
<400> 5944
GGAAGAAGGT AGTGATGAAG TGTTAGTCAA AGCCTTTGCT AAGACCGATA 50
AAGATTCGCT TTTTTGGGGC GAACAGACCA TCGAACGTAA AAACGTTTAA 100
<210> 5945
<211> 100
<212> DNA

<213> Escherichia coli K-12 MG1655
<220> ycck b0970
<222> 288-387
<223> ycck b0970 U00096 complement(1029562..1029948)
<313> 5945: 343-362
<400> 5945
AAAAGGTAAC AGCCGCTATT TATACCGACT GTTTCCGAAA GGTCCGGCTA 50
AGCAAGCCAC CAAAATTGCT GGCCTGCCAA AACCGGTAAA ATGTATTTAA 100
<210> 5946
<211> 100
<212> DNA
<213> Escherichia coli K-12 MG1655
<220> ycck b0970
<222> 251-350
<223> ycck b0970 U00096 complement(1029562..1029948)
<313> 5946: 304-323
<400> 5946
TGCTGGTAAA AGCGATGGCG AATAAATTTG GCGAAGAAAA AGGTAACAGC 50
CGCTATTTAT ACCGACTGTT TCCGAAAGGT CCGGCTAAGC AAGCCACCAA 100
<210> 5947
<211> 100
<212> DNA
<213> Escherichia coli K-12 MG1655
<220> ycck b0970
<222> 218-317
<223> ycck b0970 U00096 complement(1029562..1029948)
<313> 5947: 269-288
<400> 5947
ATCTGGAATT CAATACTTCT CCGGCGATTC GTATGCTGGT AAAAGCGATG 50
GCGAATAAAT TTGGCGAAGA AAAAGGTAAC AGCCGCTATT TATACCGACT 100
<210> 5948
<211> 100
<212> DNA
<213> Escherichia coli K-12 MG1655
<220> seqa b0688
<222> 447-546
<223> seqa b0688 U00096 712210..712755
<313> 5948: 521-540
<400> 5948
CAACACCAAC ACCGGCCGTA AATGCAGCAT GATCGAACAC ATCATGCAGT 50
CGATGCAATT CCCGGCGGAA TTGATTGAGA AGGTTTGCGG AACTATCTAA 100
<210> 5949
<211> 100
<212> DNA
<213> Escherichia coli K-12 MG1655
<220> seqa b0688
<222> 359-458
<223> seqa b0688 U00096 712210..712755
<313> 5949: 393-412
<400> 5949
ACTTTGCGGC AGATGAACAA ACGCTGCTGA AAAATGGTAA TCAGACCAAG 50
CCGAAACATG TGCCAGGCAC GCCGTATTGG GTGATCACCA ACACCAACAC 100
<210> 5950
<211> 100
<212> DNA
<213> Escherichia coli K-12 MG1655
<220> seqa b0688
<222> 193-292
<223> seqa b0688 U00096 712210..712755
<313> 5950: 243-262
<400> 5950
ATTAAAGACA AGGTTCGCGC AATGCGTGAA CTTCTGCTTT CGGATGAATA 50
CGCAGAGCAA AAGCGAGCGG TCAATCGCTT TATGCTGCTG TTGTCTACAC 100
<210> 5951
<211> 100
<212> DNA
<213> Escherichia coli K-12 MG1655
<220> b2340 b2340
<222> 387-486
<223> b2340 b2340 U00096 complement(2454347..2454832)
<313> 5951: 450-469

<400> 5951
CACCTCGGCC ATTGCCAGCG TAACGCTTGA TGAAAGCGGA AACGGCACAT 50
TCAACTGGCA GATGAGCCCG TGTAATCTGA AGATGGCAAA AGCTATCTGA 100
<210> 5952
<211> 100
<212> DNA
<213> Escherichia coli K-12 MG1655
<220> b2340 b2340
<222> 383-482
<223> b2340 b2340 U00096 complement(2454347..2454832)
<313> 5952: 407-426
<400> 5952
TTACCACCTC GGCCATTGCC AGCGTAACGC TTGATGAAAG CGGAAACGGC 50
ACATTCAACT GGCAGATGAG CCCGTGTAAT CTGAAGATGG CAAAAGCTAT 100
<210> 5953
<211> 100
<212> DNA
<213> Escherichia coli K-12 MG1655
<220> b2340 b2340
<222> 256-355
<223> b2340 b2340 U00096 complement(2454347..2454832)
<313> 5953: 282-301
<400> 5953
GGTCTGGTCA GTGCCTATTT GCAGGCGTTG ACCAATGAAG GTGTTGCTTC 50
GGTACTGGTG ATCTCTCACT TACCGTTAGT CGGGTATCTG GTTGCCGAGT 100
<210> 5954
<211> 100
<212> DNA
<213> Escherichia coli K-12 MG1655
<220> yccm b0993
<222> 961-1060
<223> yccm b0993 U00096 complement(1051512..1052585)
<313> 5954: 997-1016
<400> 5954
GGGATTATGT TTGCGGTGAT TGGTTACGCA ATGTATGCGG GAGTATGGCA 50
AAGCCCGGTG CCGGAGGAAT TGTACCGACG CTTAATTCCA CAAGCGCCAA 100
<210> 5955
<211> 100
<212> DNA
<213> Escherichia coli K-12 MG1655
<220> yccm b0993
<222> 898-997
<223> yccm b0993 U00096 complement(1051512..1052585)
<313> 5955: 952-971
<400> 5955
AAACCTGCGG CAAATAAAAA AGCCTTTGCG TTGTCTGGCT GGTTAATGAC 50
GCTACTGGTT CTGGGGATTA TGTTTGCGGT GATTGGTTAC GCAATGTATG 100
<210> 5956
<211> 100
<212> DNA
<213> Escherichia coli K-12 MG1655
<220> yccm b0993
<222> 873-972
<223> yccm b0993 U00096 complement(1051512..1052585)
<313> 5956: 914-933
<400> 5956
CTCAACATTG ACCTTTTCAC TGCAAAAACC TGCGGCAAAT AAAAAAGCCT 50
TTGCGTTGTC TGGCTGGTTA ATGACGCTAC TGGTTCTGGG GATTATGTTT 100
<210> 5957
<211> 100
<212> DNA
<213> Escherichia coli K-12 MG1655
<220> b2341 b2341
<222> 2024-2123
<223> b2341 b2341 U00096 complement(2455035..2457179)
<313> 5957: 2050-2069
<400> 5957
TTGCAATAAT GCAACGACTT GCCACGCAGT ATGGTTCCCG TTTTACCCCT 50
TGCGAGCGTT TGGTCGAGAT GGGCGCGCGT GGGGAAAGTT TTTGGAAAAC 100
<210> 5958
<211> 100

<212> DNA
<213> Escherichia coli K-12 MG1655
<220> b2341 b2341
<222> 1973-2072
<223> b2341 b2341 U00096 complement(2455035..2457179)
<313> 5958: 1995-2014
<400> 5958
TTCTCGGTGG ACCGTTCCGC TATATCGATT CTCTCGGCGC GGGCGAAGTG 50
GTTGCAATAA TGCAACGACT TGCCACGCAG TATGGTTCCC GTTTTACCCC 100
<210> 5959
<211> 100
<212> DNA
<213> Escherichia coli K-12 MG1655
<220> b2341 b2341
<222> 1930-2029
<223> b2341 b2341 U00096 complement(2455035..2457179)
<313> 5959: 1943-1962
<400> 5959
GACGGGGATA TTGGCGCGGT ATTTGGCATT GGTTTTCCGC CATTTCTCGG 50
TGGACCGTTC CGCTATATCG ATTCTCTCGG CGCGGGCGAA GTGGTTGCAA 100
<210> 5960
<211> 100
<212> DNA
<213> Escherichia coli K-12 MG1655
<220> b2342 b2342
<222> 1212-1311
<223> b2342 b2342 U00096 complement(2457179..2458489)
<313> 5960: 1276-1295
<400> 5960
GACATTGCAT GAACTTCGCC GTCGCGGCGG TGGATTTGGT TTAGTTACCG 50
CCTGTGCTGC CGGTGGGCTT GGCGCGGCAA TGGTTCTGGA GGCGGAATAA 100
<210> 5961
<211> 100
<212> DNA
<213> Escherichia coli K-12 MG1655
<220> b2342 b2342
<222> 1166-1265
<223> b2342 b2342 U00096 complement(2457179..2458489)
<313> 5961: 1198-1217
<400> 5961
CTTACGGGCA TCCCTTCGCG GCGACCGGCG CGCGGATGAT TACCCAGACA 50
TTGCATGAAC TTCGCCGTCG CGGCGGTGGA TTTGGTTTAG TTACCGCCTG 100
<210> 5962
<211> 100
<212> DNA
<213> Escherichia coli K-12 MG1655
<220> b2342 b2342
<222> 1142-1241
<223> b2342 b2342 U00096 complement(2457179..2458489)
<313> 5962: 1175-1194
<400> 5962
TTAACGTGCT TGGCGGTTCG ATTGCTTACG GGCATCCCTT CGCGGCGACC 50
GGCGCGCGGA TGATTACCCA GACATTGCAT GAACTTCGCC GTCGCGGCGG 100
<210> 5963
<211> 100
<212> DNA
<213> Escherichia coli K-12 MG1655
<220> b2343 b2344
<222> 210-309
<223> b2343 b2344 U00096 complement(2458670..2458978)
<313> 5963: 268-287
<400> 5963
CCCGACTTTC ACTGAAGAGT CTGACGGTGT GCGCCTGGAT ATCGATTTCA 50
CTTTTGCCTG CGAAGCTGAA ATGCTGATTT TCCAGCTGGG TCTGCGTTAA 100
<210> 5964
<211> 100
<212> DNA
<213> Escherichia coli K-12 MG1655
<220> b2343 b2344
<222> 181-280
<223> b2343 b2344 U00096 complement(2458670..2458978)

<313> 5964: 204-223
<400> 5964
AGCGTGGAAT CTGAACCTTG CAAAATCACC CCGACTTTCA CTGAAGAGTC 50
TGACGGTGTG CGCCTGGATA TCGATTTCAC TTTTGCCTGC GAAGCTGAAA 100
<210> 5965
<211> 100
<212> DNA
<213> Escherichia coli K-12 MG1655
<220> b2343 b2344
<222> 145-244
<223> b2343 b2344 U00096 complement(2458670..2458978)
<313> 5965: 180-199
<400> 5965
GAGCAAACGC TGGCGGCGCT GACTGAAAAA GCCCGTAGCG TGGAATCTGA 50
ACCTTGCAAA ATCACCCCGA CTTTCACTGA AGAGTCTGAC GGTGTGCGCC 100
<210> 5966
<211> 100
<212> DNA
<213> Escherichia coli K-12 MG1655
<220> b2345 b2346
<222> 735-834
<223> b2345 b2346 U00096 2461032..2462090
<313> 5966: 784-803
<400> 5966
ATTTGCAACT GGTTCCGATC ATCAGGTTGT AAACGAGCTC AACGGGGAAA 50
GACTGAGAGA ACCAAACGAC GTTTTTAAAC GTTATGGTCG AGCAATAAGA 100
<210> 5967
<211> 100
<212> DNA
<213> Escherichia coli K-12 MG1655
<220> b2345 b2346
<222> 680-779
<223> b2345 b2346 U00096 2461032..2462090
<313> 5967: 711-730
<400> 5967
ACGCCAGGAG TGCTTCAGCG TGGTTTGAAA GCAGTAAAGC TATCATGGCA 50
GAATCATTTG CAACTGGTTC CGATCATCAG GTTGTAAACG AGCTCAACGG 100
<210> 5968
<211> 100
<212> DNA
<213> Escherichia coli K-12 MG1655
<220> b2345 b2346
<222> 564-663
<223> b2345 b2346 U00096 2461032..2462090
<313> 5968: 613-632
<400> 5968
TAAAAATAAA AACGGAAATG ATTACTTATC TCTCGGTTAC TACGATCAGG 50
ACGACTACCA CATTCAAAGA GGAATTCGTA TTAATGGTGA TAGTCTCACC 100
<210> 5969
<211> 100
<212> DNA
<213> Escherichia coli K-12 MG1655
<220> yccv yccV
<222> 270-369
<223> yccv yccV U00096 complement(1027627..1027995)
<313> 5969: 337-356
<400> 5969
AAGTAGCGAA TTGCAGGATG AACATCCTGA ACAGCCATCC ATGGATGAGC 50
TGGCGCAGAC CATCCGCAAA CAACTCCAGG CCCCGCGTCT GCGTAACTAA 100
<210> 5970
<211> 100
<212> DNA
<213> Escherichia coli K-12 MG1655
<220> yccv yccV
<222> 228-327
<223> yccv yccV U00096 complement(1027627..1027995)
<313> 5970: 273-292
<400> 5970
TAACGGCCTA CCGGTTCATA CCTACCTGGC TGAAGCCCAG CTAAGTAGCG 50
AATTGCAGGA TGAACATCCT GAACAGCCAT CCATGGATGA GCTGGCGCAG 100
<210> 5971

<211> 100
<212> DNA
<213> Escherichia coli K-12 MG1655
<220> yccv yccV
<222> 74-173
<223> yccv yccV U00096 complement(1027627..1027995)
<313> 5971: 119-138
<400> 5971
TCGGCCAGCA GGTCCGCCAT TCCCTGTTAG GTTATCTCGG AGTGGTCGTG 50
GATATCGACC CGGTTTATTC GCTCTCTGAA CCGTCGCCTG ATGAGCTGGC 100
<210> 5972
<211> 100
<212> DNA
<213> Escherichia coli K-12 MG1655
<220> yccy b0983
<222> 226-325
<223> yccy b0983 U00096 complement(1043453..1043911)
<313> 5972: 274-293
<400> 5972
CTCACTGCTG AGATGGCACG AAATTACGAT CTGATTCTGG CAATGGAGTC 50
GGAACATATT GCTCAGGTTA CCGCAATTGC TCCCGAGGTC CGCGGGAAGA 100
<210> 5973
<211> 100
<212> DNA
<213> Escherichia coli K-12 MG1655
<220> yccy b0983
<222> 207-306
<223> yccy b0983 U00096 complement(1043453..1043911)
<313> 5973: 244-263
<400> 5973
AGGACATGCC GGACGTAAGC TCACTGCTGA GATGGCACGA AATTACGATC 50
TGATTCTGGC AATGGAGTCG GAACATATTG CTCAGGTTAC CGCAATTGCT 100
<210> 5974
<211> 100
<212> DNA
<213> Escherichia coli K-12 MG1655
<220> yccy b0983
<222> 174-273
<223> yccy b0983 U00096 complement(1043453..1043911)
<313> 5974: 190-209
<400> 5974
AGATGTTGCC GCTAATCATG GCGTATCTCT TGAAGGACAT GCCGGACGTA 50
AGCTCACTGC TGAGATGGCA CGAAATTACG ATCTGATTCT GGCAATGGAG 100
<210> 5975
<211> 100
<212> DNA
<213> Escherichia coli K-12 MG1655
<220> yccz b0984
<222> 1010-1109
<223> yccz b0984 U00096 complement(1043887..1045026)
<313> 5975: 1047-1066
<400> 5975
ACGACATTGT TTATGTCACT ACCGCGCCGC TGGCTCGTTG GAACCGTGTG 50
ATTTCTCTGC TGGTACCGAC GATTTCCGGG GTGCATGACC TGACAGAGAC 100
<210> 5976
<211> 100
<212> DNA
<213> Escherichia coli K-12 MG1655
<220> yccz b0984
<222> 943-1042
<223> yccz b0984 U00096 complement(1043887..1045026)
<313> 5976: 972-991
<400> 5976
ATTTATCAGC TGAATGCGAA GGACGCATCG GCAATGATAC TCGGAACGGA 50
ATTCCAGCTT GAACCTTACG ACATTGTTTA TGTCACTACC GCGCCGCTGG 100
<210> 5977
<211> 100
<212> DNA
<213> Escherichia coli K-12 MG1655
<220> yccz b0984
<222> 888-987

<223> yccz b0984 U00096 complement(1043887..1045026)
<313> 5977: 909-928
<400> 5977
GGGGATTTTT GTTATTCGTG CAACGCAAAA CAAGCAGAAT GGCAAAATCG 50
CCAATATTTA TCAGCTGAAT GCGAAGGACG CATCGGCAAT GATACTCGGA 100
<210> 5978
<211> 100
<212> DNA
<213> Escherichia coli K-12 MG1655
<220> ycdb b1020
<222> 1173-1272
<223> ycdb b1020 U00096 1082599..1083870
<313> 5978: 1213-1232
<400> 5978
GGAGGAATAC GTTAAACCTA TCGGCGGCGG TTATTTTTTT GCGCTGCCGG 50
GGGTGAAGGA CGCGAACGAT TATTTCGGAA GCGCGTTATT GCGGGTTTAA 100
<210> 5979
<211> 100
<212> DNA
<213> Escherichia coli K-12 MG1655
<220> ycdb b1020
<222> 1142-1241
<223> ycdb b1020 U00096 1082599..1083870
<313> 5979: 1170-1189
<400> 5979
CAGTACAAAA AAGGCTCAAT GGCGAAGCGC TGGAGGAATA CGTTAAACCT 50
ATCGGCGGCG GTTATTTTTT TGCGCTGCCG GGGGTGAAGG ACGCGAACGA 100
<210> 5980
<211> 100
<212> DNA
<213> Escherichia coli K-12 MG1655
<220> ycdb b1020
<222> 1116-1215
<223> ycdb b1020 U00096 1082599..1083870
<313> 5980: 1142-1161
<400> 5980
ACACGATCTG GAAAAAGGCT TCCTGACAGT ACAAAAAAGG CTCAATGGCG 50
AAGCGCTGGA GGAATACGTT AAACCTATCG GCGGCGGTTA TTTTTTTGCG 100
<210> 5981
<211> 100
<212> DNA
<213> Escherichia coli K-12 MG1655
<220> ycdc b1014
<222> 540-639
<223> ycdc b1014 U00096 1073465..1074103
<313> 5981: 617-636
<400> 5981
GGAGGCGGTG ACAGGCGCGA CGTTGCGCGA TGAGGTATTT TTCAATCAAA 50
CGGTTGAAAA CGTGCAGCGG ATTATTATTG AGGGGATTCG ACCACGTTAA 100
<210> 5982
<211> 100
<212> DNA
<213> Escherichia coli K-12 MG1655
<220> ycdc b1014
<222> 486-585
<223> ycdc b1014 U00096 1073465..1074103
<313> 5982: 542-561
<400> 5982
GATTTTTATG ATTTGGGCTT CCACTCAACA TTACGCCGAT TTCGCCCCTC 50
AGGTGGAGGC GGTGACAGGC GCGACGTTGC GCGATGAGGT ATTTTTCAAT 100
<210> 5983
<211> 100
<212> DNA
<213> Escherichia coli K-12 MG1655
<220> ycdc b1014
<222> 278-377
<223> ycdc b1014 U00096 1073465..1074103
<313> 5983: 294-313
<400> 5983
CGATCAAAGA GTACATCCGT CTGAAGCTGG AAGTCTCACG CGATTATCCG 50
CAGGCTTCGC GCCTGTTCTG TATGGAGATG CTGGCAGGCG CGCCGCTGTT 100

<210> 5984
<211> 100
<212> DNA
<213> Escherichia coli K-12 MG1655
<220> dksa b0146
<222> 357-456
<223> dksa b0146 U00096 complement(160149..160604)
<313> 5984: 413-432
<400> 5984
TGAAATTGGT ATTCGCCGTC TGGAAGCGCG CCCGACAGCC GATCTGTGCA  50
TCGACTGCAA AACGCTGGCT GAAATTCGCG AAAAACAGAT GGCTGGCTAA  100
<210> 5985
<211> 100
<212> DNA
<213> Escherichia coli K-12 MG1655
<220> dksa b0146
<222> 318-417
<223> dksa b0146 U00096 complement(160149..160604)
<313> 5985: 347-366
<400> 5985
GGAAGACGAA GATTTCGGCT ACTGCGAATC CTGCGGTGTT GAAATTGGTA  50
TTCGCCGTCT GGAAGCGCGC CCGACAGCCG ATCTGTGCAT CGACTGCAAA  100
<210> 5986
<211> 100
<212> DNA
<213> Escherichia coli K-12 MG1655
<220> dksa b0146
<222> 254-353
<223> dksa b0146 U00096 complement(160149..160604)
<313> 5986: 274-293
<400> 5986
AACTGCGTAA CCGCGATCGC GAGCGTAAGC TGATCAAAAA GATCGAGAAG  50
ACGCTGAAAA AAGTGGAAGA CGAAGATTTC GGCTACTGCG AATCCTGCGG  100
<210> 5987
<211> 100
<212> DNA
<213> Escherichia coli K-12 MG1655
<220> ycdf b1006
<222> 132-231
<223> ycdf b1006 U00096 1067141..1067371
<313> 5987: 158-177
<400> 5987
ATATGATGTC TATACCCAGA CGGAGGTCAG TAATGGCAAA CCATCGAGGC  50
GGTTCCGGCA ATTTTGCAGA AGACCGCGAA AGAGCATCAG AAGCAGGTAA  100
<210> 5988
<211> 100
<212> DNA
<213> Escherichia coli K-12 MG1655
<220> ycdf b1006
<222> 81-180
<223> ycdf b1006 U00096 1067141..1067371
<313> 5988: 120-139
<400> 5988
CCCACGTAGC GAAAATATGG CAGCCGCCAT ACGCCGCGTT AATTCTATGC  50
AATATGATGT CTATACCCAG ACGGAGGTCA GTAATGGCAA ACCATCGAGG  100
<210> 5989
<211> 100
<212> DNA
<213> Escherichia coli K-12 MG1655
<220> ycdf b1006
<222> 1-100
<223> ycdf b1006 U00096 1067141..1067371
<313> 5989: 23-42
<400> 5989
ATGATATCTA TTCTCGCTAA GAAGCTGTTG CAGGATATTA CCAAACGCGG  50
GTCTGCCCGC GTCAGTTCAC TAAGCTTAGT CCCACGTAGC GAAAATATGG  100
<210> 5990
<211> 100
<212> DNA
<213> Escherichia coli K-12 MG1655
<220> ycdg ycdG

<222> 1296-1395
<223> ycdg ycdG U00096 complement(1067734..1069128)
<313> 5990: 1365-1384
<400> 5990
GATTGGTACA GCAACCTTTG GCGCGATTTT ACTCAATGCG TTGTTAAGCA 50
GAAAGTTGGT TGACGTTCCA CCGCCAGAAG TTGTTCATCA GGAGCCATAA 100
<210> 5991
<211> 100
<212> DNA
<213> Escherichia coli K-12 MG1655
<220> ycdg ycdG
<222> 1264-1363
<223> ycdg ycdG U00096 complement(1067734..1069128)
<313> 5991: 1299-1318
<400> 5991
GCTCTCACGC TGGGCGGTTT TACGTTGGGA GGGATTGGTA CAGCAACCTT 50
TGGCGCGATT TTACTCAATG CGTTGTTAAG CAGAAAGTTG GTTGACGTTC 100
<210> 5992
<211> 100
<212> DNA
<213> Escherichia coli K-12 MG1655
<220> ycdg ycdG
<222> 1221-1320
<223> ycdg ycdG U00096 complement(1067734..1069128)
<313> 5992: 1270-1289
<400> 5992
GATTATGGTC GCAGTAACCT TAGTGCTGGG CGCGGGTGAT TTTGCTCTCA 50
CGCTGGGCGG TTTTACGTTG GGAGGGATTG GTACAGCAAC CTTTGGCGCG 100
<210> 5993
<211> 100
<212> DNA
<213> Escherichia coli K-12 MG1655
<220> sera b2914
<222> 1078-1177
<223> sera b2914 U00096 complement(3055198..3056430)
<313> 5993: 1119-1138
<400> 5993
GAGCAGGGCG TCAACATCGC CGCGCAATAT CTGCAAACTT CCGCCCAGAT 50
GGGTTATGTG GTTATTGATA TTGAAGCCGA CGAAGACGTT GCCGAAAAAG 100
<210> 5994
<211> 100
<212> DNA
<213> Escherichia coli K-12 MG1655
<220> sera b2914
<222> 1054-1153
<223> sera b2914 U00096 complement(3055198..3056430)
<313> 5994: 1067-1086
<400> 5994
ACTGCGCTGA ACAAAATCTT CGCCGAGCAG GGCGTCAACA TCGCCGCGCA 50
ATATCTGCAA ACTTCCGCCC AGATGGGTTA TGTGGTTATT GATATTGAAG 100
<210> 5995
<211> 100
<212> DNA
<213> Escherichia coli K-12 MG1655
<220> sera b2914
<222> 964-1063
<223> sera b2914 U00096 complement(3055198..3056430)
<313> 5995: 1007-1026
<400> 5995
CTCTCTGCGG TGAACTTCCC GGAAGTCTCG CTGCCACTGC ACGGTGGGCG 50
TCGTCTGATG CACATCCACG AAAACCGTCC GGGCGTGCTA ACTGCGCTGA 100
<210> 5996
<211> 100
<212> DNA
<213> Escherichia coli K-12 MG1655
<220> serb b4389
<222> 851-950
<223> serb b4389 U00096 4622464..4623432
<313> 5996: 877-896
<400> 5996
GGCTGGGGAT TGCCTACCAT GCCAAGCCAA AAGTGAATGA AAAGGCGGAA 50

GTCACCATCC GTCACGCTGA CCTGATGGGG GTATTCTGCA TCCTCTCAGG 100
<210> 5997
<211> 100
<212> DNA
<213> Escherichia coli K-12 MG1655
<220> serb b4389
<222> 796-895
<223> serb b4389 U00096 4622464..4623432
<313> 5997: 842-861
<400> 5997
CAGACCGTGG CGATTGGCGA TGGAGCCAAT GACCTGCCGA TGATCAAAGC 50
GGCAGGGCTG GGGATTGCCT ACCATGCCAA GCCAAAAGTG AATGAAAAGG 100
<210> 5998
<211> 100
<212> DNA
<213> Escherichia coli K-12 MG1655
<220> serb b4389
<222> 729-828
<223> serb b4389 U00096 4622464..4623432
<313> 5998: 763-782
<400> 5998
CGTAGACGCG CAGTACAAAG CGAAAACTCT GACTCGCCTC GCGCAGGAGT 50
ATGAAATCCC GCTGGCGCAG ACCGTGGCGA TTGGCGATGG AGCCAATGAC 100
<210> 5999
<211> 100
<212> DNA
<213> Escherichia coli K-12 MG1655
<220> b2350 b2350
<222> 264-363
<223> b2350 b2350 U00096 2465875..2466237
<313> 5999: 310-329
<400> 5999
CGCACTTCCC CCGATGATAA CTCTTGTCAC CTTCTCCGCC ATCAGCCTGG 50
TGTGCGGTTT CGTCTATTCA AAGTTCATTG TCTTTAGGGA TGCGAAATGA 100
<210> 6000
<211> 100
<212> DNA
<213> Escherichia coli K-12 MG1655
<220> b2350 b2350
<222> 244-343
<223> b2350 b2350 U00096 2465875..2466237
<313> 6000: 279-298
<400> 6000
GGATGGGCTG CTGATAGATG CGCACTTCCC CCGATGATAA CTCTTGTCAC 50
CTTCTCCGCC ATCAGCCTGG TGTGCGGTTT CGTCTATTCA AAGTTCATTG 100
<210> 6001
<211> 100
<212> DNA
<213> Escherichia coli K-12 MG1655
<220> b2350 b2350
<222> 213-312
<223> b2350 b2350 U00096 2465875..2466237
<313> 6001: 235-254
<400> 6001
TGGGTTCATG GGGACACTGA GTGCTACTGT TGGATGGGCT GCTGATAGAT 50
GCGCACTTCC CCCGATGATA ACTCTTGTCA CCTTCTCCGC CATCAGCCTG 100
<210> 6002
<211> 100
<212> DNA
<213> Escherichia coli K-12 MG1655
<220> serc b0908
<222> 914-1013
<223> serc b0908 U00096 956876..957964
<313> 6002: 941-960
<400> 6002
CGGACAGTGC GCTTGACAAA TTGTTCCTTG AAGAGTCTTT TGCTGCTGGC 50
CTTCATGCAC TGAAAGGTCA CCGTGTGGTC GGCGGAATGC GCGCTTCTAT 100
<210> 6003
<211> 100
<212> DNA
<213> Escherichia coli K-12 MG1655

<220> serc b0908
<222> 802-901
<223> serc b0908 U00096 956876..957964
<313> 6003: 868-887
<400> 6003
ATCAATCAGC AAAAAGCAGA ACTGCTATAT GGGGTGATTG ATAACAGCGA 50
TTTCTACCGC AATGACGTGG CGAAAGCTAA CCGTTCGCGG ATGAACGTGC 100
<210> 6004
<211> 100
<212> DNA
<213> Escherichia coli K-12 MG1655
<220> serc b0908
<222> 742-841
<223> serc b0908 U00096 956876..957964
<313> 6004: 787-806
<400> 6004
CTATCTGGTC TGGTCTTTAA ATGGCTGAAA GCGAACGGCG GTGTAGCTGA 50
AATGGATAAA ATCAATCAGC AAAAAGCAGA ACTGCTATAT GGGGTGATTG 100
<210> 6005
<211> 100
<212> DNA
<213> Escherichia coli K-12 MG1655
<220> b2351 b2351
<222> 815-914
<223> b2351 b2351 U00096 2466234..2467154
<313> 6005: 868-887
<400> 6005
GTGGAATTCA GATGATTGGA ATAGGAGTAT TAGGTGAATA TATTGGACGC 50
ACATACATTG AAACCAAAAA ACGCCCGAAA TACATCATCA AGAGAGTCAA 100
<210> 6006
<211> 100
<212> DNA
<213> Escherichia coli K-12 MG1655
<220> b2351 b2351
<222> 688-787
<223> b2351 b2351 U00096 2466234..2467154
<313> 6006: 718-737
<400> 6006
ACATACATAG GGTTAGTGGT AGCCAGTGTA GCATTTATTT ATGGGGCGTG 50
GATGATTTTA GATACTATCA TATTTGGAAA TGCTGTTAGG GGATATCCTT 100
<210> 6007
<211> 100
<212> DNA
<213> Escherichia coli K-12 MG1655
<220> b2351 b2351
<222> 623-722
<223> b2351 b2351 U00096 2466234..2467154
<313> 6007: 672-691
<400> 6007
GGAAACTTTG GAATTTAGCA CTTGAGGGTA TTACAAGCTT TTCCACATTC 50
CCTCTTCGCA TCTGGACATA CATAGGGTTA GTGGTAGCCA GTGTAGCATT 100
<210> 6008
<211> 100
<212> DNA
<213> Escherichia coli K-12 MG1655
<220> mopa b4144
<222> 1548-1647
<223> mopa b4144 U00096 4368603..4370249
<313> 6008: 1627-1646
<400> 6008
CACCGAATGC ATGGTTACCG ACCTGCCGAA AAACGATGCA GCTGACTTAG 50
GCGCTGCTGG CGGTATGGGC GGCATGGGTG GCATGGGCGG CATGATGTAA 100
<210> 6009
<211> 100
<212> DNA
<213> Escherichia coli K-12 MG1655
<220> mopa b4144
<222> 1535-1634
<223> mopa b4144 U00096 4368603..4370249
<313> 6009: 1562-1581
<400> 6009

GCCTGATGAT CACCACCGAA TGCATGGTTA CCGACCTGCC GAAAAACGAT 50
GCAGCTGACT TAGGCGCTGC TGGCGGTATG GGCGGCATGG GTGGCATGGG 100
<210> 6010
<211> 100
<212> DNA
<213> Escherichia coli K-12 MG1655
<220> mopa b4144
<222> 1427-1526
<223> mopa b4144 U00096 4368603..4370249
<313> 6010: 1447-1466
<400> 6010
ACGGTTACAA CGCAGCAACC GAAGAATACG GCAACATGAT CGACATGGGT 50
ATCCTGGATC CAACCAAAGT AACTCGTTCT GCTCTGCAGT ACGCAGCTTC 100
<210> 6011
<211> 100
<212> DNA
<213> Escherichia coli K-12 MG1655
<220> b2352 b2352
<222> 792-891
<223> b2352 b2352 U00096 2467151..2468482
<313> 6011: 830-849
<400> 6011
GTTAGATGCC TGGGGTAATA AATTCGACAT ATCATTTGGC GCAACCCCAA 50
CAGAAGTTGG AACGGAATGT TTCGAATCTC ATAAAGATGA AACGTTTTCG 100
<210> 6012
<211> 100
<212> DNA
<213> Escherichia coli K-12 MG1655
<220> b2352 b2352
<222> 727-826
<223> b2352 b2352 U00096 2467151..2468482
<313> 6012: 783-802
<400> 6012
AGTTATCTTT ATATGAAAAA CAACGGGTAT AAAATGCCAT CGTATGTTGA 50
TGATAAGTGT GTTGGGTTAG ATGCCTGGGG TAATAAATTC GACATATCAT 100
<210> 6013
<211> 100
<212> DNA
<213> Escherichia coli K-12 MG1655
<220> b2352 b2352
<222> 613-712
<223> b2352 b2352 U00096 2467151..2468482
<313> 6013: 641-660
<400> 6013
AAACTAATTA TTAAATCTGT AATATGCGTG GTGTGCTTGC TTGCGTCAAT 50
ATTTGCAATA TCTTATTCAA AAGGTGTTGT TGAATTAAAT AAGTACCATG 100
<210> 6014
<211> 100
<212> DNA
<213> Escherichia coli K-12 MG1655
<220> mopb b4143
<222> 195-294
<223> mopb b4143 U00096 4368266..4368559
<313> 6014: 256-275
<400> 6014
TATTTTCAAC GATGGCTACG GTGTGAAATC TGAGAAGATC GACAATGAAG 50
AAGTGTTGAT CATGTCCGAA AGCGACATTC TGGCAATTGT TGAAGCGTAA 100
<210> 6015
<211> 100
<212> DNA
<213> Escherichia coli K-12 MG1655
<220> mopb b4143
<222> 166-265
<223> mopb b4143 U00096 4368266..4368559
<313> 6015: 212-231
<400> 6015
CCGCTGGATG TGAAAGTTGG CGACATCGTT ATTTTCAACG ATGGCTACGG 50
TGTGAAATCT GAGAAGATCG ACAATGAAGA AGTGTTGATC ATGTCCGAAA 100
<210> 6016
<211> 100
<212> DNA

<213> Escherichia coli K-12 MG1655
<220> mopb b4143
<222> 127-226
<223> mopb b4143 U00096 4368266..4368559
<313> 6016: 161-180
<400> 6016
GTCGGCAATG GCCGTATCCT TGAAAATGGC GAAGTGAAGC CGCTGGATGT 50
GAAAGTTGGC GACATCGTTA TTTTCAACGA TGGCTACGGT GTGAAATCTG 100
<210> 6017
<211> 100
<212> DNA
<213> Escherichia coli K-12 MG1655
<220> ycdo b1019
<222> 1004-1103
<223> ycdo b1019 U00096 1081466..1082593
<313> 6017: 1053-1072
<400> 6017
GTACTAAAGA CGGTTTTGAA ACCTACGACA AATTGACCGA TGCCGACCGG 50
AATGCACTGA AAGGACCGAT TACTGCGCTG GCGGAAGATC TGGCGCAACT 100
<210> 6018
<211> 100
<212> DNA
<213> Escherichia coli K-12 MG1655
<220> ycdo b1019
<222> 949-1048
<223> ycdo b1019 U00096 1081466..1082593
<313> 6018: 964-983
<400> 6018
CTGGCAAAAG TCGATGCCAA CTTTAAAAAG GTCGATACCA TTCTGGCGAA 50
ATACCGTACT AAAGACGGTT TTGAAACCTA CGACAAATTG ACCGATGCCG 100
<210> 6019
<211> 100
<212> DNA
<213> Escherichia coli K-12 MG1655
<220> ycdo b1019
<222> 895-994
<223> ycdo b1019 U00096 1081466..1082593
<313> 6019: 918-937
<400> 6019
CAGAAAATTG TCGATTTGCT GCGTCCACAA CTGCAAAAAG CCAACCCGGA 50
ACTGCTGGCA AAAGTCGATG CCAACTTTAA AAAGGTCGAT ACCATTCTGG 100
<210> 6020
<211> 100
<212> DNA
<213> Escherichia coli K-12 MG1655
<220> b2353 b2353
<222> 220-319
<223> b2353 b2353 U00096 2468781..2469125
<313> 6020: 279-298
<400> 6020
GGGCGGAAGC TGATGCAGGC AGAGACCTCC CGACTTAACA CTGTGCTGGA 50
TTACATTGAC GCGGTGACGG CAACAGATAC CAGCACCGCG CCGGATGTCA 100
<210> 6021
<211> 100
<212> DNA
<213> Escherichia coli K-12 MG1655
<220> b2353 b2353
<222> 180-279
<223> b2353 b2353 U00096 2468781..2469125
<313> 6021: 236-255
<400> 6021
AATGGCTTCC ATTAGTCTGA TTCAACTGAA ATTACAGGCT GGGCGGAAGC 50
TGATGCAGGC AGAGACCTCC CGACTTAACA CTGTGCTGGA TTACATTGAC 100
<210> 6022
<211> 100
<212> DNA
<213> Escherichia coli K-12 MG1655
<220> b2353 b2353
<222> 150-249
<223> b2353 b2353 U00096 2468781..2469125
<313> 6022: 165-184

<400> 6022
AGCACAGCGC CAGTCGCTGA TTGATACTGC AATGGCTTCC ATTAGTCTGA  50
TTCAACTGAA ATTACAGGCT GGGCGGAAGC TGATGCAGGC AGAGACCTCC  100
<210> 6023
<211> 100
<212> DNA
<213> Escherichia coli K-12 MG1655
<220> b1624 b1624
<222> 981-1080
<223> b1624 b1624 U00096 complement(1701292..1702371)
<313> 6023: 1028-1047
<400> 6023
TGAAAATCCG GTTCCGGCAA GCCAGGCAAT CCAGGTAATG GAGTTGATTG  50
AGCTGGGCAT CGAATCCGCC AAACATCGCG CGACTTTGTG CCTTGCATGA  100
<210> 6024
<211> 100
<212> DNA
<213> Escherichia coli K-12 MG1655
<220> b1624 b1624
<222> 934-1033
<223> b1624 b1624 U00096 complement(1701292..1702371)
<313> 6024: 985-1004
<400> 6024
TATCCGGCTT ACTATGCGGC TATTCGTGAT GCGTTAAATG GCGATGGTGA  50
AAATCCGGTT CCGGCAAGCC AGGCAATCCA GGTAATGGAG TTGATTGAGC  100
<210> 6025
<211> 100
<212> DNA
<213> Escherichia coli K-12 MG1655
<220> b1624 b1624
<222> 898-997
<223> b1624 b1624 U00096 complement(1701292..1702371)
<313> 6025: 933-952
<400> 6025
CGTGTCGAAG AAACGCTGTT GACGGTGCCT GGGAATTATC CGGCTTACTA  50
TGCGGCTATT CGTGATGCGT TAAATGGCGA TGGTGAAAAT CCGGTTCCGG  100
<210> 6026
<211> 100
<212> DNA
<213> Escherichia coli K-12 MG1655
<220> ycdp b1022
<222> 315-414
<223> ycdp b1022 U00096 complement(1085329..1085742)
<313> 6026: 360-379
<400> 6026
CTATCAGCAA CTACAAAAAA GCCACAGGAT GAGCGTACAC TTCACCAGCC  50
AGGGGCAAAT AAAAATGGTT GTTTCAGAAA AAGCGCTAGT CCGGGCATAA  100
<210> 6027
<211> 100
<212> DNA
<213> Escherichia coli K-12 MG1655
<220> ycdp b1022
<222> 231-330
<223> ycdp b1022 U00096 complement(1085329..1085742)
<313> 6027: 283-302
<400> 6027
GCGTTTTCAA AAACAGCAGC ATCATGCAGC CTACCAATAT ACGCCGCAAG  50
AATATGCAGA GAGCTTAGCA ATACCTGATG AGCTCTATCA GCAACTACAA  100
<210> 6028
<211> 100
<212> DNA
<213> Escherichia coli K-12 MG1655
<220> ycdp b1022
<222> 202-301
<223> ycdp b1022 U00096 complement(1085329..1085742)
<313> 6028: 247-266
<400> 6028
TTAATTGTCT GGGCGCTGTA CAATAAGCTG CGTTTTCAAA AACAGCAGCA  50
TCATGCAGCC TACCAATATA CGCCGCAAGA ATATGCAGAG AGCTTAGCAA  100
<210> 6029
<211> 100

<212> DNA
<213> Escherichia coli K-12 MG1655
<220> b2354 b2355
<222> 342-441
<223> b2354 b2355 U00096 complement(2469097..2469537)
<313> 6029: 399-418
<400> 6029
TAATTTGTGG CTGGATTATC TGGACGCACT GGAGCTGGTC GATACTTCCA 50
GTGCGCCAGA TATTGAATGG CCTACTCCTC CGGCAGTTCA GGCCAGATGA 100
<210> 6030
<211> 100
<212> DNA
<213> Escherichia coli K-12 MG1655
<220> b2354 b2355
<222> 320-419
<223> b2354 b2355 U00096 complement(2469097..2469537)
<313> 6030: 365-384
<400> 6030
AAGGTGAGGA ACTGGCGCAA TATAATTTGT GGCTGGATTA TCTGGACGCA 50
CTGGAGCTGG TCGATACTTC CAGTGCGCCA GATATTGAAT GGCCTACTCC 100
<210> 6031
<211> 100
<212> DNA
<213> Escherichia coli K-12 MG1655
<220> b2354 b2355
<222> 268-367
<223> b2354 b2355 U00096 complement(2469097..2469537)
<313> 6031: 329-348
<400> 6031
GATTATATGA ACAGTAAACA ATGGGCTGGT AAAGCGGCTA TTGGTCGTCT 50
GAAAGGTGAG GAACTGGCGC AATATAATTT GTGGCTGGAT TATCTGGACG 100
<210> 6032
<211> 100
<212> DNA
<213> Escherichia coli K-12 MG1655
<220> b1625 b1625
<222> 82-181
<223> b1625 b1625 U00096 1702973..1703188
<313> 6032: 129-148
<400> 6032
TACACCCTGA CGGACGATCA GGAACTGATC AATATGTATC GTGCTGCCGA 50
TCACCGTCGC GCAGAGCTGG TTTCTGGCGG GCGTTTGTTT GACCTCGGCC 100
<210> 6033
<211> 100
<212> DNA
<213> Escherichia coli K-12 MG1655
<220> b1625 b1625
<222> 78-177
<223> b1625 b1625 U00096 1702973..1703188
<313> 6033: 117-136
<400> 6033
TAATTACACC CTGACGGACG ATCAGGAACT GATCAATATG TATCGTGCTG 50
CCGATCACCG TCGCGCAGAG CTGGTTTCTG GCGGGCGTTT GTTTGACCTC 100
<210> 6034
<211> 100
<212> DNA
<213> Escherichia coli K-12 MG1655
<220> b1625 b1625
<222> 26-125
<223> b1625 b1625 U00096 1702973..1703188
<313> 6034: 71-90
<400> 6034
AATTTCGCAA AATCAGTTCA CTCGAAAGTC TGGAAAAACT CTACGACCAT 50
CTTAATTACA CCCTGACGGA CGATCAGGAA CTGATCAATA TGTATCGTGC 100
<210> 6035
<211> 100
<212> DNA
<213> Escherichia coli K-12 MG1655
<220> ycdq b1023
<222> 1227-1326
<223> ycdq b1023 U00096 complement(1085744..1087069)

<313> 6035: 1264-1283
<400> 6035
CCTGGCAACG ACATTGGTAT CATTTACACG AGTCATGTTG ATGCCTAAAA 50
AGCAACGCGC CCGTTGGGTA AGTCCCGATC GCGGGATTCT GAGAGGTTAA 100
<210> 6036
<211> 100
<212> DNA
<213> Escherichia coli K-12 MG1655
<220> ycdq b1023
<222> 1180-1279
<223> ycdq b1023 U00096 complement(1085744..1087069)
<313> 6036: 1209-1228
<400> 6036
TCGCTTTTCT GGATTATTTG GTTCCCGGTT ATTTTCTGGA TGCTGAGCCT 50
GGCAACGACA TTGGTATCAT TTACACGAGT CATGTTGATG CCTAAAAAGC 100
<210> 6037
<211> 100
<212> DNA
<213> Escherichia coli K-12 MG1655
<220> ycdq b1023
<222> 1044-1143
<223> ycdq b1023 U00096 complement(1085744..1087069)
<313> 6037: 1079-1098
<400> 6037
CGGTGTACCG TTAAATATTG AATTGACACA TATCGCTGCG ACACATACTG 50
CCGGAATATT ATTGTGTACG TTATGTTTAC TGCAATTTAT TGTCAGCCTG 100
<210> 6038
<211> 100
<212> DNA
<213> Escherichia coli K-12 MG1655
<220> b1626 b1626
<222> 366-465
<223> b1626 b1626 U00096 1703250..1703714
<313> 6038: 410-429
<400> 6038
AGCCGTTCGC CAGTTGCTGT TTACTTTGTT GGCAACCGCA TTGATCGTCC 50
CCTATTTCAA ACGCTCGTCG CGGGTAAAAG CGACGTTTGT GAATCCGTAA 100
<210> 6039
<211> 100
<212> DNA
<213> Escherichia coli K-12 MG1655
<220> b1626 b1626
<222> 323-422
<223> b1626 b1626 U00096 1703250..1703714
<313> 6039: 360-379
<400> 6039
TGGCAGTAAA AGCATTCGCT TTTTCACCCG TGGAAGATGG CATAGCCGTT 50
CGCCAGTTGC TGTTTACTTT GTTGGCAACC GCATTGATCG TCCCCTATTT 100
<210> 6040
<211> 100
<212> DNA
<213> Escherichia coli K-12 MG1655
<220> b1626 b1626
<222> 113-212
<223> b1626 b1626 U00096 1703250..1703714
<313> 6040: 179-198
<400> 6040
CGTTGTTGCT GTATACCGCT GCGTTATCTT CTCCGCAAAC ATTTCAAACG 50
CTCGGCGGAC AAGCACTGAC GACACAAATC TTATGGGGCG TTTCTTTTAT 100
<210> 6041
<211> 100
<212> DNA
<213> Escherichia coli K-12 MG1655
<220> yrfa b3393
<222> 343-442
<223> yrfa b3393 U00096 complement(3518253..3518696)
<313> 6041: 371-390
<400> 6041
CAAGGAGATA CAAATGAAGC AATGGATAGC CGCACTACTG TTGATGCTGA 50
TACCCGGCGT ACAGGCGGCA AAGCCGCAAA AAGTGACGCT GATGGTGGAT 100
<210> 6042

<211> 100
<212> DNA
<213> Escherichia coli K-12 MG1655
<220> yrfa b3393
<222> 216-315
<223> yrfa b3393 U00096 complement(3518253..3518696)
<313> 6042: 253-272
<400> 6042
GAAATGGCGA CGGGTGCAGC AAAACGATGT GCTGGAAAAC GGCTGGACAA 50
TTTTACAGCT GACGCCAGAC GTACTAACGC TGGGTACCGG GACAAACTGC 100
<210> 6043
<211> 100
<212> DNA
<213> Escherichia coli K-12 MG1655
<220> yrfa b3393
<222> 180-279
<223> yrfa b3393 U00096 complement(3518253..3518696)
<313> 6043: 211-230
<400> 6043
CGAGCGCATC ATCGGTGTAA TAAAAGACGG GCAAAAGAAA TGGCGACGGG 50
TGCAGCAAAA CGATGTGCTG GAAAACGGCT GGACAATTTT ACAGCTGACG 100
<210> 6044
<211> 100
<212> DNA
<213> Escherichia coli K-12 MG1655
<220> ycdr b1024
<222> 1920-2019
<223> ycdr b1024 U00096 complement(1087062..1089080)
<313> 6044: 1994-2013
<400> 6044
AAACTATGGT TATTATCCCG ACAATTTTCT GCATAACCAA CCTGAAATAG 50
ACCTTATTCG TCCTGAGTTT TCAACAGCCT GGTATCCGAA AAATGATTAA 100
<210> 6045
<211> 100
<212> DNA
<213> Escherichia coli K-12 MG1655
<220> ycdr b1024
<222> 1870-1969
<223> ycdr b1024 U00096 complement(1087062..1089080)
<313> 6045: 1884-1903
<400> 6045
TCGCAACAAC TCGCTCACTG GATGAGCCTA TTACAACTGA ATGGAGTGAA 50
AAACTATGGT TATTATCCCG ACAATTTTCT GCATAACCAA CCTGAAATAG 100
<210> 6046
<211> 100
<212> DNA
<213> Escherichia coli K-12 MG1655
<220> ycdr b1024
<222> 1804-1903
<223> ycdr b1024 U00096 complement(1087062..1089080)
<313> 6046: 1830-1849
<400> 6046
GACAAATCTA TTTTAGAATT ACAGGCACAA AACTGGCAGA AAAATGGTCA 50
GCATCAGGCT ATTTCTTCGC AACAACTCGC TCACTGGATG AGCCTATTAC 100
<210> 6047
<211> 100
<212> DNA
<213> Escherichia coli K-12 MG1655
<220> b1627 b1627
<222> 483-582
<223> b1627 b1627 U00096 1703791..1704372
<313> 6047: 557-576
<400> 6047
TGATGTCCCG GCACCTTTTC GCGGTAATGC CATTGCGTTA ATTACCGCAG 50
GTCTTATGTC TCTGGCCTTT ATGGGCTTTA GTGGTTTGGT GAAGTTGTAA 100
<210> 6048
<211> 100
<212> DNA
<213> Escherichia coli K-12 MG1655
<220> b1627 b1627
<222> 450-549

<223> b1627 b1627 U00096 1703791..1704372
<313> 6048: 499-518
<400> 6048
CTTCGCCGCC ATCCGCGAAC GCCTTGCTGT GGCTGATGTC CCGGCACCTT 50
TTCGCGGTAA TGCCATTGCG TTAATTACCG CAGGTCTTAT GTCTCTGGCC 100
<210> 6049
<211> 100
<212> DNA
<213> Escherichia coli K-12 MG1655
<220> b1627 b1627
<222> 314-413
<223> b1627 b1627 U00096 1703791..1704372
<313> 6049: 377-396
<400> 6049
TTTTTTTGCC GCTTATCACC ACCAACTGTG CAGTGCTCGG CGTGGCGTTG 50
CTGAATATCA ATCTCGGGCA CAATTTCTTG CAGTCGGCGC TGTACGGTTT 100
<210> 6050
<211> 100
<212> DNA
<213> Escherichia coli K-12 MG1655
<220> yrfb b3394
<222> 342-441
<223> yrfb b3394 U00096 complement(3518647..3519087)
<313> 6050: 403-422
<400> 6050
GCTGGCAGAG CGCAACGTCA GCGTGAGCCG TTTTTCGTTA AGCGTGGAAG 50
GTGATGATCT TTTGTTCACG CTACAACTGG AGACGCCGCA TGAGGGTTAA 100
<210> 6051
<211> 100
<212> DNA
<213> Escherichia coli K-12 MG1655
<220> yrfb b3394
<222> 319-418
<223> yrfb b3394 U00096 complement(3518647..3519087)
<313> 6051: 366-385
<400> 6051
GCAGTGCCGT CGGCATTTAC ACGGCTGGCA GAGCGCAACG TCAGCGTGAG 50
CCGTTTTTCG TTAAGCGTGG AAGGTGATGA TCTTTTGTTC ACGCTACAAC 100
<210> 6052
<211> 100
<212> DNA
<213> Escherichia coli K-12 MG1655
<220> yrfb b3394
<222> 163-262
<223> yrfb b3394 U00096 complement(3518647..3519087)
<313> 6052: 226-245
<400> 6052
TGGGCCGCAC TGTATCGCCT GGTAGACACC GCTCCCTTCA GCGAGGAAAA 50
AACGCTGCCC TTTTCGCCAC TGGATTTTCA GTTATCCGGC GCGCAACTGG 100
<210> 6053
<211> 100
<212> DNA
<213> Escherichia coli K-12 MG1655
<220> ycds b1025
<222> 2325-2424
<223> ycds b1025 U00096 complement(1089089..1091512)
<313> 6053: 2356-2375
<400> 6053
GATTGATGCT GGCGCAACGC TACGCTGGGA AAAACGACCT TATGACGGTG 50
ACAGAGAACA CAACTTATAC GTTGAATTCG ATATGACATT CAGATTTTAA 100
<210> 6054
<211> 100
<212> DNA
<213> Escherichia coli K-12 MG1655
<220> ycds b1025
<222> 2260-2359
<223> ycds b1025 U00096 complement(1089089..1091512)
<313> 6054: 2306-2325
<400> 6054
AAACATTATG GCACGGATGT CGTCACCCAA CTCGGCTACG GGCAACGCAT 50
TAGTTGGAAT GACGTGATTG ATGCTGGCGC AACGCTACGC TGGGAAAAAC 100

<210> 6055
<211> 100
<212> DNA
<213> Escherichia coli K-12 MG1655
<220> ycds b1025
<222> 2219-2318
<223> ycds b1025 U00096 complement(1089089..1091512)
<313> 6055: 2259-2278
<400> 6055
GGGAGCAAAT ATTCAGCGCA GGTGTTGGTG CCTCCTGGCA AAAACATTAT  50
GGCACGGATG TCGTCACCCA ACTCGGCTAC GGGCAACGCA TTAGTTGGAA  100
<210> 6056
<211> 100
<212> DNA
<213> Escherichia coli K-12 MG1655
<220> b1628 b1628
<222> 480-579
<223> b1628 b1628 U00096 1704372..1704950
<313> 6056: 544-563
<400> 6056
CTGCATCTCG TTGCAACCGG TCGCAGAAAC ACCTGACTCC TGGAAATGGG  50
ATCTGAACAC CATTCCCGTG CGTATCATTC CCGTGGAACA CCATGCTTAA  100
<210> 6057
<211> 100
<212> DNA
<213> Escherichia coli K-12 MG1655
<220> b1628 b1628
<222> 468-567
<223> b1628 b1628 U00096 1704372..1704950
<313> 6057: 515-534
<400> 6057
GTGCCCGACG CACTGCATCT CGTTGCAACC GGTCGCAGAA ACACCTGACT  50
CCTGGAAATG GGATCTGAAC ACCATTCCCG TGCGTATCAT TCCCGTGGAA  100
<210> 6058
<211> 100
<212> DNA
<213> Escherichia coli K-12 MG1655
<220> b1628 b1628
<222> 357-456
<223> b1628 b1628 U00096 1704372..1704950
<313> 6058: 420-439
<400> 6058
CTGCACTAAA TGTATTCAGG CGTGTCCGGT AGACGCCATC GTTGGCGCTA  50
CCCGTGCCAT GCATACGGTA ATGAGTGATC TCTGTACGGG CTGCAATTTA  100
<210> 6059
<211> 100
<212> DNA
<213> Escherichia coli K-12 MG1655
<220> eaeh eaeH
<222> 789-888
<223> eaeh eaeH U00096 313581..314468
<313> 6059: 849-868
<400> 6059
CGATGAAGTC GGGCTGTTTG GTAAAGATAA GCGCCAGAAA GACCCGCATG  50
CTATTTCTGC CGAGGTGACC TATACGCCAG TGCCTCTTAC CCAGCAATAG  100
<210> 6060
<211> 100
<212> DNA
<213> Escherichia coli K-12 MG1655
<220> eaeh eaeH
<222> 775-874
<223> eaeh eaeH U00096 313581..314468
<313> 6060: 807-826
<400> 6060
GAACAGTATT ATGGCGATGA AGTCGGGCTG TTTGGTAAAG ATAAGCGCCA  50
GAAAGACCCG CATGCTATTT CTGCCGAGGT GACCTATACG CCAGTGCCTC  100
<210> 6061
<211> 100
<212> DNA
<213> Escherichia coli K-12 MG1655
<220> eaeh eaeH

<222> 726-825
<223> eaeh eaeH U00096 313581..314468
<313> 6061: 775-794
<400> 6061
TGAGGGCTAT TTACCCGCCT GGCCGCAGCT TGGCGCAAGC CTGATGTATG 50
AACAGTATTA TGGCGATGAA GTCGGGCTGT TTGGTAAAGA TAAGCGCCAG 100
<210> 6062
<211> 100
<212> DNA
<213> Escherichia coli K-12 MG1655
<220> yrfc b3395
<222> 441-540
<223> yrfc b3395 U00096 complement(3519071..3519610)
<313> 6062: 500-519
<400> 6062
TCATCTCAAT CAGCGGGGAG CCACGCAGCA GGATGCGCAG GGACGCTGGC 50
AATTTGAGTA TCAGTTAACA AGGAAGGTTA GCGATGAACA TGTTCTTTGA 100
<210> 6063
<211> 100
<212> DNA
<213> Escherichia coli K-12 MG1655
<220> yrfc b3395
<222> 425-524
<223> yrfc b3395 U00096 complement(3519071..3519610)
<313> 6063: 451-470
<400> 6063
GCCAGGATGC ATCTTTTCAT CTCAATCAGC GGGGAGCCAC GCAGCAGGAT 50
GCGCAGGGAC GCTGGCAATT TGAGTATCAG TTAACAAGGA AGGTTAGCGA 100
<210> 6064
<211> 100
<212> DNA
<213> Escherichia coli K-12 MG1655
<220> yrfc b3395
<222> 356-455
<223> yrfc b3395 U00096 complement(3519071..3519610)
<313> 6064: 410-429
<400> 6064
AGGGAACGCT GGAGATCAAG GGGCTGACAA CAAGCATTAC CGCGTTAAAC 50
GCACTAGAAA CGTCACTCCG CCAGGATGCA TCTTTTCATC TCAATCAGCG 100
<210> 6065
<211> 100
<212> DNA
<213> Escherichia coli K-12 MG1655
<220> ycdt b1026
<222> 1260-1359
<223> ycdt b1026 U00096 1092099..1093457
<313> 6065: 1309-1328
<400> 6065
GATCTATCGA CTGGCTGATA ACGCACTTTA CGAGGCGAAA GAGACCGGGC 50
GTAATAAGGT GGTTGTGAGG GATGTGGTGA ATTTTTGTGA GTCACCATAA 100
<210> 6066
<211> 100
<212> DNA
<213> Escherichia coli K-12 MG1655
<220> ycdt b1026
<222> 1231-1330
<223> ycdt b1026 U00096 1092099..1093457
<313> 6066: 1284-1303
<400> 6066
ACTCAGGAAA ATGCGTTAAA CCCAAATGAG ATCTATCGAC TGGCTGATAA 50
CGCACTTTAC GAGGCGAAAG AGACCGGGCG TAATAAGGTG GTTGTGAGGG 100
<210> 6067
<211> 100
<212> DNA
<213> Escherichia coli K-12 MG1655
<220> ycdt b1026
<222> 1068-1167
<223> ycdt b1026 U00096 1092099..1093457
<313> 6067: 1108-1127
<400> 6067
GCGCGTCGGC GGCGAAGAGT TTGGCGTCTT GCTGACGGAC ATCGATACTG 50

AACGTGCGAA AGCTTTAGCG GAAAGGATTC GGGAAAATGT TGAGCGTTTA 100
<210> 6068
<211> 100
<212> DNA
<213> Escherichia coli K-12 MG1655
<220> b1629 b1630
<222> 2124-2223
<223> b1629 b1630 U00096 1704943..1707165
<313> 6068: 2186-2205
<400> 6068
AGAAGAACAG GTTGATCCGC GCAAAGCGGC AGTTGCCGCG GCTATTGCCC 50
GCGCTCAGGC CAAAAAAGCC GCCCAGCAGA AGGTTGTAAA CGAGGACTAA 100
<210> 6069
<211> 100
<212> DNA
<213> Escherichia coli K-12 MG1655
<220> b1629 b1630
<222> 2034-2133
<223> b1629 b1630 U00096 1704943..1707165
<313> 6069: 2083-2102
<400> 6069
ACAGGTTGAT CCGCGCAAAG CTGCCGTCGA AGCGGCTATT GCACGCGCCA 50
AAGCACGCAA GCTGGAACAG CAACAGGCTA ATGCGGTACC AGAAGAACAG 100
<210> 6070
<211> 100
<212> DNA
<213> Escherichia coli K-12 MG1655
<220> b1629 b1630
<222> 2017-2116
<223> b1629 b1630 U00096 1704943..1707165
<313> 6070: 2039-2058
<400> 6070
AATGCGGAGC CAGAAGAACA GGTTGATCCG CGCAAAGCTG CCGTCGAAGC 50
GGCTATTGCA CGCGCCAAAG CACGCAAGCT GGAACAGCAA CAGGCTAATG 100
<210> 6071
<211> 100
<212> DNA
<213> Escherichia coli K-12 MG1655
<220> yrfd b3396
<222> 708-807
<223> yrfd b3396 U00096 complement(3519610..3520416)
<313> 6071: 763-782
<400> 6071
TGATCCCTGG GAGGCCGTTT CTGTTCGTCA GCCGCCGCTA CCGCCGCCAG 50
GTGGAGACTT TGCCATCGCG CTGGGGCTGG CGCTTGGGAA GGCGTACTGA 100
<210> 6072
<211> 100
<212> DNA
<213> Escherichia coli K-12 MG1655
<220> yrfd b3396
<222> 677-776
<223> yrfd b3396 U00096 complement(3519610..3520416)
<313> 6072: 722-741
<400> 6072
AAAGCGTCGC GATATGTGGC GAAGGCGGAT TTGATCCCTG GGAGGCCGTT 50
TCTGTTCGTC AGCCGCCGCT ACCGCCGCCA GGTGGAGACT TTGCCATCGC 100
<210> 6073
<211> 100
<212> DNA
<213> Escherichia coli K-12 MG1655
<220> yrfd b3396
<222> 628-727
<223> yrfd b3396 U00096 complement(3519610..3520416)
<313> 6073: 647-666
<400> 6073
GGGATGACTA GCGCGAAGGA GCTGGCGGCA GCGTTATCCG TTGATCCCGA 50
AAGCGTCGCG ATATGTGGCG AAGGCGGATT TGATCCCTGG GAGGCCGTTT 100
<210> 6074
<211> 100
<212> DNA
<213> Escherichia coli K-12 MG1655

<220> ycdu ycdU
<222> 888-987
<223> ycdu ycdU U00096 1095066..1096052
<313> 6074: 944-963
<400> 6074
GTGCTTAATT TACTATTTTT TCTATTGTCG ATTCCTCTGG CATAACGATT 50
TTATGATGGC ATGTGACATG TATTTCCGTT GGGGGCATTT TAATAAGTGA 100
<210> 6075
<211> 100
<212> DNA
<213> Escherichia coli K-12 MG1655
<220> ycdu ycdU
<222> 783-882
<223> ycdu ycdU U00096 1095066..1096052
<313> 6075: 836-855
<400> 6075
ATTGCTGGTT ATTGAAGTGA TGTGGATTAC GCTGGTGTCG GTATTGGCAA 50
CGCTTGTAGA ATGGCGGATT TGGTTTGAAG CCTATTTTTT ACTCTGCTAT 100
<210> 6076
<211> 100
<212> DNA
<213> Escherichia coli K-12 MG1655
<220> ycdu ycdU
<222> 729-828
<223> ycdu ycdU U00096 1095066..1096052
<313> 6076: 768-787
<400> 6076
TGAAAATGCG TTGTCGACCT TTTTTGCCAA GCCGCTTTGG CTTCGGTTAT 50
TCATATTGCT GGTTATTGAA GTGATGTGGA TTACGCTGGT GTCGGTATTG 100
<210> 6077
<211> 100
<212> DNA
<213> Escherichia coli K-12 MG1655
<220> rhol b3783
<222> 3-102
<223> rhol b3783 U00096 3963846..3963947
<313> 6077: 76-95
<400> 6077
GCGAAGTGAA CAGATTTCTG GCTCGTCACT CAATCCGTCT TGTCGTTTCA 50
GTTCTGCGTA CTCTCCTGTG ACCAGGCAGC GAAAAGACAT GAGTCGATGA 100
<210> 6078
<211> 100
<212> DNA
<213> Escherichia coli K-12 MG1655
<220> rhol b3783
<222> 3-102
<223> rhol b3783 U00096 3963846..3963947
<313> 6078: 72-91
<400> 6078
GCGAAGTGAA CAGATTTCTG GCTCGTCACT CAATCCGTCT TGTCGTTTCA 50
GTTCTGCGTA CTCTCCTGTG ACCAGGCAGC GAAAAGACAT GAGTCGATGA 100
<210> 6079
<211> 100
<212> DNA
<213> Escherichia coli K-12 MG1655
<220> rhol b3783
<222> 3-102
<223> rhol b3783 U00096 3963846..3963947
<313> 6079: 62-81
<400> 6079
GCGAAGTGAA CAGATTTCTG GCTCGTCACT CAATCCGTCT TGTCGTTTCA 50
GTTCTGCGTA CTCTCCTGTG ACCAGGCAGC GAAAAGACAT GAGTCGATGA 100
<210> 6080
<211> 100
<212> DNA
<213> Escherichia coli K-12 MG1655
<220> b2359 b2359
<222> 348-447
<223> b2359 b2359 U00096 2471540..2471986
<313> 6080: 423-442
<400> 6080

CAATCGCGGT GGTCGCCTGA CTATTCTGCA GGAAGACCAG GGACAATTAC 50
TGACTCTTGC CGGTGAGCCT GACGGAAAAC TCCGCGCAGC AGGTCATTAA 100
<210> 6081
<211> 100
<212> DNA
<213> Escherichia coli K-12 MG1655
<220> b2359 b2359
<222> 282-381
<223> b2359 b2359 U00096 2471540..2471986
<313> 6081: 321-340
<400> 6081
ATATGTTCGC CCGACTAACC GTGGGAAAAT TTCCGAAGAA GACACCACCG 50
AAACGCCGAT GTATGTCAAT CGCGGTGGTC GCCTGACTAT TCTGCAGGAA 100
<210> 6082
<211> 100
<212> DNA
<213> Escherichia coli K-12 MG1655
<220> b2359 b2359
<222> 232-331
<223> b2359 b2359 U00096 2471540..2471986
<313> 6082: 282-301
<400> 6082
GAAATCGAAC CATTTGATGA GAACCGTCTG AAAATCAAAC ACAAACTCTC 50
ATATGTTCGC CCGACTAACC GTGGGAAAAT TTCCGAAGAA GACACCACCG 100
<210> 6083
<211> 100
<212> DNA
<213> Escherichia coli K-12 MG1655
<220> yrfe b3398
<222> 409-508
<223> yrfe b3398 U00096 complement(3523227..3523787)
<313> 6083: 472-491
<400> 6083
CTGGAAGGCG ATGAGCCAGA GCCGCTACCA CAGGTGCGCT GGCCGCTGGC 50
GCATATGATG GATTTGCTGG AAGACCCTGA CTTCAATGAA GCGCGCAATG 100
<210> 6084
<211> 100
<212> DNA
<213> Escherichia coli K-12 MG1655
<220> yrfe b3398
<222> 373-472
<223> yrfe b3398 U00096 complement(3523227..3523787)
<313> 6084: 409-428
<400> 6084
AATATCGTGG TAGCGCAAGA TCTCTACCCG GAATCACTGG AAGGCGATGA 50
GCCAGAGCCG CTACCACAGG TGCGCTGGCC GCTGGCGCAT ATGATGGATT 100
<210> 6085
<211> 100
<212> DNA
<213> Escherichia coli K-12 MG1655
<220> yrfe b3398
<222> 325-424
<223> yrfe b3398 U00096 complement(3523227..3523787)
<313> 6085: 350-369
<400> 6085
TTTTTGAAGA AGCTCAGCAT GGCACCGTCT TACTTTTCCA GCAAAATGAA 50
TATCGTGGTA GCGCAAGATC TCTACCCGGA ATCACTGGAA GGCGATGAGC 100
<210> 6086
<211> 100
<212> DNA
<213> Escherichia coli K-12 MG1655
<220> ycdv b1032
<222> 308-407
<223> ycdv b1032 U00096 1096190..1096603
<313> 6086: 363-382
<400> 6086
CCAACTGGCT GTCGCCAGTT GTCGAACCCC GGTCGGGGCT TCTCATCCCC 50
CCGGTGTGTG CAATATACGA AAAAAAAGCC CGTACTTTCG TACGAGCTCT 100
<210> 6087
<211> 100
<212> DNA

<213> Escherichia coli K-12 MG1655
<220> ycdv b1032
<222> 315-414
<223> ycdv b1032 U00096 1096190..1096603
<313> 6087: 357-376
<400> 6087
GCTGTCGCCA GTTGTCGAAC CCCGGTCGGG GCTTCTCATC CCCCCGGTGT  50
GTGCAATATA CGAAAAAAAA GCCCGTACTT TCGTACGAGC TCTTCTTTAA  100
<210> 6088
<211> 100
<212> DNA
<213> Escherichia coli K-12 MG1655
<220> yrff b3399
<222> 2037-2136
<223> yrff b3399 U00096 3524107..3526242
<313> 6088: 2104-2123
<400> 6088
TTACCAGCGT CATCGCACTC GCATGATGGA GATTCAGGCC TATTATGAAA  50
GCTGCCTGAA CCCGCAACTG ATCACCCCTT CAGAAAGCCT TATCGAATAA  100
<210> 6089
<211> 100
<212> DNA
<213> Escherichia coli K-12 MG1655
<220> yrff b3399
<222> 2023-2122
<223> yrff b3399 U00096 3524107..3526242
<313> 6089: 2038-2057
<400> 6089
CAGGCCTGGC GTCGTTACCA GCGTCATCGC ACTCGCATGA TGGAGATTCA  50
GGCCTATTAT GAAAGCTGCC TGAACCCGCA ACTGATCACC CCTTCAGAAA  100
<210> 6090
<211> 100
<212> DNA
<213> Escherichia coli K-12 MG1655
<220> yrff b3399
<222> 1855-1954
<223> yrff b3399 U00096 3524107..3526242
<313> 6090: 1893-1912
<400> 6090
CATACGCCGT TTAACGCCGA GGGTATCGTC ACAAAAATCT TCACTGACGC  50
CAATGGTACG CAGCATATTG GCCTTCATCC GATCCCGGAT CGTTCCGGCC  100
<210> 6091
<211> 100
<212> DNA
<213> Escherichia coli K-12 MG1655
<220> ycdw b1034
<222> 879-978
<223> ycdw b1034 U00096 1097070..1098047
<313> 6091: 920-939
<400> 6091
CCGTCCCGCT GAAGCTGTGG AGTACATTTC TCGCACCATT GCCCAGCTCG  50
AAAAAGGGGA GAGGGTCTGC GGGCAAGTCG ACCGCGCACG CGGCTACTAA  100
<210> 6092
<211> 100
<212> DNA
<213> Escherichia coli K-12 MG1655
<220> ycdw b1034
<222> 829-928
<223> ycdw b1034 U00096 1097070..1098047
<313> 6092: 874-893
<400> 6092
CTCTGGCAAC ATCCACGCGT GACGATAACA CCACATGTCG CCGCGATTAC  50
CCGTCCCGCT GAAGCTGTGG AGTACATTTC TCGCACCATT GCCCAGCTCG  100
<210> 6093
<211> 100
<212> DNA
<213> Escherichia coli K-12 MG1655
<220> ycdw b1034
<222> 776-875
<223> ycdw b1034 U00096 1097070..1098047
<313> 6093: 820-839

<400> 6093
AAGGCGCAAT GTTGGATGTT TTTAATCGTG AACCCTTACC GCCTGAAAGT 50
CCGCTCTGGC AACATCCACG CGTGACGATA ACACCACATG TCGCCGCGAT 100
<210> 6094
<211> 100
<212> DNA
<213> Escherichia coli K-12 MG1655
<220> yrfg b3400
<222> 615-714
<223> yrfg b3400 U00096 3526262..3526975
<313> 6094: 688-707
<400> 6094
CCTCGGCGTG ACTAATCCTG ATTCCGGGAT TGCCGAGAAA CAGTATCAAC 50
GCCATCCGTC ACTGAATGAC TACCGCCGCC TGATCCCCTC GCTAATGTGA 100
<210> 6095
<211> 100
<212> DNA
<213> Escherichia coli K-12 MG1655
<220> yrfg b3400
<222> 557-656
<223> yrfg b3400 U00096 3526262..3526975
<313> 6095: 616-635
<400> 6095
TTATTGATGA CAGCGAAGCG ATTCTCGATG CTGCCGCGCA ATTTGGTATT 50
CGTTACTGCC TCGGCGTGAC TAATCCTGAT TCCGGGATTG CCGAGAAACA 100
<210> 6096
<211> 100
<212> DNA
<213> Escherichia coli K-12 MG1655
<220> yrfg b3400
<222> 518-617
<223> yrfg b3400 U00096 3526262..3526975
<313> 6096: 569-588
<400> 6096
TGGCCGAAGC TACGGGTCTG AAAGCTGAAA GAACGCTGTT TATTGATGAC 50
AGCGAAGCGA TTCTCGATGC TGCCGCGCAA TTTGGTATTC GTTACTGCCT 100
<210> 6097
<211> 100
<212> DNA
<213> Escherichia coli K-12 MG1655
<220> ycdx b1035
<222> 639-738
<223> ycdx b1035 U00096 1098102..1098839
<313> 6097: 694-713
<400> 6097
TTTTCCGCCA GAGCGCATTT TGAATGTTTC TCCGCGCCGC TTACTGAACT 50
TCCTTGAATC TCGCGGTATG GCACCGATTG CGGAATTTGC AGACCTTTAA 100
<210> 6098
<211> 100
<212> DNA
<213> Escherichia coli K-12 MG1655
<220> ycdx b1035
<222> 602-701
<223> ycdx b1035 U00096 1098102..1098839
<313> 6098: 651-670
<400> 6098
AATTTGAAGA GTGTCTTAAA ATCCTCGACG CGGTAGATTT TCCGCCAGAG 50
CGCATTTTGA ATGTTTCTCC GCGCCGCTTA CTGAACTTCC TTGAATCTCG 100
<210> 6099
<211> 100
<212> DNA
<213> Escherichia coli K-12 MG1655
<220> ycdx b1035
<222> 587-686
<223> ycdx b1035 U00096 1098102..1098839
<313> 6099: 609-628
<400> 6099
CGTTTACCAT GGGGGAATTT GAAGAGTGTC TTAAAATCCT CGACGCGGTA 50
GATTTTCCGC CAGAGCGCAT TTTGAATGTT TCTCCGCGCC GCTTACTGAA 100
<210> 6100
<211> 100

<212> DNA
<213> Escherichia coli K-12 MG1655
<220> yrfh b3401
<222> 303-402
<223> yrfh b3401 U00096 3526986..3527387
<313> 6100: 382-401
<400> 6100
ACGTAAACTT AATGCCTTAA CCATGCCGCA CCCGGACCGA CGCCCGGACA 50
AAAAAGAGCG CCGCGACCTG TTACGATTTA AACACGGCGA CAGTGAATAA 100
<210> 6101
<211> 100
<212> DNA
<213> Escherichia coli K-12 MG1655
<220> yrfh b3401
<222> 303-402
<223> yrfh b3401 U00096 3526986..3527387
<313> 6101: 342-361
<400> 6101
ACGTAAACTT AATGCCTTAA CCATGCCGCA CCCGGACCGA CGCCCGGACA 50
AAAAAGAGCG CCGCGACCTG TTACGATTTA AACACGGCGA CAGTGAATAA 100
<210> 6102
<211> 100
<212> DNA
<213> Escherichia coli K-12 MG1655
<220> yrfh b3401
<222> 243-342
<223> yrfh b3401 U00096 3526986..3527387
<313> 6102: 265-284
<400> 6102
AGCCTTGCTG TATGAAGAGA CTGCGGAAAG TGTAGAGAAA CGCGAAAAAA 50
TGGCGCTGGC ACGTAAACTT AATGCCTTAA CCATGCCGCA CCCGGACCGA 100
<210> 6103
<211> 100
<212> DNA
<213> Escherichia coli K-12 MG1655
<220> ycdy b1036
<222> 456-555
<223> ycdy b1036 U00096 1098863..1099417
<313> 6103: 527-546
<400> 6103
GGCCCATGCA ACCACGCCTT TCTGGCGCAC CATGGCACCG CTAACCCGCG 50
ATGCCATTAG TGCAATGTGG GACGAGCTGG AAGAAGATTC TGAAGAATAA 100
<210> 6104
<211> 100
<212> DNA
<213> Escherichia coli K-12 MG1655
<220> ycdy b1036
<222> 402-501
<223> ycdy b1036 U00096 1098863..1099417
<313> 6104: 458-477
<400> 6104
ACTGTTCAGT GAGTATCTGT TACCCTGGTG TGGTGCGTTC CTTGGCAAAG 50
TGGAGGCCCA TGCAACCACG CCTTTCTGGC GCACCATGGC ACCGCTAACC 100
<210> 6105
<211> 100
<212> DNA
<213> Escherichia coli K-12 MG1655
<220> ycdy b1036
<222> 396-495
<223> ycdy b1036 U00096 1098863..1099417
<313> 6105: 422-441
<400> 6105
GGAAACACTG TTCAGTGAGT ATCTGTTACC CTGGTGTGGT GCGTTCCTTG 50
GCAAAGTGGA GGCCCATGCA ACCACGCCTT TCTGGCGCAC CATGGCACCG 100
<210> 6106
<211> 100
<212> DNA
<213> Escherichia coli K-12 MG1655
<220> yrfi b3402
<222> 786-885
<223> yrfi b3402 U00096 3527406..3528290

<313> 6106: 835-854
<400> 6106
CATGCATTGT GATTACTGCG GTAACCACTA TCTGTTCAAT GCGATGGATA 50
TTGCTGAAAT CCGCAACAAC GCGTCTCCGG CAGATCCGCA AGTTCATTAA 100
<210> 6107
<211> 100
<212> DNA
<213> Escherichia coli K-12 MG1655
<220> yrfi b3402
<222> 747-846
<223> yrfi b3402 U00096 3527406..3528290
<313> 6107: 762-781
<400> 6107
AGAAGTTGAT AGCATCCTGG CGGAAGATGG CGAAATTGAC ATGCATTGTG 50
ATTACTGCGG TAACCACTAT CTGTTCAATG CGATGGATAT TGCTGAAATC 100
<210> 6108
<211> 100
<212> DNA
<213> Escherichia coli K-12 MG1655
<220> yrfi b3402
<222> 699-798
<223> yrfi b3402 U00096 3527406..3528290
<313> 6108: 731-750
<400> 6108
CTGCTCGCGT GAACGTTGCG CCGATGCGCT GAAAACGCTG CCTGATGAAG 50
AAGTTGATAG CATCCTGGCG GAAGATGGCG AAATTGACAT GCATTGTGAT 100
<210> 6109
<211> 100
<212> DNA
<213> Escherichia coli K-12 MG1655
<220> ycdz b1037
<222> 441-540
<223> ycdz b1037 U00096 1099471..1100010
<313> 6109: 511-530
<400> 6109
GCTGATATTT GGTTACGCAA TGAAAAACAG TGGCCTGTGG CTGGCGGCGC 50
GTAGTGCAAA GACGGCGCAC CGTGAGCAGG AAATCAAAAA TAAAGCGTGA 100
<210> 6110
<211> 100
<212> DNA
<213> Escherichia coli K-12 MG1655
<220> ycdz b1037
<222> 414-513
<223> ycdz b1037 U00096 1099471..1100010
<313> 6110: 463-482
<400> 6110
ACTGGTGTTA CCTTCGCTGG CGCTTGGGCT GATATTTGGT TACGCAATGA 50
AAAACAGTGG CCTGTGGCTG GCGGCGCGTA GTGCAAAGAC GGCGCACCGT 100
<210> 6111
<211> 100
<212> DNA
<213> Escherichia coli K-12 MG1655
<220> ycdz b1037
<222> 353-452
<223> ycdz b1037 U00096 1099471..1100010
<313> 6111: 392-411
<400> 6111
CATTTGTTCC GGGGACGTTT ATAGGCGCAT GCGCGACGTT TGCCGGGCAG 50
GGTGACTGGA AACTGGTGTT ACCTTCGCTG GCGCTTGGGC TGATATTTGG 100
<210> 6112
<211> 100
<212> DNA
<213> Escherichia coli K-12 MG1655
<220> dsda b2367
<222> 1230-1329
<223> dsda b2367 U00096 2477222..2478550
<313> 6112: 1307-1326
<400> 6112
CGCAGAACAA CTGCGTAATA CCACTCATCT GGTGTGGGCG ACGGGAGGTG 50
GAATGGTGCC GGAAGAAGAG ATGAATCAAT ATCTGGCAAA AGGCCGTTAA 100
<210> 6113

<211> 100
<212> DNA
<213> Escherichia coli K-12 MG1655
<220> dsda b2367
<222> 1205-1304
<223> dsda b2367 U00096 2477222..2478550
<313> 6113: 1259-1278
<400> 6113
GTTACCAACA GATGCACGGT TTCAGCGCAG AACAACTGCG TAATACCACT 50
CATCTGGTGT GGGCGACGGG AGGTGGAATG GTGCCGGAAG AAGAGATGAA 100
<210> 6114
<211> 100
<212> DNA
<213> Escherichia coli K-12 MG1655
<220> dsda b2367
<222> 1154-1253
<223> dsda b2367 U00096 2477222..2478550
<313> 6114: 1204-1223
<400> 6114
CTTCGGCACT GGCGGGTATG GCCGGACCTC AGCGCGTGTG TGCATCAGTA 50
AGTTACCAAC AGATGCACGG TTTCAGCGCA GAACAACTGC GTAATACCAC 100
<210> 6115
<211> 100
<212> DNA
<213> Escherichia coli K-12 MG1655
<220> sers b0894
<222> 1184-1283
<223> sers b0894 U00096 938651..939943
<313> 6115: 1239-1258
<400> 6115
TTGGTCGTAC GCTGGTTGCA GTAATGGAAA ACTATCAGCA GGCTGATGGT 50
CGTATTGAAG TACCAGAAGT TCTGCGTCCG TATATGAACG GACTGGAATA 100
<210> 6116
<211> 100
<212> DNA
<213> Escherichia coli K-12 MG1655
<220> sers b0894
<222> 1139-1238
<223> sers b0894 U00096 938651..939943
<313> 6116: 1158-1177
<400> 6116
AGAAAACCCG TCTGGTTCAT ACCCTGAACG GTTCTGGTCT GGCTGTTGGT 50
CGTACGCTGG TTGCAGTAAT GGAAAACTAT CAGCAGGCTG ATGGTCGTAT 100
<210> 6117
<211> 100
<212> DNA
<213> Escherichia coli K-12 MG1655
<220> sers b0894
<222> 1090-1189
<223> sers b0894 U00096 938651..939943
<313> 6117: 1121-1140
<400> 6117
GATTTCCAGG CACGTCGTAT GCAGGCACGT TGCCGCAGCA AGTCGGACAA 50
GAAAACCCGT CTGGTTCATA CCCTGAACGG TTCTGGTCTG GCTGTTGGTC 100
<210> 6118
<211> 100
<212> DNA
<213> Escherichia coli K-12 MG1655
<220> dsdc b2365
<222> 795-894
<223> dsdc b2365 U00096 complement(2474714..2475649)
<313> 6118: 842-861
<400> 6118
TGGTGAGCTC GTCGCGCCGT TTGGCGATAT GACGGTGAAA TGCCATCAGC 50
ATTATTACAT CACCACATTA CCGGGCAGGC AGTGGCCAAA AATTGAAGCG 100
<210> 6119
<211> 100
<212> DNA
<213> Escherichia coli K-12 MG1655
<220> dsdc b2365
<222> 715-814

<223> dsdc b2365 U00096 complement(2474714..2475649)
<313> 6119: 771-790
<400> 6119
GATTTAGCTG TTATTGCCGC GATGAATCAT ATTGGGGTGG CGATGGGAAG  50
AAAACGCCTG GTACAAAAAA GGCTTGCCAG TGGTGAGCTC GTCGCGCCGT  100
<210> 6120
<211> 100
<212> DNA
<213> Escherichia coli K-12 MG1655
<220> dsdc b2365
<222> 660-759
<223> dsdc b2365 U00096 complement(2474714..2475649)
<313> 6120: 713-732
<400> 6120
GCAACATTAT GCGGTTAATT TGCCGACATC TTCTGGAATT GGCTTTGATC  50
GTTCTGATTT AGCTGTTATT GCCGCGATGA ATCATATTGG GGTGGCGATG  100
<210> 6121
<211> 100
<212> DNA
<213> Escherichia coli K-12 MG1655
<220> ycea b1056
<222> 943-1042
<223> ycea b1056 U00096 1116030..1117082
<313> 6121: 972-991
<400> 6121
CCAGAAGAAC AGCGACGCCG TCGGGCAGGA CGTGAAAATG GCAATAAGAT  50
CTTTAATAAG TCTCGTGGAC GTCTGAATAC AACACTGTGC ATTCCTGATC  100
<210> 6122
<211> 100
<212> DNA
<213> Escherichia coli K-12 MG1655
<220> ycea b1056
<222> 808-907
<223> ycea b1056 U00096 1116030..1117082
<313> 6122: 876-895
<400> 6122
TGCGGCGCGC CGTGCGACAG CCATACCAAC TGTAAAAATG ATGGCTGCCA  50
CCTGCTGTTT ATTCAGTGTC CAGTATGCGC GGAAAAATAC AAAGGTTGTT  100
<210> 6123
<211> 100
<212> DNA
<213> Escherichia coli K-12 MG1655
<220> ycea b1056
<222> 747-846
<223> ycea b1056 U00096 1116030..1117082
<313> 6123: 786-805
<400> 6123
TGTTTTTGAC GAGCGGATGG GCGAACGTAT ATCGGATGAG ATTATCGCGC  50
ATTGCCACCA GTGCGGCGCG CCGTGCGACA GCCATACCAA CTGTAAAAAT  100
<210> 6124
<211> 100
<212> DNA
<213> Escherichia coli K-12 MG1655
<220> yceb b1064
<222> 462-561
<223> yceb b1064 U00096 complement(1121936..1122496)
<313> 6124: 518-537
<400> 6124
CCTGCGCGAA GATGGCAGCC AGGGCGAAGC AATGGCGAAA AAACTGGCGA  50
AAGGCATTGA AGTGAAGCCA GGCGAAATTG TCATTCCATT TACTGATTAA  100
<210> 6125
<211> 100
<212> DNA
<213> Escherichia coli K-12 MG1655
<220> yceb b1064
<222> 451-550
<223> yceb b1064 U00096 complement(1121936..1122496)
<313> 6125: 483-502
<400> 6125
CCTGCTTACG TCCTGCGCGA AGATGGCAGC CAGGGCGAAG CAATGGCGAA  50
AAAACTGGCG AAAGGCATTG AAGTGAAGCC AGGCGAAATT GTCATTCCAT  100

<210> 6126
<211> 100
<212> DNA
<213> Escherichia coli K-12 MG1655
<220> yceb b1064
<222> 397-496
<223> yceb b1064 U00096 complement(1121936..1122496)
<313> 6126: 440-459
<400> 6126
CAAACGTTGC TTCCCTATTT GAACCAGGCA TTACGCAATT ACTTTAACCA 50
GCAACCTGCT TACGTCCTGC GCGAAGATGG CAGCCAGGGC GAAGCAATGG 100
<210> 6127
<211> 100
<212> DNA
<213> Escherichia coli K-12 MG1655
<220> ycec b1087
<222> 861-960
<223> ycec b1087 U00096 1144163..1145122
<313> 6127: 907-926
<400> 6127
GAAGTTTACT CATCCGGGGA CCGGTGAGGT GATGCGTATC GAAGCTCCGA 50
TGGATGAAGG TTTGAAGCGT TGTTTGCAAA AGCTGCGTAA CGCGCGCTAA 100
<210> 6128
<211> 100
<212> DNA
<213> Escherichia coli K-12 MG1655
<220> ycec b1087
<222> 772-871
<223> ycec b1087 U00096 1144163..1145122
<313> 6128: 799-818
<400> 6128
GACGATCGCT ACGGTGACCG TGAATTTGAC AGGCAGCTCA CTGAAGCAGG 50
CACGGGATTA AATCGCCTGT TCCTGCACGC TGCAGCGTTG AAGTTTACTC 100
<210> 6129
<211> 100
<212> DNA
<213> Escherichia coli K-12 MG1655
<220> ycec b1087
<222> 700-799
<223> ycec b1087 U00096 1144163..1145122
<313> 6129: 739-758
<400> 6129
TGTAGCCCGG TAACAGGGCG TACTCACCAG ATCCGTGTGC ATACACAATA 50
TGCGGGTCAT CCGATTGCCT TTGACGATCG CTACGGTGAC CGTGAATTTG 100
<210> 6130
<211> 100
<212> DNA
<213> Escherichia coli K-12 MG1655
<220> yced b1089
<222> 423-522
<223> yced b1089 U00096 1146017..1146538
<313> 6130: 491-510
<400> 6130
CTGTGAAGTG TCCGAAGCGG ACATGGTCTT TGGTGAACTG CCTGAAGAAG 50
CGCAAAAGCC AAACCCATTT GCCGTATTAG CCAGCTTAAA GCGTAAGTAA 100
<210> 6131
<211> 100
<212> DNA
<213> Escherichia coli K-12 MG1655
<220> yced b1089
<222> 423-522
<223> yced b1089 U00096 1146017..1146538
<313> 6131: 440-459
<400> 6131
CTGTGAAGTG TCCGAAGCGG ACATGGTCTT TGGTGAACTG CCTGAAGAAG 50
CGCAAAAGCC AAACCCATTT GCCGTATTAG CCAGCTTAAA GCGTAAGTAA 100
<210> 6132
<211> 100
<212> DNA
<213> Escherichia coli K-12 MG1655
<220> yced b1089

<222> 387-486
<223> yced b1089 U00096 1146017..1146538
<313> 6132: 413-432
<400> 6132
CGCCTTGCCG GTAGTTCCGG TGCATGATTC TGAACACTGT GAAGTGTCCG  50
AAGCGGACAT GGTCTTTGGT GAACTGCCTG AAGAAGCGCA AAAGCCAAAC  100
<210> 6133
<211> 100
<212> DNA
<213> Escherichia coli K-12 MG1655
<220> ycee b1054
<222> 1071-1170
<223> ycee b1054 U00096 complement(1113487..1114713)
<313> 6133: 1113-1132
<400> 6133
TATTGGCAAC GTTACCGGAC CATTGATGGG AGCAGCGATT TCAGCGAACT  50
ACGGTTTCAG AGCGGTATTT CTCGTCACCG CTGGCGTAGT GTTATTCAAC  100
<210> 6134
<211> 100
<212> DNA
<213> Escherichia coli K-12 MG1655
<220> ycee b1054
<222> 1038-1137
<223> ycee b1054 U00096 complement(1113487..1114713)
<313> 6134: 1062-1081
<400> 6134
GCGTATCTTC AGCTATAACC AATCGTTTCG TGATATTGGC AACGTTACCG  50
GACCATTGAT GGGAGCAGCG ATTTCAGCGA ACTACGGTTT CAGAGCGGTA  100
<210> 6135
<211> 100
<212> DNA
<213> Escherichia coli K-12 MG1655
<220> ycee b1054
<222> 939-1038
<223> ycee b1054 U00096 complement(1113487..1114713)
<313> 6135: 996-1015
<400> 6135
ACTTGGGATT TTACGTTTTT TGCTCGGTGC CGCCGATGGT GCACTACTCC  50
CCGCCGTACA GACACTGTTG GTTTACAACT CGAGCAACCA GATCGCCGGG  100
<210> 6136
<211> 100
<212> DNA
<213> Escherichia coli K-12 MG1655
<220> ycef b1088
<222> 525-624
<223> ycef b1088 U00096 complement(1145234..1145857)
<313> 6136: 604-623
<400> 6136
GCGTTTAGAG GGGCGTGATC CTAACACGCT GGTTGGTTTA CCGCTAATCG  50
CGCTGTGTCA GATGTTACGT CGGGAAGGGA AAAACCCGCT GATGGGATAG  100
<210> 6137
<211> 100
<212> DNA
<213> Escherichia coli K-12 MG1655
<220> ycef b1088
<222> 525-624
<223> ycef b1088 U00096 complement(1145234..1145857)
<313> 6137: 555-574
<400> 6137
GCGTTTAGAG GGGCGTGATC CTAACACGCT GGTTGGTTTA CCGCTAATCG  50
CGCTGTGTCA GATGTTACGT CGGGAAGGGA AAAACCCGCT GATGGGATAG  100
<210> 6138
<211> 100
<212> DNA
<213> Escherichia coli K-12 MG1655
<220> ycef b1088
<222> 358-457
<223> ycef b1088 U00096 complement(1145234..1145857)
<313> 6138: 384-403
<400> 6138
GCGTTGTTTA ATTCGGCGAA TGGGCATCTG CAAACAGAAG TGGAGCCTTT  50

TGACGTCCAT TTCCGTCACC TGAGCGAGGC GGAGATTGAT AATTACGTGC 100
<210> 6139
<211> 100
<212> DNA
<213> Escherichia coli K-12 MG1655
<220> yceg b1098
<222> 871-970
<223> yceg b1098 U00096 1153335..1154357
<313> 6139: 910-929
<400> 6139
TCGCTGAAGG CTGCTGCGCA TCCGGCAAAA ACGCCGTATC TCTATTTTGT 50
GGCCGATGGT AAAGGTGGTC ACACGTTTAA TACCAATCTT GCCAGTCATA 100
<210> 6140
<211> 100
<212> DNA
<213> Escherichia coli K-12 MG1655
<220> yceg b1098
<222> 765-864
<223> yceg b1098 U00096 1153335..1154357
<313> 6140: 805-824
<400> 6140
TTATAATGGC AAACTTTCTC GTGCAGACCT GGAAACGCCG ACAGCGTATA 50
ACACCTATAC CATTACCGGT CTGCCGCCAG GTGCGATAGC GACGCCGGGG 100
<210> 6141
<211> 100
<212> DNA
<213> Escherichia coli K-12 MG1655
<220> yceg b1098
<222> 681-780
<223> yceg b1098 U00096 1153335..1154357
<313> 6141: 736-755
<400> 6141
GGTTGCCTCA GTATTTATCA ACCGTTTACG CATTGGTATG CGCCTGCAGA 50
CCGACCCGAC CGTGATTTAC GGGATGGGAG AGCGTTATAA TGGCAAACTT 100
<210> 6142
<211> 100
<212> DNA
<213> Escherichia coli K-12 MG1655
<220> yceh b1068
<222> 549-648
<223> yceh b1068 U00096 1125380..1126027
<313> 6142: 625-644
<400> 6142
GGTTGACGGT GATTTACAGG CCCGCGTCGA AGCCCTGGAA ATCGAAGTGG 50
CAGAACTGAA ACAGCGTCTT GATTCGTTGC TGGCCCATCT GGGAGATTAA 100
<210> 6143
<211> 100
<212> DNA
<213> Escherichia coli K-12 MG1655
<220> yceh b1068
<222> 545-644
<223> yceh b1068 U00096 1125380..1126027
<313> 6143: 573-592
<400> 6143
ACGCGGTTGA CGGTGATTTA CAGGCCCGCG TCGAAGCCCT GGAAATCGAA 50
GTGGCAGAAC TGAAACAGCG TCTTGATTCG TTGCTGGCCC ATCTGGGAGA 100
<210> 6144
<211> 100
<212> DNA
<213> Escherichia coli K-12 MG1655
<220> yceh b1068
<222> 443-542
<223> yceh b1068 U00096 1125380..1126027
<313> 6144: 495-514
<400> 6144
TTGTGGTGCG TCTGGCCCGC GAACCGGGTA AACGCGAAAA CCGCTACATG 50
CATCTTTTCA GTGGTGAGGT TGAAGATCAG CCGGCGGTGA CGGATATGTC 100
<210> 6145
<211> 100
<212> DNA
<213> Escherichia coli K-12 MG1655

<220> ycei yceI
<222> 477-576
<223> ycei yceI U00096 complement(1117124..1117699)
<313> 6145: 537-556
<400> 6145
CAAAATTAAG CTCAAGGACT TCAATATCAA GACAGATTTA GGTCCAGCTT 50
CTCAGGAAGT GGATCTGATT ATTTCAGTGG AAGGCGTACA GCAGAAGTAA 100
<210> 6146
<211> 100
<212> DNA
<213> Escherichia coli K-12 MG1655
<220> ycei yceI
<222> 447-546
<223> ycei yceI U00096 complement(1117124..1117699)
<313> 6146: 471-490
<400> 6146
TGGTAAACGT GCAGGCTTCG AGGCCGAAGG CAAAATTAAG CTCAAGGACT 50
TCAATATCAA GACAGATTTA GGTCCAGCTT CTCAGGAAGT GGATCTGATT 100
<210> 6147
<211> 100
<212> DNA
<213> Escherichia coli K-12 MG1655
<220> ycei yceI
<222> 333-432
<223> ycei yceI U00096 complement(1117124..1117699)
<313> 6147: 392-411
<400> 6147
CGTGAAGAAA GACGGTGACG AACTGGATAT TACCGGCGAT CTGACGCTGA 50
ATGGCGTAAC CAAACCCGTC ACGCTGGAAG CGAAATTAAT TGGTCAGGGC 100
<210> 6148
<211> 100
<212> DNA
<213> Escherichia coli K-12 MG1655
<220> ycek b1051
<222> 129-228
<223> ycek b1051 U00096 1112802..1113029
<313> 6148: 179-198
<400> 6148
CTCCGCCTGG CGTTATGTCA CGATCCTTGA TCTGCCATTC TCTCTGGTTT 50
TTGATACTTT ACTGCTGCCG ATCGACATTC ATCATGGCCC GTATGAGTGA 100
<210> 6149
<211> 100
<212> DNA
<213> Escherichia coli K-12 MG1655
<220> ycek b1051
<222> 54-153
<223> ycek b1051 U00096 1112802..1113029
<313> 6149: 115-134
<400> 6149
CATTATTAGT CGCACTATAC CGGGGCAGGG GCATGGCAAC CAATATTATC 50
CCGGTGTGCA ATGGGATGTG CGTGACTCCG CCTGGCGTTA TGTCACGATC 100
<210> 6150
<211> 100
<212> DNA
<213> Escherichia coli K-12 MG1655
<220> ycek b1051
<222> 32-131
<223> ycek b1051 U00096 1112802..1113029
<313> 6150: 65-84
<400> 6150
CCTTGCTGAG TGGCTGTGGC AGCATTATTA GTCGCACTAT ACCGGGGCAG 50
GGGCATGGCA ACCAATATTA TCCCGGTGTG CAATGGGATG TGCGTGACTC 100
<210> 6151
<211> 100
<212> DNA
<213> Escherichia coli K-12 MG1655
<220> ycel b1066
<222> 1132-1231
<223> ycel b1066 U00096 complement(1123341..1124579)
<313> 6151: 1172-1191
<400> 6151

CTTCCGTGGA TGATGCTGGG CATTATTGGC ATCTTCACTT TCCTTGCGCT 50
GGGTTGGCAG TTTAGCCAGA AACGCGCCGC GCGTCGTTTG CTTGAACGCG 100
<210> 6152
<211> 100
<212> DNA
<213> Escherichia coli K-12 MG1655
<220> ycel b1066
<222> 1069-1168
<223> ycel b1066 U00096 complement(1123341..1124579)
<313> 6152: 1111-1130
<400> 6152
GCTATTGGTT ATATCGGTGG CGGCTGGCTG TTTGACCTGG GCAAATCGGC 50
GCACCAGCCA GAGCTTCCGT GGATGATGCT GGGCATTATT GGCATCTTCA 100
<210> 6153
<211> 100
<212> DNA
<213> Escherichia coli K-12 MG1655
<220> ycel b1066
<222> 875-974
<223> ycel b1066 U00096 complement(1123341..1124579)
<313> 6153: 924-943
<400> 6153
TGATAATGTC ATTAAGCATG ATGCCGGTGG GCATGGTCAG CGGCCTGCAA 50
CAACTTTTCA CCCTGATTTG TCTGTTTTAT ATCGGGTCGA TCATTGCCGA 100
<210> 6154
<211> 100
<212> DNA
<213> Escherichia coli K-12 MG1655
<220> b2360 b2360
<222> 704-803
<223> b2360 b2360 U00096 2472052..2472876
<313> 6154: 742-761
<400> 6154
TTCTGCGCAT TATTCAGCTG GAAGCGGTGC AGGAAGATAT GGCTAACGAA 50
TTTCGTGATC TGCTTGTTGA GAAATTCAAA GACAGCAAAG TAGAAACCTT 100
<210> 6155
<211> 100
<212> DNA
<213> Escherichia coli K-12 MG1655
<220> b2360 b2360
<222> 625-724
<223> b2360 b2360 U00096 2472052..2472876
<313> 6155: 660-679
<400> 6155
TGCGTTCCGT TTGAAGGTCT GAAAGAACGT CCGTTTAAAT TACGCCTCAG 50
CATTATCACT GGCGATCGTC CTGTACTGGT TCTGCGCATT ATTCAGCTGG 100
<210> 6156
<211> 100
<212> DNA
<213> Escherichia coli K-12 MG1655
<220> b2360 b2360
<222> 544-643
<223> b2360 b2360 U00096 2472052..2472876
<313> 6156: 575-594
<400> 6156
GACTTCAGCG GCAAACGCTC CCTGATGGAA TCTGTCGAAG CGAAGACCAA 50
AGACATTATG CCAGTGGCAT TTGAATTTAA ATGCGTTCCG TTTGAAGGTC 100
<210> 6157
<211> 100
<212> DNA
<213> Escherichia coli K-12 MG1655
<220> b1631 b1632
<222> 522-621
<223> b1631 b1632 U00096 1708228..1708848
<313> 6157: 593-612
<400> 6157
GATTACTCCC CGCGCGGTGG TTAATGCGGT AAAACGCGCC GGATTGTACG 50
CTCAGACGTT ACCGGCACAA CTTTCTCAAC TTCCTGCCTG TGGAGAATAA 100
<210> 6158
<211> 100
<212> DNA

<213> Escherichia coli K-12 MG1655
<220> b1631 b1632
<222> 514-613
<223> b1631 b1632 U00096 1708228..1708848
<313> 6158: 551-570
<400> 6158
GGCGCGACGA TTACTCCCCG CGCGGTGGTT AATGCGGTAA AACGCGCCGG 50
ATTGTACGCT CAGACGTTAC CGGCACAACT TTCTCAACTT CCTGCCTGTG 100
<210> 6159
<211> 100
<212> DNA
<213> Escherichia coli K-12 MG1655
<220> b1631 b1632
<222> 464-563
<223> b1631 b1632 U00096 1708228..1708848
<313> 6159: 500-519
<400> 6159
ATGCGCACTG GGCGGTGAAG AAAGATGGTG GTGATTTCGA CCAGTTCACC 50
GGCGCGACGA TTACTCCCCG CGCGGTGGTT AATGCGGTAA AACGCGCCGG 100
<210> 6160
<211> 100
<212> DNA
<213> Escherichia coli K-12 MG1655
<220> b2361 b2361
<222> 461-560
<223> b2361 b2361 U00096 2472977..2473540
<313> 6160: 498-517
<400> 6160
CGGCAACAGA GATGTTCAAC GTGATTCCAC TGGCACCGGG TCATGCCTAC 50
GGGATGTTTA TGGAACGTTT TAACGATTTA TCGGAGTTAC GCAAATGCGC 100
<210> 6161
<211> 100
<212> DNA
<213> Escherichia coli K-12 MG1655
<220> b2361 b2361
<222> 428-527
<223> b2361 b2361 U00096 2472977..2473540
<313> 6161: 466-485
<400> 6161
ATGGCTCTTT CTGGCCTGTA CTGGAAGGCA TCCCGGCAAC AGAGATGTTC 50
AACGTGATTC CACTGGCACC GGGTCATGCC TACGGGATGT TTATGGAACG 100
<210> 6162
<211> 100
<212> DNA
<213> Escherichia coli K-12 MG1655
<220> b2361 b2361
<222> 311-410
<223> b2361 b2361 U00096 2472977..2473540
<313> 6162: 368-387
<400> 6162
AAAAAATAGA TGCAGTAATC CGTGAGAAAT ACGGGTTACC TCCTGTTATG 50
AGCACGCCAG TGAAATATGC CGATCTCATT ATGCTGGCAA CCGAACGCCG 100
<210> 6163
<211> 100
<212> DNA
<213> Escherichia coli K-12 MG1655
<220> b2362 b2362
<222> 264-363
<223> b2362 b2362 U00096 2473531..2473893
<313> 6163: 340-359
<400> 6163
AACCGGAGAA TGGCAGTGGA CATTTCAGGT TGGGGACCTT GAAAACGTCA 50
ATATCACTCA CTGGGCAGTA ATGCCAAAAG CACCGGAGGC TGGAGCATAA 100
<210> 6164
<211> 100
<212> DNA
<213> Escherichia coli K-12 MG1655
<220> b2362 b2362
<222> 236-335
<223> b2362 b2362 U00096 2473531..2473893
<313> 6164: 267-286

<400> 6164
CTCTCTGGTA CACCTGGGGA CAAAAAGAAA CCGGAGAATG GCAGTGGACA 50
TTTCAGGTTG GGGACCTTGA AAACGTCAAT ATCACTCACT GGGCAGTAAT 100
<210> 6165
<211> 100
<212> DNA
<213> Escherichia coli K-12 MG1655
<220> b2362 b2362
<222> 180-279
<223> b2362 b2362 U00096 2473531..2473893
<313> 6165: 228-247
<400> 6165
TGAAAGTGTT CTGTTATTTG ATGCTAACGG AGAAGGCTGG CTGATTGGCT 50
GGCGTTCTCT CTGGTACACC TGGGGACAAA AAGAAACCGG AGAATGGCAG 100
<210> 6166
<211> 100
<212> DNA
<213> Escherichia coli K-12 MG1655
<220> yceo b1059
<222> 1-100
<223> yceo b1059 U00096 complement(1118530..1118670)
<313> 6166: 32-51
<400> 6166
ATGAGGCCGT TTTTACAGGA GTATTTAATG CGTCGGCTGT TGCACTATCT 50
CATCAATAAT ATTCGCGAGC ATCTGATGCT TTATCTTTTT CTCTGGGGAT 100
<210> 6167
<211> 100
<212> DNA
<213> Escherichia coli K-12 MG1655
<220> yceo b1059
<222> 3-102
<223> yceo b1059 U00096 complement(1118530..1118670)
<313> 6167: 21-40
<400> 6167
GAGGCCGTTT TTACAGGAGT ATTTAATGCG TCGGCTGTTG CACTATCTCA 50
TCAATAATAT TCGCGAGCAT CTGATGCTTT ATCTTTTTCT CTGGGGATTG 100
<210> 6168
<211> 100
<212> DNA
<213> Escherichia coli K-12 MG1655
<220> yceo b1059
<222> 1-100
<223> yceo b1059 U00096 complement(1118530..1118670)
<313> 6168: 13-32
<400> 6168
ATGAGGCCGT TTTTACAGGA GTATTTAATG CGTCGGCTGT TGCACTATCT 50
CATCAATAAT ATTCGCGAGC ATCTGATGCT TTATCTTTTT CTCTGGGGAT 100
<210> 6169
<211> 100
<212> DNA
<213> Escherichia coli K-12 MG1655
<220> b2363 b2364
<222> 207-306
<223> b2363 b2364 U00096 2473893..2474198
<313> 6169: 263-282
<400> 6169
TAACGAATAC ATGGCATGGG ATATGACTCT CGGTTGGATC ATCTGGCAGC 50
AACGAGCTGG TATCCATTTT TCAACAATGT CACAACAAGA GGTGAAATAA 100
<210> 6170
<211> 100
<212> DNA
<213> Escherichia coli K-12 MG1655
<220> b2363 b2364
<222> 163-262
<223> b2363 b2364 U00096 2473893..2474198
<313> 6170: 226-245
<400> 6170
CGTTTCGGTG ACTTAGTAGA TCGTCGGAGA GCAAAAAACG GCGATAACGA 50
ATACATGGCA TGGGATATGA CTCTCGGTTG GATCATCTGG CAGCAACGAG 100
<210> 6171
<211> 100

<212> DNA
<213> Escherichia coli K-12 MG1655
<220> b2363 b2364
<222> 96-195
<223> b2363 b2364 U00096 2473893..2474198
<313> 6171: 117-136
<400> 6171
AATCGCACTC GTATCTCTGG AAGTAGAGCC AGATGAACGC GAAGCCTATG 50
AATTATTCAT GGAAAAGCGT TTCGGTGACT TAGTAGATCG TCGGAGAGCA 100
<210> 6172
<211> 100
<212> DNA
<213> Escherichia coli K-12 MG1655
<220> ycep b1061
<222> 156-255
<223> ycep b1061 U00096 complement(1119924..1120178)
<313> 6172: 223-242
<400> 6172
AACCACTGAT GTTGCCAGAC AATTTATTTC GATATTAGAA GCAGGAATCG 50
CCAAAATTGA ATCCGGTGAT TTCCAGGTAA ACGAGTATCG GCGTCATTAA 100
<210> 6173
<211> 100
<212> DNA
<213> Escherichia coli K-12 MG1655
<220> ycep b1061
<222> 92-191
<223> ycep b1061 U00096 complement(1119924..1120178)
<313> 6173: 113-132
<400> 6173
TGCACTACCA GACCCCAAAT AAGTCCGAGC AGGAAGGGAC TGAAGTTGGT 50
CAGACGCTCT GGTTAACCAC TGATGTTGCC AGACAATTTA TTTCGATATT 100
<210> 6174
<211> 100
<212> DNA
<213> Escherichia coli K-12 MG1655
<220> ycep b1061
<222> 48-147
<223> ycep b1061 U00096 complement(1119924..1120178)
<313> 6174: 83-102
<400> 6174
GGACATCAGC ACCGTTGATA GCTATGATGC GCTGATGTTG CGTTTGCACT 50
ACCAGACCCC AAATAAGTCC GAGCAGGAAG GGACTGAAGT TGGTCAGACG 100
<210> 6175
<211> 100
<212> DNA
<213> Escherichia coli K-12 MG1655
<220> dsdx b2366
<222> 1239-1338
<223> dsdx b2366 U00096 2475867..2477204
<313> 6175: 1291-1310
<400> 6175
CGCTACGCTC AATGAAACAT TTAAATACTA TACGACAGCG ACATTTATCG 50
CTTCAGTCGT CGCTCTGGCG GGCACATTCC TGCTGTCATT TATCATCTAA 100
<210> 6176
<211> 100
<212> DNA
<213> Escherichia coli K-12 MG1655
<220> dsdx b2366
<222> 1132-1231
<223> dsdx b2366 U00096 2475867..2477204
<313> 6176: 1196-1215
<400> 6176
TATCCCGACA TCAGCCCGGA AATTATTGCG ATTGCTATCG GTTCAGGTGC 50
AATTGGCTGC ACTATCGTTA CGGACTCGCT TTTCTGGCTA GTGAAGCAAT 100
<210> 6177
<211> 100
<212> DNA
<213> Escherichia coli K-12 MG1655
<220> dsdx b2366
<222> 1098-1197
<223> dsdx b2366 U00096 2475867..2477204

<313> 6177: 1139-1158
<400> 6177
GGCAACGGCA ATTGTTGCAC CCATGCTGCC GCTGTATCCC GACATCAGCC 50
CGGAAATTAT TGCGATTGCT ATCGGTTCAG GTGCAATTGG CTGCACTATC 100
<210> 6178
<211> 100
<212> DNA
<213> Escherichia coli K-12 MG1655
<220> ycfa ycfA
<222> 474-573
<223> ycfa ycfA U00096 complement(1207740..1208342)
<313> 6178: 528-547
<400> 6178
GGCCGGACGT AAACTGACGC AGGCAGAAAC AACCAGACTT AACGCTGTGC 50
TGGATTACAT TGACGCGGTG ACGGCAACAG ATACCAGCAC CGCGCCGGAT 100
<210> 6179
<211> 100
<212> DNA
<213> Escherichia coli K-12 MG1655
<220> ycfa ycfA
<222> 437-536
<223> ycfa ycfA U00096 complement(1207740..1208342)
<313> 6179: 478-497
<400> 6179
CAATGGCTTC CATTAGTCTG ATTCAACTGA AATTGCAGGC CGGACGTAAA 50
CTGACGCAGG CAGAAACAAC CAGACTTAAC GCTGTGCTGG ATTACATTGA 100
<210> 6180
<211> 100
<212> DNA
<213> Escherichia coli K-12 MG1655
<220> ycfa ycfA
<222> 386-485
<223> ycfa ycfA U00096 complement(1207740..1208342)
<313> 6180: 421-440
<400> 6180
GCGCCGCAGT AGACGCGGCA GAAGCACAGC GCCAGTCACT GATTGATGCT 50
GCAATGGCTT CCATTAGTCT GATTCAACTG AAATTGCAGG CCGGACGTAA 100
<210> 6181
<211> 100
<212> DNA
<213> Escherichia coli K-12 MG1655
<220> ycfb ycfB
<222> 1053-1152
<223> ycfb ycfB U00096 complement(1191890..1193041)
<313> 6181: 1110-1129
<400> 6181
GGTTGCCGCC GTGACGCCGG GCCAGTCTGC CGTCTTCTAT AACGGTGAAG 50
TGTGCCTCGG TGGCGGTATT ATTGAGCAGC GTCTGCCGCT GCCGGTCTGA 100
<210> 6182
<211> 100
<212> DNA
<213> Escherichia coli K-12 MG1655
<220> ycfb ycfB
<222> 1035-1134
<223> ycfb ycfB U00096 complement(1191890..1193041)
<313> 6182: 1068-1087
<400> 6182
AGTGATTTTC GATGAACCGG TTGCCGCCGT GACGCCGGGC CAGTCTGCCG 50
TCTTCTATAA CGGTGAAGTG TGCCTCGGTG GCGGTATTAT TGAGCAGCGT 100
<210> 6183
<211> 100
<212> DNA
<213> Escherichia coli K-12 MG1655
<220> ycfb ycfB
<222> 968-1067
<223> ycfb ycfB U00096 complement(1191890..1193041)
<313> 6183: 1006-1025
<400> 6183
TAAAAACCCG CTATCGCCAG ACCGATATCC CTTGCACCGT CAAGGCGCTG 50
GACGATGATC GCATTGAAGT GATTTTCGAT GAACCGGTTG CCGCCGTGAC 100
<210> 6184

<211> 100
<212> DNA
<213> Escherichia coli K-12 MG1655
<220> ycfc b1133
<222> 543-642
<223> ycfc b1133 U00096 complement(1191213..1191854)
<313> 6184: 606-625
<400> 6184
GCACCAGGTC GGCGGCGGAC GTCTGCAACT GATGTTTTCT CGTAATCGCC 50
TGACCACTCA GGCAAAACAA ATTCTTGCTC ATTTAACCCC GGAGTTGTGA 100
<210> 6185
<211> 100
<212> DNA
<213> Escherichia coli K-12 MG1655
<220> ycfc b1133
<222> 541-640
<223> ycfc b1133 U00096 complement(1191213..1191854)
<313> 6185: 566-585
<400> 6185
TGGCACCAGG TCGGCGGCGG ACGTCTGCAA CTGATGTTTT CTCGTAATCG 50
CCTGACCACT CAGGCAAAAC AAATTCTTGC TCATTTAACC CCGGAGTTGT 100
<210> 6186
<211> 100
<212> DNA
<213> Escherichia coli K-12 MG1655
<220> ycfc b1133
<222> 487-586
<223> ycfc b1133 U00096 complement(1191213..1191854)
<313> 6186: 515-534
<400> 6186
GTGCAGGCGA AAGTTCGCGC AACCCTGCTG GCAGGCATTC GCGCCGCCGT 50
GCTCTGGCAC CAGGTCGGCG GCGGACGTCT GCAACTGATG TTTTCTCGTA 100
<210> 6187
<211> 100
<212> DNA
<213> Escherichia coli K-12 MG1655
<220> ycfd b1129
<222> 1003-1102
<223> ycfd b1129 U00096 complement(1186342..1187472)
<313> 6187: 1026-1045
<400> 6187
GCACTGGATG CACTCGCCAG CAACATTGCG CTGACTGCGG AGAATTTTGG 50
CGATGCGCTG GAAGATCCGT CATTCCTCGC GATGCTCGCG GCGCTGGTCA 100
<210> 6188
<211> 100
<212> DNA
<213> Escherichia coli K-12 MG1655
<220> ycfd b1129
<222> 868-967
<223> ycfd b1129 U00096 complement(1186342..1187472)
<313> 6188: 882-901
<400> 6188
CCTTATCAGC CGGATGAAAT CTACGATGCG CTGAAACAAG GTGAAGTGCT 50
GGTGCGCCTG GGTGGTCTGC GCGTATTGCG CATTGGCGAC GACGTGTATG 100
<210> 6189
<211> 100
<212> DNA
<213> Escherichia coli K-12 MG1655
<220> ycfd b1129
<222> 793-892
<223> ycfd b1129 U00096 complement(1186342..1187472)
<313> 6189: 855-874
<400> 6189
GAACACTTTA AGCAATGGTT TGGCGAGTTT ATATCCCAGT CACGTCATGA 50
ACTGGATATC GCGCCGCCAG AGCCGCCTTA TCAGCCGGAT GAAATCTACG 100
<210> 6190
<211> 100
<212> DNA
<213> Escherichia coli K-12 MG1655
<220> ycff b1104
<222> 261-360

<223> ycff b1104 U00096 1161108..1161467
<313> 6190: 296-315
<400> 6190
GAATACCAAC CGCCATGGCG GACAAGAGGT TTACCACATC CATATGCACT  50
TGTTGGGTGG CCGTCCGCTG GGACCAATGC TGGCGCATAA AGGTCTGTAA  100
<210> 6191
<211> 100
<212> DNA
<213> Escherichia coli K-12 MG1655
<220> ycff b1104
<222> 160-259
<223> ycff b1104 U00096 1161108..1161467
<313> 6191: 220-239
<400> 6191
GTCTCAGCTG AGCATGAGCA GGCGCTGGGA CGCATGATCA CCGTAGCGGC  50
AAAAATTGCT GAGCAAGAAG GTATTGCCGA AGATGGCTAT CGTCTGATCA  100
<210> 6192
<211> 100
<212> DNA
<213> Escherichia coli K-12 MG1655
<220> ycff b1104
<222> 142-241
<223> ycff b1104 U00096 1161108..1161467
<313> 6192: 173-192
<400> 6192
ATTCCGACTG TGAACGACGT CTCAGCTGAG CATGAGCAGG CGCTGGGACG  50
CATGATCACC GTAGCGGCAA AAATTGCTGA GCAAGAAGGT ATTGCCGAAG  100
<210> 6193
<211> 100
<212> DNA
<213> Escherichia coli K-12 MG1655
<220> ycfh b1101
<222> 699-798
<223> ycfh b1101 U00096 1156000..1156797
<313> 6193: 775-794
<400> 6193
GTTGAAAGGT GTTGCCGTTG AAGAACTGGC GCAGGTAACC ACCGATAACT  50
TCGCCCGTCT GTTTCACATC GACGCTTCCC GCCTTCAATC CATCCGTTGA  100
<210> 6194
<211> 100
<212> DNA
<213> Escherichia coli K-12 MG1655
<220> ycfh b1101
<222> 692-791
<223> ycfh b1101 U00096 1156000..1156797
<313> 6194: 730-749
<400> 6194
TGGCTGTGTT GAAAGGTGTT GCCGTTGAAG AACTGGCGCA GGTAACCACC  50
GATAACTTCG CCCGTCTGTT TCACATCGAC GCTTCCCGCC TTCAATCCAT  100
<210> 6195
<211> 100
<212> DNA
<213> Escherichia coli K-12 MG1655
<220> ycfh b1101
<222> 600-699
<223> ycfh b1101 U00096 1156000..1156797
<313> 6195: 649-668
<400> 6195
ACTGGTGGAA ACTGACTCAC CTTACCTTGC GCCGGTACCG CATCGAGGAA  50
AAGAGAATCA ACCTGCGATG GTTCGTGACG TTGCAGAATA CATGGCTGTG  100
<210> 6196
<211> 100
<212> DNA
<213> Escherichia coli K-12 MG1655
<220> ycfj b1111
<222> 441-540
<223> ycfj b1111 U00096 1166822..1167361
<313> 6196: 498-517
<400> 6196
GATTGGCGAT CAGCAGGGCA AAATCCGCAT GGACCGCGAT CCGGGTACGC  50
AGATCCCGCT AGATAGCAAC GGGCAACTGA TTTTGAATAA CAAAGTATAA  100

<210> 6197
<211> 100
<212> DNA
<213> Escherichia coli K-12 MG1655
<220> ycfj b1111
<222> 428-527
<223> ycfj b1111 U00096 1166822..1167361
<313> 6197: 448-467
<400> 6197
ATGTGACCTA TAAGATTGGC GATCAGCAGG GCAAAATCCG CATGGACCGC 50
GATCCGGGTA CGCAGATCCC GCTAGATAGC AACGGGCAAC TGATTTTGAA 100
<210> 6198
<211> 100
<212> DNA
<213> Escherichia coli K-12 MG1655
<220> ycfj b1111
<222> 320-419
<223> ycfj b1111 U00096 1166822..1167361
<313> 6198: 347-366
<400> 6198
ATGCCGGTAA CCAGATCCAG GGCTCTCTCC AGGAAAGCGA TACTTACACG 50
ACTACGCAAC AGCGTTGTAA AACGGTGTAT GACAAGTCAG AAAAAATGCT 100
<210> 6199
<211> 100
<212> DNA
<213> Escherichia coli K-12 MG1655
<220> ycfk ycfK
<222> 515-614
<223> ycfk ycfK U00096 1206724..1207353
<313> 6199: 554-573
<400> 6199
TCAGCGCACC TATCAATGCA GTGGCAACGG GGGGATATAC AACCACTTCG 50
TTTTTATTAC GGTGCGCAGC TCAAACGGGT AGTTATTACT ATAACTGGAT 100
<210> 6200
<211> 100
<212> DNA
<213> Escherichia coli K-12 MG1655
<220> ycfk ycfK
<222> 471-570
<223> ycfk ycfK U00096 1206724..1207353
<313> 6200: 509-528
<400> 6200
GTTTAATAAT GCCTGTGTTG CCGTTGTTGC AAATTATGAC AATGTCAGCG 50
CACCTATCAA TGCAGTGGCA ACGGGGGGAT ATACAACCAC TTCGTTTTTA 100
<210> 6201
<211> 100
<212> DNA
<213> Escherichia coli K-12 MG1655
<220> ycfk ycfK
<222> 425-524
<223> ycfk ycfK U00096 1206724..1207353
<313> 6201: 460-479
<400> 6201
CATCTGCCTC TGGGGGGGAT GGAAATACAG TTGTATTCCC GGTTGCGTTT 50
AATAATGCCT GTGTTGCCGT TGTTGCAAAT TATGACAATG TCAGCGCACC 100
<210> 6202
<211> 100
<212> DNA
<213> Escherichia coli K-12 MG1655
<220> b1640 b1641
<222> 1011-1110
<223> b1640 b1641 U00096 complement(1716517..1717626)
<313> 6202: 1044-1063
<400> 6202
TGCCTGGCGG ACGCTGGCGG GATTACCAGG AAATCTGCCT TCCGTCACTG 50
GCGCAAGCCA GGAGACGGTA CTGGGGGCTA TTTTCCCCGC TAACCCGTGA 100
<210> 6203
<211> 100
<212> DNA
<213> Escherichia coli K-12 MG1655
<220> b1640 b1641

<222> 881-980
<223> b1640 b1641 U00096 complement(1716517..1717626)
<313> 6203: 943-962
<400> 6203
GTGGTGGAGG TAGTCGTAAT CCGCTACTCA TGGCGCGTCT GGCGGCATTA  50
CTGCCAGGCA CAGAAGTCAC CACCACCGAT GCCGTTGGCA TTAGTGGCGA  100
<210> 6204
<211> 100
<212> DNA
<213> Escherichia coli K-12 MG1655
<220> b1640 b1641
<222> 845-944
<223> b1640 b1641 U00096 complement(1716517..1717626)
<313> 6204: 871-890
<400> 6204
TTTTGTTGAG CGGTGGCTGC GAACGATTGA TGGTATGTGG TGGAGGTAGT  50
CGTAATCCGC TACTCATGGC GCGTCTGGCG GCATTACTGC CAGGCACAGA  100
<210> 6205
<211> 100
<212> DNA
<213> Escherichia coli K-12 MG1655
<220> ycfl b1105
<222> 279-378
<223> ycfl b1105 U00096 1161470..1161847
<313> 6205: 342-361
<400> 6205
GCCACGCAGC GTTACCATTC CCGCACATTC GGCGGTAACG CTGTACGGCA  50
GCGCCAATTT TCTGGGGGCG CACAAAGTCA GACTTTATCT ATATTTGTAA  100
<210> 6206
<211> 100
<212> DNA
<213> Escherichia coli K-12 MG1655
<220> ycfl b1105
<222> 247-346
<223> ycfl b1105 U00096 1161470..1161847
<313> 6206: 287-306
<400> 6206
GCCAGAGGGC TGGAGATGCA TCCTCTGGAA AGGCCACGCA GCGTTACCAT  50
TCCCGCACAT TCGGCGGTAA CGCTGTACGG CAGCGCCAAT TTTCTGGGGG  100
<210> 6207
<211> 100
<212> DNA
<213> Escherichia coli K-12 MG1655
<220> ycfl b1105
<222> 142-241
<223> ycfl b1105 U00096 1161470..1161847
<313> 6207: 195-214
<400> 6207
AAGCCCTTCC TTTCGACGTC TGATATTCAA CCTTCAGCAT CCTCAACGCT  50
TTATAACGAA AGGCAAGAAC CCGTTACCGT TCATTATCGT TTTTACTGGT  100
<210> 6208
<211> 100
<212> DNA
<213> Escherichia coli K-12 MG1655
<220> ycfm b1106
<222> 441-540
<223> ycfm b1106 U00096 1161861..1162502
<313> 6208: 473-492
<400> 6208
GCAACAGTTA GGTTTGTCGC CGCAGGACAG TTTAGGCACC CGTAGTAAAG  50
CCATAGGCAT TGCCCGCAAT GTCGGCGCTC ATTACGTGCT GTACTCCAGC  100
<210> 6209
<211> 100
<212> DNA
<213> Escherichia coli K-12 MG1655
<220> ycfm b1106
<222> 360-459
<223> ycfm b1106 U00096 1161861..1162502
<313> 6209: 397-416
<400> 6209
AGCGACCGAA ACGCTGCGAA ATGCGCTGGC TAATAACGGG AAATTTACCC  50

TGGTTTCCGC CCAGCAACTG TCGATGGCGA AGCAACAGTT AGGTTTGTCG 100
<210> 6210
<211> 100
<212> DNA
<213> Escherichia coli K-12 MG1655
<220> ycfm b1106
<222> 307-406
<223> ycfm b1106 U00096 1161861..1162502
<313> 6210: 364-383
<400> 6210
CTGCTGGTTG ATAGCGTTAA CAACCGTACT AACGGTTCGC TGAATGCCGC 50
AGAAGCGACC GAAACGCTGC GAAATGCGCT GGCTAATAAC GGGAAATTTA 100
<210> 6211
<211> 100
<212> DNA
<213> Escherichia coli K-12 MG1655
<220> ycfn b1107
<222> 653-752
<223> ycfn b1107 U00096 1162483..1163307
<313> 6211: 708-727
<400> 6211
CCACTCGCGC GAAGATTTAT CCGGCGCAAT TATGGCGTCA GGTCAGGCGA 50
TGGTTTCCCT GGCTGCTGAT GCTCAAAGCA GGGTGGTTTG AGTACCGCTG 100
<210> 6212
<211> 100
<212> DNA
<213> Escherichia coli K-12 MG1655
<220> ycfn b1107
<222> 588-687
<223> ycfn b1107 U00096 1162483..1163307
<313> 6212: 622-641
<400> 6212
GCTGGAACTG GCGGCGGTGT GGGTGGAAAA TACTGAACAG CACCGGCAAT 50
TGGTCAATGA CTATGCCACT CGCGCGAAGA TTTATCCGGC GCAATTATGG 100
<210> 6213
<211> 100
<212> DNA
<213> Escherichia coli K-12 MG1655
<220> ycfn b1107
<222> 510-609
<223> ycfn b1107 U00096 1162483..1163307
<313> 6213: 547-566
<400> 6213
CCACGCCGGA AATTTAGTGC ATAGCGCGTC AGGGTTAAAA CTCATCGACT 50
GGGAGTATGC CGGAGATGGT GATATCGCGC TGGAACTGGC GGCGGTGTGG 100
<210> 6214
<211> 100
<212> DNA
<213> Escherichia coli K-12 MG1655
<220> b2372 b2372
<222> 846-945
<223> b2372 b2372 U00096 complement(2487262..2488206)
<313> 6214: 898-917
<400> 6214
TGTCTACACC CGTACTGGTA CAGCGTCATT GGCTGTGAGC GTACTGGGCT 50
TTGTCGTCAC GGCTCCCTTG TGGATTTATG TCAGTCGACT GGTTTCATAA 100
<210> 6215
<211> 100
<212> DNA
<213> Escherichia coli K-12 MG1655
<220> b2372 b2372
<222> 687-786
<223> b2372 b2372 U00096 complement(2487262..2488206)
<313> 6215: 720-739
<400> 6215
AATTGCTTAT AACACCTTCC TGAAGCTGAT TCTGATGCCA CTGGCACTGC 50
TTCTCGTTGG TATGGCATGT CATTTGAACA GCGAACATCT GCAGATGATG 100
<210> 6216
<211> 100
<212> DNA
<213> Escherichia coli K-12 MG1655

<220> b2372 b2372
<222> 583-682
<223> b2372 b2372 U00096 complement(2487262..2488206)
<313> 6216: 617-636
<400> 6216
GCATGGGACC CAACCTTTAA TCTGATTGCG AAGGCTAACT CAGGGGTAGC 50
GGTATTCGCT GCGGGGTTGA CTCTGGCTGC ACATAAATTC GAGTTCAGTG 100
<210> 6217
<211> 100
<212> DNA
<213> Escherichia coli K-12 MG1655
<220> b1643 b1643
<222> 138-237
<223> b1643 b1643 U00096 1719049..1719285
<313> 6217: 197-216
<400> 6217
CTGGATCTAC GCCGGTGACA TCTGGCATCC CTTGTTAATG GATTTATCGC 50
TGTTTGCGAT TTGCGTTTGC CTTGCTCTGG CAATACTGAT TGCGTGGTAA 100
<210> 6218
<211> 100
<212> DNA
<213> Escherichia coli K-12 MG1655
<220> b1643 b1643
<222> 81-180
<223> b1643 b1643 U00096 1719049..1719285
<313> 6218: 105-124
<400> 6218
CAAAGCATTC GCGTTTGGAT TCGTCATCTG GCTTGTCGTA CACCGCCTGC 50
TTCGTGGCTG GATCTACGCC GGTGACATCT GGCATCCCTT GTTAATGGAT 100
<210> 6219
<211> 100
<212> DNA
<213> Escherichia coli K-12 MG1655
<220> b1643 b1643
<222> 26-125
<223> b1643 b1643 U00096 1719049..1719285
<313> 6219: 76-95
<400> 6219
GATTGCCCTT ACAAGATCTG GTGTTCGGTG CGTCCGTCTA CTTTCCTCCG 50
TTTTTCAAAG CATTCGCGTT TGGATTCGTC ATCTGGCTTG TCGTACACCG 100
<210> 6220
<211> 100
<212> DNA
<213> Escherichia coli K-12 MG1655
<220> ycfo b1108
<222> 927-1026
<223> ycfo b1108 U00096 1163318..1164343
<313> 6220: 988-1007
<400> 6220
TTCGCGACAG GAACTGATGG ACTCGGCTCG CTGGAAAGCG ATCAGCACCC 50
GTCTGAATCA GTTACATGAA CGCTGGCAGG AAGAGAAAGC AGGTCACTAA 100
<210> 6221
<211> 100
<212> DNA
<213> Escherichia coli K-12 MG1655
<220> ycfo b1108
<222> 927-1026
<223> ycfo b1108 U00096 1163318..1164343
<313> 6221: 954-973
<400> 6221
TTCGCGACAG GAACTGATGG ACTCGGCTCG CTGGAAAGCG ATCAGCACCC 50
GTCTGAATCA GTTACATGAA CGCTGGCAGG AAGAGAAAGC AGGTCACTAA 100
<210> 6222
<211> 100
<212> DNA
<213> Escherichia coli K-12 MG1655
<220> ycfo b1108
<222> 814-913
<223> ycfo b1108 U00096 1163318..1164343
<313> 6222: 869-888
<400> 6222

GGTTGCGATA TGATCCTGGT CTGCAATAAT CGTAAAGGGG CCGTCAGCGT 50
GTTAGATAAT CTGTCACCGA TCAAGGCAGA ACGTGTTACA CGTTTGTATC 100
<210> 6223
<211> 100
<212> DNA
<213> Escherichia coli K-12 MG1655
<220> b2373 b2373
<222> 1584-1683
<223> b2373 b2373 U00096 complement(2488276..2489970)
<313> 6223: 1613-1632
<400> 6223
CGGTATTCAG TCGCGCAAAC CGACCATTAT TAATGTGGTC ATCGACCCTG 50
CAGCAGGAAC TGAAAGTGGC CATATTACCA AACTTAACCC AAAACAAGTC 100
<210> 6224
<211> 100
<212> DNA
<213> Escherichia coli K-12 MG1655
<220> b2373 b2373
<222> 1529-1628
<223> b2373 b2373 U00096 complement(2488276..2489970)
<313> 6224: 1551-1570
<400> 6224
GTGGCGTTGG CTATAACGTC ACCACGACAG ATGAACTTCG TCATGCTTTA 50
ACCACCGGTA TTCAGTCGCG CAAACCGACC ATTATTAATG TGGTCATCGA 100
<210> 6225
<211> 100
<212> DNA
<213> Escherichia coli K-12 MG1655
<220> b2373 b2373
<222> 1375-1474
<223> b2373 b2373 U00096 complement(2488276..2489970)
<313> 6225: 1434-1453
<400> 6225
ACGATTTGTC GATATAACCT GCCGGTGACG ATCGTTATTT TTAATAATGG 50
CGGCATCTAC AGAGGAGACG GTGTTGATCT CAGTGGCGCT GGTGCACCAT 100
<210> 6226
<211> 100
<212> DNA
<213> Escherichia coli K-12 MG1655
<220> b1644 b1644
<222> 792-891
<223> b1644 b1644 U00096 1719246..1720145
<313> 6226: 841-860
<400> 6226
CTGGGTTCGA CTCGCCCAAC GCGTTCCCGT CCGCTTTGCG CTGGATAAAG 50
TCCCAGGCGA TGTCACGTTG GTGTCGGGCA CCACCTGTAG CATTGCCGTA 100
<210> 6227
<211> 100
<212> DNA
<213> Escherichia coli K-12 MG1655
<220> b1644 b1644
<222> 746-845
<223> b1644 b1644 U00096 1719246..1720145
<313> 6227: 793-812
<400> 6227
TTGAAAGTGA CTCAAGCCTT ATTCCGGATG TTAAACCTAA CGTCCCCTGG 50
GTTCGACTCG CCCAACGCGT TCCCGTCCGC TTTGCGCTGG ATAAAGTCCC 100
<210> 6228
<211> 100
<212> DNA
<213> Escherichia coli K-12 MG1655
<220> b1644 b1644
<222> 691-790
<223> b1644 b1644 U00096 1719246..1720145
<313> 6228: 736-755
<400> 6228
AAAACGTTAC AGGGTCACGT TTCCAGTATC GGTCGGGCGA TTTATGATCA 50
GAGCGTTGAA AGTGACTCAA GCCTTATTCC GGATGTTAAA CCTAACGTCC 100
<210> 6229
<211> 100
<212> DNA

<213> Escherichia coli K-12 MG1655
<220> ycfp b1109
<222> 501-600
<223> ycfp b1109 U00096 1164309..1164908
<313> 6229: 561-580
<400> 6229
TCATTATTAC GAGATTGTCT GGGACGAAGA GCAGACGCAC AAATTCAAGA 50
ATATCTCCCC GCATTTACAG CGCATTAAAG CGTTCAAAAC CCTCGGGTAA 100
<210> 6230
<211> 100
<212> DNA
<213> Escherichia coli K-12 MG1655
<220> ycfp b1109
<222> 490-589
<223> ycfp b1109 U00096 1164309..1164908
<313> 6230: 521-540
<400> 6230
GAAGAGTTGC ATCATTATTA CGAGATTGTC TGGGACGAAG AGCAGACGCA 50
CAAATTCAAG AATATCTCCC CGCATTTACA GCGCATTAAA GCGTTCAAAA 100
<210> 6231
<211> 100
<212> DNA
<213> Escherichia coli K-12 MG1655
<220> ycfp b1109
<222> 447-546
<223> ycfp b1109 U00096 1164309..1164908
<313> 6231: 494-513
<400> 6231
TTTGTCGCGT AATGATGAAG CGCTTAACAG CCAGCGGACA TCTGAAGAGT 50
TGCATCATTA TTACGAGATT GTCTGGGACG AAGAGCAGAC GCACAAATTC 100
<210> 6232
<211> 100
<212> DNA
<213> Escherichia coli K-12 MG1655
<220> b2374 b2374
<222> 1152-1251
<223> b2374 b2374 U00096 complement(2490024..2491274)
<313> 6232: 1225-1244
<400> 6232
AGCTGCGCCG CTATTAGGTG AACATACCGC TGCTGTATTG CAGGAGCTGG 50
GTTATAGCGA CGATGAAATT GCTGCAATGA AGCAAAACCA CGCCATCTGA 100
<210> 6233
<211> 100
<212> DNA
<213> Escherichia coli K-12 MG1655
<220> b2374 b2374
<222> 1124-1223
<223> b2374 b2374 U00096 complement(2490024..2491274)
<313> 6233: 1174-1193
<400> 6233
AATTCTCTGC CTTTACGCCG GATATTAAAG CTGCGCCGCT ATTAGGTGAA 50
CATACCGCTG CTGTATTGCA GGAGCTGGGT TATAGCGACG ATGAAATTGC 100
<210> 6234
<211> 100
<212> DNA
<213> Escherichia coli K-12 MG1655
<220> b2374 b2374
<222> 1091-1190
<223> b2374 b2374 U00096 complement(2490024..2491274)
<313> 6234: 1142-1161
<400> 6234
GTGGAAAATA TCTGACCGTT GGTTGTCCAA TGAAATTCTC TGCCTTTACG 50
CCGGATATTA AAGCTGCGCC GCTATTAGGT GAACATACCG CTGCTGTATT 100
<210> 6235
<211> 100
<212> DNA
<213> Escherichia coli K-12 MG1655
<220> b1645 b1646
<222> 1914-2013
<223> b1645 b1646 U00096 1720145..1722157
<313> 6235: 1978-1997

<400> 6235
TCAACCTGCC GCCCGTGAGC TGGCGGCAAT TGTCTGGCGG CTGTACTGCT 50
CGCTTTCGCA ACTTGAGCAA GCACCACCGC AAGGTACGCT GGCCTCTTAA 100
<210> 6236
<211> 100
<212> DNA
<213> Escherichia coli K-12 MG1655
<220> b1645 b1646
<222> 1863-1962
<223> b1645 b1646 U00096 1720145..1722157
<313> 6236: 1911-1930
<400> 6236
GGCCACACTT GAAGAATTAC AGCGGATTTG CGACAGCCTT GCCCGTCATC 50
ATCAACCTGC CGCCCGTGAG CTGGCGGCAA TTGTCTGGCG GCTGTACTGC 100
<210> 6237
<211> 100
<212> DNA
<213> Escherichia coli K-12 MG1655
<220> b1645 b1646
<222> 1812-1911
<223> b1645 b1646 U00096 1720145..1722157
<313> 6237: 1861-1880
<400> 6237
ACTGTTGCGG GGAGTGATGA GTGAGCGTGG CGTTCAGCAA AAATCACTGG 50
CGGCCACACT TGAAGAATTA CAGCGGATTT GCGACAGCCT TGCCCGTCAT 100
<210> 6238
<211> 100
<212> DNA
<213> Escherichia coli K-12 MG1655
<220> oxyr b3962
<222> 785-884
<223> oxyr b3962 U00096 4156069..4156986
<313> 6238: 801-820
<400> 6238
CGGAACCACG CCGCACTATT GGCCTGGTTT ATCGTCCTGG CTCACCGCTG 50
CGCAGCCGCT ATGAGCAGCT GGCAGAGGCC ATCCGCGCAA GAATGGATGG 100
<210> 6239
<211> 100
<212> DNA
<213> Escherichia coli K-12 MG1655
<220> oxyr b3962
<222> 720-819
<223> oxyr b3962 U00096 4156069..4156986
<313> 6239: 758-777
<400> 6239
GCCAGCGCTG GCTGTGCCGC CGGAGCGCAA ACGCGATGGG GTTGTTTATC 50
TGCCGTGCAT TAAGCCGGAA CCACGCCGCA CTATTGGCCT GGTTTATCGT 100
<210> 6240
<211> 100
<212> DNA
<213> Escherichia coli K-12 MG1655
<220> oxyr b3962
<222> 532-631
<223> oxyr b3962 U00096 4156069..4156986
<313> 6240: 568-587
<400> 6240
CGCGAATGCG TACCGATGGC CGATCTGGCA GGGGAAAAAC TGCTGATGCT 50
GGAAGATGGT CACTGTTTGC GCGATCAGGC AATGGGTTTC TGTTTTGAAG 100
<210> 6241
<211> 100
<212> DNA
<213> Escherichia coli K-12 MG1655
<220> ycfq b1112
<222> 612-711
<223> ycfq b1112 U00096 complement(1167423..1168133)
<313> 6241: 680-699
<400> 6241
TCAGGGGATG TCGATCAGCG CACGCGAAGG TGCATCGCTG GAAAAACTGA 50
TGCAGATTGC CGGAACGACT TTGCGTTTAT GGCCCGAACT GGTGAAATAA 100
<210> 6242
<211> 100

<212> DNA
<213> Escherichia coli K-12 MG1655
<220> ycfq b1112
<222> 557-656
<223> ycfq b1112 U00096 complement(1167423..1168133)
<313> 6242: 615-634
<400> 6242
AAATCCCGCC CCACTGTGAC GTGACACATC TGGCAGAATT CCTTAATTGT 50
ATTATTCAGG GGATGTCGAT CAGCGCACGC GAAGGTGCAT CGCTGGAAAA 100
<210> 6243
<211> 100
<212> DNA
<213> Escherichia coli K-12 MG1655
<220> ycfq b1112
<222> 553-652
<223> ycfq b1112 U00096 complement(1167423..1168133)
<313> 6243: 581-600
<400> 6243
GGGGAAATCC CGCCCCACTG TGACGTGACA CATCTGGCAG AATTCCTTAA 50
TTGTATTATT CAGGGGATGT CGATCAGCGC ACGCGAAGGT GCATCGCTGG 100
<210> 6244
<211> 100
<212> DNA
<213> Escherichia coli K-12 MG1655
<220> b2375 b2375
<222> 537-636
<223> b2375 b2375 U00096 complement(2491787..2492422)
<313> 6244: 600-619
<400> 6244
GAAACTGCTT GATAAAAAGC AATACTACGA GGCAAACCTG GCGCTTAAAG 50
GTGCGGAAGA TGGAATCATC GTCGATAGCG AAGCACTGTT TGTGAACTAA 100
<210> 6245
<211> 100
<212> DNA
<213> Escherichia coli K-12 MG1655
<220> b2375 b2375
<222> 499-598
<223> b2375 b2375 U00096 complement(2491787..2492422)
<313> 6245: 559-578
<400> 6245
CCACTTAAAC AGACTCGTAA TGCACTTGCT GATGCGCAGA AACTGCTTGA 50
TAAAAAGCAA TACTACGAGG CAAACCTGGC GCTTAAAGGT GCGGAAGATG 100
<210> 6246
<211> 100
<212> DNA
<213> Escherichia coli K-12 MG1655
<220> b2375 b2375
<222> 472-571
<223> b2375 b2375 U00096 complement(2491787..2492422)
<313> 6246: 505-524
<400> 6246
GGCGTAATGG AAAACCAATA TCTGATGCCA CTTAAACAGA CTCGTAATGC 50
ACTTGCTGAT GCGCAGAAAC TGCTTGATAA AAAGCAATAC TACGAGGCAA 100
<210> 6247
<211> 100
<212> DNA
<213> Escherichia coli K-12 MG1655
<220> yrha b3444
<222> 318-417
<223> yrha b3444 U00096 3580669..3581085
<313> 6247: 392-411
<400> 6247
CATAAACCCT GATCTACAAG AACGGTTAGT GATCGGGGAT TATAGCATTT 50
CAATATTTAC TTATGACATT AAAGGTGATG CTGCCAACTT ACTGATTTAG 100
<210> 6248
<211> 100
<212> DNA
<213> Escherichia coli K-12 MG1655
<220> yrha b3444
<222> 318-417
<223> yrha b3444 U00096 3580669..3581085

<313> 6248: 384-403
<400> 6248
CATAAACCCT GATCTACAAG AACGGTTAGT GATCGGGGAT TATAGCATTT 50
CAATATTTAC TTATGACATT AAAGGTGATG CTGCCAACTT ACTGATTTAG 100
<210> 6249
<211> 100
<212> DNA
<213> Escherichia coli K-12 MG1655
<220> yrha b3444
<222> 318-417
<223> yrha b3444 U00096 3580669..3581085
<313> 6249: 344-363
<400> 6249
CATAAACCCT GATCTACAAG AACGGTTAGT GATCGGGGAT TATAGCATTT 50
CAATATTTAC TTATGACATT AAAGGTGATG CTGCCAACTT ACTGATTTAG 100
<210> 6250
<211> 100
<212> DNA
<213> Escherichia coli K-12 MG1655
<220> ycfr b1113
<222> 145-244
<223> ycfr b1113 U00096 1168296..1168553
<313> 6250: 190-209
<400> 6250
TCGCTGGAAG AGCAGCTGGC GCAAAAAGCG GATGAGATGG GCGCAAAATC 50
TTTCCGTATT ACTTCTGTAA CCGGTCCGAA TACCCTCCAT GGAACAGCAG 100
<210> 6251
<211> 100
<212> DNA
<213> Escherichia coli K-12 MG1655
<220> ycfr b1113
<222> 109-208
<223> ycfr b1113 U00096 1168296..1168553
<313> 6251: 124-143
<400> 6251
GGTACAATCA GTGCTAACGC GGGGACAAAT CTGGGATCGC TGGAAGAGCA 50
GCTGGCGCAA AAAGCGGATG AGATGGGCGC AAAATCTTTC CGTATTACTT 100
<210> 6252
<211> 100
<212> DNA
<213> Escherichia coli K-12 MG1655
<220> ycfr b1113
<222> 49-148
<223> ycfr b1113 U00096 1168296..1168553
<313> 6252: 95-114
<400> 6252
TCATTTGCCA GCTTTGCGGC TGTCGAAGTT CAGTCAACGC CAGAAGGCCA 50
ACAAAAAGTC GGTACAATCA GTGCTAACGC GGGGACAAAT CTGGGATCGC 100
<210> 6253
<211> 100
<212> DNA
<213> Escherichia coli K-12 MG1655
<220> b2376 b2376
<222> 177-276
<223> b2376 b2376 U00096 2492718..2492993
<313> 6253: 231-250
<400> 6253
GTTTAAGATG AGAGAAGATG CGCCACCATT AAACCTCACA GAAGAAATGC 50
CGCCCCCTTT TCCGACAAAG GCGAATTATC TTATTCATCC TGTGCGATAG 100
<210> 6254
<211> 100
<212> DNA
<213> Escherichia coli K-12 MG1655
<220> b2376 b2376
<222> 161-260
<223> b2376 b2376 U00096 2492718..2492993
<313> 6254: 198-217
<400> 6254
CATCACAGGC GATTTTGTTT AAGATGAGAG AAGATGCGCC ACCATTAAAC 50
CTCACAGAAG AAATGCCGCC CCCTTTTCCG ACAAAGGCGA ATTATCTTAT 100
<210> 6255

<211> 100
<212> DNA
<213> Escherichia coli K-12 MG1655
<220> b2376 b2376
<222> 95-194
<223> b2376 b2376 U00096 2492718..2492993
<313> 6255: 143-162
<400> 6255
AGAGGAGCTA TAAAGAGACT GAAAAAACAT CAGCGATGAC GACCACACAC 50
TCTACAAAAC TTCAGCCATC ACAGGCGATT TTGTTTAAGA TGAGAGAAGA 100
<210> 6256
<211> 100
<212> DNA
<213> Escherichia coli K-12 MG1655
<220> b1647 b1647
<222> 798-897
<223> b1647 b1647 U00096 complement(1722760..1723656)
<313> 6256: 858-877
<400> 6256
TGAGCGCGTA CGGGCAGCTG TCGAAGCAGA AACACTGAAA ATGACCCGTC 50
AACAATGGTT TCGTATCCGT AAAGCGGCAC TGGGGTACGA CGTACCGTAA 100
<210> 6257
<211> 100
<212> DNA
<213> Escherichia coli K-12 MG1655
<220> b1647 b1647
<222> 725-824
<223> b1647 b1647 U00096 complement(1722760..1723656)
<313> 6257: 784-803
<400> 6257
TTGAACAGGT GGTTAACGCC TGGGTATTAC GTTTACCATC GCAGCCGCTG 50
CCAATTATCG GTTCAGGTAA AATTGAGCGC GTACGGGCAG CTGTCGAAGC 100
<210> 6258
<211> 100
<212> DNA
<213> Escherichia coli K-12 MG1655
<220> b1647 b1647
<222> 665-764
<223> b1647 b1647 U00096 complement(1722760..1723656)
<313> 6258: 685-704
<400> 6258
ATTTCCAGCC GCTGCGTGAT GAACTGGCTG TGGTGGCAGA GGAGTTAAAC 50
GCGGGCTCGA TTGAACAGGT GGTTAACGCC TGGGTATTAC GTTTACCATC 100
<210> 6259
<211> 100
<212> DNA
<213> Escherichia coli K-12 MG1655
<220> yrhb b3447
<222> 186-285
<223> yrhb b3447 U00096 3582390..3582674
<313> 6259: 257-276
<400> 6259
TAAAGACAGT GGTGAAATTC ATTCTCTGGG AACGGCAAAA CCGCTGGAAG 50
AATATCTACA GGATTACGAA ATAAAAAAGG CTACCTTCGG CTTGCCCTGA 100
<210> 6260
<211> 100
<212> DNA
<213> Escherichia coli K-12 MG1655
<220> yrhb b3447
<222> 186-285
<223> yrhb b3447 U00096 3582390..3582674
<313> 6260: 206-225
<400> 6260
TAAAGACAGT GGTGAAATTC ATTCTCTGGG AACGGCAAAA CCGCTGGAAG 50
AATATCTACA GGATTACGAA ATAAAAAAGG CTACCTTCGG CTTGCCCTGA 100
<210> 6261
<211> 100
<212> DNA
<213> Escherichia coli K-12 MG1655
<220> yrhb b3447
<222> 57-156

<223> yrhb b3447 U00096 3582390..3582674
<313> 6261: 90-109
<400> 6261
TCTCCCGGTC GTCATTACTC TACATGGACG CTTTAGCCAG GGCTGGTATT 50
TCTGTTTCGA AGCACGAGAA TTTCTCGAAA CTGGAGATGA GGCCGCGCGC 100
<210> 6262
<211> 100
<212> DNA
<213> Escherichia coli K-12 MG1655
<220> ycfs b1114
<222> 817-916
<223> ycfs b1114 U00096 complement(1168635..1169597)
<313> 6262: 879-898
<400> 6262
CTGCTGCCAA TTACGCTGAA TAGCGCAATG CAATCATTTA AAGATGCAGC 50
ACAAACTGAC GCTGAAGTGA TGCAACATGT GATGGATGTC CGTTCCGGGA 100
<210> 6263
<211> 100
<212> DNA
<213> Escherichia coli K-12 MG1655
<220> ycfs b1114
<222> 793-892
<223> ycfs b1114 U00096 complement(1168635..1169597)
<313> 6263: 831-850
<400> 6263
GAGAAGATTG ATGACGATCC GCAGCTGCTG CCAATTACGC TGAATAGCGC 50
AATGCAATCA TTTAAAGATG CAGCACAAAC TGACGCTGAA GTGATGCAAC 100
<210> 6264
<211> 100
<212> DNA
<213> Escherichia coli K-12 MG1655
<220> ycfs b1114
<222> 742-841
<223> ycfs b1114 U00096 complement(1168635..1169597)
<313> 6264: 786-805
<400> 6264
TCTGCCGAAC CAAACGGTGC GCGTCTGGTT GAAGTACATC AGCCGCTGTC 50
AGAGAAGATT GATGACGATC CGCAGCTGCT GCCAATTACG CTGAATAGCG 100
<210> 6265
<211> 100
<212> DNA
<213> Escherichia coli K-12 MG1655
<220> b2377 b2378
<222> 144-243
<223> b2377 b2378 U00096 complement(2493070..2493312)
<313> 6265: 209-228
<400> 6265
GTTAACTGGC GGTAATGAAC TAGTGACGGG GATATTTATG TTTCTTGCCC 50
CGGCTTGTGG CTTGATGATT CGCTTTATGG TGGGGTATGG CAGGCGGTAG 100
<210> 6266
<211> 100
<212> DNA
<213> Escherichia coli K-12 MG1655
<220> b2377 b2378
<222> 48-147
<223> b2377 b2378 U00096 complement(2493070..2493312)
<313> 6266: 104-123
<400> 6266
CGGCTACATC GGTCAGGTAC TGAACGTGGT ATCGGCGGTC TCTTCTTTCT 50
TCGGCATGGT GATCCTCGCT GCACTCATTT ATTACTTCAC CATGTGGTTA 100
<210> 6267
<211> 100
<212> DNA
<213> Escherichia coli K-12 MG1655
<220> b2377 b2378
<222> 42-141
<223> b2377 b2378 U00096 complement(2493070..2493312)
<313> 6267: 66-85
<400> 6267
CGTAAGCGGC TACATCGGTC AGGTACTGAA CGTGGTATCG GCGGTCTCTT 50
CTTTCTTCGG CATGGTGATC CTCGCTGCAC TCATTTATTA CTTCACCATG 100

<210> 6268
<211> 100
<212> DNA
<213> Escherichia coli K-12 MG1655
<220> b1648 b1648
<222> 279-378
<223> b1648 b1648 U00096 complement(1723705..1724082)
<313> 6268: 350-369
<400> 6268
ACAAGAGGTG CTGCGCCTTT GTCGGCAGCG ATTAATGCGC AAATTACGCG  50
CAAATAAGCC AGCGTCATCA GACGAACCGG AGCAACCATC ACTCTTTTGA  100
<210> 6269
<211> 100
<212> DNA
<213> Escherichia coli K-12 MG1655
<220> b1648 b1648
<222> 244-343
<223> b1648 b1648 U00096 complement(1723705..1724082)
<313> 6269: 269-288
<400> 6269
CGTTTTAACT GGAATAAAAT GAGCGATGGC GAAAAACAAG AGGTGCTGCG  50
CCTTTGTCGG CAGCGATTAA TGCGCAAATT ACGCGCAAAT AAGCCAGCGT  100
<210> 6270
<211> 100
<212> DNA
<213> Escherichia coli K-12 MG1655
<220> b1648 b1648
<222> 120-219
<223> b1648 b1648 U00096 complement(1723705..1724082)
<313> 6270: 180-199
<400> 6270
CTTATCTGAG GAGGGCGAAG TGGCGGAGCA ATTAGAGTTC TTTCCCGTCC  50
AGAGCCCGTG CCGGGGAATT TGCCAGTCTG ACGAACGCGG GTTTTGTCGC  100
<210> 6271
<211> 100
<212> DNA
<213> Escherichia coli K-12 MG1655
<220> b0919 b0920
<222> 795-894
<223> b0919 b0920 U00096 970975..971868
<313> 6271: 858-877
<400> 6271
TGTGTTATGG GATGAAGTTG CGCAACTGGT TAATACCGGA CGATTTAGTC  50
GGCGCAACTT CGATCTGGCA TCAAAATCAC TCTTGCCGTG GCTCGCCTGA  100
<210> 6272
<211> 100
<212> DNA
<213> Escherichia coli K-12 MG1655
<220> b0919 b0920
<222> 652-751
<223> b0919 b0920 U00096 970975..971868
<313> 6272: 701-720
<400> 6272
GTCGGGCCGG GACTAATGCT TTGGGCACCG CGAGAATACG AACTGTTCCG  50
ACTAATGGAT AATTCTCTGG CGGAAGATTT GCTCTGGAGT TACCTGCAAC  100
<210> 6273
<211> 100
<212> DNA
<213> Escherichia coli K-12 MG1655
<220> b0919 b0920
<222> 641-740
<223> b0919 b0920 U00096 970975..971868
<313> 6273: 664-683
<400> 6273
TACTGGCGAT GGTCGGGCCG GGACTAATGC TTTGGGCACC GCGAGAATAC  50
GAACTGTTCC GACTAATGGA TAATTCTCTG GCGGAAGATT TGCTCTGGAG  100
<210> 6274
<211> 100
<212> DNA
<213> Escherichia coli K-12 MG1655
<220> ycft b1116

<222> 975-1074
<223> ycft b1116 U00096 complement(1173315..1174388)
<313> 6274: 1041-1060
<400> 6274
TGTTGCGGGC TTGCTGGTAA GAAAACTTTC ACAGCGTGCA TTCAGCGATC 50
TGTTGTTCTC CCCGCCTTCA CTGCCCGCTG CCGTCAGTTA CTCCCGCTAA 100
<210> 6275
<211> 100
<212> DNA
<213> Escherichia coli K-12 MG1655
<220> ycft b1116
<222> 956-1055
<223> ycft b1116 U00096 complement(1173315..1174388)
<313> 6275: 1011-1030
<400> 6275
TCAGTTTGTT CATCTGTACT GTTGCGGGCT TGCTGGTAAG AAAACTTTCA 50
CAGCGTGCAT TCAGCGATCT GTTGTTCTCC CCGCCTTCAC TGCCCGCTGC 100
<210> 6276
<211> 100
<212> DNA
<213> Escherichia coli K-12 MG1655
<220> ycft b1116
<222> 881-980
<223> ycft b1116 U00096 complement(1173315..1174388)
<313> 6276: 930-949
<400> 6276
CTCTGCTTGC GCAAATGAAC GCAGCACGCT GGTCGCCGCA AGTCGAACTG 50
ACACTCCTGC TGGTTTATCC CTTTGTCAGT TTGTTCATCT GTACTGTTGC 100
<210> 6277
<211> 100
<212> DNA
<213> Escherichia coli K-12 MG1655
<220> b1649 b1650
<222> 501-600
<223> b1649 b1650 U00096 1724047..1724646
<313> 6277: 575-594
<400> 6277
GCTTGGCGCG AATCTGCAGG CCAAAATTTC GCGCAGTTTC GAGCCACTGG 50
AAAACGCGCT GGCCCATGTA AAAAACATTA TTGCGACGCC TGCCGTTTAG 100
<210> 6278
<211> 100
<212> DNA
<213> Escherichia coli K-12 MG1655
<220> b1649 b1650
<222> 501-600
<223> b1649 b1650 U00096 1724047..1724646
<313> 6278: 531-550
<400> 6278
GCTTGGCGCG AATCTGCAGG CCAAAATTTC GCGCAGTTTC GAGCCACTGG 50
AAAACGCGCT GGCCCATGTA AAAAACATTA TTGCGACGCC TGCCGTTTAG 100
<210> 6279
<211> 100
<212> DNA
<213> Escherichia coli K-12 MG1655
<220> b1649 b1650
<222> 284-383
<223> b1649 b1650 U00096 1724047..1724646
<313> 6279: 336-355
<400> 6279
ACCAGTTTTG CCAACATGGA ACCATCAGTG GTTGCCTGAC AGTAAAACTC 50
TCTGCCGAAG TGTGCGATCT GTCAGAAGAT ATGCGCAGCG CGATGGATAA 100
<210> 6280
<211> 100
<212> DNA
<213> Escherichia coli K-12 MG1655
<220> ycfu b1117
<222> 1055-1154
<223> ycfu b1117 U00096 1174650..1175849
<313> 6280: 1081-1100
<400> 6280
ATGGCGCGGC GCTGCCGGTG GCTATCGAAC CTTTACAGGT CATTGTTATT 50

GCGCTGGTGG CGATGGCTAT CGCGCTGCTG TCTACGCTTT ACCCTTCATG 100
<210> 6281
<211> 100
<212> DNA
<213> Escherichia coli K-12 MG1655
<220> ycfu b1117
<222> 970-1069
<223> ycfu b1117 U00096 1174650..1175849
<313> 6281: 1007-1026
<400> 6281
ATCGGTGCGA TCCTCGGAGC GGCGCTTGGC GCCCTGCTTG CCAGCCAGTT 50
AAATAATCTG ATGCCGATAA TCGGCGTCCT GCTTGATGGC GCGGCGCTGC 100
<210> 6282
<211> 100
<212> DNA
<213> Escherichia coli K-12 MG1655
<220> ycfu b1117
<222> 874-973
<223> ycfu b1117 U00096 1174650..1175849
<313> 6282: 913-932
<400> 6282
GAGAAGCAGG GCGAAGTAGC GATCCTGCAA ACGCAAGGCT TAACTCCGCG 50
ACAAATCATG ATGGTCTTTA TGGTGCAAGG GGCCAGCGCC GGGATTATCG 100
<210> 6283
<211> 100
<212> DNA
<213> Escherichia coli K-12 MG1655
<220> b2379 b2379
<222> 1140-1239
<223> b2379 b2379 U00096 complement(2495077..2496315)
<313> 6283: 1213-1232
<400> 6283
CGACCGTATT CGTCAGGCCA TCAGAGGGAT TAAAGCGATG TTCCGTGCCG 50
ACGGTTTACT ACCCGCCAGC AGCAAACATA TTCACGAAAA CGCGGAATAA 100
<210> 6284
<211> 100
<212> DNA
<213> Escherichia coli K-12 MG1655
<220> b2379 b2379
<222> 1122-1221
<223> b2379 b2379 U00096 complement(2495077..2496315)
<313> 6284: 1168-1187
<400> 6284
TGCACTGATT GAAAACCGCG ACCGTATTCG TCAGGCCATC AGAGGGATTA 50
AAGCGATGTT CCGTGCCGAC GGTTTACTAC CCGCCAGCAG CAAACATATT 100
<210> 6285
<211> 100
<212> DNA
<213> Escherichia coli K-12 MG1655
<220> b2379 b2379
<222> 1093-1192
<223> b2379 b2379 U00096 complement(2495077..2496315)
<313> 6285: 1130-1149
<400> 6285
GGCGACTATG GTGATACGCA TGTTCGCTTT GCACTGATTG AAAACCGCGA 50
CCGTATTCGT CAGGCCATCA GAGGGATTAA AGCGATGTTC CGTGCCGACG 100
<210> 6286
<211> 100
<212> DNA
<213> Escherichia coli K-12 MG1655
<220> ycfv b1118
<222> 588-687
<223> ycfv b1118 U00096 1175857..1176543
<313> 6286: 652-671
<400> 6286
GGTTACTCAC GACCTGCAAC TGGCGAAACG TATGAGCCGC CAGTTAGAAA 50
TGCGTGATGG TCGTCTGACG GCGGAACTGA GCCTGATGGG GGCGGAGTAA 100
<210> 6287
<211> 100
<212> DNA
<213> Escherichia coli K-12 MG1655

<220> ycfv b1118
<222> 568-667
<223> ycfv b1118 U00096 1175857..1176543
<313> 6287: 618-637
<400> 6287
CAGGGCACCG CCTTCCTGGT GGTTACTCAC GACCTGCAAC TGGCGAAACG 50
TATGAGCCGC CAGTTAGAAA TGCGTGATGG TCGTCTGACG GCGGAACTGA 100
<210> 6288
<211> 100
<212> DNA
<213> Escherichia coli K-12 MG1655
<220> ycfv b1118
<222> 517-616
<223> ycfv b1118 U00096 1175857..1176543
<313> 6288: 541-560
<400> 6288
GCGCGTAACG CCGACAGCAT CTTCCAGTTG CTTGGGGAAT TGAATCGCTT 50
GCAGGGCACC GCCTTCCTGG TGGTTACTCA CGACCTGCAA CTGGCGAAAC 100
<210> 6289
<211> 100
<212> DNA
<213> Escherichia coli K-12 MG1655
<220> ycfw b1119
<222> 1146-1245
<223> ycfw b1119 U00096 1176543..1177787
<313> 6289: 1208-1227
<400> 6289
CGTACTGGTC ACAGCATTGT TGCTGAGTCT TTTGGCAAGT TGGTATCCGG 50
CGCGGCGCGC CAGTAATATT GACCCTGCGC GAGTCCTTAG CGGCCAGTAA 100
<210> 6290
<211> 100
<212> DNA
<213> Escherichia coli K-12 MG1655
<220> ycfw b1119
<222> 1114-1213
<223> ycfw b1119 U00096 1176543..1177787
<313> 6290: 1143-1162
<400> 6290
CCATCGGAAT TGCACTGGCT GGACGTCTTC TACGTACTGG TCACAGCATT 50
GTTGCTGAGT CTTTTGGCAA GTTGGTATCC GGCGCGGCGC GCCAGTAATA 100
<210> 6291
<211> 100
<212> DNA
<213> Escherichia coli K-12 MG1655
<220> ycfw b1119
<222> 959-1058
<223> ycfw b1119 U00096 1176543..1177787
<313> 6291: 992-1011
<400> 6291
TCTGGTATGG ATTGCTGGCA GGGCTGTTCG GCAGCCTGTG TGGTGTGATT 50
ATCGGCGTAG TGGTTTCACT GCAACTTACC CCGATTATTG AGTGGATTGA 100
<210> 6292
<211> 100
<212> DNA
<213> Escherichia coli K-12 MG1655
<220> ycfx b1120
<222> 813-912
<223> ycfx b1120 U00096 1177816..1178727
<313> 6292: 881-900
<400> 6292
TCATCTCTTA CCTGTAGCTC GTGTTCCGCG CATTGAACGC GCGCGCCACG 50
GTGATGCGGG AGGAATGCGT GGTGCGGCCT TCCTACATCT AACCGATTAA 100
<210> 6293
<211> 100
<212> DNA
<213> Escherichia coli K-12 MG1655
<220> ycfx b1120
<222> 768-867
<223> ycfx b1120 U00096 1177816..1178727
<313> 6293: 829-848
<400> 6293

GAATTTCCCG GCAATCACAA CGCAACTGGC GGACAGGCTG CCTCGTCATC 50
TCTTACCTGT AGCTCGTGTT CCGCGCATTG AACGCGCGCG CCACGGTGAT 100
<210> 6294
<211> 100
<212> DNA
<213> Escherichia coli K-12 MG1655
<220> ycfx b1120
<222> 753-852
<223> ycfx b1120 U00096 1177816..1178727
<313> 6294: 771-790
<400> 6294
TGGTGGTGGC TTATCGAATT TCCCGGCAAT CACAACGCAA CTGGCGGACA 50
GGCTGCCTCG TCATCTCTTA CCTGTAGCTC GTGTTCCGCG CATTGAACGC 100
<210> 6295
<211> 100
<212> DNA
<213> Escherichia coli K-12 MG1655
<220> ycgb b1189
<222> 1434-1533
<223> ycgb b1189 U00096 complement(1234932..1236464)
<313> 6295: 1510-1529
<400> 6295
GCATCGCCTG TGGGGATTTG ATGTGATGCT CGAACAGCAA AACGAAGACG 50
GCAGCATCGA GTTGCTGGAA CGTTGCCCGC CAAGAATGGG AAATCTGTAA 100
<210> 6296
<211> 100
<212> DNA
<213> Escherichia coli K-12 MG1655
<220> ycgb b1189
<222> 1434-1533
<223> ycgb b1189 U00096 complement(1234932..1236464)
<313> 6296: 1456-1475
<400> 6296
GCATCGCCTG TGGGGATTTG ATGTGATGCT CGAACAGCAA AACGAAGACG 50
GCAGCATCGA GTTGCTGGAA CGTTGCCCGC CAAGAATGGG AAATCTGTAA 100
<210> 6297
<211> 100
<212> DNA
<213> Escherichia coli K-12 MG1655
<220> ycgb b1189
<222> 1357-1456
<223> ycgb b1189 U00096 complement(1234932..1236464)
<313> 6297: 1410-1429
<400> 6297
CGTTCGCTGA CGCTGCGTTA TATTCCACAT AATCGCGCAC CGCTGGATCG 50
GGGGCGCAAA GAAGTCCTGA AGCATGTGCA TCGCCTGTGG GGATTTGATG 100
<210> 6298
<211> 100
<212> DNA
<213> Escherichia coli K-12 MG1655
<220> ycgc ycgC
<222> 1323-1422
<223> ycgc ycgC U00096 complement(1246919..1248340)
<313> 6298: 1385-1404
<400> 6298
TGGCTGGATT TGCCAGCAGG GTGAGAAACT GTATGCGATA CAACCAGAAG 50
AAACGCTAAC GCTGGACGTT AAAACGCAAC GTTTCAACCG TCAGGGTTAA 100
<210> 6299
<211> 100
<212> DNA
<213> Escherichia coli K-12 MG1655
<220> ycgc ycgC
<222> 1311-1410
<223> ycgc ycgC U00096 complement(1246919..1248340)
<313> 6299: 1352-1371
<400> 6299
TGAACTGGGG ATTGGCTGGA TTTGCCAGCA GGGTGAGAAA CTGTATGCGA 50
TACAACCAGA AGAAACGCTA ACGCTGGACG TTAAAACGCA ACGTTTCAAC 100
<210> 6300
<211> 100
<212> DNA

<213> Escherichia coli K-12 MG1655
<220> ycgc ycgC
<222> 1252-1351
<223> ycgc ycgC U00096 complement(1246919..1248340)
<313> 6300: 1297-1316
<400> 6300
GTAAAAGGTA TCTGCCTTAG CGCCGGAAGT CCGGTATCCC ACAGCGCCCT 50
AATCGCCCGT GAACTGGGGA TTGGCTGGAT TTGCCAGCAG GGTGAGAAAC 100
<210> 6301
<211> 100
<212> DNA
<213> Escherichia coli K-12 MG1655
<220> ycge ycgE
<222> 633-732
<223> ycge ycgE U00096 complement(1212551..1213282)
<313> 6301: 696-715
<400> 6301
ATTGATTGTC TGGACAGGCA TGGCACCAAC GAGAAGGCAA AACGAACTTT 50
TACAACACTG GGGTGAGCAA GGTTATAAAG TCATCTTTCA TGCCCCCTAA 100
<210> 6302
<211> 100
<212> DNA
<213> Escherichia coli K-12 MG1655
<220> ycge ycgE
<222> 613-712
<223> ycge ycgE U00096 complement(1212551..1213282)
<313> 6302: 656-675
<400> 6302
GAACTGTTCC CGACGCAAAC ATTGATTGTC TGGACAGGCA TGGCACCAAC 50
GAGAAGGCAA AACGAACTTT TACAACACTG GGGTGAGCAA GGTTATAAAG 100
<210> 6303
<211> 100
<212> DNA
<213> Escherichia coli K-12 MG1655
<220> ycge ycgE
<222> 571-670
<223> ycge ycgE U00096 complement(1212551..1213282)
<313> 6303: 611-630
<400> 6303
CACATTTCTG TTCTTGCTGA TCCCATTGAA TCGCCGCGCC CGGAACTGTT 50
CCCGACGCAA ACATTGATTG TCTGGACAGG CATGGCACCA ACGAGAAGGC 100
<210> 6304
<211> 100
<212> DNA
<213> Escherichia coli K-12 MG1655
<220> ycgj b1178
<222> 258-357
<223> ycgj b1178 U00096 1225823..1226191
<313> 6304: 310-329
<400> 6304
CGATGTTAAA GAAAAATTAT GTCGTGATGA TCGTTATTTT GGTGTGGATG 50
GTAAGCGGAG TGGAAAAATC AATCAAACCA CCACAAAGAT GTTATTTATG 100
<210> 6305
<211> 100
<212> DNA
<213> Escherichia coli K-12 MG1655
<220> ycgj b1178
<222> 150-249
<223> ycgj b1178 U00096 1225823..1226191
<313> 6305: 188-207
<400> 6305
TTCCCCAGAG TTAACAAGGA AATATCTCGG CGAAAAAGCC GCTGAAAACT 50
TACAATCATT ACAAGGCTAC GATCCCAGCG AATTTACATT CGCTAACGGT 100
<210> 6306
<211> 100
<212> DNA
<213> Escherichia coli K-12 MG1655
<220> ycgj b1178
<222> 118-217
<223> ycgj b1178 U00096 1225823..1226191
<313> 6306: 164-183

<400> 6306
GACCCGTATA TTTGTGTGAA CTCAGATGGC ATTTCCCCAG AGTTAACAAG 50
GAAATATCTC GGCGAAAAAG CCGCTGAAAA CTTACAATCA TTACAAGGCT 100
<210> 6307
<211> 100
<212> DNA
<213> Escherichia coli K-12 MG1655
<220> iles b0027
<222> 2649-2748
<223> iles b0027 U00096 22391..25207
<313> 6307: 2690-2709
<400> 6307
CGAAGTACTC AAAGGGCTGA AAGTCGCGTT GAGTAAAGCC GAAGGTGAGA 50
AGTGCCCACG CTGCTGGCAC TACACCCAGG ATGTCGGCAA GGTGGCGGAA 100
<210> 6308
<211> 100
<212> DNA
<213> Escherichia coli K-12 MG1655
<220> iles b0027
<222> 2609-2708
<223> iles b0027 U00096 22391..25207
<313> 6308: 2639-2658
<400> 6308
TTGCAGACTA TAACGACGCA CCTGCTGATG CTCAGCAGAG CGAAGTACTC 50
AAAGGGCTGA AAGTCGCGTT GAGTAAAGCC GAAGGTGAGA AGTGCCCACG 100
<210> 6309
<211> 100
<212> DNA
<213> Escherichia coli K-12 MG1655
<220> iles b0027
<222> 2573-2672
<223> iles b0027 U00096 22391..25207
<313> 6309: 2615-2634
<400> 6309
AATTACGATT TGTCCTGTTG ACCTCCGGCG CTACCGTTGC AGACTATAAC 50
GACGCACCTG CTGATGCTCA GCAGAGCGAA GTACTCAAAG GGCTGAAAGT 100
<210> 6310
<211> 100
<212> DNA
<213> Escherichia coli K-12 MG1655
<220> ycgk b1179
<222> 303-402
<223> ycgk b1179 U00096 complement(1226294..1226695)
<313> 6310: 344-363
<400> 6310
CCCTGCTTCT GGCAAATATG AGCTGCGAGT GTTGCAAACC CGAAACGACG 50
CCCGTAAAAA CAAAACTAAA AAATATAACG TCGATATTCA AATTAAGTAA 100
<210> 6311
<211> 100
<212> DNA
<213> Escherichia coli K-12 MG1655
<220> ycgk b1179
<222> 285-384
<223> ycgk b1179 U00096 complement(1226294..1226695)
<313> 6311: 315-334
<400> 6311
TCACGGACAA TACTCACTCC CTGCTTCTGG CAAATATGAG CTGCGAGTGT 50
TGCAAACCCG AAACGACGCC CGTAAAAACA AAACTAAAAA ATATAACGTC 100
<210> 6312
<211> 100
<212> DNA
<213> Escherichia coli K-12 MG1655
<220> ycgk b1179
<222> 191-290
<223> ycgk b1179 U00096 complement(1226294..1226695)
<313> 6312: 228-247
<400> 6312
TGAGTATCTC TAATGAAGGC GCTGATACTT ACCTCTTCGG GCCAGGAATC 50
GATGATTCAG TTGATTTATC CAGATATTCC CCAGAACTCG ATAGTCACGG 100
<210> 6313
<211> 100

<212> DNA
<213> Escherichia coli K-12 MG1655
<220> ycgl ycgL
<222> 228-327
<223> ycgl ycgL U00096 1226904..1227230
<313> 6313: 290-309
<400> 6313
CGAGCAAGGT TACTATTTGC AGTTACCGCC ACCACCCGAA GATTTGCTGA 50
AGCAACATCT TTCCGTCATG GGGCAGAAAA CAGACGACAC TAACAAATAA 100
<210> 6314
<211> 100
<212> DNA
<213> Escherichia coli K-12 MG1655
<220> ycgl ycgL
<222> 228-327
<223> ycgl ycgL U00096 1226904..1227230
<313> 6314: 252-271
<400> 6314
CGAGCAAGGT TACTATTTGC AGTTACCGCC ACCACCCGAA GATTTGCTGA 50
AGCAACATCT TTCCGTCATG GGGCAGAAAA CAGACGACAC TAACAAATAA 100
<210> 6315
<211> 100
<212> DNA
<213> Escherichia coli K-12 MG1655
<220> ycgl ycgL
<222> 127-226
<223> ycgl ycgL U00096 1226904..1227230
<313> 6315: 167-186
<400> 6315
ATGAAAGGTT TTGGTCAGCC TCAGTTAGCG ATGATTCTGC CGCTGGATGG 50
GCGGAAGAAA CTGGTCAATG CCGATATTGA AAAGGTTAAA CAGGCATTAA 100
<210> 6316
<211> 100
<212> DNA
<213> Escherichia coli K-12 MG1655
<220> b2380 b2380
<222> 1599-1698
<223> b2380 b2380 U00096 2496691..2498388
<313> 6316: 1673-1692
<400> 6316
GGGGCTGCAT ATCCGCCGCC TGGAGCCGGG GACGGAAATT GCGTTTTACA 50
TTCCTAACCA ACGCACCCCA GTCGCCTCAC AGGCTACGTT ATTGCTTTGA 100
<210> 6317
<211> 100
<212> DNA
<213> Escherichia coli K-12 MG1655
<220> b2380 b2380
<222> 1578-1677
<223> b2380 b2380 U00096 2496691..2498388
<313> 6317: 1622-1641
<400> 6317
CGTGAAGTTA TTGTATGGCG AGGGGCTGCA TATCCGCCGC CTGGAGCCGG 50
GGACGGAAAT TGCGTTTTAC ATTCCTAACC AACGCACCCC AGTCGCCTCA 100
<210> 6318
<211> 100
<212> DNA
<213> Escherichia coli K-12 MG1655
<220> b2380 b2380
<222> 1513-1612
<223> b2380 b2380 U00096 2496691..2498388
<313> 6318: 1560-1579
<400> 6318
ATTGAGCGGG TCGAAGCGAA TGAAATGCCG GGCAATAAAA TTGGCCTGCT 50
GAATGTCCAT CATCGCGTGA AGTTATTGTA TGGCGAGGGG CTGCATATCC 100
<210> 6319
<211> 100
<212> DNA
<213> Escherichia coli K-12 MG1655
<220> ydba_1 b1402
<222> 2458-2557
<223> ydba_1 b1402 U00096 1463416..1465974

<313> 6319: 2520-2539
<400> 6319
AGTGGCGCTA CGCTAACAAT GCAGGATATT GACAGTTTTG AACATCATGG  50
GACAAGAACC CTGGATTTGC CCCTATATTT CCAGACATCT GTTATCACTT  100
<210> 6320
<211> 100
<212> DNA
<213> Escherichia coli K-12 MG1655
<220> ydba_1 b1402
<222> 2430-2529
<223> ydba_1 b1402 U00096 1463416..1465974
<313> 6320: 2467-2486
<400> 6320
ACAGGATAAA GCTTTGCTGG AAATTGGCAG TGGCGCTACG CTAACAATGC  50
AGGATATTGA CAGTTTTGAA CATCATGGGA CAAGAACCCT GGATTTGCCC  100
<210> 6321
<211> 100
<212> DNA
<213> Escherichia coli K-12 MG1655
<220> ydba_1 b1402
<222> 2246-2345
<223> ydba_1 b1402 U00096 1463416..1465974
<313> 6321: 2280-2299
<400> 6321
ATGGTGGACT TGAACTGATT GGAGAGAAAA ACGCGCTTGC AGATGGGTCG  50
CAGGTTACTT CCTTGCGCAC AGGATATAGT TATACCAGCG TTATTGTCGT  100
<210> 6322
<211> 100
<212> DNA
<213> Escherichia coli K-12 MG1655
<220> b2381 b2381
<222> 636-735
<223> b2381 b2381 U00096 2498403..2499137
<313> 6322: 706-725
<400> 6322
GTTTAATAAC ACCTACATTC TGCGACTGAA AGATCTGGAT TTTGAAGTGC  50
CGGTCAGCCG CAGCAAAGTG AAAGAATTTC GCCAGTTAAT GCATCTTTAA  100
<210> 6323
<211> 100
<212> DNA
<213> Escherichia coli K-12 MG1655
<220> b2381 b2381
<222> 547-646
<223> b2381 b2381 U00096 2498403..2499137
<313> 6323: 578-597
<400> 6323
TTTTGCAGCA AACTGCCGCC GTCGCATTTT TTCCGCTGCC ATCGCTCATT  50
TTGTGTCAAT CTGAACAAAA TACGCGAAAT CGAACCGTGG TTTAATAACA  100
<210> 6324
<211> 100
<212> DNA
<213> Escherichia coli K-12 MG1655
<220> b2381 b2381
<222> 504-603
<223> b2381 b2381 U00096 2498403..2499137
<313> 6324: 549-568
<400> 6324
TACGCGGCGT GAATCCTACG TAATGCCGAT GAACATTACC GAATTTTGCA  50
GCAAACTGCC GCCGTCGCAT TTTTTCCGCT GCCATCGCTC ATTTTGTGTC  100
<210> 6325
<211> 100
<212> DNA
<213> Escherichia coli K-12 MG1655
<220> ydba_2 b1406
<222> 3225-3324
<223> ydba_2 b1406 U00096 1468714..1472037
<313> 6325: 3298-3317
<400> 6325
CAGCAGCAAA GAAAGTTCGC TGAATCTGGA TGCGTACAAC TGGAAAGAGG  50
ATGGCATCAG CGATAAAGGC GTGATGCTGA ACTTCAAGAA AACGTTCTAA  100
<210> 6326

<211> 100
<212> DNA
<213> Escherichia coli K-12 MG1655
<220> ydba_2 b1406
<222> 3174-3273
<223> ydba_2 b1406 U00096 1468714..1472037
<313> 6326: 3217-3236
<400> 6326
CGGTCAGAAG GGCGGCGGCA TCAATAGCCT GACAAGCGTC GGCGTGAAGT 50
ACAGCAGCAA AGAAAGTTCG CTGAATCTGG ATGCGTACAA CTGGAAAGAG 100
<210> 6327
<211> 100
<212> DNA
<213> Escherichia coli K-12 MG1655
<220> ydba_2 b1406
<222> 3163-3262
<223> ydba_2 b1406 U00096 1468714..1472037
<313> 6327: 3192-3211
<400> 6327
AACGTCGATG ACGGTCAGAA GGGCGGCGGC ATCAATAGCC TGACAAGCGT 50
CGGCGTGAAG TACAGCAGCA AAGAAAGTTC GCTGAATCTG GATGCGTACA 100
<210> 6328
<211> 100
<212> DNA
<213> Escherichia coli K-12 MG1655
<220> ycgn b1182
<222> 378-477
<223> ycgn b1182 U00096 1228023..1228499
<313> 6328: 456-475
<400> 6328
GCATGGTGAA CGTATCTCTG TGCGTCATAT CGCAGTGAAA GAATCAGAAG 50
TGATTGACTG GCAGGATCAT ATCTTAAATA AACCTGACTG GGCGCAGTGA 100
<210> 6329
<211> 100
<212> DNA
<213> Escherichia coli K-12 MG1655
<220> ycgn b1182
<222> 333-432
<223> ycgn b1182 U00096 1228023..1228499
<313> 6329: 395-414
<400> 6329
TTTACCTGCG TGGCATCCGC TACTTACTGG TTCGAAAGCG GCAATGCATG 50
GTGAACGTAT CTCTGTGCGT CATATCGCAG TGAAAGAATC AGAAGTGATT 100
<210> 6330
<211> 100
<212> DNA
<213> Escherichia coli K-12 MG1655
<220> ycgn b1182
<222> 312-411
<223> ycgn b1182 U00096 1228023..1228499
<313> 6330: 347-366
<400> 6330
TTTGCTGGCG GAAGGTAAAG ATTTACCTGC GTGGCATCCG CTACTTACTG 50
GTTCGAAAGC GGCAATGCAT GGTGAACGTA TCTCTGTGCG TCATATCGCA 100
<210> 6331
<211> 100
<212> DNA
<213> Escherichia coli K-12 MG1655
<220> b2382 b2382
<222> 759-858
<223> b2382 b2382 U00096 2499150..2500007
<313> 6331: 826-845
<400> 6331
ACAGCGTTGC GGTTTTCCGG ATAGCGACTA TTTTTGTCGC GTTTTCCGGC 50
GTCAGTTTGG TCTGACGCCG GGAGAGTACA GCGCCCGTTT TCAGGGCTAA 100
<210> 6332
<211> 100
<212> DNA
<213> Escherichia coli K-12 MG1655
<220> b2382 b2382
<222> 751-850

<223> b2382 b2382 U00096 2499150..2500007
<313> 6332: 793-812
<400> 6332
GAAGTGGCAC AGCGTTGCGG TTTTCCGGAT AGCGACTATT TTTGTCGCGT 50
TTTCCGGCGT CAGTTTGGTC TGACGCCGGG AGAGTACAGC GCCCGTTTTC 100
<210> 6333
<211> 100
<212> DNA
<213> Escherichia coli K-12 MG1655
<220> b2382 b2382
<222> 637-736
<223> b2382 b2382 U00096 2499150..2500007
<313> 6333: 694-713
<400> 6333
CATCTGTCAA AACTGTTTGC TCAGCATGGA ACGATGCGTT TTATCGAGTA 50
TGTGCGTTGG GTGCGAATGG CGAAGGCGAG GATGATTTTG CAGAAATATC 100
<210> 6334
<211> 100
<212> DNA
<213> Escherichia coli K-12 MG1655
<220> b2383 b2383
<222> 2397-2496
<223> b2383 b2383 U00096 complement(2500010..2502505)
<313> 6334: 2453-2472
<400> 6334
GGCGCGTAAA CTGGTGAATA AAAACTTCCG CCAGTCGTTG TTTGCCGCGC 50
AAGATGCACA AAGTATCCTG ACGCTGCTGG AAACAGAATT AACCTTCTGA 100
<210> 6335
<211> 100
<212> DNA
<213> Escherichia coli K-12 MG1655
<220> b2383 b2383
<222> 2370-2469
<223> b2383 b2383 U00096 complement(2500010..2502505)
<313> 6335: 2415-2434
<400> 6335
GAATCATGTG AAAGTCTTCT CGCAGCTGGC GCGTAAACTG GTGAATAAAA 50
ACTTCCGCCA GTCGTTGTTT GCCGCGCAAG ATGCACAAAG TATCCTGACG 100
<210> 6336
<211> 100
<212> DNA
<213> Escherichia coli K-12 MG1655
<220> b2383 b2383
<222> 2324-2423
<223> b2383 b2383 U00096 complement(2500010..2502505)
<313> 6336: 2342-2361
<400> 6336
AAGTCGAACT GGTGATCATG CTGACGCTGG GTGCTAACGA AGGGATGAAT 50
CATGTGAAAG TCTTCTCGCA GCTGGCGCGT AAACTGGTGA ATAAAAACTT 100
<210> 6337
<211> 100
<212> DNA
<213> Escherichia coli K-12 MG1655
<220> b2384 b2384
<222> 878-977
<223> b2384 b2384 U00096 complement(2502530..2503567)
<313> 6337: 930-949
<400> 6337
CCGGCGTGCC CACAGTGGTG ATGGGGCCAG CAACCCGCCA TGGACATTGC 50
GCCGCATCGA TTGCCGATTG CCGCGACATT TTGCAGATGC AGCAACTTTT 100
<210> 6338
<211> 100
<212> DNA
<213> Escherichia coli K-12 MG1655
<220> b2384 b2384
<222> 800-899
<223> b2384 b2384 U00096 complement(2502530..2503567)
<313> 6338: 832-851
<400> 6338
CAGAAATTGG CGTGCCGTTG CAGGCAGATA TGTTCAGCAA CGGCGGCACG 50
GACGGCGGGG CGGTGCACTT AACCGGCACC GGCGTGCCCA CAGTGGTGAT 100

<210> 6339
<211> 100
<212> DNA
<213> Escherichia coli K-12 MG1655
<220> b2384 b2384
<222> 695-794
<223> b2384 b2384 U00096 complement(2502530..2503567)
<313> 6339: 758-777
<400> 6339
GCGCGGCTAA CCATCGCCAG ATTGGTAACG GGCCGATGCT GGTGTTAAGC 50
GACAAGTCGC TGATTGCGCC GCCAAAACTT ACCGCCTGGG TCGAAACCGT 100
<210> 6340
<211> 100
<212> DNA
<213> Escherichia coli K-12 MG1655
<220> b2385 b2385
<222> 987-1086
<223> b2385 b2385 U00096 complement(2503567..2504652)
<313> 6340: 1061-1080
<400> 6340
AGGGGGCGTG CGCATCGAAG ATGTTGTGCT GGTTACCCCG CAAGGCGCAG 50
AAGTGCTCTA CGCCATGCCG AAAACAGTGT TGCTCACGGG AGAGGCATAA 100
<210> 6341
<211> 100
<212> DNA
<213> Escherichia coli K-12 MG1655
<220> b2385 b2385
<222> 962-1061
<223> b2385 b2385 U00096 complement(2503567..2504652)
<313> 6341: 1020-1039
<400> 6341
AGCCGGGGAT TTATTTGCCA GGGCAAGGGG GCGTGCGCAT CGAAGATGTT 50
GTGCTGGTTA CCCCGCAAGG CGCAGAAGTG CTCTACGCCA TGCCGAAAAC 100
<210> 6342
<211> 100
<212> DNA
<213> Escherichia coli K-12 MG1655
<220> b2385 b2385
<222> 926-1025
<223> b2385 b2385 U00096 complement(2503567..2504652)
<313> 6342: 972-991
<400> 6342
CCACGACGCT ACAGCCAGGC ATGTTACTGA CCGTGGAGCC GGGGATTTAT 50
TTGCCAGGGC AAGGGGGCGT GCGCATCGAA GATGTTGTGC TGGTTACCCC 100
<210> 6343
<211> 100
<212> DNA
<213> Escherichia coli K-12 MG1655
<220> ycgr b1195
<222> 636-735
<223> ycgr b1195 U00096 complement(1243016..1243750)
<313> 6343: 673-692
<400> 6343
TTTTCTTAAC GTCAGCCCGA CGGTGGAGCG GCAATTACAG CGGATTATTT 50
TCTCTCTCGA GCGAGAAGCC CGGGAAAAAG CGGACAAAGT GCGCGACTGA 100
<210> 6344
<211> 100
<212> DNA
<213> Escherichia coli K-12 MG1655
<220> ycgr b1195
<222> 585-684
<223> ycgr b1195 U00096 complement(1243016..1243750)
<313> 6344: 619-638
<400> 6344
AGTGATTGAT GGCAAGAATG AAACCATCAC CACTCCCCGT CTGAGCTTCC 50
GTTTTCTTAA CGTCAGCCCG ACGGTGGAGC GGCAATTACA GCGGATTATT 100
<210> 6345
<211> 100
<212> DNA
<213> Escherichia coli K-12 MG1655
<220> ycgr b1195

<222> 536-635
<223> ycgr b1195 U00096 complement(1243016..1243750)
<313> 6345: 586-605
<400> 6345
GGGGTGTTTT TCACTTTGAC GCCCAGTTAA TCTCCATCAG CGAGCGCAAA  50
GTGATTGATG GCAAGAATGA AACCATCACC ACTCCCCGTC TGAGCTTCCG  100
<210> 6346
<211> 100
<212> DNA
<213> Escherichia coli K-12 MG1655
<220> b2386 b2386
<222> 1149-1248
<223> b2386 b2386 U00096 complement(2504667..2505914)
<313> 6346: 1207-1226
<400> 6346
GGCGGGGATC GCGCTGGGGG CAGTCATAAC TGCGCTGATG GTGGTGTTCC  50
TGCGTCTGAT GATGTTCCGT AAAGGCAAAT TGTTAATCGA TAGCCTGTAA  100
<210> 6347
<211> 100
<212> DNA
<213> Escherichia coli K-12 MG1655
<220> b2386 b2386
<222> 1128-1227
<223> b2386 b2386 U00096 complement(2504667..2505914)
<313> 6347: 1155-1174
<400> 6347
CACTAACCTG GGCGTCTATA TGGCGGGGAT CGCGCTGGGG GCAGTCATAA  50
CTGCGCTGAT GGTGGTGTTC CTGCGTCTGA TGATGTTCCG TAAAGGCAAA  100
<210> 6348
<211> 100
<212> DNA
<213> Escherichia coli K-12 MG1655
<220> b2386 b2386
<222> 1091-1190
<223> b2386 b2386 U00096 complement(2504667..2505914)
<313> 6348: 1119-1138
<400> 6348
GGTTCCCGGA GTCCGCTATC TGGGCATGGC CGCTGGTCAC TAACCTGGGC  50
GTCTATATGG CGGGGATCGC GCTGGGGGCA GTCATAACTG CGCTGATGGT  100
<210> 6349
<211> 100
<212> DNA
<213> Escherichia coli K-12 MG1655
<220> b1657 b1658
<222> 1071-1170
<223> b1657 b1658 U00096 complement(1734145..1735314)
<313> 6349: 1139-1158
<400> 6349
TGTGCCGGTG ATGGGAGCGA TTGTCGCGGG ACTGGCATTA TTGCTGGTGT  50
TTATGTCAGC CAGAAAACAA CCTGAAACAG TTTGCGTTGC TAACAGCTAA  100
<210> 6350
<211> 100
<212> DNA
<213> Escherichia coli K-12 MG1655
<220> b1657 b1658
<222> 992-1091
<223> b1657 b1658 U00096 complement(1734145..1735314)
<313> 6350: 1045-1064
<400> 6350
TTGGTGCCTT TAATCTTGGA AATGCGCTGG GAGCAGCTGC TGGTGGTGCG  50
GTAATTTCCG CTGGGCTGGG ATACAGCTTT GTGCCGGTGA TGGGAGCGAT  100
<210> 6351
<211> 100
<212> DNA
<213> Escherichia coli K-12 MG1655
<220> b1657 b1658
<222> 942-1041
<223> b1657 b1658 U00096 complement(1734145..1735314)
<313> 6351: 997-1016
<400> 6351
CGTGATGCGT GTCGCCAGTG AAGCGCCAGG TCTGTCTTCA TCAGTCAATA  50

TTGGTGCCTT TAATCTTGGA AATGCGCTGG GAGCAGCTGC TGGTGGTGCG 100
<210> 6352
<211> 100
<212> DNA
<213> Escherichia coli K-12 MG1655
<220> b2387 b2388
<222> 228-327
<223> b2387 b2388 U00096 complement(2505936..2506262)
<313> 6352: 291-310
<400> 6352
ATCACGCGAC GTTTATGAAA TCACTTTGCA GGACGCAATT AAAAACGCTG 50
CGGGCATCAT CAAAGAAATC GAAGAGATGA TTGCCTCTGA ACAACAGTAA 100
<210> 6353
<211> 100
<212> DNA
<213> Escherichia coli K-12 MG1655
<220> b2387 b2388
<222> 196-295
<223> b2387 b2388 U00096 complement(2505936..2506262)
<313> 6353: 241-260
<400> 6353
GCAGTTACCC CGGAAGATAA CGAACGTTTC GAATCACGCG ACGTTTATGA 50
AATCACTTTG CAGGACGCAA TTAAAAACGC TGCGGGCATC ATCAAAGAAA 100
<210> 6354
<211> 100
<212> DNA
<213> Escherichia coli K-12 MG1655
<220> b2387 b2388
<222> 152-251
<223> b2387 b2388 U00096 complement(2505936..2506262)
<313> 6354: 189-208
<400> 6354
CGGCGCAGGA TATCGCCGAA GCGACCATCA TCATCCACTC CGTGGCAGTT 50
ACCCCGGAAG ATAACGAACG TTTCGAATCA CGCGACGTTT ATGAAATCAC 100
<210> 6355
<211> 100
<212> DNA
<213> Escherichia coli K-12 MG1655
<220> b2389 b2389
<222> 1158-1257
<223> b2389 b2389 U00096 2507650..2508906
<313> 6355: 1224-1243
<400> 6355
GCTCTGTATC GTCATGCTTC CGGCATGGCT GTTATTAGCA GGTAAGCCGA 50
TGATGATGGT CAATCGTCCG AAGCAACAGC CACCCCACGA TAACGTTTAG 100
<210> 6356
<211> 100
<212> DNA
<213> Escherichia coli K-12 MG1655
<220> b2389 b2389
<222> 1151-1250
<223> b2389 b2389 U00096 2507650..2508906
<313> 6356: 1182-1201
<400> 6356
TACCGCTGCT CTGTATCGTC ATGCTTCCGG CATGGCTGTT ATTAGCAGGT 50
AAGCCGATGA TGATGGTCAA TCGTCCGAAG CAACAGCCAC CCCACGATAA 100
<210> 6357
<211> 100
<212> DNA
<213> Escherichia coli K-12 MG1655
<220> b2389 b2389
<222> 1119-1218
<223> b2389 b2389 U00096 2507650..2508906
<313> 6357: 1139-1158
<400> 6357
GGCGGCAGTC GTTGTACCCA ATACCACATT GCTACCGCTG CTCTGTATCG 50
TCATGCTTCC GGCATGGCTG TTATTAGCAG GTAAGCCGAT GATGATGGTC 100
<210> 6358
<211> 100
<212> DNA
<213> Escherichia coli K-12 MG1655

<220> ycgw b1161
<222> 218-317
<223> ycgw b1161 U00096 complement(1210903..1211226)
<313> 6358: 253-272
<400> 6358
GTTTACTCAA AAGCAGTCAA GAGTGTCCTC CAGGAGAAAG CAAAATAACA 50
AATAAATGTT TACATAATAG TTGCATTATA AAAATATGCC CATATGGGCT 100
<210> 6359
<211> 100
<212> DNA
<213> Escherichia coli K-12 MG1655
<220> ycgw b1161
<222> 197-296
<223> ycgw b1161 U00096 complement(1210903..1211226)
<313> 6359: 228-247
<400> 6359
CATTCAACAA GGATTTATGG AGTTTACTCA AAAGCAGTCA AGAGTGTCCT 50
CCAGGAGAAA GCAAAATAAC AAATAAATGT TTACATAATA GTTGCATTAT 100
<210> 6360
<211> 100
<212> DNA
<213> Escherichia coli K-12 MG1655
<220> ycgw b1161
<222> 176-275
<223> ycgw b1161 U00096 complement(1210903..1211226)
<313> 6360: 228-247
<400> 6360
TTACTATTTA TCACATCGCA CCATTCAACA AGGATTTATG GAGTTTACTC 50
AAAAGCAGTC AAGAGTGTCC TCCAGGAGAA AGCAAAATAA CAAATAAATG 100
<210> 6361
<211> 100
<212> DNA
<213> Escherichia coli K-12 MG1655
<220> ycgx b1162
<222> 255-354
<223> ycgx b1162 U00096 complement(1211926..1212330)
<313> 6361: 299-318
<400> 6361
ATCTACATTT CGGGAATACT GTTCAACACT GGCAGGCGCG GGCGTTTTCC 50
GGTGGGTTAC TGATGTTAAT CATAACAAAC GCAGTTACTA CGCCATTGAT 100
<210> 6362
<211> 100
<212> DNA
<213> Escherichia coli K-12 MG1655
<220> ycgx b1162
<222> 210-309
<223> ycgx b1162 U00096 complement(1211926..1212330)
<313> 6362: 272-291
<400> 6362
CCCTGCTAAA ATAAAAGAGG CCGCATTGCT TCATATTTGC GGAAAATCTA 50
CATTTCGGGA ATACTGTTCA ACACTGGCAG GCGCGGGCGT TTTCCGGTGG 100
<210> 6363
<211> 100
<212> DNA
<213> Escherichia coli K-12 MG1655
<220> ycgx b1162
<222> 181-280
<223> ycgx b1162 U00096 complement(1211926..1212330)
<313> 6363: 223-242
<400> 6363
AACCTGTTAA AAGTTAGTAC GACCAAGAAC CCTGCTAAAA TAAAAGAGGC 50
CGCATTGCTT CATATTTGCG GAAAATCTAC ATTTCGGGAA TACTGTTCAA 100
<210> 6364
<211> 100
<212> DNA
<213> Escherichia coli K-12 MG1655
<220> ycgy b1197
<222> 342-441
<223> ycgy b1197 U00096 1244383..1244823
<313> 6364: 406-425
<400> 6364

TGAAACACTT ACCCGTAGAA ATGACGCTAT CTTCGGCAAC CACGTATGGC 50
AAACATTTGC GCAATACTTC CCGCCAGGAC TTGAAAAACC GTCTGTGTAG 100
<210> 6365
<211> 100
<212> DNA
<213> Escherichia coli K-12 MG1655
<220> ycgy b1197
<222> 323-422
<223> ycgy b1197 U00096 1244383..1244823
<313> 6365: 367-386
<400> 6365
AATCGCCGGG TGATGAAAGT GAAACACTTA CCCGTAGAAA TGACGCTATC 50
TTCGGCAACC ACGTATGGCA AACATTTGCG CAATACTTCC CGCCAGGACT 100
<210> 6366
<211> 100
<212> DNA
<213> Escherichia coli K-12 MG1655
<220> ycgy b1197
<222> 127-226
<223> ycgy b1197 U00096 1244383..1244823
<313> 6366: 155-174
<400> 6366
CAAAATACCC GTTCGGCAAT GCTTCAGTTA CGCGAAGAGG AATGGTCTGA 50
ATTCTTTTTT TGGTTGCTAA ATTCTCTGGA GTGTCTGGAT TATGTGATTA 100
<210> 6367
<211> 100
<212> DNA
<213> Escherichia coli K-12 MG1655
<220> ycgz b1165
<222> 129-228
<223> ycgz b1165 U00096 1215012..1215248
<313> 6367: 177-196
<400> 6367
TCTGAGTCGA AAGTCGCTTT GTGCCAAACT TCTTTGCCGA CTGGAACACG 50
CGACTGGGGA AGAGGAACAG AAACATTATA ATGCACTAAT TGGGTTGCTT 100
<210> 6368
<211> 100
<212> DNA
<213> Escherichia coli K-12 MG1655
<220> ycgz b1165
<222> 83-182
<223> ycgz b1165 U00096 1215012..1215248
<313> 6368: 132-151
<400> 6368
AAACTCTCGG GGAAATTGTG ACTGAGATTT TGAAAGATGG ACGTAATCTG 50
AGTCGAAAGT CGCTTTGTGC CAAACTTCTT TGCCGACTGG AACACGCGAC 100
<210> 6369
<211> 100
<212> DNA
<213> Escherichia coli K-12 MG1655
<220> ycgz b1165
<222> 23-122
<223> ycgz b1165 U00096 1215012..1215248
<313> 6369: 68-87
<400> 6369
TAGATTCTGC GGGAGCAATC ACTCGTTACT TTGCAAAAGC TAACTTGCAT 50
ACTCAGCAGG AAACTCTCGG GGAAATTGTG ACTGAGATTT TGAAAGATGG 100
<210> 6370
<211> 100
<212> DNA
<213> Escherichia coli K-12 MG1655
<220> ycha b1215
<222> 553-652
<223> ycha b1215 U00096 1266543..1267352
<313> 6370: 590-609
<400> 6370
GATACACTCA AAGCCTCGTT GATGGAAGAA AATCAGATGG AGCTGGCGTT 50
ACGCACCAGC GAAGCTTTAT TACAATTCAA CCCTGAAGAT CCCTATGAAA 100
<210> 6371
<211> 100
<212> DNA

<213> Escherichia coli K-12 MG1655
<220> ycha b1215
<222> 497-596
<223> ycha b1215 U00096 1266543..1267352
<313> 6371: 542-561
<400> 6371
TGTTTTATGA AGACCTTGAT GAAGCTGATA ACATTGAGGT AATCCGCAAA 50
TTGCTGGATA CACTCAAAGC CTCGTTGATG GAAGAAAATC AGATGGAGCT 100
<210> 6372
<211> 100
<212> DNA
<213> Escherichia coli K-12 MG1655
<220> ycha b1215
<222> 439-538
<223> ycha b1215 U00096 1266543..1267352
<313> 6372: 461-480
<400> 6372
TTAAGCGAAC ATATGCTGGA CGTATGGTTA AAGGGAAATA TCAGCCCGTC 50
GGCGGAACTG TTTTATGAAG ACCTTGATGA AGCTGATAAC ATTGAGGTAA 100
<210> 6373
<211> 100
<212> DNA
<213> Escherichia coli K-12 MG1655
<220> ychb b1209
<222> 753-852
<223> ychb b1209 U00096 complement(1261249..1262100)
<313> 6373: 816-835
<400> 6373
GTCTGAAGCC CGCCAGGTGC TAGAGCAAGC CCCGGAATGG CTCAATGGCT 50
TTGTGGCGAA AGGCGCTAAT CTTTCCCCAT TGCACAGAGC CATGCTTTAA 100
<210> 6374
<211> 100
<212> DNA
<213> Escherichia coli K-12 MG1655
<220> ychb b1209
<222> 734-833
<223> ychb b1209 U00096 complement(1261249..1262100)
<313> 6374: 779-798
<400> 6374
TTGCTGAATT TGATACAGAG TCTGAAGCCC GCCAGGTGCT AGAGCAAGCC 50
CCGGAATGGC TCAATGGCTT TGTGGCGAAA GGCGCTAATC TTTCCCCATT 100
<210> 6375
<211> 100
<212> DNA
<213> Escherichia coli K-12 MG1655
<220> ychb b1209
<222> 716-815
<223> ychb b1209 U00096 complement(1261249..1262100)
<313> 6375: 746-765
<400> 6375
GGACAGGGGC CTGTGTCTTT GCTGAATTTG ATACAGAGTC TGAAGCCCGC 50
CAGGTGCTAG AGCAAGCCCC GGAATGGCTC AATGGCTTTG TGGCGAAAGG 100
<210> 6376
<211> 100
<212> DNA
<213> Escherichia coli K-12 MG1655
<220> yche b1243
<222> 549-648
<223> yche b1243 U00096 1297821..1298468
<313> 6376: 588-607
<400> 6376
CAACGTGATT ACGCGTATTA TGGGGCTATT GCTGATGGCA TTGGGGATTG 50
AATTTATCGT TACTGGTATT AAGGGGATTT TCCCCGGCCT GCTTAATTAA 100
<210> 6377
<211> 100
<212> DNA
<213> Escherichia coli K-12 MG1655
<220> yche b1243
<222> 469-568
<223> yche b1243 U00096 1297821..1298468
<313> 6377: 519-538

<400> 6377
ATTGCATTGT TCGCTTTATG TTGTTGGGGA TTGTTCCGCA TGGCACCGTG 50
GCTGGTACGG GTTTTACGCC AGACCGGCAT CAACGTGATT ACGCGTATTA 100
<210> 6378
<211> 100
<212> DNA
<213> Escherichia coli K-12 MG1655
<220> yche b1243
<222> 425-524
<223> yche b1243 U00096 1297821..1298468
<313> 6378: 464-483
<400> 6378
CGCGTTATCA CAGCATTAGC TATCTGTTTG GTTTCTTTGT GGCTATTGCA 50
TTGTTCGCTT TATGTTGTTG GGGATTGTTC CGCATGGCAC CGTGGCTGGT 100
<210> 6379
<211> 100
<212> DNA
<213> Escherichia coli K-12 MG1655
<220> ychf b1204
<222> 942-1041
<223> ychf b1204 U00096 complement(1255944..1257035)
<313> 6379: 1004-1023
<400> 6379
AGGCTTTATC CGTGCACAAA CCATCTCGTT TGAAGATTTC ATCACTTACA 50
AAGGTGAACA AGGCGCGAAA GAAGCAGGCA AAATGCGTGC AGAAGGTAAA 100
<210> 6380
<211> 100
<212> DNA
<213> Escherichia coli K-12 MG1655
<220> ychf b1204
<222> 861-960
<223> ychf b1204 U00096 complement(1255944..1257035)
<313> 6380: 902-921
<400> 6380
GAAAGAAGTG CGTGCATGGA CCATTCCGGT TGGAGCAACC GCGCCGCAGG 50
CAGCGGGCAA AATCCATACT GATTTTGAAA AAGGCTTTAT CCGTGCACAA 100
<210> 6381
<211> 100
<212> DNA
<213> Escherichia coli K-12 MG1655
<220> ychf b1204
<222> 808-907
<223> ychf b1204 U00096 complement(1255944..1257035)
<313> 6381: 822-841
<400> 6381
ATCCGTGCCG GTTATAAACT GCTGAACCTG CAAACTTACT TCACCGCTGG 50
GGTGAAAGAA GTGCGTGCAT GGACCATTCC GGTTGGAGCA ACCGCGCCGC 100
<210> 6382
<211> 100
<212> DNA
<213> Escherichia coli K-12 MG1655
<220> ychg ychG
<222> 492-591
<223> ychg ychG U00096 complement(1293649..1294239)
<313> 6382: 559-578
<400> 6382
GGTGTTCATA ACAAAACGCC CCCGACCATC AAGGCCGAGG TCGGTTAAGA 50
TATCAAAAAC CCGTTATCCT GTGAAACATA GTGCCGCTCC TCTTAAGTGA 100
<210> 6383
<211> 100
<212> DNA
<213> Escherichia coli K-12 MG1655
<220> ychg ychG
<222> 457-556
<223> ychg ychG U00096 complement(1293649..1294239)
<313> 6383: 512-531
<400> 6383
AAACGACTGG AGCATCTTCG GGGTGCTCTG GAAGTGGTGT TCATAACAAA 50
ACGCCCCCGA CCATCAAGGC CGAGGTCGGT TAAGATATCA AAAACCCGTT 100
<210> 6384
<211> 100

<212> DNA
<213> Escherichia coli K-12 MG1655
<220> ychg ychG
<222> 404-503
<223> ychg ychG U00096 complement(1293649..1294239)
<313> 6384: 436-455
<400> 6384
TCGCATCAGA AATGATAGAG CTGGGAAACA CGGCGTCACC AGGGACCATC 50
CCGAAACGAC TGGAGCATCT TCGGGGTGCT CTGGAAGTGG TGTTCATAAC 100
<210> 6385
<211> 100
<212> DNA
<213> Escherichia coli K-12 MG1655
<220> ychh b1206
<222> 180-279
<223> ychh b1206 U00096 1258014..1258292
<313> 6385: 241-260
<400> 6385
TGCCCGTGTT GGCGGGCATG AACAGGTGTG CGACCGTTAC TGGTGGGTTC 50
GCCACTATGA CAAACGTTGC CGCCGTAGCG ATAATCGCCG TCATAGCTAA 100
<210> 6386
<211> 100
<212> DNA
<213> Escherichia coli K-12 MG1655
<220> ychh b1206
<222> 159-258
<223> ychh b1206 U00096 1258014..1258292
<313> 6386: 186-205
<400> 6386
TGCCATTCTC TGGCTGGCGG GTGCCCGTGT TGGCGGGCAT GAACAGGTGT 50
GCGACCGTTA CTGGTGGGTT CGCCACTATG ACAAACGTTG CCGCCGTAGC 100
<210> 6387
<211> 100
<212> DNA
<213> Escherichia coli K-12 MG1655
<220> ychh b1206
<222> 73-172
<223> ychh b1206 U00096 1258014..1258292
<313> 6387: 137-156
<400> 6387
GTGGGGTATT CAATCCTCAA CCAGTTACCA CAGTTTAATA TGCCCCAGTA 50
TTTCGCACAT GGTGCAGTGC TAAGTATTTT CGTCGGTGCC ATTCTCTGGC 100
<210> 6388
<211> 100
<212> DNA
<213> Escherichia coli K-12 MG1655
<220> ychj b1234
<222> 360-459
<223> ychj b1234 U00096 complement(1287897..1288355)
<313> 6388: 418-437
<400> 6388
TCAATGGTAC TATATTGACG GTACACGTCC GCAGTTTGGT CGCAACGATC 50
CCTGCCCTTG TGGTTCAGGT AAAAAATTTA AAAAGTGCTG CGGCCAATAA 100
<210> 6389
<211> 100
<212> DNA
<213> Escherichia coli K-12 MG1655
<220> ychj b1234
<222> 230-329
<223> ychj b1234 U00096 complement(1287897..1288355)
<313> 6389: 270-289
<400> 6389
GATTGACCGT ATTTGAACAT TGTTGGCAGG ACGCCGACAA TATCGGATTT 50
GTCAGTTTTG TTGCTCGATT TACCGAAGGT GGTAAAACAG GTGCAATAAT 100
<210> 6390
<211> 100
<212> DNA
<213> Escherichia coli K-12 MG1655
<220> ychj b1234
<222> 150-249
<223> ychj b1234 U00096 complement(1287897..1288355)

<313> 6390: 190-209
<400> 6390
AAAGACCTGG CATCCCTCTT GTGGAGCAGC GGCGTTACGT GCCGAATTGA 50
TGGCCGGATT TGCGCATACT GAATGGTTGG GATTGACCGT ATTTGAACAT 100
<210> 6391
<211> 100
<212> DNA
<213> Escherichia coli K-12 MG1655
<220> rhsa b3594
<222> 4035-4134
<223> rhsa b3594 U00096 3759810..3763943
<313> 6391: 4095-4114
<400> 6391
ACTAATTGAT TGTGGTGATA TTAGTGCTAA AGATGCAAAA AGCACACAGA 50
AAGCCTGGAA TTGTCGTCAC TCCAGACAGT CAAACGATAA AAAAAGATAG 100
<210> 6392
<211> 100
<212> DNA
<213> Escherichia coli K-12 MG1655
<220> rhsa b3594
<222> 3959-4058
<223> rhsa b3594 U00096 3759810..3763943
<313> 6392: 4018-4037
<400> 6392
ATCGTGTCAA TGACATAATA AATGACAGAT TCTGGAGTGA TGGGAAAAAA 50
CCCGACAGAT GTGACGTACT TCAGGAACTA ATTGATTGTG GTGATATTAG 100
<210> 6393
<211> 100
<212> DNA
<213> Escherichia coli K-12 MG1655
<220> rhsa b3594
<222> 3919-4018
<223> rhsa b3594 U00096 3759810..3763943
<313> 6393: 3934-3953
<400> 6393
ATAGCTTCTC GTGGAAATGT TGCAGACACA GGGATAACTG ATCGTGTCAA 50
TGACATAATA AATGACAGAT TCTGGAGTGA TGGGAAAAAA CCCGACAGAT 100
<210> 6394
<211> 100
<212> DNA
<213> Escherichia coli K-12 MG1655
<220> ychk b1235
<222> 846-945
<223> ychk b1235 U00096 1288429..1289373
<313> 6394: 902-921
<400> 6394
GCTTGATTTC CATCGCGCGC ACGCTGCCAT TGCGGCCGGA CAGCTGGCAG 50
TGGAAAGGAA AATGGACGAA CTTTTGCCGT TGGTACGCAC CAACATTTGA 100
<210> 6395
<211> 100
<212> DNA
<213> Escherichia coli K-12 MG1655
<220> ychk b1235
<222> 757-856
<223> ychk b1235 U00096 1288429..1289373
<313> 6395: 800-819
<400> 6395
CAGGTGCTGG AGAACCGCCT TAAAAGGAAC CGCATGGCAG GTGATCCGCC 50
CGATATTCTG ATTCAACCTG TTTGCCCGCA AATATCTACG CTTGATTTCC 100
<210> 6396
<211> 100
<212> DNA
<213> Escherichia coli K-12 MG1655
<220> ychk b1235
<222> 717-816
<223> ychk b1235 U00096 1288429..1289373
<313> 6396: 762-781
<400> 6396
GACAGCGCCA ACGGCAACAG AGATTATGAC CACTTCTATC CAGGTGCTGG 50
AGAACCGCCT TAAAAGGAAC CGCATGGCAG GTGATCCGCC CGATATTCTG 100
<210> 6397

<211> 100
<212> DNA
<213> Escherichia coli K-12 MG1655
<220> rhsb b3483
<222> 3894-3993
<223> rhsb b3483 U00096 3616823..3621058
<313> 6397: 3944-3963
<400> 6397
AGAGTTCGCT GGTTTAATAT GTAAGGATAA TAGTGGCAGA TATTTCTCAA 50
CAGCACCTAA CCGAGGAGAA AGAAAAGGAT CATATCCATT CAATAGCCCT 100
<210> 6398
<211> 100
<212> DNA
<213> Escherichia coli K-12 MG1655
<220> rhsb b3483
<222> 3771-3870
<223> rhsb b3483 U00096 3616823..3621058
<313> 6398: 3790-3809
<400> 6398
ATGTACTAAT GGTCTTTGCG GCACACTATC CGCTATGATA GGTCCAGATA 50
AATTTGATTC TATAGATAGC ACCGCATATG ACGCCTTAAA TAAAATAAAT 100
<210> 6399
<211> 100
<212> DNA
<213> Escherichia coli K-12 MG1655
<220> rhsb b3483
<222> 3706-3805
<223> rhsb b3483 U00096 3616823..3621058
<313> 6399: 3744-3763
<400> 6399
TTGAATCCGA TATCAGACAT CGACCCCCTG GGTTTATCTA TGTGGGAGGA 50
TGCAAAATCG GGGGCATGTA CTAATGGTCT TTGCGGCACA CTATCCGCTA 100
<210> 6400
<211> 100
<212> DNA
<213> Escherichia coli K-12 MG1655
<220> rhsc rhsC
<222> 3738-3837
<223> rhsc rhsC U00096 728806..732999
<313> 6400: 3795-3814
<400> 6400
ATTAGAAACA CTAAAATGCA TTAAGCCACT GCATTCAATG GGCGGAACTG 50
GTGAAAGAAG CGGTCCAGAT ATATGGGGGA ATCCGTTCTA TCATCAATAT 100
<210> 6401
<211> 100
<212> DNA
<213> Escherichia coli K-12 MG1655
<220> rhsc rhsC
<222> 3659-3758
<223> rhsc rhsC U00096 728806..732999
<313> 6401: 3683-3702
<400> 6401
AGGATCCGAT TGGGCTGAAA GGGGGATGGA ATTTTTATCA GTATCCGTTG 50
AATCCGATCT CAAATATAGA TCCATTAGGA TTAGAAACAC TAAAATGCAT 100
<210> 6402
<211> 100
<212> DNA
<213> Escherichia coli K-12 MG1655
<220> rhsc rhsC
<222> 3487-3586
<223> rhsc rhsC U00096 728806..732999
<313> 6402: 3522-3541
<400> 6402
ACAACAGCGT GGTACGCAGA ATACGATGAA TGGGGTAACC TGCTGAATGA 50
AGAGAACCCG CATCAGCTGC AGCAGCTTAT CCGCCTGCCG GGGCAGCAGT 100
<210> 6403
<211> 100
<212> DNA
<213> Escherichia coli K-12 MG1655
<220> b2390 b2390
<222> 32-131

<223> b2390 b2390 U00096 2509021..2509347
<313> 6403: 75-94
<400> 6403
TGATGGCATT TACCCCGCTT GCAGCAAACG CAGGTGAAAT CACCCTACTG 50
CCATCAATCA AATTACAAAT TGGCGATCGC GATCATTACG GTAATTACTG 100
<210> 6404
<211> 100
<212> DNA
<213> Escherichia coli K-12 MG1655
<220> b2390 b2390
<222> 4-103
<223> b2390 b2390 U00096 2509021..2509347
<313> 6404: 64-83
<400> 6404
TTCAGGTCAC TGTTTCTGGC GGCCGCCCTG ATGGCATTTA CCCCGCTTGC 50
AGCAAACGCA GGTGAAATCA CCCTACTGCC ATCAATCAAA TTACAAATTG 100
<210> 6405
<211> 100
<212> DNA
<213> Escherichia coli K-12 MG1655
<220> b2390 b2390
<222> 1-100
<223> b2390 b2390 U00096 2509021..2509347
<313> 6405: 32-51
<400> 6405
ATGTTCAGGT CACTGTTTCT GGCGGCCGCC CTGATGGCAT TTACCCCGCT 50
TGCAGCAAAC GCAGGTGAAA TCACCCTACT GCCATCAATC AAATTACAAA 100
<210> 6406
<211> 100
<212> DNA
<213> Escherichia coli K-12 MG1655
<220> ychm b1207
<222> 1445-1544
<223> ychm b1207 U00096 complement(1258347..1259999)
<313> 6406: 1475-1494
<400> 6406
TTCTGAAGTG GGATGCCGTT CCGGTACTTG ATGCTGGTGG TCTTGATGCG 50
TTCCAGCGTT TTGTGAAGCG TCTGCCCGAG GGATGTGAAC TGCGCGTGTG 100
<210> 6407
<211> 100
<212> DNA
<213> Escherichia coli K-12 MG1655
<220> ychm b1207
<222> 1381-1480
<223> ychm b1207 U00096 complement(1258347..1259999)
<313> 6407: 1406-1425
<400> 6407
TTTGCTGCTG CTGAAGGCTT ATTCACGGAC CTGGAGTCAC GTCTTGAAGG 50
CAAACGGATT GTGATTCTGA AGTGGGATGC CGTTCCGGTA CTTGATGCTG 100
<210> 6408
<211> 100
<212> DNA
<213> Escherichia coli K-12 MG1655
<220> ychm b1207
<222> 1353-1452
<223> ychm b1207 U00096 complement(1258347..1259999)
<313> 6408: 1369-1388
<400> 6408
GGTTCTGCGC GTTATTGGCC CGCTGTTTTT TGCTGCTGCT GAAGGCTTAT 50
TCACGGACCT GGAGTCACGT CTTGAAGGCA AACGGATTGT GATTCTGAAG 100
<210> 6409
<211> 100
<212> DNA
<213> Escherichia coli K-12 MG1655
<220> rhsd b0498
<222> 4114-4213
<223> rhsd b0498 U00096 522485..526765
<313> 6409: 4167-4186
<400> 6409
TCAAGTAGCG ATAAAAATCT TGTAAGAAGT AAAGATAATA ATCTTGAAGC 50
ATTTTATCTC GCAACACCTG ATGGACGATT TGAGGCGCTT AATAATAAAG 100

<210> 6410
<211> 100
<212> DNA
<213> Escherichia coli K-12 MG1655
<220> rhsd b0498
<222> 3799-3898
<223> rhsd b0498 U00096 522485..526765
<313> 6410: 3817-3836
<400> 6410
TCTGGAGCAT GTACGGGGGG AGTTTGTGGT GTTCTTTCAC GTATAATAGG 50
ACCAAGTAAA TTTGATAGTA CTGCAGATGC TGCGTTAGAT GCTTTGAAAG 100
<210> 6411
<211> 100
<212> DNA
<213> Escherichia coli K-12 MG1655
<220> rhsd b0498
<222> 3667-3766
<223> rhsd b0498 U00096 522485..526765
<313> 6411: 3703-3722
<400> 6411
CCGTTGCAGG GGCGGTATAT TACTCAGGAC CCGATGGGGT TGAAAGGGGG 50
ATGGAATTTA TATCAGTATC CTTTAAATCC ACTACAACAA ATTGACCCTA 100
<210> 6412
<211> 100
<212> DNA
<213> Escherichia coli K-12 MG1655
<220> b2391 b2391
<222> 201-300
<223> b2391 b2391 U00096 2509085..2509429
<313> 6412: 242-261
<400> 6412
GAAGGCTGGC GCGATCGTGA CGATCATCGC GGAAAAGGCC GGGGTCATGG 50
GCACCGCCAT TAAACGCGTC CGCCAATGGA GCACAATGCC TGATGCGACG 100
<210> 6413
<211> 100
<212> DNA
<213> Escherichia coli K-12 MG1655
<220> mota b1891
<222> 789-888
<223> mota b1891 U00096 complement(1974276..1975163)
<313> 6413: 860-879
<400> 6413
AACGCTCTAT TCCAGCGAAC GTCCGTCGTT TATTGAACTG GAAGAGCATG 50
TGCGTGCGGT GAAAAATCCG CAACAACAGA CGACAACCGA GGAAGCATGA 100
<210> 6414
<211> 100
<212> DNA
<213> Escherichia coli K-12 MG1655
<220> mota b1891
<222> 754-853
<223> mota b1891 U00096 complement(1974276..1975163)
<313> 6414: 791-810
<400> 6414
TACGCACCGC CTATCGCCGT TGAGTTTGGT CGCAAAACGC TCTATTCCAG 50
CGAACGTCCG TCGTTTATTG AACTGGAAGA GCATGTGCGT GCGGTGAAAA 100
<210> 6415
<211> 100
<212> DNA
<213> Escherichia coli K-12 MG1655
<220> mota b1891
<222> 710-809
<223> mota b1891 U00096 complement(1974276..1975163)
<313> 6415: 766-785
<400> 6415
TGATGCAGTG CGTCAAAGTC ACTCTGCTTT CTAATCTGAA CGGTTACGCA 50
CCGCCTATCG CCGTTGAGTT TGGTCGCAAA ACGCTCTATT CCAGCGAACG 100
<210> 6416
<211> 100
<212> DNA
<213> Escherichia coli K-12 MG1655
<220> ychn b1220

<222> 255-354
<223> ychn b1220 U00096 complement(1272469..1272822)
<313> 6416: 323-342
<400> 6416
CGGGATTAGT ACACTTCCTC TGATTGATGG GGTGGAAATC GGTACTCTTG 50
TGGAACTGGC GCAATGGACG CTGTCAGCCG ATAAAGTGCT CACATTTTAA 100
<210> 6417
<211> 100
<212> DNA
<213> Escherichia coli K-12 MG1655
<220> ychn b1220
<222> 242-341
<223> ychn b1220 U00096 complement(1272469..1272822)
<313> 6417: 288-307
<400> 6417
GTACCGACGG GCGCGGGATT AGTACACTTC CTCTGATTGA TGGGGTGGAA 50
ATCGGTACTC TTGTGGAACT GGCGCAATGG ACGCTGTCAG CCGATAAAGT 100
<210> 6418
<211> 100
<212> DNA
<213> Escherichia coli K-12 MG1655
<220> ychn b1220
<222> 188-287
<223> ychn b1220 U00096 complement(1272469..1272822)
<313> 6418: 244-263
<400> 6418
AAATGCTGGA GATCCTTACC GCTCAGAATG TACCGGTGAA ATTGTGCAAA 50
ACCTGTACCG ACGGGCGCGG GATTAGTACA CTTCCTCTGA TTGATGGGGT 100
<210> 6419
<211> 100
<212> DNA
<213> Escherichia coli K-12 MG1655
<220> rhse b1457
<222> 1949-2048
<223> rhse b1457 U00096 1525914..1527962
<313> 6419: 1978-1997
<400> 6419
TTGAAAAATA TAACACTGGG CAACTCTTCG GGTTAGATGG TATAAAAGCA 50
GATAATCCAA TAAAAGCAAA GCAAGGGAGT TCAGATGCTT CAAATTATTA 100
<210> 6420
<211> 100
<212> DNA
<213> Escherichia coli K-12 MG1655
<220> rhse b1457
<222> 1870-1969
<223> rhse b1457 U00096 1525914..1527962
<313> 6420: 1906-1925
<400> 6420
GAAAGAATCA TGGATCTTAA AAATAACACT GTCGGTATTG CTTGTGGCGA 50
TTTTTCTGCC AAATGTAGCG ATGCATGTAT TGAAAAATAT AACACTGGGC 100
<210> 6421
<211> 100
<212> DNA
<213> Escherichia coli K-12 MG1655
<220> rhse b1457
<222> 1808-1907
<223> rhse b1457 U00096 1525914..1527962
<313> 6421: 1855-1874
<400> 6421
TAGCTGACGT GATAGGTAAA AATCATGAGG CGGCAGGGGA TAGACAAGGT 50
CAGCCAGCTA AAGAAAGAAT CATGGATCTT AAAAATAACA CTGTCGGTAT 100
<210> 6422
<211> 100
<212> DNA
<213> Escherichia coli K-12 MG1655
<220> b2392 b2393
<222> 1126-1225
<223> b2392 b2393 U00096 complement(2509488..2510726)
<313> 6422: 1160-1179
<400> 6422
TTGATGGGCG ATCTGGTGAA CAGCAAACGC GTAAAACAGA CAGGCTGGGT 50

GATTGTGGTG CTGGTCGTGG CGCTGAATAT CTGGTTGTTG GTGGGGACGG 100
<210> 6423
<211> 100
<212> DNA
<213> Escherichia coli K-12 MG1655
<220> b2392 b2393
<222> 1077-1176
<223> b2392 b2393 U00096 complement(2509488..2510726)
<313> 6423: 1132-1151
<400> 6423
TATCGCTCTG GCGCTGGTTC CACTGCTGAT TTTCACCAGT GACAGCAAGT 50
TGATGGGCGA TCTGGTGAAC AGCAAACGCG TAAAACAGAC AGGCTGGGTG 100
<210> 6424
<211> 100
<212> DNA
<213> Escherichia coli K-12 MG1655
<220> b2392 b2393
<222> 1007-1106
<223> b2392 b2393 U00096 complement(2509488..2510726)
<313> 6424: 1029-1048
<400> 6424
TTATTGTCAT TCTGATGGGA TTAGATCCGA CACGGATTCT GGTTATGAGT 50
CAGGTACTGT TAAGTTTTGG TATCGCTCTG GCGCTGGTTC CACTGCTGAT 100
<210> 6425
<211> 100
<212> DNA
<213> Escherichia coli K-12 MG1655
<220> motb b1890
<222> 828-927
<223> motb b1890 U00096 complement(1973353..1974279)
<313> 6425: 896-915
<400> 6425
AAACGCCGAA AGCCAGAATG AGCCAGTAAG CGCCCTGGAA AAACCTGAGG 50
TTGCACCACA GGTCAGTGTT CCCACAATGC CATCAGCCGA ACCGAGGTGA 100
<210> 6426
<211> 100
<212> DNA
<213> Escherichia coli K-12 MG1655
<220> motb b1890
<222> 818-917
<223> motb b1890 U00096 complement(1973353..1974279)
<313> 6426: 849-868
<400> 6426
TTTTGCATGA AAACGCCGAA AGCCAGAATG AGCCAGTAAG CGCCCTGGAA 50
AAACCTGAGG TTGCACCACA GGTCAGTGTT CCCACAATGC CATCAGCCGA 100
<210> 6427
<211> 100
<212> DNA
<213> Escherichia coli K-12 MG1655
<220> motb b1890
<222> 783-882
<223> motb b1890 U00096 complement(1973353..1974279)
<313> 6427: 807-826
<400> 6427
CCTGCTGGTA CTGAACAAAC AAGCCGAACA GGCCATTTTG CATGAAAACG 50
CCGAAAGCCA GAATGAGCCA GTAAGCGCCC TGGAAAAACC TGAGGTTGCA 100
<210> 6428
<211> 100
<212> DNA
<213> Escherichia coli K-12 MG1655
<220> b1664 b1665
<222> 1158-1257
<223> b1664 b1665 U00096 complement(1742895..1744151)
<313> 6428: 1230-1249
<400> 6428
ACACGTTGAG ATAACAGATG CCAGTCGGCA AACTCTGTGT AACGCTCTGA 50
AATTACAGCC AGACGAAGAC GGGATTGGCG CAACGCTCCA GCCTGCGTAA 100
<210> 6429
<211> 100
<212> DNA
<213> Escherichia coli K-12 MG1655

<220> b1664 b1665
<222> 1158-1257
<223> b1664 b1665 U00096 complement(1742895..1744151)
<313> 6429: 1191-1210
<400> 6429
ACACGTTGAG ATAACAGATG CCAGTCGGCA AACTCTGTGT AACGCTCTGA  50
AATTACAGCC AGACGAAGAC GGGATTGGCG CAACGCTCCA GCCTGCGTAA  100
<210> 6430
<211> 100
<212> DNA
<213> Escherichia coli K-12 MG1655
<220> b1664 b1665
<222> 1093-1192
<223> b1664 b1665 U00096 complement(1742895..1744151)
<313> 6430: 1136-1155
<400> 6430
CAAACCAGTG CAAAAGAAAT TTCACCTGAC TTCATCAATG TGAAAGGGGA  50
AGTTACCGGG GATATACACG TTGAGATAAC AGATGCCAGT CGGCAAACTC  100
<210> 6431
<211> 100
<212> DNA
<213> Escherichia coli K-12 MG1655
<220> gyra b2232
<222> 2432-2531
<223> gyra b2232 U00096 complement(2334813..2337440)
<313> 6431: 2467-2486
<400> 6431
AAATCAGCAT CGTGGGCCGT AACACCCAGG GCGTGATCCT CATCCGTACT  50
GCGGAAGATG AAAACGTAGT GGGTCTGCAA CGTGTTGCTG AACCGGTTGA  100
<210> 6432
<211> 100
<212> DNA
<213> Escherichia coli K-12 MG1655
<220> gyra b2232
<222> 2302-2401
<223> gyra b2232 U00096 complement(2334813..2337440)
<313> 6432: 2347-2366
<400> 6432
ACGAAAGGGG TTATCTCCAT CAAGGTTACC GAACGTAACG GTTTAGTTGT  50
TGGCGCGGTA CAGGTAGATG ACTGCGACCA GATCATGATG ATCACCGATG  100
<210> 6433
<211> 100
<212> DNA
<213> Escherichia coli K-12 MG1655
<220> gyra b2232
<222> 2240-2339
<223> gyra b2232 U00096 complement(2334813..2337440)
<313> 6433: 2265-2284
<400> 6433
CAACGCAAAA CGGTTACGGT AAACGTACCG CAGTGGCGGA ATACCCAACC  50
AAGTCGCGTG CGACGAAAGG GGTTATCTCC ATCAAGGTTA CCGAACGTAA  100
<210> 6434
<211> 100
<212> DNA
<213> Escherichia coli K-12 MG1655
<220> ychp b1221
<222> 1125-1224
<223> ychp b1221 U00096 1273148..1274401
<313> 6434: 1178-1197
<400> 6434
CAATCACTGG CGATTGTCTG TGGTGGTGGA AGATAACCAG GGGCAGCGTG  50
TCTCCTCCAA TGAGATCACG CTAACGCTTG TCGAACCGTT CGATGCATTG  100
<210> 6435
<211> 100
<212> DNA
<213> Escherichia coli K-12 MG1655
<220> ychp b1221
<222> 1096-1195
<223> ychp b1221 U00096 1273148..1274401
<313> 6435: 1139-1158
<400> 6435

CCTGACTGGC AGAACGGGGA AGGGGCAAGC AATCACTGGC GATTGTCTGT 50
GGTGGTGGAA GATAACCAGG GGCAGCGTGT CTCCTCCAAT GAGATCACGC 100
<210> 6436
<211> 100
<212> DNA
<213> Escherichia coli K-12 MG1655
<220> ychp b1221
<222> 1074-1173
<223> ychp b1221 U00096 1273148..1274401
<313> 6436: 1114-1133
<400> 6436
GGAGGGCTGG ACGCTGATCA TGCCTGACTG GCAGAACGGG GAAGGGGCAA 50
GCAATCACTG GCGATTGTCT GTGGTGGTGG AAGATAACCA GGGGCAGCGT 100
<210> 6437
<211> 100
<212> DNA
<213> Escherichia coli K-12 MG1655
<220> gyrb b3700
<222> 2316-2415
<223> gyrb b3700 U00096 complement(3875333..3877747)
<313> 6437: 2385-2404
<400> 6437
TGCCGACCAG TTGTTCACCA CGCTGATGGG CGACGCCGTT GAACCGCGCC 50
GTGCGTTTAT TGAAGAGAAC GCCCTGAAAG CGGCGAATAT CGATATTTAA 100
<210> 6438
<211> 100
<212> DNA
<213> Escherichia coli K-12 MG1655
<220> gyrb b3700
<222> 2301-2400
<223> gyrb b3700 U00096 complement(3875333..3877747)
<313> 6438: 2342-2361
<400> 6438
TAAAGATGCG ATTGCTGCCG ACCAGTTGTT CACCACGCTG ATGGGCGACG 50
CCGTTGAACC GCGCCGTGCG TTTATTGAAG AGAACGCCCT GAAAGCGGCG 100
<210> 6439
<211> 100
<212> DNA
<213> Escherichia coli K-12 MG1655
<220> gyrb b3700
<222> 2229-2328
<223> gyrb b3700 U00096 complement(3875333..3877747)
<313> 6439: 2279-2298
<400> 6439
CGAGATGAAC CCGGAACAGC TGTGGGAAAC CACTATGGAC CCGGAAAGTC 50
GTCGTATGCT GCGCGTTACC GTTAAAGATG CGATTGCTGC CGACCAGTTG 100
<210> 6440
<211> 100
<212> DNA
<213> Escherichia coli K-12 MG1655
<220> b1667 b1667
<222> 207-306
<223> b1667 b1667 U00096 1744724..1745029
<313> 6440: 250-269
<400> 6440
TACTGCCCGA CTGAAAAATC TCGGCGTTGA GGAAGTTGTC GCCAAAGTTT 50
TCGATGTCAA TGAGCCACTT AGTCAAATCA ATCAGGCAAA ACTCGCCTGA 100
<210> 6441
<211> 100
<212> DNA
<213> Escherichia coli K-12 MG1655
<220> b1667 b1667
<222> 163-262
<223> b1667 b1667 U00096 1744724..1745029
<313> 6441: 196-215
<400> 6441
CTTTTCACTG ATGAAAAAAG CGCCCTTGCC TATCTGGAAA AACATACTGC 50
CCGACTGAAA AATCTCGGCG TTGAGGAAGT TGTCGCCAAA GTTTTCGATG 100
<210> 6442
<211> 100
<212> DNA

<213> Escherichia coli K-12 MG1655
<220> b1667 b1667
<222> 135-234
<223> b1667 b1667 U00096 1744724..1745029
<313> 6442: 173-192
<400> 6442
AAAGAACCAC GAAGCCGGTG GGATCTACCT TTTCACTGAT GAAAAAAGCG 50
CCCTTGCCTA TCTGGAAAAA CATACTGCCC GACTGAAAAA TCTCGGCGTT 100
<210> 6443
<211> 100
<212> DNA
<213> Escherichia coli K-12 MG1655
<220> b1668 b1668
<222> 1458-1557
<223> b1668 b1668 U00096 1745155..1746759
<313> 6443: 1503-1522
<400> 6443
GCGCGTAGCG TTCGGCGCGT TGCCCTGGTT GATGCACGAC CAGCCTTTCG 50
TTCAGGGACT TACGGCATGT GCAGAAATTG GTGAGGCGAT GGCTCGGGCG 100
<210> 6444
<211> 100
<212> DNA
<213> Escherichia coli K-12 MG1655
<220> b1668 b1668
<222> 1378-1477
<223> b1668 b1668 U00096 1745155..1746759
<313> 6444: 1436-1455
<400> 6444
GAGCAATTAC AGAAAACAGG GGATGAAATC CCTGATGTTG GCGAAGATTA 50
TACGTTACAG CAACCCGAAG ATATTCGTGG GCGCGTAGCG TTCGGCGCGT 100
<210> 6445
<211> 100
<212> DNA
<213> Escherichia coli K-12 MG1655
<220> b1668 b1668
<222> 1350-1449
<223> b1668 b1668 U00096 1745155..1746759
<313> 6445: 1378-1397
<400> 6445
GAAAGATATC CCTTTCCCTG GGCTACGCGA GCAATTACAG AAAACAGGGG 50
ATGAAATCCC TGATGTTGGC GAAGATTATA CGTTACAGCA ACCCGAAGAT 100
<210> 6446
<211> 100
<212> DNA
<213> Escherichia coli K-12 MG1655
<220> b1669 b1670
<222> 714-813
<223> b1669 b1670 U00096 complement(1746771..1747583)
<313> 6446: 763-782
<400> 6446
CGAAAGAGTT CGCTTCGCCG TCGCCATTGA AGGTGAAAAC AATATTCGAG 50
CCGGAGTCCG CTATCGTCTT AATGAACAGC ATCAGTTTGT TGAGTGCTGA 100
<210> 6447
<211> 100
<212> DNA
<213> Escherichia coli K-12 MG1655
<220> b1669 b1670
<222> 705-804
<223> b1669 b1670 U00096 complement(1746771..1747583)
<313> 6447: 727-746
<400> 6447
TCATGACGGC GAAAGAGTTC GCTTCGCCGT CGCCATTGAA GGTGAAAACA 50
ATATTCGAGC CGGAGTCCGC TATCGTCTTA ATGAACAGCA TCAGTTTGTT 100
<210> 6448
<211> 100
<212> DNA
<213> Escherichia coli K-12 MG1655
<220> b1669 b1670
<222> 650-749
<223> b1669 b1670 U00096 complement(1746771..1747583)
<313> 6448: 674-693

<400> 6448
CGGGCGTGGT CGACTCAATT ATTCTCGGGC CAGGAGGAAG CGCCGCACTG  50
GCATATCATG ACGGCGAAAG AGTTCGCTTC GCCGTCGCCA TTGAAGGTGA  100
<210> 6449
<211> 100
<212> DNA
<213> Escherichia coli K-12 MG1655
<220> atoa b2223
<222> 552-651
<223> atoa b2223 U00096 2322129..2322779
<313> 6449: 609-628
<400> 6449
CGAAATTGCC GACGGGTGTG ATTTAGCCAC CGTGCGTGCC AAAACAGAAG  50
CTCGGTTTGA AGTCGCCGCC GATCTGAATA CGCAACGGGG TGATTTATGA  100
<210> 6450
<211> 100
<212> DNA
<213> Escherichia coli K-12 MG1655
<220> atoa b2223
<222> 536-635
<223> atoa b2223 U00096 2322129..2322779
<313> 6450: 552-571
<400> 6450
GCAAAATGTG GCTCACCGAA ATTGCCGACG GGTGTGATTT AGCCACCGTG  50
CGTGCCAAAA CAGAAGCTCG GTTTGAAGTC GCCGCCGATC TGAATACGCA  100
<210> 6451
<211> 100
<212> DNA
<213> Escherichia coli K-12 MG1655
<220> atoa b2223
<222> 322-421
<223> atoa b2223 U00096 2322129..2322779
<313> 6451: 360-379
<400> 6451
AACCTCGCGA ACTGGGTAGT GCCTGGGAAA ATGGTGCCCG GTATGGGTGG  50
CGCGATGGAT CTGGTGACCG GGTCGCGCAA AGTGATCATC GCCATGGAAC  100
<210> 6452
<211> 100
<212> DNA
<213> Escherichia coli K-12 MG1655
<220> ycia b1254
<222> 300-399
<223> ycia b1254 U00096 complement(1309872..1310270)
<313> 6452: 379-398
<400> 6452
GTCTGAACCA ATTGGGCAAC GCTATAAAGC GACAGAAGCA TTATTTAAGT  50
ATGTCGCGGT TGATCCTGAA GGAAAACCTC GCGCCTTACC TGTTGAGTAA  100
<210> 6453
<211> 100
<212> DNA
<213> Escherichia coli K-12 MG1655
<220> ycia b1254
<222> 195-294
<223> ycia b1254 U00096 complement(1309872..1310270)
<313> 6453: 247-266
<400> 6453
ACGGCCGGTT GCGGTCGGCG ATGTGGTGTG CTGCTATGCA CGCTGTGTCC  50
AGAAAGGGAC GACATCGGTC AGCATTAATA TTGAAGTGTG GGTGAAAAAA  100
<210> 6454
<211> 100
<212> DNA
<213> Escherichia coli K-12 MG1655
<220> ycia b1254
<222> 98-197
<223> ycia b1254 U00096 complement(1309872..1310270)
<313> 6454: 142-161
<400> 6454
GGTTAATGTC ACAAATGGAT ATTGGCGGCG CTATTCTGGC AAAAGAAATT  50
GCCCACGGTC GCGTAGTGAC TGTGCGGGTT GAAGGAATGA CTTTCTTACG  100
<210> 6455
<211> 100

<212> DNA
<213> Escherichia coli K-12 MG1655
<220> atob atoB
<222> 1086-1185
<223> atob atoB U00096 2324129..2325313
<313> 6455: 1122-1141
<400> 6455
ATTACATGCC ATGCAGGCAC GCGATAAAAC GCTGGGGCTG GCAACACTGT  50
GCATTGGCGG CGGTCAGGGA ATTGCGATGG TGATTGAACG GTTGAATTAA  100
<210> 6456
<211> 100
<212> DNA
<213> Escherichia coli K-12 MG1655
<220> atob atoB
<222> 1049-1148
<223> atob atoB U00096 2324129..2325313
<313> 6456: 1090-1109
<400> 6456
CTATCGGTGC CAGTGGTGCT CGTATTCTGG TCACACTATT ACATGCCATG  50
CAGGCACGCG ATAAAACGCT GGGGCTGGCA ACACTGTGCA TTGGCGGCGG  100
<210> 6457
<211> 100
<212> DNA
<213> Escherichia coli K-12 MG1655
<220> atob atoB
<222> 964-1063
<223> atob atoB U00096 2324129..2325313
<313> 6457: 1017-1036
<400> 6457
GCACAGTTCC TTGCCGTTGG GAAAAACCTG GGCTTTGATT CTGAGAAAGT  50
GAATGTCAAC GGCGGGGCCA TCGCGCTCGG GCATCCTATC GGTGCCAGTG  100
<210> 6458
<211> 100
<212> DNA
<213> Escherichia coli K-12 MG1655
<220> ycib b1255
<222> 441-540
<223> ycib b1255 U00096 complement(1310375..1310914)
<313> 6458: 509-528
<400> 6458
CAACTTTAAA GTCTTTGGCC TGACCGCCCT TACCTTAATC TTCACATTGT  50
TAAGCGGTAT CTATATCTAC CGCCACATGC CGCAGGAAGA TAAATCCTAA  100
<210> 6459
<211> 100
<212> DNA
<213> Escherichia coli K-12 MG1655
<220> ycib b1255
<222> 414-513
<223> ycib b1255 U00096 complement(1310375..1310914)
<313> 6459: 463-482
<400> 6459
ATTCTGGCTG CCGCAAAATA TTTGGGTCAA CTTTAAAGTC TTTGGCCTGA  50
CCGCCCTTAC CTTAATCTTC ACATTGTTAA GCGGTATCTA TATCTACCGC  100
<210> 6460
<211> 100
<212> DNA
<213> Escherichia coli K-12 MG1655
<220> ycib b1255
<222> 372-471
<223> ycib b1255 U00096 complement(1310375..1310914)
<313> 6460: 411-430
<400> 6460
TGTTTTCTTT ATCCTTTGCG GTCTGGCAAA CATCTACATC GCATTCTGGC  50
TGCCGCAAAA TATTTGGGTC AACTTTAAAG TCTTTGGCCT GACCGCCCTT  100
<210> 6461
<211> 100
<212> DNA
<213> Escherichia coli K-12 MG1655
<220> atoc b2221
<222> 1253-1352
<223> atoc b2221 U00096 2319886..2321271

<313> 6461: 1309-1328
<400> 6461
AAAAACGCAT CATTATGGAA GTGCTGGAAC AACAAGAAGG AAACCGAACC 50
CGCACTGCTT TAATGCTGGG CATCAGTCGC CGTGCATTGA TGTATAAACT 100
<210> 6462
<211> 100
<212> DNA
<213> Escherichia coli K-12 MG1655
<220> atoc b2221
<222> 1201-1300
<223> atoc b2221 U00096 2319886..2321271
<313> 6462: 1262-1281
<400> 6462
AAAACAGCCC CTGTCGGTGA GCGTAATTTA AAAGAGGAAA TTAAACGCGT 50
CGAAAAACGC ATCATTATGG AAGTGCTGGA ACAACAAGAA GGAAACCGAA 100
<210> 6463
<211> 100
<212> DNA
<213> Escherichia coli K-12 MG1655
<220> atoc b2221
<222> 1184-1283
<223> atoc b2221 U00096 2319886..2321271
<313> 6463: 1213-1232
<400> 6463
GTAATGCTGG CGAGGTAAAA ACAGCCCCTG TCGGTGAGCG TAATTTAAAA 50
GAGGAAATTA AACGCGTCGA AAAACGCATC ATTATGGAAG TGCTGGAACA 100
<210> 6464
<211> 100
<212> DNA
<213> Escherichia coli K-12 MG1655
<220> ycic b1256
<222> 645-744
<223> ycic b1256 U00096 complement(1310944..1311687)
<313> 6464: 721-740
<400> 6464
CCCGGAAATT GGTGCCGTAC TGGCGAACAC CTTGAGCAAC CTGATTTCAG 50
CCATATTGCT CATCTATCTG TTCCGCCTGT ATATGTTGAT TCGCCAATAA 100
<210> 6465
<211> 100
<212> DNA
<213> Escherichia coli K-12 MG1655
<220> ycic b1256
<222> 641-740
<223> ycic b1256 U00096 complement(1310944..1311687)
<313> 6465: 678-697
<400> 6465
TAACCCCGGA AATTGGTGCC GTACTGGCGA ACACCTTGAG CAACCTGATT 50
TCAGCCATAT TGCTCATCTA TCTGTTCCGC CTGTATATGT TGATTCGCCA 100
<210> 6466
<211> 100
<212> DNA
<213> Escherichia coli K-12 MG1655
<220> ycic b1256
<222> 604-703
<223> ycic b1256 U00096 complement(1310944..1311687)
<313> 6466: 629-648
<400> 6466
ACACTGTTGC TGCTTTTTGC CTCTTCTTTT GCCGCATTAA CCCCGGAAAT 50
TGGTGCCGTA CTGGCGAACA CCTTGAGCAA CCTGATTTCA GCCATATTGC 100
<210> 6467
<211> 100
<212> DNA
<213> Escherichia coli K-12 MG1655
<220> atod b2222
<222> 564-663
<223> atod b2222 U00096 2321467..2322129
<313> 6467: 628-647
<400> 6467
GCCAGATGAA CTGGTCGAAA CCGGCGAGCT GCAACCTGAC CATATTGTCA 50
CCCCTGGTGC CGTTATCGAC CACATCATCG TTTCACAGGA GAGCAAATAA 100
<210> 6468

<211> 100
<212> DNA
<213> Escherichia coli K-12 MG1655
<220> atod b2222
<222> 545-644
<223> atod b2222 U00096 2321467..2322129
<313> 6468: 595-614
<400> 6468
CTGATATCAC GCTGGTAGAG CCAGATGAAC TGGTCGAAAC CGGCGAGCTG 50
CAACCTGACC ATATTGTCAC CCCTGGTGCC GTTATCGACC ACATCATCGT 100
<210> 6469
<211> 100
<212> DNA
<213> Escherichia coli K-12 MG1655
<220> atod b2222
<222> 490-589
<223> atod b2222 U00096 2321467..2322129
<313> 6469: 510-529
<400> 6469
AACCTGACCT ATCAACTTAG CGCCCGCAAC TTTAACCCCC TGATAGCCCT 50
TGCGGCTGAT ATCACGCTGG TAGAGCCAGA TGAACTGGTC GAAACCGGCG 100
<210> 6470
<211> 100
<212> DNA
<213> Escherichia coli K-12 MG1655
<220> ycid b1257
<222> 423-522
<223> ycid b1257 U00096 1312044..1312682
<313> 6470: 472-491
<400> 6470
GGCAGGGCTT TCCGATCTCA GTCTGAAAGA TTCCTGGGGA GCTGCCGGGC 50
AGGTGGGGGT TGATTATCTG ATTAACCGTG ACTGGTTGGT TAACATGTCA 100
<210> 6471
<211> 100
<212> DNA
<213> Escherichia coli K-12 MG1655
<220> ycid b1257
<222> 377-476
<223> ycid b1257 U00096 1312044..1312682
<313> 6471: 423-442
<400> 6471
ACTACACCAC CTTCTTTGAT AATGGATTTA ACGATCATGG CAAAGAGGCA 50
GGGCTTTCCG ATCTCAGTCT GAAAGATTCC TGGGGAGCTG CCGGGCAGGT 100
<210> 6472
<211> 100
<212> DNA
<213> Escherichia coli K-12 MG1655
<220> ycid b1257
<222> 323-422
<223> ycid b1257 U00096 1312044..1312682
<313> 6472: 368-387
<400> 6472
GGTATTTTGG TGATGCCAGC AGCAAATTCC GTCCTTACGT TGGGGCAGGT 50
ATTAACTACA CCACCTTCTT TGATAATGGA TTTAACGATC ATGGCAAAGA 100
<210> 6473
<211> 100
<212> DNA
<213> Escherichia coli K-12 MG1655
<220> atoe b2224
<222> 1224-1323
<223> atoe b2224 U00096 2322776..2324098
<313> 6473: 1287-1306
<400> 6473
GGCAATCGCC GGACTCGGTG TCCGCGACAT CATGGGCTAC TGCATCACTG 50
CCCTGCTCTT CTCCGGTGTC ATTTTCGTCA TTGGTTTAAC GCTGTTCTGA 100
<210> 6474
<211> 100
<212> DNA
<213> Escherichia coli K-12 MG1655
<220> atoe b2224
<222> 1224-1323

<223> atoe b2224 U00096 2322776..2324098
<313> 6474: 1250-1269
<400> 6474
GGCAATCGCC GGACTCGGTG TCCGCGACAT CATGGGCTAC TGCATCACTG 50
CCCTGCTCTT CTCCGGTGTC ATTTTCGTCA TTGGTTTAAC GCTGTTCTGA 100
<210> 6475
<211> 100
<212> DNA
<213> Escherichia coli K-12 MG1655
<220> atoe b2224
<222> 1142-1241
<223> atoe b2224 U00096 2322776..2324098
<313> 6475: 1200-1219
<400> 6475
ATCTCGGTAA ATCGGTAATG GCGATCGCCT ACGGCGAGCA ATGGATGAAC 50
ATGGCACAAC CATTCTGGGC GCTGCCAGCA CTGGCAATCG CCGGACTCGG 100
<210> 6476
<211> 100
<212> DNA
<213> Escherichia coli K-12 MG1655
<220> ycie b1258
<222> 389-488
<223> ycie b1258 U00096 complement(1312742..1313248)
<313> 6476: 421-440
<400> 6476
TCGAAGCGAT TTTAAATGAG GAAAAGCAAA TGGCCGACTG GCTGATTCAG 50
AATATTCCGC AAACAACTGA GAAATTTTTA ATTCGCTCTG AAACTGATGG 100
<210> 6477
<211> 100
<212> DNA
<213> Escherichia coli K-12 MG1655
<220> ycie b1258
<222> 339-438
<223> ycie b1258 U00096 complement(1312742..1313248)
<313> 6477: 368-387
<400> 6477
ACTATTAGCA GCAGCAAAAA ATGCCGGTGA TACAGCTTCA ATTCCAACCA 50
TCGAAGCGAT TTTAAATGAG GAAAAGCAAA TGGCCGACTG GCTGATTCAG 100
<210> 6478
<211> 100
<212> DNA
<213> Escherichia coli K-12 MG1655
<220> ycie b1258
<222> 300-399
<223> ycie b1258 U00096 complement(1312742..1313248)
<313> 6478: 338-357
<400> 6478
ATATGTCTTC GAGCAATTTG AAATCGCCTG TTACACCTCA CTATTAGCAG 50
CAGCAAAAAA TGCCGGTGAT ACAGCTTCAA TTCCAACCAT CGAAGCGATT 100
<210> 6479
<211> 100
<212> DNA
<213> Escherichia coli K-12 MG1655
<220> ycif b1259
<222> 391-490
<223> ycif b1259 U00096 complement(1313294..1313794)
<313> 6479: 438-457
<400> 6479
AAAGCAGCGA AGCTTCTGAA AGAAACCCTG GAAGAAGAAA AGGCCACCGA 50
CATCAAACTG ACTGATCTGG CCATTAATAA CGTAAATAAG AAAGCCGAAA 100
<210> 6480
<211> 100
<212> DNA
<213> Escherichia coli K-12 MG1655
<220> ycif b1259
<222> 317-416
<223> ycif b1259 U00096 complement(1313294..1313794)
<313> 6480: 382-401
<400> 6480
CACAGAAAGT CGAGCATTAT GAGATTGCCA GTTACGGGAC ATTAGCGACG 50
CTGGCTGAAC AATTAGGTTA CCGTAAAGCA GCGAAGCTTC TGAAAGAAAC 100

<210> 6481
<211> 100
<212> DNA
<213> Escherichia coli K-12 MG1655
<220> ycif b1259
<222> 275-374
<223> ycif b1259 U00096 complement(1313294..1313794)
<313> 6481: 333-352
<400> 6481
CCGAGAAAAA CGAAGTGCGT GATGCCGCAC TGATTGCCGC AGCACAGAAA 50
GTCGAGCATT ATGAGATTGC CAGTTACGGG ACATTAGCGA CGCTGGCTGA 100
<210> 6482
<211> 100
<212> DNA
<213> Escherichia coli K-12 MG1655
<220> ycig b1260
<222> 138-237
<223> ycig b1260 U00096 complement(1313880..1314116)
<313> 6482: 199-218
<400> 6482
GCATAGCGGC GGTAATTTTA AAAATGATCC GCAACGCGCA TCTGAAGCGG 50
GTAAAAAAGG CGGTCAACAA AGCGGTGGTA ATAAATCAGG CAAATCCTGA 100
<210> 6483
<211> 100
<212> DNA
<213> Escherichia coli K-12 MG1655
<220> ycig b1260
<222> 100-199
<223> ycig b1260 U00096 complement(1313880..1314116)
<313> 6483: 129-148
<400> 6483
CGTGAGAAGG CATCCGACGC AGGCCGTAAA GGCGGTCAGC ATAGCGGCGG 50
TAATTTTAAA AATGATCCGC AACGCGCATC TGAAGCGGGT AAAAAAGGCG 100
<210> 6484
<211> 100
<212> DNA
<213> Escherichia coli K-12 MG1655
<220> ycig b1260
<222> 1-100
<223> ycig b1260 U00096 complement(1313880..1314116)
<313> 6484: 41-60
<400> 6484
GTGTCGACAC ACAACACTCA TATTAATGAA ACAATGCAAC GCAACGGGAG 50
AAATAACATG GCCGAACATC GTGGTGGTTC AGGAAATTTC GCCGAAGACC 100
<210> 6485
<211> 100
<212> DNA
<213> Escherichia coli K-12 MG1655
<220> ycih b1283
<222> 231-330
<223> ycih b1283 U00096 1340679..1341008
<313> 6485: 300-319
<400> 6485
AGTTAAAGAT GGAGTTATTG AAATCCAGGG CGATAAGCGT GATTTATTAA 50
AATCACTACT TGAAGCGAAA GGGATGAAGG TAAAACTCGC AGGCGGTTAA 100
<210> 6486
<211> 100
<212> DNA
<213> Escherichia coli K-12 MG1655
<220> ycih b1283
<222> 195-294
<223> ycih b1283 U00096 1340679..1341008
<313> 6486: 242-261
<400> 6486
AGCGGAACTG AAGAAAAAAT GCGGCTGCGG CGGAGCAGTT AAAGATGGAG 50
TTATTGAAAT CCAGGGCGAT AAGCGTGATT TATTAAAATC ACTACTTGAA 100
<210> 6487
<211> 100
<212> DNA
<213> Escherichia coli K-12 MG1655
<220> ycih b1283

<222> 144-243
<223> ycih b1283 U00096 1340679..1341008
<313> 6487: 189-208
<400> 6487
TTGCCTGATT ACCGGTGTCG ATCTCGATGA TGCCGAACTG ACAAAACTTG 50
CAGCGGAACT GAAGAAAAAA TGCGGCTGCG GCGGAGCAGT TAAAGATGGA 100
<210> 6488
<211> 100
<212> DNA
<213> Escherichia coli K-12 MG1655
<220> ycii b1252
<222> 294-393
<223> ycii b1252 U00096 complement(1308593..1308985)
<313> 6488: 354-373
<400> 6488
ATCGCTGGAA GCAGCTCAGG CCTGGGCCGA TGCTGACCCG TACGTAGCGG 50
CAGGAGTCTA TGAACACGTT TCAGTGAAAC CATTTAAGAA AGTGTTCTGA 100
<210> 6489
<211> 100
<212> DNA
<213> Escherichia coli K-12 MG1655
<220> ycii b1252
<222> 235-334
<223> ycii b1252 U00096 complement(1308593..1308985)
<313> 6489: 276-295
<400> 6489
GACAGTAACG ATCCAGGGGC TGCGGGATTT ACTGGCTCAA CCGTTATTGC 50
TGAATTTGAA TCGCTGGAAG CAGCTCAGGC CTGGGCCGAT GCTGACCCGT 100
<210> 6490
<211> 100
<212> DNA
<213> Escherichia coli K-12 MG1655
<220> ycii b1252
<222> 196-295
<223> ycii b1252 U00096 complement(1308593..1308985)
<313> 6490: 237-256
<400> 6490
GAAGGTCGTT TGTTGACAGC TGGTCCAATG CCAGCGGTAG ACAGTAACGA 50
TCCAGGGGCT GCGGGATTTA CTGGCTCAAC CGTTATTGCT GAATTTGAAT 100
<210> 6491
<211> 100
<212> DNA
<213> Escherichia coli K-12 MG1655
<220> ycik b1272
<222> 660-759
<223> ycik b1272 U00096 complement(1326378..1327136)
<313> 6491: 723-742
<400> 6491
CATGCCGCTC TACCTCTGGC TGATGGGCGA TGACAGCCGC CGTAAAACCG 50
GCATGACCTT TGACGCCCAA CCGGGCCGTA AACCAGGAAT TTCCCAATGA 100
<210> 6492
<211> 100
<212> DNA
<213> Escherichia coli K-12 MG1655
<220> ycik b1272
<222> 638-737
<223> ycik b1272 U00096 complement(1326378..1327136)
<313> 6492: 671-690
<400> 6492
AACTTAAAAC ACCCGCTGAT ATCATGCCGC TCTACCTCTG GCTGATGGGC 50
GATGACAGCC GCCGTAAAAC CGGCATGACC TTTGACGCCC AACCGGGCCG 100
<210> 6493
<211> 100
<212> DNA
<213> Escherichia coli K-12 MG1655
<220> ycik b1272
<222> 598-697
<223> ycik b1272 U00096 complement(1326378..1327136)
<313> 6493: 626-645
<400> 6493
GCAATGCGTG CCAGCGCCTT CCCGACCGAA GATCCACAGA AACTTAAAAC 50

ACCCGCTGAT ATCATGCCGC TCTACCTCTG GCTGATGGGC GATGACAGCC 100
<210> 6494
<211> 100
<212> DNA
<213> Escherichia coli K-12 MG1655
<220> b0941 b0941
<222> 972-1071
<223> b0941 b0941 U00096 1001030..1002100
<313> 6494: 1030-1049
<400> 6494
TATCGCGCGA ATGAATGTCT ATCCAGTCAG CACGACAGGT GTACCACCAG 50
AAACCGGGCG ATTTGAAGCC ACGGCAACGG TGAGAATAAA TTTTGATTAA 100
<210> 6495
<211> 100
<212> DNA
<213> Escherichia coli K-12 MG1655
<220> b0941 b0941
<222> 889-988
<223> b0941 b0941 U00096 1001030..1002100
<313> 6495: 947-966
<400> 6495
GTGGTGGTGA CCGATAGCCA GAACAACATT ATTTCCCCTG CTGGTGGAAC 50
ATTACCGCTC TCTATTCCTG ATGATGCAGA CAGTATCGCG CGAATGAATG 100
<210> 6496
<211> 100
<212> DNA
<213> Escherichia coli K-12 MG1655
<220> b0941 b0941
<222> 856-955
<223> b0941 b0941 U00096 1001030..1002100
<313> 6496: 910-929
<400> 6496
CAGGCGATTA AGACGTCACG TCCTGGCGTG GGCGTGGTGG TGACCGATAG 50
CCAGAACAAC ATTATTTCCC CTGCTGGTGG AACATTACCG CTCTCTATTC 100
<210> 6497
<211> 100
<212> DNA
<213> Escherichia coli K-12 MG1655
<220> ycil b1270
<222> 750-849
<223> ycil b1270 U00096 1324876..1325751
<313> 6497: 766-785
<400> 6497
CAGCTCTAAA GTCGCTGTAG AAAAAGACCG TCGTCGCATG AAGGCGAATC 50
AGATTCGTCG TGCGGTGAAA CGTCACAGTC AGGTGAGCGG CGGTCGTCGT 100
<210> 6498
<211> 100
<212> DNA
<213> Escherichia coli K-12 MG1655
<220> ycil b1270
<222> 675-774
<223> ycil b1270 U00096 1324876..1325751
<313> 6498: 712-731
<400> 6498
ACGCGGTGGC TGGACGGAGC TGGACCTCGC CCAGACTAAC TATCTGCGCG 50
AACTGGTGGA GCTACCGCCA GAAACCAGCT CTAAAGTCGC TGTAGAAAAA 100
<210> 6499
<211> 100
<212> DNA
<213> Escherichia coli K-12 MG1655
<220> ycil b1270
<222> 645-744
<223> ycil b1270 U00096 1324876..1325751
<313> 6499: 664-683
<400> 6499
CGGTGATATC CCACTGCCAA AAGGTCTGCC ACGCGGTGGC TGGACGGAGC 50
TGGACCTCGC CCAGACTAAC TATCTGCGCG AACTGGTGGA GCTACCGCCA 100
<210> 6500
<211> 100
<212> DNA
<213> Escherichia coli K-12 MG1655

<220> b1671 b1671
<222> 621-720
<223> b1671 b1671 U00096 complement(1748369..1749088)
<313> 6500: 662-681
<400> 6500
GCAGCAAAAT AAATACTATC AATATCAGCT ACCTGGTGCC GGGAAACCGC  50
ATCTGTATCG CCGGTTCGGT CAACATTTGA TTAAAAAGGA AAATGTATGA  100
<210> 6501
<211> 100
<212> DNA
<213> Escherichia coli K-12 MG1655
<220> b1671 b1671
<222> 532-631
<223> b1671 b1671 U00096 complement(1748369..1749088)
<313> 6501: 559-578
<400> 6501
AAAGGCTTTC CGCCCATTTG CGTCAGCGCC TGCCCGGAAC ACGCATTGAT  50
CTTTGGTCGC GAAGATAGCC CAGAAATTCA GGCGTGGTTG CAGCAAAATA  100
<210> 6502
<211> 100
<212> DNA
<213> Escherichia coli K-12 MG1655
<220> b1671 b1671
<222> 477-576
<223> b1671 b1671 U00096 complement(1748369..1749088)
<313> 6502: 514-533
<400> 6502
CCCTGTCACT AAAGTGGCAG ATAAATGTGA TTTCTGCGCT GAGTCCCGAC  50
TGGCAAAAGG CTTTCCGCCC ATTTGCGTCA GCGCCTGCCC GGAACACGCA  100
<210> 6503
<211> 100
<212> DNA
<213> Escherichia coli K-12 MG1655
<220> b0942 b0942
<222> 444-543
<223> b0942 b0942 U00096 1002112..1002654
<313> 6503: 508-527
<400> 6503
TAACACACTC AAATATCAAC TTCGTTATAA GTCCACAAAG GCGGGAGCAA  50
CGGGCGGTAA TGCGACGGCG GTTCTCTATT TTGATCTGGT TTACCAGTGA  100
<210> 6504
<211> 100
<212> DNA
<213> Escherichia coli K-12 MG1655
<220> b0942 b0942
<222> 434-533
<223> b0942 b0942 U00096 1002112..1002654
<313> 6504: 466-485
<400> 6504
AAGCCGGGGA TAACACACTC AAATATCAAC TTCGTTATAA GTCCACAAAG  50
GCGGGAGCAA CGGGCGGTAA TGCGACGGCG GTTCTCTATT TTGATCTGGT  100
<210> 6505
<211> 100
<212> DNA
<213> Escherichia coli K-12 MG1655
<220> b0942 b0942
<222> 372-471
<223> b0942 b0942 U00096 1002112..1002654
<313> 6505: 419-438
<400> 6505
TCTTGATGCG CAAAGTCAGC AAGAAATCCC GCTCAATCAG GTCCAGCCTC  50
TTACGCCCTT AAAAGCCGGG GATAACACAC TCAAATATCA ACTTCGTTAT  100
<210> 6506
<211> 100
<212> DNA
<213> Escherichia coli K-12 MG1655
<220> ycim b1281
<222> 1039-1138
<223> ycim b1281 U00096 1338582..1339751
<313> 6506: 1100-1119
<400> 6506

GAGAAGGTAC GTAGTAAGCC TCGTTATCGT TGCCAGAAAT GTGGTTTTAC 50
CGCATACACC CTCTACTGGC ATTGTCCGTC TTGTCGGGCC TGGTCAACCA 100
<210> 6507
<211> 100
<212> DNA
<213> Escherichia coli K-12 MG1655
<220> ycim b1281
<222> 1031-1130
<223> ycim b1281 U00096 1338582..1339751
<313> 6507: 1063-1082
<400> 6507
TGGTTGGCGA GAAGGTACGT AGTAAGCCTC GTTATCGTTG CCAGAAATGT 50
GGTTTTACCG CATACACCCT CTACTGGCAT TGTCCGTCTT GTCGGGCCTG 100
<210> 6508
<211> 100
<212> DNA
<213> Escherichia coli K-12 MG1655
<220> ycim b1281
<222> 977-1076
<223> ycim b1281 U00096 1338582..1339751
<313> 6508: 1013-1032
<400> 6508
TAAATGAAGC GGAAGAAGGG CGTGCCAAAG AGAGCCTGAT GGTGCTGCGT 50
GACATGGTTG GCGAGAAGGT ACGTAGTAAG CCTCGTTATC GTTGCCAGAA 100
<210> 6509
<211> 100
<212> DNA
<213> Escherichia coli K-12 MG1655
<220> b1672 b1672
<222> 549-648
<223> b1672 b1672 U00096 complement(1749101..1749748)
<313> 6509: 622-641
<400> 6509
GACGGCGGTG TTGTACTTGT TTAGCGAGCG ATTTATGACC TATGGCAAGG 50
CTTATGGCTT ATGCGAATGG TTCGAGGTAG AGCAGTTTCA GAACCCCTGA 100
<210> 6510
<211> 100
<212> DNA
<213> Escherichia coli K-12 MG1655
<220> b1672 b1672
<222> 524-623
<223> b1672 b1672 U00096 complement(1749101..1749748)
<313> 6510: 573-592
<400> 6510
CCGATATACG GCAGGTGGAG TCATCGACGG CGGTGTTGTA CTTGTTTAGC 50
GAGCGATTTA TGACCTATGG CAAGGCTTAT GGCTTATGCG AATGGTTCGA 100
<210> 6511
<211> 100
<212> DNA
<213> Escherichia coli K-12 MG1655
<220> b1672 b1672
<222> 485-584
<223> b1672 b1672 U00096 complement(1749101..1749748)
<313> 6511: 542-561
<400> 6511
AAGCCGCGAT CGCCGCGATG GATGTTGCAC CTGAATATGC CGATATACGG 50
CAGGTGGAGT CATCGACGGC GGTGTTGTAC TTGTTTAGCG AGCGATTTAT 100
<210> 6512
<211> 100
<212> DNA
<213> Escherichia coli K-12 MG1655
<220> b0943 b0944
<222> 465-564
<223> b0943 b0944 U00096 1002614..1003177
<313> 6512: 525-544
<400> 6512
GGTGCCATTA ACTTTTTATG CGCAATTGCG ACCTGTCAAT AGCGATGTGC 50
AGTCGGGTAA AGTGAATGCC AGCGCGACAT TTGTACTTCA TTATGACTAA 100
<210> 6513
<211> 100
<212> DNA

<213> Escherichia coli K-12 MG1655
<220> b0943 b0944
<222> 386-485
<223> b0943 b0944 U00096 1002614..1003177
<313> 6513: 438-457
<400> 6513
CAATTGCGAT TCTGGATGAT GCGAAGATAT TGATTCCGCT TAACCAGGCG 50
AGTAAAGACT ACAGCCTACA TAGCGGTAAG GTGCCATTAA CTTTTTATGC 100
<210> 6514
<211> 100
<212> DNA
<213> Escherichia coli K-12 MG1655
<220> b0943 b0944
<222> 323-422
<223> b0943 b0944 U00096 1002614..1003177
<313> 6514: 361-380
<400> 6514
CCGACGGAGT GCAGAGCGAA ATGTTGTCCA TTGAAAGTGG TACGGATGCA 50
GCCAGCGGGC TGGCAATTGC GATTCTGGAT GATGCGAAGA TATTGATTCC 100
<210> 6515
<211> 100
<212> DNA
<213> Escherichia coli K-12 MG1655
<220> ycin b1274
<222> 153-252
<223> ycin b1274 U00096 complement(1328441..1328692)
<313> 6515: 218-237
<400> 6515
AGATGAACAA GGATTGCCAA CAGCCAAAAG CACCGCGGTG TTTAATATGT 50
TCAAACACCT GGCCCATGTG CTTTCAGAAA AATATCATCT GGTTGATTAA 100
<210> 6516
<211> 100
<212> DNA
<213> Escherichia coli K-12 MG1655
<220> ycin b1274
<222> 105-204
<223> ycin b1274 U00096 complement(1328441..1328692)
<313> 6516: 163-182
<400> 6516
CGTCACGCAG CGTAATGGCG TGCTGGTATT TACCGGCGAC TACTTTTTAG 50
ATGAACAAGG ATTGCCAACA GCCAAAAGCA CCGCGGTGTT TAATATGTTC 100
<210> 6517
<211> 100
<212> DNA
<213> Escherichia coli K-12 MG1655
<220> ycin b1274
<222> 43-142
<223> ycin b1274 U00096 complement(1328441..1328692)
<313> 6517: 97-116
<400> 6517
AAAGAGGCCA ACAAAATCAT TCGTGAGCAT GAGGATACGC TCGCAGGGAT 50
TGAAGCCACC GGCGTCACGC AGCGTAATGG CGTGCTGGTA TTTACCGGCG 100
<210> 6518
<211> 100
<212> DNA
<213> Escherichia coli K-12 MG1655
<220> b1673 b1673
<222> 2004-2103
<223> b1673 b1673 U00096 complement(1749752..1751854)
<313> 6518: 2080-2099
<400> 6518
GGACCCACAG CTTGGTTGTC CAACCCGCGA AACATTGCAG CGTCTGGGGC 50
TGGAAGATAT TGCCGCCGAT CTGGCAGCAC ACAATCTACT GCCTGCGTAA 100
<210> 6519
<211> 100
<212> DNA
<213> Escherichia coli K-12 MG1655
<220> b1673 b1673
<222> 2001-2100
<223> b1673 b1673 U00096 complement(1749752..1751854)
<313> 6519: 2030-2049

<400> 6519
CTGGGACCCA CAGCTTGGTT GTCCAACCCG CGAAACATTG CAGCGTCTGG 50
GGCTGGAAGA TATTGCCGCC GATCTGGCAG CACACAATCT ACTGCCTGCG 100
<210> 6520
<211> 100
<212> DNA
<213> Escherichia coli K-12 MG1655
<220> b1673 b1673
<222> 1962-2061
<223> b1673 b1673 U00096 complement(1749752..1751854)
<313> 6520: 1997-2016
<400> 6520
TATGCATGCT TCGCTCACGA TGTTCTACAA AGAAATGGGC TGGGACCCAC 50
AGCTTGGTTG TCCAACCCGC GAAACATTGC AGCGTCTGGG GCTGGAAGAT 100
<210> 6521
<211> 100
<212> DNA
<213> Escherichia coli K-12 MG1655
<220> ycio b1268
<222> 558-657
<223> ycio b1268 U00096 1322086..1322742
<313> 6521: 634-653
<400> 6521
CGGCTATCTC GGGCAGAAAC CGACAACGGT TATTGATCTT ACCGATGATA 50
CGCCGGTCGT GGTGCGTGAA GGCGTAGGTG ATGTGAAGCC TTTCTTATAA 100
<210> 6522
<211> 100
<212> DNA
<213> Escherichia coli K-12 MG1655
<220> ycio b1268
<222> 543-642
<223> ycio b1268 U00096 1322086..1322742
<313> 6522: 584-603
<400> 6522
TTTGATTATT CACGGCGGCT ATCTCGGGCA GAAACCGACA ACGGTTATTG 50
ATCTTACCGA TGATACGCCG GTCGTGGTGC GTGAAGGCGT AGGTGATGTG 100
<210> 6523
<211> 100
<212> DNA
<213> Escherichia coli K-12 MG1655
<220> ycio b1268
<222> 504-603
<223> ycio b1268 U00096 1322086..1322742
<313> 6523: 534-553
<400> 6523
CCCGGAAGAA ATTAAAGATC GTCTGGAAAA ACAGGTAGAT TTGATTATTC 50
ACGGCGGCTA TCTCGGGCAG AAACCGACAA CGGTTATTGA TCTTACCGAT 100
<210> 6524
<211> 100
<212> DNA
<213> Escherichia coli K-12 MG1655
<220> b1674 b1674
<222> 528-627
<223> b1674 b1674 U00096 complement(1751875..1752501)
<313> 6524: 594-613
<400> 6524
AAGTAAAAAA TCCTCGAAAT GTGTGTTATG CGGTGAATGC GCAAACGCCT 50
GCCCAACAGG GGCGTTAAAA ATTATCGAGT GGAAAGATAT TACTGTGTGA 100
<210> 6525
<211> 100
<212> DNA
<213> Escherichia coli K-12 MG1655
<220> b1674 b1674
<222> 395-494
<223> b1674 b1674 U00096 complement(1751875..1752501)
<313> 6525: 458-477
<400> 6525
TGAACGTCTG CCCGATTGGT GCGATTACCT GGCAGCAGAA AGAAGGCTGT 50
ATTACCGTCG ATCATAAACG CTGTATTGGC TGTAGCGCCT GTACTACGGC 100
<210> 6526
<211> 100

<212> DNA
<213> Escherichia coli K-12 MG1655
<220> b1674 b1674
<222> 342-441
<223> b1674 b1674 U00096 complement(1751875..1752501)
<313> 6526: 380-399
<400> 6526
CGATCTCAAC TATACCGCGG ACACCTGCCG TCAATGCAAA GAGCCGCAAT 50
GCATGAACGT CTGCCCGATT GGTGCGATTA CCTGGCAGCA GAAAGAAGGC 100
<210> 6527
<211> 100
<212> DNA
<213> Escherichia coli K-12 MG1655
<220> b1675 b1676
<222> 111-210
<223> b1675 b1676 U00096 complement(1752956..1753165)
<313> 6527: 186-205
<400> 6527
CGCAGGCGTA TTACGTACAG CATTAGCCAA TAAACGCATT CAGCGCAGTG 50
AGCTGGAAAA GCAGGCGATG GAAACCGTCA TTAACGCACT GGTAAAATGA 100
<210> 6528
<211> 100
<212> DNA
<213> Escherichia coli K-12 MG1655
<220> b1675 b1676
<222> 111-210
<223> b1675 b1676 U00096 complement(1752956..1753165)
<313> 6528: 146-165
<400> 6528
CGCAGGCGTA TTACGTACAG CATTAGCCAA TAAACGCATT CAGCGCAGTG 50
AGCTGGAAAA GCAGGCGATG GAAACCGTCA TTAACGCACT GGTAAAATGA 100
<210> 6529
<211> 100
<212> DNA
<213> Escherichia coli K-12 MG1655
<220> b1675 b1676
<222> 73-172
<223> b1675 b1676 U00096 complement(1752956..1753165)
<313> 6529: 103-122
<400> 6529
ATGCGCGATC TGGGGCAGGA ACCAAAGCAT ATTGTTATCG CAGGCGTATT 50
ACGTACAGCA TTAGCCAATA AACGCATTCA GCGCAGTGAG CTGGAAAAGC 100
<210> 6530
<211> 100
<212> DNA
<213> Escherichia coli K-12 MG1655
<220> yciq b1269
<222> 1797-1896
<223> yciq b1269 U00096 1322770..1324665
<313> 6530: 1863-1882
<400> 6530
GCACCTAAGT TGCGGGGCCA ATACCAGTTT GTTGGGGCGA AGTTATTCCG 50
GTGGTGGTGC TGGCGGCGGC GCGGGTGGCG GAGGCGGTGG TGGCTGGTAA 100
<210> 6531
<211> 100
<212> DNA
<213> Escherichia coli K-12 MG1655
<220> yciq b1269
<222> 1786-1885
<223> yciq b1269 U00096 1322770..1324665
<313> 6531: 1822-1841
<400> 6531
TCATTACAGA CGCACCTAAG TTGCGGGGCC AATACCAGTT TGTTGGGGCG 50
AAGTTATTCC GGTGGTGGTG CTGGCGGCGG CGCGGGTGGC GGAGGCGGTG 100
<210> 6532
<211> 100
<212> DNA
<213> Escherichia coli K-12 MG1655
<220> yciq b1269
<222> 1749-1848
<223> yciq b1269 U00096 1322770..1324665

<313> 6532: 1795-1814
<400> 6532
TCGCGCTCCA GAGATTGCCC GTAACGGCGT TTTATTCTCA TTACAGACGC 50
ACCTAAGTTG CGGGGCCAAT ACCAGTTTGT TGGGGCGAAG TTATTCCGGT 100
<210> 6533
<211> 100
<212> DNA
<213> Escherichia coli K-12 MG1655
<220> b0947 b0948
<222> 1008-1107
<223> b0947 b0948 U00096 complement(1005714..1006823)
<313> 6533: 1029-1048
<400> 6533
AGAAGGCGAA GTCACGCCGC TGAAAAAATC AGCAATGGGC GATGATGGCA 50
CCATTCTTTG CTGTAGCTGT GTACCGAAGA CTGCACTTAA GTTGGCGCGT 100
<210> 6534
<211> 100
<212> DNA
<213> Escherichia coli K-12 MG1655
<220> b0947 b0948
<222> 884-983
<223> b0947 b0948 U00096 complement(1005714..1006823)
<313> 6534: 919-938
<400> 6534
GGCAGGGACA GGCATTTCGT GGAAATAACC AACAGGTGTT GCTGGAGCAA 50
TTAGAAAATC AGGGAATTCG TATCCCTTAT TCTTGCCGCG CGGGCATTTG 100
<210> 6535
<211> 100
<212> DNA
<213> Escherichia coli K-12 MG1655
<220> b0947 b0948
<222> 829-928
<223> b0947 b0948 U00096 complement(1005714..1006823)
<313> 6535: 879-898
<400> 6535
GCTGATGATA CCGCCAACAT CACGCAACAA CCGGACGCAA ATGTAGATAT 50
TGACTGGCAG GGACAGGCAT TTCGTGGAAA TAACCAACAG GTGTTGCTGG 100
<210> 6536
<211> 100
<212> DNA
<213> Escherichia coli K-12 MG1655
<220> ycir b1286
<222> 1887-1986
<223> ycir b1286 U00096 complement(1342781..1344766)
<313> 6536: 1948-1967
<400> 6536
CAAGAACGGG ATCAATGAGC GGCAAGGATT TTTGTTTGCC AAACCGATGC 50
CCGCCGTCGC CTTCGAACGC TGGTATAAAC GCTATCTGAA GCGCGCATAA 100
<210> 6537
<211> 100
<212> DNA
<213> Escherichia coli K-12 MG1655
<220> ycir b1286
<222> 1865-1964
<223> ycir b1286 U00096 complement(1342781..1344766)
<313> 6537: 1895-1914
<400> 6537
CAAAGGAAGA TGCTTTTTTA ACCAAGAACG GGATCAATGA GCGGCAAGGA 50
TTTTTGTTTG CCAAACCGAT GCCCGCCGTC GCCTTCGAAC GCTGGTATAA 100
<210> 6538
<211> 100
<212> DNA
<213> Escherichia coli K-12 MG1655
<220> ycir b1286
<222> 1749-1848
<223> ycir b1286 U00096 complement(1342781..1344766)
<313> 6538: 1777-1796
<400> 6538
CCAGGTTTTT GTTCGAGATA TTCACAAACA ACCTGTCTCG CAGTCACTGG 50
TCCGGGCGAT CGTCGCTGTG GCCCAGGCAT TGAATCTTCA GGTGATCGCC 100
<210> 6539

<211> 100
<212> DNA
<213> Escherichia coli K-12 MG1655
<220> atos b2220
<222> 1728-1827
<223> atos b2220 U00096 2318063..2319889
<313> 6539: 1786-1805
<400> 6539
TGCCCATCAG GGTGATATTC GCGTCGCCAG TTTGCCGGGC TACGGCGCAA 50
CCTTCACGCT TATTTTACCG ATCAACCCGC AGGGAAATCA GACTGTATGA 100
<210> 6540
<211> 100
<212> DNA
<213> Escherichia coli K-12 MG1655
<220> atos b2220
<222> 1696-1795
<223> atos b2220 U00096 2318063..2319889
<313> 6540: 1744-1763
<400> 6540
CTTGGTCTGG CGTTAAGTCA ACGCATCATT AATGCCCATC AGGGTGATAT 50
TCGCGTCGCC AGTTTGCCGG GCTACGGCGC AACCTTCACG CTTATTTTAC 100
<210> 6541
<211> 100
<212> DNA
<213> Escherichia coli K-12 MG1655
<220> atos b2220
<222> 1638-1737
<223> atos b2220 U00096 2318063..2319889
<313> 6541: 1655-1674
<400> 6541
CTCGCTGCAA AAAAAGATCT TCGATCCCTT TTTCACCACC AAAGCCTCAG 50
GAACCGGGCT TGGTCTGGCG TTAAGTCAAC GCATCATTAA TGCCCATCAG 100
<210> 6542
<211> 100
<212> DNA
<213> Escherichia coli K-12 MG1655
<220> ycis b1280
<222> 210-309
<223> ycis b1280 U00096 1338267..1338575
<313> 6542: 264-283
<400> 6542
CCTGGCGCGC GCTGAACGTA AAATAAAGCG ACTGGAAAAC CAGCTTTCAC 50
CCGCGACTGA CGTGGCTGTA GTGCCGCACT CGTCAGCGGC GAAGGAATAA 100
<210> 6543
<211> 100
<212> DNA
<213> Escherichia coli K-12 MG1655
<220> ycis b1280
<222> 209-308
<223> ycis b1280 U00096 1338267..1338575
<313> 6543: 254-273
<400> 6543
CCCTGGCGCG CGCTGAACGT AAAATAAAGC GACTGGAAAA CCAGCTTTCA 50
CCCGCGACTG ACGTGGCTGT AGTGCCGCAC TCGTCAGCGG CGAAGGAATA 100
<210> 6544
<211> 100
<212> DNA
<213> Escherichia coli K-12 MG1655
<220> ycis b1280
<222> 181-280
<223> ycis b1280 U00096 1338267..1338575
<313> 6544: 235-254
<400> 6544
GGCCTGTTCT GGCTGCGAGT TCGTGTTTCC CTGGCGCGCG CTGAACGTAA 50
AATAAAGCGA CTGGAAAACC AGCTTTCACC CGCGACTGAC GTGGCTGTAG 100
<210> 6545
<211> 100
<212> DNA
<213> Escherichia coli K-12 MG1655
<220> racc b1352
<222> 177-276

<223> racc b1352 U00096 complement(1415512..1415787)
<313> 6545: 228-247
<400> 6545
AAAAATGTGC CTGGTGCATA TCGGAAAGAA TCTGCCCGCA GAAAAAAAAG 50
CCGAATTTCT GGCAACTCTA ATTGCAATGA AATTAAAAGG TGAAATCTGA 100
<210> 6546
<211> 100
<212> DNA
<213> Escherichia coli K-12 MG1655
<220> racc b1352
<222> 173-272
<223> racc b1352 U00096 complement(1415512..1415787)
<313> 6546: 203-222
<400> 6546
TCAAAAAAAT GTGCCTGGTG CATATCGGAA AGAATCTGCC CGCAGAAAAA 50
AAAGCCGAAT TTCTGGCAAC TCTAATTGCA ATGAAATTAA AAGGTGAAAT 100
<210> 6547
<211> 100
<212> DNA
<213> Escherichia coli K-12 MG1655
<220> racc b1352
<222> 154-253
<223> racc b1352 U00096 complement(1415512..1415787)
<313> 6547: 209-228
<400> 6547
AAAACACTTG ATGAAGGTGT CAAAAAAATG TGCCTGGTGC ATATCGGAAA 50
GAATCTGCCC GCAGAAAAAA AAGCCGAATT TCTGGCAACT CTAATTGCAA 100
<210> 6548
<211> 100
<212> DNA
<213> Escherichia coli K-12 MG1655
<220> yciv b1267
<222> 783-882
<223> yciv b1267 U00096 1321244..1322125
<313> 6548: 849-868
<400> 6548
GTGGATCGAA CTGGGTCGTA AACTCTGGCT GCCCGCAGGC GTTGAAGGCG 50
TCTGGCAGCT ATGGGAACAG CCGCAGAACA CCACAGAGAG GGAATTATGA 100
<210> 6549
<211> 100
<212> DNA
<213> Escherichia coli K-12 MG1655
<220> yciv b1267
<222> 771-870
<223> yciv b1267 U00096 1321244..1322125
<313> 6549: 810-829
<400> 6549
TCAGCCATGC CCGTGGATCG AACTGGGTCG TAAACTCTGG CTGCCCGCAG 50
GCGTTGAAGG CGTCTGGCAG CTATGGGAAC AGCCGCAGAA CACCACAGAG 100
<210> 6550
<211> 100
<212> DNA
<213> Escherichia coli K-12 MG1655
<220> yciv b1267
<222> 680-779
<223> yciv b1267 U00096 1321244..1322125
<313> 6550: 744-763
<400> 6550
CGCAGTGCCA GCAATCGCCC AATGAACGTA CCCAGCTGGC GGCCCTTGCG 50
CGTCAGCATC ATTTATGGGC ATCACAAGGA TCTGATTTTC ATCAGCCATG 100
<210> 6551
<211> 100
<212> DNA
<213> Escherichia coli K-12 MG1655
<220> yciw b1288
<222> 1051-1150
<223> yciw b1288 U00096 complement(1347004..1348209)
<313> 6551: 1107-1126
<400> 6551
TGGCTGACCC GAGCACCCGA TCGCTTTAGC GCCGCCCAGT TCAGCCCTTT 50
ACTCGAACAC GAAAAATCAT CAACGCAGAT TATTAATCTG CTGGTATGGA 100

<210> 6552
<211> 100
<212> DNA
<213> Escherichia coli K-12 MG1655
<220> yciw b1288
<222> 1007-1106
<223> yciw b1288 U00096 complement(1347004..1348209)
<313> 6552: 1046-1065
<400> 6552
CTGACCGTCA TTCTGTTGAG CGCGCTACCG TTCAGGCGAT ACAATGGCTG 50
ACCCGAGCAC CCGATCGCTT TAGCGCCGCC CAGTTCAGCC CTTTACTCGA 100
<210> 6553
<211> 100
<212> DNA
<213> Escherichia coli K-12 MG1655
<220> yciw b1288
<222> 949-1048
<223> yciw b1288 U00096 complement(1347004..1348209)
<313> 6553: 995-1014
<400> 6553
GAATATAAAA AAATCAGCAC CCTTATGCGC AAGGGCGAAA ATGAAATTAA 50
CCAATGGGCT GACCGTCATT CTGTTGAGCG CGCTACCGTT CAGGCGATAC 100
<210> 6554
<211> 100
<212> DNA
<213> Escherichia coli K-12 MG1655
<220> atpa b3735
<222> 1443-1542
<223> atpa b3735 U00096 complement(3915944..3917485)
<313> 6554: 1523-1542
<400> 6554
GTTGATGCAA GAGATCAACC AGACCGGTGG CTACAACGAC GAAATCGAAG 50
GCAAGCTGAA AGGCATCCTC GATTCCTTCA AAGCAACCCA ATCCTGGTAA 100
<210> 6555
<211> 100
<212> DNA
<213> Escherichia coli K-12 MG1655
<220> atpa b3735
<222> 1316-1415
<223> atpa b3735 U00096 complement(3915944..3917485)
<313> 6555: 1359-1378
<400> 6555
TGTCCGTTGC GCAGCAGTCT CTGGTTCTGT TCGCAGCAGA ACGTGGTTAC 50
CTGGCGGATG TTGAACTGTC GAAAATTGGC AGCTTCGAAG CCGCTCTGCT 100
<210> 6556
<211> 100
<212> DNA
<213> Escherichia coli K-12 MG1655
<220> atpa b3735
<222> 1253-1352
<223> atpa b3735 U00096 complement(3915944..3917485)
<313> 6556: 1290-1309
<400> 6556
GTAAGCAGCT TGACCACGGT CAGAAAGTGA CCGAACTGCT GAAACAGAAA 50
CAGTATGCGC CGATGTCCGT TGCGCAGCAG TCTCTGGTTC TGTTCGCAGC 100
<210> 6557
<211> 100
<212> DNA
<213> Escherichia coli K-12 MG1655
<220> atpb b3739
<222> 717-816
<223> atpb b3739 U00096 complement(3918864..3919679)
<313> 6557: 746-765
<400> 6557
GCCGTGGGCC ATTTTCCACA TCCTGATCAT TACGCTGCAA GCCTTCATCT 50
TCATGGTTCT GACGATCGTC TATCTGTCGA TGGCGTCTGA AGAACATTAA 100
<210> 6558
<211> 100
<212> DNA
<213> Escherichia coli K-12 MG1655
<220> atpb b3739

<222> 632-731
<223> atpb b3739 U00096 complement(3918864..3919679)
<313> 6558: 660-679
<400> 6558
TGTTCGGTAA CATGTATGCC GGTGAGCTGA TTTTCATTCT GATTGCTGGT 50
CTGTTGCCGT GGTGGTCACA GTGGATCCTG AATGTGCCGT GGGCCATTTT 100
<210> 6559
<211> 100
<212> DNA
<213> Escherichia coli K-12 MG1655
<220> atpb b3739
<222> 585-684
<223> atpb b3739 U00096 complement(3918864..3919679)
<313> 6559: 614-633
<400> 6559
TGAAGGGGTA AGCCTGCTGT CCAAACCAGT TTCACTCGGT TTGCGACTGT 50
TCGGTAACAT GTATGCCGGT GAGCTGATTT TCATTCTGAT TGCTGGTCTG 100
<210> 6560
<211> 100
<212> DNA
<213> Escherichia coli K-12 MG1655
<220> atpc b3732
<222> 303-402
<223> atpc b3732 U00096 complement(3913181..3913600)
<313> 6560: 317-336
<400> 6560
GGCTGAAGAG CACATTAGCA GCTCTCACGG CGACGTAGAT TACGCTCAGG 50
CGTCTGCGGA ACTGGCCAAA GCGATCGCGC AGCTGCGCGT TATCGAGTTG 100
<210> 6561
<211> 100
<212> DNA
<213> Escherichia coli K-12 MG1655
<220> atpc b3732
<222> 172-271
<223> atpc b3732 U00096 complement(3913181..3913600)
<313> 6561: 229-248
<400> 6561
GGTCACGAAG AGTTTATCTA TCTGTCTGGC GGCATTCTTG AAGTGCAGCC 50
TGGCAACGTG ACCGTTCTGG CCGACACCGC AATTCGCGGC CAGGATCTCG 100
<210> 6562
<211> 100
<212> DNA
<213> Escherichia coli K-12 MG1655
<220> atpc b3732
<222> 136-235
<223> atpc b3732 U00096 complement(3913181..3913600)
<313> 6562: 185-204
<400> 6562
ATTAAGCCTG GTATGATTCG CATCGTGAAA CAGCACGGTC ACGAAGAGTT 50
TATCTATCTG TCTGGCGGCA TTCTTGAAGT GCAGCCTGGC AACGTGACCG 100
<210> 6563
<211> 100
<212> DNA
<213> Escherichia coli K-12 MG1655
<220> ycjc b1300
<222> 459-558
<223> ycjc b1300 U00096 1359935..1360492
<313> 6563: 538-557
<400> 6563
GCAAAGTTAT GCCATTAATA CCGGCATCCC GCACAGTTTC AGTAATACGT 50
CGGCAGGTAT TTGCCGAATT ATCAGCGCCC ATACGCCCAC CACGTTTTAA 100
<210> 6564
<211> 100
<212> DNA
<213> Escherichia coli K-12 MG1655
<220> ycjc b1300
<222> 459-558
<223> ycjc b1300 U00096 1359935..1360492
<313> 6564: 487-506
<400> 6564
GCAAAGTTAT GCCATTAATA CCGGCATCCC GCACAGTTTC AGTAATACGT 50

CGGCAGGTAT TTGCCGAATT ATCAGCGCCC ATACGCCCAC CACGTTTTAA 100
<210> 6565
<211> 100
<212> DNA
<213> Escherichia coli K-12 MG1655
<220> ycjc b1300
<222> 402-501
<223> ycjc b1300 U00096 1359935..1360492
<313> 6565: 447-466
<400> 6565
ACTGGAAGGT GAAATTGTTC TGACGATTAA TGGTCAGGAT TACCACCTCG 50
TCGCGGGGCA AAGTTATGCC ATTAATACCG GCATCCCGCA CAGTTTCAGT 100
<210> 6566
<211> 100
<212> DNA
<213> Escherichia coli K-12 MG1655
<220> atpd b3733
<222> 1284-1383
<223> atpd b3733 U00096 complement(3913621..3915003)
<313> 6566: 1359-1378
<400> 6566
TAAAGGCATC ATGGAAGGCG AATACGATCA CCTGCCGGAG CAGGCGTTCT 50
ACATGGTCGG TTCCATCGAA GAAGCTGTGG AAAAAGCCAA AAAACTTTAA 100
<210> 6567
<211> 100
<212> DNA
<213> Escherichia coli K-12 MG1655
<220> atpd b3733
<222> 1255-1354
<223> atpd b3733 U00096 complement(3913621..3915003)
<313> 6567: 1306-1325
<400> 6567
GTCTCCCTGA AAGACACCAT CCGTGGCTTT AAAGGCATCA TGGAAGGCGA 50
ATACGATCAC CTGCCGGAGC AGGCGTTCTA CATGGTCGGT TCCATCGAAG 100
<210> 6568
<211> 100
<212> DNA
<213> Escherichia coli K-12 MG1655
<220> atpd b3733
<222> 1204-1303
<223> atpd b3733 U00096 complement(3913621..3915003)
<313> 6568: 1255-1274
<400> 6568
TCCCAGCCGT TCTTCGTGGC AGAAGTATTC ACCGGTTCTC CGGGTAAATA 50
CGTCTCCCTG AAAGACACCA TCCGTGGCTT TAAAGGCATC ATGGAAGGCG 100
<210> 6569
<211> 100
<212> DNA
<213> Escherichia coli K-12 MG1655
<220> ycjd b1290
<222> 150-249
<223> ycjd b1290 U00096 complement(1349431..1349784)
<313> 6569: 179-198
<400> 6569
TGCTTGCTGC TCGGCGCGTG TAGTCGTTGA GCTGGATGGT GGGCAGCATG 50
ATTTAGCAGT TGCCTATGAT TCCAGGCGCA CTAGCTGGCT TGAGTCGCAG 100
<210> 6570
<211> 100
<212> DNA
<213> Escherichia coli K-12 MG1655
<220> ycjd b1290
<222> 85-184
<223> ycjd b1290 U00096 complement(1349431..1349784)
<313> 6570: 133-152
<400> 6570
AGCCGACGTT TTAGTGATTT CAAATTTCGC CGTCAACATC CAGTGGGGAG 50
CTACATTCTC GATTTTGCTT GCTGCTCGGC GCGTGTAGTC GTTGAGCTGG 100
<210> 6571
<211> 100
<212> DNA
<213> Escherichia coli K-12 MG1655

<220> ycjd b1290
<222> 51-150
<223> ycjd b1290 U00096 complement(1349431..1349784)
<313> 6571: 101-120
<400> 6571
TTTGCAGGAA CGAAAGCTCT GGCGATATCT TCGCAGCCGA CGTTTTAGTG  50
ATTTCAAATT TCGCCGTCAA CATCCAGTGG GGAGCTACAT TCTCGATTTT  100
<210> 6572
<211> 100
<212> DNA
<213> Escherichia coli K-12 MG1655
<220> atpe b3738
<222> 141-240
<223> atpe b3738 U00096 complement(3918578..3918817)
<313> 6572: 191-210
<400> 6572
TCTGCTGCGT ACTCAGTTCT TTATCGTTAT GGGTCTGGTG GATGCTATCC  50
CGATGATCGC TGTAGGTCTG GGTCTGTACG TGATGTTCGC TGTCGCGTAG  100
<210> 6573
<211> 100
<212> DNA
<213> Escherichia coli K-12 MG1655
<220> atpe b3738
<222> 99-198
<223> atpe b3738 U00096 complement(3918578..3918817)
<313> 6573: 126-145
<400> 6573
TAAATTCCTG GAAGGCGCAG CGCGTCAACC TGATCTGATT CCTCTGCTGC  50
GTACTCAGTT CTTTATCGTT ATGGGTCTGG TGGATGCTAT CCCGATGATC  100
<210> 6574
<211> 100
<212> DNA
<213> Escherichia coli K-12 MG1655
<220> atpe b3738
<222> 1-100
<223> atpe b3738 U00096 complement(3918578..3918817)
<313> 6574: 26-45
<400> 6574
ATGGAAAACC TGAATATGGA TCTGCTGTAC ATGGCTGCCG CTGTGATGAT  50
GGGTCTGGCG GCAATCGGTG CTGCGATCGG TATCGGCATC CTCGGGGGTA  100
<210> 6575
<211> 100
<212> DNA
<213> Escherichia coli K-12 MG1655
<220> atpf b3737
<222> 372-471
<223> atpf b3737 U00096 complement(3918046..3918516)
<313> 6575: 434-453
<400> 6575
TGCTATCCTG GCTGTTGCTG GCGCCGAGAA GATCATCGAA CGTTCCGTGG  50
ATGAAGCTGC TAACAGCGAC ATCGTGGATA AACTTGTCGC TGAACTGTAA  100
<210> 6576
<211> 100
<212> DNA
<213> Escherichia coli K-12 MG1655
<220> atpf b3737
<222> 307-406
<223> atpf b3737 U00096 complement(3918046..3918516)
<313> 6576: 372-391
<400> 6576
GCCCAGGCGC AGGCGGAAAT TGAAGCCGAG CGTAAACGTG CCCGTGAAGA  50
GCTGCGTAAG CAAGTTGCTA TCCTGGCTGT TGCTGGCGCC GAGAAGATCA  100
<210> 6577
<211> 100
<212> DNA
<213> Escherichia coli K-12 MG1655
<220> atpf b3737
<222> 292-391
<223> atpf b3737 U00096 complement(3918046..3918516)
<313> 6577: 323-342
<400> 6577

CGTACTAAAA TCGTGGCCCA GGCGCAGGCG GAAATTGAAG CCGAGCGTAA 50
ACGTGCCCGT GAAGAGCTGC GTAAGCAAGT TGCTATCCTG GCTGTTGCTG 100
<210> 6578
<211> 100
<212> DNA
<213> Escherichia coli K-12 MG1655
<220> ycjf b1323
<222> 963-1062
<223> ycjf b1323 U00096 1383535..1384596
<313> 6578: 1041-1060
<400> 6578
GATTGACGAT GACAAACCTC GCCTCGGGGA TTTCCGTCGT CAGCTTATCG 50
GTCAGGTGAA AGAAACGCTG CAAAAAGGCA AAACGCCCAG CGAAAAATAA 100
<210> 6579
<211> 100
<212> DNA
<213> Escherichia coli K-12 MG1655
<220> ycjf b1323
<222> 939-1038
<223> ycjf b1323 U00096 1383535..1384596
<313> 6579: 960-979
<400> 6579
GGAGCTTTGC CGCCCGCTGC CGTGGATTGA CGATGACAAA CCTCGCCTCG 50
GGGATTTCCG TCGTCAGCTT ATCGGTCAGG TGAAAGAAAC GCTGCAAAAA 100
<210> 6580
<211> 100
<212> DNA
<213> Escherichia coli K-12 MG1655
<220> ycjf b1323
<222> 859-958
<223> ycjf b1323 U00096 1383535..1384596
<313> 6580: 913-932
<400> 6580
CTCGCTGCTC GTTTGTCTAC CCGCGCAGCT CAGGGGATTG GTGCAGGACT 50
TCTGACGGCA CGACTCGGGA TTAAAGCTAT GGAGCTTTGC CGCCCGCTGC 100
<210> 6581
<211> 100
<212> DNA
<213> Escherichia coli K-12 MG1655
<220> atpg b3734
<222> 765-864
<223> atpg b3734 U00096 complement(3915030..3915893)
<313> 6581: 812-831
<400> 6581
CAGCCTGATT AAAGAGCTGC AGTTGGTATA CAACAAAGCT CGTCAGGCCA 50
GCATTACTCA GGAACTCACC GAGATCGTCT CGGGGGCCGC CGCGGTTTAA 100
<210> 6582
<211> 100
<212> DNA
<213> Escherichia coli K-12 MG1655
<220> atpg b3734
<222> 637-736
<223> atpg b3734 U00096 complement(3915030..3915893)
<313> 6582: 679-698
<400> 6582
AAGGCGTTGC TGGATACCCT GCTGCGTCGT TATGTCGAAT CTCAGGTTTA 50
TCAGGGCGTG GTTGAAAACC TGGCCAGCGA GCAGGCCGCC CGTATGGTGG 100
<210> 6583
<211> 100
<212> DNA
<213> Escherichia coli K-12 MG1655
<220> atpg b3734
<222> 608-707
<223> atpg b3734 U00096 complement(3915030..3915893)
<313> 6583: 634-653
<400> 6583
CCTGGGATTA CCTGTACGAA CCCGATCCGA AGGCGTTGCT GGATACCCTG 50
CTGCGTCGTT ATGTCGAATC TCAGGTTTAT CAGGGCGTGG TTGAAAACCT 100
<210> 6584
<211> 100
<212> DNA

<213> Escherichia coli K-12 MG1655
<220> ycjg b1326
<222> 909-1008
<223> ycjg b1326 U00096 1386912..1387919
<313> 6584: 979-998
<400> 6584
GGTGCCGCAG GTCAGTTTCG CCGATCTTGA CGGACCGACC TGGCTGGCGG 50
TAGATGTGGA ACCGGCGCTT CAGTTCACGA CGGGCGAATT GCATCTTTAG 100
<210> 6585
<211> 100
<212> DNA
<213> Escherichia coli K-12 MG1655
<220> ycjg b1326
<222> 837-936
<223> ycjg b1326 U00096 1386912..1387919
<313> 6585: 881-900
<400> 6585
TGCACAAGGT TTCAGTCTGA TGCTGGGCTG CATGTTGTGT ACCTCTCGTG 50
CCATTAGCGC CGCTTTACCG CTGGTGCCGC AGGTCAGTTT CGCCGATCTT 100
<210> 6586
<211> 100
<212> DNA
<213> Escherichia coli K-12 MG1655
<220> ycjg b1326
<222> 721-820
<223> ycjg b1326 U00096 1386912..1387919
<313> 6586: 754-773
<400> 6586
AGTTGTCATA CTCGTAGCAA TTTGAAGGCG CTGAAAGGGC GCTATGAGAT 50
GGTTAATATT AAGCTCGATA AAACCGGGGG TCTGACGGAA GCGCTGGCGC 100
<210> 6587
<211> 100
<212> DNA
<213> Escherichia coli K-12 MG1655
<220> atph b3736
<222> 386-485
<223> atph b3736 U00096 complement(3917498..3918031)
<313> 6587: 437-456
<400> 6587
TGGAAAAACG TCTGTCACGC AAAGTTAAGC TGAATTGCAA AATCGATAAG 50
TCTGTAATGG CAGGCGTTAT CATCCGAGCG GGTGATATGG TCATTGATGG 100
<210> 6588
<211> 100
<212> DNA
<213> Escherichia coli K-12 MG1655
<220> atph b3736
<222> 336-435
<223> atph b3736 U00096 complement(3917498..3918031)
<313> 6588: 383-402
<400> 6588
TTCCGCTGCC GCACTGAGTG AACAACAGCT CGCGAAAATT TCTGCTGCGA 50
TGGAAAAACG TCTGTCACGC AAAGTTAAGC TGAATTGCAA AATCGATAAG 100
<210> 6589
<211> 100
<212> DNA
<213> Escherichia coli K-12 MG1655
<220> atph b3736
<222> 263-362
<223> atph b3736 U00096 complement(3917498..3918031)
<313> 6589: 302-321
<400> 6589
CGCTCCCGGA TGTTCTGGAG CAGTTTATTC ACCTGCGTGC CGTGAGTGAG 50
GCTACCGCTG AGGTAGACGT CATTTCCGCT GCCGCACTGA GTGAACAACA 100
<210> 6590
<211> 100
<212> DNA
<213> Escherichia coli K-12 MG1655
<220> atpi b3740
<222> 294-393
<223> atpi b3740 U00096 complement(3919688..3920080)
<313> 6590: 369-388

<400> 6590
GGCGGTTTTA AAGGCGGTAT TCTTGCCGCT GATCGTTACG TGGGTTTTGG  50
TGCTGGTGGT TCAGATACTG GCACCGGCTG TAATTAACAA CAAAGGGTAA  100
<210> 6591
<211> 100
<212> DNA
<213> Escherichia coli K-12 MG1655
<220> atpi b3740
<222> 122-221
<223> atpi b3740 U00096 complement(3919688..3920080)
<313> 6591: 142-161
<400> 6591
GCGTCTCTGC AATAAGCGGG GGCCTGGCAG TCTTTCTGCC TAACGTTTTG  50
TTTATGATAT TTGCCTGGCG TCACCAGGCG CATACACCAG CGAAAGGCCG  100
<210> 6592
<211> 100
<212> DNA
<213> Escherichia coli K-12 MG1655
<220> atpi b3740
<222> 64-163
<223> atpi b3740 U00096 complement(3919688..3920080)
<313> 6592: 81-100
<400> 6592
CAGTTACTGG TGGTGATAGC AAGTGGATTG CTGTTCAGCC TCAAAGACCC  50
CTTCTGGGGC GTCTCTGCAA TAAGCGGGGG CCTGGCAGTC TTTCTGCCTA  100
<210> 6593
<211> 100
<212> DNA
<213> Escherichia coli K-12 MG1655
<220> ycji ycjI
<222> 690-789
<223> ycji ycjI U00096 complement(1387894..1388682)
<313> 6593: 764-783
<400> 6593
GTTTCCGCCC ATCTCCTCCG ACGAAGCCAG CGAAAAATAT CTCTTTGCGA  50
TGGCAAACTT GCTGCGCTGG CATCCTAAAG ATGCAATTCG CCCGTCGTGA  100
<210> 6594
<211> 100
<212> DNA
<213> Escherichia coli K-12 MG1655
<220> ycji ycjI
<222> 689-788
<223> ycji ycjI U00096 complement(1387894..1388682)
<313> 6594: 719-738
<400> 6594
AGTTTCCGCC CATCTCCTCC GACGAAGCCA GCGAAAAATA TCTCTTTGCG  50
ATGGCAAACT TGCTGCGCTG GCATCCTAAA GATGCAATTC GCCCGTCGTG  100
<210> 6595
<211> 100
<212> DNA
<213> Escherichia coli K-12 MG1655
<220> ycji ycjI
<222> 639-738
<223> ycji ycjI U00096 complement(1387894..1388682)
<313> 6595: 676-695
<400> 6595
CTCTTTCGGC AGTTGGTGTG CAGATCTGAA CTTGCATTGC ATCACCGCAG  50
AGTTTCCGCC CATCTCCTCC GACGAAGCCA GCGAAAAATA TCTCTTTGCG  100
<210> 6596
<211> 100
<212> DNA
<213> Escherichia coli K-12 MG1655
<220> ycjj ycjJ
<222> 1325-1424
<223> ycjj ycjJ U00096 complement(1355826..1357265)
<313> 6596: 1379-1398
<400> 6596
TGACACTCGG TCTGGTATGG GCTTCGTTAG GCGGCGCATA TTTATGGTAT  50
CTGATCCGCC GCTATCGCAA AGTGCCGTTG TATGATGGTG ACAGAACGCC  100
<210> 6597
<211> 100

<212> DNA
<213> Escherichia coli K-12 MG1655
<220> ycjj ycjJ
<222> 1311-1410
<223> ycjj ycjJ U00096 complement(1355826..1357265)
<313> 6597: 1345-1364
<400> 6597
CGAGTCAACG TCACTGACAC TCGGTCTGGT ATGGGCTTCG TTAGGCGGCG 50
CATATTTATG GTATCTGATC CGCCGCTATC GCAAAGTGCC GTTGTATGAT 100
<210> 6598
<211> 100
<212> DNA
<213> Escherichia coli K-12 MG1655
<220> ycjj ycjJ
<222> 1176-1275
<223> ycjj ycjJ U00096 complement(1355826..1357265)
<313> 6598: 1217-1236
<400> 6598
TACCTTCGTG AATCTGTCGG TGTTTAATCA TTTCTGGCGG CGTAAAGGAA 50
TGAATAAAAG CTGGAAGGAT CACTTCCACT ATTTGCTGAT GCCGCTGGTT 100
<210> 6599
<211> 100
<212> DNA
<213> Escherichia coli K-12 MG1655
<220> b1680 b1681
<222> 1122-1221
<223> b1680 b1681 U00096 complement(1757327..1758547)
<313> 6599: 1187-1206
<400> 6599
CCCTGCGATG TGTCGGGCGT CGCTGGCCAT GTATAACACC CATGAAGAAG 50
TGGATCGTCT GGTGACCGGC CTGCAACGTA TTCACCGTTT GCTGGGATAA 100
<210> 6600
<211> 100
<212> DNA
<213> Escherichia coli K-12 MG1655
<220> b1680 b1681
<222> 997-1096
<223> b1680 b1681 U00096 complement(1757327..1758547)
<313> 6600: 1038-1057
<400> 6600
GGCGTTATTG CTTTTAATCT CGGTAAACAC CACGCCTATG ATGTTGGCAG 50
TTTTCTCGAT AATTACGGCA TTGCTGTGCG TACCGGACAT CACTGCGCAA 100
<210> 6601
<211> 100
<212> DNA
<213> Escherichia coli K-12 MG1655
<220> b1680 b1681
<222> 906-1005
<223> b1680 b1681 U00096 complement(1757327..1758547)
<313> 6601: 967-986
<400> 6601
AGCCGAGTAT GAACAGAATC TGATGCATTA TGCGCTATCA CAGCTGGAAT 50
CTGTACCGGA TCTCACTCTC TATGGCCCAC AAAACAGGCT TGGCGTTATT 100
<210> 6602
<211> 100
<212> DNA
<213> Escherichia coli K-12 MG1655
<220> ycjl b1299
<222> 678-777
<223> ycjl b1299 U00096 1359132..1359908
<313> 6602: 735-754
<400> 6602
CCCGGAATGG AACAGTAGCG AGTACGCGCT TTCGCGTATA TTGTTCGAGG 50
GCTTTATCAC CGCTTGTCAG CACCATATCG CTGAAAAACA GCGACTCTGA 100
<210> 6603
<211> 100
<212> DNA
<213> Escherichia coli K-12 MG1655
<220> ycjl b1299
<222> 652-751
<223> ycjl b1299 U00096 1359132..1359908

<313> 6603: 690-709
<400> 6603
CCTTTTGCGC TGGGCGTACA GTGGCACCCG GAATGGAACA GTAGCGAGTA 50
CGCGCTTTCG CGTATATTGT TCGAGGGCTT TATCACCGCT TGTCAGCACC 100
<210> 6604
<211> 100
<212> DNA
<213> Escherichia coli K-12 MG1655
<220> ycjl b1299
<222> 603-702
<223> ycjl b1299 U00096 1359132..1359908
<313> 6604: 639-658
<400> 6604
AGCTCGCTCG CCGGATGGCT TAGTTGAGGC GGTTAGCGTC ATCAATCATC 50
CTTTTGCGCT GGGCGTACAG TGGCACCCGG AATGGAACAG TAGCGAGTAC 100
<210> 6605
<211> 100
<212> DNA
<213> Escherichia coli K-12 MG1655
<220> ycjm ycjM
<222> 1608-1707
<223> ycjm ycjM U00096 1368213..1369919
<313> 6605: 1673-1692
<400> 6605
AAATATTACT AATCTGCATG GTCGGGATCT GATTAGTGAA GTTGATATAT 50
TGGGTAATGA AATAACGCTG CGCCCCTGGC AGGTTATGTG GATTAAATAA 100
<210> 6606
<211> 100
<212> DNA
<213> Escherichia coli K-12 MG1655
<220> ycjm ycjM
<222> 1431-1530
<223> ycjm ycjM U00096 1368213..1369919
<313> 6606: 1453-1472
<400> 6606
GGTATATCAT GAGTTGTCGC GTTTAATTAC ACTTCGTCGC AGCCATAACG 50
AGTTTCATCC GGATAATAAT TTTACCATTG ATACGATTAA TTCATCCGTA 100
<210> 6607
<211> 100
<212> DNA
<213> Escherichia coli K-12 MG1655
<220> ycjm ycjM
<222> 1318-1417
<223> ycjm ycjM U00096 1368213..1369919
<313> 6607: 1339-1358
<400> 6607
AATGATTACG CAGGTGTCGA AAAACTCGGA TATAACCGTG CGATTAACCG 50
TAAAAAATAT CACAGTAAAG AGATAACCCG AGAACTGAAC GATGAAGCTA 100
<210> 6608
<211> 100
<212> DNA
<213> Escherichia coli K-12 MG1655
<220> ycjo b1312
<222> 783-882
<223> ycjo b1312 U00096 1371246..1372127
<313> 6608: 853-872
<400> 6608
TTTAGGAAAA GCGGCGGCGA TCTCGGTGGT GCTCTTCATC ATTATTTTCG 50
CTGTCATTCT GCTGACCAGG AAACGGGTGA ACCTCAATGG CAACAAATAA 100
<210> 6609
<211> 100
<212> DNA
<213> Escherichia coli K-12 MG1655
<220> ycjo b1312
<222> 783-882
<223> ycjo b1312 U00096 1371246..1372127
<313> 6609: 817-836
<400> 6609
TTTAGGAAAA GCGGCGGCGA TCTCGGTGGT GCTCTTCATC ATTATTTTCG 50
CTGTCATTCT GCTGACCAGG AAACGGGTGA ACCTCAATGG CAACAAATAA 100
<210> 6610

<211> 100
<212> DNA
<213> Escherichia coli K-12 MG1655
<220> ycjo b1312
<222> 667-766
<223> ycjo b1312 U00096 1371246..1372127
<313> 6610: 716-735
<400> 6610
GTGGTGACAC TGCGCACCAT CTGGATGTTC TACATGTTCG CGGATGTTTA 50
TTTGCTGACG ACCAAGGTCG ATATTCTCGG TGTATATCTC TACAAAACCG 100
<210> 6611
<211> 100
<212> DNA
<213> Escherichia coli K-12 MG1655
<220> b0955 b0956
<222> 1657-1756
<223> b0955 b0956 U00096 complement(1015762..1017522)
<313> 6611: 1689-1708
<400> 6611
CTGATGCAAA CCATCCAGGA ACGTATCGCG CAAGCATCGC AACAGGAAGG 50
ACGTCACCGT TTTCCATGGC CATTACGTTG GCTGAACTGG TTTATTCCGA 100
<210> 6612
<211> 100
<212> DNA
<213> Escherichia coli K-12 MG1655
<220> b0955 b0956
<222> 1602-1701
<223> b0955 b0956 U00096 complement(1015762..1017522)
<313> 6612: 1642-1661
<400> 6612
TGACGCACTG CCGTTATTAT TAAATCTGGT GTGGGATGGC GAAGGCCAAA 50
CGACGCTGAT GCAAACCATC CAGGAACGTA TCGCGCAAGC ATCGCAACAG 100
<210> 6613
<211> 100
<212> DNA
<213> Escherichia coli K-12 MG1655
<220> b0955 b0956
<222> 1555-1654
<223> b0955 b0956 U00096 complement(1015762..1017522)
<313> 6613: 1598-1617
<400> 6613
GCGGTAGAAG AAGGCAAATT CACCATCTGG GCAGTAGACG ATGTGACTGA 50
CGCACTGCCG TTATTATTAA ATCTGGTGTG GGATGGCGAA GGCCAAACGA 100
<210> 6614
<211> 100
<212> DNA
<213> Escherichia coli K-12 MG1655
<220> yi22_1 yi22_1
<222> 186-285
<223> yi22_1 yi22_1 U00096 380898..381803
<313> 6614: 223-242
<400> 6614
GCTGCCAACG TATGGTTATC GTCGGGTATG GGCGCTGCTT CGCAGACAGG 50
CAGAACTTGA TGGTATGCCT GCGATCAATG CCAAACGTGT TTACCGGATC 100
<210> 6615
<211> 100
<212> DNA
<213> Escherichia coli K-12 MG1655
<220> yi22_1 yi22_1
<222> 155-254
<223> yi22_1 yi22_1 U00096 380898..381803
<313> 6615: 177-196
<400> 6615
TGCTTCTCCG TATACACCAT GTTATCGGAG AGCTGCCAAC GTATGGTTAT 50
CGTCGGGTAT GGGCGCTGCT TCGCAGACAG GCAGAACTTG ATGGTATGCC 100
<210> 6616
<211> 100
<212> DNA
<213> Escherichia coli K-12 MG1655
<220> yi22_1 yi22_1
<222> 112-211

<223> yi22_1 yi22_1 U00096 380898..381803
<313> 6616: 132-151
<400> 6616
TGGATGGATG GCCGCCGCAG TCGTCACACT GATGATACGG ATGTGCTTCT 50
CCGTATACAC CATGTTATCG GAGAGCTGCC AACGTATGGT TATCGTCGGG 100
<210> 6617
<211> 100
<212> DNA
<213> Escherichia coli K-12 MG1655
<220> ycjp b1313
<222> 636-735
<223> ycjp b1313 U00096 1372114..1372956
<313> 6617: 670-689
<400> 6617
CGACTATCTG TTTGCGTCGA TTTTCCTCTC CAGCGCCAGC AATTTCACCT 50
TACCGGTGGG CCTGAACGCG CTGTTCAGTA CGCCAGATTA CATCTGGGGA 100
<210> 6618
<211> 100
<212> DNA
<213> Escherichia coli K-12 MG1655
<220> ycjp b1313
<222> 582-681
<223> ycjp b1313 U00096 1372114..1372956
<313> 6618: 621-640
<400> 6618
GGCGATGTCC GGGCTGATAT CCGTGTTCGT CTACTGCTTT ATGGTGGCGT 50
GGAACGACTA TCTGTTTGCG TCGATTTTCC TCTCCAGCGC CAGCAATTTC 100
<210> 6619
<211> 100
<212> DNA
<213> Escherichia coli K-12 MG1655
<220> ycjp b1313
<222> 506-605
<223> ycjp b1313 U00096 1372114..1372956
<313> 6619: 555-574
<400> 6619
CCGATGAGAT CGAAGAAGCG GCGATGATGG ATGGCCTCAA CCGTCTGCAA 50
ATTATCTTCC GCATCACCGT GCCGTTGGCG ATGTCCGGGC TGATATCCGT 100
<210> 6620
<211> 100
<212> DNA
<213> Escherichia coli K-12 MG1655
<220> b1685 b1685
<222> 171-270
<223> b1685 b1685 U00096 complement(1762958..1763227)
<313> 6620: 230-249
<400> 6620
TCTGAAACAA CTAGAACTTG AATTTGCTGA CCTGCTTACT CTCTCTTCTG 50
CTGAATTAAA AGAAGAAATC TACTTTGCCT GGCGTCTGGG CGTGCATTGA 100
<210> 6621
<211> 100
<212> DNA
<213> Escherichia coli K-12 MG1655
<220> b1685 b1685
<222> 171-270
<223> b1685 b1685 U00096 complement(1762958..1763227)
<313> 6621: 212-231
<400> 6621
TCTGAAACAA CTAGAACTTG AATTTGCTGA CCTGCTTACT CTCTCTTCTG 50
CTGAATTAAA AGAAGAAATC TACTTTGCCT GGCGTCTGGG CGTGCATTGA 100
<210> 6622
<211> 100
<212> DNA
<213> Escherichia coli K-12 MG1655
<220> b1685 b1685
<222> 168-267
<223> b1685 b1685 U00096 complement(1762958..1763227)
<313> 6622: 209-228
<400> 6622
ACGTCTGAAA CAACTAGAAC TTGAATTTGC TGACCTGCTT ACTCTCTCTT 50
CTGCTGAATT AAAAGAAGAA ATCTACTTTG CCTGGCGTCT GGGCGTGCAT 100

<210> 6623
<211> 100
<212> DNA
<213> Escherichia coli K-12 MG1655
<220> yi22_2 b1403
<222> 196-295
<223> yi22_2 b1403 U00096 complement(1465945..1466850)
<313> 6623: 220-239
<400> 6623
TATGGTTATC GTCGGGTATG GGCGCTGCTT CGCAGACAGG CAGAACTTGA 50
TGGTATGCCT GCGATCAATG CCAAACGTGT TTACCGGATC ATGCGCCAGA 100
<210> 6624
<211> 100
<212> DNA
<213> Escherichia coli K-12 MG1655
<220> yi22_2 b1403
<222> 133-232
<223> yi22_2 b1403 U00096 complement(1465945..1466850)
<313> 6624: 175-194
<400> 6624
CGTCACACTG ATGATACGGA TGTGCTTCTC CGTATACACC ATGTTATCGG 50
AGAGCTGCCA ACGTATGGTT ATCGTCGGGT ATGGGCGCTG CTTCGCAGAC 100
<210> 6625
<211> 100
<212> DNA
<213> Escherichia coli K-12 MG1655
<220> yi22_2 b1403
<222> 112-211
<223> yi22_2 b1403 U00096 complement(1465945..1466850)
<313> 6625: 139-158
<400> 6625
TGGATGGATG GCCGCCGCAG TCGTCACACT GATGATACGG ATGTGCTTCT 50
CCGTATACAC CATGTTATCG GAGAGCTGCC AACGTATGGT TATCGTCGGG 100
<210> 6626
<211> 100
<212> DNA
<213> Escherichia coli K-12 MG1655
<220> ycjq ycjQ
<222> 954-1053
<223> ycjq ycjQ U00096 1372987..1374039
<313> 6626: 972-991
<400> 6626
AATCGACCCG GTGGTGACCT TTGCCAATAG CCCGGAAAGC TATATGCAGT 50
ATGTCGACCA GCATCCGGAA CAGAGCATCA AAATGGGCGT TACGTTTTAA 100
<210> 6627
<211> 100
<212> DNA
<213> Escherichia coli K-12 MG1655
<220> ycjq ycjQ
<222> 909-1008
<223> ycjq ycjQ U00096 1372987..1374039
<313> 6627: 931-950
<400> 6627
CTGTTGGGAA CTGCTGATGA ACGGTTATCT CAATTGCGAA GATTTAATCG 50
ACCCGGTGGT GACCTTTGCC AATAGCCCGG AAAGCTATAT GCAGTATGTC 100
<210> 6628
<211> 100
<212> DNA
<213> Escherichia coli K-12 MG1655
<220> ycjq ycjQ
<222> 868-967
<223> ycjq ycjQ U00096 1372987..1374039
<313> 6628: 900-919
<400> 6628
CCGGATTATC CGCGCTGGAG CCGCAAGCGT ATTGAAGAAA CCTGTTGGGA 50
ACTGCTGATG AACGGTTATC TCAATTGCGA AGATTTAATC GACCCGGTGG 100
<210> 6629
<211> 100
<212> DNA
<213> Escherichia coli K-12 MG1655
<220> b1686 b1687

<222> 312-411
<223> b1686 b1687 U00096 complement(1763246..1763656)
<313> 6629: 360-379
<400> 6629
GTTGCATCTC GGTTCGCGTC ACCAGGTCTG GCAGATTGAA ATCTTCGATG  50
AGAAAGGGCG TTTGTGCTGT TCGTCACGAT TGACGACCGC CATTTTGTGA  100
<210> 6630
<211> 100
<212> DNA
<213> Escherichia coli K-12 MG1655
<220> b1686 b1687
<222> 286-385
<223> b1686 b1687 U00096 complement(1763246..1763656)
<313> 6630: 306-325
<400> 6630
GGGCGGGTGC GCGGCGTATG CAAACCGTTG CATCTCGGTT CGCGTCACCA  50
GGTCTGGCAG ATTGAAATCT TCGATGAGAA AGGGCGTTTG TGCTGTTCGT  100
<210> 6631
<211> 100
<212> DNA
<213> Escherichia coli K-12 MG1655
<220> b1686 b1687
<222> 201-300
<223> b1686 b1687 U00096 complement(1763246..1763656)
<313> 6631: 259-278
<400> 6631
CGTTGCCGGT TATTTATGTA CCGAAGGTGA GCAAAAAGTG GTTGGTCTGG  50
AAATCAATGC TAACCACGTC CGCTCGGCAC GAGAAGGGCG GGTGCGCGGC  100
<210> 6632
<211> 100
<212> DNA
<213> Escherichia coli K-12 MG1655
<220> yi22_3 b1997
<222> 184-283
<223> yi22_3 b1997 U00096 complement(2066974..2067879)
<313> 6632: 213-232
<400> 6632
GAGCTGCCAA CGTATGGTTA TCGTCGGGTA TGGGCGCTGC TTCGCAGACA  50
GGCAGAACTT GATGGTATGC CTGCGATCAA TGCCAAACGT GTTTACCGGA  100
<210> 6633
<211> 100
<212> DNA
<213> Escherichia coli K-12 MG1655
<220> yi22_3 b1997
<222> 160-259
<223> yi22_3 b1997 U00096 complement(2066974..2067879)
<313> 6633: 178-197
<400> 6633
CTCCGTATAC ACCATGTTAT CGGAGAGCTG CCAACGTATG GTTATCGTCG  50
GGTATGGGCG CTGCTTCGCA GACAGGCAGA ACTTGATGGT ATGCCTGCGA  100
<210> 6634
<211> 100
<212> DNA
<213> Escherichia coli K-12 MG1655
<220> yi22_3 b1997
<222> 75-174
<223> yi22_3 b1997 U00096 complement(2066974..2067879)
<313> 6634: 138-157
<400> 6634
TGCGCAGTTG CACGTCATTC TCAGACGAAC CGATGACTGG ATGGATGGCC  50
GCCGCAGTCG TCACACTGAT GATACGGATG TGCTTCTCCG TATACACCAT  100
<210> 6635
<211> 100
<212> DNA
<213> Escherichia coli K-12 MG1655
<220> yi22_4 b2861
<222> 175-274
<223> yi22_4 b2861 U00096 complement(2994394..2995299)
<313> 6635: 215-234
<400> 6635
GTTATCGGAG AGCTGCCAAC GTATGGTTAT CGTCGGGTAT GGGCGCTGCT  50

TCGCAGACAG GCAGAACTTG ATGGTATGCC TGCGATCAAT GCCAAACGTG 100
<210> 6636
<211> 100
<212> DNA
<213> Escherichia coli K-12 MG1655
<220> yi22_4 b2861
<222> 156-255
<223> yi22_4 b2861 U00096 complement(2994394..2995299)
<313> 6636: 178-197
<400> 6636
GCTTCTCCGT ATACACCATG TTATCGGAGA GCTGCCAACG TATGGTTATC 50
GTCGGGTATG GGCGCTGCTT CGCAGACAGG CAGAACTTGA TGGTATGCCT 100
<210> 6637
<211> 100
<212> DNA
<213> Escherichia coli K-12 MG1655
<220> yi22_4 b2861
<222> 100-199
<223> yi22_4 b2861 U00096 complement(2994394..2995299)
<313> 6637: 129-148
<400> 6637
CGAACCGATG ACTGGATGGA TGGCCGCCGC AGTCGTCACA CTGATGATAC 50
GGATGTGCTT CTCCGTATAC ACCATGTTAT CGGAGAGCTG CCAACGTATG 100
<210> 6638
<211> 100
<212> DNA
<213> Escherichia coli K-12 MG1655
<220> ycjs b1316
<222> 936-1035
<223> ycjs b1316 U00096 1374856..1375911
<313> 6638: 988-1007
<400> 6638
TATCAATCAC GTACAGGGCA AGCCCGTGAT GATAGCCGAC GCCGAGCAGG 50
GGTACATCAT CCAGCAACTG GTGGCGGCGT TGTATCAATC CGCAGAAACA 100
<210> 6639
<211> 100
<212> DNA
<213> Escherichia coli K-12 MG1655
<220> ycjs b1316
<222> 898-997
<223> ycjs b1316 U00096 1374856..1375911
<313> 6639: 927-946
<400> 6639
GCAGACGACA ACCGCCATTT GCGCAGCATG GAAGCCTTTA TCAATCACGT 50
ACAGGGCAAG CCCGTGATGA TAGCCGACGC CGAGCAGGGG TACATCATCC 100
<210> 6640
<211> 100
<212> DNA
<213> Escherichia coli K-12 MG1655
<220> ycjs b1316
<222> 852-951
<223> ycjs b1316 U00096 1374856..1375911
<313> 6640: 880-899
<400> 6640
CACCGATAAC AACGGTGAAT TAATGACGCT GATGCAACGG GAAATAGCAG 50
ACGACAACCG CCATTTGCGC AGCATGGAAG CCTTTATCAA TCACGTACAG 100
<210> 6641
<211> 100
<212> DNA
<213> Escherichia coli K-12 MG1655
<220> b1688 b1688
<222> 1014-1113
<223> b1688 b1688 U00096 1767098..1768210
<313> 6641: 1069-1088
<400> 6641
GCGTCTGTTT GCCGCGTGGG TGGAAGAAGT CCCGCCGCCG ACGGACCAAC 50
CGGAAGAAAT TCTCGAAGAA CTTGGCGAAA TCGAGAAACC GAATAAGTAA 100
<210> 6642
<211> 100
<212> DNA
<213> Escherichia coli K-12 MG1655

<220> b1688 b1688
<222> 944-1043
<223> b1688 b1688 U00096 1767098..1768210
<313> 6642: 1010-1029
<400> 6642
TTATTGGTGG TTTGATTGCT TTCGGGATGA TCGGTCTGTT TATTGGTCCG 50
GTTCTGTTAG CCGTTTCCTG GCGTCTGTTT GCCGCGTGGG TGGAAGAAGT 100
<210> 6643
<211> 100
<212> DNA
<213> Escherichia coli K-12 MG1655
<220> b1688 b1688
<222> 905-1004
<223> b1688 b1688 U00096 1767098..1768210
<313> 6643: 944-963
<400> 6643
TGGGTGCCGA TTTACCGCTG ATCCTGATTC TCTCTGGCGT TATTGGTGGT 50
TTGATTGCTT TCGGGATGAT CGGTCTGTTT ATTGGTCCGG TTCTGTTAGC 100
<210> 6644
<211> 100
<212> DNA
<213> Escherichia coli K-12 MG1655
<220> b0959 b0959
<222> 531-630
<223> b0959 b0959 U00096 1020361..1020990
<313> 6644: 604-623
<400> 6644
CATTCATGAA GCTGCGCTCC CGGTGGCACG CCGCCAGGAG CTTGCAGAAT 50
GGGCTGACTC TCTTACGCCG AAACAGGAGT TTCCTGCGGA ACTTGAGTAA 100
<210> 6645
<211> 100
<212> DNA
<213> Escherichia coli K-12 MG1655
<220> b0959 b0959
<222> 414-513
<223> b0959 b0959 U00096 1020361..1020990
<313> 6645: 482-501
<400> 6645
TAAGGATGTA CGGGCGTTAC GTATACTTGG GGCAAAAATG TGTTGGTTGC 50
GACTGCGGCA GCAAAACAGT CTGGTGACAG AAAAGATTCT GTTTATGCTT 100
<210> 6646
<211> 100
<212> DNA
<213> Escherichia coli K-12 MG1655
<220> b0959 b0959
<222> 387-486
<223> b0959 b0959 U00096 1020361..1020990
<313> 6646: 425-444
<400> 6646
GGAAGCGATT CTCGGGGAGG TGGGGATTAA GGATGTACGG GCGTTACGTA 50
TACTTGGGGC AAAAATGTGT TGGTTGCGAC TGCGGCAGCA AAACAGTCTG 100
<210> 6647
<211> 100
<212> DNA
<213> Escherichia coli K-12 MG1655
<220> yi22_5 b3046
<222> 164-263
<223> yi22_5 b3046 U00096 3184526..3185431
<313> 6647: 227-246
<400> 6647
GTATACACCA TGTTATCGGA GAGCTGCCAA CGTATGGTTA TCGTCGGGTA 50
TGGGCGCTGC TTCGCAGACA GGCAGAACTT GATGGTATGC CTGCGATCAA 100
<210> 6648
<211> 100
<212> DNA
<213> Escherichia coli K-12 MG1655
<220> yi22_5 b3046
<222> 148-247
<223> yi22_5 b3046 U00096 3184526..3185431
<313> 6648: 172-191
<400> 6648

ACGGATGTGC TTCTCCGTAT ACACCATGTT ATCGGAGAGC TGCCAACGTA 50
TGGTTATCGT CGGGTATGGG CGCTGCTTCG CAGACAGGCA GAACTTGATG 100
<210> 6649
<211> 100
<212> DNA
<213> Escherichia coli K-12 MG1655
<220> yi22_5 b3046
<222> 80-179
<223> yi22_5 b3046 U00096 3184526..3185431
<313> 6649: 133-152
<400> 6649
AGTTGCACGT CATTCTCAGA CGAACCGATG ACTGGATGGA TGGCCGCCGC 50
AGTCGTCACA CTGATGATAC GGATGTGCTT CTCCGTATAC ACCATGTTAT 100
<210> 6650
<211> 100
<212> DNA
<213> Escherichia coli K-12 MG1655
<220> ycjt b1317
<222> 2169-2268
<223> ycjt b1317 U00096 1375908..1378175
<313> 6650: 2245-2264
<400> 6650
CGGTCAGCTT ATAACCGTGG CTGAAGAATC TGTTTTCTGT TTGGGTGATT 50
TTATTTTGCC CTTCAATGGG ACCGCTACCA AACATCAGGA GGATGAATGA 100
<210> 6651
<211> 100
<212> DNA
<213> Escherichia coli K-12 MG1655
<220> ycjt b1317
<222> 2146-2245
<223> ycjt b1317 U00096 1375908..1378175
<313> 6651: 2183-2202
<400> 6651
GCGCCCGTTT CACTGCGTTT GAACGGTCAG CTTATAACCG TGGCTGAAGA 50
ATCTGTTTTC TGTTTGGGTG ATTTTATTTT GCCCTTCAAT GGGACCGCTA 100
<210> 6652
<211> 100
<212> DNA
<213> Escherichia coli K-12 MG1655
<220> ycjt b1317
<222> 2042-2141
<223> ycjt b1317 U00096 1375908..1378175
<313> 6652: 2097-2116
<400> 6652
CGGCGTTACC TGAGCAGTGG CAACAGTTGT CTTTCCCTCT GTTCTGGCAG 50
GGCTGCGAAT TACAGGTCAC TCTTGACGCG CAGCGTATTG CGATTCGAAC 100
<210> 6653
<211> 100
<212> DNA
<213> Escherichia coli K-12 MG1655
<220> radc b3639
<222> 576-675
<223> radc b3639 U00096 complement(3809518..3810192)
<313> 6653: 646-665
<400> 6653
GATAATAAAG AGTTGTCAGT TCATGGATTT ACGCGTGCTC GACCATATCG 50
TGATTGGGCG TGGAGAGTAT GTTTCTTTTG CCGAACGCGG CTGGATTTAA 100
<210> 6654
<211> 100
<212> DNA
<213> Escherichia coli K-12 MG1655
<220> radc b3639
<222> 509-608
<223> radc b3639 U00096 complement(3809518..3810192)
<313> 6654: 541-560
<400> 6654
TCCTTGCACA TAATCACCCT TCGGGTTGTG CTGAACCCAG TAAAGCGGAT 50
AAACTCATTA CTGAACGGAT AATAAAGAGT TGTCAGTTCA TGGATTTACG 100
<210> 6655
<211> 100
<212> DNA

<213> Escherichia coli K-12 MG1655
<220> radc b3639
<222> 440-539
<223> radc b3639 U00096 complement(3809518..3810192)
<313> 6655: 469-488
<400> 6655
TAAACCATGT TGAAGTCCAT CCTCGGGAAA TTATCCGCGA AGCGATAAAA 50
ATAAACGCCT CGGCGCTGAT CCTTGCACAT AATCACCCTT CGGGTTGTGC 100
<210> 6656
<211> 100
<212> DNA
<213> Escherichia coli K-12 MG1655
<220> b1689 b1689
<222> 285-384
<223> b1689 b1689 U00096 1768612..1768995
<313> 6656: 359-378
<400> 6656
ATTTCAACGA GTCTACCCTG ATGGCAATTT TATTGACTGG AAGATCTATC 50
AATCGGTAGC TGCTGAATTA TATGCACACG ATCTGGAGCG GCTTTGTTAA 100
<210> 6657
<211> 100
<212> DNA
<213> Escherichia coli K-12 MG1655
<220> b1689 b1689
<222> 246-345
<223> b1689 b1689 U00096 1768612..1768995
<313> 6657: 281-300
<400> 6657
CGGCAACAAC ACCATGCGTT TTTTCCCCGA CTTAACCGCA TTTCAACGAG 50
TCTACCCTGA TGGCAATTTT ATTGACTGGA AGATCTATCA ATCGGTAGCT 100
<210> 6658
<211> 100
<212> DNA
<213> Escherichia coli K-12 MG1655
<220> b1689 b1689
<222> 187-286
<223> b1689 b1689 U00096 1768612..1768995
<313> 6658: 235-254
<400> 6658
ATGCGTCAGC AAAGAAAAAA ACATCTTCAT GCCATCATAG AGAAAATAAA 50
TAACCGCATC GGCAACAACA CCATGCGTTT TTTCCCCGAC TTAACCGCAT 100
<210> 6659
<211> 100
<212> DNA
<213> Escherichia coli K-12 MG1655
<220> yi22_6 b4274
<222> 168-267
<223> yi22_6 b4274 U00096 4496163..4497068
<313> 6659: 214-233
<400> 6659
ACACCATGTT ATCGGAGAGC TGCCAACGTA TGGTTATCGT CGGGTATGGG 50
CGCTGCTTCG CAGACAGGCA GAACTTGATG GTATGCCTGC GATCAATGCC 100
<210> 6660
<211> 100
<212> DNA
<213> Escherichia coli K-12 MG1655
<220> yi22_6 b4274
<222> 154-253
<223> yi22_6 b4274 U00096 4496163..4497068
<313> 6660: 174-193
<400> 6660
GTGCTTCTCC GTATACACCA TGTTATCGGA GAGCTGCCAA CGTATGGTTA 50
TCGTCGGGTA TGGGCGCTGC TTCGCAGACA GGCAGAACTT GATGGTATGC 100
<210> 6661
<211> 100
<212> DNA
<213> Escherichia coli K-12 MG1655
<220> yi22_6 b4274
<222> 76-175
<223> yi22_6 b4274 U00096 4496163..4497068
<313> 6661: 126-145

<400> 6661
GCGCAGTTGC ACGTCATTCT CAGACGAACC GATGACTGGA TGGATGGCCG 50
CCGCAGTCGT CACACTGATG ATACGGATGT GCTTCTCCGT ATACACCATG 100
<210> 6662
<211> 100
<212> DNA
<213> Escherichia coli K-12 MG1655
<220> ycju b1318
<222> 561-660
<223> ycju b1318 U00096 1378172..1378831
<313> 6662: 598-617
<400> 6662
GGTGGGGATC GGCGCGGGCT TAACCGGGGC GCAATTACTG TTGCCTTCAA 50
CGGAATCACT CACCTGGCCG CGGTTATCGG CCTTCTGGCA AAACGTATAG 100
<210> 6663
<211> 100
<212> DNA
<213> Escherichia coli K-12 MG1655
<220> ycju b1318
<222> 533-632
<223> ycju b1318 U00096 1378172..1378831
<313> 6663: 552-571
<400> 6663
ACGCCATTAA CGCCAGCGGT ATGCGCTCGG TGGGGATCGG CGCGGGCTTA 50
ACCGGGGCGC AATTACTGTT GCCTTCAACG GAATCACTCA CCTGGCCGCG 100
<210> 6664
<211> 100
<212> DNA
<213> Escherichia coli K-12 MG1655
<220> ycju b1318
<222> 401-500
<223> ycju b1318 U00096 1378172..1378831
<313> 6664: 440-459
<400> 6664
TCACCTTCTG CGCGGATGCT TCCCAACTTA AAAACTCGAA ACCGGACCCG 50
GAAATCTTTC TCGCCGCCTG TGCAGGGCTG GGCGTGCCGC CGCAGGCATG 100
<210> 6665
<211> 100
<212> DNA
<213> Escherichia coli K-12 MG1655
<220> ycjv b1319
<222> 870-969
<223> ycjv b1319 U00096 1378845..1379813
<313> 6665: 939-958
<400> 6665
GGACGCGCAA GAGGAAAATA ACATTTCCGC CAAAATTAGC GTGGCAGAAT 50
TAACCGGTGC GGAATTTATG CTCTACACCA CGGTTGGGGG CACGAGTTAG 100
<210> 6666
<211> 100
<212> DNA
<213> Escherichia coli K-12 MG1655
<220> ycjv b1319
<222> 850-949
<223> ycjv b1319 U00096 1378845..1379813
<313> 6666: 893-912
<400> 6666
CGACCGGAAG ATATTCATCC GGACGCGCAA GAGGAAAATA ACATTTCCGC 50
CAAAATTAGC GTGGCAGAAT TAACCGGTGC GGAATTTATG CTCTACACCA 100
<210> 6667
<211> 100
<212> DNA
<213> Escherichia coli K-12 MG1655
<220> ycjv b1319
<222> 759-858
<223> ycjv b1319 U00096 1378845..1379813
<313> 6667: 802-821
<400> 6667
TACGGAAACG CTTAAATTAA CCATTCCCGA AGAGAAATTA GCGGTTCTGA 50
AAACACAGGA AAGTTTGCAT AAGCCCATCG TGATGGGAAT ACGACCGGAA 100
<210> 6668
<211> 100

<212> DNA
<213> Escherichia coli K-12 MG1655
<220> ycjw b1321
<222> 900-999
<223> ycjw b1321 U00096 complement(1380987..1381985)
<313> 6668: 966-985
<400> 6668
GGAGTTAGGT CAACAAAGCT GCGAGTTATT ATTCCGCTTA ATTTCTGGAA 50
AACCGTCACC ACAGAATATT ACCGTTGCCA CGCATATGAC GCTGAAATAA 100
<210> 6669
<211> 100
<212> DNA
<213> Escherichia coli K-12 MG1655
<220> ycjw b1321
<222> 861-960
<223> ycjw b1321 U00096 complement(1380987..1381985)
<313> 6669: 905-924
<400> 6669
TTTAACGCCT GCACCGGGCG GCATCGATGT TCCGACGCAG GAGTTAGGTC 50
AACAAAGCTG CGAGTTATTA TTCCGCTTAA TTTCTGGAAA ACCGTCACCA 100
<210> 6670
<211> 100
<212> DNA
<213> Escherichia coli K-12 MG1655
<220> ycjw b1321
<222> 793-892
<223> ycjw b1321 U00096 complement(1380987..1381985)
<313> 6670: 841-860
<400> 6670
GAGAAAAATA TTGCTATTCC GCAGCAGTTA GCGGTGGTGG GTTATAGCAA 50
TGAAACGCTG TCATTTATTT TAACGCCTGC ACCGGGCGGC ATCGATGTTC 100
<210> 6671
<211> 100
<212> DNA
<213> Escherichia coli K-12 MG1655
<220> ycjx b1322
<222> 1299-1398
<223> ycjx b1322 U00096 1382141..1383538
<313> 6671: 1348-1367
<400> 6671
TGAGGCATTT CGTCCGCAGG TGATGGATGT CGACAAACCA CTACCGCATA 50
TTCGTCTTGA TGCTGCGCTG GAATTTTTAA TAGGAGATAA ATTGCGATGA 100
<210> 6672
<211> 100
<212> DNA
<213> Escherichia coli K-12 MG1655
<220> ycjx b1322
<222> 1261-1360
<223> ycjx b1322 U00096 1382141..1383538
<313> 6672: 1303-1322
<400> 6672
CCTGGTCAGG CGTTCTGGGA TAAGCAAGGC TTCCAGTTTG AGGCATTTCG 50
TCCGCAGGTG ATGGATGTCG ACAAACCACT ACCGCATATT CGTCTTGATG 100
<210> 6673
<211> 100
<212> DNA
<213> Escherichia coli K-12 MG1655
<220> ycjx b1322
<222> 1180-1279
<223> ycjx b1322 U00096 1382141..1383538
<313> 6673: 1234-1253
<400> 6673
AAAATCCCGG CGCTGCGTGG TAATCGACTT AGCGATGGCG CACCGCTCAC 50
TGTTTATCCT GGCGAAGTTC CCGCACGTTT GCCTGGTCAG GCGTTCTGGG 100
<210> 6674
<211> 100
<212> DNA
<213> Escherichia coli K-12 MG1655
<220> hofb b0108
<222> 1287-1386
<223> hofb b0108 U00096 complement(115714..117099)

<313> 6674: 1362-1381
<400> 6674
TATGCGTACG CTTTTTGAAA ACGGCTGCCT GGCCGTAGAG CAAGGCTTAA 50
CCACCTTTGA AGAGTTAATC CGCGTACTGG GGATGCCGCA TGGCGAGTAA 100
<210> 6675
<211> 100
<212> DNA
<213> Escherichia coli K-12 MG1655
<220> hofb b0108
<222> 1229-1328
<223> hofb b0108 U00096 complement(115714..117099)
<313> 6675: 1252-1271
<400> 6675
AGCTTATTTC CGCTAATACC GACGTTGAAT CGCTGGAAAC GCACGCACGA 50
CAGGCGGGTA TGCGTACGCT TTTTGAAAAC GGCTGCCTGG CCGTAGAGCA 100
<210> 6676
<211> 100
<212> DNA
<213> Escherichia coli K-12 MG1655
<220> hofb b0108
<222> 1180-1279
<223> hofb b0108 U00096 complement(115714..117099)
<313> 6676: 1226-1245
<400> 6676
GGTCGTACGG CCTTATTTGA AGTTCTGCCC ATAACGCCGG TCATTCGTCA 50
GCTTATTTCC GCTAATACCG ACGTTGAATC GCTGGAAACG CACGCACGAC 100
<210> 6677
<211> 100
<212> DNA
<213> Escherichia coli K-12 MG1655
<220> ycjz ycjZ
<222> 789-888
<223> ycjz ycjZ U00096 1390015..1390914
<313> 6677: 819-838
<400> 6677
TAAATTCACA CCAGATTTAC CCGGTTATCA CCTGTACTAT CCACACCGTC 50
GACATGCTGG CTCGGCATTC TCATTATTTA TAGATAGGCT GAAGTATAAA 100
<210> 6678
<211> 100
<212> DNA
<213> Escherichia coli K-12 MG1655
<220> ycjz ycjZ
<222> 690-789
<223> ycjz ycjZ U00096 1390015..1390914
<313> 6678: 730-749
<400> 6678
GATCATTGAT GCTGCAATTG ATGGGCATGG ATTGGCGTAT CTACCTTATG 50
ATCAGGTTGA GCGGGCTATT AAAGAAAAAA AACTGATACG TGTTTTGGAT 100
<210> 6679
<211> 100
<212> DNA
<213> Escherichia coli K-12 MG1655
<220> ycjz ycjZ
<222> 622-721
<223> ycjz ycjZ U00096 1390015..1390914
<313> 6679: 639-658
<400> 6679
TTAATACGCG GTGGACGTGA AGTTCGTGTT CGCATGGAAG GTCAGCTTTT 50
ACTGAATACG ATAGACCTGA TCATTGATGC TGCAATTGAT GGGCATGGAT 100
<210> 6680
<211> 100
<212> DNA
<213> Escherichia coli K-12 MG1655
<220> hofc b0107
<222> 1103-1202
<223> hofc b0107 U00096 complement(114522..115724)
<313> 6680: 1166-1185
<400> 6680
CGGCGTTGCT GATCATAACG GGAGGAATTA TTGGTACGCT GGTGGTGGCA 50
ATGTATCTGC CAATTTTCCA TTTAGGCGAT GCGATGAGTG GGATGGGATA 100
<210> 6681

<211> 100
<212> DNA
<213> Escherichia coli K-12 MG1655
<220> hofc b0107
<222> 1082-1181
<223> hofc b0107 U00096 complement(114522..115724)
<313> 6681: 1106-1125
<400> 6681
ACCTCGCAGC CTTACTGGAA CCGGCGTTGC TGATCATAAC GGGAGGAATT 50
ATTGGTACGC TGGTGGTGGC AATGTATCTG CCAATTTTCC ATTTAGGCGA 100
<210> 6682
<211> 100
<212> DNA
<213> Escherichia coli K-12 MG1655
<220> hofc b0107
<222> 1008-1107
<223> hofc b0107 U00096 complement(114522..115724)
<313> 6682: 1037-1056
<400> 6682
ATCCGGCTCT CTGGATCTCA TGTTAGACAA CCTCGCCCAT CATCATCGGG 50
AAAACACAAT GGCGCTGGCG GATAACCTCG CAGCCTTACT GGAACCGGCG 100
<210> 6683
<211> 100
<212> DNA
<213> Escherichia coli K-12 MG1655
<220> hofd b3336
<222> 579-678
<223> hofd b3336 U00096 3463180..3463857
<313> 6683: 642-661
<400> 6683
ACTTCTGCAA CGCCGGACTA TAACCACTAT CGCATTCGGC CCGTGGCTTG 50
CGCTCGGCAG CATGATAAAC CTGGGGTATC TGGCCTGGAT CTCTTATTAA 100
<210> 6684
<211> 100
<212> DNA
<213> Escherichia coli K-12 MG1655
<220> hofd b3336
<222> 579-678
<223> hofd b3336 U00096 3463180..3463857
<313> 6684: 599-618
<400> 6684
ACTTCTGCAA CGCCGGACTA TAACCACTAT CGCATTCGGC CCGTGGCTTG 50
CGCTCGGCAG CATGATAAAC CTGGGGTATC TGGCCTGGAT CTCTTATTAA 100
<210> 6685
<211> 100
<212> DNA
<213> Escherichia coli K-12 MG1655
<220> hofd b3336
<222> 514-613
<223> hofd b3336 U00096 3463180..3463857
<313> 6685: 570-589
<400> 6685
CAAACGTTGC CAATGATACT GCTGATTGCC TCGCTGGGTG GCATTGGTTA 50
CGCCATCGTT TCACAACTTC TGCAACGCCG GACTATAACC ACTATCGCAT 100
<210> 6686
<211> 100
<212> DNA
<213> Escherichia coli K-12 MG1655
<220> hoff b3328
<222> 1068-1167
<223> hoff b3328 U00096 3457454..3458650
<313> 6686: 1118-1137
<400> 6686
ACAAAATCGG ATCGCCTTAA CGCTCTCCAT CTTCGAGCCA GCACTCATTA 50
TTACGATGGC ACTGATCGTC CTGTTTATTG TCGTGTCGGT ACTCCAACCT 100
<210> 6687
<211> 100
<212> DNA
<213> Escherichia coli K-12 MG1655
<220> hoff b3328
<222> 1033-1132

<223> hoff b3328 U00096 3457454..3458650
<313> 6687: 1067-1086
<400> 6687
ATGGTCAGAG CCGCAGATAA CCAGGAGACA CTCCAACAAA ATCGGATCGC 50
CTTAACGCTC TCCATCTTCG AGCCAGCACT CATTATTACG ATGGCACTGA 100
<210> 6688
<211> 100
<212> DNA
<213> Escherichia coli K-12 MG1655
<220> hoff b3328
<222> 963-1062
<223> hoff b3328 U00096 3457454..3458650
<313> 6688: 997-1016
<400> 6688
CGCAATTTTC CCGCCGATGA TGCTCTACAT GGTGGCCTCT GGCGAAAAAA 50
GCGGGCAGCT CGGCACATTA ATGGTCAGAG CCGCAGATAA CCAGGAGACA 100
<210> 6689
<211> 100
<212> DNA
<213> Escherichia coli K-12 MG1655
<220> hofg b3329
<222> 339-438
<223> hofg b3329 U00096 3458660..3459097
<313> 6689: 411-430
<400> 6689
TGAACATGGT GCATACGATC TGCTTTCAGC AGGGCCCGAT GGTGAAATGG 50
GAACCGAGGA CGACATCACC AACTGGGGTT TGAGCAAGAA GAAAAAGTAA 100
<210> 6690
<211> 100
<212> DNA
<213> Escherichia coli K-12 MG1655
<220> hofg b3329
<222> 297-396
<223> hofg b3329 U00096 3458660..3459097
<313> 6690: 343-362
<400> 6690
TGCCGATCCC TGGGGCAATG ATTATGTCCT CGTTAATCCT GGTGAACATG 50
GTGCATACGA TCTGCTTTCA GCAGGGCCCG ATGGTGAAAT GGGAACCGAG 100
<210> 6691
<211> 100
<212> DNA
<213> Escherichia coli K-12 MG1655
<220> hofg b3329
<222> 238-337
<223> hofg b3329 U00096 3458660..3459097
<313> 6691: 275-294
<400> 6691
CCGACACTGC CACCGCTGGC CGCAAACTAT AACAAGGAAG GTTATATCAA 50
GCGTCTGCCT GCCGATCCCT GGGGCAATGA TTATGTCCTC GTTAATCCTG 100
<210> 6692
<211> 100
<212> DNA
<213> Escherichia coli K-12 MG1655
<220> hofh b3330
<222> 411-510
<223> hofh b3330 U00096 3459105..3459614
<313> 6692: 484-503
<400> 6692
GTTGATGGCT AACGCCGGGA CCGGTGAGCC GCTCCTGACG TTAGTCTGTT 50
CAGGCTCATG GCCCCTGGAT CAAACGCTCG CCAGAGATAC CCGCCCATGA 100
<210> 6693
<211> 100
<212> DNA
<213> Escherichia coli K-12 MG1655
<220> hofh b3330
<222> 369-468
<223> hofh b3330 U00096 3459105..3459614
<313> 6693: 401-420
<400> 6693
GGTTGTGATT CTGGCCGACG GACAAATCAC GCCCTTCTCT TTGTTGATGG 50
CTAACGCCGG GACCGGTGAG CCGCTCCTGA CGTTAGTCTG TTCAGGCTCA 100

<210> 6694
<211> 100
<212> DNA
<213> Escherichia coli K-12 MG1655
<220> hofh b3330
<222> 329-428
<223> hofh b3330 U00096 3459105..3459614
<313> 6694: 358-377
<400> 6694
ATCTTCAGCC ATTTAAACCT GATGACAGCA ACCAGCCACA GGTTGTGATT 50
CTGGCCGACG GACAAATCAC GCCCTTCTCT TTGTTGATGG CTAACGCCGG 100
<210> 6695
<211> 100
<212> DNA
<213> Escherichia coli K-12 MG1655
<220> imp b0055
<222> 2250-2349
<223> imp b0055 U00096 complement(54755..57109)
<313> 6695: 2292-2311
<400> 6695
CGCAATCGGC TTTAACATCG AACTTCGCGG CCTGAGCTCC AACTACGGTC 50
TGGGTACGCA AGAGATGCTG CGTTCGAACA TTCTGCCGTA TCAAAACACT 100
<210> 6696
<211> 100
<212> DNA
<213> Escherichia coli K-12 MG1655
<220> imp b0055
<222> 2226-2325
<223> imp b0055 U00096 complement(54755..57109)
<313> 6696: 2252-2271
<400> 6696
TAAACAACAT GCGGTATATG ACAACGCAAT CGGCTTTAAC ATCGAACTTC 50
GCGGCCTGAG CTCCAACTAC GGTCTGGGTA CGCAAGAGAT GCTGCGTTCG 100
<210> 6697
<211> 100
<212> DNA
<213> Escherichia coli K-12 MG1655
<220> imp b0055
<222> 2160-2259
<223> imp b0055 U00096 complement(54755..57109)
<313> 6697: 2215-2234
<400> 6697
ATACAGCTCC TGCTGCTATG CAATTCGCGT CGGTTACGAG CGGAAGCTGA 50
ACGGTTGGGA TAACGATAAA CAACATGCGG TATATGACAA CGCAATCGGC 100
<210> 6698
<211> 100
<212> DNA
<213> Escherichia coli K-12 MG1655
<220> hofq b3392
<222> 1127-1226
<223> hofq b3392 U00096 complement(3517103..3518341)
<313> 6698: 1173-1192
<400> 6698
CGTTGCTTGG CGACATTCCC TGGTTCGGGC AATTATTTCG TCATGACGGA 50
AAAGAAGATG AACGACGCGA GTTAGTGGTG TTTATCACGC CACGACTGGT 100
<210> 6699
<211> 100
<212> DNA
<213> Escherichia coli K-12 MG1655
<220> hofq b3392
<222> 1099-1198
<223> hofq b3392 U00096 complement(3517103..3518341)
<313> 6699: 1125-1144
<400> 6699
AAAAATAAAT CGGGTCAGGA TAGCGTACCG TTGCTTGGCG ACATTCCCTG 50
GTTCGGGCAA TTATTTCGTC ATGACGGAAA AGAAGATGAA CGACGCGAGT 100
<210> 6700
<211> 100
<212> DNA
<213> Escherichia coli K-12 MG1655
<220> hofq b3392

<222> 1018-1117
<223> hofq b3392 U00096 complement(3517103..3518341)
<313> 6700: 1043-1062
<400> 6700
GCGATTGATA AGCAGGAGAT CGAAACGCAG GTCGAGGTCA AAAGCGGAGA 50
AACGTTGGCG CTGGGCGGCA TTTTTACCCG TAAAAATAAA TCGGGTCAGG 100
<210> 6701
<211> 100
<212> DNA
<213> Escherichia coli K-12 MG1655
<220> b0960 b0961
<222> 2064-2163
<223> b0960 b0961 U00096 complement(1020953..1023115)
<313> 6701: 2132-2151
<400> 6701
CGTACAACAA GTTGGATTAT TGATTGCATT ACTGCCTGAG ATTGGTCGTC 50
TGCAACGCCA GATTACTCAA GTTCCGCAGG AAACTCCTGT TTCGGCGTAA 100
<210> 6702
<211> 100
<212> DNA
<213> Escherichia coli K-12 MG1655
<220> b0960 b0961
<222> 2064-2163
<223> b0960 b0961 U00096 complement(1020953..1023115)
<313> 6702: 2089-2108
<400> 6702
CGTACAACAA GTTGGATTAT TGATTGCATT ACTGCCTGAG ATTGGTCGTC 50
TGCAACGCCA GATTACTCAA GTTCCGCAGG AAACTCCTGT TTCGGCGTAA 100
<210> 6703
<211> 100
<212> DNA
<213> Escherichia coli K-12 MG1655
<220> b0960 b0961
<222> 1999-2098
<223> b0960 b0961 U00096 complement(1020953..1023115)
<313> 6703: 2051-2070
<400> 6703
TTAGCTAGCC TGAAACAGCG GATGCAGCAA CTTGAACCAC GGGCAGACAG 50
CAAAGAACCT CTGGTCGTAC AACAAGTTGG ATTATTGATT GCATTACTGC 100
<210> 6704
<211> 100
<212> DNA
<213> Escherichia coli K-12 MG1655
<220> b1690 b1690
<222> 1113-1212
<223> b1690 b1690 U00096 1769095..1770309
<313> 6704: 1146-1165
<400> 6704
CATCGCTGCC ATCGGTTTTT TACTGGCACT GTTTATCGGC TTACGCAGCC 50
GCAAAAAAAC GCGGCATCAC TCGCTAAAGG AAAATGTCGC TCCGGGTGGG 100
<210> 6705
<211> 100
<212> DNA
<213> Escherichia coli K-12 MG1655
<220> b1690 b1690
<222> 1067-1166
<223> b1690 b1690 U00096 1769095..1770309
<313> 6705: 1115-1134
<400> 6705
ATCTGTCCCA AAGAAGTATT GCCGATATTA TGTGGTTCGA TACCGCCATC 50
GCTGCCATCG GTTTTTTACT GGCACTGTTT ATCGGCTTAC GCAGCCGCAA 100
<210> 6706
<211> 100
<212> DNA
<213> Escherichia coli K-12 MG1655
<220> b1690 b1690
<222> 1016-1115
<223> b1690 b1690 U00096 1769095..1770309
<313> 6706: 1071-1090
<400> 6706
ATTACAGTGC GGGCAGTATT GCGACCTTTA CTATTCCGTT GATTACGGCT 50

CATCTGTCCC AAAGAAGTAT TGCCGATATT ATGTGGTTCG ATACCGCCAT 100
<210> 6707
<211> 100
<212> DNA
<213> Escherichia coli K-12 MG1655
<220> b1691 b1692
<222> 1173-1272
<223> b1691 b1692 U00096 1770530..1771801
<313> 6707: 1241-1260
<400> 6707
TATCCGCTAT TACCGCGTTT TCATTATTCC TGAAAATGAT GTGCGGTTTG 50
GCGAGCGTAA ATTTTGCACC CGGTTAAACA CAATTAAGCA TAGAGGTTAA 100
<210> 6708
<211> 100
<212> DNA
<213> Escherichia coli K-12 MG1655
<220> b1691 b1692
<222> 1106-1205
<223> b1691 b1692 U00096 1770530..1771801
<313> 6708: 1149-1168
<400> 6708
TGCAATATAT CATTGTTCTC GATTTTACTT TCGCGCTGCT GGCCCTGATT 50
ACCGCAATTA TTGTTTTTAT CCGCTATTAC CGCGTTTTCA TTATTCCTGA 100
<210> 6709
<211> 100
<212> DNA
<213> Escherichia coli K-12 MG1655
<220> b1691 b1692
<222> 972-1071
<223> b1691 b1692 U00096 1770530..1771801
<313> 6709: 1010-1029
<400> 6709
ACAGCTCGGC GTTTCGGTCA TGTCAGAGTT TTTTCCCAAA AGCAAAGCCA 50
AAGTCACCAG TATTTATATG ATGATGGGTG GACTGGCTAA CTTTGTTATT 100
<210> 6710
<211> 100
<212> DNA
<213> Escherichia coli K-12 MG1655
<220> b0964 b0964
<222> 549-648
<223> b0964 b0964 U00096 complement(1026334..1026996)
<313> 6710: 598-617
<400> 6710
TCCACCGCAG TCTCAGGTGC TCACAGAACA ACGGCTGAAG TATTGGTTTA 50
AACTGGCTGA CCCACAAACG CGAAATACTT TCCTGCAGTG GGCGGAAAAA 100
<210> 6711
<211> 100
<212> DNA
<213> Escherichia coli K-12 MG1655
<220> b0964 b0964
<222> 507-606
<223> b0964 b0964 U00096 complement(1026334..1026996)
<313> 6711: 565-584
<400> 6711
TGACAGTACG CTGCTTTCGG GTGTTTCCGA GCTGGATGCT ATTCCACCGC 50
AGTCTCAGGT GCTCACAGAA CAACGGCTGA AGTATTGGTT TAAACTGGCT 100
<210> 6712
<211> 100
<212> DNA
<213> Escherichia coli K-12 MG1655
<220> b0964 b0964
<222> 488-587
<223> b0964 b0964 U00096 complement(1026334..1026996)
<313> 6712: 521-540
<400> 6712
TTGCCACGAT GATGGCAGAT GACAGTACGC TGCTTTCGGG TGTTTCCGAG 50
CTGGATGCTA TTCCACCGCA GTCTCAGGTG CTCACAGAAC AACGGCTGAA 100
<210> 6713
<211> 100
<212> DNA
<213> Escherichia coli K-12 MG1655

<220> b0965 b0966
<222> 396-495
<223> b0965 b0966 U00096 1027088..1027582
<313> 6713: 442-461
<400> 6713
TAATGAGCAA GCGGCAGTAC TGGCACGGGA TGCCGGGTTA AATGTGGTGA  50
TGGATCGCTG CCCGGCTATT GAGATCCCTC GCCTGGGCCT GGCCAAATAA  100
<210> 6714
<211> 100
<212> DNA
<213> Escherichia coli K-12 MG1655
<220> b0965 b0966
<222> 361-460
<223> b0965 b0966 U00096 1027088..1027582
<313> 6714: 389-408
<400> 6714
GGCGCGAAAA CTCTCTGGAT GCAACTGGGG GTGATTAATG AGCAAGCGGC  50
AGTACTGGCA CGGGATGCCG GGTTAAATGT GGTGATGGAT CGCTGCCCGG  100
<210> 6715
<211> 100
<212> DNA
<213> Escherichia coli K-12 MG1655
<220> b0965 b0966
<222> 325-424
<223> b0965 b0966 U00096 1027088..1027582
<313> 6715: 336-355
<400> 6715
GCGGCGTGGG GCGTGGCACA GGAAGCTATT GCCATTGGCG CGAAAACTCT  50
CTGGATGCAA CTGGGGGTGA TTAATGAGCA AGCGGCAGTA CTGGCACGGG  100
<210> 6716
<211> 100
<212> DNA
<213> Escherichia coli K-12 MG1655
<220> b1695 b1695
<222> 1107-1206
<223> b1695 b1695 U00096 1775166..1776371
<313> 6716: 1181-1200
<400> 6716
CTGGCGTGAT GTCCGTTGTG AACGTATCGG CGGCGGTACA GACGAAATTA  50
TGATTTACGT AGCAGGTCGG CAGATCCTGA AAGACTATCA GAACAAATAA  100
<210> 6717
<211> 100
<212> DNA
<213> Escherichia coli K-12 MG1655
<220> b1695 b1695
<222> 1050-1149
<223> b1695 b1695 U00096 1775166..1776371
<313> 6717: 1108-1127
<400> 6717
TGCGATTCAA ATCATGGGCG GTCTGGGCTA TACCGATGAG GCGCGCGTCT  50
CCCGCTTCTG GCGTGATGTC CGTTGTGAAC GTATCGGCGG CGGTACAGAC  100
<210> 6718
<211> 100
<212> DNA
<213> Escherichia coli K-12 MG1655
<220> b1695 b1695
<222> 937-1036
<223> b1695 b1695 U00096 1775166..1776371
<313> 6718: 968-987
<400> 6718
CGCAACATGG TGCTGAAAGT GGCATGGCAA GCCGATCAGC ATCAGTCACT  50
GCGCACCAGC GCGGCGCTGG CAAAACTGTA CTGTGCACGT ACCGCAATGG  100
<210> 6719
<211> 100
<212> DNA
<213> Escherichia coli K-12 MG1655
<220> b1696 b1697
<222> 802-901
<223> b1696 b1697 U00096 complement(1776414..1777325)
<313> 6719: 833-852
<400> 6719

TTGCGCCATG TCGGTTGTTC GCCCAGCGAT TACCGTAGAC AATTTAAAAA 50
CTGTTTTGCG GAACAAGAAA TCCTATCTGA ATTTCCTCAA CCGGTAAGTC 100
<210> 6720
<211> 100
<212> DNA
<213> Escherichia coli K-12 MG1655
<220> b1696 b1697
<222> 733-832
<223> b1696 b1697 U00096 complement(1776414..1777325)
<313> 6720: 788-807
<400> 6720
GAATTATCAC AGGCAGAGAT CTCCTGGCGT GTGGGTTATG AAAATGTCGA 50
TCACTTTGCC AAACTGTTTT TGCGCCATGT CGGTTGTTCG CCCAGCGATT 100
<210> 6721
<211> 100
<212> DNA
<213> Escherichia coli K-12 MG1655
<220> b1696 b1697
<222> 681-780
<223> b1696 b1697 U00096 complement(1776414..1777325)
<313> 6721: 706-725
<400> 6721
CTATGTTATT CAACGGCGTA TGACGGAAGC AAAATGGTCA CTCACTAATA 50
CTGAATTATC ACAGGCAGAG ATCTCCTGGC GTGTGGGTTA TGAAAATGTC 100
<210> 6722
<211> 100
<212> DNA
<213> Escherichia coli K-12 MG1655
<220> b0967 b0967
<222> 1005-1104
<223> b0967 b0967 U00096 complement(1028002..1029105)
<313> 6722: 1079-1098
<400> 6722
ACAATTTATA GAGCAGTTCC GTCAGGCAGC CGATCATCCG GTGATCGCTA 50
CCTATCCGGA AGGGCTATAT CTGAAAGGGT TTGCCTGTCG CGTCATGTAA 100
<210> 6723
<211> 100
<212> DNA
<213> Escherichia coli K-12 MG1655
<220> b0967 b0967
<222> 984-1083
<223> b0967 b0967 U00096 complement(1028002..1029105)
<313> 6723: 1012-1031
<400> 6723
AATTGATGCC GGTCGTGATG TACAATTTAT AGAGCAGTTC CGTCAGGCAG 50
CCGATCATCC GGTGATCGCT ACCTATCCGG AAGGGCTATA TCTGAAAGGG 100
<210> 6724
<211> 100
<212> DNA
<213> Escherichia coli K-12 MG1655
<220> b0967 b0967
<222> 916-1015
<223> b0967 b0967 U00096 complement(1028002..1029105)
<313> 6724: 967-986
<400> 6724
CTCCTGACTT TCTCCTGTTC CGGTCTGATG ACCAGCGATT TATTTCAGAA 50
AATCATCGCG GATGCCGCAA TTGATGCCGG TCGTGATGTA CAATTTATAG 100
<210> 6725
<211> 100
<212> DNA
<213> Escherichia coli K-12 MG1655
<220> b0968 b0969
<222> 140-239
<223> b0968 b0969 U00096 1029287..1029565
<313> 6725: 175-194
<400> 6725
TTGCTTGCGG TGAAGAAGGG CAGGTGGAAA AATTAATGCA GTGGTTAAAG 50
AGTGGCGGCC CGCGTTCTGC GCGTGTAGAA CGGGTGCTCA GCGAGCCGCA 100
<210> 6726
<211> 100
<212> DNA

<213> Escherichia coli K-12 MG1655
<220> b0968 b0969
<222> 61-160
<223> b0968 b0969 U00096 1029287..1029565
<313> 6726: 114-133
<400> 6726
TACACCACAC AGTACGAAGC GAAAAGACTG GGTTTAACCG GGTACGCCAA 50
AAATCTTGAC GATGGCAGCG TGGAAGTGGT TGCTTGCGGT GAAGAAGGGC 100
<210> 6727
<211> 100
<212> DNA
<213> Escherichia coli K-12 MG1655
<220> b0968 b0969
<222> 47-146
<223> b0968 b0969 U00096 1029287..1029565
<313> 6727: 79-98
<400> 6727
GCGTAGGATT TCGCTACACC ACACAGTACG AAGCGAAAAG ACTGGGTTTA 50
ACCGGGTACG CCAAAAATCT TGACGATGGC AGCGTGGAAG TGGTTGCTTG 100
<210> 6728
<211> 100
<212> DNA
<213> Escherichia coli K-12 MG1655
<220> tyna b1387
<222> 2175-2274
<223> tyna b1387 U00096 complement(1447100..1449373)
<313> 6728: 2245-2264
<400> 6728
GTGGCCGATT ATGCCGACCG AATGGGTACA TACTCTGCTG AAACCATGGA 50
ACTTCTTTGA CGAAACGCCA ACGCTAGGGG CGCTGAAGAA AGATAAGTGA 100
<210> 6729
<211> 100
<212> DNA
<213> Escherichia coli K-12 MG1655
<220> tyna b1387
<222> 2136-2235
<223> tyna b1387 U00096 complement(1447100..1449373)
<313> 6729: 2200-2219
<400> 6729
GATGACCACC GGCACCACAC ATGTGGCCCG CGCCGAAGAG TGGCCGATTA 50
TGCCGACCGA ATGGGTACAT ACTCTGCTGA AACCATGGAA CTTCTTTGAC 100
<210> 6730
<211> 100
<212> DNA
<213> Escherichia coli K-12 MG1655
<220> tyna b1387
<222> 2074-2173
<223> tyna b1387 U00096 complement(1447100..1449373)
<313> 6730: 2093-2112
<400> 6730
ACCGGTCTTG GACAATACAG TAAGGATAAC GAGTCGCTGG ACAACACCGA 50
CGCCGTTGTC TGGATGACCA CCGGCACCAC ACATGTGGCC CGCGCCGAAG 100
<210> 6731
<211> 100
<212> DNA
<213> Escherichia coli K-12 MG1655
<220> ugpa b3453
<222> 789-888
<223> ugpa b3453 U00096 complement(3587655..3588542)
<313> 6731: 852-871
<400> 6731
GGCTTCGTCT GCCGCACAGT CGGTGGTGTT GATGTTCCTC GTCATCGTGC 50
TGACGGTGGT GCAGTTCCGC TATGTTGAAA GCAAGGTGCG TTACCAATGA 100
<210> 6732
<211> 100
<212> DNA
<213> Escherichia coli K-12 MG1655
<220> ugpa b3453
<222> 749-848
<223> ugpa b3453 U00096 complement(3587655..3588542)
<313> 6732: 801-820

<400> 6732
TTTATAAGAT CTACCGCGAA GGTTTTACCG GACTGGATCT GGCTTCGTCT 50
GCCGCACAGT CGGTGGTGTT GATGTTCCTC GTCATCGTGC TGACGGTGGT 100
<210> 6733
<211> 100
<212> DNA
<213> Escherichia coli K-12 MG1655
<220> ugpa b3453
<222> 706-805
<223> ugpa b3453 U00096 complement(3587655..3588542)
<313> 6733: 747-766
<400> 6733
GCCGCCACGT CCGGCGGGCC GGTTCAGGCC ACCACGACGC TGATTTATAA 50
GATCTACCGC GAAGGTTTTA CCGGACTGGA TCTGGCTTCG TCTGCCGCAC 100
<210> 6734
<211> 100
<212> DNA
<213> Escherichia coli K-12 MG1655
<220> ugpb b3454
<222> 1218-1317
<223> ugpb b3454 U00096 complement(3588640..3589956)
<313> 6734: 1290-1309
<400> 6734
CGTGTGGACC GGTAAGAAGA CACCACAGCA GGCACTGGAT ACCGCCGTTG 50
AGCGTGGAAA TCAGTTGCTG CGCCGCTTTG AGAAATCGAC GAAGTCTTAA 100
<210> 6735
<211> 100
<212> DNA
<213> Escherichia coli K-12 MG1655
<220> ugpb b3454
<222> 1204-1303
<223> ugpb b3454 U00096 complement(3588640..3589956)
<313> 6735: 1252-1271
<400> 6735
GAAGAGCTGG AGAGCGTGTG GACCGGTAAG AAGACACCAC AGCAGGCACT 50
GGATACCGCC GTTGAGCGTG GAAATCAGTT GCTGCGCCGC TTTGAGAAAT 100
<210> 6736
<211> 100
<212> DNA
<213> Escherichia coli K-12 MG1655
<220> ugpb b3454
<222> 1090-1189
<223> ugpb b3454 U00096 complement(3588640..3589956)
<313> 6736: 1146-1165
<400> 6736
GAGAAAAACC CAGGGGCGGA TACCGCGACG CGTCAGATGC TGAATAAGCC 50
GCCGTTGCCG TTCACCAAAG GGCTGCGTCT GGGCAACATG CCGCAGATCC 100
<210> 6737
<211> 100
<212> DNA
<213> Escherichia coli K-12 MG1655
<220> ugpc b3451
<222> 952-1051
<223> ugpc b3451 U00096 complement(3585741..3586850)
<313> 6737: 974-993
<400> 6737
CTCGGCGCAG ATAACCTGGC GCACGGACGC TGGGGCGAGC AGAAGCTGGT 50
GGTGCGACTG GCGCATCAGG AGCGCCCGAC GGCAGGCAGC ACGCTGTGGC 100
<210> 6738
<211> 100
<212> DNA
<213> Escherichia coli K-12 MG1655
<220> ugpc b3451
<222> 910-1009
<223> ugpc b3451 U00096 complement(3585741..3586850)
<313> 6738: 947-966
<400> 6738
GCAGAAGGCG GCGTACCGAT GGTGATGGAC ACGCTGGAGA TCCTCGGCGC 50
AGATAACCTG GCGCACGGAC GCTGGGGCGA GCAGAAGCTG GTGGTGCGAC 100
<210> 6739
<211> 100

<212> DNA
<213> Escherichia coli K-12 MG1655
<220> ugpc b3451
<222> 878-977
<223> ugpc b3451 U00096 complement(3585741..3586850)
<313> 6739: 909-928
<400> 6739
TTCGCCCGGA ACATATTGCG CTAAGCTCGC AGGCAGAAGG CGGCGTACCG 50
ATGGTGATGG ACACGCTGGA GATCCTCGGC GCAGATAACC TGGCGCACGG 100
<210> 6740
<211> 100
<212> DNA
<213> Escherichia coli K-12 MG1655
<220> ugpe b3452
<222> 747-846
<223> ugpe b3452 U00096 complement(3586813..3587658)
<313> 6740: 787-806
<400> 6740
AGTGATGGTG GCGATGTTGT TAACGCTTAT CCCTCCGGTG GTGATTGTTT 50
TAGTGATGCA GCGTGCCTTC GTGCGCGGCC TGGTCGATAG TGAGAAATAA 100
<210> 6741
<211> 100
<212> DNA
<213> Escherichia coli K-12 MG1655
<220> ugpe b3452
<222> 681-780
<223> ugpe b3452 U00096 complement(3586813..3587658)
<313> 6741: 731-750
<400> 6741
CGGCACCACC GTGGCAGGGA TCAAAGGGAT GATCGCTACA GGCGAAGGCA 50
CCACGGAATG GAACTCAGTG ATGGTGGCGA TGTTGTTAAC GCTTATCCCT 100
<210> 6742
<211> 100
<212> DNA
<213> Escherichia coli K-12 MG1655
<220> ugpe b3452
<222> 673-772
<223> ugpe b3452 U00096 complement(3586813..3587658)
<313> 6742: 694-713
<400> 6742
GTGGATCTCG GCACCACCGT GGCAGGGATC AAAGGGATGA TCGCTACAGG 50
CGAAGGCACC ACGGAATGGA ACTCAGTGAT GGTGGCGATG TTGTTAACGC 100
<210> 6743
<211> 100
<212> DNA
<213> Escherichia coli K-12 MG1655
<220> b3808 b3809
<222> 387-486
<223> b3808 b3809 U00096 3991463..3991948
<313> 6743: 432-451
<400> 6743
TGTTTCTGGT AAACATTATG ATCAGGTTTA CCGAGCGAGC ATCCTCACGC 50
TGACGGAACT AAAAAAGACA ACAAACAAAC CACATTGCGA TAGTGCATAA 100
<210> 6744
<211> 100
<212> DNA
<213> Escherichia coli K-12 MG1655
<220> b3808 b3809
<222> 318-417
<223> b3808 b3809 U00096 3991463..3991948
<313> 6744: 384-403
<400> 6744
AATAATGATG TCTCTGTGTA GCGAAAGATT TGTCTCTTCA TTAGGGCGCA 50
GTTACACCAC GTCTTTCCCT GTTTCTGGTA AACATTATGA TCAGGTTTAC 100
<210> 6745
<211> 100
<212> DNA
<213> Escherichia coli K-12 MG1655
<220> b3808 b3809
<222> 260-359
<223> b3808 b3809 U00096 3991463..3991948

<313> 6745: 315-334
<400> 6745
AGGGCTTCAA AGTTTGCACC TCTGCGGCTG CGTTCCGGCA CGATTCATCC  50
GTCACCGGAA TAATGATGTC TCTGTGTAGC GAAAGATTTG TCTCTTCATT  100
<210> 6746
<211> 100
<212> DNA
<213> Escherichia coli K-12 MG1655
<220> ugpq b3450
<222> 645-744
<223> ugpq b3450 U00096 complement(3585001..3585744)
<313> 6746: 715-734
<400> 6746
CAATAAACCC CAGCGCGCAG CAGAGTTGCT GCGCTGGGGT GTGGATTGCA  50
TCTGTACCGA TGCGATTGAC GTGATTGGTC CGAACTTTAC GGCCCAATAG  100
<210> 6747
<211> 100
<212> DNA
<213> Escherichia coli K-12 MG1655
<220> ugpq b3450
<222> 562-661
<223> ugpq b3450 U00096 complement(3585001..3585744)
<313> 6747: 614-633
<400> 6747
ATTCATCTCA ATCATAAGTT ACTCAATAAA GCGCGAGTGA TGCAGTTGAA  50
AGACGCCGGA CTGCGGATTC TGGTTTATAC CGTCAATAAA CCCCAGCGCG  100
<210> 6748
<211> 100
<212> DNA
<213> Escherichia coli K-12 MG1655
<220> ugpq b3450
<222> 499-598
<223> ugpq b3450 U00096 complement(3585001..3585744)
<313> 6748: 561-580
<400> 6748
TTGTTGCTGG ATGAGTGGCG CGACGACTGG CGCGAACTGA CCGCGCGGCT  50
GGGCTGCGTC TCTATTCATC TCAATCATAA GTTACTCAAT AAAGCGCGAG  100
<210> 6749
<211> 100
<212> DNA
<213> Escherichia coli K-12 MG1655
<220> uvra b4059
<222> 2724-2823
<223> uvra b4059 U00096 complement(4268628..4271450)
<313> 6749: 2790-2809
<400> 6749
CGGCAGTGGT GGCGGCGAGA TCCTCGTCTC CGGTACGCCA GAAACCGTCG  50
CGGAGTGCGA AGCATCACAC ACGGCACGCT TCCTTAAGCC GATGCTGTAA  100
<210> 6750
<211> 100
<212> DNA
<213> Escherichia coli K-12 MG1655
<220> uvra b4059
<222> 2690-2789
<223> uvra b4059 U00096 complement(4268628..4271450)
<313> 6750: 2716-2735
<400> 6750
CCGCTGACTG GATTGTCGAC CTGGGACCAG AAGGCGGCAG TGGTGGCGGC  50
GAGATCCTCG TCTCCGGTAC GCCAGAAACC GTCGCGGAGT GCGAAGCATC  100
<210> 6751
<211> 100
<212> DNA
<213> Escherichia coli K-12 MG1655
<220> uvra b4059
<222> 2626-2725
<223> uvra b4059 U00096 complement(4268628..4271450)
<313> 6751: 2682-2701
<400> 6751
CATAAACTGC GCGATCAGGG CAACACCATT GTGGTGATTG AGCACAATCT  50
CGACGTGATC AAAACCGCTG ACTGGATTGT CGACCTGGGA CCAGAAGGCG  100
<210> 6752

<211> 100
<212> DNA
<213> Escherichia coli K-12 MG1655
<220> uvrb b0780
<222> 1923-2022
<223> uvrb b0780 U00096 812749..814770
<313> 6752: 1955-1974
<400> 6752
GTTGATGATG CAACACGCGC AGAATCTGGA GTTCGAAGAA GCGGCGCAAA 50
TTCGTGACCA GTTGCATCAG CTGCGTGAGC TGTTTATCGC GGCATCGTAA 100
<210> 6753
<211> 100
<212> DNA
<213> Escherichia coli K-12 MG1655
<220> uvrb b0780
<222> 1882-1981
<223> uvrb b0780 U00096 812749..814770
<313> 6753: 1912-1931
<400> 6753
TCGCCTAAAG CGTTGCAGCA GAAAATCCAT GAGCTGGAAG GGTTGATGAT 50
GCAACACGCG CAGAATCTGG AGTTCGAAGA AGCGGCGCAA ATTCGTGACC 100
<210> 6754
<211> 100
<212> DNA
<213> Escherichia coli K-12 MG1655
<220> uvrb b0780
<222> 1814-1913
<223> uvrb b0780 U00096 812749..814770
<313> 6754: 1848-1867
<400> 6754
AAACCAAAGC GAAGGGCAGA GGAAAATCGC GCCCGATTGT TGAGCCGGAT 50
AATGTGCCGA TGGATATGTC GCCTAAAGCG TTGCAGCAGA AAATCCATGA 100
<210> 6755
<211> 100
<212> DNA
<213> Escherichia coli K-12 MG1655
<220> grea b3182
<222> 363-462
<223> grea b3182 U00096 complement(3325880..3326341)
<313> 6755: 418-437
<400> 6755
TGCTCGTGGC CTGATCGGCA AAGAAGAAGA TGATGTTGTG GTCATCAAAA 50
CGCCGGGCGG CGAAGTAGAA TTTGAAGTAA TTAAGGTGGA ATACCTGTAA 100
<210> 6756
<211> 100
<212> DNA
<213> Escherichia coli K-12 MG1655
<220> grea b3182
<222> 298-397
<223> grea b3182 U00096 complement(3325880..3326341)
<313> 6756: 358-377
<400> 6756
TATCGCATCG TTGGCGATGA CGAAGCTGAC TTTAAACAAA ACCTGATTTC 50
TGTAAACTCG CCTATTGCTC GTGGCCTGAT CGGCAAAGAA GAAGATGATG 100
<210> 6757
<211> 100
<212> DNA
<213> Escherichia coli K-12 MG1655
<220> grea b3182
<222> 136-235
<223> grea b3182 U00096 complement(3325880..3326341)
<313> 6757: 175-194
<400> 6757
GCTCGTGAAC AGCAGGGTTT CTGCGAAGGC CGTATTAAAG ACATCGAAGC 50
CAAGCTGTCG AACGCGCAGG TGATTGATGT CACCAAAATG CCCAACAATG 100
<210> 6758
<211> 100
<212> DNA
<213> Escherichia coli K-12 MG1655
<220> uvrc b1914
<222> 1668-1767

<223> uvrc b1914 U00096 complement(1990897..1992663)
<313> 6758: 1696-1715
<400> 6758
TTTGCAAGGT TTACGTAACG CCAGCGTCGA GGAAATTGCA AAAGTGCCGG 50
GTATTTCGCA AGGTCTGGCA GAAAAGATCT TCTGGTCGTT GAAACATTGA 100
<210> 6759
<211> 100
<212> DNA
<213> Escherichia coli K-12 MG1655
<220> uvrc b1914
<222> 1608-1707
<223> uvrc b1914 U00096 complement(1990897..1992663)
<313> 6759: 1647-1666
<400> 6759
GGAAACCATT GAAGGCGTCG GGCCAAAACG TCGGCAAATG TTGTTGAAAT 50
ATATGGGCGG TTTGCAAGGT TTACGTAACG CCAGCGTCGA GGAAATTGCA 100
<210> 6760
<211> 100
<212> DNA
<213> Escherichia coli K-12 MG1655
<220> uvrc b1914
<222> 1574-1673
<223> uvrc b1914 U00096 complement(1990897..1992663)
<313> 6760: 1609-1628
<400> 6760
AAAAACGGGC GAAGGTCAAA AATACCAGTT CCCTGGAAAC CATTGAAGGC 50
GTCGGGCCAA AACGTCGGCA AATGTTGTTG AAATATATGG GCGGTTTGCA 100
<210> 6761
<211> 100
<212> DNA
<213> Escherichia coli K-12 MG1655
<220> greb b3407
<222> 414-513
<223> greb b3407 U00096 3534414..3534926
<313> 6761: 474-493
<400> 6761
CGCATTGCTG AAAAAAGAAG TCGGCGATCT GGCGGTGGTG AATACCCCTG 50
CCGGGGAAGC GAGCTGGTAT GTTAATGCTA TCGAGTACGT GAAACCGTAA 100
<210> 6762
<211> 100
<212> DNA
<213> Escherichia coli K-12 MG1655
<220> greb b3407
<222> 405-504
<223> greb b3407 U00096 3534414..3534926
<313> 6762: 444-463
<400> 6762
GATGGCCCGC GCATTGCTGA AAAAAGAAGT CGGCGATCTG GCGGTGGTGA 50
ATACCCCTGC CGGGGAAGCG AGCTGGTATG TTAATGCTAT CGAGTACGTG 100
<210> 6763
<211> 100
<212> DNA
<213> Escherichia coli K-12 MG1655
<220> greb b3407
<222> 343-442
<223> greb b3407 U00096 3534414..3534926
<313> 6763: 398-417
<400> 6763
TTCCGTATTG TCGGCTACGA TGAAATTTTT GGCCGTAAAG ATTACATCTC 50
TATCGATTCC CCGATGGCCC GCGCATTGCT GAAAAAAGAA GTCGGCGATC 100
<210> 6764
<211> 100
<212> DNA
<213> Escherichia coli K-12 MG1655
<220> uvrd uvrD
<222> 2058-2157
<223> uvrd uvrD U00096 3995596..3997758
<313> 6764: 2113-2132
<400> 6764
TGTCAATATG GAAGGCAGCG GTGAGCATAG CCGTTTGCAG GTGGCATTTC 50
AGGGCCAGGG TATTAAATGG CTGGTGGCGG CATACGCCCG GCTGGAGTCG 100

<210> 6765
<211> 100
<212> DNA
<213> Escherichia coli K-12 MG1655
<220> uvrd uvrD
<222> 2032-2131
<223> uvrd uvrD U00096 3995596..3997758
<313> 6765: 2058-2077
<400> 6765
CACGCTAAGT TTGGTGAAGG CACCATTGTC AATATGGAAG GCAGCGGTGA 50
GCATAGCCGT TTGCAGGTGG CATTTCAGGG CCAGGGTATT AAATGGCTGG 100
<210> 6766
<211> 100
<212> DNA
<213> Escherichia coli K-12 MG1655
<220> uvrd uvrD
<222> 1942-2041
<223> uvrd uvrD U00096 3995596..3997758
<313> 6766: 1992-2011
<400> 6766
GCCACGGTAA GCCGCCCGGT CAGCCATCAG CGGATGGGTA CGCCGATGGT 50
CGAGAACGAC AGCGGCTACA AGCTCGGCCA GCGCGTACGC CACGCTAAGT 100
<210> 6767
<211> 100
<212> DNA
<213> Escherichia coli K-12 MG1655
<220> b3814 b3815
<222> 201-300
<223> b3814 b3815 U00096 complement(3997905..3998204)
<313> 6767: 262-281
<400> 6767
TCTTGATGGC ACGATTTCAC ATATGTATAT GAAAGGCGGG GCAATCCCGT 50
GGCAACGCTA TCCTTTTGTC ACCATGATTT CCGTCGAGCG GAAAGGTTAA 100
<210> 6768
<211> 100
<212> DNA
<213> Escherichia coli K-12 MG1655
<220> b3814 b3815
<222> 124-223
<223> b3814 b3815 U00096 complement(3997905..3998204)
<313> 6768: 162-181
<400> 6768
TTGAGCCAGC AGGCAACAAA TTTTTATGAT TTTGCCTGTT ATGCCAAAGC 50
GAAACTGAAC GTTGAGCAGC TGCTTTATCT TGATGGCACG ATTTCACATA 100
<210> 6769
<211> 100
<212> DNA
<213> Escherichia coli K-12 MG1655
<220> b3814 b3815
<222> 32-131
<223> b3814 b3815 U00096 complement(3997905..3998204)
<313> 6769: 73-92
<400> 6769
GTGTAATTAA TCCGCGTATT CATCCCAACG TCGCCTCAAG CAAAATTCGT 50
AACGGTGTTG GGATTAATAA ACATGGGAAC GCCGTGTTTT TGTTGAGCCA 100
<210> 6770
<211> 100
<212> DNA
<213> Escherichia coli K-12 MG1655
<220> uvry b1915
<222> 546-645
<223> uvry b1915 U00096 complement(1992726..1993382)
<313> 6770: 588-607
<400> 6770
CAGCTACCGC TATCGTATGT TCAGTAAACT AAACATTCAT GGCGATGTTG 50
AGCTGACTCA CCTGGCAATT CGCCATGGTC TGTGTAATGC GGAGACATTA 100
<210> 6771
<211> 100
<212> DNA
<213> Escherichia coli K-12 MG1655
<220> uvry b1915

<222> 495-594
<223> uvry b1915 U00096 complement(1992726..1993382)
<313> 6771: 543-562
<400> 6771
GAAGGTCAAT GAGATCTCAG AACAGCTCAA TCTCAGTCCG AAAACGGTGA 50
ACAGCTACCG CTATCGTATG TTCAGTAAAC TAAACATTCA TGGCGATGTT 100
<210> 6772
<211> 100
<212> DNA
<213> Escherichia coli K-12 MG1655
<220> uvry b1915
<222> 455-554
<223> uvry b1915 U00096 complement(1992726..1993382)
<313> 6772: 484-503
<400> 6772
AACGTGAATT GCAGATTATG CTGATGATCA CCAAGGGCCA GAAGGTCAAT 50
GAGATCTCAG AACAGCTCAA TCTCAGTCCG AAAACGGTGA ACAGCTACCG 100
<210> 6773
<211> 100
<212> DNA
<213> Escherichia coli K-12 MG1655
<220> fuca b2801
<222> 549-648
<223> fuca b2801 U00096 complement(2931063..2931710)
<313> 6773: 594-613
<400> 6773
GACCCTGGCG ATTACGGACC CGGTGCCAGT GCTGAGCGAT GAAGAGATTG 50
CCGTAGTGCT GGAGAAATTC AAAACCTATG GGTTACGAAT TGAAGAGTAA 100
<210> 6774
<211> 100
<212> DNA
<213> Escherichia coli K-12 MG1655
<220> fuca b2801
<222> 493-592
<223> fuca b2801 U00096 complement(2931063..2931710)
<313> 6774: 538-557
<400> 6774
GAAAAAGCGT TATGGCTGGC GCATGAAGTT GAAGTGCTGG CGCAACTTTA 50
CCTGACGACC CTGGCGATTA CGGACCCGGT GCCAGTGCTG AGCGATGAAG 100
<210> 6775
<211> 100
<212> DNA
<213> Escherichia coli K-12 MG1655
<220> fuca b2801
<222> 460-559
<223> fuca b2801 U00096 complement(2931063..2931710)
<313> 6775: 502-521
<400> 6775
CATCATGGGC TTATCGCTTG TGAGGTGAAT CTGGAAAAAG CGTTATGGCT 50
GGCGCATGAA GTTGAAGTGC TGGCGCAACT TTACCTGACG ACCCTGGCGA 100
<210> 6776
<211> 100
<212> DNA
<213> Escherichia coli K-12 MG1655
<220> fuci b2803
<222> 1677-1776
<223> fuci b2803 U00096 2933606..2935381
<313> 6776: 1736-1755
<400> 6776
CAAAGTGTAT CGTCCTTCTG CCTGGGCTGC GCACGGCATG GATATTGAAG 50
GCCAGGATTA CCGCGCTTGC CAGAACTACG GTCCGTTGTA CAAGCGTTAA 100
<210> 6777
<211> 100
<212> DNA
<213> Escherichia coli K-12 MG1655
<220> fuci b2803
<222> 1567-1666
<223> fuci b2803 U00096 2933606..2935381
<313> 6777: 1621-1640
<400> 6777
AACTGGGGCG CTAACCATGG GGTTCTGACC ATCGGCCACG TTGGCGCAGA 50

CTTTATCACT CTCGCCTCCA TGCTGCGTAT CCCGGTATGT ATGCACAACG 100
<210> 6778
<211> 100
<212> DNA
<213> Escherichia coli K-12 MG1655
<220> fuci b2803
<222> 1513-1612
<223> fuci b2803 U00096 2933606..2935381
<313> 6778: 1548-1567
<400> 6778
GCACCGCGCC TCACCGGTAA AGGGCCGTTT ACGGATGTGT ACTCGGTAAT 50
GGCGAACTGG GGCGCTAACC ATGGGGTTCT GACCATCGGC CACGTTGGCG 100
<210> 6779
<211> 100
<212> DNA
<213> Escherichia coli K-12 MG1655
<220> fuck b2804
<222> 1349-1448
<223> fuck b2804 U00096 2935460..2936908
<313> 6779: 1386-1405
<400> 6779
TAGGGGAATT TAACAGCCCG GAAGAAGCCC GCGCACAGAT TCATTATCAG 50
TACCGTTATT TCTACCCGCA AACTGAACCT GAATTTATAG AGGAAGTGTG 100
<210> 6780
<211> 100
<212> DNA
<213> Escherichia coli K-12 MG1655
<220> fuck b2804
<222> 1317-1416
<223> fuck b2804 U00096 2935460..2936908
<313> 6780: 1349-1368
<400> 6780
CGCAGGAGCT GCGCTGTTCG GTTGGTATGG CGTAGGGGAA TTTAACAGCC 50
CGGAAGAAGC CCGCGCACAG ATTCATTATC AGTACCGTTA TTTCTACCCG 100
<210> 6781
<211> 100
<212> DNA
<213> Escherichia coli K-12 MG1655
<220> fuck b2804
<222> 1253-1352
<223> fuck b2804 U00096 2935460..2936908
<313> 6781: 1311-1330
<400> 6781
ATCAGATTAA AGCCAATATG CTTGATATTC CGGTAAAAGT TCTCGACGAC 50
GCCGAAACGA CCGTCGCAGG AGCTGCGCTG TTCGGTTGGT ATGGCGTAGG 100
<210> 6782
<211> 100
<212> DNA
<213> Escherichia coli K-12 MG1655
<220> rpia b2915
<222> 552-651
<223> rpia b2915 U00096 complement(3056686..3057345)
<313> 6782: 564-583
<400> 6782
CGCCATAAAT GCGATTCCTG GCGTGGTGAC TGTTGGCTTG TTTGCTAACC 50
GTGGCGCGGA CGTTGCGCTG ATTGGCACAC CTGACGGTGT CAAAACCATT 100
<210> 6783
<211> 100
<212> DNA
<213> Escherichia coli K-12 MG1655
<220> rpia b2915
<222> 483-582
<223> rpia b2915 U00096 complement(3056686..3057345)
<313> 6783: 523-542
<400> 6783
GACCGATAAT GGCAACGTGA TCCTCGACGT CCACGGCATG GAAATCCTTG 50
ACCCGATAGC GATGGAAAAC GCCATAAATG CGATTCCTGG CGTGGTGACT 100
<210> 6784
<211> 100
<212> DNA
<213> Escherichia coli K-12 MG1655

<220> rpia b2915
<222> 459-558
<223> rpia b2915 U00096 complement(3056686..3057345)
<313> 6784: 479-498
<400> 6784
TCCGGAATAC CGTCAGGGCG TGGTGACCGA TAATGGCAAC GTGATCCTCG 50
ACGTCCACGG CATGGAAATC CTTGACCCGA TAGCGATGGA AAACGCCATA 100
<210> 6785
<211> 100
<212> DNA
<213> Escherichia coli K-12 MG1655
<220> rpib b4091
<222> 351-450
<223> rpib b4091 U00096 4310929..4311378
<313> 6785: 417-436
<400> 6785
AAAAATGATT GTGGATGCGT GGCTGGGCGC ACAGTACGAA GGCGGTCGTC 50
ATCAACAACG CGTGGAGGCG ATTACGGCAA TAGAGCAGCG GAGAAATTGA 100
<210> 6786
<211> 100
<212> DNA
<213> Escherichia coli K-12 MG1655
<220> rpib b4091
<222> 336-435
<223> rpib b4091 U00096 4310929..4311378
<313> 6786: 361-380
<400> 6786
TGGCCTCGAA CTGGCAAAAA TGATTGTGGA TGCGTGGCTG GGCGCACAGT 50
ACGAAGGCGG TCGTCATCAA CAACGCGTGG AGGCGATTAC GGCAATAGAG 100
<210> 6787
<211> 100
<212> DNA
<213> Escherichia coli K-12 MG1655
<220> rpib b4091
<222> 264-363
<223> rpib b4091 U00096 4310929..4311378
<313> 6787: 310-329
<400> 6787
CGAACCTTAT TCCGCGCAAC TTTCGCGGCA GCATAACGAC ACCAACGTGC 50
TGGCTTTTGG TTCACGAGTG GTTGGCCTCG AACTGGCAAA AATGATTGTG 100
<210> 6788
<211> 100
<212> DNA
<213> Escherichia coli K-12 MG1655
<220> fuco b2800
<222> 1053-1152
<223> fuco b2800 U00096 complement(2929887..2931038)
<313> 6788: 1105-1124
<400> 6788
GGCACTGGCG CAGGCGGCAC TGGATGATGT TTGTACCGGT GGCAACCCGC 50
GTGAAGCAAC GCTTGAGGAT ATTGTAGAGC TTTACCATAC CGCCTGGTAA 100
<210> 6789
<211> 100
<212> DNA
<213> Escherichia coli K-12 MG1655
<220> fuco b2800
<222> 1008-1107
<223> fuco b2800 U00096 complement(2929887..2931038)
<313> 6789: 1067-1086
<400> 6789
TCCGCCACAT TTGCGTGATG TTGGTGTACG CAAGGAAGAC ATTCCGGCAC 50
TGGCGCAGGC GGCACTGGAT GATGTTTGTA CCGGTGGCAA CCCGCGTGAA 100
<210> 6790
<211> 100
<212> DNA
<213> Escherichia coli K-12 MG1655
<220> fuco b2800
<222> 923-1022
<223> fuco b2800 U00096 complement(2929887..2931038)
<313> 6790: 946-965
<400> 6790

GCGTGAAAGT GGAAGGTATG AGCCTGGAAG AGGCGCGTAA TGCCGCTGTT 50
GAAGCGGTGT TTGCTCTCAA CCGTGATGTC GGTATTCCGC CACATTTGCG 100
<210> 6791
<211> 100
<212> DNA
<213> Escherichia coli K-12 MG1655
<220> fucp b2802
<222> 1218-1317
<223> fucp b2802 U00096 2932257..2933573
<313> 6791: 1270-1289
<400> 6791
CGCGGCGGGC AACATCCCCA CTGCTGAACT GATCCCCGCA CTCTGCTTCG 50
CGGTCATCTT TATCTTTGCC CGTTTCCGTT CTCAAACGGC AACTAACTGA 100
<210> 6792
<211> 100
<212> DNA
<213> Escherichia coli K-12 MG1655
<220> fucp b2802
<222> 1190-1289
<223> fucp b2802 U00096 2932257..2933573
<313> 6792: 1229-1248
<400> 6792
TCACTCCGGT CATGGGTTTT GTCAGTGACG CGGCGGGCAA CATCCCCACT 50
GCTGAACTGA TCCCCGCACT CTGCTTCGCG GTCATCTTTA TCTTTGCCCG 100
<210> 6793
<211> 100
<212> DNA
<213> Escherichia coli K-12 MG1655
<220> fucp b2802
<222> 1118-1217
<223> fucp b2802 U00096 2932257..2933573
<313> 6793: 1132-1151
<400> 6793
TTAAGAATCT CGGCCAGGAC ACCAAATATG GTTCGTCCTT CATCGTTATG 50
ACCATTATTG GCGGCGGTAT TGTCACTCCG GTCATGGGTT TTGTCAGTGA 100
<210> 6794
<211> 100
<212> DNA
<213> Escherichia coli K-12 MG1655
<220> fucr b2806
<222> 633-732
<223> fucr b2806 U00096 2937390..2938121
<313> 6794: 689-708
<400> 6794
ATTTAATCGT TCAGGGGAAG CCCGCATCGG GCATCTGGAT GAGGTAACGC 50
ACATTATTTC TGATGAGCGC CAGGTTGCAA CTTCTTTGGT AACAGCCTGA 100
<210> 6795
<211> 100
<212> DNA
<213> Escherichia coli K-12 MG1655
<220> fucr b2806
<222> 548-647
<223> fucr b2806 U00096 2937390..2938121
<313> 6795: 593-612
<400> 6795
GGGACTCCAA TGCGATCAAC GCTGATTACA AATCGATGCT ATTAAAACGT 50
GCCGCGCAAT CGTTGTTATT GATTGATAAA AGTAAATTTA ATCGTTCAGG 100
<210> 6796
<211> 100
<212> DNA
<213> Escherichia coli K-12 MG1655
<220> fucr b2806
<222> 513-612
<223> fucr b2806 U00096 2937390..2938121
<313> 6796: 546-565
<400> 6796
TTCTTGTGAA GGGATCGATA GCAGCGGCGC ACTGTGGGAC TCCAATGCGA 50
TCAACGCTGA TTACAAATCG ATGCTATTAA AACGTGCCGC GCAATCGTTG 100
<210> 6797
<211> 100
<212> DNA

<213> Escherichia coli K-12 MG1655
<220> fucu b2805
<222> 287-386
<223> fucu b2805 U00096 2936910..2937332
<313> 6797: 319-338
<400> 6797
AAGCCCCGTG TCCTGACATC ATCCGCATCA ATCGTTTTGC GTTTTATGAA  50
CGGGCGCAAA AAGCCTTTGC GATCGTTATC ACAGGCGAAC GAGCGAAGTA  100
<210> 6798
<211> 100
<212> DNA
<213> Escherichia coli K-12 MG1655
<220> fucu b2805
<222> 221-320
<223> fucu b2805 U00096 2936910..2937332
<313> 6798: 265-284
<400> 6798
CGGCGGTAGA AGGTGACACT CTCGATCCTG AAGTAGAACG ACGTTACCGT  50
AATGCGCTTT CACTACAAGC CCCGTGTCCT GACATCATCC GCATCAATCG  100
<210> 6799
<211> 100
<212> DNA
<213> Escherichia coli K-12 MG1655
<220> fucu b2805
<222> 208-307
<223> fucu b2805 U00096 2936910..2937332
<313> 6799: 234-253
<400> 6799
CTGGTGATGA TGGCGGCGGT AGAAGGTGAC ACTCTCGATC CTGAAGTAGA  50
ACGACGTTAC CGTAATGCGC TTTCACTACA AGCCCCGTGT CCTGACATCA  100
<210> 6800
<211> 100
<212> DNA
<213> Escherichia coli K-12 MG1655
<220> ddla b0382
<222> 996-1095
<223> ddla b0382 U00096 complement(399053..400147)
<313> 6800: 1072-1091
<400> 6800
GCAAGCCAGC GGTCTGGGTT ACACCGATCT GATCACACGT TTGATTGAAC  50
TGGCGCTGGA GCGTCACGCT GCGGATAACG CATTGAAAAC CACAATGTAA  100
<210> 6801
<211> 100
<212> DNA
<213> Escherichia coli K-12 MG1655
<220> ddla b0382
<222> 967-1066
<223> ddla b0382 U00096 complement(399053..400147)
<313> 6801: 1004-1023
<400> 6801
ACTAATATCA GTATGTATCC GAAGCTGTGG CAAGCCAGCG GTCTGGGTTA  50
CACCGATCTG ATCACACGTT TGATTGAACT GGCGCTGGAG CGTCACGCTG  100
<210> 6802
<211> 100
<212> DNA
<213> Escherichia coli K-12 MG1655
<220> ddla b0382
<222> 900-999
<223> ddla b0382 U00096 complement(399053..400147)
<313> 6802: 950-969
<400> 6802
TGTAGACGTG TTTTTAACCC CAGAGAACGA AGTGGTGATC AACGAGATCA  50
ACACCCTGCC TGGCTTCACT AATATCAGTA TGTATCCGAA GCTGTGGCAA  100
<210> 6803
<211> 100
<212> DNA
<213> Escherichia coli K-12 MG1655
<220> ddlb b0093
<222> 822-921
<223> ddlb b0093 U00096 102233..103153
<313> 6803: 875-894

<400> 6803
ACCGGGTATG ACCAGCCACA GCCTGGTGCC GATGGCGGCA CGTCAGGCAG 50
GTATGAGCTT CTCGCAGTTG GTAGTACGAA TTCTGGAACT GGCGGACTAA 100
<210> 6804
<211> 100
<212> DNA
<213> Escherichia coli K-12 MG1655
<220> ddlb b0093
<222> 791-890
<223> ddlb b0093 U00096 102233..103153
<313> 6804: 825-844
<400> 6804
GACAGTTTTA TCTGCTGGAA GCCAATACCT CACCGGGTAT GACCAGCCAC 50
AGCCTGGTGC CGATGGCGGC ACGTCAGGCA GGTATGAGCT TCTCGCAGTT 100
<210> 6805
<211> 100
<212> DNA
<213> Escherichia coli K-12 MG1655
<220> ddlb b0093
<222> 721-820
<223> ddlb b0093 U00096 102233..103153
<313> 6805: 733-752
<400> 6805
GTGCTGAAAG CATGGACGAC GTTAGGTTGC AAAGGATGGG GACGTATTGA 50
CGTTATGCTG GACAGCGATG GACAGTTTTA TCTGCTGGAA GCCAATACCT 100
<210> 6806
<211> 100
<212> DNA
<213> Escherichia coli K-12 MG1655
<220> rpir b4090
<222> 796-895
<223> rpir b4090 U00096 complement(4309680..4310603)
<313> 6806: 831-850
<400> 6806
ATATTACAAC TAACTTTGCT GGACGCTTTT TTTGTCTCTG TCGCCCAGCT 50
CAACATTGAA CAAGCTAATA TTAATATGCA AAAAACCGGC GCAATTGTTG 100
<210> 6807
<211> 100
<212> DNA
<213> Escherichia coli K-12 MG1655
<220> rpir b4090
<222> 754-853
<223> rpir b4090 U00096 complement(4309680..4310603)
<313> 6807: 782-801
<400> 6807
GCCCCGGAAA CGCCGTTATT AGGTCGTAAT GCCTCGGCAA GAATATTACA 50
ACTAACTTTG CTGGACGCTT TTTTTGTCTC TGTCGCCCAG CTCAACATTG 100
<210> 6808
<211> 100
<212> DNA
<213> Escherichia coli K-12 MG1655
<220> rpir b4090
<222> 679-778
<223> rpir b4090 U00096 complement(4309680..4310603)
<313> 6808: 725-744
<400> 6808
GCAAAGATTA TTTGTATAAC CCATAGCTAC CATTCACCGA TAGCGAAACT 50
GGCCGATTAT ATTATTTGCT CACCAGCCCC GGAAACGCCG TTATTAGGTC 100
<210> 6809
<211> 100
<212> DNA
<213> Escherichia coli K-12 MG1655
<220> pnta pntA
<222> 1426-1525
<223> pnta pntA U00096 complement(1674395..1675927)
<313> 6809: 1473-1492
<400> 6809
GGCTGGGTTA GCTTCCTTAG TTTTATCGCG GTGCTTATAG CCAGCATTAA 50
TATTTTCGGT GGCTTCACCG TGACTCAGCG CATGCTGAAA ATGTTCCGCA 100
<210> 6810
<211> 100

<212> DNA
<213> Escherichia coli K-12 MG1655
<220> pnta pntA
<222> 1352-1451
<223> pnta pntA U00096 complement(1674395..1675927)
<313> 6810: 1391-1410
<400> 6810
CACCGTTGAT GTCGGTCACC AACGCGATTT CAGGGATTAT TGTTGTCGGA 50
GCACTGTTGC AGATTGGCCA GGGCGGCTGG GTTAGCTTCC TTAGTTTTAT 100
<210> 6811
<211> 100
<212> DNA
<213> Escherichia coli K-12 MG1655
<220> pnta pntA
<222> 1239-1338
<223> pnta pntA U00096 complement(1674395..1675927)
<313> 6811: 1298-1317
<400> 6811
TGGCTGGATG GCAAGCGTTG CGCCGAAAGA ATTCCTTGGG CACTTCACCG 50
TTTTCGCGCT GGCCTGCGTT GTCGGTTATT ACGTGGTGTG GAATGTATCG 100
<210> 6812
<211> 100
<212> DNA
<213> Escherichia coli K-12 MG1655
<220> pntb b1603
<222> 1290-1389
<223> pntb b1603 U00096 complement(1672996..1674384)
<313> 6812: 1370-1389
<400> 6812
CTATGCTGGT GTGCAAAACC CGCTGTTCTT CAAGGAAAAC ACCCACATGC 50
TGTTTGGTGA CGCCAAAGCC AGCGTGGATG CAATCCTGAA AGCTCTGTAA 100
<210> 6813
<211> 100
<212> DNA
<213> Escherichia coli K-12 MG1655
<220> pntb b1603
<222> 1282-1381
<223> pntb b1603 U00096 complement(1672996..1674384)
<313> 6813: 1311-1330
<400> 6813
AACACTGGCT ATGCTGGTGT GCAAAACCCG CTGTTCTTCA AGGAAAACAC 50
CCACATGCTG TTTGGTGACG CCAAAGCCAG CGTGGATGCA ATCCTGAAAG 100
<210> 6814
<211> 100
<212> DNA
<213> Escherichia coli K-12 MG1655
<220> pntb b1603
<222> 1189-1288
<223> pntb b1603 U00096 complement(1672996..1674384)
<313> 6814: 1250-1269
<400> 6814
GCGGCGCAGG ATGATCCGAA GAGTCCGATT GCTGGTATGC CTGTGCTGGA 50
AGTGTGGAAA GCGCAGAACG TGATTGTCTT TAAACGTTCG ATGAACACTG 100
<210> 6815
<211> 100
<212> DNA
<213> Escherichia coli K-12 MG1655
<220> b3836 b3836
<222> 125-224
<223> b3836 b3836 U00096 4019490..4019801
<313> 6815: 187-206
<400> 6815
TCGGTTCCGA TCTTGGTGCG TCGATCAAAG GCTTTAAAAA AGCAATGAGC 50
GATGATGAAC CAAAGCAGGA TAAAACCAGT CAGGATGCTG ATTTTACTGC 100
<210> 6816
<211> 100
<212> DNA
<213> Escherichia coli K-12 MG1655
<220> b3836 b3836
<222> 148-247
<223> b3836 b3836 U00096 4019490..4019801

<313> 6816: 164-183
<400> 6816
ATCAAAGGCT TTAAAAAAGC AATGAGCGAT GATGAACCAA AGCAGGATAA 50
AACCAGTCAG GATGCTGATT TTACTGCGAA AACTATCGCC GATAAGCAGG 100
<210> 6817
<211> 100
<212> DNA
<213> Escherichia coli K-12 MG1655
<220> b3836 b3836
<222> 115-214
<223> b3836 b3836 U00096 4019490..4019801
<313> 6817: 157-176
<400> 6817
CTCGGCTCCA TCGGTTCCGA TCTTGGTGCG TCGATCAAAG GCTTTAAAAA 50
AGCAATGAGC GATGATGAAC CAAAGCAGGA TAAAACCAGT CAGGATGCTG 100
<210> 6818
<211> 100
<212> DNA
<213> Escherichia coli K-12 MG1655
<220> b3837 b3837
<222> 121-220
<223> b3837 b3837 U00096 4019665..4020006
<313> 6818: 177-196
<400> 6818
ATAAAGAGCA GGTGTAATCC GTGTTTGATA TCGGTTTTAG CGAACTGCTA 50
TTGGTGTTCA TCATCGGCCT CGCCGCCTTC TGGGGCCGCA ACGACTGCCT 100
<210> 6819
<211> 100
<212> DNA
<213> Escherichia coli K-12 MG1655
<220> b3837 b3837
<222> 128-227
<223> b3837 b3837 U00096 4019665..4020006
<313> 6819: 162-181
<400> 6819
GCAGGTGTAA TCCGTGTTTG ATATCGGTTT TAGCGAACTG CTATTGGTGT 50
TCATCATCGG CCTCGCCGCC TTCTGGGGCC GCAACGACTG CCTGTGGCGG 100
<210> 6820
<211> 100
<212> DNA
<213> Escherichia coli K-12 MG1655
<220> b3837 b3837
<222> 75-174
<223> b3837 b3837 U00096 4019665..4020006
<313> 6820: 123-142
<400> 6820
GATACGAATC AGGAACAGGC TAAAACAGAA GACGCGAAGC GCCACGATAA 50
AGAGCAGGTG TAATCCGTGT TTGATATCGG TTTTAGCGAA CTGCTATTGG 100
<210> 6821
<211> 100
<212> DNA
<213> Escherichia coli K-12 MG1655
<220> b3838 b3839
<222> 339-438
<223> b3838 b3839 U00096 4019885..4020322
<313> 6821: 417-436
<400> 6821
ACAGAAGCCA GAAACCACGC CAGAGCCGGT GGTAAAACCT GCTGCGGACG 50
CTGAACCGAA AACCGCTGCA CCTTCCCCTT CGTCGAGTGA TAAACCGTAA 100
<210> 6822
<211> 100
<212> DNA
<213> Escherichia coli K-12 MG1655
<220> b3838 b3839
<222> 321-420
<223> b3838 b3839 U00096 4019885..4020322
<313> 6822: 364-383
<400> 6822
GCAGGCCAGT TCGCCGGAAC AGAAGCCAGA AACCACGCCA GAGCCGGTGG 50
TAAAACCTGC TGCGGACGCT GAACCGAAAA CCGCTGCACC TTCCCCTTCG 100
<210> 6823

<211> 100
<212> DNA
<213> Escherichia coli K-12 MG1655
<220> b3838 b3839
<222> 303-402
<223> b3838 b3839 U00096 4019885..4020322
<313> 6823: 323-342
<400> 6823
GCCTGCCGCT GCACAAACGC AGGCCAGTTC GCCGGAACAG AAGCCAGAAA 50
CCACGCCAGA GCCGGTGGTA AAACCTGCTG CGGACGCTGA ACCGAAAACC 100
<210> 6824
<211> 100
<212> DNA
<213> Escherichia coli K-12 MG1655
<220> tyra b2601
<222> 1023-1122
<223> tyra b2601 U00096 complement(2736968..2738089)
<313> 6824: 1087-1106
<400> 6824
CAGTTTCCGC AAGGTGGAGC ACTGGTTCGG CGATTACGCA CAGCGTTTTC 50
AGAGTGAAAG CCGCGTGTTA TTGCGTCAGG CGAATGACAA TCGCCAGTAA 100
<210> 6825
<211> 100
<212> DNA
<213> Escherichia coli K-12 MG1655
<220> tyra b2601
<222> 1012-1111
<223> tyra b2601 U00096 complement(2736968..2738089)
<313> 6825: 1057-1076
<400> 6825
GCGTTTATTG ACAGTTTCCG CAAGGTGGAG CACTGGTTCG GCGATTACGC 50
ACAGCGTTTT CAGAGTGAAA GCCGCGTGTT ATTGCGTCAG GCGAATGACA 100
<210> 6826
<211> 100
<212> DNA
<213> Escherichia coli K-12 MG1655
<220> tyra b2601
<222> 947-1046
<223> tyra b2601 U00096 complement(2736968..2738089)
<313> 6826: 989-1008
<400> 6826
TAATCAAACG TTACTATAAG CGTTTCGGCG AGGCGATTGA GTTGCTGGAG 50
CAGGGCGATA AGCAGGCGTT TATTGACAGT TTCCGCAAGG TGGAGCACTG 100
<210> 6827
<211> 100
<212> DNA
<213> Escherichia coli K-12 MG1655
<220> tyrb b4055
<222> 1095-1194
<223> tyrb b4055 U00096 4264693..4265886
<313> 6827: 1143-1162
<400> 6827
ATTTGGTGTC TATCTCATCG CCAGCGGTCG CATGTGTGTC GCCGGGTTAA 50
ATACGGCAAA TGTACAACGT GTGGCAAAGG CGTTTGCTGC GGTGATGTAA 100
<210> 6828
<211> 100
<212> DNA
<213> Escherichia coli K-12 MG1655
<220> tyrb b4055
<222> 1048-1147
<223> tyrb b4055 U00096 4264693..4265886
<313> 6828: 1087-1106
<400> 6828
AGTTATACCG GTTTAAGTGC CGCTCAGGTT GACCGACTAC GTGAAGAATT 50
TGGTGTCTAT CTCATCGCCA GCGGTCGCAT GTGTGTCGCC GGGTTAAATA 100
<210> 6829
<211> 100
<212> DNA
<213> Escherichia coli K-12 MG1655
<220> tyrb b4055
<222> 989-1088

<223> tyrb b4055 U00096 4264693..4265886
<313> 6829: 1026-1045
<400> 6829
TAAGCACAGA GATGCCAGAA CGCAATTTCG ATTATCTGCT TAATCAGCGC  50
GGCATGTTCA GTTATACCGG TTTAAGTGCC GCTCAGGTTG ACCGACTACG  100
<210> 6830
<211> 100
<212> DNA
<213> Escherichia coli K-12 MG1655
<220> exbb exbB
<222> 636-735
<223> exbb exbB U00096 complement(3149265..3149999)
<313> 6830: 700-719
<400> 6830
TGCAGCGCAG GTATTGTTGC TGCAAAGCCG TGACCTGGAT CTGGAAGCCA  50
GCGCCGCTGC GCATCCGGTT CGTGTCGCAC AAAAATTACG CGCAGGATAA  100
<210> 6831
<211> 100
<212> DNA
<213> Escherichia coli K-12 MG1655
<220> exbb exbB
<222> 442-541
<223> exbb exbB U00096 complement(3149265..3149999)
<313> 6831: 489-508
<400> 6831
ACGGTCTGGG GCATCATGAA CAGCTTTATT GGTATCGCGC AAACGCAGAC  50
CACTAACCTG GCAGTCGTTG CGCCGGGTAT CGCAGAAGCT CTGTTAGCAA  100
<210> 6832
<211> 100
<212> DNA
<213> Escherichia coli K-12 MG1655
<220> exbb exbB
<222> 392-491
<223> exbb exbB U00096 complement(3149265..3149999)
<313> 6832: 452-471
<400> 6832
GCTATCTGGC AACCATCGGC GCGATTTCTC CGTTCGTTGG TCTGTTTGGT  50
ACGGTCTGGG GCATCATGAA CAGCTTTATT GGTATCGCGC AAACGCAGAC  100
<210> 6833
<211> 100
<212> DNA
<213> Escherichia coli K-12 MG1655
<220> nema b1651
<222> 999-1098
<223> nema b1651 U00096 1724683..1725780
<313> 6833: 1066-1085
<400> 6833
GGTCGCCCGC TTGCAGCGCA AAGCTGAGCT TAACCCACAG CGTGCCGAAA  50
GTTTCTACGG TGGCGGCGCG GAAGGCTATA CCGATTACCC GACGTTGTAA  100
<210> 6834
<211> 100
<212> DNA
<213> Escherichia coli K-12 MG1655
<220> nema b1651
<222> 861-960
<223> nema b1651 U00096 1724683..1725780
<313> 6834: 911-930
<400> 6834
CGAAAAAGTA CGCGCCCGTT TCCACGGTCC GATTATCGGC GCAGGTGCAT  50
ACACAGTAGA AAAAGCTGAA ACGCTGATCG GCAAAGGGTT AATTGATGCG  100
<210> 6835
<211> 100
<212> DNA
<213> Escherichia coli K-12 MG1655
<220> nema b1651
<222> 809-908
<223> nema b1651 U00096 1724683..1725780
<313> 6835: 876-895
<400> 6835
ATATGTCAGA ACCAGATTGG GCGGGGGGTG AACCGTATAC TGATGCGTTC  50
CGCGAAAAAG TACGCGCCCG TTTCCACGGT CCGATTATCG GCGCAGGTGC  100

<210> 6836
<211> 100
<212> DNA
<213> Escherichia coli K-12 MG1655
<220> exbd b3006
<222> 309-408
<223> exbd b3006 U00096 complement(3148833..3149258)
<313> 6836: 360-379
<400> 6836
CTTCCGAGCG GACAAAACCG TCGATTACGA GACGTTGATG AAGGTAATGG 50
ATACGCTGCA TCAGGCGGGT TACCTGAAGA TAGGTCTGGT CGGCGAAGAA 100
<210> 6837
<211> 100
<212> DNA
<213> Escherichia coli K-12 MG1655
<220> exbd b3006
<222> 292-391
<223> exbd b3006 U00096 complement(3148833..3149258)
<313> 6837: 321-340
<400> 6837
AAAGACACCA CCATCTTCTT CCGAGCGGAC AAAACCGTCG ATTACGAGAC 50
GTTGATGAAG GTAATGGATA CGCTGCATCA GGCGGGTTAC CTGAAGATAG 100
<210> 6838
<211> 100
<212> DNA
<213> Escherichia coli K-12 MG1655
<220> exbd b3006
<222> 226-325
<223> exbd b3006 U00096 complement(3148833..3149258)
<313> 6838: 283-302
<400> 6838
ATCGGTAACG ATCCGGTCAC CGATGAAACA ATGATTACGG CGTTGAATGC 50
GTTAACCGAA GGCAAGAAAG ACACCACCAT CTTCTTCCGA GCGGACAAAA 100
<210> 6839
<211> 100
<212> DNA
<213> Escherichia coli K-12 MG1655
<220> tyrp b1908
<222> 1113-1212
<223> tyrp b1908 U00096 1987704..1988915
<313> 6839: 1180-1199
<400> 6839
AGGTGGTCGT CCGGCGCTGG TGGTGGTGTT TCTCTGTGGT ATTGCTGTGA 50
TTGGCGTGCA ATTTTTGATT GCGGCAGGGT TGTTACCAGA AGTGGGGTGA 100
<210> 6840
<211> 100
<212> DNA
<213> Escherichia coli K-12 MG1655
<220> tyrp b1908
<222> 1064-1163
<223> tyrp b1908 U00096 1987704..1988915
<313> 6840: 1086-1105
<400> 6840
TGACCTGGCA AAGCAGAAAG CACAATCCTC AGGCGGGTTA CCGGGTCAAA 50
GGTGGTCGTC CGGCGCTGGT GGTGGTGTTT CTCTGTGGTA TTGCTGTGAT 100
<210> 6841
<211> 100
<212> DNA
<213> Escherichia coli K-12 MG1655
<220> tyrp b1908
<222> 1001-1100
<223> tyrp b1908 U00096 1987704..1988915
<313> 6841: 1046-1065
<400> 6841
TGATGGCGCT GGGTTACGCC GGTGTGGCGC TGGCGGTACT GGCATTGATT 50
ATCCCTTCGC TGTTGACCTG GCAAAGCAGA AAGCACAATC CTCAGGCGGG 100
<210> 6842
<211> 100
<212> DNA
<213> Escherichia coli K-12 MG1655
<220> tyrr b1324

<222> 1443-1542
<223> tyrr b1324 U00096 1384744..1386285
<313> 6842: 1502-1521
<400> 6842
CAGCACGCGC AAACTGGCAA AACGTCTCGG CGTTTCACAT ACCGCGATTG  50
CCAATAAGTT GCGGGAATAT GGTCTGAGTC AGAAGAAGAA CGAAGAGTAA  100
<210> 6843
<211> 100
<212> DNA
<213> Escherichia coli K-12 MG1655
<220> tyrr b1324
<222> 1399-1498
<223> tyrr b1324 U00096 1384744..1386285
<313> 6843: 1438-1457
<400> 6843
CGTTTTGAAC GCTCGGTATT AACCCAGCTT TATCGCAATT ATCCCAGCAC  50
GCGCAAACTG GCAAAACGTC TCGGCGTTTC ACATACCGCG ATTGCCAATA  100
<210> 6844
<211> 100
<212> DNA
<213> Escherichia coli K-12 MG1655
<220> tyrr b1324
<222> 1352-1451
<223> tyrr b1324 U00096 1384744..1386285
<313> 6844: 1405-1424
<400> 6844
CCGTGGGCGA AGATGCGATG GAAGGTTCGC TGGACGAAAT CACCAGCCGT  50
TTTGAACGCT CGGTATTAAC CCAGCTTTAT CGCAATTATC CCAGCACGCG  100
<210> 6845
<211> 100
<212> DNA
<213> Escherichia coli K-12 MG1655
<220> tyrs b1638
<222> 1173-1272
<223> tyrs b1638 U00096 complement(1713972..1715246)
<313> 6845: 1218-1237
<400> 6845
GTCCGATCCT GAATACTTCT TTAAAGAAGA AGATCGTCTG TTTGGTCGTT  50
TTACCTTACT GCGTCGCGGT AAAAAGAATT ACTGTCTGAT TTGCTGGAAA  100
<210> 6846
<211> 100
<212> DNA
<213> Escherichia coli K-12 MG1655
<220> tyrs b1638
<222> 1103-1202
<223> tyrs b1638 U00096 complement(1713972..1715246)
<313> 6846: 1152-1171
<400> 6846
TGCAACCTTC CCGTGGTCAG GCACGTAAAA CTATCGCCTC CAATGCCATC  50
ACCATTAACG GTGAAAAACA GTCCGATCCT GAATACTTCT TTAAAGAAGA  100
<210> 6847
<211> 100
<212> DNA
<213> Escherichia coli K-12 MG1655
<220> tyrs b1638
<222> 1070-1169
<223> tyrs b1638 U00096 complement(1713972..1715246)
<313> 6847: 1122-1141
<400> 6847
CAGACCTGAT GCAGGCACTG GTCGATTCTG AACTGCAACC TTCCCGTGGT  50
CAGGCACGTA AAACTATCGC CTCCAATGCC ATCACCATTA ACGGTGAAAA  100
<210> 6848
<211> 100
<212> DNA
<213> Escherichia coli K-12 MG1655
<220> pnuc b0752
<222> 621-720
<223> pnuc b0752 U00096 782389..783108
<313> 6848: 654-673
<400> 6848
GTCTCTGGAG TACATCATCC TGACCTTTAT TGCGCTCAAC GGCAGCCGGA  50

TGTGGATCAA CAGCGCACGT GAAAGAGGCT CACGCGCGCT GTCCCATTAA 100
<210> 6849
<211> 100
<212> DNA
<213> Escherichia coli K-12 MG1655
<220> pnuc b0752
<222> 545-644
<223> pnuc b0752 U00096 782389..783108
<313> 6849: 597-616
<400> 6849
TGGAAAACTG GCTGTTGTGG GTGATTATTA ACGTGATTAG CGTCGTTATT 50
TTTGCACTTC AGGGCGTTTA CGCCATGTCT CTGGAGTACA TCATCCTGAC 100
<210> 6850
<211> 100
<212> DNA
<213> Escherichia coli K-12 MG1655
<220> pnuc b0752
<222> 427-526
<223> pnuc b0752 U00096 782389..783108
<313> 6850: 453-472
<400> 6850
CAAGCATTAG GATTACAGGT TGTGATGCCT GAACTGCAAC CGGACGCTTT 50
CCCGTTCTGG GATTCATGCA TGATGGTGTT ATCTATCGTG GCAATGATTC 100
<210> 6851
<211> 100
<212> DNA
<213> Escherichia coli K-12 MG1655
<220> bax b3571
<222> 726-825
<223> bax b3571 U00096 complement(3733982..3734806)
<313> 6851: 780-799
<400> 6851
TCACAAGCTG AAGGGCTACT CGACCAAAGG GAAGAGTTAT AACAACTACC 50
TGTTCGCAAT GTACCAGGAT AACCAACGGT TAATCGCGGC GCATATGTAA 100
<210> 6852
<211> 100
<212> DNA
<213> Escherichia coli K-12 MG1655
<220> bax b3571
<222> 684-783
<223> bax b3571 U00096 complement(3733982..3734806)
<313> 6852: 738-757
<400> 6852
GCTGCGTAAA GCGGATCAGG AAGTGACTGC CACAGCGATG ATTCACAAGC 50
TGAAGGGCTA CTCGACCAAA GGGAAGAGTT ATAACAACTA CCTGTTCGCA 100
<210> 6853
<211> 100
<212> DNA
<213> Escherichia coli K-12 MG1655
<220> bax b3571
<222> 636-735
<223> bax b3571 U00096 complement(3733982..3734806)
<313> 6853: 689-708
<400> 6853
CCTGAATACG CACCCGGCTT ACTCTTCGTT CCGTAAATCG CGTGCGCAGC 50
TGCGTAAAGC GGATCAGGAA GTGACTGCCA CAGCGATGAT TCACAAGCTG 100
<210> 6854
<211> 100
<212> DNA
<213> Escherichia coli K-12 MG1655
<220> hola b0641
<222> 933-1032
<223> hola b0641 U00096 complement(669797..670828)
<313> 6854: 990-1009
<400> 6854
CCTCAAACAA GATTACGGTC AGTCAGTGTG GGCAGAGCTG GAAGGGTTAT 50
CTCTTCTGTT GTGCCATAAA CCCCTGGCGG ACGTATTTAT CGACGGTTGA 100
<210> 6855
<211> 100
<212> DNA
<213> Escherichia coli K-12 MG1655

<220> hola b0641
<222> 931-1030
<223> hola b0641 U00096 complement(669797..670828)
<313> 6855: 961-980
<400> 6855
ACCCTCAAAC AAGATTACGG TCAGTCAGTG TGGGCAGAGC TGGAAGGGTT  50
ATCTCTTCTG TTGTGCCATA AACCCCTGGC GGACGTATTT ATCGACGGTT  100
<210> 6856
<211> 100
<212> DNA
<213> Escherichia coli K-12 MG1655
<220> hola b0641
<222> 886-985
<223> hola b0641 U00096 complement(669797..670828)
<313> 6856: 919-938
<400> 6856
ACGCAGTTAC GTCAGGCCGT GCAACTCCTG ACACGAACGG AACTCACCCT  50
CAAACAAGAT TACGGTCAGT CAGTGTGGGC AGAGCTGGAA GGGTTATCTC  100
<210> 6857
<211> 100
<212> DNA
<213> Escherichia coli K-12 MG1655
<220> holb b1100
<222> 904-1003
<223> holb b1100 U00096 1154985..1155989
<313> 6857: 953-972
<400> 6857
TCTGTTACAG GCATCAACCG CGAGCTTCTC ATCACCGATC TTTTACTGCG  50
TATTGAGCAT TACCTGCAAC CGGGCGTTGT GCTACCGGTT CCTCATCTTT  100
<210> 6858
<211> 100
<212> DNA
<213> Escherichia coli K-12 MG1655
<220> holb b1100
<222> 881-980
<223> holb b1100 U00096 1154985..1155989
<313> 6858: 913-932
<400> 6858
GCCACATTCG TGAACAGTTA ATGTCTGTTA CAGGCATCAA CCGCGAGCTT  50
CTCATCACCG ATCTTTTACT GCGTATTGAG CATTACCTGC AACCGGGCGT  100
<210> 6859
<211> 100
<212> DNA
<213> Escherichia coli K-12 MG1655
<220> holb b1100
<222> 803-902
<223> holb b1100 U00096 1154985..1155989
<313> 6859: 834-853
<400> 6859
TTGATGTGCC GGGCCTGGTC GCCGAACTGG CAAACCATCT TTCTCCCTCG  50
CGCCTGCAGG CTATACTGGG GGATGTTTGC CACATTCGTG AACAGTTAAT  100
<210> 6860
<211> 100
<212> DNA
<213> Escherichia coli K-12 MG1655
<220> holc b4260
<222> 345-444
<223> holc b4260 U00096 complement(4481405..4481848)
<313> 6860: 421-440
<400> 6860
CTTCGTTCCT TATGAAGATT CTCTGAAACA ACTGGCGCGC GAACGCTATA  50
AAGCCTACCG CGTGGCTGGT TTCAACCTGA ATACGGCAAC CTGGAAATAA  100
<210> 6861
<211> 100
<212> DNA
<213> Escherichia coli K-12 MG1655
<220> holc b4260
<222> 11-110
<223> holc b4260 U00096 complement(4481405..4481848)
<313> 6861: 58-77
<400> 6861

CGACGTTCTA CCTTCTGGAC AATGACACCA CCGTCGATGG CTTAAGCGCC 50
GTTGAGCAAC TGGTGTGTGA AATTGCCGCA GAACGTTGGC GCAGCGGTAA 100
<210> 6862
<211> 100
<212> DNA
<213> Escherichia coli K-12 MG1655
<220> holc b4260
<222> 1-100
<223> holc b4260 U00096 complement(4481405..4481848)
<313> 6862: 20-39
<400> 6862
ATGAAAAACG CGACGTTCTA CCTTCTGGAC AATGACACCA CCGTCGATGG 50
CTTAAGCGCC GTTGAGCAAC TGGTGTGTGA AATTGCCGCA GAACGTTGGC 100
<210> 6863
<211> 100
<212> DNA
<213> Escherichia coli K-12 MG1655
<220> hold b4373
<222> 315-414
<223> hold b4373 U00096 4605372..4605785
<313> 6863: 376-395
<400> 6863
ACCGGCGCTC ACCGATTTAC GGGCAAACCC AACGGCACGC GCCGCGTTAT 50
GGCAACAAAT TTGCACATAT GAACACGATT TCTTCCCTCG AAACGACTGA 100
<210> 6864
<211> 100
<212> DNA
<213> Escherichia coli K-12 MG1655
<220> hold b4373
<222> 291-390
<223> hold b4373 U00096 4605372..4605785
<313> 6864: 355-374
<400> 6864
ACTGGAAGGC GCTCAGGTGG CATCACCGGC GCTCACCGAT TTACGGGCAA 50
ACCCAACGGC ACGCGCCGCG TTATGGCAAC AAATTTGCAC ATATGAACAC 100
<210> 6865
<211> 100
<212> DNA
<213> Escherichia coli K-12 MG1655
<220> hold b4373
<222> 269-368
<223> hold b4373 U00096 4605372..4605785
<313> 6865: 305-324
<400> 6865
TGGGTACTGA CGAACCGCTA TCACTGGAAG GCGCTCAGGT GGCATCACCG 50
GCGCTCACCG ATTTACGGGC AAACCCAACG GCACGCGCCG CGTTATGGCA 100
<210> 6866
<211> 100
<212> DNA
<213> Escherichia coli K-12 MG1655
<220> hole holE
<222> 132-231
<223> hole holE U00096 1923132..1923362
<313> 6866: 201-220
<400> 6866
GCCTGAACAT TTGCGCAGCT GGTTTCGCGA GCGGCTTATT GCCCACCGTT 50
TGGCTTCGGT CAATCTGTCA CGTTTACCTT ACGAGCCCAA ACTTAAATAA 100
<210> 6867
<211> 100
<212> DNA
<213> Escherichia coli K-12 MG1655
<220> hole holE
<222> 132-231
<223> hole holE U00096 1923132..1923362
<313> 6867: 201-220
<400> 6867
GCCTGAACAT TTGCGCAGCT GGTTTCGCGA GCGGCTTATT GCCCACCGTT 50
TGGCTTCGGT CAATCTGTCA CGTTTACCTT ACGAGCCCAA ACTTAAATAA 100
<210> 6868
<211> 100
<212> DNA

<213> Escherichia coli K-12 MG1655
<220> hole holE
<222> 132-231
<223> hole holE U00096 1923132..1923362
<313> 6868: 174-193
<400> 6868
GCCTGAACAT TTGCGCAGCT GGTTTCGCGA GCGGCTTATT GCCCACCGTT 50
TGGCTTCGGT CAATCTGTCA CGTTTACCTT ACGAGCCCAA ACTTAAATAA 100
<210> 6869
<211> 100
<212> DNA
<213> Escherichia coli K-12 MG1655
<220> rpla b3985
<222> 555-654
<223> rpla b3985 U00096 4176457..4177161
<313> 6869: 600-619
<400> 6869
GAAAGAAAAC CTGGAAGCTC TGCTGGTTGC GCTGAAAAAA GCAAAACCGA 50
CTCAGGCGAA AGGCGTGTAC ATCAAGAAAG TTAGCATCTC CACCACCATG 100
<210> 6870
<211> 100
<212> DNA
<213> Escherichia coli K-12 MG1655
<220> rpla b3985
<222> 511-610
<223> rpla b3985 U00096 4176457..4177161
<313> 6870: 561-580
<400> 6870
ATCCACACCA CCATCGGTAA AGTGGACTTT GACGCTGACA AACTGAAAGA 50
AAACCTGGAA GCTCTGCTGG TTGCGCTGAA AAAAGCAAAA CCGACTCAGG 100
<210> 6871
<211> 100
<212> DNA
<213> Escherichia coli K-12 MG1655
<220> rpla b3985
<222> 479-578
<223> rpla b3985 U00096 4176457..4177161
<313> 6871: 511-530
<400> 6871
AGGTTCGTTA CCGTAACGAC AAAAACGGCA TCATCCACAC CACCATCGGT 50
AAAGTGGACT TTGACGCTGA CAAACTGAAA GAAAACCTGG AAGCTCTGCT 100
<210> 6872
<211> 100
<212> DNA
<213> Escherichia coli K-12 MG1655
<220> rplb b3318
<222> 723-822
<223> rplb b3318 U00096 complement(3448180..3449001)
<313> 6872: 784-803
<400> 6872
TAAGCACCCG GTAACTCCGT GGGGCGTTCA GACCAAAGGT AAGAAGACCC 50
GCAGCAACAA GCGTACTGAT AAATTCATCG TACGTCGCCG TAGCAAATAA 100
<210> 6873
<211> 100
<212> DNA
<213> Escherichia coli K-12 MG1655
<220> rplb b3318
<222> 681-780
<223> rplb b3318 U00096 complement(3448180..3449001)
<313> 6873: 718-737
<400> 6873
GGTAGACCAC CCACATGGTG GTGGTGAAGG TCGTAACTTT GGTAAGCACC 50
CGGTAACTCC GTGGGGCGTT CAGACCAAAG GTAAGAAGAC CCGCAGCAAC 100
<210> 6874
<211> 100
<212> DNA
<213> Escherichia coli K-12 MG1655
<220> rplb b3318
<222> 588-687
<223> rplb b3318 U00096 complement(3448180..3449001)
<313> 6874: 610-629

<400> 6874
CAATGCTGAG CATATGCTGC GCGTTCTGGG TAAAGCAGGT GCTGCACGCT  50
GGCGTGGTGT TCGTCCGACC GTTCGCGGTA CCGCGATGAA CCCGGTAGAC  100
<210> 6875
<211> 100
<212> DNA
<213> Escherichia coli K-12 MG1655
<220> rplc b3321
<222> 527-626
<223> rplc b3321 U00096 complement(3449934..3450563)
<313> 6875: 560-579
<400> 6875
ACGTAGTACG CGTTGACGCT GAGCGCAACC TGCTGCTGGT TAAAGGTGCT  50
GTCCCGGGTG CAACCGGTAG CGACCTGATC GTTAAACCAG CTGTGAAGGC  100
<210> 6876
<211> 100
<212> DNA
<213> Escherichia coli K-12 MG1655
<220> rplc b3321
<222> 453-552
<223> rplc b3321 U00096 complement(3449934..3450563)
<313> 6876: 500-519
<400> 6876
TCCGGGCAAA GTGTTCAAAG GCAAGAAAAT GGCAGGTCAG ATGGGTAACG  50
AACGTGTAAC CGTTCAGAGC CTTGACGTAG TACGCGTTGA CGCTGAGCGC  100
<210> 6877
<211> 100
<212> DNA
<213> Escherichia coli K-12 MG1655
<220> rplc b3321
<222> 407-506
<223> rplc b3321 U00096 complement(3449934..3450563)
<313> 6877: 465-484
<400> 6877
ACTCCTTGTC TCACCGCGTT CCGGGTTCTA TCGGTCAGAA CCAGACTCCG  50
GGCAAAGTGT TCAAAGGCAA GAAAATGGCA GGTCAGATGG GTAACGAACG  100
<210> 6878
<211> 100
<212> DNA
<213> Escherichia coli K-12 MG1655
<220> rpld b3320
<222> 507-606
<223> rpld b3320 U00096 complement(3449318..3449923)
<313> 6878: 565-584
<400> 6878
ACGCGATGCA ACTGGTATCG ACCCGGTTAG CCTGATCGCC TTCGACAAAG  50
TCGTAATGAC TGCTGATGCT GTTAAGCAAG TTGAGGAGAT GCTGGCATGA  100
<210> 6879
<211> 100
<212> DNA
<213> Escherichia coli K-12 MG1655
<220> rpld b3320
<222> 377-476
<223> rpld b3320 U00096 complement(3449318..3449923)
<313> 6879: 424-443
<400> 6879
TAGAAGCGCC GAAAACTAAG CTGCTGGCAC AGAAACTGAA AGACATGGCT  50
CTGGAAGATG TGCTGATCAT CACCGGTGAG CTGGACGAAA ACCTGTTCCT  100
<210> 6880
<211> 100
<212> DNA
<213> Escherichia coli K-12 MG1655
<220> rpld b3320
<222> 301-400
<223> rpld b3320 U00096 complement(3449318..3449923)
<313> 6880: 341-360
<400> 6880
TACCGCGGCG CGCTGAAAAG CATCCTGTCC GAACTGGTAC GTCAGGATCG  50
TCTGATCGTT GTCGAGAAGT TCTCTGTAGA AGCGCCGAAA ACTAAGCTGC  100
<210> 6881
<211> 100

<212> DNA
<213> Escherichia coli K-12 MG1655
<220> rple b3309
<222> 441-540
<223> rple b3309 U00096 complement(3444536..3445075)
<313> 6881: 514-533
<400> 6881
CCGCGTTCGT GGTTTGGATA TTACCATTAC CACTACTGCG AAATCTGACG 50
AAGAAGGCCG CGCTCTGCTG GCTGCCTTTG ACTTCCCGTT CCGCAAGTAA 100
<210> 6882
<211> 100
<212> DNA
<213> Escherichia coli K-12 MG1655
<220> rple b3309
<222> 417-516
<223> rple b3309 U00096 complement(3444536..3445075)
<313> 6882: 474-493
<400> 6882
AGAAATCGAC TACGATAAAG TCGACCGCGT TCGTGGTTTG GATATTACCA 50
TTACCACTAC TGCGAAATCT GACGAAGAAG GCCGCGCTCT GCTGGCTGCC 100
<210> 6883
<211> 100
<212> DNA
<213> Escherichia coli K-12 MG1655
<220> rple b3309
<222> 367-466
<223> rple b3309 U00096 complement(3444536..3445075)
<313> 6883: 412-431
<400> 6883
GACGGTCGTG GTAACTACAG CATGGGTGTC CGTGAGCAGA TCATCTTCCC 50
AGAAATCGAC TACGATAAAG TCGACCGCGT TCGTGGTTTG GATATTACCA 100
<210> 6884
<211> 100
<212> DNA
<213> Escherichia coli K-12 MG1655
<220> ntpa b1866
<222> 354-453
<223> ntpa b1866 U00096 complement(1946204..1946656)
<313> 6884: 407-426
<400> 6884
TCTGGCTTAC AAGTGGCTTG ATGCGCCTGC TGCGGCGGCG CTCACTAAGT 50
CCTGGAGCAA CCGGCAGGCG ATTGAACAGT TTGTAATTAA CGCTGCCTGA 100
<210> 6885
<211> 100
<212> DNA
<213> Escherichia coli K-12 MG1655
<220> ntpa b1866
<222> 326-425
<223> ntpa b1866 U00096 complement(1946204..1946656)
<313> 6885: 349-368
<400> 6885
ACGAGCGGCA GATCGTTTTC ACTGAACATC TGGCTTACAA GTGGCTTGAT 50
GCGCCTGCTG CGGCGGCGCT CACTAAGTCC TGGAGCAACC GGCAGGCGAT 100
<210> 6886
<211> 100
<212> DNA
<213> Escherichia coli K-12 MG1655
<220> ntpa b1866
<222> 293-392
<223> ntpa b1866 U00096 complement(1946204..1946656)
<313> 6886: 322-341
<400> 6886
ATACGGAATC ATGGTTCTGT CTTGCGCTTC CGCACGAGCG GCAGATCGTT 50
TTCACTGAAC ATCTGGCTTA CAAGTGGCTT GATGCGCCTG CTGCGGCGGC 100
<210> 6887
<211> 100
<212> DNA
<213> Escherichia coli K-12 MG1655
<220> rplf b3306
<222> 435-534
<223> rplf b3306 U00096 complement(3443244..3443777)

<313> 6887: 482-501
<400> 6887
AGCGGATCTG CGCGCCTACC GTCGTCCTGA GCCTTATAAA GGCAAGGGTG 50
TTCGTTACGC CGACGAAGTC GTGCGTACCA AAGAGGCTAA GAAGAAGTAA 100
<210> 6888
<211> 100
<212> DNA
<213> Escherichia coli K-12 MG1655
<220> rplf b3306
<222> 354-453
<223> rplf b3306 U00096 complement(3443244..3443777)
<313> 6888: 398-417
<400> 6888
TGCGGGTATC ACTGCTGAAT GTCCGACTCA GACTGAAATC GTGCTGAAAG 50
GCGCTGATAA GCAGGTGATC GGCCAGGTTG CAGCGGATCT GCGCGCCTAC 100
<210> 6889
<211> 100
<212> DNA
<213> Escherichia coli K-12 MG1655
<220> rplf b3306
<222> 276-375
<223> rplf b3306 U00096 complement(3443244..3443777)
<313> 6889: 312-331
<400> 6889
AGGTTACCGT GCAGCGGTTA AAGGCAATGT GATTAACCTG TCTCTGGGTT 50
TCTCTCATCC TGTTGACCAT CAGCTGCCTG CGGGTATCAC TGCTGAATGT 100
<210> 6890
<211> 100
<212> DNA
<213> Escherichia coli K-12 MG1655
<220> rpli b4204
<222> 324-423
<223> rpli b4204 U00096 4423686..4424135
<313> 6890: 363-382
<400> 6890
TGAAGTGGCT AAGAGCGAAG TTCGTCTGCC GAACGGCGTT CTGCGTACCA 50
CTGGCGAACA CGAAGTGAGC TTCCAGGTTC ACAGCGAAGT ATTCGCGAAA 100
<210> 6891
<211> 100
<212> DNA
<213> Escherichia coli K-12 MG1655
<220> rpli b4204
<222> 284-383
<223> rpli b4204 U00096 4423686..4424135
<313> 6891: 335-354
<400> 6891
GTACTCGCGA CATCGCTGAC GCTGTAACTG CAGCTGGCGT TGAAGTGGCT 50
AAGAGCGAAG TTCGTCTGCC GAACGGCGTT CTGCGTACCA CTGGCGAACA 100
<210> 6892
<211> 100
<212> DNA
<213> Escherichia coli K-12 MG1655
<220> rpli b4204
<222> 209-308
<223> rpli b4204 U00096 4423686..4424135
<313> 6892: 252-271
<400> 6892
AGAAAATCAA TGCACTGGAA ACTGTTACCA TCGCGTCTAA AGCTGGCGAC 50
GAAGGTAAAC TGTTCGGTTC CATCGGTACT CGCGACATCG CTGACGCTGT 100
<210> 6893
<211> 100
<212> DNA
<213> Escherichia coli K-12 MG1655
<220> rplj b3986
<222> 399-498
<223> rplj b3986 U00096 4177574..4178071
<313> 6893: 474-493
<400> 6893
AGAAGCAATT GCACGCCTGA TGGCAACCAT GAAAGAAGCT TCGGCTGGCA 50
AACTGGTTCG TACTCTGGCT GCTGTACGCG ATGCGAAAGA AGCTGCTTAA 100
<210> 6894

<211> 100
<212> DNA
<213> Escherichia coli K-12 MG1655
<220> rplj b3986
<222> 335-434
<223> rplj b3986 U00096 4177574..4178071
<313> 6894: 370-389
<400> 6894
CCTTTGAAGG TGAGCTGATC CCGGCGTCTC AGATCGACCG CCTGGCAACT 50
CTGCCGACCT ACGAAGAAGC AATTGCACGC CTGATGGCAA CCATGAAAGA 100
<210> 6895
<211> 100
<212> DNA
<213> Escherichia coli K-12 MG1655
<220> rplj b3986
<222> 278-377
<223> rplj b3986 U00096 4177574..4178071
<313> 6895: 304-323
<400> 6895
CTCGTCTGTT CAAAGAGTTC GCGAAAGCGA ATGCAAAATT TGAGGTCAAA 50
GCCGCTGCCT TTGAAGGTGA GCTGATCCCG GCGTCTCAGA TCGACCGCCT 100
<210> 6896
<211> 100
<212> DNA
<213> Escherichia coli K-12 MG1655
<220> rplk b3984
<222> 330-429
<223> rplk b3984 U00096 4176025..4176453
<313> 6896: 370-389
<400> 6896
GCAGACCAAA GCTGCCGACA TGACTGGTGC CGACATTGAA GCGATGACTC 50
GCTCCATCGA AGGTACTGCA CGTTCCATGG GCCTGGTAGT GGAGGACTAA 100
<210> 6897
<211> 100
<212> DNA
<213> Escherichia coli K-12 MG1655
<220> rplk b3984
<222> 277-376
<223> rplk b3984 U00096 4176025..4176453
<313> 6897: 333-352
<400> 6897
CCGAACAAAG ACAAAGTGGG TAAAATTTCC CGCGCTCAGC TGCAGGAAAT 50
CGCGCAGACC AAAGCTGCCG ACATGACTGG TGCCGACATT GAAGCGATGA 100
<210> 6898
<211> 100
<212> DNA
<213> Escherichia coli K-12 MG1655
<220> rplk b3984
<222> 257-356
<223> rplk b3984 U00096 4176025..4176453
<313> 6898: 297-316
<400> 6898
TCAAGTCTGG TTCCGGTAAG CCGAACAAAG ACAAAGTGGG TAAAATTTCC 50
CGCGCTCAGC TGCAGGAAAT CGCGCAGACC AAAGCTGCCG ACATGACTGG 100
<210> 6899
<211> 100
<212> DNA
<213> Escherichia coli K-12 MG1655
<220> rpll b3987
<222> 266-365
<223> rpll b3987 U00096 4178138..4178503
<313> 6899: 321-340
<400> 6899
AATCTGCACC GGCTGCTCTG AAAGAAGGCG TGAGCAAAGA CGACGCAGAA 50
GCACTGAAAA AAGCTCTGGA AGAAGCTGGC GCTGAAGTTG AAGTTAAATA 100
<210> 6900
<211> 100
<212> DNA
<213> Escherichia coli K-12 MG1655
<220> rpll b3987
<222> 242-341

<223> rpll b3987 U00096 4178138..4178503
<313> 6900: 277-296
<400> 6900
TGAAAGAAGC TAAAGACCTG GTAGAATCTG CACCGGCTGC TCTGAAAGAA  50
GGCGTGAGCA AAGACGACGC AGAAGCACTG AAAAAAGCTC TGGAAGAAGC  100
<210> 6901
<211> 100
<212> DNA
<213> Escherichia coli K-12 MG1655
<220> rpll b3987
<222> 186-285
<223> rpll b3987 U00096 4178138..4178503
<313> 6901: 214-233
<400> 6901
TGGCGCTAAC AAAGTTGCTG TTATCAAAGC AGTACGTGGC GCAACTGGCC  50
TGGGTCTGAA AGAAGCTAAA GACCTGGTAG AATCTGCACC GGCTGCTCTG  100
<210> 6902
<211> 100
<212> DNA
<213> Escherichia coli K-12 MG1655
<220> rplm b3232
<222> 330-429
<223> rplm b3232 U00096 complement(3375858..3376286)
<313> 6902: 395-414
<400> 6902
AAAAGGCCCG CTGGGTCGTG CTATGTTCCG TAAACTGAAA GTTTACGCGG  50
GTAACGAGCA CAACCACGCG GCACAGCAAC CGCAAGTTCT TGACATCTAA  100
<210> 6903
<211> 100
<212> DNA
<213> Escherichia coli K-12 MG1655
<220> rplm b3232
<222> 320-419
<223> rplm b3232 U00096 complement(3375858..3376286)
<313> 6903: 334-353
<400> 6903
GCATGTTGCC AAAAGGCCCG CTGGGTCGTG CTATGTTCCG TAAACTGAAA  50
GTTTACGCGG GTAACGAGCA CAACCACGCG GCACAGCAAC CGCAAGTTCT  100
<210> 6904
<211> 100
<212> DNA
<213> Escherichia coli K-12 MG1655
<220> rplm b3232
<222> 241-340
<223> rplm b3232 U00096 complement(3375858..3376286)
<313> 6904: 275-294
<400> 6904
ATCGGTGGTA TCAAACAAGC GACCTTTGAA GAGATGATTG CTCGCCGTCC  50
TGAGCGTGTG ATTGAAATCG CGGTTAAAGG CATGTTGCCA AAAGGCCCGC  100
<210> 6905
<211> 100
<212> DNA
<213> Escherichia coli K-12 MG1655
<220> rpln b3311
<222> 214-313
<223> rpln b3311 U00096 complement(3445415..3445786)
<313> 6905: 249-268
<400> 6905
CCGGACGGTT CTGTCATTCG CTTCGATGGT AATGCTTGTG TTCTTCTGAA  50
CAACAACAGC GAGCAGCCTA TCGGTACGCG TATTTTTGGG CCGGTAACTC  100
<210> 6906
<211> 100
<212> DNA
<213> Escherichia coli K-12 MG1655
<220> rpln b3311
<222> 194-293
<223> rpln b3311 U00096 complement(3445415..3445786)
<313> 6906: 210-229
<400> 6906
CCAAGAAGGG TGTTCGTCGC CCGGACGGTT CTGTCATTCG CTTCGATGGT  50
AATGCTTGTG TTCTTCTGAA CAACAACAGC GAGCAGCCTA TCGGTACGCG  100

<210> 6907
<211> 100
<212> DNA
<213> Escherichia coli K-12 MG1655
<220> rpln b3311
<222> 135-234
<223> rpln b3311 U00096 complement(3445415..3445786)
<313> 6907: 157-176
<400> 6907
AGCAATTCCG CGTGGTAAGG TCAAAAAAGG TGATGTGCTG AAGGCGGTAG 50
TGGTGCGCAC CAAGAAGGGT GTTCGTCGCC CGGACGGTTC TGTCATTCGC 100
<210> 6908
<211> 100
<212> DNA
<213> Escherichia coli K-12 MG1655
<220> rplo b3302
<222> 336-435
<223> rplo b3302 U00096 complement(3441742..3442176)
<313> 6908: 385-404
<400> 6908
GGCTGGCGAA GTAACGACTC CGGTAACTGT TCGTGGCCTG CGTGTTACTA 50
AAGGCGCTCG TGCTGCTATC GAAGCTGCTG GCGGTAAAAT CGAGGAATAA 100
<210> 6909
<211> 100
<212> DNA
<213> Escherichia coli K-12 MG1655
<220> rplo b3302
<222> 295-394
<223> rplo b3302 U00096 complement(3441742..3442176)
<313> 6909: 343-362
<400> 6909
AACATTATCG GTATCCAGAT CGAGTTCGCG AAAGTGATCC TGGCTGGCGA 50
AGTAACGACT CCGGTAACTG TTCGTGGCCT GCGTGTTACT AAAGGCGCTC 100
<210> 6910
<211> 100
<212> DNA
<213> Escherichia coli K-12 MG1655
<220> rplo b3302
<222> 244-343
<223> rplo b3302 U00096 complement(3441742..3442176)
<313> 6910: 275-294
<400> 6910
CTGGCTAAAG TAGAAGGCGG TGTAGTAGAC CTGAACACGC TGAAAGCGGC 50
TAACATTATC GGTATCCAGA TCGAGTTCGC GAAAGTGATC CTGGCTGGCG 100
<210> 6911
<211> 100
<212> DNA
<213> Escherichia coli K-12 MG1655
<220> rplp b3314
<222> 312-411
<223> rplp b3314 U00096 complement(3446396..3446806)
<313> 6911: 362-381
<400> 6911
AATGGACGGT GTTCCGGAAG AGCTGGCCCG TGAAGCATTC AAGCTGGCAG 50
CAGCGAAACT GCCGATTAAA ACCACCTTTG TAACTAAGAC GGTGATGTAA 100
<210> 6912
<211> 100
<212> DNA
<213> Escherichia coli K-12 MG1655
<220> rplp b3314
<222> 269-368
<223> rplp b3314 U00096 complement(3446396..3446806)
<313> 6912: 290-309
<400> 6912
AGTATTGGGT TGCCTTGATT CAGCCGGGTA AAGTCCTGTA TGAAATGGAC 50
GGTGTTCCGG AAGAGCTGGC CCGTGAAGCA TTCAAGCTGG CAGCAGCGAA 100
<210> 6913
<211> 100
<212> DNA
<213> Escherichia coli K-12 MG1655
<220> rplp b3314

<222> 195-294
<223> rplp b3314 U00096 complement(3446396..3446806)
<313> 6913: 244-263
<400> 6913
CCGTGTGTTC CCGGACAAAC CGATCACTGA AAAGCCGCTG GCAGTGCGTA 50
TGGGTAAAGG TAAAGGTAAC GTGGAGTATT GGGTTGCCTT GATTCAGCCG 100
<210> 6914
<211> 100
<212> DNA
<213> Escherichia coli K-12 MG1655
<220> rplq b3295
<222> 269-368
<223> rplq b3295 U00096 complement(3437253..3437636)
<313> 6914: 312-331
<400> 6914
GTGCCGGTGG TTACACTCGT ATTCTGAAGT GTGGCTTCCG TGCAGGCGAC 50
AACGCGCCGA TGGCTTACAT CGAGCTGGTT GATCGTTCAG AGAAAGCAGA 100
<210> 6915
<211> 100
<212> DNA
<213> Escherichia coli K-12 MG1655
<220> rplq b3295
<222> 167-266
<223> rplq b3295 U00096 complement(3437253..3437636)
<313> 6915: 216-235
<400> 6915
AGACTGATAG CGTTGCTAAT CGTCGTCTGG CATTCGCCCG TACTCGTGAT 50
AACGAGATCG TGGCAAAACT GTTTAACGAA CTGGGCCCGC GTTTCGCGAG 100
<210> 6916
<211> 100
<212> DNA
<213> Escherichia coli K-12 MG1655
<220> rplq b3295
<222> 129-228
<223> rplq b3295 U00096 complement(3437253..3437636)
<313> 6916: 188-207
<400> 6916
GCTGCGCCGC GTAGTTGAGC CGCTGATTAC TCTTGCCAAG ACTGATAGCG 50
TTGCTAATCG TCGTCTGGCA TTCGCCCGTA CTCGTGATAA CGAGATCGTG 100
<210> 6917
<211> 100
<212> DNA
<213> Escherichia coli K-12 MG1655
<220> rplr b3305
<222> 255-354
<223> rplr b3305 U00096 complement(3442881..3443234)
<313> 6917: 329-348
<400> 6917
AGGCATCAAA GATGTATCCT TTGACCGTTC CGGGTTCCAA TATCATGGTC 50
GTGTCCAGGC ACTGGCAGAT GCTGCCCGTG AAGCTGGCCT TCAGTTCTAA 100
<210> 6918
<211> 100
<212> DNA
<213> Escherichia coli K-12 MG1655
<220> rplr b3305
<222> 199-298
<223> rplr b3305 U00096 complement(3442881..3443234)
<313> 6918: 248-267
<400> 6918
AACAAAGACG CGGCTGCAGC TGTGGGTAAA GCTGTCGCTG AACGCGCTCT 50
GGAAAAAGGC ATCAAAGATG TATCCTTTGA CCGTTCCGGG TTCCAATATC 100
<210> 6919
<211> 100
<212> DNA
<213> Escherichia coli K-12 MG1655
<220> rplr b3305
<222> 46-145
<223> rplr b3305 U00096 complement(3442881..3443234)
<313> 6919: 86-105
<400> 6919
CGCAAGCTCC AGGAGCTGGG CGCAACTCGC CTGGTGGTAC ATCGTACCCC 50

GCGTCACATT TACGCACAGG TAATTGCACC GAACGGTTCT GAAGTTCTGG 100
<210> 6920
<211> 100
<212> DNA
<213> Escherichia coli K-12 MG1655
<220> rpls b2607
<222> 249-348
<223> rpls b2607 U00096 complement(2742203..2742550)
<313> 6920: 289-308
<400> 6920
CATTTCTGTC AAACGTCGTG GTGCTGTTCG TAAAGCTAAA CTGTACTACC 50
TGCGTGAGCG TACTGGTAAG GCTGCTCGTA TCAAAGAGCG TCTTAACTAA 100
<210> 6921
<211> 100
<212> DNA
<213> Escherichia coli K-12 MG1655
<220> rpls b2607
<222> 202-301
<223> rpls b2607 U00096 complement(2742203..2742550)
<313> 6921: 229-248
<400> 6921
GAAGGCGTTG AGCGTGTCTT CCAGACTCAC TCTCCGGTAG TTGACAGCAT 50
TTCTGTCAAA CGTCGTGGTG CTGTTCGTAA AGCTAAACTG TACTACCTGC 100
<210> 6922
<211> 100
<212> DNA
<213> Escherichia coli K-12 MG1655
<220> rpls b2607
<222> 150-249
<223> rpls b2607 U00096 complement(2742203..2742550)
<313> 6922: 183-202
<400> 6922
TCGTAACCGC GGTCTGCACT CTGCATTCAC TGTTCGTAAA ATTTCCAACG 50
GCGAAGGCGT TGAGCGTGTC TTCCAGACTC ACTCTCCGGT AGTTGACAGC 100
<210> 6923
<211> 100
<212> DNA
<213> Escherichia coli K-12 MG1655
<220> rplt b1717
<222> 258-357
<223> rplt b1717 U00096 complement(1797417..1797773)
<313> 6923: 329-348
<400> 6923
CTCTGTTGAA ATCGACCGTA AGATCCTGGC TGATATCGCA GTATTCGACA 50
AAGTAGCGTT CACCGCTCTG GTTGAAAAAG CGAAAGCAGC TCTGGCATAA 100
<210> 6924
<211> 100
<212> DNA
<213> Escherichia coli K-12 MG1655
<220> rplt b1717
<222> 242-341
<223> rplt b1717 U00096 complement(1797417..1797773)
<313> 6924: 257-276
<400> 6924
ATGGCCTGAA AAAAGCCTCT GTTGAAATCG ACCGTAAGAT CCTGGCTGAT 50
ATCGCAGTAT TCGACAAAGT AGCGTTCACC GCTCTGGTTG AAAAAGCGAA 100
<210> 6925
<211> 100
<212> DNA
<213> Escherichia coli K-12 MG1655
<220> rplt b1717
<222> 164-263
<223> rplt b1717 U00096 complement(1797417..1797773)
<313> 6925: 218-237
<400> 6925
GTCAGTTCCG TCAACTGTGG ATTGCGCGTA TCAACGCAGC AGCACGTCAG 50
AACGGTATTT CTTACAGCAA ATTCATCAAT GGCCTGAAAA AAGCCTCTGT 100
<210> 6926
<211> 100
<212> DNA
<213> Escherichia coli K-12 MG1655

<220> rplu b3187
<222> 213-312
<223> rplu b3187 U00096 complement(3330781..3331092)
<313> 6926: 258-277
<400> 6926
AGTTAAAATC GTTAAGTTTC GTCGTCGTAA ACACTATCGT AAGCAGCAGG 50
GCCATCGTCA GTGGTTCACT GATGTGAAAA TTACTGGCAT CAGCGCCTAA 100
<210> 6927
<211> 100
<212> DNA
<213> Escherichia coli K-12 MG1655
<220> rplu b3187
<222> 148-247
<223> rplu b3187 U00096 complement(3330781..3331092)
<313> 6927: 170-189
<400> 6927
GGCGTTCCTT TCGTTGATGG CGGCGTAATC AAAGCTGAAG TTGTTGCTCA 50
CGGTCGTGGC GAGAAAGTTA AAATCGTTAA GTTTCGTCGT CGTAAACACT 100
<210> 6928
<211> 100
<212> DNA
<213> Escherichia coli K-12 MG1655
<220> rplu b3187
<222> 94-193
<223> rplu b3187 U00096 complement(3330781..3331092)
<313> 6928: 125-144
<400> 6928
ACTGTTGAGT TCGCTGAAGT GCTGATGATC GCAAACGGTG AAGAAGTCAA 50
AATCGGCGTT CCTTTCGTTG ATGGCGGCGT AATCAAAGCT GAAGTTGTTG 100
<210> 6929
<211> 100
<212> DNA
<213> Escherichia coli K-12 MG1655
<220> rplv b3316
<222> 234-333
<223> rplv b3316 U00096 complement(3447538..3447870)
<313> 6929: 293-312
<400> 6929
AGGCCCGAGC ATGAAGCGCA TTATGCCGCG TGCAAAAGGT CGTGCAGATC 50
GCATCCTGAA GCGCACCAGC CACATCACTG TGGTTGTGTC CGATCGCTGA 100
<210> 6930
<211> 100
<212> DNA
<213> Escherichia coli K-12 MG1655
<220> rplv b3316
<222> 187-286
<223> rplv b3316 U00096 complement(3447538..3447870)
<313> 6930: 247-266
<400> 6930
GGCGCTGACA TTGACGATCT GAAAGTTACG AAAATTTTCG TAGACGAAGG 50
CCCGAGCATG AAGCGCATTA TGCCGCGTGC AAAAGGTCGT GCAGATCGCA 100
<210> 6931
<211> 100
<212> DNA
<213> Escherichia coli K-12 MG1655
<220> rplv b3316
<222> 70-169
<223> rplv b3316 U00096 complement(3447538..3447870)
<313> 6931: 100-119
<400> 6931
ATTCGCGGTA AGAAAGTGTC GCAGGCTCTG GATATTTTGA CCTACACCAA 50
CAAGAAAGCG GCTGTACTGG TCAAGAAAGT TCTGGAATCT GCCATTGCTA 100
<210> 6932
<211> 100
<212> DNA
<213> Escherichia coli K-12 MG1655
<220> rplw b3319
<222> 204-303
<223> rplw b3319 U00096 complement(3449019..3449321)
<313> 6932: 257-276
<400> 6932

ACGTCACGGA CAGCGTATCG GTCGTCGTAG CGACTGGAAA AAAGCTTACG 50
TCACCCTGAA AGAAGGCCAG AATCTGGACT TCGTTGGCGG CGCTGAGTAA 100
<210> 6933
<211> 100
<212> DNA
<213> Escherichia coli K-12 MG1655
<220> rplw b3319
<222> 175-274
<223> rplw b3319 U00096 complement(3449019..3449321)
<313> 6933: 212-231
<400> 6933
AACACCCTGG TAGTTAAAGG GAAAGTTAAA CGTCACGGAC AGCGTATCGG 50
TCGTCGTAGC GACTGGAAAA AAGCTTACGT CACCCTGAAA GAAGGCCAGA 100
<210> 6934
<211> 100
<212> DNA
<213> Escherichia coli K-12 MG1655
<220> rplw b3319
<222> 103-202
<223> rplw b3319 U00096 complement(3449019..3449321)
<313> 6934: 163-182
<400> 6934
GCTAAAGACG CGACCAAAGC AGAAATCAAA GCTGCTGTGC AGAAACTGTT 50
TGAAGTCGAA GTCGAAGTCG TTAACACCCT GGTAGTTAAA GGGAAAGTTA 100
<210> 6935
<211> 100
<212> DNA
<213> Escherichia coli K-12 MG1655
<220> rplx b3310
<222> 210-309
<223> rplx b3310 U00096 complement(3445090..3445404)
<313> 6935: 252-271
<400> 6935
AGCAATCTTC AATGCGGCAA CCGGCAAGGC TGACCGTGTA GGCTTTAGAT 50
TCGAAGACGG TAAAAAAGTC CGTTTCTTCA AGTCTAACAG CGAAACTATC 100
<210> 6936
<211> 100
<212> DNA
<213> Escherichia coli K-12 MG1655
<220> rplx b3310
<222> 172-271
<223> rplx b3310 U00096 complement(3445090..3445404)
<313> 6936: 213-232
<400> 6936
ATCGTTGAAA AAGAAGCCGC TATTCAGGTT TCCAACGTAG CAATCTTCAA 50
TGCGGCAACC GGCAAGGCTG ACCGTGTAGG CTTTAGATTC GAAGACGGTA 100
<210> 6937
<211> 100
<212> DNA
<213> Escherichia coli K-12 MG1655
<220> rplx b3310
<222> 134-233
<223> rplx b3310 U00096 complement(3445090..3445404)
<313> 6937: 159-178
<400> 6937
ATCAGAAGCC GGTTCCGGCC CTGAACCAAC CGGGTGGCAT CGTTGAAAAA 50
GAAGCCGCTA TTCAGGTTTC CAACGTAGCA ATCTTCAATG CGGCAACCGG 100
<210> 6938
<211> 100
<212> DNA
<213> Escherichia coli K-12 MG1655
<220> rply b2186
<222> 186-285
<223> rply b2186 U00096 2280537..2280821
<313> 6938: 239-258
<400> 6938
CATCGTTGTT GACGGTAAAG AAATCAAAGT TAAAGCTCAG GACGTACAGC 50
GTCACCCGTA CAAACCGAAG CTGCAGCACA TCGACTTCGT TCGCGCTTAA 100
<210> 6939
<211> 100
<212> DNA

<213> Escherichia coli K-12 MG1655
<220> rply b2186
<222> 109-208
<223> rply b2186 U00096 2280537..2280821
<313> 6939: 169-188
<400> 6939
CCGCTGGCTA TCGAGCTGGA TCACGACAAA GTCATGAACA TGCAAGCTAA 50
AGCTGAATTC TACAGCGAAG TTCTGACCAT CGTTGTTGAC GGTAAAGAAA 100
<210> 6940
<211> 100
<212> DNA
<213> Escherichia coli K-12 MG1655
<220> rply b2186
<222> 74-173
<223> rply b2186 U00096 2280537..2280821
<313> 6940: 110-129
<400> 6940
AGTTCCCGGC AATCATCTAC GGTGGCAAAG AAGCGCCGCT GGCTATCGAG 50
CTGGATCACG ACAAAGTCAT GAACATGCAA GCTAAAGCTG AATTCTACAG 100
<210> 6941
<211> 100
<212> DNA
<213> Escherichia coli K-12 MG1655
<220> rpma b3186
<222> 159-258
<223> rpma b3186 U00096 complement(3330503..3330760)
<313> 6941: 204-223
<400> 6941
CGGTCGTGAC CACACTCTGT TTGCTAAAGC AGACGGTAAA GTGAAATTCG 50
AAGTTAAAGG CCCGAAAAAC CGTAAATTTA TCAGCATCGA AGCTGAATAA 100
<210> 6942
<211> 100
<212> DNA
<213> Escherichia coli K-12 MG1655
<220> rpma b3186
<222> 159-258
<223> rpma b3186 U00096 complement(3330503..3330760)
<313> 6942: 204-223
<400> 6942
CGGTCGTGAC CACACTCTGT TTGCTAAAGC AGACGGTAAA GTGAAATTCG 50
AAGTTAAAGG CCCGAAAAAC CGTAAATTTA TCAGCATCGA AGCTGAATAA 100
<210> 6943
<211> 100
<212> DNA
<213> Escherichia coli K-12 MG1655
<220> rpma b3186
<222> 30-129
<223> rpma b3186 U00096 complement(3330503..3330760)
<313> 6943: 95-114
<400> 6943
ACGTAACGGT CGCGATTCAG AAGCTAAACG CCTGGGCGTT AAGCGTTTCG 50
GTGGCGAATC CGTTCTGGCG GGTAGCATCA TCGTTCGTCA ACGTGGTACC 100
<210> 6944
<211> 100
<212> DNA
<213> Escherichia coli K-12 MG1655
<220> smpa smpA
<222> 32-131
<223> smpa smpA U00096 2751815..2751967
<313> 6944: 86-105
<400> 6944
TCTATGTCTT CCGCCAGCAA CCAGGTCATG AAGGTGTAAC TCAGCAAACG 50
CTGACGCTGA CCTTTAACAG TAGCGGTGTG TTGACCAATA TTGATAACAA 100
<210> 6945
<211> 100
<212> DNA
<213> Escherichia coli K-12 MG1655
<220> smpa smpA
<222> 54-153
<223> smpa smpA U00096 2751815..2751967
<313> 6945: 79-98

<400> 6945
AGGTCATGAA GGTGTAACTC AGCAAACGCT GACGCTGACC TTTAACAGTA 50
GCGGTGTGTT GACCAATATT GATAACAAAC CTGCGCTGAG TGGTAACTAA 100
<210> 6946
<211> 100
<212> DNA
<213> Escherichia coli K-12 MG1655
<220> smpa smpA
<222> 14-113
<223> smpa smpA U00096 2751815..2751967
<313> 6946: 67-86
<400> 6946
TTGGTACGAA TACCTGGTTC TATGTCTTCC GCCAGCAACC AGGTCATGAA 50
GGTGTAACTC AGCAAACGCT GACGCTGACC TTTAACAGTA GCGGTGTGTT 100
<210> 6947
<211> 100
<212> DNA
<213> Escherichia coli K-12 MG1655
<220> rpmb b3638
<222> 138-237
<223> rpmb b3638 U00096 complement(3809065..3809301)
<313> 6947: 199-218
<400> 6947
TGTCACCCTG CGCGTATCTG CTAAAGGTAT GCGTGTAATC GATAAAAAAG 50
GCATCGATAC AGTTCTGGCT GAACTGCGTG CCCGTGGCGA AAAGTACTAA 100
<210> 6948
<211> 100
<212> DNA
<213> Escherichia coli K-12 MG1655
<220> rpmb b3638
<222> 38-137
<223> rpmb b3638 U00096 complement(3809065..3809301)
<313> 6948: 84-103
<400> 6948
TGACCGGTAA CAACCGTTCC CACGCACTGA ACGCGACTAA ACGCCGTTTC 50
CTGCCGAACC TGCACTCTCA CCGTTTCTGG GTTGAGAGCG AGAAGCGTTT 100
<210> 6949
<211> 100
<212> DNA
<213> Escherichia coli K-12 MG1655
<220> rpmb b3638
<222> 11-110
<223> rpmb b3638 U00096 complement(3809065..3809301)
<313> 6949: 49-68
<400> 6949
TCTGCCAAGT TACTGGCAAG CGTCCGGTGA CCGGTAACAA CCGTTCCCAC 50
GCACTGAACG CGACTAAACG CCGTTTCCTG CCGAACCTGC ACTCTCACCG 100
<210> 6950
<211> 100
<212> DNA
<213> Escherichia coli K-12 MG1655
<220> smpb b2621
<222> 384-483
<223> smpb b2621 U00096 2752917..2753399
<313> 6950: 447-466
<400> 6950
CGTCGCCAAA GGTAAGAAAC AGCACGATAA ACGTTCAGAT ATCAAAGAGC 50
GCGAATGGCA GGTGGATAAA GCACGTATCA TGAAAAACGC CCACCGTTAA 100
<210> 6951
<211> 100
<212> DNA
<213> Escherichia coli K-12 MG1655
<220> smpb b2621
<222> 374-473
<223> smpb b2621 U00096 2752917..2753399
<313> 6951: 410-429
<400> 6951
TGAAAATCGG CGTCGCCAAA GGTAAGAAAC AGCACGATAA ACGTTCAGAT 50
ATCAAAGAGC GCGAATGGCA GGTGGATAAA GCACGTATCA TGAAAAACGC 100
<210> 6952
<211> 100

<212> DNA
<213> Escherichia coli K-12 MG1655
<220> smpb b2621
<222> 310-409
<223> smpb b2621 U00096 2752917..2753399
<313> 6952: 345-364
<400> 6952
GTCAATCGAG AAGGCTATAC CGTAGTGGCG CTCTCCCTGT ACTGGAAAAA 50
TGCCTGGTGC AAAGTGAAAA TCGGCGTCGC CAAAGGTAAG AAACAGCACG 100
<210> 6953
<211> 100
<212> DNA
<213> Escherichia coli K-12 MG1655
<220> rpmc b3313
<222> 15-114
<223> rpmc b3313 U00096 complement(3446205..3446396)
<313> 6953: 46-65
<400> 6953
GCTGCGTGAG AAGAGCGTTG AAGAGCTGAA CACCGAGCTG CTGAACCTGC 50
TGCGTGAGCA GTTCAACCTG CGTATGCAGG CTGCAAGTGG CCAGCTGCAA 100
<210> 6954
<211> 100
<212> DNA
<213> Escherichia coli K-12 MG1655
<220> rpmc b3313
<222> 13-112
<223> rpmc b3313 U00096 complement(3446205..3446396)
<313> 6954: 44-63
<400> 6954
GAGCTGCGTG AGAAGAGCGT TGAAGAGCTG AACACCGAGC TGCTGAACCT 50
GCTGCGTGAG CAGTTCAACC TGCGTATGCA GGCTGCAAGT GGCCAGCTGC 100
<210> 6955
<211> 100
<212> DNA
<213> Escherichia coli K-12 MG1655
<220> rpmc b3313
<222> 1-100
<223> rpmc b3313 U00096 complement(3446205..3446396)
<313> 6955: 21-40
<400> 6955
ATGAAAGCAA AAGAGCTGCG TGAGAAGAGC GTTGAAGAGC TGAACACCGA 50
GCTGCTGAAC CTGCTGCGTG AGCAGTTCAA CCTGCGTATG CAGGCTGCAA 100
<210> 6956
<211> 100
<212> DNA
<213> Escherichia coli K-12 MG1655
<220> rpmd b3303
<222> 81-180
<223> rpmd b3303 U00096 complement(3442180..3442359)
<313> 6956: 153-172
<400> 6956
GGGTCTGCGT CGTATTGGTC ACACCGTAGA GCGCGAGGAT ACTCCTGCTA 50
TTCGCGGTAT GATCAACGCG GTTTCCTTCA TGGTTAAAGT TGAGGAGTAA 100
<210> 6957
<211> 100
<212> DNA
<213> Escherichia coli K-12 MG1655
<220> rpmd b3303
<222> 70-169
<223> rpmd b3303 U00096 complement(3442180..3442359)
<313> 6957: 104-123
<400> 6957
CTGCTTGGCC TGGGTCTGCG TCGTATTGGT CACACCGTAG AGCGCGAGGA 50
TACTCCTGCT ATTCGCGGTA TGATCAACGC GGTTTCCTTC ATGGTTAAAG 100
<210> 6958
<211> 100
<212> DNA
<213> Escherichia coli K-12 MG1655
<220> rpmd b3303
<222> 19-118
<223> rpmd b3303 U00096 complement(3442180..3442359)

<313> 6958: 53-72
<400> 6958
ATTACTCAAA CCCGCAGTGC AATCGGTCGT CTGCCGAAAC ACAAGGCAAC 50
GCTGCTTGGC CTGGGTCTGC GTCGTATTGG TCACACCGTA GAGCGCGAGG 100
<210> 6959
<211> 100
<212> DNA
<213> Escherichia coli K-12 MG1655
<220> rpme b3937
<222> 67-166
<223> rpme b3937 U00096 4124593..4124805
<313> 6959: 118-137
<400> 6959
AAAATCCGCT CCACCGTTGG TCATGACCTG AACCTCGACG TGTGCAGCAA 50
GTGCCACCCG TTCTTCACTG GCAAACAGCG TGATGTTGCT ACCGGTGGCC 100
<210> 6960
<211> 100
<212> DNA
<213> Escherichia coli K-12 MG1655
<220> rpme b3937
<222> 81-180
<223> rpme b3937 U00096 4124593..4124805
<313> 6960: 119-138
<400> 6960
CGTTGGTCAT GACCTGAACC TCGACGTGTG CAGCAAGTGC CACCCGTTCT 50
TCACTGGCAA ACAGCGTGAT GTTGCTACCG GTGGCCGTGT TGACCGCTTC 100
<210> 6961
<211> 100
<212> DNA
<213> Escherichia coli K-12 MG1655
<220> rpme b3937
<222> 78-177
<223> rpme b3937 U00096 4124593..4124805
<313> 6961: 94-113
<400> 6961
CACCGTTGGT CATGACCTGA ACCTCGACGT GTGCAGCAAG TGCCACCCGT 50
TCTTCACTGG CAAACAGCGT GATGTTGCTA CCGGTGGCCG TGTTGACCGC 100
<210> 6962
<211> 100
<212> DNA
<213> Escherichia coli K-12 MG1655
<220> rpmf b1090
<222> 75-174
<223> rpmf b1090 U00096 1146590..1146763
<313> 6962: 118-137
<400> 6962
CACCAGCCTG TCTGTAGACA AAACTTCTGG TGAAAAACAC CTGCGTCACC 50
ACATCACTGC CGACGGTTAC TACCGCGGCC GCAAGGTCAT CGCTAAGTAA 100
<210> 6963
<211> 100
<212> DNA
<213> Escherichia coli K-12 MG1655
<220> rpmf b1090
<222> 12-111
<223> rpmf b1090 U00096 1146590..1146763
<313> 6963: 69-88
<400> 6963
ACAGAATAAA CCAACCCGTT CCAAACGTGG CATGCGTCGT TCCCATGACG 50
CGCTGACCGC AGTCACCAGC CTGTCTGTAG ACAAAACTTC TGGTGAAAAA 100
<210> 6964
<211> 100
<212> DNA
<213> Escherichia coli K-12 MG1655
<220> rpmf b1090
<222> 1-100
<223> rpmf b1090 U00096 1146590..1146763
<313> 6964: 12-31
<400> 6964
ATGGCCGTAC AACAGAATAA ACCAACCCGT TCCAAACGTG GCATGCGTCG 50
TTCCCATGAC GCGCTGACCG CAGTCACCAG CCTGTCTGTA GACAAAACTT 100
<210> 6965

<211> 100
<212> DNA
<213> Escherichia coli K-12 MG1655
<220> bcp b2481
<222> 361-460
<223> bcp b2481 U00096 2598498..2598968
<313> 6965: 403-422
<400> 6965
CGCATCAGCT TCCTGATTGA CGCTGATGGC AAAATCGAAC ATGTCTTTGA 50
CGATTTCAAA ACCAGCAATC ACCACGACGT TGTGCTGAAC TGGCTGAAAG 100
<210> 6966
<211> 100
<212> DNA
<213> Escherichia coli K-12 MG1655
<220> bcp b2481
<222> 303-402
<223> bcp b2481 U00096 2598498..2598968
<313> 6966: 364-383
<400> 6966
ACAATTCGGC GTCTGGGGTG AAAAGTCCTT CATGGGCAAA ACCTACGATG 50
GCATTCATCG CATCAGCTTC CTGATTGACG CTGATGGCAA AATCGAACAT 100
<210> 6967
<211> 100
<212> DNA
<213> Escherichia coli K-12 MG1655
<220> bcp b2481
<222> 269-368
<223> bcp b2481 U00096 2598498..2598968
<313> 6967: 320-339
<400> 6967
CGCTCCTGTC TGATGAGGAC CACCAGGTGT GCGAACAATT CGGCGTCTGG 50
GGTGAAAAGT CCTTCATGGG CAAAACCTAC GATGGCATTC ATCGCATCAG 100
<210> 6968
<211> 100
<212> DNA
<213> Escherichia coli K-12 MG1655
<220> rpmg b3637
<222> 69-168
<223> rpmg b3637 U00096 complement(3808877..3809044)
<313> 6968: 103-122
<400> 6968
TACGAAGAAC AAACGTACTA AGCCGGAAAA ACTGGAACTG AAAAAATTCG 50
ATCCAGTTGT TCGCCAGCAC GTGATCTACA AAGAAGCGAA AATCAAATAA 100
<210> 6969
<211> 100
<212> DNA
<213> Escherichia coli K-12 MG1655
<220> rpmg b3637
<222> 66-165
<223> rpmg b3637 U00096 complement(3808877..3809044)
<313> 6969: 89-108
<400> 6969
CACTACGAAG AACAAACGTA CTAAGCCGGA AAAACTGGAA CTGAAAAAAT 50
TCGATCCAGT TGTTCGCCAG CACGTGATCT ACAAAGAAGC GAAAATCAAA 100
<210> 6970
<211> 100
<212> DNA
<213> Escherichia coli K-12 MG1655
<220> rpmg b3637
<222> 44-143
<223> rpmg b3637 U00096 complement(3808877..3809044)
<313> 6970: 78-97
<400> 6970
CTGGTACTGG TCACTTCTAT ACCACTACGA AGAACAAACG TACTAAGCCG 50
GAAAAACTGG AACTGAAAAA ATTCGATCCA GTTGTTCGCC AGCACGTGAT 100
<210> 6971
<211> 100
<212> DNA
<213> Escherichia coli K-12 MG1655
<220> rpmh b3704
<222> 42-141

<223> rpmh b3704 U00096 3881965..3882105
<313> 6971: 111-130
<400> 6971
TTCTCACGGC TTCCGTGCTC GTATGGCTAC TAAAAATGGT CGTCAGGTTC  50
TGGCACGTCG TCGTGCTAAA GGCCGCGCTC GTCTGACCGT TTCTAAGTAA  100
<210> 6972
<211> 100
<212> DNA
<213> Escherichia coli K-12 MG1655
<220> rpmh b3704
<222> 39-138
<223> rpmh b3704 U00096 3881965..3882105
<313> 6972: 74-93
<400> 6972
CCGTTCTCAC GGCTTCCGTG CTCGTATGGC TACTAAAAAT GGTCGTCAGG  50
TTCTGGCACG TCGTCGTGCT AAAGGCCGCG CTCGTCTGAC CGTTTCTAAG  100
<210> 6973
<211> 100
<212> DNA
<213> Escherichia coli K-12 MG1655
<220> rpmh b3704
<222> 1-100
<223> rpmh b3704 U00096 3881965..3882105
<313> 6973: 27-46
<400> 6973
ATGAAACGCA CTTTTCAACC GTCTGTACTG AAGCGCAACC GTTCTCACGG  50
CTTCCGTGCT CGTATGGCTA CTAAAAATGG TCGTCAGGTT CTGGCACGTC  100
<210> 6974
<211> 100
<212> DNA
<213> Escherichia coli K-12 MG1655
<220> bcr b2183
<222> 1090-1189
<223> bcr b2183 U00096 complement(2276590..2277780)
<313> 6974: 1142-1161
<400> 6974
GCGACCTTTA ACTCTGCATG GCCGATGATT TGGTCAATTG CATTCTGCGC  50
AACCAGCTCC ATTCTCTTCT GTCTGTACGC CAGTCGGCCG AAAAAACGGT  100
<210> 6975
<211> 100
<212> DNA
<213> Escherichia coli K-12 MG1655
<220> bcr b2183
<222> 1060-1159
<223> bcr b2183 U00096 complement(2276590..2277780)
<313> 6975: 1096-1115
<400> 6975
GGGGCAATTG TTGGCGCATT GCTTTCTCTT GCGACCTTTA ACTCTGCATG  50
GCCGATGATT TGGTCAATTG CATTCTGCGC AACCAGCTCC ATTCTCTTCT  100
<210> 6976
<211> 100
<212> DNA
<213> Escherichia coli K-12 MG1655
<220> bcr b2183
<222> 988-1087
<223> bcr b2183 U00096 complement(2276590..2277780)
<313> 6976: 1039-1058
<400> 6976
GTCATTCTTG ATGAGTTTCC CCATATGGCG GGAACGGCAT CTTCGCTGGC  50
AGGAACCTTC CGTTTTGGCA TAGGGGCAAT TGTTGGCGCA TTGCTTTCTC  100
<210> 6977
<211> 100
<212> DNA
<213> Escherichia coli K-12 MG1655
<220> rpmi b1718
<222> 92-191
<223> rpmi b1718 U00096 complement(1797826..1798023)
<313> 6977: 112-131
<400> 6977
ACATTCTGAC CAAAAAAGCG ACCAAACGTA AACGTCACCT GCGTCCGAAA  50
GCCATGGTTT CCAAAGGCGA TCTGGGCCTG GTAATCGCGT GCCTGCCGTA  100

<210> 6978
<211> 100
<212> DNA
<213> Escherichia coli K-12 MG1655
<220> rpmi b1718
<222> 77-176
<223> rpmi b1718 U00096 complement(1797826..1798023)
<313> 6978: 98-117
<400> 6978
ACGCTAACCT GCGTCACATT CTGACCAAAA AAGCGACCAA ACGTAAACGT 50
CACCTGCGTC CGAAAGCCAT GGTTTCCAAA GGCGATCTGG GCCTGGTAAT 100
<210> 6979
<211> 100
<212> DNA
<213> Escherichia coli K-12 MG1655
<220> rpmi b1718
<222> 59-158
<223> rpmi b1718 U00096 complement(1797826..1798023)
<313> 6979: 99-118
<400> 6979
GTGGTTTTAA GCACAAGCAC GCTAACCTGC GTCACATTCT GACCAAAAAA 50
GCGACCAAAC GTAAACGTCA CCTGCGTCCG AAAGCCATGG TTTCCAAAGG 100
<210> 6980
<211> 100
<212> DNA
<213> Escherichia coli K-12 MG1655
<220> rpmj b3300
<222> 18-117
<223> rpmj b3300 U00096 complement(3440255..3440371)
<313> 6980: 92-111
<400> 6980
CGTCAAGAAA TTATGCCGTA ACTGCAAAAT CGTTAAGCGT GATGGTGTCA 50
TCCGTGTGAT TTGCAGTGCC GAGCCGAAGC ATAAACAGCG CCAAGGCTGA 100
<210> 6981
<211> 100
<212> DNA
<213> Escherichia coli K-12 MG1655
<220> rpmj b3300
<222> 18-117
<223> rpmj b3300 U00096 complement(3440255..3440371)
<313> 6981: 86-105
<400> 6981
CGTCAAGAAA TTATGCCGTA ACTGCAAAAT CGTTAAGCGT GATGGTGTCA 50
TCCGTGTGAT TTGCAGTGCC GAGCCGAAGC ATAAACAGCG CCAAGGCTGA 100
<210> 6982
<211> 100
<212> DNA
<213> Escherichia coli K-12 MG1655
<220> rpmj b3300
<222> 1-100
<223> rpmj b3300 U00096 complement(3440255..3440371)
<313> 6982: 18-37
<400> 6982
ATGAAAGTTC GTGCTTCCGT CAAGAAATTA TGCCGTAACT GCAAAATCGT 50
TAAGCGTGAT GGTGTCATCC GTGTGATTTG CAGTGCCGAG CCGAAGCATA 100
<210> 6983
<211> 100
<212> DNA
<213> Escherichia coli K-12 MG1655
<220> dsrb dsrB
<222> 90-189
<223> dsrb dsrB U00096 complement(2022657..2022845)
<313> 6983: 137-156
<400> 6983
CACAATGTAC CTGGTTTCGC TGGAAGACTA CCCGCTCGGC ATCTGGTTCT 50
TTAATGAAGC AGGGCATCAG GACGGTATCT TTGTGGAGAA AGCAGAGTAA 100
<210> 6984
<211> 100
<212> DNA
<213> Escherichia coli K-12 MG1655
<220> dsrb dsrB

<222> 68-167
<223> dsrb dsrB U00096 complement(2022657..2022845)
<313> 6984: 104-123
<400> 6984
CAGTTGAGGA GTTTAGTGAA GGCACAATGT ACCTGGTTTC GCTGGAAGAC 50
TACCCGCTCG GCATCTGGTT CTTTAATGAA GCAGGGCATC AGGACGGTAT 100
<210> 6985
<211> 100
<212> DNA
<213> Escherichia coli K-12 MG1655
<220> dsrb dsrB
<222> 41-140
<223> dsrb dsrB U00096 complement(2022657..2022845)
<313> 6985: 68-87
<400> 6985
GTCCGCGTCG TCCTGGCGTG GTACTGGCAG TTGAGGAGTT TAGTGAAGGC 50
ACAATGTACC TGGTTTCGCT GGAAGACTAC CCGCTCGGCA TCTGGTTCTT 100
<210> 6986
<211> 100
<212> DNA
<213> Escherichia coli K-12 MG1655
<220> cvpa b2314
<222> 390-489
<223> cvpa b2314 U00096 complement(2428295..2428783)
<313> 6986: 465-484
<400> 6986
AGACTGGAGC AAATCACAGC TGATCCCGCA ATTCAGTTTT ATCATCAGAT 50
GTTTTTTTGA TTATCTGCAA AGCTCGTCAA GTTTCTTGCC CAGAGCGTAA 100
<210> 6987
<211> 100
<212> DNA
<213> Escherichia coli K-12 MG1655
<220> cvpa b2314
<222> 321-420
<223> cvpa b2314 U00096 complement(2428295..2428783)
<313> 6987: 390-409
<400> 6987
GCGCGGTGTG TTGATTGTTG CTGCCATTCT CTTCTTTCTC GACTCCTTTA 50
CCGGGGTGTC GAAAAGCGAA GACTGGAGCA AATCACAGCT GATCCCGCAA 100
<210> 6988
<211> 100
<212> DNA
<213> Escherichia coli K-12 MG1655
<220> cvpa b2314
<222> 280-379
<223> cvpa b2314 U00096 complement(2428295..2428783)
<313> 6988: 349-368
<400> 6988
TCAGGCACCG ATCGGGTGCT GGGCGTCTGT TTCGGTGCGT TGCGCGGTGT 50
GTTGATTGTT GCTGCCATTC TCTTCTTTCT CGACTCCTTT ACCGGGGTGT 100
<210> 6989
<211> 100
<212> DNA
<213> Escherichia coli K-12 MG1655
<220> trea b1198
<222> 1599-1698
<223> trea b1198 U00096 complement(1244902..1246599)
<313> 6989: 1675-1694
<400> 6989
CCCGAAAGAG CAACCGTGTG ACAATGTTCC GGCGACGCGT CCGACCGTTA 50
AGTCAGCAAC GACGCAACCC TCAACCAAAG AGGCACAACC CACACCTTAA 100
<210> 6990
<211> 100
<212> DNA
<213> Escherichia coli K-12 MG1655
<220> trea b1198
<222> 1558-1657
<223> trea b1198 U00096 complement(1244902..1246599)
<313> 6990: 1611-1630
<400> 6990
TGGACCAATG GCGTGACGCT GAAAATGCTG GATTTGATCT GCCCGAAAGA 50

GCAACCGTGT GACAATGTTC CGGCGACGCG TCCGACCGTT AAGTCAGCAA 100
<210> 6991
<211> 100
<212> DNA
<213> Escherichia coli K-12 MG1655
<220> trea b1198
<222> 1541-1640
<223> trea b1198 U00096 complement(1244902..1246599)
<313> 6991: 1570-1589
<400> 6991
TACAGGATGG CTTTGGCTGG ACCAATGGCG TGACGCTGAA AATGCTGGAT 50
TTGATCTGCC CGAAAGAGCA ACCGTGTGAC AATGTTCCGG CGACGCGTCC 100
<210> 6992
<211> 100
<212> DNA
<213> Escherichia coli K-12 MG1655
<220> treb b4241
<222> 1320-1419
<223> treb b4241 U00096 complement(4462333..4463754)
<313> 6992: 1375-1394
<400> 6992
GGTGTTTGCG CTGGCAATGG CTATCGCCAT CATCATCCCG ATTGTACTCA 50
CCTCGTTTAT CTATCAGCGG AAATACCGCC TGGGCACGCT GGACATTGTT 100
<210> 6993
<211> 100
<212> DNA
<213> Escherichia coli K-12 MG1655
<220> treb b4241
<222> 1178-1277
<223> treb b4241 U00096 complement(4462333..4463754)
<313> 6993: 1228-1247
<400> 6993
TGAAATATCG CTTCCCGATG CTGTGCGCGA TGATTGGTTC TGGTCTGGCA 50
GGATTGCTAT GCGGCCTGAA CGGCGTTATG GCGAATGGCA TCGGCGTAGG 100
<210> 6994
<211> 100
<212> DNA
<213> Escherichia coli K-12 MG1655
<220> treb b4241
<222> 1112-1211
<223> treb b4241 U00096 complement(4462333..4463754)
<313> 6994: 1173-1192
<400> 6994
TCTCCGTGCC TGCCGCTATC TCCGCCTGGC TTGGGGTCAC TGAGCCTGCA 50
ATGTACGGCA TCAACCTGAA ATATCGCTTC CCGATGCTGT GCGCGATGAT 100
<210> 6995
<211> 100
<212> DNA
<213> Escherichia coli K-12 MG1655
<220> trec b4240
<222> 1543-1642
<223> trec b4240 U00096 complement(4460628..4462283)
<313> 6995: 1597-1616
<400> 6995
GGGCAAATGC GCGGCAACTG GCAGCTTGTG ATGCATAACT ACGAAGAAGC 50
CTCACCACAA CCCTGTGCCA TGAATTTACG GCCTTTTGAG GCTGTCTGGT 100
<210> 6996
<211> 100
<212> DNA
<213> Escherichia coli K-12 MG1655
<220> trec b4240
<222> 1541-1640
<223> trec b4240 U00096 complement(4460628..4462283)
<313> 6996: 1557-1576
<400> 6996
CAGGGCAAAT GCGCGGCAAC TGGCAGCTTG TGATGCATAA CTACGAAGAA 50
GCCTCACCAC AACCCTGTGC CATGAATTTA CGGCCTTTTG AGGCTGTCTG 100
<210> 6997
<211> 100
<212> DNA
<213> Escherichia coli K-12 MG1655

<220> trec b4240
<222> 1484-1583
<223> trec b4240 U00096 complement(4460628..4462283)
<313> 6997: 1523-1542
<400> 6997
AGGGGCAAAC CTTGCTGGTC ATTGCCAACC TTAGCCGTGA GATCCAACCC 50
TGGCAGGCAG GGCAAATGCG CGGCAACTGG CAGCTTGTGA TGCATAACTA 100
<210> 6998
<211> 100
<212> DNA
<213> Escherichia coli K-12 MG1655
<220> tref b3520
<222> 1526-1625
<223> tref b3520 U00096 3667222..3668871
<313> 6998: 1543-1562
<400> 6998
AAAAATACCA TATTGCCGAT GGTGTTCCCC GCGAAGGCGG CGGTGGCGAG 50
TATCCGTTGC AGGATGGGTT TGGCTGGACT AACGGTGTGG TACGCCGTTT 100
<210> 6999
<211> 100
<212> DNA
<213> Escherichia coli K-12 MG1655
<220> tref b3520
<222> 1453-1552
<223> tref b3520 U00096 3667222..3668871
<313> 6999: 1483-1502
<400> 6999
CTGGGTGATG AAATCGCGCG AAGCTGGCTG AAGACGGTGA ATCAGTTCTA 50
TCTGGAACAG CACAAACTGA TCGAAAAATA CCATATTGCC GATGGTGTTC 100
<210> 7000
<211> 100
<212> DNA
<213> Escherichia coli K-12 MG1655
<220> tref b3520
<222> 1388-1487
<223> tref b3520 U00096 3667222..3668871
<313> 7000: 1437-1456
<400> 7000
CCAACGGCTG GGCACCGTTA CAATGGATGG CGATTCAGGG ATTTAAAATG 50
TACGGCGATG ACCTTCTGGG TGATGAAATC GCGCGAAGCT GGCTGAAGAC 100
<210> 7001
<211> 100
<212> DNA
<213> Escherichia coli K-12 MG1655
<220> rpoa b3296
<222> 880-979
<223> rpoa b3296 U00096 complement(3437677..3438666)
<313> 7001: 912-931
<400> 7001
AACCTTGGTA AAAAATCTCT TACTGAGATT AAAGACGTGC TGGCTTCCCG 50
TGGACTGTCT CTGGGCATGC GCCTGGAAAA CTGGCCACCG GCAAGCATCG 100
<210> 7002
<211> 100
<212> DNA
<213> Escherichia coli K-12 MG1655
<220> rpoa b3296
<222> 811-910
<223> rpoa b3296 U00096 complement(3437677..3438666)
<313> 7002: 843-862
<400> 7002
AAAGCAGAAG CTATCCACTA TATCGGTGAT CTGGTACAGC GTACCGAGGT 50
TGAGCTCCTT AAAACGCCTA ACCTTGGTAA AAAATCTCTT ACTGAGATTA 100
<210> 7003
<211> 100
<212> DNA
<213> Escherichia coli K-12 MG1655
<220> rpoa b3296
<222> 752-851
<223> rpoa b3296 U00096 complement(3437677..3438666)
<313> 7003: 795-814
<400> 7003

CGATCCTGCT GCGCCCTGTT GACGATCTGG AATTGACTGT CCGCTCTGCT 50
AACTGCCTTA AAGCAGAAGC TATCCACTAT ATCGGTGATC TGGTACAGCG 100
<210> 7004
<211> 100
<212> DNA
<213> Escherichia coli K-12 MG1655
<220> rpob b3988
<222> 3930-4029
<223> rpob b3988 U00096 4178823..4182851
<313> 7004: 4010-4029
<400> 7004
CGGCAACCAT CAGATGGAGC CGGGCATGCC AGAATCCTTC AACGTATTGT 50
TGAAAGAGAT TCGTTCGCTG GGTATCAACA TCGAACTGGA AGACGAGTAA 100
<210> 7005
<211> 100
<212> DNA
<213> Escherichia coli K-12 MG1655
<220> rpob b3988
<222> 3895-3994
<223> rpob b3988 U00096 4178823..4182851
<313> 7005: 3918-3937
<400> 7005
AACGGTCGTA CCAAGATGTA TAAAAACATC GTGGACGGCA ACCATCAGAT 50
GGAGCCGGGC ATGCCAGAAT CCTTCAACGT ATTGTTGAAA GAGATTCGTT 100
<210> 7006
<211> 100
<212> DNA
<213> Escherichia coli K-12 MG1655
<220> rpob b3988
<222> 3795-3894
<223> rpob b3988 U00096 4178823..4182851
<313> 7006: 3809-3828
<400> 7006
CGGTGGTCAG CGTTTCGGGG AGATGGAAGT GTGGGCGCTG GAAGCATACG 50
GCGCAGCATA CACCCTGCAG GAAATGCTCA CCGTTAAGTC TGATGACGTG 100
<210> 7007
<211> 100
<212> DNA
<213> Escherichia coli K-12 MG1655
<220> rpoc b3989
<222> 4028-4127
<223> rpoc b3989 U00096 4182928..4187151
<313> 7007: 4058-4077
<400> 7007
AACTGCGCGG CCTGAAAGAG AACGTTATCG TGGGTCGTCT GATCCCGGCA 50
GGTACCGGTT ACGCGTACCA CCAGGATCGT ATGCGTCGCC GTGCTGCGGG 100
<210> 7008
<211> 100
<212> DNA
<213> Escherichia coli K-12 MG1655
<220> rpoc b3989
<222> 3939-4038
<223> rpoc b3989 U00096 4182928..4187151
<313> 7008: 3993-4012
<400> 7008
TCTGGCAACC GAGTCCTTCA TCTCCGCGGC ATCGTTCCAG GAGACCACTC 50
GCGTGCTGAC CGAAGCAGCC GTTGCGGGCA AACGCGACGA ACTGCGCGGC 100
<210> 7009
<211> 100
<212> DNA
<213> Escherichia coli K-12 MG1655
<220> rpoc b3989
<222> 3913-4012
<223> rpoc b3989 U00096 4182928..4187151
<313> 7009: 3938-3957
<400> 7009
GATCTGCTGG GTATCACCAA AGCGTCTCTG GCAACCGAGT CCTTCATCTC 50
CGCGGCATCG TTCCAGGAGA CCACTCGCGT GCTGACCGAA GCAGCCGTTG 100
<210> 7010
<211> 100
<212> DNA

<213> Escherichia coli K-12 MG1655
<220> rpod b3068
<222> 1726-1825
<223> rpod b3068 U00096 3210688..3212529
<313> 7010: 1757-1776
<400> 7010
GTGGGTAAAC AGTTCGACGT TACCCGCGAA CGTATCCGTC AGATCGAAGC 50
GAAGGCGCTG CGCAAACTGC GTCACCCGAG CCGTTCTGAA GTGCTGCGTA 100
<210> 7011
<211> 100
<212> DNA
<213> Escherichia coli K-12 MG1655
<220> rpod b3068
<222> 1668-1767
<223> rpod b3068 U00096 3210688..3212529
<313> 7011: 1717-1736
<400> 7011
AAAAGTTCTG CGTATGCGTT TCGGTATCGA TATGAACACC GACTACACGC 50
TGGAAGAAGT GGGTAAACAG TTCGACGTTA CCCGCGAACG TATCCGTCAG 100
<210> 7012
<211> 100
<212> DNA
<213> Escherichia coli K-12 MG1655
<220> rpod b3068
<222> 1538-1637
<223> rpod b3068 U00096 3210688..3212529
<313> 7012: 1585-1604
<400> 7012
ATGATGAAGA TTCGCATCTG GGGGATTTCA TCGAGGATAC CACCCTCGAG 50
CTGCCGCTGG ATTCTGCGAC CACCGAAAGC CTGCGTGCGG CAACGCACGA 100
<210> 7013
<211> 100
<212> DNA
<213> Escherichia coli K-12 MG1655
<220> rpoe b2574
<222> 477-576
<223> rpoe b2574 U00096 complement(2707457..2708032)
<313> 7013: 543-562
<400> 7013
AGCCGCTATC ATGGATTGTC CGGTAGGTAC GGTGCGTTCA CGTATCTTCC 50
GAGCGAGGGA AGCTATTGAT AACAAAGTTC AACCGCTTAT CAGGCGTTGA 100
<210> 7014
<211> 100
<212> DNA
<213> Escherichia coli K-12 MG1655
<220> rpoe b2574
<222> 449-548
<223> rpoe b2574 U00096 complement(2707457..2708032)
<313> 7014: 491-510
<400> 7014
AGCTGGATGG CCTGAGCTAT GAAGAGATAG CCGCTATCAT GGATTGTCCG 50
GTAGGTACGG TGCGTTCACG TATCTTCCGA GCGAGGGAAG CTATTGATAA 100
<210> 7015
<211> 100
<212> DNA
<213> Escherichia coli K-12 MG1655
<220> rpoe b2574
<222> 374-473
<223> rpoe b2574 U00096 complement(2707457..2708032)
<313> 7015: 412-431
<400> 7015
AAGAACTGAG ACAGATAGTT TTCCGAACTA TTGAGTCCCT CCCGGAAGAT 50
TTACGCATGG CAATAACCTT GCGGGAGCTG GATGGCCTGA GCTATGAAGA 100
<210> 7016
<211> 100
<212> DNA
<213> Escherichia coli K-12 MG1655
<220> amia b2436
<222> 771-870
<223> amia b2436 U00096 2550372..2551241
<313> 7016: 809-828

<400> 7016
GGCGGCGTTT CGTCAGAAAA TCGCCACAGC GATTGCTGAA GGCGTGATCA 50
GTTATTTCCA CTGGTTCGAC AACCAGAAAG CACATTCGAA AAAGCGATAA 100
<210> 7017
<211> 100
<212> DNA
<213> Escherichia coli K-12 MG1655
<220> amia b2436
<222> 723-822
<223> amia b2436 U00096 2550372..2551241
<313> 7017: 770-789
<400> 7017
GGAAACCTCG TTTATCACCA ACCCGGAAGA AGAACGGCTG TTAGGCACGG 50
CGGCGTTTCG TCAGAAAATC GCCACAGCGA TTGCTGAAGG CGTGATCAGT 100
<210> 7018
<211> 100
<212> DNA
<213> Escherichia coli K-12 MG1655
<220> amia b2436
<222> 688-787
<223> amia b2436 U00096 2550372..2551241
<313> 7018: 727-746
<400> 7018
GTGTTGAAAT CACCGTCGGT TCCTTCGGTG CTGGTGGAAA CCTCGTTTAT 50
CACCAACCCG GAAGAAGAAC GGCTGTTAGG CACGGCGGCG TTTCGTCAGA 100
<210> 7019
<211> 100
<212> DNA
<213> Escherichia coli K-12 MG1655
<220> rpoh b3462
<222> 747-846
<223> rpoh b3462 U00096 complement(3597560..3598414)
<313> 7019: 768-787
<400> 7019
CAAGTCCACG TTGCAGGAAC TGGCTGACCG TTACGGCGTT TCCGCTGAGC 50
GTGTACGCCA GCTGGAAAAG AACGCGATGA AAAAATTGCG TGCTGCCATT 100
<210> 7020
<211> 100
<212> DNA
<213> Escherichia coli K-12 MG1655
<220> rpoh b3462
<222> 621-720
<223> rpoh b3462 U00096 complement(3597560..3598414)
<313> 7020: 644-663
<400> 7020
CTTTGCCGAC GGCATTGAAG ATGATAACTG GGAAGAGCAG GCGGCAAACC 50
GTCTGACCGA CGCGATGCAG GGTCTGGACG AACGCAGCCA GGACATCATC 100
<210> 7021
<211> 100
<212> DNA
<213> Escherichia coli K-12 MG1655
<220> rpoh b3462
<222> 538-637
<223> rpoh b3462 U00096 complement(3597560..3598414)
<313> 7021: 575-594
<400> 7021
ATGACCTTTG ACCTGTCTTC CGACGACGAT TCCGACAGCC AGCCGATGGC 50
TCCGGTGCTC TATCTGCAGG ATAAATCATC TAACTTTGCC GACGGCATTG 100
<210> 7022
<211> 100
<212> DNA
<213> Escherichia coli K-12 MG1655
<220> amib b4170
<222> 1239-1338
<223> amib b4170 U00096 4393643..4394980
<313> 7022: 1317-1336
<400> 7022
CAATTACTTC CTTGCGCATC CGATGCAATC TGCGCCGCAG GGTGCAACGG 50
CACAAACTGC CAGTACGGTG ACGACGCCAG ATCGCACGCT GCCAAACTAA 100
<210> 7023
<211> 100

<212> DNA
<213> Escherichia coli K-12 MG1655
<220> amib b4170
<222> 973-1072
<223> amib b4170 U00096 4393643..4394980
<313> 7023: 1030-1049
<400> 7023
TTAAGCCAGG CGGTGCTGGA TTTACAGTTC GGTCATTCCC AGCGGGTAGG 50
GTATGATGTA GCGACCAGTA TGATCAGTCA GTTGCAACGC ATTGGCGAAA 100
<210> 7024
<211> 100
<212> DNA
<213> Escherichia coli K-12 MG1655
<220> amib b4170
<222> 925-1024
<223> amib b4170 U00096 4393643..4394980
<313> 7024: 953-972
<400> 7024
CTGGGTGGGG CGGGTGATGT GCTGGCGAAC AGTCAGTCTG ACCCCTATTT 50
AAGCCAGGCG GTGCTGGATT TACAGTTCGG TCATTCCCAG CGGGTAGGGT 100
<210> 7025
<211> 100
<212> DNA
<213> Escherichia coli K-12 MG1655
<220> insb_1 b0022
<222> 108-207
<223> insb_1 b0022 U00096 complement(19811..20314)
<313> 7025: 136-155
<400> 7025
CGTCATCGTC TGCGCGGAAA TGGACGAACA GTGGGGCTAT GTCGGGGCTA 50
AATCGCGCCA GCGCTGGCTG TTTTACGCGT ATGACAGTCT CCGGAAGACG 100
<210> 7026
<211> 100
<212> DNA
<213> Escherichia coli K-12 MG1655
<220> insb_1 b0022
<222> 104-203
<223> insb_1 b0022 U00096 complement(19811..20314)
<313> 7026: 136-155
<400> 7026
GTGACGTCAT CGTCTGCGCG GAAATGGACG AACAGTGGGG CTATGTCGGG 50
GCTAAATCGC GCCAGCGCTG GCTGTTTTAC GCGTATGACA GTCTCCGGAA 100
<210> 7027
<211> 100
<212> DNA
<213> Escherichia coli K-12 MG1655
<220> insb_1 b0022
<222> 17-116
<223> insb_1 b0022 U00096 complement(19811..20314)
<313> 7027: 71-90
<400> 7027
CGCATTATGG GCGTTGGCCT CAACACGATT TTACGTCACT TAAAAAACTC 50
AGGCCGCAGT CGGTAACCTC GCGCATACAG CCGGGCAGTG ACGTCATCGT 100
<210> 7028
<211> 100
<212> DNA
<213> Escherichia coli K-12 MG1655
<220> trer b4242
<222> 849-948
<223> trer b4242 U00096 complement(4463873..4464820)
<313> 7028: 929-948
<400> 7028
TTACGCCGAA GCTGGACGCC AGGCGGCTTG CCAGTTGATC GCGCAGGTAA 50
CCGGGCGCAG CGAACCGCAA CAAATCATCA TCCCCGCCAC CCTGTCCTGA 100
<210> 7029
<211> 100
<212> DNA
<213> Escherichia coli K-12 MG1655
<220> trer b4242
<222> 849-948
<223> trer b4242 U00096 complement(4463873..4464820)

<313> 7029: 869-888
<400> 7029
TTACGCCGAA GCTGGACGCC AGGCGGCTTG CCAGTTGATC GCGCAGGTAA 50
CCGGGCGCAG CGAACCGCAA CAAATCATCA TCCCCGCCAC CCTGTCCTGA 100
<210> 7030
<211> 100
<212> DNA
<213> Escherichia coli K-12 MG1655
<220> trer b4242
<222> 699-798
<223> trer b4242 U00096 complement(4463873..4464820)
<313> 7030: 753-772
<400> 7030
TACCGCCTTA CTGTGCGCAA CCGACACGCT GGCACTTGGC GCAAGTAAAT 50
ACCTGCAAGA GCAACGCATC GACACCTTGC AACTGGCGAG CGTCGGTAAT 100
<210> 7031
<211> 100
<212> DNA
<213> Escherichia coli K-12 MG1655
<220> insb_2 b0265
<222> 405-504
<223> insb_2 b0265 U00096 complement(278402..278905)
<313> 7031: 467-486
<400> 7031
CCTGGCACGG CTGGGACGGA AGTCGCTGTC GTTCTCAAAA TCGGTGGAGC 50
AGCATGACAA AGTCATCGGG CATTATCTGA ACATAAAACA CTATCAATAA 100
<210> 7032
<211> 100
<212> DNA
<213> Escherichia coli K-12 MG1655
<220> insb_2 b0265
<222> 403-502
<223> insb_2 b0265 U00096 complement(278402..278905)
<313> 7032: 463-482
<400> 7032
CACCTGGCAC GGCTGGGACG GAAGTCGCTG TCGTTCTCAA AATCGGTGGA 50
GCAGCATGAC AAAGTCATCG GGCATTATCT GAACATAAAA CACTATCAAT 100
<210> 7033
<211> 100
<212> DNA
<213> Escherichia coli K-12 MG1655
<220> insb_2 b0265
<222> 380-479
<223> insb_2 b0265 U00096 complement(278402..278905)
<313> 7033: 447-466
<400> 7033
GGCATAACCT GAATCTGAGG CAGCACCTGG CACGGCTGGG ACGGAAGTCG 50
CTGTCGTTCT CAAAATCGGT GGAGCAGCAT GACAAAGTCA TCGGGCATTA 100
<210> 7034
<211> 100
<212> DNA
<213> Escherichia coli K-12 MG1655
<220> insb_3 b0275
<222> 405-504
<223> insb_3 b0275 U00096 complement(289873..290376)
<313> 7034: 457-476
<400> 7034
CCTGGCACGG CTGGGACGGA AGTCGCTGTC GTTCTCAAAA TCGGTGGAGC 50
AGCATGACAA AGTCATCGGG CATTATCTGA ACATAAAACA CTATCAATAA 100
<210> 7035
<211> 100
<212> DNA
<213> Escherichia coli K-12 MG1655
<220> insb_3 b0275
<222> 405-504
<223> insb_3 b0275 U00096 complement(289873..290376)
<313> 7035: 447-466
<400> 7035
CCTGGCACGG CTGGGACGGA AGTCGCTGTC GTTCTCAAAA TCGGTGGAGC 50
AGCATGACAA AGTCATCGGG CATTATCTGA ACATAAAACA CTATCAATAA 100
<210> 7036

<211> 100
<212> DNA
<213> Escherichia coli K-12 MG1655
<220> insb_3 b0275
<222> 405-504
<223> insb_3 b0275 U00096 complement(289873..290376)
<313> 7036: 443-462
<400> 7036
CCTGGCACGG CTGGGACGGA AGTCGCTGTC GTTCTCAAAA TCGGTGGAGC 50
AGCATGACAA AGTCATCGGG CATTATCTGA ACATAAAACA CTATCAATAA 100
<210> 7037
<211> 100
<212> DNA
<213> Escherichia coli K-12 MG1655
<220> rpon b3203
<222> 1335-1434
<223> rpon b3203 U00096 3342358..3343791
<313> 7037: 1396-1415
<400> 7037
GCTGTCGGAA CAAGGTATCA TGGTGGCACG CCGCACTGTT GCGAAGTACC 50
GAGAGTCTTT ATCCATTCCG CCGTCAAACC AGCGTAAACA ACTCGTTTGA 100
<210> 7038
<211> 100
<212> DNA
<213> Escherichia coli K-12 MG1655
<220> rpon b3203
<222> 1269-1368
<223> rpon b3203 U00096 3342358..3343791
<313> 7038: 1310-1329
<400> 7038
GGTGAAGAAA TTAATCGCGG CGGAAAACCC AGCGAAACCG TTGAGCGACA 50
GCAAGTTAAC CTCTTTGCTG TCGGAACAAG GTATCATGGT GGCACGCCGC 100
<210> 7039
<211> 100
<212> DNA
<213> Escherichia coli K-12 MG1655
<220> rpon b3203
<222> 1173-1272
<223> rpon b3203 U00096 3342358..3343791
<313> 7039: 1210-1229
<400> 7039
TAGTCCACGA GGCATTTTTG AACTGAAGTA TTTCTTTTCC AGTCACGTCA 50
ATACCGAGGG CGGCGGCGAA GCTTCCTCCA CGGCGATTCG TGCGCTGGTG 100
<210> 7040
<211> 100
<212> DNA
<213> Escherichia coli K-12 MG1655
<220> insb_4 b0989
<222> 336-435
<223> insb_4 b0989 U00096 1049250..1049753
<313> 7040: 393-412
<400> 7040
AAAGCTGCAC GTAATCAGCA AGCGATATAC GCAGCGAATT GAGCGGTATA 50
ACCTGAATCT GAGGCAGCAC CTGGCACGGC TGGGACGGAA GTCGCTGTCG 100
<210> 7041
<211> 100
<212> DNA
<213> Escherichia coli K-12 MG1655
<220> insb_4 b0989
<222> 358-457
<223> insb_4 b0989 U00096 1049250..1049753
<313> 7041: 382-401
<400> 7041
CGATATACGC AGCGAATTGA GCGGTATAAC CTGAATCTGA GGCAGCACCT 50
GGCACGGCTG GGACGGAAGT CGCTGTCGTT CTCAAAATCG GTGGAGCTGC 100
<210> 7042
<211> 100
<212> DNA
<213> Escherichia coli K-12 MG1655
<220> insb_4 b0989
<222> 325-424

<223> insb_4 b0989 U00096 1049250..1049753
<313> 7042: 363-382
<400> 7042
CGCCTGAAGG GAAAGCTGCA CGTAATCAGC AAGCGATATA CGCAGCGAAT 50
TGAGCGGTAT AACCTGAATC TGAGGCAGCA CCTGGCACGG CTGGGACGGA 100
<210> 7043
<211> 100
<212> DNA
<213> Escherichia coli K-12 MG1655
<220> insb_5 b1894
<222> 84-183
<223> insb_5 b1894 U00096 complement(1976542..1977045)
<313> 7043: 149-168
<400> 7043
CTCGCGCATA CAGCCGGGCA GTGACGTCAT CGTCTGCGCG GAAATGGACG 50
AACAGTGGGG CTATGTCGGG GCTAAATCGC GCCAGCGCTG GCTGTTTTAC 100
<210> 7044
<211> 100
<212> DNA
<213> Escherichia coli K-12 MG1655
<220> insb_5 b1894
<222> 109-208
<223> insb_5 b1894 U00096 complement(1976542..1977045)
<313> 7044: 138-157
<400> 7044
GTCATCGTCT GCGCGGAAAT GGACGAACAG TGGGGCTATG TCGGGGCTAA 50
ATCGCGCCAG CGCTGGCTGT TTTACGCGTA TGACAGTCTC CGGAAGACGG 100
<210> 7045
<211> 100
<212> DNA
<213> Escherichia coli K-12 MG1655
<220> insb_5 b1894
<222> 22-121
<223> insb_5 b1894 U00096 complement(1976542..1977045)
<313> 7045: 57-76
<400> 7045
TATGGGCGTT GGCCTCAACA CGATTTTACG TCACTTAAAA AACTCAGGCC 50
GCAGTCGGTA ACCTCGCGCA TACAGCCGGG CAGTGACGTC ATCGTCTGCG 100
<210> 7046
<211> 100
<212> DNA
<213> Escherichia coli K-12 MG1655
<220> insb_6 b3446
<222> 83-182
<223> insb_6 b3446 U00096 3581308..3581811
<313> 7046: 132-151
<400> 7046
CCTCGCGCAT ACAGCCGGGC AGTGACGTCA TCGTCTGCGC GGAAATGGAC 50
GAACAGTGGG GCTATGTCGG GGCTAAATCG CGCCAGCGCT GGCTGTTTTA 100
<210> 7047
<211> 100
<212> DNA
<213> Escherichia coli K-12 MG1655
<220> insb_6 b3446
<222> 110-209
<223> insb_6 b3446 U00096 3581308..3581811
<313> 7047: 127-146
<400> 7047
TCATCGTCTG CGCGGAAATG GACGAACAGT GGGGCTATGT CGGGGCTAAA 50
TCGCGCCAGC GCTGGCTGTT TTACGCGTAT GACAGTCTCC GGAAGACGGT 100
<210> 7048
<211> 100
<212> DNA
<213> Escherichia coli K-12 MG1655
<220> insb_6 b3446
<222> 18-117
<223> insb_6 b3446 U00096 3581308..3581811
<313> 7048: 73-92
<400> 7048
GCATTATGGG CGTTGGCCTC AACACGATTT TACGTCACTT AAAAAACTCA 50
GGCCGCAGTC GGTAACCTCG CGCATACAGC CGGGCAGTGA CGTCATCGTC 100

<210> 7049
<211> 100
<212> DNA
<213> Escherichia coli K-12 MG1655
<220> rpos b2742
<222> 838-937
<223> rpos b2742 U00096 complement(2864582..2865574)
<313> 7049: 867-886
<400> 7049
TTGCTGGGGT ACGAAGCGGC AACACTGGAA GATGTAGGTC GTGAAATTGG 50
CCTCACCCGT GAACGTGTTC GCCAGATTCA GGTTGAAGGC CTGCGCCGTT 100
<210> 7050
<211> 100
<212> DNA
<213> Escherichia coli K-12 MG1655
<220> rpos b2742
<222> 805-904
<223> rpos b2742 U00096 complement(2864582..2865574)
<313> 7050: 839-858
<400> 7050
AAACAGCGTG AAGTGCTGGC ACGTCGATTC GGTTTGCTGG GGTACGAAGC 50
GGCAACACTG GAAGATGTAG GTCGTGAAAT TGGCCTCACC CGTGAACGTG 100
<210> 7051
<211> 100
<212> DNA
<213> Escherichia coli K-12 MG1655
<220> rpos b2742
<222> 701-800
<223> rpos b2742 U00096 complement(2864582..2865574)
<313> 7051: 752-771
<400> 7051
TGCTGGACAT CCTGGCCGAT GAAAAAGAGA ACGGTCCGGA AGATACCACG 50
CAAGATGACG ATATGAAGCA GAGCATCGTC AAATGGCTGT TCGAGCTGAA 100
<210> 7052
<211> 100
<212> DNA
<213> Escherichia coli K-12 MG1655
<220> rpoz b3650
<222> 177-276
<223> rpoz b3650 U00096 3819733..3820008
<313> 7052: 255-274
<400> 7052
CAACAACCAG ATCCTCGACG TTCGCGAACG CCAGGAACAG CAAGAGCAGG 50
AAGCCGCTGA ATTACAAGCC GTTACCGCTA TTGCTGAAGG TCGTCGTTAA 100
<210> 7053
<211> 100
<212> DNA
<213> Escherichia coli K-12 MG1655
<220> rpoz b3650
<222> 140-239
<223> rpoz b3650 U00096 3819733..3820008
<313> 7053: 182-201
<400> 7053
CTGTAATCGC GCTGCGCGAA ATCGAAGAAG GTCTGATCAA CAACCAGATC 50
CTCGACGTTC GCGAACGCCA GGAACAGCAA GAGCAGGAAG CCGCTGAATT 100
<210> 7054
<211> 100
<212> DNA
<213> Escherichia coli K-12 MG1655
<220> rpoz b3650
<222> 71-170
<223> rpoz b3650 U00096 3819733..3820008
<313> 7054: 130-149
<400> 7054
CGCGTCGCGC TCGTCAGATG CAGGTAGGCG GAAAGGATCC GCTGGTACCG 50
GAAGAAAACG ATAAAACCAC TGTAATCGCG CTGCGCGAAA TCGAAGAAGG 100
<210> 7055
<211> 100
<212> DNA
<213> Escherichia coli K-12 MG1655
<220> suca b0727

<222> 2703-2802
<223> suca b0727 U00096 757929..760730
<313> 7055: 2767-2786
<400> 7055
TGCAGGCCGC CCGGCCTCCG CCTCTCCGGC GGTAGGGTAT ATGTCCGTTC 50
ACCAGAAACA GCAACAAGAT CTGGTTAATG ACGCGCTGAA CGTCGAATAA 100
<210> 7056
<211> 100
<212> DNA
<213> Escherichia coli K-12 MG1655
<220> suca b0727
<222> 2564-2663
<223> suca b0727 U00096 757929..760730
<313> 7056: 2619-2638
<400> 7056
CGATGCAGGA AGTGTTGCAG CAGTTTGCTC ACGTCAAGGA TTTTGTCTGG 50
TGCCAGGAAG AGCCGCTCAA CCAGGGCGCA TGGTACTGCA GCCAGCATCA 100
<210> 7057
<211> 100
<212> DNA
<213> Escherichia coli K-12 MG1655
<220> suca b0727
<222> 2518-2617
<223> suca b0727 U00096 757929..760730
<313> 7057: 2559-2578
<400> 7057
GTCGCCATTG TGCGTATCGA GCAACTCTAC CCGTTCCCGC ATAAAGCGAT 50
GCAGGAAGTG TTGCAGCAGT TTGCTCACGT CAAGGATTTT GTCTGGTGCC 100
<210> 7058
<211> 100
<212> DNA
<213> Escherichia coli K-12 MG1655
<220> sucb b0728
<222> 1035-1134
<223> sucb b0728 U00096 760745..761962
<313> 7058: 1083-1102
<400> 7058
TCTGGGTATG CACGCTATCA AAGATCGTCC GATGGCGGTG AATGGTCAGG 50
TTGAGATCCT GCCGATGATG TACCTGGCGC TGTCCTACGA TCACCGTCTG 100
<210> 7059
<211> 100
<212> DNA
<213> Escherichia coli K-12 MG1655
<220> sucb b0728
<222> 978-1077
<223> sucb b0728 U00096 760745..761962
<313> 7059: 1034-1053
<400> 7059
TGGTGTGTTC GGTTCCCTGA TGTCTACGCC GATCATCAAC CCGCCGCAGA 50
GCGCAATTCT GGGTATGCAC GCTATCAAAG ATCGTCCGAT GGCGGTGAAT 100
<210> 7060
<211> 100
<212> DNA
<213> Escherichia coli K-12 MG1655
<220> sucb b0728
<222> 973-1072
<223> sucb b0728 U00096 760745..761962
<313> 7060: 993-1012
<400> 7060
AACGGTGGTG TGTTCGGTTC CCTGATGTCT ACGCCGATCA TCAACCCGCC 50
GCAGAGCGCA ATTCTGGGTA TGCACGCTAT CAAAGATCGT CCGATGGCGG 100
<210> 7061
<211> 100
<212> DNA
<213> Escherichia coli K-12 MG1655
<220> succ b0729
<222> 1068-1167
<223> succ b0729 U00096 762237..763403
<313> 7061: 1135-1154
<400> 7061
CGGCGCGAAG AAACTGGCTG ACAGCGGCCT GAATATTATT GCAGCAAAAG 50

GTCTGACGGA TGCAGCTCAG CAGGTTGTTG CCGCAGTGGA GGGGAAATAA 100
<210> 7062
<211> 100
<212> DNA
<213> Escherichia coli K-12 MG1655
<220> succ b0729
<222> 1042-1141
<223> succ b0729 U00096 762237..763403
<313> 7062: 1092-1111
<400> 7062
CGTCTGGAAG GTAACAACGC CGAACTCGGC GCGAAGAAAC TGGCTGACAG 50
CGGCCTGAAT ATTATTGCAG CAAAAGGTCT GACGGATGCA GCTCAGCAGG 100
<210> 7063
<211> 100
<212> DNA
<213> Escherichia coli K-12 MG1655
<220> succ b0729
<222> 997-1096
<223> succ b0729 U00096 762237..763403
<313> 7063: 1050-1069
<400> 7063
ATCGGCGCGG TAGCAGAAGT GGGTGTTAAC GTACCGGTCG TGGTACGTCT 50
GGAAGGTAAC AACGCCGAAC TCGGCGCGAA GAAACTGGCT GACAGCGGCC 100
<210> 7064
<211> 100
<212> DNA
<213> Escherichia coli K-12 MG1655
<220> sucd b0730
<222> 771-870
<223> sucd b0730 U00096 763403..764272
<313> 7064: 837-856
<400> 7064
GACTGCGGAT GAGAAATTCG CTGCTCTGGA AGCCGCAGGC GTGAAAACCG 50
TTCGCAGCCT GGCGGATATC GGTGAAGCAC TGAAAACTGT TCTGAAATAA 100
<210> 7065
<211> 100
<212> DNA
<213> Escherichia coli K-12 MG1655
<220> sucd b0730
<222> 753-852
<223> sucd b0730 U00096 763403..764272
<313> 7065: 807-826
<400> 7065
CATTGCCGGT GGGAAAGGGA CTGCGGATGA GAAATTCGCT GCTCTGGAAG 50
CCGCAGGCGT GAAAACCGTT CGCAGCCTGG CGGATATCGG TGAAGCACTG 100
<210> 7066
<211> 100
<212> DNA
<213> Escherichia coli K-12 MG1655
<220> sucd b0730
<222> 674-773
<223> sucd b0730 U00096 763403..764272
<313> 7066: 736-755
<400> 7066
ACGTTACCAA GCCAGTTGTG GGTTACATCG CTGGTGTGAC TGCGCCGAAA 50
GGCAAACGTA TGGGCCACGC GGGTGCCATC ATTGCCGGTG GGAAAGGGAC 100
<210> 7067
<211> 100
<212> DNA
<213> Escherichia coli K-12 MG1655
<220> bfr b3337
<222> 378-477
<223> bfr b3337 U00096 complement(3463886..3464362)
<313> 7067: 445-464
<400> 7067
TGAAGAAGGC CATATCGACT GGCTGGAAAC GGAACTTGAT CTGATTCAGA 50
AGATGGGCCT GCAAAATTAT CTGCAAGCAC AGATCCGCGA AGAAGGTTGA 100
<210> 7068
<211> 100
<212> DNA
<213> Escherichia coli K-12 MG1655

<220> bfr b3337
<222> 329-428
<223> bfr b3337 U00096 complement(3463886..3464362)
<313> 7068: 354-373
<400> 7068
GCGTTCATGA TTACGTCAGC CGCGATATGA TGATAGAAAT TTTGCGTGAT 50
GAAGAAGGCC ATATCGACTG GCTGGAAACG GAACTTGATC TGATTCAGAA 100
<210> 7069
<211> 100
<212> DNA
<213> Escherichia coli K-12 MG1655
<220> bfr b3337
<222> 228-327
<223> bfr b3337 U00096 complement(3463886..3464362)
<313> 7069: 266-285
<400> 7069
ACTGAACATT GGTGAAGATG TTGAGGAAAT GCTGCGTTCT GATCTGGCAC 50
TTGAGCTGGA TGGCGCGAAG AATTTGCGTG AGGCAATTGG TTATGCCGAT 100
<210> 7070
<211> 100
<212> DNA
<213> Escherichia coli K-12 MG1655
<220> yzgl b3428
<222> 195-294
<223> yzgl b3428 U00096 complement(3561357..3561650)
<313> 7070: 262-281
<400> 7070
GCTGGGCGCA TTAATGCTAT TTTTCTCGGG GCGAGTGGGG AACGTGCCTG 50
AGTTTTGTTA CGTTGGGTAT GATGGCGTGG GCTTTGCGGT AATTCCCTGA 100
<210> 7071
<211> 100
<212> DNA
<213> Escherichia coli K-12 MG1655
<220> yzgl b3428
<222> 195-294
<223> yzgl b3428 U00096 complement(3561357..3561650)
<313> 7071: 251-270
<400> 7071
GCTGGGCGCA TTAATGCTAT TTTTCTCGGG GCGAGTGGGG AACGTGCCTG 50
AGTTTTGTTA CGTTGGGTAT GATGGCGTGG GCTTTGCGGT AATTCCCTGA 100
<210> 7072
<211> 100
<212> DNA
<213> Escherichia coli K-12 MG1655
<220> yzgl b3428
<222> 195-294
<223> yzgl b3428 U00096 complement(3561357..3561650)
<313> 7072: 222-241
<400> 7072
GCTGGGCGCA TTAATGCTAT TTTTCTCGGG GCGAGTGGGG AACGTGCCTG 50
AGTTTTGTTA CGTTGGGTAT GATGGCGTGG GCTTTGCGGT AATTCCCTGA 100
<210> 7073
<211> 100
<212> DNA
<213> Escherichia coli K-12 MG1655
<220> mhpa b0348
<222> 1566-1665
<223> mhpa b0348 U00096 367835..369499
<313> 7073: 1646-1665
<400> 7073
CGCCATTCCG CAAACCCTGG GCAAGACCCT GAATAAACTG GCGTCGGTGA 50
TGACGCTGAC CCGCCCTGAT GCCGACGTTT CTGTCGAAAA GGTAGCCTGA 100
<210> 7074
<211> 100
<212> DNA
<213> Escherichia coli K-12 MG1655
<220> mhpa b0348
<222> 1521-1620
<223> mhpa b0348 U00096 367835..369499
<313> 7074: 1589-1608
<400> 7074

TGCTTCGCTG GTGGTGATGC GCCCGGATCG CTTTGTTGCC GCCACCGCCA 50
TTCCGCAAAC CCTGGGCAAG ACCCTGAATA AACTGGCGTC GGTGATGACG 100
<210> 7075
<211> 100
<212> DNA
<213> Escherichia coli K-12 MG1655
<220> mhpa b0348
<222> 1406-1505
<223> mhpa b0348 U00096 367835..369499
<313> 7075: 1438-1457
<400> 7075
GCTTCATTCA GGTGGTGCCG GAAGTGCAAA TTCATACCGC ACAGGATAAC 50
CACGACGGCG TACTACGCGT GGGCGATACG CAAGGTCGCC TGCGTAGCTG 100
<210> 7076
<211> 100
<212> DNA
<213> Escherichia coli K-12 MG1655
<220> mhpb b0349
<222> 820-919
<223> mhpb b0349 U00096 369501..370445
<313> 7076: 860-879
<400> 7076
ACCTGGGTCG CCGCTTTTGC CGCTATTTCT GCGTTTGGCA ACTGGCGTAG 50
CGAAGGGCGT TATTACCGCC CAATCCCGGA GTGGATTGCC GGATTTGGCT 100
<210> 7077
<211> 100
<212> DNA
<213> Escherichia coli K-12 MG1655
<220> mhpb b0349
<222> 721-820
<223> mhpb b0349 U00096 369501..370445
<313> 7077: 744-763
<400> 7077
TTCATGACTT TGCTGGAGCA GGGACGCATA CAGGAACTGG ATGCCGTCAG 50
TAACGAAGAG CTTTCCGCCA TTGCCGGAAA GTCGACACAT GAAATCAAAA 100
<210> 7078
<211> 100
<212> DNA
<213> Escherichia coli K-12 MG1655
<220> mhpb b0349
<222> 658-757
<223> mhpb b0349 U00096 369501..370445
<313> 7078: 708-727
<400> 7078
GCTGAGAAGT TTGTTGAGGA TCAGAGAACG CTGCATCCGC TCAACCCGAT 50
TTGGGATAAC CAGTTCATGA CTTTGCTGGA GCAGGGACGC ATACAGGAAC 100
<210> 7079
<211> 100
<212> DNA
<213> Escherichia coli K-12 MG1655
<220> mhpc b0350
<222> 831-930
<223> mhpc b0350 U00096 370400..371329
<313> 7079: 910-929
<400> 7079
TTCTGAACTG CATATCTTCC GCGACTGTGG TCACTGGGCG CAGTGGGAAC 50
ATGCCGACGC TTTCAATCAA CTGGTGCTGA ATTTCCTCGC ACGCCCTTAA 100
<210> 7080
<211> 100
<212> DNA
<213> Escherichia coli K-12 MG1655
<220> mhpc b0350
<222> 773-872
<223> mhpc b0350 U00096 370400..371329
<313> 7080: 828-847
<400> 7080
GCAACGACCG CTTTGTGCCG ATGGATGCGG GTCTGCGTCT GCTGTCCGGC 50
ATTGCCGGTT CTGAACTGCA TATCTTCCGC GACTGTGGTC ACTGGGCGCA 100
<210> 7081
<211> 100
<212> DNA

<213> Escherichia coli K-12 MG1655
<220> mhpc b0350
<222> 723-822
<223> mhpc b0350 U00096 370400..371329
<313> 7081: 747-766
<400> 7081
TGGCCCACGT CTGGCGGAAA TCAAAGCGCA AACCCTGATT GTCTGGGGGC 50
GCAACGACCG CTTTGTGCCG ATGGATGCGG GTCTGCGTCT GCTGTCCGGC 100
<210> 7082
<211> 100
<212> DNA
<213> Escherichia coli K-12 MG1655
<220> mhpd b0351
<222> 717-816
<223> mhpd b0351 U00096 371333..372148
<313> 7082: 789-808
<400> 7082
GGCGGTGAAT GCGGGCGATC GTTTTGAAGC CCATATTGAA GGCATAGGTT 50
CAGTTGCTGC GACATTTTCA AGCGCAGCCC CAAAAGGAAG TCTGTCATGA 100
<210> 7083
<211> 100
<212> DNA
<213> Escherichia coli K-12 MG1655
<220> mhpd b0351
<222> 686-785
<223> mhpd b0351 U00096 371333..372148
<313> 7083: 702-721
<400> 7083
TCATTCTTAC CGGGGCATTA GGTCCGATGG TGGCGGTGAA TGCGGGCGAT 50
CGTTTTGAAG CCCATATTGA AGGCATAGGT TCAGTTGCTG CGACATTTTC 100
<210> 7084
<211> 100
<212> DNA
<213> Escherichia coli K-12 MG1655
<220> mhpd b0351
<222> 544-643
<223> mhpd b0351 U00096 371333..372148
<313> 7084: 595-614
<400> 7084
AACTGCGCCA TGAAGATGAC GCGTAATAAC GAAGAGGTTT CTAGCGGGCG 50
CGGCAGCGAA TGCCTGGGAC ATCCGCTTAA TGCGGCCGTC TGGCTGGCAC 100
<210> 7085
<211> 100
<212> DNA
<213> Escherichia coli K-12 MG1655
<220> mhpe b0353
<222> 905-1004
<223> mhpe b0353 U00096 373092..374105
<313> 7085: 940-959
<400> 7085
ATGGCTTAAG TGCGGTGGAT ATTCTCGTTG AGCTGGGCAA ACGCCGGATG 50
GTTGGCGGCC AGGAGGATAT GATCGTTGAC GTGGCGCTGG ATCTGCGCAA 100
<210> 7086
<211> 100
<212> DNA
<213> Escherichia coli K-12 MG1655
<220> mhpe b0353
<222> 655-754
<223> mhpe b0353 U00096 373092..374105
<313> 7086: 699-718
<400> 7086
CGAATCGACG CCAGCCTCGC GGGAATGGGC GCGGGCGCAG GTAACGCACC 50
GCTGGAAGTG TTTATTGCCG CCGCGGATAA ACTGGGCTGG CAGCATGGGA 100
<210> 7087
<211> 100
<212> DNA
<213> Escherichia coli K-12 MG1655
<220> mhpe b0353
<222> 579-678
<223> mhpe b0353 U00096 373092..374105
<313> 7087: 597-616

<400> 7087
AACTGGCATG CACGCTCACC ATAACCTGAG TCTTGGCGTG GCGAACTCTA  50
TCGCGGCGGT GGAAGAGGGC TGCGACCGAA TCGACGCCAG CCTCGCGGGA  100
<210> 7088
<211> 100
<212> DNA
<213> Escherichia coli K-12 MG1655
<220> ykfa b0254
<222> 768-867
<223> ykfa b0254 U00096 complement(267321..268187)
<313> 7088: 833-852
<400> 7088
TGCCTTTCCC CTGATACCCG CCCCGGTGCG GGCCGTCATT CAGGCCGTGC  50
GCACCACGGT GGTGTCAGTG GCCCGCGCCG TCTGGGATTT CTTCTTCTGA  100
<210> 7089
<211> 100
<212> DNA
<213> Escherichia coli K-12 MG1655
<220> ykfa b0254
<222> 712-811
<223> ykfa b0254 U00096 complement(267321..268187)
<313> 7089: 765-784
<400> 7089
GAACAGGCTC GTAGCGATTT TGGTGAAACC GTCGGGGCCG TACTCGATAG  50
CATCAGTGCC TTTCCCCTGA TACCCGCCCC GGTGCGGGCC GTCATTCAGG  100
<210> 7090
<211> 100
<212> DNA
<213> Escherichia coli K-12 MG1655
<220> ykfa b0254
<222> 673-772
<223> ykfa b0254 U00096 complement(267321..268187)
<313> 7090: 730-749
<400> 7090
TCGCAACTCC ATCCTTCCTT TCGCACGACG GTAGTCCGGG AACAGGCTCG  50
TAGCGATTTT GGTGAAACCG TCGGGGCCGT ACTCGATAGC ATCAGTGCCT  100
<210> 7091
<211> 100
<212> DNA
<213> Escherichia coli K-12 MG1655
<220> mhpf b0352
<222> 825-924
<223> mhpf b0352 U00096 372145..373095
<313> 7091: 857-876
<400> 7091
AGTCGAAGGC GCAGCGCATT ATCTGCCTGC CTATGCGGGC AACCTCGACA  50
TTATGACTTC CAGTGCGCTG GCGACAGCGG AAAAAATGGC CCAGTCACTG  100
<210> 7092
<211> 100
<212> DNA
<213> Escherichia coli K-12 MG1655
<220> mhpf b0352
<222> 555-654
<223> mhpf b0352 U00096 372145..373095
<313> 7092: 590-609
<400> 7092
GGGCGGCGCG GCAAAAGGGA AGGCGATTAT TGTGCTTAAC CCAGCAGAGC  50
CACCGTTGAT GATGCGTGAC ACGGTGTATG TATTGAGCGA CGAAGCTTCA  100
<210> 7093
<211> 100
<212> DNA
<213> Escherichia coli K-12 MG1655
<220> mhpf b0352
<222> 515-614
<223> mhpf b0352 U00096 372145..373095
<313> 7093: 563-582
<400> 7093
ATGAATTTAC GGAAACCACT TCCCGAGCCA TTGAAGTGGT GGGCGGCGCG  50
GCAAAAGGGA AGGCGATTAT TGTGCTTAAC CCAGCAGAGC CACCGTTGAT  100
<210> 7094
<211> 100

<212> DNA
<213> Escherichia coli K-12 MG1655
<220> ykfb b0251
<222> 369-468
<223> ykfb b0251 U00096 complement(264844..265311)
<313> 7094: 419-438
<400> 7094
CGACGTCTAC GTCAATGACC ACAAAGCAGG GACCTTCACT GCGGACCGGA 50
AGAACGCTGA GAAAGTTCTG TCCACTCTCG GAGACTGCAG CAACGACTGA 100
<210> 7095
<211> 100
<212> DNA
<213> Escherichia coli K-12 MG1655
<220> ykfb b0251
<222> 333-432
<223> ykfb b0251 U00096 complement(264844..265311)
<313> 7095: 392-411
<400> 7095
TGACTTCATC AGCAATATCT CTGAGGCCGG GCAGTTCGAC GTCTACGTCA 50
ATGACCACAA AGCAGGGACC TTCACTGCGG ACCGGAAGAA CGCTGAGAAA 100
<210> 7096
<211> 100
<212> DNA
<213> Escherichia coli K-12 MG1655
<220> ykfb b0251
<222> 304-403
<223> ykfb b0251 U00096 complement(264844..265311)
<313> 7096: 350-369
<400> 7096
GGCTGGCGTA ACGGCGATAA CGCCTGGTTT GACTTCATCA GCAATATCTC 50
TGAGGCCGGG CAGTTCGACG TCTACGTCAA TGACCACAAA GCAGGGACCT 100
<210> 7097
<211> 100
<212> DNA
<213> Escherichia coli K-12 MG1655
<220> ykfc b0259
<222> 1032-1131
<223> ykfc b0259 U00096 272086..273216
<313> 7097: 1108-1127
<400> 7097
GGTCTCAACG ATCCCGCAGG AGAAAGCCAG AAACTTCGCC GCATCGCTGA 50
CAGCACTGTT ATGGAAGGTG CGAATAAGCG GGGAAATTCT TCTCGGCTGA 100
<210> 7098
<211> 100
<212> DNA
<213> Escherichia coli K-12 MG1655
<220> ykfc b0259
<222> 1032-1131
<223> ykfc b0259 U00096 272086..273216
<313> 7098: 1061-1080
<400> 7098
GGTCTCAACG ATCCCGCAGG AGAAAGCCAG AAACTTCGCC GCATCGCTGA 50
CAGCACTGTT ATGGAAGGTG CGAATAAGCG GGGAAATTCT TCTCGGCTGA 100
<210> 7099
<211> 100
<212> DNA
<213> Escherichia coli K-12 MG1655
<220> ykfc b0259
<222> 950-1049
<223> ykfc b0259 U00096 272086..273216
<313> 7099: 983-1002
<400> 7099
AAATCCCCCA TGTTAATGAC GGCTTTATCT TTCTGGGGCA CAGGCTCATT 50
CGCAAACGCA GTCGTTATGG CGAGATGCGA GTGGTCTCAA CGATCCCGCA 100
<210> 7100
<211> 100
<212> DNA
<213> Escherichia coli K-12 MG1655
<220> ykfd b0261
<222> 1329-1428
<223> ykfd b0261 U00096 274525..275952

<313> 7100: 1395-1414
<400> 7100
GTTGTGGTGC GGGTTACCGT TTGTTGCGTT GTGCTATGGT GCTTATTTCC 50
TTACTCAACC CCGAAACGCA AAACAGGAGC CAGAACATGT CGCAGAATAA 100
<210> 7101
<211> 100
<212> DNA
<213> Escherichia coli K-12 MG1655
<220> ykfd b0261
<222> 1234-1333
<223> ykfd b0261 U00096 274525..275952
<313> 7101: 1289-1308
<400> 7101
CGCGCGCCGT GGTATCCGCT GGTGCCAGTA TTAGGTTTTG TGCTGTGCCT 50
GGTGGCCTGT GTTGGGCTGG CATTCGATCC AGCGCAGAGA ATTGCGTTGT 100
<210> 7102
<211> 100
<212> DNA
<213> Escherichia coli K-12 MG1655
<220> ykfd b0261
<222> 1163-1262
<223> ykfd b0261 U00096 274525..275952
<313> 7102: 1195-1214
<400> 7102
TCTGCGCCTC GCATTTTGTT TTTCGTCGCC GTCATCTGCA ACAAGGTAAG 50
GCATTGAGTG AATTACATTA TCGCGCGCCG TGGTATCCGC TGGTGCCAGT 100
<210> 7103
<211> 100
<212> DNA
<213> Escherichia coli K-12 MG1655
<220> ykfe b0221
<222> 347-446
<223> ykfe b0221 U00096 240343..240816
<313> 7103: 364-383
<400> 7103
AAACGTCGCA AGAGAAACTC ACCTGGCTGA ATGTGAACGA TGCGCTTTCG 50
ATTGATGGTA AAACGGTGTT GTTCGCGGCG TTGACCGGCA GCCTGGAAAA 100
<210> 7104
<211> 100
<212> DNA
<213> Escherichia coli K-12 MG1655
<220> ykfe b0221
<222> 293-392
<223> ykfe b0221 U00096 240343..240816
<313> 7104: 326-345
<400> 7104
TGTGGTCCGA GAAATCTAAT CAGATGACGG GGCTGTTCTC GACTATTGAT 50
GAGAAAACGT CGCAAGAGAA ACTCACCTGG CTGAATGTGA ACGATGCGCT 100
<210> 7105
<211> 100
<212> DNA
<213> Escherichia coli K-12 MG1655
<220> ykfe b0221
<222> 229-328
<223> ykfe b0221 U00096 240343..240816
<313> 7105: 268-287
<400> 7105
GAGACGTATC AGGTGATGAG CGCGTGCAAA CCGCATGACT GTGGCTCGCA 50
ACGTATCGCT GTGATGTGGT CCGAGAAATC TAATCAGATG ACGGGGCTGT 100
<210> 7106
<211> 100
<212> DNA
<213> Escherichia coli K-12 MG1655
<220> ykff b0250
<222> 141-240
<223> ykff b0250 U00096 complement(264528..264767)
<313> 7106: 218-237
<400> 7106
GATGGTCTGG CGGGCATGGA ACTTTGAGCC GGATGCCGGT GAAGGTCTTA 50
ACCGCTATAT CCGCACCTCA GGCATCCGTA CAGACACGGC CACCCGCTGA 100
<210> 7107

<211> 100
<212> DNA
<213> Escherichia coli K-12 MG1655
<220> ykff b0250
<222> 85-184
<223> ykff b0250 U00096 complement(264528..264767)
<313> 7107: 112-131
<400> 7107
CTCGAAGACG ACCAGGGCAG TCACTTCCGT CTGGTGGTTC GTGATACTGA 50
AGGCCGGATG GTCTGGCGGG CATGGAACTT TGAGCCGGAT GCCGGTGAAG 100
<210> 7108
<211> 100
<212> DNA
<213> Escherichia coli K-12 MG1655
<220> ykff b0250
<222> 10-109
<223> ykff b0250 U00096 complement(264528..264767)
<313> 7108: 77-96
<400> 7108
TCCGTGTTGC TGCCACCGGG GCCTTTCACC CGGAGACAAG CGCAAGCGGT 50
CACTACCACG TACAGCAATA TCACACTCGA AGACGACCAG GGCAGTCACT 100
<210> 7109
<211> 100
<212> DNA
<213> Escherichia coli K-12 MG1655
<220> ykfg b0248
<222> 378-477
<223> ykfg b0248 U00096 complement(263480..263956)
<313> 7109: 445-464
<400> 7109
ACTGGTTCAG GTGCTTCAGC TGGTGGATAT CCGTGTCCCT GACCATCTGA 50
TTGTCGGTGG CAGGCAAATC TATTCGTTCG CAGAACACGG TCTGCTTTGA 100
<210> 7110
<211> 100
<212> DNA
<213> Escherichia coli K-12 MG1655
<220> ykfg b0248
<222> 341-440
<223> ykfg b0248 U00096 complement(263480..263956)
<313> 7110: 360-379
<400> 7110
CGACACCTAG CCAGGCCGAC AAAACCCTCA CGCAGCGACT GGTTCAGGTG 50
CTTCAGCTGG TGGATATCCG TGTCCCTGAC CATCTGATTG TCGGTGGCAG 100
<210> 7111
<211> 100
<212> DNA
<213> Escherichia coli K-12 MG1655
<220> ykfg b0248
<222> 290-389
<223> ykfg b0248 U00096 complement(263480..263956)
<313> 7111: 322-341
<400> 7111
ACTTCAACGC GGCGGCGGTG ATACTCGCGC ATAACCATCC TTCCGGCGAG 50
ACGACACCTA GCCAGGCCGA CAAAACCCTC ACGCAGCGAC TGGTTCAGGT 100
<210> 7112
<211> 100
<212> DNA
<213> Escherichia coli K-12 MG1655
<220> coaa coaA
<222> 852-951
<223> coaa coaA U00096 complement(4171655..4172605)
<313> 7112: 914-933
<400> 7112
GAACTTAAAG CAAAATATTC TACCTACTCG TGAGCGCGCC AGTTTAATCC 50
TGACGAAAAG TGCTAATCAT GCGGTAGAAG AGGTCAGACT ACGCAAATAA 100
<210> 7113
<211> 100
<212> DNA
<213> Escherichia coli K-12 MG1655
<220> coaa coaA
<222> 784-883

<223> coaa coaA U00096 complement(4171655..4172605)
<313> 7113: 836-855
<400> 7113
TACGCGAAAT TAACTAAAGA AGAAGCGATT AAGACTGCCA TGACATTGTG 50
GAAAGAGATC AACTGGCTGA ACTTAAAGCA AAATATTCTA CCTACTCGTG 100
<210> 7114
<211> 100
<212> DNA
<213> Escherichia coli K-12 MG1655
<220> coaa coaA
<222> 720-819
<223> coaa coaA U00096 complement(4171655..4172605)
<313> 7114: 756-775
<400> 7114
TATCAACCGT TTTCTGAAAT TCCGCGAAGG GGCTTTTACC GACCCGGATT 50
CCTATTTTCA TAACTACGCG AAATTAACTA AAGAAGAAGC GATTAAGACT 100
<210> 7115
<211> 100
<212> DNA
<213> Escherichia coli K-12 MG1655
<220> smta b0922
<222> 643-742
<223> smta b0922 U00096 972760..973545
<313> 7115: 669-688
<400> 7115
CTGCGCGAGA AACACCAGCA GCGCGACTGC TATGAAGCAT TACTTGAATT 50
AGAAACGCGT TATTGCCGTC AGGAACCGTA TATTACCCTG GGGCGTTATA 100
<210> 7116
<211> 100
<212> DNA
<213> Escherichia coli K-12 MG1655
<220> smta b0922
<222> 559-658
<223> smta b0922 U00096 972760..973545
<313> 7116: 614-633
<400> 7116
GACCCGGCGC AGGTTTATCT GTGGCTGGAA GAAGCTGGTT GGCAAATTAT 50
GGGTAAGACA GGCGTTCGCG TGTTTCATGA TTATCTGCGC GAGAAACACC 100
<210> 7117
<211> 100
<212> DNA
<213> Escherichia coli K-12 MG1655
<220> smta b0922
<222> 518-617
<223> smta b0922 U00096 972760..973545
<313> 7117: 539-558
<400> 7117
CGAAAAAGAA AAAACGGACG CTTTCGCCAG ATTATCCACG CGACCCGGCG 50
CAGGTTTATC TGTGGCTGGA AGAAGCTGGT TGGCAAATTA TGGGTAAGAC 100
<210> 7118
<211> 100
<212> DNA
<213> Escherichia coli K-12 MG1655
<220> mhpr b0347
<222> 849-948
<223> mhpr b0347 U00096 complement(366811..367758)
<313> 7118: 879-898
<400> 7118
TCTTCCGGCG CTACAACGGG TAGCAAAACA GATCGAAGAA GGGGTTGAAT 50
CGCAGGCTAT TCTGGTGGCC GGAAGGCGAA GCGGCATGCA TTTACGTTGA 100
<210> 7119
<211> 100
<212> DNA
<213> Escherichia coli K-12 MG1655
<220> mhpr b0347
<222> 810-909
<223> mhpr b0347 U00096 complement(366811..367758)
<313> 7119: 838-857
<400> 7119
GGCGAGCGCA ATGACCATTG AACAGGCAGC GGAAAAGCAT CTTCCGGCGC 50
TACAACGGGT AGCAAAACAG ATCGAAGAAG GGGTTGAATC GCAGGCTATT 100

<210> 7120
<211> 100
<212> DNA
<213> Escherichia coli K-12 MG1655
<220> mhpr b0347
<222> 755-854
<223> mhpr b0347 U00096 complement(366811..367758)
<313> 7120: 800-819
<400> 7120
CCGTACCGCT GCGCAGTGAA CAACGGGTGA TTGGCTGTCT GAATCTGGTG 50
TATATGGCGA GCGCAATGAC CATTGAACAG GCAGCGGAAA AGCATCTTCC 100
<210> 7121
<211> 100
<212> DNA
<213> Escherichia coli K-12 MG1655
<220> mhpt b0354
<222> 1158-1257
<223> mhpt b0354 U00096 374638..375894
<313> 7121: 1225-1244
<400> 7121
CGTAATGGCC GCTTCTGCAC CGGGTATTCT TGTTGCTGGG TTGGCGGTGT 50
TTATTTTGAT GAGCCGGAGA TCACGAATAC AGCCGTGCGC CGATGCCTGA 100
<210> 7122
<211> 100
<212> DNA
<213> Escherichia coli K-12 MG1655
<220> mhpt b0354
<222> 1158-1257
<223> mhpt b0354 U00096 374638..375894
<313> 7122: 1185-1204
<400> 7122
CGTAATGGCC GCTTCTGCAC CGGGTATTCT TGTTGCTGGG TTGGCGGTGT 50
TTATTTTGAT GAGCCGGAGA TCACGAATAC AGCCGTGCGC CGATGCCTGA 100
<210> 7123
<211> 100
<212> DNA
<213> Escherichia coli K-12 MG1655
<220> mhpt b0354
<222> 1123-1222
<223> mhpt b0354 U00096 374638..375894
<313> 7123: 1144-1163
<400> 7123
GGGAAAATGC TGGCATTAGG CACTGGCACG GTCGGCGTAA TGGCCGCTTC 50
TGCACCGGGT ATTCTTGTTG CTGGGTTGGC GGTGTTTATT TTGATGAGCC 100
<210> 7124
<211> 100
<212> DNA
<213> Escherichia coli K-12 MG1655
<220> sfca b1480
<222> 1626-1725
<223> sfca b1480 U00096 complement(1551996..1553720)
<313> 7124: 1688-1707
<400> 7124
AGGCGTGGCG GTGAAAACCT CTGCCGAAGC CCTGCAACAG GCCATTGACG 50
ATAATTTCTG GCAAGCCGAA TACCGCGACT ACCGCCGTAC CTCCATCTAA 100
<210> 7125
<211> 100
<212> DNA
<213> Escherichia coli K-12 MG1655
<220> sfca b1480
<222> 1598-1697
<223> sfca b1480 U00096 complement(1551996..1553720)
<313> 7125: 1662-1681
<400> 7125
TTGCGGTTGG CAAAATGGCG CAGCAGCAAG GCGTGGCGGT GAAAACCTCT 50
GCCGAAGCCC TGCAACAGGC CATTGACGAT AATTTCTGGC AAGCCGAATA 100
<210> 7126
<211> 100
<212> DNA
<213> Escherichia coli K-12 MG1655
<220> sfca b1480

<222> 1563-1662
<223> sfca b1480 U00096 complement(1551996..1553720)
<313> 7126: 1616-1635
<400> 7126
GAAAGATATT CAGAAAGTCT CCCGCGCAAT TGCGTTTGCG GTTGGCAAAA 50
TGGCGCAGCA GCAAGGCGTG GCGGTGAAAA CCTCTGCCGA AGCCCTGCAA 100
<210> 7127
<211> 100
<212> DNA
<213> Escherichia coli K-12 MG1655
<220> ykga b0301
<222> 621-720
<223> ykga b0301 U00096 complement(315674..316393)
<313> 7127: 674-693
<400> 7127
TTTTGCTGGT ACGGTATGTG AATATGCTGA CATGCCAAAA GAGTGGACAA 50
CGACCCGAGG ACTTTATGAT CCTACCCACG TAATATGGAC ACAGGCCTAA 100
<210> 7128
<211> 100
<212> DNA
<213> Escherichia coli K-12 MG1655
<220> ykga b0301
<222> 562-661
<223> ykga b0301 U00096 complement(315674..316393)
<313> 7128: 611-630
<400> 7128
TATTTCGTTT CCACGTTTGA ACCGTCAACA AAATCGGTCG ATTTGCTCAC 50
GGTTGAAACT TTTGCTGGTA CGGTATGTGA ATATGCTGAC ATGCCAAAAG 100
<210> 7129
<211> 100
<212> DNA
<213> Escherichia coli K-12 MG1655
<220> ykga b0301
<222> 303-402
<223> ykga b0301 U00096 complement(315674..316393)
<313> 7129: 334-353
<400> 7129
CGAATTTAAA AAGTTATTCG GCTGCTCTCC CCGTGAATAC CGCCACCGTG 50
ATTATTGGGA TCTCGCAAAT ATCTTCCCTT CTTTTTTAAT ACGTCAACAG 100
<210> 7130
<211> 100
<212> DNA
<213> Escherichia coli K-12 MG1655
<220> ykgb ykgB
<222> 504-603
<223> ykgb ykgB U00096 complement(316950..317552)
<313> 7130: 560-579
<400> 7130
GATGCTGGCA GGTGCAGTCA TGATAATGGC AGATTCGGCG CGGGAAATTC 50
TTAAACAACG CAGTAATGAA TCCAGTTCAA CGTTAAAAAC TGAATATTGA 100
<210> 7131
<211> 100
<212> DNA
<213> Escherichia coli K-12 MG1655
<220> ykgb ykgB
<222> 351-450
<223> ykgb ykgB U00096 complement(316950..317552)
<313> 7131: 407-426
<400> 7131
TTTATTGGGA GGACTGATGG CATTTACCAC GCCGCTGGTA ACACTCTCAT 50
TTTTAATCAC CACCCCGGAG GCATGGGTAC CCGCATTGGG TGACGCTCAT 100
<210> 7132
<211> 100
<212> DNA
<213> Escherichia coli K-12 MG1655
<220> ykgb ykgB
<222> 323-422
<223> ykgb ykgB U00096 complement(316950..317552)
<313> 7132: 360-379
<400> 7132
TGGCTAATCC TGTCAATCGC TGGTTAGGTT TATTGGGAGG ACTGATGGCA 50

TTTACCACGC CGCTGGTAAC ACTCTCATTT TTAATCACCA CCCCGGAGGC 100
<210> 7133
<211> 100
<212> DNA
<213> Escherichia coli K-12 MG1655
<220> ykgc b0305
<222> 1210-1309
<223> ykgc b0305 U00096 complement(317900..319252)
<313> 7133: 1270-1289
<400> 7133
CTGTGTGTTG ACTCCCACGA GATGATCAAT ATAGTGAAAA TGGTGATGGA 50
TGCCGGGCTG CCTTATAGCA TATTACGCGA TCAGATATTT ACTCATCCGT 100
<210> 7134
<211> 100
<212> DNA
<213> Escherichia coli K-12 MG1655
<220> ykgc b0305
<222> 1168-1267
<223> ykgc b0305 U00096 complement(317900..319252)
<313> 7134: 1212-1231
<400> 7134
ATTGTTGATA ATAAAACCCA ACGTATGTTA GGGGCATCAC TGCTGTGTGT 50
TGACTCCCAC GAGATGATCA ATATAGTGAA AATGGTGATG GATGCCGGGC 100
<210> 7135
<211> 100
<212> DNA
<213> Escherichia coli K-12 MG1655
<220> ykgc b0305
<222> 1094-1193
<223> ykgc b0305 U00096 complement(317900..319252)
<313> 7135: 1132-1151
<400> 7135
AGGTGGTGAC ATTGCCTGTA GCTGCAATTC CGCGTGCCAG AGTGATGAAT 50
GATACTCGTG GGGTATTAAA AGCGATTGTT GATAATAAAA CCCAACGTAT 100
<210> 7136
<211> 100
<212> DNA
<213> Escherichia coli K-12 MG1655
<220> ykgd b0306
<222> 731-830
<223> ykgd b0306 U00096 319451..320305
<313> 7136: 794-813
<400> 7136
CTGTTGTGGT GATCGCTGAG TCAGTAGGCT ATGCCAGTGA ATCATCTTTT 50
CACAAGGCGT TTGTCCGCGA GTTTGGTTGT ACCCCGGGAG AATATCGGGA 100
<210> 7137
<211> 100
<212> DNA
<213> Escherichia coli K-12 MG1655
<220> ykgd b0306
<222> 647-746
<223> ykgd b0306 U00096 319451..320305
<313> 7137: 689-708
<400> 7137
ATGTTTCCGG AACCACGCCG CTGGCTGTAT TAACAAAGTT GCGTCTACAA 50
ATAGCGGCCC AGATGTTTTC CCGGGAAACG CTCCCTGTTG TGGTGATCGC 100
<210> 7138
<211> 100
<212> DNA
<213> Escherichia coli K-12 MG1655
<220> ykgd b0306
<222> 513-612
<223> ykgd b0306 U00096 319451..320305
<313> 7138: 563-582
<400> 7138
CAGTTTGCTT CTGCATCCAC GTCTTGGTGC GGTAATACAG CAAATGCTGG 50
AAATGCCAGG ACACGCCTGG ACCGTCGAAT CGCTGGCCAG CATCGCTCAC 100
<210> 7139
<211> 100
<212> DNA
<213> Escherichia coli K-12 MG1655

<220> ykge b0307
<222> 621-720
<223> ykge b0307 U00096 320832..321551
<313> 7139: 696-715
<400> 7139
TGCTGACGTG AGTTGCCTGC TAAACATCAG TGGGCGATTA CAACGGGAAG 50
GGCAGAAAGT CAAAGTGATG CATATTGCTG AAGTGTTGAT GAGCCGCTGA 100
<210> 7140
<211> 100
<212> DNA
<213> Escherichia coli K-12 MG1655
<220> ykge b0307
<222> 596-695
<223> ykge b0307 U00096 320832..321551
<313> 7140: 647-666
<400> 7140
AAGTCCGCCC TGAGTATTTA ATTGGTGCTG ACGTGAGTTG CCTGCTAAAC 50
ATCAGTGGGC GATTACAACG GGAAGGGCAG AAAGTCAAAG TGATGCATAT 100
<210> 7141
<211> 100
<212> DNA
<213> Escherichia coli K-12 MG1655
<220> ykge b0307
<222> 507-606
<223> ykge b0307 U00096 320832..321551
<313> 7141: 556-575
<400> 7141
TACCTGCTGC GGATTTGGCG GCACGTTCTC GGTCAAAATG GCCGAAATAT 50
CCGGCGAGAT GGTGAAAGAA AAGGTTGCGC ACCTGATGGA AGTCCGCCCT 100
<210> 7142
<211> 100
<212> DNA
<213> Escherichia coli K-12 MG1655
<220> ykgf b0308
<222> 1208-1307
<223> ykgf b0308 U00096 321562..322989
<313> 7142: 1264-1283
<400> 7142
TAAAAATGTT CGCTTATGCC AATAGTCATC CAGGATTGTG GAAAGTCGGG 50
ATGATGGCCG GTGCTCATGC GGCAAGCTGG TTTATCAATG GCGGCAAAAC 100
<210> 7143
<211> 100
<212> DNA
<213> Escherichia coli K-12 MG1655
<220> ykgf b0308
<222> 1165-1264
<223> ykgf b0308 U00096 321562..322989
<313> 7143: 1205-1224
<400> 7143
ATGGCTGAAA AAGGGATCAC CGCAAAAGCA GAGCAACGGG CGATAAAAAT 50
GTTCGCTTAT GCCAATAGTC ATCCAGGATT GTGGAAAGTC GGGATGATGG 100
<210> 7144
<211> 100
<212> DNA
<213> Escherichia coli K-12 MG1655
<220> ykgf b0308
<222> 1114-1213
<223> ykgf b0308 U00096 321562..322989
<313> 7144: 1131-1150
<400> 7144
TGTCCGGTGC GTATTCCGCT GTCAAAACTG ATTTTGCGTC ATCGTCGGGT 50
GATGGCTGAA AAAGGGATCA CCGCAAAAGC AGAGCAACGG GCGATAAAAA 100
<210> 7145
<211> 100
<212> DNA
<213> Escherichia coli K-12 MG1655
<220> ykgg ykgG
<222> 750-849
<223> ykgg ykgG U00096 322829..323677
<313> 7145: 825-844
<400> 7145

CATTAGCGGC CCCAGTTCAA CGGCGGATAT TGAGCTTATC AAAGTCGTCG 50
GAGTTCATGG CCCGGTGAAA GCGGTGTATC TGATTATTGA GGATTGTTGA 100
<210> 7146
<211> 100
<212> DNA
<213> Escherichia coli K-12 MG1655
<220> ykgg ykgG
<222> 695-794
<223> ykgg ykgG U00096 322829..323677
<313> 7146: 743-762
<400> 7146
AAAAATTGCA TCAGAAAGCG CAGGCCGGTG AACGAATGCC TTCCTGCATT 50
AACATCATTA GCGGCCCCAG TTCAACGGCG GATATTGAGC TTATCAAAGT 100
<210> 7147
<211> 100
<212> DNA
<213> Escherichia coli K-12 MG1655
<220> ykgg ykgG
<222> 665-764
<223> ykgg ykgG U00096 322829..323677
<313> 7147: 702-721
<400> 7147
CTATCCTGCC GCGTGTAGCG CAACTCGCAG AAAAATTGCA TCAGAAAGCG 50
CAGGCCGGTG AACGAATGCC TTCCTGCATT AACATCATTA GCGGCCCCAG 100
<210> 7148
<211> 100
<212> DNA
<213> Escherichia coli K-12 MG1655
<220> ykgh b0311
<222> 530-629
<223> ykgh b0311 U00096 complement(323920..324588)
<313> 7148: 574-593
<400> 7148
TAAGCTCACC AGAAATAAAA AATAAATACC CTGCTGCCAT TTGCCTGGCT 50
CTGGGATTTT TTCTAAGCAT TGTAATTTCT GTAATGTTTT GCCTTGTCAA 100
<210> 7149
<211> 100
<212> DNA
<213> Escherichia coli K-12 MG1655
<220> ykgh b0311
<222> 463-562
<223> ykgh b0311 U00096 complement(323920..324588)
<313> 7149: 520-539
<400> 7149
TATACAAAAA TGATATACCT GACCAGGAAC TGGTCATTGT ATCCGAACGG 50
TGATGGCTGT GTAACTATAA GCTCACCAGA AATAAAAAAT AAATACCCTG 100
<210> 7150
<211> 100
<212> DNA
<213> Escherichia coli K-12 MG1655
<220> ykgh b0311
<222> 388-487
<223> ykgh b0311 U00096 complement(323920..324588)
<313> 7150: 426-445
<400> 7150
TATGCGTCAG AATATCATAA TGGCAGGCAA GTTCTCGATG AGATACAAAC 50
GCCTTCAGCA ATTAACAGTG AGCTTTATAC AAAAATGATA TACCTGACCA 100
<210> 7151
<211> 100
<212> DNA
<213> Escherichia coli K-12 MG1655
<220> ykgi b0304
<222> 153-252
<223> ykgi b0304 U00096 complement(317555..317806)
<313> 7151: 207-226
<400> 7151
CATTGCTGAT ATAGCCAGAA AATATAATGC TTCATCCTGG AAAGTCACAT 50
CGATGCGAAT TGATAATAAT TCAACCGCAA CAGCAGTATT GTATAAATAA 100
<210> 7152
<211> 100
<212> DNA

<213> Escherichia coli K-12 MG1655
<220> ykgi b0304
<222> 136-235
<223> ykgi b0304 U00096 complement(317555..317806)
<313> 7152: 169-188
<400> 7152
ACAGTTCTGG AGGATACCAT TGCTGATATA GCCAGAAAAT ATAATGCTTC 50
ATCCTGGAAA GTCACATCGA TGCGAATTGA TAATAATTCA ACCGCAACAG 100
<210> 7153
<211> 100
<212> DNA
<213> Escherichia coli K-12 MG1655
<220> ykgi b0304
<222> 138-237
<223> ykgi b0304 U00096 complement(317555..317806)
<313> 7153: 166-185
<400> 7153
AGTTCTGGAG GATACCATTG CTGATATAGC CAGAAAATAT AATGCTTCAT 50
CCTGGAAAGT CACATCGATG CGAATTGATA ATAATTCAAC CGCAACAGCA 100
<210> 7154
<211> 100
<212> DNA
<213> Escherichia coli K-12 MG1655
<220> ykgj b0289
<222> 231-330
<223> ykgj b0289 U00096 complement(303077..303406)
<313> 7154: 296-315
<400> 7154
ATCGTCCACA TGCAGAGAAT TCGCCATGTC TGGTGAGAAC GGAGTCGTCA 50
ATGAGGCTTG CAATCGTGCA AGGGCTAAAT ACGGGCTGAC ACCGTTATAA 100
<210> 7155
<211> 100
<212> DNA
<213> Escherichia coli K-12 MG1655
<220> ykgj b0289
<222> 74-173
<223> ykgj b0289 U00096 complement(303077..303406)
<313> 7155: 129-148
<400> 7155
AAGCCGACGA TGCTGGCGGT ACTATTCCCG CCAGGCTCAC TGAACAAATA 50
TCCCCTTTTC ACCGATGCAT GAGCGGTACC AATCAGAAAA ACCCCCGATG 100
<210> 7156
<211> 100
<212> DNA
<213> Escherichia coli K-12 MG1655
<220> ykgj b0289
<222> 17-116
<223> ykgj b0289 U00096 complement(303077..303406)
<313> 7156: 55-74
<400> 7156
CATGCATGAC GTGTGGTGCC TGTTGTGCAT TTTTCCGCGT CTCTTTTTAC 50
TGGGCCGAAG CCGACGATGC TGGCGGTACT ATTCCCGCCA GGCTCACTGA 100
<210> 7157
<211> 100
<212> DNA
<213> Escherichia coli K-12 MG1655
<220> ykgk b0295
<222> 411-510
<223> ykgk b0295 U00096 complement(309970..310560)
<313> 7157: 469-488
<400> 7157
AAAAGATATT AAGAAAGATA AAATCACGGA CCGGGAAATG GAAATTATCC 50
GCATGACGGC CCAGGGAATG CAACCTAAAT CGATTGCCAG AATTGAAAAT 100
<210> 7158
<211> 100
<212> DNA
<213> Escherichia coli K-12 MG1655
<220> ykgk b0295
<222> 298-397
<223> ykgk b0295 U00096 complement(309970..310560)
<313> 7158: 339-358

<400> 7158
GCCTTAGCAA ATTATTGGTA TTACAATAGC AATATTAGGG GCGTGGTATA 50
CGCTGGACTG AGTCGTGATA TTAGAAAAGA ACTGGTCTAT GTGATTAATG 100
<210> 7159
<211> 100
<212> DNA
<213> Escherichia coli K-12 MG1655
<220> ykgk b0295
<222> 18-117
<223> ykgk b0295 U00096 complement(309970..310560)
<313> 7159: 75-94
<400> 7159
TTACAGTAGG GACTATGAGG TTAAAAACCA TATGGAATGT CAAAACCGTT 50
CTGATAAATA CATCTGGTCT CCCCATGACG CCTACTTCTA TAAAGGACTA 100
<210> 7160
<211> 100
<212> DNA
<213> Escherichia coli K-12 MG1655
<220> cobb cobB
<222> 741-840
<223> cobb cobB U00096 1178743..1179582
<313> 7160: 815-834
<400> 7160
TAATGAATTT GCCGAGAAAT ATTACGGCCC GGCAAGCCAG GTGGTGCCTG 50
AGTTTGTTGA AAAGTTGCTG AAGGGATTAA AAGCGGGAAG CATTGCCTGA 100
<210> 7161
<211> 100
<212> DNA
<213> Escherichia coli K-12 MG1655
<220> cobb cobB
<222> 737-836
<223> cobb cobB U00096 1178743..1179582
<313> 7161: 760-779
<400> 7161
TTGGTAATGA ATTTGCCGAG AAATATTACG GCCCGGCAAG CCAGGTGGTG 50
CCTGAGTTTG TTGAAAAGTT GCTGAAGGGA TTAAAAGCGG GAAGCATTGC 100
<210> 7162
<211> 100
<212> DNA
<213> Escherichia coli K-12 MG1655
<220> cobb cobB
<222> 665-764
<223> cobb cobB U00096 1178743..1179582
<313> 7162: 716-735
<400> 7162
CGGCTGGGTT TGTTCACGAA GCGAAACTGC ATGGCGCGCA CACCGTGGAA 50
CTGAATCTTG AACCGAGTCA GGTTGGTAAT GAATTTGCCG AGAAATATTA 100
<210> 7163
<211> 100
<212> DNA
<213> Escherichia coli K-12 MG1655
<220> ykgl b0296
<222> 119-218
<223> ykgl b0296 U00096 311336..311563
<313> 7163: 137-156
<400> 7163
CGGAGAGAAA CGTCAGGCAG TTGGCCTCAA ATGAACAGGA AGAATATATC 50
CCCAGGAAAA TTAATGTTGG TGTTATAAAC ACACCAACAT TAATCCGATC 100
<210> 7164
<211> 100
<212> DNA
<213> Escherichia coli K-12 MG1655
<220> ykgl b0296
<222> 37-136
<223> ykgl b0296 U00096 311336..311563
<313> 7164: 91-110
<400> 7164
CGTGTTCAGT CTAAAAGAGG GTTGATGCCA GCATACAGGG GCGAAGCTGG 50
TCAACAAGTC AACATTAAGA TAATGGAATA TTCGGAGAGA AACGTCAGGC 100
<210> 7165
<211> 100

<212> DNA
<213> Escherichia coli K-12 MG1655
<220> ykgl b0296
<222> 8-107
<223> ykgl b0296 U00096 311336..311563
<313> 7165: 37-56
<400> 7165
CTGCGCCGCA AAGGGAGGAG ACTGAGTGGC GTGTTCAGTC TAAAAGAGGG 50
TTGATGCCAG CATACAGGGG CGAAGCTGGT CAACAAGTCA ACATTAAGAT 100
<210> 7166
<211> 100
<212> DNA
<213> Escherichia coli K-12 MG1655
<220> ykgm b0297
<222> 165-264
<223> ykgm b0297 U00096 complement(311738..312001)
<313> 7166: 209-228
<400> 7166
GTTCTATACA GGGAAGCTGA GAACAGTGGC ATCAGAAGGA AATGTTGCAC 50
GATTCACCCA ACGTTTTGGT CGTTTTGTTA GCACGAAAAA GGGGGCGTGA 100
<210> 7167
<211> 100
<212> DNA
<213> Escherichia coli K-12 MG1655
<220> ykgm b0297
<222> 149-248
<223> ykgm b0297 U00096 complement(311738..312001)
<313> 7167: 177-196
<400> 7167
CTTCTAAATC GCACCCGTTC TATACAGGGA AGCTGAGAAC AGTGGCATCA 50
GAAGGAAATG TTGCACGATT CACCCAACGT TTTGGTCGTT TTGTTAGCAC 100
<210> 7168
<211> 100
<212> DNA
<213> Escherichia coli K-12 MG1655
<220> ykgm b0297
<222> 104-203
<223> ykgm b0297 U00096 complement(311738..312001)
<313> 7168: 143-162
<400> 7168
TTGAGCTGGA TGGCGTAACG TATCCATACG TGACAATTGA TGTCTCTTCT 50
AAATCGCACC CGTTCTATAC AGGGAAGCTG AGAACAGTGG CATCAGAAGG 100
<210> 7169
<211> 100
<212> DNA
<213> Escherichia coli K-12 MG1655
<220> cobs b1993
<222> 645-744
<223> cobs b1993 U00096 complement(2062501..2063244)
<313> 7169: 695-714
<400> 7169
CGGTCAGCTT CTCAAACGTA CGCTGGGCGG GCAAACGGGC GATACGCTGG 50
GAGCAGCCAT AGAGCTTGGT GAACTGGTCT TCCTGCTGGC ACTGCTATAA 100
<210> 7170
<211> 100
<212> DNA
<213> Escherichia coli K-12 MG1655
<220> cobs b1993
<222> 615-714
<223> cobs b1993 U00096 complement(2062501..2063244)
<313> 7170: 635-654
<400> 7170
GGTGGTGACG ATGGTGGCGA TTTTCATCCT CGGTCAGCTT CTCAAACGTA 50
CGCTGGGCGG GCAAACGGGC GATACGCTGG GAGCAGCCAT AGAGCTTGGT 100
<210> 7171
<211> 100
<212> DNA
<213> Escherichia coli K-12 MG1655
<220> cobs b1993
<222> 519-618
<223> cobs b1993 U00096 complement(2062501..2063244)

<313> 7171: 568-587
<400> 7171
TGGCAAAATT GATGGGCGAC AAACCTGCGT CACACTCGGC TTAGCCGCTA 50
TTTTTGCTGC CGTATTATTG CCGGGTATGC ATGGTGTGGC TGCTATGGTG 100
<210> 7172
<211> 100
<212> DNA
<213> Escherichia coli K-12 MG1655
<220> cobt b1992
<222> 952-1051
<223> cobt b1992 U00096 complement(2061410..2062489)
<313> 7172: 1008-1027
<400> 7172
GAGGGGAGTG GCGCAGCTCT GGCGATGCCC ATCATCGAAG CTGCCTGTGC 50
GATATACAAC AACATGGGCG AACTTGCTGC CAGTAATATT GTTCTACCGG 100
<210> 7173
<211> 100
<212> DNA
<213> Escherichia coli K-12 MG1655
<220> cobt b1992
<222> 914-1013
<223> cobt b1992 U00096 complement(2061410..2062489)
<313> 7173: 959-978
<400> 7173
GGCTGGAGCC TTATCTCAAT ATGGAGATGC GTTTAGGTGA GGGGAGTGGC 50
GCAGCTCTGG CGATGCCCAT CATCGAAGCT GCCTGTGCGA TATACAACAA 100
<210> 7174
<211> 100
<212> DNA
<213> Escherichia coli K-12 MG1655
<220> cobt b1992
<222> 812-911
<223> cobt b1992 U00096 complement(2061410..2062489)
<313> 7174: 844-863
<400> 7174
CTGCGCTCGC AGCCTGCCAG ATGTCTCCTG CGATCAAACC GTATCTCATT 50
CCTTCTCACC TGTCGGCAGA AAAAGGCGCG CGTATAGCGC TCTCGCATTT 100
<210> 7175
<211> 100
<212> DNA
<213> Escherichia coli K-12 MG1655
<220> cobu b1994
<222> 447-546
<223> cobu b1994 U00096 complement(2063241..2063786)
<313> 7175: 497-516
<400> 7175
ACATTTTCGT GATATTGCCG GGCGGGTAAA TCAGCAGTTG GCCGCTGCGG 50
CAAATGAAGT ATGGCTGGTG GTTTCGGGTA TTGGAGTAAA AATCAAATGA 100
<210> 7176
<211> 100
<212> DNA
<213> Escherichia coli K-12 MG1655
<220> cobu b1994
<222> 390-489
<223> cobu b1994 U00096 complement(2063241..2063786)
<313> 7176: 408-427
<400> 7176
TGTATTAGTG ACTAACGAAG TGGGAATGGG GATTGTGCCG GAGAGTCGTC 50
TGGCACGACA TTTTCGTGAT ATTGCCGGGC GGGTAAATCA GCAGTTGGCC 100
<210> 7177
<211> 100
<212> DNA
<213> Escherichia coli K-12 MG1655
<220> cobu b1994
<222> 317-416
<223> cobu b1994 U00096 complement(2063241..2063786)
<313> 7177: 352-371
<400> 7177
AGGCGATGGA ACAGGCGATT AATGCTGAGA TTCAGTCGTT GATTGCTGCC 50
TGCCAACGTT GCCCCGCAAA GGTTGTATTA GTGACTAACG AAGTGGGAAT 100
<210> 7178

<211> 100
<212> DNA
<213> Escherichia coli K-12 MG1655
<220> fuma b1613
<222> 1548-1647
<223> fuma b1613 U00096 complement(1684755..1686401)
<313> 7178: 1600-1619
<400> 7178
TGAAGTGGAA GATTTCCCGG CGTTTATCCT TGTGGATGAT AAAGGAAATG 50
ACTTCTTCCA GCAGATACAA CTCACACAAT GCACCCGCTG TGTGAAATAA 100
<210> 7179
<211> 100
<212> DNA
<213> Escherichia coli K-12 MG1655
<220> fuma b1613
<222> 1525-1624
<223> fuma b1613 U00096 complement(1684755..1686401)
<313> 7179: 1564-1583
<400> 7179
GGAATGGAAG CCATCTGGAA AATTGAAGTG GAAGATTTCC CGGCGTTTAT 50
CCTTGTGGAT GATAAAGGAA ATGACTTCTT CCAGCAGATA CAACTCACAC 100
<210> 7180
<211> 100
<212> DNA
<213> Escherichia coli K-12 MG1655
<220> fuma b1613
<222> 1435-1534
<223> fuma b1613 U00096 complement(1684755..1686401)
<313> 7180: 1495-1514
<400> 7180
GGCTTCTACC TTGGCAGTAT CGGTGGTCCG GCCGCTGTAT TGGCGCAGGG 50
AAGTATTAAG AGCCTGGAAT GTGTTGAATA TCCGGAACTG GGAATGGAAG 100
<210> 7181
<211> 100
<212> DNA
<213> Escherichia coli K-12 MG1655
<220> fumb b4123
<222> 1548-1647
<223> fumb b4123 U00096 complement(4343258..4344904)
<313> 7181: 1615-1634
<400> 7181
CGAAGTAGAA GATTTCCCGG CGTTTATCCT GGTCGATGAC AAAGGTAACG 50
ACTTCTTCCA GCAAATCGTC AACAAACAGT GCGCGAACTG CACTAAGTAA 100
<210> 7182
<211> 100
<212> DNA
<213> Escherichia coli K-12 MG1655
<220> fumb b4123
<222> 1546-1645
<223> fumb b4123 U00096 complement(4343258..4344904)
<313> 7182: 1569-1588
<400> 7182
ATCGAAGTAG AAGATTTCCC GGCGTTTATC CTGGTCGATG ACAAAGGTAA 50
CGACTTCTTC CAGCAAATCG TCAACAAACA GTGCGCGAAC TGCACTAAGT 100
<210> 7183
<211> 100
<212> DNA
<213> Escherichia coli K-12 MG1655
<220> fumb b4123
<222> 1496-1595
<223> fumb b4123 U00096 complement(4343258..4344904)
<313> 7183: 1525-1544
<400> 7183
ATCTGGAGTG CGTCGCTTAT CCGGAGCTGG GTATGGAAGC TATCTGGAAA 50
ATCGAAGTAG AAGATTTCCC GGCGTTTATC CTGGTCGATG ACAAAGGTAA 100
<210> 7184
<211> 100
<212> DNA
<213> Escherichia coli K-12 MG1655
<220> fumc b1612
<222> 1305-1404

<223> fumc b1612 U00096 complement(1683209..1684612)
<313> 7184: 1371-1390
<400> 7184
AAAAGCTGCG GCCCTTGCGC TGGGGTATCT TAGCGAAGCC GAGTTTGACA 50
GCTGGGTACG GCCAGAACAG ATGGTCGGCA GTATGAAAGC CGGGCGTTAA 100
<210> 7185
<211> 100
<212> DNA
<213> Escherichia coli K-12 MG1655
<220> fumc b1612
<222> 1238-1337
<223> fumc b1612 U00096 complement(1683209..1684612)
<313> 7185: 1286-1305
<400> 7185
ACACCCACAT TGGTTATGAC AAAGCCGCCG AGATCGCCAA AAAAGCGCAT 50
AAAGAAGGGC TGACCTTAAA AGCTGCGGCC CTTGCGCTGG GGTATCTTAG 100
<210> 7186
<211> 100
<212> DNA
<213> Escherichia coli K-12 MG1655
<220> fumc b1612
<222> 1176-1275
<223> fumc b1612 U00096 complement(1683209..1684612)
<313> 7186: 1220-1239
<400> 7186
ACCGAATCGT GAGCGAATCA ATCAATTACT CAATGAATCG CTGATGCTGG 50
TGACTGCGCT TAACACCCAC ATTGGTTATG ACAAAGCCGC CGAGATCGCC 100
<210> 7187
<211> 100
<212> DNA
<213> Escherichia coli K-12 MG1655
<220> ilva b3773
<222> 1446-1545
<223> ilva b3773 U00096 3952952..3954496
<313> 7187: 1515-1534
<400> 7187
CGACCATGAA CCGGATTTCG AAACCCGGCT GAATGAGCTG GGCTACGATT 50
GCCACGACGA AACCAATAAC CCGGCGTTCA GGTTCTTTTT GGCGGGTTAG 100
<210> 7188
<211> 100
<212> DNA
<213> Escherichia coli K-12 MG1655
<220> ilva b3773
<222> 1253-1352
<223> ilva b3773 U00096 3952952..3954496
<313> 7188: 1295-1314
<400> 7188
ACGTGCGCTA TATGGTCGGC GGACGTCCAT CGCATCCGTT GCAGGAACGC 50
CTCTACAGCT TCGAATTCCC GGAATCACCG GGCGCGCTGC TGCGCTTCCT 100
<210> 7189
<211> 100
<212> DNA
<213> Escherichia coli K-12 MG1655
<220> ilva b3773
<222> 1195-1294
<223> ilva b3773 U00096 3952952..3954496
<313> 7189: 1222-1241
<400> 7189
CTCAACGACG GCGGCTACAG CGTGGTTGAT CTCTCCGACG ACGAAATGGC 50
GAAGCTACAC GTGCGCTATA TGGTCGGCGG ACGTCCATCG CATCCGTTGC 100
<210> 7190
<211> 100
<212> DNA
<213> Escherichia coli K-12 MG1655
<220> ilvb b3672
<222> 1529-1628
<223> ilvb b3672 U00096 complement(3848723..3850411)
<313> 7190: 1564-1583
<400> 7190
AAACCTGTGA TTTGAATAAC GAAGCCGATC CGCAGGCTTC ATTGCAGGAA 50
ATCATCAATC GCCCTGGCCC GGCGCTGATC CATGTGCGCA TTGATGCCGA 100

<210> 7191
<211> 100
<212> DNA
<213> Escherichia coli K-12 MG1655
<220> ilvb b3672
<222> 1488-1587
<223> ilvb b3672 U00096 complement(3848723..3850411)
<313> 7191: 1540-1559
<400> 7191
CAAAATCAAC TTTATGCAGA TTGCCGCCGG ATTCGGCCTC GAAACCTGTG 50
ATTTGAATAA CGAAGCCGAT CCGCAGGCTT CATTGCAGGA AATCATCAAT 100
<210> 7192
<211> 100
<212> DNA
<213> Escherichia coli K-12 MG1655
<220> ilvb b3672
<222> 1446-1545
<223> ilvb b3672 U00096 complement(3848723..3850411)
<313> 7192: 1481-1500
<400> 7192
TCTGTTCTAC GAGCAAGGCG TTTTTGCCGC CACCTATCCG GGCAAAATCA 50
ACTTTATGCA GATTGCCGCC GGATTCGGCC TCGAAACCTG TGATTTGAAT 100
<210> 7193
<211> 100
<212> DNA
<213> Escherichia coli K-12 MG1655
<220> grpe b2615
<222> 459-558
<223> grpe b2615 U00096 complement(2748136..2748729)
<313> 7193: 523-542
<400> 7193
GCATCAGGCC ATCGCAATGG TGGAATCTGA TGACGTTGCG CCAGGTAACG 50
TACTGGGCAT TATGCAGAAG GGTTATACGC TGAATGGTCG TACGATTCGT 100
<210> 7194
<211> 100
<212> DNA
<213> Escherichia coli K-12 MG1655
<220> grpe b2615
<222> 322-421
<223> grpe b2615 U00096 complement(2748136..2748729)
<313> 7194: 362-381
<400> 7194
GTGGCTGATA AAGCTAACCC GGATATGTCT GCGATGGTTG AAGGCATTGA 50
GCTGACGCTG AAGTCGATGC TGGATGTTGT GCGTAAGTTT GGCGTTGAAG 100
<210> 7195
<211> 100
<212> DNA
<213> Escherichia coli K-12 MG1655
<220> grpe b2615
<222> 297-396
<223> grpe b2615 U00096 complement(2748136..2748729)
<313> 7195: 310-329
<400> 7195
TGATAGCCTG GATCGTGCGC TGGAAGTGGC TGATAAAGCT AACCCGGATA 50
TGTCTGCGAT GGTTGAAGGC ATTGAGCTGA CGCTGAAGTC GATGCTGGAT 100
<210> 7196
<211> 100
<212> DNA
<213> Escherichia coli K-12 MG1655
<220> ilvc b3775
<222> 1377-1476
<223> ilvc b3775 U00096 3955591..3957066
<313> 7196: 1438-1457
<400> 7196
TGATGTGAAC GAAGCGATTC GCAGCCATGC GATTGAGCAG GTAGGTAAGA 50
AACTGCGCGG CTATATGACA GATATGAAAC GTATTGCTGT TGCGGGTTAA 100
<210> 7197
<211> 100
<212> DNA
<213> Escherichia coli K-12 MG1655
<220> ilvc b3775

<222> 1363-1462
<223> ilvc b3775 U00096 3955591..3957066
<313> 7197: 1400-1419
<400> 7197
AACGGGCAAC TGCGTGATGT GAACGAAGCG ATTCGCAGCC ATGCGATTGA 50
GCAGGTAGGT AAGAAACTGC GCGGCTATAT GACAGATATG AAACGTATTG 100
<210> 7198
<211> 100
<212> DNA
<213> Escherichia coli K-12 MG1655
<220> ilvc b3775
<222> 1272-1371
<223> ilvc b3775 U00096 3955591..3957066
<313> 7198: 1307-1326
<400> 7198
CTCTTACGCT TGTGTGCCGT TGCTGAAACC GTTTATGGCA GAGCTGCAAC 50
CGGGCGACCT GGGTAAAGCT ATTCCGGAAG GCGCGGTAGA TAACGGGCAA 100
<210> 7199
<211> 100
<212> DNA
<213> Escherichia coli K-12 MG1655
<220> ilvd b3772
<222> 1719-1818
<223> ilvd b3772 U00096 3951132..3952949
<313> 7199: 1789-1808
<400> 7199
TCGTGAACGT CAGGTCTCCT TTGCCCTGCG TGCTTATGCC AGCCTGGCAA 50
CCAGCGCCGA CAAAGGCGCG GTGCGCGATA AATCGAAACT GGGGGGTTAA 100
<210> 7200
<211> 100
<212> DNA
<213> Escherichia coli K-12 MG1655
<220> ilvd b3772
<222> 1678-1777
<223> ilvd b3772 U00096 3951132..3952949
<313> 7200: 1741-1760
<400> 7200
GCGCAGGACG CTCGAGGTGA CAAAGCCTGG ACGCCGAAAA ATCGTGAACG 50
TCAGGTCTCC TTTGCCCTGC GTGCTTATGC CAGCCTGGCA ACCAGCGCCG 100
<210> 7201
<211> 100
<212> DNA
<213> Escherichia coli K-12 MG1655
<220> ilvd b3772
<222> 1604-1703
<223> ilvd b3772 U00096 3951132..3952949
<313> 7201: 1615-1634
<400> 7201
TGATCGCTAT CGACATCCCG AACCGTGGCA TTCAGTTACA GGTAAGCGAT 50
GCCGAACTGG CGGCGCGTCG TGAAGCGCAG GACGCTCGAG GTGACAAAGC 100
<210> 7202
<211> 100
<212> DNA
<213> Escherichia coli K-12 MG1655
<220> ilve b3771
<222> 831-930
<223> ilve b3771 U00096 3950107..3951036
<313> 7202: 884-903
<400> 7202
TTGTGGCCCG GTTACCAAAC GCATTCAGCA AGCCTTCTTC GGCCTCTTCA 50
CTGGCGAAAC CGAAGATAAA TGGGGCTGGT TAGATCAAGT TAATCAATAA 100
<210> 7203
<211> 100
<212> DNA
<213> Escherichia coli K-12 MG1655
<220> ilve b3771
<222> 798-897
<223> ilve b3771 U00096 3950107..3951036
<313> 7203: 856-875
<400> 7203
CAGCGTAGAC GGTATTCAGG TTGGCGAAGG CCGTTGTGGC CCGGTTACCA 50

AACGCATTCA GCAAGCCTTC TTCGGCCTCT TCACTGGCGA AACCGAAGAT 100
<210> 7204
<211> 100
<212> DNA
<213> Escherichia coli K-12 MG1655
<220> ilve b3771
<222> 768-867
<223> ilve b3771 U00096 3950107..3951036
<313> 7204: 813-832
<400> 7204
CGGTACGGCG GCAGAAATCA CGCCAGTGCG CAGCGTAGAC GGTATTCAGG 50
TTGGCGAAGG CCGTTGTGGC CCGGTTACCA AACGCATTCA GCAAGCCTTC 100
<210> 7205
<211> 100
<212> DNA
<213> Escherichia coli K-12 MG1655
<220> ilvh b0079
<222> 391-490
<223> ilvh b0079 U00096 87357..87848
<313> 7205: 435-454
<400> 7205
CTTGATGCAT TTTTAGCATC GATTCGCGAT GTGGCGAAAA TTGTGGAGGT 50
TGCTCGCTCT GGTGTGGTCG GACTTTCGCG CGGCGATAAA ATAATGCGTT 100
<210> 7206
<211> 100
<212> DNA
<213> Escherichia coli K-12 MG1655
<220> ilvh b0079
<222> 304-403
<223> ilvh b0079 U00096 87357..87848
<313> 7206: 353-372
<400> 7206
AAACGTAATA CGGAAATATT CCGTGGGCAA ATTATCGATG TCACACCCTC 50
GCTTTATACC GTTCAATTAG CAGGCACCAG CGGTAAGCTT GATGCATTTT 100
<210> 7207
<211> 100
<212> DNA
<213> Escherichia coli K-12 MG1655
<220> ilvh b0079
<222> 263-362
<223> ilvh b0079 U00096 87357..87848
<313> 7207: 293-312
<400> 7207
TGGTGAAAAT TCAGGCCAGC GGTTACGGGC GTGACGAAGT GAAACGTAAT 50
ACGGAAATAT TCCGTGGGCA AATTATCGAT GTCACACCCT CGCTTTATAC 100
<210> 7208
<211> 100
<212> DNA
<213> Escherichia coli K-12 MG1655
<220> ilvi b0078
<222> 1716-1815
<223> ilvi b0078 U00096 85540..87354
<313> 7208: 1776-1795
<400> 7208
TGTTGATGTT ACCGTCGATG GCAGCGAGCA CGTCTACCCG ATGCAGATTC 50
GCGGGGGCGG AATGGATGAA ATGTGGTTAA GCAAAACGGA GAGAACCTGA 100
<210> 7209
<211> 100
<212> DNA
<213> Escherichia coli K-12 MG1655
<220> ilvi b0078
<222> 1618-1717
<223> ilvi b0078 U00096 85540..87354
<313> 7209: 1657-1676
<400> 7209
GCCTATGGGC ATGTCGGGAT CCAGATTTCT CATCCGCATG AGCTGGAAAG 50
CAAACTTAGC GAGGCGCTGG AACAGGTGCG CAATAATCGC CTGGTGTTTG 100
<210> 7210
<211> 100
<212> DNA
<213> Escherichia coli K-12 MG1655

<220> ilvi b0078
<222> 1562-1661
<223> ilvi b0078 U00096 85540..87354
<313> 7210: 1575-1594
<400> 7210
GCCGTCATTC ACAATCTTAT ATGCAATCGC TACCCGATTT CGTCCGTCTG 50
GCGGAAGCCT ATGGGCATGT CGGGATCCAG ATTTCTCATC CGCATGAGCT 100
<210> 7211
<211> 100
<212> DNA
<213> Escherichia coli K-12 MG1655
<220> rpsa b0912
<222> 1570-1669
<223> rpsa b0912 U00096 961218..962891
<313> 7211: 1618-1637
<400> 7211
GAAGCTGACG AGAAAGATGC AATCGCAACT GTTAACAAAC AGGAAGATGC 50
AAACTTCTCC AACAACGCAA TGGCTGAAGC TTTCAAAGCA GCTAAAGGCG 100
<210> 7212
<211> 100
<212> DNA
<213> Escherichia coli K-12 MG1655
<220> rpsa b0912
<222> 1523-1622
<223> rpsa b0912 U00096 961218..962891
<313> 7212: 1576-1595
<400> 7212
TTGATCGTAA AAACCGCGCA ATCAGCCTGT CTGTTCGTGC GAAAGACGAA 50
GCTGACGAGA AAGATGCAAT CGCAACTGTT AACAAACAGG AAGATGCAAA 100
<210> 7213
<211> 100
<212> DNA
<213> Escherichia coli K-12 MG1655
<220> rpsa b0912
<222> 1504-1603
<223> rpsa b0912 U00096 961218..962891
<313> 7213: 1537-1556
<400> 7213
GAAGCTAAAT TCACCGGCGT TGATCGTAAA AACCGCGCAA TCAGCCTGTC 50
TGTTCGTGCG AAAGACGAAG CTGACGAGAA AGATGCAATC GCAACTGTTA 100
<210> 7214
<211> 100
<212> DNA
<213> Escherichia coli K-12 MG1655
<220> sufi b3018
<222> 1305-1404
<223> sufi b3018 U00096 complement(3159273..3160685)
<313> 7214: 1357-1376
<400> 7214
TTTCGGTCAG CCTTCCTGGG CGCACTTCCC GTTCTACTTC AACAGTCAGA 50
CGCTGGAAAT GGCGGACCGT GGCTCGATTG GGCAACTGTT GGTCAATCCG 100
<210> 7215
<211> 100
<212> DNA
<213> Escherichia coli K-12 MG1655
<220> sufi b3018
<222> 1277-1376
<223> sufi b3018 U00096 complement(3159273..3160685)
<313> 7215: 1316-1335
<400> 7215
TTGACGGACA AGTGGAGCTG CTTGTTTATT TCGGTCAGCC TTCCTGGGCG 50
CACTTCCCGT TCTACTTCAA CAGTCAGACG CTGGAAATGG CGGACCGTGG 100
<210> 7216
<211> 100
<212> DNA
<213> Escherichia coli K-12 MG1655
<220> sufi b3018
<222> 1227-1326
<223> sufi b3018 U00096 complement(3159273..3160685)
<313> 7216: 1267-1286
<400> 7216

TGGCGCGATG CCGTTCCCGG AAGACAGAGG CTGGAAAGAT ACCGTTTGGG 50
TTGACGGACA AGTGGAGCTG CTTGTTTATT TCGGTCAGCC TTCCTGGGCG 100
<210> 7217
<211> 100
<212> DNA
<213> Escherichia coli K-12 MG1655
<220> rpsb b0170
<222> 627-726
<223> rpsb b0170 U00096 189874..190599
<313> 7217: 686-705
<400> 7217
TGTGACCCTG TACCTGGGCG CTGTTGCTGC AACCGTACGT GAAGGCCGTT 50
CTCAGGATCT GGCTTCCCAG GCGGAAGAAA GCTTCGTAGA AGCTGAGTAA 100
<210> 7218
<211> 100
<212> DNA
<213> Escherichia coli K-12 MG1655
<220> rpsb b0170
<222> 611-710
<223> rpsb b0170 U00096 189874..190599
<313> 7218: 625-644
<400> 7218
ACGACGCAAT CCGTGCTGTG ACCCTGTACC TGGGCGCTGT TGCTGCAACC 50
GTACGTGAAG GCCGTTCTCA GGATCTGGCT TCCCAGGCGG AAGAAAGCTT 100
<210> 7219
<211> 100
<212> DNA
<213> Escherichia coli K-12 MG1655
<220> rpsb b0170
<222> 479-578
<223> rpsb b0170 U00096 189874..190599
<313> 7219: 506-525
<400> 7219
CTCTGTTTGT AATCGATGCT GACCACGAAC ACATTGCTAT CAAAGAAGCA 50
AACAACCTGG GTATTCCGGT ATTTGCTATC GTTGATACCA ACTCTGATCC 100
<210> 7220
<211> 100
<212> DNA
<213> Escherichia coli K-12 MG1655
<220> ilvl b3767
<222> 42-99
<223> ilvl b3767 U00096 3947945..3948043
<313> 7220: 78-97
<400> 7220
CGTGGTGGTG ATTATTATCC CACCGTGCGG GGCTGCACTT GGACGAGGAA 50
AGGCTTAG 100
<210> 7221
<211> 100
<212> DNA
<213> Escherichia coli K-12 MG1655
<220> rpsc b3315
<222> 603-702
<223> rpsc b3315 U00096 complement(3446819..3447520)
<313> 7221: 673-692
<400> 7221
GATCTTCAAA GGCGAGATCC TGGGTGGTAT GGCTGCTGTT GAACAACCGG 50
AAAAACCGGC TGCTCAGCCT AAAAAGCAGC AGCGTAAAGG CCGTAAATAA 100
<210> 7222
<211> 100
<212> DNA
<213> Escherichia coli K-12 MG1655
<220> rpsc b3315
<222> 603-702
<223> rpsc b3315 U00096 complement(3446819..3447520)
<313> 7222: 626-645
<400> 7222
GATCTTCAAA GGCGAGATCC TGGGTGGTAT GGCTGCTGTT GAACAACCGG 50
AAAAACCGGC TGCTCAGCCT AAAAAGCAGC AGCGTAAAGG CCGTAAATAA 100
<210> 7223
<211> 100
<212> DNA

<213> Escherichia coli K-12 MG1655
<220> rpsc b3315
<222> 557-656
<223> rpsc b3315 U00096 complement(3446819..3447520)
<313> 7223: 600-619
<400> 7223
CCTCTGAAGC GCACACCACT TACGGTGTAA TCGGCGTTAA AGTGTGGATC 50
TTCAAAGGCG AGATCCTGGG TGGTATGGCT GCTGTTGAAC AACCGGAAAA 100
<210> 7224
<211> 100
<212> DNA
<213> Escherichia coli K-12 MG1655
<220> ilvm b3770
<222> 165-264
<223> ilvm b3770 U00096 3949824..3950087
<313> 7224: 240-259
<400> 7224
GTCGGTCGAC TTACTGTTTA GTCAGTTAAA TAAACTGGTG GACGTCGCAC 50
ACGTTGCCAT CTGCCAGAGC ACAACCACAT CACAACAAAT CCGCGCCTGA 100
<210> 7225
<211> 100
<212> DNA
<213> Escherichia coli K-12 MG1655
<220> ilvm b3770
<222> 74-173
<223> ilvm b3770 U00096 3949824..3950087
<313> 7225: 126-145
<400> 7225
GTCATCGTGG TTTCCACGTC TGCTCAATGA ATATGGCCGC CGCCAGCGAT 50
GCACAAAATA TAAATATCGA ATTGACCGTT GCCAGCCCAC GGTCGGTCGA 100
<210> 7226
<211> 100
<212> DNA
<213> Escherichia coli K-12 MG1655
<220> ilvm b3770
<222> 55-154
<223> ilvm b3770 U00096 3949824..3950087
<313> 7226: 78-97
<400> 7226
CGTGTTTTAC GCGTGGTGCG TCATCGTGGT TTCCACGTCT GCTCAATGAA 50
TATGGCCGCC GCCAGCGATG CACAAAATAT AAATATCGAA TTGACCGTTG 100
<210> 7227
<211> 100
<212> DNA
<213> Escherichia coli K-12 MG1655
<220> rpsd b3297
<222> 522-621
<223> rpsd b3297 U00096 complement(3438692..3439312)
<313> 7227: 593-612
<400> 7227
TGCTGGCAAG ATGGAAGGTA CGTTTAAGCG TAAGCCGGAG CGTTCTGATC 50
TGTCTGCGGA CATTAACGAA CACCTGATCG TCGAGCTTTA CTCCAAGTAA 100
<210> 7228
<211> 100
<212> DNA
<213> Escherichia coli K-12 MG1655
<220> rpsd b3297
<222> 401-500
<223> rpsd b3297 U00096 complement(3438692..3439312)
<313> 7228: 440-459
<400> 7228
CTTATCAGGT TAGTCCGAAT GACGTTGTAA GCATTCGTGA GAAAGCGAAG 50
AAGCAGTCTC GCGTGAAAGC CGCTCTGGAG CTGGCTGAGC AGCGTGAAAA 100
<210> 7229
<211> 100
<212> DNA
<213> Escherichia coli K-12 MG1655
<220> rpsd b3297
<222> 264-363
<223> rpsd b3297 U00096 complement(3438692..3439312)
<313> 7229: 327-346

<400> 7229
AAACCTGTTG GCTCTGCTGG AAGGTCGTCT GGACAACGTT GTATACCGTA  50
TGGGCTTCGG TGCCACTCGT GCAGAAGCAC GTCAGCTGGT TAGCCATAAA  100
<210> 7230
<211> 100
<212> DNA
<213> Escherichia coli K-12 MG1655
<220> ilvn b3671
<222> 192-291
<223> ilvn b3671 U00096 complement(3848429..3848719)
<313> 7230: 247-266
<400> 7230
GATGATAAGC CAAATCGATA AGCTGGAAGA TGTCGTGAAA GTGCAGCGTA  50
ATCAGTCCGA TCCGACGATG TTTAACAAGA TCGCGGTGTT TTTTCAGTAA  100
<210> 7231
<211> 100
<212> DNA
<213> Escherichia coli K-12 MG1655
<220> ilvn b3671
<222> 167-266
<223> ilvn b3671 U00096 complement(3848429..3848719)
<313> 7231: 193-212
<400> 7231
TCAATGACGA CCAGCGTCTG GAGCAGATGA TAAGCCAAAT CGATAAGCTG  50
GAAGATGTCG TGAAAGTGCA GCGTAATCAG TCCGATCCGA CGATGTTTAA  100
<210> 7232
<211> 100
<212> DNA
<213> Escherichia coli K-12 MG1655
<220> ilvn b3671
<222> 114-213
<223> ilvn b3671 U00096 complement(3848429..3848719)
<313> 7232: 142-161
<400> 7232
CATTCTTTGT CTGCCGATTC AGGACAGCGA CAAAAGCCAT ATCTGGCTAC  50
TGGTCAATGA CGACCAGCGT CTGGAGCAGA TGATAAGCCA AATCGATAAG  100
<210> 7233
<211> 100
<212> DNA
<213> Escherichia coli K-12 MG1655
<220> rpse b3304
<222> 405-504
<223> rpse b3304 U00096 complement(3442363..3442866)
<313> 7233: 483-502
<400> 7233
CGTGGTTCGT GCAACTATTG ATGGCCTGGA AAATATGAAT TCTCCAGAAA  50
TGGTCGCTGC CAAGCGTGGT AAATCCGTTG AAGAAATTCT GGGGAAATAA  100
<210> 7234
<211> 100
<212> DNA
<213> Escherichia coli K-12 MG1655
<220> rpse b3304
<222> 367-466
<223> rpse b3304 U00096 complement(3442363..3442866)
<313> 7234: 396-415
<400> 7234
GTTCTGGCTA AAGCCTATGG TTCCACCAAC CCGATCAACG TGGTTCGTGC  50
AACTATTGAT GGCCTGGAAA ATATGAATTC TCCAGAAATG GTCGCTGCCA  100
<210> 7235
<211> 100
<212> DNA
<213> Escherichia coli K-12 MG1655
<220> rpse b3304
<222> 312-411
<223> rpse b3304 U00096 complement(3442363..3442866)
<313> 7235: 348-367
<400> 7235
TATCATCGCC GGTGGTGCAA TGCGCGCCGT TCTGGAAGTC GCTGGGGTTC  50
ATAACGTTCT GGCTAAAGCC TATGGTTCCA CCAACCCGAT CAACGTGGTT  100
<210> 7236
<211> 100

<212> DNA
<213> Escherichia coli K-12 MG1655
<220> rpsf b4201
<222> 297-396
<223> rpsf b4201 U00096 4422696..4423091
<313> 7236: 373-392
<400> 7236
ATCTCCGATG GTTAAAGCGA AAGACGAGCG CCGTGAGCGT CGCGATGATT 50
TCGCAAACGA AACCGCTGAT GATGCTGAAG CTGGGGATTC TGAAGAGTAA 100
<210> 7237
<211> 100
<212> DNA
<213> Escherichia coli K-12 MG1655
<220> rpsf b4201
<222> 278-377
<223> rpsf b4201 U00096 4422696..4423091
<313> 7237: 299-318
<400> 7237
AGCACGCTGT TACCGAAGCA TCTCCGATGG TTAAAGCGAA AGACGAGCGC 50
CGTGAGCGTC GCGATGATTT CGCAAACGAA ACCGCTGATG ATGCTGAAGC 100
<210> 7238
<211> 100
<212> DNA
<213> Escherichia coli K-12 MG1655
<220> rpsf b4201
<222> 217-316
<223> rpsf b4201 U00096 4422696..4423091
<313> 7238: 236-255
<400> 7238
GAGCTGGAAA CTACCTTCCG CTTCAACGAT GCCGTTATCC GCAGCATGGT 50
TATGCGTACC AAGCACGCTG TTACCGAAGC ATCTCCGATG GTTAAAGCGA 100
<210> 7239
<211> 100
<212> DNA
<213> Escherichia coli K-12 MG1655
<220> rpsg b3342
<222> 393-492
<223> rpsg b3342 U00096 complement(3471179..3471718)
<313> 7239: 457-476
<400> 7239
AGGTACTGCA GTTAAGAAAC GTGAAGACGT TCACCGTATG GCCGAAGCCA 50
ACAAGGCGTT CGCACACTAC CGTTGGTTAT CCCTTCGGAG TTTTAGTCAC 100
<210> 7240
<211> 100
<212> DNA
<213> Escherichia coli K-12 MG1655
<220> rpsg b3342
<222> 263-362
<223> rpsg b3342 U00096 complement(3471179..3471718)
<313> 7240: 316-335
<400> 7240
CAGTTGAAGT CCGTCCGGTT CGTCGTAATG CTCTGGCAAT GCGTTGGATC 50
GTTGAAGCTG CTCGTAAACG CGGTGATAAA TCCATGGCTC TGCGCCTGGC 100
<210> 7241
<211> 100
<212> DNA
<213> Escherichia coli K-12 MG1655
<220> rpsg b3342
<222> 233-332
<223> rpsg b3342 U00096 complement(3471179..3471718)
<313> 7241: 269-288
<400> 7241
GCCGCGTTGG TGGTTCTACT TATCAGGTAC CAGTTGAAGT CCGTCCGGTT 50
CGTCGTAATG CTCTGGCAAT GCGTTGGATC GTTGAAGCTG CTCGTAAACG 100
<210> 7242
<211> 100
<212> DNA
<213> Escherichia coli K-12 MG1655
<220> rpsh b3307
<222> 227-326
<223> rpsh b3307 U00096 complement(3443790..3444182)

<313> 7242: 258-277
<400> 7242
AGCGTGTCAG CCGCCCAGGT CTGCGCATCT ATAAACGTAA AGATGAGCTG 50
CCGAAAGTTA TGGCGGGTCT GGGTATCGCA GTTGTTTCTA CCTCTAAAGG 100
<210> 7243
<211> 100
<212> DNA
<213> Escherichia coli K-12 MG1655
<220> rpsh b3307
<222> 163-262
<223> rpsh b3307 U00096 complement(3443790..3444182)
<313> 7243: 216-235
<400> 7243
ACCAAGCCTG AACTGGAACT TACTCTGAAG TATTTCCAGG GCAAAGCTGT 50
TGTAGAAAGC ATTCAGCGTG TCAGCCGCCC AGGTCTGCGC ATCTATAAAC 100
<210> 7244
<211> 100
<212> DNA
<213> Escherichia coli K-12 MG1655
<220> rpsh b3307
<222> 120-219
<223> rpsh b3307 U00096 complement(3443790..3444182)
<313> 7244: 163-182
<400> 7244
GAAGGAAGAA GGTTTTATTG AAGATTTTAA AGTTGAAGGC GACACCAAGC 50
CTGAACTGGA ACTTACTCTG AAGTATTTCC AGGGCAAAGC TGTTGTAGAA 100
<210> 7245
<211> 100
<212> DNA
<213> Escherichia coli K-12 MG1655
<220> rpsi b3231
<222> 294-393
<223> rpsi b3231 U00096 complement(3375450..3375842)
<313> 7245: 361-380
<400> 7245
GCGTAAAGCT GGCTTCGTTA CTCGTGACGC TCGTCAGGTT GAACGTAAGA 50
AAGTCGGTCT GCGTAAAGCA CGTCGTCGTC CGCAGTTCTC CAAACGTTAA 100
<210> 7246
<211> 100
<212> DNA
<213> Escherichia coli K-12 MG1655
<220> rpsi b3231
<222> 268-367
<223> rpsi b3231 U00096 complement(3375450..3375842)
<313> 7246: 313-332
<400> 7246
TACGACGAGT CCCTGCGTTC TGAACTGCGT AAAGCTGGCT TCGTTACTCG 50
TGACGCTCGT CAGGTTGAAC GTAAGAAAGT CGGTCTGCGT AAAGCACGTC 100
<210> 7247
<211> 100
<212> DNA
<213> Escherichia coli K-12 MG1655
<220> rpsi b3231
<222> 246-345
<223> rpsi b3231 U00096 complement(3375450..3375842)
<313> 7247: 276-295
<400> 7247
TATCACCCGC GCTCTGATGG AATACGACGA GTCCCTGCGT TCTGAACTGC 50
GTAAAGCTGG CTTCGTTACT CGTGACGCTC GTCAGGTTGA ACGTAAGAAA 100
<210> 7248
<211> 100
<212> DNA
<213> Escherichia coli K-12 MG1655
<220> riba b1278
<222> 492-591
<223> riba b1278 U00096 complement(1336594..1337184)
<313> 7248: 564-583
<400> 7248
TATTGTTGAA CGCGTACCAT TGATTGTAGG TCGTAACCCC AATAACGAAC 50
ATTATCTCGA TACCAAAGCC GAGAAAATGG GCCATTTGCT GAACAAATAA 100
<210> 7249

<211> 100
<212> DNA
<213> Escherichia coli K-12 MG1655
<220> riba b1278
<222> 472-571
<223> riba b1278 U00096 complement(1336594..1337184)
<313> 7249: 514-533
<400> 7249
CTGACCGAAG CAGGGATTAA TATTGTTGAA CGCGTACCAT TGATTGTAGG 50
TCGTAACCCC AATAACGAAC ATTATCTCGA TACCAAAGCC GAGAAAATGG 100
<210> 7250
<211> 100
<212> DNA
<213> Escherichia coli K-12 MG1655
<220> riba b1278
<222> 419-518
<223> riba b1278 U00096 complement(1336594..1337184)
<313> 7250: 457-476
<400> 7250
TTGGCGTCAA TGAAGTCCGC TTGTTAACCA ATAACCCGAA AAAAGTCGAA 50
ATTCTGACCG AAGCAGGGAT TAATATTGTT GAACGCGTAC CATTGATTGT 100
<210> 7251
<211> 100
<212> DNA
<213> Escherichia coli K-12 MG1655
<220> rpsj b3322
<222> 179-278
<223> rpsj b3322 U00096 complement(3450596..3450907)
<313> 7251: 236-255
<400> 7251
ACGCGCGCGA TCAGTACGAA ATCCGTACTC ACTTGCGTCT GGTTGACATC 50
GTTGAGCCAA CCGAGAAAAC CGTTGATGCT CTGATGCGTC TGGATCTGGC 100
<210> 7252
<211> 100
<212> DNA
<213> Escherichia coli K-12 MG1655
<220> rpsj b3322
<222> 131-230
<223> rpsj b3322 U00096 complement(3450596..3450907)
<313> 7252: 144-163
<400> 7252
CACGCAAAGA GCGCTTCACT GTTCTGATCT CCCCGCACGT CAACAAAGAC 50
GCGCGCGATC AGTACGAAAT CCGTACTCAC TTGCGTCTGG TTGACATCGT 100
<210> 7253
<211> 100
<212> DNA
<213> Escherichia coli K-12 MG1655
<220> rpsj b3322
<222> 91-190
<223> rpsj b3322 U00096 complement(3450596..3450907)
<313> 7253: 106-125
<400> 7253
CGCACTGGTG CGCAGGTCCG TGGTCCGATC CCGCTGCCGA CACGCAAAGA 50
GCGCTTCACT GTTCTGATCT CCCCGCACGT CAACAAAGAC GCGCGCGATC 100
<210> 7254
<211> 100
<212> DNA
<213> Escherichia coli K-12 MG1655
<220> ribb ribB
<222> 546-645
<223> ribb ribB U00096 complement(3181829..3182482)
<313> 7254: 597-616
<400> 7254
GGCGCGTGCA CCAGAGTGTA TTGAGTTTGC CAATAAACAC AATATGGCGC 50
TCGTGACTAT TGAAGACCTG GTGGCATACC GTCAGGCACA TGAGCGTAAA 100
<210> 7255
<211> 100
<212> DNA
<213> Escherichia coli K-12 MG1655
<220> ribb ribB
<222> 464-563

<223> ribb ribB U00096 complement(3181829..3182482)
<313> 7255: 521-540
<400> 7255
AAGCAACTAT TGATCTGATG ACGCTGGCAG GCTTTAAACC GGCTGGTGTA  50
CTGTGTGAGC TGACTAATGA CGATGGCACG ATGGCGCGTG CACCAGAGTG  100
<210> 7256
<211> 100
<212> DNA
<213> Escherichia coli K-12 MG1655
<220> ribb ribB
<222> 444-543
<223> ribb ribB U00096 complement(3181829..3182482)
<313> 7256: 477-496
<400> 7256
GACGCGTGGC GGTCATACTG AAGCAACTAT TGATCTGATG ACGCTGGCAG  50
GCTTTAAACC GGCTGGTGTA CTGTGTGAGC TGACTAATGA CGATGGCACG  100
<210> 7257
<211> 100
<212> DNA
<213> Escherichia coli K-12 MG1655
<220> rpsk b3298
<222> 291-390
<223> rpsk b3298 U00096 complement(3439346..3439735)
<313> 7257: 366-385
<400> 7257
TCGTGCTCTG AACGCCGCAG GTTTCCGCAT CACTAACATT ACTGATGTGA  50
CTCCGATCCC TCATAACGGT TGTCGTCCGC CGAAAAAACG TCGCGTATAA  100
<210> 7258
<211> 100
<212> DNA
<213> Escherichia coli K-12 MG1655
<220> rpsk b3298
<222> 268-367
<223> rpsk b3298 U00096 complement(3439346..3439735)
<313> 7258: 299-318
<400> 7258
GGTCCAGGCC GCGAATCTAC TATTCGTGCT CTGAACGCCG CAGGTTTCCG  50
CATCACTAAC ATTACTGATG TGACTCCGAT CCCTCATAAC GGTTGTCGTC  100
<210> 7259
<211> 100
<212> DNA
<213> Escherichia coli K-12 MG1655
<220> rpsk b3298
<222> 132-231
<223> rpsk b3298 U00096 complement(3439346..3439735)
<313> 7259: 175-194
<400> 7259
GGCAACAGCC GGTGGTTCCG GTTTCCGTGG TTCTCGCAAA TCCACTCCGT  50
TTGCAGCTCA GGTTGCAGCA GAGCGTTGCG CTGACGCCGT GAAAGAATAC  100
<210> 7260
<211> 100
<212> DNA
<213> Escherichia coli K-12 MG1655
<220> rpsl b3343
<222> 276-375
<223> rpsl b3343 U00096 complement(3471815..3472189)
<313> 7260: 340-359
<400> 7260
TGTTCGTTAC CACACCGTAC GTGGTGCGCT TGACTGCTCC GGCGTTAAAG  50
ACCGTAAGCA GGCTCGTTCC AAGTATGGCG TGAAGCGTCC TAAGGCTTAA  100
<210> 7261
<211> 100
<212> DNA
<213> Escherichia coli K-12 MG1655
<220> rpsl b3343
<222> 276-375
<223> rpsl b3343 U00096 complement(3471815..3472189)
<313> 7261: 339-358
<400> 7261
TGTTCGTTAC CACACCGTAC GTGGTGCGCT TGACTGCTCC GGCGTTAAAG  50
ACCGTAAGCA GGCTCGTTCC AAGTATGGCG TGAAGCGTCC TAAGGCTTAA  100

<210> 7262
<211> 100
<212> DNA
<213> Escherichia coli K-12 MG1655
<220> rpsl b3343
<222> 276-375
<223> rpsl b3343 U00096 complement(3471815..3472189)
<313> 7262: 325-344
<400> 7262
TGTTCGTTAC CACACCGTAC GTGGTGCGCT TGACTGCTCC GGCGTTAAAG 50
ACCGTAAGCA GGCTCGTTCC AAGTATGGCG TGAAGCGTCC TAAGGCTTAA 100
<210> 7263
<211> 100
<212> DNA
<213> Escherichia coli K-12 MG1655
<220> ribd b0415
<222> 991-1090
<223> ribd b0415 U00096 432679..433782
<313> 7263: 1013-1032
<400> 7263
CGCGGATTAT GCACGCTGCC AGGGCTTGAG AAATTAGCCG ACGCCCCCCA 50
ATTTAAATTC AAAGAGATAC GTCATGTAGG CCCGGATGTT TGCCTGCATT 100
<210> 7264
<211> 100
<212> DNA
<213> Escherichia coli K-12 MG1655
<220> ribd b0415
<222> 943-1042
<223> ribd b0415 U00096 432679..433782
<313> 7264: 960-979
<400> 7264
GATGAGCTGA TTGTCTATAT CGCACCTAAA CTATTAGGCA GCGACGCCCG 50
CGGATTATGC ACGCTGCCAG GGCTTGAGAA ATTAGCCGAC GCCCCCCAAT 100
<210> 7265
<211> 100
<212> DNA
<213> Escherichia coli K-12 MG1655
<220> ribd b0415
<222> 671-770
<223> ribd b0415 U00096 432679..433782
<313> 7265: 731-750
<400> 7265
TCCGTCAGCC GATACGTATT GTGATTGATA GCCAAAATCG CGTGACGCCG 50
GTACATCGCA TTGTGCAGCA GCCCGGCGAA ACCTGGTTCG CGCGTACGCA 100
<210> 7266
<211> 100
<212> DNA
<213> Escherichia coli K-12 MG1655
<220> rpsm b3299
<222> 258-357
<223> rpsm b3299 U00096 complement(3439752..3440108)
<313> 7266: 323-342
<400> 7266
TCGCGGTTTG CGTCATCGTC GTGGTCTCCC GGTTCGCGGT CAGCGTACCA 50
AGACCAACGC ACGTACCCGT AAGGGTCCGC GCAAACCGAT CAAGAAATAA 100
<210> 7267
<211> 100
<212> DNA
<213> Escherichia coli K-12 MG1655
<220> rpsm b3299
<222> 255-354
<223> rpsm b3299 U00096 complement(3439752..3440108)
<313> 7267: 284-303
<400> 7267
CTATCGCGGT TTGCGTCATC GTCGTGGTCT CCCGGTTCGC GGTCAGCGTA 50
CCAAGACCAA CGCACGTACC CGTAAGGGTC CGCGCAAACC GATCAAGAAA 100
<210> 7268
<211> 100
<212> DNA
<213> Escherichia coli K-12 MG1655
<220> rpsm b3299

<222> 173-272
<223> rpsm b3299 U00096 complement(3439752..3440108)
<313> 7268: 223-242
<400> 7268
ACGAAGTTGC CAAATTTGTC GTTGAAGGTG ATCTGCGCCG TGAAATCAGC 50
ATGAGCATCA AGCGCCTGAT GGATCTTGGT TGCTATCGCG GTTTGCGTCA 100
<210> 7269
<211> 100
<212> DNA
<213> Escherichia coli K-12 MG1655
<220> ribe b1663
<222> 543-642
<223> ribe b1663 U00096 complement(1740625..1741266)
<313> 7269: 615-634
<400> 7269
CATTGAAATC GATCCACAAA CTCAGGCAGT GGTAGATACG GTAGAACGTG 50
TGCTGGCGGC ACGAGAAAAT GCCATGAATC AACCAGGCAC AGAAGCCTGA 100
<210> 7270
<211> 100
<212> DNA
<213> Escherichia coli K-12 MG1655
<220> ribe b1663
<222> 511-610
<223> ribe b1663 U00096 complement(1740625..1741266)
<313> 7270: 554-573
<400> 7270
CTTGGGAAGA AAAAACTTGG CGCACGCGTC AACATTGAAA TCGATCCACA 50
AACTCAGGCA GTGGTAGATA CGGTAGAACG TGTGCTGGCG GCACGAGAAA 100
<210> 7271
<211> 100
<212> DNA
<213> Escherichia coli K-12 MG1655
<220> ribe b1663
<222> 451-550
<223> ribe b1663 U00096 complement(1740625..1741266)
<313> 7271: 513-532
<400> 7271
GAAGTCACGC CAACGCGTTT TTGCGTCCAT TTAATTCCGG AAACACTGGA 50
ACGCACGACT CTTGGGAAGA AAAAACTTGG CGCACGCGTC AACATTGAAA 100
<210> 7272
<211> 100
<212> DNA
<213> Escherichia coli K-12 MG1655
<220> rpsn b3308
<222> 195-294
<223> rpsn b3308 U00096 complement(3444216..3444521)
<313> 7272: 261-280
<400> 7272
TCAAACAGGT CGTCCGCATG GTTTCCTGCG GAAGTTCGGG TTGAGCCGTA 50
TTAAGGTCCG TGAAGCCGCT ATGCGCGGTG AAATCCCGGG TCTGAAAAAG 100
<210> 7273
<211> 100
<212> DNA
<213> Escherichia coli K-12 MG1655
<220> rpsn b3308
<222> 150-249
<223> rpsn b3308 U00096 complement(3444216..3444521)
<313> 7273: 185-204
<400> 7273
TCTGCCGCGT GATTCCAGCC CGTCTCGTCA GCGTAACCGC TGCCGTCAAA 50
CAGGTCGTCC GCATGGTTTC CTGCGGAAGT TCGGGTTGAG CCGTATTAAG 100
<210> 7274
<211> 100
<212> DNA
<213> Escherichia coli K-12 MG1655
<220> rpsn b3308
<222> 97-196
<223> rpsn b3308 U00096 complement(3444216..3444521)
<313> 7274: 158-177
<400> 7274
GATGTGAACG CTTCCGACGA AGATCGTTGG AACGCTGTTC TCAAGCTGCA 50

GACTCTGCCG CGTGATTCCA GCCCGTCTCG TCAGCGTAAC CGCTGCCGTC 100
<210> 7275
<211> 100
<212> DNA
<213> Escherichia coli K-12 MG1655
<220> ribf b0026
<222> 843-942
<223> ribf b0026 U00096 21407..22348
<313> 7275: 916-935
<400> 7275
CAATGAGCAG CGATTTGCGT CGCTGGACGA ACTGAAAGCG CAGATTGCGC 50
GTGATGAATT AACCGCCCGC GAATTTTTTG GGCTAACAAA ACCGGCTTAA 100
<210> 7276
<211> 100
<212> DNA
<213> Escherichia coli K-12 MG1655
<220> ribf b0026
<222> 657-756
<223> ribf b0026 U00096 21407..22348
<313> 7276: 699-718
<400> 7276
CCCGGTGAAA GGGGTTTATG CGGTAGAAGT GCTGGGCCTC GGTGAAAAGC 50
CGTTACCCGG CGTGGCAAAC ATCGGAACAC GCCCAACGGT TGCCGGTATT 100
<210> 7277
<211> 100
<212> DNA
<213> Escherichia coli K-12 MG1655
<220> ribf b0026
<222> 613-712
<223> ribf b0026 U00096 21407..22348
<313> 7277: 665-684
<400> 7277
ATAGGTTTCC CGACGGCGAA TGTACCGCTG CGCCGTCAGG TTTCCCCGGT 50
GAAAGGGGTT TATGCGGTAG AAGTGCTGGG CCTCGGTGAA AAGCCGTTAC 100
<210> 7278
<211> 100
<212> DNA
<213> Escherichia coli K-12 MG1655
<220> rpso b3166
<222> 171-270
<223> rpso b3166 U00096 complement(3309056..3309325)
<313> 7278: 231-250
<400> 7278
GCGCATGGTT TCTCAGCGTC GTAAACTGCT CGACTACCTG AAACGTAAAG 50
ACGTAGCACG TTACACCCAG CTCATCGAGC GCCTGGGTCT GCGTCGCTAA 100
<210> 7279
<211> 100
<212> DNA
<213> Escherichia coli K-12 MG1655
<220> rpso b3166
<222> 133-232
<223> rpso b3166 U00096 complement(3309056..3309325)
<313> 7279: 181-200
<400> 7279
GAGCACAAAA AAGATCACCA CAGCCGTCGT GGTCTGCTGC GCATGGTTTC 50
TCAGCGTCGT AAACTGCTCG ACTACCTGAA ACGTAAAGAC GTAGCACGTT 100
<210> 7280
<211> 100
<212> DNA
<213> Escherichia coli K-12 MG1655
<220> rpso b3166
<222> 45-144
<223> rpso b3166 U00096 complement(3309056..3309325)
<313> 7280: 85-104
<400> 7280
TGGTCGTGAC GCAAACGACA CCGGTTCTAC CGAAGTTCAG GTAGCACTGC 50
TGACTGCACA GATCAACCAC CTGCAGGGCC ACTTTGCAGA GCACAAAAAA 100
<210> 7281
<211> 100
<212> DNA
<213> Escherichia coli K-12 MG1655

<220> ilvy b3774
<222> 752-851
<223> ilvy b3774 U00096 complement(3954548..3955441)
<313> 7281: 803-822
<400> 7281
CCGAACCGGT GCGTAACCGC GTGATGATTT TAGAGCGCAG CGATGAGAAA 50
ACGCCGTTTG AGCTGGGCGT GTGTGCGCAG AAAAAGCGGC TACATGAGCC 100
<210> 7282
<211> 100
<212> DNA
<213> Escherichia coli K-12 MG1655
<220> ilvy b3774
<222> 708-807
<223> ilvy b3774 U00096 complement(3954548..3955441)
<313> 7282: 736-755
<400> 7282
CTGTGGCGTG GCATTGTTGC CGGAAGTGGT GCTGGAAAAC AGCCCCGAAC 50
CGGTGCGTAA CCGCGTGATG ATTTTAGAGC GCAGCGATGA GAAAACGCCG 100
<210> 7283
<211> 100
<212> DNA
<213> Escherichia coli K-12 MG1655
<220> ilvy b3774
<222> 607-706
<223> ilvy b3774 U00096 complement(3954548..3955441)
<313> 7283: 659-678
<400> 7283
CGCCGCATTG AACTGTGGTT TCGACGCAAT AAAATCAGTA ACCCGATGAT 50
TTACGCCACG GTTGGCGGGC ATGAAGCGAT GGTATCGATG GTGGCACTCG 100
<210> 7284
<211> 100
<212> DNA
<213> Escherichia coli K-12 MG1655
<220> rpsp b2610
<222> 150-249
<223> rpsp b2610 U00096 complement(2743957..2744205)
<313> 7284: 224-243
<400> 7284
TCGCCTGGAT CTGGATCGCA TCGCTCACTG GGTTGGCCAG GGCGCAACTA 50
TTTCTGATCG CGTTGCTGCG CTGATCAAAG AAGTAAACAA AGCAGCTTAA 100
<210> 7285
<211> 100
<212> DNA
<213> Escherichia coli K-12 MG1655
<220> rpsp b2610
<222> 49-148
<223> rpsp b2610 U00096 complement(2743957..2744205)
<313> 7285: 70-89
<400> 7285
TACCAGGTTG TTGTCGCTGA CAGCCGTAAT GCACGCAACG GTCGCTTCAT 50
CGAGCGCGTT GGTTTCTTCA ACCCAATCGC TAGCGAAAAA GAAGAAGGCA 100
<210> 7286
<211> 100
<212> DNA
<213> Escherichia coli K-12 MG1655
<220> rpsp b2610
<222> 1-100
<223> rpsp b2610 U00096 complement(2743957..2744205)
<313> 7286: 16-35
<400> 7286
ATGGTAACTA TTCGTTTAGC ACGTCACGGC GCTAAAAAGC GTCCGTTCTA 50
CCAGGTTGTT GTCGCTGACA GCCGTAATGC ACGCAACGGT CGCTTCATCG 100
<210> 7287
<211> 100
<212> DNA
<213> Escherichia coli K-12 MG1655
<220> ribh b0416
<222> 372-471
<223> ribh b0416 U00096 433871..434341
<313> 7287: 416-435
<400> 7287

AGCGATCGAA CGTGCTGGCA CCAAAGCTGG CAACAAAGGT GCAGAAGCTG 50
CACTGACCGC GCTTGAAATG ATTAATGTAT TGAAAGCCAT CAAGGCCTGA 100
<210> 7288
<211> 100
<212> DNA
<213> Escherichia coli K-12 MG1655
<220> ribh b0416
<222> 269-368
<223> ribh b0416 U00096 433871..434341
<313> 7288: 323-342
<400> 7288
TTGAATATGT CGCTGGTGGT GCAAGCAACG GCCTGGCGCA TGTTGCCCAG 50
GACAGCGAAA TTCCGGTTGC TTTTGGGGTT CTGACCACTG AAAGCATTGA 100
<210> 7289
<211> 100
<212> DNA
<213> Escherichia coli K-12 MG1655
<220> ribh b0416
<222> 210-309
<223> ribh b0416 U00096 433871..434341
<313> 7289: 245-264
<400> 7289
CGGTAAATAC GACGCGGTGA TTGCGCTGGG TACGGTTATT CGTGGTGGCA 50
CTGCCCACTT TGAATATGTC GCTGGTGGTG CAAGCAACGG CCTGGCGCAT 100
<210> 7290
<211> 100
<212> DNA
<213> Escherichia coli K-12 MG1655
<220> rpsq b3312
<222> 156-255
<223> rpsq b3312 U00096 complement(3445951..3446205)
<313> 7290: 218-237
<400> 7290
ATGCGGTATC GGTGACGTGG TTGAAATCCG CGAATGCCGT CCGCTGTCCA 50
AGACTAAATC CTGGACGCTG GTTCGCGTTG TAGAGAAAGC GGTTCTGTAA 100
<210> 7291
<211> 100
<212> DNA
<213> Escherichia coli K-12 MG1655
<220> rpsq b3312
<222> 128-227
<223> rpsq b3312 U00096 complement(3445951..3446205)
<313> 7291: 182-201
<400> 7291
AACTGCACGT ACATGACGAG AACAACGAAT GCGGTATCGG TGACGTGGTT 50
GAAATCCGCG AATGCCGTCC GCTGTCCAAG ACTAAATCCT GGACGCTGGT 100
<210> 7292
<211> 100
<212> DNA
<213> Escherichia coli K-12 MG1655
<220> rpsq b3312
<222> 109-208
<223> rpsq b3312 U00096 complement(3445951..3446205)
<313> 7292: 130-149
<400> 7292
TTCATCAAGC GTACGACCAA ACTGCACGTA CATGACGAGA ACAACGAATG 50
CGGTATCGGT GACGTGGTTG AAATCCGCGA ATGCCGTCCG CTGTCCAAGA 100
<210> 7293
<211> 100
<212> DNA
<213> Escherichia coli K-12 MG1655
<220> rpsr b4203
<222> 129-228
<223> rpsr b4203 U00096 4423417..4423644
<313> 7293: 195-214
<400> 7293
TATCACCGGT ACCCGTGCAA AATACCAGCG TCAGCTGGCT CGCGCTATCA 50
AACGCGCTCG CTACCTGTCC CTGCTGCCGT ACACTGATCG CCATCAGTAA 100
<210> 7294
<211> 100
<212> DNA

<213> Escherichia coli K-12 MG1655
<220> rpsr b4203
<222> 54-153
<223> rpsr b4203 U00096 4423417..4423644
<313> 7294: 100-119
<400> 7294
TCAAGAGATC GACTATAAAG ATATCGCTAC GCTGAAAAAC TACATCACCG 50
AAAGCGGTAA GATTGTCCCA AGCCGTATCA CCGGTACCCG TGCAAAATAC 100
<210> 7295
<211> 100
<212> DNA
<213> Escherichia coli K-12 MG1655
<220> rpsr b4203
<222> 1-100
<223> rpsr b4203 U00096 4423417..4423644
<313> 7295: 15-34
<400> 7295
ATGGCACGTT ATTTCCGTCG TCGCAAGTTC TGCCGTTTCA CCGCGGAAGG 50
CGTTCAAGAG ATCGACTATA AAGATATCGC TACGCTGAAA AACTACATCA 100
<210> 7296
<211> 100
<212> DNA
<213> Escherichia coli K-12 MG1655
<220> rpss b3317
<222> 180-279
<223> rpss b3317 U00096 complement(3447885..3448163)
<313> 7296: 249-268
<400> 7296
ATTTGTAACC GACGAAATGG TTGGTCACAA ACTGGGTGAA TTCGCACCGA 50
CTCGTACTTA TCGCGGCCAC GCTGCTGATA AAAAAGCGAA GAAGAAATAA 100
<210> 7297
<211> 100
<212> DNA
<213> Escherichia coli K-12 MG1655
<220> rpss b3317
<222> 140-239
<223> rpss b3317 U00096 complement(3447885..3448163)
<313> 7297: 169-188
<400> 7297
TGACCATCGC TGTCCATAAT GGTCGTCAGC ACGTTCCGGT ATTTGTAACC 50
GACGAAATGG TTGGTCACAA ACTGGGTGAA TTCGCACCGA CTCGTACTTA 100
<210> 7298
<211> 100
<212> DNA
<213> Escherichia coli K-12 MG1655
<220> rpss b3317
<222> 70-169
<223> rpss b3317 U00096 complement(3447885..3448163)
<313> 7298: 134-153
<400> 7298
GAAAGCGGAG ACAAGAAGCC CCTGCGCACT TGGTCCCGTC GTTCAACGAT 50
CTTTCCTAAC ATGATCGGTT TGACCATCGC TGTCCATAAT GGTCGTCAGC 100
<210> 7299
<211> 100
<212> DNA
<213> Escherichia coli K-12 MG1655
<220> rpst rpsT
<222> 165-264
<223> rpst rpsT U00096 complement(20815..21078)
<313> 7299: 224-243
<400> 7299
ACCGATCGTG GACCGTCAGG CTGCTAAAGG TCTGATCCAC AAAAACAAAG 50
CTGCACGTCA TAAGGCTAAC CTGACTGCAC AGATCAACAA ACTGGCTTAA 100
<210> 7300
<211> 100
<212> DNA
<213> Escherichia coli K-12 MG1655
<220> rpst rpsT
<222> 91-190
<223> rpst rpsT U00096 complement(20815..21078)
<313> 7300: 145-164

<400> 7300
TTCATCAAGA AAGTATACGC AGCTATCGAA GCTGGCGACA AAGCTGCTGC 50
ACAGAAAGCA TTTAACGAAA TGCAACCGAT CGTGGACCGT CAGGCTGCTA 100
<210> 7301
<211> 100
<212> DNA
<213> Escherichia coli K-12 MG1655
<220> rpst rpsT
<222> 59-158
<223> rpst rpsT U00096 complement(20815..21078)
<313> 7301: 107-126
<400> 7301
ACAACGCAAG CCGTCGCTCT ATGATGCGTA CTTTCATCAA GAAAGTATAC 50
GCAGCTATCG AAGCTGGCGA CAAAGCTGCT GCACAGAAAG CATTTAACGA 100
<210> 7302
<211> 100
<212> DNA
<213> Escherichia coli K-12 MG1655
<220> rard b3820
<222> 804-903
<223> rard b3820 U00096 complement(4000900..4001802)
<313> 7302: 867-886
<400> 7302
ACCGGGTGCC GATAAGATGG TGACTTTCGC CTTTATTTGG GTGGCGCTGG 50
CAATTTTTGT GATGGATGCG ATTTATACGC AGCGTAGAAC GTCCAAATAA 100
<210> 7303
<211> 100
<212> DNA
<213> Escherichia coli K-12 MG1655
<220> rard b3820
<222> 772-871
<223> rard b3820 U00096 complement(4000900..4001802)
<313> 7303: 820-839
<400> 7303
TTCCTGCTGG CTGTGACGTT TTATGGTGAA AAACCGGGTG CCGATAAGAT 50
GGTGACTTTC GCCTTTATTT GGGTGGCGCT GGCAATTTTT GTGATGGATG 100
<210> 7304
<211> 100
<212> DNA
<213> Escherichia coli K-12 MG1655
<220> rard b3820
<222> 740-839
<223> rard b3820 U00096 complement(4000900..4001802)
<313> 7304: 773-792
<400> 7304
GCTTTTTCCA GTACATTGGC CCGACGCTGA TGTTCCTGCT GGCTGTGACG 50
TTTTATGGTG AAAAACCGGG TGCCGATAAG ATGGTGACTT TCGCCTTTAT 100
<210> 7305
<211> 100
<212> DNA
<213> Escherichia coli K-12 MG1655
<220> rpsu b3066
<222> 77-176
<223> rpsu b3066 U00096 3208422..3208637
<313> 7305: 113-132
<400> 7305
CAGGTGTTCT GGCGGAAGTT CGTCGTCGTG AGTTCTATGA AAAACCGACT 50
ACCGAACGTA AGCGCGCTAA AGCTTCTGCA GTGAAACGTC ACGCGAAGAA 100
<210> 7306
<211> 100
<212> DNA
<213> Escherichia coli K-12 MG1655
<220> rpsu b3066
<222> 30-129
<223> rpsu b3066 U00096 3208422..3208637
<313> 7306: 59-78
<400> 7306
GCCGTTCGAC GTAGCTCTGC GTCGCTTCAA GCGTTCCTGC GAAAAAGCAG 50
GTGTTCTGGC GGAAGTTCGT CGTCGTGAGT TCTATGAAAA ACCGACTACC 100
<210> 7307
<211> 100

<212> DNA
<213> Escherichia coli K-12 MG1655
<220> rpsu b3066
<222> 1-100
<223> rpsu b3066 U00096 3208422..3208637
<313> 7307: 29-48
<400> 7307
ATGCCGGTAA TTAAAGTACG TGAAAACGAG CCGTTCGACG TAGCTCTGCG 50
TCGCTTCAAG CGTTCCTGCG AAAAAGCAGG TGTTCTGGCG GAAGTTCGTC 100
<210> 7308
<211> 100
<212> DNA
<213> Escherichia coli K-12 MG1655
<220> rpsv rpsV
<222> 39-138
<223> rpsv rpsV U00096 complement(1553850..1553987)
<313> 7308: 118-137
<400> 7308
GGACCATAAA ATTTCTAACC AGCGCAAAAT AGTTACCGAA GGTGACAAAT 50
CCAGCGTAGT AAATAACCCA ACCGGCAGAA AACGCCCCGC TGAAAAGTAA 100
<210> 7309
<211> 100
<212> DNA
<213> Escherichia coli K-12 MG1655
<220> rpsv rpsV
<222> 19-118
<223> rpsv rpsV U00096 complement(1553850..1553987)
<313> 7309: 67-86
<400> 7309
GCACGTCATA TTCTTGGACT GGACCATAAA ATTTCTAACC AGCGCAAAAT 50
AGTTACCGAA GGTGACAAAT CCAGCGTAGT AAATAACCCA ACCGGCAGAA 100
<210> 7310
<211> 100
<212> DNA
<213> Escherichia coli K-12 MG1655
<220> rpsv rpsV
<222> 1-100
<223> rpsv rpsV U00096 complement(1553850..1553987)
<313> 7310: 20-39
<400> 7310
ATGAAATCGA ACCGTCAGGC ACGTCATATT CTTGGACTGG ACCATAAAAT 50
TTCTAACCAG CGCAAAATAG TTACCGAAGG TGACAAATCC AGCGTAGTAA 100
<210> 7311
<211> 100
<212> DNA
<213> Escherichia coli K-12 MG1655
<220> suge b4149
<222> 369-468
<223> suge b4149 U00096 4374303..4374770
<313> 7311: 421-440
<400> 7311
CGGCATTGTG CTGCTCGGTG AGTCCGCTAA CCCGATGCGC CTGGCGAGTC 50
TGGCGTTAAT CGTATTGGGG ATTATTGGTC TGAAACTCAG CACTCACTAA 100
<210> 7312
<211> 100
<212> DNA
<213> Escherichia coli K-12 MG1655
<220> suge b4149
<222> 360-459
<223> suge b4149 U00096 4374303..4374770
<313> 7312: 385-404
<400> 7312
GGCCATAACC GGCATTGTGC TGCTCGGTGA GTCCGCTAAC CCGATGCGCC 50
TGGCGAGTCT GGCGTTAATC GTATTGGGGA TTATTGGTCT GAAACTCAGC 100
<210> 7313
<211> 100
<212> DNA
<213> Escherichia coli K-12 MG1655
<220> suge b4149
<222> 299-398
<223> suge b4149 U00096 4374303..4374770

<313> 7313: 334-353
<400> 7313
TGAAATCGTT ACCAGTAGGG ACGGCTTATG CCGTGTGGAC GGGTATTGGC 50
GCAGTCGGCG CGGCCATAAC CGGCATTGTG CTGCTCGGTG AGTCCGCTAA 100
<210> 7314
<211> 100
<212> DNA
<213> Escherichia coli K-12 MG1655
<220> coda b0338
<222> 1185-1284
<223> coda b0338 U00096 355395..356678
<313> 7314: 1247-1266
<400> 7314
ACGTTATTCG GTACGTGGCG GCAAGGTGAT TGCCAGCACA CAACCGGCAC 50
AAACCACCGT ATATCTGGAG CAGCCAGAAG CCATCGATTA CAAACGTTGA 100
<210> 7315
<211> 100
<212> DNA
<213> Escherichia coli K-12 MG1655
<220> coda b0338
<222> 1162-1261
<223> coda b0338 U00096 355395..356678
<313> 7315: 1196-1215
<400> 7315
GCGCTGCGCC GTCAGGTTCC GGTACGTTAT TCGGTACGTG GCGGCAAGGT 50
GATTGCCAGC ACACAACCGG CACAAACCAC CGTATATCTG GAGCAGCCAG 100
<210> 7316
<211> 100
<212> DNA
<213> Escherichia coli K-12 MG1655
<220> coda b0338
<222> 1069-1168
<223> coda b0338 U00096 355395..356678
<313> 7316: 1110-1129
<400> 7316
CACAGCGCAA GGACGTTGAA TTTGCAGGAT TACGGCATTG CCGCCGGAAA 50
CAGCGCCAAC CTGATTATCC TGCCGGCTGA AAATGGGTTT GATGCGCTGC 100
<210> 7317
<211> 100
<212> DNA
<213> Escherichia coli K-12 MG1655
<220> codb b0337
<222> 1161-1260
<223> codb b0337 U00096 354146..355405
<313> 7317: 1214-1233
<400> 7317
GGTATTAGGT GGCGCGCTGA GCTATCTGAT CCTTAACCCG ATTTTGAATC 50
GTAAAACGAC AGCAGCAATG ACGCATGTGG AGGCTAACAG TGTCGAATAA 100
<210> 7318
<211> 100
<212> DNA
<213> Escherichia coli K-12 MG1655
<220> codb b0337
<222> 1018-1117
<223> codb b0337 U00096 354146..355405
<313> 7318: 1048-1067
<400> 7318
ATCGCCGACT ATCTGATGAA CCGTCGCCGC TATGAGCACT TTGCGACCAC 50
GCGTATGATG AGTGTCAATT GGGTGGCGAT TCTGGCGGTC GCCTTGGGGA 100
<210> 7319
<211> 100
<212> DNA
<213> Escherichia coli K-12 MG1655
<220> codb b0337
<222> 948-1047
<223> codb b0337 U00096 354146..355405
<313> 7319: 979-998
<400> 7319
GCTGTATAAC AATTTTGTCG GCTGGTTGAC CTTCCTTTCG GCAGCTATTC 50
CTCCAGTGGG TGGCGTGATC ATCGCCGACT ATCTGATGAA CCGTCGCCGC 100
<210> 7320

<211> 100
<212> DNA
<213> Escherichia coli K-12 MG1655
<220> gatr_1 gatR_1
<222> 348-447
<223> gatr_1 gatR_1 U00096 complement(2167715..2168161)
<313> 7320: 412-431
<400> 7320
ACCAATCGAA AAAATTGACA TTATTATTAC CGATGATGCT GTGTCAGAGG 50
TGGATAAACT GGCATTGCAG AAGACGCGGG TAAAATTAAT TACGGTGTAG 100
<210> 7321
<211> 100
<212> DNA
<213> Escherichia coli K-12 MG1655
<220> gatr_1 gatR_1
<222> 256-355
<223> gatr_1 gatR_1 U00096 complement(2167715..2168161)
<313> 7321: 285-304
<400> 7321
TCTATTAGCG GTGCGATGGT CACAGCCGCT AATAAAGTGA TTGCCGTTCT 50
CGATTCATCG AAATTTAACC GTCGCGGTTT TAATCAGGTA CTACCAATCG 100
<210> 7322
<211> 100
<212> DNA
<213> Escherichia coli K-12 MG1655
<220> gatr_1 gatR_1
<222> 74-173
<223> gatr_1 gatR_1 U00096 complement(2167715..2168161)
<313> 7322: 140-159
<400> 7322
CCCTTTCCGA AAATAAAGAC ATTACTCTGG TCGTCTGTGG TGGCACGGTC 50
CGCCATAAAA CGCGCTCGAT GCATGGTTCC ATTGCCGAGC GTTCATTACA 100
<210> 7323
<211> 100
<212> DNA
<213> Escherichia coli K-12 MG1655
<220> gatr_2 b2091
<222> 240-339
<223> gatr_2 b2091 U00096 complement(2169417..2169755)
<313> 7323: 313-332
<400> 7323
GCTCGCCAGC GCGCCAAAAA ACAGAATAGC GCAGGCGGCA GTCAAAATGA 50
TCCACGAAGG GATGACTGAT CCTACCCACG TAATATGGAC ACAGGCCTAA 100
<210> 7324
<211> 100
<212> DNA
<213> Escherichia coli K-12 MG1655
<220> gatr_2 b2091
<222> 216-315
<223> gatr_2 b2091 U00096 complement(2169417..2169755)
<313> 7324: 258-277
<400> 7324
AGTCGGGTTT AAAGAGCGAT TTCAGCTCGC CAGCGCGCCA AAAAACAGAA 50
TAGCGCAGGC GGCAGTCAAA ATGATCCACG AAGGGATGAC TGATCCTACC 100
<210> 7325
<211> 100
<212> DNA
<213> Escherichia coli K-12 MG1655
<220> gatr_2 b2091
<222> 176-275
<223> gatr_2 b2091 U00096 complement(2169417..2169755)
<313> 7325: 213-232
<400> 7325
CGAAAATAAT GTCTGGTAAT AGTGAAACCG AGACCCAGGA AGTCGGGTTT 50
AAAGAGCGAT TTCAGCTCGC CAGCGCGCCA AAAAACAGAA TAGCGCAGGC 100
<210> 7326
<211> 100
<212> DNA
<213> Escherichia coli K-12 MG1655
<220> gcpe b2516
<222> 940-1039

<223> gcpe b2516 U00096 complement(2638706..2639824)
<313> 7326: 970-989
<400> 7326
CTGGTTTCTA CACTCGGCGT CACCGGCGGC AACAAGAAAA GCGGCCTCTA 50
TGAAGATGGC GTGCGCAAAG ACCGTCTGGA CAACAACGAT ATGATCGACC 100
<210> 7327
<211> 100
<212> DNA
<213> Escherichia coli K-12 MG1655
<220> gcpe b2516
<222> 875-974
<223> gcpe b2516 U00096 complement(2638706..2639824)
<313> 7327: 928-947
<400> 7327
AAGATATCAT CACTCCGATG GACGTTTCGA TTATCGGCTG CGTGGTGAAT 50
GGCCCAGGTG AGGCGCTGGT TTCTACACTC GGCGTCACCG GCGGCAACAA 100
<210> 7328
<211> 100
<212> DNA
<213> Escherichia coli K-12 MG1655
<220> gcpe b2516
<222> 851-950
<223> gcpe b2516 U00096 complement(2638706..2639824)
<313> 7328: 871-890
<400> 7328
TTAACGCGCT GGAGCAACGC CTGGAAGATA TCATCACTCC GATGGACGTT 50
TCGATTATCG GCTGCGTGGT GAATGGCCCA GGTGAGGCGC TGGTTTCTAC 100
<210> 7329
<211> 100
<212> DNA
<213> Escherichia coli K-12 MG1655
<220> wbbh b2036
<222> 925-1024
<223> wbbh b2036 U00096 complement(2104082..2105248)
<313> 7329: 993-1012
<400> 7329
TATGTTTATA TTTCCGCGGA GCTAAGCTAT TTGATGATGG TTATTCATGG 50
CTGTATTTCA GGTGTTTTAT GGAGATTGTC TCGAAATTAC ATATCTGTGA 100
<210> 7330
<211> 100
<212> DNA
<213> Escherichia coli K-12 MG1655
<220> wbbh b2036
<222> 951-1050
<223> wbbh b2036 U00096 complement(2104082..2105248)
<313> 7330: 976-995
<400> 7330
CTATTTGATG ATGGTTATTC ATGGCTGTAT TTCAGGTGTT TTATGGAGAT 50
TGTCTCGAAA TTACATATCT GTGAAAATAT TTTATTCATA TTTTATTTAT 100
<210> 7331
<211> 100
<212> DNA
<213> Escherichia coli K-12 MG1655
<220> wbbh b2036
<222> 911-1010
<223> wbbh b2036 U00096 complement(2104082..2105248)
<313> 7331: 963-982
<400> 7331
CTGCTTTTTC GGATTATGTT TATATTTCCG CGGAGCTAAG CTATTTGATG 50
ATGGTTATTC ATGGCTGTAT TTCAGGTGTT TTATGGAGAT TGTCTCGAAA 100
<210> 7332
<211> 100
<212> DNA
<213> Escherichia coli K-12 MG1655
<220> wbbi b2035
<222> 741-840
<223> wbbi b2035 U00096 complement(2103087..2104079)
<313> 7332: 774-793
<400> 7332
ATCTCTTTAT CTTTCAATGG AACTTCCAGT ATTTATATGG GATAAAGCCG 50
CCCTTGCGGA TTTCATTGTA GATAATAGAA TAGGATATGC AGTGGGATCA 100

<210> 7333
<211> 100
<212> DNA
<213> Escherichia coli K-12 MG1655
<220> wbbi b2035
<222> 650-749
<223> wbbi b2035 U00096 complement(2103087..2104079)
<313> 7333: 685-704
<400> 7333
TGCAATTTGG ACTCATTTGG GATGGAGATT CTGTCGAAAC CTGTAGTGGT 50
GCCTTTGGCG ACTATTTAAA GTTTAATAAC CCTCATAAGA CATCTCTTTA 100
<210> 7334
<211> 100
<212> DNA
<213> Escherichia coli K-12 MG1655
<220> wbbi b2035
<222> 439-538
<223> wbbi b2035 U00096 complement(2103087..2104079)
<313> 7334: 468-487
<400> 7334
CTCGTCTCAT CTGATGTGGA GCATCGAGAT GTTACGGATA AGCAACGAGG 50
GGTCATATAT GCTGGCAACC TTTCTAGGCA TAAATGTTCT TTCATATATA 100
<210> 7335
<211> 100
<212> DNA
<213> Escherichia coli K-12 MG1655
<220> wbbj b2034
<222> 463-562
<223> wbbj b2034 U00096 complement(2102516..2103106)
<313> 7335: 516-535
<400> 7335
AATGGAGTCG TAGTCGGCGC CAATTCTGTT GTTAGAGGTT CTATTCCCGA 50
AAATACTGTC ATTGCGGGAG TACCAGCAAA AATCATAAAG AAATACAATC 100
<210> 7336
<211> 100
<212> DNA
<213> Escherichia coli K-12 MG1655
<220> wbbj b2034
<222> 389-488
<223> wbbj b2034 U00096 complement(2102516..2103106)
<313> 7336: 437-456
<400> 7336
CTTCAGCTGT TGTAATTGGC CAGAGGGTTT GGTTGGGTGA GAATGTGACG 50
GTTTTGCCTG GAACAATTAT TGGTAATGGA GTCGTAGTCG GCGCCAATTC 100
<210> 7337
<211> 100
<212> DNA
<213> Escherichia coli K-12 MG1655
<220> wbbj b2034
<222> 296-395
<223> wbbj b2034 U00096 complement(2102516..2103106)
<313> 7337: 344-363
<400> 7337
AAGTATTTAT TACCGATCAT AATCACGGTT CCTTTAAGCA CTCTGATCCA 50
ATGAGTTCGC CAAATATACC TCCAGACATG CGCACGTTGG AATCTTCAGC 100
<210> 7338
<211> 100
<212> DNA
<213> Escherichia coli K-12 MG1655
<220> wbbk b2033
<222> 824-923
<223> wbbk b2033 U00096 complement(2101413..2102531)
<313> 7338: 877-896
<400> 7338
ATATAGTTTG TTTTCCCTCT AGGTTAGAAA CATGGGGATT GCCGTTGTCT 50
GAAGCTAAAG AGCGAGGTAA GTGGGTATTA GCATCAGATT TCCCATTTAC 100
<210> 7339
<211> 100
<212> DNA
<213> Escherichia coli K-12 MG1655
<220> wbbk b2033

<222> 708-807
<223> wbbk b2033 U00096 complement(2101413..2102531)
<313> 7339: 762-781
<400> 7339
TGGTACAGAA AATGCGTATG CAAAATATAT TATCAGTCTT GCAGAAGGAC  50
TGGATAATGT TCATTTCCTC GGGTACTTGG ATAAAGAAAA AATCGATCAT  100
<210> 7340
<211> 100
<212> DNA
<213> Escherichia coli K-12 MG1655
<220> wbbk b2033
<222> 540-639
<223> wbbk b2033 U00096 complement(2101413..2102531)
<313> 7340: 596-615
<400> 7340
TGATAAAAGC CAACTTACTG ATGATGATTC TCAATTTAAG AATAACCCTT  50
CTGAGTTGAC AATATTTTAC CCTGCTGTTC CACGAGTATT TAAAAATTAC  100
<210> 7341
<211> 100
<212> DNA
<213> Escherichia coli K-12 MG1655
<220> b0235 b0236
<222> 163-262
<223> b0235 b0236 U00096 253467..253733
<313> 7341: 184-203
<400> 7341
GGACGCGGCT ACCCGATTGG CGCAGCGTTC TTCTCTGTTG GTCGTTTTTA  50
CCCGGCAAGA CGTCGCGGTA ACGGTGCTGG AAACAGAAAC GGGCCGCTAC  100
<210> 7342
<211> 100
<212> DNA
<213> Escherichia coli K-12 MG1655
<220> b0235 b0236
<222> 96-195
<223> b0235 b0236 U00096 253467..253733
<313> 7342: 147-166
<400> 7342
ACAATTACAC ACCACGGCAA ATTTACCCAA CATGCAGCGC GTAGTGGGGA  50
TGCCAGATTT ACACCCCGGA CGCGGCTACC CGATTGGCGC AGCGTTCTTC  100
<210> 7343
<211> 100
<212> DNA
<213> Escherichia coli K-12 MG1655
<220> b0235 b0236
<222> 60-159
<223> b0235 b0236 U00096 253467..253733
<313> 7343: 107-126
<400> 7343
ATCTGATGAC CTGTGGATCG AGAGTTTAGC GATCCAACAA TTACACACCA  50
CGGCAAATTT ACCCAACATG CAGCGCGTAG TGGGGATGCC AGATTTACAC  100
<210> 7344
<211> 100
<212> DNA
<213> Escherichia coli K-12 MG1655
<220> trka b3291
<222> 1278-1377
<223> trka b3291 U00096 3434155..3435531
<313> 7344: 1355-1374
<400> 7344
GCGCATTGAG CAAGGCGATC ACGTAATTAT GTTCCTCACA GATAAAAAGT  50
TTATTACCGA CGTCGAAAGA CTCTTCCAGC CAAGCCCTTT CTTCTTGTAA  100
<210> 7345
<211> 100
<212> DNA
<213> Escherichia coli K-12 MG1655
<220> trka b3291
<222> 1207-1306
<223> trka b3291 U00096 3434155..3435531
<313> 7345: 1243-1262
<400> 7345
AAGCTACCGC CAGGAACGAT TATTGGAGCG GTGGTACGTG GAAACGACGT  50

GATGATTGCC AATGACAATC TGCGCATTGA GCAAGGCGAT CACGTAATTA 100
<210> 7346
<211> 100
<212> DNA
<213> Escherichia coli K-12 MG1655
<220> trka b3291
<222> 1172-1271
<223> trka b3291 U00096 3434155..3435531
<313> 7346: 1207-1226
<400> 7346
CCTCACGCGT TGTCGGCAGA GTCATTGACG AAATCAAGCT ACCGCCAGGA 50
ACGATTATTG GAGCGGTGGT ACGTGGAAAC GACGTGATGA TTGCCAATGA 100
<210> 7347
<211> 100
<212> DNA
<213> Escherichia coli K-12 MG1655
<220> suhb suhB
<222> 553-652
<223> suhb suhB U00096 2661462..2662265
<313> 7347: 587-606
<400> 7347
ACCGGTTCTG CGGCGCTGGA TCTGGCTTAC GTCGCTGCGG GTCGTGTTGA 50
CGGTTTCTTT GAAATCGGTC TGCGCCCGTG GGACTTCGCC GCAGGCGAGC 100
<210> 7348
<211> 100
<212> DNA
<213> Escherichia coli K-12 MG1655
<220> suhb suhB
<222> 472-571
<223> suhb suhB U00096 2661462..2662265
<313> 7348: 519-538
<400> 7348
CCGTTCAAAG CAAAACAGTA CGCCACTACC TACATCAACA TCGTCGGCAA 50
ACTGTTCAAC GAATGTGCAG ACTTCCGTCG TACCGGTTCT GCGGCGCTGG 100
<210> 7349
<211> 100
<212> DNA
<213> Escherichia coli K-12 MG1655
<220> suhb suhB
<222> 460-559
<223> suhb suhB U00096 2661462..2662265
<313> 7349: 491-510
<400> 7349
GCGACCGGCT TCCCGTTCAA AGCAAAACAG TACGCCACTA CCTACATCAA 50
CATCGTCGGC AAACTGTTCA ACGAATGTGC AGACTTCCGT CGTACCGGTT 100
<210> 7350
<211> 100
<212> DNA
<213> Escherichia coli K-12 MG1655
<220> trkg trkG
<222> 1293-1392
<223> trkg trkG U00096 1421806..1423263
<313> 7350: 1353-1372
<400> 7350
GGCTGCATGT ATTAATAATA TGGGATTAGG TTTTGGGGCT ACTGCATCGT 50
CATTCGGAGT GCTTAATGAC ATTGCAAAAT GCTTAATGTG CATAGCTATG 100
<210> 7351
<211> 100
<212> DNA
<213> Escherichia coli K-12 MG1655
<220> trkg trkG
<222> 1106-1205
<223> trkg trkG U00096 1421806..1423263
<313> 7351: 1142-1161
<400> 7351
AACACGAGAT AAATCAGCTT TCTCATCCCA GAGCGTTGTT GAGTGTAAAT 50
GTAGGAGGGA AGATAGTTAC AGATCGTGTA ATGAGGTCTG TATGGAGTTT 100
<210> 7352
<211> 100
<212> DNA
<213> Escherichia coli K-12 MG1655

<220> trkg trkG
<222> 962-1061
<223> trkg trkG U00096 1421806..1423263
<313> 7352: 991-1010
<400> 7352
ATGGTTTAGC TACGCAGGAT TATGCAAGTT GGCCCACGCA CACGATAGTG 50
TTTTTGCTGT TGTCAAGTTT CTTTGGGGGA TGTATAGGTT CAACTTGTGG 100
<210> 7353
<211> 100
<212> DNA
<213> Escherichia coli K-12 MG1655
<220> trkh b3850
<222> 1191-1290
<223> trkh b3850 U00096 4030735..4032033
<313> 7353: 1240-1259
<400> 7353
TTGGGGATTT TTCTCCGCCT ATGCATTGGT GTTTATTGTC AGTATGCTGG 50
CGATTATCGC ACGGGCGTGG ATGACTTTTC TGCCTTTGCG TCGGTTGTTG 100
<210> 7354
<211> 100
<212> DNA
<213> Escherichia coli K-12 MG1655
<220> trkh b3850
<222> 1156-1255
<223> trkh b3850 U00096 4030735..4032033
<313> 7354: 1184-1203
<400> 7354
AATCGCGCAC TGCCGGAACG TATCCTCGAA GCCGTTTGGG GATTTTTCTC 50
CGCCTATGCA TTGGTGTTTA TTGTCAGTAT GCTGGCGATT ATCGCACGGG 100
<210> 7355
<211> 100
<212> DNA
<213> Escherichia coli K-12 MG1655
<220> trkh b3850
<222> 1117-1216
<223> trkh b3850 U00096 4030735..4032033
<313> 7355: 1150-1169
<400> 7355
CTGGTGCATC CGAACGCCGT CTATAGCATT AAGCTGGGGA ATCGCGCACT 50
GCCGGAACGT ATCCTCGAAG CCGTTTGGGG ATTTTTCTCC GCCTATGCAT 100
<210> 7356
<211> 100
<212> DNA
<213> Escherichia coli K-12 MG1655
<220> b0245 b0246
<222> 190-289
<223> b0245 b0246 U00096 complement(262552..262893)
<313> 7356: 236-255
<400> 7356
AATTTTCTGG TAGAAAAATA CGAGCTGGTT CGTATCGACA GGAAGGGATT 50
TAGCTGGCAG GAACAATCTC CTTATCTCCG GGCTGCAGAC ATTCTGCGAG 100
<210> 7357
<211> 100
<212> DNA
<213> Escherichia coli K-12 MG1655
<220> b0245 b0246
<222> 146-245
<223> b0245 b0246 U00096 complement(262552..262893)
<313> 7357: 196-215
<400> 7357
TTAAGGAACA CATCGATGCC GGTATCACCC TAGCCGATGC CGTGAATTTT 50
CTGGTAGAAA AATACGAGCT GGTTCGTATC GACAGGAAGG GATTTAGCTG 100
<210> 7358
<211> 100
<212> DNA
<213> Escherichia coli K-12 MG1655
<220> b0245 b0246
<222> 104-203
<223> b0245 b0246 U00096 complement(262552..262893)
<313> 7358: 140-159
<400> 7358

GTCTGACAAT AAACGACACG CCATTCTGCA ATGAGGCTGT GATTAAGGAA 50
CACATCGATG CCGGTATCAC CCTAGCCGAT GCCGTGAATT TTCTGGTAGA 100
<210> 7359
<211> 100
<212> DNA
<213> Escherichia coli K-12 MG1655
<220> blc b4150
<222> 401-500
<223> blc b4150 U00096 complement(4374767..4375300)
<313> 7359: 438-457
<400> 7359
ACCGCGACTA CCTGTGGATA CTCTCCCGCA CGCCAACCAT TTCTGACGAA 50
GTGAAACAGG AGATGCTGGC AGTCGCGACC CGGGAAGGGT TTGATGTCAG 100
<210> 7360
<211> 100
<212> DNA
<213> Escherichia coli K-12 MG1655
<220> blc b4150
<222> 271-370
<223> blc b4150 U00096 complement(4374767..4375300)
<313> 7360: 320-339
<400> 7360
GGGAAAGCGT ACTTTACCGG CGCACCAACT CGCGCTGCGC TGAAAGTGTC 50
ATTCTTTGGT CCTTTCTATG GCGGTTATAA CGTTATTGCA CTCGATCGGG 100
<210> 7361
<211> 100
<212> DNA
<213> Escherichia coli K-12 MG1655
<220> blc b4150
<222> 235-334
<223> blc b4150 U00096 complement(4374767..4375300)
<313> 7361: 279-298
<400> 7361
TATAACCCTG ACAGAGGAAT GTGGCAGCAG AGTGAAGGGA AAGCGTACTT 50
TACCGGCGCA CCAACTCGCG CTGCGCTGAA AGTGTCATTC TTTGGTCCTT 100
<210> 7362
<211> 100
<212> DNA
<213> Escherichia coli K-12 MG1655
<220> ampc b4151
<222> 996-1095
<223> ampc b4151 U00096 complement(4375389..4376522)
<313> 7362: 1023-1042
<400> 7362
AGGGGCGACC GGCGGATTTG GTAGCTATGT CGCGTTTATT CCAGAAAAAG 50
AGCTGGGTAT CGTGATGCTG GCAAACAAAA ACTATCCCAA TCCAGCGAGA 100
<210> 7363
<211> 100
<212> DNA
<213> Escherichia coli K-12 MG1655
<220> ampc b4151
<222> 965-1064
<223> ampc b4151 U00096 complement(4375389..4376522)
<313> 7363: 987-1006
<400> 7363
CTGCAGTACG CGCATCATGG GTACATAAAA CAGGGGCGAC CGGCGGATTT 50
GGTAGCTATG TCGCGTTTAT TCCAGAAAAA GAGCTGGGTA TCGTGATGCT 100
<210> 7364
<211> 100
<212> DNA
<213> Escherichia coli K-12 MG1655
<220> ampc b4151
<222> 906-1005
<223> ampc b4151 U00096 complement(4375389..4376522)
<313> 7364: 941-960
<400> 7364
CAGTGACAAT AAAATTGCAC TGGCAGCACG CCCCGTAAAA GCGATTACGC 50
CCCCAACTCC TGCAGTACGC GCATCATGGG TACATAAAAC AGGGGCGACC 100
<210> 7365
<211> 100
<212> DNA

<213> Escherichia coli K-12 MG1655
<220> ampd b0111
<222> 265-364
<223> ampd b0111 U00096 118733..119284
<313> 7365: 321-340
<400> 7365
CCTTTCGATA AACGTGCATG GCATGCGGGA GTCTCTCAGT ATCAGGGGCG 50
CGAACGCTGC AATGATTTTT CTATTGGGAT TGAGCTTGAA GGCACCGATA 100
<210> 7366
<211> 100
<212> DNA
<213> Escherichia coli K-12 MG1655
<220> ampd b0111
<222> 168-267
<223> ampd b0111 U00096 118733..119284
<313> 7366: 211-230
<400> 7366
TGATCCGCAG GCACATCCTT TCTTTGCTGA GATCGCCCAT TTGCGCGTCT 50
CCGCTCACTG TTTGATTCGC CGTGATGGTG AAATAGTCCA GTATGTTCCT 100
<210> 7367
<211> 100
<212> DNA
<213> Escherichia coli K-12 MG1655
<220> ampd b0111
<222> 146-245
<223> ampd b0111 U00096 118733..119284
<313> 7367: 184-203
<400> 7367
ACGCATTATT CACTGGAACT ATTGATCCGC AGGCACATCC TTTCTTTGCT 50
GAGATCGCCC ATTTGCGCGT CTCCGCTCAC TGTTTGATTC GCCGTGATGG 100
<210> 7368
<211> 100
<212> DNA
<213> Escherichia coli K-12 MG1655
<220> sfhb sfhB
<222> 872-971
<223> sfhb sfhB U00096 complement(2733051..2734031)
<313> 7368: 906-925
<400> 7368
CCGGCATCGA AATGGAATGG CATGCGCCTA TTCCACAAGA TATGGTGGAG 50
CTGATTGAGG TGATGCGCGC CGATTTCGAA GAACATAAGG ATGAAGTGGA 100
<210> 7369
<211> 100
<212> DNA
<213> Escherichia coli K-12 MG1655
<220> sfhb sfhB
<222> 844-943
<223> sfhb sfhB U00096 complement(2733051..2734031)
<313> 7369: 875-894
<400> 7369
ACCATGCTGC GTCTTTATCA CCCGATCTCC GGCATCGAAA TGGAATGGCA 50
TGCGCCTATT CCACAAGATA TGGTGGAGCT GATTGAGGTG ATGCGCGCCG 100
<210> 7370
<211> 100
<212> DNA
<213> Escherichia coli K-12 MG1655
<220> sfhb sfhB
<222> 767-866
<223> sfhb sfhB U00096 complement(2733051..2734031)
<313> 7370: 827-846
<400> 7370
GTCCGCGTCC GCCAAAAGGT GCTTCGGAAG CATTTATCTC CACGCTGCGT 50
AAGTTTGACC GCCAGGCGCT ACATGCAACC ATGCTGCGTC TTTATCACCC 100
<210> 7371
<211> 100
<212> DNA
<213> Escherichia coli K-12 MG1655
<220> ampe b0112
<222> 753-852
<223> ampe b0112 U00096 119281..120135
<313> 7371: 776-795

<400> 7371
TAAGGTGGAG ACGCCGAAGG CAGCGGTTTC AATGGCGAAG AAAACCTCGT 50
TCGTGGTCGT GGTGGTGATT GCACTACTGA CGATTTACGG GGCGTTGGTG 100
<210> 7372
<211> 100
<212> DNA
<213> Escherichia coli K-12 MG1655
<220> ampe b0112
<222> 692-791
<223> ampe b0112 U00096 119281..120135
<313> 7372: 717-736
<400> 7372
CGCAGTATCA GGTGTTAACG CGTCTGGCGC AGTTCTCTCT GGCGCGTGAA 50
CCGCATGTCG ATAAGGTGGA GACGCCGAAG GCAGCGGTTT CAATGGCGAA 100
<210> 7373
<211> 100
<212> DNA
<213> Escherichia coli K-12 MG1655
<220> ampe b0112
<222> 654-753
<223> ampe b0112 U00096 119281..120135
<313> 7373: 671-690
<400> 7373
ACCGGCCTGG TTTGCTTCGC TGGGTGATTT CCATACTTCG CAGTATCAGG 50
TGTTAACGCG TCTGGCGCAG TTCTCTCTGG CGCGTGAACC GCATGTCGAT 100
<210> 7374
<211> 100
<212> DNA
<213> Escherichia coli K-12 MG1655
<220> ampg b0434
<222> 1333-1432
<223> ampg b0434 U00096 complement(451294..452769)
<313> 7374: 1380-1399
<400> 7374
CTATTGACGA TGGACGCGCT GGATTTGACG CACTTCTCTT TCCTGCCTGC 50
TCTGCTGGAA GTCGGGGTTT TAGTCGCCCT TTCTGGCGTC GTGCTTGGTG 100
<210> 7375
<211> 100
<212> DNA
<213> Escherichia coli K-12 MG1655
<220> ampg b0434
<222> 1297-1396
<223> ampg b0434 U00096 complement(451294..452769)
<313> 7375: 1344-1363
<400> 7375
GCGGCGGGCG TCAGCCTGTT GGCCGTGTGG TTACTGCTAT TGACGATGGA 50
CGCGCTGGAT TTGACGCACT TCTCTTTCCT GCCTGCTCTG CTGGAAGTCG 100
<210> 7376
<211> 100
<212> DNA
<213> Escherichia coli K-12 MG1655
<220> ampg b0434
<222> 1224-1323
<223> ampg b0434 U00096 complement(451294..452769)
<313> 7376: 1269-1288
<400> 7376
TACACGAGTA AATGACAACT TTATCTCCCG TACCGCATAT CCGGCAGGTT 50
ATGCCTTTGC CATGTGGACA CTGGCGGCGG GCGTCAGCCT GTTGGCCGTG 100
<210> 7377
<211> 100
<212> DNA
<213> Escherichia coli K-12 MG1655
<220> b0257 b0258
<222> 327-426
<223> b0257 b0258 U00096 271054..271479
<313> 7377: 393-412
<400> 7377
ACCAAATCAG GTGTGGTGCG GTGATGTGAC CTATAGTGTG CCCGGAGTTC 50
AGGGCGGGCA TGGATGCTTA AATGAACCGC GAGTCTGTCT GGAATATTGA 100
<210> 7378
<211> 100

<212> DNA
<213> Escherichia coli K-12 MG1655
<220> b0257 b0258
<222> 319-418
<223> b0257 b0258 U00096 271054..271479
<313> 7378: 341-360
<400> 7378
GTAACGGAAC CAAATCAGGT GTGGTGCGGT GATGTGACCT ATAGTGTGCC 50
CGGAGTTCAG GGCGGGCATG GATGCTTAAA TGAACCGCGA GTCTGTCTGG 100
<210> 7379
<211> 100
<212> DNA
<213> Escherichia coli K-12 MG1655
<220> b0257 b0258
<222> 268-367
<223> b0257 b0258 U00096 271054..271479
<313> 7379: 300-319
<400> 7379
GGCGGTCATG AGCACGTTGC TATCCCGAAT CATCTTGAGC GACAGTTCGC 50
CGTAACGGAA CCAAATCAGG TGTGGTGCGG TGATGTGACC TATAGTGTGC 100
<210> 7380
<211> 100
<212> DNA
<213> Escherichia coli K-12 MG1655
<220> trma b3966
<222> 953-1052
<223> trma b3966 U00096 complement(4159749..4160849)
<313> 7380: 1003-1022
<400> 7380
GTATTTTGTA CATCTCCTGT AACCCGGAAA CGTTATGCAA GAATCTGGAA 50
ACATTAAGCC AGACGCACAA GGTCGAACGT CTGGCTCTGT TTGATCAGTT 100
<210> 7381
<211> 100
<212> DNA
<213> Escherichia coli K-12 MG1655
<220> trma b3966
<222> 898-997
<223> trma b3966 U00096 complement(4159749..4160849)
<313> 7381: 961-980
<400> 7381
CCTCCGCGCA GCGGTCTGGA CAGTGAAACC GAGAAAATGG TGCAGGCGTA 50
TCCGCGTATT TTGTACATCT CCTGTAACCC GGAAACGTTA TGCAAGAATC 100
<210> 7382
<211> 100
<212> DNA
<213> Escherichia coli K-12 MG1655
<220> trma b3966
<222> 835-934
<223> trma b3966 U00096 complement(4159749..4160849)
<313> 7382: 859-878
<400> 7382
GAGTTTAACC GCCTGCAAGG GATCGACTTA AAGAGTTATC AGTGCGAAAC 50
CATTTTTGTC GACCCTCCGC GCAGCGGTCT GGACAGTGAA ACCGAGAAAA 100
<210> 7383
<211> 100
<212> DNA
<213> Escherichia coli K-12 MG1655
<220> trmd b2608
<222> 669-768
<223> trmd b2608 U00096 complement(2742592..2743359)
<313> 7383: 745-764
<400> 7383
TCTGGAAAAC CTGGCTCTGA CTGAAGAGCA AGCAAGGTTG CTGGCGGAGT 50
TCAAAACGGA ACACGCACAA CAGCAACATA AACATGATGG GATGGCGTAA 100
<210> 7384
<211> 100
<212> DNA
<213> Escherichia coli K-12 MG1655
<220> trmd b2608
<222> 648-747
<223> trmd b2608 U00096 complement(2742592..2743359)

<313> 7384: 680-699
<400> 7384
CTGGCTTAGA AGACCTGAAC TTCTGGAAAA CCTGGCTCTG ACTGAAGAGC 50
AAGCAAGGTT GCTGGCGGAG TTCAAAACGG AACACGCACA ACAGCAACAT 100
<210> 7385
<211> 100
<212> DNA
<213> Escherichia coli K-12 MG1655
<220> trmd b2608
<222> 570-669
<223> trmd b2608 U00096 complement(2742592..2743359)
<313> 7385: 590-609
<400> 7385
GGAAGTTCCG CCAGTGTTAC TGTCGGGCAA CCATGCCGAG ATACGTCGCT 50
GGCGTTTGAA ACAGTCGCTG GGCCGTACCT GGCTTAGAAG ACCTGAACTT 100
<210> 7386
<211> 100
<212> DNA
<213> Escherichia coli K-12 MG1655
<220> pgpa b0419
<222> 408-507
<223> pgpa b0419 U00096 435813..436331
<313> 7386: 474-493
<400> 7386
CAATGTGCAT GGCGGCATGG GGATCATGAT CGACGATATT GTCGCCGGGG 50
TGATTTCCGC AGGCATCCTG TATTTTATCG GTCATCACTG GCCGCTGGGT 100
<210> 7387
<211> 100
<212> DNA
<213> Escherichia coli K-12 MG1655
<220> pgpa b0419
<222> 308-407
<223> pgpa b0419 U00096 435813..436331
<313> 7387: 344-363
<400> 7387
TGGCGCTGCC GACCAATGAC TGGCAGTGGG TTGCCGCCGG GTTTGTGATT 50
TTCCGTATTC TGGATATGTG GAAGCCGTGG CCGATCCGCT GGTTTGATCG 100
<210> 7388
<211> 100
<212> DNA
<213> Escherichia coli K-12 MG1655
<220> pgpa b0419
<222> 239-338
<223> pgpa b0419 U00096 435813..436331
<313> 7388: 280-299
<400> 7388
AAGACATGGG TGTGCACGAT CATGGCAGCA TTGTCTGGGA CGAATTTATT 50
GGTATGTGGA TCACGCTCAT GGCGCTGCCG ACCAATGACT GGCAGTGGGT 100
<210> 7389
<211> 100
<212> DNA
<213> Escherichia coli K-12 MG1655
<220> pgpb b1279
<222> 553-652
<223> pgpb b1279 U00096 1337354..1338118
<313> 7389: 590-609
<400> 7389
TTAACCATTG CTATCTTGCT GGTCTGGGCA ACGGGAGTCA TGGGAAGCCG 50
CCTGCTGCTC GGGATGCATT GGCCACGCGA TCTGGTAGTA GCTACGTTGA 100
<210> 7390
<211> 100
<212> DNA
<213> Escherichia coli K-12 MG1655
<220> pgpb b1279
<222> 425-524
<223> pgpb b1279 U00096 1337354..1338118
<313> 7390: 480-499
<400> 7390
ATATCCCACA ATATTTGCGT TCACACTGGC AGAAAGAGAC GGGGTTTGCC 50
TTTCCTTCCG GTCACACGAT GTTTGCTGCC AGTTGGGCAC TGCTGGCCGT 100
<210> 7391

<211> 100
<212> DNA
<213> Escherichia coli K-12 MG1655
<220> pgpb b1279
<222> 379-478
<223> pgpb b1279 U00096 1337354..1338118
<313> 7391: 434-453
<400> 7391
GCAGAACGCG GAAATCTAGT GAAAGAACAG TTGGCTGAAG AGAAAAATAT 50
CCCACAATAT TTGCGTTCAC ACTGGCAGAA AGAGACGGGG TTTGCCTTTC 100
<210> 7392
<211> 100
<212> DNA
<213> Escherichia coli K-12 MG1655
<220> b0263 b0264
<222> 264-363
<223> b0263 b0264 U00096 complement(278038..278400)
<313> 7392: 333-352
<400> 7392
TTCCATTCTG ATCGTGGTGA TGCTGGCGAT TATTTTCATC TTTGACTGGC 50
TGATCGGTGA ATCACGTACC TCCCGTTCAA AAGCCAAAAA CCAGGCGTAA 100
<210> 7393
<211> 100
<212> DNA
<213> Escherichia coli K-12 MG1655
<220> b0263 b0264
<222> 158-257
<223> b0263 b0264 U00096 complement(278038..278400)
<313> 7393: 192-211
<400> 7393
CCGTCAGCGC CATTGTGTTC CTCGTCACGC CGGATACCCG CGTAGCGACG 50
GCTTACATCC TCAACCGCGT GGAAGATGGC GAATACGGTG TAGCGATTGC 100
<210> 7394
<211> 100
<212> DNA
<213> Escherichia coli K-12 MG1655
<220> b0263 b0264
<222> 131-230
<223> b0263 b0264 U00096 complement(278038..278400)
<313> 7394: 164-183
<400> 7394
TTTACAGCTT TGTGCGCGCC ATTACCACCG TCAGCGCCAT TGTGTTCCTC 50
GTCACGCCGG ATACCCGCGT AGCGACGGCT TACATCCTCA ACCGCGTGGA 100
<210> 7395
<211> 100
<212> DNA
<213> Escherichia coli K-12 MG1655
<220> qor b4052
<222> 885-984
<223> qor b4052 U00096 complement(4260827..4261810)
<313> 7395: 959-978
<400> 7395
TGTCGCCGAG CAGCAGAAAT ATCCGCTGAA GGATGCGCAG CGTGCGCATG 50
AGATTCTGGA AAGCCGGGCG ACGCAAGGTT CCAGCCTGCT GATTCCATAA 100
<210> 7396
<211> 100
<212> DNA
<213> Escherichia coli K-12 MG1655
<220> qor b4052
<222> 819-918
<223> qor b4052 U00096 complement(4260827..4261810)
<313> 7396: 839-858
<400> 7396
GGAGGAATTA ACCGAGGCCA GTAATGAACT GTTCTCTTTG ATTGCCAGCG 50
GTGTGATTAA GGTCGATGTC GCCGAGCAGC AGAAATATCC GCTGAAGGAT 100
<210> 7397
<211> 100
<212> DNA
<213> Escherichia coli K-12 MG1655
<220> qor b4052
<222> 754-853

<223> qor b4052 U00096 complement(4260827..4261810)
<313> 7397: 805-824
<400> 7397
ATTCTCAATC AAAAAGGCTC GTTGTATGTG ACACGCCCTT CCCTGCAAGG 50
CTATATCACC ACGCGGGAGG AATTAACCGA GGCCAGTAAT GAACTGTTCT 100
<210> 7398
<211> 100
<212> DNA
<213> Escherichia coli K-12 MG1655
<220> ldcc b0187
<222> 2035-2134
<223> ldcc b0187 U00096 209679..211820
<313> 7398: 2093-2112
<400> 7398
CTTTGTTCCG TCGGGCAACA TTACCCCGGT TTTGAAACGG ATATTCACGG 50
CGCGAAACAG GACGAAGACG GCGTTTACCG CGTACGAGTC CTAAAAATGG 100
<210> 7399
<211> 100
<212> DNA
<213> Escherichia coli K-12 MG1655
<220> ldcc b0187
<222> 2002-2101
<223> ldcc b0187 U00096 209679..211820
<313> 7399: 2037-2056
<400> 7399
GAGAGCCGCA CAGTACTCGA TTTTCTACTG ATGCTTTGTT CCGTCGGGCA 50
ACATTACCCC GGTTTTGAAA CGGATATTCA CGGCGCGAAA CAGGACGAAG 100
<210> 7400
<211> 100
<212> DNA
<213> Escherichia coli K-12 MG1655
<220> ldcc b0187
<222> 1970-2069
<223> ldcc b0187 U00096 209679..211820
<313> 7400: 1998-2017
<400> 7400
CGCTGTTGAT GCCTGGAGAA ATGCTGACCA AAGAGAGCCG CACAGTACTC 50
GATTTTCTAC TGATGCTTTG TTCCGTCGGG CAACATTACC CCGGTTTTGA 100
<210> 7401
<211> 100
<212> DNA
<213> Escherichia coli K-12 MG1655
<220> b3100 b3101
<222> 201-300
<223> b3100 b3101 U00096 3247717..3248016
<313> 7401: 273-292
<400> 7401
TCCTAATATG CTGGTCCGCT GGGCCAGACG CGGTTTTGGC GTATGGAGCG 50
CCTGGCGTCT GGTTAAAACG ACCCTCAAGC AGCAACAGCT TCGCGGTTAA 100
<210> 7402
<211> 100
<212> DNA
<213> Escherichia coli K-12 MG1655
<220> b3100 b3101
<222> 146-245
<223> b3100 b3101 U00096 3247717..3248016
<313> 7402: 196-215
<400> 7402
TGCGCTCCTG GGCGCTGGTT GGCAGTAGCG TGATGGCGAT CTGGACGATT 50
CGCCATCCTA ATATGCTGGT CCGCTGGGCC AGACGCGGTT TTGGCGTATG 100
<210> 7403
<211> 100
<212> DNA
<213> Escherichia coli K-12 MG1655
<220> b3100 b3101
<222> 1-100
<223> b3100 b3101 U00096 3247717..3248016
<313> 7403: 41-60
<400> 7403
GTGAGCAGTA AAGTCGAACG TGAACGACGT AAGGCGCAAC TGCTTAGCCA 50
GATCCAGCAA CAACGGCTGG ATCTTTCCGC CAGTCGTCGT GAATGGCTGG 100

<210> 7404
<211> 100
<212> DNA
<213> Escherichia coli K-12 MG1655
<220> fusa b3341
<222> 1979-2078
<223> fusa b3341 U00096 complement(3469037..3471151)
<313> 7404: 2024-2043
<400> 7404
TGTCTGAAAT GTTCGGATAC GCAACTCAGC TGCGTTCTCT GACCAAAGGT  50
CGTGCATCAT ACACTATGGA ATTCCTGAAG TATGATGAAG CGCCGAGTAA  100
<210> 7405
<211> 100
<212> DNA
<213> Escherichia coli K-12 MG1655
<220> fusa b3341
<222> 1905-2004
<223> fusa b3341 U00096 complement(3469037..3471151)
<313> 7405: 1956-1975
<400> 7405
GAGCCGTCGT CGTGGTATGC TCAAAGGTCA GGAATCTGAA GTTACTGGCG  50
TTAAGATCCA CGCTGAAGTA CCGCTGTCTG AAATGTTCGG ATACGCAACT  100
<210> 7406
<211> 100
<212> DNA
<213> Escherichia coli K-12 MG1655
<220> fusa b3341
<222> 1863-1962
<223> fusa b3341 U00096 complement(3469037..3471151)
<313> 7406: 1922-1941
<400> 7406
AGAAACTCCG GAAGAGAACA CCGGTGACGT TATCGGTGAC TTGAGCCGTC  50
GTCGTGGTAT GCTCAAAGGT CAGGAATCTG AAGTTACTGG CGTTAAGATC  100
<210> 7407
<211> 100
<212> DNA
<213> Escherichia coli K-12 MG1655
<220> sula sulA
<222> 411-510
<223> sula sulA U00096 complement(1019633..1020142)
<313> 7407: 460-479
<400> 7407
AGGTAACGCT ATGGGGTTTA TTATGCGTCC GGTAAGCGCA TCCTCTCACG  50
CCACGAGACA ACTTTCCGGG CTAAAAATTC ACTCTAATTT GTATCATTAA  100
<210> 7408
<211> 100
<212> DNA
<213> Escherichia coli K-12 MG1655
<220> sula sulA
<222> 382-481
<223> sula sulA U00096 complement(1019633..1020142)
<313> 7408: 424-443
<400> 7408
CATGCTGAAC TTGTTGATGC GGCAAATGAA GGTAACGCTA TGGGGTTTAT  50
TATGCGTCCG GTAAGCGCAT CCTCTCACGC CACGAGACAA CTTTCCGGGC  100
<210> 7409
<211> 100
<212> DNA
<213> Escherichia coli K-12 MG1655
<220> sula sulA
<222> 282-381
<223> sula sulA U00096 complement(1019633..1020142)
<313> 7409: 330-349
<400> 7409
CTCCCCTTGC CACACTGTGG AGTCAATGGT TCGCGCTTTA CGCACGGGCA  50
ATTACAGTGT GGTGATCGGT TGGTTGGCAG ATGATTTGAC TGAAGAAGAG  100
<210> 7410
<211> 100
<212> DNA
<213> Escherichia coli K-12 MG1655
<220> trpa b1261

<222> 672-771
<223> trpa b1261 U00096 complement(1314440..1315246)
<313> 7410: 708-727
<400> 7410
TGATGCAGGA GCTGCGGGCG CGATTTCTGG TTCGGCCATT GTTAAAATCA  50
TCGAGCAACA TATTAATGAG CCAGAGAAAA TGCTGGCGGC ACTGAAAGTT  100
<210> 7411
<211> 100
<212> DNA
<213> Escherichia coli K-12 MG1655
<220> trpa b1261
<222> 627-726
<223> trpa b1261 U00096 complement(1314440..1315246)
<313> 7411: 664-683
<400> 7411
GCAGGGATTT GGTATTTCCG CCCCGGATCA GGTAAAAGCA GCGATTGATG  50
CAGGAGCTGC GGGCGCGATT TCTGGTTCGG CCATTGTTAA AATCATCGAG  100
<210> 7412
<211> 100
<212> DNA
<213> Escherichia coli K-12 MG1655
<220> trpa b1261
<222> 571-670
<223> trpa b1261 U00096 complement(1314440..1315246)
<313> 7412: 623-642
<400> 7412
TTACCCCTCA ATCATCTGGT TGCGAAGCTG AAAGAGTACA ACGCTGCACC  50
TCCATTGCAG GGATTTGGTA TTTCCGCCCC GGATCAGGTA AAAGCAGCGA  100
<210> 7413
<211> 100
<212> DNA
<213> Escherichia coli K-12 MG1655
<220> trpb b1262
<222> 1095-1194
<223> trpb b1262 U00096 complement(1315246..1316439)
<313> 7413: 1158-1177
<400> 7413
CCCGGATAAA GAGCAGCTAC TGGTGGTTAA CCTTTCCGGT CGCGGCGATA  50
AAGACATCTT CACCGTTCAC GATATTTTGA AAGCACGAGG GGAAATCTGA  100
<210> 7414
<211> 100
<212> DNA
<213> Escherichia coli K-12 MG1655
<220> trpb b1262
<222> 1048-1147
<223> trpb b1262 U00096 complement(1315246..1316439)
<313> 7414: 1095-1114
<400> 7414
GAATCCTCCC ACGCCCTGGC CCATGCGTTG AAAATGATGC GCGAAAACCC  50
GGATAAAGAG CAGCTACTGG TGGTTAACCT TTCCGGTCGC GGCGATAAAG  100
<210> 7415
<211> 100
<212> DNA
<213> Escherichia coli K-12 MG1655
<220> trpb b1262
<222> 974-1073
<223> trpb b1262 U00096 complement(1315246..1316439)
<313> 7415: 1005-1024
<400> 7415
TGTCTATTAC CGATGATGAA GCCCTTGAAG CCTTCAAAAC GCTGTGCCTG  50
CACGAAGGGA TCATCCCGGC GCTGGAATCC TCCCACGCCC TGGCCCATGC  100
<210> 7416
<211> 100
<212> DNA
<213> Escherichia coli K-12 MG1655
<220> trpc b1263
<222> 1260-1359
<223> trpc b1263 U00096 complement(1316451..1317812)
<313> 7416: 1287-1306
<400> 7416
CGGCTGCGCC GGACTTGATT TTAATTCTGC TGTAGAGTCG CAACCGGGCA  50

TCAAAGACGC ACGTCTTTTG GCCTCGGTTT TCCAGACGCT GCGCGCATAT 100
<210> 7417
<211> 100
<212> DNA
<213> Escherichia coli K-12 MG1655
<220> trpc b1263
<222> 1134-1233
<223> trpc b1263 U00096 complement(1316451..1317812)
<313> 7417: 1188-1207
<400> 7417
TTTAGACAAC GGCCAGGGTG GAAGCGGGCA ACGTTTTGAC TGGTCACTAT 50
TAAATGGTCA ATCGCTTGGC AACGTTCTGC TGGCGGGGGG CTTAGGCGCA 100
<210> 7418
<211> 100
<212> DNA
<213> Escherichia coli K-12 MG1655
<220> trpc b1263
<222> 1100-1199
<223> trpc b1263 U00096 complement(1316451..1317812)
<313> 7418: 1143-1162
<400> 7418
CCGCCCGCGA GTTTCAGCAC GTTGATAAAT ATGTTTTAGA CAACGGCCAG 50
GGTGGAAGCG GGCAACGTTT TGACTGGTCA CTATTAAATG GTCAATCGCT 100
<210> 7419
<211> 100
<212> DNA
<213> Escherichia coli K-12 MG1655
<220> trpd b1264
<222> 1497-1596
<223> trpd b1264 U00096 complement(1317813..1319408)
<313> 7419: 1575-1594
<400> 7419
TGGCCATGAA GATCTGCAAG CCAATGCGCA AACCGTTCTT GAGGTACTGC 50
GCAGTGGTTC CGCTTACGAC AGAGTCACCG CACTGGCGGC ACGAGGGTAA 100
<210> 7420
<211> 100
<212> DNA
<213> Escherichia coli K-12 MG1655
<220> trpd b1264
<222> 1418-1517
<223> trpd b1264 U00096 complement(1317813..1319408)
<313> 7420: 1460-1479
<400> 7420
CACGTTTGTT ACAAGGTAAA GGCGACGCCG CCCATGAAGC AGCCGTCGCT 50
GCGAACGTCG CCATGTTAAT GCGCCTGCAT GGCCATGAAG ATCTGCAAGC 100
<210> 7421
<211> 100
<212> DNA
<213> Escherichia coli K-12 MG1655
<220> trpd b1264
<222> 1378-1477
<223> trpd b1264 U00096 complement(1317813..1319408)
<313> 7421: 1418-1437
<400> 7421
CTGGCAGGCG GAACACCGGA AGAAAACCGT GACATTTTAA CACGTTTGTT 50
ACAAGGTAAA GGCGACGCCG CCCATGAAGC AGCCGTCGCT GCGAACGTCG 100
<210> 7422
<211> 100
<212> DNA
<213> Escherichia coli K-12 MG1655
<220> trpe b1265
<222> 1464-1563
<223> trpe b1265 U00096 complement(1319408..1320970)
<313> 7422: 1528-1547
<400> 7422
TGATTCTGTT CCGCAGTCGG AAGCCGACGA AACCCGTAAC AAAGCCCGCG 50
CTGTACTGCG CGCTATTGCC ACCGCGCATC ATGCACAGGA GACTTTCTGA 100
<210> 7423
<211> 100
<212> DNA
<213> Escherichia coli K-12 MG1655

<220> trpe b1265
<222> 1454-1553
<223> trpe b1265 U00096 complement(1319408..1320970)
<313> 7423: 1492-1511
<400> 7423
GTGTAGTCCT TGATTCTGTT CCGCAGTCGG AAGCCGACGA AACCCGTAAC  50
AAAGCCCGCG CTGTACTGCG CGCTATTGCC ACCGCGCATC ATGCACAGGA  100
<210> 7424
<211> 100
<212> DNA
<213> Escherichia coli K-12 MG1655
<220> trpe b1265
<222> 1404-1503
<223> trpe b1265 U00096 complement(1319408..1320970)
<313> 7424: 1457-1476
<400> 7424
CCGCTCGGCG CTGGTGGAAA ACGGTATCGC CACCGTGCAA GCGGGTGCTG  50
GTGTAGTCCT TGATTCTGTT CCGCAGTCGG AAGCCGACGA AACCCGTAAC  100
<210> 7425
<211> 100
<212> DNA
<213> Escherichia coli K-12 MG1655
<220> gcva gcvA
<222> 819-918
<223> gcva gcvA U00096 complement(2939672..2940589)
<313> 7425: 894-913
<400> 7425
CAGTCAGGCA GAACTGGGTA AAATAGCCGC CTTTCGCCAA TGGATCCTGG  50
CGAAAGCCGC TGCTGAACAA GAAAAATTCC GCTTTCGTTA TGAACAATAA  100
<210> 7426
<211> 100
<212> DNA
<213> Escherichia coli K-12 MG1655
<220> gcva gcvA
<222> 793-892
<223> gcva gcvA U00096 complement(2939672..2940589)
<313> 7426: 832-851
<400> 7426
AATGCTTTTT ATCTGGTTTG TCATGACAGT CAGGCAGAAC TGGGTAAAAT  50
AGCCGCCTTT CGCCAATGGA TCCTGGCGAA AGCCGCTGCT GAACAAGAAA  100
<210> 7427
<211> 100
<212> DNA
<213> Escherichia coli K-12 MG1655
<220> gcva gcvA
<222> 695-794
<223> gcva gcvA U00096 complement(2939672..2940589)
<313> 7427: 724-743
<400> 7427
ACGGGCAGGG AGTGGCGCTG GCAAATAACG TGATGGCGCA ATCTGAAATC  50
GAGGCCGGAC GTCTTGTTTG CCCGTTTAAT GATGTTCTGG TCAGTAAAAA  100
<210> 7428
<211> 100
<212> DNA
<213> Escherichia coli K-12 MG1655
<220> trpl b1266
<222> 1-45
<223> trpl b1266 U00096 complement(1321062..1321106)
<313> 7428: 8-27
<400> 7428
ATGAAAGCAA TTTTCGTACT GAAAGGTTGG TGGCGCACTT CCTGA  50
 100
<210> 7429
<211> 100
<212> DNA
<213> Escherichia coli K-12 MG1655
<220> amtb b0452
<222> 1188-1287
<223> amtb b0452 U00096 472190..473476
<313> 7429: 1260-1279
<400> 7429

CAAATTGGCG GATCTGACGG TTGGTCTGCG TGTACCGGAA GAGCAGGAGC 50
GAGAAGGGCT GGATGTCAAC AGCCACGGCG AGAATGCCTA TAACGCGTAA 100
<210> 7430
<211> 100
<212> DNA
<213> Escherichia coli K-12 MG1655
<220> amtb b0452
<222> 1159-1258
<223> amtb b0452 U00096 472190..473476
<313> 7430: 1222-1241
<400> 7430
TGGTCCGGTG TTGTGGCATT TATCGGCTAC AAATTGGCGG ATCTGACGGT 50
TGGTCTGCGT GTACCGGAAG AGCAGGAGCG AGAAGGGCTG GATGTCAACA 100
<210> 7431
<211> 100
<212> DNA
<213> Escherichia coli K-12 MG1655
<220> amtb b0452
<222> 1080-1179
<223> amtb b0452 U00096 472190..473476
<313> 7431: 1107-1126
<400> 7431
CGGCGTGGGC TTCGCTGAAG GTGTGACGAT GGGCCATCAG TTGCTGGTAC 50
AGCTGGAAAG CATCGCCATT ACGATCGTCT GGTCCGGTGT TGTGGCATTT 100
<210> 7432
<211> 100
<212> DNA
<213> Escherichia coli K-12 MG1655
<220> trpr b4394
<222> 228-327
<223> trpr b4394 U00096 4630329..4630655
<313> 7432: 295-314
<400> 7432
CGCAGGCATC GCGACGATTA CGCGTGGATC TAACAGCCTG AAAGCCGCGC 50
CCGTCGAGCT GCGCCAGTGG CTGGAAGAGG TGTTGCTGAA AAGCGATTGA 100
<210> 7433
<211> 100
<212> DNA
<213> Escherichia coli K-12 MG1655
<220> trpr b4394
<222> 183-282
<223> trpr b4394 U00096 4630329..4630655
<313> 7433: 207-226
<400> 7433
GTTGCGCGGC GAAATGAGCC AGCGTGAGTT AAAAAATGAA CTCGGCGCAG 50
GCATCGCGAC GATTACGCGT GGATCTAACA GCCTGAAAGC CGCGCCCGTC 100
<210> 7434
<211> 100
<212> DNA
<213> Escherichia coli K-12 MG1655
<220> trpr b4394
<222> 98-197
<223> trpr b4394 U00096 4630329..4630655
<313> 7434: 126-145
<400> 7434
ATCTCCATTT ACCGTTGTTA AACCTGATGC TGACGCCAGA TGAGCGCGAA 50
GCGTTGGGGA CTCGCGTGCG TATTGTCGAA GAGCTGTTGC GCGGCGAAAT 100
<210> 7435
<211> 100
<212> DNA
<213> Escherichia coli K-12 MG1655
<220> gcvh b2905
<222> 291-390
<223> gcvh b2905 U00096 complement(3047180..3047569)
<313> 7435: 367-386
<400> 7435
TGCAGGCGGC TGGATCTTTA AAATCAAAGC CAGCGATGAA AGCGAACTGG 50
AATCACTGCT GGATGCGACC GCATACGAAG CATTGTTAGA AGACGAGTAA 100
<210> 7436
<211> 100
<212> DNA

<213> Escherichia coli K-12 MG1655
<220> gcvh b2905
<222> 272-371
<223> gcvh b2905 U00096 complement(3047180..3047569)
<313> 7436: 330-349
<400> 7436
TGGTGAACAG CGAACCGTAT GCAGGCGGCT GGATCTTTAA AATCAAAGCC 50
AGCGATGAAA GCGAACTGGA ATCACTGCTG GATGCGACCG CATACGAAGC 100
<210> 7437
<211> 100
<212> DNA
<213> Escherichia coli K-12 MG1655
<220> gcvh b2905
<222> 231-330
<223> gcvh b2905 U00096 complement(3047180..3047569)
<313> 7437: 281-300
<400> 7437
AATCGTGGCG GTAAACGACG CACTGAGCGA TTCCCCGGAA CTGGTGAACA 50
GCGAACCGTA TGCAGGCGGC TGGATCTTTA AAATCAAAGC CAGCGATGAA 100
<210> 7438
<211> 100
<212> DNA
<213> Escherichia coli K-12 MG1655
<220> trps b3385
<222> 873-972
<223> trps b3385 U00096 complement(3510272..3511276)
<313> 7438: 922-941
<400> 7438
CTATCACCGT TTCCGCAACG ATGAAGCCTT CCTGCAACAG GTGATGAAAG 50
ATGGCGCGGA AAAAGCCAGC GCGCACGCTT CCCGTACGCT AAAAGCGGTG 100
<210> 7439
<211> 100
<212> DNA
<213> Escherichia coli K-12 MG1655
<220> trps b3385
<222> 824-923
<223> trps b3385 U00096 complement(3510272..3511276)
<313> 7439: 876-895
<400> 7439
GTGAAGTGGC TGATGCCGTT TCCGGTATGC TGACTGAATT GCAGGAACGC 50
TATCACCGTT TCCGCAACGA TGAAGCCTTC CTGCAACAGG TGATGAAAGA 100
<210> 7440
<211> 100
<212> DNA
<213> Escherichia coli K-12 MG1655
<220> trps b3385
<222> 711-810
<223> trps b3385 U00096 complement(3510272..3511276)
<313> 7440: 768-787
<400> 7440
GCAGAACAAA GCGGGCGTTT CCAACCTGTT GGATATCCTT TCAGCGGTAA 50
CGGGCCAGAG CATCCCAGAA CTGGAAAAAC AGTTCGAAGG CAAGATGTAT 100
<210> 7441
<211> 100
<212> DNA
<213> Escherichia coli K-12 MG1655
<220> b3122 b3123
<222> 66-165
<223> b3122 b3123 U00096 3267304..3267468
<313> 7441: 130-149
<400> 7441
TGCGCTACGC TTATCAGGCC TACGCCATCT CTGCAATATA TTGAATTTGC 50
GTGCTTTTGT AGGCAGGATA AGGCGTTCAC GCCGCATCCG GCATGAATAA 100
<210> 7442
<211> 100
<212> DNA
<213> Escherichia coli K-12 MG1655
<220> b3122 b3123
<222> 66-165
<223> b3122 b3123 U00096 3267304..3267468
<313> 7442: 124-143

<400> 7442
TGCGCTACGC TTATCAGGCC TACGCCATCT CTGCAATATA TTGAATTTGC 50
GTGCTTTTGT AGGCAGGATA AGGCGTTCAC GCCGCATCCG GCATGAATAA 100
<210> 7443
<211> 100
<212> DNA
<213> Escherichia coli K-12 MG1655
<220> b3122 b3123
<222> 61-160
<223> b3122 b3123 U00096 3267304..3267468
<313> 7443: 112-131
<400> 7443
CCTGATGCGC TACGCTTATC AGGCCTACGC CATCTCTGCA ATATATTGAA 50
TTTGCGTGCT TTTGTAGGCA GGATAAGGCG TTCACGCCGC ATCCGGCATG 100
<210> 7444
<211> 100
<212> DNA
<213> Escherichia coli K-12 MG1655
<220> pgsa b1913
<222> 397-496
<223> pgsa b1913 U00096 complement(1990292..1990840)
<313> 7444: 445-464
<400> 7444
GCCCAGATGG TGGCGTTGGC ATGGCTGCTG TGGCGTCCGA ACATTTGGGT 50
TGAGTACGCC GGTATTGCAC TTTTCTTTGT GGCTGCGGTA CTGACTCTGT 100
<210> 7445
<211> 100
<212> DNA
<213> Escherichia coli K-12 MG1655
<220> pgsa b1913
<222> 371-470
<223> pgsa b1913 U00096 complement(1990292..1990840)
<313> 7445: 392-411
<400> 7445
CCTGGATTGG GAAAGTGAAA ACCACTGCCC AGATGGTGGC GTTGGCATGG 50
CTGCTGTGGC GTCCGAACAT TTGGGTTGAG TACGCCGGTA TTGCACTTTT 100
<210> 7446
<211> 100
<212> DNA
<213> Escherichia coli K-12 MG1655
<220> pgsa b1913
<222> 298-397
<223> pgsa b1913 U00096 complement(1990292..1990840)
<313> 7446: 343-362
<400> 7446
GCCCGTGAAA TTATTATTTC TGCGCTACGC GAATGGATGG CGGAGTTGGG 50
TAAACGCAGT AGCGTGGCCG TCTCCTGGAT TGGGAAAGTG AAAACCACTG 100
<210> 7447
<211> 100
<212> DNA
<213> Escherichia coli K-12 MG1655
<220> gcvp b2904
<222> 2775-2874
<223> gcvp b2904 U00096 complement(3044188..3047061)
<313> 7447: 2850-2869
<400> 7447
TGTGGCAGAC AAATACTGGC CGACAGTGAA ACGTCTGGAT GATGTTTACG 50
GCGACCGTAA CCTGTTCTGC TCCTGCGTAC CGATTAGCGA ATACCAGTAA 100
<210> 7448
<211> 100
<212> DNA
<213> Escherichia coli K-12 MG1655
<220> gcvp b2904
<222> 2753-2852
<223> gcvp b2904 U00096 complement(3044188..3047061)
<313> 7448: 2778-2797
<400> 7448
AAGTTGCGGT ATTCCCGGCA GGTGTGGCAG ACAAATACTG GCCGACAGTG 50
AAACGTCTGG ATGATGTTTA CGGCGACCGT AACCTGTTCT GCTCCTGCGT 100
<210> 7449
<211> 100

<212> DNA
<213> Escherichia coli K-12 MG1655
<220> gcvp b2904
<222> 2691-2790
<223> gcvp b2904 U00096 complement(3044188..3047061)
<313> 7449: 2751-2770
<400> 7449
GGTGAACGCG CCGCACATTC AGAGCGAACT GGTCGCCGAG TGGGCGCATC 50
CGTACAGCCG TGAAGTTGCG GTATTCCCGG CAGGTGTGGC AGACAAATAC 100
<210> 7450
<211> 100
<212> DNA
<213> Escherichia coli K-12 MG1655
<220> gcvr b2480
<222> 512-611
<223> gcvr b2480 U00096 2597860..2598498
<313> 7450: 564-583
<400> 7450
GCCCCGCATC TGCGGACGCA GCAAATATTG AGCAAGCGTT CAAAGCCCTA 50
TGTACAGAAC TCAATGCACA AGGCAGTATT AACGTCGTCA ATTATTCCCA 100
<210> 7451
<211> 100
<212> DNA
<213> Escherichia coli K-12 MG1655
<220> gcvr b2480
<222> 452-551
<223> gcvr b2480 U00096 2597860..2598498
<313> 7451: 501-520
<400> 7451
GCACGCAACC TGCTGAAAAT GAACGGGCTG CGCAGTTGCA TATTCAGATA 50
ACCGCCCACA GCCCCGCATC TGCGGACGCA GCAAATATTG AGCAAGCGTT 100
<210> 7452
<211> 100
<212> DNA
<213> Escherichia coli K-12 MG1655
<220> gcvr b2480
<222> 422-521
<223> gcvr b2480 U00096 2597860..2598498
<313> 7452: 457-476
<400> 7452
ATCATATGAA CATTGCGGAG CTGGTGTCGC GCACGCAACC TGCTGAAAAT 50
GAACGGGCTG CGCAGTTGCA TATTCAGATA ACCGCCCACA GCCCCGCATC 100
<210> 7453
<211> 100
<212> DNA
<213> Escherichia coli K-12 MG1655
<220> grxa b0850
<222> 159-258
<223> grxa b0850 U00096 complement(889719..889976)
<313> 7453: 237-256
<400> 7453
TAAACCCGTA GAAACCGTGC CGCAGATTTT TGTCGATCAG CAACATATCG 50
GCGGCTATAC CGATTTTGCT GCATGGGTGA AAGAAAATCT GGACGCCTGA 100
<210> 7454
<211> 100
<212> DNA
<213> Escherichia coli K-12 MG1655
<220> grxa b0850
<222> 122-221
<223> grxa b0850 U00096 complement(889719..889976)
<313> 7454: 177-196
<400> 7454
AAGGGATCAC TAAAGAAGAT CTACAACAAA AGGCAGGTAA ACCCGTAGAA 50
ACCGTGCCGC AGATTTTTGT CGATCAGCAA CATATCGGCG GCTATACCGA 100
<210> 7455
<211> 100
<212> DNA
<213> Escherichia coli K-12 MG1655
<220> grxa b0850
<222> 4-103
<223> grxa b0850 U00096 complement(889719..889976)

<313> 7455: 68-87
<400> 7455
CAAACCGTTA TTTTTGGTCG TTCGGGTTGC CCTTACTGTG TGCGTGCAAA 50
AGATCTGGCT GAGAAATTGA GCAATGAACG CGATGATTTT CAGTATCAGT 100
<210> 7456
<211> 100
<212> DNA
<213> Escherichia coli K-12 MG1655
<220> grxb b1065
<222> 549-648
<223> grxb b1065 U00096 complement(1122630..1123277)
<313> 7456: 583-602
<400> 7456
GCTGGTAGCC GGAATTAACT GGCCAAGCCG CGTTGCTGAT TACCGCGATA 50
ATATGGCGAA ACAGACACAA ATCAATTTGT TATCATCAAT GGCGATTTAA 100
<210> 7457
<211> 100
<212> DNA
<213> Escherichia coli K-12 MG1655
<220> grxb b1065
<222> 499-598
<223> grxb b1065 U00096 complement(1122630..1123277)
<313> 7457: 516-535
<400> 7457
GAACTTTCGG AAGATGATAT TCAGCTATTC CCGCTACTGC GTAATCTGAC 50
GCTGGTAGCC GGAATTAACT GGCCAAGCCG CGTTGCTGAT TACCGCGATA 100
<210> 7458
<211> 100
<212> DNA
<213> Escherichia coli K-12 MG1655
<220> grxb b1065
<222> 445-544
<223> grxb b1065 U00096 complement(1122630..1123277)
<313> 7458: 476-495
<400> 7458
GATGATTTAC GTGCGCTGGA CAAACTGATC GTCAAACCGA ACGCCGTGAA 50
TGGCGAACTT TCGGAAGATG ATATTCAGCT ATTCCCGCTA CTGCGTAATC 100
<210> 7459
<211> 100
<212> DNA
<213> Escherichia coli K-12 MG1655
<220> gcvt b2906
<222> 996-1095
<223> gcvt b2906 U00096 complement(3047593..3048687)
<313> 7459: 1034-1053
<400> 7459
AGGTATTGGC GAAACGGCGA TTGTGCAAAT TCGCAACCGT GAAATGCCGG 50
TTAAAGTGAC AAAACCTGTT TTTGTGCGTA ACGGCAAAGC CGTCGCGTGA 100
<210> 7460
<211> 100
<212> DNA
<213> Escherichia coli K-12 MG1655
<220> gcvt b2906
<222> 900-999
<223> gcvt b2906 U00096 complement(3047593..3048687)
<313> 7460: 956-975
<400> 7460
TACCGATGCG CAGGGCAACC AGCATGAAGG CATTATCACC AGCGGTACTT 50
TCTCCCCGAC GCTGGGTTAC AGCATTGCGC TGGCGCGCGT GCCGGAAGGT 100
<210> 7461
<211> 100
<212> DNA
<213> Escherichia coli K-12 MG1655
<220> gcvt b2906
<222> 778-877
<223> gcvt b2906 U00096 complement(3047593..3048687)
<313> 7461: 808-827
<400> 7461
GCAGATCGTG ACTTTATCGG TCGTGAAGCC CTGGAAGTGC AGCGTGAGCA 50
TGGTACAGAA AAACTGGTTG GTCTGGTGAT GACCGAAAAA GGCGTGCTGC 100
<210> 7462

<211> 100
<212> DNA
<213> Escherichia coli K-12 MG1655
<220> grxc b3611
<222> 137-236
<223> grxc b3611 U00096 complement(3781818..3782069)
<313> 7462: 158-177
<400> 7462
AACGCAGCGG TCGCACCACG GTTCCCCAGA TTTTTATTGA CGCACAGCAC 50
ATTGGCGGCT GTGATGACTT GTATGCATTG GATGCACGTG GTGGACTGGA 100
<210> 7463
<211> 100
<212> DNA
<213> Escherichia coli K-12 MG1655
<220> grxc b3611
<222> 60-159
<223> grxc b3611 U00096 complement(3781818..3782069)
<313> 7463: 124-143
<400> 7463
ACTGCTGAGC AGCAAGGGCG TGAGTTTCCA GGAGCTGCCG ATCGATGGCA 50
ACGCCGCCAA GCGTGAAGAG ATGATCAAAC GCAGCGGTCG CACCACGGTT 100
<210> 7464
<211> 100
<212> DNA
<213> Escherichia coli K-12 MG1655
<220> grxc b3611
<222> 22-121
<223> grxc b3611 U00096 complement(3781818..3782069)
<313> 7464: 66-85
<400> 7464
ACCAAAGAAA CCTGCCCGTA TTGCCATCGT GCAAAAGCAC TGCTGAGCAG 50
CAAGGGCGTG AGTTTCCAGG AGCTGCCGAT CGATGGCAAC GCCGCCAAGC 100
<210> 7465
<211> 100
<212> DNA
<213> Escherichia coli K-12 MG1655
<220> b0298 b0299
<222> 123-222
<223> b0298 b0299 U00096 314506..314814
<313> 7465: 145-164
<400> 7465
ACTCAGCCTG TATGAATCAC AACTCTACAA CTGGCGCAGT AAACAGCAAA 50
ATCAGCAGAC GTCTTCTGAA CGTGAACTGG AGATGTCTAC CGAGATTGCA 100
<210> 7466
<211> 100
<212> DNA
<213> Escherichia coli K-12 MG1655
<220> b0298 b0299
<222> 63-162
<223> b0298 b0299 U00096 314506..314814
<313> 7466: 117-136
<400> 7466
ATTTCGCAGT GAAGCCCTGA AGCTTGCTGA ACGCATCGGT GTTACTGCCG 50
CAGCCCGTGA ACTCAGCCTG TATGAATCAC AACTCTACAA CTGGCGCAGT 100
<210> 7467
<211> 100
<212> DNA
<213> Escherichia coli K-12 MG1655
<220> b0298 b0299
<222> 24-123
<223> b0298 b0299 U00096 314506..314814
<313> 7467: 72-91
<400> 7467
ATCAACCAGT AAAAAACCCC GTAAACAGCA TTCGCCTGAA TTTCGCAGTG 50
AAGCCCTGAA GCTTGCTGAA CGCATCGGTG TTACTGCCGC AGCCCGTGAA 100
<210> 7468
<211> 100
<212> DNA
<213> Escherichia coli K-12 MG1655
<220> trs5_1 b0260
<222> 841-940

<223> trs5_1 b0260 U00096 complement(273325..274341)
<313> 7468: 875-894
<400> 7468
GCCAGCATCC GGGCCAGGGT GGAGCACCCA TTTCGCATCA TCAAGCGACA 50
GTTCGGCTTC GTGAAAGCCA GATACAAGGG GTTGCTGAAA AACGATAACC 100
<210> 7469
<211> 100
<212> DNA
<213> Escherichia coli K-12 MG1655
<220> trs5_1 b0260
<222> 802-901
<223> trs5_1 b0260 U00096 complement(273325..274341)
<313> 7469: 852-871
<400> 7469
CGCAAGAACA AAACGGCCAT CAACATCGAA TACATGAAAG CCAGCATCCG 50
GGCCAGGGTG GAGCACCCAT TTCGCATCAT CAAGCGACAG TTCGGCTTCG 100
<210> 7470
<211> 100
<212> DNA
<213> Escherichia coli K-12 MG1655
<220> trs5_1 b0260
<222> 813-912
<223> trs5_1 b0260 U00096 complement(273325..274341)
<313> 7470: 847-866
<400> 7470
AACGGCCATC AACATCGAAT ACATGAAAGC CAGCATCCGG GCCAGGGTGG 50
AGCACCCATT TCGCATCATC AAGCGACAGT TCGGCTTCGT GAAAGCCAGA 100
<210> 7471
<211> 100
<212> DNA
<213> Escherichia coli K-12 MG1655
<220> trs5_2 b0553
<222> 833-932
<223> trs5_2 b0553 U00096 complement(573960..574976)
<313> 7471: 868-887
<400> 7471
ACATGAAAGC CAGCATCCGG GCCAGGGTGG AGCACCCATT TCGCATCATC 50
AAGCGACAGT TCGGCTTCGT GAAAGCCAGA TACAAGGGGT TGCTGAAAAA 100
<210> 7472
<211> 100
<212> DNA
<213> Escherichia coli K-12 MG1655
<220> trs5_2 b0553
<222> 824-923
<223> trs5_2 b0553 U00096 complement(573960..574976)
<313> 7472: 862-881
<400> 7472
ACATCGAATA CATGAAAGCC AGCATCCGGG CCAGGGTGGA GCACCCATTT 50
CGCATCATCA AGCGACAGTT CGGCTTCGTG AAAGCCAGAT ACAAGGGGTT 100
<210> 7473
<211> 100
<212> DNA
<213> Escherichia coli K-12 MG1655
<220> trs5_2 b0553
<222> 787-886
<223> trs5_2 b0553 U00096 complement(573960..574976)
<313> 7473: 834-853
<400> 7473
TTGAAACAGC ATCCACGCAA GAACAAAACG GCCATCAACA TCGAATACAT 50
GAAAGCCAGC ATCCGGGCCA GGGTGGAGCA CCCATTTCGC ATCATCAAGC 100
<210> 7474
<211> 100
<212> DNA
<213> Escherichia coli K-12 MG1655
<220> trs5_3 b0657
<222> 816-915
<223> trs5_3 b0657 U00096 complement(687220..688236)
<313> 7474: 870-889
<400> 7474
GGCCATCAAC ATCGAATACA TGAAAGCCAG CATCCGGGCC AGGGTGGAGC 50
ACCCATTTCG CATCATCAAG CGACAGTTCG GCTTCGTGAA AGCCAGATAC 100

<210> 7475
<211> 100
<212> DNA
<213> Escherichia coli K-12 MG1655
<220> trs5_3 b0657
<222> 808-907
<223> trs5_3 b0657 U00096 complement(687220..688236)
<313> 7475: 853-872
<400> 7475
AACAAAACGG CCATCAACAT CGAATACATG AAAGCCAGCA TCCGGGCCAG  50
GGTGGAGCAC CCATTTCGCA TCATCAAGCG ACAGTTCGGC TTCGTGAAAG  100
<210> 7476
<211> 100
<212> DNA
<213> Escherichia coli K-12 MG1655
<220> trs5_3 b0657
<222> 792-891
<223> trs5_3 b0657 U00096 complement(687220..688236)
<313> 7476: 832-851
<400> 7476
ACAGCATCCA CGCAAGAACA AAACGGCCAT CAACATCGAA TACATGAAAG  50
CCAGCATCCG GGCCAGGGTG GAGCACCCAT TTCGCATCAT CAAGCGACAG  100
<210> 7477
<211> 100
<212> DNA
<213> Escherichia coli K-12 MG1655
<220> trs5_4 b1332
<222> 840-939
<223> trs5_4 b1332 U00096 1394100..1395116
<313> 7477: 875-894
<400> 7477
AGCCAGCATC CGGGCCAGGG TGGAGCACCC ATTTCGCATC ATCAAGCGAC  50
AGTTCGGCTT CGTGAAAGCC AGATACAAGG GGTTGCTGAA AAACGATAAC  100
<210> 7478
<211> 100
<212> DNA
<213> Escherichia coli K-12 MG1655
<220> trs5_4 b1332
<222> 805-904
<223> trs5_4 b1332 U00096 1394100..1395116
<313> 7478: 850-869
<400> 7478
AAGAACAAAA CGGCCATCAA CATCGAATAC ATGAAAGCCA GCATCCGGGC  50
CAGGGTGGAG CACCCATTTC GCATCATCAA GCGACAGTTC GGCTTCGTGA  100
<210> 7479
<211> 100
<212> DNA
<213> Escherichia coli K-12 MG1655
<220> trs5_4 b1332
<222> 783-882
<223> trs5_4 b1332 U00096 1394100..1395116
<313> 7479: 833-852
<400> 7479
AACCTTGAAA CAGCATCCAC GCAAGAACAA AACGGCCATC AACATCGAAT  50
ACATGAAAGC CAGCATCCGG GCCAGGGTGG AGCACCCATT TCGCATCATC  100
<210> 7480
<211> 100
<212> DNA
<213> Escherichia coli K-12 MG1655
<220> trs5_5 trs5_5
<222> 882-981
<223> trs5_5 trs5_5 U00096 complement(1425770..1426750)
<313> 7480: 939-958
<400> 7480
GGGGCTGCTG AAAAACGATA ACCAACTGGC GATGTTATTC ACCCTGGCCA  50
ACCTGTTTCG GGTGGACCAA ATGATACGTC AGTGGGAGAG ATCTCAGTAA  100
<210> 7481
<211> 100
<212> DNA
<213> Escherichia coli K-12 MG1655
<220> trs5_5 trs5_5

<222> 865-964
<223> trs5_5 trs5_5 U00096 complement(1425770..1426750)
<313> 7481: 896-915
<400> 7481
GTGAAAGCCA GATACAAGGG GCTGCTGAAA AACGATAACC AACTGGCGAT  50
GTTATTCACC CTGGCCAACC TGTTTCGGGT GGACCAAATG ATACGTCAGT  100
<210> 7482
<211> 100
<212> DNA
<213> Escherichia coli K-12 MG1655
<220> trs5_5 trs5_5
<222> 815-914
<223> trs5_5 trs5_5 U00096 complement(1425770..1426750)
<313> 7482: 831-850
<400> 7482
GTGCCAGGGT GGAGCACCCG TTTCGCATCA TCAAGCGGCA GTTCGGCTTC  50
GTGAAAGCCA GATACAAGGG GCTGCTGAAA AACGATAACC AACTGGCGAT  100
<210> 7483
<211> 100
<212> DNA
<213> Escherichia coli K-12 MG1655
<220> trs5_6 trs5_6
<222> 820-919
<223> trs5_6 trs5_6 U00096 complement(2064327..2065343)
<313> 7483: 872-891
<400> 7483
ATCAACATCG AATACATGAA AGCCAGCATC CGGGCCAAGG TGGAGCACCC  50
ATTTCGCATC ATCAAGCGAC AGTTCGGCTT CGTGAAAGCC AGATACAAGG  100
<210> 7484
<211> 100
<212> DNA
<213> Escherichia coli K-12 MG1655
<220> trs5_6 trs5_6
<222> 835-934
<223> trs5_6 trs5_6 U00096 complement(2064327..2065343)
<313> 7484: 849-868
<400> 7484
ATGAAAGCCA GCATCCGGGC CAAGGTGGAG CACCCATTTC GCATCATCAA  50
GCGACAGTTC GGCTTCGTGA AAGCCAGATA CAAGGGGTTG CTGAAAAACG  100
<210> 7485
<211> 100
<212> DNA
<213> Escherichia coli K-12 MG1655
<220> trs5_6 trs5_6
<222> 791-890
<223> trs5_6 trs5_6 U00096 complement(2064327..2065343)
<313> 7485: 838-857
<400> 7485
AACAGCATCC ACGCAAGAAC AAAACGGCCA TCAACATCGA ATACATGAAA  50
GCCAGCATCC GGGCCAAGGT GGAGCACCCA TTTCGCATCA TCAAGCGACA  100
<210> 7486
<211> 100
<212> DNA
<213> Escherichia coli K-12 MG1655
<220> oraa b2699
<222> 402-501
<223> oraa b2699 U00096 complement(2820162..2820662)
<313> 7486: 454-473
<400> 7486
AACTGTCTTT TCAGAAAAAG TTAAGATCCA GCGTTTTCTG CTCTATCGTG  50
GCTATCTGAT GGAAGATATC CAGGAGATTT GGCGAAATTT TGCCGACTGA  100
<210> 7487
<211> 100
<212> DNA
<213> Escherichia coli K-12 MG1655
<220> oraa b2699
<222> 290-389
<223> oraa b2699 U00096 complement(2820162..2820662)
<313> 7487: 332-351
<400> 7487
ATCAGAAAGG TATTTCCCGC GAAGCGACAG AAAAAGCGAT GCGTGAATGT  50

GACATCGACT GGTGCGCACT GGCGCGCGAT CAGGCGACGC GAAAATATGG 100
<210> 7488
<211> 100
<212> DNA
<213> Escherichia coli K-12 MG1655
<220> oraa b2699
<222> 244-343
<223> oraa b2699 U00096 complement(2820162..2820662)
<313> 7488: 293-312
<400> 7488
CGTAGCCGCA AAGGTTATGG ACCTGCGCGT ATTCGCCAGG AACTGAATCA 50
GAAAGGTATT TCCCGCGAAG CGACAGAAAA AGCGATGCGT GAATGTGACA 100
<210> 7489
<211> 100
<212> DNA
<213> Escherichia coli K-12 MG1655
<220> trs5_7 b2031
<222> 817-916
<223> trs5_7 b2031 U00096 complement(2099917..2100933)
<313> 7489: 872-891
<400> 7489
GCCATCAACA TCGAATACAT GAAAGCCAGC ATCCGGGCCA GGGTGGAGCA 50
CCCATTTCGC ATCATCAAGC GACAGTTCGG CTTCGTGAAA GCCAGATACA 100
<210> 7490
<211> 100
<212> DNA
<213> Escherichia coli K-12 MG1655
<220> trs5_7 b2031
<222> 809-908
<223> trs5_7 b2031 U00096 complement(2099917..2100933)
<313> 7490: 852-871
<400> 7490
ACAAAACGGC CATCAACATC GAATACATGA AAGCCAGCAT CCGGGCCAGG 50
GTGGAGCACC CATTTCGCAT CATCAAGCGA CAGTTCGGCT TCGTGAAAGC 100
<210> 7491
<211> 100
<212> DNA
<213> Escherichia coli K-12 MG1655
<220> trs5_7 b2031
<222> 791-890
<223> trs5_7 b2031 U00096 complement(2099917..2100933)
<313> 7491: 848-867
<400> 7491
AACAGCATCC ACGCAAGAAC AAAACGGCCA TCAACATCGA ATACATGAAA 50
GCCAGCATCC GGGCCAGGGT GGAGCACCCA TTTCGCATCA TCAAGCGACA 100
<210> 7492
<211> 100
<212> DNA
<213> Escherichia coli K-12 MG1655
<220> trs5_8 b2193
<222> 816-915
<223> trs5_8 b2193 U00096 complement(2287085..2288101)
<313> 7492: 871-890
<400> 7492
GGCCATCAAC ATCGAATACA TGAAAGCCAG CATCCGGGCC AGGGTGGAGC 50
ACCCATTTCG CATCATCAAG CGACAGTTCG GCTTCGTGAA AGCCAGATAC 100
<210> 7493
<211> 100
<212> DNA
<213> Escherichia coli K-12 MG1655
<220> trs5_8 b2193
<222> 820-919
<223> trs5_8 b2193 U00096 complement(2287085..2288101)
<313> 7493: 851-870
<400> 7493
ATCAACATCG AATACATGAA AGCCAGCATC CGGGCCAGGG TGGAGCACCC 50
ATTTCGCATC ATCAAGCGAC AGTTCGGCTT CGTGAAAGCC AGATACAAGG 100
<210> 7494
<211> 100
<212> DNA
<213> Escherichia coli K-12 MG1655

<220> trs5_8 b2193
<222> 815-914
<223> trs5_8 b2193 U00096 complement(2287085..2288101)
<313> 7494: 838-857
<400> 7494
CGGCCATCAA CATCGAATAC ATGAAAGCCA GCATCCGGGC CAGGGTGGAG 50
CACCCATTTC GCATCATCAA GCGACAGTTC GGCTTCGTGA AAGCCAGATA 100
<210> 7495
<211> 100
<212> DNA
<213> Escherichia coli K-12 MG1655
<220> trs5_9 b2983
<222> 561-660
<223> trs5_9 b2983 U00096 3128193..3129209
<313> 7495: 589-608
<400> 7495
TGGCATGAAG GCCCACATTG GTGTCGATGC CAAGAGCGGC CTGACCCACA 50
GCCTAGTCAC CACCGCGGCC AACGAGCATG ACCTCAATCA GCTGGGTAAT 100
<210> 7496
<211> 100
<212> DNA
<213> Escherichia coli K-12 MG1655
<220> sfma b0531
<222> 477-576
<223> sfma b0531 U00096 557402..557977
<313> 7496: 538-557
<400> 7496
CAACGTTCTT CATTTTTCTG CACGTTATAA AGGCACCGGT ACAAGTGCAT 50
CAGCAGGGCA AGCAAATGCT GACGCGACTT TTATTATGAG ATATGAATAA 100
<210> 7497
<211> 100
<212> DNA
<213> Escherichia coli K-12 MG1655
<220> sfma b0531
<222> 457-556
<223> sfma b0531 U00096 557402..557977
<313> 7497: 488-507
<400> 7497
CAGAACTTGA TCCCCGGGAC CAACGTTCTT CATTTTTCTG CACGTTATAA 50
AGGCACCGGT ACAAGTGCAT CAGCAGGGCA AGCAAATGCT GACGCGACTT 100
<210> 7498
<211> 100
<212> DNA
<213> Escherichia coli K-12 MG1655
<220> sfma b0531
<222> 382-481
<223> sfma b0531 U00096 557402..557977
<313> 7498: 436-455
<400> 7498
ACGGGTGTCG GTATTGAAAT ACTTGATAAT ACATCCGCAA TTCTCAAACC 50
TGATGGGAAT AGCTTCTCAA CCAACCAGAA CTTGATCCCC GGGACCAACG 100
<210> 7499
<211> 100
<212> DNA
<213> Escherichia coli K-12 MG1655
<220> sfmc b0532
<222> 512-611
<223> sfmc b0532 U00096 558197..558889
<313> 7499: 541-560
<400> 7499
CACCTTTTTA TGTCACCCTG GTTAACTTAC AAGTGGGCAG CCAAAAGTTG 50
GGGAATGCTA TGGCTGCACC CAGAGTTAAT TCACAAATTC CCTTACCCTC 100
<210> 7500
<211> 100
<212> DNA
<213> Escherichia coli K-12 MG1655
<220> sfmc b0532
<222> 395-494
<223> sfmc b0532 U00096 558197..558889
<313> 7500: 449-468
<400> 7500

TTTTATCGCG CATGAAATTA TTTCTCCGTC CAATTCAATT ACAAGAATTA 50
CCCGCAGAAG CGCCGGACAC ACTCAAGTTT TCGCGATCCG GTAACTATAT 100
<210> 7501
<211> 100
<212> DNA
<213> Escherichia coli K-12 MG1655
<220> sfmc b0532
<222> 360-459
<223> sfmc b0532 U00096 558197..558889
<313> 7501: 397-416
<400> 7501
TGCATTGAAC GGCAGGAATG TTTTGCAACT GGCGATTTTA TCGCGCATGA 50
AATTATTTCT CCGTCCAATT CAATTACAAG AATTACCCGC AGAAGCGCCG 100
<210> 7502
<211> 100
<212> DNA
<213> Escherichia coli K-12 MG1655
<220> sfmd b0533
<222> 2505-2604
<223> sfmd b0533 U00096 558920..561523
<313> 7502: 2581-2600
<400> 7502
CGCGAACTCA CGCTGCATTG CGCACTATGT ATTGCCGAAG CAAAGCTTAC 50
AGCAAGCCGT CACTGTTATT TCGGCAGTTT GCACACATCC TGGCTCATAA 100
<210> 7503
<211> 100
<212> DNA
<213> Escherichia coli K-12 MG1655
<220> sfmd b0533
<222> 2478-2577
<223> sfmd b0533 U00096 558920..561523
<313> 7503: 2531-2550
<400> 7503
TGAATTACTG GTTCAGTGGG GAGACGGCGC GAACTCACGC TGCATTGCGC 50
ACTATGTATT GCCGAAGCAA AGCTTACAGC AAGCCGTCAC TGTTATTTCG 100
<210> 7504
<211> 100
<212> DNA
<213> Escherichia coli K-12 MG1655
<220> sfmd b0533
<222> 2466-2565
<223> sfmd b0533 U00096 558920..561523
<313> 7504: 2486-2505
<400> 7504
GCCATTGTCT GGTGAATTAC TGGTTCAGTG GGGAGACGGC GCGAACTCAC 50
GCTGCATTGC GCACTATGTA TTGCCGAAGC AAAGCTTACA GCAAGCCGTC 100
<210> 7505
<211> 100
<212> DNA
<213> Escherichia coli K-12 MG1655
<220> sfmf b0535
<222> 417-516
<223> sfmf b0535 U00096 562553..563068
<313> 7505: 476-495
<400> 7505
TGTCACACTA AACTTTTTTG CCAATTATCG AGCGTTAGCC AGCGGTGTTC 50
GGCCAGGTGT GGCGAAAGCG GATGCGATAT TTATGATCAA TTATAATTAA 100
<210> 7506
<211> 100
<212> DNA
<213> Escherichia coli K-12 MG1655
<220> sfmf b0535
<222> 398-497
<223> sfmf b0535 U00096 562553..563068
<313> 7506: 426-445
<400> 7506
AAGTAGATGC AAATGGCAAT GTCACACTAA ACTTTTTTGC CAATTATCGA 50
GCGTTAGCCA GCGGTGTTCG GCCAGGTGTG GCGAAAGCGG ATGCGATATT 100
<210> 7507
<211> 100
<212> DNA

<213> Escherichia coli K-12 MG1655
<220> sfmf b0535
<222> 336-435
<223> sfmf b0535 U00096 562553..563068
<313> 7507: 395-414
<400> 7507
CATCGAATTA CGTAATAGCG ATCGCTCCCG GCTCGCACTG GGGGAGGCGA 50
GCCCGACTGA GGAAGTAGAT GCAAATGGCA ATGTCACACT AAACTTTTTT 100
<210> 7508
<211> 100
<212> DNA
<213> Escherichia coli K-12 MG1655
<220> sfmh b0534
<222> 853-952
<223> sfmh b0534 U00096 561565..562542
<313> 7508: 894-913
<400> 7508
TTCCGTCTGG ATGCAGCTGC GGCAGCCAAT GTCACACTTC GCGCCTGGCC 50
TATCAGTATT ACCGGTCAAA AACCGACCGA AGGGCCGTTT AGCGCGCTGG 100
<210> 7509
<211> 100
<212> DNA
<213> Escherichia coli K-12 MG1655
<220> sfmh b0534
<222> 768-867
<223> sfmh b0534 U00096 561565..562542
<313> 7509: 807-826
<400> 7509
GTCGGACAAT CAGGATTTAG GTTTTATTGT CGCCGATCAG AACGATACGC 50
CGATCACGCC TAACGATCTC AATAGCGTTA TTCCTTTCCG TCTGGATGCA 100
<210> 7510
<211> 100
<212> DNA
<213> Escherichia coli K-12 MG1655
<220> sfmh b0534
<222> 667-766
<223> sfmh b0534 U00096 561565..562542
<313> 7510: 732-751
<400> 7510
CCGCAAACCA AGAGCATTGC GGTCAAATGT ACGAATGTTG CTGCGCAGGC 50
TTATTTAACA ATGCGTCTGG AAGCCAGTGC CGTTTCTGGT CAGGCGATGG 100
<210> 7511
<211> 100
<212> DNA
<213> Escherichia coli K-12 MG1655
<220> ldha b1381
<222> 891-990
<223> ldha b1381 U00096 complement(1439878..1440867)
<313> 7511: 950-969
<400> 7511
ATTCCTGACA GCAGAAGCTC TGACCAGTAT TTCTCAGACT ACGCTGCAAA 50
ACTTAAGCAA TCTGGAAAAA GGCGAAACCT GCCCGAACGA ACTGGTTTAA 100
<210> 7512
<211> 100
<212> DNA
<213> Escherichia coli K-12 MG1655
<220> ldha b1381
<222> 853-952
<223> ldha b1381 U00096 complement(1439878..1440867)
<313> 7512: 911-930
<400> 7512
TCTGCCTGCC ACAACGTGCT GTTTACCGGG CACCAGGCAT TCCTGACAGC 50
AGAAGCTCTG ACCAGTATTT CTCAGACTAC GCTGCAAAAC TTAAGCAATC 100
<210> 7513
<211> 100
<212> DNA
<213> Escherichia coli K-12 MG1655
<220> ldha b1381
<222> 845-944
<223> ldha b1381 U00096 complement(1439878..1440867)
<313> 7513: 877-896

<400> 7513
GTCGCCTGTC TGCCTGCCAC AACGTGCTGT TTACCGGGCA CCAGGCATTC 50
CTGACAGCAG AAGCTCTGAC CAGTATTTCT CAGACTACGC TGCAAAACTT 100
<210> 7514
<211> 100
<212> DNA
<213> Escherichia coli K-12 MG1655
<220> ydaa b1334
<222> 852-951
<223> ydaa b1334 U00096 complement(1395696..1396646)
<313> 7514: 921-940
<400> 7514
GGCGGAACAG GTGATTGATC ATCTTCGCTG CGACCTGCTG GTCATTAAAC 50
CTGACCAGTA TCAGACACCC GTTGAACTGG ATGACGAAGA AGACGATTAA 100
<210> 7515
<211> 100
<212> DNA
<213> Escherichia coli K-12 MG1655
<220> ydaa b1334
<222> 819-918
<223> ydaa b1334 U00096 complement(1395696..1396646)
<313> 7515: 869-888
<400> 7515
CACCGGTATT TCAGCAGCAT TCCTCGGCAA CACGGCGGAA CAGGTGATTG 50
ATCATCTTCG CTGCGACCTG CTGGTCATTA AACCTGACCA GTATCAGACA 100
<210> 7516
<211> 100
<212> DNA
<213> Escherichia coli K-12 MG1655
<220> ydaa b1334
<222> 808-907
<223> ydaa b1334 U00096 complement(1395696..1396646)
<313> 7516: 835-854
<400> 7516
ACGGTAGGAC GCACCGGTAT TTCAGCAGCA TTCCTCGGCA ACACGGCGGA 50
ACAGGTGATT GATCATCTTC GCTGCGACCT GCTGGTCATT AAACCTGACC 100
<210> 7517
<211> 100
<212> DNA
<213> Escherichia coli K-12 MG1655
<220> ydac b1348
<222> 92-191
<223> ydac b1348 U00096 complement(1411555..1411764)
<313> 7517: 136-155
<400> 7517
GCGAAAAAAT GCTCTCAGGT AAAAAAGAAG CAGGCGTTTC CAGAGAGCAG 50
AAAGTAAAAC TGATAAATTT TAATTGCGAG AAACTTCAGT CCTCGAGAAT 100
<210> 7518
<211> 100
<212> DNA
<213> Escherichia coli K-12 MG1655
<220> ydac b1348
<222> 101-200
<223> ydac b1348 U00096 complement(1411555..1411764)
<313> 7518: 122-141
<400> 7518
TGCTCTCAGG TAAAAAAGAA GCAGGCGTTT CCAGAGAGCA GAAAGTAAAA 50
CTGATAAATT TTAATTGCGA GAAACTTCAG TCCTCGAGAA TTGCATTGTA 100
<210> 7519
<211> 100
<212> DNA
<213> Escherichia coli K-12 MG1655
<220> ydac b1348
<222> 62-161
<223> ydac b1348 U00096 complement(1411555..1411764)
<313> 7519: 113-132
<400> 7519
ATTCAAAATC TAAACTAACG AAATCAGAAT GCGAAAAAAT GCTCTCAGGT 50
AAAAAAGAAG CAGGCGTTTC CAGAGAGCAG AAAGTAAAAC TGATAAATTT 100
<210> 7520
<211> 100

<212> DNA
<213> Escherichia coli K-12 MG1655
<220> ydad b1353
<222> 135-234
<223> ydad b1353 U00096 complement(1416032..1416265)
<313> 7520: 198-217
<400> 7520
ATATATTCGT AACCTGACCA CAAAAACATT CATTACTGAC CGCATGATTA 50
AAGTCTTCCT CGGTCGTGAT GGTTTACCTG TAAAGGCGGA GTCATGGTGA 100
<210> 7521
<211> 100
<212> DNA
<213> Escherichia coli K-12 MG1655
<220> ydad b1353
<222> 135-234
<223> ydad b1353 U00096 complement(1416032..1416265)
<313> 7521: 194-213
<400> 7521
ATATATTCGT AACCTGACCA CAAAAACATT CATTACTGAC CGCATGATTA 50
AAGTCTTCCT CGGTCGTGAT GGTTTACCTG TAAAGGCGGA GTCATGGTGA 100
<210> 7522
<211> 100
<212> DNA
<213> Escherichia coli K-12 MG1655
<220> ydad b1353
<222> 135-234
<223> ydad b1353 U00096 complement(1416032..1416265)
<313> 7522: 168-187
<400> 7522
ATATATTCGT AACCTGACCA CAAAAACATT CATTACTGAC CGCATGATTA 50
AAGTCTTCCT CGGTCGTGAT GGTTTACCTG TAAAGGCGGA GTCATGGTGA 100
<210> 7523
<211> 100
<212> DNA
<213> Escherichia coli K-12 MG1655
<220> ydah ydaH
<222> 1390-1489
<223> ydah ydaH U00096 complement(1398271..1399803)
<313> 7523: 1436-1455
<400> 7523
AAACCAGACG CGAAACTGGG TACTTACTAT TCGTTAGTCT TGCCCTATCC 50
ACTTATCTTT TTGGTGGTAT GGCTGCTGAT GTTGCTGGCG TGGTATCTTG 100
<210> 7524
<211> 100
<212> DNA
<213> Escherichia coli K-12 MG1655
<220> ydah ydaH
<222> 1333-1432
<223> ydah ydaH U00096 complement(1398271..1399803)
<313> 7524: 1383-1402
<400> 7524
CCTTTAGCGC CGGTATCTCC TTTTGTTCCA CTGTTTCTTG GATTCCTGCA 50
ACGCTACAAA CCAGACGCGA AACTGGGTAC TTACTATTCG TTAGTCTTGC 100
<210> 7525
<211> 100
<212> DNA
<213> Escherichia coli K-12 MG1655
<220> ydah ydaH
<222> 1299-1398
<223> ydah ydaH U00096 complement(1398271..1399803)
<313> 7525: 1342-1361
<400> 7525
AATCCTCTTT CGTATTGCCG ACTCATCCGT ATTGCCTTTA GCGCCGGTAT 50
CTCCTTTTGT TCCACTGTTT CTTGGATTCC TGCAACGCTA CAAACCAGAC 100
<210> 7526
<211> 100
<212> DNA
<213> Escherichia coli K-12 MG1655
<220> ydaj b1339
<222> 1221-1320
<223> ydaj b1339 U00096 complement(1401279..1402604)

<313> 7526: 1252-1271
<400> 7526
AGGTCATCAC AACGAAAAAT TCGATTTTGA CGAGCAGGTT CTCGCTATTG 50
CCGTCGAAAC GCTGGCGCGC ACCGCGCTCA ATTTTCCCTG GACGCGAGGT 100
<210> 7527
<211> 100
<212> DNA
<213> Escherichia coli K-12 MG1655
<220> ydaj b1339
<222> 1128-1227
<223> ydaj b1339 U00096 complement(1401279..1402604)
<313> 7527: 1160-1179
<400> 7527
TGCGGGTTCC GAAGATGCCA CATTAATGAT GGCCCGCGTG CAGCAACATC 50
AAGGGCAAGC CTCCTACGTG GTGTTTGGCA CACAGCTGGC GGCAGGTCAT 100
<210> 7528
<211> 100
<212> DNA
<213> Escherichia coli K-12 MG1655
<220> ydaj b1339
<222> 1064-1163
<223> ydaj b1339 U00096 complement(1401279..1402604)
<313> 7528: 1078-1097
<400> 7528
GGTTGCAAAG TCAGGCGGCT CAGGTCGCGG GGGTCAATCA GGCCATTGAA 50
CGTGTTGAAG CGCCTGCGGG TTCCGAAGAT GCCACATTAA TGATGGCCCG 100
<210> 7529
<211> 100
<212> DNA
<213> Escherichia coli K-12 MG1655
<220> ydak b1340
<222> 810-909
<223> ydak b1340 U00096 1402765..1403673
<313> 7529: 887-906
<400> 7529
GGCCTATTAT TTGATTCAGC GGCGTGATAG TCGCCAGACA CCACTGACCG 50
CGTCATTAAT CACGCAATTC CGGCGAGAAT GCGGCTATCT GCAAAGTTAA 100
<210> 7530
<211> 100
<212> DNA
<213> Escherichia coli K-12 MG1655
<220> ydak b1340
<222> 809-908
<223> ydak b1340 U00096 1402765..1403673
<313> 7530: 835-854
<400> 7530
CGGCCTATTA TTTGATTCAG CGGCGTGATA GTCGCCAGAC ACCACTGACC 50
GCGTCATTAA TCACGCAATT CCGGCGAGAA TGCGGCTATC TGCAAAGTTA 100
<210> 7531
<211> 100
<212> DNA
<213> Escherichia coli K-12 MG1655
<220> ydak b1340
<222> 740-839
<223> ydak b1340 U00096 1402765..1403673
<313> 7531: 787-806
<400> 7531
AAATGGGCTG CGATCCCTTG CACGGACAGG GGCTGGTGAT GTTGCCGGTT 50
AGCGAAATTT TACCGAAAGC GGCCTATTAT TTGATTCAGC GGCGTGATAG 100
<210> 7532
<211> 100
<212> DNA
<213> Escherichia coli K-12 MG1655
<220> b2420 b2421
<222> 636-735
<223> b2420 b2421 U00096 2535769..2536503
<313> 7532: 677-696
<400> 7532
TCAGCTTACC GCAAAAATTC TTCAATGGGG TGACGGTCAG GACTCACCAA 50
TACTTCAAAA AATGATTGAT GACCTTACGG CGTTTCCTCA CGAAAATTAA 100
<210> 7533

<211> 100
<212> DNA
<213> Escherichia coli K-12 MG1655
<220> b2420 b2421
<222> 574-673
<223> b2420 b2421 U00096 2535769..2536503
<313> 7533: 642-661
<400> 7533
AAAGTCATGG GTAAAATCTC CCATAAACTT AAAAAGATGG GACTCAATGC 50
ACTAGCGCGT CATCAGCTTA CCGCAAAAAT TCTTCAATGG GGTGACGGTC 100
<210> 7534
<211> 100
<212> DNA
<213> Escherichia coli K-12 MG1655
<220> b2420 b2421
<222> 527-626
<223> b2420 b2421 U00096 2535769..2536503
<313> 7534: 555-574
<400> 7534
CTATCCAGAG CAATATGATT ACCTACGCAA CTGCATTCGG TCAGATTAAA 50
GTCATGGGTA AAATCTCCCA TAAACTTAAA AAGATGGGAC TCAATGCACT 100
<210> 7535
<211> 100
<212> DNA
<213> Escherichia coli K-12 MG1655
<220> ydal ydaL
<222> 465-564
<223> ydal ydaL U00096 1404003..1404566
<313> 7535: 526-545
<400> 7535
ACCGCATCAT GGCGGCAGCG GGGCGTGTTA CGTCGCACTA CGTAAAACGG 50
CGCAGGCGAA GCAAGAAAAC TGGGAGCGCC ACGCTAAGCG CAGTCGTTGA 100
<210> 7536
<211> 100
<212> DNA
<213> Escherichia coli K-12 MG1655
<220> ydal ydaL
<222> 437-536
<223> ydal ydaL U00096 1404003..1404566
<313> 7536: 489-508
<400> 7536
ATGATGTTCA GGCATATTGC ACCGCGCTAC CGCATCATGG CGGCAGCGGG 50
GCGTGTTACG TCGCACTACG TAAAACGGCG CAGGCGAAGC AAGAAAACTG 100
<210> 7537
<211> 100
<212> DNA
<213> Escherichia coli K-12 MG1655
<220> ydal ydaL
<222> 428-527
<223> ydal ydaL U00096 1404003..1404566
<313> 7537: 460-479
<400> 7537
CCGAATTTGA TGATGTTCAG GCATATTGCA CCGCGCTACC GCATCATGGC 50
GGCAGCGGGG CGTGTTACGT CGCACTACGT AAAACGGCGC AGGCGAAGCA 100
<210> 7538
<211> 100
<212> DNA
<213> Escherichia coli K-12 MG1655
<220> ydao ydaO
<222> 837-936
<223> ydao ydaO U00096 complement(1409037..1409972)
<313> 7538: 882-901
<400> 7538
TGATCGCGAA GAGATCCCAC TACAACCGGC GTGCTGGCAG CCAGAAGAAG 50
ATGAAAATCA GTTGGATGAG TTACGGCTGA ATGTGGTTGA AGTGAAATAA 100
<210> 7539
<211> 100
<212> DNA
<213> Escherichia coli K-12 MG1655
<220> ydao ydaO
<222> 782-881

<223> ydao ydaO U00096 complement(1409037..1409972)
<313> 7539: 804-823
<400> 7539
ATTTCAAAGG CATCACCCAC GGTTCTGAAG TGGTTAACGG GGGTGATCTG 50
GCGTTTGATC GCGAAGAGAT CCCACTACAA CCGGCGTGCT GGCAGCCAGA 100
<210> 7540
<211> 100
<212> DNA
<213> Escherichia coli K-12 MG1655
<220> ydao ydaO
<222> 739-838
<223> ydao ydaO U00096 complement(1409037..1409972)
<313> 7540: 775-794
<400> 7540
CAGAATGTGG TGCCGTCGCA TCTGTGCGAT ACCAACCTGT TCGATTTCAA 50
AGGCATCACC CACGGTTCTG AAGTGGTTAA CGGGGGTGAT CTGGCGTTTG 100
<210> 7541
<211> 100
<212> DNA
<213> Escherichia coli K-12 MG1655
<220> nmpc b0554
<222> 1029-1128
<223> nmpc b0554 U00096 complement(574981..576108)
<313> 7541: 1098-1117
<400> 7541
TTACTTCAAC AAAAATATGT CTACTTTCGT TGATTACAAA ATCAACCTGC 50
TTGACAAAAA TGACTTCACT AAGGAAGGTG CGAACAAGTC CCTGATATGA 100
<210> 7542
<211> 100
<212> DNA
<213> Escherichia coli K-12 MG1655
<220> nmpc b0554
<222> 897-996
<223> nmpc b0554 U00096 complement(574981..576108)
<313> 7542: 946-965
<400> 7542
CGAAGCTGTT GCACAATATC AGTTCGATTT CGGTCTGCGT CCGTCCGTTG 50
CTTACCTGCA ATCTAAAGGT AAGGATCTTG GAGTATGGGG CGATCAGGAC 100
<210> 7543
<211> 100
<212> DNA
<213> Escherichia coli K-12 MG1655
<220> nmpc b0554
<222> 733-832
<223> nmpc b0554 U00096 complement(574981..576108)
<313> 7543: 762-781
<400> 7543
GGGAAAGTTC TTCCTGAAGT ATTTGCTTCC GGTAAAAATG CAGAAGTTTG 50
GGCCGCAGGT CTGAAATATG ACGCTAACAA CATTTACCTG GCCACTACCT 100
<210> 7544
<211> 100
<212> DNA
<213> Escherichia coli K-12 MG1655
<220> ydaq b1347
<222> 141-240
<223> ydaq b1347 U00096 complement(1411261..1411500)
<313> 7544: 185-204
<400> 7544
CCACTTCAAA CGAGTATCTC CTTTAGGTAA GCCAGACAGC AAACGAGGGA 50
TTATCTGGTA CAACTATCCA AAGATAAATC AGTTTATCAA AGACTCATGA 100
<210> 7545
<211> 100
<212> DNA
<213> Escherichia coli K-12 MG1655
<220> ydaq b1347
<222> 78-177
<223> ydaq b1347 U00096 complement(1411261..1411500)
<313> 7545: 114-133
<400> 7545
TCGCACTGGT CTGGGGGCCA GACAAATTGA AGCATACCGC CAGAACTGTT 50
GGGTGGAGGG CTTCCACTTC AAACGAGTAT CTCCTTTAGG TAAGCCAGAC 100

<210> 7546
<211> 100
<212> DNA
<213> Escherichia coli K-12 MG1655
<220> ydaq b1347
<222> 7-106
<223> ydaq b1347 U00096 complement(1411261..1411500)
<313> 7546: 75-94
<400> 7546
CCTGCCAGAG GTGATGCGAT GGCACAAGTA ATCTTTAATG AAGAGTGGAT  50
GGTTGAATAC GGCCTGATGC TTCGCACTGG TCTGGGGGCC AGACAAATTG  100
<210> 7547
<211> 100
<212> DNA
<213> Escherichia coli K-12 MG1655
<220> ydar b1357
<222> 369-468
<223> ydar b1357 U00096 complement(1417789..1418265)
<313> 7547: 392-411
<400> 7547
CCACGATGTT CGCGCCTACA TAGCAGCGAT GGAAATGAAA GTGGGTAAGT  50
ATCTGAAAGC TCTTGAATCT GAACGGAAAA GCCAGAACAT CACAAAAACT  100
<210> 7548
<211> 100
<212> DNA
<213> Escherichia coli K-12 MG1655
<220> ydar b1357
<222> 269-368
<223> ydar b1357 U00096 complement(1417789..1418265)
<313> 7548: 299-318
<400> 7548
TTTTAGATAT AAACAACCTT TATCAAGCAG CCTCTGATGA AATAAGAGCG  50
ATTGTAGCTT TCCTGTTATC TGGAAATGCT ACAGAACCAG ATTGGGTTGA  100
<210> 7549
<211> 100
<212> DNA
<213> Escherichia coli K-12 MG1655
<220> ydar b1357
<222> 187-286
<223> ydar b1357 U00096 complement(1417789..1418265)
<313> 7549: 247-266
<400> 7549
TCTGATGTTG TTGGTCCAGA GCATTGGGGG CTTAACGAAT ACCCCATACC  50
AACCCCCACC AATTCAGATA CAAAAAGTGA ACTTTTAGAT ATAAACAACC  100
<210> 7550
<211> 100
<212> DNA
<213> Escherichia coli K-12 MG1655
<220> ydas b1358
<222> 132-231
<223> ydas b1358 U00096 1418389..1418685
<313> 7550: 157-176
<400> 7550
CGAGAGATGT CCAGCAATTG AACGTGCAAC AAGAGGTGAG GTTCTGTGCG  50
AAGAACTTCG TCCTGATATT GACTGGTCAT ATTTACGACG TTCGGCATGT  100
<210> 7551
<211> 100
<212> DNA
<213> Escherichia coli K-12 MG1655
<220> ydas b1358
<222> 79-178
<223> ydas b1358 U00096 1418389..1418685
<313> 7551: 121-140
<400> 7551
GTATCACCTC CAAGCGTAAA TCAATGGATC AAAGGGGTAC GTCAATTGCC  50
TGCCGAGAGA TGTCCAGCAA TTGAACGTGC AACAAGAGGT GAGGTTCTGT  100
<210> 7552
<211> 100
<212> DNA
<213> Escherichia coli K-12 MG1655
<220> ydas b1358

<222> 13-112
<223> ydas b1358 U00096 1418389..1418685
<313> 7552: 64-83
<400> 7552
AACTATTCAT TCAAGCAAGC TTGTGCTGTT GTCGGTGGGC AATCAGCAAT 50
GGCTAGGCTT TTAGGTGTAT CACCTCCAAG CGTAAATCAA TGGATCAAAG 100
<210> 7553
<211> 100
<212> DNA
<213> Escherichia coli K-12 MG1655
<220> ydat b1359
<222> 324-423
<223> ydat b1359 U00096 1418708..1419130
<313> 7553: 388-407
<400> 7553
TACGGCAATT GATGCGCATG ATGATGCAGT CCAAGCCGTT TACCGGAAAG 50
CGCATTTCAG CGGCGGCGGT TCTTCCGACG ATTCTGTCAT TGTTCATTAA 100
<210> 7554
<211> 100
<212> DNA
<213> Escherichia coli K-12 MG1655
<220> ydat b1359
<222> 295-394
<223> ydat b1359 U00096 1418708..1419130
<313> 7554: 355-374
<400> 7554
GCATTACTGG CGGCGCAGGA AGCGTTAAGT ACGGCAATTG ATGCGCATGA 50
TGATGCAGTC CAAGCCGTTT ACCGGAAAGC GCATTTCAGC GGCGGCGGTT 100
<210> 7555
<211> 100
<212> DNA
<213> Escherichia coli K-12 MG1655
<220> ydat b1359
<222> 275-374
<223> ydat b1359 U00096 1418708..1419130
<313> 7555: 304-323
<400> 7555
TCGATACCCT GGAACGCCGT GCATTACTGG CGGCGCAGGA AGCGTTAAGT 50
ACGGCAATTG ATGCGCATGA TGATGCAGTC CAAGCCGTTT ACCGGAAAGC 100
<210> 7556
<211> 100
<212> DNA
<213> Escherichia coli K-12 MG1655
<220> b2429 b2429
<222> 1267-1366
<223> b2429 b2429 U00096 2544693..2546117
<313> 7556: 1315-1334
<400> 7556
GCCTTTGGGC CGTCTGGTCT GGTAGCGCTG CCGCTGATGA CTTCCGCACA 50
AGGCATCCTT CCGGCCATGG CGGTTTATGC TGGCGGGATT CTGGTGGCAT 100
<210> 7557
<211> 100
<212> DNA
<213> Escherichia coli K-12 MG1655
<220> b2429 b2429
<222> 1196-1295
<223> b2429 b2429 U00096 2544693..2546117
<313> 7557: 1253-1272
<400> 7557
TAGGCGGCGC GGCGGGCGGT TTGTTTATCG GCCTGATAGC CTGGTGGGGT 50
CTGCCGATGG GCTTAAACAG CGCCTTTGGG CCGTCTGGTC TGGTAGCGCT 100
<210> 7558
<211> 100
<212> DNA
<213> Escherichia coli K-12 MG1655
<220> b2429 b2429
<222> 1142-1241
<223> b2429 b2429 U00096 2544693..2546117
<313> 7558: 1177-1196
<400> 7558
CGCTGATTTA CGGTGTCACC CTGCCCCGCA TGAAGCCGTT TGTTACCGCC 50

TGTTTAGGCG GCGCGGCGGG CGGTTTGTTT ATCGGCCTGA TAGCCTGGTG 100
<210> 7559
<211> 100
<212> DNA
<213> Escherichia coli K-12 MG1655
<220> ydau ydaU
<222> 759-858
<223> ydau ydaU U00096 1419143..1420000
<313> 7559: 822-841
<400> 7559
CTTGTTCGCG CCGAAAGGTG TTCGACCTGA GACGTTACTC CGACAGGTTA 50
ACACCGTCTC GTTACCGGAT AGTGCGATCC CGCCAGGCTT CAGGGGGTAA 100
<210> 7560
<211> 100
<212> DNA
<213> Escherichia coli K-12 MG1655
<220> ydau ydaU
<222> 733-832
<223> ydau ydaU U00096 1419143..1420000
<313> 7560: 783-802
<400> 7560
CACAGCATCG CCAATGAATA CCAGGGCTTG TTCGCGCCGA AAGGTGTTCG 50
ACCTGAGACG TTACTCCGAC AGGTTAACAC CGTCTCGTTA CCGGATAGTG 100
<210> 7561
<211> 100
<212> DNA
<213> Escherichia coli K-12 MG1655
<220> ydau ydaU
<222> 690-789
<223> ydau ydaU U00096 1419143..1420000
<313> 7561: 743-762
<400> 7561
AAAATTCCGC CAGCAGGGTT TTTCACCTGA GCAGGTGATT CGACACAGCA 50
TCGCCAATGA ATACCAGGGC TTGTTCGCGC CGAAAGGTGT TCGACCTGAG 100
<210> 7562
<211> 100
<212> DNA
<213> Escherichia coli K-12 MG1655
<220> ydaw ydaW
<222> 513-612
<223> ydaw ydaW U00096 1420725..1421336
<313> 7562: 590-609
<400> 7562
GTTATCTGCA TCGTCGTCTG CCTGTCATGG GGTGTTAATC ATTACCGTGA 50
TAACGCCATC GCCTACAAAG AACAGCGCGA CAAAAATGCC AGAGAACTGA 100
<210> 7563
<211> 100
<212> DNA
<213> Escherichia coli K-12 MG1655
<220> ydaw ydaW
<222> 499-598
<223> ydaw ydaW U00096 1420725..1421336
<313> 7563: 531-550
<400> 7563
GTATCTCCGC TCTGGTTATC TGCATCGTCG TCTGCCTGTC ATGGGGTGTT 50
AATCATTACC GTGATAACGC CATCGCCTAC AAAGAACAGC GCGACAAAAA 100
<210> 7564
<211> 100
<212> DNA
<213> Escherichia coli K-12 MG1655
<220> ydaw ydaW
<222> 432-531
<223> ydaw ydaW U00096 1420725..1421336
<313> 7564: 492-511
<400> 7564
TCAGGGCGGT AAATTTCGTT ACTGCATACC GGGCGATAAT TTACCAGCAG 50
AGCCGAAAGC AGCATCGGTA TCTCCGCTCT GGTTATCTGC ATCGTCGTCT 100
<210> 7565
<211> 100
<212> DNA
<213> Escherichia coli K-12 MG1655

<220> yday ydaY
<222> 261-360
<223> yday ydaY U00096 1423645..1424004
<313> 7565: 321-340
<400> 7565
TAAGCAGAAT CCAGAGTTGT ACTGGCAGAT CCTTGCAGAA GCAGCCAGCA 50
TCGCCCAGGG TAAAGAGCAG CAGAAGCAGG ATACGATAAA AAAGCCATAG 100
<210> 7566
<211> 100
<212> DNA
<213> Escherichia coli K-12 MG1655
<220> yday ydaY
<222> 246-345
<223> yday ydaY U00096 1423645..1424004
<313> 7566: 281-300
<400> 7566
AGGGATCGCG CTGCTTAAGC AGAATCCAGA GTTGTACTGG CAGATCCTTG 50
CAGAAGCAGC CAGCATCGCC CAGGGTAAAG AGCAGCAGAA GCAGGATACG 100
<210> 7567
<211> 100
<212> DNA
<213> Escherichia coli K-12 MG1655
<220> yday ydaY
<222> 189-288
<223> yday ydaY U00096 1423645..1424004
<313> 7567: 233-252
<400> 7567
AGGGGTTGGT GAACTGGTTA ATGGTGTTGA GGTTGCACTG GAATATACGG 50
CAGAACGAGG GATCGCGCTG CTTAAGCAGA ATCCAGAGTT GTACTGGCAG 100
<210> 7568
<211> 100
<212> DNA
<213> Escherichia coli K-12 MG1655
<220> male b4035
<222> 1092-1191
<223> male b4035 U00096 complement(4242808..4243998)
<313> 7568: 1171-1190
<400> 7568
TTTCTGGTAT GCCGTGCGTA CTGCGGTGAT CAACGCCGCC AGCGGTCGTC 50
AGACTGTCGA TGAAGCCCTG AAAGACGCGC AGACTCGTAT CACCAAGTAA 100
<210> 7569
<211> 100
<212> DNA
<213> Escherichia coli K-12 MG1655
<220> male b4035
<222> 1040-1139
<223> male b4035 U00096 complement(4242808..4243998)
<313> 7569: 1078-1097
<400> 7569
TGGAAAACGC CCAGAAAGGT GAAATCATGC CGAACATCCC GCAGATGTCC 50
GCTTTCTGGT ATGCCGTGCG TACTGCGGTG ATCAACGCCG CCAGCGGTCG 100
<210> 7570
<211> 100
<212> DNA
<213> Escherichia coli K-12 MG1655
<220> male b4035
<222> 981-1080
<223> male b4035 U00096 complement(4242808..4243998)
<313> 7570: 1019-1038
<400> 7570
CGTAGCGCTG AAGTCTTACG AGGAAGAGTT GGCGAAAGAT CCACGTATTG 50
CCGCCACCAT GGAAAACGCC CAGAAAGGTG AAATCATGCC GAACATCCCG 100
<210> 7571
<211> 100
<212> DNA
<213> Escherichia coli K-12 MG1655
<220> malf b4034
<222> 1446-1545
<223> malf b4034 U00096 complement(4241110..4242654)
<313> 7571: 1508-1527
<400> 7571

GGACTTCGGT CTGGCGGCAG CAATTGCCAC GCTGATCTTC CTGCTGGTGG 50
GTGCGCTGGC GATAGTGAAC CTGAAAGCCA CGCGAATGAA GTTTGATTAA 100
<210> 7572
<211> 100
<212> DNA
<213> Escherichia coli K-12 MG1655
<220> malf b4034
<222> 1397-1496
<223> malf b4034 U00096 complement(4241110..4242654)
<313> 7572: 1433-1452
<400> 7572
TGCTTGTTAA CTACACCTAC CGCATCGCTT TTGAAGGCGG CGGGGGTCAG 50
GACTTCGGTC TGGCGGCAGC AATTGCCACG CTGATCTTCC TGCTGGTGGG 100
<210> 7573
<211> 100
<212> DNA
<213> Escherichia coli K-12 MG1655
<220> malf b4034
<222> 1350-1449
<223> malf b4034 U00096 complement(4241110..4242654)
<313> 7573: 1399-1418
<400> 7573
CGGCCCGGAT CGTCTTGGCA CGACCACGCC AGCCGGTTAT ACCGACCTGC 50
TTGTTAACTA CACCTACCGC ATCGCTTTTG AAGGCGGCGG GGGTCAGGAC 100
<210> 7574
<211> 100
<212> DNA
<213> Escherichia coli K-12 MG1655
<220> malg b4033
<222> 792-891
<223> malg b4033 U00096 complement(4240205..4241095)
<313> 7574: 858-877
<400> 7574
CGCCGCTGCC GTGATGTCTG CATTACCGAT CACCATCGTC TTCTTGCTGG 50
CTCAACGCTG GCTGGTCAAC GGCCTGACGG CAGGTGGTGT GAAAGGTTAA 100
<210> 7575
<211> 100
<212> DNA
<213> Escherichia coli K-12 MG1655
<220> malg b4033
<222> 773-872
<223> malg b4033 U00096 complement(4240205..4241095)
<313> 7575: 818-837
<400> 7575
ACCTGTGGGG TGACTTTGCC GCCGCTGCCG TGATGTCTGC ATTACCGATC 50
ACCATCGTCT TCTTGCTGGC TCAACGCTGG CTGGTCAACG GCCTGACGGC 100
<210> 7576
<211> 100
<212> DNA
<213> Escherichia coli K-12 MG1655
<220> malg b4033
<222> 715-814
<223> malg b4033 U00096 complement(4240205..4241095)
<313> 7576: 741-760
<400> 7576
GACGTAAACA GCTACACCCT GGCCGTGGGG ATGCAGCAAT ACCTCAACCC 50
GCAAAACTAC CTGTGGGGTG ACTTTGCCGC CGCTGCCGTG ATGTCTGCAT 100
<210> 7577
<211> 100
<212> DNA
<213> Escherichia coli K-12 MG1655
<220> ydbc b1407
<222> 762-861
<223> ydbc b1407 U00096 1472245..1473105
<313> 7577: 813-832
<400> 7577
GACGTCTTCG GTTGCGCATT TACGGGAGAA TATGGCTGCT GAAAAATTGC 50
ATCTTTCTGA GGAAGTGTTG TCTACGTTGG ATGGTATTTC GCGAGAATAA 100
<210> 7578
<211> 100
<212> DNA

<213> Escherichia coli K-12 MG1655
<220> ydbc b1407
<222> 684-783
<223> ydbc b1407 U00096 1472245..1473105
<313> 7578: 735-754
<400> 7578
GAGCCTGGGT GCAACACCAA TGCAGGTGGC GCTGGCGTGG CTGTTACAGC 50
GTTCACCGAA TATTTTGCTG ATCCCAGGGA CGTCTTCGGT TGCGCATTTA 100
<210> 7579
<211> 100
<212> DNA
<213> Escherichia coli K-12 MG1655
<220> ydbc b1407
<222> 634-733
<223> ydbc b1407 U00096 1472245..1473105
<313> 7579: 658-677
<400> 7579
CTCGGGGGCT TTACACCGCT GCAATCGTCC ACACTTTCCG ATGTTGCTGC 50
GAGCCTGGGT GCAACACCAA TGCAGGTGGC GCTGGCGTGG CTGTTACAGC 100
<210> 7580
<211> 100
<212> DNA
<213> Escherichia coli K-12 MG1655
<220> mali b1621
<222> 930-1029
<223> mali b1621 U00096 complement(1696176..1697204)
<313> 7580: 996-1015
<400> 7580
TGGTATCACA CTTGCGGATC GCATGATGCA AAAAATCACC CATGAAGAGA 50
CGCATTCACG CAATCTTATT ATTCCCGCCC GGCTCATTGC AGCGAAATAA 100
<210> 7581
<211> 100
<212> DNA
<213> Escherichia coli K-12 MG1655
<220> mali b1621
<222> 917-1016
<223> mali b1621 U00096 complement(1696176..1697204)
<313> 7581: 965-984
<400> 7581
CAGCGCGGGA ACTTGGTATC ACACTTGCGG ATCGCATGAT GCAAAAAATC 50
ACCCATGAAG AGACGCATTC ACGCAATCTT ATTATTCCCG CCCGGCTCAT 100
<210> 7582
<211> 100
<212> DNA
<213> Escherichia coli K-12 MG1655
<220> mali b1621
<222> 881-980
<223> mali b1621 U00096 complement(1696176..1697204)
<313> 7582: 906-925
<400> 7582
CACTTGATGA TATACCTGTT ACCTGGGCCA GCACGCCAGC GCGGGAACTT 50
GGTATCACAC TTGCGGATCG CATGATGCAA AAAATCACCC ATGAAGAGAC 100
<210> 7583
<211> 100
<212> DNA
<213> Escherichia coli K-12 MG1655
<220> ydbd ydbD
<222> 2214-2313
<223> ydbd ydbD U00096 1473162..1475474
<313> 7583: 2269-2288
<400> 7583
TGTTAACCCT GAACTGGATT TAGTCCGCCA GCTGGCGCTT AACGGTAAGA 50
CGCGCGCAAT ACGGGAACAA GCGATGAGAA AACTTGATGA GTTGTATTGA 100
<210> 7584
<211> 100
<212> DNA
<213> Escherichia coli K-12 MG1655
<220> ydbd ydbD
<222> 2150-2249
<223> ydbd ydbD U00096 1473162..1475474
<313> 7584: 2171-2190

<400> 7584
CCCGTTTTCC AGTTGATGTA TTAAAGGCGA GGCTGAAACG TGATCCTTTG 50
CTGGCGTTAG AGCTTGTTAA CCCTGAACTG GATTTAGTCC GCCAGCTGGC 100
<210> 7585
<211> 100
<212> DNA
<213> Escherichia coli K-12 MG1655
<220> ydbd ydbD
<222> 2080-2179
<223> ydbd ydbD U00096 1473162..1475474
<313> 7585: 2110-2129
<400> 7585
CGCCACACAC CGCCTGCAGT ACTGGCGGCA GAAATTGATC CCGAGTGGTG 50
GATTGTGGCG ATGAATAATC CCCGTTTTCC AGTTGATGTA TTAAAGGCGA 100
<210> 7586
<211> 100
<212> DNA
<213> Escherichia coli K-12 MG1655
<220> exur b3095
<222> 693-792
<223> exur b3095 U00096 3244277..3245068
<313> 7586: 731-750
<400> 7586
TAACGAAACC AGCGACGACT TCGAGTTCAA TGCCGACCGC TATCTATTCG 50
CCGAAAACCC GGTGGTGCAT CTCGATACAG CCACCAGCGG CAGTAAATGA 100
<210> 7587
<211> 100
<212> DNA
<213> Escherichia coli K-12 MG1655
<220> exur b3095
<222> 612-711
<223> exur b3095 U00096 3244277..3245068
<313> 7587: 665-684
<400> 7587
CAAGGCGCTG ATTCGCAAAG ATCCTCATGC TGCTAAGCTG GCAATGTGGC 50
AGCATCTGGA AAACACCAAG ATCATGTTAT TTAACGAAAC CAGCGACGAC 100
<210> 7588
<211> 100
<212> DNA
<213> Escherichia coli K-12 MG1655
<220> exur b3095
<222> 587-686
<223> exur b3095 U00096 3244277..3245068
<313> 7588: 615-634
<400> 7588
GGTGTGATGA CCACGATCAA ATCCTCAAGG CGCTGATTCG CAAAGATCCT 50
CATGCTGCTA AGCTGGCAAT GTGGCAGCAT CTGGAAAACA CCAAGATCAT 100
<210> 7589
<211> 100
<212> DNA
<213> Escherichia coli K-12 MG1655
<220> malk b4036
<222> 1016-1115
<223> malk b4036 U00096 4244363..4245478
<313> 7589: 1054-1073
<400> 7589
AAGGTGCCAC ATTCGCTATC GGCCTGCCGC CAGAGCGTTG CCATCTGTTC 50
CGTGAGGATG GCACTGCATG TCGTCGACTG CATAAGGAGC CGGGCGTTTA 100
<210> 7590
<211> 100
<212> DNA
<213> Escherichia coli K-12 MG1655
<220> malk b4036
<222> 937-1036
<223> malk b4036 U00096 4244363..4245478
<313> 7590: 955-974
<400> 7590
GAAACTCAAA TCCATATCCA GATCCCTTCC ATTCGTCAAA ACCTGGTGTA 50
CCGCCAGAAC GACGTGGTGT TGGTAGAAGA AGGTGCCACA TTCGCTATCG 100
<210> 7591
<211> 100

<212> DNA
<213> Escherichia coli K-12 MG1655
<220> malk b4036
<222> 787-886
<223> malk b4036 U00096 4244363..4245478
<313> 7591: 821-840
<400> 7591
CGTCAGCAAG TCTGGCTGCC AGTTGAAAGC CGTGATGTCC AGGTTGGAGC 50
CAATATGTCG CTGGGTATTC GCCCGGAACA TCTACTGCCG AGTGATATCG 100
<210> 7592
<211> 100
<212> DNA
<213> Escherichia coli K-12 MG1655
<220> exut b3094
<222> 1320-1419
<223> exut b3094 U00096 3242744..3244162
<313> 7592: 1383-1402
<400> 7592
CGACCTGTTA GGTGCGCTGG TTATCTGGAC CGTGTTGCAG AACAAACCGG 50
CGATTGAGGT GGCGCAGGAA ACACATAACG ATCCCGCACC GCAACATTAA 100
<210> 7593
<211> 100
<212> DNA
<213> Escherichia coli K-12 MG1655
<220> exut b3094
<222> 1304-1403
<223> exut b3094 U00096 3242744..3244162
<313> 7593: 1359-1378
<400> 7593
CAGTTCTGGC AGTGTTCGAC CTGTTAGGTG CGCTGGTTAT CTGGACCGTG 50
TTGCAGAACA AACCGGCGAT TGAGGTGGCG CAGGAAACAC ATAACGATCC 100
<210> 7594
<211> 100
<212> DNA
<213> Escherichia coli K-12 MG1655
<220> exut b3094
<222> 1268-1367
<223> exut b3094 U00096 3242744..3244162
<313> 7594: 1297-1316
<400> 7594
CACTGGCTGA CACCATCGGC TTCAGCCCGC TGTTCGCAGT TCTGGCAGTG 50
TTCGACCTGT TAGGTGCGCT GGTTATCTGG ACCGTGTTGC AGAACAAACC 100
<210> 7595
<211> 100
<212> DNA
<213> Escherichia coli K-12 MG1655
<220> malm b4038
<222> 822-921
<223> malm b4038 U00096 4247133..4248053
<313> 7595: 888-907
<400> 7595
TGATGTTGAT AAGGCGTTAA AACTGCTTGA TGAAGCTGAA CGCCTGGGAT 50
CGACATCTGC CCGTTCCACC TTTATCAGCA GTGTAAAAGG CAAGGGGTAA 100
<210> 7596
<211> 100
<212> DNA
<213> Escherichia coli K-12 MG1655
<220> malm b4038
<222> 800-899
<223> malm b4038 U00096 4247133..4248053
<313> 7596: 857-876
<400> 7596
TCAAAAACGC TGTCGCGAAA GGTGATGTTG ATAAGGCGTT AAAACTGCTT 50
GATGAAGCTG AACGCCTGGG ATCGACATCT GCCCGTTCCA CCTTTATCAG 100
<210> 7597
<211> 100
<212> DNA
<213> Escherichia coli K-12 MG1655
<220> malm b4038
<222> 741-840
<223> malm b4038 U00096 4247133..4248053

<313> 7597: 775-794
<400> 7597
ACCGGTGAAG AAAAGCGAGC CGATGCTCAA CGACACGGAA AGTTATTTTA  50
ATACCGCGAT CAAAAACGCT GTCGCGAAAG GTGATGTTGA TAAGGCGTTA  100
<210> 7598
<211> 100
<212> DNA
<213> Escherichia coli K-12 MG1655
<220> ydbh b1382
<222> 2541-2640
<223> ydbh b1382 U00096 1441075..1443714
<313> 7598: 2611-2630
<400> 7598
TTTACGCTTT GGCGATAATC TCCAGGCATG GCTGGAGCAG AACGCACGTC  50
TGCCGGGAAA TGACTGTCCG CAAGGAAAAG AGTGTGAGGA AAAACAATGA  100
<210> 7599
<211> 100
<212> DNA
<213> Escherichia coli K-12 MG1655
<220> ydbh b1382
<222> 2540-2639
<223> ydbh b1382 U00096 1441075..1443714
<313> 7599: 2574-2593
<400> 7599
GTTTACGCTT TGGCGATAAT CTCCAGGCAT GGCTGGAGCA GAACGCACGT  50
CTGCCGGGAA ATGACTGTCC GCAAGGAAAA GAGTGTGAGG AAAAACAATG  100
<210> 7600
<211> 100
<212> DNA
<213> Escherichia coli K-12 MG1655
<220> ydbh b1382
<222> 2438-2537
<223> ydbh b1382 U00096 1441075..1443714
<313> 7600: 2479-2498
<400> 7600
CCATGCAGGC CAACATTACA GGTACCAGTC GCGTTGATGG TAAAAGCGGT  50
ACGGTAAACC TTAATTACCA TCATGAAGAG AATATTTTTA CGCTGTGGCG  100
<210> 7601
<211> 100
<212> DNA
<213> Escherichia coli K-12 MG1655
<220> malp b3418
<222> 2206-2305
<223> malp b3418 U00096 complement(3547713..3550106)
<313> 7601: 2266-2285
<400> 7601
GGCGATCCGT ATCTGGTGAT GGCGGATTTC GCAGCCTATG TAGAGGCACA  50
AAAGCAGGTG GATGTGCTGT ACCGCGACCA GGAGGCCTGG ACTCGCGCGG  100
<210> 7602
<211> 100
<212> DNA
<213> Escherichia coli K-12 MG1655
<220> malp b3418
<222> 2161-2260
<223> malp b3418 U00096 complement(3547713..3550106)
<313> 7602: 2205-2224
<400> 7602
AAGCATGCCT TCGACCAGAT GCTGCACAGT ATCGGCAAAC AGGGCGGCGA  50
TCCGTATCTG GTGATGGCGG ATTTCGCAGC CTATGTAGAG GCACAAAAGC  100
<210> 7603
<211> 100
<212> DNA
<213> Escherichia coli K-12 MG1655
<220> malp b3418
<222> 2092-2191
<223> malp b3418 U00096 complement(3547713..3550106)
<313> 7603: 2145-2164
<400> 7603
CGGAAGAAAG GTAAGGTGCT GGACGCAGTA TTGAAAGAGC TGGAAAGCGG  50
TAAATACAGC GACGGCGATA AGCATGCCTT CGACCAGATG CTGCACAGTA  100
<210> 7604

<211> 100
<212> DNA
<213> Escherichia coli K-12 MG1655
<220> ydbk b1379
<222> 3426-3525
<223> ydbk b1379 U00096 complement(1435284..1438808)
<313> 7604: 3489-3508
<400> 7604
GGCAGAACAG TTATGGAAAG ATGCTGCAGC TGATTTGCAA AAACGCTATG 50
ACTTCCTGGC ACAAATGGCC GGAAAAGCGG AAAAAAGCAA CACCGATTAA 100
<210> 7605
<211> 100
<212> DNA
<213> Escherichia coli K-12 MG1655
<220> ydbk b1379
<222> 3394-3493
<223> ydbk b1379 U00096 complement(1435284..1438808)
<313> 7605: 3415-3434
<400> 7605
TTCCGTCGGC TGAATTCGCA GCAGCCAGAA GTGGCAGAAC AGTTATGGAA 50
AGATGCTGCA GCTGATTTGC AAAAACGCTA TGACTTCCTG GCACAAATGG 100
<210> 7606
<211> 100
<212> DNA
<213> Escherichia coli K-12 MG1655
<220> ydbk b1379
<222> 3337-3436
<223> ydbk b1379 U00096 complement(1435284..1438808)
<313> 7606: 3365-3384
<400> 7606
TTGGATTCAC GCCCGCCGTC AGAAGCACCG GAAGAAACGT TACTTCACGA 50
GCAACGTTTC CGTCGGCTGA ATTCGCAGCA GCCAGAAGTG GCAGAACAGT 100
<210> 7607
<211> 100
<212> DNA
<213> Escherichia coli K-12 MG1655
<220> b2430 b2430
<222> 1275-1374
<223> b2430 b2430 U00096 2546035..2547426
<313> 7607: 1314-1333
<400> 7607
ACATTCGCCG GTTGCCGATC CGCAAAAGAA TCCCAATATG TTCGAAAGCG 50
GTCAGTTGCC GATTGCAACT TATGGTTGGG TAGTTGATCA GGTGTATGCG 100
<210> 7608
<211> 100
<212> DNA
<213> Escherichia coli K-12 MG1655
<220> b2430 b2430
<222> 1238-1337
<223> b2430 b2430 U00096 2546035..2547426
<313> 7608: 1269-1288
<400> 7608
TGAATCATAT GACGATTGTG ATGTTAAGCA ACAAGCCACA TTCGCCGGTT 50
GCCGATCCGC AAAAGAATCC CAATATGTTC GAAAGCGGTC AGTTGCCGAT 100
<210> 7609
<211> 100
<212> DNA
<213> Escherichia coli K-12 MG1655
<220> b2430 b2430
<222> 1153-1252
<223> b2430 b2430 U00096 2546035..2547426
<313> 7609: 1183-1202
<400> 7609
ATGACGCCGA CGTTTGGCAC GCTGGCAAGC CCGCAAACCT ACGGTCACAC 50
TGGCTGGACC GGAACGGTGA CCGTTATCGA TCCGGTGAAT CATATGACGA 100
<210> 7610
<211> 100
<212> DNA
<213> Escherichia coli K-12 MG1655
<220> malq b3417
<222> 1986-2085

<223> malq b3417 U00096 complement(3545619..3547703)
<313> 7610: 2043-2062
<400> 7610
GCTTTCCGCA ACGCTTGAGT CGATGTTTGC CGATGATGGC GTGAACAAGT 50
TGCTGAAGGA TTTGGACAGA CGGCGCAGAG CTGCAGCGAA GAAGAAGTAG 100
<210> 7611
<211> 100
<212> DNA
<213> Escherichia coli K-12 MG1655
<220> malq b3417
<222> 1969-2068
<223> malq b3417 U00096 complement(3545619..3547703)
<313> 7611: 1986-2005
<400> 7611
AAAAACTGGC GACGCAAGCT TTCCGCAACG CTTGAGTCGA TGTTTGCCGA 50
TGATGGCGTG AACAAGTTGC TGAAGGATTT GGACAGACGG CGCAGAGCTG 100
<210> 7612
<211> 100
<212> DNA
<213> Escherichia coli K-12 MG1655
<220> malq b3417
<222> 1869-1968
<223> malq b3417 U00096 complement(3545619..3547703)
<313> 7612: 1883-1902
<400> 7612
CTACATTGCC GACAGTAACA GTGCTCTGTT AGGACTACAG CCGGAAGACT 50
GGCTGGATAT GGCCGAACCG GTGAATATTC CTGGCACCAG TTACCAGTAT 100
<210> 7613
<211> 100
<212> DNA
<213> Escherichia coli K-12 MG1655
<220> ydbl b1384
<222> 234-333
<223> ydbl b1384 U00096 1443898..1444230
<313> 7613: 295-314
<400> 7613
AGATGATATC GCGAAACTCG CCGGGCAAAA GCTCGTAGCT CGGGCCAAAC 50
CGGGGGAATA TGTACAAGGG ATAAACGGTA AATGGGTGCG AAAATTTTAA 100
<210> 7614
<211> 100
<212> DNA
<213> Escherichia coli K-12 MG1655
<220> ydbl b1384
<222> 168-267
<223> ydbl b1384 U00096 1443898..1444230
<313> 7614: 188-207
<400> 7614
AGCCGACATT AATGCCGAAC GTAAAGCGAG TTACCAACAA CTGGCAAAGC 50
AAAATAATGT GTCGGTAGAT GATATCGCGA AACTCGCCGG GCAAAAGCTC 100
<210> 7615
<211> 100
<212> DNA
<213> Escherichia coli K-12 MG1655
<220> ydbl b1384
<222> 89-188
<223> ydbl b1384 U00096 1443898..1444230
<313> 7615: 141-160
<400> 7615
GAACTCAGGG GCGGGTAGGT GAAACATTTT ACGGTTATCT GGTTGCGCTG 50
AAAACGGATG CTGAAACAGA GAAATTAGTA GCCGACATTA ATGCCGAACG 100
<210> 7616
<211> 100
<212> DNA
<213> Escherichia coli K-12 MG1655
<220> b2431 b2431
<222> 734-833
<223> b2431 b2431 U00096 complement(2547666..2548592)
<313> 7616: 752-771
<400> 7616
CGTACGGCAC TGCCAGTGGC ACTCACGGTC TGTACTTCTG CGCCTACTGC 50
GCGCGTCTGC ATAACATTGA GCAGCAACTG CTGAGCATGT TTGGCGATAC 100

<210> 7617
<211> 100
<212> DNA
<213> Escherichia coli K-12 MG1655
<220> b2431 b2431
<222> 675-774
<223> b2431 b2431 U00096 complement(2547666..2548592)
<313> 7617: 703-722
<400> 7617
CACCCGCGTT GATCTGAAAG AAGATGGCAA AGGGCTGAAG ATTGTTCGCC 50
AGAGCCTGCC GTACGGCACT GCCAGTGGCA CTCACGGTCT GTACTTCTGC 100
<210> 7618
<211> 100
<212> DNA
<213> Escherichia coli K-12 MG1655
<220> b2431 b2431
<222> 628-727
<223> b2431 b2431 U00096 complement(2547666..2548592)
<313> 7618: 661-680
<400> 7618
GCCAACGAAG AGATCGACGG CGACGAACGT CCGGAAACCT CTCACCTCAC 50
CCGCGTTGAT CTGAAAGAAG ATGGCAAAGG GCTGAAGATT GTTCGCCAGA 100
<210> 7619
<211> 100
<212> DNA
<213> Escherichia coli K-12 MG1655
<220> b2432 b2432
<222> 477-576
<223> b2432 b2432 U00096 complement(2548661..2549236)
<313> 7619: 535-554
<400> 7619
CTACCAGTTC AGCGGGGAAT GGCGTGGTCC TGAAGGGTTA ATGCCTTCGG 50
ACGATACCCT GAAAAACTGG AAAGTCAGTA AAATTATCTG GCGGCGTTAA 100
<210> 7620
<211> 100
<212> DNA
<213> Escherichia coli K-12 MG1655
<220> b2432 b2432
<222> 431-530
<223> b2432 b2432 U00096 complement(2548661..2549236)
<313> 7620: 488-507
<400> 7620
ATAATCGTGC CGTCGAATGT AAAGCCGAAG GCAGTCAACA TATTAGCTAC 50
CAGTTCAGCG GGGAATGGCG TGGTCCTGAA GGGTTAATGC CTTCGGACGA 100
<210> 7621
<211> 100
<212> DNA
<213> Escherichia coli K-12 MG1655
<220> b2432 b2432
<222> 389-488
<223> b2432 b2432 U00096 complement(2548661..2549236)
<313> 7621: 448-467
<400> 7621
ACAGCAAAGC ATTTGGCAAT TGCCAAAAAG CTGATGGTGA TGATAATCGT 50
GCCGTCGAAT GTAAAGCCGA AGGCAGTCAA CATATTAGCT ACCAGTTCAG 100
<210> 7622
<211> 100
<212> DNA
<213> Escherichia coli K-12 MG1655
<220> malS b3572
<222> 1901-2000
<223> malS b3572 U00096 3735126..3737156
<313> 7622: 1952-1971
<400> 7622
GCCAGTTCCG CGCCCGCCAT CCCGCAATTG GCGCGGGCAA ACAAACGACA 50
CTTTTGCTGA AGCAGGGCTA CGGCTTTGTT CGTGAGCATG GCGACGATAA 100
<210> 7623
<211> 100
<212> DNA
<213> Escherichia coli K-12 MG1655
<220> malS b3572

<222> 1859-1958
<223> mals b3572 U00096 3735126..3737156
<313> 7623: 1906-1925
<400> 7623
GCGGTAAATC TGCCGCCAGC GTCGCGCACT GGCAGAAAAT CAGCCAGTTC 50
CGCGCCCGCC ATCCCGCAAT TGGCGCGGGC AAACAAACGA CACTTTTGCT 100
<210> 7624
<211> 100
<212> DNA
<213> Escherichia coli K-12 MG1655
<220> mals b3572
<222> 1789-1888
<223> mals b3572 U00096 3735126..3737156
<313> 7624: 1831-1850
<400> 7624
CGTCCGTTCG GTCCTACAGG TTCTGATCCG CTGCAAGGTA CACGTTCGGA 50
TATGAACTGG CAGGATGTTA GCGGTAAATC TGCCGCCAGC GTCGCGCACT 100
<210> 7625
<211> 100
<212> DNA
<213> Escherichia coli K-12 MG1655
<220> b2433 b2433
<222> 357-456
<223> b2433 b2433 U00096 complement(2549297..2549752)
<313> 7625: 414-433
<400> 7625
CGGTATCGGT CTGGCGCTGG TAGTGGCGGA TATTGTGGCG TTAATCTGGC 50
TGCTGACCAA TCGACGTTTA CGCGCTTGTT TTTATGAAGT AAAAGAATAA 100
<210> 7626
<211> 100
<212> DNA
<213> Escherichia coli K-12 MG1655
<220> b2433 b2433
<222> 242-341
<223> b2433 b2433 U00096 complement(2549297..2549752)
<313> 7626: 287-306
<400> 7626
GTCGGCGGGC TACGTTTCCT CGCACCTGGC GTGTACTCTA TTTTCTGTTG 50
CTGTTGGCGC AGGTGGTTTT ACTTTGCTGG CAACCGTGGC TGTGGCTGAA 100
<210> 7627
<211> 100
<212> DNA
<213> Escherichia coli K-12 MG1655
<220> b2433 b2433
<222> 220-319
<223> b2433 b2433 U00096 complement(2549297..2549752)
<313> 7627: 241-260
<400> 7627
GTGCTGGCGT TTTTGTTGAG CGGTCGGCGG GCTACGTTTC CTCGCACCTG 50
GCGTGTACTC TATTTTCTGT TGCTGTTGGC GCAGGTGGTT TTACTTTGCT 100
<210> 7628
<211> 100
<212> DNA
<213> Escherichia coli K-12 MG1655
<220> malt b3419
<222> 2607-2706
<223> malt b3419 U00096 3550718..3553423
<313> 7628: 2651-2670
<400> 7628
GCATATCCGC AATCTGTATC AGAAACTCGG CGTGGCCCAT CGCCAGGATG 50
CGGTACAACA CGCCCAGCAA TTGCTGAAGA TGATGGGGTA CGGCGTGTAA 100
<210> 7629
<211> 100
<212> DNA
<213> Escherichia coli K-12 MG1655
<220> malt b3419
<222> 2567-2666
<223> malt b3419 U00096 3550718..3553423
<313> 7629: 2599-2618
<400> 7629
TTGCCGGAGA ACTGGAAGTC GCGGCAACCA CCATCAAAAC GCATATCCGC 50

AATCTGTATC AGAAACTCGG CGTGGCCCAT CGCCAGGATG CGGTACAACA 100
<210> 7630
<211> 100
<212> DNA
<213> Escherichia coli K-12 MG1655
<220> malt b3419
<222> 2532-2631
<223> malt b3419 U00096 3550718..3553423
<313> 7630: 2553-2572
<400> 7630
GGGGCTGATC TACTCTGGTT ACAGCAATGA GCAAATTGCC GGAGAACTGG 50
AAGTCGCGGC AACCACCATC AAAACGCATA TCCGCAATCT GTATCAGAAA 100
<210> 7631
<211> 100
<212> DNA
<213> Escherichia coli K-12 MG1655
<220> ydbo b1389
<222> 831-930
<223> ydbo b1389 U00096 1451951..1452880
<313> 7631: 898-917
<400> 7631
GAGGCTCGAC GCCAAACGTA AAGCCTGGGA AGAAGGTACC TGGGTACGGG 50
AAGCAGCGCT GGCCCATGCA CAAAAACAAC ATGCCCGTAA GGTCGCATAA 100
<210> 7632
<211> 100
<212> DNA
<213> Escherichia coli K-12 MG1655
<220> ydbo b1389
<222> 796-895
<223> ydbo b1389 U00096 1451951..1452880
<313> 7632: 838-857
<400> 7632
GAAGTAATTA ACGGTCGCGG AATTTGTAAT CAGGAGAGGC TCGACGCCAA 50
ACGTAAAGCC TGGGAAGAAG GTACCTGGGT ACGGGAAGCA GCGCTGGCCC 100
<210> 7633
<211> 100
<212> DNA
<213> Escherichia coli K-12 MG1655
<220> ydbo b1389
<222> 744-843
<223> ydbo b1389 U00096 1451951..1452880
<313> 7633: 796-815
<400> 7633
CACTGAAAGC GGTCACTACC GCTTTGGTGA GATCGACTGG CAGGAGTTTA 50
ACGAAGTAAT TAACGGTCGC GGAATTTGTA ATCAGGAGAG GCTCGACGCC 100
<210> 7634
<211> 100
<212> DNA
<213> Escherichia coli K-12 MG1655
<220> b2434 b2435
<222> 438-537
<223> b2434 b2435 U00096 complement(2549733..2550269)
<313> 7634: 485-504
<400> 7634
AGATAACGAC ATGGTGCTCG GAATGTATGA ACGCCTGGGA TATGAACACG 50
CCGACGTGCT GAGTCTGGGT AAGCGTTTGA TTGAAGATGA AGAGTACTGA 100
<210> 7635
<211> 100
<212> DNA
<213> Escherichia coli K-12 MG1655
<220> b2434 b2435
<222> 403-502
<223> b2434 b2435 U00096 complement(2549733..2550269)
<313> 7635: 435-454
<400> 7635
CGTGGCTGCC CGAAAATTCA GATCAATGTG CCGGAAGATA ACGACATGGT 50
GCTCGGAATG TATGAACGCC TGGGATATGA ACACGCCGAC GTGCTGAGTC 100
<210> 7636
<211> 100
<212> DNA
<213> Escherichia coli K-12 MG1655

<220> b2434 b2435
<222> 304-403
<223> b2434 b2435 U00096 complement(2549733..2550269)
<313> 7636: 324-343
<400> 7636
CATCGCGGGT CTGCTTATTA TCTTGGCGTG CATCCAGAGT TTCGTGGGCG 50
TGGGATTGCC AATGCGTTGC TTAATCGGCT GGAGAAAAAG CTGATTGCTC 100
<210> 7637
<211> 100
<212> DNA
<213> Escherichia coli K-12 MG1655
<220> ydbp ydbP
<222> 648-747
<223> ydbp ydbP U00096 1453188..1453934
<313> 7637: 719-738
<400> 7637
TCGCACTGGC GGTAAAAAAG GACTGCATAC CGAACACCTT GGACCCATGC 50
TGGCAGAAAT GCAGTATCTC CAGCGTGTCT TGCCCGGTCA GCAATGGTAA 100
<210> 7638
<211> 100
<212> DNA
<213> Escherichia coli K-12 MG1655
<220> ydbp ydbP
<222> 638-737
<223> ydbp ydbP U00096 1453188..1453934
<313> 7638: 663-682
<400> 7638
AGCAGGCGTA TCGCACTGGC GGTAAAAAAG GACTGCATAC CGAACACCTT 50
GGACCCATGC TGGCAGAAAT GCAGTATCTC CAGCGTGTCT TGCCCGGTCA 100
<210> 7639
<211> 100
<212> DNA
<213> Escherichia coli K-12 MG1655
<220> ydbp ydbP
<222> 585-684
<223> ydbp ydbP U00096 1453188..1453934
<313> 7639: 622-641
<400> 7639
GGAAGCCGAA GTTTTTGCCG GGATCAACGA AGCCACATTG AACGTACCGC 50
AAGAGCAGGC GTATCGCACT GGCGGTAAAA AAGGACTGCA TACCGAACAC 100
<210> 7640
<211> 100
<212> DNA
<213> Escherichia coli K-12 MG1655
<220> b1706 b1706
<222> 1325-1424
<223> b1706 b1706 U00096 complement(1787832..1789268)
<313> 7640: 1350-1369
<400> 7640
AATTGCACCG CCTGCATGAG GCGTTGCGAA ATCCTTTCAG CGACAGAGAT 50
GATGACTATG TCAGTCGTCC ACCTGACTGG GGTAAACGGC TGGAAGTCAG 100
<210> 7641
<211> 100
<212> DNA
<213> Escherichia coli K-12 MG1655
<220> b1706 b1706
<222> 1273-1372
<223> b1706 b1706 U00096 complement(1787832..1789268)
<313> 7641: 1312-1331
<400> 7641
AACTGGTTGG CGCAACGGGC GATTGAAGCG GCAGAAAAGG GTGATATGAC 50
GGAATTGCAC CGCCTGCATG AGGCGTTGCG AAATCCTTTC AGCGACAGAG 100
<210> 7642
<211> 100
<212> DNA
<213> Escherichia coli K-12 MG1655
<220> b1706 b1706
<222> 1206-1305
<223> b1706 b1706 U00096 complement(1787832..1789268)
<313> 7642: 1233-1252
<400> 7642

AGACGAGGTT AGCGATAGTG AGCGTCAGCA ACTGATGCAA AGCGTTAACC 50
CCGCTCTGGT ATTGCGCAAC TGGTTGGCGC AACGGGCGAT TGAAGCGGCA 100
<210> 7643
<211> 100
<212> DNA
<213> Escherichia coli K-12 MG1655
<220> b1707 b1708
<222> 615-714
<223> b1707 b1708 U00096 complement(1789331..1790044)
<313> 7643: 679-698
<400> 7643
AATATTGGCA CAGATTACCC CCTTCGATTT CCATGCTCTC CAGGGATGCC 50
TGTGGCCTGC CGTTCCCATA AATCAGATAA CGACGTTGGT TCAGCGATAA 100
<210> 7644
<211> 100
<212> DNA
<213> Escherichia coli K-12 MG1655
<220> b1707 b1708
<222> 596-695
<223> b1707 b1708 U00096 complement(1789331..1790044)
<313> 7644: 635-654
<400> 7644
GAGGCATTGA TACGGCAGAA ATATTGGCAC AGATTACCCC CTTCGATTTC 50
CATGCTCTCC AGGGATGCCT GTGGCCTGCC GTTCCCATAA ATCAGATAAC 100
<210> 7645
<211> 100
<212> DNA
<213> Escherichia coli K-12 MG1655
<220> b1707 b1708
<222> 550-649
<223> b1707 b1708 U00096 complement(1789331..1790044)
<313> 7645: 581-600
<400> 7645
GCGATCCAGG CGCAAATTTC CCCTTGTTGC AATTGCATCA TTGCGGGAGG 50
CATTGATACG GCAGAAATAT TGGCACAGAT TACCCCCTTC GATTTCCATG 100
<210> 7646
<211> 100
<212> DNA
<213> Escherichia coli K-12 MG1655
<220> ordl b1302
<222> 1132-1231
<223> ordl b1302 U00096 1362256..1363536
<313> 7646: 1170-1189
<400> 7646
ATTGCCGAAC TGCTGCGCGG CGACGCCGAA CGTTTCGATG CCTTCGCCAA 50
TCTGCCGCAT TACCCGTTCC CCGGCGGGCG CACGCTGCGT GTGCCGTTTA 100
<210> 7647
<211> 100
<212> DNA
<213> Escherichia coli K-12 MG1655
<220> ordl b1302
<222> 1046-1145
<223> ordl b1302 U00096 1362256..1363536
<313> 7647: 1107-1126
<400> 7647
AGTTTGGTCG CCTCGATACC AACATCTATT ACATGCAGGG CTACAGCGGC 50
CACGGCGTGA CCTGTACTCA TCTAGCCGGA CGTTTGATTG CCGAACTGCT 100
<210> 7648
<211> 100
<212> DNA
<213> Escherichia coli K-12 MG1655
<220> ordl b1302
<222> 1004-1103
<223> ordl b1302 U00096 1362256..1363536
<313> 7648: 1066-1085
<400> 7648
GCTGGACGGG CAACTTCCTG CTGACCCTGT CGCGTATGCC GCAGTTTGGT 50
CGCCTCGATA CCAACATCTA TTACATGCAG GGCTACAGCG GCCACGGCGT 100
<210> 7649
<211> 100
<212> DNA

<213> Escherichia coli K-12 MG1655
<220> rimi b4374
<222> 348-447
<223> rimi b4374 U00096 4605754..4606200
<313> 7649: 386-405
<400> 7649
AAGTTTAGGC TTTAACGAGG CGACGATTCG CCGCAATTAC TACCCCACCA 50
CGGACGGTCG CGAAGACGCC ATCATCATGG CGTTGCCAAT CAGTATGTAA 100
<210> 7650
<211> 100
<212> DNA
<213> Escherichia coli K-12 MG1655
<220> rimi b4374
<222> 283-382
<223> rimi b4374 U00096 4605754..4606200
<313> 7650: 328-347
<400> 7650
CGCGGCGTGG CGACACTATG GCTGGAAGTC CGTGCTTCAA ACGCTGCCGC 50
CATTGCCCTG TACGAAAGTT TAGGCTTTAA CGAGGCGACG ATTCGCCGCA 100
<210> 7651
<211> 100
<212> DNA
<213> Escherichia coli K-12 MG1655
<220> rimi b4374
<222> 237-336
<223> rimi b4374 U00096 4605754..4606200
<313> 7651: 288-307
<400> 7651
ATTGGGAAGG GCGCTGCTGG AACATCTGAT CGACGAACTG GAAAAACGCG 50
GCGTGGCGAC ACTATGGCTG GAAGTCCGTG CTTCAAACGC TGCCGCCATT 100
<210> 7652
<211> 100
<212> DNA
<213> Escherichia coli K-12 MG1655
<220> malx b1622
<222> 1477-1576
<223> malx b1622 U00096 1697379..1698971
<313> 7652: 1532-1551
<400> 7652
AATCGGGCAA TTGGCGTAGT ACAACTTAAT CAACATAACC TGCAGGTTGT 50
TATCGGGCCA CAAGTTCAGT CAGTAAAAGA TGAAATGGCC GGTCTGATGC 100
<210> 7653
<211> 100
<212> DNA
<213> Escherichia coli K-12 MG1655
<220> malx b1622
<222> 1446-1545
<223> malx b1622 U00096 1697379..1698971
<313> 7653: 1492-1511
<400> 7653
GTCGCTTGTT AATGTGCAGG CACTGAAGGA CAATCGGGCA ATTGGCGTAG 50
TACAACTTAA TCAACATAAC CTGCAGGTTG TTATCGGGCC ACAAGTTCAG 100
<210> 7654
<211> 100
<212> DNA
<213> Escherichia coli K-12 MG1655
<220> malx b1622
<222> 1369-1468
<223> malx b1622 U00096 1697379..1698971
<313> 7654: 1405-1424
<400> 7654
GAAGCATTAG GCGGTGCCGA CAATATTGTC AGCCTCGATA ACTGCATTAC 50
CCGTCTGCGT TTGTCTGTGA AAGATATGTC GCTTGTTAAT GTGCAGGCAC 100
<210> 7655
<211> 100
<212> DNA
<213> Escherichia coli K-12 MG1655
<220> rimj b1067
<222> 486-585
<223> rimj b1067 U00096 1124785..1125369
<313> 7655: 556-575

<400> 7655
AAAAGAAGGC TATGCGAAAG ACTATCTGTT GATTGATGGA CAATGGCGCG  50
ATCACGTACT GACGGCATTA ACTACCCCAG ACTGGACGCC CGGCCGCTAA  100
<210> 7656
<211> 100
<212> DNA
<213> Escherichia coli K-12 MG1655
<220> rimj b1067
<222> 441-540
<223> rimj b1067 U00096 1124785..1125369
<313> 7656: 478-497
<400> 7656
GCACAATAAA CGCAGCGGTG ATTTACTGGC GCGACTGGGT TTTGAAAAAG  50
AAGGCTATGC GAAAGACTAT CTGTTGATTG ATGGACAATG GCGCGATCAC  100
<210> 7657
<211> 100
<212> DNA
<213> Escherichia coli K-12 MG1655
<220> rimj b1067
<222> 413-512
<223> rimj b1067 U00096 1124785..1125369
<313> 7657: 434-453
<400> 7657
TTCATCGCAT TATGGCTAAT TATATGCCGC ACAATAAACG CAGCGGTGAT  50
TTACTGGCGC GACTGGGTTT TGAAAAAGAA GGCTATGCGA AAGACTATCT  100
<210> 7658
<211> 100
<212> DNA
<213> Escherichia coli K-12 MG1655
<220> ydbs ydbS
<222> 669-768
<223> ydbs ydbS U00096 1455521..1456288
<313> 7658: 739-758
<400> 7658
GGAGCGACAG TTATTCACCT TGCTGGCGGC AACAGAAGAT CGTCATGAAG  50
GCATCTCCGC TTTCTTACAA AAACGCACGC CCGACTTTAA AGGACGCTAA  100
<210> 7659
<211> 100
<212> DNA
<213> Escherichia coli K-12 MG1655
<220> ydbs ydbS
<222> 631-730
<223> ydbs ydbS U00096 1455521..1456288
<313> 7659: 663-682
<400> 7659
CAGTCGCAGG AAGTGGCTTT GCAAGCCGGA CTTGCCCAGG AGCGACAGTT  50
ATTCACCTTG CTGGCGGCAA CAGAAGATCG TCATGAAGGC ATCTCCGCTT  100
<210> 7660
<211> 100
<212> DNA
<213> Escherichia coli K-12 MG1655
<220> ydbs ydbS
<222> 540-639
<223> ydbs ydbS U00096 1455521..1456288
<313> 7660: 584-603
<400> 7660
CGATTTAACC CTCGAATACG CCTTACAGCT GGCATCGAAA ATGGCACGTC  50
ACTCGCCGCT GGCCTTACAA GCGGCAAAGC AAGCGCTGCG CCAGTCGCAG  100
<210> 7661
<211> 100
<212> DNA
<213> Escherichia coli K-12 MG1655
<220> b2438 b2438
<222> 408-507
<223> b2438 b2438 U00096 complement(2553248..2553754)
<313> 7661: 486-505
<400> 7661
TGGCTGGCCG CTGGAAAAAG CCAGAAATGC GCTCGAACAG CTCTTTTCTG  50
CCGGGACGTT ACGTAAACGC AGTAGTCGTT ATCGTCTCAA GCCCCATTAA  100
<210> 7662
<211> 100

<212> DNA
<213> Escherichia coli K-12 MG1655
<220> b2438 b2438
<222> 399-498
<223> b2438 b2438 U00096 complement(2553248..2553754)
<313> 7662: 438-457
<400> 7662
TGCCCACTTT GGCTGGCCGC TGGAAAAAGC CAGAAATGCG CTCGAACAGC 50
TCTTTTCTGC CGGGACGTTA CGTAAACGCA GTAGTCGTTA TCGTCTCAAG 100
<210> 7663
<211> 100
<212> DNA
<213> Escherichia coli K-12 MG1655
<220> b2438 b2438
<222> 313-412
<223> b2438 b2438 U00096 complement(2553248..2553754)
<313> 7663: 372-391
<400> 7663
GCGCCAGTTA TCGTCGCGGA ATCTGCTGAC GAGTTGTTGG CGCTGTTAAC 50
ATCAGTACGT CAGGGAATGA CGGCAGGAGA AGTGGCTGCC CACTTTGGCT 100
<210> 7664
<211> 100
<212> DNA
<213> Escherichia coli K-12 MG1655
<220> maly b1623
<222> 1074-1173
<223> maly b1623 U00096 1698981..1700153
<313> 7664: 1117-1136
<400> 7664
AGAAGGTCGT GGTTTTGTCC GTCTCAATGC CGGCTGCCCA CGTTCGAAAC 50
TGGAAAAAGG TGTGGCTGGA TTAATTAACG CCATCCGCGC TGTTCGTTAA 100
<210> 7665
<211> 100
<212> DNA
<213> Escherichia coli K-12 MG1655
<220> maly b1623
<222> 935-1034
<223> maly b1623 U00096 1698981..1700153
<313> 7665: 989-1008
<400> 7665
AACTCAACTG GCAGATCCCA CAATCCACTT ATCTGGCATG GCTTGATTTA 50
CGTCCGTTGA ATATTGACGA CAACGCGTTG CAAAAAGCAC TTATCGAACA 100
<210> 7666
<211> 100
<212> DNA
<213> Escherichia coli K-12 MG1655
<220> maly b1623
<222> 886-985
<223> maly b1623 U00096 1698981..1700153
<313> 7666: 944-963
<400> 7666
AAAGATAACC TGACGTATAT CGCAGATAAA ATGAACGCCG CGTTTCCTGA 50
ACTCAACTGG CAGATCCCAC AATCCACTTA TCTGGCATGG CTTGATTTAC 100
<210> 7667
<211> 100
<212> DNA
<213> Escherichia coli K-12 MG1655
<220> rimk b0853
<222> 804-903
<223> rimk b0853 U00096 891190..892092
<313> 7667: 860-879
<400> 7667
AGGAATAGAA AAAACCACCG GTATCGACAT CGCGGGTAAA ATGATCCGCT 50
GGATCGAACG CCACGCTACG ACAGAATATT GCCTGAAAAC GGGTGGTTAG 100
<210> 7668
<211> 100
<212> DNA
<213> Escherichia coli K-12 MG1655
<220> rimk b0853
<222> 743-842
<223> rimk b0853 U00096 891190..892092

<313> 7668: 803-822
<400> 7668
ATATTCTGCG TGCTAATCGC GGGCCGTTGG TGATGGAGGT GAATGCGTCG 50
CCGGGGCTGG AAGGAATAGA AAAAACCACC GGTATCGACA TCGCGGGTAA 100
<210> 7669
<211> 100
<212> DNA
<213> Escherichia coli K-12 MG1655
<220> rimk b0853
<222> 726-825
<223> rimk b0853 U00096 891190..892092
<313> 7669: 757-776
<400> 7669
GGACGTTGCT GGTGTGGATA TTCTGCGTGC TAATCGCGGG CCGTTGGTGA 50
TGGAGGTGAA TGCGTCGCCG GGGCTGGAAG GAATAGAAAA AACCACCGGT 100
<210> 7670
<211> 100
<212> DNA
<213> Escherichia coli K-12 MG1655
<220> b2439 b2440
<222> 560-659
<223> b2439 b2440 U00096 complement(2553761..2554420)
<313> 7670: 594-613
<400> 7670
CAGCATTTTT AACCGGTAGC CAGGCAGCGT GTAAAGCAGC CTGTAACGCC 50
TTTACCGATG CAGTGCTGGA AATCGCGCGT AATCCAATCC AGCGTGCGTA 100
<210> 7671
<211> 100
<212> DNA
<213> Escherichia coli K-12 MG1655
<220> b2439 b2440
<222> 496-595
<223> b2439 b2440 U00096 complement(2553761..2554420)
<313> 7671: 553-572
<400> 7671
CTGAAGTCTG CCGACGTACA GCTGGCGACC TATGTCCCGC CACCGTCTGA 50
AACCAACTAC TCGGCAGCAT TTTTAACCGG TAGCCAGGCA GCGTGTAAAG 100
<210> 7672
<211> 100
<212> DNA
<213> Escherichia coli K-12 MG1655
<220> b2439 b2440
<222> 408-507
<223> b2439 b2440 U00096 complement(2553761..2554420)
<313> 7672: 438-457
<400> 7672
CTCATCAACC GCCGGGATCA CGCTTGGCGA TCCGATGGCG TATCTGGTGG 50
CACCGCCGCT GGAAGCGACC TATGGCATTG ATGCAGCCCT GAAGTCTGCC 100
<210> 7673
<211> 100
<212> DNA
<213> Escherichia coli K-12 MG1655
<220> malz b0404
<222> 1719-1818
<223> malz b0404 U00096 421739..423556
<313> 7673: 1793-1812
<400> 7673
TCTCAATGCC GTGCAATGGC AATGCAAAGA AGGGCATGGG CAACTGACTG 50
ACGGGATTCT GGCTTTGCCT GCCATTTCGG CTACGGTATG GATGAACTAA 100
<210> 7674
<211> 100
<212> DNA
<213> Escherichia coli K-12 MG1655
<220> malz b0404
<222> 1709-1808
<223> malz b0404 U00096 421739..423556
<313> 7674: 1744-1763
<400> 7674
CGTCACCGTT TCTCAATGCC GTGCAATGGC AATGCAAAGA AGGGCATGGG 50
CAACTGACTG ACGGGATTCT GGCTTTGCCT GCCATTTCGG CTACGGTATG 100
<210> 7675

<211> 100
<212> DNA
<213> Escherichia coli K-12 MG1655
<220> malz b0404
<222> 1653-1752
<223> malz b0404 U00096 421739..423556
<313> 7675: 1714-1733
<400> 7675
GCAACGTGTA CTGGTGGCAA TCAACCGTGG CGAGGCCTGT GAAGTGGTGC 50
TACCCGCGTC ACCGTTTCTC AATGCCGTGC AATGGCAATG CAAAGAAGGG 100
<210> 7676
<211> 100
<212> DNA
<213> Escherichia coli K-12 MG1655
<220> riml rimL
<222> 440-539
<223> riml rimL U00096 1496962..1497501
<313> 7676: 475-494
<400> 7676
GCAATGGTTT TATCCTTGAA GGTTGCCTGA AACAGGCTGA GTTCCTGAAT 50
GATGCCTATG ATGATGTGAA CTTATACGCG CGTATTATCG ATTCACAATA 100
<210> 7677
<211> 100
<212> DNA
<213> Escherichia coli K-12 MG1655
<220> riml rimL
<222> 360-459
<223> riml rimL U00096 1496962..1497501
<313> 7677: 400-419
<400> 7677
TTACGCCCAG TCTGGTGAAC TTAGACGCTT CGTGATCAAA TGTCGGGTGG 50
ACAATCCGCA AAGCAACCAG GTCGCTTTGC GCAATGGTTT TATCCTTGAA 100
<210> 7678
<211> 100
<212> DNA
<213> Escherichia coli K-12 MG1655
<220> riml rimL
<222> 276-375
<223> riml rimL U00096 1496962..1497501
<313> 7678: 316-335
<400> 7678
AACCGCTGAA ATAGGCTACT GGCTGGACGA ATCTCATCAG GGGCAGGGGA 50
TCATTTCTCA GGCGCTGCAG GCATTGATTC ATCATTACGC CCAGTCTGGT 100
<210> 7679
<211> 100
<212> DNA
<213> Escherichia coli K-12 MG1655
<220> ydbu ydbU
<222> 1329-1428
<223> ydbu ydbU U00096 1457078..1458505
<313> 7679: 1375-1394
<400> 7679
GCTCCTTGAA AATCTACAGC ATCACTATGG CGAAGAACGC TATCGCCCAT 50
GTTCATTGCT GCGCCAACGG GCGCTTCTGG AGAGCGGTTA TGAGTCATAA 100
<210> 7680
<211> 100
<212> DNA
<213> Escherichia coli K-12 MG1655
<220> ydbu ydbU
<222> 1282-1381
<223> ydbu ydbU U00096 1457078..1458505
<313> 7680: 1328-1347
<400> 7680
GGCCCACTTG CCTGGGGAGC GCAACTTGGC TGGCAGCGAA TATTAAGGCT 50
CCTTGAAAAT CTACAGCATC ACTATGGCGA AGAACGCTAT CGCCCATGTT 100
<210> 7681
<211> 100
<212> DNA
<213> Escherichia coli K-12 MG1655
<220> ydbu ydbU
<222> 1171-1270

<223> ydbu ydbU U00096 1457078..1458505
<313> 7681: 1221-1240
<400> 7681
TGGCGAACGG TAGCAATGAT CATCAATGAA GCCCTTGATG CGCTTCAAAA 50
AGGCGTGGCC TCTGAACAGG ATATCGATAC CGCCATGCGT CTTGGGGTGA 100
<210> 7682
<211> 100
<212> DNA
<213> Escherichia coli K-12 MG1655
<220> sura b0054
<222> 1188-1287
<223> sura b0054 U00096 complement(53416..54702)
<313> 7682: 1262-1281
<400> 7682
TGCATACCGC ATGCTGATGA ACCGTAAGTT CTCGGAAGAA GCAGCAAGCT 50
GGATGCAGGA ACAACGTGCC AGCGCCTACG TTAAAATCCT GAGCAACTAA 100
<210> 7683
<211> 100
<212> DNA
<213> Escherichia coli K-12 MG1655
<220> sura b0054
<222> 1174-1273
<223> sura b0054 U00096 complement(53416..54702)
<313> 7683: 1221-1240
<400> 7683
GCGCAGAAAG ATCGTGCATA CCGCATGCTG ATGAACCGTA AGTTCTCGGA 50
AGAAGCAGCA AGCTGGATGC AGGAACAACG TGCCAGCGCC TACGTTAAAA 100
<210> 7684
<211> 100
<212> DNA
<213> Escherichia coli K-12 MG1655
<220> sura b0054
<222> 1027-1126
<223> sura b0054 U00096 complement(53416..54702)
<313> 7684: 1077-1096
<400> 7684
TGGGCTACAC CAGATATTTT CGATCCGGCC TTCCGTGACG CCCTGACTCG 50
CCTGAACAAA GGTCAAATGA GTGCACCGGT TCACTCTTCA TTCGGCTGGC 100
<210> 7685
<211> 100
<212> DNA
<213> Escherichia coli K-12 MG1655
<220> trua b2319
<222> 682-781
<223> trua b2319 U00096 complement(2432844..2433656)
<313> 7685: 711-730
<400> 7685
CTGGCGGCAA AGGACAGAAC GCTGGCGGCA GCAACGGCAA AAGCGGAAGG 50
GCTGTATCTG GTCGCGGTGG ATTACCCTGA CCGGTATGAT CTTCCAAAAC 100
<210> 7686
<211> 100
<212> DNA
<213> Escherichia coli K-12 MG1655
<220> trua b2319
<222> 628-727
<223> trua b2319 U00096 complement(2432844..2433656)
<313> 7686: 650-669
<400> 7686
AGCCTGATGG AAGTCGGTGC CCACAACCAG CCGGAGAGCT GGATAGCAGA 50
GTTGCTGGCG GCAAAGGACA GAACGCTGGC GGCAGCAACG GCAAAAGCGG 100
<210> 7687
<211> 100
<212> DNA
<213> Escherichia coli K-12 MG1655
<220> trua b2319
<222> 505-604
<223> trua b2319 U00096 complement(2432844..2433656)
<313> 7687: 557-576
<400> 7687
TGCCAGTCCC GAACCCCGTG GCGCAACGTT ATGCATATTA ATGTCACGCG 50
TCACGGTCCT TATGTGGTGG TAGATATTAA AGCGAATGCT TTTGTACATC 100

<210> 7688
<211> 100
<212> DNA
<213> Escherichia coli K-12 MG1655
<220> trub b3167
<222> 846-945
<223> trub b3167 U00096 complement(3309474..3310418)
<313> 7688: 914-933
<400> 7688
CGTCACGGAA GGTGAGAACG GCAAATTTAT CGGTATGGGC GAAATTGACG 50
ATGAAGGCCG CGTTGCGCCT CGTCGCCTGG TGGTTGAATA CCCGGCGTAA 100
<210> 7689
<211> 100
<212> DNA
<213> Escherichia coli K-12 MG1655
<220> trub b3167
<222> 824-923
<223> trub b3167 U00096 complement(3309474..3310418)
<313> 7689: 859-878
<400> 7689
CGCCACTGGA AGGACTGGTT CGCGTCACGG AAGGTGAGAA CGGCAAATTT 50
ATCGGTATGG GCGAAATTGA CGATGAAGGC CGCGTTGCGC CTCGTCGCCT 100
<210> 7690
<211> 100
<212> DNA
<213> Escherichia coli K-12 MG1655
<220> trub b3167
<222> 694-793
<223> trub b3167 U00096 complement(3309474..3310418)
<313> 7690: 747-766
<400> 7690
CCAGCCGCGG AGTTACTTGA TCCATTACTG ATGCCAATGG ACAGTCCAGC 50
TTCGGACTAT CCGGTGGTGA ATCTTCCGTT AACGTCTTCT GTTTACTTCA 100
<210> 7691
<211> 100
<212> DNA
<213> Escherichia coli K-12 MG1655
<220> sure b2745
<222> 663-762
<223> sure b2745 U00096 complement(2867536..2868297)
<313> 7691: 735-754
<400> 7691
TGTCTCCATC ACGCCGCTGC ATGTGGATTT AACTGCGCAT AGCGCGCAAG 50
ATGTGGTTTC AGACTGGTTA AACAGCGTGG GAGTTGGCAC GCAATGGTAA 100
<210> 7692
<211> 100
<212> DNA
<213> Escherichia coli K-12 MG1655
<220> sure b2745
<222> 600-699
<223> sure b2745 U00096 complement(2867536..2868297)
<313> 7692: 647-666
<400> 7692
CCCGCCGGGC GGTAAATGTG ATGCTGGTCC GGGGACCGAT TTTGCTGCGG 50
TAGATGAGGG CTATGTCTCC ATCACGCCGC TGCATGTGGA TTTAACTGCG 100
<210> 7693
<211> 100
<212> DNA
<213> Escherichia coli K-12 MG1655
<220> sure b2745
<222> 490-589
<223> sure b2745 U00096 complement(2867536..2868297)
<313> 7693: 546-565
<400> 7693
CCCTTGGATC AAATCAAAGG TATTCGCGTG ACGCGCTGCG GTACACGACA 50
TCCGGCAGAT CAGGTGATCC CGCAGCAAGA TCCGCGCGGC AATACGCTGT 100
<210> 7694
<211> 100
<212> DNA
<213> Escherichia coli K-12 MG1655
<220> ydca ydcA

<222> 75-174
<223> ydca ydcA U00096 1489701..1489874
<313> 7694: 130-149
<400> 7694
TTCTGGTTCG AAAGGGGGGA TCTCACACTG TACGGCAGGC GGCAAATTTG 50
TCTGTAATGA TGGTTCTATT AGTGCATCGA AAAAAACATG CACTAACTGA 100
<210> 7695
<211> 100
<212> DNA
<213> Escherichia coli K-12 MG1655
<220> ydca ydcA
<222> 27-126
<223> ydca ydcA U00096 1489701..1489874
<313> 7695: 59-78
<400> 7695
TATGGGAACG CTTGTTTCCT TTTATGCCGA TGCCGGGCGC AAACCCTGTT 50
CTGGTTCGAA AGGGGGGATC TCACACTGTA CGGCAGGCGG CAAATTTGTC 100
<210> 7696
<211> 100
<212> DNA
<213> Escherichia coli K-12 MG1655
<220> ydca ydcA
<222> 21-120
<223> ydca ydcA U00096 1489701..1489874
<313> 7696: 57-76
<400> 7696
TTTGTTTATG GGAACGCTTG TTTCCTTTTA TGCCGATGCC GGGCGCAAAC 50
CCTGTTCTGG TTCGAAAGGG GGGATCTCAC ACTGTACGGC AGGCGGCAAA 100
<210> 7697
<211> 100
<212> DNA
<213> Escherichia coli K-12 MG1655
<220> ydcc b1461
<222> 877-976
<223> ydcc b1461 U00096 1529840..1530976
<313> 7697: 916-935
<400> 7697
GAGAAGTTCG CCACAGCAAT CCGAAACCAC TGGCATGTGG AGAATAAGCT 50
GCACTGGCGT CTGGACGTGG TAATGAATGA AGACGACTGC AAAATAAGAA 100
<210> 7698
<211> 100
<212> DNA
<213> Escherichia coli K-12 MG1655
<220> ydcc b1461
<222> 847-946
<223> ydcc b1461 U00096 1529840..1530976
<313> 7698: 864-883
<400> 7698
TATTATATCA GTTCTGCTGA TTTAACCGCA GAGAAGTTCG CCACAGCAAT 50
CCGAAACCAC TGGCATGTGG AGAATAAGCT GCACTGGCGT CTGGACGTGG 100
<210> 7699
<211> 100
<212> DNA
<213> Escherichia coli K-12 MG1655
<220> ydcc b1461
<222> 631-730
<223> ydcc b1461 U00096 1529840..1530976
<313> 7699: 676-695
<400> 7699
CCGCTGAAAG AATTAAATAA TCCAGCGCAT GACAGTTACG CAATGAGTGA 50
AAAGAGTCAC GGCAGAGAAG AAATCCGTCT TCATATTGTT TGCGATGTCC 100
<210> 7700
<211> 100
<212> DNA
<213> Escherichia coli K-12 MG1655
<220> ydcd ydcD
<222> 336-435
<223> ydcd ydcD U00096 1527946..1528428
<313> 7700: 354-373
<400> 7700
CTGCGCTACA AAACAACGGG TGTATTTTAG TGATAAAAAC AAGAAGGCTA 50

GTTATAAAAT AATTAATTAT GGAGACTATT ATGATGTTGA TTATTATGAT 100
<210> 7701
<211> 100
<212> DNA
<213> Escherichia coli K-12 MG1655
<220> ydcd ydcD
<222> 303-402
<223> ydcd ydcD U00096 1527946..1528428
<313> 7701: 340-359
<400> 7701
AAAAAACATT AGTAAACTGG AGAATATGGA GTTCTGCGCT ACAAAACAAC 50
GGGTGTATTT TAGTGATAAA AACAAGAAGG CTAGTTATAA AATAATTAAT 100
<210> 7702
<211> 100
<212> DNA
<213> Escherichia coli K-12 MG1655
<220> ydcd ydcD
<222> 258-357
<223> ydcd ydcD U00096 1527946..1528428
<313> 7702: 308-327
<400> 7702
TATAAATCTT AATTTTTTCA ATGATATTCT TTATAGCGTG CGATTAAAAA 50
ACATTAGTAA ACTGGAGAAT ATGGAGTTCT GCGCTACAAA ACAACGGGTG 100
<210> 7703
<211> 100
<212> DNA
<213> Escherichia coli K-12 MG1655
<220> ydce ydcE
<222> 135-234
<223> ydce ydcE U00096 1531076..1531309
<313> 7703: 183-202
<400> 7703
GCAGATTCAA CCAGAATCTT GGCAAGCTAT CTGGGATGCC GAAATCGCGC 50
CCCAAATGGA GGCTTTGATA AAGAAACCTG GTTATAGCAT GAATGCTTAA 100
<210> 7704
<211> 100
<212> DNA
<213> Escherichia coli K-12 MG1655
<220> ydce ydcE
<222> 103-202
<223> ydce ydcE U00096 1531076..1531309
<313> 7704: 156-175
<400> 7704
AGTAAAGACA GTTCGATAAG CATTGCTCTA CAGCAGATTC AACCAGAATC 50
TTGGCAAGCT ATCTGGGATG CCGAAATCGC GCCCCAAATG GAGGCTTTGA 100
<210> 7705
<211> 100
<212> DNA
<213> Escherichia coli K-12 MG1655
<220> ydce ydcE
<222> 1-100
<223> ydce ydcE U00096 1531076..1531309
<313> 7705: 41-60
<400> 7705
ATGCCGCACA TCGACATTAA ATGTTTTCCG CGTGAACTGG ACGAACAACA 50
AAAAGCAGCA CTTGCTGCAG ATATTACCGA CGTTATTATT CGTCATCTGA 100
<210> 7706
<211> 100
<212> DNA
<213> Escherichia coli K-12 MG1655
<220> ydcf b1415
<222> 702-801
<223> ydcf b1415 U00096 1485259..1486059
<313> 7706: 782-801
<400> 7706
CGTTCACGTT GATTTTCCGG CAGAAGTCAT CCATGCATGG CAAACGCTGA 50
AACATGATGC GGTGCTCATC GAGGCGATGG AAAGTCGCTC GTTACGTTAA 100
<210> 7707
<211> 100
<212> DNA
<213> Escherichia coli K-12 MG1655

<220> ydcf b1415
<222> 667-766
<223> ydcf b1415 U00096 1485259..1486059
<313> 7707: 715-734
<400> 7707
AGCGATGGCT ACGGTCCCCG CGGGCGAGAT TTTATCGTTC ACGTTGATTT  50
TCCGGCAGAA GTCATCCATG CATGGCAAAC GCTGAAACAT GATGCGGTGC  100
<210> 7708
<211> 100
<212> DNA
<213> Escherichia coli K-12 MG1655
<220> ydcf b1415
<222> 624-723
<223> ydcf b1415 U00096 1485259..1486059
<313> 7708: 655-674
<400> 7708
TCTCTCACTA CTCACTGGCG AGCTGCCGCG TTTACGCGAT GATAGCGATG  50
GCTACGGTCC CCGCGGGCGA GATTTTATCG TTCACGTTGA TTTTCCGGCA  100
<210> 7709
<211> 100
<212> DNA
<213> Escherichia coli K-12 MG1655
<220> ydcg ydcG
<222> 1557-1656
<223> ydcg ydcG U00096 1494880..1496535
<313> 7709: 1613-1632
<400> 7709
ACGTTGTCAG GGGGACGCTA TCAGTGAAAC ATGGCTGTAT CAGTATTTCC  50
CGCCAGCGCC GGATAAACGT CAGTATGTTG ACGACCGCGT GATGAGTTAA  100
<210> 7710
<211> 100
<212> DNA
<213> Escherichia coli K-12 MG1655
<220> ydcg ydcG
<222> 1539-1638
<223> ydcg ydcG U00096 1494880..1496535
<313> 7710: 1571-1590
<400> 7710
CGATATGCGG ATGTATCTAC GTTGTCAGGG GGACGCTATC AGTGAAACAT  50
GGCTGTATCA GTATTTCCCG CCAGCGCCGG ATAAACGTCA GTATGTTGAC  100
<210> 7711
<211> 100
<212> DNA
<213> Escherichia coli K-12 MG1655
<220> ydcg ydcG
<222> 1488-1587
<223> ydcg ydcG U00096 1494880..1496535
<313> 7711: 1519-1538
<400> 7711
TTATCGTATT CAGTTTGACT GGTATCCGAC TTCGGACTCC ACTGATCCGG  50
TCGATATGCG GATGTATCTA CGTTGTCAGG GGGACGCTAT CAGTGAAACA  100
<210> 7712
<211> 100
<212> DNA
<213> Escherichia coli K-12 MG1655
<220> amya b1928
<222> 1389-1488
<223> amya b1928 U00096 2004179..2005666
<313> 7712: 1450-1469
<400> 7712
GAACCGGCAA GAGAGAGTAG TGACCGACGA AAACGGCGAA GCAACCTTCT  50
TTTGCAACGG CGGCAGCGTC AGCGTGTGGG TTATCGAAGA GGTGATTTAA  100
<210> 7713
<211> 100
<212> DNA
<213> Escherichia coli K-12 MG1655
<220> amya b1928
<222> 1389-1488
<223> amya b1928 U00096 2004179..2005666
<313> 7713: 1409-1428
<400> 7713

GAACCGGCAA GAGAGAGTAG TGACCGACGA AAACGGCGAA GCAACCTTCT 50
TTTGCAACGG CGGCAGCGTC AGCGTGTGGG TTATCGAAGA GGTGATTTAA 100
<210> 7714
<211> 100
<212> DNA
<213> Escherichia coli K-12 MG1655
<220> amya b1928
<222> 1309-1408
<223> amya b1928 U00096 2004179..2005666
<313> 7714: 1351-1370
<400> 7714
ATGTCGAACG GGGATGATGG CGAAAAAACC ATTCATCTGG GAGAGAATTA 50
CGGCAATAAA ACCTGGCGTG ATTTTCTGGG GAACCGGCAA GAGAGAGTAG 100
<210> 7715
<211> 100
<212> DNA
<213> Escherichia coli K-12 MG1655
<220> ydch b1427
<222> 64-163
<223> ydch b1427 U00096 1496732..1496899
<313> 7715: 109-128
<400> 7715
GGTCGAGGGT ACAATGCGGA AGTGGTCCGC ATGAAAAAAC AAAAGCTACA 50
GTTAAAAGAT GAGATGCTCA AAATCCTGCA GCAGGAGAGC GTCAAAGAGG 100
<210> 7716
<211> 100
<212> DNA
<213> Escherichia coli K-12 MG1655
<220> ydch b1427
<222> 55-154
<223> ydch b1427 U00096 1496732..1496899
<313> 7716: 93-112
<400> 7716
GGTTCCGACG GTCGAGGGTA CAATGCGGAA GTGGTCCGCA TGAAAAAACA 50
AAAGCTACAG TTAAAAGATG AGATGCTCAA AATCCTGCAG CAGGAGAGCG 100
<210> 7717
<211> 100
<212> DNA
<213> Escherichia coli K-12 MG1655
<220> ydch b1427
<222> 68-167
<223> ydch b1427 U00096 1496732..1496899
<313> 7717: 83-102
<400> 7717
GAGGGTACAA TGCGGAAGTG GTCCGCATGA AAAAACAAAA GCTACAGTTA 50
AAAGATGAGA TGCTCAAAAT CCTGCAGCAG GAGAGCGTCA AAGAGGTGTA 100
<210> 7718
<211> 100
<212> DNA
<213> Escherichia coli K-12 MG1655
<220> b2442 b2442
<222> 1197-1296
<223> b2442 b2442 U00096 2556791..2558086
<313> 7718: 1277-1296
<400> 7718
GGCTGCCTAC CTGAAGCAAC GGCGAGCAAT GATGCAGGAT TACGCCGACG 50
CTATTGATTC AATATTGGCA GGTAACGGCA ATCCTCTAGA ACCGGAATGA 100
<210> 7719
<211> 100
<212> DNA
<213> Escherichia coli K-12 MG1655
<220> b2442 b2442
<222> 1175-1274
<223> b2442 b2442 U00096 2556791..2558086
<313> 7719: 1213-1232
<400> 7719
GAATAAGGGG CGTTTATAAC CAGGCTGCCT ACCTGAAGCA ACGGCGAGCA 50
ATGATGCAGG ATTACGCCGA CGCTATTGAT TCAATATTGG CAGGTAACGG 100
<210> 7720
<211> 100
<212> DNA

<213> Escherichia coli K-12 MG1655
<220> b2442 b2442
<222> 1118-1217
<223> b2442 b2442 U00096 2556791..2558086
<313> 7720: 1161-1180
<400> 7720
AATCAGGCAG ATACTCGCCG GATTGGATTG AGATCCAACT AGCCCACGTC 50
CCGAAGGGAA TAAGGGGCGT TTATAACCAG GCTGCCTACC TGAAGCAACG 100
<210> 7721
<211> 100
<212> DNA
<213> Escherichia coli K-12 MG1655
<220> dead b3163
<222> 1721-1820
<223> dead b3163 U00096 complement(3303612..3305552)
<313> 7721: 1780-1799
<400> 7721
TGCAGTTACT GGGCGATGCA CAGCCGCATA CTGGCGGTGA GCGTCGTGGC 50
GGTGGTCGTG GTTTCGGTGG CGAACGTCGT GAAGGCGGTC GTAACTTCAG 100
<210> 7722
<211> 100
<212> DNA
<213> Escherichia coli K-12 MG1655
<220> dead b3163
<222> 1704-1803
<223> dead b3163 U00096 complement(3303612..3305552)
<313> 7722: 1735-1754
<400> 7722
CAACAAGCCG ATGAACATGC AGTTACTGGG CGATGCACAG CCGCATACTG 50
GCGGTGAGCG TCGTGGCGGT GGTCGTGGTT TCGGTGGCGA ACGTCGTGAA 100
<210> 7723
<211> 100
<212> DNA
<213> Escherichia coli K-12 MG1655
<220> dead b3163
<222> 1672-1771
<223> dead b3163 U00096 complement(3303612..3305552)
<313> 7723: 1698-1717
<400> 7723
GTGCTGCAAC ACTTTACGCG CACTCGCATT CTCAACAAGC CGATGAACAT 50
GCAGTTACTG GGCGATGCAC AGCCGCATAC TGGCGGTGAG CGTCGTGGCG 100
<210> 7724
<211> 100
<212> DNA
<213> Escherichia coli K-12 MG1655
<220> ydcn b1435
<222> 438-537
<223> ydcn b1435 U00096 1504196..1504732
<313> 7724: 512-531
<400> 7724
GGAAGGCGTT CGATTTGCTG CAGACGTCAC GCATATCTAT CGTAATGGTG 50
GGGAGCAAAC CGTACATTTT CATTCCCTCA TCCATTACCC GCGCAGTTAA 100
<210> 7725
<211> 100
<212> DNA
<213> Escherichia coli K-12 MG1655
<220> ydcn b1435
<222> 388-487
<223> ydcn b1435 U00096 1504196..1504732
<313> 7725: 433-452
<400> 7725
GGACAACTTG ATCTGTGCGT TGATGGGGAA TGGCAGACCC TTAATTGTGG 50
GGAAGGCGTT CGATTTGCTG CAGACGTCAC GCATATCTAT CGTAATGGTG 100
<210> 7726
<211> 100
<212> DNA
<213> Escherichia coli K-12 MG1655
<220> ydcn b1435
<222> 305-404
<223> ydcn b1435 U00096 1504196..1504732
<313> 7726: 364-383

<400> 7726
TCTCGATTCA GATGGCATCT GGTGCAATAA GCGAATCGAC GCCCCATGAG  50
AAGGGGGTAA TCGAACATGT CGTCGTCATT GATGGACAAC TTGATCTGTG  100
<210> 7727
<211> 100
<212> DNA
<213> Escherichia coli K-12 MG1655
<220> b2443 b2443
<222> 541-640
<223> b2443 b2443 U00096 2558277..2558918
<313> 7727: 580-599
<400> 7727
CACTTCAAAC TTGTTGAGCG TACTGAAGGC TATATACCAG GATCGGCAGA  50
AAGGGAGTGC AACGAATACC AGTTATTATT CAAGCCGTAC AACATGAAAT  100
<210> 7728
<211> 100
<212> DNA
<213> Escherichia coli K-12 MG1655
<220> b2443 b2443
<222> 510-609
<223> b2443 b2443 U00096 2558277..2558918
<313> 7728: 541-560
<400> 7728
GGTTAAAAAA TGCCTCGATC GTCTGGTCAA CCACTTCAAA CTTGTTGAGC  50
GTACTGAAGG CTATATACCA GGATCGGCAG AAAGGGAGTG CAACGAATAC  100
<210> 7729
<211> 100
<212> DNA
<213> Escherichia coli K-12 MG1655
<220> b2443 b2443
<222> 441-540
<223> b2443 b2443 U00096 2558277..2558918
<313> 7729: 491-510
<400> 7729
CTCACAGTTG GGATGGTGCA GGGTTGGTAG GGGAACAATC GACAAACGAG  50
CCAGGCTATC AAAAAACACG GTTAAAAAAT GCCTCGATCG TCTGGTCAAC  100
<210> 7730
<211> 100
<212> DNA
<213> Escherichia coli K-12 MG1655
<220> b2444 b2444
<222> 133-232
<223> b2444 b2444 U00096 2559388..2559633
<313> 7730: 168-187
<400> 7730
GCTTCCTTGT ACGACTGCAG CGAAGAAGAA AAGCTGTTTG TTAAGCGCCT  50
GAAGTTAATC AAAGCGGATA TCCACGCCCA GCTGAAGGCC TGTGATTGCG  100
<210> 7731
<211> 100
<212> DNA
<213> Escherichia coli K-12 MG1655
<220> b2444 b2444
<222> 138-237
<223> b2444 b2444 U00096 2559388..2559633
<313> 7731: 169-188
<400> 7731
CTTGTACGAC TGCAGCGAAG AAGAAAAGCT GTTTGTTAAG CGCCTGAAGT  50
TAATCAAAGC GGATATCCAC GCCCAGCTGA AGGCCTGTGA TTGCGATATT  100
<210> 7732
<211> 100
<212> DNA
<213> Escherichia coli K-12 MG1655
<220> b2444 b2444
<222> 78-177
<223> b2444 b2444 U00096 2559388..2559633
<313> 7732: 118-137
<400> 7732
AGTTTTGGGG ACCCTTTCCC TGCTTCTGGA GCAAAAGACG AACGGCAAAT  50
GCAGTGCTTC CTTGTACGAC TGCAGCGAAG AAGAAAAGCT GTTTGTTAAG  100
<210> 7733
<211> 100

<212> DNA
<213> Escherichia coli K-12 MG1655
<220> ydcp ydcP
<222> 1905-2004
<223> ydcp ydcP U00096 1504763..1506766
<313> 7733: 1968-1987
<400> 7733
AATCAAAAAT CACATACTGA AAATGCCGTT ACCGGGAAGC GTAGTGGCAT 50
CCGTAAGTCC GGATGAGCTG CTGAAAACAT TGCCGAAGCG AAAAGGGTAA 100
<210> 7734
<211> 100
<212> DNA
<213> Escherichia coli K-12 MG1655
<220> ydcp ydcP
<222> 1832-1931
<223> ydcp ydcP U00096 1504763..1506766
<313> 7734: 1891-1910
<400> 7734
AACTGGTTAA CGGCGATGAA GTATTAACGC TAAAGTTTGA TTGCCGCCCA 50
TGCGAGATGC ACGTCATTGG CAAAATCAAA AATCACATAC TGAAAATGCC 100
<210> 7735
<211> 100
<212> DNA
<213> Escherichia coli K-12 MG1655
<220> ydcp ydcP
<222> 1766-1865
<223> ydcp ydcP U00096 1504763..1506766
<313> 7735: 1801-1820
<400> 7735
TTGCCTTTAA TCTGTGCCCG AAACAGGCGA AAGGCAATAT CAAAAGCTGG 50
AAGGCGACGC CAATGCAACT GGTTAACGGC GATGAAGTAT TAACGCTAAA 100
<210> 7736
<211> 100
<212> DNA
<213> Escherichia coli K-12 MG1655
<220> b2445 b2445
<222> 285-384
<223> b2445 b2445 U00096 2559630..2560013
<313> 7736: 358-377
<400> 7736
CATGCGTGAA CCAGACTTTC AATGCCTGGA AATGGTTCAC GAAATCATAA 50
GAATTGCTAG AGATTTGGAA GCGCCAGTTG ACGCTCCGTT AGATTGCTAA 100
<210> 7737
<211> 100
<212> DNA
<213> Escherichia coli K-12 MG1655
<220> b2445 b2445
<222> 196-295
<223> b2445 b2445 U00096 2559630..2560013
<313> 7737: 216-235
<400> 7737
CAGATCTATG AGGCCGGAGG CGATGGAATA GAGCCACTCA ATTGTGGAAT 50
CGAATCAAGG AAGGTGGCAA TTGCTGCCCT GCTTCGCGTC ATGCGTGAAC 100
<210> 7738
<211> 100
<212> DNA
<213> Escherichia coli K-12 MG1655
<220> b2445 b2445
<222> 119-218
<223> b2445 b2445 U00096 2559630..2560013
<313> 7738: 153-172
<400> 7738
TCCGCGATCT AGCCAATGAT ATCAAAGAGC GTGGGTGTGT TGAGCTTGTC 50
CAGCCTGGTG GATTCGATGA GCTGGTTCAG ATCTATGAGG CCGGAGGCGA 100
<210> 7739
<211> 100
<212> DNA
<213> Escherichia coli K-12 MG1655
<220> b2446 b2446
<222> 316-415
<223> b2446 b2446 U00096 2560131..2560547

<313> 7739: 374-393
<400> 7739
CCGAAGCAGT TGCGCAAAGC AGAAGCAAAG GCAAAGGCAC TACTGGCAGC 50
AGCCAAGCTG AATAGTTTGA AACATTCACC ATTTGGAGAT GACAAACAAT 100
<210> 7740
<211> 100
<212> DNA
<213> Escherichia coli K-12 MG1655
<220> b2446 b2446
<222> 244-343
<223> b2446 b2446 U00096 2560131..2560547
<313> 7740: 304-323
<400> 7740
CTATGGTGCG CTGAAGCTGG CCGACGTGCC GTCGAGATAA TCGACGAGGT 50
TGATATAAAC GCAGAGGATG GGCCGAAGCA GTTGCGCAAA GCAGAAGCAA 100
<210> 7741
<211> 100
<212> DNA
<213> Escherichia coli K-12 MG1655
<220> b2446 b2446
<222> 172-271
<223> b2446 b2446 U00096 2560131..2560547
<313> 7741: 217-236
<400> 7741
AGCCACCGTC AGATGATGCG CTGGCAACTG GAGCATATGG ACTTCAGGGA 50
AGGGATAGCC TGGATGTCGT GGCTATGGTG CGCTGAAGCT GGCCGACGTG 100
<210> 7742
<211> 100
<212> DNA
<213> Escherichia coli K-12 MG1655
<220> b2447 b2447
<222> 495-594
<223> b2447 b2447 U00096 2560544..2561137
<313> 7742: 557-576
<400> 7742
ACTGTTCCAA CGCGATGACT TTTCTGAGGA TAGCCATGCC AACGATCCGA 50
AGTTGGTTGG TGATGATTAC GTCCCCCAGG CACCGGAGCA AATCAACTGA 100
<210> 7743
<211> 100
<212> DNA
<213> Escherichia coli K-12 MG1655
<220> b2447 b2447
<222> 492-591
<223> b2447 b2447 U00096 2560544..2561137
<313> 7743: 513-532
<400> 7743
CAAACTGTTC CAACGCGATG ACTTTTCTGA GGATAGCCAT GCCAACGATC 50
CGAAGTTGGT TGGTGATGAT TACGTCCCCC AGGCACCGGA GCAAATCAAC 100
<210> 7744
<211> 100
<212> DNA
<213> Escherichia coli K-12 MG1655
<220> b2447 b2447
<222> 392-491
<223> b2447 b2447 U00096 2560544..2561137
<313> 7744: 423-442
<400> 7744
AACGGTTTCA ATACTTCCAA ACGATGTACG ACATGGAACG GCAAATCAAC 50
GCAAACCTGC CGGTTGGCGG CCTTCCCAAC GTTGACACAG AGCGAACTGG 100
<210> 7745
<211> 100
<212> DNA
<213> Escherichia coli K-12 MG1655
<220> b2448 b2448
<222> 294-393
<223> b2448 b2448 U00096 2561597..2561989
<313> 7745: 359-378
<400> 7745
GAAAGAGAAT AGCGATGAAA AGATTAACCT ATTGAACAAG ATGGGGGATC 50
TGGCTGGTCG TATCGTTGCA GAGCAGTATT TAGGCATGAG TTTTGAGTAA 100
<210> 7746

<211> 100
<212> DNA
<213> Escherichia coli K-12 MG1655
<220> b2448 b2448
<222> 164-263
<223> b2448 b2448 U00096 2561597..2561989
<313> 7746: 209-228
<400> 7746
GTGACAGGCT TGCGAGGGTT GAGATCGAAT ATATAGCGAC ATTGTGCAAG 50
CCGGTGGCTT TGGTGATTAT TTACGACAGA TTTGACGATA TAGCGGCAAT 100
<210> 7747
<211> 100
<212> DNA
<213> Escherichia coli K-12 MG1655
<220> b2448 b2448
<222> 85-184
<223> b2448 b2448 U00096 2561597..2561989
<313> 7747: 145-164
<400> 7747
AGGCTGAACG GGGAGGATAA CGCAAGAATT GAATCAGTCG TTACTGATAA 50
CTGTGAAAAA ACAGCCGTAT TGGTAGGCGG TGACAGGCTT GCGAGGGTTG 100
<210> 7748
<211> 100
<212> DNA
<213> Escherichia coli K-12 MG1655
<220> b2449 b2449
<222> 274-373
<223> b2449 b2449 U00096 2562000..2562392
<313> 7748: 325-344
<400> 7748
TACCATTTCA GCAATGAAAC TGAGTTCAAA CTTAAAGCGG CAGCGGTCAG 50
AGAATGCGCC AACGTAGCAG AAAACAACGG CTTTGTTTTG GTAGGTAGAG 100
<210> 7749
<211> 100
<212> DNA
<213> Escherichia coli K-12 MG1655
<220> b2449 b2449
<222> 189-288
<223> b2449 b2449 U00096 2562000..2562392
<313> 7749: 243-262
<400> 7749
CGACTACGCA TGGAAACATG TAAAAGAAGA CAGGTTGCCA TGTCTGTTTG 50
CCGCCGGGGT TGTTGCTGAT AACATCCTGG TAAAATACCA TTTCAGCAAT 100
<210> 7750
<211> 100
<212> DNA
<213> Escherichia coli K-12 MG1655
<220> b2449 b2449
<222> 131-230
<223> b2449 b2449 U00096 2562000..2562392
<313> 7750: 151-170
<400> 7750
ACTATGGCGA ACCAGGTGAA GGGGCGAAAA TCATGAATGC CGCAAAAATA 50
ATGGGTCGCG ACTACGCATG GAAACATGTA AAAGAAGACA GGTTGCCATG 100
<210> 7751
<211> 100
<212> DNA
<213> Escherichia coli K-12 MG1655
<220> mana b1614
<222> 1040-1139
<223> mana b1614 U00096 1686600..1687775
<313> 7751: 1086-1105
<400> 7751
AAGGCGATGC AACGTTGTGG AAAGGTTCTC AGCAGTTACA GCTTAAACCG 50
GGTGAATCAG CGTTTATTGC CGCCAACGAA TCACCGGTGA CTGTCAAAGG 100
<210> 7752
<211> 100
<212> DNA
<213> Escherichia coli K-12 MG1655
<220> mana b1614
<222> 943-1042

<223> mana b1614 U00096 1686600..1687775
<313> 7752: 998-1017
<400> 7752
CCGATTCCAG TGGATGATTT TGCCTTCTCG CTGCATGACC TTAGTGATAA 50
AGAAACCACC ATTAGCCAGC AGAGTGCCGC CATTTTGTTC TGCGTCGAAG 100
<210> 7753
<211> 100
<212> DNA
<213> Escherichia coli K-12 MG1655
<220> mana b1614
<222> 934-1033
<223> mana b1614 U00096 1686600..1687775
<313> 7753: 967-986
<400> 7753
CTGGACTTCC CGATTCCAGT GGATGATTTT GCCTTCTCGC TGCATGACCT 50
TAGTGATAAA GAAACCACCA TTAGCCAGCA GAGTGCCGCC ATTTTGTTCT 100
<210> 7754
<211> 100
<212> DNA
<213> Escherichia coli K-12 MG1655
<220> ydda yddA
<222> 1587-1686
<223> ydda yddA U00096 complement(1575681..1577366)
<313> 7754: 1650-1669
<400> 7754
GCGTGAAAAA CTACCCACAA GCGGCGTCAT TATGGTTACA CATCAACCCG 50
GTGTCTGGAA CCTGGCCGAT GATATTTGTG ACATTAGCGC GGTTTTATAA 100
<210> 7755
<211> 100
<212> DNA
<213> Escherichia coli K-12 MG1655
<220> ydda yddA
<222> 1584-1683
<223> ydda yddA U00096 complement(1575681..1577366)
<313> 7755: 1620-1639
<400> 7755
AGTGCGTGAA AAACTACCCA CAAGCGGCGT CATTATGGTT ACACATCAAC 50
CCGGTGTCTG GAACCTGGCC GATGATATTT GTGACATTAG CGCGGTTTTA 100
<210> 7756
<211> 100
<212> DNA
<213> Escherichia coli K-12 MG1655
<220> ydda yddA
<222> 1536-1635
<223> ydda yddA U00096 complement(1575681..1577366)
<313> 7756: 1578-1597
<400> 7756
AACTACCTCT CATCTTGAGG AACAAGAGGC TATCCGCTTA CTGCGTTTAG 50
TGCGTGAAAA ACTACCCACA AGCGGCGTCA TTATGGTTAC ACATCAACCC 100
<210> 7757
<211> 100
<212> DNA
<213> Escherichia coli K-12 MG1655
<220> yddb b1496
<222> 2247-2346
<223> yddb b1496 U00096 complement(1573271..1575643)
<313> 7757: 2278-2297
<400> 7757
CCTGACGATC AGTGCCGATA TTCTCAATGT ACTGGATAGC AAAACCGCTG 50
TTGATACAAC GAACACCGGT GTGGCGACCT ACGCCAGTGG CCGTACTTTC 100
<210> 7758
<211> 100
<212> DNA
<213> Escherichia coli K-12 MG1655
<220> yddb b1496
<222> 2190-2289
<223> yddb b1496 U00096 complement(1573271..1575643)
<313> 7758: 2251-2270
<400> 7758
CAGCCTGACC TGGGACACCC GCTTGTCCTG GACGCCACAA TTTCTGCAAC 50
AACAAAACCT GACGATCAGT GCCGATATTC TCAATGTACT GGATAGCAAA 100

<210> 7759
<211> 100
<212> DNA
<213> Escherichia coli K-12 MG1655
<220> yddb b1496
<222> 2159-2258
<223> yddb b1496 U00096 complement(1573271..1575643)
<313> 7759: 2196-2215
<400> 7759
ACAAGCAGTA TGTTGACGAA AAACTGGATA GCAGCCTGAC CTGGGACACC  50
CGCTTGTCCT GGACGCCACA ATTTCTGCAA CAACAAAACC TGACGATCAG  100
<210> 7760
<211> 100
<212> DNA
<213> Escherichia coli K-12 MG1655
<220> ydde b1465
<222> 795-894
<223> ydde b1465 U00096 complement(1532989..1533882)
<313> 7760: 866-885
<400> 7760
TGAAGTGACG GTGACAATTC GTGATAACCA ACCGGAAAAA GTCACCATTT  50
CAGGCACAGC GGTGATTTTG TTTCATGCCG AATGGGCAAT TGAACTCTGA  100
<210> 7761
<211> 100
<212> DNA
<213> Escherichia coli K-12 MG1655
<220> ydde b1465
<222> 774-873
<223> ydde b1465 U00096 complement(1532989..1533882)
<313> 7761: 833-852
<400> 7761
ATTGGGGCGT GACGGTATGA TTGAAGTGAC GGTGACAATT CGTGATAACC  50
AACCGGAAAA AGTCACCATT TCAGGCACAG CGGTGATTTT GTTTCATGCC  100
<210> 7762
<211> 100
<212> DNA
<213> Escherichia coli K-12 MG1655
<220> ydde b1465
<222> 748-847
<223> ydde b1465 U00096 complement(1532989..1533882)
<313> 7762: 779-798
<400> 7762
CGTGTTAAAG GCCATCAGGG GCGCGCATTG GGGCGTGACG GTATGATTGA  50
AGTGACGGTG ACAATTCGTG ATAACCAACC GGAAAAAGTC ACCATTTCAG  100
<210> 7763
<211> 100
<212> DNA
<213> Escherichia coli K-12 MG1655
<220> yddg b1474
<222> 771-870
<223> yddg b1474 U00096 complement(1544312..1545193)
<313> 7763: 793-812
<400> 7763
AGCGCTTGCA GCCGTGCTGC TCAGCGCCCC GCTGTCGTTC TCGTTCTGGC  50
AAGGCGCGCT GATGGTCTGC GGCGGTTCCC TGCTCTGCTG GCTGGCGACA  100
<210> 7764
<211> 100
<212> DNA
<213> Escherichia coli K-12 MG1655
<220> yddg b1474
<222> 687-786
<223> yddg b1474 U00096 complement(1544312..1545193)
<313> 7764: 746-765
<400> 7764
TTATGCTGCA TGGAATGTCG GTATATTGCA TGGCAATGTC ACCATTATGG  50
CGGTAGGTTC GTATTTTACG CCTGTACTTT CCTCAGCGCT TGCAGCCGTG  100
<210> 7765
<211> 100
<212> DNA
<213> Escherichia coli K-12 MG1655
<220> yddg b1474

<222> 586-685
<223> yddg b1474 U00096 complement(1544312..1545193)
<313> 7765: 618-637
<400> 7765
AGTCTGTGGG TTTACTATTT TCTTACGCCA CAACCAGAAA TGATATTTAG 50
CACGCCCGTC ATGATTAAAC TCATCTCTGC GGCATTTACC TTAGGATTTG 100
<210> 7766
<211> 100
<212> DNA
<213> Escherichia coli K-12 MG1655
<220> b1720 b1720
<222> 355-454
<223> b1720 b1720 U00096 1801118..1801591
<313> 7766: 394-413
<400> 7766
ATACTGGATT ATATTGAACA GGAACAGTTA ACTGCTGAAG ATCGTAACCA 50
TTGTATGAAA CTGTCTAAAA AAATCCATAG AGAAAAAAAC TATACACCCA 100
<210> 7767
<211> 100
<212> DNA
<213> Escherichia coli K-12 MG1655
<220> b1720 b1720
<222> 332-431
<223> b1720 b1720 U00096 1801118..1801591
<313> 7767: 349-368
<400> 7767
TTAAAGCTAA CCATTTGCGA AGCATACTGG ATTATATTGA ACAGGAACAG 50
TTAACTGCTG AAGATCGTAA CCATTGTATG AAACTGTCTA AAAAAATCCA 100
<210> 7768
<211> 100
<212> DNA
<213> Escherichia coli K-12 MG1655
<220> b1720 b1720
<222> 281-380
<223> b1720 b1720 U00096 1801118..1801591
<313> 7768: 327-346
<400> 7768
CATTACACTA TAAAAAAGAA ATCATTTCCT TATTACTGGA AACCACGCCT 50
ATTAAAGCTA ACCATTTGCG AAGCATACTG GATTATATTG AACAGGAACA 100
<210> 7769
<211> 100
<212> DNA
<213> Escherichia coli K-12 MG1655
<220> b1721 b1721
<222> 1160-1259
<223> b1721 b1721 U00096 1801602..1803017
<313> 7769: 1194-1213
<400> 7769
ACATCGCCAT GAGCAAGGGT AATGAAGACG TCGTGTTATC TTATATATCG 50
ACGCTGGGTG CTTTTGCAAA AAAACATTCT TTTAGTCAAC ATCAGTTATT 100
<210> 7770
<211> 100
<212> DNA
<213> Escherichia coli K-12 MG1655
<220> b1721 b1721
<222> 1122-1221
<223> b1721 b1721 U00096 1801602..1803017
<313> 7770: 1161-1180
<400> 7770
ATTAAAAGGA GCTACAGCAC AGGGAACCCC TGCATTATAC ATCGCCATGA 50
GCAAGGGTAA TGAAGACGTC GTGTTATCTT ATATATCGAC GCTGGGTGCT 100
<210> 7771
<211> 100
<212> DNA
<213> Escherichia coli K-12 MG1655
<220> b1721 b1721
<222> 1033-1132
<223> b1721 b1721 U00096 1801602..1803017
<313> 7771: 1058-1077
<400> 7771
AATCACCCTT TGTGTGTCAC ACGGTTCCTC TCTAAAATTA ATGGCATCGC 50

CTTTAAATAC AAGTTGAGCA AAGCTAACAT CATGGATTTA TTAAAAGGAG 100
<210> 7772
<211> 100
<212> DNA
<213> Escherichia coli K-12 MG1655
<220> b2450 b2450
<222> 741-840
<223> b2450 b2450 U00096 2562513..2563352
<313> 7772: 815-834
<400> 7772
CAAGAAAGAC GAAAACGGCA ACCAGATCGG CAAAGGCAAG AAAGGTGACA 50
AGCTGATAAC TTTGCTTCCC AACTGGATTG ATAAGCTGGG AGACGAATAA 100
<210> 7773
<211> 100
<212> DNA
<213> Escherichia coli K-12 MG1655
<220> b2450 b2450
<222> 738-837
<223> b2450 b2450 U00096 2562513..2563352
<313> 7773: 772-791
<400> 7773
ACACAAGAAA GACGAAAACG GCAACCAGAT CGGCAAAGGC AAGAAAGGTG 50
ACAAGCTGAT AACTTTGCTT CCCAACTGGA TTGATAAGCT GGGAGACGAA 100
<210> 7774
<211> 100
<212> DNA
<213> Escherichia coli K-12 MG1655
<220> b2450 b2450
<222> 658-757
<223> b2450 b2450 U00096 2562513..2563352
<313> 7774: 697-716
<400> 7774
CCAATCAGTA ACGGCGCGTT TTATCGACTG ATTGAGCAGG CCAAAGGGAG 50
AGGATTGATT AGCGTTGAAC AGGAAATCAA ACACAAGAAA GACGAAAACG 100
<210> 7775
<211> 100
<212> DNA
<213> Escherichia coli K-12 MG1655
<220> b1722 b1723
<222> 660-759
<223> b1722 b1723 U00096 complement(1803349..1804107)
<313> 7775: 725-744
<400> 7775
GAAGGTGGCT TACAACGTCA CATGGAACTC TGAACCTCCA GAGTCTGCAC 50
CGGAACATAC CGACCGTCGG ACAACCTTAT CGCTGGGCTA CAGCATGTAA 100
<210> 7776
<211> 100
<212> DNA
<213> Escherichia coli K-12 MG1655
<220> b1722 b1723
<222> 634-733
<223> b1722 b1723 U00096 complement(1803349..1804107)
<313> 7776: 681-700
<400> 7776
GTTGCTATTA ATGAACATTT TGGACTGAAG GTGGCTTACA ACGTCACATG 50
GAACTCTGAA CCTCCAGAGT CTGCACCGGA ACATACCGAC CGTCGGACAA 100
<210> 7777
<211> 100
<212> DNA
<213> Escherichia coli K-12 MG1655
<220> b1722 b1723
<222> 562-661
<223> b1722 b1723 U00096 complement(1803349..1804107)
<313> 7777: 614-633
<400> 7777
GCTAAATTTA CGCAGGGTGT TTCGGTCTTT GGCGCGGAAG ACACAACGCT 50
GAACTCCGAA AGCGCATTAA ATGTTGCTAT TAATGAACAT TTTGGACTGA 100
<210> 7778
<211> 100
<212> DNA
<213> Escherichia coli K-12 MG1655

<220> b2451 b2452
<222> 1281-1380
<223> b2451 b2452 U00096 complement(2563501..2564904)
<313> 7778: 1338-1357
<400> 7778
ACTCCCGTTG GCAGTCATTG ACGAAGTGAT TGTCCGCGCG GGGGACTATA 50
TCGACATTGG TACGCCTCTT TTTGGCGGAT CGGTTGTGCC GGTGACGGTG 100
<210> 7779
<211> 100
<212> DNA
<213> Escherichia coli K-12 MG1655
<220> b2451 b2452
<222> 1239-1338
<223> b2451 b2452 U00096 complement(2563501..2564904)
<313> 7779: 1277-1296
<400> 7779
TGGTAAAGCT CTGGGCATGT TGTTGCGCCC ACAGCTACAA CAACTCCCGT 50
TGGCAGTCAT TGACGAAGTG ATTGTCCGCG CGGGGGACTA TATCGACATT 100
<210> 7780
<211> 100
<212> DNA
<213> Escherichia coli K-12 MG1655
<220> b2451 b2452
<222> 1200-1299
<223> b2451 b2452 U00096 complement(2563501..2564904)
<313> 7780: 1246-1265
<400> 7780
GAATCCGCAT CCCCTGCTGG TGGTGGCCGG GCAGGACTTT GGTAAAGCTC 50
TGGGCATGTT GTTGCGCCCA CAGCTACAAC AACTCCCGTT GGCAGTCATT 100
<210> 7781
<211> 100
<212> DNA
<213> Escherichia coli K-12 MG1655
<220> yddm b1478
<222> 264-363
<223> yddm b1478 U00096 complement(1550422..1550784)
<313> 7781: 307-326
<400> 7781
TTCGCCGCAA ATGTGGCTGA ATCTGCAAAA CGCCTGGAGT CTGGCAGAGG 50
CAGAAAAAAC AGTGGATGTG TCGCGATTAC GCCGTCTGGT TACGCAATAA 100
<210> 7782
<211> 100
<212> DNA
<213> Escherichia coli K-12 MG1655
<220> yddm b1478
<222> 197-296
<223> yddm b1478 U00096 complement(1550422..1550784)
<313> 7782: 240-259
<400> 7782
GATTGCTGAC AGGAAAAGCA GCTTTGACGC CAGAAATGGC GATTAAACTT 50
TCCGTGGTGA TCGGCAGTTC GCCGCAAATG TGGCTGAATC TGCAAAACGC 100
<210> 7783
<211> 100
<212> DNA
<213> Escherichia coli K-12 MG1655
<220> yddm b1478
<222> 174-273
<223> yddm b1478 U00096 complement(1550422..1550784)
<313> 7783: 201-220
<400> 7783
AATTGCTCCC TCAACGGCAA GCCGATTGCT GACAGGAAAA GCAGCTTTGA 50
CGCCAGAAAT GGCGATTAAA CTTTCCGTGG TGATCGGCAG TTCGCCGCAA 100
<210> 7784
<211> 100
<212> DNA
<213> Escherichia coli K-12 MG1655
<220> b1724 b1724
<222> 192-291
<223> b1724 b1724 U00096 1805424..1805714
<313> 7784: 264-283
<400> 7784

AATTAGAGAA CTGGTTTCGG GGCTTACCCA AAGTGCAACC GGCAAAGATC 50
CTGAAATCAC CATCACGACC TGGGAGGAAT GGAATAGCAA CAGAAAATAA 100
<210> 7785
<211> 100
<212> DNA
<213> Escherichia coli K-12 MG1655
<220> b1724 b1724
<222> 182-281
<223> b1724 b1724 U00096 1805424..1805714
<313> 7785: 229-248
<400> 7785
GTGAAGAAGA AATTAGAGAA CTGGTTTCGG GGCTTACCCA AAGTGCAACC 50
GGCAAAGATC CTGAAATCAC CATCACGACC TGGGAGGAAT GGAATAGCAA 100
<210> 7786
<211> 100
<212> DNA
<213> Escherichia coli K-12 MG1655
<220> b1724 b1724
<222> 86-185
<223> b1724 b1724 U00096 1805424..1805714
<313> 7786: 119-138
<400> 7786
ACCTGACTCG CGACGGTTTT TTGCTCACTA TGACGGATGA TGAGGGAAAT 50
ATCCATGAGC TGGGAACTAA CACTTTTGGA CTTATCAGTA CCCAAAGTGA 100
<210> 7787
<211> 100
<212> DNA
<213> Escherichia coli K-12 MG1655
<220> b1725 b1725
<222> 762-861
<223> b1725 b1725 U00096 1805820..1806680
<313> 7787: 817-836
<400> 7787
GGTTTACCAA CTCTACACGC TGTTAAATCG TGCAAGGTTA TTTGGCGGTC 50
AGCATTTGGT TATTGCTCAG CAGTCATTGG ATAGATTATT AGCAGCATGA 100
<210> 7788
<211> 100
<212> DNA
<213> Escherichia coli K-12 MG1655
<220> b1725 b1725
<222> 711-810
<223> b1725 b1725 U00096 1805820..1806680
<313> 7788: 736-755
<400> 7788
CGGCTATCAG TCAGTATCCC CGCTACCTGC CGATTTCCTT GAACGTCAAC 50
CGGTTTACCA ACTCTACACG CTGTTAAATC GTGCAAGGTT ATTTGGCGGT 100
<210> 7789
<211> 100
<212> DNA
<213> Escherichia coli K-12 MG1655
<220> b1725 b1725
<222> 654-753
<223> b1725 b1725 U00096 1805820..1806680
<313> 7789: 683-702
<400> 7789
AGAGTGCGAC CTGGCGATGT TACCGCTGCA TACTGAACAG CCGCCACAAA 50
TCTATGACGG CTATCAGTCA GTATCCCCGC TACCTGCCGA TTTCCTTGAA 100
<210> 7790
<211> 100
<212> DNA
<213> Escherichia coli K-12 MG1655
<220> b1726 b1727
<222> 386-485
<223> b1726 b1727 U00096 complement(1806721..1807257)
<313> 7790: 430-449
<400> 7790
AAGGGGAAGA GGGGCTAACG CGTGAACAAA TTGAGTCCCG TATTGTTGTT 50
CCGCACCATA CTATTTTCCT GCAGTTTTTC TCACTGTATA TCCTGCCGGT 100
<210> 7791
<211> 100
<212> DNA

<213> Escherichia coli K-12 MG1655
<220> b1726 b1727
<222> 220-319
<223> b1726 b1727 U00096 complement(1806721..1807257)
<313> 7791: 268-287
<400> 7791
AACTCCCCGA CGCCCAATTT TAATGGTGGC GTGAACGTTA TGTTCTGGGT 50
GATTTTCATC CTGATTTTTG TCGGACTGGC GTTGCAGGAT TCCGGTGCCC 100
<210> 7792
<211> 100
<212> DNA
<213> Escherichia coli K-12 MG1655
<220> b1726 b1727
<222> 182-281
<223> b1726 b1727 U00096 complement(1806721..1807257)
<313> 7792: 227-246
<400> 7792
TGCAGGCGAA TACGCCTTTT TTGAATCTAT TCTGGTATAA CTCCCCGACG 50
CCCAATTTTA ATGGTGGCGT GAACGTTATG TTCTGGGTGA TTTTCATCCT 100
<210> 7793
<211> 100
<212> DNA
<213> Escherichia coli K-12 MG1655
<220> b1728 b1728
<222> 504-603
<223> b1728 b1728 U00096 1808223..1808825
<313> 7793: 567-586
<400> 7793
TTCATTACCC GAGAACAGCG CTGTTCGTTG GTCATCGCAA ATGATCAATA 50
CCTTGCAGAT CCAGTTTCAT CGGCTTATTA AGCATCAGGT TGAATACTAA 100
<210> 7794
<211> 100
<212> DNA
<213> Escherichia coli K-12 MG1655
<220> b1728 b1728
<222> 387-486
<223> b1728 b1728 U00096 1808223..1808825
<313> 7794: 423-442
<400> 7794
CCTGCTCTGG CCATGCCGCT GGCGTTTCCG CTTGCCTATC CTGGTTCCCC 50
AAAAGGGCAA CCAACTGGAA CGTTTTATCT GCATGGCATT ATTTGTCTGG 100
<210> 7795
<211> 100
<212> DNA
<213> Escherichia coli K-12 MG1655
<220> b1728 b1728
<222> 298-397
<223> b1728 b1728 U00096 1808223..1808825
<313> 7795: 344-363
<400> 7795
TGGTTCATTC CGGCTGATGC GCTACAAGGA ATGGTGCTGG GTTATTTGAG 50
CCACATACTT GCCGATATGC TGACACCCGC CGGTGTTCCC CTGCTCTGGC 100
<210> 7796
<211> 100
<212> DNA
<213> Escherichia coli K-12 MG1655
<220> manx b1818
<222> 873-972
<223> manx b1818 U00096 1900072..1901043
<313> 7796: 926-945
<400> 7796
CAAGAAACTG AATGCGCGCG GTATTGAGCT GGAAGTCCGT AAGGTTTCCA 50
CCGATCCGAA ACTGAAAATG ATGGATCTGA TCAGCAAAAT CGATAAGTAA 100
<210> 7797
<211> 100
<212> DNA
<213> Escherichia coli K-12 MG1655
<220> manx b1818
<222> 799-898
<223> manx b1818 U00096 1900072..1901043
<313> 7797: 829-848

<400> 7797
GGTATGGCAT TCCGTCAGGG TAAAACCCAG GTGAATAACG CGGTTTCGGT 50
TGATGAAAAA GATATCGAGG CGTTCAAGAA ACTGAATGCG CGCGGTATTG 100
<210> 7798
<211> 100
<212> DNA
<213> Escherichia coli K-12 MG1655
<220> manx b1818
<222> 728-827
<223> manx b1818 U00096 1900072..1901043
<313> 7798: 762-781
<400> 7798
TATTTACCAA CCCAACAGAT GTAGAGCGTC TCGTTGAAGG CGGCGTGAAA 50
ATCACCTCTG TTAACGTCGG TGGTATGGCA TTCCGTCAGG GTAAAACCCA 100
<210> 7799
<211> 100
<212> DNA
<213> Escherichia coli K-12 MG1655
<220> b1729 b1729
<222> 1293-1392
<223> b1729 b1729 U00096 1808958..1810349
<313> 7799: 1365-1384
<400> 7799
TGGCTCGATG ACAGCTGGCA CGCTGACCAG CCAGTGGCTG AAGCAAACCG 50
ATAAAGCCAT TCTGGATAGC GAAGACGACG CCGAACTGGC ACACCATTAA 100
<210> 7800
<211> 100
<212> DNA
<213> Escherichia coli K-12 MG1655
<220> b1729 b1729
<222> 1214-1313
<223> b1729 b1729 U00096 1808958..1810349
<313> 7800: 1238-1257
<400> 7800
GCCTGCCAGT AACCCTGGTG GCGCTGTTAA TCTCCGTTGA ACCGCTTATC 50
GACATGGGCC GTACGGCGTT AAACGTTAGT GGCTCGATGA CAGCTGGCAC 100
<210> 7801
<211> 100
<212> DNA
<213> Escherichia coli K-12 MG1655
<220> b1729 b1729
<222> 1142-1241
<223> b1729 b1729 U00096 1808958..1810349
<313> 7801: 1195-1214
<400> 7801
TTAGTTCCGC AGGCGTTGCC GGTGTCGGTG GTGGTGCAAC TTTCGCCGCA 50
CTGATTGTAC TGCCTGCGAT GGGCCTGCCA GTAACCCTGG TGGCGCTGTT 100
<210> 7802
<211> 100
<212> DNA
<213> Escherichia coli K-12 MG1655
<220> many b1819
<222> 695-794
<223> many b1819 U00096 1901106..1901906
<313> 7802: 732-751
<400> 7802
CTGTTATGGC AGTGCTCTAC ATCCAACTTA GCCCGAAATA CAACCGCGTA 50
GCCGGTGCGC CTGCTCAGGC AGCTGGTAAC AACGATCTCG ATAACGAACT 100
<210> 7803
<211> 100
<212> DNA
<213> Escherichia coli K-12 MG1655
<220> many b1819
<222> 611-710
<223> many b1819 U00096 1901106..1901906
<313> 7803: 663-682
<400> 7803
GCTACCTGAT GCCGTTCTTC TACCTCGGCT TCGTAACCGC AGCATTCACC 50
AACTTTAACC TGGTTGCTCT GGGTGTGATT GGTACTGTTA TGGCAGTGCT 100
<210> 7804
<211> 100

<212> DNA
<213> Escherichia coli K-12 MG1655
<220> many b1819
<222> 579-678
<223> many b1819 U00096 1901106..1901906
<313> 7804: 633-652
<400> 7804
TTATGCGATG GTTATCAACA TGATGCGTGC TGGCTACCTG ATGCCGTTCT 50
TCTACCTCGG CTTCGTAACC GCAGCATTCA CCAACTTTAA CCTGGTTGCT 100
<210> 7805
<211> 100
<212> DNA
<213> Escherichia coli K-12 MG1655
<220> b2459 b2459
<222> 705-804
<223> b2459 b2459 U00096 complement(2571522..2572325)
<313> 7805: 770-789
<400> 7805
CCGCCTTTCG AGCACGGTCT ACGTGATGAT GATCCTGAGC GTAACGAAGC 50
AGCCGCTAAC GGTGAAGCAG ATCCAACAAC GACTGGGGGA AACGCAATGA 100
<210> 7806
<211> 100
<212> DNA
<213> Escherichia coli K-12 MG1655
<220> b2459 b2459
<222> 701-800
<223> b2459 b2459 U00096 complement(2571522..2572325)
<313> 7806: 742-761
<400> 7806
TGAACCGCCT TTCGAGCACG GTCTACGTGA TGATGATCCT GAGCGTAACG 50
AAGCAGCCGC TAACGGTGAA GCAGATCCAA CAACGACTGG GGGAAACGCA 100
<210> 7807
<211> 100
<212> DNA
<213> Escherichia coli K-12 MG1655
<220> b2459 b2459
<222> 656-755
<223> b2459 b2459 U00096 complement(2571522..2572325)
<313> 7807: 704-723
<400> 7807
TCACCCGCAG CTTTGAAGTA TTACGCCCGG ACATTTTGCA GGCGCTGAAC 50
CGCCTTTCGA GCACGGTCTA CGTGATGATG ATCCTGAGCG TAACGAAGCA 100
<210> 7808
<211> 100
<212> DNA
<213> Escherichia coli K-12 MG1655
<220> manz manZ
<222> 762-861
<223> manz manZ U00096 1901910..1902770
<313> 7808: 798-817
<400> 7808
TATGTGGCTA CTGCGCAAAA AAGTTAACCC GCTGTGGATC ATCGTTGGCT 50
TCTTCGTCAT CGGTATCGCT GGTTACGCTT GCGGCCTGCT GGGACTGTAA 100
<210> 7809
<211> 100
<212> DNA
<213> Escherichia coli K-12 MG1655
<220> manz manZ
<222> 626-725
<223> manz manZ U00096 1901910..1902770
<313> 7809: 687-706
<400> 7809
AGTGGACACA TGTCAACATC CCGCTGGTTG TCTCTCGCAT TACTGACCAG 50
ACGGGCAAAG AACACGTTAC TACTGTCCAG ACTATTCTGG ACCAGTTAAT 100
<210> 7810
<211> 100
<212> DNA
<213> Escherichia coli K-12 MG1655
<220> manz manZ
<222> 587-686
<223> manz manZ U00096 1901910..1902770

<313> 7810: 637-656
<400> 7810
CTATCCTCGG CCTGTTTGTC ATGGGGGCAT TGGTTAACAA GTGGACACAT 50
GTCAACATCC CGCTGGTTGT CTCTCGCATT ACTGACCAGA CGGGCAAAGA 100
<210> 7811
<211> 100
<212> DNA
<213> Escherichia coli K-12 MG1655
<220> maoc b1388
<222> 1929-2028
<223> maoc b1388 U00096 complement(1449621..1451666)
<313> 7811: 1963-1982
<400> 7811
AAAACCAACA GGTGTGGTGG AATGGGCTGT AGAGGTATTC AATCAGCATC 50
AAACCCCGGT GGCGCTGTAT TCAATTCTGA CGCTGGTGGC CAGGCAGCAC 100
<210> 7812
<211> 100
<212> DNA
<213> Escherichia coli K-12 MG1655
<220> maoc b1388
<222> 1899-1998
<223> maoc b1388 U00096 complement(1449621..1451666)
<313> 7812: 1930-1949
<400> 7812
GACGCTGAAA AAACAGCGTA GCGCAGAAGA AAAACCAACA GGTGTGGTGG 50
AATGGGCTGT AGAGGTATTC AATCAGCATC AAACCCCGGT GGCGCTGTAT 100
<210> 7813
<211> 100
<212> DNA
<213> Escherichia coli K-12 MG1655
<220> maoc b1388
<222> 1827-1926
<223> maoc b1388 U00096 complement(1449621..1451666)
<313> 7813: 1851-1870
<400> 7813
GGAAAGCTTG CGTTTTATCG AACCCGTAAA GCCAGGCGAT ACCATCCAGG 50
TGCGTCTCAC CTGTAAGCGC AAGACGCTGA AAAAACAGCG TAGCGCAGAA 100
<210> 7814
<211> 100
<212> DNA
<213> Escherichia coli K-12 MG1655
<220> ydea b1529
<222> 998-1097
<223> ydea b1529 U00096 1615052..1616242
<313> 7814: 1054-1073
<400> 7814
TCGCGATGGC GCTATTCTCC GGCATATTTA ATATTGGAAT CGGGGCGGGT 50
GCGTTGGTAG GTAATCAGGT GAGTTTGCAC TGGTCAATGT CGATGATTGG 100
<210> 7815
<211> 100
<212> DNA
<213> Escherichia coli K-12 MG1655
<220> ydea b1529
<222> 771-870
<223> ydea b1529 U00096 1615052..1616242
<313> 7815: 814-833
<400> 7815
GGGCATTATT GGCAGCGTGA TTTTCGGTAA ACTGGGTAAT CAGTATGCGT 50
CTGCGTTGGT GAGTACGGCG ATTGCGCTGT TGCTGGTGTG CCTGGCATTG 100
<210> 7816
<211> 100
<212> DNA
<213> Escherichia coli K-12 MG1655
<220> ydea b1529
<222> 729-828
<223> ydea b1529 U00096 1615052..1616242
<313> 7816: 778-797
<400> 7816
CGCCAACTTT GCCACGGCAT TACTGTTATT ACTCGGTGGT GCGGGCATTA 50
TTGGCAGCGT GATTTTCGGT AAACTGGGTA ATCAGTATGC GTCTGCGTTG 100
<210> 7817

<211> 100
<212> DNA
<213> Escherichia coli K-12 MG1655
<220> ydeb b1530
<222> 520-619
<223> ydeb b1530 U00096 complement(1616267..1616932)
<313> 7817: 561-580
<400> 7817
AGCCTACGCA GCTCCGGCGC AATTATGCGG CTGGTGGGCA AGGGTGGCAT 50
TGAAGCGATC TCCCGTCTGA TGGGATTCCT GCTGGTATGT ATGGGTGTGC 100
<210> 7818
<211> 100
<212> DNA
<213> Escherichia coli K-12 MG1655
<220> ydeb b1530
<222> 489-588
<223> ydeb b1530 U00096 complement(1616267..1616932)
<313> 7818: 512-531
<400> 7818
CTTTTTCCTG GTGGCGGTTA TTTTGTGGGG AAGCCTACGC AGCTCCGGCG 50
CAATTATGCG GCTGGTGGGC AAGGGTGGCA TTGAAGCGAT CTCCCGTCTG 100
<210> 7819
<211> 100
<212> DNA
<213> Escherichia coli K-12 MG1655
<220> ydeb b1530
<222> 420-519
<223> ydeb b1530 U00096 complement(1616267..1616932)
<313> 7819: 468-487
<400> 7819
CTCGGCGTCC ACAGTGCGTC AGAGTTCAAC CTTCGCTGAT TGGGTACTGA 50
TGGTTGCGCC GCCGCTGATC TTTTTCCTGG TGGCGGTTAT TTTGTGGGGA 100
<210> 7820
<211> 100
<212> DNA
<213> Escherichia coli K-12 MG1655
<220> yded b1534
<222> 702-801
<223> yded b1534 U00096 complement(1618262..1619062)
<313> 7820: 780-799
<400> 7820
AACGGGTCTG CAATTTTTAG GTGCGGTGCT CATTATGACC GGGCTGTATA 50
TCAATGTATT TGGCTTGCGG TGGCGTAAAG CGGTAAAGGT GGGAAGTTAA 100
<210> 7821
<211> 100
<212> DNA
<213> Escherichia coli K-12 MG1655
<220> yded b1534
<222> 698-797
<223> yded b1534 U00096 complement(1618262..1619062)
<313> 7821: 723-742
<400> 7821
GCTTAACGGG TCTGCAATTT TTAGGTGCGG TGCTCATTAT GACCGGGCTG 50
TATATCAATG TATTTGGCTT GCGGTGGCGT AAAGCGGTAA AGGTGGGAAG 100
<210> 7822
<211> 100
<212> DNA
<213> Escherichia coli K-12 MG1655
<220> yded b1534
<222> 654-753
<223> yded b1534 U00096 complement(1618262..1619062)
<313> 7822: 689-708
<400> 7822
GCCCGTAGTA GGACTGGCAA GTGCGGCACT ATTGTTGGAT GAACGCTTAA 50
CGGGTCTGCA ATTTTTAGGT GCGGTGCTCA TTATGACCGG GCTGTATATC 100
<210> 7823
<211> 100
<212> DNA
<213> Escherichia coli K-12 MG1655
<220> ydef b1535
<222> 1089-1188

<223> ydef b1535 U00096 1619356..1620543
<313> 7823: 1137-1156
<400> 7823
CTCGCTGTTT GTCATCTTAG CGTTGGTGAT CATTGCTGCG TGGGTGCTGA 50
TGTTAAAAGG GATTCGAGCA AGACCGTGGG GGCAGCCCGC GCTTTGTTGA 100
<210> 7824
<211> 100
<212> DNA
<213> Escherichia coli K-12 MG1655
<220> ydef b1535
<222> 1048-1147
<223> ydef b1535 U00096 1619356..1620543
<313> 7824: 1075-1094
<400> 7824
CCATTAGTGA GTGGCGTAGT GCTAACCAGC CTGCCGCCTT CCTCGCTGTT 50
TGTCATCTTA GCGTTGGTGA TCATTGCTGC GTGGGTGCTG ATGTTAAAAG 100
<210> 7825
<211> 100
<212> DNA
<213> Escherichia coli K-12 MG1655
<220> ydef b1535
<222> 955-1054
<223> ydef b1535 U00096 1619356..1620543
<313> 7825: 985-1004
<400> 7825
GAGTATATGT TGATTGACCA TATTGCGCCG CCAGAAATGA AAGCCAGCTA 50
TTTTTCCGCC CAGTCTTTAG GCTGGCTTGG TGCCGCGATT AACCCATTAG 100
<210> 7826
<211> 100
<212> DNA
<213> Escherichia coli K-12 MG1655
<220> ydeh b1536
<222> 792-891
<223> ydeh b1536 U00096 complement(1620984..1621874)
<313> 7826: 843-862
<400> 7826
GGTCATTGGA AGAGCGGACC GGGCAATGTA TGAGGGTAAG CAAACCGGAA 50
GAAATCGCTG CATGTTTATT GACGAACAAA ATGTGATTAA CCGAGTTTAA 100
<210> 7827
<211> 100
<212> DNA
<213> Escherichia coli K-12 MG1655
<220> ydeh b1536
<222> 766-865
<223> ydeh b1536 U00096 complement(1620984..1621874)
<313> 7827: 792-811
<400> 7827
GCATTTCCTG AAGAGCCTCT GGATGTGGTC ATTGGAAGAG CGGACCGGGC 50
AATGTATGAG GGTAAGCAAA CCGGAAGAAA TCGCTGCATG TTTATTGACG 100
<210> 7828
<211> 100
<212> DNA
<213> Escherichia coli K-12 MG1655
<220> ydeh b1536
<222> 734-833
<223> ydeh b1536 U00096 complement(1620984..1621874)
<313> 7828: 761-780
<400> 7828
TCAACATTAC CGTGACAGCA GGTGTGAGTC GCGCATTTCC TGAAGAGCCT 50
CTGGATGTGG TCATTGGAAG AGCGGACCGG GCAATGTATG AGGGTAAGCA 100
<210> 7829
<211> 100
<212> DNA
<213> Escherichia coli K-12 MG1655
<220> ydei b1537
<222> 261-360
<223> ydei b1537 U00096 complement(1622129..1622521)
<313> 7829: 296-315
<400> 7829
AATTAATGTC GTTATTCCTG CGGCCGTCTT TGATGGACGA GAAGTTCAGC 50
CGGACCAGAT GATCAACATT AGTGGCAGTC TGGATAAGAA AAGCGCGCCG 100

<210> 7830
<211> 100
<212> DNA
<213> Escherichia coli K-12 MG1655
<220> ydei b1537
<222> 163-262
<223> ydei b1537 U00096 complement(1622129..1622521)
<313> 7830: 206-225
<400> 7830
GCAAAAACCA TGCACGATGG CGCTTCGGTT TCACTACGCG GTAATTTGAT 50
TTCTCACAAA GGAGAGGACC GTTACGTTTT TCGCGATAAG AGCGGTGAAA 100
<210> 7831
<211> 100
<212> DNA
<213> Escherichia coli K-12 MG1655
<220> ydei b1537
<222> 137-236
<223> ydei b1537 U00096 complement(1622129..1622521)
<313> 7831: 169-188
<400> 7831
ATGCAAAAAA AATGACCGTT GATTTCGCAA AAACCATGCA CGATGGCGCT 50
TCGGTTTCAC TACGCGGTAA TTTGATTTCT CACAAAGGAG AGGACCGTTA 100
<210> 7832
<211> 100
<212> DNA
<213> Escherichia coli K-12 MG1655
<220> ydej b1538
<222> 420-519
<223> ydej b1538 U00096 1622797..1623315
<313> 7832: 462-481
<400> 7832
GAACTACACT GCGGTTATGC ATTTTGCTGG CGACTGCGAA ACGGTATTAG 50
CTTTAGCGGT GAGGTTTGCC CTCGCCCAGC TGCTGCAATT ACTGCTATAA 100
<210> 7833
<211> 100
<212> DNA
<213> Escherichia coli K-12 MG1655
<220> ydej b1538
<222> 326-425
<223> ydej b1538 U00096 1622797..1623315
<313> 7833: 367-386
<400> 7833
TCAGTATTGC CATTACCGGC TACGGCGGAC CGGAGGGCGG TGAAGATGGT 50
ACGCCAGCGG GTACCGTCTG GTTTGCGTGG CATATTAAAG GCCAGAACTA 100
<210> 7834
<211> 100
<212> DNA
<213> Escherichia coli K-12 MG1655
<220> ydej b1538
<222> 230-329
<223> ydej b1538 U00096 1622797..1623315
<313> 7834: 248-267
<400> 7834
GCGTAAGCCA GCAATCTCTT GAACGATATT CTGCGGTGAG TGAGAAAGTG 50
GCAGCAGAAA TGGCAACCGG TGCCATAGAG CGTGCGGATG CTGATGTCAG 100
<210> 7835
<211> 100
<212> DNA
<213> Escherichia coli K-12 MG1655
<220> b1730 b1730
<222> 716-815
<223> b1730 b1730 U00096 complement(1810353..1811168)
<313> 7835: 768-787
<400> 7835
CTGACGATCT GTTTAAATAT GAGTCAGTAT GCATCGCCGC GATGGAACAT 50
TTAAAAAGTA AGGCAACGGC CCCTATACAA AACGGACCGT TGCCAGCATA 100
<210> 7836
<211> 100
<212> DNA
<213> Escherichia coli K-12 MG1655
<220> b1730 b1730

<222> 644-743
<223> b1730 b1730 U00096 complement(1810353..1811168)
<313> 7836: 664-683
<400> 7836
AGGTTTTAAG CTTCTCGCTA CAGGCACATT CATTGAGCCA GGAATGTATT 50
GATAAGTTGG ACATTTTTTT CGCTGACGAT CTGTTTAAAT ATGAGTCAGT 100
<210> 7837
<211> 100
<212> DNA
<213> Escherichia coli K-12 MG1655
<220> b1730 b1730
<222> 550-649
<223> b1730 b1730 U00096 complement(1810353..1811168)
<313> 7837: 580-599
<400> 7837
TTTAATCCAC TAAATCTTCC GGGAATGGGA AGTGGTGTGC TTGAAGATAT 50
TATGAGTATT CCAGATAGTT CACTTAGAAA ACGTCTCGGG TATGAGGTTT 100
<210> 7838
<211> 100
<212> DNA
<213> Escherichia coli K-12 MG1655
<220> sfsa b0147
<222> 606-705
<223> sfsa b0147 U00096 complement(160782..161486)
<313> 7838: 681-700
<400> 7838
GTCAGAAGCT CAACAGAGGG GGGTAGAAAT TCTGGCTTAC AAAGCGGAAA 50
TTTCTGCTGA AGGCATGGCT CTTAAAAAAT CACTGCCGGT TACATTGTAG 100
<210> 7839
<211> 100
<212> DNA
<213> Escherichia coli K-12 MG1655
<220> sfsa b0147
<222> 604-703
<223> sfsa b0147 U00096 complement(160782..161486)
<313> 7839: 637-656
<400> 7839
TTGTCAGAAG CTCAACAGAG GGGGGTAGAA ATTCTGGCTT ACAAAGCGGA 50
AATTTCTGCT GAAGGCATGG CTCTTAAAAA ATCACTGCCG GTTACATTGT 100
<210> 7840
<211> 100
<212> DNA
<213> Escherichia coli K-12 MG1655
<220> sfsa b0147
<222> 510-609
<223> sfsa b0147 U00096 complement(160782..161486)
<313> 7840: 533-552
<400> 7840
AGGCCAGCGT GCGGTTATCT TTTTCGCCGT GCTGCATTCA GCCATTACAC 50
GGTTTTCACC CGCGCGCCAC ATCGATGAGA AATACGCGCA ACTATTGTCA 100
<210> 7841
<211> 100
<212> DNA
<213> Escherichia coli K-12 MG1655
<220> ydek b1511
<222> 3879-3978
<223> ydek b1511 U00096 complement(1592133..1596110)
<313> 7841: 3947-3966
<400> 7841
TCAACACATT AATATCCATA ACAGCGGTGT GATGTCGGGA AATGTCACCA 50
CTGCAGGTGA TATGAACGTT ATGCCTGGGG GGGGCACTGC GTGTCGCTAA 100
<210> 7842
<211> 100
<212> DNA
<213> Escherichia coli K-12 MG1655
<220> ydek b1511
<222> 3874-3973
<223> ydek b1511 U00096 complement(1592133..1596110)
<313> 7842: 3905-3924
<400> 7842
GCAAGTCAAC ACATTAATAT CCATAACAGC GGTGTGATGT CGGGAAATGT 50

CACCACTGCA GGTGATATGA ACGTTATGCC TGGGGGGGGC ACTGCGTGTC 100
<210> 7843
<211> 100
<212> DNA
<213> Escherichia coli K-12 MG1655
<220> ydek b1511
<222> 3792-3891
<223> ydek b1511 U00096 complement(1592133..1596110)
<313> 7843: 3847-3866
<400> 7843
CAACGATTCC GCGGCGTATC AGGGTACGAC GGATATCGTG GGGGGGGAAA 50
TTGCTTTCGG TTCCGACTCT GCCATTAATA TGGCAAGTCA ACACATTAAT 100
<210> 7844
<211> 100
<212> DNA
<213> Escherichia coli K-12 MG1655
<220> b1731 b1732
<222> 165-264
<223> b1731 b1732 U00096 complement(1811445..1811708)
<313> 7844: 227-246
<400> 7844
GATGGGAGAG TCATTTCTGC GCTCACCGGC GTTTACTGTG CCTGAATCTG 50
CCCAACGTTG GGCAAATCAG ATCCGCCAGG AAGGGGAAGT GACTGAGTAA 100
<210> 7845
<211> 100
<212> DNA
<213> Escherichia coli K-12 MG1655
<220> b1731 b1732
<222> 164-263
<223> b1731 b1732 U00096 complement(1811445..1811708)
<313> 7845: 194-213
<400> 7845
TGATGGGAGA GTCATTTCTG CGCTCACCGG CGTTTACTGT GCCTGAATCT 50
GCCCAACGTT GGGCAAATCA GATCCGCCAG GAAGGGGAAG TGACTGAGTA 100
<210> 7846
<211> 100
<212> DNA
<213> Escherichia coli K-12 MG1655
<220> b1731 b1732
<222> 61-160
<223> b1731 b1732 U00096 complement(1811445..1811708)
<313> 7846: 80-99
<400> 7846
AGCTATGTCC CACGCACGGA ACCCGCGCCG CCAGAACATG CGATAAAGAT 50
GGATTCGTTT CGTGATGTCT GGATGCTGCG TGGCAAATAT GTTGCGTTTG 100
<210> 7847
<211> 100
<212> DNA
<213> Escherichia coli K-12 MG1655
<220> b2460 b2460
<222> 582-681
<223> b2460 b2460 U00096 complement(2572322..2573023)
<313> 7847: 608-627
<400> 7847
CATGATCGCC AAAGCAGGCG ACGTGATGTT TATCCCGAAA GGCTCCAGCA 50
TCGAATTTGG TACGACATCC AGCGTGAAAT TCCTGTATGT CGCCTGGCCG 100
<210> 7848
<211> 100
<212> DNA
<213> Escherichia coli K-12 MG1655
<220> b2460 b2460
<222> 545-644
<223> b2460 b2460 U00096 complement(2572322..2573023)
<313> 7848: 575-594
<400> 7848
TGGAAGGGGA GCTGCATGTT CGCCACGAAG GCCAAACCAT GATCGCCAAA 50
GCAGGCGACG TGATGTTTAT CCCGAAAGGC TCCAGCATCG AATTTGGTAC 100
<210> 7849
<211> 100
<212> DNA
<213> Escherichia coli K-12 MG1655

<220> b2460 b2460
<222> 499-598
<223> b2460 b2460 U00096 complement(2572322..2573023)
<313> 7849: 525-544
<400> 7849
GCATTCTTCC CGTGGACCCT GAACTACGAC GAAATCGACA TGGTGCTGGA  50
AGGGGAGCTG CATGTTCGCC ACGAAGGCCA AACCATGATC GCCAAAGCAG  100
<210> 7850
<211> 100
<212> DNA
<213> Escherichia coli K-12 MG1655
<220> b2461 b2461
<222> 381-480
<223> b2461 b2461 U00096 complement(2572998..2573477)
<313> 7850: 445-464
<400> 7850
AAACAGCCAC GACCCGCAAA GCGTGCAGCA ACTGGTGGAT TATCTGGCAT  50
CTCTCACCAA ACAGGAGGAA GCAGGTGAAA AAACTCATCA CAGCGAATGA  100
<210> 7851
<211> 100
<212> DNA
<213> Escherichia coli K-12 MG1655
<220> b2461 b2461
<222> 366-465
<223> b2461 b2461 U00096 complement(2572998..2573477)
<313> 7851: 403-422
<400> 7851
GCCGATGTTC GAGCTAAACA GCCACGACCC GCAAAGCGTG CAGCAACTGG  50
TGGATTATCT GGCATCTCTC ACCAAACAGG AGGAAGCAGG TGAAAAAACT  100
<210> 7852
<211> 100
<212> DNA
<213> Escherichia coli K-12 MG1655
<220> b2461 b2461
<222> 288-387
<223> b2461 b2461 U00096 complement(2572998..2573477)
<313> 7852: 340-359
<400> 7852
CGTCATCAGT AAAACGGACA TGCCAGATGC TGACGTCGCC GCAACGCGAA  50
AGTTATTGCT TGAGACGGGT TTCGAAGAGC CGATGTTCGA GCTAAACAGC  100
<210> 7853
<211> 100
<212> DNA
<213> Escherichia coli K-12 MG1655
<220> b2462 b2462
<222> 284-383
<223> b2462 b2462 U00096 complement(2573490..2573897)
<313> 7853: 320-339
<400> 7853
ACCGATTCAG CGGTGCATTG GTGATTTATG GCTCGGTGGG CGCAGTAGAA  50
GAAGCGTTAT CACAAACCGT CAGCGGTTTA GGTCGCTTAT TAAATTATAC  100
<210> 7854
<211> 100
<212> DNA
<213> Escherichia coli K-12 MG1655
<220> b2462 b2462
<222> 169-268
<223> b2462 b2462 U00096 complement(2573490..2573897)
<313> 7854: 196-215
<400> 7854
ATCGGCGTTC CGGATGCGGG CGCAATTGGC ATTATGACGC TAACTCCCGG  50
CGAAACGGCG ATGATCGCCG GTGATTTAGC GCTGAAAGCC GCTGATGTGC  100
<210> 7855
<211> 100
<212> DNA
<213> Escherichia coli K-12 MG1655
<220> b2462 b2462
<222> 96-195
<223> b2462 b2462 U00096 complement(2573490..2573897)
<313> 7855: 136-155
<400> 7855

GGAATTTGTG CCCGGCAAAC AGGTCACACT GGCGCATCTC ATCGCGCATC 50
CCGGTGAGGA ACTGGCGAAG AAAATCGGCG TTCCGGATGC GGGCGCAATT 100
<210> 7856
<211> 100
<212> DNA
<213> Escherichia coli K-12 MG1655
<220> b2463 b2464
<222> 2109-2208
<223> b2463 b2464 U00096 complement(2574118..2576397)
<313> 7856: 2149-2168
<400> 7856
CCGCATTAGT TACAACTTAC TGCGTGTTTC CAGCTCGGAA GGTGTGACTG 50
TCGGCCCGGT GCTGATGGGT GTGGCGAAAC CGGTTCACGT GTTAACGCCG 100
<210> 7857
<211> 100
<212> DNA
<213> Escherichia coli K-12 MG1655
<220> b2463 b2464
<222> 2045-2144
<223> b2463 b2464 U00096 complement(2574118..2576397)
<313> 7857: 2093-2112
<400> 7857
GTATGCCGGA CAGCTCTTTG AAAGGTTCCG CCAATATTCT GGTGATGCCG 50
AACATGGAAG CTGCCCGCAT TAGTTACAAC TTACTGCGTG TTTCCAGCTC 100
<210> 7858
<211> 100
<212> DNA
<213> Escherichia coli K-12 MG1655
<220> b2463 b2464
<222> 2038-2137
<223> b2463 b2464 U00096 complement(2574118..2576397)
<313> 7858: 2060-2079
<400> 7858
AACGACCGTA TGCCGGACAG CTCTTTGAAA GGTTCCGCCA ATATTCTGGT 50
GATGCCGAAC ATGGAAGCTG CCCGCATTAG TTACAACTTA CTGCGTGTTT 100
<210> 7859
<211> 100
<212> DNA
<213> Escherichia coli K-12 MG1655
<220> brnq b0402
<222> 1202-1301
<223> brnq b0402 U00096 418815..420134
<313> 7859: 1233-1252
<400> 7859
AGCGTTTACC GCTGGCCGAA CAAGGTCTGG CGTGGTTAAT GCCAACAGTG 50
GTGATGGTGG TTCTGGCCAT TATCTGGGAT CGCGCGGCAG GTCGTCAGGT 100
<210> 7860
<211> 100
<212> DNA
<213> Escherichia coli K-12 MG1655
<220> brnq b0402
<222> 1160-1259
<223> brnq b0402 U00096 418815..420134
<313> 7860: 1194-1213
<400> 7860
TCAAAGCATC TGCATTCAGC GATATCTTAC CGTCCTGGGC GCAGCGTTTA 50
CCGCTGGCCG AACAAGGTCT GGCGTGGTTA ATGCCAACAG TGGTGATGGT 100
<210> 7861
<211> 100
<212> DNA
<213> Escherichia coli K-12 MG1655
<220> brnq b0402
<222> 1115-1214
<223> brnq b0402 U00096 418815..420134
<313> 7861: 1152-1171
<400> 7861
CTCCGCCGAT GTTTATCAGC CTGCTTTTTG GTATTCTCGA CGGGATCAAA 50
GCATCTGCAT TCAGCGATAT CTTACCGTCC TGGGCGCAGC GTTTACCGCT 100
<210> 7862
<211> 100
<212> DNA

<213> Escherichia coli K-12 MG1655
<220> ysga b3831
<222> 900-999
<223> ysga b3831 U00096 complement(4012721..4013719)
<313> 7862: 965-984
<400> 7862
ACAGTCCGGG TTGACCCACA GGCGCTCTGC CGGAATGCGT TTTGCCGCTT  50
TCTTCAGCAA GGCTTCAATC CATTCCACGC TCGGTACGTT TGGCGAGTGA  100
<210> 7863
<211> 100
<212> DNA
<213> Escherichia coli K-12 MG1655
<220> ysga b3831
<222> 786-885
<223> ysga b3831 U00096 complement(4012721..4013719)
<313> 7863: 835-854
<400> 7863
ACCGGGTGGT ATTACCACCC GGTTTGGATT TTACCCCCGA CGCAAGTTCT  50
GCGCCGCCTG CACCATGTTC GCCAGTGCCG CGCGGGTTTC TGGCCAGCCG  100
<210> 7864
<211> 100
<212> DNA
<213> Escherichia coli K-12 MG1655
<220> ysga b3831
<222> 727-826
<223> ysga b3831 U00096 complement(4012721..4013719)
<313> 7864: 779-798
<400> 7864
AGCGTATGCT GGAATGGTTT AAGCAGTATG GTGGGAAGAA GTCGCTGTAA  50
TGAGAAAAGA CCGGGTGGTA TTACCACCCG GTTTGGATTT TACCCCCGAC  100
<210> 7865
<211> 100
<212> DNA
<213> Escherichia coli K-12 MG1655
<220> b2466 b2467
<222> 932-1031
<223> b2466 b2467 U00096 complement(2579754..2580797)
<313> 7865: 983-1002
<400> 7865
CGCGCTGGCG CTTTGACGGT CAGCGTTTTC GCCTGGTGCG TTACGCCGCA  50
GAACCCACCT GCGATAACTG GCATGGGCCA GACGCCTGGC CCACGTTGTG  100
<210> 7866
<211> 100
<212> DNA
<213> Escherichia coli K-12 MG1655
<220> b2466 b2467
<222> 877-976
<223> b2466 b2467 U00096 complement(2579754..2580797)
<313> 7866: 904-923
<400> 7866
CGTGAACTGG TGACCTTAGC GAAAGGGCGC GGATTAAGCG ATTGTGGCAT  50
TCAGGCGCGC TGGCGCTTTG ACGGTCAGCG TTTTCGCCTG GTGCGTTACG  100
<210> 7867
<211> 100
<212> DNA
<213> Escherichia coli K-12 MG1655
<220> b2466 b2467
<222> 779-878
<223> b2466 b2467 U00096 complement(2579754..2580797)
<313> 7867: 847-866
<400> 7867
CACTAGCTTC GCGCCCGGTT CGGTTGCGTT TGCCGTTCAA CAACGGTCAG  50
GAGACGAATG AACTGGAACT GATGAACGCA ACATTTGATG AGAAATCGCG  100
<210> 7868
<211> 100
<212> DNA
<213> Escherichia coli K-12 MG1655
<220> ydev b1512
<222> 1464-1563
<223> ydev b1512 U00096 complement(1596641..1598233)
<313> 7868: 1507-1526

<400> 7868
CACACCAGAC CCGGAAAAGC ATGAACTTTA TCAGGATTCA CGCGATAAGT 50
GGCAGGCAGT TTATCAGGAT CAGCTGGGGC TGGTTGATCA TGGACTGACG 100
<210> 7869
<211> 100
<212> DNA
<213> Escherichia coli K-12 MG1655
<220> ydev b1512
<222> 1436-1535
<223> ydev b1512 U00096 complement(1596641..1598233)
<313> 7869: 1466-1485
<400> 7869
AACGCCTGGT TCGCTGGGAA CGGACGCACA CACCAGACCC GGAAAAGCAT 50
GAACTTTATC AGGATTCACG CGATAAGTGG CAGGCAGTTT ATCAGGATCA 100
<210> 7870
<211> 100
<212> DNA
<213> Escherichia coli K-12 MG1655
<220> ydev b1512
<222> 1398-1497
<223> ydev b1512 U00096 complement(1596641..1598233)
<313> 7870: 1422-1441
<400> 7870
CGGTGCCGGA ATTTTTTCAT CAATGGCAGA AACCGGAGAA CGCCTGGTTC 50
GCTGGGAACG GACGCACACA CCAGACCCGG AAAAGCATGA ACTTTATCAG 100
<210> 7871
<211> 100
<212> DNA
<213> Escherichia coli K-12 MG1655
<220> ydew ydeW
<222> 855-954
<223> ydew ydeW U00096 complement(1598312..1599265)
<313> 7871: 909-928
<400> 7871
AGAAAATAAA GCCGAAGCAA TTGCCGCTGC AATGAAAGGC GGTTATATCA 50
ACGCACTGGT TACCGATCAG GACACAGCAG CGGCGATTTT ACGTAGTTAA 100
<210> 7872
<211> 100
<212> DNA
<213> Escherichia coli K-12 MG1655
<220> ydew ydeW
<222> 811-910
<223> ydew ydeW U00096 complement(1598312..1599265)
<313> 7872: 850-869
<400> 7872
AGCGCGCTGA AGACCATACC CGTCCGGGTT GGCGTGGCAG GGGGAGAAAA 50
TAAAGCCGAA GCAATTGCCG CTGCAATGAA AGGCGGTTAT ATCAACGCAC 100
<210> 7873
<211> 100
<212> DNA
<213> Escherichia coli K-12 MG1655
<220> ydew ydeW
<222> 670-769
<223> ydew ydeW U00096 complement(1598312..1599265)
<313> 7873: 719-738
<400> 7873
TCCGGTTATA TCAGCCAGGG CGAACAGTTA ATGATTGGCC GAAAAGGGGC 50
GGTTGGCGAC ATTTTAGGCT ACTTTTTTGA TGCAAAAGGT GACGTTGTCA 100
<210> 7874
<211> 100
<212> DNA
<213> Escherichia coli K-12 MG1655
<220> ydey b1515
<222> 930-1029
<223> ydey b1515 U00096 1601043..1602071
<313> 7874: 1005-1024
<400> 7874
GCTGGAACGT AATCTACGGC GGCAAAAATA TGCCCGCTTT ATGACGCCAC 50
CGCCATCCGT TAAACCCGCT TCGTCAGGTA AAAAACGGGA GGCCGCATAA 100
<210> 7875
<211> 100

<212> DNA
<213> Escherichia coli K-12 MG1655
<220> ydey b1515
<222> 912-1011
<223> ydey b1515 U00096 1601043..1602071
<313> 7875: 967-986
<400> 7875
TGGACGCCTG CGTTGTGCGC TGGAACGTAA TCTACGGCGG CAAAAATATG 50
CCCGCTTTAT GACGCCACCG CCATCCGTTA AACCCGCTTC GTCAGGTAAA 100
<210> 7876
<211> 100
<212> DNA
<213> Escherichia coli K-12 MG1655
<220> ydey b1515
<222> 870-969
<223> ydey b1515 U00096 1601043..1602071
<313> 7876: 923-942
<400> 7876
TTTTATCGCG GGTCTGGTTC TGCTGGCGGT GCTGGTGTTT GATGGACGCC 50
TGCGTTGTGC GCTGGAACGT AATCTACGGC GGCAAAAATA TGCCCGCTTT 100
<210> 7877
<211> 100
<212> DNA
<213> Escherichia coli K-12 MG1655
<220> ydez ydeZ
<222> 887-986
<223> ydez ydeZ U00096 1602071..1603063
<313> 7877: 933-952
<400> 7877
TTTCCGGTGC GCTACTTATC GTCGTTGTCG TAGGTCGTTC CGTTAGCCTG 50
CATCGCCAGC AAATTAAAGA GTGGCTGGCG CGTCGGGCCA ATAACCCATT 100
<210> 7878
<211> 100
<212> DNA
<213> Escherichia coli K-12 MG1655
<220> ydez ydeZ
<222> 843-942
<223> ydez ydeZ U00096 1602071..1603063
<313> 7878: 866-885
<400> 7878
AGGTTTGCAA ATGGCAGGAG TGCCAAATCA GGTGTCCAGC GCCCTTTCCG 50
GTGCGCTACT TATCGTCGTT GTCGTAGGTC GTTCCGTTAG CCTGCATCGC 100
<210> 7879
<211> 100
<212> DNA
<213> Escherichia coli K-12 MG1655
<220> ydez ydeZ
<222> 762-861
<223> ydez ydeZ U00096 1602071..1603063
<313> 7879: 800-819
<400> 7879
TGGCGGGGCC AATATTTATG GTGGTTCCGG TTCCATTATC GGCACCGCCA 50
TTGCGGTTTT ATTAGTGGGA TATTTGCAAC AAGGTTTGCA AATGGCAGGA 100
<210> 7880
<211> 100
<212> DNA
<213> Escherichia coli K-12 MG1655
<220> trxa b3782
<222> 285-384
<223> trxa b3782 U00096 3963322..3963705
<313> 7880: 303-322
<400> 7880
GACTCTGCTG CTGTTCAAAA ACGGTGAAGT GGCGGCAACC AAAGTGGGTG 50
CACTGTCTAA AGGTCAGTTG AAAGAGTTCC TCGACGCTAA CCTGGCGTAA 100
<210> 7881
<211> 100
<212> DNA
<213> Escherichia coli K-12 MG1655
<220> trxa b3782
<222> 242-341
<223> trxa b3782 U00096 3963322..3963705

<313> 7881: 261-280
<400> 7881
AAAACCCTGG CACTGCGCCG AAATATGGCA TCCGTGGTAT CCCGACTCTG 50
CTGCTGTTCA AAAACGGTGA AGTGGCGGCA ACCAAAGTGG GTGCACTGTC 100
<210> 7882
<211> 100
<212> DNA
<213> Escherichia coli K-12 MG1655
<220> trxa b3782
<222> 170-269
<223> trxa b3782 U00096 3963322..3963705
<313> 7882: 219-238
<400> 7882
TCGCCCCGAT TCTGGATGAA ATCGCTGACG AATATCAGGG CAAACTGACC 50
GTTGCAAAAC TGAACATCGA TCAAAACCCT GGCACTGCGC CGAAATATGG 100
<210> 7883
<211> 100
<212> DNA
<213> Escherichia coli K-12 MG1655
<220> trxb b0889
<222> 867-966
<223> trxb b0889 U00096 complement(930308..931273)
<313> 7883: 931-950
<400> 7883
GGATCACATT TATCGCCAGG CCATTACTTC GGCCGGTACA GGCTGCATGG 50
CAGCACTTGA TGCGGAACGC TACCTCGATG GTTTAGCTGA CGCAAAATAA 100
<210> 7884
<211> 100
<212> DNA
<213> Escherichia coli K-12 MG1655
<220> trxb b0889
<222> 798-897
<223> trxb b0889 U00096 complement(930308..931273)
<313> 7884: 844-863
<400> 7884
ACAGTCGGGT ATTCATGGTA ATGCCACCCA GACCAGCATT CCTGGCGTCT 50
TTGCCGCAGG CGACGTGATG GATCACATTT ATCGCCAGGC CATTACTTCG 100
<210> 7885
<211> 100
<212> DNA
<213> Escherichia coli K-12 MG1655
<220> trxb b0889
<222> 774-873
<223> trxb b0889 U00096 complement(930308..931273)
<313> 7885: 801-820
<400> 7885
ACTGGAAAAC GGCTACATCA AAGTACAGTC GGGTATTCAT GGTAATGCCA 50
CCCAGACCAG CATTCCTGGC GTCTTTGCCG CAGGCGACGT GATGGATCAC 100
<210> 7886
<211> 100
<212> DNA
<213> Escherichia coli K-12 MG1655
<220> trxc b2583
<222> 270-369
<223> trxc b2583 U00096 2716755..2717174
<313> 7886: 294-313
<400> 7886
GAATACCGAA GCTGAACGTG AATTGAGCAG TCGCTTTGGA ATTCGTAGTA 50
TACCGACGAT CATGATTTTC AAAAACGGTC AGGTTGTCGA CATGCTTAAT 100
<210> 7887
<211> 100
<212> DNA
<213> Escherichia coli K-12 MG1655
<220> trxc b2583
<222> 226-325
<223> trxc b2583 U00096 2716755..2717174
<313> 7887: 266-285
<400> 7887
GATGTCGCGC AAGAGCGTAG CGGTAAAGTG CGCTTTGTGA AAGTGAATAC 50
CGAAGCTGAA CGTGAATTGA GCAGTCGCTT TGGAATTCGT AGTATACCGA 100
<210> 7888

<211> 100
<212> DNA
<213> Escherichia coli K-12 MG1655
<220> trxc b2583
<222> 118-217
<223> trxc b2583 U00096 2716755..2717174
<313> 7888: 157-176
<400> 7888
GCGACCGGTG AAACACTCGA CAAATTGCTG AAGGATGATC TACCTGTGGT 50
GATCGACTTC TGGGCACCGT GGTGCGGCCC CTGCCGTAAT TTCGCACCAA 100
<210> 7889
<211> 100
<212> DNA
<213> Escherichia coli K-12 MG1655
<220> ydfa b1572
<222> 14-113
<223> ydfa b1572 U00096 1646532..1646687
<313> 7889: 66-85
<400> 7889
ATCTTGGCAA CAACGAATCT CTGGTGTACG GCGTGTTTCC AAACCAGGAC 50
GGCACGTTCA CCGCAATGAC GTATACCAAA AGCAAAACGT TTAAAACCGA 100
<210> 7890
<211> 100
<212> DNA
<213> Escherichia coli K-12 MG1655
<220> ydfa b1572
<222> 21-120
<223> ydfa b1572 U00096 1646532..1646687
<313> 7890: 55-74
<400> 7890
CAACAACGAA TCTCTGGTGT ACGGCGTGTT TCCAAACCAG GACGGCACGT 50
TCACCGCAAT GACGTATACC AAAAGCAAAA CGTTTAAAAC CGAAAATGGT 100
<210> 7891
<211> 100
<212> DNA
<213> Escherichia coli K-12 MG1655
<220> ydfa b1572
<222> 1-100
<223> ydfa b1572 U00096 1646532..1646687
<313> 7891: 40-59
<400> 7891
ATGGATACTA TCGATCTTGG CAACAACGAA TCTCTGGTGT ACGGCGTGTT 50
TCCAAACCAG GACGGCACGT TCACCGCAAT GACGTATACC AAAAGCAAAA 100
<210> 7892
<211> 100
<212> DNA
<213> Escherichia coli K-12 MG1655
<220> cora b3817
<222> 852-951
<223> cora b3817 U00096 3999038..3999988
<313> 7892: 922-941
<400> 7892
AGAACTGAAG TGGAGCTTCG GCTACCCTGG CGCGATTATC TTTATGATCC 50
TCGCGGGCCT GGCACCGTAT CTGTACTTTA AGCGGAAGAA CTGGTTGTAA 100
<210> 7893
<211> 100
<212> DNA
<213> Escherichia coli K-12 MG1655
<220> cora b3817
<222> 838-937
<223> cora b3817 U00096 3999038..3999988
<313> 7893: 872-891
<400> 7893
TTTGAGTTTA TGCCAGAACT GAAGTGGAGC TTCGGCTACC CTGGCGCGAT 50
TATCTTTATG ATCCTCGCGG GCCTGGCACC GTATCTGTAC TTTAAGCGGA 100
<210> 7894
<211> 100
<212> DNA
<213> Escherichia coli K-12 MG1655
<220> cora b3817
<222> 754-853

<223> cora b3817 U00096 3999038..3999988
<313> 7894: 791-810
<400> 7894
CAGAACCGCA TCATCAAAAT CTTCTCGGTG GTATCCGTGG TATTCCTGCC  50
GCCGACGCTC GTTGCTTCCA GCTATGGCAT GAACTTTGAG TTTATGCCAG  100
<210> 7895
<211> 100
<212> DNA
<213> Escherichia coli K-12 MG1655
<220> ydfb b1573
<222> 31-130
<223> ydfb b1573 U00096 1646647..1646817
<313> 7895: 55-74
<400> 7895
CAGGTGAGTG ATATGGATTT CGACACAATC ATGGAAAAGG CTTACGAAGA  50
ATACTTCGAA GGCCTTGCCG AAGGCGAAGA AGCTCTCAGC TTCAGTGAGT  100
<210> 7896
<211> 100
<212> DNA
<213> Escherichia coli K-12 MG1655
<220> ydfb b1573
<222> 2-101
<223> ydfb b1573 U00096 1646647..1646817
<313> 7896: 53-72
<400> 7896
TGGTGCCCGT CGCTGGCTGG AAAGAAACTC AGGTGAGTGA TATGGATTTC  50
GACACAATCA TGGAAAAGGC TTACGAAGAA TACTTCGAAG GCCTTGCCGA  100
<210> 7897
<211> 100
<212> DNA
<213> Escherichia coli K-12 MG1655
<220> ydfb b1573
<222> 1-100
<223> ydfb b1573 U00096 1646647..1646817
<313> 7897: 32-51
<400> 7897
ATGGTGCCCG TCGCTGGCTG GAAAGAAACT CAGGTGAGTG ATATGGATTT  50
CGACACAATC ATGGAAAAGG CTTACGAAGA ATACTTCGAA GGCCTTGCCG  100
<210> 7898
<211> 100
<212> DNA
<213> Escherichia coli K-12 MG1655
<220> ydfc b1574
<222> 120-219
<223> ydfc b1574 U00096 1646847..1647065
<313> 7898: 181-200
<400> 7898
TAAGCAGGCT GAAAATCTGT CCGCTCTGGA GCAGGATATC ACCAATCTGG  50
AAAAAGCAGC GGCATTAAGC ATTGCCAGAA TGATTACATA CCCGCGTTAG  100
<210> 7899
<211> 100
<212> DNA
<213> Escherichia coli K-12 MG1655
<220> ydfc b1574
<222> 56-155
<223> ydfc b1574 U00096 1646847..1647065
<313> 7899: 108-127
<400> 7899
ATGAGTGGAT GCGCCAGAAA ATTAATGCAG CGCAGGATCT GAAATGGGCT  50
AATGAAGCCA GGGCTAAGCA GGCTGAAAAT CTGTCCGCTC TGGAGCAGGA  100
<210> 7900
<211> 100
<212> DNA
<213> Escherichia coli K-12 MG1655
<220> ydfc b1574
<222> 30-129
<223> ydfc b1574 U00096 1646847..1647065
<313> 7900: 79-98
<400> 7900
GCCAGACTAT ACCGATGATG AACTTTATGA GTGGATGCGC CAGAAAATTA  50
ATGCAGCGCA GGATCTGAAA TGGGCTAATG AAGCCAGGGC TAAGCAGGCT  100

<210> 7901
<211> 100
<212> DNA
<213> Escherichia coli K-12 MG1655
<220> ydfd b1577
<222> 93-192
<223> ydfd b1577 U00096 1647818..1648009
<313> 7901: 162-181
<400> 7901
GTGTATGACT GCAGCAACCG AACAGAAAAT TCCCGGTAAC TGTTACCCGG 50
TCGATAAAGT TATTCACCAG GATAATATCG AAATCCCGGC AGGTCTTTAA 100
<210> 7902
<211> 100
<212> DNA
<213> Escherichia coli K-12 MG1655
<220> ydfd b1577
<222> 93-192
<223> ydfd b1577 U00096 1647818..1648009
<313> 7902: 163-182
<400> 7902
GTGTATGACT GCAGCAACCG AACAGAAAAT TCCCGGTAAC TGTTACCCGG 50
TCGATAAAGT TATTCACCAG GATAATATCG AAATCCCGGC AGGTCTTTAA 100
<210> 7903
<211> 100
<212> DNA
<213> Escherichia coli K-12 MG1655
<220> ydfd b1577
<222> 91-190
<223> ydfd b1577 U00096 1647818..1648009
<313> 7903: 144-163
<400> 7903
GAGTGTATGA CTGCAGCAAC CGAACAGAAA ATTCCCGGTA ACTGTTACCC 50
GGTCGATAAA GTTATTCACC AGGATAATAT CGAAATCCCG GCAGGTCTTT 100
<210> 7904
<211> 100
<212> DNA
<213> Escherichia coli K-12 MG1655
<220> ydfe ydfE
<222> 729-828
<223> ydfe ydfE U00096 1648102..1649022
<313> 7904: 769-788
<400> 7904
CAAGCAGACA TGGAAAACAC TGGATGACGA ACTGGCCTAT GGGCGCTGCT 50
TCGCAGACAG GCAGAACTTG ATGGTATGCC TGCGATCAAT GCCAAACGTG 100
<210> 7905
<211> 100
<212> DNA
<213> Escherichia coli K-12 MG1655
<220> ydfe ydfE
<222> 681-780
<223> ydfe ydfE U00096 1648102..1649022
<313> 7905: 733-752
<400> 7905
TTCAACAGGA AAAATCAGCC CGGTCATTGC TGCCATTCAT CGCGAATACA 50
AGCAGACATG GAAAACACTG GATGACGAAC TGGCCTATGG GCGCTGCTTC 100
<210> 7906
<211> 100
<212> DNA
<213> Escherichia coli K-12 MG1655
<220> ydfe ydfE
<222> 665-764
<223> ydfe ydfE U00096 1648102..1649022
<313> 7906: 692-711
<400> 7906
AACAGTACAC TGAAAATTCA ACAGGAAAAA TCAGCCCGGT CATTGCTGCC 50
ATTCATCGCG AATACAAGCA GACATGGAAA ACACTGGATG ACGAACTGGC 100
<210> 7907
<211> 100
<212> DNA
<213> Escherichia coli K-12 MG1655
<220> ydfg b1540

<222> 648-747
<223> ydfg b1540 U00096 1625541..1626287
<313> 7907: 723-742
<400> 7907
CTGGTGGGTG TCAACGCTGC CTGCTCACGT CAATATCAAT ACCCTGGAAA 50
TGATGCCGGT TACCCAAAGC TATGCCGGAC TGAATGTCCA CCGTCAGTAA 100
<210> 7908
<211> 100
<212> DNA
<213> Escherichia coli K-12 MG1655
<220> ydfg b1540
<222> 627-726
<223> ydfg b1540 U00096 1625541..1626287
<313> 7908: 683-702
<400> 7908
AGAAGATGTC AGCGAAGCCG TCTGGTGGGT GTCAACGCTG CCTGCTCACG 50
TCAATATCAA TACCCTGGAA ATGATGCCGG TTACCCAAAG CTATGCCGGA 100
<210> 7909
<211> 100
<212> DNA
<213> Escherichia coli K-12 MG1655
<220> ydfg b1540
<222> 591-690
<223> ydfg b1540 U00096 1625541..1626287
<313> 7909: 627-646
<400> 7909
AGAAAAAACC TATCAAAATA CCGTTGCATT GACGCCAGAA GATGTCAGCG 50
AAGCCGTCTG GTGGGTGTCA ACGCTGCCTG CTCACGTCAA TATCAATACC 100
<210> 7910
<211> 100
<212> DNA
<213> Escherichia coli K-12 MG1655
<220> ydfh ydfH
<222> 588-687
<223> ydfh ydfH U00096 1626376..1627062
<313> 7910: 668-687
<400> 7910
CGATGCGGTA GAACGTGCAA TGACGCAACA TTTGCAGGAA ATCAGCGAAT 50
CCGTGCGCCA GATCCGCCAG GAAAACAGCG ACTGGTTTAG CGAAGAGTAA 100
<210> 7911
<211> 100
<212> DNA
<213> Escherichia coli K-12 MG1655
<220> ydfh ydfH
<222> 588-687
<223> ydfh ydfH U00096 1626376..1627062
<313> 7911: 628-647
<400> 7911
CGATGCGGTA GAACGTGCAA TGACGCAACA TTTGCAGGAA ATCAGCGAAT 50
CCGTGCGCCA GATCCGCCAG GAAAACAGCG ACTGGTTTAG CGAAGAGTAA 100
<210> 7912
<211> 100
<212> DNA
<213> Escherichia coli K-12 MG1655
<220> ydfh ydfH
<222> 531-630
<223> ydfh ydfH U00096 1626376..1627062
<313> 7912: 575-594
<400> 7912
AGAAATGCTG TTACGCCAGC ATCTTGATAT TTTCTCTGCC CTGCAAAAAC 50
GTGATGGCGA TGCGGTAGAA CGTGCAATGA CGCAACATTT GCAGGAAATC 100
<210> 7913
<211> 100
<212> DNA
<213> Escherichia coli K-12 MG1655
<220> ydfi ydfI
<222> 1362-1461
<223> ydfi ydfI U00096 complement(1627477..1628937)
<313> 7913: 1432-1451
<400> 7913
GCCAGACAAT AGTTTGTTTA CTGCAAGAGT GACGGAAACG TACTTGTCTT 50

TATTAGCGCA TGGCGCGAAA GCGACCGTGG CAAAATATTC CGTGAAGTAA 100
<210> 7914
<211> 100
<212> DNA
<213> Escherichia coli K-12 MG1655
<220> ydfi ydfI
<222> 1351-1450
<223> ydfi ydfI U00096 complement(1627477..1628937)
<313> 7914: 1401-1420
<400> 7914
GGTGATGATT TGCCAGACAA TAGTTTGTTT ACTGCAAGAG TGACGGAAAC 50
GTACTTGTCT TTATTAGCGC ATGGCGCGAA AGCGACCGTG GCAAAATATT 100
<210> 7915
<211> 100
<212> DNA
<213> Escherichia coli K-12 MG1655
<220> ydfi ydfI
<222> 1234-1333
<223> ydfi ydfI U00096 complement(1627477..1628937)
<313> 7915: 1295-1314
<400> 7915
CAGGGAAATC CGATAGAAAT CAGTGACCCA CTGTTACCTG TTATTCAGAA 50
GGCTGTACAA AGTAGTGCCG AAGGGAAAGC GCGCGTCCAG TCATTGCTGG 100
<210> 7916
<211> 100
<212> DNA
<213> Escherichia coli K-12 MG1655
<220> chaa b1217
<222> 1002-1101
<223> chaa b1217 U00096 complement(1269972..1271072)
<313> 7916: 1068-1087
<400> 7916
GCTGTGCCAT ATCTCCTTCT CCACCGGACG TACTAACGTG CTCAATGGCG 50
CAGCGCATCT GGCACTGTTT GCCGCCTATT TGATGACGAT ATTTGCCTGA 100
<210> 7917
<211> 100
<212> DNA
<213> Escherichia coli K-12 MG1655
<220> chaa b1217
<222> 1002-1101
<223> chaa b1217 U00096 complement(1269972..1271072)
<313> 7917: 1024-1043
<400> 7917
GCTGTGCCAT ATCTCCTTCT CCACCGGACG TACTAACGTG CTCAATGGCG 50
CAGCGCATCT GGCACTGTTT GCCGCCTATT TGATGACGAT ATTTGCCTGA 100
<210> 7918
<211> 100
<212> DNA
<213> Escherichia coli K-12 MG1655
<220> chaa b1217
<222> 941-1040
<223> chaa b1217 U00096 complement(1269972..1271072)
<313> 7918: 998-1017
<400> 7918
GTAACGAATT GCAGTTTGCA CTTGGTGCGC CAGAAATGGT GGTGATGGTG 50
GCCTCTTTAG TGCTGTGCCA TATCTCCTTC TCCACCGGAC GTACTAACGT 100
<210> 7919
<211> 100
<212> DNA
<213> Escherichia coli K-12 MG1655
<220> chab b1218
<222> 132-231
<223> chab b1218 U00096 1271342..1271572
<313> 7919: 212-231
<400> 7919
TGACGCCAGT CGCGAAGAAA CCGCGCATAA AGTGGCCTGG GCTGCTGTGA 50
AGCATGAATA TGCCAAAGGG GATGATGATA AATGGCATAA AAAATCGTAA 100
<210> 7920
<211> 100
<212> DNA
<213> Escherichia coli K-12 MG1655

<220> chab b1218
<222> 132-231
<223> chab b1218 U00096 1271342..1271572
<313> 7920: 190-209
<400> 7920
TGACGCCAGT CGCGAAGAAA CCGCGCATAA AGTGGCCTGG GCTGCTGTGA  50
AGCATGAATA TGCCAAAGGG GATGATGATA AATGGCATAA AAAATCGTAA  100
<210> 7921
<211> 100
<212> DNA
<213> Escherichia coli K-12 MG1655
<220> chab b1218
<222> 129-228
<223> chab b1218 U00096 1271342..1271572
<313> 7921: 176-195
<400> 7921
TGATGACGCC AGTCGCGAAG AAACCGCGCA TAAAGTGGCC TGGGCTGCTG  50
TGAAGCATGA ATATGCCAAA GGGGATGATG ATAAATGGCA TAAAAAATCG  100
<210> 7922
<211> 100
<212> DNA
<213> Escherichia coli K-12 MG1655
<220> deda b2318
<222> 561-660
<223> deda b2318 U00096 complement(2432102..2432761)
<313> 7922: 616-635
<400> 7922
CCTTAAGCTG CTGATCGTCG GGATTATTGT GGTTTCTATT TTGCCGGGCG  50
TCATCGAAAT AATCCGTCAC AAACGCGCTG CGGCACGCGC CGCAAAATAA  100
<210> 7923
<211> 100
<212> DNA
<213> Escherichia coli K-12 MG1655
<220> deda b2318
<222> 514-613
<223> deda b2318 U00096 complement(2432102..2432761)
<313> 7923: 563-582
<400> 7923
ACCTACGCAG GCTATTTCTT CGGTACAATC CCGATGGTTC AGGATAACCT  50
TAAGCTGCTG ATCGTCGGGA TTATTGTGGT TTCTATTTTG CCGGGCGTCA  100
<210> 7924
<211> 100
<212> DNA
<213> Escherichia coli K-12 MG1655
<220> deda b2318
<222> 469-568
<223> deda b2318 U00096 complement(2432102..2432761)
<313> 7924: 508-527
<400> 7924
GCCGCTTATA ACGTGATCGG CGCACTGTTG TGGGTACTGC TTTTTACCTA  50
CGCAGGCTAT TTCTTCGGTA CAATCCCGAT GGTTCAGGAT AACCTTAAGC  100
<210> 7925
<211> 100
<212> DNA
<213> Escherichia coli K-12 MG1655
<220> ydfk ydfK
<222> 142-241
<223> ydfk ydfK U00096 1631063..1631329
<313> 7925: 197-216
<400> 7925
ACCAGAACAT TGCTTGATTA CATTGAAGGA AACATAAAGA AAAAATCATG  50
GCTGGATAAC AAAGAATTAT TACAAACAGC GATATCAGTT CTTAAAGACA  100
<210> 7926
<211> 100
<212> DNA
<213> Escherichia coli K-12 MG1655
<220> b1741 b1741
<222> 789-888
<223> b1741 b1741 U00096 1821539..1822426
<313> 7926: 863-882
<400> 7926

GTTTGATCAC GACGGTTATA AAATTCTTTG TAAGCCGCTG CTTTCCGGTA 50
ACTATGAAAT TACTGAACTT GATCCGGCGA ATGACCAGCG AGCCAGTTGA 100
<210> 7927
<211> 100
<212> DNA
<213> Escherichia coli K-12 MG1655
<220> b1741 b1741
<222> 743-842
<223> b1741 b1741 U00096 1821539..1822426
<313> 7927: 797-816
<400> 7927
TTAATTCGCT GGAAGAAGCG ACAACGTTAA TTCGGACACC CGCCGGGTTT 50
GATCACGACG GTTATAAAAT TCTTTGTAAG CCGCTGCTTT CCGGTAACTA 100
<210> 7928
<211> 100
<212> DNA
<213> Escherichia coli K-12 MG1655
<220> b1741 b1741
<222> 713-812
<223> b1741 b1741 U00096 1821539..1822426
<313> 7928: 750-769
<400> 7928
TTATTCAAAA CTGGCTGTGG CTGGGGGCGG TTAATTCGCT GGAAGAAGCG 50
ACAACGTTAA TTCGGACACC CGCCGGGTTT GATCACGACG GTTATAAAAT 100
<210> 7929
<211> 100
<212> DNA
<213> Escherichia coli K-12 MG1655
<220> chac b1219
<222> 584-683
<223> chac b1219 U00096 1271709..1272425
<313> 7929: 618-637
<400> 7929
ACCTGTTTTC ACTGGAACAG GAGCTTATCA AACTGGGAAT GCAGGACGAT 50
GGGCTGAATG ATTTGCTGGT ATCGGTAAAA AAACTGCTGG CGGAGAATTT 100
<210> 7930
<211> 100
<212> DNA
<213> Escherichia coli K-12 MG1655
<220> chac b1219
<222> 536-635
<223> chac b1219 U00096 1271709..1272425
<313> 7930: 561-580
<400> 7930
CGCCGTTGAT TGCGGCGGCG AGCGGTCCGC TGGGAACCAA CGCACAATAC 50
CTGTTTTCAC TGGAACAGGA GCTTATCAAA CTGGGAATGC AGGACGATGG 100
<210> 7931
<211> 100
<212> DNA
<213> Escherichia coli K-12 MG1655
<220> chac b1219
<222> 478-577
<223> chac b1219 U00096 1271709..1272425
<313> 7931: 527-546
<400> 7931
TTTATTATGG ACCCGCGACA TCCAGAATAT GAATCTGATA CTCGCGCTCA 50
GGTCATCGCG CCGTTGATTG CGGCGGCGAG CGGTCCGCTG GGAACCAACG 100
<210> 7932
<211> 100
<212> DNA
<213> Escherichia coli K-12 MG1655
<220> ebga b3077
<222> 2962-3061
<223> ebga b3077 U00096 3220238..3223366
<313> 7932: 3017-3036
<400> 7932
TGGGGCAGCG AGGTGCTGGA CTCCTGGCGC GTCTGGTTCC GTGACTTCAG 50
CTACGGCTTT ACGTTGCTGC CGGTTTCTGG CGGAGAAGCT ACCGCGCAAA 100
<210> 7933
<211> 100
<212> DNA

<213> Escherichia coli K-12 MG1655
<220> ebga b3077
<222> 2840-2939
<223> ebga b3077 U00096 3220238..3223366
<313> 7933: 2869-2888
<400> 7933
TCAGCGCCTG GCACTATACC CAGGAAAACA TCCACGCTGC CCAGCACTGT  50
AACGAGCTGC AGCGCAGTGA TGACATCACC CTGAACCTCG ATCACCAGCT  100
<210> 7934
<211> 100
<212> DNA
<213> Escherichia coli K-12 MG1655
<220> ebga b3077
<222> 2810-2909
<223> ebga b3077 U00096 3220238..3223366
<313> 7934: 2830-2849
<400> 7934
TGCTGGTGGT TCCGCAGCGC CCAATTAACT TCAGCGCCTG GCACTATACC  50
CAGGAAAACA TCCACGCTGC CCAGCACTGT AACGAGCTGC AGCGCAGTGA  100
<210> 7935
<211> 100
<212> DNA
<213> Escherichia coli K-12 MG1655
<220> b1742 b1743
<222> 540-639
<223> b1742 b1743 U00096 complement(1822386..1823024)
<313> 7935: 614-633
<400> 7935
CGATGGCAGG CACACCCTGC GGCTTTTACA ACCGCAAGGT AAACTGTTGT  50
TCAGTGAAAT CAACTGGCTC GCTGGTCATT CGCCGGATCA AGTTCAGTAA  100
<210> 7936
<211> 100
<212> DNA
<213> Escherichia coli K-12 MG1655
<220> b1742 b1743
<222> 527-626
<223> b1742 b1743 U00096 complement(1822386..1823024)
<313> 7936: 562-581
<400> 7936
GCGCGTGCTG GTTCGATGGC AGGCACACCC TGCGGCTTTT ACAACCGCAA  50
GGTAAACTGT TGTTCAGTGA AATCAACTGG CTCGCTGGTC ATTCGCCGGA  100
<210> 7937
<211> 100
<212> DNA
<213> Escherichia coli K-12 MG1655
<220> b1742 b1743
<222> 430-529
<223> b1742 b1743 U00096 complement(1822386..1823024)
<313> 7937: 486-505
<400> 7937
GAACGTACGT TTACTACCTT TGGCTCACGC GGCGGGGTGG TGTTTGTGAT  50
TAACGGGGCG TGGCAACTGG GCGATAAATT GCTCACTACC GACCAGGGCG  100
<210> 7938
<211> 100
<212> DNA
<213> Escherichia coli K-12 MG1655
<220> ydfm ydfM
<222> 178-277
<223> ydfm ydfM U00096 complement(1632334..1632909)
<313> 7938: 221-240
<400> 7938
AGTGATGAGT CATCGTGGCA TCTCGTTGAA GATCATCGGG GTAAAACGGT  50
TTATGACGTG GCTTCCGGCG ACGCGTTATT TATTTCTGAA CTCGGTCCGT  100
<210> 7939
<211> 100
<212> DNA
<213> Escherichia coli K-12 MG1655
<220> dedd b2315
<222> 528-627
<223> dedd b2315 U00096 complement(2429042..2429677)
<313> 7939: 572-591

<400> 7939
TATTCTGGTT GGGCCGGATG CCTCGAAAGA TAAGCTGAAA GGTTCGCTGG 50
GTGAGTTGAA GCAACTTTCT GGCTTAAGTG GCGTGGTAAT GGGCTATACG 100
<210> 7940
<211> 100
<212> DNA
<213> Escherichia coli K-12 MG1655
<220> dedd b2315
<222> 511-610
<223> dedd b2315 U00096 complement(2429042..2429677)
<313> 7940: 535-554
<400> 7940
CAGGGTAAAA TTACCCGTAT TCTGGTTGGG CCGGATGCCT CGAAAGATAA 50
GCTGAAAGGT TCGCTGGGTG AGTTGAAGCA ACTTTCTGGC TTAAGTGGCG 100
<210> 7941
<211> 100
<212> DNA
<213> Escherichia coli K-12 MG1655
<220> dedd b2315
<222> 469-568
<223> dedd b2315 U00096 complement(2429042..2429677)
<313> 7941: 501-520
<400> 7941
GGTGCCGGTT ATCGGGTTTA TACGTCGCCA TCCACGCCAG TGCAGGGTAA 50
AATTACCCGT ATTCTGGTTG GGCCGGATGC CTCGAAAGAT AAGCTGAAAG 100
<210> 7942
<211> 100
<212> DNA
<213> Escherichia coli K-12 MG1655
<220> ebgc b3078
<222> 351-450
<223> ebgc b3078 U00096 3223363..3223812
<313> 7942: 409-428
<400> 7942
TTGCGATATC CATGAAGCGT ATCGGTTTAT CTGCAATAAC GCGGTCAAAA 50
AAGTGGTTCT CAAAGTCACC ATCGAAGATG GTTATTTCCA TAACAAATAA 100
<210> 7943
<211> 100
<212> DNA
<213> Escherichia coli K-12 MG1655
<220> ebgc b3078
<222> 305-404
<223> ebgc b3078 U00096 3223363..3223812
<313> 7943: 349-368
<400> 7943
AAGGCTGCGG AGAAACCGTT GAGGTCCACG AAGGGCAAAT CGTTATTTGC 50
GATATCCATG AAGCGTATCG GTTTATCTGC AATAACGCGG TCAAAAAAGT 100
<210> 7944
<211> 100
<212> DNA
<213> Escherichia coli K-12 MG1655
<220> ebgc b3078
<222> 248-347
<223> ebgc b3078 U00096 3223363..3223812
<313> 7944: 302-321
<400> 7944
AAGAGACATT ACAGGTAGTG GAATATTATC GTGATGAAAC TGACCGTGAA 50
TATTTAAAAG GCTGCGGAGA AACCGTTGAG GTCCACGAAG GGCAAATCGT 100
<210> 7945
<211> 100
<212> DNA
<213> Escherichia coli K-12 MG1655
<220> ydfo ydfO
<222> 327-426
<223> ydfo ydfO U00096 1635056..1635481
<313> 7945: 401-420
<400> 7945
TACAAATATC CACGAACAAA AAAGATATTA CTATACCTTT GATAATTCAT 50
TACTATTTAC TGAAAGCATC CAGAAAACTA CACAGATCTT ACCACGCTAA 100
<210> 7946
<211> 100

<212> DNA
<213> Escherichia coli K-12 MG1655
<220> ydfo ydfO
<222> 302-401
<223> ydfo ydfO U00096 1635056..1635481
<313> 7946: 325-344
<400> 7946
CTAAAGCAGG AGTTTTTCGG TGGGTTACAA ATATCCACGA ACAAAAAAGA 50
TATTACTATA CCTTTGATAA TTCATTACTA TTTACTGAAA GCATCCAGAA 100
<210> 7947
<211> 100
<212> DNA
<213> Escherichia coli K-12 MG1655
<220> ydfo ydfO
<222> 268-367
<223> ydfo ydfO U00096 1635056..1635481
<313> 7947: 311-330
<400> 7947
GAGATCACAT TTCAGGAATA CAGAAGAAAC CTTGCTAAAG CAGGAGTTTT 50
TCGGTGGGTT ACAAATATCC ACGAACAAAA AAGATATTAC TATACCTTTG 100
<210> 7948
<211> 100
<212> DNA
<213> Escherichia coli K-12 MG1655
<220> b1745 b1745
<222> 1245-1344
<223> b1745 b1745 U00096 complement(1824940..1826283)
<313> 7948: 1306-1325
<400> 7948
ATTGCTGCGC GAAGGGCGGG AAGCACTGGA TGTATTGAGC CAATTACTGA 50
ATCTCGGTTC GGTTTATCCG TTCCAGCGCG AGGGAGGGGG CAATGGATAA 100
<210> 7949
<211> 100
<212> DNA
<213> Escherichia coli K-12 MG1655
<220> b1745 b1745
<222> 1219-1318
<223> b1745 b1745 U00096 complement(1824940..1826283)
<313> 7949: 1266-1285
<400> 7949
ACTGCTGCCG ATCTGGCCGA CCCGCAATTG CTGCGCGAAG GGCGGGAAGC 50
ACTGGATGTA TTGAGCCAAT TACTGAATCT CGGTTCGGTT TATCCGTTCC 100
<210> 7950
<211> 100
<212> DNA
<213> Escherichia coli K-12 MG1655
<220> b1745 b1745
<222> 1121-1220
<223> b1745 b1745 U00096 complement(1824940..1826283)
<313> 7950: 1176-1195
<400> 7950
AAGAAGAACG CCGGGCGGTG AATCCGGCGG TGATGATGAA CGATACGCTG 50
TTTAATGCGC TCAATGACTG GGTGGATCGT TACTACCGCG ATCGCCTTAC 100
<210> 7951
<211> 100
<212> DNA
<213> Escherichia coli K-12 MG1655
<220> b1746 b1746
<222> 1376-1475
<223> b1746 b1746 U00096 complement(1826280..1827758)
<313> 7951: 1423-1442
<400> 7951
CAGATTACTG CGCATGGCCG ATGGCGAGCC TGGAGTCGGA CTCGTTAACA 50
TTGCCCGCCA CGCTTAACCC CGGGCTGGAT TTTTCCGATG AGGTGGTGCG 100
<210> 7952
<211> 100
<212> DNA
<213> Escherichia coli K-12 MG1655
<220> b1746 b1746
<222> 1224-1323
<223> b1746 b1746 U00096 complement(1826280..1827758)

<313> 7952: 1282-1301
<400> 7952
TTCCCCCGAG CGGGAAAAGT TCGATCAACT GTTGCTGGAG GCGCGGGCGG 50
GGATTGTTAA CTGGAACAAA CCGCTTACCG GTGCTGCCAG TACCGCGCCA 100
<210> 7953
<211> 100
<212> DNA
<213> Escherichia coli K-12 MG1655
<220> b1746 b1746
<222> 1153-1252
<223> b1746 b1746 U00096 complement(1826280..1827758)
<313> 7953: 1196-1215
<400> 7953
CGTTATGATA CTTTCGATGA AGCGATTCGA ATGGCGAATA ACACTCGCTT 50
CGGACTCTCT TGCGGTCTGG TTTCCCCCGA GCGGGAAAAG TTCGATCAAC 100
<210> 7954
<211> 100
<212> DNA
<213> Escherichia coli K-12 MG1655
<220> b2475 b2476
<222> 765-864
<223> b2475 b2476 U00096 complement(2593894..2594757)
<313> 7954: 835-854
<400> 7954
TTTCACTCAT GGCACTTCTC AGCAACGCTA CAGCTGGTTT AAACGTGGTT 50
TCGACAGCGG CGATCCGGCA CAATGCAATA CTTTTGGTAA AAGCATTTAA 100
<210> 7955
<211> 100
<212> DNA
<213> Escherichia coli K-12 MG1655
<220> b2475 b2476
<222> 721-820
<223> b2475 b2476 U00096 complement(2593894..2594757)
<313> 7955: 774-793
<400> 7955
GACCGTTTAC AACAGCAAAG TCAGGGGCGA GTAGTACCAG ACAGTTTCAC 50
TCATGGCACT TCTCAGCAAC GCTACAGCTG GTTTAAACGT GGTTTCGACA 100
<210> 7956
<211> 100
<212> DNA
<213> Escherichia coli K-12 MG1655
<220> b2475 b2476
<222> 702-801
<223> b2475 b2476 U00096 complement(2593894..2594757)
<313> 7956: 736-755
<400> 7956
GGCGCAGGCC ATCGGCGATG ACCGTTTACA ACAGCAAAGT CAGGGGCGAG 50
TAGTACCAGA CAGTTTCACT CATGGCACTT CTCAGCAACG CTACAGCTGG 100
<210> 7957
<211> 100
<212> DNA
<213> Escherichia coli K-12 MG1655
<220> ysha b3876
<222> 594-693
<223> ysha b3876 U00096 complement(4061182..4061874)
<313> 7957: 657-676
<400> 7957
CTGGCTCCCT TATTTCGAAT TGCGCTGGTT AGACCGCAAC GTCGAACCGT 50
ACCACCGCGA GCAAAACCAG ATCCGTATCG GGACGAAGTA TTTCTTCTGA 100
<210> 7958
<211> 100
<212> DNA
<213> Escherichia coli K-12 MG1655
<220> ysha b3876
<222> 589-688
<223> ysha b3876 U00096 complement(4061182..4061874)
<313> 7958: 618-637
<400> 7958
GAACACTGGC TCCCTTATTT CGAATTGCGC TGGTTAGACC GCAACGTCGA 50
ACCGTACCAC CGCGAGCAAA ACCAGATCCG TATCGGGACG AAGTATTTCT 100
<210> 7959

<211> 100
<212> DNA
<213> Escherichia coli K-12 MG1655
<220> ysha b3876
<222> 518-617
<223> ysha b3876 U00096 complement(4061182..4061874)
<313> 7959: 583-602
<400> 7959
ATGACTTTAA TAGTAGCAAC GGGAAAGATC ATCATTGGGA AATCACTAAC 50
ACCTTCCGTT ACCGTATTAA TGAACACTGG CTCCCTTATT TCGAATTGCG 100
<210> 7960
<211> 100
<212> DNA
<213> Escherichia coli K-12 MG1655
<220> b1747 b1748
<222> 921-1020
<223> b1747 b1748 U00096 complement(1827755..1828789)
<313> 7960: 979-998
<400> 7960
CGATCCGGCA ACCGAGCGTT TGATTTTAAC CGCCGCACAA CTGGATGCCC 50
TCAAATGCCA CGCCGGGGAT CGCGTTCGTC TGGTGCGCCT GTGCGCAGAG 100
<210> 7961
<211> 100
<212> DNA
<213> Escherichia coli K-12 MG1655
<220> b1747 b1748
<222> 832-931
<223> b1747 b1748 U00096 complement(1827755..1828789)
<313> 7961: 877-896
<400> 7961
GCAGAAGGGC AGCCTGCGCA GGGCGATTTC CCAGCCTGCC TGGTCGCCAA 50
TGAAAATTAT CACCATTTCC GCGTGGTGCT GGTGCGTACC GATCCGGCAA 100
<210> 7962
<211> 100
<212> DNA
<213> Escherichia coli K-12 MG1655
<220> b1747 b1748
<222> 698-797
<223> b1747 b1748 U00096 complement(1827755..1828789)
<313> 7962: 749-768
<400> 7962
CGCCTGCCCG CGCGGTGCTG GAGAAAGAAG GTTTTCGCTA CCGTAACTAT 50
ATCGACATCT TTGACGGTGG GCCGACGCTT GAGTGTGACA TCGACCGCGT 100
<210> 7963
<211> 100
<212> DNA
<213> Escherichia coli K-12 MG1655
<220> miaa b4172
<222> 852-951
<223> miaa b4172 U00096 4396830..4397780
<313> 7963: 920-939
<400> 7963
CTGGCTGCGT GGTTGGGAAG GGGTTCACTG GCTTGACAGT GAAAAACCAG 50
AACAGGCGCG TGACGAAGTA TTACAGGTTG TTGGTGCTAT CGCAGGCTGA 100
<210> 7964
<211> 100
<212> DNA
<213> Escherichia coli K-12 MG1655
<220> miaa b4172
<222> 807-906
<223> miaa b4172 U00096 4396830..4397780
<313> 7964: 832-851
<400> 7964
TCGAGGTGTT TGCGCCACGA GACAGTTGGC GAAGCGGCAG ATAACCTGGC 50
TGCGTGGTTG GGAAGGGGTT CACTGGCTTG ACAGTGAAAA ACCAGAACAG 100
<210> 7965
<211> 100
<212> DNA
<213> Escherichia coli K-12 MG1655
<220> miaa b4172
<222> 744-843

<223> miaa b4172 U00096 4396830..4397780
<313> 7965: 763-782
<400> 7965
CGTGGGTTAT CGCCAGATGT GGTCTTACCT TGAAGGCGAA ATCTCATACG  50
ATGAAATGGT TTATCGAGGT GTTTGCGCCA CGAGACAGTT GGCGAAGCGG  100
<210> 7966
<211> 100
<212> DNA
<213> Escherichia coli K-12 MG1655
<220> ebgr b3076
<222> 883-982
<223> ebgr b3076 U00096 3219107..3220090
<313> 7966: 930-949
<400> 7966
CAGGGCGTTA ACCTGGTGTA TGAAAAAGCC CGCGATGGTC GCGCGCTGCC  50
GCTGTTAGTC TTCGTTCCCA GCAAATTAAA ACTGCGCGGC ACGACCCGTT  100
<210> 7967
<211> 100
<212> DNA
<213> Escherichia coli K-12 MG1655
<220> ebgr b3076
<222> 862-961
<223> ebgr b3076 U00096 3219107..3220090
<313> 7967: 893-912
<400> 7967
CATTCCGAGA TGATGGGAAG TCAGGGCGTT AACCTGGTGT ATGAAAAAGC  50
CCGCGATGGT CGCGCGCTGC CGCTGTTAGT CTTCGTTCCC AGCAAATTAA  100
<210> 7968
<211> 100
<212> DNA
<213> Escherichia coli K-12 MG1655
<220> ebgr b3076
<222> 751-850
<223> ebgr b3076 U00096 3219107..3220090
<313> 7968: 781-800
<400> 7968
CGGGCAATTC ATGAACGAGG CCTGAACATC CCACAGGATA TTTCGCTTAT  50
CAGCGTTAAC GATATCCCCA CCGCGCGATT TACCTTTCCG CCGCTCTCCA  100
<210> 7969
<211> 100
<212> DNA
<213> Escherichia coli K-12 MG1655
<220> ydga b1615
<222> 1410-1509
<223> ydga b1615 U00096 1687876..1689384
<313> 7969: 1480-1499
<400> 7969
AACGTTAAAT GGGCAGAAAA TGTCGTTAGA AGATTTTGTT GGCATGTTTG  50
CGATGCCAGC TCTTAACGTT CCGGCTGTAC CGGCAATTCC GCAGCAGTAA  100
<210> 7970
<211> 100
<212> DNA
<213> Escherichia coli K-12 MG1655
<220> ydga b1615
<222> 1351-1450
<223> ydga b1615 U00096 1687876..1689384
<313> 7970: 1378-1397
<400> 7970
CTGACCACCT TGCAGGACAA TACCATCACC ACCAGCCTGC AATATGCTAA  50
CGGTCAGATA ACGTTAAATG GGCAGAAAAT GTCGTTAGAA GATTTTGTTG  100
<210> 7971
<211> 100
<212> DNA
<213> Escherichia coli K-12 MG1655
<220> ydga b1615
<222> 1237-1336
<223> ydga b1615 U00096 1687876..1689384
<313> 7971: 1292-1311
<400> 7971
GCAACTGAGT TTATGACTCA GGTAGCGAAG CTGGAAGGTT ATCAGGAAGA  50
TCAAGCGAAA AAACTGGCGA AACAGCAAGT TGAAGGTGCA TCAGCAATGG  100

<210> 7972
<211> 100
<212> DNA
<213> Escherichia coli K-12 MG1655
<220> ydgb b1607
<222> 624-723
<223> ydgb b1607 U00096 1679000..1679722
<313> 7972: 687-706
<400> 7972
TGGCGAGAAA GAAGTGATCG ACCTGGTCGA TTACTTACTT ACCAGTTGCT 50
TTGTCACCGG ACGCAGTTTC CCACTTGATG GCGGTCGTCA TCTGCGTTAA 100
<210> 7973
<211> 100
<212> DNA
<213> Escherichia coli K-12 MG1655
<220> ydgb b1607
<222> 576-675
<223> ydgb b1607 U00096 1679000..1679722
<313> 7973: 615-634
<400> 7973
CGAATATCGA CAACAGGCGC TGAATAAATC ACTGATGAAA ACCGCGCCTG 50
GCGAGAAAGA AGTGATCGAC CTGGTCGATT ACTTACTTAC CAGTTGCTTT 100
<210> 7974
<211> 100
<212> DNA
<213> Escherichia coli K-12 MG1655
<220> ydgb b1607
<222> 530-629
<223> ydgb b1607 U00096 1679000..1679722
<313> 7974: 576-595
<400> 7974
ATTCTATTGC GCCATCGCTG ATCCTGTTTA ATGAACATGA TGATGCCGAA 50
TATCGACAAC AGGCGCTGAA TAAATCACTG ATGAAAACCG CGCCTGGCGA 100
<210> 7975
<211> 100
<212> DNA
<213> Escherichia coli K-12 MG1655
<220> ydgc b1608
<222> 237-336
<223> ydgc b1608 U00096 complement(1679719..1680054)
<313> 7975: 305-324
<400> 7975
CACCGGAATG ATGCGCTTGC CCGCCGCTTT TGTTGGTTCT GTTGCGTGTT 50
GGGGGATAAG CGCATGGGTG TTGATTATAT GCTGGATAAA GCTGCATTAA 100
<210> 7976
<211> 100
<212> DNA
<213> Escherichia coli K-12 MG1655
<220> ydgc b1608
<222> 236-335
<223> ydgc b1608 U00096 complement(1679719..1680054)
<313> 7976: 261-280
<400> 7976
TCACCGGAAT GATGCGCTTG CCCGCCGCTT TTGTTGGTTC TGTTGCGTGT 50
TGGGGGATAA GCGCATGGGT GTTGATTATA TGCTGGATAA AGCTGCATTA 100
<210> 7977
<211> 100
<212> DNA
<213> Escherichia coli K-12 MG1655
<220> ydgc b1608
<222> 113-212
<223> ydgc b1608 U00096 complement(1679719..1680054)
<313> 7977: 162-181
<400> 7977
TTGCGCTTAT CGCGCATTAT ATTGTTGCCA GCGAACGCGG CATTGAAGCC 50
TTACGCGCAA CCATCATTTT TAGTATGTGG TCGATTATTC CCTATTTTGT 100
<210> 7978
<211> 100
<212> DNA
<213> Escherichia coli K-12 MG1655
<220> pola b3864

<222> 2688-2787
<223> pola b3864 U00096 4044546..4047332
<313> 7978: 2766-2785
<400> 7978
CGCGAAGCAG ATTCATCAAC TGATGGAAAA CTGTACCCGT CTGGATGTGC 50
CGTTGCTGGT GGAAGTGGGG AGTGGCGAAA ACTGGGATCA GGCGCACTAA 100
<210> 7979
<211> 100
<212> DNA
<213> Escherichia coli K-12 MG1655
<220> pola b3864
<222> 2533-2632
<223> pola b3864 U00096 4044546..4047332
<313> 7979: 2578-2597
<400> 7979
AACGCGCCAA TGCAGGGAAC CGCCGCCGAC ATTATCAAAC GGGCGATGAT 50
TGCCGTTGAT GCGTGGTTAC AGGCTGAGCA ACCGCGTGTA CGTATGATCA 100
<210> 7980
<211> 100
<212> DNA
<213> Escherichia coli K-12 MG1655
<220> pola b3864
<222> 2485-2584
<223> pola b3864 U00096 4044546..4047332
<313> 7980: 2534-2553
<400> 7980
AAATCCAGCA ATGGTGCTCG TCGTGCAGCG GCTGAACGTG CAGCCATTAA 50
CGCGCCAATG CAGGGAACCG CCGCCGACAT TATCAAACGG GCGATGATTG 100
<210> 7981
<211> 100
<212> DNA
<213> Escherichia coli K-12 MG1655
<220> polb b0061
<222> 2253-2352
<223> polb b0061 U00096 complement(63429..65780)
<313> 7981: 2327-2346
<400> 7981
CTATCTGACC CGCCAGCTAC AACCCGTGGC GGAGGGAATA CTCCCTTTTA 50
TTGAGGATAA TTTTGCTACA CTTATGACCG GGCAACTTGG GCTATTTTGA 100
<210> 7982
<211> 100
<212> DNA
<213> Escherichia coli K-12 MG1655
<220> polb b0061
<222> 2209-2308
<223> polb b0061 U00096 complement(63429..65780)
<313> 7982: 2257-2276
<400> 7982
CCGGAGCCGC TGGACTACCA ACGTTCACCA CTGGATTACG AACACTATCT 50
GACCCGCCAG CTACAACCCG TGGCGGAGGG AATACTCCCT TTTATTGAGG 100
<210> 7983
<211> 100
<212> DNA
<213> Escherichia coli K-12 MG1655
<220> polb b0061
<222> 2170-2269
<223> polb b0061 U00096 complement(63429..65780)
<313> 7983: 2202-2221
<400> 7983
AATCGCGGCA CCATTAAGTA CGTATGGACC ACCAACGGCC CGGAGCCGCT 50
GGACTACCAA CGTTCACCAC TGGATTACGA ACACTATCTG ACCCGCCAGC 100
<210> 7984
<211> 100
<212> DNA
<213> Escherichia coli K-12 MG1655
<220> lspa b0028
<222> 373-472
<223> lspa b0028 U00096 25207..25701
<313> 7984: 414-433
<400> 7984
ATCGACTTCT ACGTCGGCGA CTGGCACTTC GCCACCTTCA ACCTTGCCGA 50

TACTGCCATC TGTGTCGGTG CGGCACTGAT TGTGCTGGAA GGTTTTTTGC 100
<210> 7985
<211> 100
<212> DNA
<213> Escherichia coli K-12 MG1655
<220> lspa b0028
<222> 312-411
<223> lspa b0028 U00096 25207..25701
<313> 7985: 337-356
<400> 7985
TATTGGCGGC GCGCTGGGCA ACCTGTTCGA CCGCCTGTGG CACGGCTTCG 50
TTGTCGATAT GATCGACTTC TACGTCGGCG ACTGGCACTT CGCCACCTTC 100
<210> 7986
<211> 100
<212> DNA
<213> Escherichia coli K-12 MG1655
<220> lspa b0028
<222> 254-353
<223> lspa b0028 U00096 25207..25701
<313> 7986: 291-310
<400> 7986
TGATGATGTA TCGCTCGAAG GCCACGCAGA AGCTAAACAA TATCGCTTAC 50
GCGCTGATTA TTGGCGGCGC GCTGGGCAAC CTGTTCGACC GCCTGTGGCA 100
<210> 7987
<211> 100
<212> DNA
<213> Escherichia coli K-12 MG1655
<220> b1754 b1754
<222> 1071-1170
<223> b1754 b1754 U00096 1834094..1835263
<313> 7987: 1145-1164
<400> 7987
AAGAATGCCG CAGGATCTGC CGCCGGTACT GGCTGAACCG CACGCAGGTT 50
GGGTAAATGC GCTGGAACAA GAATGGCTAC ACCGTTACGG TACGCATTAA 100
<210> 7988
<211> 100
<212> DNA
<213> Escherichia coli K-12 MG1655
<220> b1754 b1754
<222> 991-1090
<223> b1754 b1754 U00096 1834094..1835263
<313> 7988: 1010-1029
<400> 7988
GCAGATCCCG CTGTCTGGGG CGATCCTTCT GTTCTCGATC CGCAAAAACT 50
GCCTGACGGG CAGCGCGAAT CATTGCAATC AAGAATGCCG CAGGATCTGC 100
<210> 7989
<211> 100
<212> DNA
<213> Escherichia coli K-12 MG1655
<220> b1754 b1754
<222> 877-976
<223> b1754 b1754 U00096 1834094..1835263
<313> 7989: 908-927
<400> 7989
GAGGGGATGA TTGGCAACGT GCATTTCGTC ACCATTCCTG CCAACGCGAA 50
TGCCAGTGCT GCGGCGAAGG TAGTTGCCAA TTTCCTGCTC TCACCCGATG 100
<210> 7990
<211> 100
<212> DNA
<213> Escherichia coli K-12 MG1655
<220> ydgo ydgO
<222> 912-1011
<223> ydgo ydgO U00096 1707166..1708224
<313> 7990: 952-971
<400> 7990
CGCGCTTGCG GGCTTATTAG TCTGGTTGAT CCGCAGTTTC GGCGGCTATC 50
CTGACGGCGT GGCTTTTGCC GTCCTGCTGG CGAACATCAC GGTTCCTCTG 100
<210> 7991
<211> 100
<212> DNA
<213> Escherichia coli K-12 MG1655

<220> ydgo ydgO
<222> 840-939
<223> ydgo ydgO U00096 1707166..1708224
<313> 7991: 855-874
<400> 7991
GCTCGGCGCA TTCTTTATTT TGACTGACCC GGTTACCGCT TCTACGACCA 50
ATCGTGGTCG TCTTATTTTC GGCGCGCTTG CGGGCTTATT AGTCTGGTTG 100
<210> 7992
<211> 100
<212> DNA
<213> Escherichia coli K-12 MG1655
<220> ydgo ydgO
<222> 778-877
<223> ydgo ydgO U00096 1707166..1708224
<313> 7992: 832-851
<400> 7992
TGGTTGTTCT CACCAGAAAC ACTGGCAGCA CCGCAAATTC ATCTGCTGTC 50
TGGAGCGACC ATGCTCGGCG CATTCTTTAT TTTGACTGAC CCGGTTACCG 100
<210> 7993
<211> 100
<212> DNA
<213> Escherichia coli K-12 MG1655
<220> b1755 b1755
<222> 1392-1491
<223> b1755 b1755 U00096 1835281..1836771
<313> 7993: 1453-1472
<400> 7993
CGCCCAGGCT TTATGGCAAC TGCTATTACC GCTTATTATT TTTGCCCTGA 50
CCGCGTTAGT CGCAAAATGG GTAGGTTATG TCAGACAAGG ACTCCGCTAA 100
<210> 7994
<211> 100
<212> DNA
<213> Escherichia coli K-12 MG1655
<220> b1755 b1755
<222> 1366-1465
<223> b1755 b1755 U00096 1835281..1836771
<313> 7994: 1420-1439
<400> 7994
AGCGGCGGCA GCAACGGTAT TCTCGCCGCC CAGGCTTTAT GGCAACTGCT 50
ATTACCGCTT ATTATTTTTG CCCTGACCGC GTTAGTCGCA AAATGGGTAG 100
<210> 7995
<211> 100
<212> DNA
<213> Escherichia coli K-12 MG1655
<220> b1755 b1755
<222> 1342-1441
<223> b1755 b1755 U00096 1835281..1836771
<313> 7995: 1381-1400
<400> 7995
ACCACTGAGG CGGTGGCATT AAGCAGCGGC GGCAGCAACG GTATTCTCGC 50
CGCCCAGGCT TTATGGCAAC TGCTATTACC GCTTATTATT TTTGCCCTGA 100
<210> 7996
<211> 100
<212> DNA
<213> Escherichia coli K-12 MG1655
<220> b1756 b1756
<222> 555-654
<223> b1756 b1756 U00096 1836771..1837424
<313> 7996: 601-620
<400> 7996
CCCCGTCGTT CAGGTAACGC ACGATCTCCA GGATGTTCCT GCTGATAGTT 50
CTGTTCTGGA TATGGCGCAG TGGTCAGAAA ATTACAACAA ACTGCGATAA 100
<210> 7997
<211> 100
<212> DNA
<213> Escherichia coli K-12 MG1655
<220> b1756 b1756
<222> 536-635
<223> b1756 b1756 U00096 1836771..1837424
<313> 7997: 572-591
<400> 7997

AAGTTCGCGC CCTGGCGATC CCCGTCGTTC AGGTAACGCA CGATCTCCAG 50
GATGTTCCTG CTGATAGTTC TGTTCTGGAT ATGGCGCAGT GGTCAGAAAA 100
<210> 7998
<211> 100
<212> DNA
<213> Escherichia coli K-12 MG1655
<220> b1756 b1756
<222> 473-572
<223> b1756 b1756 U00096 1836771..1837424
<313> 7998: 510-529
<400> 7998
ATGAGCCATT CAGCCGTCTT GATGTGGCTC TGCGCGATAA TTTTCGCCAG 50
TGGGTGTTCA GCGAAGTTCG CGCCCTGGCG ATCCCCGTCG TTCAGGTAAC 100
<210> 7999
<211> 100
<212> DNA
<213> Escherichia coli K-12 MG1655
<220> ydgq b1633
<222> 597-696
<223> ydgq b1633 U00096 1708852..1709547
<313> 7999: 677-696
<400> 7999
GCTGGCAGGA AAATACCTGA TTGATGAAAG AATGAAAAAG CGCCGTGCTG 50
AAGCAGCTGC AGAACGTGCA TTGCCAAACG GTGAAACAGG GAATGTCTGA 100
<210> 8000
<211> 100
<212> DNA
<213> Escherichia coli K-12 MG1655
<220> ydgq b1633
<222> 581-680
<223> ydgq b1633 U00096 1708852..1709547
<313> 8000: 617-636
<400> 8000
TTGGCCTGGG ACTGATGCTG GCAGGAAAAT ACCTGATTGA TGAAAGAATG 50
AAAAAGCGCC GTGCTGAAGC AGCTGCAGAA CGTGCATTGC CAAACGGTGA 100
<210> 8001
<211> 100
<212> DNA
<213> Escherichia coli K-12 MG1655
<220> ydgq b1633
<222> 465-564
<223> ydgq b1633 U00096 1708852..1709547
<313> 8001: 487-506
<400> 8001
CACATTGTTT GACGGTGCAG ATGCGCTGTT AGGTAGCTGG GCAAAAGTAT 50
TACGCGTGGA GATTTTCCAC ACCGACTCCC CTTTCCTGCT GGCGATGCTG 100
<210> 8002
<211> 100
<212> DNA
<213> Escherichia coli K-12 MG1655
<220> b1757 b1757
<222> 1224-1323
<223> b1757 b1757 U00096 1837476..1838798
<313> 8002: 1259-1278
<400> 8002
GAAGAATGTT TCCGTGTATG ACGGCGGCTG GTACGAATGG AGCAGCGATC 50
CAAAAAATCC GGTAGCAACC GGTGAACGCG GCCCGGACAG TAGCAAATAA 100
<210> 8003
<211> 100
<212> DNA
<213> Escherichia coli K-12 MG1655
<220> b1757 b1757
<222> 1127-1226
<223> b1757 b1757 U00096 1837476..1838798
<313> 8003: 1155-1174
<400> 8003
AAGCGTGGAA TATCAAACCA GAGCAGCAAG TTTCATTCTA CTGCGGCACC 50
GGCTGGCGCG CGTCCGAAAC CTTTATGTAC GCACGCGCCA TGGGTTGGAA 100
<210> 8004
<211> 100
<212> DNA

<213> Escherichia coli K-12 MG1655
<220> b1757 b1757
<222> 1064-1163
<223> b1757 b1757 U00096 1837476..1838798
<313> 8004: 1115-1134
<400> 8004
TGGAAGATTT TCATAACCCG GATGGCACCA TGCGCAGCGC CGATGATATT 50
ACCGCTATGT GGAAAGCGTG GAATATCAAA CCAGAGCAGC AAGTTTCATT 100
<210> 8005
<211> 100
<212> DNA
<213> Escherichia coli K-12 MG1655
<220> ydgr b1635
<222> 1389-1488
<223> ydgr b1635 U00096 1710793..1712295
<313> 8005: 1438-1457
<400> 8005
GATTGGTGTC GCTACTGCCG TTATTGCAGT ACTGATGCTG CTGACCGCGC 50
CGAAACTGCA CCGCATGACG CAGGATGACG CTGCAGACAA AGCGGCGAAA 100
<210> 8006
<211> 100
<212> DNA
<213> Escherichia coli K-12 MG1655
<220> ydgr b1635
<222> 1355-1454
<223> ydgr b1635 U00096 1710793..1712295
<313> 8006: 1386-1405
<400> 8006
TGTCACTGGA AGTCTATGGT CGCGTATTCT TGCAGATTGG TGTCGCTACT 50
GCCGTTATTG CAGTACTGAT GCTGCTGACC GCGCCGAAAC TGCACCGCAT 100
<210> 8007
<211> 100
<212> DNA
<213> Escherichia coli K-12 MG1655
<220> ydgr b1635
<222> 1276-1375
<223> ydgr b1635 U00096 1710793..1712295
<313> 8007: 1312-1331
<400> 8007
ACCACTGCCG GTGCAAACCT GATTGGTGGT TATGTTGCGG GTATGATGGC 50
TGTGCCGGAT AACGTTACCG ATCCGCTGAT GTCACTGGAA GTCTATGGTC 100
<210> 8008
<211> 100
<212> DNA
<213> Escherichia coli K-12 MG1655
<220> b1758 b1758
<222> 475-574
<223> b1758 b1758 U00096 complement(1838807..1839433)
<313> 8008: 515-534
<400> 8008
GGCACAGAAA CGATCTTACT GTTTGTGCTG GGATGTTTAT TCCCTGCATG 50
GTTTGCGTGG TTTGCGTGGA TCTTCGGTGC GTTGTGCTGG ATGACGACCT 100
<210> 8009
<211> 100
<212> DNA
<213> Escherichia coli K-12 MG1655
<220> b1758 b1758
<222> 444-543
<223> b1758 b1758 U00096 complement(1838807..1839433)
<313> 8009: 464-483
<400> 8009
GTCGTTTTAC TATCTGGGGG GATTAACCGA AGGCACAGAA ACGATCTTAC 50
TGTTTGTGCT GGGATGTTTA TTCCCTGCAT GGTTTGCGTG GTTTGCGTGG 100
<210> 8010
<211> 100
<212> DNA
<213> Escherichia coli K-12 MG1655
<220> b1758 b1758
<222> 374-473
<223> b1758 b1758 U00096 complement(1838807..1839433)
<313> 8010: 421-440

<400> 8010
GCAGCAGTTT TCTCGCCTTT GCTGCACTGG CAGCAAAACA TCAGATTGAT 50
AACCCCGGTT ATGCACATAA GTCGTTTTAC TATCTGGGGG GATTAACCGA 100
<210> 8011
<211> 100
<212> DNA
<213> Escherichia coli K-12 MG1655
<220> b1759 b1759
<222> 309-408
<223> b1759 b1759 U00096 1839514..1839921
<313> 8011: 384-403
<400> 8011
GTGCTCACCT GAAGAGGCGC TGCAATATCC GCTGGCCCCT GCTGACATTC 50
CATTATTAGA GGCGTTTATG GCTTTACGCG CCGCCAGACC AGCGGATTAG 100
<210> 8012
<211> 100
<212> DNA
<213> Escherichia coli K-12 MG1655
<220> b1759 b1759
<222> 296-395
<223> b1759 b1759 U00096 1839514..1839921
<313> 8012: 342-361
<400> 8012
AGGCGCTGGT CTGGTGCTCA CCTGAAGAGG CGCTGCAATA TCCGCTGGCC 50
CCTGCTGACA TTCCATTATT AGAGGCGTTT ATGGCTTTAC GCGCCGCCAG 100
<210> 8013
<211> 100
<212> DNA
<213> Escherichia coli K-12 MG1655
<220> b1759 b1759
<222> 241-340
<223> b1759 b1759 U00096 1839514..1839921
<313> 8013: 274-293
<400> 8013
CTTCATGCCT GGCACGTACC CGACTTCCAC GGGACGTTAC AGGCACATGA 50
ACATCAGGCG CTGGTCTGGT GCTCACCTGA AGAGGCGCTG CAATATCCGC 100
<210> 8014
<211> 100
<212> DNA
<213> Escherichia coli K-12 MG1655
<220> mara b1532
<222> 291-390
<223> mara b1532 U00096 1617592..1617981
<313> 8014: 344-363
<400> 8014
CTTCAAAAAT TACTTTGATG TTCCGCCGCA TAAATACCGG ATGACCAATA 50
TGCAGGGCGA ATCGCGCTTT TTACATCCAT TAAATCATTA CAACAGCTAG 100
<210> 8015
<211> 100
<212> DNA
<213> Escherichia coli K-12 MG1655
<220> mara b1532
<222> 252-351
<223> mara b1532 U00096 1617592..1617981
<313> 8015: 313-332
<400> 8015
ACGATATGGC TTCGAGTCGC AACAAACTCT GACCCGAACC TTCAAAAATT 50
ACTTTGATGT TCCGCCGCAT AAATACCGGA TGACCAATAT GCAGGGCGAA 100
<210> 8016
<211> 100
<212> DNA
<213> Escherichia coli K-12 MG1655
<220> mara b1532
<222> 244-343
<223> mara b1532 U00096 1617592..1617981
<313> 8016: 263-282
<400> 8016
CTGGCAGAAC GATATGGCTT CGAGTCGCAA CAAACTCTGA CCCGAACCTT 50
CAAAAATTAC TTTGATGTTC CGCCGCATAA ATACCGGATG ACCAATATGC 100
<210> 8017
<211> 100

<212> DNA
<213> Escherichia coli K-12 MG1655
<220> marb b1533
<222> 120-219
<223> marb b1533 U00096 1618013..1618231
<313> 8017: 151-170
<400> 8017
ATCGCAGGAA CACCCACCGT TTGATTTAAA TCACATGGGT ACTGGCAGTG 50
ATAAGTCGGA TGCGCTCGGC GTGCCCTATT ATAATCAACA CGCTATGTAG 100
<210> 8018
<211> 100
<212> DNA
<213> Escherichia coli K-12 MG1655
<220> marb b1533
<222> 52-151
<223> marb b1533 U00096 1618013..1618231
<313> 8018: 99-118
<400> 8018
CAGGGCGTTG CGGAACAAAC CACGCAGCCA GTTGTTACTT CTTGTGCCAA 50
TGTCGTGGTT GTTCCCCCAT CGCAGGAACA CCCACCGTTT GATTTAAATC 100
<210> 8019
<211> 100
<212> DNA
<213> Escherichia coli K-12 MG1655
<220> marb b1533
<222> 47-146
<223> marb b1533 U00096 1618013..1618231
<313> 8019: 72-91
<400> 8019
CCGCGCAGGG CGTTGCGGAA CAAACCACGC AGCCAGTTGT TACTTCTTGT 50
GCCAATGTCG TGGTTGTTCC CCCATCGCAG GAACACCCAC CGTTTGATTT 100
<210> 8020
<211> 100
<212> DNA
<213> Escherichia coli K-12 MG1655
<220> ydha ydhA
<222> 150-249
<223> ydha ydhA U00096 complement(1716090..1716338)
<313> 8020: 205-224
<400> 8020
CGGAATCTAT GTTTTCTGGT CGAAAGGCGA TGAAGCGACT GTCTATAAAC 50
GCGACCGCAT CGTCTTGAAT AACTGTCAGT TACAAAATCC ACAGCGTTGA 100
<210> 8021
<211> 100
<212> DNA
<213> Escherichia coli K-12 MG1655
<220> ydha ydhA
<222> 109-208
<223> ydha ydhA U00096 complement(1716090..1716338)
<313> 8021: 163-182
<400> 8021
CTCAAACAGG GCATTTCAGC CTCTGGCGCG CGTTACACTG ACGGAATCTA 50
TGTTTTCTGG TCGAAAGGCG ATGAAGCGAC TGTCTATAAA CGCGACCGCA 100
<210> 8022
<211> 100
<212> DNA
<213> Escherichia coli K-12 MG1655
<220> ydha ydhA
<222> 57-156
<223> ydha ydhA U00096 complement(1716090..1716338)
<313> 8022: 109-128
<400> 8022
GAATAATCCG CGCCAGGAGG TCAGTTTTGT TTACGATAAT CAACTACTGC 50
ATCTCAAACA GGGCATTTCA GCCTCTGGCG CGCGTTACAC TGACGGAATC 100
<210> 8023
<211> 100
<212> DNA
<213> Escherichia coli K-12 MG1655
<220> ydhb b1660
<222> 698-797
<223> ydhb b1660 U00096 complement(1736890..1737822)

<313> 8023: 749-768
<400> 8023
CAGGATTATG CATAGGGATG GTGCCAACAC ATTTCGCCAA ACCGTGGCTT 50
AATGAGGGGA AGTGGGTAGC ATTAGAACTG GAAAACCCCT TTCCGGATTC 100
<210> 8024
<211> 100
<212> DNA
<213> Escherichia coli K-12 MG1655
<220> ydhb b1660
<222> 623-722
<223> ydhb b1660 U00096 complement(1736890..1737822)
<313> 8024: 678-697
<400> 8024
GTATTACCTG GTTGCTGGAT AACCAAAAAA GAGTTGTCGT GCCTGACTGG 50
GAATCATCGG CAACCTGTAT CTCGGCAGGA TTATGCATAG GGATGGTGCC 100
<210> 8025
<211> 100
<212> DNA
<213> Escherichia coli K-12 MG1655
<220> ydhb b1660
<222> 619-718
<223> ydhb b1660 U00096 complement(1736890..1737822)
<313> 8025: 639-658
<400> 8025
AAACGTATTA CCTGGTTGCT GGATAACCAA AAAAGAGTTG TCGTGCCTGA 50
CTGGGAATCA TCGGCAACCT GTATCTCGGC AGGATTATGC ATAGGGATGG 100
<210> 8026
<211> 100
<212> DNA
<213> Escherichia coli K-12 MG1655
<220> ydhc b1661
<222> 1113-1212
<223> ydhc b1661 U00096 1737935..1739146
<313> 8026: 1191-1210
<400> 8026
AGTAATGCTG GTCGCGCTGG GTTACATGAT GCAACGTTGT GAAGAAGTTG 50
GCTGCCAGAA TCATGGCAAT GCCGAAGTCG CTCATAGCGA ATCACACTGA 100
<210> 8027
<211> 100
<212> DNA
<213> Escherichia coli K-12 MG1655
<220> ydhc b1661
<222> 1064-1163
<223> ydhc b1661 U00096 1737935..1739146
<313> 8027: 1113-1132
<400> 8027
TCAGTATCAG CACGCCATTG CTCACCACCA CCAGCGTGAT GTTATCAACA 50
GTAATGCTGG TCGCGCTGGG TTACATGATG CAACGTTGTG AAGAAGTTGG 100
<210> 8028
<211> 100
<212> DNA
<213> Escherichia coli K-12 MG1655
<220> ydhc b1661
<222> 1014-1113
<223> ydhc b1661 U00096 1737935..1739146
<313> 8028: 1028-1047
<400> 8028
TCTTCAACTG GGTCTGTGCT TCCTCGCAAG TCTGGTAGTT TCCTGGCTGA 50
TCAGTATCAG CACGCCATTG CTCACCACCA CCAGCGTGAT GTTATCAACA 100
<210> 8029
<211> 100
<212> DNA
<213> Escherichia coli K-12 MG1655
<220> ydhd b1655
<222> 249-348
<223> ydhd b1655 U00096 complement(1731778..1732125)
<313> 8029: 328-347
<400> 8029
TTGTGATATC GTGATCGAAA TGTATCAGCG TGGCGAACTG CAGCAGCTGA 50
TCAAAGAAAC TGCCGCTAAA TACAAGTCTG AAGAGCCGGA CGCGGAATAA 100
<210> 8030

<211> 100
<212> DNA
<213> Escherichia coli K-12 MG1655
<220> ydhd b1655
<222> 157-256
<223> ydhd b1655 U00096 complement(1731778..1732125)
<313> 8030: 196-215
<400> 8030
CTGCAGAATC CGGACATTCG TGCGGAACTG CCGAAATATG CTAACTGGCC 50
GACCTTCCCG CAACTGTGGG TTGACGGCGA GCTGGTCGGC GGTTGTGATA 100
<210> 8031
<211> 100
<212> DNA
<213> Escherichia coli K-12 MG1655
<220> ydhd b1655
<222> 4-103
<223> ydhd b1655 U00096 complement(1731778..1732125)
<313> 8031: 65-84
<400> 8031
AGCACCACTA TCGAAAAAAT CCAACGCCAG ATTGCTGAAA ACCCGATCCT 50
GCTGTACATG AAAGGTTCAC CGAAACTGCC GAGCTGCGGT TTCTCTGCCC 100
<210> 8032
<211> 100
<212> DNA
<213> Escherichia coli K-12 MG1655
<220> ydhe ydhE
<222> 1275-1374
<223> ydhe ydhE U00096 1741481..1742854
<313> 8032: 1352-1371
<400> 8032
TATTATTGGC CTGACGTCGG CAGCCATTAT GATGATGTTG CGTATGCGGT 50
TCCTGCAACG TCTGCCGTCA GCCATCATTC TGCAACGAGC ATCCCGCTAA 100
<210> 8033
<211> 100
<212> DNA
<213> Escherichia coli K-12 MG1655
<220> ydhe ydhE
<222> 1172-1271
<223> ydhe ydhE U00096 1741481..1742854
<313> 8033: 1222-1241
<400> 8033
CCTTTACGGC TTACTGGGTG CTGGGCTTGC CAAGCGGCTA TATTCTGGCA 50
CTGACCGATC TGGTCGTTGA ACCTATGGGG CCAGCAGGCT TCTGGATAGG 100
<210> 8034
<211> 100
<212> DNA
<213> Escherichia coli K-12 MG1655
<220> ydhe ydhE
<222> 1132-1231
<223> ydhe ydhE U00096 1741481..1742854
<313> 8034: 1183-1202
<400> 8034
TTGCGTGGTT ATAAAGATAC GCGTTCCATT TTCTATATTA CCTTTACGGC 50
TTACTGGGTG CTGGGCTTGC CAAGCGGCTA TATTCTGGCA CTGACCGATC 100
<210> 8035
<211> 100
<212> DNA
<213> Escherichia coli K-12 MG1655
<220> tra5_1 tra5_1
<222> 768-867
<223> tra5_1 tra5_1 U00096 complement(390963..391829)
<313> 8035: 799-818
<400> 8035
GGTGTTTAAT TATATCGAAT GTGATTACAA TCGGTGGCGG CGGCACAGTT 50
GGTGTGGCGG CCTCAGTCCG GAACAATTTG AAAACAAGAA CCTCGCTTAG 100
<210> 8036
<211> 100
<212> DNA
<213> Escherichia coli K-12 MG1655
<220> tra5_1 tra5_1
<222> 697-796

<223> tra5_1 tra5_1 U00096 complement(390963..391829)
<313> 8036: 756-775
<400> 8036
TTCTTTCATT CGCTGAAAGT GGAATGTATC CATGGAGAAC ACTTTATCAG 50
CCGGGAAATA ATGCGGGCAA CGGTGTTTAA TTATATCGAA TGTGATTACA 100
<210> 8037
<211> 100
<212> DNA
<213> Escherichia coli K-12 MG1655
<220> tra5_1 tra5_1
<222> 630-729
<223> tra5_1 tra5_1 U00096 complement(390963..391829)
<313> 8037: 657-676
<400> 8037
GCGGCATAAT CTGCGTGGAA GTATGAGCGC AAAAGGTTGC TGCTACGATA 50
ATGCCTGCGT GGAAAGCTTC TTTCATTCGC TGAAAGTGGA ATGTATCCAT 100
<210> 8038
<211> 100
<212> DNA
<213> Escherichia coli K-12 MG1655
<220> tra5_2 tra5_2
<222> 768-867
<223> tra5_2 tra5_2 U00096 566361..567227
<313> 8038: 807-826
<400> 8038
GGTGTTTAAT TATATCGAAT GTGATTACAA TCGGTGGCGG CGGCACAGTT 50
GGTGTGGCGG CCTCAGTCCG GAACAATTTG AAAACAAGAA CCTCGCTTAG 100
<210> 8039
<211> 100
<212> DNA
<213> Escherichia coli K-12 MG1655
<220> tra5_2 tra5_2
<222> 698-797
<223> tra5_2 tra5_2 U00096 566361..567227
<313> 8039: 745-764
<400> 8039
TCTTTCATTC GCTGAAAGTG GAATGTATCC ATGGAGAACA CTTTATCAGC 50
CGGGAAATAA TGCGGGCAAC GGTGTTTAAT TATATCGAAT GTGATTACAA 100
<210> 8040
<211> 100
<212> DNA
<213> Escherichia coli K-12 MG1655
<220> tra5_2 tra5_2
<222> 610-709
<223> tra5_2 tra5_2 U00096 566361..567227
<313> 8040: 662-681
<400> 8040
GATTATCAGG CGCAACTGAA GCGGCATAAT CTGCGTGGAA GTATGAGCGC 50
AAAAGGTTGC TGCTACGATA ATGCCTGCGT GGAAAGCTTC TTTCATTCGC 100
<210> 8041
<211> 100
<212> DNA
<213> Escherichia coli K-12 MG1655
<220> tra5_3 tra5_3
<222> 768-867
<223> tra5_3 tra5_3 U00096 complement(1093498..1094364)
<313> 8041: 803-822
<400> 8041
GGTGTTTAAT TATATCGAAT GTGATTACAA TCGGTGGCGG CGGCACAGTT 50
GGTGTGGCGG CCTCAGTCCG GAACAATTTG AAAACAAGAA CCTCGCTTAG 100
<210> 8042
<211> 100
<212> DNA
<213> Escherichia coli K-12 MG1655
<220> tra5_3 tra5_3
<222> 699-798
<223> tra5_3 tra5_3 U00096 complement(1093498..1094364)
<313> 8042: 762-781
<400> 8042
CTTTCATTCG CTGAAAGTGG AATGTATCCA TGGAGAACAC TTTATCAGCC 50
GGGAAATAAT GCGGGCAACG GTGTTTAATT ATATCGAATG TGATTACAAT 100

<210> 8043
<211> 100
<212> DNA
<213> Escherichia coli K-12 MG1655
<220> tra5_3 tra5_3
<222> 631-730
<223> tra5_3 tra5_3 U00096 complement(1093498..1094364)
<313> 8043: 661-680
<400> 8043
CGGCATAATC TGCGTGGAAG TATGAGCGCA AAAGGTTGCT GCTACGATAA 50
TGCCTGCGTG GAAAGCTTCT TTCATTCGCT GAAAGTGGAA TGTATCCATG 100
<210> 8044
<211> 100
<212> DNA
<213> Escherichia coli K-12 MG1655
<220> tra5_4 b2090
<222> 761-860
<223> tra5_4 b2090 U00096 2168554..2169420
<313> 8044: 813-832
<400> 8044
GGGCAACGGT GTTTAATTAT ATCGAATGTG ATTACAATCG GTGGCGGCGG 50
CACAGTTGGT GTGGCGGCCT CAGTCCGGAA CAATTTGAAA ACAAGAACCT 100
<210> 8045
<211> 100
<212> DNA
<213> Escherichia coli K-12 MG1655
<220> tra5_4 b2090
<222> 696-795
<223> tra5_4 b2090 U00096 2168554..2169420
<313> 8045: 747-766
<400> 8045
CTTCTTTCAT TCGCTGAAAG TGGAATGTAT CCATGGAGAA CACTTTATCA 50
GCCGGGAAAT AATGCGGGCA ACGGTGTTTA ATTATATCGA ATGTGATTAC 100
<210> 8046
<211> 100
<212> DNA
<213> Escherichia coli K-12 MG1655
<220> tra5_4 b2090
<222> 629-728
<223> tra5_4 b2090 U00096 2168554..2169420
<313> 8046: 660-679
<400> 8046
AGCGGCATAA TCTGCGTGGA AGTATGAGCG CAAAAGGTTG CTGCTACGAT 50
AATGCCTGCG TGGAAAGCTT CTTTCATTCG CTGAAAGTGG AATGTATCCA 100
<210> 8047
<211> 100
<212> DNA
<213> Escherichia coli K-12 MG1655
<220> b1760 b1761
<222> 169-268
<223> b1760 b1761 U00096 complement(1839887..1840159)
<313> 8047: 227-246
<400> 8047
TCTGAAGGGG CGGTAATTAA AGCCGAAGGA ATACTGCTGC AATGCCAGCG 50
TGATGACAAA ACCCTTAGCA CTAATCCGCT GGTCTGGCGG CGCGTAAAGC 100
<210> 8048
<211> 100
<212> DNA
<213> Escherichia coli K-12 MG1655
<220> b1760 b1761
<222> 72-171
<223> b1760 b1761 U00096 complement(1839887..1840159)
<313> 8048: 111-130
<400> 8048
GGACGTGGAG GTAAACGTAC CGCCAGAAGT GTTCAGTTCA GGCGGGCAGT 50
CAGCGCAGCC CTGTACTCAG TGCTGCGTTT ATCAGGATCA AAATTATTCT 100
<210> 8049
<211> 100
<212> DNA
<213> Escherichia coli K-12 MG1655
<220> b1760 b1761

<222> 4-103
<223> b1760 b1761 U00096 complement(1839887..1840159)
<313> 8049: 46-65
<400> 8049
AGTCGAGCAT TGTTCGCCGT AGTATTAGCA TTTCCATTAA TTGCACTGGC 50
TAACCCGCAT TATCGTCCGG ACGTGGAGGT AAACGTACCG CCAGAAGTGT 100
<210> 8050
<211> 100
<212> DNA
<213> Escherichia coli K-12 MG1655
<220> tra5_5 b0300
<222> 768-867
<223> tra5_5 b0300 U00096 314811..315677
<313> 8050: 807-826
<400> 8050
GGTGTTTAAT TATATCGAAT GTGATTACAA TCGGTGGCGG CGGCACAGTT 50
GGTGTGGCGG CCTCAGTCCG GAACAATTTG AAAACAAGAA CCTCGCTTAG 100
<210> 8051
<211> 100
<212> DNA
<213> Escherichia coli K-12 MG1655
<220> tra5_5 b0300
<222> 711-810
<223> tra5_5 b0300 U00096 314811..315677
<313> 8051: 753-772
<400> 8051
GAAAGTGGAA TGTATCCATG GAGAACACTT TATCAGCCGG GAAATAATGC 50
GGGCAACGGT GTTTAATTAT ATCGAATGTG ATTACAATCG GTGGCGGCGG 100
<210> 8052
<211> 100
<212> DNA
<213> Escherichia coli K-12 MG1655
<220> tra5_5 b0300
<222> 622-721
<223> tra5_5 b0300 U00096 314811..315677
<313> 8052: 662-681
<400> 8052
CAACTGAAGC GGCATAATCT GCGTGGAAGT ATGAGCGCAA AAGGTTGCTG 50
CTACGATAAT GCCTGCGTGG AAAGCTTCTT TCATTCGCTG AAAGTGGAAT 100
<210> 8053
<211> 100
<212> DNA
<213> Escherichia coli K-12 MG1655
<220> b2490 b2491
<222> 378-477
<223> b2490 b2491 U00096 2609414..2609890
<313> 8053: 433-452
<400> 8053
GACGTCTGTG TGGCAGGCGC GTAGCATTCA ATTCATTCAT CTGTTGGATG 50
AAATTGTGCA GGAACCGGCC ATCTATCTGA TGGCCAGAAA AATTGCGTGA 100
<210> 8054
<211> 100
<212> DNA
<213> Escherichia coli K-12 MG1655
<220> b2490 b2491
<222> 266-365
<223> b2490 b2491 U00096 2609414..2609890
<313> 8054: 318-337
<400> 8054
AGGAGCAAGC CCGACGTAAG ATGCTGGGGG TGATGACTTT TGGTGAGATT 50
GTTATTGACG CCAGCCACAC CGCCCTGTTG ACCCGGGCAT TCGCGCCACT 100
<210> 8055
<211> 100
<212> DNA
<213> Escherichia coli K-12 MG1655
<220> b2490 b2491
<222> 243-342
<223> b2490 b2491 U00096 2609414..2609890
<313> 8055: 271-290
<400> 8055
AGATTGGCTT GCACTGGTCG AAGAGGAGCA AGCCCGACGT AAGATGCTGG 50

GGGTGATGAC TTTTGGTGAG ATTGTTATTG ACGCCAGCCA CACCGCCCTG 100
<210> 8056
<211> 100
<212> DNA
<213> Escherichia coli K-12 MG1655
<220> b1762 b1763
<222> 1065-1164
<223> b1762 b1763 U00096 complement(1841855..1843018)
<313> 8056: 1117-1136
<400> 8056
TTATCGTTAC GACAAGCAGT TTTGGGAGCA CTACAGTAAG AAATGCCGTT 50
GGCCCATAAG AAATGTTCCG TGGGGAGCAT CATCCGTTAA ATACTCATAA 100
<210> 8057
<211> 100
<212> DNA
<213> Escherichia coli K-12 MG1655
<220> b1762 b1763
<222> 1037-1136
<223> b1762 b1763 U00096 complement(1841855..1843018)
<313> 8057: 1062-1081
<400> 8057
AGTTAACGGA TGCTGAACTT GAAGCGTGTT ATCGTTACGA CAAGCAGTTT 50
TGGGAGCACT ACAGTAAGAA ATGCCGTTGG CCCATAAGAA ATGTTCCGTG 100
<210> 8058
<211> 100
<212> DNA
<213> Escherichia coli K-12 MG1655
<220> b1762 b1763
<222> 965-1064
<223> b1762 b1763 U00096 complement(1841855..1843018)
<313> 8058: 1019-1038
<400> 8058
AAAGCGAAGA AGATTATCAA AAAAATAGTC AAATGGGATT TCTTTACTGG 50
CATATCTGCT GCTATCGCAG AAAGTTAACG GATGCTGAAC TTGAAGCGTG 100
<210> 8059
<211> 100
<212> DNA
<213> Escherichia coli K-12 MG1655
<220> marr b1531
<222> 275-374
<223> marr b1531 U00096 1617201..1617578
<313> 8059: 318-337
<400> 8059
GCCATCAATT AGTTGGCCAG GACCTGCACC AAGAATTAAC AAAAAACCTG 50
ACGGCGGACG AAGTGGCAAC ACTTGAGTAT TTGCTTAAGA AAGTCCTGCC 100
<210> 8060
<211> 100
<212> DNA
<213> Escherichia coli K-12 MG1655
<220> marr b1531
<222> 223-322
<223> marr b1531 U00096 1617201..1617578
<313> 8060: 257-276
<400> 8060
CGCGGCGTAC TGGTAAAACT TACCACCGGC GGCGCGGCAA TATGTGAACA 50
ATGCCATCAA TTAGTTGGCC AGGACCTGCA CCAAGAATTA ACAAAAAACC 100
<210> 8061
<211> 100
<212> DNA
<213> Escherichia coli K-12 MG1655
<220> marr b1531
<222> 177-276
<223> marr b1531 U00096 1617201..1617578
<313> 8061: 199-218
<400> 8061
GGTCTGTAAA GGCTGGGTGG AAAGGTTGCC GAACCCGAAT GACAAGCGCG 50
GCGTACTGGT AAAACTTACC ACCGGCGGCG CGGCAATATG TGAACAATGC 100
<210> 8062
<211> 100
<212> DNA
<213> Escherichia coli K-12 MG1655

<220> ydho b1656
<222> 717-816
<223> ydho b1656 U00096 1732459..1733274
<313> 8062: 796-815
<400> 8062
CACAGGTCAG GAAATTCAAA TCACTTCTCT CAGTGAAGAC TACTGGCAGC 50
GCCACTATGT TGGCGCTCGT CGGGTAATGA CCCCAAAAAC ACTTCGCTAA 100
<210> 8063
<211> 100
<212> DNA
<213> Escherichia coli K-12 MG1655
<220> ydho b1656
<222> 717-816
<223> ydho b1656 U00096 1732459..1733274
<313> 8063: 742-761
<400> 8063
CACAGGTCAG GAAATTCAAA TCACTTCTCT CAGTGAAGAC TACTGGCAGC 50
GCCACTATGT TGGCGCTCGT CGGGTAATGA CCCCAAAAAC ACTTCGCTAA 100
<210> 8064
<211> 100
<212> DNA
<213> Escherichia coli K-12 MG1655
<220> ydho b1656
<222> 672-771
<223> ydho b1656 U00096 1732459..1733274
<313> 8064: 708-727
<400> 8064
TGTCGGCGTG TATGTCGGCA ACGGCAAATT TATTCAGTCA CCGCGCACAG 50
GTCAGGAAAT TCAAATCACT TCTCTCAGTG AAGACTACTG GCAGCGCCAC 100
<210> 8065
<211> 100
<212> DNA
<213> Escherichia coli K-12 MG1655
<220> b2494 b2494
<222> 1290-1389
<223> b2494 b2494 U00096 2614114..2615577
<313> 8065: 1317-1336
<400> 8065
GGCTGCGCGA GCAGAAGGTT ATGCGCTCGC CGGACGACTC GATCAGGCCA 50
TTTCGCTGTT GAGTAGCGCC AGTTCGCAGG TGAAATTAGG CAGCCTGCAA 100
<210> 8066
<211> 100
<212> DNA
<213> Escherichia coli K-12 MG1655
<220> b2494 b2494
<222> 1217-1316
<223> b2494 b2494 U00096 2614114..2615577
<313> 8066: 1268-1287
<400> 8066
ATAAAGATGA CAGCAACGGC TGGGATTTGC TGGCACAGGC GGAAGCCGCG 50
CTAAATAACC GCGATCAGGA GCTGGCTGCG CGAGCAGAAG GTTATGCGCT 100
<210> 8067
<211> 100
<212> DNA
<213> Escherichia coli K-12 MG1655
<220> b2494 b2494
<222> 1137-1236
<223> b2494 b2494 U00096 2614114..2615577
<313> 8067: 1185-1204
<400> 8067
GCTCAACCTG GCGAACGCTT ATTTGCAAGG CGGTCAACCA CAAGAAGCGG 50
CCAATATTCT GAATCGCTAC ACCTTTAATA ATAAAGATGA CAGCAACGGC 100
<210> 8068
<211> 100
<212> DNA
<213> Escherichia coli K-12 MG1655
<220> b2495 b2495
<222> 261-360
<223> b2495 b2495 U00096 2615598..2615957
<313> 8068: 324-343
<400> 8068

GGTGGATAAT CCGAAGCTGA TGGAGCGCCC GATTGTGGTC GCGAATGGCA 50
AAGCGCGGAT TGGTCGTCCA CCGGAGCAGG TACTGGAAAT CGTGGGTTAA 100
<210> 8069
<211> 100
<212> DNA
<213> Escherichia coli K-12 MG1655
<220> b2495 b2495
<222> 224-323
<223> b2495 b2495 U00096 2615598..2615957
<313> 8069: 271-290
<400> 8069
ACAGTTCGCT TAGCGAAGAG GCGCTGATTC AGGCAATGGT GGATAATCCG 50
AAGCTGATGG AGCGCCCGAT TGTGGTCGCG AATGGCAAAG CGCGGATTGG 100
<210> 8070
<211> 100
<212> DNA
<213> Escherichia coli K-12 MG1655
<220> b2495 b2495
<222> 136-235
<223> b2495 b2495 U00096 2615598..2615957
<313> 8070: 171-190
<400> 8070
CGCGATTTGC TGAAAATACT GGGGATGAAT AGCGCCCGTG AATTGATGCG 50
CCAGAAAGAG GATCTTTATA AAGAACTGAA CCTGGCTGAC AGTTCGCTTA 100
<210> 8071
<211> 100
<212> DNA
<213> Escherichia coli K-12 MG1655
<220> b2496 b2497
<222> 648-747
<223> b2496 b2497 U00096 complement(2616095..2616841)
<313> 8071: 717-736
<400> 8071
GCGCACGCTA TTTATGACGT TGGATCAGTT GGATCGTGCG TCGATTACCG 50
CGCAACGTAA GCTGACCATT CCGTTTGTGA AAGAAATTCT GAAGTTGTAG 100
<210> 8072
<211> 100
<212> DNA
<213> Escherichia coli K-12 MG1655
<220> b2496 b2497
<222> 643-742
<223> b2496 b2497 U00096 complement(2616095..2616841)
<313> 8072: 671-690
<400> 8072
GAAATGCGCA CGCTATTTAT GACGTTGGAT CAGTTGGATC GTGCGTCGAT 50
TACCGCGCAA CGTAAGCTGA CCATTCCGTT TGTGAAAGAA ATTCTGAAGT 100
<210> 8073
<211> 100
<212> DNA
<213> Escherichia coli K-12 MG1655
<220> b2496 b2497
<222> 491-590
<223> b2496 b2497 U00096 complement(2616095..2616841)
<313> 8073: 552-571
<400> 8073
ATCTCGCGTC GCGACTCGAC TGGGGGCAGA TCTACAAATT GCAGCCACTT 50
TCTGATGAAG ATAAGTTGCA GGCGCTACAG TTACGCGCGC GTTTGCGTGG 100
<210> 8074
<211> 100
<212> DNA
<213> Escherichia coli K-12 MG1655
<220> ydhu ydhU
<222> 656-755
<223> ydhu ydhU U00096 complement(1747587..1748372)
<313> 8074: 699-718
<400> 8074
TATTGCAGAC ACATTTTGCT CTGGCATTTA TAAGCCTCTT TTTTATCTTC 50
GGTCATCTTT ATCTTTGCAC CACGGGGCGT ACGCCACACG AAACCTTTAA 100
<210> 8075
<211> 100
<212> DNA

<213> Escherichia coli K-12 MG1655
<220> ydhu ydhU
<222> 628-727
<223> ydhu ydhU U00096 complement(1747587..1748372)
<313> 8075: 649-668
<400> 8075
GATGTGTTTC CTGGCGTAAG ATACTGGTTA TTGCAGACAC ATTTTGCTCT 50
GGCATTTATA AGCCTCTTTT TTATCTTCGG TCATCTTTAT CTTTGCACCA 100
<210> 8076
<211> 100
<212> DNA
<213> Escherichia coli K-12 MG1655
<220> ydhu ydhU
<222> 574-673
<223> ydhu ydhU U00096 complement(1747587..1748372)
<313> 8076: 600-619
<400> 8076
CTGCCGTTGT TACTATTGAC GGGGCTGCTG TGTCTCTATC CGCAAGCCGT 50
GGGAGATGTG TTTCCTGGCG TAAGATACTG GTTATTGCAG ACACATTTTG 100
<210> 8077
<211> 100
<212> DNA
<213> Escherichia coli K-12 MG1655
<220> ydia b1704
<222> 735-834
<223> ydia b1704 U00096 1785469..1786302
<313> 8077: 803-822
<400> 8077
TAAAAATCAG ATCCCGTGGA TTAACAGTAC CAATTATTCG GTAGAAGAGA 50
TTGCCACCAA GATCCTCGAT ATCATGGGCC TTAGTCGCCG AATGTACTAG 100
<210> 8078
<211> 100
<212> DNA
<213> Escherichia coli K-12 MG1655
<220> ydia b1704
<222> 695-794
<223> ydia b1704 U00096 1785469..1786302
<313> 8078: 732-751
<400> 8078
AGTGCAGGAT GGAAGTCGCG GAAGTGGAAG CCTTGTACCG TAAAAATCAG 50
ATCCCGTGGA TTAACAGTAC CAATTATTCG GTAGAAGAGA TTGCCACCAA 100
<210> 8079
<211> 100
<212> DNA
<213> Escherichia coli K-12 MG1655
<220> ydia b1704
<222> 625-724
<223> ydia b1704 U00096 1785469..1786302
<313> 8079: 684-703
<400> 8079
ATCGACCCGG AACGTCTGGC GGCGATTCGC GAGGAACGTC GGGAGAACAG 50
TCGCTATGCC TCGCTTCGTC AGTGCAGGAT GGAAGTCGCG GAAGTGGAAG 100
<210> 8080
<211> 100
<212> DNA
<213> Escherichia coli K-12 MG1655
<220> ydib b1693
<222> 768-867
<223> ydib b1693 U00096 1771813..1772679
<313> 8080: 830-849
<400> 8080
CGGCATGTTG TTGTGGCAAG GGGCTGAACA GTTCACATTA TGGACTGGCA 50
AAGATTTCCC TCTGGAATAT GTTAAACAGG TCATGGGGTT CGGTGCCTGA 100
<210> 8081
<211> 100
<212> DNA
<213> Escherichia coli K-12 MG1655
<220> ydib b1693
<222> 716-815
<223> ydib b1693 U00096 1771813..1772679
<313> 8081: 748-767

<400> 8081
CGAAGTTATT GCAGCAGGCG CAACAAGCTG GTTGCAAAAC GATTGATGGA 50
TACGGCATGT TGTTGTGGCA AGGGGCTGAA CAGTTCACAT TATGGACTGG 100
<210> 8082
<211> 100
<212> DNA
<213> Escherichia coli K-12 MG1655
<220> ydib b1693
<222> 664-763
<223> ydib b1693 U00096 1771813..1772679
<313> 8082: 703-722
<400> 8082
CTGTTACATC CGGGACTTCT GGTCACTGAA TGCGTGTATA ACCCGCATAT 50
GACGAAGTTA TTGCAGCAGG CGCAACAAGC TGGTTGCAAA ACGATTGATG 100
<210> 8083
<211> 100
<212> DNA
<213> Escherichia coli K-12 MG1655
<220> ydic ydiC
<222> 195-294
<223> ydic ydiC U00096 complement(1762042..1762410)
<313> 8083: 234-253
<400> 8083
AGACGATCTG CTGTTTGAAC ACGACGGCGC GAAGCTGTTT GTCCCGCTGC 50
AAGCGATGCC GTTTATTGAT GGCACGGAAG TCGATTTCGT TCGTGAAGGA 100
<210> 8084
<211> 100
<212> DNA
<213> Escherichia coli K-12 MG1655
<220> ydic ydiC
<222> 154-253
<223> ydic ydiC U00096 complement(1762042..1762410)
<313> 8084: 187-206
<400> 8084
GGCTTTGGCT ATGTGCTCGA CAGTGTTAGC GAGCCGGACA AAGACGATCT 50
GCTGTTTGAA CACGACGGCG CGAAGCTGTT TGTCCCGCTG CAAGCGATGC 100
<210> 8085
<211> 100
<212> DNA
<213> Escherichia coli K-12 MG1655
<220> ydic ydiC
<222> 50-149
<223> ydic ydiC U00096 complement(1762042..1762410)
<313> 8085: 104-123
<400> 8085
GCTTAACGCT GACACCCGCA GCGGCGATAC ACATCCGTGA GCTGGTGGCA 50
AAGCAGCCGG GTATGGTCGG CGTGCGCTTA GGCGTGAAGC AAACGGGCTG 100
<210> 8086
<211> 100
<212> DNA
<213> Escherichia coli K-12 MG1655
<220> ydid b1702
<222> 1602-1701
<223> ydid b1702 U00096 1781001..1782701
<313> 8086: 1663-1682
<400> 8086
AAAACTACCG CGAACTACCT CAGGTAAAAT ACAAAAGTTT TTGTTAAGAA 50
AAGATATTAT GCGGCGTTTA ACGCAGGATG TCTGTGAAGA GATTGAATAA 100
<210> 8087
<211> 100
<212> DNA
<213> Escherichia coli K-12 MG1655
<220> ydid b1702
<222> 1513-1612
<223> ydid b1702 U00096 1781001..1782701
<313> 8087: 1547-1566
<400> 8087
CATTCATTAT CGCTGGAAGA GGTAGTGGCT TTTTTTAGCC GTAAACGGGT 50
CGCAAAATAT AAATATCCTG AACATATCGT GGTAATCGAA AAACTACCGC 100
<210> 8088
<211> 100

<212> DNA
<213> Escherichia coli K-12 MG1655
<220> ydid b1702
<222> 1450-1549
<223> ydid b1702 U00096 1781001..1782701
<313> 8088: 1510-1529
<400> 8088
GCAATGTCCG ATGAACGTTT AGGTGAACGA TCATGCGCTT ATGTCGTGCT 50
GAAAGCGCCG CATCATTCAT TATCGCTGGA AGAGGTAGTG GCTTTTTTTA 100
<210> 8089
<211> 100
<212> DNA
<213> Escherichia coli K-12 MG1655
<220> ydie ydiE
<222> 93-192
<223> ydie ydiE U00096 1787637..1787828
<313> 8089: 155-174
<400> 8089
GACACTGTTA GGTCCGGATG GCAAACTGAT TATCGATCAT GACGGGCAAG 50
AATATCTGCT CCGTAAAACT CAGGCTGGCA AGCTGCTGTT GACCAAGTAG 100
<210> 8090
<211> 100
<212> DNA
<213> Escherichia coli K-12 MG1655
<220> ydie ydiE
<222> 87-186
<223> ydie ydiE U00096 1787637..1787828
<313> 8090: 113-132
<400> 8090
CAGCCAGACA CTGTTAGGTC CGGATGGCAA ACTGATTATC GATCATGACG 50
GGCAAGAATA TCTGCTCCGT AAAACTCAGG CTGGCAAGCT GCTGTTGACC 100
<210> 8091
<211> 100
<212> DNA
<213> Escherichia coli K-12 MG1655
<220> ydie ydiE
<222> 57-156
<223> ydie ydiE U00096 1787637..1787828
<313> 8091: 70-89
<400> 8091
CGCTTCCCCG CAGCCTATTC GGCGAATTTC CAGCCAGACA CTGTTAGGTC 50
CGGATGGCAA ACTGATTATC GATCATGACG GGCAAGAATA TCTGCTCCGT 100
<210> 8092
<211> 100
<212> DNA
<213> Escherichia coli K-12 MG1655
<220> ydif ydiF
<222> 1471-1570
<223> ydif ydiF U00096 1773611..1775206
<313> 8092: 1512-1531
<400> 8092
TTACAAAAAG ATATTCTCGA CAAAATGGAT TTCACCCCAG TGATTTCGCC 50
AGAACTCAAA CTGATGGACG AAAGATTATT TATCGATGCG GCGATGGGTT 100
<210> 8093
<211> 100
<212> DNA
<213> Escherichia coli K-12 MG1655
<220> ydif ydiF
<222> 1414-1513
<223> ydif ydiF U00096 1773611..1775206
<313> 8093: 1438-1457
<400> 8093
GTATTCACGC TGAAAGAAGA CGGCCTGCAT TTAATCGAAA TCGCCCCTGG 50
CGTCGATTTA CAAAAAGATA TTCTCGACAA AATGGATTTC ACCCCAGTGA 100
<210> 8094
<211> 100
<212> DNA
<213> Escherichia coli K-12 MG1655
<220> ydif ydiF
<222> 1287-1386
<223> ydif ydiF U00096 1773611..1775206

<313> 8094: 1348-1367
<400> 8094
CGGCAAATTA AATATCGTCC AGGAAGGACG GGTGAAGAAA TTTATTCGGG 50
AACTACCGGA AATTACTTTC AGCGGAAAAA TCGCTCTCGA GCGAGGGCTG 100
<210> 8095
<211> 100
<212> DNA
<213> Escherichia coli K-12 MG1655
<220> ydij ydiJ
<222> 2958-3057
<223> ydij ydiJ U00096 complement(1763653..1766709)
<313> 8095: 2991-3010
<400> 8095
CTGTCTGGCG ACCGGATATT CCTGCCGTAG CCAGGTAAAA CGGGTTGAAG 50
GCACGGGGGT ACGCCATCCT GTGCAGGCTT TACTGGAGAT TATTAAATGA 100
<210> 8096
<211> 100
<212> DNA
<213> Escherichia coli K-12 MG1655
<220> ydij ydiJ
<222> 2875-2974
<223> ydij ydiJ U00096 complement(1763653..1766709)
<313> 8096: 2919-2938
<400> 8096
CATGAAGCGA AAAACCATGA AAATTCGCTC GGGATCTATG AGTTATCCTG 50
GCATCAGGCT ATGCAGCGAC TGCCGCGTAA CCGCTGTCTG GCGACCGGAT 100
<210> 8097
<211> 100
<212> DNA
<213> Escherichia coli K-12 MG1655
<220> ydij ydiJ
<222> 2847-2946
<223> ydij ydiJ U00096 complement(1763653..1766709)
<313> 8097: 2877-2896
<400> 8097
TTGCTGCGGC ATGGCAGGGA CTTACGGACA TGAAGCGAAA AACCATGAAA 50
ATTCGCTCGG GATCTATGAG TTATCCTGGC ATCAGGCTAT GCAGCGACTG 100
<210> 8098
<211> 100
<212> DNA
<213> Escherichia coli K-12 MG1655
<220> b1770 b1770
<222> 567-666
<223> b1770 b1770 U00096 complement(1852120..1852878)
<313> 8098: 588-607
<400> 8098
AGCTGAAATC AAAAAGACAA TGATCCGTCA GGCGACAGAA GTTGCGTTAT 50
TGGTTGATCA CTCTAAGTTT GATCGCAAAG CTTTTGTCCA GTTAGCTGAT 100
<210> 8099
<211> 100
<212> DNA
<213> Escherichia coli K-12 MG1655
<220> b1770 b1770
<222> 398-497
<223> b1770 b1770 U00096 complement(1852120..1852878)
<313> 8099: 436-455
<400> 8099
AGTCAGAAAT TAAAGTCGTT TCAACGGGTG GGGAACTAAA CAAAAATACA 50
CTTTCACTGC AAGGAAGAAT TACTAAAGAG ATCATCAGGC GCTATCATGT 100
<210> 8100
<211> 100
<212> DNA
<213> Escherichia coli K-12 MG1655
<220> b1770 b1770
<222> 335-434
<223> b1770 b1770 U00096 complement(1852120..1852878)
<313> 8100: 376-395
<400> 8100
TACAGGACCG TAGTGGCCTG ACGTTGCTAA CCAACTCGGC GGAAGCGATT 50
CATGTTCTGG CTCAGTCAGA AATTAAAGTC GTTTCAACGG GTGGGGAACT 100
<210> 8101

<211> 100
<212> DNA
<213> Escherichia coli K-12 MG1655
<220> b1771 b1771
<222> 881-980
<223> b1771 b1771 U00096 complement(1853015..1853995)
<313> 8101: 913-932
<400> 8101
CACCGGAACA GGTACGCGAA AATGTCGCGG CACTGAATAT CAACTTATCG 50
GATGCAGACG CAACATTGAT GAGGGAAATG GCAGAGGCCC TGGAGCGTTA 100
<210> 8102
<211> 100
<212> DNA
<213> Escherichia coli K-12 MG1655
<220> b1771 b1771
<222> 834-933
<223> b1771 b1771 U00096 complement(1853015..1853995)
<313> 8102: 884-903
<400> 8102
GATATTAAAA CAGAGTGATT TAATCTCCAT TCTTAGTGGG GCTACTGCAC 50
CGGAACAGGT ACGCGAAAAT GTCGCGGCAC TGAATATCAA CTTATCGGAT 100
<210> 8103
<211> 100
<212> DNA
<213> Escherichia coli K-12 MG1655
<220> b1771 b1771
<222> 780-879
<223> b1771 b1771 U00096 complement(1853015..1853995)
<313> 8103: 808-827
<400> 8103
GCAGCCACTT TGTGCTCGTT ATCAGTGCAC AATTCCCACT CTGGCACTGG 50
CGTGGATATT AAAACAGAGT GATTTAATCT CCATTCTTAG TGGGGCTACT 100
<210> 8104
<211> 100
<212> DNA
<213> Escherichia coli K-12 MG1655
<220> b1772 b1772
<222> 870-969
<223> b1772 b1772 U00096 complement(1854005..1854973)
<313> 8104: 941-960
<400> 8104
CAATGCAACG GCGGCTATCT CGGTTCTAAG CGTCGGTGCC ACCACCGGCG 50
TAAAAAACAG AAAGCTGGTC GAACAATTGC TGGAAGAATA CGAAGGATAA 100
<210> 8105
<211> 100
<212> DNA
<213> Escherichia coli K-12 MG1655
<220> b1772 b1772
<222> 833-932
<223> b1772 b1772 U00096 complement(1854005..1854973)
<313> 8105: 873-892
<400> 8105
TAGAAGGCAA AAATCTGCGT GAATGCGCAC GCTTTGCCAA TGCAACGGCG 50
GCTATCTCGG TTCTAAGCGT CGGTGCCACC ACCGGCGTAA AAAACAGAAA 100
<210> 8106
<211> 100
<212> DNA
<213> Escherichia coli K-12 MG1655
<220> b1772 b1772
<222> 773-872
<223> b1772 b1772 U00096 complement(1854005..1854973)
<313> 8106: 836-855
<400> 8106
CCGCCATCGA CACCATTGGC GCGGGCGATA ACTTTGCTTC AGGTTTTATT 50
GCGGCACTGT TAGAAGGCAA AAATCTGCGT GAATGCGCAC GCTTTGCCAA 100
<210> 8107
<211> 100
<212> DNA
<213> Escherichia coli K-12 MG1655
<220> b1773 b1774
<222> 715-814

<223> b1773 b1774 U00096 complement(1854957..1855793)
<313> 8107: 778-797
<400> 8107
GTTGCAGTGA AAGAAAACCA GGATCAGCCT TTCCTGCATT TAGAACGTGA 50
AGTACGTAAA GCTGTAAAAG AACGGGCATT GGAAAAAATT AAACTGTTCG 100
<210> 8108
<211> 100
<212> DNA
<213> Escherichia coli K-12 MG1655
<220> b1773 b1774
<222> 699-798
<223> b1773 b1774 U00096 complement(1854957..1855793)
<313> 8108: 721-740
<400> 8108
CTGTCAGGCC GCAATGGTTG CAGTGAAAGA AAACCAGGAT CAGCCTTTCC 50
TGCATTTAGA ACGTGAAGTA CGTAAAGCTG TAAAAGAACG GGCATTGGAA 100
<210> 8109
<211> 100
<212> DNA
<213> Escherichia coli K-12 MG1655
<220> b1773 b1774
<222> 607-706
<223> b1773 b1774 U00096 complement(1854957..1855793)
<313> 8109: 632-651
<400> 8109
CTGGTTTTGC ACGGTGCATC GGGGATCAGT GATGCCGACA TTAAAACTGC 50
AATTTCGTTG GGTATCGCGA AAATCAACAT TCATACGGAG CTCTGTCAGG 100
<210> 8110
<211> 100
<212> DNA
<213> Escherichia coli K-12 MG1655
<220> b1775 b1775
<222> 1281-1380
<223> b1775 b1775 U00096 complement(1856874..1858253)
<313> 8110: 1318-1337
<400> 8110
GGTTTCAATT ATTGTCGCAA TTGCTATCGC CACCATTGGA ATCGAAACCA 50
AAGGTGTCTC CGTTGAAAGT TTAAGTATTG ATGCAGTAGC CAATAAATAA 100
<210> 8111
<211> 100
<212> DNA
<213> Escherichia coli K-12 MG1655
<220> b1775 b1775
<222> 1217-1316
<223> b1775 b1775 U00096 complement(1856874..1858253)
<313> 8111: 1242-1261
<400> 8111
CTTATGCCGT TGCAGTGCTG CTCAGTAGTT ATGGCGTTAC GGGAGTCTTT 50
ATTCTTCTGG GGGCGGTTTC AATTATTGTC GCAATTGCTA TCGCCACCAT 100
<210> 8112
<211> 100
<212> DNA
<213> Escherichia coli K-12 MG1655
<220> b1775 b1775
<222> 1131-1230
<223> b1775 b1775 U00096 complement(1856874..1858253)
<313> 8112: 1176-1195
<400> 8112
TGTCCCTGAA ATCTGGCCGA CAGAGGCCAA ACTCCGTGGC TCCGGTCTGG 50
CGAATGCGGT AGGGCGAATC AGTGGTATTG CCGCACCTTA TGCCGTTGCA 100
<210> 8113
<211> 100
<212> DNA
<213> Escherichia coli K-12 MG1655
<220> b1776 b1776
<222> 978-1077
<223> b1776 b1776 U00096 complement(1858280..1859356)
<313> 8113: 1018-1037
<400> 8113
CACGCATCGT ATCGGCCTGT CGCAATGGCG CGAAGGGTTT GATGCGATGG 50
TCGATAAAAC CGCAATCAAA GTGATCATGA CTTACGACTT TGATGAATAA 100

<210> 8114
<211> 100
<212> DNA
<213> Escherichia coli K-12 MG1655
<220> b1776 b1776
<222> 953-1052
<223> b1776 b1776 U00096 complement(1858280..1859356)
<313> 8114: 985-1004
<400> 8114
GCGCTATCAA AGTCAAACCG ATGATCACGC ATCGTATCGG CCTGTCGCAA  50
TGGCGCGAAG GGTTTGATGC GATGGTCGAT AAAACCGCAA TCAAAGTGAT  100
<210> 8115
<211> 100
<212> DNA
<213> Escherichia coli K-12 MG1655
<220> b1776 b1776
<222> 898-997
<223> b1776 b1776 U00096 complement(1858280..1859356)
<313> 8115: 943-962
<400> 8115
CATATGGCCT ATGACTCCAC CTCATGGCGT AACGCTATCA GGCTATTAGC  50
CAGCGGCGCT ATCAAAGTCA AACCGATGAT CACGCATCGT ATCGGCCTGT  100
<210> 8116
<211> 100
<212> DNA
<213> Escherichia coli K-12 MG1655
<220> ydiq b1698
<222> 666-765
<223> ydiq b1698 U00096 1777641..1778405
<313> 8116: 736-755
<400> 8116
CGTACCGCCG CAAACAGAAC GTAAGCACAT CATTATCGAT AACGATTCGC  50
CGGAGGCCAT TGCCGAGCTG GCGGAACATC TGAAGAAAGC CCTGAACTGA  100
<210> 8117
<211> 100
<212> DNA
<213> Escherichia coli K-12 MG1655
<220> ydiq b1698
<222> 641-740
<223> ydiq b1698 U00096 1777641..1778405
<313> 8117: 680-699
<400> 8117
CACTTGCTGA ACTGGTCGGC ATTCGCGTAC CGCCGCAAAC AGAACGTAAG  50
CACATCATTA TCGATAACGA TTCGCCGGAG GCCATTGCCG AGCTGGCGGA  100
<210> 8118
<211> 100
<212> DNA
<213> Escherichia coli K-12 MG1655
<220> ydiq b1698
<222> 571-670
<223> ydiq b1698 U00096 1777641..1778405
<313> 8118: 605-624
<400> 8118
ATTCTCGGCG CGGGTAAAAA ACCGGTAAAT CAGTGGCAGG CAAGTGATAT  50
TGACTGGAGC CAGAGCGCGC CACTTGCTGA ACTGGTCGGC ATTCGCGTAC  100
<210> 8119
<211> 100
<212> DNA
<213> Escherichia coli K-12 MG1655
<220> b1777 b1778
<222> 157-256
<223> b1777 b1778 U00096 complement(1859726..1860043)
<313> 8119: 203-222
<400> 8119
AAAGAGAACT GCCTGCAACT GGTGATGCTG TGGCAAGCCC GCCATAATAT  50
CGAAGCACAG CATATGACAA TTGACACAAA TGGTCAGATG GTCATGAAGA  100
<210> 8120
<211> 100
<212> DNA
<213> Escherichia coli K-12 MG1655
<220> b1777 b1778

<222> 133-232
<223> b1777 b1778 U00096 complement(1859726..1860043)
<313> 8120: 166-185
<400> 8120
GGCGTTGCGT TGACCGAGGA ACAAAAAGAG AACTGCCTGC AACTGGTGAT 50
GCTGTGGCAA GCCCGCCATA ATATCGAAGC ACAGCATATG ACAATTGACA 100
<210> 8121
<211> 100
<212> DNA
<213> Escherichia coli K-12 MG1655
<220> b1777 b1778
<222> 87-186
<223> b1777 b1778 U00096 complement(1859726..1860043)
<313> 8121: 120-139
<400> 8121
ATACCAGCGT TTGTCGACCG CCGTTGAACT GGGGAAATGG CCTGATGGCG 50
TTGCGTTGAC CGAGGAACAA AAAGAGAACT GCCTGCAACT GGTGATGCTG 100
<210> 8122
<211> 100
<212> DNA
<213> Escherichia coli K-12 MG1655
<220> ydir b1699
<222> 827-926
<223> ydir b1699 U00096 1778425..1779363
<313> 8122: 874-893
<400> 8122
TTGTCGCCAT CAATAAAGAT AAAAATGCGC CAATCTTCAA CTATGCCGAC 50
TACGGTCTGG TGGGCGATAT CTACAAAGTC GTCCCTGCCC TGATTAGCCA 100
<210> 8123
<211> 100
<212> DNA
<213> Escherichia coli K-12 MG1655
<220> ydir b1699
<222> 770-869
<223> ydir b1699 U00096 1778425..1779363
<313> 8123: 814-833
<400> 8123
CGCTGGGGAT CTCCGGGCAG ATCCAGCATA TGGTTGGCGG CAACGGCGCA 50
AAAGTGATTG TCGCCATCAA TAAAGATAAA AATGCGCCAA TCTTCAACTA 100
<210> 8124
<211> 100
<212> DNA
<213> Escherichia coli K-12 MG1655
<220> ydir b1699
<222> 656-755
<223> ydir b1699 U00096 1778425..1779363
<313> 8124: 708-727
<400> 8124
TGCTGAATGC TGAAGTCGGC TGTTCACGTC CAATTGCCGA AGGCGAGAAC 50
TGGATGGAGC GTGAACGTTA TATCGGTGTC TCCGGCGTGT TGCTGAAATC 100
<210> 8125
<211> 100
<212> DNA
<213> Escherichia coli K-12 MG1655
<220> ydis b1700
<222> 1191-1290
<223> ydis b1700 U00096 1779419..1780708
<313> 8125: 1264-1283
<400> 8125
ACCAAACCAG CCGGTACGAA AAATGATCAT GGGACATGCG AAGAAAATTG 50
GGCTGATCAA CTTGCTGAAA GATGGCATTA AGGGAGCAAC CGCGCTATGA 100
<210> 8126
<211> 100
<212> DNA
<213> Escherichia coli K-12 MG1655
<220> ydis b1700
<222> 1145-1244
<223> ydis b1700 U00096 1779419..1780708
<313> 8126: 1194-1213
<400> 8126
GAATGGTAGC CGACATCATG AACGAGATGT TCACCATCGA CGGCAAACCA 50

AACCAGCCGG TACGAAAAAT GATCATGGGA CATGCGAAGA AAATTGGGCT 100
<210> 8127
<211> 100
<212> DNA
<213> Escherichia coli K-12 MG1655
<220> ydis b1700
<222> 1098-1197
<223> ydis b1700 U00096 1779419..1780708
<313> 8127: 1144-1163
<400> 8127
GATCCCGGCG CTGATGGAAA ACCCGCGCCT GTTTAGCCAA TACCCACGAA 50
TGGTAGCCGA CATCATGAAC GAGATGTTCA CCATCGACGG CAAACCAAAC 100
<210> 8128
<211> 100
<212> DNA
<213> Escherichia coli K-12 MG1655
<220> ydit b1701
<222> 191-290
<223> ydit b1701 U00096 1780705..1780998
<313> 8128: 244-263
<400> 8128
GTTGTCTGGA GTGCGGCACC TGTCGGGTGC TGTGCGGTAA CACTATTCTC 50
GAACAGTGGC AATATCCCGC AGGCACCTTC GGTATTGATT TTCGCTACGG 100
<210> 8129
<211> 100
<212> DNA
<213> Escherichia coli K-12 MG1655
<220> ydit b1701
<222> 160-259
<223> ydit b1701 U00096 1780705..1780998
<313> 8129: 187-206
<400> 8129
GACGCAGGAA ACATTCATTT TGATTCCGCC GGTTGTCTGG AGTGCGGCAC 50
CTGTCGGGTG CTGTGCGGTA ACACTATTCT CGAACAGTGG CAATATCCCG 100
<210> 8130
<211> 100
<212> DNA
<213> Escherichia coli K-12 MG1655
<220> ydit b1701
<222> 120-219
<223> ydit b1701 U00096 1780705..1780998
<313> 8130: 135-154
<400> 8130
ATTAATGAAA GCCTGCCCTG CCGGACTTTA TAAGCAGGAT GACGCAGGAA 50
ACATTCATTT TGATTCCGCC GGTTGTCTGG AGTGCGGCAC CTGTCGGGTG 100
<210> 8131
<211> 100
<212> DNA
<213> Escherichia coli K-12 MG1655
<220> degq b3235
<222> 1269-1368
<223> degq b3235 U00096 3378378..3379745
<313> 8131: 1334-1353
<400> 8131
CTCGATTGCT GAAATGCGTA AAGTGCTGGC GGCAAAACCG GCCATCATCG 50
CCCTGCAAAT TGTACGCGGC AATGAAAGCA TCTATCTGCT GATGCGTTAA 100
<210> 8132
<211> 100
<212> DNA
<213> Escherichia coli K-12 MG1655
<220> degq b3235
<222> 1102-1201
<223> degq b3235 U00096 3378378..3379745
<313> 8132: 1144-1163
<400> 8132
GCTGAAATGA TCACGCCAGC GCTGGAAGGT GCAACGTTGA GCGATGGTCA 50
GCTAAAAGAT GGCGGCAAAG GTATTAAAAT CGATGAAGTT GTCAAAGGAA 100
<210> 8133
<211> 100
<212> DNA
<213> Escherichia coli K-12 MG1655

<220> degq b3235
<222> 1029-1128
<223> degq b3235 U00096 3378378..3379745
<313> 8133: 1064-1083
<400> 8133
TGGCCTGCTG CGTAACGGCA AACCACTGGA AGTAGAAGTG ACGCTCGATA 50
CCAGCACCTC TTCGTCGGCC AGCGCTGAAA TGATCACGCC AGCGCTGGAA 100
<210> 8134
<211> 100
<212> DNA
<213> Escherichia coli K-12 MG1655
<220> degs b3236
<222> 939-1038
<223> degs b3236 U00096 3379835..3380902
<313> 8134: 995-1014
<400> 8134
CTCTGCTCTG GAGACGATGG ATCAGGTGGC GGAAATTCGC CCTGGTTCGG 50
TGATCCCTGT AGTAGTGATG CGTGATGATA AGCAGTTAAC GCTGCAGGTC 100
<210> 8135
<211> 100
<212> DNA
<213> Escherichia coli K-12 MG1655
<220> degs b3236
<222> 870-969
<223> degs b3236 U00096 3379835..3380902
<313> 8135: 922-941
<400> 8135
CGGCCCGGCG GCGAATGCGG GTATTCAGGT CAACGATCTG ATTATTTCGG 50
TGGATAACAA ACCGGCCATC TCTGCTCTGG AGACGATGGA TCAGGTGGCG 100
<210> 8136
<211> 100
<212> DNA
<213> Escherichia coli K-12 MG1655
<220> degs b3236
<222> 854-953
<223> degs b3236 U00096 3379835..3380902
<313> 8136: 871-890
<400> 8136
ATGAAGTGTC ACCTGACGGC CCGGCGGCGA ATGCGGGTAT TCAGGTCAAC 50
GATCTGATTA TTTCGGTGGA TAACAAACCG GCCATCTCTG CTCTGGAGAC 100
<210> 8137
<211> 100
<212> DNA
<213> Escherichia coli K-12 MG1655
<220> ydja b1766
<222> 453-552
<223> ydja b1766 U00096 complement(1846149..1846700)
<313> 8137: 527-546
<400> 8137
GGATAAAATT GTCGGTTTTC TCTACCTCGG TACGCCGCAG CTGAAAGCAT 50
CTACGTCGAT TAACGTCCCG GACCCGACGC CGTTTGTAAC TTATTTCTGA 100
<210> 8138
<211> 100
<212> DNA
<213> Escherichia coli K-12 MG1655
<220> ydja b1766
<222> 432-531
<223> ydja b1766 U00096 complement(1846149..1846700)
<313> 8138: 465-484
<400> 8138
AGCATTCGGT TGCCGTGAGC AGGATAAAAT TGTCGGTTTT CTCTACCTCG 50
GTACGCCGCA GCTGAAAGCA TCTACGTCGA TTAACGTCCC GGACCCGACG 100
<210> 8139
<211> 100
<212> DNA
<213> Escherichia coli K-12 MG1655
<220> ydja b1766
<222> 383-482
<223> ydja b1766 U00096 complement(1846149..1846700)
<313> 8139: 428-447
<400> 8139

GCGGCATCTG GCGCAGTGGC GCATTAACTG AAAGTCCGGT AGTGCGTGAA 50
GCATTCGGTT GCCGTGAGCA GGATAAAATT GTCGGTTTTC TCTACCTCGG 100
<210> 8140
<211> 100
<212> DNA
<213> Escherichia coli K-12 MG1655
<220> ydjb b1769
<222> 561-660
<223> ydjb b1769 U00096 1849893..1850552
<313> 8140: 626-645
<400> 8140
GAATATCCAG CCCCAGGACA GTGCGCACGC GTTTATGGAG ATGTCAGCAG 50
CTGGGGCAAC GCTATATACG CTGGCAGACT GGGAAGAGAC ACAGGGGTAA 100
<210> 8141
<211> 100
<212> DNA
<213> Escherichia coli K-12 MG1655
<220> ydjb b1769
<222> 431-530
<223> ydjb b1769 U00096 1849893..1850552
<313> 8141: 474-493
<400> 8141
TACGCGATCA TGAAATCGAT GAATTGATCG TTATGGGCCT GGCTACTGAC 50
TATTGCGTGA AGTTTACCGT GCTGGACGCG TTACAGTTAG GTTATAAGGT 100
<210> 8142
<211> 100
<212> DNA
<213> Escherichia coli K-12 MG1655
<220> ydjb b1769
<222> 318-417
<223> ydjb b1769 U00096 1849893..1850552
<313> 8142: 349-368
<400> 8142
GCACCAAAAA GCGATCGCAG CGGTGTTCCA TAAAGGCGAA AATCCTTTAG 50
TTGACAGTTA CAGTGCCTTT TTTGATAACG GCCGTCGGCA GAAAACCTCT 100
<210> 8143
<211> 100
<212> DNA
<213> Escherichia coli K-12 MG1655
<220> ydjc b1734
<222> 651-750
<223> ydjc b1734 U00096 complement(1814410..1815159)
<313> 8143: 719-738
<400> 8143
TTTCCCACGC TTAACGGAGC TGGATGTGTT GACATCAGCG TCGTTGAAAG 50
GTGCTATCGC TCAGCGTGGT TATCGGTTGG GGAGTTATCG GGATGTGTAA 100
<210> 8144
<211> 100
<212> DNA
<213> Escherichia coli K-12 MG1655
<220> ydjc b1734
<222> 592-691
<223> ydjc b1734 U00096 complement(1814410..1815159)
<313> 8144: 647-666
<400> 8144
CTGGAGGTGA TGTGTCATCC GGCGTTTATC GATAATACTA TCCGTCAGAG 50
TGCTTACTGT TTCCCACGCT TAACGGAGCT GGATGTGTTG ACATCAGCGT 100
<210> 8145
<211> 100
<212> DNA
<213> Escherichia coli K-12 MG1655
<220> ydjc b1734
<222> 498-597
<223> ydjc b1734 U00096 complement(1814410..1815159)
<313> 8145: 565-584
<400> 8145
CCAGGGATTC AGCAGTGCAT TCTACGGTGA AGAGATCAGT GAGTCGCTGT 50
TCCTGCAAGT GCTGGATGAC GCAGGCCATC GGGGTGATCG TTCGCTGGAG 100
<210> 8146
<211> 100
<212> DNA

<213> Escherichia coli K-12 MG1655
<220> ydje ydjE
<222> 1260-1359
<223> ydje ydjE U00096 complement(1850645..1852003)
<313> 8146: 1315-1334
<400> 8146
GGTACTTGGT GTTATGTTAT TGCTCTGTGC GCTGGTTCTC TCCATTTTTG 50
GCATCGAAAC GCGGAAGGTG TCGTTGGAAG AGATTTCTGA GGTGAATTAA 100
<210> 8147
<211> 100
<212> DNA
<213> Escherichia coli K-12 MG1655
<220> ydje ydjE
<222> 1252-1351
<223> ydje ydjE U00096 complement(1850645..1852003)
<313> 8147: 1274-1293
<400> 8147
GTGTTTATGG TACTTGGTGT TATGTTATTG CTCTGTGCGC TGGTTCTCTC 50
CATTTTTGGC ATCGAAACGC GGAAGGTGTC GTTGGAAGAG ATTTCTGAGG 100
<210> 8148
<211> 100
<212> DNA
<213> Escherichia coli K-12 MG1655
<220> ydje ydjE
<222> 1125-1224
<223> ydje ydjE U00096 complement(1850645..1852003)
<313> 8148: 1186-1205
<400> 8148
CCCGGAGCTT TGGCCAACGC ATTTACGCCT GCGCGGTTCG GGTTTCGTTA 50
ATGCCGTCGG ACGGATCGTC GCAGTCTTCA CGCCCTATGG CGTTGCGGCA 100
<210> 8149
<211> 100
<212> DNA
<213> Escherichia coli K-12 MG1655
<220> chea b1889
<222> 1866-1965
<223> chea b1889 U00096 complement(1971384..1973348)
<313> 8149: 1945-1964
<400> 8149
TGCGACCATT CTTGGCGACG GCAGCGTGGC ACTGATTGTT GATGTCTCCG 50
CCTTGCAGGC GATAAACCGC GAACAACGTA TGGCGAACAC CGCCGCCTGA 100
<210> 8150
<211> 100
<212> DNA
<213> Escherichia coli K-12 MG1655
<220> chea b1889
<222> 1832-1931
<223> chea b1889 U00096 complement(1971384..1973348)
<313> 8150: 1888-1907
<400> 8150
AAAGTAACTA TCGCAAAGTC CCCGGCATTT CTGCTGCGAC CATTCTTGGC 50
GACGGCAGCG TGGCACTGAT TGTTGATGTC TCCGCCTTGC AGGCGATAAA 100
<210> 8151
<211> 100
<212> DNA
<213> Escherichia coli K-12 MG1655
<220> chea b1889
<222> 1819-1918
<223> chea b1889 U00096 complement(1971384..1973348)
<313> 8151: 1849-1868
<400> 8151
GTTAAAAACC TTGAAAGTAA CTATCGCAAA GTCCCCGGCA TTTCTGCTGC 50
GACCATTCTT GGCGACGGCA GCGTGGCACT GATTGTTGAT GTCTCCGCCT 100
<210> 8152
<211> 100
<212> DNA
<213> Escherichia coli K-12 MG1655
<220> ydjj ydjJ
<222> 945-1044
<223> ydjj ydjJ U00096 complement(1855814..1856857)
<313> 8152: 976-995

<400> 8152
GACGCATATT TACGATTATC GGGATGTACA ACAGGCATTT GAAGAGTCAG 50
TTAACAACAA ACGCGACATT ATTAAAGGCG TTATTAAAAT TAGCGATTAA 100
<210> 8153
<211> 100
<212> DNA
<213> Escherichia coli K-12 MG1655
<220> ydjj ydjJ
<222> 872-971
<223> ydjj ydjJ U00096 complement(1855814..1856857)
<313> 8153: 927-946
<400> 8153
TCCGCTATGC CAATCGTTAT CCGGTCACGA TTGAAGCTAT TTCTTCAGGG 50
CGATTCGATG TGAAATCGAT GGTGACGCAT ATTTACGATT ATCGGGATGT 100
<210> 8154
<211> 100
<212> DNA
<213> Escherichia coli K-12 MG1655
<220> ydjj ydjJ
<222> 821-920
<223> ydjj ydjJ U00096 complement(1855814..1856857)
<313> 8154: 870-889
<400> 8154
CGGCAATCAA TTTCCTCAAA ATCAATCGCG AAGTCACTAT CCAGACGGTA 50
TTCCGCTATG CCAATCGTTA TCCGGTCACG ATTGAAGCTA TTTCTTCAGG 100
<210> 8155
<211> 100
<212> DNA
<213> Escherichia coli K-12 MG1655
<220> cheb b1884
<222> 951-1050
<223> cheb b1884 U00096 complement(1965476..1966525)
<313> 8155: 1024-1043
<400> 8155
GGCCATCAAT ATGGGTGGTG TCTGCGAAGT GGTCGATCTT AGCCAGGTAA 50
GCCAGCAAAT GTTGGCAAAA ATTAGTGCCG GACAGGCGAT ACGTATTTAA 100
<210> 8156
<211> 100
<212> DNA
<213> Escherichia coli K-12 MG1655
<220> cheb b1884
<222> 816-915
<223> cheb b1884 U00096 complement(1965476..1966525)
<313> 8156: 850-869
<400> 8156
GCGTAATGCG GTTGGGGTGA TCCTGACCGG TATGGGCAAC GACGGCGCGG 50
CGGGAATGTT GGCGATGCGT CAGGCGGGGG CATGGACCCT TGCGCAAAAC 100
<210> 8157
<211> 100
<212> DNA
<213> Escherichia coli K-12 MG1655
<220> cheb b1884
<222> 774-873
<223> cheb b1884 U00096 complement(1965476..1966525)
<313> 8157: 804-823
<400> 8157
TTCGGTAGAT GTGTTGTTCC ATTCTGTCGC CAAACAGGCG GGGCGTAATG 50
CGGTTGGGGT GATCCTGACC GGTATGGGCA ACGACGGCGC GGCGGGAATG 100
<210> 8158
<211> 100
<212> DNA
<213> Escherichia coli K-12 MG1655
<220> b1781 b1782
<222> 754-853
<223> b1781 b1782 U00096 complement(1862806..1863660)
<313> 8158: 818-837
<400> 8158
AATGCGGCTG TGCTTGAGGT CGAACTTTCT TCAGCGGAAT TAGCTATGCT 50
GGATAAGGCA TATCCGGCAC CAAAAGGAAA AACTGCGCTG GATATGGTGT 100
<210> 8159
<211> 100

<212> DNA
<213> Escherichia coli K-12 MG1655
<220> b1781 b1782
<222> 721-820
<223> b1781 b1782 U00096 complement(1862806..1863660)
<313> 8159: 773-792
<400> 8159
CCAAAAGCGG CCACGATTGC CCATGTCCAA CAAAATGCGG CTGTGCTTGA 50
GGTCGAACTT TCTTCAGCGG AATTAGCTAT GCTGGATAAG GCATATCCGG 100
<210> 8160
<211> 100
<212> DNA
<213> Escherichia coli K-12 MG1655
<220> b1781 b1782
<222> 686-785
<223> b1781 b1782 U00096 complement(1862806..1863660)
<313> 8160: 727-746
<400> 8160
CGTGGGTGAT CAGTCATCAG GGTGTGATGG CGATTCCAAA AGCGGCCACG 50
ATTGCCCATG TCCAACAAAA TGCGGCTGTG CTTGAGGTCG AACTTTCTTC 100
<210> 8161
<211> 100
<212> DNA
<213> Escherichia coli K-12 MG1655
<220> b1788 b1789
<222> 54-153
<223> b1788 b1789 U00096 complement(1872102..1872254)
<313> 8161: 119-138
<400> 8161
CGATCAAATG TTTGTCGAGA CACTGATTAT CACGTCATCG TTTTTTGCCA 50
TTGCTGTTGT ACTGGTTTTG TCCGTTCTTC TTATCGAACG GACCGGCTAG 100
<210> 8162
<211> 100
<212> DNA
<213> Escherichia coli K-12 MG1655
<220> b1788 b1789
<222> 54-153
<223> b1788 b1789 U00096 complement(1872102..1872254)
<313> 8162: 97-116
<400> 8162
CGATCAAATG TTTGTCGAGA CACTGATTAT CACGTCATCG TTTTTTGCCA 50
TTGCTGTTGT ACTGGTTTTG TCCGTTCTTC TTATCGAACG GACCGGCTAG 100
<210> 8163
<211> 100
<212> DNA
<213> Escherichia coli K-12 MG1655
<220> b1788 b1789
<222> 45-144
<223> b1788 b1789 U00096 complement(1872102..1872254)
<313> 8163: 99-118
<400> 8163
TGCTATGAAC GATCAAATGT TTGTCGAGAC ACTGATTATC ACGTCATCGT 50
TTTTTGCCAT TGCTGTTGTA CTGGTTTTGT CCGTTCTTCT TATCGAACGG 100
<210> 8164
<211> 100
<212> DNA
<213> Escherichia coli K-12 MG1655
<220> ydjs ydjS
<222> 803-902
<223> ydjs ydjS U00096 complement(1823979..1824947)
<313> 8164: 828-847
<400> 8164
TGGCAAGTGA CACGCTGAAT TTTATGCCGT TTGAGAAAGG AACATTGCTG 50
GCGCAGGACG GAGAGGAACG TTTTACCGTA ACCCATGATG TAGAGTATGT 100
<210> 8165
<211> 100
<212> DNA
<213> Escherichia coli K-12 MG1655
<220> ydjs ydjS
<222> 750-849
<223> ydjs ydjS U00096 complement(1823979..1824947)

<313> 8165: 790-809
<400> 8165
TTATCGGGTG GTTTCGCAAA TTACTCGCCA CTCGCCGTCC TTCGAAATGC  50
ATATGGCAAG TGACACGCTG AATTTTATGC CGTTTGAGAA AGGAACATTG  100
<210> 8166
<211> 100
<212> DNA
<213> Escherichia coli K-12 MG1655
<220> ydjs ydjS
<222> 609-708
<223> ydjs ydjS U00096 complement(1823979..1824947)
<313> 8166: 668-687
<400> 8166
CGCGCTGGCC TGTACGCTGG AACTTGGCAA AGCGTTGCCC TTTGGGCAAA  50
ACGATCTTCG CCAGTTTGCA GTAACTGCCA GCGCAATTGC TGCGCTGCTA  100
<210> 8167
<211> 100
<212> DNA
<213> Escherichia coli K-12 MG1655
<220> ydjx b1751
<222> 557-656
<223> ydjx b1751 U00096 1831377..1832135
<313> 8167: 608-627
<400> 8167
TACCCGGTAT TGTTATTTAT ACCGTGATGG CAAGCGATCT CGCCAATGAA  50
GGCATTACGC TGCGCTTTAT TTTACAACTC TGTCTGGCGG GCCTGGCGCT  100
<210> 8168
<211> 100
<212> DNA
<213> Escherichia coli K-12 MG1655
<220> ydjx b1751
<222> 490-589
<223> ydjx b1751 U00096 1831377..1832135
<313> 8168: 538-557
<400> 8168
AATTACGCTT ACGGATTAAC CACAATCGCC TTCTGGCCTT ATACCCTTAT  50
TTCGGCACTC ACGACCCTAC CCGGTATTGT TATTTATACC GTGATGGCAA  100
<210> 8169
<211> 100
<212> DNA
<213> Escherichia coli K-12 MG1655
<220> ydjx b1751
<222> 472-571
<223> ydjx b1751 U00096 1831377..1832135
<313> 8169: 506-525
<400> 8169
TTTCCTTACA ATATTCAAAA TTACGCTTAC GGATTAACCA CAATCGCCTT  50
CTGGCCTTAT ACCCTTATTT CGGCACTCAC GACCCTACCC GGTATTGTTA  100
<210> 8170
<211> 100
<212> DNA
<213> Escherichia coli K-12 MG1655
<220> ydjy b1752
<222> 741-840
<223> ydjy b1752 U00096 1831978..1832817
<313> 8170: 812-831
<400> 8170
TGCGGTTGAA AAACGTGGTG AAGTTAAATT CAAAGGCAAT GCCTCAGTTC  50
TCCCGGCGGA TAACACGCTG GCAACGGTTA CCTTTAAAAT CGCCGAATAA  100
<210> 8171
<211> 100
<212> DNA
<213> Escherichia coli K-12 MG1655
<220> ydjy b1752
<222> 731-830
<223> ydjy b1752 U00096 1831978..1832817
<313> 8171: 777-796
<400> 8171
ACACTTATGG TGCGGTTGAA AAACGTGGTG AAGTTAAATT CAAAGGCAAT  50
GCCTCAGTTC TCCCGGCGGA TAACACGCTG GCAACGGTTA CCTTTAAAAT  100
<210> 8172

<211> 100
<212> DNA
<213> Escherichia coli K-12 MG1655
<220> ydjy b1752
<222> 671-770
<223> ydjy b1752 U00096 1831978..1832817
<313> 8172: 734-753
<400> 8172
AAACGGGTTG CCTGGTGTGT CTGGATAGCT GCCCGGTCGG CATCGTCAGC 50
AATGCAACAT ACACTTATGG TGCGGTTGAA AAACGTGGTG AAGTTAAATT 100
<210> 8173
<211> 100
<212> DNA
<213> Escherichia coli K-12 MG1655
<220> ydjz b1753
<222> 609-708
<223> ydjz b1753 U00096 1832832..1833539
<313> 8173: 684-703
<400> 8173
TTTCTGGTTT GTCACCGGAC TGTTTATTCT GTTTGCCCTG ACCGTGGTGA 50
TTTTTATGGC GAAGAAAATA TGGCTTGAAC GCCAGAAGAG GAATGCCTGA 100
<210> 8174
<211> 100
<212> DNA
<213> Escherichia coli K-12 MG1655
<220> ydjz b1753
<222> 604-703
<223> ydjz b1753 U00096 1832832..1833539
<313> 8174: 620-639
<400> 8174
GGTACTTTCT GGTTTGTCAC CGGACTGTTT ATTCTGTTTG CCCTGACCGT 50
GGTGATTTTT ATGGCGAAGA AAATATGGCT TGAACGCCAG AAGAGGAATG 100
<210> 8175
<211> 100
<212> DNA
<213> Escherichia coli K-12 MG1655
<220> ydjz b1753
<222> 533-632
<223> ydjz b1753 U00096 1832832..1833539
<313> 8175: 558-577
<400> 8175
TTATCGCCAC CGGGCTTGGT CAGTTACCGG CAACTATTGT TTATTCCTGG 50
GCGGGCAGCA TGTTAACAGG CGGTACTTTC TGGTTTGTCA CCGGACTGTT 100
<210> 8176
<211> 100
<212> DNA
<213> Escherichia coli K-12 MG1655
<220> cher b1885
<222> 750-849
<223> cher b1885 U00096 complement(1966528..1967388)
<313> 8176: 805-824
<400> 8176
GCTCCTTAAA CCCGACGGAT TGCTGTTTGC GGGTCACTCT GAAAACTTTA 50
GCCACCTTGA GCGCCGCTTC ACGCTGCGTG GTCAGACGGT GTATGCGCTA 100
<210> 8177
<211> 100
<212> DNA
<213> Escherichia coli K-12 MG1655
<220> cher b1885
<222> 713-812
<223> cher b1885 U00096 complement(1966528..1967388)
<313> 8177: 759-778
<400> 8177
AAACTACCCA GCAGGAGATT TTGCGCCGCT TTGTTCCGCT CCTTAAACCC 50
GACGGATTGC TGTTTGCGGG TCACTCTGAA AACTTTAGCC ACCTTGAGCG 100
<210> 8178
<211> 100
<212> DNA
<213> Escherichia coli K-12 MG1655
<220> cher b1885
<222> 679-778

<223> cher b1885 U00096 complement(1966528..1967388)
<313> 8178: 727-746
<400> 8178
ATCTTCTGTC GTAACGTCAT GATCTACTTC GATCAAACTA CCCAGCAGGA 50
GATTTTGCGC CGCTTTGTTC CGCTCCTTAA ACCCGACGGA TTGCTGTTTG 100
<210> 8179
<211> 100
<212> DNA
<213> Escherichia coli K-12 MG1655
<220> chew b1888
<222> 404-503
<223> chew b1888 U00096 complement(1970860..1971363)
<313> 8179: 442-461
<400> 8179
GCGCACTGGG CGACCGGATG TTGATTCTGG TGAACATCGA AAAACTGCTG 50
AACAGCGAAG AGATGGCGCT GTTAGATAGC GCGGCGTCAG AAGTGGCGTA 100
<210> 8180
<211> 100
<212> DNA
<213> Escherichia coli K-12 MG1655
<220> chew b1888
<222> 361-460
<223> chew b1888 U00096 complement(1970860..1971363)
<313> 8180: 392-411
<400> 8180
GAATTTGCCG TGACGCTTTC AACAGAATAT CTCACTGGAC TGGGCGCACT 50
GGGCGACCGG ATGTTGATTC TGGTGAACAT CGAAAAACTG CTGAACAGCG 100
<210> 8181
<211> 100
<212> DNA
<213> Escherichia coli K-12 MG1655
<220> chew b1888
<222> 337-436
<223> chew b1888 U00096 complement(1970860..1971363)
<313> 8181: 365-384
<400> 8181
GCGGAGCAAA TTCGTCCGGC ACCGGAATTT GCCGTGACGC TTTCAACAGA 50
ATATCTCACT GGACTGGGCG CACTGGGCGA CCGGATGTTG ATTCTGGTGA 100
<210> 8182
<211> 100
<212> DNA
<213> Escherichia coli K-12 MG1655
<220> chey b1883
<222> 291-390
<223> chey b1883 U00096 complement(1965072..1965461)
<313> 8182: 355-374
<400> 8182
TGCGGCGCAA GCGGGGGCCA GTGGCTATGT GGTGAAGCCA TTTACCGCCG 50
CGACGCTGGA GGAAAAACTC AACAAAATCT TTGAGAAACT GGGCATGTGA 100
<210> 8183
<211> 100
<212> DNA
<213> Escherichia coli K-12 MG1655
<220> chey b1883
<222> 159-258
<223> chey b1883 U00096 complement(1965072..1965461)
<313> 8183: 202-221
<400> 8183
TGTTATCTCC GACTGGAACA TGCCCAATAT GGATGGCCTG GAATTGCTGA 50
AAACAATTCG TGCGGATGGC GCGATGTCGG CATTGCCAGT GTTAATGGTG 100
<210> 8184
<211> 100
<212> DNA
<213> Escherichia coli K-12 MG1655
<220> chey b1883
<222> 125-224
<223> chey b1883 U00096 complement(1965072..1965461)
<313> 8184: 153-172
<400> 8184
CTCTCAATAA GTTGCAGGCA GGCGGTTATG GATTTGTTAT CTCCGACTGG 50
AACATGCCCA ATATGGATGG CCTGGAATTG CTGAAAACAA TTCGTGCGGA 100

<210> 8185
<211> 100
<212> DNA
<213> Escherichia coli K-12 MG1655
<220> chez b1882
<222> 546-645
<223> chez b1882 U00096 complement(1964417..1965061)
<313> 8185: 620-639
<400> 8185
CCAGAGTTTG CTTAATGGAC CTCAGGTCGA TACCAGCAAA GCCGGTGTGG  50
TAGCCAGTCA GGATCAGGTG GACGATTTGT TGGATAGTCT TGGATTTTGA  100
<210> 8186
<211> 100
<212> DNA
<213> Escherichia coli K-12 MG1655
<220> chez b1882
<222> 541-640
<223> chez b1882 U00096 complement(1964417..1965061)
<313> 8186: 575-594
<400> 8186
GAAAACCAGA GTTTGCTTAA TGGACCTCAG GTCGATACCA GCAAAGCCGG  50
TGTGGTAGCC AGTCAGGATC AGGTGGACGA TTTGTTGGAT AGTCTTGGAT  100
<210> 8187
<211> 100
<212> DNA
<213> Escherichia coli K-12 MG1655
<220> chez b1882
<222> 493-592
<223> chez b1882 U00096 complement(1964417..1965061)
<313> 8187: 540-559
<400> 8187
ATGGTGCTGT TGGAAAACAT CCCGGAACAG GAGTCGCGTC CAAAACGTGA  50
AAACCAGAGT TTGCTTAATG GACCTCAGGT CGATACCAGC AAAGCCGGTG  100
<210> 8188
<211> 100
<212> DNA
<213> Escherichia coli K-12 MG1655
<220> uhpa b3670
<222> 482-581
<223> uhpa b3670 U00096 complement(3847763..3848353)
<313> 8188: 543-562
<400> 8188
AACTGGGCTT GTCACCGAAA ACGGTACACG TCCATCGCGC CAATCTGATG  50
GAAAAACTGG GCGTCAGTAA CGACGTAGAG CTGGCGCGCC GCATGTTTGA  100
<210> 8189
<211> 100
<212> DNA
<213> Escherichia coli K-12 MG1655
<220> uhpa b3670
<222> 449-548
<223> uhpa b3670 U00096 complement(3847763..3848353)
<313> 8189: 495-514
<400> 8189
CGCAAGGAAT GGCGGTGAAA GAGATTGCCG CCGAACTGGG CTTGTCACCG  50
AAAACGGTAC ACGTCCATCG CGCCAATCTG ATGGAAAAAC TGGGCGTCAG  100
<210> 8190
<211> 100
<212> DNA
<213> Escherichia coli K-12 MG1655
<220> uhpa b3670
<222> 425-524
<223> uhpa b3670 U00096 complement(3847763..3848353)
<313> 8190: 450-469
<400> 8190
AACGCCAGGT GGCGGAAAAA CTGGCGCAAG GAATGGCGGT GAAAGAGATT  50
GCCGCCGAAC TGGGCTTGTC ACCGAAAACG GTACACGTCC ATCGCGCCAA  100
<210> 8191
<211> 100
<212> DNA
<213> Escherichia coli K-12 MG1655
<220> uhpb b3669

<222> 1388-1487
<223> uhpb b3669 U00096 complement(3846261..3847766)
<313> 8191: 1433-1452
<400> 8191
AACAAGGTTT TGGCCTCACC GGAATGCGCG AGCGCGTAAC GGCGCTGGGT  50
GGCACATTAC ACATTTCCTG TCTGCACGGC ACGCGTGTCA GCGTTTCTCT  100
<210> 8192
<211> 100
<212> DNA
<213> Escherichia coli K-12 MG1655
<220> uhpb b3669
<222> 1312-1411
<223> uhpb b3669 U00096 complement(3846261..3847766)
<313> 8192: 1375-1394
<400> 8192
CAAGGCTGGC AGCAGGATGA ACGGTTGATG CTGGTTATTG AAGACGATGG  50
CAGCGGTTTG CCGCCGGGTT CCGGGCAACA AGGTTTTGGC CTCACCGGAA  100
<210> 8193
<211> 100
<212> DNA
<213> Escherichia coli K-12 MG1655
<220> uhpb b3669
<222> 1275-1374
<223> uhpb b3669 U00096 complement(3846261..3847766)
<313> 8193: 1320-1339
<400> 8193
CATTGTGAAA CATGCTGATG CCAGCGCGGT CACCCTGCAA GGCTGGCAGC  50
AGGATGAACG GTTGATGCTG GTTATTGAAG ACGATGGCAG CGGTTTGCCG  100
<210> 8194
<211> 100
<212> DNA
<213> Escherichia coli K-12 MG1655
<220> uhpc b3668
<222> 1224-1323
<223> uhpc b3668 U00096 complement(3844932..3846254)
<313> 8194: 1286-1305
<400> 8194
GCACTGGAGC GGATTTTTTG TGGTTATCTC TATCGCCGCC GGGATTTCCG  50
CACTGCTGTT ACTGCCCTTT TTGAACGCCC AGACACCGCG CGAAGCGTGA  100
<210> 8195
<211> 100
<212> DNA
<213> Escherichia coli K-12 MG1655
<220> uhpc b3668
<222> 1200-1299
<223> uhpc b3668 U00096 complement(3844932..3846254)
<313> 8195: 1240-1259
<400> 8195
GCTGGCGAAA GTACTCGATA CCTGGCACTG GAGCGGATTT TTTGTGGTTA  50
TCTCTATCGC CGCCGGGATT TCCGCACTGC TGTTACTGCC CTTTTTGAAC  100
<210> 8196
<211> 100
<212> DNA
<213> Escherichia coli K-12 MG1655
<220> uhpc b3668
<222> 1143-1242
<223> uhpc b3668 U00096 complement(3844932..3846254)
<313> 8196: 1161-1180
<400> 8196
GGCGACGGGG TTTGTCGGCT TGTTTGCTTA TCTGGGGGCG TCGCTTGCTG  50
GTTGGCCGCT GGCGAAAGTA CTCGATACCT GGCACTGGAG CGGATTTTTT  100
<210> 8197
<211> 100
<212> DNA
<213> Escherichia coli K-12 MG1655
<220> rbfa b3168
<222> 303-402
<223> rbfa b3168 U00096 complement(3310418..3310819)
<313> 8197: 368-387
<400> 8197
CTCTCTGGTT GAAGGGATGC GCATGTCAAA CCTGGTGACC AGCGTGGTCA  50

AACATGACGA AGAACGTCGT GTTAACCCGG ACGACAGCAA GGAGGACTAA 100
<210> 8198
<211> 100
<212> DNA
<213> Escherichia coli K-12 MG1655
<220> rbfa b3168
<222> 246-345
<223> rbfa b3168 U00096 complement(3310418..3310819)
<313> 8198: 292-311
<400> 8198
GCTGGGGAAA GCGATGCGCC TGCGTATCGT GCCGGAACTG ACCTTCTTCT 50
ACGACAACTC TCTGGTTGAA GGGATGCGCA TGTCAAACCT GGTGACCAGC 100
<210> 8199
<211> 100
<212> DNA
<213> Escherichia coli K-12 MG1655
<220> rbfa b3168
<222> 181-280
<223> rbfa b3168 U00096 complement(3310418..3310819)
<313> 8199: 246-265
<400> 8199
GATGAAGACG CGGTTAAAGC GGGCATCAAA GCGTTGCAAG AAGCTTCTGG 50
TTTCATCCGC AGCCTGCTGG GGAAAGCGAT GCGCCTGCGT ATCGTGCCGG 100
<210> 8200
<211> 100
<212> DNA
<213> Escherichia coli K-12 MG1655
<220> lldd b3606
<222> 1066-1165
<223> lldd b3606 U00096 3777454..3778644
<313> 8200: 1087-1106
<400> 8200
AAAGTGGCGA TGACGCTGAC TGGCGCGAAA TCGATCAGCG AAATTACGCA 50
AGATTCGCTG GTGCAGGGGC TGGGTAAAGA GTTGCCTGCG GCACTGGCTC 100
<210> 8201
<211> 100
<212> DNA
<213> Escherichia coli K-12 MG1655
<220> lldd b3606
<222> 951-1050
<223> lldd b3606 U00096 3777454..3778644
<313> 8201: 1010-1029
<400> 8201
TATGATTGCG CTCGGTGCCG ACACCGTACT GCTGGGTCGT GCTTTCTTGT 50
ATGCGCTGGC AACAGCGGGC CAGGCGGGTG TAGCTAACCT GCTAAATCTG 100
<210> 8202
<211> 100
<212> DNA
<213> Escherichia coli K-12 MG1655
<220> lldd b3606
<222> 878-977
<223> lldd b3606 U00096 3777454..3778644
<313> 8202: 896-915
<400> 8202
TTGCAGATGC GGTGAAAGGT GATATAGCCA TTCTGGCGGA TAGCGGAATT 50
CGTAACGGGC TTGATGTCGT GCGTATGATT GCGCTCGGTG CCGACACCGT 100
<210> 8203
<211> 100
<212> DNA
<213> Escherichia coli K-12 MG1655
<220> uhpt b3667
<222> 1293-1392
<223> uhpt b3667 U00096 complement(3843403..3844794)
<313> 8203: 1363-1382
<400> 8203
CGCCGCGATT GGTTGTATCT GCCTGATGGC GATAGTGGCG GTAATGGAAG 50
AACGCAAAAT CCGCCGCGAG AAAAAAATTC AGCAGTTGAC AGTGGCATAA 100
<210> 8204
<211> 100
<212> DNA
<213> Escherichia coli K-12 MG1655

<220> uhpt b3667
<222> 1265-1364
<223> uhpt b3667 U00096 complement(3843403..3844794)
<313> 8204: 1326-1345
<400> 8204
GGGCAGGCAC CTTCGCCGCG CTGGATATCG CCGCGATTGG TTGTATCTGC 50
CTGATGGCGA TAGTGGCGGT AATGGAAGAA CGCAAAATCC GCCGCGAGAA 100
<210> 8205
<211> 100
<212> DNA
<213> Escherichia coli K-12 MG1655
<220> uhpt b3667
<222> 1242-1341
<223> uhpt b3667 U00096 complement(3843403..3844794)
<313> 8205: 1285-1304
<400> 8205
GCCGGTATTC GGCCTTACCG GCTGGGCAGG CACCTTCGCC GCGCTGGATA 50
TCGCCGCGAT TGGTTGTATC TGCCTGATGG CGATAGTGGC GGTAATGGAA 100
<210> 8206
<211> 100
<212> DNA
<213> Escherichia coli K-12 MG1655
<220> lldp b3604
<222> 1557-1656
<223> lldp b3604 U00096 3775026..3776681
<313> 8206: 1633-1652
<400> 8206
TTTGTTCCGC TTTACTGTCA AACACAGCCT GATCTTCACC TGTATAGTGG 50
GCGTGATCAC CACGCTTCAG GCTTATGTCT TAACGTGGAT GATTCCTTAA 100
<210> 8207
<211> 100
<212> DNA
<213> Escherichia coli K-12 MG1655
<220> lldp b3604
<222> 1517-1616
<223> lldp b3604 U00096 3775026..3776681
<313> 8207: 1574-1593
<400> 8207
TCGCCTGTGC GGCGGTAGGC CTGGTGGGCA AAGAGTCTGA TTTGTTCCGC 50
TTTACTGTCA AACACAGCCT GATCTTCACC TGTATAGTGG GCGTGATCAC 100
<210> 8208
<211> 100
<212> DNA
<213> Escherichia coli K-12 MG1655
<220> lldp b3604
<222> 1476-1575
<223> lldp b3604 U00096 3775026..3776681
<313> 8208: 1500-1519
<400> 8208
TGGCGTCACC GGTAAGATGA TCTCCCCGCA ATCTATCGCT ATCGCCTGTG 50
CGGCGGTAGG CCTGGTGGGC AAAGAGTCTG ATTTGTTCCG CTTTACTGTC 100
<210> 8209
<211> 100
<212> DNA
<213> Escherichia coli K-12 MG1655
<220> lldr b3605
<222> 678-777
<223> lldr b3605 U00096 3776681..3777457
<313> 8209: 753-772
<400> 8209
CACCACCATG AAACGATTCG ATGAAGATCA GGCTCGCCAC GCACGGATTA 50
CCCGCCTGCC CGGTGAGCAT AATGAGCATT CGAGGGAGAA AAACGCATGA 100
<210> 8210
<211> 100
<212> DNA
<213> Escherichia coli K-12 MG1655
<220> lldr b3605
<222> 678-777
<223> lldr b3605 U00096 3776681..3777457
<313> 8210: 709-728
<400> 8210

CACCACCATG AAACGATTCG ATGAAGATCA GGCTCGCCAC GCACGGATTA 50
CCCGCCTGCC CGGTGAGCAT AATGAGCATT CGAGGGAGAA AAACGCATGA 100
<210> 8211
<211> 100
<212> DNA
<213> Escherichia coli K-12 MG1655
<220> lldr b3605
<222> 614-713
<223> lldr b3605 U00096 3776681..3777457
<313> 8211: 670-689
<400> 8211
CCATTTTTGC CGGTGATGCT GACGGGGCGC GTAAAGCAAT GATGGCGCAC 50
CTTAGTTTTG TTCACACCAC CATGAAACGA TTCGATGAAG ATCAGGCTCG 100
<210> 8212
<211> 100
<212> DNA
<213> Escherichia coli K-12 MG1655
<220> b3913 b3913
<222> 51-150
<223> b3913 b3913 U00096 4103398..4103547
<313> 8212: 121-140
<400> 8212
TTCATTAAGC CACGCTGCTG AAGTCGGTTC AGGCGATAAC TGGCATCCGG 50
GTGAAGAACT TACGCAGCGC AGACGCAGAG CCATATGTTC GACGGCATAA 100
<210> 8213
<211> 100
<212> DNA
<213> Escherichia coli K-12 MG1655
<220> b3913 b3913
<222> 51-150
<223> b3913 b3913 U00096 4103398..4103547
<313> 8213: 114-133
<400> 8213
TTCATTAAGC CACGCTGCTG AAGTCGGTTC AGGCGATAAC TGGCATCCGG 50
GTGAAGAACT TACGCAGCGC AGACGCAGAG CCATATGTTC GACGGCATAA 100
<210> 8214
<211> 100
<212> DNA
<213> Escherichia coli K-12 MG1655
<220> b3913 b3913
<222> 7-106
<223> b3913 b3913 U00096 4103398..4103547
<313> 8214: 69-88
<400> 8214
CGCATAGTTA CCGCTGCCGT CATGGCCTCA ACGCTGGCAG TCAGTTCATT 50
AAGCCACGCT GCTGAAGTCG GTTCAGGCGA TAACTGGCAT CCGGGTGAAG 100
<210> 8215
<211> 100
<212> DNA
<213> Escherichia coli K-12 MG1655
<220> b3914 b3915
<222> 270-369
<223> b3914 b3915 U00096 4103532..4103900
<313> 8215: 335-354
<400> 8215
GAAACATCAA CAACGAATGG AGCAGTTGCG TGACGTGACG CAATGGCAAA 50
AAAGTTCATC GTTGAAGCTA TTGAGTAGTA GCAACTCACG TTCCCAGTAG 100
<210> 8216
<211> 100
<212> DNA
<213> Escherichia coli K-12 MG1655
<220> b3914 b3915
<222> 223-322
<223> b3914 b3915 U00096 4103532..4103900
<313> 8216: 262-281
<400> 8216
CAAATGTATC GCCTGTTAAC GCCGGAGCAG CAAGCGGTTT TAAACGAGAA 50
ACATCAACAA CGAATGGAGC AGTTGCGTGA CGTGACGCAA TGGCAAAAAA 100
<210> 8217
<211> 100
<212> DNA

<213> Escherichia coli K-12 MG1655
<220> b3914 b3915
<222> 171-270
<223> b3914 b3915 U00096 4103532..4103900
<313> 8217: 204-223
<400> 8217
AATGGCGAAT GAGCAAATTG CTCGTCAGGT TGAGATGGCA AAAGTCCGCA 50
ACCAAATGTA TCGCCTGTTA ACGCCGGAGC AGCAAGCGGT TTTAAACGAG 100
<210> 8218
<211> 100
<212> DNA
<213> Escherichia coli K-12 MG1655
<220> pota b1127
<222> 1038-1137
<223> pota b1127 U00096 complement(1183681..1184817)
<313> 8218: 1100-1119
<400> 8218
TGAAGACGAT CCTGACTTTG ACCACTCTCT CGACCAAAAA ATGGCCATTA 50
ATTGGGTAGA AAGCTGGGAG GTCGTACTGG CTGATGAAGA ACACAAGTAA 100
<210> 8219
<211> 100
<212> DNA
<213> Escherichia coli K-12 MG1655
<220> pota b1127
<222> 1016-1115
<223> pota b1127 U00096 complement(1183681..1184817)
<313> 8219: 1051-1070
<400> 8219
TGATGGTCAG CGAATTCTTC AATGAAGACG ATCCTGACTT TGACCACTCT 50
CTCGACCAAA AAATGGCCAT TAATTGGGTA GAAAGCTGGG AGGTCGTACT 100
<210> 8220
<211> 100
<212> DNA
<213> Escherichia coli K-12 MG1655
<220> pota b1127
<222> 957-1056
<223> pota b1127 U00096 complement(1183681..1184817)
<313> 8220: 1016-1035
<400> 8220
GCGTAACTAC AAAGGCATGA CGCTGGAGTC GGTTGTTGAA CTGGAAAATG 50
GCAAGATGGT GATGGTCAGC GAATTCTTCA ATGAAGACGA TCCTGACTTT 100
<210> 8221
<211> 100
<212> DNA
<213> Escherichia coli K-12 MG1655
<220> potb b1126
<222> 690-789
<223> potb b1126 U00096 complement(1182840..1183667)
<313> 8221: 727-746
<400> 8221
CAAGGTCCAG TTCCTTAATA TTCGTGACTG GCCGTTTGGT GCAGCTACCA 50
GCATTACGCT GACTATCGTA ATGGGCCTGA TGTTGCTGGT TTACTGGCGC 100
<210> 8222
<211> 100
<212> DNA
<213> Escherichia coli K-12 MG1655
<220> potb b1126
<222> 619-718
<223> potb b1126 U00096 complement(1182840..1183667)
<313> 8222: 640-659
<400> 8222
CCAGCGATGG GCCTGTTCTA TGTATCCGAC CTGATGGGCG GTGCGAAAAA 50
CCTGCTGATC GGTAACGTCA TCAAGGTCCA GTTCCTTAAT ATTCGTGACT 100
<210> 8223
<211> 100
<212> DNA
<213> Escherichia coli K-12 MG1655
<220> potb b1126
<222> 533-632
<223> potb b1126 U00096 complement(1182840..1183667)
<313> 8223: 576-595

<400> 8223
GTGCCAGCAA GTTACAGACT TTTATCCGTA TCATTATTCC ACTGACGATG 50
CCGGGAATTA TTGCCGGATG TCTGCTGGTG ATGCTGCCAG CGATGGGCCT 100
<210> 8224
<211> 100
<212> DNA
<213> Escherichia coli K-12 MG1655
<220> potc b1125
<222> 696-795
<223> potc b1125 U00096 complement(1182049..1182843)
<313> 8224: 765-784
<400> 8224
GCTGGCAACC ATATTACTGG TGCTGTCGCT GGTGATGGTT ATTGCCAGCC 50
AGCTTATTGC TCGTGATAAA ACGAAAGGTA ACACAGGGGA CGTTAAATGA 100
<210> 8225
<211> 100
<212> DNA
<213> Escherichia coli K-12 MG1655
<220> potc b1125
<222> 639-738
<223> potc b1125 U00096 complement(1182049..1182843)
<313> 8225: 688-707
<400> 8225
TCTGCCGTTA AAAATTTATT CGATGGTCAA AGTCGGCGTA TCGCCGGAAG 50
TTAACGCGCT GGCAACCATA TTACTGGTGC TGTCGCTGGT GATGGTTATT 100
<210> 8226
<211> 100
<212> DNA
<213> Escherichia coli K-12 MG1655
<220> potc b1125
<222> 575-674
<223> potc b1125 U00096 complement(1182049..1182843)
<313> 8226: 621-640
<400> 8226
GCTTTACCCT GTCGATGGAC GATGTGGTAG TTTCTTCGTT CGTCACCGGA 50
CCAAGTTATG AAATTCTGCC GTTAAAAATT TATTCGATGG TCAAAGTCGG 100
<210> 8227
<211> 100
<212> DNA
<213> Escherichia coli K-12 MG1655
<220> potd b1124
<222> 948-1047
<223> potd b1124 U00096 complement(1181006..1182052)
<313> 8227: 994-1013
<400> 8227
GGATGCTGAA ACCATTAAAA ATGGCGAATG GCAGAATGAC GTTGGCGCAG 50
CCAGCAGCAT TTATGAAGAG TATTATCAGA AGCTGAAAGC AGGACGTTAA 100
<210> 8228
<211> 100
<212> DNA
<213> Escherichia coli K-12 MG1655
<220> potd b1124
<222> 930-1029
<223> potd b1124 U00096 complement(1181006..1182052)
<313> 8228: 948-967
<400> 8228
CGATAAAACA CTCTACCCGG ATGCTGAAAC CATTAAAAAT GGCGAATGGC 50
AGAATGACGT TGGCGCAGCC AGCAGCATTT ATGAAGAGTA TTATCAGAAG 100
<210> 8229
<211> 100
<212> DNA
<213> Escherichia coli K-12 MG1655
<220> potd b1124
<222> 853-952
<223> potd b1124 U00096 complement(1181006..1182052)
<313> 8229: 915-934
<400> 8229
AAACAGGTTG CTGAAACTAT CGGTTATCCA ACGCCAAACC TTGCGGCGCG 50
TAAGCTGTTA AGTCCAGAAG TGGCGAACGA TAAAACACTC TACCCGGATG 100
<210> 8230
<211> 100

<212> DNA
<213> Escherichia coli K-12 MG1655
<220> pote b0693
<222> 1196-1295
<223> pote b0693 U00096 complement(716169..717488)
<313> 8230: 1257-1276
<400> 8230
GCTTTTATGC GCTGTACTCA TCCGGGGAAG AAGCCATGCT GTACGGTTCC 50
ATCGTGACCT TCCTTGGTTG GACACTGTAT GGTCTGGTAT CACCACGCTT 100
<210> 8231
<211> 100
<212> DNA
<213> Escherichia coli K-12 MG1655
<220> pote b0693
<222> 1181-1280
<223> pote b0693 U00096 complement(716169..717488)
<313> 8231: 1209-1228
<400> 8231
TTGGCGCGAT GTATAGCTTT TATGCGCTGT ACTCATCCGG GGAAGAAGCC 50
ATGCTGTACG GTTCCATCGT GACCTTCCTT GGTTGGACAC TGTATGGTCT 100
<210> 8232
<211> 100
<212> DNA
<213> Escherichia coli K-12 MG1655
<220> pote b0693
<222> 1131-1230
<223> pote b0693 U00096 complement(716169..717488)
<313> 8232: 1161-1180
<400> 8232
CGCTAATGTG CCCCCCTCAA AAGCGAAAGT TGCAAACTTT GTTGCTTTTG 50
TTGGCGCGAT GTATAGCTTT TATGCGCTGT ACTCATCCGG GGAAGAAGCC 100
<210> 8233
<211> 100
<212> DNA
<213> Escherichia coli K-12 MG1655
<220> potf b0855
<222> 942-1041
<223> potf b0855 U00096 893007..894119
<313> 8233: 975-994
<400> 8233
TGCCAACGCC AATAAAGCAG CCACGCCGCT GGTGAGTGCG GAAGTCCGTG 50
AGAACCCAGG TATTTATCCG CCTGCGGATG TTCGTGCGAA GCTGTTCACT 100
<210> 8234
<211> 100
<212> DNA
<213> Escherichia coli K-12 MG1655
<220> potf b0855
<222> 908-1007
<223> potf b0855 U00096 893007..894119
<313> 8234: 947-966
<400> 8234
ATGTAGTAGC GCATATTTCC GACCATGTGT TCTATGCCAA CGCCAATAAA 50
GCAGCCACGC CGCTGGTGAG TGCGGAAGTC CGTGAGAACC CAGGTATTTA 100
<210> 8235
<211> 100
<212> DNA
<213> Escherichia coli K-12 MG1655
<220> potf b0855
<222> 878-977
<223> potf b0855 U00096 893007..894119
<313> 8235: 941-960
<400> 8235
ATCAGTTCCT GAATTACCTG CTGCGCCCGG ATGTAGTAGC GCATATTTCC 50
GACCATGTGT TCTATGCCAA CGCCAATAAA GCAGCCACGC CGCTGGTGAG 100
<210> 8236
<211> 100
<212> DNA
<213> Escherichia coli K-12 MG1655
<220> potg b0856
<222> 1106-1205
<223> potg b0856 U00096 894133..895347

<313> 8236: 1149-1168
<400> 8236
TTAGCGCCCA GCTACAAAAC GCCCATCGTC ATCGTAAAGG GTTACCGACC 50
TGGGGCGACG AAGTGCGTTT GTGCTGGGAA GTGGACAGCT GTGTGGTGCT 100
<210> 8237
<211> 100
<212> DNA
<213> Escherichia coli K-12 MG1655
<220> potg b0856
<222> 1072-1171
<223> potg b0856 U00096 894133..895347
<313> 8237: 1100-1119
<400> 8237
GTGTATCACG TTCGTCTGAA AAGTGGGCAG ATGATTAGCG CCCAGCTACA 50
AAACGCCCAT CGTCATCGTA AAGGGTTACC GACCTGGGGC GACGAAGTGC 100
<210> 8238
<211> 100
<212> DNA
<213> Escherichia coli K-12 MG1655
<220> potg b0856
<222> 1020-1119
<223> potg b0856 U00096 894133..895347
<313> 8238: 1063-1082
<400> 8238
TAACTTCGCG GTGGGGGAGG TGATACACAT TGCCTATCTC GGCGATCTTT 50
CGGTGTATCA CGTTCGTCTG AAAAGTGGGC AGATGATTAG CGCCCAGCTA 100
<210> 8239
<211> 100
<212> DNA
<213> Escherichia coli K-12 MG1655
<220> poth b0857
<222> 855-954
<223> poth b0857 U00096 895357..896310
<313> 8239: 930-949
<400> 8239
GGTGGCCTCG GCGGTAGCGA TCATCATGTT GCTGCTGCTA ATTGTGCCGA 50
TAATGTGGTT CCACAAACAC CAGCAAAAAA GCGTGGGAGA ACACGGATGA 100
<210> 8240
<211> 100
<212> DNA
<213> Escherichia coli K-12 MG1655
<220> poth b0857
<222> 844-943
<223> poth b0857 U00096 895357..896310
<313> 8240: 890-909
<400> 8240
CGCGACTGGC CGGTGGCCTC GGCGGTAGCG ATCATCATGT TGCTGCTGCT 50
AATTGTGCCG ATAATGTGGT TCCACAAACA CCAGCAAAAA AGCGTGGGAG 100
<210> 8241
<211> 100
<212> DNA
<213> Escherichia coli K-12 MG1655
<220> poth b0857
<222> 741-840
<223> poth b0857 U00096 895357..896310
<313> 8241: 773-792
<400> 8241
GGTGTTTATC CCGGCTGTGG GCGAGTTTGT GATCCCGGAA CTGCTCGGTG 50
GCCCGGACAG CATCATGATC GGGCGCGTGC TATGGCAAGA GTTTTTCAAT 100
<210> 8242
<211> 100
<212> DNA
<213> Escherichia coli K-12 MG1655
<220> poti b0858
<222> 747-846
<223> poti b0858 U00096 896307..897152
<313> 8242: 814-833
<400> 8242
CCTCGGCGCG GTCGGAATTG TCGGATTTAT CGCCTGGTAT CTGATGGCTC 50
GCGCAGAAAA ACAGCGGATA CGCGACATCC AGCGTGCAAG ACGTGGCTGA 100
<210> 8243

<211> 100
<212> DNA
<213> Escherichia coli K-12 MG1655
<220> poti b0858
<222> 724-823
<223> poti b0858 U00096 896307..897152
<313> 8243: 770-789
<400> 8243
ATCAACGCCC TGGCAACATT GATCCTCGGC GCGGTCGGAA TTGTCGGATT 50
TATCGCCTGG TATCTGATGG CTCGCGCAGA AAAACAGCGG ATACGCGACA 100
<210> 8244
<211> 100
<212> DNA
<213> Escherichia coli K-12 MG1655
<220> poti b0858
<222> 711-810
<223> poti b0858 U00096 896307..897152
<313> 8244: 746-765
<400> 8244
GGTGAATCCG GAAATCAACG CCCTGGCAAC ATTGATCCTC GGCGCGGTCG 50
GAATTGTCGG ATTTATCGCC TGGTATCTGA TGGCTCGCGC AGAAAAACAG 100
<210> 8245
<211> 100
<212> DNA
<213> Escherichia coli K-12 MG1655
<220> rbn b3887
<222> 768-867
<223> rbn b3887 U00096 4073726..4074598
<313> 8245: 780-799
<400> 8245
CTGGACATGG TGTATCGTCT TGCTTGGCGC GGAAATTACT GTCACTCTCG 50
GGGAATACCG CAAACTAAAA CAAGCAGCTG AACAAGAAGA AGACGACGAA 100
<210> 8246
<211> 100
<212> DNA
<213> Escherichia coli K-12 MG1655
<220> rbn b3887
<222> 714-813
<223> rbn b3887 U00096 4073726..4074598
<313> 8246: 741-760
<400> 8246
TCAGCTCATT TATGGTGTGC TGGCGGTGAT CCCCATTCTC TTTGTTTGGG 50
TCTACTGGAC ATGGTGTATC GTCTTGCTTG GCGCGGAAAT TACTGTCACT 100
<210> 8247
<211> 100
<212> DNA
<213> Escherichia coli K-12 MG1655
<220> rbn b3887
<222> 651-750
<223> rbn b3887 U00096 4073726..4074598
<313> 8247: 703-722
<400> 8247
CGCACTCCTG TTCGAAGCAG GAAAGAAAGG TTTCGCGCTT TATATCACCA 50
TGTTCCCGTC ATATCAGCTC ATTTATGGTG TGCTGGCGGT GATCCCCATT 100
<210> 8248
<211> 100
<212> DNA
<213> Escherichia coli K-12 MG1655
<220> gsha gshA
<222> 1458-1557
<223> gsha gshA U00096 complement(2812905..2814461)
<313> 8248: 1513-1532
<400> 8248
GGATTTTGTA GCCGAGCGCG AGGCGTCTGA ACGCCGTCAG CAGGAAATGG 50
AAGCCGCTGA TACCGAACCG TTTGCGGTGT GGCTGGAAAA ACACGCCTGA 100
<210> 8249
<211> 100
<212> DNA
<213> Escherichia coli K-12 MG1655
<220> gsha gshA
<222> 1398-1497

<223> gsha gshA U00096 complement(2812905..2814461)
<313> 8249: 1426-1445
<400> 8249
ATTTGCAGAA GCCTACCGTA ATCTGCTGCG TGAAGAGCCG CTGGAAATTC 50
TGCGCGAAGA GGATTTTGTA GCCGAGCGCG AGGCGTCTGA ACGCCGTCAG 100
<210> 8250
<211> 100
<212> DNA
<213> Escherichia coli K-12 MG1655
<220> gsha gshA
<222> 1276-1375
<223> gsha gshA U00096 complement(2812905..2814461)
<313> 8250: 1335-1354
<400> 8250
GGCGAAGCGT ATCAGAAAGT GTGTGATGAA CTGGTTGCCT GCTTCGATAA 50
TCCCGATCTG ACTTTCTCTG CCCGTATCTT AAGGTCTATG ATTGATACTG 100
<210> 8251
<211> 100
<212> DNA
<213> Escherichia coli K-12 MG1655
<220> gshb b2948
<222> 852-951
<223> gshb b2948 U00096 3089897..3090847
<313> 8251: 931-950
<400> 8251
CACCAGCCCA ACCTGTATTC GTGAGATTGA AGCAGAGTTT CCGGTGTCGA 50
TCACCGGAAT GTTAATGGAT GCCATCGAAG CACGTTTACA GCAGCAGTAA 100
<210> 8252
<211> 100
<212> DNA
<213> Escherichia coli K-12 MG1655
<220> gshb b2948
<222> 844-943
<223> gshb b2948 U00096 3089897..3090847
<313> 8252: 868-887
<400> 8252
ATTAACGTCA CCAGCCCAAC CTGTATTCGT GAGATTGAAG CAGAGTTTCC 50
GGTGTCGATC ACCGGAATGT TAATGGATGC CATCGAAGCA CGTTTACAGC 100
<210> 8253
<211> 100
<212> DNA
<213> Escherichia coli K-12 MG1655
<220> gshb b2948
<222> 776-875
<223> gshb b2948 U00096 3089897..3090847
<313> 8253: 838-857
<400> 8253
GGCCGACGCT GAAAGAAAAA GGGCTGATTT TTGTTGGTCT GGATATCATC 50
GGCGACCGTC TGACTGAAAT TAACGTCACC AGCCCAACCT GTATTCGTGA 100
<210> 8254
<211> 100
<212> DNA
<213> Escherichia coli K-12 MG1655
<220> mazg b2782
<222> 693-792
<223> mazg b2782 U00096 complement(2907916..2908707)
<313> 8254: 771-790
<400> 8254
GCGTATTGTT GCCGCGCGTG GACTGGAAAT GACAGGTGTT GACCTCGAAA 50
CAATGGAAGA AGTCTGGCAA CAGGTAAAAC GGCAGGAAAT TGATCTCTAA 100
<210> 8255
<211> 100
<212> DNA
<213> Escherichia coli K-12 MG1655
<220> mazg b2782
<222> 693-792
<223> mazg b2782 U00096 complement(2907916..2908707)
<313> 8255: 731-750
<400> 8255
GCGTATTGTT GCCGCGCGTG GACTGGAAAT GACAGGTGTT GACCTCGAAA 50
CAATGGAAGA AGTCTGGCAA CAGGTAAAAC GGCAGGAAAT TGATCTCTAA 100

<210> 8256
<211> 100
<212> DNA
<213> Escherichia coli K-12 MG1655
<220> mazg b2782
<222> 612-711
<223> mazg b2782 U00096 complement(2907916..2908707)
<313> 8256: 654-673
<400> 8256
GGCTCGCCAT TTAGGGACGA AAGCAGAAAT CGCATTGCAA AAAGCGAACG 50
AAAAATTCGA GCGTCGTTTT CGCGAAGTGG AGCGTATTGT TGCCGCGCGT 100
<210> 8257
<211> 100
<212> DNA
<213> Escherichia coli K-12 MG1655
<220> nuoa b2289
<222> 345-444
<223> nuoa b2289 U00096 complement(2402649..2403092)
<313> 8257: 411-430
<400> 8257
TTATCTGGTG CGTATTGGCG CGCTGGACTG GACGCCCGCG CGTTCACGCC 50
GCGAGCGTAT GAACCCGGAA ACGAACAGTA TCGCTAATCG TCAACGCTAA 100
<210> 8258
<211> 100
<212> DNA
<213> Escherichia coli K-12 MG1655
<220> nuoa b2289
<222> 245-344
<223> nuoa b2289 U00096 complement(2402649..2403092)
<313> 8258: 295-314
<400> 8258
CGCTGTATCT GTTCGCATGG TCAACCTCTA TCCGCGAAAG CGGCTGGGTA 50
GGCTTTGTGG AAGCTGCAAT TTTTATTTTT GTGTTACTGG CAGGTCTGGT 100
<210> 8259
<211> 100
<212> DNA
<213> Escherichia coli K-12 MG1655
<220> nuoa b2289
<222> 224-323
<223> nuoa b2289 U00096 complement(2402649..2403092)
<313> 8259: 263-282
<400> 8259
TCGTTATCTT CGACGTTGAA GCGCTGTATC TGTTCGCATG GTCAACCTCT 50
ATCCGCGAAA GCGGCTGGGT AGGCTTTGTG GAAGCTGCAA TTTTTATTTT 100
<210> 8260
<211> 100
<212> DNA
<213> Escherichia coli K-12 MG1655
<220> nuob b2288
<222> 564-663
<223> nuob b2288 U00096 complement(2401971..2402633)
<313> 8260: 644-663
<400> 8260
CGATCAGGGC GTTTATCGCG CCAATATGCA ATCAGAGCGC GAACGCAAGC 50
GCGGTGAACG CATTGCCGTA ACTAACCTGC GTACACCTGA CGAGATTTAA 100
<210> 8261
<211> 100
<212> DNA
<213> Escherichia coli K-12 MG1655
<220> nuob b2288
<222> 536-635
<223> nuob b2288 U00096 complement(2401971..2402633)
<313> 8261: 593-612
<400> 8261
AACGTCGTCC GCTCTCCTGG GTGGTTGGCG ATCAGGGCGT TTATCGCGCC 50
AATATGCAAT CAGAGCGCGA ACGCAAGCGC GGTGAACGCA TTGCCGTAAC 100
<210> 8262
<211> 100
<212> DNA
<213> Escherichia coli K-12 MG1655
<220> nuob b2288

<222> 503-602
<223> nuob b2288 U00096 complement(2401971..2402633)
<313> 8262: 537-556
<400> 8262
CACTGATGCT GTTGCAGGAA TCTATCGGCA AAGAACGTCG TCCGCTCTCC 50
TGGGTGGTTG GCGATCAGGG CGTTTATCGC GCCAATATGC AATCAGAGCG 100
<210> 8263
<211> 100
<212> DNA
<213> Escherichia coli K-12 MG1655
<220> nuoc b2287
<222> 1704-1803
<223> nuoc b2287 U00096 complement(2400075..2401877)
<313> 8263: 1782-1801
<400> 8263
TTTGCAGCAA ATTCCGGCGG CGATCCGCGG CAGCCTGGTG TCTGACCTGA 50
TTGTTTATCT GGGCAGTATC GATTTTGTTA TGTCAGATGT GGACCGCTAA 100
<210> 8264
<211> 100
<212> DNA
<213> Escherichia coli K-12 MG1655
<220> nuoc b2287
<222> 1642-1741
<223> nuoc b2287 U00096 complement(2400075..2401877)
<313> 8264: 1681-1700
<400> 8264
CTGACCAGCG ACGGCAGCAC CATGAGTTAC CGCACCCGTG TTCGTACCCC 50
GAGCTTTGCG CATTTGCAGC AAATTCCGGC GGCGATCCGC GGCAGCCTGG 100
<210> 8265
<211> 100
<212> DNA
<213> Escherichia coli K-12 MG1655
<220> nuoc b2287
<222> 1593-1692
<223> nuoc b2287 U00096 complement(2400075..2401877)
<313> 8265: 1626-1645
<400> 8265
TGAATCTTTC CAGATGATTG AGGCGACCAA AGGGATCAAC AGTTACTACC 50
TGACCAGCGA CGGCAGCACC ATGAGTTACC GCACCCGTGT TCGTACCCCG 100
<210> 8266
<211> 100
<212> DNA
<213> Escherichia coli K-12 MG1655
<220> kgtp b2588
<222> 1168-1267
<223> kgtp b2588 U00096 complement(2722468..2723766)
<313> 8266: 1207-1226
<400> 8266
GAGTACGTAG CGTTGTCGCT GAAATCAATA GGAATGGAAA CAGCCTTCTT 50
CTGGTATGTG ACCTTGATGG CCGTGGTGGC GTTTCTGGTT TCTTTGATGC 100
<210> 8267
<211> 100
<212> DNA
<213> Escherichia coli K-12 MG1655
<220> kgtp b2588
<222> 1097-1196
<223> kgtp b2588 U00096 complement(2722468..2723766)
<313> 8267: 1143-1162
<400> 8267
CGGCACAGGT TCGCGCATTA GGCGTTGGTC TGTCATATGC GGTCGCTAAT 50
GCTATATTTG GTGGTTCGGC GGAGTACGTA GCGTTGTCGC TGAAATCAAT 100
<210> 8268
<211> 100
<212> DNA
<213> Escherichia coli K-12 MG1655
<220> kgtp b2588
<222> 1076-1175
<223> kgtp b2588 U00096 complement(2722468..2723766)
<313> 8268: 1104-1123
<400> 8268
TACTGAAGGC TGAGATGTTC CCGGCACAGG TTCGCGCATT AGGCGTTGGT 50

CTGTCATATG CGGTCGCTAA TGCTATATTT GGTGGTTCGG CGGAGTACGT 100
<210> 8269
<211> 100
<212> DNA
<213> Escherichia coli K-12 MG1655
<220> nuoe b2286
<222> 402-501
<223> nuoe b2286 U00096 complement(2399572..2400072)
<313> 8269: 465-484
<400> 8269
GGGGAACTGT GATAAAGGGC CAAACATGAT GATCGATGAG GACACTCACG 50
CGCATCTGAC CCCGGAAGCG ATCCCTGAAC TGCTGGAGCG GTATAAATGA 100
<210> 8270
<211> 100
<212> DNA
<213> Escherichia coli K-12 MG1655
<220> nuoe b2286
<222> 378-477
<223> nuoe b2286 U00096 complement(2399572..2400072)
<313> 8270: 439-458
<400> 8270
TACGCTGCTG CCAACTTGCT GCCTGGGGAA CTGTGATAAA GGGCCAAACA 50
TGATGATCGA TGAGGACACT CACGCGCATC TGACCCCGGA AGCGATCCCT 100
<210> 8271
<211> 100
<212> DNA
<213> Escherichia coli K-12 MG1655
<220> nuoe b2286
<222> 359-458
<223> nuoe b2286 U00096 complement(2399572..2400072)
<313> 8271: 387-406
<400> 8271
CGACATTTGA TGGCCGCTTT ACGCTGCTGC CAACTTGCTG CCTGGGGAAC 50
TGTGATAAAG GGCCAAACAT GATGATCGAT GAGGACACTC ACGCGCATCT 100
<210> 8272
<211> 100
<212> DNA
<213> Escherichia coli K-12 MG1655
<220> nuof b2285
<222> 1213-1312
<223> nuof b2285 U00096 complement(2398238..2399575)
<313> 8272: 1274-1293
<400> 8272
GTGGAGCCGT TACAGAGCGC CATCAAATAT TTCCGCGAAG AATTTGAGGC 50
GGGAATCAAA CAGCCGTTCA GCAATACCCA TTTGATTAAT GGGATTCAGC 100
<210> 8273
<211> 100
<212> DNA
<213> Escherichia coli K-12 MG1655
<220> nuof b2285
<222> 1129-1228
<223> nuof b2285 U00096 complement(2398238..2399575)
<313> 8273: 1142-1161
<400> 8273
CAGCCGGGCG ATATCGAAAC ACTTGAGCAA CTGTGTCGAT TCTTAGGCCC 50
GGGTAAAACT TTCTGTGCCC ACGCACCTGG TGCAGTGGAG CCGTTACAGA 100
<210> 8274
<211> 100
<212> DNA
<213> Escherichia coli K-12 MG1655
<220> nuof b2285
<222> 1022-1121
<223> nuof b2285 U00096 complement(2398238..2399575)
<313> 8274: 1078-1097
<400> 8274
ACCTGGAAGA GTTTTTCGCC CGTGAGTCCT GCGGCTGGTG TACGCCGTGC 50
CGCGACGGTC TGCCGTGGAG CGTGAAAATT CTGCGTGCGC TGGAGCGTGG 100
<210> 8275
<211> 100
<212> DNA
<213> Escherichia coli K-12 MG1655

<220> nuog b2284
<222> 2587-2686
<223> nuog b2284 U00096 complement(2395459..2398191)
<313> 8275: 2634-2653
<400> 8275
AGTTACGATG GCAACACGGT CACGCTGCCG GTTGAAATCG CCGAAGGACT 50
GACGGCAGGG CAGGTGGGCT TGCCGATGGG TATGTCCGGC ATTGCTCCGG 100
<210> 8276
<211> 100
<212> DNA
<213> Escherichia coli K-12 MG1655
<220> nuog b2284
<222> 2530-2629
<223> nuog b2284 U00096 complement(2395459..2398191)
<313> 8276: 2595-2614
<400> 8276
CTCAACCCAG CGGATGCCGC GAAGTTGGGT GTGAACGCAG GTACACGCGT 50
CTCCTTTAGT TACGATGGCA ACACGGTCAC GCTGCCGGTT GAAATCGCCG 100
<210> 8277
<211> 100
<212> DNA
<213> Escherichia coli K-12 MG1655
<220> nuog b2284
<222> 2482-2581
<223> nuog b2284 U00096 complement(2395459..2398191)
<313> 8277: 2526-2545
<400> 8277
CAGCGTGCTC CGGTCTTCCA GAGCCGTATG CCGCAGCCGT ACATCAAACT 50
CAACCCAGCG GATGCCGCGA AGTTGGGTGT GAACGCAGGT ACACGCGTCT 100
<210> 8278
<211> 100
<212> DNA
<213> Escherichia coli K-12 MG1655
<220> nuoh b2283
<222> 875-974
<223> nuoh b2283 U00096 complement(2394485..2395462)
<313> 8278: 919-938
<400> 8278
CGCGTTATGA CCAGGTAATG TCCTTCGGCT GGAAAATCTG CCTGCCGCTG 50
ACGCTGATCA ACTTGCTGGT AACGGCGGCT GTCATTCTCT GGCAGGCGCA 100
<210> 8279
<211> 100
<212> DNA
<213> Escherichia coli K-12 MG1655
<220> nuoh b2283
<222> 808-907
<223> nuoh b2283 U00096 complement(2394485..2395462)
<313> 8279: 861-880
<400> 8279
TGGTTCGCGC TGAAAACCGC GTTCTTTATG ATGATGTTCA TTTTGATTCG 50
TGCGTCGTTA CCGCGTCCGC GTTATGACCA GGTAATGTCC TTCGGCTGGA 100
<210> 8280
<211> 100
<212> DNA
<213> Escherichia coli K-12 MG1655
<220> nuoh b2283
<222> 783-882
<223> nuoh b2283 U00096 complement(2394485..2395462)
<313> 8280: 809-828
<400> 8280
AGGCCCGTTG TTACCGCCAT TCATCTGGTT CGCGCTGAAA ACCGCGTTCT 50
TTATGATGAT GTTCATTTTG ATTCGTGCGT CGTTACCGCG TCCGCGTTAT 100
<210> 8281
<211> 100
<212> DNA
<213> Escherichia coli K-12 MG1655
<220> nuoi b2282
<222> 440-539
<223> nuoi b2282 U00096 complement(2393928..2394470)
<313> 8281: 489-508
<400> 8281

AATATAACTT CTACCGGATG GCAGGTATGG CAATCGACGG CAAAGATAAG 50
GGCGAAGCAG AGAACGAAGC CAAGCCTATC GACGTCAAGA GCCTGTTACC 100
<210> 8282
<211> 100
<212> DNA
<213> Escherichia coli K-12 MG1655
<220> nuoi b2282
<222> 434-533
<223> nuoi b2282 U00096 complement(2393928..2394470)
<313> 8282: 445-464
<400> 8282
ACCCGGAATA TAACTTCTAC CGGATGGCAG GTATGGCAAT CGACGGCAAA 50
GATAAGGGCG AAGCAGAGAA CGAAGCCAAG CCTATCGACG TCAAGAGCCT 100
<210> 8283
<211> 100
<212> DNA
<213> Escherichia coli K-12 MG1655
<220> nuoi b2282
<222> 363-462
<223> nuoi b2282 U00096 complement(2393928..2394470)
<313> 8283: 404-423
<400> 8283
AATGGGGGAA TACAAGCGCC AGGATCTGGT TTACGAGAAA GAGGATCTGC 50
TGATCTCCGG TCCGGGCAAA TACCCGGAAT ATAACTTCTA CCGGATGGCA 100
<210> 8284
<211> 100
<212> DNA
<213> Escherichia coli K-12 MG1655
<220> nuoj b2281
<222> 456-555
<223> nuoj b2281 U00096 complement(2393362..2393916)
<313> 8284: 527-546
<400> 8284
GGTTGTGGCC TTCCACGTCG GTCGTGAAGA GCGTGCGGGT GAAGTGCTGA 50
GCAATCGTAA AGACGACAGC GCGAAAAGAA AAACGGAGGA GCACGCATGA 100
<210> 8285
<211> 100
<212> DNA
<213> Escherichia coli K-12 MG1655
<220> nuoj b2281
<222> 433-532
<223> nuoj b2281 U00096 complement(2393362..2393916)
<313> 8285: 487-506
<400> 8285
TCTATGCTGC TGCTCGCAGG TCTGGTTGTG GCCTTCCACG TCGGTCGTGA 50
AGAGCGTGCG GGTGAAGTGC TGAGCAATCG TAAAGACGAC AGCGCGAAAA 100
<210> 8286
<211> 100
<212> DNA
<213> Escherichia coli K-12 MG1655
<220> nuoj b2281
<222> 378-477
<223> nuoj b2281 U00096 complement(2393362..2393916)
<313> 8286: 420-439
<400> 8286
TAAAGCAGTG GGTATTACGC TGTTCGGGCC TTACGTACTG GCGGTGGAAC 50
TGGCTTCTAT GCTGCTGCTC GCAGGTCTGG TTGTGGCCTT CCACGTCGGT 100
<210> 8287
<211> 100
<212> DNA
<213> Escherichia coli K-12 MG1655
<220> nuok b2280
<222> 204-303
<223> nuok b2280 U00096 complement(2393063..2393365)
<313> 8287: 277-296
<400> 8287
CGCGGCGGCA GAAGCGAGTA TCGGCCTTGC GCTGCTGCTG CAACTTCACC 50
GTCGTCGCCA GAACCTGAAC ATCGATTCAG TAAGTGAGAT GCGCGGATGA 100
<210> 8288
<211> 100
<212> DNA

<213> Escherichia coli K-12 MG1655
<220> nuok b2280
<222> 190-289
<223> nuok b2280 U00096 complement(2393063..2393365)
<313> 8288: 244-263
<400> 8288
CTCGCCATCA GCCTCGCGGC GGCAGAAGCG AGTATCGGCC TTGCGCTGCT  50
GCTGCAACTT CACCGTCGTC GCCAGAACCT GAACATCGAT TCAGTAAGTG  100
<210> 8289
<211> 100
<212> DNA
<213> Escherichia coli K-12 MG1655
<220> nuok b2280
<222> 141-240
<223> nuok b2280 U00096 complement(2393063..2393365)
<313> 8289: 188-207
<400> 8289
GGTCGCCGGA AGCTACTGGG GCCAGACCGA CGGTCAGGTG ATGTACATTC  50
TCGCCATCAG CCTCGCGGCG GCAGAAGCGA GTATCGGCCT TGCGCTGCTG  100
<210> 8290
<211> 100
<212> DNA
<213> Escherichia coli K-12 MG1655
<220> nuol b2279
<222> 1716-1815
<223> nuol b2279 U00096 complement(2391225..2393066)
<313> 8290: 1780-1799
<400> 8290
GGCTGTCCTT TCCCGCTTTG CAGGTAAAGG TCTGCTGTTA AGTGAGAACG  50
GCTATCTGCG CTGGTATGTG GCATCCATGA GCATCGGTGC GGTCGTGGTG  100
<210> 8291
<211> 100
<212> DNA
<213> Escherichia coli K-12 MG1655
<220> nuol b2279
<222> 1697-1796
<223> nuol b2279 U00096 complement(2391225..2393066)
<313> 8291: 1729-1748
<400> 8291
ACTCAATGAT GAACATCCCG GCTGTCCTTT CCCGCTTTGC AGGTAAAGGT  50
CTGCTGTTAA GTGAGAACGG CTATCTGCGC TGGTATGTGG CATCCATGAG  100
<210> 8292
<211> 100
<212> DNA
<213> Escherichia coli K-12 MG1655
<220> nuol b2279
<222> 1663-1762
<223> nuol b2279 U00096 complement(2391225..2393066)
<313> 8292: 1690-1709
<400> 8292
GGTATTGCCT GGTTGCTGAA ACGCGATCCG CTGAACTCAA TGATGAACAT  50
CCCGGCTGTC CTTTCCCGCT TTGCAGGTAA AGGTCTGCTG TTAAGTGAGA  100
<210> 8293
<211> 100
<212> DNA
<213> Escherichia coli K-12 MG1655
<220> afuc afuC
<222> 924-1023
<223> afuc afuC U00096 complement(276980..278038)
<313> 8293: 951-970
<400> 8293
TGAAGTGACG GTGGAATGGC ACGGGCAGGA GATATTATTG CAGGTCAACG  50
CTACGCGTCT GCAACCGGAC GTCGGCGAGC AGTATTATCT TGAAATCCAT  100
<210> 8294
<211> 100
<212> DNA
<213> Escherichia coli K-12 MG1655
<220> afuc afuC
<222> 739-838
<223> afuc afuC U00096 complement(276980..278038)
<313> 8294: 787-806

<400> 8294
TTCCCGGCAA CCTTCAGCGA CGGATACGTT GATATCTACG GCTATCATCT  50
GCCGCGCCCG CTGCACTTTG GTACACAGGG TGAAGGGATG GTCGGTGTGC  100
<210> 8295
<211> 100
<212> DNA
<213> Escherichia coli K-12 MG1655
<220> afuc afuC
<222> 694-793
<223> afuc afuC U00096 complement(276980..278038)
<313> 8295: 747-766
<400> 8295
CCCGCCTCCC GCTTTATGGC GAGCTTTATG GGCGATGCCA ACCTGTTCCC  50
GGCAACCTTC AGCGACGGAT ACGTTGATAT CTACGGCTAT CATCTGCCGC  100
<210> 8296
<211> 100
<212> DNA
<213> Escherichia coli K-12 MG1655
<220> nuom b2278
<222> 1431-1530
<223> nuom b2278 U00096 complement(2389532..2391061)
<313> 8296: 1467-1486
<400> 8296
GCTGGGCTTC TATCCGCAGC CGATTCTGGA TACCTCGCAC TCCGCGATTG  50
GCAATATCCA GCAGTGGTTT GTTAATTCCG TTACTACTAC AAGGCCGTAA  100
<210> 8297
<211> 100
<212> DNA
<213> Escherichia coli K-12 MG1655
<220> nuom b2278
<222> 1382-1481
<223> nuom b2278 U00096 complement(2389532..2391061)
<313> 8297: 1436-1455
<400> 8297
CGCTGCGTGA GCTGTTTATG ATCCTGTTGC TGGTGGTGCT GCTGGTACTG  50
CTGGGCTTCT ATCCGCAGCC GATTCTGGAT ACCTCGCACT CCGCGATTGG  100
<210> 8298
<211> 100
<212> DNA
<213> Escherichia coli K-12 MG1655
<220> nuom b2278
<222> 1360-1459
<223> nuom b2278 U00096 complement(2389532..2391061)
<313> 8298: 1376-1395
<400> 8298
AGCCAGGAAC TGCCAGGGAT GTCGCTGCGT GAGCTGTTTA TGATCCTGTT  50
GCTGGTGGTG CTGCTGGTAC TGCTGGGCTT CTATCCGCAG CCGATTCTGG  100
<210> 8299
<211> 100
<212> DNA
<213> Escherichia coli K-12 MG1655
<220> nuon b2277
<222> 1101-1200
<223> nuon b2277 U00096 complement(2388068..2389345)
<313> 8299: 1160-1179
<400> 8299
CGTGGCGGTG AGCCTGTATC TTCACGCCCC GGAACAACCG GGTCGCGATG  50
CACCATCAAA CTGGCAGTAC AGCGCGGGCG GTATCGTTGT GCTGATCTCT  100
<210> 8300
<211> 100
<212> DNA
<213> Escherichia coli K-12 MG1655
<220> nuon b2277
<222> 971-1070
<223> nuon b2277 U00096 complement(2388068..2389345)
<313> 8300: 1019-1038
<400> 8300
CCGGTATCCC GATGACGCTG GGCTTTATCG GTAAGTTCTA CGTGCTGGCG  50
GTCGGTGTCC AGGCACACTT GTGGTGGCTG GTGGGTGCCG TGGTTGTCGG  100
<210> 8301
<211> 100

<212> DNA
<213> Escherichia coli K-12 MG1655
<220> nuon b2277
<222> 900-999
<223> nuon b2277 U00096 complement(2388068..2389345)
<313> 8301: 936-955
<400> 8301
CTCTTACCGC GGTCTGTTCT GGCATCGTCC GATCCTCGCG GCAGTGATGA 50
CGGTGATGAT GCTGTCTCTG GCCGGTATCC CGATGACGCT GGGCTTTATC 100
<210> 8302
<211> 100
<212> DNA
<213> Escherichia coli K-12 MG1655
<220> nfnb b0579
<222> 555-654
<223> nfnb b0579 U00096 complement(603994..604647)
<313> 8302: 620-639
<400> 8302
TCTGGTGGTT GTTCCGGTAG GTCATCACAG CGTTGAAGAT TTTAACGCTA 50
CGCTGCCGAA ATCTCGTCTG CCGCAAAACA TCACCTTAAC CGAAGTGTAA 100
<210> 8303
<211> 100
<212> DNA
<213> Escherichia coli K-12 MG1655
<220> nfnb b0579
<222> 480-579
<223> nfnb b0579 U00096 complement(603994..604647)
<313> 8303: 523-542
<400> 8303
CGCGGTACCC ATCGAAGGTT TTGACGCCGC CATCCTCGAT GCAGAATTTG 50
GTCTGAAAGA GAAAGGCTAC ACCAGTCTGG TGGTTGTTCC GGTAGGTCAT 100
<210> 8304
<211> 100
<212> DNA
<213> Escherichia coli K-12 MG1655
<220> nfnb b0579
<222> 379-478
<223> nfnb b0579 U00096 complement(603994..604647)
<313> 8304: 433-452
<400> 8304
ATGCACCGTA AAGATCTGCA TGATGATGCA GAGTGGATGG CAAAACAGGT 50
TTATCTCAAC GTCGGTAACT TCCTGCTCGG CGTGGCGGCT CTGGGTCTGG 100
<210> 8305
<211> 100
<212> DNA
<213> Escherichia coli K-12 MG1655
<220> ylab b0458
<222> 1458-1557
<223> ylab b0458 U00096 complement(476291..477847)
<313> 8305: 1531-1550
<400> 8305
GTGGTTACGC CAGCATGGCG TGCACTACGG TCAGGGCTGG CTCTACAGCA 50
AGGCATTACC GAAAGAAGAT TTCTTACGCT GGGCCGAGCA ACATTTGTGA 100
<210> 8306
<211> 100
<212> DNA
<213> Escherichia coli K-12 MG1655
<220> ylab b0458
<222> 1421-1520
<223> ylab b0458 U00096 complement(476291..477847)
<313> 8306: 1447-1466
<400> 8306
TGGTAGCGGA GGGAATCGAA ACCAGTAAAC AAGAAGAGTG GTTACGCCAG 50
CATGGCGTGC ACTACGGTCA GGGCTGGCTC TACAGCAAGG CATTACCGAA 100
<210> 8307
<211> 100
<212> DNA
<213> Escherichia coli K-12 MG1655
<220> ylab b0458
<222> 1366-1465
<223> ylab b0458 U00096 complement(476291..477847)

<313> 8307: 1397-1416
<400> 8307
TATAAAAATG TCACGCCGCA TATCATCGAA ATGGCAAAAA CACTGAAACT 50
GAAAATGGTA GCGGAGGGAA TCGAAACCAG TAAACAAGAA GAGTGGTTAC 100
<210> 8308
<211> 100
<212> DNA
<213> Escherichia coli K-12 MG1655
<220> ylac b0459
<222> 411-510
<223> ylac b0459 U00096 complement(478005..478514)
<313> 8308: 484-503
<400> 8308
GGATGTTCAT AAGGAACAAC TGCAAAAAAT GATCGTCCGT AAAGGTGAAC 50
TGTCTTTTTA CGATATTTTT ACCCTCGCTC GCGCCGAATC AACATCTTAA 100
<210> 8309
<211> 100
<212> DNA
<213> Escherichia coli K-12 MG1655
<220> ylac b0459
<222> 386-485
<223> ylac b0459 U00096 complement(478005..478514)
<313> 8309: 408-427
<400> 8309
CCATCTTGAA CTCTCCGCGT GTCGCGGATG TTCATAAGGA ACAACTGCAA 50
AAAATGATCG TCCGTAAAGG TGAACTGTCT TTTTACGATA TTTTTACCCT 100
<210> 8310
<211> 100
<212> DNA
<213> Escherichia coli K-12 MG1655
<220> ylac b0459
<222> 350-449
<223> ylac b0459 U00096 complement(478005..478514)
<313> 8310: 372-391
<400> 8310
ACAACACGCG CTTTGTACCT GCCGCGCTGG TTGAAGCCAT CTTGAACTCT 50
CCGCGTGTCG CGGATGTTCA TAAGGAACAA CTGCAAAAAA TGATCGTCCG 100
<210> 8311
<211> 100
<212> DNA
<213> Escherichia coli K-12 MG1655
<220> ylad b0460
<222> 453-552
<223> ylad b0460 U00096 complement(478591..479142)
<313> 8311: 517-536
<400> 8311
TGATAACGTC GTGGTAGCCT CAGGTGCAGT TGTCACAAAA GATGTCCCGG 50
ACAACGTTGT CGTGGGCGGT AATCCAGCCA GAATAATTAA AAAATTGTAA 100
<210> 8312
<211> 100
<212> DNA
<213> Escherichia coli K-12 MG1655
<220> ylad b0460
<222> 445-544
<223> ylad b0460 U00096 complement(478591..479142)
<313> 8312: 476-495
<400> 8312
ACCATTGGTG ATAACGTCGT GGTAGCCTCA GGTGCAGTTG TCACAAAAGA 50
TGTCCCGGAC AACGTTGTCG TGGGCGGTAA TCCAGCCAGA ATAATTAAAA 100
<210> 8313
<211> 100
<212> DNA
<213> Escherichia coli K-12 MG1655
<220> ylad b0460
<222> 354-453
<223> ylad b0460 U00096 complement(478591..479142)
<313> 8313: 386-405
<400> 8313
AGCACGTAAT AGCGGTGCTG AACTGGGGAA ACCCGTCACC ATCGGTAATA 50
ACGTCTGGAT TGGCGGACGC GCGGTCATTA ACCCTGGTGT GACCATTGGT 100
<210> 8314

<211> 100
<212> DNA
<213> Escherichia coli K-12 MG1655
<220> cbl b1988
<222> 852-951
<223> cbl b1988 U00096 complement(2057986..2058936)
<313> 8314: 921-940
<400> 8314
CTGGCGCTTT CTGGAACTTT GTAATGCAGG ACTGTCAGTT GAGGATATCA 50
AGCGGCAGGT GATGGAAAGC AGTGAAGAGG AAATTGATTA TCAGATATAG 100
<210> 8315
<211> 100
<212> DNA
<213> Escherichia coli K-12 MG1655
<220> cbl b1988
<222> 813-912
<223> cbl b1988 U00096 complement(2057986..2058936)
<313> 8315: 827-846
<400> 8315
GTTGGGACTG AAGCGAGGAC AACTTCAGCG TAACTATGTC TGGCGCTTTC 50
TGGAACTTTG TAATGCAGGA CTGTCAGTTG AGGATATCAA GCGGCAGGTG 100
<210> 8316
<211> 100
<212> DNA
<213> Escherichia coli K-12 MG1655
<220> cbl b1988
<222> 770-869
<223> cbl b1988 U00096 complement(2057986..2058936)
<313> 8316: 799-818
<400> 8316
TCCGCCTGGA TACGCGGCAT CTTTTTGATG CTAATACTGT CTGGTTGGGA 50
CTGAAGCGAG GACAACTTCA GCGTAACTAT GTCTGGCGCT TTCTGGAACT 100
<210> 8317
<211> 100
<212> DNA
<213> Escherichia coli K-12 MG1655
<220> gdha gdhA
<222> 1245-1344
<223> gdha gdhA U00096 1840395..1841738
<313> 8317: 1300-1319
<400> 8317
GCATGGTGGT GAAGGTGAGC AAACCAACTA CGTGCAGGGC GCGAACATTG 50
CCGGTTTTGT GAAGGTTGCC GATGCGATGC TGGCGCAGGG TGTGATTTAA 100
<210> 8318
<211> 100
<212> DNA
<213> Escherichia coli K-12 MG1655
<220> gdha gdhA
<222> 1210-1309
<223> gdha gdhA U00096 1840395..1841738
<313> 8318: 1266-1285
<400> 8318
CACATCATGC TGGATATCCA CCATGCCTGT GTTGAGCATG GTGGTGAAGG 50
TGAGCAAACC AACTACGTGC AGGGCGCGAA CATTGCCGGT TTTGTGAAGG 100
<210> 8319
<211> 100
<212> DNA
<213> Escherichia coli K-12 MG1655
<220> gdha gdhA
<222> 1147-1246
<223> gdha gdhA U00096 1840395..1841738
<313> 8319: 1197-1216
<400> 8319
GAAATGGCAC AAAACGCTGC GCGCCTGGGC TGGAAAGCCG AGAAAGTTGA 50
CGCACGTTTG CATCACATCA TGCTGGATAT CCACCATGCC TGTGTTGAGC 100
<210> 8320
<211> 100
<212> DNA
<213> Escherichia coli K-12 MG1655
<220> nupc b2394
<222> 1104-1203

<223> nupc b2394 U00096 2511062..2512264
<313> 8320: 1155-1174
<400> 8320
AGAGCAAGGT AACGTGGTTT CTCGCTTCGG TCTGAAGCTG GTTTACGGCT 50
CTACCCTGGT GAGTGTGCTG TCTGCGTCAA TCGCAGCACT GGTGCTGTAA 100
<210> 8321
<211> 100
<212> DNA
<213> Escherichia coli K-12 MG1655
<220> nupc b2394
<222> 1077-1176
<223> nupc b2394 U00096 2511062..2512264
<313> 8321: 1102-1121
<400> 8321
CGCAGGTGCA GTTAAAGGCC TGAATGAAGA GCAAGGTAAC GTGGTTTCTC 50
GCTTCGGTCT GAAGCTGGTT TACGGCTCTA CCCTGGTGAG TGTGCTGTCT 100
<210> 8322
<211> 100
<212> DNA
<213> Escherichia coli K-12 MG1655
<220> nupc b2394
<222> 1039-1138
<223> nupc b2394 U00096 2511062..2512264
<313> 8322: 1063-1082
<400> 8322
CTGGTTTCCT TCGCTAACTT CTCTTCAATC GGGATTATCG CAGGTGCAGT 50
TAAAGGCCTG AATGAAGAGC AAGGTAACGT GGTTTCTCGC TTCGGTCTGA 100
<210> 8323
<211> 100
<212> DNA
<213> Escherichia coli K-12 MG1655
<220> deoa b4383
<222> 1224-1323
<223> deoa b4383 U00096 4615798..4617120
<313> 8323: 1295-1314
<400> 8323
CAACTGGCAG GAAGCGGCGA AAGCGGTGAA AGCGGCAATT AAACTTGCCG 50
ATAAAGCACC GGAAAGCACA CCAACTGTCT ATCGCCGTAT CAGCGAATAA 100
<210> 8324
<211> 100
<212> DNA
<213> Escherichia coli K-12 MG1655
<220> deoa b4383
<222> 1209-1308
<223> deoa b4383 U00096 4615798..4617120
<313> 8324: 1231-1250
<400> 8324
CGCGAAAGAC GAAAACAACT GGCAGGAAGC GGCGAAAGCG GTGAAAGCGG 50
CAATTAAACT TGCCGATAAA GCACCGGAAA GCACACCAAC TGTCTATCGC 100
<210> 8325
<211> 100
<212> DNA
<213> Escherichia coli K-12 MG1655
<220> deoa b4383
<222> 1095-1194
<223> deoa b4383 U00096 4615798..4617120
<313> 8325: 1124-1143
<400> 8325
AATGGGCGGC GGACGCCGTC AGGCATCTGA CACCATCGAT TACAGCGTCG 50
GCTTTACTGA TATGGCGCGT CTGGGCGACC AGGTAGACGG TCAGCGTCCG 100
<210> 8326
<211> 100
<212> DNA
<213> Escherichia coli K-12 MG1655
<220> deob b4384
<222> 1095-1194
<223> deob b4384 U00096 4617172..4618395
<313> 8326: 1128-1147
<400> 8326
GGTACTGGTA TATGGCCCGA AAGTAAAACC GGGCTCACTG GGTCATCGTG 50
AAACCTTCGC GGATATCGGC CAGACTCTGG CAAAATATTT TGGTACTTCT 100

<210> 8327
<211> 100
<212> DNA
<213> Escherichia coli K-12 MG1655
<220> deob b4384
<222> 977-1076
<223> deob b4384 U00096 4617172..4618395
<313> 8327: 1026-1045
<400> 8327
ACCGCCGTCT GCCGGAGCTG ATGTCTCTGC TGCGCGATGA CGACATCCTG 50
ATCCTCACCG CTGACCACGG TTGCGATCCG ACCTGGACCG GTACTGACCA 100
<210> 8328
<211> 100
<212> DNA
<213> Escherichia coli K-12 MG1655
<220> deob b4384
<222> 952-1051
<223> deob b4384 U00096 4617172..4618395
<313> 8328: 979-998
<400> 8328
TATGCCGCGG GTCTGGAACT GTTCGACCGC CGTCTGCCGG AGCTGATGTC 50
TCTGCTGCGC GATGACGACA TCCTGATCCT CACCGCTGAC CACGGTTGCG 100
<210> 8329
<211> 100
<212> DNA
<213> Escherichia coli K-12 MG1655
<220> nupg b2965
<222> 1206-1305
<223> nupg b2965 U00096 3103684..3104988
<313> 8329: 1277-1296
<400> 8329
TTTCGCTGGT TACTCCGTGG TTCTGGCCTT CGCGTTCATG GCGATGTTCA 50
AATATAAACA CGTTCGTGTC CCGACAGGCA CACAGACGGT TAGCCACTAA 100
<210> 8330
<211> 100
<212> DNA
<213> Escherichia coli K-12 MG1655
<220> nupg b2965
<222> 1191-1290
<223> nupg b2965 U00096 3103684..3104988
<313> 8330: 1229-1248
<400> 8330
GACCGTATGG TTGATTTTCG CTGGTTACTC CGTGGTTCTG GCCTTCGCGT 50
TCATGGCGAT GTTCAAATAT AAACACGTTC GTGTCCCGAC AGGCACACAG 100
<210> 8331
<211> 100
<212> DNA
<213> Escherichia coli K-12 MG1655
<220> nupg b2965
<222> 1144-1243
<223> nupg b2965 U00096 3103684..3104988
<313> 8331: 1174-1193
<400> 8331
GGTAAAGTTG TTGAGATGTA CACCCAAAAC GGCATTACCG ACTGGCAGAC 50
CGTATGGTTG ATTTTCGCTG GTTACTCCGT GGTTCTGGCC TTCGCGTTCA 100
<210> 8332
<211> 100
<212> DNA
<213> Escherichia coli K-12 MG1655
<220> deoc b4382
<222> 681-780
<223> deoc b4382 U00096 4614892..4615671
<313> 8332: 747-766
<400> 8332
GGCAGATGCG CGTCACTACC GCTTTGGCGC TTCCAGCCTG CTGGCAAGCC 50
TGCTGAAAGC GCTGGGTCAC GGCGACGGTA AGAGCGCCAG CAGCTACTAA 100
<210> 8333
<211> 100
<212> DNA
<213> Escherichia coli K-12 MG1655
<220> deoc b4382

<222> 596-695
<223> deoc b4382 U00096 4614892..4615671
<313> 8333: 638-657
<400> 8333
GTTTCAAACC GGCGGGCGGC GTGCGTACTG CGGAAGATGC GCAGAAATAT 50
CTCGCCATTG CAGATGAACT GTTCGGTGCT GACTGGGCAG ATGCGCGTCA 100
<210> 8334
<211> 100
<212> DNA
<213> Escherichia coli K-12 MG1655
<220> deoc b4382
<222> 519-618
<223> deoc b4382 U00096 4614892..4615671
<313> 8334: 580-599
<400> 8334
GGCTGTGAAC GCGACGCCGG AAAGCGCGCG CATCATGATG GAAGTGATCC 50
GTGATATGGG CGTAGAAAAA ACCGTTGGTT TCAAACCGGC GGGCGGCGTG 100
<210> 8335
<211> 100
<212> DNA
<213> Escherichia coli K-12 MG1655
<220> deod b4385
<222> 621-720
<223> deod b4385 U00096 4618452..4619171
<313> 8335: 692-711
<400> 8335
CCGCACTCAC GAGCAGACCA CTGCCGCTGA GCGTCAGACT ACCTTCAACG 50
ACATGATCAA AATCGCACTG GAATCCGTTC TGCTGGGCGA TAAAGAGTAA 100
<210> 8336
<211> 100
<212> DNA
<213> Escherichia coli K-12 MG1655
<220> deod b4385
<222> 621-720
<223> deod b4385 U00096 4618452..4619171
<313> 8336: 647-666
<400> 8336
CCGCACTCAC GAGCAGACCA CTGCCGCTGA GCGTCAGACT ACCTTCAACG 50
ACATGATCAA AATCGCACTG GAATCCGTTC TGCTGGGCGA TAAAGAGTAA 100
<210> 8337
<211> 100
<212> DNA
<213> Escherichia coli K-12 MG1655
<220> deod b4385
<222> 575-674
<223> deod b4385 U00096 4618452..4619171
<313> 8337: 617-636
<400> 8337
AATTTGGCGC GAAAGCCCTG ACCATCTGCA CCGTATCTGA CCACATCCGC 50
ACTCACGAGC AGACCACTGC CGCTGAGCGT CAGACTACCT TCAACGACAT 100
<210> 8338
<211> 100
<212> DNA
<213> Escherichia coli K-12 MG1655
<220> phea b2600
<222> 1062-1161
<223> phea b2600 U00096 2735765..2736925
<313> 8338: 1131-1150
<400> 8338
AATGCAAAAA GCATTGAAAG AGTTAGGGGA AATCACCCGT TCAATGAAGG 50
TATTGGGCTG TTACCCAAGT GAGAACGTAG TGCCTGTTGA TCCAACCTGA 100
<210> 8339
<211> 100
<212> DNA
<213> Escherichia coli K-12 MG1655
<220> phea b2600
<222> 995-1094
<223> phea b2600 U00096 2735765..2736925
<313> 8339: 1052-1071
<400> 8339
CGATTCACGG TAATCCATGG GAAGAGATGT TCTATCTGGA TATTCAGGCC 50

AATCTTGAAT CAGCGGAAAT GCAAAAAGCA TTGAAAGAGT TAGGGGAAAT 100
<210> 8340
<211> 100
<212> DNA
<213> Escherichia coli K-12 MG1655
<220> phea b2600
<222> 931-1030
<223> phea b2600 U00096 2735765..2736925
<313> 8340: 985-1004
<400> 8340
CTGGTTGAAG CGTTGCTGGT ACTGCGCAAC CACAATCTGA TTATGACCCG 50
TCTGGAATCA CGCCCGATTC ACGGTAATCC ATGGGAAGAG ATGTTCTATC 100
<210> 8341
<211> 100
<212> DNA
<213> Escherichia coli K-12 MG1655
<220> cca b3057
<222> 1119-1218
<223> cca b3057 U00096 3199532..3200770
<313> 8341: 1186-1205
<400> 8341
GCCGACAAAA GCCGTCGTTG AAGCGGGATT TAAAGGTGTG GAGATTCGCG 50
AGGAGCTGAC CCGACGGCGG ATTGCGGCGG TAGCCAGCTG GAAGGAACAA 100
<210> 8342
<211> 100
<212> DNA
<213> Escherichia coli K-12 MG1655
<220> cca b3057
<222> 1098-1197
<223> cca b3057 U00096 3199532..3200770
<313> 8342: 1133-1152
<400> 8342
CTGGGAAGTG GCGCAGTCAG TGCCGACAAA AGCCGTCGTT GAAGCGGGAT 50
TTAAAGGTGT GGAGATTCGC GAGGAGCTGA CCCGACGGCG GATTGCGGCG 100
<210> 8343
<211> 100
<212> DNA
<213> Escherichia coli K-12 MG1655
<220> cca b3057
<222> 898-997
<223> cca b3057 U00096 3199532..3200770
<313> 8343: 927-946
<400> 8343
CTGGTGGCTG AGTTTCACGA TCTCATCCAC ACCTTCCCAA TGCTGAACCC 50
AAAAACCATC GTCAAATTAT TTGATTCCAT CGATGCCTGG CGTAAACCGC 100
<210> 8344
<211> 100
<212> DNA
<213> Escherichia coli K-12 MG1655
<220> ylba b0516
<222> 687-786
<223> ylba b0516 U00096 complement(542485..543270)
<313> 8344: 730-749
<400> 8344
TATGGGCGCT TATTCTTTAC AGGCTGGTTA TGGTGTAGGG CGTGGTGAAG 50
CGTTCAGCTA TATTTACTCG AAAGATTGTA ACCGCGACGT AGAGATTTAA 100
<210> 8345
<211> 100
<212> DNA
<213> Escherichia coli K-12 MG1655
<220> ylba b0516
<222> 592-691
<223> ylba b0516 U00096 complement(542485..543270)
<313> 8345: 635-654
<400> 8345
CACGTTCAGG AACACGGTGC CTATATTCTT TCCGGTCAGG GGGTTTATAA 50
CCTCGACAAT AACTGGATCC CGGTGAAAAA AGGCGATTAC ATCTTTATGG 100
<210> 8346
<211> 100
<212> DNA
<213> Escherichia coli K-12 MG1655

<220> ylba b0516
<222> 565-664
<223> ylba b0516 U00096 complement(542485..543270)
<313> 8346: 580-599
<400> 8346
GGTGCCAGCC ACGGTTATAT CGAAACACAC GTTCAGGAAC ACGGTGCCTA  50
TATTCTTTCC GGTCAGGGGG TTTATAACCT CGACAATAAC TGGATCCCGG  100
<210> 8347
<211> 100
<212> DNA
<213> Escherichia coli K-12 MG1655
<220> ylbb b0517
<222> 1137-1236
<223> ylbb b0517 U00096 complement(543281..544516)
<313> 8347: 1206-1225
<400> 8347
GATCAGCCAT AACCCGGCGG AACGCACCAA TATTACCGAC CTTGCCGAAG  50
GGGTCAAAAC GTTGGCACTC ATGCTTTATC AACTTGCCTG GCAGAAATAA  100
<210> 8348
<211> 100
<212> DNA
<213> Escherichia coli K-12 MG1655
<220> ylbb b0517
<222> 1123-1222
<223> ylbb b0517 U00096 complement(543281..544516)
<313> 8348: 1174-1193
<400> 8348
CCCAGCATCA ACGGGATCAG CCATAACCCG GCGGAACGCA CCAATATTAC  50
CGACCTTGCC GAAGGGGTCA AAACGTTGGC ACTCATGCTT TATCAACTTG  100
<210> 8349
<211> 100
<212> DNA
<213> Escherichia coli K-12 MG1655
<220> ylbb b0517
<222> 1094-1193
<223> ylbb b0517 U00096 complement(543281..544516)
<313> 8349: 1124-1143
<400> 8349
CTCGCGTACC AACCTGCATG ATTTTTATCC CCAGCATCAA CGGGATCAGC  50
CATAACCCGG CGGAACGCAC CAATATTACC GACCTTGCCG AAGGGGTCAA  100
<210> 8350
<211> 100
<212> DNA
<213> Escherichia coli K-12 MG1655
<220> phel b2599
<222> 1-48
<223> phel b2599 U00096 2735619..2735666
<313> 8350: 23-42
<400> 8350
ATGAAACACA TACCGTTTTT CTTCGCATTC TTTTTTACCT TCCCCTGA  50
 100
<210> 8351
<211> 100
<212> DNA
<213> Escherichia coli K-12 MG1655
<220> ylbc b0518
<222> 951-1050
<223> ylbc b0518 U00096 complement(544538..545587)
<313> 8351: 1017-1036
<400> 8351
CGTCGAAGGC ATCGAAATTG TTGATGATAT TTACCAGTAT TTAATTTCCG  50
ACGCGCTTTA TAACACGTCA TACGAAACGA AAAATCCCTT TGCGCAATAA  100
<210> 8352
<211> 100
<212> DNA
<213> Escherichia coli K-12 MG1655
<220> ylbc b0518
<222> 901-1000
<223> ylbc b0518 U00096 complement(544538..545587)
<313> 8352: 946-965
<400> 8352

CAGGTTTATT ATCCCGGACA GGATCAGGAT ATTAAACAAC GCAAAGCCGC 50
CGTCGAAGGC ATCGAAATTG TTGATGATAT TTACCAGTAT TTAATTTCCG 100
<210> 8353
<211> 100
<212> DNA
<213> Escherichia coli K-12 MG1655
<220> ylbc b0518
<222> 868-967
<223> ylbc b0518 U00096 complement(544538..545587)
<313> 8353: 896-915
<400> 8353
TTAAATGCCA TTACCCCCGC GCCCGGTTTT AATCAGGTTT ATTATCCCGG 50
ACAGGATCAG GATATTAAAC AACGCAAAGC CGCCGTCGAA GGCATCGAAA 100
<210> 8354
<211> 100
<212> DNA
<213> Escherichia coli K-12 MG1655
<220> deor b0841
<222> 660-759
<223> deor b0841 U00096 complement(881199..881957)
<313> 8354: 719-738
<400> 8354
GGGTGACCTG AAACGCTTTG ATATTGTGGT GAGCGATTGT TGCCCGGAAG 50
ATGAGTATGT GAAGTACGCG CAGACGCAGC GCATTAAGTT GATGTATTAA 100
<210> 8355
<211> 100
<212> DNA
<213> Escherichia coli K-12 MG1655
<220> deor b0841
<222> 636-735
<223> deor b0841 U00096 complement(881199..881957)
<313> 8355: 659-678
<400> 8355
TGGCAAGGTG CGTCCGGCGC GCATGGGTGA CCTGAAACGC TTTGATATTG 50
TGGTGAGCGA TTGTTGCCCG GAAGATGAGT ATGTGAAGTA CGCGCAGACG 100
<210> 8356
<211> 100
<212> DNA
<213> Escherichia coli K-12 MG1655
<220> deor b0841
<222> 536-635
<223> deor b0841 U00096 complement(881199..881957)
<313> 8356: 575-594
<400> 8356
GTAAAGGCGC TACCTGTTTT AATCTTGAAG AGTTGCCGGT AAAACACTGG 50
GCCATGTCGA TGGCGCAAAA GCATGTGCTG GTTGTCGACC ACAGTAAATT 100
<210> 8357
<211> 100
<212> DNA
<213> Escherichia coli K-12 MG1655
<220> phem b1716
<222> 1-45
<223> phem b1716 U00096 complement(1797250..1797294)
<313> 8357: 17-36
<400> 8357
ATGAATGCTG CTATTTTCCG CTTCTTTTTT TACTTTAGCA CCTGA 50
 100
<210> 8358
<211> 100
<212> DNA
<213> Escherichia coli K-12 MG1655
<220> ylbe b0520
<222> 903-1002
<223> ylbe b0520 U00096 547838..548839
<313> 8358: 966-985
<400> 8358
GGCGGGGATC GGGCAGATTG GCGCAGGCAC CGTGCGGGCA CCGCTGGCGT 50
GCTTTGAACA GGCGCTGGAA GCACTGGCTG AAAGCATGGG TATTGGTTGA 100
<210> 8359
<211> 100
<212> DNA

<213> Escherichia coli K-12 MG1655
<220> ylbe b0520
<222> 884-983
<223> ylbe b0520 U00096 547838..548839
<313> 8359: 906-925
<400> 8359
CCGGTATCGC CCATAAAGAG GCGGGGATCG GGCAGATTGG CGCAGGCACC 50
GTGCGGGCAC CGCTGGCGTG CTTTGAACAG GCGCTGGAAG CACTGGCTGA 100
<210> 8360
<211> 100
<212> DNA
<213> Escherichia coli K-12 MG1655
<220> ylbe b0520
<222> 716-815
<223> ylbe b0520 U00096 547838..548839
<313> 8360: 761-780
<400> 8360
TCGGTGCGGG TGGCATGGAA GCGGCAAGAG CGGTATCTGA AGAGATGGCG 50
GAAATTTACC TTGAACGCAA TATGCAGTTG CAGATCCCAA GCTGGGATTT 100
<210> 8361
<211> 100
<212> DNA
<213> Escherichia coli K-12 MG1655
<220> ylbf b0521
<222> 717-816
<223> ylbf b0521 U00096 548850..549665
<313> 8361: 760-779
<400> 8361
TGTTGCGATT GATTCGCTGC TGGCGCTGGG GCATACGTCA GGGGCAGATA 50
CGCTGCTGGG GTTCTGGCTT GGCCAACAAT TATTACAAGG AAAACCATGA 100
<210> 8362
<211> 100
<212> DNA
<213> Escherichia coli K-12 MG1655
<220> ylbf b0521
<222> 687-786
<223> ylbf b0521 U00096 548850..549665
<313> 8362: 710-729
<400> 8362
TCATGCTCTG AGTTGCCCGA AACGTACCGC TGTTGCGATT GATTCGCTGC 50
TGGCGCTGGG GCATACGTCA GGGGCAGATA CGCTGCTGGG GTTCTGGCTT 100
<210> 8363
<211> 100
<212> DNA
<213> Escherichia coli K-12 MG1655
<220> ylbf b0521
<222> 645-744
<223> ylbf b0521 U00096 548850..549665
<313> 8363: 663-682
<400> 8363
TGCCGCGCAA GGATATTTCG CCTCGCCACT CCTGCACTTT GTTCATGCTC 50
TGAGTTGCCC GAAACGTACC GCTGTTGCGA TTGATTCGCT GCTGGCGCTG 100
<210> 8364
<211> 100
<212> DNA
<213> Escherichia coli K-12 MG1655
<220> phep b0577
<222> 1249-1348
<223> phep b0577 U00096 601182..602558
<313> 8364: 1294-1313
<400> 8364
TGCATTGCCT TCCTCGGCAT GATTTTGCTG CTGATGTGCA CGATGGATGA 50
TATGCGCTTG TCAGCGATCC TGCTGCCGGT GTGGATTGTA TTCCTGTTTA 100
<210> 8365
<211> 100
<212> DNA
<213> Escherichia coli K-12 MG1655
<220> phep b0577
<222> 1219-1318
<223> phep b0577 U00096 601182..602558
<313> 8365: 1259-1278

<400> 8365
GCGCTGCTCT ATCCGTTCGG CAACTATCTC TGCATTGCCT TCCTCGGCAT 50
GATTTTGCTG CTGATGTGCA CGATGGATGA TATGCGCTTG TCAGCGATCC 100
<210> 8366
<211> 100
<212> DNA
<213> Escherichia coli K-12 MG1655
<220> phep b0577
<222> 1181-1280
<223> phep b0577 U00096 601182..602558
<313> 8366: 1220-1239
<400> 8366
CAGCGATGCG ACGTCAGGGG CGTGAAACAC AGTTTAAGGC GCTGCTCTAT 50
CCGTTCGGCA ACTATCTCTG CATTGCCTTC CTCGGCATGA TTTTGCTGCT 100
<210> 8367
<211> 100
<212> DNA
<213> Escherichia coli K-12 MG1655
<220> phes b1715
<222> 885-984
<223> phes b1715 U00096 complement(1795983..1796966)
<313> 8367: 939-958
<400> 8367
CGGGATGGGG ATGGAGCGTC TGACTATGTT GCGTTACGGC GTCACCGACC 50
TGCGTTCATT CTTCGAAAAC GATCTGCGTT TCCTCAAACA GTTTAAATAA 100
<210> 8368
<211> 100
<212> DNA
<213> Escherichia coli K-12 MG1655
<220> phes b1715
<222> 862-961
<223> phes b1715 U00096 complement(1795983..1796966)
<313> 8368: 899-918
<400> 8368
GAAGTTTACT CTGGTTTCGC CTTCGGGATG GGGATGGAGC GTCTGACTAT 50
GTTGCGTTAC GGCGTCACCG ACCTGCGTTC ATTCTTCGAA AACGATCTGC 100
<210> 8369
<211> 100
<212> DNA
<213> Escherichia coli K-12 MG1655
<220> phes b1715
<222> 807-906
<223> phes b1715 U00096 complement(1795983..1796966)
<313> 8369: 853-872
<400> 8369
GCTGGGCTGC GGGATGGTGC ATCCGAACGT GTTGCGTAAC GTTGGCATCG 50
ACCCGGAAGT TTACTCTGGT TTCGCCTTCG GGATGGGGAT GGAGCGTCTG 100
<210> 8370
<211> 100
<212> DNA
<213> Escherichia coli K-12 MG1655
<220> phet b1714
<222> 2289-2388
<223> phet b1714 U00096 complement(1793581..1795968)
<313> 8370: 2351-2370
<400> 8370
AGATACCAGC CGTACACTCG AAGAAGAGGA GATTGCCGCT ACCGTCGCCA 50
AATGTGTAGA GGCATTAAAA GAGCGATTCC AGGCATCATT GAGGGATTGA 100
<210> 8371
<211> 100
<212> DNA
<213> Escherichia coli K-12 MG1655
<220> phet b1714
<222> 2284-2383
<223> phet b1714 U00096 complement(1793581..1795968)
<313> 8371: 2321-2340
<400> 8371
CTGCAAGATA CCAGCCGTAC ACTCGAAGAA GAGGAGATTG CCGCTACCGT 50
CGCCAAATGT GTAGAGGCAT TAAAAGAGCG ATTCCAGGCA TCATTGAGGG 100
<210> 8372
<211> 100

<212> DNA
<213> Escherichia coli K-12 MG1655
<220> phet b1714
<222> 2228-2327
<223> phet b1714 U00096 complement(1793581..1795968)
<313> 8372: 2279-2298
<400> 8372
TGTACCGCGG TAAGGGTGTT GCGGAGGGGT ATAAGAGCCT CGCCATAAGC 50
CTGATCCTGC AAGATACCAG CCGTACACTC GAAGAAGAGG AGATTGCCGC 100
<210> 8373
<211> 100
<212> DNA
<213> Escherichia coli K-12 MG1655
<220> rem b1562
<222> 153-252
<223> rem b1562 U00096 complement(1642675..1642926)
<313> 8373: 205-224
<400> 8373
GGACGGATTT ATCTTGCGCG AAGGTGAGTT TGTCACTTCA TTACAGGCAT 50
TGAAAGAGAT CGGAATAAAA GCTGGCTTTT CTGCTTTTTC AGGAGAATAA 100
<210> 8374
<211> 100
<212> DNA
<213> Escherichia coli K-12 MG1655
<220> rem b1562
<222> 151-250
<223> rem b1562 U00096 complement(1642675..1642926)
<313> 8374: 183-202
<400> 8374
ACGGACGGAT TTATCTTGCG CGAAGGTGAG TTTGTCACTT CATTACAGGC 50
ATTGAAAGAG ATCGGAATAA AAGCTGGCTT TTCTGCTTTT TCAGGAGAAT 100
<210> 8375
<211> 100
<212> DNA
<213> Escherichia coli K-12 MG1655
<220> rem b1562
<222> 57-156
<223> rem b1562 U00096 complement(1642675..1642926)
<313> 8375: 86-105
<400> 8375
GTGCGTTGAA AATGGAAATC TTGTTAGTCA TTACCGCATT ATGTGTTTGC 50
GAAAGAATGG GGCTGCGTTA ATTAATTTTG TGGATGCTCG GGTCACGGAC 100
<210> 8376
<211> 100
<212> DNA
<213> Escherichia coli K-12 MG1655
<220> rep b3779
<222> 1923-2022
<223> rep b3779 U00096 3958292..3960313
<313> 8376: 1985-2004
<400> 8376
ACAGGAGCGC AAAGTGGTCA GCGCCGAAGA ACGGATGCAG AAAGGGCAAA 50
GCCATCTGGC GAATCTGAAA GCGATGATGG CGGCAAAACG AGGGAAATAA 100
<210> 8377
<211> 100
<212> DNA
<213> Escherichia coli K-12 MG1655
<220> rep b3779
<222> 1917-2016
<223> rep b3779 U00096 3958292..3960313
<313> 8377: 1951-1970
<400> 8377
TTGGGAACAG GAGCGCAAAG TGGTCAGCGC CGAAGAACGG ATGCAGAAAG 50
GGCAAAGCCA TCTGGCGAAT CTGAAAGCGA TGATGGCGGC AAAACGAGGG 100
<210> 8378
<211> 100
<212> DNA
<213> Escherichia coli K-12 MG1655
<220> rep b3779
<222> 1887-1986
<223> rep b3779 U00096 3958292..3960313

<313> 8378: 1908-1927
<400> 8378
TTTGCTGGAG CTGCCGCAGG ATGATCTGAT TTGGGAACAG GAGCGCAAAG 50
TGGTCAGCGC CGAAGAACGG ATGCAGAAAG GGCAAAGCCA TCTGGCGAAT 100
<210> 8379
<211> 100
<212> DNA
<213> Escherichia coli K-12 MG1655
<220> ylca b0572
<222> 585-684
<223> ylca b0572 U00096 complement(593983..594666)
<313> 8379: 663-682
<400> 8379
GAAGCGGCTG CGCGGCAAAA TCGACAACGA CTTTGAGCCG AAGCTAATTC 50
AGACCGTGCG CGGCGTGGGT TACATGCTTG AGGTGCCGGA TGGTCAGTAA 100
<210> 8380
<211> 100
<212> DNA
<213> Escherichia coli K-12 MG1655
<220> ylca b0572
<222> 585-684
<223> ylca b0572 U00096 complement(593983..594666)
<313> 8380: 613-632
<400> 8380
GAAGCGGCTG CGCGGCAAAA TCGACAACGA CTTTGAGCCG AAGCTAATTC 50
AGACCGTGCG CGGCGTGGGT TACATGCTTG AGGTGCCGGA TGGTCAGTAA 100
<210> 8381
<211> 100
<212> DNA
<213> Escherichia coli K-12 MG1655
<220> ylca b0572
<222> 513-612
<223> ylca b0572 U00096 complement(593983..594666)
<313> 8381: 559-578
<400> 8381
CCGCTCGCTT ATCGCCTCGC AGGTATGGGA CATGAATTTT GACAGCGATA 50
CCAATGCTAT TGATGTGGCG GTGAAGCGGC TGCGCGGCAA AATCGACAAC 100
<210> 8382
<211> 100
<212> DNA
<213> Escherichia coli K-12 MG1655
<220> ylcb b0573
<222> 1275-1374
<223> ylcb b0573 U00096 594823..596196
<313> 8382: 1310-1329
<400> 8382
CGAGCGTTCT TTATTTGCAA CCCGACAAAC TTTACTTGAT CTGAATTATG 50
CCCGTCAGGT TAACGAAATT TCTTTGTATA CCGCACTTGG TGGCGGTTGA 100
<210> 8383
<211> 100
<212> DNA
<213> Escherichia coli K-12 MG1655
<220> ylcb b0573
<222> 1230-1329
<223> ylcb b0573 U00096 594823..596196
<313> 8383: 1255-1274
<400> 8383
ATTATATCAG CACGGCGCAG TAAGTTATCT GGAAGTGCTG GATGCCGAGC 50
GTTCTTTATT TGCAACCCGA CAAACTTTAC TTGATCTGAA TTATGCCCGT 100
<210> 8384
<211> 100
<212> DNA
<213> Escherichia coli K-12 MG1655
<220> ylcb b0573
<222> 1175-1274
<223> ylcb b0573 U00096 594823..596196
<313> 8384: 1213-1232
<400> 8384
GCGCCCAGCA GCGTTATCTG GCATCGCTGC AAATTACTTT GCAACGGGCG 50
CGGGCATTAT ATCAGCACGG CGCAGTAAGT TATCTGGAAG TGCTGGATGC 100
<210> 8385

<211> 100
<212> DNA
<213> Escherichia coli K-12 MG1655
<220> ylcc b0574
<222> 234-333
<223> ylcc b0574 U00096 596354..596686
<313> 8385: 282-301
<400> 8385
GACGAAAATG AGTGAAATTA AAACCGGCGA CAAAGTGGCG TTTAATTTTG 50
TCCAGCAGGG CAACCTTTCT TTATTACAGG ATATTAAAGT CAGCCAGTAA 100
<210> 8386
<211> 100
<212> DNA
<213> Escherichia coli K-12 MG1655
<220> ylcc b0574
<222> 197-296
<223> ylcc b0574 U00096 596354..596686
<313> 8386: 248-267
<400> 8386
GGCCGGAGAT GACCATGCGC TTTACCATCA CCCCGCAGAC GAAAATGAGT 50
GAAATTAAAA CCGGCGACAA AGTGGCGTTT AATTTTGTCC AGCAGGGCAA 100
<210> 8387
<211> 100
<212> DNA
<213> Escherichia coli K-12 MG1655
<220> ylcc b0574
<222> 137-236
<223> ylcc b0574 U00096 596354..596686
<313> 8387: 160-179
<400> 8387
GTATCGATCT GGAAAGCAAA AAAATCACCA TCCATCACGA TCCGATTGCT 50
GCCGTGAACT GGCCGGAGAT GACCATGCGC TTTACCATCA CCCCGCAGAC 100
<210> 8388
<211> 100
<212> DNA
<213> Escherichia coli K-12 MG1655
<220> cdd b2144
<222> 786-885
<223> cdd b2144 U00096 2229864..2230748
<313> 8388: 863-882
<400> 8388
GGCAGAAAAA GCCGATGCGC CGTTGATTCA GTGGGATGCC ACCTCCGCAA 50
CGCTGAAAGC TCTCGGCTGT CACAGTATCG ACCGAGTGCT TCTCGCTTAA 100
<210> 8389
<211> 100
<212> DNA
<213> Escherichia coli K-12 MG1655
<220> cdd b2144
<222> 764-863
<223> cdd b2144 U00096 2229864..2230748
<313> 8389: 790-809
<400> 8389
CGGATATCCA GCGCGCGGTT CTGGCAGAAA AAGCCGATGC GCCGTTGATT 50
CAGTGGGATG CCACCTCCGC AACGCTGAAA GCTCTCGGCT GTCACAGTAT 100
<210> 8390
<211> 100
<212> DNA
<213> Escherichia coli K-12 MG1655
<220> cdd b2144
<222> 691-790
<223> cdd b2144 U00096 2229864..2230748
<313> 8390: 726-745
<400> 8390
GCCGCATTCA ACCCGACTCT GCCACCGTTG CAGGGAGCGT TAATTCTGTT 50
GAATCTCAAG GGTTATGATT ACCCGGATAT CCAGCGCGCG GTTCTGGCAG 100
<210> 8391
<211> 100
<212> DNA
<213> Escherichia coli K-12 MG1655
<220> ylcd b0575
<222> 1121-1220

<223> ylcd b0575 U00096 596702..597925
<313> 8391: 1171-1190
<400> 8391
GTGAAAAGGT GGTTTCCAGC GGCCTGTTCC TGATTGATTC CGAAGCCAAT  50
ATTTCTGGCG CACTGGAGCG GATGCGCTCT GAAAGTGCTA CCCATGCGCA  100
<210> 8392
<211> 100
<212> DNA
<213> Escherichia coli K-12 MG1655
<220> ylcd b0575
<222> 1067-1166
<223> ylcd b0575 U00096 596702..597925
<313> 8392: 1128-1147
<400> 8392
CTGTTTTCCA GGCATCGCAA GGCGTCACCG CATTACGCTC TGGTCTGGCG  50
GAAGGTGAAA AGGTGGTTTC CAGCGGCCTG TTCCTGATTG ATTCCGAAGC  100
<210> 8393
<211> 100
<212> DNA
<213> Escherichia coli K-12 MG1655
<220> ylcd b0575
<222> 1030-1129
<223> ylcd b0575 U00096 596702..597925
<313> 8393: 1082-1101
<400> 8393
GTTGATGCCG ACGGGCGCTT TGTACCGAAA CGCGTTGCTG TTTTCCAGGC  50
ATCGCAAGGC GTCACCGCAT TACGCTCTGG TCTGGCGGAA GGTGAAAAGG  100
<210> 8394
<211> 100
<212> DNA
<213> Escherichia coli K-12 MG1655
<220> ylce b0564
<222> 72-171
<223> ylce b0564 U00096 582098..582283
<313> 8394: 89-108
<400> 8394
GACCGTCACC ACACAGTTCT ACATCCAGGA ATTCAGTGAG TATATTGCGC  50
CACTGCAGGA TGCTGTAGAT CTGGAAATCG CAACGGAGGA AGAAAGATCG  100
<210> 8395
<211> 100
<212> DNA
<213> Escherichia coli K-12 MG1655
<220> ylce b0564
<222> 39-138
<223> ylce b0564 U00096 582098..582283
<313> 8395: 89-108
<400> 8395
TGCTTCAATA CAGCAGGAGC CGAATGGCTG GAAGACCGTC ACCACACAGT  50
TCTACATCCA GGAATTCAGT GAGTATATTG CGCCACTGCA GGATGCTGTA  100
<210> 8396
<211> 100
<212> DNA
<213> Escherichia coli K-12 MG1655
<220> ylce b0564
<222> 42-141
<223> ylce b0564 U00096 582098..582283
<313> 8396: 76-95
<400> 8396
TTCAATACAG CAGGAGCCGA ATGGCTGGAA GACCGTCACC ACACAGTTCT  50
ACATCCAGGA ATTCAGTGAG TATATTGCGC CACTGCAGGA TGCTGTAGAT  100
<210> 8397
<211> 100
<212> DNA
<213> Escherichia coli K-12 MG1655
<220> cdh b3919
<222> 657-756
<223> cdh b3919 U00096 4107510..4108265
<313> 8397: 716-735
<400> 8397
TAACTCATTT GTATTGCTGG CGACACAACG AAATCTACTG ACGCTTAACC  50
GTGCTTCAGC CGAGGAAATT CAGGATCATC AGTGTGAGAT TTTGCGTTAA  100

<210> 8398
<211> 100
<212> DNA
<213> Escherichia coli K-12 MG1655
<220> cdh b3919
<222> 594-693
<223> cdh b3919 U00096 4107510..4108265
<313> 8398: 639-658
<400> 8398
AGAAGTACCT GAGGCGCGGG AACATATGGG ACGCTACGGG CTGGCGATGG 50
TACGGCAGAG TGATAACTCA TTTGTATTGC TGGCGACACA ACGAAATCTA 100
<210> 8399
<211> 100
<212> DNA
<213> Escherichia coli K-12 MG1655
<220> cdh b3919
<222> 573-672
<223> cdh b3919 U00096 4107510..4108265
<313> 8399: 610-629
<400> 8399
CCCATTTATG ATGCTGGCAG AAGAAGTACC TGAGGCGCGG GAACATATGG 50
GACGCTACGG GCTGGCGATG GTACGGCAGA GTGATAACTC ATTTGTATTG 100
<210> 8400
<211> 100
<212> DNA
<213> Escherichia coli K-12 MG1655
<220> tkta b2936
<222> 1893-1992
<223> tkta b2936 U00096 complement(3077663..3079654)
<313> 8400: 1941-1960
<400> 8400
TATGACCACC TTCGGTGAAT CTGCTCCGGC AGAGCTGCTG TTTGAAGAGT 50
TCGGCTTCAC TGTTGATAAC GTTGTTGCGA AAGCAAAAGA ACTGCTGTAA 100
<210> 8401
<211> 100
<212> DNA
<213> Escherichia coli K-12 MG1655
<220> tkta b2936
<222> 1843-1942
<223> tkta b2936 U00096 complement(3077663..3079654)
<313> 8401: 1898-1917
<400> 8401
ATTGCTGACT ACTGGTACAA GTATGTTGGC CTGAACGGTG CTATCGTCGG 50
TATGACCACC TTCGGTGAAT CTGCTCCGGC AGAGCTGCTG TTTGAAGAGT 100
<210> 8402
<211> 100
<212> DNA
<213> Escherichia coli K-12 MG1655
<220> tkta b2936
<222> 1760-1859
<223> tkta b2936 U00096 complement(3077663..3079654)
<313> 8402: 1797-1816
<400> 8402
ACGCATTTGA CAAGCAGGAT GCTGCTTACC GTGAATCCGT ACTGCCGAAA 50
GCGGTTACTG CACGCGTTGC TGTAGAAGCG GGTATTGCTG ACTACTGGTA 100
<210> 8403
<211> 100
<212> DNA
<213> Escherichia coli K-12 MG1655
<220> tktb tktB
<222> 1905-2004
<223> tktb tktB U00096 2577656..2579659
<313> 8403: 1973-1992
<400> 8403
GGAATCTGCT CCGGCGGATA AGCTGTTCCC GTTCTTTGGC TTTACCGCCG 50
AGAATATTGT GGCAAAAGCG CATAAGGTGC TGGGAGTGAA AGGTGCCTGA 100
<210> 8404
<211> 100
<212> DNA
<213> Escherichia coli K-12 MG1655
<220> tktb tktB

<222> 1884-1983
<223> tktb tktB U00096 2577656..2579659
<313> 8404: 1935-1954
<400> 8404
TGTCGGGATG ACGGGTTACG GGGAATCTGC TCCGGCGGAT AAGCTGTTCC 50
CGTTCTTTGG CTTTACCGCC GAGAATATTG TGGCAAAAGC GCATAAGGTG 100
<210> 8405
<211> 100
<212> DNA
<213> Escherichia coli K-12 MG1655
<220> tktb tktB
<222> 1695-1794
<223> tktb tktB U00096 2577656..2579659
<313> 8405: 1754-1773
<400> 8405
AGCGGCAGAG AAATTAGCAG GAGAAGGTCG CAATGTACGC GTAGTTTCCC 50
TGCCCTCGAC CGATATTTTC GACGCCCAGG ATGAGGAATA TCGGGAGTCG 100
<210> 8406
<211> 100
<212> DNA
<213> Escherichia coli K-12 MG1655
<220> poxb poxB
<222> 1620-1719
<223> poxb poxB U00096 complement(908554..910272)
<313> 8406: 1683-1702
<400> 8406
CGAACAGGCC AAAGGTTTCA GCCTGTATAT GCTGCGCGCA ATCATCAGCG 50
GACGCGGTGA TGAAGTGATC GAACTGGCGA AAACAAACTG GCTAAGGTAA 100
<210> 8407
<211> 100
<212> DNA
<213> Escherichia coli K-12 MG1655
<220> poxb poxB
<222> 1591-1690
<223> poxb poxB U00096 complement(908554..910272)
<313> 8407: 1605-1624
<400> 8407
GAGTTAGCCA TTCCACCGCA GATCAAACTC GAACAGGCCA AAGGTTTCAG 50
CCTGTATATG CTGCGCGCAA TCATCAGCGG ACGCGGTGAT GAAGTGATCG 100
<210> 8408
<211> 100
<212> DNA
<213> Escherichia coli K-12 MG1655
<220> poxb poxB
<222> 1487-1586
<223> poxb poxB U00096 complement(908554..910272)
<313> 8408: 1525-1544
<400> 8408
TTACGGGTAT CCGTGTAGAA AAAGCGTCTG AAGTTGATGA AGCCCTGCAA 50
CGCGCCTTCT CCATCGACGG TCCGGTGTTG GTGGATGTGG TGGTCGCCAA 100
<210> 8409
<211> 100
<212> DNA
<213> Escherichia coli K-12 MG1655
<220> b3975 b3976
<222> 57-156
<223> b3975 b3976 U00096 complement(4172792..4172947)
<313> 8409: 126-145
<400> 8409
CATTTTATGC GACACGATGA AGAAACAGCC GAACGGAGGG CAGATTAGCA 50
CACTTTTTCA ACATCATTGT GCTCAACAAT GCGCTCCTGC TAAACCATAA 100
<210> 8410
<211> 100
<212> DNA
<213> Escherichia coli K-12 MG1655
<220> b3975 b3976
<222> 57-156
<223> b3975 b3976 U00096 complement(4172792..4172947)
<313> 8410: 110-129
<400> 8410
CATTTTATGC GACACGATGA AGAAACAGCC GAACGGAGGG CAGATTAGCA 50

CACTTTTTCA ACATCATTGT GCTCAACAAT GCGCTCCTGC TAAACCATAA 100
<210> 8411
<211> 100
<212> DNA
<213> Escherichia coli K-12 MG1655
<220> b3975 b3976
<222> 57-156
<223> b3975 b3976 U00096 complement(4172792..4172947)
<313> 8411: 99-118
<400> 8411
CATTTTATGC GACACGATGA AGAAACAGCC GAACGGAGGG CAGATTAGCA 50
CACTTTTTCA ACATCATTGT GCTCAACAAT GCGCTCCTGC TAAACCATAA 100
<210> 8412
<211> 100
<212> DNA
<213> Escherichia coli K-12 MG1655
<220> tdca b3119
<222> 806-905
<223> tdca b3119 U00096 complement(3263768..3264706)
<313> 8412: 857-876
<400> 8412
CTGTGGCACA ATATGCCGCG GTATGGTCGA AAAATTATCG TATTAAAAAA 50
GCAGCATCGG TTTTGGTGGA ATTAGCCAAA GAGTATTCAT CTTATAATGG 100
<210> 8413
<211> 100
<212> DNA
<213> Escherichia coli K-12 MG1655
<220> tdca b3119
<222> 547-646
<223> tdca b3119 U00096 complement(3263768..3264706)
<313> 8413: 601-620
<400> 8413
TGCACCGGCA CCACCACGCT GGAGTCGTTG AAGAACGAAC AGTGGGTGTT 50
GCCACAAACT AATATGGGGT ACTACAGCGA ACTGCTTACT ACGTTACAAA 100
<210> 8414
<211> 100
<212> DNA
<213> Escherichia coli K-12 MG1655
<220> tdca b3119
<222> 519-618
<223> tdca b3119 U00096 complement(3263768..3264706)
<313> 8414: 565-584
<400> 8414
TGTGCTGGTA GCCAGTAAGT CCCGAACATG CACCGGCACC ACCACGCTGG 50
AGTCGTTGAA GAACGAACAG TGGGTGTTGC CACAAACTAA TATGGGGTAC 100
<210> 8415
<211> 100
<212> DNA
<213> Escherichia coli K-12 MG1655
<220> rfe b3785
<222> 1005-1104
<223> rfe b3785 U00096 3965531..3966634
<313> 8415: 1075-1094
<400> 8415
ATATTGCATT AAGCGTGCCT GGAAAGTTGC TCGCTTTATT AAGCGCGTAA 50
AACGCAGACT GCGTAGAAAT CGTGGTGGCA GCCCCAATTT AACCAAATAA 100
<210> 8416
<211> 100
<212> DNA
<213> Escherichia coli K-12 MG1655
<220> rfe b3785
<222> 984-1083
<223> rfe b3785 U00096 3965531..3966634
<313> 8416: 1031-1050
<400> 8416
GCTAGCATTC TTCCTCTATG GATATTGCAT TAAGCGTGCC TGGAAAGTTG 50
CTCGCTTTAT TAAGCGCGTA AAACGCAGAC TGCGTAGAAA TCGTGGTGGC 100
<210> 8417
<211> 100
<212> DNA
<213> Escherichia coli K-12 MG1655

<220> rfe b3785
<222> 920-1019
<223> rfe b3785 U00096 3965531..3966634
<313> 8417: 952-971
<400> 8417
CCATTGGCGT GCTGGCAGAA TATTCTCATT TTGTCCCGGA GTGGGTCATG 50
CTGGTGCTCT TTTTGCTAGC ATTCTTCCTC TATGGATATT GCATTAAGCG 100
<210> 8418
<211> 100
<212> DNA
<213> Escherichia coli K-12 MG1655
<220> tdcb b3118
<222> 891-990
<223> tdcb b3118 U00096 complement(3262680..3263669)
<313> 8418: 936-955
<400> 8418
AGACCAATAT ATTCAAAACA GAAAAACCGT CAGTATTATT TCCGGCGGCA 50
ATATCGATCT TTCTCGCGTC TCTCAAATCA CCGGTTTCGT TGACGCTTAA 100
<210> 8419
<211> 100
<212> DNA
<213> Escherichia coli K-12 MG1655
<220> tdcb b3118
<222> 792-891
<223> tdcb b3118 U00096 complement(3262680..3263669)
<313> 8419: 846-865
<400> 8419
CGAAATCAGA AACAGTATGA TTGCCTTAAT TCAGCGCAAT AAAGTCGTCA 50
CCGAAGGCGC AGGCGCTCTG GCATGTGCTG CATTATTAAG CGGTAAATTA 100
<210> 8420
<211> 100
<212> DNA
<213> Escherichia coli K-12 MG1655
<220> tdcb b3118
<222> 743-842
<223> tdcb b3118 U00096 complement(3262680..3263669)
<313> 8420: 782-801
<400> 8420
ACGAAATCGT TCGTGAATTA GTCGATGACA TCGTGCTGGT CAGCGAAGAC 50
GAAATCAGAA ACAGTATGAT TGCCTTAATT CAGCGCAATA AAGTCGTCAC 100
<210> 8421
<211> 100
<212> DNA
<213> Escherichia coli K-12 MG1655
<220> tdcc b3117
<222> 1233-1332
<223> tdcc b3117 U00096 complement(3261327..3262658)
<313> 8421: 1286-1305
<400> 8421
AGCGCCGTCT CTGGCGAAAT ACCGTGGTCG TCTGGATAAC GTGTTTGTTA 50
CCGTGATTGG TCTGCTGACC ATCCTGAACA TCGTATACAA ACTGTTTTAA 100
<210> 8422
<211> 100
<212> DNA
<213> Escherichia coli K-12 MG1655
<220> tdcc b3117
<222> 1189-1288
<223> tdcc b3117 U00096 complement(3261327..3262658)
<313> 8422: 1233-1252
<400> 8422
ATCGCATCCC TGCTGTGCCT GTTGCCGATG TATGCCATCC GTAAAGCGCC 50
GTCTCTGGCG AAATACCGTG GTCGTCTGGA TAACGTGTTT GTTACCGTGA 100
<210> 8423
<211> 100
<212> DNA
<213> Escherichia coli K-12 MG1655
<220> tdcc b3117
<222> 1120-1219
<223> tdcc b3117 U00096 complement(3261327..3262658)
<313> 8423: 1146-1165
<400> 8423

TCCACCTGGG TTGTTGCCTA CGCCAACCCG AACATCCTTG ACCTGATTGA 50
AGCCATGGGC GCACCGATTA TCGCATCCCT GCTGTGCCTG TTGCCGATGT 100
<210> 8424
<211> 100
<212> DNA
<213> Escherichia coli K-12 MG1655
<220> tdcd b3116
<222> 1122-1221
<223> tdcd b3116 U00096 complement(3260093..3261313)
<313> 8424: 1183-1202
<400> 8424
GCGTGTCATT TGTGCCGTTA TTCCGACTAA CGAAGAAAAA ATGATTGCTT 50
TGGATGCCAT TCATTTAGGC AAAGTTAACG CGCCCGCAGA ATTTGCATAA 100
<210> 8425
<211> 100
<212> DNA
<213> Escherichia coli K-12 MG1655
<220> tdcd b3116
<222> 1087-1186
<223> tdcd b3116 U00096 complement(3260093..3261313)
<313> 8425: 1131-1150
<400> 8425
TCCTGTGGTG AGCGAATTGT TTCCAGTGAA AATGCGCGTG TCATTTGTGC 50
CGTTATTCCG ACTAACGAAG AAAAAATGAT TGCTTTGGAT GCCATTCATT 100
<210> 8426
<211> 100
<212> DNA
<213> Escherichia coli K-12 MG1655
<220> tdcd b3116
<222> 1059-1158
<223> tdcd b3116 U00096 complement(3260093..3261313)
<313> 8426: 1098-1117
<400> 8426
TGATACAGAA ATGAATAATC GCTCTAACTC CTGTGGTGAG CGAATTGTTT 50
CCAGTGAAAA TGCGCGTGTC ATTTGTGCCG TTATTCCGAC TAACGAAGAA 100
<210> 8427
<211> 100
<212> DNA
<213> Escherichia coli K-12 MG1655
<220> tdce b3115
<222> 2142-2241
<223> tdce b3115 U00096 complement(3257819..3260059)
<313> 8427: 2198-2217
<400> 8427
AATGAATCGG GAAATGCTGC TGGATGCCAT CGAGCACCCG GAAAAATATC 50
CTAACCTGAC AATCCGTGTC TCTGGCTACG CGTGCGCTTC AACGCACTGA 100
<210> 8428
<211> 100
<212> DNA
<213> Escherichia coli K-12 MG1655
<220> tdce b3115
<222> 2114-2213
<223> tdce b3115 U00096 complement(3257819..3260059)
<313> 8428: 2155-2174
<400> 8428
AAGGCGGTCA ACACCTCAAC GTCAACGTAA TGAATCGGGA AATGCTGCTG 50
GATGCCATCG AGCACCCGGA AAAATATCCT AACCTGACAA TCCGTGTCTC 100
<210> 8429
<211> 100
<212> DNA
<213> Escherichia coli K-12 MG1655
<220> tdce b3115
<222> 2065-2164
<223> tdce b3115 U00096 complement(3257819..3260059)
<313> 8429: 2116-2135
<400> 8429
AACCTTGTCG GCCTGCTGGA TGGGTATTTC CACCACGAAG CGGATGTCGA 50
AGGCGGTCAA CACCTCAACG TCAACGTAAT GAATCGGGAA ATGCTGCTGG 100
<210> 8430
<211> 100
<212> DNA

<213> Escherichia coli K-12 MG1655
<220> minc b1177
<222> 597-696
<223> minc b1177 U00096 complement(1224608..1225303)
<313> 8430: 672-691
<400> 8430
TGAATACTGG CTGAGTGATC AAATCCCAGC AGAATTTTAT GGCAAAGCGG 50
CGCGACTGCA GTTAGTCGAA AACGCTTTGA CCGTTCAACC GTTAAATTGA 100
<210> 8431
<211> 100
<212> DNA
<213> Escherichia coli K-12 MG1655
<220> minc b1177
<222> 595-694
<223> minc b1177 U00096 complement(1224608..1225303)
<313> 8431: 619-638
<400> 8431
GGTGAATACT GGCTGAGTGA TCAAATCCCA GCAGAATTTT ATGGCAAAGC 50
GGCGCGACTG CAGTTAGTCG AAAACGCTTT GACCGTTCAA CCGTTAAATT 100
<210> 8432
<211> 100
<212> DNA
<213> Escherichia coli K-12 MG1655
<220> minc b1177
<222> 529-628
<223> minc b1177 U00096 complement(1224608..1225303)
<313> 8432: 577-596
<400> 8432
GCAAGTGGTG ACCGGGAAAC GCAAATATTT TGTACGAACC TGATGGCGGA 50
ACTGGTGTCC ATCGCAGGTG AATACTGGCT GAGTGATCAA ATCCCAGCAG 100
<210> 8433
<211> 100
<212> DNA
<213> Escherichia coli K-12 MG1655
<220> mind b1176
<222> 714-813
<223> mind b1176 U00096 complement(1223772..1224584)
<313> 8433: 779-798
<400> 8433
CTACGCAGAT ACCGTAGAAC GTCTGTTGGG AGAAGAACGT CCTTTCCGCT 50
TCATTGAAGA AGAGAAGAAA GGCTTCCTCA AACGCTTGTT CGGAGGATAA 100
<210> 8434
<211> 100
<212> DNA
<213> Escherichia coli K-12 MG1655
<220> mind b1176
<222> 706-805
<223> mind b1176 U00096 complement(1223772..1224584)
<313> 8434: 741-760
<400> 8434
GGTAAAGCCT ACGCAGATAC CGTAGAACGT CTGTTGGGAG AAGAACGTCC 50
TTTCCGCTTC ATTGAAGAAG AGAAGAAAGG CTTCCTCAAA CGCTTGTTCG 100
<210> 8435
<211> 100
<212> DNA
<213> Escherichia coli K-12 MG1655
<220> mind b1176
<222> 642-741
<223> mind b1176 U00096 complement(1223772..1224584)
<313> 8435: 693-712
<400> 8435
TCAATCAGTA TTGCGCGCCT CTAACCAGGG TGAACCGGTC ATTCTCGACA 50
TTAACGCCGA TGCGGGTAAA GCCTACGCAG ATACCGTAGA ACGTCTGTTG 100
<210> 8436
<211> 100
<212> DNA
<213> Escherichia coli K-12 MG1655
<220> mine b1175
<222> 168-267
<223> mine b1175 U00096 complement(1223502..1223768)
<313> 8436: 245-264

<400> 8436
TGATCCTGAG ATGGTAACCG TACAGCTTGA GCAAAAAGAT GGCGATATTT 50
CTATTCTTGA GCTGAACGTG ACCTTACCGG AAGCAGAAGA GCTGAAATAA 100
<210> 8437
<211> 100
<212> DNA
<213> Escherichia coli K-12 MG1655
<220> mine b1175
<222> 168-267
<223> mine b1175 U00096 complement(1223502..1223768)
<313> 8437: 219-238
<400> 8437
TGATCCTGAG ATGGTAACCG TACAGCTTGA GCAAAAAGAT GGCGATATTT 50
CTATTCTTGA GCTGAACGTG ACCTTACCGG AAGCAGAAGA GCTGAAATAA 100
<210> 8438
<211> 100
<212> DNA
<213> Escherichia coli K-12 MG1655
<220> mine b1175
<222> 168-267
<223> mine b1175 U00096 complement(1223502..1223768)
<313> 8438: 221-240
<400> 8438
TGATCCTGAG ATGGTAACCG TACAGCTTGA GCAAAAAGAT GGCGATATTT 50
CTATTCTTGA GCTGAACGTG ACCTTACCGG AAGCAGAAGA GCTGAAATAA 100
<210> 8439
<211> 100
<212> DNA
<213> Escherichia coli K-12 MG1655
<220> b1007 b1007
<222> 335-434
<223> b1007 b1007 U00096 complement(1069083..1069541)
<313> 8439: 378-397
<400> 8439
TGGTGAGCGT CGGCACCCAC GACATTCTGT TTTGCGCCAT CGAAGCGATT 50
CATCGTCACA CCACACCCTA CGGGCTGGTG TGGTTCGATC GCAGTTATCA 100
<210> 8440
<211> 100
<212> DNA
<213> Escherichia coli K-12 MG1655
<220> b1007 b1007
<222> 300-399
<223> b1007 b1007 U00096 complement(1069083..1069541)
<313> 8440: 343-362
<400> 8440
GGCTCTGGTT TCGTTTGACT GCCGTATCAG CCAGGTGGTG AGCGTCGGCA 50
CCCACGACAT TCTGTTTTGC GCCATCGAAG CGATTCATCG TCACACCACA 100
<210> 8441
<211> 100
<212> DNA
<213> Escherichia coli K-12 MG1655
<220> b1007 b1007
<222> 242-341
<223> b1007 b1007 U00096 complement(1069083..1069541)
<313> 8441: 295-314
<400> 8441
ACCGCTTTGC CGCCGCCCGC TGGCAGACGG GGGTGACCGG ATGCCCGCAA 50
CTGGAAGAGG CTCTGGTTTC GTTTGACTGC CGTATCAGCC AGGTGGTGAG 100
<210> 8442
<211> 100
<212> DNA
<213> Escherichia coli K-12 MG1655
<220> b1008 b1008
<222> 464-563
<223> b1008 b1008 U00096 complement(1069588..1070178)
<313> 8442: 487-506
<400> 8442
ACGACGCCTT TTTTACGGGC AGCACGCTGA AGAGCAATCT GCTGATTAAT 50
ATCGGCTATG GCGATAGCAG CAAGCTTTAT GCGCGCCTGC CACGTCTGTC 100
<210> 8443
<211> 100

<212> DNA
<213> Escherichia coli K-12 MG1655
<220> b1008 b1008
<222> 329-428
<223> b1008 b1008 U00096 complement(1069588..1070178)
<313> 8443: 365-384
<400> 8443
TTACCTCCAG CCCACAACTT GCCGAAGAAA CAGCGTTTCG CAACAGTTCC 50
ATGCAGGCGG CCTATCTGAT CGTCGCCTGC CGGGCGCTGG GACTGGATAC 100
<210> 8444
<211> 100
<212> DNA
<213> Escherichia coli K-12 MG1655
<220> b1008 b1008
<222> 293-392
<223> b1008 b1008 U00096 complement(1069588..1070178)
<313> 8444: 324-343
<400> 8444
CACTACTGTT TCCCCACGGT GATGCCCGCA GTTGGTTTAC CTCCAGCCCA 50
CAACTTGCCG AAGAAACAGC GTTTCGCAAC AGTTCCATGC AGGCGGCCTA 100
<210> 8445
<211> 100
<212> DNA
<213> Escherichia coli K-12 MG1655
<220> b1009 b1009
<222> 702-801
<223> b1009 b1009 U00096 complement(1070188..1070988)
<313> 8445: 764-783
<400> 8445
TGGCGGACAC GCCTGCAACG TGACCGATCC CGAAACGTTT AATGCTCTGT 50
TACTCAACGG GCTTGCCAGC CTGTTACATC ACCGTGAAGC CGCCCTGTAA 100
<210> 8446
<211> 100
<212> DNA
<213> Escherichia coli K-12 MG1655
<220> b1009 b1009
<222> 670-769
<223> b1009 b1009 U00096 complement(1070188..1070988)
<313> 8446: 705-724
<400> 8446
CTGCCCGATA GCCAGAAAAT GGTGATGCCC TATGGCGGAC ACGCCTGCAA 50
CGTGACCGAT CCCGAAACGT TTAATGCTCT GTTACTCAAC GGGCTTGCCA 100
<210> 8447
<211> 100
<212> DNA
<213> Escherichia coli K-12 MG1655
<220> b1009 b1009
<222> 645-744
<223> b1009 b1009 U00096 complement(1070188..1070988)
<313> 8447: 678-697
<400> 8447
ATGTTCCAGT GAACTTCATG CCGCCCTGCC CGATAGCCAG AAAATGGTGA 50
TGCCCTATGG CGGACACGCC TGCAACGTGA CCGATCCCGA AACGTTTAAT 100
<210> 8448
<211> 100
<212> DNA
<213> Escherichia coli K-12 MG1655
<220> tdcr b3120
<222> 241-340
<223> tdcr b3120 U00096 3264895..3265239
<313> 8448: 288-307
<400> 8448
TTTAAAATTT CGAATACATG TATTGATCAT CTCGAACAAT TGATTAACGT 50
CAACTTTTTC TCTTCTGACA GGACGTCATT TTGTGAATGC AATCGTTTTC 100
<210> 8449
<211> 100
<212> DNA
<213> Escherichia coli K-12 MG1655
<220> tdcr b3120
<222> 221-320
<223> tdcr b3120 U00096 3264895..3265239

<313> 8449: 278-297
<400> 8449
AACATTTATT TTATCAATAT TTTAAAATTT CGAATACATG TATTGATCAT 50
CTCGAACAAT TGATTAACGT CAACTTTTTC TCTTCTGACA GGACGTCATT 100
<210> 8450
<211> 100
<212> DNA
<213> Escherichia coli K-12 MG1655
<220> tdcr b3120
<222> 141-240
<223> tdcr b3120 U00096 3264895..3265239
<313> 8450: 195-214
<400> 8450
AAATTTTATT ATAAATAAAC CATTTTCAGT GATAAATAAT GCGGCATGTC 50
ACATTTTTTC ACGCTATTTG TTGGAGAACA AACATTTATT TTATCAATAT 100
<210> 8451
<211> 100
<212> DNA
<213> Escherichia coli K-12 MG1655
<220> nusa b3170
<222> 1389-1488
<223> nusa b3170 U00096 complement(3313680..3315167)
<313> 8451: 1450-1469
<400> 8451
CATTGATGAT CTGGCTGATA TCGAAGGGTT GACCGACGAA AAAGCCGGAG 50
CACTGATTAT GGCTGCCCGT AATATTTGCT GGTTCGGTGA CGAAGCGTAA 100
<210> 8452
<211> 100
<212> DNA
<213> Escherichia coli K-12 MG1655
<220> nusa b3170
<222> 1325-1424
<223> nusa b3170 U00096 complement(3313680..3315167)
<313> 8452: 1375-1394
<400> 8452
GTGATTTGGC ATTCAAACTG GCCGCCCGTG GCGTTTGTAC GCTGGAAGAT 50
CTCGCCGAAC AGGGCATTGA TGATCTGGCT GATATCGAAG GGTTGACCGA 100
<210> 8453
<211> 100
<212> DNA
<213> Escherichia coli K-12 MG1655
<220> nusa b3170
<222> 1235-1334
<223> nusa b3170 U00096 complement(3313680..3315167)
<313> 8453: 1293-1312
<400> 8453
ATGCACTGGC CACCATTGCA CAGGCCCAGG AAGAAAGCCT CGGTGATAAC 50
AAACCGGCTG ACGATCTGCT GAACCTTGAA GGGGTAGATC GTGATTTGGC 100
<210> 8454
<211> 100
<212> DNA
<213> Escherichia coli K-12 MG1655
<220> nusb b0417
<222> 321-420
<223> nusb b0417 U00096 434361..434780
<313> 8454: 397-416
<400> 8454
GATCGAACTG GCGAAATCGT TCGGCGCAGA AGACAGCCAT AAGTTCGTCA 50
ACGGCGTACT CGATAAAGCA GCACCTGTGA TTCGCCCTAA CAAAAAGTGA 100
<210> 8455
<211> 100
<212> DNA
<213> Escherichia coli K-12 MG1655
<220> nusb b0417
<222> 321-420
<223> nusb b0417 U00096 434361..434780
<313> 8455: 359-378
<400> 8455
GATCGAACTG GCGAAATCGT TCGGCGCAGA AGACAGCCAT AAGTTCGTCA 50
ACGGCGTACT CGATAAAGCA GCACCTGTGA TTCGCCCTAA CAAAAAGTGA 100
<210> 8456

<211> 100
<212> DNA
<213> Escherichia coli K-12 MG1655
<220> nusb b0417
<222> 237-336
<223> nusb b0417 U00096 434361..434780
<313> 8456: 275-294
<400> 8456
GGTAGAAAAA GCAGTACTGC GCATTGCGCT GTACGAACTG TCTAAACGTA 50
GCGATGTGCC ATACAAAGTG GCCATTAACG AAGCGATCGA ACTGGCGAAA 100
<210> 8457
<211> 100
<212> DNA
<213> Escherichia coli K-12 MG1655
<220> nusg b3983
<222> 358-457
<223> nusg b3983 U00096 4175322..4175867
<313> 8457: 411-430
<400> 8457
GATAAGCCGC GTCCGAAAAC GCTGTTTGAA CCGGGTGAAA TGGTCCGTGT 50
TAATGATGGT CCGTTCGCTG ACTTCAACGG TGTTGTTGAA GAAGTGGATT 100
<210> 8458
<211> 100
<212> DNA
<213> Escherichia coli K-12 MG1655
<220> nusg b3983
<222> 307-406
<223> nusg b3983 U00096 4175322..4175867
<313> 8458: 364-383
<400> 8458
ATCAGCGATA AAGAAGTCGA TGCGATTATG AACCGCCTGC AGCAGGTTGG 50
TGATAAGCCG CGTCCGAAAA CGCTGTTTGA ACCGGGTGAA ATGGTCCGTG 100
<210> 8459
<211> 100
<212> DNA
<213> Escherichia coli K-12 MG1655
<220> nusg b3983
<222> 291-390
<223> nusg b3983 U00096 4175322..4175867
<313> 8459: 319-338
<400> 8459
CGATCGTCCT GCGCCAATCA GCGATAAAGA AGTCGATGCG ATTATGAACC 50
GCCTGCAGCA GGTTGGTGAT AAGCCGCGTC CGAAAACGCT GTTTGAACCG 100
<210> 8460
<211> 100
<212> DNA
<213> Escherichia coli K-12 MG1655
<220> sgab b4195
<222> 202-301
<223> sgab b4195 U00096 4418971..4419276
<313> 8460: 249-268
<400> 8460
GGCAACAAAT ACGTGGTTGG CGTGCGCAAC ATGCTCTCTC CTGCCGACTT 50
TGGCCCGAAA CTGCTGGAAG TGATCAAAGA GCATTTCCCG CAGGATGTGA 100
<210> 8461
<211> 100
<212> DNA
<213> Escherichia coli K-12 MG1655
<220> sgab b4195
<222> 147-246
<223> sgab b4195 U00096 4418971..4419276
<313> 8461: 191-210
<400> 8461
TGGCGCGGAT ATCATCATCG CTTCTACGCA CATTGCGGGC GAAATCACCG 50
TGACCGGCAA CAAATACGTG GTTGGCGTGC GCAACATGCT CTCTCCTGCC 100
<210> 8462
<211> 100
<212> DNA
<213> Escherichia coli K-12 MG1655
<220> sgab b4195
<222> 117-216

<223> sgab b4195 U00096 4418971..4419276
<313> 8462: 144-163
<400> 8462
CGCGGTTGGC GAGTACAAAA GCGAGTTGAG TGGCGCGGAT ATCATCATCG 50
CTTCTACGCA CATTGCGGGC GAAATCACCG TGACCGGCAA CAAATACGTG 100
<210> 8463
<211> 100
<212> DNA
<213> Escherichia coli K-12 MG1655
<220> sgae b4199
<222> 588-687
<223> sgae b4199 U00096 4421278..4421964
<313> 8463: 661-680
<400> 8463
CCGCGGCATT AACCCACAAC TCAATCACAT CGACAGCTTC CTGATGAATA 50
AACACTTCAT GCGTAAACAC GGTCCTAACG CTTATTACGG GCAGAAGTAG 100
<210> 8464
<211> 100
<212> DNA
<213> Escherichia coli K-12 MG1655
<220> sgae b4199
<222> 574-673
<223> sgae b4199 U00096 4421278..4421964
<313> 8464: 607-626
<400> 8464
ATGGCGTGGA TTGCCCGCGG CATTAACCCA CAACTCAATC ACATCGACAG 50
CTTCCTGATG AATAAACACT TCATGCGTAA ACACGGTCCT AACGCTTATT 100
<210> 8465
<211> 100
<212> DNA
<213> Escherichia coli K-12 MG1655
<220> sgae b4199
<222> 526-625
<223> sgae b4199 U00096 4421278..4421964
<313> 8465: 556-575
<400> 8465
GCTCACGATG CGGTGCATAA CGCGGTGGTG ATGGAAGAAG TGGCGAAAAT 50
GGCGTGGATT GCCCGCGGCA TTAACCCACA ACTCAATCAC ATCGACAGCT 100
<210> 8466
<211> 100
<212> DNA
<213> Escherichia coli K-12 MG1655
<220> sgah b4197
<222> 552-651
<223> sgah b4197 U00096 4419764..4420414
<313> 8466: 595-614
<400> 8466
GATTCACGTC TTTATCGCGG GCCGTAGTAT CCGTGATGCC GCTTCTCCGG 50
TGGAAGCCGC ACGTCAGTTC AAACGTTCCA TCGCTGAACT GTGGGGCTAA 100
<210> 8467
<211> 100
<212> DNA
<213> Escherichia coli K-12 MG1655
<220> sgah b4197
<222> 503-602
<223> sgah b4197 U00096 4419764..4420414
<313> 8467: 558-577
<400> 8467
TCACCGGAGG CCTGGCGCTG GAAGATCTGC CGCTGTTCAA GGGTATTCCG 50
ATTCACGTCT TTATCGCGGG CCGTAGTATC CGTGATGCCG CTTCTCCGGT 100
<210> 8468
<211> 100
<212> DNA
<213> Escherichia coli K-12 MG1655
<220> sgah b4197
<222> 435-534
<223> sgah b4197 U00096 4419764..4420414
<313> 8468: 476-495
<400> 8468
CGTGGCGTGG GGCGAAGCGG ACATCACCGC GATCAAACGT CTTTCCGATA 50
TGGGCTTCAA AGTCACCGTC ACCGGAGGCC TGGCGCTGGA AGATCTGCCG 100

<210> 8469
<211> 100
<212> DNA
<213> Escherichia coli K-12 MG1655
<220> b1010 b1010
<222> 248-347
<223> b1010 b1010 U00096 complement(1070996..1071382)
<313> 8469: 295-314
<400> 8469
GGAAAAATTA CGCCGCGATT AACGAAATCT ACGCCGAGTT TTTTCCGGGT  50
GATAAACCGG CGCGATTCTG CATTCAGTGC GGACTGGTAA AACCTGACGC  100
<210> 8470
<211> 100
<212> DNA
<213> Escherichia coli K-12 MG1655
<220> b1010 b1010
<222> 200-299
<223> b1010 b1010 U00096 complement(1070996..1071382)
<313> 8470: 250-269
<400> 8470
GTGGCACGAT GGCGGATGTG ACCTTCAACA GCATCTTTAT TACCGACTGG  50
AAAAATTACG CCGCGATTAA CGAAATCTAC GCCGAGTTTT TTCCGGGTGA  100
<210> 8471
<211> 100
<212> DNA
<213> Escherichia coli K-12 MG1655
<220> b1010 b1010
<222> 171-270
<223> b1010 b1010 U00096 complement(1070996..1071382)
<313> 8471: 213-232
<400> 8471
AACTATCCGC AAGGTGATCG AGACGGCGGG TGGCACGATG GCGGATGTGA  50
CCTTCAACAG CATCTTTATT ACCGACTGGA AAAATTACGC CGCGATTAAC  100
<210> 8472
<211> 100
<212> DNA
<213> Escherichia coli K-12 MG1655
<220> b1011 b1011
<222> 636-735
<223> b1011 b1011 U00096 complement(1071394..1072128)
<313> 8472: 702-721
<400> 8472
AGCCGCGTTG TTCAATATCG AAACCTTTTT TGGCTGGGTC AGCGACGTCG  50
AAACATTCTG CGACGCGCTT TCTCCCACGT CCTTTGCTCA TATCGCTTAA  100
<210> 8473
<211> 100
<212> DNA
<213> Escherichia coli K-12 MG1655
<220> b1011 b1011
<222> 613-712
<223> b1011 b1011 U00096 complement(1071394..1072128)
<313> 8473: 652-671
<400> 8473
GCGGGGCCGA AATTTGCGCA GAAAGCCGCG TTGTTCAATA TCGAAACCTT  50
TTTTGGCTGG GTCAGCGACG TCGAAACATT CTGCGACGCG CTTTCTCCCA  100
<210> 8474
<211> 100
<212> DNA
<213> Escherichia coli K-12 MG1655
<220> b1011 b1011
<222> 554-653
<223> b1011 b1011 U00096 complement(1071394..1072128)
<313> 8474: 608-627
<400> 8474
TACGCGACGG CTTTTTTCTG GAGTATTTCG GCGTGGTGCT TGAAGACGCA  50
ACTCACCAGG CGGGGCCGAA ATTTGCGCAG AAAGCCGCGT TGTTCAATAT  100
<210> 8475
<211> 100
<212> DNA
<213> Escherichia coli K-12 MG1655
<220> b1012 b1013

<222> 1050-1149
<223> b1012 b1013 U00096 complement(1072086..1073234)
<313> 8475: 1119-1138
<400> 8475
CGACGATTTT CTGTCGGGAA TCGAAACCTT CGGCGAGCGC ATTCAACCAC 50
TGATGCAATG CCGCGCCCAT CTACCTGCGC TGACTCAGGA GGTGGCATGA 100
<210> 8476
<211> 100
<212> DNA
<213> Escherichia coli K-12 MG1655
<220> b1012 b1013
<222> 1044-1143
<223> b1012 b1013 U00096 complement(1072086..1073234)
<313> 8476: 1073-1092
<400> 8476
AACCTTCGAC GATTTTCTGT CGGGAATCGA AACCTTCGGC GAGCGCATTC 50
AACCACTGAT GCAATGCCGC GCCCATCTAC CTGCGCTGAC TCAGGAGGTG 100
<210> 8477
<211> 100
<212> DNA
<213> Escherichia coli K-12 MG1655
<220> b1012 b1013
<222> 915-1014
<223> b1012 b1013 U00096 complement(1072086..1073234)
<313> 8477: 970-989
<400> 8477
CGTTCGTCAG ATGGCCGATC CCACTTCGGC GGTAAACATC AATATGGGGA 50
CGTTAGTCGG TTCTTACGCC AGTGTCGCGC GCATGTTAGA TGAAGTCGCA 100
<210> 8478
<211> 100
<212> DNA
<213> Escherichia coli K-12 MG1655
<220> nfra b0569
<222> 2830-2929
<223> nfra b0569 U00096 complement(587205..590177)
<313> 8478: 2862-2881
<400> 8478
AATATCTGGA CCGGCGAGAC GCACTACGAC GCCTGGCCGC ACAAAGTCAG 50
TCTCGGCGTC GAGTATCAAC ATACCTTTAA GGCGATTAAT CAACGTAACG 100
<210> 8479
<211> 100
<212> DNA
<213> Escherichia coli K-12 MG1655
<220> nfra b0569
<222> 2725-2824
<223> nfra b0569 U00096 complement(587205..590177)
<313> 8479: 2753-2772
<400> 8479
GTAGCTAACG GACAGACTAT TGAGCCTTAC GCTCACGTTC AGGACAACGG 50
CTATCGTGAT AAAGGCACTC AGGGCGCGCA GCTTGGCGGA GTCGGGGTCC 100
<210> 8480
<211> 100
<212> DNA
<213> Escherichia coli K-12 MG1655
<220> nfra b0569
<222> 2628-2727
<223> nfra b0569 U00096 complement(587205..590177)
<313> 8480: 2645-2664
<400> 8480
CTGGTTTGCC CAAAACCTGT ACCTCGATGC GGCGCAATAT ATCCGCCAGG 50
ATATTCAGGC GTGGACGGCA GATTATCGCG TCAGCTGGCA TCAGAAGGTA 100
<210> 8481
<211> 100
<212> DNA
<213> Escherichia coli K-12 MG1655
<220> mioc b3743
<222> 343-442
<223> mioc b3743 U00096 complement(3923640..3924083)
<313> 8481: 401-420
<400> 8481
AAACAGACAG GCGAAACACT GAAGATCAAC ATTCTTGATC ACGACATTCC 50

GGAAGATCCG GCAGAAGAAT GGCTGGGATC GTGGGTTAAT TTACTCAAAT 100
<210> 8482
<211> 100
<212> DNA
<213> Escherichia coli K-12 MG1655
<220> mioc b3743
<222> 323-422
<223> mioc b3743 U00096 complement(3923640..3924083)
<313> 8482: 362-381
<400> 8482
AACTCAAAAA TTCCGGTGCA AAACAGACAG GCGAAACACT GAAGATCAAC 50
ATTCTTGATC ACGACATTCC GGAAGATCCG GCAGAAGAAT GGCTGGGATC 100
<210> 8483
<211> 100
<212> DNA
<213> Escherichia coli K-12 MG1655
<220> mioc b3743
<222> 254-353
<223> mioc b3743 U00096 complement(3923640..3924083)
<313> 8483: 284-303
<400> 8483
TTGGCGCAAT CGGTATTGGC AGTCGTGAAT ATGACACCTT TTGTGGGGCT 50
ATCGATAAAC TCGAGGCAGA ACTCAAAAAT TCCGGTGCAA AACAGACAGG 100
<210> 8484
<211> 100
<212> DNA
<213> Escherichia coli K-12 MG1655
<220> nfrb b0570
<222> 2086-2185
<223> nfrb b0570 U00096 complement(590164..592401)
<313> 8484: 2140-2159
<400> 8484
TTGGTCACCG AAGGCGTTAT CAGCCAGGAA ACGTTGGATC GCGTCCTGAC 50
AATTCAACGC GAATTACAAG TTTCGATGCA ATCACTATTA CTCAAAGCAG 100
<210> 8485
<211> 100
<212> DNA
<213> Escherichia coli K-12 MG1655
<220> nfrb b0570
<222> 2073-2172
<223> nfrb b0570 U00096 complement(590164..592401)
<313> 8485: 2099-2118
<400> 8485
GCTCGGCAAG TTTTTGGTCA CCGAAGGCGT TATCAGCCAG GAAACGTTGG 50
ATCGCGTCCT GACAATTCAA CGCGAATTAC AAGTTTCGAT GCAATCACTA 100
<210> 8486
<211> 100
<212> DNA
<213> Escherichia coli K-12 MG1655
<220> nfrb b0570
<222> 2010-2109
<223> nfrb b0570 U00096 complement(590164..592401)
<313> 8486: 2046-2065
<400> 8486
CGGTCATATT AATCGTTCAG CAATTAACGT GTTGTTATTG CGCCATGAAC 50
GCAGTTCTCT GCCGCTCGGC AAGTTTTTGG TCACCGAAGG CGTTATCAGC 100
<210> 8487
<211> 100
<212> DNA
<213> Escherichia coli K-12 MG1655
<220> b1016 b1016
<222> 21-120
<223> b1016 b1016 U00096 1080570..1080689
<313> 8487: 98-117
<400> 8487
GTTTCTCATT ATGTTGCGCG AAGGACTTGA AGCCGCGCTG ATTGTCAGTT 50
TGATTGCCAG CTATCTTAAG CGTACCCAGC GAGGCCGATG GATTGTGTGA 100
<210> 8488
<211> 100
<212> DNA
<213> Escherichia coli K-12 MG1655

<220> b1016 b1016
<222> 21-120
<223> b1016 b1016 U00096 1080570..1080689
<313> 8488: 72-91
<400> 8488
GTTTCTCATT ATGTTGCGCG AAGGACTTGA AGCCGCGCTG ATTGTCAGTT 50
TGATTGCCAG CTATCTTAAG CGTACCCAGC GAGGCCGATG GATTGTGTGA 100
<210> 8489
<211> 100
<212> DNA
<213> Escherichia coli K-12 MG1655
<220> b1016 b1016
<222> 21-120
<223> b1016 b1016 U00096 1080570..1080689
<313> 8489: 79-98
<400> 8489
GTTTCTCATT ATGTTGCGCG AAGGACTTGA AGCCGCGCTG ATTGTCAGTT 50
TGATTGCCAG CTATCTTAAG CGTACCCAGC GAGGCCGATG GATTGTGTGA 100
<210> 8490
<211> 100
<212> DNA
<213> Escherichia coli K-12 MG1655
<220> cfa b1662
<222> 1050-1149
<223> cfa b1662 U00096 1739437..1740585
<313> 8490: 1126-1145
<400> 8490
TCTGAATGCC TGTGCAGGTG CTTTCCGCGC CCGTGATATT CAGCTCTGGC 50
AGGTCGTGTT CTCACGCGGT GTTGAAAACG GCCTTCGAGT GGCTCGCTAA 100
<210> 8491
<211> 100
<212> DNA
<213> Escherichia coli K-12 MG1655
<220> cfa b1662
<222> 1041-1140
<223> cfa b1662 U00096 1739437..1740585
<313> 8491: 1081-1100
<400> 8491
TACCTATTAT CTGAATGCCT GTGCAGGTGC TTTCCGCGCC CGTGATATTC 50
AGCTCTGGCA GGTCGTGTTC TCACGCGGTG TTGAAAACGG CCTTCGAGTG 100
<210> 8492
<211> 100
<212> DNA
<213> Escherichia coli K-12 MG1655
<220> cfa b1662
<222> 938-1037
<223> cfa b1662 U00096 1739437..1740585
<313> 8492: 960-979
<400> 8492
TCGGTGCTGA TTACGATACT ACGTTGATGG CGTGGTATGA ACGATTCCTC 50
GCCGCATGGC CAGAAATTGC GGATAACTAT AGTGAACGCT TTAAACGAAT 100
<210> 8493
<211> 100
<212> DNA
<213> Escherichia coli K-12 MG1655
<220> ylea b0662
<222> 1326-1425
<223> ylea b0662 U00096 complement(692754..694178)
<313> 8493: 1402-1421
<400> 8493
TGAAGATGAA ATGGGTCTGC GCGTGGCAGA AACACCGGAA TCAGTGATTG 50
CCCGTACCCG CAAAGAAAAC GACCTTGGCG TGGGTTATTA TCAGCCGTAA 100
<210> 8494
<211> 100
<212> DNA
<213> Escherichia coli K-12 MG1655
<220> ylea b0662
<222> 1320-1419
<223> ylea b0662 U00096 complement(692754..694178)
<313> 8494: 1360-1379
<400> 8494

GCGTACTGAA GATGAAATGG GTCTGCGCGT GGCAGAAACA CCGGAATCAG 50
TGATTGCCCG TACCCGCAAA GAAAACGACC TTGGCGTGGG TTATTATCAG 100
<210> 8495
<211> 100
<212> DNA
<213> Escherichia coli K-12 MG1655
<220> ylea b0662
<222> 1147-1246
<223> ylea b0662 U00096 complement(692754..694178)
<313> 8495: 1190-1209
<400> 8495
ACCACCCAGC GTATTCTGGT AGAAGGTACA TCGCGTAAGA GCATCATGGA 50
GCTTTCCGGG CGTACGGAAA ATAACCGCGT GGTCAACTTC GAAGGCACGC 100
<210> 8496
<211> 100
<212> DNA
<213> Escherichia coli K-12 MG1655
<220> b1017 b1018
<222> 605-704
<223> b1017 b1018 U00096 1080677..1081408
<313> 8496: 653-672
<400> 8496
GGATTTTTGG CTATCAGGAA GCGCCGAGCG TCAGCGAAGT CGCCGTCTGG 50
TTTATTTATC TCATCCCGGC GCTGGTGGCA TTTGCTCTGC CACCACGCGC 100
<210> 8497
<211> 100
<212> DNA
<213> Escherichia coli K-12 MG1655
<220> b1017 b1018
<222> 539-638
<223> b1017 b1018 U00096 1080677..1081408
<313> 8497: 590-609
<400> 8497
AGGAAATCGC CTTCGATATG AGTGCGGTGC TCTCAACTCA CTCGCTGTTT 50
GGCACGCTGA TGGAAGGGAT TTTTGGCTAT CAGGAAGCGC CGAGCGTCAG 100
<210> 8498
<211> 100
<212> DNA
<213> Escherichia coli K-12 MG1655
<220> b1017 b1018
<222> 494-593
<223> b1017 b1018 U00096 1080677..1081408
<313> 8498: 555-574
<400> 8498
GTGCCATTCG CGCATTTCAT GAAGCCGGAT TGTGGAACCA CTTTCAGGAA 50
ATCGCCTTCG ATATGAGTGC GGTGCTCTCA ACTCACTCGC TGTTTGGCAC 100
<210> 8499
<211> 100
<212> DNA
<213> Escherichia coli K-12 MG1655
<220> yleb yleB
<222> 1077-1176
<223> yleb yleB U00096 694324..695499
<313> 8499: 1139-1158
<400> 8499
TCTGCCACCA CTGCGTTTTA TGCGTAATCT CGGGTTAATG GCGGCGGAGC 50
GTGCTGGCGT GTTGAAACGT CAGGCGCTGA AATATGCGTT AGGGTTGTAG 100
<210> 8500
<211> 100
<212> DNA
<213> Escherichia coli K-12 MG1655
<220> yleb yleB
<222> 1038-1137
<223> yleb yleB U00096 694324..695499
<313> 8500: 1067-1086
<400> 8500
AAGCGGTATG GATCTGTTTT ATGCCGGATT CAGCAATAAT CTGCCACCAC 50
TGCGTTTTAT GCGTAATCTC GGGTTAATGG CGGCGGAGCG TGCTGGCGTG 100
<210> 8501
<211> 100
<212> DNA

<213> Escherichia coli K-12 MG1655
<220> yleb yleB
<222> 970-1069
<223> yleb yleB U00096 694324..695499
<313> 8501: 1021-1040
<400> 8501
GCGTGGGCCA GTTATCCTGT CCTCAAGCGT TACCAGATGC GGCGCATGGC 50
GGATAACTTC ATTATGCAAA GCGGTATGGA TCTGTTTTAT GCCGGATTCA 100
<210> 8502
<211> 100
<212> DNA
<213> Escherichia coli K-12 MG1655
<220> sgat b4194
<222> 1356-1455
<223> sgat b4194 U00096 4417501..4418955
<313> 8502: 1413-1432
<400> 8502
CGTCATCATT GTAATTGCAC TGGCTTATAT GTTCTTCGCT GGCCGCGCGC 50
TGCGCGCAGA AGAAGATGCA GAAAAACAAC TGGCAGAACA GTCTGCTTAA 100
<210> 8503
<211> 100
<212> DNA
<213> Escherichia coli K-12 MG1655
<220> sgat b4194
<222> 1286-1385
<223> sgat b4194 U00096 4417501..4418955
<313> 8503: 1313-1332
<400> 8503
TGGCGGACTG GTCGATTCTG GCACCGCCGA TGATGCAAGG CTTCTTCTCC 50
ATCGGTATCG CCTTTATGGC CGTCATCATT GTAATTGCAC TGGCTTATAT 100
<210> 8504
<211> 100
<212> DNA
<213> Escherichia coli K-12 MG1655
<220> sgat b4194
<222> 1174-1273
<223> sgat b4194 U00096 4417501..4418955
<313> 8504: 1212-1231
<400> 8504
CACTTCGGCG GCTGGCGTGC GGCGCTGAAG ATTTGTCTGG TGATGGGGAT 50
GATCGAAATC TTTGGTTGCG TCTGGGCGGT GAAACTCACC GGTATGAGTG 100
<210> 8505
<211> 100
<212> DNA
<213> Escherichia coli K-12 MG1655
<220> sgau b4198
<222> 745-844
<223> sgau b4198 U00096 4420424..4421278
<313> 8505: 791-810
<400> 8505
CTGATTGAGA TGTGGAGCGA AACGGCGGAA GACCCGGCGG CAGAAGTGGC 50
GAAAGCGCGT GATTGGGTGA AAGCGCGCAT GGCGAAAGCG GGCATGGTGG 100
<210> 8506
<211> 100
<212> DNA
<213> Escherichia coli K-12 MG1655
<220> sgau b4198
<222> 693-792
<223> sgau b4198 U00096 4420424..4421278
<313> 8506: 740-759
<400> 8506
TTTCGAACGT TGTTTCGAAA CGCTCAAACA GAGTGGCTAT TGCGGGCCGT 50
ACCTGATTGA GATGTGGAGC GAAACGGCGG AAGACCCGGC GGCAGAAGTG 100
<210> 8507
<211> 100
<212> DNA
<213> Escherichia coli K-12 MG1655
<220> sgau b4198
<222> 638-737
<223> sgau b4198 U00096 4420424..4421278
<313> 8507: 666-685

<400> 8507
AAGACACCAA ACCTGGCGTC TTCAAAAACG TGCCGTTTGG CGAAGGTGTA 50
GTGGATTTCG AACGTTGTTT CGAAACGCTC AAACAGAGTG GCTATTGCGG 100
<210> 8508
<211> 100
<212> DNA
<213> Escherichia coli K-12 MG1655
<220> chpa b2783
<222> 237-336
<223> chpa b2783 U00096 complement(2908778..2909113)
<313> 8508: 279-298
<400> 8508
AAGTATCGCC TGGCGGGCAA GAGGAGCAAC GAAGAAAGGA ACAGTTGCCC 50
CAGAGGAATT ACAACTCATT AAAGCCAAAA TTAACGTACT GATTGGGTAG 100
<210> 8509
<211> 100
<212> DNA
<213> Escherichia coli K-12 MG1655
<220> chpa b2783
<222> 210-309
<223> chpa b2783 U00096 complement(2908778..2909113)
<313> 8509: 252-271
<400> 8509
TGGCGTAGCG TTAGCTGATC AGGTAAAAAG TATCGCCTGG CGGGCAAGAG 50
GAGCAACGAA GAAAGGAACA GTTGCCCCAG AGGAATTACA ACTCATTAAA 100
<210> 8510
<211> 100
<212> DNA
<213> Escherichia coli K-12 MG1655
<220> chpa b2783
<222> 151-250
<223> chpa b2783 U00096 complement(2908778..2909113)
<313> 8510: 183-202
<400> 8510
TGTACAACGC AATCAAAAGG ATATCCGTTC GAAGTTGTTT TATCCGGTCA 50
GGAACGTGAT GGCGTAGCGT TAGCTGATCA GGTAAAAAGT ATCGCCTGGC 100
<210> 8511
<211> 100
<212> DNA
<213> Escherichia coli K-12 MG1655
<220> chpb b4226
<222> 252-351
<223> chpb b4226 U00096 4446269..4446619
<313> 8511: 300-319
<400> 8511
GATGATGGAT CTACACGCCC GGCTGGCAAA GCGTATTGGT CTGGCTGCGG 50
ATGAGGTGGT GGAAGAGGCG TTATTACGCT TGCAGGCGGT GGTGGAATAA 100
<210> 8512
<211> 100
<212> DNA
<213> Escherichia coli K-12 MG1655
<220> chpb b4226
<222> 178-277
<223> chpb b4226 U00096 4446269..4446619
<313> 8512: 230-249
<400> 8512
TATGCCGGAT TTAGCGTTCC TTTACATTGC GAAGAAGGCG ATGTGCACGG 50
CGTGGTGCTG GTGAATCAGG TGCGGATGAT GGATCTACAC GCCCGGCTGG 100
<210> 8513
<211> 100
<212> DNA
<213> Escherichia coli K-12 MG1655
<220> chpb b4226
<222> 158-257
<223> chpb b4226 U00096 4446269..4446619
<313> 8513: 179-198
<400> 8513
AGGGCGGAAA TTTTGCCCGT TATGCCGGAT TTAGCGTTCC TTTACATTGC 50
GAAGAAGGCG ATGTGCACGG CGTGGTGCTG GTGAATCAGG TGCGGATGAT 100
<210> 8514
<211> 100

<212> DNA
<213> Escherichia coli K-12 MG1655
<220> sgbe b3584
<222> 597-696
<223> sgbe b3584 U00096 3747716..3748411
<313> 8514: 675-694
<400> 8514
CTCGCGCCAG CTTGCGCCGC AGCTCCCTGC GATGCAAAAC GAACTGCTGG 50
ATAAGCACTA CCTGCGTAAG CATGGGGCCA ATGCCTATTA CGGGCAGTAA 100
<210> 8515
<211> 100
<212> DNA
<213> Escherichia coli K-12 MG1655
<220> sgbe b3584
<222> 588-687
<223> sgbe b3584 U00096 3747716..3748411
<313> 8515: 622-641
<400> 8515
GGGTCTATTC TCGCGCCAGC TTGCGCCGCA GCTCCCTGCG ATGCAAAACG 50
AACTGCTGGA TAAGCACTAC CTGCGTAAGC ATGGGGCCAA TGCCTATTAC 100
<210> 8516
<211> 100
<212> DNA
<213> Escherichia coli K-12 MG1655
<220> sgbe b3584
<222> 525-624
<223> sgbe b3584 U00096 3747716..3748411
<313> 8516: 568-587
<400> 8516
ATGGGGTAAA AACGCCGCCG ATGCCGTGCA TAACGCCGTA GTACTCGAAG 50
AATGCGCCTA TATGGGTCTA TTCTCGCGCC AGCTTGCGCC GCAGCTCCCT 100
<210> 8517
<211> 100
<212> DNA
<213> Escherichia coli K-12 MG1655
<220> sgbh b3582
<222> 564-663
<223> sgbh b3582 U00096 3746207..3746869
<313> 8517: 592-611
<400> 8517
TATTGCCGGG CGGGCACTGG CAGGCGCGGC AAATCCGGCG CAAGTGGCTG 50
GCGATTTCCA TGCGCAAATC GACGCTATCT GGGGAGGTGC GCGTGCGTAA 100
<210> 8518
<211> 100
<212> DNA
<213> Escherichia coli K-12 MG1655
<220> sgbh b3582
<222> 504-603
<223> sgbh b3582 U00096 3746207..3746869
<313> 8518: 536-555
<400> 8518
TACTGGCGGG ATTACCCCTG CTGACCTGCC GCTGTTTAAA GATATCCGCG 50
TGAAAGCGTT TATTGCCGGG CGGGCACTGG CAGGCGCGGC AAATCCGGCG 100
<210> 8519
<211> 100
<212> DNA
<213> Escherichia coli K-12 MG1655
<220> sgbh b3582
<222> 460-559
<223> sgbh b3582 U00096 3746207..3746869
<313> 8519: 496-515
<400> 8519
GCACGCATGA AGGCGCTTTC AGATATCGGC CTTGAGCTTT CCATTACTGG 50
CGGGATTACC CCTGCTGACC TGCCGCTGTT TAAAGATATC CGCGTGAAAG 100
<210> 8520
<211> 100
<212> DNA
<213> Escherichia coli K-12 MG1655
<220> mipb b0826
<222> 636-735
<223> mipb b0826 U00096 862793..863527

<313> 8520: 702-721
<400> 8520
GGATGTGGCA CAACAGATGA TTAGCTATCC GGCGGTTGAT GCCGCTGTGG 50
CGAAGTTTGA GCAGGACTGG CAGGGAGCGT TTGGCAGAAC GTCGATTTAA 100
<210> 8521
<211> 100
<212> DNA
<213> Escherichia coli K-12 MG1655
<220> mipb b0826
<222> 589-688
<223> mipb b0826 U00096 862793..863527
<313> 8521: 643-662
<400> 8521
GCGCTGGACT GCTTACTGGC AGGATGTGAA TCAATTACTC TGCCACTGGA 50
TGTGGCACAA CAGATGATTA GCTATCCGGC GGTTGATGCC GCTGTGGCGA 100
<210> 8522
<211> 100
<212> DNA
<213> Escherichia coli K-12 MG1655
<220> mipb b0826
<222> 564-663
<223> mipb b0826 U00096 862793..863527
<313> 8522: 604-623
<400> 8522
AGCGAGTTTC AAAACCCCGC GTCAGGCGCT GGACTGCTTA CTGGCAGGAT 50
GTGAATCAAT TACTCTGCCA CTGGATGTGG CACAACAGAT GATTAGCTAT 100
<210> 8523
<211> 100
<212> DNA
<213> Escherichia coli K-12 MG1655
<220> rho b3784
<222> 1131-1230
<223> rho b3784 U00096 3964032..3965291
<313> 8523: 1160-1179
<400> 8523
GCAGAAAATG TGGATCCTGC GCAAAATCAT TCACCCGATG GGCGAAATCG 50
ATGCAATGGA ATTCCTCATT AATAAACTGG CAATGACCAA GACCAATGAC 100
<210> 8524
<211> 100
<212> DNA
<213> Escherichia coli K-12 MG1655
<220> rho b3784
<222> 1069-1168
<223> rho b3784 U00096 3964032..3965291
<313> 8524: 1096-1115
<400> 8524
GCTATCGACT ACAACCGTTC TGGTACCCGT AAAGAAGAGC TGCTCACGAC 50
TCAGGAAGAA CTGCAGAAAA TGTGGATCCT GCGCAAAATC ATTCACCCGA 100
<210> 8525
<211> 100
<212> DNA
<213> Escherichia coli K-12 MG1655
<220> rho b3784
<222> 999-1098
<223> rho b3784 U00096 3964032..3965291
<313> 8525: 1029-1048
<400> 8525
AGAGTTTAAA GGTACAGGCA ACATGGAACT GCACCTCTCT CGTAAGATCG 50
CTGAAAAACG CGTCTTCCCG GCTATCGACT ACAACCGTTC TGGTACCCGT 100
<210> 8526
<211> 100
<212> DNA
<213> Escherichia coli K-12 MG1655
<220> b1027 b1027
<222> 125-224
<223> b1027 b1027 U00096 complement(1094361..1094669)
<313> 8526: 161-180
<400> 8526
TCAGCCTGTA TGAATCACAA CTCTACAACT GGCGCAGTAA ACAGCAAAAT 50
CAGCAGACGT CTTCTGAACG TGAACTGGAG ATGTCTACCG AGATTGCACG 100
<210> 8527

<211> 100
<212> DNA
<213> Escherichia coli K-12 MG1655
<220> b1027 b1027
<222> 87-186
<223> b1027 b1027 U00096 complement(1094361..1094669)
<313> 8527: 121-140
<400> 8527
TGCTGAACGC ATCGGTGTTA CTGCCGCAGC CCGTGAACTC AGCCTGTATG 50
AATCACAACT CTACAACTGG CGCAGTAAAC AGCAAAATCA GCAGACGTCT 100
<210> 8528
<211> 100
<212> DNA
<213> Escherichia coli K-12 MG1655
<220> b1027 b1027
<222> 26-125
<223> b1027 b1027 U00096 complement(1094361..1094669)
<313> 8528: 78-97
<400> 8528
CAACCAGTAA AAAACCCCGT AAACAGCATT CGCCTGAATT TCGCAGTGAA 50
GCCCTGAAGC TTGCTGAACG CATCGGTGTT ACTGCCGCAG CCCGTGAACT 100
<210> 8529
<211> 100
<212> DNA
<213> Escherichia coli K-12 MG1655
<220> b1028 b1029
<222> 225-324
<223> b1028 b1029 U00096 1094746..1095069
<313> 8529: 276-295
<400> 8529
GCCCTATTTG GGGAATATGT TAAATGATTT TGCTGGCGTG GATCAGCAAC 50
GAGTTATGCA TTGTCGTAAA GCATTTAGTG ATGCAAAGGT ATTCAAATGA 100
<210> 8530
<211> 100
<212> DNA
<213> Escherichia coli K-12 MG1655
<220> b1028 b1029
<222> 169-268
<223> b1028 b1029 U00096 1094746..1095069
<313> 8530: 208-227
<400> 8530
CTCGATACAT TTCTGAATAT GCCAACCCCG GTAGCTTTCC TTGAAATAAG 50
TAATAAGCCC TATTTGGGGA ATATGTTAAA TGATTTTGCT GGCGTGGATC 100
<210> 8531
<211> 100
<212> DNA
<213> Escherichia coli K-12 MG1655
<220> b1028 b1029
<222> 43-142
<223> b1028 b1029 U00096 1094746..1095069
<313> 8531: 93-112
<400> 8531
CTGGATATCG CTTCGCCAGA GCATCAGGTT CTGATGAACG TAACAGCAAA 50
AAGCGAGGTC GCTCCGTCAA TTATCAAAGA AAATCTCTCA TTACACTTAA 100
<210> 8532
<211> 100
<212> DNA
<213> Escherichia coli K-12 MG1655
<220> chpr b2784
<222> 150-249
<223> chpr b2784 U00096 complement(2909113..2909361)
<313> 8532: 222-241
<400> 8532
GCCCGTATTT ACGCTTGCTG AACTGGTCAA CGACATCACG CCGGAAAACC 50
TCCACGAGAA TATCGACTGG GGAGAGCCGA AAGATAAGGA AGTCTGGTAA 100
<210> 8533
<211> 100
<212> DNA
<213> Escherichia coli K-12 MG1655
<220> chpr b2784
<222> 150-249

<223> chpr b2784 U00096 complement(2909113..2909361)
<313> 8533: 173-192
<400> 8533
GCCCGTATTT ACGCTTGCTG AACTGGTCAA CGACATCACG CCGGAAAACC 50
TCCACGAGAA TATCGACTGG GGAGAGCCGA AAGATAAGGA AGTCTGGTAA 100
<210> 8534
<211> 100
<212> DNA
<213> Escherichia coli K-12 MG1655
<220> chpr b2784
<222> 1-100
<223> chpr b2784 U00096 complement(2909113..2909361)
<313> 8534: 28-47
<400> 8534
ATGATCCACA GTAGCGTAAA GCGTTGGGGA AATTCACCGG CGGTGCGGAT 50
CCCGGCTACG TTAATGCAGG CGCTCAATCT GAATATTGAT GATGAAGTGA 100
<210> 8535
<211> 100
<212> DNA
<213> Escherichia coli K-12 MG1655
<220> chps b4225
<222> 159-258
<223> chps b4225 U00096 4446018..4446275
<313> 8535: 214-233
<400> 8535
GCTTGATGAA CTGCTGGCAC AGTGTGACAT GAACGCCGCG GAACTTAGCG 50
AGCAGGATGT CTGGGGTAAA TCCACCCCTG CGGGTGACGA AATATGGTAA 100
<210> 8536
<211> 100
<212> DNA
<213> Escherichia coli K-12 MG1655
<220> chps b4225
<222> 90-189
<223> chps b4225 U00096 4446018..4446275
<313> 8536: 128-147
<400> 8536
GGGGCAGAGC GTGGAGGCGC AAGTGAGCAA CAATCAACTG ATTCTGACAC 50
CCATCTCCAG GCGCTACTCG CTTGATGAAC TGCTGGCACA GTGTGACATG 100
<210> 8537
<211> 100
<212> DNA
<213> Escherichia coli K-12 MG1655
<220> chps b4225
<222> 31-130
<223> chps b4225 U00096 4446018..4446275
<313> 8537: 90-109
<400> 8537
GGGAACAGTG CAGGTATGGT CATTCCCAAT ATCGTAATGA AAGAACTTAA 50
CTTACAGCCG GGGCAGAGCG TGGAGGCGCA AGTGAGCAAC AATCAACTGA 100
<210> 8538
<211> 100
<212> DNA
<213> Escherichia coli K-12 MG1655
<220> sgbu b3583
<222> 762-861
<223> sgbu b3583 U00096 3746829..3747722
<313> 8538: 794-813
<400> 8538
GCATAAACTG AACTATCGCG GTTCTTTCCT GATTGAGATG TGGACCGAAA 50
AAGCCAAAGA GCCGGTGCTG GAGATTATTC AGGCGCGGCG TTGGATTGAA 100
<210> 8539
<211> 100
<212> DNA
<213> Escherichia coli K-12 MG1655
<220> sgbu b3583
<222> 721-820
<223> sgbu b3583 U00096 3746829..3747722
<313> 8539: 742-761
<400> 8539
GGCGAAGGCT GCGTCGATTT CGTTGGCATC TTCAAAACGC TGCATAAACT 50
GAACTATCGC GGTTCTTTCC TGATTGAGAT GTGGACCGAA AAAGCCAAAG 100

<210> 8540
<211> 100
<212> DNA
<213> Escherichia coli K-12 MG1655
<220> sgbu b3583
<222> 681-780
<223> sgbu b3583 U00096 3746829..3747722
<313> 8540: 694-713
<400> 8540
CACCGGGCAA AGTCCCGGAC AGTTCCGCGA CGTGCCGTTT GGCGAAGGCT  50
GCGTCGATTT CGTTGGCATC TTCAAAACGC TGCATAAACT GAACTATCGC  100
<210> 8541
<211> 100
<212> DNA
<213> Escherichia coli K-12 MG1655
<220> sgca b4303
<222> 333-432
<223> sgca b4303 U00096 complement(4525117..4525548)
<313> 8541: 377-396
<400> 8541
CATTCAGCGC ATCAGCCAGT TTATTGATTC GCCACAACGC TTAACGGCAG  50
TGGGAAATGC CAGCACAGAC GATGCACTGT TTGCGCTGGT ATCAGGTTAA  100
<210> 8542
<211> 100
<212> DNA
<213> Escherichia coli K-12 MG1655
<220> sgca b4303
<222> 319-418
<223> sgca b4303 U00096 complement(4525117..4525548)
<313> 8542: 347-366
<400> 8542
GCACATATTT TGACCATTCA GCGCATCAGC CAGTTTATTG ATTCGCCACA  50
ACGCTTAACG GCAGTGGGAA ATGCCAGCAC AGACGATGCA CTGTTTGCGC  100
<210> 8543
<211> 100
<212> DNA
<213> Escherichia coli K-12 MG1655
<220> sgca b4303
<222> 258-357
<223> sgca b4303 U00096 complement(4525117..4525548)
<313> 8543: 297-316
<400> 8543
TGGTAACGAG GAGTGCGACC CCGTCTGGCT GCTTCTCTGC GTCAGCGCCA  50
CGGATGCCAA CGCACATATT TTGACCATTC AGCGCATCAG CCAGTTTATT  100
<210> 8544
<211> 100
<212> DNA
<213> Escherichia coli K-12 MG1655
<220> sgcc b4305
<222> 1195-1294
<223> sgcc b4305 U00096 complement(4526498..4527811)
<313> 8544: 1226-1245
<400> 8544
AATATGTTCG GTTGGTCGAT TTCTGAATTG ATGTCGCTTG GGATTATTGG  50
CGTTGTCGTG GCGGTCGGTA TTGTCGCCAG CGTCGTGCTG TTCTTACGTA  100
<210> 8545
<211> 100
<212> DNA
<213> Escherichia coli K-12 MG1655
<220> sgcc b4305
<222> 1071-1170
<223> sgcc b4305 U00096 complement(4526498..4527811)
<313> 8545: 1096-1115
<400> 8545
CATCGTGATG ATTACCGTCC TGCTGATCGC CACCCAGTTC GCGCCTTACT  50
TCACTGAAAT GGCGCTCAAA GGCGGTTTCA GTTTTGCCGG TGAAAGTGCG  100
<210> 8546
<211> 100
<212> DNA
<213> Escherichia coli K-12 MG1655
<220> sgcc b4305

<222> 951-1050
<223> sgcc b4305 U00096 complement(4526498..4527811)
<313> 8546: 1008-1027
<400> 8546
CAGCATTTTA CCGGGCAACA AAGTGTTACC CCTCGCAGAC CTGCCGGTAG 50
CACCGTTCTT TATCTGTATG GCTACGGTTA TCCACCGCGG AGACCTGGTC 100
<210> 8547
<211> 100
<212> DNA
<213> Escherichia coli K-12 MG1655
<220> sgce b4302
<222> 457-556
<223> sgce b4302 U00096 complement(4524473..4525105)
<313> 8547: 490-509
<400> 8547
AAAATCCAAA AAGTACGCAC GGCTTTTCCT CAGACCGAGT GCTGGGCCGA 50
CGGTGGAATT ACGCTGGCAG CTGCACAGCA ACTGGCAGCA GCTGGGGCGC 100
<210> 8548
<211> 100
<212> DNA
<213> Escherichia coli K-12 MG1655
<220> sgce b4302
<222> 405-504
<223> sgce b4302 U00096 complement(4524473..4525105)
<313> 8548: 437-456
<400> 8548
GACCAGTGAG CCCGATGGGC AGGGACAGCG GTTTATTCCC TCCATGTGCG 50
AGAAAATCCA AAAAGTACGC ACGGCTTTTC CTCAGACCGA GTGCTGGGCC 100
<210> 8549
<211> 100
<212> DNA
<213> Escherichia coli K-12 MG1655
<220> sgce b4302
<222> 375-474
<223> sgce b4302 U00096 complement(4524473..4525105)
<313> 8549: 406-425
<400> 8549
GGCGTCTGAG CTGGACGGAG TGATGGTGAT GACCAGTGAG CCCGATGGGC 50
AGGGACAGCG GTTTATTCCC TCCATGTGCG AGAAAATCCA AAAAGTACGC 100
<210> 8550
<211> 100
<212> DNA
<213> Escherichia coli K-12 MG1655
<220> b1030 b1031
<222> 1-100
<223> b1030 b1031 U00096 1096171..1096422
<313> 8550: 24-43
<400> 8550
GTGGGCGGGG GCACCCGGTG TGTGCAATAT ACGAAAAAAA AGCCCGTACT 50
TTCGTACGAG CTCTTCTTTA AATATGGCGG TGAGGGGGGG ATTGACTCGC 100
<210> 8551
<211> 100
<212> DNA
<213> Escherichia coli K-12 MG1655
<220> b1030 b1031
<222> 1-100
<223> b1030 b1031 U00096 1096171..1096422
<313> 8551: 20-39
<400> 8551
GTGGGCGGGG GCACCCGGTG TGTGCAATAT ACGAAAAAAA AGCCCGTACT 50
TTCGTACGAG CTCTTCTTTA AATATGGCGG TGAGGGGGGG ATTGACTCGC 100
<210> 8552
<211> 100
<212> DNA
<213> Escherichia coli K-12 MG1655
<220> b0302 b0303
<222> 171-270
<223> b0302 b0303 U00096 317526..317795
<313> 8552: 219-238
<400> 8552
GCTGATATGT TTCAGAATTA TTTTATCATC TGCATTGGTG GAAAATGCCA 50

TAACGCCAGA TAATAGTGTT GCAAATAAAA CAGATTTTTT AAACATATAA 100
<210> 8553
<211> 100
<212> DNA
<213> Escherichia coli K-12 MG1655
<220> b0302 b0303
<222> 171-270
<223> b0302 b0303 U00096 317526..317795
<313> 8553: 221-240
<400> 8553
GCTGATATGT TTCAGAATTA TTTTATCATC TGCATTGGTG GAAAATGCCA 50
TAACGCCAGA TAATAGTGTT GCAAATAAAA CAGATTTTTT AAACATATAA 100
<210> 8554
<211> 100
<212> DNA
<213> Escherichia coli K-12 MG1655
<220> b0302 b0303
<222> 171-270
<223> b0302 b0303 U00096 317526..317795
<313> 8554: 202-221
<400> 8554
GCTGATATGT TTCAGAATTA TTTTATCATC TGCATTGGTG GAAAATGCCA 50
TAACGCCAGA TAATAGTGTT GCAAATAAAA CAGATTTTTT AAACATATAA 100
<210> 8555
<211> 100
<212> DNA
<213> Escherichia coli K-12 MG1655
<220> b0309 b0310
<222> 114-213
<223> b0309 b0310 U00096 complement(323632..323844)
<313> 8555: 189-208
<400> 8555
TATTTTCAAC CTCGTATGCC TTCTTCAGGT TTATGTCCAG ACTTCATATC 50
TCTCTCAACA ATCCTCAATA ATCAGATACA CCGCTTTCAC CGGGCCATGA 100
<210> 8556
<211> 100
<212> DNA
<213> Escherichia coli K-12 MG1655
<220> b0309 b0310
<222> 114-213
<223> b0309 b0310 U00096 complement(323632..323844)
<313> 8556: 181-200
<400> 8556
TATTTTCAAC CTCGTATGCC TTCTTCAGGT TTATGTCCAG ACTTCATATC 50
TCTCTCAACA ATCCTCAATA ATCAGATACA CCGCTTTCAC CGGGCCATGA 100
<210> 8557
<211> 100
<212> DNA
<213> Escherichia coli K-12 MG1655
<220> b0309 b0310
<222> 114-213
<223> b0309 b0310 U00096 complement(323632..323844)
<313> 8557: 152-171
<400> 8557
TATTTTCAAC CTCGTATGCC TTCTTCAGGT TTATGTCCAG ACTTCATATC 50
TCTCTCAACA ATCCTCAATA ATCAGATACA CCGCTTTCAC CGGGCCATGA 100
<210> 8558
<211> 100
<212> DNA
<213> Escherichia coli K-12 MG1655
<220> sgcq b4304
<222> 708-807
<223> sgcq b4304 U00096 complement(4525679..4526485)
<313> 8558: 771-790
<400> 8558
GACCGCAACC ACCTTTAAAA AGGACGGCGT TTTTGCCAAT TTCGTTGACC 50
AGGCGCGAGT CAGTCAGTTT ATGGAGAAAG TGCATCATAT ACGCCGATAA 100
<210> 8559
<211> 100
<212> DNA
<213> Escherichia coli K-12 MG1655

<220> sgcq b4304
<222> 705-804
<223> sgcq b4304 U00096 complement(4525679..4526485)
<313> 8559: 726-745
<400> 8559
TGTGACCGCA ACCACCTTTA AAAAGGACGG CGTTTTTGCC AATTTCGTTG 50
ACCAGGCGCG AGTCAGTCAG TTTATGGAGA AAGTGCATCA TATACGCCGA 100
<210> 8560
<211> 100
<212> DNA
<213> Escherichia coli K-12 MG1655
<220> sgcq b4304
<222> 660-759
<223> sgcq b4304 U00096 complement(4525679..4526485)
<313> 8560: 691-710
<400> 8560
CTGCCTGGAA AACGTGGAGG AACAACTCAG TATCGCCGAT GGCTGTGTGA 50
CCGCAACCAC CTTTAAAAAG GACGGCGTTT TTGCCAATTT CGTTGACCAG 100
<210> 8561
<211> 100
<212> DNA
<213> Escherichia coli K-12 MG1655
<220> sgcr b4301
<222> 684-783
<223> sgcr b4301 U00096 complement(4523674..4524456)
<313> 8561: 755-774
<400> 8561
TATTAAAACG ATCATCAGTG ACAGCGGGTT ACCTGAGACT ATTGCACAGC 50
GTTATCAGCG AGCCGGATGT CAGTTGTTTT TACCTCATTC AATAAAGTGA 100
<210> 8562
<211> 100
<212> DNA
<213> Escherichia coli K-12 MG1655
<220> sgcr b4301
<222> 673-772
<223> sgcr b4301 U00096 complement(4523674..4524456)
<313> 8562: 702-721
<400> 8562
ACACTATCAT GTATTAAAAC GATCATCAGT GACAGCGGGT TACCTGAGAC 50
TATTGCACAG CGTTATCAGC GAGCCGGATG TCAGTTGTTT TTACCTCATT 100
<210> 8563
<211> 100
<212> DNA
<213> Escherichia coli K-12 MG1655
<220> sgcr b4301
<222> 588-687
<223> sgcr b4301 U00096 complement(4523674..4524456)
<313> 8563: 639-658
<400> 8563
AACACGGATT ATTCAACGCT CGCGTGAAGT TATTCTCATG GCCGATCACT 50
CAAAATTTGA TGCCGTTGAG CCGCATGCTG TAGCAACACT ATCATGTATT 100
<210> 8564
<211> 100
<212> DNA
<213> Escherichia coli K-12 MG1655
<220> sgcx b4306
<222> 1053-1152
<223> sgcx b4306 U00096 complement(4528098..4529249)
<313> 8564: 1103-1122
<400> 8564
TGATTGCATC CGCCTATTGA CCGCTCTGGC AGGTATGTCA GCAGCACATT 50
TCCCCGTTGA GCCTGATTCA GGCACTACAC AAGAGGCACA TCCATTATGA 100
<210> 8565
<211> 100
<212> DNA
<213> Escherichia coli K-12 MG1655
<220> sgcx b4306
<222> 1001-1100
<223> sgcx b4306 U00096 complement(4528098..4529249)
<313> 8565: 1051-1070
<400> 8565

CTTGTCGCTA TACCCATTCT CCGGCGGAGG TTGCCAGCCT GCGTGATTTG 50
ACTGATTGCA TCCGCCTATT GACCGCTCTG GCAGGTATGT CAGCAGCACA 100
<210> 8566
<211> 100
<212> DNA
<213> Escherichia coli K-12 MG1655
<220> sgcx b4306
<222> 938-1037
<223> sgcx b4306 U00096 complement(4528098..4529249)
<313> 8566: 1002-1021
<400> 8566
TAACAGAAAC CGGTTATATC CAGGTTGAGC AGGATGGTAT TCCCTGCGCC 50
AGTCTCTCTA TTCCTTGTCG CTATACCCAT TCTCCGGCGG AGGTTGCCAG 100
<210> 8567
<211> 100
<212> DNA
<213> Escherichia coli K-12 MG1655
<220> b1044 b1044
<222> 200-299
<223> b1044 b1044 U00096 1104637..1104948
<313> 8567: 234-253
<400> 8567
CCAGCAAAGC GTTAATGAGC GGCAATGTCA AAAACGCGCA AATAGCCTCA 50
GTAAAAGTGC AGTATCTTGA TAAACAGAAA AAGCTGGCTG TAATGAACAT 100
<210> 8568
<211> 100
<212> DNA
<213> Escherichia coli K-12 MG1655
<220> b1044 b1044
<222> 156-255
<223> b1044 b1044 U00096 1104637..1104948
<313> 8568: 192-211
<400> 8568
AATGAACTGC CCACAGAATG GTTCTATTCC GGGCAAAACC TACTCCAGCA 50
AAGCGTTAAT GAGCGGCAAT GTCAAAAACG CGCAAATAGC CTCAGTAAAA 100
<210> 8569
<211> 100
<212> DNA
<213> Escherichia coli K-12 MG1655
<220> b1044 b1044
<222> 92-191
<223> b1044 b1044 U00096 1104637..1104948
<313> 8569: 146-165
<400> 8569
TCCATTTTTA TGGCCAAATT GTGGAACCGG CATGTGACGT CAGCACCCAG 50
TCATCACCCG TAGAAATGAA CTGCCCACAG AATGGTTCTA TTCCGGGCAA 100
<210> 8570
<211> 100
<212> DNA
<213> Escherichia coli K-12 MG1655
<220> b1045 b1046
<222> 402-501
<223> b1045 b1046 U00096 1105043..1105576
<313> 8570: 436-455
<400> 8570
TGAAATCGCA GTAAAAACCG TTTCAGAATT TATTACCCGT CACGCTTTAC 50
CCGAACAGGT ATACTTTGTC TGTTATGATG AAGAAAACGC CCACCTCTAC 100
<210> 8571
<211> 100
<212> DNA
<213> Escherichia coli K-12 MG1655
<220> b1045 b1046
<222> 311-410
<223> b1045 b1046 U00096 1105043..1105576
<313> 8571: 337-356
<400> 8571
GCCTACGACT GGTGGCGGCA AACAGCTATA CGTCAGTGGC TTTTCCTGCA 50
ATCAGTACTG GGGTTTATGG TTACCCTCGT GCGGCAGCGG CTGAAATCGC 100
<210> 8572
<211> 100
<212> DNA

<213> Escherichia coli K-12 MG1655
<220> b1045 b1046
<222> 239-338
<223> b1045 b1046 U00096 1105043..1105576
<313> 8572: 259-278
<400> 8572
ACACCGTCGG GCCAGTCTGG CGTGGTGGTG AACAAAACGA AGACCAGCTT 50
TTGCAGGATG CCTATCTCAA TAGCCTACGA CTGGTGGCGG CAAACAGCTA 100
<210> 8573
<211> 100
<212> DNA
<213> Escherichia coli K-12 MG1655
<220> wrba b1005
<222> 498-597
<223> wrba b1005 U00096 complement(1066335..1066931)
<313> 8573: 549-568
<400> 8573
AGGCGGTGAC GGTTCACGCC AGCCCAGCCA GGAAGAACTG TCTATTGCTC 50
GTTATCAAGG GGAATATGTC GCAGGTCTGG CAGTTAAACT TAACGGCTAA 100
<210> 8574
<211> 100
<212> DNA
<213> Escherichia coli K-12 MG1655
<220> wrba b1005
<222> 456-555
<223> wrba b1005 U00096 complement(1066335..1066931)
<313> 8574: 513-532
<400> 8574
ACAGGTTCGC GGCGGTACGC CGTACGGCGC AACCACCATC GCAGGCGGTG 50
ACGGTTCACG CCAGCCCAGC CAGGAAGAAC TGTCTATTGC TCGTTATCAA 100
<210> 8575
<211> 100
<212> DNA
<213> Escherichia coli K-12 MG1655
<220> wrba b1005
<222> 424-523
<223> wrba b1005 U00096 complement(1066335..1066931)
<313> 8575: 451-470
<400> 8575
GGCTACGCAG CGCAGGAATT ATTTGACGTT TCACAGGTTC GCGGCGGTAC 50
GCCGTACGGC GCAACCACCA TCGCAGGCGG TGACGGTTCA CGCCAGCCCA 100
<210> 8576
<211> 100
<212> DNA
<213> Escherichia coli K-12 MG1655
<220> yi81_1 b0017
<222> 1014-1113
<223> yi81_1 b0017 U00096 15445..16557
<313> 8576: 1050-1069
<400> 8576
GATATTTGCT AATCTACTCG CCGCATTTTT AATTGACGAC ATAATCCAGC 50
CATCGCTGGA TTTCCCCCCC AGAAGTGCCG GATCCGAAAA GAAGAACTAA 100
<210> 8577
<211> 100
<212> DNA
<213> Escherichia coli K-12 MG1655
<220> yi81_1 b0017
<222> 909-1008
<223> yi81_1 b0017 U00096 15445..16557
<313> 8577: 944-963
<400> 8577
TTGTTACCGT CTGCGATGGC AAATTGAACT GGCTTTTAAG CGGCTCAAAA 50
GTTTGCTGCA CCTGGATGCT TTGCGTGCAA AGGAACCTGA ACTCGCGAAA 100
<210> 8578
<211> 100
<212> DNA
<213> Escherichia coli K-12 MG1655
<220> yi81_1 b0017
<222> 870-969
<223> yi81_1 b0017 U00096 15445..16557
<313> 8578: 919-938

<400> 8578
ATTACCGGAA GATGAATATT CAGCAGAGCA AGTGGCTGAT TGTTACCGTC 50
TGCGATGGCA AATTGAACTG GCTTTTAAGC GGCTCAAAAG TTTGCTGCAC 100
<210> 8579
<211> 100
<212> DNA
<213> Escherichia coli K-12 MG1655
<220> wcaa wcaA
<222> 741-840
<223> wcaa wcaA U00096 complement(2130580..2131419)
<313> 8579: 810-829
<400> 8579
CTACCAGATC CGCAATAAAC GTATGACCTG GCGCACTTTG CTGACGCTGC 50
TGTCGGTGCG TAACGGCAAA CGTCTGGCTG ATGGTATTCG GGGGCGCTAA 100
<210> 8580
<211> 100
<212> DNA
<213> Escherichia coli K-12 MG1655
<220> wcaa wcaA
<222> 711-810
<223> wcaa wcaA U00096 complement(2130580..2131419)
<313> 8580: 763-782
<400> 8580
CGCCAGCAAA AAATATCAGC TGTTTACCCT CTACCAGATC CGCAATAAAC 50
GTATGACCTG GCGCACTTTG CTGACGCTGC TGTCGGTGCG TAACGGCAAA 100
<210> 8581
<211> 100
<212> DNA
<213> Escherichia coli K-12 MG1655
<220> wcaa wcaA
<222> 667-766
<223> wcaa wcaA U00096 complement(2130580..2131419)
<313> 8581: 693-712
<400> 8581
GGGTACTTCC ACTTTTACCG CAAGCACAAA GACAAATTCG ACCGCGCCAG 50
CAAAAAATAT CAGCTGTTTA CCCTCTACCA GATCCGCAAT AAACGTATGA 100
<210> 8582
<211> 100
<212> DNA
<213> Escherichia coli K-12 MG1655
<220> yi81_2 b0583
<222> 1007-1106
<223> yi81_2 b0583 U00096 607288..608400
<313> 8582: 1036-1055
<400> 8582
AAGCGTGGAT ATTTGCTAAT CTACTCGCCG CATTTTTAAT TGACGACATA 50
ATCCAGCCAT CGCTGGATTT CCCCCCCAGA AGTGCCGGAT CCGAAAAGAA 100
<210> 8583
<211> 100
<212> DNA
<213> Escherichia coli K-12 MG1655
<220> yi81_2 b0583
<222> 912-1011
<223> yi81_2 b0583 U00096 607288..608400
<313> 8583: 961-980
<400> 8583
TTACCGTCTG CGATGGCAAA TTGAACTGGC TTTTAAGCGG CTCAAAAGTT 50
TGCTGCACCT GGATGCTTTG CGTGCAAAGG AACCTGAACT CGCGAAAGCG 100
<210> 8584
<211> 100
<212> DNA
<213> Escherichia coli K-12 MG1655
<220> yi81_2 b0583
<222> 855-954
<223> yi81_2 b0583 U00096 607288..608400
<313> 8584: 922-941
<400> 8584
GCTATTGCTA ACATCATTAC CGGAAGATGA ATATTCAGCA GAGCAAGTGG 50
CTGATTGTTA CCGTCTGCGA TGGCAAATTG AACTGGCTTT TAAGCGGCTC 100
<210> 8585
<211> 100

<212> DNA
<213> Escherichia coli K-12 MG1655
<220> wcab b2059
<222> 390-489
<223> wcab b2059 U00096 complement(2130089..2130577)
<313> 8585: 461-480
<400> 8585
TAACAACGTC ACCGTGGGCG CGGGCAGCGT GGTGCTCGAC TCTGTCCCGG 50
ACAACGCGCT GGTGGTGGGA GAAAAAGCGC GAGTGAAGGT AATTAAATGA 100
<210> 8586
<211> 100
<212> DNA
<213> Escherichia coli K-12 MG1655
<220> wcab b2059
<222> 359-458
<223> wcab b2059 U00096 complement(2130089..2130577)
<313> 8586: 387-406
<400> 8586
ACGTCATTAT TCTTGGTGAT ATCACGCTTG GTAACAACGT CACCGTGGGC 50
GCGGGCAGCG TGGTGCTCGA CTCTGTCCCG GACAACGCGC TGGTGGTGGG 100
<210> 8587
<211> 100
<212> DNA
<213> Escherichia coli K-12 MG1655
<220> wcab b2059
<222> 212-311
<223> wcab b2059 U00096 complement(2130089..2130577)
<313> 8587: 269-288
<400> 8587
CTATCCATCA CGGTTACGCC GTCGTGATCA ATAAAAACGT GGTAGCGGGG 50
GATGATTTTA CCATTCGCCA CGGCGTCACT ATCGGCAATC GTGGTGCCGA 100
<210> 8588
<211> 100
<212> DNA
<213> Escherichia coli K-12 MG1655
<220> yi81_3 b2395
<222> 1020-1119
<223> yi81_3 b2395 U00096 2512345..2513463
<313> 8588: 1045-1064
<400> 8588
GATATTTGCT AATCTACTCG CCGCATTTTT AATTGACGAC ATAATCCAGC 50
CATCGCTGGA TTTCCCCCCC AGAAGTGCCG GATCCGAAAA GAAGAACTAA 100
<210> 8589
<211> 100
<212> DNA
<213> Escherichia coli K-12 MG1655
<220> yi81_3 b2395
<222> 926-1025
<223> yi81_3 b2395 U00096 2512345..2513463
<313> 8589: 950-969
<400> 8589
TGCGATGGCA AATTGAACTG GCTTTTAAGC GGCTCAAAAG TTTGCTGCAC 50
CTGGATGCTT TGCGTGCAAA GGAACCTGAA CTCGCGAAAG CGTGGATATT 100
<210> 8590
<211> 100
<212> DNA
<213> Escherichia coli K-12 MG1655
<220> yi81_3 b2395
<222> 860-959
<223> yi81_3 b2395 U00096 2512345..2513463
<313> 8590: 925-944
<400> 8590
TGCTATTGCT AACATCATTA CCGGAAGATG AATATTCAGC AGAGCAAGTG 50
GCTGATTGTT ACCGTCTGCG ATGGCAAATT GAACTGGCTT TTAAGCGGCT 100
<210> 8591
<211> 100
<212> DNA
<213> Escherichia coli K-12 MG1655
<220> wcac b2058
<222> 1119-1218
<223> wcac b2058 U00096 complement(2128875..2130092)

<313> 8591: 1169-1188
<400> 8591
ATTTGGTACC ACGCTGGCTG AGTTCAGCCA ACGCAGCCGC GCCGCCTACA 50
GTGGACAACA GATGCTGGAG GAGTATGTCA ACTTCTATCA GAATCTGTAG 100
<210> 8592
<211> 100
<212> DNA
<213> Escherichia coli K-12 MG1655
<220> wcac b2058
<222> 1070-1169
<223> wcac b2058 U00096 complement(2128875..2130092)
<313> 8592: 1092-1111
<400> 8592
AGGTGCTGCA ACTGGTGCAG TTAAGCAAAC CGGAAATCGC GCAGGCGATA 50
TTTGGTACCA CGCTGGCTGA GTTCAGCCAA CGCAGCCGCG CCGCCTACAG 100
<210> 8593
<211> 100
<212> DNA
<213> Escherichia coli K-12 MG1655
<220> wcac b2058
<222> 933-1032
<223> wcac b2058 U00096 complement(2128875..2130092)
<313> 8593: 956-975
<400> 8593
ATTCAGTTCT CGCGTCGATA ACTACCCGCT GATTTTGTGT GAGGCGCTAT 50
CGATTGGCGT GCCGGTGATT GCCACCCATA GCGATGCGGC GCGGGAAGTG 100
<210> 8594
<211> 100
<212> DNA
<213> Escherichia coli K-12 MG1655
<220> wcad b2057
<222> 1044-1143
<223> wcad b2057 U00096 complement(2127683..2128900)
<313> 8594: 1077-1096
<400> 8594
GAAAGTGATA AAAATGATGA TCAACGCTTT TGGTGATAAC CGCAATTTTC 50
GCGTGCAATT ATATCTTTTT ACTCCGGTAT CGCTGTTTTT TACCGGTTCG 100
<210> 8595
<211> 100
<212> DNA
<213> Escherichia coli K-12 MG1655
<220> wcad b2057
<222> 897-996
<223> wcad b2057 U00096 complement(2127683..2128900)
<313> 8595: 937-956
<400> 8595
AGTCAGATTT GGCTCACTTT ATGAATATGT CGCATCATTC GGAATATTTA 50
ACGGTGCGGA TGTCGGAAAA ACCATAGACA ATGGTTTGTA TCTGCTGATT 100
<210> 8596
<211> 100
<212> DNA
<213> Escherichia coli K-12 MG1655
<220> wcad b2057
<222> 719-818
<223> wcad b2057 U00096 complement(2127683..2128900)
<313> 8596: 769-788
<400> 8596
AGAAAAAGTT ACCGTTAGCA TTGATTTCTC TGGCTGTATT CCTGGTTGGT 50
GTGGTAATCG CGTTTCCTTA TATTTCCACC CGTCTGGGCG ATTTAGGTAC 100
<210> 8597
<211> 100
<212> DNA
<213> Escherichia coli K-12 MG1655
<220> wcae b2056
<222> 648-747
<223> wcae b2056 U00096 complement(2126926..2127672)
<313> 8597: 696-715
<400> 8597
ACGACAAATA TTACATGTGC CTGGCTTTTG GGCTGAATTA TCCTGGCATT 50
TACGCCAACG TACTACCTCA AAGACGAAAG CCTTATATAA CAAAGTCTGA 100
<210> 8598

<211> 100
<212> DNA
<213> Escherichia coli K-12 MG1655
<220> wcae b2056
<222> 588-687
<223> wcae b2056 U00096 complement(2126926..2127672)
<313> 8598: 627-646
<400> 8598
CATGGGTGGG GTATCTACCA CCAATAATAT GGAATTGTGT GCTGACGCGA 50
AAAAAGTCCA ACGACAAATA TTACATGTGC CTGGCTTTTG GGCTGAATTA 100
<210> 8599
<211> 100
<212> DNA
<213> Escherichia coli K-12 MG1655
<220> wcae b2056
<222> 530-629
<223> wcae b2056 U00096 complement(2126926..2127672)
<313> 8599: 549-568
<400> 8599
AAATGTATAA AGCCGGTTAT GCATTTAAAA AACTCAATGG CCTGGTGTCT 50
GAATTTTCCA TGGGTGGGGT ATCTACCACC AATAATATGG AATTGTGTGC 100
<210> 8600
<211> 100
<212> DNA
<213> Escherichia coli K-12 MG1655
<220> wcaf b2055
<222> 447-546
<223> wcaf b2055 U00096 complement(2126362..2126910)
<313> 8600: 508-527
<400> 8600
CGTCGTGGGT GCACGAAGCA GTGTTTTTAA ATCGCTTCCG GCAAATGTGG 50
TTTGCCGGGG GAATCCCGCA GTGGTGATAC GCGAACGCGT TGAAACTGAA 100
<210> 8601
<211> 100
<212> DNA
<213> Escherichia coli K-12 MG1655
<220> wcaf b2055
<222> 317-416
<223> wcaf b2055 U00096 complement(2126362..2126910)
<313> 8601: 371-390
<400> 8601
TATGCACCGG TAGCCACGAC CATGCAAGTC AACATTTCAC CATTAACGCC 50
ACGCCTATTG TGATTGGCGA GAAATGCTGG CTGGCAACCG ATGTCTTTGT 100
<210> 8602
<211> 100
<212> DNA
<213> Escherichia coli K-12 MG1655
<220> wcaf b2055
<222> 232-331
<223> wcaf b2055 U00096 complement(2126362..2126910)
<313> 8602: 278-297
<400> 8602
GCGTGGGTCG GCGATGACGT CAATTTATAT ACCCTCGGTG AAATAACCAT 50
TGGCGCACAT TCGGTGATAT CGCAAAAAAG TTATTTATGC ACCGGTAGCC 100
<210> 8603
<211> 100
<212> DNA
<213> Escherichia coli K-12 MG1655
<220> wcag b2053
<222> 867-966
<223> wcag b2053 U00096 complement(2124247..2125212)
<313> 8603: 929-948
<400> 8603
CCTGCATCAG CTTGGCTGGT ATCACGAAAT CTCACTGGAA GCGGGGCTTG 50
CCAGCACTTA CCAGTGGTTC CTTGAGAATC AAGACCGCTT TCGGGGGTAA 100
<210> 8604
<211> 100
<212> DNA
<213> Escherichia coli K-12 MG1655
<220> wcag b2053
<222> 800-899

<223> wcag b2053 U00096 complement(2124247..2125212)
<313> 8604: 849-868
<400> 8604
AAGGCCGGGT GGTTTTTGAT GCCAGCAAAC CGGATGGCAC GCCGCGCAAA 50
CTGCTGGATG TGACGCGCCT GCATCAGCTT GGCTGGTATC ACGAAATCTC 100
<210> 8605
<211> 100
<212> DNA
<213> Escherichia coli K-12 MG1655
<220> wcag b2053
<222> 760-859
<223> wcag b2053 U00096 complement(2124247..2125212)
<313> 8605: 806-825
<400> 8605
CGCGAGCTGG CGCAAACCAT CGCCAAAGTG GTGGGTTACA AAGGCCGGGT 50
GGTTTTTGAT GCCAGCAAAC CGGATGGCAC GCCGCGCAAA CTGCTGGATG 100
<210> 8606
<211> 100
<212> DNA
<213> Escherichia coli K-12 MG1655
<220> wcah b2052
<222> 384-483
<223> wcah b2052 U00096 complement(2123765..2124247)
<313> 8606: 461-480
<400> 8606
CTGGCTGACG TCGGACGCGC TGCTCGCCAG TGATAATGTT CATGCTAACA 50
GCCGCGCCTA TTTTCTCGCT GAGAAGCGTA CCGGAGTACC CGGATTATGA 100
<210> 8607
<211> 100
<212> DNA
<213> Escherichia coli K-12 MG1655
<220> wcah b2052
<222> 372-471
<223> wcah b2052 U00096 complement(2123765..2124247)
<313> 8607: 422-441
<400> 8607
TGACGATTAC CGCTGGCTGA CGTCGGACGC GCTGCTCGCC AGTGATAATG 50
TTCATGCTAA CAGCCGCGCC TATTTTCTCG CTGAGAAGCG TACCGGAGTA 100
<210> 8608
<211> 100
<212> DNA
<213> Escherichia coli K-12 MG1655
<220> wcah b2052
<222> 331-430
<223> wcah b2052 U00096 complement(2123765..2124247)
<313> 8608: 357-376
<400> 8608
AGAGTATCGG AAGAAGAGCT GTTACTGCCG GATGAGCAGC ATGACGATTA 50
CCGCTGGCTG ACGTCGGACG CGCTGCTCGC CAGTGATAAT GTTCATGCTA 100
<210> 8609
<211> 100
<212> DNA
<213> Escherichia coli K-12 MG1655
<220> wcai b2051
<222> 961-1060
<223> wcai b2051 U00096 complement(2122545..2123768)
<313> 8609: 1013-1032
<400> 8609
TTGCCGTCGA AACTGACCAA TATTCTGGCA GTAGGCGGTA ACGCGGTGAT 50
TACTGCTGAA GCCTACACAG AACTGGGGCA GCTTTGCGAA ACCTTTCCGG 100
<210> 8610
<211> 100
<212> DNA
<213> Escherichia coli K-12 MG1655
<220> wcai b2051
<222> 923-1022
<223> wcai b2051 U00096 complement(2122545..2123768)
<313> 8610: 976-995
<400> 8610
ATCTGGTGGT GCAAAAACGC GGCGCGGCAG ATGCCGTATT GCCGTCGAAA 50
CTGACCAATA TTCTGGCAGT AGGCGGTAAC GCGGTGATTA CTGCTGAAGC 100

<210> 8611
<211> 100
<212> DNA
<213> Escherichia coli K-12 MG1655
<220> wcai b2051
<222> 779-878
<223> wcai b2051 U00096 complement(2122545..2123768)
<313> 8611: 828-847
<400> 8611
CGCTGATTTT TGCCATTGTC GGGCAGGGCG GCGGCAAAGC GCGGCTGGAA 50
AAAATGGCGC AGCAGCGTGG ACTGCGCAAC ATGCAATTTT TCCCGCTGCA 100
<210> 8612
<211> 100
<212> DNA
<213> Escherichia coli K-12 MG1655
<220> wcaj b2048
<222> 1296-1395
<223> wcaj b2048 U00096 complement(2118182..2119576)
<313> 8612: 1367-1386
<400> 8612
GTTCGACCTT GAGTACATCC GCGAATGGAG CGTCTGGTTC GATATCAAAA 50
TCGTTTTCCT GACGGTGTTC AAAGGTTTCG TTAACAAAGC GGCATATTGA 100
<210> 8613
<211> 100
<212> DNA
<213> Escherichia coli K-12 MG1655
<220> wcaj b2048
<222> 1296-1395
<223> wcaj b2048 U00096 complement(2118182..2119576)
<313> 8613: 1328-1347
<400> 8613
GTTCGACCTT GAGTACATCC GCGAATGGAG CGTCTGGTTC GATATCAAAA 50
TCGTTTTCCT GACGGTGTTC AAAGGTTTCG TTAACAAAGC GGCATATTGA 100
<210> 8614
<211> 100
<212> DNA
<213> Escherichia coli K-12 MG1655
<220> wcaj b2048
<222> 1218-1317
<223> wcaj b2048 U00096 complement(2118182..2119576)
<313> 8614: 1263-1282
<400> 8614
GGGCATTACC GGCTGGGCGC AGATTAACGG CTGGCGCGGC GAAACCGACA 50
CGCTGGAGAA AATGGAAAAA CGCGTCGAGT TCGACCTTGA GTACATCCGC 100
<210> 8615
<211> 100
<212> DNA
<213> Escherichia coli K-12 MG1655
<220> wcak b2046
<222> 1182-1281
<223> wcak b2046 U00096 complement(2115146..2116426)
<313> 8615: 1230-1249
<400> 8615
GGCGCTGAAT GCGCGACTTA GTGAAGCCGT CAGTCGTGAG CGTCAGACAG 50
GAATGCAGAT GGTGCAGTCT GTGCTTGAGC GCATCGGGGA GGTGAAATGA 100
<210> 8616
<211> 100
<212> DNA
<213> Escherichia coli K-12 MG1655
<220> wcak b2046
<222> 1104-1203
<223> wcak b2046 U00096 complement(2115146..2116426)
<313> 8616: 1165-1184
<400> 8616
GGAGATGGCA ATTGATATCC GTCATTTATT AGACGGCAGC CTGCAAGCGA 50
TGGTTGCGGA TACCTTAGGC CAGCTTCCGG CGCTGAATGC GCGACTTAGT 100
<210> 8617
<211> 100
<212> DNA
<213> Escherichia coli K-12 MG1655
<220> wcak b2046

<222> 1070-1169
<223> wcak b2046 U00096 complement(2115146..2116426)
<313> 8617: 1110-1129
<400> 8617
AATCCGCCGG GATTATGCAG CAGCTGGGAC TACCGGAGAT GGCAATTGAT 50
ATCCGTCATT TATTAGACGG CAGCCTGCAA GCGATGGTTG CGGATACCTT 100
<210> 8618
<211> 100
<212> DNA
<213> Escherichia coli K-12 MG1655
<220> wcal b2045
<222> 1122-1221
<223> wcal b2045 U00096 complement(2113929..2115149)
<313> 8618: 1185-1204
<400> 8618
ATTGGCTCCG GTCGTCAAAC GCGCGCGCGA AAAAGTTGAA CACGATTTTA 50
ACCAGCAGGT GATCAATCGA GAACTCGCCA GCTTGCTGCA GGCTTTATAG 100
<210> 8619
<211> 100
<212> DNA
<213> Escherichia coli K-12 MG1655
<220> wcal b2045
<222> 1085-1184
<223> wcal b2045 U00096 complement(2113929..2115149)
<313> 8619: 1108-1127
<400> 8619
AACGACTGGC GGCGTTTAGC CAACTGGACA CCGACGAATT GGCTCCGGTC 50
GTCAAACGCG CGCGCGAAAA AGTTGAACAC GATTTTAACC AGCAGGTGAT 100
<210> 8620
<211> 100
<212> DNA
<213> Escherichia coli K-12 MG1655
<220> wcal b2045
<222> 1051-1150
<223> wcal b2045 U00096 complement(2113929..2115149)
<313> 8620: 1075-1094
<400> 8620
CTGGTGCCTG AGAACGATGC TCGCGCACTG GCGCAACGAC TGGCGGCGTT 50
TAGCCAACTG GACACCGACG AATTGGCTCC GGTCGTCAAA CGCGCGCGCG 100
<210> 8621
<211> 100
<212> DNA
<213> Escherichia coli K-12 MG1655
<220> wcam b2044
<222> 1243-1342
<223> wcam b2044 U00096 complement(2112524..2113918)
<313> 8621: 1289-1308
<400> 8621
GTTTTTATGG CGAAAAAAGA AACACTGCTG TCTCTTGCAA ATGTTCATGC 50
GGTGAATGAA AAAGGACAAA GCTCCGTCGA TATCGACAGG ATTAATCACC 100
<210> 8622
<211> 100
<212> DNA
<213> Escherichia coli K-12 MG1655
<220> wcam b2044
<222> 1145-1244
<223> wcam b2044 U00096 complement(2112524..2113918)
<313> 8622: 1201-1220
<400> 8622
CGCAACATTT TTTTATGCGA AATATCAATG TGATGCAGGA ATCCTCAGTT 50
GGACCCGCAT TGAGCATGAA CTTTGACATG CGCAAAGACG TTCGTGGTGT 100
<210> 8623
<211> 100
<212> DNA
<213> Escherichia coli K-12 MG1655
<220> wcam b2044
<222> 1085-1184
<223> wcam b2044 U00096 complement(2112524..2113918)
<313> 8623: 1135-1154
<400> 8623
CCTTTGTGGC GCTAACTAAC ATTGAGATGA AGCGTGCGTC GCTGGAGTTA 50

CACAACAAAC CGCAACATTT TTTTATGCGA AATATCAATG TGATGCAGGA 100
<210> 8624
<211> 100
<212> DNA
<213> Escherichia coli K-12 MG1655
<220> b1052 b1053
<222> 47-99
<223> b1052 b1053 U00096 complement(1113434..1113532)
<313> 8624: 61-80
<400> 8624
TTTTTCGCCT TTCATACTTG CAAAAGCGGA GAATCAGCTA TCCTTTTCCC 50
TGA 100
<210> 8625
<211> 100
<212> DNA
<213> Escherichia coli K-12 MG1655
<220> b1052 b1053
<222> 17-99
<223> b1052 b1053 U00096 complement(1113434..1113532)
<313> 8625: 55-74
<400> 8625
CGTCGTCGAA TACCCCAGGT ATCGAACTGA TTTTTCGCCT TTCATACTTG 50
CAAAAGCGGA GAATCAGCTA TCCTTTTCCC TGA 100
<210> 8626
<211> 100
<212> DNA
<213> Escherichia coli K-12 MG1655
<220> b1052 b1053
<222> 23-99
<223> b1052 b1053 U00096 complement(1113434..1113532)
<313> 8626: 44-63
<400> 8626
CGAATACCCC AGGTATCGAA CTGATTTTTC GCCTTTCATA CTTGCAAAAG 50
CGGAGAATCA GCTATCCTTT TCCCTGA 100
<210> 8627
<211> 100
<212> DNA
<213> Escherichia coli K-12 MG1655
<220> b1057 b1058
<222> 468-567
<223> b1057 b1058 U00096 complement(1117703..1118269)
<313> 8627: 497-516
<400> 8627
CGTTATGCAC GGATTTATGG CCCTGAAGCA TCATTTCATC GATAAAGACG 50
ACACTCTGAA GCGCATGCTG GGAAAGTCGT CATCTGACTA TGGAGTATAA 100
<210> 8628
<211> 100
<212> DNA
<213> Escherichia coli K-12 MG1655
<220> b1057 b1058
<222> 313-412
<223> b1057 b1058 U00096 complement(1117703..1118269)
<313> 8628: 335-354
<400> 8628
GGCATCAGCG GCTATCTGAT CTCAACTGCC GATGGTAAAC CGATCAGCGT 50
TTTTGGCTGG TTTGACGTCC CCGCGACCCT TGCCGACGCT GGCGCACAGG 100
<210> 8629
<211> 100
<212> DNA
<213> Escherichia coli K-12 MG1655
<220> b1057 b1058
<222> 280-379
<223> b1057 b1058 U00096 complement(1117703..1118269)
<313> 8629: 299-318
<400> 8629
CATCTCGCAT TGTACCTATT GCTTTTCGCC ATCGGCATCA GCGGCTATCT 50
GATCTCAACT GCCGATGGTA AACCGATCAG CGTTTTTGGC TGGTTTGACG 100
<210> 8630
<211> 100
<212> DNA
<213> Escherichia coli K-12 MG1655

<220> ylig yliG
<222> 1227-1326
<223> ylig yliG U00096 complement(875933..877258)
<313> 8630: 1291-1310
<400> 8630
GGTTTATCTC AATGGTGAAA CCAACGTTAA GCCGGGTGAT ATCCTGCGTG 50
TGAAAGTCGA GCACGCCGAT GAGTACGATT TGTGGGGTAG CCGGGTTTAA 100
<210> 8631
<211> 100
<212> DNA
<213> Escherichia coli K-12 MG1655
<220> ylig yliG
<222> 1203-1302
<223> ylig yliG U00096 complement(875933..877258)
<313> 8631: 1262-1281
<400> 8631
AGATGCACCG GAAATCGACG GCGCGGTTTA TCTCAATGGT GAAACCAACG 50
TTAAGCCGGG TGATATCCTG CGTGTGAAAG TCGAGCACGC CGATGAGTAC 100
<210> 8632
<211> 100
<212> DNA
<213> Escherichia coli K-12 MG1655
<220> ylig yliG
<222> 1184-1283
<223> ylig yliG U00096 complement(875933..877258)
<313> 8632: 1216-1235
<400> 8632
CGATTGGTCG CAGCATGGCA GATGCACCGG AAATCGACGG CGCGGTTTAT 50
CTCAATGGTG AAACCAACGT TAAGCCGGGT GATATCCTGC GTGTGAAAGT 100
<210> 8633
<211> 100
<212> DNA
<213> Escherichia coli K-12 MG1655
<220> ylii b0838
<222> 1017-1116
<223> ylii b0838 U00096 877965..879080
<313> 8633: 1076-1095
<400> 8633
GCAGCGAATT CGTGATGTTC GCACTGGACC CGACGGTTAT TTATACGTTC 50
TCACCGACGA GTCCAGTGGG GAATTACTTA AAGTTAGCCC ACGCAATTAG 100
<210> 8634
<211> 100
<212> DNA
<213> Escherichia coli K-12 MG1655
<220> ylii b0838
<222> 1015-1114
<223> ylii b0838 U00096 877965..879080
<313> 8634: 1043-1062
<400> 8634
GGGCAGCGAA TTCGTGATGT TCGCACTGGA CCCGACGGTT ATTTATACGT 50
TCTCACCGAC GAGTCCAGTG GGGAATTACT TAAAGTTAGC CCACGCAATT 100
<210> 8635
<211> 100
<212> DNA
<213> Escherichia coli K-12 MG1655
<220> ylii b0838
<222> 940-1039
<223> ylii b0838 U00096 877965..879080
<313> 8635: 974-993
<400> 8635
AAAGATAAAG ATGTCATTGT GATGAGCGTC AACGGCGACA AAGTGACAGA 50
AGATGGCCGT ATTTTAACGG ACAGAGGGCA GCGAATTCGT GATGTTCGCA 100
<210> 8636
<211> 100
<212> DNA
<213> Escherichia coli K-12 MG1655
<220> ylij b0839
<222> 488-587
<223> ylij b0839 U00096 complement(879077..879709)
<313> 8636: 541-560
<400> 8636

TGGGCGATAT CGCTATCGCA CCGTTCATCT ACAATTTGTT TAACGTTGGC 50
CTGACCTGGA CACCGCGTCC AAATCTGCAA CGCTGGTATC AGCAACTCAC 100
<210> 8637
<211> 100
<212> DNA
<213> Escherichia coli K-12 MG1655
<220> ylij b0839
<222> 423-522
<223> ylij b0839 U00096 complement(879077..879709)
<313> 8637: 463-482
<400> 8637
CCTGTTTGCC CTGCTCGATG CGGAACTGGC AAAAGTAAAA TGGTTCTCTG 50
GCGACGAGTT TGGTGTGGGC GATATCGCTA TCGCACCGTT CATCTACAAT 100
<210> 8638
<211> 100
<212> DNA
<213> Escherichia coli K-12 MG1655
<220> ylij b0839
<222> 371-470
<223> ylij b0839 U00096 complement(879077..879709)
<313> 8638: 429-448
<400> 8638
CGGAAGAGCG CGATCAGGCC GCCATTGATG CCAGTTGCAA AGAGTGCGAC 50
GCCCTGTTTG CCCTGCTCGA TGCGGAACTG GCAAAAGTAA AATGGTTCTC 100
<210> 8639
<211> 100
<212> DNA
<213> Escherichia coli K-12 MG1655
<220> cpdb b4214
<222> 1822-1921
<223> cpdb b4214 U00096 complement(4432200..4434143)
<313> 8639: 1861-1880
<400> 8639
TTCGAAACCT CTCCGTCAGA TAAAGCCGCA GCGTTTATTA AAGAGAAAGG 50
GCAGTATCCG ATGAATAAAG TCGCGACCGA TGATATCGGG TTTGCGATTT 100
<210> 8640
<211> 100
<212> DNA
<213> Escherichia coli K-12 MG1655
<220> cpdb b4214
<222> 1729-1828
<223> cpdb b4214 U00096 complement(4432200..4434143)
<313> 8640: 1785-1804
<400> 8640
GATGAGTCGA AACGTGCGGG GGAAATTCAC CCGGCGGCAG ATAACAACTG 50
GCGTTTAGCA CCGATAGCTG GCGATAAGAA ACTGGATATC CGTTTCGAAA 100
<210> 8641
<211> 100
<212> DNA
<213> Escherichia coli K-12 MG1655
<220> cpdb b4214
<222> 1703-1802
<223> cpdb b4214 U00096 complement(4432200..4434143)
<313> 8641: 1743-1762
<400> 8641
GCTCGGTGCT GGCAGCGTGG ATTGCTGATG AGTCGAAACG TGCGGGGGAA 50
ATTCACCCGG CGGCAGATAA CAACTGGCGT TTAGCACCGA TAGCTGGCGA 100
<210> 8642
<211> 100
<212> DNA
<213> Escherichia coli K-12 MG1655
<220> rbsa b3750
<222> 1407-1506
<223> rbsa b3750 U00096 3931404..3932909
<313> 8642: 1468-1487
<400> 8642
TGAAGGGCAT CTCAGCGGGG AATTTACTCG TGAGCAGGCC ACCCAGGAAG 50
TGTTAATGGC TGCCGCTGTG GGCAAGCTTA ATCGCGTGAA TCAGGAGTAA 100
<210> 8643
<211> 100
<212> DNA

<213> Escherichia coli K-12 MG1655
<220> rbsa b3750
<222> 1200-1299
<223> rbsa b3750 U00096 3931404..3932909
<313> 8643: 1247-1266
<400> 8643
TCAGCAAAAA GTGGCGATTG CCCGCGGTCT GATGACACGC CCCAAAGTGT 50
TGATCCTTGA TGAACCTACC CGTGGCGTAG ATGTCGGCGC GAAAAAAGAG 100
<210> 8644
<211> 100
<212> DNA
<213> Escherichia coli K-12 MG1655
<220> rbsa b3750
<222> 1153-1252
<223> rbsa b3750 U00096 3931404..3932909
<313> 8644: 1195-1214
<400> 8644
AAAACTCCGT CGATGGAACA GGCAATTGGT CTGCTTTCCG GTGGCAATCA 50
GCAAAAAGTG GCGATTGCCC GCGGTCTGAT GACACGCCCC AAAGTGTTGA 100
<210> 8645
<211> 100
<212> DNA
<213> Escherichia coli K-12 MG1655
<220> rbsb b3752
<222> 786-885
<223> rbsb b3752 U00096 3933904..3934794
<313> 8645: 845-864
<400> 8645
CGATCAGATT GGCGCGAAAG GCGTCGAAAC CGCAGATAAA GTGCTGAAAG 50
GCGAGAAAGT TCAGGCTAAG TATCCGGTTG ATCTGAAACT GGTTGTTAAG 100
<210> 8646
<211> 100
<212> DNA
<213> Escherichia coli K-12 MG1655
<220> rbsb b3752
<222> 780-879
<223> rbsb b3752 U00096 3933904..3934794
<313> 8646: 796-815
<400> 8646
GCTACCCGAT CAGATTGGCG CGAAAGGCGT CGAAACCGCA GATAAAGTGC 50
TGAAAGGCGA GAAAGTTCAG GCTAAGTATC CGGTTGATCT GAAACTGGTT 100
<210> 8647
<211> 100
<212> DNA
<213> Escherichia coli K-12 MG1655
<220> rbsb b3752
<222> 744-843
<223> rbsb b3752 U00096 3933904..3934794
<313> 8647: 774-793
<400> 8647
GGTGAATGAT GGCAAACTAG CAGCGACTAT CGCTCAGCTA CCCGATCAGA 50
TTGGCGCGAA AGGCGTCGAA ACCGCAGATA AAGTGCTGAA AGGCGAGAAA 100
<210> 8648
<211> 100
<212> DNA
<213> Escherichia coli K-12 MG1655
<220> b0332 b0333
<222> 348-447
<223> b0332 b0333 U00096 complement(348742..349188)
<313> 8648: 416-435
<400> 8648
CGACGCATCA GGCATCGGCT GTCGAATACA ATCAACGGGC GTTTTTTATT 50
TCACCTGGCT ACGGGCAAAC AGGTTGTCGA GCTTCTCTTC GTACTGGTAG 100
<210> 8649
<211> 100
<212> DNA
<213> Escherichia coli K-12 MG1655
<220> b0332 b0333
<222> 319-418
<223> b0332 b0333 U00096 complement(348742..349188)
<313> 8649: 371-390

<400> 8649
CACCTCGTAG GCCTGATAAG TCGCGTCAGC GACGCATCAG GCATCGGCTG 50
TCGAATACAA TCAACGGGCG TTTTTTATTT CACCTGGCTA CGGGCAAACA 100
<210> 8650
<211> 100
<212> DNA
<213> Escherichia coli K-12 MG1655
<220> b0332 b0333
<222> 256-355
<223> b0332 b0333 U00096 complement(348742..349188)
<313> 8650: 297-316
<400> 8650
GGCGCATCAG GCATCGGTTG TCGGATGCGA CGCCATCCGT CTTATCCGAC 50
CTACAGTTCA GTGCACCTCG TAGGCCTGAT AAGTCGCGTC AGCGACGCAT 100
<210> 8651
<211> 100
<212> DNA
<213> Escherichia coli K-12 MG1655
<220> rbsc b3751
<222> 755-854
<223> rbsc b3751 U00096 3932914..3933879
<313> 8651: 811-830
<400> 8651
GCTATGAGCT GGATGCTATT GCTGCGGTGG TTCTGGGCGG TACGAGTCTG 50
GCGGGCGGAA AAGGTCGCAT TGTTGGGACG TTGATCGGCG CATTAATTCT 100
<210> 8652
<211> 100
<212> DNA
<213> Escherichia coli K-12 MG1655
<220> rbsc b3751
<222> 710-809
<223> rbsc b3751 U00096 3932914..3933879
<313> 8652: 749-768
<400> 8652
TTGAAGTGGC GCGTCTCTCC TCCGCACAAC CCACGGCGGG GACTGGCTAT 50
GAGCTGGATG CTATTGCTGC GGTGGTTCTG GGCGGTACGA GTCTGGCGGG 100
<210> 8653
<211> 100
<212> DNA
<213> Escherichia coli K-12 MG1655
<220> rbsc b3751
<222> 545-644
<223> rbsc b3751 U00096 3932914..3933879
<313> 8653: 599-618
<400> 8653
TCTTCCTCGC GGCCTGGTAC ATGCTGCATC ACACGCGTCT GGGGCGTTAC 50
ATCTACGCGC TGGGCGGCAA CGAAGCGGCA ACGCGTCTTT CTGGTATCAA 100
<210> 8654
<211> 100
<212> DNA
<213> Escherichia coli K-12 MG1655
<220> rbsd b3749
<222> 354-453
<223> rbsd b3749 U00096 3930941..3931396
<313> 8654: 380-399
<400> 8654
TGAACAATTC AAACAACAAA CCGCAGAAAG TCAGGCGGTA ATTCGCAGCG 50
GAGAATGTTC TCCGTATGCG AATATCATTC TCTGTGCTGG CGTGACGTTC 100
<210> 8655
<211> 100
<212> DNA
<213> Escherichia coli K-12 MG1655
<220> rbsd b3749
<222> 271-370
<223> rbsd b3749 U00096 3930941..3931396
<313> 8655: 323-342
<400> 8655
CAACTCCACG AAACGTTGCT CACTCACCTT GAGCAGCTGC AAAAACACCA 50
GGGAAATACC ATTGAAATTC GTTACACCAC GCATGAACAA TTCAAACAAC 100
<210> 8656
<211> 100

<212> DNA
<213> Escherichia coli K-12 MG1655
<220> rbsd b3749
<222> 222-321
<223> rbsd b3749 U00096 3930941..3931396
<313> 8656: 264-283
<400> 8656
GCAGGTCGAG GCGGCCATTA TCGCGGAAGA GATCAAACAC CATAATCCGC 50
AACTCCACGA AACGTTGCTC ACTCACCTTG AGCAGCTGCA AAAACACCAG 100
<210> 8657
<211> 100
<212> DNA
<213> Escherichia coli K-12 MG1655
<220> ylja b0882
<222> 222-321
<223> ylja b0882 U00096 922136..922456
<313> 8657: 264-283
<400> 8657
AGTCTTTACC GCCGAGGTTG CAGAAACCAA AGTGGCGATG GTGAACAAGT 50
ACGCGAGGGA GAATGAGCAT CCATTGCTGT GTACGCTAGA AAAAGCCTGA 100
<210> 8658
<211> 100
<212> DNA
<213> Escherichia coli K-12 MG1655
<220> ylja b0882
<222> 185-284
<223> ylja b0882 U00096 922136..922456
<313> 8658: 239-258
<400> 8658
TGCTCGCTGT TCACTACCAG GGGAAGGCCA TTTGCGGAGT CTTTACCGCC 50
GAGGTTGCAG AAACCAAAGT GGCGATGGTG AACAAGTACG CGAGGGAGAA 100
<210> 8659
<211> 100
<212> DNA
<213> Escherichia coli K-12 MG1655
<220> ylja b0882
<222> 133-232
<223> ylja b0882 U00096 922136..922456
<313> 8659: 185-204
<400> 8659
GACGTGTTAC AAAAATTCTT TTCTTATGAT GTAGAACGTG CAACGCAATT 50
GATGCTCGCT GTTCACTACC AGGGGAAGGC CATTTGCGGA GTCTTTACCG 100
<210> 8660
<211> 100
<212> DNA
<213> Escherichia coli K-12 MG1655
<220> rbsk b3753
<222> 831-930
<223> rbsk b3753 U00096 3934920..3935849
<313> 8660: 903-922
<400> 8660
TGCCCATGCT GCCGCTGCGA TTGCCGTAAC ACGTAAAGGC GCACAACCTT 50
CCGTACCGTG GCGTGAAGAG ATCGACGCAT TTTTAGACAG GCAGAGGTGA 100
<210> 8661
<211> 100
<212> DNA
<213> Escherichia coli K-12 MG1655
<220> rbsk b3753
<222> 820-919
<223> rbsk b3753 U00096 3934920..3935849
<313> 8661: 868-887
<400> 8661
GCGATTCGTT TTGCCCATGC TGCCGCTGCG ATTGCCGTAA CACGTAAAGG 50
CGCACAACCT TCCGTACCGT GGCGTGAAGA GATCGACGCA TTTTTAGACA 100
<210> 8662
<211> 100
<212> DNA
<213> Escherichia coli K-12 MG1655
<220> rbsk b3753
<222> 768-867
<223> rbsk b3753 U00096 3934920..3935849

<313> 8662: 815-834
<400> 8662
CTTTAACGGT GCGTTAATCA CGGCATTGCT GGAAGAAAAA CCATTGCCAG 50
AGGCGATTCG TTTTGCCCAT GCTGCCGCTG CGATTGCCGT AACACGTAAA 100
<210> 8663
<211> 100
<212> DNA
<213> Escherichia coli K-12 MG1655
<220> rbsr b3754
<222> 894-993
<223> rbsr b3754 U00096 3935853..3936845
<313> 8663: 969-988
<400> 8663
GCTGGCGATT GATGTACTCA TCCATCGGAT AACCCAGCCG ACCCTTCAGC 50
AACAACGATT ACAACTTACT CCGATTCTGA TGGAACGCGG TTCGGCTTAG 100
<210> 8664
<211> 100
<212> DNA
<213> Escherichia coli K-12 MG1655
<220> rbsr b3754
<222> 894-993
<223> rbsr b3754 U00096 3935853..3936845
<313> 8664: 917-936
<400> 8664
GCTGGCGATT GATGTACTCA TCCATCGGAT AACCCAGCCG ACCCTTCAGC 50
AACAACGATT ACAACTTACT CCGATTCTGA TGGAACGCGG TTCGGCTTAG 100
<210> 8665
<211> 100
<212> DNA
<213> Escherichia coli K-12 MG1655
<220> rbsr b3754
<222> 844-943
<223> rbsr b3754 U00096 3935853..3936845
<313> 8665: 887-906
<400> 8665
ATGACGCCAC CATTAACCAC TATCCACCAA CCGAAAGATG AACTGGGGGA 50
GCTGGCGATT GATGTACTCA TCCATCGGAT AACCCAGCCG ACCCTTCAGC 100
<210> 8666
<211> 100
<212> DNA
<213> Escherichia coli K-12 MG1655
<220> rmf b0954
<222> 55-154
<223> rmf b0954 U00096 1014938..1015105
<313> 8666: 110-129
<400> 8666
GCCGGCATCG CCGGACGCTC AAAAGAAATG TGTCCCTATC AGACGCTGAA 50
TCAAAGGTCA CAATGGCTGG GAGGCTGGCG AGAAGCCATG GCGGACAGGG 100
<210> 8667
<211> 100
<212> DNA
<213> Escherichia coli K-12 MG1655
<220> rmf b0954
<222> 69-168
<223> rmf b0954 U00096 1014938..1015105
<313> 8667: 108-127
<400> 8667
ACGCTCAAAA GAAATGTGTC CCTATCAGAC GCTGAATCAA AGGTCACAAT 50
GGCTGGGAGG CTGGCGAGAA GCCATGGCGG ACAGGGTAGT AATGGCCTGA 100
<210> 8668
<211> 100
<212> DNA
<213> Escherichia coli K-12 MG1655
<220> rmf b0954
<222> 39-138
<223> rmf b0954 U00096 1014938..1015105
<313> 8668: 101-120
<400> 8668
TCAACGTGGT TATCAGGCCG GCATCGCCGG ACGCTCAAAA GAAATGTGTC 50
CCTATCAGAC GCTGAATCAA AGGTCACAAT GGCTGGGAGG CTGGCGAGAA 100
<210> 8669

<211> 100
<212> DNA
<213> Escherichia coli K-12 MG1655
<220> phna b4109
<222> 235-334
<223> phna b4109 U00096 complement(4323977..4324312)
<313> 8669: 269-288
<400> 8669
GTGAAAAACA TCCGCCTGGT TGAAGGCGAC CATAACATCG ATTGCAAAAT 50
CGACGGTTTT GGTCCGATGA AACTGAAATC TGAGTTTGTG AAAAAGAACT 100
<210> 8670
<211> 100
<212> DNA
<213> Escherichia coli K-12 MG1655
<220> phna b4109
<222> 176-275
<223> phna b4109 U00096 complement(4323977..4324312)
<313> 8670: 216-235
<400> 8670
CCATCATTAA AGATCTGAAG GTGAAAGGTA GCTCTTCGAT GCTGAAAATT 50
GGCACCAAAG TGAAAAACAT CCGCCTGGTT GAAGGCGACC ATAACATCGA 100
<210> 8671
<211> 100
<212> DNA
<213> Escherichia coli K-12 MG1655
<220> phna b4109
<222> 141-240
<223> phna b4109 U00096 complement(4323977..4324312)
<313> 8671: 174-193
<400> 8671
TAACGGCAAT CTGCTGGCTG ACGGCGACAG CGTTACCATC ATTAAAGATC 50
TGAAGGTGAA AGGTAGCTCT TCGATGCTGA AAATTGGCAC CAAAGTGAAA 100
<210> 8672
<211> 100
<212> DNA
<213> Escherichia coli K-12 MG1655
<220> phnb b4108
<222> 345-444
<223> phnb b4108 U00096 complement(4322876..4323319)
<313> 8672: 406-425
<400> 8672
CTGGCAGGAA ACTTTCTGGG CGCATGGCTT TGGCAAAGTC ACCGATAAAT 50
TTGGCGTACC GTGGATGATT AATGTCGTCA AACAACAACC AACGCAATAA 100
<210> 8673
<211> 100
<212> DNA
<213> Escherichia coli K-12 MG1655
<220> phnb b4108
<222> 313-412
<223> phnb b4108 U00096 complement(4322876..4323319)
<313> 8673: 350-369
<400> 8673
AATCTTGCCG CTAACGGAAA AATCGAAATG GCCTGGCAGG AAACTTTCTG 50
GGCGCATGGC TTTGGCAAAG TCACCGATAA ATTTGGCGTA CCGTGGATGA 100
<210> 8674
<211> 100
<212> DNA
<213> Escherichia coli K-12 MG1655
<220> phnb b4108
<222> 274-373
<223> phnb b4108 U00096 complement(4322876..4323319)
<313> 8674: 303-322
<400> 8674
GATTCGCAAC AGGTCGAAGA AGGAAAACGC TGGTTTGACA ATCTTGCCGC 50
TAACGGAAAA ATCGAAATGG CCTGGCAGGA AACTTTCTGG GCGCATGGCT 100
<210> 8675
<211> 100
<212> DNA
<213> Escherichia coli K-12 MG1655
<220> cada b4132
<222> 2049-2148

<223> cada b4132 U00096 complement(4354048..4356195)
<313> 8675: 2100-2119
<400> 8675
CGCTCACTAT CCGGGCTTTG AAACCGATAT TCACGGTGCA TACCGTCAGG 50
CTGATGGCCG CTATACCGTT AAGGTATTGA AAGAAGAAAG CAAAAAATAA 100
<210> 8676
<211> 100
<212> DNA
<213> Escherichia coli K-12 MG1655
<220> cada b4132
<222> 2022-2121
<223> cada b4132 U00096 complement(4354048..4356195)
<313> 8676: 2061-2080
<400> 8676
GTTCCTGCAG ATGCTGTGTG AAATCGGCGC TCACTATCCG GGCTTTGAAA 50
CCGATATTCA CGGTGCATAC CGTCAGGCTG ATGGCCGCTA TACCGTTAAG 100
<210> 8677
<211> 100
<212> DNA
<213> Escherichia coli K-12 MG1655
<220> cada b4132
<222> 1937-2036
<223> cada b4132 U00096 complement(4354048..4356195)
<313> 8677: 1991-2010
<400> 8677
CCAATATGAT CCTTCCGTAC CCGCCGGGAG TTCCTCTGGT AATGCCGGGT 50
GAAATGATCA CCGAAGAAAG CCGTCCGGTT CTGGAGTTCC TGCAGATGCT 100
<210> 8678
<211> 100
<212> DNA
<213> Escherichia coli K-12 MG1655
<220> phnc b4107
<222> 690-789
<223> phnc b4107 U00096 complement(4321955..4322743)
<313> 8678: 760-779
<400> 8678
CGTCTTCTAC GACGGCAGCA GCCAACAGTT TGATAACGAA CGTTTTGACC 50
ATCTCTACCG CAGCATTAAC CGCGTCGAAG AGAACGCGAA AGCTGCCTGA 100
<210> 8679
<211> 100
<212> DNA
<213> Escherichia coli K-12 MG1655
<220> phnc b4107
<222> 674-773
<223> phnc b4107 U00096 complement(4321955..4322743)
<313> 8679: 712-731
<400> 8679
CCCTGCGCCA GGGGCACGTC TTCTACGACG GCAGCAGCCA ACAGTTTGAT 50
AACGAACGTT TTGACCATCT CTACCGCAGC ATTAACCGCG TCGAAGAGAA 100
<210> 8680
<211> 100
<212> DNA
<213> Escherichia coli K-12 MG1655
<220> phnc b4107
<222> 636-735
<223> phnc b4107 U00096 complement(4321955..4322743)
<313> 8680: 668-687
<400> 8680
GGTGGATTAC GCCCTGCGCT ACTGCGAACG CATCGTCGCC CTGCGCCAGG 50
GGCACGTCTT CTACGACGGC AGCAGCCAAC AGTTTGATAA CGAACGTTTT 100
<210> 8681
<211> 100
<212> DNA
<213> Escherichia coli K-12 MG1655
<220> cadb b4133
<222> 1236-1335
<223> cadb b4133 U00096 complement(4356275..4357609)
<313> 8681: 1316-1335
<400> 8681
CTTCATCGTC AGCCTGATTA TCCTGATGTT CTACGCTCGC AAAATGCACG 50
AGCGCCAGAG CCACTCAATG GATAACCACA CCGCGTCTAA CGCACATTAA 100

<210> 8682
<211> 100
<212> DNA
<213> Escherichia coli K-12 MG1655
<220> cadb b4133
<222> 1218-1317
<223> cadb b4133 U00096 complement(4356275..4357609)
<313> 8682: 1261-1280
<400> 8682
CTTCGAGCTG GCAGGTACCT TCATCGTCAG CCTGATTATC CTGATGTTCT 50
ACGCTCGCAA AATGCACGAG CGCCAGAGCC ACTCAATGGA TAACCACACC 100
<210> 8683
<211> 100
<212> DNA
<213> Escherichia coli K-12 MG1655
<220> cadb b4133
<222> 1169-1268
<223> cadb b4133 U00096 complement(4356275..4357609)
<313> 8683: 1206-1225
<400> 8683
CTGTACTGGG TTGCGTGTTC TGCTTCATCG CGCTGATGGG CGCAAGCTCC 50
TTCGAGCTGG CAGGTACCTT CATCGTCAGC CTGATTATCC TGATGTTCTA 100
<210> 8684
<211> 100
<212> DNA
<213> Escherichia coli K-12 MG1655
<220> phnd b4106
<222> 918-1017
<223> phnd b4106 U00096 complement(4320914..4321930)
<313> 8684: 992-1011
<400> 8684
GCTGGCAAAA ACCACCGAGA TTCAGGCGCA GCTGGATGAC CTGGACCGCC 50
TGAACAACGC GCTAAGCGCG ATGAGTTCGG TGAGTAAAGC GGTGCAGTAA 100
<210> 8685
<211> 100
<212> DNA
<213> Escherichia coli K-12 MG1655
<220> phnd b4106
<222> 868-967
<223> phnd b4106 U00096 complement(4320914..4321930)
<313> 8685: 916-935
<400> 8685
AAAGAGATGC AGGGCGTGAA AAGCAATAAA GGACTGAATG AGCAGGACAA 50
GCTGGCAAAA ACCACCGAGA TTCAGGCGCA GCTGGATGAC CTGGACCGCC 100
<210> 8686
<211> 100
<212> DNA
<213> Escherichia coli K-12 MG1655
<220> phnd b4106
<222> 855-954
<223> phnd b4106 U00096 complement(4320914..4321930)
<313> 8686: 884-903
<400> 8686
GCTCGCGCTG TTTAAAGAGA TGCAGGGCGT GAAAAGCAAT AAAGGACTGA 50
ATGAGCAGGA CAAGCTGGCA AAAACCACCG AGATTCAGGC GCAGCTGGAT 100
<210> 8687
<211> 100
<212> DNA
<213> Escherichia coli K-12 MG1655
<220> t150 b3559
<222> 522-621
<223> t150 b3559 U00096 3718827..3719678
<313> 8687: 548-567
<400> 8687
CAAAAAGCTT AATCCTCACG AGCATCCTGT TCTGCACTCT GACCAGGGAT 50
GGCAGTATCG TATGAGAAGA TATCAAAATA TCCTTAAAGA ACATGGTATT 100
<210> 8688
<211> 100
<212> DNA
<213> Escherichia coli K-12 MG1655
<220> t150 b3559

<222> 354-453
<223> t150 b3559 U00096 3718827..3719678
<313> 8688: 407-426
<400> 8688
TACGCGGCCA AACGAGAAGT GGGTTACCGA TGTTACTGAA TTTGCAGTCA 50
ATGGGCGCAA GCTGTATTTG TCTCCAGTAA TAGATCTCTT CAACAACGAA 100
<210> 8689
<211> 100
<212> DNA
<213> Escherichia coli K-12 MG1655
<220> t150 b3559
<222> 344-443
<223> t150 b3559 U00096 3718827..3719678
<313> 8689: 370-389
<400> 8689
ATTTCAAGGC TACGCGGCCA AACGAGAAGT GGGTTACCGA TGTTACTGAA 50
TTTGCAGTCA ATGGGCGCAA GCTGTATTTG TCTCCAGTAA TAGATCTCTT 100
<210> 8690
<211> 100
<212> DNA
<213> Escherichia coli K-12 MG1655
<220> cadc b4134
<222> 1427-1526
<223> cadc b4134 U00096 complement(4357974..4359512)
<313> 8690: 1446-1465
<400> 8690
CCTTTAATTT ACGCCCAGGG GCAAACACCC TTTACTGGAT TGAAAATGGT 50
ATATTCCAGA CTTCTGTTCC TTATGTTGTA CCTTATCTCG ACAAATTTCT 100
<210> 8691
<211> 100
<212> DNA
<213> Escherichia coli K-12 MG1655
<220> cadc b4134
<222> 1221-1320
<223> cadc b4134 U00096 complement(4357974..4359512)
<313> 8691: 1286-1305
<400> 8691
TGTTACACTG CCGGAATTGA ACAACCTGTC CATTATATAT CAAATAAAAG 50
CGGTCAGTGC TCTGGTAAAA GGTAAAACAG ATGAGTCTTA CCAGGCGATA 100
<210> 8692
<211> 100
<212> DNA
<213> Escherichia coli K-12 MG1655
<220> cadc b4134
<222> 1061-1160
<223> cadc b4134 U00096 complement(4357974..4359512)
<313> 8692: 1115-1134
<400> 8692
CATTGAACCG TGCCAGTGAA TTATTAGGTG AGATTGTTCA ATCATCCCCA 50
GAATTTACCT ACGCGAGAGC AGAAAAAGCA TTAGTTGATA TCGTGCGCCA 100
<210> 8693
<211> 100
<212> DNA
<213> Escherichia coli K-12 MG1655
<220> phne b4105
<222> 499-598
<223> phne b4105 U00096 complement(4320239..4320859)
<313> 8693: 546-565
<400> 8693
GCGTGCTCTC CAAGCTGCTT TCCGAAGCGG TGGAAGCGAT TGAGCCCGGC 50
CCGGTGGAAG GCATTCGCGC CACCGGTGCC AACAAGCTCG AAGAGATCCT 100
<210> 8694
<211> 100
<212> DNA
<213> Escherichia coli K-12 MG1655
<220> phne b4105
<222> 361-460
<223> phne b4105 U00096 complement(4320239..4320859)
<313> 8694: 404-423
<400> 8694
TACCAGCCCG TTCGCCGCCT GATGGACGCC TGCCGCGCCA TTAACGAAAT 50

GGTCTTCGCC ATGCTGTTCG TGGTCGCCGT CGGCCTCGGC CCGTTCGCTG 100
<210> 8695
<211> 100
<212> DNA
<213> Escherichia coli K-12 MG1655
<220> phne b4105
<222> 315-414
<223> phne b4105 U00096 complement(4320239..4320859)
<313> 8695: 370-389
<400> 8695
CCCCTTTGGC CTGATGAGCG CCGAAAACCT GGTGCCGTGG TGGGTTTACC 50
AGCCCGTTCG CCGCCTGATG GACGCCTGCC GCGCCATTAA CGAAATGGTC 100
<210> 8696
<211> 100
<212> DNA
<213> Escherichia coli K-12 MG1655
<220> phnf phnF
<222> 561-660
<223> phnf phnF U00096 complement(4319275..4320000)
<313> 8696: 603-622
<400> 8696
CGCCCGCCGC GCCCAGGCCA AAGAGTGCCA GCGTCTTGAA ATCCCGAATA 50
TGTCGCCGCT GCTGTGCGTG CGCACCCTTA ACCACCGTGA CGGTGAAAGC 100
<210> 8697
<211> 100
<212> DNA
<213> Escherichia coli K-12 MG1655
<220> phnf phnF
<222> 448-547
<223> phnf phnF U00096 complement(4319275..4320000)
<313> 8697: 477-496
<400> 8697
TTCGCGGACC TCACCCTCTG GCCGACGCTG CAACGCTTCG ACAGCGGCTC 50
GCTGCACGAT TTTCTGCGCG AGCAAACCGG AATTGCGCTG CGCCGCAGCC 100
<210> 8698
<211> 100
<212> DNA
<213> Escherichia coli K-12 MG1655
<220> phnf phnF
<222> 338-437
<223> phnf phnF U00096 complement(4319275..4320000)
<313> 8698: 367-386
<400> 8698
CGTCCGGCCA CGTCGCTGAC GCACTGGGGA TTACCGAGGG GGAGAACGTC 50
ATCCACCTGC GCACCCTGCG TCGTGTCAAC GGCGTCGCGC TCTGTTTAAT 100
<210> 8699
<211> 100
<212> DNA
<213> Escherichia coli K-12 MG1655
<220> phng b4102
<222> 332-431
<223> phng b4102 U00096 complement(4318822..4319274)
<313> 8699: 387-406
<400> 8699
TATCAGAAAC CCTTATTGCC CCGCTGGACG CTGACCGTAT GGCACGCATT 50
GCCGCACGCC AGGCCGAAGT GAACGCCAGC CGGGTCGACT TCTTTACGAT 100
<210> 8700
<211> 100
<212> DNA
<213> Escherichia coli K-12 MG1655
<220> phng b4102
<222> 286-385
<223> phng b4102 U00096 complement(4318822..4319274)
<313> 8700: 334-353
<400> 8700
GCGCTGATTG ACGCGCTGAT GCAGCAATCT CGCCACTTTC AAAACTTATC 50
AGAAACCCTT ATTGCCCCGC TGGACGCTGA CCGTATGGCA CGCATTGCCG 100
<210> 8701
<211> 100
<212> DNA
<213> Escherichia coli K-12 MG1655

<220> phng b4102
<222> 278-377
<223> phng b4102 U00096 complement(4318822..4319274)
<313> 8701: 307-326
<400> 8701
AACGCTGCGC GCTGATTGAC GCGCTGATGC AGCAATCTCG CCACTTTCAA  50
AACTTATCAG AAACCCTTAT TGCCCCGCTG GACGCTGACC GTATGGCACG  100
<210> 8702
<211> 100
<212> DNA
<213> Escherichia coli K-12 MG1655
<220> phnh b4101
<222> 486-585
<223> phnh b4101 U00096 complement(4318241..4318825)
<313> 8702: 552-571
<400> 8702
CGAGCGCCCG CATCCGTTCC CGCTCGGCAT CGACCTGATC CTGACCTGTG  50
GCGAGCGCCT GCTGGCTATT CCGCGAACCA CTCATGTGGA GGTGTGCTGA  100
<210> 8703
<211> 100
<212> DNA
<213> Escherichia coli K-12 MG1655
<220> phnh b4101
<222> 466-565
<223> phnh b4101 U00096 complement(4318241..4318825)
<313> 8703: 507-526
<400> 8703
TGCATTCTGC ACGAACTCAC CGAGCGCCCG CATCCGTTCC CGCTCGGCAT  50
CGACCTGATC CTGACCTGTG GCGAGCGCCT GCTGGCTATT CCGCGAACCA  100
<210> 8704
<211> 100
<212> DNA
<213> Escherichia coli K-12 MG1655
<220> phnh b4101
<222> 378-477
<223> phnh b4101 U00096 complement(4318241..4318825)
<313> 8704: 427-446
<400> 8704
GGTCGCCAGC CTGAGCGGCG GACGCATGTT GCGCCTTACT GGTGCGGGTA  50
TTGCCGAAGA ACGAATGATC GCTCCGCAGC TGCCGGAGTG CATTCTGCAC  100
<210> 8705
<211> 100
<212> DNA
<213> Escherichia coli K-12 MG1655
<220> phni b4100
<222> 966-1065
<223> phni b4100 U00096 complement(4317177..4318241)
<313> 8705: 1032-1051
<400> 8705
CGCAGGCTTT GTCTCACACC TCAAACTCCC CCACTACGTC GATTTCCAGG  50
CCGAACTGGA GCTACTCAAA CGTCTGCAAC AGGAGCAGAA CCATGGCTAA  100
<210> 8706
<211> 100
<212> DNA
<213> Escherichia coli K-12 MG1655
<220> phni b4100
<222> 965-1064
<223> phni b4100 U00096 complement(4317177..4318241)
<313> 8706: 987-1006
<400> 8706
CCGCAGGCTT TGTCTCACAC CTCAAACTCC CCCACTACGT CGATTTCCAG  50
GCCGAACTGG AGCTACTCAA ACGTCTGCAA CAGGAGCAGA ACCATGGCTA  100
<210> 8707
<211> 100
<212> DNA
<213> Escherichia coli K-12 MG1655
<220> phni b4100
<222> 869-968
<223> phni b4100 U00096 complement(4317177..4318241)
<313> 8707: 897-916
<400> 8707

TCGACCGCGC TCTGCAAGCC CCGGAGTACG GCGAGCACGC GACAGGCCCG 50
GCGCAGGATG AAGAGTTCGT GCTGGCACAT GCCGACAACG TCGAAGCCGC 100
<210> 8708
<211> 100
<212> DNA
<213> Escherichia coli K-12 MG1655
<220> phnj b4099
<222> 747-846
<223> phnj b4099 U00096 complement(4316339..4317184)
<313> 8708: 819-838
<400> 8708
CTATCTTGAT GAAGTGGTGC TGGATGACGC CGGAAACCGC ATGTTTGTCT 50
GCTCCGATAC CGATTATTGC CGCCAACAGA GCGAGGCAAA AAACCAATGA 100
<210> 8709
<211> 100
<212> DNA
<213> Escherichia coli K-12 MG1655
<220> phnj b4099
<222> 720-819
<223> phnj b4099 U00096 complement(4316339..4317184)
<313> 8709: 783-802
<400> 8709
ATGCGCCATC TGCGGATCGA CCCACAGCTA TCTTGATGAA GTGGTGCTGG 50
ATGACGCCGG AAACCGCATG TTTGTCTGCT CCGATACCGA TTATTGCCGC 100
<210> 8710
<211> 100
<212> DNA
<213> Escherichia coli K-12 MG1655
<220> phnj b4099
<222> 675-774
<223> phnj b4099 U00096 complement(4316339..4317184)
<313> 8710: 698-717
<400> 8710
CGATTTCGAC GATCACCCGT TCACCGTTCA GCAGTGGGAT GAGCCATGCG 50
CCATCTGCGG ATCGACCCAC AGCTATCTTG ATGAAGTGGT GCTGGATGAC 100
<210> 8711
<211> 100
<212> DNA
<213> Escherichia coli K-12 MG1655
<220> phnk b4098
<222> 660-759
<223> phnk b4098 U00096 complement(4315584..4316342)
<313> 8711: 710-729
<400> 8711
GCAGGGGCAA GTGGTGGAGA GTGGGTTAAC CGACCGCGTG CTCGACGACC 50
CGCATCATCC GTATACACAG CTGCTGGTGT CATCGGTTTT GCAGAATTGA 100
<210> 8712
<211> 100
<212> DNA
<213> Escherichia coli K-12 MG1655
<220> phnk b4098
<222> 606-705
<223> phnk b4098 U00096 complement(4315584..4316342)
<313> 8712: 652-671
<400> 8712
CACCCATGAT TTAGGCGTCG CCCGCCTGCT GGCGGACCGT TTGCTGGTGA 50
TGAAGCAGGG GCAAGTGGTG GAGAGTGGGT TAACCGACCG CGTGCTCGAC 100
<210> 8713
<211> 100
<212> DNA
<213> Escherichia coli K-12 MG1655
<220> phnk b4098
<222> 528-627
<223> phnk b4098 U00096 complement(4315584..4316342)
<313> 8713: 576-595
<400> 8713
GGATGTGTCG GTGCAGGCCC GCCTGCTCGA CCTGCTGCGC GGCCTGGTGG 50
TGGAGCTGAA CCTCGCGGTG GTGATTGTCA CCCATGATTT AGGCGTCGCC 100
<210> 8714
<211> 100
<212> DNA

<213> Escherichia coli K-12 MG1655
<220> phnl b4097
<222> 556-655
<223> phnl b4097 U00096 complement(4314793..4315473)
<313> 8714: 618-637
<400> 8714
GCCGCGGTGG TGGAACTGAT TCGCGAAGCC AAAACCCGTG GCGCAGCCAT 50
CGTAGGCATC TTCCATGACG AAGCTGTACG TAATGACGTC GCCGACCGCC 100
<210> 8715
<211> 100
<212> DNA
<213> Escherichia coli K-12 MG1655
<220> phnl b4097
<222> 479-578
<223> phnl b4097 U00096 complement(4314793..4315473)
<313> 8715: 530-549
<400> 8715
TCGCCCGCGG CTTTATCGTC GACTACCCCA TTCTGCTGCT TGACGAACCT 50
ACCGCCTCGC TGGACGCCAA AAACAGCGCC GCGGTGGTGG AACTGATTCG 100
<210> 8716
<211> 100
<212> DNA
<213> Escherichia coli K-12 MG1655
<220> phnl b4097
<222> 434-533
<223> phnl b4097 U00096 complement(4314793..4315473)
<313> 8716: 458-477
<400> 8716
TGGCACCATC GACATTTTCC GGTGGCGAAC AGCAGCGCGT CAACATCGCC 50
CGCGGCTTTA TCGTCGACTA CCCCATTCTG CTGCTTGACG AACCTACCGC 100
<210> 8717
<211> 100
<212> DNA
<213> Escherichia coli K-12 MG1655
<220> phnm b4096
<222> 1035-1134
<223> phnm b4096 U00096 complement(4313660..4314796)
<313> 8717: 1086-1105
<400> 8717
GGTGATTGGC GAGGGCAAAC GCGCCGACCT GGTGCTGGCG CATCGCAAGG 50
ACAATCATAT TCATATCGAC CACGTCTGGC GTCAGGGTAA AAGGGTGTTC 100
<210> 8718
<211> 100
<212> DNA
<213> Escherichia coli K-12 MG1655
<220> phnm b4096
<222> 981-1080
<223> phnm b4096 U00096 complement(4313660..4314796)
<313> 8718: 1031-1050
<400> 8718
GGTGAAGCTG GTGACTAAAA ATCCAGCGCA GGCGCTTAAT CTCCAGGATC 50
GCGGGGTGAT TGGCGAGGGC AAACGCGCCG ACCTGGTGCT GGCGCATCGC 100
<210> 8719
<211> 100
<212> DNA
<213> Escherichia coli K-12 MG1655
<220> phnm b4096
<222> 944-1043
<223> phnm b4096 U00096 complement(4313660..4314796)
<313> 8719: 992-1011
<400> 8719
CCGATGACCA GAGCAACCGC TTTACGCTGC CGCAGGCGGT GAAGCTGGTG 50
ACTAAAAATC CAGCGCAGGC GCTTAATCTC CAGGATCGCG GGGTGATTGG 100
<210> 8720
<211> 100
<212> DNA
<213> Escherichia coli K-12 MG1655
<220> phnn b4095
<222> 459-558
<223> phnn b4095 U00096 complement(4313103..4313660)
<313> 8720: 524-543

<400> 8720
GGATTGCCAT ACGCTCAACA ATGACGGCAG CCTGCGCCAG TCGGTCGACA 50
CGCTGCTGAC GCTGATCCAT CAGAAGGAGA AACACCATGC CTGCTTGTGA 100
<210> 8721
<211> 100
<212> DNA
<213> Escherichia coli K-12 MG1655
<220> phnn b4095
<222> 426-525
<223> phnn b4095 U00096 complement(4313103..4313660)
<313> 8721: 479-498
<400> 8721
CCGCCTGGCG CGCGCCGCCC GCTATACTCC ACAGGATTGC CATACGCTCA 50
ACAATGACGG CAGCCTGCGC CAGTCGGTCG ACACGCTGCT GACGCTGATC 100
<210> 8722
<211> 100
<212> DNA
<213> Escherichia coli K-12 MG1655
<220> phnn b4095
<222> 329-428
<223> phnn b4095 U00096 complement(4313103..4313660)
<313> 8722: 369-388
<400> 8722
AATCGGCGCT GCTGCCCGTC TGTTTACAGG TTTCGCCGGA GATCCTCCGC 50
CAGCGCCTGG AAAACCGTGG CCGTGAAAAC GCCAGTGAAA TTAACGCCCG 100
<210> 8723
<211> 100
<212> DNA
<213> Escherichia coli K-12 MG1655
<220> phno b4094
<222> 336-435
<223> phno b4094 U00096 complement(4312682..4313116)
<313> 8723: 413-432
<400> 8723
GACCGAACTT TCGACCAACG TGAAGCGCCA CGACGCGCAC CGTTTCTATC 50
TGCGCGAAGG CTACGAGCAG AGCCACTTCC GCTTCACCAA GGCGCTGTAA 100
<210> 8724
<211> 100
<212> DNA
<213> Escherichia coli K-12 MG1655
<220> phno b4094
<222> 305-404
<223> phno b4094 U00096 complement(4312682..4313116)
<313> 8724: 357-376
<400> 8724
AAGAAGAAGC CCGCCAGGCC GGGGCCGAAA TGACCGAACT TTCGACCAAC 50
GTGAAGCGCC ACGACGCGCA CCGTTTCTAT CTGCGCGAAG GCTACGAGCA 100
<210> 8725
<211> 100
<212> DNA
<213> Escherichia coli K-12 MG1655
<220> phno b4094
<222> 285-384
<223> phno b4094 U00096 complement(4312682..4313116)
<313> 8725: 321-340
<400> 8725
TAAGTTACTG GCGTGGGCAG AAGAAGAAGC CCGCCAGGCC GGGGCCGAAA 50
TGACCGAACT TTCGACCAAC GTGAAGCGCC ACGACGCGCA CCGTTTCTAT 100
<210> 8726
<211> 100
<212> DNA
<213> Escherichia coli K-12 MG1655
<220> phnp b4093
<222> 660-759
<223> phnp b4093 U00096 complement(4311922..4312680)
<313> 8726: 734-753
<400> 8726
GACCCATATC AGCCACCAGT TTGATGCGTG GCTGATGGAA AACGCACTAC 50
CGTCAGGGTT TGAGGTGGGG TTTGATGGGA TGGAGATTGG GGTGGCGTGA 100
<210> 8727
<211> 100

<212> DNA
<213> Escherichia coli K-12 MG1655
<220> phnp b4093
<222> 632-731
<223> phnp b4093 U00096 complement(4311922..4312680)
<313> 8727: 688-707
<400> 8727
AGGTTATCCG CTCGCCACGG GTGATTCTGA CCCATATCAG CCACCAGTTT 50
GATGCGTGGC TGATGGAAAA CGCACTACCG TCAGGGTTTG AGGTGGGGTT 100
<210> 8728
<211> 100
<212> DNA
<213> Escherichia coli K-12 MG1655
<220> phnp b4093
<222> 595-694
<223> phnp b4093 U00096 complement(4311922..4312680)
<313> 8728: 626-645
<400> 8728
AATCACTGTG ATTTAAATAC CGTGCTTGCG CTGAATCAGG TTATCCGCTC 50
GCCACGGGTG ATTCTGACCC ATATCAGCCA CCAGTTTGAT GCGTGGCTGA 100
<210> 8729
<211> 100
<212> DNA
<213> Escherichia coli K-12 MG1655
<220> phnq b4092
<222> 277-376
<223> phnq b4092 U00096 complement(4311389..4311796)
<313> 8729: 318-337
<400> 8729
GATAATATCG GCCTGGGCCG GAACGTGGAC AGCGTGAGCA GGGTGAATAA 50
AAGAAATAGC GGAAAGCGTT TCATTAGCCA ACCTCAAAAA GAAACTCTAT 100
<210> 8730
<211> 100
<212> DNA
<213> Escherichia coli K-12 MG1655
<220> phnq b4092
<222> 180-279
<223> phnq b4092 U00096 complement(4311389..4311796)
<313> 8730: 222-241
<400> 8730
GCTGCCAGCC GTCGTCGCGC GAATCTTCAT AGTCTCGCCC ACGGTCAGGG 50
TTATAAGCGT CATTAATCGC CTGCTGAATA TTGCCAATGG TGTCGTCGAT 100
<210> 8731
<211> 100
<212> DNA
<213> Escherichia coli K-12 MG1655
<220> phnq b4092
<222> 9-108
<223> phnq b4092 U00096 complement(4311389..4311796)
<313> 8731: 65-84
<400> 8731
TCCATACCCA ACCTCATCGC CCATACTCAT CTTCCATTCT CCGCTCTTCA 50
TCCTCCAGTT GCCGACGCTC CTGATCAAGC TGGCGCTGGC GATCGTCCAG 100
<210> 8732
<211> 100
<212> DNA
<213> Escherichia coli K-12 MG1655
<220> rna b0612
<222> 708-807
<223> rna b0612 U00096 complement(643420..644226)
<313> 8732: 772-791
<400> 8732
TGACGCCATC AACGCTCCGC TTTCTGCAAA CTCATTCTTG CCACAACCTC 50
ACCCAGGTAA CTGTGGCAAA ACCTTTGTGA TTGATAAAGC GGGTTATTAA 100
<210> 8733
<211> 100
<212> DNA
<213> Escherichia coli K-12 MG1655
<220> rna b0612
<222> 682-781
<223> rna b0612 U00096 complement(643420..644226)

<313> 8733: 737-756
<400> 8733
ACTGAAATTC AGATCTCGAT CAAAGCTGAC GCCATCAACG CTCCGCTTTC 50
TGCAAACTCA TTCTTGCCAC AACCTCACCC AGGTAACTGT GGCAAAACCT 100
<210> 8734
<211> 100
<212> DNA
<213> Escherichia coli K-12 MG1655
<220> rna b0612
<222> 598-697
<223> rna b0612 U00096 complement(643420..644226)
<313> 8734: 638-657
<400> 8734
TTTGACGCCG CCTTTGCCAA AAGCTGGGGA AAAGAGAACG TGAAAGCAGT 50
TAAGCTAACG TGCCAGGGTA ACCCTGCGTA TTTGACTGAA ATTCAGATCT 100
<210> 8735
<211> 100
<212> DNA
<213> Escherichia coli K-12 MG1655
<220> cls b1250
<222> 1362-1461
<223> cls b1250 U00096 complement(1305209..1306669)
<313> 8735: 1423-1442
<400> 8735
TTCGCGTTCA CGTCTGCTCG ATGCCCGTTT ATGGCTAAAA CGTCCATTAT 50
GGCAACGTGT CGCCGAGCGA CTGTTTTACT TCTTCAGTCC GTTGCTGTAA 100
<210> 8736
<211> 100
<212> DNA
<213> Escherichia coli K-12 MG1655
<220> cls b1250
<222> 1339-1438
<223> cls b1250 U00096 complement(1305209..1306669)
<313> 8736: 1384-1403
<400> 8736
GCCGCCGTTC AGGACGATTA TATTTCGCGT TCACGTCTGC TCGATGCCCG 50
TTTATGGCTA AAACGTCCAT TATGGCAACG TGTCGCCGAG CGACTGTTTT 100
<210> 8737
<211> 100
<212> DNA
<213> Escherichia coli K-12 MG1655
<220> cls b1250
<222> 1310-1409
<223> cls b1250 U00096 complement(1305209..1306669)
<313> 8737: 1352-1371
<400> 8737
TCGACGATAA AGGTTTTGGT GCTGACCTCG CCGCCGTTCA GGACGATTAT 50
ATTTCGCGTT CACGTCTGCT CGATGCCCGT TTATGGCTAA AACGTCCATT 100
<210> 8738
<211> 100
<212> DNA
<213> Escherichia coli K-12 MG1655
<220> rnb b1287
<222> 1836-1935
<223> rnb b1287 U00096 complement(1345002..1346936)
<313> 8738: 1891-1910
<400> 8738
TAAAGGTGAA ACGGTTTACA AAGTGACTGA CGTTATTGAC GTCACTATTG 50
CCGAAGTCCG CATGGAAACC CGCAGCATTA TTGCGCGCCC GGTCGCGTAA 100
<210> 8739
<211> 100
<212> DNA
<213> Escherichia coli K-12 MG1655
<220> rnb b1287
<222> 1760-1859
<223> rnb b1287 U00096 complement(1345002..1346936)
<313> 8739: 1814-1833
<400> 8739
CCTTTATTCC GGCACCTTTC TTACACGCTG TGCGCGATGA ACTGGTTTGC 50
AGCCAGGAAA ACGGCACTGT ACAAATTAAA GGTGAAACGG TTTACAAAGT 100
<210> 8740

<211> 100
<212> DNA
<213> Escherichia coli K-12 MG1655
<220> rnb b1287
<222> 1741-1840
<223> rnb b1287 U00096 complement(1345002..1346936)
<313> 8740: 1764-1783
<400> 8740
GTTGATAACG GCGCTATCGC CTTTATTCCG GCACCTTTCT TACACGCTGT 50
GCGCGATGAA CTGGTTTGCA GCCAGGAAAA CGGCACTGTA CAAATTAAAG 100
<210> 8741
<211> 100
<212> DNA
<213> Escherichia coli K-12 MG1655
<220> rnc b2568
<222> 582-681
<223> rnc b2568 U00096 complement(2701403..2702083)
<313> 8741: 660-679
<400> 8741
CAGCGGCCTG AGTGAACCGG TGGTTGGCAC AGGTTCAAGC CGTCGTAAGG 50
CTGAGCAGGC TGCCGCCGAA CAGGCGTTGA AAAAACTGGA GCTGGAATGA 100
<210> 8742
<211> 100
<212> DNA
<213> Escherichia coli K-12 MG1655
<220> rnc b2568
<222> 578-677
<223> rnc b2568 U00096 complement(2701403..2702083)
<313> 8742: 610-629
<400> 8742
AGGTCAGCGG CCTGAGTGAA CCGGTGGTTG GCACAGGTTC AAGCCGTCGT 50
AAGGCTGAGC AGGCTGCCGC CGAACAGGCG TTGAAAAAAC TGGAGCTGGA 100
<210> 8743
<211> 100
<212> DNA
<213> Escherichia coli K-12 MG1655
<220> rnc b2568
<222> 518-617
<223> rnc b2568 U00096 complement(2701403..2702083)
<313> 8743: 556-575
<400> 8743
CTTATCTGGT AGTCCAGGTA CGTGGCGAAG CGCACGATCA GGAATTTACT 50
ATCCACTGCC AGGTCAGCGG CCTGAGTGAA CCGGTGGTTG GCACAGGTTC 100
<210> 8744
<211> 100
<212> DNA
<213> Escherichia coli K-12 MG1655
<220> rnd b1805
<222> 1029-1128
<223> rnd b1805 U00096 complement(1884888..1886015)
<313> 8744: 1097-1116
<400> 8744
ACTGAAACCG CAGAACAATT TGCCGGAGCT GATTTCCGGC TGGCGTGGTG 50
AGCTGATGGC GGAAGCATTA CACAATTTAT TGCAGGAATA TCCGCAGTAA 100
<210> 8745
<211> 100
<212> DNA
<213> Escherichia coli K-12 MG1655
<220> rnd b1805
<222> 994-1093
<223> rnd b1805 U00096 complement(1884888..1886015)
<313> 8745: 1034-1053
<400> 8745
CGGCAAATCA ACCAACTGCT GAACTGGCAC TGGAAACTGA AACCGCAGAA 50
CAATTTGCCG GAGCTGATTT CCGGCTGGCG TGGTGAGCTG ATGGCGGAAG 100
<210> 8746
<211> 100
<212> DNA
<213> Escherichia coli K-12 MG1655
<220> rnd b1805
<222> 955-1054

<223> rnd b1805 U00096 complement(1884888..1886015)
<313> 8746: 991-1010
<400> 8746
GAAACGCATA AGATCAGCGC CGAATTGCTG GCATCGCGTC GGCAAATCAA 50
CCAACTGCTG AACTGGCACT GGAAACTGAA ACCGCAGAAC AATTTGCCGG 100
<210> 8747
<211> 100
<212> DNA
<213> Escherichia coli K-12 MG1655
<220> rne rne
<222> 3087-3186
<223> rne rne U00096 complement(1140405..1143590)
<313> 8747: 3156-3175
<400> 8747
GCGCCCTACT TTTGCCTTCG AAGGTAAAGG TGCCGCAGGT GGTCATACGG 50
CAACACATCA TGCCTCTGCC GCTCCTGCGC GTCCGCAACC TGTTGAGTAA 100
<210> 8748
<211> 100
<212> DNA
<213> Escherichia coli K-12 MG1655
<220> rne rne
<222> 3081-3180
<223> rne rne U00096 complement(1140405..1143590)
<313> 8748: 3125-3144
<400> 8748
CTGGCAGCGC CCTACTTTTG CCTTCGAAGG TAAAGGTGCC GCAGGTGGTC 50
ATACGGCAAC ACATCATGCC TCTGCCGCTC CTGCGCGTCC GCAACCTGTT 100
<210> 8749
<211> 100
<212> DNA
<213> Escherichia coli K-12 MG1655
<220> rne rne
<222> 3048-3147
<223> rne rne U00096 complement(1140405..1143590)
<313> 8749: 3069-3088
<400> 8749
GGAATATGTT CCGGAGGCAC CGCGTCACAG TGACTGGCAG CGCCCTACTT 50
TTGCCTTCGA AGGTAAAGGT GCCGCAGGTG GTCATACGGC AACACATCAT 100
<210> 8750
<211> 100
<212> DNA
<213> Escherichia coli K-12 MG1655
<220> phoa b0384
<222> 1386-1485
<223> phoa b0384 U00096 400902..402386
<313> 8750: 1445-1464
<400> 8750
GCGTATTGCG GCGTATGGCC CGCATGCCGC CAATGTTGTT GGACTGACCG 50
ACCAGACCGA TCTCTTCTAC ACCATGAAAG CCGCTCTGGG GCTGAAATAA 100
<210> 8751
<211> 100
<212> DNA
<213> Escherichia coli K-12 MG1655
<220> phoa b0384
<222> 1345-1444
<223> phoa b0384 U00096 400902..402386
<313> 8751: 1386-1405
<400> 8751
AACTCCGAAG AGGATTCACA AGAACATACC GGCAGTCAGT TGCGTATTGC 50
GGCGTATGGC CCGCATGCCG CCAATGTTGT TGGACTGACC GACCAGACCG 100
<210> 8752
<211> 100
<212> DNA
<213> Escherichia coli K-12 MG1655
<220> phoa b0384
<222> 1301-1400
<223> phoa b0384 U00096 400902..402386
<313> 8752: 1347-1366
<400> 8752
CGCTAAATAC CAAAGATGGC GCAGTGATGG TGATGAGTTA CGGGAACTCC 50
GAAGAGGATT CACAAGAACA TACCGGCAGT CAGTTGCGTA TTGCGGCGTA 100

<210> 8753
<211> 100
<212> DNA
<213> Escherichia coli K-12 MG1655
<220> phob b0400
<222> 565-664
<223> phob b0400 U00096 416366..417055
<313> 8753: 606-625
<400> 8753
TATGTGGAAG ACCGCACGGT CGATGTCCAC ATTCGTCGCC TGCGTAAAGC  50
ACTGGAGCCC GGCGGGCATG ACCGCATGGT GCAGACCGTG CGCGGTACAG  100
<210> 8754
<211> 100
<212> DNA
<213> Escherichia coli K-12 MG1655
<220> phob b0400
<222> 432-531
<223> phob b0400 U00096 416366..417055
<313> 8754: 477-496
<400> 8754
CCGAGTGATG GCGGGCGAAG AGCCGCTGGA GATGGGGCCG ACAGAATTTA  50
AACTGCTGCA CTTTTTTATG ACGCATCCTG AGCGCGTGTA CAGCCGCGAG  100
<210> 8755
<211> 100
<212> DNA
<213> Escherichia coli K-12 MG1655
<220> phob b0400
<222> 407-506
<223> phob b0400 U00096 416366..417055
<313> 8755: 437-456
<400> 8755
GATTAAGTCT CGACCCGACA TCTCACCGAG TGATGGCGGG CGAAGAGCCG  50
CTGGAGATGG GGCCGACAGA ATTTAAACTG CTGCACTTTT TTATGACGCA  100
<210> 8756
<211> 100
<212> DNA
<213> Escherichia coli K-12 MG1655
<220> rnk b0611
<222> 312-411
<223> rnk b0611 U00096 complement(642780..643190)
<313> 8756: 363-382
<400> 8756
CGATTCCATT CACTGGGAAC TTCCGGGCGG CGTTGCAACC CACCTTGAAG  50
TGCTGGAACT CGAATACCAG CCAGAAGCTG CTGGCGACTA CCTGCTTTAA  100
<210> 8757
<211> 100
<212> DNA
<213> Escherichia coli K-12 MG1655
<220> rnk b0611
<222> 227-326
<223> rnk b0611 U00096 complement(642780..643190)
<313> 8757: 271-290
<400> 8757
TGTATCCGGC AAAAATGACC GATAGCAATA CTCAGCTTTC CGTTATGGCT  50
CCGGTAGGTG CCGCACTGCT GGGGCTGCGC GTTGGCGATT CCATTCACTG  100
<210> 8758
<211> 100
<212> DNA
<213> Escherichia coli K-12 MG1655
<220> rnk b0611
<222> 144-243
<223> rnk b0611 U00096 complement(642780..643190)
<313> 8758: 188-207
<400> 8758
GATGCCACAC GACGTGGTGA CAATGAACAG CCGGGTTAAA TTCCGCAATC  50
TTAGCGATGG CGAAGTGCGT GTGCGCACGC TGGTGTATCC GGCAAAAATG  100
<210> 8759
<211> 100
<212> DNA
<213> Escherichia coli K-12 MG1655
<220> phoe b0242

<222> 810-909
<223> phoe b0242 U00096 complement(258269..259324)
<313> 8759: 847-866
<400> 8759
GGTCGCTCAA TACCAGTTTG ACTTTGGTCT GCGTCCATCG CTGGGTTATG 50
TCTTATCGAA AGGGAAAGAT ATTGAAGGTA TCGGTGATGA AGATCTGGTC 100
<210> 8760
<211> 100
<212> DNA
<213> Escherichia coli K-12 MG1655
<220> phoe b0242
<222> 723-822
<223> phoe b0242 U00096 complement(258269..259324)
<313> 8760: 768-787
<400> 8760
TATTTATCTG GCAACTTTCT ATTCTGAAAC ACGCAAAATG ACGCCAATAA 50
CTGGCGGCTT TGCCAATAAG ACACAGAACT TTGAAGCGGT CGCTCAATAC 100
<210> 8761
<211> 100
<212> DNA
<213> Escherichia coli K-12 MG1655
<220> phoe b0242
<222> 652-751
<223> phoe b0242 U00096 complement(258269..259324)
<313> 8761: 667-686
<400> 8761
AACCTGCAAA GCCGTGGCAC AGGCAAGCGT GCAGAAGCAT GGGCAACAGG 50
TCTGAAATAC GATGCCAATA ATATTTATCT GGCAACTTTC TATTCTGAAA 100
<210> 8762
<211> 100
<212> DNA
<213> Escherichia coli K-12 MG1655
<220> phoh b1021
<222> 960-1059
<223> phoh b1021 U00096 1084215..1085279
<313> 8762: 998-1017
<400> 8762
GCTGGAACGT TTCGAAGAAG ATGAAATGGT TGGGATCGTC CGGTTCGGTA 50
AAGAGGACTG CGTACGTTCG GCACTTTGCC AACGTACGCT GCATGCCTAC 100
<210> 8763
<211> 100
<212> DNA
<213> Escherichia coli K-12 MG1655
<220> phoh b1021
<222> 801-900
<223> phoh b1021 U00096 1084215..1085279
<313> 8763: 837-856
<400> 8763
TGAAAATGCA GTCGTCATTC TTGACGAGGC GCAGAATGTG ACTGCCGCGC 50
AAATGAAAAT GTTTTTAACC CGCCTCGGGG AGAACGTGAC GGTTATCGTC 100
<210> 8764
<211> 100
<212> DNA
<213> Escherichia coli K-12 MG1655
<220> phoh b1021
<222> 767-866
<223> phoh b1021 U00096 1084215..1085279
<313> 8764: 797-816
<400> 8764
TTGCGCCGTT CGCCTATATG CGTGGACGTA CCTTTGAAAA TGCAGTCGTC 50
ATTCTTGACG AGGCGCAGAA TGTGACTGCC GCGCAAATGA AAATGTTTTT 100
<210> 8765
<211> 100
<212> DNA
<213> Escherichia coli K-12 MG1655
<220> b1085 b1086
<222> 233-332
<223> b1085 b1086 U00096 1143671..1144045
<313> 8765: 288-307
<400> 8765
TGGGTTATTC CGTAAAATTT CTTGTTTTAA CAAGGATGGA CACGGAAACG 50

GCCTCATTAT TCCACTGCAA GCCTTGTTAT AGCAAGATGA CTTTTACCAT 100
<210> 8766
<211> 100
<212> DNA
<213> Escherichia coli K-12 MG1655
<220> b1085 b1086
<222> 155-254
<223> b1085 b1086 U00096 1143671..1144045
<313> 8766: 181-200
<400> 8766
GCAACCCGCA GCCCGCTAAC TGCCTGAAAG ATCAATACGT CTTACGCCAT 50
TGCTGCGTGG ATGATCGGTC GGGCAAAATG GGTTATTCCG TAAAATTTCT 100
<210> 8767
<211> 100
<212> DNA
<213> Escherichia coli K-12 MG1655
<220> b1085 b1086
<222> 64-163
<223> b1085 b1086 U00096 1143671..1144045
<313> 8767: 92-111
<400> 8767
GCCCGTTTTT CATGCGGAAA AACAGCGCAA TTATCAAAGA AACAGACTGG 50
GTATTACTCT CCAGAAATAT TTCCATCTAC CGGTAAGGAC TGCAACCCGC 100
<210> 8768
<211> 100
<212> DNA
<213> Escherichia coli K-12 MG1655
<220> b0359 b0360
<222> 345-444
<223> b0359 b0360 U00096 complement(380068..380511)
<313> 8768: 412-431
<400> 8768
TTCCCCGAAC ACGCAACTTG GCCCGCGCGT GATTGTAGAA CGTAATTTAC 50
CTACCGGAAC TTATTCACTC CGACAAGAAC TTATCCGTAC AGGAGATTAA 100
<210> 8769
<211> 100
<212> DNA
<213> Escherichia coli K-12 MG1655
<220> b0359 b0360
<222> 266-365
<223> b0359 b0360 U00096 complement(380068..380511)
<313> 8769: 299-318
<400> 8769
GATGCGATAA ATTAGGTTGT TATATCGGGC AGCGTTCACG CCTTGGCGTA 50
CAAGTTATTA TTTTGCCTGG GCGAATTATT TCCCCGAACA CGCAACTTGG 100
<210> 8770
<211> 100
<212> DNA
<213> Escherichia coli K-12 MG1655
<220> b0359 b0360
<222> 109-208
<223> b0359 b0360 U00096 complement(380068..380511)
<313> 8770: 170-189
<400> 8770
GTTATTGAAG CGGAAGCAAC GATTGGTCCG CAATGTTTTA TTGCCGACTC 50
GGTAGTTGCA AACCAGGCAT ATTTAGGCGC ACAAGTACGT ACCAGTAATC 100
<210> 8771
<211> 100
<212> DNA
<213> Escherichia coli K-12 MG1655
<220> rnt b1653
<222> 547-646
<223> rnt b1653 U00096 1726371..1727018
<313> 8771: 600-619
<400> 8771
GCGCTGTACG ACACCGAACG CACTGCTGTG CTGTTTTGTG AAATCGTCAA 50
CCGCTGGAAA CGTCTGGGAG GCTGGCCGCT ATCTGCCGCC GAAGAGGTGT 100
<210> 8772
<211> 100
<212> DNA
<213> Escherichia coli K-12 MG1655

<220> rnt b1653
<222> 523-622
<223> rnt b1653 U00096 1726371..1727018
<313> 8772: 557-576
<400> 8772
TTCGACAGCA CCCAGGCGCA CTCCGCGCTG TACGACACCG AACGCACTGC 50
TGTGCTGTTT TGTGAAATCG TCAACCGCTG GAAACGTCTG GGAGGCTGGC 100
<210> 8773
<211> 100
<212> DNA
<213> Escherichia coli K-12 MG1655
<220> rnt b1653
<222> 464-563
<223> rnt b1653 U00096 1726371..1727018
<313> 8773: 491-510
<400> 8773
CCGGGCTGGC ACTCGGACAA ACCGTATTGT CAAAGGCTTG CCAGACCGCT 50
GGCATGGACT TCGACAGCAC CCAGGCGCAC TCCGCGCTGT ACGACACCGA 100
<210> 8774
<211> 100
<212> DNA
<213> Escherichia coli K-12 MG1655
<220> phop b1131
<222> 458-557
<223> phop b1131 U00096 complement(1188999..1189670)
<313> 8774: 480-499
<400> 8774
TCGAATACAC TATTATGGAA ACGTTGATAC GCAATAATGG CAAAGTGGTC 50
AGCAAAGATT CGTTAATGCT CCAACTCTAT CCGGATGCGG AGCTGCGGGA 100
<210> 8775
<211> 100
<212> DNA
<213> Escherichia coli K-12 MG1655
<220> phop b1131
<222> 322-421
<223> phop b1131 U00096 complement(1188999..1189670)
<313> 8775: 367-386
<400> 8775
GTGATGGCGC GAATGCAGGC ATTAATGCGG CGTAATAGCG GTCTGGCTTC 50
ACAGGTCATT TCGCTCCCCC CGTTTCAGGT TGATCTCTCT CGCCGTGAAT 100
<210> 8776
<211> 100
<212> DNA
<213> Escherichia coli K-12 MG1655
<220> phop b1131
<222> 296-395
<223> phop b1131 U00096 complement(1188999..1189670)
<313> 8776: 321-340
<400> 8776
TGACTAAACC GTTTCATATT GAAGAGGTGA TGGCGCGAAT GCAGGCATTA 50
ATGCGGCGTA ATAGCGGTCT GGCTTCACAG GTCATTTCGC TCCCCCCGTT 100
<210> 8777
<211> 100
<212> DNA
<213> Escherichia coli K-12 MG1655
<220> glcb b2977
<222> 2073-2172
<223> glcb b2977 U00096 complement(3119650..3121821)
<313> 8777: 2131-2150
<400> 8777
AGCTGCCAGC GATTTAATCT TCCTCGGCGT GAAACAGCCA AACGGCTATA 50
CCGAACCGTT ATTACACGCC TGGCGTTTAC GCGAAAAAGA AAGTCATTAA 100
<210> 8778
<211> 100
<212> DNA
<213> Escherichia coli K-12 MG1655
<220> glcb b2977
<222> 2073-2172
<223> glcb b2977 U00096 complement(3119650..3121821)
<313> 8778: 2106-2125
<400> 8778

AGCTGCCAGC GATTTAATCT TCCTCGGCGT GAAACAGCCA AACGGCTATA 50
CCGAACCGTT ATTACACGCC TGGCGTTTAC GCGAAAAAGA AAGTCATTAA 100
<210> 8779
<211> 100
<212> DNA
<213> Escherichia coli K-12 MG1655
<220> glcb b2977
<222> 2022-2121
<223> glcb b2977 U00096 complement(3119650..3121821)
<313> 8779: 2066-2085
<400> 8779
TCCGGCTTAT CGTCCGATGG CGGGGAATTT CGCTAACTCG TGTGCTTTTA 50
AAGCTGCCAG CGATTTAATC TTCCTCGGCG TGAAACAGCC AAACGGCTAT 100
<210> 8780
<211> 100
<212> DNA
<213> Escherichia coli K-12 MG1655
<220> phoq b1130
<222> 1355-1454
<223> phoq b1130 U00096 complement(1187539..1188999)
<313> 8780: 1418-1437
<400> 8780
TCACCGAGCA ATATGAGGGT AAAATCGTCG CCGGAGAGAG CATGCTGGGC 50
GGTGCGCGGA TGGAGGTGAT TTTTGGTCGC CAGCATTCTG CGCCGAAAGA 100
<210> 8781
<211> 100
<212> DNA
<213> Escherichia coli K-12 MG1655
<220> phoq b1130
<222> 1340-1439
<223> phoq b1130 U00096 complement(1187539..1188999)
<313> 8781: 1364-1383
<400> 8781
CGGTAGCCCG CGAAATCACC GAGCAATATG AGGGTAAAAT CGTCGCCGGA 50
GAGAGCATGC TGGGCGGTGC GCGGATGGAG GTGATTTTTG GTCGCCAGCA 100
<210> 8782
<211> 100
<212> DNA
<213> Escherichia coli K-12 MG1655
<220> phoq b1130
<222> 1210-1309
<223> phoq b1130 U00096 complement(1187539..1188999)
<313> 8782: 1258-1277
<400> 8782
CAAACCGACG AGCATCTCTA TATTGTGGTC GAGGATGATG GCCCCGGTAT 50
TCCATTAAGC AAGCGAGAGG TCATTTTCGA CCGTGGTCAA CGGGTTGATA 100
<210> 8783
<211> 100
<212> DNA
<213> Escherichia coli K-12 MG1655
<220> glcc glcC
<222> 630-729
<223> glcc glcC U00096 3126287..3127051
<313> 8783: 672-691
<400> 8783
GCAGCGGCTG CCGCACGTCG CCCAGCGCGC AGCACGCGAT CATGTGCGGA 50
CCGTGAAAAA GAATCTCCAC GATATCGAGC TGGAAGGCCA CCATTTGATT 100
<210> 8784
<211> 100
<212> DNA
<213> Escherichia coli K-12 MG1655
<220> glcc glcC
<222> 513-612
<223> glcc glcC U00096 3126287..3127051
<313> 8784: 565-584
<400> 8784
ATCATTGACC GATCTGATGT TTAATTCAGT GTTTGCCAGC GTAAATAATC 50
TCTACCATCG ACCACAGCAA AAAAAGCAGA TCGATCGCCA GCATGCGCGG 100
<210> 8785
<211> 100
<212> DNA

<213> Escherichia coli K-12 MG1655
<220> glcc glcC
<222> 474-573
<223> glcc glcC U00096 3126287..3127051
<313> 8785: 498-517
<400> 8785
TCAGGCTTCT CACAATCAGG TGCTGGTGTT TACGCTGCAA TCATTGACCG 50
ATCTGATGTT TAATTCAGTG TTTGCCAGCG TAAATAATCT CTACCATCGA 100
<210> 8786
<211> 100
<212> DNA
<213> Escherichia coli K-12 MG1655
<220> phor b0401
<222> 1137-1236
<223> phor b0401 U00096 417113..418408
<313> 8786: 1191-1210
<400> 8786
GCGTTCCCGG CAAACCGGCG GTAGCGGATT AGGGTTAGCG ATCGTGAAAC 50
ATGCTGTGAA TCATCACGAA AGTCGCCTGA ATATTGAGAG TACAGTAGGA 100
<210> 8787
<211> 100
<212> DNA
<213> Escherichia coli K-12 MG1655
<220> phor b0401
<222> 1121-1220
<223> phor b0401 U00096 417113..418408
<313> 8787: 1154-1173
<400> 8787
ATCGCGTTGA TAAAGCGCGT TCCCGGCAAA CCGGCGGTAG CGGATTAGGG 50
TTAGCGATCG TGAAACATGC TGTGAATCAT CACGAAAGTC GCCTGAATAT 100
<210> 8788
<211> 100
<212> DNA
<213> Escherichia coli K-12 MG1655
<220> phor b0401
<222> 1045-1144
<223> phor b0401 U00096 417113..418408
<313> 8788: 1096-1115
<400> 8788
GGTGCCGAAT TTAGCGTTGA AGATAACGGA CCGGGCATTG CACCGGAGCA 50
TATTCCGCGC CTGACCGAGC GTTTTTATCG CGTTGATAAA GCGCGTTCCC 100
<210> 8789
<211> 100
<212> DNA
<213> Escherichia coli K-12 MG1655
<220> glcd b2980
<222> 1401-1500
<223> glcd b2980 U00096 complement(3124537..3126036)
<313> 8789: 1457-1476
<400> 8789
CCCTGGGAAA AACATTCCCA CGCTACACCG CTGTGCTGAA TTTGGTGCCA 50
TGCATGTGCA TCACGGTCAT TTACCTTTCC CTGAACTGGA GCGTTTCTGA 100
<210> 8790
<211> 100
<212> DNA
<213> Escherichia coli K-12 MG1655
<220> glcd b2980
<222> 1346-1445
<223> glcd b2980 U00096 complement(3124537..3126036)
<313> 8790: 1395-1414
<400> 8790
AAATCACGAC CTTCCATGCG GTCAAGGCGG CGTTTGACCC CGATGGTTTG 50
CTGAACCCTG GGAAAAACAT TCCCACGCTA CACCGCTGTG CTGAATTTGG 100
<210> 8791
<211> 100
<212> DNA
<213> Escherichia coli K-12 MG1655
<220> glcd b2980
<222> 1307-1406
<223> glcd b2980 U00096 complement(3124537..3126036)
<313> 8791: 1353-1372

<400> 8791
AAAAAATCAA TCAAATGTGC GCCCAGTTCA ACAGCGATGA AATCACGACC 50
TTCCATGCGG TCAAGGCGGC GTTTGACCCC GATGGTTTGC TGAACCCTGG 100
<210> 8792
<211> 100
<212> DNA
<213> Escherichia coli K-12 MG1655
<220> cmk b0911
<222> 572-671
<223> cmk b0911 U00096 960424..961107
<313> 8792: 625-644
<400> 8792
CGCCACTGGT TCCGGCAGCC GATGCTTTAG TGTTGGATTC CACCACCTTA 50
AGCATTGAGC AAGTGATTGA AAAAGCGCTA CAATACGCGC GCCAGAAATT 100
<210> 8793
<211> 100
<212> DNA
<213> Escherichia coli K-12 MG1655
<220> cmk b0911
<222> 537-636
<223> cmk b0911 U00096 960424..961107
<313> 8793: 569-588
<400> 8793
AGAACGCGAC GACCGCGATC GTAACCGAGC GGTAGCGCCA CTGGTTCCGG 50
CAGCCGATGC TTTAGTGTTG GATTCCACCA CCTTAAGCAT TGAGCAAGTG 100
<210> 8794
<211> 100
<212> DNA
<213> Escherichia coli K-12 MG1655
<220> cmk b0911
<222> 374-473
<223> cmk b0911 U00096 960424..961107
<313> 8794: 403-422
<400> 8794
GTCTGATTGC CGATGGCCGC GACATGGGAA CGGTGGTATT CCCTGATGCA 50
CCAGTGAAAA TTTTCCTTGA CGCCTCCTCG GAAGAACGTG CGCATCGCCG 100
<210> 8795
<211> 100
<212> DNA
<213> Escherichia coli K-12 MG1655
<220> glcf b2979
<222> 2187-2286
<223> glcf b2979 U00096 complement(3122252..3124537)
<313> 8795: 2245-2264
<400> 8795
CACCGCCAAC ATTGGTTGCC AGACGCATCT GGCGAGCGCC GGTCGTACCT 50
CTGTGCGTCA CTGGATTGAA ATTGTAGAAC AAGCCCTTGA AAAGGAATAA 100
<210> 8796
<211> 100
<212> DNA
<213> Escherichia coli K-12 MG1655
<220> glcf b2979
<222> 2127-2226
<223> glcf b2979 U00096 complement(3122252..3124537)
<313> 8796: 2155-2174
<400> 8796
ACGCCAGCTG CGGGATAACA AAATGAATGC GCTGGAAAGC GGCAAACCGG 50
AAATGATCGT CACCGCCAAC ATTGGTTGCC AGACGCATCT GGCGAGCGCC 100
<210> 8797
<211> 100
<212> DNA
<213> Escherichia coli K-12 MG1655
<220> glcf b2979
<222> 1930-2029
<223> glcf b2979 U00096 complement(3122252..3124537)
<313> 8797: 1969-1988
<400> 8797
CCGCTGGAAA AACTGGCAAT TCGCGGCGAT AAAAAGCTGG CCTTCCACTG 50
TCCGTGTACC CTACAACATG CGCAAAAGCT GAACGGCGAA GTGGAAAAAG 100
<210> 8798
<211> 100

<212> DNA
<213> Escherichia coli K-12 MG1655
<220> phou b3725
<222> 601-700
<223> phou b3725 U00096 complement(3904481..3905206)
<313> 8798: 637-656
<400> 8798
CGTATTGGCG ACCGCTGCCA GAATATTTGT GAGTTTATCT TCTACTACGT 50
GAAGGGGCAG GATTTCCGTC ACGTCGGTGG CGATGAGCTG GATAAACTGC 100
<210> 8799
<211> 100
<212> DNA
<213> Escherichia coli K-12 MG1655
<220> phou b3725
<222> 571-670
<223> phou b3725 U00096 complement(3904481..3905206)
<313> 8799: 605-624
<400> 8799
ACTGCGCTGT TCTGCGCGCG TTCTATCGAA CGTATTGGCG ACCGCTGCCA 50
GAATATTTGT GAGTTTATCT TCTACTACGT GAAGGGGCAG GATTTCCGTC 100
<210> 8800
<211> 100
<212> DNA
<213> Escherichia coli K-12 MG1655
<220> phou b3725
<222> 512-611
<223> phou b3725 U00096 complement(3904481..3905206)
<313> 8800: 555-574
<400> 8800
TTGTTCGTCA ACTGATGACC TACATGATGG AAGATTCGCG TACCATTCCG 50
AGCGTACTTA CTGCGCTGTT CTGCGCGCGT TCTATCGAAC GTATTGGCGA 100
<210> 8801
<211> 100
<212> DNA
<213> Escherichia coli K-12 MG1655
<220> glcg b2978
<222> 238-337
<223> glcg b2978 U00096 complement(3121843..3122247)
<313> 8801: 261-280
<400> 8801
AACAACGGAC GTACCGCGTT CGTGACTGCG CCGTTATTAA CGTCGCTGGA 50
AGGCGGCGTA CCGGTTGTTG TGGATGGGCA AATTATTGGT GCCGTGGGCG 100
<210> 8802
<211> 100
<212> DNA
<213> Escherichia coli K-12 MG1655
<220> glcg b2978
<222> 171-270
<223> glcg b2978 U00096 complement(3121843..3122247)
<313> 8802: 220-239
<400> 8802
CTCCCAGGAG AAAGCGCGTA CCGCCGCGCT GGGGCGTCGT GAAACTAAGG 50
GCTATGAAGA GATGGTGAAC AACGGACGTA CCGCGTTCGT GACTGCGCCG 100
<210> 8803
<211> 100
<212> DNA
<213> Escherichia coli K-12 MG1655
<220> glcg b2978
<222> 113-212
<223> glcg b2978 U00096 complement(3121843..3122247)
<313> 8803: 164-183
<400> 8803
GCGGTCATCT GCTGGCGTTA AGTCGCATGG ACGATTGCGC GCCGATTGCG 50
GCTTATATCT CCCAGGAGAA AGCGCGTACC GCCGCGCTGG GGCGTCGTGA 100
<210> 8804
<211> 100
<212> DNA
<213> Escherichia coli K-12 MG1655
<220> cmr b0843
<222> 1100-1199
<223> cmr b0843 U00096 882896..884128

<313> 8804: 1133-1152
<400> 8804
TCAGCAAACA TGCCTGGCTG AACGGGGGCA ACGGACTGTT TAATCTCTTC  50
AACCTTGTCA ACGGAATTTT GTGGCTGTCG CTGATGGTTA TCTTTTTAAA  100
<210> 8805
<211> 100
<212> DNA
<213> Escherichia coli K-12 MG1655
<220> cmr b0843
<222> 1064-1163
<223> cmr b0843 U00096 882896..884128
<313> 8805: 1084-1103
<400> 8805
TGCTGCAAAT GCTGATCTTT ACCGTTGGTA TTGAAATCAG CAAACATGCC  50
TGGCTGAACG GGGGCAACGG ACTGTTTAAT CTCTTCAACC TTGTCAACGG  100
<210> 8806
<211> 100
<212> DNA
<213> Escherichia coli K-12 MG1655
<220> cmr b0843
<222> 1023-1122
<223> cmr b0843 U00096 882896..884128
<313> 8806: 1059-1078
<400> 8806
CAGCGATATG AGTAAAGGTA CGGTTTCTGC CGCGATGGGA ATGCTGCAAA  50
TGCTGATCTT TACCGTTGGT ATTGAAATCA GCAAACATGC CTGGCTGAAC  100
<210> 8807
<211> 100
<212> DNA
<213> Escherichia coli K-12 MG1655
<220> rob b4397
<222> 771-870
<223> rob b4397 U00096 complement(4632010..4632879)
<313> 8807: 836-855
<400> 8807
AGGTCAGGAT ATTGAGCGAT ACTACCCGGC AGAAGATGCC AAAGCGGGAG  50
ATCGCCCAAT TAATCTACGC TGTGAACTGC TGATTCCGAT CCGTCGTTAA  100
<210> 8808
<211> 100
<212> DNA
<213> Escherichia coli K-12 MG1655
<220> rob b4397
<222> 751-850
<223> rob b4397 U00096 complement(4632010..4632879)
<313> 8808: 800-819
<400> 8808
CTCAACCTGA CGCGCCGTAA AGGTCAGGAT ATTGAGCGAT ACTACCCGGC  50
AGAAGATGCC AAAGCGGGAG ATCGCCCAAT TAATCTACGC TGTGAACTGC  100
<210> 8809
<211> 100
<212> DNA
<213> Escherichia coli K-12 MG1655
<220> rob b4397
<222> 680-779
<223> rob b4397 U00096 complement(4632010..4632879)
<313> 8809: 715-734
<400> 8809
TTACCTATGA AGGTCTGGGA ACCGGCGTGC AGGAGTTTAT CCTGACGGTA  50
TACGGAACGT GCATGCCAAT GCTCAACCTG ACGCGCCGTA AAGGTCAGGA  100
<210> 8810
<211> 100
<212> DNA
<213> Escherichia coli K-12 MG1655
<220> phpb b0639
<222> 513-612
<223> phpb b0639 U00096 complement(668519..669130)
<313> 8810: 585-604
<400> 8810
TCGCGTTGAC CAGGGGTGTT GGAGCGCCAT TGATATCAAC CAAAAATTCG  50
CGACGCTACG CGTCCTCAAT AGCCGTGCCA TCGGGGTCGA GAATGCATGA  100
<210> 8811

<211> 100
<212> DNA
<213> Escherichia coli K-12 MG1655
<220> phpb b0639
<222> 491-590
<223> phpb b0639 U00096 complement(668519..669130)
<313> 8811: 521-540
<400> 8811
CTGCCGAAGC TATGTGGCAT TTTCGCGTTG ACCAGGGGTG TTGGAGCGCC 50
ATTGATATCA ACCAAAAATT CGCGACGCTA CGCGTCCTCA ATAGCCGTGC 100
<210> 8812
<211> 100
<212> DNA
<213> Escherichia coli K-12 MG1655
<220> phpb b0639
<222> 435-534
<223> phpb b0639 U00096 complement(668519..669130)
<313> 8812: 464-483
<400> 8812
AGTCGTCAGC CATCAGGGTG TACTGAGTCT GTTAATCGCC CGTTTAATTG 50
GCATGCCTGC CGAAGCTATG TGGCATTTTC GCGTTGACCA GGGGTGTTGG 100
<210> 8813
<211> 100
<212> DNA
<213> Escherichia coli K-12 MG1655
<220> cafa b3248
<222> 1344-1443
<223> cafa b3248 U00096 complement(3393963..3395450)
<313> 8813: 1378-1397
<400> 8813
CTATGCTTCT CCGGCAGTAG CTGAAGCCTT GAAAGGCGAA GAGTCACACT 50
CGCTGGCGGA AGTGGAAATT TTCGTTGGCA AACAGGTTAA AGTACAAATT 100
<210> 8814
<211> 100
<212> DNA
<213> Escherichia coli K-12 MG1655
<220> cafa b3248
<222> 1289-1388
<223> cafa b3248 U00096 complement(3393963..3395450)
<313> 8814: 1333-1352
<400> 8814
TCATGCGCGA GATTGTTCGT GTCCACCATG CTTACGACTC CGACCGTTTC 50
CTGGTCTATG CTTCTCCGGC AGTAGCTGAA GCCTTGAAAG GCGAAGAGTC 100
<210> 8815
<211> 100
<212> DNA
<213> Escherichia coli K-12 MG1655
<220> cafa b3248
<222> 1262-1361
<223> cafa b3248 U00096 complement(3393963..3395450)
<313> 8815: 1294-1313
<400> 8815
AAACCGTGGA AACGGTATGC TATGAAATCA TGCGCGAGAT TGTTCGTGTC 50
CACCATGCTT ACGACTCCGA CCGTTTCCTG GTCTATGCTT CTCCGGCAGT 100
<210> 8816
<211> 100
<212> DNA
<213> Escherichia coli K-12 MG1655
<220> b0362 b0363
<222> 253-352
<223> b0362 b0363 U00096 complement(381728..382114)
<313> 8816: 302-321
<400> 8816
GTTATTCAGG GTCCGGTAGT GATTGGCGCA AACTGCCTGA TAGGTAATTG 50
GATTTGCCCC TATATTTCCA GACATCTGTT ATCACTTAAC CCATTACAAG 100
<210> 8817
<211> 100
<212> DNA
<213> Escherichia coli K-12 MG1655
<220> b0362 b0363
<222> 250-349

<223> b0362 b0363 U00096 complement(381728..382114)
<313> 8817: 293-312
<400> 8817
GCCGTTATTC AGGGTCCGGT AGTGATTGGC GCAAACTGCC TGATAGGTAA 50
TTGGATTTGC CCCTATATTT CCAGACATCT GTTATCACTT AACCCATTAC 100
<210> 8818
<211> 100
<212> DNA
<213> Escherichia coli K-12 MG1655
<220> b0362 b0363
<222> 239-338
<223> b0362 b0363 U00096 complement(381728..382114)
<313> 8818: 297-316
<400> 8818
TTTGTCATGG TGCCGTTATT CAGGGTCCGG TAGTGATTGG CGCAAACTGC 50
CTGATAGGTA ATTGGATTTG CCCCTATATT TCCAGACATC TGTTATCACT 100
<210> 8819
<211> 100
<212> DNA
<213> Escherichia coli K-12 MG1655
<220> uxaa b3092
<222> 1389-1488
<223> uxaa b3092 U00096 complement(3239467..3240954)
<313> 8819: 1466-1485
<400> 8819
TATCGACACC ATCGTTGAGT TTGCCAACGG TAAGCAAACC TGTAACGAGC 50
GTAACGACTT CCGCGAACTG GCGATCTTCA AAAGCGGCGT AACGCTATAA 100
<210> 8820
<211> 100
<212> DNA
<213> Escherichia coli K-12 MG1655
<220> uxaa b3092
<222> 1376-1475
<223> uxaa b3092 U00096 complement(3239467..3240954)
<313> 8820: 1422-1441
<400> 8820
TGCTGGAAGA ATTTATCGAC ACCATCGTTG AGTTTGCCAA CGGTAAGCAA 50
ACCTGTAACG AGCGTAACGA CTTCCGCGAA CTGGCGATCT TCAAAAGCGG 100
<210> 8821
<211> 100
<212> DNA
<213> Escherichia coli K-12 MG1655
<220> uxaa b3092
<222> 1280-1379
<223> uxaa b3092 U00096 complement(3239467..3240954)
<313> 8821: 1318-1337
<400> 8821
TGAAAATCGC CACCAACAGT GAACTGGCGG CGAAGAAAAA ACACTGGATC 50
GACTTTGACG CGGGTCAGTT GATCCACGGT AAAGCGATGC CGCAGTTGCT 100
<210> 8822
<211> 100
<212> DNA
<213> Escherichia coli K-12 MG1655
<220> uxab uxaB
<222> 1353-1452
<223> uxab uxaB U00096 complement(1607253..1608704)
<313> 8822: 1425-1444
<400> 8822
GGACCTGACG CAAGTGCCTG GTCTGGTCGA GCAGGTTGCT AATGACCTGG 50
ATGCGATTCT CGAAAAAGGG ATGCGCGAAG CTGTACGTCC GTTGTGCTAA 100
<210> 8823
<211> 100
<212> DNA
<213> Escherichia coli K-12 MG1655
<220> uxab uxaB
<222> 1342-1441
<223> uxab uxaB U00096 complement(1607253..1608704)
<313> 8823: 1362-1381
<400> 8823
CACTGGGAGC AGGACCTGAC GCAAGTGCCT GGTCTGGTCG AGCAGGTTGC 50
TAATGACCTG GATGCGATTC TCGAAAAAGG GATGCGCGAA GCTGTACGTC 100

<210> 8824
<211> 100
<212> DNA
<213> Escherichia coli K-12 MG1655
<220> uxab uxaB
<222> 1297-1396
<223> uxab uxaB U00096 complement(1607253..1608704)
<313> 8824: 1332-1351
<400> 8824
ATCGGCACCC AGGAACTGGT AGCGATTGTT TTGGCAGAAA AAGACCACTG 50
GGAGCAGGAC CTGACGCAAG TGCCTGGTCT GGTCGAGCAG GTTGCTAATG 100
<210> 8825
<211> 100
<212> DNA
<213> Escherichia coli K-12 MG1655
<220> uxac b3093
<222> 1290-1389
<223> uxac b3093 U00096 complement(3240969..3242381)
<313> 8825: 1341-1360
<400> 8825
CCAGATGATC GGTCGCTGGG TGGAAGCGGG CGAAGCACCG GCGGACATCA 50
ACCTGCTGGG CGAGATGGTG AAAAATATTT GCTTTAACAA TGCGCGTGAC 100
<210> 8826
<211> 100
<212> DNA
<213> Escherichia coli K-12 MG1655
<220> uxac b3093
<222> 1225-1324
<223> uxac b3093 U00096 complement(3240969..3242381)
<313> 8826: 1262-1281
<400> 8826
ATGCTGACTG ACAGCCGTAG CTTCCTGTCA TACACCCGTC ACGAATACTT 50
CCGCCGCATT CTGTGCCAGA TGATCGGTCG CTGGGTGGAA GCGGGCGAAG 100
<210> 8827
<211> 100
<212> DNA
<213> Escherichia coli K-12 MG1655
<220> uxac b3093
<222> 1141-1240
<223> uxac b3093 U00096 complement(3240969..3242381)
<313> 8827: 1159-1178
<400> 8827
TGGTGGTTTA ACGATCAGAA AGACGGTATG GAACGTCAGA TGACCCAACT 50
GGCGCAGCTC GGTCTGCTGA GCCGCTTTGT CGGTATGCTG ACTGACAGCC 100
<210> 8828
<211> 100
<212> DNA
<213> Escherichia coli K-12 MG1655
<220> zipa b2413
<222> 888-987
<223> zipa b2413 U00096 complement(2528267..2529253)
<313> 8828: 959-978
<400> 8828
GGGCGGTGTC GTGCTTGACG ATCAGCGCCG TATGATGACT CCGCAGAAAT 50
TGCGCGAGTA CCAGGACATC ATCCGCGAAG TCAAAGACGC CAACGCCTGA 100
<210> 8829
<211> 100
<212> DNA
<213> Escherichia coli K-12 MG1655
<220> zipa b2413
<222> 861-960
<223> zipa b2413 U00096 complement(2528267..2529253)
<313> 8829: 910-929
<400> 8829
ATCTGCGCAG CATATTGCCG ATGAAGTGGG CGGTGTCGTG CTTGACGATC 50
AGCGCCGTAT GATGACTCCG CAGAAATTGC GCGAGTACCA GGACATCATC 100
<210> 8830
<211> 100
<212> DNA
<213> Escherichia coli K-12 MG1655
<220> zipa b2413

<222> 706-805
<223> zipa b2413 U00096 complement(2528267..2529253)
<313> 8830: 758-777
<400> 8830
GATGGCAGCG GCCCGGCGTT ATTCAGCCTG GCGAATATGG TGAAACCGGG  50
AACCTTTGAT CCTGAAATGA AGGATTTCAC TACTCCGGGT GTCACTATCT  100
<210> 8831
<211> 100
<212> DNA
<213> Escherichia coli K-12 MG1655
<220> glda b3946
<222> 1044-1143
<223> glda b3946 U00096 complement(4135512..4136654)
<313> 8831: 1099-1118
<400> 8831
CATTCACAAC ATGCCTGGCG GCGCGACGCC AGATCAGGTT TACGCCGCTC  50
TGCTGGTAGC CGACCAGTAC GGTCAGCGTT TCCTGCAAGA GTGGGAATAA  100
<210> 8832
<211> 100
<212> DNA
<213> Escherichia coli K-12 MG1655
<220> glda b3946
<222> 1000-1099
<223> glda b3946 U00096 complement(4135512..4136654)
<313> 8832: 1036-1055
<400> 8832
AAAATGCGAA TTGTGGCAGA AGCGGCATGT GCAGAAGGTG AAACCATTCA  50
CAACATGCCT GGCGGCGCGA CGCCAGATCA GGTTTACGCC GCTCTGCTGG  100
<210> 8833
<211> 100
<212> DNA
<213> Escherichia coli K-12 MG1655
<220> glda b3946
<222> 961-1060
<223> glda b3946 U00096 complement(4135512..4136654)
<313> 8833: 1001-1020
<400> 8833
ACTCTCGCTC AACTGGATAT TAAAGAAGAT GTCCCGGCGA AAATGCGAAT  50
TGTGGCAGAA GCGGCATGTG CAGAAGGTGA AACCATTCAC AACATGCCTG  100
<210> 8834
<211> 100
<212> DNA
<213> Escherichia coli K-12 MG1655
<220> rpe b3387
<222> 558-657
<223> rpe b3387 U00096 complement(3512020..3512697)
<313> 8834: 604-623
<400> 8834
CGAAATCGCT GCGGCGGGCG CGGATATGTT CGTCGCCGGT TCGGCAATCT  50
TCGACCAGCC AGACTACAAA AAAGTCATTG ATGAAATGCG CAGTGAACTG  100
<210> 8835
<211> 100
<212> DNA
<213> Escherichia coli K-12 MG1655
<220> rpe b3387
<222> 507-606
<223> rpe b3387 U00096 complement(3512020..3512697)
<313> 8835: 531-550
<400> 8835
CTTTGACATT CGACTAGAAG TGGACGGTGG CGTGAAGGTG AACAACATTG  50
GCGAAATCGC TGCGGCGGGC GCGGATATGT TCGTCGCCGG TTCGGCAATC  100
<210> 8836
<211> 100
<212> DNA
<213> Escherichia coli K-12 MG1655
<220> rpe b3387
<222> 460-559
<223> rpe b3387 U00096 complement(3512020..3512697)
<313> 8836: 490-509
<400> 8836
ACACTGGATA AACTGCGCGA AGTACGTCGC CGTATCGACG AGTCTGGCTT  50

TGACATTCGA CTAGAAGTGG ACGGTGGCGT GAAGGTGAAC AACATTGGCG 100
<210> 8837
<211> 100
<212> DNA
<213> Escherichia coli K-12 MG1655
<220> agaa b3136
<222> 392-491
<223> agaa b3136 U00096 3278763..3279266
<313> 8837: 433-452
<400> 8837
TTGATGGTGT TCTGGGATCG CTTAAACCGG GCAAACGCGC CAGAGTCGTT 50
GCGCTGGATA GCGGGCTACA TGTGCAACAA ATCTGGATTC AGGGTCAATT 100
<210> 8838
<211> 100
<212> DNA
<213> Escherichia coli K-12 MG1655
<220> agaa b3136
<222> 114-213
<223> agaa b3136 U00096 3278763..3279266
<313> 8838: 158-177
<400> 8838
GCATCCGGCG GCAATGTCGC TGTGTTGTTG CTGTGCGAAA GAGAGAATCG 50
TACTGATCAC CGACGCGATG CAGGCAGCTG GGATGCCGGA TGGTCGCTAT 100
<210> 8839
<211> 100
<212> DNA
<213> Escherichia coli K-12 MG1655
<220> agaa b3136
<222> 1-100
<223> agaa b3136 U00096 3278763..3279266
<313> 8839: 45-64
<400> 8839
GTGCATTGCT ATAACGGGAT GACAGGTTTA CATCACCGCG AACCGGGAAT 50
GGTTGGCGCG GGATTAACGG ACAAGCGCGC CTGGCTGGAA CTGATAGCCG 100
<210> 8840
<211> 100
<212> DNA
<213> Escherichia coli K-12 MG1655
<220> rph b3644
<222> 588-687
<223> rph b3644 U00096 complement(3813490..3814176)
<313> 8840: 655-674
<400> 8840
CATCATTGAA GTGCAGGGGA CGGCAGAAGG CGAGCCGTTC ACCCATGAAG 50
AGCTACTCAT CTTGTTGGCT CTGGCCCGAG GGGAATCGAA TCCATTGTAG 100
<210> 8841
<211> 100
<212> DNA
<213> Escherichia coli K-12 MG1655
<220> rph b3644
<222> 576-675
<223> rph b3644 U00096 complement(3813490..3814176)
<313> 8841: 623-642
<400> 8841
CGAAGACGGG CGCATCATTG AAGTGCAGGG GACGGCAGAA GGCGAGCCGT 50
TCACCCATGA AGAGCTACTC ATCTTGTTGG CTCTGGCCCG AGGGGAATCG 100
<210> 8842
<211> 100
<212> DNA
<213> Escherichia coli K-12 MG1655
<220> rph b3644
<222> 537-636
<223> rph b3644 U00096 complement(3813490..3814176)
<313> 8842: 569-588
<400> 8842
AGACTCTGCC GCAGAGACCG ACATGAACGT AGTGATGACC GAAGACGGGC 50
GCATCATTGA AGTGCAGGGG ACGGCAGAAG GCGAGCCGTT CACCCATGAA 100
<210> 8843
<211> 100
<212> DNA
<213> Escherichia coli K-12 MG1655

<220> agab b3139
<222> 322-421
<223> agab b3139 U00096 3281811..3282287
<313> 8843: 363-382
<400> 8843
GGCAATATGC ATTTCTCGGA AGGGAAAAAG CAAATCAGCA GTAAAGTTTA 50
TGTCGATGAC CAGGATCTCA CGGACTTACG TTTTATTAAA CAACGTGGCG 100
<210> 8844
<211> 100
<212> DNA
<213> Escherichia coli K-12 MG1655
<220> agab b3139
<222> 277-376
<223> agab b3139 U00096 3281811..3282287
<313> 8844: 320-339
<400> 8844
CGTAAATTGG TAGAAGGTGG TATTGACCTG AAAGATGTCA ACGTCGGCAA 50
TATGCATTTC TCGGAAGGGA AAAAGCAAAT CAGCAGTAAA GTTTATGTCG 100
<210> 8845
<211> 100
<212> DNA
<213> Escherichia coli K-12 MG1655
<220> agab b3139
<222> 191-290
<223> agab b3139 U00096 3281811..3282287
<313> 8845: 211-230
<400> 8845
TTACTATCGA AAAAACCATT AACGTCATCG GCAAAGCTGC ACCACATCAG 50
AAGATCTTCC TGATTTGCCG TACGCCACAA ACGGTACGTA AATTGGTAGA 100
<210> 8846
<211> 100
<212> DNA
<213> Escherichia coli K-12 MG1655
<220> agac b3140
<222> 705-804
<223> agac b3140 U00096 3282326..3283129
<313> 8846: 774-793
<400> 8846
TGCGGTGTAT GAGTTTTTCA ATGCGAAATC CCGGCAGCAA GCGCAACCGC 50
AGCCCGTTGC CAGTAAAAAT GAAGAAGAGG ACTACAGCAA TGGGATCTGA 100
<210> 8847
<211> 100
<212> DNA
<213> Escherichia coli K-12 MG1655
<220> agac b3140
<222> 682-781
<223> agac b3140 U00096 3282326..3283129
<313> 8847: 729-748
<400> 8847
GTTGCCGTAC TGGGCGCAGG CTTTGCGGTG TATGAGTTTT TCAATGCGAA 50
ATCCCGGCAG CAAGCGCAAC CGCAGCCCGT TGCCAGTAAA AATGAAGAAG 100
<210> 8848
<211> 100
<212> DNA
<213> Escherichia coli K-12 MG1655
<220> agac b3140
<222> 610-709
<223> agac b3140 U00096 3282326..3283129
<313> 8848: 644-663
<400> 8848
GCGCAATATA TCCCTTACCT GATCGCCGGT TTCCTGTTTG TTTGCTACAT 50
CCAGGTCAGC AACCTGTTGC CGGTTGCCGT ACTGGGCGCA GGCTTTGCGG 100
<210> 8849
<211> 100
<212> DNA
<213> Escherichia coli K-12 MG1655
<220> b0370 b0371
<222> 171-270
<223> b0370 b0371 U00096 complement(389121..389390)
<313> 8849: 237-256
<400> 8849

ACCAGATAAA TATCCTATGA ATATGCAACA AAGTTTTAAT TATTCAATTT 50
ATAGCAGCGC AACCAAACTC CAAAAAATAA TAACCAACAG CTATAAATAA 100
<210> 8850
<211> 100
<212> DNA
<213> Escherichia coli K-12 MG1655
<220> b0370 b0371
<222> 171-270
<223> b0370 b0371 U00096 complement(389121..389390)
<313> 8850: 233-252
<400> 8850
ACCAGATAAA TATCCTATGA ATATGCAACA AAGTTTTAAT TATTCAATTT 50
ATAGCAGCGC AACCAAACTC CAAAAAATAA TAACCAACAG CTATAAATAA 100
<210> 8851
<211> 100
<212> DNA
<213> Escherichia coli K-12 MG1655
<220> b0370 b0371
<222> 171-270
<223> b0370 b0371 U00096 complement(389121..389390)
<313> 8851: 221-240
<400> 8851
ACCAGATAAA TATCCTATGA ATATGCAACA AAGTTTTAAT TATTCAATTT 50
ATAGCAGCGC AACCAAACTC CAAAAAATAA TAACCAACAG CTATAAATAA 100
<210> 8852
<211> 100
<212> DNA
<213> Escherichia coli K-12 MG1655
<220> agad b3141
<222> 693-792
<223> agad b3141 U00096 3283119..3283910
<313> 8852: 760-779
<400> 8852
GATGTATTAC TTCCTGCGGG TGAAAAAAGC GCATCCGGTG CTGTTAATCG 50
GCGTGACTTT TGTGCTCTCT ATTGTTTGTT CCGCATTCGG CATTTTGTAA 100
<210> 8853
<211> 100
<212> DNA
<213> Escherichia coli K-12 MG1655
<220> agad b3141
<222> 634-733
<223> agad b3141 U00096 3283119..3283910
<313> 8853: 666-685
<400> 8853
CAGCAGGATT TCTTCGATAA AGTCTTCCCG AACATTTTAC CGATGGCCTA 50
CACCCTGCTG ATGTATTACT TCCTGCGGGT GAAAAAAGCG CATCCGGTGC 100
<210> 8854
<211> 100
<212> DNA
<213> Escherichia coli K-12 MG1655
<220> agad b3141
<222> 562-661
<223> agad b3141 U00096 3283119..3283910
<313> 8854: 589-608
<400> 8854
CTGATCGCTT CGTATGTGCA TATTAACGTG GTGACATCGT TTGCCATCGA 50
CAATACCCAC AGCGTTGCGC TGCAGCAGGA TTTCTTCGAT AAAGTCTTCC 100
<210> 8855
<211> 100
<212> DNA
<213> Escherichia coli K-12 MG1655
<220> b0373 b0374
<222> 96-195
<223> b0373 b0374 U00096 complement(391826..392134)
<313> 8855: 160-179
<400> 8855
CATCGGTGTT ACTGCCGCAG CCCGTGAACT CAGCCTGTAT GAATCACAAC 50
TCTACAACTG GCGCAGTAAA CAGCAAAATC AGCAGACGTC TTCTGAACGT 100
<210> 8856
<211> 100
<212> DNA

<213> Escherichia coli K-12 MG1655
<220> b0373 b0374
<222> 65-164
<223> b0373 b0374 U00096 complement(391826..392134)
<313> 8856: 106-125
<400> 8856
TTCGCAGTGA AGCCCTGAAG CTTGCTGAAC GCATCGGTGT TACTGCCGCA 50
GCCCGTGAAC TCAGCCTGTA TGAATCACAA CTCTACAACT GGCGCAGTAA 100
<210> 8857
<211> 100
<212> DNA
<213> Escherichia coli K-12 MG1655
<220> b0373 b0374
<222> 24-123
<223> b0373 b0374 U00096 complement(391826..392134)
<313> 8857: 78-97
<400> 8857
ATCAACCAGT AAAAAACCCC GTAAACAGCA TTCGCCTGAA TTTCGCAGTG 50
AAGCCCTGAA GCTTGCTGAA CGCATCGGTG TTACTGCCGC AGCCCGTGAA 100
<210> 8858
<211> 100
<212> DNA
<213> Escherichia coli K-12 MG1655
<220> agai b3142
<222> 657-756
<223> agai b3142 U00096 3283911..3284666
<313> 8858: 698-717
<400> 8858
GGATGCGACA GATCGTTTTC TCACGGCTAA AGTCTCTACC GCTATCCCGG 50
CTTCATTTTT ATGGCTGCAC AGTAACTTTA TTTGTTTAAT TAACACCTAA 100
<210> 8859
<211> 100
<212> DNA
<213> Escherichia coli K-12 MG1655
<220> agai b3142
<222> 642-741
<223> agai b3142 U00096 3283911..3284666
<313> 8859: 659-678
<400> 8859
TGGCGAAGGA AAGCAGGATG CGACAGATCG TTTTCTCACG GCTAAAGTCT 50
CTACCGCTAT CCCGGCTTCA TTTTTATGGC TGCACAGTAA CTTTATTTGT 100
<210> 8860
<211> 100
<212> DNA
<213> Escherichia coli K-12 MG1655
<220> agai b3142
<222> 501-600
<223> agai b3142 U00096 3283911..3284666
<313> 8860: 536-555
<400> 8860
ACCGGCCTGC CATATCAGTC AACTTGATGC CAGAACACAG CAACATGAGA 50
TGTTAAAAAC CGCGGGTCGC CCCGTGACTC GTGGGATCAC CTTAGGCCTG 100
<210> 8861
<211> 100
<212> DNA
<213> Escherichia coli K-12 MG1655
<220> b0379 b0379
<222> 200-299
<223> b0379 b0379 U00096 complement(398249..398557)
<313> 8861: 235-254
<400> 8861
AAATTGGTTT AGGTGTCGGC ACCATTTTCG GGCTGATCCC GTTTTTAGTA 50
GGCTGCCTCA TTTTTGCAGT GGTGGCGCTA TGGCTGCACT GGCGACATCG 100
<210> 8862
<211> 100
<212> DNA
<213> Escherichia coli K-12 MG1655
<220> b0379 b0379
<222> 155-254
<223> b0379 b0379 U00096 complement(398249..398557)
<313> 8862: 177-196

<400> 8862
ATGAGCGTTT AATGCAGGTC CAGGAAACAG GTCGCCCACG GGTGGAAATT 50
GGTTTAGGTG TCGGCACCAT TTTCGGGCTG ATCCCGTTTT TAGTAGGCTG 100
<210> 8863
<211> 100
<212> DNA
<213> Escherichia coli K-12 MG1655
<220> b0379 b0379
<222> 64-163
<223> b0379 b0379 U00096 complement(398249..398557)
<313> 8863: 84-103
<400> 8863
CCTGGCAACA TTGCCTATGC GTTGTTTGTG CTGTTTTGCT TTTGGGCTGG 50
GGCGCAATTG CTGAACCTGT TAGTGCATGC GCCCGGCGTC TATGAGCGTT 100
<210> 8864
<211> 100
<212> DNA
<213> Escherichia coli K-12 MG1655
<220> cof b0447
<222> 732-831
<223> cof b0447 U00096 466624..467454
<313> 8864: 772-791
<400> 8864
CCCGCATTTA CCGGTGATTG GACATTGCCG AAATCAGGCT GTCTCTCACT 50
ATTTGACGCA CTGGCTGGAC TATCCACATC TACCTTATTC CCCCGAATAA 100
<210> 8865
<211> 100
<212> DNA
<213> Escherichia coli K-12 MG1655
<220> cof b0447
<222> 661-760
<223> cof b0447 U00096 466624..467454
<313> 8865: 682-701
<400> 8865
CGCGAAATGT TAGTCAGCGT CGGTAGCGGA TTTATTATGG GCAATGCGAT 50
GCCGCAACTG CGCGCGGAGC TCCCGCATTT ACCGGTGATT GGACATTGCC 100
<210> 8866
<211> 100
<212> DNA
<213> Escherichia coli K-12 MG1655
<220> cof b0447
<222> 485-584
<223> cof b0447 U00096 466624..467454
<313> 8866: 540-559
<400> 8866
TGCAGATCCA GCTATACGAA GCATTAGGCG AGCGTGCACA TTTGTGTTTT 50
TCCGCCACGG ATTGCCTCGA AGTGCTGCCG GTGGGCTGCA ATAAAGGCGC 100
<210> 8867
<211> 100
<212> DNA
<213> Escherichia coli K-12 MG1655
<220> agar b3132
<222> 711-810
<223> agar b3132 U00096 complement(3275497..3276306)
<313> 8867: 770-789
<400> 8867
TCAACGTATC GACATGATCA TTGTTGATGA AGGCATTCCT GCGGATAGCC 50
TGGAAGGACT GCGAAAGGCT GGGGTTGAAG TGATTCTGGT CGGGGAGTGA 100
<210> 8868
<211> 100
<212> DNA
<213> Escherichia coli K-12 MG1655
<220> agar b3132
<222> 596-695
<223> agar b3132 U00096 complement(3275497..3276306)
<313> 8868: 636-655
<400> 8868
ATAATGAAGA TGAAGCTCGT TTAAACCGTC GGATGTGCGA AGTTGCGGAA 50
CGGATCATCG TGGTCACCGA TTCCAGTAAG TTCAATCGTT CCAGTTTACA 100
<210> 8869
<211> 100

<212> DNA
<213> Escherichia coli K-12 MG1655
<220> agar b3132
<222> 556-655
<223> agar b3132 U00096 complement(3275497..3276306)
<313> 8869: 590-609
<400> 8869
GGTGTAGATG CGATCGATCT GGAGCGCGGC GTCAGCACAC ATAATGAAGA 50
TGAAGCTCGT TTAAACCGTC GGATGTGCGA AGTTGCGGAA CGGATCATCG 100
<210> 8870
<211> 100
<212> DNA
<213> Escherichia coli K-12 MG1655
<220> agas b3137
<222> 1056-1155
<223> agas b3137 U00096 3279617..3280771
<313> 8870: 1132-1151
<400> 8870
GCAGTCGCTG CACATGGGCA ATACGCCGGA TACCCCATCA GCCAGTGGCA 50
CCGTTAACCG CGTGGTGCAA GGCGTAATCA TTCATCCGTG GCAGGCATAA 100
<210> 8871
<211> 100
<212> DNA
<213> Escherichia coli K-12 MG1655
<220> agas b3137
<222> 1044-1143
<223> agas b3137 U00096 3279617..3280771
<313> 8871: 1081-1100
<400> 8871
GTTTGCACTG ATGCAGTCGC TGCACATGGG CAATACGCCG GATACCCCAT 50
CAGCCAGTGG CACCGTTAAC CGCGTGGTGC AAGGCGTAAT CATTCATCCG 100
<210> 8872
<211> 100
<212> DNA
<213> Escherichia coli K-12 MG1655
<220> agas b3137
<222> 961-1060
<223> agas b3137 U00096 3279617..3280771
<313> 8872: 1012-1031
<400> 8872
GCCGGTCCAC ATATCATCCT GCCACCGTCA CGTCACTTTA TCGACGTTGA 50
GCAGGCATTT TGCTTCCTGA TGTACGCCCA GACGTTTGCA CTGATGCAGT 100
<210> 8873
<211> 100
<212> DNA
<213> Escherichia coli K-12 MG1655
<220> agav b3134
<222> 411-510
<223> agav b3134 U00096 3277822..3278331
<313> 8873: 484-503
<400> 8873
CGATATCGCA GCATTTAACG ACCTGAAAAC CGCTGGGGTG GAATGCTTCG 50
TTCAGGGCGT CCCGACAGAG CCTGCTGTGG ACCTCTTTAA ATTACTTTGA 100
<210> 8874
<211> 100
<212> DNA
<213> Escherichia coli K-12 MG1655
<220> agav b3134
<222> 399-498
<223> agav b3134 U00096 3277822..3278331
<313> 8874: 422-441
<400> 8874
TGTGGATGCG GGCGATATCG CAGCATTTAA CGACCTGAAA ACCGCTGGGG 50
TGGAATGCTT CGTTCAGGGC GTCCCGACAG AGCCTGCTGT GGACCTCTTT 100
<210> 8875
<211> 100
<212> DNA
<213> Escherichia coli K-12 MG1655
<220> agav b3134
<222> 346-445
<223> agav b3134 U00096 3277822..3278331

<313> 8875: 383-402
<400> 8875
GTTGGCAATA TGCACTACGC CAATGGCAAA CAACAAATCG CCAAAACGGT 50
TTCTGTGGAT GCGGGCGATA TCGCAGCATT TAACGACCTG AAAACCGCTG 100
<210> 8876
<211> 100
<212> DNA
<213> Escherichia coli K-12 MG1655
<220> agaw b3135
<222> 303-402
<223> agaw b3135 U00096 3278342..3278743
<313> 8876: 377-396
<400> 8876
TTTCGCTGTC GCAGTACAGA TGGGGATTAC CTTCCTGTTC TCGGTGATGT 50
CCGGCGTGAT GTCTCGCTGC GACCTGGCAA CAAACCCGCG CCGCATTTGA 100
<210> 8877
<211> 100
<212> DNA
<213> Escherichia coli K-12 MG1655
<220> agaw b3135
<222> 288-387
<223> agaw b3135 U00096 3278342..3278743
<313> 8877: 325-344
<400> 8877
AGGTGTCGCC GTACCTTTCG CTGTCGCAGT ACAGATGGGG ATTACCTTCC 50
TGTTCTCGGT GATGTCCGGC GTGATGTCTC GCTGCGACCT GGCAACAAAC 100
<210> 8878
<211> 100
<212> DNA
<213> Escherichia coli K-12 MG1655
<220> agaw b3135
<222> 240-339
<223> agaw b3135 U00096 3278342..3278743
<313> 8878: 288-307
<400> 8878
CGGCACGGCG TTTGCCATTA CTACTGGCGT GAAACCCGAT GTCGCAGTAG 50
GTGTCGCCGT ACCTTTCGCT GTCGCAGTAC AGATGGGGAT TACCTTCCTG 100
<210> 8879
<211> 100
<212> DNA
<213> Escherichia coli K-12 MG1655
<220> agay b3138
<222> 641-740
<223> agay b3138 U00096 3280784..3281644
<313> 8879: 692-711
<400> 8879
CGGATGAATT TGTCCGTCGC ACTATTGAAC TTGGCGTCAC AAAAGTGAAC 50
GTTGCCACAG AATTAAAAAT AGCCTTCGCT GGCGCGGTTA AAGCCTGGTT 100
<210> 8880
<211> 100
<212> DNA
<213> Escherichia coli K-12 MG1655
<220> agay b3138
<222> 470-569
<223> agay b3138 U00096 3280784..3281644
<313> 8880: 527-546
<400> 8880
CACAAGAAGC TAAACGCTTT GTCGAACTGA CTGGCGTCGA CAGCCTGGCG 50
GTAGCGATTG GTACGGCGCA CGGCTTATAC AGCAAAACGC CGAAGATTGA 100
<210> 8881
<211> 100
<212> DNA
<213> Escherichia coli K-12 MG1655
<220> agay b3138
<222> 453-552
<223> agay b3138 U00096 3280784..3281644
<313> 8881: 465-484
<400> 8881
TGCATTCCTG ACCGATCCAC AAGAAGCTAA ACGCTTTGTC GAACTGACTG 50
GCGTCGACAG CCTGGCGGTA GCGATTGGTA CGGCGCACGG CTTATACAGC 100
<210> 8882

<211> 100
<212> DNA
<213> Escherichia coli K-12 MG1655
<220> ansa b1768
<222> 913-1012
<223> ansa b1768 U00096 1848884..1849900
<313> 8882: 956-975
<400> 8882
ACGCTAACCA AACTGCATTA CCTGCTTAGC CAGGAACTGG ATACTGAAAC 50
CATTCGCAAG GCCATGAGCC AAAACCTGCG CGGCGAACTG ACGCCGGATG 100
<210> 8883
<211> 100
<212> DNA
<213> Escherichia coli K-12 MG1655
<220> ansa b1768
<222> 867-966
<223> ansa b1768 U00096 1848884..1849900
<313> 8883: 921-940
<400> 8883
CGCCCATGCC GGCGTAATTG GCGGTGCAGA TATGACTGTA GAAGCCACGC 50
TAACCAAACT GCATTACCTG CTTAGCCAGG AACTGGATAC TGAAACCATT 100
<210> 8884
<211> 100
<212> DNA
<213> Escherichia coli K-12 MG1655
<220> ansa b1768
<222> 782-881
<223> ansa b1768 U00096 1848884..1849900
<313> 8884: 821-840
<400> 8884
GCGATCGCGG TATTGTGGTG GTCAACCTGA CACAATGTAT GTCCGGTAAA 50
GTGAACATGG GTGGTTATGC CACCGGTAAC GCCCTCGCCC ATGCCGGCGT 100
<210> 8885
<211> 100
<212> DNA
<213> Escherichia coli K-12 MG1655
<220> agaz b3133
<222> 1182-1281
<223> agaz b3133 U00096 3276555..3277835
<313> 8885: 1252-1271
<400> 8885
AGTTCGCTCC GGCGAGCTGC AGCCAACGCC ACGGGAACTC ATTATCAACC 50
ATATTCAGGA CATCCTGGCG CAGTACCACA CAGCCTGTGA AGGCCAATAA 100
<210> 8886
<211> 100
<212> DNA
<213> Escherichia coli K-12 MG1655
<220> agaz b3133
<222> 1182-1281
<223> agaz b3133 U00096 3276555..3277835
<313> 8886: 1206-1225
<400> 8886
AGTTCGCTCC GGCGAGCTGC AGCCAACGCC ACGGGAACTC ATTATCAACC 50
ATATTCAGGA CATCCTGGCG CAGTACCACA CAGCCTGTGA AGGCCAATAA 100
<210> 8887
<211> 100
<212> DNA
<213> Escherichia coli K-12 MG1655
<220> agaz b3133
<222> 1086-1185
<223> agaz b3133 U00096 3276555..3277835
<313> 8887: 1102-1121
<400> 8887
GATTGATGAC GCTTTCGCTC ATCTGGTACG TAATCTGGCG GATTCACCAA 50
TTCCGCTGCC GCTGATCAGC CAGTATCTGC CGCTGCAGTA CGTGAAAGTT 100
<210> 8888
<211> 100
<212> DNA
<213> Escherichia coli K-12 MG1655
<220> ansb b2958
<222> 948-1047

<223> ansb b2958 U00096 complement(3097701..3098747)
<313> 8888: 1022-1041
<400> 8888
TGGCACGCTG AACCCGCAAA AAGCGCGCGT TCTGCTGCAA CTGGCTCTGA 50
CGCAAACCAA AGATCCGCAG CAGATCCAGC AGATCTTCAA TCAGTACTAA 100
<210> 8889
<211> 100
<212> DNA
<213> Escherichia coli K-12 MG1655
<220> ansb b2958
<222> 901-1000
<223> ansb b2958 U00096 complement(3097701..3098747)
<313> 8889: 937-956
<400> 8889
ACTCAGGATG CCGAAGTGGA TGATGCGAAA TACGGCTTCG TCGCCTCTGG 50
CACGCTGAAC CCGCAAAAAG CGCGCGTTCT GCTGCAACTG GCTCTGACGC 100
<210> 8890
<211> 100
<212> DNA
<213> Escherichia coli K-12 MG1655
<220> ansb b2958
<222> 872-971
<223> ansb b2958 U00096 complement(3097701..3098747)
<313> 8890: 901-920
<400> 8890
GTTCTTCCCG CGTACCGACG GGCGCTACCA CTCAGGATGC CGAAGTGGAT 50
GATGCGAAAT ACGGCTTCGT CGCCTCTGGC ACGCTGAACC CGCAAAAAGC 100
<210> 8891
<211> 100
<212> DNA
<213> Escherichia coli K-12 MG1655
<220> phrb b0709
<222> 1320-1419
<223> phrb b0709 U00096 738730..740148
<313> 8891: 1384-1403
<400> 8891
AGCAGGTGTG ACGCTGGATT ATCCGCAACC GATAGTCGAG CACAAAGAAG 50
CGAGAGTACA AACGTTGGCA GCGTATGAGG CGGCGCGGAA GGGGAAATAA 100
<210> 8892
<211> 100
<212> DNA
<213> Escherichia coli K-12 MG1655
<220> phrb b0709
<222> 1318-1417
<223> phrb b0709 U00096 738730..740148
<313> 8892: 1343-1362
<400> 8892
AAAGCAGGTG TGACGCTGGA TTATCCGCAA CCGATAGTCG AGCACAAAGA 50
AGCGAGAGTA CAAACGTTGG CAGCGTATGA GGCGGCGCGG AAGGGGAAAT 100
<210> 8893
<211> 100
<212> DNA
<213> Escherichia coli K-12 MG1655
<220> phrb b0709
<222> 1254-1353
<223> phrb b0709 U00096 738730..740148
<313> 8893: 1312-1331
<400> 8893
GTGGCTACCG GAACTGCGCG ATGTGCCAGG GAAAGTGGTG CATGAGCCGT 50
GGAAGTGGGC GCAGAAAGCA GGTGTGACGC TGGATTATCC GCAACCGATA 100
<210> 8894
<211> 100
<212> DNA
<213> Escherichia coli K-12 MG1655
<220> b0380 b0381
<222> 217-316
<223> b0380 b0381 U00096 398685..399029
<313> 8894: 261-280
<400> 8894
GGGTTACTGG GATTTGAAGC AAAAGGTTGG CAGACCTATG CCGTCGGTCT 50
GGTGACGTGG GTGATTAGTT TCTGGCTGGC GGGGTTGATT ATTCGTCGTC 100

<210> 8895
<211> 100
<212> DNA
<213> Escherichia coli K-12 MG1655
<220> b0380 b0381
<222> 175-274
<223> b0380 b0381 U00096 398685..399029
<313> 8895: 223-242
<400> 8895
GCGTTCGCCA TTGGCCTTAT ATTCATTCTT AATGGCGTGG TGGGGTTACT 50
GGGATTTGAA GCAAAAGGTT GGCAGACCTA TGCCGTCGGT CTGGTGACGT 100
<210> 8896
<211> 100
<212> DNA
<213> Escherichia coli K-12 MG1655
<220> b0380 b0381
<222> 129-228
<223> b0380 b0381 U00096 398685..399029
<313> 8896: 175-194
<400> 8896
TGCCATGAAT CTGCCTGTAA AAATCCGCCG TGACTGGCAC TACTATGCGT 50
TCGCCATTGG CCTTATATTC ATTCTTAATG GCGTGGTGGG GTTACTGGGA 100
<210> 8897
<211> 100
<212> DNA
<213> Escherichia coli K-12 MG1655
<220> ansp ansP
<222> 1440-1539
<223> ansp ansP U00096 complement(1522505..1524055)
<313> 8897: 1493-1512
<400> 8897
AGGCTGGTTT GGTGTGCGCA AACGCGTTGC TGAAATTCAC AGCACTGCGC 50
CAGTCGTCGA GGAAGATGAA GAAAAACAGG AAATTGTGTT TAAGCCTGAA 100
<210> 8898
<211> 100
<212> DNA
<213> Escherichia coli K-12 MG1655
<220> ansp ansP
<222> 1294-1393
<223> ansp ansP U00096 complement(1522505..1524055)
<313> 8898: 1329-1348
<400> 8898
GTCAGTTTTA AACTGCCTGG CGCGCCCTTC ACTTCCTGGC TGACATTACT 50
GTTTTTACTG AGTGTCCTTG TGCTGATGGC GTTCGATTAC CCGAACGGGA 100
<210> 8899
<211> 100
<212> DNA
<213> Escherichia coli K-12 MG1655
<220> ansp ansP
<222> 1226-1325
<223> ansp ansP U00096 complement(1522505..1524055)
<313> 8899: 1291-1310
<400> 8899
CATGGGCGTT TATCATCGTG TGCCAGATGC GCCTGCGTAA AGCGATTAAA 50
GAAGGCAAAG CAGCGGATGT CAGTTTTAAA CTGCCTGGCG CGCCCTTCAC 100
<210> 8900
<211> 100
<212> DNA
<213> Escherichia coli K-12 MG1655
<220> mbha b0231
<222> 687-786
<223> mbha b0231 U00096 250042..250827
<313> 8900: 743-762
<400> 8900
TACGCTGTAT CAATACTTTG GTCAGCATGG GGATAAAGTG GTGCAGCCGC 50
TGGTGCAAAA GCTGGATAAG CAGCAGGTGC TTTCGCAGCG AACGCGTTAA 100
<210> 8901
<211> 100
<212> DNA
<213> Escherichia coli K-12 MG1655
<220> mbha b0231

<222> 673-772
<223> mbha b0231 U00096 250042..250827
<313> 8901: 706-725
<400> 8901
AAAAGTGCGT CCGATACGCT GTATCAATAC TTTGGTCAGC ATGGGGATAA  50
AGTGGTGCAG CCGCTGGTGC AAAAGCTGGA TAAGCAGCAG GTGCTTTCGC  100
<210> 8902
<211> 100
<212> DNA
<213> Escherichia coli K-12 MG1655
<220> mbha b0231
<222> 624-723
<223> mbha b0231 U00096 250042..250827
<313> 8902: 665-684
<400> 8902
AAATCCGCAA AGCGCGGGCA ACCGGCGCAT TGAGATTATG GTGCTGACCA  50
AAAGTGCGTC CGATACGCTG TATCAATACT TTGGTCAGCA TGGGGATAAA  100
<210> 8903
<211> 100
<212> DNA
<213> Escherichia coli K-12 MG1655
<220> foca b0905
<222> 759-858
<223> foca b0905 U00096 complement(952832..953689)
<313> 8903: 823-842
<400> 8903
TAACCTGATT CCGGTTACGA TCGGCAACAT TATCGGTGGT GGTTTGTTGG  50
TTGGGTTGAC ATACTGGGTC ATTTACCTGC GTGAAAACGA CCACCATTAA  100
<210> 8904
<211> 100
<212> DNA
<213> Escherichia coli K-12 MG1655
<220> foca b0905
<222> 628-727
<223> foca b0905 U00096 complement(952832..953689)
<313> 8904: 655-674
<400> 8904
AGTATCGCAA ACATGTTTAT GATCCCGATG GGTATTGTAA TCCGCGACTT  50
CGCATCCCCG GAATTTTGGA CCGCAGTCGG TTCTGCACCG GAAAATTTTT  100
<210> 8905
<211> 100
<212> DNA
<213> Escherichia coli K-12 MG1655
<220> foca b0905
<222> 504-603
<223> foca b0905 U00096 complement(952832..953689)
<313> 8905: 553-572
<400> 8905
TATCCTGGCA AACCTGATGG TATGTCTGGC AGTATGGATG AGTTATTCTG  50
GCCGCAGCCT GATGGACAAA GCGTTCATTA TGGTGCTGCC GGTCGCGATG  100
<210> 8906
<211> 100
<212> DNA
<213> Escherichia coli K-12 MG1655
<220> focb b2493
<222> 681-780
<223> focb b2493 U00096 2611954..2612802
<313> 8906: 746-765
<400> 8906
TCCCCCCTTC TGGCAGCTGG CGCACAGTAG CGCAGACAAT TTTCCGGCAC  50
TGACGGTCAG CCATTTTATT ACCGCCAATC TGCTCCCGGT GATGCTGGGT  100
<210> 8907
<211> 100
<212> DNA
<213> Escherichia coli K-12 MG1655
<220> focb b2493
<222> 643-742
<223> focb b2493 U00096 2611954..2612802
<313> 8907: 668-687
<400> 8907
GTGATTCCGT TCGCCATTGC CATTCGCCAT TTCGCCCCTC CCCCCTTCTG  50

GCAGCTGGCG CACAGTAGCG CAGACAATTT TCCGGCACTG ACGGTCAGCC  100
<210> 8908
<211> 100
<212> DNA
<213> Escherichia coli K-12 MG1655
<220> focb b2493
<222> 577-676
<223> focb b2493 U00096 2611954..2612802
<313> 8908: 628-647
<400> 8908
GCCATGATTT TGCCCATCAC CCTGTTTGTC GCCAGTGGCT TTGAGCACTG  50
TATCGCCAAT TTGTTTGTGA TTCCGTTCGC CATTGCCATT CGCCATTTCG  100
<210> 8909
<211> 100
<212> DNA
<213> Escherichia coli K-12 MG1655
<220> caia b0040
<222> 990-1089
<223> caia b0040 U00096 complement(39244..40386)
<313> 8909: 1041-1060
<400> 8909
TAGCGCAATG CAGGTGCTGG GCGGTGTCGG GATTGCGGGC AACCACCGCA  50
TCAGCCGCTT CTGGCGTGAT CTGCGTGTAG ACCGCGTCTC CGGGGGATCT  100
<210> 8910
<211> 100
<212> DNA
<213> Escherichia coli K-12 MG1655
<220> caia b0040
<222> 951-1050
<223> caia b0040 U00096 complement(39244..40386)
<313> 8910: 985-1004
<400> 8910
CAAATACTTC TGCGCCAATG CGGCATTTGA AGTTGTGGAT AGCGCAATGC  50
AGGTGCTGGG CGGTGTCGGG ATTGCGGGCA ACCACCGCAT CAGCCGCTTC  100
<210> 8911
<211> 100
<212> DNA
<213> Escherichia coli K-12 MG1655
<220> caia b0040
<222> 903-1002
<223> caia b0040 U00096 complement(39244..40386)
<313> 8911: 940-959
<400> 8911
GTGGAAAGCA GACAACGGCA CCATCACCTC TGGCGATGCA GCGATGTGCA  50
AATACTTCTG CGCCAATGCG GCATTTGAAG TTGTGGATAG CGCAATGCAG  100
<210> 8912
<211> 100
<212> DNA
<213> Escherichia coli K-12 MG1655
<220> caib b0039
<222> 1119-1218
<223> caib b0039 U00096 complement(37898..39115)
<313> 8912: 1152-1171
<400> 8912
ACATGGCATG GACACGGCTG CCATTTTGAA AAATATCGGC TACAGCGAAA  50
ACGACATTCA GGAGTTGGTC AGCAAAGGTC TGGCCAAAGT TGAGGACTAA  100
<210> 8913
<211> 100
<212> DNA
<213> Escherichia coli K-12 MG1655
<220> caib b0039
<222> 1068-1167
<223> caib b0039 U00096 complement(37898..39115)
<313> 8913: 1104-1123
<400> 8913
CATGCCGAAA TTCAAAAATA ACCCCGGACA AATCTGGCGC GGAATGCCCT  50
CACATGGCAT GGACACGGCT GCCATTTTGA AAAATATCGG CTACAGCGAA  100
<210> 8914
<211> 100
<212> DNA
<213> Escherichia coli K-12 MG1655

<220> caib b0039
<222> 1016-1115
<223> caib b0039 U00096 complement(37898..39115)
<313> 8914: 1069-1088
<400> 8914
CAATCACTCA GTGGCAAACG ATGGATGGTC GCACCTGCAA AGGGCCGAAC 50
ATCATGCCGA AATTCAAAAA TAACCCCGGA CAAATCTGGC GCGGAATGCC 100
<210> 8915
<211> 100
<212> DNA
<213> Escherichia coli K-12 MG1655
<220> caic b0038
<222> 1454-1553
<223> caic b0038 U00096 complement(36271..37839)
<313> 8915: 1497-1516
<400> 8915
TTTTCCGCTT CTGCGAACAA AATATGGCGA AATTTAAAGT GCCCTCTTAT 50
CTGGAGATCA GAAAAGATCT GCCACGTAAT TGCTCGGGGA AAATAATTAG 100
<210> 8916
<211> 100
<212> DNA
<213> Escherichia coli K-12 MG1655
<220> caic b0038
<222> 1399-1498
<223> caic b0038 U00096 complement(36271..37839)
<313> 8916: 1431-1450
<400> 8916
GCCATCAAAG CATTTGTGGT GCTGAATGAA GGTGAAACAT TGAGCGAAGA 50
GGAATTTTTC CGCTTCTGCG AACAAAATAT GGCGAAATTT AAAGTGCCCT 100
<210> 8917
<211> 100
<212> DNA
<213> Escherichia coli K-12 MG1655
<220> caic b0038
<222> 1299-1398
<223> caic b0038 U00096 complement(36271..37839)
<313> 8917: 1334-1353
<400> 8917
CGAGAATGTC TCCTGCGTGG AGCTGGAAAA TATTATCGCC GCGCACCCGA 50
AAATTCAGGA CATCGTGGTT GTGGGTATTA AAGATTCGAT TCGCGATGAA 100
<210> 8918
<211> 100
<212> DNA
<213> Escherichia coli K-12 MG1655
<220> b0392 b0393
<222> 183-282
<223> b0392 b0393 U00096 407893..408174
<313> 8918: 261-280
<400> 8918
CGAACAACCC GATCTTTCGC TGTTACGTGA TTTAGTGAAA ACCGGGGCAA 50
CGCGGAAAAT TCGCAGTGAA GCGCGTCACC GGCTTGAGGA AAAACAATGA 100
<210> 8919
<211> 100
<212> DNA
<213> Escherichia coli K-12 MG1655
<220> b0392 b0393
<222> 183-282
<223> b0392 b0393 U00096 407893..408174
<313> 8919: 235-254
<400> 8919
CGAACAACCC GATCTTTCGC TGTTACGTGA TTTAGTGAAA ACCGGGGCAA 50
CGCGGAAAAT TCGCAGTGAA GCGCGTCACC GGCTTGAGGA AAAACAATGA 100
<210> 8920
<211> 100
<212> DNA
<213> Escherichia coli K-12 MG1655
<220> b0392 b0393
<222> 183-282
<223> b0392 b0393 U00096 407893..408174
<313> 8920: 238-257
<400> 8920

CGAACAACCC GATCTTTCGC TGTTACGTGA TTTAGTGAAA ACCGGGGCAA 50
CGCGGAAAAT TCGCAGTGAA GCGCGTCACC GGCTTGAGGA AAAACAATGA 100
<210> 8921
<211> 100
<212> DNA
<213> Escherichia coli K-12 MG1655
<220> caid b0037
<222> 795-894
<223> caid b0037 U00096 complement(35377..36270)
<313> 8921: 874-893
<400> 8921
CGTGTTGAAA CACTATCCAT CGGTTCTGCA TTCGGAAGAT GCCATTGAAG 50
GGCCGCTGGC GTTTGCCGAG AAGCGCGATC CGGTGTGGAA AGGACGTTAA 100
<210> 8922
<211> 100
<212> DNA
<213> Escherichia coli K-12 MG1655
<220> caid b0037
<222> 763-862
<223> caid b0037 U00096 complement(35377..36270)
<313> 8922: 811-830
<400> 8922
GTAGAAGAAG CGTATCGCTA TATTCGCAGC GGCGTGTTGA AACACTATCC 50
ATCGGTTCTG CATTCGGAAG ATGCCATTGA AGGGCCGCTG GCGTTTGCCG 100
<210> 8923
<211> 100
<212> DNA
<213> Escherichia coli K-12 MG1655
<220> caid b0037
<222> 734-833
<223> caid b0037 U00096 complement(35377..36270)
<313> 8923: 776-795
<400> 8923
AGATCTACCG CACCACCAGC GAAATGCCGG TAGAAGAAGC GTATCGCTAT 50
ATTCGCAGCG GCGTGTTGAA ACACTATCCA TCGGTTCTGC ATTCGGAAGA 100
<210> 8924
<211> 100
<212> DNA
<213> Escherichia coli K-12 MG1655
<220> caie b0036
<222> 513-612
<223> caie b0036 U00096 complement(34781..35392)
<313> 8924: 570-589
<400> 8924
GCGCTGTCAT GTATCGTTAC ATGAAACGCA GCCACTGAGG CAAATGGAGG 50
AAAATCGCCC CCGTTTGCAG GGGACGACGG ATGTGACGCC TAAACGGTGA 100
<210> 8925
<211> 100
<212> DNA
<213> Escherichia coli K-12 MG1655
<220> caie b0036
<222> 480-579
<223> caie b0036 U00096 complement(34781..35392)
<313> 8925: 531-550
<400> 8925
GTTGAATACC AAAGAGTATC AGGATCTTGT CGGGCGCTGT CATGTATCGT 50
TACATGAAAC GCAGCCACTG AGGCAAATGG AGGAAAATCG CCCCCGTTTG 100
<210> 8926
<211> 100
<212> DNA
<213> Escherichia coli K-12 MG1655
<220> caie b0036
<222> 411-510
<223> caie b0036 U00096 complement(34781..35392)
<313> 8926: 459-478
<400> 8926
CCAGTTGTTG ATGGGTACGC CAGCCCGCGC TGTACGCAAT GTTAGTGACG 50
ACGAGTTACA CTGGAAACGG TTGAATACCA AAGAGTATCA GGATCTTGTC 100
<210> 8927
<211> 100
<212> DNA

<213> Escherichia coli K-12 MG1655
<220> caif b0035
<222> 402-501
<223> caif b0035 U00096 34195..34695
<313> 8927: 457-476
<400> 8927
CGGTGTAAGA AAGACGCCGC GTATTCCTGC CGTTCCGCTC ACGGAACTTA  50
ACCGCGAGCA GAAGTGGCAG ATGATGTTGT CAAAGAGTAT GCGTCGTTAA  100
<210> 8928
<211> 100
<212> DNA
<213> Escherichia coli K-12 MG1655
<220> caif b0035
<222> 272-371
<223> caif b0035 U00096 34195..34695
<313> 8928: 327-346
<400> 8928
GCTGCGAAGT TAAGATGATC CCTAATAAGC TGGAAGGGCG GGGATGCCAG  50
TGTCAGCGAC TGGTTAAAGT GGTCGATATC GATGAGCAAA TTTACGCGCG  100
<210> 8929
<211> 100
<212> DNA
<213> Escherichia coli K-12 MG1655
<220> caif b0035
<222> 134-233
<223> caif b0035 U00096 34195..34695
<313> 8929: 168-187
<400> 8929
TCTACTTGTT AATCGCCGAA TGGATGATGG CTGAAAATCG GTGGGTGATA  50
GCAAGAGAGA TCTCTATTCA TTTCGATATT GAACACAGCA AGGCGGTTAA  100
<210> 8930
<211> 100
<212> DNA
<213> Escherichia coli K-12 MG1655
<220> b0395 b0396
<222> 144-243
<223> b0395 b0396 U00096 410255..410497
<313> 8930: 207-226
<400> 8930
TCCGATGCCT GATGCGACGC TTGCCGCGTC TTATCAGGCC TGCAAAATGT  50
GCCAGAACCG CGTAGGGCGG ATAAGGCGTT CACGCCGCAT CCGGCAATAA  100
<210> 8931
<211> 100
<212> DNA
<213> Escherichia coli K-12 MG1655
<220> b0395 b0396
<222> 92-191
<223> b0395 b0396 U00096 410255..410497
<313> 8931: 127-146
<400> 8931
GCCAGAACCG TAGGCCGGAT AAGGCGTTCA CGCCGCATCC GGCAATAAGT  50
GCTCCGATGC CTGATGCGAC GCTTGCCGCG TCTTATCAGG CCTGCAAAAT  100
<210> 8932
<211> 100
<212> DNA
<213> Escherichia coli K-12 MG1655
<220> b0395 b0396
<222> 1-100
<223> b0395 b0396 U00096 410255..410497
<313> 8932: 34-53
<400> 8932
GTGGTTATGG CCACAAGAGT AAAAAACGTA GGCAATTGGC GCATCATGCC  50
TGATGCGACG CTTGCCGCGT CTTATCAGGC CTACAAAAGG TGCCAGAACC  100
<210> 8933
<211> 100
<212> DNA
<213> Escherichia coli K-12 MG1655
<220> paba b3361
<222> 465-564
<223> paba b3361 U00096 complement(3487903..3488466)
<313> 8933: 533-552

<400> 8933
TCGCCATCGC CAGTGGGATC TGGAAGGTGT GCAGTTCCAT CCAGAAAGTA 50
TTCTTAGCGA ACAAGGACAT CAACTGCTGG CTAATTTCCT GCATCGCTGA 100
<210> 8934
<211> 100
<212> DNA
<213> Escherichia coli K-12 MG1655
<220> paba b3361
<222> 460-559
<223> paba b3361 U00096 complement(3487903..3488466)
<313> 8934: 502-521
<400> 8934
GGGATTCGCC ATCGCCAGTG GGATCTGGAA GGTGTGCAGT TCCATCCAGA 50
AAGTATTCTT AGCGAACAAG GACATCAACT GCTGGCTAAT TTCCTGCATC 100
<210> 8935
<211> 100
<212> DNA
<213> Escherichia coli K-12 MG1655
<220> paba b3361
<222> 399-498
<223> paba b3361 U00096 complement(3487903..3488466)
<313> 8935: 463-482
<400> 8935
ACCTGACTCA TTACCAGCGT GCTTTGACGT GACGGCCTGG AGCGAAACCC 50
GAGAGATTAT GGGGATTCGC CATCGCCAGT GGGATCTGGA AGGTGTGCAG 100
<210> 8936
<211> 100
<212> DNA
<213> Escherichia coli K-12 MG1655
<220> pabb b1813
<222> 1263-1362
<223> pabb b1813 U00096 1892829..1894190
<313> 8936: 1293-1312
<400> 8936
CTCTGCGGGC GGTGGAATTG TCGCCGATAG CCAGGAAGAA GCGGAATATC 50
AGGAAACTTT TGATAAAGTT AATCGTATCC TGAAGCAACT GGAGAAGTAA 100
<210> 8937
<211> 100
<212> DNA
<213> Escherichia coli K-12 MG1655
<220> pabb b1813
<222> 1188-1287
<223> pabb b1813 U00096 1892829..1894190
<313> 8937: 1236-1255
<400> 8937
TTTGAGCTTT TGCGGCAACA TGGATACCAG TATTACTATC CGCACGCTGA 50
CTGCCATTAA CGGACAAATT TTCTGCTCTG CGGGCGGTGG AATTGTCGCC 100
<210> 8938
<211> 100
<212> DNA
<213> Escherichia coli K-12 MG1655
<220> pabb b1813
<222> 1166-1265
<223> pabb b1813 U00096 1892829..1894190
<313> 8938: 1186-1205
<400> 8938
CCTGGTGCGG CAGCATTGGC TATTTGAGCT TTTGCGGCAA CATGGATACC 50
AGTATTACTA TCCGCACGCT GACTGCCATT AACGGACAAA TTTTCTGCTC 100
<210> 8939
<211> 100
<212> DNA
<213> Escherichia coli K-12 MG1655
<220> pabc b1097
<222> 705-804
<223> pabc b1097 U00096 1152523..1153332
<313> 8939: 749-768
<400> 8939
TAATGCGTTA ATGCCAGTGA TGCCCGTATG TGCCTGTGGC GATGTCTCCT 50
TTTCGTCAGC AACGTTATAT GAATATTTAG CCCCACTTTG TGAGCGCCCG 100
<210> 8940
<211> 100

<212> DNA
<213> Escherichia coli K-12 MG1655
<220> pabc b1097
<222> 631-730
<223> pabc b1097 U00096 1152523..1153332
<313> 8940: 667-686
<400> 8940
CAATCCTCTT ATCAGCTTGT CGAAGTGCAA GCCTCTCTGG AAGAGTCGTT 50
GCAGGCAGAT GAGATGGTTA TTTGTAATGC GTTAATGCCA GTGATGCCCG 100
<210> 8941
<211> 100
<212> DNA
<213> Escherichia coli K-12 MG1655
<220> pabc b1097
<222> 557-656
<223> pabc b1097 U00096 1152523..1153332
<313> 8941: 594-613
<400> 8941
TAGTTTATAC GCCGCGACTG GATCAGGCAG GTGTTAACGG CATTATGCGA 50
CAATTCTGTA TCCGTTTGCT GGCACAATCC TCTTATCAGC TTGTCGAAGT 100
<210> 8942
<211> 100
<212> DNA
<213> Escherichia coli K-12 MG1655
<220> glga b3430
<222> 1335-1434
<223> glga b3430 U00096 complement(3564233..3565666)
<313> 8942: 1410-1429
<400> 8942
TTCACTGTGG CGGTTTGTGC AACGTCAGGC TATGGCAATG GATTTTAGCT 50
GGCAGGTCGC GGCGAAGTCG TACCGTGAGC TTTACTATCG CTTGAAATAG 100
<210> 8943
<211> 100
<212> DNA
<213> Escherichia coli K-12 MG1655
<220> glga b3430
<222> 1279-1378
<223> glga b3430 U00096 complement(3564233..3565666)
<313> 8943: 1330-1349
<400> 8943
AATGCCTGGT CGCTGTTACG GGCTATTCGA CGTGCTTTTG TACTGTGGTC 50
CCGTCCTTCA CTGTGGCGGT TTGTGCAACG TCAGGCTATG GCAATGGATT 100
<210> 8944
<211> 100
<212> DNA
<213> Escherichia coli K-12 MG1655
<220> glga b3430
<222> 1242-1341
<223> glga b3430 U00096 complement(3564233..3565666)
<313> 8944: 1290-1309
<400> 8944
AGATGGCGTC GCCAGTGGGT TTGTCTTTGA AGATAGTAAT GCCTGGTCGC 50
TGTTACGGGC TATTCGACGT GCTTTTGTAC TGTGGTCCCG TCCTTCACTG 100
<210> 8945
<211> 100
<212> DNA
<213> Escherichia coli K-12 MG1655
<220> glgb b3433
<222> 2046-2145
<223> glgb b3433 U00096 complement(3568949..3571135)
<313> 8945: 2092-2111
<400> 8945
TTCCATGCAC TATCACGGCA GTAATGCAGG CAATGGCGGC ACGGTACACA 50
GCGATGAGAT TGCCAGCCAC GGTCGTCAGC ATTCACTAAG CCTGACGCTA 100
<210> 8946
<211> 100
<212> DNA
<213> Escherichia coli K-12 MG1655
<220> glgb b3433
<222> 2007-2106
<223> glgb b3433 U00096 complement(3568949..3571135)

<313> 8946: 2046-2065
<400> 8946
AAACCAGCCG GGCAAATGGC GTGAAATCCT CAATACCGAT TCCATGCACT 50
ATCACGGCAG TAATGCAGGC AATGGCGGCA CGGTACACAG CGATGAGATT 100
<210> 8947
<211> 100
<212> DNA
<213> Escherichia coli K-12 MG1655
<220> glgb b3433
<222> 1959-2058
<223> glgb b3433 U00096 complement(3568949..3571135)
<313> 8947: 2001-2020
<400> 8947
TGCCAGTAAC TTTACGCCGG TACCGCGTCA TGATTATCGC TTCGGCATAA 50
ACCAGCCGGG CAAATGGCGT GAAATCCTCA ATACCGATTC CATGCACTAT 100
<210> 8948
<211> 100
<212> DNA
<213> Escherichia coli K-12 MG1655
<220> glgc b3431
<222> 1197-1296
<223> glgc b3431 U00096 complement(3565666..3566961)
<313> 8948: 1276-1295
<400> 8948
CGCAGAGGAA GATGCACGTC GTTTCTATCG TTCAGAAGAA GGCATCGTGC 50
TGGTAACGCG CGAAATGCTA CGGAAGTTAG GGCATAAACA GGAGCGATAA 100
<210> 8949
<211> 100
<212> DNA
<213> Escherichia coli K-12 MG1655
<220> glgc b3431
<222> 1176-1275
<223> glgc b3431 U00096 complement(3565666..3566961)
<313> 8949: 1230-1249
<400> 8949
AGGCATGGTG ATTGGTGAAA ACGCAGAGGA AGATGCACGT CGTTTCTATC 50
GTTCAGAAGA AGGCATCGTG CTGGTAACGC GCGAAATGCT ACGGAAGTTA 100
<210> 8950
<211> 100
<212> DNA
<213> Escherichia coli K-12 MG1655
<220> glgc b3431
<222> 1151-1250
<223> glgc b3431 U00096 complement(3565666..3566961)
<313> 8950: 1193-1212
<400> 8950
TCGATCGTGC TTGTGTTATT CCGGAAGGCA TGGTGATTGG TGAAAACGCA 50
GAGGAAGATG CACGTCGTTT CTATCGTTCA GAAGAAGGCA TCGTGCTGGT 100
<210> 8951
<211> 100
<212> DNA
<213> Escherichia coli K-12 MG1655
<220> cait b0041
<222> 1416-1515
<223> cait b0041 U00096 complement(40417..41931)
<313> 8951: 1474-1493
<400> 8951
TCAAACCGCC ATTATCGCCG GAGGATGCCC GCTGTTCTTC GTCAACATTA 50
TGGTGACGCT CTCCTTTATT AAAGACGCGA AACAGAACTG GAAAGATTAA 100
<210> 8952
<211> 100
<212> DNA
<213> Escherichia coli K-12 MG1655
<220> cait b0041
<222> 1368-1467
<223> cait b0041 U00096 complement(40417..41931)
<313> 8952: 1414-1433
<400> 8952
TGGCATTATC GGTATTGTTC TGCTGGCGCT CGGCGGCCTG AAACCGATTC 50
AAACCGCCAT TATCGCCGGA GGATGCCCGC TGTTCTTCGT CAACATTATG 100
<210> 8953

<211> 100
<212> DNA
<213> Escherichia coli K-12 MG1655
<220> cait b0041
<222> 1254-1353
<223> cait b0041 U00096 complement(40417..41931)
<313> 8953: 1305-1324
<400> 8953
CACCGTTACG CTGGTTAACG CCTGCTCTTA TACCCTGGCG ATGTCCACTT 50
GCCGCGAAGT ACGCGATGGT GAAGAACCAC CTCTGCTGGT GCGTATCGGT 100
<210> 8954
<211> 100
<212> DNA
<213> Escherichia coli K-12 MG1655
<220> ppda b2827
<222> 372-471
<223> ppda b2827 U00096 complement(2961729..2962199)
<313> 8954: 442-461
<400> 8954
TATTCGCTTC AAAAACTCAA CGGGCGAGTG GTGGCTGGTG GTTTCGCCGT 50
GGGGAAGACT CCGGCTTTGT CAGCAAGGAG AAACAGAAGG ATGCCTGTAA 100
<210> 8955
<211> 100
<212> DNA
<213> Escherichia coli K-12 MG1655
<220> ppda b2827
<222> 347-446
<223> ppda b2827 U00096 complement(2961729..2962199)
<313> 8955: 393-412
<400> 8955
TGCGCAATAC CGCATGGGCC GGGCATATTC GCTTCAAAAA CTCAACGGGC 50
GAGTGGTGGC TGGTGGTTTC GCCGTGGGGA AGACTCCGGC TTTGTCAGCA 100
<210> 8956
<211> 100
<212> DNA
<213> Escherichia coli K-12 MG1655
<220> ppda b2827
<222> 240-339
<223> ppda b2827 U00096 complement(2961729..2962199)
<313> 8956: 290-309
<400> 8956
CGCTGCTGGG GCCAATACCT GTCATGGCAG TTCACCATTG GTCTTTGTGC 50
CACGCTGGCC CGAAGTCGAA ATGAGCGACC TGACACCTTC GCTTGCTTTC 100
<210> 8957
<211> 100
<212> DNA
<213> Escherichia coli K-12 MG1655
<220> ppdb b2826
<222> 461-560
<223> ppdb b2826 U00096 complement(2961175..2961738)
<313> 8957: 494-513
<400> 8957
GCGGCTTCTC GCCGGTGTTG ACGGTTAATA TGCGTGCTGC CAGTAAGTCT 50
GAACCGCAAA CCGTGGTGAA TGCCAGCTAT AGCGTGACAG GATTCAACCT 100
<210> 8958
<211> 100
<212> DNA
<213> Escherichia coli K-12 MG1655
<220> ppdb b2826
<222> 336-435
<223> ppdb b2826 U00096 complement(2961175..2961738)
<313> 8958: 388-407
<400> 8958
TCTGAAGGAG CATGTGCTGG AAACGCTACG CGGTGCGACA TCCTGTGAAG 50
GTAAGGGCTG GGATAAAGTC ACTAATCCGG ATGCCATCAT TATCGACACT 100
<210> 8959
<211> 100
<212> DNA
<213> Escherichia coli K-12 MG1655
<220> ppdb b2826
<222> 202-301

<223> ppdb b2826 U00096 complement(2961175..2961738)
<313> 8959: 232-251
<400> 8959
TGTCATGGCA TCTGTACCGG CGAAGGGCTG GAAATTGTCG GACAGGGTGA 50
CTGTGTCATT GTGCAGTGGG ATGCGAACAG TAACGGTATC TGGGATCGCG 100
<210> 8960
<211> 100
<212> DNA
<213> Escherichia coli K-12 MG1655
<220> ppdc b2824
<222> 225-324
<223> ppdc b2824 U00096 complement(2960463..2960786)
<313> 8960: 303-322
<400> 8960
GACATCGCAG GCGGGATGTG TCAGCATCAG CGTTACGCTA GTTTCACCCG 50
GGGGCAGAGA AGGCGAGATG ACCCGCCTGC ATTGCCCGAA TCGTCAGTAG 100
<210> 8961
<211> 100
<212> DNA
<213> Escherichia coli K-12 MG1655
<220> ppdc b2824
<222> 198-297
<223> ppdc b2824 U00096 complement(2960463..2960786)
<313> 8961: 249-268
<400> 8961
TGCCAACTGG CAGGTCAACC GAATGCAGAC ATCGCAGGCG GGATGTGTCA 50
GCATCAGCGT TACGCTAGTT TCACCCGGGG GCAGAGAAGG CGAGATGACC 100
<210> 8962
<211> 100
<212> DNA
<213> Escherichia coli K-12 MG1655
<220> ppdc b2824
<222> 106-205
<223> ppdc b2824 U00096 complement(2960463..2960786)
<313> 8962: 158-177
<400> 8962
ACATTAATGA ACAGTCTTGC CAGCAGAAAC CAGTACCAAC AGCTCTGGCG 50
GCATGGCTGG CAGCAAACGC AACTGCGCGC GATTTCGCCA CCTGCCAACT 100
<210> 8963
<211> 100
<212> DNA
<213> Escherichia coli K-12 MG1655
<220> ppdd b0109
<222> 342-441
<223> ppdd b0109 U00096 complement(117109..117549)
<313> 8963: 386-405
<400> 8963
AAACGGCGTC ACCGGCTGGA CGCGCAACTG CAATATTCAA AGTGACAGCG 50
CATTGCAGCA AGCCTGCGAA GATGTCTTCC GCTTTGATGA CGCCAACTAA 100
<210> 8964
<211> 100
<212> DNA
<213> Escherichia coli K-12 MG1655
<220> ppdd b0109
<222> 242-341
<223> ppdd b0109 U00096 complement(117109..117549)
<313> 8964: 292-311
<400> 8964
ATGTTTCAGC CATGAGTGTG GCAAAGGGCG TGGTGTCGCT GACCGGGCAA 50
GAAAGTCTCA ATGGGCTAAG CGTCGTCATG ACACCGGGTT GGGATAACGC 100
<210> 8965
<211> 100
<212> DNA
<213> Escherichia coli K-12 MG1655
<220> ppdd b0109
<222> 192-291
<223> ppdd b0109 U00096 complement(117109..117549)
<313> 8965: 225-244
<400> 8965
TACCTGCGAC GGTGGCAGCA ATGGCATTCC CTCGCCTACC ACCACCCGCT 50
ATGTTTCAGC CATGAGTGTG GCAAAGGGCG TGGTGTCGCT GACCGGGCAA 100

<210> 8966
<211> 100
<212> DNA
<213> Escherichia coli K-12 MG1655
<220> glgp b3429
<222> 2349-2448
<223> glgp b3429 U00096 complement(3561767..3564214)
<313> 8966: 2422-2441
<400> 8966
AGCGATGCTG AACATTGCCA ATATGGGCTA CTTCTCTTCT GACCGTACTA 50
TCAAAGAGTA CGCCGATCAT ATCTGGCATA TCGATCCGGT GAGATTGTAA 100
<210> 8967
<211> 100
<212> DNA
<213> Escherichia coli K-12 MG1655
<220> glgp b3429
<222> 2314-2413
<223> glgp b3429 U00096 complement(3561767..3564214)
<313> 8967: 2369-2388
<400> 8967
GAACTCTACG AGCTTCAGGA AGAGTGGACC GCAAAAGCGA TGCTGAACAT 50
TGCCAATATG GGCTACTTCT CTTCTGACCG TACTATCAAA GAGTACGCCG 100
<210> 8968
<211> 100
<212> DNA
<213> Escherichia coli K-12 MG1655
<220> glgp b3429
<222> 2292-2391
<223> glgp b3429 U00096 complement(3561767..3564214)
<313> 8968: 2315-2334
<400> 8968
CGATTGTCAG GATAAAGTCG ATGAACTCTA CGAGCTTCAG GAAGAGTGGA 50
CCGCAAAAGC GATGCTGAAC ATTGCCAATA TGGGCTACTT CTCTTCTGAC 100
<210> 8969
<211> 100
<212> DNA
<213> Escherichia coli K-12 MG1655
<220> b2503 b2503
<222> 2073-2172
<223> b2503 b2503 U00096 complement(2624715..2626958)
<313> 8969: 2117-2136
<400> 8969
CCTGGCGCGA ATGAAGAAAA TGCTGGTAGT GGCAGAGTAC GTTGAAAACG 50
AAGAGATCCG CGAGGCGGTG CTCTCTTTGG GGATCGATTA TATGCAGGGT 100
<210> 8970
<211> 100
<212> DNA
<213> Escherichia coli K-12 MG1655
<220> b2503 b2503
<222> 1925-2024
<223> b2503 b2503 U00096 complement(2624715..2626958)
<313> 8970: 1977-1996
<400> 8970
AGATTGCGAT TGATGATTTC GGCACCGGCT ACGCCAGCTA TGCGCGGCTT 50
AAAAATGTGA ATGCCGATCT GCTTAAAATT GACGGCAGTT TTATCCGCAA 100
<210> 8971
<211> 100
<212> DNA
<213> Escherichia coli K-12 MG1655
<220> b2503 b2503
<222> 1854-1953
<223> b2503 b2503 U00096 complement(2624715..2626958)
<313> 8971: 1894-1913
<400> 8971
CGAAAGTAAT GCTCTGACCA ATGTTAAGCA GGCGCAAATC ACCTTGCAGC 50
ATCTTCAGGA ATTAGGCTGC CAGATTGCGA TTGATGATTT CGGCACCGGC 100
<210> 8972
<211> 100
<212> DNA
<213> Escherichia coli K-12 MG1655
<220> b2504 b2504

<222> 93-192
<223> b2504 b2504 U00096 2627310..2627501
<313> 8972: 147-166
<400> 8972
CCGTTTTATC TACTCGTCCG TCGGTGCCTG GCAGCACCAT CAGAGCAAAA  50
AAGAAGCTCA GCAATCCACA CTCTCCGTCG AATCACCGGT ACAACGTTAG  100
<210> 8973
<211> 100
<212> DNA
<213> Escherichia coli K-12 MG1655
<220> b2504 b2504
<222> 14-113
<223> b2504 b2504 U00096 2627310..2627501
<313> 8973: 57-76
<400> 8973
CCAGTATGCG AAAACGACAC CGATTTAACA GTCGCATGAC CCGTATCGTA  50
CTGCTCATCA GCTTTATCTT CTTCTTTGGC CGTTTTATCT ACTCGTCCGT  100
<210> 8974
<211> 100
<212> DNA
<213> Escherichia coli K-12 MG1655
<220> b2504 b2504
<222> 1-100
<223> b2504 b2504 U00096 2627310..2627501
<313> 8974: 26-45
<400> 8974
GTGAGTCAGG CAACCAGTAT GCGAAAACGA CACCGATTTA ACAGTCGCAT  50
GACCCGTATC GTACTGCTCA TCAGCTTTAT CTTCTTCTTT GGCCGTTTTA  100
<210> 8975
<211> 100
<212> DNA
<213> Escherichia coli K-12 MG1655
<220> glgs glgS
<222> 102-201
<223> glgs glgS U00096 complement(3189755..3189955)
<313> 8975: 169-188
<400> 8975
TGGTAACATG GATGAAGAAC ATAGAACCTG GTTTTGCGCA CGTTATGCCT  50
GGTATTGTCA ACAGATGATG CAGGCAAGAG AGCTGGAGTT AGAGCACTGA  100
<210> 8976
<211> 100
<212> DNA
<213> Escherichia coli K-12 MG1655
<220> glgs glgS
<222> 82-181
<223> glgs glgS U00096 complement(3189755..3189955)
<313> 8976: 104-123
<400> 8976
GTCGATATTC TGGCCGTTAC TGGTAACATG GATGAAGAAC ATAGAACCTG  50
GTTTTGCGCA CGTTATGCCT GGTATTGTCA ACAGATGATG CAGGCAAGAG  100
<210> 8977
<211> 100
<212> DNA
<213> Escherichia coli K-12 MG1655
<220> glgs glgS
<222> 51-150
<223> glgs glgS U00096 complement(3189755..3189955)
<313> 8977: 65-84
<400> 8977
TTTTGCCAGA ATGCACGCAG AAGGTCGCCC GGTCGATATT CTGGCCGTTA  50
CTGGTAACAT GGATGAAGAA CATAGAACCT GGTTTTGCGC ACGTTATGCC  100
<210> 8978
<211> 100
<212> DNA
<213> Escherichia coli K-12 MG1655
<220> b2505 b2505
<222> 395-494
<223> b2505 b2505 U00096 2627812..2628330
<313> 8978: 436-455
<400> 8978
AAGTGTATAC CTCTACCCAG GTGGGTAAAG AGTGCCAGTT TACGACAGGT  50

TTAGCCGTTG TTATTACCAC GACGTATAAC GAAACGCGTA TTCAGCCAAA 100
<210> 8979
<211> 100
<212> DNA
<213> Escherichia coli K-12 MG1655
<220> b2505 b2505
<222> 307-406
<223> b2505 b2505 U00096 2627812..2628330
<313> 8979: 324-343
<400> 8979
GGCGGAGCTG TAGGCGCGGC AGCGGGTTCT ATGGTGAATG ATAAAACCTT 50
AGTGGAAGGT GTTTCTTTAA CCTATAAGGA AGGCACCAAA GTGTATACCT 100
<210> 8980
<211> 100
<212> DNA
<213> Escherichia coli K-12 MG1655
<220> b2505 b2505
<222> 267-366
<223> b2505 b2505 U00096 2627812..2628330
<313> 8980: 296-315
<400> 8980
GTCTGGCAGC AATTCCGGAA CGACGGCAGG TGCAGTTGGC GGCGGAGCTG 50
TAGGCGCGGC AGCGGGTTCT ATGGTGAATG ATAAAACCTT AGTGGAAGGT 100
<210> 8981
<211> 100
<212> DNA
<213> Escherichia coli K-12 MG1655
<220> b2506 b2507
<222> 441-540
<223> b2506 b2507 U00096 2628346..2628885
<313> 8981: 503-522
<400> 8981
TGAAGCTGAC GCCAACAGAA ATATGACTGA AGGCGGTCGC GATCTGATGA 50
AAAGCGTGGG CAAAGCAGAA GAGAACAAAT CGGACAGCTG GTTTAATTAA 100
<210> 8982
<211> 100
<212> DNA
<213> Escherichia coli K-12 MG1655
<220> b2506 b2507
<222> 393-492
<223> b2506 b2507 U00096 2628346..2628885
<313> 8982: 444-463
<400> 8982
CGATCAGGAA CTGAAGCACA AAGCTGCGCA AACCGATGTG GTGCAATCTG 50
AAGCTGACGC CAACAGAAAT ATGACTGAAG GCGGTCGCGA TCTGATGAAA 100
<210> 8983
<211> 100
<212> DNA
<213> Escherichia coli K-12 MG1655
<220> b2506 b2507
<222> 343-442
<223> b2506 b2507 U00096 2628346..2628885
<313> 8983: 397-416
<400> 8983
CTGGCGCTGG CGAAAGAAGA AGCCCGCGTT AAGCGAGAAA ACGAATTTAT 50
CGATCAGGAA CTGAAGCACA AAGCTGCGCA AACCGATGTG GTGCAATCTG 100
<210> 8984
<211> 100
<212> DNA
<213> Escherichia coli K-12 MG1655
<220> glgx b3432
<222> 1833-1932
<223> glgx b3432 U00096 complement(3566979..3568952)
<313> 8984: 1895-1914
<400> 8984
TAACGCCACG CTTGAGGTAA CAGAGATTGT TTTACCTGCT GGGGAGTGGC 50
ACGCCATTCC CCCATTCGCT GGAGAGGATA ACCCAGTGAT TACGGCTGTC 100
<210> 8985
<211> 100
<212> DNA
<213> Escherichia coli K-12 MG1655

<220> glgx b3432
<222> 1808-1907
<223> glgx b3432 U00096 complement(3566979..3568952)
<313> 8985: 1850-1869
<400> 8985
TCTCGGATCG CTTTTTGATC GCAATTAACG CCACGCTTGA GGTAACAGAG 50
ATTGTTTTAC CTGCTGGGGA GTGGCACGCC ATTCCCCCAT TCGCTGGAGA 100
<210> 8986
<211> 100
<212> DNA
<213> Escherichia coli K-12 MG1655
<220> glgx b3432
<222> 1780-1879
<223> glgx b3432 U00096 complement(3566979..3568952)
<313> 8986: 1802-1821
<400> 8986
AACGGGCCGA AACAGCTGCA AATTCTGCTC TCGGATCGCT TTTTGATCGC 50
AATTAACGCC ACGCTTGAGG TAACAGAGAT TGTTTTACCT GCTGGGGAGT 100
<210> 8987
<211> 100
<212> DNA
<213> Escherichia coli K-12 MG1655
<220> kbl b3618
<222> 1048-1147
<223> kbl b3618 U00096 complement(3788982..3790178)
<313> 8987: 1079-1098
<400> 8987
ATTTACGTTA CCGGTTTCTT CTATCCGGTC GTTCCGAAAG GTCAGGCGCG 50
TATTCGTACC CAGATGTCTG CGGCGCATAC CCCTGAGCAA ATTACGCGTG 100
<210> 8988
<211> 100
<212> DNA
<213> Escherichia coli K-12 MG1655
<220> kbl b3618
<222> 991-1090
<223> kbl b3618 U00096 complement(3788982..3790178)
<313> 8988: 1037-1056
<400> 8988
ATGCTTGGTG ATGCGGTAGT GGCGCAGAAA TTTGCCCGTG AGCTGCAAAA 50
AGAGGGCATT TACGTTACCG GTTTCTTCTA TCCGGTCGTT CCGAAAGGTC 100
<210> 8989
<211> 100
<212> DNA
<213> Escherichia coli K-12 MG1655
<220> kbl b3618
<222> 950-1049
<223> kbl b3618 U00096 complement(3788982..3790178)
<313> 8989: 986-1005
<400> 8989
GCTTTACCCT GGCGGGAGCC GATCACGCCA TTATTCCGGT CATGCTTGGT 50
GATGCGGTAG TGGCGCAGAA ATTTGCCCGT GAGCTGCAAA AAGAGGGCAT 100
<210> 8990
<211> 100
<212> DNA
<213> Escherichia coli K-12 MG1655
<220> crl b0241
<222> 303-402
<223> crl b0241 U00096 257829..258230
<313> 8990: 374-393
<400> 8990
TGAGTTTCAC GAGAAGCTGC GTGAACTGCT GACGACGCTG AATCTGAAGC 50
TGGAACCGGC GGATGATTTT CGTGACGAGC CGGTGAAGTT AACGGCGTGA 100
<210> 8991
<211> 100
<212> DNA
<213> Escherichia coli K-12 MG1655
<220> crl b0241
<222> 293-392
<223> crl b0241 U00096 257829..258230
<313> 8991: 325-344
<400> 8991

ACACCCTGCG TGAGTTTCAC GAGAAGCTGC GTGAACTGCT GACGACGCTG 50
AATCTGAAGC TGGAACCGGC GGATGATTTT CGTGACGAGC CGGTGAAGTT 100
<210> 8992
<211> 100
<212> DNA
<213> Escherichia coli K-12 MG1655
<220> crl b0241
<222> 244-343
<223> crl b0241 U00096 257829..258230
<313> 8992: 288-307
<400> 8992
TGGAAGAGTG TTCCGGTAAA AGACACTGAA GTGGTTGAAC GACTGGAGCA 50
CACCCTGCGT GAGTTTCACG AGAAGCTGCG TGAACTGCTG ACGACGCTGA 100
<210> 8993
<211> 100
<212> DNA
<213> Escherichia coli K-12 MG1655
<220> uida b1618
<222> 1713-1812
<223> uida b1618 U00096 complement(1692284..1694095)
<313> 8993: 1774-1793
<400> 8993
CACTCGCGAC CGCAAACCGA AGTCGGCGGC TTTTCTGCTG CAAAAACGCT 50
GGACTGGCAT GAACTTCGGT GAAAAACCGC AGCAGGGAGG CAAACAATGA 100
<210> 8994
<211> 100
<212> DNA
<213> Escherichia coli K-12 MG1655
<220> uida b1618
<222> 1711-1810
<223> uida b1618 U00096 complement(1692284..1694095)
<313> 8994: 1740-1759
<400> 8994
TTCACTCGCG ACCGCAAACC GAAGTCGGCG GCTTTTCTGC TGCAAAAACG 50
CTGGACTGGC ATGAACTTCG GTGAAAAACC GCAGCAGGGA GGCAAACAAT 100
<210> 8995
<211> 100
<212> DNA
<213> Escherichia coli K-12 MG1655
<220> uida b1618
<222> 1555-1654
<223> uida b1618 U00096 complement(1692284..1694095)
<313> 8995: 1604-1623
<400> 8995
GACATGTGGA GTGAAGAGTA TCAGTGTGCA TGGCTGGATA TGTATCACCG 50
CGTCTTTGAT CGCGTCAGCG CCGTCGTCGG TGAACAGGTA TGGAATTTCG 100
<210> 8996
<211> 100
<212> DNA
<213> Escherichia coli K-12 MG1655
<220> uidb b1617
<222> 1187-1286
<223> uidb b1617 U00096 complement(1690914..1692287)
<313> 8996: 1229-1248
<400> 8996
TGCAAACGCC GGAAGTTATT ATGGGCATCC GCACATCAAT TGCCTTAGTA 50
CCTTGCGGAT TTATGCTACT GGCATTCGTT ATTATCTGGT TTTATCCGCT 100
<210> 8997
<211> 100
<212> DNA
<213> Escherichia coli K-12 MG1655
<220> uidb b1617
<222> 1145-1244
<223> uidb b1617 U00096 complement(1690914..1692287)
<313> 8997: 1197-1216
<400> 8997
CTGCCTTTAT TTTGGGGTTA AGCGGATATA TCGCCAATCA GGTGCAAACG 50
CCGGAAGTTA TTATGGGCAT CCGCACATCA ATTGCCTTAG TACCTTGCGG 100
<210> 8998
<211> 100
<212> DNA

<213> Escherichia coli K-12 MG1655
<220> uidb b1617
<222> 1087-1186
<223> uidb b1617 U00096 complement(1690914..1692287)
<313> 8998: 1136-1155
<400> 8998
ACCTATTCAC TATTCTCATT TACCCGTAAA TGCGGTCAGG CAATCGGAGG 50
TTCAATTCCT GCCTTTATTT TGGGGTTAAG CGGATATATC GCCAATCAGG 100
<210> 8999
<211> 100
<212> DNA
<213> Escherichia coli K-12 MG1655
<220> crp b3358
<222> 534-633
<223> crp b3358 U00096 3483757..3484389
<313> 8999: 600-619
<400> 8999
CTGTTCTCGT GAAACCGTGG GACGCATTCT GAAGATGCTG GAAGATCAGA 50
ACCTGATCTC CGCACACGGT AAAACCATCG TCGTTTACGG CACTCGTTAA 100
<210> 9000
<211> 100
<212> DNA
<213> Escherichia coli K-12 MG1655
<220> crp b3358
<222> 502-601
<223> crp b3358 U00096 3483757..3484389
<313> 9000: 564-583
<400> 9000
ATTACCCGTC AGGAAATTGG TCAGATTGTC GGCTGTTCTC GTGAAACCGT 50
GGGACGCATT CTGAAGATGC TGGAAGATCA GAACCTGATC TCCGCACACG 100
<210> 9001
<211> 100
<212> DNA
<213> Escherichia coli K-12 MG1655
<220> crp b3358
<222> 474-573
<223> crp b3358 U00096 3483757..3484389
<313> 9001: 519-538
<400> 9001
GACTCACCCG GACGGTATGC AAATCAAAAT TACCCGTCAG GAAATTGGTC 50
AGATTGTCGG CTGTTCTCGT GAAACCGTGG GACGCATTCT GAAGATGCTG 100
<210> 9002
<211> 100
<212> DNA
<213> Escherichia coli K-12 MG1655
<220> uidc b1616
<222> 1123-1222
<223> uidc b1616 U00096 complement(1689610..1690863)
<313> 9002: 1164-1183
<400> 9002
CCGTTACAAG GGATGAATTT CTTTCTTGCT GCCGATAAAG GGCGGGAAAA 50
GCGCGATGGC AGTACGCTGG GCGATCGCCT GAATTACTGG GATGTGAAAA 100
<210> 9003
<211> 100
<212> DNA
<213> Escherichia coli K-12 MG1655
<220> uidc b1616
<222> 1074-1173
<223> uidc b1616 U00096 complement(1689610..1690863)
<313> 9003: 1128-1147
<400> 9003
TGAAGGCACG GGTATCTTAG GTGGTATGAA CTATCGGGTC AGCGAAGGGC 50
CGTTACAAGG GATGAATTTC TTTCTTGCTG CCGATAAAGG GCGGGAAAAG 100
<210> 9004
<211> 100
<212> DNA
<213> Escherichia coli K-12 MG1655
<220> uidc b1616
<222> 1029-1128
<223> uidc b1616 U00096 complement(1689610..1690863)
<313> 9004: 1082-1101

<400> 9004
GATTGTGACT CGCGGCTATG AATCCAGTAA ACGAGATGTG CGGATTGAAG 50
GCACGGGTAT CTTAGGTGGT ATGAACTATC GGGTCAGCGA AGGGCCGTTA 100
<210> 9005
<211> 100
<212> DNA
<213> Escherichia coli K-12 MG1655
<220> crr b2418
<222> 411-510
<223> crr b2418 U00096 2533854..2534363
<313> 9005: 459-478
<400> 9005
TCCGGTTGTT ATCTCCAACA TGGACGAAAT CAAAGAACTG ATCAAACTGT 50
CCGGTAGCGT AACCGTGGGT GAAACCCCGG TTATCCGCAT CAAGAAGTAA 100
<210> 9006
<211> 100
<212> DNA
<213> Escherichia coli K-12 MG1655
<220> crr b2418
<222> 366-465
<223> crr b2418 U00096 2533854..2534363
<313> 9006: 414-433
<400> 9006
ATTTGATCTG CCGCTGCTGG AAGAGAAAGC CAAGTCTACC CTGACTCCGG 50
TTGTTATCTC CAACATGGAC GAAATCAAAG AACTGATCAA ACTGTCCGGT 100
<210> 9007
<211> 100
<212> DNA
<213> Escherichia coli K-12 MG1655
<220> crr b2418
<222> 319-418
<223> crr b2418 U00096 2533854..2534363
<313> 9007: 367-386
<400> 9007
ATTGCTGAAG AAGGTCAGCG CGTGAAAGTT GGCGATACTG TCATTGAATT 50
TGATCTGCCG CTGCTGGAAG AGAAAGCCAA GTCTACCCTG ACTCCGGTTG 100
<210> 9008
<211> 100
<212> DNA
<213> Escherichia coli K-12 MG1655
<220> b2510 b2510
<222> 148-247
<223> b2510 b2510 U00096 complement(2633619..2633870)
<313> 9008: 188-207
<400> 9008
CATCAACCGT TGCAGGTGTT AAAAGCCTGT GGTGCAGTAG ATTACTTCTG 50
TCAGCACGGG CATGGCTTGA TATCGAAAAA ACGCGTTGAA TTCGTGCTGG 100
<210> 9009
<211> 100
<212> DNA
<213> Escherichia coli K-12 MG1655
<220> b2510 b2510
<222> 117-216
<223> b2510 b2510 U00096 complement(2633619..2633870)
<313> 9009: 180-199
<400> 9009
TATTGAAATG AAAGCTCTTT GTCCAGACTG CCATCAACCG TTGCAGGTGT 50
TAAAAGCCTG TGGTGCAGTA GATTACTTCT GTCAGCACGG GCATGGCTTG 100
<210> 9010
<211> 100
<212> DNA
<213> Escherichia coli K-12 MG1655
<220> b2510 b2510
<222> 108-207
<223> b2510 b2510 U00096 complement(2633619..2633870)
<313> 9010: 136-155
<400> 9010
CGGGGAATTT ATTGAAATGA AAGCTCTTTG TCCAGACTGC CATCAACCGT 50
TGCAGGTGTT AAAAGCCTGT GGTGCAGTAG ATTACTTCTG TCAGCACGGG 100
<210> 9011
<211> 100

<212> DNA
<213> Escherichia coli K-12 MG1655
<220> b2511 b2511
<222> 1413-1512
<223> b2511 b2511 U00096 complement(2633904..2635415)
<313> 9011: 1472-1491
<400> 9011
AGAAGGGGAA AACCCGTATG CGAATAAGCG TAACACCCTG ACGCCAACCC 50
AGATGCGTAA ACGTAAGCGT CTGATGAAGC ACATCAAGAA AAATAAATAA 100
<210> 9012
<211> 100
<212> DNA
<213> Escherichia coli K-12 MG1655
<220> b2511 b2511
<222> 1345-1444
<223> b2511 b2511 U00096 complement(2633904..2635415)
<313> 9012: 1394-1413
<400> 9012
CGCTACTTGA TGAACTACTT CCGCAAATCG CTGGACGTAA TGGGATCGCC 50
GATTCGTATT CAGTTCAAAG AAGGGGAAAA CCCGTATGCG AATAAGCGTA 100
<210> 9013
<211> 100
<212> DNA
<213> Escherichia coli K-12 MG1655
<220> b2511 b2511
<222> 1306-1405
<223> b2511 b2511 U00096 complement(2633904..2635415)
<313> 9013: 1359-1378
<400> 9013
CACGGTAATC AGGTGAAAGA CCTGCCTGAT TCCTACAAGC GCTACTTGAT 50
GAACTACTTC CGCAAATCGC TGGACGTAAT GGGATCGCCG ATTCGTATTC 100
<210> 9014
<211> 100
<212> DNA
<213> Escherichia coli K-12 MG1655
<220> b2512 b2512
<222> 1080-1179
<223> b2512 b2512 U00096 complement(2635494..2636672)
<313> 9014: 1152-1171
<400> 9014
AAAAGTTGAT AGTTCCGGTT TCCAGACTGA ACCGGTTGCC GCTGACGGCA 50
AACTGCTGAT CCAGGCAAAA GACGGAACCG TGTACTCTAT TACACGTTAA 100
<210> 9015
<211> 100
<212> DNA
<213> Escherichia coli K-12 MG1655
<220> b2512 b2512
<222> 1028-1127
<223> b2512 b2512 U00096 complement(2635494..2636672)
<313> 9015: 1072-1091
<400> 9015
AAGGTTATCT GCACTGGATT AACGTCGAAG ATGGTCGTTT CGTTGCCCAG 50
CAAAAAGTTG ATAGTTCCGG TTTCCAGACT GAACCGGTTG CCGCTGACGG 100
<210> 9016
<211> 100
<212> DNA
<213> Escherichia coli K-12 MG1655
<220> b2512 b2512
<222> 976-1075
<223> b2512 b2512 U00096 complement(2635494..2636672)
<313> 9016: 1036-1055
<400> 9016
CTGCTGACTT CTCCGGTGCT GTATAATGGC AACCTGGTGG TCGGTGACAG 50
TGAAGGTTAT CTGCACTGGA TTAACGTCGA AGATGGTCGT TTCGTTGCCC 100
<210> 9017
<211> 100
<212> DNA
<213> Escherichia coli K-12 MG1655
<220> b2513 b2514
<222> 522-621
<223> b2513 b2514 U00096 complement(2636683..2637303)

<313> 9017: 582-601
<400> 9017
GCAAGGTGCG CGTAGTGCAT GGGAAGCAGG CGTGAAAAGC GATGTTACTC 50
CGGCACTGAG CGAAATGATG CAGATGAAAA TTAATAATTT GTCCATCTGA 100
<210> 9018
<211> 100
<212> DNA
<213> Escherichia coli K-12 MG1655
<220> b2513 b2514
<222> 479-578
<223> b2513 b2514 U00096 complement(2636683..2637303)
<313> 9018: 493-512
<400> 9018
TTGCCGACCT GCGTGGTGAA GCATTGCTGA GCAAAGGTGA TAAGCAAGGT 50
GCGCGTAGTG CATGGGAAGC AGGCGTGAAA AGCGATGTTA CTCCGGCACT 100
<210> 9019
<211> 100
<212> DNA
<213> Escherichia coli K-12 MG1655
<220> b2513 b2514
<222> 408-507
<223> b2513 b2514 U00096 complement(2636683..2637303)
<313> 9019: 468-487
<400> 9019
ACAGCTCAAG CAGGCTGATG CCGCGCTGAA AACCCTTGAT ACCATCAAAG 50
GTGAAGGGTG GGCTGCCATT GTTGCCGACC TGCGTGGTGA AGCATTGCTG 100
<210> 9020
<211> 100
<212> DNA
<213> Escherichia coli K-12 MG1655
<220> rtn b2177
<222> 1458-1557
<223> rtn b2177 U00096 2268746..2270302
<313> 9020: 1521-1540
<400> 9020
ACGCGGCGTT AATTTCATGC AAGGCTACTG GATTAGCCGC CCGTTACCGC 50
TGGACGATTT TGTTCGCTGG CTAAAGAAAC CGTATACGCC GCAGTGGTAA 100
<210> 9021
<211> 100
<212> DNA
<213> Escherichia coli K-12 MG1655
<220> rtn b2177
<222> 1448-1547
<223> rtn b2177 U00096 2268746..2270302
<313> 9021: 1471-1490
<400> 9021
GGCTAAGCGA ACGCGGCGTT AATTTCATGC AAGGCTACTG GATTAGCCGC 50
CCGTTACCGC TGGACGATTT TGTTCGCTGG CTAAAGAAAC CGTATACGCC 100
<210> 9022
<211> 100
<212> DNA
<213> Escherichia coli K-12 MG1655
<220> rtn b2177
<222> 1391-1490
<223> rtn b2177 U00096 2268746..2270302
<313> 9022: 1439-1458
<400> 9022
AACGCCTCAA TATGCTGACG GTTGCTGAAG GGGTCGAAAC GCCGGAACAG 50
GCGCGATGGC TAAGCGAACG CGGCGTTAAT TTCATGCAAG GCTACTGGAT 100
<210> 9023
<211> 100
<212> DNA
<213> Escherichia coli K-12 MG1655
<220> uidr b1619
<222> 492-591
<223> uidr b1619 U00096 complement(1694486..1695076)
<313> 9023: 527-546
<400> 9023
CGGTGTACTG GCCGATATCG AAGCGGAAGA CCTGGCGCGT GAAGCGTCGT 50
TTGCTCAGGG ATTACGCGCG ATGATTGGCG GTATCTTAAC CGCATCCTGA 100
<210> 9024

<211> 100
<212> DNA
<213> Escherichia coli K-12 MG1655
<220> uidr b1619
<222> 432-531
<223> uidr b1619 U00096 complement(1694486..1695076)
<313> 9024: 469-488
<400> 9024
CGAGATAAGC CCAGACATCA ACACGGCAAT GACTTCACGT TTACTGCTGG 50
ATCTGACCTA CGGTGTACTG GCCGATATCG AAGCGGAAGA CCTGGCGCGT 100
<210> 9025
<211> 100
<212> DNA
<213> Escherichia coli K-12 MG1655
<220> uidr b1619
<222> 408-507
<223> uidr b1619 U00096 complement(1694486..1695076)
<313> 9025: 420-439
<400> 9025
GATGCGTGAT GCCCAGCAAA AAGGCGAGAT AAGCCCAGAC ATCAACACGG 50
CAATGACTTC ACGTTTACTG CTGGATCTGA CCTACGGTGT ACTGGCCGAT 100
<210> 9026
<211> 100
<212> DNA
<213> Escherichia coli K-12 MG1655
<220> kch b1251
<222> 1155-1254
<223> kch b1251 U00096 complement(1307040..1308293)
<313> 9026: 1217-1236
<400> 9026
TATGCTCGTT TCAATGTTGT TGAACTCAGG TCATGGTATT TTCAGCGATA 50
ACGATGAACT AGAAACGAAA GCTGACAGTA AAGAATCGGC GCAAAAATAG 100
<210> 9027
<211> 100
<212> DNA
<213> Escherichia coli K-12 MG1655
<220> kch b1251
<222> 1063-1162
<223> kch b1251 U00096 complement(1307040..1308293)
<313> 9027: 1119-1138
<400> 9027
AAGATGGTAC ATCCGGATAT CATTCTCTCA CCGCAACTGT TTGGCAGCGA 50
AATTCTGGCG CGAGTTTTAA ATGGTGAAGA GATTAATAAT GATATGCTCG 100
<210> 9028
<211> 100
<212> DNA
<213> Escherichia coli K-12 MG1655
<220> kch b1251
<222> 972-1071
<223> kch b1251 U00096 complement(1307040..1308293)
<313> 9028: 1021-1040
<400> 9028
TAACGCGTTT GTTGTACTCT CGGCAAAAGA TATGAGCAGT GATGTCAAAA 50
CAGTTCTCGC CGTCAGTGAT AGCAAAAACC TGAATAAGAT TAAGATGGTA 100
<210> 9029
<211> 100
<212> DNA
<213> Escherichia coli K-12 MG1655
<220> yeaa b1779
<222> 315-414
<223> yeaa b1779 U00096 complement(1860040..1860453)
<313> 9029: 374-393
<400> 9029
CTTCCCCGAC GGGCCGCAGC CAACGGGCGA ACGTTATTGT GTTAACTCTG 50
CCTCTTTACG CTTTACCGAT GGCGAAAACG GCGAAGAAAT CAACGGTTGA 100
<210> 9030
<211> 100
<212> DNA
<213> Escherichia coli K-12 MG1655
<220> yeaa b1779
<222> 315-414

<223> yeaa b1779 U00096 complement(1860040..1860453)
<313> 9030: 346-365
<400> 9030
CTTCCCCGAC GGGCCGCAGC CAACGGGCGA ACGTTATTGT GTTAACTCTG  50
CCTCTTTACG CTTTACCGAT GGCGAAAACG GCGAAGAAAT CAACGGTTGA  100
<210> 9031
<211> 100
<212> DNA
<213> Escherichia coli K-12 MG1655
<220> yeaa b1779
<222> 127-226
<223> yeaa b1779 U00096 complement(1860040..1860453)
<313> 9031: 174-193
<400> 9031
GTATATCACT GTTTGATCTG CGATGCCCCG CTGTTTCATT CCCAAACCAA  50
GTATGATTCC GGCTGTGGCT GGCCCAGTTT CTACGAACCG GTAAGTGAAG  100
<210> 9032
<211> 100
<212> DNA
<213> Escherichia coli K-12 MG1655
<220> yeab b1814
<222> 466-565
<223> yeab b1814 U00096 1894194..1894772
<313> 9032: 517-536
<400> 9032
CGCCGTGGTG ATTCACATCG GGTATGGCTG TCCTGGTACG AACAGTATTT  50
TGTATGGGGA ATGACCGCAG GCATAATTCG TGAGCTGGCG CTGCAAATTG  100
<210> 9033
<211> 100
<212> DNA
<213> Escherichia coli K-12 MG1655
<220> yeab b1814
<222> 438-537
<223> yeab b1814 U00096 1894194..1894772
<313> 9033: 489-508
<400> 9033
GGGTCGTTAT CACCCTTTAG ATATCTACCG CCGTGGTGAT TCACATCGGG  50
TATGGCTGTC CTGGTACGAA CAGTATTTTG TATGGGGAAT GACCGCAGGC  100
<210> 9034
<211> 100
<212> DNA
<213> Escherichia coli K-12 MG1655
<220> yeab b1814
<222> 334-433
<223> yeab b1814 U00096 1894194..1894772
<313> 9034: 387-406
<400> 9034
GTAACCCCAG TGGTCGGCAT TATCCCGCCC GATCTGCCGT ATCGCGCCAG  50
TGAAGATGAA GTCTCGGCGG TGTTTGAAAT GCCGCTCGCC CAGGCATTAC  100
<210> 9035
<211> 100
<212> DNA
<213> Escherichia coli K-12 MG1655
<220> yead yeaD
<222> 807-906
<223> yead yeaD U00096 1861853..1862758
<313> 9035: 886-905
<400> 9035
ATTTGTTTGT GTAGAAACGG CTTACGCTTC AGAAACGCAA AAAGTGACCA  50
AAGAGAAACC TGCACATCTG GCGCAATCCA TTCGCGTTGC GAAACGTTAA  100
<210> 9036
<211> 100
<212> DNA
<213> Escherichia coli K-12 MG1655
<220> yead yeaD
<222> 807-906
<223> yead yeaD U00096 1861853..1862758
<313> 9036: 828-847
<400> 9036
ATTTGTTTGT GTAGAAACGG CTTACGCTTC AGAAACGCAA AAAGTGACCA  50
AAGAGAAACC TGCACATCTG GCGCAATCCA TTCGCGTTGC GAAACGTTAA  100

<210> 9037
<211> 100
<212> DNA
<213> Escherichia coli K-12 MG1655
<220> yead yeaD
<222> 766-865
<223> yead yeaD U00096 1861853..1862758
<313> 9037: 793-812
<400> 9037
TCAATTAGCA TGGGCGATAT GCCGGATGAT GGCTACAAAA CATTTGTTTG 50
TGTAGAAACG GCTTACGCTT CAGAAACGCA AAAAGTGACC AAAGAGAAAC 100
<210> 9038
<211> 100
<212> DNA
<213> Escherichia coli K-12 MG1655
<220> yeaf b1783
<222> 648-747
<223> yeaf b1783 U00096 complement(1863750..1864496)
<313> 9038: 724-743
<400> 9038
CGCACGCTAC ACCCGTCTGT CTGATGAAGT TACTGACAGC CCGATGGTGG 50
ATAAATCCTG GACTGGCCTG ATTTCTACCG GGATCACCTA CAAATTCTGA 100
<210> 9039
<211> 100
<212> DNA
<213> Escherichia coli K-12 MG1655
<220> yeaf b1783
<222> 633-732
<223> yeaf b1783 U00096 complement(1863750..1864496)
<313> 9039: 683-702
<400> 9039
GAGTGTTTAC GGTACCGCAC GCTACACCCG TCTGTCTGAT GAAGTTACTG 50
ACAGCCCGAT GGTGGATAAA TCCTGGACTG GCCTGATTTC TACCGGGATC 100
<210> 9040
<211> 100
<212> DNA
<213> Escherichia coli K-12 MG1655
<220> yeaf b1783
<222> 572-671
<223> yeaf b1783 U00096 complement(1863750..1864496)
<313> 9040: 611-630
<400> 9040
CGAACGACAG CTGGAGCCCT TACCTGGAGC TGAGCGCCAG CTACAACTTC 50
CTCGGCGACT GGAGTGTTTA CGGTACCGCA CGCTACACCC GTCTGTCTGA 100
<210> 9041
<211> 100
<212> DNA
<213> Escherichia coli K-12 MG1655
<220> yeag b1784
<222> 1812-1911
<223> yeag b1784 U00096 1864932..1866866
<313> 9041: 1858-1877
<400> 9041
AACGTCAACC GACGAGCAGA AGAAACACGA CGACTTTGTC GACCGTATGA 50
TGGAAAAAGG CTACACCCGT AAACAGGTGC GTTTACTGTG CGAATGGTAT 100
<210> 9042
<211> 100
<212> DNA
<213> Escherichia coli K-12 MG1655
<220> yeag b1784
<222> 1780-1879
<223> yeag b1784 U00096 1864932..1866866
<313> 9042: 1823-1842
<400> 9042
GAGCTGTTGC CGGTTATCTC GTTTAACGCC AAAACGTCAA CCGACGAGCA 50
GAAGAAACAC GACGACTTTG TCGACCGTAT GATGGAAAAA GGCTACACCC 100
<210> 9043
<211> 100
<212> DNA
<213> Escherichia coli K-12 MG1655
<220> yeag b1784

<222> 1735-1834
<223> yeag b1784 U00096 1864932..1866866
<313> 9043: 1790-1809
<400> 9043
AAACTGCGCA CGGTCATCGA GAAGAAAATG TTCTCCAATA CCGAGGAGCT 50
GTTGCCGGTT ATCTCGTTTA ACGCCAAAAC GTCAACCGAC GAGCAGAAGA 100
<210> 9044
<211> 100
<212> DNA
<213> Escherichia coli K-12 MG1655
<220> yeah b1785
<222> 1171-1270
<223> yeah b1785 U00096 1866979..1868262
<313> 9044: 1230-1249
<400> 9044
CATCTGCAAT CTACTTTCGA CAACTTTGCG ATGCAGCACA TCCGCGACCA 50
GGATGATATT TATCCGGTGT TCCGTGAACT GTTTCATAAA CAAAATGCAA 100
<210> 9045
<211> 100
<212> DNA
<213> Escherichia coli K-12 MG1655
<220> yeah b1785
<222> 1141-1240
<223> yeah b1785 U00096 1866979..1868262
<313> 9045: 1184-1203
<400> 9045
GCACATCAGA CATTGTGGCG AGAATATGAG CATCTGCAAT CTACTTTCGA 50
CAACTTTGCG ATGCAGCACA TCCGCGACCA GGATGATATT TATCCGGTGT 100
<210> 9046
<211> 100
<212> DNA
<213> Escherichia coli K-12 MG1655
<220> yeah b1785
<222> 999-1098
<223> yeah b1785 U00096 1866979..1868262
<313> 9046: 1060-1079
<400> 9046
CCCGGCACAG TGGAATATTT ACGCTGCACA AGCATCGGAC GGCGATAACT 50
GGGCCGATGA CTCTCCGCTT TGCCATGAAA TCCTGGCGAA AAAATTATTA 100
<210> 9047
<211> 100
<212> DNA
<213> Escherichia coli K-12 MG1655
<220> yeai b1786
<222> 1377-1476
<223> yeai b1786 U00096 1868409..1869884
<313> 9047: 1425-1444
<400> 9047
TGACACGCGT AATATCTCGC TTGTCATGAC GGAAGCAGAT AAAGCCTTAC 50
GCGAAGCGAA AAGCGAGGGG GGCAACAAAG TGATTATTCA TCATATTTAA 100
<210> 9048
<211> 100
<212> DNA
<213> Escherichia coli K-12 MG1655
<220> yeai b1786
<222> 1244-1343
<223> yeai b1786 U00096 1868409..1869884
<313> 9048: 1292-1311
<400> 9048
AACTCAATAG TGCCCAGGCA AAAATCATTG CGGAACGTAT GCGTAAAAAT 50
GTCGAACTCC TGACCGGCTT TAGTAACAGA TATGATGTTC CTGAACAAAT 100
<210> 9049
<211> 100
<212> DNA
<213> Escherichia coli K-12 MG1655
<220> yeai b1786
<222> 1231-1330
<223> yeai b1786 U00096 1868409..1869884
<313> 9049: 1259-1278
<400> 9049
TTGCTATTTA CCGAACTCAA TAGTGCCCAG GCAAAAATCA TTGCGGAACG 50

TATGCGTAAA AATGTCGAAC TCCTGACCGG CTTTAGTAAC AGATATGATG 100
<210> 9050
<211> 100
<212> DNA
<213> Escherichia coli K-12 MG1655
<220> yeaj b1787
<222> 1523-1622
<223> yeaj b1787 U00096 1869885..1871555
<313> 9050: 1565-1584
<400> 9050
AACGTCTGCA ACATATCGCG CCGCAGAAAG AGATCGGCTT CTCTTCCGGT 50
ATTTACGCGA TGAAAGAAAA CGATACGTTA CATGATGCGT ATAAAGCTTC 100
<210> 9051
<211> 100
<212> DNA
<213> Escherichia coli K-12 MG1655
<220> yeaj b1787
<222> 1468-1567
<223> yeaj b1787 U00096 1869885..1871555
<313> 9051: 1533-1552
<400> 9051
ATTCTTGTCG ATTCGACGCC GCAAATTGCA GCACAACTGC CTGAACGTAT 50
CGAAAAACGT CTGCAACATA TCGCGCCGCA GAAAGAGATC GGCTTCTCTT 100
<210> 9052
<211> 100
<212> DNA
<213> Escherichia coli K-12 MG1655
<220> yeaj b1787
<222> 1454-1553
<223> yeaj b1787 U00096 1869885..1871555
<313> 9052: 1480-1499
<400> 9052
ATGAATTCTG CATCATTCTT GTCGATTCGA CGCCGCAAAT TGCAGCACAA 50
CTGCCTGAAC GTATCGAAAA ACGTCTGCAA CATATCGCGC CGCAGAAAGA 100
<210> 9053
<211> 100
<212> DNA
<213> Escherichia coli K-12 MG1655
<220> yeak yeaK
<222> 351-450
<223> yeak yeaK U00096 1871598..1872101
<313> 9053: 392-411
<400> 9053
AAAACTCAAA CTGGTTGCCG ACCCTCTACT CTTCGAACGA TTTGATGAAA 50
TCGCCTTCAA TGCAGGCATG CTGGATAAAT CCGTTATTTT GAAAACCGCG 100
<210> 9054
<211> 100
<212> DNA
<213> Escherichia coli K-12 MG1655
<220> yeak yeaK
<222> 280-379
<223> yeak yeaK U00096 1871598..1872101
<313> 9054: 328-347
<400> 9054
GCCAGCCCGG CGGAAGTCGA TGAACTCACA GGCTGTGTCT TCGGCGCGAT 50
CCCCCCTTTC AGCTTCCATC CAAAACTCAA ACTGGTTGCC GACCCTCTAC 100
<210> 9055
<211> 100
<212> DNA
<213> Escherichia coli K-12 MG1655
<220> yeak yeaK
<222> 246-345
<223> yeak yeaK U00096 1871598..1872101
<313> 9055: 293-312
<400> 9055
CGCCAGCCAT ATAGGCGGAT TACGCGCTTC GTTGGCCAGC CCGGCGGAAG 50
TCGATGAACT CACAGGCTGT GTCTTCGGCG CGATCCCCCC TTTCAGCTTC 100
<210> 9056
<211> 100
<212> DNA
<213> Escherichia coli K-12 MG1655

<220> b2520 b2521
<222> 4863-4962
<223> b2520 b2521 U00096 complement(2645346..2650307)
<313> 9056: 4937-4956
<400> 9056
GGGAACGTAT CAGGTACCGC AACCGATGGT GGAATCAATG TATGTTCCCC 50
AATGGCGGGC GACCGGCGCG GCTGAAGATC TGCTGATTGT CAGACCGTAA 100
<210> 9057
<211> 100
<212> DNA
<213> Escherichia coli K-12 MG1655
<220> b2520 b2521
<222> 4732-4831
<223> b2520 b2521 U00096 complement(2645346..2650307)
<313> 9057: 4754-4773
<400> 9057
AACTTACTGA ACCAGATGCA GCAGGCGAGC ATTAAGCACA TTGAGTTCCG 50
TGACGATCGC TTTGTGGCGG CGGTTGCCGT TGATGAATAC CAACCGGTAA 100
<210> 9058
<211> 100
<212> DNA
<213> Escherichia coli K-12 MG1655
<220> b2520 b2521
<222> 4694-4793
<223> b2520 b2521 U00096 complement(2645346..2650307)
<313> 9058: 4714-4733
<400> 9058
GTAGCGCCAG CCTGGAGCAA AGTGGTGGCG AAGTGCAGAA CTTACTGAAC 50
CAGATGCAGC AGGCGAGCAT TAAGCACATT GAGTTCCGTG ACGATCGCTT 100
<210> 9059
<211> 100
<212> DNA
<213> Escherichia coli K-12 MG1655
<220> yeal b1790
<222> 195-294
<223> yeal b1790 U00096 1872376..1872822
<313> 9059: 220-239
<400> 9059
CATCGCCAGC GGGACGCTAC CGCCCTCAAC CTTGATTCAC TCGTTTCTGA 50
ACTGGAAATC ACTGGTGGCG ATTGCAGTAG GCGTGATTGT CTCCTGGCTG 100
<210> 9060
<211> 100
<212> DNA
<213> Escherichia coli K-12 MG1655
<220> yeal b1790
<222> 139-238
<223> yeal b1790 U00096 1872376..1872822
<313> 9060: 170-189
<400> 9060
GAAAAACAGG GGCTAAGTAT CGGGATAATC ATTCTGACGA TTGGCGTTAT 50
GGCCCCCATC GCCAGCGGGA CGCTACCGCC CTCAACCTTG ATTCACTCGT 100
<210> 9061
<211> 100
<212> DNA
<213> Escherichia coli K-12 MG1655
<220> yeal b1790
<222> 68-167
<223> yeal b1790 U00096 1872376..1872822
<313> 9061: 102-121
<400> 9061
CTGTCGCCGT TTCAATTCTG GTGTTAATCA TTGTCCGCGT CACACCGTTA 50
AGCACCTTTT TTCCGTGGAT TGAAAAACAG GGGCTAAGTA TCGGGATAAT 100
<210> 9062
<211> 100
<212> DNA
<213> Escherichia coli K-12 MG1655
<220> yeam b1791
<222> 723-822
<223> yeam b1791 U00096 complement(1872779..1873600)
<313> 9062: 776-795
<400> 9062

TACTATGTTT AAGAAAGGCC TGGGGCAAAC GCCAGGCCGA TATATTGCGA 50
GATTAACTAC TGTTTCCCCA CAATCAGCGA AACCAGACCC GCGGCAATAA 100
<210> 9063
<211> 100
<212> DNA
<213> Escherichia coli K-12 MG1655
<220> yeam b1791
<222> 654-753
<223> yeam b1791 U00096 complement(1872779..1873600)
<313> 9063: 696-715
<400> 9063
ATTAGTGAAA GGCGATACTG TACAGAAAGT TGCTCATACG CTGGGGTATG 50
ATTCAACAAC GGCCTTTATT ACTATGTTTA AGAAAGGCCT GGGGCAAACG 100
<210> 9064
<211> 100
<212> DNA
<213> Escherichia coli K-12 MG1655
<220> yeam b1791
<222> 596-695
<223> yeam b1791 U00096 complement(1872779..1873600)
<313> 9064: 640-659
<400> 9064
CGGGGTTGAG TTTTCGCCAG TGGCGTCAGC AGTTACAGCT GATTATGGCG 50
CTTCAGGGAT TAGTGAAAGG CGATACTGTA CAGAAAGTTG CTCATACGCT 100
<210> 9065
<211> 100
<212> DNA
<213> Escherichia coli K-12 MG1655
<220> yean b1792
<222> 1083-1182
<223> yean b1792 U00096 1873697..1874878
<313> 9065: 1146-1165
<400> 9065
TAACGGTAAC TGGTCTGTAC CACTTATGGG TGTTGCCATA CTTTCACTAC 50
TGATGGCGAT TTTCGGACTT TGCGCCGGGA GAGACAAAGA AATTCGCTAA 100
<210> 9066
<211> 100
<212> DNA
<213> Escherichia coli K-12 MG1655
<220> yean b1792
<222> 1012-1111
<223> yean b1792 U00096 1873697..1874878
<313> 9066: 1061-1080
<400> 9066
GGGATGGCAC AATCCGTCGG GTATTTGTTG GCAGCCTGTG GGCCGCCGCT 50
GATGGGTAAA ATACACGATG CTAACGGTAA CTGGTCTGTA CCACTTATGG 100
<210> 9067
<211> 100
<212> DNA
<213> Escherichia coli K-12 MG1655
<220> yean b1792
<222> 894-993
<223> yean b1792 U00096 1873697..1874878
<313> 9067: 946-965
<400> 9067
AGCGCACGCG ATCACCTGGA CTCTGCTTTT CGGTTTTGGT TCCGGCGCAA 50
CAATGATACT GGGGTTGACG TTCATTGGTC TGCGGGCTAG TTCTGCGCAT 100
<210> 9068
<211> 100
<212> DNA
<213> Escherichia coli K-12 MG1655
<220> yeao b1793
<222> 270-369
<223> yeao b1793 U00096 1874912..1875280
<313> 9068: 316-335
<400> 9068
GGACATCGCC AAAAAACAGC CGCTGACCCT GCTCTACTCA GCAAAAAACA 50
CCACGCAGAA CCATGCGCTG GTGCTGGCCG ACTGGCTACG TAGCTTGTGA 100
<210> 9069
<211> 100
<212> DNA

<213> Escherichia coli K-12 MG1655
<220> yeao b1793
<222> 223-322
<223> yeao b1793 U00096 1874912..1875280
<313> 9069: 252-271
<400> 9069
TATCTTGCAG AACTGGCGCA ACACGAGCAA GAAGGAAAGC GGCTGGCGGA 50
CATCGCCAAA AAACAGCCGC TGACCCTGCT CTACTCAGCA AAAAACACCA 100
<210> 9070
<211> 100
<212> DNA
<213> Escherichia coli K-12 MG1655
<220> yeao b1793
<222> 96-195
<223> yeao b1793 U00096 1874912..1875280
<313> 9070: 143-162
<400> 9070
CTGGCCGCGC GGTATCAAAA AAACCGATTT AGCCCTTGAT GAGTGGGATA 50
AAGAAATCAC GCCGTCAACG GAACTGCGCA AAGCCTTTCA CGGCGAAGTC 100
<210> 9071
<211> 100
<212> DNA
<213> Escherichia coli K-12 MG1655
<220> yeap b1795
<222> 1056-1155
<223> yeap b1795 U00096 1875610..1876764
<313> 9071: 1114-1133
<400> 9071
TGCAGACAGT GCCCTGCATG CTGCCGATAT TGCGATGTAT CAGGAGAAAA 50
AACACAAACA GAAAACACCT TTTGTCGCGC ATCCAGCGCT ACATTCCTGA 100
<210> 9072
<211> 100
<212> DNA
<213> Escherichia coli K-12 MG1655
<220> yeap b1795
<222> 986-1085
<223> yeap b1795 U00096 1875610..1876764
<313> 9072: 1033-1052
<400> 9072
TAGGTGATGT TGATTTGTAT TATCCCGGTG CCAGTCTTGG CATAGTAGAA 50
GTCGATCCTG AAACAACCGA TGCAGACAGT GCCCTGCATG CTGCCGATAT 100
<210> 9073
<211> 100
<212> DNA
<213> Escherichia coli K-12 MG1655
<220> yeap b1795
<222> 912-1011
<223> yeap b1795 U00096 1875610..1876764
<313> 9073: 956-975
<400> 9073
ACTAAACAAC GAGAATGCGG ATATTTCGTC GCTGCGAGAA CGCATTCAGC 50
AGCAAATACG TGGAGAATAT CACTTAGGTG ATGTTGATTT GTATTATCCC 100
<210> 9074
<211> 100
<212> DNA
<213> Escherichia coli K-12 MG1655
<220> b3254 b3255
<222> 3-102
<223> b3254 b3255 U00096 complement(3402153..3402254)
<313> 9074: 66-85
<400> 9074
GGTCAGTCGA AATCATGACT TTCCAGTTTG GATGATGTTG TATAACTTCC 50
TGACCAAGGT GTTCGACGAA AGCTGTCCGG CTAACCGCTT TTCCCGTTAA 100
<210> 9075
<211> 100
<212> DNA
<213> Escherichia coli K-12 MG1655
<220> b3254 b3255
<222> 3-102
<223> b3254 b3255 U00096 complement(3402153..3402254)
<313> 9075: 54-73

<400> 9075
GGTCAGTCGA AATCATGACT TTCCAGTTTG GATGATGTTG TATAACTTCC 50
TGACCAAGGT GTTCGACGAA AGCTGTCCGG CTAACCGCTT TTCCCGTTAA 100
<210> 9076
<211> 100
<212> DNA
<213> Escherichia coli K-12 MG1655
<220> b3254 b3255
<222> 3-102
<223> b3254 b3255 U00096 complement(3402153..3402254)
<313> 9076: 55-74
<400> 9076
GGTCAGTCGA AATCATGACT TTCCAGTTTG GATGATGTTG TATAACTTCC 50
TGACCAAGGT GTTCGACGAA AGCTGTCCGG CTAACCGCTT TTCCCGTTAA 100
<210> 9077
<211> 100
<212> DNA
<213> Escherichia coli K-12 MG1655
<220> yeaq b1796
<222> 130-229
<223> yeaq b1796 U00096 complement(1877031..1877279)
<313> 9077: 157-176
<400> 9077
GGATGGATCA GCACGCTGTT TGGCTTTGGT AAAGTCGATG GCTTCAATTT 50
TGGCAGCTTC GTGGTTGCCG TTATTGGTGC GATTGTCGTG CTATTTATCT 100
<210> 9078
<211> 100
<212> DNA
<213> Escherichia coli K-12 MG1655
<220> yeaq b1796
<222> 84-183
<223> yeaq b1796 U00096 complement(1877031..1877279)
<313> 9078: 128-147
<400> 9078
ATTCTTTATG ACTATCCTGC TGGGGATAGT CGGTGCCGTA GTCGGCGGAT 50
GGATCAGCAC GCTGTTTGGC TTTGGTAAAG TCGATGGCTT CAATTTTGGC 100
<210> 9079
<211> 100
<212> DNA
<213> Escherichia coli K-12 MG1655
<220> yeaq b1796
<222> 33-132
<223> yeaq b1796 U00096 complement(1877031..1877279)
<313> 9079: 59-78
<400> 9079
TATTGCCGGT ATTCTGGCGA AGTGGATCAT GCCAGGTAAA GATGGAGGTG 50
GATTCTTTAT GACTATCCTG CTGGGGATAG TCGGTGCCGT AGTCGGCGGA 100
<210> 9080
<211> 100
<212> DNA
<213> Escherichia coli K-12 MG1655
<220> year b1798
<222> 213-312
<223> year b1798 U00096 complement(1877613..1877972)
<313> 9080: 241-260
<400> 9080
GGGGCAGTTT GCGGTGTTCC CTCCAGAAAA GTGGCACAAC ATTGAAGCCA 50
TGACTGACGA TACTTATTTC AACATTGACT TCTTCGTGGC TCCTGAAGTC 100
<210> 9081
<211> 100
<212> DNA
<213> Escherichia coli K-12 MG1655
<220> year b1798
<222> 181-280
<223> year b1798 U00096 complement(1877613..1877972)
<313> 9081: 204-223
<400> 9081
GCAGAGCCTG ATCAGGTGAT CCTTATCGAA GCGGGGCAGT TTGCGGTGTT 50
CCCTCCAGAA AAGTGGCACA ACATTGAAGC CATGACTGAC GATACTTATT 100
<210> 9082
<211> 100

<212> DNA
<213> Escherichia coli K-12 MG1655
<220> year b1798
<222> 118-217
<223> year b1798 U00096 complement(1877613..1877972)
<313> 9082: 168-187
<400> 9082
TACCCACGCC TTTCCGTTAT GCATGGGGCG GTCAAATATC TCGGCTACGC 50
TGATGAACAC AGTGCAGAGC CTGATCAGGT GATCCTTATC GAAGCGGGGC 100
<210> 9083
<211> 100
<212> DNA
<213> Escherichia coli K-12 MG1655
<220> yeas b1799
<222> 540-639
<223> yeas b1799 U00096 complement(1878145..1878783)
<313> 9083: 619-638
<400> 9083
GTACATACGT ACCAAAAAGA AACTGGCTAA AGTTGGCAAC TCACTGATTG 50
GTTTGATGTT CGTGGGTTTC GCTGCCCGAC TGGCGACGCT GCAATCCTGA 100
<210> 9084
<211> 100
<212> DNA
<213> Escherichia coli K-12 MG1655
<220> yeas b1799
<222> 492-591
<223> yeas b1799 U00096 complement(1878145..1878783)
<313> 9084: 522-541
<400> 9084
TTTCTGCTAT TTGAGCTTCC TGATTATATC TGGTGCTTTT GTCACGCAGT 50
ACATACGTAC CAAAAAGAAA CTGGCTAAAG TTGGCAACTC ACTGATTGGT 100
<210> 9085
<211> 100
<212> DNA
<213> Escherichia coli K-12 MG1655
<220> yeas b1799
<222> 459-558
<223> yeas b1799 U00096 complement(1878145..1878783)
<313> 9085: 493-512
<400> 9085
CTTTATTCTG GCGGCGACGC TGGAACTGGT GAGTTTCTGC TATTTGAGCT 50
TCCTGATTAT ATCTGGTGCT TTTGTCACGC AGTACATACG TACCAAAAAG 100
<210> 9086
<211> 100
<212> DNA
<213> Escherichia coli K-12 MG1655
<220> yeat b1800
<222> 846-945
<223> yeat b1800 U00096 complement(1878910..1879854)
<313> 9086: 909-928
<400> 9086
TTCAAGGCTG GCGACGTCAG CGAAAGTGCG GATAACGGTA GAATTTTTAC 50
GCCAGTATTT TGCCGAGCAC TACCCGAATT TTTCACTGGA GCATGCCTGA 100
<210> 9087
<211> 100
<212> DNA
<213> Escherichia coli K-12 MG1655
<220> yeat b1800
<222> 820-919
<223> yeat b1800 U00096 complement(1878910..1879854)
<313> 9087: 867-886
<400> 9087
CCAGCGAACG TCTGGGCCGT TTATGTTTCA AGGCTGGCGA CGTCAGCGAA 50
AGTGCGGATA ACGGTAGAAT TTTTACGCCA GTATTTTGCC GAGCACTACC 100
<210> 9088
<211> 100
<212> DNA
<213> Escherichia coli K-12 MG1655
<220> yeat b1800
<222> 745-844
<223> yeat b1800 U00096 complement(1878910..1879854)

<313> 9088: 797-816
<400> 9088
CTGCGTTCCT GGTGGGATGT TAGCGAAAAC ATTGCCAGTG GTCATTTAGT 50
GCAAGTGCTA CCGGAATATT ACCAGCCAGC GAACGTCTGG GCCGTTTATG 100
<210> 9089
<211> 100
<212> DNA
<213> Escherichia coli K-12 MG1655
<220> b2529 b2530
<222> 288-387
<223> b2529 b2530 U00096 complement(2657923..2658309)
<313> 9089: 351-370
<400> 9089
ACTTGAACTG CCGCCGGTGA AAATTCACTG TTCTATTCTG GCAGAAGACG 50
CGATCAAAGC CGCCATTGCG GACTATAAAA GCAAACGTGA AGCAAAATAA 100
<210> 9090
<211> 100
<212> DNA
<213> Escherichia coli K-12 MG1655
<220> b2529 b2530
<222> 197-296
<223> b2529 b2530 U00096 complement(2657923..2658309)
<313> 9090: 250-269
<400> 9090
CTATCGCTTC CAGCTCCCTG GTCACCGAAT GGGTGAAAGG GAAGTCTCTC 50
GACGAAGCGC AGGCGATCAA AAACACCGAT ATTGCTGAAG AACTTGAACT 100
<210> 9091
<211> 100
<212> DNA
<213> Escherichia coli K-12 MG1655
<220> b2529 b2530
<222> 165-264
<223> b2529 b2530 U00096 complement(2657923..2658309)
<313> 9091: 198-217
<400> 9091
CGCGCGTTTT AAAACTTACG GCTGCGGTTC CGCTATCGCT TCCAGCTCCC 50
TGGTCACCGA ATGGGTGAAA GGGAAGTCTC TCGACGAAGC GCAGGCGATC 100
<210> 9092
<211> 100
<212> DNA
<213> Escherichia coli K-12 MG1655
<220> yeau b1801
<222> 981-1080
<223> yeau b1801 U00096 1879936..1881021
<313> 9092: 1037-1056
<400> 9092
AGCAATTGAA GAAGTGATTG CTCACGGGCC GAAAACACCT GATATGAAAG 50
GCAATGCCAC CACGCCACAG GTTGCCGACG CGATTTGCAA AATTATTTTG 100
<210> 9093
<211> 100
<212> DNA
<213> Escherichia coli K-12 MG1655
<220> yeau b1801
<222> 915-1014
<223> yeau b1801 U00096 1879936..1881021
<313> 9093: 968-987
<400> 9093
AATGATGCTC GATTTTCTCG GCAATGGCGA TGAGCGTTTC CAGCAAGCGC 50
ATAACGGTAT TCTGGCAGCA ATTGAAGAAG TGATTGCTCA CGGGCCGAAA 100
<210> 9094
<211> 100
<212> DNA
<213> Escherichia coli K-12 MG1655
<220> yeau b1801
<222> 888-987
<223> yeau b1801 U00096 1879936..1881021
<313> 9094: 925-944
<400> 9094
CCCTATCGCC ACGATTTGGG CCGGGGCAAT GATGCTCGAT TTTCTCGGCA 50
ATGGCGATGA GCGTTTCCAG CAAGCGCATA ACGGTATTCT GGCAGCAATT 100
<210> 9095

<211> 100
<212> DNA
<213> Escherichia coli K-12 MG1655
<220> yeav b1802
<222> 1325-1424
<223> yeav b1802 U00096 1881212..1882657
<313> 9095: 1348-1367
<400> 9095
AACAGGATTA CGCCGACATT CCGGCTCATC AAGTTGAACA TTATCTCCCG 50
CAGACACCGG TTGAAGCCCT GGAAAAAACG CCAGTGCTCC CTGCGGGAAC 100
<210> 9096
<211> 100
<212> DNA
<213> Escherichia coli K-12 MG1655
<220> yeav b1802
<222> 1287-1386
<223> yeav b1802 U00096 1881212..1882657
<313> 9096: 1314-1333
<400> 9096
TTTACTGGTG AAAGTCGGCG GGTTTATTCG CTGGCTGAAA CAGGATTACG 50
CCGACATTCC GGCTCATCAA GTTGAACATT ATCTCCCGCA GACACCGGTT 100
<210> 9097
<211> 100
<212> DNA
<213> Escherichia coli K-12 MG1655
<220> yeav b1802
<222> 1218-1317
<223> yeav b1802 U00096 1881212..1882657
<313> 9097: 1269-1288
<400> 9097
GTTTACCGGT GCTTCGCTGG AAACGATGAA AACCACCGTC GTGCTCACAG 50
CCCTTCCCTT CCTCGTCATT TTACTGGTGA AAGTCGGCGG GTTTATTCGC 100
<210> 9098
<211> 100
<212> DNA
<213> Escherichia coli K-12 MG1655
<220> yeaw b1803
<222> 1026-1125
<223> yeaw b1803 U00096 1882689..1883813
<313> 9098: 1084-1103
<400> 9098
TGGTCAGGGG CGCATCATGG CCGACAGTAG CGGTAGTGGC ATTTCCGAAC 50
ATGGTATCGC CCATTTCCAT AATCTGCTGG CGCAGGTGTT TAAGGACTAA 100
<210> 9099
<211> 100
<212> DNA
<213> Escherichia coli K-12 MG1655
<220> yeaw b1803
<222> 1013-1112
<223> yeaw b1803 U00096 1882689..1883813
<313> 9099: 1035-1054
<400> 9099
CGCGTGGCTA TCGTGGTCAG GGGCGCATCA TGGCCGACAG TAGCGGTAGT 50
GGCATTTCCG AACATGGTAT CGCCCATTTC CATAATCTGC TGGCGCAGGT 100
<210> 9100
<211> 100
<212> DNA
<213> Escherichia coli K-12 MG1655
<220> yeaw b1803
<222> 956-1055
<223> yeaw b1803 U00096 1882689..1883813
<313> 9100: 982-1001
<400> 9100
ATGTGTTCCG TCCGGAAGAT TTACGTCTGG TTGAAAGCGT ACAGAAAGGG 50
CTGAAATCGC GTGGCTATCG TGGTCAGGGG CGCATCATGG CCGACAGTAG 100
<210> 9101
<211> 100
<212> DNA
<213> Escherichia coli K-12 MG1655
<220> yeax b1804
<222> 867-966

<223> yeax b1804 U00096 1883869..1884834
<313> 9101: 907-926
<400> 9101
TCGGGATCAA TATTTTAGCG ATGAAGAGCG TGCCAGCCAG CAAAGTATGT 50
TGATCTGTTG TTCGCGTGCG AAGGGTAAAC GCCTGGTGTT GGATTTGTAG 100
<210> 9102
<211> 100
<212> DNA
<213> Escherichia coli K-12 MG1655
<220> yeax b1804
<222> 812-911
<223> yeax b1804 U00096 1883869..1884834
<313> 9102: 852-871
<400> 9102
GTGAAGGGGT ATGCGGAACC TGCGAAACAG CAATACTGGA AGGTGAAGCT 50
GACCATCGGG ATCAATATTT TAGCGATGAA GAGCGTGCCA GCCAGCAAAG 100
<210> 9103
<211> 100
<212> DNA
<213> Escherichia coli K-12 MG1655
<220> yeax b1804
<222> 785-884
<223> yeax b1804 U00096 1883869..1884834
<313> 9103: 826-845
<400> 9103
AAGCCGCGAA AGTGGAATGT TTATGTCGTG AAGGGGTATG CGGAACCTGC 50
GAAACAGCAA TACTGGAAGG TGAAGCTGAC CATCGGGATC AATATTTTAG 100
<210> 9104
<211> 100
<212> DNA
<213> Escherichia coli K-12 MG1655
<220> yeaz yeaZ
<222> 597-696
<223> yeaz yeaZ U00096 complement(1888596..1889291)
<313> 9104: 674-693
<400> 9104
TCAGATGTTT GCTGAGGGTA AAACGGTGGC GGTGGAACAT GCCGAACCGG 50
TTTATTTACG TAACAACGTC GCATGGAAGA AACTTCCGGG CAAAGAATGA 100
<210> 9105
<211> 100
<212> DNA
<213> Escherichia coli K-12 MG1655
<220> yeaz yeaZ
<222> 594-693
<223> yeaz yeaZ U00096 complement(1888596..1889291)
<313> 9105: 625-644
<400> 9105
GTGTCAGATG TTTGCTGAGG GTAAAACGGT GGCGGTGGAA CATGCCGAAC 50
CGGTTTATTT ACGTAACAAC GTCGCATGGA AGAAACTTCC GGGCAAAGAA 100
<210> 9106
<211> 100
<212> DNA
<213> Escherichia coli K-12 MG1655
<220> yeaz yeaZ
<222> 516-615
<223> yeaz yeaZ U00096 complement(1888596..1889291)
<313> 9106: 557-576
<400> 9106
TCTCGGTAAA GAGAGCGGGC TGGTTTTGCG CGATGGCGAA GTGTTACTGC 50
CTGCTGCTGA AGATATGCTG CCGATTGCGT GTCAGATGTT TGCTGAGGGT 100
<210> 9107
<211> 100
<212> DNA
<213> Escherichia coli K-12 MG1655
<220> rus b0551
<222> 264-363
<223> rus b0551 U00096 572594..572956
<313> 9107: 335-354
<400> 9107
GCTGGATGAT GCTCAGGTCG TTGATTACCG CGTTGTGAAG ATGCCTGTTA 50
CCAAAGGTGG GAGGCTGGAA CTGACCATCA CCGAAATGGG GAATGAATGA 100

<210> 9108
<211> 100
<212> DNA
<213> Escherichia coli K-12 MG1655
<220> rus b0551
<222> 242-341
<223> rus b0551 U00096 572594..572956
<313> 9108: 290-309
<400> 9108
CACTCACTAA AGCAGGTTTC TGGCTGGATG ATGCTCAGGT CGTTGATTAC  50
CGCGTTGTGA AGATGCCTGT TACCAAAGGT GGGAGGCTGG AACTGACCAT  100
<210> 9109
<211> 100
<212> DNA
<213> Escherichia coli K-12 MG1655
<220> rus b0551
<222> 198-297
<223> rus b0551 U00096 572594..572956
<313> 9109: 243-262
<400> 9109
CCGTCGCCGT GACCTGGATA ATCTGCAAAA AGCCGCTTTT GACGCACTCA  50
CTAAAGCAGG TTTCTGGCTG GATGATGCTC AGGTCGTTGA TTACCGCGTT  100
<210> 9110
<211> 100
<212> DNA
<213> Escherichia coli K-12 MG1655
<220> hima b1713
<222> 201-300
<223> hima b1713 U00096 complement(1793277..1793576)
<313> 9110: 272-291
<400> 9110
GGGCGAGGAT ATTCCCATTA CAGCACGGCG CGTGGTGACC TTCAGACCCG  50
GGCAGAAGTT AAAAAGCCGG GTCGAAAACG CTTCGCCCAA AGACGAGTAA  100
<210> 9111
<211> 100
<212> DNA
<213> Escherichia coli K-12 MG1655
<220> hima b1713
<222> 193-292
<223> hima b1713 U00096 complement(1793277..1793576)
<313> 9111: 214-233
<400> 9111
CCGAAAACGG GCGAGGATAT TCCCATTACA GCACGGCGCG TGGTGACCTT  50
CAGACCCGGG CAGAAGTTAA AAAGCCGGGT CGAAAACGCT TCGCCCAAAG  100
<210> 9112
<211> 100
<212> DNA
<213> Escherichia coli K-12 MG1655
<220> hima b1713
<222> 106-205
<223> hima b1713 U00096 complement(1793277..1793576)
<313> 9112: 154-173
<400> 9112
CGCGCTCTGG AAAACGGCGA ACAGGTGAAA CTCTCTGGTT TTGGTAACTT  50
CGATCTGCGT GATAAGAATC AACGCCCGGG ACGTAACCCG AAAACGGGCG  100
<210> 9113
<211> 100
<212> DNA
<213> Escherichia coli K-12 MG1655
<220> yeba b1857
<222> 1161-1260
<223> yeba b1857 U00096 complement(1938337..1939596)
<313> 9113: 1226-1245
<400> 9113
GACGGCAAAA CTGCCGCGTA CCGAAGGGCT GACCGGCTCC GATCGTCGCG  50
AATTCCTGGC GCAGGCCAAA GAGATTGTGC CGCAGCTACG GTTTGATTAA  100
<210> 9114
<211> 100
<212> DNA
<213> Escherichia coli K-12 MG1655
<220> yeba b1857

<222> 1137-1236
<223> yeba b1857 U00096 complement(1938337..1939596)
<313> 9114: 1167-1186
<400> 9114
AAACCAGCAG GCCGTAAACC CGCTGACGGC AAAACTGCCG CGTACCGAAG 50
GGCTGACCGG CTCCGATCGT CGCGAATTCC TGGCGCAGGC CAAAGAGATT 100
<210> 9115
<211> 100
<212> DNA
<213> Escherichia coli K-12 MG1655
<220> yeba b1857
<222> 1081-1180
<223> yeba b1857 U00096 complement(1938337..1939596)
<313> 9115: 1131-1150
<400> 9115
CTTTCCGGTA ATACCGGACG TTCAACCGGG CCGCATCTGC ACTATGAAGT 50
ATGGATAAAC CAGCAGGCCG TAAACCCGCT GACGGCAAAA CTGCCGCGTA 100
<210> 9116
<211> 100
<212> DNA
<213> Escherichia coli K-12 MG1655
<220> himd b0913
<222> 186-285
<223> himd b0913 U00096 963051..963335
<313> 9116: 237-256
<400> 9116
TAATCCGAAG ACTGGCGATA AAGTAGAACT GGAAGGAAAA TACGTTCCTC 50
ACTTTAAACC TGGTAAAGAA CTGCGCGATC GCGCCAATAT TTACGGTTAA 100
<210> 9117
<211> 100
<212> DNA
<213> Escherichia coli K-12 MG1655
<220> himd b0913
<222> 146-245
<223> himd b0913 U00096 963051..963335
<313> 9117: 200-219
<400> 9117
GCAGTTTCTC TTTGCACTAC CGCGCACCAC GTACCGGACG TAATCCGAAG 50
ACTGGCGATA AAGTAGAACT GGAAGGAAAA TACGTTCCTC ACTTTAAACC 100
<210> 9118
<211> 100
<212> DNA
<213> Escherichia coli K-12 MG1655
<220> himd b0913
<222> 120-219
<223> himd b0913 U00096 963051..963335
<313> 9118: 145-164
<400> 9118
CGAGCGTATT GAAATCCGCG GTTTCGGCAG TTTCTCTTTG CACTACCGCG 50
CACCACGTAC CGGACGTAAT CCGAAGACTG GCGATAAAGT AGAACTGGAA 100
<210> 9119
<211> 100
<212> DNA
<213> Escherichia coli K-12 MG1655
<220> yebb b1863
<222> 603-702
<223> yebb b1863 U00096 1944176..1944877
<313> 9119: 658-677
<400> 9119
AACCGTCACG CCAGCCAGTT TGTGGCATCA TCCGGGTTTG GTGTTGATTC 50
ATGCGGAGGG AGTGGAAACG CCTCAGCCGG AACTGACCGA GGCGGTATAA 100
<210> 9120
<211> 100
<212> DNA
<213> Escherichia coli K-12 MG1655
<220> yebb b1863
<222> 563-662
<223> yebb b1863 U00096 1944176..1944877
<313> 9120: 610-629
<400> 9120
GGAAATTCTG GTTCCTGGGT TCTATTCCGT GGGAGCGTAA AACCGTCACG 50

CCAGCCAGTT TGTGGCATCA TCCGGGTTTG GTGTTGATTC ATGCGGAGGG 100
<210> 9121
<211> 100
<212> DNA
<213> Escherichia coli K-12 MG1655
<220> yebb b1863
<222> 407-506
<223> yebb b1863 U00096 1944176..1944877
<313> 9121: 422-441
<400> 9121
AACTTTACCA CACCGGTTTT AAATACGAAT CTTCGCGCCA GTTCTGTTCA 50
AAATTTGTTT TTGATATTTA TAAAGAAGCG CTATGTATTC CGGTGGGTGA 100
<210> 9122
<211> 100
<212> DNA
<213> Escherichia coli K-12 MG1655
<220> yebc b1865
<222> 642-741
<223> yebc b1865 U00096 complement(1945435..1946175)
<313> 9122: 704-723
<400> 9122
GAAACTGATG CGTCTGATCG ATATGCTGGA AGATTGCGAC GACGTGCAGG 50
AAGTTTACCA TAACGGTGAA ATCTCTGATG AGGTCGCAGC GACTCTCTGA 100
<210> 9123
<211> 100
<212> DNA
<213> Escherichia coli K-12 MG1655
<220> yebc b1865
<222> 624-723
<223> yebc b1865 U00096 complement(1945435..1946175)
<313> 9123: 669-688
<400> 9123
GGATGCAGAA ACCGCACCGA AACTGATGCG TCTGATCGAT ATGCTGGAAG 50
ATTGCGACGA CGTGCAGGAA GTTTACCATA ACGGTGAAAT CTCTGATGAG 100
<210> 9124
<211> 100
<212> DNA
<213> Escherichia coli K-12 MG1655
<220> yebc b1865
<222> 571-670
<223> yebc b1865 U00096 complement(1945435..1946175)
<313> 9124: 613-632
<400> 9124
CTGAAAGCAG ACAGCGCGGA AGTTTCCATG ATCCCGTCTA CCAAAGCTGA 50
TATGGATGCA GAAACCGCAC CGAAACTGAT GCGTCTGATC GATATGCTGG 100
<210> 9125
<211> 100
<212> DNA
<213> Escherichia coli K-12 MG1655
<220> yebe b1847
<222> 561-660
<223> yebe b1847 U00096 complement(1927072..1927731)
<313> 9125: 632-651
<400> 9125
TTACCTGAAT GCACTGGGTG ACGCGCTGAA AATCCCCCAG GATGTTCGTG 50
ACGGCATTGA ACGCGATCTC GAACAGCAAA AACGCACTTT GGCGGAATAA 100
<210> 9126
<211> 100
<212> DNA
<213> Escherichia coli K-12 MG1655
<220> yebe b1847
<222> 535-634
<223> yebe b1847 U00096 complement(1927072..1927731)
<313> 9126: 599-618
<400> 9126
GATATTGACC ATTTTATGGA ACGCAGTTAC CTGAATGCAC TGGGTGACGC 50
GCTGAAAATC CCCCAGGATG TTCGTGACGG CATTGAACGC GATCTCGAAC 100
<210> 9127
<211> 100
<212> DNA
<213> Escherichia coli K-12 MG1655

<220> yebe b1847
<222> 460-559
<223> yebe b1847 U00096 complement(1927072..1927731)
<313> 9127: 501-520
<400> 9127
CCACAACGCC TGGCTACCGG GGTCCGCAAT GAAGAAGAGG CGCTGGAAAT  50
CTATTTCCTG AGTTGCGCGG CTATTGATAT TGACCATTTT ATGGAACGCA  100
<210> 9128
<211> 100
<212> DNA
<213> Escherichia coli K-12 MG1655
<220> yebf b1848
<222> 270-369
<223> yebf b1848 U00096 complement(1928058..1928426)
<313> 9128: 331-350
<400> 9128
ATGGTCAGTA CCGCTAACCG TGCGTGGTAA AAGTGCCGAT ATTCATTACC  50
AGGTCAGCGT GGACTGCAAA GCGGGAATGG CGGAATATCA GCGGCGTTAA  100
<210> 9129
<211> 100
<212> DNA
<213> Escherichia coli K-12 MG1655
<220> yebf b1848
<222> 270-369
<223> yebf b1848 U00096 complement(1928058..1928426)
<313> 9129: 293-312
<400> 9129
ATGGTCAGTA CCGCTAACCG TGCGTGGTAA AAGTGCCGAT ATTCATTACC  50
AGGTCAGCGT GGACTGCAAA GCGGGAATGG CGGAATATCA GCGGCGTTAA  100
<210> 9130
<211> 100
<212> DNA
<213> Escherichia coli K-12 MG1655
<220> yebf b1848
<222> 218-317
<223> yebf b1848 U00096 complement(1928058..1928426)
<313> 9130: 241-260
<400> 9130
CCGATCCCGT GGCTTGGGTC AGTTTGCAGG ACATTCAGGG TAAAGATGAT  50
AAATGGTCAG TACCGCTAAC CGTGCGTGGT AAAAGTGCCG ATATTCATTA  100
<210> 9131
<211> 100
<212> DNA
<213> Escherichia coli K-12 MG1655
<220> yebg b1849
<222> 192-291
<223> yebg b1849 U00096 complement(1928481..1928771)
<313> 9131: 272-291
<400> 9131
AAAAGATGTG CTGAGCACCA TTCTGAAAAC CGGCAAATTA CCGTCCCCAC  50
AGGTCGTCGG TGCTGAATCC GAAGAGGAAG ATGCCTCACA CGCGGCCTGA  100
<210> 9132
<211> 100
<212> DNA
<213> Escherichia coli K-12 MG1655
<220> yebg b1849
<222> 182-281
<223> yebg b1849 U00096 complement(1928481..1928771)
<313> 9132: 226-245
<400> 9132
TGGCAGAACA AAAAGATGTG CTGAGCACCA TTCTGAAAAC CGGCAAATTA  50
CCGTCCCCAC AGGTCGTCGG TGCTGAATCC GAAGAGGAAG ATGCCTCACA  100
<210> 9133
<211> 100
<212> DNA
<213> Escherichia coli K-12 MG1655
<220> yebg b1849
<222> 101-200
<223> yebg b1849 U00096 complement(1928481..1928771)
<313> 9133: 157-176
<400> 9133

CGGCGGATCT GCTTGACACC TGGCTGACAA ATTCTCCAGT GCAAATGGAA 50
GACGAGCAAC GTGAAGCCCT TTCGCTATGG CTGGCAGAAC AAAAAGATGT 100
<210> 9134
<211> 100
<212> DNA
<213> Escherichia coli K-12 MG1655
<220> yebh b1823
<222> 711-810
<223> yebh b1823 U00096 complement(1904275..1905084)
<313> 9134: 750-769
<400> 9134
CTGGCGTGCG AAGCCAGAAG TCTGGCAAAC ACTGGCAGCA AAAGAAGTGT 50
TCGACTGCCA GACGGACTTT AATATTCACC TCTGGCAGCG TTCTTATTAA 100
<210> 9135
<211> 100
<212> DNA
<213> Escherichia coli K-12 MG1655
<220> yebh b1823
<222> 577-676
<223> yebh b1823 U00096 complement(1904275..1905084)
<313> 9135: 626-645
<400> 9135
AATGAAGTAC ATCTTCATGC ACCTCATGCA GAACAACTGG AAGGTTTTAC 50
ATTACAGCAG AGTGCGGAGT TGTGTTATCC GATGCGTCTT CGCGGTGATG 100
<210> 9136
<211> 100
<212> DNA
<213> Escherichia coli K-12 MG1655
<220> yebh b1823
<222> 516-615
<223> yebh b1823 U00096 complement(1904275..1905084)
<313> 9136: 563-582
<400> 9136
CTGGGTCATT ACTGCCACGC CGGGACCGCG ACATTTGATG GAGCTGAAGG 50
GGCTGATTTA CAATGAAGTA CATCTTCATG CACCTCATGC AGAACAACTG 100
<210> 9137
<211> 100
<212> DNA
<213> Escherichia coli K-12 MG1655
<220> yebi b1860
<222> 642-741
<223> yebi b1860 U00096 1941438..1942223
<313> 9137: 669-688
<400> 9137
TGGTGTCGCT GTTTTGGTGG GGATGGTGGC AGTGACTGGC GGTTTAACCT 50
TTTCCGCGGT TTACGATACG CCGGCGGGTC CGTCGGTGGT GCTATGTGCG 100
<210> 9138
<211> 100
<212> DNA
<213> Escherichia coli K-12 MG1655
<220> yebi b1860
<222> 557-656
<223> yebi b1860 U00096 1941438..1942223
<313> 9138: 611-630
<400> 9138
TTGTCGGTGC GTTGATTATT ACTTCGTTGC TGATTATTCC TGCTGCTACT 50
GCGCGTCGCT TTGCCCGCAC GCCGGAACAG ATGGCTGGTG TCGCTGTTTT 100
<210> 9139
<211> 100
<212> DNA
<213> Escherichia coli K-12 MG1655
<220> yebi b1860
<222> 477-576
<223> yebi b1860 U00096 1941438..1942223
<313> 9139: 536-555
<400> 9139
TGTTGATGGT GTGAAATTAC AGCGAGTGAA ATTGTTGTTG ATGCTGGTGA 50
CGGCATTGAC GATTGGTGTA GCGATGAAAT TTGTCGGTGC GTTGATTATT 100
<210> 9140
<211> 100
<212> DNA

<213> Escherichia coli K-12 MG1655
<220> yebj yebJ
<222> 11-110
<223> yebj yebJ U00096 complement(1912860..1912979)
<313> 9140: 35-54
<400> 9140
GTCAAAACGC GATGGATGCC ACCGTATTAG AAATCACCAA AGACGGCGTC  50
CGCGTCCAGC TGAATTCGGG TATGTCTTTG ATTGTGCGCG CAGAACACCT  100
<210> 9141
<211> 100
<212> DNA
<213> Escherichia coli K-12 MG1655
<220> yebj yebJ
<222> 1-100
<223> yebj yebJ U00096 complement(1912860..1912979)
<313> 9141: 18-37
<400> 9141
GTGAAAGCGG GTCAAAACGC GATGGATGCC ACCGTATTAG AAATCACCAA  50
AGACGGCGTC CGCGTCCAGC TGAATTCGGG TATGTCTTTG ATTGTGCGCG  100
<210> 9142
<211> 100
<212> DNA
<213> Escherichia coli K-12 MG1655
<220> yebj yebJ
<222> 1-100
<223> yebj yebJ U00096 complement(1912860..1912979)
<313> 9142: 11-30
<400> 9142
GTGAAAGCGG GTCAAAACGC GATGGATGCC ACCGTATTAG AAATCACCAA  50
AGACGGCGTC CGCGTCCAGC TGAATTCGGG TATGTCTTTG ATTGTGCGCG  100
<210> 9143
<211> 100
<212> DNA
<213> Escherichia coli K-12 MG1655
<220> yebk b1854
<222> 730-829
<223> yebk b1854 U00096 1934676..1935545
<313> 9143: 789-808
<400> 9143
CTGACCGTGA TAGATGTGCT GGCGACAGGA TTTACTTTGC GACGCGGTGC  50
AAAATTCAGA GATAACTTGA AGCGGGTCAA AGAAGCGCTG AAGGAATCGC  100
<210> 9144
<211> 100
<212> DNA
<213> Escherichia coli K-12 MG1655
<220> yebk b1854
<222> 645-744
<223> yebk b1854 U00096 1934676..1935545
<313> 9144: 672-691
<400> 9144
CGCCCGGGAA GCAACGCTGG CAATTACCCT CGACGTACCG GAAGATACTG  50
ACATTTATAT GCCCATGGTT TCTCGACTTG CACAGCTGAC CGTGATAGAT  100
<210> 9145
<211> 100
<212> DNA
<213> Escherichia coli K-12 MG1655
<220> yebk b1854
<222> 517-616
<223> yebk b1854 U00096 1934676..1935545
<313> 9145: 554-573
<400> 9145
TGTAGCGACG GAGACGTGGT GGTGCTGATT TCTCACACTG GAAGAACAAA  50
AAATCTGGTC GAGCTGGCGC AGCTGGCACG CGAAAACGAC GCCATGGTGA  100
<210> 9146
<211> 100
<212> DNA
<213> Escherichia coli K-12 MG1655
<220> yebl b1858
<222> 888-987
<223> yebl b1858 U00096 complement(1939675..1940661)
<313> 9146: 962-981

<400> 9146
GGATCCCCTG GGGACGAATA TCAAACTGGG TAAAACAAGC TATTCAGAAT 50
TCCTGAGTCA ATTAGCCAAC CAGTATGCGA GCTGCCTGAA AGGAGATTAA 100
<210> 9147
<211> 100
<212> DNA
<213> Escherichia coli K-12 MG1655
<220> yebl b1858
<222> 810-909
<223> yebl b1858 U00096 complement(1939675..1940661)
<313> 9147: 856-875
<400> 9147
CGTTTTTGCT GAGCCACAGT TCAGGCCAGC GGTCGTTGAG AGCGTCGCAC 50
GAGGGACATC CGTTCGTATG GGAACGTTGG ATCCCCTGGG GACGAATATC 100
<210> 9148
<211> 100
<212> DNA
<213> Escherichia coli K-12 MG1655
<220> yebl b1858
<222> 787-886
<223> yebl b1858 U00096 complement(1939675..1940661)
<313> 9148: 804-823
<400> 9148
TTGGTTGAGC AAAAAGCAAC CTGCGTTTTT GCTGAGCCAC AGTTCAGGCC 50
AGCGGTCGTT GAGAGCGTCG CACGAGGGAC ATCCGTTCGT ATGGGAACGT 100
<210> 9149
<211> 100
<212> DNA
<213> Escherichia coli K-12 MG1655
<220> b2531 b2531
<222> 390-489
<223> b2531 b2531 U00096 complement(2659663..2660151)
<313> 9149: 434-453
<400> 9149
TAATAACCAG GAAGTGCTGG ATGTGTCTGG TCGTCAGCAT ACTCACGACG 50
CGCCACGCAC CCGCACACAA GACGCGATCG ACGTTAAGTT ACGCGCTTAA 100
<210> 9150
<211> 100
<212> DNA
<213> Escherichia coli K-12 MG1655
<220> b2531 b2531
<222> 149-248
<223> b2531 b2531 U00096 complement(2659663..2660151)
<313> 9150: 199-218
<400> 9150
GTAAAAATGG TCTGGTTTCC AGCGTACGTG GACCAGGCGG TGGTTATCTG 50
TTAGGCAAAG ATGCCAGCAG CATCGCCGTT GGCGAAGTAA TTAGCGCCGT 100
<210> 9151
<211> 100
<212> DNA
<213> Escherichia coli K-12 MG1655
<220> b2531 b2531
<222> 124-223
<223> b2531 b2531 U00096 complement(2659663..2660151)
<313> 9151: 135-154
<400> 9151
CTGGAACAAC TGTTTTCCCG TCTGCGTAAA AATGGTCTGG TTTCCAGCGT 50
ACGTGGACCA GGCGGTGGTT ATCTGTTAGG CAAAGATGCC AGCAGCATCG 100
<210> 9152
<211> 100
<212> DNA
<213> Escherichia coli K-12 MG1655
<220> yebm b1859
<222> 657-756
<223> yebm b1859 U00096 1940686..1941441
<313> 9152: 717-736
<400> 9152
TCGTGGTGCT GAACAACTGG GTATCTATCG CCATCATCAT AACCATCGTC 50
ACGATTTACA GGGACGAATT GTTTTGCGTC GGGGAAATGA TCGCTCATGA 100
<210> 9153
<211> 100

<212> DNA
<213> Escherichia coli K-12 MG1655
<220> yebm b1859
<222> 519-618
<223> yebm b1859 U00096 1940686..1941441
<313> 9153: 584-603
<400> 9153
CGTTTTAATG GTTTCTCACG ATCTGCATCT GGTGATGGCA AAAACCGATG 50
AAGTGCTGTG CCTGAATCAC CACATTTGTT GTTCCGGCAC ACCGGAAGTT 100
<210> 9154
<211> 100
<212> DNA
<213> Escherichia coli K-12 MG1655
<220> yebm b1859
<222> 499-598
<223> yebm b1859 U00096 1940686..1941441
<313> 9154: 521-540
<400> 9154
CGTCGTGAAC TGGATTGTGG CGTTTTAATG GTTTCTCACG ATCTGCATCT 50
GGTGATGGCA AAAACCGATG AAGTGCTGTG CCTGAATCAC CACATTTGTT 100
<210> 9155
<211> 100
<212> DNA
<213> Escherichia coli K-12 MG1655
<220> b2532 b2533
<222> 642-741
<223> b2532 b2533 U00096 complement(2660603..2661343)
<313> 9155: 707-726
<400> 9155
TCTGTTTACC CGTGCGCGCC CGGAAAGCCA GGAGTTGAAT ATCCTGCGCG 50
GGATTCTGGC TTCTATTGAG CAGCAGAATA AAGGTAACAA GGCCGAATAA 100
<210> 9156
<211> 100
<212> DNA
<213> Escherichia coli K-12 MG1655
<220> b2532 b2533
<222> 558-657
<223> b2532 b2533 U00096 complement(2660603..2661343)
<313> 9156: 616-635
<400> 9156
TTATGGTCAT CTGGAACAAA CGCTGCTGGC AACCGGTTTT ATCCGTGAAA 50
ACCATCCGGG GCAGGTGATG AATAAATTGC GCCGTCTGTT TACCCGTGCG 100
<210> 9157
<211> 100
<212> DNA
<213> Escherichia coli K-12 MG1655
<220> b2532 b2533
<222> 498-597
<223> b2532 b2533 U00096 complement(2660603..2661343)
<313> 9157: 529-548
<400> 9157
CGGCGAGCAG GTCGAGCATG AAGAGACGCC GTATCCGCTG GTCGATGATC 50
TGGAGCGTTT TTATGGTCAT CTGGAACAAA CGCTGCTGGC AACCGGTTTT 100
<210> 9158
<211> 100
<212> DNA
<213> Escherichia coli K-12 MG1655
<220> b2534 b2535
<222> 743-842
<223> b2534 b2535 U00096 2662383..2663264
<313> 9158: 772-791
<400> 9158
ATAGCCTGGC AAAAGAGCCA AAACGGTTGA TCCTAATCCC GGATGGCGAA 50
CACATTGATG CTTTTTCCGA TCGTCACGGC GATGTTTATC GCGAACAGAT 100
<210> 9159
<211> 100
<212> DNA
<213> Escherichia coli K-12 MG1655
<220> b2534 b2535
<222> 649-748
<223> b2534 b2535 U00096 2662383..2663264

<313> 9159: 688-707
<400> 9159
GAAAATTATA TCGCCAGCGT CAGCCCGATC CCGCTTTTAC TCATTCACGG 50
TAAAGCTGAT CACGTTATCC CATGGCAGCA CAGCGAAAAG TTGTATAGCC 100
<210> 9160
<211> 100
<212> DNA
<213> Escherichia coli K-12 MG1655
<220> b2534 b2535
<222> 591-690
<223> b2534 b2535 U00096 2662383..2663264
<313> 9160: 640-659
<400> 9160
CATCGCCAAC CAAATGATCC CCGGCAGTGG CTACTTACTT GATGAGAGTT 50
ACAGCGGCGA AAATTATATC GCCAGCGTCA GCCCGATCCC GCTTTTACTC 100
<210> 9161
<211> 100
<212> DNA
<213> Escherichia coli K-12 MG1655
<220> b1806 b1807
<222> 432-531
<223> b1806 b1807 U00096 complement(1887975..1888556)
<313> 9161: 453-472
<400> 9161
CAAACGTTGG CATTTAACCC AGCAGGTGAT TATGCCGCCT CAGCCGATTG 50
ATCCATGGTT TTATGGCGGT CGTGGCTGGC CCTATGGCTA CGGCGGATGG 100
<210> 9162
<211> 100
<212> DNA
<213> Escherichia coli K-12 MG1655
<220> b1806 b1807
<222> 324-423
<223> b1806 b1807 U00096 complement(1887975..1888556)
<313> 9162: 373-392
<400> 9162
GGATTTCCGT GGACAACTGG TTACGGTAGT CGGGCCAATC ACTGGTGCGG 50
TTGACGGCAA AATCGGCAAT ACGCCCTATA AATTTATGGT GATGCAAGTA 100
<210> 9163
<211> 100
<212> DNA
<213> Escherichia coli K-12 MG1655
<220> b1806 b1807
<222> 269-368
<223> b1806 b1807 U00096 complement(1887975..1888556)
<313> 9163: 305-324
<400> 9163
GAGAACCTTC TCGCGGTCGC ATTTATGCCG ATGTGAACGG TTTTCTGGAC 50
CCGGTGGATT TCCGTGGACA ACTGGTTACG GTAGTCGGGC CAATCACTGG 100
<210> 9164
<211> 100
<212> DNA
<213> Escherichia coli K-12 MG1655
<220> b1808 b1808
<222> 1812-1911
<223> b1808 b1808 U00096 complement(1889349..1891259)
<313> 9164: 1856-1875
<400> 9164
TCCTTACGGC GCGACGTTTC TCGCCAGTCT GCCGCCCGCG CCACGCACCC 50
GTGACATTGC CCGTGCGGTT CGTTTCCTTG CGATACCATC CTCCAGGTAA 100
<210> 9165
<211> 100
<212> DNA
<213> Escherichia coli K-12 MG1655
<220> b1808 b1808
<222> 1658-1757
<223> b1808 b1808 U00096 complement(1889349..1891259)
<313> 9165: 1705-1724
<400> 9165
GCATGGAAGA TTGTCGTTTG CGCGGTGGCG ACCCGTTCGA TGAAGTGCAA 50
CTACCAGATG CCGTCATTAC TCTCAAACAG GGGGTAGGGC GACTGATTCG 100
<210> 9166

<211> 100
<212> DNA
<213> Escherichia coli K-12 MG1655
<220> b1808 b1808
<222> 1597-1696
<223> b1808 b1808 U00096 complement(1889349..1891259)
<313> 9166: 1625-1644
<400> 9166
TCATTGGTAA TTATCGACAA ATTGCCGTTT ACCTCGCCGG ATGATCCACT 50
GTTAAAAGCG CGCATGGAAG ATTGTCGTTT GCGCGGTGGC GACCCGTTCG 100
<210> 9167
<211> 100
<212> DNA
<213> Escherichia coli K-12 MG1655
<220> b1809 b1809
<222> 263-362
<223> b1809 b1809 U00096 1891343..1891735
<313> 9167: 297-316
<400> 9167
ACTTCGCGGC GATGAATAAA GCGTGGGATG CTTGGGTTGT CGCGGGTCAT 50
GCGCCGGTGC GCTGCACGGT ACAAGCGGGT TTGATGAACC CGAAGTATAA 100
<210> 9168
<211> 100
<212> DNA
<213> Escherichia coli K-12 MG1655
<220> b1809 b1809
<222> 222-321
<223> b1809 b1809 U00096 1891343..1891735
<313> 9168: 244-263
<400> 9168
GAGCATTCTG GATGCCACCA TTTTCCTGGC CGATAAAAAC GACTTCGCGG 50
CGATGAATAA AGCGTGGGAT GCTTGGGTTG TCGCGGGTCA TGCGCCGGTG 100
<210> 9169
<211> 100
<212> DNA
<213> Escherichia coli K-12 MG1655
<220> b1809 b1809
<222> 169-268
<223> b1809 b1809 U00096 1891343..1891735
<313> 9169: 196-215
<400> 9169
AACACGCTGG CACAGATTGA CGCCGTGCTG GAAAAACAGG GCAGCAATAA 50
ATCGAGCATT CTGGATGCCA CCATTTTCCT GGCCGATAAA AACGACTTCG 100
<210> 9170
<211> 100
<212> DNA
<213> Escherichia coli K-12 MG1655
<220> yebu yebU
<222> 1347-1446
<223> yebu yebU U00096 1918241..1919686
<313> 9170: 1404-1423
<400> 9170
TCAACCGATT GGTTTAGCCA AACGGATTGG TTCGCGATTG AAAAACAGCT 50
ATCCGCGTGA ACTGGTGCGC GATGGGAAAC TTTTTACCGG TAACGCCTGA 100
<210> 9171
<211> 100
<212> DNA
<213> Escherichia coli K-12 MG1655
<220> yebu yebU
<222> 1299-1398
<223> yebu yebU U00096 1918241..1919686
<313> 9171: 1322-1341
<400> 9171
CCCGCAAGCC GCGCCAGTGG CGGATGATGT ATTGGTTACT TTCCAGCATC 50
AACCGATTGG TTTAGCCAAA CGGATTGGTT CGCGATTGAA AAACAGCTAT 100
<210> 9172
<211> 100
<212> DNA
<213> Escherichia coli K-12 MG1655
<220> yebu yebU
<222> 1228-1327

<223> yebu yebU U00096 1918241..1919686
<313> 9172: 1251-1270
<400> 9172
CCCGATAATA TGAATGCATT CGAGCTGACA CCGCAGGAAG CGGAGGAGTG 50
GTATCGCGGG CGCGATGTTT ACCCGCAAGC CGCGCCAGTG GCGGATGATG 100
<210> 9173
<211> 100
<212> DNA
<213> Escherichia coli K-12 MG1655
<220> inaa b2238
<222> 552-651
<223> inaa b2238 U00096 complement(2346842..2347492)
<313> 9173: 618-637
<400> 9173
TAAAGCGATT AATCATGACT TCCGCCAGCT GGAAAAATAT CTGGAGCCAA 50
TCCCCAAAGC GGACTGGGAG CAGGTGAAAG CGTATTACTA CGCCATGTAA 100
<210> 9174
<211> 100
<212> DNA
<213> Escherichia coli K-12 MG1655
<220> inaa b2238
<222> 470-569
<223> inaa b2238 U00096 complement(2346842..2347492)
<313> 9174: 526-545
<400> 9174
GCCATATCTA TGTGAAAACA GAAGGTAATG CAGAAGCTGG TTTTCTTGAC 50
CTGGAGAAAA GTCGTCGCCG CTTGCGTCGC GATAAAGCGA TTAATCATGA 100
<210> 9175
<211> 100
<212> DNA
<213> Escherichia coli K-12 MG1655
<220> inaa b2238
<222> 336-435
<223> inaa b2238 U00096 complement(2346842..2347492)
<313> 9175: 380-399
<400> 9175
CATTGCTGAC TGGTATGCCC AGCATGCAGT ATCGCCTTAT TCTGACGAAG 50
TACGGCAAGC CATGTTGAAA GCAGTGGCGC TGGCGTTTAA GAAAATGCAT 100
<210> 9176
<211> 100
<212> DNA
<213> Escherichia coli K-12 MG1655
<220> yeca b1909
<222> 555-654
<223> yeca b1909 U00096 complement(1988977..1989642)
<313> 9176: 591-610
<400> 9176
TCCGCAGGAA AAAGCTGTTC AGCAACCGAT CAAAGCAGAA GAGAAACCGG 50
GGCGTAACGA TCCTTGCCCG TGCGGTAGTG GTAAGAAATT TAAGCAGTGC 100
<210> 9177
<211> 100
<212> DNA
<213> Escherichia coli K-12 MG1655
<220> yeca b1909
<222> 535-634
<223> yeca b1909 U00096 complement(1988977..1989642)
<313> 9177: 553-572
<400> 9177
CATGCGTACT GGATGGCGCA TCCGCAGGAA AAAGCTGTTC AGCAACCGAT 50
CAAAGCAGAA GAGAAACCGG GGCGTAACGA TCCTTGCCCG TGCGGTAGTG 100
<210> 9178
<211> 100
<212> DNA
<213> Escherichia coli K-12 MG1655
<220> yeca b1909
<222> 469-568
<223> yeca b1909 U00096 complement(1988977..1989642)
<313> 9178: 515-534
<400> 9178
GAAAAGATGT CGCCAGAAGC GTTTGAAGAG AGCGTAGATG CCATTCGACT 50
GGCGGCGCTT GATCTACATG CGTACTGGAT GGCGCATCCG CAGGAAAAAG 100

<210> 9179
<211> 100
<212> DNA
<213> Escherichia coli K-12 MG1655
<220> yecc b1918
<222> 654-753
<223> yecc b1918 U00096 complement(1995085..1995837)
<313> 9179: 704-723
<400> 9179
CCAGGGGCGG ATAGTCGAGC AGGGGGCCGC AAAAGCGTTA TTTGCCGACC 50
CCGAGCAGCC TCGCACCCGC CAGTTCCTCG AGAAGTTTCT GCTGCAATAA 100
<210> 9180
<211> 100
<212> DNA
<213> Escherichia coli K-12 MG1655
<220> yecc b1918
<222> 628-727
<223> yecc b1918 U00096 complement(1995085..1995837)
<313> 9180: 670-689
<400> 9180
GTTGCGGACC GGGCGATCTT TATGGACCAG GGGCGGATAG TCGAGCAGGG 50
GGCCGCAAAA GCGTTATTTG CCGACCCCGA GCAGCCTCGC ACCCGCCAGT 100
<210> 9181
<211> 100
<212> DNA
<213> Escherichia coli K-12 MG1655
<220> yecc b1918
<222> 564-663
<223> yecc b1918 U00096 complement(1995085..1995837)
<313> 9181: 588-607
<400> 9181
TCAGCTGGCG CAGGAAAAGC GCACGATGGT GATTGTGACG CACGAAATGA 50
GCTTTGCCCG GGATGTTGCG GACCGGGCGA TCTTTATGGA CCAGGGGCGG 100
<210> 9182
<211> 100
<212> DNA
<213> Escherichia coli K-12 MG1655
<220> yecd b1868
<222> 497-596
<223> yecd b1868 U00096 1948823..1949422
<313> 9182: 545-564
<400> 9182
CCTGTAGTGC CGCCAGCGCC GAGCAGCACA ATAACAGTAT CAACCATATC 50
TACCCGCGCA TCGCCCGTGT GCGTAGCGTG GAAGAGATCC TCAACGCGTT 100
<210> 9183
<211> 100
<212> DNA
<213> Escherichia coli K-12 MG1655
<220> yecd b1868
<222> 469-568
<223> yecd b1868 U00096 1948823..1949422
<313> 9183: 492-511
<400> 9183
GGCTTTAATC TGGTGATTGC CGAAGACGCC TGTAGTGCCG CCAGCGCCGA 50
GCAGCACAAT AACAGTATCA ACCATATCTA CCCGCGCATC GCCCGTGTGC 100
<210> 9184
<211> 100
<212> DNA
<213> Escherichia coli K-12 MG1655
<220> yecd b1868
<222> 417-516
<223> yecd b1868 U00096 1948823..1949422
<313> 9184: 454-473
<400> 9184
GATCTCGACC AATATCGGTG TTGAATCCAC CGCCCGCAAT GCCTGGGAAC 50
TTGGCTTTAA TCTGGTGATT GCCGAAGACG CCTGTAGTGC CGCCAGCGCC 100
<210> 9185
<211> 100
<212> DNA
<213> Escherichia coli K-12 MG1655
<220> yece b1869

<222> 720-819
<223> yece b1869 U00096 1949419..1950237
<313> 9185: 794-813
<400> 9185
GGCACCGGAA CTGGTACATA CCCTGTGGGA AGACTTACGT AAAACGCTTC  50
CAGAGATCGG AGCAGTTCCG GCTATTCCAC AGCAATCTTC TCTTTTCTGA  100
<210> 9186
<211> 100
<212> DNA
<213> Escherichia coli K-12 MG1655
<220> yece b1869
<222> 675-774
<223> yece b1869 U00096 1949419..1950237
<313> 9186: 712-731
<400> 9186
GACCACTACG CCTTATCTTT TTTTACATAC GCCAGACATT GCCCAGGCAC  50
CGGAACTGGT ACATACCCTG TGGGAAGACT TACGTAAAAC GCTTCCAGAG  100
<210> 9187
<211> 100
<212> DNA
<213> Escherichia coli K-12 MG1655
<220> yece b1869
<222> 644-743
<223> yece b1869 U00096 1949419..1950237
<313> 9187: 678-697
<400> 9187
TCTGGTTACA AAAATTAGCG CAGTGGCATC AGACCACTAC GCCTTATCTT  50
TTTTTACATA CGCCAGACAT TGCCCAGGCA CCGGAACTGG TACATACCCT  100
<210> 9188
<211> 100
<212> DNA
<213> Escherichia coli K-12 MG1655
<220> yecf b1916
<222> 126-225
<223> yecf b1916 U00096 1993841..1994065
<313> 9188: 194-213
<400> 9188
GTTAAAGATG GAAAAACAGC TTGCGCTGAT CGAAGACGAA ACCCAGGCTG  50
CAGTATTTTC CAAAACGGTT AAGCAAATTA AACAAGCCTA CCGTCAGTAA  100
<210> 9189
<211> 100
<212> DNA
<213> Escherichia coli K-12 MG1655
<220> yecf b1916
<222> 126-225
<223> yecf b1916 U00096 1993841..1994065
<313> 9189: 184-203
<400> 9189
GTTAAAGATG GAAAAACAGC TTGCGCTGAT CGAAGACGAA ACCCAGGCTG  50
CAGTATTTTC CAAAACGGTT AAGCAAATTA AACAAGCCTA CCGTCAGTAA  100
<210> 9190
<211> 100
<212> DNA
<213> Escherichia coli K-12 MG1655
<220> yecf b1916
<222> 93-192
<223> yecf b1916 U00096 1993841..1994065
<313> 9190: 149-168
<400> 9190
GGCGATGGGA CAAGCTGACG CGGGCCGCGT GATGTTAAAG ATGGAAAAAC  50
AGCTTGCGCT GATCGAAGAC GAAACCCAGG CTGCAGTATT TTCCAAAACG  100
<210> 9191
<211> 100
<212> DNA
<213> Escherichia coli K-12 MG1655
<220> tldd b3245
<222> 1300-1399
<223> tldd b3245 U00096 complement(3388218..3389663)
<313> 9191: 1327-1346
<400> 9191
ACCATGCAGC AGATTTCGAT GGTTGGCAAC GACCTGAAAC TGGATAACGG  50

CGTGGGTGTC TGCGGTAAAG AAGGGCAAAG TTTGCCGGTT GGCGTGGGCC 100
<210> 9192
<211> 100
<212> DNA
<213> Escherichia coli K-12 MG1655
<220> tldd b3245
<222> 1216-1315
<223> tldd b3245 U00096 complement(3388218..3389663)
<313> 9192: 1242-1261
<400> 9192
TCCACTTCAG AAGCATATCT GATTGAAAAC GGTAAAGTAA CGAAGCCGGT 50
GAAAGGCGCA ACGTTGATTG GTTCCGGTAT CGAAACCATG CAGCAGATTT 100
<210> 9193
<211> 100
<212> DNA
<213> Escherichia coli K-12 MG1655
<220> tldd b3245
<222> 1161-1260
<223> tldd b3245 U00096 complement(3388218..3389663)
<313> 9193: 1205-1224
<400> 9193
TGCACCGAAC TTTGGTGGCG GTCAGGTGGA TATCACCTCC GGCAAATTCG 50
TTTTCTCCAC TTCAGAAGCA TATCTGATTG AAAACGGTAA AGTAACGAAG 100
<210> 9194
<211> 100
<212> DNA
<213> Escherichia coli K-12 MG1655
<220> yecg b1896
<222> 330-429
<223> yecg b1896 U00096 1977777..1978205
<313> 9194: 399-418
<400> 9194
TCACAATCAC AGTTTCTTTT CGCGAGCATC CTGCTCGGCG AAAAGAGTTA 50
TTGCCTCAAG TGAGGTCGAT GTGCTGTTAG TTCCACTTAC GGGAGATTAA 100
<210> 9195
<211> 100
<212> DNA
<213> Escherichia coli K-12 MG1655
<220> yecg b1896
<222> 114-213
<223> yecg b1896 U00096 1977777..1978205
<313> 9195: 179-198
<400> 9195
TCTCGCTTCC GACCCGGAAA TGTACAATCA ATTAGCTGCG CCGATGCTTG 50
AAGATTTACG CAGCGTGATG CATGAAGAAA CGCAAAGCTT TCTTGATAAG 100
<210> 9196
<211> 100
<212> DNA
<213> Escherichia coli K-12 MG1655
<220> yecg b1896
<222> 103-202
<223> yecg b1896 U00096 1977777..1978205
<313> 9196: 136-155
<400> 9196
AGTTTAATTA CTCTCGCTTC CGACCCGGAA ATGTACAATC AATTAGCTGC 50
GCCGATGCTT GAAGATTTAC GCAGCGTGAT GCATGAAGAA ACGCAAAGCT 100
<210> 9197
<211> 100
<212> DNA
<213> Escherichia coli K-12 MG1655
<220> ppha pphA
<222> 488-587
<223> ppha pphA U00096 complement(1920337..1920996)
<313> 9197: 548-567
<400> 9197
TGTGGAGCCG CTCGCGATTA GGTGAACGCC AAAAAGGGCA GGGAATTACA 50
GGTGCTGATC ATTTCTGGTT TGGTCATACA CCGTTGCGAC ATCGCGTGGA 100
<210> 9198
<211> 100
<212> DNA
<213> Escherichia coli K-12 MG1655

<220> ppha pphA
<222> 425-524
<223> ppha pphA U00096 complement(1920337..1920996)
<313> 9198: 464-483
<400> 9198
CTCATGCCGA TTATCCAGAT GATGTTTATG AATGGCAAAA GGACGTTGAT  50
TTGCATCAGG TCTTGTGGAG CCGCTCGCGA TTAGGTGAAC GCCAAAAAGG  100
<210> 9199
<211> 100
<212> DNA
<213> Escherichia coli K-12 MG1655
<220> ppha pphA
<222> 364-463
<223> ppha pphA U00096 complement(1920337..1920996)
<313> 9199: 384-403
<400> 9199
AAATGTCAGC ATTTGCCCTT TATTCTTGAA GTACACAGTC GCACCGGCAA  50
GCATGTTATT GCTCATGCCG ATTATCCAGA TGATGTTTAT GAATGGCAAA  100
<210> 9200
<211> 100
<212> DNA
<213> Escherichia coli K-12 MG1655
<220> yech b1907
<222> 141-240
<223> yech b1907 U00096 complement(1987274..1987513)
<313> 9200: 171-190
<400> 9200
GACAGCGGGA GAGCTGGTAG CGTTTCTGGC AGCAAAAGGC AAATTTATAC  50
CTTCGAAAGA CGGTTTTTCG ACCGATCAGA GTAAGATTTG CCGTCACTGA  100
<210> 9201
<211> 100
<212> DNA
<213> Escherichia coli K-12 MG1655
<220> yech b1907
<222> 107-206
<223> yech b1907 U00096 complement(1987274..1987513)
<313> 9201: 159-178
<400> 9201
AGGCACGTTT TCACACCTGC TCGGCAGAAG GGATGACAGC GGGAGAGCTG  50
GTAGCGTTTC TGGCAGCAAA AGGCAAATTT ATACCTTCGA AAGACGGTTT  100
<210> 9202
<211> 100
<212> DNA
<213> Escherichia coli K-12 MG1655
<220> yech b1907
<222> 105-204
<223> yech b1907 U00096 complement(1987274..1987513)
<313> 9202: 159-178
<400> 9202
ACAGGCACGT TTTCACACCT GCTCGGCAGA AGGGATGACA GCGGGAGAGC  50
TGGTAGCGTT TCTGGCAGCA AAAGGCAAAT TTATACCTTC GAAAGACGGT  100
<210> 9203
<211> 100
<212> DNA
<213> Escherichia coli K-12 MG1655
<220> pphb b2735
<222> 558-657
<223> pphb b2735 U00096 2857783..2858439
<313> 9203: 598-617
<400> 9203
ACATATGATG TTTGATAACA TTCAGACGTT CGCTAACCAG ATTTATATTG  50
ATACCGGATC GCCGAACAGC GGGCGGCTGT CATTTTATAA AATAAAGTAG  100
<210> 9204
<211> 100
<212> DNA
<213> Escherichia coli K-12 MG1655
<220> pphb b2735
<222> 447-546
<223> pphb b2735 U00096 2857783..2858439
<313> 9204: 495-514
<400> 9204

CTTTGGTAAA GAAATAGCGG AGAGCGAATT ACTCTGGCCT GTTGATCGTG 50
TGCAGAAATC GCTTAATGGC GAGTTACAAC AAATAAACGG CGCTGATTAT 100
<210> 9205
<211> 100
<212> DNA
<213> Escherichia coli K-12 MG1655
<220> pphb b2735
<222> 416-515
<223> pphb b2735 U00096 2857783..2858439
<313> 9205: 467-486
<400> 9205
CACATGCAGA TTATCCGGGG AGTGAATATC TCTTTGGTAA AGAAATAGCG 50
GAGAGCGAAT TACTCTGGCC TGTTGATCGT GTGCAGAAAT CGCTTAATGG 100
<210> 9206
<211> 100
<212> DNA
<213> Escherichia coli K-12 MG1655
<220> yeci yecI
<222> 367-466
<223> yeci yecI U00096 1984948..1985451
<313> 9206: 389-408
<400> 9206
CTGGAAAAAG AACAGCAGCA TGATGGTCTG TTGCTGCAAA CCATTCTTGA 50
TGAAGTGCGC AGTGCGAAAC TTGCGGGTAT GTGCCCTGTG CAGACCGACC 100
<210> 9207
<211> 100
<212> DNA
<213> Escherichia coli K-12 MG1655
<220> yeci yecI
<222> 332-431
<223> yeci yecI U00096 1984948..1985451
<313> 9207: 346-365
<400> 9207
TGAATGATGA TTCAACCGTC AATTTCCTGC GCGATCTGGA AAAAGAACAG 50
CAGCATGATG GTCTGTTGCT GCAAACCATT CTTGATGAAG TGCGCAGTGC 100
<210> 9208
<211> 100
<212> DNA
<213> Escherichia coli K-12 MG1655
<220> yeci yecI
<222> 259-358
<223> yeci yecI U00096 1984948..1985451
<313> 9208: 313-332
<400> 9208
TTCCAAAAAA CGATGGAAGA ATACGAGCAA CGTTCTAGTA CGTTGGCACA 50
GTTAGCCGAT GAAGCGAAAG AACTGAATGA TGATTCAACC GTCAATTTCC 100
<210> 9209
<211> 100
<212> DNA
<213> Escherichia coli K-12 MG1655
<220> b1810 b1810
<222> 210-309
<223> b1810 b1810 U00096 1892097..1892456
<313> 9209: 268-287
<400> 9209
GGCGCGAGCT GACCAGGAAG GCTGGAACCC TGGGTTCACG GAAAAAATGG 50
TTGGATGGGC AAAAAAAATG GAGACAGGTG AACGTTCTGT GATTAAAAAT 100
<210> 9210
<211> 100
<212> DNA
<213> Escherichia coli K-12 MG1655
<220> b1810 b1810
<222> 173-272
<223> b1810 b1810 U00096 1892097..1892456
<313> 9210: 211-230
<400> 9210
AAGAACTGGG AGTACCCGCG AGTGCCGCTG ATATTACGGC GCGAGCTGAC 50
CAGGAAGGCT GGAACCCTGG GTTCACGGAA AAAATGGTTG GATGGGCAAA 100
<210> 9211
<211> 100
<212> DNA

<213> Escherichia coli K-12 MG1655
<220> b1810 b1810
<222> 101-200
<223> b1810 b1810 U00096 1892097..1892456
<313> 9211: 128-147
<400> 9211
CCATGGATGT ATCAGCGCCA ACACCAAGTT CGATGAATGA AAGCACGGCG 50
AAGGGAATCT TTAAATATTT AAAAGAACTG GGAGTACCCG CGAGTGCCGC 100
<210> 9212
<211> 100
<212> DNA
<213> Escherichia coli K-12 MG1655
<220> yeck b1874
<222> 1002-1101
<223> yeck b1874 U00096 complement(1955056..1956156)
<313> 9212: 1074-1093
<400> 9212
CAAAGGAATG GGGGCACGAA CCAGCATGAG CGAAAACGAA CTGGACATTT 50
TAACGCGGTA TTTCCAGTAC AACGCCAAAG ATATTACCGA GAAACAGTGA 100
<210> 9213
<211> 100
<212> DNA
<213> Escherichia coli K-12 MG1655
<220> yeck b1874
<222> 992-1091
<223> yeck b1874 U00096 complement(1955056..1956156)
<313> 9213: 1018-1037
<400> 9213
CGTCCATTGC CAAAGGAATG GGGGCACGAA CCAGCATGAG CGAAAACGAA 50
CTGGACATTT TAACGCGGTA TTTCCAGTAC AACGCCAAAG ATATTACCGA 100
<210> 9214
<211> 100
<212> DNA
<213> Escherichia coli K-12 MG1655
<220> yeck b1874
<222> 917-1016
<223> yeck b1874 U00096 complement(1955056..1956156)
<313> 9214: 956-975
<400> 9214
AAAAACTTGA CGATACCTAT TGCGCTGGTT GTCATGCCCC TATTGCCGCC 50
GACCATTACA CCGTCAATGC GTGGCCGTCC ATTGCCAAAG GAATGGGGGC 100
<210> 9215
<211> 100
<212> DNA
<213> Escherichia coli K-12 MG1655
<220> b1811 b1812
<222> 81-180
<223> b1811 b1812 U00096 complement(1892576..1892755)
<313> 9215: 128-147
<400> 9215
GATGAGCAGC GGCCAGGCAA TCGCGCTGGT GGCAGAAGAG CTGCGTGCCA 50
ACCACTCCGG CGAGCGAATC GTGGCGCGTT TTGAGGATGA AGACGAGTAA 100
<210> 9216
<211> 100
<212> DNA
<213> Escherichia coli K-12 MG1655
<220> b1811 b1812
<222> 29-128
<223> b1811 b1812 U00096 complement(1892576..1892755)
<313> 9216: 72-91
<400> 9216
ATGAACAGCA GCAAAAAGCT GTCGAGCGGA TCCAGGAACT GATGGCACAA 50
GGGATGAGCA GCGGCCAGGC AATCGCGCTG GTGGCAGAAG AGCTGCGTGC 100
<210> 9217
<211> 100
<212> DNA
<213> Escherichia coli K-12 MG1655
<220> b1811 b1812
<222> 1-100
<223> b1811 b1812 U00096 complement(1892576..1892755)
<313> 9217: 25-44

<400> 9217
ATGTTTGCAG GTTTACCTTC ACTCACCCAT GAACAGCAGC AAAAAGCTGT 50
CGAGCGGATC CAGGAACTGA TGGCACAAGG GATGAGCAGC GGCCAGGCAA 100
<210> 9218
<211> 100
<212> DNA
<213> Escherichia coli K-12 MG1655
<220> yecm b1876
<222> 474-573
<223> yecm b1876 U00096 complement(1957304..1957876)
<313> 9218: 549-568
<400> 9218
ATTACCGAAT CCGACGCTGG CGGTCACCGA TGGCAAAACA ACTATTAAGT 50
TTCATCCGTG GTCCATTGAA GAGATCGTTG CCAGCGAGCA GTCGGCGTAA 100
<210> 9219
<211> 100
<212> DNA
<213> Escherichia coli K-12 MG1655
<220> yecm b1876
<222> 469-568
<223> yecm b1876 U00096 complement(1957304..1957876)
<313> 9219: 501-520
<400> 9219
GAGCGATTAC CGAATCCGAC GCTGGCGGTC ACCGATGGCA AAACAACTAT 50
TAAGTTTCAT CCGTGGTCCA TTGAAGAGAT CGTTGCCAGC GAGCAGTCGG 100
<210> 9220
<211> 100
<212> DNA
<213> Escherichia coli K-12 MG1655
<220> yecm b1876
<222> 422-521
<223> yecm b1876 U00096 complement(1957304..1957876)
<313> 9220: 461-480
<400> 9220
GCCTACCGGG GATCTCAGTG AAAACCAGTT CGCCAAAAGG TGAGCATGAG 50
CGATTACCGA ATCCGACGCT GGCGGTCACC GATGGCAAAA CAACTATTAA 100
<210> 9221
<211> 100
<212> DNA
<213> Escherichia coli K-12 MG1655
<220> yecn b1870
<222> 208-307
<223> yecn b1870 U00096 1950260..1950685
<313> 9221: 260-279
<400> 9221
CTGATGCTGT TTATGGAAAT GAATGGCGCA GAAACCTGGA TGGTGCATAT 50
TTGCGGCATC GTTTTGCTTG CTGGTCGTCT GATGCATTAT TACGGTTTTC 100
<210> 9222
<211> 100
<212> DNA
<213> Escherichia coli K-12 MG1655
<220> yecn b1870
<222> 194-293
<223> yecn b1870 U00096 1950260..1950685
<313> 9222: 228-247
<400> 9222
CCATCGCGAT TGTGCTGATG CTGTTTATGG AAATGAATGG CGCAGAAACC 50
TGGATGGTGC ATATTTGCGG CATCGTTTTG CTTGCTGGTC GTCTGATGCA 100
<210> 9223
<211> 100
<212> DNA
<213> Escherichia coli K-12 MG1655
<220> yecn b1870
<222> 1-100
<223> yecn b1870 U00096 1950260..1950685
<313> 9223: 8-27
<400> 9223
GTGCCATCGG CCATTTTAAA GGGAGTTTGT ATGGTAAGCG CGCTGTACGC 50
CGTTTTAAGT GCGTTGTTAT TAATGAAGTT CTCTTTTGAT GTCGTTCGCC 100
<210> 9224
<211> 100

<212> DNA
<213> Escherichia coli K-12 MG1655
<220> yeco b1871
<222> 597-696
<223> yeco b1871 U00096 1950726..1951469
<313> 9224: 648-667
<400> 9224
CAGCATGCTG GAAAACGTGA TGCTGACCGA TTCCGTGGAA ACCCATAAAG 50
CACGCCTGCA TAACGCCGGT TTTGAGCATA GTGAGCTGTG GTTCCAGTGC 100
<210> 9225
<211> 100
<212> DNA
<213> Escherichia coli K-12 MG1655
<220> yeco b1871
<222> 543-642
<223> yeco b1871 U00096 1950726..1951469
<313> 9225: 565-584
<400> 9225
CCACGACTTT AAACGTGCCA ACGGTTACAG CGAACTGGAG ATCAGCCAGA 50
AGCGCAGCAT GCTGGAAAAC GTGATGCTGA CCGATTCCGT GGAAACCCAT 100
<210> 9226
<211> 100
<212> DNA
<213> Escherichia coli K-12 MG1655
<220> yeco b1871
<222> 505-604
<223> yeco b1871 U00096 1950726..1951469
<313> 9226: 532-551
<400> 9226
GAAGATGCCA AAGTTGGTGA ACTGTTGTTC AACATGCACC ACGACTTTAA 50
ACGTGCCAAC GGTTACAGCG AACTGGAGAT CAGCCAGAAG CGCAGCATGC 100
<210> 9227
<211> 100
<212> DNA
<213> Escherichia coli K-12 MG1655
<220> b1815 b1816
<222> 1528-1627
<223> b1815 b1816 U00096 1896421..1898049
<313> 9227: 1585-1604
<400> 9227
CAGCCGATGC CGCTACGTGA TTTTCCCAAA TGGCTGGCGG GCAGCCAACC 50
GCCGCCCGCC CGGCATAATG GACATATCAC GCCCATTATG CCGTTACGTT 100
<210> 9228
<211> 100
<212> DNA
<213> Escherichia coli K-12 MG1655
<220> b1815 b1816
<222> 1509-1608
<223> b1815 b1816 U00096 1896421..1898049
<313> 9228: 1523-1542
<400> 9228
GCAAGGGTAT TTGTACGCAC AGCCGATGCC GCTACGTGAT TTTCCCAAAT 50
GGCTGGCGGG CAGCCAACCG CCGCCCGCCC GGCATAATGG ACATATCACG 100
<210> 9229
<211> 100
<212> DNA
<213> Escherichia coli K-12 MG1655
<220> b1815 b1816
<222> 1442-1541
<223> b1815 b1816 U00096 1896421..1898049
<313> 9229: 1462-1481
<400> 9229
TGGTGGCGGA GGGCGTGGAA ACGCAAGAAC AGGCGAAGTA TTTGCGCCGT 50
CATGGCGTGC ATATTTTGCA AGGGTATTTG TACGCACAGC CGATGCCGCT 100
<210> 9230
<211> 100
<212> DNA
<213> Escherichia coli K-12 MG1655
<220> yecp b1872
<222> 857-956
<223> yecp b1872 U00096 1951466..1952437

<313> 9230: 888-907
<400> 9230
CCGAATGGAT GGTTACCGAA TCGCTGGCCG ATTTCCTCGA CCCACATGAT  50
CCGGGTAAAA CGGTGGAAGG TTATCCGGCA CCTAAACGCG CGGTGCTGAT  100
<210> 9231
<211> 100
<212> DNA
<213> Escherichia coli K-12 MG1655
<220> yecp b1872
<222> 807-906
<223> yecp b1872 U00096 1951466..1952437
<313> 9231: 849-868
<400> 9231
TATCCGCATT GCGGATGTGA GCGTTACCAC CACAGAAGAG CAGCGACGCA  50
CCGAATGGAT GGTTACCGAA TCGCTGGCCG ATTTCCTCGA CCCACATGAT  100
<210> 9232
<211> 100
<212> DNA
<213> Escherichia coli K-12 MG1655
<220> yecp b1872
<222> 757-856
<223> yecp b1872 U00096 1951466..1952437
<313> 9232: 779-798
<400> 9232
CCTTCCGCGC TGGCGCTGAA AAACTGGCTG AAGAAGTGTG GTTTTGTAGA  50
TATCCGCATT GCGGATGTGA GCGTTACCAC CACAGAAGAG CAGCGACGCA  100
<210> 9233
<211> 100
<212> DNA
<213> Escherichia coli K-12 MG1655
<220> yecs b1919
<222> 551-650
<223> yecs b1919 U00096 complement(1995834..1996502)
<313> 9233: 585-604
<400> 9233
AGGTGTTCAC CATGTATCTG GCGGCTTCGC TCATCTACTG GATCATGGCG  50
ACTGTGTTAT CGACGTTGCA GAACCATTTT GAGAATCAAC TTAATCGCCA  100
<210> 9234
<211> 100
<212> DNA
<213> Escherichia coli K-12 MG1655
<220> yecs b1919
<222> 446-545
<223> yecs b1919 U00096 complement(1995834..1996502)
<313> 9234: 496-515
<400> 9234
TGTCGAACAG CTTTATTAGC CTGGTAAAAG ATACGTCGCT GGCCGCGACA  50
ATCCAGGTGC CGGAGCTGTT CCGTCAGGCG CAGTTGATTA CCTCGCGTAC  100
<210> 9235
<211> 100
<212> DNA
<213> Escherichia coli K-12 MG1655
<220> yecs b1919
<222> 252-351
<223> yecs b1919 U00096 complement(1995834..1996502)
<313> 9235: 292-311
<400> 9235
ATTAGATCCC ATTCCGTCAG CAATGATTGG TCTGTCGTTG AACACTGCCG  50
CCTATGCTGC CGAAACGCTG CGCGCCGCCA TTTCTTCTAT TGATAAAGGT  100
<210> 9236
<211> 100
<212> DNA
<213> Escherichia coli K-12 MG1655
<220> yect b1878
<222> 307-406
<223> yect b1878 U00096 1959975..1960484
<313> 9236: 337-356
<400> 9236
AAATCACAGC GAGCCTGGCT GGATTTTAGG GACAAAGAAT GTGAATTAAT  50
CCTTTCAAAT GAGGACGTTC AGGATTTAAG TGACCCTTAT TCTGAATCAG  100
<210> 9237

<211> 100
<212> DNA
<213> Escherichia coli K-12 MG1655
<220> yect b1878
<222> 283-382
<223> yect b1878 U00096 1959975..1960484
<313> 9237: 330-349
<400> 9237
ACCGAACAAC TTAACTTGCT TAAAAAATCA CAGCGAGCCT GGCTGGATTT 50
TAGGGACAAA GAATGTGAAT TAATCCTTTC AAATGAGGAC GTTCAGGATT 100
<210> 9238
<211> 100
<212> DNA
<213> Escherichia coli K-12 MG1655
<220> yect b1878
<222> 245-344
<223> yect b1878 U00096 1959975..1960484
<313> 9238: 289-308
<400> 9238
TAGAACAAGT GCCACGCACA GGTGTAAAAA AACCTGATAC CGAACAACTT 50
AACTTGCTTA AAAAATCACA GCGAGCCTGG CTGGATTTTA GGGACAAAGA 100
<210> 9239
<211> 100
<212> DNA
<213> Escherichia coli K-12 MG1655
<220> yeda b1960
<222> 802-901
<223> yeda b1960 U00096 2027561..2028481
<313> 9239: 839-858
<400> 9239
ATTGAATGGC TGGCGCTCGG CGTAATTGTC TTCGCGGTGG TACTGGTCAC 50
GTTGGGAAAA TATCTCTTCC CGGCAAAACC CGTAGTTGCG CCAGTTATTC 100
<210> 9240
<211> 100
<212> DNA
<213> Escherichia coli K-12 MG1655
<220> yeda b1960
<222> 767-866
<223> yeda b1960 U00096 2027561..2028481
<313> 9240: 797-816
<400> 9240
TGGGTACGGG ACTGGGTGGA GAAACACTGT CGAAGATTGA ATGGCTGGCG 50
CTCGGCGTAA TTGTCTTCGC GGTGGTACTG GTCACGTTGG GAAAATATCT 100
<210> 9241
<211> 100
<212> DNA
<213> Escherichia coli K-12 MG1655
<220> yeda b1960
<222> 681-780
<223> yeda b1960 U00096 2027561..2028481
<313> 9241: 721-740
<400> 9241
CAACGCTTAT ATGTATTTAA TCCGTAATGT CAGTCCGGCT CTCGCCACCA 50
GCTACGCTTA CGTTAACCCG GTGGTCGCGG TCTTGCTGGG TACGGGACTG 100
<210> 9242
<211> 100
<212> DNA
<213> Escherichia coli K-12 MG1655
<220> dmsa b0895
<222> 2259-2358
<223> dmsa b0895 U00096 940269..942626
<313> 9242: 2330-2349
<400> 9242
TAAGGGTGGT TGTATTAACG TACTGACCAC TCAACGTCCG TCTCCTCTCG 50
CTAAGGGGAA TCCGTCACAT ACAAACCTTG TTCAGGTTGA AAAGGTGTAA 100
<210> 9243
<211> 100
<212> DNA
<213> Escherichia coli K-12 MG1655
<220> dmsa b0895
<222> 2244-2343

<223> dmsa b0895 U00096 940269..942626
<313> 9243: 2273-2292
<400> 9243
TGCAAAACGT GTCGATAAGG GTGGTTGTAT TAACGTACTG ACCACTCAAC  50
GTCCGTCTCC TCTCGCTAAG GGGAATCCGT CACATACAAA CCTTGTTCAG  100
<210> 9244
<211> 100
<212> DNA
<213> Escherichia coli K-12 MG1655
<220> dmsa b0895
<222> 2148-2247
<223> dmsa b0895 U00096 940269..942626
<313> 9244: 2180-2199
<400> 9244
TAACGATCGT GGTGAGGTTC ATATTGAGGC GAAAGTGACG CCACGAATGA  50
TGCCGGGTGT GGTCGCACTG GGTGAAGGTG CCTGGTATGA CCCGGATGCA  100
<210> 9245
<211> 100
<212> DNA
<213> Escherichia coli K-12 MG1655
<220> dmsb b0896
<222> 519-618
<223> dmsb b0896 U00096 942637..943254
<313> 9245: 583-602
<400> 9245
AGCTCACTTT ACCAAACCGA ATATTGTGAT CAAACCCAAT GCCAATAGCC  50
GCCCGACCGG GGATACCACT GGCTATCTGG CAAACCCGAA GGAGGTGTGA  100
<210> 9246
<211> 100
<212> DNA
<213> Escherichia coli K-12 MG1655
<220> dmsb b0896
<222> 519-618
<223> dmsb b0896 U00096 942637..943254
<313> 9246: 550-569
<400> 9246
AGCTCACTTT ACCAAACCGA ATATTGTGAT CAAACCCAAT GCCAATAGCC  50
GCCCGACCGG GGATACCACT GGCTATCTGG CAAACCCGAA GGAGGTGTGA  100
<210> 9247
<211> 100
<212> DNA
<213> Escherichia coli K-12 MG1655
<220> dmsb b0896
<222> 442-541
<223> dmsb b0896 U00096 942637..943254
<313> 9247: 476-495
<400> 9247
CGGGCGCTGG ATTTCGGCCC TATCGACGAG CTGCGTAAAA AACATGGCGA  50
TCTGGCGGCC GTTGCGCCGT TGCCGCGAGC TCACTTTACC AAACCGAATA  100
<210> 9248
<211> 100
<212> DNA
<213> Escherichia coli K-12 MG1655
<220> yedd b1929
<222> 315-414
<223> yedd b1929 U00096 complement(2005700..2006113)
<313> 9248: 392-411
<400> 9248
CATTGAGTAC GATGGTATGA CGATGGAGCG TGTGGATCGC CCGACAGCAG  50
AGTGTGCCGC CGCGCTGGAT AAAGCGCCGT TACCGACGCC ACTGCCGTAA  100
<210> 9249
<211> 100
<212> DNA
<213> Escherichia coli K-12 MG1655
<220> yedd b1929
<222> 314-413
<223> yedd b1929 U00096 complement(2005700..2006113)
<313> 9249: 351-370
<400> 9249
CCATTGAGTA CGATGGTATG ACGATGGAGC GTGTGGATCG CCCGACAGCA  50
GAGTGTGCCG CCGCGCTGGA TAAAGCGCCG TTACCGACGC CACTGCCGTA  100

<210> 9250
<211> 100
<212> DNA
<213> Escherichia coli K-12 MG1655
<220> yedd b1929
<222> 261-360
<223> yedd b1929 U00096 complement(2005700..2006113)
<313> 9250: 307-326
<400> 9250
CACTAACGTT ACCGTAAAAC GTGAGCTGTA CGAGGTTGAG CGTGACGGTA 50
ACACCATTGA GTACGATGGT ATGACGATGG AGCGTGTGGA TCGCCCGACA 100
<210> 9251
<211> 100
<212> DNA
<213> Escherichia coli K-12 MG1655
<220> dmsc b0897
<222> 749-848
<223> dmsc b0897 U00096 943256..944119
<313> 9251: 785-804
<400> 9251
AGCCTGCGGT TCCGCTACTT TCTGTATCAT TCATTCTGCT TCTGGCAGGG 50
GAATTAATCG GTCGCGGCGT ATTCTATGGT TTGCATATGA CCGTGGGGAT 100
<210> 9252
<211> 100
<212> DNA
<213> Escherichia coli K-12 MG1655
<220> dmsc b0897
<222> 718-817
<223> dmsc b0897 U00096 943256..944119
<313> 9252: 750-769
<400> 9252
TTGTGGATTG CACCACAGCT AAAAGGTTAT CAGCCTGCGG TTCCGCTACT 50
TTCTGTATCA TTCATTCTGC TTCTGGCAGG GGAATTAATC GGTCGCGGCG 100
<210> 9253
<211> 100
<212> DNA
<213> Escherichia coli K-12 MG1655
<220> dmsc b0897
<222> 665-764
<223> dmsc b0897 U00096 943256..944119
<313> 9253: 700-719
<400> 9253
ACTATGGTGC GCTGATGTCC TGGCGGATCG TGCTTTTGGC CGTTGCCCTG 50
TGCTTGTGGA TTGCACCACA GCTAAAAGGT TATCAGCCTG CGGTTCCGCT 100
<210> 9254
<211> 100
<212> DNA
<213> Escherichia coli K-12 MG1655
<220> yede b1930
<222> 1083-1182
<223> yede b1930 U00096 2006300..2007505
<313> 9254: 1110-1129
<400> 9254
TGGTCCGATG GGTGGCCTGC TGGTGACATA TTTGCTGTTG TTTGCTGCGC 50
TGATGTTGAT TATCGGCTGG GAAAAACGCT TCTTCCGCCG TGCGGCACCG 100
<210> 9255
<211> 100
<212> DNA
<213> Escherichia coli K-12 MG1655
<220> yede b1930
<222> 1039-1138
<223> yede b1930 U00096 2006300..2007505
<313> 9255: 1070-1089
<400> 9255
GCGCTGGCCA CCGACTGGGA CAAAATCAAC CTGCTGAAAA CCTTTGGTCC 50
GATGGGTGGC CTGCTGGTGA CATATTTGCT GTTGTTTGCT GCGCTGATGT 100
<210> 9256
<211> 100
<212> DNA
<213> Escherichia coli K-12 MG1655
<220> yede b1930

<222> 955-1054
<223> yede b1930 U00096 2006300..2007505
<313> 9256: 1013-1032
<400> 9256
GGCCAGGTGC ACTACTGGTG GGTCGGTCTG GGCAATGTGA TCGGCTCAAC  50
GATTCTGGCG TATTACTGGG ATGATTTCGC TCCGGCGCTG GCCACCGACT  100
<210> 9257
<211> 100
<212> DNA
<213> Escherichia coli K-12 MG1655
<220> erfk b1991
<222> 834-933
<223> erfk b1991 U00096 complement(2060413..2061345)
<313> 9257: 907-926
<400> 9257
CAACGGGCAA GAAGTTGATG TAAATCGCGC AAATGCTGCG TTGCAACGTC  50
GATCGGGAAT GCCTGTGCAA ATTAGTTCTG GTTCAAGACA GATGTTTTAA  100
<210> 9258
<211> 100
<212> DNA
<213> Escherichia coli K-12 MG1655
<220> erfk b1991
<222> 803-902
<223> erfk b1991 U00096 complement(2060413..2061345)
<313> 9258: 846-865
<400> 9258
TGCCGGTAAC CCCATCTTTG CGGGCGTTTA TCAACGGGCA AGAAGTTGAT  50
GTAAATCGCG CAAATGCTGC GTTGCAACGT CGATCGGGAA TGCCTGTGCA  100
<210> 9259
<211> 100
<212> DNA
<213> Escherichia coli K-12 MG1655
<220> erfk b1991
<222> 732-831
<223> erfk b1991 U00096 complement(2060413..2061345)
<313> 9259: 782-801
<400> 9259
GAACTGGCTG GAAGTTCATG AGCCATTGTC GCGCAATCGT GCAGAATATG  50
AGTCTGACCG AAAAGTGCCA TTGCCGGTAA CCCCATCTTT GCGGGCGTTT  100
<210> 9260
<211> 100
<212> DNA
<213> Escherichia coli K-12 MG1655
<220> yedf b1931
<222> 135-234
<223> yedf b1931 U00096 2007502..2007735
<313> 9260: 212-231
<400> 9260
GTCGATCAAT AATATTCCAC TGGATGCGCG TAATCACGGC TATACGGTGC  50
TGGATATTCA GCAAGACGGG CCGACCATTC GTTATTTAAT TCAGAAGTAA  100
<210> 9261
<211> 100
<212> DNA
<213> Escherichia coli K-12 MG1655
<220> yedf b1931
<222> 55-154
<223> yedf b1931 U00096 2007502..2007735
<313> 9261: 101-120
<400> 9261
TATCCGGCAG TCGCAACCCT TGAGGCGATG CCGCAGTTAA AAAAAGGGGA  50
AATCCTGGAA GTGGTGAGCG ACTGTCCGCA GTCGATCAAT AATATTCCAC  100
<210> 9262
<211> 100
<212> DNA
<213> Escherichia coli K-12 MG1655
<220> yedf b1931
<222> 25-124
<223> yedf b1931 U00096 2007502..2007735
<313> 9262: 42-61
<400> 9262
CGCCTTGATA TGGTGGGTGA ACCCTGCCCT TATCCGGCAG TCGCAACCCT  50

TGAGGCGATG CCGCAGTTAA AAAAAGGGGA AATCCTGGAA GTGGTGAGCG 100
<210> 9263
<211> 100
<212> DNA
<213> Escherichia coli K-12 MG1655
<220> ppia b3364
<222> 457-556
<223> ppia b3364 U00096 complement(3489362..3489934)
<313> 9263: 501-520
<400> 9263
GTGAAAGGCA TGGACGTTGC CGATAAGATT TCCCAGGTGC CGACTCATGA 50
CGTTGGTCCG TACCAGAATG TGCCGTCAAA ACCGGTAGTT ATCCTTTCCG 100
<210> 9264
<211> 100
<212> DNA
<213> Escherichia coli K-12 MG1655
<220> ppia b3364
<222> 424-523
<223> ppia b3364 U00096 complement(3489362..3489934)
<313> 9264: 474-493
<400> 9264
CGTGATTTCG GTTACGCGGT ATTTGGTAAA GTGGTGAAAG GCATGGACGT 50
TGCCGATAAG ATTTCCCAGG TGCCGACTCA TGACGTTGGT CCGTACCAGA 100
<210> 9265
<211> 100
<212> DNA
<213> Escherichia coli K-12 MG1655
<220> ppia b3364
<222> 314-413
<223> ppia b3364 U00096 complement(3489362..3489934)
<313> 9265: 371-390
<400> 9265
TGCGCAACAC GCGTGGCACC ATCGCGATGG CACGTACCGC TGACAAAGAC 50
AGCGCCACCA GCCAGTTCTT TATCAACGTT GCCGATAACG CCTTCCTTGA 100
<210> 9266
<211> 100
<212> DNA
<213> Escherichia coli K-12 MG1655
<220> ppib b0526
<222> 396-495
<223> ppib b0526 U00096 complement(553166..553660)
<313> 9266: 458-477
<400> 9266
GGTAGACAAA ATCAAAGGTG TTGCAACCGG TCGTAGCGGT ATGCACCAGG 50
ACGTGCCAAA AGAAGACGTT ATCATTGAAA GCGTGACCGT TAGCGAGTAA 100
<210> 9267
<211> 100
<212> DNA
<213> Escherichia coli K-12 MG1655
<220> ppib b0526
<222> 350-449
<223> ppib b0526 U00096 complement(553166..553660)
<313> 9267: 395-414
<400> 9267
GTTGGGGCTA CTGCGTGTTT GCTGAAGTGG TTGACGGCAT GGACGTGGTA 50
GACAAAATCA AAGGTGTTGC AACCGGTCGT AGCGGTATGC ACCAGGACGT 100
<210> 9268
<211> 100
<212> DNA
<213> Escherichia coli K-12 MG1655
<220> ppib b0526
<222> 308-407
<223> ppib b0526 U00096 complement(553166..553660)
<313> 9268: 344-363
<400> 9268
TTGATAACGA CTTCCTGAAC TTCTCTGGCG AAAGCCTGCA AGGTTGGGGC 50
TACTGCGTGT TTGCTGAAGT GGTTGACGGC ATGGACGTGG TAGACAAAAT 100
<210> 9269
<211> 100
<212> DNA
<213> Escherichia coli K-12 MG1655

<220> yedi b1959
<222> 819-918
<223> yedi b1959 U00096 complement(2026471..2027388)
<313> 9269: 897-916
<400> 9269
ATTACCGACT GTTTTAAATC TGATTCTTGG ATTTATCATC GGCGGCATCG  50
TGGTGCTGGG AGTGAAAGCC GTAGAGAAAA TGCGCGGTCA GGCACATTAA  100
<210> 9270
<211> 100
<212> DNA
<213> Escherichia coli K-12 MG1655
<220> yedi b1959
<222> 777-876
<223> yedi b1959 U00096 complement(2026471..2027388)
<313> 9270: 806-825
<400> 9270
TGAACATTTC GCCGGGCAGC AAAGTGCAGT AGTGGCGATG ATATTACCGA  50
CTGTTTTAAA TCTGATTCTT GGATTTATCA TCGGCGGCAT CGTGGTGCTG  100
<210> 9271
<211> 100
<212> DNA
<213> Escherichia coli K-12 MG1655
<220> yedi b1959
<222> 737-836
<223> yedi b1959 U00096 complement(2026471..2027388)
<313> 9271: 765-784
<400> 9271
GGATTGTGGT ACATGGAATT GCGCCGCTGC ATCACGCCAT TGAACATTTC  50
GCCGGGCAGC AAAGTGCAGT AGTGGCGATG ATATTACCGA CTGTTTTAAA  100
<210> 9272
<211> 100
<212> DNA
<213> Escherichia coli K-12 MG1655
<220> ppic ppiC
<222> 183-282
<223> ppic ppiC U00096 complement(3957150..3957431)
<313> 9272: 251-270
<400> 9272
GTTCGATAAA GTGGTTTTCT CTTGTCCGGT ACTGGAGCCG ACCGGCCCGC  50
TGCACACCCA GTTCGGATAT CACATCATTA AGGTGCTGTA CCGCAACTAA  100
<210> 9273
<211> 100
<212> DNA
<213> Escherichia coli K-12 MG1655
<220> ppic ppiC
<222> 149-248
<223> ppic ppiC U00096 complement(3957150..3957431)
<313> 9273: 190-209
<400> 9273
TAGGTGAATT CCGCCAGGGT CAGATGGTTC CGGCGTTCGA TAAAGTGGTT  50
TTCTCTTGTC CGGTACTGGA GCCGACCGGC CCGCTGCACA CCCAGTTCGG  100
<210> 9274
<211> 100
<212> DNA
<213> Escherichia coli K-12 MG1655
<220> ppic ppiC
<222> 106-205
<223> ppic ppiC U00096 complement(3957150..3957431)
<313> 9274: 139-158
<400> 9274
AAGAAACACT CCATTTGCCC ATCAGGCAAA CGCGGCGGTG ATTTAGGTGA  50
ATTCCGCCAG GGTCAGATGG TTCCGGCGTT CGATAAAGTG GTTTTCTCTT  100
<210> 9275
<211> 100
<212> DNA
<213> Escherichia coli K-12 MG1655
<220> yedj yedJ
<222> 595-694
<223> yedj yedJ U00096 complement(2030406..2031101)
<313> 9275: 654-673
<400> 9275

CATTTTTTAG TCGAGTTTAT GGCGAAGCTC AGTGCCGAAC TGGCGGGGGA 50
GAATGAAGGT GTCGATCACA AGGTGATAGA TGCGTTTTCA TCCGCTGGCT 100
<210> 9276
<211> 100
<212> DNA
<213> Escherichia coli K-12 MG1655
<220> yedj yedJ
<222> 549-648
<223> yedj yedJ U00096 complement(2030406..2031101)
<313> 9276: 609-628
<400> 9276
AACCATGCAA ACCGCAAGAG GCAAGCAGCT GGCGCAGCAC AACGCGCATT 50
TTTTAGTCGA GTTTATGGCG AAGCTCAGTG CCGAACTGGC GGGGGAGAAT 100
<210> 9277
<211> 100
<212> DNA
<213> Escherichia coli K-12 MG1655
<220> yedj yedJ
<222> 511-610
<223> yedj yedJ U00096 complement(2030406..2031101)
<313> 9277: 555-574
<400> 9277
CTGGATCATT TCCAGACTAA GTTGCTGAAG CTACCGCAAA CCATGCAAAC 50
CGCAAGAGGC AAGCAGCTGG CGCAGCACAA CGCGCATTTT TTAGTCGAGT 100
<210> 9278
<211> 100
<212> DNA
<213> Escherichia coli K-12 MG1655
<220> b1820 b1820
<222> 358-457
<223> b1820 b1820 U00096 1902825..1903283
<313> 9278: 399-418
<400> 9278
GGCGTGTTGG TGATGCAATT AGAACAGCGT CGGCTGTTAA TCCGCGTTCG 50
AAATATCGAC GATCTGGAAA AAATTTATAA GCTTCTCGTT TCAACTCAAT 100
<210> 9279
<211> 100
<212> DNA
<213> Escherichia coli K-12 MG1655
<220> b1820 b1820
<222> 296-395
<223> b1820 b1820 U00096 1902825..1903283
<313> 9279: 355-374
<400> 9279
TTTTCTTCGC CAATGTCTGG ATTGAATATA GCCGAATCAA AGCGATGAAC 50
TTGTCGGAAG ATGGCGTGTT GGTGATGCAA TTAGAACAGC GTCGGCTGTT 100
<210> 9280
<211> 100
<212> DNA
<213> Escherichia coli K-12 MG1655
<220> b1820 b1820
<222> 269-368
<223> b1820 b1820 U00096 1902825..1903283
<313> 9280: 322-341
<400> 9280
CGAAGATCAT CTTTAAACAA AAAGGTTTTT TCTTCGCCAA TGTCTGGATT 50
GAATATAGCC GAATCAAAGC GATGAACTTG TCGGAAGATG GCGTGTTGGT 100
<210> 9281
<211> 100
<212> DNA
<213> Escherichia coli K-12 MG1655
<220> yedk b1932
<222> 536-635
<223> yedk b1932 U00096 2007844..2008497
<313> 9281: 577-596
<400> 9281
AGATTAGCGG TAAAGAAGCC TCAGAAATCG CGGCCAGTGG CTGTGTTCCA 50
GCAAACCAGT TCTCCTGGCA CCCGGTATCG CGCGCGGTGG GTAATGTTAA 100
<210> 9282
<211> 100
<212> DNA

<213> Escherichia coli K-12 MG1655
<220> yedk b1932
<222> 502-601
<223> yedk b1932 U00096 2007844..2008497
<313> 9282: 542-561
<400> 9282
TCGCCAGAAG CTGCGCGGGA ATGGATGCGG CAAGAGATTA GCGGTAAAGA 50
AGCCTCAGAA ATCGCGGCCA GTGGCTGTGT TCCAGCAAAC CAGTTCTCCT 100
<210> 9283
<211> 100
<212> DNA
<213> Escherichia coli K-12 MG1655
<220> yedk b1932
<222> 465-564
<223> yedk b1932 U00096 2007844..2008497
<313> 9283: 487-506
<400> 9283
TCTGGTAGAT ATTCATGACC GCCGCCCACT GGTACTGTCG CCAGAAGCTG 50
CGCGGGAATG GATGCGGCAA GAGATTAGCG GTAAAGAAGC CTCAGAAATC 100
<210> 9284
<211> 100
<212> DNA
<213> Escherichia coli K-12 MG1655
<220> b1821 b1822
<222> 521-620
<223> b1821 b1822 U00096 1903658..1904278
<313> 9284: 550-569
<400> 9284
GCTTTATCGG CTCAATTATT GGGAAAAAAG CGGAAATTCT CGGCGGGCTG 50
GTGCTGATCG GCATCGGCGT CCAGATCCTC TGGACGCACT TCCACGGTTA 100
<210> 9285
<211> 100
<212> DNA
<213> Escherichia coli K-12 MG1655
<220> b1821 b1822
<222> 449-548
<223> b1821 b1822 U00096 1903658..1904278
<313> 9285: 516-535
<400> 9285
ACATTATCGC GACCGCATTG GCCATTGGTT GTGCAACCTT GATTATGTCA 50
ACATTAGGGA TGATGGTTGG TCGCTTTATC GGCTCAATTA TTGGGAAAAA 100
<210> 9286
<211> 100
<212> DNA
<213> Escherichia coli K-12 MG1655
<220> b1821 b1822
<222> 208-307
<223> b1821 b1822 U00096 1903658..1904278
<313> 9286: 272-291
<400> 9286
CTGATCGGCT GGGGAATGGG CATGTTAGCC AGCCGGTTTG TCCTTGAATG 50
GAACCACTGG ATTGCGTTTG TGCTGCTGAT ATTCCTCGGC GGGCGAATGA 100
<210> 9287
<211> 100
<212> DNA
<213> Escherichia coli K-12 MG1655
<220> yedl yedL
<222> 381-480
<223> yedl yedL U00096 2008622..2009101
<313> 9287: 407-426
<400> 9287
GCATGGTTTC GCTGATTGCG AACCGTTTGC TGATTATCGA CTTGATCCAC 50
ACAGTCGATT TTTATCATTG ACGCTATGCG AAAATAATGA ATTGCCATGA 100
<210> 9288
<211> 100
<212> DNA
<213> Escherichia coli K-12 MG1655
<220> yedl yedL
<222> 335-434
<223> yedl yedL U00096 2008622..2009101
<313> 9288: 368-387

<400> 9288
CGGGTACACA GGCTGGATTT ACGGCCTGCC ATCAACTTTA TTTGAAGCAT 50
GGTTTCGCTG ATTGCGAACC GTTTGCTGAT TATCGACTTG ATCCACACAG 100
<210> 9289
<211> 100
<212> DNA
<213> Escherichia coli K-12 MG1655
<220> yedl yedL
<222> 245-344
<223> yedl yedL U00096 2008622..2009101
<313> 9289: 295-314
<400> 9289
ATTATTTACG TCGTGGTGTC GCCAGTCTGA TTTTACGCCA CATTTTGCAG 50
GTCGCCCAGG ACAGATGCCT TCATCGCCTG AGCTTAGAAA CGGGTACACA 100
<210> 9290
<211> 100
<212> DNA
<213> Escherichia coli K-12 MG1655
<220> yedm yedM
<222> 252-351
<223> yedm yedM U00096 complement(2010023..2010373)
<313> 9290: 316-335
<400> 9290
TAGTCTCTGG TTTCAAGATT TCTCGATTTA TAATAAGATA GGTTTTATCA 50
TGATAGAAAA AATCAGGGAA CTGGTAACCC ACCCATTCGG CATAAATTAG 100
<210> 9291
<211> 100
<212> DNA
<213> Escherichia coli K-12 MG1655
<220> yedm yedM
<222> 252-351
<223> yedm yedM U00096 complement(2010023..2010373)
<313> 9291: 312-331
<400> 9291
TAGTCTCTGG TTTCAAGATT TCTCGATTTA TAATAAGATA GGTTTTATCA 50
TGATAGAAAA AATCAGGGAA CTGGTAACCC ACCCATTCGG CATAAATTAG 100
<210> 9292
<211> 100
<212> DNA
<213> Escherichia coli K-12 MG1655
<220> yedm yedM
<222> 88-187
<223> yedm yedM U00096 complement(2010023..2010373)
<313> 9292: 133-152
<400> 9292
CATGAGTTAG TATCTGATGA TGCAATCATA AATGAATTAT CAAGTGAGTT 50
AGTCAGTTGT CTTGGAAATG GTAAGTTTAC ACCAATTAGT GAAGACAGCA 100
<210> 9293
<211> 100
<212> DNA
<213> Escherichia coli K-12 MG1655
<220> yedn b1935
<222> 210-309
<223> yedn b1935 U00096 complement(2009571..2009891)
<313> 9293: 272-291
<400> 9293
AGTATCCGAT GGAATTACCC AGGTCTCCGT AGATAATAAT CTGATATCGC 50
GTGTTGACTC ATCTCCGTCA AAGGCTATGA CAATTTCTAT TTATCGCAAT 100
<210> 9294
<211> 100
<212> DNA
<213> Escherichia coli K-12 MG1655
<220> yedn b1935
<222> 65-164
<223> yedn b1935 U00096 complement(2009571..2009891)
<313> 9294: 126-145
<400> 9294
CAGATCTTCA CTTGGGGTCT TTGCCACCTC TTCCATCCTG GATTGAGGTG 50
CTTTATCTTC GTCATAATGG CCTTGCCACA ATACAAATTC CCAAATTCTG 100
<210> 9295
<211> 100

<212> DNA
<213> Escherichia coli K-12 MG1655
<220> yedn b1935
<222> 54-153
<223> yedn b1935 U00096 complement(2009571..2009891)
<313> 9295: 74-93
<400> 9295
ACTTGACTTA ACAGATCTTC ACTTGGGGTC TTTGCCACCT CTTCCATCCT 50
GGATTGAGGT GCTTTATCTT CGTCATAATG GCCTTGCCAC AATACAAATT 100
<210> 9296
<211> 100
<212> DNA
<213> Escherichia coli K-12 MG1655
<220> b1824 b1824
<222> 40-139
<223> b1824 b1824 U00096 complement(1905472..1905615)
<313> 9296: 96-115
<400> 9296
GTTGACGTTG AATACCGCTT CTCTGCCGAG CCTTATATTG GTGCCTCATG 50
CAGTAATGTG TCAGTTTTAT CTATGTTATG CCTGCGGGCG AAGAAAACAA 100
<210> 9297
<211> 100
<212> DNA
<213> Escherichia coli K-12 MG1655
<220> b1824 b1824
<222> 45-144
<223> b1824 b1824 U00096 complement(1905472..1905615)
<313> 9297: 77-96
<400> 9297
CGTTGAATAC CGCTTCTCTG CCGAGCCTTA TATTGGTGCC TCATGCAGTA 50
ATGTGTCAGT TTTATCTATG TTATGCCTGC GGGCGAAGAA AACAATCTAA 100
<210> 9298
<211> 100
<212> DNA
<213> Escherichia coli K-12 MG1655
<220> b1824 b1824
<222> 34-133
<223> b1824 b1824 U00096 complement(1905472..1905615)
<313> 9298: 59-78
<400> 9298
AAACACGTTG ACGTTGAATA CCGCTTCTCT GCCGAGCCTT ATATTGGTGC 50
CTCATGCAGT AATGTGTCAG TTTTATCTAT GTTATGCCTG CGGGCGAAGA 100
<210> 9299
<211> 100
<212> DNA
<213> Escherichia coli K-12 MG1655
<220> yedo b1920
<222> 984-1083
<223> yedo b1920 U00096 complement(1996517..1997599)
<313> 9299: 1013-1032
<400> 9299
TGACGGTATC AGTCAGAAAC GCTTCAAAGA TGAAGGGCCG ATTCTGTTTA 50
TTCATACCGG CGGTGCGCCT GCGCTGTTCG CCTATCATCC CCACGTTTAG 100
<210> 9300
<211> 100
<212> DNA
<213> Escherichia coli K-12 MG1655
<220> yedo b1920
<222> 906-1005
<223> yedo b1920 U00096 complement(1996517..1997599)
<313> 9300: 923-942
<400> 9300
AGCAGTGAAA TTGCTGGCGC GGCTGGAAGG CATTCTGCTT GATCCTGTGT 50
ATACCGGAAA AGCGATGGCG GGGCTGATTG ACGGTATCAG TCAGAAACGC 100
<210> 9301
<211> 100
<212> DNA
<213> Escherichia coli K-12 MG1655
<220> yedo b1920
<222> 823-922
<223> yedo b1920 U00096 complement(1996517..1997599)

<313> 9301: 849-868
<400> 9301
GAGCTGACCG CATCAGCGGA AATTTTACTC TGGGATGACT ATTTTGCACC 50
TGGCTACGGC GTGCCGAACG ACGAAGGCAT GGAAGCAGTG AAATTGCTGG 100
<210> 9302
<211> 100
<212> DNA
<213> Escherichia coli K-12 MG1655
<220> b1825 b1825
<222> 151-250
<223> b1825 b1825 U00096 complement(1906285..1906572)
<313> 9302: 204-223
<400> 9302
GATCAGCAAA AACGTCAAAA TGCACTGTTA CGTCGTTTGT GCGAAGCAAA 50
CGAACCAGAG AAAGCAGATA AAAAGACCGT TGAGAGTCAA AAATCGGTTG 100
<210> 9303
<211> 100
<212> DNA
<213> Escherichia coli K-12 MG1655
<220> b1825 b1825
<222> 107-206
<223> b1825 b1825 U00096 complement(1906285..1906572)
<313> 9303: 163-182
<400> 9303
GTTCACGGAC TAACGAACAG ATTGAACTGC TTGAGGCGTT GCTGGATCAG 50
CAAAAACGTC AAAATGCACT GTTACGTCGT TTGTGCGAAG CAAACGAACC 100
<210> 9304
<211> 100
<212> DNA
<213> Escherichia coli K-12 MG1655
<220> b1825 b1825
<222> 65-164
<223> b1825 b1825 U00096 complement(1906285..1906572)
<313> 9304: 112-131
<400> 9304
TTCTTATCGG GCTCATCTTG TGGTTTTTTA TCAATCGTGC CAGTTCACGG 50
ACTAACGAAC AGATTGAACT GCTTGAGGCG TTGCTGGATC AGCAAAAACG 100
<210> 9305
<211> 100
<212> DNA
<213> Escherichia coli K-12 MG1655
<220> b1826 b1826
<222> 45-144
<223> b1826 b1826 U00096 complement(1906647..1906790)
<313> 9305: 116-135
<400> 9305
TTGCTTGCTG CTTTGGGCGC AGGTATTCAA CATGATGTGC GATCAGGATG 50
TACAATTTTT CAGCGGAATT TGTGCCATTA ACCAGTTTAT CCCGTGGTGA 100
<210> 9306
<211> 100
<212> DNA
<213> Escherichia coli K-12 MG1655
<220> b1826 b1826
<222> 39-138
<223> b1826 b1826 U00096 complement(1906647..1906790)
<313> 9306: 70-89
<400> 9306
GTTGGCTTGC TTGCTGCTTT GGGCGCAGGT ATTCAACATG ATGTGCGATC 50
AGGATGTACA ATTTTTCAGC GGAATTTGTG CCATTAACCA GTTTATCCCG 100
<210> 9307
<211> 100
<212> DNA
<213> Escherichia coli K-12 MG1655
<220> b1826 b1826
<222> 1-100
<223> b1826 b1826 U00096 complement(1906647..1906790)
<313> 9307: 31-50
<400> 9307
GTGAAAAAGT TTCGATGGGT CGTTCTGGTT GTCGTGGTGT TGGCTTGCTT 50
GCTGCTTTGG GCGCAGGTAT TCAACATGAT GTGCGATCAG GATGTACAAT 100
<210> 9308

<211> 100
<212> DNA
<213> Escherichia coli K-12 MG1655
<220> ytfa b4206
<222> 218-317
<223> ytfa b4206 U00096 4425347..4425673
<313> 9308: 264-283
<400> 9308
ATCACATCAG TATGGTTTCA CCCCAGGCAC TGGAAGCCAG ACGTAAGGTC 50
ATCAAAGAGA CAATCCTTAG CTGGCTTTTG GTTGACCCTT CATCTACCGC 100
<210> 9309
<211> 100
<212> DNA
<213> Escherichia coli K-12 MG1655
<220> ytfa b4206
<222> 212-311
<223> ytfa b4206 U00096 4425347..4425673
<313> 9309: 243-262
<400> 9309
TGGTTTATCA CATCAGTATG GTTTCACCCC AGGCACTGGA AGCCAGACGT 50
AAGGTCATCA AAGAGACAAT CCTTAGCTGG CTTTTGGTTG ACCCTTCATC 100
<210> 9310
<211> 100
<212> DNA
<213> Escherichia coli K-12 MG1655
<220> ytfa b4206
<222> 165-264
<223> ytfa b4206 U00096 4425347..4425673
<313> 9310: 227-246
<400> 9310
TGCTGCGCTT GGCGGATATT ATTTGATCAA CCAGCATACG CTTGGCCTGG 50
TTTATCACAT CAGTATGGTT TCACCCCAGG CACTGGAAGC CAGACGTAAG 100
<210> 9311
<211> 100
<212> DNA
<213> Escherichia coli K-12 MG1655
<220> b1827 b1827
<222> 659-758
<223> b1827 b1827 U00096 complement(1907332..1908123)
<313> 9311: 695-714
<400> 9311
TCATTGCCGG TTTGAGCATC TCCTTCCCGA CGTTGCGTTT CTCTGAAGAG 50
CGTTTACAGG AATATGTCGC AATGTTGCAT ACCGCAGCGC GCAAAATTTC 100
<210> 9312
<211> 100
<212> DNA
<213> Escherichia coli K-12 MG1655
<220> b1827 b1827
<222> 627-726
<223> b1827 b1827 U00096 complement(1907332..1908123)
<313> 9312: 657-676
<400> 9312
TGCGGTACCG GTATTTGATC GCTTTGGCGT GGTCATTGCC GGTTTGAGCA 50
TCTCCTTCCC GACGTTGCGT TTCTCTGAAG AGCGTTTACA GGAATATGTC 100
<210> 9313
<211> 100
<212> DNA
<213> Escherichia coli K-12 MG1655
<220> b1827 b1827
<222> 552-651
<223> b1827 b1827 U00096 complement(1907332..1908123)
<313> 9313: 593-612
<400> 9313
ACCCGTTCTG GACCAGGTGC GCGAGCAGGG GTATGGCGAA GATAATGAAG 50
AGCAGGAAGA AGGGCTGCGA TGCATTGCGG TACCGGTATT TGATCGCTTT 100
<210> 9314
<211> 100
<212> DNA
<213> Escherichia coli K-12 MG1655
<220> ytfb b4207
<222> 576-675

<223> ytfb b4207 U00096 complement(4425657..4426331)
<313> 9314: 639-658
<400> 9314
CGCCAGCGGC GTGGTGACGG GCTTAACCAT TGATACGGGG AATAATCAAC 50
AGGTCCTGTT CACCCGCCAG CCGGATGGCA GTTTTATTCG TGCGCGGTAG 100
<210> 9315
<211> 100
<212> DNA
<213> Escherichia coli K-12 MG1655
<220> ytfb b4207
<222> 553-652
<223> ytfb b4207 U00096 complement(4425657..4426331)
<313> 9315: 608-627
<400> 9315
CAGATGGTGA AAATCCGCCA GAACGCCAGC GGCGTGGTGA CGGGCTTAAC 50
CATTGATACG GGGAATAATC AACAGGTCCT GTTCACCCGC CAGCCGGATG 100
<210> 9316
<211> 100
<212> DNA
<213> Escherichia coli K-12 MG1655
<220> ytfb b4207
<222> 502-601
<223> ytfb b4207 U00096 complement(4425657..4426331)
<313> 9316: 567-586
<400> 9316
ATGGCGCAGG TAGAAGGCGC GGGTAAGCCG CTGAGTAATT TGCAAAACGG 50
GCAGATGGTG AAAATCCGCC AGAACGCCAG CGGCGTGGTG ACGGGCTTAA 100
<210> 9317
<211> 100
<212> DNA
<213> Escherichia coli K-12 MG1655
<220> b1828 b1829
<222> 1386-1485
<223> b1828 b1829 U00096 1908189..1909673
<313> 9317: 1462-1481
<400> 9317
TAATGGTACA CACGTCTCGC TGATGGCTGC GGCTATTCTG GCAGTGATTG 50
CTGCCTGTGT CAGTGGTTTA CGTATCACTC AGCCACGATC CAGGGCATAA 100
<210> 9318
<211> 100
<212> DNA
<213> Escherichia coli K-12 MG1655
<220> b1828 b1829
<222> 1359-1458
<223> b1828 b1829 U00096 1908189..1909673
<313> 9318: 1386-1405
<400> 9318
GGCGCTGATG CTAAATCAGT TTGGAGATAA TGGTACACAC GTCTCGCTGA 50
TGGCTGCGGC TATTCTGGCA GTGATTGCTG CCTGTGTCAG TGGTTTACGT 100
<210> 9319
<211> 100
<212> DNA
<213> Escherichia coli K-12 MG1655
<220> b1828 b1829
<222> 1301-1400
<223> b1828 b1829 U00096 1908189..1909673
<313> 9319: 1330-1349
<400> 9319
CCAGTGGCAT GTTAGGAACG GCTCGTCTAC TGGGTCAGAG TAGCGGCGCG 50
GCGCTGGTGG CGCTGATGCT AAATCAGTTT GGAGATAATG GTACACACGT 100
<210> 9320
<211> 100
<212> DNA
<213> Escherichia coli K-12 MG1655
<220> yedu b1968
<222> 747-846
<223> yedu b1968 U00096 2033857..2034708
<313> 9320: 774-793
<400> 9320
GCGAGTACAT AAGGACCGTA AACTTCTCAC CGGCGACAGT CCTTTTGCAG 50
CGAATGCGTT GGGTAAACTG GCGGCGCAGG AAATGCTGGC AGCTTACGCG 100

<210> 9321
<211> 100
<212> DNA
<213> Escherichia coli K-12 MG1655
<220> yedu b1968
<222> 667-766
<223> yedu b1968 U00096 2033857..2034708
<313> 9321: 698-717
<400> 9321
ATGCCGGGTC ATCTCACCTG GTACTTCGGC GAAGAACTGA AGAAAATGGG 50
CATGAATATC ATTAATGACG ACATCACCGG GCGAGTACAT AAGGACCGTA 100
<210> 9322
<211> 100
<212> DNA
<213> Escherichia coli K-12 MG1655
<220> yedu b1968
<222> 629-728
<223> yedu b1968 U00096 2033857..2034708
<313> 9322: 660-679
<400> 9322
CAGACGCCGC AGACAAACAA ACGCCAGAGA TTGGCTATAT GCCGGGTCAT 50
CTCACCTGGT ACTTCGGCGA AGAACTGAAG AAAATGGGCA TGAATATCAT 100
<210> 9323
<211> 100
<212> DNA
<213> Escherichia coli K-12 MG1655
<220> ytfe b4210
<222> 564-663
<223> ytfe b4210 U00096 complement(4428899..4429561)
<313> 9323: 609-628
<400> 9323
AGCGATGTAT AACGGCATTA ATGAACTGAT TGATGACCTG ATGGATCACA 50
TCAGTCTGGA AAACAATGTA CTGTTCCCAC GCGCGCTGGC GGGTGAGTGA 100
<210> 9324
<211> 100
<212> DNA
<213> Escherichia coli K-12 MG1655
<220> ytfe b4210
<222> 502-601
<223> ytfe b4210 U00096 complement(4428899..4429561)
<313> 9324: 549-568
<400> 9324
GAAGTGATTA AACACACCAC CAATAACGTC ACACCGCCGC CAGAAGCCTG 50
CACCACCTGG AAAGCGATGT ATAACGGCAT TAATGAACTG ATTGATGACC 100
<210> 9325
<211> 100
<212> DNA
<213> Escherichia coli K-12 MG1655
<220> ytfe b4210
<222> 488-587
<223> ytfe b4210 U00096 complement(4428899..4429561)
<313> 9325: 512-531
<400> 9325
CGGGCGAACT GCTGGAAGTG ATTAAACACA CCACCAATAA CGTCACACCG 50
CCGCCAGAAG CCTGCACCAC CTGGAAAGCG ATGTATAACG GCATTAATGA 100
<210> 9326
<211> 100
<212> DNA
<213> Escherichia coli K-12 MG1655
<220> yedv b1969
<222> 1259-1358
<223> yedv b1969 U00096 complement(2034816..2036174)
<313> 9326: 1296-1315
<400> 9326
CTTTAGTCAA AGCGATTGCC GAATTGCATG GGGGAAGTGC TACGTATCAC 50
TATCTCAATA AGCATAATGT GTTCCGGATT ACGTTACCGC AAAGAAATTA 100
<210> 9327
<211> 100
<212> DNA
<213> Escherichia coli K-12 MG1655
<220> yedv b1969

<222> 1192-1291
<223> yedv b1969 U00096 complement(2034816..2036174)
<313> 9327: 1226-1245
<400> 9327
AAACTCTTCC GTAGATTTTG GCGGGGAGAT AATTCGCGTC ATTCCGTAGG  50
TCAGGGACTA GGCCTTTCTT TAGTCAAAGC GATTGCCGAA TTGCATGGGG  100
<210> 9328
<211> 100
<212> DNA
<213> Escherichia coli K-12 MG1655
<220> yedv b1969
<222> 1144-1243
<223> yedv b1969 U00096 complement(2034816..2036174)
<313> 9328: 1171-1190
<400> 9328
CTTAATATTG ATATCGCCAG CCCTGGAACG AAAATTAATG AGCCTGAAAA  50
ACTCTTCCGT AGATTTTGGC GGGGAGATAA TTCGCGTCAT TCCGTAGGTC  100
<210> 9329
<211> 100
<212> DNA
<213> Escherichia coli K-12 MG1655
<220> ytff b4211
<222> 807-906
<223> ytff b4211 U00096 complement(4429669..4430643)
<313> 9329: 868-887
<400> 9329
GCTGATTGTT TTCGAAACGC TGGCAGGTTT GCTGTACACC TTTTTACTCC  50
GCCAGCAAAT GCCGCCGCTA ATGACGCTGA GCGGTATCGC GCTGTTAGTG  100
<210> 9330
<211> 100
<212> DNA
<213> Escherichia coli K-12 MG1655
<220> ytff b4211
<222> 751-850
<223> ytff b4211 U00096 complement(4429669..4430643)
<313> 9330: 785-804
<400> 9330
GGCGCACTCT GCTGGAACGT CGCCAGCCAG CTATTACCGA CAGTGATTCT  50
CGGGCCGCTG ATTGTTTTCG AAACGCTGGC AGGTTTGCTG TACACCTTTT  100
<210> 9331
<211> 100
<212> DNA
<213> Escherichia coli K-12 MG1655
<220> ytff b4211
<222> 688-787
<223> ytff b4211 U00096 complement(4429669..4430643)
<313> 9331: 718-737
<400> 9331
GGCCCCCGTC CGCTGGTGTT TATTAGTCTG ATGGTTGCGA TAGCCGTGCT  50
TTGCTCATGG GTTGGCGCAC TCTGCTGGAA CGTCGCCAGC CAGCTATTAC  100
<210> 9332
<211> 100
<212> DNA
<213> Escherichia coli K-12 MG1655
<220> yedw yedW
<222> 621-720
<223> yedw yedW U00096 complement(2036174..2036893)
<313> 9332: 701-720
<400> 9332
CATTCGCAGG CTCCGCGCAA AAGTTGATGA TCCTTTTCCT GAAAAGCTAA  50
TTGCCACAAT CCGGGGGATG GGCTATTCAT TCGTAGCGGT AAAAAAATAA  100
<210> 9333
<211> 100
<212> DNA
<213> Escherichia coli K-12 MG1655
<220> yedw yedW
<222> 603-702
<223> yedw yedW U00096 complement(2036174..2036893)
<313> 9333: 640-659
<400> 9333
CAATACGGTG GACGTCGCCA TTCGCAGGCT CCGCGCAAAA GTTGATGATC  50

CTTTTCCTGA AAAGCTAATT GCCACAATCC GGGGGATGGG CTATTCATTC  100
<210> 9334
<211> 100
<212> DNA
<213> Escherichia coli K-12 MG1655
<220> yedw yedW
<222> 525-624
<223> yedw yedW U00096 complement(2036174..2036893)
<313> 9334: 569-588
<400> 9334
GGCCTCCAGA GCTGGCGAAA TTATACCCAG AACGGTTATT GCGAGTGAAA  50
TTTGGGGAAT CAACTTTGAT AGTGATACCA ATACGGTGGA CGTCGCCATT  100
<210> 9335
<211> 100
<212> DNA
<213> Escherichia coli K-12 MG1655
<220> ytfg b4212
<222> 762-861
<223> ytfg b4212 U00096 complement(4430742..4431602)
<313> 9335: 819-838
<400> 9335
CGGTCTGTTT GATGACAGCA AAACACTTAG CAAATTGATT GGCCACCCAA  50
CGACAACGTT AGCCGAAAGC GTAAGCCATC TTTTTAATGT TAATAACTAG  100
<210> 9336
<211> 100
<212> DNA
<213> Escherichia coli K-12 MG1655
<220> ytfg b4212
<222> 731-830
<223> ytfg b4212 U00096 complement(4430742..4431602)
<313> 9336: 766-785
<400> 9336
TGGCGGATTC TGACGTTGGC GCATCGAAAG GCGGTCTGTT TGATGACAGC  50
AAAACACTTA GCAAATTGAT TGGCCACCCA ACGACAACGT TAGCCGAAAG  100
<210> 9337
<211> 100
<212> DNA
<213> Escherichia coli K-12 MG1655
<220> ytfg b4212
<222> 609-708
<223> ytfg b4212 U00096 complement(4430742..4431602)
<313> 9337: 658-677
<400> 9337
ACAGTTAGCG GCAGAGCTGA CCAAACAGAG CGGCAAACAG GTTACCTATC  50
AAAATCTGAG CGAAGCCGAT TTCGCCGCGG CACTGAAAAG CGTCGGACTG  100
<210> 9338
<211> 100
<212> DNA
<213> Escherichia coli K-12 MG1655
<220> ytfh b4213
<222> 372-471
<223> ytfh b4213 U00096 4431601..4432071
<313> 9338: 449-468
<400> 9338
GCTGGGCGAG CAGGTGAGCG AAAAGGTTGC TGCACTGGCG GACTGGATTG  50
AGTTGAATTT GCCCGAGGTG TTGGCGGTGC GGGATGAACG TGCGGCATAA  100
<210> 9339
<211> 100
<212> DNA
<213> Escherichia coli K-12 MG1655
<220> ytfh b4213
<222> 347-446
<223> ytfh b4213 U00096 4431601..4432071
<313> 9339: 392-411
<400> 9339
CGCATGTGGA ATATAGCCTC ACGCCGCTGG GCGAGCAGGT GAGCGAAAAG  50
GTTGCTGCAC TGGCGGACTG GATTGAGTTG AATTTGCCCG AGGTGTTGGC  100
<210> 9340
<211> 100
<212> DNA
<213> Escherichia coli K-12 MG1655

<220> ytfh b4213
<222> 303-402
<223> ytfh b4213 U00096 4431601..4432071
<313> 9340: 352-371
<400> 9340
ACAGGATGGT TTTCTTAACC GTATCGCGTA TCCGGTGGTG CCGCCGCATG 50
TGGAATATAG CCTCACGCCG CTGGGCGAGC AGGTGAGCGA AAAGGTTGCT 100
<210> 9341
<211> 100
<212> DNA
<213> Escherichia coli K-12 MG1655
<220> ytfi b4216
<222> 401-500
<223> ytfi b4216 U00096 4435285..4435785
<313> 9341: 461-480
<400> 9341
CAGACGATTG TATGCCTGGG ATATTCTACA GAAAAAACAG TCTGACATAC 50
CGAAAAGGAT TAAAGGTTAT ATTCACCAGG CGATTGGTGA CGTGTCTTTA 100
<210> 9342
<211> 100
<212> DNA
<213> Escherichia coli K-12 MG1655
<220> ytfi b4216
<222> 400-499
<223> ytfi b4216 U00096 4435285..4435785
<313> 9342: 431-450
<400> 9342
ACAGACGATT GTATGCCTGG GATATTCTAC AGAAAAAACA GTCTGACATA 50
CCGAAAAGGA TTAAAGGTTA TATTCACCAG GCGATTGGTG ACGTGTCTTT 100
<210> 9343
<211> 100
<212> DNA
<213> Escherichia coli K-12 MG1655
<220> ytfi b4216
<222> 401-500
<223> ytfi b4216 U00096 4435285..4435785
<313> 9343: 433-452
<400> 9343
CAGACGATTG TATGCCTGGG ATATTCTACA GAAAAAACAG TCTGACATAC 50
CGAAAAGGAT TAAAGGTTAT ATTCACCAGG CGATTGGTGA CGTGTCTTTA 100
<210> 9344
<211> 100
<212> DNA
<213> Escherichia coli K-12 MG1655
<220> ytfj b4217
<222> 456-555
<223> ytfj b4217 U00096 complement(4436285..4436839)
<313> 9344: 514-533
<400> 9344
GCTGGATAAG GATGGTCGCG TGCAATGGGC CAAAGACGGG GCGCTCACAC 50
CGGAAGAGGT GCAGCAAGTG ATGGACCTGC TGCAAAAGTT GCTCAAATAA 100
<210> 9345
<211> 100
<212> DNA
<213> Escherichia coli K-12 MG1655
<220> ytfj b4217
<222> 436-535
<223> ytfj b4217 U00096 complement(4436285..4436839)
<313> 9345: 463-482
<400> 9345
GAAAGTTCCG CTGTAGTGGT GCTGGATAAG GATGGTCGCG TGCAATGGGC 50
CAAAGACGGG GCGCTCACAC CGGAAGAGGT GCAGCAAGTG ATGGACCTGC 100
<210> 9346
<211> 100
<212> DNA
<213> Escherichia coli K-12 MG1655
<220> ytfj b4217
<222> 264-363
<223> ytfj b4217 U00096 complement(4436285..4436839)
<313> 9346: 318-337
<400> 9346

GTTACCGCAC GATCGTTACC AGACCACCAC CATTGTTAAC ACCGACGACG 50
CGATTCCGGG TTCAGGGATG TTTGTGCGCA GCAGTCTGGA GAGTAATAAA 100
<210> 9347
<211> 100
<212> DNA
<213> Escherichia coli K-12 MG1655
<220> ytfk b4218
<222> 147-246
<223> ytfk b4218 U00096 4437125..4437370
<313> 9347: 222-241
<400> 9347
GGGTAAGATT CTTATCCCAA AAGTGAAGGA TAAGCTGCAT TTGTCAGTGA 50
TGTCCGAAGT TAACCGTCAG GTTATGCGTC TGCAAACAGA GATGGCTTAA 100
<210> 9348
<211> 100
<212> DNA
<213> Escherichia coli K-12 MG1655
<220> ytfk b4218
<222> 45-144
<223> ytfk b4218 U00096 4437125..4437370
<313> 9348: 78-97
<400> 9348
AATTTTCCAA CGGTACAACC CACTTCAGGT GGCGAAGTAC GTAAAGATCC 50
TGTTCCGTGG ACGGTTATAC ATCAAGGACG TTGGCGCTTT TGAATTCGAT 100
<210> 9349
<211> 100
<212> DNA
<213> Escherichia coli K-12 MG1655
<220> ytfk b4218
<222> 1-100
<223> ytfk b4218 U00096 4437125..4437370
<313> 9349: 22-41
<400> 9349
GTGGCACCGC ACAGGCGTAA TACTCAGCAG GAGATAACAA TGAAAATTTT 50
CCAACGGTAC AACCCACTTC AGGTGGCGAA GTACGTAAAG ATCCTGTTCC 100
<210> 9350
<211> 100
<212> DNA
<213> Escherichia coli K-12 MG1655
<220> ytfl b4219
<222> 1245-1344
<223> ytfl b4219 U00096 complement(4437449..4438792)
<313> 9350: 1309-1328
<400> 9350
CCGCATCGAC CAGCTGCTGG TGACCCGGAT CGACAGCAAG GCCACCGCCC 50
TTTCGCCAAA ACTGCCTGAC GCTAAAGATA AAGAAGAAAG CGTCGCGTAA 100
<210> 9351
<211> 100
<212> DNA
<213> Escherichia coli K-12 MG1655
<220> ytfl b4219
<222> 1109-1208
<223> ytfl b4219 U00096 complement(4437449..4438792)
<313> 9351: 1166-1185
<400> 9351
TTGACGAGTT CCCGCAGTCG GGCAACTACG AAACCATCGG CGGCTTTATG 50
ATGTTTATGC TGCGTAAGAT CCCGAAACGC ACCGATTCGG TGAAATTCGC 100
<210> 9352
<211> 100
<212> DNA
<213> Escherichia coli K-12 MG1655
<220> ytfl b4219
<222> 1080-1179
<223> ytfl b4219 U00096 complement(4437449..4438792)
<313> 9352: 1128-1147
<400> 9352
AATTGACGAC GTCATGCGCG TGCTGGATAT TGACGAGTTC CCGCAGTCGG 50
GCAACTACGA AACCATCGGC GGCTTTATGA TGTTTATGCT GCGTAAGATC 100
<210> 9353
<211> 100
<212> DNA

<213> Escherichia coli K-12 MG1655
<220> ytfm b4221
<222> 1635-1734
<223> ytfm b4221 U00096 4439959..4441692
<313> 9353: 1699-1718
<400> 9353
ATCGCCGGTC GGGCCAATCA AACTCGATTT TGCCGTACCG GTCGCGGATA 50
AAGACGAACA CGGGTTACAG TTTTACATCG GTCTGGGGCC AGAATTATGA 100
<210> 9354
<211> 100
<212> DNA
<213> Escherichia coli K-12 MG1655
<220> ytfm b4221
<222> 1551-1650
<223> ytfm b4221 U00096 4439959..4441692
<313> 9354: 1578-1597
<400> 9354
GGTGTTTGTC GATAGTGGCG AAGCGGTAAG CGATATTCGC CGCAGCGACT 50
TTAAAACCGG TACCGGGGTC GGCGTGCGCT GGGAATCGCC GGTCGGGCCA 100
<210> 9355
<211> 100
<212> DNA
<213> Escherichia coli K-12 MG1655
<220> ytfm b4221
<222> 1500-1599
<223> ytfm b4221 U00096 4439959..4441692
<313> 9355: 1540-1559
<400> 9355
AACCGGATCG CTGGAATACC AGTACAACGT GACCGGAAAA TGGTGGGGCG 50
CGGTGTTTGT CGATAGTGGC GAAGCGGTAA GCGATATTCG CCGCAGCGAC 100
<210> 9356
<211> 100
<212> DNA
<213> Escherichia coli K-12 MG1655
<220> ytfn b4222
<222> 3681-3780
<223> ytfn b4222 U00096 4441689..4445468
<313> 9356: 3716-3735
<400> 9356
AACACTCACG TTACGTTATC GCCTGATGCC TAAGCTATAT CTGGAAGCCG 50
TGTCTGGTGT AGACCAGGCA CTGGATTTGC TCTATCAGTT CGAGTTTTAG 100
<210> 9357
<211> 100
<212> DNA
<213> Escherichia coli K-12 MG1655
<220> ytfn b4222
<222> 3574-3673
<223> ytfn b4222 U00096 4441689..4445468
<313> 9357: 3613-3632
<400> 9357
GCGCTCGACA CCCAGGGAGT AGGCGACTCC TCCCAGGTAG TGGTCAGCGG 50
CTATGTATTG CCAGGTCTGC AAGTGAAATA CGGCGTGGGT ATATTTGACT 100
<210> 9358
<211> 100
<212> DNA
<213> Escherichia coli K-12 MG1655
<220> ytfn b4222
<222> 3440-3539
<223> ytfn b4222 U00096 4441689..4445468
<313> 9358: 3469-3488
<400> 9358
TGCTACGTGG ACAAGGGCTG GAGAGCGATC AGAGCGACAG TGCGGCAATG 50
ACCTCGATGC TGATTGGTTT GGGGGTTGCG CAAAGTGGCC AGATTGTGGG 100
<210> 9359
<211> 100
<212> DNA
<213> Escherichia coli K-12 MG1655
<220> hipa b1508
<222> 1224-1323
<223> hipa b1508 U00096 complement(1588878..1590200)
<313> 9359: 1300-1319

<400> 9359
TTCATTACCG ACAGATTTTC CGGAGAACGT GGTGACGGCA GTTGAAAGCA  50
ATGTGTTGAG GTTGCATGGA CGGTTAAGCC GAGAATACGG TAGTAAGTGA  100
<210> 9360
<211> 100
<212> DNA
<213> Escherichia coli K-12 MG1655
<220> hipa b1508
<222> 1172-1271
<223> hipa b1508 U00096 complement(1588878..1590200)
<313> 9360: 1212-1231
<400> 9360
TCCTGAGTGA CTTTGCCAGA ATGATTCCAG CAGCACTGGA TAACGTGAAG  50
ACTTCATTAC CGACAGATTT TCCGGAGAAC GTGGTGACGG CAGTTGAAAG  100
<210> 9361
<211> 100
<212> DNA
<213> Escherichia coli K-12 MG1655
<220> hipa b1508
<222> 1138-1237
<223> hipa b1508 U00096 complement(1588878..1590200)
<313> 9361: 1176-1195
<400> 9361
GTGCTGAGAT TCCCGGAAGT GCAGATGCAT GAAATCCTGA GTGACTTTGC  50
CAGAATGATT CCAGCAGCAC TGGATAACGT GAAGACTTCA TTACCGACAG  100
<210> 9362
<211> 100
<212> DNA
<213> Escherichia coli K-12 MG1655
<220> ytfp b4223
<222> 243-342
<223> ytfp b4223 U00096 4445471..4445812
<313> 9362: 313-332
<400> 9362
TCAGACGCCG TACGGGAGTG CATGGATGTA CGTTTATCAA CGACCCGTCG  50
ATGGATTAAA GCTAATTGAA AGCGGCGACT GGTTAGACAG GGATAAGTAA  100
<210> 9363
<211> 100
<212> DNA
<213> Escherichia coli K-12 MG1655
<220> ytfp b4223
<222> 131-230
<223> ytfp b4223 U00096 4445471..4445812
<313> 9363: 172-191
<400> 9363
CAGTTCCGGG GAACGGAACG GTACACGGTG AAGTTTATCG TATTGACAAC  50
GCCACGCTGG CCGAACTTGA TGCCTTGCGC ACCAGGGGCG GTGAATACGC  100
<210> 9364
<211> 100
<212> DNA
<213> Escherichia coli K-12 MG1655
<220> ytfp b4223
<222> 9-108
<223> ytfp b4223 U00096 4445471..4445812
<313> 9364: 39-58
<400> 9364
ATTTGTCTAC GGCAGTTTAC GCCACAAACA AGGCAACAGT CACTGGATGA  50
CCAATGCCCA GTTACTGGGC GATTTCAGTA TCGATAACTA CCAGTTGTAT  100
<210> 9365
<211> 100
<212> DNA
<213> Escherichia coli K-12 MG1655
<220> hipb hipB
<222> 168-267
<223> hipb hipB U00096 complement(1590200..1590466)
<313> 9365: 237-256
<400> 9365
ATTTTTTAAG ATTTTACAGT CGCTTGAACT CTCAATGACG CTATGCGACG  50
CGAAAAATGC CTCGCCAGAA TCAACAGAAC AGCAAAATCT GGAGTGGTAA  100
<210> 9366
<211> 100

<212> DNA
<213> Escherichia coli K-12 MG1655
<220> hipb hipB
<222> 164-263
<223> hipb hipB U00096 complement(1590200..1590466)
<313> 9366: 200-219
<400> 9366
CGACATTTTT TAAGATTTTA CAGTCGCTTG AACTCTCAAT GACGCTATGC 50
GACGCGAAAA ATGCCTCGCC AGAATCAACA GAACAGCAAA ATCTGGAGTG 100
<210> 9367
<211> 100
<212> DNA
<213> Escherichia coli K-12 MG1655
<220> hipb hipB
<222> 93-192
<223> hipb hipB U00096 complement(1590200..1590466)
<313> 9367: 147-166
<400> 9367
GGCGAAAAAA ATTGGTATTA AGCAGGCGAC GATTTCCAAT TTCGAAAACA 50
ACCCTGACAA TACCACGCTC ACGACATTTT TTAAGATTTT ACAGTCGCTT 100
<210> 9368
<211> 100
<212> DNA
<213> Escherichia coli K-12 MG1655
<220> ytfq b4228
<222> 847-946
<223> ytfq b4228 U00096 4447539..4448495
<313> 9368: 908-927
<400> 9368
TACAAAAAAG ACGGCACCAT GCCTGAAAAG CTGACGTTAA CCAAATCCAC 50
CCTTTACCTG CCTGATACCG CAAAAGAAGA ATTAGAGAAG AAGAAAAATA 100
<210> 9369
<211> 100
<212> DNA
<213> Escherichia coli K-12 MG1655
<220> ytfq b4228
<222> 832-931
<223> ytfq b4228 U00096 4447539..4448495
<313> 9369: 847-866
<400> 9369
GACGCGCTGG AGAAATACAA AAAAGACGGC ACCATGCCTG AAAAGCTGAC 50
GTTAACCAAA TCCACCCTTT ACCTGCCTGA TACCGCAAAA GAAGAATTAG 100
<210> 9370
<211> 100
<212> DNA
<213> Escherichia coli K-12 MG1655
<220> ytfq b4228
<222> 758-857
<223> ytfq b4228 U00096 4447539..4448495
<313> 9370: 821-840
<400> 9370
TCTACAAAGC GATGATGGAT GGCGAAGCGA ACGCCAGTGT TGAACTGACG 50
CCGAATATGG CAGGTCCCGC CTTCGACGCG CTGGAGAAAT ACAAAAAAGA 100
<210> 9371
<211> 100
<212> DNA
<213> Escherichia coli K-12 MG1655
<220> yeea b2009
<222> 893-992
<223> yeea b2009 U00096 complement(2077555..2078613)
<313> 9371: 931-950
<400> 9371
CGGAAAAATT GAACGATGCT GTGGAAGAGC TGCGTCAGTT GCTCAATAAC 50
CACCATGACC TGAAGGTTGT GGAAACACCA ATCTATGGTT ATGTGTGGCT 100
<210> 9372
<211> 100
<212> DNA
<213> Escherichia coli K-12 MG1655
<220> yeea b2009
<222> 819-918
<223> yeea b2009 U00096 complement(2077555..2078613)

<313> 9372: 850-869
<400> 9372
CATGATGCAG CAAATACTGC TGAGCCTTGT TCATGCGCTG TACGAAGGTA 50
ATCCGCAGCC GGTTTTTGCC AATACGGAAA AATTGAACGA TGCTGTGGAA 100
<210> 9373
<211> 100
<212> DNA
<213> Escherichia coli K-12 MG1655
<220> yeea b2009
<222> 783-882
<223> yeea b2009 U00096 complement(2077555..2078613)
<313> 9373: 808-827
<400> 9373
GTTATTGAAC GCGCAAAAAC TTCGTGATAC CCAGCACATG ATGCAGCAAA 50
TACTGCTGAG CCTTGTTCAT GCGCTGTACG AAGGTAATCC GCAGCCGGTT 100
<210> 9374
<211> 100
<212> DNA
<213> Escherichia coli K-12 MG1655
<220> ytfr b4229
<222> 1155-1254
<223> ytfr b4229 U00096 4448633..4449886
<313> 9374: 1219-1238
<400> 9374
ATCCGCCAGC TTGGCATTCG CACACCTTCA ACTGAACAAC CGATTGAATT 50
TCTCTCCGGC GGCAATCAGC AAAAAGTGTT GCTTTCACGT TGGCTACTGA 100
<210> 9375
<211> 100
<212> DNA
<213> Escherichia coli K-12 MG1655
<220> ytfr b4229
<222> 1134-1233
<223> ytfr b4229 U00096 4448633..4449886
<313> 9375: 1181-1200
<400> 9375
CAAGAGATTG CCGAACGCTT TATCCGCCAG CTTGGCATTC GCACACCTTC 50
AACTGAACAA CCGATTGAAT TTCTCTCCGG CGGCAATCAG CAAAAAGTGT 100
<210> 9376
<211> 100
<212> DNA
<213> Escherichia coli K-12 MG1655
<220> ytfr b4229
<222> 1116-1215
<223> ytfr b4229 U00096 4448633..4449886
<313> 9376: 1144-1163
<400> 9376
ATTTCCCGCA AAGAACAGCA AGAGATTGCC GAACGCTTTA TCCGCCAGCT 50
TGGCATTCGC ACACCTTCAA CTGAACAACC GATTGAATTT CTCTCCGGCG 100
<210> 9377
<211> 100
<212> DNA
<213> Escherichia coli K-12 MG1655
<220> ytfs b4230
<222> 113-212
<223> ytfs b4230 U00096 4449862..4450134
<313> 9377: 149-168
<400> 9377
TATGCGCCGA TGGTCTGGCG CTGCTGGTGA TCTCCTCCGA ACTGGAAGAG 50
CTGGTGGGCT ATGCCGATCG GGTGATTATC ATGCGCGATC GCAAACAGGT 100
<210> 9378
<211> 100
<212> DNA
<213> Escherichia coli K-12 MG1655
<220> ytfs b4230
<222> 72-171
<223> ytfs b4230 U00096 4449862..4450134
<313> 9378: 105-124
<400> 9378
TGTTGGTGCC CACGCCGAGA TCATCCGCCT GATTGAAACG CTATGCGCCG 50
ATGGTCTGGC GCTGCTGGTG ATCTCCTCCG AACTGGAAGA GCTGGTGGGC 100
<210> 9379

<211> 100
<212> DNA
<213> Escherichia coli K-12 MG1655
<220> ytfs b4230
<222> 1-100
<223> ytfs b4230 U00096 4449862..4450134
<313> 9379: 36-55
<400> 9379
GTGTTGCTTT CACGTTGGCT ACTGACCCGA CCGCAATTTC TGATCCTCGA 50
TGAGCCAACC CGCGGCATTG ATGTTGGTGC CCACGCCGAG ATCATCCGCC 100
<210> 9380
<211> 100
<212> DNA
<213> Escherichia coli K-12 MG1655
<220> ytft b4231
<222> 927-1026
<223> ytft b4231 U00096 4450145..4451170
<313> 9380: 981-1000
<400> 9380
CCAGGTGGTG AAAGCGGTGG TGGTGCTTTG CGTGCTGATT GTTCAGTCGC 50
AACGCTTTAT CAGTCTGATT AAAGGAGTAC GTAGCCGTGA TAAAACGTAA 100
<210> 9381
<211> 100
<212> DNA
<213> Escherichia coli K-12 MG1655
<220> ytft b4231
<222> 891-990
<223> ytft b4231 U00096 4450145..4451170
<313> 9381: 935-954
<400> 9381
CGGAATTTTG CTTTCGGGCT TTCCGCCAGA GATGAACCAG GTGGTGAAAG 50
CGGTGGTGGT GCTTTGCGTG CTGATTGTTC AGTCGCAACG CTTTATCAGT 100
<210> 9382
<211> 100
<212> DNA
<213> Escherichia coli K-12 MG1655
<220> ytft b4231
<222> 844-943
<223> ytft b4231 U00096 4450145..4451170
<313> 9382: 874-893
<400> 9382
CTGCTACTTT CGGTGGTGGG GGCGCTGATT ATTCAGGGGA TGAACACCGG 50
AATTTTGCTT TCGGGCTTTC CGCCAGAGAT GAACCAGGTG GTGAAAGCGG 100
<210> 9383
<211> 100
<212> DNA
<213> Escherichia coli K-12 MG1655
<220> yeed b2013
<222> 129-228
<223> yeed b2013 U00096 complement(2082248..2082475)
<313> 9383: 192-211
<400> 9383
TGAAGCAATC CCACAGTGGG CGGCAGAAGA AGGACATGCC ATCACCGATT 50
ATCAGCAGAT TGGCGATGCA GCGTGGAGCA TCACCGTTCA AAAAGCCTGA 100
<210> 9384
<211> 100
<212> DNA
<213> Escherichia coli K-12 MG1655
<220> yeed b2013
<222> 129-228
<223> yeed b2013 U00096 complement(2082248..2082475)
<313> 9384: 166-185
<400> 9384
TGAAGCAATC CCACAGTGGG CGGCAGAAGA AGGACATGCC ATCACCGATT 50
ATCAGCAGAT TGGCGATGCA GCGTGGAGCA TCACCGTTCA AAAAGCCTGA 100
<210> 9385
<211> 100
<212> DNA
<213> Escherichia coli K-12 MG1655
<220> yeed b2013
<222> 56-155

<223> yeed b2013 U00096 complement(2082248..2082475)
<313> 9385: 82-101
<400> 9385
AAGCAAAAGC AGCTCTGGCA GAAATGGTCA GCGGCGATGA GTTAGTGATT  50
GAGTTTGATT GCACCCAGGC AACTGAAGCA ATCCCACAGT GGGCGGCAGA  100
<210> 9386
<211> 100
<212> DNA
<213> Escherichia coli K-12 MG1655
<220> yeee b2014
<222> 960-1059
<223> yeee b2014 U00096 complement(2082489..2083547)
<313> 9386: 1022-1041
<400> 9386
TGGCCTTGTA TTTATGATTC TCGGAGTCTG GACTGCGTCC TGGCTTGTGT  50
ATGTTCGACC GCAGCGTAAG GCGCGACTGG CAACTGCTGC GGCAAATTAA  100
<210> 9387
<211> 100
<212> DNA
<213> Escherichia coli K-12 MG1655
<220> yeee b2014
<222> 766-865
<223> yeee b2014 U00096 complement(2082489..2083547)
<313> 9387: 800-819
<400> 9387
TTAGGGATCT TCGTGGGGTC ATTTATTGCA GCCAAAGCGA GCCGTGAGTT  50
CCGCGTTCGC GCAGCTGATG CACAAACAAC ATTACGTAGC GGGTTAGGTG  100
<210> 9388
<211> 100
<212> DNA
<213> Escherichia coli K-12 MG1655
<220> yeee b2014
<222> 695-794
<223> yeee b2014 U00096 complement(2082489..2083547)
<313> 9388: 741-760
<400> 9388
CAACGGCCAA TATTCTGCAA TTTCTGGTCG CGGGTGACAT GAAATACATT  50
AACTGGGGCG TTTTCCTGGT GTTAGGGATC TTCGTGGGGT CATTTATTGC  100
<210> 9389
<211> 100
<212> DNA
<213> Escherichia coli K-12 MG1655
<220> yeef b2015
<222> 1266-1365
<223> yeef b2015 U00096 complement(2083726..2085090)
<313> 9389: 1331-1350
<400> 9389
GGTTCTGGGT CTGATCTGGG CTGCTATCGG TCTGATTTAC CTGGCTTGCG  50
TCACCAAGAG CTTCCGTAAT CCGGTTCCGC AGTACGAAGA CGTAGCATAA  100
<210> 9390
<211> 100
<212> DNA
<213> Escherichia coli K-12 MG1655
<220> yeef b2015
<222> 1246-1345
<223> yeef b2015 U00096 complement(2083726..2085090)
<313> 9390: 1299-1318
<400> 9390
AACCTGGAAG AAAGCTCAAT GGTTCTGGGT CTGATCTGGG CTGCTATCGG  50
TCTGATTTAC CTGGCTTGCG TCACCAAGAG CTTCCGTAAT CCGGTTCCGC  100
<210> 9391
<211> 100
<212> DNA
<213> Escherichia coli K-12 MG1655
<220> yeef b2015
<222> 1137-1236
<223> yeef b2015 U00096 complement(2083726..2085090)
<313> 9391: 1183-1202
<400> 9391
CATCTCGCAG TTCTGGATCC GTGAGAAGCG TAACAAGACG CTGAAAGATC  50
ACTTCCAGTA TCTGTTCCTG CCGATGTGTG GTGCGCTGAC CGTTGGTGCG  100

<210> 9392
<211> 100
<212> DNA
<213> Escherichia coli K-12 MG1655
<220> b1832 b1832
<222> 440-539
<223> b1832 b1832 U00096 complement(1913655..1914206)
<313> 9392: 482-501
<400> 9392
TCGGTCGCTT TACAGACGAG GACGAGCAAG GCTTACGTCA GCTTGTGGCA  50
CAGCTTGAAA AAGTGCTTGC AACGACGGAT TACAAAAAAT TCTTTGCGAG  100
<210> 9393
<211> 100
<212> DNA
<213> Escherichia coli K-12 MG1655
<220> b1832 b1832
<222> 376-475
<223> b1832 b1832 U00096 complement(1913655..1914206)
<313> 9393: 421-440
<400> 9393
ATTGTTCTGC CGCTGGTGGT GAAAAATCAG ATTATTGGTG TTCTCGACAT  50
CGATAGTACC GTCTTCGGTC GCTTTACAGA CGAGGACGAG CAAGGCTTAC  100
<210> 9394
<211> 100
<212> DNA
<213> Escherichia coli K-12 MG1655
<220> b1832 b1832
<222> 258-357
<223> b1832 b1832 U00096 complement(1913655..1914206)
<313> 9394: 295-314
<400> 9394
GATACCCGTC GGGCGCGGCG TGTGCGGCAC TGCGGTTGCC CGCAATCAAG  50
TGCAGCGTAT CGAGGATGTT CATGTGTTTG ACGGGCATAT TGCCTGTGAT  100
<210> 9395
<211> 100
<212> DNA
<213> Escherichia coli K-12 MG1655
<220> b1833 b1833
<222> 1185-1284
<223> b1833 b1833 U00096 1914282..1915565
<313> 9395: 1245-1264
<400> 9395
CGGCGCAGCG GTAATTTTGA CTATTCTTGC TGTGGAATGG CTGGACAGCC  50
GCTTACTTTG GGATGCACAT GAGTCAGGAA ACGCCCGCTT CGACGACTGA  100
<210> 9396
<211> 100
<212> DNA
<213> Escherichia coli K-12 MG1655
<220> b1833 b1833
<222> 1151-1250
<223> b1833 b1833 U00096 1914282..1915565
<313> 9396: 1209-1228
<400> 9396
TCGCTTTTAC TATGGGACCG GCTGCGTTTT ATTTCGGCGC AGCGGTAATT  50
TTGACTATTC TTGCTGTGGA ATGGCTGGAC AGCCGCTTAC TTTGGGATGC  100
<210> 9397
<211> 100
<212> DNA
<213> Escherichia coli K-12 MG1655
<220> b1833 b1833
<222> 1137-1236
<223> b1833 b1833 U00096 1914282..1915565
<313> 9397: 1151-1170
<400> 9397
TCGCGATCAG ATCCTCGCTT TTACTATGGG ACCGGCTGCG TTTTATTTCG  50
GCGCAGCGGT AATTTTGACT ATTCTTGCTG TGGAATGGCT GGACAGCCGC  100
<210> 9398
<211> 100
<212> DNA
<213> Escherichia coli K-12 MG1655
<220> b1834 b1835

<222> 2541-2640
<223> b1834 b1835 U00096 1915528..1918167
<313> 9398: 2608-2627
<400> 9398
TACGCCACTG GCACCGAAAG CCCAGGAAGG CAAACACTTC CTGTTGCAGG 50
AAAGTGAACC GAAAGAGTGG CGTGAATGGG GAACTGCGCT TCCCAAATAA 100
<210> 9399
<211> 100
<212> DNA
<213> Escherichia coli K-12 MG1655
<220> b1834 b1835
<222> 2530-2629
<223> b1834 b1835 U00096 1915528..1918167
<313> 9399: 2558-2577
<400> 9399
ACGCCTCCGG GTACGCCACT GGCACCGAAA GCCCAGGAAG GCAAACACTT 50
CCTGTTGCAG GAAAGTGAAC CGAAAGAGTG GCGTGAATGG GGAACTGCGC 100
<210> 9400
<211> 100
<212> DNA
<213> Escherichia coli K-12 MG1655
<220> b1834 b1835
<222> 2460-2559
<223> b1834 b1835 U00096 1915528..1918167
<313> 9400: 2504-2523
<400> 9400
TGGTCTGACG GGCGGCGTAG TGAAAACCGG CACCTTTAAC CAGTTTATCC 50
GTGGCGGCAT CGCCTTCGCC ACGCCTCCGG GTACGCCACT GGCACCGAAA 100
<210> 9401
<211> 100
<212> DNA
<213> Escherichia coli K-12 MG1655
<220> yeeo b1986
<222> 1545-1644
<223> yeeo b1986 U00096 complement(2056225..2057868)
<313> 9401: 1623-1642
<400> 9401
CGTGCTGTTT TACTGGCGAA TGGTTACTGG ACGTTGGCTA TGGAAATACC 50
CTCGACCCGA GCCGCAAAAG TGTGAAAAAA AGCCAGTTGT GTCGGAATAA 100
<210> 9402
<211> 100
<212> DNA
<213> Escherichia coli K-12 MG1655
<220> yeeo b1986
<222> 1532-1631
<223> yeeo b1986 U00096 complement(2056225..2057868)
<313> 9402: 1584-1603
<400> 9402
GGGCTGTGCG GGCCGTGCTG TTTTACTGGC GAATGGTTAC TGGACGTTGG 50
CTATGGAAAT ACCCTCGACC CGAGCCGCAA AAGTGTGAAA AAAAGCCAGT 100
<210> 9403
<211> 100
<212> DNA
<213> Escherichia coli K-12 MG1655
<220> yeeo b1986
<222> 1361-1460
<223> yeeo b1986 U00096 complement(2056225..2057868)
<313> 9403: 1392-1411
<400> 9403
GGTCCGCCTC ATGGGTGCTA CCCGCTGGAT TTAAAGGTGC TCGTGATGCC 50
CGTTACGCCA TGTGGGTTTC GATGTTGAGC ATGTGGGGTT GTCGGGTTGT 100
<210> 9404
<211> 100
<212> DNA
<213> Escherichia coli K-12 MG1655
<220> glms b3730
<222> 1726-1825
<223> glms b3730 U00096 complement(3909467..3911296)
<313> 9404: 1756-1775
<400> 9404
TTCTACACCG TTCCGCTGCA GCTGCTGGCT TACCATGTCG CGCTGATCAA 50

AGGCACCGAC GTTGACCAGC CGCGTAACCT GGCAAAATCG GTTACGGTTG 100
<210> 9405
<211> 100
<212> DNA
<213> Escherichia coli K-12 MG1655
<220> glms b3730
<222> 1666-1765
<223> glms b3730 U00096 complement(3909467..3911296)
<313> 9405: 1712-1731
<400> 9405
AGTAGCGATA ACATGCACAT CATCGAGATG CCGCATGTGG AAGAGGTGAT 50
TGCACCGATC TTCTACACCG TTCCGCTGCA GCTGCTGGCT TACCATGTCG 100
<210> 9406
<211> 100
<212> DNA
<213> Escherichia coli K-12 MG1655
<220> glms b3730
<222> 1651-1750
<223> glms b3730 U00096 complement(3909467..3911296)
<313> 9406: 1667-1686
<400> 9406
GATGCGGGTT TTGTAAGTAG CGATAACATG CACATCATCG AGATGCCGCA 50
TGTGGAAGAG GTGATTGCAC CGATCTTCTA CACCGTTCCG CTGCAGCTGC 100
<210> 9407
<211> 100
<212> DNA
<213> Escherichia coli K-12 MG1655
<220> yeep b2000
<222> 612-711
<223> yeep b2000 U00096 2068523..2069233
<313> 9407: 649-668
<400> 9407
GAGCGTCTGT ATTGCCTCTG TTGCACGCAC GGTTCTGCGT GCCGTCCGTG 50
ACACGGTGGT CTCTGTTGCC CGTGCGGTAT GGAACTGGAT TTTCTTCTGA 100
<210> 9408
<211> 100
<212> DNA
<213> Escherichia coli K-12 MG1655
<220> yeep b2000
<222> 523-622
<223> yeep b2000 U00096 2068523..2069233
<313> 9408: 546-565
<400> 9408
CTGCAGGACG AGCTGCGCAC GGAATCTGTC CGGTCTCAGG CCCGTGAACA 50
GTTTACCGGT GCGGTGGACC GGATATTTGA CACGGCGGAG AGCGTCTGTA 100
<210> 9409
<211> 100
<212> DNA
<213> Escherichia coli K-12 MG1655
<220> yeep b2000
<222> 455-554
<223> yeep b2000 U00096 2068523..2069233
<313> 9409: 489-508
<400> 9409
TGGATACGCT GGTCAGTGCG CTCATGACAG CGCTTCCCGA CCATGCCGCC 50
AGTCCCCTGA TGACCCGACT GCAGGACGAG CTGCGCACGG AATCTGTCCG 100
<210> 9410
<211> 100
<212> DNA
<213> Escherichia coli K-12 MG1655
<220> b1836 b1836
<222> 153-252
<223> b1836 b1836 U00096 1919789..1920040
<313> 9410: 229-248
<400> 9410
TGAAACCAGT ATCCCTGCCC TGCTAAATGG CGAACACTCT GTCCTTTACC 50
GTACCCGTTA CGATCAACAA TCTGATGCCT GGATTATGCG TCTTGCCTGA 100
<210> 9411
<211> 100
<212> DNA
<213> Escherichia coli K-12 MG1655

<220> b1836 b1836
<222> 138-237
<223> b1836 b1836 U00096 1919789..1920040
<313> 9411: 181-200
<400> 9411
GGATGCCTGG GATGCTGAAA CCAGTATCCC TGCCCTGCTA AATGGCGAAC  50
ACTCTGTCCT TTACCGTACC CGTTACGATC AACAATCTGA TGCCTGGATT  100
<210> 9412
<211> 100
<212> DNA
<213> Escherichia coli K-12 MG1655
<220> b1836 b1836
<222> 34-133
<223> b1836 b1836 U00096 1919789..1920040
<313> 9412: 91-110
<400> 9412
ATAGGCGCTT TTGAAATCGA CGACGGCGAA TTACACGGTG AATCGCCAGG  50
TGATCGAACG TTAACCATTC CTTGTAAATC TGACCCCGAT TTATGTATGC  100
<210> 9413
<211> 100
<212> DNA
<213> Escherichia coli K-12 MG1655
<220> b1837 b1838
<222> 177-276
<223> b1837 b1838 U00096 1920061..1920336
<313> 9413: 240-259
<400> 9413
TTCTATGAGC GATCAACGGA TCCGCCAGGG CGGTTGTTTT CCGATTGAGG  50
ATTTTATAGA TGGTTTCTGG CGACCTGCAC AGGAGTACGG TGATTTTTAA  100
<210> 9414
<211> 100
<212> DNA
<213> Escherichia coli K-12 MG1655
<220> b1837 b1838
<222> 130-229
<223> b1837 b1838 U00096 1920061..1920336
<313> 9414: 171-190
<400> 9414
GAAGATATTG TTGTGGATGT CTACAACACG GAACAGCAGT GTCTTTATTC  50
TATGAGCGAT CAACGGATCC GCCAGGGCGG TTGTTTTCCG ATTGAGGATT  100
<210> 9415
<211> 100
<212> DNA
<213> Escherichia coli K-12 MG1655
<220> b1837 b1838
<222> 86-185
<223> b1837 b1838 U00096 1920061..1920336
<313> 9415: 113-132
<400> 9415
TGTTCGCGCT GGTACTTTTT GTTTGCTACC TGGATGGCGG TTGTGAAGAT  50
ATTGTTGTGG ATGTCTACAA CACGGAACAG CAGTGTCTTT ATTCTATGAG  100
<210> 9416
<211> 100
<212> DNA
<213> Escherichia coli K-12 MG1655
<220> glmu b3731
<222> 1272-1371
<223> glmu b3731 U00096 complement(3911458..3912828)
<313> 9416: 1322-1341
<400> 9416
TACAACTGTG ACGCGTAATG TCGGCGAAAA TGCATTAGCT ATCAGCCGTG  50
TGCCGCAGAC TCAGAAAGAA GGCTGGCGTC GTCCGGTAAA GAAAAAGTGA  100
<210> 9417
<211> 100
<212> DNA
<213> Escherichia coli K-12 MG1655
<220> glmu b3731
<222> 1228-1327
<223> glmu b3731 U00096 complement(3911458..3912828)
<313> 9417: 1258-1277
<400> 9417

GTGGCCCCGG TAACAGTAGG CAAAGGCGCG ACCATTGCTG CGGGTACAAC 50
TGTGACGCGT AATGTCGGCG AAAATGCATT AGCTATCAGC CGTGTGCCGC 100
<210> 9418
<211> 100
<212> DNA
<213> Escherichia coli K-12 MG1655
<220> glmu b3731
<222> 1169-1268
<223> glmu b3731 U00096 complement(3911458..3912828)
<313> 9418: 1203-1222
<400> 9418
CGAATAAATT TAAGACCATT ATCGGCGACG ATGTGTTTGT TGGTTCCGAC 50
ACTCAGCTGG TGGCCCCGGT AACAGTAGGC AAAGGCGCGA CCATTGCTGC 100
<210> 9419
<211> 100
<212> DNA
<213> Escherichia coli K-12 MG1655
<220> yees b2003
<222> 348-447
<223> yees b2003 U00096 2074330..2074776
<313> 9419: 417-436
<400> 9419
TCTGGTACAG GCACTGGGCC TGGTGGATAT CCGGGTGCCG GACCATCTGA 50
TAGTCGGTGG CAACCAGGTT TTCTCCTTTG CCGAACATGG TCTGCTTTAA 100
<210> 9420
<211> 100
<212> DNA
<213> Escherichia coli K-12 MG1655
<220> yees b2003
<222> 305-404
<223> yees b2003 U00096 2074330..2074776
<313> 9420: 340-359
<400> 9420
GTGAAGTCAC ACCCAGTAAG GCAGACCGGC TTATCACCGA ACGTCTGGTA 50
CAGGCACTGG GCCTGGTGGA TATCCGGGTG CCGGACCATC TGATAGTCGG 100
<210> 9421
<211> 100
<212> DNA
<213> Escherichia coli K-12 MG1655
<220> yees b2003
<222> 251-350
<223> yees b2003 U00096 2074330..2074776
<313> 9421: 295-314
<400> 9421
GCGCCCTGTA CCACAATGCC GCTGCCGTGG TACTGGCACA CAATCACCCG 50
TCCGGTGAAG TCACACCCAG TAAGGCAGAC CGGCTTATCA CCGAACGTCT 100
<210> 9422
<211> 100
<212> DNA
<213> Escherichia coli K-12 MG1655
<220> b1839 b1839
<222> 243-342
<223> b1839 b1839 U00096 complement(1921389..1921730)
<313> 9422: 303-322
<400> 9422
TGCCCAACCC ATTTCGGTGA TTCAGATTGA TGATCCTAAC AATCCCGGCG 50
AAAAAATGAG TCTGGCACCG TTTATAGAAC GAGCTGAAAA ACTCTGTTAA 100
<210> 9423
<211> 100
<212> DNA
<213> Escherichia coli K-12 MG1655
<220> b1839 b1839
<222> 215-314
<223> b1839 b1839 U00096 complement(1921389..1921730)
<313> 9423: 276-295
<400> 9423
AAAAGGAAGT TGCCAGTGGG AAGACCAATG CCCAACCCAT TTCGGTGATT 50
CAGATTGATG ATCCTAACAA TCCCGGCGAA AAAATGAGTC TGGCACCGTT 100
<210> 9424
<211> 100
<212> DNA

<213> Escherichia coli K-12 MG1655
<220> b1839 b1839
<222> 175-274
<223> b1839 b1839 U00096 complement(1921389..1921730)
<313> 9424: 226-245
<400> 9424
CCGAGTACCC TCGTGCAGTA TCCTTTAAAC GATATCGCAC AAAAGGAAGT 50
TGCCAGTGGG AAGACCAATG CCCAACCCAT TTCGGTGATT CAGATTGATG 100
<210> 9425
<211> 100
<212> DNA
<213> Escherichia coli K-12 MG1655
<220> yeet b2004
<222> 123-222
<223> yeet b2004 U00096 2074839..2075060
<313> 9425: 201-220
<400> 9425
TGAAGTGCAG GATATTCCCG GTGTGCTGGC CGTGTTTGCT GAACGCCGTA 50
AGGACAGTTT TGGTCCGTAT GTCCGGCTGA TGAGCGTCAC CCTGAACTGA 100
<210> 9426
<211> 100
<212> DNA
<213> Escherichia coli K-12 MG1655
<220> yeet b2004
<222> 108-207
<223> yeet b2004 U00096 2074839..2075060
<313> 9426: 149-168
<400> 9426
AGCGTATACC GGTCGTGAAG TGCAGGATAT TCCCGGTGTG CTGGCCGTGT 50
TTGCTGAACG CCGTAAGGAC AGTTTTGGTC CGTATGTCCG GCTGATGAGC 100
<210> 9427
<211> 100
<212> DNA
<213> Escherichia coli K-12 MG1655
<220> yeet b2004
<222> 1-100
<223> yeet b2004 U00096 2074839..2075060
<313> 9427: 44-63
<400> 9427
ATGAAAATTA TCACCCGTGG TGAAGCCATG CGTATTCACC AACAACATCC 50
GACATCCCGT CTTTTTCCGT TCTGTACCGG TAAATACCGC TGGCACGGCA 100
<210> 9428
<211> 100
<212> DNA
<213> Escherichia coli K-12 MG1655
<220> yeeu b2005
<222> 270-369
<223> yeeu b2005 U00096 2075134..2075502
<313> 9428: 338-357
<400> 9428
CACGCTGTAT GCAAAAGGGC TGACCTGCAA AGCCGATACC CTCAGCAGTT 50
GTGATTACGT TTATCTGGCT GTTTATCCGA CGCCCGAAAT GAAAAATTAA 100
<210> 9429
<211> 100
<212> DNA
<213> Escherichia coli K-12 MG1655
<220> yeeu b2005
<222> 266-365
<223> yeeu b2005 U00096 2075134..2075502
<313> 9429: 293-312
<400> 9429
CCGTCACGCT GTATGCAAAA GGGCTGACCT GCAAAGCCGA TACCCTCAGC 50
AGTTGTGATT ACGTTTATCT GGCTGTTTAT CCGACGCCCG AAATGAAAAA 100
<210> 9430
<211> 100
<212> DNA
<213> Escherichia coli K-12 MG1655
<220> yeeu b2005
<222> 221-320
<223> yeeu b2005 U00096 2075134..2075502
<313> 9430: 246-265

<400> 9430
AACTCATGCT CACCAGCGGT GAACTGAATC CCCGCCATCA GCATACCGTC  50
ACGCTGTATG CAAAAGGGCT GACCTGCAAA GCCGATACCC TCAGCAGTTG  100
<210> 9431
<211> 100
<212> DNA
<213> Escherichia coli K-12 MG1655
<220> yeev b2006
<222> 272-371
<223> yeev b2006 U00096 2075591..2075965
<313> 9431: 317-336
<400> 9431
GCATCGATAT CCTCCGGGCT CGCAGGGCGA CCGGCCTGAT GACCCGCGAC  50
AATTACAGAA CGGTAAATAA CATTACCCTG GGTAAGTATC CGGAGGCGAA  100
<210> 9432
<211> 100
<212> DNA
<213> Escherichia coli K-12 MG1655
<220> yeev b2006
<222> 225-324
<223> yeev b2006 U00096 2075591..2075965
<313> 9432: 257-276
<400> 9432
CGACCAGCCG GGATTCAGCG CCTGTACCCG CTCTCAGTTA ATAAACAGCA  50
TCGATATCCT CCGGGCTCGC AGGGCGACCG GCCTGATGAC CCGCGACAAT  100
<210> 9433
<211> 100
<212> DNA
<213> Escherichia coli K-12 MG1655
<220> yeev b2006
<222> 166-265
<223> yeev b2006 U00096 2075591..2075965
<313> 9433: 191-210
<400> 9433
GGCATTTCAC TGTGTGATGC GGTGAACTTT CTCGTGGAAA AATACGCGCT  50
GGTGCGTACC GACCAGCCGG GATTCAGCGC CTGTACCCGC TCTCAGTTAA  100
<210> 9434
<211> 100
<212> DNA
<213> Escherichia coli K-12 MG1655
<220> yeew b2007
<222> 96-195
<223> yeew b2007 U00096 2075962..2076156
<313> 9434: 149-168
<400> 9434
TAATAAGGCC GCTGAAAACG GTTATTCACT CCGCGTGGTG GATGACCGTG  50
ACTCTACCGA AACACCGGCA ACTTATGCCA GCCCTCACCA GTTGCTGTAA  100
<210> 9435
<211> 100
<212> DNA
<213> Escherichia coli K-12 MG1655
<220> yeew b2007
<222> 75-174
<223> yeew b2007 U00096 2075962..2076156
<313> 9435: 112-131
<400> 9435
TGTTAATATC ACTGAACTGA TTAATAAGGC CGCTGAAAAC GGTTATTCAC  50
TCCGCGTGGT GGATGACCGT GACTCTACCG AAACACCGGC AACTTATGCC  100
<210> 9436
<211> 100
<212> DNA
<213> Escherichia coli K-12 MG1655
<220> yeew b2007
<222> 1-100
<223> yeew b2007 U00096 2075962..2076156
<313> 9436: 35-54
<400> 9436
ATGATGACAC TGGAAGCCGA CAGCGTTAAC GTACAGGCGC TGGATATGGG  50
GCACATTGTC GTTGACATTG ATGGTGTTAA TATCACTGAA CTGATTAATA  100
<210> 9437
<211> 100

<212> DNA
<213> Escherichia coli K-12 MG1655
<220> yeex b2008
<222> 297-396
<223> yeex b2008 U00096 complement(2077054..2077449)
<313> 9437: 376-395
<400> 9437
CGATTACATC ATCAAAAATG CCGAGCTCTC CAAAGAACGC CGCGATATCT 50
CCAAAAAGCT GAAAGCTATG GGCGAAATGA AAAACGGCGA AGCGAAGTAA 100
<210> 9438
<211> 100
<212> DNA
<213> Escherichia coli K-12 MG1655
<220> yeex b2008
<222> 230-329
<223> yeex b2008 U00096 complement(2077054..2077449)
<313> 9438: 272-291
<400> 9438
GGATGAGCGT TGAAGCAATC CAGGGCATCA TCGCCAGCAT GAAAGGTGAC 50
TATGAAGATC GCGTTGACGA TTACATCATC AAAAATGCCG AGCTCTCCAA 100
<210> 9439
<211> 100
<212> DNA
<213> Escherichia coli K-12 MG1655
<220> yeex b2008
<222> 173-272
<223> yeex b2008 U00096 complement(2077054..2077449)
<313> 9439: 232-251
<400> 9439
GTGACAACCA GAAGCGCGTC CTGCTGCTGG ACAACCTGAG CGATTACATC 50
AAGCCGGGGA TGAGCGTTGA AGCAATCCAG GGCATCATCG CCAGCATGAA 100
<210> 9440
<211> 100
<212> DNA
<213> Escherichia coli K-12 MG1655
<220> yeey yeeY
<222> 852-951
<223> yeey yeeY U00096 complement(2085351..2086301)
<313> 9440: 923-942
<400> 9440
CGCAGGACTT GCCAGTGGCT GGTGGCGGGA TGAAATTTTA GCAAATAGTG 50
CGATCGCCGG TGTTTATGCA AAATCTGATG ACGGAAAATC AGCCATTTAA 100
<210> 9441
<211> 100
<212> DNA
<213> Escherichia coli K-12 MG1655
<220> yeey yeeY
<222> 805-904
<223> yeey yeeY U00096 complement(2085351..2086301)
<313> 9441: 843-862
<400> 9441
GCCCAAACTC TCTTTGAACC CGTCTGGATT GGCTGGAACG AACAGACCGC 50
AGGACTTGCC AGTGGCTGGT GGCGGGATGA AATTTTAGCA AATAGTGCGA 100
<210> 9442
<211> 100
<212> DNA
<213> Escherichia coli K-12 MG1655
<220> yeey yeeY
<222> 708-807
<223> yeey yeeY U00096 complement(2085351..2086301)
<313> 9442: 743-762
<400> 9442
GGAGCTGCAA ATTAGCGGCC TCGGCTGCGG CTATTTACCC CGTTATCTGG 50
CGCAACGTTT TCTCGATAGT GGCGCGTTAA TCGAGAAGAA AGTGGTCGCC 100
<210> 9443
<211> 100
<212> DNA
<213> Escherichia coli K-12 MG1655
<220> glna b3871
<222> 1264-1363
<223> glna b3871 U00096 complement(4054205..4055614)

<313> 9443: 1304-1323
<400> 9443
CTGGATCTGG ACCGCGAGTT CCTGAAAGCC GGTGGCGTGT TCACTGACGA 50
AGCAATTGAT GCGTACATCG CTCTGCGTCG CGAAGAAGAT GACCGCGTGC 100
<210> 9444
<211> 100
<212> DNA
<213> Escherichia coli K-12 MG1655
<220> glna b3871
<222> 1199-1298
<223> glna b3871 U00096 complement(4054205..4055614)
<313> 9444: 1239-1258
<400> 9444
TGCCGCCAGA AGAAGCGAAA GAGATCCCAC AGGTTGCAGG CTCTCTGGAA 50
GAAGCACTGA ACGAACTGGA TCTGGACCGC GAGTTCCTGA AAGCCGGTGG 100
<210> 9445
<211> 100
<212> DNA
<213> Escherichia coli K-12 MG1655
<220> glna b3871
<222> 1147-1246
<223> glna b3871 U00096 complement(4054205..4055614)
<313> 9445: 1210-1229
<400> 9445
ATCAAGAACA AGATCCATCC GGGCGAAGCC ATGGACAAAA ACCTGTATGA 50
CCTGCCGCCA GAAGAAGCGA AAGAGATCCC ACAGGTTGCA GGCTCTCTGG 100
<210> 9446
<211> 100
<212> DNA
<213> Escherichia coli K-12 MG1655
<220> glnb b2554
<222> 237-336
<223> glnb b2554 U00096 complement(2685090..2685428)
<313> 9446: 282-301
<400> 9446
CACGGCGCAA ACCGGCAAAA TCGGTGACGG TAAAATCTTC GTCTTTGACG 50
TGGCACGGGT CATTCGCATC CGTACCGGTG AAGAGGACGA CGCGGCAATT 100
<210> 9447
<211> 100
<212> DNA
<213> Escherichia coli K-12 MG1655
<220> glnb b2554
<222> 215-314
<223> glnb b2554 U00096 complement(2685090..2685428)
<313> 9447: 239-258
<400> 9447
CCTGTGTCGA TACCATTATT CGCACGGCGC AAACCGGCAA AATCGGTGAC 50
GGTAAAATCT TCGTCTTTGA CGTGGCACGG GTCATTCGCA TCCGTACCGG 100
<210> 9448
<211> 100
<212> DNA
<213> Escherichia coli K-12 MG1655
<220> glnb b2554
<222> 114-213
<223> glnb b2554 U00096 complement(2685090..2685428)
<313> 9448: 154-173
<400> 9448
CCAGAAAGGC CATACCGAGC TGTACCGCGG CGCGGAGTAT ATGGTGGATT 50
TTCTGCCGAA AGTGAAAATT GAGATTGTCG TACCGGACGA CATTGTCGAT 100
<210> 9449
<211> 100
<212> DNA
<213> Escherichia coli K-12 MG1655
<220> glnd b0168
<222> 2574-2673
<223> glnd b0168 U00096 complement(185978..188650)
<313> 9449: 2640-2659
<400> 9449
CATAATTGCC ACCGCTGACC GGCGTGCGCT TAATAACGAG TTGCAGCAGG 50
AAGTGCATCA GCGGTTGACA GAGGCCCTCA ATCCAAACGA TAAAGGGTGA 100
<210> 9450

<211> 100
<212> DNA
<213> Escherichia coli K-12 MG1655
<220> glnd b0168
<222> 2519-2618
<223> glnd b0168 U00096 complement(185978..188650)
<313> 9450: 2568-2587
<400> 9450
TTTCGCTTCA TGGTGCCCGA ATTACAACCA TTGGCGAGCG AGTAGAAGAT 50
TTATTCATAA TTGCCACCGC TGACCGGCGT GCGCTTAATA ACGAGTTGCA 100
<210> 9451
<211> 100
<212> DNA
<213> Escherichia coli K-12 MG1655
<220> glnd b0168
<222> 2418-2517
<223> glnd b0168 U00096 complement(185978..188650)
<313> 9451: 2459-2478
<400> 9451
TTTGCCGACC CATACCGACC GCAAATCGTT CCTCGAACTG ATCGCCCTCG 50
ACCAACCTGG ACTGCTGGCG CGAGTCGGGA AAATTTTTGC CGATCTGGGA 100
<210> 9452
<211> 100
<212> DNA
<213> Escherichia coli K-12 MG1655
<220> glne b3054
<222> 2742-2841
<223> glne b3054 U00096 complement(3194442..3197282)
<313> 9452: 2818-2837
<400> 9452
GGCATTACAG GAATTGCCGG GCCATGTGTC GGAGGATTGC TTCACCGCAG 50
AGCGTGAACT GGTGCGGGCA AGCTGGCAGA AGTGGCTGGT GGAAGAATGA 100
<210> 9453
<211> 100
<212> DNA
<213> Escherichia coli K-12 MG1655
<220> glne b3054
<222> 2715-2814
<223> glne b3054 U00096 complement(3194442..3197282)
<313> 9453: 2769-2788
<400> 9453
TACGCTTCGC GATGAACTTC ATCATCTGGC ATTACAGGAA TTGCCGGGCC 50
ATGTGTCGGA GGATTGCTTC ACCGCAGAGC GTGAACTGGT GCGGGCAAGC 100
<210> 9454
<211> 100
<212> DNA
<213> Escherichia coli K-12 MG1655
<220> glne b3054
<222> 2634-2733
<223> glne b3054 U00096 complement(3194442..3197282)
<313> 9454: 2667-2686
<400> 9454
CAACGTGCGT ATTCTGGAAC TACTGGCGCA AAACGACATT ATGGAAGAGC 50
AGGAAGCGAT GGCGCTGACC CGTGCTTACA CTACGCTTCG CGATGAACTT 100
<210> 9455
<211> 100
<212> DNA
<213> Escherichia coli K-12 MG1655
<220> soda b3909
<222> 522-621
<223> soda b3909 U00096 4098391..4099011
<313> 9455: 595-614
<400> 9455
CTACCTGAAA TTCCAGAACC GCCGTCCGGA CTACATTAAA GAGTTCTGGA 50
ACGTGGTGAA CTGGGACGAA GCAGCGGCAC GTTTTGCGGC GAAAAAATAA 100
<210> 9456
<211> 100
<212> DNA
<213> Escherichia coli K-12 MG1655
<220> soda b3909
<222> 489-588

<223> soda b3909 U00096 4098391..4099011
<313> 9456: 522-541
<400> 9456
GATTATGGGC CTGGATGTGT GGGAACATGC TTACTACCTG AAATTCCAGA 50
ACCGCCGTCC GGACTACATT AAAGAGTTCT GGAACGTGGT GAACTGGGAC 100
<210> 9457
<211> 100
<212> DNA
<213> Escherichia coli K-12 MG1655
<220> soda b3909
<222> 439-538
<223> soda b3909 U00096 4098391..4099011
<313> 9457: 465-484
<400> 9457
CAGGATTCTC CGCTGATGGG TGAAGCTATT TCTGGCGCTT CCGGCTTCCC 50
GATTATGGGC CTGGATGTGT GGGAACATGC TTACTACCTG AAATTCCAGA 100
<210> 9458
<211> 100
<212> DNA
<213> Escherichia coli K-12 MG1655
<220> glng b3869
<222> 1280-1379
<223> glng b3869 U00096 complement(4051449..4052858)
<313> 9458: 1324-1343
<400> 9458
CAGAGCTGGA GCGGACGTTA CTGACGACCG CGTTGCGACA TACGCAGGGG 50
CATAAACAGG AAGCGGCGCG GCTACTCGGC TGGGGCCGCA ACACCCTGAC 100
<210> 9459
<211> 100
<212> DNA
<213> Escherichia coli K-12 MG1655
<220> glng b3869
<222> 1249-1348
<223> glng b3869 U00096 complement(4051449..4052858)
<313> 9459: 1294-1313
<400> 9459
GGTCATCAAA ATCTGCTTTC CGAAGCGCAG CCAGAGCTGG AGCGGACGTT 50
ACTGACGACC GCGTTGCGAC ATACGCAGGG GCATAAACAG GAAGCGGCGC 100
<210> 9460
<211> 100
<212> DNA
<213> Escherichia coli K-12 MG1655
<220> glng b3869
<222> 1103-1202
<223> glng b3869 U00096 complement(4051449..4052858)
<313> 9460: 1126-1145
<400> 9460
CGGTGATGGC CGCCGGGCAG GAAGTGTTGA TTCAGGATTT GCCCGGCGAA 50
CTGTTTGAAT CAACGGTTGC GGAGAGTACT TCGCAAATGC AACCGGACAG 100
<210> 9461
<211> 100
<212> DNA
<213> Escherichia coli K-12 MG1655
<220> sodb sodB
<222> 483-582
<223> sodb sodB U00096 1733402..1733983
<313> 9461: 511-530
<400> 9461
CGCTTATTAC ATCGACTATC GCAATGCACG TCCTGGCTAT CTGGAGCACT 50
TCTGGGCGCT GGTGAACTGG GAATTCGTAG CGAAAAATCT CGCTGCATAA 100
<210> 9462
<211> 100
<212> DNA
<213> Escherichia coli K-12 MG1655
<220> sodb sodB
<222> 435-534
<223> sodb sodB U00096 1733402..1733983
<313> 9462: 459-478
<400> 9462
GCTGACCACC GATGCGACTC CGCTGCTGAC CGTTGATGTC TGGGAACACG 50
CTTATTACAT CGACTATCGC AATGCACGTC CTGGCTATCT GGAGCACTTC 100

<210> 9463
<211> 100
<212> DNA
<213> Escherichia coli K-12 MG1655
<220> sodb sodB
<222> 370-469
<223> sodb sodB U00096 1733402..1733983
<313> 9463: 392-411
<400> 9463
ACCTGGCTGG TGAAAAACAG CGATGGCAAA CTGGCTATCG TTTCAACCTC 50
TAACGCGGGT ACTCCGCTGA CCACCGATGC GACTCCGCTG CTGACCGTTG 100
<210> 9464
<211> 100
<212> DNA
<213> Escherichia coli K-12 MG1655
<220> glnh b0812
<222> 648-747
<223> glnh b0812 U00096 complement(846481..847227)
<313> 9464: 685-704
<400> 9464
CGAGCTGCGT GACAAAGTCA ACGGCGCGTT GAAAACCCTG CGCGAGAACG 50
GAACTTACAA CGAAATCTAC AAAAAATGGT TCGGTACTGA ACCGAAATAA 100
<210> 9465
<211> 100
<212> DNA
<213> Escherichia coli K-12 MG1655
<220> glnh b0812
<222> 599-698
<223> glnh b0812 U00096 complement(846481..847227)
<313> 9465: 648-667
<400> 9465
ACTCTCTGGA AGCGCAGCAA TACGGTATTG CGTTCCCGAA AGGTAGCGAC 50
GAGCTGCGTG ACAAAGTCAA CGGCGCGTTG AAAACCCTGC GCGAGAACGG 100
<210> 9466
<211> 100
<212> DNA
<213> Escherichia coli K-12 MG1655
<220> glnh b0812
<222> 551-650
<223> glnh b0812 U00096 complement(846481..847227)
<313> 9466: 591-610
<400> 9466
TGTACTTCAT CAAAACCGCC GGTAACGGTC AGTTCAAAGC GGTAGGTGAC 50
TCTCTGGAAG CGCAGCAATA CGGTATTGCG TTCCCGAAAG GTAGCGACGA 100
<210> 9467
<211> 100
<212> DNA
<213> Escherichia coli K-12 MG1655
<220> sodc sodC
<222> 423-522
<223> sodc sodC U00096 complement(1722158..1722679)
<313> 9467: 498-517
<400> 9467
CAAAGCGCTG ATGGTCCACG TTGGCGGCGA TAATATGTCC GATCAACCTA 50
AACCGCTGGG CGGTGGCGGT GAACGCTATG CCTGTGGTGT AATTAAGTAA 100
<210> 9468
<211> 100
<212> DNA
<213> Escherichia coli K-12 MG1655
<220> sodc sodC
<222> 373-472
<223> sodc sodC U00096 complement(1722158..1722679)
<313> 9468: 405-424
<400> 9468
ACCGATGCCG TCATCGCGCC TCGTCTGAAA TCACTGGATG AAATCAAAGA 50
CAAAGCGCTG ATGGTCCACG TTGGCGGCGA TAATATGTCC GATCAACCTA 100
<210> 9469
<211> 100
<212> DNA
<213> Escherichia coli K-12 MG1655
<220> sodc sodC

<222> 207-306
<223> sodc sodC U00096 complement(1722158..1722679)
<313> 9469: 256-275
<400> 9469
TGCCAAAGGA AGCTGCCAGC CAGCCACCAA AGATGGCAAA GCCAGCGCCG 50
CGGAATCCGC AGGCGGGCAT CTTGATCCAC AAAATACCGG TAAACATGAA 100
<210> 9470
<211> 100
<212> DNA
<213> Escherichia coli K-12 MG1655
<220> glnk b0451
<222> 198-297
<223> glnk b0451 U00096 471822..472160
<313> 9470: 249-268
<400> 9470
TGATGACCAA CTCGATGAAG TGATCGATAT CGTCAGTAAG GCGGCTTACA 50
CCGGAAAAAT TGGCGACGGC AAAATCTTCG TCGCTGAATT GCAACGCGTC 100
<210> 9471
<211> 100
<212> DNA
<213> Escherichia coli K-12 MG1655
<220> glnk b0451
<222> 111-210
<223> glnk b0451 U00096 471822..472160
<313> 9471: 156-175
<400> 9471
GCGTCAGAAA GGGCATGCCG AGCTGTACCG GGGGGCGGAA TACAGCGTCA 50
ATTTCCTGCC AAAAGTAAAA ATTGATGTGG CGATTGCTGA TGACCAACTC 100
<210> 9472
<211> 100
<212> DNA
<213> Escherichia coli K-12 MG1655
<220> glnk b0451
<222> 31-130
<223> glnk b0451 U00096 471822..472160
<313> 9472: 67-86
<400> 9472
TTCAAGCTGG AAGACGTTCG TGAAGCGTTA TCTTCCATTG GTATTCAGGG 50
CCTGACCGTC ACCGAAGTGA AAGGTTTCGG GCGTCAGAAA GGGCATGCCG 100
<210> 9473
<211> 100
<212> DNA
<213> Escherichia coli K-12 MG1655
<220> glnl b3870
<222> 862-961
<223> glnl b3870 U00096 complement(4052870..4053919)
<313> 9473: 898-917
<400> 9473
AACGGGCCGG GCATTCCGCC TCATTTGCAG GATACGCTGT TTTACCCGAT 50
GGTCAGCGGC CGCGAAGGTG GCACCGGGCT TGGCTTATCC ATCGCTCGTA 100
<210> 9474
<211> 100
<212> DNA
<213> Escherichia coli K-12 MG1655
<220> glnl b3870
<222> 804-903
<223> glnl b3870 U00096 complement(4052870..4053919)
<313> 9474: 841-860
<400> 9474
TCAACTGACC TTACACGGCG AGCGCTACCG GCTGGCGGCG CGGATTGATG 50
TGGAAGATAA CGGGCCGGGC ATTCCGCCTC ATTTGCAGGA TACGCTGTTT 100
<210> 9475
<211> 100
<212> DNA
<213> Escherichia coli K-12 MG1655
<220> glnl b3870
<222> 738-837
<223> glnl b3870 U00096 complement(4052870..4053919)
<313> 9475: 800-819
<400> 9475
GCGCAATGCG CTACAGGCGC TGGGGCCGGA AGGCGGTGAA ATCATTCTGC 50

GTACCCGCAC CGCGTTTCAA CTGACCTTAC ACGGCGAGCG CTACCGGCTG 100
<210> 9476
<211> 100
<212> DNA
<213> Escherichia coli K-12 MG1655
<220> yefj b2032
<222> 375-474
<223> yefj b2032 U00096 complement(2100938..2101411)
<313> 9476: 429-448
<400> 9476
TTTACATGAT TATTCCGTAA GAAAATTTCC TGTGCTTTCT GATTTTATTG 50
TGTCATTTAT GTTAGGGATT AAGGAAGGTG CGAACAAGTC CCTGATATGA 100
<210> 9477
<211> 100
<212> DNA
<213> Escherichia coli K-12 MG1655
<220> yefj b2032
<222> 346-445
<223> yefj b2032 U00096 complement(2100938..2101411)
<313> 9477: 403-422
<400> 9477
TTATGCCTGT TCCGAGATGA AGCGAAATCT TTACATGATT ATTCCGTAAG 50
AAAATTTCCT GTGCTTTCTG ATTTTATTGT GTCATTTATG TTAGGGATTA 100
<210> 9478
<211> 100
<212> DNA
<213> Escherichia coli K-12 MG1655
<220> yefj b2032
<222> 335-434
<223> yefj b2032 U00096 complement(2100938..2101411)
<313> 9478: 388-407
<400> 9478
CTTTTAGTAC ATTATGCCTG TTCCGAGATG AAGCGAAATC TTTACATGAT 50
TATTCCGTAA GAAAATTTCC TGTGCTTTCT GATTTTATTG TGTCATTTAT 100
<210> 9479
<211> 100
<212> DNA
<213> Escherichia coli K-12 MG1655
<220> b1840 b1840
<222> 771-870
<223> b1840 b1840 U00096 complement(1921743..1922615)
<313> 9479: 817-836
<400> 9479
TCGTGCGGCA CCCTGGTTTG TGTGGATGAC TAAACTCGAA TGGGCGATAG 50
GTGCGGTAGT GCTGGTGATC ATCAGCCTGC TTGCGACTCT CGAACCTTTT 100
<210> 9480
<211> 100
<212> DNA
<213> Escherichia coli K-12 MG1655
<220> b1840 b1840
<222> 701-800
<223> b1840 b1840 U00096 complement(1921743..1922615)
<313> 9480: 751-770
<400> 9480
TGATGATTAT GGTGGTCATT GCGCTGGCTA ATCGTTATGT TCTTGTACCG 50
CGCATGAGGC AGGATGAAGA TCGTGCGGCA CCCTGGTTTG TGTGGATGAC 100
<210> 9481
<211> 100
<212> DNA
<213> Escherichia coli K-12 MG1655
<220> b1840 b1840
<222> 620-719
<223> b1840 b1840 U00096 complement(1921743..1922615)
<313> 9481: 680-699
<400> 9481
TTAATGCATT GTTGATTACC GGATTTCCCC CCACGCTTAC GACTTACTGG 50
GGCCAACTCT TATTGCTCAA AGCCATCCTG GTGATGATTA TGGTGGTCAT 100
<210> 9482
<211> 100
<212> DNA
<213> Escherichia coli K-12 MG1655

<220> b1841 b1842
<222> 276-375
<223> b1841 b1842 U00096 complement(1922619..1922993)
<313> 9482: 340-359
<400> 9482
GGCCGATTCG CTGAAACCCG GAACCTACAC CGTTGACTGG CATGTTGTTT  50
CGGTGGACGG GCATAAAACG AAAGGACACT ACACCTTTAG CGTGAAATAA  100
<210> 9483
<211> 100
<212> DNA
<213> Escherichia coli K-12 MG1655
<220> b1841 b1842
<222> 255-354
<223> b1841 b1842 U00096 complement(1922619..1922993)
<313> 9483: 295-314
<400> 9483
AAAGCAACTG ATTGTTCCAC TGGCCGATTC GCTGAAACCC GGAACCTACA  50
CCGTTGACTG GCATGTTGTT TCGGTGGACG GGCATAAAAC GAAAGGACAC  100
<210> 9484
<211> 100
<212> DNA
<213> Escherichia coli K-12 MG1655
<220> b1841 b1842
<222> 192-291
<223> b1841 b1842 U00096 complement(1922619..1922993)
<313> 9484: 259-278
<400> 9484
AATCACGGGG CCAAAAAACG AAAATATTAA AACATTACCG GCGAAGCGAA  50
ATGAGCAGGA CCAAAAGCAA CTGATTGTTC CACTGGCCGA TTCGCTGAAA  100
<210> 9485
<211> 100
<212> DNA
<213> Escherichia coli K-12 MG1655
<220> glnp b0811
<222> 561-660
<223> glnp b0811 U00096 complement(845683..846342)
<313> 9485: 626-645
<400> 9485
CCTTGAGATC TGGAGCGCCG TGGCGGTGTT CTATCTGATT ATTACCCTGG  50
TGCTGAGCTT TATTCTGCGT CGTCTGGAAA GAAGGATGAA AATCCTGTGA  100
<210> 9486
<211> 100
<212> DNA
<213> Escherichia coli K-12 MG1655
<220> glnp b0811
<222> 461-560
<223> glnp b0811 U00096 complement(845683..846342)
<313> 9486: 490-509
<400> 9486
AGTGGATCAT CAGCATTAAA GACACCTCGC TGTTTATTGT GATCGGCGTG  50
GCGGAACTGA CCCGTCAGGG GCAAGAAATT ATTGCCGGTA ACTTCCGCGC  100
<210> 9487
<211> 100
<212> DNA
<213> Escherichia coli K-12 MG1655
<220> glnp b0811
<222> 405-504
<223> glnp b0811 U00096 complement(845683..846342)
<313> 9487: 438-457
<400> 9487
TCGCTACGTC ATTTTACCGC TGGCACTGCG TCGTATGCTG CCGCCGCTGG  50
GTAACCAGTG GATCATCAGC ATTAAAGACA CCTCGCTGTT TATTGTGATC  100
<210> 9488
<211> 100
<212> DNA
<213> Escherichia coli K-12 MG1655
<220> yefm b2018
<222> 165-264
<223> yefm b2018 U00096 complement(2087484..2087762)
<313> 9488: 211-230
<400> 9488

CTCGCTGGAA GAGACGGCTT ATCTACTGCG CTCCCCCGCT AACGCCCGGA 50
GATTGATGGA CTCAATCGAT AGCCTGAAAT CAGGCAAAGG AACGGAAAAG 100
<210> 9489
<211> 100
<212> DNA
<213> Escherichia coli K-12 MG1655
<220> yefm b2018
<222> 127-226
<223> yefm b2018 U00096 complement(2087484..2087762)
<313> 9489: 182-201
<400> 9489
GGAGAGGCTT GTGTTCTGAT GTCACTCGAA GAATACAACT CGCTGGAAGA 50
GACGGCTTAT CTACTGCGCT CCCCCGCTAA CGCCCGGAGA TTGATGGACT 100
<210> 9490
<211> 100
<212> DNA
<213> Escherichia coli K-12 MG1655
<220> yefm b2018
<222> 78-177
<223> yefm b2018 U00096 complement(2087484..2087762)
<313> 9490: 105-124
<400> 9490
GATGAAAGCC GTTGAAGATC ATGCCCCGAT CCTTATTACT CGTCAGAATG 50
GAGAGGCTTG TGTTCTGATG TCACTCGAAG AATACAACTC GCTGGAAGAG 100
<210> 9491
<211> 100
<212> DNA
<213> Escherichia coli K-12 MG1655
<220> b1843 b1843
<222> 558-657
<223> b1843 b1843 U00096 1923464..1924120
<313> 9491: 633-652
<400> 9491
ATATGGTCGT CTCATCGTTC GCGCCGATCG TGGTTCATTA TTGTTAGTCG 50
GTCAGCGTTC ATCACAGGGT TGGCAAGGCG ATATCATTCC ATTACGCTAG 100
<210> 9492
<211> 100
<212> DNA
<213> Escherichia coli K-12 MG1655
<220> b1843 b1843
<222> 428-527
<223> b1843 b1843 U00096 1923464..1924120
<313> 9492: 485-504
<400> 9492
TTACTACAGG GCAATGTCTG GGAGAACCCG ACCTGCTGGC GTCGGCCCGT 50
CGCTTACAAT TTTTTTCACA TCAGTACTCG ATTGCCGTGC TGATGGCAAA 100
<210> 9493
<211> 100
<212> DNA
<213> Escherichia coli K-12 MG1655
<220> b1843 b1843
<222> 393-492
<223> b1843 b1843 U00096 1923464..1924120
<313> 9493: 439-458
<400> 9493
GCAAGGTATG GATATGGACC CGACCTGTTC ACTGTTTACT ACAGGGCAAT 50
GTCTGGGAGA ACCCGACCTG CTGGCGTCGG CCCGTCGCTT ACAATTTTTT 100
<210> 9494
<211> 100
<212> DNA
<213> Escherichia coli K-12 MG1655
<220> glnq b0810
<222> 570-669
<223> glnq b0810 U00096 complement(844964..845686)
<313> 9494: 620-639
<400> 9494
GATCGTGACC CACGAAATCG GTTTTGCCGA GAAAGTAGCT TCGCGGCTGA 50
TCTTTATCGA CAAAGGCCGG ATTGCGGAAG ATGGCAATCC GCAGGTGTTG 100
<210> 9495
<211> 100
<212> DNA

<213> Escherichia coli K-12 MG1655
<220> glnq b0810
<222> 537-636
<223> glnq b0810 U00096 complement(844964..845686)
<313> 9495: 561-580
<400> 9495
GCAGGATCTG GCTGAAGAAG GGATGACGAT GGTGATCGTG ACCCACGAAA 50
TCGGTTTTGC CGAGAAAGTA GCTTCGCGGC TGATCTTTAT CGACAAAGGC 100
<210> 9496
<211> 100
<212> DNA
<213> Escherichia coli K-12 MG1655
<220> glnq b0810
<222> 484-583
<223> glnq b0810 U00096 complement(844964..845686)
<313> 9496: 518-537
<400> 9496
GAACCGACTT CCGCTCTTGA CCCGGAACTG CGCCATGAAG TGCTGAAGGT 50
TATGCAGGAT CTGGCTGAAG AAGGGATGAC GATGGTGATC GTGACCCACG 100
<210> 9497
<211> 100
<212> DNA
<213> Escherichia coli K-12 MG1655
<220> b1844 b1845
<222> 564-663
<223> b1844 b1845 U00096 1924144..1924806
<313> 9497: 618-637
<400> 9497
CGAACGCGAT CCGGGCTATC TGCGCTGGTT ATTTAATAAC CTGGACAGCA 50
TGAGCCCGGA GCTGCGTTTA ACACTGAAAC ATTATCTGGA AAATACTTAG 100
<210> 9498
<211> 100
<212> DNA
<213> Escherichia coli K-12 MG1655
<220> b1844 b1845
<222> 540-639
<223> b1844 b1845 U00096 1924144..1924806
<313> 9498: 574-593
<400> 9498
TGGCAAAGCG GTTTCCGACG TTGCCGAACG CGATCCGGGC TATCTGCGCT 50
GGTTATTTAA TAACCTGGAC AGCATGAGCC CGGAGCTGCG TTTAACACTG 100
<210> 9499
<211> 100
<212> DNA
<213> Escherichia coli K-12 MG1655
<220> b1844 b1845
<222> 477-576
<223> b1844 b1845 U00096 1924144..1924806
<313> 9499: 527-546
<400> 9499
ACAGATGGCC GATATCACCG GACGTCCGTC GTTGATGACG ACCTTCACCT 50
TTGGCAAATA CCGTGGCAAA GCGGTTTCCG ACGTTGCCGA ACGCGATCCG 100
<210> 9500
<211> 100
<212> DNA
<213> Escherichia coli K-12 MG1655
<220> glns b0681
<222> 1566-1665
<223> glns b0681 U00096 705316..706980
<313> 9500: 1632-1651
<400> 9500
AGGTTACTTC TGCCTCGATA GCCGCCATTC TACGGCGGAA AAACCGGTAT 50
TTAACCGCAC CGTTGGGCTG CGTGATACCT GGGCGAAAGT AGGCGAGTAA 100
<210> 9501
<211> 100
<212> DNA
<213> Escherichia coli K-12 MG1655
<220> glns b0681
<222> 1527-1626
<223> glns b0681 U00096 705316..706980
<313> 9501: 1571-1590

<400> 9501
AGATGCGGTT GCGGGTAAAG CATTCCAGTT TGAGCGTGAA GGTTACTTCT 50
GCCTCGATAG CCGCCATTCT ACGGCGGAAA AACCGGTATT TAACCGCACC 100
<210> 9502
<211> 100
<212> DNA
<213> Escherichia coli K-12 MG1655
<220> glns b0681
<222> 1421-1520
<223> glns b0681 U00096 705316..706980
<313> 9502: 1453-1472
<400> 9502
ACCGTCTGTT CAGCGTGCCT AACCCAGGTG CTGCGGATGA TTTCCTGTCG 50
GTGATTAACC CGGAATCGCT GGTGATCAAA CAGGGCTTTG CTGAACCGTC 100
<210> 9503
<211> 100
<212> DNA
<213> Escherichia coli K-12 MG1655
<220> fola b0049
<222> 381-480
<223> fola b0049 U00096 49823..50302
<313> 9503: 444-463
<400> 9503
TTACGAGCCG GATGACTGGG AATCGGTATT CAGCGAATTC CACGATGCTG 50
ATGCGCAGAA CTCTCACAGC TATTGCTTTG AGATTCTGGA GCGGCGGTAA 100
<210> 9504
<211> 100
<212> DNA
<213> Escherichia coli K-12 MG1655
<220> fola b0049
<222> 378-477
<223> fola b0049 U00096 49823..50302
<313> 9504: 407-426
<400> 9504
GGATTACGAG CCGGATGACT GGGAATCGGT ATTCAGCGAA TTCCACGATG 50
CTGATGCGCA GAACTCTCAC AGCTATTGCT TTGAGATTCT GGAGCGGCGG 100
<210> 9505
<211> 100
<212> DNA
<213> Escherichia coli K-12 MG1655
<220> fola b0049
<222> 290-389
<223> fola b0049 U00096 49823..50302
<313> 9505: 337-356
<400> 9505
GTCGCGTTTA TGAACAGTTC TTGCCAAAAG CGCAAAAACT GTATCTGACG 50
CATATCGACG CAGAAGTGGA AGGCGACACC CATTTCCCGG ATTACGAGCC 100
<210> 9506
<211> 100
<212> DNA
<213> Escherichia coli K-12 MG1655
<220> gloa b1652
<222> 297-396
<223> gloa b1652 U00096 1725861..1726268
<313> 9506: 314-333
<400> 9506
AGCGGGTCCG GTAAAAGGCG GTACTACGGT TATCGCGTTT GTGGAAGATC 50
CGGACGGTTA CAAAATTGAG TTAATCGAAG AGAAAGACGC CGGTCGCGGT 100
<210> 9507
<211> 100
<212> DNA
<213> Escherichia coli K-12 MG1655
<220> gloa b1652
<222> 215-314
<223> gloa b1652 U00096 1725861..1726268
<313> 9507: 270-289
<400> 9507
ATGGTCACAT CGCGCTTAGC GTAGATAACG CCGCTGAAGC GTGCGAAAAA 50
ATCCGTCAAA ACGGGGGTAA CGTGACCCGT GAAGCGGGTC CGGTAAAAGG 100
<210> 9508
<211> 100

<212> DNA
<213> Escherichia coli K-12 MG1655
<220> gloa b1652
<222> 199-298
<223> gloa b1652 U00096 1725861..1726268
<313> 9508: 234-253
<400> 9508
GAACTCGGCA CTGCTTATGG TCACATCGCG CTTAGCGTAG ATAACGCCGC 50
TGAAGCGTGC GAAAAAATCC GTCAAAACGG GGGTAACGTG ACCCGTGAAG 100
<210> 9509
<211> 100
<212> DNA
<213> Escherichia coli K-12 MG1655
<220> folc b2316
<222> 1170-1269
<223> folc b2316 U00096 complement(2429694..2430962)
<313> 9509: 1241-1260
<400> 9509
GGACGCTAAA GCGGAAGACA CCGTGCTGGT GTGTGGTTCT TTCCACACGG 50
TCGCACATGT CATGGAAGTG ATTGACGCGA GGAGAAGCGG TGGCAAGTAA 100
<210> 9510
<211> 100
<212> DNA
<213> Escherichia coli K-12 MG1655
<220> folc b2316
<222> 1129-1228
<223> folc b2316 U00096 complement(2429694..2430962)
<313> 9510: 1174-1193
<400> 9510
TCATTTGATA GCGTTGCGCA GGCATGGGAT GCCGCAATGG CGGACGCTAA 50
AGCGGAAGAC ACCGTGCTGG TGTGTGGTTC TTTCCACACG GTCGCACATG 100
<210> 9511
<211> 100
<212> DNA
<213> Escherichia coli K-12 MG1655
<220> folc b2316
<222> 1091-1190
<223> folc b2316 U00096 complement(2429694..2430962)
<313> 9511: 1119-1138
<400> 9511
CGGCAGAACA ACTGCTTGAG CATTTGGGTA ACGGCAAATC ATTTGATAGC 50
GTTGCGCAGG CATGGGATGC CGCAATGGCG GACGCTAAAG CGGAAGACAC 100
<210> 9512
<211> 100
<212> DNA
<213> Escherichia coli K-12 MG1655
<220> glob b0213
<222> 352-451
<223> glob b0213 U00096 complement(234027..234782)
<313> 9512: 370-389
<400> 9512
TTCAGTAAAC CTTATCTATT TTGCGGCGAC ACACTGTTTT CTGGTGGGTG 50
TGGTCGGTTG TTTGAAGGGA CAGCATCACA AATGTATCAA TCACTTAAAA 100
<210> 9513
<211> 100
<212> DNA
<213> Escherichia coli K-12 MG1655
<220> glob b0213
<222> 301-400
<223> glob b0213 U00096 complement(234027..234782)
<313> 9513: 336-355
<400> 9513
GAATTTAGTG TAATTGCTAC GCCGGGTCAC ACTTTAGGAC ATATCTGTTA 50
CTTCAGTAAA CCTTATCTAT TTTGCGGCGA CACACTGTTT TCTGGTGGGT 100
<210> 9514
<211> 100
<212> DNA
<213> Escherichia coli K-12 MG1655
<220> glob b0213
<222> 260-359
<223> glob b0213 U00096 complement(234027..234782)

<313> 9514: 283-302
<400> 9514
AGGTAGTCAA AGATGGCGAA ACTGCCTTCG TTTTGGGGCA TGAATTTAGT 50
GTAATTGCTA CGCCGGGTCA CACTTTAGGA CATATCTGTT ACTTCAGTAA 100
<210> 9515
<211> 100
<212> DNA
<213> Escherichia coli K-12 MG1655
<220> fold b0530
<222> 749-848
<223> fold b0530 U00096 complement(556098..556964)
<313> 9515: 813-832
<400> 9515
CTAAACGCGC CTCATACATT ACGCCTGTTC CCGGCGGCGT TGGCCCGATG 50
ACGGTTGCCA CGCTGATTGA AAACACGCTA CAGGCGTGCG TTGAATATCA 100
<210> 9516
<211> 100
<212> DNA
<213> Escherichia coli K-12 MG1655
<220> fold b0530
<222> 724-823
<223> fold b0530 U00096 complement(556098..556964)
<313> 9516: 745-764
<400> 9516
GGCGACGTCG TGTTTGAAGA CGCGGCTAAA CGCGCCTCAT ACATTACGCC 50
TGTTCCCGGC GGCGTTGGCC CGATGACGGT TGCCACGCTG ATTGAAAACA 100
<210> 9517
<211> 100
<212> DNA
<213> Escherichia coli K-12 MG1655
<220> fold b0530
<222> 667-766
<223> fold b0530 U00096 complement(556098..556964)
<313> 9517: 691-710
<400> 9517
GAAGGCGCAA TTGTGATTGA TGTCGGCATC AACCGTCTGG AAAATGGCAA 50
AGTTGTGGGC GACGTCGTGT TTGAAGACGC GGCTAAACGC GCCTCATACA 100
<210> 9518
<211> 100
<212> DNA
<213> Escherichia coli K-12 MG1655
<220> fole b2154
<222> 570-669
<223> fole b2154 U00096 complement(2241004..2241672)
<313> 9518: 625-644
<400> 9518
TGCAACCAGT GCCACGACAA CGACCTCTCT TGGTGGATTG TTCAAATCCA 50
GTCAGAATAC GCGCCACGAG TTTCTGCGCG CTGTGCGTCA TCACAACTGA 100
<210> 9519
<211> 100
<212> DNA
<213> Escherichia coli K-12 MG1655
<220> fole b2154
<222> 550-649
<223> fole b2154 U00096 complement(2241004..2241672)
<313> 9519: 578-597
<400> 9519
AAGGCGCGTG GCATCCGCGA TGCAACCAGT GCCACGACAA CGACCTCTCT 50
TGGTGGATTG TTCAAATCCA GTCAGAATAC GCGCCACGAG TTTCTGCGCG 100
<210> 9520
<211> 100
<212> DNA
<213> Escherichia coli K-12 MG1655
<220> fole b2154
<222> 449-548
<223> fole b2154 U00096 complement(2241004..2241672)
<313> 9520: 505-524
<400> 9520
AGGTGCAGGA ACGTCTGACG CAGCAAATTC TTATTGCGCT ACAAACGCTG 50
CTGGGCACCA ATAACGTGGC TGTCTCGATC GACGCGGTGC ATTACTGCGT 100
<210> 9521

<211> 100
<212> DNA
<213> Escherichia coli K-12 MG1655
<220> yegb b2078
<222> 1312-1411
<223> yegb b2078 U00096 2159486..2160901
<313> 9521: 1355-1374
<400> 9521
AGCATGGCGT TGATCATCGC CCTTCCGGCG TTCATCTTTG CCAGAGTGCC 50
GAACGATACG CATCAAAATG TAGCTATTTC GCGGCGAAAA AGGAGCGCGC 100
<210> 9522
<211> 100
<212> DNA
<213> Escherichia coli K-12 MG1655
<220> yegb b2078
<222> 1291-1390
<223> yegb b2078 U00096 2159486..2160901
<313> 9522: 1310-1329
<400> 9522
GTCTTTATGT ACACCTGGCT TAGCATGGCG TTGATCATCG CCCTTCCGGC 50
GTTCATCTTT GCCAGAGTGC CGAACGATAC GCATCAAAAT GTAGCTATTT 100
<210> 9523
<211> 100
<212> DNA
<213> Escherichia coli K-12 MG1655
<220> yegb b2078
<222> 1203-1302
<223> yegb b2078 U00096 2159486..2160901
<313> 9523: 1246-1265
<400> 9523
GATGAGTATC GGCGTCACTA TCGCCGGGCT GTTGCTGGGA CTTTTTGGTT 50
CACAGCATGT CAGCGTCGAC AGCGGCACCA CACAAACCGT CTTTATGTAC 100
<210> 9524
<211> 100
<212> DNA
<213> Escherichia coli K-12 MG1655
<220> cpsb b2050
<222> 1338-1437
<223> cpsb b2050 U00096 complement(2121106..2122542)
<313> 9524: 1376-1395
<400> 9524
CCCGGGGAAA ATTCCGCTCG ATTTAATTGA AGTGCGCTCC GGCTCTTATC 50
TCGAAGAGGA TGATGTGGTG CGTTTCGCGG ATCGCTACGG ACGGGTGTAA 100
<210> 9525
<211> 100
<212> DNA
<213> Escherichia coli K-12 MG1655
<220> cpsb b2050
<222> 1268-1367
<223> cpsb b2050 U00096 complement(2121106..2122542)
<313> 9525: 1308-1327
<400> 9525
GTGATATCAA ACTGCTTGGT GAAAACGAGT CCATTTATAT TCCGCTGGGG 50
GCGACGCATT GCCTGGAAAA CCCGGGGAAA ATTCCGCTCG ATTTAATTGA 100
<210> 9526
<211> 100
<212> DNA
<213> Escherichia coli K-12 MG1655
<220> cpsb b2050
<222> 1204-1303
<223> cpsb b2050 U00096 complement(2121106..2122542)
<313> 9526: 1235-1254
<400> 9526
ATGCACCATC ACCGCGCGGA ACACTGGGTG GTTGTCGCGG GAACGGCAAA 50
AGTCACCATT GATGGTGATA TCAAACTGCT TGGTGAAAAC GAGTCCATTT 100
<210> 9527
<211> 100
<212> DNA
<213> Escherichia coli K-12 MG1655
<220> yegd yegD
<222> 1317-1416

<223> yegd yegD U00096 2145633..2147048
<313> 9527: 1378-1397
<400> 9527
GCTGGCAGAA CAGTTGCCGG GCATTCCGAT TGCAGGCGGC GATGACTTTG 50
GCTCCGTCAC CGCCGGGCTG GCACGCTGGG CGGAAGTGGT GTTTCGTTAA 100
<210> 9528
<211> 100
<212> DNA
<213> Escherichia coli K-12 MG1655
<220> yegd yegD
<222> 1317-1416
<223> yegd yegD U00096 2145633..2147048
<313> 9528: 1339-1358
<400> 9528
GCTGGCAGAA CAGTTGCCGG GCATTCCGAT TGCAGGCGGC GATGACTTTG 50
GCTCCGTCAC CGCCGGGCTG GCACGCTGGG CGGAAGTGGT GTTTCGTTAA 100
<210> 9529
<211> 100
<212> DNA
<213> Escherichia coli K-12 MG1655
<220> yegd yegD
<222> 1207-1306
<223> yegd yegD U00096 2145633..2147048
<313> 9529: 1247-1266
<400> 9529
CTGACGCGGA TTCTGGAACA GGTGCAACTG GCCCTGGATA ACGCCCAGGA 50
AAAACCGGAC GTTATCTACC TGACCGGCGG TAGCGCCCGT TCTCCGCTGA 100
<210> 9530
<211> 100
<212> DNA
<213> Escherichia coli K-12 MG1655
<220> yege b2068
<222> 3167-3266
<223> yege b2068 U00096 2141288..2144605
<313> 9530: 3203-3222
<400> 9530
TCGGGATGAA AACCATCGCC GGGCCAGTCG TTTTACCCTT AGTGATGGAT 50
ACGCTTTCTG GCATCGGCGT CGATCTGATT TATGGTGAGG TGATTGCCGA 100
<210> 9531
<211> 100
<212> DNA
<213> Escherichia coli K-12 MG1655
<220> yege b2068
<222> 3131-3230
<223> yege b2068 U00096 2141288..2144605
<313> 9531: 3165-3184
<400> 9531
TGCTGATTAC GATTATTCAG GGGCACGCTC AGCGACTCGG GATGAAAACC 50
ATCGCCGGGC CAGTCGTTTT ACCCTTAGTG ATGGATACGC TTTCTGGCAT 100
<210> 9532
<211> 100
<212> DNA
<213> Escherichia coli K-12 MG1655
<220> yege b2068
<222> 3082-3181
<223> yege b2068 U00096 2141288..2144605
<313> 9532: 3103-3122
<400> 9532
CTTGATGGTG AGTTATGCGC CAACGTGCAG GGTAATTTGA TGGATGAGAT 50
GCTGATTACG ATTATTCAGG GGCACGCTCA GCGACTCGGG ATGAAAACCA 100
<210> 9533
<211> 100
<212> DNA
<213> Escherichia coli K-12 MG1655
<220> folk b0143
<222> 349-448
<223> folk b0143 U00096 complement(157253..157732)
<313> 9533: 388-407
<400> 9533
TACGATATGA AGAATCGTGG ATTTATGCTG TGGCCGCTGT TTGAAATCGC 50
GCCGGAGTTG GTGTTTCCTG ATGGGGAGAT GTTGCGTCAA ATCTTACATA 100

<210> 9534
<211> 100
<212> DNA
<213> Escherichia coli K-12 MG1655
<220> folk b0143
<222> 304-403
<223> folk b0143 U00096 complement(157253..157732)
<313> 9534: 330-349
<400> 9534
TTTGGTAATG AAGTGATAAA TACTGAACGC CTGACCGTTC CGCACTACGA 50
TATGAAGAAT CGTGGATTTA TGCTGTGGCC GCTGTTTGAA ATCGCGCCGG 100
<210> 9535
<211> 100
<212> DNA
<213> Escherichia coli K-12 MG1655
<220> folk b0143
<222> 263-362
<223> folk b0143 U00096 complement(157253..157732)
<313> 9535: 296-315
<400> 9535
AACGCTGGGG ACCACGCACG CTGGATCTCG ACATCATGCT GTTTGGTAAT 50
GAAGTGATAA ATACTGAACG CCTGACCGTT CCGCACTACG ATATGAAGAA 100
<210> 9536
<211> 100
<212> DNA
<213> Escherichia coli K-12 MG1655
<220> cpsg b2049
<222> 1272-1371
<223> cpsg b2049 U00096 complement(2119631..2121001)
<313> 9536: 1340-1359
<400> 9536
CAATACCGAA CCGGTGGTGC GCCTGAATGT GGAATCGCGC GGTGATGTGC 50
CGCTGATGGA AGCGCGAACG CGAACTCTGC TGACGTTGCT GAACGAGTAA 100
<210> 9537
<211> 100
<212> DNA
<213> Escherichia coli K-12 MG1655
<220> cpsg b2049
<222> 1192-1291
<223> cpsg b2049 U00096 complement(2119631..2121001)
<313> 9537: 1228-1247
<400> 9537
AGCCGTGAGG CGCTGGCGGT GGATCGCACC GATGGCATCA GCATGACCTT 50
TGCCGACTGG CGCTTTAACC TGCGCACCTC CAATACCGAA CCGGTGGTGC 100
<210> 9538
<211> 100
<212> DNA
<213> Escherichia coli K-12 MG1655
<220> cpsg b2049
<222> 1098-1197
<223> cpsg b2049 U00096 complement(2119631..2121001)
<313> 9538: 1148-1167
<400> 9538
ACGCGACCGG ATGGCGGCGT TTCCGGCAAG CGGTGAGATC AACAGCAAAC 50
TGGCGCAACC CGTTGAGGCG ATTAACCGCG TGGAACAGCA TTTTAGCCGT 100
<210> 9539
<211> 100
<212> DNA
<213> Escherichia coli K-12 MG1655
<220> yegh b2064
<222> 1551-1650
<223> yegh b2064 U00096 2135858..2137507
<313> 9539: 1625-1644
<400> 9539
GGATTATTTG CTTAAAACGT TGCAGGTAGA AAGCCATCGC GTGCAGAAGG 50
TGCAGATTAT ACCGCTGCGT AAGGATGGCG AGATGGAGTA CGAGGTGTGA 100
<210> 9540
<211> 100
<212> DNA
<213> Escherichia coli K-12 MG1655
<220> yegh b2064

<222> 1551-1650
<223> yegh b2064 U00096 2135858..2137507
<313> 9540: 1583-1602
<400> 9540
GGATTATTTG CTTAAAACGT TGCAGGTAGA AAGCCATCGC GTGCAGAAGG  50
TGCAGATTAT ACCGCTGCGT AAGGATGGCG AGATGGAGTA CGAGGTGTGA  100
<210> 9541
<211> 100
<212> DNA
<213> Escherichia coli K-12 MG1655
<220> yegh b2064
<222> 1489-1588
<223> yegh b2064 U00096 2135858..2137507
<313> 9541: 1543-1562
<400> 9541
GCCGGGCTGT TGATGGAGTA TTTGCAGCGT ATTCCAAAAC CTGGCGAAGA  50
AGTTCAGGTG GGGGATTATT TGCTTAAAAC GTTGCAGGTA GAAAGCCATC  100
<210> 9542
<211> 100
<212> DNA
<213> Escherichia coli K-12 MG1655
<220> folp b3178
<222> 795-894
<223> folp b3178 U00096 complement(3321704..3322597)
<313> 9542: 867-886
<400> 9542
CGCGCACATC ATTCGTGTTC ATGACGTCAA AGAAACCGTA GAAGCGATGC  50
GGGTGGTGGA AGCCACTCTG TCTGCAAAGG AAAACAAACG CTATGAGTAA  100
<210> 9543
<211> 100
<212> DNA
<213> Escherichia coli K-12 MG1655
<220> folp b3178
<222> 699-798
<223> folp b3178 U00096 complement(3321704..3322597)
<313> 9543: 748-767
<400> 9543
GTCACGAAAA TCGATGATTG GGCAGCTGCT GAACGTGGGG CCGTCCGAGC  50
GCCTGAGCGG TAGTCTGGCC TGTGCGGTCA TTGCCGCAAT GCAAGGCGCG  100
<210> 9544
<211> 100
<212> DNA
<213> Escherichia coli K-12 MG1655
<220> folp b3178
<222> 638-737
<223> folp b3178 U00096 complement(3321704..3322597)
<313> 9544: 687-706
<400> 9544
ATTCATTACT GGCGCGCCTG GCTGAATTTC ACCATTTCAA CCTGCCGCTG  50
TTGGTGGGTA TGTCACGAAA ATCGATGATT GGGCAGCTGC TGAACGTGGG  100
<210> 9545
<211> 100
<212> DNA
<213> Escherichia coli K-12 MG1655
<220> yegn b2076
<222> 3024-3123
<223> yegn b2076 U00096 2153285..2156407
<313> 9545: 3100-3119
<400> 9545
GGTGCTGACG CTGTTTACCA CGCCGGTGAT TTATTTGCTG TTCGACCGCC  50
TGGCATTGTG GACCAAAAGC CGCTTTGCCC GTCATGAAGA GGAGGCGTAA  100
<210> 9546
<211> 100
<212> DNA
<213> Escherichia coli K-12 MG1655
<220> yegn b2076
<222> 2954-3053
<223> yegn b2076 U00096 2153285..2156407
<313> 9546: 3001-3020
<400> 9546
GTACCGGGGT CGGCGCGGAA CTGCGTCGTC CGTTAGGTAT CGGCATGGTC  50

GGCGGTCTGA TTGTCAGCCA GGTGCTGACG CTGTTTACCA CGCCGGTGAT 100
<210> 9547
<211> 100
<212> DNA
<213> Escherichia coli K-12 MG1655
<220> yegn b2076
<222> 2847-2946
<223> yegn b2076 U00096 2153285..2156407
<313> 9547: 2908-2927
<400> 9547
AGGCATGTCG CCGCGCGAGG CAATCTACCA GGCTTGTCTG TTGCGTTTTC 50
GTCCGATCCT GATGACCACT CTGGCGGCTC TGCTTGGCGC GCTGCCGCTG 100
<210> 9548
<211> 100
<212> DNA
<213> Escherichia coli K-12 MG1655
<220> yego b2077
<222> 2979-3078
<223> yego b2077 U00096 2156408..2159485
<313> 9548: 3050-3069
<400> 9548
CCAGCTCCTT ACGCTGTATA CCACGCCGGT GGTGTATCTC TTTTTCGACC 50
GTCTGCGGCT GCGTTTTTCG CGTAAACCTA AACAAACGGT AACCGAGTAA 100
<210> 9549
<211> 100
<212> DNA
<213> Escherichia coli K-12 MG1655
<220> yego b2077
<222> 2925-3024
<223> yego b2077 U00096 2156408..2159485
<313> 9549: 2971-2990
<400> 9549
CTCGGAGCTG CGGCAACCCC TGGGGATCAC CATTGTCGGC GGACTGGTAA 50
TGAGCCAGCT CCTTACGCTG TATACCACGC CGGTGGTGTA TCTCTTTTTC 100
<210> 9550
<211> 100
<212> DNA
<213> Escherichia coli K-12 MG1655
<220> yego b2077
<222> 2818-2917
<223> yego b2077 U00096 2156408..2159485
<313> 9550: 2841-2860
<400> 9550
CAGGAAGCTA TTTTCCAGGC CTGTCTGCTG CGTTTTCGCC CGATTATGAT 50
GACTACCCTG GCGGCGCTGT TTGGTGCGCT GCCGCTGGTA TTGTCGGGCG 100
<210> 9551
<211> 100
<212> DNA
<213> Escherichia coli K-12 MG1655
<220> yegq b2082
<222> 1230-1329
<223> yegq b2082 U00096 2163690..2165051
<313> 9551: 1261-1280
<400> 9551
CGAAGCTATG CCGATAGCAC CAGGCGATGG TTATACTGTG TGGCTCCCGG 50
TCCCGCAGGA TCTTGAGCTC AATTACGCGC TGCTGATGCG TAATTTCTCC 100
<210> 9552
<211> 100
<212> DNA
<213> Escherichia coli K-12 MG1655
<220> yegq b2082
<222> 1193-1292
<223> yegq b2082 U00096 2163690..2165051
<313> 9552: 1214-1233
<400> 9552
TTAATTTTAC CCTTGAGCAC ATGGAAAACG CCAAAGGCGA AGCTATGCCG 50
ATAGCACCAG GCGATGGTTA TACTGTGTGG CTCCCGGTCC CGCAGGATCT 100
<210> 9553
<211> 100
<212> DNA
<213> Escherichia coli K-12 MG1655

<220> yegq b2082
<222> 1140-1239
<223> yegq b2082 U00096 2163690..2165051
<313> 9553: 1183-1202
<400> 9553
AAATAAATTC TCCGTTGGCG ACAGCCTTGA GCTGATGACG CCGCAAGGCA 50
ACATTAATTT TACCCTTGAG CACATGGAAA ACGCCAAAGG CGAAGCTATG 100
<210> 9554
<211> 100
<212> DNA
<213> Escherichia coli K-12 MG1655
<220> cara b0033
<222> 1050-1149
<223> cara b0033 U00096 29651..30799
<313> 9554: 1097-1116
<400> 9554
CCAGGGGCAC CCTGAAGCCA GCCCTGGTCC ACACGACGCC GCGCCGTTGT 50
TCGACCACTT TATCGAGTTA ATTGAGCAGT ACCGTAAAAC CGCTAAGTAA 100
<210> 9555
<211> 100
<212> DNA
<213> Escherichia coli K-12 MG1655
<220> cara b0033
<222> 993-1092
<223> cara b0033 U00096 29651..30799
<313> 9555: 1034-1053
<400> 9555
CCTGTTCGAC GGTACGTTAC AGGGCATTCA TCGCACCGAT AAACCGGCAT 50
TCAGCTTCCA GGGGCACCCT GAAGCCAGCC CTGGTCCACA CGACGCCGCG 100
<210> 9556
<211> 100
<212> DNA
<213> Escherichia coli K-12 MG1655
<220> cara b0033
<222> 911-1010
<223> cara b0033 U00096 29651..30799
<313> 9556: 957-976
<400> 9556
TGGTAATGAT CACCGCCCAG AACCACGGTT TTGCGGTGGA CGAAGCAACA 50
TTACCTGCAA ACCTGCGTGT CACGCATAAA TCCCTGTTCG ACGGTACGTT 100
<210> 9557
<211> 100
<212> DNA
<213> Escherichia coli K-12 MG1655
<220> carb b0034
<222> 3123-3222
<223> carb b0034 U00096 30817..34038
<313> 9557: 3188-3207
<400> 9557
CACCACCCTG AACGGCGGCT TTGCCACCGC GATGGCGCTG AATGCCGATG 50
CGACTGAAAA AGTAATTTCG GTGCAGGAAA TGCACGCACA GATCAAATAA 100
<210> 9558
<211> 100
<212> DNA
<213> Escherichia coli K-12 MG1655
<220> carb b0034
<222> 3102-3201
<223> carb b0034 U00096 30817..34038
<313> 9558: 3126-3145
<400> 9558
GCAATATAAA GTGCATTACG ACACCACCCT GAACGGCGGC TTTGCCACCG 50
CGATGGCGCT GAATGCCGAT GCGACTGAAA AAGTAATTTC GGTGCAGGAA 100
<210> 9559
<211> 100
<212> DNA
<213> Escherichia coli K-12 MG1655
<220> carb b0034
<222> 2977-3076
<223> carb b0034 U00096 30817..34038
<313> 9559: 3041-3060
<400> 9559

AAGGTGCATG AAGGCCGTCC GCACATTCAG GACCGTATCA AGAATGGCGA 50
ATATACCTAC ATCATCAACA CCACCTCAGG CCGTCGTGCG ATTGAAGACT 100
<210> 9560
<211> 100
<212> DNA
<213> Escherichia coli K-12 MG1655
<220> folx folX
<222> 264-363
<223> folx folX U00096 2419345..2419707
<313> 9560: 332-351
<400> 9560
TGAACATCAC TGGGTGACGT ATGCTGAAGT GGAGATCGAT AAACTGCACG 50
CGCTGCGCTA CGCCGATTCG GTATCCATGA CCTTAAGCTG GCAGCGTTAA 100
<210> 9561
<211> 100
<212> DNA
<213> Escherichia coli K-12 MG1655
<220> folx folX
<222> 263-362
<223> folx folX U00096 2419345..2419707
<313> 9561: 290-309
<400> 9561
GTGAACATCA CTGGGTGACG TATGCTGAAG TGGAGATCGA TAAACTGCAC 50
GCGCTGCGCT ACGCCGATTC GGTATCCATG ACCTTAAGCT GGCAGCGTTA 100
<210> 9562
<211> 100
<212> DNA
<213> Escherichia coli K-12 MG1655
<220> folx folX
<222> 125-224
<223> folx folX U00096 2419345..2419707
<313> 9562: 162-181
<400> 9562
CCGATAAAGC GCGCACTAGC GAAGATATCA ACGATGCGCT GAATTATCGC 50
ACCGTAACGA AAAACATTAT TCAGCATGTA GAGAATAACC GTTTCTCTTT 100
<210> 9563
<211> 100
<212> DNA
<213> Escherichia coli K-12 MG1655
<220> yegt yegT
<222> 1179-1278
<223> yegt yegT U00096 2176841..2178118
<313> 9563: 1214-1233
<400> 9563
CGTGCTGTTC ATGATTTTTT TCCGCGAATC CGACAACGAA ATTACGGCTA 50
TCAAGGTCGA TGATCGCGAT ATTGCGTTGA CACAAGGGGA AGTTAAATGA 100
<210> 9564
<211> 100
<212> DNA
<213> Escherichia coli K-12 MG1655
<220> yegt yegT
<222> 1122-1221
<223> yegt yegT U00096 2176841..2178118
<313> 9564: 1173-1192
<400> 9564
GACTTTCAAC TGGTCCGGGA TGTGGACTTT CGGCGCGGTG ATGATTGCCA 50
TTATCGCCGT GCTGTTCATG ATTTTTTTCC GCGAATCCGA CAACGAAATT 100
<210> 9565
<211> 100
<212> DNA
<213> Escherichia coli K-12 MG1655
<220> yegt yegT
<222> 1097-1196
<223> yegt yegT U00096 2176841..2178118
<313> 9565: 1143-1162
<400> 9565
CTTATCAGGA ACCGGTAAAC GGACTGACTT TCAACTGGTC CGGGATGTGG 50
ACTTTCGGCG CGGTGATGAT TGCCATTATC GCCGTGCTGT TCATGATTTT 100
<210> 9566
<211> 100
<212> DNA

<213> Escherichia coli K-12 MG1655
<220> yegw b2102
<222> 648-747
<223> yegw b2102 U00096 complement(2180055..2180801)
<313> 9566: 707-726
<400> 9566
GGTGATCAAG CAAGTGGCGC TTGACCAACA GCAACGGCCT ATTGAGTACA 50
GCATTAGCCA CTGTCGCAGC GATCTATACG TTTTTGTGTG CGAGGAGTAG 100
<210> 9567
<211> 100
<212> DNA
<213> Escherichia coli K-12 MG1655
<220> yegw b2102
<222> 578-677
<223> yegw b2102 U00096 complement(2180055..2180801)
<313> 9567: 602-621
<400> 9567
GCGTTAGCGC CCGGATGCCG GATGCCGAGT TTCAGTCACA TATTCAGTTA 50
GATAGCAAAA TACCGGTGCT GGTGATCAAG CAAGTGGCGC TTGACCAACA 100
<210> 9568
<211> 100
<212> DNA
<213> Escherichia coli K-12 MG1655
<220> yegw b2102
<222> 488-587
<223> yegw b2102 U00096 complement(2180055..2180801)
<313> 9568: 533-552
<400> 9568
CGCATTTAAT TCATGATGTT GATGCCATCG GGATTTCACT TTATGACTAT 50
TTCCGCAGCC AGCACATTTA CCCACAACGT ACGCGTTCCC GCGTTAGCGC 100
<210> 9569
<211> 100
<212> DNA
<213> Escherichia coli K-12 MG1655
<220> yegx b2103
<222> 729-828
<223> yegx b2103 U00096 complement(2180853..2181680)
<313> 9569: 788-807
<400> 9569
ACAGGTAGAT GGCATCAATG GTCCGGTGGA TTTTAATGTA TTTAATGGCA 50
CGGTGGAGGA GTTGCAGGCA TTCGTTGATG GGATTAAAGA AACGCCTTAA 100
<210> 9570
<211> 100
<212> DNA
<213> Escherichia coli K-12 MG1655
<220> yegx b2103
<222> 713-812
<223> yegx b2103 U00096 complement(2180853..2181680)
<313> 9570: 734-753
<400> 9570
AGCATTCCGA CCGTGGACAG GTAGATGGCA TCAATGGTCC GGTGGATTTT 50
AATGTATTTA ATGGCACGGT GGAGGAGTTG CAGGCATTCG TTGATGGGAT 100
<210> 9571
<211> 100
<212> DNA
<213> Escherichia coli K-12 MG1655
<220> yegx b2103
<222> 593-692
<223> yegx b2103 U00096 complement(2180853..2181680)
<313> 9571: 634-653
<400> 9571
ACTCAGGAGC CGTTTTTTAT CACACCAATC TGGCGGGCTA TTTCAATGAA 50
TATCCGTGGT GGGTGGCTCA CTACTATCAA CGTCGTCCGG ACAATGACGG 100
<210> 9572
<211> 100
<212> DNA
<213> Escherichia coli K-12 MG1655
<220> infa b0885
<222> 120-219
<223> infa b0885 U00096 complement(925448..925666)
<313> 9572: 188-207

<400> 9572
GCGCAAAAAC TACATCCGCA TCCTGACGGG CGACAAAGTG ACTGTTGAAC 50
TGACCCCGTA CGACCTGAGC AAAGGCCGCA TTGTCTTCCG TAGTCGCTGA 100
<210> 9573
<211> 100
<212> DNA
<213> Escherichia coli K-12 MG1655
<220> infa b0885
<222> 93-192
<223> infa b0885 U00096 complement(925448..925666)
<313> 9573: 146-165
<400> 9573
GGTTACTGCA CACATCTCCG GTAAAATGCG CAAAAACTAC ATCCGCATCC 50
TGACGGGCGA CAAAGTGACT GTTGAACTGA CCCCGTACGA CCTGAGCAAA 100
<210> 9574
<211> 100
<212> DNA
<213> Escherichia coli K-12 MG1655
<220> infa b0885
<222> 31-130
<223> infa b0885 U00096 complement(925448..925666)
<313> 9574: 53-72
<400> 9574
GGTACCGTTC TTGAAACGTT GCCTAATACC ATGTTCCGCG TAGAGTTAGA 50
AAACGGTCAC GTGGTTACTG CACACATCTC CGGTAAAATG CGCAAAAACT 100
<210> 9575
<211> 100
<212> DNA
<213> Escherichia coli K-12 MG1655
<220> infb b3169
<222> 2574-2673
<223> infb b3169 U00096 complement(3310983..3313655)
<313> 9575: 2618-2637
<400> 9575
TATGGAATGT GGTATCGGCG TTAAGAACTA CAACGACGTC CGCACTGGCG 50
ATGTGATCGA AGTATTCGAA ATCATCGAGA TCCAACGTAC CATTGCTTAA 100
<210> 9576
<211> 100
<212> DNA
<213> Escherichia coli K-12 MG1655
<220> infb b3169
<222> 2556-2655
<223> infb b3169 U00096 complement(3310983..3313655)
<313> 9576: 2575-2594
<400> 9576
TAACGAAGTC CGTAACGGTA TGGAATGTGG TATCGGCGTT AAGAACTACA 50
ACGACGTCCG CACTGGCGAT GTGATCGAAG TATTCGAAAT CATCGAGATC 100
<210> 9577
<211> 100
<212> DNA
<213> Escherichia coli K-12 MG1655
<220> infb b3169
<222> 2499-2598
<223> infb b3169 U00096 complement(3310983..3313655)
<313> 9577: 2526-2545
<400> 9577
CAACGTGGTT ATCTACGAAG GCGAGCTGGA GTCCCTGCGC CGCTTCAAAG 50
ATGACGTTAA CGAAGTCCGT AACGGTATGG AATGTGGTAT CGGCGTTAAG 100
<210> 9578
<211> 100
<212> DNA
<213> Escherichia coli K-12 MG1655
<220> infc b1719
<222> 444-543
<223> infc b1719 U00096 complement(1798120..1798662)
<313> 9578: 486-505
<400> 9578
GAAAGACGAT TTGCAAGAAC TGGCAGTGGT CGAATCCTTC CCAACGAAGA 50
TCGAAGGCCG CCAGATGATC ATGGTGCTCG CTCCTAAGAA GAAACAGTAA 100
<210> 9579
<211> 100

<212> DNA
<213> Escherichia coli K-12 MG1655
<220> infc b1719
<222> 379-478
<223> infc b1719 U00096 complement(1798120..1798662)
<313> 9579: 407-426
<400> 9579
ACGCTGCGTT TCCGCGGTCG TGAGATGGCG CACCAGCAAA TCGGTATGGA 50
AGTGCTTAAT CGCGTGAAAG ACGATTTGCA AGAACTGGCA GTGGTCGAAT 100
<210> 9580
<211> 100
<212> DNA
<213> Escherichia coli K-12 MG1655
<220> infc b1719
<222> 338-437
<223> infc b1719 U00096 complement(1798120..1798662)
<313> 9580: 369-388
<400> 9580
GCCTGATTCG CTTTCTCGAA GAGGGTGATA AAGCCAAAAT CACGCTGCGT 50
TTCCGCGGTC GTGAGATGGC GCACCAGCAA ATCGGTATGG AAGTGCTTAA 100
<210> 9581
<211> 100
<212> DNA
<213> Escherichia coli K-12 MG1655
<220> glpa b2242
<222> 1530-1629
<223> glpa b2242 U00096 2350667..2352295
<313> 9581: 1609-1628
<400> 9581
ACCCATCGCC TGGGGAGATG CACTGCGCGA AAGCGAATTT ACCCGCTGGG 50
TTTATCAGGG ATTGTGTGGT CTGGAGAAGG AGCAGAAAGA TGCGCTTTGA 100
<210> 9582
<211> 100
<212> DNA
<213> Escherichia coli K-12 MG1655
<220> glpa b2242
<222> 1530-1629
<223> glpa b2242 U00096 2350667..2352295
<313> 9582: 1552-1571
<400> 9582
ACCCATCGCC TGGGGAGATG CACTGCGCGA AAGCGAATTT ACCCGCTGGG 50
TTTATCAGGG ATTGTGTGGT CTGGAGAAGG AGCAGAAAGA TGCGCTTTGA 100
<210> 9583
<211> 100
<212> DNA
<213> Escherichia coli K-12 MG1655
<220> glpa b2242
<222> 1482-1581
<223> glpa b2242 U00096 2350667..2352295
<313> 9583: 1528-1547
<400> 9583
TATCGAGCAA CTTTCCACCT TCCTTAACGA ACGCTGGAAA GGCGTGCAAC 50
CCATCGCCTG GGGAGATGCA CTGCGCGAAA GCGAATTTAC CCGCTGGGTT 100
<210> 9584
<211> 100
<212> DNA
<213> Escherichia coli K-12 MG1655
<220> glpb b2243
<222> 1161-1260
<223> glpb b2243 U00096 2352285..2353544
<313> 9584: 1234-1253
<400> 9584
ATTTGATCCC ATCGCCCAGG GATGCGGCGG CGGTGTTTGT GCCGTCAGTG 50
CTTTACATGC CGCTCAACAG ATTGCCCAAC GCGCAGGAGG CCAACAATGA 100
<210> 9585
<211> 100
<212> DNA
<213> Escherichia coli K-12 MG1655
<220> glpb b2243
<222> 1069-1168
<223> glpb b2243 U00096 2352285..2353544

<313> 9585: 1104-1123
<400> 9585
CAGTTCGGTG TAACCACTGA TGAGACGCTA CGCCCGTCAC AGGCAGGGCA 50
AACCATTGAA AACCTGTTTG CCATCGGTTC GGTGCTGGGC GGATTTGATC 100
<210> 9586
<211> 100
<212> DNA
<213> Escherichia coli K-12 MG1655
<220> glpb b2243
<222> 1014-1113
<223> glpb b2243 U00096 2352285..2353544
<313> 9586: 1073-1092
<400> 9586
CGCCACGCGG GGTGAATGGT ATAAGGGAGA TTTTTTTGCG CCGCAACCGT 50
GGCAGCAGTT CGGTGTAACC ACTGATGAGA CGCTACGCCC GTCACAGGCA 100
<210> 9587
<211> 100
<212> DNA
<213> Escherichia coli K-12 MG1655
<220> hisa b2025
<222> 642-741
<223> hisa b2025 U00096 2093144..2093884
<313> 9587: 692-711
<400> 9587
CCTGCGTGGC ACTGGTGTGC GCGGCGTAAT AGTTGGTCGG GCATTACTGG 50
AAGGTAAATT CACCGTGAAG GAGGCCATCG CATGCTGGCA AAACGCATAA 100
<210> 9588
<211> 100
<212> DNA
<213> Escherichia coli K-12 MG1655
<220> hisa b2025
<222> 566-665
<223> hisa b2025 U00096 2093144..2093884
<313> 9588: 602-621
<400> 9588
AAGAAGTGTG CGCCAGATAT CCGCAGGTGG CATTTCAGTC CTCCGGCGGT 50
ATTGGCGACA TTGATGATGT GGCGGCCCTG CGTGGCACTG GTGTGCGCGG 100
<210> 9589
<211> 100
<212> DNA
<213> Escherichia coli K-12 MG1655
<220> hisa b2025
<222> 492-591
<223> hisa b2025 U00096 2093144..2093884
<313> 9589: 540-559
<400> 9589
CGGCCTGAAA CATGTGCTGT GTACCGATAT CTCGCGCGAC GGCACGCTGG 50
CAGGCTCTAA CGTCTCTTTA TATGAAGAAG TGTGCGCCAG ATATCCGCAG 100
<210> 9590
<211> 100
<212> DNA
<213> Escherichia coli K-12 MG1655
<220> glpc b2244
<222> 1092-1191
<223> glpc b2244 U00096 2353541..2354731
<313> 9590: 1162-1181
<400> 9590
GGTGGTCACC GACTGCGAAA CCTGTAAATG GCAGATTGAG ATGTCCACAA 50
GTCTTCGCTG CGAACATCCG ATTACGCTAC TGGCCCAGGC GCTGGCTTAA 100
<210> 9591
<211> 100
<212> DNA
<213> Escherichia coli K-12 MG1655
<220> glpc b2244
<222> 1069-1168
<223> glpc b2244 U00096 2353541..2354731
<313> 9591: 1115-1134
<400> 9591
ATAGAAGAAA GCGGCGCAGA TCTGGTGGTC ACCGACTGCG AAACCTGTAA 50
ATGGCAGATT GAGATGTCCA CAAGTCTTCG CTGCGAACAT CCGATTACGC 100
<210> 9592

<211> 100
<212> DNA
<213> Escherichia coli K-12 MG1655
<220> glpc b2244
<222> 1014-1113
<223> glpc b2244 U00096 2353541..2354731
<313> 9592: 1051-1070
<400> 9592
CAAAAAAGAG AACTACCCCA CCTCACAAGC CATCGGCGCA CCACTGTTCC 50
GCCAGATAGA AGAAAGCGGC GCAGATCTGG TGGTCACCGA CTGCGAAACC 100
<210> 9593
<211> 100
<212> DNA
<213> Escherichia coli K-12 MG1655
<220> hisb b2023
<222> 972-1071
<223> hisb b2023 U00096 2091487..2092557
<313> 9593: 1041-1060
<400> 9593
TCACCGTGTA GAGAGTCTGT TCAAAGCCTT TGGTCGCACC CTGCGCCAGG 50
CCATCCGCGT GGAAGGCGAT ACCCTGCCCT CGTCGAAAGG AGTGCTGTAA 100
<210> 9594
<211> 100
<212> DNA
<213> Escherichia coli K-12 MG1655
<220> hisb b2023
<222> 972-1071
<223> hisb b2023 U00096 2091487..2092557
<313> 9594: 993-1012
<400> 9594
TCACCGTGTA GAGAGTCTGT TCAAAGCCTT TGGTCGCACC CTGCGCCAGG 50
CCATCCGCGT GGAAGGCGAT ACCCTGCCCT CGTCGAAAGG AGTGCTGTAA 100
<210> 9595
<211> 100
<212> DNA
<213> Escherichia coli K-12 MG1655
<220> hisb b2023
<222> 893-992
<223> hisb b2023 U00096 2091487..2092557
<313> 9595: 953-972
<400> 9595
TGATCGAGCA CTTCTTCCGT TCGCTCTCAT ACACCATGGG CGTGACGCTA 50
CACCTGAAAA CCAAAGGTAA AAACGATCAT CACCGTGTAG AGAGTCTGTT 100
<210> 9596
<211> 100
<212> DNA
<213> Escherichia coli K-12 MG1655
<220> glpd b3427
<222> 1407-1506
<223> glpd b3427 U00096 3559646..3561151
<313> 9596: 1465-1484
<400> 9596
TCGCACAAAA CAAGGCATGT GGCTAAATGC GGATCAACAA TCTCGTGTGA 50
GTCAGTGGCT GGTGGAGTAT ACGCAGCAGA GGTTATCGCT GGCGTCGTAA 100
<210> 9597
<211> 100
<212> DNA
<213> Escherichia coli K-12 MG1655
<220> glpd b3427
<222> 1375-1474
<223> glpd b3427 U00096 3559646..3561151
<313> 9597: 1419-1438
<400> 9597
TGGGTCCGCC GCGCCGACGA CGCCCTGTGG CGTCGCACAA AACAAGGCAT 50
GTGGCTAAAT GCGGATCAAC AATCTCGTGT GAGTCAGTGG CTGGTGGAGT 100
<210> 9598
<211> 100
<212> DNA
<213> Escherichia coli K-12 MG1655
<220> glpd b3427
<222> 1317-1416

<223> glpd b3427 U00096 3559646..3561151
<313> 9598: 1349-1368
<400> 9598
GGAAGATTTC GGTCATGAGT TCTACGAAGC GGAGCTGAAA TACCTGGTGG 50
ATCACGAATG GGTCCGCCGC GCCGACGACG CCCTGTGGCG TCGCACAAAA 100
<210> 9599
<211> 100
<212> DNA
<213> Escherichia coli K-12 MG1655
<220> hisc b2022
<222> 972-1071
<223> hisc b2022 U00096 2090420..2091490
<313> 9599: 1027-1046
<400> 9599
GAATAAACAA CCCTCTTTAA GCGGCTGCCT GCGAATTACC GTCGGAACCC 50
GTGAAGAAAG CCAGCGCGTC ATTGACGCCT TACGTGCGGA GCAAGTTTGA 100
<210> 9600
<211> 100
<212> DNA
<213> Escherichia coli K-12 MG1655
<220> hisc b2022
<222> 906-1005
<223> hisc b2022 U00096 2090420..2091490
<313> 9600: 929-948
<400> 9600
GCGCTTTAAA GCCTCCAGTG CGGTGTTTAA ATCTTTGTGG GATCAGGGCA 50
TTATCTTACG TGATCAGAAT AAACAACCCT CTTTAAGCGG CTGCCTGCGA 100
<210> 9601
<211> 100
<212> DNA
<213> Escherichia coli K-12 MG1655
<220> hisc b2022
<222> 845-944
<223> hisc b2022 U00096 2090420..2091490
<313> 9601: 876-895
<400> 9601
CACTGAAAGA GATCCCCTGC GTAGAGCAGG TTTTCGACTC TGAAACCAAC 50
TACATTCTGG CGCGCTTTAA AGCCTCCAGT GCGGTGTTTA AATCTTTGTG 100
<210> 9602
<211> 100
<212> DNA
<213> Escherichia coli K-12 MG1655
<220> yeha b2109
<222> 936-1035
<223> yeha b2109 U00096 complement(2185400..2186434)
<313> 9602: 997-1016
<400> 9602
TTCAATTCCG ATAACAGCCA GTTACTACGT CTACGATACA AACAAAGTTA 50
AATCTGGCGC ACTGGAGGCA ACCGCATTAA TCAACGTGAA ATACGACTAA 100
<210> 9603
<211> 100
<212> DNA
<213> Escherichia coli K-12 MG1655
<220> yeha b2109
<222> 770-869
<223> yeha b2109 U00096 complement(2185400..2186434)
<313> 9603: 788-807
<400> 9603
TAACCAACAG CAACACAATA CTGAAAGGTG CGGAAGATAA TGGCGTAGGA 50
TTTATTCTTG AAAGTAATGG TTCGCCAGTC ACACTTTTAA ATATCACTAA 100
<210> 9604
<211> 100
<212> DNA
<213> Escherichia coli K-12 MG1655
<220> yeha b2109
<222> 593-692
<223> yeha b2109 U00096 complement(2185400..2186434)
<313> 9604: 630-649
<400> 9604
CGGACGATTT ATTAATTACT TTAGACAGTA TTCCCGTTTC ACAATTACCA 50
GCCACAGGTA ACAAAGCAAC AATAAATAGT AAACAAGGGG ATATTATTCT 100

<210> 9605
<211> 100
<212> DNA
<213> Escherichia coli K-12 MG1655
<220> glpe b3426
<222> 228-327
<223> glpe b3426 U00096 complement(3559130..3559456)
<313> 9605: 289-308
<400> 9605
GTATCTGCTG CAACAGGGCT ACGATGTGGT CTATAGCATT GACGGCGGCT 50
TTGAAGCCTG GCAACGTCAG TTTCCCGCAG AGGTGGCGTA CGGCGCGTAA 100
<210> 9606
<211> 100
<212> DNA
<213> Escherichia coli K-12 MG1655
<220> glpe b3426
<222> 203-302
<223> glpe b3426 U00096 complement(3559130..3559456)
<313> 9606: 221-240
<400> 9606
GCAATAGCAG CAAAGGCGCG GCGCAGTATC TGCTGCAACA GGGCTACGAT 50
GTGGTCTATA GCATTGACGG CGGCTTTGAA GCCTGGCAAC GTCAGTTTCC 100
<210> 9607
<211> 100
<212> DNA
<213> Escherichia coli K-12 MG1655
<220> glpe b3426
<222> 107-206
<223> glpe b3426 U00096 complement(3559130..3559456)
<313> 9607: 175-194
<400> 9607
ATGCGGTGCA GGCTTTCCAT TTAACCAACG ACACGCTGGG CGCTTTTATG 50
CGTGATAACG ACTTTGACAC TCCGGTGATG GTGATGTGTT ATCACGGCAA 100
<210> 9608
<211> 100
<212> DNA
<213> Escherichia coli K-12 MG1655
<220> hisd b2021
<222> 1202-1301
<223> hisd b2021 U00096 2089119..2090423
<313> 9608: 1252-1271
<400> 9608
CCGCGCTGGC TTCAACCATA GAAACACTGG CCGCCGCCGA GCGCCTGACC 50
GCCCACAAAA ATGCCGTTAC TTTGCGTGTT AACGCCCTTA AGGAGCAAGC 100
<210> 9609
<211> 100
<212> DNA
<213> Escherichia coli K-12 MG1655
<220> hisd b2021
<222> 1178-1277
<223> hisd b2021 U00096 2089119..2090423
<313> 9609: 1211-1230
<400> 9609
AGGAACTGTC GAAAGAGGGG TTCTCCGCGC TGGCTTCAAC CATAGAAACA 50
CTGGCCGCCG CCGAGCGCCT GACCGCCCAC AAAAATGCCG TTACTTTGCG 100
<210> 9610
<211> 100
<212> DNA
<213> Escherichia coli K-12 MG1655
<220> hisd b2021
<222> 1132-1231
<223> hisd b2021 U00096 2089119..2090423
<313> 9610: 1169-1188
<400> 9610
TGTTCCAGCC TCGGGCTGGC AGATTTCCAG AAGCGCATGA CCGTACAGGA 50
ACTGTCGAAA GAGGGGTTCT CCGCGCTGGC TTCAACCATA GAAACACTGG 100
<210> 9611
<211> 100
<212> DNA
<213> Escherichia coli K-12 MG1655
<220> yehb b2110

<222> 2382-2481
<223> yehb b2110 U00096 complement(2186450..2188930)
<313> 9611: 2422-2441
<400> 9611
GCCATCGGTT AATGTGGCAA TTGATAAGCA ACAAGGACTT TCATGCACAA  50
TCACCTTCGG TAAAGAGATT GATGAAAGTA GAAATTATAT TTGCCAGTAA  100
<210> 9612
<211> 100
<212> DNA
<213> Escherichia coli K-12 MG1655
<220> yehb b2110
<222> 2352-2451
<223> yehb b2110 U00096 complement(2186450..2188930)
<313> 9612: 2369-2388
<400> 9612
TCAGTTATTT ATTCGCACCA ATGAAGTACC GCCATCGGTT AATGTGGCAA  50
TTGATAAGCA ACAAGGACTT TCATGCACAA TCACCTTCGG TAAAGAGATT  100
<210> 9613
<211> 100
<212> DNA
<213> Escherichia coli K-12 MG1655
<220> yehb b2110
<222> 2279-2378
<223> yehb b2110 U00096 complement(2186450..2188930)
<313> 9613: 2341-2360
<400> 9613
GGCAATCATT AACGTTTGGT TATGAAGTCA ATGATATCCA TGGTCATAAT  50
ATTGGCGTTG TCGGCCAGGG AAGTCAGTTA TTTATTCGCA CCAATGAAGT  100
<210> 9614
<211> 100
<212> DNA
<213> Escherichia coli K-12 MG1655
<220> glpf b3928
<222> 747-846
<223> glpf b3928 U00096 complement(4114825..4115670)
<313> 9614: 822-841
<400> 9614
TGCCTACCGC AAACTGATTG GTCGCCATTT GCCTTGCGAT ATCTGTGTTG  50
TGGAAGAAAA GGAAACCACA ACTCCTTCAG AACAAAAAGC TTCGCTGTAA  100
<210> 9615
<211> 100
<212> DNA
<213> Escherichia coli K-12 MG1655
<220> glpf b3928
<222> 745-844
<223> glpf b3928 U00096 complement(4114825..4115670)
<313> 9615: 773-792
<400> 9615
TTTGCCTACC GCAAACTGAT TGGTCGCCAT TTGCCTTGCG ATATCTGTGT  50
TGTGGAAGAA AAGGAAACCA CAACTCCTTC AGAACAAAAA GCTTCGCTGT  100
<210> 9616
<211> 100
<212> DNA
<213> Escherichia coli K-12 MG1655
<220> glpf b3928
<222> 657-756
<223> glpf b3928 U00096 complement(4114825..4115670)
<313> 9616: 670-689
<400> 9616
GGGCAATGTC GCCTTTACCG GCGGCAGAGA CATTCCTTAC TTCCTGGTGC  50
CGCTTTTCGG CCCTATCGTT GGCGCGATTG TAGGTGCATT TGCCTACCGC  100
<210> 9617
<211> 100
<212> DNA
<213> Escherichia coli K-12 MG1655
<220> yehc b2111
<222> 462-561
<223> yehc b2111 U00096 complement(2188946..2189665)
<313> 9617: 518-537
<400> 9617
GGGTATTGCT CCGGTAAATA AAGCGACATT TAAAAAATTG CTGGTAAATC  50

GCAGTGGCAA TGGTTTGGTG ATAAAAAATG ACTCAGCTAA TTGGGTGACG 100
<210> 9618
<211> 100
<212> DNA
<213> Escherichia coli K-12 MG1655
<220> yehc b2111
<222> 418-517
<223> yehc b2111 U00096 complement(2188946..2189665)
<313> 9618: 457-476
<400> 9618
AAGTTTGCGA TGCAAAACAG AATTAAGTTG TTTTACCGGC CAGCGGGTAT 50
TGCTCCGGTA AATAAAGCGA CATTTAAAAA ATTGCTGGTA AATCGCAGTG 100
<210> 9619
<211> 100
<212> DNA
<213> Escherichia coli K-12 MG1655
<220> yehc b2111
<222> 357-456
<223> yehc b2111 U00096 complement(2188946..2189665)
<313> 9619: 401-420
<400> 9619
TTATCTGAAT GTTCTGGATA TTCCACCAAA TAGTCCAGAG CAAGAAGGTA 50
AGAATGCACT GAAGTTTGCG ATGCAAAACA GAATTAAGTT GTTTTACCGG 100
<210> 9620
<211> 100
<212> DNA
<213> Escherichia coli K-12 MG1655
<220> glpg b3425
<222> 732-831
<223> glpg b3425 U00096 complement(3558255..3559085)
<313> 9620: 803-822
<400> 9620
GTTTGGGATG TCGATGGCGA ACGGAGCACA CATCGCCGGG TTAGCCGTGG 50
GTTTAGCGAT GGCTTTTGTT GATTCGCTCA ATGCGCGAAA ACGAAAATAA 100
<210> 9621
<211> 100
<212> DNA
<213> Escherichia coli K-12 MG1655
<220> glpg b3425
<222> 679-778
<223> glpg b3425 U00096 complement(3558255..3559085)
<313> 9621: 708-727
<400> 9621
CGTGGGTTAA TTATCTTTGC GCTGATCTGG ATTGTCGCCG GATGGTTTGA 50
TTTGTTTGGG ATGTCGATGG CGAACGGAGC ACACATCGCC GGGTTAGCCG 100
<210> 9622
<211> 100
<212> DNA
<213> Escherichia coli K-12 MG1655
<220> glpg b3425
<222> 633-732
<223> glpg b3425 U00096 complement(3558255..3559085)
<313> 9622: 672-691
<400> 9622
CTGGCTACGT GGCGAACGCG ATCCGCAAAG TGGCATTTAC CTGCAACGTG 50
GGTTAATTAT CTTTGCGCTG ATCTGGATTG TCGCCGGATG GTTTGATTTG 100
<210> 9623
<211> 100
<212> DNA
<213> Escherichia coli K-12 MG1655
<220> hisf b2026
<222> 678-777
<223> hisf b2026 U00096 2093866..2094642
<313> 9623: 742-761
<400> 9623
CGGCGCGCTG GCAGCTTCCG TATTCCACAA ACAAATAATC AATATTGGTG 50
AATTAAAAGC GTACCTGGCA ACACAGGGCG TGGAGATCAG GATATGTTAA 100
<210> 9624
<211> 100
<212> DNA
<213> Escherichia coli K-12 MG1655

<220> hisf b2026
<222> 666-765
<223> hisf b2026 U00096 2093866..2094642
<313> 9624: 688-707
<400> 9624
TGCCGACGTT GACGGCGCGC TGGCAGCTTC CGTATTCCAC AAACAAATAA 50
TCAATATTGG TGAATTAAAA GCGTACCTGG CAACACAGGG CGTGGAGATC 100
<210> 9625
<211> 100
<212> DNA
<213> Escherichia coli K-12 MG1655
<220> hisf b2026
<222> 555-654
<223> hisf b2026 U00096 2093866..2094642
<313> 9625: 592-611
<400> 9625
TAACGGTTAC GACCTCGAAC AACTGAAAAA AGTGCGTGAA GTTTGCCACG 50
TCCCGCTGAT TGCCTCCGGT GGCGCGGGCA CCATGGAACA CTTCCTCGAA 100
<210> 9626
<211> 100
<212> DNA
<213> Escherichia coli K-12 MG1655
<220> yehd b2112
<222> 366-465
<223> yehd b2112 U00096 complement(2189700..2190242)
<313> 9626: 385-404
<400> 9626
TTTAGGTGAT AACAGCGATG TCCTGGATGT TTCTGCACCT TTTAACATTG 50
AGAGTTATAA AACAGCGGAA GGTCAATATG CTATTCCGTT TAAAGCAAAA 100
<210> 9627
<211> 100
<212> DNA
<213> Escherichia coli K-12 MG1655
<220> yehd b2112
<222> 282-381
<223> yehd b2112 U00096 complement(2189700..2190242)
<313> 9627: 340-359
<400> 9627
ACTCTCGTTG ATTACCAACG ATAAAATAAC CGGTAACGAT AAGGCGATAG 50
CCAGTAGCAA CGATACCGTG GGTTACTATC TCTATTTAGG TGATAACAGC 100
<210> 9628
<211> 100
<212> DNA
<213> Escherichia coli K-12 MG1655
<220> yehd b2112
<222> 216-315
<223> yehd b2112 U00096 complement(2189700..2190242)
<313> 9628: 258-277
<400> 9628
TGATGCAGTC GGTGTAACCA GCAGCATGTT AAAAATTAGC TGCCCGAAAG 50
AAGTTGCTGG TGTAAAACTC TCGTTGATTA CCAACGATAA AATAACCGGT 100
<210> 9629
<211> 100
<212> DNA
<213> Escherichia coli K-12 MG1655
<220> hisg b2020
<222> 801-900
<223> hisg b2020 U00096 2088214..2089113
<313> 9629: 869-888
<400> 9629
GGTCAGCAGC GAAACCCTGT TCTGGGAAAC CATGGAAAAA CTGAAAGCGC 50
TGGGTGCCAG TTCAATTCTG GTCCTGCCGA TTGAGAAGAT GATGGAGTGA 100
<210> 9630
<211> 100
<212> DNA
<213> Escherichia coli K-12 MG1655
<220> hisg b2020
<222> 749-848
<223> hisg b2020 U00096 2088214..2089113
<313> 9630: 803-822
<400> 9630

GCCCAACTAT TCTGCCGCTG GCGGGTGACC AACAGCGCGT AGCGATGCAC 50
ATGGTCAGCA GCGAAACCCT GTTCTGGGAA ACCATGGAAA AACTGAAAGC 100
<210> 9631
<211> 100
<212> DNA
<213> Escherichia coli K-12 MG1655
<220> hisg b2020
<222> 685-784
<223> hisg b2020 U00096 2088214..2089113
<313> 9631: 749-768
<400> 9631
ATCATGATGC ACGCACCGAC CGAACGTCTG GATGAAGTCA TCGCCCTGCT 50
GCCAGGTGCC GAACGCCCAA CTATTCTGCC GCTGGCGGGT GACCAACAGC 100
<210> 9632
<211> 100
<212> DNA
<213> Escherichia coli K-12 MG1655
<220> yehe b2113
<222> 183-282
<223> yehe b2113 U00096 complement(2190535..2190816)
<313> 9632: 243-262
<400> 9632
CCGTCATCTG TTTTCTTCGC CCGTCAAAGG TGTAACGACA GAAGTGGTTG 50
TTGCAGGTAG CGATAGCAAA CGTCGAATCG TGTTAAATCG CTACGATTAA 100
<210> 9633
<211> 100
<212> DNA
<213> Escherichia coli K-12 MG1655
<220> yehe b2113
<222> 166-265
<223> yehe b2113 U00096 complement(2190535..2190816)
<313> 9633: 208-227
<400> 9633
ACGTATAATG TTGACACCCG TCATCTGTTT TCTTCGCCCG TCAAAGGTGT 50
AACGACAGAA GTGGTTGTTG CAGGTAGCGA TAGCAAACGT CGAATCGTGT 100
<210> 9634
<211> 100
<212> DNA
<213> Escherichia coli K-12 MG1655
<220> yehe b2113
<222> 77-176
<223> yehe b2113 U00096 complement(2190535..2190816)
<313> 9634: 126-145
<400> 9634
CCACATTAGC GATTAATGGC AGGATCAGTC CCCCAACATG CAGTATGGCT 50
ATGGTTAATG GTCAACCTCA ACAGCATTGT GGTCAGTTAA CGTATAATGT 100
<210> 9635
<211> 100
<212> DNA
<213> Escherichia coli K-12 MG1655
<220> hish b2024
<222> 467-566
<223> hish b2024 U00096 2092557..2093147
<313> 9635: 514-533
<400> 9635
AGTGTAATTA CGGCGAACCG TTCACCGCGG CGGTACAAAA AGATAACTTC 50
TACGGCGTGC AGTTCCACCC GGAGCGTTCT GGTGCCGCTG GTGCTAAGTT 100
<210> 9636
<211> 100
<212> DNA
<213> Escherichia coli K-12 MG1655
<220> hish b2024
<222> 369-468
<223> hish b2024 U00096 2092557..2093147
<313> 9636: 421-440
<400> 9636
GCAGGCAGGC AACCGCCTGT TTCAGGGGAT TGAAGACGGC GCGTACTTTT 50
ACTTTGTTCA CAGCTACGCA ATGCCGGTCA ATCCGTGGAC CATCGCCCAG 100
<210> 9637
<211> 100
<212> DNA

<213> Escherichia coli K-12 MG1655
<220> hish b2024
<222> 325-424
<223> hish b2024 U00096 2092557..2093147
<313> 9637: 388-407
<400> 9637
TTTGGTCTGC CACTGCCACA TATGGGCTGG AACCGCGTTT ACCCGCAGGC  50
AGGCAACCGC CTGTTTCAGG GGATTGAAGA CGGCGCGTAC TTTTACTTTG  100
<210> 9638
<211> 100
<212> DNA
<213> Escherichia coli K-12 MG1655
<220> hisi b2027
<222> 513-612
<223> hisi b2027 U00096 2094636..2095247
<313> 9638: 584-603
<400> 9638
CGAGGCGTCT GATTTGATGT ATCACCTGCT GGTGTTGTTG CAGGATCAGG  50
GGCTGGATTT AACGACGGTA ATTGAGAACC TGCGTAAACG GCATCAGTGA  100
<210> 9639
<211> 100
<212> DNA
<213> Escherichia coli K-12 MG1655
<220> hisi b2027
<222> 513-612
<223> hisi b2027 U00096 2094636..2095247
<313> 9639: 535-554
<400> 9639
CGAGGCGTCT GATTTGATGT ATCACCTGCT GGTGTTGTTG CAGGATCAGG  50
GGCTGGATTT AACGACGGTA ATTGAGAACC TGCGTAAACG GCATCAGTGA  100
<210> 9640
<211> 100
<212> DNA
<213> Escherichia coli K-12 MG1655
<220> hisi b2027
<222> 457-556
<223> hisi b2027 U00096 2094636..2095247
<313> 9640: 494-513
<400> 9640
GAAGGCGTGG AAACCGCGCT GGCAGCAACG GTACATGACC GCTTTGAGCT  50
GACCAACGAG GCGTCTGATT TGATGTATCA CCTGCTGGTG TTGTTGCAGG  100
<210> 9641
<211> 100
<212> DNA
<213> Escherichia coli K-12 MG1655
<220> glpk b3927
<222> 1360-1459
<223> glpk b3927 U00096 complement(4113294..4114802)
<313> 9641: 1423-1442
<400> 9641
TTCTGGCAGA ACCTCGACGA GCTGCAAGAG AAAGCGGTGA TTGAGCGCGA  50
GTTCCGTCCA GGCATCGAAA CCACTGAGCG TAATTACCGT TACGCAGGCT  100
<210> 9642
<211> 100
<212> DNA
<213> Escherichia coli K-12 MG1655
<220> glpk b3927
<222> 1333-1432
<223> glpk b3927 U00096 complement(4113294..4114802)
<313> 9642: 1381-1400
<400> 9642
GCCTATCTCG CAGGCCTGGC GGTTGGCTTC TGGCAGAACC TCGACGAGCT  50
GCAAGAGAAA GCGGTGATTG AGCGCGAGTT CCGTCCAGGC ATCGAAACCA  100
<210> 9643
<211> 100
<212> DNA
<213> Escherichia coli K-12 MG1655
<220> glpk b3927
<222> 1241-1340
<223> glpk b3927 U00096 complement(4113294..4114802)
<313> 9643: 1296-1315

<400> 9643
TAGCAAACAA TTTCCTGATG CAGTTCCAGT CCGATATTCT CGGCACCCGC  50
GTTGAGCGCC CGGAAGTGCG CGAAGTCACC GCATTGGGTG CGGCCTATCT  100
<210> 9644
<211> 100
<212> DNA
<213> Escherichia coli K-12 MG1655
<220> hisj b2310
<222> 658-757
<223> hisj b2310 U00096 complement(2424026..2424808)
<313> 9644: 681-700
<400> 9644
GGCCTGCGTA AAGAAGATAA CGAACTGCGC GAAGCACTGA ACAAAGCCTT  50
TGCCGAAATG CGCGCTGACG GTACTTACGA GAAATTAGCG AAAAAGTACT  100
<210> 9645
<211> 100
<212> DNA
<213> Escherichia coli K-12 MG1655
<220> hisj b2310
<222> 608-707
<223> hisj b2310 U00096 complement(2424026..2424808)
<313> 9645: 627-646
<400> 9645
GTGGCCCGTC TGTTAAAGAT GAAAAACTGT TTGGCGTAGG GACCGGCATG  50
GGCCTGCGTA AAGAAGATAA CGAACTGCGC GAAGCACTGA ACAAAGCCTT  100
<210> 9646
<211> 100
<212> DNA
<213> Escherichia coli K-12 MG1655
<220> hisj b2310
<222> 544-643
<223> hisj b2310 U00096 complement(2424026..2424808)
<313> 9646: 590-609
<400> 9646
CAGGATGAGG TCGCTGCCAG CGAAGGTTTC CTCAAACAAC CTGTCGGTAA  50
AGATTACAAA TTCGGTGGCC CGTCTGTTAA AGATGAAAAA CTGTTTGGCG  100
<210> 9647
<211> 100
<212> DNA
<213> Escherichia coli K-12 MG1655
<220> yehi b2119
<222> 3505-3604
<223> yehi b2119 U00096 2198299..2201931
<313> 9647: 3566-3585
<400> 9647
GATAGCAAAG CGCTAATGAG CTTCCGTTCT CTTCGAGTGT ACCGTGACAA  50
TAAATCCGTC ACTTTTGCCG AACTTGATGT GTTTGATTTG AGTGAGGCGT  100
<210> 9648
<211> 100
<212> DNA
<213> Escherichia coli K-12 MG1655
<220> yehi b2119
<222> 3367-3466
<223> yehi b2119 U00096 2198299..2201931
<313> 9648: 3411-3430
<400> 9648
AAATCCGCTA CCGTTGGGCA ACTTATGGGA ATGCGATACA AAGGCTGGGA  50
GTCAGGCTAT GAGGACGCAT TTGTCTATAA CCTGGGTGAG TACCGGCTGG  100
<210> 9649
<211> 100
<212> DNA
<213> Escherichia coli K-12 MG1655
<220> yehi b2119
<222> 3354-3453
<223> yehi b2119 U00096 2198299..2201931
<313> 9649: 3378-3397
<400> 9649
TCGCTGGGAA GGTAAATCCG CTACCGTTGG GCAACTTATG GGAATGCGAT  50
ACAAAGGCTG GGAGTCAGGC TATGAGGACG CATTTGTCTA TAACCTGGGT  100
<210> 9650
<211> 100

<212> DNA
<213> Escherichia coli K-12 MG1655
<220> hisl b2019
<222> 1-51
<223> hisl b2019 U00096 2088018..2088068
<313> 9650: 16-35
<400> 9650
ATGACACGCG TTCAATTTAA ACACCACCAT CATCACCATC ATCCTGACTA 50
G 100
<210> 9651
<211> 100
<212> DNA
<213> Escherichia coli K-12 MG1655
<220> hism b2308
<222> 566-665
<223> hism b2308 U00096 complement(2422537..2423253)
<313> 9651: 588-607
<400> 9651
CCCGCGATAT TAACGCCGCC ACGTATCAAC CTTTTACCGC CTTCGGCATT 50
GCCGCGGTGC TCTATTTAAT CATCTCTTAT GTCCTGATCA GCCTCTTTCG 100
<210> 9652
<211> 100
<212> DNA
<213> Escherichia coli K-12 MG1655
<220> hism b2308
<222> 458-557
<223> hism b2308 U00096 complement(2422537..2423253)
<313> 9652: 516-535
<400> 9652
CTTCTGCGCT GCGTATTGCG TTACCGGCAT ACAGCAACGA AGTGATCCTG 50
ATGCTGCACT CTACTGCGTT GGCATTTACT GCCACGGTGC CGGATCTGCT 100
<210> 9653
<211> 100
<212> DNA
<213> Escherichia coli K-12 MG1655
<220> hism b2308
<222> 449-548
<223> hism b2308 U00096 complement(2422537..2423253)
<313> 9653: 480-499
<400> 9653
TTATTTTGCC TTCTGCGCTG CGTATTGCGT TACCGGCATA CAGCAACGAA 50
GTGATCCTGA TGCTGCACTC TACTGCGTTG GCATTTACTG CCACGGTGCC 100
<210> 9654
<211> 100
<212> DNA
<213> Escherichia coli K-12 MG1655
<220> yehl b2120
<222> 1056-1155
<223> yehl b2120 U00096 2202550..2203704
<313> 9654: 1117-1136
<400> 9654
AGATCGCGCG CGTCTGCGCC GTTACTTCGA ACAGCGTGTC GCCACACATA 50
AAGAAGCTCA CTGGCAGGCT TATTATCAAG CCCGCCACCG CCTGCCGTGA 100
<210> 9655
<211> 100
<212> DNA
<213> Escherichia coli K-12 MG1655
<220> yehl b2120
<222> 1031-1130
<223> yehl b2120 U00096 2202550..2203704
<313> 9655: 1076-1095
<400> 9655
GAACCATCGT CAAAGATAAC GAAGAAGATC GCGCGCGTCT GCGCCGTTAC 50
TTCGAACAGC GTGTCGCCAC ACATAAAGAA GCTCACTGGC AGGCTTATTA 100
<210> 9656
<211> 100
<212> DNA
<213> Escherichia coli K-12 MG1655
<220> yehl b2120
<222> 982-1081
<223> yehl b2120 U00096 2202550..2203704

<313> 9656: 1041-1060
<400> 9656
TTAGCGAATC GCGCGGGGGA GCCTGCCGAC CTGGTGGAAT GTATTGCCGG 50
AACCATCGTC AAAGATAACG AAGAAGATCG CGCGCGTCTG CGCCGTTACT 100
<210> 9657
<211> 100
<212> DNA
<213> Escherichia coli K-12 MG1655
<220> yehm b2121
<222> 2181-2280
<223> yehm b2121 U00096 2203715..2205994
<313> 9657: 2233-2252
<400> 9657
AATGCCGTTG CATTGTCCAC CACAGGCCAT TGCACATCAT CAACAACTCG 50
AACAGCAGGC ACTGGCATCG CTGCAAAACT GGGGAGTTTT CCATGTCTGA 100
<210> 9658
<211> 100
<212> DNA
<213> Escherichia coli K-12 MG1655
<220> yehm b2121
<222> 2141-2240
<223> yehm b2121 U00096 2203715..2205994
<313> 9658: 2196-2215
<400> 9658
AGCATTATCA ACTGGCGCAA CTTCCCGTTT CGGCGCTGCA AATGCCGTTG 50
CATTGTCCAC CACAGGCCAT TGCACATCAT CAACAACTCG AACAGCAGGC 100
<210> 9659
<211> 100
<212> DNA
<213> Escherichia coli K-12 MG1655
<220> yehm b2121
<222> 2047-2146
<223> yehm b2121 U00096 2203715..2205994
<313> 9659: 2088-2107
<400> 9659
GAAGCCGATT TTATTAACGC CCTGCCCGAT TTACGCGCGG CGATGGCCTG 50
GCTACCACCA CGAGAACGCG GGACGCTGGC GCATCAGGTG CTTGAGCATT 100
<210> 9660
<211> 100
<212> DNA
<213> Escherichia coli K-12 MG1655
<220> glpq b2240
<222> 900-999
<223> glpq b2240 U00096 complement(2347955..2349031)
<313> 9660: 964-983
<400> 9660
GCAAGATGCT CAGCAGAATA AACTGGTAGT GCATCCTTAT ACCGTGCGGT 50
CAGATAAACT GCCTGAATAC ACTCCTGATG TGAATCAGTT ATATGATGCT 100
<210> 9661
<211> 100
<212> DNA
<213> Escherichia coli K-12 MG1655
<220> glpq b2240
<222> 878-977
<223> glpq b2240 U00096 complement(2347955..2349031)
<313> 9661: 914-933
<400> 9661
ATATCAAACT CACTGGCATG GTGCAAGATG CTCAGCAGAA TAAACTGGTA 50
GTGCATCCTT ATACCGTGCG GTCAGATAAA CTGCCTGAAT ACACTCCTGA 100
<210> 9662
<211> 100
<212> DNA
<213> Escherichia coli K-12 MG1655
<220> glpq b2240
<222> 829-928
<223> glpq b2240 U00096 complement(2347955..2349031)
<313> 9662: 871-890
<400> 9662
ATTGGTCCGG ATTACCATAT GTTGATTGAG GAGACATCGC AGCCGGGTAA 50
TATCAAACTC ACTGGCATGG TGCAAGATGC TCAGCAGAAT AAACTGGTAG 100
<210> 9663

<211> 100
<212> DNA
<213> Escherichia coli K-12 MG1655
<220> hisp b2307
<222> 675-774
<223> hisp b2307 U00096 complement(2421756..2422529)
<313> 9663: 726-745
<400> 9663
CCATCAGGGG AAAATAGAAG AAGAGGGCGC GCCGGAGCAG TTATTTGGCA 50
ACCCGCAAAG CCCTCGTCTG CAACGGTTCC TTAAGGGATC GCTGAAATAA 100
<210> 9664
<211> 100
<212> DNA
<213> Escherichia coli K-12 MG1655
<220> hisp b2307
<222> 630-729
<223> hisp b2307 U00096 complement(2421756..2422529)
<313> 9664: 661-680
<400> 9664
CGAAATGGGC TTTGCTCGCC ATGTTTCTAC TCATGTCATT TTCCTCCATC 50
AGGGGAAAAT AGAAGAAGAG GGCGCGCCGG AGCAGTTATT TGGCAACCCG 100
<210> 9665
<211> 100
<212> DNA
<213> Escherichia coli K-12 MG1655
<220> hisp b2307
<222> 530-629
<223> hisp b2307 U00096 complement(2421756..2422529)
<313> 9665: 590-609
<400> 9665
ATGAACCTAC CTCGGCGCTC GATCCTGAAC TGGTAGGCGA AGTGTTGCGT 50
ATTATGCAGC AACTGGCAGA AGAGGGGAAA ACCATGGTGG TAGTGACTCA 100
<210> 9666
<211> 100
<212> DNA
<213> Escherichia coli K-12 MG1655
<220> glpr b3424
<222> 660-759
<223> glpr b3424 U00096 complement(3557480..3558238)
<313> 9666: 724-743
<400> 9666
CAGCATCAGC ATGGTAGATG CCGTCTACAC CGACGCCCCG CCGCCAGTAA 50
GCGTGATGCA GGTGCTGACG GACCACCATA TTCAACTGGA GCTGTGCTGA 100
<210> 9667
<211> 100
<212> DNA
<213> Escherichia coli K-12 MG1655
<220> glpr b3424
<222> 660-759
<223> glpr b3424 U00096 complement(3557480..3558238)
<313> 9667: 685-704
<400> 9667
CAGCATCAGC ATGGTAGATG CCGTCTACAC CGACGCCCCG CCGCCAGTAA 50
GCGTGATGCA GGTGCTGACG GACCACCATA TTCAACTGGA GCTGTGCTGA 100
<210> 9668
<211> 100
<212> DNA
<213> Escherichia coli K-12 MG1655
<220> glpr b3424
<222> 618-717
<223> glpr b3424 U00096 complement(3557480..3558238)
<313> 9668: 647-666
<400> 9668
CGATCACTCG AAATTTGGCC GTAACGCGAT GGTCAATATG GGCAGCATCA 50
GCATGGTAGA TGCCGTCTAC ACCGACGCCC CGCCGCCAGT AAGCGTGATG 100
<210> 9669
<211> 100
<212> DNA
<213> Escherichia coli K-12 MG1655
<220> hisq b2309
<222> 588-687

<223> hisq b2309 U00096 complement(2423250..2423936)
<313> 9669: 639-658
<400> 9669
CTGTGGCGTG ATTTACCTGG TTTTCACCAC TGTTTCCAAT GGTGTGCTGC  50
TGTTCCTTGA GCGCCGCTAC TCCGTGGGTG TGAAGAGGGC TGACCTGTGA  100
<210> 9670
<211> 100
<212> DNA
<213> Escherichia coli K-12 MG1655
<220> hisq b2309
<222> 568-667
<223> hisq b2309 U00096 complement(2423250..2423936)
<313> 9670: 604-623
<400> 9670
CCGTTCTATT TCGCCATCGT CTGTGGCGTG ATTTACCTGG TTTTCACCAC  50
TGTTTCCAAT GGTGTGCTGC TGTTCCTTGA GCGCCGCTAC TCCGTGGGTG  100
<210> 9671
<211> 100
<212> DNA
<213> Escherichia coli K-12 MG1655
<220> hisq b2309
<222> 498-597
<223> hisq b2309 U00096 complement(2423250..2423936)
<313> 9671: 557-576
<400> 9671
ACTGGTTTCG TTACTCGGCC TGGAAGATGT GGTCAAAGCC ACCCAACTGG  50
CAGGCAAAAG TACCTGGGAA CCGTTCTATT TCGCCATCGT CTGTGGCGTG  100
<210> 9672
<211> 100
<212> DNA
<213> Escherichia coli K-12 MG1655
<220> yehp b2122
<222> 1038-1137
<223> yehp b2122 U00096 2205987..2207123
<313> 9672: 1074-1093
<400> 9672
CCGCGATACG GCCCAGGCGC TGGTTAATGT CGGCGCACAA ATAGCCGCCA  50
TGACGCCGGG CGAGCTGGCA TCATGGCTTG CGGAGAATCT TCAGTCATGA  100
<210> 9673
<211> 100
<212> DNA
<213> Escherichia coli K-12 MG1655
<220> yehp b2122
<222> 1018-1117
<223> yehp b2122 U00096 2205987..2207123
<313> 9673: 1033-1052
<400> 9673
ACCGCAACAC CTTGCTATGA CCGCGATACG GCCCAGGCGC TGGTTAATGT  50
CGGCGCACAA ATAGCCGCCA TGACGCCGGG CGAGCTGGCA TCATGGCTTG  100
<210> 9674
<211> 100
<212> DNA
<213> Escherichia coli K-12 MG1655
<220> yehp b2122
<222> 925-1024
<223> yehp b2122 U00096 2205987..2207123
<313> 9674: 983-1002
<400> 9674
TACGAAGGGG GTTCATCATC ATTACTGACG CATCAGGTGA AAAAGTGTGT  50
CCAGAGCGGC ATCAAAGTGC TGGGACTGGC AGCGCTCGAT AGCACCGCAA  100
<210> 9675
<211> 100
<212> DNA
<213> Escherichia coli K-12 MG1655
<220> b2595 b2595
<222> 639-738
<223> b2595 b2595 U00096 2734166..2734903
<313> 9675: 709-728
<400> 9675
TGCGCTGCCG CTGATGGAAA ATGCATACCG TCAGATGCAG ATGAATGCGC  50
AAGCTGAAAA AGTAGCGAAA ATCATCGCCG CAAACAGCAG CAATACATAA  100

<210> 9676
<211> 100
<212> DNA
<213> Escherichia coli K-12 MG1655
<220> b2595 b2595
<222> 540-639
<223> b2595 b2595 U00096 2734166..2734903
<313> 9676: 569-588
<400> 9676
CGTGGCCGAG TACTATACAG AACGTGGCGC ATGGGTTGCC GTCGTTAACC 50
GCGTAGAAGG CATGTTGCGC GACTACCCGG ATACCCAGGC TACGCGTGAT 100
<210> 9677
<211> 100
<212> DNA
<213> Escherichia coli K-12 MG1655
<220> b2595 b2595
<222> 476-575
<223> b2595 b2595 U00096 2734166..2734903
<313> 9677: 536-555
<400> 9677
ACACCACCGA TGCCACCAAA CGTCTGGTAT TCCTGAAAGA TCGTCTGGCG 50
AAATATGAAT ACTCCGTGGC CGAGTACTAT ACAGAACGTG GCGCATGGGT 100
<210> 9678
<211> 100
<212> DNA
<213> Escherichia coli K-12 MG1655
<220> glpt b2241
<222> 1260-1359
<223> glpt b2241 U00096 complement(2349036..2350394)
<313> 9678: 1336-1355
<400> 9678
GATTGGCGGC AGCATTCTGG CGGTTATCTT GTTGATTGTT GTGATGATTG 50
GCGAAAAACG TCGCCATGAA CAATTACTGC AAGAACGCAA CGGAGGCTAA 100
<210> 9679
<211> 100
<212> DNA
<213> Escherichia coli K-12 MG1655
<220> glpt b2241
<222> 1232-1331
<223> glpt b2241 U00096 complement(2349036..2350394)
<313> 9679: 1272-1291
<400> 9679
TCGGCTGGGA TGGCGGCTTT ATGGTAATGA TTGGCGGCAG CATTCTGGCG 50
GTTATCTTGT TGATTGTTGT GATGATTGGC GAAAAACGTC GCCATGAACA 100
<210> 9680
<211> 100
<212> DNA
<213> Escherichia coli K-12 MG1655
<220> glpt b2241
<222> 1185-1284
<223> glpt b2241 U00096 complement(2349036..2350394)
<313> 9680: 1202-1221
<400> 9680
CGGTTCGGTG GCGGCGAGCG CGATTGTTGG CTACACCGTG GACTTCTTCG 50
GCTGGGATGG CGGCTTTATG GTAATGATTG GCGGCAGCAT TCTGGCGGTT 100
<210> 9681
<211> 100
<212> DNA
<213> Escherichia coli K-12 MG1655
<220> hiss b2515
<222> 1157-1256
<223> hiss b2515 U00096 complement(2637321..2638595)
<313> 9681: 1210-1229
<400> 9681
TGCTGGGTGA GTCTGAAGTG GCTAACGGCA CAGCAGTAGT GAAGGATTTG 50
CGCTCTGGTG AGCAAACGGC AGTTGCGCAG GATAGCGTAG CCGCGCATTT 100
<210> 9682
<211> 100
<212> DNA
<213> Escherichia coli K-12 MG1655
<220> hiss b2515

<222> 1111-1210
<223> hiss b2515 U00096 complement(2637321..2638595)
<313> 9682: 1161-1180
<400> 9682
AAACAGTTTG CCCGTGCTGA TAAATGGGGT GCCCGCGTTG CTGTGGTGCT 50
GGGTGAGTCT GAAGTGGCTA ACGGCACAGC AGTAGTGAAG GATTTGCGCT 100
<210> 9683
<211> 100
<212> DNA
<213> Escherichia coli K-12 MG1655
<220> hiss b2515
<222> 1068-1167
<223> hiss b2515 U00096 complement(2637321..2638595)
<313> 9683: 1102-1121
<400> 9683
GGGCGTGAAA TTGATGACCA ACCACGGCGG CGGCAACTTT AAGAAACAGT 50
TTGCCCGTGC TGATAAATGG GGTGCCCGCG TTGCTGTGGT GCTGGGTGAG 100
<210> 9684
<211> 100
<212> DNA
<213> Escherichia coli K-12 MG1655
<220> yehq b2123
<222> 1769-1868
<223> yehq b2123 U00096 2207096..2208964
<313> 9684: 1794-1813
<400> 9684
CTACTGCTCA TCAGTTGCTG ATACGCTGCC AGGCGTTACT TATTCAACTG 50
CTCCATAACG GCTGGCGCTA TCAGGAACAG AGTGCTATTG GTCAGGCATA 100
<210> 9685
<211> 100
<212> DNA
<213> Escherichia coli K-12 MG1655
<220> yehq b2123
<222> 1710-1809
<223> yehq b2123 U00096 2207096..2208964
<313> 9685: 1760-1779
<400> 9685
GGACGCAGAA ACTACGCCTG TGGCACCGCT ACCTTCTGCC AGCGTTTTGC 50
CTGTGCCGTC TACTGCTCAT CAGTTGCTGA TACGCTGCCA GGCGTTACTT 100
<210> 9686
<211> 100
<212> DNA
<213> Escherichia coli K-12 MG1655
<220> yehq b2123
<222> 1667-1766
<223> yehq b2123 U00096 2207096..2208964
<313> 9686: 1710-1729
<400> 9686
TGGAACCACG GGTGATGATG ACAAAAACCC GTGCCTGGGC GCTGGACGCA 50
GAAACTACGC CTGTGGCACC GCTACCTTCT GCCAGCGTTT TGCCTGTGCC 100
<210> 9687
<211> 100
<212> DNA
<213> Escherichia coli K-12 MG1655
<220> b2596 b2597
<222> 108-207
<223> b2596 b2597 U00096 2734933..2735139
<313> 9687: 182-201
<400> 9687
ATCTCTTCCT GCGATTTCTC ACAAAAAAGA TTCGTTGACA AAAAGTGACA 50
AAATTATGAG ATTTTCATCA CACATTTTGA CATCAGGAAC GGTATGCTGA 100
<210> 9688
<211> 100
<212> DNA
<213> Escherichia coli K-12 MG1655
<220> b2596 b2597
<222> 82-181
<223> b2596 b2597 U00096 2734933..2735139
<313> 9688: 122-141
<400> 9688
TTGAGATATT CCTCAAGTAA AAAAACATCT CTTCCTGCGA TTTCTCACAA 50

AAAAGATTCG TTGACAAAAA GTGACAAAAT TATGAGATTT TCATCACACA 100
<210> 9689
<211> 100
<212> DNA
<213> Escherichia coli K-12 MG1655
<220> b2596 b2597
<222> 67-166
<223> b2596 b2597 U00096 2734933..2735139
<313> 9689: 110-129
<400> 9689
CAAATATGGC TCGCTTTGAG ATATTCCTCA AGTAAAAAAA CATCTCTTCC 50
TGCGATTTCT CACAAAAAAG ATTCGTTGAC AAAAAGTGAC AAAATTATGA 100
<210> 9690
<211> 100
<212> DNA
<213> Escherichia coli K-12 MG1655
<220> yehr b2124
<222> 375-474
<223> yehr b2124 U00096 2209233..2209706
<313> 9690: 403-422
<400> 9690
TTCAGGAATC AACGTTTCTG CTGAAGATGC CAAAAAAGGT ATCACTATGG 50
CGCAAATGGA ACTGGTGATG AAAGCCGCTG GTTTTAAAGA AGTGAAATAA 100
<210> 9691
<211> 100
<212> DNA
<213> Escherichia coli K-12 MG1655
<220> yehr b2124
<222> 256-355
<223> yehr b2124 U00096 2209233..2209706
<313> 9691: 297-316
<400> 9691
AGCGCCAAAT ACAAAAACAT CGCGGGTGTT GAAGAAAAAT TAACCTATAC 50
CGATACCTAC GCGCAGGAAA ACGTGACTAT CGATATGGAA AAAGTGGATT 100
<210> 9692
<211> 100
<212> DNA
<213> Escherichia coli K-12 MG1655
<220> yehr b2124
<222> 239-338
<223> yehr b2124 U00096 2209233..2209706
<313> 9692: 275-294
<400> 9692
AGACACTTGA GCCGTTAAGC GCCAAATACA AAAACATCGC GGGTGTTGAA 50
GAAAAATTAA CCTATACCGA TACCTACGCG CAGGAAAACG TGACTATCGA 100
<210> 9693
<211> 100
<212> DNA
<213> Escherichia coli K-12 MG1655
<220> yehs b2125
<222> 362-461
<223> yehs b2125 U00096 complement(2209746..2210216)
<313> 9693: 401-420
<400> 9693
CAGCGATGAT GCGCGCACCG GATCATAAAA ACTTCCGCGA ATGCGGCGAT 50
CAATTTTTAC GTTATTTTCT GCGTGGACTG GCAGCGCGCC AGCATGTGAA 100
<210> 9694
<211> 100
<212> DNA
<213> Escherichia coli K-12 MG1655
<220> yehs b2125
<222> 297-396
<223> yehs b2125 U00096 complement(2209746..2210216)
<313> 9694: 344-363
<400> 9694
GAAAACCGAT GACATTCTGG CTATCCTCAC CGAACAGCAG TTCCGCGTTT 50
CGATGCCGGA AATTACAGCG ATGATGCGCG CACCGGATCA TAAAAACTTC 100
<210> 9695
<211> 100
<212> DNA
<213> Escherichia coli K-12 MG1655

<220> yehs b2125
<222> 259-358
<223> yehs b2125 U00096 complement(2209746..2210216)
<313> 9695: 301-320
<400> 9695
AACATCGTGC TGAAAAAATT ACGCATCGCG TTTTCGCTGA AAACCGATGA 50
CATTCTGGCT ATCCTCACCG AACAGCAGTT CCGCGTTTCG ATGCCGGAAA 100
<210> 9696
<211> 100
<212> DNA
<213> Escherichia coli K-12 MG1655
<220> yeht b2126
<222> 636-735
<223> yeht b2126 U00096 complement(2210263..2210997)
<313> 9696: 715-734
<400> 9696
GGAAGATAAC GGCCAGGCCG AGTTGATTTT GCGTAATGGC TTAACCGTGC 50
CGGTCAGCCG CCGTTATCTG AAAAGCTTAA AAGAGGCGAT TGGCCTGTAA 100
<210> 9697
<211> 100
<212> DNA
<213> Escherichia coli K-12 MG1655
<220> yeht b2126
<222> 577-676
<223> yeht b2126 U00096 complement(2210263..2210997)
<313> 9697: 623-642
<400> 9697
CTACTGCGCT GCCATCGTCA GTATCTGGTT AACCTCGCGC ATTTACAGGA 50
GATTCGTCTG GAAGATAACG GCCAGGCCGA GTTGATTTTG CGTAATGGCT 100
<210> 9698
<211> 100
<212> DNA
<213> Escherichia coli K-12 MG1655
<220> yeht b2126
<222> 538-637
<223> yeht b2126 U00096 complement(2210263..2210997)
<313> 9698: 581-600
<400> 9698
ACCGAATTGA CATTACGTAC CCTGGAAAGT CGTACACCAC TACTGCGCTG 50
CCATCGTCAG TATCTGGTTA ACCTCGCGCA TTTACAGGAG ATTCGTCTGG 100
<210> 9699
<211> 100
<212> DNA
<213> Escherichia coli K-12 MG1655
<220> glpx b3926
<222> 893-992
<223> glpx b3926 U00096 complement(4112149..4113159)
<313> 9699: 946-965
<400> 9699
GCAATATCGC GACTACCGAA ACGCTGCTGA TCCGCGGCAA GTCACGCACC 50
ATTCGCCGCA TTCAGTCCAT CCACTATCTG GATCGCAAAG ACCCGGAAAT 100
<210> 9700
<211> 100
<212> DNA
<213> Escherichia coli K-12 MG1655
<220> glpx b3926
<222> 817-916
<223> glpx b3926 U00096 complement(4112149..4113159)
<313> 9700: 864-883
<400> 9700
GCGCGCAGCG ATAACGTCAT CTTCTCTGCC ACCGGTATTA CCAAAGGCGA 50
TCTGCTGGAA GGCATTAGCC GCAAAGGCAA TATCGCGACT ACCGAAACGC 100
<210> 9701
<211> 100
<212> DNA
<213> Escherichia coli K-12 MG1655
<220> glpx b3926
<222> 748-847
<223> glpx b3926 U00096 complement(4112149..4113159)
<313> 9701: 763-782
<400> 9701

GAGCAGGAGC TGGCACGCTG CAAAGCGATG GGCATCGAAG CCGGTAAAGT 50
ATTGCGCCTG GGCGATATGG CGCGCAGCGA TAACGTCATC TTCTCTGCCA 100
<210> 9702
<211> 100
<212> DNA
<213> Escherichia coli K-12 MG1655
<220> yehu b2127
<222> 1587-1686
<223> yehu b2127 U00096 complement(2210979..2212664)
<313> 9702: 1630-1649
<400> 9702
ACGTGAACGG TTTGGCGATG ACTATGGAAT AAGCGTCGCC TGTGAGCCTG 50
ATAGTTACAC CCGAATAACG TTACGACTAC CATGGAGGGA CGAGGCATGA 100
<210> 9703
<211> 100
<212> DNA
<213> Escherichia coli K-12 MG1655
<220> yehu b2127
<222> 1502-1601
<223> yehu b2127 U00096 complement(2210979..2212664)
<313> 9703: 1558-1577
<400> 9703
TGCTGGAGAT CGAAGACAAT GCCGGTTTGT ATCAACCGGT AACCAATGCC 50
AGTGGGCTGG GGATGAATCT GGTGGATAAG CGTTTACGTG AACGGTTTGG 100
<210> 9704
<211> 100
<212> DNA
<213> Escherichia coli K-12 MG1655
<220> yehu b2127
<222> 1466-1565
<223> yehu b2127 U00096 complement(2210979..2212664)
<313> 9704: 1499-1518
<400> 9704
TGGCAATCAG CGCCCGACGT GAGGGGCAAC ATTTGATGCT GGAGATCGAA 50
GACAATGCCG GTTTGTATCA ACCGGTAACC AATGCCAGTG GGCTGGGGAT 100
<210> 9705
<211> 100
<212> DNA
<213> Escherichia coli K-12 MG1655
<220> yehv b2128
<222> 633-732
<223> yehv b2128 U00096 2212886..2213617
<313> 9705: 710-729
<400> 9705
ATTGCTGGTG TGGTGCGGTG AAAATCGAAC CTCCGCCCAA CAGCAGCAAC 50
TCACCAGTTG GCAAGAACAA GGCCATGATA TTTTCCCACT CGGCATTTAA 100
<210> 9706
<211> 100
<212> DNA
<213> Escherichia coli K-12 MG1655
<220> yehv b2128
<222> 607-706
<223> yehv b2128 U00096 2212886..2213617
<313> 9706: 669-688
<400> 9706
CGCCCTGAAC TATTCGAAGG CCGTACATTG CTGGTGTGGT GCGGTGAAAA 50
TCGAACCTCC GCCCAACAGC AGCAACTCAC CAGTTGGCAA GAACAAGGCC 100
<210> 9707
<211> 100
<212> DNA
<213> Escherichia coli K-12 MG1655
<220> yehv b2128
<222> 586-685
<223> yehv b2128 U00096 2212886..2213617
<313> 9707: 617-636
<400> 9707
GCCCACTCGC TCAATCAACT ACGCCCTGAA CTATTCGAAG GCCGTACATT 50
GCTGGTGTGG TGCGGTGAAA ATCGAACCTC CGCCCAACAG CAGCAACTCA 100
<210> 9708
<211> 100
<212> DNA

<213> Escherichia coli K-12 MG1655
<220> yehw b2129
<222> 633-732
<223> yehw b2129 U00096 complement(2213765..2214496)
<313> 9708: 679-698
<400> 9708
GTATGTGATC CAGGGGGCGT TACTGGTGGC ACTGGCGGCG ATCATCGCAG 50
ACCGCCTGTT TGAAAGGCTG GTGCAGGCGC TTAGCCAGCA CGCAAAATAA 100
<210> 9709
<211> 100
<212> DNA
<213> Escherichia coli K-12 MG1655
<220> yehw b2129
<222> 565-664
<223> yehw b2129 U00096 complement(2213765..2214496)
<313> 9709: 607-626
<400> 9709
GCCTCAACGG TAGGGGCCAG CACGCTGGGT ACGCCCATCA TCATCGGGCT 50
TAGCGGATTT AATACCGCGT ATGTGATCCA GGGGGCGTTA CTGGTGGCAC 100
<210> 9710
<211> 100
<212> DNA
<213> Escherichia coli K-12 MG1655
<220> yehw b2129
<222> 459-558
<223> yehw b2129 U00096 complement(2213765..2214496)
<313> 9710: 504-523
<400> 9710
GAGTCGTGGT CAGCGAGTGC GTAAGGTCGA GCTACCGCTG GCGGCTCCGG 50
TGATTCTGGC GGGCGTGCGA ACTTCGGTGA TTATCAACAT TGGTACGGCG 100
<210> 9711
<211> 100
<212> DNA
<213> Escherichia coli K-12 MG1655
<220> yehx b2130
<222> 767-866
<223> yehx b2130 U00096 complement(2214501..2215427)
<313> 9711: 815-834
<400> 9711
GCGAAGAACG AGCAGATGGT GAGGCACTGG CAGAAGAGAT GACGCTACGC 50
GATGCGCTCT CTCTGTTTGT TGCGCGGGGA TGCGAGGTGC TGCCGGTGGT 100
<210> 9712
<211> 100
<212> DNA
<213> Escherichia coli K-12 MG1655
<220> yehx b2130
<222> 659-758
<223> yehx b2130 U00096 complement(2214501..2215427)
<313> 9712: 680-699
<400> 9712
TGACGATGCT GACTCGTCCG GCGAATGATT TTGTCCGCCA GTTTTTTGGA 50
CGTAGTGAAC TGGGTGTGCG CCTGCTTTCG TTACGTAGTG TGGCGGATTA 100
<210> 9713
<211> 100
<212> DNA
<213> Escherichia coli K-12 MG1655
<220> yehx b2130
<222> 614-713
<223> yehx b2130 U00096 complement(2214501..2215427)
<313> 9713: 659-678
<400> 9713
TGGTATTGAT GGATCACGGT GAAGTAGTGC AGCAGGGCAA TCCGCTGACG 50
ATGCTGACTC GTCCGGCGAA TGATTTTGTC CGCCAGTTTT TTGGACGTAG 100
<210> 9714
<211> 100
<212> DNA
<213> Escherichia coli K-12 MG1655
<220> yehy b2131
<222> 1059-1158
<223> yehy b2131 U00096 complement(2215420..2216577)
<313> 9714: 1134-1153

<400> 9714
AGTGTTGCTG GGGGTGATCC CGGTAATTGT TCTGGCGGTG CTTACCGACG 50
CGCTGTTCGA TTTGCTTATC GCACTGCTGA AGGTGAAACG TAATGATTGA 100
<210> 9715
<211> 100
<212> DNA
<213> Escherichia coli K-12 MG1655
<220> yehy b2131
<222> 956-1055
<223> yehy b2131 U00096 complement(2215420..2216577)
<313> 9715: 985-1004
<400> 9715
TGATGGTGCA AACTGTAGGT ATGGCGGTGA TTGCGGCGTT AATCGGCGCA 50
GGCGGTTTTG GTGCGCTGGT TTTCCAGGGG CTGCTAAGCA GCGCCATTGA 100
<210> 9716
<211> 100
<212> DNA
<213> Escherichia coli K-12 MG1655
<220> yehy b2131
<222> 825-924
<223> yehy b2131 U00096 complement(2215420..2216577)
<313> 9716: 856-875
<400> 9716
AGTCGGTTTG AACCAGATCC CGCGCGATGT GCTGGAAAGC GCCAGAGCGA 50
TGGGGATGAG CGGGGCGCAG CGTTTCCTGC ATGTTCAGTT ACCGCTGGCG 100
<210> 9717
<211> 100
<212> DNA
<213> Escherichia coli K-12 MG1655
<220> yehz b2132
<222> 819-918
<223> yehz b2132 U00096 complement(2216584..2217501)
<313> 9717: 880-899
<400> 9717
AAAAACATTG CAGCAACTGA ATGCCAGCAT TGCTGTGGAA GGACTGGATG 50
CCAAAAAAGT GGCTGCCGAC TACCTGAAAC AAAAAGGGTG GACGAAGTAA 100
<210> 9718
<211> 100
<212> DNA
<213> Escherichia coli K-12 MG1655
<220> yehz b2132
<222> 813-912
<223> yehz b2132 U00096 complement(2216584..2217501)
<313> 9718: 841-860
<400> 9718
CGATGCAAAA ACATTGCAGC AACTGAATGC CAGCATTGCT GTGGAAGGAC 50
TGGATGCCAA AAAAGTGGCT GCCGACTACC TGAAACAAAA AGGGTGGACG 100
<210> 9719
<211> 100
<212> DNA
<213> Escherichia coli K-12 MG1655
<220> yehz b2132
<222> 782-881
<223> yehz b2132 U00096 complement(2216584..2217501)
<313> 9719: 802-821
<400> 9719
CACAGTGGCT ACAGCCAGTC TTCGCCAGCC TCGATGCAAA AACATTGCAG 50
CAACTGAATG CCAGCATTGC TGTGGAAGGA CTGGATGCCA AAAAAGTGGC 100
<210> 9720
<211> 100
<212> DNA
<213> Escherichia coli K-12 MG1655
<220> lepa b2570
<222> 1701-1800
<223> lepa b2570 U00096 complement(2703345..2705144)
<313> 9720: 1776-1795
<400> 9720
GCAGAAAGAA GGTAAGAAAC GCATGAAGCA GATCGGTAAC GTCGAGCTGC 50
CGCAGGAAGC GTTCCTCGCC ATTCTGCACG TCGGCAAAGA CAACAAATAA 100
<210> 9721
<211> 100

<212> DNA
<213> Escherichia coli K-12 MG1655
<220> lepa b2570
<222> 1700-1799
<223> lepa b2570 U00096 complement(2703345..2705144)
<313> 9721: 1725-1744
<400> 9721
AGCAGAAAGA AGGTAAGAAA CGCATGAAGC AGATCGGTAA CGTCGAGCTG 50
CCGCAGGAAG CGTTCCTCGC CATTCTGCAC GTCGGCAAAG ACAACAAATA 100
<210> 9722
<211> 100
<212> DNA
<213> Escherichia coli K-12 MG1655
<220> lepa b2570
<222> 1529-1628
<223> lepa b2570 U00096 complement(2703345..2705144)
<313> 9722: 1591-1610
<400> 9722
AGTTGGTGGA GAAGATGAAA GATCTGATCC CACGCCAGCA GTTTGATATC 50
GCCATTCAGG CAGCGATTGG TACGCACATC ATTGCGCGAT CCACCGTGAA 100
<210> 9723
<211> 100
<212> DNA
<213> Escherichia coli K-12 MG1655
<220> lepb b2569
<222> 868-967
<223> lepb b2569 U00096 complement(2702355..2703329)
<313> 9723: 910-929
<400> 9723
GAAGCGAATC TGGTCGGTCG GGCAACGGCT ATCTGGATGA GCTTCGATAA 50
GCAAGAAGGC GAATGGCCGA CTGGTCTGCG CTTAAGTCGC ATTGGCGGCA 100
<210> 9724
<211> 100
<212> DNA
<213> Escherichia coli K-12 MG1655
<220> lepb b2569
<222> 821-920
<223> lepb b2569 U00096 complement(2702355..2703329)
<313> 9724: 853-872
<400> 9724
ACAACCGCGA CAACAGCGCG GACAGCCGTT ACTGGGGCTT TGTGCCGGAA 50
GCGAATCTGG TCGGTCGGGC AACGGCTATC TGGATGAGCT TCGATAAGCA 100
<210> 9725
<211> 100
<212> DNA
<213> Escherichia coli K-12 MG1655
<220> lepb b2569
<222> 778-877
<223> lepb b2569 U00096 complement(2702355..2703329)
<313> 9725: 818-837
<400> 9725
GCAACCTGGA TTGTTCCTCC GGGACAATAC TTCATGATGG GCGACAACCG 50
CGACAACAGC GCGGACAGCC GTTACTGGGG CTTTGTGCCG GAAGCGAATC 100
<210> 9726
<211> 100
<212> DNA
<213> Escherichia coli K-12 MG1655
<220> yeia b2148
<222> 1143-1242
<223> yeia b2148 U00096 2233279..2234520
<313> 9726: 1200-1219
<400> 9726
GGGTTGTCTG CTTTGTGGTC ACGTCTGCCC GGTGGGTTGT ATTGAGCTCG 50
GGGAAGTGAA GTTTAAGAAA GGCGAGAAAG AACACCCGGT AACGTTGTAA 100
<210> 9727
<211> 100
<212> DNA
<213> Escherichia coli K-12 MG1655
<220> yeia b2148
<222> 1113-1212
<223> yeia b2148 U00096 2233279..2234520

<313> 9727: 1125-1144
<400> 9727
CACACCGCAT TGTAATACCG AGAAATGTGT GGGTTGTCTG CTTTGTGGTC 50
ACGTCTGCCC GGTGGGTTGT ATTGAGCTCG GGGAAGTGAA GTTTAAGAAA 100
<210> 9728
<211> 100
<212> DNA
<213> Escherichia coli K-12 MG1655
<220> yeia b2148
<222> 1036-1135
<223> yeia b2148 U00096 2233279..2234520
<313> 9728: 1084-1103
<400> 9728
TGTGTTGGCT GTGGACGCTG TTATATTTCC TGTTACGACG GCGGTCACCA 50
GGCGATGGAA TGGAGCGAGA AAACCCGCAC ACCGCATTGT AATACCGAGA 100
<210> 9729
<211> 100
<212> DNA
<213> Escherichia coli K-12 MG1655
<220> yeib b2153
<222> 994-1093
<223> yeib b2153 U00096 complement(2239830..2240987)
<313> 9729: 1038-1057
<400> 9729
TTTGACCGCC TGGAGCTGCT GGCGTTTGTT ATTCCGGTAT GGCTGGCGAA 50
TATCCTCTTC TCTGTTATCT GGCTGCGTTA CTTCCGCCAG GGGCCGGTGG 100
<210> 9730
<211> 100
<212> DNA
<213> Escherichia coli K-12 MG1655
<220> yeib b2153
<222> 961-1060
<223> yeib b2153 U00096 complement(2239830..2240987)
<313> 9730: 994-1013
<400> 9730
ACGCTTTTTT ACCACCTCGG TTTGTTTATG CATTTTGACC GCCTGGAGCT 50
GCTGGCGTTT GTTATTCCGG TATGGCTGGC GAATATCCTC TTCTCTGTTA 100
<210> 9731
<211> 100
<212> DNA
<213> Escherichia coli K-12 MG1655
<220> yeib b2153
<222> 902-1001
<223> yeib b2153 U00096 complement(2239830..2240987)
<313> 9731: 933-952
<400> 9731
TCGCCTGCGT CGGACGGATG GCGCTGACCA ACTATCTATT GCAAACGCTG 50
ATTTGTACCA CGCTTTTTTA CCACCTCGGT TTGTTTATGC ATTTTGACCG 100
<210> 9732
<211> 100
<212> DNA
<213> Escherichia coli K-12 MG1655
<220> yeic b2167
<222> 822-921
<223> yeic b2167 U00096 complement(2256375..2257316)
<313> 9732: 855-874
<400> 9732
ATCTGTTCGT TTCGCACAGG GATGTTCGTC AATGGCGCTC TCCTGTGAAT 50
ACACCAATAA CCCCGATTTA TCGATTGCCA ACGTTATATC GTTAGTGGAG 100
<210> 9733
<211> 100
<212> DNA
<213> Escherichia coli K-12 MG1655
<220> yeic b2167
<222> 780-879
<223> yeic b2167 U00096 complement(2256375..2257316)
<313> 9733: 830-849
<400> 9733
GGGACTTGCT TCGTGTTGGG TAGACGGAAT GCCGTTTGCC GAATCTGTTC 50
GTTTCGCACA GGGATGTTCG TCAATGGCGC TCTCCTGTGA ATACACCAAT 100
<210> 9734

<211> 100
<212> DNA
<213> Escherichia coli K-12 MG1655
<220> yeic b2167
<222> 729-828
<223> yeic b2167 U00096 complement(2256375..2257316)
<313> 9734: 788-807
<400> 9734
GATCAAAACC AATGTTATTA ATGTTACCGG AGCGGGCGAT GCCATGATGG 50
CGGGACTTGC TTCGTGTTGG GTAGACGGAA TGCCGTTTGC CGAATCTGTT 100
<210> 9735
<211> 100
<212> DNA
<213> Escherichia coli K-12 MG1655
<220> yeie b2158
<222> 783-882
<223> yeie b2158 U00096 complement(2246757..2247638)
<313> 9735: 824-843
<400> 9735
GATGCGTACG TTGTGGCGTA TACATCATCG GCAAAAACAC CTTTCCAACG 50
CGCTACGGCG CTTTCTGGAC TATTGCGATC CCGCAAATGT GCCGCGTTAA 100
<210> 9736
<211> 100
<212> DNA
<213> Escherichia coli K-12 MG1655
<220> yeie b2158
<222> 722-821
<223> yeie b2158 U00096 complement(2246757..2247638)
<313> 9736: 761-780
<400> 9736
TGATTGAAGA TCAATTGCAG GCAGGCACAT TAAGTGAAGT TGCGGTCCCT 50
CTGCCGCGCC TGATGCGTAC GTTGTGGCGT ATACATCATC GGCAAAAACA 100
<210> 9737
<211> 100
<212> DNA
<213> Escherichia coli K-12 MG1655
<220> yeie b2158
<222> 610-709
<223> yeie b2158 U00096 complement(2246757..2247638)
<313> 9737: 650-669
<400> 9737
CTGTTGCTGT CACATTTACC GAAGTTTGAG ATGGCGATGG AATTAGGTAA 50
CTCCGAGGCA ATCAAACATG CGGTGCGTCA TGGGTTGGGA ATTAGTTGCC 100
<210> 9738
<211> 100
<212> DNA
<213> Escherichia coli K-12 MG1655
<220> yeig b2155
<222> 707-806
<223> yeig b2155 U00096 2241930..2242766
<313> 9738: 758-777
<400> 9738
CAGAAGCCGC GCGCCAGAAA GCGTGGCCGA TGACGCTGCG TATTCAGCCG 50
GGATATGATC ACAGTTACTA CTTCATCGCC TCTTTTATAG AGGATCACCT 100
<210> 9739
<211> 100
<212> DNA
<213> Escherichia coli K-12 MG1655
<220> yeig b2155
<222> 635-734
<223> yeig b2155 U00096 2241930..2242766
<313> 9739: 666-685
<400> 9739
CGATCCCGAC GCTTATCGAT CAGGGCGATA ATGATCAGTT TCTTGCCGAC 50
CAGTTGCAAC CTGCGGTACT GGCAGAAGCC GCGCGCCAGA AAGCGTGGCC 100
<210> 9740
<211> 100
<212> DNA
<213> Escherichia coli K-12 MG1655
<220> yeig b2155
<222> 598-697

<223> yeig b2155 U00096 2241930..2242766
<313> 9740: 630-649
<400> 9740
AGTTGCGCAC TGATGTATGC CAGTAACGCG CAGGATGCGA TCCCGACGCT 50
TATCGATCAG GGCGATAATG ATCAGTTTCT TGCCGACCAG TTGCAACCTG 100
<210> 9741
<211> 100
<212> DNA
<213> Escherichia coli K-12 MG1655
<220> yeih b2159
<222> 951-1050
<223> yeih b2159 U00096 2247737..2248786
<313> 9741: 1008-1027
<400> 9741
AGCTGGGGCG AAACCTCTGC TGATGGCACT GGTACTGTTT GCCTGGCTGA 50
TTGTTGGTGG TGGTGCGATT AACTATGTGA TTCAAAGCGT CATCGCATAA 100
<210> 9742
<211> 100
<212> DNA
<213> Escherichia coli K-12 MG1655
<220> yeih b2159
<222> 929-1028
<223> yeih b2159 U00096 2247737..2248786
<313> 9742: 968-987
<400> 9742
CTCACGTCAG CGCGCTGAAA AAAGCTGGGG CGAAACCTCT GCTGATGGCA 50
CTGGTACTGT TTGCCTGGCT GATTGTTGGT GGTGGTGCGA TTAACTATGT 100
<210> 9743
<211> 100
<212> DNA
<213> Escherichia coli K-12 MG1655
<220> yeih b2159
<222> 835-934
<223> yeih b2159 U00096 2247737..2248786
<313> 9743: 875-894
<400> 9743
TCGTTCCACC TGTTACCGCA GAGCGTGGTG AACATGCTGG TAACGCTGGA 50
TACCTTCCTG CTGGCAATGG CGATGGCGGC GCTGGGTCTG ACCACTCACG 100
<210> 9744
<211> 100
<212> DNA
<213> Escherichia coli K-12 MG1655
<220> yeii b2161
<222> 990-1089
<223> yeii b2161 U00096 2249720..2250808
<313> 9744: 1045-1064
<400> 9744
TTTTGCGGTA GCCTGCGCGG CAATTTCGCG CGCCAGCGGC AGCTTAAACA 50
ACCCTACCCT GTCTGCCGAT AACGCGCTTT CATTAGTGCC AATGGTGTAA 100
<210> 9745
<211> 100
<212> DNA
<213> Escherichia coli K-12 MG1655
<220> yeii b2161
<222> 944-1043
<223> yeii b2161 U00096 2249720..2250808
<313> 9745: 958-977
<400> 9745
TGGTATATAG CTTTCTTGAA GGATACAGTT TCCGCGACAG CGCCCGTTTT 50
GCGGTAGCCT GCGCGGCAAT TTCGCGCGCC AGCGGCAGCT TAAACAACCC 100
<210> 9746
<211> 100
<212> DNA
<213> Escherichia coli K-12 MG1655
<220> yeii b2161
<222> 863-962
<223> yeii b2161 U00096 2249720..2250808
<313> 9746: 895-914
<400> 9746
ATGGAGAACA ATTTTTGCTG ACCGCGCCAG CGCATACGAC AGTAGACAGT 50
TTTGGTGCTG ACGATGGTTT TATGGCGGGC CTGGTATATA GCTTTCTTGA 100

<210> 9747
<211> 100
<212> DNA
<213> Escherichia coli K-12 MG1655
<220> yeij b2162
<222> 1152-1251
<223> yeij b2162 U00096 complement(2250915..2252165)
<313> 9747: 1223-1242
<400> 9747
GCCGGAAATC GCCCAGCTTG GTTTACGGGC GCTGGCGGCG GCGACGCTTT 50
CCAACTTGAT GAGTGCGACC ATTGCCGGGT TCTTTATTGG TTTAGCTTGA 100
<210> 9748
<211> 100
<212> DNA
<213> Escherichia coli K-12 MG1655
<220> yeij b2162
<222> 1124-1223
<223> yeij b2162 U00096 complement(2250915..2252165)
<313> 9748: 1153-1172
<400> 9748
CGTTTTCTGC GGTTGCGCCA CACCGTGCGC CGGAAATCGC CCAGCTTGGT 50
TTACGGGCGC TGGCGGCGGC GACGCTTTCC AACTTGATGA GTGCGACCAT 100
<210> 9749
<211> 100
<212> DNA
<213> Escherichia coli K-12 MG1655
<220> yeij b2162
<222> 1068-1167
<223> yeij b2162 U00096 complement(2250915..2252165)
<313> 9749: 1097-1116
<400> 9749
TTCCTTCGCG TTGTGCGGTT TCGCTAACTT TGGTTCTATC GGGGTGGTGG 50
TGGGGGCGTT TTCTGCGGTT GCGCCACACC GTGCGCCGGA AATCGCCCAG 100
<210> 9750
<211> 100
<212> DNA
<213> Escherichia coli K-12 MG1655
<220> yeik b2163
<222> 838-937
<223> yeik b2163 U00096 complement(2252265..2253206)
<313> 9750: 870-889
<400> 9750
GTTCTTGGCA AGCCCGCCAA TACCAAAGTC GGCATCACTA TTGATACAGA 50
CTGGTTCTGG GGATTAGTCG AAGAGTGCGT GCGCGGCTAC ATCAAAACCC 100
<210> 9751
<211> 100
<212> DNA
<213> Escherichia coli K-12 MG1655
<220> yeik b2163
<222> 768-867
<223> yeik b2163 U00096 complement(2252265..2253206)
<313> 9751: 803-822
<400> 9751
GGAGATGTAC GTCGAAGTGG ACGTCAACAG TGGCCCTTGC TATGGGCGTA 50
CCGTCTGCGA CGAGCTGGGC GTTCTTGGCA AGCCCGCCAA TACCAAAGTC 100
<210> 9752
<211> 100
<212> DNA
<213> Escherichia coli K-12 MG1655
<220> yeik b2163
<222> 704-803
<223> yeik b2163 U00096 complement(2252265..2253206)
<313> 9752: 739-758
<400> 9752
GCGGCCCGGT GCACGACGCC ACCTGCATCG GTTATCTGAT TAACCCTGAT 50
GGCATTAAAA CCCAGGAGAT GTACGTCGAA GTGGACGTCA ACAGTGGCCC 100
<210> 9753
<211> 100
<212> DNA
<213> Escherichia coli K-12 MG1655
<220> yeil b2164

<222> 474-573
<223> yeil b2164 U00096 2253375..2254034
<313> 9753: 522-541
<400> 9753
GCATACGCAA GCGGCAGAGT ATTTAGGCGT TTCTTATCGA CATCTTTTAT 50
ATGTTCTCGC GCAGTTCATT CACGACGGTT TATTAATTAA AAGCAAGAAA 100
<210> 9754
<211> 100
<212> DNA
<213> Escherichia coli K-12 MG1655
<220> yeil b2164
<222> 456-555
<223> yeil b2164 U00096 2253375..2254034
<313> 9754: 484-503
<400> 9754
TGATCTTTAT CACGAAAAGC ATACGCAAGC GGCAGAGTAT TTAGGCGTTT 50
CTTATCGACA TCTTTTATAT GTTCTCGCGC AGTTCATTCA CGACGGTTTA 100
<210> 9755
<211> 100
<212> DNA
<213> Escherichia coli K-12 MG1655
<220> yeil b2164
<222> 390-489
<223> yeil b2164 U00096 2253375..2254034
<313> 9755: 425-444
<400> 9755
AACTCAGAAT CAATCATTTC CGTTAGTGAA TCGCCTGGCA GCATTTATAT 50
TACTCTCGCA GGAAGGTGAT CTTTATCACG AAAAGCATAC GCAAGCGGCA 100
<210> 9756
<211> 100
<212> DNA
<213> Escherichia coli K-12 MG1655
<220> yeim b2165
<222> 1152-1251
<223> yeim b2165 U00096 complement(2254105..2255355)
<313> 9756: 1215-1234
<400> 9756
GCCGGAAATC GCCCAGCTTG GTTTACGGGC GCTGGCAGCA GCAACGCTTT 50
CCAACCTGAT GAGTGCGACT ATTGCCGGGT TCTTTATTGG TCTGGCGTAA 100
<210> 9757
<211> 100
<212> DNA
<213> Escherichia coli K-12 MG1655
<220> yeim b2165
<222> 1125-1224
<223> yeim b2165 U00096 complement(2254105..2255355)
<313> 9757: 1175-1194
<400> 9757
ATTTTCGGCT ATTTCGCCAA AACGCGCGCC GGAAATCGCC CAGCTTGGTT 50
TACGGGCGCT GGCAGCAGCA ACGCTTTCCA ACCTGATGAG TGCGACTATT 100
<210> 9758
<211> 100
<212> DNA
<213> Escherichia coli K-12 MG1655
<220> yeim b2165
<222> 1064-1163
<223> yeim b2165 U00096 complement(2254105..2255355)
<313> 9758: 1095-1114
<400> 9758
TTATCTCCTT TGCGCTTTGT GGTTTTGCTA ACTTTGGTTC TATCGGTGTT 50
GTCGTTGGCG CATTTTCGGC TATTTCGCCA AAACGCGCGC CGGAAATCGC 100
<210> 9759
<211> 100
<212> DNA
<213> Escherichia coli K-12 MG1655
<220> yein b2166
<222> 840-939
<223> yein b2166 U00096 complement(2255449..2256387)
<313> 9759: 906-925
<400> 9759
GACCGGCGGT GACAGCCTGA AATCCAACAT CCAGCTGGTG TTCAACAACG 50

CCATTCTGGC GAGCGAAATT GCCAAAGAAT ATCAGCGTCT CGCGGGTTAA 100
<210> 9760
<211> 100
<212> DNA
<213> Escherichia coli K-12 MG1655
<220> yein b2166
<222> 811-910
<223> yein b2166 U00096 complement(2255449..2256387)
<313> 9760: 848-867
<400> 9760
CCATTCCTGC TGGCGCGCGT TGCTGAACTG ACCGGCGGTG ACAGCCTGAA 50
ATCCAACATC CAGCTGGTGT TCAACAACGC CATTCTGGCG AGCGAAATTG 100
<210> 9761
<211> 100
<212> DNA
<213> Escherichia coli K-12 MG1655
<220> yein b2166
<222> 757-856
<223> yein b2166 U00096 complement(2255449..2256387)
<313> 9761: 790-809
<400> 9761
GATCAGGCGG TAGCTGAAGC TGAAGCTCAG GGAGTCATTG GTAAAGAAAG 50
TACGCCATTC CTGCTGGCGC GCGTTGCTGA ACTGACCGGC GGTGACAGCC 100
<210> 9762
<211> 100
<212> DNA
<213> Escherichia coli K-12 MG1655
<220> yeio b2171
<222> 1080-1179
<223> yeio b2171 U00096 2261883..2263064
<313> 9762: 1102-1121
<400> 9762
GGGCATCGTC GCCGAGATCT GGAATTATCA CGCTGTGTTC TGGTTTGCGA 50
TGGTGATGAT TATCGCCACT CTGTTTTGCT TACTGCGGAT TAAAGATGTT 100
<210> 9763
<211> 100
<212> DNA
<213> Escherichia coli K-12 MG1655
<220> yeio b2171
<222> 1028-1127
<223> yeio b2171 U00096 2261883..2263064
<313> 9763: 1051-1070
<400> 9763
CGCTCTATAC CAACACTTCG CGCGTGGGCT GGATCATCGC AGGATCAGTG 50
GCGGGCATCG TCGCCGAGAT CTGGAATTAT CACGCTGTGT TCTGGTTTGC 100
<210> 9764
<211> 100
<212> DNA
<213> Escherichia coli K-12 MG1655
<220> yeio b2171
<222> 971-1070
<223> yeio b2171 U00096 2261883..2263064
<313> 9764: 1019-1038
<400> 9764
GCATCGGGAT GCTCTATTTT CAGGATCTGA TGCCCGGTCA GGCGGGTTCA 50
GCCACCACGC TCTATACCAA CACTTCGCGC GTGGGCTGGA TCATCGCAGG 100
<210> 9765
<211> 100
<212> DNA
<213> Escherichia coli K-12 MG1655
<220> yeip b2172
<222> 729-828
<223> yeip b2172 U00096 2263215..2264042
<313> 9765: 772-791
<400> 9765
GAGCACTGGT CTGGTGATTC AGGTACCAGA ATACTTAAGC CCGGGCGAAA 50
AAATTCGTAT CCATATCGAA GAACGCCGTT ATATGGGGCG TGCTGACTAA 100
<210> 9766
<211> 100
<212> DNA
<213> Escherichia coli K-12 MG1655

<220> yeip b2172
<222> 681-780
<223> yeip b2172 U00096 2263215..2264042
<313> 9766: 725-744
<400> 9766
AGGCATCAAA GGGGCATCTG CCAGCGCCCG TAACAAACCG GCGACATTGA 50
GCACTGGTCT GGTGATTCAG GTACCAGAAT ACTTAAGCCC GGGCGAAAAA 100
<210> 9767
<211> 100
<212> DNA
<213> Escherichia coli K-12 MG1655
<220> yeip b2172
<222> 631-730
<223> yeip b2172 U00096 2263215..2264042
<313> 9767: 676-695
<400> 9767
GCGCTTGAGC TGCCGCAGAC CGTTGATCTG GAAATCGTTG AAACCGCACC 50
AGGCATCAAA GGGGCATCTG CCAGCGCCCG TAACAAACCG GCGACATTGA 100
<210> 9768
<211> 100
<212> DNA
<213> Escherichia coli K-12 MG1655
<220> yeiq b2173
<222> 1368-1467
<223> yeiq b2173 U00096 2264265..2265731
<313> 9768: 1435-1454
<400> 9768
GCCAGATAAC CCGCATTTTG TGCAGGCCAT CGAACAAGCC TGGCAACAAA 50
TCGTACAATT CGGCGCACAT CAGGCGCTAT TAAACACCCT CAAAATTTAA 100
<210> 9769
<211> 100
<212> DNA
<213> Escherichia coli K-12 MG1655
<220> yeiq b2173
<222> 1357-1456
<223> yeiq b2173 U00096 2264265..2265731
<313> 9769: 1397-1416
<400> 9769
GGTGATGATC TGCCAGATAA CCCGCATTTT GTGCAGGCCA TCGAACAAGC 50
CTGGCAACAA ATCGTACAAT TCGGCGCACA TCAGGCGCTA TTAAACACCC 100
<210> 9770
<211> 100
<212> DNA
<213> Escherichia coli K-12 MG1655
<220> yeiq b2173
<222> 1318-1417
<223> yeiq b2173 U00096 2264265..2265731
<313> 9770: 1358-1377
<400> 9770
CAACGCGTAA CCGCCCTGCT TTCCCTGCGT GAAGTTTTCG GTGATGATCT 50
GCCAGATAAC CCGCATTTTG TGCAGGCCAT CGAACAAGCC TGGCAACAAA 100
<210> 9771
<211> 100
<212> DNA
<213> Escherichia coli K-12 MG1655
<220> yeir yeiR
<222> 867-966
<223> yeir yeiR U00096 2265849..2266835
<313> 9771: 906-925
<400> 9771
CATTGAAACG CAAAACGTTG CGCCACCGGA CAGCCGTATT GAGCTGATTT 50
CCAGCAGCGA AGCTGACTGG AATGCCTTGC AGAGCGCGCT GTTGAAGCTT 100
<210> 9772
<211> 100
<212> DNA
<213> Escherichia coli K-12 MG1655
<220> yeir yeiR
<222> 811-910
<223> yeir yeiR U00096 2265849..2266835
<313> 9772: 874-893
<400> 9772

GTGCTGCGTA TTCCCGAAGG GCTGGTGCGA ATCAACCGTC AGGGCGATGA 50
CCTGCACATT GAAACGCAAA ACGTTGCGCC ACCGGACAGC CGTATTGAGC 100
<210> 9773
<211> 100
<212> DNA
<213> Escherichia coli K-12 MG1655
<220> yeir yeiR
<222> 776-875
<223> yeir yeiR U00096 2265849..2266835
<313> 9773: 817-836
<400> 9773
GGGCGCGACT TGCACCGGTG GAACGCGTCA AAGGCGTGCT GCGTATTCCC 50
GAAGGGCTGG TGCGAATCAA CCGTCAGGGC GATGACCTGC ACATTGAAAC 100
<210> 9774
<211> 100
<212> DNA
<213> Escherichia coli K-12 MG1655
<220> yeja b2178
<222> 1722-1821
<223> yeja b2178 U00096 2270378..2272198
<313> 9774: 1793-1812
<400> 9774
GCCGGCCGTG CGCCCCATCT ATAGCCTCGG TATCGATACC TGGTGGTATG 50
ACGTCAATAA AGCGGCCAAA CTGCCGTCCG CCAGCAAACA GGGAGAGTAG 100
<210> 9775
<211> 100
<212> DNA
<213> Escherichia coli K-12 MG1655
<220> yeja b2178
<222> 1585-1684
<223> yeja b2178 U00096 2270378..2272198
<313> 9775: 1645-1664
<400> 9775
ATTGCCGCGC AGGGAAATAA AGAAAAATTA CTGCCGTTGG GGCGAGCACT 50
GGATCGCGTA TTAACGTGGA ATTATTACAT GCTGCCAATG TGGTACATGG 100
<210> 9776
<211> 100
<212> DNA
<213> Escherichia coli K-12 MG1655
<220> yeja b2178
<222> 1550-1649
<223> yeja b2178 U00096 2270378..2272198
<313> 9776: 1608-1627
<400> 9776
AAAGCCCGGT TATCGACTCG CTGATCAACC AAATTATTGC CGCGCAGGGA 50
AATAAAGAAA AATTACTGCC GTTGGGGCGA GCACTGGATC GCGTATTAAC 100
<210> 9777
<211> 100
<212> DNA
<213> Escherichia coli K-12 MG1655
<220> yejb b2179
<222> 996-1095
<223> yejb b2179 U00096 2272199..2273293
<313> 9777: 1062-1081
<400> 9777
TACCTTGTAT ATTTTCACCC TGATTGGCCT GCTGCTGAAT ATTGTCAGTG 50
ATATCAGCTA TACGCTGGTT GATCCGCGTA TTGATTTTGA GGGACGTTAA 100
<210> 9778
<211> 100
<212> DNA
<213> Escherichia coli K-12 MG1655
<220> yejb b2179
<222> 959-1058
<223> yejb b2179 U00096 2272199..2273293
<313> 9778: 1023-1042
<400> 9778
AAGCGACCGT CTCGCGCGAT TATCCTGTAA TGTTTGGTAC CTTGTATATT 50
TTCACCCTGA TTGGCCTGCT GCTGAATATT GTCAGTGATA TCAGCTATAC 100
<210> 9779
<211> 100
<212> DNA

<213> Escherichia coli K-12 MG1655
<220> yejb b2179
<222> 877-976
<223> yejb b2179 U00096 2272199..2273293
<313> 9779: 907-926
<400> 9779
ACGTTTATCA GCATGTTTTT TACCGGCTCG CTGCTGATTG AGGTGATGTT 50
TTCACTCAAT GGTCTGGGCT TACTGGGCTA CGAAGCGACC GTCTCGCGCG 100
<210> 9780
<211> 100
<212> DNA
<213> Escherichia coli K-12 MG1655
<220> soha b3130
<222> 237-336
<223> soha b3130 U00096 3274643..3274978
<313> 9780: 271-290
<400> 9780
CAACCCGCAA AAAACTCGTC CATTCAACAT TCAACAAGGA AAGAAACTTG 50
TCGCTGGCAT GGACGTCAAC ATTGATGATG AGATTGGCGA CGACGAATAA 100
<210> 9781
<211> 100
<212> DNA
<213> Escherichia coli K-12 MG1655
<220> soha b3130
<222> 182-281
<223> soha b3130 U00096 3274643..3274978
<313> 9781: 239-258
<400> 9781
AGGAGGATCA TACTATGAAT GCATTTTTGC GTTTTCTGGA TGCAGATATC 50
CAGAACAACC CGCAAAAAAC TCGTCCATTC AACATTCAAC AAGGAAAGAA 100
<210> 9782
<211> 100
<212> DNA
<213> Escherichia coli K-12 MG1655
<220> soha b3130
<222> 135-234
<223> soha b3130 U00096 3274643..3274978
<313> 9782: 177-196
<400> 9782
AATTCTGCCT GGTGGGCAAG TATTTATGTG CCGACTGGGA GATGAACAGG 50
AGGATCATAC TATGAATGCA TTTTTGCGTT TTCTGGATGC AGATATCCAG 100
<210> 9783
<211> 100
<212> DNA
<213> Escherichia coli K-12 MG1655
<220> sohb b1273
<222> 925-1024
<223> sohb b1273 U00096 1327356..1328405
<313> 9783: 971-990
<400> 9783
GAAGTGGTCA ATGTACGCTA TATGCAGCGT AAACGACTCA TTGACCGATT 50
CACCGGCAGC GCGGCAGAGA GCGCCGATCG ATTGTTGCTA CGCTGGTGGC 100
<210> 9784
<211> 100
<212> DNA
<213> Escherichia coli K-12 MG1655
<220> sohb b1273
<222> 884-983
<223> sohb b1273 U00096 1327356..1328405
<313> 9784: 924-943
<400> 9784
ACACCAGTGA TGAAGTTATT CTTAGCCTGA TGGAAGGCCG TGAAGTGGTC 50
AATGTACGCT ATATGCAGCG TAAACGACTC ATTGACCGAT TCACCGGCAG 100
<210> 9785
<211> 100
<212> DNA
<213> Escherichia coli K-12 MG1655
<220> sohb b1273
<222> 805-904
<223> sohb b1273 U00096 1327356..1328405
<313> 9785: 833-852

<400> 9785
CTGGATATTG AACAGGTGGC AACGGGTGAA CACTGGTACG GACAACAGGC 50
GGTAGAGAAA GGCCTGGTTG ATGAAATCAA CACCAGTGAT GAAGTTATTC 100
<210> 9786
<211> 100
<212> DNA
<213> Escherichia coli K-12 MG1655
<220> yeje b2180
<222> 927-1026
<223> yeje b2180 U00096 2273293..2274318
<313> 9786: 996-1015
<400> 9786
TGGGATCACC GCCTTCTTGT CGGTGGCGAT ATTATTGTCT TTGCTGATCT 50
TTATTGGTGA AGCCGTCCGC GACGCATTTG ATCCTAATAA GGCGGTGTAG 100
<210> 9787
<211> 100
<212> DNA
<213> Escherichia coli K-12 MG1655
<220> yeje b2180
<222> 915-1014
<223> yeje b2180 U00096 2273293..2274318
<313> 9787: 939-958
<400> 9787
GGCCCCGTGG CTTGGGATCA CCGCCTTCTT GTCGGTGGCG ATATTATTGT 50
CTTTGCTGAT CTTTATTGGT GAAGCCGTCC GCGACGCATT TGATCCTAAT 100
<210> 9788
<211> 100
<212> DNA
<213> Escherichia coli K-12 MG1655
<220> yeje b2180
<222> 859-958
<223> yeje b2180 U00096 2273293..2274318
<313> 9788: 892-911
<400> 9788
CCGCTCGGTT CACCGTCACT CGGTGAACTG CTGTTACAAG GGAAAAATAA 50
CCTTCAGGCC CCGTGGCTTG GGATCACCGC CTTCTTGTCG GTGGCGATAT 100
<210> 9789
<211> 100
<212> DNA
<213> Escherichia coli K-12 MG1655
<220> yejf b2181
<222> 1452-1551
<223> yejf b2181 U00096 2274320..2275909
<313> 9789: 1500-1519
<400> 9789
CGATTTGCAC GTTGTCCGCG CGTTATGTCA TCAGGTTATC ATACTGCGAC 50
AAGGGGAAGT AGTGGAACAA GGACCGTGCG CGCGCGTGTT TGCCACACCG 100
<210> 9790
<211> 100
<212> DNA
<213> Escherichia coli K-12 MG1655
<220> yejf b2181
<222> 1394-1493
<223> yejf b2181 U00096 2274320..2275909
<313> 9790: 1433-1452
<400> 9790
TGACGCTATT GAAATCATTG CAACAAAAGC ATCAACTGGC CTATTTGTTT 50
ATCAGCCACG ATTTGCACGT TGTCCGCGCG TTATGTCATC AGGTTATCAT 100
<210> 9791
<211> 100
<212> DNA
<213> Escherichia coli K-12 MG1655
<220> yejf b2181
<222> 1324-1423
<223> yejf b2181 U00096 2274320..2275909
<313> 9791: 1375-1394
<400> 9791
CTTAAGCCCT CGCTGATCAT ACTTGATGAA CCGACATCAT CACTCGACAA 50
AACGGTACAG GCGCAAATAT TGACGCTATT GAAATCATTG CAACAAAAGC 100
<210> 9792
<211> 100

<212> DNA
<213> Escherichia coli K-12 MG1655
<220> yejg b2182
<222> 224-323
<223> yejg b2182 U00096 complement(2275913..2276257)
<313> 9792: 276-295
<400> 9792
TGGAGTGTGA AGGTGAGCCG TGCCTGTTTG TAAATCGCCA GGACGAGTTT 50
GCTGCAACAT GCCGATTGAA AAATTTTGGT GTGGCAATTG CCGAACCCTT 100
<210> 9793
<211> 100
<212> DNA
<213> Escherichia coli K-12 MG1655
<220> yejg b2182
<222> 144-243
<223> yejg b2182 U00096 complement(2275913..2276257)
<313> 9793: 184-203
<400> 9793
CCCGGATGGT GATAATGCAT GGTCGGTGAT GTATAAACTA AGCCAGGCGT 50
TAAGCGACAT CGAAGTTCCA TGTTCGGTGC TGGAGTGTGA AGGTGAGCCG 100
<210> 9794
<211> 100
<212> DNA
<213> Escherichia coli K-12 MG1655
<220> yejg b2182
<222> 109-208
<223> yejg b2182 U00096 complement(2275913..2276257)
<313> 9794: 156-175
<400> 9794
GGTGACAACA GCCTGGTGGC GCTTAAATTG CTTAGCCCGG ATGGTGATAA 50
TGCATGGTCG GTGATGTATA AACTAAGCCA GGCGTTAAGC GACATCGAAG 100
<210> 9795
<211> 100
<212> DNA
<213> Escherichia coli K-12 MG1655
<220> yejh b2185
<222> 1662-1761
<223> yejh b2185 U00096 2278652..2280412
<313> 9795: 1742-1761
<400> 9795
TTTTGTCGTC GCCCGCATGA AAGGCCAGTA CTGGCAGGTG CGTGAAAAAG 50
TGTTCGATTA CGAAGGTCGT TTTCGTCTGG CGCACGAATT ACGCGGTTAA 100
<210> 9796
<211> 100
<212> DNA
<213> Escherichia coli K-12 MG1655
<220> yejh b2185
<222> 1642-1741
<223> yejh b2185 U00096 2278652..2280412
<313> 9796: 1661-1680
<400> 9796
GCCTTATTGC GACACCCGGA TTTTGTCGTC GCCCGCATGA AAGGCCAGTA 50
CTGGCAGGTG CGTGAAAAAG TGTTCGATTA CGAAGGTCGT TTTCGTCTGG 100
<210> 9797
<211> 100
<212> DNA
<213> Escherichia coli K-12 MG1655
<220> yejh b2185
<222> 1576-1675
<223> yejh b2185 U00096 2278652..2280412
<313> 9797: 1630-1649
<400> 9797
CCGCATACGC GCACACCGGG CATCCCGCTG CGCTGGATCA CCGCCGCCGA 50
TATCCTCGCC CAGCAAGCCT TATTGCGACA CCCGGATTTT GTCGTCGCCC 100
<210> 9798
<211> 100
<212> DNA
<213> Escherichia coli K-12 MG1655
<220> yejk b2187
<222> 893-992
<223> yejk b2187 U00096 complement(2280960..2281967)

<313> 9798: 932-951
<400> 9798
CGATGCTGTT AGGTGAACGC ATCTTCTGGG ATCCAGCAAC CGATACCTTG 50
ACCATTAAAG GCACGCCGCC GAATTTGCGC GACCAATTGC AGCGCCGGAC 100
<210> 9799
<211> 100
<212> DNA
<213> Escherichia coli K-12 MG1655
<220> yejk b2187
<222> 840-939
<223> yejk b2187 U00096 complement(2280960..2281967)
<313> 9799: 870-889
<400> 9799
TCAGTTGACG AAGTTTGCCG GTAGCGGCGG TGGCTTGACT ATTAACTTTG 50
ATGCGATGCT GTTAGGTGAA CGCATCTTCT GGGATCCAGC AACCGATACC 100
<210> 9800
<211> 100
<212> DNA
<213> Escherichia coli K-12 MG1655
<220> yejk b2187
<222> 763-862
<223> yejk b2187 U00096 complement(2280960..2281967)
<313> 9800: 825-844
<400> 9800
GTGAGCTTCA CGGAGTTTGC GGCTGAAAAA GGCTACGAAC TGGAAGAGAG 50
CTTCCCGGCA GACCGTAGCA CACTGCGTCA GTTGACGAAG TTTGCCGGTA 100
<210> 9801
<211> 100
<212> DNA
<213> Escherichia coli K-12 MG1655
<220> yejl b2188
<222> 129-228
<223> yejl b2188 U00096 2282149..2282376
<313> 9801: 192-211
<400> 9801
CCTTATCAAC ACCAGCATTG CCCCGGCCCA ACGCCAGGCA ATTGCCAACT 50
CTTTTGCCCG CGCCTTACAG TCCTCTATCA ACGAAGACAA AGCGCACTAA 100
<210> 9802
<211> 100
<212> DNA
<213> Escherichia coli K-12 MG1655
<220> yejl b2188
<222> 85-184
<223> yejl b2188 U00096 2282149..2282376
<313> 9802: 143-162
<400> 9802
CCGACCGACC TTTCCCTGAT GGTGTTAGGC AACATGGTCA CTAACCTTAT 50
CAACACCAGC ATTGCCCCGG CCCAACGCCA GGCAATTGCC AACTCTTTTG 100
<210> 9803
<211> 100
<212> DNA
<213> Escherichia coli K-12 MG1655
<220> yejl b2188
<222> 68-167
<223> yejl b2188 U00096 2282149..2282376
<313> 9803: 92-111
<400> 9803
TGGAAAAACA TAAGGCTCCG ACCGACCTTT CCCTGATGGT GTTAGGCAAC 50
ATGGTCACTA ACCTTATCAA CACCAGCATT GCCCCGGCCC AACGCCAGGC 100
<210> 9804
<211> 100
<212> DNA
<213> Escherichia coli K-12 MG1655
<220> yejm b2189
<222> 1593-1692
<223> yejm b2189 U00096 2282396..2284156
<313> 9804: 1630-1649
<400> 9804
GGATAACGAT ACGCTGGCAA TTACCACCCC GAAAAAGACG CTGGTACTGA 50
ACAATAACGG TAAATACCGC ACTTACAACT TACGTGGTGA AAGAGTGAAA 100
<210> 9805

<211> 100
<212> DNA
<213> Escherichia coli K-12 MG1655
<220> yejm b2189
<222> 1543-1642
<223> yejm b2189 U00096 2282396..2284156
<313> 9805: 1583-1602
<400> 9805
GGTCAGGATT TGTTCAACCC TCAACGCCGT CATTACTGGG TTACCGCAGC 50
GGATAACGAT ACGCTGGCAA TTACCACCCC GAAAAAGACG CTGGTACTGA 100
<210> 9806
<211> 100
<212> DNA
<213> Escherichia coli K-12 MG1655
<220> yejm b2189
<222> 1492-1591
<223> yejm b2189 U00096 2282396..2284156
<313> 9806: 1552-1571
<400> 9806
CTGATGCAAC GCCTGCTACA TGTCAGCACA CCTGCCAGCG AATATTCGCA 50
AGGTCAGGAT TTGTTCAACC CTCAACGCCG TCATTACTGG GTTACCGCAG 100
<210> 9807
<211> 100
<212> DNA
<213> Escherichia coli K-12 MG1655
<220> yejo yejO
<222> 2412-2511
<223> yejo yejO U00096 complement(2284410..2286920)
<313> 9807: 2477-2496
<400> 9807
TACCCCGACG TTAAGCGGTC ATTTGTCAGT CAGCTATGGC AATGGCGCAG 50
GGGTAGAATC GCCGTGGAAT ACTCAGGCGG GTGTGGTCTG GACGTTCTGA 100
<210> 9808
<211> 100
<212> DNA
<213> Escherichia coli K-12 MG1655
<220> yejo yejO
<222> 2374-2473
<223> yejo yejO U00096 complement(2284410..2286920)
<313> 9808: 2428-2447
<400> 9808
ACCAGTGGCG TTTATCAGGC TGGTATAAGG TCATCGTTTA CCCCGACGTT 50
AAGCGGTCAT TTGTCAGTCA GCTATGGCAA TGGCGCAGGG GTAGAATCGC 100
<210> 9809
<211> 100
<212> DNA
<213> Escherichia coli K-12 MG1655
<220> yejo yejO
<222> 2332-2431
<223> yejo yejO U00096 complement(2284410..2286920)
<313> 9809: 2390-2409
<400> 9809
AAAGTTAATG ACGATGGCAA ATTTAATAAT GATGTGGCTG GAACCAGTGG 50
CGTTTATCAG GCTGGTATAA GGTCATCGTT TACCCCGACG TTAAGCGGTC 100
<210> 9810
<211> 100
<212> DNA
<213> Escherichia coli K-12 MG1655
<220> panb b0135
<222> 696-795
<223> panb b0135 U00096 complement(148807..149601)
<313> 9810: 750-769
<400> 9810
TTTCCTCGCC GAAACGGGCG ACATCCGCGC GGCTGTGCGG CAGTATATGG 50
CTGAAGTGGA GTCCGGCGTT TATCCGGGCG AAGAACACAG TTTCCATTAA 100
<210> 9811
<211> 100
<212> DNA
<213> Escherichia coli K-12 MG1655
<220> panb b0135
<222> 658-757

<223> panb b0135 U00096 complement(148807..149601)
<313> 9811: 701-720
<400> 9811
GGTATTACCG GCGGTCACAT TCCTAAATTC GCTAAAAATT TCCTCGCCGA 50
AACGGGCGAC ATCCGCGCGG CTGTGCGGCA GTATATGGCT GAAGTGGAGT 100
<210> 9812
<211> 100
<212> DNA
<213> Escherichia coli K-12 MG1655
<220> panb b0135
<222> 620-719
<223> panb b0135 U00096 complement(148807..149601)
<313> 9812: 644-663
<400> 9812
TCACTGACGG GCAGATCCTC GTGATGCACG ACGCCTTTGG TATTACCGGC 50
GGTCACATTC CTAAATTCGC TAAAAATTTC CTCGCCGAAA CGGGCGACAT 100
<210> 9813
<211> 100
<212> DNA
<213> Escherichia coli K-12 MG1655
<220> panc b0134
<222> 753-852
<223> panc b0134 U00096 complement(147944..148795)
<313> 9813: 831-850
<400> 9813
GCTGGAAGTT TCTGAAACCA GCAAACGGGC AGTAATTCTG GTAGCCGCCT 50
GGCTTGGCGA TGCTCGCCTG ATCGACAACA AAATGGTCGA GCTGGCGTAA 100
<210> 9814
<211> 100
<212> DNA
<213> Escherichia coli K-12 MG1655
<220> panc b0134
<222> 753-852
<223> panc b0134 U00096 complement(147944..148795)
<313> 9814: 775-794
<400> 9814
GCTGGAAGTT TCTGAAACCA GCAAACGGGC AGTAATTCTG GTAGCCGCCT 50
GGCTTGGCGA TGCTCGCCTG ATCGACAACA AAATGGTCGA GCTGGCGTAA 100
<210> 9815
<211> 100
<212> DNA
<213> Escherichia coli K-12 MG1655
<220> panc b0134
<222> 668-767
<223> panc b0134 U00096 complement(147944..148795)
<313> 9815: 709-728
<400> 9815
ATGAAATTAT TACCATTGCG GGGCAAGAAC TGAATGAAAA AGGCTTCCGC 50
GCCGATGATA TTCAGATTCG CGATGCCGAC ACATTGCTGG AAGTTTCTGA 100
<210> 9816
<211> 100
<212> DNA
<213> Escherichia coli K-12 MG1655
<220> pand b0132
<222> 282-381
<223> pand b0132 U00096 complement(146314..146694)
<313> 9816: 343-362
<400> 9816
AGATGAAGAA GCTCGCACCT GGCGACCCAA CGTCGCCTAT TTTGAAGGCG 50
ACAATGAAAT GAAACGTACC GCGAAAGCGA TTCCGGTACA GGTTGCTTGA 100
<210> 9817
<211> 100
<212> DNA
<213> Escherichia coli K-12 MG1655
<220> pand b0132
<222> 211-310
<223> pand b0132 U00096 complement(146314..146694)
<313> 9817: 260-279
<400> 9817
GTTAACGGTG CGGCGGCCCA CTGCGCCAGT GTCGGCGATA TTGTCATCAT 50
CGCCAGCTTC GTTACCATGC CAGATGAAGA AGCTCGCACC TGGCGACCCA 100

<210> 9818
<211> 100
<212> DNA
<213> Escherichia coli K-12 MG1655
<220> pand b0132
<222> 113-212
<223> pand b0132 U00096 complement(146314..146694)
<313> 9818: 132-151
<400> 9818
TTCTCGAAAA CGAAGCCATT GATATCTGGA ATGTCACCAA CGGCAAGCGT 50
TTCTCCACTT ATGCCATCGC GGCAGAACGC GGTTCGAGAA TTATTTCTGT 100
<210> 9819
<211> 100
<212> DNA
<213> Escherichia coli K-12 MG1655
<220> panf b3259
<222> 1359-1458
<223> panf b3259 U00096 3405238..3406695
<313> 9819: 1427-1446
<400> 9819
TATCGTGCCC TCGTTACTAC TAAGTTTGCT GGCTTTCCTG GTCGGAAACC 50
GTTTCGGTAC ATCCGTCCCG CAAGCTACCG TTTTGACTAC TGATAAATAA 100
<210> 9820
<211> 100
<212> DNA
<213> Escherichia coli K-12 MG1655
<220> panf b3259
<222> 1347-1446
<223> panf b3259 U00096 3405238..3406695
<313> 9820: 1365-1384
<400> 9820
GGGCTTCCAC CCTATCGTGC CCTCGTTACT ACTAAGTTTG CTGGCTTTCC 50
TGGTCGGAAA CCGTTTCGGT ACATCCGTCC CGCAAGCTAC CGTTTTGACT 100
<210> 9821
<211> 100
<212> DNA
<213> Escherichia coli K-12 MG1655
<220> panf b3259
<222> 1164-1263
<223> panf b3259 U00096 3405238..3406695
<313> 9821: 1211-1230
<400> 9821
GAAGCCGCCA GAAATGATCA TCTGGCTGAA TTTGTTGGCC TTCGGTGGGC 50
TGGAAGCCGT TTTCCTGTGG CCGCTGGTGC TGGGTCTTTA CTGGGAACGC 100
<210> 9822
<211> 100
<212> DNA
<213> Escherichia coli K-12 MG1655
<220> osmb osmB
<222> 104-203
<223> osmb osmB U00096 complement(1341134..1341352)
<313> 9822: 143-162
<400> 9822
CAATCGGCGC GGGTGCAGGG GCATTAGGCG GTGCAGTACT GACCGATGGC 50
AGTACGTTGG GTACATTAGG TGGTGCAGCT GTCGGAGGTG TTATTGGTCA 100
<210> 9823
<211> 100
<212> DNA
<213> Escherichia coli K-12 MG1655
<220> osmb osmB
<222> 79-178
<223> osmb osmB U00096 complement(1341134..1341352)
<313> 9823: 106-125
<400> 9823
TGGTCTAAAC GGGACCGCAA CACCGCAATC GGCGCGGGTG CAGGGGCATT 50
AGGCGGTGCA GTACTGACCG ATGGCAGTAC GTTGGGTACA TTAGGTGGTG 100
<210> 9824
<211> 100
<212> DNA
<213> Escherichia coli K-12 MG1655
<220> osmb osmB

<222> 16-115
<223> osmb osmB U00096 complement(1341134..1341352)
<313> 9824: 72-91
<400> 9824
AAAAAAATGA CCGCGGCTGT TCTGGCAATT ACTTTGGCAA TGTCTCTGAG 50
TGCCTGTTCT AACTGGTCTA AACGGGACCG CAACACCGCA ATCGGCGCGG 100
<210> 9825
<211> 100
<212> DNA
<213> Escherichia coli K-12 MG1655
<220> osmc osmC
<222> 333-432
<223> osmc osmC U00096 1554649..1555080
<313> 9825: 404-423
<400> 9825
CTCTACCTTT GACGGCATAA TCCAGAAAGC AAAAGCAGGA TGCCCGGTCT 50
CTCAGGTACT GAAAGCGGAA ATTACGCTGG ATTACCAGTT GAAATCGTAA 100
<210> 9826
<211> 100
<212> DNA
<213> Escherichia coli K-12 MG1655
<220> osmc osmC
<222> 307-406
<223> osmc osmC U00096 1554649..1555080
<313> 9826: 358-377
<400> 9826
GAAGTTGCGG TGCCGGGTAT TGATGCCTCT ACCTTTGACG GCATAATCCA 50
GAAAGCAAAA GCAGGATGCC CGGTCTCTCA GGTACTGAAA GCGGAAATTA 100
<210> 9827
<211> 100
<212> DNA
<213> Escherichia coli K-12 MG1655
<220> osmc osmC
<222> 240-339
<223> osmc osmC U00096 1554649..1555080
<313> 9827: 264-283
<400> 9827
CACCGCCGAT GTGTCGCTGG ATAAAGTGGA TGCCGGTTTT GCGATTACGA 50
AAATCGCACT GAAGAGTGAA GTTGCGGTGC CGGGTATTGA TGCCTCTACC 100
<210> 9828
<211> 100
<212> DNA
<213> Escherichia coli K-12 MG1655
<220> osme b1740
<222> 240-339
<223> osme b1740 U00096 complement(1819942..1820280)
<313> 9828: 301-320
<400> 9828
AGAAACCTAC TTTGTCGCGT TAGATGATAC CGGACATGTC ATCAACTCCG 50
GTTATCAGAC CTGTGCTGAA TACGACACTG ATCCACAGGC TGCGAAGTAA 100
<210> 9829
<211> 100
<212> DNA
<213> Escherichia coli K-12 MG1655
<220> osme b1740
<222> 215-314
<223> osme b1740 U00096 complement(1819942..1820280)
<313> 9829: 255-274
<400> 9829
TCCTGGGTCA ACGTGATGGT AAAGCAGAAA CCTACTTTGT CGCGTTAGAT 50
GATACCGGAC ATGTCATCAA CTCCGGTTAT CAGACCTGTG CTGAATACGA 100
<210> 9830
<211> 100
<212> DNA
<213> Escherichia coli K-12 MG1655
<220> osme b1740
<222> 165-264
<223> osme b1740 U00096 complement(1819942..1820280)
<313> 9830: 207-226
<400> 9830
TTCGTCTGAA GTGAGCATGA TCCATGCTCG CGGTACTTGC CAGACCTACA 50

TCCTGGGTCA ACGTGATGGT AAAGCAGAAA CCTACTTTGT CGCGTTAGAT 100
<210> 9831
<211> 100
<212> DNA
<213> Escherichia coli K-12 MG1655
<220> glta b0721
<222> 1163-1262
<223> glta b0721 U00096 complement(752408..753691)
<313> 9831: 1203-1222
<400> 9831
GTACCGTTGG CTGGATCGCC CACTGGAGCG AAATGCACAG TGACGGTATG 50
AAGATTGCCC GTCCGCGTCA GCTGTATACA GGATATGAAA AACGCGACTT 100
<210> 9832
<211> 100
<212> DNA
<213> Escherichia coli K-12 MG1655
<220> glta b0721
<222> 1124-1223
<223> glta b0721 U00096 complement(752408..753691)
<313> 9832: 1161-1180
<400> 9832
TTCCGTCTTC CATGTTCACC GTCATTTTCG CAATGGCACG TACCGTTGGC 50
TGGATCGCCC ACTGGAGCGA AATGCACAGT GACGGTATGA AGATTGCCCG 100
<210> 9833
<211> 100
<212> DNA
<213> Escherichia coli K-12 MG1655
<220> glta b0721
<222> 1087-1186
<223> glta b0721 U00096 complement(752408..753691)
<313> 9833: 1128-1147
<400> 9833
GATTTCTACT CTGGTATCAT CCTGAAAGCG ATGGGTATTC CGTCTTCCAT 50
GTTCACCGTC ATTTTCGCAA TGGCACGTAC CGTTGGCTGG ATCGCCCACT 100
<210> 9834
<211> 100
<212> DNA
<213> Escherichia coli K-12 MG1655
<220> osmy b4377
<222> 507-606
<223> osmy b4377 U00096 4608965..4609570
<313> 9834: 564-583
<400> 9834
CGGTACCGTC GATTCTCAGG CACAAAGTGA CCGTGCTGAA AGTATCGCCA 50
AAGCGGTAGA TGGTGTGAAA AGCGTTAAAA ATGATCTGAA AACTAAGTAA 100
<210> 9835
<211> 100
<212> DNA
<213> Escherichia coli K-12 MG1655
<220> osmy b4377
<222> 472-571
<223> osmy b4377 U00096 4608965..4609570
<313> 9835: 515-534
<400> 9835
AAAGTTGAAA CCACCGACGG CGTGGTTCAG CTCTCCGGTA CCGTCGATTC 50
TCAGGCACAA AGTGACCGTG CTGAAAGTAT CGCCAAAGCG GTAGATGGTG 100
<210> 9836
<211> 100
<212> DNA
<213> Escherichia coli K-12 MG1655
<220> osmy b4377
<222> 390-489
<223> osmy b4377 U00096 4608965..4609570
<313> 9836: 414-433
<400> 9836
GGGCTACGCG GGTGACACCG CCACCACCAG TGAAATCAAA GCCAAACTGC 50
TGGCGGACGA TATCGTCCCT TCCCGTCATG TGAAAGTTGA AACCACCGAC 100
<210> 9837
<211> 100
<212> DNA
<213> Escherichia coli K-12 MG1655

<220> gltb b3213
<222> 4453-4552
<223> gltb b3213 U00096 3352267..3356820
<313> 9837: 4498-4517
<400> 9837
GCCACTAAAT TTGCGCTGGT TAAACCGAAG TCCAGTGATG TAAAAGCACT 50
GCTGGGTCAC CGTAGTCGTA GCGCAGCTGA GTTGCGCGTG CAGGCGCAGT 100
<210> 9838
<211> 100
<212> DNA
<213> Escherichia coli K-12 MG1655
<220> gltb b3213
<222> 4420-4519
<223> gltb b3213 U00096 3352267..3356820
<313> 9838: 4467-4486
<400> 9838
GGTGAAGAGA TTCTGGCGAA CTGGTCAACC TTCGCCACTA AATTTGCGCT 50
GGTTAAACCG AAGTCCAGTG ATGTAAAAGC ACTGCTGGGT CACCGTAGTC 100
<210> 9839
<211> 100
<212> DNA
<213> Escherichia coli K-12 MG1655
<220> gltb b3213
<222> 4387-4486
<223> gltb b3213 U00096 3352267..3356820
<313> 9839: 4418-4437
<400> 9839
ACCGAGCATG TGCAGCATAC CGGCTCTCAG CGCGGTGAAG AGATTCTGGC 50
GAACTGGTCA ACCTTCGCCA CTAAATTTGC GCTGGTTAAA CCGAAGTCCA 100
<210> 9840
<211> 100
<212> DNA
<213> Escherichia coli K-12 MG1655
<220> gltd b3214
<222> 1320-1419
<223> gltd b3214 U00096 3356833..3358251
<313> 9840: 1373-1392
<400> 9840
TGCTGGCGGC GATATCGTCC GTGGTTCCGA TCTGGTGGTG ACCGCTATTG 50
CCGAAGGTCG TAAGGCGGCA GACGGTATTA TGAACTGGCT GGAAGTTTAA 100
<210> 9841
<211> 100
<212> DNA
<213> Escherichia coli K-12 MG1655
<220> gltd b3214
<222> 1258-1357
<223> gltd b3214 U00096 3356833..3358251
<313> 9841: 1285-1304
<400> 9841
GGCCGCATCA TCGCCCCGGA AGGCAGCGAC AACGCCTTCC AGACCAGCAA 50
CCCGAAAATC TTTGCTGGCG GCGATATCGT CCGTGGTTCC GATCTGGTGG 100
<210> 9842
<211> 100
<212> DNA
<213> Escherichia coli K-12 MG1655
<220> gltd b3214
<222> 1192-1291
<223> gltd b3214 U00096 3356833..3358251
<313> 9842: 1223-1242
<400> 9842
GCGTTTGGTT TCCGTCCACA CAACATGGAA TGGCTGGCAA AACACAGCGT 50
CGAGCTGGAT TCACAAGGCC GCATCATCGC CCCGGAAGGC AGCGACAACG 100
<210> 9843
<211> 100
<212> DNA
<213> Escherichia coli K-12 MG1655
<220> cpxa b3912
<222> 1275-1374
<223> cpxa b3912 U00096 complement(4101183..4102556)
<313> 9843: 1340-1359
<400> 9843

AACCGCCATT CAGCAGCATC GTGGCTGGGT GAAGGCAGAA GACAGCCCGC 50
TGGGCGGTTT ACGGCTGGTG ATTTGGTTGC CGCTGTATAA GCGGAGTTAA 100
<210> 9844
<211> 100
<212> DNA
<213> Escherichia coli K-12 MG1655
<220> cpxa b3912
<222> 1268-1367
<223> cpxa b3912 U00096 complement(4101183..4102556)
<313> 9844: 1306-1325
<400> 9844
TTGTTGAAAC CGCCATTCAG CAGCATCGTG GCTGGGTGAA GGCAGAAGAC 50
AGCCCGCTGG GCGGTTTACG GCTGGTGATT TGGTTGCCGC TGTATAAGCG 100
<210> 9845
<211> 100
<212> DNA
<213> Escherichia coli K-12 MG1655
<220> cpxa b3912
<222> 1146-1245
<223> cpxa b3912 U00096 complement(4101183..4102556)
<313> 9845: 1205-1224
<400> 9845
TACGGTGGAC GACGATGGTC CTGGCGTTAG CCCGGAAGAT CGCGAACAGA 50
TTTTCCGTCC GTTCTATCGT ACCGATGAAG CACGCGATCG TGAATCTGGC 100
<210> 9846
<211> 100
<212> DNA
<213> Escherichia coli K-12 MG1655
<220> gltf b3215
<222> 596-695
<223> gltf b3215 U00096 3358811..3359575
<313> 9846: 653-672
<400> 9846
TCGAGCGCGG TTATTCTGTT GCAAAAACAG GGGAACTGAC ACCTGTTGCT 50
ATTACAGCGG TCACCTTCCC GTTACTGATT GATGCTGCGG TAAATGACAA 100
<210> 9847
<211> 100
<212> DNA
<213> Escherichia coli K-12 MG1655
<220> gltf b3215
<222> 572-671
<223> gltf b3215 U00096 3358811..3359575
<313> 9847: 609-628
<400> 9847
GCTCGCTGAC AAGTTGCTCA GCAATCGAGC GCGGTTATTC TGTTGCAAAA 50
ACAGGGGAAC TGACACCTGT TGCTATTACA GCGGTCACCT TCCCGTTACT 100
<210> 9848
<211> 100
<212> DNA
<213> Escherichia coli K-12 MG1655
<220> gltf b3215
<222> 515-614
<223> gltf b3215 U00096 3358811..3359575
<313> 9848: 568-587
<400> 9848
CTGACCACTG GTCAGCAGCG ACCCCCACAT GGAAAGCCTC ATCTAATGGC 50
GCATTCTGCT CGCTGACAAG TTGCTCAGCA ATCGAGCGCG GTTATTCTGT 100
<210> 9849
<211> 100
<212> DNA
<213> Escherichia coli K-12 MG1655
<220> gltj b0655
<222> 642-741
<223> gltj b0655 U00096 complement(685152..685892)
<313> 9849: 708-727
<400> 9849
CATTACGCTG GCGTATGTGT TGATTAACGC TTTTATCATG CTGGTGATGA 50
CGCTGGTTGA ACGTAAAGTT CGCCTGCCTG GCAACATGGG GGGCAAATAA 100
<210> 9850
<211> 100
<212> DNA

<213> Escherichia coli K-12 MG1655
<220> gltj b0655
<222> 570-669
<223> gltj b0655 U00096 complement(685152..685892)
<313> 9850: 628-647
<400> 9850
TCTGGTGGAT ATGGCGGCGC AGGCGGGTAA ATTGCTCGAT TACTCAGCCC 50
ATGCCTGGGA GTCGTTTACC GCCATTACGC TGGCGTATGT GTTGATTAAC 100
<210> 9851
<211> 100
<212> DNA
<213> Escherichia coli K-12 MG1655
<220> gltj b0655
<222> 530-629
<223> gltj b0655 U00096 complement(685152..685892)
<313> 9851: 565-584
<400> 9851
TGAACCTGGT GAAAAACTCC GCCATCGCCT CGACTATCGG TCTGGTGGAT 50
ATGGCGGCGC AGGCGGGTAA ATTGCTCGAT TACTCAGCCC ATGCCTGGGA 100
<210> 9852
<211> 100
<212> DNA
<213> Escherichia coli K-12 MG1655
<220> gltk b0654
<222> 550-649
<223> gltk b0654 U00096 complement(684478..685152)
<313> 9852: 582-601
<400> 9852
GCCTCAACCA TTGGTGAGCG TGATGGTACG CAGGTTGAGA TGATCCTGTT 50
TGCCGGATTT GTTTATTTCG TTATTAGTCT TAGCGCGTCG TTGTTGGTCA 100
<210> 9853
<211> 100
<212> DNA
<213> Escherichia coli K-12 MG1655
<220> gltk b0654
<222> 496-595
<223> gltk b0654 U00096 complement(684478..685152)
<313> 9853: 544-563
<400> 9853
TTCCAGGATA CCTCACTGGT GTATGTGTTA TCCCTGGCCG ATTTCTTCCG 50
TACCGCCTCA ACCATTGGTG AGCGTGATGG TACGCAGGTT GAGATGATCC 100
<210> 9854
<211> 100
<212> DNA
<213> Escherichia coli K-12 MG1655
<220> gltk b0654
<222> 462-561
<223> gltk b0654 U00096 complement(684478..685152)
<313> 9854: 491-510
<400> 9854
GGTGCCGCTG CTGCTCACTC AGGGCATCGT ACTGTTCCAG GATACCTCAC 50
TGGTGTATGT GTTATCCCTG GCCGATTTCT TCCGTACCGC CTCAACCATT 100
<210> 9855
<211> 100
<212> DNA
<213> Escherichia coli K-12 MG1655
<220> gltl b0653
<222> 627-726
<223> gltl b0653 U00096 complement(683753..684478)
<313> 9855: 676-695
<400> 9855
GGACGAGGGT AAAATTGTCG AAGACTCGCC GAAAGACGCT TTCTTCGATG 50
ATCCGAAATC GGACCGCGCA AAAGACTTCC TCGCGAAAAT CCTGCATTAA 100
<210> 9856
<211> 100
<212> DNA
<213> Escherichia coli K-12 MG1655
<220> gltl b0653
<222> 618-717
<223> gltl b0653 U00096 complement(683753..684478)
<313> 9856: 639-658

<400> 9856
GATCTTTATG GACGAGGGTA AAATTGTCGA AGACTCGCCG AAAGACGCTT 50
TCTTCGATGA TCCGAAATCG GACCGCGCAA AAGACTTCCT CGCGAAAATC 100
<210> 9857
<211> 100
<212> DNA
<213> Escherichia coli K-12 MG1655
<220> gltl b0653
<222> 514-613
<223> gltl b0653 U00096 complement(683753..684478)
<313> 9857: 562-581
<400> 9857
ATCAACGAAG TACTGGACGT GATGGTGGAA CTGGCGAACG AAGGAATGAC 50
CATGATGGTG GTGACCCACG AAATGGGCTT TGCCCGTAAA GTGGCGAATC 100
<210> 9858
<211> 100
<212> DNA
<213> Escherichia coli K-12 MG1655
<220> gltp b4078
<222> 1099-1198
<223> gltp b4078 U00096 4292060..4293373
<313> 9858: 1149-1168
<400> 9858
GCAACGCTGG GTAGCGTAGG TATTCCGCTG GAAGGTCTGG CGTTTATTGC 50
TGGTGTTGAC CGTATCCTCG ACATGGCGCG TACTGCGCTG AACGTGGTGG 100
<210> 9859
<211> 100
<212> DNA
<213> Escherichia coli K-12 MG1655
<220> gltp b4078
<222> 1083-1182
<223> gltp b4078 U00096 4292060..4293373
<313> 9859: 1117-1136
<400> 9859
GTTTGTGGTG TTGCTGGCAA CGCTGGGTAG CGTAGGTATT CCGCTGGAAG 50
GTCTGGCGTT TATTGCTGGT GTTGACCGTA TCCTCGACAT GGCGCGTACT 100
<210> 9860
<211> 100
<212> DNA
<213> Escherichia coli K-12 MG1655
<220> gltp b4078
<222> 981-1080
<223> gltp b4078 U00096 4292060..4293373
<313> 9860: 1032-1051
<400> 9860
CGCGCAGTTG TATGGCATTG ACCTGTCCAT CTGGCAGGAA ATCATTCTGG 50
TGCTGACGCT GATGGTGACC TCGAAAGGGA TTGCTGGCGT GCCTGGCGTG 100
<210> 9861
<211> 100
<212> DNA
<213> Escherichia coli K-12 MG1655
<220> glts b3654
<222> 1050-1149
<223> glts b3654 U00096 complement(3825087..3826292)
<313> 9861: 1082-1101
<400> 9861
GCCAACGGCA ATCGCCAACA TGCAGGCGAT CACTGAACGC TTTGGCCCGT 50
CGCACATGGC GTTTTTGGTG GTGCCGATGG TCGGTGCGTT CTTTATCGAT 100
<210> 9862
<211> 100
<212> DNA
<213> Escherichia coli K-12 MG1655
<220> glts b3654
<222> 1003-1102
<223> glts b3654 U00096 complement(3825087..3826292)
<313> 9862: 1030-1049
<400> 9862
GCGGCAGTGC TGGCTGCGGG TCACTGTGGT TTTGGCCTCG GTGCAACGCC 50
AACGGCAATC GCCAACATGC AGGCGATCAC TGAACGCTTT GGCCCGTCGC 100
<210> 9863
<211> 100

<212> DNA
<213> Escherichia coli K-12 MG1655
<220> glts b3654
<222> 952-1051
<223> glts b3654 U00096 complement(3825087..3826292)
<313> 9863: 990-1009
<400> 9863
GCGTTGTATG CCATCTTCGT TACCTGGCGC ATGATGGGCA AAAACTACGA 50
TGCGGCAGTG CTGGCTGCGG GTCACTGTGG TTTTGGCCTC GGTGCAACGC 100
<210> 9864
<211> 100
<212> DNA
<213> Escherichia coli K-12 MG1655
<220> cpxr cpxR
<222> 596-695
<223> cpxr cpxR U00096 complement(4102553..4103251)
<313> 9864: 647-666
<400> 9864
TGCACATTTC CAACCTGCGT CGTAAACTGC CGGATCGTAA AGATGGTCAC 50
CCGTGGTTTA AAACCTTGCG TGGTCGCGGC TATCTGATGG TTTCTGCTTC 100
<210> 9865
<211> 100
<212> DNA
<213> Escherichia coli K-12 MG1655
<220> cpxr cpxR
<222> 539-638
<223> cpxr cpxR U00096 complement(4102553..4103251)
<313> 9865: 590-609
<400> 9865
ATTTAAGCCA GGAAGTGTTG GGCAAACGCC TGACGCCTTT CGACCGCGCT 50
ATTGATATGC ACATTTCCAA CCTGCGTCGT AAACTGCCGG ATCGTAAAGA 100
<210> 9866
<211> 100
<212> DNA
<213> Escherichia coli K-12 MG1655
<220> cpxr cpxR
<222> 488-587
<223> cpxr cpxR U00096 complement(4102553..4103251)
<313> 9866: 529-548
<400> 9866
CCCTGCTCTA TTTGCTGGCA CAGCATCTGG GTCAGGTGGT TTCCCGTGAA 50
CATTTAAGCC AGGAAGTGTT GGGCAAACGC CTGACGCCTT TCGACCGCGC 100
<210> 9867
<211> 100
<212> DNA
<213> Escherichia coli K-12 MG1655
<220> gltx b2401
<222> 1317-1416
<223> gltx b2401 U00096 complement(2517277..2518692)
<313> 9867: 1353-1372
<400> 9867
AGCACTGGAT GTTACCGTTC ACGCAATTGG TAAGACCCGC AGTATCGAGC 50
GTATCAACAA AGCGCTGGAT TTTATTGCTG AACGCGAAAA TCAGCAGTAA 100
<210> 9868
<211> 100
<212> DNA
<213> Escherichia coli K-12 MG1655
<220> gltx b2401
<222> 1259-1358
<223> gltx b2401 U00096 complement(2517277..2518692)
<313> 9868: 1305-1324
<400> 9868
GTATGGGTAA AGTTGGTATG CCGCTGCGTG TCGCCGTAAC CGGTGCGGGG 50
CAGTCTCCAG CACTGGATGT TACCGTTCAC GCAATTGGTA AGACCCGCAG 100
<210> 9869
<211> 100
<212> DNA
<213> Escherichia coli K-12 MG1655
<220> gltx b2401
<222> 1202-1301
<223> gltx b2401 U00096 complement(2517277..2518692)

<313> 9869: 1242-1261
<400> 9869
GGACCGCTGA AAACGTTCAT CACGCTATTC AGGCGACGGC GGATGAGCTG 50
GAAGTGGGTA TGGGTAAAGT TGGTATGCCG CTGCGTGTCG CCGTAACCGG 100
<210> 9870
<211> 100
<212> DNA
<213> Escherichia coli K-12 MG1655
<220> quea b0406
<222> 972-1071
<223> quea b0406 U00096 424235..425305
<313> 9870: 1037-1056
<400> 9870
TAAAGCAGCG GTAGAAGAGA AATATCGCTT TTTTAGTTAC GGTGATGCGA 50
TGTTTATCAC GTACAATCCG CAGGCAATTA ATGAGCGCGT CGGGGAGTAA 100
<210> 9871
<211> 100
<212> DNA
<213> Escherichia coli K-12 MG1655
<220> quea b0406
<222> 926-1025
<223> quea b0406 U00096 424235..425305
<313> 9871: 967-986
<400> 9871
TGCTGGTTTC GGCCTTTGCC GGTTATCAAC ACACCATGAA CGCCTATAAA 50
GCAGCGGTAG AAGAGAAATA TCGCTTTTTT AGTTACGGTG ATGCGATGTT 100
<210> 9872
<211> 100
<212> DNA
<213> Escherichia coli K-12 MG1655
<220> quea b0406
<222> 869-968
<223> quea b0406 U00096 424235..425305
<313> 9872: 928-947
<400> 9872
ACAAAGTGGT CGATGCGCTG GTGACGAACT TCCACTTGCC AGAGTCGACG 50
CTGATTATGC TGGTTTCGGC CTTTGCCGGT TATCAACACA CCATGAACGC 100
<210> 9873
<211> 100
<212> DNA
<213> Escherichia coli K-12 MG1655
<220> leua b0075
<222> 1473-1572
<223> leua b0075 U00096 complement(81958..83529)
<313> 9873: 1540-1559
<400> 9873
CATGGTGCAC GTTCTGAACA ATATCTGGCG TGCCGCAGAA GTCGAAAAAG 50
AGTTGCAACG CAAAGCTCAA CACAACGAAA ACAACAAGGA AACCGTGTGA 100
<210> 9874
<211> 100
<212> DNA
<213> Escherichia coli K-12 MG1655
<220> leua b0075
<222> 1350-1449
<223> leua b0075 U00096 complement(81958..83529)
<313> 9874: 1407-1426
<400> 9874
CCTGACCGCC AAAGGCCACG GTAAAGATGC GCTGGGTCAG GTGGATATCG 50
TCGCTAACTA CAACGGTCGC CGCTTCCACG GCGTCGGCCT GGCTACCGAT 100
<210> 9875
<211> 100
<212> DNA
<213> Escherichia coli K-12 MG1655
<220> leua b0075
<222> 1335-1434
<223> leua b0075 U00096 complement(81958..83529)
<313> 9875: 1348-1367
<400> 9875
ACTGGTGAAA TACAGCCTGA CCGCCAAAGG CCACGGTAAA GATGCGCTGG 50
GTCAGGTGGA TATCGTCGCT AACTACAACG GTCGCCGCTT CCACGGCGTC 100
<210> 9876

<211> 100
<212> DNA
<213> Escherichia coli K-12 MG1655
<220> leub b0074
<222> 996-1095
<223> leub b0074 U00096 complement(80867..81961)
<313> 9876: 1041-1060
<400> 9876
AGAAGGCATT CGCACCGGGG ATTTAGCCCG TGGCGCTGCC GCCGTTAGTA 50
CCGATGAAAT GGGCGATATC ATTGCCCGCT ATGTAGCAGA AGGGGTGTAA 100
<210> 9877
<211> 100
<212> DNA
<213> Escherichia coli K-12 MG1655
<220> leub b0074
<222> 966-1065
<223> leub b0074 U00096 complement(80867..81961)
<313> 9877: 1007-1026
<400> 9877
CATTGAACGC GCCATTAACC GCGCATTAGA AGAAGGCATT CGCACCGGGG 50
ATTTAGCCCG TGGCGCTGCC GCCGTTAGTA CCGATGAAAT GGGCGATATC 100
<210> 9878
<211> 100
<212> DNA
<213> Escherichia coli K-12 MG1655
<220> leub b0074
<222> 916-1015
<223> leub b0074 U00096 complement(80867..81961)
<313> 9878: 960-979
<400> 9878
CTGGCACTGC TGCTGCGTTA CAGCCTGGAT GCCGATGATG CGGCTTGCGC 50
CATTGAACGC GCCATTAACC GCGCATTAGA AGAAGGCATT CGCACCGGGG 100
<210> 9879
<211> 100
<212> DNA
<213> Escherichia coli K-12 MG1655
<220> leuc b0073
<222> 1302-1401
<223> leuc b0073 U00096 complement(79464..80864)
<313> 9879: 1376-1395
<400> 9879
CCGCCAGGGG CGCGGCGGGC GCACGCATCT GGTCAGCCCG GCAATGGCTG 50
CCGCTGCTGC TGTGACCGGA CATTTCGCCG ACATTCGCAA CATTAAATAA 100
<210> 9880
<211> 100
<212> DNA
<213> Escherichia coli K-12 MG1655
<220> leuc b0073
<222> 1225-1324
<223> leuc b0073 U00096 complement(79464..80864)
<313> 9880: 1274-1293
<400> 9880
ATGTGTCTGG CGATGAACAA CGACCGTCTG AATCCGGGCG AACGTTGTGC 50
CTCCACCAGC AACCGTAACT TTGAAGGCCG CCAGGGGCGC GGCGGGCGCA 100
<210> 9881
<211> 100
<212> DNA
<213> Escherichia coli K-12 MG1655
<220> leuc b0073
<222> 1190-1289
<223> leuc b0073 U00096 complement(79464..80864)
<313> 9881: 1235-1254
<400> 9881
AAGCCGGTTT TGAATGGCGC TTGCCTGGCT GCTCAATGTG TCTGGCGATG 50
AACAACGACC GTCTGAATCC GGGCGAACGT TGTGCCTCCA CCAGCAACCG 100
<210> 9882
<211> 100
<212> DNA
<213> Escherichia coli K-12 MG1655
<220> leud b0072
<222> 507-606

<223> leud b0072 U00096 complement(78848..79453)
<313> 9882: 570-589
<400> 9882
CCACTGCATG ATGAACGGTC TGGACAGTAT TGGGCTTACC TTGCAGCACG 50
ACGACGCCAT TGCCGCTTAT GAAGCAAAAC AACCTGCGTT TATGAATTAA 100
<210> 9883
<211> 100
<212> DNA
<213> Escherichia coli K-12 MG1655
<220> leud b0072
<222> 476-575
<223> leud b0072 U00096 complement(78848..79453)
<313> 9883: 508-527
<400> 9883
CCTATCGCTT TACCATCGAT GCCTTCCGCC GCCACTGCAT GATGAACGGT 50
CTGGACAGTA TTGGGCTTAC CTTGCAGCAC GACGACGCCA TTGCCGCTTA 100
<210> 9884
<211> 100
<212> DNA
<213> Escherichia coli K-12 MG1655
<220> leud b0072
<222> 414-513
<223> leud b0072 U00096 complement(78848..79453)
<313> 9884: 455-474
<400> 9884
TAATCCGGGG ATCCATTTCG ACGTGGATCT GGAAGCGCAA GAGGTGAAAG 50
CGGGAGAGAA AACCTATCGC TTTACCATCG ATGCCTTCCG CCGCCACTGC 100
<210> 9885
<211> 100
<212> DNA
<213> Escherichia coli K-12 MG1655
<220> glvb b3683
<222> 298-397
<223> glvb b3683 U00096 complement(3859614..3860099)
<313> 9885: 340-359
<400> 9885
CTGCATGACA TGTCACAAAC GCTGGATGAC GAAGTCTTTA AAAAGCTGGG 50
AGCGCACGGC GTCTTCCGTA GTGGCGATGC CATTCAGGTG ATCATTGGTC 100
<210> 9886
<211> 100
<212> DNA
<213> Escherichia coli K-12 MG1655
<220> glvb b3683
<222> 220-319
<223> glvb b3683 U00096 complement(3859614..3860099)
<313> 9886: 240-259
<400> 9886
GCCGGTATCC TGCAAGCCCT GGGCGGGGTC GGCAATATCT CCAGCATTAA 50
CAATTGCGCG ACGCGTTTAC GTATTGCACT GCATGACATG TCACAAACGC 100
<210> 9887
<211> 100
<212> DNA
<213> Escherichia coli K-12 MG1655
<220> glvb b3683
<222> 87-186
<223> glvb b3683 U00096 complement(3859614..3860099)
<313> 9887: 150-169
<400> 9887
ACTGATTCTG CAGTTCAACA TGTGCACGCC GGGACGTGAA GATGCGGAAG 50
TGAAACTCTA CTCAAAAGCC GAATACAAAG CCTCGCGAGG CCAAACCACC 100
<210> 9888
<211> 100
<212> DNA
<213> Escherichia coli K-12 MG1655
<220> avta b3573
<222> 1155-1254
<223> avta b3573 U00096 3737334..3738587
<313> 9888: 1213-1232
<400> 9888
ATGTATGCGC ATGAACTACG TACCAGAGCC GGAGAAAATT GAGGCGGGGG 50
TGAAGATTCT GGCGGAAGAG ATAGAAAGAG CCTGGGCTGA AAGTCACTAA 100

<210> 9889
<211> 100
<212> DNA
<213> Escherichia coli K-12 MG1655
<220> avta b3573
<222> 1056-1155
<223> avta b3573 U00096 3737334..3738587
<313> 9889: 1109-1128
<400> 9889
CAAGCAGCTC TATCAGCGCC TGAAAGCACG CGGCGTGCTG ATGGTGCCGG 50
GGCACAACTT CTTCCCAGGG CTGGATAAAC CGTGGCCGCA TACGCATCAA 100
<210> 9890
<211> 100
<212> DNA
<213> Escherichia coli K-12 MG1655
<220> avta b3573
<222> 1017-1116
<223> avta b3573 U00096 3737334..3738587
<313> 9890: 1066-1085
<400> 9890
TTTCCTCTGG CTATGGTTTA AGGATTTGCC CATTACGACC AAGCAGCTCT 50
ATCAGCGCCT GAAAGCACGC GGCGTGCTGA TGGTGCCGGG GCACAACTTC 100
<210> 9891
<211> 100
<212> DNA
<213> Escherichia coli K-12 MG1655
<220> glvc b3684
<222> 1269-1368
<223> glvc b3684 U00096 complement(3860124..3861491)
<313> 9891: 1342-1361
<400> 9891
GTTGCTGTTT GCGGTACACG CCGTGCTGGC GGCCTCAATG TCGACCGTAA 50
TGTATCTCTT TGGTGTGGTG GGCAACATGG GCGGAGGTCT GATTGACTAG 100
<210> 9892
<211> 100
<212> DNA
<213> Escherichia coli K-12 MG1655
<220> glvc b3684
<222> 1224-1323
<223> glvc b3684 U00096 complement(3860124..3861491)
<313> 9892: 1262-1281
<400> 9892
GGGAATTACC GAACCGCTGG AATTTACCTT CCTGTTCATT TCACCGTTGC 50
TGTTTGCGGT ACACGCCGTG CTGGCGGCCT CAATGTCGAC CGTAATGTAT 100
<210> 9893
<211> 100
<212> DNA
<213> Escherichia coli K-12 MG1655
<220> glvc b3684
<222> 1110-1209
<223> glvc b3684 U00096 complement(3860124..3861491)
<313> 9893: 1142-1161
<400> 9893
AAAAATCTTT GGTGCCGTGG GCATTTCTTT AGCGATGTAC TTCACTGCCG 50
CACCGGAAAA TCGGGTAAAA GTGGCGGGCT TGCTGATTCC CGCAACCTTA 100
<210> 9894
<211> 100
<212> DNA
<213> Escherichia coli K-12 MG1655
<220> sola b1060
<222> 1020-1119
<223> sola b1060 U00096 complement(1118691..1119809)
<313> 9894: 1084-1103
<400> 9894
TAAATTTGCG TCAGTTTTAG GGGAAATAGC TGCCGATTTT GCGCAAGACA 50
AAAAAAGCGA TTTTGATTTG ACGCCATTCA GGCTTTCCCG CTTCCAATAA 100
<210> 9895
<211> 100
<212> DNA
<213> Escherichia coli K-12 MG1655
<220> sola b1060

<222> 1005-1104
<223> sola b1060 U00096 complement(1118691..1119809)
<313> 9895: 1040-1059
<400> 9895
GAGTGGGCAC GGTTTTAAAT TTGCGTCAGT TTTAGGGGAA ATAGCTGCCG 50
ATTTTGCGCA AGACAAAAAA AGCGATTTTG ATTTGACGCC ATTCAGGCTT 100
<210> 9896
<211> 100
<212> DNA
<213> Escherichia coli K-12 MG1655
<220> sola b1060
<222> 950-1049
<223> sola b1060 U00096 complement(1118691..1119809)
<313> 9896: 969-988
<400> 9896
ATTTTATTAT CGATACCCTA CCCGGCCACG ATAATACACT GCTCATTACC 50
GGCCTGAGTG GGCACGGTTT TAAATTTGCG TCAGTTTTAG GGGAAATAGC 100
<210> 9897
<211> 100
<212> DNA
<213> Escherichia coli K-12 MG1655
<220> glvg b3682
<222> 540-639
<223> glvg b3682 U00096 complement(3858976..3859614)
<313> 9897: 605-624
<400> 9897
GCAAATTGTT GGGCTGCAAG ATCGCAAACA GATGCGCGTG CGCTACTACG 50
GCCTGAACCA CTGGTGGTCG GCGATATCCC GCAGTTTCAG AAAGGGTTAA 100
<210> 9898
<211> 100
<212> DNA
<213> Escherichia coli K-12 MG1655
<220> glvg b3682
<222> 520-619
<223> glvg b3682 U00096 complement(3858976..3859614)
<313> 9898: 552-571
<400> 9898
GGTATTGAAA GCCGGATGGC GCAAATTGTT GGGCTGCAAG ATCGCAAACA 50
GATGCGCGTG CGCTACTACG GCCTGAACCA CTGGTGGTCG GCGATATCCC 100
<210> 9899
<211> 100
<212> DNA
<213> Escherichia coli K-12 MG1655
<220> glvg b3682
<222> 451-550
<223> glvg b3682 U00096 complement(3858976..3859614)
<313> 9899: 498-517
<400> 9899
ATTGTCGCAG AAGCCACGCG TCGTCTGCGC CCGAATGCGA AAATCCTCAA 50
CATCTGTGAC ATGCCAATCG GTATTGAAAG CCGGATGGCG CAAATTGTTG 100
<210> 9900
<211> 100
<212> DNA
<213> Escherichia coli K-12 MG1655
<220> leul b0076
<222> 6-87
<223> leul b0076 U00096 complement(83622..83708)
<313> 9900: 20-39
<400> 9900
TCACATCGTT CGCTTTATCG GTCTACTACT ACTAAACGCA TCTTCTTTGC 50
GCGGTAGACG AGTGAGCGGC ATCCAGCATT AA 100
<210> 9901
<211> 100
<212> DNA
<213> Escherichia coli K-12 MG1655
<220> leul b0076
<222> 1-87
<223> leul b0076 U00096 complement(83622..83708)
<313> 9901: 26-45
<400> 9901
ATGACTCACA TCGTTCGCTT TATCGGTCTA CTACTACTAA ACGCATCTTC 50

TTTGCGCGGT AGACGAGTGA GCGGCATCCA GCATTAA 100
<210> 9902
<211> 100
<212> DNA
<213> Escherichia coli K-12 MG1655
<220> ppsa b1703
<222> 2280-2379
<223> ppsa b1703 U00096 complement(1782758..1785136)
<313> 9902: 2354-2373
<400> 9902
CTTTGCCGCA TGGTTGATGG AAGAGGGGAT CGATAGCCTG TCTCTGAACC 50
CGGACACCGT GGTGCAAACC TGGTTAAGCC TGGCTGAACT GAAGAAATAA 100
<210> 9903
<211> 100
<212> DNA
<213> Escherichia coli K-12 MG1655
<220> ppsa b1703
<222> 2263-2362
<223> ppsa b1703 U00096 complement(1782758..1785136)
<313> 9903: 2319-2338
<400> 9903
CCGTCCGACC ACGAAGACTT TGCCGCATGG TTGATGGAAG AGGGGATCGA 50
TAGCCTGTCT CTGAACCCGG ACACCGTGGT GCAAACCTGG TTAAGCCTGG 100
<210> 9904
<211> 100
<212> DNA
<213> Escherichia coli K-12 MG1655
<220> ppsa b1703
<222> 2216-2315
<223> ppsa b1703 U00096 complement(1782758..1785136)
<313> 9904: 2268-2287
<400> 9904
GTGCCGCGAA GAAACAGGGC AAATATGTCG GGATTTGCGG TCAGGGTCCG 50
TCCGACCACG AAGACTTTGC CGCATGGTTG ATGGAAGAGG GGATCGATAG 100
<210> 9905
<211> 100
<212> DNA
<213> Escherichia coli K-12 MG1655
<220> leuo b0077
<222> 1023-1122
<223> leuo b0077 U00096 84191..85312
<313> 9905: 1078-1097
<400> 9905
AAACAGCAGA ACCTGTTATC TCTCCTGGCA TGAAGCTGCC GGGCGCGATA 50
AAGGCCATCA GTGGATGGAA GAGCAATTAG TCTCAATTTG CAAACGCTAA 100
<210> 9906
<211> 100
<212> DNA
<213> Escherichia coli K-12 MG1655
<220> leuo b0077
<222> 985-1084
<223> leuo b0077 U00096 84191..85312
<313> 9906: 1038-1057
<400> 9906
TCCTTAGAAT TACAGGTATT ACCGCTGCCG TTAAAACAAA ACAGCAGAAC 50
CTGTTATCTC TCCTGGCATG AAGCTGCCGG GCGCGATAAA GGCCATCAGT 100
<210> 9907
<211> 100
<212> DNA
<213> Escherichia coli K-12 MG1655
<220> leuo b0077
<222> 950-1049
<223> leuo b0077 U00096 84191..85312
<313> 9907: 989-1008
<400> 9907
TTGCGCCGCG TTGGCTGGCT GAAGAGTTCG CTGAATCCTT AGAATTACAG 50
GTATTACCGC TGCCGTTAAA ACAAAACAGC AGAACCTGTT ATCTCTCCTG 100
<210> 9908
<211> 100
<212> DNA
<213> Escherichia coli K-12 MG1655

<220> leus b0643
<222> 2365-2464
<223> leus b0643 U00096 complement(671424..674006)
<313> 9908: 2411-2430
<400> 9908
GTTGCTGACG AAAAAGCGAT GGTGGAAGAC TCCACGCTGG TCGTGGTGCA 50
GGTTAACGGT AAAGTCCGTG CCAAAATCAC CGTTCCGGTG GACGCAACGG 100
<210> 9909
<211> 100
<212> DNA
<213> Escherichia coli K-12 MG1655
<220> leus b0643
<222> 2328-2427
<223> leus b0643 U00096 complement(671424..674006)
<313> 9909: 2380-2399
<400> 9909
GAAAGGCGAA GGCGATATCG ACAACGCGCC GTGGCCGGTT GCTGACGAAA 50
AAGCGATGGT GGAAGACTCC ACGCTGGTCG TGGTGCAGGT TAACGGTAAA 100
<210> 9910
<211> 100
<212> DNA
<213> Escherichia coli K-12 MG1655
<220> leus b0643
<222> 2248-2347
<223> leus b0643 U00096 complement(671424..674006)
<313> 9910: 2292-2311
<400> 9910
ATGCAGGAAG CACTGCTGGC CGTTGTCCGT ATGCTTAACC CGTTCACCCC 50
GCACATCTGC TTCACGCTGT GGCAGGAACT GAAAGGCGAA GGCGATATCG 100
<210> 9911
<211> 100
<212> DNA
<213> Escherichia coli K-12 MG1655
<220> parc parC
<222> 2089-2188
<223> parc parC U00096 complement(3161731..3163989)
<313> 9911: 2137-2156
<400> 9911
ACGCTGACCA TTCATGTTGG GAAACGCAAA ATTAAACTGC GCCCGGAAGA 50
GTTACAGAAA GTCACTGGCG AACGTGGACG CCGCGGTACG TTGATGCGCG 100
<210> 9912
<211> 100
<212> DNA
<213> Escherichia coli K-12 MG1655
<220> parc parC
<222> 2063-2162
<223> parc parC U00096 complement(3161731..3163989)
<313> 9912: 2097-2116
<400> 9912
AATTGTACGT TCTGCCGCCG CAAAGCACGC TGACCATTCA TGTTGGGAAA 50
CGCAAAATTA AACTGCGCCC GGAAGAGTTA CAGAAAGTCA CTGGCGAACG 100
<210> 9913
<211> 100
<212> DNA
<213> Escherichia coli K-12 MG1655
<220> parc parC
<222> 1966-2065
<223> parc parC U00096 complement(3161731..3163989)
<313> 9913: 2005-2024
<400> 9913
CCGGTAAGTG ATCTGCCGCA GCTGTCGAAG GGCAAAGGCA ACAAGATTAT 50
CAACATTCCA TCGGCAGAAG CCGCGCGTGG AGAAGATGGT CTGGCGCAAT 100
<210> 9914
<211> 100
<212> DNA
<213> Escherichia coli K-12 MG1655
<220> pare b3031
<222> 1794-1893
<223> pare b3031 U00096 complement(3171520..3173412)
<313> 9914: 1872-1891
<400> 9914

TACTGACGCG ATGATGGATA TGCTGCTGGC GAAGAAACGC TCGGAAGATC 50
GCCGCAACTG GTTGCAAGAG AAAGGCGACA TGGCGGAGAT TGAGGTTTAA 100
<210> 9915
<211> 100
<212> DNA
<213> Escherichia coli K-12 MG1655
<220> pare b3031
<222> 1755-1854
<223> pare b3031 U00096 complement(3171520..3173412)
<313> 9915: 1792-1811
<400> 9915
TCTGGTGCAG TTGACTATCG ATGATGAAGA CGATCAGCGT ACTGACGCGA 50
TGATGGATAT GCTGCTGGCG AAGAAACGCT CGGAAGATCG CCGCAACTGG 100
<210> 9916
<211> 100
<212> DNA
<213> Escherichia coli K-12 MG1655
<220> pare b3031
<222> 1684-1783
<223> pare b3031 U00096 complement(3171520..3173412)
<313> 9916: 1742-1761
<400> 9916
CGTTTTAAAG GTCTGGGGGA AATGAACCCG ATGCAATTGC GCGAAACCAC 50
GCTTGATCCG AACACTCGCC GTCTGGTGCA GTTGACTATC GATGATGAAG 100
<210> 9917
<211> 100
<212> DNA
<213> Escherichia coli K-12 MG1655
<220> ecpd b0141
<222> 642-741
<223> ecpd b0141 U00096 complement(155461..156201)
<313> 9917: 713-732
<400> 9917
GAAAGTCAAT GGCCTTAATG GCAAAGCGAA TTCTGCAAAA GTGCATTTTT 50
ACGCCATTAA TGACTTTGGT GGCGCAATTG AAGGTAATGC CAGGCTGTAA 100
<210> 9918
<211> 100
<212> DNA
<213> Escherichia coli K-12 MG1655
<220> ecpd b0141
<222> 633-732
<223> ecpd b0141 U00096 complement(155461..156201)
<313> 9918: 661-680
<400> 9918
TGAGGTCATG AAAGTCAATG GCCTTAATGG CAAAGCGAAT TCTGCAAAAG 50
TGCATTTTTA CGCCATTAAT GACTTTGGTG GCGCAATTGA AGGTAATGCC 100
<210> 9919
<211> 100
<212> DNA
<213> Escherichia coli K-12 MG1655
<220> ecpd b0141
<222> 537-636
<223> ecpd b0141 U00096 complement(155461..156201)
<313> 9919: 587-606
<400> 9919
TCCAACCCCT TACTACGTCT CTTTTAGCAG TGGTGATTTA GAAGCTAGCG 50
GTAAACGCTA TCCGATTGAT GTGAAAATGA TTGCACCATT TAGTGATGAG 100
<210> 9920
<211> 100
<212> DNA
<213> Escherichia coli K-12 MG1655
<220> sbm b2918
<222> 2046-2145
<223> sbm b2918 U00096 3058870..3061014
<313> 9920: 2084-2103
<400> 9920
CCTGCAAGAG CGCGGCGTGG CGGCGATTTA TGGTCCAGGT ACACCTATGC 50
TCGACAGTGT GCGCGACGTA CTGAATCTGA TAAGCCAGCA TCATGATTAA 100
<210> 9921
<211> 100
<212> DNA

<213> Escherichia coli K-12 MG1655
<220> sbm b2918
<222> 1888-1987
<223> sbm b2918 U00096 3058870..3061014
<313> 9921: 1954-1973
<400> 9921
CTGGCCGTAG AAAACGACGT TCACGTAGTG GGCGCATCCT CACTGGCTGC 50
CGGTCATAAA ACGCTGATCC CGGAACTGGT CGAAGCGCTG AAAAAATGGG 100
<210> 9922
<211> 100
<212> DNA
<213> Escherichia coli K-12 MG1655
<220> sbm b2918
<222> 1841-1940
<223> sbm b2918 U00096 3058870..3061014
<313> 9922: 1884-1903
<400> 9922
ACGTAGATTT AAGCCCGATG TTCTCTACAC CTGAAGAGAT CGCCCGCCTG 50
GCCGTAGAAA ACGACGTTCA CGTAGTGGGC GCATCCTCAC TGGCTGCCGG 100
<210> 9923
<211> 100
<212> DNA
<213> Escherichia coli K-12 MG1655
<220> uxua b4323
<222> 1070-1169
<223> uxua b4323 U00096 4549205..4550389
<313> 9923: 1106-1125
<400> 9923
TGGACGACCT GAAGAAGAAA ACCAACCCAG GTTACTCCGC AATTGGTCGT 50
CTGAAAGGCC TGGCCGAAGT TCGCGGTGTC GAACTGGCGA TCCAGCGCGC 100
<210> 9924
<211> 100
<212> DNA
<213> Escherichia coli K-12 MG1655
<220> uxua b4323
<222> 1010-1109
<223> uxua b4323 U00096 4549205..4550389
<313> 9924: 1065-1084
<400> 9924
GTAAAGCGGA AGGCAAAGAA GACCTGATCC CGATGCGTCC GGACCACGGT 50
CATCAGATGC TGGACGACCT GAAGAAGAAA ACCAACCCAG GTTACTCCGC 100
<210> 9925
<211> 100
<212> DNA
<213> Escherichia coli K-12 MG1655
<220> uxua b4323
<222> 984-1083
<223> uxua b4323 U00096 4549205..4550389
<313> 9925: 1018-1037
<400> 9925
AGCGATTGTT GAAGAAGAAC ACCGTCGTAA AGCGGAAGGC AAAGAAGACC 50
TGATCCCGAT GCGTCCGGAC CACGGTCATC AGATGCTGGA CGACCTGAAG 100
<210> 9926
<211> 100
<212> DNA
<213> Escherichia coli K-12 MG1655
<220> uxub b4324
<222> 1330-1429
<223> uxub b4324 U00096 4550470..4551930
<313> 9926: 1365-1384
<400> 9926
AAAGCGCTGC TGGGCCTGAG CGGTATTTTT GCCGATGATC TGCCGCAGAA 50
TGCCGACTTT GTTGGCGCAG TGACGGCGGC ATATCAGCAG CTGTGCGAAC 100
<210> 9927
<211> 100
<212> DNA
<213> Escherichia coli K-12 MG1655
<220> uxub b4324
<222> 1251-1350
<223> uxub b4324 U00096 4550470..4551930
<313> 9927: 1305-1324

<400> 9927
TAATGCCATT GACGTGGTCG ACCCGATGCT GGCGGAGTTC CAGAAGATCA 50
ACGCGCAGTA TCAGGGCGCA GACCGCGTGA AAGCGCTGCT GGGCCTGAGC 100
<210> 9928
<211> 100
<212> DNA
<213> Escherichia coli K-12 MG1655
<220> uxub b4324
<222> 1205-1304
<223> uxub b4324 U00096 4550470..4551930
<313> 9928: 1249-1268
<400> 9928
GCGTGGCTGG CTGGATGCGT TACACCCAGG GCGTGGATGA GCAGGGTAAT 50
GCCATTGACG TGGTCGACCC GATGCTGGCG GAGTTCCAGA AGATCAACGC 100
<210> 9929
<211> 100
<212> DNA
<213> Escherichia coli K-12 MG1655
<220> sbp b3918
<222> 891-990
<223> sbp b3918 U00096 4106414..4107403
<313> 9929: 948-967
<400> 9929
GAAGTTATTC ACCATTGATG AAGAGTTCGG CGGCTGGACG AAAGCGCAAA 50
AAGAGCATTT TGCTAACGGC GGTACGTTCG ATCAGATCAG CAAACGCTGA 100
<210> 9930
<211> 100
<212> DNA
<213> Escherichia coli K-12 MG1655
<220> sbp b3918
<222> 762-861
<223> sbp b3918 U00096 4106414..4107403
<313> 9930: 824-843
<400> 9930
TAAAGAGGTG GCGGAAGCCT ACCTGAAATA TCTCTACTCG CCAGAAGGTC 50
AGGAAATTGC CGCGAAAAAC TACTACCGTC CGCGCGACGC TGAGGTGGCG 100
<210> 9931
<211> 100
<212> DNA
<213> Escherichia coli K-12 MG1655
<220> sbp b3918
<222> 721-820
<223> sbp b3918 U00096 4106414..4107403
<313> 9931: 759-778
<400> 9931
ACCGTGTCGG TGGTCGATAA AGTGGTCGAG AAAAAAGGTA CTAAAGAGGT 50
GGCGGAAGCC TACCTGAAAT ATCTCTACTC GCCAGAAGGT CAGGAAATTG 100
<210> 9932
<211> 100
<212> DNA
<213> Escherichia coli K-12 MG1655
<220> dam b3388
<222> 728-827
<223> dam b3388 U00096 complement(3512715..3513551)
<313> 9932: 776-795
<400> 9932
ATGTCGTCAA AGTTCGACGC AGTATAAGCA GCAACGGCGG CACACGTAAA 50
AAGGTGGACG AACTGCTGGC TTTGTACAAA CCAGGAGTCG TTTCACCCGC 100
<210> 9933
<211> 100
<212> DNA
<213> Escherichia coli K-12 MG1655
<220> dam b3388
<222> 695-794
<223> dam b3388 U00096 complement(3512715..3513551)
<313> 9933: 732-751
<400> 9933
TAACGCGTGA GTGGTATCAG CGCGCAAAAT TGCATGTCGT CAAAGTTCGA 50
CGCAGTATAA GCAGCAACGG CGGCACACGT AAAAAGGTGG ACGAACTGCT 100
<210> 9934
<211> 100

<212> DNA
<213> Escherichia coli K-12 MG1655
<220> dam b3388
<222> 605-704
<223> dam b3388 U00096 complement(3512715..3513551)
<313> 9934: 634-653
<400> 9934
CGCTTGAACA ACAAGCGCAT CTGGCGGAGA TCGCCGAAGG TCTGGTTGAG 50
CGCCATATTC CAGTGCTGAT CTCCAATCAC GATACGATGT TAACGCGTGA 100
<210> 9935
<211> 100
<212> DNA
<213> Escherichia coli K-12 MG1655
<220> uxur b4325
<222> 638-737
<223> uxur b4325 U00096 4552145..4552918
<313> 9935: 668-687
<400> 9935
CCCGAGCGGC GAAGCTGGCA ATGTGGCAGC ATCTGGAAAA CGTTAAGCAA 50
CGTCTGCTGG AATTCTCGAA CGTTGACGAT ATTTATTTTG ATGGCTATCT 100
<210> 9936
<211> 100
<212> DNA
<213> Escherichia coli K-12 MG1655
<220> uxur b4325
<222> 612-711
<223> uxur b4325 U00096 4552145..4552918
<313> 9936: 635-654
<400> 9936
CCTCGCCGCG TTAATCAAAA AAGATGCCCG AGCGGCGAAG CTGGCAATGT 50
GGCAGCATCT GGAAAACGTT AAGCAACGTC TGCTGGAATT CTCGAACGTT 100
<210> 9937
<211> 100
<212> DNA
<213> Escherichia coli K-12 MG1655
<220> uxur b4325
<222> 406-505
<223> uxur b4325 U00096 4552145..4552918
<313> 9937: 453-472
<400> 9937
CTGGCTTCCA GTGCGCCGGG CAGCAGCGAA AGCGGTGACA TGCAGTTCCA 50
TCTCGCTATT GCCGAAGCAA CGCATAACAG CATGCTGGTG GAGCTGTTCC 100
<210> 9938
<211> 100
<212> DNA
<213> Escherichia coli K-12 MG1655
<220> lexa b4044
<222> 510-609
<223> lexa b4044 U00096 4254694..4255302
<313> 9938: 577-596
<400> 9938
AAATAGCGAG TTTAAACCAA TTGTCGTTGA CCTTCGTCAG CAGAGCTTCA 50
CCATTGAAGG GCTGGCGGTT GGGGTTATTC GCAACGGCGA CTGGCTGTAA 100
<210> 9939
<211> 100
<212> DNA
<213> Escherichia coli K-12 MG1655
<220> lexa b4044
<222> 496-595
<223> lexa b4044 U00096 4254694..4255302
<313> 9939: 540-559
<400> 9939
GAACTGTTGC CAGAAAATAG CGAGTTTAAA CCAATTGTCG TTGACCTTCG 50
TCAGCAGAGC TTCACCATTG AAGGGCTGGC GGTTGGGGTT ATTCGCAACG 100
<210> 9940
<211> 100
<212> DNA
<213> Escherichia coli K-12 MG1655
<220> lexa b4044
<222> 428-527
<223> lexa b4044 U00096 4254694..4255302

<313> 9940: 464-483
<400> 9940
AGGTCGTTGT CGCACGTATT GATGACGAAG TTACCGTTAA GCGCCTGAAA 50
AAACAGGGCA ATAAAGTCGA ACTGTTGCCA GAAAATAGCG AGTTTAAACC 100
<210> 9941
<211> 100
<212> DNA
<213> Escherichia coli K-12 MG1655
<220> glya b2552
<222> 1145-1244
<223> glya b2552 U00096 complement(2682274..2683527)
<313> 9941: 1196-1215
<400> 9941
TGGCTGGCTG GATGTGTGAC GTGCTGGACA GCATCAATGA TGAAGCCGTT 50
ATCGAGCGCA TCAAAGGTAA AGTTCTCGAC ATCTGCGCAC GTTACCCGGT 100
<210> 9942
<211> 100
<212> DNA
<213> Escherichia coli K-12 MG1655
<220> glya b2552
<222> 1129-1228
<223> glya b2552 U00096 complement(2682274..2683527)
<313> 9942: 1161-1180
<400> 9942
GCCGAAGCGA AAGAACTGGC TGGCTGGATG TGTGACGTGC TGGACAGCAT 50
CAATGATGAA GCCGTTATCG AGCGCATCAA AGGTAAAGTT CTCGACATCT 100
<210> 9943
<211> 100
<212> DNA
<213> Escherichia coli K-12 MG1655
<220> glya b2552
<222> 1038-1137
<223> glya b2552 U00096 complement(2682274..2683527)
<313> 9943: 1083-1102
<400> 9943
AAACAGCGTA CCGAACGATC CGAAGAGCCC GTTTGTGACC TCCGGTATTC 50
GTGTAGGTAC TCCGGCGATT ACCCGTCGCG GCTTTAAAGA AGCCGAAGCG 100
<210> 9944
<211> 100
<212> DNA
<213> Escherichia coli K-12 MG1655
<220> glyq b3561
<222> 813-912
<223> glyq b3561 U00096 complement(3722036..3722947)
<313> 9944: 860-879
<400> 9944
CTACATTCTG CGCATTCGCA CCCTGACCAA AGCAGTGGCA GAAGCATACT 50
ACGCTTCCCG TGAAGCCCTC GGCTTCCCGA TGTGCAACAA AGATAAGTAA 100
<210> 9945
<211> 100
<212> DNA
<213> Escherichia coli K-12 MG1655
<220> glyq b3561
<222> 715-814
<223> glyq b3561 U00096 complement(3722036..3722947)
<313> 9945: 750-769
<400> 9945
CCGCTGCCGC TGCCAGCCTA CGAGCGTATT CTGAAAGCCG CCCACAGCTT 50
CAACCTGCTG GATGCGCGTA AAGCCATCTC CGTCACCGAG CGTCAGCGCT 100
<210> 9946
<211> 100
<212> DNA
<213> Escherichia coli K-12 MG1655
<220> glyq b3561
<222> 613-712
<223> glyq b3561 U00096 complement(3722036..3722947)
<313> 9946: 673-692
<400> 9946
GAGCAGTCCA CTTACAACTT CGAATACGCG GATGTGGACT TCCTGTTCAC 50
CTGCTTCGAG CAGTACGAGA AAGAAGCGCA GCAGCTGCTG GCGCTGGAAA 100
<210> 9947

<211> 100
<212> DNA
<213> Escherichia coli K-12 MG1655
<220> glys b3560
<222> 1971-2070
<223> glys b3560 U00096 complement(3719957..3722026)
<313> 9947: 2044-2063
<400> 9947
CATGGTTGAT GACAAAGAAT TGCGTATCAA CCGTCTGACC ATGCTGGAGA 50
AACTGCGCGA ACTGTTCCTG CGCGTTGCGG ATATTTCGCT GTTGCAATAA 100
<210> 9948
<211> 100
<212> DNA
<213> Escherichia coli K-12 MG1655
<220> glys b3560
<222> 1940-2039
<223> glys b3560 U00096 complement(3719957..3722026)
<313> 9948: 1992-2011
<400> 9948
CGGTTGATGC TTTCTTCGAT AAAGTGATGG TCATGGTTGA TGACAAAGAA 50
TTGCGTATCA ACCGTCTGAC CATGCTGGAG AAACTGCGCG AACTGTTCCT 100
<210> 9949
<211> 100
<212> DNA
<213> Escherichia coli K-12 MG1655
<220> glys b3560
<222> 1873-1972
<223> glys b3560 U00096 complement(3719957..3722026)
<313> 9949: 1915-1934
<400> 9949
CTGGAGCCGT ACTTTACGGA AGGTCGTTAC CAGGATGCGC TGGTCGAACT 50
GGCTGAGCTG CGTGAACCGG TTGATGCTTT CTTCGATAAA GTGATGGTCA 100
<210> 9950
<211> 100
<212> DNA
<213> Escherichia coli K-12 MG1655
<220> ymba b0953
<222> 450-549
<223> ymba b0953 U00096 1014134..1014682
<313> 9950: 516-535
<400> 9950
GCAAACTCAG GATGGTTACG ATGAGATGGT TAAAGTGCTG GCCGGTGTCT 50
GGAGTCAGGA AGCCGCTTCT ATTGCACAAG AGATAAAGCG TCTACCTTAA 100
<210> 9951
<211> 100
<212> DNA
<213> Escherichia coli K-12 MG1655
<220> ymba b0953
<222> 413-512
<223> ymba b0953 U00096 1014134..1014682
<313> 9951: 445-464
<400> 9951
GACAACTGAT CAAACGTCCG TTCCGTCTGG AAGGAGTGCA AACTCAGGAT 50
GGTTACGATG AGATGGTTAA AGTGCTGGCC GGTGTCTGGA GTCAGGAAGC 100
<210> 9952
<211> 100
<212> DNA
<213> Escherichia coli K-12 MG1655
<220> ymba b0953
<222> 353-452
<223> ymba b0953 U00096 1014134..1014682
<313> 9952: 402-421
<400> 9952
TTAACGGTCG CTATGATGGC AAGGTCATTG TCAGTGGTGA GTGGCTGTTG 50
AACCACCAGG GACAACTGAT CAAACGTCCG TTCCGTCTGG AAGGAGTGCA 100
<210> 9953
<211> 100
<212> DNA
<213> Escherichia coli K-12 MG1655
<220> dcd b2066
<222> 483-582

<223> dcd b2066 U00096 complement(2139656..2140237)
<313> 9953: 551-570
<400> 9953
GCCGCTTTCC GGCCCGGCGG TGCGACCTTA CAACCGCCGT GAAGATGCGA 50
AATATCGCAA CCAGCAGGGC GCGGTAGCCA GCCGAATCGA TAAAGACTAA 100
<210> 9954
<211> 100
<212> DNA
<213> Escherichia coli K-12 MG1655
<220> dcd b2066
<222> 461-560
<223> dcd b2066 U00096 complement(2139656..2140237)
<313> 9954: 520-539
<400> 9954
TAATTGGTGC GCTGAGCTTT GAGCCGCTTT CCGGCCCGGC GGTGCGACCT 50
TACAACCGCC GTGAAGATGC GAAATATCGC AACCAGCAGG GCGCGGTAGC 100
<210> 9955
<211> 100
<212> DNA
<213> Escherichia coli K-12 MG1655
<220> dcd b2066
<222> 200-299
<223> dcd b2066 U00096 complement(2139656..2140237)
<313> 9955: 231-250
<400> 9955
GCGTGATGAG CGATGAGATC GTTCTCGACG AGGGCGAGGC GTTCTATCTT 50
CACCCAGGAG AGCTGGCGCT GGCGGTGACG CTGGAGTCGG TGACGCTGCC 100
<210> 9956
<211> 100
<212> DNA
<213> Escherichia coli K-12 MG1655
<220> dcm b1962
<222> 1320-1419
<223> dcm b1962 U00096 complement(2028921..2030339)
<313> 9956: 1390-1409
<400> 9956
GCCGGTCTTT GCCGCGGTGG CAAAACTGCT TGAGCCAAAA ATCAAACAGG 50
CGGTGGCGTT GCGTCAGCAA GAGGCACAAC ATGGCCGACG TTCACGATAA 100
<210> 9957
<211> 100
<212> DNA
<213> Escherichia coli K-12 MG1655
<220> dcm b1962
<222> 1248-1347
<223> dcm b1962 U00096 complement(2028921..2030339)
<313> 9957: 1310-1329
<400> 9957
GGGAGAAGCG AAATTCCGTA TTCCGGTTTC GGACACTCAG GCCTATCGCC 50
AGTTCGGTAA CTCGGTGGTC GTGCCGGTCT TTGCCGCGGT GGCAAAACTG 100
<210> 9958
<211> 100
<212> DNA
<213> Escherichia coli K-12 MG1655
<220> dcm b1962
<222> 1228-1327
<223> dcm b1962 U00096 complement(2028921..2030339)
<313> 9958: 1259-1278
<400> 9958
TTAATGGGTT TTGAAGCGCC GGGAGAAGCG AAATTCCGTA TTCCGGTTTC 50
GGACACTCAG GCCTATCGCC AGTTCGGTAA CTCGGTGGTC GTGCCGGTCT 100
<210> 9959
<211> 100
<212> DNA
<213> Escherichia coli K-12 MG1655
<220> dcp b1539
<222> 1947-2046
<223> dcp b1539 U00096 complement(1623359..1625404)
<313> 9959: 2018-2037
<400> 9959
CAGAGGTAAC AGCGAAGATC TGGAACGCCT GTATCGACAA TGGCGCGGTA 50
AGGCACCTAA GATTATGCCG ATGCTGCAAC ATCGTGGCTT GAACATATAA 100

<210> 9960
<211> 100
<212> DNA
<213> Escherichia coli K-12 MG1655
<220> dcp b1539
<222> 1937-2036
<223> dcp b1539 U00096 complement(1623359..1625404)
<313> 9960: 1981-2000
<400> 9960
CGATCCTTTC CAGAGGTAAC AGCGAAGATC TGGAACGCCT GTATCGACAA 50
TGGCGCGGTA AGGCACCTAA GATTATGCCG ATGCTGCAAC ATCGTGGCTT 100
<210> 9961
<211> 100
<212> DNA
<213> Escherichia coli K-12 MG1655
<220> dcp b1539
<222> 1758-1857
<223> dcp b1539 U00096 complement(1623359..1625404)
<313> 9961: 1780-1799
<400> 9961
GGATCTTCCT GCTATACCGC CACGCTATCG CAGCAGTTAT TTCGCCCATA 50
TTTTTGGTGG CGGATATGCT GCAGGTTATT ACGCTTATCT GTGGACGCAA 100
<210> 9962
<211> 100
<212> DNA
<213> Escherichia coli K-12 MG1655
<220> ymca b0985
<222> 1998-2097
<223> ymca b0985 U00096 complement(1045072..1047168)
<313> 9962: 2047-2066
<400> 9962
TTCGTGGACA CCATTGACGC GTGATGGTGG ACAAATGCTG TCACGCAAAT 50
ACCAGCTCTA TCCAATGACG GCAGAGCGAG AAGTACCGGT TGGACAATAA 100
<210> 9963
<211> 100
<212> DNA
<213> Escherichia coli K-12 MG1655
<220> ymca b0985
<222> 1901-2000
<223> ymca b0985 U00096 complement(1045072..1047168)
<313> 9963: 1957-1976
<400> 9963
AAGATGACTA CGGCGAAGGC GGCTTTAGTA AAGGTTTTTA TATCTCGATT 50
CCATTCGACT TGATGACCAT TGGACCTAAC CGCAACCGCG CGGTGGTTTC 100
<210> 9964
<211> 100
<212> DNA
<213> Escherichia coli K-12 MG1655
<220> ymca b0985
<222> 1882-1981
<223> ymca b0985 U00096 complement(1045072..1047168)
<313> 9964: 1902-1921
<400> 9964
GCAATCAGTA ACGTATCTAA AGATGACTAC GGCGAAGGCG GCTTTAGTAA 50
AGGTTTTTAT ATCTCGATTC CATTCGACTT GATGACCATT GGACCTAACC 100
<210> 9965
<211> 100
<212> DNA
<213> Escherichia coli K-12 MG1655
<220> ymcb b0986
<222> 578-677
<223> ymcb b0986 U00096 complement(1047168..1047914)
<313> 9965: 620-639
<400> 9965
CTGAGGGTGA GACTGTCGTC GCCCCTGTTG CCCTGTGGAA TAAAAGACAT 50
GTGGAACCAC CGCCTGGCAG CCAGCTTTGG TTGGGCTTTT CTGCGCATGT 100
<210> 9966
<211> 100
<212> DNA
<213> Escherichia coli K-12 MG1655
<220> ymcb b0986

<222> 402-501
<223> ymcb b0986 U00096 complement(1047168..1047914)
<313> 9966: 438-457
<400> 9966
GTTGGTCGGG GACTATACGC TCTATACCGT ACAGCGTCCG GTGACTATTA 50
CTCTGCTGGG TGCGGTTTCT GGGGCCGGGC AATTGCCCTG GCAGGCAGGC 100
<210> 9967
<211> 100
<212> DNA
<213> Escherichia coli K-12 MG1655
<220> ymcb b0986
<222> 279-378
<223> ymcb b0986 U00096 complement(1047168..1047914)
<313> 9967: 326-345
<400> 9967
CGACGATGTG GCGGCAACGA TTAAATCCGT GCGCCAGCAG TTACTCAATC 50
TCAATATCAC TGGTCGCTTA CCGGTCAAAC TGGACCCTGA TTTTGTACGA 100
<210> 9968
<211> 100
<212> DNA
<213> Escherichia coli K-12 MG1655
<220> ymcc b0987
<222> 546-645
<223> ymcc b0987 U00096 complement(1047911..1048555)
<313> 9968: 593-612
<400> 9968
CTGGGTCGAT TCGGCTACTG GCCAGGTTCG TCAAAGTCGG CAAATGTTAG 50
GCGCAGGGGT TATTCCCGTA GAAATGACGT TTCTTAAACC CGCACCATGA 100
<210> 9969
<211> 100
<212> DNA
<213> Escherichia coli K-12 MG1655
<220> ymcc b0987
<222> 481-580
<223> ymcc b0987 U00096 complement(1047911..1048555)
<313> 9969: 514-533
<400> 9969
CTGTGTAATG TCTGGCAGGA AGAGGTGACT TCCACGCGGC CAGAAAAACA 50
GTGGCAAAAC ACATTCTGGG TCGATTCGGC TACTGGCCAG GTTCGTCAAA 100
<210> 9970
<211> 100
<212> DNA
<213> Escherichia coli K-12 MG1655
<220> ymcc b0987
<222> 454-553
<223> ymcc b0987 U00096 complement(1047911..1048555)
<313> 9970: 486-505
<400> 9970
ACGCTGAATA TTGCGGGTCG CAATGTGCTG TGTAATGTCT GGCAGGAAGA 50
GGTGACTTCC ACGCGGCCAG AAAAACAGTG GCAAAACACA TTCTGGGTCG 100
<210> 9971
<211> 100
<212> DNA
<213> Escherichia coli K-12 MG1655
<220> ymcd b0988
<222> 225-324
<223> ymcd b0988 U00096 complement(1048662..1048985)
<313> 9971: 297-316
<400> 9971
AACGGCTGTT GGTGTGGCGA CGTTGGCAGT TGTTGCAGCA AATGACAGTG 50
GCGATGGCGG TTCTCATAAC ACCTCAACCA CCACGTCCAC TACACGCTAA 100
<210> 9972
<211> 100
<212> DNA
<213> Escherichia coli K-12 MG1655
<220> ymcd b0988
<222> 106-205
<223> ymcd b0988 U00096 complement(1048662..1048985)
<313> 9972: 138-157
<400> 9972
GAGGCAACGA CGGGTACTAC TGGCACCACA ACGACCACTA CCGGCGCAAC 50

CACGACTGCT ACTACCACTG GTGGCGTGGC TGCTGGCGCT GTCGGCACGG 100
<210> 9973
<211> 100
<212> DNA
<213> Escherichia coli K-12 MG1655
<220> ymcd b0988
<222> 51-150
<223> ymcd b0988 U00096 complement(1048662..1048985)
<313> 9973: 97-116
<400> 9973
CTTCATGTTG ACGACGCCAG CTGCGTTTGC TGCCCCTGAA GCAACAAACG 50
GGACCGAGGC AACGACGGGT ACTACTGGCA CCACAACGAC CACTACCGGC 100
<210> 9974
<211> 100
<212> DNA
<213> Escherichia coli K-12 MG1655
<220> ttda b3062
<222> 813-912
<223> ttda b3062 U00096 3204104..3205015
<313> 9974: 881-900
<400> 9974
TGCTGTCTCT ACCGGCTGCT GGGCGCATCG TCGCGGCACG CTGCTGGTTC 50
ATGCCGATCT CACCTTTGAA AATCTGTCTC ACACCCGGAG CGCGTTATGA 100
<210> 9975
<211> 100
<212> DNA
<213> Escherichia coli K-12 MG1655
<220> ttda b3062
<222> 731-830
<223> ttda b3062 U00096 3204104..3205015
<313> 9975: 781-800
<400> 9975
GTCCACAAGG GCTGACCGGC AACAGTTCAG TGATGGGCGT ACATATCGAA 50
TCTGCCGCCC GCCATCCGTC AACCATCGGC GTTGCTGTCT CTACCGGCTG 100
<210> 9976
<211> 100
<212> DNA
<213> Escherichia coli K-12 MG1655
<220> ttda b3062
<222> 721-820
<223> ttda b3062 U00096 3204104..3205015
<313> 9976: 743-762
<400> 9976
CTGGGGATTG GTCCACAAGG GCTGACCGGC AACAGTTCAG TGATGGGCGT 50
ACATATCGAA TCTGCCGCCC GCCATCCGTC AACCATCGGC GTTGCTGTCT 100
<210> 9977
<211> 100
<212> DNA
<213> Escherichia coli K-12 MG1655
<220> ttdb b3063
<222> 507-606
<223> ttdb b3063 U00096 3205012..3205617
<313> 9977: 569-588
<400> 9977
CCACGGCAAC AACCTGATAG CCGAAAACAA AAAGCTGTTC GCCGAACGCC 50
GCGATCCCAT CGTGGAAGAG ATCTGCGAGC ACGTCCATTA CATCAAATAA 100
<210> 9978
<211> 100
<212> DNA
<213> Escherichia coli K-12 MG1655
<220> ttdb b3063
<222> 507-606
<223> ttdb b3063 U00096 3205012..3205617
<313> 9978: 535-554
<400> 9978
CCACGGCAAC AACCTGATAG CCGAAAACAA AAAGCTGTTC GCCGAACGCC 50
GCGATCCCAT CGTGGAAGAG ATCTGCGAGC ACGTCCATTA CATCAAATAA 100
<210> 9979
<211> 100
<212> DNA
<213> Escherichia coli K-12 MG1655

<220> ttdb b3063
<222> 473-572
<223> ttdb b3063 U00096 3205012..3205617
<313> 9979: 487-506
<400> 9979
AAGAGTTCGG CCCGCTGATT GTCTCTATTG ATACCCACGG CAACAACCTG  50
ATAGCCGAAA ACAAAAAGCT GTTCGCCGAA CGCCGCGATC CCATCGTGGA  100
<210> 9980
<211> 100
<212> DNA
<213> Escherichia coli K-12 MG1655
<220> ddg ddg
<222> 888-987
<223> ddg ddg U00096 2493599..2494585
<313> 9980: 951-970
<400> 9980
GATTATCGAG AAAGAGATCA TGCGCGCACC GGAGCAGTAC CTCTGGATCC  50
ACCGTCGCTT TAAAACGCGC CCGGTGGGAG AATCGTCGTT GTACATTTAA  100
<210> 9981
<211> 100
<212> DNA
<213> Escherichia coli K-12 MG1655
<220> ddg ddg
<222> 831-930
<223> ddg ddg U00096 2493599..2494585
<313> 9981: 852-871
<400> 9981
CCCAGAGATG GAAGGCTACC CGACAGATGA AAATCAAGCC GCTGCCTATA  50
TGAACAAGAT TATCGAGAAA GAGATCATGC GCGCACCGGA GCAGTACCTC  100
<210> 9982
<211> 100
<212> DNA
<213> Escherichia coli K-12 MG1655
<220> ddg ddg
<222> 782-881
<223> ddg ddg U00096 2493599..2494585
<313> 9982: 811-830
<400> 9982
TAACGATGGT AAGAAAAGCG GATTACAGCG GATATCGTTT GTTCATCACC  50
CCAGAGATGG AAGGCTACCC GACAGATGAA AATCAAGCCG CTGCCTATAT  100
<210> 9983
<211> 100
<212> DNA
<213> Escherichia coli K-12 MG1655
<220> kup b3748
<222> 1414-1513
<223> kup b3748 U00096 3928943..3930502
<313> 9983: 1453-1472
<400> 9983
CGCGTGCCCG GGACCGCGGT GTATATGTCG CGTGCAATCA ACGTCATTCC  50
CTTTGCGCTG ATGCATAACC TTAAACATAA CAAGGTATTG CATGAGCGGG  100
<210> 9984
<211> 100
<212> DNA
<213> Escherichia coli K-12 MG1655
<220> kup b3748
<222> 1352-1451
<223> kup b3748 U00096 3928943..3930502
<313> 9984: 1388-1407
<400> 9984
TGCATGAACA TGGTAACTCT CTGGAAGCGA TGATTGCTTC GCTGGAGAAA  50
TCACCGCCTG TTCGCGTGCC CGGGACCGCG GTGTATATGT CGCGTGCAAT  100
<210> 9985
<211> 100
<212> DNA
<213> Escherichia coli K-12 MG1655
<220> kup b3748
<222> 1317-1416
<223> kup b3748 U00096 3928943..3930502
<313> 9985: 1351-1370
<400> 9985

CTGGAAGAGC GAGCGTTTCC GCTTGCTGCG GCGGATGCAT GAACATGGTA 50
ACTCTCTGGA AGCGATGATT GCTTCGCTGG AGAAATCACC GCCTGTTCGC 100
<210> 9986
<211> 100
<212> DNA
<213> Escherichia coli K-12 MG1655
<220> dnaa b3703
<222> 1255-1354
<223> dnaa b3703 U00096 complement(3879954..3881357)
<313> 9986: 1304-1323
<400> 9986
AACCACAGTC TGCCGGAGAT TGGCGATGCG TTTGGTGGCC GTGACCACAC 50
GACGGTGCTT CATGCCTGCC GTAAGATCGA GCAGTTGCGT GAAGAGAGCC 100
<210> 9987
<211> 100
<212> DNA
<213> Escherichia coli K-12 MG1655
<220> dnaa b3703
<222> 1221-1320
<223> dnaa b3703 U00096 complement(3879954..3881357)
<313> 9987: 1259-1278
<400> 9987
CCAGATGGCG ATGGCGCTGG CGAAAGAGCT GACTAACCAC AGTCTGCCGG 50
AGATTGGCGA TGCGTTTGGT GGCCGTGACC ACACGACGGT GCTTCATGCC 100
<210> 9988
<211> 100
<212> DNA
<213> Escherichia coli K-12 MG1655
<220> dnaa b3703
<222> 1132-1231
<223> dnaa b3703 U00096 complement(3879954..3881357)
<313> 9988: 1191-1210
<400> 9988
AATATTCAGA AGACGGTGGC GGAGTACTAC AAGATCAAAG TCGCGGATCT 50
CCTTTCCAAG CGTCGATCCC GCTCGGTGGC GCGTCCGCGC CAGATGGCGA 100
<210> 9989
<211> 100
<212> DNA
<213> Escherichia coli K-12 MG1655
<220> dnab b4053
<222> 1317-1416
<223> dnab b4053 U00096 4261893..4263308
<313> 9989: 1395-1414
<400> 9989
TAAACAACGT AACGGCCCAA TCGGGACGGT ACGCCTGACC TTTAACGGTC 50
AATGGTCGCG CTTCGACAAC TATGCGGGGC CGCAGTACGA CGACGAATAA 100
<210> 9990
<211> 100
<212> DNA
<213> Escherichia coli K-12 MG1655
<220> dnab b4053
<222> 1306-1405
<223> dnab b4053 U00096 4261893..4263308
<313> 9990: 1340-1359
<400> 9990
ATTATTATCG GTAAACAACG TAACGGCCCA ATCGGGACGG TACGCCTGAC 50
CTTTAACGGT CAATGGTCGC GCTTCGACAA CTATGCGGGG CCGCAGTACG 100
<210> 9991
<211> 100
<212> DNA
<213> Escherichia coli K-12 MG1655
<220> dnab b4053
<222> 1243-1342
<223> dnab b4053 U00096 4261893..4263308
<313> 9991: 1274-1293
<400> 9991
ATCATGTTTA TCTATCGTGA TGAGGTGTAT CACGAAAACA GTGATTTAAA 50
AGGCATCGCG GAAATTATTA TCGGTAAACA ACGTAACGGC CCAATCGGGA 100
<210> 9992
<211> 100
<212> DNA

<213> Escherichia coli K-12 MG1655
<220> dnac b4362
<222> 639-738
<223> dnac b4362 U00096 complement(4597807..4598544)
<313> 9992: 713-732
<400> 9992
GGGCGAACGC GTGATGGACC GTATGCGCCT GGGTAACAGT TTGTGGGTGA 50
TCTTCAACTG GGATAGCTAC CGTAGCCGGG TAACAGGTAA AGAGTATTAA 100
<210> 9993
<211> 100
<212> DNA
<213> Escherichia coli K-12 MG1655
<220> dnac b4362
<222> 585-684
<223> dnac b4362 U00096 complement(4597807..4598544)
<313> 9993: 625-644
<400> 9993
ACGCCCAACC GGGATGCTGA CCAACAGTAA TATGGAAGAG ATGACCAAGT 50
TGCTGGGCGA ACGCGTGATG GACCGTATGC GCCTGGGTAA CAGTTTGTGG 100
<210> 9994
<211> 100
<212> DNA
<213> Escherichia coli K-12 MG1655
<220> dnac b4362
<222> 553-652
<223> dnac b4362 U00096 complement(4597807..4598544)
<313> 9994: 573-592
<400> 9994
AACCAGATCG TCGATCGCCG CTCTTCTTCC AAACGCCCAA CCGGGATGCT 50
GACCAACAGT AATATGGAAG AGATGACCAA GTTGCTGGGC GAACGCGTGA 100
<210> 9995
<211> 100
<212> DNA
<213> Escherichia coli K-12 MG1655
<220> zwf b1853
<222> 1368-1467
<223> zwf b1853 U00096 complement(1932863..1934338)
<313> 9995: 1397-1416
<400> 9995
GGCGATGGAC AATGATGCGC CGAAACCGTA TCAGGCCGGA ACCTGGGGAC 50
CCGTTGCCTC GGTGGCGATG ATTACCCGTG ATGGTCGTTC CTGGAATGAG 100
<210> 9996
<211> 100
<212> DNA
<213> Escherichia coli K-12 MG1655
<220> zwf b1853
<222> 1317-1416
<223> zwf b1853 U00096 complement(1932863..1934338)
<313> 9996: 1347-1366
<400> 9996
CGACGAAGTG GAAGAAGCCT GGAAATGGGT AGACTCCATT ACTGAGGCGT 50
GGGCGATGGA CAATGATGCG CCGAAACCGT ATCAGGCCGG AACCTGGGGA 100
<210> 9997
<211> 100
<212> DNA
<213> Escherichia coli K-12 MG1655
<220> zwf b1853
<222> 1203-1302
<223> zwf b1853 U00096 complement(1932863..1934338)
<313> 9997: 1226-1245
<400> 9997
CACCAAGCTG GATCTGAGCT ATTCAGAAAC CTTTAATCAG ACGCATCTGG 50
CGGATGCCTA TGAACGTTTG CTGCTGGAAA CCATGCGTGG TATTCAGGCA 100
<210> 9998
<211> 100
<212> DNA
<213> Escherichia coli K-12 MG1655
<220> dnae b0185
<222> 3384-3483
<223> dnae b0185 U00096 205126..208608
<313> 9998: 3460-3479

<400> 9998
GCGTTTTGGC GCGACGTGGC GTGTCTCTCC GAGCGATCGT TTATTAAACG 50
ATCTCCGTGG CCTCATTGGT TCGGAGCAGG TGGAACTGGA GTTTGACTAA 100
<210> 9999
<211> 100
<212> DNA
<213> Escherichia coli K-12 MG1655
<220> dnae b0185
<222> 3293-3392
<223> dnae b0185 U00096 205126..208608
<313> 9999: 3336-3355
<400> 9999
TAAACCGACT CCGTCAGTCT CTGGAACCCC ACCGCTCTGG GACAATTCCA 50
GTACATCTCT ACTATCAGAG GGCGGATGCA CGCGCGCGGT TGCGTTTTGG 100
<210> 10000
<211> 100
<212> DNA
<213> Escherichia coli K-12 MG1655
<220> dnae b0185
<222> 3249-3348
<223> dnae b0185 U00096 205126..208608
<313> 10000: 3293-3312
<400> 10000
GCTTGCTATC TCGCTGACGG ACAGGCAAAT TGATGACCAG CTTTTAAACC 50
GACTCCGTCA GTCTCTGGAA CCCCACCGCT CTGGGACAAT TCCAGTACAT 100
<210> 10001
<211> 100
<212> DNA
<213> Escherichia coli K-12 MG1655
<220> dnag b3067
<222> 1609-1708
<223> dnag b3067 U00096 3208748..3210493
<313> 10001: 1642-1661
<400> 10001
GACTCACTCA ACCATATGTT TGATTCGCTG CTTGAACTGC GCCAGGAAGA 50
GTTAATCGCT CGTGAGCGCA CGCATGGTTT AAGCAACGAA GAACGCCTGG 100
<210> 10002
<211> 100
<212> DNA
<213> Escherichia coli K-12 MG1655
<220> dnag b3067
<222> 1550-1649
<223> dnag b3067 U00096 3208748..3210493
<313> 10002: 1595-1614
<400> 10002
AAAAACTGTC GATGTGGGAC GATATAGCAG ATAAGAATAT TGCTGAGCAA 50
ACCTTCACCG ACTCACTCAA CCATATGTTT GATTCGCTGC TTGAACTGCG 100
<210> 10003
<211> 100
<212> DNA
<213> Escherichia coli K-12 MG1655
<220> dnag b3067
<222> 1507-1606
<223> dnag b3067 U00096 3208748..3210493
<313> 10003: 1547-1566
<400> 10003
CTTTTAGAGC ACTATCGTGG TACAAATAAT GCTGCCACCC TTGAAAAACT 50
GTCGATGTGG GACGATATAG CAGATAAGAA TATTGCTGAG CAAACCTTCA 100
<210> 10004
<211> 100
<212> DNA
<213> Escherichia coli K-12 MG1655
<220> sfa b0992
<222> 132-231
<223> sfa b0992 U00096 1051070..1051300
<313> 10004: 206-225
<400> 10004
GGCTTATAAC ATTTCTTACC TGACCAGGGT ACCGGGAACC AACACCTTAC 50
TGGCGTGTTG CTGTCTTTTA AGACCAGAAG AGGTTAACAG TGAATATTGA 100
<210> 10005
<211> 100

<212> DNA
<213> Escherichia coli K-12 MG1655
<220> sfa b0992
<222> 132-231
<223> sfa b0992 U00096 1051070..1051300
<313> 10005: 180-199
<400> 10005
GGCTTATAAC ATTTCTTACC TGACCAGGGT ACCGGGAACC AACACCTTAC 50
TGGCGTGTTG CTGTCTTTTA AGACCAGAAG AGGTTAACAG TGAATATTGA 100
<210> 10006
<211> 100
<212> DNA
<213> Escherichia coli K-12 MG1655
<220> sfa b0992
<222> 91-190
<223> sfa b0992 U00096 1051070..1051300
<313> 10006: 120-139
<400> 10006
GTAAGCGATA ACCTGTTGAT TATTGAATCT TTCGGGGAGA TGGCTTATAA 50
CATTTCTTAC CTGACCAGGG TACCGGGAAC CAACACCTTA CTGGCGTGTT 100
<210> 10007
<211> 100
<212> DNA
<213> Escherichia coli K-12 MG1655
<220> dnaj b0016
<222> 1008-1107
<223> dnaj b0016 U00096 14168..15298
<313> 10007: 1057-1076
<400> 10007
GCAGAAACAG CTGCTGCAAG AGCTGCAAGA AAGCTTCGGT GGCCCAACCG 50
GCGAGCACAA CAGCCCGCGC TCAAAGAGCT TCTTTGATGG TGTGAAGAAG 100
<210> 10008
<211> 100
<212> DNA
<213> Escherichia coli K-12 MG1655
<220> dnaj b0016
<222> 918-1017
<223> dnaj b0016 U00096 14168..15298
<313> 10008: 971-990
<400> 10008
CGGTAAAGGC GTCAAGTCTG TCCGCGGTGG CGCACAGGGT GATTTGCTGT 50
GCCGCGTTGT CGTCGAAACA CCGGTAGGCC TGAACGAAAG GCAGAAACAG 100
<210> 10009
<211> 100
<212> DNA
<213> Escherichia coli K-12 MG1655
<220> dnaj b0016
<222> 815-914
<223> dnaj b0016 U00096 14168..15298
<313> 10009: 873-892
<400> 10009
CTATGGCGGC GCTGGGTGGC GAAATCGAAG TACCGACCCT TGATGGTCGC 50
GTCAAACTGA AAGTGCCTGG CGAAACCCAG ACCGGTAAGC TATTCCGTAT 100
<210> 10010
<211> 100
<212> DNA
<213> Escherichia coli K-12 MG1655
<220> dnak b0015
<222> 1818-1917
<223> dnak b0015 U00096 12163..14079
<313> 10010: 1853-1872
<400> 10010
TGCCCAGCAG CAGACTGCCG GTGCTGATGC TTCTGCAAAC AACGCGAAAG 50
ATGACGATGT TGTCGACGCT GAATTTGAAG AAGTCAAAGA CAAAAAATAA 100
<210> 10011
<211> 100
<212> DNA
<213> Escherichia coli K-12 MG1655
<220> dnak b0015
<222> 1763-1862
<223> dnak b0015 U00096 12163..14079

<313> 10011: 1806-1825
<400> 10011
TGCAGGAACT GGCACAGGTT TCCCAGAAAC TGATGGAAAT CGCCCAGCAG 50
CAACATGCCC AGCAGCAGAC TGCCGGTGCT GATGCTTCTG CAAACAACGC 100
<210> 10012
<211> 100
<212> DNA
<213> Escherichia coli K-12 MG1655
<220> dnak b0015
<222> 1717-1816
<223> dnak b0015 U00096 12163..14079
<313> 10012: 1777-1796
<400> 10012
GAAACTGCTC TGAAAGGTGA AGACAAAGCC GCTATCGAAG CGAAAATGCA 50
GGAACTGGCA CAGGTTTCCC AGAAACTGAT GGAAATCGCC CAGCAGCAAC 100
<210> 10013
<211> 100
<212> DNA
<213> Escherichia coli K-12 MG1655
<220> dnan b3702
<222> 1002-1101
<223> dnan b3702 U00096 complement(3878849..3879949)
<313> 10013: 1079-1098
<400> 10013
AAACGTCCGC ATGATGCTGA CCGATTCGGT TTCCAGCGTG CAGATTGAAG 50
ATGCGGCCAG CCAGAGCGCG GCTTATGTTG TCATGCCAAT GAGACTGTAA 100
<210> 10014
<211> 100
<212> DNA
<213> Escherichia coli K-12 MG1655
<220> dnan b3702
<222> 999-1098
<223> dnan b3702 U00096 complement(3878849..3879949)
<313> 10014: 1028-1047
<400> 10014
CGAAAACGTC CGCATGATGC TGACCGATTC GGTTTCCAGC GTGCAGATTG 50
AAGATGCGGC CAGCCAGAGC GCGGCTTATG TTGTCATGCC AATGAGACTG 100
<210> 10015
<211> 100
<212> DNA
<213> Escherichia coli K-12 MG1655
<220> dnan b3702
<222> 953-1052
<223> dnan b3702 U00096 complement(3878849..3879949)
<313> 10015: 983-1002
<400> 10015
GCTTCAACGT CAGTTATGTG CTGGATGTTC TGAACGCGCT GAAATGCGAA 50
AACGTCCGCA TGATGCTGAC CGATTCGGTT TCCAGCGTGC AGATTGAAGA 100
<210> 10016
<211> 100
<212> DNA
<213> Escherichia coli K-12 MG1655
<220> dnaq b0216
<222> 633-732
<223> dnaq b0216 U00096 236067..236798
<313> 10016: 711-730
<400> 10016
GTTACGCGTT GTTTTTGCGA CAGATGAAGA GATTGCAGCT CATGAAGCCC 50
GTCTCGATCT GGTGCAGAAG AAAGGCGGAA GTTGCCTCTG GCGAGCATAA 100
<210> 10017
<211> 100
<212> DNA
<213> Escherichia coli K-12 MG1655
<220> dnaq b0216
<222> 633-732
<223> dnaq b0216 U00096 236067..236798
<313> 10017: 653-672
<400> 10017
GTTACGCGTT GTTTTTGCGA CAGATGAAGA GATTGCAGCT CATGAAGCCC 50
GTCTCGATCT GGTGCAGAAG AAAGGCGGAA GTTGCCTCTG GCGAGCATAA 100
<210> 10018

<211> 100
<212> DNA
<213> Escherichia coli K-12 MG1655
<220> dnaq b0216
<222> 567-666
<223> dnaq b0216 U00096 236067..236798
<313> 10018: 609-628
<400> 10018
GGAAGGAGAG ACACAACAGC AACAAGGTGA AGCAACAATT CAGCGCATTG 50
TACGTCAGGC AAGTAAGTTA CGCGTTGTTT TTGCGACAGA TGAAGAGATT 100
<210> 10019
<211> 100
<212> DNA
<213> Escherichia coli K-12 MG1655
<220> dnat b4363
<222> 441-540
<223> dnat b4363 U00096 complement(4598547..4599086)
<313> 10019: 513-532
<400> 10019
CCTGCAAATC GGTCGTGCCA GCAACGGCGG ACTGCCGAAA CGAGATGTGA 50
ATACGGTCAG CGAACCTGAC AGCCAAATTC CACCAGGATT CAGAGGGTAA 100
<210> 10020
<211> 100
<212> DNA
<213> Escherichia coli K-12 MG1655
<220> dnat b4363
<222> 353-452
<223> dnat b4363 U00096 complement(4598547..4599086)
<313> 10020: 407-426
<400> 10020
CCGAAGAACT GGCCTCATTC ATTGCCTACT GGCAGGCGGA AGGTAAAGTC 50
TTTCACCATG TGCAGTGGCA ACAAAAACTG GCGCGCAGCC TGCAAATCGG 100
<210> 10021
<211> 100
<212> DNA
<213> Escherichia coli K-12 MG1655
<220> dnat b4363
<222> 336-435
<223> dnat b4363 U00096 complement(4598547..4599086)
<313> 10021: 375-394
<400> 10021
AAGAGAGCCG GTGACCACCG AAGAACTGGC CTCATTCATT GCCTACTGGC 50
AGGCGGAAGG TAAAGTCTTT CACCATGTGC AGTGGCAACA AAAACTGGCG 100
<210> 10022
<211> 100
<212> DNA
<213> Escherichia coli K-12 MG1655
<220> molr_1 b2116
<222> 726-825
<223> molr_1 b2116 U00096 2194494..2195318
<313> 10022: 794-813
<400> 10022
ACAGATTGAT ATTGAATGGG ATTATGCGAA TAATGTCATT ATTATTCTGC 50
CGTCTGGCAT TGCACCTAGC TACTTGGAGC AATATTCCAG ATTTGAATGA 100
<210> 10023
<211> 100
<212> DNA
<213> Escherichia coli K-12 MG1655
<220> molr_1 b2116
<222> 648-747
<223> molr_1 b2116 U00096 2194494..2195318
<313> 10023: 691-710
<400> 10023
AGTGCGTGAG GATGTTATGG ATCAGATCGT CCAGGAAGGC GGTCTGGAAT 50
ATGCTACTGA TGTAATAATA CACCTTCAAC AGATTGATAT TGAATGGGAT 100
<210> 10024
<211> 100
<212> DNA
<213> Escherichia coli K-12 MG1655
<220> molr_1 b2116
<222> 585-684

<223> molr_1 b2116 U00096 2194494..2195318
<313> 10024: 611-630
<400> 10024
ACAGTTACCA TCACCAGTAA TGGCTGTACT CACGGCACTT GAAATGAAAT 50
GCACAAGATA TAAAGTGCGT GAGGATGTTA TGGATCAGAT CGTCCAGGAA 100
<210> 10025
<211> 100
<212> DNA
<213> Escherichia coli K-12 MG1655
<220> dnax b0471
<222> 1833-1932
<223> dnax b0471 U00096 491316..493247
<313> 10025: 1903-1922
<400> 10025
TGCGCAGGCG CGCGAGTCCA TTATTGCGGA TAATAATATT CAGACCCTGC 50
GTCGGTTCTT CGATGCGGAG CTGGATGAAG AAAGTATCCG CCCCATTTGA 100
<210> 10026
<211> 100
<212> DNA
<213> Escherichia coli K-12 MG1655
<220> dnax b0471
<222> 1624-1723
<223> dnax b0471 U00096 491316..493247
<313> 10026: 1660-1679
<400> 10026
AATGCCTGGA AAGAGGAGAG CGACAACGCA GTATGTCTGC ATTTGCGCTC 50
CTCTCAGCGG CATTTGAACA ACCGCGGTGC ACAGCAAAAA CTGGCTGAAG 100
<210> 10027
<211> 100
<212> DNA
<213> Escherichia coli K-12 MG1655
<220> dnax b0471
<222> 1587-1686
<223> dnax b0471 U00096 491316..493247
<313> 10027: 1620-1639
<400> 10027
ACTTTCGCTA CCAAAACTGG TCGAACAGGT GGCGTTAAAT GCCTGGAAAG 50
AGGAGAGCGA CAACGCAGTA TGTCTGCATT TGCGCTCCTC TCAGCGGCAT 100
<210> 10028
<211> 100
<212> DNA
<213> Escherichia coli K-12 MG1655
<220> molr_2 b2117
<222> 1806-1905
<223> molr_2 b2117 U00096 2195430..2197367
<313> 10028: 1855-1874
<400> 10028
CATCGCCCAG AAACTGAAAT TCAAAGCATT ACAGGAGCGG GCAAAAGAAA 50
AAATTGCCGA CATTGCCGAG AGCCGCGAAC TCACGGTGGC GGAGCTTGAA 100
<210> 10029
<211> 100
<212> DNA
<213> Escherichia coli K-12 MG1655
<220> molr_2 b2117
<222> 1762-1861
<223> molr_2 b2117 U00096 2195430..2197367
<313> 10029: 1817-1836
<400> 10029
CTCGCTGCTA TCGGTAGTGA TATCGCCCTT ATGCAGCTTA ACGGCATCGC 50
CCAGAAACTG AAATTCAAAG CATTACAGGA GCGGGCAAAA GAAAAAATTG 100
<210> 10030
<211> 100
<212> DNA
<213> Escherichia coli K-12 MG1655
<220> molr_2 b2117
<222> 1663-1762
<223> molr_2 b2117 U00096 2195430..2197367
<313> 10030: 1713-1732
<400> 10030
GTTCTCGGTA ACGATGACAC CGCCCGCAAA CTGACGCCAT TAATACGCGC 50
CTGGCCTGGT GAATCCCAGC ATAAACGCGC CACCGTTGGG TTGGATATTC 100

<210> 10031
<211> 100
<212> DNA
<213> Escherichia coli K-12 MG1655
<220> molr_3 b2118
<222> 903-1002
<223> molr_3 b2118 U00096 2197288..2198289
<313> 10031: 950-969
<400> 10031
TGGCTGGGGG AGTAATTCAA CACAGGAAGC GCAGTTCTCC GTAATCGATG 50
CCATCACCGC CAGCGAGCTA ATTAACGATA TTGAAGCCCT GTTTGAATAA 100
<210> 10032
<211> 100
<212> DNA
<213> Escherichia coli K-12 MG1655
<220> molr_3 b2118
<222> 855-954
<223> molr_3 b2118 U00096 2197288..2198289
<313> 10032: 911-930
<400> 10032
GCAGCTCTTA AGCAAGCTGT GGCTATGGGA AGGCAAAGCA GAAAGATATG 50
GCTGGGGGAG TAATTCAACA CAGGAAGCGC AGTTCTCCGT AATCGATGCC 100
<210> 10033
<211> 100
<212> DNA
<213> Escherichia coli K-12 MG1655
<220> molr_3 b2118
<222> 738-837
<223> molr_3 b2118 U00096 2197288..2198289
<313> 10033: 788-807
<400> 10033
ACCACAGGAT GGAGGCTGGA TCGGATGGAT GATCAAACCT TTGGGTCGCT 50
GGTCGTTAAT CATGGAAATC GATGAAGGCT TTGCGGTAGG CATGTCGCCA 100
<210> 10034
<211> 100
<212> DNA
<213> Escherichia coli K-12 MG1655
<220> ymdc b1047
<222> 1260-1359
<223> ymdc b1047 U00096 1105518..1106999
<313> 10034: 1310-1329
<400> 10034
CGTGATAGAG AGCGAAACGC TGGCACAGTT AATTGATAAA CGCTTTATTC 50
AGAGCCAGTA TGATGCGGCC TGGCAGCTCC GTCTGGACAG GTGGGGACGG 100
<210> 10035
<211> 100
<212> DNA
<213> Escherichia coli K-12 MG1655
<220> ymdc b1047
<222> 1230-1329
<223> ymdc b1047 U00096 1105518..1106999
<313> 10035: 1253-1272
<400> 10035
TTCAACATTG CTCAATACTG AAATGGGCTT CGTGATAGAG AGCGAAACGC 50
TGGCACAGTT AATTGATAAA CGCTTTATTC AGAGCCAGTA TGATGCGGCC 100
<210> 10036
<211> 100
<212> DNA
<213> Escherichia coli K-12 MG1655
<220> ymdc b1047
<222> 1195-1294
<223> ymdc b1047 U00096 1105518..1106999
<313> 10036: 1216-1235
<400> 10036
ACGGTGTTTA TCGGCTCTTT CAATTTCGAT CCGCGTTCAA CATTGCTCAA 50
TACTGAAATG GGCTTCGTGA TAGAGAGCGA AACGCTGGCA CAGTTAATTG 100
<210> 10037
<211> 100
<212> DNA
<213> Escherichia coli K-12 MG1655
<220> ivbl b3673

<222> 1-99
<223> ivbl b3673 U00096 complement(3850517..3850615)
<313> 10037: 22-41
<400> 10037
ATGACTACTT CCATGCTCAA CGCAAAACTA CTACCAACTG CGCCATCCGC 50
CGCAGTGGTC GTCGTGCGTG TGGTGGTGGT CGTCGGCAAT GCGCCGTAG 100
<210> 10038
<211> 100
<212> DNA
<213> Escherichia coli K-12 MG1655
<220> ivbl b3673
<222> 1-99
<223> ivbl b3673 U00096 complement(3850517..3850615)
<313> 10038: 22-41
<400> 10038
ATGACTACTT CCATGCTCAA CGCAAAACTA CTACCAACTG CGCCATCCGC 50
CGCAGTGGTC GTCGTGCGTG TGGTGGTGGT CGTCGGCAAT GCGCCGTAG 100
<210> 10039
<211> 100
<212> DNA
<213> Escherichia coli K-12 MG1655
<220> ymdd b1048
<222> 1059-1158
<223> ymdd b1048 U00096 complement(1107007..1108164)
<313> 10039: 1133-1152
<400> 10039
AATTCTGTAT GAAATTCATT TGCGCATCCC GTTACTGAAG TTTTTGTTCT 50
CTGGTAAACC GGTTGTTAAG CGTGAGAACG ATAAAGCACC AGCCCGTTAA 100
<210> 10040
<211> 100
<212> DNA
<213> Escherichia coli K-12 MG1655
<220> ymdd b1048
<222> 972-1071
<223> ymdd b1048 U00096 complement(1107007..1108164)
<313> 10040: 1025-1044
<400> 10040
GTTTTTCGGC GCATACATTA CACCGCACAT CACCTCCAAC TGGCTTGGTT 50
TTCTCTGTGG CCTGATATTT GTAGTAGGGA TTGCGATAAT TCTGTATGAA 100
<210> 10041
<211> 100
<212> DNA
<213> Escherichia coli K-12 MG1655
<220> ymdd b1048
<222> 935-1034
<223> ymdd b1048 U00096 complement(1107007..1108164)
<313> 10041: 981-1000
<400> 10041
CGCTGTTTAT CTATCTGGTT CACCACCCGT TAACGCTGTT TTTCGGCGCA 50
TACATTACAC CGCACATCAC CTCCAACTGG CTTGGTTTTC TCTGTGGCCT 100
<210> 10042
<211> 100
<212> DNA
<213> Escherichia coli K-12 MG1655
<220> def b3288
<222> 411-510
<223> def b3288 U00096 3431327..3431836
<313> 10042: 466-485
<400> 10042
CCTGGTCGGC AAACTGTTTA TGGATTATCT GTCACCGCTG AAACAACAAC 50
GTATTCGTCA GAAAGTTGAA AAACTGGATC GTCTGAAAGC CCGGGCTTAA 100
<210> 10043
<211> 100
<212> DNA
<213> Escherichia coli K-12 MG1655
<220> def b3288
<222> 374-473
<223> def b3288 U00096 3431327..3431836
<313> 10043: 425-444
<400> 10043
GTCTGTTAGC CATCTGTATT CAGCATGAGA TGGATCACCT GGTCGGCAAA 50

CTGTTTATGG ATTATCTGTC ACCGCTGAAA CAACAACGTA TTCGTCAGAA 100
<210> 10044
<211> 100
<212> DNA
<213> Escherichia coli K-12 MG1655
<220> def b3288
<222> 304-403
<223> def b3288 U00096 3431327..3431836
<313> 10044: 339-358
<400> 10044
CCGCGCGCAG AGAAAGTTAA AATTCGCGCC CTTGACCGCG ACGGTAAACC 50
ATTTGAACTG GAAGCAGACG GTCTGTTAGC CATCTGTATT CAGCATGAGA 100
<210> 10045
<211> 100
<212> DNA
<213> Escherichia coli K-12 MG1655
<220> fhia b0230
<222> 1641-1740
<223> fhia b0230 U00096 complement(248358..250097)
<313> 10045: 1708-1727
<400> 10045
GGCGCGTTAT GCAAGGTTGT TTGCGCCGGG GCTGCATGTG CTGTCGTATA 50
ACGAAGTGCC GGATGAGCTG GAGTTGAAGA TTATGGGGGC GTTGATGTAA 100
<210> 10046
<211> 100
<212> DNA
<213> Escherichia coli K-12 MG1655
<220> fhia b0230
<222> 1529-1628
<223> fhia b0230 U00096 complement(248358..250097)
<313> 10046: 1572-1591
<400> 10046
ATCCGAATAT GCTCAACCAG TTCCAGAGTA CGATGCCACA GGTGAAAGAG 50
CAGATGAAAG CGGCGGGGAA AGACCCGGTG CTGCTGGTGC CACCGCAGCT 100
<210> 10047
<211> 100
<212> DNA
<213> Escherichia coli K-12 MG1655
<220> fhia b0230
<222> 1520-1619
<223> fhia b0230 U00096 complement(248358..250097)
<313> 10047: 1533-1552
<400> 10047
TGCCGGTCGA TCCGAATATG CTCAACCAGT TCCAGAGTAC GATGCCACAG 50
GTGAAAGAGC AGATGAAAGC GGCGGGGAAA GACCCGGTGC TGCTGGTGCC 100
<210> 10048
<211> 100
<212> DNA
<213> Escherichia coli K-12 MG1655
<220> dfp b3640
<222> 1152-1251
<223> dfp b3640 U00096 3810286..3811578
<313> 10048: 1188-1207
<400> 10048
AGGATTTAAC AGCGACAACA ACGCATTACA CCTTTTCTGG CAGGACGGAG 50
ATAAAGTCTT ACCGCTTGAG CGCAAAGAGC TCCTTGGCCA ATTATTACTC 100
<210> 10049
<211> 100
<212> DNA
<213> Escherichia coli K-12 MG1655
<220> dfp b3640
<222> 1096-1195
<223> dfp b3640 U00096 3810286..3811578
<313> 10049: 1148-1167
<400> 10049
CGTATCCGTA AAAACCTTGA TCTGATCTGC GCGAACGATG TTTCCCAGCC 50
AACTCAAGGA TTTAACAGCG ACAACAACGC ATTACACCTT TTCTGGCAGG 100
<210> 10050
<211> 100
<212> DNA
<213> Escherichia coli K-12 MG1655

<220> dfp b3640
<222> 1062-1161
<223> dfp b3640 U00096 3810286..3811578
<313> 10050: 1112-1131
<400> 10050
AACAAATAAT GTGGAAGAAT ACGCCCGGCA AAAACGTATC CGTAAAAACC 50
TTGATCTGAT CTGCGCGAAC GATGTTTCCC AGCCAACTCA AGGATTTAAC 100
<210> 10051
<211> 100
<212> DNA
<213> Escherichia coli K-12 MG1655
<220> hyaa b0973
<222> 1020-1119
<223> hyaa b0973 U00096 1031362..1032480
<313> 10051: 1089-1108
<400> 10051
TGTGCACGCA GTCGCCAGCG CCGTTGACCA GCGCAGACGT CATAACCAGC 50
AACCTACAGA AACCGAACAT CAGCCAGGCA ATGAGGATAA ACAGGCATGA 100
<210> 10052
<211> 100
<212> DNA
<213> Escherichia coli K-12 MG1655
<220> hyaa b0973
<222> 929-1028
<223> hyaa b0973 U00096 1031362..1032480
<313> 10052: 959-978
<400> 10052
GCCGCGTGGT CGATATTCCG CAAATGGGTA CTCATTCCAC CGCCGATACC 50
GTCGGTTTAA CCGCGCTTGG CGTGGTGGCA GCGGCTGTTG GTGTGCACGC 100
<210> 10053
<211> 100
<212> DNA
<213> Escherichia coli K-12 MG1655
<220> hyaa b0973
<222> 821-920
<223> hyaa b0973 U00096 1031362..1032480
<313> 10053: 847-866
<400> 10053
CCTGTTCCTC CACACGCTGG AATGATGGCG TTTCTTTCCC AATCCAGTCT 50
GGTCACGGCT GCCTGGGCTG TGCGGAAAAT GGTTTCTGGG ATCGCGGTTC 100
<210> 10054
<211> 100
<212> DNA
<213> Escherichia coli K-12 MG1655
<220> hyab b0974
<222> 1695-1794
<223> hyab b0974 U00096 1032477..1034270
<313> 10054: 1765-1784
<400> 10054
GATCCTGCGT ACTCTGCACA GCTTTGACCC GTGCCTCGCC TGTTCAACAC 50
ACGTGCTGGG CGACGACGGT AGCGAGCTGA TCTCCGTGCA GGTGCGTTAA 100
<210> 10055
<211> 100
<212> DNA
<213> Escherichia coli K-12 MG1655
<220> hyab b0974
<222> 1663-1762
<223> hyab b0974 U00096 1032477..1034270
<313> 10055: 1726-1745
<400> 10055
ACCAAAATGG CGATCCCCGA GCAACCGCTG GAGATCCTGC GTACTCTGCA 50
CAGCTTTGAC CCGTGCCTCG CCTGTTCAAC ACACGTGCTG GGCGACGACG 100
<210> 10056
<211> 100
<212> DNA
<213> Escherichia coli K-12 MG1655
<220> hyab b0974
<222> 1649-1748
<223> hyab b0974 U00096 1032477..1034270
<313> 10056: 1678-1697
<400> 10056

CGGCGCTGAT GAACACCAAA ATGGCGATCC CCGAGCAACC GCTGGAGATC 50
CTGCGTACTC TGCACAGCTT TGACCCGTGC CTCGCCTGTT CAACACACGT 100
<210> 10057
<211> 100
<212> DNA
<213> Escherichia coli K-12 MG1655
<220> hyac b0975
<222> 577-676
<223> hyac b0975 U00096 1034289..1034996
<313> 10057: 618-637
<400> 10057
GCGTTTGTGA TCGGTCATGT CTACATGGCG CTGCGTGAAG ACATCATGTC 50
CGACGACACG GTGATCTCCA CCATGGTCAA CGGCTACCGT AGCCACAAAT 100
<210> 10058
<211> 100
<212> DNA
<213> Escherichia coli K-12 MG1655
<220> hyac b0975
<222> 462-561
<223> hyac b0975 U00096 1034289..1034996
<313> 10058: 522-541
<400> 10058
CAGCCAGTAC GCTATTTTTG CGCCGTTCCG TTATGTGGTG GAATTTTTCT 50
ACTGGACGGG TGGCAACTCA ATGGACATTC ACAGCTGGCA TCGGCTGGGG 100
<210> 10059
<211> 100
<212> DNA
<213> Escherichia coli K-12 MG1655
<220> hyac b0975
<222> 409-508
<223> hyac b0975 U00096 1034289..1034996
<313> 10059: 468-487
<400> 10059
TTCCTGATGT CGGTCTTTAT GATCATCACT GGTTTTGCGC TGTACAGCGA 50
ACACAGCCAG TACGCTATTT TTGCGCCGTT CCGTTATGTG GTGGAATTTT 100
<210> 10060
<211> 100
<212> DNA
<213> Escherichia coli K-12 MG1655
<220> hyad b0976
<222> 489-588
<223> hyad b0976 U00096 1034993..1035580
<313> 10060: 558-577
<400> 10060
TAATGAATCG CGCTGTCTCA ATTATGACTG TCTGTCGATG GAAAATTACG 50
AAGGCGTTCG CTTGCGCCAG TACCGGATGA CACAGGAGGA GCAGGGATGA 100
<210> 10061
<211> 100
<212> DNA
<213> Escherichia coli K-12 MG1655
<220> hyad b0976
<222> 457-556
<223> hyad b0976 U00096 1034993..1035580
<313> 10061: 502-521
<400> 10061
CTTGCTGCGT GGGGAATTGT GCCGCAACCG GCTAATGAAT CGCGCTGTCT 50
CAATTATGAC TGTCTGTCGA TGGAAAATTA CGAAGGCGTT CGCTTGCGCC 100
<210> 10062
<211> 100
<212> DNA
<213> Escherichia coli K-12 MG1655
<220> hyad b0976
<222> 362-461
<223> hyad b0976 U00096 1034993..1035580
<313> 10062: 416-435
<400> 10062
GTCTGCAACC CGCAATGCTC GACGACTACG GCGGTAGCCT GAGCGAACTG 50
GCACGGGAGC AACTGCCCGC TGCGGAACAG GCGGCGCTGG CGCAGCTTGC 100
<210> 10063
<211> 100
<212> DNA

<213> Escherichia coli K-12 MG1655
<220> b1121 b1122
<222> 690-789
<223> b1121 b1122 U00096 complement(1179702..1180490)
<313> 10063: 743-762
<400> 10063
CGATAATAGC TTTGCGATTA CCAGAATATC GCCACTCTGG GCAGGGCTAA 50
TTGGTGTCTG TACGTTTATT GCCGCATTGT TATTGACGAG TAAACGATAA 100
<210> 10064
<211> 100
<212> DNA
<213> Escherichia coli K-12 MG1655
<220> b1121 b1122
<222> 681-780
<223> b1121 b1122 U00096 complement(1179702..1180490)
<313> 10064: 717-736
<400> 10064
TTTGTATATC GATAATAGCT TTGCGATTAC CAGAATATCG CCACTCTGGG 50
CAGGGCTAAT TGGTGTCTGT ACGTTTATTG CCGCATTGTT ATTGACGAGT 100
<210> 10065
<211> 100
<212> DNA
<213> Escherichia coli K-12 MG1655
<220> b1121 b1122
<222> 508-607
<223> b1121 b1122 U00096 complement(1179702..1180490)
<313> 10065: 544-563
<400> 10065
GGACAACAGC ATGAAAATGC TCCCCCCGGC AGCACCAACA CATTAGGTTT 50
TATCGCCTGG GCTGCGACGT TTATCTTATT TTCCAGGATC TTTTACTACA 100
<210> 10066
<211> 100
<212> DNA
<213> Escherichia coli K-12 MG1655
<220> hyae b0977
<222> 239-338
<223> hyae b0977 U00096 1035577..1035975
<313> 10066: 287-306
<400> 10066
GCGAAGCCAT CGGCGATCGC TTTGGCGTCT TTCGCTTTCC TGCCACTTTA 50
GTGTTTACCG GCGGAAACTA TCGCGGCGTG CTGAATGGTA TTCACCCGTG 100
<210> 10067
<211> 100
<212> DNA
<213> Escherichia coli K-12 MG1655
<220> hyae b0977
<222> 170-269
<223> hyae b0977 U00096 1035577..1035975
<313> 10067: 197-216
<400> 10067
TGATTGGCGA ATTACTGCGC GAGTTTCCCG ACTATACATG GCAGGTGGCG 50
ATTGCTGACC TTGAGCAGAG CGAAGCCATC GGCGATCGCT TTGGCGTCTT 100
<210> 10068
<211> 100
<212> DNA
<213> Escherichia coli K-12 MG1655
<220> hyae b0977
<222> 111-210
<223> hyae b0977 U00096 1035577..1035975
<313> 10068: 144-163
<400> 10068
CGTGGTGTTA TTAAGCAGTG ACCCGAAACG CACGCCAGAG GTCAGCGATA 50
ATCCGGTAAT GATTGGCGAA TTACTGCGCG AGTTTCCCGA CTATACATGG 100
<210> 10069
<211> 100
<212> DNA
<213> Escherichia coli K-12 MG1655
<220> hyaf b0978
<222> 759-858
<223> hyaf b0978 U00096 1035972..1036829
<313> 10069: 836-855

<400> 10069
AATACCGGAA GTGGTGCTGG CAGCGCCAGA AGATTTGGTC GACTCTGCGC 50
AGCGGCTTAG CGAGGTATGT CAGTGGCTGG CGGAAGCTGC ACCGACGTAA 100
<210> 10070
<211> 100
<212> DNA
<213> Escherichia coli K-12 MG1655
<220> hyaf b0978
<222> 705-804
<223> hyaf b0978 U00096 1035972..1036829
<313> 10070: 751-770
<400> 10070
ACGCTGTACG GACACCTTAA AAGGCCCGTT ACTGGAAAGT TATGAAATCT 50
GCCCAATACC GGAAGTGGTG CTGGCAGCGC CAGAAGATTT GGTCGACTCT 100
<210> 10071
<211> 100
<212> DNA
<213> Escherichia coli K-12 MG1655
<220> hyaf b0978
<222> 636-735
<223> hyaf b0978 U00096 1035972..1036829
<313> 10071: 684-703
<400> 10071
TCAGATTCGT ACCATTGGCT ATGGCGAGAG CTATATCAAC GCCACGGGGT 50
TACGCCATGT CTGGCATTTA CGCTGTACGG ACACCTTAAA AGGCCCGTTA 100
<210> 10072
<211> 100
<212> DNA
<213> Escherichia coli K-12 MG1655
<220> inta b2623
<222> 1143-1242
<223> inta b2623 U00096 2754180..2755421
<313> 10072: 1177-1196
<400> 10072
ACGTCGTCCG ATGATGCAAT GGTGGGCTGA TCTCGTAAAA GCAGCAGATA 50
GTGGTAGCAT CGTTTTAACT CATTTGAGCA AAATTCGTCT TGTCGGATAA 100
<210> 10073
<211> 100
<212> DNA
<213> Escherichia coli K-12 MG1655
<220> inta b2623
<222> 1081-1180
<223> inta b2623 U00096 2754180..2755421
<313> 10073: 1115-1134
<400> 10073
CTTGCTCATG TAGACAAAAA TGAGGTGCGT CGCGCTTATA ACCGCAGTGA 50
TTATCTTGAG CAACGTCGTC CGATGATGCA ATGGTGGGCT GATCTCGTAA 100
<210> 10074
<211> 100
<212> DNA
<213> Escherichia coli K-12 MG1655
<220> inta b2623
<222> 979-1078
<223> inta b2623 U00096 2754180..2755421
<313> 10074: 1036-1055
<400> 10074
CGTGCTGGCT TAGGAGGTGT ACTTGTTTCA CACGGCTTGC GTTCTATCGC 50
CAGTACGGCA CTCAATGAGG AAGGATTTCC ACCTGATGTC ATTGAAGCAG 100
<210> 10075
<211> 100
<212> DNA
<213> Escherichia coli K-12 MG1655
<220> ymfa b1123
<222> 372-471
<223> ymfa b1123 U00096 complement(1180487..1180957)
<313> 10075: 449-468
<400> 10075
GTTTATCGGC ATTATGGGCG TTATTTTTAT CGCCGTCGGT TTATTAATAA 50
GTATGCCCTC CTCTAAAAAA TCACGCAGGA AACGGAAGTC CCGCCCATGA 100
<210> 10076
<211> 100

<212> DNA
<213> Escherichia coli K-12 MG1655
<220> ymfa b1123
<222> 347-446
<223> ymfa b1123 U00096 complement(1180487..1180957)
<313> 10076: 396-415
<400> 10076
CGTGGGTTAA TTTTTTTAAA TGGGGGTTTA TCGGCATTAT GGGCGTTATT 50
TTTATCGCCG TCGGTTTATT AATAAGTATG CCCTCCTCTA AAAAATCACG 100
<210> 10077
<211> 100
<212> DNA
<213> Escherichia coli K-12 MG1655
<220> ymfa b1123
<222> 180-279
<223> ymfa b1123 U00096 complement(1180487..1180957)
<313> 10077: 216-235
<400> 10077
GGGTAAAGAT GGTTCGTGGT ATCCCGTCGT TGCCTTTCGA CCGACGCCAG 50
ATTACACGCT AATTTTCAAT TCGAGTATCG GCAGCGATTT TTATGAAGAC 100
<210> 10078
<211> 100
<212> DNA
<213> Escherichia coli K-12 MG1655
<220> intb b4272
<222> 1073-1172
<223> intb b4272 U00096 4494318..4495508
<313> 10078: 1114-1133
<400> 10078
AGCATCTGGA AGAACGGCGA TTGATGCTAC AGTGGTGGGC CGATTTTCTG 50
GATGTAAACA GAGAAAGGTT TATCAGTCCA TTTGAATATG CAAAGATTAA 100
<210> 10079
<211> 100
<212> DNA
<213> Escherichia coli K-12 MG1655
<220> intb b4272
<222> 1039-1138
<223> intb b4272 U00096 4494318..4495508
<313> 10079: 1068-1087
<400> 10079
AATTCAGTGA GGGCCGCGTA TATCCATAAA GCAGAGCATC TGGAAGAACG 50
GCGATTGATG CTACAGTGGT GGGCCGATTT TCTGGATGTA AACAGAGAAA 100
<210> 10080
<211> 100
<212> DNA
<213> Escherichia coli K-12 MG1655
<220> intb b4272
<222> 973-1072
<223> intb b4272 U00096 4494318..4495508
<313> 10080: 1015-1034
<400> 10080
TCATTGGTCG AATCAGGTCT GTGGTCTCGT GATGCTGTTG AACGTCAGAT 50
GAGCCACATG GCGCGAAATT CAGTGAGGGC CGCGTATATC CATAAAGCAG 100
<210> 10081
<211> 100
<212> DNA
<213> Escherichia coli K-12 MG1655
<220> intc intC
<222> 1059-1158
<223> intc intC U00096 2464565..2465722
<313> 10081: 1126-1145
<400> 10081
CGGATCTGTG CGTGGGATTT ACAACCATGC TCAGTATCTC GATAAACGCA 50
GAGAAATGAT GCAATGGTGG GCGGACTGGC TTGATGAGAA GGTGGAGTGA 100
<210> 10082
<211> 100
<212> DNA
<213> Escherichia coli K-12 MG1655
<220> intc intC
<222> 1021-1120
<223> intc intC U00096 2464565..2465722

<313> 10082: 1080-1099
<400> 10082
GCTGACGCTA TTGAAGTGCA ACTGGCACAT GCAAACGGCG GATCTGTGCG 50
TGGGATTTAC AACCATGCTC AGTATCTCGA TAAACGCAGA GAAATGATGC 100
<210> 10083
<211> 100
<212> DNA
<213> Escherichia coli K-12 MG1655
<220> intc intC
<222> 975-1074
<223> intc intC U00096 2464565..2465722
<313> 10083: 1038-1057
<400> 10083
ATTCAGGCAT GAATTCAGCA CGATTATGAA CGAGCACGAA TGGCCTGCTG 50
ACGCTATTGA AGTGCAACTG GCACATGCAA ACGGCGGATC TGTGCGTGGG 100
<210> 10084
<211> 100
<212> DNA
<213> Escherichia coli K-12 MG1655
<220> ymfc b1136
<222> 525-624
<223> ymfc b1136 U00096 complement(1193521..1194144)
<313> 10084: 552-571
<400> 10084
CCATGTTGGC TTCCCCACGC TGCGACTGAT TCGCTATGCG ATGGGTGATT 50
ACTCTTTGGA TAATCTTGCC AATGGTGAAT GGCGAGAAGT GACAGATTAA 100
<210> 10085
<211> 100
<212> DNA
<213> Escherichia coli K-12 MG1655
<220> ymfc b1136
<222> 428-527
<223> ymfc b1136 U00096 complement(1193521..1194144)
<313> 10085: 480-499
<400> 10085
GGAATCCACC AATTCGTGAA CGCAAAAGTA TTCCCACCAG CTGGCTGAAG 50
ATCACCTTAT ATGAAGGACG TAATCGCCAG GTGCGCCGCA TGACCGCCCA 100
<210> 10086
<211> 100
<212> DNA
<213> Escherichia coli K-12 MG1655
<220> ymfc b1136
<222> 305-404
<223> ymfc b1136 U00096 complement(1193521..1194144)
<313> 10086: 354-373
<400> 10086
AGGTGGAAGG TATTCCCACA CAAGACGCAC TTGAAGCCTT GCGCAATGGC 50
GTAACCTTAA ATGATGGCCC TACCCTGCCC GCCGGCGCGG AGCTGGTTGA 100
<210> 10087
<211> 100
<212> DNA
<213> Escherichia coli K-12 MG1655
<220> intd intD
<222> 1008-1107
<223> intd intD U00096 complement(564038..565201)
<313> 10087: 1051-1070
<400> 10087
AATGGGCGGA TGGGAGTCCA TAGAAATGGT TCGTAGGTAT GCTCACCTTG 50
CGCCTAATCA TTTGACAGAG CATGCGAGGA AAATAGACGA CATTTTTGGT 100
<210> 10088
<211> 100
<212> DNA
<213> Escherichia coli K-12 MG1655
<220> intd intD
<222> 972-1071
<223> intd intD U00096 complement(564038..565201)
<313> 10088: 1016-1035
<400> 10088
GATTCAGTCA GGCGTCCCAT TATCAGTGCT TCAGGAAATG GGCGGATGGG 50
AGTCCATAGA AATGGTTCGT AGGTATGCTC ACCTTGCGCC TAATCATTTG 100
<210> 10089

<211> 100
<212> DNA
<213> Escherichia coli K-12 MG1655
<220> intd intD
<222> 914-1013
<223> intd intD U00096 complement(564038..565201)
<313> 10089: 949-968
<400> 10089
GTGCAGGAAT TGAAGATTTC CGTTTCCATG ACCTCAGACA CACCTGGGCA 50
AGCTGGCTGA TTCAGTCAGG CGTCCCATTA TCAGTGCTTC AGGAAATGGG 100
<210> 10090
<211> 100
<212> DNA
<213> Escherichia coli K-12 MG1655
<220> ymfd b1138
<222> 567-666
<223> ymfd b1138 U00096 complement(1196090..1196755)
<313> 10090: 626-645
<400> 10090
AGTGCAAGAA ATATGTTCTT CACATGGCCT TGAAATATTA AAGTCGTGGA 50
GTAAAGCAGG AAGTTCATAT GTCACTGTTG GGAGTTGTAA TGCAATATAA 100
<210> 10091
<211> 100
<212> DNA
<213> Escherichia coli K-12 MG1655
<220> ymfd b1138
<222> 550-649
<223> ymfd b1138 U00096 complement(1196090..1196755)
<313> 10091: 572-591
<400> 10091
TTACTCGATG AACTCGCAGT GCAAGAAATA TGTTCTTCAC ATGGCCTTGA 50
AATATTAAAG TCGTGGAGTA AAGCAGGAAG TTCATATGTC ACTGTTGGGA 100
<210> 10092
<211> 100
<212> DNA
<213> Escherichia coli K-12 MG1655
<220> ymfd b1138
<222> 320-419
<223> ymfd b1138 U00096 complement(1196090..1196755)
<313> 10092: 366-385
<400> 10092
AAATAATTGG GGGTTACGAT TTCATCCTTT GCTCTAATGT TCTCTCTGCC 50
GTTCCTTGTC GGGATACAAT CGACAAAATA GTTCTTAGCA TCAAGAGATT 100
<210> 10093
<211> 100
<212> DNA
<213> Escherichia coli K-12 MG1655
<220> inte intE
<222> 952-1051
<223> inte intE U00096 complement(1198902..1200029)
<313> 10093: 980-999
<400> 10093
AGAATTGACT GGGGAGAGGG TTCACCTGCG ACATTCCATG AGCAAAGGTC 50
TTTATCTGAA CGTCTGTATA AAGAACAGGG GTTAGACACA CAAAAACTAC 100
<210> 10094
<211> 100
<212> DNA
<213> Escherichia coli K-12 MG1655
<220> inte intE
<222> 877-976
<223> inte intE U00096 complement(1198902..1200029)
<313> 10094: 903-922
<400> 10094
TCACAAGCAG AGCGCGGAGC GCAGGTTAAA GCCAATACAT TGACGATGAA 50
TTTTAGTAAA GCGAGAGATT TAGCAAGAAT TGACTGGGGA GAGGGTTCAC 100
<210> 10095
<211> 100
<212> DNA
<213> Escherichia coli K-12 MG1655
<220> inte intE
<222> 780-879

<223> inte intE U00096 complement(1198902..1200029)
<313> 10095: 830-849
<400> 10095
TCGCCTCAAT GCGATTAACT GGAGTTTACG CGATGTAGTA GCCCGCTGCC  50
GTGACTATGC AGTCAGTGCA TACTTAGTGC ATTTTTTTCG TTCAACTTCA  100
<210> 10096
<211> 100
<212> DNA
<213> Escherichia coli K-12 MG1655
<220> ymfe b1139
<222> 606-705
<223> ymfe b1139 U00096 complement(1196756..1197460)
<313> 10096: 648-667
<400> 10096
TATCTTTGCT TTGACAGTTA AGCCCAGAAC TGAAAGTCAA GTCGGAAAAA  50
TCCCGGACGA TAAAATAAAA GAATTTTTCA CTAAAAATAA CATTAATTGA  100
<210> 10097
<211> 100
<212> DNA
<213> Escherichia coli K-12 MG1655
<220> ymfe b1139
<222> 594-693
<223> ymfe b1139 U00096 complement(1196756..1197460)
<313> 10097: 649-668
<400> 10097
AGTGAGTGGT ATTATCTTTG CTTTGACAGT TAAGCCCAGA ACTGAAAGTC  50
AAGTCGGAAA AATCCCGGAC GATAAAATAA AAGAATTTTT CACTAAAAAT  100
<210> 10098
<211> 100
<212> DNA
<213> Escherichia coli K-12 MG1655
<220> ymfe b1139
<222> 606-705
<223> ymfe b1139 U00096 complement(1196756..1197460)
<313> 10098: 635-654
<400> 10098
TATCTTTGCT TTGACAGTTA AGCCCAGAAC TGAAAGTCAA GTCGGAAAAA  50
TCCCGGACGA TAAAATAAAA GAATTTTTCA CTAAAAATAA CATTAATTGA  100
<210> 10099
<211> 100
<212> DNA
<213> Escherichia coli K-12 MG1655
<220> intf b0282
<222> 1246-1345
<223> intf b0282 U00096 complement(294920..296320)
<313> 10099: 1287-1306
<400> 10099
GTTGCTCAAA TTATGGGACA CAAACCCAGC GCTCTTGCCG AAAAACACTA  50
TCGCCGTCGT CCGTTAGATC TGTTACGAAA ATGGCACGAG AAAATTGAGA  100
<210> 10100
<211> 100
<212> DNA
<213> Escherichia coli K-12 MG1655
<220> intf b0282
<222> 1134-1233
<223> intf b0282 U00096 complement(294920..296320)
<313> 10100: 1197-1216
<400> 10100
TTCAGCGCAC AACAGAGCAT TAGTGCTGGC TGAGTTACCA CATATCAGCC  50
TTCACGGTTT ACGTCGTAGT TTTGGTACTT TGGCCGAGTG GGTTGAAGTT  100
<210> 10101
<211> 100
<212> DNA
<213> Escherichia coli K-12 MG1655
<220> intf b0282
<222> 1095-1194
<223> intf b0282 U00096 complement(294920..296320)
<313> 10101: 1127-1146
<400> 10101
CAGAAGTAAC AGTAAAAGTG GCAAAATTAT TGAGCCGCGT TCAGCGCACA  50
ACAGAGCATT AGTGCTGGCT GAGTTACCAC ATATCAGCCT TCACGGTTTA  100

<210> 10102
<211> 100
<212> DNA
<213> Escherichia coli K-12 MG1655
<220> ymfh b1143
<222> 213-312
<223> ymfh b1143 U00096 complement(1200292..1200603)
<313> 10102: 285-304
<400> 10102
TGACGGGTTA ATAACCCGAC CCATCCTGAT AGCTGTGGCC ACAGGTGCTC 50
TTCTCTGTTT TGTGGAGAAA CTAACCGACC GTGCAGGGTC GATATGCTGA 100
<210> 10103
<211> 100
<212> DNA
<213> Escherichia coli K-12 MG1655
<220> ymfh b1143
<222> 156-255
<223> ymfh b1143 U00096 complement(1200292..1200603)
<313> 10103: 186-205
<400> 10103
TTCCTTAGTC CTTTCTGGTA CCGCCCTTTT TACAAAACAG AGAAGAGTAT 50
CCCCGGATGA CGGGTTAATA ACCCGACCCA TCCTGATAGC TGTGGCCACA 100
<210> 10104
<211> 100
<212> DNA
<213> Escherichia coli K-12 MG1655
<220> ymfh b1143
<222> 77-176
<223> ymfh b1143 U00096 complement(1200292..1200603)
<313> 10104: 100-119
<400> 10104
GTGTGCAACC TTCGCCAGGC ACCGGGGGCA CCCGACACCA CAACTTTATG 50
CTGTGTGTAG TCTTGGCGGT ACCAGTTTTT TCCTTAGTCC TTTCTGGTAC 100
<210> 10105
<211> 100
<212> DNA
<213> Escherichia coli K-12 MG1655
<220> ymfi b1144
<222> 288-387
<223> ymfi b1144 U00096 1200675..1201061
<313> 10105: 346-365
<400> 10105
ATTTGAACAA AGCGCATGCT TACTTCCGTC TGCTTCCTTA GTTGGTAACG 50
ATTTCGTTCA TTACGATCTT ACGCCTAAGC CCATCAAGCT CGATTCTTAA 100
<210> 10106
<211> 100
<212> DNA
<213> Escherichia coli K-12 MG1655
<220> ymfi b1144
<222> 256-355
<223> ymfi b1144 U00096 1200675..1201061
<313> 10106: 322-341
<400> 10106
GCCGGTGTTG ACGCAAATAT ATTTGTCTGT AAATTTGAAC AAAGCGCATG 50
CTTACTTCCG TCTGCTTCCT TAGTTGGTAA CGATTTCGTT CATTACGATC 100
<210> 10107
<211> 100
<212> DNA
<213> Escherichia coli K-12 MG1655
<220> ymfi b1144
<222> 52-151
<223> ymfi b1144 U00096 1200675..1201061
<313> 10107: 83-102
<400> 10107
CAGTTTTATG TTCACGTTCG TCTATTTGAA GACACAGCCG AACAGACCAA 50
AAAATTTGAA GAATTAATGC TTAACTTTCT GTACCAGAAA ACAGTTAAAG 100
<210> 10108
<211> 100
<212> DNA
<213> Escherichia coli K-12 MG1655
<220> ymfj ymfJ

<222> 153-252
<223> ymfj ymfJ U00096 complement(1200999..1201283)
<313> 10108: 191-210
<400> 10108
CACACTCACC CCTCTGCCAT GCAATGGAGA GAAGGTGAGC CGTGTTTATC 50
ACGACACAAT GGTTAAGGCT TTAAGAATCG AGCTTGATGG GCTTAGGCGT 100
<210> 10109
<211> 100
<212> DNA
<213> Escherichia coli K-12 MG1655
<220> ymfj ymfJ
<222> 126-225
<223> ymfj ymfJ U00096 complement(1200999..1201283)
<313> 10109: 176-195
<400> 10109
AACCGAGTTC ATGAATTTCA TGGCTAACAC ACTCACCCCT CTGCCATGCA 50
ATGGAGAGAA GGTGAGCCGT GTTTATCACG ACACAATGGT TAAGGCTTTA 100
<210> 10110
<211> 100
<212> DNA
<213> Escherichia coli K-12 MG1655
<220> ymfj ymfJ
<222> 121-220
<223> ymfj ymfJ U00096 complement(1200999..1201283)
<313> 10110: 162-181
<400> 10110
GGTCGAACCG AGTTCATGAA TTTCATGGCT AACACACTCA CCCCTCTGCC 50
ATGCAATGGA GAGAAGGTGA GCCGTGTTTA TCACGACACA ATGGTTAAGG 100
<210> 10111
<211> 100
<212> DNA
<213> Escherichia coli K-12 MG1655
<220> ymfl b1148
<222> 470-569
<223> ymfl b1148 U00096 1202479..1203048
<313> 10111: 496-515
<400> 10111
ACAAAATTTT TTGCATTTCA GAAAGTAATG ACGCCCGCGA GTGTGCAGCT 50
CCGGGCGTCG TGGCGTCGAT TGCTTCTGGT TGTGGAGAAA CTAACGCATG 100
<210> 10112
<211> 100
<212> DNA
<213> Escherichia coli K-12 MG1655
<220> ymfl b1148
<222> 357-456
<223> ymfl b1148 U00096 1202479..1203048
<313> 10112: 403-422
<400> 10112
ACAGATTCGT TCAGCAATCG AAGACGGTGT AGTGGAACCG CATGAGAAGA 50
CAGCAATTAA CGACGAGCTG TATCTCTCAA TTTCGAAGCT GCAGGAGCAT 100
<210> 10113
<211> 100
<212> DNA
<213> Escherichia coli K-12 MG1655
<220> ymfl b1148
<222> 314-413
<223> ymfl b1148 U00096 1202479..1203048
<313> 10113: 370-389
<400> 10113
AGCGTCTGCT GGAAGTCATT GAACAGATCG GCAGTTATTC AAAACAGATT 50
CGTTCAGCAA TCGAAGACGG TGTAGTGGAA CCGCATGAGA AGACAGCAAT 100
<210> 10114
<211> 100
<212> DNA
<213> Escherichia coli K-12 MG1655
<220> ymfm b1149
<222> 240-339
<223> ymfm b1149 U00096 1203045..1203383
<313> 10114: 305-324
<400> 10114
TGGGAGCCGG AAACACAACG GGTTATCTAC CTCCGCGAAG GTTATGAGCA 50

TGAATGCTTC AGCCCGCTCG AACAGTTTCG TCGTAAATTC AGGGAAATAG 100
<210> 10115
<211> 100
<212> DNA
<213> Escherichia coli K-12 MG1655
<220> ymfm b1149
<222> 227-326
<223> ymfm b1149 U00096 1203045..1203383
<313> 10115: 265-284
<400> 10115
CAGAGTCATT CGTTGGGAGC CGGAAACACA ACGGGTTATC TACCTCCGCG 50
AAGGTTATGA GCATGAATGC TTCAGCCCGC TCGAACAGTT TCGTCGTAAA 100
<210> 10116
<211> 100
<212> DNA
<213> Escherichia coli K-12 MG1655
<220> ymfm b1149
<222> 197-296
<223> ymfm b1149 U00096 1203045..1203383
<313> 10116: 230-249
<400> 10116
CTGGTTCAAA GATCACTACG GAGTGCCCGT CAGAGTCATT CGTTGGGAGC 50
CGGAAACACA ACGGGTTATC TACCTCCGCG AAGGTTATGA GCATGAATGC 100
<210> 10117
<211> 100
<212> DNA
<213> Escherichia coli K-12 MG1655
<220> ymfn b1150
<222> 1269-1368
<223> ymfn b1150 U00096 1203393..1204760
<313> 10117: 1323-1342
<400> 10117
TGCAGTTGCG CTGATTATGG CGGTTGGCAG AGCCATGCTG TACGAGAAAG 50
AAGACACGCT GTCTGATCAC ATTGAGTCCT ACGGGATCCG CTCGCTTTAA 100
<210> 10118
<211> 100
<212> DNA
<213> Escherichia coli K-12 MG1655
<220> ymfn b1150
<222> 1237-1336
<223> ymfn b1150 U00096 1203393..1204760
<313> 10118: 1290-1309
<400> 10118
GTCAAGGAGC AGGCGGAAAA CAAAATCGAT GGTGCAGTTG CGCTGATTAT 50
GGCGGTTGGC AGAGCCATGC TGTACGAGAA AGAAGACACG CTGTCTGATC 100
<210> 10119
<211> 100
<212> DNA
<213> Escherichia coli K-12 MG1655
<220> ymfn b1150
<222> 1192-1291
<223> ymfn b1150 U00096 1203393..1204760
<313> 10119: 1237-1256
<400> 10119
GTGGTCGGCA AAACCATTCC GGGTAACGAT GATGTGGTGA AGCCCGTCAA 50
GGAGCAGGCG GAAAACAAAA TCGATGGTGC AGTTGCGCTG ATTATGGCGG 100
<210> 10120
<211> 100
<212> DNA
<213> Escherichia coli K-12 MG1655
<220> ymfo ymfO
<222> 327-426
<223> ymfo ymfO U00096 1204954..1205427
<313> 10120: 381-400
<400> 10120
GAGGGGAAAC TTTTACGCCT ACAAAGTGAA AGCATTTGGC GAAGTGGCTG 50
AACTGCTGCC CGTCGATCCC GGCTGTGTGG TATATGCGCT GGGAAGGTGT 100
<210> 10121
<211> 100
<212> DNA
<213> Escherichia coli K-12 MG1655

<220> ymfo ymfO
<222> 268-367
<223> ymfo ymfO U00096 1204954..1205427
<313> 10121: 327-346
<400> 10121
CCCAATGGCT ATATGACGCC GCAGGAGTTC TGGGAGCTGG TGGTCACCTG 50
TCTGTGCCTG AGGGGAAACT TTTACGCCTA CAAAGTGAAA GCATTTGGCG 100
<210> 10122
<211> 100
<212> DNA
<213> Escherichia coli K-12 MG1655
<220> ymfo ymfO
<222> 221-320
<223> ymfo ymfO U00096 1204954..1205427
<313> 10122: 242-261
<400> 10122
AGCAGAGAGC CACCGGCGAA CGTCTGCATA AACTGATCTC CACGCATCCC 50
AATGGCTATA TGACGCCGCA GGAGTTCTGG GAGCTGGTGG TCACCTGTCT 100
<210> 10123
<211> 100
<212> DNA
<213> Escherichia coli K-12 MG1655
<220> ymfr b1151
<222> 84-183
<223> ymfr b1151 U00096 1204772..1204954
<313> 10123: 146-165
<400> 10123
GGCGGGTTTT GTTGTTGCCG GGGCGCTGTG CCTGTTCTGG TCGTGGCTGG 50
TGGCGCGATA TCTCGACCGT ACACAGTCGT CTGTCGGCGG AGGTAAATAG 100
<210> 10124
<211> 100
<212> DNA
<213> Escherichia coli K-12 MG1655
<220> ymfr b1151
<222> 47-146
<223> ymfr b1151 U00096 1204772..1204954
<313> 10124: 64-83
<400> 10124
TTTTGCTGGC GTATGGTGCC TGGCTGATTT ATCCCCCGGC GGGTTTTGTT 50
GTTGCCGGGG CGCTGTGCCT GTTCTGGTCG TGGCTGGTGG CGCGATATCT 100
<210> 10125
<211> 100
<212> DNA
<213> Escherichia coli K-12 MG1655
<220> ymfr b1151
<222> 17-116
<223> ymfr b1151 U00096 1204772..1204954
<313> 10125: 69-88
<400> 10125
TCGCGCCTCT GGTGGGCGTG CTGGGTGCGC TTTTGCTGGC GTATGGTGCC 50
TGGCTGATTT ATCCCCCGGC GGGTTTTGTT GTTGCCGGGG CGCTGTGCCT 100
<210> 10126
<211> 100
<212> DNA
<213> Escherichia coli K-12 MG1655
<220> dgt b0161
<222> 1392-1491
<223> dgt b0161 U00096 179237..180754
<313> 10126: 1420-1439
<400> 10126
AGATTCTCCT GAGTTTCCGC TATGGGAATA TTATTACCGT TGCCGCCTGC 50
TGCAGGATTA TATCAGCGGT ATGACCGACC TCTATGCGTG GGATGAATAC 100
<210> 10127
<211> 100
<212> DNA
<213> Escherichia coli K-12 MG1655
<220> dgt b0161
<222> 1337-1436
<223> dgt b0161 U00096 179237..180754
<313> 10127: 1376-1395
<400> 10127

AACTCTCGAC GCGCCATCGG CTGGCCTATG TCGAGGCTGT CAGTAAATTA 50
CCGTCAGATT CTCCTGAGTT TCCGCTATGG GAATATTATT ACCGTTGCCG 100
<210> 10128
<211> 100
<212> DNA
<213> Escherichia coli K-12 MG1655
<220> dgt b0161
<222> 1301-1400
<223> dgt b0161 U00096 179237..180754
<313> 10128: 1320-1339
<400> 10128
TGAAACGTTT CCCTATTGAA TCGCGCTTAT TCCACAAACT CTCGACGCGC 50
CATCGGCTGG CCTATGTCGA GGCTGTCAGT AAATTACCGT CAGATTCTCC 100
<210> 10129
<211> 100
<212> DNA
<213> Escherichia coli K-12 MG1655
<220> ubia b4041
<222> 719-818
<223> ubia b4041 U00096 4250595..4251467
<313> 10129: 776-795
<400> 10129
ACTGGTCAAT TCTGGTGGCT GGCGCGCTGT TTGTTTATCA ACAAAAACTG 50
ATTGCCAACC GCGAGCGTGA AGCCTGCTTT AAAGCATTTA TGAATAATAA 100
<210> 10130
<211> 100
<212> DNA
<213> Escherichia coli K-12 MG1655
<220> ubia b4041
<222> 685-784
<223> ubia b4041 U00096 4250595..4251467
<313> 10130: 716-735
<400> 10130
ATCGGTGAGT TAAATGGCTT AGGCTGGGGA TATTACTGGT CAATTCTGGT 50
GGCTGGCGCG CTGTTTGTTT ATCAACAAAA ACTGATTGCC AACCGCGAGC 100
<210> 10131
<211> 100
<212> DNA
<213> Escherichia coli K-12 MG1655
<220> ubia b4041
<222> 565-664
<223> ubia b4041 U00096 4250595..4251467
<313> 10131: 611-630
<400> 10131
ATGGTTGACC GCGATGATGA TGTGAAGATT GGCATTAAAT CCACGGCAAT 50
CCTGTTCGGC CAATACGATA AATTGATTAT TGGTATTTTG CAGATTGGCG 100
<210> 10132
<211> 100
<212> DNA
<213> Escherichia coli K-12 MG1655
<220> ubib b3845
<222> 603-702
<223> ubib b3845 U00096 4024117..4024818
<313> 10132: 652-671
<400> 10132
CGGACGTTTT GAGATGGCGA AAATTGCCCG CGATCTGTTT TGCAGTGAGC 50
GTAATGCGCG GGAAGATCGC CTGTTTGGCG ATGCGTTTGC ATTTATCTGA 100
<210> 10133
<211> 100
<212> DNA
<213> Escherichia coli K-12 MG1655
<220> ubib b3845
<222> 533-632
<223> ubib b3845 U00096 4024117..4024818
<313> 10133: 580-599
<400> 10133
GTACTGGCAC CGTGTTAACG GCGGTATTGC AGGATCACGG TACGCTGGCA 50
GAGCATGATA TCTATATTGC CGGACGTTTT GAGATGGCGA AAATTGCCCG 100
<210> 10134
<211> 100
<212> DNA

<213> Escherichia coli K-12 MG1655
<220> ubib b3845
<222> 484-583
<223> ubib b3845 U00096 4024117..4024818
<313> 10134: 520-539
<400> 10134
CTGCAAGTGG TGCCGGTGGT TGAACAACCG GAAGCGGGCT GGCGTGGGCG  50
TACTGGCACC GTGTTAACGG CGGTATTGCA GGATCACGGT ACGCTGGCAG  100
<210> 10135
<211> 100
<212> DNA
<213> Escherichia coli K-12 MG1655
<220> ubic b4040
<222> 510-609
<223> ubic b4040 U00096 4249974..4250582
<313> 10135: 576-595
<400> 10135
CCGTGATGCC GGGCTGTGGG GGCGACGTTC CCGCCTGCGA TTAAGCGGTA  50
AACCGCTGTT GCTAACAGAA CTGTTTTTAC CGGCGTCACC GTTGTACTAA  100
<210> 10136
<211> 100
<212> DNA
<213> Escherichia coli K-12 MG1655
<220> ubic b4040
<222> 478-577
<223> ubic b4040 U00096 4249974..4250582
<313> 10136: 500-519
<400> 10136
TCGACATTAA CCCGGGACTT TATTGAGATA GGCCGTGATG CCGGGCTGTG  50
GGGGCGACGT TCCCGCCTGC GATTAAGCGG TAAACCGCTG TTGCTAACAG  100
<210> 10137
<211> 100
<212> DNA
<213> Escherichia coli K-12 MG1655
<220> ubic b4040
<222> 442-541
<223> ubic b4040 U00096 4249974..4250582
<313> 10137: 463-482
<400> 10137
GGTAAAACGC CGTTAGGACG CTATCTGTTC ACATCATCGA CATTAACCCG  50
GGACTTTATT GAGATAGGCC GTGATGCCGG GCTGTGGGGG CGACGTTCCC  100
<210> 10138
<211> 100
<212> DNA
<213> Escherichia coli K-12 MG1655
<220> hyba hybA
<222> 888-987
<223> hyba hybA U00096 complement(3142169..3143155)
<313> 10138: 954-973
<400> 10138
CATGATGCTA CCACTGGCTG TGCTGGCGGG CTTAACCGTG CTGGTTCGTC  50
GCAACACCAA AAACGACCAT CACGACGGAG GAGACGATCA TGAGTCATGA  100
<210> 10139
<211> 100
<212> DNA
<213> Escherichia coli K-12 MG1655
<220> hyba hybA
<222> 854-953
<223> hyba hybA U00096 complement(3142169..3143155)
<313> 10139: 893-912
<400> 10139
GTTCCGAAAA TATTCAACAC ACCCTGTATA AAGGCATGAT GCTACCACTG  50
GCTGTGCTGG CGGGCTTAAC CGTGCTGGTT CGTCGCAACA CCAAAAACGA  100
<210> 10140
<211> 100
<212> DNA
<213> Escherichia coli K-12 MG1655
<220> hyba hybA
<222> 777-876
<223> hyba hybA U00096 complement(3142169..3143155)
<313> 10140: 826-845

<400> 10140
TCAGGTTCTG GTACTGACGG GTGTGCCTTA TGAAAATCTC GACCTGCCGA 50
AACTGGACGA TCTTTCTACC GGTGCGCGTT CCGAAAATAT TCAACACACC 100
<210> 10141
<211> 100
<212> DNA
<213> Escherichia coli K-12 MG1655
<220> hybb b2996
<222> 1043-1142
<223> hybb b2996 U00096 complement(3141001..3142179)
<313> 10141: 1111-1130
<400> 10141
CCTGGGAAGA ACTGTTGATT TCTATTGGTT TTGTGGCTAT TGAGATTTGC 50
GCTTACATCG TACTCATTCG TCTACTGCCG ATACTTCCTC CTTTAAAACA 100
<210> 10142
<211> 100
<212> DNA
<213> Escherichia coli K-12 MG1655
<220> hybb b2996
<222> 901-1000
<223> hybb b2996 U00096 complement(3141001..3142179)
<313> 10142: 954-973
<400> 10142
GTTCTGCGTG TGGCGAAGCT GCGTAATGAT TCCCGCATGC TGTTCCTGTC 50
AGCACTGAGC GCACTGTTAG GTTGTGCAAC CTGGCGTCTG ACCTATTCGC 100
<210> 10143
<211> 100
<212> DNA
<213> Escherichia coli K-12 MG1655
<220> hybb b2996
<222> 831-930
<223> hybb b2996 U00096 complement(3141001..3142179)
<313> 10143: 875-894
<400> 10143
AGCGTTTGCC GGTGACTTCT ACTCCGTGAT GTTCTGGATT GAAGTCCTGC 50
TGATGCTCTT CCCGCTGGTC GTTCTGCGTG TGGCGAAGCT GCGTAATGAT 100
<210> 10144
<211> 100
<212> DNA
<213> Escherichia coli K-12 MG1655
<220> ubie b3834
<222> 619-718
<223> ubie b3834 U00096 4016442..4017197
<313> 10144: 667-686
<400> 10144
TATCTGGCAG AATCCATCCG TATGCATCCC GATCAGGATA CCCTGAAAGC 50
CATGATGCAG GATGCCGGAT TCGAAAGTGT CGACTACTAC AATCTGACGG 100
<210> 10145
<211> 100
<212> DNA
<213> Escherichia coli K-12 MG1655
<220> ubie b3834
<222> 561-660
<223> ubie b3834 U00096 4016442..4017197
<313> 10145: 611-630
<400> 10145
CTTCCATGTG CTGCCGCGTA TTGGCTCACT GGTCGCGAAC GACGCCGACA 50
GCTACCGTTA TCTGGCAGAA TCCATCCGTA TGCATCCCGA TCAGGATACC 100
<210> 10146
<211> 100
<212> DNA
<213> Escherichia coli K-12 MG1655
<220> ubie b3834
<222> 539-638
<223> ubie b3834 U00096 4016442..4017197
<313> 10146: 572-591
<400> 10146
GCAAAGCCTA TGATGCATAC TCCTTCCATG TGCTGCCGCG TATTGGCTCA 50
CTGGTCGCGA ACGACGCCGA CAGCTACCGT TATCTGGCAG AATCCATCCG 100
<210> 10147
<211> 100

<212> DNA
<213> Escherichia coli K-12 MG1655
<220> hybc b2995
<222> 1605-1704
<223> hybc b2995 U00096 complement(3139301..3141004)
<313> 10147: 1669-1688
<400> 10147
AGTGGTGCGT ACCATTCACT CCTTTGACCC GTGCATGGCC TGTGCGGTAC 50
ACGTAGTGGA TGCTGACGGC AACGAAGTGG TTTCAGTGAA GGTTCTGTAA 100
<210> 10148
<211> 100
<212> DNA
<213> Escherichia coli K-12 MG1655
<220> hybc b2995
<222> 1477-1576
<223> hybc b2995 U00096 complement(3139301..3141004)
<313> 10148: 1524-1543
<400> 10148
ATCAGCAACT ACCAGGCGGT TGTTCCATCA ACCTGGAACT CTGGTCCGCG 50
TAACTTCAAT GATGACGTCG GTCCTTACGA GCAGTCGCTG GTGGGTACAC 100
<210> 10149
<211> 100
<212> DNA
<213> Escherichia coli K-12 MG1655
<220> hybc b2995
<222> 1437-1536
<223> hybc b2995 U00096 complement(3139301..3141004)
<313> 10149: 1468-1487
<400> 10149
CGGTATGCTC TCTCACTGGA TGGTTATTAA AGACGGTATC ATCAGCAACT 50
ACCAGGCGGT TGTTCCATCA ACCTGGAACT CTGGTCCGCG TAACTTCAAT 100
<210> 10150
<211> 100
<212> DNA
<213> Escherichia coli K-12 MG1655
<220> hybd b2994
<222> 396-495
<223> hybd b2994 U00096 complement(3138807..3139301)
<313> 10150: 469-488
<400> 10150
GACGGTTGAA GCAATGATTG AACCTGCGCT TGAGCAGGTT CTGGCTGCGC 50
TGCGTGAATC TGGCGTGGAA GCCATCCCAC GGGAGGCGAT TCATGACTGA 100
<210> 10151
<211> 100
<212> DNA
<213> Escherichia coli K-12 MG1655
<220> hybd b2994
<222> 355-454
<223> hybd b2994 U00096 complement(3138807..3139301)
<313> 10151: 395-414
<400> 10151
GTGATCCCGG AATCGCTGGA GCCACACATC GGCTTAACGC CGACGGTTGA 50
AGCAATGATT GAACCTGCGC TTGAGCAGGT TCTGGCTGCG CTGCGTGAAT 100
<210> 10152
<211> 100
<212> DNA
<213> Escherichia coli K-12 MG1655
<220> hybd b2994
<222> 265-364
<223> hybd b2994 U00096 complement(3138807..3139301)
<313> 10152: 318-337
<400> 10152
AAAATCTCTC CGCATCAGCT TGGCCTGGCC GACGTCTTGT CGGCCCTGCG 50
CTTCACCGGC GAGTTTCCGA AAAAGCTGAC CCTGGTCGGC GTGATCCCGG 100
<210> 10153
<211> 100
<212> DNA
<213> Escherichia coli K-12 MG1655
<220> ubig b2233
<222> 624-723
<223> ubig b2233 U00096 2337587..2338309

<313> 10153: 697-716
<400> 10153
GCGGCATATC ACTGGGCTGC ATTACAACCC GATCACTAAT ACTTTTAAAC 50
TCGGCCCCGG CGTGGATGTG AACTATATGC TGCACACGCA GAATAAGTGA 100
<210> 10154
<211> 100
<212> DNA
<213> Escherichia coli K-12 MG1655
<220> ubig b2233
<222> 578-677
<223> ubig b2233 U00096 2337587..2338309
<313> 10154: 641-660
<400> 10154
AACCGGCAGA ATTGCTGGGC TGGGTGGATC AGACCAGTTT AAAAGAGCGG 50
CATATCACTG GGCTGCATTA CAACCCGATC ACTAATACTT TTAAACTCGG 100
<210> 10155
<211> 100
<212> DNA
<213> Escherichia coli K-12 MG1655
<220> ubig b2233
<222> 529-628
<223> ubig b2233 U00096 2337587..2338309
<313> 10155: 558-577
<400> 10155
ATTTTGCGCA TGGTGCCCAA AGGCACGCAT GATGTGAAGA AGTTTATTAA 50
ACCGGCAGAA TTGCTGGGCT GGGTGGATCA GACCAGTTTA AAAGAGCGGC 100
<210> 10156
<211> 100
<212> DNA
<213> Escherichia coli K-12 MG1655
<220> hybe b2993
<222> 390-489
<223> hybe b2993 U00096 complement(3138326..3138814)
<313> 10156: 456-475
<400> 10156
CGCACGAATG ATCCTTTCGC TGCCAGTCAC GAATCCGGAT GTACCACACG 50
CAGGGCGTCG CGCCCTGCTG TTTGGTCGCA GGAGTGGCGA AAATGCATGA 100
<210> 10157
<211> 100
<212> DNA
<213> Escherichia coli K-12 MG1655
<220> hybe b2993
<222> 277-376
<223> hybe b2993 U00096 complement(3138326..3138814)
<313> 10157: 329-348
<400> 10157
ACCTTTACCG TTGGCGAACT GGACGGTGTT TCGCAATATC TCTCCTGTTC 50
GCTGATGTCG CCGCTTTCGC ACAGCATGTC GATTGAAGAG GGCCAACGCC 100
<210> 10158
<211> 100
<212> DNA
<213> Escherichia coli K-12 MG1655
<220> hybe b2993
<222> 202-301
<223> hybe b2993 U00096 complement(3138326..3138814)
<313> 10158: 237-256
<400> 10158
CCCGGCCCGG ATCAACTCTG GCCGCTGCGC AAAGTGAGTG AAAAAATTGG 50
TCTGCAACTG CCGTATGGCA CTATGACCTT TACCGTTGGC GAACTGGACG 100
<210> 10159
<211> 100
<212> DNA
<213> Escherichia coli K-12 MG1655
<220> ubih b2908
<222> 1075-1174
<223> ubih b2908 U00096 complement(3050360..3051538)
<313> 10159: 1123-1142
<400> 10159
GCACCGCTGG TTGTCGGGCG CAACATCGGG CTGATGACGA TGGAATTATT 50
CACCCCGGCA CGCGATGTGC TGGCGCAGCG CACCCTCGGT TGGGTGGCGC 100
<210> 10160

<211> 100
<212> DNA
<213> Escherichia coli K-12 MG1655
<220> ubih b2908
<222> 1042-1141
<223> ubih b2908 U00096 complement(3050360..3051538)
<313> 10160: 1067-1086
<400> 10160
GACAGCCTTG TACATCTTTT TGCCAACCGT TGGGCACCGC TGGTTGTCGG 50
GCGCAACATC GGGCTGATGA CGATGGAATT ATTCACCCCG GCACGCGATG 100
<210> 10161
<211> 100
<212> DNA
<213> Escherichia coli K-12 MG1655
<220> ubih b2908
<222> 912-1011
<223> ubih b2908 U00096 complement(3050360..3051538)
<313> 10161: 960-979
<400> 10161
AGATGTGATG AGTCTTGCGG AAACCCTGAC TCAGGCGCAG GAGCGCGGAG 50
AAGACATGGG GGATTACGGC GTATTGTGCC GTTATCAGCA GCGTCGACAG 100
<210> 10162
<211> 100
<212> DNA
<213> Escherichia coli K-12 MG1655
<220> hybf b2992
<222> 176-275
<223> hybf b2992 U00096 complement(3137992..3138333)
<313> 10162: 224-243
<400> 10162
GGTGCGATTT ACATATCGTC TATAAACCCG CCCAGGCTTG GTGCTGGGAT 50
TGCAGCCAGG TGGTGGAGAT TCATCAGCAC GATGCGCAGT GTCCGCTCTG 100
<210> 10163
<211> 100
<212> DNA
<213> Escherichia coli K-12 MG1655
<220> hybf b2992
<222> 157-256
<223> hybf b2992 U00096 complement(3137992..3138333)
<313> 10163: 191-210
<400> 10163
CACGGAACGG TGGCGCAAGG GTGCGATTTA CATATCGTCT ATAAACCCGC 50
CCAGGCTTGG TGCTGGGATT GCAGCCAGGT GGTGGAGATT CATCAGCACG 100
<210> 10164
<211> 100
<212> DNA
<213> Escherichia coli K-12 MG1655
<220> hybf b2992
<222> 25-124
<223> hybf b2992 U00096 complement(3137992..3138333)
<313> 10164: 47-66
<400> 10164
AGCGCCGTTG AAATTATCCA ACGGCAGGCG GAGCAGCACG ATGTTAAGCG 50
CGTCACCGCC GTGTGGCTGG AAATTGGCGC GCTCTCCTGC GTTGAGGAGA 100
<210> 10165
<211> 100
<212> DNA
<213> Escherichia coli K-12 MG1655
<220> hybg b2991
<222> 150-249
<223> hybg b2991 U00096 complement(3137731..3137979)
<313> 10165: 217-236
<400> 10165
GGTACACGTC GGATTTGCCA TGAGCATCAT CGACGAAGAT GAAGCCAAAG 50
CCACATTAGA CGCACTGCGC CAAATGGATT ACGACATTAC CAGCGCGTGA 100
<210> 10166
<211> 100
<212> DNA
<213> Escherichia coli K-12 MG1655
<220> hybg b2991
<222> 120-219

<223> hybg b2991 U00096 complement(3137731..3137979)
<313> 10166: 182-201
<400> 10166
CCCTGCCGAT CTACTGGGCC AGTGGGTGCT GGTACACGTC GGATTTGCCA  50
TGAGCATCAT CGACGAAGAT GAAGCCAAAG CCACATTAGA CGCACTGCGC  100
<210> 10167
<211> 100
<212> DNA
<213> Escherichia coli K-12 MG1655
<220> hybg b2991
<222> 79-178
<223> hybg b2991 U00096 complement(3137731..3137979)
<313> 10167: 116-135
<400> 10167
ATCAAGCGCG ATGTGAATAT CGCCCTGATT TGTGAAGGTA ACCCTGCCGA  50
TCTACTGGGC CAGTGGGTGC TGGTACACGT CGGATTTGCC ATGAGCATCA  100
<210> 10168
<211> 100
<212> DNA
<213> Escherichia coli K-12 MG1655
<220> b1134 b1135
<222> 363-462
<223> b1134 b1135 U00096 complement(1193050..1193511)
<313> 10168: 415-434
<400> 10168
GCTGGTGGCG GAAAGTATTC GTTGTTATCA AAGCGGGCAA CGTTATCCGC  50
TGGAGATGAT TGGCGATTTT AACTGGCCTT TTACAAAGGG TGTCATCTAA  100
<210> 10169
<211> 100
<212> DNA
<213> Escherichia coli K-12 MG1655
<220> b1134 b1135
<222> 308-407
<223> b1134 b1135 U00096 complement(1193050..1193511)
<313> 10169: 348-367
<400> 10169
GCTGCCGTTG GGTCAGCGCC GAAGAAATTT TACAGGCGTC AAATCTTCGT  50
TCGCCGCTGG TGGCGGAAAG TATTCGTTGT TATCAAAGCG GGCAACGTTA  100
<210> 10170
<211> 100
<212> DNA
<213> Escherichia coli K-12 MG1655
<220> b1134 b1135
<222> 252-351
<223> b1134 b1135 U00096 complement(1193050..1193511)
<313> 10170: 273-292
<400> 10170
TGCCATTGAG CTTGAGCAAA TATGCCCGAC CCAGCCTCAT GACAGCGATA  50
TCGACTGCTG CCGTTGGGTC AGCGCCGAAG AAATTTTACA GGCGTCAAAT  100
<210> 10171
<211> 100
<212> DNA
<213> Escherichia coli K-12 MG1655
<220> ymga b1166
<222> 142-241
<223> ymga b1166 U00096 1215291..1215563
<313> 10171: 201-220
<400> 10171
TTCCGACGCG ATTTAATTGA CAGCATAATT CGTGATTTTA GCAATAGTGA  50
TTCAGGAGGG CCAAACCGAA GAACAGCGAC AGCTGACAAT AAAAGTATGT  100
<210> 10172
<211> 100
<212> DNA
<213> Escherichia coli K-12 MG1655
<220> ymga b1166
<222> 151-250
<223> ymga b1166 U00096 1215291..1215563
<313> 10172: 185-204
<400> 10172
GATTTAATTG ACAGCATAAT TCGTGATTTT AGCAATAGTG ATTCAGGAGG  50
GCCAAACCGA AGAACAGCGA CAGCTGACAA TAAAAGTATG TTCAATGGTA  100

<210> 10173
<211> 100
<212> DNA
<213> Escherichia coli K-12 MG1655
<220> ymga b1166
<222> 132-231
<223> ymga b1166 U00096 1215291..1215563
<313> 10173: 179-198
<400> 10173
GGAAGATGCT TTCCGACGCG ATTTAATTGA CAGCATAATT CGTGATTTTA 50
GCAATAGTGA TTCAGGAGGG CCAAACCGAA GAACAGCGAC AGCTGACAAT 100
<210> 10174
<211> 100
<212> DNA
<213> Escherichia coli K-12 MG1655
<220> ymgb b1167
<222> 149-248
<223> ymgb b1167 U00096 1215592..1215858
<313> 10174: 204-223
<400> 10174
ATAAAAATAT TATCTTAAGT CTGATACACT CCTTGGAAAC AACAAGTGAT 50
ATTCTCAAAG CTGATGTGAT TAGAAAAACA CTGGAAATCG TGTTGCGATA 100
<210> 10175
<211> 100
<212> DNA
<213> Escherichia coli K-12 MG1655
<220> ymgb b1167
<222> 165-264
<223> ymgb b1167 U00096 1215592..1215858
<313> 10175: 197-216
<400> 10175
AAGTCTGATA CACTCCTTGG AAACAACAAG TGATATTCTC AAAGCTGATG 50
TGATTAGAAA AACACTGGAA ATCGTGTTGC GATACACAGC TGATGATATG 100
<210> 10176
<211> 100
<212> DNA
<213> Escherichia coli K-12 MG1655
<220> ymgb b1167
<222> 161-260
<223> ymgb b1167 U00096 1215592..1215858
<313> 10176: 183-202
<400> 10176
TCTTAAGTCT GATACACTCC TTGGAAACAA CAAGTGATAT TCTCAAAGCT 50
GATGTGATTA GAAAAACACT GGAAATCGTG TTGCGATACA CAGCTGATGA 100
<210> 10177
<211> 100
<212> DNA
<213> Escherichia coli K-12 MG1655
<220> ymgc ymgC
<222> 141-240
<223> ymgc ymgC U00096 1215971..1216219
<313> 10177: 190-209
<400> 10177
CAGTGTGATT CTTTCTGATC TCTATCTGCA AATATTGCAG TACATTGAAA 50
TGCATAAGAC TACACTAACG GATATCATTA TTAATGACAG GGAATCCGTG 100
<210> 10178
<211> 100
<212> DNA
<213> Escherichia coli K-12 MG1655
<220> ymgc ymgC
<222> 100-199
<223> ymgc ymgC U00096 1215971..1216219
<313> 10178: 138-157
<400> 10178
ATTATTGATC AGGCTTTACG CTTGCGTTTA AAAGATGAAA CCAGTGTGAT 50
TCTTTCTGAT CTCTATCTGC AAATATTGCA GTACATTGAA ATGCATAAGA 100
<210> 10179
<211> 100
<212> DNA
<213> Escherichia coli K-12 MG1655
<220> ymgc ymgC

<222> 82-181
<223> ymgc ymgC U00096 1215971..1216219
<313> 10179: 126-145
<400> 10179
GGGTATGTTG ATAGTCATAT TATTGATCAG GCTTTACGCT TGCGTTTAAA 50
AGATGAAACC AGTGTGATTC TTTCTGATCT CTATCTGCAA ATATTGCAGT 100
<210> 10180
<211> 100
<212> DNA
<213> Escherichia coli K-12 MG1655
<220> ymge b1196
<222> 156-255
<223> ymge b1196 U00096 1243951..1244205
<313> 10180: 225-244
<400> 10180
TGGCGGCTCC ATCAGTGGTT TTAATCTGCA CAGCTTCCTG GTGGCGGTGG 50
TGGGAGCTAT TCTCGTTCTG GGCATATTCC GCCTCCTGCG AAGAGAATAA 100
<210> 10181
<211> 100
<212> DNA
<213> Escherichia coli K-12 MG1655
<220> ymge b1196
<222> 122-221
<223> ymge b1196 U00096 1243951..1244205
<313> 10181: 182-201
<400> 10181
TGGTCGGCGG CTGGCTGGCG ACCATGTTTG GCATTGGCGG CTCCATCAGT 50
GGTTTTAATC TGCACAGCTT CCTGGTGGCG GTGGTGGGAG CTATTCTCGT 100
<210> 10182
<211> 100
<212> DNA
<213> Escherichia coli K-12 MG1655
<220> ymge b1196
<222> 18-117
<223> ymge b1196 U00096 1243951..1244205
<313> 10182: 75-94
<400> 10182
GATTATTTTT GACCTGATAG CCGGCATTAT CGCCAAGCTA ATCATGCCGG 50
GGCGTGATGG TGGTGGATTT TTCCTGACCT GTATTCTCGG GATAGTCGGT 100
<210> 10183
<211> 100
<212> DNA
<213> Escherichia coli K-12 MG1655
<220> ubix b2312
<222> 471-570
<223> ubix b2312 U00096 complement(2426077..2426646)
<313> 10183: 525-544
<400> 10183
ATCCCTTGAT GATGTGATAA ATCAGACGGT TAATCGTGTT CTTGACCAGT 50
TTGCGATAAC CCTTCCTGAA GATCTCTTTG CCCGCTGGCA GGGCGCATAA 100
<210> 10184
<211> 100
<212> DNA
<213> Escherichia coli K-12 MG1655
<220> ubix b2312
<222> 443-542
<223> ubix b2312 U00096 complement(2426077..2426646)
<313> 10184: 471-490
<400> 10184
CCGTTCCGGC GTTTTATCAT CGCCCGCAAT CCCTTGATGA TGTGATAAAT 50
CAGACGGTTA ATCGTGTTCT TGACCAGTTT GCGATAACCC TTCCTGAAGA 100
<210> 10185
<211> 100
<212> DNA
<213> Escherichia coli K-12 MG1655
<220> ubix b2312
<222> 374-473
<223> ubix b2312 U00096 complement(2426077..2426646)
<313> 10185: 427-446
<400> 10185
CACCATTGCA CTTAGGCCAT CTGCGTTTAA TGACTCAGGC GGCAGAAATC 50

GGTGCGGTGA TTATGCCTCC CGTTCCGGCG TTTTATCATC GCCCGCAATC 100
<210> 10186
<211> 100
<212> DNA
<213> Escherichia coli K-12 MG1655
<220> mray b0088
<222> 984-1083
<223> mray b0088 U00096 96002..97084
<313> 10186: 1053-1072
<400> 10186
TGAACTGAAA GGCTGGCCGG AACCGCGCGT CATTGTGCGT TTCTGGATTA 50
TTTCGCTGAT GCTGGTTCTG ATTGGTCTGG CAACGCTGAA GGTACGTTAA 100
<210> 10187
<211> 100
<212> DNA
<213> Escherichia coli K-12 MG1655
<220> mray b0088
<222> 915-1014
<223> mray b0088 U00096 96002..97084
<313> 10187: 972-991
<400> 10187
CCTGCAGGTC GGCTCCTTTA AACTGCGCGG ACAACGTATT TTCCGCATGG 50
CACCGATTCA TCACCACTAT GAACTGAAAG GCTGGCCGGA ACCGCGCGTC 100
<210> 10188
<211> 100
<212> DNA
<213> Escherichia coli K-12 MG1655
<220> mray b0088
<222> 780-879
<223> mray b0088 U00096 96002..97084
<313> 10188: 835-854
<400> 10188
GGCGCAGGTC TTTATGGGCG ATGTAGGTTC GCTGGCGTTA GGTGGTGCGT 50
TAGGCATTAT CGCCGTACTG CTACGTCAGG AATTCCTGCT GGTGATTATG 100
<210> 10189
<211> 100
<212> DNA
<213> Escherichia coli K-12 MG1655
<220> cira b2156
<222> 1804-1903
<223> cira b2156 U00096 complement(2242798..2244789)
<313> 10189: 1858-1877
<400> 10189
GACAGCGCGA CGGCTAAAAC ACCGGGCGGT TATACCATCT GGAATACCGG 50
CGCGGCCTGG CAGGTGACTA AAGACGTCAA ACTGCGCGCA GGCGTGCTGA 100
<210> 10190
<211> 100
<212> DNA
<213> Escherichia coli K-12 MG1655
<220> cira b2156
<222> 1780-1879
<223> cira b2156 U00096 complement(2242798..2244789)
<313> 10190: 1809-1828
<400> 10190
CACTATACCG GGCAGAAACG CGCCGACAGC GCGACGGCTA AAACACCGGG 50
CGGTTATACC ATCTGGAATA CCGGCGCGGC CTGGCAGGTG ACTAAAGACG 100
<210> 10191
<211> 100
<212> DNA
<213> Escherichia coli K-12 MG1655
<220> cira b2156
<222> 1690-1789
<223> cira b2156 U00096 complement(2242798..2244789)
<313> 10191: 1721-1740
<400> 10191
CCGCTATCCG ATCTGCCGTT CCATACTGCT AACGGTACGC TGGACTGGAA 50
ACCGCTGGCG CTGGAAGACT GGTCATTCTA TGTTTCTGGG CACTATACCG 100
<210> 10192
<211> 100
<212> DNA
<213> Escherichia coli K-12 MG1655

<220> hyca b2726
<222> 363-462
<223> hyca b2726 U00096 complement(2847997..2848458)
<313> 10192: 438-457
<400> 10192
AAAAATCGCT GGCGTTTACG ACAGCTTATA CACCGCTATT GAAAACAATG 50
TGCCGGTGAA TTTAAGCCAA CTGGTAAAGG GACAAAGCCC GGCAGCATGA 100
<210> 10193
<211> 100
<212> DNA
<213> Escherichia coli K-12 MG1655
<220> hyca b2726
<222> 328-427
<223> hyca b2726 U00096 complement(2847997..2848458)
<313> 10193: 384-403
<400> 10193
CGCGATCGCG AACAGGTTCT GGAACACTAT CTCGAAAAAA TCGCTGGCGT 50
TTACGACAGC TTATACACCG CTATTGAAAA CAATGTGCCG GTGAATTTAA 100
<210> 10194
<211> 100
<212> DNA
<213> Escherichia coli K-12 MG1655
<220> hyca b2726
<222> 270-369
<223> hyca b2726 U00096 complement(2847997..2848458)
<313> 10194: 307-326
<400> 10194
GCGGATTGCG CAGGCGCAAC TGAGCATTGA CGGCATGATT GATGGCAAGG 50
TCAACATCCG CGATCGCGAA CAGGTTCTGG AACACTATCT CGAAAAAATC 100
<210> 10195
<211> 100
<212> DNA
<213> Escherichia coli K-12 MG1655
<220> hycb b2725
<222> 513-612
<223> hycb b2725 U00096 complement(2847261..2847872)
<313> 10195: 581-600
<400> 10195
TATCGCCCGC GTCAGCAAAC GTAAGCGTGA GCTGACCTTT AACACGGACT 50
TTGGCGATCT CACCTTGTTT CAGCAGGCTC AAAGTGGAGA GGCTAAATGA 100
<210> 10196
<211> 100
<212> DNA
<213> Escherichia coli K-12 MG1655
<220> hycb b2725
<222> 495-594
<223> hycb b2725 U00096 complement(2847261..2847872)
<313> 10196: 543-562
<400> 10196
TCTGGTGGAT AACACCGATA TCGCCCGCGT CAGCAAACGT AAGCGTGAGC 50
TGACCTTTAA CACGGACTTT GGCGATCTCA CCTTGTTTCA GCAGGCTCAA 100
<210> 10197
<211> 100
<212> DNA
<213> Escherichia coli K-12 MG1655
<220> hycb b2725
<222> 445-544
<223> hycb b2725 U00096 complement(2847261..2847872)
<313> 10197: 496-515
<400> 10197
GATGAACAAG GTCCGGCCTG CGTGCGGATG TGCCCGACTA AAGCCCTGCA 50
TCTGGTGGAT AACACCGATA TCGCCCGCGT CAGCAAACGT AAGCGTGAGC 100
<210> 10198
<211> 100
<212> DNA
<213> Escherichia coli K-12 MG1655
<220> hycc b2724
<222> 1643-1742
<223> hycc b2724 U00096 complement(2845438..2847264)
<313> 10198: 1693-1712
<400> 10198

CCGCTCACGG TTTTGCCATG CCGGTGAAAC AGGCGTTTGC GCCGGTGCTG 50
AAACTACGCA AATGGCTGAA TCCGGTGTCT CTGGTGCCGG GCTGGCAGTG 100
<210> 10199
<211> 100
<212> DNA
<213> Escherichia coli K-12 MG1655
<220> hycc b2724
<222> 1495-1594
<223> hycc b2724 U00096 complement(2845438..2847264)
<313> 10199: 1538-1557
<400> 10199
TCTCAACCGA TGATCACGTT GCTGCTGATT GCCTGCCCGC TGCTGCCATT 50
CATCATTATG GCGATTTGCA AAGGCGATCG TTTGCCATCG CGTTCCCGCG 100
<210> 10200
<211> 100
<212> DNA
<213> Escherichia coli K-12 MG1655
<220> hycc b2724
<222> 1457-1556
<223> hycc b2724 U00096 complement(2845438..2847264)
<313> 10200: 1486-1505
<400> 10200
CTGTACCTCT GCCGCTGGAG CCTGCTAACA CCACCGTTTC TCAACCGATG 50
ATCACGTTGC TGCTGATTGC CTGCCCGCTG CTGCCATTCA TCATTATGGC 100
<210> 10201
<211> 100
<212> DNA
<213> Escherichia coli K-12 MG1655
<220> hycd b2723
<222> 808-907
<223> hycd b2723 U00096 complement(2844512..2845435)
<313> 10201: 847-866
<400> 10201
GTTATCGCGC TGTTCGAAAA CAGCATGGCC CGTCTGCGTC TTGATATTAC 50
TCCGCGCATT ACCTGGGCTG GGTTTGGCTT TGCATTTTTA GCGTTCGTCT 100
<210> 10202
<211> 100
<212> DNA
<213> Escherichia coli K-12 MG1655
<220> hycd b2723
<222> 659-758
<223> hycd b2723 U00096 complement(2844512..2845435)
<313> 10202: 702-721
<400> 10202
TGAAATGGGG TATCAGCCTG AAACAGCTGG TGGTGTTGCA GATGTTCGTC 50
GGGGTGTTTA TTCCGTGGGG ACAAATGGAA ACCTTCACCG CCGGTGGACT 100
<210> 10203
<211> 100
<212> DNA
<213> Escherichia coli K-12 MG1655
<220> hycd b2723
<222> 603-702
<223> hycd b2723 U00096 complement(2844512..2845435)
<313> 10203: 627-646
<400> 10203
GCAGGAGCTG CAGGAAGGCC CGCTCTCTGA ATACAGCGGC AGCGGCTTTG 50
GCGTCATGAA ATGGGGTATC AGCCTGAAAC AGCTGGTGGT GTTGCAGATG 100
<210> 10204
<211> 100
<212> DNA
<213> Escherichia coli K-12 MG1655
<220> b1141 b1142
<222> 147-246
<223> b1141 b1142 U00096 complement(1200010..1200255)
<313> 10204: 213-232
<400> 10204
GGACGCCGAG TTAGTTGGGG AGTTGGTAAC ACCTGTAATC AAGAAAAATG 50
ACTCATTACT TTTGCAACGG ATTTTGAGTA ATGGCAGCCA GACCGCGTAA 100
<210> 10205
<211> 100
<212> DNA

<213> Escherichia coli K-12 MG1655
<220> b1141 b1142
<222> 103-202
<223> b1141 b1142 U00096 complement(1200010..1200255)
<313> 10205: 164-183
<400> 10205
TGTCCACCGG CCATGAAACA AGGCCGGTTA TGGCGAGTGC GTGAGGACGC 50
CGAGTTAGTT GGGGAGTTGG TAACACCTGT AATCAAGAAA AATGACTCAT 100
<210> 10206
<211> 100
<212> DNA
<213> Escherichia coli K-12 MG1655
<220> b1141 b1142
<222> 64-163
<223> b1141 b1142 U00096 complement(1200010..1200255)
<313> 10206: 81-100
<400> 10206
GTGTCCACAT TGCGTCGTTA TGCTAAGCAG AATTTATTTT GTCCACCGGC 50
CATGAAACAA GGCCGGTTAT GGCGAGTGCG TGAGGACGCC GAGTTAGTTG 100
<210> 10207
<211> 100
<212> DNA
<213> Escherichia coli K-12 MG1655
<220> hyce b2722
<222> 1611-1710
<223> hyce b2722 U00096 complement(2842785..2844494)
<313> 10207: 1673-1692
<400> 10207
CATGACCGTG GTCGATGTGC GTAAGAAGAA GAGCAAAGTG GTGCCGTACA 50
AAGAACTCGA GCGTTACAGC ATTGAGCGTA AAAACTCGCC GCTGAAATAA 100
<210> 10208
<211> 100
<212> DNA
<213> Escherichia coli K-12 MG1655
<220> hyce b2722
<222> 1552-1651
<223> hyce b2722 U00096 complement(2842785..2844494)
<313> 10208: 1581-1600
<400> 10208
GTTTCCGATG CGCCGCTGAT TATCGGTAGC CTCGACCCTT GCTACTCCTG 50
TACCGACCGC ATGACCGTGG TCGATGTGCG TAAGAAGAAG AGCAAAGTGG 100
<210> 10209
<211> 100
<212> DNA
<213> Escherichia coli K-12 MG1655
<220> hyce b2722
<222> 1471-1570
<223> hyce b2722 U00096 complement(2842785..2844494)
<313> 10209: 1503-1522
<400> 10209
CAGAAGCTGT ACCGCTGGCG CTGCCGTGCC GCGACCTACG CGAACTGGCC 50
GACCCTGCGC TACATGCTGC GCGGCAACAC CGTTTCCGAT GCGCCGCTGA 100
<210> 10210
<211> 100
<212> DNA
<213> Escherichia coli K-12 MG1655
<220> hycf b2721
<222> 444-543
<223> hycf b2721 U00096 complement(2842233..2842775)
<313> 10210: 507-526
<400> 10210
CCGCGAAAGC TTTGAGACTT GCCCGGAATG TAAGCGCCAG AAATGCCTGG 50
TGCCGTCCGA CCGTATTGAA CTGACTCGCC ATATGAAAGA GGCCATCTGA 100
<210> 10211
<211> 100
<212> DNA
<213> Escherichia coli K-12 MG1655
<220> hycf b2721
<222> 411-510
<223> hycf b2721 U00096 complement(2842233..2842775)
<313> 10211: 467-486

<400> 10211
TAAGCACAAC GGCGACAGCC GCGCGGAAAA CCACCGCGAA AGCTTTGAGA 50
CTTGCCCGGA ATGTAAGCGC CAGAAATGCC TGGTGCCGTC CGACCGTATT 100
<210> 10212
<211> 100
<212> DNA
<213> Escherichia coli K-12 MG1655
<220> hycf b2721
<222> 345-444
<223> hycf b2721 U00096 complement(2842233..2842775)
<313> 10212: 384-403
<400> 10212
CAACTGCCGC GTCTGCAATC GTCCTTTCGC CGTCCAGAAA GAGATCGACT 50
ACGCCATTGC GCTGCTTAAG CACAACGGCG ACAGCCGCGC GGAAAACCAC 100
<210> 10213
<211> 100
<212> DNA
<213> Escherichia coli K-12 MG1655
<220> hycg b2720
<222> 669-768
<223> hycg b2720 U00096 complement(2841466..2842233)
<313> 10213: 732-751
<400> 10213
AGAACAGGTT GCACGCTGGC TGGAAGCGGA AAACGATCCG CGTCTGAACG 50
AGATTGTCAG CCATCTGAAT CATGTTGTTG AAGAGGCGCG TATCCGATGA 100
<210> 10214
<211> 100
<212> DNA
<213> Escherichia coli K-12 MG1655
<220> hycg b2720
<222> 669-768
<223> hycg b2720 U00096 complement(2841466..2842233)
<313> 10214: 704-723
<400> 10214
AGAACAGGTT GCACGCTGGC TGGAAGCGGA AAACGATCCG CGTCTGAACG 50
AGATTGTCAG CCATCTGAAT CATGTTGTTG AAGAGGCGCG TATCCGATGA 100
<210> 10215
<211> 100
<212> DNA
<213> Escherichia coli K-12 MG1655
<220> hycg b2720
<222> 595-694
<223> hycg b2720 U00096 complement(2841466..2842233)
<313> 10215: 663-682
<400> 10215
GCACGTCGCC TGGCGGGTTA TCGTTACGGT CGTCAGATTG CCGATGATTA 50
CCTTACACAG TTAGGGCAGG GCGAAGAACA GGTTGCACGC TGGCTGGAAG 100
<210> 10216
<211> 100
<212> DNA
<213> Escherichia coli K-12 MG1655
<220> hych b2719
<222> 273-372
<223> hych b2719 U00096 complement(2841059..2841469)
<313> 10216: 304-323
<400> 10216
GCTGGCCAAC GCATTCGATA AAGCCCGGGC AGCGCAAACT TCGCAGCAGC 50
AGGAATGGAG TACAATGCTG TTAAGTATGC TGCATGATAT TCATCAGGAA 100
<210> 10217
<211> 100
<212> DNA
<213> Escherichia coli K-12 MG1655
<220> hych b2719
<222> 213-312
<223> hych b2719 U00096 complement(2841059..2841469)
<313> 10217: 245-264
<400> 10217
CAAAATGGAA GGCGTGCCGA AATATGGTGA GATCGTCATC GACATTAACC 50
ATGTGCCGAT GCTGGCCAAC GCATTCGATA AAGCCCGGGC AGCGCAAACT 100
<210> 10218
<211> 100

<212> DNA
<213> Escherichia coli K-12 MG1655
<220> hych b2719
<222> 160-259
<223> hych b2719 U00096 complement(2841059..2841469)
<313> 10218: 208-227
<400> 10218
GATGAATATC TGGCATGGAT TGCCACTCTG GAGGCAGGCA GTGAAGCCCG 50
CCGCAAAATG GAAGGCGTGC CGAAATATGG TGAGATCGTC ATCGACATTA 100
<210> 10219
<211> 100
<212> DNA
<213> Escherichia coli K-12 MG1655
<220> b1145 b1145
<222> 524-623
<223> b1145 b1145 U00096 complement(1201482..1202156)
<313> 10219: 580-599
<400> 10219
TCAAAAGGTT GATAGAAGAT GGGACACAGC GTTATCTCAA AGCGTTAAAC 50
CCAAACTGGC CTGAGCCTTA CATTAAGATC AACGGTAATT GCTCTATAAT 100
<210> 10220
<211> 100
<212> DNA
<213> Escherichia coli K-12 MG1655
<220> b1145 b1145
<222> 491-590
<223> b1145 b1145 U00096 complement(1201482..1202156)
<313> 10220: 526-545
<400> 10220
TTGCATTGAT GCACGATACA GGTGAAACCA CCTTCAAAAG GTTGATAGAA 50
GATGGGACAC AGCGTTATCT CAAAGCGTTA AACCCAAACT GGCCTGAGCC 100
<210> 10221
<211> 100
<212> DNA
<213> Escherichia coli K-12 MG1655
<220> b1145 b1145
<222> 409-508
<223> b1145 b1145 U00096 complement(1201482..1202156)
<313> 10221: 445-464
<400> 10221
GATTCAATGA TTGATGAGTA CCGCCCGGGA GACATGATTT TTGTCGATCC 50
TGAAGTACCT GCCTGCCACG GTGACGACGT TATTGCATTG ATGCACGATA 100
<210> 10222
<211> 100
<212> DNA
<213> Escherichia coli K-12 MG1655
<220> hyci b2718
<222> 372-471
<223> hyci b2718 U00096 complement(2840596..2841066)
<313> 10222: 433-452
<400> 10222
GACCCAGCCG ATTAAAGATG CGGTAGAAAC CGTTTATCAA CGACTGGAAG 50
GCTGGGAAGG AAATGGCGGC TTCGCGCAGT TAGCGGTGGA AGAAGAGTAG 100
<210> 10223
<211> 100
<212> DNA
<213> Escherichia coli K-12 MG1655
<220> hyci b2718
<222> 353-452
<223> hyci b2718 U00096 complement(2840596..2841066)
<313> 10223: 403-422
<400> 10223
TCGGCTTTTA CTACCCGATG ACCCAGCCGA TTAAAGATGC GGTAGAAACC 50
GTTTATCAAC GACTGGAAGG CTGGGAAGGA AATGGCGGCT TCGCGCAGTT 100
<210> 10224
<211> 100
<212> DNA
<213> Escherichia coli K-12 MG1655
<220> hyci b2718
<222> 282-381
<223> hyci b2718 U00096 complement(2840596..2841066)

<313> 10224: 315-334
<400> 10224
GAATTACCTT ATCGACCAGT TGAAAGAAGA TATTGGCGAA GTGATTTTCC 50
TCGGCATTCA GCCGGATATC GTCGGCTTTT ACTACCCGAT GACCCAGCCG 100
<210> 10225
<211> 100
<212> DNA
<213> Escherichia coli K-12 MG1655
<220> b1146 b1147
<222> 400-499
<223> b1146 b1147 U00096 1201944..1202447
<313> 10225: 426-445
<400> 10225
GGCTATAAAA AAGCCAGTTT TGTGCTGGCT AAAAAACTTG AGCAATACAC 50
ATCAGGTGCA ATTACGAAAT CTGACTTAAG ACCGGATATC TATCCGAAAG 100
<210> 10226
<211> 100
<212> DNA
<213> Escherichia coli K-12 MG1655
<220> b1146 b1147
<222> 337-436
<223> b1146 b1147 U00096 1201944..1202447
<313> 10226: 367-386
<400> 10226
CAGCAGGGCG AGTTGGCCGG AAAAGTTGGC TCAACACCTG GCTACTTACG 50
GCTGGTTTTC AATGGCTATA AAAAAGCCAG TTTTGTGCTG GCTAAAAAAC 100
<210> 10227
<211> 100
<212> DNA
<213> Escherichia coli K-12 MG1655
<220> b1146 b1147
<222> 293-392
<223> b1146 b1147 U00096 1201944..1202447
<313> 10227: 321-340
<400> 10227
GTACATATTG TATGAAAGCG TATTGGGACT CTTTAACCAA AGAACAGCAG 50
GGCGAGTTGG CCGGAAAAGT TGGCTCAACA CCTGGCTACT TACGGCTGGT 100
<210> 10228
<211> 100
<212> DNA
<213> Escherichia coli K-12 MG1655
<220> fhla b2732
<222> 1980-2079
<223> fhla b2732 U00096 2852361..2854439
<313> 10228: 2050-2069
<400> 10228
TGCCGGGCCT AAAGGCGCTG CGCAACGTCT GGGGCTGAAA CGCACGACCC 50
TGCTGTCACG GATGAAGCGG CTGGGAATTG ATAAATCGGC ATTGATTTAA 100
<210> 10229
<211> 100
<212> DNA
<213> Escherichia coli K-12 MG1655
<220> fhla b2732
<222> 1935-2034
<223> fhla b2732 U00096 2852361..2854439
<313> 10229: 1962-1981
<400> 10229
ATATCAGTTG ATTGTGCGCG TGCTGAAAGA AACCAACGGC GTGGTTGCCG 50
GGCCTAAAGG CGCTGCGCAA CGTCTGGGGC TGAAACGCAC GACCCTGCTG 100
<210> 10230
<211> 100
<212> DNA
<213> Escherichia coli K-12 MG1655
<220> fhla b2732
<222> 1867-1966
<223> fhla b2732 U00096 2852361..2854439
<313> 10230: 1890-1909
<400> 10230
GATATTGTTT TACCGGAACC TGAAACGCCG CCTGCCGCAA CGGTTGTCGC 50
CCTGGAGGGC GAAGATGAAT ATCAGTTGAT TGTGCGCGTG CTGAAAGAAA 100
<210> 10231

<211> 100
<212> DNA
<213> Escherichia coli K-12 MG1655
<220> yi82_1 b0018
<222> 210-309
<223> yi82_1 b0018 U00096 complement(15869..16177)
<313> 10231: 287-306
<400> 10231
GAACCGAATC CCCGGTCAGC AATGCGTATC TCGTCTGCCG TTTGCGCAAA  50
TCGGTCCAGC CGTTCAGCGT CTCTGCTGTC GGTTAGCTCA AAATCAGTGA  100
<210> 10232
<211> 100
<212> DNA
<213> Escherichia coli K-12 MG1655
<220> yi82_1 b0018
<222> 159-258
<223> yi82_1 b0018 U00096 complement(15869..16177)
<313> 10232: 185-204
<400> 10232
ACGATATAAT CAGCTTCTCC AAAAGCAAGT GAGCGGATAC ATTCGGGACG  50
CGAACCGAAT CCCCGGTCAG CAATGCGTAT CTCGTCTGCC GTTTGCGCAA  100
<210> 10233
<211> 100
<212> DNA
<213> Escherichia coli K-12 MG1655
<220> yi82_1 b0018
<222> 112-211
<223> yi82_1 b0018 U00096 complement(15869..16177)
<313> 10233: 144-163
<400> 10233
GCATTCCTTC TGCAGTTAAC CAGCGCAATC CTCGCCAGTG AACCCGGACG  50
ATATAATCAG CTTCTCCAAA AGCAAGTGAG CGGATACATT CGGGACGCGA  100
<210> 10234
<211> 100
<212> DNA
<213> Escherichia coli K-12 MG1655
<220> teha b1430
<222> 882-981
<223> teha b1430 U00096 1498597..1499589
<313> 10234: 923-942
<400> 10234
CAATTTTATT ATTGCAATAC TGCTCATCCG TACTTTTGCG CTTCTGATGC  50
AGGGAAAATT GTTAGTCAGA ACCGAGCGCG CCGTTTTAAT GAAAGCAGAG  100
<210> 10235
<211> 100
<212> DNA
<213> Escherichia coli K-12 MG1655
<220> teha b1430
<222> 780-879
<223> teha b1430 U00096 1498597..1499589
<313> 10235: 838-857
<400> 10235
CTCGTTCGGC GTATCTGCAC TGGCAACCAC CGGTTTGCAT CTGGGGAGTG  50
GCAGCGATAA TGGATTTTTC CATACGCTGG CGGTGCCGCT GTTTATCTTT  100
<210> 10236
<211> 100
<212> DNA
<213> Escherichia coli K-12 MG1655
<220> teha b1430
<222> 737-836
<223> teha b1430 U00096 1498597..1499589
<313> 10236: 786-805
<400> 10236
CATGGTATCT CTCCCAGCCA TTTAATGCTT CATTCTGGAG TTTCTCGTTC  50
GGCGTATCTG CACTGGCAAC CACCGGTTTG CATCTGGGGA GTGGCAGCGA  100
<210> 10237
<211> 100
<212> DNA
<213> Escherichia coli K-12 MG1655
<220> div div
<222> 847-946

<223> div div U00096 2435970..2436965
<313> 10237: 897-916
<400> 10237
CGGGTTAAAG GGATTGATCC GCGTGTTATG CAACCGCTGC TTAATCCTTT 50
TCCACCGATG ATGGACACGT TGCAAAATAT GGCAACGCGT CCCGCGCTGT 100
<210> 10238
<211> 100
<212> DNA
<213> Escherichia coli K-12 MG1655
<220> div div
<222> 695-794
<223> div div U00096 2435970..2436965
<313> 10238: 752-771
<400> 10238
TGGAATCACT ACAGATGACG CTAAAACAAC CTTTAGATGC CAGTGAACTG 50
GCGGCGTTAT CGGCATATAT CCAGCAAAAA TATGGTCTTT CTGCGCAATC 100
<210> 10239
<211> 100
<212> DNA
<213> Escherichia coli K-12 MG1655
<220> div div
<222> 653-752
<223> div div U00096 2435970..2436965
<313> 10239: 699-718
<400> 10239
TACAGGCGCG TCAGACGCTA AGCCAGCAAA ATACGCCGAC GCTGGAATCA 50
CTACAGATGA CGCTAAAACA ACCTTTAGAT GCCAGTGAAC TGGCGGCGTT 100
<210> 10240
<211> 100
<212> DNA
<213> Escherichia coli K-12 MG1655
<220> mrca b3397
<222> 2454-2553
<223> mrca b3397 U00096 3520485..3523061
<313> 10240: 2496-2515
<400> 10240
GTTAGCTAAT GGTGGCAACA GCCGCGAAGA GTATTTCATC GAAGGTACGC 50
AGCCGACACA ACAGGCAGTG CACGAGGTGG GAACGACCAT TATCGATAAT 100
<210> 10241
<211> 100
<212> DNA
<213> Escherichia coli K-12 MG1655
<220> mrca b3397
<222> 2347-2446
<223> mrca b3397 U00096 3520485..3523061
<313> 10241: 2414-2433
<400> 10241
CCTGCATGGG ACGCTTATAT GAAAGCCGTT CTTGAAGGTG TGCCGGAGCA 50
GCCGCTGACG CCGCCACCGG GTATTGTGAC GGTGAATATC GATCGCAGCA 100
<210> 10242
<211> 100
<212> DNA
<213> Escherichia coli K-12 MG1655
<220> mrca b3397
<222> 2337-2436
<223> mrca b3397 U00096 3520485..3523061
<313> 10242: 2366-2385
<400> 10242
GAGTGCCCAG CCTGCATGGG ACGCTTATAT GAAAGCCGTT CTTGAAGGTG 50
TGCCGGAGCA GCCGCTGACG CCGCCACCGG GTATTGTGAC GGTGAATATC 100
<210> 10243
<211> 100
<212> DNA
<213> Escherichia coli K-12 MG1655
<220> tehb tehB
<222> 495-594
<223> tehb tehB U00096 1499586..1500179
<313> 10243: 543-562
<400> 10243
GGTGAAATAC AATGAAGACG TCGGCGAGCT GCACCGCACC GACGCCAACG 50
GTAATCGTAT TAAACTGCGT TTCGCCACGA TGCTGGCACG TAAAAAATGA 100

<210> 10244
<211> 100
<212> DNA
<213> Escherichia coli K-12 MG1655
<220> tehb tehB
<222> 441-540
<223> tehb tehB U00096 1499586..1500179
<313> 10244: 485-504
<400> 10244
CCCGTTTGCC TTCAAAGAGG GAGAATTACG TCGATATTAC GAAGGCTGGG 50
AGAGGGTGAA ATACAATGAA GACGTCGGCG AGCTGCACCG CACCGACGCC 100
<210> 10245
<211> 100
<212> DNA
<213> Escherichia coli K-12 MG1655
<220> tehb tehB
<222> 411-510
<223> tehb tehB U00096 1499586..1500179
<313> 10245: 447-466
<400> 10245
TACCGCTGAT TATCCATGTA CCGTCGGCTT CCCGTTTGCC TTCAAAGAGG 50
GAGAATTACG TCGATATTAC GAAGGCTGGG AGAGGGTGAA ATACAATGAA 100
<210> 10246
<211> 100
<212> DNA
<213> Escherichia coli K-12 MG1655
<220> mrcb b0150
<222> 2392-2491
<223> mrcb b0150 U00096 164730..167264
<313> 10246: 2450-2469
<400> 10246
GAGATGCAGC AGCAGCCGTC AGGCAATCCG TTTGATCAGT CTTCTCAGCC 50
GCAGCAACAG CCGCAACAGC AACCTGCTCA GCAAGAGCAG AAAGACAGCG 100
<210> 10247
<211> 100
<212> DNA
<213> Escherichia coli K-12 MG1655
<220> mrcb b0150
<222> 2339-2438
<223> mrcb b0150 U00096 164730..167264
<313> 10247: 2393-2412
<400> 10247
TGCGTATCTT GCCGGTCTGG ACCAGCGATC CGCAATCGCT GTGCCAGCAG 50
AGCGAGATGC AGCAGCAGCC GTCAGGCAAT CCGTTTGATC AGTCTTCTCA 100
<210> 10248
<211> 100
<212> DNA
<213> Escherichia coli K-12 MG1655
<220> mrcb b0150
<222> 2278-2377
<223> mrcb b0150 U00096 164730..167264
<313> 10248: 2307-2326
<400> 10248
CCAGAAGATA TTGCAGATAT GGGCGTGGAC TACGACGGCA ACTTTGTTTG 50
CAGCGGTGGC ATGCGTATCT TGCCGGTCTG GACCAGCGAT CCGCAATCGC 100
<210> 10249
<211> 100
<212> DNA
<213> Escherichia coli K-12 MG1655
<220> b1152 b1152
<222> 681-780
<223> b1152 b1152 U00096 1205354..1206145
<313> 10249: 743-762
<400> 10249
GGCGATTTCC GGTGCGAACG GGGAATACAG CCATCAGTTG CTTGCACCGG 50
TGGACAATAT CTCCATTGCG AAAAACGAAC TGGCGGTACT GGGGACGATT 100
<210> 10250
<211> 100
<212> DNA
<213> Escherichia coli K-12 MG1655
<220> b1152 b1152

<222> 675-774
<223> b1152 b1152 U00096 1205354..1206145
<313> 10250: 691-710
<400> 10250
CAGTGAGGCG ATTTCCGGTG CGAACGGGGA ATACAGCCAT CAGTTGCTTG  50
CACCGGTGGA CAATATCTCC ATTGCGAAAA ACGAACTGGC GGTACTGGGG  100
<210> 10251
<211> 100
<212> DNA
<213> Escherichia coli K-12 MG1655
<220> b1152 b1152
<222> 614-713
<223> b1152 b1152 U00096 1205354..1206145
<313> 10251: 646-665
<400> 10251
AGTTGCGTTC GTTCCTGCTG CGTGATGGTT ATCCGCAGGG AGAACTCAAG  50
GTATCGCGTA TCAGTGAGGC GATTTCCGGT GCGAACGGGG AATACAGCCA  100
<210> 10252
<211> 100
<212> DNA
<213> Escherichia coli K-12 MG1655
<220> b1153 b1154
<222> 486-585
<223> b1153 b1154 U00096 1206136..1206720
<313> 10252: 538-557
<400> 10252
TGATTCCGCA CTGCGTATCT GGGGTGACAC CGTTGTCGAG TGTGTGCTTA  50
ACAAACTCTG CCCGTCGCAT ACCTACGTAA TTTTTAAATA TCCGGAGTAA  100
<210> 10253
<211> 100
<212> DNA
<213> Escherichia coli K-12 MG1655
<220> b1153 b1154
<222> 456-555
<223> b1153 b1154 U00096 1206136..1206720
<313> 10253: 497-516
<400> 10253
CTCCACCTGG ATGACATGTG GCGATCCCTG TGATTCCGCA CTGCGTATCT  50
GGGGTGACAC CGTTGTCGAG TGTGTGCTTA ACAAACTCTG CCCGTCGCAT  100
<210> 10254
<211> 100
<212> DNA
<213> Escherichia coli K-12 MG1655
<220> b1153 b1154
<222> 437-536
<223> b1153 b1154 U00096 1206136..1206720
<313> 10254: 449-468
<400> 10254
ACATGCCAGC CACCACCAAC TCCACCTGGA TGACATGTGG CGATCCCTGT  50
GATTCCGCAC TGCGTATCTG GGGTGACACC GTTGTCGAGT GTGTGCTTAA  100
<210> 10255
<211> 100
<212> DNA
<213> Escherichia coli K-12 MG1655
<220> hydg b4005
<222> 1227-1326
<223> hydg b4005 U00096 4200898..4202223
<313> 10255: 1297-1316
<400> 10255
GATTCTGGCG GCGCTGGAGA AAACGGGCGG CAACAAAACC GAAGCCGCCC  50
GTCAGTTAGG GATCACGCGC AAAACGCTAT TGGCAAAACT GTCGCGTTAG  100
<210> 10256
<211> 100
<212> DNA
<213> Escherichia coli K-12 MG1655
<220> hydg b4005
<222> 1140-1239
<223> hydg b4005 U00096 4200898..4202223
<313> 10256: 1158-1177
<400> 10256
CGAGCTGCCG CTGGCGATTG CCAGTACGCC GATCCCGCTG GGACAAAGTC  50

AGGATATTCA GCCGTTGGTG GAAGTGGAAA AAGAGGTGAT TCTGGCGGCG 100
<210> 10257
<211> 100
<212> DNA
<213> Escherichia coli K-12 MG1655
<220> hydg b4005
<222> 1036-1135
<223> hydg b4005 U00096 4200898..4202223
<313> 10257: 1086-1105
<400> 10257
ATGGATCTGT TGATTCATTA CGACTGGCCG GGAAATATTC GTGAGCTGGA 50
AAACGCGGTG GAACGGGCAG TGGTGCTGCT GACCGGGGAA TATATTTCCG 100
<210> 10258
<211> 100
<212> DNA
<213> Escherichia coli K-12 MG1655
<220> hydh b4004
<222> 1299-1398
<223> hydh b4004 U00096 4199504..4200901
<313> 10258: 1345-1364
<400> 10258
ACACGGTGGT ACAATTCAGG TCGCAAGCCA GGAGGGAAAA GGCTCAACGT 50
TCACCCTCTG GCTTCCGGTC AATATTACGC GTAAGGACCC ACAAGGATGA 100
<210> 10259
<211> 100
<212> DNA
<213> Escherichia coli K-12 MG1655
<220> hydh b4004
<222> 1278-1377
<223> hydh b4004 U00096 4199504..4200901
<313> 10259: 1302-1321
<400> 10259
CGTGCATAAT ATTGTTGAAC AACACGGTGG TACAATTCAG GTCGCAAGCC 50
AGGAGGGAAA AGGCTCAACG TTCACCCTCT GGCTTCCGGT CAATATTACG 100
<210> 10260
<211> 100
<212> DNA
<213> Escherichia coli K-12 MG1655
<220> hydh b4004
<222> 1192-1291
<223> hydh b4004 U00096 4199504..4200901
<313> 10260: 1240-1259
<400> 10260
AAGGGAATTG CGGCAGATCA GCTTGATGCC ATCTTCACTC CGTACTTCAC 50
CACTAAAGCC GAAGGCACCG GATTGGGGCT GGCGGTCGTG CATAATATTG 100
<210> 10261
<211> 100
<212> DNA
<213> Escherichia coli K-12 MG1655
<220> b1155 b1156
<222> 315-414
<223> b1155 b1156 U00096 1207355..1207768
<313> 10261: 372-391
<400> 10261
TAATTTGTGG CTGGATTATC TGGAAGCACT GGAACTGGTT GATACATCCA 50
GTGCGCCAGA TATTGAATGG CCTACGCCTC CGGCAGTTCA GGCCAGATGA 100
<210> 10262
<211> 100
<212> DNA
<213> Escherichia coli K-12 MG1655
<220> b1155 b1156
<222> 253-352
<223> b1155 b1156 U00096 1207355..1207768
<313> 10262: 272-291
<400> 10262
AGCAGACAGT GGCAGGGTAA GGCTGCATTG GGAAGACTTA AAGAAGATGA 50
GCTGAAACAA TATAATTTGT GGCTGGATTA TCTGGAAGCA CTGGAACTGG 100
<210> 10263
<211> 100
<212> DNA
<213> Escherichia coli K-12 MG1655

<220> b1155 b1156
<222> 147-246
<223> b1155 b1156 U00096 1207355..1207768
<313> 10263: 195-214
<400> 10263
TTTTCCTCCT TCTATTAAAG AGGTTATCCG TACTCATGAT GATGAAGTCG  50
CAGATGCGAA CTTTCAAAAG CAGATGCTTA TCTCTGATGC AACTGATTTT  100
<210> 10264
<211> 100
<212> DNA
<213> Escherichia coli K-12 MG1655
<220> b1157 b1158
<222> 420-519
<223> b1157 b1158 U00096 complement(1208342..1208881)
<313> 10264: 468-487
<400> 10264
ACCAGCCCCG GATTATGGAA CCTTTAAAAC ATATAAACAA TCAGTGGATG  50
GTTTAGGTGC CGCAGCCTCA GAAACGCGTC CGCGTAATAT TGCATTTAAC  100
<210> 10265
<211> 100
<212> DNA
<213> Escherichia coli K-12 MG1655
<220> b1157 b1158
<222> 401-500
<223> b1157 b1158 U00096 complement(1208342..1208881)
<313> 10265: 427-446
<400> 10265
ACACAAACGA TGCCGGATTA CCAGCCCCGG ATTATGGAAC CTTTAAAACA  50
TATAAACAAT CAGTGGATGG TTTAGGTGCC GCAGCCTCAG AAACGCGTCC  100
<210> 10266
<211> 100
<212> DNA
<213> Escherichia coli K-12 MG1655
<220> b1157 b1158
<222> 259-358
<223> b1157 b1158 U00096 complement(1208342..1208881)
<313> 10266: 288-307
<400> 10266
ATTTTAAGTA TTCAGGGGTA TGCAACGGAG GATCATGCTC ATGGATTACC  50
GTCAAGATCC ACGATTGTGA CTGATGCAAC GATTAATTTC TATTTTGATG  100
<210> 10267
<211> 100
<212> DNA
<213> Escherichia coli K-12 MG1655
<220> hydn b2714
<222> 429-528
<223> hydn b2714 U00096 complement(2835601..2836128)
<313> 10267: 474-493
<400> 10267
TATGGCGGCT TGCCCGACTC ATGCGCTGAT TTGTGTCGAT CGTAATAAAC  50
TTGAACAACT GAGCGCAGAA AAACGCCGCC GTACGGCGCT GATGTTCTAA  100
<210> 10268
<211> 100
<212> DNA
<213> Escherichia coli K-12 MG1655
<220> hydn b2714
<222> 367-466
<223> hydn b2714 U00096 complement(2835601..2836128)
<313> 10268: 426-445
<400> 10268
GCTGACAAAG CCGAAGCCAA TAAATGCGAC CTGTGCAACC ATCGTGAAGA  50
CGGCCCGGCG TGTATGGCGG CTTGCCCGAC TCATGCGCTG ATTTGTGTCG  100
<210> 10269
<211> 100
<212> DNA
<213> Escherichia coli K-12 MG1655
<220> hydn b2714
<222> 340-439
<223> hydn b2714 U00096 complement(2835601..2836128)
<313> 10269: 386-405
<400> 10269

AACAGCGGCG CGGGTCTGAA TGTACGGGCT GACAAAGCCG AAGCCAATAA 50
ATGCGACCTG TGCAACCATC GTGAAGACGG CCCGGCGTGT ATGGCGGCTT 100
<210> 10270
<211> 100
<212> DNA
<213> Escherichia coli K-12 MG1655
<220> cita b0620
<222> 1495-1594
<223> cita b0620 U00096 651458..653116
<313> 10270: 1519-1538
<400> 10270
GAGCCCGGTG AACATGGCAT TGGGTTGTAC TTGATTGCCA GCTACGTAAC 50
GCGCTGCGGT GGTGTTATCA CTCTCGAAGA TAATGATCCC TGCGGTACCT 100
<210> 10271
<211> 100
<212> DNA
<213> Escherichia coli K-12 MG1655
<220> cita b0620
<222> 1345-1444
<223> cita b0620 U00096 651458..653116
<313> 10271: 1384-1403
<400> 10271
CTGCGTAGCG ATGAAGGAAA CAAGATCGTT GAATTATTCC TCAGCGATGA 50
AGGCGATGAT GTGGTGATTG AAGTCGCCGA TCAGGGCTGC GGCGTTCCAG 100
<210> 10272
<211> 100
<212> DNA
<213> Escherichia coli K-12 MG1655
<220> cita b0620
<222> 1271-1370
<223> cita b0620 U00096 651458..653116
<313> 10272: 1313-1332
<400> 10272
TGCCGCCAGG ACTGGATAGC ACCGAGTTTG CAGCCATTGT GGGCAATTTA 50
CTTGATAACG CCTTCGAAGC CAGCCTGCGT AGCGATGAAG GAAACAAGAT 100
<210> 10273
<211> 100
<212> DNA
<213> Escherichia coli K-12 MG1655
<220> citb b0621
<222> 582-681
<223> citb b0621 U00096 653085..653765
<313> 10273: 652-671
<400> 10273
CAGGCGTTAT CTTGAATATT GCGCCAGCCG CCATCTGATT ATTGCTGAAA 50
TTGTTCACGG CAAAGTTGGC AGACCACAAC GCATATACCA CAGTGGGTGA 100
<210> 10274
<211> 100
<212> DNA
<213> Escherichia coli K-12 MG1655
<220> citb b0621
<222> 571-670
<223> citb b0621 U00096 653085..653765
<313> 10274: 607-626
<400> 10274
CGCACCACTG CCAGGCGTTA TCTTGAATAT TGCGCCAGCC GCCATCTGAT 50
TATTGCTGAA ATTGTTCACG GCAAAGTTGG CAGACCACAA CGCATATACC 100
<210> 10275
<211> 100
<212> DNA
<213> Escherichia coli K-12 MG1655
<220> citb b0621
<222> 522-621
<223> citb b0621 U00096 653085..653765
<313> 10275: 578-597
<400> 10275
TGGTGTGCAA CATACGGCAG AAACGGTGGC GCAGGCACTG ACCATCAGCC 50
GCACCACTGC CAGGCGTTAT CTTGAATATT GCGCCAGCCG CCATCTGATT 100
<210> 10276
<211> 100
<212> DNA

<213> Escherichia coli K-12 MG1655
<220> citc b0619
<222> 1047-1146
<223> citc b0619 U00096 complement(650021..651166)
<313> 10276: 1125-1144
<400> 10276
TATCGCGCCG CTGGTCCCTG CAGTCACGCT GCATTATTTG CAGAACCTGC 50
TTGAGCACTC CCGCCAGGAC GCGGCAGCTC GTCAAAAGAC CCCCGCATGA 100
<210> 10277
<211> 100
<212> DNA
<213> Escherichia coli K-12 MG1655
<220> citc b0619
<222> 1011-1110
<223> citc b0619 U00096 complement(650021..651166)
<313> 10277: 1031-1050
<400> 10277
GGTACGTCAA CTGCTGGCGA AAAACGATCT CACGGCTATC GCGCCGCTGG 50
TCCCTGCAGT CACGCTGCAT TATTTGCAGA ACCTGCTTGA GCACTCCCGC 100
<210> 10278
<211> 100
<212> DNA
<213> Escherichia coli K-12 MG1655
<220> citc b0619
<222> 901-1000
<223> citc b0619 U00096 complement(650021..651166)
<313> 10278: 928-947
<400> 10278
AACCAGGATA TGCGCTACTG GCTGGAAACG CCGACTATCT CCGCACCGCC 50
CATCGAACTG GTTGAAATTG AGCGGCTGCG TTACCAGGAG ATGCCGATAT 100
<210> 10279
<211> 100
<212> DNA
<213> Escherichia coli K-12 MG1655
<220> citd b0618
<222> 142-241
<223> citd b0618 U00096 complement(649710..650006)
<313> 10279: 203-222
<400> 10279
ACCATTCTGG ACGTTCTCGC CCGCTACAAC GTGCGCGGCG TACAGCTGAA 50
TGTCGATGAC AAAGGCGCAC TGGACTGCAT TTTACGTGCA CGACTGGAAG 100
<210> 10280
<211> 100
<212> DNA
<213> Escherichia coli K-12 MG1655
<220> citd b0618
<222> 100-199
<223> citd b0618 U00096 complement(649710..650006)
<313> 10280: 134-153
<400> 10280
AATAGCAGCG TTGAGAAACA GTTTGGCGAT GCAATTCGCA CCACCATTCT 50
GGACGTTCTC GCCCGCTACA ACGTGCGCGG CGTACAGCTG AATGTCGATG 100
<210> 10281
<211> 100
<212> DNA
<213> Escherichia coli K-12 MG1655
<220> citd b0618
<222> 37-136
<223> citd b0618 U00096 complement(649710..650006)
<313> 10281: 89-108
<400> 10281
GAGTCTGGGG ATGTGATGAT ACGCATCGCC CCACTCGATA CGCAGGATAT 50
CGACCTGCAA ATCAATAGCA GCGTTGAGAA ACAGTTTGGC GATGCAATTC 100
<210> 10282
<211> 100
<212> DNA
<213> Escherichia coli K-12 MG1655
<220> mrda b0636
<222> 1803-1902
<223> mrda b0636 U00096 complement(665539..667440)
<313> 10282: 1844-1863

<400> 10282
TACACTGATG CGCCAGATCC TCGACCACAT TATGCTGGGT GATAACAACA 50
CCGATCTGCC TGCGGAAAAT CCAGCGGTTG CCGCAGCGGA GGACCATTAA 100
<210> 10283
<211> 100
<212> DNA
<213> Escherichia coli K-12 MG1655
<220> mrda b0636
<222> 1754-1853
<223> mrda b0636 U00096 complement(665539..667440)
<313> 10283: 1820-1839
<400> 10283
TGGCTGTCGC CATGATTCTG GAGAACGGTG GTGCGGGTCC GGCGGTTGGT 50
ACACTGATGC GCCAGATCCT CGACCACATT ATGCTGGGTG ATAACAACAC 100
<210> 10284
<211> 100
<212> DNA
<213> Escherichia coli K-12 MG1655
<220> mrda b0636
<222> 1731-1830
<223> mrda b0636 U00096 complement(665539..667440)
<313> 10284: 1770-1789
<400> 10284
TGCGCCATAC AACAATCCGC AAGTGGCTGT CGCCATGATT CTGGAGAACG 50
GTGGTGCGGG TCCGGCGGTT GGTACACTGA TGCGCCAGAT CCTCGACCAC 100
<210> 10285
<211> 100
<212> DNA
<213> Escherichia coli K-12 MG1655
<220> cite b0617
<222> 825-924
<223> cite b0617 U00096 complement(648805..649728)
<313> 10285: 898-917
<400> 10285
CGTGGTTTCC CTGAACGGCA AGATGGTGGA CGGTCCGGTT ATCGATCGCG 50
CCCGTCTGGT GCTCTCCCGT GCAGAACTTT CCGGCATCCG CGAAGAATAA 100
<210> 10286
<211> 100
<212> DNA
<213> Escherichia coli K-12 MG1655
<220> cite b0617
<222> 786-885
<223> cite b0617 U00096 complement(648805..649728)
<313> 10286: 843-862
<400> 10286
CGTAGAAGCC GCTGAAGCCG CCGCTCGCGA AGGCCTCGGC GTGGTTTCCC 50
TGAACGGCAA GATGGTGGAC GGTCCGGTTA TCGATCGCGC CCGTCTGGTG 100
<210> 10287
<211> 100
<212> DNA
<213> Escherichia coli K-12 MG1655
<220> cite b0617
<222> 733-832
<223> cite b0617 U00096 complement(648805..649728)
<313> 10287: 783-802
<400> 10287
CTGCACAACC TCTACGCACC GACCCAGAAA GAAGTGGATC ACGCCCGCCG 50
CGTCGTAGAA GCCGCTGAAG CCGCCGCTCG CGAAGGCCTC GGCGTGGTTT 100
<210> 10288
<211> 100
<212> DNA
<213> Escherichia coli K-12 MG1655
<220> mrdb b0635
<222> 1014-1113
<223> mrdb b0635 U00096 complement(664424..665536)
<313> 10288: 1094-1113
<400> 10288
GGTCAGTTAT GGAGGATCGG CGCTAATTGT GCTGATGGCT GGGTTCGGGA 50
TTGTAATGTC AATCCACACC CACAGGAAAA TGTTGTCGAA AAGCGTGTAA 100
<210> 10289
<211> 100

<212> DNA
<213> Escherichia coli K-12 MG1655
<220> mrdb b0635
<222> 953-1052
<223> mrdb b0635 U00096 complement(664424..665536)
<313> 10289: 983-1002
<400> 10289
TCTTCGTAAA TATTGGTATG GTAAGCGGTA TTCTGCCGGT TGTAGGGGTT 50
CCGCTCCCAC TGGTCAGTTA TGGAGGATCG GCGCTAATTG TGCTGATGGC 100
<210> 10290
<211> 100
<212> DNA
<213> Escherichia coli K-12 MG1655
<220> mrdb b0635
<222> 827-926
<223> mrdb b0635 U00096 complement(664424..665536)
<313> 10290: 884-903
<400> 10290
GCATTCTGAT TCTGCTCGCT CTCTACATTC TGCTGATCAT GCGCGGGCTG 50
TGGATAGCCG CCAGAGCGCA AACCACCTTT GGTCGCGTCA TGGCTGGCGG 100
<210> 10291
<211> 100
<212> DNA
<213> Escherichia coli K-12 MG1655
<220> citf b0616
<222> 1434-1533
<223> citf b0616 U00096 complement(647262..648794)
<313> 10291: 1503-1522
<400> 10291
CGGTGAACCA CAGCCGATTG AATTCACAGA CCGCGTCGTT GCCGTTGTGC 50
GTTACCGCGA TGGCTCGGTG ATCGATGTTG TGCATCAGGT GAAGGAATAA 100
<210> 10292
<211> 100
<212> DNA
<213> Escherichia coli K-12 MG1655
<220> citf b0616
<222> 1351-1450
<223> citf b0616 U00096 complement(647262..648794)
<313> 10292: 1390-1409
<400> 10292
CCGGAACTGG CAGAACGTCT GCAGGAAGCG GGCATTAAAG TGGTTTCCAT 50
TGAGTGGCTG CGCGAACGTG CGCGTCTGCT GACCGGTGAA CCACAGCCGA 100
<210> 10293
<211> 100
<212> DNA
<213> Escherichia coli K-12 MG1655
<220> citf b0616
<222> 1227-1326
<223> citf b0616 U00096 complement(647262..648794)
<313> 10293: 1286-1305
<400> 10293
CGTCGCGCCG CTGGTACGCG GTCGTATTCC GACTCTGGTG GATAACGTAC 50
TGACCTGCAT CACCCCAGGC TCCAGTGTCG ATATTCTGGT CACAGACCAC 100
<210> 10294
<211> 100
<212> DNA
<213> Escherichia coli K-12 MG1655
<220> citg b0614
<222> 772-871
<223> citg b0614 U00096 complement(645854..646732)
<313> 10294: 817-836
<400> 10294
GATCTCGATT ATCTCCGGCA GTTCGACAGG GAGTGTATCG AACGAAATCT 50
CAGTCCAGGC GGCAGTGCTG ACCTACTGAT CCTTACCTGG TTTTTAGCAC 100
<210> 10295
<211> 100
<212> DNA
<213> Escherichia coli K-12 MG1655
<220> citg b0614
<222> 748-847
<223> citg b0614 U00096 complement(645854..646732)

<313> 10295: 778-797
<400> 10295
AAAGGGGGCA TTCGAACCCC CGCCGATCTC GATTATCTCC GGCAGTTCGA 50
CAGGGAGTGT ATCGAACGAA ATCTCAGTCC AGGCGGCAGT GCTGACCTAC 100
<210> 10296
<211> 100
<212> DNA
<213> Escherichia coli K-12 MG1655
<220> citg b0614
<222> 688-787
<223> citg b0614 U00096 complement(645854..646732)
<313> 10296: 722-741
<400> 10296
TCGCGCGGTG GCGAGGGGGG CCTGCGCTGG CTACAGCGCG AGGCGCAAAC 50
ATTATTGCAA AAAGGGGGCA TTCGAACCCC CGCCGATCTC GATTATCTCC 100
<210> 10297
<211> 100
<212> DNA
<213> Escherichia coli K-12 MG1655
<220> rcsa b1952
<222> 510-609
<223> rcsa b1952 U00096 2021990..2022613
<313> 10297: 564-583
<400> 10297
ATCGCATAAA GGTAATATTA AACGTAAGAT CAAAACGCAT AATAAACAGG 50
TTATCTACCA TGTCGTCCGA CTGACGGATA ATGTGACTAA TGGTATTTTT 100
<210> 10298
<211> 100
<212> DNA
<213> Escherichia coli K-12 MG1655
<220> rcsa b1952
<222> 407-506
<223> rcsa b1952 U00096 2021990..2022613
<313> 10298: 434-453
<400> 10298
TATCATTGAG CCGAACCGAA TCGAGTATGT TGCGAATGTG GATGGCAGGT 50
CAGGGAACCA TTCAAATCTC TGACCAAATG AATATCAAAG CCAAGACCGT 100
<210> 10299
<211> 100
<212> DNA
<213> Escherichia coli K-12 MG1655
<220> rcsa b1952
<222> 291-390
<223> rcsa b1952 U00096 2021990..2022613
<313> 10299: 344-363
<400> 10299
CAGAAAAAAT TTATTGATCA GTTCTAAATC GATTAAACCG GAATCTCTCG 50
ACGATATCCT TGGCGATATT CTGAAAAAAG AGACAACGAT AACCTCGTTT 100
<210> 10300
<211> 100
<212> DNA
<213> Escherichia coli K-12 MG1655
<220> rcsb b2218
<222> 552-651
<223> rcsb b2218 U00096 2314197..2314847
<313> 10300: 618-637
<400> 10300
CCAGAAGAAA TCTGCGATGA TGAAGCTGGG TGTCGAGAAC GATATCGCCC 50
TGCTGAATTA TCTCTCTTCA GTGACCTTAA GTCCGGCAGA TAAAGACTAA 100
<210> 10301
<211> 100
<212> DNA
<213> Escherichia coli K-12 MG1655
<220> rcsb b2218
<222> 515-614
<223> rcsb b2218 U00096 2314197..2314847
<313> 10301: 564-583
<400> 10301
CTAAAAAGCT GAACCGCAGT ATTAAAACCA TCAGTAGCCA GAAGAAATCT 50
GCGATGATGA AGCTGGGTGT CGAGAACGAT ATCGCCCTGC TGAATTATCT 100
<210> 10302

<211> 100
<212> DNA
<213> Escherichia coli K-12 MG1655
<220> rcsb b2218
<222> 472-571
<223> rcsb b2218 U00096 2314197..2314847
<313> 10302: 509-528
<400> 10302
GTTCTGCGCC TGTTTGCGGA AGGCTTCCTG GTGACCGAGA TCGCTAAAAA 50
GCTGAACCGC AGTATTAAAA CCATCAGTAG CCAGAAGAAA TCTGCGATGA 100
<210> 10303
<211> 100
<212> DNA
<213> Escherichia coli K-12 MG1655
<220> rcsc b2219
<222> 2703-2802
<223> rcsc b2219 U00096 complement(2315047..2317848)
<313> 10303: 2767-2786
<400> 10303
CGGTATGGAC AGCTGCCTGT CGAAGCCGGT AACGCTGGAT GTGATAAAAC 50
AGACGCTGAC GTTATATGCC GAGAGGGTCA GGAAATCGCG GGATTCGTAG 100
<210> 10304
<211> 100
<212> DNA
<213> Escherichia coli K-12 MG1655
<220> rcsc b2219
<222> 2677-2776
<223> rcsc b2219 U00096 complement(2315047..2317848)
<313> 10304: 2738-2757
<400> 10304
GAAGAGAAGC AGCGGTGTCT GGAGTCCGGT ATGGACAGCT GCCTGTCGAA 50
GCCGGTAACG CTGGATGTGA TAAAACAGAC GCTGACGTTA TATGCCGAGA 100
<210> 10305
<211> 100
<212> DNA
<213> Escherichia coli K-12 MG1655
<220> rcsc b2219
<222> 2654-2753
<223> rcsc b2219 U00096 complement(2315047..2317848)
<313> 10305: 2686-2705
<400> 10305
GAGTAACTGC TAATGCGTTG GCTGAAGAGA AGCAGCGGTG TCTGGAGTCC 50
GGTATGGACA GCTGCCTGTC GAAGCCGGTA ACGCTGGATG TGATAAAACA 100
<210> 10306
<211> 100
<212> DNA
<213> Escherichia coli K-12 MG1655
<220> b1163 b1164
<222> 1069-1168
<223> b1163 b1164 U00096 complement(1213487..1214698)
<313> 10306: 1109-1128
<400> 10306
ATTCAAGTCA GTGCTATGGG CGTGGCAACA CCAGAAGAGT GGATGTGGCT 50
TGAATCCGCA GGAATTGAGA TGTTTCAGGG AGATCTGTTT GCTAAAGCTA 100
<210> 10307
<211> 100
<212> DNA
<213> Escherichia coli K-12 MG1655
<220> b1163 b1164
<222> 975-1074
<223> b1163 b1164 U00096 complement(1213487..1214698)
<313> 10307: 1020-1039
<400> 10307
TAAAATCAGT CAGGAATTGA TTACCAATGT TCATAAAAGT GGGCCACGGC 50
AGGCAATTAT TCAGGCGATC ATAAAATGCT GTACATCACT TGAAATTCAA 100
<210> 10308
<211> 100
<212> DNA
<213> Escherichia coli K-12 MG1655
<220> b1163 b1164
<222> 893-992

<223> b1163 b1164 U00096 complement(1213487..1214698)
<313> 10308: 938-957
<400> 10308
CTGGTATCAG TGTAGCAATT GATCATTTTG GCGCAGGTTT TGCTGGTTTG 50
TTACTCCTGT CACGCTTCCA GCCTGACAGA ATTAAAATCA GTCAGGAATT 100
<210> 10309
<211> 100
<212> DNA
<213> Escherichia coli K-12 MG1655
<220> rcsf b0197
<222> 306-405
<223> rcsf b0197 U00096 complement(219591..219995)
<313> 10309: 382-401
<400> 10309
TGCTGTATTA CTGCATAGCT GCGAAGTCAC CAGCGGTACG CCAGGCTGCT 50
ATCGTCAGGC TGTATGTATC GGTTCTGCGC TTAACATTAC GGCGAAATGA 100
<210> 10310
<211> 100
<212> DNA
<213> Escherichia coli K-12 MG1655
<220> rcsf b0197
<222> 306-405
<223> rcsf b0197 U00096 complement(219591..219995)
<313> 10310: 336-355
<400> 10310
TGCTGTATTA CTGCATAGCT GCGAAGTCAC CAGCGGTACG CCAGGCTGCT 50
ATCGTCAGGC TGTATGTATC GGTTCTGCGC TTAACATTAC GGCGAAATGA 100
<210> 10311
<211> 100
<212> DNA
<213> Escherichia coli K-12 MG1655
<220> rcsf b0197
<222> 241-340
<223> rcsf b0197 U00096 complement(219591..219995)
<313> 10311: 291-310
<400> 10311
TCTCCGCCGA GCATTCCAAC CGCACGTAAG CGGATGCAAA TCAACGCCTC 50
TAAAATGAAA GCCAATGCTG TATTACTGCA TAGCTGCGAA GTCACCAGCG 100
<210> 10312
<211> 100
<212> DNA
<213> Escherichia coli K-12 MG1655
<220> slp b3507
<222> 412-511
<223> slp b3507 U00096 3651558..3652157
<313> 10312: 464-483
<400> 10312
AATAAAGTCC CGTATAACTT CCTGGAAGTG AATATGCAGG GCATCCAGGT 50
GTGGCATTTG AGAGAAGTGG TTAATACCAC CTATAACCTG TGGGATTACG 100
<210> 10313
<211> 100
<212> DNA
<213> Escherichia coli K-12 MG1655
<220> slp b3507
<222> 342-441
<223> slp b3507 U00096 3651558..3652157
<313> 10313: 386-405
<400> 10313
TCCAGTGAAC TATCGTAATC ACTTTGTTAC CATCCTCGGC ACCATTCAGG 50
GTGAACAACC TGGCTTTATC AATAAAGTCC CGTATAACTT CCTGGAAGTG 100
<210> 10314
<211> 100
<212> DNA
<213> Escherichia coli K-12 MG1655
<220> slp b3507
<222> 287-386
<223> slp b3507 U00096 3651558..3652157
<313> 10314: 339-358
<400> 10314
ATATTGAAGC CAACTATCAG GGCCGACTGC TCGCCAGACA AAGCGGCTTC 50
CTTGATCCAG TGAACTATCG TAATCACTTT GTTACCATCC TCGGCACCAT 100

<210> 10315
<211> 100
<212> DNA
<213> Escherichia coli K-12 MG1655
<220> ymja b1296
<222> 147-246
<223> ymja b1296 U00096 complement(1355447..1355692)
<313> 10315: 211-230
<400> 10315
CAGCGAAGAA GCCCAACATG AAATGGCTGC TGAAGCGGGG ATTAATCCCG  50
TACGCATTGA TGAGATCGCA GAGTTTTTGA ATCAATGGGG TAATGAGTAA  100
<210> 10316
<211> 100
<212> DNA
<213> Escherichia coli K-12 MG1655
<220> ymja b1296
<222> 141-240
<223> ymja b1296 U00096 complement(1355447..1355692)
<313> 10316: 156-175
<400> 10316
AGAGATCAGC GAAGAAGCCC AACATGAAAT GGCTGCTGAA GCGGGGATTA  50
ATCCCGTACG CATTGATGAG ATCGCAGAGT TTTTGAATCA ATGGGGTAAT  100
<210> 10317
<211> 100
<212> DNA
<213> Escherichia coli K-12 MG1655
<220> ymja b1296
<222> 122-221
<223> ymja b1296 U00096 complement(1355447..1355692)
<313> 10317: 162-181
<400> 10317
CCCGTTTGAT GAACAATGAA GAGATCAGCG AAGAAGCCCA ACATGAAATG  50
GCTGCTGAAG CGGGGATTAA TCCCGTACGC ATTGATGAGA TCGCAGAGTT  100
<210> 10318
<211> 100
<212> DNA
<213> Escherichia coli K-12 MG1655
<220> b1168 b1168
<222> 1363-1462
<223> b1168 b1168 U00096 1216509..1218074
<313> 10318: 1429-1448
<400> 10318
ATTCTGGATT GTGTATTGGA TCTGGCGCGT AAACTTTCGA TCAGTATCGT  50
CGCTGAAGGT GTCGAAACGA AAGAACAACT TGACTATCTG AACCAAAATT  100
<210> 10319
<211> 100
<212> DNA
<213> Escherichia coli K-12 MG1655
<220> b1168 b1168
<222> 1283-1382
<223> b1168 b1168 U00096 1216509..1218074
<313> 10319: 1332-1351
<400> 10319
TTCATGACCT GCATATTGAT TATATCAAAA TTGATCACAG TTTCGTTGGC  50
CGCGTAAACG CAGACCCAGA ATCAACCCGA ATTCTGGATT GTGTATTGGA  100
<210> 10320
<211> 100
<212> DNA
<213> Escherichia coli K-12 MG1655
<220> b1168 b1168
<222> 1248-1347
<223> b1168 b1168 U00096 1216509..1218074
<313> 10320: 1276-1295
<400> 10320
TGATTTCGGT ACTGGCTACT CAGGGCTTTC TTATCTTCAT GACCTGCATA  50
TTGATTATAT CAAAATTGAT CACAGTTTCG TTGGCCGCGT AAACGCAGAC  100
<210> 10321
<211> 100
<212> DNA
<213> Escherichia coli K-12 MG1655
<220> b1169 b1169

<222> 1422-1521
<223> b1169 b1169 U00096 1218824..1220344
<313> 10321: 1496-1515
<400> 10321
CTATGTGAAA AATGCTGGAG GAAGCGGCGC GGCCACGCTA AACGGGATCG  50
AACTCATAAC TGTGAATGGC GATGAATCTC CAGCAGATGC CTTCAGATAA  100
<210> 10322
<211> 100
<212> DNA
<213> Escherichia coli K-12 MG1655
<220> b1169 b1169
<222> 1422-1521
<223> b1169 b1169 U00096 1218824..1220344
<313> 10322: 1449-1468
<400> 10322
CTATGTGAAA AATGCTGGAG GAAGCGGCGC GGCCACGCTA AACGGGATCG  50
AACTCATAAC TGTGAATGGC GATGAATCTC CAGCAGATGC CTTCAGATAA  100
<210> 10323
<211> 100
<212> DNA
<213> Escherichia coli K-12 MG1655
<220> b1169 b1169
<222> 1327-1426
<223> b1169 b1169 U00096 1218824..1220344
<313> 10323: 1377-1396
<400> 10323
AATGGTCAGC TTAATATCCG CACCGTATTA GGTGATGATA AATCGGCTAC  50
GGACAGACTT ATTGTTGAGG GTAATACTTC GGGTTCAACT ACCGTCTATG  100
<210> 10324
<211> 100
<212> DNA
<213> Escherichia coli K-12 MG1655
<220> slt b4393
<222> 1838-1937
<223> slt b4393 U00096 4628275..4630239
<313> 10324: 1890-1909
<400> 10324
AGAGTATTCC ATTCTCCGAG ACGCGCGGTT ATGTGAAGAA CGTGCTGGCT  50
TATGACGCTT ACTACCGCTA TTTCATGGGG GATAAACCGA CGTTGATGAG  100
<210> 10325
<211> 100
<212> DNA
<213> Escherichia coli K-12 MG1655
<220> slt b4393
<222> 1787-1886
<223> slt b4393 U00096 4628275..4630239
<313> 10325: 1808-1827
<400> 10325
GAACCTGGCT TGGCAACAGC GCCGGGCGTA TCGACGCAGT GGCATTTGTC  50
GAGAGTATTC CATTCTCCGA GACGCGCGGT TATGTGAAGA ACGTGCTGGC  100
<210> 10326
<211> 100
<212> DNA
<213> Escherichia coli K-12 MG1655
<220> slt b4393
<222> 1734-1833
<223> slt b4393 U00096 4628275..4630239
<313> 10326: 1770-1789
<400> 10326
CAATAATCGT ATTTTCTCCT CAGCAGCTTA TAACGCCGGA CCAGGGCGGG  50
TGCGAACCTG GCTTGGCAAC AGCGCCGGGC GTATCGACGC AGTGGCATTT  100
<210> 10327
<211> 100
<212> DNA
<213> Escherichia coli K-12 MG1655
<220> mreb b3252
<222> 1005-1104
<223> mreb b3252 U00096 complement(3397681..3398784)
<313> 10327: 1073-1092
<400> 10327
CGTTGTTGCT GAAGACCCGC TGACCTGTGT GGCGCGCGGT GGCGGCAAAG  50

CGCTGGAAAT GATCGACATG CACGGCGGCG ACCTGTTCAG CGAAGAGTAA 100
<210> 10328
<211> 100
<212> DNA
<213> Escherichia coli K-12 MG1655
<220> mreb b3252
<222> 949-1048
<223> mreb b3252 U00096 complement(3397681..3398784)
<313> 10328: 1003-1022
<400> 10328
GGCGCACTGC TGCGTAACCT TGACCGTTTG TTAATGGAAG AAACCGGCAT 50
TCCAGTCGTT GTTGCTGAAG ACCCGCTGAC CTGTGTGGCG CGCGGTGGCG 100
<210> 10329
<211> 100
<212> DNA
<213> Escherichia coli K-12 MG1655
<220> mreb b3252
<222> 860-959
<223> mreb b3252 U00096 complement(3397681..3398784)
<313> 10329: 902-921
<400> 10329
TTGTGAGCGC GGTAATGGTT GCACTGGAAC AGTGCCCGCC GGAACTGGCT 50
TCCGACATCT CCGAGCGCGG CATGGTGCTC ACCGGTGGTG GCGCACTGCT 100
<210> 10330
<211> 100
<212> DNA
<213> Escherichia coli K-12 MG1655
<220> noha b1549
<222> 471-570
<223> noha b1549 U00096 complement(1633822..1634391)
<313> 10330: 526-545
<400> 10330
AGCCATGAAC AAAGCAGCCG CGCTGGATGA ACTGATACCG GGGTTGCTGA 50
GTGAATATAA CCGCGCTGAC AGACAATACG CAGGGGGCAG CAGGTCTTGA 100
<210> 10331
<211> 100
<212> DNA
<213> Escherichia coli K-12 MG1655
<220> noha b1549
<222> 242-341
<223> noha b1549 U00096 complement(1633822..1634391)
<313> 10331: 291-310
<400> 10331
CTATTGAGTA CGAACGCCAT CGACTTACGC GTGCGCAGGC CGACGCACAG 50
GAGCTGAAAA ATGCCAGAGA CTCCGCTGAA GTGGTGGAAA CCGCATTCTG 100
<210> 10332
<211> 100
<212> DNA
<213> Escherichia coli K-12 MG1655
<220> noha b1549
<222> 161-260
<223> noha b1549 U00096 complement(1633822..1634391)
<313> 10332: 225-244
<400> 10332
ATGCTGAAAT TGAGAACGAA AAGCTGCGCC GGGAAGTTGA AGAACTGCGG 50
CAGGCCAGCG AGACAGATCT CCAGCCAGGG ACTATTGAGT ACGAACGCCA 100
<210> 10333
<211> 100
<212> DNA
<213> Escherichia coli K-12 MG1655
<220> mrec b3251
<222> 1005-1104
<223> mrec b3251 U00096 complement(3396512..3397615)
<313> 10333: 1055-1074
<400> 10333
TGCTGCGCCG ACACAGCCTG CTGCTAATCG CTCTCCACAA AGGGCTACGC 50
CGCCGCAAAG TGGTGCTCAA CCGCCTGCGC GTGCGCCGGG AGGGCAATAG 100
<210> 10334
<211> 100
<212> DNA
<213> Escherichia coli K-12 MG1655

<220> mrec b3251
<222> 967-1066
<223> mrec b3251 U00096 complement(3396512..3397615)
<313> 10334: 1013-1032
<400> 10334
CCGCAGCAAC CGGCGACAGG AAATGCAGCT ACTGCGCCTG CTGCGCCGAC 50
ACAGCCTGCT GCTAATCGCT CTCCACAAAG GGCTACGCCG CCGCAAAGTG 100
<210> 10335
<211> 100
<212> DNA
<213> Escherichia coli K-12 MG1655
<220> mrec b3251
<222> 930-1029
<223> mrec b3251 U00096 complement(3396512..3397615)
<313> 10335: 977-996
<400> 10335
GTTACCTGAA CCGGCAACGG GGATCGCTCA GCCGACTCCG CAGCAACCGG 50
CGACAGGAAA TGCAGCTACT GCGCCTGCTG CGCCGACACA GCCTGCTGCT 100
<210> 10336
<211> 100
<212> DNA
<213> Escherichia coli K-12 MG1655
<220> nohb b0561
<222> 447-546
<223> nohb b0561 U00096 580057..580602
<313> 10336: 510-529
<400> 10336
TTTCCTGAAA CGGGATATCA TCAAAGCCAT GAACAAAGCA GCCGCGCTGG 50
ATGAACTGAT ACCGGGGTTG CTGAGTGAAT ATATCGAACA GTCAGGTTAA 100
<210> 10337
<211> 100
<212> DNA
<213> Escherichia coli K-12 MG1655
<220> nohb b0561
<222> 262-361
<223> nohb b0561 U00096 580057..580602
<313> 10337: 287-306
<400> 10337
CGACTTACGC GTGCGCAGGC CGATGCACAG GAGCTGAAAA ATGCCAGAGA 50
CTCCGCTGAA GTGGTGGAAA CCGCATTCTG TACTTTCGTG CTGTCGCGGA 100
<210> 10338
<211> 100
<212> DNA
<213> Escherichia coli K-12 MG1655
<220> nohb b0561
<222> 162-261
<223> nohb b0561 U00096 580057..580602
<313> 10338: 210-229
<400> 10338
TGCTGAAATT GAGAACGAAA AGCTGCGCCG GGAGGTTGAA GAACTGCTGC 50
AGGCCAGCGA GACAGATCTC CAGCCAGGGA CTATTGAGTA CGAACGCCAT 100
<210> 10339
<211> 100
<212> DNA
<213> Escherichia coli K-12 MG1655
<220> smg b3285
<222> 375-474
<223> smg b3285 U00096 complement(3429629..3430102)
<313> 10339: 414-433
<400> 10339
GGTGATCCTG ATGGTGTTGT TCAATATTCC GGGCTGCGAA AATGCGTACC 50
AGCAAATGGA AGAATTACTC TTTGAAGTGA ATGAAGGTAT GCTGCATTAA 100
<210> 10340
<211> 100
<212> DNA
<213> Escherichia coli K-12 MG1655
<220> smg b3285
<222> 343-442
<223> smg b3285 U00096 complement(3429629..3430102)
<313> 10340: 369-388
<400> 10340

AACGCAGAGT TCGAACTGGA TGATCTGAAA TGGGTGATCC TGATGGTGTT 50
GTTCAATATT CCGGGCTGCG AAAATGCGTA CCAGCAAATG GAAGAATTAC 100
<210> 10341
<211> 100
<212> DNA
<213> Escherichia coli K-12 MG1655
<220> smg b3285
<222> 270-369
<223> smg b3285 U00096 complement(3429629..3430102)
<313> 10341: 298-317
<400> 10341
TTTCCTTGAG CAGATTCAGG TGCTCAACCT TGAAACTCGT GAAATGGTGA 50
TAGAGCGAGT GCTCGCGCTG GATAACGCAG AGTTCGAACT GGATGATCTG 100
<210> 10342
<211> 100
<212> DNA
<213> Escherichia coli K-12 MG1655
<220> hyfa b2482
<222> 548-647
<223> hyfa b2482 U00096 2599182..2599838
<313> 10342: 569-588
<400> 10342
TCACCGGTGA TAGCCTGCAA CGTCAGATGA AAGAAAAACA GCGCCTTGCC 50
GCAAGCTGGT TTGCCAATGG CGGGGAGGAT CCCCTTTCCC TCACTCAGGA 100
<210> 10343
<211> 100
<212> DNA
<213> Escherichia coli K-12 MG1655
<220> hyfa b2482
<222> 460-559
<223> hyfa b2482 U00096 2599182..2599838
<313> 10343: 512-531
<400> 10343
ACCGTCGCGG TGAAATGCGA CCTGTGTGAT TTCTTGCCAG AAGGTCCGGC 50
CTGCGTTCGC GCTTGCCCGA ATCAGGCGTT ACGGCTGATC ACCGGTGATA 100
<210> 10344
<211> 100
<212> DNA
<213> Escherichia coli K-12 MG1655
<220> hyfa b2482
<222> 372-471
<223> hyfa b2482 U00096 2599182..2599838
<313> 10344: 430-449
<400> 10344
TGTTTTTCAG GCTGAAGGCT CACTCAAAGA CGGCGAAGAA AACGCGCCAA 50
CACAACATGC TTTGCTGCGC TGGGAACCTG GTGTCCAGAC CGTCGCGGTG 100
<210> 10345
<211> 100
<212> DNA
<213> Escherichia coli K-12 MG1655
<220> mred b3250
<222> 272-371
<223> mred b3250 U00096 complement(3396024..3396512)
<313> 10345: 299-318
<400> 10345
AATACCAGCT TTTCCGCAAC CTCGCATTAT GGCAGCAGGC GCTGGTCGTC 50
ATGTTGCTTT CGCTGGTGGT GGATATTATT GTTTTCTGGG CAGAGTTTTT 100
<210> 10346
<211> 100
<212> DNA
<213> Escherichia coli K-12 MG1655
<220> mred b3250
<222> 123-222
<223> mred b3250 U00096 complement(3396024..3396512)
<313> 10346: 168-187
<400> 10346
CATCTTGTTG TATTGGATCC TGGCCTTGCC TCATCGCGTA AATGTGGGCA 50
CAGGTTTTGT GATGGGTGCC ATACTGGATC TGATCAGCGG CTCGACGCTT 100
<210> 10347
<211> 100
<212> DNA

<213> Escherichia coli K-12 MG1655
<220> mred b3250
<222> 52-151
<223> mred b3250 U00096 complement(3396024..3396512)
<313> 10347: 119-138
<400> 10347
ATTGCGCTGT TGCTGCAAAT CATGCCCTGG CCGGATAACC TGATTGTTTT 50
CCGGCCAAAC TGGGTGTTAC TCATCTTGTT GTATTGGATC CTGGCCTTGC 100
<210> 10348
<211> 100
<212> DNA
<213> Escherichia coli K-12 MG1655
<220> hyfb b2483
<222> 1920-2019
<223> hyfb b2483 U00096 2599838..2601856
<313> 10348: 2000-2019
<400> 10348
GGCCAAAGAA ATACAGCATC TGCAAAGCGG CGACTTTCGT CTCTATTGCC 50
TGTATGTGGT CGCCGCACTG GTTGTGCTGC TAATCGCTAT TGCCGTCTAA 100
<210> 10349
<211> 100
<212> DNA
<213> Escherichia coli K-12 MG1655
<220> hyfb b2483
<222> 1908-2007
<223> hyfb b2483 U00096 2599838..2601856
<313> 10349: 1943-1962
<400> 10349
CACCCAACGG CTGGCCAAAG AAATACAGCA TCTGCAAAGC GGCGACTTTC 50
GTCTCTATTG CCTGTATGTG GTCGCCGCAC TGGTTGTGCT GCTAATCGCT 100
<210> 10350
<211> 100
<212> DNA
<213> Escherichia coli K-12 MG1655
<220> hyfb b2483
<222> 1866-1965
<223> hyfb b2483 U00096 2599838..2601856
<313> 10350: 1899-1918
<400> 10350
ACCCTTCTGG GATGAGCGGG TGATCCGCCC CATCGTGAGC GCCACCCAAC 50
GGCTGGCCAA AGAAATACAG CATCTGCAAA GCGGCGACTT TCGTCTCTAT 100
<210> 10351
<211> 100
<212> DNA
<213> Escherichia coli K-12 MG1655
<220> hyfc b2484
<222> 870-969
<223> hyfc b2484 U00096 2601846..2602814
<313> 10351: 938-957
<400> 10351
CACGCTGGCA CGCGGGCGTT TTTTACTCAT TCACCATGTG ACCTGGCTTG 50
GCTTCAGCCT TGCTGCGCTT GCATGGGTCT TCTGGTTAAC CGGTCTGTAA 100
<210> 10352
<211> 100
<212> DNA
<213> Escherichia coli K-12 MG1655
<220> hyfc b2484
<222> 870-969
<223> hyfc b2484 U00096 2601846..2602814
<313> 10352: 908-927
<400> 10352
CACGCTGGCA CGCGGGCGTT TTTTACTCAT TCACCATGTG ACCTGGCTTG 50
GCTTCAGCCT TGCTGCGCTT GCATGGGTCT TCTGGTTAAC CGGTCTGTAA 100
<210> 10353
<211> 100
<212> DNA
<213> Escherichia coli K-12 MG1655
<220> hyfc b2484
<222> 870-969
<223> hyfc b2484 U00096 2601846..2602814
<313> 10353: 912-931

<400> 10353
CACGCTGGCA CGCGGGCGTT TTTTACTCAT TCACCATGTG ACCTGGCTTG 50
GCTTCAGCCT TGCTGCGCTT GCATGGGTCT TCTGGTTAAC CGGTCTGTAA 100
<210> 10354
<211> 100
<212> DNA
<213> Escherichia coli K-12 MG1655
<220> b1170 b1170
<222> 918-1017
<223> b1170 b1170 U00096 1220429..1221445
<313> 10354: 961-980
<400> 10354
AATTAAACTT GGCGTAAATG GCAACCTGAA TCCAGCGGCC AGCGTCTGGG 50
GAATGTGGGC GTGCAGCTGG GTGATAATGG CTACAATGAC ACCGCAGTGA 100
<210> 10355
<211> 100
<212> DNA
<213> Escherichia coli K-12 MG1655
<220> b1170 b1170
<222> 852-951
<223> b1170 b1170 U00096 1220429..1221445
<313> 10355: 902-921
<400> 10355
TAAAGATTTT GCTGTCTCAA TGAACGGTGT GAAAGTCGAA CAAGATGGGG 50
CGAGTAATTT GGGGGAAATT AAACTTGGCG TAAATGGCAA CCTGAATCCA 100
<210> 10356
<211> 100
<212> DNA
<213> Escherichia coli K-12 MG1655
<220> b1170 b1170
<222> 764-863
<223> b1170 b1170 U00096 1220429..1221445
<313> 10356: 814-833
<400> 10356
GCACCTTTAT TCGTACTCAG GAGAAAAACA GCGGTCCGCA CGGTGACGAC 50
TTTGAACCTT TTGTTGAAAT GAACTGGATC CATAACAGTA AAGATTTTGC 100
<210> 10357
<211> 100
<212> DNA
<213> Escherichia coli K-12 MG1655
<220> hyfd b2485
<222> 1289-1388
<223> hyfd b2485 U00096 2602831..2604270
<313> 10357: 1354-1373
<400> 10357
TTATTCGCTG GTTTGGTCGC GTTGTGCCTG GCAAACCGAG CGAGGCCGTC 50
GCCGATGCCG CACCGCTGCC AGGATCAATG CGCCTGGTGT TGATTGTACT 100
<210> 10358
<211> 100
<212> DNA
<213> Escherichia coli K-12 MG1655
<220> hyfd b2485
<222> 1265-1364
<223> hyfd b2485 U00096 2602831..2604270
<313> 10358: 1285-1304
<400> 10358
AATCGGTCGC CAGTTTCGCC TGGTTTATTC GCTGGTTTGG TCGCGTTGTG 50
CCTGGCAAAC CGAGCGAGGC CGTCGCCGAT GCCGCACCGC TGCCAGGATC 100
<210> 10359
<211> 100
<212> DNA
<213> Escherichia coli K-12 MG1655
<220> hyfd b2485
<222> 1218-1317
<223> hyfd b2485 U00096 2602831..2604270
<313> 10359: 1244-1263
<400> 10359
AGTGGAGTAC TGGATCCTGC TGCCCGCCAT GATTCTTCTG ATGATTGAAT 50
CGGTCGCCAG TTTCGCCTGG TTTATTCGCT GGTTTGGTCG CGTTGTGCCT 100
<210> 10360
<211> 100

<212> DNA
<213> Escherichia coli K-12 MG1655
<220> b1171 b1171
<222> 237-336
<223> b1171 b1171 U00096 complement(1221528..1221863)
<313> 10360: 279-298
<400> 10360
CAAAGTGGTA GAAAAACTGA ATCAGGTTTG CGCTAAAGAC CCGCAAATGC 50
TGTTGATCAC GGCAATTGAT GACACCATGC GTGCTATCGG CAAAAAATAA 100
<210> 10361
<211> 100
<212> DNA
<213> Escherichia coli K-12 MG1655
<220> b1171 b1171
<222> 205-304
<223> b1171 b1171 U00096 complement(1221528..1221863)
<313> 10361: 249-268
<400> 10361
TTAAAAATTG AGCAATCTCC GGAACTGAGC GCCAAAGTGG TAGAAAAACT 50
GAATCAGGTT TGCGCTAAAG ACCCGCAAAT GCTGTTGATC ACGGCAATTG 100
<210> 10362
<211> 100
<212> DNA
<213> Escherichia coli K-12 MG1655
<220> b1171 b1171
<222> 92-191
<223> b1171 b1171 U00096 complement(1221528..1221863)
<313> 10362: 133-152
<400> 10362
CGTCAGAAAC CTATATTACT TGCGCAGAAG CCAACGAAAT GGCAAAAACT 50
GACAGCGCAC AGGTTGCAGA AATTGTTGCG GTAATGGGTA ATGCCAGCGT 100
<210> 10363
<211> 100
<212> DNA
<213> Escherichia coli K-12 MG1655
<220> hyfe b2486
<222> 552-651
<223> hyfe b2486 U00096 2604282..2604932
<313> 10363: 619-638
<400> 10363
CGCCATCTTC GCCGTCATTG TGATGGTGTT ACTGGCAAGA AAAATATGGC 50
GTACCCACGG CACGCTGGAC GTGAACAACT TGACCGCGCT GAAGGGATAA 100
<210> 10364
<211> 100
<212> DNA
<213> Escherichia coli K-12 MG1655
<220> hyfe b2486
<222> 536-635
<223> hyfe b2486 U00096 2604282..2604932
<313> 10364: 576-595
<400> 10364
TAGGTATCGC TACCGACGCC ATCTTCGCCG TCATTGTGAT GGTGTTACTG 50
GCAAGAAAAA TATGGCGTAC CCACGGCACG CTGGACGTGA ACAACTTGAC 100
<210> 10365
<211> 100
<212> DNA
<213> Escherichia coli K-12 MG1655
<220> hyfe b2486
<222> 513-612
<223> hyfe b2486 U00096 2604282..2604932
<313> 10365: 545-564
<400> 10365
GCGAGCACCG GAACTGGTGG AAATAGGTAT CGCTACCGAC GCCATCTTCG 50
CCGTCATTGT GATGGTGTTA CTGGCAAGAA AAATATGGCG TACCCACGGC 100
<210> 10366
<211> 100
<212> DNA
<213> Escherichia coli K-12 MG1655
<220> soxr b4064
<222> 269-368
<223> soxr b4064 U00096 4275048..4275512

<313> 10366: 298-317
<400> 10366
AGTGGAAACA GCTTTCGTCC CAATGGCGAG AAGAGTTGGA TCGGCGCATT 50
CATACCTTAG TGGCGCTGCG TGACGAACTG GACGGATGTA TTGGTTGTGG 100
<210> 10367
<211> 100
<212> DNA
<213> Escherichia coli K-12 MG1655
<220> soxr b4064
<222> 212-311
<223> soxr b4064 U00096 4275048..4275512
<313> 10367: 262-281
<400> 10367
CGACCATTGG TGAAGCGTTT GGCGTGTTGC CCGAAGGGCA TACGTTAAGT 50
GCGAAAGAGT GGAAACAGCT TTCGTCCCAA TGGCGAGAAG AGTTGGATCG 100
<210> 10368
<211> 100
<212> DNA
<213> Escherichia coli K-12 MG1655
<220> soxr b4064
<222> 105-204
<223> soxr b4064 U00096 4275048..4275512
<313> 10368: 122-141
<400> 10368
GTTGATTACC AGTATCCGTA ACAGCGGCAA TCAGCGGCGA TATAAACGTG 50
ATGTGTTGCG ATATGTTGCA ATTATCAAAA TTGCTCAGCG TATTGGCATT 100
<210> 10369
<211> 100
<212> DNA
<213> Escherichia coli K-12 MG1655
<220> b1172 b1172
<222> 186-285
<223> b1172 b1172 U00096 complement(1221867..1222151)
<313> 10369: 250-269
<400> 10369
TGCTGTCACT GGCGTTCTGA CCGGCAATGG CCTCGAAGGG GCGATTAAAG 50
GTGCCGTGAT TGGCGGTACG GGTGGTGCAA TTCTGGGTAA AATGAAATAA 100
<210> 10370
<211> 100
<212> DNA
<213> Escherichia coli K-12 MG1655
<220> b1172 b1172
<222> 178-277
<223> b1172 b1172 U00096 complement(1221867..1222151)
<313> 10370: 211-230
<400> 10370
GCCGTTGGTG CTGTCACTGG CGTTCTGACC GGCAATGGCC TCGAAGGGGC 50
GATTAAAGGT GCCGTGATTG GCGGTACGGG TGGTGCAATT CTGGGTAAAA 100
<210> 10371
<211> 100
<212> DNA
<213> Escherichia coli K-12 MG1655
<220> b1172 b1172
<222> 140-239
<223> b1172 b1172 U00096 complement(1221867..1222151)
<313> 10371: 171-190
<400> 10371
ACGGTAAAAA TGCGCGTAAA GGTGCAAAAG TGGGTGCCGC CGTTGGTGCT 50
GTCACTGGCG TTCTGACCGG CAATGGCCTC GAAGGGGCGA TTAAAGGTGC 100
<210> 10372
<211> 100
<212> DNA
<213> Escherichia coli K-12 MG1655
<220> hyff b2487
<222> 1482-1581
<223> hyff b2487 U00096 2604937..2606517
<313> 10372: 1557-1576
<400> 10372
CCTCTCAGGG ACGCACGATC TGCCTGCACA ACGTAGCACC TGGCATGATT 50
TTTTGCCTTC AGGCACCGCA TCTGTTTCGG AGAAACACAG TGAACGTTAA 100
<210> 10373

<211> 100
<212> DNA
<213> Escherichia coli K-12 MG1655
<220> hyff b2487
<222> 1465-1564
<223> hyff b2487 U00096 2604937..2606517
<313> 10373: 1515-1534
<400> 10373
GGCGCTTCCA CTATAGTCCT CTCAGGGACG CACGATCTGC CTGCACAACG 50
TAGCACCTGG CATGATTTTT TGCCTTCAGG CACCGCATCT GTTTCGGAGA 100
<210> 10374
<211> 100
<212> DNA
<213> Escherichia coli K-12 MG1655
<220> hyff b2487
<222> 1395-1494
<223> hyff b2487 U00096 2604937..2606517
<313> 10374: 1433-1452
<400> 10374
GGTGATTCTG CTGGTCATGA TGCTGGCGAT GGGAACGCAT ATTCCACAAC 50
CTGTCATCAG GATCCTGGCG GGCGCTTCCA CTATAGTCCT CTCAGGGACG 100
<210> 10375
<211> 100
<212> DNA
<213> Escherichia coli K-12 MG1655
<220> soxs b4063
<222> 225-324
<223> soxs b4063 U00096 complement(4274639..4274962)
<313> 10375: 280-299
<400> 10375
TATCGCAATG GACCTGGGTT ATGTCTCGCA GCAGACCTTC TCCCGCGTTT 50
TCCGTCGGCA GTTTGATCGC ACTCCCAGCG ATTATCGCCA CCGCCTGTAA 100
<210> 10376
<211> 100
<212> DNA
<213> Escherichia coli K-12 MG1655
<220> soxs b4063
<222> 119-218
<223> soxs b4063 U00096 complement(4274639..4274962)
<313> 10376: 175-194
<400> 10376
GAATGTTCCG CACGGTGACG CATCAGACGC TTGGCGATTA CATTCGCCAA 50
CGCCGCCTGT TACTGGCCGC CGTTGAGTTG CGCACCACCG AGCGTCCGAT 100
<210> 10377
<211> 100
<212> DNA
<213> Escherichia coli K-12 MG1655
<220> soxs b4063
<222> 92-191
<223> soxs b4063 U00096 complement(4274639..4274962)
<313> 10377: 132-151
<400> 10377
CAGGCTATTC AAAGTGGTAC TTGCAACGAA TGTTCCGCAC GGTGACGCAT 50
CAGACGCTTG GCGATTACAT TCGCCAACGC CGCCTGTTAC TGGCCGCCGT 100
<210> 10378
<211> 100
<212> DNA
<213> Escherichia coli K-12 MG1655
<220> b1173 b1174
<222> 114-213
<223> b1173 b1174 U00096 1222918..1223130
<313> 10378: 180-199
<400> 10378
CAGCAGTACG TTTAGCGGAC ATCTCGGGGT GGGGTATAGC CATGGTGCCG 50
GTGTGGAATC CCCGTGGAAC GCGGTGGCTG GTGTGAACTG GTCGTTCTGA 100
<210> 10379
<211> 100
<212> DNA
<213> Escherichia coli K-12 MG1655
<220> b1173 b1174
<222> 52-151

<223> b1173 b1174 U00096 1222918..1223130
<313> 10379: 111-130
<400> 10379
AATTTCGTCA ATGATTTGTC GGGCAGACGT GGAATATACC AGGCAGCTAT 50
TAAAGCCTCA TTCAGCAGTA CGTTTAGCGG ACATCTCGGG GTGGGGTATA 100
<210> 10380
<211> 100
<212> DNA
<213> Escherichia coli K-12 MG1655
<220> b1173 b1174
<222> 42-141
<223> b1173 b1174 U00096 1222918..1223130
<313> 10380: 71-90
<400> 10380
TAATGACGGT AATTTCGTCA ATGATTTGTC GGGCAGACGT GGAATATACC 50
AGGCAGCTAT TAAAGCCTCA TTCAGCAGTA CGTTTAGCGG ACATCTCGGG 100
<210> 10381
<211> 100
<212> DNA
<213> Escherichia coli K-12 MG1655
<220> hyfg b2488
<222> 1569-1668
<223> hyfg b2488 U00096 2606507..2608174
<313> 10381: 1646-1665
<400> 10381
TGTGACGCTG GTAGATGTGC GCAAGCGCCA GTCAAAAACC GTGCCGTATA 50
AAGAGATCGA ACGCTACGGC ATTGATCGTA ACCGTTCGCC GCTGAAGTAA 100
<210> 10382
<211> 100
<212> DNA
<213> Escherichia coli K-12 MG1655
<220> hyfg b2488
<222> 1551-1650
<223> hyfg b2488 U00096 2606507..2608174
<313> 10382: 1594-1613
<400> 10382
CTACTCCTGT ACCGACCGTG TGACGCTGGT AGATGTGCGC AAGCGCCAGT 50
CAAAAACCGT GCCGTATAAA GAGATCGAAC GCTACGGCAT TGATCGTAAC 100
<210> 10383
<211> 100
<212> DNA
<213> Escherichia coli K-12 MG1655
<220> hyfg b2488
<222> 1528-1627
<223> hyfg b2488 U00096 2606507..2608174
<313> 10383: 1552-1571
<400> 10383
ATTATCGGTA GCCTTGATCC CTGCTACTCC TGTACCGACC GTGTGACGCT 50
GGTAGATGTG CGCAAGCGCC AGTCAAAAAC CGTGCCGTAT AAAGAGATCG 100
<210> 10384
<211> 100
<212> DNA
<213> Escherichia coli K-12 MG1655
<220> hyfh b2489
<222> 364-463
<223> hyfh b2489 U00096 2608184..2608729
<313> 10384: 416-435
<400> 10384
CGCCCGTTTG CCCCGCAAAA AACCATCGCA CTGGCTGCTG AATTGTTAGC 50
ACAGCAACAA AATGCGCCAC AAAACCGCGA AATGTTGTGG GCGCAAGCGA 100
<210> 10385
<211> 100
<212> DNA
<213> Escherichia coli K-12 MG1655
<220> hyfh b2489
<222> 359-458
<223> hyfh b2489 U00096 2608184..2608729
<313> 10385: 382-401
<400> 10385
GCGAACGCCC GTTTGCCCCG CAAAAAACCA TCGCACTGGC TGCTGAATTG 50
TTAGCACAGC AACAAAATGC GCCACAAAAC CGCGAAATGT TGTGGGCGCA 100

<210> 10386
<211> 100
<212> DNA
<213> Escherichia coli K-12 MG1655
<220> hyfh b2489
<222> 276-375
<223> hyfh b2489 U00096 2608184..2608729
<313> 10386: 300-319
<400> 10386
TACCAATAAC TTTGAACTGA CCGTCACCAA TAAAGCCGAT CTCTATACCC 50
GCGCGACGTT CCATCTACAA CGTTGCAGCC GTTGCGAACG CCCGTTTGCC 100
<210> 10387
<211> 100
<212> DNA
<213> Escherichia coli K-12 MG1655
<220> hyfi hyfI
<222> 654-753
<223> hyfi hyfI U00096 2608726..2609484
<313> 10387: 699-718
<400> 10387
TCCTACAGGA AATCGGGTTA ACACCTGGTT GCGCGATGCC GACGATCCAC 50
GTCTCAATAG TATCGTTCAG CAACTCTTTC GCGTACTCCG GGGGTTACAT 100
<210> 10388
<211> 100
<212> DNA
<213> Escherichia coli K-12 MG1655
<220> hyfi hyfI
<222> 614-713
<223> hyfi hyfI U00096 2608726..2609484
<313> 10388: 641-660
<400> 10388
GAGAAATTTG CGATCGGCTC CTGCGCCATT TAAGCGACGA TCCTACAGGA 50
AATCGGGTTA ACACCTGGTT GCGCGATGCC GACGATCCAC GTCTCAATAG 100
<210> 10389
<211> 100
<212> DNA
<213> Escherichia coli K-12 MG1655
<220> hyfi hyfI
<222> 513-612
<223> hyfi hyfI U00096 2608726..2609484
<313> 10389: 559-578
<400> 10389
CACCGGGGTG ACTATGCAAC CGTTGTGGCC GCAGATCCCG CCATCACAGC 50
GTATCGCCAT TGAGCGAGAA GCGCGGCGGC TGGCGGGCTA TCGTCAGGGG 100
<210> 10390
<211> 100
<212> DNA
<213> Escherichia coli K-12 MG1655
<220> smp b4388
<222> 529-628
<223> smp b4388 U00096 complement(4621714..4622358)
<313> 10390: 580-599
<400> 10390
GTAGTGCTGA CCCGCACGCT GCTACAGGGT AAACGCACCC GCTGGCAGCA 50
ATCGCCCTTC CTGTTAACCG CCAGCAAACC GGTGCCGGAA GAGGAAGAAA 100
<210> 10391
<211> 100
<212> DNA
<213> Escherichia coli K-12 MG1655
<220> smp b4388
<222> 505-604
<223> smp b4388 U00096 complement(4621714..4622358)
<313> 10391: 549-568
<400> 10391
TTGCTGCTCT CACTGGCAAT CGGTGTAGTG CTGACCCGCA CGCTGCTACA 50
GGGTAAACGC ACCCGCTGGC AGCAATCGCC CTTCCTGTTA ACCGCCAGCA 100
<210> 10392
<211> 100
<212> DNA
<213> Escherichia coli K-12 MG1655
<220> smp b4388

<222> 476-575
<223> smp b4388 U00096 complement(4621714..4622358)
<313> 10392: 499-518
<400> 10392
ATAACACCAC TAACATTTTA CGCCTGATGT TGCTGCTCTC ACTGGCAATC 50
GGTGTAGTGC TGACCCGCAC GCTGCTACAG GGTAAACGCA CCCGCTGGCA 100
<210> 10393
<211> 100
<212> DNA
<213> Escherichia coli K-12 MG1655
<220> sms b4390
<222> 1283-1382
<223> sms b4390 U00096 4623481..4624863
<313> 10393: 1308-1327
<400> 10393
TTGTTCCGGC GGCTAACGTG CCGAAAAAAG CGCCGGAAGG GATGCAGATT 50
TTTGGCGTTA AAAAACTCTC CGACGCGCTT AGCGTGTTCG ACGACTTATA 100
<210> 10394
<211> 100
<212> DNA
<213> Escherichia coli K-12 MG1655
<220> sms b4390
<222> 1201-1300
<223> sms b4390 U00096 4623481..4624863
<313> 10394: 1233-1252
<400> 10394
GGGCTGGCAG GGGAGATCCG CCCGGTGCCC AGCGGTCAGG AACGAATCTC 50
TGAAGCGGCG AAACACGGTT TTCGCCGGGC GATTGTTCCG GCGGCTAACG 100
<210> 10395
<211> 100
<212> DNA
<213> Escherichia coli K-12 MG1655
<220> sms b4390
<222> 1163-1262
<223> sms b4390 U00096 4623481..4624863
<313> 10395: 1181-1200
<400> 10395
GACCGCTGCC GCAGGATCTG GTGGTGTTTG GTGAAGTCGG GCTGGCAGGG 50
GAGATCCGCC CGGTGCCCAG CGGTCAGGAA CGAATCTCTG AAGCGGCGAA 100
<210> 10396
<211> 100
<212> DNA
<213> Escherichia coli K-12 MG1655
<220> dld b2134
<222> 1617-1716
<223> dld b2134 U00096 2220205..2221920
<313> 10396: 1694-1713
<400> 10396
GACGTTGCAG AAGTTCTATC GCGAGAACGA TCCGACCAAC AGCATGAATC 50
CGGGGATCGG TAAAACCAGT AAACGGAAAA ACTGGCAGGA AGTGGAGTAA 100
<210> 10397
<211> 100
<212> DNA
<213> Escherichia coli K-12 MG1655
<220> dld b2134
<222> 1535-1634
<223> dld b2134 U00096 2220205..2221920
<313> 10397: 1579-1598
<400> 10397
AACAGATGCT GGAACTGCTA CAGCAGCGCG GCGCGCAGTA CCCTGCCGAG 50
CATAACGTCG GTCATTTGTA TAAAGCACCG GAGACGTTGC AGAAGTTCTA 100
<210> 10398
<211> 100
<212> DNA
<213> Escherichia coli K-12 MG1655
<220> dld b2134
<222> 1387-1486
<223> dld b2134 U00096 2220205..2221920
<313> 10398: 1422-1441
<400> 10398
CGGCGTAACG ACACCGAGTG GTATGAGCAT TTACCGCCGG AGATCGACAG 50

CCAGCTGGTG CACAAGCTCT ATTACGGCCA TTTTATGTGC TATGTCTTCC 100
<210> 10399
<211> 100
<212> DNA
<213> Escherichia coli K-12 MG1655
<220> pino b3323
<222> 321-420
<223> pino b3323 U00096 complement(3451145..3451564)
<313> 10399: 380-399
<400> 10399
CTCTTCTTCT GAAGACGAAG AAAATACTGT GGAAGAAAGC GAAGAGAAAG 50
CCGGTTTAAG AGAGCGTGTC AAAAATGCAC TGAACGAACT TGAGAGATAA 100
<210> 10400
<211> 100
<212> DNA
<213> Escherichia coli K-12 MG1655
<220> pino b3323
<222> 269-368
<223> pino b3323 U00096 complement(3451145..3451564)
<313> 10400: 316-335
<400> 10400
TCGAATCCGT CGAGGAGGAA GATGACCCAG GCGTCGCGGT TGAGAATGCC 50
CCCTCTTCTT CTGAAGACGA AGAAAATACT GTGGAAGAAA GCGAAGAGAA 100
<210> 10401
<211> 100
<212> DNA
<213> Escherichia coli K-12 MG1655
<220> pino b3323
<222> 229-328
<223> pino b3323 U00096 complement(3451145..3451564)
<313> 10401: 281-300
<400> 10401
ACACAACACT ATTTCTTCAA AAAACAGCCA CTCCCCGTCG TCGAATCCGT 50
CGAGGAGGAA GATGACCCAG GCGTCGCGGT TGAGAATGCC CCCTCTTCTT 100
<210> 10402
<211> 100
<212> DNA
<213> Escherichia coli K-12 MG1655
<220> hyfr b2492
<222> 1880-1979
<223> hyfr b2492 U00096 2609941..2611932
<313> 10402: 1922-1941
<400> 10402
CCAATGGCAT TGTTGCCGGG CCCCGTGGCG CAGCGACGCG ATTAGGGATG 50
AAGCGCACCA CGCTGCTGTC ACGAATGCAG CGGCTGGGGA TCTCGGTTCG 100
<210> 10403
<211> 100
<212> DNA
<213> Escherichia coli K-12 MG1655
<220> hyfr b2492
<222> 1832-1931
<223> hyfr b2492 U00096 2609941..2611932
<313> 10403: 1880-1899
<400> 10403
AGAATGACGA AGAAGAACGT CAGCGCATTG TTCAGGTATT GCGAGAAACC 50
AATGGCATTG TTGCCGGGCC CCGTGGCGCA GCGACGCGAT TAGGGATGAA 100
<210> 10404
<211> 100
<212> DNA
<213> Escherichia coli K-12 MG1655
<220> hyfr b2492
<222> 1798-1897
<223> hyfr b2492 U00096 2609941..2611932
<313> 10404: 1833-1852
<400> 10404
TCAATGGCGC AGTTACTGCA CCCGACGACG CCAGAGAATG ACGAAGAAGA 50
ACGTCAGCGC ATTGTTCAGG TATTGCGAGA AACCAATGGC ATTGTTGCCG 100
<210> 10405
<211> 100
<212> DNA
<213> Escherichia coli K-12 MG1655

<220> ilvg_1 ilvG_1
<222> 885-984
<223> ilvg_1 ilvG_1 U00096 3948183..3949166
<313> 10405: 948-967
<400> 10405
CGACCCGGCA GAAATGAACA AGCTGCGTCA GGCACATGTG GCATTACAAG 50
GTGATTTAAA TGCTCTGTTA CCAGCATTAC AGCAGCCGTT AAATCAATGA 100
<210> 10406
<211> 100
<212> DNA
<213> Escherichia coli K-12 MG1655
<220> ilvg_1 ilvG_1
<222> 860-959
<223> ilvg_1 ilvG_1 U00096 3948183..3949166
<313> 10406: 902-921
<400> 10406
ACGCCAGTGT TATCCATATG GATATCGACC CGGCAGAAAT GAACAAGCTG 50
CGTCAGGCAC ATGTGGCATT ACAAGGTGAT TTAAATGCTC TGTTACCAGC 100
<210> 10407
<211> 100
<212> DNA
<213> Escherichia coli K-12 MG1655
<220> ilvg_1 ilvG_1
<222> 813-912
<223> ilvg_1 ilvG_1 U00096 3948183..3949166
<313> 10407: 849-868
<400> 10407
ACGTTTTGAT GACCGGGTGA CCGGCAAACT GAACACCTTC GCGCCACACG 50
CCAGTGTTAT CCATATGGAT ATCGACCCGG CAGAAATGAA CAAGCTGCGT 100
<210> 10408
<211> 100
<212> DNA
<213> Escherichia coli K-12 MG1655
<220> ilvg_2 b3769
<222> 560-659
<223> ilvg_2 b3769 U00096 3949162..3949827
<313> 10408: 580-599
<400> 10408
GTGATGGGCC ATACCTGCTT CATGTCTCAA TCGACGAACT TGAGAACGTC 50
TGGCCGCTGG TGCCGCCTGG CGCCAGTAAT TCAGAAATGT TGGAGAAATT 100
<210> 10409
<211> 100
<212> DNA
<213> Escherichia coli K-12 MG1655
<220> ilvg_2 b3769
<222> 506-605
<223> ilvg_2 b3769 U00096 3949162..3949827
<313> 10409: 559-578
<400> 10409
AACACATCAC CCGGAAAGAC CAGGTTGAAG CGGCACTCGA CACCATGCTG 50
AACAGTGATG GGCCATACCT GCTTCATGTC TCAATCGACG AACTTGAGAA 100
<210> 10410
<211> 100
<212> DNA
<213> Escherichia coli K-12 MG1655
<220> ilvg_2 b3769
<222> 452-551
<223> ilvg_2 b3769 U00096 3949162..3949827
<313> 10410: 473-492
<400> 10410
TTACTGATAA CCCCGATTTC CTCATGTTAG CCAGCGCCTT CGGCATCCAT 50
GGCCAACACA TCACCCGGAA AGACCAGGTT GAAGCGGCAC TCGACACCAT 100
<210> 10411
<211> 100
<212> DNA
<213> Escherichia coli K-12 MG1655
<220> b1180 b1181
<222> 561-660
<223> b1180 b1181 U00096 1227302..1227961
<313> 10411: 639-658
<400> 10411

TGTGTTGACA GGCACGCCTG ATGGCGTCGG CCCGTTGCAA AGCGGTGATG 50
AGCTGACAGT CACTTTCGAT GGGCATTCTT TGACAACTCG CGTTTTGTAA 100
<210> 10412
<211> 100
<212> DNA
<213> Escherichia coli K-12 MG1655
<220> b1180 b1181
<222> 475-574
<223> b1180 b1181 U00096 1227302..1227961
<313> 10412: 495-514
<400> 10412
CAAGGTACGA CTGCGGACAT GATCCATAAA ATCGTTCCGC TGATCGCTTA 50
TATGAGCAAG TTTTTTACCC TCAAGGCCGG TGACGTTGTG TTGACAGGCA 100
<210> 10413
<211> 100
<212> DNA
<213> Escherichia coli K-12 MG1655
<220> b1180 b1181
<222> 406-505
<223> b1180 b1181 U00096 1227302..1227961
<313> 10413: 457-476
<400> 10413
GCGGCGGAAT TTACCGGCGA CCCGCAAAAT ACAACGCTGA GCCTGAGCGT 50
AAACGGCGAA CAACGCCAGC AAGGTACGAC TGCGGACATG ATCCATAAAA 100
<210> 10414
<211> 100
<212> DNA
<213> Escherichia coli K-12 MG1655
<220> dnir b0212
<222> 1260-1359
<223> dnir b0212 U00096 complement(232597..233955)
<313> 10414: 1310-1329
<400> 10414
CATCAAAGAT GTGATGCGCT GGAACAGCGA CACTGCGAAT CTGCAACCAG 50
GCGATAAGCT GACGTTGTTT GTGAAAAACA ACAACATGCC AGATTCCTGA 100
<210> 10415
<211> 100
<212> DNA
<213> Escherichia coli K-12 MG1655
<220> dnir b0212
<222> 1242-1341
<223> dnir b0212 U00096 complement(232597..233955)
<313> 10415: 1261-1280
<400> 10415
TAAACGCCAC GGCGTGAACA TCAAAGATGT GATGCGCTGG AACAGCGACA 50
CTGCGAATCT GCAACCAGGC GATAAGCTGA CGTTGTTTGT GAAAAACAAC 100
<210> 10416
<211> 100
<212> DNA
<213> Escherichia coli K-12 MG1655
<220> dnir b0212
<222> 1152-1251
<223> dnir b0212 U00096 complement(232597..233955)
<313> 10416: 1177-1196
<400> 10416
TGGTGCAGGC AGTAGCGCAC AGCGACTGGC AAACAACAGC GATAGCATTA 50
CGTATCGTGT GCGCAAAGGC GATTCGCTTT CAAGCATTGC TAAACGCCAC 100
<210> 10417
<211> 100
<212> DNA
<213> Escherichia coli K-12 MG1655
<220> shia b1982
<222> 1211-1310
<223> shia b1982 U00096 2051665..2052981
<313> 10417: 1263-1282
<400> 10417
TCACTTACTT TGCCGGGAAC TGGCATAGCG TCGCCATTTA TTTGCTGGCT 50
GGATGCCTGA TTTCCGCAAT GACCGCTTTG TTGATGAAAG ACAGTCAACG 100
<210> 10418
<211> 100
<212> DNA

<213> Escherichia coli K-12 MG1655
<220> shia b1982
<222> 1157-1256
<223> shia b1982 U00096 2051665..2052981
<313> 10418: 1209-1228
<400> 10418
AGGTTGCCAG TGTGGTTGGC GGTGGATTTA CACCTTTTAT TGCCGCTGCA 50
CTCATCACTT ACTTTGCCGG GAACTGGCAT AGCGTCGCCA TTTATTTGCT 100
<210> 10419
<211> 100
<212> DNA
<213> Escherichia coli K-12 MG1655
<220> shia b1982
<222> 962-1061
<223> shia b1982 U00096 2051665..2052981
<313> 10419: 989-1008
<400> 10419
CAGGTACGTT AATCGGAACG TTGAGCGCAT TTCCTTTCTT TATGGCGCTT 50
GAAGCACAAT CTATTTTCTG GATAGTTTTC TTCTCCATAA TGCTGGCAAA 100
<210> 10420
<211> 100
<212> DNA
<213> Escherichia coli K-12 MG1655
<220> wzxc b2047
<222> 1380-1479
<223> wzxc b2047 U00096 complement(2116702..2118180)
<313> 10420: 1443-1462
<400> 10420
TGTGGTGATG ATTGTGCTGT CGCGCCATCC GCTGGTGGTG GAAGTGAAGC 50
GTCAGTTTTG TCGCAGCGAA AAAATGAAAA TGCTTTTACG GGCGGGGTGA 100
<210> 10421
<211> 100
<212> DNA
<213> Escherichia coli K-12 MG1655
<220> wzxc b2047
<222> 1337-1436
<223> wzxc b2047 U00096 complement(2116702..2118180)
<313> 10421: 1391-1410
<400> 10421
GGATGCTGCT GGCGGTGCAA ATAGCCACGG GGGTGCTGGC GTTTGTGGTG 50
ATGATTGTGC TGTCGCGCCA TCCGCTGGTG GTGGAAGTGA AGCGTCAGTT 100
<210> 10422
<211> 100
<212> DNA
<213> Escherichia coli K-12 MG1655
<220> wzxc b2047
<222> 1273-1372
<223> wzxc b2047 U00096 complement(2116702..2118180)
<313> 10422: 1292-1311
<400> 10422
TCGCTGCCGA CGCTGGTGGT CAGTTATGCG CTGGGCATTG TGCTGAAAGG 50
GCAACTGGCG CTGGGGATGC TGCTGGCGGT GCAAATAGCC ACGGGGGTGC 100
<210> 10423
<211> 100
<212> DNA
<213> Escherichia coli K-12 MG1655
<220> b1191 b1191
<222> 1512-1611
<223> b1191 b1191 U00096 complement(1239558..1241168)
<313> 10423: 1545-1564
<400> 10423
GGTAGAGTTT GCCGGGATGA TCTGGACGGT GGCCGAGAAA GAAGATAATG 50
AAGTGTTGAA GATTGGCGTA CGGGTGGCTG AAGAGGAAGC TGAATCTTAA 100
<210> 10424
<211> 100
<212> DNA
<213> Escherichia coli K-12 MG1655
<220> b1191 b1191
<222> 1375-1474
<223> b1191 b1191 U00096 complement(1239558..1241168)
<313> 10424: 1417-1436

<400> 10424
CTCGAAGCCA GCGCCAAATA TGCTGATGTT GCGCTGATAT ATGGCCTGGA 50
AGACGGGCGA GAATATCGTG ATAAGCAGCA AACGCTGGGT GAAATTGTCC 100
<210> 10425
<211> 100
<212> DNA
<213> Escherichia coli K-12 MG1655
<220> b1191 b1191
<222> 1331-1430
<223> b1191 b1191 U00096 complement(1239558..1241168)
<313> 10425: 1372-1391
<400> 10425
CGCCGCCGGT CGCGCTGGAT CAACGCTTCT TTGGTGACTT CATTCTCGAA 50
GCCAGCGCCA AATATGCTGA TGTTGCGCTG ATATATGGCC TGGAAGACGG 100
<210> 10426
<211> 100
<212> DNA
<213> Escherichia coli K-12 MG1655
<220> wzxe b4404
<222> 1152-1251
<223> wzxe b4404 U00096 3973890..3975140
<313> 10426: 1212-1231
<400> 10426
GCATGGTGCA CTGGGCGCGG CGCAGGCATA TATGGCAACT TATATCGTCT 50
ATTTTTCTCT TTGTTGTGGC GTGTTTTTAC TCTGGCGTAG GCGGGCATGA 100
<210> 10427
<211> 100
<212> DNA
<213> Escherichia coli K-12 MG1655
<220> wzxe b4404
<222> 1089-1188
<223> wzxe b4404 U00096 3973890..3975140
<313> 10427: 1115-1134
<400> 10427
TATTCTGGCG GAAGTCAGCC AGTTCACTTT ATTGATGGTA TTTGCCCACT 50
GGCTAATCCC TGCGCATGGT GCACTGGGCG CGGCGCAGGC ATATATGGCA 100
<210> 10428
<211> 100
<212> DNA
<213> Escherichia coli K-12 MG1655
<220> wzxe b4404
<222> 978-1077
<223> wzxe b4404 U00096 3973890..3975140
<313> 10428: 1008-1027
<400> 10428
ATTTACCGCT ATGCGCGATC TCTTTGCCTG GCAGTTAGTG GGTGATGTGT 50
TAAAAGTGGG CGCTTATGTC TTTGGTTATC TGGTGATCGC CAAAGCGTCA 100
<210> 10429
<211> 100
<212> DNA
<213> Escherichia coli K-12 MG1655
<220> b1192 b1193
<222> 801-900
<223> b1192 b1193 U00096 complement(1241389..1242303)
<313> 10429: 830-849
<400> 10429
TTTTGGTCAT GAACAACGCA CGGTTACGCT GCCGTTGGGC GCACATGCCA 50
TTCTGAATAA CACCCGGGAA GGCACTCAGT TGACGATTAG CGGTCATCCT 100
<210> 10430
<211> 100
<212> DNA
<213> Escherichia coli K-12 MG1655
<220> b1192 b1193
<222> 736-835
<223> b1192 b1193 U00096 complement(1241389..1242303)
<313> 10430: 799-818
<400> 10430
AACCTGGAGT CAGTGTACGC GTTTTTGCGT TCCCGCCTGT CGATTCCACT 50
CATTACCGGC CTCGATTTTG GTCATGAACA ACGCACGGTT ACGCTGCCGT 100
<210> 10431
<211> 100

<212> DNA
<213> Escherichia coli K-12 MG1655
<220> b1192 b1193
<222> 670-769
<223> b1192 b1193 U00096 complement(1241389..1242303)
<313> 10431: 719-738
<400> 10431
CGTCAGAAGG CGATTATTCT CGGTAGCTTT AGCGGCAGCA CGCCCAATGA 50
TTATGACGCA GGTTACAACC TGGAGTCAGT GTACGCGTTT TTGCGTTCCC 100
<210> 10432
<211> 100
<212> DNA
<213> Escherichia coli K-12 MG1655
<220> b1199 b1199
<222> 505-604
<223> b1199 b1199 U00096 complement(1248348..1248980)
<313> 10432: 531-550
<400> 10432
ACGATTACGA TGCAAGCCCG CAAAGGCCGC GCCAGTTATC TCGGTGAACG 50
CAGTATTGGT CACCAGGATC CCGGCGCGAC CTCGGTGATG TTTATGATGC 100
<210> 10433
<211> 100
<212> DNA
<213> Escherichia coli K-12 MG1655
<220> b1199 b1199
<222> 471-570
<223> b1199 b1199 U00096 complement(1248348..1248980)
<313> 10433: 501-520
<400> 10433
TGCCAGTAGC ATCGCCGAAT CCGCTGCACA AAGTACGATT ACGATGCAAG 50
CCCGCAAAGG CCGCGCCAGT TATCTCGGTG AACGCAGTAT TGGTCACCAG 100
<210> 10434
<211> 100
<212> DNA
<213> Escherichia coli K-12 MG1655
<220> b1199 b1199
<222> 401-500
<223> b1199 b1199 U00096 complement(1248348..1248980)
<313> 10434: 450-469
<400> 10434
GGGTGCCGGT GGTGGAATCG TTACGTCAGT CCAGCGAGCA AAATCTCTCT 50
GTTCCGGTGG CGCTCGAAGC TGCCAGTAGC ATCGCCGAAT CCGCTGCACA 100
<210> 10435
<211> 100
<212> DNA
<213> Escherichia coli K-12 MG1655
<220> spr b2176
<222> 468-567
<223> spr b2176 U00096 2267999..2268565
<313> 10435: 547-566
<400> 10435
CCATGCTTCC ACCAGCAGTG GTGTTATTAT TTCCAGCATG AATGAACCGT 50
ACTGGAAGAA GCGTTACAAC GAAGCACGCC GGGTTCTCAG CCGCAGCTAA 100
<210> 10436
<211> 100
<212> DNA
<213> Escherichia coli K-12 MG1655
<220> spr b2176
<222> 429-528
<223> spr b2176 U00096 2267999..2268565
<313> 10436: 459-478
<400> 10436
ACGCCATGTC GGTATTTATA TCGGCAACAA TCAGTTTGTC CATGCTTCCA 50
CCAGCAGTGG TGTTATTATT TCCAGCATGA ATGAACCGTA CTGGAAGAAG 100
<210> 10437
<211> 100
<212> DNA
<213> Escherichia coli K-12 MG1655
<220> spr b2176
<222> 281-380
<223> spr b2176 U00096 2267999..2268565

<313> 10437: 347-366
<400> 10437
GTTCTGGTTT CGTACAGCGT ACATTCCGTG AGCAATTTGG CTTAGAACTT 50
CCGCGTTCGA CTTACGAACA GCAGGAAATG GGTAAATCTG TTTCCCGCAG 100
<210> 10438
<211> 100
<212> DNA
<213> Escherichia coli K-12 MG1655
<220> spy b1744
<222> 387-486
<223> spy b1744 U00096 complement(1823164..1823649)
<313> 10438: 447-466
<400> 10438
CCTGACGCCG GAACAGAAAA AGCAATTTAA TGCTAATTTT GAGAAGCGTC 50
TGACAGAACG TCCAGCGGCA AAAGGTAAAA TGCCTGCAAC TGCTGAATAA 100
<210> 10439
<211> 100
<212> DNA
<213> Escherichia coli K-12 MG1655
<220> spy b1744
<222> 353-452
<223> spy b1744 U00096 complement(1823164..1823649)
<313> 10439: 384-403
<400> 10439
CGCACATGGA AACCCAGAAC AAAATTTACA ACATCCTGAC GCCGGAACAG 50
AAAAAGCAAT TTAATGCTAA TTTTGAGAAG CGTCTGACAG AACGTCCAGC 100
<210> 10440
<211> 100
<212> DNA
<213> Escherichia coli K-12 MG1655
<220> spy b1744
<222> 303-402
<223> spy b1744 U00096 complement(1823164..1823649)
<313> 10440: 319-338
<400> 10440
TGAAGCGCAG ATCGCAAAAA TGGAAGAACA GCGCAAAGCT AACATGCTGG 50
CGCACATGGA AACCCAGAAC AAAATTTACA ACATCCTGAC GCCGGAACAG 100
<210> 10441
<211> 100
<212> DNA
<213> Escherichia coli K-12 MG1655
<220> wzzb b2028
<222> 861-960
<223> wzzb b2028 U00096 complement(2095343..2096359)
<313> 10441: 927-946
<400> 10441
TCATGCTTAC CGTTATGTGA TGAAACCGAT GTTACCTATT CGTCGTGATA 50
GCCCGAAAAA GGCAATTACC TTGATTCTGG CGGTGCTGCT GGGTGGCATG 100
<210> 10442
<211> 100
<212> DNA
<213> Escherichia coli K-12 MG1655
<220> wzzb b2028
<222> 743-842
<223> wzzb b2028 U00096 complement(2095343..2096359)
<313> 10442: 793-812
<400> 10442
CGCTGGAGTC GATGATTAAG CATGAGGCGA CCCGTCCGTT GGTGTTCTCG 50
CCAAACTACT ATCAGACTCG TCAAAACCTG CTGGATATCG AAAGCTTAAA 100
<210> 10443
<211> 100
<212> DNA
<213> Escherichia coli K-12 MG1655
<220> wzzb b2028
<222> 725-824
<223> wzzb b2028 U00096 complement(2095343..2096359)
<313> 10443: 762-781
<400> 10443
TCCTTTTGGG GAGCGAAGCG CTGGAGTCGA TGATTAAGCA TGAGGCGACC 50
CGTCCGTTGG TGTTCTCGCC AAACTACTAT CAGACTCGTC AAAACCTGCT 100
<210> 10444

<211> 100
<212> DNA
<213> Escherichia coli K-12 MG1655
<220> wzze b3786
<222> 951-1050
<223> wzze b3786 U00096 3966643..3967692
<313> 10444: 1028-1047
<400> 10444
ACGCGATAGC CCACGTCGTG CCTTCCTGAT GATTATGTGG GGCATTGTCG 50
GGGGGCTGAT CGGGGCTGGT GTCGCATTAA CCCGCCGTTG CTCGAAATAG 100
<210> 10445
<211> 100
<212> DNA
<213> Escherichia coli K-12 MG1655
<220> wzze b3786
<222> 926-1025
<223> wzze b3786 U00096 3966643..3967692
<313> 10445: 958-977
<400> 10445
TGCGTACGCC GGAAGAACCG GTAAAACGCG ATAGCCCACG TCGTGCCTTC 50
CTGATGATTA TGTGGGGCAT TGTCGGGGGG CTGATCGGGG CTGGTGTCGC 100
<210> 10446
<211> 100
<212> DNA
<213> Escherichia coli K-12 MG1655
<220> wzze b3786
<222> 802-901
<223> wzze b3786 U00096 3966643..3967692
<313> 10446: 858-877
<400> 10446
CGACTGGAAA ATTTACAGGC CGTCGGTCCG GCCTTTGATC TCGACTATGA 50
TCAGAATCGG GCCATGTTAA ACACCCTGAA TGTTGGTCCA ACCCTGGATC 100
<210> 10447
<211> 100
<212> DNA
<213> Escherichia coli K-12 MG1655
<220> dps b0813
<222> 405-504
<223> dps b0813 U00096 complement(847631..848134)
<313> 10447: 427-446
<400> 10447
GATTGGCGAA GCGAAAGATG ACGACACCGC AGATATCCTG ACCGCCGCGT 50
CTCGCGACCT GGATAAATTC CTGTGGTTTA TCGAGTCTAA CATCGAATAA 100
<210> 10448
<211> 100
<212> DNA
<213> Escherichia coli K-12 MG1655
<220> dps b0813
<222> 314-413
<223> dps b0813 U00096 complement(847631..848134)
<313> 10448: 364-383
<400> 10448
AAAGTTACCC GCTGGACATC CACAACGTTC AGGATCACCT GAAAGAACTG 50
GCTGACCGTT ACGCAATCGT CGCTAATGAC GTACGCAAAG CGATTGGCGA 100
<210> 10449
<211> 100
<212> DNA
<213> Escherichia coli K-12 MG1655
<220> dps b0813
<222> 282-381
<223> dps b0813 U00096 complement(847631..848134)
<313> 10449: 321-340
<400> 10449
CACTCAAGTT ATCAACAGCA AAACCCCGCT GAAAAGTTAC CCGCTGGACA 50
TCCACAACGT TCAGGATCAC CTGAAAGAAC TGGCTGACCG TTACGCAATC 100
<210> 10450
<211> 100
<212> DNA
<213> Escherichia coli K-12 MG1655
<220> faba fabA
<222> 420-519

<223> faba fabA U00096 complement(1015175..1015693)
<313> 10450: 495-514
<400> 10450
TATGGGCCTG GCGGATGGCG AAGTGCTGGT TGATGGTCGT CTGATCTATA 50
CCGCCAGCGA CCTGAAAGTC GGTCTGTTCC AGGATACGTC TGCCTTCTGA 100
<210> 10451
<211> 100
<212> DNA
<213> Escherichia coli K-12 MG1655
<220> faba fabA
<222> 327-426
<223> faba fabA U00096 complement(1015175..1015693)
<313> 10451: 362-381
<400> 10451
TGGCGAAGTG AAATTCACTG GTCAGGTACT GCCGACAGCG AAAAAAGTGA 50
CCTACCGTAT TCACTTTAAA CGCATTGTTA ACCGTCGTCT GATTATGGGC 100
<210> 10452
<211> 100
<212> DNA
<213> Escherichia coli K-12 MG1655
<220> faba fabA
<222> 178-277
<223> faba fabA U00096 complement(1015175..1015693)
<313> 10452: 217-236
<400> 10452
GATATCAATC CGGATCTGTG GTTCTTCGGA TGCCACTTTA TTGGCGATCC 50
GGTTATGCCG GGATGCCTGG GCCTGGACGC AATGTGGCAG CTGGTAGGGT 100
<210> 10453
<211> 100
<212> DNA
<213> Escherichia coli K-12 MG1655
<220> fabb fabB
<222> 1122-1221
<223> fabb fabB U00096 complement(2438405..2439625)
<313> 10453: 1178-1197
<400> 10453
GACCGAAACG ACCGATCGCG AACTGACCAC CGTTATGTCT AACAGCTTCG 50
GCTTCGGCGG CACCAACGCC ACGCTGGTAA TGCGCAAGCT GAAAGATTAA 100
<210> 10454
<211> 100
<212> DNA
<213> Escherichia coli K-12 MG1655
<220> fabb fabB
<222> 1041-1140
<223> fabb fabB U00096 complement(2438405..2439625)
<313> 10454: 1078-1097
<400> 10454
GCTGATGCTG GAACACGGCT TTATCGCCCC GAGCATCAAC ATTGAAGAGC 50
TGGACGAGCA GGCTGCGGGT CTGAACATCG TGACCGAAAC GACCGATCGC 100
<210> 10455
<211> 100
<212> DNA
<213> Escherichia coli K-12 MG1655
<220> fabb fabB
<222> 987-1086
<223> fabb fabB U00096 complement(2438405..2439625)
<313> 10455: 1048-1067
<400> 10455
CATGACCGGT CACTCTCTGG GCGCTGCTGG CGTACAGGAA GCTATCTACT 50
CTCTGCTGAT GCTGGAACAC GGCTTTATCG CCCCGAGCAT CAACATTGAA 100
<210> 10456
<211> 100
<212> DNA
<213> Escherichia coli K-12 MG1655
<220> fabd b1093
<222> 745-844
<223> fabd b1093 U00096 1148951..1149880
<313> 10456: 797-816
<400> 10456
CGTCAGTTGT ATAACCCGGT TCAGTGGACG AAGTCTGTTG AGTACATGGC 50
AGCGCAAGGC GTAGAACATC TCTATGAAGT CGGCCCGGGC AAAGTGCTTA 100

<210> 10457
<211> 100
<212> DNA
<213> Escherichia coli K-12 MG1655
<220> fabd b1093
<222> 677-776
<223> fabd b1093 U00096 1148951..1149880
<313> 10457: 732-751
<400> 10457
TTCCTGTTGT GAATAACGTT GATGTGAAAT GCGAAACCAA TGGTGATGCC 50
ATCCGTGACG CACTGGTACG TCAGTTGTAT AACCCGGTTC AGTGGACGAA 100
<210> 10458
<211> 100
<212> DNA
<213> Escherichia coli K-12 MG1655
<220> fabd b1093
<222> 586-685
<223> fabd b1093 U00096 1148951..1149880
<313> 10458: 639-658
<400> 10458
GTGAGCGTAC CGTCTCACTG TGCGCTGATG AAACCAGCAG CCGACAAACT 50
GGCAGTAGAA TTAGCGAAAA TCACCTTTAA CGCACCAACA GTTCCTGTTG 100
<210> 10459
<211> 100
<212> DNA
<213> Escherichia coli K-12 MG1655
<220> fabf b1096
<222> 1143-1242
<223> fabf b1096 U00096 1151162..1152403
<313> 10459: 1209-1228
<400> 10459
ACCGCACGAA GCGCGTCAGG TTAGCGGAAT GGAATACACT CTGTGTAACT 50
CCTTCGGCTT CGGTGGCACT AATGGTTCTT TGATCTTTAA AAAGATCTAA 100
<210> 10460
<211> 100
<212> DNA
<213> Escherichia coli K-12 MG1655
<220> fabf b1096
<222> 1141-1240
<223> fabf b1096 U00096 1151162..1152403
<313> 10460: 1167-1186
<400> 10460
GTACCGCACG AAGCGCGTCA GGTTAGCGGA ATGGAATACA CTCTGTGTAA 50
CTCCTTCGGC TTCGGTGGCA CTAATGGTTC TTTGATCTTT AAAAAGATCT 100
<210> 10461
<211> 100
<212> DNA
<213> Escherichia coli K-12 MG1655
<220> fabf b1096
<222> 1036-1135
<223> fabf b1096 U00096 1151162..1152403
<313> 10461: 1092-1111
<400> 10461
GCGGGTGCAG TAGAATCTAT CTACTCCATC CTGGCGCTGC GCGATCAGGC 50
TGTTCCGCCA ACCATCAACC TGGATAACCC GGATGAAGGT TGCGATCTGG 100
<210> 10462
<211> 100
<212> DNA
<213> Escherichia coli K-12 MG1655
<220> fabg b1094
<222> 636-735
<223> fabg b1094 U00096 1149893..1150627
<313> 10462: 672-691
<400> 10462
ACAGGAAATC GCCAACGCGG TTGCATTCCT GGCATCCGAC GAAGCAGCTT 50
ACATCACGGG TGAAACTTTG CATGTGAACG GCGGGATGTA CATGGTCTGA 100
<210> 10463
<211> 100
<212> DNA
<213> Escherichia coli K-12 MG1655
<220> fabg b1094

<222> 499-598
<223> fabg b1094 U00096 1149893..1150627
<313> 10463: 533-552
<400> 10463
CGCGAAGTTG CGTCACGCGG TATTACTGTA AACGTTGTTG CTCCGGGCTT 50
TATTGAAACG GACATGACAC GTGCGCTGAG CGATGACCAG CGTGCGGGTA 100
<210> 10464
<211> 100
<212> DNA
<213> Escherichia coli K-12 MG1655
<220> fabg b1094
<222> 365-464
<223> fabg b1094 U00096 1149893..1150627
<313> 10464: 399-418
<400> 10464
TGCGCGCTAT GATGAAAAAG CGTCATGGTC GTATTATCAC TATCGGTTCT 50
GTGGTTGGTA CCATGGGAAA TGGCGGTCAG GCCAACTACG CTGCGGCGAA 100
<210> 10465
<211> 100
<212> DNA
<213> Escherichia coli K-12 MG1655
<220> fabh b1092
<222> 855-954
<223> fabh b1092 U00096 1147982..1148935
<313> 10465: 899-918
<400> 10465
TGAAGCTGTA CGCGACGGGC GCATTAAGCC GGGGCAGTTG GTTCTGCTTG 50
AAGCCTTTGG CGGTGGATTC ACCTGGGGCT CCGCGCTGGT TCGTTTCTAG 100
<210> 10466
<211> 100
<212> DNA
<213> Escherichia coli K-12 MG1655
<220> fabh b1092
<222> 758-857
<223> fabh b1092 U00096 1147982..1148935
<313> 10466: 818-837
<400> 10466
CAACGGCGAA AAAACTCGGT ATGTCTATGG ATAATGTCGT GGTGACGCTG 50
GATCGCCACG GTAATACCTC TGCGGCCTCT GTCCCGTGCG CGCTGGATGA 100
<210> 10467
<211> 100
<212> DNA
<213> Escherichia coli K-12 MG1655
<220> fabh b1092
<222> 703-802
<223> fabh b1092 U00096 1147982..1148935
<313> 10467: 750-769
<400> 10467
CGTTCTCAAC TGGACTGGCT GGTTCCGCAT CAGGCTAACC TGCGTATTAT 50
CAGTGCAACG GCGAAAAAAC TCGGTATGTC TATGGATAAT GTCGTGGTGA 100
<210> 10468
<211> 100
<212> DNA
<213> Escherichia coli K-12 MG1655
<220> fabi b1289
<222> 631-730
<223> fabi b1289 U00096 complement(1348275..1349063)
<313> 10468: 675-694
<400> 10468
GAAGCCGTTA CCCCGATTCG CCGTACCGTT ACTATTGAAG ATGTGGGTAA 50
CTCTGCGGCA TTCCTGTGCT CCGATCTCTC TGCCGGTATC TCCGGTGAAG 100
<210> 10469
<211> 100
<212> DNA
<213> Escherichia coli K-12 MG1655
<220> fabi b1289
<222> 617-716
<223> fabi b1289 U00096 complement(1348275..1349063)
<313> 10469: 636-655
<400> 10469
TGCTGGCTCA TTGCGAAGCC GTTACCCCGA TTCGCCGTAC CGTTACTATT 50

GAAGATGTGG GTAACTCTGC GGCATTCCTG TGCTCCGATC TCTCTGCCGG 100
<210> 10470
<211> 100
<212> DNA
<213> Escherichia coli K-12 MG1655
<220> fabi b1289
<222> 557-656
<223> fabi b1289 U00096 complement(1348275..1349063)
<313> 10470: 598-617
<400> 10470
CCATCTCTGC TGGTCCGATC CGTACTCTGG CGGCCTCCGG TATCAAAGAC 50
TTCCGCAAAA TGCTGGCTCA TTGCGAAGCC GTTACCCCGA TTCGCCGTAC 100
<210> 10471
<211> 100
<212> DNA
<213> Escherichia coli K-12 MG1655
<220> b0499 b0500
<222> 223-322
<223> b0499 b0500 U00096 527173..527883
<313> 10471: 269-288
<400> 10471
GGATGGAGTC TGTATGCGTA TCCGCTGAAT CCGGTGAATG GTATTGATCC 50
ATTAGGGTTA AGTCCCGCAG ATGTAGCGCT AATAAGAAGA AAAGATCAAC 100
<210> 10472
<211> 100
<212> DNA
<213> Escherichia coli K-12 MG1655
<220> b0499 b0500
<222> 172-271
<223> b0499 b0500 U00096 527173..527883
<313> 10472: 209-228
<400> 10472
GATCCGTTGC AGGGGCGGTA TATCACTCAG GACCCGATAG GGCTGGAGGG 50
GGGATGGAGT CTGTATGCGT ATCCGCTGAA TCCGGTGAAT GGTATTGATC 100
<210> 10473
<211> 100
<212> DNA
<213> Escherichia coli K-12 MG1655
<220> b0499 b0500
<222> 97-196
<223> b0499 b0500 U00096 527173..527883
<313> 10473: 128-147
<400> 10473
CACCAGCCGT ACCGGCTGCC GGGGCAGCAG TATGATAAGG AGTCGGGGCT 50
GTACTACAAC CGGAACCGGT ACTACGATCC GTTGCAGGGG CGGTATATCA 100
<210> 10474
<211> 100
<212> DNA
<213> Escherichia coli K-12 MG1655
<220> lar b1349
<222> 96-195
<223> lar b1349 U00096 complement(1411757..1411951)
<313> 10474: 164-183
<400> 10474
ATGCGAATCC CGAACCGGTT ATGGTGGAAG TGAAAAAGAA GCACTCGAAA 50
GATGGAATAA ACGAACCACT GGAAATAATA ATGGAGGTGT TCATGTATAA 100
<210> 10475
<211> 100
<212> DNA
<213> Escherichia coli K-12 MG1655
<220> lar b1349
<222> 96-195
<223> lar b1349 U00096 complement(1411757..1411951)
<313> 10475: 160-179
<400> 10475
ATGCGAATCC CGAACCGGTT ATGGTGGAAG TGAAAAAGAA GCACTCGAAA 50
GATGGAATAA ACGAACCACT GGAAATAATA ATGGAGGTGT TCATGTATAA 100
<210> 10476
<211> 100
<212> DNA
<213> Escherichia coli K-12 MG1655

<220> lar b1349
<222> 96-195
<223> lar b1349 U00096 complement(1411757..1411951)
<313> 10476: 147-166
<400> 10476
ATGCGAATCC CGAACCGGTT ATGGTGGAAG TGAAAAAGAA GCACTCGAAA 50
GATGGAATAA ACGAACCACT GGAAATAATA ATGGAGGTGT TCATGTATAA 100
<210> 10477
<211> 100
<212> DNA
<213> Escherichia coli K-12 MG1655
<220> fabz b0181
<222> 331-430
<223> fabz b0181 U00096 202101..202556
<313> 10477: 392-411
<400> 10477
ATGATCATGG AAGTCACTTT CGAAAAAACG CGCCGCGGCC TGACCCGTTT 50
TAAAGGGGTT GCTCTGGTCG ATGGTAAAGT AGTTTGCGAA GCAACGATGA 100
<210> 10478
<211> 100
<212> DNA
<213> Escherichia coli K-12 MG1655
<220> fabz b0181
<222> 227-326
<223> fabz b0181 U00096 202101..202556
<313> 10478: 263-282
<400> 10478
TTCTGGCGTT TAAAAGCGTA GGAAAACTGG AACCGGGTGA GCTGTACTAC 50
TTCGCTGGTA TTGACGAAGC GCGCTTCAAG CGCCCGGTCG TGCCTGGCGA 100
<210> 10479
<211> 100
<212> DNA
<213> Escherichia coli K-12 MG1655
<220> fabz b0181
<222> 204-303
<223> fabz b0181 U00096 202101..202556
<313> 10479: 221-240
<400> 10479
AGCAATGGCA CAGGCAACAG GTATTCTGGC GTTTAAAAGC GTAGGAAAAC 50
TGGAACCGGG TGAGCTGTAC TACTTCGCTG GTATTGACGA AGCGCGCTTC 100
<210> 10480
<211> 100
<212> DNA
<213> Escherichia coli K-12 MG1655
<220> ssb b4060
<222> 438-537
<223> ssb b4060 U00096 4271704..4272240
<313> 10480: 511-530
<400> 10480
TCAGTTCAGC GGCGGCGCGC AGTCTCGCCC GCAGCAGTCC GCTCCGGCAG 50
CGCCGTCTAA CGAGCCGCCG ATGGACTTTG ATGATGACAT TCCGTTCTGA 100
<210> 10481
<211> 100
<212> DNA
<213> Escherichia coli K-12 MG1655
<220> ssb b4060
<222> 438-537
<223> ssb b4060 U00096 4271704..4272240
<313> 10481: 473-492
<400> 10481
TCAGTTCAGC GGCGGCGCGC AGTCTCGCCC GCAGCAGTCC GCTCCGGCAG 50
CGCCGTCTAA CGAGCCGCCG ATGGACTTTG ATGATGACAT TCCGTTCTGA 100
<210> 10482
<211> 100
<212> DNA
<213> Escherichia coli K-12 MG1655
<220> ssb b4060
<222> 380-479
<223> ssb b4060 U00096 4271704..4272240
<313> 10482: 423-442
<400> 10482

TCGGTGGTGG TCAGCCGCAG GGCGGTTGGG GTCAGCCTCA GCAGCCGCAG 50
GGTGGCAATC AGTTCAGCGG CGGCGCGCAG TCTCGCCCGC AGCAGTCCGC 100
<210> 10483
<211> 100
<212> DNA
<213> Escherichia coli K-12 MG1655
<220> b2603 b2604
<222> 420-519
<223> b2603 b2604 U00096 2739895..2740413
<313> 10483: 485-504
<400> 10483
TGACGTCAGA TTTGCCGTAA ACCTGGATGC CTTATCGCGT AGCGGTGTAA 50
AGGTCAACCC GGATGTCTTA ATGCTCGCAC GGAAGAAAAA TGATGGATAA 100
<210> 10484
<211> 100
<212> DNA
<213> Escherichia coli K-12 MG1655
<220> b2603 b2604
<222> 411-510
<223> b2603 b2604 U00096 2739895..2740413
<313> 10484: 442-461
<400> 10484
CCACAATAAT GACGTCAGAT TTGCCGTAAA CCTGGATGCC TTATCGCGTA 50
GCGGTGTAAA GGTCAACCCG GATGTCTTAA TGCTCGCACG GAAGAAAAAT 100
<210> 10485
<211> 100
<212> DNA
<213> Escherichia coli K-12 MG1655
<220> b2603 b2604
<222> 290-389
<223> b2603 b2604 U00096 2739895..2740413
<313> 10485: 327-346
<400> 10485
TTGGCAACGA GTCACCGACA TTCCAGATGG AATTAACCGA ACAATATCCG 50
TCAAAGGCGT TATTATTAAT TGCCGAACAA AATACCGAGT GCATTATTGG 100
<210> 10486
<211> 100
<212> DNA
<213> Escherichia coli K-12 MG1655
<220> pita b3494
<222> 1347-1446
<223> pita b3494 U00096 3635272..3636771
<313> 10486: 1401-1420
<400> 10486
ACTCTCCTCT TCTGTCGCGG GGACGATGGT GGTAGATGGT GGCGGCTTAC 50
AGCGTAAAAC CGTGACCAGC ATTCTGATGG CCTGGGTGTT TACCCTTCCG 100
<210> 10487
<211> 100
<212> DNA
<213> Escherichia coli K-12 MG1655
<220> pita b3494
<222> 1322-1421
<223> pita b3494 U00096 3635272..3636771
<313> 10487: 1347-1366
<400> 10487
GGATGCCGGT TTCCACTACT CACGTACTCT CCTCTTCTGT CGCGGGGACG 50
ATGGTGGTAG ATGGTGGCGG CTTACAGCGT AAAACCGTGA CCAGCATTCT 100
<210> 10488
<211> 100
<212> DNA
<213> Escherichia coli K-12 MG1655
<220> pita b3494
<222> 1243-1342
<223> pita b3494 U00096 3635272..3636771
<313> 10488: 1299-1318
<400> 10488
AAGAAAGGCA TGACCTACGC TCAGGGGATG TCTGCCCAGA TGACGGCGGC 50
AGTGTCTATC GGCCTGGCGA GTTATACCGG GATGCCGGTT TCCACTACTC 100
<210> 10489
<211> 100
<212> DNA

<213> Escherichia coli K-12 MG1655
<220> pitb b2988
<222> 1401-1500
<223> pitb b2988 U00096 complement(3132887..3134386)
<313> 10489: 1453-1472
<400> 10489
TAAAACGGTA ACCAGCATCC TGATGGCGTG GGTATTTACT TTACCGGCGG 50
CAATTTTTCT TTCTGGTGGG CTGTACTGGA TAGCATTGCA GTTGATTTAA 100
<210> 10490
<211> 100
<212> DNA
<213> Escherichia coli K-12 MG1655
<220> pitb b2988
<222> 1356-1455
<223> pitb b2988 U00096 complement(3132887..3134386)
<313> 10490: 1415-1434
<400> 10490
TGCAGTTGCA GGGACGATGG TGGTGGACGG CGGTGGGTTA CAGCGTAAAA 50
CGGTAACCAG CATCCTGATG GCGTGGGTAT TTACTTTACC GGCGGCAATT 100
<210> 10491
<211> 100
<212> DNA
<213> Escherichia coli K-12 MG1655
<220> pitb b2988
<222> 1282-1381
<223> pitb b2988 U00096 complement(3132887..3134386)
<313> 10491: 1334-1353
<400> 10491
ATGACGGCGG CAGTGTCTAT CGGTCTTGCC AGTTATATTG GGATGCCCGT 50
CTCCACAACA CACGTCCTCT CGTCTGCAGT TGCAGGGACG ATGGTGGTGG 100
<210> 10492
<211> 100
<212> DNA
<213> Escherichia coli K-12 MG1655
<220> gmha b0223
<222> 462-561
<223> gmha b0223 U00096 243543..244121
<313> 10492: 507-526
<400> 10492
CACGGCGGAT ATCGAAATTC GCGTACCGCA CTTTGGTTAT GCCGACCGCA 50
TTCAGGAGAT TCACATTAAA GTGATCCATA TCCTGATCCA GTTGATTGAA 100
<210> 10493
<211> 100
<212> DNA
<213> Escherichia coli K-12 MG1655
<220> gmha b0223
<222> 446-545
<223> gmha b0223 U00096 243543..244121
<313> 10493: 475-494
<400> 10493
GCGGCAAAAT GGCTGGCACG GCGGATATCG AAATTCGCGT ACCGCACTTT 50
GGTTATGCCG ACCGCATTCA GGAGATTCAC ATTAAAGTGA TCCATATCCT 100
<210> 10494
<211> 100
<212> DNA
<213> Escherichia coli K-12 MG1655
<220> gmha b0223
<222> 305-404
<223> gmha b0223 U00096 243543..244121
<313> 10494: 355-374
<400> 10494
CCCGCTACGT TGAAGCGGTA GGTCGCGAAG GCGATGTACT GCTGGGGATC 50
TCCACCTCCG GTAACTCTGC AAACGTGATC AAAGCGATCG CAGCGGCGCG 100
<210> 10495
<211> 100
<212> DNA
<213> Escherichia coli K-12 MG1655
<220> fhua b0151
<222> 2139-2238
<223> fhua b0151 U00096 167484..169727
<313> 10495: 2191-2210

<400> 10495
TAACAACCTG TTCGATCGTG AATACGTCGC CAGCTGCTTT AACACTTATG 50
GCTGCTTCTG GGGCGCAGAA CGTCAGGTCG TTGCAACCGC AACCTTCCGT 100
<210> 10496
<211> 100
<212> DNA
<213> Escherichia coli K-12 MG1655
<220> fhua b0151
<222> 1969-2068
<223> fhua b0151 U00096 167484..169727
<313> 10496: 2013-2032
<400> 10496
GACGGTCCGC TTTCAGGTCT GACGCTGGGC ACCGGTGGTC GTTATACTGG 50
CTCCAGTTAT GGTGATCCGG CTAACTCCTT TAAAGTGGGA AGTTATACGG 100
<210> 10497
<211> 100
<212> DNA
<213> Escherichia coli K-12 MG1655
<220> fhua b0151
<222> 1908-2007
<223> fhua b0151 U00096 167484..169727
<313> 10497: 1952-1971
<400> 10497
CAATACGCCT GCACAGGTGC CAAAACACAT GGCTTCGTTG TGGGCTGACT 50
ACACCTTCTT TGACGGTCCG CTTTCAGGTC TGACGCTGGG CACCGGTGGT 100
<210> 10498
<211> 100
<212> DNA
<213> Escherichia coli K-12 MG1655
<220> kate b1733
<222> 2150-2249
<223> kate b1733 U00096 1811891..1814152
<313> 10498: 2200-2219
<400> 10498
GGATTGTGGA AGCTGACAGC GCTGACGGTA GTTTTATGGA TGAACTGCTA 50
ACGCTGATGG CAGCACACCG CGTGTGGTCA CGCATTCCTA AGATTGACAA 100
<210> 10499
<211> 100
<212> DNA
<213> Escherichia coli K-12 MG1655
<220> kate b1733
<222> 2083-2182
<223> kate b1733 U00096 1811891..1814152
<313> 10499: 2133-2152
<400> 10499
ATTGCGCTGG CGGGTGACGC GCGCAAGTTT AAAGCAACAA TCAAGATCGC 50
TGACCAGGGT GAAGAAGGGA TTGTGGAAGC TGACAGCGCT GACGGTAGTT 100
<210> 10500
<211> 100
<212> DNA
<213> Escherichia coli K-12 MG1655
<220> kate b1733
<222> 2022-2121
<223> kate b1733 U00096 1811891..1814152
<313> 10500: 2068-2087
<400> 10500
TATCGCTGAC AACGGCGATG CCAACTACTA CCTGATGGAA GCCTACAAAC 50
ACCTTAAACC GATTGCGCTG GCGGGTGACG CGCGCAAGTT TAAAGCAACA 100
<210> 10501
<211> 100
<212> DNA
<213> Escherichia coli K-12 MG1655
<220> fhub b0154
<222> 1884-1983
<223> fhub b0154 U00096 171462..173444
<313> 10501: 1919-1938
<400> 10501
GCGGATGGTG CTGTTTCCAT TCCAGATCCC GGCGGGGCTG CTGTCAACCT 50
TTATCGGCGC GCCATATTTT ATCTATTTGT TGAGAAAGCA GAGCCGTTAA 100
<210> 10502
<211> 100

<212> DNA
<213> Escherichia coli K-12 MG1655
<220> fhub b0154
<222> 1832-1931
<223> fhub b0154 U00096 171462..173444
<313> 10502: 1861-1880
<400> 10502
TAATTTCGGC GCTGGTGGGT GGTTTACTGC TGGTGTTCGC TGACTGGTGT 50
GGGCGGATGG TGCTGTTTCC ATTCCAGATC CCGGCGGGGC TGCTGTCAAC 100
<210> 10503
<211> 100
<212> DNA
<213> Escherichia coli K-12 MG1655
<220> fhub b0154
<222> 1716-1815
<223> fhub b0154 U00096 171462..173444
<313> 10503: 1741-1760
<400> 10503
AGCGGCTTGC CTGACGGCGA CCGCGACGAT GACTATTGGA CCGTTGAGTT 50
TTGTTGGTTT AATGGCACCG CATATTGCGC GGATGATGGG CTTTCGACGG 100
<210> 10504
<211> 100
<212> DNA
<213> Escherichia coli K-12 MG1655
<220> fhuc b0152
<222> 697-796
<223> fhuc b0152 U00096 169778..170575
<313> 10504: 726-745
<400> 10504
GCGGAAATTA TGCGCGGCGA AACCCTCGAA ATGATTTATG GCATCCCGAT 50
GGGTATTTTG CCGCATCCGG CGGGTGCTGC ACCTGTGAGT TTTGTTTATT 100
<210> 10505
<211> 100
<212> DNA
<213> Escherichia coli K-12 MG1655
<220> fhuc b0152
<222> 649-748
<223> fhuc b0152 U00096 169778..170575
<313> 10505: 694-713
<400> 10505
TATCTGGTCG CCCTGCGCGG CGGTGAAATG ATTGCTCAGG GAACGCCTGC 50
GGAAATTATG CGCGGCGAAA CCCTCGAAAT GATTTATGGC ATCCCGATGG 100
<210> 10506
<211> 100
<212> DNA
<213> Escherichia coli K-12 MG1655
<220> fhuc b0152
<222> 589-688
<223> fhuc b0152 U00096 169778..170575
<313> 10506: 623-642
<400> 10506
CGTGGCCTGA CGGTCATTGC CGTGTTGCAC GATATCAATA TGGCGGCACG 50
CTACTGTGAT TATCTGGTCG CCCTGCGCGG CGGTGAAATG ATTGCTCAGG 100
<210> 10507
<211> 100
<212> DNA
<213> Escherichia coli K-12 MG1655
<220> katg b3943
<222> 2082-2181
<223> katg b3943 U00096 4131415..4133595
<313> 10507: 2151-2170
<400> 10507
GGAAGTTTAC GCCAGTAGCG ATGCCCACGA GAAGTTTGTT AAAGACTTCG 50
TGGCGGCATG GGTGAAAGTG ATGAACCTCG ACCGTTTCGA CCTGCTGTAA 100
<210> 10508
<211> 100
<212> DNA
<213> Escherichia coli K-12 MG1655
<220> katg b3943
<222> 2043-2142
<223> katg b3943 U00096 4131415..4133595

<313> 10508: 2103-2122
<400> 10508
TCTGGTGTTT GGTTCTAACT CCGTCCTGCG TGCGGTGGCG GAAGTTTACG 50
CCAGTAGCGA TGCCCACGAG AAGTTTGTTA AAGACTTCGT GGCGGCATGG 100
<210> 10509
<211> 100
<212> DNA
<213> Escherichia coli K-12 MG1655
<220> katg b3943
<222> 1949-2048
<223> katg b3943 U00096 4131415..4133595
<313> 10509: 1993-2012
<400> 10509
GTTACGAGTG GAAAGCGACC GACGAATCGA AAGAGCTGTT CGAAGGCCGT 50
GACCGTGAAA CCGGCGAAGT GAAATTTACG GCCAGCCGTG CGGATCTGGT 100
<210> 10510
<211> 100
<212> DNA
<213> Escherichia coli K-12 MG1655
<220> fhud b0153
<222> 792-891
<223> fhud b0153 U00096 170575..171465
<313> 10510: 832-851
<400> 10510
ACGCTTTCAG CGCGTACCTG CAGTCTGGTT TTATGGTGCG ACGCTCTCGG 50
CAATGCACTT TGTGCGCGTT CTGGATAACG CCATCGGAGG TAAAGCGTGA 100
<210> 10511
<211> 100
<212> DNA
<213> Escherichia coli K-12 MG1655
<220> fhud b0153
<222> 687-786
<223> fhud b0153 U00096 170575..171465
<313> 10511: 724-743
<400> 10511
GTATAAAGAC GTTGATGTGC TCTGTTTTGA TCACGACAAC AGCAAAGACA 50
TGGATGCGCT AATGGCAACG CCGCTGTGGC AGGCCATGCC GTTTGTCCGC 100
<210> 10512
<211> 100
<212> DNA
<213> Escherichia coli K-12 MG1655
<220> fhud b0153
<222> 607-706
<223> fhud b0153 U00096 170575..171465
<313> 10512: 643-662
<400> 10512
GAGTACGGCA TCCCAAATGC CTGGCAAGGG GAAACCAACT TCTGGGGCAG 50
TACCGCCGTC AGTATCGATC GTCTGGCGGC GTATAAAGAC GTTGATGTGC 100
<210> 10513
<211> 100
<212> DNA
<213> Escherichia coli K-12 MG1655
<220> fhue b1103
<222> 2091-2190
<223> fhue b1103 U00096 complement(1158585..1160774)
<313> 10513: 2169-2188
<400> 10513
CAATAACCTG TTCGACAAAA CCTACGATAC CAACGTGGAA GGTTCTATCG 50
TCTACGGCAC ACCGCGTAAT TTCAGCATTA CCGGCACGTA TCAATTCTGA 100
<210> 10514
<211> 100
<212> DNA
<213> Escherichia coli K-12 MG1655
<220> fhue b1103
<222> 2073-2172
<223> fhue b1103 U00096 complement(1158585..1160774)
<313> 10514: 2129-2148
<400> 10514
CTCGTTACAG GGGAACGTCA ATAACCTGTT CGACAAAACC TACGATACCA 50
ACGTGGAAGG TTCTATCGTC TACGGCACAC CGCGTAATTT CAGCATTACC 100
<210> 10515

<211> 100
<212> DNA
<213> Escherichia coli K-12 MG1655
<220> fhue b1103
<222> 2030-2129
<223> fhue b1103 U00096 complement(1158585..1160774)
<313> 10515: 2072-2091
<400> 10515
CGCTGGTGGA TCTCTTCACC CGCTACCAGG TGACGAAAAA CTTCTCGTTA 50
CAGGGGAACG TCAATAACCT GTTCGACAAA ACCTACGATA CCAACGTGGA 100
<210> 10516
<211> 100
<212> DNA
<213> Escherichia coli K-12 MG1655
<220> fhuf b4368
<222> 690-789
<223> fhuf b4368 U00096 complement(4602444..4603232)
<313> 10516: 764-783
<400> 10516
TACCGTGGTA CTGCGCGACG GCCTGCTGGT GCGCCGCACT TGCTGCCAGC 50
GTTATCGCTT ACCGGATGTG CAGCAATGTG GCGATTGTAC GCTGAAATGA 100
<210> 10517
<211> 100
<212> DNA
<213> Escherichia coli K-12 MG1655
<220> fhuf b4368
<222> 615-714
<223> fhuf b4368 U00096 complement(4602444..4603232)
<313> 10517: 654-673
<400> 10517
TACTGTTGAA TCGCTGCGCC ATGCCCTCTT TTTGAGAAAA ACGCTCACGA 50
ACGGTGAAGA TAATCCACTC TGGCGTACCG TGGTACTGCG CGACGGCCTG 100
<210> 10518
<211> 100
<212> DNA
<213> Escherichia coli K-12 MG1655
<220> fhuf b4368
<222> 586-685
<223> fhuf b4368 U00096 complement(4602444..4603232)
<313> 10518: 631-650
<400> 10518
ACTGAGATGA AGCAACTGCT CGGCGAGGCT ACTGTTGAAT CGCTGCGCCA 50
TGCCCTCTTT TTTGAGAAAA CGCTCACGAA CGGTGAAGAT AATCCACTCT 100
<210> 10519
<211> 100
<212> DNA
<213> Escherichia coli K-12 MG1655
<220> fada b3846
<222> 1036-1135
<223> fada b3846 U00096 complement(4025199..4026362)
<313> 10519: 1087-1106
<400> 10519
GGTTGTTCCG GTGCGCGTAT CAGCACCACG CTGCTGAATC TGATGGAACG 50
CAAAGACGTT CAGTTTGGTC TGGCGACGAT GTGTATCGGT CTGGGTCAGG 100
<210> 10520
<211> 100
<212> DNA
<213> Escherichia coli K-12 MG1655
<220> fada b3846
<222> 950-1049
<223> fada b3846 U00096 complement(4025199..4026362)
<313> 10520: 989-1008
<400> 10520
CATGTATTAA AGATCTGGGA CTAATTGAGC AGATTGACGA GAAGATCAAC 50
CTCAACGGTG GCGCGATCGC GCTGGGTCAT CCGCTGGGTT GTTCCGGTGC 100
<210> 10521
<211> 100
<212> DNA
<213> Escherichia coli K-12 MG1655
<220> fada b3846
<222> 906-1005

<223> fada b3846 U00096 complement(4025199..4026362)
<313> 10521: 936-955
<400> 10521
CGGCGTGTTT GAAATGAACG AAGCCTTTGC CGCGCAGATC CTGCCATGTA  50
TTAAAGATCT GGGACTAATT GAGCAGATTG ACGAGAAGAT CAACCTCAAC  100
<210> 10522
<211> 100
<212> DNA
<213> Escherichia coli K-12 MG1655
<220> b2611 b2612
<222> 760-859
<223> b2611 b2612 U00096 2745907..2746773
<313> 10522: 816-835
<400> 10522
CATGAAGGAT GGCGTGGACG CCGCGTCGTC TGGTTTAACG TTGCGGGCGC  50
GGTAATTCTG ACACTGGCCT ACTTCGGCAG CCGAATTGTC CAGCAGTTAA  100
<210> 10523
<211> 100
<212> DNA
<213> Escherichia coli K-12 MG1655
<220> b2611 b2612
<222> 698-797
<223> b2611 b2612 U00096 2745907..2746773
<313> 10523: 745-764
<400> 10523
ACAAGGCTGT GCTCTCTATC GTGGCGTGGT TTGTCTATAT TGTGCTGCTG  50
TGGGGACATT ATCATGAAGG ATGGCGTGGA CGCCGCGTCG TCTGGTTTAA  100
<210> 10524
<211> 100
<212> DNA
<213> Escherichia coli K-12 MG1655
<220> b2611 b2612
<222> 645-744
<223> b2611 b2612 U00096 2745907..2746773
<313> 10524: 701-720
<400> 10524
GCTTTGCACT GGCCTGTTCT ACATGCACAA CTTATTTAGC ATGGAAAATA  50
TCGACAAGGC TGTGCTCTCT ATCGTGGCGT GGTTTGTCTA TATTGTGCTG  100
<210> 10525
<211> 100
<212> DNA
<213> Escherichia coli K-12 MG1655
<220> fadb b3847
<222> 2091-2190
<223> fadb b3847 U00096 complement(4026372..4028561)
<313> 10525: 2128-2147
<400> 10525
TGAAGTGCCG GAAGGTCTGC GTAATAAAGC GCGTCATAAC GAACCGTACT  50
ATCCTCCGGT TGAGCCAGCC CGTCCGGTTG GCGACCTGAA AACGGCTTAA  100
<210> 10526
<211> 100
<212> DNA
<213> Escherichia coli K-12 MG1655
<220> fadb b3847
<222> 2053-2152
<223> fadb b3847 U00096 complement(4026372..4028561)
<313> 10526: 2090-2109
<400> 10526
GATATGGCAC AGCAATATCA GCACCTCGGC CCGCTGTATG AAGTGCCGGA  50
AGGTCTGCGT AATAAAGCGC GTCATAACGA ACCGTACTAT CCTCCGGTTG  100
<210> 10527
<211> 100
<212> DNA
<213> Escherichia coli K-12 MG1655
<220> fadb b3847
<222> 2029-2128
<223> fadb b3847 U00096 complement(4026372..4028561)
<313> 10527: 2050-2069
<400> 10527
ACCCTCGGTA GCGCAAAATA CCTCGATATG GCACAGCAAT ATCAGCACCT  50
CGGCCCGCTG TATGAAGTGC CGGAAGGTCT GCGTAATAAA GCGCGTCATA  100

<210> 10528
<211> 100
<212> DNA
<213> Escherichia coli K-12 MG1655
<220> fadd fadD
<222> 1533-1632
<223> fadd fadD U00096 complement(1886085..1887770)
<313> 10528: 1551-1570
<400> 10528
GTCACTGGTG ACTTTTTGCC GCCGTCAGCT CACGGGATAC AAAGTACCGA 50
AGCTGGTGGA GTTTCGTGAT GAGTTACCGA AATCTAACGT CGGAAAAATT 100
<210> 10529
<211> 100
<212> DNA
<213> Escherichia coli K-12 MG1655
<220> fadd fadD
<222> 1440-1539
<223> fadd fadD U00096 complement(1886085..1887770)
<313> 10529: 1483-1502
<400> 10529
ACAGGAAGTC GCGGCTGTTG GCGTACCTTC CGGCTCCAGT GGTGAAGCGG 50
TGAAAATCTT CGTAGTGAAA AAAGATCCAT CGCTTACCGA AGAGTCACTG 100
<210> 10530
<211> 100
<212> DNA
<213> Escherichia coli K-12 MG1655
<220> fadd fadD
<222> 1365-1464
<223> fadd fadD U00096 complement(1886085..1887770)
<313> 10530: 1429-1448
<400> 10530
AGACATGATT CTGGTTTCCG GTTTTAACGT CTATCCCAAC GAGATTGAAG 50
ATGTCGTCAT GCAGCATCCT GGCGTACAGG AAGTCGCGGC TGTTGGCGTA 100
<210> 10531
<211> 100
<212> DNA
<213> Escherichia coli K-12 MG1655
<220> b2618 b2618
<222> 210-309
<223> b2618 b2618 U00096 complement(2752029..2752337)
<313> 10531: 289-308
<400> 10531
TAGTGTGCAT GATGGCGATC GGGTGGAGAT TTATCGCCCT CTCATTGCCG 50
ATCCGAAAGA GCTTCGCAGG CAACGAGCAG AAAAATCAGC GAATAAATAA 100
<210> 10532
<211> 100
<212> DNA
<213> Escherichia coli K-12 MG1655
<220> b2618 b2618
<222> 186-285
<223> b2618 b2618 U00096 complement(2752029..2752337)
<313> 10532: 243-262
<400> 10532
CAGCCGTCCG GCAAAACTAA GCGATAGTGT GCATGATGGC GATCGGGTGG 50
AGATTTATCG CCCTCTCATT GCCGATCCGA AAGAGCTTCG CAGGCAACGA 100
<210> 10533
<211> 100
<212> DNA
<213> Escherichia coli K-12 MG1655
<220> b2618 b2618
<222> 143-242
<223> b2618 b2618 U00096 complement(2752029..2752337)
<313> 10533: 202-221
<400> 10533
TGCGTACCGA TATCGATTTA ACTAAAAACA AAGTCGGCAT TTACAGCCGT 50
CCGGCAAAAC TAAGCGATAG TGTGCATGAT GGCGATCGGG TGGAGATTTA 100
<210> 10534
<211> 100
<212> DNA
<213> Escherichia coli K-12 MG1655
<220> b2619 b2620

<222> 368-467
<223> b2619 b2620 U00096 complement(2752309..2752785)
<313> 10534: 417-436
<400> 10534
TTACCAATAA GTTGATTGAA CTCGCCTTTG GTCGCGTGTT TAAAGAGCTG 50
GCGGCTAATA TGGTCCAGGC TTTTACGGTT CGTGCGAAAG AGGTTTACAG 100
<210> 10535
<211> 100
<212> DNA
<213> Escherichia coli K-12 MG1655
<220> b2619 b2620
<222> 348-447
<223> b2619 b2620 U00096 complement(2752309..2752785)
<313> 10535: 387-406
<400> 10535
GTTTCATCTC GACTTTGAGT TTACCAATAA GTTGATTGAA CTCGCCTTTG 50
GTCGCGTGTT TAAAGAGCTG GCGGCTAATA TGGTCCAGGC TTTTACGGTT 100
<210> 10536
<211> 100
<212> DNA
<213> Escherichia coli K-12 MG1655
<220> b2619 b2620
<222> 275-374
<223> b2619 b2620 U00096 complement(2752309..2752785)
<313> 10536: 300-319
<400> 10536
TGGATGGGCC GTTCAAGAAA TTGATTGGTG GATGGAAGTT TACGCCGCTG 50
AGCCAGGAGG CGTGTCGTAT CGAGTTTCAT CTCGACTTTG AGTTTACCAA 100
<210> 10537
<211> 100
<212> DNA
<213> Escherichia coli K-12 MG1655
<220> fadl fadL
<222> 1248-1347
<223> fadl fadL U00096 2459320..2460666
<313> 10537: 1312-1331
<400> 10537
TATGCACGGT CAGAGCGTGA AAATTAACGA AGGCCCATAC CAGTTCGAGT 50
CTGAAGGTAA AGCCTGGCTG TTCGGTACTA ACTTTAACTA CGCGTTCTGA 100
<210> 10538
<211> 100
<212> DNA
<213> Escherichia coli K-12 MG1655
<220> fadl fadL
<222> 1204-1303
<223> fadl fadL U00096 2459320..2460666
<313> 10538: 1261-1280
<400> 10538
TACGCATTTA ATAAAGATGC TTCAGTCGAC GTTGGTGTTT CTTATATGCA 50
CGGTCAGAGC GTGAAAATTA ACGAAGGCCC ATACCAGTTC GAGTCTGAAG 100
<210> 10539
<211> 100
<212> DNA
<213> Escherichia coli K-12 MG1655
<220> fadl fadL
<222> 1135-1234
<223> fadl fadL U00096 2459320..2460666
<313> 10539: 1194-1213
<400> 10539
GTTCCTGCAC AGAATCGTTC TATCTCCATT CCGGACCAGG ACCGTTTCTG 50
GCTGAGTGCA GGTACGACTT ACGCATTTAA TAAAGATGCT TCAGTCGACG 100
<210> 10540
<211> 100
<212> DNA
<213> Escherichia coli K-12 MG1655
<220> fadr b1188
<222> 621-720
<223> fadr b1188 U00096 1234161..1234880
<313> 10540: 683-702
<400> 10540
GGTGTACGAA ACAGTGCGTC GCTATGGGCA TGAGAGTGGC GAGATTTGGC 50

ACCGGATGCA GAAAAATCTG CCGGGTGATT TAGCCATTCA GGGGCGATAA 100
<210> 10541
<211> 100
<212> DNA
<213> Escherichia coli K-12 MG1655
<220> fadr b1188
<222> 606-705
<223> fadr b1188 U00096 1234161..1234880
<313> 10541: 622-641
<400> 10541
AGGCGCGCAC GATCAGGTGT ACGAAACAGT GCGTCGCTAT GGGCATGAGA 50
GTGGCGAGAT TTGGCACCGG ATGCAGAAAA ATCTGCCGGG TGATTTAGCC 100
<210> 10542
<211> 100
<212> DNA
<213> Escherichia coli K-12 MG1655
<220> fadr b1188
<222> 432-531
<223> fadr b1188 U00096 1234161..1234880
<313> 10542: 478-497
<400> 10542
GGATTACAAC ATATTCCGCG GCCTGGCGTT TGCTTCCGGC AACCCGATTT 50
ACGGTCTGAT TCTTAACGGG ATGAAAGGGC TGTATACGCG TATTGGTCGT 100
<210> 10543
<211> 100
<212> DNA
<213> Escherichia coli K-12 MG1655
<220> yfaa b2231
<222> 1615-1714
<223> yfaa b2231 U00096 complement(2332976..2334712)
<313> 10543: 1663-1682
<400> 10543
CAAACTTTAT TAATGCCGAA GCTGGACGCT TTATCTCAAC AACCGCGTTA 50
TGTCATGAAG CTGGCCCAGA TGGAACCCGG TGCCGCCTGG CAGTGGCTAC 100
<210> 10544
<211> 100
<212> DNA
<213> Escherichia coli K-12 MG1655
<220> yfaa b2231
<222> 1595-1694
<223> yfaa b2231 U00096 complement(2332976..2334712)
<313> 10544: 1616-1635
<400> 10544
CGGTTTTTTA TAACGCCGCA CAAACTTTAT TAATGCCGAA GCTGGACGCT 50
TTATCTCAAC AACCGCGTTA TGTCATGAAG CTGGCCCAGA TGGAACCCGG 100
<210> 10545
<211> 100
<212> DNA
<213> Escherichia coli K-12 MG1655
<220> yfaa b2231
<222> 1532-1631
<223> yfaa b2231 U00096 complement(2332976..2334712)
<313> 10545: 1575-1594
<400> 10545
CACAAGGAAT AGCGAAACTG CTGCGTAACG AAACGTTGAC CAGTCTGCCG 50
AAGAATCTCG AACCGGTTTT TTATAACGCC GCACAAACTT TATTAATGCC 100
<210> 10546
<211> 100
<212> DNA
<213> Escherichia coli K-12 MG1655
<220> yfad yfaD
<222> 801-900
<223> yfad yfaD U00096 2354924..2355823
<313> 10546: 866-885
<400> 10546
CATTAAAATT GCTTTGCGCA TGCTGGAGCA GGGCTTTGAA CGTGAGATTG 50
TGCTGGCGAC AACCCAACTC ACTGATGCTG ATATTCCGAA CTGTCATTAA 100
<210> 10547
<211> 100
<212> DNA
<213> Escherichia coli K-12 MG1655

<220> yfad yfaD
<222> 703-802
<223> yfad yfaD U00096 2354924..2355823
<313> 10547: 756-775
<400> 10547
CGTTCACCAC TACAAAAGGA GAGATTAATG ACTATTGCTG AACGGCTACG 50
GCAGGAAGGG CATCAAATTG GCTGGCAGGA AGGTATGCAT GAACAAGCCA 100
<210> 10548
<211> 100
<212> DNA
<213> Escherichia coli K-12 MG1655
<220> yfad yfaD
<222> 633-732
<223> yfad yfaD U00096 2354924..2355823
<313> 10548: 667-686
<400> 10548
ACTGTTTAAT TATCTGCTGC AATGCGGCGA TACCTCCCGT TTCACCCGTT 50
TTATTGAGGA GATTGCCGAA CGTTCACCAC TACAAAAGGA GAGATTAATG 100
<210> 10549
<211> 100
<212> DNA
<213> Escherichia coli K-12 MG1655
<220> yfae b2237
<222> 152-251
<223> yfae b2237 U00096 2346534..2346788
<313> 10549: 195-214
<400> 10549
CAGGTCAAGT TGACTGGATT GCCGAACCGT TAGCCTTTAT TCAGCCGGGG 50
GAAATTTTGC CCTGTTGTTG CCGGGCAAAA GGCGATATTG AAATCGAGAT 100
<210> 10550
<211> 100
<212> DNA
<213> Escherichia coli K-12 MG1655
<220> yfae b2237
<222> 156-255
<223> yfae b2237 U00096 2346534..2346788
<313> 10550: 179-198
<400> 10550
TCAAGTTGAC TGGATTGCCG AACCGTTAGC CTTTATTCAG CCGGGGGAAA 50
TTTTGCCCTG TTGTTGCCGG GCAAAAGGCG ATATTGAAAT CGAGATGTGA 100
<210> 10551
<211> 100
<212> DNA
<213> Escherichia coli K-12 MG1655
<220> yfae b2237
<222> 145-244
<223> yfae b2237 U00096 2346534..2346788
<313> 10551: 182-201
<400> 10551
CTGGTTGCAG GTCAAGTTGA CTGGATTGCC GAACCGTTAG CCTTTATTCA 50
GCCGGGGGAA ATTTTGCCCT GTTGTTGCCG GGCAAAAGGC GATATTGAAA 100
<210> 10552
<211> 100
<212> DNA
<213> Escherichia coli K-12 MG1655
<220> yfah b2239
<222> 66-165
<223> yfah b2239 U00096 2347707..2347913
<313> 10552: 90-109
<400> 10552
AGGTGAATTG TGTTCCGTTC CTCTCGAACC AACTTTCTAT CGACAGATTT 50
CGTTGCTGGC TAAAGAAAAG CCGGTAGAAG GCAGTCCACT GTTTTTACTA 100
<210> 10553
<211> 100
<212> DNA
<213> Escherichia coli K-12 MG1655
<220> yfah b2239
<222> 21-120
<223> yfah b2239 U00096 2347707..2347913
<313> 10553: 52-71
<400> 10553

ATTAGGCATC GCCTTACAAC CAGAGTTAAC GCTGAAAAGC ATTGCAGGTG 50
AATTGTGTTC CGTTCCTCTC GAACCAACTT TCTATCGACA GATTTCGTTG 100
<210> 10554
<211> 100
<212> DNA
<213> Escherichia coli K-12 MG1655
<220> yfah b2239
<222> 1-100
<223> yfah b2239 U00096 2347707..2347913
<313> 10554: 11-30
<400> 10554
ATGAATTTTA TTCGACAGGG ATTAGGCATC GCCTTACAAC CAGAGTTAAC 50
GCTGAAAAGC ATTGCAGGTG AATTGTGTTC CGTTCCTCTC GAACCAACTT 100
<210> 10555
<211> 100
<212> DNA
<213> Escherichia coli K-12 MG1655
<220> yfal b2234
<222> 3654-3753
<223> yfal b2234 U00096 complement(2338437..2342189)
<313> 10555: 3691-3710
<400> 10555
CAGTCAGCGA GTGTCCCTGC GCGGCAGCGT GGCGTGGCAG AAAGGGAGTG 50
ATGATTTTGC CCAGACGGCA GGGTTTTTGT CGATGACGGT GAAATGGTAA 100
<210> 10556
<211> 100
<212> DNA
<213> Escherichia coli K-12 MG1655
<220> yfal b2234
<222> 3600-3699
<223> yfal b2234 U00096 complement(2338437..2342189)
<313> 10556: 3652-3671
<400> 10556
TGACGATGCG GTGAAGCAAC GGGGTGAAAT AAAAGTGGGA GTCACGGGCA 50
ATATCAGTCA GCGAGTGTCC CTGCGCGGCA GCGTGGCGTG GCAGAAAGGG 100
<210> 10557
<211> 100
<212> DNA
<213> Escherichia coli K-12 MG1655
<220> yfal b2234
<222> 3558-3657
<223> yfal b2234 U00096 complement(2338437..2342189)
<313> 10557: 3598-3617
<400> 10557
TCCCCATTCG ACGGAAATTG AAGAGGATGG CAGCACTATC AGTGACGATG 50
CGGTGAAGCA ACGGGGTGAA ATAAAAGTGG GAGTCACGGG CAATATCAGT 100
<210> 10558
<211> 100
<212> DNA
<213> Escherichia coli K-12 MG1655
<220> yfao b2252
<222> 327-426
<223> yfao b2252 U00096 2362574..2362999
<313> 10558: 389-408
<400> 10558
AGAGTTTCAG GACTACGCGT GGGTAAAACC TGAAGATCTG GTGCATTATG 50
ATTTGAATGT CGCCACCCGA AAAACGTTAC GTTTGAAAGG TCTTCTGTAA 100
<210> 10559
<211> 100
<212> DNA
<213> Escherichia coli K-12 MG1655
<220> yfao b2252
<222> 250-349
<223> yfao b2252 U00096 2362574..2362999
<313> 10559: 302-321
<400> 10559
GGTCGCAAGG AAGAGATTTA TATGATTTAC CTGATTTTTG ACTGCGTTTC 50
TGCCAACCGA GAAGTGAAAA TAAACGAAGA GTTTCAGGAC TACGCGTGGG 100
<210> 10560
<211> 100
<212> DNA

<213> Escherichia coli K-12 MG1655
<220> yfao b2252
<222> 185-284
<223> yfao b2252 U00096 2362574..2362999
<313> 10560: 234-253
<400> 10560
TGCTTTTGAC AGAAATCACG CCGTGGACCT TCAGCGATGA TATTCGCACC 50
AAGACGTATG CAGATGGTCG CAAGGAAGAG ATTTATATGA TTTACCTGAT 100
<210> 10561
<211> 100
<212> DNA
<213> Escherichia coli K-12 MG1655
<220> sun b3290
<222> 1191-1290
<223> sun b3290 U00096 3432844..3434133
<313> 10561: 1264-1283
<400> 10561
ACTTTGCGAA ACAGGAACAC CAGAGCAACC GGGTAAACAA AATCTACCTG 50
GTGCCGAAGA GGGCGACGGC TTCTTTTACG CTAAGCTAAT CAAAAAGTGA 100
<210> 10562
<211> 100
<212> DNA
<213> Escherichia coli K-12 MG1655
<220> sun b3290
<222> 1184-1283
<223> sun b3290 U00096 3432844..3434133
<313> 10562: 1202-1221
<400> 10562
ATGCCGAACT TTGCGAAACA GGAACACCAG AGCAACCGGG TAAACAAAAT 50
CTACCTGGTG CCGAAGAGGG CGACGGCTTC TTTTACGCTA AGCTAATCAA 100
<210> 10563
<211> 100
<212> DNA
<213> Escherichia coli K-12 MG1655
<220> sun b3290
<222> 1143-1242
<223> sun b3290 U00096 3432844..3434133
<313> 10563: 1174-1193
<400> 10563
GAATAGCCTG CAGATTAAAG CCTTTTTGCA ACGTACCGCT GATGCCGAAC 50
TTTGCGAAAC AGGAACACCA GAGCAACCGG GTAAACAAAA TCTACCTGGT 100
<210> 10564
<211> 100
<212> DNA
<213> Escherichia coli K-12 MG1655
<220> yfbb b2264
<222> 651-750
<223> yfbb b2264 U00096 complement(2374854..2375612)
<313> 10564: 694-713
<400> 10564
GGCGGAACTG GCTGCCGACT GCCATGTCAT TCCTCGCGCC GGACATAACG 50
CGCATCGGGA AAATCCCGCT GGCGTAATCG CAAGTCTGGC GCAGATCTTG 100
<210> 10565
<211> 100
<212> DNA
<213> Escherichia coli K-12 MG1655
<220> yfbb b2264
<222> 570-669
<223> yfbb b2264 U00096 complement(2374854..2375612)
<313> 10565: 615-634
<400> 10565
TTTACGTGCT AACCTTAGCG CCCGCACATT TGCGTTTTAT TATTTATGTG 50
GTGAACGTGA CAGCAAATTC CGCGCCCTGG CGGCGGAACT GGCTGCCGAC 100
<210> 10566
<211> 100
<212> DNA
<213> Escherichia coli K-12 MG1655
<220> yfbb b2264
<222> 502-601
<223> yfbb b2264 U00096 complement(2374854..2375612)
<313> 10566: 553-572

<400> 10566
CGCAGCAACA ATAATGGCGC AACGCTTGCC GCCATGCTGG AGGCGACTTC 50
TCTCGCCGTC CAGCCTGATT TACGTGCTAA CCTTAGCGCC CGCACATTTG 100
<210> 10567
<211> 100
<212> DNA
<213> Escherichia coli K-12 MG1655
<220> yfbk b2271
<222> 1626-1725
<223> yfbk b2271 U00096 complement(2382015..2383742)
<313> 10567: 1655-1674
<400> 10567
ACAGTGGGCT CAGCAGGCAA AAGGGGAAGA TCCACAGGGT TACAGGGCGG 50
AATTTATTCG CCTGATTGAA CTGGCGGATG GTGTGACTGA CATCAGTCAG 100
<210> 10568
<211> 100
<212> DNA
<213> Escherichia coli K-12 MG1655
<220> yfbk b2271
<222> 1551-1650
<223> yfbk b2271 U00096 complement(2382015..2383742)
<313> 10568: 1602-1621
<400> 10568
CGCAGCAGTA GCTGCATATG GGCAAAAGTT ACGCGGTTCT GAATACCTGA 50
ACAATACCTC CTGGCAGCAG ATCAAACAGT GGGCTCAGCA GGCAAAAGGG 100
<210> 10569
<211> 100
<212> DNA
<213> Escherichia coli K-12 MG1655
<220> yfbk b2271
<222> 1478-1577
<223> yfbk b2271 U00096 complement(2382015..2383742)
<313> 10569: 1531-1550
<400> 10569
GAAAAGAAAG TCAGTTAGTT GAATTCCCGC TGGGGCCAAC AATAAACGCG 50
CCCTCTGAAG ATATGCGTTT TCGCGCAGCA GTAGCTGCAT ATGGGCAAAA 100
<210> 10570
<211> 100
<212> DNA
<213> Escherichia coli K-12 MG1655
<220> tesa b0495
<222> 514-613
<223> tesa b0495 U00096 complement(518363..518989)
<313> 10570: 567-586
<400> 10570
CTCAAGCCAC AATGGATGCA GGATGACGGT ATTCATCCCA ACCGCGACGC 50
CCAGCCGTTT ATTGCCGACT GGATGGCGAA GCAGTTGCAG CCTTTAGTAA 100
<210> 10571
<211> 100
<212> DNA
<213> Escherichia coli K-12 MG1655
<220> tesa b0495
<222> 491-590
<223> tesa b0495 U00096 complement(518363..518989)
<313> 10571: 514-533
<400> 10571
CCTTTTTTAT GGAAGAGGTC TACCTCAAGC CACAATGGAT GCAGGATGAC 50
GGTATTCATC CCAACCGCGA CGCCCAGCCG TTTATTGCCG ACTGGATGGC 100
<210> 10572
<211> 100
<212> DNA
<213> Escherichia coli K-12 MG1655
<220> tesa b0495
<222> 450-549
<223> tesa b0495 U00096 complement(518363..518989)
<313> 10572: 479-498
<400> 10572
TTACCCCAAA CTCGCCAAAG AGTTTGATGT TCCGCTGCTG CCCTTTTTTA 50
TGGAAGAGGT CTACCTCAAG CCACAATGGA TGCAGGATGA CGGTATTCAT 100
<210> 10573
<211> 100

<212> DNA
<213> Escherichia coli K-12 MG1655
<220> yfbl b2272
<222> 878-977
<223> yfbl b2272 U00096 2383874..2384851
<313> 10573: 903-922
<400> 10573
AACAATACCA CTTGCCCGGT GATACCGCAG ACAGATTGAA TTATCAGAAA 50
ATGGCTCAGG TAGTGGATGG TGTTATAACT TTGTTATACA ACAGTAAATA 100
<210> 10574
<211> 100
<212> DNA
<213> Escherichia coli K-12 MG1655
<220> yfbl b2272
<222> 806-905
<223> yfbl b2272 U00096 2383874..2384851
<313> 10574: 839-858
<400> 10574
CTGACCACCT GAATTATTGG CAACACGATA TTCCCGCCAT AATGATTACT 50
GACACCGCTT TTTATCGTAA TAAACAATAC CACTTGCCCG GTGATACCGC 100
<210> 10575
<211> 100
<212> DNA
<213> Escherichia coli K-12 MG1655
<220> yfbl b2272
<222> 763-862
<223> yfbl b2272 U00096 2383874..2384851
<313> 10575: 786-805
<400> 10575
TATTCTATGA ATACCCCAGG GTTTATTCCC GGTATTGATT TCTCTGACCA 50
CCTGAATTAT TGGCAACACG ATATTCCCGC CATAATGATT ACTGACACCG 100
<210> 10576
<211> 100
<212> DNA
<213> Escherichia coli K-12 MG1655
<220> tesb b0453
<222> 762-861
<223> tesb b0453 U00096 complement(473525..474385)
<313> 10576: 824-843
<400> 10576
GTCCAGCGCA CGTGGCTTTG TGCGCGGTGA GTTTTATACC CAAGACGGCG 50
TACTGGTTGC CTCGACCGTT CAGGAAGGGG TGATGCGTAA TCACAATTAA 100
<210> 10577
<211> 100
<212> DNA
<213> Escherichia coli K-12 MG1655
<220> tesb b0453
<222> 658-757
<223> tesb b0453 U00096 complement(473525..474385)
<313> 10577: 705-724
<400> 10577
CTCGAACCGG GGATTCAGAT TGCCACCATT GACCATTCCA TGTGGTTCCA 50
TCGCCCGTTT AATTTGAATG AATGGCTGCT GTATAGCGTG GAGAGCACCT 100
<210> 10578
<211> 100
<212> DNA
<213> Escherichia coli K-12 MG1655
<220> tesb b0453
<222> 624-723
<223> tesb b0453 U00096 complement(473525..474385)
<313> 10578: 673-692
<400> 10578
GCCGGTAGCT CTACAGCCGC ACGGCATCGG TTTTCTCGAA CCGGGGATTC 50
AGATTGCCAC CATTGACCAT TCCATGTGGT TCCATCGCCC GTTTAATTTG 100
<210> 10579
<211> 100
<212> DNA
<213> Escherichia coli K-12 MG1655
<220> yfbm b2273
<222> 405-504
<223> yfbm b2273 U00096 2384954..2385457

<313> 10579: 480-499
<400> 10579
ACTCTTTGCC TCGATCATGC TTGATATGGA AAAGCTGATT AGCGCGTATC 50
GACGTATGCT GCGACAAGGG AATCATGCGC TGACGGTTAT TGTTGGGTGA 100
<210> 10580
<211> 100
<212> DNA
<213> Escherichia coli K-12 MG1655
<220> yfbm b2273
<222> 383-482
<223> yfbm b2273 U00096 2384954..2385457
<313> 10580: 404-423
<400> 10580
TTTCAGAAGA GTTAGAGGGG CAACTCTTTG CCTCGATCAT GCTTGATATG 50
GAAAAGCTGA TTAGCGCGTA TCGACGTATG CTGCGACAAG GGAATCATGC 100
<210> 10581
<211> 100
<212> DNA
<213> Escherichia coli K-12 MG1655
<220> yfbm b2273
<222> 296-395
<223> yfbm b2273 U00096 2384954..2385457
<313> 10581: 363-382
<400> 10581
GACTGGAATC ACTGGATCGA AATGAACTGC GTAAACAGTT TTCCATTAAG 50
CGACTCAATG AAATGGAGAT TTATCCGGGA GTGACCTTTT CAGAAGAGTT 100
<210> 10582
<211> 100
<212> DNA
<213> Escherichia coli K-12 MG1655
<220> b1903 b1903
<222> 240-339
<223> b1903 b1903 U00096 1985467..1985805
<313> 10582: 314-333
<400> 10582
GAATTTGTTT GATAACCAAC TGGCAAGCAA CCACATCAGA AACCAATTCC 50
TGATCGGCAT TCAGCGGCTG GGACATCGTA AACTCCTGAT AGCATTTTGA 100
<210> 10583
<211> 100
<212> DNA
<213> Escherichia coli K-12 MG1655
<220> b1903 b1903
<222> 214-313
<223> b1903 b1903 U00096 1985467..1985805
<313> 10583: 262-281
<400> 10583
GGGCGCGATT ACATCGAGAA CATCCAGAAT TTGTTTGATA ACCAACTGGC 50
AAGCAACCAC ATCAGAAACC AATTCCTGAT CGGCATTCAG CGGCTGGGAC 100
<210> 10584
<211> 100
<212> DNA
<213> Escherichia coli K-12 MG1655
<220> b1903 b1903
<222> 178-277
<223> b1903 b1903 U00096 1985467..1985805
<313> 10584: 218-237
<400> 10584
CTTCAGCTGA CTGGACATTT TTTCACGAAC TTCAACGGGC GCGATTACAT 50
CGAGAACATC CAGAATTTGT TTGATAACCA ACTGGCAAGC AACCACATCA 100
<210> 10585
<211> 100
<212> DNA
<213> Escherichia coli K-12 MG1655
<220> yfbn yfbN
<222> 517-616
<223> yfbn yfbN U00096 complement(2385730..2386446)
<313> 10585: 528-547
<400> 10585
AAAATGGTTA CTGCAAGAAT CCGATATGAT GAGCTAAAAG ACAAGGCCGA 50
AAATTTTCAC GAGATTACAG AGGATGACAT ATTCAAAATG CTTATAATAC 100
<210> 10586

<211> 100
<212> DNA
<213> Escherichia coli K-12 MG1655
<220> yfbn yfbN
<222> 395-494
<223> yfbn yfbN U00096 complement(2385730..2386446)
<313> 10586: 416-435
<400> 10586
TTATGCTGGT TTATGAATGG CATGAATATG TTGGTTGGGA AGGAATGGAG 50
TGTGGGATAT TTTTTGAAAG TGATCATACT CACCAGCAAT TGCAAGCTTT 100
<210> 10587
<211> 100
<212> DNA
<213> Escherichia coli K-12 MG1655
<220> yfbn yfbN
<222> 246-345
<223> yfbn yfbN U00096 complement(2385730..2386446)
<313> 10587: 281-300
<400> 10587
ACTGACGGAA TTGATTTTGC GTAAAAATAA GGATGCCGGA GATGCCTTAA 50
ACAATATAGC ATTGGCGATT GAATATGAGT GGGATCTCAA GCGTAAGTTT 100
<210> 10588
<211> 100
<212> DNA
<213> Escherichia coli K-12 MG1655
<220> b1904 b1905
<222> 225-324
<223> b1904 b1905 U00096 1986245..1986568
<313> 10588: 299-318
<400> 10588
AGTAGGCAAC TGTAGCCTTT TTGCGGGTTC CTTATGTTTG AATACGGAAT 50
TCACTTTGTC ATATCAGTGC CATCATTCCG CCTTCCCTGT CTTTCTCTAA 100
<210> 10589
<211> 100
<212> DNA
<213> Escherichia coli K-12 MG1655
<220> b1904 b1905
<222> 180-279
<223> b1904 b1905 U00096 1986245..1986568
<313> 10589: 232-251
<400> 10589
ATGCCAGCAG GAAGGCTATA CCCACGCGGT CCCCTTTGGT CAGCCAGTAG 50
GCAACTGTAG CCTTTTTGCG GGTTCCTTAT GTTTGAATAC GGAATTCACT 100
<210> 10590
<211> 100
<212> DNA
<213> Escherichia coli K-12 MG1655
<220> b1904 b1905
<222> 140-239
<223> b1904 b1905 U00096 1986245..1986568
<313> 10590: 194-213
<400> 10590
ATACCGATCG TTATGTCAGT CGCGGGATCG CCGATCGCGC ATGCCAGCAG 50
GAAGGCTATA CCCACGCGGT CCCCTTTGGT CAGCCAGTAG GCAACTGTAG 100
<210> 10591
<211> 100
<212> DNA
<213> Escherichia coli K-12 MG1655
<220> b2635 b2635
<222> 110-209
<223> b2635 b2635 U00096 2768310..2768702
<313> 10591: 153-172
<400> 10591
GGAATAGCGC AGGCGTTGTT ATCAACCCGG CAACATGGAG GAGCCCAGTG 50
CCCGTTATTG CCATTATCGC CATTGTTATC ATCGTCATCA TTCTGAACAA 100
<210> 10592
<211> 100
<212> DNA
<213> Escherichia coli K-12 MG1655
<220> b2635 b2635
<222> 101-200

<223> b2635 b2635 U00096 2768310..2768702
<313> 10592: 138-157
<400> 10592
CAGCCCACCG GAATAGCGCA GGCGTTGTTA TCAACCCGGC AACATGGAGG 50
AGCCCAGTGC CCGTTATTGC CATTATCGCC ATTGTTATCA TCGTCATCAT 100
<210> 10593
<211> 100
<212> DNA
<213> Escherichia coli K-12 MG1655
<220> b2635 b2635
<222> 99-198
<223> b2635 b2635 U00096 2768310..2768702
<313> 10593: 126-145
<400> 10593
GACAGCCCAC CGGAATAGCG CAGGCGTTGT TATCAACCCG GCAACATGGA 50
GGAGCCCAGT GCCCGTTATT GCCATTATCG CCATTGTTAT CATCGTCATC 100
<210> 10594
<211> 100
<212> DNA
<213> Escherichia coli K-12 MG1655
<220> b2636 b2637
<222> 594-693
<223> b2636 b2637 U00096 2768453..2769145
<313> 10594: 656-675
<400> 10594
TACGGACCCG TATACGAAAG AAAAATACTT CGAATATTCC CATGACGGCA 50
AACTGATGGG ACGGCTGCTG ATACCGTCAA ACCCTGATAA CCGGGAATAA 100
<210> 10595
<211> 100
<212> DNA
<213> Escherichia coli K-12 MG1655
<220> b2636 b2637
<222> 546-645
<223> b2636 b2637 U00096 2768453..2769145
<313> 10595: 577-596
<400> 10595
GAATGCGCTT TGTATTGCCG ACAATATCAA ACTGGAATAC CACGAAGTTA 50
CGGACCCGTA TACGAAAGAA AAATACTTCG AATATTCCCA TGACGGCAAA 100
<210> 10596
<211> 100
<212> DNA
<213> Escherichia coli K-12 MG1655
<220> b2636 b2637
<222> 477-576
<223> b2636 b2637 U00096 2768453..2769145
<313> 10596: 540-559
<400> 10596
CGATGGTTGC GAGGGTGGCA GTTACTACTA TCTGGTAGAT ATGGAGGAGA 50
AAACCGTCCA GCCGCTGATG AATGCGCTTT GTATTGCCGA CAATATCAAA 100
<210> 10597
<211> 100
<212> DNA
<213> Escherichia coli K-12 MG1655
<220> yfbs b2293
<222> 1734-1833
<223> yfbs b2293 U00096 complement(2407540..2409372)
<313> 10597: 1797-1816
<400> 10597
AAATTACAGC TTCAGTGACT TTGTGAAGTT GGGGGTACCG TTCACCATTA 50
TCGTGATGGC GGTTTGTGTG GTGATGATCC CGATGCTGTT TCCGTTTTGA 100
<210> 10598
<211> 100
<212> DNA
<213> Escherichia coli K-12 MG1655
<220> yfbs b2293
<222> 1678-1777
<223> yfbs b2293 U00096 complement(2407540..2409372)
<313> 10598: 1717-1736
<400> 10598
GCCGCCTTTA TGACACCGGT TTCTTCACCT GTTAACACAC TGGTTTTAGG 50
TCCGGGAAAT TACAGCTTCA GTGACTTTGT GAAGTTGGGG GTACCGTTCA 100

<210> 10599
<211> 100
<212> DNA
<213> Escherichia coli K-12 MG1655
<220> yfbs b2293
<222> 1551-1650
<223> yfbs b2293 U00096 complement(2407540..2409372)
<313> 10599: 1597-1616
<400> 10599
GGTTATTGGG CTATTTATCT CTAATACCGC GACGGCGGTG TTGATGGCTC 50
CGATTGCGCT GGCTGCTGCC AAAACGATGG GGGTGTCGCC TTATCCATTC 100
<210> 10600
<211> 100
<212> DNA
<213> Escherichia coli K-12 MG1655
<220> b2638 b2638
<222> 152-251
<223> b2638 b2638 U00096 complement(2769861..2770175)
<313> 10600: 193-212
<400> 10600
TCTTCAGTGA CAAAATCATC GAGTGCATCA GGGAGCAATG TCACCTGATG 50
CCTGCCTTGT CCTTGAATGT GATTGGCCAT GTTGATATCC GCAGTATTCA 100
<210> 10601
<211> 100
<212> DNA
<213> Escherichia coli K-12 MG1655
<220> b2638 b2638
<222> 74-173
<223> b2638 b2638 U00096 complement(2769861..2770175)
<313> 10601: 111-130
<400> 10601
ACGGCAACGA ACCGACAATA ATTAATTATC TCGATATGCC ACCGACCCGT 50
GATGAACTGA TTAAACTTAT TTCAGATATC TTCAGTGACA AAATCATCGA 100
<210> 10602
<211> 100
<212> DNA
<213> Escherichia coli K-12 MG1655
<220> b2638 b2638
<222> 46-145
<223> b2638 b2638 U00096 complement(2769861..2770175)
<313> 10602: 66-85
<400> 10602
CGCAACACGC TGGAGATGCT TCATAACAAC GGCAACGAAC CGACAATAAT 50
TAATTATCTC GATATGCCAC CGACCCGTGA TGAACTGATT AAACTTATTT 100
<210> 10603
<211> 100
<212> DNA
<213> Escherichia coli K-12 MG1655
<220> yfbt yfbT
<222> 570-669
<223> yfbt yfbT U00096 complement(2409459..2410127)
<313> 10603: 621-640
<400> 10603
GGCAGATACC CCGCGCCTGA ATGAGGTCGA TTTGGTCCTC CACAGTCTGG 50
AGCAAATTAC TGTGACCAAA CAGCCAAATG GCGATGTTAT TATTCAGTGA 100
<210> 10604
<211> 100
<212> DNA
<213> Escherichia coli K-12 MG1655
<220> yfbt yfbT
<222> 530-629
<223> yfbt yfbT U00096 complement(2409459..2410127)
<313> 10604: 569-588
<400> 10604
GCCTGGCGGC GGGTTGTCAT GTCATTGCGG TTAACGCTCC GGCAGATACC 50
CCGCGCCTGA ATGAGGTCGA TTTGGTCCTC CACAGTCTGG AGCAAATTAC 100
<210> 10605
<211> 100
<212> DNA
<213> Escherichia coli K-12 MG1655
<220> yfbt yfbT

<222> 505-604
<223> yfbt yfbT U00096 complement(2409459..2410127)
<313> 10605: 530-549
<400> 10605
GATGCTCCCG CTGGCGTGCT TTCTGGCCTG GCGGCGGGTT GTCATGTCAT 50
TGCGGTTAAC GCTCCGGCAG ATACCCCGCG CCTGAATGAG GTCGATTTGG 100
<210> 10606
<211> 100
<212> DNA
<213> Escherichia coli K-12 MG1655
<220> b2639 b2639
<222> 382-481
<223> b2639 b2639 U00096 complement(2770188..2770706)
<313> 10606: 432-451
<400> 10606
CTGTGGCTGC ATGTGCTTGC CCAGAAAAAT ATGACAATCA CTTGGGGATA 50
TTACTTCCGT ACCGGCATTG TCATGACTGT GCCGGTGCTG TTTGTCACTC 100
<210> 10607
<211> 100
<212> DNA
<213> Escherichia coli K-12 MG1655
<220> b2639 b2639
<222> 166-265
<223> b2639 b2639 U00096 complement(2770188..2770706)
<313> 10607: 213-232
<400> 10607
GCAGACAAGG GGTTATGGGC AGCGACGTTC GGCACCGGCT TCTTGACGGC 50
ATTTCTCTCA TCAGTGATGA ACAATATGCC GACGGTGCTG ATTGGTGCGC 100
<210> 10608
<211> 100
<212> DNA
<213> Escherichia coli K-12 MG1655
<220> b2639 b2639
<222> 1-100
<223> b2639 b2639 U00096 complement(2770188..2770706)
<313> 10608: 22-41
<400> 10608
GTGCTGTTTG TGGTGGCGAA AAGAGGTCAT TCGATCAACA CCGGAAAAGT 50
CCTGCGCGGT GCGCCATGGC AGATCGTTAT TTTCTCACTG GGCATGTACA 100
<210> 10609
<211> 100
<212> DNA
<213> Escherichia coli K-12 MG1655
<220> yfca b2328
<222> 711-810
<223> yfca b2328 U00096 complement(2442771..2443580)
<313> 10609: 785-804
<400> 10609
GATCCGCCCG ATGATTGTTA TCGTCTCGGC GGTGATGAGT GCCAAATTAC 50
TTTACGACAG CCACGGACAG GAGATCCTCC ACTGGTTGGG GATGAACTAA 100
<210> 10610
<211> 100
<212> DNA
<213> Escherichia coli K-12 MG1655
<220> yfca b2328
<222> 703-802
<223> yfca b2328 U00096 complement(2442771..2443580)
<313> 10610: 742-761
<400> 10610
CAAAAGCTGA TCCGCCCGAT GATTGTTATC GTCTCGGCGG TGATGAGTGC 50
CAAATTACTT TACGACAGCC ACGGACAGGA GATCCTCCAC TGGTTGGGGA 100
<210> 10611
<211> 100
<212> DNA
<213> Escherichia coli K-12 MG1655
<220> yfca b2328
<222> 655-754
<223> yfca b2328 U00096 complement(2442771..2443580)
<313> 10611: 707-726
<400> 10611
CAGTTCCTCG GCGCGCGCAT GGGTTCACGA CTGGTGTTGA GCAAAGGACA 50

AAAGCTGATC CGCCCGATGA TTGTTATCGT CTCGGCGGTG ATGAGTGCCA 100
<210> 10612
<211> 100
<212> DNA
<213> Escherichia coli K-12 MG1655
<220> yfcb yfcB
<222> 1164-1263
<223> yfcb yfcB U00096 complement(2445528..2446793)
<313> 10612: 1224-1243
<400> 10612
GTTCACCTGG CTGGAGTTTG ATAACGGCGG CGATGGTGTG TTTATGCTCA 50
CCAAAGAGCA GCTTATTGCC GCACGAGAAC ATTTCGCGAT TTATAAAGAT 100
<210> 10613
<211> 100
<212> DNA
<213> Escherichia coli K-12 MG1655
<220> yfcb yfcB
<222> 1138-1237
<223> yfcb yfcB U00096 complement(2445528..2446793)
<313> 10613: 1177-1196
<400> 10613
CTTATGGAAC AATATCCGGA TGTTCCGTTC ACCTGGCTGG AGTTTGATAA 50
CGGCGGCGAT GGTGTGTTTA TGCTCACCAA AGAGCAGCTT ATTGCCGCAC 100
<210> 10614
<211> 100
<212> DNA
<213> Escherichia coli K-12 MG1655
<220> yfcb yfcB
<222> 1089-1188
<223> yfcb yfcB U00096 complement(2445528..2446793)
<313> 10614: 1128-1147
<400> 10614
TGCTGATGAT GGCGTGTTGA TTTGTGAAGT CGGCAACAGC ATGGTACATC 50
TTATGGAACA ATATCCGGAT GTTCCGTTCA CCTGGCTGGA GTTTGATAAC 100
<210> 10615
<211> 100
<212> DNA
<213> Escherichia coli K-12 MG1655
<220> yfcc yfcC
<222> 1443-1542
<223> yfcc yfcC U00096 2415080..2416621
<313> 10615: 1485-1504
<400> 10615
CTTCCGTAAC TGGCTGAAGG TGGGCGCGAC CCTACTTGGA CTGCTGTTTA 50
TTATGTCCAG CGTCGTAGTG ATCGGCGCTC AGTTGATGGG CTACCACTAA 100
<210> 10616
<211> 100
<212> DNA
<213> Escherichia coli K-12 MG1655
<220> yfcc yfcC
<222> 1398-1497
<223> yfcc yfcC U00096 2415080..2416621
<313> 10616: 1443-1462
<400> 10616
AACCTCAGCG TCGTTAATGG CAACGCTCGG TGTGTGCAGG GTGGACTTCC 50
GTAACTGGCT GAAGGTGGGC GCGACCCTAC TTGGACTGCT GTTTATTATG 100
<210> 10617
<211> 100
<212> DNA
<213> Escherichia coli K-12 MG1655
<220> yfcc yfcC
<222> 1358-1457
<223> yfcc yfcC U00096 2415080..2416621
<313> 10617: 1382-1401
<400> 10617
CGTTCCAGTT TGGTGATGGC TTCAGTCACA TCATTTACCC AACCTCAGCG 50
TCGTTAATGG CAACGCTCGG TGTGTGCAGG GTGGACTTCC GTAACTGGCT 100
<210> 10618
<211> 100
<212> DNA
<213> Escherichia coli K-12 MG1655

<220> yfce b2301
<222> 284-383
<223> yfce b2301 U00096 complement(2417254..2417808)
<313> 10618: 349-368
<400> 10618
TACTGGAAAA ACAACGTCTG TTTTTGACGC ATGGTCATCT TTTTGGCCCG  50
GAAAATCTGC CTGCTTTAAA CCAGAACGAT GTGCTGGTGT ACGGTCATAC  100
<210> 10619
<211> 100
<212> DNA
<213> Escherichia coli K-12 MG1655
<220> yfce b2301
<222> 216-315
<223> yfce b2301 U00096 complement(2417254..2417808)
<313> 10619: 240-259
<400> 10619
CAACTGCGAC AGCGAAGTGG ATCAAATGCT GCTGCATTTC CCGATAACCG  50
CGCCGTGGCA ACAGGTATTA CTGGAAAAAC AACGTCTGTT TTTGACGCAT  100
<210> 10620
<211> 100
<212> DNA
<213> Escherichia coli K-12 MG1655
<220> yfce b2301
<222> 156-255
<223> yfce b2301 U00096 complement(2417254..2417808)
<313> 10620: 173-192
<400> 10620
GCCAGCCAAA GTCGCTGAAC GGCTTAATGA AGTGGCACAT AAGGTTATCG  50
CTGTGCGCGG CAACTGCGAC AGCGAAGTGG ATCAAATGCT GCTGCATTTC  100
<210> 10621
<211> 100
<212> DNA
<213> Escherichia coli K-12 MG1655
<220> yfcf b2302
<222> 546-645
<223> yfcf b2302 U00096 complement(2417861..2418505)
<313> 10621: 617-636
<400> 10621
CGATGAGGTG CCAGAACGCC TGGTGGATTA TGCAACATTC CAGTGGCAGC  50
GAGCGTCTGT CCAGCGTTTT ATTGCACTTT CGGCGAAGCA ATCTGGCTGA  100
<210> 10622
<211> 100
<212> DNA
<213> Escherichia coli K-12 MG1655
<220> yfcf b2302
<222> 518-617
<223> yfcf b2302 U00096 complement(2417861..2418505)
<313> 10622: 570-589
<400> 10622
TAATGATTAA CCGCCTGGTA CTACATGGCG ATGAGGTGCC AGAACGCCTG  50
GTGGATTATG CAACATTCCA GTGGCAGCGA GCGTCTGTCC AGCGTTTTAT  100
<210> 10623
<211> 100
<212> DNA
<213> Escherichia coli K-12 MG1655
<220> yfcf b2302
<222> 216-315
<223> yfcf b2302 U00096 complement(2417861..2418505)
<313> 10623: 259-278
<400> 10623
TTCTGCCATT GCGGAGTATC TGGAAGATCG ATTTGCGCCA CCGACCTGGG  50
AACGTATTTA TCCGCTTGAT TTAGAAAATC GTGCGCGTGC ACGACAGATT  100
<210> 10624
<211> 100
<212> DNA
<213> Escherichia coli K-12 MG1655
<220> yfcg b2303
<222> 549-648
<223> yfcg b2303 U00096 2418641..2419288
<313> 10624: 605-624
<400> 10624

TCCGGCAGTC AAGAACTGGC ATGAGCGGAT CCGTTCGCGC CCTGCCACCG 50
GGCAGGCACT GCTAAAAGCA CAACTCGGTG ATGAGCGTTC GGATAGTTAA 100
<210> 10625
<211> 100
<212> DNA
<213> Escherichia coli K-12 MG1655
<220> yfcg b2303
<222> 488-587
<223> yfcg b2303 U00096 2418641..2419288
<313> 10625: 543-562
<400> 10625
ATATTGCCTG CTGGCCGTGG GTTAATGCCT GGACTCGCCA GCGAATTGAC 50
CTAGCAATGT ATCCGGCAGT CAAGAACTGG CATGAGCGGA TCCGTTCGCG 100
<210> 10626
<211> 100
<212> DNA
<213> Escherichia coli K-12 MG1655
<220> yfcg b2303
<222> 455-554
<223> yfcg b2303 U00096 2418641..2419288
<313> 10626: 487-506
<400> 10626
CCTGGCTGGG AGGCGAGAAC TACAGCATTG CGGATATTGC CTGCTGGCCG 50
TGGGTTAATG CCTGGACTCG CCAGCGAATT GACCTAGCAA TGTATCCGGC 100
<210> 10627
<211> 100
<212> DNA
<213> Escherichia coli K-12 MG1655
<220> xapa b2408
<222> 645-744
<223> xapa b2408 U00096 complement(2522065..2522898)
<313> 10627: 698-717
<400> 10627
GTCTGTGGTG CCTGAGGTTA TTTCAGCTCG CCATTGCGAC CTTAAAGTCG 50
TTGCGGTCTC TGCGATTACC AATATGGCGG AAGGTTTAAG CGATGTGAAA 100
<210> 10628
<211> 100
<212> DNA
<213> Escherichia coli K-12 MG1655
<220> xapa b2408
<222> 616-715
<223> xapa b2408 U00096 complement(2522065..2522898)
<313> 10628: 660-679
<400> 10628
CAAATTATTG GTGGGGATGT TGTTGGTATG TCTGTGGTGC CTGAGGTTAT 50
TTCAGCTCGC CATTGCGACC TTAAAGTCGT TGCGGTCTCT GCGATTACCA 100
<210> 10629
<211> 100
<212> DNA
<213> Escherichia coli K-12 MG1655
<220> xapa b2408
<222> 594-693
<223> xapa b2408 U00096 complement(2522065..2522898)
<313> 10629: 619-638
<400> 10629
TGCGGCGGAA ATTCGCATGA TGCAAATTAT TGGTGGGGAT GTTGTTGGTA 50
TGTCTGTGGT GCCTGAGGTT ATTTCAGCTC GCCATTGCGA CCTTAAAGTC 100
<210> 10630
<211> 100
<212> DNA
<213> Escherichia coli K-12 MG1655
<220> xapb b2407
<222> 1158-1257
<223> xapb b2407 U00096 complement(2520749..2522005)
<313> 10630: 1217-1236
<400> 10630
GTTTGCAGGA TATGCTCTTT TTCTCGCAGT GATATTTTTC TTTGGGTTTA 50
AATATAATCA TGACCCTGAA AAGATAAAGC ATCGAGCGGT GACTCATTAA 100
<210> 10631
<211> 100
<212> DNA

<213> Escherichia coli K-12 MG1655
<220> xapb b2407
<222> 1114-1213
<223> xapb b2407 U00096 complement(2520749..2522005)
<313> 10631: 1167-1186
<400> 10631
TTTTCGGTGG ATGGCGTAAA AGACTGGCAA ACTATCTGGC TGGTGTTTGC 50
AGGATATGCT CTTTTTCTCG CAGTGATATT TTTCTTTGGG TTTAAATATA 100
<210> 10632
<211> 100
<212> DNA
<213> Escherichia coli K-12 MG1655
<220> xapb b2407
<222> 1085-1184
<223> xapb b2407 U00096 complement(2520749..2522005)
<313> 10632: 1124-1143
<400> 10632
CGATTCTGAG TGGCATGGCA GTAGATTACT TTTCGGTGGA TGGCGTAAAA 50
GACTGGCAAA CTATCTGGCT GGTGTTTGCA GGATATGCTC TTTTTCTCGC 100
<210> 10633
<211> 100
<212> DNA
<213> Escherichia coli K-12 MG1655
<220> yfci b2306
<222> 792-891
<223> yfci b2306 U00096 complement(2420669..2421559)
<313> 10633: 857-876
<400> 10633
GCGTATTGCT CAGGAGATGC TGGATAGAGG TTTAGACAGA GAGTTAGTTA 50
TGATGGTGAC CCGACTTTCA CCAGACGATC TTATCGCGCA AAGCCACTAA 100
<210> 10634
<211> 100
<212> DNA
<213> Escherichia coli K-12 MG1655
<220> yfci b2306
<222> 761-860
<223> yfci b2306 U00096 complement(2420669..2421559)
<313> 10634: 787-806
<400> 10634
GCGCAATGCA GGGCAAACAC GAAGAAGCCC TGCGTATTGC TCAGGAGATG 50
CTGGATAGAG GTTTAGACAG AGAGTTAGTT ATGATGGTGA CCCGACTTTC 100
<210> 10635
<211> 100
<212> DNA
<213> Escherichia coli K-12 MG1655
<220> yfci b2306
<222> 692-791
<223> yfci b2306 U00096 complement(2420669..2421559)
<313> 10635: 712-731
<400> 10635
AGATAGCGGA ACGCGCACCA CAAGAAAAGG AGAAACTGAT GACCATTGCT 50
GACAGATTAC GTGAAGAAGG CGCAATGCAG GGCAAACACG AAGAAGCCCT 100
<210> 10636
<211> 100
<212> DNA
<213> Escherichia coli K-12 MG1655
<220> dut b3641
<222> 334-433
<223> dut b3641 U00096 3811559..3812014
<313> 10636: 358-377
<400> 10636
CCTGGCGAAC GCATCGCCCA GATGATTTTT GTTCCGGTAG TACAGGCTGA 50
ATTTAATCTG GTGGAAGATT TCGACGCCAC CGACCGCGGT GAAGGCGGCT 100
<210> 10637
<211> 100
<212> DNA
<213> Escherichia coli K-12 MG1655
<220> dut b3641
<222> 242-341
<223> dut b3641 U00096 3811559..3812014
<313> 10637: 265-284

<400> 10637
TTGGTAACCT GGTAGGATTG ATCGATTCTG ACTATCAGGG CCAGTTGATG  50
ATTTCCGTGT GGAACCGTGG TCAGGACAGC TTCACCATTC AACCTGGCGA  100
<210> 10638
<211> 100
<212> DNA
<213> Escherichia coli K-12 MG1655
<220> dut b3641
<222> 67-166
<223> dut b3641 U00096 3811559..3812014
<313> 10638: 127-146
<400> 10638
GCCACCTCTG GCTCTGCCGG ACTTGACCTG CGTGCCTGTC TCAACGACGC  50
CGTAGAACTG GCTCCGGGTG ACACTACGCT GGTTCCGACC GGGCTGGCGA  100
<210> 10639
<211> 100
<212> DNA
<213> Escherichia coli K-12 MG1655
<220> b2640 b2640
<222> 102-201
<223> b2640 b2640 U00096 complement(2770857..2771057)
<313> 10639: 173-192
<400> 10639
GTACGCCTCC GTTATGATCT CCGTGGATGC GGCAGCGATT GCGGCCACGC  50
TGATTATGCT GTATCTCTTC TTTCGCAGAG TTATTCCGGC AACGTATTAA  100
<210> 10640
<211> 100
<212> DNA
<213> Escherichia coli K-12 MG1655
<220> b2640 b2640
<222> 102-201
<223> b2640 b2640 U00096 complement(2770857..2771057)
<313> 10640: 162-181
<400> 10640
GTACGCCTCC GTTATGATCT CCGTGGATGC GGCAGCGATT GCGGCCACGC  50
TGATTATGCT GTATCTCTTC TTTCGCAGAG TTATTCCGGC AACGTATTAA  100
<210> 10641
<211> 100
<212> DNA
<213> Escherichia coli K-12 MG1655
<220> b2640 b2640
<222> 100-199
<223> b2640 b2640 U00096 complement(2770857..2771057)
<313> 10641: 157-176
<400> 10641
CAGTACGCCT CCGTTATGAT CTCCGTGGAT GCGGCAGCGA TTGCGGCCAC  50
GCTGATTATG CTGTATCTCT TCTTTCGCAG AGTTATTCCG GCAACGTATT  100
<210> 10642
<211> 100
<212> DNA
<213> Escherichia coli K-12 MG1655
<220> b2641 b2642
<222> 18-117
<223> b2641 b2642 U00096 complement(2770997..2771113)
<313> 10642: 88-107
<400> 10642
CGCACTGGGT TCAGCCAGGG CACGACACTG GCCTTTGTCA TGGCTGCAGG  50
ATTTATTGCA GATACGGCCA GCCTGCCGCT CATTGTTTCT AACCTGGTGA  100
<210> 10643
<211> 100
<212> DNA
<213> Escherichia coli K-12 MG1655
<220> b2641 b2642
<222> 18-117
<223> b2641 b2642 U00096 complement(2770997..2771113)
<313> 10643: 68-87
<400> 10643
CGCACTGGGT TCAGCCAGGG CACGACACTG GCCTTTGTCA TGGCTGCAGG  50
ATTTATTGCA GATACGGCCA GCCTGCCGCT CATTGTTTCT AACCTGGTGA  100
<210> 10644
<211> 100

<212> DNA
<213> Escherichia coli K-12 MG1655
<220> hypa b2727
<222> 252-351
<223> hypa b2727 U00096 2848670..2849020
<313> 10644: 319-338
<400> 10644
CCAGCGCGTC CGCCGCTGTC CACAGTGTCA TGGTGACATG CTGCAGATTG 50
TGGCAGACGA CGGTTTACAG ATTCGGCGGA TAGAAATAGA CCAGGAGTGA 100
<210> 10645
<211> 100
<212> DNA
<213> Escherichia coli K-12 MG1655
<220> hypa b2727
<222> 213-312
<223> hypa b2727 U00096 2848670..2849020
<313> 10645: 252-271
<400> 10645
CTGGTGTGAA ACATGCCAAC AGTATGTGAC GCTACTGACC CAGCGCGTCC 50
GCCGCTGTCC ACAGTGTCAT GGTGACATGC TGCAGATTGT GGCAGACGAC 100
<210> 10646
<211> 100
<212> DNA
<213> Escherichia coli K-12 MG1655
<220> hypa b2727
<222> 163-262
<223> hypa b2727 U00096 2848670..2849020
<313> 10646: 195-214
<400> 10646
AGCGTGGCGG AAGGTTGTAA ACTGCACCTC GAAGAACAAG AGGCCGAATG 50
CTGGTGTGAA ACATGCCAAC AGTATGTGAC GCTACTGACC CAGCGCGTCC 100
<210> 10647
<211> 100
<212> DNA
<213> Escherichia coli K-12 MG1655
<220> hypb b2728
<222> 774-873
<223> hypb b2728 U00096 2849024..2849896
<313> 10647: 820-839
<400> 10647
CGAAGTCAAT CCAGAAATTG AAATCATCCT TATTTCCGCC ACCAGCGGCG 50
AAGGGATGGA CCAGTGGCTG AACTGGCTGG AGACACAGCG ATGTGCATAG 100
<210> 10648
<211> 100
<212> DNA
<213> Escherichia coli K-12 MG1655
<220> hypb b2728
<222> 757-856
<223> hypb b2728 U00096 2849024..2849896
<313> 10648: 787-806
<400> 10648
TGCATCGCCT GCGCCCGCGA AGTCAATCCA GAAATTGAAA TCATCCTTAT 50
TTCCGCCACC AGCGGCGAAG GGATGGACCA GTGGCTGAAC TGGCTGGAGA 100
<210> 10649
<211> 100
<212> DNA
<213> Escherichia coli K-12 MG1655
<220> hypb b2728
<222> 665-764
<223> hypb b2728 U00096 2849024..2849896
<313> 10649: 701-720
<400> 10649
CACTGAAATA TCCGCATATG TTTGCCGCCG CCTCGCTGAT GCTGCTCAAC 50
AAAGTTGACC TGTTGCCGTA TCTCAACTTT GACGTTGAAA AGTGCATCGC 100
<210> 10650
<211> 100
<212> DNA
<213> Escherichia coli K-12 MG1655
<220> hypc b2729
<222> 159-258
<223> hypc b2729 U00096 2849887..2850159

<313> 10650: 191-210
<400> 10650
CTTTGCCATG AGCGTAATTA ATGAAGCCGA AGCACGCGAC ACTCTCGACG 50
CCTTACAAAA CATGTTTGAC GTTGAGCCGG ATGTCGGCGC GCTGTTGTAT 100
<210> 10651
<211> 100
<212> DNA
<213> Escherichia coli K-12 MG1655
<220> hypc b2729
<222> 162-261
<223> hypc b2729 U00096 2849887..2850159
<313> 10651: 173-192
<400> 10651
TGCCATGAGC GTAATTAATG AAGCCGAAGC ACGCGACACT CTCGACGCCT 50
TACAAAACAT GTTTGACGTT GAGCCGGATG TCGGCGCGCT GTTGTATGGC 100
<210> 10652
<211> 100
<212> DNA
<213> Escherichia coli K-12 MG1655
<220> hypc b2729
<222> 131-230
<223> hypc b2729 U00096 2849887..2850159
<313> 10652: 178-197
<400> 10652
TGGGCCAGTG GGTACTGGTA CACGTTGGCT TTGCCATGAG CGTAATTAAT 50
GAAGCCGAAG CACGCGACAC TCTCGACGCC TTACAAAACA TGTTTGACGT 100
<210> 10653
<211> 100
<212> DNA
<213> Escherichia coli K-12 MG1655
<220> hypd b2730
<222> 982-1081
<223> hypd b2730 U00096 2850159..2851280
<313> 10653: 1015-1034
<400> 10653
ACGGGCAAAT GTAAGCCGCA TCAATGCCCG CTGTTTGGTA ACACCTGTAA 50
TCCTCAAACC GCGTTTGGTG CGCTGATGGT TTCCTCCGAA GGAGCGTGCG 100
<210> 10654
<211> 100
<212> DNA
<213> Escherichia coli K-12 MG1655
<220> hypd b2730
<222> 930-1029
<223> hypd b2730 U00096 2850159..2851280
<313> 10654: 974-993
<400> 10654
GGCACCGCAG CAGGTCTGCG ATGACCCGCG CGCGCGTTGT GGTGAGGTAT 50
TAACGGGCAA ATGTAAGCCG CATCAATGCC CGCTGTTTGG TAACACCTGT 100
<210> 10655
<211> 100
<212> DNA
<213> Escherichia coli K-12 MG1655
<220> hypd b2730
<222> 857-956
<223> hypd b2730 U00096 2850159..2851280
<313> 10655: 910-929
<400> 10655
GCGTGATTGA ATCTTCTGGC GTGCACCTGA CGCCGGATTA TCAACGATTC 50
GATGCCGAAG CACATTTCCG CCCGGCACCG CAGCAGGTCT GCGATGACCC 100
<210> 10656
<211> 100
<212> DNA
<213> Escherichia coli K-12 MG1655
<220> yfcs yfcS
<222> 654-753
<223> yfcs yfcS U00096 complement(2449604..2450356)
<313> 10656: 685-704
<400> 10656
GCTGACTTAC GTCAACGACT TTGGCGCACG GATGCCGCTG TTCTTCCAGT 50
GCAATGGCAA CAGCTGCCAG GTGGATGAAG AGCAGAGCCG CAAAGGCTGA 100
<210> 10657

<211> 100
<212> DNA
<213> Escherichia coli K-12 MG1655
<220> yfcs yfcS
<222> 541-640
<223> yfcs yfcS U00096 complement(2449604..2450356)
<313> 10657: 600-619
<400> 10657
ATCAGCAACG CCAGTGCGCA GAAAAACGGT AATCCAGCTG CAGGGTTCTC 50
GCCTCTGGTC ATTGAGCCGA AAACCACGGT ACCGCTGAAT GTGAAAATGG 100
<210> 10658
<211> 100
<212> DNA
<213> Escherichia coli K-12 MG1655
<220> yfcs yfcS
<222> 499-598
<223> yfcs yfcS U00096 complement(2449604..2450356)
<313> 10658: 534-553
<400> 10658
CAGGCATTTA CCGTCAATAA CCCAACGCCG TACTACGTGA TTATCAGCAA 50
CGCCAGTGCG CAGAAAAACG GTAATCCAGC TGCAGGGTTC TCGCCTCTGG 100
<210> 10659
<211> 100
<212> DNA
<213> Escherichia coli K-12 MG1655
<220> hype b2731
<222> 870-969
<223> hype b2731 U00096 2851319..2852287
<313> 10659: 927-946
<400> 10659
TGAAGTGGTG GAACGTAAAG GTGTTCGTCT TGCCGGTCTG TATGGCGTGA 50
AACGAACCCT CGATTTACCA CACGCCGAAC CGCTTCCGCG TATATGCTAA 100
<210> 10660
<211> 100
<212> DNA
<213> Escherichia coli K-12 MG1655
<220> hype b2731
<222> 862-961
<223> hype b2731 U00096 2851319..2852287
<313> 10660: 889-908
<400> 10660
CTGATTGGTG AAGTGGTGGA ACGTAAAGGT GTTCGTCTTG CCGGTCTGTA 50
TGGCGTGAAA CGAACCCTCG ATTTACCACA CGCCGAACCG CTTCCGCGTA 100
<210> 10661
<211> 100
<212> DNA
<213> Escherichia coli K-12 MG1655
<220> hype b2731
<222> 805-904
<223> hype b2731 U00096 2851319..2852287
<313> 10661: 841-860
<400> 10661
GCGGCAGAGC AAGTGCTGGC AGCGTTACAT TCCCATCCAC TGGGGAAAGA 50
CGCGGCGCTG ATTGGTGAAG TGGTGGAACG TAAAGGTGTT CGTCTTGCCG 100
<210> 10662
<211> 100
<212> DNA
<213> Escherichia coli K-12 MG1655
<220> b2648 b2648
<222> 45-144
<223> b2648 b2648 U00096 complement(2781085..2781228)
<313> 10662: 93-112
<400> 10662
TATTAACTTT CGTGCTCTGG CCCAATCTAT ATTTGCCCAA CAATGGGGGG 50
ACGAATGCTG TAAAAGTAAA ACAATCTGCG ATCTCAAAGT TATTGTGTGA 100
<210> 10663
<211> 100
<212> DNA
<213> Escherichia coli K-12 MG1655
<220> b2648 b2648
<222> 15-114

<223> b2648 b2648 U00096 complement(2781085..2781228)
<313> 10663: 57-76
<400> 10663
CGTGCTGTTG GAGGAGCTGT GCGAACGTGG TATTAACTTT CGTGCTCTGG 50
CCCAATCTAT ATTTGCCCAA CAATGGGGGG ACGAATGCTG TAAAAGTAAA 100
<210> 10664
<211> 100
<212> DNA
<213> Escherichia coli K-12 MG1655
<220> b2648 b2648
<222> 1-100
<223> b2648 b2648 U00096 complement(2781085..2781228)
<313> 10664: 26-45
<400> 10664
ATGTGGCATC TTGTCGTGCT GTTGGAGGAG CTGTGCGAAC GTGGTATTAA 50
CTTTCGTGCT CTGGCCCAAT CTATATTTGC CCAACAATGG GGGGACGAAT 100
<210> 10665
<211> 100
<212> DNA
<213> Escherichia coli K-12 MG1655
<220> hypf b2713
<222> 2119-2218
<223> hypf b2713 U00096 complement(2833196..2835448)
<313> 10665: 2163-2182
<400> 10665
CGTCTGGCGC ATTATCTCGC TGATTTCACA TTGCTCTTTC CACAGAGTTT 50
ACCGGCGGGT GATGGCGGTT TGTCTCTGGG GCAGGGGGTT ATTGCTGCGG 100
<210> 10666
<211> 100
<212> DNA
<213> Escherichia coli K-12 MG1655
<220> hypf b2713
<222> 2093-2192
<223> hypf b2713 U00096 complement(2833196..2835448)
<313> 10666: 2120-2139
<400> 10666
TTATTCATAA CCGTTTGCTG CGTGCACGTC TGGCGCATTA TCTCGCTGAT 50
TTCACATTGC TCTTTCCACA GAGTTTACCG GCGGGTGATG GCGGTTTGTC 100
<210> 10667
<211> 100
<212> DNA
<213> Escherichia coli K-12 MG1655
<220> hypf b2713
<222> 2052-2151
<223> hypf b2713 U00096 complement(2833196..2835448)
<313> 10667: 2071-2090
<400> 10667
CACGATGCGT GGTATCACTA CGCTGGTATT TAGCGGCGGG GTTATTCATA 50
ACCGTTTGCT GCGTGCACGT CTGGCGCATT ATCTCGCTGA TTTCACATTG 100
<210> 10668
<211> 100
<212> DNA
<213> Escherichia coli K-12 MG1655
<220> b2649 b2649
<222> 687-786
<223> b2649 b2649 U00096 complement(2781658..2782449)
<313> 10668: 721-740
<400> 10668
TATGGATGAT GACTTTGATG ATAGTTCCTC TGAGGATGAT CCCGTTGAGA 50
ATAGTCCTGT TGTGACTAGT CCCGTTGTAT CAAGTTCTAA AAGCAGTTTT 100
<210> 10669
<211> 100
<212> DNA
<213> Escherichia coli K-12 MG1655
<220> b2649 b2649
<222> 636-735
<223> b2649 b2649 U00096 complement(2781658..2782449)
<313> 10669: 652-671
<400> 10669
TAATAAAATA AAACTCATCA CGGGCGTGGC ACCATTCGAT GATATTTTAT 50
TTATGGATGA TGACTTTGAT GATAGTTCCT CTGAGGATGA TCCCGTTGAG 100

<210> 10670
<211> 100
<212> DNA
<213> Escherichia coli K-12 MG1655
<220> b2649 b2649
<222> 316-415
<223> b2649 b2649 U00096 complement(2781658..2782449)
<313> 10670: 355-374
<400> 10670
GTCAAGCTGT CTGGAGAACA TCTTGAAACG GTATTCAAAT ACAAAAATGG 50
CGATGATGTC CGGGAGATAG CCAAAATTAA CATTACTCTC CAAAAAGAAG 100
<210> 10671
<211> 100
<212> DNA
<213> Escherichia coli K-12 MG1655
<220> yfcu yfcU
<222> 1638-1737
<223> yfcu yfcU U00096 complement(2451285..2453021)
<313> 10671: 1704-1723
<400> 10671
GCTCTCCCAC TATTTCAATA TGGGCAGCAT TCGTAATATG AGCGTTTCCC 50
TGACTGGCTA CCGCTACGAG TATGACAACC GGGCGGATAA GGGCATGTAG 100
<210> 10672
<211> 100
<212> DNA
<213> Escherichia coli K-12 MG1655
<220> yfcu yfcU
<222> 1617-1716
<223> yfcu yfcU U00096 complement(2451285..2453021)
<313> 10672: 1668-1687
<400> 10672
GCAGACAAAC TACAACATCA TGCTCTCCCA CTATTTCAAT ATGGGCAGCA 50
TTCGTAATAT GAGCGTTTCC CTGACTGGCT ACCGCTACGA GTATGACAAC 100
<210> 10673
<211> 100
<212> DNA
<213> Escherichia coli K-12 MG1655
<220> yfcu yfcU
<222> 1574-1673
<223> yfcu yfcU U00096 complement(2451285..2453021)
<313> 10673: 1612-1631
<400> 10673
TTTATCTCAA CTATACCCGC CATACCTACT GGGATCGTGA GGAGCAGACA 50
AACTACAACA TCATGCTCTC CCACTATTTC AATATGGGCA GCATTCGTAA 100
<210> 10674
<211> 100
<212> DNA
<213> Escherichia coli K-12 MG1655
<220> nhaa b0020
<222> 1011-1110
<223> nhaa b0020 U00096 17489..18655
<313> 10674: 1079-1098
<400> 10674
CGGTTTTACT ATGTCTATCT TTATTGCCAG CCTGGCCTTT GGTAGCGTAG 50
ATCCAGAACT GATTAACTGG GCGAAACTCG GTATCCTGGT CGGTTCTATC 100
<210> 10675
<211> 100
<212> DNA
<213> Escherichia coli K-12 MG1655
<220> nhaa b0020
<222> 970-1069
<223> nhaa b0020 U00096 17489..18655
<313> 10675: 1027-1046
<400> 10675
ACTTATCAGC AAATTATGGT GGTGGGGATC CTGTGCGGTA TCGGTTTTAC 50
TATGTCTATC TTTATTGCCA GCCTGGCCTT TGGTAGCGTA GATCCAGAAC 100
<210> 10676
<211> 100
<212> DNA
<213> Escherichia coli K-12 MG1655
<220> nhaa b0020

<222> 948-1047
<223> nhaa b0020 U00096 17489..18655
<313> 10676: 964-983
<400> 10676
GGCGCATCTG CCTGAGGGAA CGACTTATCA GCAAATTATG GTGGTGGGGA 50
TCCTGTGCGG TATCGGTTTT ACTATGTCTA TCTTTATTGC CAGCCTGGCC 100
<210> 10677
<211> 100
<212> DNA
<213> Escherichia coli K-12 MG1655
<220> nhab b1187
<222> 1443-1542
<223> nhab b1187 U00096 complement(1232399..1233940)
<313> 10677: 1513-1532
<400> 10677
CACCCTCGTC CTGACACTCG TCGGCTTGCT CTGCGTCGAG TTTACGCTTG 50
CCCCTGTAAC CGAATGGTTT ATGCAAATGG GCTGGATAGC AACGCTTTGA 100
<210> 10678
<211> 100
<212> DNA
<213> Escherichia coli K-12 MG1655
<220> nhab b1187
<222> 1441-1540
<223> nhab b1187 U00096 complement(1232399..1233940)
<313> 10678: 1473-1492
<400> 10678
TACACCCTCG TCCTGACACT CGTCGGCTTG CTCTGCGTCG AGTTTACGCT 50
TGCCCCTGTA ACCGAATGGT TTATGCAAAT GGGCTGGATA GCAACGCTTT 100
<210> 10679
<211> 100
<212> DNA
<213> Escherichia coli K-12 MG1655
<220> nhab b1187
<222> 1382-1481
<223> nhab b1187 U00096 complement(1232399..1233940)
<313> 10679: 1437-1456
<400> 10679
CTGCACTCGC GCCATTGATT CGCCTCTCTT ATGGCCGCAT GGTGTGGATG 50
GCCCTGCCTT ACACCCTCGT CCTGACACTC GTCGGCTTGC TCTGCGTCGA 100
<210> 10680
<211> 100
<212> DNA
<213> Escherichia coli K-12 MG1655
<220> xapr b2406
<222> 786-885
<223> xapr b2406 U00096 complement(2519613..2520497)
<313> 10680: 846-865
<400> 10680
TCCGCTCAAA CAGCGCATCC CTGCAGATTT ATATATTGTT TATGAAACAC 50
AGCAGGTGAC GCCCGCGATG GTTAAGCTGT TGGCTGCATT GACACAGTAG 100
<210> 10681
<211> 100
<212> DNA
<213> Escherichia coli K-12 MG1655
<220> xapr b2406
<222> 739-838
<223> xapr b2406 U00096 complement(2519613..2520497)
<313> 10681: 779-798
<400> 10681
ATAGCGGACA GCTACGCACA AATGAACTGG CCAGGTGTCA TTTTCCGTCC 50
GCTCAAACAG CGCATCCCTG CAGATTTATA TATTGTTTAT GAAACACAGC 100
<210> 10682
<211> 100
<212> DNA
<213> Escherichia coli K-12 MG1655
<220> xapr b2406
<222> 706-805
<223> xapr b2406 U00096 complement(2519613..2520497)
<313> 10682: 747-766
<400> 10682
GCTATGGTCA GTATGGGCAT TGGTATCACA TTGATAGCGG ACAGCTACGC 50

ACAAATGAAC TGGCCAGGTG TCATTTTCCG TCCGCTCAAA CAGCGCATCC 100
<210> 10683
<211> 100
<212> DNA
<213> Escherichia coli K-12 MG1655
<220> yfdc b2348
<222> 832-931
<223> yfdc b2348 U00096 2463321..2464253
<313> 10683: 891-910
<400> 10683
GCACAGATTC GTAACGACAT GAGCAATAAG CGTAAAGCAG AAGCACGCCA 50
AAAAGCAGAA CGTGCGGAAA ACATTAAGAA AAATTATAAA AACCCGGCAT 100
<210> 10684
<211> 100
<212> DNA
<213> Escherichia coli K-12 MG1655
<220> yfdc b2348
<222> 798-897
<223> yfdc b2348 U00096 2463321..2464253
<313> 10684: 846-865
<400> 10684
CGGCGGCACC TTTATCTTCG CGTTAATGAG TCATGCACAG ATTCGTAACG 50
ACATGAGCAA TAAGCGTAAA GCAGAAGCAC GCCAAAAAGC AGAACGTGCG 100
<210> 10685
<211> 100
<212> DNA
<213> Escherichia coli K-12 MG1655
<220> yfdc b2348
<222> 744-843
<223> yfdc b2348 U00096 2463321..2464253
<313> 10685: 778-797
<400> 10685
CTGGAGCGAT TTCATCTGGC CCTTCGCACT ACCTACTTTA GCGGGGAACA 50
TCTGCGGCGG CACCTTTATC TTCGCGTTAA TGAGTCATGC ACAGATTCGT 100
<210> 10686
<211> 100
<212> DNA
<213> Escherichia coli K-12 MG1655
<220> yfde yfdE
<222> 1086-1185
<223> yfde yfdE U00096 complement(2486043..2487227)
<313> 10686: 1161-1180
<400> 10686
TCCGATAAAA ATCAGCGGCT GCGCGGACCC GCATGTTATG CCGGGAGCGG 50
CAACGCTCGA CCAGCATGGG GAACAAATTC GCCAGGAGTT CTCATCATAA 100
<210> 10687
<211> 100
<212> DNA
<213> Escherichia coli K-12 MG1655
<220> yfde yfdE
<222> 1027-1126
<223> yfde yfdE U00096 complement(2486043..2487227)
<313> 10687: 1068-1087
<400> 10687
CCACAAACTC AGGCGAGAAA TATGTTGATT GAAGCCGGGG GAATAATGAT 50
GCCGGGTAAT CCGATAAAAA TCAGCGGCTG CGCGGACCCG CATGTTATGC 100
<210> 10688
<211> 100
<212> DNA
<213> Escherichia coli K-12 MG1655
<220> yfde yfdE
<222> 1004-1103
<223> yfde yfdE U00096 complement(2486043..2487227)
<313> 10688: 1046-1065
<400> 10688
GTGTGGCTGA GGCCATTAAA TTGCCACAAA CTCAGGCGAG AAATATGTTG 50
ATTGAAGCCG GGGGAATAAT GATGCCGGGT AATCCGATAA AAATCAGCGG 100
<210> 10689
<211> 100
<212> DNA
<213> Escherichia coli K-12 MG1655

<220> nhar b0021
<222> 807-906
<223> nhar b0021 U00096 18715..19620
<313> 10689: 883-902
<400> 10689
GTACCATGCT ATTTTTGCTG AGCGGATGAT TCAGCACCCG GCGGTACAGC  50
GAATCTGCAA TACGGATTAT TCTGCGCTTT TTAGTCCAGC GGTGCGTTAA  100
<210> 10690
<211> 100
<212> DNA
<213> Escherichia coli K-12 MG1655
<220> nhar b0021
<222> 784-883
<223> nhar b0021 U00096 18715..19620
<313> 10690: 841-860
<400> 10690
CGCGTCGAGA ATGTGATGGA AGAGTACCAT GCTATTTTTG CTGAGCGGAT  50
GATTCAGCAC CCGGCGGTAC AGCGAATCTG CAATACGGAT TATTCTGCGC  100
<210> 10691
<211> 100
<212> DNA
<213> Escherichia coli K-12 MG1655
<220> nhar b0021
<222> 752-851
<223> nhar b0021 U00096 18715..19620
<313> 10691: 791-810
<400> 10691
TTTATGCCGA TAAAACTGTC GTAGAAATTG GTCGCGTCGA GAATGTGATG  50
GAAGAGTACC ATGCTATTTT TGCTGAGCGG ATGATTCAGC ACCCGGCGGT  100
<210> 10692
<211> 100
<212> DNA
<213> Escherichia coli K-12 MG1655
<220> pqia b0951
<222> 1155-1254
<223> pqia b0951 U00096 1011224..1012477
<313> 10692: 1221-1240
<400> 10692
GTTTGCTTTA GTCGTCATAA TGACAATGTT TTCTGCTATG ACGTTTGACC  50
CGCGTTTGTC GTGGGATCGT CAACCTGAAT CAGAGCATGA GGAGTCCTGA  100
<210> 10693
<211> 100
<212> DNA
<213> Escherichia coli K-12 MG1655
<220> pqia b0951
<222> 1019-1118
<223> pqia b0951 U00096 1011224..1012477
<313> 10693: 1055-1074
<400> 10693
GAATGCATTT GATTTATGAA GTTGTTGAGT TTGTAGGCCG CTGGTCGATG  50
ATTGACGTTT TCGTTATCGC GGTGCTCTCG GCGCTGGTGC GTATGGGAGG  100
<210> 10694
<211> 100
<212> DNA
<213> Escherichia coli K-12 MG1655
<220> pqia b0951
<222> 947-1046
<223> pqia b0951 U00096 1011224..1012477
<313> 10694: 997-1016
<400> 10694
CAACGTTAAA GATGATCGCC ATCGCGTGGC TGTGTTGGGA TGCCAAAGGG  50
CATGGCAAGC GCGACAGTGA AAGAATGCAT TTGATTTATG AAGTTGTTGA  100
<210> 10695
<211> 100
<212> DNA
<213> Escherichia coli K-12 MG1655
<220> pqib b0952
<222> 1542-1641
<223> pqib b0952 U00096 1012482..1014122
<313> 10695: 1607-1626
<400> 10695

ACTGCAACCG GTGCTGAAAA CGCTCAATGA GAAGAGTAAC GCGCTGGTAT 50
TTGAAGCGAA GGACAAAAAA GATCCAGAGC CGAAGAGGGC GAAACAATGA 100
<210> 10696
<211> 100
<212> DNA
<213> Escherichia coli K-12 MG1655
<220> pqib b0952
<222> 1529-1628
<223> pqib b0952 U00096 1012482..1014122
<313> 10696: 1579-1598
<400> 10696
AGGTGTTGCG AGAACTGCAA CCGGTGCTGA AAACGCTCAA TGAGAAGAGT 50
AACGCGCTGG TATTTGAAGC GAAGGACAAA AAAGATCCAG AGCCGAAGAG 100
<210> 10697
<211> 100
<212> DNA
<213> Escherichia coli K-12 MG1655
<220> pqib b0952
<222> 1453-1552
<223> pqib b0952 U00096 1012482..1014122
<313> 10697: 1489-1508
<400> 10697
AATCGCAGCA TGCAGGGCTT CCAGCCTGGC TCCGCAGCCT ACAACAAGAT 50
GGTGGCGGAT ATGCAGCGCC TTGATCAGGT GTTGCGAGAA CTGCAACCGG 100
<210> 10698
<211> 100
<212> DNA
<213> Escherichia coli K-12 MG1655
<220> b2650 b2650
<222> 384-483
<223> b2650 b2650 U00096 complement(2782549..2783031)
<313> 10698: 448-467
<400> 10698
AAAGCTGGAA AACAATGAAA ATGTGTTTTC TGAAACTAAA AAATTATCGA 50
ATAAAAATAA TGCCGATCAG TTTTTTGAAT GCGCTAAAAG AAATGAATAG 100
<210> 10699
<211> 100
<212> DNA
<213> Escherichia coli K-12 MG1655
<220> b2650 b2650
<222> 384-483
<223> b2650 b2650 U00096 complement(2782549..2783031)
<313> 10699: 448-467
<400> 10699
AAAGCTGGAA AACAATGAAA ATGTGTTTTC TGAAACTAAA AAATTATCGA 50
ATAAAAATAA TGCCGATCAG TTTTTTGAAT GCGCTAAAAG AAATGAATAG 100
<210> 10700
<211> 100
<212> DNA
<213> Escherichia coli K-12 MG1655
<220> b2650 b2650
<222> 339-438
<223> b2650 b2650 U00096 complement(2782549..2783031)
<313> 10700: 369-388
<400> 10700
AATATGTTTA AGCAAGCAGG TCTGGGAAAA TCTTCTCATT AAACTAAAGC 50
TGGAAAACAA TGAAAATGTG TTTTCTGAAA CTAAAAAATT ATCGAATAAA 100
<210> 10701
<211> 100
<212> DNA
<213> Escherichia coli K-12 MG1655
<220> yfdl b2356
<222> 419-518
<223> yfdl b2356 U00096 complement(2469564..2470082)
<313> 10701: 448-467
<400> 10701
GCTATATAGC TTGTGCCGGT TATAACCAGG ACCCGGTGAA ATTTATTTCC 50
AGAGCAGGCG TACCCGGTAT CGGTGCTTCA TTTTTCGCAT TAGGGTGTTA 100
<210> 10702
<211> 100
<212> DNA

<213> Escherichia coli K-12 MG1655
<220> yfdl b2356
<222> 365-464
<223> yfdl b2356 U00096 complement(2469564..2470082)
<313> 10702: 394-413
<400> 10702
GATGTCTGCG GGTATTTGCG ACTCAGGATT ACACTCCCGG CAGTGCAAAT 50
GTTGGCTATA TAGCTTGTGC CGGTTATAAC CAGGACCCGG TGAAATTTAT 100
<210> 10703
<211> 100
<212> DNA
<213> Escherichia coli K-12 MG1655
<220> yfdl b2356
<222> 267-366
<223> yfdl b2356 U00096 complement(2469564..2470082)
<313> 10703: 292-311
<400> 10703
AAAATTGCCA TCCGGTTTAA TTATTCAATG GGGGCCAATT GATGTTCCGC 50
TGACGTCTCA GGACACAGTA ACCTATTTTC CGATTGCATT TCCGAATAGA 100
<210> 10704
<211> 100
<212> DNA
<213> Escherichia coli K-12 MG1655
<220> b2651 b2652
<222> 30-129
<223> b2651 b2652 U00096 2783241..2783372
<313> 10704: 80-99
<400> 10704
TTATATCTCA ATAGCGTTGG TTAATGAGCA TAGCCACGCT CCTGTAACGC 50
TCACAAAACT CATCTGCCTG CGGCGGGTGT TCTGGTCAGT AGTAGATGTT 100
<210> 10705
<211> 100
<212> DNA
<213> Escherichia coli K-12 MG1655
<220> b2651 b2652
<222> 2-101
<223> b2651 b2652 U00096 2783241..2783372
<313> 10705: 58-77
<400> 10705
TGAAAATCGT TTTTCAAGTG TTAGAAATTT ATATCTCAAT AGCGTTGGTT 50
AATGAGCATA GCCACGCTCC TGTAACGCTC ACAAAACTCA TCTGCCTGCG 100
<210> 10706
<211> 100
<212> DNA
<213> Escherichia coli K-12 MG1655
<220> yfdm b2357
<222> 162-261
<223> yfdm b2357 U00096 complement(2470132..2470440)
<313> 10706: 218-237
<400> 10706
GTTTGTTCTG GATCTGTTTA CTGATGGTGA TAACGCCAAA TGTGCCGCGT 50
ATTACACTGC GGAAGACAAC GCGCTGGCGC ATGACTGGTC AGAACGTCTT 100
<210> 10707
<211> 100
<212> DNA
<213> Escherichia coli K-12 MG1655
<220> yfdm b2357
<222> 145-244
<223> yfdm b2357 U00096 complement(2470132..2470440)
<313> 10707: 161-180
<400> 10707
AACACCCTGT TTGGCCCGTT TGTTCTGGAT CTGTTTACTG ATGGTGATAA 50
CGCCAAATGT GCCGCGTATT ACACTGCGGA AGACAACGCG CTGGCGCATG 100
<210> 10708
<211> 100
<212> DNA
<213> Escherichia coli K-12 MG1655
<220> yfdm b2357
<222> 85-184
<223> yfdm b2357 U00096 complement(2470132..2470440)
<313> 10708: 116-135

<400> 10708
CATGAACTGA AGGAAGTGGG CGATCAGTGG CGCACGCCGG ACAACATTTT 50
CTGGGGAATT AACACCCTGT TTGGCCCGTT TGTTCTGGAT CTGTTTACTG 100
<210> 10709
<211> 100
<212> DNA
<213> Escherichia coli K-12 MG1655
<220> yfdn b2358
<222> 396-495
<223> yfdn b2358 U00096 complement(2470407..2470901)
<313> 10709: 460-479
<400> 10709
CAATTTTCTG GCGCGTCGTG AACGGTGTTT ACGGAAAAGC AGTAAGCGGG 50
CTGCATCAGG TGAAGAGTGG TATCTCTCAG GGAATTACGT GGGGGCTTAA 100
<210> 10710
<211> 100
<212> DNA
<213> Escherichia coli K-12 MG1655
<220> yfdn b2358
<222> 396-495
<223> yfdn b2358 U00096 complement(2470407..2470901)
<313> 10710: 433-452
<400> 10710
CAATTTTCTG GCGCGTCGTG AACGGTGTTT ACGGAAAAGC AGTAAGCGGG 50
CTGCATCAGG TGAAGAGTGG TATCTCTCAG GGAATTACGT GGGGGCTTAA 100
<210> 10711
<211> 100
<212> DNA
<213> Escherichia coli K-12 MG1655
<220> yfdn b2358
<222> 320-419
<223> yfdn b2358 U00096 complement(2470407..2470901)
<313> 10711: 378-397
<400> 10711
CCAGGGGCTT ATACCGTCGT GCTGCAACGG TGTGGATGGC GGCATTCCGT 50
GAAAGCCACT CCCAGCCAGA ACGAAACAAT TTTCTGGCGC GTCGTGAACG 100
<210> 10712
<211> 100
<212> DNA
<213> Escherichia coli K-12 MG1655
<220> b2653 b2653
<222> 75-174
<223> b2653 b2653 U00096 complement(2783820..2783993)
<313> 10712: 122-141
<400> 10712
TTTACATCAT ATGATCATCA TGTTTATTGC GTGGTTTATA AGTATTGTTA 50
TTGCACACTC AAGGCCCTTT AGCTCAGTGG TTAGAGCAGG CGACTCATAA 100
<210> 10713
<211> 100
<212> DNA
<213> Escherichia coli K-12 MG1655
<220> b2653 b2653
<222> 11-110
<223> b2653 b2653 U00096 complement(2783820..2783993)
<313> 10713: 55-74
<400> 10713
TGTCGGGTTT TATGTCGCGT GTAATCACGA CCCAAGGTGA ATGGGAACGC 50
ATAAAAATCT GTTATTTACA TCATATGATC ATCATGTTTA TTGCGTGGTT 100
<210> 10714
<211> 100
<212> DNA
<213> Escherichia coli K-12 MG1655
<220> b2653 b2653
<222> 12-111
<223> b2653 b2653 U00096 complement(2783820..2783993)
<313> 10714: 41-60
<400> 10714
GTCGGGTTTT ATGTCGCGTG TAATCACGAC CCAAGGTGAA TGGGAACGCA 50
TAAAAATCTG TTATTTACAT CATATGATCA TCATGTTTAT TGCGTGGTTT 100
<210> 10715
<211> 100

<212> DNA
<213> Escherichia coli K-12 MG1655
<220> yfdo yfdO
<222> 270-369
<223> yfdo yfdO U00096 complement(2470898..2471266)
<313> 10715: 345-364
<400> 10715
GTCCGTCAGC ACGTACACAG AAAAACGCGA AGACCCGTAC TTCAAAGCCA 50
GTTACGACAA TGTGGACTAC AGCCAGATCC CGGCAGGATT CAGGGGGTGA 100
<210> 10716
<211> 100
<212> DNA
<213> Escherichia coli K-12 MG1655
<220> yfdo yfdO
<222> 264-363
<223> yfdo yfdO U00096 complement(2470898..2471266)
<313> 10716: 307-326
<400> 10716
ATCGCCGTCC GTCAGCACGT ACACAGAAAA ACGCGAAGAC CCGTACTTCA 50
AAGCCAGTTA CGACAATGTG GACTACAGCC AGATCCCGGC AGGATTCAGG 100
<210> 10717
<211> 100
<212> DNA
<213> Escherichia coli K-12 MG1655
<220> yfdo yfdO
<222> 218-317
<223> yfdo yfdO U00096 complement(2470898..2471266)
<313> 10717: 266-285
<400> 10717
GCCCGTCGAA GCTGCGGGAA AAATGGGATG AGCTTTCCCT GCGCTTATCG 50
CCGTCCGTCA GCACGTACAC AGAAAAACGC GAAGACCCGT ACTTCAAAGC 100
<210> 10718
<211> 100
<212> DNA
<213> Escherichia coli K-12 MG1655
<220> b2654 b2654
<222> 234-333
<223> b2654 b2654 U00096 2784417..2784749
<313> 10718: 314-333
<400> 10718
TAAAATTTCC CTTTCTCCAG ATACAACTTA CCGAGAGTTG AATAAAGCAG 50
CACAGCGTAG CATTACAGCG CCAGACAGGA TAGGAGAAAG AAAAACATAG 100
<210> 10719
<211> 100
<212> DNA
<213> Escherichia coli K-12 MG1655
<220> b2654 b2654
<222> 234-333
<223> b2654 b2654 U00096 2784417..2784749
<313> 10719: 291-310
<400> 10719
TAAAATTTCC CTTTCTCCAG ATACAACTTA CCGAGAGTTG AATAAAGCAG 50
CACAGCGTAG CATTACAGCG CCAGACAGGA TAGGAGAAAG AAAAACATAG 100
<210> 10720
<211> 100
<212> DNA
<213> Escherichia coli K-12 MG1655
<220> b2654 b2654
<222> 234-333
<223> b2654 b2654 U00096 2784417..2784749
<313> 10720: 283-302
<400> 10720
TAAAATTTCC CTTTCTCCAG ATACAACTTA CCGAGAGTTG AATAAAGCAG 50
CACAGCGTAG CATTACAGCG CCAGACAGGA TAGGAGAAAG AAAAACATAG 100
<210> 10721
<211> 100
<212> DNA
<213> Escherichia coli K-12 MG1655
<220> b2655 b2655
<222> 378-477
<223> b2655 b2655 U00096 2784768..2785262

<313> 10721: 419-438
<400> 10721
GTATATACAA ATCCCTCCAG TGACAACTTT CCGCGTCCGA CGGACCAGAA 50
ACTCCCGAAG AAGAAAAGGG TTACTGGTTT CATGCTTATC AACCCGAAGA 100
<210> 10722
<211> 100
<212> DNA
<213> Escherichia coli K-12 MG1655
<220> b2655 b2655
<222> 256-355
<223> b2655 b2655 U00096 2784768..2785262
<313> 10722: 321-340
<400> 10722
CAGAAATCAC AATGTGAAAT CATCACCCCC AAAAAACCTG GCGAAGCGAT 50
TCTTCATGCT TTTAATGCCA CCTACCAGCA GATCAGAGAA AATATGTCTG 100
<210> 10723
<211> 100
<212> DNA
<213> Escherichia coli K-12 MG1655
<220> b2655 b2655
<222> 236-335
<223> b2655 b2655 U00096 2784768..2785262
<313> 10723: 280-299
<400> 10723
GTAAATCTCT TACTGCTGTT CAGAAATCAC AATGTGAAAT CATCACCCCC 50
AAAAAACCTG GCGAAGCGAT TCTTCATGCT TTTAATGCCA CCTACCAGCA 100
<210> 10724
<211> 100
<212> DNA
<213> Escherichia coli K-12 MG1655
<220> b2656 b2656
<222> 90-189
<223> b2656 b2656 U00096 2785267..2785455
<313> 10724: 158-177
<400> 10724
GGGAATTGGG GGTAGTGGTA AAAATGACCT GGATTATCCC TCTGCTGATA 50
TACGAGCGAA GATCAGTAAA GATATAGAAG GTGGTATCCC TGGCTATTAG 100
<210> 10725
<211> 100
<212> DNA
<213> Escherichia coli K-12 MG1655
<220> b2656 b2656
<222> 90-189
<223> b2656 b2656 U00096 2785267..2785455
<313> 10725: 153-172
<400> 10725
GGGAATTGGG GGTAGTGGTA AAAATGACCT GGATTATCCC TCTGCTGATA 50
TACGAGCGAA GATCAGTAAA GATATAGAAG GTGGTATCCC TGGCTATTAG 100
<210> 10726
<211> 100
<212> DNA
<213> Escherichia coli K-12 MG1655
<220> b2656 b2656
<222> 90-189
<223> b2656 b2656 U00096 2785267..2785455
<313> 10726: 123-142
<400> 10726
GGGAATTGGG GGTAGTGGTA AAAATGACCT GGATTATCCC TCTGCTGATA 50
TACGAGCGAA GATCAGTAAA GATATAGAAG GTGGTATCCC TGGCTATTAG 100
<210> 10727
<211> 100
<212> DNA
<213> Escherichia coli K-12 MG1655
<220> b2657 b2657
<222> 454-553
<223> b2657 b2657 U00096 2785627..2786259
<313> 10727: 495-514
<400> 10727
AATGGTAATC TTTTCAATGA CTTTCTATTT AACCATCAAC AAGATGAACA 50
ATTAGCAATG GCATATATAG CCGCTCTGCC GTTCAGCAGG CCTTTAGTTT 100
<210> 10728

<211> 100
<212> DNA
<213> Escherichia coli K-12 MG1655
<220> b2657 b2657
<222> 453-552
<223> b2657 b2657 U00096 2785627..2786259
<313> 10728: 494-513
<400> 10728
CAATGGTAAT CTTTTCAATG ACTTTCTATT TAACCATCAA CAAGATGAAC 50
AATTAGCAAT GGCATATATA GCCGCTCTGC CGTTCAGCAG GCCTTTAGTT 100
<210> 10729
<211> 100
<212> DNA
<213> Escherichia coli K-12 MG1655
<220> b2657 b2657
<222> 416-515
<223> b2657 b2657 U00096 2785627..2786259
<313> 10729: 454-473
<400> 10729
GACGGATATT GATTAACATT AACCACGATT TTCCTAACAA TGGTAATCTT 50
TTCAATGACT TTCTATTTAA CCATCAACAA GATGAACAAT TAGCAATGGC 100
<210> 10730
<211> 100
<212> DNA
<213> Escherichia coli K-12 MG1655
<220> b2658 b2658
<222> 174-273
<223> b2658 b2658 U00096 2786398..2786670
<313> 10730: 244-263
<400> 10730
ATTTACGCTT AGAGGCGGAA CTCTAAATAT CAATATTCCT GGGCGTAGCG 50
CTCTTCTTTT AGGGAAAACA GGAGAACCGC CGAACTATCT CTATTTATAA 100
<210> 10731
<211> 100
<212> DNA
<213> Escherichia coli K-12 MG1655
<220> b2658 b2658
<222> 174-273
<223> b2658 b2658 U00096 2786398..2786670
<313> 10731: 232-251
<400> 10731
ATTTACGCTT AGAGGCGGAA CTCTAAATAT CAATATTCCT GGGCGTAGCG 50
CTCTTCTTTT AGGGAAAACA GGAGAACCGC CGAACTATCT CTATTTATAA 100
<210> 10732
<211> 100
<212> DNA
<213> Escherichia coli K-12 MG1655
<220> b2658 b2658
<222> 169-268
<223> b2658 b2658 U00096 2786398..2786670
<313> 10732: 191-210
<400> 10732
AAAGTATTTA CGCTTAGAGG CGGAACTCTA AATATCAATA TTCCTGGGCG 50
TAGCGCTCTT CTTTTAGGGA AAACAGGAGA ACCGCCGAAC TATCTCTATT 100
<210> 10733
<211> 100
<212> DNA
<213> Escherichia coli K-12 MG1655
<220> b2659 b2660
<222> 984-1083
<223> b2659 b2660 U00096 2786901..2787983
<313> 10733: 1046-1065
<400> 10733
TCGCGACCGC TTTACTCCGC ACCCGGATCT GCGCCGTGAA CTGATGCGCC 50
AGCGTGGCTA TTTCGCTTAC GCCTCTAACC ACTACCAGAC GCATCAGTAA 100
<210> 10734
<211> 100
<212> DNA
<213> Escherichia coli K-12 MG1655
<220> b2659 b2660
<222> 891-990

<223> b2659 b2660 U00096 2786901..2787983
<313> 10734: 937-956
<400> 10734
TTCCGACGCC ATTGAAACCA GCAAAGGCAT TCTTTCTGTG CCCGTTCCCG 50
TTGGCAAATT CCTGTTGATT AACAACCTGT TCTGGCTGCA CGGTCGCGAC 100
<210> 10735
<211> 100
<212> DNA
<213> Escherichia coli K-12 MG1655
<220> b2659 b2660
<222> 847-946
<223> b2659 b2660 U00096 2786901..2787983
<313> 10735: 893-912
<400> 10735
GTCCAGCCGA AAGACTTCGA AGAAGGCGTG TGGTTGAGCG AGCTTTCCGA 50
CGCCATTGAA ACCAGCAAAG GCATTCTTTC TGTGCCCGTT CCCGTTGGCA 100
<210> 10736
<211> 100
<212> DNA
<213> Escherichia coli K-12 MG1655
<220> cyaa b3807
<222> 2448-2547
<223> cyaa b3807 U00096 3988766..3991312
<313> 10736: 2504-2523
<400> 10736
TCGTGAACAG GTGATTCCGT TCCGCACAAA ATCTATCGGT AACATGCCGC 50
CTGCCAATCA GGATCACGAT ACGCCGCTAT TACAGCAATA TTTTTCGTGA 100
<210> 10737
<211> 100
<212> DNA
<213> Escherichia coli K-12 MG1655
<220> cyaa b3807
<222> 2410-2509
<223> cyaa b3807 U00096 3988766..3991312
<313> 10737: 2463-2482
<400> 10737
AACCTGCCGC AGTTCTATCA GATTGTGAAG GTTGATGGTC GTGAACAGGT 50
GATTCCGTTC CGCACAAAAT CTATCGGTAA CATGCCGCCT GCCAATCAGG 100
<210> 10738
<211> 100
<212> DNA
<213> Escherichia coli K-12 MG1655
<220> cyaa b3807
<222> 2380-2479
<223> cyaa b3807 U00096 3988766..3991312
<313> 10738: 2411-2430
<400> 10738
TTTACCTACG GCTCAAGCTT CATCAACTTC AACCTGCCGC AGTTCTATCA 50
GATTGTGAAG GTTGATGGTC GTGAACAGGT GATTCCGTTC CGCACAAAAT 100
<210> 10739
<211> 100
<212> DNA
<213> Escherichia coli K-12 MG1655
<220> yfea b2396
<222> 2205-2304
<223> yfea b2396 U00096 complement(2513663..2515969)
<313> 10739: 2260-2279
<400> 10739
GGAGTTTGTC GAGACGCAAC AGCAGCAGGC GCTATTGCAT AAGCTCGGGG 50
TGCAATATCT GCAAGGGTAT TTGATTGGTC GCCCGCAGCC ATTAGCTGAT 100
<210> 10740
<211> 100
<212> DNA
<213> Escherichia coli K-12 MG1655
<220> yfea b2396
<222> 2182-2281
<223> yfea b2396 U00096 complement(2513663..2515969)
<313> 10740: 2212-2231
<400> 10740
GCGAAGTCAT TGAGTGTGGT CGCGGAGTTT GTCGAGACGC AACAGCAGCA 50
GGCGCTATTG CATAAGCTCG GGGTGCAATA TCTGCAAGGG TATTTGATTG 100

<210> 10741
<211> 100
<212> DNA
<213> Escherichia coli K-12 MG1655
<220> yfea b2396
<222> 2115-2214
<223> yfea b2396 U00096 complement(2513663..2515969)
<313> 10741: 2174-2193
<400> 10741
TGTGAAAGAT ATTGTCACGA ACACGCTGGA TGCGATGATT GTGAGATCAA 50
TTACCGATCT GGCGAAAGCG AAGTCATTGA GTGTGGTCGC GGAGTTTGTC 100
<210> 10742
<211> 100
<212> DNA
<213> Escherichia coli K-12 MG1655
<220> lgt b2829
<222> 777-876
<223> lgt b2829 U00096 complement(2963184..2964059)
<313> 10742: 848-867
<400> 10742
CATCAGCATG GGGCAAATTC TTTCCATCCC GATGATTGTC GCGGGTGTGA 50
TCATGATGGT CTGGGCATAT CGTCGCAGCC CACAGCAACA CGTTTCCTGA 100
<210> 10743
<211> 100
<212> DNA
<213> Escherichia coli K-12 MG1655
<220> lgt b2829
<222> 729-828
<223> lgt b2829 U00096 complement(2963184..2964059)
<313> 10743: 779-798
<400> 10743
GTTTTTCCGC CAGCCCGACG CGCAGTTTAC CGGTGCCTGG GTGCAGTACA 50
TCAGCATGGG GCAAATTCTT TCCATCCCGA TGATTGTCGC GGGTGTGATC 100
<210> 10744
<211> 100
<212> DNA
<213> Escherichia coli K-12 MG1655
<220> lgt b2829
<222> 709-808
<223> lgt b2829 U00096 complement(2963184..2964059)
<313> 10744: 737-756
<400> 10744
GCGTTTCGCA TCATTGTTGA GTTTTTCCGC CAGCCCGACG CGCAGTTTAC 50
CGGTGCCTGG GTGCAGTACA TCAGCATGGG GCAAATTCTT TCCATCCCGA 100
<210> 10745
<211> 100
<212> DNA
<213> Escherichia coli K-12 MG1655
<220> yfec b2399
<222> 261-360
<223> yfec b2399 U00096 2516472..2516831
<313> 10745: 319-338
<400> 10745
AGAGATGACG CCAGTTGAAC AAAAACAGTT TACATCCTTG CTTCTGCGGG 50
AAGGGATTAT CGGATTGTTA CAACGCTTAG GGATTCGCGA TAGCAAATAA 100
<210> 10746
<211> 100
<212> DNA
<213> Escherichia coli K-12 MG1655
<220> yfec b2399
<222> 251-350
<223> yfec b2399 U00096 2516472..2516831
<313> 10746: 286-305
<400> 10746
CACTGGCGAA AGAGATGACG CCAGTTGAAC AAAAACAGTT TACATCCTTG 50
CTTCTGCGGG AAGGGATTAT CGGATTGTTA CAACGCTTAG GGATTCGCGA 100
<210> 10747
<211> 100
<212> DNA
<213> Escherichia coli K-12 MG1655
<220> yfec b2399

<222> 159-258
<223> yfec b2399 U00096 2516472..2516831
<313> 10747: 217-236
<400> 10747
TCGTGAATAT ATTCGCAATG CTGAACGTCC AGAAGGTCAG GGAGAAGCGC 50
CTGCCCTTTC TGGTGATGCG CCGCTTGAAG TATTGCTGGT GACACTGGCG 100
<210> 10748
<211> 100
<212> DNA
<213> Escherichia coli K-12 MG1655
<220> yfed b2400
<222> 268-367
<223> yfed b2400 U00096 2516833..2517225
<313> 10748: 316-335
<400> 10748
TATCGACAAA TCATCAACGC GATAGATAAT ATGACAGACA TTGAGCAGGA 50
AAAAGTCGCG CAATTTTTGT CACGTGAAGG AATTCGCAAT TTCCTCGCCC 100
<210> 10749
<211> 100
<212> DNA
<213> Escherichia coli K-12 MG1655
<220> yfed b2400
<222> 245-344
<223> yfed b2400 U00096 2516833..2517225
<313> 10749: 260-279
<400> 10749
CTCCTGGAGC ACGCGCCCCG GCGTATCGAC AAATCATCAA CGCGATAGAT 50
AATATGACAG ACATTGAGCA GGAAAAAGTC GCGCAATTTT TGTCACGTGA 100
<210> 10750
<211> 100
<212> DNA
<213> Escherichia coli K-12 MG1655
<220> yfed b2400
<222> 195-294
<223> yfed b2400 U00096 2516833..2517225
<313> 10750: 223-242
<400> 10750
CCATAACACA CCAATGTTGA TGGAAGCTGA AGAGAGAATT GCTGAATACG 50
CTCCTGGAGC ACGCGCCCCG GCGTATCGAC AAATCATCAA CGCGATAGAT 100
<210> 10751
<211> 100
<212> DNA
<213> Escherichia coli K-12 MG1655
<220> yfeg yfeG
<222> 954-1053
<223> yfeg yfeG U00096 complement(2552150..2553202)
<313> 10751: 1017-1036
<400> 10751
GGGATTCTGG CATCTGGGGC AATTTGCCAC GGATTACCAG CAGCTGTTTT 50
CCGAGAAGCC GTCACTGACG CTGCATCAGC GGATGCGGGA GTGGGGGTGA 100
<210> 10752
<211> 100
<212> DNA
<213> Escherichia coli K-12 MG1655
<220> yfeg yfeG
<222> 934-1033
<223> yfeg yfeG U00096 complement(2552150..2553202)
<313> 10752: 978-997
<400> 10752
AAAGACGCCG CCATGCAGTG GGGATTCTGG CATCTGGGGC AATTTGCCAC 50
GGATTACCAG CAGCTGTTTT CCGAGAAGCC GTCACTGACG CTGCATCAGC 100
<210> 10753
<211> 100
<212> DNA
<213> Escherichia coli K-12 MG1655
<220> yfeg yfeG
<222> 875-974
<223> yfeg yfeG U00096 complement(2552150..2553202)
<313> 10753: 938-957
<400> 10753
TTCGCCTGAA CGCCGTACGC CGCGAACTGA TAAGTCCGTG GTCGCAAAGT 50

ATGACGGTAA AAGACGCCGC CATGCAGTGG GGATTCTGGC ATCTGGGGCA 100
<210> 10754
<211> 100
<212> DNA
<213> Escherichia coli K-12 MG1655
<220> yfeh b2411
<222> 900-999
<223> yfeh b2411 U00096 2524966..2525964
<313> 10754: 955-974
<400> 10754
TCAGATCCAA TTGATGGTCT GTGCGGTGCT GGCGCGTCGA TACAAACGCC 50
AGACCGAACA GTTACAGGCG CAGCAGGAAA GCAGCGCCGA TAAAGCTTAA 100
<210> 10755
<211> 100
<212> DNA
<213> Escherichia coli K-12 MG1655
<220> yfeh b2411
<222> 837-936
<223> yfeh b2411 U00096 2524966..2525964
<313> 10755: 871-890
<400> 10755
GGCAAACATT CTGTTCCCCA CATCGGTGAT CGGTATGATG GTGCTGCCCC 50
TGATGATTTT CCATCAGATC CAATTGATGG TCTGTGCGGT GCTGGCGCGT 100
<210> 10756
<211> 100
<212> DNA
<213> Escherichia coli K-12 MG1655
<220> yfeh b2411
<222> 805-904
<223> yfeh b2411 U00096 2524966..2525964
<313> 10756: 834-853
<400> 10756
TCGAAAAAGA GTCTGGCAAA TGGCATCCCG ATGGCAAACA TTCTGTTCCC 50
CACATCGGTG ATCGGTATGA TGGTGCTGCC CCTGATGATT TTCCATCAGA 100
<210> 10757
<211> 100
<212> DNA
<213> Escherichia coli K-12 MG1655
<220> yfek yfeK
<222> 258-357
<223> yfek yfeK U00096 2535362..2535736
<313> 10757: 308-327
<400> 10757
GATTACTGGG AAGCCGTATA TTGTGAAGAT CCCCGGTAAG AGCGATGAGA 50
ACGCACAGCC TTTTTTACAT GCGTTAATTG CGCAGACGGA TAAAACGGTG 100
<210> 10758
<211> 100
<212> DNA
<213> Escherichia coli K-12 MG1655
<220> yfek yfeK
<222> 200-299
<223> yfek yfeK U00096 2535362..2535736
<313> 10758: 250-269
<400> 10758
CCCGTAACCG CATTGACACT GCCGAGCAGT TTATTGATAA GGTTGCTTCG 50
TCGTCATCGA TTACTGGGAA GCCGTATATT GTGAAGATCC CCGGTAAGAG 100
<210> 10759
<211> 100
<212> DNA
<213> Escherichia coli K-12 MG1655
<220> yfek yfeK
<222> 176-275
<223> yfek yfeK U00096 2535362..2535736
<313> 10759: 201-220
<400> 10759
ATCTGCGTCT GAAGCTCGGC AATACCCGTA ACCGCATTGA CACTGCCGAG 50
CAGTTTATTG ATAAGGTTGC TTCGTCGTCA TCGATTACTG GGAAGCCGTA 100
<210> 10760
<211> 100
<212> DNA
<213> Escherichia coli K-12 MG1655

<220> syd b2794
<222> 445-544
<223> syd b2794 U00096 complement(2922757..2923302)
<313> 10760: 485-504
<400> 10760
CTCTCCGGTG AAGTGTGCAA AGAGACGCTG GGAACACGTA AACGGACGCA 50
TTTAGCCTCA AATCTTGCGG AATTCCTTAA TCAACTTAAG CCTCTTCTGT 100
<210> 10761
<211> 100
<212> DNA
<213> Escherichia coli K-12 MG1655
<220> syd b2794
<222> 418-517
<223> syd b2794 U00096 complement(2922757..2923302)
<313> 10761: 447-466
<400> 10761
GAGTTAGAGG TCATCTCGGT GTGTAATCTC TCCGGTGAAG TGTGCAAAGA 50
GACGCTGGGA ACACGTAAAC GGACGCATTT AGCCTCAAAT CTTGCGGAAT 100
<210> 10762
<211> 100
<212> DNA
<213> Escherichia coli K-12 MG1655
<220> syd b2794
<222> 333-432
<223> syd b2794 U00096 complement(2922757..2923302)
<313> 10762: 372-391
<400> 10762
GGTGCAGGAA AATCTGATTG GTCATCTGGT GACTCAGAAA AGGCTAAAAC 50
TGCCGCCCAC ACTATTTATC GCGACGCTGG AAGAAGAGTT AGAGGTCATC 100
<210> 10763
<211> 100
<212> DNA
<213> Escherichia coli K-12 MG1655
<220> b1933 b1934
<222> 81-180
<223> b1933 b1934 U00096 complement(2009370..2009561)
<313> 10763: 102-121
<400> 10763
AGCACGTATG GAGTGTGATA TTGAGGTTGT CTCTGCGATA TATAGCAATA 50
AAGAATTACT TTTTAAAGAA TGTAATTCTT TTGAAGAGAC ATTTTTATAT 100
<210> 10764
<211> 100
<212> DNA
<213> Escherichia coli K-12 MG1655
<220> b1933 b1934
<222> 31-130
<223> b1933 b1934 U00096 complement(2009370..2009561)
<313> 10764: 85-104
<400> 10764
GGCGATAATA AGATAAAAGA AATTCAATAC TTCCCAAATC ATCTGGAGAA 50
AGCACGTATG GAGTGTGATA TTGAGGTTGT CTCTGCGATA TATAGCAATA 100
<210> 10765
<211> 100
<212> DNA
<213> Escherichia coli K-12 MG1655
<220> b1933 b1934
<222> 35-134
<223> b1933 b1934 U00096 complement(2009370..2009561)
<313> 10765: 87-106
<400> 10765
ATAATAAGAT AAAAGAAATT CAATACTTCC CAAATCATCT GGAGAAAGCA 50
CGTATGGAGT GTGATATTGA GGTTGTCTCT GCGATATATA GCAATAAAGA 100
<210> 10766
<211> 100
<212> DNA
<213> Escherichia coli K-12 MG1655
<220> yfen b2409
<222> 621-720
<223> yfen b2409 U00096 2523147..2523911
<313> 10766: 652-671
<400> 10766

AGAAGGGCTG AATGGCGCAG TTGCACTGTG GTGGAATGCA ACATCACACA 50
TTACTACGGG GATTCAGTAT CGATATGCGG ATGACAAACT GGGCGAAGAT 100
<210> 10767
<211> 100
<212> DNA
<213> Escherichia coli K-12 MG1655
<220> yfen b2409
<222> 591-690
<223> yfen b2409 U00096 2523147..2523911
<313> 10767: 619-638
<400> 10767
CGCTACCTAT GCAGCGGGTA ATGGCGGTAA AGAAGGGCTG AATGGCGCAG 50
TTGCACTGTG GTGGAATGCA ACATCACACA TTACTACGGG GATTCAGTAT 100
<210> 10768
<211> 100
<212> DNA
<213> Escherichia coli K-12 MG1655
<220> yfen b2409
<222> 529-628
<223> yfen b2409 U00096 2523147..2523911
<313> 10768: 566-585
<400> 10768
TTTATGCTGG GCAGCGAGAA ATTCACTCTT ACCAACTGGA ACGAGTACGA 50
GTTTGACCGT GACGCTACCT ATGCAGCGGG TAATGGCGGT AAAGAAGGGC 100
<210> 10769
<211> 100
<212> DNA
<213> Escherichia coli K-12 MG1655
<220> b1936 b1937
<222> 180-279
<223> b1936 b1937 U00096 2010524..2010802
<313> 10769: 239-258
<400> 10769
TAACAACAGG TTACAAAACC TACACCTGCA TGCTCATCAC TTCCTGATAC 50
GCCGCCACCA GCTTATTACG CACCTGAATC CCCATTTGCA TAGAAACTGA 100
<210> 10770
<211> 100
<212> DNA
<213> Escherichia coli K-12 MG1655
<220> b1936 b1937
<222> 146-245
<223> b1936 b1937 U00096 2010524..2010802
<313> 10770: 160-179
<400> 10770
CATCCATGCC CCACGCGGTG AGATCGGATG TAATTAACAA CAGGTTACAA 50
AACCTACACC TGCATGCTCA TCACTTCCTG ATACGCCGCC ACCAGCTTAT 100
<210> 10771
<211> 100
<212> DNA
<213> Escherichia coli K-12 MG1655
<220> b1936 b1937
<222> 68-167
<223> b1936 b1937 U00096 2010524..2010802
<313> 10771: 84-103
<400> 10771
GCGTATACGG ATCGTGGATG GCAGAAAACG TTCAGGATCA GGTATCCATT 50
TTAAACCAGA AATTGAGTGA GTTTGCCCCA TCCATGCCCC ACGCGGTGAG 100
<210> 10772
<211> 100
<212> DNA
<213> Escherichia coli K-12 MG1655
<220> cyay cyaY
<222> 222-321
<223> cyay cyaY U00096 complement(3991352..3991672)
<313> 10772: 291-310
<400> 10772
AGGCGATGAG TGGATTTGTG ATCGCAGCGG CGAAACCTTC TGGGATTTGC 50
TGGAACAGGC GGCGACGCAG CAGGCGGGTG AAACAGTCAG TTTCCGCTAA 100
<210> 10773
<211> 100
<212> DNA

<213> Escherichia coli K-12 MG1655
<220> cyay cyaY
<222> 179-278
<223> cyay cyaY U00096 complement(3991352..3991672)
<313> 10773: 215-234
<400> 10773
TATGGCTGGC AACCAAACAG GGCGGCTACC ATTTTGACCT GAAAGGCGAT 50
GAGTGGATTT GTGATCGCAG CGGCGAAACC TTCTGGGATT TGCTGGAACA 100
<210> 10774
<211> 100
<212> DNA
<213> Escherichia coli K-12 MG1655
<220> cyay cyaY
<222> 112-211
<223> cyay cyaY U00096 complement(3991352..3991672)
<313> 10774: 177-196
<400> 10774
GTACTGACCA TTACCTTTGA GAATGGCAGC AAAATCATTA TCAACCGCCA 50
GGAGCCGCTG CACCAGGTAT GGCTGGCAAC CAAACAGGGC GGCTACCATT 100
<210> 10775
<211> 100
<212> DNA
<213> Escherichia coli K-12 MG1655
<220> cbpa b1001
<222> 822-921
<223> cbpa b1001 U00096 complement(1062078..1062998)
<313> 10775: 871-890
<400> 10775
GATGCCGCCG AAACCGGATG AAAACACTGC CGCGCTGTGG CAGCAACTGG 50
CAGACGCCCA GTCGTCTTTT GATCCACGTA AAGATTGGGG GAAAGCATAA 100
<210> 10776
<211> 100
<212> DNA
<213> Escherichia coli K-12 MG1655
<220> cbpa b1001
<222> 777-876
<223> cbpa b1001 U00096 complement(1062078..1062998)
<313> 10776: 825-844
<400> 10776
GAGCAAAAAA CAGACCGGCG ATCTGTATGC GGTACTGAAA ATCGTGATGC 50
CGCCGAAACC GGATGAAAAC ACTGCCGCGC TGTGGCAGCA ACTGGCAGAC 100
<210> 10777
<211> 100
<212> DNA
<213> Escherichia coli K-12 MG1655
<220> cbpa b1001
<222> 723-822
<223> cbpa b1001 U00096 complement(1062078..1062998)
<313> 10777: 756-775
<400> 10777
CCCGCCAGGC AGCCAGGCCG GGCAACGATT GCGCGTTAAA GGCAAAGGTC 50
TGGTGAGCAA AAAACAGACC GGCGATCTGT ATGCGGTACT GAAAATCGTG 100
<210> 10778
<211> 100
<212> DNA
<213> Escherichia coli K-12 MG1655
<220> b2666 b2666
<222> 1-100
<223> b2666 b2666 U00096 complement(2794891..2795049)
<313> 10778: 18-37
<400> 10778
ATGGGTTTCT GGAGAATCGT CATCACCATC ATTCTGCCGC CGCTCGGCGT 50
GCTGCTCGGT AAAGGGTTCG GTTGGGCGTT CATTATTAAT ATTCTGTTGA 100
<210> 10779
<211> 100
<212> DNA
<213> Escherichia coli K-12 MG1655
<220> yfer b2410
<222> 823-922
<223> yfer b2410 U00096 complement(2523950..2524876)
<313> 10779: 888-907

<400> 10779
AACCGTTCAC TTTCCACCGC CGCTGAAGCT TTATGGGACG TGGTACGAGA 50
CCAGGGCAAT GCGCTAATGG CTGGGCGGGA AGGGGATCCG TTGTATCAGA 100
<210> 10780
<211> 100
<212> DNA
<213> Escherichia coli K-12 MG1655
<220> yfer b2410
<222> 741-840
<223> yfer b2410 U00096 complement(2523950..2524876)
<313> 10780: 772-791
<400> 10780
ACCATTGCCT GAAGGTAGCC CACTGGTGGT AAAGCGCATT ACGCCGGTTG 50
TTGAACGCCA GTTGATGCTG GTGCGCAGGA AGAACCGTTC ACTTTCCACC 100
<210> 10781
<211> 100
<212> DNA
<213> Escherichia coli K-12 MG1655
<220> yfer b2410
<222> 666-765
<223> yfer b2410 U00096 complement(2523950..2524876)
<313> 10781: 714-733
<400> 10781
GATTGGACAT CCGGCGACGC TGTTCCCGAT GGTAGCCGCA GGCATCGGCA 50
TCAGTATTCT TCCCGCACTG GCGCTACCAT TGCCTGAAGG TAGCCCACTG 100
<210> 10782
<211> 100
<212> DNA
<213> Escherichia coli K-12 MG1655
<220> b2667 b2668
<222> 201-300
<223> b2667 b2668 U00096 2795232..2795531
<313> 10782: 277-296
<400> 10782
TATCGACAGC CAACGGGATG CCCAACGCAT TCTATATTCC ATTAAAAATG 50
AGGCGGTAAA TGCCATTATC GCCACCCTGA AAAATGTCTA TTGTCCGTAA 100
<210> 10783
<211> 100
<212> DNA
<213> Escherichia coli K-12 MG1655
<220> b2667 b2668
<222> 179-278
<223> b2667 b2668 U00096 2795232..2795531
<313> 10783: 194-213
<400> 10783
CTCGTATGCG GGACGAAGGG CTTATCGACA GCCAACGGGA TGCCCAACGC 50
ATTCTATATT CCATTAAAAA TGAGGCGGTA AATGCCATTA TCGCCACCCT 100
<210> 10784
<211> 100
<212> DNA
<213> Escherichia coli K-12 MG1655
<220> b2667 b2668
<222> 127-226
<223> b2667 b2668 U00096 2795232..2795531
<313> 10784: 152-171
<400> 10784
GAGCTGACGC GCATTACCGG ACTGAGTGCC TCTGCGACAT CACAGCATCT 50
CGCTCGTATG CGGGACGAAG GGCTTATCGA CAGCCAACGG GATGCCCAAC 100
<210> 10785
<211> 100
<212> DNA
<213> Escherichia coli K-12 MG1655
<220> yfet b2428
<222> 759-858
<223> yfet b2428 U00096 complement(2542772..2543629)
<313> 10785: 825-844
<400> 10785
TGTTACCGAT TTACTGTTTG TTGGCCTTGT GCAACTCAAT GATGTGGAGT 50
CATTGAAAAT GATTCAGCGC AGCAGTGAGC TAACACAACG CCTGAAATGA 100
<210> 10786
<211> 100

<212> DNA
<213> Escherichia coli K-12 MG1655
<220> yfet b2428
<222> 707-806
<223> yfet b2428 U00096 complement(2542772..2543629)
<313> 10786: 754-773
<400> 10786
GAGAAACCGA ATGGCGTAGC TCGTCGATGT CTACCCGCAC GGCGCAAAAT 50
TCTGTTACCG ATTTACTGTT TGTTGGCCTT GTGCAACTCA ATGATGTGGA 100
<210> 10787
<211> 100
<212> DNA
<213> Escherichia coli K-12 MG1655
<220> yfet b2428
<222> 510-609
<223> yfet b2428 U00096 complement(2542772..2543629)
<313> 10787: 564-583
<400> 10787
GCACGTACAA GCCACCGTTT CTCAGGCGTT GAAAAAAGGC GATGTACAAA 50
TCGCTATCTC TTACAGCGGC AGTAAAAAAG AGATTGTCTT GTGCGCAGAA 100
<210> 10788
<211> 100
<212> DNA
<213> Escherichia coli K-12 MG1655
<220> yfeu yfeU
<222> 798-897
<223> yfeu yfeU U00096 2543793..2544689
<313> 10788: 871-890
<400> 10788
GGCCATTGTG ATGGTGCTGA AAAATCTCGA TGCCGCAGAA GCTAAAAAAC 50
GCCTGGATCA ACACGGCGGC TTTATTCGTC AGGTTTTAGA CAAGGAATAA 100
<210> 10789
<211> 100
<212> DNA
<213> Escherichia coli K-12 MG1655
<220> yfeu yfeU
<222> 786-885
<223> yfeu yfeU U00096 2543793..2544689
<313> 10789: 825-844
<400> 10789
CAACTGTAAA ACGGCCATTG TGATGGTGCT GAAAAATCTC GATGCCGCAG 50
AAGCTAAAAA ACGCCTGGAT CAACACGGCG GCTTTATTCG TCAGGTTTTA 100
<210> 10790
<211> 100
<212> DNA
<213> Escherichia coli K-12 MG1655
<220> yfeu yfeU
<222> 711-810
<223> yfeu yfeU U00096 2543793..2544689
<313> 10790: 744-763
<400> 10790
ACAGGTCAAT ATTGTTAAAA ACGCCACCGG ATGTAGCGCA GAGCAAGCGG 50
AAGCGGCGTT AATTGCTTGC GAGCGCAACT GTAAAACGGC CATTGTGATG 100
<210> 10791
<211> 100
<212> DNA
<213> Escherichia coli K-12 MG1655
<220> cybb b1419
<222> 468-567
<223> cybb b1419 U00096 1488890..1489456
<313> 10791: 534-553
<400> 10791
ATATTTTGTC ATCGGGTTGC ACGCTGCGGC TGCACTGGCA CACCACTATT 50
TCTGGAAGGA CAACACACTT CTACGCATGA TGCCGCGTAA ACGTTCCTGA 100
<210> 10792
<211> 100
<212> DNA
<213> Escherichia coli K-12 MG1655
<220> cybb b1419
<222> 370-469
<223> cybb b1419 U00096 1488890..1489456

<313> 10792: 409-428
<400> 10792
TGGTTTGCGT TTGGTTTGAC GATGCCTTAC GCTTCAGAGG CCAATTTCGA 50
ACGGGTAGAT AGCTTAAAGT CGTGGCATGA AACGCTGGCG AATCTGGGAT 100
<210> 10793
<211> 100
<212> DNA
<213> Escherichia coli K-12 MG1655
<220> cybb b1419
<222> 315-414
<223> cybb b1419 U00096 1488890..1489456
<313> 10793: 375-394
<400> 10793
GTTTATTGCG CTGCCAGTGA TTGGTTTGGT GATGATGTAT AACCGGGGCA 50
ACCCGTGGTT TGCGTTTGGT TTGACGATGC CTTACGCTTC AGAGGCCAAT 100
<210> 10794
<211> 100
<212> DNA
<213> Escherichia coli K-12 MG1655
<220> cybc b4237
<222> 204-303
<223> cybc b4237 U00096 4457127..4457429
<313> 10794: 258-277
<400> 10794
CGCGCTGAAG CTGGCAAATG AAGGTAAAGT AAAAGAAGCG CAGGCTGCTG 50
CAGAGCAACT GAAAACGACC CGCAATTCAT ATCACAAGAA GTATCGTTAA 100
<210> 10795
<211> 100
<212> DNA
<213> Escherichia coli K-12 MG1655
<220> cybc b4237
<222> 198-297
<223> cybc b4237 U00096 4457127..4457429
<313> 10795: 230-249
<400> 10795
TGACGACGCG CTGAAGCTGG CAAATGAAGG TAAAGTAAAA GAAGCGCAGG 50
CTGCTGCAGA GCAACTGAAA ACGACCCGCA ATTCATATCA CAAGAAGTAT 100
<210> 10796
<211> 100
<212> DNA
<213> Escherichia coli K-12 MG1655
<220> cybc b4237
<222> 133-232
<223> cybc b4237 U00096 4457127..4457429
<313> 10796: 166-185
<400> 10796
AAATCACCGG ACAGCCCGGA AATGAAAGAT TTCCGCCACG GTTTCGACAT 50
TCTGGTCGGT CAGATTGACG ACGCGCTGAA GCTGGCAAAT GAAGGTAAAG 100
<210> 10797
<211> 100
<212> DNA
<213> Escherichia coli K-12 MG1655
<220> lhr b1654
<222> 4518-4617
<223> lhr b1654 U00096 1727111..1731727
<313> 10797: 4596-4615
<400> 10797
AACAGAAGTG AATGATCTAC CGGTCCGGCA AACGCCGATG TTTACGCTGC 50
TGCGTGAGGC GGGATTTTCA AGTTCGCCAC AAGGGCTGGA TTGGGGATAG 100
<210> 10798
<211> 100
<212> DNA
<213> Escherichia coli K-12 MG1655
<220> lhr b1654
<222> 4454-4553
<223> lhr b1654 U00096 1727111..1731727
<313> 10798: 4514-4533
<400> 10798
CCCCAGAGGT TTTCCACGCG CTGACTACCG CACTGCGTCG CGAACCACGG 50
CTGCGCTTTA CGCTAACAGA AGTGAATGAT CTACCGGTCC GGCAAACGCC 100
<210> 10799

<211> 100
<212> DNA
<213> Escherichia coli K-12 MG1655
<220> lhr b1654
<222> 4432-4531
<223> lhr b1654 U00096 1727111..1731727
<313> 10799: 4466-4485
<400> 10799
CAGGAAAAAG AGGAATTACT CGCCCCAGAG GTTTTCCACG CGCTGACTAC 50
CGCACTGCGT CGCGAACCAC GGCTGCGCTT TACGCTAACA GAAGTGAATG 100
<210> 10800
<211> 100
<212> DNA
<213> Escherichia coli K-12 MG1655
<220> yffb b2472
<222> 258-357
<223> yffb b2472 U00096 2589267..2589623
<313> 10800: 308-327
<400> 10800
GCCTGCAATT ATCAAACGTC CATTGCTCTG CGTGCCCGGT AAGCCTATGC 50
TGCTGGGTTT CAGTGATTCC AGTTATCAGC AATTTTTCCA TGAGGTGTAG 100
<210> 10801
<211> 100
<212> DNA
<213> Escherichia coli K-12 MG1655
<220> yffb b2472
<222> 216-315
<223> yffb b2472 U00096 2589267..2589623
<313> 10801: 264-283
<400> 10801
AATCACCGAT GCGGCCTCTG CGGCGGCATT AATGACTGAA ATGCCTGCAA 50
TTATCAAACG TCCATTGCTC TGCGTGCCCG GTAAGCCTAT GCTGCTGGGT 100
<210> 10802
<211> 100
<212> DNA
<213> Escherichia coli K-12 MG1655
<220> yffb b2472
<222> 174-273
<223> yffb b2472 U00096 2589267..2589623
<313> 10802: 227-246
<400> 10802
TGGTACAACC TGGCGTAAAC TGGACGAAAC CACCCGCAAT AAAATCACCG 50
ATGCGGCCTC TGCGGCGGCA TTAATGACTG AAATGCCTGC AATTATCAAA 100
<210> 10803
<211> 100
<212> DNA
<213> Escherichia coli K-12 MG1655
<220> yffg b2469
<222> 1791-1890
<223> yffg b2469 U00096 complement(2581566..2583545)
<313> 10803: 1841-1860
<400> 10803
AGACAAATGG GGCCGCATCA TCGCGGATGT GGAAAGCCAG TACCGTTACC 50
AGACCACCAA TCCGAAAATC TTCGCTGGTG GTGACGCCGT GCGTGGTGCG 100
<210> 10804
<211> 100
<212> DNA
<213> Escherichia coli K-12 MG1655
<220> yffg b2469
<222> 1730-1829
<223> yffg b2469 U00096 complement(2581566..2583545)
<313> 10804: 1797-1816
<400> 10804
TTATGGCGTT TGGCTTCAAT CCGCACGGGA TGCCGTGGCT GGAGTCGCAC 50
GGTGTAACGG TAGACAAATG GGGCCGCATC ATCGCGGATG TGGAAAGCCA 100
<210> 10805
<211> 100
<212> DNA
<213> Escherichia coli K-12 MG1655
<220> yffg b2469
<222> 1702-1801

<223> yffg b2469 U00096 complement(2581566..2583545)
<313> 10805: 1725-1744
<400> 10805
GAATTTGTCA TGCCAGCCGA CGCGGTGATT ATGGCGTTTG GCTTCAATCC 50
GCACGGGATG CCGTGGCTGG AGTCGCACGG TGTAACGGTA GACAAATGGG 100
<210> 10806
<211> 100
<212> DNA
<213> Escherichia coli K-12 MG1655
<220> xasa b1493
<222> 1437-1536
<223> xasa b1493 U00096 complement(1566978..1568513)
<313> 10806: 1455-1474
<400> 10806
GGAGCCAATC AACAGTCAGA ACGCACCAAA AGGTCACTTC TTCCTGCACC 50
CGCGTGCACG TTCACCACAC TATATTGTGA TGAATGACAA GAAACACTAA 100
<210> 10807
<211> 100
<212> DNA
<213> Escherichia coli K-12 MG1655
<220> xasa b1493
<222> 1342-1441
<223> xasa b1493 U00096 complement(1566978..1568513)
<313> 10807: 1363-1382
<400> 10807
TTACTGGTTG TTAGTTTCCT GGTGGTACTT GCCCTGCCCT TTATTCTCTA 50
TGCTGTTCAT GATCGTAAAG GCAAAGCAAA TACCGGCGTC ACTCTGGAGC 100
<210> 10808
<211> 100
<212> DNA
<213> Escherichia coli K-12 MG1655
<220> xasa b1493
<222> 1252-1351
<223> xasa b1493 U00096 complement(1566978..1568513)
<313> 10808: 1296-1315
<400> 10808
GTCGGTCTGC TGACTTCAAT TATGGCGTTT ATTGTTTCCT TCCTGCCGCC 50
GGATAACATC CAGGGTGATT CTACCGATAT GTATGTTGAA TTACTGGTTG 100
<210> 10809
<211> 100
<212> DNA
<213> Escherichia coli K-12 MG1655
<220> yffh b2468
<222> 477-576
<223> yffh b2468 U00096 complement(2580923..2581498)
<313> 10809: 515-534
<400> 10809
GTTCAGCCAG GCGCTGGAGA TGATCAAAAC CGGCGAGATA CGTGACGGTA 50
AGACGGTGTT ATTGCTTAAC TATTTGCAAA CGTCACATTT AATGGACTGA 100
<210> 10810
<211> 100
<212> DNA
<213> Escherichia coli K-12 MG1655
<220> yffh b2468
<222> 434-533
<223> yffh b2468 U00096 complement(2580923..2581498)
<313> 10810: 481-500
<400> 10810
GTGGCGGTGT CGAAGATGAA GATATTGAAG TGCTCGAGCT GCCGTTCAGC 50
CAGGCGCTGG AGATGATCAA AACCGGCGAG ATACGTGACG GTAAGACGGT 100
<210> 10811
<211> 100
<212> DNA
<213> Escherichia coli K-12 MG1655
<220> yffh b2468
<222> 323-422
<223> yffh b2468 U00096 complement(2580923..2581498)
<313> 10811: 387-406
<400> 10811
AAGTGGGTGA AGTGCGCAAA TTATTTGAAC TGTATATGTC GCCAGGCGGT 50
GTGACTGAGC TAATCCACTT TTTTATCGCC GAATACAGTG ACAATCAGCG 100

<210> 10812
<211> 100
<212> DNA
<213> Escherichia coli K-12 MG1655
<220> dxs b0421
<222> 1764-1863
<223> dxs b0421 U00096 complement(437539..439401)
<313> 10812: 1828-1847
<400> 10812
GGACTTCTTT ATTCCGCAAG GAACTCAGGA AGAAATGCGC GCCGAACTCG 50
GCCTCGATGC CGCTGGTATG GAAGCCAAAA TCAAGGCCTG GCTGGCATAA 100
<210> 10813
<211> 100
<212> DNA
<213> Escherichia coli K-12 MG1655
<220> dxs b0421
<222> 1737-1836
<223> dxs b0421 U00096 complement(437539..439401)
<313> 10813: 1782-1801
<400> 10813
AGTACCCGTG CTGAACATTG GCCTGCCGGA CTTCTTTATT CCGCAAGGAA 50
CTCAGGAAGA AATGCGCGCC GAACTCGGCC TCGATGCCGC TGGTATGGAA 100
<210> 10814
<211> 100
<212> DNA
<213> Escherichia coli K-12 MG1655
<220> dxs b0421
<222> 1703-1802
<223> dxs b0421 U00096 complement(437539..439401)
<313> 10814: 1740-1759
<400> 10814
GCGTGAACGA AGTGCTGATG GCCCATCGTA AACCAGTACC CGTGCTGAAC 50
ATTGGCCTGC CGGACTTCTT TATTCCGCAA GGAACTCAGG AAGAAATGCG 100
<210> 10815
<211> 100
<212> DNA
<213> Escherichia coli K-12 MG1655
<220> b2670 b2671
<222> 351-450
<223> b2670 b2671 U00096 2797185..2797634
<313> 10815: 410-429
<400> 10815
CATCGTTGTT TCGATGTTGA TGGGGGCGGT TTATGGCTAC TTCCTCGATT 50
ATTGCCGCCG ACTGTTTAAA GTCAGCCGTT ACCAGCAGGT AAAAGCCTGA 100
<210> 10816
<211> 100
<212> DNA
<213> Escherichia coli K-12 MG1655
<220> b2670 b2671
<222> 336-435
<223> b2670 b2671 U00096 2797185..2797634
<313> 10816: 375-394
<400> 10816
GGCGGTCAGT TCAAACATCG TTGTTTCGAT GTTGATGGGG GCGGTTTATG 50
GCTACTTCCT CGATTATTGC CGCCGACTGT TTAAAGTCAG CCGTTACCAG 100
<210> 10817
<211> 100
<212> DNA
<213> Escherichia coli K-12 MG1655
<220> b2670 b2671
<222> 263-362
<223> b2670 b2671 U00096 2797185..2797634
<313> 10817: 318-337
<400> 10817
ATGTGACGTT CCAGTCACCG GTGTATGTGG CGATCTTGTT AGTGGTGGGC 50
GCAGACTGGC ATCAGATTAT GGCGGCGGTC AGTTCAAACA TCGTTGTTTC 100
<210> 10818
<211> 100
<212> DNA
<213> Escherichia coli K-12 MG1655
<220> lig b2412

<222> 1917-2016
<223> lig b2412 U00096 complement(2526181..2528196)
<313> 10818: 1984-2003
<400> 10818
GATAGCGGGT GAAGCTGCAG GATCTAAACT GGCGAAGGCG CAGGAACTGG  50
GCATTGAAGT CATCGACGAA GCGGAAATGC TGCGTTTGCT GGGTAGCTGA  100
<210> 10819
<211> 100
<212> DNA
<213> Escherichia coli K-12 MG1655
<220> lig b2412
<222> 1916-2015
<223> lig b2412 U00096 complement(2526181..2528196)
<313> 10819: 1957-1976
<400> 10819
TGATAGCGGG TGAAGCTGCA GGATCTAAAC TGGCGAAGGC GCAGGAACTG  50
GGCATTGAAG TCATCGACGA AGCGGAAATG CTGCGTTTGC TGGGTAGCTG  100
<210> 10820
<211> 100
<212> DNA
<213> Escherichia coli K-12 MG1655
<220> lig b2412
<222> 1876-1975
<223> lig b2412 U00096 complement(2526181..2528196)
<313> 10820: 1902-1921
<400> 10820
GCGAAAGTCG CGGGCAGCGT GTCGAAGAAA ACCGATCTGG TGATAGCGGG  50
TGAAGCTGCA GGATCTAAAC TGGCGAAGGC GCAGGAACTG GGCATTGAAG  100
<210> 10821
<211> 100
<212> DNA
<213> Escherichia coli K-12 MG1655
<220> cyca b4209
<222> 1295-1394
<223> cyca b4209 U00096 4427442..4428854
<313> 10821: 1328-1347
<400> 10821
TGCTGACACT GGAAGATGAC ACTCGCCAGG CGCTGCTGGT TACCCCGCTG  50
TGGTTTATCG CGCTGGGGTT GGGCTGGCTG TTTATTGGTA AGAAGCGGGC  100
<210> 10822
<211> 100
<212> DNA
<213> Escherichia coli K-12 MG1655
<220> cyca b4209
<222> 1218-1317
<223> cyca b4209 U00096 4427442..4428854
<313> 10822: 1268-1287
<400> 10822
GAAGTCGATC TACAAGATGC CGCTCGGCAA GCTGATGTGC TGGGTATGTA  50
TGGCGTTCTT TGTGTTCGTG GTCGTGTTGC TGACACTGGA AGATGACACT  100
<210> 10823
<211> 100
<212> DNA
<213> Escherichia coli K-12 MG1655
<220> cyca b4209
<222> 1164-1263
<223> cyca b4209 U00096 4427442..4428854
<313> 10823: 1225-1244
<400> 10823
GATTATCCTT TGCTCGTACC TTGTGTATCG CAAACAGCGT CCTCATCTAC  50
ATGAGAAGTC GATCTACAAG ATGCCGCTCG GCAAGCTGAT GTGCTGGGTA  100
<210> 10824
<211> 100
<212> DNA
<213> Escherichia coli K-12 MG1655
<220> yfga b2517
<222> 915-1014
<223> yfga b2517 U00096 complement(2639851..2640864)
<313> 10824: 980-999
<400> 10824
GATCCAGTAT CAAGGGAAAC CTGTCGATCT GAGTCGTTTT ATCAGAACTA  50

ACCAGGTTGC GCGTCTGACC CTCAATGCCG AACAATCACC GGCGCAGTAA 100
<210> 10825
<211> 100
<212> DNA
<213> Escherichia coli K-12 MG1655
<220> yfga b2517
<222> 878-977
<223> yfga b2517 U00096 complement(2639851..2640864)
<313> 10825: 906-925
<400> 10825
CGTACAAACT GAAAATTGGT GCGCCAGCCG CAGTACAGAT CCAGTATCAA 50
GGGAAACCTG TCGATCTGAG TCGTTTTATC AGAACTAACC AGGTTGCGCG 100
<210> 10826
<211> 100
<212> DNA
<213> Escherichia coli K-12 MG1655
<220> yfga b2517
<222> 837-936
<223> yfga b2517 U00096 complement(2639851..2640864)
<313> 10826: 862-881
<400> 10826
GCAGCGTAAA GACGGTAATT TGAACTTAAC CGGCCAGGCA CCGTACAAAC 50
TGAAAATTGG TGCGCCAGCC GCAGTACAGA TCCAGTATCA AGGGAAACCT 100
<210> 10827
<211> 100
<212> DNA
<213> Escherichia coli K-12 MG1655
<220> yfgb b2518
<222> 1056-1155
<223> yfgb b2518 U00096 complement(2641149..2642303)
<313> 10827: 1119-1138
<400> 10827
TGCTGCCTGT GGTCAGTTGG CGGGCGATGT TATCGACCGT ACGAAACGTA 50
CCCTGCGTAA ACGGATGCAG GGTGAAGCCA TCGACATTAA AGCGGTCTGA 100
<210> 10828
<211> 100
<212> DNA
<213> Escherichia coli K-12 MG1655
<220> yfgb b2518
<222> 955-1054
<223> yfgb b2518 U00096 complement(2641149..2642303)
<313> 10828: 997-1016
<400> 10828
TATGGACGCA GCTCGAACAG CCGTATCGAC CGTTTCTCAA AAGTGCTGAT 50
GAGCTACGGT TTCACCACTA TTGTGCGTAA AACTCGTGGT GATGATATCG 100
<210> 10829
<211> 100
<212> DNA
<213> Escherichia coli K-12 MG1655
<220> yfgb b2518
<222> 865-964
<223> yfgb b2518 U00096 complement(2641149..2642303)
<313> 10829: 925-944
<400> 10829
ACTGAACACG CGCACCAACT GGCGGAACTG CTGAAAGATA CGCCGTGTAA 50
GATCAACCTG ATCCCGTGGA ACCCGTTCCC GGGTGCGCCG TATGGACGCA 100
<210> 10830
<211> 100
<212> DNA
<213> Escherichia coli K-12 MG1655
<220> lit b1140
<222> 487-586
<223> lit b1140 U00096 1197918..1198811
<313> 10830: 544-563
<400> 10830
AGTCATGTTG TTTTACAGCA TCCATTGGTC ACTACAGCAT TCTCCACTCA 50
AGAGGAGCGT GAAGCAGATT CACATGCTAC AAAATGGATA TTAGGCAACC 100
<210> 10831
<211> 100
<212> DNA
<213> Escherichia coli K-12 MG1655

<220> lit b1140
<222> 343-442
<223> lit b1140 U00096 1197918..1198811
<313> 10831: 402-421
<400> 10831
AGGAAAAACT TGCAAACAAC AGGTTGCGAA TCATGGCCTA AAAAATGTCC 50
CAAGCCAGAA GCATATTTAC AAGGAAGCGA AGACTCACAA GTTGCTAGCG 100
<210> 10832
<211> 100
<212> DNA
<213> Escherichia coli K-12 MG1655
<220> lit b1140
<222> 299-398
<223> lit b1140 U00096 1197918..1198811
<313> 10832: 354-373
<400> 10832
GAAAAAATAG GCTTAAAAAG TCTGATGAAC TTCTTAAATG GGCAAGGAAA 50
AACTTGCAAA CAACAGGTTG CGAATCATGG CCTAAAAAAT GTCCCAAGCC 100
<210> 10833
<211> 100
<212> DNA
<213> Escherichia coli K-12 MG1655
<220> spea b2939
<222> 1861-1960
<223> spea b2939 U00096 complement(3081954..3083930)
<313> 10833: 1879-1898
<400> 10833
TTAACCCAGT TCCGCGATCA AGTGAAGAAA ACCGATCTTG ATGCTGAACT 50
GCAACAACAG TTCCTTGAAG AGTTCGAGGC AGGTTTGTAC GGTTATACTT 100
<210> 10834
<211> 100
<212> DNA
<213> Escherichia coli K-12 MG1655
<220> spea b2939
<222> 1809-1908
<223> spea b2939 U00096 complement(3081954..3083930)
<313> 10834: 1838-1857
<400> 10834
CGATACCGTG GCGGACATGC TGCAATATGT ACAGCTCGAT CCGAAAACGC 50
TGTTAACCCA GTTCCGCGAT CAAGTGAAGA AAACCGATCT TGATGCTGAA 100
<210> 10835
<211> 100
<212> DNA
<213> Escherichia coli K-12 MG1655
<220> spea b2939
<222> 1756-1855
<223> spea b2939 U00096 complement(3081954..3083930)
<313> 10835: 1792-1811
<400> 10835
GACGTGTTCG TCTTCCCTGA CGGTAGCGTA GAAGTAGAAC TGTCTGACGA 50
AGGCGATACC GTGGCGGACA TGCTGCAATA TGTACAGCTC GATCCGAAAA 100
<210> 10836
<211> 100
<212> DNA
<213> Escherichia coli K-12 MG1655
<220> speb b2938
<222> 822-921
<223> speb b2938 U00096 complement(3080896..3081816)
<313> 10836: 889-908
<400> 10836
AGTGGCTCCG GCATACGATC AGTCGGAAAT CACTGCTCTG GCAGCGGCAA 50
CGCTGGCGCT GGAAATGCTG TATATTCAGG CGGCGAAAAA GGGCGAGTAA 100
<210> 10837
<211> 100
<212> DNA
<213> Escherichia coli K-12 MG1655
<220> speb b2938
<222> 795-894
<223> speb b2938 U00096 complement(3080896..3081816)
<313> 10837: 854-873
<400> 10837

CAACATTGTT GGGATGGACG TAGTGGAAGT GGCTCCGGCA TACGATCAGT 50
CGGAAATCAC TGCTCTGGCA GCGGCAACGC TGGCGCTGGA AATGCTGTAT 100
<210> 10838
<211> 100
<212> DNA
<213> Escherichia coli K-12 MG1655
<220> speb b2938
<222> 748-847
<223> speb b2938 U00096 complement(3080896..3081816)
<313> 10838: 763-782
<400> 10838
CTGACCTCCG ATCGCGCTAT TAAACTGGTA CGCGGCCTGA AAGATCTCAA 50
CATTGTTGGG ATGGACGTAG TGGAAGTGGC TCCGGCATAC GATCAGTCGG 100
<210> 10839
<211> 100
<212> DNA
<213> Escherichia coli K-12 MG1655
<220> spec b2966
<222> 2097-2196
<223> spec b2966 U00096 complement(3105038..3107233)
<313> 10839: 2148-2167
<400> 10839
GGTGAATTTG TTGCCGGGAT TTTCGCCGGA GCTGCAAGGT GTTTATAGCG 50
AAACCGATGC GGATGGCGTG AAACGGTTGT ACGGTTATGT GTTGAAGTAA 100
<210> 10840
<211> 100
<212> DNA
<213> Escherichia coli K-12 MG1655
<220> spec b2966
<222> 2011-2110
<223> spec b2966 U00096 complement(3105038..3107233)
<313> 10840: 2078-2097
<400> 10840
CCTTATCCAC CTGGCGTGCT TTGCGTGGTA CCCGGGGAAG TCTGGGGTGG 50
GGCGGTTCAA CGTTATTTCC TTGCACTGGA AGAAGGGGTG AATTTGTTGC 100
<210> 10841
<211> 100
<212> DNA
<213> Escherichia coli K-12 MG1655
<220> spec b2966
<222> 1954-2053
<223> spec b2966 U00096 complement(3105038..3107233)
<313> 10841: 1997-2016
<400> 10841
GTGGAGTTGG TGCGGATTCG TGATGCCGAA GGGCGAATTG CGGCAGAAGG 50
GGCGTTGCCT TATCCACCTG GCGTGCTTTG CGTGGTACCC GGGGAAGTCT 100
<210> 10842
<211> 100
<212> DNA
<213> Escherichia coli K-12 MG1655
<220> sped b0121
<222> 696-795
<223> sped b0121 U00096 complement(134788..135582)
<313> 10842: 769-788
<400> 10842
ACCGGAAGAC TTAACCGACA GCGAGCGCCA GGAAATTACC GCTGCGCTGT 50
GGAAAGAAAT GCGCGAGATT TATTACGGGC GCAATATGCC AGCTGTTTAA 100
<210> 10843
<211> 100
<212> DNA
<213> Escherichia coli K-12 MG1655
<220> sped b0121
<222> 654-753
<223> sped b0121 U00096 complement(134788..135582)
<313> 10843: 703-722
<400> 10843
GCTTAAAGAG TTCGACCTTA AGCACTACAT GTTCCACACC AAACCGGAAG 50
ACTTAACCGA CAGCGAGCGC CAGGAAATTA CCGCTGCGCT GTGGAAAGAA 100
<210> 10844
<211> 100
<212> DNA

<213> Escherichia coli K-12 MG1655
<220> sped b0121
<222> 611-710
<223> sped b0121 U00096 complement(134788..135582)
<313> 10844: 672-691
<400> 10844
ATGTGAACGT CTATCAGGAA AATATCTTCC ATACCAAGAT GTTGCTTAAA 50
GAGTTCGACC TTAAGCACTA CATGTTCCAC ACCAAACCGG AAGACTTAAC 100
<210> 10845
<211> 100
<212> DNA
<213> Escherichia coli K-12 MG1655
<220> spee b0122
<222> 768-867
<223> spee b0122 U00096 complement(135598..136464)
<313> 10845: 833-852
<400> 10845
CTCTGGCCTG AAATGCCGTT ATTACAATCC GGCAATCCAT ACGGCAGCTT 50
TTGCCTTACC TCAGTATCTG CAAGACGCAC TGGCTTCACA GCCGTCCTAA 100
<210> 10846
<211> 100
<212> DNA
<213> Escherichia coli K-12 MG1655
<220> spee b0122
<222> 743-842
<223> spee b0122 U00096 complement(135598..136464)
<313> 10846: 789-808
<400> 10846
AAATTATTCA GGCGCGTTTT CTCGCCTCTG GCCTGAAATG CCGTTATTAC 50
AATCCGGCAA TCCATACGGC AGCTTTTGCC TTACCTCAGT ATCTGCAAGA 100
<210> 10847
<211> 100
<212> DNA
<213> Escherichia coli K-12 MG1655
<220> spee b0122
<222> 698-797
<223> spee b0122 U00096 complement(135598..136464)
<313> 10847: 734-753
<400> 10847
TTGCATGGGC GACAGATAAC GACGCCTTAC GCCATCTCTC AACCGAAATT 50
ATTCAGGCGC GTTTTCTCGC CTCTGGCCTG AAATGCCGTT ATTACAATCC 100
<210> 10848
<211> 100
<212> DNA
<213> Escherichia coli K-12 MG1655
<220> hcab b2542
<222> 714-813
<223> hcab b2542 U00096 2669245..2670057
<313> 10848: 777-796
<400> 10848
GCGGCGCAAT AATCGCGCAT TAAGCGGTGT GATGATCAAC GCTGATGCGG 50
GTTTAGCGAT TCGCGGCATT CGCCACGTAG CGGCTGGGCT GGATCTTTAA 100
<210> 10849
<211> 100
<212> DNA
<213> Escherichia coli K-12 MG1655
<220> hcab b2542
<222> 703-802
<223> hcab b2542 U00096 2669245..2670057
<313> 10849: 753-772
<400> 10849
ATGTTGACAT CGCGGCGCAA TAATCGCGCA TTAAGCGGTG TGATGATCAA 50
CGCTGATGCG GGTTTAGCGA TTCGCGGCAT TCGCCACGTA GCGGCTGGGC 100
<210> 10850
<211> 100
<212> DNA
<213> Escherichia coli K-12 MG1655
<220> hcab b2542
<222> 622-721
<223> hcab b2542 U00096 2669245..2670057
<313> 10850: 684-703

<400> 10850
TCTCTGACGC CGGAGAAAAT TGCCGCCATT TTACCGCTGC AATTTTTCCC 50
GCAACCGGCG GATTTTACGG GGCCGTATGT GATGTTGACA TCGCGGCGCA 100
<210> 10851
<211> 100
<212> DNA
<213> Escherichia coli K-12 MG1655
<220> spef b0694
<222> 2064-2163
<223> spef b0694 U00096 complement(717485..719683)
<313> 10851: 2103-2122
<400> 10851
CAACCTGCTG CCAGGTTTTG CACCGGAGCT GCAGGGTGTC TATATCGAAG 50
AACATGATGG TCGTAAGCAA GTTTGGTGCT ATGTCATCAA GCCTCGTGAT 100
<210> 10852
<211> 100
<212> DNA
<213> Escherichia coli K-12 MG1655
<220> spef b0694
<222> 1944-2043
<223> spef b0694 U00096 complement(717485..719683)
<313> 10852: 1980-1999
<400> 10852
AGAAGGCCGT ATCGCTGCCG AAGGTGCGCT TCCTTATCCT CCGGGTGTGC 50
TGTGTGTTGT TCCGGGTGAA ATCTGGGGTG GTGCTGTTCT GCGTTACTTC 100
<210> 10853
<211> 100
<212> DNA
<213> Escherichia coli K-12 MG1655
<220> spef b0694
<222> 1896-1995
<223> spef b0694 U00096 complement(717485..719683)
<313> 10853: 1930-1949
<400> 10853
CTACGCCTAT TTACGCGGTG AAGTGGAACT GGTTCGTCTG CCGGATGCAG 50
AAGGCCGTAT CGCTGCCGAA GGTGCGCTTC CTTATCCTCC GGGTGTGCTG 100
<210> 10854
<211> 100
<212> DNA
<213> Escherichia coli K-12 MG1655
<220> hcac b2541
<222> 222-321
<223> hcac b2541 U00096 2668928..2669248
<313> 10854: 282-301
<400> 10854
GAAAGCGTTA TGCCTGCCCG CCACCGATCC GCTCACCACT TATCCAGTAC 50
ACGTTGAAGG TGGTGACATT TTCATCGACT TACCGGAGGC GCAGCCATGA 100
<210> 10855
<211> 100
<212> DNA
<213> Escherichia coli K-12 MG1655
<220> hcac b2541
<222> 216-315
<223> hcac b2541 U00096 2668928..2669248
<313> 10855: 242-261
<400> 10855
AACGGGGAAA GCGTTATGCC TGCCCGCCAC CGATCCGCTC ACCACTTATC 50
CAGTACACGT TGAAGGTGGT GACATTTTCA TCGACTTACC GGAGGCGCAG 100
<210> 10856
<211> 100
<212> DNA
<213> Escherichia coli K-12 MG1655
<220> hcac b2541
<222> 150-249
<223> hcac b2541 U00096 2668928..2669248
<313> 10856: 202-221
<400> 10856
AGAAGGGTAT CTGGAAGATG ACGCCACGGT GGAGTGCCCG CTACACGCCG 50
CCAGTTTTTG CCTGAAAACG GGGAAAGCGT TATGCCTGCC CGCCACCGAT 100
<210> 10857
<211> 100

<212> DNA
<213> Escherichia coli K-12 MG1655
<220> speg b1585
<222> 462-561
<223> speg b1585 U00096 1654208..1654768
<313> 10857: 533-552
<400> 10857
TCAATATCGT AATGCCATTC GCATGTGTAT ATTCCAGCAT CAGTATCTGG 50
CAGAGCACAA AACACCGGGT CAGACTCTCC TGAAGCCGAC CGCACAATAG 100
<210> 10858
<211> 100
<212> DNA
<213> Escherichia coli K-12 MG1655
<220> speg b1585
<222> 293-392
<223> speg b1585 U00096 1654208..1654768
<313> 10858: 309-328
<400> 10858
GTCTGGCAAC CCGTGCCGCC AAATTAGCAA TGGACTATGG CTTTACCGTT 50
CTCAATCTCT ATAAGCTGTA TCTGATCGTT GATAAAGAGA ATGAAAAAGC 100
<210> 10859
<211> 100
<212> DNA
<213> Escherichia coli K-12 MG1655
<220> speg b1585
<222> 224-323
<223> speg b1585 U00096 1654208..1654768
<313> 10859: 272-291
<400> 10859
TGGAAATTAA CCATGTTCAT CGCCGCGCAG AATTTCAGAT AATTATCTCC 50
CCGGAGTATC AGGGGAAAGG TCTGGCAACC CGTGCCGCCA AATTAGCAAT 100
<210> 10860
<211> 100
<212> DNA
<213> Escherichia coli K-12 MG1655
<220> hcad b2543
<222> 1104-1203
<223> hcad b2543 U00096 2670067..2671269
<313> 10860: 1166-1185
<400> 10860
GGGGCGTGAG ATTCGCCCAA TTCGCAAATG GATCCAGAGC GGCAAAACGT 50
TTGATGCGAA ACTGCTGATA GATGAGAACA TCGCGCTTAA ATCACTGTAA 100
<210> 10861
<211> 100
<212> DNA
<213> Escherichia coli K-12 MG1655
<220> hcad b2543
<222> 997-1096
<223> hcad b2543 U00096 2670067..2671269
<313> 10861: 1054-1073
<400> 10861
GATATGCGTG GCGATGACTG GCTTTGTCGT GGCAACCCGG AAACTCAGAA 50
GGCGATTTGG TTTAATCTGC AAAACGGCGT GCTTATCGGT GCGGTCACGC 100
<210> 10862
<211> 100
<212> DNA
<213> Escherichia coli K-12 MG1655
<220> hcad b2543
<222> 959-1058
<223> hcad b2543 U00096 2670067..2671269
<313> 10862: 994-1013
<400> 10862
GGAGCGATCA GTACAGTGAT AACTTACAGT TTATTGGCGA TATGCGTGGC 50
GATGACTGGC TTTGTCGTGG CAACCCGGAA ACTCAGAAGG CGATTTGGTT 100
<210> 10863
<211> 100
<212> DNA
<213> Escherichia coli K-12 MG1655
<220> b2680 b2680
<222> 168-267
<223> b2680 b2680 U00096 2806337..2806603

<313> 10863: 229-248
<400> 10863
GTTGGGCTAT GCCGCAGGTC TACTGTTTCT TGTTCCCCTC GGTGATATGT 50
TTGAACGCCG CCGCCTGATT GTCTCGATGA CCTTACTGGC GGCATGTTGA 100
<210> 10864
<211> 100
<212> DNA
<213> Escherichia coli K-12 MG1655
<220> b2680 b2680
<222> 130-229
<223> b2680 b2680 U00096 2806337..2806603
<313> 10864: 197-216
<400> 10864
TCCGCCAGTT CGGCAGGCTT TATTGTTACC GCCGCGCAGT TGGGCTATGC 50
CGCAGGTCTA CTGTTTCTTG TTCCCCTCGG TGATATGTTT GAACGCCGCC 100
<210> 10865
<211> 100
<212> DNA
<213> Escherichia coli K-12 MG1655
<220> b2680 b2680
<222> 92-191
<223> b2680 b2680 U00096 2806337..2806603
<313> 10865: 151-170
<400> 10865
AGCCATTGCT CGACACCATC GCGCGTAACT TTTCCCTTTC CGCCAGTTCG 50
GCAGGCTTTA TTGTTACCGC CGCGCAGTTG GGCTATGCCG CAGGTCTACT 100
<210> 10866
<211> 100
<212> DNA
<213> Escherichia coli K-12 MG1655
<220> mrsa b3177
<222> 1239-1338
<223> mrsa b3177 U00096 complement(3320374..3321711)
<313> 10866: 1281-1300
<400> 10866
CGGCACCGAA CCGTTAATTC GCGTGATGGT GGAAGGCGAA GACGAAGCGC 50
AGGTGACTGA ATTTGCACAC CGCATCGCCG ATGCAGTAAA AGCCGTTTAA 100
<210> 10867
<211> 100
<212> DNA
<213> Escherichia coli K-12 MG1655
<220> mrsa b3177
<222> 1193-1292
<223> mrsa b3177 U00096 complement(3320374..3321711)
<313> 10867: 1238-1257
<400> 10867
TTGAAGCTGC GCTGGGCAAC CGTGGACGCG TGTTGCTGCG TAAATCCGGC 50
ACCGAACCGT TAATTCGCGT GATGGTGGAA GGCGAAGACG AAGCGCAGGT 100
<210> 10868
<211> 100
<212> DNA
<213> Escherichia coli K-12 MG1655
<220> mrsa b3177
<222> 1148-1247
<223> mrsa b3177 U00096 complement(3320374..3321711)
<313> 10868: 1201-1220
<400> 10868
GCGATCCACT TGAGCATGAG TCAGTTAAAG CCGTGACCGC AGAGGTTGAA 50
GCTGCGCTGG GCAACCGTGG ACGCGTGTTG CTGCGTAAAT CCGGCACCGA 100
<210> 10869
<211> 100
<212> DNA
<213> Escherichia coli K-12 MG1655
<220> b2681 b2681
<222> 759-858
<223> b2681 b2681 U00096 2806597..2807514
<313> 10869: 793-812
<400> 10869
GACTAGCTAC TTTATTGGCG GTGCCGCCGG TTCGCTAATT TCAGCCTCAG 50
CCTGGCAACA TGGCGGTTGG GCTGGCGTTT GTCTGGCTGG CGCGACGATT 100
<210> 10870

<211> 100
<212> DNA
<213> Escherichia coli K-12 MG1655
<220> b2681 b2681
<222> 731-830
<223> b2681 b2681 U00096 2806597..2807514
<313> 10870: 762-781
<400> 10870
CGCGCAATCG CCTGACCGCA GGTTACATGA CTAGCTACTT TATTGGCGGT 50
GCCGCCGGTT CGCTAATTTC AGCCTCAGCC TGGCAACATG GCGGTTGGGC 100
<210> 10871
<211> 100
<212> DNA
<213> Escherichia coli K-12 MG1655
<220> b2681 b2681
<222> 666-765
<223> b2681 b2681 U00096 2806597..2807514
<313> 10871: 687-706
<400> 10871
GGATCTCACC GTGCAGGGCG TGCATATCAC TAACCAGACG GTAATTTATC 50
GGATACATCC TGATGCGCGC AATCGCCTGA CCGCAGGTTA CATGACTAGC 100
<210> 10872
<211> 100
<212> DNA
<213> Escherichia coli K-12 MG1655
<220> b2682 b2683
<222> 639-738
<223> b2682 b2683 U00096 2807638..2808375
<313> 10872: 704-723
<400> 10872
GCTATTTTCT ATTCCCGTCG CCATTCTGGC AGGCATTGTC TGTGGCTGCC 50
TCACTGCGTT AATCCAGGCA TTCTGGCAAG GAGCGCCCGA TGAGCTATGA 100
<210> 10873
<211> 100
<212> DNA
<213> Escherichia coli K-12 MG1655
<220> b2682 b2683
<222> 617-716
<223> b2682 b2683 U00096 2807638..2808375
<313> 10873: 657-676
<400> 10873
TTGGTGCCCT TGCAGGCGTA ACGCTATTTT CTATTCCCGT CGCCATTCTG 50
GCAGGCATTG TCTGTGGCTG CCTCACTGCG TTAATCCAGG CATTCTGGCA 100
<210> 10874
<211> 100
<212> DNA
<213> Escherichia coli K-12 MG1655
<220> b2682 b2683
<222> 520-619
<223> b2682 b2683 U00096 2807638..2808375
<313> 10874: 579-598
<400> 10874
GAAGCTGCAT TAGGTTTTAT GCTTCCGGCA CTCTTTATGA GTTTCCTGCT 50
CGCCTCTTTC CAGCGCAAAC AATCTCTTTG CGTTACCGCA GCGTTAGTTG 100
<210> 10875
<211> 100
<212> DNA
<213> Escherichia coli K-12 MG1655
<220> b1953 b1954
<222> 129-228
<223> b1953 b1954 U00096 2022993..2023235
<313> 10875: 171-190
<400> 10875
GAACGATTCT GAACACGTTA GCGTCGATGG ACGTGAATAT CATACATGGC 50
GTGAATTGGC GGATGCCTTC GAACTGGATA TTCATGACTT CAGCGTCTCT 100
<210> 10876
<211> 100
<212> DNA
<213> Escherichia coli K-12 MG1655
<220> b1953 b1954
<222> 76-175

<223> b1953 b1954 U00096 2022993..2023235
<313> 10876: 127-146
<400> 10876
GCCCTGCTGG CGGCGATCAA TGAAATTAGC GAAAGCGAAG TTCATCGCAG 50
CCAGAACGAT TCTGAACACG TTAGCGTCGA TGGACGTGAA TATCATACAT 100
<210> 10877
<211> 100
<212> DNA
<213> Escherichia coli K-12 MG1655
<220> b1953 b1954
<222> 1-100
<223> b1953 b1954 U00096 2022993..2023235
<313> 10877: 32-51
<400> 10877
TTGCGAGGAA AGATGATGAA AACCGCAAAA GAGTACAGCG ATACCGCAAA 50
ACGTGAGGTC AGCGTCGATG TCGATGCCCT GCTGGCGGCG ATCAATGAAA 100
<210> 10878
<211> 100
<212> DNA
<213> Escherichia coli K-12 MG1655
<220> b1955 b1955
<222> 717-816
<223> b1955 b1955 U00096 2023533..2024348
<313> 10878: 781-800
<400> 10878
GCATCTGCAT GATGAGGATC CGGCCCGCGT CTGGCGAACG CAGCGTGAAG 50
GACCGGAAGG ATGGCGTGAA GGGCTGGACC ATTTTTTCTC CGCCCGTTAA 100
<210> 10879
<211> 100
<212> DNA
<213> Escherichia coli K-12 MG1655
<220> b1955 b1955
<222> 703-802
<223> b1955 b1955 U00096 2023533..2024348
<313> 10879: 751-770
<400> 10879
AATCGTGAAG GGGTGCATCT GCATGATGAG GATCCGGCCC GCGTCTGGCG 50
AACGCAGCGT GAAGGACCGG AAGGATGGCG TGAAGGGCTG GACCATTTTT 100
<210> 10880
<211> 100
<212> DNA
<213> Escherichia coli K-12 MG1655
<220> b1955 b1955
<222> 646-745
<223> b1955 b1955 U00096 2023533..2024348
<313> 10880: 688-707
<400> 10880
CCAAACGATG CGCCCTTACT GGAGGTAATG GATTACGCGG TGATTGTGAA 50
AGGGCTAAAT CGTGAAGGGG TGCATCTGCA TGATGAGGAT CCGGCCCGCG 100
<210> 10881
<211> 100
<212> DNA
<213> Escherichia coli K-12 MG1655
<220> b1956 b1956
<222> 1611-1710
<223> b1956 b1956 U00096 complement(2024345..2026054)
<313> 10881: 1657-1676
<400> 10881
TGATTATGAT TTTGAACAAC TCCAGTCACT GGCCGACCGT CGGCTTTATC 50
TCGCTAAACA GGCTGGGCGT AATCGGGTAT TCGCGAGCGA TAACGCTTAA 100
<210> 10882
<211> 100
<212> DNA
<213> Escherichia coli K-12 MG1655
<220> b1956 b1956
<222> 1537-1636
<223> b1956 b1956 U00096 complement(2024345..2026054)
<313> 10882: 1561-1580
<400> 10882
GAGATGTTGA TCGCCAAGAG TACGACGATA CGCATCAGTG CCTCGCTGGG 50
GGTAAGCAGC AGCGAGGAAA CCGGTGATTA TGATTTTGAA CAACTCCAGT 100

<210> 10883
<211> 100
<212> DNA
<213> Escherichia coli K-12 MG1655
<220> b1956 b1956
<222> 1480-1579
<223> b1956 b1956 U00096 complement(2024345..2026054)
<313> 10883: 1504-1523
<400> 10883
AGTCTGACGG AGGCTGCGGA GGTCGCAGAA CGTATTCGAC TGAAATTAAA 50
TGAAAAAGAG ATGTTGATCG CCAAGAGTAC GACGATACGC ATCAGTGCCT 100
<210> 10884
<211> 100
<212> DNA
<213> Escherichia coli K-12 MG1655
<220> b1957 b1958
<222> 40-139
<223> b1957 b1958 U00096 complement(2026210..2026392)
<313> 10884: 94-113
<400> 10884
GGCGGCCCGC GGATGATTGT CACTGGATAC TCCAGCGGTA TGGTTGAGTG 50
TCGTTGGTAT GACGGTTACG GGGTCAAGCG GGAAGCTTTT CATGAAACCG 100
<210> 10885
<211> 100
<212> DNA
<213> Escherichia coli K-12 MG1655
<220> b1957 b1958
<222> 65-164
<223> b1957 b1958 U00096 complement(2026210..2026392)
<313> 10885: 81-100
<400> 10885
GATACTCCAG CGGTATGGTT GAGTGTCGTT GGTATGACGG TTACGGGGTC 50
AAGCGGGAAG CTTTTCATGA AACCGAGCTT GTTCCGGGGG AGGGGAGTCG 100
<210> 10886
<211> 100
<212> DNA
<213> Escherichia coli K-12 MG1655
<220> b1957 b1958
<222> 1-100
<223> b1957 b1958 U00096 complement(2026210..2026392)
<313> 10886: 8-27
<400> 10886
ATGAGCTTTA TGGTTAGTGA GGAAGTTACG GTTAAAGAGG GCGGCCCGCG 50
GATGATTGTC ACTGGATACT CCAGCGGTAT GGTTGAGTGT CGTTGGTATG 100
<210> 10887
<211> 100
<212> DNA
<213> Escherichia coli K-12 MG1655
<220> b2689 b2690
<222> 330-429
<223> b2689 b2690 U00096 complement(2814534..2814962)
<313> 10887: 391-410
<400> 10887
CTCGTGGGGA CCGGTAATCT TTTTTTTGTG CCTTGGTAAA GCGTTACGCT 50
ATGTTGCAGT TGCAGCAGCG ACCGTTCAGG GCATGATGTG GTGGCACTAA 100
<210> 10888
<211> 100
<212> DNA
<213> Escherichia coli K-12 MG1655
<220> b2689 b2690
<222> 246-345
<223> b2689 b2690 U00096 complement(2814534..2814962)
<313> 10888: 289-308
<400> 10888
ACGCTATGGT GCAGTCACGC TATTATTAAG CTGGATGCCC GTGGTTGGCG 50
ATTTACTGTG TCTGTTAGCG GGATGGATGC GCATCTCGTG GGGACCGGTA 100
<210> 10889
<211> 100
<212> DNA
<213> Escherichia coli K-12 MG1655
<220> b2689 b2690

<222> 63-162
<223> b2689 b2690 U00096 complement(2814534..2814962)
<313> 10889: 115-134
<400> 10889
ACCCGGCAAC TCCGAAGTCG TGCTGGTGGC AATGTTGCTT TCCGGGATCA 50
GTCATCCCTG GGTTTTAGTT TTAACAGCAA CAATGGGTAA TAGCCTTGGA 100
<210> 10890
<211> 100
<212> DNA
<213> Escherichia coli K-12 MG1655
<220> hcar b2538
<222> 776-875
<223> hcar b2538 U00096 complement(2666026..2666916)
<313> 10890: 806-825
<400> 10890
CCGGAAATGT GCCTTCCATT GCTTTACTGA TGGCGTGGAA GAAAGGGGAG 50
ATGAAGCCGG CATTACGCGA TTTCATCGCC ATTGTGCAGG AACGTTTGGC 100
<210> 10891
<211> 100
<212> DNA
<213> Escherichia coli K-12 MG1655
<220> hcar b2538
<222> 740-839
<223> hcar b2538 U00096 complement(2666026..2666916)
<313> 10891: 759-778
<400> 10891
ATTTTAATAC CGGACAGGTT GTTTTTCGTC CCATTGCCGG AAATGTGCCT 50
TCCATTGCTT TACTGATGGC GTGGAAGAAA GGGGAGATGA AGCCGGCATT 100
<210> 10892
<211> 100
<212> DNA
<213> Escherichia coli K-12 MG1655
<220> hcar b2538
<222> 648-747
<223> hcar b2538 U00096 complement(2666026..2666916)
<313> 10892: 689-708
<400> 10892
AAATATCGTC CAGGTGGCAA CGAATATTCT GGTGACCATG AATCTGGTGG 50
GCATGGGGCT GGGCGTCACT TTGATACCCG GTTATATGAA TAATTTTAAT 100
<210> 10893
<211> 100
<212> DNA
<213> Escherichia coli K-12 MG1655
<220> hcat b2537
<222> 1041-1140
<223> hcat b2537 U00096 complement(2664727..2665866)
<313> 10893: 1116-1135
<400> 10893
TTTCCTGTAT CAATATCTGG GCCACGGCGT GTTCTGGGTA ATGGCGCTGG 50
TGGCGCTTCC GGCAATGTTT TTGCGCCCGA AAGTTGTTCC CTCATGCTGA 100
<210> 10894
<211> 100
<212> DNA
<213> Escherichia coli K-12 MG1655
<220> hcat b2537
<222> 1022-1121
<223> hcat b2537 U00096 complement(2664727..2665866)
<313> 10894: 1061-1080
<400> 10894
TCATGACCGT TTTCGCCGGT TTCCTGTATC AATATCTGGG CCACGGCGTG 50
TTCTGGGTAA TGGCGCTGGT GGCGCTTCCG GCAATGTTTT TGCGCCCGAA 100
<210> 10895
<211> 100
<212> DNA
<213> Escherichia coli K-12 MG1655
<220> hcat b2537
<222> 988-1087
<223> hcat b2537 U00096 complement(2664727..2665866)
<313> 10895: 1019-1038
<400> 10895
TACTCTGCCG TCGCGATGGG CGGCAGTATC GCTATCATGA CCGTTTTCGC 50

CGGTTTCCTG TATCAATATC TGGGCCACGG CGTGTTCTGG GTAATGGCGC 100
<210> 10896
<211> 100
<212> DNA
<213> Escherichia coli K-12 MG1655
<220> cyda b0734
<222> 1473-1572
<223> cyda b0734 U00096 770678..772249
<313> 10896: 1539-1558
<400> 10896
GTTCTTAATG TTCAAGTTTG CACGCCTCGG CCCAAGCAGC CTGAAAACCG 50
GTCGCTATCA CTTTGAGCAG TCTTCCACGA CTACTCAGCC GGCACGCTAA 100
<210> 10897
<211> 100
<212> DNA
<213> Escherichia coli K-12 MG1655
<220> cyda b0734
<222> 1405-1504
<223> cyda b0734 U00096 770678..772249
<313> 10897: 1466-1485
<400> 10897
GCAGGCGATC TCATCTTCTC AATGGTGCTG ATTTGCGGCC TGTATACCCT 50
GTTCCTGGTG GCAGAATTGT TCTTAATGTT CAAGTTTGCA CGCCTCGGCC 100
<210> 10898
<211> 100
<212> DNA
<213> Escherichia coli K-12 MG1655
<220> cyda b0734
<222> 1363-1462
<223> cyda b0734 U00096 770678..772249
<313> 10898: 1428-1447
<400> 10898
GGTGAAGTGC TGCCGACAGC TGTGGCGAAC TCGTCACTGA CCGCAGGCGA 50
TCTCATCTTC TCAATGGTGC TGATTTGCGG CCTGTATACC CTGTTCCTGG 100
<210> 10899
<211> 100
<212> DNA
<213> Escherichia coli K-12 MG1655
<220> cydb b0735
<222> 1039-1138
<223> cydb b0735 U00096 772265..773404
<313> 10899: 1077-1096
<400> 10899
CTGGTACCGA TCATTCTGCT CTACACCGCC TGGTGTTACT GGAAAATGTT 50
CGGTCGTATC ACCAAAGAAG ATATTGAACG TAACACCCAC TCTCTGTACT 100
<210> 10900
<211> 100
<212> DNA
<213> Escherichia coli K-12 MG1655
<220> cydb b0735
<222> 934-1033
<223> cydb b0735 U00096 772265..773404
<313> 10900: 970-989
<400> 10900
CCGTTTGTGA TGCCGTCCAG CACCATGATG AACGCAAGTC TGACAATGTG 50
GGATGCAACT TCCAGCCAGC TGACGCTTAA CGTCATGACC TGGGTTGCGG 100
<210> 10901
<211> 100
<212> DNA
<213> Escherichia coli K-12 MG1655
<220> cydb b0735
<222> 896-995
<223> cydb b0735 U00096 772265..773404
<313> 10901: 940-959
<400> 10901
TGGCCTGCAT CATCCTGACA GCCGGTATCG CAATGTTCCC GTTTGTGATG 50
CCGTCCAGCA CCATGATGAA CGCAAGTCTG ACAATGTGGG ATGCAACTTC 100
<210> 10902
<211> 100
<212> DNA
<213> Escherichia coli K-12 MG1655

<220> mcra b1160
<222> 713-812
<223> mcra b1160 U00096 1209569..1210402
<313> 10902: 750-769
<400> 10902
CTGATACAAC AGATAACTGT GTTGCCCTTT GTCCGAATTG CCATAGAGAA 50
TTGCACTATA GTAAAAATGC AAAAGAACTA ATCGAGATGC TTTACGTTAA 100
<210> 10903
<211> 100
<212> DNA
<213> Escherichia coli K-12 MG1655
<220> mcra b1160
<222> 674-773
<223> mcra b1160 U00096 1209569..1210402
<313> 10903: 697-716
<400> 10903
TGGAAGTACA TCATGTAATT CCCCTGTCTT CAGGTGGTGC TGATACAACA 50
GATAACTGTG TTGCCCTTTG TCCGAATTGC CATAGAGAAT TGCACTATAG 100
<210> 10904
<211> 100
<212> DNA
<213> Escherichia coli K-12 MG1655
<220> mcra b1160
<222> 554-653
<223> mcra b1160 U00096 1209569..1210402
<313> 10904: 578-597
<400> 10904
TACAAAAAGT TTATGTCCGA GACCCGATGG TAAAAGCTTG GATTTTACAG 50
CAAAGTAAAG GTATATGTGA AAACTGTGGT AAAAATGCTC CGTTTTATTT 100
<210> 10905
<211> 100
<212> DNA
<213> Escherichia coli K-12 MG1655
<220> cydc b0887
<222> 1623-1722
<223> cydc b0887 U00096 complement(926697..928418)
<313> 10905: 1697-1716
<400> 10905
AATAATAGTG ATGGACAACG GGCAAATTAT TGAGCAAGGT ACTCACGCAG 50
AACTGCTTGC CAGACAGGGG CGTTATTACC AGTTCAAGCA GGGTTTGTAA 100
<210> 10906
<211> 100
<212> DNA
<213> Escherichia coli K-12 MG1655
<220> cydc b0887
<222> 1595-1694
<223> cydc b0887 U00096 complement(926697..928418)
<313> 10906: 1648-1667
<400> 10906
GACTTCGCGG ACTCTCTCGT TTCCAACAAA TAATAGTGAT GGACAACGGG 50
CAAATTATTG AGCAAGGTAC TCACGCAGAA CTGCTTGCCA GACAGGGGCG 100
<210> 10907
<211> 100
<212> DNA
<213> Escherichia coli K-12 MG1655
<220> cydc b0887
<222> 1525-1624
<223> cydc b0887 U00096 complement(926697..928418)
<313> 10907: 1563-1582
<400> 10907
ACCGAAAGCC AGATCCTTGA ATTGCTTGCA GAAATGATGC GTGAGAAAAC 50
GGTGTTAATG GTCACCCATC GACTTCGCGG ACTCTCTCGT TTCCAACAAA 100
<210> 10908
<211> 100
<212> DNA
<213> Escherichia coli K-12 MG1655
<220> mcrb b4347
<222> 1254-1353
<223> mcrb b4347 U00096 complement(4575528..4576925)
<313> 10908: 1294-1313
<400> 10908

TGGGTTGGAA GATGGCACCT CTCCGGATAC GCAATGGCTT AATGAAATTG 50
TGATGACGGA TATCGCCCCT TTACTCGAAG AATATTTCTT TGATGACCCC 100
<210> 10909
<211> 100
<212> DNA
<213> Escherichia coli K-12 MG1655
<220> mcrb b4347
<222> 1207-1306
<223> mcrb b4347 U00096 complement(4575528..4576925)
<313> 10909: 1240-1259
<400> 10909
ACTATCCTTG GGAAAGGATT CCGCATTGGG CATAGTTACT TCTGCTGTGG 50
GTTGGAAGAT GGCACCTCTC CGGATACGCA ATGGCTTAAT GAAATTGTGA 100
<210> 10910
<211> 100
<212> DNA
<213> Escherichia coli K-12 MG1655
<220> mcrb b4347
<222> 1175-1274
<223> mcrb b4347 U00096 complement(4575528..4576925)
<313> 10910: 1216-1235
<400> 10910
ACGAGTTGAA CCAGGAAATC AGCAAAGAGG CCACTATCCT TGGGAAAGGA 50
TTCCGCATTG GGCATAGTTA CTTCTGCTGT GGGTTGGAAG ATGGCACCTC 100
<210> 10911
<211> 100
<212> DNA
<213> Escherichia coli K-12 MG1655
<220> cydd b0888
<222> 1668-1767
<223> cydd b0888 U00096 complement(928419..930185)
<313> 10911: 1742-1761
<400> 10911
GCAGGATGGC CGGATTATTG AGCAAGGACG TTACGCGGAA TTAAGTGTGG 50
CTGGTGGCCC ATTCGCCACA TTACTGGCCC ATCGTCAGGA GGAGATTTAA 100
<210> 10912
<211> 100
<212> DNA
<213> Escherichia coli K-12 MG1655
<220> cydd b0888
<222> 1662-1761
<223> cydd b0888 U00096 complement(928419..930185)
<313> 10912: 1708-1727
<400> 10912
GGTTATGCAG GATGGCCGGA TTATTGAGCA AGGACGTTAC GCGGAATTAA 50
GTGTGGCTGG TGGCCCATTC GCCACATTAC TGGCCCATCG TCAGGAGGAG 100
<210> 10913
<211> 100
<212> DNA
<213> Escherichia coli K-12 MG1655
<220> cydd b0888
<222> 1617-1716
<223> cydd b0888 U00096 complement(928419..930185)
<313> 10913: 1650-1669
<400> 10913
GGTCACCCAC CAGTTAGAAG ATCTTGCTGA CTGGGATGTC ATTTGGGTTA 50
TGCAGGATGG CCGGATTATT GAGCAAGGAC GTTACGCGGA ATTAAGTGTG 100
<210> 10914
<211> 100
<212> DNA
<213> Escherichia coli K-12 MG1655
<220> mcrc b4346
<222> 825-924
<223> mcrc b4346 U00096 complement(4574482..4575528)
<313> 10914: 877-896
<400> 10914
TTCGCAAAAT CTTTATCAAC TGATGAATTA CTTATGGTCG TTAAAGCCTG 50
AAAATGGCGA AAACATAGGG GGGTTATTAA TATATCCCCA CGTAGATACC 100
<210> 10915
<211> 100
<212> DNA

<213> Escherichia coli K-12 MG1655
<220> mcrc b4346
<222> 598-697
<223> mcrc b4346 U00096 complement(4574482..4575528)
<313> 10915: 640-659
<400> 10915
CTTTATCAAA AGTTTCTTTA TGAATTTTGC CGTCGTGAAT TAACGTCTGC 50
AAACACAACC CGCTCTTATT TAAAATGGGA TGCATCGAGT ATATCGGATC 100
<210> 10916
<211> 100
<212> DNA
<213> Escherichia coli K-12 MG1655
<220> mcrc b4346
<222> 414-513
<223> mcrc b4346 U00096 complement(4574482..4575528)
<313> 10916: 453-472
<400> 10916
GGGCATTAGC ACTCTTCATT TAACTCCGCA GCATTTCAGC TATCTGAATG 50
GCGGAAAAAA TACGCGTTAT TATAAATTCG TTATCAGTGT CTGCAAATTC 100
<210> 10917
<211> 100
<212> DNA
<213> Escherichia coli K-12 MG1655
<220> mcrd b4345
<222> 1164-1263
<223> mcrd b4345 U00096 4573163..4574425
<313> 10917: 1227-1246
<400> 10917
CCAGTTAGTC AAAATGGCTC AGGCCTGCAA GGAAGGGAAT GTCGCCAACA 50
GCGAAGAGAG TTGGGCAACG GATGTGCTGG TGGAGGTGAT CGCCTCCTGA 100
<210> 10918
<211> 100
<212> DNA
<213> Escherichia coli K-12 MG1655
<220> mcrd b4345
<222> 1120-1219
<223> mcrd b4345 U00096 4573163..4574425
<313> 10918: 1153-1172
<400> 10918
TCATCGCAAT GGCATTACTT TGATTTCCGC GATGACGTTT CTTTCCAGTT 50
AGTCAAAATG GCTCAGGCCT GCAAGGAAGG GAATGTCGCC AACAGCGAAG 100
<210> 10919
<211> 100
<212> DNA
<213> Escherichia coli K-12 MG1655
<220> mcrd b4345
<222> 1087-1186
<223> mcrd b4345 U00096 4573163..4574425
<313> 10919: 1125-1144
<400> 10919
GATCAGGGGA TGGGCTACTG GCGCATCAAC TTTTCATCGC AATGGCATTA 50
CTTTGATTTC CGCGATGACG TTTCTTTCCA GTTAGTCAAA ATGGCTCAGG 100
<210> 10920
<211> 100
<212> DNA
<213> Escherichia coli K-12 MG1655
<220> yfha b2555
<222> 1236-1335
<223> yfha b2555 U00096 complement(2685489..2686823)
<313> 10920: 1313-1332
<400> 10920
CAACGTCACC CACGCGGCGA GAATGGCGGG GCGCAACCGG ACAGAATTTT 50
ATAAACTGCT TTCCCGACAC GAGCTGGATG CAAACGATTT CAAGGAATGA 100
<210> 10921
<211> 100
<212> DNA
<213> Escherichia coli K-12 MG1655
<220> yfha b2555
<222> 1227-1326
<223> yfha b2555 U00096 complement(2685489..2686823)
<313> 10921: 1265-1284

<400> 10921
CACCAAAGGC AACGTCACCC ACGCGGCGAG AATGGCGGGG CGCAACCGGA  50
CAGAATTTTA TAAACTGCTT TCCCGACACG AGCTGGATGC AAACGATTTC  100
<210> 10922
<211> 100
<212> DNA
<213> Escherichia coli K-12 MG1655
<220> yfha b2555
<222> 1165-1264
<223> yfha b2555 U00096 complement(2685489..2686823)
<313> 10922: 1226-1245
<400> 10922
CCAACCTTTG TTGAGGCACG TAATCAGTTT GAACTCAACT ATTTGCGTAA  50
GCTGCTGCAA ATCACCAAAG GCAACGTCAC CCACGCGGCG AGAATGGCGG  100
<210> 10923
<211> 100
<212> DNA
<213> Escherichia coli K-12 MG1655
<220> yfhb b2561
<222> 461-560
<223> yfhb b2561 U00096 complement(2695935..2696507)
<313> 10923: 510-529
<400> 10923
CGCTGCGGCT GTACAGTGGC TATAGCGACA GTAATCAGGA CAATCCGCTG  50
CTTTATTTCT GTCAGCATCG TTGGCGAGTA ACCCCGCGCG GTGAACTCCA  100
<210> 10924
<211> 100
<212> DNA
<213> Escherichia coli K-12 MG1655
<220> yfhb b2561
<222> 395-494
<223> yfhb b2561 U00096 complement(2695935..2696507)
<313> 10924: 438-457
<400> 10924
GGGTATTGAC GATGCGTTGT CTGGGACATG AAAAGGTCGC ACAACTGGAG  50
CGCAAAATCG GCACTCCGCT GCGGCTGTAC AGTGGCTATA GCGACAGTAA  100
<210> 10925
<211> 100
<212> DNA
<213> Escherichia coli K-12 MG1655
<220> yfhb b2561
<222> 235-334
<223> yfhb b2561 U00096 complement(2695935..2696507)
<313> 10925: 285-304
<400> 10925
GTTCAGGAGC GATTAACCAC CTACCTGTTA AGTTCCGATG CTGATATCTG  50
GTTGATTACC GGCTCTCCGC AGCCGCTGGT TGAAGCGGTT TATTTCGATA  100
<210> 10926
<211> 100
<212> DNA
<213> Escherichia coli K-12 MG1655
<220> yfhc b2560
<222> 438-537
<223> yfhc b2560 U00096 complement(2695374..2695910)
<313> 10926: 504-523
<400> 10926
AATACTGGCG GATGAGTGCG CGGCGTTGCT CAGTGACTTC TTTCGCATGC  50
GCCGCCAGGA AATTAAAGCG CAGAAAAAAG CGCAATCCTC GACGGATTAA  100
<210> 10927
<211> 100
<212> DNA
<213> Escherichia coli K-12 MG1655
<220> yfhc b2560
<222> 398-497
<223> yfhc b2560 U00096 complement(2695374..2695910)
<313> 10927: 434-453
<400> 10927
ATCATCCGGG TATGAATCAC CGAGTGGAAA TTACGGAAGG AATACTGGCG  50
GATGAGTGCG CGGCGTTGCT CAGTGACTTC TTTCGCATGC GCCGCCAGGA  100
<210> 10928
<211> 100

<212> DNA
<213> Escherichia coli K-12 MG1655
<220> yfhc b2560
<222> 253-352
<223> yfhc b2560 U00096 complement(2695374..2695910)
<313> 10928: 296-315
<400> 10928
CGTCTGATCG ACGCCACGTT GTATGTCACG CTTGAACCAT GTGTAATGTG 50
TGCCGGAGCG ATGATCCACA GTCGCATTGG TCGCGTGGTC TTTGGTGCGC 100
<210> 10929
<211> 100
<212> DNA
<213> Escherichia coli K-12 MG1655
<220> yfhd b2559
<222> 1320-1419
<223> yfhd b2559 U00096 2693959..2695377
<313> 10929: 1369-1388
<400> 10929
TCCTTTTCTC TCGTTTCTTT CCCAGTCGTC ATCAAACTAT TTGACCCATT 50
CTCCCTCTCT GCTGTTTTCC AGGAAAGGGA GTGAAGAGAA ACAAAATTAA 100
<210> 10930
<211> 100
<212> DNA
<213> Escherichia coli K-12 MG1655
<220> yfhd b2559
<222> 1212-1311
<223> yfhd b2559 U00096 2693959..2695377
<313> 10930: 1265-1284
<400> 10930
CCTGGTGGGT TATCTGCAAG AGAAAGAGAA GCAGGCTACA GAAGCGGCGA 50
TGCAACTGGC GCAGGATTAT CCGGCGGTAT CGCCTACGGA GTTGGGCAAA 100
<210> 10931
<211> 100
<212> DNA
<213> Escherichia coli K-12 MG1655
<220> yfhd b2559
<222> 1200-1299
<223> yfhd b2559 U00096 2693959..2695377
<313> 10931: 1225-1244
<400> 10931
GTATCAGATT AGCCTGGTGG GTTATCTGCA AGAGAAAGAG AAGCAGGCTA 50
CAGAAGCGGC GATGCAACTG GCGCAGGATT ATCCGGCGGT ATCGCCTACG 100
<210> 10932
<211> 100
<212> DNA
<213> Escherichia coli K-12 MG1655
<220> yfhe b2528
<222> 417-516
<223> yfhe b2528 U00096 complement(2656972..2657487)
<313> 10932: 484-503
<400> 10932
GACGTGGGAC GCGGCGGCGG ATACCGTGCG TAAGCTGCGT TTTCTCGATA 50
AACTGCGAAG CAGTGCCGAA CAACTCGAAG AAAAACTGCT CGATTTTTAA 100
<210> 10933
<211> 100
<212> DNA
<213> Escherichia coli K-12 MG1655
<220> yfhe b2528
<222> 410-509
<223> yfhe b2528 U00096 complement(2656972..2657487)
<313> 10933: 451-470
<400> 10933
ACAACGAGAC GTGGGACGCG GCGGCGGATA CCGTGCGTAA GCTGCGTTTT 50
CTCGATAAAC TGCGAAGCAG TGCCGAACAA CTCGAAGAAA AACTGCTCGA 100
<210> 10934
<211> 100
<212> DNA
<213> Escherichia coli K-12 MG1655
<220> yfhe b2528
<222> 371-470
<223> yfhe b2528 U00096 complement(2656972..2657487)

<313> 10934: 412-431
<400> 10934
TGTTTGATAC CCGCCATCAG TTGATGGTTG AACAGTTAGA CAACGAGACG 50
TGGGACGCGG CGGCGGATAC CGTGCGTAAG CTGCGTTTTC TCGATAAACT 100
<210> 10935
<211> 100
<212> DNA
<213> Escherichia coli K-12 MG1655
<220> yfhf yfhF
<222> 152-251
<223> yfhf yfhF U00096 complement(2657583..2657906)
<313> 10935: 196-215
<400> 10935
AAGACATCGT GTTTGAAGAC AAAGGCGTGA AAGTCGTGGT CGATGGCAAG 50
AGCCTGCAAT TTCTGGACGG TACGCAGCTG GACTTCGTAA AAGAAGGCCT 100
<210> 10936
<211> 100
<212> DNA
<213> Escherichia coli K-12 MG1655
<220> yfhf yfhF
<222> 108-207
<223> yfhf yfhF U00096 complement(2657583..2657906)
<313> 10936: 154-173
<400> 10936
AGGTATGGCT TATGTACTGG AATTTGTTGA CGAACCGACG CCGGAAGACA 50
TCGTGTTTGA AGACAAAGGC GTGAAAGTCG TGGTCGATGG CAAGAGCCTG 100
<210> 10937
<211> 100
<212> DNA
<213> Escherichia coli K-12 MG1655
<220> yfhf yfhF
<222> 82-181
<223> yfhf yfhF U00096 complement(2657583..2657906)
<313> 10937: 103-122
<400> 10937
CTGGGCGTGA GAACCTCCGG GTGTTCAGGT ATGGCTTATG TACTGGAATT 50
TGTTGACGAA CCGACGCCGG AAGACATCGT GTTTGAAGAC AAAGGCGTGA 100
<210> 10938
<211> 100
<212> DNA
<213> Escherichia coli K-12 MG1655
<220> yfhg b2556
<222> 615-714
<223> yfhg b2556 U00096 complement(2686813..2687526)
<313> 10938: 690-709
<400> 10938
CTCGACCCGC AAACCGGCTG GAAATTTCTC GCCGGATACG CCGCACGAGA 50
GTGAAAAGCC CGCGCCATCC ACCCATGAGG TCACTCCTGA TGAGCCATAA 100
<210> 10939
<211> 100
<212> DNA
<213> Escherichia coli K-12 MG1655
<220> yfhg b2556
<222> 541-640
<223> yfhg b2556 U00096 complement(2686813..2687526)
<313> 10939: 579-598
<400> 10939
CATCACGTTC TACAGCAGCA ACTGGAACTC ACCACCCGCA AGCTGGAAAA 50
CCTGACCGAT ATTGAACGCC AGCTCTCGAC CCGCAAACCG GCTGGAAATT 100
<210> 10940
<211> 100
<212> DNA
<213> Escherichia coli K-12 MG1655
<220> yfhg b2556
<222> 499-598
<223> yfhg b2556 U00096 complement(2686813..2687526)
<313> 10940: 524-543
<400> 10940
CAGCAGTCCA GCGACAGCGA GCTGGATACA TTGCGCCAGC AACATCACGT 50
TCTACAGCAG CAACTGGAAC TCACCACCCG CAAGCTGGAA AACCTGACCG 100
<210> 10941

<211> 100
<212> DNA
<213> Escherichia coli K-12 MG1655
<220> yfhh b2562
<222> 822-921
<223> yfhh b2562 U00096 2696707..2697627
<313> 10941: 868-887
<400> 10941
GTTAACGGAT TTGCTGTTCA TTGCGCTGAT TCAGCAGGAT CTGGAACTGG 50
CACCAGAACG TATTCGTCAT AGTGAAGCGC TGGTGAAAAA ACTGGTCTGA 100
<210> 10942
<211> 100
<212> DNA
<213> Escherichia coli K-12 MG1655
<220> yfhh b2562
<222> 757-856
<223> yfhh b2562 U00096 2696707..2697627
<313> 10942: 794-813
<400> 10942
TATACCATTG CCGAAGAACA AGCGACAAAC AGTGCTTCAA TCTCTGCTTG 50
TCACGCTCAG GGAATGTTAA CGGATTTGCT GTTCATTGCG CTGATTCAGC 100
<210> 10943
<211> 100
<212> DNA
<213> Escherichia coli K-12 MG1655
<220> yfhh b2562
<222> 698-797
<223> yfhh b2562 U00096 2696707..2697627
<313> 10943: 765-784
<400> 10943
TGCTGGCGAT TACCGGCTTT ACTCCGAATG CCCTGCAACA GCGTGCTTCT 50
CATTGCCTGT ATACCATTGC CGAAGAACAA GCGACAAACA GTGCTTCAAT 100
<210> 10944
<211> 100
<212> DNA
<213> Escherichia coli K-12 MG1655
<220> yfhj b2525
<222> 102-201
<223> yfhj b2525 U00096 complement(2654556..2654756)
<313> 10944: 159-178
<400> 10944
GTGGATTTGC GATCTGGAAG ATTTCGACGA CGACCCGCAG GCATCCAACG 50
AGAAAATCCT CGAAGCGATT TTGTTAGTCT GGCTGGACGA GGCCGAATAA 100
<210> 10945
<211> 100
<212> DNA
<213> Escherichia coli K-12 MG1655
<220> yfhj b2525
<222> 26-125
<223> yfhj b2525 U00096 complement(2654556..2654756)
<313> 10945: 73-92
<400> 10945
GCGAAATTGG CGAAGCACTG TACGATGCGT ATCCCGATCT TGATCCGAAA 50
ACGGTTCGAT TCACCGATAT GCATCAGTGG ATTTGCGATC TGGAAGATTT 100
<210> 10946
<211> 100
<212> DNA
<213> Escherichia coli K-12 MG1655
<220> yfhj b2525
<222> 3-102
<223> yfhj b2525 U00096 complement(2654556..2654756)
<313> 10946: 25-44
<400> 10946
GGGACTTAAG TGGACCGATA GCCGCGAAAT TGGCGAAGCA CTGTACGATG 50
CGTATCCCGA TCTTGATCCG AAAACGGTTC GATTCACCGA TATGCATCAG 100
<210> 10947
<211> 100
<212> DNA
<213> Escherichia coli K-12 MG1655
<220> yfhk b2557
<222> 1392-1491

<223> yfhk b2557 U00096 complement(2687691..2689181)
<313> 10947: 1461-1480
<400> 10947
TTGTATTCGC CGTATGCAAG GGGAACTGTA TCTGGTCGAC GAGAGCGGGC  50
AAGACGTTTG TTTCCGCATT GAATTACCGT CGTCGAAAAA CACGAAATAA  100
<210> 10948
<211> 100
<212> DNA
<213> Escherichia coli K-12 MG1655
<220> yfhk b2557
<222> 1348-1447
<223> yfhk b2557 U00096 complement(2687691..2689181)
<313> 10948: 1408-1427
<400> 10948
GGGGCGGTGA AGGGCAGCGG TCTGGGATTA AGCATTGCCA GGGATTGTAT  50
TCGCCGTATG CAAGGGGAAC TGTATCTGGT CGACGAGAGC GGGCAAGACG  100
<210> 10949
<211> 100
<212> DNA
<213> Escherichia coli K-12 MG1655
<220> yfhk b2557
<222> 1315-1414
<223> yfhk b2557 U00096 complement(2687691..2689181)
<313> 10949: 1363-1382
<400> 10949
GAACCCTTTT TTCAGGGAAG CCACCAGCGA AAAGGGGCGG TGAAGGGCAG  50
CGGTCTGGGA TTAAGCATTG CCAGGGATTG TATTCGCCGT ATGCAAGGGG  100
<210> 10950
<211> 100
<212> DNA
<213> Escherichia coli K-12 MG1655
<220> yfhl b2563
<222> 140-239
<223> yfhl b2563 U00096 2697683..2697943
<313> 10950: 182-201
<400> 10950
CACCAACCTG CCAGAAGGTG TGCCCGATCC CCAATACTAT TGTGAAAGAT  50
CCGGCGCATG TCGAGACAGA AGAACAGTTG TGGGATAAAT TTGTGCTGAT  100
<210> 10951
<211> 100
<212> DNA
<213> Escherichia coli K-12 MG1655
<220> yfhl b2563
<222> 110-209
<223> yfhl b2563 U00096 2697683..2697943
<313> 10951: 129-148
<400> 10951
AGTGTACCGA ATGCGTAGGG CACTACGAGA CACCAACCTG CCAGAAGGTG  50
TGCCCGATCC CCAATACTAT TGTGAAAGAT CCGGCGCATG TCGAGACAGA  100
<210> 10952
<211> 100
<212> DNA
<213> Escherichia coli K-12 MG1655
<220> yfhl b2563
<222> 45-144
<223> yfhl b2563 U00096 2697683..2697943
<313> 10952: 74-93
<400> 10952
TGAACCCGAA TGCCCGAATG AGGCGATTTC AATGGGAGAT CATATCTACG  50
AGATTAACAG CGATAAGTGT ACCGAATGCG TAGGGCACTA CGAGACACCA  100
<210> 10953
<211> 100
<212> DNA
<213> Escherichia coli K-12 MG1655
<220> b1963 b1963
<222> 285-384
<223> b1963 b1963 U00096 complement(2031141..2031524)
<313> 10953: 354-373
<400> 10953
ACTGGTCGGA TTTTTATTTA TGGCTCTGGT CGGGGCATTG TTAGGCAGCA  50
TTGCCTGGTA CACCCGACCT CGTCCCGCAC GTAGCAGACG CTATGAATAA  100

<210> 10954
<211> 100
<212> DNA
<213> Escherichia coli K-12 MG1655
<220> b1963 b1963
<222> 198-297
<223> b1963 b1963 U00096 complement(2031141..2031524)
<313> 10954: 236-255
<400> 10954
CATCTGGGTG ATGAGCCGGA TTCCCGATGC CCCCGTGTCG GGATTTGAAA 50
GCGATTTAGG CTCTACCGCG GGAAAGTGGT TAGGGGCACT GGTCGGATTT 100
<210> 10955
<211> 100
<212> DNA
<213> Escherichia coli K-12 MG1655
<220> b1963 b1963
<222> 120-219
<223> b1963 b1963 U00096 complement(2031141..2031524)
<313> 10955: 163-182
<400> 10955
TCGCAGCCCT GGCGGCAAAC TGGCTTTGTG GGCTTTCTAT ACATGGTGTG 50
GCTATTTCGT CTGGGCAATG GCGCGTTACA TCTGGGTGAT GAGCCGGATT 100
<210> 10956
<211> 100
<212> DNA
<213> Escherichia coli K-12 MG1655
<220> b1964 b1964
<222> 361-460
<223> b1964 b1964 U00096 2032043..2032558
<313> 10956: 399-418
<400> 10956
GACTATGGTC GTAACTACGG TGTCGTATAT GACGTAGAAG CATGGACCGA 50
TATGCTGCCA GAATTTGGTG GCGATACCTA TGCTGGCGCT GACAACTTCA 100
<210> 10957
<211> 100
<212> DNA
<213> Escherichia coli K-12 MG1655
<220> b1964 b1964
<222> 263-362
<223> b1964 b1964 U00096 2032043..2032558
<313> 10957: 290-309
<400> 10957
AATTTAACAT TGGCGCAAAC GGTCCTGAAA GCGACAAGGG TAATACCGCA 50
ACGCGTCTGG CATTTGCAGG TTTAGGCTTT GGTCAGAATG GTACTTTCGA 100
<210> 10958
<211> 100
<212> DNA
<213> Escherichia coli K-12 MG1655
<220> b1964 b1964
<222> 125-224
<223> b1964 b1964 U00096 2032043..2032558
<313> 10958: 154-173
<400> 10958
TGGATTTGTA CGGAAAAGTA GTGGGTCTGC ACTACTTCTC TGATGATTCT 50
GGCAGCGATG GCGACATGTC ATATGCCCGT ATCGGTTTCA AAGGTGAAAC 100
<210> 10959
<211> 100
<212> DNA
<213> Escherichia coli K-12 MG1655
<220> yfho yfhO
<222> 1140-1239
<223> yfho yfhO U00096 complement(2658337..2659575)
<313> 10959: 1198-1217
<400> 10959
AGTTCGTAAA TCCATCGGTC GTCTGCGTGA CCTTTCTCCG CTGTGGGAAA 50
TGTACAAGCA GGGCGTGGAT CTGAACAGCA TCGAATGGGC TCATCATTAA 100
<210> 10960
<211> 100
<212> DNA
<213> Escherichia coli K-12 MG1655
<220> yfho yfhO

<222> 1102-1201
<223> yfho yfhO U00096 complement(2658337..2659575)
<313> 10960: 1152-1171
<400> 10960
TTTACTACTG AAGAAGAGAT CGACTACACC ATCGAGTTAG TTCGTAAATC 50
CATCGGTCGT CTGCGTGACC TTTCTCCGCT GTGGGAAATG TACAAGCAGG 100
<210> 10961
<211> 100
<212> DNA
<213> Escherichia coli K-12 MG1655
<220> yfho yfhO
<222> 1047-1146
<223> yfho yfhO U00096 complement(2658337..2659575)
<313> 10961: 1075-1094
<400> 10961
GCTGGGGCTG AACGACGAGC TGGCACATAG CTCTATCCGT TTCTCTTTAG 50
GTCGTTTTAC TACTGAAGAA GAGATCGACT ACACCATCGA GTTAGTTCGT 100
<210> 10962
<211> 100
<212> DNA
<213> Escherichia coli K-12 MG1655
<220> b1965 b1965
<222> 111-210
<223> b1965 b1965 U00096 2032568..2032777
<313> 10962: 177-196
<400> 10962
TTTCGGTATG TCAACATCCT ACGACTTCGA CTTCGGTTTA AGTCTGGGTG 50
CTGCATATCA AACTCTGATC GTTCAGATCG TCAGGTTGGA GTTGGTTTAA 100
<210> 10963
<211> 100
<212> DNA
<213> Escherichia coli K-12 MG1655
<220> b1965 b1965
<222> 84-183
<223> b1965 b1965 U00096 2032568..2032777
<313> 10963: 111-130
<400> 10963
TAGCCTTTCC AAAGAAAATG GTGATGGTTT CGGTATGTCA ACATCCTACG 50
ACTTCGACTT CGGTTTAAGT CTGGGTGCTG CATATCAAAC TCTGATCGTT 100
<210> 10964
<211> 100
<212> DNA
<213> Escherichia coli K-12 MG1655
<220> b1965 b1965
<222> 16-115
<223> b1965 b1965 U00096 2032568..2032777
<313> 10964: 58-77
<400> 10964
CAATATCAGG GTAATAATGA AAGTGGTGGA CCATTCGGGC AGGAAGGTTC 50
CGGTAGTGGT GATGGCCGTA GCCTTTCCAA AGAAAATGGT GATGGTTTCG 100
<210> 10965
<211> 100
<212> DNA
<213> Escherichia coli K-12 MG1655
<220> b1966 b1967
<222> 306-405
<223> b1966 b1967 U00096 2032861..2033265
<313> 10965: 348-367
<400> 10965
TTACAAAATC AACCTGCTGG ACAACGACGA TGACTTCTAC GAAGCAAACG 50
GCATCGCTAC CGATGATATC GTTGCTGTGG GCTTAGTCTA CCAGTTCTAA 100
<210> 10966
<211> 100
<212> DNA
<213> Escherichia coli K-12 MG1655
<220> b1966 b1967
<222> 213-312
<223> b1966 b1967 U00096 2032861..2033265
<313> 10966: 275-294
<400> 10966
CGGCGACCCA CGTTACACCA ATAAAGATCT GGTTAAATAC GTTGATATTG 50

GTGCGACCTA CTACTTCAAC AAAAACATGT CCACCTATGT TGATTACAAA 100
<210> 10967
<211> 100
<212> DNA
<213> Escherichia coli K-12 MG1655
<220> b1966 b1967
<222> 186-285
<223> b1966 b1967 U00096 2032861..2033265
<313> 10967: 220-239
<400> 10967
TTTAGGCGGT TGGGCTCATG ACGGCAACGG CGACCCACGT TACACCAATA 50
AAGATCTGGT TAAATACGTT GATATTGGTG CGACCTACTA CTTCAACAAA 100
<210> 10968
<211> 100
<212> DNA
<213> Escherichia coli K-12 MG1655
<220> guaa b2508
<222> 1475-1574
<223> guaa b2508 U00096 complement(2628978..2630555)
<313> 10968: 1508-1527
<400> 10968
TCCTCGGTCG CGTTTCCAAC CGCATTATCA ATGAAGTGAA CGGTATTTCC 50
CGCGTGGTGT ATGACATCAG CGGCAAGCCG CCAGCTACCA TTGAGTGGGA 100
<210> 10969
<211> 100
<212> DNA
<213> Escherichia coli K-12 MG1655
<220> guaa b2508
<222> 1411-1510
<223> guaa b2508 U00096 complement(2628978..2630555)
<313> 10969: 1435-1454
<400> 10969
TCTCTGCGTG CTGTCGAAAC CATCGACTTT ATGACCGCAC ACTGGGCGCA 50
TCTGCCGTAC GATTTCCTCG GTCGCGTTTC CAACCGCATT ATCAATGAAG 100
<210> 10970
<211> 100
<212> DNA
<213> Escherichia coli K-12 MG1655
<220> guaa b2508
<222> 1354-1453
<223> guaa b2508 U00096 complement(2628978..2630555)
<313> 10970: 1405-1424
<400> 10970
CTGCCGGTAC GTTCCGTTGG CGTAATGGGC GATGGTCGTA AGTATGACTG 50
GGTTGTCTCT CTGCGTGCTG TCGAAACCAT CGACTTTATG ACCGCACACT 100
<210> 10971
<211> 100
<212> DNA
<213> Escherichia coli K-12 MG1655
<220> guab b2509
<222> 1368-1467
<223> guab b2509 U00096 complement(2630624..2632090)
<313> 10971: 1429-1448
<400> 10971
AGCGGAGTTT GTACGTATCA GCGGTGCGGG CATTCAGGAA AGCCACGTTC 50
ACGACGTGAC CATTACTAAA GAGTCCCCGA ACTACCGTCT GGGCTCCTGA 100
<210> 10972
<211> 100
<212> DNA
<213> Escherichia coli K-12 MG1655
<220> guab b2509
<222> 1356-1455
<223> guab b2509 U00096 complement(2630624..2632090)
<313> 10972: 1388-1407
<400> 10972
ACTGCGTACT AAAGCGGAGT TTGTACGTAT CAGCGGTGCG GGCATTCAGG 50
AAAGCCACGT TCACGACGTG ACCATTACTA AAGAGTCCCC GAACTACCGT 100
<210> 10973
<211> 100
<212> DNA
<213> Escherichia coli K-12 MG1655

<220> guab b2509
<222> 1250-1349
<223> guab b2509 U00096 complement(2630624..2632090)
<313> 10973: 1306-1325
<400> 10973
TCGAAGGTCG CGTAGCCTAT AAAGGTCGCC TGAAAGAGAT CATTCACCAG 50
CAGATGGGCG GCCTGCGCTC CTGTATGGGT CTGACCGGCT GTGGTACTAT 100
<210> 10974
<211> 100
<212> DNA
<213> Escherichia coli K-12 MG1655
<220> guac guaC
<222> 945-1044
<223> guac guaC U00096 113444..114487
<313> 10974: 1023-1042
<400> 10974
TTGTACATAC GTTGGGGCTT CACGCCTGAA AGAGCTGACC AAGCGCACCA 50
CGTTTATTCG TGTGCAGGAA CAAGAAAACC GCATCTTCAA CAACCTGTAA 100
<210> 10975
<211> 100
<212> DNA
<213> Escherichia coli K-12 MG1655
<220> guac guaC
<222> 945-1044
<223> guac guaC U00096 113444..114487
<313> 10975: 979-998
<400> 10975
TTGTACATAC GTTGGGGCTT CACGCCTGAA AGAGCTGACC AAGCGCACCA 50
CGTTTATTCG TGTGCAGGAA CAAGAAAACC GCATCTTCAA CAACCTGTAA 100
<210> 10976
<211> 100
<212> DNA
<213> Escherichia coli K-12 MG1655
<220> guac guaC
<222> 902-1001
<223> guac guaC U00096 113444..114487
<313> 10976: 941-960
<400> 10976
TTGAAAATAC CGCGCGAGAT ATTTTGGGCG GCCTGCGTTC AGCTTGTACA 50
TACGTTGGGG CTTCACGCCT GAAAGAGCTG ACCAAGCGCA CCACGTTTAT 100
<210> 10977
<211> 100
<212> DNA
<213> Escherichia coli K-12 MG1655
<220> yfia b2598
<222> 132-231
<223> yfia b2598 U00096 2735174..2735515
<313> 10977: 194-213
<400> 10977
GCCACAAGGG TTTGTTGCTG ACGCCACAAT CAATACACCT AACGGCGTTC 50
TGGTTGCCAG TGGTAAACAT GAAGATATGT ACACCGCAAT TAACGAATTG 100
<210> 10978
<211> 100
<212> DNA
<213> Escherichia coli K-12 MG1655
<220> yfia b2598
<222> 86-185
<223> yfia b2598 U00096 2735174..2735515
<313> 10978: 145-164
<400> 10978
AATGGCAAAC ACATCTGATT AATCCACATA TCATTCTGTC CAAAGAGCCA 50
CAAGGGTTTG TTGCTGACGC CACAATCAAT ACACCTAACG GCGTTCTGGT 100
<210> 10979
<211> 100
<212> DNA
<213> Escherichia coli K-12 MG1655
<220> yfia b2598
<222> 32-131
<223> yfia b2598 U00096 2735174..2735515
<313> 10979: 80-99
<400> 10979

AAATTACTCC GGCCATCCGC CAACATGTCG CAGACCGTCT CGCCAAACTG 50
GAAAAATGGC AAACACATCT GATTAATCCA CATATCATTC TGTCCAAAGA 100
<210> 10980
<211> 100
<212> DNA
<213> Escherichia coli K-12 MG1655
<220> yfib b2606
<222> 384-483
<223> yfib b2606 U00096 2741645..2742127
<313> 10980: 438-457
<400> 10980
CACACAGGGT TTAGGAAAAA AATATCCCAT AGCCAGTAAC AAGACCGCCC 50
AGGGCCGCGC CGAGAACCGC CGCGTCGCAG TGGTGATTAC TACCCCTTAA 100
<210> 10981
<211> 100
<212> DNA
<213> Escherichia coli K-12 MG1655
<220> yfib b2606
<222> 326-425
<223> yfib b2606 U00096 2741645..2742127
<313> 10981: 381-400
<400> 10981
CGAATGTCGT GGCCGATGCA TGGGCTATGG GTGGACAAAT TCCACGCAGC 50
AATCTCACCA CACAGGGTTT AGGAAAAAAA TATCCCATAG CCAGTAACAA 100
<210> 10982
<211> 100
<212> DNA
<213> Escherichia coli K-12 MG1655
<220> yfib b2606
<222> 258-357
<223> yfib b2606 U00096 2741645..2742127
<313> 10982: 312-331
<400> 10982
CCGTATGGAT GGACACACCG ATAACTATGG TGAAGACAGT TACAACGAAG 50
GCTTATCATT GAAACGGGCG AATGTCGTGG CCGATGCATG GGCTATGGGT 100
<210> 10983
<211> 100
<212> DNA
<213> Escherichia coli K-12 MG1655
<220> yfic b2576
<222> 751-850
<223> yfic b2576 U00096 complement(2710047..2710904)
<313> 10983: 801-820
<400> 10983
CCGCAGGCGG GAGAATGCTT TAGCGATCGC TTAGTCATTC GTGGGCCAGA 50
CCAGAACTAT TCCGAAGCGT ATACGGCGCT GACCCAGGCT TTTTATCTGT 100
<210> 10984
<211> 100
<212> DNA
<213> Escherichia coli K-12 MG1655
<220> yfic b2576
<222> 681-780
<223> yfic b2576 U00096 complement(2710047..2710904)
<313> 10984: 704-723
<400> 10984
TTTACGTTTG CGGACGGATG TGGCGGAAAA CGAAGCGCGA CTGCCGCATC 50
GGGTGCTGCT GGCATTCTCC CCGCAGGCGG GAGAATGCTT TAGCGATCGC 100
<210> 10985
<211> 100
<212> DNA
<213> Escherichia coli K-12 MG1655
<220> yfic b2576
<222> 642-741
<223> yfic b2576 U00096 complement(2710047..2710904)
<313> 10985: 656-675
<400> 10985
TAATGGTTTT ACGGAGCTGG CATTAAGCAT GGGCTGGCAT TTACGTTTGC 50
GGACGGATGT GGCGGAAAAC GAAGCGCGAC TGCCGCATCG GGTGCTGCTG 100
<210> 10986
<211> 100
<212> DNA

<213> Escherichia coli K-12 MG1655
<220> yfid b2580
<222> 285-384
<223> yfid b2580 U00096 complement(2714086..2714469)
<313> 10986: 351-370
<400> 10986
GCTGACCATC CGTGTATCCG GTTATGCAGT TCGCTTTAAC TCTCTGACTC 50
CGGAACAGCA GCGCGACGTT ATCGCTCGTA CCTTTACTGA AAGCCTGTAA 100
<210> 10987
<211> 100
<212> DNA
<213> Escherichia coli K-12 MG1655
<220> yfid b2580
<222> 249-348
<223> yfid b2580 U00096 complement(2714086..2714469)
<313> 10987: 308-327
<400> 10987
GGAAGATGCA GTTAAGCATC CGGAAAAATA TCCGCAGCTG ACCATCCGTG 50
TATCCGGTTA TGCAGTTCGC TTTAACTCTC TGACTCCGGA ACAGCAGCGC 100
<210> 10988
<211> 100
<212> DNA
<213> Escherichia coli K-12 MG1655
<220> yfid b2580
<222> 150-249
<223> yfid b2580 U00096 complement(2714086..2714469)
<313> 10988: 206-225
<400> 10988
TGACATTGAA TACCGTGAAG TTCCAGTAGA AGTGAAACCA GAAGTTCGCG 50
TTGAAGGTGG TCAACACCTG AACGTTAACG TTCTGCGTCG CGAAACTCTG 100
<210> 10989
<211> 100
<212> DNA
<213> Escherichia coli K-12 MG1655
<220> yfie b2578
<222> 773-872
<223> yfie b2578 U00096 complement(2712459..2713385)
<313> 10989: 823-842
<400> 10989
CAAAAGAGTT GGAGTGCGGA GAATTAATTG AATTGCCTTT TGGCGAACAG 50
TCGCAGACCA TTACGGCCAT GTGCGCACAT CATGCTGGAA AAGCGGTTAG 100
<210> 10990
<211> 100
<212> DNA
<213> Escherichia coli K-12 MG1655
<220> yfie b2578
<222> 736-835
<223> yfie b2578 U00096 complement(2712459..2713385)
<313> 10990: 769-788
<400> 10990
AATATCGGGG TCAGCTACCT ACCGCGCTTT GCGGTGGCAA AAGAGTTGGA 50
GTGCGGAGAA TTAATTGAAT TGCCTTTTGG CGAACAGTCG CAGACCATTA 100
<210> 10991
<211> 100
<212> DNA
<213> Escherichia coli K-12 MG1655
<220> yfie b2578
<222> 636-735
<223> yfie b2578 U00096 complement(2712459..2713385)
<313> 10991: 676-695
<400> 10991
CCGGCAGATA TTTGAGAGCA CGCTGCGTCA GCGGCGGATC ACGGTGGAAA 50
ACACCATTGA GCTTATAAGT ATCGAAAGCA TCAAGCGTTG TGTCGCGGCG 100
<210> 10992
<211> 100
<212> DNA
<213> Escherichia coli K-12 MG1655
<220> yfif b2582
<222> 939-1038
<223> yfif b2582 U00096 complement(2715511..2716548)
<313> 10992: 998-1017

<400> 10992
GCGCGTGAAG ATTGACGGTA CGGGCAACGT TGCCGGGCTG AATATCTCTG 50
TCGCAACCGG CGTTTTGCTT GGTGAATGGT GGCGTCAGAA TAAAGCCTGA 100
<210> 10993
<211> 100
<212> DNA
<213> Escherichia coli K-12 MG1655
<220> yfif b2582
<222> 885-984
<223> yfif b2582 U00096 complement(2715511..2716548)
<313> 10993: 912-931
<400> 10993
GCTGGGTCAG GAATATGAAG GGTTACCGGA TGCCGCACGC GATCCGAACG 50
ATCTGCGCGT GAAGATTGAC GGTACGGGCA ACGTTGCCGG GCTGAATATC 100
<210> 10994
<211> 100
<212> DNA
<213> Escherichia coli K-12 MG1655
<220> yfif b2582
<222> 792-891
<223> yfif b2582 U00096 complement(2715511..2716548)
<313> 10994: 848-867
<400> 10994
TTTCCGTCAG GCAGGTTACA CCGTAGTGAC CACTTCCAGC GAGCAGGGTA 50
AACCGCTGTT CAAAACTAGT CTGCCAGCGA AAATGGTACT GGTGCTGGGT 100
<210> 10995
<211> 100
<212> DNA
<213> Escherichia coli K-12 MG1655
<220> yfih b2594
<222> 622-721
<223> yfih b2594 U00096 complement(2732323..2733054)
<313> 10995: 655-674
<400> 10995
GAGCAAATTT TCGGCGGCGA CCGTTGTACA TATACGGAAA ATGAGACTTT 50
CTTCTCTTAT CGTCGCGACA AGACCACCGG TCGTATGGCA AGTTTCATTT 100
<210> 10996
<211> 100
<212> DNA
<213> Escherichia coli K-12 MG1655
<220> yfih b2594
<222> 528-627
<223> yfih b2594 U00096 complement(2732323..2733054)
<313> 10996: 589-608
<400> 10996
CGCTAAAGCA AGTGCAGCTT TCATTCAGCA TGGTGATAAG TATCTGGCGG 50
ATATTTATCA GCTTGCCCGG CAGCGTCTGG CGAACGTGGG TGTTGAGCAA 100
<210> 10997
<211> 100
<212> DNA
<213> Escherichia coli K-12 MG1655
<220> yfih b2594
<222> 519-618
<223> yfih b2594 U00096 complement(2732323..2733054)
<313> 10997: 551-570
<400> 10997
GGCAGTAGAC GCTAAAGCAA GTGCAGCTTT CATTCAGCAT GGTGATAAGT 50
ATCTGGCGGA TATTTATCAG CTTGCCCGGC AGCGTCTGGC GAACGTGGGT 100
<210> 10998
<211> 100
<212> DNA
<213> Escherichia coli K-12 MG1655
<220> b1970 b1970
<222> 213-312
<223> b1970 b1970 U00096 2036978..2037391
<313> 10998: 255-274
<400> 10998
TAAGGCACTG TGGCCCGAGC AAACTGCAAC TACGGGCGAT TACCGTGTCG 50
TATTTAAAAC CGGGGACTAT TTCAAGAAAC AAAATCTTGA AAGTTTCTTC 100
<210> 10999
<211> 100

<212> DNA
<213> Escherichia coli K-12 MG1655
<220> b1970 b1970
<222> 181-280
<223> b1970 b1970 U00096 2036978..2037391
<313> 10999: 208-227
<400> 10999
AATACCGCCA AAACAGATAA GGATGGACGA ATTAAGGCAC TGTGGCCCGA 50
GCAAACTGCA ACTACGGGCG ATTACCGTGT CGTATTTAAA ACCGGGGACT 100
<210> 11000
<211> 100
<212> DNA
<213> Escherichia coli K-12 MG1655
<220> b1970 b1970
<222> 116-215
<223> b1970 b1970 U00096 2036978..2037391
<313> 11000: 161-180
<400> 11000
GAAAACCCGC TGCTGACGTG ACAGTCACTC TTGAAAAGAA GGCGGATAAC 50
GGCTGGTTAC AACTTAATAC CGCCAAAACA GATAAGGATG GACGAATTAA 100
<210> 11001
<211> 100
<212> DNA
<213> Escherichia coli K-12 MG1655
<220> yfik b2579
<222> 489-588
<223> yfik b2579 U00096 2713443..2714030
<313> 11001: 532-551
<400> 11001
GGGGCATCTG TTTCAGCGAT TGTTTCGCCA GTATGGTCGC CAGTTAAATA 50
TCGTGCTTGC CCTGTTGCTG GTCTATTGCG CGGTACGCAT TTTCTATTAA 100
<210> 11002
<211> 100
<212> DNA
<213> Escherichia coli K-12 MG1655
<220> yfik b2579
<222> 357-456
<223> yfik b2579 U00096 2713443..2714030
<313> 11002: 406-425
<400> 11002
AATCATTTTG TACGGTGTTA CGGCACTGTC GACGTTTGTT CTGCCGCAAA 50
CACAGGCGTT AAGCTGGGTA GTTGGCGTCA GCGTTTTGCT GGCGATGATT 100
<210> 11003
<211> 100
<212> DNA
<213> Escherichia coli K-12 MG1655
<220> yfik b2579
<222> 292-391
<223> yfik b2579 U00096 2713443..2714030
<313> 11003: 325-344
<400> 11003
GACGGACTTC AGGCAAAACC AATCAGCTTT TGGGCCAGCT TTGCTTTGCA 50
GTTTGTGAAC GTCAAAATCA TTTTGTACGG TGTTACGGCA CTGTCGACGT 100
<210> 11004
<211> 100
<212> DNA
<213> Escherichia coli K-12 MG1655
<220> b1971 b1971
<222> 906-1005
<223> b1971 b1971 U00096 2037500..2038504
<313> 11004: 967-986
<400> 11004
CATCCTCGAT GTACAGCGCC AGCCAACGCT ACTGTTTAAT GGTTACGCCG 50
CCCAGGTGGC ATCGCTGTAT CGTGGCCTGG ATTTGCGGGA GAATTTCTAA 100
<210> 11005
<211> 100
<212> DNA
<213> Escherichia coli K-12 MG1655
<220> b1971 b1971
<222> 883-982
<223> b1971 b1971 U00096 2037500..2038504

<313> 11005: 935-954
<400> 11005
GAACGATTTA TTGGTTCAGG CGGCATCCTC GATGTACAGC GCCAGCCAAC 50
GCTACTGTTT AATGGTTACG CCGCCCAGGT GGCATCGCTG TATCGTGGCC 100
<210> 11006
<211> 100
<212> DNA
<213> Escherichia coli K-12 MG1655
<220> b1971 b1971
<222> 865-964
<223> b1971 b1971 U00096 2037500..2038504
<313> 11006: 897-916
<400> 11006
CGCTGGTCAC AGGCTACCGA ACGATTTATT GGTTCAGGCG GCATCCTCGA 50
TGTACAGCGC CAGCCAACGC TACTGTTTAA TGGTTACGCC GCCCAGGTGG 100
<210> 11007
<211> 100
<212> DNA
<213> Escherichia coli K-12 MG1655
<220> yfil yfiL
<222> 306-405
<223> yfil yfiL U00096 2739341..2739745
<313> 11007: 379-398
<400> 11007
CTTTCCTGCC AGCTGCAACA ATGTTGAAAA TGCCAGCCAA CTGCATGAAG 50
TATGGCAAAA AGGGGCTGAT GAAAATGCCA GCACAATACG ATTAAATTAA 100
<210> 11008
<211> 100
<212> DNA
<213> Escherichia coli K-12 MG1655
<220> yfil yfiL
<222> 247-346
<223> yfil yfiL U00096 2739341..2739745
<313> 11008: 268-287
<400> 11008
GGACAAAAGA AAACTTGCCA GACCGATTTT ACTTATGCCC GAGGACTTTC 50
CGGTAAAAGC TTTCCTGCCA GCTGCAACAA TGTTGAAAAT GCCAGCCAAC 100
<210> 11009
<211> 100
<212> DNA
<213> Escherichia coli K-12 MG1655
<220> yfil yfiL
<222> 90-189
<223> yfil yfiL U00096 2739341..2739745
<313> 11009: 116-135
<400> 11009
TCAGATTGAT CCTTATACTC ACGCGCCAAC CTTGACCAGC ACCGACTGGT 50
ATGATGTCGG TATGGAAGAT GCGATATCGG GCAGCGCCAT AAAAGATGAC 100
<210> 11010
<211> 100
<212> DNA
<213> Escherichia coli K-12 MG1655
<220> b1972 b1972
<222> 518-617
<223> b1972 b1972 U00096 2038505..2039140
<313> 11010: 555-574
<400> 11010
CACCGCAGCC GCTCATCTAC GCTGGGCTGG CTGTACTGCT TTTAGCCTTA 50
CGGTATAAGA AGTTGCGTTC CCTTTTTAAC CGGTTACGCA AGCAGGTTCA 100
<210> 11011
<211> 100
<212> DNA
<213> Escherichia coli K-12 MG1655
<220> b1972 b1972
<222> 475-574
<223> b1972 b1972 U00096 2038505..2039140
<313> 11011: 512-531
<400> 11011
ATTCTGGCCC CAATACATTA TCTGTGGTCC GTGAAGATTA TTTCACCGCA 50
GCCGCTCATC TACGCTGGGC TGGCTGTACT GCTTTTAGCC TTACGGTATA 100
<210> 11012

<211> 100
<212> DNA
<213> Escherichia coli K-12 MG1655
<220> b1972 b1972
<222> 418-517
<223> b1972 b1972 U00096 2038505..2039140
<313> 11012: 448-467
<400> 11012
CAGCGAAAAC TCGGCAAGCA CTGGCAACAG TTGCATAATT TCGTCTATCT 50
TGTCGCGATT CTGGCCCCAA TACATTATCT GTGGTCCGTG AAGATTATTT 100
<210> 11013
<211> 100
<212> DNA
<213> Escherichia coli K-12 MG1655
<220> yfim b2587
<222> 174-273
<223> yfim b2587 U00096 2722199..2722471
<313> 11013: 250-269
<400> 11013
TAGCCGCCCC GAAGGGAGCG GCTGGAGCTG GAAGGATTTG GCCTGGGATG 50
TCGCCGGTGC AAGCACCGGC TATACCGTCT GGCAACTGAC CCGTCACTAA 100
<210> 11014
<211> 100
<212> DNA
<213> Escherichia coli K-12 MG1655
<220> yfim b2587
<222> 155-254
<223> yfim b2587 U00096 2722199..2722471
<313> 11014: 193-212
<400> 11014
CGTCAAAAGA GCTTTGGGAT AGCCGCCCCG AAGGGAGCGG CTGGAGCTGG 50
AAGGATTTGG CCTGGGATGT CGCCGGTGCA AGCACCGGCT ATACCGTCTG 100
<210> 11015
<211> 100
<212> DNA
<213> Escherichia coli K-12 MG1655
<220> yfim b2587
<222> 89-188
<223> yfim b2587 U00096 2722199..2722471
<313> 11015: 121-140
<400> 11015
AGCATCAGGG GATGAGCCGG GATCGCAGTG CCATGTTTGG ATTGATGTTC 50
TCTGTCAGTT TGGGGGCGTC AAAAGAGCTT TGGGATAGCC GCCCCGAAGG 100
<210> 11016
<211> 100
<212> DNA
<213> Escherichia coli K-12 MG1655
<220> b1973 b1974
<222> 268-367
<223> b1973 b1974 U00096 2039397..2040047
<313> 11016: 330-349
<400> 11016
TTTGCTGAAA TTAAAGATTA TTATCACAAA GGTTATGCAA CAGATATCGA 50
GATGATTGGC ATTGAGGACG GCATTGTTGA ATTCCATAGA AATAATGAAA 100
<210> 11017
<211> 100
<212> DNA
<213> Escherichia coli K-12 MG1655
<220> b1973 b1974
<222> 187-286
<223> b1973 b1974 U00096 2039397..2040047
<313> 11017: 241-260
<400> 11017
CAATCCGTTT ATCCTTTACT GCAAAGTGGC AAACTTGACC CCGTCTTTCA 50
GAAGAAAGCG GATGCAGATA AAACTAAAAC ATTTGCTGAA ATTAAAGATT 100
<210> 11018
<211> 100
<212> DNA
<213> Escherichia coli K-12 MG1655
<220> b1973 b1974
<222> 163-262

<223> b1973 b1974 U00096 2039397..2040047
<313> 11018: 191-210
<400> 11018
CTCAGTGACT GGGATGGAGT CTGGCAATCC GTTTATCCTT TACTGCAAAG 50
TGGCAAACTT GACCCCGTCT TTCAGAAGAA AGCGGATGCA GATAAAACTA 100
<210> 11019
<211> 100
<212> DNA
<213> Escherichia coli K-12 MG1655
<220> yfin b2605
<222> 1128-1227
<223> yfin b2605 U00096 2740403..2741629
<313> 11019: 1185-1204
<400> 11019
GATGACCATT GAGCACGCCT CTGCGGAAAA ATTACAAGAG CTTGCCGATC 50
ACAATATGTA TCAGGCCAAA CACCAGCGTG CCGAAAAGCT GGTGAGATAA 100
<210> 11020
<211> 100
<212> DNA
<213> Escherichia coli K-12 MG1655
<220> yfin b2605
<222> 1090-1189
<223> yfin b2605 U00096 2740403..2741629
<313> 11020: 1129-1148
<400> 11020
GGCCATCAGA CCACCATGAC ATTAAGCATT GGTTACGCGA TGACCATTGA 50
GCACGCCTCT GCGGAAAAAT TACAAGAGCT TGCCGATCAC AATATGTATC 100
<210> 11021
<211> 100
<212> DNA
<213> Escherichia coli K-12 MG1655
<220> yfin b2605
<222> 999-1098
<223> yfin b2605 U00096 2740403..2741629
<313> 11021: 1025-1044
<400> 11021
GGTGCTCTAT GATGTACAGT CGGAATCTGA AGTGCAGCAG ATATGTTCAG 50
CACTGACACA AATCTTTAAT CTCCCGTTTG ATCTTCATAA TGGCCATCAG 100
<210> 11022
<211> 100
<212> DNA
<213> Escherichia coli K-12 MG1655
<220> yfip b2584
<222> 624-723
<223> yfip b2584 U00096 2717219..2717941
<313> 11022: 681-700
<400> 11022
AGGGTTAGGC GAGCATTTTA CCCGCTTTAA AACACGCTAT CTGGCAGGAA 50
AAACGCAACA TCTGGGTAGC ATCACAGCAG AACAGTTAGA AAGCGTTTAA 100
<210> 11023
<211> 100
<212> DNA
<213> Escherichia coli K-12 MG1655
<220> yfip b2584
<222> 603-702
<223> yfip b2584 U00096 2717219..2717941
<313> 11023: 658-677
<400> 11023
CGGCGATACC GGGGCGGCGG CAGGGTTAGG CGAGCATTTT ACCCGCTTTA 50
AAACACGCTA TCTGGCAGGA AAAACGCAAC ATCTGGGTAG CATCACAGCA 100
<210> 11024
<211> 100
<212> DNA
<213> Escherichia coli K-12 MG1655
<220> yfip b2584
<222> 493-592
<223> yfip b2584 U00096 2717219..2717941
<313> 11024: 534-553
<400> 11024
CCCGTCATTT CCGTCGATCT TTCCCGGCTT TCTGCCTATC GCCTGCGTGA 50
AGCCCAGGCT GAAGGCCAAT ATTGTACTGC CGAGGTAGCC ATCGCACTGT 100

<210> 11025
<211> 100
<212> DNA
<213> Escherichia coli K-12 MG1655
<220> b1976 b1977
<222> 738-837
<223> b1976 b1977 U00096 2041634..2042470
<313> 11025: 797-816
<400> 11025
ATTGTGGCAA CGTTTCTGCC AATTTTATCA ACAAGATCCT TTGCAGAGAC 50
TGCATCACGC TAATGATACA GACTCGTTTT CGGCGACGAA TGTTCATTAA 100
<210> 11026
<211> 100
<212> DNA
<213> Escherichia coli K-12 MG1655
<220> b1976 b1977
<222> 708-807
<223> b1976 b1977 U00096 2041634..2042470
<313> 11026: 728-747
<400> 11026
CCCAGAACTT TTTGCTCCTC GTTTCCCTTC ATTGTGGCAA CGTTTCTGCC 50
AATTTTATCA ACAAGATCCT TTGCAGAGAC TGCATCACGC TAATGATACA 100
<210> 11027
<211> 100
<212> DNA
<213> Escherichia coli K-12 MG1655
<220> b1976 b1977
<222> 639-738
<223> b1976 b1977 U00096 2041634..2042470
<313> 11027: 664-683
<400> 11027
CATTGATGCT TATGCAGCCA GCGATCCTGC TGAATGTTTT GCCGTACTTT 50
CTGAATATTT CTTTAGCGCC CCAGAACTTT TTGCTCCTCG TTTCCCTTCA 100
<210> 11028
<211> 100
<212> DNA
<213> Escherichia coli K-12 MG1655
<220> yfiq b2585
<222> 2495-2594
<223> yfiq b2585 U00096 2717973..2720633
<313> 11028: 2519-2538
<400> 11028
TTACCTATAC GCGAGATCAC GGACTACAAC GTCTGAATGG TATTACGATG 50
CCAAACAATC GTGGCATGGT GGCGCTAGCC CGCAAGCTCG GGTTTAACGT 100
<210> 11029
<211> 100
<212> DNA
<213> Escherichia coli K-12 MG1655
<220> yfiq b2585
<222> 2386-2485
<223> yfiq b2585 U00096 2717973..2720633
<313> 11029: 2442-2461
<400> 11029
GTCACGCGTG CGATTTCCGA TCCTGATAAC ATCGATGCCG AATTTGCTGT 50
ACTGGTTCGC TCGGATCTCA AAGGGTTAGG CTTAGGTCGA CGCTTAATGG 100
<210> 11030
<211> 100
<212> DNA
<213> Escherichia coli K-12 MG1655
<220> yfiq b2585
<222> 2150-2249
<223> yfiq b2585 U00096 2717973..2720633
<313> 11030: 2210-2229
<400> 11030
GGGTAGAATT GAAAAACGGT GAACGCTGCT TGTTCCGCCC GATTTTGCCA 50
GAAGATGAGC CACAACTTCA GCAATTCATT TCGCGAGTCA CCAAAGAAGA 100
<210> 11031
<211> 100
<212> DNA
<213> Escherichia coli K-12 MG1655
<220> b1978 b1978

<222> 7053-7152
<223> b1978 b1978 U00096 2042885..2050036
<313> 11031: 7089-7108
<400> 11031
AAACTCAAGC GAACAATACG TAGTTTCCCT GGCAACAGGT GATCAAAGCG 50
TATTTGAAAA GCTTGGGTTT GCTTATGCGA CATGTTATAA AAACCTGTGA 100
<210> 11032
<211> 100
<212> DNA
<213> Escherichia coli K-12 MG1655
<220> b1978 b1978
<222> 6990-7089
<223> b1978 b1978 U00096 2042885..2050036
<313> 11032: 7042-7061
<400> 11032
TATGGGGCAT TACACGACTG ACGCTGGTTT TCAATCAAAT ATGTATTGGT 50
CATCTAGTCC CGCAAACTCA AGCGAACAAT ACGTAGTTTC CCTGGCAACA 100
<210> 11033
<211> 100
<212> DNA
<213> Escherichia coli K-12 MG1655
<220> b1978 b1978
<222> 6957-7056
<223> b1978 b1978 U00096 2042885..2050036
<313> 11033: 7009-7028
<400> 11033
AGGCATCGGG TCACTGTACA GTGAATGGGG AGATATGGGG CATTACACGA 50
CTGACGCTGG TTTTCAATCA AATATGTATT GGTCATCTAG TCCCGCAAAC 100
<210> 11034
<211> 100
<212> DNA
<213> Escherichia coli K-12 MG1655
<220> b1979 b1979
<222> 225-324
<223> b1979 b1979 U00096 complement(2050298..2050624)
<313> 11034: 248-267
<400> 11034
AATGGCCGAT GTCTCGGAGT CAGTCGTCGT TGGGCAAAAA ATTGACAATA 50
TAAGTCTGGA TGAATTCGCA AAGGCATTAA AAAGTGTTTT GGCGAGAATA 100
<210> 11035
<211> 100
<212> DNA
<213> Escherichia coli K-12 MG1655
<220> b1979 b1979
<222> 171-270
<223> b1979 b1979 U00096 complement(2050298..2050624)
<313> 11035: 215-234
<400> 11035
CTATAATACC CCTGTTGTTG CAATTTATGC TGATTACAAA ACGCGATGGC 50
CCGCAATGGC CGATGTCTCG GAGTCAGTCG TCGTTGGGCA AAAAATTGAC 100
<210> 11036
<211> 100
<212> DNA
<213> Escherichia coli K-12 MG1655
<220> b1979 b1979
<222> 24-123
<223> b1979 b1979 U00096 complement(2050298..2050624)
<313> 11036: 89-108
<400> 11036
TGAAGATAAA GCCAGGGCAT TAGTGGATTG TTATAATAAT GTCTACCGTT 50
TATCCTTACC ACCCTCGATT AAACGTTCAG CAGCAATCAT AAAAGATGCT 100
<210> 11037
<211> 100
<212> DNA
<213> Escherichia coli K-12 MG1655
<220> yfja b2609
<222> 450-549
<223> yfja b2609 U00096 complement(2743390..2743947)
<313> 11037: 490-509
<400> 11037
TGCGTTTGGT ATCAAGGAAC GTCTCGTACC GTTCCTCGAT GGGCAGGTTA 50

TCAAGAAAGT CGATCTCACT ACTCGTTCAA TCGAAGTAGA TTGGGATCCT 100
<210> 11038
<211> 100
<212> DNA
<213> Escherichia coli K-12 MG1655
<220> yfja b2609
<222> 376-475
<223> yfja b2609 U00096 complement(2743390..2743947)
<313> 11038: 393-412
<400> 11038
GATCTCGGTA AAGTCGTCGA TATGATGGAA ACCGGATCTA ATGACGTTCT 50
CGTCATTAAG GCAAACCTGA AAGATGCGTT TGGTATCAAG GAACGTCTCG 100
<210> 11039
<211> 100
<212> DNA
<213> Escherichia coli K-12 MG1655
<220> yfja b2609
<222> 327-426
<223> yfja b2609 U00096 complement(2743390..2743947)
<313> 11039: 350-369
<400> 11039
CTACTGGAAA GACCTGATGG GCTGCCAGGT AGTAACCACT GAAGGCTACG 50
ATCTCGGTAA AGTCGTCGAT ATGATGGAAA CCGGATCTAA TGACGTTCTC 100
<210> 11040
<211> 100
<212> DNA
<213> Escherichia coli K-12 MG1655
<220> rsea b2573
<222> 552-651
<223> rsea b2573 U00096 complement(2706774..2707424)
<313> 11040: 631-650
<400> 11040
CCGACTCCAC TCTGAACAGC TTCAGTTTGA GCAGGCGCAA ACCCAGCAAG 50
CCGCTGTACA GGTGCCAGGA ATTCAAACTT TAGGAACGCA ATCGCAGTAA 100
<210> 11041
<211> 100
<212> DNA
<213> Escherichia coli K-12 MG1655
<220> rsea b2573
<222> 548-647
<223> rsea b2573 U00096 complement(2706774..2707424)
<313> 11041: 584-603
<400> 11041
AACGCCGACT CCACTCTGAA CAGCTTCAGT TTGAGCAGGC GCAAACCCAG 50
CAAGCCGCTG TACAGGTGCC AGGAATTCAA ACTTTAGGAA CGCAATCGCA 100
<210> 11042
<211> 100
<212> DNA
<213> Escherichia coli K-12 MG1655
<220> rsea b2573
<222> 518-617
<223> rsea b2573 U00096 complement(2706774..2707424)
<313> 11042: 542-561
<400> 11042
TTAATGCAAT GTTGCAGGAT TACGAACTGC AACGCCGACT CCACTCTGAA 50
CAGCTTCAGT TTGAGCAGGC GCAAACCCAG CAAGCCGCTG TACAGGTGCC 100
<210> 11043
<211> 100
<212> DNA
<213> Escherichia coli K-12 MG1655
<220> yfjb b2616
<222> 707-806
<223> yfjb b2616 U00096 2748852..2749730
<313> 11043: 747-766
<400> 11043
GCCGTAACGA CCTGGAAATC AGTTGCGACA GCCAGATAGC ACTGCCGATT 50
CAGGAAGGTG AAGATGTCCT GATTCGTCGC TGTGATTACC ATCTGAATCT 100
<210> 11044
<211> 100
<212> DNA
<213> Escherichia coli K-12 MG1655

<220> yfjb b2616
<222> 672-771
<223> yfjb b2616 U00096 2748852..2749730
<313> 11044: 698-717
<400> 11044
CAGCAGCAGC ACGATCCGTC TGCGTTTTTC GCATCGCCGT AACGACCTGG 50
AAATCAGTTG CGACAGCCAG ATAGCACTGC CGATTCAGGA AGGTGAAGAT 100
<210> 11045
<211> 100
<212> DNA
<213> Escherichia coli K-12 MG1655
<220> yfjb b2616
<222> 606-705
<223> yfjb b2616 U00096 2748852..2749730
<313> 11045: 665-684
<400> 11045
TCTGGATGCG ATTACCCTGG TGCCCATGTT CCCGCATACG TTGTCAGCAC 50
GACCACTGGT CATAAACAGC AGCAGCACGA TCCGTCTGCG TTTTTCGCAT 100
<210> 11046
<211> 100
<212> DNA
<213> Escherichia coli K-12 MG1655
<220> rseb b2572
<222> 858-957
<223> rseb b2572 U00096 complement(2705818..2706774)
<313> 11046: 915-934
<400> 11046
AAGCGTACGT GATAACGCCG AAATCACCAT TGTCGGTGAA CTGCCGCCGC 50
AAACGGCGAA ACGCATTGCC GAGAATATTA AGTTCGGGGC AGCGCAATGA 100
<210> 11047
<211> 100
<212> DNA
<213> Escherichia coli K-12 MG1655
<220> rseb b2572
<222> 755-854
<223> rseb b2572 U00096 complement(2705818..2706774)
<313> 11047: 812-831
<400> 11047
GTCTCTATTC CGACGGATTA TTCAGCTTCT CGGTAAACGT TAACCGCGCT 50
ACGCCATCGA GCACCGATCA GATGTTGCGC ACCGGACGCA GAACCGTCAG 100
<210> 11048
<211> 100
<212> DNA
<213> Escherichia coli K-12 MG1655
<220> rseb b2572
<222> 731-830
<223> rseb b2572 U00096 complement(2705818..2706774)
<313> 11048: 768-787
<400> 11048
TGGACAACAT GCCTATCGAA TCACGTCTCT ATTCCGACGG ATTATTCAGC 50
TTCTCGGTAA ACGTTAACCG CGCTACGCCA TCGAGCACCG ATCAGATGTT 100
<210> 11049
<211> 100
<212> DNA
<213> Escherichia coli K-12 MG1655
<220> rsec b2571
<222> 381-480
<223> rsec b2571 U00096 complement(2705342..2705821)
<313> 11049: 452-471
<400> 11049
GTTTGCTGCC CGGGCAGAAT GGCAACCGAT CATCTTAAGC GTGGCATTGC 50
CGCCTGGCCT TGTGCGATTT GAAACATCTT CGGAAGACGC GAGCCAGTGA 100
<210> 11050
<211> 100
<212> DNA
<213> Escherichia coli K-12 MG1655
<220> rsec b2571
<222> 370-469
<223> rsec b2571 U00096 complement(2705342..2705821)
<313> 11050: 399-418
<400> 11050

TACTCGCGTA AGTTTGCTGC CCGGGCAGAA TGGCAACCGA TCATCTTAAG 50
CGTGGCATTG CCGCCTGGCC TTGTGCGATT TGAAACATCT TCGGAAGACG 100
<210> 11051
<211> 100
<212> DNA
<213> Escherichia coli K-12 MG1655
<220> rsec b2571
<222> 281-380
<223> rsec b2571 U00096 complement(2705342..2705821)
<313> 11051: 324-343
<400> 11051
CGCTATTTCA GCTACTCTTT GCTTCTGACG TCGCAGCATT ATGTGGTGCG 50
ATCCTCGGTG GTATTGGCGG GTTCCTGATT GCGCGCGGCT ACTCGCGTAA 100
<210> 11052
<211> 100
<212> DNA
<213> Escherichia coli K-12 MG1655
<220> yfjd b2614
<222> 503-602
<223> yfjd b2614 U00096 2747398..2748081
<313> 11052: 559-578
<400> 11052
CGGAAGATGA TGCCCGCACG GTTAATGGCG TCATTCTTGA GGCACTGGAA 50
GAGATCCCTG TCGCAGGCAC CCGCGTGCGT ATTGGCGAGT ACGATATCGA 100
<210> 11053
<211> 100
<212> DNA
<213> Escherichia coli K-12 MG1655
<220> yfjd b2614
<222> 437-536
<223> yfjd b2614 U00096 2747398..2748081
<313> 11053: 486-505
<400> 11053
GTTCGGTGAT TATCGATGGC ACCGCCAACG TGCGGGAAAT CAACAAAGCC 50
TTTAACTGGC ATCTACCGGA AGATGATGCC CGCACGGTTA ATGGCGTCAT 100
<210> 11054
<211> 100
<212> DNA
<213> Escherichia coli K-12 MG1655
<220> yfjd b2614
<222> 387-486
<223> yfjd b2614 U00096 2747398..2748081
<313> 11054: 405-424
<400> 11054
TACGTCGATG TCGCCAACAC TTGCCGAAGA GGTCACGCCG CAAAACGACG 50
GTTCGGTGAT TATCGATGGC ACCGCCAACG TGCGGGAAAT CAACAAAGCC 100
<210> 11055
<211> 100
<212> DNA
<213> Escherichia coli K-12 MG1655
<220> yfjh b2624
<222> 635-734
<223> yfjh b2624 U00096 complement(2755665..2756621)
<313> 11055: 664-683
<400> 11055
GGAGTGCAGG AAAGAAGGGT GGCAAAAGCA AAGCGGAAGT GTACCATATC 50
ATTCAACTGA AGTTGGTTGA GCTAATTAAT GACTCTGTTC CGAATGATGG 100
<210> 11056
<211> 100
<212> DNA
<213> Escherichia coli K-12 MG1655
<220> yfjh b2624
<222> 512-611
<223> yfjh b2624 U00096 complement(2755665..2756621)
<313> 11056: 542-561
<400> 11056
CTGAAAATGA AAACGTTGCC TTTAAAGCAT TTCATCGGGC TGCGGAGTTA 50
TTTGATAAGT GCATAGGTAT GACTTGGTTT AACATCTCTG TTTGTAATCA 100
<210> 11057
<211> 100
<212> DNA

<213> Escherichia coli K-12 MG1655
<220> yfjh b2624
<222> 417-516
<223> yfjh b2624 U00096 complement(2755665..2756621)
<313> 11057: 461-480
<400> 11057
ACCTCTTGTT ATCGAGCCAT TTTCCATTAA TGACATTTTC AAGGCACACC  50
TTATGTTCGC CTCAATCTCT TTTTTCTGGG GAAAATCTAT CATGTCTGAA  100
<210> 11058
<211> 100
<212> DNA
<213> Escherichia coli K-12 MG1655
<220> yfji b2626
<222> 1067-1166
<223> yfji b2626 U00096 2757006..2758415
<313> 11058: 1109-1128
<400> 11058
ACTTCAGCGG TGATGGAGGC GATATATCTG TTACCGCAGT AAAAGCAGCA  50
GTGGAGATAG TGGCTTGGTA TATTGAAGAA TACATCCGCT TGTTCTCTAA  100
<210> 11059
<211> 100
<212> DNA
<213> Escherichia coli K-12 MG1655
<220> yfji b2626
<222> 1011-1110
<223> yfji b2626 U00096 2757006..2758415
<313> 11059: 1045-1064
<400> 11059
GGATTATGCT TCTAAAATGG CGGAGAACAT GGCGAGGCTT GCTGCCTTAC  50
TTCATTACTT CAGCGGTGAT GGAGGCGATA TATCTGTTAC CGCAGTAAAA  100
<210> 11060
<211> 100
<212> DNA
<213> Escherichia coli K-12 MG1655
<220> yfji b2626
<222> 747-846
<223> yfji b2626 U00096 2757006..2758415
<313> 11060: 784-803
<400> 11060
GATTGGATTT TTTGCACGGT GCCTCATGTG CCAGCCTGCT TCAACACAAG  50
GTAACAGAAA AATTTCCAAC CCAATTTTTT CAAATGAACA TTTGCCGGTA  100
<210> 11061
<211> 100
<212> DNA
<213> Escherichia coli K-12 MG1655
<220> yfjj b2627
<222> 528-627
<223> yfjj b2627 U00096 2758568..2759194
<313> 11061: 591-610
<400> 11061
GACTTATAGC GATTTGATGA ATAGGGTGGA TTACATGACC AAAGTAAGGA  50
CTAAAATAGT CGGTGACGGA GACCGTAATT TCGGCTGCAG TCGCGGGTAA  100
<210> 11062
<211> 100
<212> DNA
<213> Escherichia coli K-12 MG1655
<220> yfjj b2627
<222> 451-550
<223> yfjj b2627 U00096 2758568..2759194
<313> 11062: 493-512
<400> 11062
GATAGCTCGG GGAAGTTAGT TAATTACCCG CCAAATGGCA AATACCTTCT  50
CAATCGTAAA AGGGACAACT TTGAGCAGAC TTATAGCGAT TTGATGAATA  100
<210> 11063
<211> 100
<212> DNA
<213> Escherichia coli K-12 MG1655
<220> yfjj b2627
<222> 266-365
<223> yfjj b2627 U00096 2758568..2759194
<313> 11063: 306-325

<400> 11063
GTCGACATTC CACTTTACGT TACGCATGGG TCAGAGAATA TACCAAAAAT 50
AGAAAGCGGC ATTACCATTT GATACTGTGT TTCAATCAGG ATGCTTATTA 100
<210> 11064
<211> 100
<212> DNA
<213> Escherichia coli K-12 MG1655
<220> b1980 b1981
<222> 558-657
<223> b1980 b1981 U00096 complement(2050646..2051350)
<313> 11064: 590-609
<400> 11064
TGAATTGCCA CTAAGTGAGG ATGTACTCGA TGAGGTGCGC GAGGAAATGC 50
GATCGTTAGG ATCTTACATT GCGTTCAATT TAGAAGGTAG CTCGCAGGAA 100
<210> 11065
<211> 100
<212> DNA
<213> Escherichia coli K-12 MG1655
<220> b1980 b1981
<222> 486-585
<223> b1980 b1981 U00096 complement(2050646..2051350)
<313> 11065: 543-562
<400> 11065
CCGGGCTCCC GTACCTATGA CATGGGCATT TATGATGCGT GAGGCGGGTT 50
TTCCAGCAGT CAGGCCAATA TATGAATTGC CACTAAGTGA GGATGTACTC 100
<210> 11066
<211> 100
<212> DNA
<213> Escherichia coli K-12 MG1655
<220> b1980 b1981
<222> 452-551
<223> b1980 b1981 U00096 complement(2050646..2051350)
<313> 11066: 504-523
<400> 11066
TGTGCAAGAA CGCGTCCAGT ATGGATCAGA AACTCCGGGC TCCCGTACCT 50
ATGACATGGG CATTTATGAT GCGTGAGGCG GGTTTTCCAG CAGTCAGGCC 100
<210> 11067
<211> 100
<212> DNA
<213> Escherichia coli K-12 MG1655
<220> yfjk b2628
<222> 2051-2150
<223> yfjk b2628 U00096 complement(2759372..2761561)
<313> 11067: 2083-2102
<400> 11067
ACTTGCTGGA ATTTTCCGAC GAGGCTGATG TGGATGAGTT GAGCCAGTAT 50
CTTCGGGATA CTCAGGACAT TTGGAGTAGA TCGATTGGGT ACGTTGATCA 100
<210> 11068
<211> 100
<212> DNA
<213> Escherichia coli K-12 MG1655
<220> yfjk b2628
<222> 2004-2103
<223> yfjk b2628 U00096 complement(2759372..2761561)
<313> 11068: 2036-2055
<400> 11068
CTTAGAAGAG ATTGGCATTC CCATTCAGAC GTTACACCGA CTGGTAGACT 50
TGCTGGAATT TTCCGACGAG GCTGATGTGG ATGAGTTGAG CCAGTATCTT 100
<210> 11069
<211> 100
<212> DNA
<213> Escherichia coli K-12 MG1655
<220> yfjk b2628
<222> 1954-2053
<223> yfjk b2628 U00096 complement(2759372..2761561)
<313> 11069: 1978-1997
<400> 11069
CATGTCAAAT TGGCGTTCGA AAGTTTCCAT CTACCACCAG GTGTCAATGC 50
CTTAGAAGAG ATTGGCATTC CCATTCAGAC GTTACACCGA CTGGTAGACT 100
<210> 11070
<211> 100

<212> DNA
<213> Escherichia coli K-12 MG1655
<220> yfjl b2629
<222> 1518-1617
<223> yfjl b2629 U00096 complement(2761558..2763174)
<313> 11070: 1549-1568
<400> 11070
GCCGTTTGCG AATCTTTGCA TCCACGTATT CATTTATCCA GCCCCGAGTC 50
TTGAGCGGTT AACCCAATTA GTTGATGAGA AGGTGAGAGA GGTCGTATGA 100
<210> 11071
<211> 100
<212> DNA
<213> Escherichia coli K-12 MG1655
<220> yfjl b2629
<222> 1455-1554
<223> yfjl b2629 U00096 complement(2761558..2763174)
<313> 11071: 1505-1524
<400> 11071
TGACCTGGAG AAAGAAACAG CGGTGCTGTT CGAAAAATTC GCGGCTGACC 50
TCATCGAGGA TTCGCCGTTT GCGAATCTTT GCATCCACGT ATTCATTTAT 100
<210> 11072
<211> 100
<212> DNA
<213> Escherichia coli K-12 MG1655
<220> yfjl b2629
<222> 1406-1505
<223> yfjl b2629 U00096 complement(2761558..2763174)
<313> 11072: 1457-1476
<400> 11072
GCTACGACTC TAATCTACTA ACCGAGCCTG AAACGCCGGG GTTCGAGGAT 50
GACCTGGAGA AAGAAACAGC GGTGCTGTTC GAAAAATTCG CGGCTGACCT 100
<210> 11073
<211> 100
<212> DNA
<213> Escherichia coli K-12 MG1655
<220> yfjm b2630
<222> 165-264
<223> yfjm b2630 U00096 complement(2763534..2763797)
<313> 11073: 224-243
<400> 11073
TCCGGATGGG CAAGGCATCA ACAATATAGA AAGCCATGAA CACGCGATGT 50
TGACATTGCT CAAAGAGCAT CGAGATATGA CCCTACCAAC CAGGAAATAA 100
<210> 11074
<211> 100
<212> DNA
<213> Escherichia coli K-12 MG1655
<220> yfjm b2630
<222> 134-233
<223> yfjm b2630 U00096 complement(2763534..2763797)
<313> 11074: 186-205
<400> 11074
AAGCCATCAT GATGAGCTAT AAGGAGTGGG GTCCGGATGG GCAAGGCATC 50
AACAATATAG AAAGCCATGA ACACGCGATG TTGACATTGC TCAAAGAGCA 100
<210> 11075
<211> 100
<212> DNA
<213> Escherichia coli K-12 MG1655
<220> yfjm b2630
<222> 74-173
<223> yfjm b2630 U00096 complement(2763534..2763797)
<313> 11075: 108-127
<400> 11075
ATTCGCCAGC AGACAAGAAA AAATTCTACG TCGAAACAGA ACGAGCAGAG 50
AGCCCAAAAC AAGCCATCAT GATGAGCTAT AAGGAGTGGG GTCCGGATGG 100
<210> 11076
<211> 100
<212> DNA
<213> Escherichia coli K-12 MG1655
<220> b1983 b1984
<222> 618-717
<223> b1983 b1984 U00096 2054880..2055596

<313> 11076: 654-673
<400> 11076
GCTTTCCCCA GAAGATTTAG AAATCTTTGA AGGGCTTGTT GATGCCCTTG 50
AAGATGACGA CGATGTACAA AAAGTTTATC ATAACGTCGC AAATCTCTAA 100
<210> 11077
<211> 100
<212> DNA
<213> Escherichia coli K-12 MG1655
<220> b1983 b1984
<222> 474-573
<223> b1983 b1984 U00096 2054880..2055596
<313> 11077: 527-546
<400> 11077
TCGTGATGTG ACTGAAGAAG AAGGTAACAT TGTTATTTAT ACTGAACCTA 50
CTGACCTTCA TAAAGGAATC GCGGCTCTAA AAGCAGCTGG AATCACTGAG 100
<210> 11078
<211> 100
<212> DNA
<213> Escherichia coli K-12 MG1655
<220> b1983 b1984
<222> 347-446
<223> b1983 b1984 U00096 2054880..2055596
<313> 11078: 368-387
<400> 11078
AAAAAGGCGG CAATATCGGA GCGGCAGGTT CTGTCAGCTA TATGTTTGAC 50
AATACGGGTG TGATTGTATT TAAAGGGACA GACCCTGACC ATATTTTTGA 100
<210> 11079
<211> 100
<212> DNA
<213> Escherichia coli K-12 MG1655
<220> yfjn b2631
<222> 975-1074
<223> yfjn b2631 U00096 2763939..2765012
<313> 11079: 1034-1053
<400> 11079
AACTGTGGTC GATGCCAGCC GTATGATTTC TGATATGGCC CGGTTGATGG 50
GTAAAGCCAC TAGAGCGTGG GGAATAATCA AGGACTTATA TATTGTTTGA 100
<210> 11080
<211> 100
<212> DNA
<213> Escherichia coli K-12 MG1655
<220> yfjn b2631
<222> 961-1060
<223> yfjn b2631 U00096 2763939..2765012
<313> 11080: 976-995
<400> 11080
CTGTTCCACA TGGAAACTGT GGTCGATGCC AGCCGTATGA TTTCTGATAT 50
GGCCCGGTTG ATGGGTAAAG CCACTAGAGC GTGGGGAATA ATCAAGGACT 100
<210> 11081
<211> 100
<212> DNA
<213> Escherichia coli K-12 MG1655
<220> yfjn b2631
<222> 882-981
<223> yfjn b2631 U00096 2763939..2765012
<313> 11081: 912-931
<400> 11081
ATTTGCAGCT ACTCTTAGAC CGGAACAGAT TAACATCATC AGCACAGCCT 50
ATACTTTTTT TAATGTGGAA CGTCATTCTC TGTTCCACAT GGAAACTGTG 100
<210> 11082
<211> 100
<212> DNA
<213> Escherichia coli K-12 MG1655
<220> yfjo b2632
<222> 222-321
<223> yfjo b2632 U00096 2765056..2765376
<313> 11082: 288-307
<400> 11082
GAGCAAACTG ACTTTTACTT TACGTAATCA TCCTGAGTAC CTAGAAGCCT 50
CTGTTTTGTC TCCCGATGAT GTACGCCAAG TCCTGTCAAT GGATTTTTGA 100
<210> 11083

<211> 100
<212> DNA
<213> Escherichia coli K-12 MG1655
<220> yfjo b2632
<222> 113-212
<223> yfjo b2632 U00096 2765056..2765376
<313> 11083: 150-169
<400> 11083
CTAACGGTCC AGAGTGGAAC CGCTTCGTTA CGCTGGAAAT GAAATATGGC 50
CGGATCATGC TGGATACGGC CAAAATTATT GATGAGCAGG ACGTTCCGAC 100
<210> 11084
<211> 100
<212> DNA
<213> Escherichia coli K-12 MG1655
<220> yfjo b2632
<222> 62-161
<223> yfjo b2632 U00096 2765056..2765376
<313> 11084: 113-132
<400> 11084
CCCTACTGGG TAGTGAGACC ACTGGTGAAA TCCTTTTTGA CCTCTTGTGC 50
GCTAACGGTC CAGAGTGGAA CCGCTTCGTT ACGCTGGAAA TGAAATATGG 100
<210> 11085
<211> 100
<212> DNA
<213> Escherichia coli K-12 MG1655
<220> yfjp b2633
<222> 771-870
<223> yfjp b2633 U00096 2765725..2766594
<313> 11085: 845-864
<400> 11085
CACCTTTCCC CTTATTCCCG CCCCGGTGCG GACCATTATT CAGGCCGTGC 50
GTTCCTCGGT GGTGTCAGTG GCCCGCGCTG TCTGGGATTT CTTCTTCTGA 100
<210> 11086
<211> 100
<212> DNA
<213> Escherichia coli K-12 MG1655
<220> yfjp b2633
<222> 746-845
<223> yfjp b2633 U00096 2765725..2766594
<313> 11086: 803-822
<400> 11086
TAGATACCAT ACTCGATACG GTAAGCACCT TTCCCCTTAT TCCCGCCCCG 50
GTGCGGACCA TTATTCAGGC CGTGCGTTCC TCGGTGGTGT CAGTGGCCCG 100
<210> 11087
<211> 100
<212> DNA
<213> Escherichia coli K-12 MG1655
<220> yfjp b2633
<222> 718-817
<223> yfjp b2633 U00096 2765725..2766594
<313> 11087: 753-772
<400> 11087
CAGGCACGTG ACGATTTCGG TGAAACCGTA GATACCATAC TCGATACGGT 50
AAGCACCTTT CCCCTTATTC CCGCCCCGGT GCGGACCATT ATTCAGGCCG 100
<210> 11088
<211> 100
<212> DNA
<213> Escherichia coli K-12 MG1655
<220> yfjq b2634
<222> 723-822
<223> yfjq b2634 U00096 2766686..2767507
<313> 11088: 766-785
<400> 11088
ACGAACTCAT ACTCGTGCCG TGCGTGGCAT TGACGGGGAT GTGAAGCTTA 50
ACCGTGCGCT GTGGGTGATG GCAGAAACAC TGCTCACGCA ACTGCAGTAG 100
<210> 11089
<211> 100
<212> DNA
<213> Escherichia coli K-12 MG1655
<220> yfjq b2634
<222> 675-774

<223> yfjq b2634 U00096 2766686..2767507
<313> 11089: 710-729
<400> 11089
AGAAAACCTG ATTAAGGGCG GACTCAGTGG GCGTAATGCT AAAGGCGGAC  50
GAACTCATAC TCGTGCCGTG CGTGGCATTG ACGGGGATGT GAAGCTTAAC  100
<210> 11090
<211> 100
<212> DNA
<213> Escherichia coli K-12 MG1655
<220> yfjq b2634
<222> 648-747
<223> yfjq b2634 U00096 2766686..2767507
<313> 11090: 670-689
<400> 11090
CCTTTGGACC ACCTATCAGC GGATTCAAGA AAACCTGATT AAGGGCGGAC  50
TCAGTGGGCG TAATGCTAAA GGCGGACGAA CTCATACTCG TGCCGTGCGT  100
<210> 11091
<211> 100
<212> DNA
<213> Escherichia coli K-12 MG1655
<220> yfjr yfjR
<222> 546-645
<223> yfjr yfjR U00096 2767724..2768425
<313> 11091: 585-604
<400> 11091
AATCCATCTG ATTCAGCCTT TGCAGGAGAC TTTTCGCCGC AATGACAGTC  50
TGTGTCGCCT GGTTGAAGAC CCGGTCTTCA TTCAGGCCTT ACCCCATTTT  100
<210> 11092
<211> 100
<212> DNA
<213> Escherichia coli K-12 MG1655
<220> yfjr yfjR
<222> 507-606
<223> yfjr yfjR U00096 2767724..2768425
<313> 11092: 537-556
<400> 11092
TGAACACGAA GGGCATATCG CCGTATTCAC ACTTGATGAA ATCCATCTGA  50
TTCAGCCTTT GCAGGAGACT TTTCGCCGCA ATGACAGTCT GTGTCGCCTG  100
<210> 11093
<211> 100
<212> DNA
<213> Escherichia coli K-12 MG1655
<220> yfjr yfjR
<222> 454-553
<223> yfjr yfjR U00096 2767724..2768425
<313> 11093: 495-514
<400> 11093
CGCCTAGCTC CCTGCCGGTT ACTCATCCAC CAGCAGACCT GGTATCTGGT  50
GGCTGAACAC GAAGGGCATA TCGCCGTATT CACACTTGAT GAAATCCATC  100
<210> 11094
<211> 100
<212> DNA
<213> Escherichia coli K-12 MG1655
<220> yfjt yfjT
<222> 369-468
<223> yfjt yfjT U00096 2769169..2769636
<313> 11094: 436-455
<400> 11094
CGACGTCTAC GTCAATGACC AGAAAGTTGG ATCGTTTAAC CCTGATGTTC  50
GGAATGCACA AAAGGTCCTG CCAACATTAG CGGACTGCAC TAACGACTGA  100
<210> 11095
<211> 100
<212> DNA
<213> Escherichia coli K-12 MG1655
<220> yfjt yfjT
<222> 369-468
<223> yfjt yfjT U00096 2769169..2769636
<313> 11095: 402-421
<400> 11095
CGACGTCTAC GTCAATGACC AGAAAGTTGG ATCGTTTAAC CCTGATGTTC  50
GGAATGCACA AAAGGTCCTG CCAACATTAG CGGACTGCAC TAACGACTGA  100

<210> 11096
<211> 100
<212> DNA
<213> Escherichia coli K-12 MG1655
<220> yfjt yfjT
<222> 280-379
<223> yfjt yfjT U00096 2769169..2769636
<313> 11096: 316-335
<400> 11096
CAATATGCCA TTCCCTCAAC CCTTGGCTGG CGTAACGGAG ATAATGCCTG 50
GTTCAGCTTC ATCATGGATA TCCGTAAGGC CCGGCAGTTC GACGTCTACG 100
<210> 11097
<211> 100
<212> DNA
<213> Escherichia coli K-12 MG1655
<220> yfjw b2643
<222> 1372-1471
<223> yfjw b2643 U00096 2771339..2773042
<313> 11097: 1410-1429
<400> 11097
CTGAGAGCAA CAGGTATCTC CCAGTTTAGT ATCAACGGGA AACATTTGTC 50
GTTAAGGCCA GAATCGGAGA TTATTATTTC TGAAGGGAGT TTGTATGGAC 100
<210> 11098
<211> 100
<212> DNA
<213> Escherichia coli K-12 MG1655
<220> yfjw b2643
<222> 878-977
<223> yfjw b2643 U00096 2771339..2773042
<313> 11098: 908-927
<400> 11098
TCATACAAGG TGAATCCGCT GATGTGATGC CTAAAGCAGC ATCCAGGTTA 50
AGTTTGAGCA AGCAAGATGA TAAATTTATG CTAGCCTCAA GTGTTACTGA 100
<210> 11099
<211> 100
<212> DNA
<213> Escherichia coli K-12 MG1655
<220> yfjw b2643
<222> 734-833
<223> yfjw b2643 U00096 2771339..2773042
<313> 11099: 784-803
<400> 11099
AGGGTTTAAG TGGTGCTGAT ATTTATATTG TCTCCGGTTG TTATGCCCTT 50
CCAGATTTAC TTCGCAATGT ACCGCTTGAT GCTAAAAAAT CTTTTCTGAA 100
<210> 11100
<211> 100
<212> DNA
<213> Escherichia coli K-12 MG1655
<220> yfjx b2644
<222> 331-430
<223> yfjx b2644 U00096 2773940..2774398
<313> 11100: 376-395
<400> 11100
TGCCTGATTG AATATAGCCA TCACGCCTGC CTCACCGAAT GCGATGCCAT 50
GACGGAGCAC TATTACCGCC TGCGGGATTA CGCTCTGCAG CACCCTGAAT 100
<210> 11101
<211> 100
<212> DNA
<213> Escherichia coli K-12 MG1655
<220> yfjx b2644
<222> 299-398
<223> yfjx b2644 U00096 2773940..2774398
<313> 11101: 332-351
<400> 11101
AAATGAGCGC GGAAGCCGCA GGTATCGCTG TCTGCCTGAT TGAATATAGC 50
CATCACGCCT GCCTCACCGA ATGCGATGCC ATGACGGAGC ACTATTACCG 100
<210> 11102
<211> 100
<212> DNA
<213> Escherichia coli K-12 MG1655
<220> yfjx b2644

<222> 246-345
<223> yfjx b2644 U00096 2773940..2774398
<313> 11102: 285-304
<400> 11102
TGACGGTGAC GATAAATGGC ATTTGCTCAA CGGCATGAAT GGCAATGGTG  50
CGGAAATGAG CGCGGAAGCC GCAGGTATCG CTGTCTGCCT GATTGAATAT  100
<210> 11103
<211> 100
<212> DNA
<213> Escherichia coli K-12 MG1655
<220> yfjy b2645
<222> 384-483
<223> yfjy b2645 U00096 2774407..2774889
<313> 11103: 455-474
<400> 11103
TCTGGTGCAG GCGCTTCAGC TCGTTGATAT CCGTGTCCCT GACCATCTGA  50
TTGTTGGTGG CAGGCAAATC TATTCGTTCG CAGAACACGG TCTGCTTTGA  100
<210> 11104
<211> 100
<212> DNA
<213> Escherichia coli K-12 MG1655
<220> yfjy b2645
<222> 348-447
<223> yfjy b2645 U00096 2774407..2774889
<313> 11104: 400-419
<400> 11104
TACTCCCAGC CAGGCAGATA AGACTATAAC GCAGCGTCTG GTGCAGGCGC  50
TTCAGCTCGT TGATATCCGT GTCCCTGACC ATCTGATTGT TGGTGGCAGG  100
<210> 11105
<211> 100
<212> DNA
<213> Escherichia coli K-12 MG1655
<220> yfjy b2645
<222> 333-432
<223> yfjy b2645 U00096 2774407..2774889
<313> 11105: 365-384
<400> 11105
CCCCTCCGGC GACACTACTC CCAGCCAGGC AGATAAGACT ATAACGCAGC  50
GTCTGGTGCA GGCGCTTCAG CTCGTTGATA TCCGTGTCCC TGACCATCTG  100
<210> 11106
<211> 100
<212> DNA
<213> Escherichia coli K-12 MG1655
<220> yfjz b2646
<222> 219-318
<223> yfjz b2646 U00096 2775136..2775453
<313> 11106: 296-315
<400> 11106
CCAATGCGTC ACCCTGTACC ACAACGGTTT TACCTGCGAA GCCGATACTC  50
TTGGTAGTTG CGGCTACGTA TACATCGCTG TTTACCCCAC TCAACGCTAA  100
<210> 11107
<211> 100
<212> DNA
<213> Escherichia coli K-12 MG1655
<220> yfjz b2646
<222> 174-273
<223> yfjz b2646 U00096 2775136..2775453
<313> 11107: 238-257
<400> 11107
GATAGAGTCG ATGCTGACCA CTGGTGAACT GAATCCCCGC CATGCCCAAT  50
GCGTCACCCT GTACCACAAC GGTTTTACCT GCGAAGCCGA TACTCTTGGT  100
<210> 11108
<211> 100
<212> DNA
<213> Escherichia coli K-12 MG1655
<220> yfjz b2646
<222> 104-203
<223> yfjz b2646 U00096 2775136..2775453
<313> 11108: 128-147
<400> 11108
GTATCACCGG TAAGTTTAGT GACGCCGAAT GTCCTAAGCT GGATGTGGTA  50

TTTCCACATT TTATCAGCCA GATAGAGTCG ATGCTGACCA CTGGTGAACT 100
<210> 11109
<211> 100
<212> DNA
<213> Escherichia coli K-12 MG1655
<220> b1995 b1996
<222> 303-402
<223> b1995 b1996 U00096 2066630..2067049
<313> 11109: 347-366
<400> 11109
GGCAGGCCGT AAACAAATCC GCATTCGTGG CTGGATTTGC CCCTATATTT 50
CCAGACATCT GTTATCACTT AACCCATTAC AAGCCCGCTG CCGCAGATAT 100
<210> 11110
<211> 100
<212> DNA
<213> Escherichia coli K-12 MG1655
<220> b1995 b1996
<222> 242-341
<223> b1995 b1996 U00096 2066630..2067049
<313> 11110: 295-314
<400> 11110
ATGCCTCCAT AGCCGATAAT TTGCAGGACA TCCCCGGAGT AGAGATAACA 50
GACAACTCCT TGGCAGGCCG TAAACAAATC CGCATTCGTG GCTGGATTTG 100
<210> 11111
<211> 100
<212> DNA
<213> Escherichia coli K-12 MG1655
<220> b1995 b1996
<222> 205-304
<223> b1995 b1996 U00096 2066630..2067049
<313> 11111: 255-274
<400> 11111
CAGGTTATTG ACCAACAAAC ATTGCAGAAC TCCACCAATG CCTCCATAGC 50
CGATAATTTG CAGGACATCC CCGGAGTAGA GATAACAGAC AACTCCTTGG 100
<210> 11112
<211> 100
<212> DNA
<213> Escherichia coli K-12 MG1655
<220> ahpc b0606
<222> 465-564
<223> ahpc b0606 U00096 638168..638731
<313> 11112: 537-556
<400> 11112
ACAGTACGTA GCTTCTCACC CAGGTGAAGT TTGCCCGGCT AAATGGAAAG 50
AAGGTGAAGC AACTCTGGCT CCGTCTCTGG ACCTGGTTGG TAAAATCTAA 100
<210> 11113
<211> 100
<212> DNA
<213> Escherichia coli K-12 MG1655
<220> ahpc b0606
<222> 465-564
<223> ahpc b0606 U00096 638168..638731
<313> 11113: 503-522
<400> 11113
ACAGTACGTA GCTTCTCACC CAGGTGAAGT TTGCCCGGCT AAATGGAAAG 50
AAGGTGAAGC AACTCTGGCT CCGTCTCTGG ACCTGGTTGG TAAAATCTAA 100
<210> 11114
<211> 100
<212> DNA
<213> Escherichia coli K-12 MG1655
<220> ahpc b0606
<222> 375-474
<223> ahpc b0606 U00096 638168..638731
<313> 11114: 411-430
<400> 11114
TGACCCGCAG GGTATCATCC AGGCAATCGA AGTTACCGCT GAAGGCATTG 50
GCCGTGACGC GTCTGACCTG CTGCGTAAAA TCAAAGCAGC ACAGTACGTA 100
<210> 11115
<211> 100
<212> DNA
<213> Escherichia coli K-12 MG1655

<220> b1998 b1999
<222> 99-198
<223> b1998 b1999 U00096 2068266..2068526
<313> 11115: 143-162
<400> 11115
TAATTATGGT GTGGGACTGT TGATAGATGA GTCTGCGCTG GAGCGTGTTG 50
AGGTAGTGAA AGGTCCATAT TCCGTACTGT ACGGTTCACA GGCAATTGGC 100
<210> 11116
<211> 100
<212> DNA
<213> Escherichia coli K-12 MG1655
<220> b1998 b1999
<222> 87-186
<223> b1998 b1999 U00096 2068266..2068526
<313> 11116: 138-157
<400> 11116
GCGCGCCGGA GATAATTATG GTGTGGGACT GTTGATAGAT GAGTCTGCGC 50
TGGAGCGTGT TGAGGTAGTG AAAGGTCCAT ATTCCGTACT GTACGGTTCA 100
<210> 11117
<211> 100
<212> DNA
<213> Escherichia coli K-12 MG1655
<220> b1998 b1999
<222> 87-186
<223> b1998 b1999 U00096 2068266..2068526
<313> 11117: 139-158
<400> 11117
GCGCGCCGGA GATAATTATG GTGTGGGACT GTTGATAGAT GAGTCTGCGC 50
TGGAGCGTGT TGAGGTAGTG AAAGGTCCAT ATTCCGTACT GTACGGTTCA 100
<210> 11118
<211> 100
<212> DNA
<213> Escherichia coli K-12 MG1655
<220> ahpf b0607
<222> 1497-1596
<223> ahpf b0607 U00096 638946..640541
<313> 11118: 1574-1593
<400> 11118
TACGACGGTT CCGTACAAGC AGATCATCAT CGCCACTGGC GAAGGTGCCA 50
AAGCCTCTCT GAGTGCTTTT GACTACCTGA TTCGCACCAA AACTGCATAA 100
<210> 11119
<211> 100
<212> DNA
<213> Escherichia coli K-12 MG1655
<220> ahpf b0607
<222> 1475-1574
<223> ahpf b0607 U00096 638946..640541
<313> 11119: 1514-1533
<400> 11119
GCGTGTTCGC AGCGGGTGAC TGTACGACGG TTCCGTACAA GCAGATCATC 50
ATCGCCACTG GCGAAGGTGC CAAAGCCTCT CTGAGTGCTT TTGACTACCT 100
<210> 11120
<211> 100
<212> DNA
<213> Escherichia coli K-12 MG1655
<220> ahpf b0607
<222> 1361-1460
<223> ahpf b0607 U00096 638946..640541
<313> 11120: 1414-1433
<400> 11120
GTATTTTCGT CCAGATTGGT CTGCTGCCGA ACACCAACTG GCTCGAAGGC 50
GCAGTCGAAC GTAACCGCAT GGGCGAGATT ATCATTGATG CGAAATGCGA 100
<210> 11121
<211> 100
<212> DNA
<213> Escherichia coli K-12 MG1655
<220> btub b3967
<222> 1697-1796
<223> btub b3967 U00096 4161218..4163062
<313> 11121: 1751-1770
<400> 11121

TGTGGGATCT TGCGGTTGCG TATCCGGTCA CCTCTCACCT GACAGTTCGT 50
GGTAAAATAG CCAACCTGTT CGACAAAGAT TATGAGACAG TCTATGGCTA 100
<210> 11122
<211> 100
<212> DNA
<213> Escherichia coli K-12 MG1655
<220> btub b3967
<222> 1512-1611
<223> btub b3967 U00096 4161218..4163062
<313> 11122: 1565-1584
<400> 11122
CGATGCGCGC AATGCGATTA CCGACACGCC GTTGTTACGC CGTGCTAAAC 50
AGCAGGTGAA ATACCAGCTC GACTGGCAGT TGTATGACTT CGACTGGGGT 100
<210> 11123
<211> 100
<212> DNA
<213> Escherichia coli K-12 MG1655
<220> btub b3967
<222> 1486-1585
<223> btub b3967 U00096 4161218..4163062
<313> 11123: 1537-1556
<400> 11123
ACGCATACTG TGAGTTATGA TTATGTCGAT GCGCGCAATG CGATTACCGA 50
CACGCCGTTG TTACGCCGTG CTAAACAGCA GGTGAAATAC CAGCTCGACT 100
<210> 11124
<211> 100
<212> DNA
<213> Escherichia coli K-12 MG1655
<220> lnt b0658
<222> 1440-1539
<223> lnt b0658 U00096 complement(688566..690104)
<313> 11124: 1498-1517
<400> 11124
CACACCATAC GCACGTACCG GCAACTGGCC GCTGTGGGTG CTGACGGCAT 50
TGTTTGGTTT TGCTGCTGTG TTGATGAGTC TGCGTCAGCG ACGTAAATAA 100
<210> 11125
<211> 100
<212> DNA
<213> Escherichia coli K-12 MG1655
<220> lnt b0658
<222> 1419-1518
<223> lnt b0658 U00096 complement(688566..690104)
<313> 11125: 1448-1467
<400> 11125
CGTGACGCCG ACCACCGGAC TCACACCATA CGCACGTACC GGCAACTGGC 50
CGCTGTGGGT GCTGACGGCA TTGTTTGGTT TTGCTGCTGT GTTGATGAGT 100
<210> 11126
<211> 100
<212> DNA
<213> Escherichia coli K-12 MG1655
<220> lnt b0658
<222> 1357-1456
<223> lnt b0658 U00096 complement(688566..690104)
<313> 11126: 1391-1410
<400> 11126
GGCCCGCAGG GTGAGATTCA GGCGATGATC CCGCAGTTCA CCCGCGAGGT 50
GTTAACCACT AACGTGACGC CGACCACCGG ACTCACACCA TACGCACGTA 100
<210> 11127
<211> 100
<212> DNA
<213> Escherichia coli K-12 MG1655
<220> btuc b1712
<222> 844-943
<223> btuc b1712 U00096 complement(1792196..1793176)
<313> 11127: 905-924
<400> 11127
GCAGGGGCGA GCGCATTGCT GCTGGCCGAT ATTGTAGCGC GCCTGGCATT 50
AGCTGCCGCA GAGCTGCCTA TTGGCGTGGT CACCGCAACG TTAGGTGCGC 100
<210> 11128
<211> 100
<212> DNA

<213> Escherichia coli K-12 MG1655
<220> btuc b1712
<222> 768-867
<223> btuc b1712 U00096 complement(1792196..1793176)
<313> 11128: 812-831
<400> 11128
TGGTCTGGTG ATCCCCCATA TTCTCCGGTT GTGTGGTTTA ACCGATCATC 50
GCGTATTACT TCCCGGCTGC GCGCTGGCAG GGGCGAGCGC ATTGCTGCTG 100
<210> 11129
<211> 100
<212> DNA
<213> Escherichia coli K-12 MG1655
<220> btuc b1712
<222> 712-811
<223> btuc b1712 U00096 complement(1792196..1793176)
<313> 11129: 763-782
<400> 11129
GCGACCGGCT GGATGGTTGG CGTCAGTGTG GCGCTGGCGG GTGCTATCGG 50
CTTTATTGGT CTGGTGATCC CCCATATTCT CCGGTTGTGT GGTTTAACCG 100
<210> 11130
<211> 100
<212> DNA
<213> Escherichia coli K-12 MG1655
<220> btud b1710
<222> 651-750
<223> btud b1710 U00096 complement(1790833..1791582)
<313> 11130: 714-733
<400> 11130
GGAAGAGGTG CTCACGCCGC CAAATCTGGC GCAGGCCTAT GGGATGAATT 50
TTCGCCGTCT GGATATCGAA GGTCACAGAA TGCTGATTTC GACCATCTGA 100
<210> 11131
<211> 100
<212> DNA
<213> Escherichia coli K-12 MG1655
<220> btud b1710
<222> 630-729
<223> btud b1710 U00096 complement(1790833..1791582)
<313> 11131: 651-670
<400> 11131
AATGCTGGCC AGTGGACGCA GGGAAGAGGT GCTCACGCCG CCAAATCTGG 50
CGCAGGCCTA TGGGATGAAT TTTCGCCGTC TGGATATCGA AGGTCACAGA 100
<210> 11132
<211> 100
<212> DNA
<213> Escherichia coli K-12 MG1655
<220> btud b1710
<222> 574-673
<223> btud b1710 U00096 complement(1790833..1791582)
<313> 11132: 614-633
<400> 11132
GATCTCAACC ACACATTGCG TCATGCGCAT CGGGCGTGGT TGCTAAAAGG 50
TGGAAAAATG CTGGCCAGTG GACGCAGGGA AGAGGTGCTC ACGCCGCCAA 100
<210> 11133
<211> 100
<212> DNA
<213> Escherichia coli K-12 MG1655
<220> btue b1711
<222> 453-552
<223> btue b1711 U00096 complement(1791582..1792133)
<313> 11133: 491-510
<400> 11133
GGTTGGCAGG GACGGAAAAG TCATCCAGCG TTTTTCCCCG GATATGACGC 50
CGGAAGATCC CATTGTGATG GAAAGCATTA AACTGGCGTT GGCAAAATAA 100
<210> 11134
<211> 100
<212> DNA
<213> Escherichia coli K-12 MG1655
<220> btue b1711
<222> 396-495
<223> btue b1711 U00096 complement(1791582..1792133)
<313> 11134: 460-479

<400> 11134
CAAAGGCCGT GCACCGCTGT ACCCGGATGA TATTTTATGG AATTTTGAAA 50
AATTCCTGGT TGGCAGGGAC GGAAAAGTCA TCCAGCGTTT TTCCCCGGAT 100
<210> 11135
<211> 100
<212> DNA
<213> Escherichia coli K-12 MG1655
<220> btue b1711
<222> 331-430
<223> btue b1711 U00096 complement(1791582..1792133)
<313> 11135: 351-370
<400> 11135
TTGATTGCCG CAGCGCCGAC CGCAGTCGCG CCGGAAGAGA GCGGATTCTA 50
TGCCCGTATG GTCAGCAAAG GCCGTGCACC GCTGTACCCG GATGATATTT 100
<210> 11136
<211> 100
<212> DNA
<213> Escherichia coli K-12 MG1655
<220> apag b0051
<222> 279-378
<223> apag b0051 U00096 complement(51229..51606)
<313> 11136: 344-363
<400> 11136
CACCATGCAG GGTCACTACG AAATGATCGA TGAAAATGGC GTCCCTTTCA 50
GCATCGACAT TCCCGTATTC CGACTCGCCG TTCCCACACT CATTCATTAA 100
<210> 11137
<211> 100
<212> DNA
<213> Escherichia coli K-12 MG1655
<220> apag b0051
<222> 248-347
<223> apag b0051 U00096 complement(51229..51606)
<313> 11137: 313-332
<400> 11137
CCAGCGGTGC AATCATTGAA ACCCCGCTGG GCACCATGCA GGGTCACTAC 50
GAAATGATCG ATGAAAATGG CGTCCCTTTC AGCATCGACA TTCCCGTATT 100
<210> 11138
<211> 100
<212> DNA
<213> Escherichia coli K-12 MG1655
<220> apag b0051
<222> 204-303
<223> apag b0051 U00096 complement(51229..51606)
<313> 11138: 271-290
<400> 11138
TGGCGTCCAG CCACTTATCG CGCCTGGCGA AGAGTACCAG TACACCAGCG 50
GTGCAATCAT TGAAACCCCG CTGGGCACCA TGCAGGGTCA CTACGAAATG 100
<210> 11139
<211> 100
<212> DNA
<213> Escherichia coli K-12 MG1655
<220> apah b0050
<222> 744-843
<223> apah b0050 U00096 complement(50380..51222)
<313> 11139: 806-825
<400> 11139
CTGGGGTGGT ACATTAACCT GCCTGCGCTG GGAAGATAAA CAGTATTTTG 50
TCCAGCCGTC GAACCGGCAT AAGGATTTGG GCGAAGCGGC GGCGTCTTAA 100
<210> 11140
<211> 100
<212> DNA
<213> Escherichia coli K-12 MG1655
<220> apah b0050
<222> 738-837
<223> apah b0050 U00096 complement(50380..51222)
<313> 11140: 770-789
<400> 11140
CTGCTGCTGG GGTGGTACAT TAACCTGCCT GCGCTGGGAA GATAAACAGT 50
ATTTTGTCCA GCCGTCGAAC CGGCATAAGG ATTTGGGCGA AGCGGCGGCG 100
<210> 11141
<211> 100

<212> DNA
<213> Escherichia coli K-12 MG1655
<220> apah b0050
<222> 632-731
<223> apah b0050 U00096 complement(50380..51222)
<313> 11141: 697-716
<400> 11141
TTGCGATTCC TGGCCCTGTC GCTGAAGAAT ACAGCATCGC CTTTGGTCAC 50
TGGGCATCGC TGGAGGGCAA AGGTACGCCG GAAGGTATAT ACGCGCTGGA 100
<210> 11142
<211> 100
<212> DNA
<213> Escherichia coli K-12 MG1655
<220> beta b0312
<222> 1534-1633
<223> beta b0312 U00096 complement(324801..326471)
<313> 11142: 1578-1597
<400> 11142
ATCACCGGGA ATTTGAACGC CACGACAATT ATGATTGGCG AGAAAATAGC 50
GGATATGATT CGTGGACAGG AAGCGCTGCC GAGGAGCACG GCGGGATATT 100
<210> 11143
<211> 100
<212> DNA
<213> Escherichia coli K-12 MG1655
<220> beta b0312
<222> 1489-1588
<223> beta b0312 U00096 complement(324801..326471)
<313> 11143: 1542-1561
<400> 11143
TTAGAAGGCC TGCGTGTGGT GGATGCGTCG ATTATGCCGC AGATTATCAC 50
CGGGAATTTG AACGCCACGA CAATTATGAT TGGCGAGAAA ATAGCGGATA 100
<210> 11144
<211> 100
<212> DNA
<213> Escherichia coli K-12 MG1655
<220> beta b0312
<222> 1420-1519
<223> beta b0312 U00096 complement(324801..326471)
<313> 11144: 1447-1466
<400> 11144
CCGTGCGGTA CCTGCAAAAT GGGTTACGAC GAGATGTCCG TGGTTGACGG 50
CGAAGGCCGC GTACACGGGT TAGAAGGCCT GCGTGTGGTG GATGCGTCGA 100
<210> 11145
<211> 100
<212> DNA
<213> Escherichia coli K-12 MG1655
<220> btur b1271
<222> 474-573
<223> btur b1271 U00096 complement(1325791..1326381)
<313> 11145: 521-540
<400> 11145
CACGGGTCGT GGTTGTCATC GGGATATTCT TGAACTGGCA GATACGGTAA 50
GTGAATTACG CCCCGTCAAA CATGCGTTTG ATGCCGGTGT AAAAGCGCAG 100
<210> 11146
<211> 100
<212> DNA
<213> Escherichia coli K-12 MG1655
<220> btur b1271
<222> 422-521
<223> btur b1271 U00096 complement(1325791..1326381)
<313> 11146: 451-470
<400> 11146
AAGAAGTGGT GCAGGCGTTA AATGAACGTC CACATCAACA GACGGTGATT 50
ATCACGGGTC GTGGTTGTCA TCGGGATATT CTTGAACTGG CAGATACGGT 100
<210> 11147
<211> 100
<212> DNA
<213> Escherichia coli K-12 MG1655
<220> btur b1271
<222> 304-403
<223> btur b1271 U00096 complement(1325791..1326381)

<313> 11147: 360-379
<400> 11147
ACCGCCGCCT GCCGCGAAGT CTGGCAACAT GCAAAGCGGA TGCTTGCTGA 50
TTCCTCACTG GATATGGTTT TGCTTGATGA ACTGACGTAT ATGGTGGCGT 100
<210> 11148
<211> 100
<212> DNA
<213> Escherichia coli K-12 MG1655
<220> betb b0313
<222> 1374-1473
<223> betb b0313 U00096 complement(326485..327957)
<313> 11148: 1436-1455
<400> 11148
CTCCGGCATT GGTCGCGAGA ACGGCGTGAT GACGCTCCAG AGTTACACCC 50
AGGTGAAGTC CATCCAGGTT GAGATGGCTA AATTCCAGTC CATATTCTAA 100
<210> 11149
<211> 100
<212> DNA
<213> Escherichia coli K-12 MG1655
<220> betb b0313
<222> 1195-1294
<223> betb b0313 U00096 complement(326485..327957)
<313> 11149: 1250-1269
<400> 11149
GAGTCGGAAG ACGAAGTCAT TCGCCGCGCT AACGATACCG ACTACGGCCT 50
GGCGGCGGGC ATCGTGACAG CGGACCTGAA CCGCGCGCAT CGCGTCATTC 100
<210> 11150
<211> 100
<212> DNA
<213> Escherichia coli K-12 MG1655
<220> betb b0313
<222> 1164-1263
<223> betb b0313 U00096 complement(326485..327957)
<313> 11150: 1204-1223
<400> 11150
CTTCGGGCCA GTGATGTCCA TTCTGACCTA CGAGTCGGAA GACGAAGTCA 50
TTCGCCGCGC TAACGATACC GACTACGGCC TGGCGGCGGG CATCGTGACA 100
<210> 11151
<211> 100
<212> DNA
<213> Escherichia coli K-12 MG1655
<220> pqql b1495
<222> 2574-2673
<223> pqql b1495 U00096 complement(1570431..1573226)
<313> 11151: 2607-2626
<400> 11151
ATACCAGCAA AACGTTCAGC GCAGCCTCGA TATCCAACAG CGTAGCGTTC 50
AACAATTAGC GAACACTATT GTAAATAGTC TTATTCAATA TGACGATCCT 100
<210> 11152
<211> 100
<212> DNA
<213> Escherichia coli K-12 MG1655
<220> pqql b1495
<222> 2518-2617
<223> pqql b1495 U00096 complement(1570431..1573226)
<313> 11152: 2552-2571
<400> 11152
AATGAAGTGA TGGTTAAGCG TCTGGCTAAA GGGATCAGTG AGCAAGAACT 50
GAATGAATAC CAGCAAAACG TTCAGCGCAG CCTCGATATC CAACAGCGTA 100
<210> 11153
<211> 100
<212> DNA
<213> Escherichia coli K-12 MG1655
<220> pqql b1495
<222> 2434-2533
<223> pqql b1495 U00096 complement(1570431..1573226)
<313> 11153: 2468-2487
<400> 11153
GTTGATCCTC AGGCCAAAGA TATCAGTCAT TTGCTGGCTT TTACTTGTCA 50
ACCAGAACGA CATGATGAAC TGTTAACGTT AGCGAATGAA GTGATGGTTA 100
<210> 11154

<211> 100
<212> DNA
<213> Escherichia coli K-12 MG1655
<220> beti b0314
<222> 489-588
<223> beti b0314 U00096 complement(327971..328558)
<313> 11154: 553-572
<400> 11154
ATTATGGCTG CGCGCGGCTC TGAGCGGCAA ACCGCTGGAT AAAACCCGCG 50
CTAATTCCCT GACCCGCCAC TTTATCACTC AGCATCTACC CACCGATTAA 100
<210> 11155
<211> 100
<212> DNA
<213> Escherichia coli K-12 MG1655
<220> beti b0314
<222> 485-584
<223> beti b0314 U00096 complement(327971..328558)
<313> 11155: 517-536
<400> 11155
ATGGATTATG GCTGCGCGCG GCTCTGAGCG GCAAACCGCT GGATAAAACC 50
CGCGCTAATT CCCTGACCCG CCACTTTATC ACTCAGCATC TACCCACCGA 100
<210> 11156
<211> 100
<212> DNA
<213> Escherichia coli K-12 MG1655
<220> beti b0314
<222> 315-414
<223> beti b0314 U00096 complement(327971..328558)
<313> 11156: 379-398
<400> 11156
GATGAAAGCC TGGCTGGCGT TCTGGGCCAG CAGTATGCAT CAGCCGATGC 50
TCTATCGTTT ACAGCAGGTC AGCAGCCGCC GCTTGCTGTC GAATCTGGTG 100
<210> 11157
<211> 100
<212> DNA
<213> Escherichia coli K-12 MG1655
<220> pbpc pbpC
<222> 2177-2276
<223> pbpc pbpC U00096 complement(2643033..2645345)
<313> 11157: 2221-2240
<400> 11157
GGTGGTTTCT GAATGGCGAA CCGTTAACTG AACGCGGGCG CAACGTGACC 50
CTGCATTTGA CGGATAAAGG TGATTATCAA TTGCTGGTGA TGGATGACGT 100
<210> 11158
<211> 100
<212> DNA
<213> Escherichia coli K-12 MG1655
<220> pbpc pbpC
<222> 2025-2124
<223> pbpc pbpC U00096 complement(2643033..2645345)
<313> 11158: 2052-2071
<400> 11158
ACCGGCCTCG ACAAGTTGCC CGCCATACGG TCACGATGCC CAGCTCCCGC 50
TGCAGCTGAC AGGCGTTCGC GATGGCGCGA TTATTAAACG TTTACCGGGC 100
<210> 11159
<211> 100
<212> DNA
<213> Escherichia coli K-12 MG1655
<220> pbpc pbpC
<222> 1985-2084
<223> pbpc pbpC U00096 complement(2643033..2645345)
<313> 11159: 1999-2018
<400> 11159
CCTGGCTGCC CGCATCAGAA CGTCGCGCTG TGCGCTTACC ACCGGCCTCG 50
ACAAGTTGCC CGCCATACGG TCACGATGCC CAGCTCCCGC TGCAGCTGAC 100
<210> 11160
<211> 100
<212> DNA
<213> Escherichia coli K-12 MG1655
<220> pbpg b2135
<222> 843-942

<223> pbpg b2135 U00096 complement(2221958..2222899)
<313> 11160: 921-940
<400> 11160
AACCGGTAAA GTGATGCCTG TGCCGGCAGC AGCGTTGAGC TATAAAAAAC 50
AAAAAGCCGC CCAAATGGCG GCGGCGGGGC AGACGGCACA GAACGATTAA 100
<210> 11161
<211> 100
<212> DNA
<213> Escherichia coli K-12 MG1655
<220> pbpg b2135
<222> 784-883
<223> pbpg b2135 U00096 complement(2221958..2222899)
<313> 11161: 824-843
<400> 11161
GACGCGTTTG GCAAATATAC CCATTTTGCC GATGCCAGCC GCCTGCGTAC 50
CTGGATTGAA ACCGGTAAAG TGATGCCTGT GCCGGCAGCA GCGTTGAGCT 100
<210> 11162
<211> 100
<212> DNA
<213> Escherichia coli K-12 MG1655
<220> pbpg b2135
<222> 693-792
<223> pbpg b2135 U00096 complement(2221958..2222899)
<313> 11162: 709-728
<400> 11162
GTTAACCAAA ACCGGCTTTA CCAATGCGGC GGGCCATTGT CTGGTGATGC 50
GTACGGTTAT CAATAATAAA CCGGTGGCGC TGGTAGTGAT GGACGCGTTT 100
<210> 11163
<211> 100
<212> DNA
<213> Escherichia coli K-12 MG1655
<220> lon b0440
<222> 2253-2352
<223> lon b0440 U00096 458112..460466
<313> 11163: 2290-2309
<400> 11163
AATTGCCGAT CTGGACATTC ATCCTGTGAA GCGCATTGAG GAAGTTCTGA 50
CTCTGGCGCT GCAAAATGAA CCGTCTGGTA TGCAGGTTGT GACTGCAAAA 100
<210> 11164
<211> 100
<212> DNA
<213> Escherichia coli K-12 MG1655
<220> lon b0440
<222> 2168-2267
<223> lon b0440 U00096 458112..460466
<313> 11164: 2228-2247
<400> 11164
TCCTGGCAGC GCATCGCGGC GGGATTAAAA CAGTGCTAAT TCCGTTCGAA 50
AATAAACGCG ATCTGGAAGA GATTCCTGAC AACGTAATTG CCGATCTGGA 100
<210> 11165
<211> 100
<212> DNA
<213> Escherichia coli K-12 MG1655
<220> lon b0440
<222> 2120-2219
<223> lon b0440 U00096 458112..460466
<313> 11165: 2184-2203
<400> 11165
TCACTCTGCG TGGTCAGGTA CTGCCGATCG GTGGTTTGAA AGAAAAACTC 50
CTGGCAGCGC ATCGCGGCGG GATTAAAACA GTGCTAATTC CGTTCGAAAA 100
<210> 11166
<211> 100
<212> DNA
<213> Escherichia coli K-12 MG1655
<220> uraa b2498
<222> 1191-1290
<223> uraa b2498 U00096 complement(2616891..2618180)
<313> 11166: 1268-1287
<400> 11166
CGTCGGTATC GGCTTAAGTT TGATCTTCAA ACTGATCAGT GTGCTGCGCC 50
CTGAAGAAGT GGTGCTGGAC GCAGAAGACG CGGATATAAC AGACAAATAA 100

<210> 11167
<211> 100
<212> DNA
<213> Escherichia coli K-12 MG1655
<220> uraa b2498
<222> 1100-1199
<223> uraa b2498 U00096 complement(2616891..2618180)
<313> 11167: 1151-1170
<400> 11167
TCCTGACTTC CGTGATTTTG ATCATCGGCG TCAGTGGCGC GAAGGTAAAC 50
ATCGGCGCGG CGGAGTTAAA AGGTATGGCG CTGGCGACCA TCGTCGGTAT 100
<210> 11168
<211> 100
<212> DNA
<213> Escherichia coli K-12 MG1655
<220> uraa b2498
<222> 1064-1163
<223> uraa b2498 U00096 complement(2616891..2618180)
<313> 11168: 1109-1128
<400> 11168
AATCGAAAGT GGATTACAAC AAAGCACAGA ACCTGATCCT GACTTCCGTG 50
ATTTTGATCA TCGGCGTCAG TGGCGCGAAG GTAAACATCG GCGCGGCGGA 100
<210> 11169
<211> 100
<212> DNA
<213> Escherichia coli K-12 MG1655
<220> bett b0315
<222> 1934-2033
<223> bett b0315 U00096 328687..330720
<313> 11169: 1986-2005
<400> 11169
AGGTGATCAC CGATATTCTT GACCAGTACG AGCGGCACCT TAACTTTATT 50
CATCTCCATC GTGAAGCGCC GGGCCATAGC GTGATGTTCC CGGACGCGTG 100
<210> 11170
<211> 100
<212> DNA
<213> Escherichia coli K-12 MG1655
<220> bett b0315
<222> 1925-2024
<223> bett b0315 U00096 328687..330720
<313> 11170: 1939-1958
<400> 11170
GCAAAGAGCA GGTGATCACC GATATTCTTG ACCAGTACGA GCGGCACCTT 50
AACTTTATTC ATCTCCATCG TGAAGCGCCG GGCCATAGCG TGATGTTCCC 100
<210> 11171
<211> 100
<212> DNA
<213> Escherichia coli K-12 MG1655
<220> bett b0315
<222> 1861-1960
<223> bett b0315 U00096 328687..330720
<313> 11171: 1902-1921
<400> 11171
ACCTACTACC GGCTGGAAAC CTTCCTGTTA GAAGGCAGCC AGGGCAACGA 50
CCTGATGGAC TACAGCAAAG AGCAGGTGAT CACCGATATT CTTGACCAGT 100
<210> 11172
<211> 100
<212> DNA
<213> Escherichia coli K-12 MG1655
<220> apba b0426
<222> 813-912
<223> apba b0426 U00096 complement(442828..443739)
<313> 11172: 870-889
<400> 11172
TGGGATTGCC GTACCGGAAA ACACCCGCCT GTTTGAAATG GTAAAAAGAA 50
AGGAGAGTGA ATATGAGCGC ATCGGCACTG GTTTGCCTCG CCCCTGGTAG 100
<210> 11173
<211> 100
<212> DNA
<213> Escherichia coli K-12 MG1655
<220> apba b0426

<222> 810-909
<223> apba b0426 U00096 complement(442828..443739)
<313> 11173: 829-848
<400> 11173
GCATGGGATT GCCGTACCGG AAAACACCCG CCTGTTTGAA ATGGTAAAAA 50
GAAAGGAGAG TGAATATGAG CGCATCGGCA CTGGTTTGCC TCGCCCCTGG 100
<210> 11174
<211> 100
<212> DNA
<213> Escherichia coli K-12 MG1655
<220> apba b0426
<222> 755-854
<223> apba b0426 U00096 complement(442828..443739)
<313> 11174: 770-789
<400> 11174
TGCGCCACAC TGAAATCGAC TATATCAATG GTTTTCTCTT ACGCCGCGCC 50
CGCGCGCATG GGATTGCCGT ACCGGAAAAC ACCCGCCTGT TTGAAATGGT 100
<210> 11175
<211> 100
<212> DNA
<213> Escherichia coli K-12 MG1655
<220> dgka b4043
<222> 185-284
<223> dgka b4043 U00096 4254216..4254584
<313> 11175: 210-229
<400> 11175
CCGTGATGCT GGTGATGATT GTGGAAATCC TCAATAGCGC CATCGAAGCA 50
GTGGTTGACC GAATTGGCTC TGAATACCAT GAGCTTTCCG GACGCGCAAA 100
<210> 11176
<211> 100
<212> DNA
<213> Escherichia coli K-12 MG1655
<220> dgka b4043
<222> 115-214
<223> dgka b4043 U00096 4254216..4254584
<313> 11176: 175-194
<400> 11176
GTATTGTTGG CGGTGGTCAT CGCCTGCTGG CTGGATGTGG ACGCGATTAC 50
CCGCGTGCTG CTTATCAGCT CCGTGATGCT GGTGATGATT GTGGAAATCC 100
<210> 11177
<211> 100
<212> DNA
<213> Escherichia coli K-12 MG1655
<220> dgka b4043
<222> 47-146
<223> dgka b4043 U00096 4254216..4254584
<313> 11177: 83-102
<400> 11177
GCTATTCCTG GAAAGGTTTA CGCGCTGCAT GGATCAACGA AGCGGCATTC 50
CGTCAGGAAG GCGTAGCGGT ATTGTTGGCG GTGGTCATCG CCTGCTGGCT 100
<210> 11178
<211> 100
<212> DNA
<213> Escherichia coli K-12 MG1655
<220> lpp b1678
<222> 36-135
<223> lpp b1678 U00096 1755445..1755681
<313> 11178: 77-96
<400> 11178
CCTGGGTTCT ACTCTGCTGG CAGGTTGCTC CAGCAACGCT AAAATCGATC 50
AGCTGTCTTC TGACGTTCAG ACTCTGAACG CTAAAGTTGA CCAGCTGAGC 100
<210> 11179
<211> 100
<212> DNA
<213> Escherichia coli K-12 MG1655
<220> gntu_1 b3437
<222> 900-999
<223> gntu_1 b3437 U00096 complement(3573695..3574693)
<313> 11179: 948-967
<400> 11179
GCAACCGGCG GGGATCATTC TGCTGGTGAT TGGTGCGGGC GGCGTGTTCA 50

AACAGGTGCT GGTTGACTCT GGCGTAGGTC CGGCACTGGG CGAACGTTAA 100
<210> 11180
<211> 100
<212> DNA
<213> Escherichia coli K-12 MG1655
<220> gntu_1 b3437
<222> 827-926
<223> gntu_1 b3437 U00096 complement(3573695..3574693)
<313> 11180: 873-892
<400> 11180
TGGCGATTTA CGGTCTGGCA ATGCGTCAGG GCATGCCAAA AGACAAAGTG 50
ATGGAGATTT GCGGTCACGC GCTGCAACCG GCGGGGATCA TTCTGCTGGT 100
<210> 11181
<211> 100
<212> DNA
<213> Escherichia coli K-12 MG1655
<220> gntu_1 b3437
<222> 782-881
<223> gntu_1 b3437 U00096 complement(3573695..3574693)
<313> 11181: 834-853
<400> 11181
AGTTTATCGG TCATCCGTTT ACCGCGATTC TGGTTGCTTG TCTGGTGGCG 50
ATTTACGGTC TGGCAATGCG TCAGGGCATG CCAAAAGACA AAGTGATGGA 100
<210> 11182
<211> 100
<212> DNA
<213> Escherichia coli K-12 MG1655
<220> gntu_2 b3436
<222> 230-329
<223> gntu_2 b3436 U00096 complement(3573354..3573689)
<313> 11182: 271-290
<400> 11182
CCGGCGCGAC CGAAGCCGAA ACGCTGAAAA CCTGGACCAT GATGGAAACC 50
ATCCTCGGCA CTGTCGGTGC CATCGTTGGG ATGATTGCGT TCCAGCTGTT 100
<210> 11183
<211> 100
<212> DNA
<213> Escherichia coli K-12 MG1655
<220> gntu_2 b3436
<222> 200-299
<223> gntu_2 b3436 U00096 complement(3573354..3573689)
<313> 11183: 263-282
<400> 11183
ACGCCGGTTT CTGGTTGTTC GGTAAATTTA CCGGCGCGAC CGAAGCCGAA 50
ACGCTGAAAA CCTGGACCAT GATGGAAACC ATCCTCGGCA CTGTCGGTGC 100
<210> 11184
<211> 100
<212> DNA
<213> Escherichia coli K-12 MG1655
<220> gntu_2 b3436
<222> 214-313
<223> gntu_2 b3436 U00096 complement(3573354..3573689)
<313> 11184: 235-254
<400> 11184
TTGTTCGGTA AATTTACCGG CGCGACCGAA GCCGAAACGC TGAAAACCTG 50
GACCATGATG GAAACCATCC TCGGCACTGT CGGTGCCATC GTTGGGATGA 100
<210> 11185
<211> 100
<212> DNA
<213> Escherichia coli K-12 MG1655
<220> tag b3550
<222> 465-564
<223> tag b3550 U00096 3710721..3711284
<313> 11185: 501-520
<400> 11185
GTTTGTCGGC ACCACAATCT GTTACTCCTT TATGCAGGCA TGTGGGCTGG 50
TGAATGATCA TGTGGTTGGC TGCTGTTGCT ATCCGGGAAA TAAACCATGA 100
<210> 11186
<211> 100
<212> DNA
<213> Escherichia coli K-12 MG1655

<220> tag b3550
<222> 412-511
<223> tag b3550 U00096 3710721..3711284
<313> 11186: 442-461
<400> 11186
TCTACGTCCG CCTCCGACGC CCTATCTAAG GCACTGAAAA AACGTGGTTT 50
TAAGTTTGTC GGCACCACAA TCTGTTACTC CTTTATGCAG GCATGTGGGC 100
<210> 11187
<211> 100
<212> DNA
<213> Escherichia coli K-12 MG1655
<220> tag b3550
<222> 260-359
<223> tag b3550 U00096 3710721..3711284
<313> 11187: 310-329
<400> 11187
GCCATCGAGG GAAAATTCAG GCAATTATTG GTAATGCGCG GGCGTACCTG 50
CAAATGGAAC AGAACGGCGA ACCGTTTGTC GACTTTGTCT GGTCGTTTGT 100
<210> 11188
<211> 100
<212> DNA
<213> Escherichia coli K-12 MG1655
<220> tap b1886
<222> 1503-1602
<223> tap b1886 U00096 complement(1967407..1969008)
<313> 11188: 1557-1576
<400> 11188
CGACCATCTT TCGTCGCGTG TGGCGGTATT TACCCTTGAA GAACATGAAG 50
TAGCACGACA TGAGTCGGTG CAGTTACAAA TTGCGCCAGT GGTATCCTGA 100
<210> 11189
<211> 100
<212> DNA
<213> Escherichia coli K-12 MG1655
<220> tap b1886
<222> 1433-1532
<223> tap b1886 U00096 complement(1967407..1969008)
<313> 11189: 1484-1503
<400> 11189
AGGTGACTCA GCAGAACGCC TCGCTGGTAG AAGAAGCGGC GGTGGCAACG 50
GAACAACTGG CGAATCAGGC CGACCATCTT TCGTCGCGTG TGGCGGTATT 100
<210> 11190
<211> 100
<212> DNA
<213> Escherichia coli K-12 MG1655
<220> tap b1886
<222> 1406-1505
<223> tap b1886 U00096 complement(1967407..1969008)
<313> 11190: 1436-1455
<400> 11190
TTGCACAGGC TGTCAGCCAG ATGGATCAGG TGACTCAGCA GAACGCCTCG 50
CTGGTAGAAG AAGCGGCGGT GGCAACGGAA CAACTGGCGA ATCAGGCCGA 100
<210> 11191
<211> 100
<212> DNA
<213> Escherichia coli K-12 MG1655
<220> tar b1887
<222> 1563-1662
<223> tar b1887 U00096 complement(1969054..1970715)
<313> 11191: 1626-1645
<400> 11191
CAATAAACCG CAAACACCAT CCCGTCCTGC CAGTGAGCAA CCACCGGCTC 50
AGCCACGACT GCGAATTGCT GAACAAGATC CAAACTGGGA AACATTTTGA 100
<210> 11192
<211> 100
<212> DNA
<213> Escherichia coli K-12 MG1655
<220> tar b1887
<222> 1536-1635
<223> tar b1887 U00096 complement(1969054..1970715)
<313> 11192: 1592-1611
<400> 11192

GTTCCGTCTG GCAGCCAGCC CACTCACCAA TAAACCGCAA ACACCATCCC 50
GTCCTGCCAG TGAGCAACCA CCGGCTCAGC CACGACTGCG AATTGCTGAA 100
<210> 11193
<211> 100
<212> DNA
<213> Escherichia coli K-12 MG1655
<220> tar b1887
<222> 1513-1612
<223> tar b1887 U00096 complement(1969054..1970715)
<313> 11193: 1553-1572
<400> 11193
CGTTTAACGC AAGCAGTTTC CGCGTTCCGT CTGGCAGCCA GCCCACTCAC 50
CAATAAACCG CAAACACCAT CCCGTCCTGC CAGTGAGCAA CCACCGGCTC 100
<210> 11194
<211> 100
<212> DNA
<213> Escherichia coli K-12 MG1655
<220> farr b0731
<222> 539-638
<223> farr b0731 U00096 complement(764376..765098)
<313> 11194: 596-615
<400> 11194
TCATTCCTCT GATGCCAACC GAAGAGATGA GCCGTCTGCT TAATATCAGC 50
CAGACAAAGC CGATTCTTGA AAAAGTATCG CGTGGATATT TAGTTGATGG 100
<210> 11195
<211> 100
<212> DNA
<213> Escherichia coli K-12 MG1655
<220> farr b0731
<222> 503-602
<223> farr b0731 U00096 complement(764376..765098)
<313> 11195: 544-563
<400> 11195
TGAAGAAAAT GGTTATCGAT CGTAGCGAAC AGGAAATCAT TCCTCTGATG 50
CCAACCGAAG AGATGAGCCG TCTGCTTAAT ATCAGCCAGA CAAAGCCGAT 100
<210> 11196
<211> 100
<212> DNA
<213> Escherichia coli K-12 MG1655
<220> farr b0731
<222> 417-516
<223> farr b0731 U00096 complement(764376..765098)
<313> 11196: 461-480
<400> 11196
GGAAGAAACC TGGATGCCGC TTGCGTTGTT CCCGGATCTC ACCTGGCAGG 50
TCATGGAAAA TTCGAAATAT CACTTTATCG AAGAAGTGAA GAAAATGGTT 100
<210> 11197
<211> 100
<212> DNA
<213> Escherichia coli K-12 MG1655
<220> lrp b0890
<222> 396-495
<223> lrp b0890 U00096 931818..932312
<313> 11197: 472-491
<400> 11197
GGAAACCCTG CTGCGTCTGC CTGGCGTCAA TGACACACGG ACATACGTTG 50
TTATGGAAGA AGTCAAGCAG AGTAATCGTC TGGTTATTAA GACGCGCTAA 100
<210> 11198
<211> 100
<212> DNA
<213> Escherichia coli K-12 MG1655
<220> lrp b0890
<222> 366-465
<223> lrp b0890 U00096 931818..932312
<313> 11198: 391-410
<400> 11198
GGATATGTCA GCCTACCGTA AGTTGCTGGG GGAAACCCTG CTGCGTCTGC 50
CTGGCGTCAA TGACACACGG ACATACGTTG TTATGGAAGA AGTCAAGCAG 100
<210> 11199
<211> 100
<212> DNA

<213> Escherichia coli K-12 MG1655
<220> lrp b0890
<222> 208-307
<223> lrp b0890 U00096 931818..932312
<313> 11199: 237-256
<400> 11199
CTGGATGCAT CACTTCTGGT ATTCGTTGAG ATTACTCTGA ATCGTGGCGC 50
ACCGGATGTG TTTGAACAAT TCAATACCGC TGTACAAAAA CTTGAAGAAA 100
<210> 11200
<211> 100
<212> DNA
<213> Escherichia coli K-12 MG1655
<220> elaa b2268
<222> 363-462
<223> elaa b2268 U00096 complement(2379102..2379563)
<313> 11200: 431-450
<400> 11200
AAACTTCTAC CAGAGTTTCG GTTTTATCCC TGTGACGGAG GTCTATGAAG 50
AAGATGGTAT CCCACACATT GGGATGGCAC GCGAGGTAAT TCAGGCGTAA 100
<210> 11201
<211> 100
<212> DNA
<213> Escherichia coli K-12 MG1655
<220> elaa b2268
<222> 359-458
<223> elaa b2268 U00096 complement(2379102..2379563)
<313> 11201: 382-401
<400> 11201
TGCAAAACTT CTACCAGAGT TTCGGTTTTA TCCCTGTGAC GGAGGTCTAT 50
GAAGAAGATG GTATCCCACA CATTGGGATG GCACGCGAGG TAATTCAGGC 100
<210> 11202
<211> 100
<212> DNA
<213> Escherichia coli K-12 MG1655
<220> elaa b2268
<222> 281-380
<223> elaa b2268 U00096 complement(2379102..2379563)
<313> 11202: 305-324
<400> 11202
AGCTGATGAG TAAAACACTC GAAACATGTA CGCATCACTG GCCTGATAAG 50
CCTGTTTACC TCGGGGCGCA GGCGCATCTG CAAAACTTCT ACCAGAGTTT 100
<210> 11203
<211> 100
<212> DNA
<213> Escherichia coli K-12 MG1655
<220> elab b2267
<222> 207-306
<223> elab b2267 U00096 complement(2378742..2379047)
<313> 11203: 240-259
<400> 11203
TTATCGTGCT GATGACTACG TCCACGAAAA ACCCTGGCAA GGAATTGGTG 50
TGGGCGCGGC CGTTGGGCTG GTACTAGGAC TGTTGCTGGC ACGCCGTTAA 100
<210> 11204
<211> 100
<212> DNA
<213> Escherichia coli K-12 MG1655
<220> elab b2267
<222> 163-262
<223> elab b2267 U00096 complement(2378742..2379047)
<313> 11204: 199-218
<400> 11204
AGCCAGGCTT CAGACAGTTA TTACTATCGG GCGAAGCAGG CAGTTTATCG 50
TGCTGATGAC TACGTCCACG AAAAACCCTG GCAAGGAATT GGTGTGGGCG 100
<210> 11205
<211> 100
<212> DNA
<213> Escherichia coli K-12 MG1655
<220> elab b2267
<222> 38-137
<223> elab b2267 U00096 complement(2378742..2379047)
<313> 11205: 99-118

<400> 11205
ACCTGACGCT GCTTAGTGAA ACACTGGAAG AGGTGCTCCG CTCCTCTGGC 50
GATCCCGCCG ATCAGAAATA TGTTGAGCTG AAAGCGCGTG CAGAAAAAGC 100
<210> 11206
<211> 100
<212> DNA
<213> Escherichia coli K-12 MG1655
<220> elac b2269
<222> 837-936
<223> elac b2269 U00096 2379610..2380545
<313> 11206: 910-929
<400> 11206
GCGCTATGAT GACAAAGGTT GTCAGCACCT GTTACGTGAA TGCAGGTCAA 50
TTTTCCCGGC GACTGAACTG GCGAATGATT TCACCGTGTT TAACGTTTAA 100
<210> 11207
<211> 100
<212> DNA
<213> Escherichia coli K-12 MG1655
<220> elac b2269
<222> 796-895
<223> elac b2269 U00096 2379610..2380545
<313> 11207: 845-864
<400> 11207
GAGGCTGGAG TCGGCAAGCT AATCATTACC CACGTCAGCT CGCGCTATGA 50
TGACAAAGGT TGTCAGCACC TGTTACGTGA ATGCAGGTCA ATTTTCCCGG 100
<210> 11208
<211> 100
<212> DNA
<213> Escherichia coli K-12 MG1655
<220> elac b2269
<222> 771-870
<223> elac b2269 U00096 2379610..2380545
<313> 11208: 805-824
<400> 11208
ACGCCAGGCT GCGACACTAG CCCGTGAGGC TGGAGTCGGC AAGCTAATCA 50
TTACCCACGT CAGCTCGCGC TATGATGACA AAGGTTGTCA GCACCTGTTA 100
<210> 11209
<211> 100
<212> DNA
<213> Escherichia coli K-12 MG1655
<220> elad b2270
<222> 1113-1212
<223> elad b2270 U00096 2380733..2381944
<313> 11209: 1176-1195
<400> 11209
AGATAAATAT GGCGATGCAA ATATCAATCA TTATTATAGA AACTTAGAAA 50
TAAAACACTC ACAACCCAAA AATAGAGCAT CCGGCAAAAG AGTGAGTTAA 100
<210> 11210
<211> 100
<212> DNA
<213> Escherichia coli K-12 MG1655
<220> elad b2270
<222> 980-1079
<223> elad b2270 U00096 2380733..2381944
<313> 11210: 1001-1020
<400> 11210
AAAGGGAAAG CAATTCTGAC AGCGCCCCCT ATACGTTATT AAAAAACCAT 50
GCTGACAGAT TTAAAAAATA TTCAGCAGAA GAGCAGTACG AAATTGATTT 100
<210> 11211
<211> 100
<212> DNA
<213> Escherichia coli K-12 MG1655
<220> elad b2270
<222> 913-1012
<223> elad b2270 U00096 2380733..2381944
<313> 11211: 961-980
<400> 11211
CTTCAGCAAT ACTTAAGCCA AAGTTGCGGT GCATTTGTGT GCATGGCAGC 50
CCAGGAAGTG ATTGAACAAA GGGAAAGCAA TTCTGACAGC GCCCCCTATA 100
<210> 11212
<211> 100

<212> DNA
<213> Escherichia coli K-12 MG1655
<220> ynac ynaC
<222> 178-277
<223> ynac ynaC U00096 1430435..1431010
<313> 11212: 231-250
<400> 11212
AGTGATGAGT CATCGTGGCA TCTCGTTGAA GATCATCGGG GTAAAACGGT 50
TTATGACGTG GCTTCCGGCA ACGCGTTATT TATTTCTGAA CTCGGTCCGT 100
<210> 11213
<211> 100
<212> DNA
<213> Escherichia coli K-12 MG1655
<220> ynae b1376
<222> 168-267
<223> ynae b1376 U00096 complement(1432015..1432281)
<313> 11213: 193-212
<400> 11213
AGGAAACATA AAGAAAACAT CATGGCTGGA TAACAAAGAA TTATTACAAA 50
CAGCGATATC AGTTCTTAAA GACAACCAAA ATTTAAATGG TAAAATGTAA 100
<210> 11214
<211> 100
<212> DNA
<213> Escherichia coli K-12 MG1655
<220> ynae b1376
<222> 147-246
<223> ynae b1376 U00096 complement(1432015..1432281)
<313> 11214: 182-201
<400> 11214
AACATTGCTT GATTACATTG AAGGAAACAT AAAGAAAACA TCATGGCTGG 50
ATAACAAAGA ATTATTACAA ACAGCGATAT CAGTTCTTAA AGACAACCAA 100
<210> 11215
<211> 100
<212> DNA
<213> Escherichia coli K-12 MG1655
<220> ynae b1376
<222> 145-244
<223> ynae b1376 U00096 complement(1432015..1432281)
<313> 11215: 168-187
<400> 11215
AGAACATTGC TTGATTACAT TGAAGGAAAC ATAAAGAAAA CATCATGGCT 50
GGATAACAAA GAATTATTAC AAACAGCGAT ATCAGTTCTT AAAGACAACC 100
<210> 11216
<211> 100
<212> DNA
<213> Escherichia coli K-12 MG1655
<220> ynaf ynaF
<222> 408-507
<223> ynaf ynaF U00096 complement(1433209..1433715)
<313> 11216: 441-460
<400> 11216
CATTGCTTCC CATCGACCGG ATATCACCAC TTATCTGCTC GGTTCCAACG 50
CCGCAGCTGT AGTGCGTCAC GCAGAGTGCT CCGTGCTGGT TGTGCGCTGA 100
<210> 11217
<211> 100
<212> DNA
<213> Escherichia coli K-12 MG1655
<220> ynaf ynaF
<222> 318-417
<223> ynaf ynaF U00096 complement(1433209..1433715)
<313> 11217: 351-370
<400> 11217
AACCGACAGA GTGCATGTCC ATGTTGAGGA AGGCTCGCCC AAAGACCGCA 50
TTCTGGAATT GGCGAAGAAG ATCCCCGCTC ATATGATCAT CATTGCTTCC 100
<210> 11218
<211> 100
<212> DNA
<213> Escherichia coli K-12 MG1655
<220> ynaf ynaF
<222> 229-328
<223> ynaf ynaF U00096 complement(1433209..1433715)

<313> 11218: 277-296
<400> 11218
GCGTATTCCG CAGAATTACC GGCAATGGAT GACCTGAAAG CGGAAGCCAA 50
ATCGCAACTG GAAGAGATCA TTAAAAAATT TAAACTGCCA ACCGACAGAG 100
<210> 11219
<211> 100
<212> DNA
<213> Escherichia coli K-12 MG1655
<220> ynaj b1333
<222> 152-251
<223> ynaj b1333 U00096 1395389..1395646
<313> 11219: 179-198
<400> 11219
CAATGTATGT GATTCAGTCA TCGATGATTT TTGTTTATAG CCTGGTCTTT 50
ACTGTGTTGC TGGCAATCCC GTTGGGAATT TATTTCCTTG GCGGCGAAGA 100
<210> 11220
<211> 100
<212> DNA
<213> Escherichia coli K-12 MG1655
<220> ynaj b1333
<222> 103-202
<223> ynaj b1333 U00096 1395389..1395646
<313> 11220: 137-156
<400> 11220
GGCGGCGTTA TAGAACAGTA CAATATTCCG CTGTCTGAGT GGACGACATC 50
AATGTATGTG ATTCAGTCAT CGATGATTTT TGTTTATAGC CTGGTCTTTA 100
<210> 11221
<211> 100
<212> DNA
<213> Escherichia coli K-12 MG1655
<220> ynaj b1333
<222> 110-209
<223> ynaj b1333 U00096 1395389..1395646
<313> 11221: 143-162
<400> 11221
TTATAGAACA GTACAATATT CCGCTGTCTG AGTGGACGAC ATCAATGTAT 50
GTGATTCAGT CATCGATGAT TTTTGTTTAT AGCCTGGTCT TTACTGTGTT 100
<210> 11222
<211> 100
<212> DNA
<213> Escherichia coli K-12 MG1655
<220> otsa b1897
<222> 1326-1425
<223> otsa b1897 U00096 complement(1978212..1979636)
<313> 11222: 1384-1403
<400> 11222
CTGGCAGGAG TGCTTCATTA GCGACCTAAA GCAGATAGTT CCGCGAAGCG 50
CGGAAAGCCA GCAGCGCGAT AAAGTTGCTA CCTTTCCAAA GCTTGCGTAG 100
<210> 11223
<211> 100
<212> DNA
<213> Escherichia coli K-12 MG1655
<220> otsa b1897
<222> 1302-1401
<223> otsa b1897 U00096 complement(1978212..1979636)
<313> 11223: 1351-1370
<400> 11223
TATCGTGAAA AACGATATTA ACCACTGGCA GGAGTGCTTC ATTAGCGACC 50
TAAAGCAGAT AGTTCCGCGA AGCGCGGAAA GCCAGCAGCG CGATAAAGTT 100
<210> 11224
<211> 100
<212> DNA
<213> Escherichia coli K-12 MG1655
<220> otsa b1897
<222> 1230-1329
<223> otsa b1897 U00096 complement(1978212..1979636)
<313> 11224: 1281-1300
<400> 11224
AGCTGCGCTG GATCGTGCAT TGACTATGTC GCTGGCGGAA CGTATTTCCC 50
GTCATGCAGA AATGCTGGAC GTTATCGTGA AAAACGATAT TAACCACTGG 100
<210> 11225

<211> 100
<212> DNA
<213> Escherichia coli K-12 MG1655
<220> otsb b1898
<222> 620-719
<223> otsb b1898 U00096 complement(1979611..1980411)
<313> 11225: 658-677
<400> 11225
CAGTCGTTAA CCGACTGGGC GGAATGTCAG TAAAAATTGG CACAGGTGCA 50
ACTCAGGCAT CATGGCGACT GGCGGGTGTG CCGGATGTCT GGAGCTGGCT 100
<210> 11226
<211> 100
<212> DNA
<213> Escherichia coli K-12 MG1655
<220> otsb b1898
<222> 548-647
<223> otsb b1898 U00096 complement(1979611..1980411)
<313> 11226: 607-626
<400> 11226
TGCAGGAAGC TCCCTTTATC GGGCGAACGC CCGTATTTCT GGGCGATGAT 50
TTAACCGATG AATCTGGCTT CGCAGTCGTT AACCGACTGG GCGGAATGTC 100
<210> 11227
<211> 100
<212> DNA
<213> Escherichia coli K-12 MG1655
<220> otsb b1898
<222> 526-625
<223> otsb b1898 U00096 complement(1979611..1980411)
<313> 11227: 554-573
<400> 11227
GGTGAGGCAA TTGCAGCTTT TATGCAGGAA GCTCCCTTTA TCGGGCGAAC 50
GCCCGTATTT CTGGGCGATG ATTTAACCGA TGAATCTGGC TTCGCAGTCG 100
<210> 11228
<211> 100
<212> DNA
<213> Escherichia coli K-12 MG1655
<220> dgoa b3693
<222> 1646-1745
<223> dgoa b3693 U00096 complement(3869477..3871240)
<313> 11228: 1701-1720
<400> 11228
CGAAACCGGG CTTAGGCGTG GAAATCGACG AAGCTAAAGT GATTGAGTTC 50
AGTAAAAATG CCCCGGACTG GCGTAATCCG CTCTGGCGTC ATGAAGATAA 100
<210> 11229
<211> 100
<212> DNA
<213> Escherichia coli K-12 MG1655
<220> dgoa b3693
<222> 1582-1681
<223> dgoa b3693 U00096 complement(3869477..3871240)
<313> 11229: 1624-1643
<400> 11229
TTACTCGACT TTGTGAAAAA CAAAGAAGAC TTCAGCATGG TCGGCGGCTT 50
CTTTAAACCG TTAACGAAAC CGGGCTTAGG CGTGGAAATC GACGAAGCTA 100
<210> 11230
<211> 100
<212> DNA
<213> Escherichia coli K-12 MG1655
<220> dgoa b3693
<222> 1518-1617
<223> dgoa b3693 U00096 complement(3869477..3871240)
<313> 11230: 1569-1588
<400> 11230
TGTTTCCTAT AACGCCGTAC TTCAGGAACA AAGTATGGGA ATTCATTACA 50
ACAAAGGCGC GGAGTTACTC GACTTTGTGA AAAACAAAGA AGACTTCAGC 100
<210> 11231
<211> 100
<212> DNA
<213> Escherichia coli K-12 MG1655
<220> tdh b3617
<222> 885-984

<223> tdh b3617 U00096 complement(3787947..3788972)
<313> 11231: 917-936
<400> 11231
GTACAAGATG GCGGCGCTGA TTCAGTCTGG CCTCGATCTT TCGCCGATCA 50
TTACCCATCG TTTCTCTATC GATGATTTCC AGAAGGGCTT TGACGCTATG 100
<210> 11232
<211> 100
<212> DNA
<213> Escherichia coli K-12 MG1655
<220> tdh b3617
<222> 828-927
<223> tdh b3617 U00096 complement(3787947..3788972)
<313> 11232: 866-885
<400> 11232
GATCTTTAAA GGCTTGTTCA TTAAAGGTAT TTACGGTCGT GAGATGTTTG 50
AAACCTGGTA CAAGATGGCG GCGCTGATTC AGTCTGGCCT CGATCTTTCG 100
<210> 11233
<211> 100
<212> DNA
<213> Escherichia coli K-12 MG1655
<220> tdh b3617
<222> 720-819
<223> tdh b3617 U00096 complement(3787947..3788972)
<313> 11233: 733-752
<400> 11233
CGGTGCGCCG CCAGCGTTTC GTACCATGCT TGACACCATG AATCACGGCG 50
GCCGTATTGC GATGCTGGGT ATTCCGCCGT CTGATATGTC TATCGACTGG 100
<210> 11234
<211> 100
<212> DNA
<213> Escherichia coli K-12 MG1655
<220> sana sanA
<222> 621-720
<223> sana sanA U00096 2230898..2231617
<313> 11234: 670-689
<400> 11234
CCCTATTCCG GCTATGCACC AGGTACCGGA AGATGCGCAG GGGTATCCCG 50
CCGTCACACC CGAACAGTTA CTTGAATTAC AAAAGAAACA AGGAAAGTAG 100
<210> 11235
<211> 100
<212> DNA
<213> Escherichia coli K-12 MG1655
<220> sana sanA
<222> 578-677
<223> sana sanA U00096 2230898..2231617
<313> 11235: 615-634
<400> 11235
TTTATATTTT TAAACGTGAA CCGCGTTTTT TAGGGCCGCT GGTCCCTATT 50
CCGGCTATGC ACCAGGTACC GGAAGATGCG CAGGGGTATC CCGCCGTCAC 100
<210> 11236
<211> 100
<212> DNA
<213> Escherichia coli K-12 MG1655
<220> sana sanA
<222> 452-551
<223> sana sanA U00096 2230898..2231617
<313> 11236: 510-529
<400> 11236
AGCGAGCATT ATTTATTGCG CTGCATATGG GGATTCAGGC TCAGTGTTAT 50
GCCGTACCGT CACCGAAAGA TATGCTGTCA GTACGTATTC GTGAATTTGC 100
<210> 11237
<211> 100
<212> DNA
<213> Escherichia coli K-12 MG1655
<220> tdk b1239
<222> 519-618
<223> tdk b1239 U00096 1292750..1293367
<313> 11237: 586-605
<400> 11237
TGGTAATGAA CGATACGTTT CTGTATGCCG TAAACACTAT AAAGAGGCGT 50
TACAAGTCGA CTCATTAACG GCTATTCAGG AAAGGCATCG CCACGATTAA 100

<210> 11238
<211> 100
<212> DNA
<213> Escherichia coli K-12 MG1655
<220> tdk b1239
<222> 454-553
<223> tdk b1239 U00096 1292750..1293367
<313> 11238: 508-527
<400> 11238
AGCATGGTGC TGCGTCTTGA TCAAGCAGGC AGACCTTATA ACGAAGGTGA 50
GCAGGTGGTA ATTGGTGGTA ATGAACGATA CGTTTCTGTA TGCCGTAAAC 100
<210> 11239
<211> 100
<212> DNA
<213> Escherichia coli K-12 MG1655
<220> tdk b1239
<222> 288-387
<223> tdk b1239 U00096 1292750..1293367
<313> 11239: 304-323
<400> 11239
AGTATATGAA TTATCGGAGG TTGTCGATCA ACTCGATATA CCCGTACTTT 50
GTTATGGTTT ACGTACCGAT TTTCGAGGTG AATTATTTAT TGGCAGCCAA 100
<210> 11240
<211> 100
<212> DNA
<213> Escherichia coli K-12 MG1655
<220> dgok dgoK
<222> 780-879
<223> dgok dgoK U00096 complement(3871224..3872102)
<313> 11240: 845-864
<400> 11240
GCAAGCCTTT CAGGCGATGG GTTGCGACGT GACGGCGGTG GCGGGCGACA 50
CGGCATTTCA GGCTGGTATA AGGAGCATCG CTCATGCAGT GGCAAACTAA 100
<210> 11241
<211> 100
<212> DNA
<213> Escherichia coli K-12 MG1655
<220> dgok dgoK
<222> 697-796
<223> dgok dgoK U00096 complement(3871224..3872102)
<313> 11241: 736-755
<400> 11241
GAGGTCGCCA GTATGCGCGA CTATGTGGCC CATCAACACG CCATCACCCT 50
TGTCGCCGGA ACATCGCTGA CCGCGCGCTA CCAGCAAGCC TTTCAGGCGA 100
<210> 11242
<211> 100
<212> DNA
<213> Escherichia coli K-12 MG1655
<220> dgok dgoK
<222> 609-708
<223> dgok dgoK U00096 complement(3871224..3872102)
<313> 11242: 655-674
<400> 11242
GCTTTTTGAA GTTCGCGCCT CGCATGTGCT GGGAACACTT CCCCGCGAAC 50
AGGTCAGCGA ATTTCTCTCT GGTTTGTTGA TTGGCGCAGA GGTCGCCAGT 100
<210> 11243
<211> 100
<212> DNA
<213> Escherichia coli K-12 MG1655
<220> spot b3651
<222> 2010-2109
<223> spot b3651 U00096 3820027..3822135
<313> 11243: 2072-2091
<400> 11243
TATTCGTCTG ACCGCTCGTG ACCGTGTGCA TCTGGCGAAT ATCATGCGCA 50
AAATCCGCGT GATGCCAGAC GTGATTAAAG TCACCCGAAA CCGAAATTAA 100
<210> 11244
<211> 100
<212> DNA
<213> Escherichia coli K-12 MG1655
<220> spot b3651

<222> 1990-2089
<223> spot b3651 U00096 3820027..3822135
<313> 11244: 2019-2038
<400> 11244
GGTCGCGTCT ACAGCGCCTT TATTCGTCTG ACCGCTCGTG ACCGTGTGCA 50
TCTGGCGAAT ATCATGCGCA AAATCCGCGT GATGCCAGAC GTGATTAAAG 100
<210> 11245
<211> 100
<212> DNA
<213> Escherichia coli K-12 MG1655
<220> spot b3651
<222> 1931-2030
<223> spot b3651 U00096 3820027..3822135
<313> 11245: 1986-2005
<400> 11245
CGGCGGCAAT TAACACCACG ACTTCGAATA TTCAAAGTTT GAATACGGAA 50
GAGAAAGATG GTCGCGTCTA CAGCGCCTTT ATTCGTCTGA CCGCTCGTGA 100
<210> 11246
<211> 100
<212> DNA
<213> Escherichia coli K-12 MG1655
<220> spou b3652
<222> 591-690
<223> spou b3652 U00096 3822142..3822831
<313> 11246: 649-668
<400> 11246
AGTCGCAAAA CGCAAAGGCC TGCCTTATCC CCACGTCAAT CAGCAAGGCG 50
AGATCGAAGC TGATGCCGAC TGGTGGGCTA CTATGCAGGC TGCAGGGTAA 100
<210> 11247
<211> 100
<212> DNA
<213> Escherichia coli K-12 MG1655
<220> spou b3652
<222> 459-558
<223> spou b3652 U00096 3822142..3822831
<313> 11247: 495-514
<400> 11247
CTCAGCCCTC ATTCTTTACG AAGCCCAGCG TCAGCGGCAA AATGCAGGCA 50
TGTACCTGCG TGAAAACAGC ATGTTGCCGG AAGCAGAGCA ACAACGCCTG 100
<210> 11248
<211> 100
<212> DNA
<213> Escherichia coli K-12 MG1655
<220> spou b3652
<222> 421-520
<223> spou b3652 U00096 3822142..3822831
<313> 11248: 447-466
<400> 11248
ATGATCGGCA TGGTGCAGTC GCTGAATGTT TCCGTTGCCT CAGCCCTCAT 50
TCTTTACGAA GCCCAGCGTC AGCGGCAAAA TGCAGGCATG TACCTGCGTG 100
<210> 11249
<211> 100
<212> DNA
<213> Escherichia coli K-12 MG1655
<220> ynbd b1412
<222> 1194-1293
<223> ynbd b1412 U00096 1478933..1480225
<313> 11249: 1271-1290
<400> 11249
AACCGTTAAT GAAGCGATTA GCTATATTCG AGCCAGACGC CCGCAGATTG 50
TGCTGACAGA CGAGCACAAA GCGATGCTGA GATTATGGGA AAACAGGTAA 100
<210> 11250
<211> 100
<212> DNA
<213> Escherichia coli K-12 MG1655
<220> ynbd b1412
<222> 1194-1293
<223> ynbd b1412 U00096 1478933..1480225
<313> 11250: 1230-1249
<400> 11250
AACCGTTAAT GAAGCGATTA GCTATATTCG AGCCAGACGC CCGCAGATTG 50

TGCTGACAGA CGAGCACAAA GCGATGCTGA GATTATGGGA AAACAGGTAA 100
<210> 11251
<211> 100
<212> DNA
<213> Escherichia coli K-12 MG1655
<220> ynbd b1412
<222> 1148-1247
<223> ynbd b1412 U00096 1478933..1480225
<313> 11251: 1181-1200
<400> 11251
GTGCGCTGGT GGTGGCGGCA TGGTTGTTAT GTTACGGACA CTGTAAAACC 50
GTTAATGAAG CGATTAGCTA TATTCGAGCC AGACGCCCGC AGATTGTGCT 100
<210> 11252
<211> 100
<212> DNA
<213> Escherichia coli K-12 MG1655
<220> ynbe b1383
<222> 87-186
<223> ynbe b1383 U00096 1443711..1443896
<313> 11252: 158-177
<400> 11252
GATCACTATC AATATGAACG TTAAAATTGA GCATGAGATC ATCATCAAGG 50
CAGACAAAGA TGTCGAAGAG CTGCTTGAAA CTCGTAGCGA TCTTTTCTGA 100
<210> 11253
<211> 100
<212> DNA
<213> Escherichia coli K-12 MG1655
<220> ynbe b1383
<222> 87-186
<223> ynbe b1383 U00096 1443711..1443896
<313> 11253: 134-153
<400> 11253
GATCACTATC AATATGAACG TTAAAATTGA GCATGAGATC ATCATCAAGG 50
CAGACAAAGA TGTCGAAGAG CTGCTTGAAA CTCGTAGCGA TCTTTTCTGA 100
<210> 11254
<211> 100
<212> DNA
<213> Escherichia coli K-12 MG1655
<220> ynbe b1383
<222> 81-180
<223> ynbe b1383 U00096 1443711..1443896
<313> 11254: 140-159
<400> 11254
GGAACCGATC ACTATCAATA TGAACGTTAA AATTGAGCAT GAGATCATCA 50
TCAAGGCAGA CAAAGATGTC GAAGAGCTGC TTGAAACTCG TAGCGATCTT 100
<210> 11255
<211> 100
<212> DNA
<213> Escherichia coli K-12 MG1655
<220> dgot b3692
<222> 1219-1318
<223> dgot b3692 U00096 complement(3868065..3869402)
<313> 11255: 1254-1273
<400> 11255
GTGGGGTACC TGGCGCAGGG TTACGGTTTC GCACCTGCAC TGGTTTATAT 50
CTCCGCCGTC GCGTTGATTG GCGCGCTCTC TTATATCCTG CTGGTGGGCG 100
<210> 11256
<211> 100
<212> DNA
<213> Escherichia coli K-12 MG1655
<220> dgot b3692
<222> 1110-1209
<223> dgot b3692 U00096 complement(3868065..3869402)
<313> 11256: 1136-1155
<400> 11256
GATTACCTGG TCGCTGGTTT CTTCTCTGGC ACCGATGCGC CTGATTGGTT 50
TAACCGGCGG CGTGTTTAAC TTCGCCGGTG GTCTGGGCGG CATCACCGTT 100
<210> 11257
<211> 100
<212> DNA
<213> Escherichia coli K-12 MG1655

<220> dgot b3692
<222> 1064-1163
<223> dgot b3692 U00096 complement(3868065..3869402)
<313> 11257: 1089-1108
<400> 11257
TTATGTGCCT GATGGCGCTG GCATTCTTCG GTAACGGTTT TGCTTCGATT 50
ACCTGGTCGC TGGTTTCTTC TCTGGCACCG ATGCGCCTGA TTGGTTTAAC 100
<210> 11258
<211> 100
<212> DNA
<213> Escherichia coli K-12 MG1655
<220> ynbf b1390
<222> 189-288
<223> ynbf b1390 U00096 1452892..1453179
<313> 11258: 253-272
<400> 11258
GCAACCGGAA GAACGCGGTG AATTTTTTGA TCCGGCTGAA AGCAAGGTCT 50
ATCGCCATCC AACGTTTTAC ACCATCCCTG ATGGCATTGA GCACATGTGA 100
<210> 11259
<211> 100
<212> DNA
<213> Escherichia coli K-12 MG1655
<220> ynbf b1390
<222> 161-260
<223> ynbf b1390 U00096 1452892..1453179
<313> 11259: 221-240
<400> 11259
TGGTGAAGGC GAGTGAAATT GTTGCCTCGC AACCGGAAGA ACGCGGTGAA 50
TTTTTTGATC CGGCTGAAAG CAAGGTCTAT CGCCATCCAA CGTTTTACAC 100
<210> 11260
<211> 100
<212> DNA
<213> Escherichia coli K-12 MG1655
<220> ynbf b1390
<222> 138-237
<223> ynbf b1390 U00096 1452892..1453179
<313> 11260: 169-188
<400> 11260
TAGCGAAGGA TGTTCAATTT GGGTGGTGAA GGCGAGTGAA ATTGTTGCCT 50
CGCAACCGGA AGAACGCGGT GAATTTTTTG ATCCGGCTGA AAGCAAGGTC 100
<210> 11261
<211> 100
<212> DNA
<213> Escherichia coli K-12 MG1655
<220> sppa b1767
<222> 1758-1857
<223> sppa b1767 U00096 1846861..1848717
<313> 11261: 1823-1842
<400> 11261
GGTAGCCTCT ACCGTTAAAA GTGAAAGTGA CAAGCTGGCC GCGTTTAATG 50
ACCCACAAAA CCGTTATGCG TTTTGCCTGA CCTGCGCCAA CATGCGTTAA 100
<210> 11262
<211> 100
<212> DNA
<213> Escherichia coli K-12 MG1655
<220> sppa b1767
<222> 1749-1848
<223> sppa b1767 U00096 1846861..1848717
<313> 11262: 1789-1808
<400> 11262
GCTGGCCTCG GTAGCCTCTA CCGTTAAAAG TGAAAGTGAC AAGCTGGCCG 50
CGTTTAATGA CCCACAAAAC CGTTATGCGT TTTGCCTGAC CTGCGCCAAC 100
<210> 11263
<211> 100
<212> DNA
<213> Escherichia coli K-12 MG1655
<220> sppa b1767
<222> 1672-1771
<223> sppa b1767 U00096 1846861..1848717
<313> 11263: 1722-1741
<400> 11263

GACAAAGTGA TGGACAACAT GTCTGGTTCT GTCCGGGCAA TGTTGCCAGA 50
TGCGTTCCAG GCCATGTTAC CTGCACCGCT GGCCTCGGTA GCCTCTACCG 100
<210> 11264
<211> 100
<212> DNA
<213> Escherichia coli K-12 MG1655
<220> eco b2210
<222> 390-489
<223> eco b2210 U00096 2301925..2302413
<313> 11264: 457-476
<400> 11264
CAGCAAGCTG CCGATCGTGG TGTATACGCC AGACAATGTA GATGTGAAGT 50
ACCGCGTCTG GAAGGCGGAA GAGAAAATTG ACAACGCGGT AGTTCGCTAA 100
<210> 11265
<211> 100
<212> DNA
<213> Escherichia coli K-12 MG1655
<220> eco b2210
<222> 338-437
<223> eco b2210 U00096 2301925..2302413
<313> 11265: 369-388
<400> 11265
AGAAGAAATT TGTCACCGCG TATCTGGGCG ATGCTGGAAT GCTGCGTTAC 50
AACAGCAAGC TGCCGATCGT GGTGTATACG CCAGACAATG TAGATGTGAA 100
<210> 11266
<211> 100
<212> DNA
<213> Escherichia coli K-12 MG1655
<220> eco b2210
<222> 275-374
<223> eco b2210 U00096 2301925..2302413
<313> 11266: 339-358
<400> 11266
ATGTCTTTGA TAAAGTCAGT TCCCCGGTTT CAACGATGAT GGCCTGCCCG 50
GATGGCAAGA AAGAGAAGAA ATTTGTCACC GCGTATCTGG GCGATGCTGG 100
<210> 11267
<211> 100
<212> DNA
<213> Escherichia coli K-12 MG1655
<220> cynr b0339
<222> 801-900
<223> cynr b0339 U00096 complement(357015..357914)
<313> 11267: 875-894
<400> 11267
GTTGCGGCGG AAAAATAGCT GGCAGACAGC CGCCGCGAAG GCATTTTTGC 50
ACATGGCGTT GGATAAATGC GCGGTTGTTG GCGGAAATGA ATCACGGTAG 100
<210> 11268
<211> 100
<212> DNA
<213> Escherichia coli K-12 MG1655
<220> cynr b0339
<222> 783-882
<223> cynr b0339 U00096 complement(357015..357914)
<313> 11268: 833-852
<400> 11268
GGAGAGAACG GCGGTTTTGT TGCGGCGGAA AAATAGCTGG CAGACAGCCG 50
CCGCGAAGGC ATTTTTGCAC ATGGCGTTGG ATAAATGCGC GGTTGTTGGC 100
<210> 11269
<211> 100
<212> DNA
<213> Escherichia coli K-12 MG1655
<220> cynr b0339
<222> 708-807
<223> cynr b0339 U00096 complement(357015..357914)
<313> 11269: 760-779
<400> 11269
TTCCACATTG TTACCAGCAG CGATTGCCAC ACAACATGAC GGGCTTAAAG 50
CTATTTCTCT TGCCCCGCCA CTACTGGAGA GAACGGCGGT TTTGTTGCGG 100
<210> 11270
<211> 100
<212> DNA

<213> Escherichia coli K-12 MG1655
<220> cyns b0341
<222> 372-471
<223> cyns b0341 U00096 358713..359183
<313> 11270: 438-457
<400> 11270
TAACTTCAAA CTCGACGTTA AGAAAGTGGC GGACCCGGAA GGTGGCGAAC 50
GTGCGGTCAT CACCTTAGAT GGTAAATATC TGCCGACCAA ACCGTTCTGA 100
<210> 11271
<211> 100
<212> DNA
<213> Escherichia coli K-12 MG1655
<220> cyns b0341
<222> 343-442
<223> cyns b0341 U00096 358713..359183
<313> 11271: 390-409
<400> 11271
AAATTTGGCG ATGGCATTAT TAGCGCGATT AACTTCAAAC TCGACGTTAA 50
GAAAGTGGCG GACCCGGAAG GTGGCGAACG TGCGGTCATC ACCTTAGATG 100
<210> 11272
<211> 100
<212> DNA
<213> Escherichia coli K-12 MG1655
<220> cyns b0341
<222> 277-376
<223> cyns b0341 U00096 358713..359183
<313> 11272: 310-329
<400> 11272
ACGATGTATC GTTTCTATGA AATGTTGCAG GTGTACGGTA CAACCCTGAA 50
AGCGTTGGTT CATGAGAAAT TTGGCGATGG CATTATTAGC GCGATTAACT 100
<210> 11273
<211> 100
<212> DNA
<213> Escherichia coli K-12 MG1655
<220> cynt b0340
<222> 561-660
<223> cynt b0340 U00096 358023..358682
<313> 11273: 623-642
<400> 11273
CAGCATCGCA GCTTTTGACG GCGCAACCCG CCAGTTTGTG CCACTGGCCG 50
CTAATCCTCG CGTTTGTGCC ATACCGCTAC GCCAACCGAC CGCAGCGTAA 100
<210> 11274
<211> 100
<212> DNA
<213> Escherichia coli K-12 MG1655
<220> cynt b0340
<222> 528-627
<223> cynt b0340 U00096 358023..358682
<313> 11274: 572-591
<400> 11274
CCTGCACGGC TGGGTCTACG ACATTGAAAG CGGCAGCATC GCAGCTTTTG 50
ACGGCGCAAC CCGCCAGTTT GTGCCACTGG CCGCTAATCC TCGCGTTTGT 100
<210> 11275
<211> 100
<212> DNA
<213> Escherichia coli K-12 MG1655
<220> cynt b0340
<222> 481-580
<223> cynt b0340 U00096 358023..358682
<313> 11275: 539-558
<400> 11275
CAAACTCATC CATCGGTGCG CCTGGCGCTC GAAGAGGGGC GGATCGCCCT 50
GCACGGCTGG GTCTACGACA TTGAAAGCGG CAGCATCGCA GCTTTTGACG 100
<210> 11276
<211> 100
<212> DNA
<213> Escherichia coli K-12 MG1655
<220> cynx b0342
<222> 1051-1150
<223> cynx b0342 U00096 359216..360370
<313> 11276: 1107-1126

<400> 11276
CTGATGGACT GGGCATTTCA TGCGCTGTGC GTCGTTGGGC TGATGATCAT 50
AACCCTGCGT TTTGCACCAG TACGTTTTCC GCAGCTGTGG GTCAAAGAGG 100
<210> 11277
<211> 100
<212> DNA
<213> Escherichia coli K-12 MG1655
<220> cynx b0342
<222> 1019-1118
<223> cynx b0342 U00096 359216..360370
<313> 11277: 1063-1082
<400> 11277
CTGGCGTGCT GCGTAGTATC AGCGGCAATT ACCTGATGGA CTGGGCATTT 50
CATGCGCTGT GCGTCGTTGG GCTGATGATC ATAACCCTGC GTTTTGCACC 100
<210> 11278
<211> 100
<212> DNA
<213> Escherichia coli K-12 MG1655
<220> cynx b0342
<222> 711-810
<223> cynx b0342 U00096 359216..360370
<313> 11278: 768-787
<400> 11278
GGCATTGATG ACGCTTGGGC AAGCCGCAGG AGCTTTGCTG ATGCCTGCTA 50
TGGCTCGCCA TCAGGATCGG CGCAAACTGT TAATGCTGGC GCTGGTGTTA 100
<210> 11279
<211> 100
<212> DNA
<213> Escherichia coli K-12 MG1655
<220> eda b1851
<222> 543-642
<223> eda b1851 U00096 complement(1930139..1930780)
<313> 11279: 594-613
<400> 11279
CGGTGGTTCC TGGCTGGTTC CGGCAGATGC GCTGGAAGCG GGCGATTACG 50
ACCGCATTAC TAAGCTGGCG CGTGAAGCTG TAGAAGGCGC TAAGCTGTAA 100
<210> 11280
<211> 100
<212> DNA
<213> Escherichia coli K-12 MG1655
<220> eda b1851
<222> 448-547
<223> eda b1851 U00096 complement(1930139..1930780)
<313> 11280: 478-497
<400> 11280
GCGGGTCCGT TCTCCCAGGT CCGTTTCTGC CCGACGGGTG GTATTTCTCC 50
GGCTAACTAC CGTGACTACC TGGCGCTGAA AAGCGTGCTG TGCATCGGTG 100
<210> 11281
<211> 100
<212> DNA
<213> Escherichia coli K-12 MG1655
<220> eda b1851
<222> 354-453
<223> eda b1851 U00096 complement(1930139..1930780)
<313> 11281: 408-427
<400> 11281
TTCCGAACTG ATGCTGGGTA TGGACTACGG TTTGAAAGAG TTCAAATTCT 50
TCCCGGCTGA AGCTAACGGC GGCGTGAAAG CCCTGCAGGC GATCGCGGGT 100
<210> 11282
<211> 100
<212> DNA
<213> Escherichia coli K-12 MG1655
<220> yncb yncB
<222> 1005-1104
<223> yncb yncB U00096 1516958..1518088
<313> 11282: 1038-1057
<400> 11282
AGAGGATAAA ATCCACTACC GCGAAGAAAT TACTGACGGT TTAGAGAATG 50
CGCCACAGAC GTTTATCGGC CTGCTGAAGG GTAAAAACTT CGGCAAAGTG 100
<210> 11283
<211> 100

<212> DNA
<213> Escherichia coli K-12 MG1655
<220> yncb yncB
<222> 972-1071
<223> yncb yncB U00096 1516958..1518088
<313> 11283: 1001-1020
<400> 11283
TGAGTTTCAG AGGGAGATGG GGCAATGGGT GAAAGAGGAT AAAATCCACT 50
ACCGCGAAGA AATTACTGAC GGTTTAGAGA ATGCGCCACA GACGTTTATC 100
<210> 11284
<211> 100
<212> DNA
<213> Escherichia coli K-12 MG1655
<220> yncb yncB
<222> 802-901
<223> yncb yncB U00096 1516958..1518088
<313> 11284: 849-868
<400> 11284
TTACTTAATA CATCTGCGCG CATTCCCGTC TGCGGATTAG TGAGCAGCTA 50
TAACGCTACA GAGCTACCAC CCGGTCCGGA TCGTTTACCT CTGTTGATGG 100
<210> 11285
<211> 100
<212> DNA
<213> Escherichia coli K-12 MG1655
<220> cyoa b0433
<222> 849-948
<223> cyoa b0433 U00096 complement(449887..450834)
<313> 11285: 918-937
<400> 11285
TCACGGTAAG AGCATGGACA TGACCCAGCC AGAAGGTGAG CACAGCGCAC 50
ACGAAGGTAT GGAAGGCATG GACATGAGCC ACGCGGAATC CGCCCATTAA 100
<210> 11286
<211> 100
<212> DNA
<213> Escherichia coli K-12 MG1655
<220> cyoa b0433
<222> 820-919
<223> cyoa b0433 U00096 complement(449887..450834)
<313> 11286: 889-908
<400> 11286
TTTGCCGATG TAATTAACAA GTTTATGGCT CACGGTAAGA GCATGGACAT 50
GACCCAGCCA GAAGGTGAGC ACAGCGCACA CGAAGGTATG GAAGGCATGG 100
<210> 11287
<211> 100
<212> DNA
<213> Escherichia coli K-12 MG1655
<220> cyoa b0433
<222> 718-817
<223> cyoa b0433 U00096 complement(449887..450834)
<313> 11287: 763-782
<400> 11287
CAGTCGCCGA ACACCATGTC TGACATGGCT GCGTTCGAAA AACTGGCCGC 50
GCCTAGCGAA TACAACCAGG TGGAATATTT CTCCAACGTG AAACCAGACT 100
<210> 11288
<211> 100
<212> DNA
<213> Escherichia coli K-12 MG1655
<220> edd b1852
<222> 1701-1800
<223> edd b1852 U00096 complement(1930817..1932628)
<313> 11288: 1754-1773
<400> 11288
GCACATTCCT GACCTGAGCG CGTCACGCGT GGGAACAGGA CGTGAATTAT 50
TCAGCGCCTT GCGTGAAAAA CTGTCCGGTG CCGAACAGGG CGCAACCTGT 100
<210> 11289
<211> 100
<212> DNA
<213> Escherichia coli K-12 MG1655
<220> edd b1852
<222> 1516-1615
<223> edd b1852 U00096 complement(1930817..1932628)

<313> 11289: 1565-1584
<400> 11289
TTAGTTACCG ATGGACGACT CTCCGGCGCT TCAGGTAAAG TGCCGTCAGC 50
TATCCACGTA ACACCAGAAG CCTACGATGG CGGGCTGCTG GCAAAAGTGC 100
<210> 11290
<211> 100
<212> DNA
<213> Escherichia coli K-12 MG1655
<220> edd b1852
<222> 1470-1569
<223> edd b1852 U00096 complement(1930817..1932628)
<313> 11290: 1534-1553
<400> 11290
CATGCCGCCA CTTGGTGTAT TATTGGACCG GTGTTTCAAA ATTGCGTTAG 50
TTACCGATGG ACGACTCTCC GGCGCTTCAG GTAAAGTGCC GTCAGCTATC 100
<210> 11291
<211> 100
<212> DNA
<213> Escherichia coli K-12 MG1655
<220> cyob b0432
<222> 1893-1992
<223> cyob b0432 U00096 complement(447874..449865)
<313> 11291: 1963-1982
<400> 11291
CGAGGACGTG GATTACTACG TGCCGGTGGC AGAAATCGAA AAACTGGAAA 50
ACCAGCATTT CGATGAGATT ACTAAGGCAG GGCTGAAAAA TGGCAACTGA 100
<210> 11292
<211> 100
<212> DNA
<213> Escherichia coli K-12 MG1655
<220> cyob b0432
<222> 1860-1959
<223> cyob b0432 U00096 complement(447874..449865)
<313> 11292: 1897-1916
<400> 11292
CATGATCATC ACCTGGATCG TGAAAAGCTT CGACGAGGAC GTGGATTACT 50
ACGTGCCGGT GGCAGAAATC GAAAAACTGG AAAACCAGCA TTTCGATGAG 100
<210> 11293
<211> 100
<212> DNA
<213> Escherichia coli K-12 MG1655
<220> cyob b0432
<222> 1765-1864
<223> cyob b0432 U00096 complement(447874..449865)
<313> 11293: 1801-1820
<400> 11293
AGCGGTGCAG GTATCGTCAT TGCAGCTTTC TCCACCATCT TCGGTTTCGC 50
CATGATCTGG CATATCTGGT GGCTGGCGAT TGTTGGCTTC GCAGGCATGA 100
<210> 11294
<211> 100
<212> DNA
<213> Escherichia coli K-12 MG1655
<220> cyoc b0431
<222> 516-615
<223> cyoc b0431 U00096 complement(447270..447884)
<313> 11294: 582-601
<400> 11294
TAACCGTACC CGCATCATGT GCCTGAGCCT GTTCTGGCAC TTCCTGGATG 50
TGGTTTGGAT CTGTGTGTTC ACTGTTGTTT ATCTGATGGG GGCGATGTAA 100
<210> 11295
<211> 100
<212> DNA
<213> Escherichia coli K-12 MG1655
<220> cyoc b0431
<222> 489-588
<223> cyoc b0431 U00096 complement(447270..447884)
<313> 11295: 550-569
<400> 11295
AATCGCCCGT CGCGGCCTGA CCAGCACTAA CCGTACCCGC ATCATGTGCC 50
TGAGCCTGTT CTGGCACTTC CTGGATGTGG TTTGGATCTG TGTGTTCACT 100
<210> 11296

<211> 100
<212> DNA
<213> Escherichia coli K-12 MG1655
<220> cyoc b0431
<222> 386-485
<223> cyoc b0431 U00096 complement(447270..447884)
<313> 11296: 434-453
<400> 11296
GTCCGGATCG CAGCGGCTTC CTGTCAGCGT TCTTTGCGCT GGTCGGCACG 50
CACGGTCTGC ACGTCACTTC TGGTCTTATC TGGATGGCGG TGCTGATGGT 100
<210> 11297
<211> 100
<212> DNA
<213> Escherichia coli K-12 MG1655
<220> cyod b0430
<222> 231-330
<223> cyod b0430 U00096 complement(446941..447270)
<313> 11297: 266-285
<400> 11297
CATGACGGCG TTTGTCTTCA CCGTGCTAAT CATCGCTATC CTGGTTGTAG 50
GCTCCATCTG GATTATGTGG AACCTCAACT ACAACATGAT GATGCACTAA 100
<210> 11298
<211> 100
<212> DNA
<213> Escherichia coli K-12 MG1655
<220> cyod b0430
<222> 164-263
<223> cyod b0430 U00096 complement(446941..447270)
<313> 11298: 219-238
<400> 11298
TGGTACAGGT TCTGGTGCAT CTGGTGTGCT TCCTGCACAT GAATACCAAA 50
TCAGATGAAG GCTGGAACAT GACGGCGTTT GTCTTCACCG TGCTAATCAT 100
<210> 11299
<211> 100
<212> DNA
<213> Escherichia coli K-12 MG1655
<220> cyod b0430
<222> 125-224
<223> cyod b0430 U00096 complement(446941..447270)
<313> 11299: 143-162
<400> 11299
CTCCGGCCGT AATTCTGGGA ACAATCCTGG CAATGGCAGT GGTACAGGTT 50
CTGGTGCATC TGGTGTGCTT CCTGCACATG AATACCAAAT CAGATGAAGG 100
<210> 11300
<211> 100
<212> DNA
<213> Escherichia coli K-12 MG1655
<220> cyoe b0429
<222> 792-891
<223> cyoe b0429 U00096 complement(446039..446929)
<313> 11300: 821-840
<400> 11300
GTTCGGCTTC TCTATCATCG CCATCACTGC CCTCTCGGTG ATGATGTCCG 50
TTGATTTTAT GGTACCGGAC TCGCATACGC TGCTGGCTGC TGTGTGGTAA 100
<210> 11301
<211> 100
<212> DNA
<213> Escherichia coli K-12 MG1655
<220> cyoe b0429
<222> 633-732
<223> cyoe b0429 U00096 complement(446039..446929)
<313> 11301: 650-669
<400> 11301
GTATATCATC GCCTTTGCCG TTGCCACGCT GATGCTCTCT CTTGGCGGTT 50
ACGCTGGGTA TAAATATCTG GTGGTCGCCG CGGCGGTTAG CGTCTGGTGG 100
<210> 11302
<211> 100
<212> DNA
<213> Escherichia coli K-12 MG1655
<220> cyoe b0429
<222> 560-659

<223> cyoe b0429 U00096 complement(446039..446929)
<313> 11302: 622-641
<400> 11302
ATTACCAGGC GGCAAACATT CCGGTATTGC CAGTGGTAAA AGGCATTTCG 50
GTGGCGAAGA ATCACATCAC GCTGTATATC ATCGCCTTTG CCGTTGCCAC 100
<210> 11303
<211> 100
<212> DNA
<213> Escherichia coli K-12 MG1655
<220> apha b4056
<222> 599-698
<223> apha b4056 U00096 4266993..4267706
<313> 11303: 655-674
<400> 11303
CACGCGATGT CGGCGCTCGT GGTATCCGCA TTCTGCGCGC CTCCAACTCT 50
ACCTACAAAC CCTTGCCACA AGCGGGTGCG TTTGGTGAAG AGGTGATCGT 100
<210> 11304
<211> 100
<212> DNA
<213> Escherichia coli K-12 MG1655
<220> apha b4056
<222> 559-658
<223> apha b4056 U00096 4266993..4267706
<313> 11304: 576-595
<400> 11304
CGAATTTTTT ATGGCGATTC TGATAATGAT ATTACCGCCG CACGCGATGT 50
CGGCGCTCGT GGTATCCGCA TTCTGCGCGC CTCCAACTCT ACCTACAAAC 100
<210> 11305
<211> 100
<212> DNA
<213> Escherichia coli K-12 MG1655
<220> apha b4056
<222> 466-565
<223> apha b4056 U00096 4266993..4267706
<313> 11305: 506-525
<400> 11305
CATATTCCTG CCACCAACAT GAATCCGGTG ATCTTTGCGG GCGATAAACC 50
AGGGCAAAAT ACAAAATCGC AATGGCTGCA GGATAAAAAT ATCCGAATTT 100
<210> 11306
<211> 100
<212> DNA
<213> Escherichia coli K-12 MG1655
<220> b1200 b1200
<222> 1002-1101
<223> b1200 b1200 U00096 complement(1248991..1250091)
<313> 11306: 1071-1090
<400> 11306
TATGACCGGT TTCTCAATCA CCTTACTGAA AGTTGATGAC GAAACGCTGG 50
CACTCTGGGA CGCCCCGGTC CACACCCCGG CCCTTAACTG GGGTAAATAA 100
<210> 11307
<211> 100
<212> DNA
<213> Escherichia coli K-12 MG1655
<220> b1200 b1200
<222> 927-1026
<223> b1200 b1200 U00096 complement(1248991..1250091)
<313> 11307: 981-1000
<400> 11307
CCTGACCACA CGTTGCCAGC AAGCGGGATT GACTATCGAA CGTAATTTAA 50
TTGGCGCGTA CTGCACCTCA CTGGATATGA CCGGTTTCTC AATCACCTTA 100
<210> 11308
<211> 100
<212> DNA
<213> Escherichia coli K-12 MG1655
<220> b1200 b1200
<222> 897-996
<223> b1200 b1200 U00096 complement(1248991..1250091)
<313> 11308: 942-961
<400> 11308
GCTTTCTGAG CTGTACGGCG TCTATAACCG CCTGACCACA CGTTGCCAGC 50
AAGCGGGATT GACTATCGAA CGTAATTTAA TTGGCGCGTA CTGCACCTCA 100

<210> 11309
<211> 100
<212> DNA
<213> Escherichia coli K-12 MG1655
<220> sapa b1295
<222> 1545-1644
<223> sapa b1295 U00096 complement(1353491..1355134)
<313> 11309: 1579-1598
<400> 11309
CTATCGGTAC GATATCAAAG GTCTGGTACT TAGCCCGTTT GGTAACGCCT 50
CCTTTGCTGG GGTGTATCGC GAGAAACAGG ATGAGGTGAA AAAACCATGA 100
<210> 11310
<211> 100
<212> DNA
<213> Escherichia coli K-12 MG1655
<220> sapa b1295
<222> 1422-1521
<223> sapa b1295 U00096 complement(1353491..1355134)
<313> 11310: 1462-1481
<400> 11310
ATTGCGTAAG GCGCTCTCTT CGCAGCAGCT GGCGGCGCGT ATTGAAGCCT 50
ATGACGAAGC GCAGAGTATT CTGGCGCAGG AATTGCCCAT TTTGCCGCTG 100
<210> 11311
<211> 100
<212> DNA
<213> Escherichia coli K-12 MG1655
<220> sapa b1295
<222> 1294-1393
<223> sapa b1295 U00096 complement(1353491..1355134)
<313> 11311: 1326-1345
<400> 11311
CTGACGTTAT CCGGTTGGGC GACGGACAGT AACGACCCGG ACAGTTTCTT 50
CCGTCCGTTA CTGAGCTGCG CGGCAATTCA TTCGCAGACC AACCTCGCCC 100
<210> 11312
<211> 100
<212> DNA
<213> Escherichia coli K-12 MG1655
<220> b1201 b1201
<222> 1756-1855
<223> b1201 b1201 U00096 1250280..1252208
<313> 11312: 1804-1823
<400> 11312
TCCACCAGTC TGTCATTTGC GGAAGTTGAA AAAGAGGCAA TTATTAACGC 50
AGCCCAGGTC ACAGGCGGTC GCATTCAGGA AATGTCGGCT TTACTTGGGA 100
<210> 11313
<211> 100
<212> DNA
<213> Escherichia coli K-12 MG1655
<220> b1201 b1201
<222> 1726-1825
<223> b1201 b1201 U00096 1250280..1252208
<313> 11313: 1758-1777
<400> 11313
GCGACAGATG ATGTCAGCGC CACCCGCCTT TCCACCAGTC TGTCATTTGC 50
GGAAGTTGAA AAAGAGGCAA TTATTAACGC AGCCCAGGTC ACAGGCGGTC 100
<210> 11314
<211> 100
<212> DNA
<213> Escherichia coli K-12 MG1655
<220> b1201 b1201
<222> 1662-1761
<223> b1201 b1201 U00096 1250280..1252208
<313> 11314: 1693-1712
<400> 11314
TCTGAGTAGT GATAACGGGC GCATTCGCGT CAGTGATTTG CCGGAACATC 50
TGTTTACCGA GCAGGCGACA GATGATGTCA GCGCCACCCG CCTTTCCACC 100
<210> 11315
<211> 100
<212> DNA
<213> Escherichia coli K-12 MG1655
<220> sapb b1294

<222> 799-898
<223> sapb b1294 U00096 complement(1352529..1353494)
<313> 11315: 865-884
<400> 11315
GGTTTAGGGC GCTGGTTAAT TAACGCCATC CGCCAGCAGG ATTACGCAGC 50
CATTTCCGCC GGAGTGATGG TGTGTGGCTC ACTGGTCATT ATTGTTAACG 100
<210> 11316
<211> 100
<212> DNA
<213> Escherichia coli K-12 MG1655
<220> sapb b1294
<222> 646-745
<223> sapb b1294 U00096 complement(1352529..1353494)
<313> 11316: 670-689
<400> 11316
AAAGCGGCGG CGACCCGCGG TTTGTCGCGC TTTACTATTT TGCGTCGCCA 50
CGTTCTGCAT AACGCGTTAC CGCCGGTTAT TCCTCGCCTG GGCTTACAGT 100
<210> 11317
<211> 100
<212> DNA
<213> Escherichia coli K-12 MG1655
<220> sapb b1294
<222> 598-697
<223> sapb b1294 U00096 complement(1352529..1353494)
<313> 11317: 629-648
<400> 11317
CGCCTGATGC GTATCAGCAC TATCGAAGTG TATGACCAGA ACTATGTAAA 50
AGCGGCGGCG ACCCGCGGTT TGTCGCGCTT TACTATTTTG CGTCGCCACG 100
<210> 11318
<211> 100
<212> DNA
<213> Escherichia coli K-12 MG1655
<220> b1202 b1203
<222> 2769-2868
<223> b1202 b1203 U00096 complement(1252308..1255175)
<313> 11318: 2806-2825
<400> 11318
GTATAACAAA CAGCACACAT TCTATCTCGA AGCGGATTAC ACGCAGGGTA 50
ACCTCTTTGA TCAGAAGCAA GTCAACGGAG GATATCGCTT CAGCTTTTAA 100
<210> 11319
<211> 100
<212> DNA
<213> Escherichia coli K-12 MG1655
<220> b1202 b1203
<222> 2705-2804
<223> b1202 b1203 U00096 complement(1252308..1255175)
<313> 11319: 2742-2761
<400> 11319
ACTCCCGGGA GAAGTACAGT TTCAAAGGTA ATGGCTGGAA TAACGGCGTG 50
GGAGTCAGTG CACAGTATAA CAAACAGCAC ACATTCTATC TCGAAGCGGA 100
<210> 11320
<211> 100
<212> DNA
<213> Escherichia coli K-12 MG1655
<220> b1202 b1203
<222> 2649-2748
<223> b1202 b1203 U00096 complement(1252308..1255175)
<313> 11320: 2680-2699
<400> 11320
TGTGAAGACT GGCGCTATCC GCGAGTTTTC AGGGGATACC GAATATCTGT 50
TGAACAACTC CCGGGAGAAG TACAGTTTCA AAGGTAATGG CTGGAATAAC 100
<210> 11321
<211> 100
<212> DNA
<213> Escherichia coli K-12 MG1655
<220> sapc b1293
<222> 729-828
<223> sapc b1293 U00096 complement(1351652..1352542)
<313> 11321: 793-812
<400> 11321
CCCCTCGCCT GAATGGGGAG CGATGCTCGG TGATGCGCTG GAACTGATTT 50

ATGTCGCCCC GTGGACTGTC ATGCTGCCAG GTGCGGCAAT TATGATTAGC 100
<210> 11322
<211> 100
<212> DNA
<213> Escherichia coli K-12 MG1655
<220> sapc b1293
<222> 718-817
<223> sapc b1293 U00096 complement(1351652..1352542)
<313> 11322: 739-758
<400> 11322
GGCGCACAAC TCCCCTCGCC TGAATGGGGA GCGATGCTCG GTGATGCGCT 50
GGAACTGATT TATGTCGCCC CGTGGACTGT CATGCTGCCA GGTGCGGCAA 100
<210> 11323
<211> 100
<212> DNA
<213> Escherichia coli K-12 MG1655
<220> sapc b1293
<222> 536-635
<223> sapc b1293 U00096 complement(1351652..1352542)
<313> 11323: 553-572
<400> 11323
GCATGGTGCA TGACGAACTG GAAAAAGAGT ACGTTATCGC CGCCCGTCTG 50
GATGGCGCAT CAACGCTGAA TATTCTCTGG TTTGCTGTGA TGCCAAACAT 100
<210> 11324
<211> 100
<212> DNA
<213> Escherichia coli K-12 MG1655
<220> sapd b1292
<222> 894-993
<223> sapd b1292 U00096 complement(1350660..1351652)
<313> 11324: 954-973
<400> 11324
CCCGTATGCA CAACGAGAAT GCATTGTGAC GCCACGTTTG ACGGGGGCGA 50
AAAATCATCT CTATGCCTGT CATTTCCCGC TGAACATGGA GAAAGAGTGA 100
<210> 11325
<211> 100
<212> DNA
<213> Escherichia coli K-12 MG1655
<220> sapd b1292
<222> 837-936
<223> sapd b1292 U00096 complement(1350660..1351652)
<313> 11325: 894-913
<400> 11325
CGGCGCTATC CCACTGCTGG AACAGTTACC GATTGGTTGT CGTCTGGGGC 50
CACGTTGCCC GTATGCACAA CGAGAATGCA TTGTGACGCC ACGTTTGACG 100
<210> 11326
<211> 100
<212> DNA
<213> Escherichia coli K-12 MG1655
<220> sapd b1292
<222> 796-895
<223> sapd b1292 U00096 complement(1350660..1351652)
<313> 11326: 834-853
<400> 11326
GGCAGCGCGA TGCCGCATAA AAGTCGCCTC AATACGCTGC CCGGCGCTAT 50
CCCACTGCTG GAACAGTTAC CGATTGGTTG TCGTCTGGGG CCACGTTGCC 100
<210> 11327
<211> 100
<212> DNA
<213> Escherichia coli K-12 MG1655
<220> sapf b1291
<222> 708-807
<223> sapf b1291 U00096 complement(1349852..1350658)
<313> 11327: 788-807
<400> 11327
GGATGTGCTG GCATCGCCGC TGCATGAACT CACCAAACGG CTGATTGCCG 50
GTCATTTTGG CGAGGCATTG ACGGCGGATG CGTGGCGTAA AGATCGCTAA 100
<210> 11328
<211> 100
<212> DNA
<213> Escherichia coli K-12 MG1655

<220> sapf b1291
<222> 702-801
<223> sapf b1291 U00096 complement(1349852..1350658)
<313> 11328: 728-747
<400> 11328
CACCGCGGAT GTGCTGGCAT CGCCGCTGCA TGAACTCACC AAACGGCTGA  50
TTGCCGGTCA TTTTGGCGAG GCATTGACGG CGGATGCGTG GCGTAAAGAT  100
<210> 11329
<211> 100
<212> DNA
<213> Escherichia coli K-12 MG1655
<220> sapf b1291
<222> 581-680
<223> sapf b1291 U00096 complement(1349852..1350658)
<313> 11329: 631-650
<400> 11329
TGGAATTACA GGAAAAACAG GGCATTTCGT ATATTTATGT TACCCAGCAT  50
ATCGGAATGA TGAAGCACAT TAGCGACCAG GTGCTGGTGA TGCATCAGGG  100
<210> 11330
<211> 100
<212> DNA
<213> Escherichia coli K-12 MG1655
<220> b1213 b1214
<222> 294-393
<223> b1213 b1214 U00096 1266147..1266539
<313> 11330: 354-373
<400> 11330
GCACAGTCAG CAGGCGCGCA TTATTGCCTT CCCGCTGGCG TTGGTGGTGC  50
TGTACATCAT CATTAAACTC GCCACCACAA AAGTACCGTT ACTGGGGTAA  100
<210> 11331
<211> 100
<212> DNA
<213> Escherichia coli K-12 MG1655
<220> b1213 b1214
<222> 230-329
<223> b1213 b1214 U00096 1266147..1266539
<313> 11331: 280-299
<400> 11331
AAAAGCTGTT TGGAGTTATC ATTTATATCG TTTTGGGTTT TATTGCACTC  50
GATTATCGTC GTATGCACAG TCAGCAGGCG CGCATTATTG CCTTCCCGCT  100
<210> 11332
<211> 100
<212> DNA
<213> Escherichia coli K-12 MG1655
<220> b1213 b1214
<222> 183-282
<223> b1213 b1214 U00096 1266147..1266539
<313> 11332: 225-244
<400> 11332
TAAAGCGCAC ATCCTGCCAT TTTCCGGGCA GGCACAGTGG CTGACTGAAA  50
AGCTGTTTGG AGTTATCATT TATATCGTTT TGGGTTTTAT TGCACTCGAT  100
<210> 11333
<211> 100
<212> DNA
<213> Escherichia coli K-12 MG1655
<220> sprt b2945
<222> 353-452
<223> sprt b2945 U00096 3087774..3088271
<313> 11333: 398-417
<400> 11333
AACTGCAATC CGTGCGTCGC AACACCTTCC CCTACCGCTG CAAGTGCCAG  50
GAGCATCAGC TTACCGTACG CCGCCATAAT CGCGTAGTTC GTGGCGAGGC  100
<210> 11334
<211> 100
<212> DNA
<213> Escherichia coli K-12 MG1655
<220> sprt b2945
<222> 242-341
<223> sprt b2945 U00096 3087774..3088271
<313> 11334: 288-307
<400> 11334

CACATTTGCT GGTATGGAAA CATTTCGGCC GCGTAGCGCC ACATGGCAAA 50
GAGTGGAAGT GGATGATGGA AAACGTGCTG GGTGTTCCCG CCCGTCGTAC 100
<210> 11335
<211> 100
<212> DNA
<213> Escherichia coli K-12 MG1655
<220> sprt b2945
<222> 137-236
<223> sprt b2945 U00096 3087774..3088271
<313> 11335: 180-199
<400> 11335
CCGCCGGAAC GGCCTGGCTG GAAAGCTATG AAATTCGCCT CAATCCCGTT 50
TTGCTGTTGG AAAACAGTGA AGCTTTTATT GAAGAAGTGG TACCGCACGA 100
<210> 11336
<211> 100
<212> DNA
<213> Escherichia coli K-12 MG1655
<220> hrpa b1414
<222> 3747-3846
<223> hrpa b1414 U00096 1481142..1484987
<313> 11336: 3812-3831
<400> 11336
AGAAGAGTTG CGCGTTAGTT ACTTCGCTCA ACAACTTGGT ACGCCTTATC 50
CGATTTCAGA TAAGCGTATT TTGCAGGCGA TGGAGCAGAT TAGCGGTTAA 100
<210> 11337
<211> 100
<212> DNA
<213> Escherichia coli K-12 MG1655
<220> hrpa b1414
<222> 3721-3820
<223> hrpa b1414 U00096 1481142..1484987
<313> 11337: 3777-3796
<400> 11337
GACGTGAAAG AGATCCGTTG GATGATAGAA GAGTTGCGCG TTAGTTACTT 50
CGCTCAACAA CTTGGTACGC CTTATCCGAT TTCAGATAAG CGTATTTTGC 100
<210> 11338
<211> 100
<212> DNA
<213> Escherichia coli K-12 MG1655
<220> hrpa b1414
<222> 3711-3810
<223> hrpa b1414 U00096 1481142..1484987
<313> 11338: 3728-3747
<400> 11338
TGAGGATGAA GACGTGAAAG AGATCCGTTG GATGATAGAA GAGTTGCGCG 50
TTAGTTACTT CGCTCAACAA CTTGGTACGC CTTATCCGAT TTCAGATAAG 100
<210> 11339
<211> 100
<212> DNA
<213> Escherichia coli K-12 MG1655
<220> hrpb b0149
<222> 2376-2475
<223> hrpb b0149 U00096 162060..164534
<313> 11339: 2441-2460
<400> 11339
TGAGGTGCAA AAAGAGATGA AAGGGCGTTA TCCCAAACAT GTCTGGCCGG 50
ACGACCCGGC AAATACTGCA CCGACGCGAC GGACGAAAAA GTATTCGTAA 100
<210> 11340
<211> 100
<212> DNA
<213> Escherichia coli K-12 MG1655
<220> hrpb b0149
<222> 2363-2462
<223> hrpb b0149 U00096 162060..164534
<313> 11340: 2400-2419
<400> 11340
AAGGAGCGTA CCGTGAGGTG CAAAAAGAGA TGAAAGGGCG TTATCCCAAA 50
CATGTCTGGC CGGACGACCC GGCAAATACT GCACCGACGC GACGGACGAA 100
<210> 11341
<211> 100
<212> DNA

<213> Escherichia coli K-12 MG1655
<220> hrpb b0149
<222> 2303-2402
<223> hrpb b0149 U00096 162060..164534
<313> 11341: 2324-2343
<400> 11341
AGTTGCTTTC ACCTGCCCAA AGGCCATTAC AAATCACACG AGATTTGAGC  50
GACTTCTGGA AAGGAGCGTA CCGTGAGGTG CAAAAAGAGA TGAAAGGGCG  100
<210> 11342
<211> 100
<212> DNA
<213> Escherichia coli K-12 MG1655
<220> yneb yneB
<222> 746-845
<223> yneb yneB U00096 1604124..1604999
<313> 11342: 801-820
<400> 11342
GTGTGGATAT GGGGCGTAAT ATTTTCCAGT CTGACCATCC GGTGGCGATG  50
ATGAAAGCCG TACAGGCGGT GGTTCACCAT AACGAAACGG CTGATCGGGC  100
<210> 11343
<211> 100
<212> DNA
<213> Escherichia coli K-12 MG1655
<220> yneb yneB
<222> 732-831
<223> yneb yneB U00096 1604124..1604999
<313> 11343: 765-784
<400> 11343
TCAGGGCGCT TCTGGTGTGG ATATGGGGCG TAATATTTTC CAGTCTGACC  50
ATCCGGTGGC GATGATGAAA GCCGTACAGG CGGTGGTTCA CCATAACGAA  100
<210> 11344
<211> 100
<212> DNA
<213> Escherichia coli K-12 MG1655
<220> yneb yneB
<222> 659-758
<223> yneb yneB U00096 1604124..1604999
<313> 11344: 695-714
<400> 11344
TACCCATTGT TATTGCTGGC GGTAAAAAAT TACCGGAGCG CGAGGCGCTG  50
GAAATGTGCT GGCAGGCTAT CGATCAGGGC GCTTCTGGTG TGGATATGGG  100
<210> 11345
<211> 100
<212> DNA
<213> Escherichia coli K-12 MG1655
<220> tgt b0407
<222> 1029-1128
<223> tgt b0407 U00096 425361..426488
<313> 11345: 1090-1109
<400> 11345
GGGTTTACGC AAGGCTATTG AAGAGGGTAA ATTAGAGAGC TTCGTAACTG  50
ATTTTTACCA GCGTCAGGGG CGAGAAGTAC CACCTTTGAA CGTTGATTAA  100
<210> 11346
<211> 100
<212> DNA
<213> Escherichia coli K-12 MG1655
<220> tgt b0407
<222> 1010-1109
<223> tgt b0407 U00096 425361..426488
<313> 11346: 1039-1058
<400> 11346
ACTACCAGCG TTTGATGGCG GGTTTACGCA AGGCTATTGA AGAGGGTAAA  50
TTAGAGAGCT TCGTAACTGA TTTTTACCAG CGTCAGGGGC GAGAAGTACC  100
<210> 11347
<211> 100
<212> DNA
<213> Escherichia coli K-12 MG1655
<220> tgt b0407
<222> 946-1045
<223> tgt b0407 U00096 425361..426488
<313> 11347: 989-1008

<400> 11347
CATCATCTTG ACCGTTGCAA CGAAATATTA GGCGCGCGAC TCAACACCAT 50
TCATAACCTT CGTTACTACC AGCGTTTGAT GGCGGGTTTA CGCAAGGCTA 100
<210> 11348
<211> 100
<212> DNA
<213> Escherichia coli K-12 MG1655
<220> tufa b3340
<222> 100-199
<223> tufa b3340 U00096 complement(3467782..3468966)
<313> 11348: 149-168
<400> 11348
ACCGTACTGG CTAAAACCTA CGGCGGTGCT GCTCGTGCAT TCGACCAGAT 50
CGATAACGCG CCGGAAGAAA AAGCTCGTGG TATCACCATC AACACTTCTC 100
<210> 11349
<211> 100
<212> DNA
<213> Escherichia coli K-12 MG1655
<220> tufa b3340
<222> 58-157
<223> tufa b3340 U00096 complement(3467782..3468966)
<313> 11349: 89-108
<400> 11349
CACGTTGACC ACGGTAAAAC TACTCTGACC GCTGCAATCA CCACCGTACT 50
GGCTAAAACC TACGGCGGTG CTGCTCGTGC ATTCGACCAG ATCGATAACG 100
<210> 11350
<211> 100
<212> DNA
<213> Escherichia coli K-12 MG1655
<220> tufa b3340
<222> 29-128
<223> tufa b3340 U00096 complement(3467782..3468966)
<313> 11350: 82-101
<400> 11350
AACCGCACGT TAACGTTGGT ACTATCGGCC ACGTTGACCA CGGTAAAACT 50
ACTCTGACCG CTGCAATCAC CACCGTACTG GCTAAAACCT ACGGCGGTGC 100
<210> 11351
<211> 100
<212> DNA
<213> Escherichia coli K-12 MG1655
<220> tufb b3981
<222> 89-188
<223> tufb b3981 U00096 4173523..4174707
<313> 11351: 146-165
<400> 11351
CTGCAATCAC TACCGTACTG GCTAAAACCT ACGGCGGTGC TGCTCGCGCA 50
TTCGACCAGA TCGATAACGC GCCGGAAGAA AAAGCTCGTG GTATCACCAT 100
<210> 11352
<211> 100
<212> DNA
<213> Escherichia coli K-12 MG1655
<220> tufb b3981
<222> 43-142
<223> tufb b3981 U00096 4173523..4174707
<313> 11352: 91-110
<400> 11352
GTCGGTACTA TCGGCCACGT TGACCATGGT AAAACAACGC TGACCGCTGC 50
AATCACTACC GTACTGGCTA AAACCTACGG CGGTGCTGCT CGCGCATTCG 100
<210> 11353
<211> 100
<212> DNA
<213> Escherichia coli K-12 MG1655
<220> tufb b3981
<222> 33-132
<223> tufb b3981 U00096 4173523..4174707
<313> 11353: 88-107
<400> 11353
GCACGTTAAC GTCGGTACTA TCGGCCACGT TGACCATGGT AAAACAACGC 50
TGACCGCTGC AATCACTACC GTACTGGCTA AAACCTACGG CGGTGCTGCT 100
<210> 11354
<211> 100

<212> DNA
<213> Escherichia coli K-12 MG1655
<220> yneh yneH
<222> 828-927
<223> yneh yneH U00096 complement(1610349..1611275)
<313> 11354: 888-907
<400> 11354
CGCTGTCTGG AGTCCGGAAC TGGATGATGC AGGTAACTCG CTTGCGGGTA 50
TTGCCGTTCT TGAACAATTG ACGAAACAGT TAGGGCGTTC GGTTTATTAA 100
<210> 11355
<211> 100
<212> DNA
<213> Escherichia coli K-12 MG1655
<220> yneh yneH
<222> 718-817
<223> yneh yneH U00096 complement(1610349..1611275)
<313> 11355: 759-778
<400> 11355
ACCAGTGGTA TGTACCAGAA CGCGGGGGAG TTTGCCTGGC GGGTGGGGCT 50
ACCGGCGAAA TCTGGCGTTG GTGGCGGTAT TGTGGCGATT GTTCCGCATG 100
<210> 11356
<211> 100
<212> DNA
<213> Escherichia coli K-12 MG1655
<220> yneh yneH
<222> 647-746
<223> yneh yneH U00096 complement(1610349..1611275)
<313> 11356: 680-699
<400> 11356
GGAAAGCTAT TCATATTGAT GAACCAGTGG TGACGCCAAT GCAGGCGCGG 50
CAAATTAACG CGCTGATGGC GACCAGTGGT ATGTACCAGA ACGCGGGGGA 100
<210> 11357
<211> 100
<212> DNA
<213> Escherichia coli K-12 MG1655
<220> ynej yneJ
<222> 783-882
<223> ynej yneJ U00096 1612828..1613709
<313> 11357: 859-878
<400> 11357
AACCTGGCTG GTCTGGCGTC GTGGTGCGAA AACACGTCCG CTCGAGGCAT 50
TTATTCAACT GCTGGATGTG CCTGACTCGG CAAGACAGGG ATATCAATGA 100
<210> 11358
<211> 100
<212> DNA
<213> Escherichia coli K-12 MG1655
<220> ynej yneJ
<222> 687-786
<223> ynej yneJ U00096 1612828..1613709
<313> 11358: 722-741
<400> 11358
AGGCATTGCG CTTATTCCGC GCTCTATGCT GGAAAGTATG CCGGGGCATC 50
ACCAGGTTGA AGCGTGGCCG TTAGCTGAGC AATGGCGTTG GTTAACAACC 100
<210> 11359
<211> 100
<212> DNA
<213> Escherichia coli K-12 MG1655
<220> ynej yneJ
<222> 613-712
<223> ynej yneJ U00096 1612828..1613709
<313> 11359: 647-666
<400> 11359
GACGGTGCCG CTCCGGGAAC TATCCATGAG ATGGAGTCTT ATCACGGAAT 50
GTTGGCCTGT GTGATCGCAG GAGCAGGCAT TGCGCTTATT CCGCGCTCTA 100
<210> 11360
<211> 100
<212> DNA
<213> Escherichia coli K-12 MG1655
<220> rspa rspA
<222> 1116-1215
<223> rspa rspA U00096 complement(1651951..1653165)

<313> 11360: 1187-1206
<400> 11360
TGGTATCGAA TTCGATGAAA AGCTGGCGGC GAAATATCCC TATGAACCTG 50
CTTATCTACC AGTCGCACGT CTGGAAGATG GCACGCTGTG GAACTGGTAA 100
<210> 11361
<211> 100
<212> DNA
<213> Escherichia coli K-12 MG1655
<220> rspa rspA
<222> 1087-1186
<223> rspa rspA U00096 complement(1651951..1653165)
<313> 11361: 1143-1162
<400> 11361
TATATGCATC CGGGAGACAA ACCGGGTCTT GGTATCGAAT TCGATGAAAA 50
GCTGGCGGCG AAATATCCCT ATGAACCTGC TTATCTACCA GTCGCACGTC 100
<210> 11362
<211> 100
<212> DNA
<213> Escherichia coli K-12 MG1655
<220> rspa rspA
<222> 1028-1127
<223> rspa rspA U00096 complement(1651951..1653165)
<313> 11362: 1081-1100
<400> 11362
TGGGTTATTC CGAACAAATG CTCGAAGTCT TCCCGCACAA CTGGACTTTC 50
GATAACGGCT ATATGCATCC GGGAGACAAA CCGGGTCTTG GTATCGAATT 100
<210> 11363
<211> 100
<212> DNA
<213> Escherichia coli K-12 MG1655
<220> rspb b1581
<222> 747-846
<223> rspb b1581 U00096 complement(1650920..1651939)
<313> 11363: 791-810
<400> 11363
TTCTCCAGCG GCACGTATTG TATTGATGGG GTTCTCCAGT GAACCGTCTG 50
AAGTGATTCA GCAAGGAATT ACCGGAAAAG AACTCTCTAT TTTCTCTTCA 100
<210> 11364
<211> 100
<212> DNA
<213> Escherichia coli K-12 MG1655
<220> rspb b1581
<222> 676-775
<223> rspb b1581 U00096 complement(1650920..1651939)
<313> 11364: 725-744
<400> 11364
GGCATCAAGC CGACATTAAT TATCGATGCG GCTTGTCATC CTTCTATCCT 50
GAAAGAGGCC GTAACGCTGG CTTCTCCAGC GGCACGTATT GTATTGATGG 100
<210> 11365
<211> 100
<212> DNA
<213> Escherichia coli K-12 MG1655
<220> rspb b1581
<222> 638-737
<223> rspb b1581 U00096 complement(1650920..1651939)
<313> 11365: 657-676
<400> 11365
ACAGCCAGAC ACCGCTTGGC GAGATTTTCA CTGAAAAAGG CATCAAGCCG 50
ACATTAATTA TCGATGCGGC TTGTCATCCT TCTATCCTGA AAGAGGCCGT 100
<210> 11366
<211> 100
<212> DNA
<213> Escherichia coli K-12 MG1655
<220> efp b4148
<222> 468-567
<223> efp b4148 U00096 4373277..4373843
<313> 11366: 532-551
<400> 11366
GTCTACTGGC GCTGTGGTTA AAGTTCCGCT GTTTGTACAA ATCGGCGAAG 50
TCATCAAAGT GGATACCCGC TCTGGTGAAT ACGTCTCTCG CGTGAAGTAA 100
<210> 11367

<211> 100
<212> DNA
<213> Escherichia coli K-12 MG1655
<220> efp b4148
<222> 393-492
<223> efp b4148 U00096 4373277..4373843
<313> 11367: 433-452
<400> 11367
TGAACTGGAA ATCGTTGATA CCGATCCGGG CCTGAAAGGT GATACCGCAG 50
GTACTGGTGG CAAACCGGCT ACCCTGTCTA CTGGCGCTGT GGTTAAAGTT 100
<210> 11368
<211> 100
<212> DNA
<213> Escherichia coli K-12 MG1655
<220> efp b4148
<222> 331-430
<223> efp b4148 U00096 4373277..4373843
<313> 11368: 387-406
<400> 11368
GCAGAGTGTA TCGTAACTCT GTGGAATGGT CAGCCGATCT CCGTTACTCC 50
GCCGAACTTC GTTGAACTGG AAATCGTTGA TACCGATCCG GGCCTGAAAG 100
<210> 11369
<211> 100
<212> DNA
<213> Escherichia coli K-12 MG1655
<220> b1228 b1229
<222> 79-178
<223> b1228 b1229 U00096 1285932..1286207
<313> 11369: 119-138
<400> 11369
GCGCTCAAGT CGAACCTTGG TCGAAGCTTC TCATCCTTCC CCGCTTGGGC 50
AGAATATTTG ATTGCGGATT CGTTTGAGAA TTCCGGGGCT TTTGAAAGTG 100
<210> 11370
<211> 100
<212> DNA
<213> Escherichia coli K-12 MG1655
<220> ynfc ynfC
<222> 647-746
<223> ynfc ynfC U00096 complement(1654771..1655517)
<313> 11370: 686-705
<400> 11370
ACGCTAATCC GGTGGACTGT CAGCTAATCA TTGTTGATGA AGGAGTAAAA 50
CCCGCCGTCG AACGGGTTTA CACCATCAAA AATACGATCG ATTATTATTA 100
<210> 11371
<211> 100
<212> DNA
<213> Escherichia coli K-12 MG1655
<220> ynfc ynfC
<222> 599-698
<223> ynfc ynfC U00096 complement(1654771..1655517)
<313> 11371: 625-644
<400> 11371
ATAATCAACG GGTTGGTAAT GTAAAACAGA GCTGTGAATA TGACAGTCAC 50
GCTAATCCGG TGGACTGTCA GCTAATCATT GTTGATGAAG GAGTAAAACC 100
<210> 11372
<211> 100
<212> DNA
<213> Escherichia coli K-12 MG1655
<220> ynfc ynfC
<222> 488-587
<223> ynfc ynfC U00096 complement(1654771..1655517)
<313> 11372: 529-548
<400> 11372
GTTATCCGCT GGGTAAAACC ACGAAAAGTA ACGACAAAAC ATTATCTGTC 50
AGCGCCACGC CATCAACGGA TCCGATCAAA AAATTAGATT ACACAGCGGT 100
<210> 11373
<211> 100
<212> DNA
<213> Escherichia coli K-12 MG1655
<220> pyka b1855
<222> 1344-1443

<223> pyka b1855 U00096 1935673..1937115
<313> 11373: 1401-1420
<400> 11373
AGGTTACTTG ATGTCTGGTG ACCTGGTGAT TGTCACCCAG GGCGACGTGA 50
TGAGTACCGT GGGTTCTACT AATACCACGC GTATTTTAAC GGTAGAGTAA 100
<210> 11374
<211> 100
<212> DNA
<213> Escherichia coli K-12 MG1655
<220> pyka b1855
<222> 1184-1283
<223> pyka b1855 U00096 1935673..1937115
<313> 11374: 1218-1237
<400> 11374
CCTCCCGTAT CAGCTCTGGT CTGCCAATTT TCGCCATGTC GCGCCATGAA 50
CGTACGCTGA ACCTGACTGC TCTCTATCGT GGCGTTACGC CGGTGCACTT 100
<210> 11375
<211> 100
<212> DNA
<213> Escherichia coli K-12 MG1655
<220> pyka b1855
<222> 1125-1224
<223> pyka b1855 U00096 1935673..1937115
<313> 11375: 1150-1169
<400> 11375
CCTGAAAGGC GTTACGGCGA TCATCACCAT GACCGAATCG GGTCGTACCG 50
CGCTGATGAC CTCCCGTATC AGCTCTGGTC TGCCAATTTT CGCCATGTCG 100
<210> 11376
<211> 100
<212> DNA
<213> Escherichia coli K-12 MG1655
<220> ynfl b1596
<222> 795-894
<223> ynfl b1596 U00096 complement(1666723..1667616)
<313> 11376: 818-837
<400> 11376
GGATGCGGTT TCTGAAATGT GGTTGGTCTG GCCGAAACAT CATGAACAAA 50
GTCCGGCTGC GCGTAACTTT CGTATTCATC TGCTGAATGC TCTCAGGTGA 100
<210> 11377
<211> 100
<212> DNA
<213> Escherichia coli K-12 MG1655
<220> ynfl b1596
<222> 732-831
<223> ynfl b1596 U00096 complement(1666723..1667616)
<313> 11377: 777-796
<400> 11377
TTTGCCTGCG TCATTTAAAC GTGTTCAGCT CAACGAAATG CGCTGGGTGC 50
CGATTGCTGA AGAGGATGCG GTTTCTGAAA TGTGGTTGGT CTGGCCGAAA 100
<210> 11378
<211> 100
<212> DNA
<213> Escherichia coli K-12 MG1655
<220> ynfl b1596
<222> 699-798
<223> ynfl b1596 U00096 complement(1666723..1667616)
<313> 11378: 717-736
<400> 11378
CGGTCTGGTT TCCGCCGGTC TGGGTGTTTC AATTTTGCCT GCGTCATTTA 50
AACGTGTTCA GCTCAACGAA ATGCGCTGGG TGCCGATTGC TGAAGAGGAT 100
<210> 11379
<211> 100
<212> DNA
<213> Escherichia coli K-12 MG1655
<220> ynfm b1597
<222> 1136-1235
<223> ynfm b1597 U00096 1667723..1668976
<313> 11379: 1186-1205
<400> 11379
CGCTGGGTGG TGTTTTCTGG CATAACTATG GCTGGAACGG CGTCGGCGCA 50
TTTATTGCTC TGATGCTGGT CATTGCTCTG CTGGTCGGGA CGCGTTTGCA 100

<210> 11380
<211> 100
<212> DNA
<213> Escherichia coli K-12 MG1655
<220> ynfm b1597
<222> 1088-1187
<223> ynfm b1597 U00096 1667723..1668976
<313> 11380: 1113-1132
<400> 11380
CCTCGCTGTA TCTGTTCAGT TACTATCTGG GGTCGAGTAT TGCCGGGACG 50
CTGGGTGGTG TTTTCTGGCA TAACTATGGC TGGAACGGCG TCGGCGCATT 100
<210> 11381
<211> 100
<212> DNA
<213> Escherichia coli K-12 MG1655
<220> ynfm b1597
<222> 1015-1114
<223> ynfm b1597 U00096 1667723..1668976
<313> 11381: 1060-1079
<400> 11381
GGATTCTTCG CAGCCCACTC AGTAGCCAGC AGCTGGATCG GCCCCCGCGC 50
AAAACGCGCT AAAGGCCAGG CCTCCTCGCT GTATCTGTTC AGTTACTATC 100
<210> 11382
<211> 100
<212> DNA
<213> Escherichia coli K-12 MG1655
<220> pykf b1677
<222> 1314-1413
<223> pykf b1677 U00096 1753722..1755134
<313> 11382: 1393-1412
<400> 11382
GCAGAGCGGT CTGGCACACA AAGGTGACGT TGTAGTTATG GTTTCTGGTG 50
CACTGGTACC GAGCGGCACT ACTAACACCG CATCTGTTCA CGTCCTGTAA 100
<210> 11383
<211> 100
<212> DNA
<213> Escherichia coli K-12 MG1655
<220> pykf b1677
<222> 1240-1339
<223> pykf b1677 U00096 1753722..1755134
<313> 11383: 1282-1301
<400> 11383
GGCGTTGTGC CGCAGCTTGT TAAAGAGATC ACTTCTACTG ATGATTTCTA 50
CCGTCTGGGT AAAGAACTGG CTCTGCAGAG CGGTCTGGCA CACAAAGGTG 100
<210> 11384
<211> 100
<212> DNA
<213> Escherichia coli K-12 MG1655
<220> pykf b1677
<222> 1190-1289
<223> pykf b1677 U00096 1753722..1755134
<313> 11384: 1255-1274
<400> 11384
TGGCACTGAC CACCAACGAA AAAACGGCTC ATCAGTTGGT ACTGAGCAAA 50
GGCGTTGTGC CGCAGCTTGT TAAAGAGATC ACTTCTACTG ATGATTTCTA 100
<210> 11385
<211> 100
<212> DNA
<213> Escherichia coli K-12 MG1655
<220> tig b0437
<222> 1200-1299
<223> tig b0437 U00096 454357..455655
<313> 11385: 1278-1297
<400> 11385
TGTTGCTCTG GAAGAACAGG CTGTTGAAGC TGTACTGGCG AAAGCGAAAG 50
TGACTGAAAA AGAAACCACT TTCAACGAGC TGATGAACCA GCAGGCGTAA 100
<210> 11386
<211> 100
<212> DNA
<213> Escherichia coli K-12 MG1655
<220> tig b0437

<222> 1171-1270
<223> tig b0437 U00096 454357..455655
<313> 11386: 1223-1242
<400> 11386
AACAAAGAAC TGATGGACAA CATGCGCAAT GTTGCTCTGG AAGAACAGGC 50
TGTTGAAGCT GTACTGGCGA AAGCGAAAGT GACTGAAAAA GAAACCACTT 100
<210> 11387
<211> 100
<212> DNA
<213> Escherichia coli K-12 MG1655
<220> tig b0437
<222> 1050-1149
<223> tig b0437 U00096 454357..455655
<313> 11387: 1070-1089
<400> 11387
GCTGGGCGAA GTTATCCGCA CCAACGAGCT GAAAGCTGAC GAAGAGCGCG 50
TGAAAGGCCT GATCGAAGAG ATGGCTTCTG CGTACGAAGA TCCGAAAGAA 100
<210> 11388
<211> 100
<212> DNA
<213> Escherichia coli K-12 MG1655
<220> b0501 b0501
<222> 93-192
<223> b0501 b0501 U00096 528163..528354
<313> 11388: 157-176
<400> 11388
AGGGGATGGT GTAAACCTTT TTTTTGTTGG TGAAATTAAT GTTACGCATT 50
ATTTTATAAC AAATATTGGA GCTGGATTGC CTGATGCTTG TGCAGAGTAA 100
<210> 11389
<211> 100
<212> DNA
<213> Escherichia coli K-12 MG1655
<220> b0501 b0501
<222> 41-140
<223> b0501 b0501 U00096 528163..528354
<313> 11389: 68-87
<400> 11389
ATGATCCAGA TACAAAAAAA GTGTGGTGTA AATATGGTAA GATACCAGGG 50
CAAGGGGATG GTGTAAACCT TTTTTTTGTT GGTGAAATTA ATGTTACGCA 100
<210> 11390
<211> 100
<212> DNA
<213> Escherichia coli K-12 MG1655
<220> b0501 b0501
<222> 1-100
<223> b0501 b0501 U00096 528163..528354
<313> 11390: 19-38
<400> 11390
GTGAGGGCCA GGGAACAAGT GGCGAAAATC GTATCAAAGA ATGATCCAGA 50
TACAAAAAAA GTGTGGTGTA AATATGGTAA GATACCAGGG CAAGGGGATG 100
<210> 11391
<211> 100
<212> DNA
<213> Escherichia coli K-12 MG1655
<220> b0502 b0503
<222> 309-408
<223> b0502 b0503 U00096 complement(528869..529276)
<313> 11391: 358-377
<400> 11391
CAGCAACGAG CTGCGTAAAC AGGGGGTTTT TATCTCCGAC AGTGGTGTCC 50
GTTCTGTCTG GTTACTCCAT AACCTTGAGA ATCTCAAAAG ACGATACTGA 100
<210> 11392
<211> 100
<212> DNA
<213> Escherichia coli K-12 MG1655
<220> b0502 b0503
<222> 287-386
<223> b0502 b0503 U00096 complement(528869..529276)
<313> 11392: 324-343
<400> 11392
CGACACATGG TCAGCACCGG GCCAGCAACG AGCTGCGTAA ACAGGGGGTT 50

TTTATCTCCG ACAGTGGTGT CCGTTCTGTC TGGTTACTCC ATAACCTTGA  100
<210> 11393
<211> 100
<212> DNA
<213> Escherichia coli K-12 MG1655
<220> b0502 b0503
<222> 206-305
<223> b0502 b0503 U00096 complement(528869..529276)
<313> 11393: 264-283
<400> 11393
GTAGTCGCCG CGCTCCTAAC CTTAAGAACC GTACCGATGA GGCAACTGAA  50
CAGGCTGTTG TTGATTACGC CGTCGCTTTC CCGACACATG GTCAGCACCG  100
<210> 11394
<211> 100
<212> DNA
<213> Escherichia coli K-12 MG1655
<220> cysa b2423
<222> 999-1098
<223> cysa b2423 U00096 complement(2537737..2538834)
<313> 11394: 1072-1091
<400> 11394
CGAGCGTTTA TTCGTTGGTC TGCAACATGC GCGGCTGTAT AACGGCGACG  50
AGCGTATCGA AACCCGCGAT GAGGAACTTG CTCTCGCACA AAGCGCCTGA  100
<210> 11395
<211> 100
<212> DNA
<213> Escherichia coli K-12 MG1655
<220> cysa b2423
<222> 980-1079
<223> cysa b2423 U00096 complement(2537737..2538834)
<313> 11395: 1012-1031
<400> 11395
ACGATGCCCC GCAGCGTGGC GAGCGTTTAT TCGTTGGTCT GCAACATGCG  50
CGGCTGTATA ACGGCGACGA GCGTATCGAA ACCCGCGATG AGGAACTTGC  100
<210> 11396
<211> 100
<212> DNA
<213> Escherichia coli K-12 MG1655
<220> cysa b2423
<222> 911-1010
<223> cysa b2423 U00096 complement(2537737..2538834)
<313> 11396: 929-948
<400> 11396
ACTACACCCA ATTAGTGGTG CAGCCGCTGG GGTGGTACAA CGAACCGCTG  50
ACGGTCGTGA TGCATGGCGA CGATGCCCCG CAGCGTGGCG AGCGTTTATT  100
<210> 11397
<211> 100
<212> DNA
<213> Escherichia coli K-12 MG1655
<220> cysb b1276
<222> 876-975
<223> cysb b1276 U00096 1331879..1332853
<313> 11397: 923-942
<400> 11397
ACCGCATTTA ACGCGTGATG TCGTTGATGC GGCTGTCGCA TTGCGCTCTA  50
ATGAAGAAAT TGAGGTCATG TTTAAAGATA TAAAACTGCC GGAAAAATAA  100
<210> 11398
<211> 100
<212> DNA
<213> Escherichia coli K-12 MG1655
<220> cysb b1276
<222> 684-783
<223> cysb b1276 U00096 1331879..1332853
<313> 11398: 707-726
<400> 11398
CGTCATTAAA ACTTACGTCC GGTTAGGGCT GGGGGTAGGG GTCATTGCCA  50
GCATGGCGGT GGATCCGGTC GCCGATCCCG ACCTTGTGCG TGTTGATGCT  100
<210> 11399
<211> 100
<212> DNA
<213> Escherichia coli K-12 MG1655

<220> cysb b1276
<222> 611-710
<223> cysb b1276 U00096 1331879..1332853
<313> 11399: 660-679
<400> 11399
GTTCAGAACT GGATACTGCC TTTAATCGCG CAGGGTTAAC GCCGCGTATC 50
GTTTTCACGG CAACGGATGC TGACGTCATT AAAACTTACG TCCGGTTAGG 100
<210> 11400
<211> 100
<212> DNA
<213> Escherichia coli K-12 MG1655
<220> cysc b2751
<222> 473-572
<223> cysc b2751 U00096 complement(2871410..2872015)
<313> 11400: 504-523
<400> 11400
ACTTTACGGG AATAGATTCC GTTTACGAAG CGCCTGAATC GGCAGAAATT 50
CATCTCAATG GTGAACAATT AGTAACAAAT TTGGTACAGC AATTATTAGA 100
<210> 11401
<211> 100
<212> DNA
<213> Escherichia coli K-12 MG1655
<220> cysc b2751
<222> 304-403
<223> cysc b2751 U00096 complement(2871410..2872015)
<313> 11401: 334-353
<400> 11401
GTGGTGCTGA CCGCATTTAT CTCGCCACAC CGCGCCGAAC GCCAGATGGT 50
TCGCGAACGC GTAGGAGAAG GGCGCTTTAT CGAAGTGTTT GTCGATACGC 100
<210> 11402
<211> 100
<212> DNA
<213> Escherichia coli K-12 MG1655
<220> cysc b2751
<222> 155-254
<223> cysc b2751 U00096 complement(2871410..2872015)
<313> 11402: 218-237
<400> 11402
TACATAAACT CGGCGTCAGT ACGTATCTGC TGGATGGCGA CAATGTTCGC 50
CACGGATTAT GCAGCGATCT CGGTTTTAGC GATGCCGATC GTAAAGAGAA 100
<210> 11403
<211> 100
<212> DNA
<213> Escherichia coli K-12 MG1655
<220> cysd b2753
<222> 810-909
<223> cysd b2753 U00096 complement(2873444..2874352)
<313> 11403: 849-868
<400> 11403
AGAGATGCTG GTTTCCACCA CCAGTGAACG TCAGGGCCGC GTGATTGACC 50
GCGACCAGGC GGGGTCTATG GAGCTGAAAA AACGTCAGGG GTATTTTTAA 100
<210> 11404
<211> 100
<212> DNA
<213> Escherichia coli K-12 MG1655
<220> cysd b2753
<222> 767-866
<223> cysd b2753 U00096 complement(2873444..2874352)
<313> 11404: 787-806
<400> 11404
GTGCGGTGGA GTCAAATGCA CAAACACTGC CGGAAATCAT CGAAGAGATG 50
CTGGTTTCCA CCACCAGTGA ACGTCAGGGC CGCGTGATTG ACCGCGACCA 100
<210> 11405
<211> 100
<212> DNA
<213> Escherichia coli K-12 MG1655
<220> cysd b2753
<222> 653-752
<223> cysd b2753 U00096 complement(2873444..2874352)
<313> 11405: 717-736
<400> 11405

AACGCGACGG TATGTTGATG ATGATTGATG ACAACCGTAT CGACCTGCAA 50
CCGGGCGAAG TGATTAAAAA ACGGATGGTG CGTTTCCGTA CGCTGGGCTG 100
<210> 11406
<211> 100
<212> DNA
<213> Escherichia coli K-12 MG1655
<220> cyse b3608
<222> 723-822
<223> cyse b3608 U00096 complement(3779368..3780189)
<313> 11406: 755-774
<400> 11406
TCGTATTGTC GGTAAACCAG ACAGCGATAA GCCATCAATG GATATGGACC 50
AGCATTTCAA CGGTATTAAC CATACATTTG AGTATGGGGA TGGGATCTAA 100
<210> 11407
<211> 100
<212> DNA
<213> Escherichia coli K-12 MG1655
<220> cyse b3608
<222> 612-711
<223> cyse b3608 U00096 complement(3779368..3780189)
<313> 11407: 654-673
<400> 11407
GGGCGCGAAA ATCCTCGGCA ATATTGAAGT TGGGCGCGGC GCGAAGATTG 50
GCGCAGGTTC CGTGGTGCTG CAACCGGTGC CGCCGCATAC CACCGCCGCT 100
<210> 11408
<211> 100
<212> DNA
<213> Escherichia coli K-12 MG1655
<220> cyse b3608
<222> 569-668
<223> cyse b3608 U00096 complement(3779368..3780189)
<313> 11408: 611-630
<400> 11408
GTGACCGTCA CCCGAAAATT CGTGAAGGTG TGATGATTGG CGCGGGCGCG 50
AAAATCCTCG GCAATATTGA AGTTGGGCGC GGCGCGAAGA TTGGCGCAGG 100
<210> 11409
<211> 100
<212> DNA
<213> Escherichia coli K-12 MG1655
<220> cysg b3369
<222> 1275-1374
<223> cysg b3369 U00096 3495465..3496838
<313> 11409: 1337-1356
<400> 11409
GCTGGGAGAA CTGGCGCAGC AAATGAACAG TCCATCGCTA ATTATTATTG 50
GTCGGGTTGT TGGCCTGCGC GATAAACTGA ACTGGTTCTC CAACCATTAA 100
<210> 11410
<211> 100
<212> DNA
<213> Escherichia coli K-12 MG1655
<220> cysg b3369
<222> 1256-1355
<223> cysg b3369 U00096 3495465..3496838
<313> 11410: 1293-1312
<400> 11410
TTGACGGTAC GCTCACACAG CTGGGAGAAC TGGCGCAGCA AATGAACAGT 50
CCATCGCTAA TTATTATTGG TCGGGTTGTT GGCCTGCGCG ATAAACTGAA 100
<210> 11411
<211> 100
<212> DNA
<213> Escherichia coli K-12 MG1655
<220> cysg b3369
<222> 1138-1237
<223> cysg b3369 U00096 3495465..3496838
<313> 11411: 1189-1208
<400> 11411
TTCTATATGG GGTTGAATCA GGCCGCGACT ATTCAGCAAA AGCTGATTGA 50
ACACGGAATG CCAGGCGAAA TGCCGGTGGC AATTGTCGAA AACGGTACGG 100
<210> 11412
<211> 100
<212> DNA

<213> Escherichia coli K-12 MG1655
<220> cysh b2763
<222> 636-735
<223> cysh b2763 U00096 complement(2885601..2886335)
<313> 11412: 701-720
<400> 11412
GGGCGATACC CATACAACCC GTAAATGGGA ACCCGGCATG GCGGAAGAAG 50
AAACGCGTTT CTTTGGCTTA AAAAGGGAAT GTGGGTTACA CGAAGGGTAA 100
<210> 11413
<211> 100
<212> DNA
<213> Escherichia coli K-12 MG1655
<220> cysh b2763
<222> 636-735
<223> cysh b2763 U00096 complement(2885601..2886335)
<313> 11413: 654-673
<400> 11413
GGGCGATACC CATACAACCC GTAAATGGGA ACCCGGCATG GCGGAAGAAG 50
AAACGCGTTT CTTTGGCTTA AAAAGGGAAT GTGGGTTACA CGAAGGGTAA 100
<210> 11414
<211> 100
<212> DNA
<213> Escherichia coli K-12 MG1655
<220> cysh b2763
<222> 539-638
<223> cysh b2763 U00096 complement(2885601..2886335)
<313> 11414: 580-599
<400> 11414
TGCCGATTAT CGACTGGGAT AACCGAACTA TTTATCAGTA CCTGCAAAAA 50
CATGGCCTGA AATATCACCC ATTATGGGAT GAAGGATATT TATCGGTGGG 100
<210> 11415
<211> 100
<212> DNA
<213> Escherichia coli K-12 MG1655
<220> cysi b2764
<222> 1614-1713
<223> cysi b2764 U00096 complement(2886410..2888122)
<313> 11415: 1691-1710
<400> 11415
GGCGAAAGAG CGCGAAGCGG GTGAAGGCTT CGGCGACTTT ACGGTGCGTG 50
CGGGCATCAT TCGCCCGGTG CTCGATCCGG CGCGTGATTT GTGGGATTAA 100
<210> 11416
<211> 100
<212> DNA
<213> Escherichia coli K-12 MG1655
<220> cysi b2764
<222> 1559-1658
<223> cysi b2764 U00096 complement(2886410..2888122)
<313> 11416: 1610-1629
<400> 11416
AAAACATCAC CGAGCCGGAA ATCCTGGCGT CGCTTGATGA ACTGATAGGG 50
CGCTGGGCGA AAGAGCGCGA AGCGGGTGAA GGCTTCGGCG ACTTTACGGT 100
<210> 11417
<211> 100
<212> DNA
<213> Escherichia coli K-12 MG1655
<220> cysi b2764
<222> 1519-1618
<223> cysi b2764 U00096 complement(2886410..2888122)
<313> 11417: 1552-1571
<400> 11417
GGCAACCGCA TTGGGACACG TATCCCACGG ATGTATAAAG AAAACATCAC 50
CGAGCCGGAA ATCCTGGCGT CGCTTGATGA ACTGATAGGG CGCTGGGCGA 100
<210> 11418
<211> 100
<212> DNA
<213> Escherichia coli K-12 MG1655
<220> cysj b2765
<222> 1682-1781
<223> cysj b2765 U00096 complement(2888122..2889921)
<313> 11418: 1742-1761

<400> 11418
ACGTTGAGCA GGCACTTCTG GAAGTGATTG CCGAATTTGG TGGCATGGAC 50
ACCGAAGCGG CGGATGAATT TTTAAGTGAG CTGCGCGTAG AGCGCCGTTA 100
<210> 11419
<211> 100
<212> DNA
<213> Escherichia coli K-12 MG1655
<220> cysj b2765
<222> 1631-1730
<223> cysj b2765 U00096 complement(2888122..2889921)
<313> 11419: 1681-1700
<400> 11419
ATGATGGTGC CCACATTTAT GTCTGCGGCG ACGCTAATCG CATGGCGAAA 50
GACGTTGAGC AGGCACTTCT GGAAGTGATT GCCGAATTTG GTGGCATGGA 100
<210> 11420
<211> 100
<212> DNA
<213> Escherichia coli K-12 MG1655
<220> cysj b2765
<222> 1599-1698
<223> cysj b2765 U00096 complement(2888122..2889921)
<313> 11420: 1628-1647
<400> 11420
CGAACAGGGC GCGGAGCTGT GGCGCTGGAT CAATGATGGT GCCCACATTT 50
ATGTCTGCGG CGACGCTAAT CGCATGGCGA AAGACGTTGA GCAGGCACTT 100
<210> 11421
<211> 100
<212> DNA
<213> Escherichia coli K-12 MG1655
<220> cysk b2415
<222> 873-972
<223> cysk b2415 U00096 2530429..2531400
<313> 11421: 935-954
<400> 11421
CAACAAGAAT ATTGTGGTTA TTCTACCATC ATCGGGTGAG CGTTATTTAA 50
GCACCGCATT GTTTGCCGAT CTCTTCACTG AGAAAGAATT GCAACAGTAA 100
<210> 11422
<211> 100
<212> DNA
<213> Escherichia coli K-12 MG1655
<220> cysk b2415
<222> 675-774
<223> cysk b2415 U00096 2530429..2531400
<313> 11422: 734-753
<400> 11422
TAAAATTCAG GGTATTGGCG CTGGTTTTAT CCCGGCTAAC CTCGATCTCA 50
AGCTGGTCGA TAAAGTCATT GGCATCACCA ATGAAGAAGC GATTTCTACC 100
<210> 11423
<211> 100
<212> DNA
<213> Escherichia coli K-12 MG1655
<220> cysk b2415
<222> 603-702
<223> cysk b2415 U00096 2530429..2531400
<313> 11423: 667-686
<400> 11423
CGCCGTTGAG CCAACCGATT CTCCAGTTAT CGCCCAGGCG CTGGCAGGTG 50
AAGAGATTAA ACCTGGCCCG CATAAAATTC AGGGTATTGG CGCTGGTTTT 100
<210> 11424
<211> 100
<212> DNA
<213> Escherichia coli K-12 MG1655
<220> cysm b2422
<222> 813-912
<223> cysm b2422 U00096 complement(2536692..2537603)
<313> 11424: 879-898
<400> 11424
TGACGCGGTG GTGGTGGCGA TCATCTGCGA TCGTGGCGAT CGCTACCTTT 50
CTACCGGGGT GTTTGGGGAA GAGCATTTTA GCCAGGGGGC GGGGATTTAA 100
<210> 11425
<211> 100

<212> DNA
<213> Escherichia coli K-12 MG1655
<220> cysm b2422
<222> 766-865
<223> cysm b2422 U00096 complement(2536692..2537603)
<313> 11425: 822-841
<400> 11425
TCCGGCGGCG CGGTTGCCGG AGCACTGCGG GTGGCAAAAG CTAACCCTGA 50
CGCGGTGGTG GTGGCGATCA TCTGCGATCG TGGCGATCGC TACCTTTCTA 100
<210> 11426
<211> 100
<212> DNA
<213> Escherichia coli K-12 MG1655
<220> cysm b2422
<222> 685-784
<223> cysm b2422 U00096 complement(2536692..2537603)
<313> 11426: 725-744
<400> 11426
GTGCTGGATA TTCATCAGCG CGATGCGGAA AACACCATGC GCGAACTGGC 50
GGTGCGGGAA GGAATATTCT GTGGCGTCAG CTCCGGCGGC GCGGTTGCCG 100
<210> 11427
<211> 100
<212> DNA
<213> Escherichia coli K-12 MG1655
<220> cysn b2752
<222> 1329-1428
<223> cysn b2752 U00096 complement(2872015..2873442)
<313> 11427: 1364-1383
<400> 11427
TGCGCCATCT GAATTCAGTG CATTCGAACT GGAATTGAAT GCTCTGGTTC 50
GTCGCCACTT TCCGCACTGG GGCGCGCGCG ATTTGCTGGG GGATAAATAA 100
<210> 11428
<211> 100
<212> DNA
<213> Escherichia coli K-12 MG1655
<220> cysn b2752
<222> 1264-1363
<223> cysn b2752 U00096 complement(2872015..2873442)
<313> 11428: 1311-1330
<400> 11428
GATCGCCTGA GCAATGTGAC CGTGGGTGCC GGTATGGTGC ACGAGCCAGT 50
TAGCCAGGCA ACTGCTGCGC CATCTGAATT CAGTGCATTC GAACTGGAAT 100
<210> 11429
<211> 100
<212> DNA
<213> Escherichia coli K-12 MG1655
<220> cysn b2752
<222> 1114-1213
<223> cysn b2752 U00096 complement(2872015..2873442)
<313> 11429: 1160-1179
<400> 11429
CGCTATCAGG TTGATATTAA TAACCTTACC CAGCGTGAAG TTGAAAACCT 50
GCCACTGAAT GGGATCGGCC TCGTGGATCT CACTTTTGAC GAGCCGCTGG 100
<210> 11430
<211> 100
<212> DNA
<213> Escherichia coli K-12 MG1655
<220> cysp b2426
<222> 918-1017
<223> cysp b2426 U00096 complement(2540532..2541548)
<313> 11430: 980-999
<400> 11430
CCGCGTGGAA GACAAATTTG GCTCCTGGCC GGAAGTGATG AAAACCCACT 50
TCACCAGCGG CGGCGAGTTA GACAAGCTGT TAGCGGCGGG GCGTAACTGA 100
<210> 11431
<211> 100
<212> DNA
<213> Escherichia coli K-12 MG1655
<220> cysp b2426
<222> 852-951
<223> cysp b2426 U00096 complement(2540532..2541548)

<313> 11431: 894-913
<400> 11431
CTACCGCGTG AATAACCCGG AGGTGATGGA CAAACTGAAA GACAAATTCC 50
CGCAGACCGA GCTGTTCCGC GTGGAAGACA AATTTGGCTC CTGGCCGGAA 100
<210> 11432
<211> 100
<212> DNA
<213> Escherichia coli K-12 MG1655
<220> cysp b2426
<222> 824-923
<223> cysp b2426 U00096 complement(2540532..2541548)
<313> 11432: 843-862
<400> 11432
AGGCGCAAAC CATCATCACC GACTATTACT ACCGCGTGAA TAACCCGGAG 50
GTGATGGACA AACTGAAAGA CAAATTCCCG CAGACCGAGC TGTTCCGCGT 100
<210> 11433
<211> 100
<212> DNA
<213> Escherichia coli K-12 MG1655
<220> cysq b4215
<222> 642-741
<223> cysq b4215 U00096 4434333..4435073
<313> 11433: 686-705
<400> 11433
AGCTGCCGGA GCGCACGTTC ACGACTGGCA GGGTAAACCG CTGGATTACA 50
CTCCGCGTGA GTCGTTCCTG AATCCGGGGT TCAGAGTGTC TATTTACTAA 100
<210> 11434
<211> 100
<212> DNA
<213> Escherichia coli K-12 MG1655
<220> cysq b4215
<222> 485-584
<223> cysq b4215 U00096 4434333..4435073
<313> 11434: 535-554
<400> 11434
AGCTGAAAGA GTATCTGCAA CAGCTTGGCG AACATCAGAC CACGTCCATC 50
GGCTCTTCGC TGAAATTCTG CCTGGTGGCG GAAGGACAGG CGCAGCTGTA 100
<210> 11435
<211> 100
<212> DNA
<213> Escherichia coli K-12 MG1655
<220> cysq b4215
<222> 431-530
<223> cysq b4215 U00096 4434333..4435073
<313> 11435: 487-506
<400> 11435
TCCGCGATGC GCGCCCGCCG CTGGTGGTGA TCAGCCGTTC CCATGCGGAT 50
GCGGAGCTGA AAGAGTATCT GCAACAGCTT GGCGAACATC AGACCACGTC 100
<210> 11436
<211> 100
<212> DNA
<213> Escherichia coli K-12 MG1655
<220> cyss b0527
<222> 1287-1386
<223> cyss b0527 U00096 553834..555219
<313> 11436: 1348-1367
<400> 11436
GAAAGACTGG GCGGCGGCGG ATGCGGCGCG TGATCGTCTT AACGAGATGG 50
GGATCGTGCT GGAAGATGGC CCGCAAGGGA CCACCTGGCG TCGTAAGTAA 100
<210> 11437
<211> 100
<212> DNA
<213> Escherichia coli K-12 MG1655
<220> cyss b0527
<222> 1284-1383
<223> cyss b0527 U00096 553834..555219
<313> 11437: 1304-1323
<400> 11437
AGCGAAAGAC TGGGCGGCGG CGGATGCGGC GCGTGATCGT CTTAACGAGA 50
TGGGGATCGT GCTGGAAGAT GGCCCGCAAG GGACCACCTG GCGTCGTAAG 100
<210> 11438

<211> 100
<212> DNA
<213> Escherichia coli K-12 MG1655
<220> cyss b0527
<222> 1198-1297
<223> cyss b0527 U00096 553834..555219
<313> 11438: 1250-1269
<400> 11438
GCGTTCCTGC AAAGCGGCGC GCAGGCAGAC GACAGCGAAG TGGCTGAGAT 50
TGAAGCGTTA ATTCAACAGC GTCTGGATGC CCGTAAAGCG AAAGACTGGG 100
<210> 11439
<211> 100
<212> DNA
<213> Escherichia coli K-12 MG1655
<220> msba b0915
<222> 1640-1739
<223> msba b0915 U00096 965844..967592
<313> 11439: 1668-1687
<400> 11439
AAATCGTGGT CGTCGAGGAT GGTGTCATTG TGGAACGCGG TACGCATAAC 50
GATTTGCTTG AGCACCGCGG CGTTTACGCG CAACTTCACA AAATGCAGTT 100
<210> 11440
<211> 100
<212> DNA
<213> Escherichia coli K-12 MG1655
<220> msba b0915
<222> 1600-1699
<223> msba b0915 U00096 965844..967592
<313> 11440: 1624-1643
<400> 11440
GTGATTGCCC ACCGCTTGTC TACCATTGAA AAGGCAGACG AAATCGTGGT 50
CGTCGAGGAT GGTGTCATTG TGGAACGCGG TACGCATAAC GATTTGCTTG 100
<210> 11441
<211> 100
<212> DNA
<213> Escherichia coli K-12 MG1655
<220> msba b0915
<222> 1523-1622
<223> msba b0915 U00096 965844..967592
<313> 11441: 1570-1589
<400> 11441
CCTCGGCTCT GGATACCGAA TCCGAACGTG CGATTCAGGC GGCACTGGAT 50
GAGTTGCAGA AAAACCGTAC CTCTCTGGTG ATTGCCCACC GCTTGTCTAC 100
<210> 11442
<211> 100
<212> DNA
<213> Escherichia coli K-12 MG1655
<220> msbb b1856
<222> 868-967
<223> msbb b1856 U00096 complement(1937246..1938217)
<313> 11442: 925-944
<400> 11442
GGTCCGCGAC CAGAACAATA CACCTGGATA CTAAAATTGC TGAAAACTCG 50
CAAACCGGGC GAAATCCAGC CGTATAAGCG CAAAGATCTT TATCCCATCA 100
<210> 11443
<211> 100
<212> DNA
<213> Escherichia coli K-12 MG1655
<220> msbb b1856
<222> 840-939
<223> msbb b1856 U00096 complement(1937246..1938217)
<313> 11443: 873-892
<400> 11443
GATGAATGAA GAAGTCGAGA TTTTTGTTGG TCCGCGACCA GAACAATACA 50
CCTGGATACT AAAATTGCTG AAAACTCGCA AACCGGGCGA AATCCAGCCG 100
<210> 11444
<211> 100
<212> DNA
<213> Escherichia coli K-12 MG1655
<220> msbb b1856
<222> 814-913

<223> msbb b1856 U00096 complement(1937246..1938217)
<313> 11444: 849-868
<400> 11444
GCGGATGATC ATACGATTGC GCGGCGGATG AATGAAGAAG TCGAGATTTT 50
TGTTGGTCCG CGACCAGAAC AATACACCTG GATACTAAAA TTGCTGAAAA 100
<210> 11445
<211> 100
<212> DNA
<213> Escherichia coli K-12 MG1655
<220> cysu b2425
<222> 735-834
<223> cysu b2425 U00096 complement(2539699..2540532)
<313> 11445: 809-828
<400> 11445
GAGCGCGATT GCTTCGGTGA TCCTCGCGGC ATCTCTGCTG CTGCTGTTCT 50
CAATTAACAC TCTGCAAAGT CGCTTTGGTC GGCGTGTGGT AGGTCATTAA 100
<210> 11446
<211> 100
<212> DNA
<213> Escherichia coli K-12 MG1655
<220> cysu b2425
<222> 679-778
<223> cysu b2425 U00096 complement(2539699..2540532)
<313> 11446: 713-732
<400> 11446
GAAGTGACGT CGCTGATGAT TTTTGTGCGC TTACAGGAGT TTGATTACCC 50
GGCAGCGAGC GCGATTGCTT CGGTGATCCT CGCGGCATCT CTGCTGCTGC 100
<210> 11447
<211> 100
<212> DNA
<213> Escherichia coli K-12 MG1655
<220> cysu b2425
<222> 566-665
<223> cysu b2425 U00096 complement(2539699..2540532)
<313> 11447: 630-649
<400> 11447
TGCTGCCGGA GCTTTCTCCG GCGCTGGTGG CGGGCGTGGC GCTGTCGTTT 50
ACCCGTAGTC TTGGTGAATT TGGCGCGGTG ATTTTTATCG CCGGAAATAT 100
<210> 11448
<211> 100
<212> DNA
<213> Escherichia coli K-12 MG1655
<220> cysw b2424
<222> 351-450
<223> cysw b2424 U00096 complement(2538824..2539273)
<313> 11448: 420-439
<400> 11448
GCTGATGGCG ATTATCACCC TGTTTTTAAA AAGTATGTTG CAGTGGCGCC 50
TGGAGAATCA GGAAAAACGC GCACAGCAGG AGGAACATCA TGAGCATTGA 100
<210> 11449
<211> 100
<212> DNA
<213> Escherichia coli K-12 MG1655
<220> cysw b2424
<222> 274-373
<223> cysw b2424 U00096 complement(2538824..2539273)
<313> 11449: 314-333
<400> 11449
TCGCTGCCGT TACAGATTGA ATTGCTGGAG CAGGACTACA ACACCGTCGG 50
CTCCTTTACC GCTGCGGCGC TGTTAACGCT GATGGCGATT ATCACCCTGT 100
<210> 11450
<211> 100
<212> DNA
<213> Escherichia coli K-12 MG1655
<220> cysw b2424
<222> 126-225
<223> cysw b2424 U00096 complement(2538824..2539273)
<313> 11450: 182-201
<400> 11450
CGCGTCCGGC TGGCAGATGT TCCGTCGCGT CACATTACCG AACATCCGCT 50
GGGCGCTGCT TTATGGCGTG GTGTTGACCA ACGCCCGCGC AATTGGCGAG 100

<210> 11451
<211> 100
<212> DNA
<213> Escherichia coli K-12 MG1655
<220> cysz b2414
<222> 652-751
<223> cysz b2414 U00096 2529483..2530244
<313> 11451: 684-703
<400> 11451
CTGTTTACGA TGATCCCGCT GCTTAATCTG TTCATCATGC CCGTTGCCGT 50
TTGTGGCGCG ACGGCGATGT GGGTCGATTG CTATCGCGAT AAACACGCGA 100
<210> 11452
<211> 100
<212> DNA
<213> Escherichia coli K-12 MG1655
<220> cysz b2414
<222> 611-710
<223> cysz b2414 U00096 2529483..2530244
<313> 11452: 634-653
<400> 11452
CACGCAAAAT CACCAATATG CAGTTTGGTG CTTTAACCAG CCTGTTTACG 50
ATGATCCCGC TGCTTAATCT GTTCATCATG CCCGTTGCCG TTTGTGGCGC 100
<210> 11453
<211> 100
<212> DNA
<213> Escherichia coli K-12 MG1655
<220> cysz b2414
<222> 519-618
<223> cysz b2414 U00096 2529483..2530244
<313> 11453: 574-593
<400> 11453
GTTTAGCGCC TGGATGTTAG CCATCCAGTA TTGCGATTAC CCCTTCGATA 50
ACCACAAAGT GCCGTTTAAA GAGATGCGCA CCGCCCTGCG CACACGCAAA 100
<210> 11454
<211> 100
<212> DNA
<213> Escherichia coli K-12 MG1655
<220> b1240 b1241
<222> 129-228
<223> b1240 b1241 U00096 complement(1294191..1294421)
<313> 11454: 183-202
<400> 11454
GGCCCGCAGC GCTGTCACCC AGGCGCGTCA GCGCGTGGGG GCCGCCCCAG 50
TGGAATGGCT CTTCCGCCAG ACCGCACAGA CAGGGGCGCG GAACGTTACC 100
<210> 11455
<211> 100
<212> DNA
<213> Escherichia coli K-12 MG1655
<220> b1240 b1241
<222> 58-157
<223> b1240 b1241 U00096 complement(1294191..1294421)
<313> 11455: 103-122
<400> 11455
AATGAGCCAA TTACCGATGT TGTTCGCCGT CTGAACCTGA GCGCGGATGG 50
CGAAGCGGGG ATGAACCTGC TGGCCCGCAG CGCTGTCACC CAGGCGCGTC 100
<210> 11456
<211> 100
<212> DNA
<213> Escherichia coli K-12 MG1655
<220> b1240 b1241
<222> 26-125
<223> b1240 b1241 U00096 complement(1294191..1294421)
<313> 11456: 51-70
<400> 11456
ACCGGGGGAC ATGGTTATCT GGATGGTGGT GCAATGAGCC AATTACCGAT 50
GTTGTTCGCC GTCTGAACCT GAGCGCGGAT GGCGAAGCGG GGATGAACCT 100
<210> 11457
<211> 100
<212> DNA
<213> Escherichia coli K-12 MG1655
<220> hrsa b0732

<222> 1878-1977
<223> hrsa b0732 U00096 765207..767183
<313> 11457: 1941-1960
<400> 11457
GGCATTGGTG GGGGCTGCAA TCTCGACTGC AATTCTCCTG ATGTGGCGGC  50
GTCACGCGGT TAAGCATGGC AACTATCTGA CTGATGGCGT AATGCCATAA  100
<210> 11458
<211> 100
<212> DNA
<213> Escherichia coli K-12 MG1655
<220> hrsa b0732
<222> 1853-1952
<223> hrsa b0732 U00096 765207..767183
<313> 11458: 1895-1914
<400> 11458
TGGCGGCAAT TGGCTGGTTT GGCGCGGCAT TGGTGGGGGC TGCAATCTCG  50
ACTGCAATTC TCCTGATGTG GCGGCGTCAC GCGGTTAAGC ATGGCAACTA  100
<210> 11459
<211> 100
<212> DNA
<213> Escherichia coli K-12 MG1655
<220> hrsa b0732
<222> 1790-1889
<223> hrsa b0732 U00096 765207..767183
<313> 11459: 1816-1835
<400> 11459
CGACACCCGG TGCCGGCATT TTCTCGCTCT TTTTACTTCA TGATAATGGC  50
GCGGGCGGTG TTATGGCGGC AATTGGCTGG TTTGGCGCGG CATTGGTGGG  100
<210> 11460
<211> 100
<212> DNA
<213> Escherichia coli K-12 MG1655
<220> ynha ynhA
<222> 318-417
<223> ynha ynhA U00096 complement(1756898..1757314)
<313> 11460: 386-405
<400> 11460
TGAAAAAATG GCGCTCACCC AACATCTCAC CCCATCTCGT TCACAAGGTC  50
TGGAAGCGAT GATTCGCGCA ATTCGCGCCA AAGCCGCTGC ACTTAGCTAA  100
<210> 11461
<211> 100
<212> DNA
<213> Escherichia coli K-12 MG1655
<220> ynha ynhA
<222> 294-393
<223> ynha ynhA U00096 complement(1756898..1757314)
<313> 11461: 351-370
<400> 11461
CAATTTCGAT GTGCGTCCGT GGTTTGAAAA AATGGCGCTC ACCCAACATC  50
TCACCCCATC TCGTTCACAA GGTCTGGAAG CGATGATTCG CGCAATTCGC  100
<210> 11462
<211> 100
<212> DNA
<213> Escherichia coli K-12 MG1655
<220> ynha ynhA
<222> 248-347
<223> ynha ynhA U00096 complement(1756898..1757314)
<313> 11462: 285-304
<400> 11462
CGGTCGTCTT TATTCTCTAC GATCAGATGA CGCCGCAGGA TATTGTCAAT  50
TTCGATGTGC GTCCGTGGTT TGAAAAAATG GCGCTCACCC AACATCTCAC  100
<210> 11463
<211> 100
<212> DNA
<213> Escherichia coli K-12 MG1655
<220> b1248 b1249
<222> 309-408
<223> b1248 b1249 U00096 complement(1304845..1305252)
<313> 11463: 383-402
<400> 11463
GGCGGACAGT GAAGATGGTG AAATCAACGA TGTTTTTGCC CGCATTCTGT  50

TATGCCGCGA AAAAGATCAC AAACTTTGCC ATATCATCTG GCGGGAATAA  100
<210> 11464
<211> 100
<212> DNA
<213> Escherichia coli K-12 MG1655
<220> b1248 b1249
<222> 297-396
<223> b1248 b1249 U00096 complement(1304845..1305252)
<313> 11464: 341-360
<400> 11464
GGTGATTGGT CTGGCGGACA GTGAAGATGG TGAAATCAAC GATGTTTTTG  50
CCCGCATTCT GTTATGCCGC GAAAAAGATC ACAAACTTTG CCATATCATC  100
<210> 11465
<211> 100
<212> DNA
<213> Escherichia coli K-12 MG1655
<220> b1248 b1249
<222> 271-370
<223> b1248 b1249 U00096 complement(1304845..1305252)
<313> 11465: 293-312
<400> 11465
TTGAACCCGG ACTTTTTTGC CGAAGTGGTG ATTGGTCTGG CGGACAGTGA  50
AGATGGTGAA ATCAACGATG TTTTTGCCCG CATTCTGTTA TGCCGCGAAA  100
<210> 11466
<211> 100
<212> DNA
<213> Escherichia coli K-12 MG1655
<220> ynhc b1682
<222> 1173-1272
<223> ynhc b1682 U00096 complement(1758544..1759815)
<313> 11466: 1252-1271
<400> 11466
CGCCTTCGCT GCCGAACTGA CGGAAGCACT GCGTGATGAG GGGCTTAAAC  50
AGCAGGTGCT GGCCCGAATC GGTCAACGGC TGCCAGGAGG TGCAAGATGA  100
<210> 11467
<211> 100
<212> DNA
<213> Escherichia coli K-12 MG1655
<220> ynhc b1682
<222> 1160-1259
<223> ynhc b1682 U00096 complement(1758544..1759815)
<313> 11467: 1196-1215
<400> 11467
AGATGATCAT TTACGCCTTC GCTGCCGAAC TGACGGAAGC ACTGCGTGAT  50
GAGGGGCTTA AACAGCAGGT GCTGGCCCGA ATCGGTCAAC GGCTGCCAGG  100
<210> 11468
<211> 100
<212> DNA
<213> Escherichia coli K-12 MG1655
<220> ynhc b1682
<222> 1041-1140
<223> ynhc b1682 U00096 complement(1758544..1759815)
<313> 11468: 1096-1115
<400> 11468
GCAGCTGGAA ATCTATGCAG ATGATGTGAA ATGCAGCCAC GGCGCGACGG  50
TGGGGCGTAT TGATGATGAA CAGATATTCT ATCTGCGCTC GCGCGGGATC  100
<210> 11469
<211> 100
<212> DNA
<213> Escherichia coli K-12 MG1655
<220> ynhd b1683
<222> 607-706
<223> ynhd b1683 U00096 complement(1759790..1760536)
<313> 11469: 650-669
<400> 11469
CACTACCAAC GCATTCTCGA CTACATCAAG CCTGATTACG TTCATGTGCT  50
ATATCAGGGA CGAATTGTGA AATCCGGCGA TTTCACGTTG GTCAAACAAC  100
<210> 11470
<211> 100
<212> DNA
<213> Escherichia coli K-12 MG1655

<220> ynhd b1683
<222> 559-658
<223> ynhd b1683 U00096 complement(1759790..1760536)
<313> 11470: 608-627
<400> 11470
GGCGTGAACT CGCTGCGTGA TGGCAAGCGC TCATTCATCA TTGTTACGCA 50
CTACCAACGC ATTCTCGACT ACATCAAGCC TGATTACGTT CATGTGCTAT 100
<210> 11471
<211> 100
<212> DNA
<213> Escherichia coli K-12 MG1655
<220> ynhd b1683
<222> 494-593
<223> ynhd b1683 U00096 complement(1759790..1760536)
<313> 11471: 513-532
<400> 11471
AGTTATGCAT TCTTGATGAG TCGGACTCCG GGCTGGATAT TGACGCATTA 50
AAAGTGGTCG CCGATGGCGT GAACTCGCTG CGTGATGGCA AGCGCTCATT 100
<210> 11472
<211> 100
<212> DNA
<213> Escherichia coli K-12 MG1655
<220> mdaa b0852
<222> 624-723
<223> mdaa b0852 U00096 890407..891129
<313> 11472: 689-708
<400> 11472
CCGGGATACC TGGAGCGATC ATATCCGCCG AACAATCATT AAAGAAAGCC 50
GCCCATTTAT TCTGGATTAT TTGCACAAAC AGGGTTGGGC GACGCGCTAA 100
<210> 11473
<211> 100
<212> DNA
<213> Escherichia coli K-12 MG1655
<220> mdaa b0852
<222> 598-697
<223> mdaa b0852 U00096 890407..891129
<313> 11473: 651-670
<400> 11473
TACCTCACCC GTGGCAGCAA TAATCGCCGG GATACCTGGA GCGATCATAT 50
CCGCCGAACA ATCATTAAAG AAAGCCGCCC ATTTATTCTG GATTATTTGC 100
<210> 11474
<211> 100
<212> DNA
<213> Escherichia coli K-12 MG1655
<220> mdaa b0852
<222> 590-689
<223> mdaa b0852 U00096 890407..891129
<313> 11474: 608-627
<400> 11474
CGGAATATTA CCTCACCCGT GGCAGCAATA ATCGCCGGGA TACCTGGAGC 50
GATCATATCC GCCGAACAAT CATTAAAGAA AGCCGCCCAT TTATTCTGGA 100
<210> 11475
<211> 100
<212> DNA
<213> Escherichia coli K-12 MG1655
<220> ynhe b1684
<222> 1428-1527
<223> ynhe b1684 U00096 complement(1760546..1762072)
<313> 11475: 1507-1526
<400> 11475
TAACGGTTTC TGCAAAGACG TGTTCTCGGA GCTGCCGTTG GAATTTGCCG 50
TTGAAGCACA AAAACTCCTC GCCATCAGTC TTGAACACAG CGTCGGATAA 100
<210> 11476
<211> 100
<212> DNA
<213> Escherichia coli K-12 MG1655
<220> ynhe b1684
<222> 1419-1518
<223> ynhe b1684 U00096 complement(1760546..1762072)
<313> 11476: 1466-1485
<400> 11476

GATGATTGTT AACGGTTTCT GCAAAGACGT GTTCTCGGAG CTGCCGTTGG 50
AATTTGCCGT TGAAGCACAA AAACTCCTCG CCATCAGTCT TGAACACAGC 100
<210> 11477
<211> 100
<212> DNA
<213> Escherichia coli K-12 MG1655
<220> ynhe b1684
<222> 1370-1469
<223> ynhe b1684 U00096 complement(1760546..1762072)
<313> 11477: 1394-1413
<400> 11477
AACTGTTTTA CTGCCTGCAA CGCGGGATCA GCGAAGAAGA CGCCATCTCG 50
ATGATTGTTA ACGGTTTCTG CAAAGACGTG TTCTCGGAGC TGCCGTTGGA 100
<210> 11478
<211> 100
<212> DNA
<213> Escherichia coli K-12 MG1655
<220> nada b0751
<222> 933-1032
<223> nada b0751 U00096 781308..782351
<313> 11478: 977-996
<400> 11478
ATTAGAACAG GAAGGAAGCA ATCACGAGGT TCATGTTGAT GAAAGGCTGC 50
GAGAGAGGGC GCTGGTGCCG CTCAATCGTA TGCTGGATTT TGCGGCTACA 100
<210> 11479
<211> 100
<212> DNA
<213> Escherichia coli K-12 MG1655
<220> nada b0751
<222> 898-997
<223> nada b0751 U00096 781308..782351
<313> 11479: 921-940
<400> 11479
ATGGCCATGA ATGGCCTTCA GGCCATCGCA GAGGCATTAG AACAGGAAGG 50
AAGCAATCAC GAGGTTCATG TTGATGAAAG GCTGCGAGAG AGGGCGCTGG 100
<210> 11480
<211> 100
<212> DNA
<213> Escherichia coli K-12 MG1655
<220> nada b0751
<222> 820-919
<223> nada b0751 U00096 781308..782351
<313> 11480: 841-860
<400> 11480
GTGCCAGATA AAGAGTTACT GGAAGCACCA ACCGCAGGTG AGGGTGCAAC 50
CTGCCGCAGC TGCGCGCATT GTCCGTGGAT GGCCATGAAT GGCCTTCAGG 100
<210> 11481
<211> 100
<212> DNA
<213> Escherichia coli K-12 MG1655
<220> mdab b3029
<222> 457-556
<223> mdab b3029 U00096 3170546..3171127
<313> 11481: 520-539
<400> 11481
AAAGCAAACC AATTCCTCGG TATGGAACCG CTGCCGACAT TTATCGCTAA 50
TGACGTGATA AAAATGCCTG ATGTTCCCCG CTATACTGAA GAATATCGCA 100
<210> 11482
<211> 100
<212> DNA
<213> Escherichia coli K-12 MG1655
<220> mdab b3029
<222> 448-547
<223> mdab b3029 U00096 3170546..3171127
<313> 11482: 462-481
<400> 11482
CCGTTCCATA AAGCAAACCA ATTCCTCGGT ATGGAACCGC TGCCGACATT 50
TATCGCTAAT GACGTGATAA AAATGCCTGA TGTTCCCCGC TATACTGAAG 100
<210> 11483
<211> 100
<212> DNA

<213> Escherichia coli K-12 MG1655
<220> mdab b3029
<222> 356-455
<223> mdab b3029 U00096 3170546..3171127
<313> 11483: 396-415
<400> 11483
ATATGCTTTC TCTGACCTGG AACGCACCAA TGGAAGCCTT CACCGAAAAA 50
GATCAGTTCT TCCACGGCGT TGGCGTTGAC GGTGTGTATC TGCCGTTCCA 100
<210> 11484
<211> 100
<212> DNA
<213> Escherichia coli K-12 MG1655
<220> nadb b2575
<222> 1524-1623
<223> nadb b2575 U00096 2708440..2710062
<313> 11484: 1587-1606
<400> 11484
AGAGAGTCGG GGGTTGCATT TCACGCTGGA TTATCCGGAA CTGCTCACCC 50
ATTCCGGTCC GTCGATCCTT TCCCCCGGCA ATCATTACAT AAACAGATAA 100
<210> 11485
<211> 100
<212> DNA
<213> Escherichia coli K-12 MG1655
<220> nadb b2575
<222> 1487-1586
<223> nadb b2575 U00096 2708440..2710062
<313> 11485: 1530-1549
<400> 11485
TTGCCGAGTT GATTGTTCGC TGTGCAATGA TGCGTAAAGA GAGTCGGGGG 50
TTGCATTTCA CGCTGGATTA TCCGGAACTG CTCACCCATT CCGGTCCGTC 100
<210> 11486
<211> 100
<212> DNA
<213> Escherichia coli K-12 MG1655
<220> nadb b2575
<222> 1468-1567
<223> nadb b2575 U00096 2708440..2710062
<313> 11486: 1494-1513
<400> 11486
CTGCGTAATC TGGTACAGGT TGCCGAGTTG ATTGTTCGCT GTGCAATGAT 50
GCGTAAAGAG AGTCGGGGGT TGCATTTCAC GCTGGATTAT CCGGAACTGC 100
<210> 11487
<211> 100
<212> DNA
<213> Escherichia coli K-12 MG1655
<220> ynhg b1679
<222> 906-1005
<223> ynhg b1679 U00096 complement(1755745..1756749)
<313> 11487: 968-987
<400> 11487
GAGCAGTGGA GCAACTCCCG CAGCCAGCAA TGCGCCTTCA GTAGAGTCAG 50
CGCAGAATGG TGAACCAGAG CAAGGGAATA TGTTACGCGT GACGCAGTAG 100
<210> 11488
<211> 100
<212> DNA
<213> Escherichia coli K-12 MG1655
<220> ynhg b1679
<222> 898-997
<223> ynhg b1679 U00096 complement(1755745..1756749)
<313> 11488: 921-940
<400> 11488
GTTTCGGTGA GCAGTGGAGC AACTCCCGCA GCCAGCAATG CGCCTTCAGT 50
AGAGTCAGCG CAGAATGGTG AACCAGAGCA AGGGAATATG TTACGCGTGA 100
<210> 11489
<211> 100
<212> DNA
<213> Escherichia coli K-12 MG1655
<220> ynhg b1679
<222> 806-905
<223> ynhg b1679 U00096 complement(1755745..1756749)
<313> 11489: 860-879

<400> 11489
CACTGCCAGC AGGCTTTACG CAATTTAAAG ACAATAAGGC TGTAGATCAG 50
AAGTTAGTCG ATAAAGCGTT GTATCGTCGG GCAGGGTATC CGGTTTCGGT 100
<210> 11490
<211> 100
<212> DNA
<213> Escherichia coli K-12 MG1655
<220> nadc b0110
<222> 795-894
<223> nadc b0110 U00096 complement(117752..118645)
<313> 11490: 858-877
<400> 11490
AACACTGCGT GAATTTGCCG AAACGGGCGT GGACTTTATC TCCGTCGGTG 50
CGCTAACTAA ACACGTACAA GCACTCGACC TTTCAATGCG TTTTCGCTAA 100
<210> 11491
<211> 100
<212> DNA
<213> Escherichia coli K-12 MG1655
<220> nadc b0110
<222> 722-821
<223> nadc b0110 U00096 complement(117752..118645)
<313> 11491: 776-795
<400> 11491
AACAGATGCG CGAAGCCGTC AAACGCACCA ACGGCAAGGC GCTACTGGAA 50
GTGTCTGGCA ACGTCACTGA CAAAACACTG CGTGAATTTG CCGAAACGGG 100
<210> 11492
<211> 100
<212> DNA
<213> Escherichia coli K-12 MG1655
<220> nadc b0110
<222> 703-802
<223> nadc b0110 U00096 complement(117752..118645)
<313> 11492: 732-751
<400> 11492
CTGGATAACT TCGAAACAGA ACAGATGCGC GAAGCCGTCA AACGCACCAA 50
CGGCAAGGCG CTACTGGAAG TGTCTGGCAA CGTCACTGAC AAAACACTGC 100
<210> 11493
<211> 100
<212> DNA
<213> Escherichia coli K-12 MG1655
<220> nade nadE
<222> 729-828
<223> nade nadE U00096 1820482..1821309
<313> 11493: 792-811
<400> 11493
ACCTCAACAG GTCGCCAGAA CAATAGAGAA CTGGTATCTG AAAACCGAAC 50
ATAAACGCCG TCCGCCAATT ACCGTTTTCG ATGATTTCTG GAAAAAGTAA 100
<210> 11494
<211> 100
<212> DNA
<213> Escherichia coli K-12 MG1655
<220> nade nadE
<222> 707-806
<223> nade nadE U00096 1820482..1821309
<313> 11494: 738-757
<400> 11494
ACTATCTGGA AGGGAAAAAC GTACCTCAAC AGGTCGCCAG AACAATAGAG 50
AACTGGTATC TGAAAACCGA ACATAAACGC CGTCCGCCAA TTACCGTTTT 100
<210> 11495
<211> 100
<212> DNA
<213> Escherichia coli K-12 MG1655
<220> nade nadE
<222> 665-764
<223> nade nadE U00096 1820482..1821309
<313> 11495: 700-719
<400> 11495
CAGATGAAGT GGCACTCGGC GTGACCTATG ACAATATCGA CGACTATCTG 50
GAAGGGAAAA ACGTACCTCA ACAGGTCGCC AGAACAATAG AGAACTGGTA 100
<210> 11496
<211> 100

<212> DNA
<213> Escherichia coli K-12 MG1655
<220> crca b0623
<222> 462-561
<223> crca b0623 U00096 655780..656340
<313> 11496: 529-548
<400> 11496
CTCCGTGGGT TATGGCCCAG TGACTTTTCA GATGACCTAC ATTCCGGGTA 50
CCTACAACAA TGGCAATGTG TACTTTGCCT GGATGCGCTT TCAGTTTTGA 100
<210> 11497
<211> 100
<212> DNA
<213> Escherichia coli K-12 MG1655
<220> crca b0623
<222> 462-561
<223> crca b0623 U00096 655780..656340
<313> 11497: 474-493
<400> 11497
CTCCGTGGGT TATGGCCCAG TGACTTTTCA GATGACCTAC ATTCCGGGTA 50
CCTACAACAA TGGCAATGTG TACTTTGCCT GGATGCGCTT TCAGTTTTGA 100
<210> 11498
<211> 100
<212> DNA
<213> Escherichia coli K-12 MG1655
<220> crca b0623
<222> 381-480
<223> crca b0623 U00096 655780..656340
<313> 11498: 447-466
<400> 11498
TTTAGGTCTG GGATTCACCG CTGGCGTAAC GGCACGCGAT AACTGGAATT 50
ACATCCCTCT CCCGGTTCTA CTGCCATTGG CCTCCGTGGG TTATGGCCCA 100
<210> 11499
<211> 100
<212> DNA
<213> Escherichia coli K-12 MG1655
<220> crcb b0625
<222> 285-384
<223> crcb b0625 U00096 complement(656778..657161)
<313> 11499: 348-367
<400> 11499
CTTTGGCTGG GCATTACTGA ACGTTTTCGT CAACCTTCTG GGGTCTTTTG 50
CCATGACCGC ACTGGCATTC TGGCTGTTTT CGGCCTCAAC CGCACACTAA 100
<210> 11500
<211> 100
<212> DNA
<213> Escherichia coli K-12 MG1655
<220> crcb b0625
<222> 285-384
<223> crcb b0625 U00096 complement(656778..657161)
<313> 11500: 309-328
<400> 11500
CTTTGGCTGG GCATTACTGA ACGTTTTCGT CAACCTTCTG GGGTCTTTTG 50
CCATGACCGC ACTGGCATTC TGGCTGTTTT CGGCCTCAAC CGCACACTAA 100
<210> 11501
<211> 100
<212> DNA
<213> Escherichia coli K-12 MG1655
<220> crcb b0625
<222> 228-327
<223> crcb b0625 U00096 complement(656778..657161)
<313> 11501: 279-298
<400> 11501
TCTAACAACC TTCTCAACAT TTTCGGCAGA AGTGGTGTTT TTGTTACAAG 50
AGGGCCGCTT TGGCTGGGCA TTACTGAACG TTTTCGTCAA CCTTCTGGGG 100
<210> 11502
<211> 100
<212> DNA
<213> Escherichia coli K-12 MG1655
<220> cytr b3935
<222> 927-1026
<223> cytr b3935 U00096 complement(4121011..4122036)

<313> 11502: 981-1000
<400> 11502
GTTATTGCTT GATCAAATGC AGGGGCAACA CGTTGGCAGT GGCTCTCGTT 50
TAATGGACTG CGAACTTATC ATCCGGGGAT CAACACGCGC GTTACCTTAA 100
<210> 11503
<211> 100
<212> DNA
<213> Escherichia coli K-12 MG1655
<220> cytr b3935
<222> 855-954
<223> cytr b3935 U00096 complement(4121011..4122036)
<313> 11503: 891-910
<400> 11503
GACGCAATTT TGTGATCCGC CGCTGACAAC CATCGCGCAG CCGCGTTACG 50
AAATCGGTCG GGAAGCTATG CTGTTATTGC TTGATCAAAT GCAGGGGCAA 100
<210> 11504
<211> 100
<212> DNA
<213> Escherichia coli K-12 MG1655
<220> cytr b3935
<222> 743-842
<223> cytr b3935 U00096 complement(4121011..4122036)
<313> 11504: 775-794
<400> 11504
CTGTCTTCTG CCATAGCGAT GTGATGGCGC TCGGCGCACT TTCTCAGGCA 50
AAACGCCAGG GGCTGAAAGT CCCGGAAGAC CTTTCCATAA TCGGTTTTGA 100
<210> 11505
<211> 100
<212> DNA
<213> Escherichia coli K-12 MG1655
<220> sieb sieB
<222> 268-367
<223> sieb sieB U00096 1416572..1417183
<313> 11505: 324-343
<400> 11505
CCTTGGCTCT TTCAGATTAT CGTTTTTGCC TTTTCTTTCG TGGTCGCCAT 50
TTCCTTCTCA AGATTGCGAG CACATATTCA AAAGCATTAT TCATTACTAC 100
<210> 11506
<211> 100
<212> DNA
<213> Escherichia coli K-12 MG1655
<220> sieb sieB
<222> 192-291
<223> sieb sieB U00096 1416572..1417183
<313> 11506: 223-242
<400> 11506
GATTATCATT TTTGGTCTCA CTCTCTGGCT GATACCGAAA GAGTTTACTG 50
TCGCATTCAA TGCTTATACT GAAATACCTT GGCTCTTTCA GATTATCGTT 100
<210> 11507
<211> 100
<212> DNA
<213> Escherichia coli K-12 MG1655
<220> sieb sieB
<222> 7-106
<223> sieb sieB U00096 1416572..1417183
<313> 11507: 64-83
<400> 11507
CTTCGTGTTG TGCCCGGTTT TATTTCACCA CCTCCGGGCT TCGGTGGTCT 50
CGGCTATACC CCTACAGCGA GAGCTTGTGT TAACATTTCA ATACCCTTAC 100
<210> 11508
<211> 100
<212> DNA
<213> Escherichia coli K-12 MG1655
<220> nadr b4391
<222> 1155-1254
<223> nadr b4391 U00096 4624863..4626116
<313> 11508: 1222-1241
<400> 11508
GAACAATATC GAATTTGTGC GGGTTGAAGA GGAAGATTAC GACAGTCGTT 50
TCCTGCGCTG CGTGGAACTG GTGCGGGAGA TGATGGGGGA GCAGAGATAA 100
<210> 11509

<211> 100
<212> DNA
<213> Escherichia coli K-12 MG1655
<220> nadr b4391
<222> 1137-1236
<223> nadr b4391 U00096 4624863..4626116
<313> 11509: 1187-1206
<400> 11509
GGTGGAGATG CTCGAAGAGA ACAATATCGA ATTTGTGCGG GTTGAAGAGG 50
AAGATTACGA CAGTCGTTTC CTGCGCTGCG TGGAACTGGT GCGGGAGATG 100
<210> 11510
<211> 100
<212> DNA
<213> Escherichia coli K-12 MG1655
<220> nadr b4391
<222> 1118-1217
<223> nadr b4391 U00096 4624863..4626116
<313> 11510: 1137-1156
<400> 11510
AAGAGTTCCA GAACTTGCTG GTGGAGATGC TCGAAGAGAA CAATATCGAA 50
TTTGTGCGGG TTGAAGAGGA AGATTACGAC AGTCGTTTCC TGCGCTGCGT 100
<210> 11511
<211> 100
<212> DNA
<213> Escherichia coli K-12 MG1655
<220> mscl b3292
<222> 306-405
<223> mscl b3292 U00096 3435661..3436071
<313> 11511: 341-360
<400> 11511
GAATCGGAAA AAAGAAGAAC CAGCAGCCGC ACCTGCACCA ACTAAAGAAG 50
AAGTATTACT GACAGAAATT CGTGATTTGC TGAAAGAGCA GAATAACCGC 100
<210> 11512
<211> 100
<212> DNA
<213> Escherichia coli K-12 MG1655
<220> mscl b3292
<222> 70-169
<223> mscl b3292 U00096 3435661..3436071
<313> 11512: 122-141
<400> 11512
ATTATCGGTG CGGCATTCGG GAAGATTGTC TCTTCACTGG TTGCCGATAT 50
CATCATGCCT CCTCTGGGCT TATTAATTGG CGGGATCGAT TTTAAACAGT 100
<210> 11513
<211> 100
<212> DNA
<213> Escherichia coli K-12 MG1655
<220> mscl b3292
<222> 58-157
<223> mscl b3292 U00096 3435661..3436071
<313> 11513: 94-113
<400> 11513
GCGGTGGGTG TCATTATCGG TGCGGCATTC GGGAAGATTG TCTCTTCACT 50
GGTTGCCGAT ATCATCATGC CTCCTCTGGG CTTATTAATT GGCGGGATCG 100
<210> 11514
<211> 100
<212> DNA
<213> Escherichia coli K-12 MG1655
<220> ynic yniC
<222> 570-669
<223> ynic yniC U00096 1807404..1808072
<313> 11514: 649-668
<400> 11514
CGTTCCTGCG CCAGAAGCGC AAAATGATCC ACGTTTTGTA TTAGCAGACG 50
TCAAACTTTC ATCGCTGACA GAACTCACCG CAAAAGACCT TCTCGGTTAA 100
<210> 11515
<211> 100
<212> DNA
<213> Escherichia coli K-12 MG1655
<220> ynic yniC
<222> 530-629

<223> ynic yniC U00096 1807404..1808072
<313> 11515: 566-585
<400> 11515
GCATGATCGC CTCTAAAGCA GCCCGCATGC GTTCCATCGT CGTTCCTGCG 50
CCAGAAGCGC AAAATGATCC ACGTTTTGTA TTAGCAGACG TCAAACTTTC 100
<210> 11516
<211> 100
<212> DNA
<213> Escherichia coli K-12 MG1655
<220> ynic yniC
<222> 499-598
<223> ynic yniC U00096 1807404..1808072
<313> 11516: 530-549
<400> 11516
ACCTGCGTAG CGCTGGAAGA TTCGGTAAAT GGCATGATCG CCTCTAAAGC 50
AGCCCGCATG CGTTCCATCG TCGTTCCTGC GCCAGAAGCG CAAAATGATC 100
<210> 11517
<211> 100
<212> DNA
<213> Escherichia coli K-12 MG1655
<220> rsta b1609
<222> 630-729
<223> rsta b1609 U00096 1680174..1680902
<313> 11517: 681-700
<400> 11517
GCGGTTAAGA AAAAAACTGC TCGATAACGC CGCAGAACCT TATCGCATTA 50
AAACTGTGCG TAACAAAGGC TATCTTTTTG CGCCTCATGC ATGGGAATAA 100
<210> 11518
<211> 100
<212> DNA
<213> Escherichia coli K-12 MG1655
<220> rsta b1609
<222> 597-696
<223> rsta b1609 U00096 1680174..1680902
<313> 11518: 631-650
<400> 11518
CGGACTGGAT CGTAGCGTGG ACGTGGCTAT TTCGCGGTTA AGAAAAAAAC 50
TGCTCGATAA CGCCGCAGAA CCTTATCGCA TTAAAACTGT GCGTAACAAA 100
<210> 11519
<211> 100
<212> DNA
<213> Escherichia coli K-12 MG1655
<220> rsta b1609
<222> 478-577
<223> rsta b1609 U00096 1680174..1680902
<313> 11519: 533-552
<400> 11519
AACACTGAAA TCTCGCTCTC GACAGCTGAT TTCGAATTAT TGTGGGAATT 50
AGCTACCCAT GCCGGGCAAA TCATGGACCG CGATGCATTG CTGAAAAATT 100
<210> 11520
<211> 100
<212> DNA
<213> Escherichia coli K-12 MG1655
<220> rstb b1610
<222> 1203-1302
<223> rstb b1610 U00096 1680906..1682207
<313> 11520: 1280-1299
<400> 11520
GGGCGGTACG GTTAATTGTG ACACCAGCGA ACTGGGTGGT GCCCGCTTCT 50
CGTTTAGCTG GCCGTTATGG CATAACATCC CGCAATTTAC CTCTGCCTGA 100
<210> 11521
<211> 100
<212> DNA
<213> Escherichia coli K-12 MG1655
<220> rstb b1610
<222> 1185-1284
<223> rstb b1610 U00096 1680906..1682207
<313> 11521: 1225-1244
<400> 11521
CTCTATAGCA CTGGCAATGG GCGGTACGGT TAATTGTGAC ACCAGCGAAC 50
TGGGTGGTGC CCGCTTCTCG TTTAGCTGGC CGTTATGGCA TAACATCCCG 100

<210> 11522
<211> 100
<212> DNA
<213> Escherichia coli K-12 MG1655
<220> rstb b1610
<222> 1140-1239
<223> rstb b1610 U00096 1680906..1682207
<313> 11522: 1192-1211
<400> 11522
GGATCGCTCA ACCGGCGGCT GCGGGCTGGG GCTGGCAATT GTCCACTCTA 50
TAGCACTGGC AATGGGCGGT ACGGTTAATT GTGACACCAG CGAACTGGGT 100
<210> 11523
<211> 100
<212> DNA
<213> Escherichia coli K-12 MG1655
<220> fima b4315
<222> 362-461
<223> fima b4315 U00096 4540683..4541231
<313> 11523: 420-439
<400> 11523
ACGTTGGTGT GCAGATCCTG GACAGAACGG GTGCTGCGCT GACGCTGGAT 50
GGTGCGACAT TTAGTTCAGA AACAACCCTG AATAACGGAA CCAATACCAT 100
<210> 11524
<211> 100
<212> DNA
<213> Escherichia coli K-12 MG1655
<220> fima b4315
<222> 336-435
<223> fima b4315 U00096 4540683..4541231
<313> 11524: 384-403
<400> 11524
GAGTTCAGCT GCGGGTAGCG CAACAAACGT TGGTGTGCAG ATCCTGGACA 50
GAACGGGTGC TGCGCTGACG CTGGATGGTG CGACATTTAG TTCAGAAACA 100
<210> 11525
<211> 100
<212> DNA
<213> Escherichia coli K-12 MG1655
<220> fima b4315
<222> 270-369
<223> fima b4315 U00096 4540683..4541231
<313> 11525: 304-323
<400> 11525
TAAAGCCGCT GTTGCCTTTT TAGGTACGGC GATTGATGCG GGTCATACCA 50
ACGTTCTGGC TCTGCAGAGT TCAGCTGCGG GTAGCGCAAC AAACGTTGGT 100
<210> 11526
<211> 100
<212> DNA
<213> Escherichia coli K-12 MG1655
<220> fimb b4313
<222> 504-603
<223> fimb b4313 U00096 4538525..4539127
<313> 11526: 571-590
<400> 11526
CAATATTCGT CATACTGTCT GGTATACCGC CAGCAATGCA GGGCGTTTTT 50
ACGGCATCTG GGATAGAGCC AGAGGACGAC AGCGTCACGC TGTTTTATAG 100
<210> 11527
<211> 100
<212> DNA
<213> Escherichia coli K-12 MG1655
<220> fimb b4313
<222> 423-522
<223> fimb b4313 U00096 4538525..4539127
<313> 11527: 453-472
<400> 11527
CATGTTACGC CATTCGTGTG GTTTTGCTTT GGCGAATATG GGAATAGATA 50
CGCGACTTAT CCAGGATTAT CTTGGGCATC GCAATATTCG TCATACTGTC 100
<210> 11528
<211> 100
<212> DNA
<213> Escherichia coli K-12 MG1655
<220> fimb b4313

<222> 357-456
<223> fimb b4313 U00096 4538525..4539127
<313> 11528: 412-431
<400> 11528
ACAGTTTTAC CATATTATCT CGACTTCCGG TGGTAATGCC GGGTTGTCAC 50
TGGAGATTCA TCCGCACATG TTACGCCATT CGTGTGGTTT TGCTTTGGCG 100
<210> 11529
<211> 100
<212> DNA
<213> Escherichia coli K-12 MG1655
<220> fimc b4317
<222> 627-726
<223> fimc b4317 U00096 4541872..4542597
<313> 11529: 702-721
<400> 11529
CACGGTTAAA TTGCCTTCTG ATGCAGGAAG CAATATTACT TACCGAACAA 50
TAAATGATTA TGGCGCACTT ACCCCCAAAA TGACGGGCGT AATGGAATAA 100
<210> 11530
<211> 100
<212> DNA
<213> Escherichia coli K-12 MG1655
<220> fimc b4317
<222> 591-690
<223> fimc b4317 U00096 4541872..4542597
<313> 11530: 634-653
<400> 11530
TCTTGAAAAT GCATTGGTGC CTCCAATGGG CGAAAGCACG GTTAAATTGC 50
CTTCTGATGC AGGAAGCAAT ATTACTTACC GAACAATAAA TGATTATGGC 100
<210> 11531
<211> 100
<212> DNA
<213> Escherichia coli K-12 MG1655
<220> fimc b4317
<222> 518-617
<223> fimc b4317 U00096 4541872..4542597
<313> 11531: 556-575
<400> 11531
CGAATTCTCT GACGCTGATT AACCCGACAC CCTATTACCT GACGGTAACA 50
GAGTTGAATG CCGGAACCCG GGTTCTTGAA AATGCATTGG TGCCTCCAAT 100
<210> 11532
<211> 100
<212> DNA
<213> Escherichia coli K-12 MG1655
<220> fimd b4318
<222> 2538-2637
<223> fimd b4318 U00096 4542665..4545301
<313> 11532: 2592-2611
<400> 11532
ATGGGGAGAA GAGGAAAATG CTCACTGTGT CGCCAATTAT CAACTGCCAC 50
CAGAGAGTCA GCAGCAGTTA TTAACCCAGC TATCAGCTGA ATGTCGTTAA 100
<210> 11533
<211> 100
<212> DNA
<213> Escherichia coli K-12 MG1655
<220> fimd b4318
<222> 2505-2604
<223> fimd b4318 U00096 4542665..4545301
<313> 11533: 2553-2572
<400> 11533
CGGAATGCCT TTAGCGGGAA AAGTTCAGGT GAAATGGGGA GAAGAGGAAA 50
ATGCTCACTG TGTCGCCAAT TATCAACTGC CACCAGAGAG TCAGCAGCAG 100
<210> 11534
<211> 100
<212> DNA
<213> Escherichia coli K-12 MG1655
<220> fimd b4318
<222> 2489-2588
<223> fimd b4318 U00096 4542665..4545301
<313> 11534: 2516-2535
<400> 11534
GTCAGGTTTA CCTCAGCGGA ATGCCTTTAG CGGGAAAAGT TCAGGTGAAA 50

TGGGGAGAAG AGGAAAATGC TCACTGTGTC GCCAATTATC AACTGCCACC 100
<210> 11535
<211> 100
<212> DNA
<213> Escherichia coli K-12 MG1655
<220> fime b4314
<222> 472-571
<223> fime b4314 U00096 4539605..4540201
<313> 11535: 529-548
<400> 11535
GATTATCTCG GGCATCGAAA TATTCGCCAT ACTGTGCGTT ATACCGCCAG 50
TAATGCTGCT CGTTTTGCCG GATTATGGGA AAGAAATAAT CTCATAAACG 100
<210> 11536
<211> 100
<212> DNA
<213> Escherichia coli K-12 MG1655
<220> fime b4314
<222> 458-557
<223> fime b4314 U00096 4539605..4540201
<313> 11536: 480-499
<400> 11536
CTCGTTTAAT TCAGGATTAT CTCGGGCATC GAAATATTCG CCATACTGTG 50
CGTTATACCG CCAGTAATGC TGCTCGTTTT GCCGGATTAT GGGAAAGAAA 100
<210> 11537
<211> 100
<212> DNA
<213> Escherichia coli K-12 MG1655
<220> fime b4314
<222> 418-517
<223> fime b4314 U00096 4539605..4540201
<313> 11537: 446-465
<400> 11537
CATGCTTGCG GTTATGAATT GGCGGAGCGT GGTGCAGATA CTCGTTTAAT 50
TCAGGATTAT CTCGGGCATC GAAATATTCG CCATACTGTG CGTTATACCG 100
<210> 11538
<211> 100
<212> DNA
<213> Escherichia coli K-12 MG1655
<220> fimf b4319
<222> 432-531
<223> fimf b4319 U00096 4545311..4545841
<313> 11538: 501-520
<400> 11538
AAATACGCTG AATTTTTACG CCCGGCTAAT GGCGACACAG GTGCCTGTCA 50
CTGCGGGGCA TATCAATGCC ACGGCTACCT TCACTCTTGA ATATCAGTAA 100
<210> 11539
<211> 100
<212> DNA
<213> Escherichia coli K-12 MG1655
<220> fimf b4319
<222> 392-491
<223> fimf b4319 U00096 4545311..4545841
<313> 11539: 440-459
<400> 11539
CGTCCGCGCT TTCGTGGACG ACCCTGACGC CGGGTAAACC AAATACGCTG 50
AATTTTTACG CCCGGCTAAT GGCGACACAG GTGCCTGTCA CTGCGGGGCA 100
<210> 11540
<211> 100
<212> DNA
<213> Escherichia coli K-12 MG1655
<220> fimf b4319
<222> 379-478
<223> fimf b4319 U00096 4545311..4545841
<313> 11540: 414-433
<400> 11540
CTTAATGCTC CATCGTCCGC GCTTTCGTGG ACGACCCTGA CGCCGGGTAA 50
ACCAAATACG CTGAATTTTT ACGCCCGGCT AATGGCGACA CAGGTGCCTG 100
<210> 11541
<211> 100
<212> DNA
<213> Escherichia coli K-12 MG1655

<220> fimg b4320
<222> 405-504
<223> fimg b4320 U00096 4545854..4546357
<313> 11541: 474-493
<400> 11541
AGCGCACTTC CCGTTACAGG TCAGAGCATT GACAGTAAAT GGCGGAGCCA 50
CTCAGGGAAC CATTCAGGCA GTGATTAGCA TCACCTATAC CTACAGCTGA 100
<210> 11542
<211> 100
<212> DNA
<213> Escherichia coli K-12 MG1655
<220> fimg b4320
<222> 391-490
<223> fimg b4320 U00096 4545854..4546357
<313> 11542: 425-444
<400> 11542
GATTCCTCAC AATCAGCGCA CTTCCCGTTA CAGGTCAGAG CATTGACAGT 50
AAATGGCGGA GCCACTCAGG GAACCATTCA GGCAGTGATT AGCATCACCT 100
<210> 11543
<211> 100
<212> DNA
<213> Escherichia coli K-12 MG1655
<220> fimg b4320
<222> 328-427
<223> fimg b4320 U00096 4545854..4546357
<313> 11543: 393-412
<400> 11543
GAGCTACAGG ATGACAGTGG CAACACATTG AATACTGGCG CAACCAAAAC 50
AGTTCAGGTG GATGATTCCT CACAATCAGC GCACTTCCCG TTACAGGTCA 100
<210> 11544
<211> 100
<212> DNA
<213> Escherichia coli K-12 MG1655
<220> fimh b4321
<222> 804-903
<223> fimh b4321 U00096 4546377..4547279
<313> 11544: 835-854
<400> 11544
GGTGAGTCTG GGATTAACGG CAAATTATGC ACGTACCGGA GGGCAGGTGA 50
CTGCAGGGAA TGTGCAATCG ATTATTGGCG TGACTTTTGT TTATCAATAA 100
<210> 11545
<211> 100
<212> DNA
<213> Escherichia coli K-12 MG1655
<220> fimh b4321
<222> 758-857
<223> fimh b4321 U00096 4546377..4547279
<313> 11545: 782-801
<400> 11545
TTCCAGCGAA TAACACGGTA TCGTTAGGAG CAGTAGGGAC TTCGGCGGTG 50
AGTCTGGGAT TAACGGCAAA TTATGCACGT ACCGGAGGGC AGGTGACTGC 100
<210> 11546
<211> 100
<212> DNA
<213> Escherichia coli K-12 MG1655
<220> fimh b4321
<222> 688-787
<223> fimh b4321 U00096 4546377..4547279
<313> 11546: 743-762
<400> 11546
TTCACCAATA CCGCGTCGTT TTCACCTGCA CAGGGCGTCG GCGTACAGTT 50
GACGCGCAAC GGTACGATTA TTCCAGCGAA TAACACGGTA TCGTTAGGAG 100
<210> 11547
<211> 100
<212> DNA
<213> Escherichia coli K-12 MG1655
<220> fimi b4316
<222> 547-646
<223> fimi b4316 U00096 4541188..4541835
<313> 11547: 582-601
<400> 11547

TCTACTTCGC TACATTTCAT CGCCAAATAT CGTGCTACCG GGCGTCGGGT 50
TACTGGCGGC ATCGCCAATG CCCAGGCCTG GTTCTCTTTA ACCTATCAGT 100
<210> 11548
<211> 100
<212> DNA
<213> Escherichia coli K-12 MG1655
<220> fimi b4316
<222> 508-607
<223> fimi b4316 U00096 4541188..4541835
<313> 11548: 535-554
<400> 11548
AATCGTCCTC CAGCAAACTG GAAACGGCTT TATTCAGGCT CTACTTCGCT 50
ACATTTCATC GCCAAATATC GTGCTACCGG GCGTCGGGTT ACTGGCGGCA 100
<210> 11549
<211> 100
<212> DNA
<213> Escherichia coli K-12 MG1655
<220> fimi b4316
<222> 338-437
<223> fimi b4316 U00096 4541188..4541835
<313> 11549: 370-389
<400> 11549
TTATTCATTT ACGGGAATGT AGCACGGTGG TGAGTGAACG TGTAGGTGTG 50
GCGTTTCACG GTGTCGCGGA TGGTAAAAAT CCGGATGTGC TTTCCGTGGG 100
<210> 11550
<211> 100
<212> DNA
<213> Escherichia coli K-12 MG1655
<220> ynja ynjA
<222> 418-517
<223> ynja ynjA U00096 1833539..1834087
<313> 11550: 465-484
<400> 11550
GCGCATTTTG ACGCTCAGGC GCTCACCGAA CTGACGGCAT TGATCGGCCT 50
GCAAAATCTG TCAGCCCGTT TTAATTCTGC CATGGACATT CCCGCTCAGG 100
<210> 11551
<211> 100
<212> DNA
<213> Escherichia coli K-12 MG1655
<220> ynja ynjA
<222> 376-475
<223> ynja ynjA U00096 1833539..1834087
<313> 11551: 428-447
<400> 11551
ACGCCGCCAA CGGTCGATGA TGCCCTGCGT ACCCGACTGG CTGCGCATTT 50
TGACGCTCAG GCGCTCACCG AACTGACGGC ATTGATCGGC CTGCAAAATC 100
<210> 11552
<211> 100
<212> DNA
<213> Escherichia coli K-12 MG1655
<220> ynja ynjA
<222> 350-449
<223> ynja ynjA U00096 1833539..1834087
<313> 11552: 374-393
<400> 11552
TGGAGTACGC CGAAGCCGCA AGCGTAACGC CGCCAACGGT CGATGATGCC 50
CTGCGTACCC GACTGGCTGC GCATTTTGAC GCTCAGGCGC TCACCGAACT 100
<210> 11553
<211> 100
<212> DNA
<213> Escherichia coli K-12 MG1655
<220> b0538 b0538
<222> 462-561
<223> b0538 b0538 U00096 565195..565755
<313> 11553: 530-549
<400> 11553
TCGAACGCGC ATTACTGGTT GGTTACCAAC TTGTACCAGA ACATGCGGGC 50
CAATGCGCTG GCTGACGCGG AATTACGGCG CAAGGCTGCC GATGAACTGA 100
<210> 11554
<211> 100
<212> DNA

<213> Escherichia coli K-12 MG1655
<220> b0538 b0538
<222> 462-561
<223> b0538 b0538 U00096 565195..565755
<313> 11554: 492-511
<400> 11554
TCGAACGCGC ATTACTGGTT GGTTACCAAC TTGTACCAGA ACATGCGGGC 50
CAATGCGCTG GCTGACGCGG AATTACGGCG CAAGGCTGCC GATGAACTGA 100
<210> 11555
<211> 100
<212> DNA
<213> Escherichia coli K-12 MG1655
<220> b0538 b0538
<222> 359-458
<223> b0538 b0538 U00096 565195..565755
<313> 11555: 405-424
<400> 11555
ATTGCCTCGA AACCACCGAG CCGGAATTTC ATGAAATCCC GGGAGGTACG 50
AGTATTGCCG GAAGCGTGGC CTGTATCCGG ATGCAGAGTC TTATCCGTGG 100
<210> 11556
<211> 100
<212> DNA
<213> Escherichia coli K-12 MG1655
<220> b0539 b0539
<222> 149-248
<223> b0539 b0539 U00096 complement(565321..565584)
<313> 11556: 210-229
<400> 11556
TTGAGCGGAA TGAAAAGTAC ATGGCGAGTT TTGACGAGAT GGTGCCGGAG 50
TTCATCGAAA AAATGGACGA GGCACTGGCT GAAATTGGTT TTGTATTTGG 100
<210> 11557
<211> 100
<212> DNA
<213> Escherichia coli K-12 MG1655
<220> b0539 b0539
<222> 138-237
<223> b0539 b0539 U00096 complement(565321..565584)
<313> 11557: 154-173
<400> 11557
TTATGTCGTG ATTGAGCGGA ATGAAAAGTA CATGGCGAGT TTTGACGAGA 50
TGGTGCCGGA GTTCATCGAA AAAATGGACG AGGCACTGGC TGAAATTGGT 100
<210> 11558
<211> 100
<212> DNA
<213> Escherichia coli K-12 MG1655
<220> b0539 b0539
<222> 51-150
<223> b0539 b0539 U00096 complement(565321..565584)
<313> 11558: 98-117
<400> 11558
CCAGGTGCAG TACAGCATGT GGGTGACGCG AAAAGATGCC TGGTACTTTG 50
CCAACTATGA CCCGCGCATG AAGCGTGAAG GCCTGCATTA TGTCGTGATT 100
<210> 11559
<211> 100
<212> DNA
<213> Escherichia coli K-12 MG1655
<220> fimz b0536
<222> 244-343
<223> fimz b0536 U00096 complement(563071..563703)
<313> 11559: 299-318
<400> 11559
TTTTTATCAT CGAAATCAGA ATGCTTTTAT GCTGGCAGAG CGATACAAGC 50
TGGTGCTAAC GGTTTTGTCA GTAAATGCAA TGATCAGAAT GATATTTTTC 100
<210> 11560
<211> 100
<212> DNA
<213> Escherichia coli K-12 MG1655
<220> fimz b0536
<222> 168-267
<223> fimz b0536 U00096 complement(563071..563703)
<313> 11560: 188-207

<400> 11560
TATAGACTTG CCCGGAACAG ACGGTTTTAC CTTCCTGAAA AGGATCAAAC  50
AAATCCAGAG CACAGTGAAA GTGTTATTTT TATCATCGAA ATCAGAATGC  100
<210> 11561
<211> 100
<212> DNA
<213> Escherichia coli K-12 MG1655
<220> fimz b0536
<222> 76-175
<223> fimz b0536 U00096 complement(563071..563703)
<313> 11561: 117-136
<400> 11561
AACAGTGAAT TGCAGATTGT CCTGAAAACG GATGATTATC GCATAACCAT  50
CGATTATCTC CGAACCCGTC CTGTTGATTT AATCATTATG GATATAGACT  100
<210> 11562
<211> 100
<212> DNA
<213> Escherichia coli K-12 MG1655
<220> crea b4398
<222> 375-474
<223> crea b4398 U00096 4633090..4633563
<313> 11562: 442-461
<400> 11562
ACGCAACGCG CTCGCTTATC TGGCTTACTC CGACAAAGTT GTAGAAGGTT  50
CGCCGAAAAA CGCGATTAGC GCGGTTCCTG TCATGCCGTG GCGGCAATAA  100
<210> 11563
<211> 100
<212> DNA
<213> Escherichia coli K-12 MG1655
<220> crea b4398
<222> 359-458
<223> crea b4398 U00096 4633090..4633563
<313> 11563: 384-403
<400> 11563
GCTTTTATGA TGCCAAACGC AACGCGCTCG CTTATCTGGC TTACTCCGAC  50
AAAGTTGTAG AAGGTTCGCC GAAAAACGCG ATTAGCGCGG TTCCTGTCAT  100
<210> 11564
<211> 100
<212> DNA
<213> Escherichia coli K-12 MG1655
<220> crea b4398
<222> 301-400
<223> crea b4398 U00096 4633090..4633563
<313> 11564: 359-378
<400> 11564
GGCGAGGTAG TATTCAAAAA ACGCACGTCC CTGGTCTTTA AGTCGTTACA  50
GGTCGTGCGC TTTTATGATG CCAAACGCAA CGCGCTCGCT TATCTGGCTT  100
<210> 11565
<211> 100
<212> DNA
<213> Escherichia coli K-12 MG1655
<220> rsua b2184
<222> 597-696
<223> rsua b2184 U00096 complement(2277808..2278503)
<313> 11565: 658-677
<400> 11565
GCTGCATCGT GAACGTATTG GCGGTATTAC GCTGGATGCT GATTTAGCCC  50
CCGGTGAATA TCGTCCGTTA ACTGAAGAAG AAATTGCCAG CGTCGTCTAA  100
<210> 11566
<211> 100
<212> DNA
<213> Escherichia coli K-12 MG1655
<220> rsua b2184
<222> 569-668
<223> rsua b2184 U00096 complement(2277808..2278503)
<313> 11566: 635-654
<400> 11566
TCGCCGCCGT GGGTAACCAC GTGGTTGAGC TGCATCGTGA ACGTATTGGC  50
GGTATTACGC TGGATGCTGA TTTAGCCCCC GGTGAATATC GTCCGTTAAC  100
<210> 11567
<211> 100

<212> DNA
<213> Escherichia coli K-12 MG1655
<220> rsua b2184
<222> 528-627
<223> rsua b2184 U00096 complement(2277808..2278503)
<313> 11567: 551-570
<400> 11567
GACCATCAGC GAAGGGCGTT ATCATCAGGT GAAACGCATG TTCGCCGCCG 50
TGGGTAACCA CGTGGTTGAG CTGCATCGTG AACGTATTGG CGGTATTACG 100
<210> 11568
<211> 100
<212> DNA
<213> Escherichia coli K-12 MG1655
<220> creb b4399
<222> 591-690
<223> creb b4399 U00096 4633576..4634265
<313> 11568: 657-676
<400> 11568
CCACATTAAA ACGCTGCGTG CCAAGCTGCG CGCCATCAAC CCCGATCTTT 50
CACCGATTAA TACTCATCGC GGCATGGGAT ATAGCCTGAG GGGCCTGTAA 100
<210> 11569
<211> 100
<212> DNA
<213> Escherichia coli K-12 MG1655
<220> creb b4399
<222> 565-664
<223> creb b4399 U00096 4633576..4634265
<313> 11569: 597-616
<400> 11569
GACACCTACG ATCGCACCGT CGATACCCAC ATTAAAACGC TGCGTGCCAA 50
GCTGCGCGCC ATCAACCCCG ATCTTTCACC GATTAATACT CATCGCGGCA 100
<210> 11570
<211> 100
<212> DNA
<213> Escherichia coli K-12 MG1655
<220> creb b4399
<222> 491-590
<223> creb b4399 U00096 4633576..4634265
<313> 11570: 532-551
<400> 11570
AGACGTTACT CAAGTCACCG GGCCGCGTCT GGTCCCGCCA GCAACTGATG 50
GATAGCGTAT GGGAAGATGC GCAGGACACC TACGATCGCA CCGTCGATAC 100
<210> 11571
<211> 100
<212> DNA
<213> Escherichia coli K-12 MG1655
<220> crec b4400
<222> 1326-1425
<223> crec b4400 U00096 4634265..4635689
<313> 11571: 1402-1421
<400> 11571
CAGTGAGGTC GCCCGTTTGT TTAACGGCGA AGTCACGCTG CGCAACGTGC 50
AGGAAGGTGG CGTGCTGGCC TCGCTTCGAC TTCACCGTCA CTTCACATAG 100
<210> 11572
<211> 100
<212> DNA
<213> Escherichia coli K-12 MG1655
<220> crec b4400
<222> 1308-1407
<223> crec b4400 U00096 4634265..4635689
<313> 11572: 1338-1357
<400> 11572
TCTGGGGTTG GCGTTCGTCA GTGAGGTCGC CCGTTTGTTT AACGGCGAAG 50
TCACGCTGCG CAACGTGCAG GAAGGTGGCG TGCTGGCCTC GCTTCGACTT 100
<210> 11573
<211> 100
<212> DNA
<213> Escherichia coli K-12 MG1655
<220> crec b4400
<222> 1245-1344
<223> crec b4400 U00096 4634265..4635689

<313> 11573: 1304-1323
<400> 11573
GCTTTCACGT ATTTTTGAAC GCTTTTACTC TTTGCCTCGT GCAAATGGGC 50
AAAAAAGCAG CGGTCTGGGG TTGGCGTTCG TCAGTGAGGT CGCCCGTTTG 100
<210> 11574
<211> 100
<212> DNA
<213> Escherichia coli K-12 MG1655
<220> cred b4401
<222> 1254-1353
<223> cred b4401 U00096 4635747..4637099
<313> 11574: 1300-1319
<400> 11574
GTTTGTGACC CGTAATATCG ACTGGTATGC GTTTTCACTG CCGAAAATGA 50
AAGCCAGTAA AGAAGTTACA ACGGACGATG AGTTACGTAT CTGGAAATAA 100
<210> 11575
<211> 100
<212> DNA
<213> Escherichia coli K-12 MG1655
<220> cred b4401
<222> 1228-1327
<223> cred b4401 U00096 4635747..4637099
<313> 11575: 1259-1278
<400> 11575
CTGGTGGTGG CGCTGGCCGG CATGATGTTT GTGACCCGTA ATATCGACTG 50
GTATGCGTTT TCACTGCCGA AAATGAAAGC CAGTAAAGAA GTTACAACGG 100
<210> 11576
<211> 100
<212> DNA
<213> Escherichia coli K-12 MG1655
<220> cred b4401
<222> 1152-1251
<223> cred b4401 U00096 4635747..4637099
<313> 11576: 1170-1189
<400> 11576
GCTGTTGTTG CTGGATGGTG TGATGTGGGG ACTGCTCAAC TCTGCCGATA 50
GCGCGCTGTT GTTGGGAACC AGTGTGCTGG TGGTGGCGCT GGCCGGCATG 100
<210> 11577
<211> 100
<212> DNA
<213> Escherichia coli K-12 MG1655
<220> wecb b3787
<222> 1019-1118
<223> wecb b3787 U00096 3967706..3968875
<313> 11577: 1081-1100
<400> 11577
CGGATAAGCA GCGAATTGTC GAGGAAGTGA CGCGTCTTTT AAAAGACGAA 50
AACGAATATC AAGCTATGAG CCGCGCCCAT AACCCGTATG GTGATGGTCA 100
<210> 11578
<211> 100
<212> DNA
<213> Escherichia coli K-12 MG1655
<220> wecb b3787
<222> 965-1064
<223> wecb b3787 U00096 3967706..3968875
<313> 11578: 1019-1038
<400> 11578
ATACCACTGA GCGTCCGGAA GCGGTGACGG CGGGTACGGT GCGTCTGGTA 50
GGCACGGATA AGCAGCGAAT TGTCGAGGAA GTGACGCGTC TTTTAAAAGA 100
<210> 11579
<211> 100
<212> DNA
<213> Escherichia coli K-12 MG1655
<220> wecb b3787
<222> 921-1020
<223> wecb b3787 U00096 3967706..3968875
<313> 11579: 959-978
<400> 11579
GGAAGAAGCG CCTTCGCTGG GGAAACCTGT GCTGGTGATG CGCGATACCA 50
CTGAGCGTCC GGAAGCGGTG ACGGCGGGTA CGGTGCGTCT GGTAGGCACG 100
<210> 11580

<211> 100
<212> DNA
<213> Escherichia coli K-12 MG1655
<220> wecc b3788
<222> 1063-1162
<223> wecc b3788 U00096 3968872..3970134
<313> 11580: 1119-1138
<400> 11580
GGCGAAACTC TGGTTGTTGA GCCTAACATC CACCAGTTGC CGAAAAAACT 50
GACCGGGCTT TGTACTCTGG CGCAGCTTGA CGAGGCGCTG GCAACGGCAG 100
<210> 11581
<211> 100
<212> DNA
<213> Escherichia coli K-12 MG1655
<220> wecc b3788
<222> 1045-1144
<223> wecc b3788 U00096 3968872..3970134
<313> 11581: 1069-1088
<400> 11581
ATCGCCCAGT GGCATAGCGG CGAAACTCTG GTTGTTGAGC CTAACATCCA 50
CCAGTTGCCG AAAAAACTGA CCGGGCTTTG TACTCTGGCG CAGCTTGACG 100
<210> 11582
<211> 100
<212> DNA
<213> Escherichia coli K-12 MG1655
<220> wecc b3788
<222> 951-1050
<223> wecc b3788 U00096 3968872..3970134
<313> 11582: 1016-1035
<400> 11582
CGCCAGTGAA CTGAAAATCG CCTGCTTTGG TCTGGCGTTT AAACCGAATA 50
TTGATGACCT GCGCGAAAGC CCGGCGATGG AAATCGCTGA ACTGATCGCC 100
<210> 11583
<211> 100
<212> DNA
<213> Escherichia coli K-12 MG1655
<220> wecd b3791
<222> 447-546
<223> wecd b3791 U00096 3972208..3972753
<313> 11583: 514-533
<400> 11583
TTTGCGGGTG GCGACCCAAA TGGGCAACAC CGCCGCGCTT AAACGATACA 50
TACAAAGTGG TGCGAATGTA GAAAGCACCG CGTACTGGTT ATACAGGTGA 100
<210> 11584
<211> 100
<212> DNA
<213> Escherichia coli K-12 MG1655
<220> wecd b3791
<222> 430-529
<223> wecd b3791 U00096 3972208..3972753
<313> 11584: 472-491
<400> 11584
CGTCGCGGTA AAACAACTTT GCGGGTGGCG ACCCAAATGG GCAACACCGC 50
CGCGCTTAAA CGATACATAC AAAGTGGTGC GAATGTAGAA AGCACCGCGT 100
<210> 11585
<211> 100
<212> DNA
<213> Escherichia coli K-12 MG1655
<220> wecd b3791
<222> 319-418
<223> wecd b3791 U00096 3972208..3972753
<313> 11585: 349-368
<400> 11585
CGCGGCTATG TCTCTTTACG GGAACTCAAT GCGACAGATG CGCGAATTGG 50
CCTGCTGGCT GGACGCGGTG CAGGTGCTGA GCTGATGCAA ACGGCGCTAA 100
<210> 11586
<211> 100
<212> DNA
<213> Escherichia coli K-12 MG1655
<220> wece b3792
<222> 1019-1118

<223> wece b3792 U00096 3972758..3973888
<313> 11586: 1073-1092
<400> 11586
ATCGCTACAC CACCAAAGAG AGCGAGCGCC TGCTGCGCCT GCCGCTGTTC 50
TACAACCTGT CGCCCGTCAA TCAGCGTACG GTAATTGCGA CTTTGTTGAA 100
<210> 11587
<211> 100
<212> DNA
<213> Escherichia coli K-12 MG1655
<220> wece b3792
<222> 967-1066
<223> wece b3792 U00096 3972758..3973888
<313> 11587: 1018-1037
<400> 11587
CCGCTGCACG GTTGCCCTGC GGGGGAACAC TTTGGTGAGT TCCACGGTGA 50
AGATCGCTAC ACCACCAAAG AGAGCGAGCG CCTGCTGCGC CTGCCGCTGT 100
<210> 11588
<211> 100
<212> DNA
<213> Escherichia coli K-12 MG1655
<220> wece b3792
<222> 927-1026
<223> wece b3792 U00096 3972758..3973888
<313> 11588: 963-982
<400> 11588
TCTGAAAGAA GCGGAAATCA TGGCGGTGTT TCATTACATT CCGCTGCACG 50
GTTGCCCTGC GGGGGAACAC TTTGGTGAGT TCCACGGTGA AGATCGCTAC 100
<210> 11589
<211> 100
<212> DNA
<213> Escherichia coli K-12 MG1655
<220> wecf b3794
<222> 1247-1346
<223> wecf b3794 U00096 3976214..3977566
<313> 11589: 1297-1316
<400> 11589
TCGGCGCATG TCTGATGATC GCAAAACTGT TGTACTGGCT TTTTGAAAGC 50
GCCGGACTCA TTCATAAACG TACAAAATCA TCGCTCCGGA CGCAGGTTGA 100
<210> 11590
<211> 100
<212> DNA
<213> Escherichia coli K-12 MG1655
<220> wecf b3794
<222> 1222-1321
<223> wecf b3794 U00096 3976214..3977566
<313> 11590: 1266-1285
<400> 11590
CGCGTGGTCT TTTTTATCGT GGTCTTCGGC GCATGTCTGA TGATCGCAAA 50
ACTGTTGTAC TGGCTTTTTG AAAGCGCCGG ACTCATTCAT AAACGTACAA 100
<210> 11591
<211> 100
<212> DNA
<213> Escherichia coli K-12 MG1655
<220> wecf b3794
<222> 1094-1193
<223> wecf b3794 U00096 3976214..3977566
<313> 11591: 1127-1146
<400> 11591
ACTGGCTGTA TGAGCTGGGC AACCGCGAGC CTAATCGCTA TAAAGCTGCG 50
ATATTGCACA GTTTCTGCTT TGGGGCGATC TTCAATATGA TCGTGCTGGC 100
<210> 11592
<211> 100
<212> DNA
<213> Escherichia coli K-12 MG1655
<220> wecg b3795
<222> 642-741
<223> wecg b3795 U00096 3977569..3978309
<313> 11592: 711-730
<400> 11592
GCTGGAGTGG CTCTACCGCC TGCTTTCGCA GCCGAGCCGC ATTAAGCGTC 50
AGCTTCGTTT GCTGCGTTAT TTACGCTGGC ACTACACCGG CAACCTATGA 100

<210> 11593
<211> 100
<212> DNA
<213> Escherichia coli K-12 MG1655
<220> wecg b3795
<222> 611-710
<223> wecg b3795 U00096 3977569..3978309
<313> 11593: 661-680
<400> 11593
AACGCGCACC GAAAATCTGG CAAACGCTGG GGCTGGAGTG GCTCTACCGC 50
CTGCTTTCGC AGCCGAGCCG CATTAAGCGT CAGCTTCGTT TGCTGCGTTA 100
<210> 11594
<211> 100
<212> DNA
<213> Escherichia coli K-12 MG1655
<220> wecg b3795
<222> 591-690
<223> wecg b3795 U00096 3977569..3978309
<313> 11594: 629-648
<400> 11594
TGTTTTCACC GGTCACGTAA AACGCGCACC GAAAATCTGG CAAACGCTGG 50
GGCTGGAGTG GCTCTACCGC CTGCTTTCGC AGCCGAGCCG CATTAAGCGT 100
<210> 11595
<211> 100
<212> DNA
<213> Escherichia coli K-12 MG1655
<220> b4103 b4104
<222> 123-222
<223> b4103 b4104 U00096 complement(4320021..4320242)
<313> 11595: 180-199
<400> 11595
TTTCCAGTTC CAACAAACCT GCGCCCTGAT GGTGCTTATC ATCGTCACGG 50
TCAGCCTGCT GGATTTCCTC TCTCAACGGT TGCGTAAGCA CTTTATCTGA 100
<210> 11596
<211> 100
<212> DNA
<213> Escherichia coli K-12 MG1655
<220> b4103 b4104
<222> 101-200
<223> b4103 b4104 U00096 complement(4320021..4320242)
<313> 11596: 140-159
<400> 11596
CCCTGTGGGA AGCGATTCGC GGTTTCCAGT TCCAACAAAC CTGCGCCCTG 50
ATGGTGCTTA TCATCGTCAC GGTCAGCCTG CTGGATTTCC TCTCTCAACG 100
<210> 11597
<211> 100
<212> DNA
<213> Escherichia coli K-12 MG1655
<220> b4103 b4104
<222> 1-100
<223> b4103 b4104 U00096 complement(4320021..4320242)
<313> 11597: 46-65
<400> 11597
GTGATGCCAC TGCTGATCTC CTACTCCCTC TATCGCTTCG AATCCAACGT 50
CCGCTCGGCG ACCGTCGTCG GCATGGTCGG CGCAGGCGGG ATCGGCGTCA 100
<210> 11598
<211> 100
<212> DNA
<213> Escherichia coli K-12 MG1655
<220> b0540 b0541
<222> 120-219
<223> b0540 b0541 U00096 566056..566364
<313> 11598: 148-167
<400> 11598
TGAACTCAGC CTGTATGAAT CACAACTCTA CAACTGGCGC AGTAAACAGC 50
AAAATCAGCA GACGTCTTCT GAACGTGAAC TGGAGATGTC TACCGAGATT 100
<210> 11599
<211> 100
<212> DNA
<213> Escherichia coli K-12 MG1655
<220> b0540 b0541

<222> 61-160
<223> b0540 b0541 U00096 566056..566364
<313> 11599: 112-131
<400> 11599
GAATTTCGCA GTGAAGCCCT GAAGCTTGCT GAACGCATCG GTGTTACTGC  50
CGCAGCCCGT GAACTCAGCC TGTATGAATC ACAACTCTAC AACTGGCGCA  100
<210> 11600
<211> 100
<212> DNA
<213> Escherichia coli K-12 MG1655
<220> b0540 b0541
<222> 24-123
<223> b0540 b0541 U00096 566056..566364
<313> 11600: 73-92
<400> 11600
ATCAACCAGT AAAAAACCCC GTAAACAGCA TTCGCCTGAA TTTCGCAGTG  50
AAGCCCTGAA GCTTGCTGAA CGCATCGGTG TTACTGCCGC AGCCCGTGAA  100
<210> 11601
<211> 100
<212> DNA
<213> Escherichia coli K-12 MG1655
<220> tmk b1099
<222> 543-642
<223> tmk b1099 U00096 1154347..1154988
<313> 11601: 606-625
<400> 11601
TAAAAGCATT CATACCATTG ATGCCACCCA GCCGCTGGAG GCCGTGATGG  50
ATGCAATCCG CACTACCGTG ACCCACTGGG TGAAGGAGTT GGACGCATGA  100
<210> 11602
<211> 100
<212> DNA
<213> Escherichia coli K-12 MG1655
<220> tmk b1099
<222> 540-639
<223> tmk b1099 U00096 1154347..1154988
<313> 11602: 569-588
<400> 11602
AGATAAAAGC ATTCATACCA TTGATGCCAC CCAGCCGCTG GAGGCCGTGA  50
TGGATGCAAT CCGCACTACC GTGACCCACT GGGTGAAGGA GTTGGACGCA  100
<210> 11603
<211> 100
<212> DNA
<213> Escherichia coli K-12 MG1655
<220> tmk b1099
<222> 477-576
<223> tmk b1099 U00096 1154347..1154988
<313> 11603: 522-541
<400> 11603
TGAGCAAGAA TCTTTCGATT TCTTTAATCG CACCCGCGCC CGCTATCTGG  50
AACTGGCAGC ACAAGATAAA AGCATTCATA CCATTGATGC CACCCAGCCG  100
<210> 11604
<211> 100
<212> DNA
<213> Escherichia coli K-12 MG1655
<220> b0542 b0543
<222> 39-138
<223> b0542 b0543 U00096 567333..567470
<313> 11604: 114-133
<400> 11604
AAAGGTGAGC ACGAATCTGA TTTTTAAGGA GTGTCGCCAG AGTGCCGCGA  50
TGAAACGGGT ATTGAGGGTA TATAAAAGAA CATCAATGGG AACACAATGA  100
<210> 11605
<211> 100
<212> DNA
<213> Escherichia coli K-12 MG1655
<220> b0542 b0543
<222> 39-138
<223> b0542 b0543 U00096 567333..567470
<313> 11605: 97-116
<400> 11605
AAAGGTGAGC ACGAATCTGA TTTTTAAGGA GTGTCGCCAG AGTGCCGCGA  50

TGAAACGGGT ATTGAGGGTA TATAAAAGAA CATCAATGGG AACACAATGA 100
<210> 11606
<211> 100
<212> DNA
<213> Escherichia coli K-12 MG1655
<220> b0542 b0543
<222> 39-138
<223> b0542 b0543 U00096 567333..567470
<313> 11606: 88-107
<400> 11606
AAAGGTGAGC ACGAATCTGA TTTTTAAGGA GTGTCGCCAG AGTGCCGCGA 50
TGAAACGGGT ATTGAGGGTA TATAAAAGAA CATCAATGGG AACACAATGA 100
<210> 11607
<211> 100
<212> DNA
<213> Escherichia coli K-12 MG1655
<220> naga b0678
<222> 1021-1120
<223> naga b0678 U00096 complement(700826..701974)
<313> 11607: 1079-1098
<400> 11607
CGTGCGATTG GCGTTGAGAA ACGTCTCGGC ACACTCGCCG CAGGTAAAGT 50
AGCCAACCTG ACTGCATTCA CACCTGATTT TAAAATCACC AAGACCATCG 100
<210> 11608
<211> 100
<212> DNA
<213> Escherichia coli K-12 MG1655
<220> naga b0678
<222> 929-1028
<223> naga b0678 U00096 complement(700826..701974)
<313> 11608: 989-1008
<400> 11608
CCTTAACCAT GATTGAAGGC GTGCGTAATC TGGTCGAACA TTGCGGTATC 50
GCACTGGATG AAGTGCTACG TATGGCGACG CTCTATCCGG CGCGTGCGAT 100
<210> 11609
<211> 100
<212> DNA
<213> Escherichia coli K-12 MG1655
<220> naga b0678
<222> 893-992
<223> naga b0678 U00096 complement(700826..701974)
<313> 11609: 928-947
<400> 11609
TTTGTGTGGA TGAGAACGGT ACGTTAAGCG GTTCATCCTT AACCATGATT 50
GAAGGCGTGC GTAATCTGGT CGAACATTGC GGTATCGCAC TGGATGAAGT 100
<210> 11610
<211> 100
<212> DNA
<213> Escherichia coli K-12 MG1655
<220> nagb b0679
<222> 529-628
<223> nagb b0679 U00096 complement(702034..702834)
<313> 11610: 574-593
<400> 11610
GATGTTAATC AGGTGCCAAA ATATGCCCTG ACTGTCGGTG TTGGTACACT 50
GCTGGATGCC GAAGAAGTGA TGATTCTGGT GCTGGGTAGC CAGAAAGCAC 100
<210> 11611
<211> 100
<212> DNA
<213> Escherichia coli K-12 MG1655
<220> nagb b0679
<222> 461-560
<223> nagb b0679 U00096 complement(702034..702834)
<313> 11611: 500-519
<400> 11611
CTTCTCGTAC TCGTATCAAA ACCCTGACTC ATGACACTCG CGTCGCAAAC 50
TCTCGTTTCT TTGATAACGA TGTTAATCAG GTGCCAAAAT ATGCCCTGAC 100
<210> 11612
<211> 100
<212> DNA
<213> Escherichia coli K-12 MG1655

<220> nagb b0679
<222> 404-503
<223> nagb b0679 U00096 complement(702034..702834)
<313> 11612: 467-486
<400> 11612
TGGGCGGTGT AGGTAACGAC GGTCATATTG CATTTAACGA ACCGGCGTCT  50
TCTCTGGCTT CTCGTACTCG TATCAAAACC CTGACTCATG ACACTCGCGT  100
<210> 11613
<211> 100
<212> DNA
<213> Escherichia coli K-12 MG1655
<220> nagc b0677
<222> 1122-1221
<223> nagc b0677 U00096 complement(699597..700817)
<313> 11613: 1180-1199
<400> 11613
ACGTTCTGAG CTGGATCACC GCTCGGCAAT CGGCGCTTTT GCGCTGGTAA  50
AACGCGCCAT GCTCAACGGT ATTTTGCTCC AGCATTTGCT GGAAAATTAA  100
<210> 11614
<211> 100
<212> DNA
<213> Escherichia coli K-12 MG1655
<220> nagc b0677
<222> 1036-1135
<223> nagc b0677 U00096 complement(699597..700817)
<313> 11614: 1090-1109
<400> 11614
GAAGCCGATA AAGTGCTGCT CCCTGCTATT GAAAGCTGCA TTAATACCCA  50
GGCGCTGAAG GCGTTTCGCA CTAATCTGCC GGTGGTACGT TCTGAGCTGG  100
<210> 11615
<211> 100
<212> DNA
<213> Escherichia coli K-12 MG1655
<220> nagc b0677
<222> 994-1093
<223> nagc b0677 U00096 complement(699597..700817)
<313> 11615: 1053-1072
<400> 11615
TTATTTAATC CGCAAAAAAT TGTTATTGCC GGTGAAATCA CCGAAGCCGA  50
TAAAGTGCTG CTCCCTGCTA TTGAAAGCTG CATTAATACC CAGGCGCTGA  100
<210> 11616
<211> 100
<212> DNA
<213> Escherichia coli K-12 MG1655
<220> nagd b0676
<222> 654-753
<223> nagd b0676 U00096 complement(698797..699549)
<313> 11616: 718-737
<400> 11616
TCTGGTGCTT TCTGGTGTTT CGTCGCTCGA CGATATCGAC AGTATGCCTT  50
TCCGCCCCAG CTGGATTTAC CCGTCAGTCG CTGAAATCGA CGTTATCTGA  100
<210> 11617
<211> 100
<212> DNA
<213> Escherichia coli K-12 MG1655
<220> nagd b0676
<222> 627-726
<223> nagd b0676 U00096 complement(698797..699549)
<313> 11617: 647-666
<400> 11617
CGGCTTCCAG GCAGGTCTGG AGACGATTCT GGTGCTTTCT GGTGTTTCGT  50
CGCTCGACGA TATCGACAGT ATGCCTTTCC GCCCCAGCTG GATTTACCCG  100
<210> 11618
<211> 100
<212> DNA
<213> Escherichia coli K-12 MG1655
<220> nagd b0676
<222> 535-634
<223> nagd b0676 U00096 complement(698797..699549)
<313> 11618: 573-592
<400> 11618

CCGTGGATCA TCCGCGCAGC ATTAAACAAA ATGCAGGCGC ATTCGGAAGA 50
AACGGTGATT GTCGGCGATA ACCTGCGTAC CGATATTCTG GCCGGCTTCC 100
<210> 11619
<211> 100
<212> DNA
<213> Escherichia coli K-12 MG1655
<220> nage b0680
<222> 1848-1947
<223> nage b0680 U00096 703167..705113
<313> 11619: 1892-1911
<400> 11619
GGTGGTTTGC AGCAATATCG ACGATTTCAG TGGCTTGATC ATTAAAGCTC 50
AGGGCCATAT TGTGGCGGGT CAAACACCGC TGTATGAAAT CAAAAAGTAA 100
<210> 11620
<211> 100
<212> DNA
<213> Escherichia coli K-12 MG1655
<220> nage b0680
<222> 1808-1907
<223> nage b0680 U00096 703167..705113
<313> 11620: 1859-1878
<400> 11620
TGGATTACCT GAACGCTAAC GCCCGCTCGA TGATTAGCCC GGTGGTTTGC 50
AGCAATATCG ACGATTTCAG TGGCTTGATC ATTAAAGCTC AGGGCCATAT 100
<210> 11621
<211> 100
<212> DNA
<213> Escherichia coli K-12 MG1655
<220> nage b0680
<222> 1657-1756
<223> nage b0680 U00096 703167..705113
<313> 11621: 1675-1694
<400> 11621
AACCACGCGT TCTGCCTGGA AACCGAAAAA GGCGCGGAGA TCGTCGTCCA 50
TATGGGTATC GACACCGTAG CGCTGGAAGG TAAAGGCTTT AAACGTCTGG 100
<210> 11622
<211> 100
<212> DNA
<213> Escherichia coli K-12 MG1655
<220> appa b0981
<222> 1200-1299
<223> appa b0981 U00096 1039840..1041138
<313> 11622: 1248-1267
<400> 11622
CCTGGCAGGA TGTGAAGAGC GAAATGCGCA GGGCATGTGT TCGTTGGCAG 50
GTTTTACGCA AATCGTGAAT GAAGCACGCA TACCGGCGTG CAGTTTGTAA 100
<210> 11623
<211> 100
<212> DNA
<213> Escherichia coli K-12 MG1655
<220> appa b0981
<222> 1165-1264
<223> appa b0981 U00096 1039840..1041138
<313> 11623: 1200-1219
<400> 11623
TCATTAAATA CGCCGCCCGG AGAGGTGAAA CTGACCCTGG CAGGATGTGA 50
AGAGCGAAAT GCGCAGGGCA TGTGTTCGTT GGCAGGTTTT ACGCAAATCG 100
<210> 11624
<211> 100
<212> DNA
<213> Escherichia coli K-12 MG1655
<220> appa b0981
<222> 1109-1208
<223> appa b0981 U00096 1039840..1041138
<313> 11624: 1160-1179
<400> 11624
TTCAGGTTTC GCTGGTCTTC CAGACTTTAC AGCAGATGCG TGATAAAACG 50
CCGCTGTCAT TAAATACGCC GCCCGGAGAG GTGAAACTGA CCCTGGCAGG 100
<210> 11625
<211> 100
<212> DNA

<213> Escherichia coli K-12 MG1655
<220> appb b0980
<222> 1038-1137
<223> appb b0980 U00096 1038519..1039655
<313> 11625: 1104-1123
<400> 11625
TTTGCCCATT GTGTTGCTCT ACACTCTCTG GAGCTACTAC AAAATGTGGG 50
GGCGCATGAC AACAGAAACT CTCCGCCGTA ACGAAAACGA GTTGTACTAA 100
<210> 11626
<211> 100
<212> DNA
<213> Escherichia coli K-12 MG1655
<220> appb b0980
<222> 998-1097
<223> appb b0980 U00096 1038519..1039655
<313> 11626: 1041-1060
<400> 11626
AGCTGACGCT GAGCATTATG TTGGTAATCG TGCTGATATT TTTGCCCATT 50
GTGTTGCTCT ACACTCTCTG GAGCTACTAC AAAATGTGGG GGCGCATGAC 100
<210> 11627
<211> 100
<212> DNA
<213> Escherichia coli K-12 MG1655
<220> appb b0980
<222> 923-1022
<223> appb b0980 U00096 1038519..1039655
<313> 11627: 975-994
<400> 11627
CGCTGTTCCC CTTTGTCATG CCGTCAAGCG TGAGTCCGAT CTCCAGCCTG 50
ACGTTGTGGG ACAGTACTTC CAGTCAGCTG ACGCTGAGCA TTATGTTGGT 100
<210> 11628
<211> 100
<212> DNA
<213> Escherichia coli K-12 MG1655
<220> appc b0979
<222> 1446-1545
<223> appc b0979 U00096 1036963..1038507
<313> 11628: 1510-1529
<400> 11628
CACCCTGTTC TTAATCGCCG AAGTCTACCT GATGCAGAAA TATGCCCGTC 50
TGGGGCCGAG CGCGATGCAG AGTGAACAAC CGACGCAGCA ACAGGGGTAA 100
<210> 11629
<211> 100
<212> DNA
<213> Escherichia coli K-12 MG1655
<220> appc b0979
<222> 1436-1535
<223> appc b0979 U00096 1036963..1038507
<313> 11629: 1486-1505
<400> 11629
TAGGGCTTTA CACCCTGTTC TTAATCGCCG AAGTCTACCT GATGCAGAAA 50
TATGCCCGTC TGGGGCCGAG CGCGATGCAG AGTGAACAAC CGACGCAGCA 100
<210> 11630
<211> 100
<212> DNA
<213> Escherichia coli K-12 MG1655
<220> appc b0979
<222> 1380-1479
<223> appc b0979 U00096 1036963..1038507
<313> 11630: 1406-1425
<400> 11630
CTCCGCGCAC TCCGCTTTAA CCACAGGACA ACTGGCTTTC TCACTGATCA 50
TGATCGTAGG GCTTTACACC CTGTTCTTAA TCGCCGAAGT CTACCTGATG 100
<210> 11631
<211> 100
<212> DNA
<213> Escherichia coli K-12 MG1655
<220> b1284 b1285
<222> 651-750
<223> b1284 b1285 U00096 complement(1341621..1342370)
<313> 11631: 717-736

<400> 11631
TGAGCGATTC AATCGCGTGA TTACCGATTC GAAAATACGC GCCAGCGATC 50
TGATGCATCT TGAGCACAGT AAACTCACCA TTCACGTCGT TGACATTTAA 100
<210> 11632
<211> 100
<212> DNA
<213> Escherichia coli K-12 MG1655
<220> b1284 b1285
<222> 580-679
<223> b1284 b1285 U00096 complement(1341621..1342370)
<313> 11632: 631-650
<400> 11632
GAGTGCGAAG CGATAGTCCT GACTGACAGC TCGAAATTTG GTGCTGTACA 50
TTCATACTCC ATCGGTCCCG TTGAGCGATT CAATCGCGTG ATTACCGATT 100
<210> 11633
<211> 100
<212> DNA
<213> Escherichia coli K-12 MG1655
<220> b1284 b1285
<222> 554-653
<223> b1284 b1285 U00096 complement(1341621..1342370)
<313> 11633: 587-606
<400> 11633
ATGTGGTCAA TGCCGTGCTG GAAAAAGAGT GCGAAGCGAT AGTCCTGACT 50
GACAGCTCGA AATTTGGTGC TGTACATTCA TACTCCATCG GTCCCGTTGA 100
<210> 11634
<211> 100
<212> DNA
<213> Escherichia coli K-12 MG1655
<220> appy b0565
<222> 651-750
<223> appy b0565 U00096 582904..583653
<313> 11634: 712-731
<400> 11634
TTTCATATGT GCATTTAAAG ATTATTATGG TGTCACGCCA TCTCATTATT 50
TTGAGAAAAT AATCGGCGTC ACAGATGGAA TAAACAAAAC AATTGACTGA 100
<210> 11635
<211> 100
<212> DNA
<213> Escherichia coli K-12 MG1655
<220> appy b0565
<222> 651-750
<223> appy b0565 U00096 582904..583653
<313> 11635: 702-721
<400> 11635
TTTCATATGT GCATTTAAAG ATTATTATGG TGTCACGCCA TCTCATTATT 50
TTGAGAAAAT AATCGGCGTC ACAGATGGAA TAAACAAAAC AATTGACTGA 100
<210> 11636
<211> 100
<212> DNA
<213> Escherichia coli K-12 MG1655
<220> appy b0565
<222> 547-646
<223> appy b0565 U00096 582904..583653
<313> 11636: 613-632
<400> 11636
ATATTGAGAG ATACTAGGAT GAGGTATGCA AAAAAACTCA TAACTTCAAA 50
CTCTTATTCT ATCAATGTCG TAGCCCAGAA ATGTGGCTAT AACAGTACTT 100
<210> 11637
<211> 100
<212> DNA
<213> Escherichia coli K-12 MG1655
<220> gutm b2707
<222> 261-360
<223> gutm b2707 U00096 2826644..2827003
<313> 11637: 319-338
<400> 11637
CGGTATGCAT GCGGGTGATT TACAGCCCGA TGTGATCTTT CCCCATGATC 50
CACTATCACA GAATGCTCTA TCATTGGCGC TTAAACTGAA ACGTGGATAA 100
<210> 11638
<211> 100

<212> DNA
<213> Escherichia coli K-12 MG1655
<220> gutm b2707
<222> 256-355
<223> gutm b2707 U00096 2826644..2827003
<313> 11638: 294-313
<400> 11638
ATTACCGGTA TGCATGCGGG TGATTTACAG CCCGATGTGA TCTTTCCCCA 50
TGATCCACTA TCACAGAATG CTCTATCATT GGCGCTTAAA CTGAAACGTG 100
<210> 11639
<211> 100
<212> DNA
<213> Escherichia coli K-12 MG1655
<220> gutm b2707
<222> 183-282
<223> gutm b2707 U00096 2826644..2827003
<313> 11639: 228-247
<400> 11639
TCAGCAGCGC ATCGTCGACA CCTTGTTTAT GAAAGGACTG ACCGTCTTCG 50
CCCGACCGCA AAAAATTCCC GCAATTACCG GTATGCATGC GGGTGATTTA 100
<210> 11640
<211> 100
<212> DNA
<213> Escherichia coli K-12 MG1655
<220> gutq b2709
<222> 828-927
<223> gutq b2709 U00096 2827875..2828801
<313> 11640: 874-893
<400> 11640
GATGAAGCGC AAAATCACTG CCGCACCGGT GGTGGATGAA AACGGCAAAC 50
TCACCGGCGC AATAAACCTG CAGGATTTCT ATCAGGCCGG GATTATTTAA 100
<210> 11641
<211> 100
<212> DNA
<213> Escherichia coli K-12 MG1655
<220> gutq b2709
<222> 805-904
<223> gutq b2709 U00096 2827875..2828801
<313> 11641: 822-841
<400> 11641
GCCATCGACG CCAAAGAGAT CCTGATGAAG CGCAAAATCA CTGCCGCACC 50
GGTGGTGGAT GAAAACGGCA AACTCACCGG CGCAATAAAC CTGCAGGATT 100
<210> 11642
<211> 100
<212> DNA
<213> Escherichia coli K-12 MG1655
<220> gutq b2709
<222> 720-819
<223> gutq b2709 U00096 2827875..2828801
<313> 11642: 751-770
<400> 11642
CTGGCTGGTT GGCGGCGGCG CACTCACCAC GCCAGTCAAT GAAGCGATGA 50
CGGTCGGCGG CACCACGTTG CAATCGCAAA GTCGCGCCAT CGACGCCAAA 100
<210> 11643
<211> 100
<212> DNA
<213> Escherichia coli K-12 MG1655
<220> b1297 b1298
<222> 1398-1497
<223> b1297 b1298 U00096 complement(1357514..1359010)
<313> 11643: 1458-1477
<400> 11643
CTTCTGCCAT GTCTATCACG CCTGTAAAAA TGATGAGCTG TTGCAGTTTG 50
AGCGCCTCAT CACAGAAACA GAAATTGAGT GGATGTTGAA AAACGCGTAA 100
<210> 11644
<211> 100
<212> DNA
<213> Escherichia coli K-12 MG1655
<220> b1297 b1298
<222> 1378-1477
<223> b1297 b1298 U00096 complement(1357514..1359010)

<313> 11644: 1422-1441
<400> 11644
CGCCGCTATT TAGGCGAACG CTTCTGCCAT GTCTATCACG CCTGTAAAAA 50
TGATGAGCTG TTGCAGTTTG AGCGCCTCAT CACAGAAACA GAAATTGAGT 100
<210> 11645
<211> 100
<212> DNA
<213> Escherichia coli K-12 MG1655
<220> b1297 b1298
<222> 1334-1433
<223> b1297 b1298 U00096 complement(1357514..1359010)
<313> 11645: 1377-1396
<400> 11645
GCCAGAGCGA TGCCCTGGGT GAGTTTATCG AGAATGATCA CCTGCGCCGC 50
TATTTAGGCG AACGCTTCTG CCATGTCTAT CACGCCTGTA AAAATGATGA 100
<210> 11646
<211> 100
<212> DNA
<213> Escherichia coli K-12 MG1655
<220> pyrb b4246
<222> 837-936
<223> pyrb b4246 U00096 complement(4469034..4469969)
<313> 11646: 885-904
<400> 11646
TAAAACGCCA CACGCCTGGT ACTTCCAGCA GGCAGGCAAC GGGATTTTCG 50
CTCGCCAGGC GTTACTGGCA CTGGTTCTGA ATCGCGATCT GGTACTGTAA 100
<210> 11647
<211> 100
<212> DNA
<213> Escherichia coli K-12 MG1655
<220> pyrb b4246
<222> 799-898
<223> pyrb b4246 U00096 complement(4469034..4469969)
<313> 11647: 855-874
<400> 11647
CCGCTGCCGC GTGTTGATGA GATTGCGACG GATGTTGATA AAACGCCACA 50
CGCCTGGTAC TTCCAGCAGG CAGGCAACGG GATTTTCGCT CGCCAGGCGT 100
<210> 11648
<211> 100
<212> DNA
<213> Escherichia coli K-12 MG1655
<220> pyrb b4246
<222> 752-851
<223> pyrb b4246 U00096 complement(4469034..4469969)
<313> 11648: 817-836
<400> 11648
GCGCCAGCGA TCTCCACAAC GCCAAAGCCA ATATGAAAGT GCTGCATCCG 50
CTGCCGCGTG TTGATGAGAT TGCGACGGAT GTTGATAAAA CGCCACACGC 100
<210> 11649
<211> 100
<212> DNA
<213> Escherichia coli K-12 MG1655
<220> pyrc b1063
<222> 948-1047
<223> pyrc b1063 U00096 complement(1120784..1121830)
<313> 11649: 1023-1042
<400> 11649
ACGTGAAGAG CAACAGGTTG CTGAAAGCAT CGCACTGACT GATGACACGC 50
TGGTGCCATT CCTCGCCGGG GAAACGGTAC GCTGGTCCGT TAAACAATAA 100
<210> 11650
<211> 100
<212> DNA
<213> Escherichia coli K-12 MG1655
<220> pyrc b1063
<222> 919-1018
<223> pyrc b1063 U00096 complement(1120784..1121830)
<313> 11650: 968-987
<400> 11650
CCGGTCAACG ACACATTCAT CGAACTGGTA CGTGAAGAGC AACAGGTTGC 50
TGAAAGCATC GCACTGACTG ATGACACGCT GGTGCCATTC CTCGCCGGGG 100
<210> 11651

<211> 100
<212> DNA
<213> Escherichia coli K-12 MG1655
<220> pyrc b1063
<222> 910-1009
<223> pyrc b1063 U00096 complement(1120784..1121830)
<313> 11651: 940-959
<400> 11651
TATGGGTTGC CGGTCAACGA CACATTCATC GAACTGGTAC GTGAAGAGCA 50
ACAGGTTGCT GAAAGCATCG CACTGACTGA TGACACGCTG GTGCCATTCC 100
<210> 11652
<211> 100
<212> DNA
<213> Escherichia coli K-12 MG1655
<220> pyrd b0946
<222> 821-920
<223> pyrd b0946 U00096 1003991..1005001
<313> 11652: 855-874
<400> 11652
AAAGCACCGA AATTATTCGC CGCTTGTCAC TGGAATTAAA CGGTCGCTTA 50
CCGATCATCG GTGTTGGCGG CATCGACTCG GTTATCGCTG CGCGTGAAAA 100
<210> 11653
<211> 100
<212> DNA
<213> Escherichia coli K-12 MG1655
<220> pyrd b0946
<222> 628-727
<223> pyrd b0946 U00096 1003991..1005001
<313> 11653: 661-680
<400> 11653
AAATATGTGC CGATCGCAGT GAAGATCGCG CCGGATCTTT CTGAAGAAGA 50
ATTGATCCAG GTTGCCGATA GTTTAGTTCG CCATAATATT GATGGCGTTA 100
<210> 11654
<211> 100
<212> DNA
<213> Escherichia coli K-12 MG1655
<220> pyrd b0946
<222> 481-580
<223> pyrd b0946 U00096 1003991..1005001
<313> 11654: 541-560
<400> 11654
AAAATCTATG CCTATGCGGG ATATATCGCC ATCAATATTT CATCGCCGAA 50
TACCCCAGGA TTACGCACGC TGCAATATGG TGAAGCGCTG GATGATCTCT 100
<210> 11655
<211> 100
<212> DNA
<213> Escherichia coli K-12 MG1655
<220> pyre b3643
<222> 522-621
<223> pyre b3643 U00096 complement(3812754..3813395)
<313> 11655: 566-585
<400> 11655
CAACTGCAAA GTGATCTCTA TCATCACCCT GAAAGACCTG ATTGCTTACC 50
TGGAAGAGAA GCCGGAAATG GCGGAACATC TGGCGGCGGT TAAGGCCTAT 100
<210> 11656
<211> 100
<212> DNA
<213> Escherichia coli K-12 MG1655
<220> pyre b3643
<222> 377-476
<223> pyre b3643 U00096 complement(3812754..3813395)
<313> 11656: 414-433
<400> 11656
TGATCACCGC CGGAACGGCG ATTCGCGAGT CGATGGAGAT TATTCAGGCC 50
AATGGCGCGA CGCTTGCTGG CGTGTTGATT TCGCTCGATC GTCAGGAACG 100
<210> 11657
<211> 100
<212> DNA
<213> Escherichia coli K-12 MG1655
<220> pyre b3643
<222> 260-359

<223> pyre b3643 U00096 complement(3812754..3813395)
<313> 11657: 306-325
<400> 11657
AGCATCACGA CCTGGACCTG CCGTACTGCT TTAACCGCAA AGAAGCAAAA 50
GACCACGGTG AAGGCGGCAA TCTGGTTGGT AGCGCGTTAC AAGGACGCGT 100
<210> 11658
<211> 100
<212> DNA
<213> Escherichia coli K-12 MG1655
<220> pyrf b1282
<222> 639-738
<223> pyrf b1282 U00096 1339945..1340682
<313> 11658: 719-738
<400> 11658
TGGTGTTGAT TATATGGTGA TTGGTCGCCC GGTAACGCAA TCGGTAGATC 50
CAGCGCAGAC GCTGAAAGCG ATCAACGCCT CTTTACAGCG GAGTGCATGA 100
<210> 11659
<211> 100
<212> DNA
<213> Escherichia coli K-12 MG1655
<220> pyrf b1282
<222> 628-727
<223> pyrf b1282 U00096 1339945..1340682
<313> 11659: 666-685
<400> 11659
GCGTTGTCGG CTGGTGTTGA TTATATGGTG ATTGGTCGCC CGGTAACGCA 50
ATCGGTAGAT CCAGCGCAGA CGCTGAAAGC GATCAACGCC TCTTTACAGC 100
<210> 11660
<211> 100
<212> DNA
<213> Escherichia coli K-12 MG1655
<220> pyrf b1282
<222> 516-615
<223> pyrf b1282 U00096 1339945..1340682
<313> 11660: 579-598
<400> 11660
AGCTGTGCGC TTTAAACAGG TATTCGGTCA GGAGTTCAAA CTGGTTACGC 50
CGGGCATTCG TCCGCAGGGG AGTGAAGCTG GTGACCAGCG CCGCATTATG 100
<210> 11661
<211> 100
<212> DNA
<213> Escherichia coli K-12 MG1655
<220> eno b2780
<222> 1200-1299
<223> eno b2780 U00096 complement(2904665..2905963)
<313> 11661: 1241-1260
<400> 11661
TGACCGTGTT GCTAAATACA ACCAGCTGAT TCGTATCGAA GAAGCTCTGG 50
GCGAAAAAGC ACCGTACAAC GGTCGTAAAG AGATCAAAGG CCAGGCATAA 100
<210> 11662
<211> 100
<212> DNA
<213> Escherichia coli K-12 MG1655
<220> eno b2780
<222> 1167-1266
<223> eno b2780 U00096 complement(2904665..2905963)
<313> 11662: 1192-1211
<400> 11662
AGGCCAGATC AAAACTGGTT CTATGAGCCG TTCTGACCGT GTTGCTAAAT 50
ACAACCAGCT GATTCGTATC GAAGAAGCTC TGGGCGAAAA AGCACCGTAC 100
<210> 11663
<211> 100
<212> DNA
<213> Escherichia coli K-12 MG1655
<220> eno b2780
<222> 1108-1207
<223> eno b2780 U00096 complement(2904665..2905963)
<313> 11663: 1144-1163
<400> 11663
CACCGTTCTG GCGAAACTGA AGACGCTACC ATCGCTGACC TGGCTGTTGG 50
TACTGCTGCA GGCCAGATCA AAACTGGTTC TATGAGCCGT TCTGACCGTG 100

<210> 11664
<211> 100
<212> DNA
<213> Escherichia coli K-12 MG1655
<220> pyrg b2781
<222> 1539-1638
<223> pyrg b2781 U00096 complement(2906051..2907688)
<313> 11664: 1612-1631
<400> 11664
GTTCCATCCG GAGTTTACTT CTACTCCACG TGATGGTCAC CCGCTGTTTG 50
CAGGCTTTGT GAAAGCCGCC AGCGAGTTCC AGAAACGTCA GGCGAAGTAA 100
<210> 11665
<211> 100
<212> DNA
<213> Escherichia coli K-12 MG1655
<220> pyrg b2781
<222> 1377-1476
<223> pyrg b2781 U00096 complement(2906051..2907688)
<313> 11665: 1403-1422
<400> 11665
TGCGCCGACA ATTGTTGAGC GTCATCGTCA CCGTTACGAA GTCAACAACA 50
TGCTGTTGAA ACAGATTGAA GATGCAGGTC TGCGCGTTGC GGGCCGTTCC 100
<210> 11666
<211> 100
<212> DNA
<213> Escherichia coli K-12 MG1655
<220> pyrg b2781
<222> 1257-1356
<223> pyrg b2781 U00096 complement(2906051..2907688)
<313> 11666: 1277-1296
<400> 11666
GCGCGATGAA AACGGCAACG TTGAAGTTCG TAGCGAGAAG AGCGATCTCG 50
GCGGTACCAT GCGTCTCGGC GCACAGCAGT GCCAGTTGGT TGACGATAGC 100
<210> 11667
<211> 100
<212> DNA
<213> Escherichia coli K-12 MG1655
<220> pyrh b0172
<222> 603-702
<223> pyrh b0172 U00096 191855..192580
<313> 11667: 637-656
<400> 11667
CCTGGCGGCC TTCACGCTGG CTCGTGACCA TAAATTACCG ATTCGTGTTT 50
TCAATATGAA CAAACCGGGT GCGCTGCGCC GTGTGGTAAT GGGTGAAAAA 100
<210> 11668
<211> 100
<212> DNA
<213> Escherichia coli K-12 MG1655
<220> pyrh b0172
<222> 561-660
<223> pyrh b0172 U00096 191855..192580
<313> 11668: 585-604
<400> 11668
GACTTACAGC GAAGTGCTGG AAAAAGAGCT GAAAGTCATG GACCTGGCGG 50
CCTTCACGCT GGCTCGTGAC CATAAATTAC CGATTCGTGT TTTCAATATG 100
<210> 11669
<211> 100
<212> DNA
<213> Escherichia coli K-12 MG1655
<220> pyrh b0172
<222> 494-593
<223> pyrh b0172 U00096 191855..192580
<313> 11669: 555-574
<400> 11669
CCAAAGTTGA CGGCGTGTTT ACCGCTGATC CGGCGAAAGA TCCAACCGCA 50
ACCATGTACG AGCAACTGAC TTACAGCGAA GTGCTGGAAA AAGAGCTGAA 100
<210> 11670
<211> 100
<212> DNA
<213> Escherichia coli K-12 MG1655
<220> pyri b4245

<222> 363-462
<223> pyri b4245 U00096 complement(4468560..4469021)
<313> 11670: 405-424
<400> 11670
TTCATCCAGC TTTGCCGTGC GAAAACGCGC CAATGATATC GCGCTCAAAT 50
GCAAATACTG TGAAAAAGAG TTTTCCCATA ATGTGGTGCT GGCCAATTAA 100
<210> 11671
<211> 100
<212> DNA
<213> Escherichia coli K-12 MG1655
<220> pyri b4245
<222> 317-416
<223> pyri b4245 U00096 complement(4468560..4469021)
<313> 11671: 374-393
<400> 11671
TGCTGGTCTG CCCGAACAGC AACTGTATCA GCCATGCCGA ACCGGTTTCA 50
TCCAGCTTTG CCGTGCGAAA ACGCGCCAAT GATATCGCGC TCAAATGCAA 100
<210> 11672
<211> 100
<212> DNA
<213> Escherichia coli K-12 MG1655
<220> pyri b4245
<222> 292-391
<223> pyri b4245 U00096 complement(4468560..4469021)
<313> 11672: 337-356
<400> 11672
AGTCTGCCGG AGCGCATCGA CAATGTGCTG GTCTGCCCGA ACAGCAACTG 50
TATCAGCCAT GCCGAACCGG TTTCATCCAG CTTTGCCGTG CGAAAACGCG 100
<210> 11673
<211> 100
<212> DNA
<213> Escherichia coli K-12 MG1655
<220> pyrl b4247
<222> 36-135
<223> pyrl b4247 U00096 complement(4469973..4470107)
<313> 11673: 80-99
<400> 11673
TCTGAAAAAA GACGCTGGCC TGCCGTTTTT CTTCCCGTTG ATCACCCATT 50
CCCAGCCCCT CAATCGAGGG GCTTTTTTTT GCCCAGGCGT CAGGAGATAA 100
<210> 11674
<211> 100
<212> DNA
<213> Escherichia coli K-12 MG1655
<220> pyrl b4247
<222> 29-128
<223> pyrl b4247 U00096 complement(4469973..4470107)
<313> 11674: 73-92
<400> 11674
TACCGCGTCT GAAAAAAGAC GCTGGCCTGC CGTTTTTCTT CCCGTTGATC 50
ACCCATTCCC AGCCCCTCAA TCGAGGGGCT TTTTTTTGCC CAGGCGTCAG 100
<210> 11675
<211> 100
<212> DNA
<213> Escherichia coli K-12 MG1655
<220> pyrl b4247
<222> 4-103
<223> pyrl b4247 U00096 complement(4469973..4470107)
<313> 11675: 60-79
<400> 11675
GTTCAGTGTG TTCGACATTT TGTCTTACCG CGTCTGAAAA AAGACGCTGG 50
CCTGCCGTTT TTCTTCCCGT TGATCACCCA TTCCCAGCCC CTCAATCGAG 100
<210> 11676
<211> 100
<212> DNA
<213> Escherichia coli K-12 MG1655
<220> ucpa b2427
<222> 737-836
<223> ucpa b2427 U00096 complement(2541852..2542709)
<313> 11676: 762-781
<400> 11676
CGCTGGAAGT CGGCGAACTG GCGGCCTTCC TCGCATCGGA TGAATCCAGC 50

TATTTAACCG GTACACAGAA TGTGATTGAT GGCGGCAGCA CACTGCCGGA 100
<210> 11677
<211> 100
<212> DNA
<213> Escherichia coli K-12 MG1655
<220> ucpa b2427
<222> 614-713
<223> ucpa b2427 U00096 complement(2541852..2542709)
<313> 11677: 655-674
<400> 11677
TTTGCCCGGG ATACGTGCGC ACACCAATGG CGGAAAGCAT TGCCCGCCAG 50
TCGAACCCGG AAGATCCAGA GTCGGTGCTG ACTGAAATGG CGAAAGCAAT 100
<210> 11678
<211> 100
<212> DNA
<213> Escherichia coli K-12 MG1655
<220> ucpa b2427
<222> 405-504
<223> ucpa b2427 U00096 complement(2541852..2542709)
<313> 11678: 445-464
<400> 11678
TATTAAAGGC GTATGGAACG TCACGAAGGC GGTGCTGCCG GAGATGATTG 50
CCCGCAAAGA TGGTCGCATT GTGATGATGT CTTCAGTCAC TGGTGATATG 100
<210> 11679
<211> 100
<212> DNA
<213> Escherichia coli K-12 MG1655
<220> tpr b1230
<222> 1-100
<223> tpr b1230 U00096 complement(1286310..1286411)
<313> 11679: 41-60
<400> 11679
ATGCGGGGAA GGATGAGAAG CTTCGACCAA GGTTCGACTC GAGCGCCAGC 50
GAGAGAGCGT TGCCGCAGGC AACGACCCGA AGGGCGAAGC GCGCAGCGCT 100
<210> 11680
<211> 100
<212> DNA
<213> Escherichia coli K-12 MG1655
<220> tpx b1325
<222> 408-507
<223> tpx b1325 U00096 complement(1386329..1386835)
<313> 11680: 450-469
<400> 11680
GGTTATTGAC GAAAATGACA ATGTGATTTT CAGCCAGCTG GTGGATGAAA 50
TCACCACCGA GCCGGATTAC GAAGCAGCTC TGGCTGTACT GAAAGCATAA 100
<210> 11681
<211> 100
<212> DNA
<213> Escherichia coli K-12 MG1655
<220> tpx b1325
<222> 381-480
<223> tpx b1325 U00096 complement(1386329..1386835)
<313> 11681: 407-426
<400> 11681
GAAAGGTCTG GCAGCGCGTG CCGTTGTGGT TATTGACGAA AATGACAATG 50
TGATTTTCAG CCAGCTGGTG GATGAAATCA CCACCGAGCC GGATTACGAA 100
<210> 11682
<211> 100
<212> DNA
<213> Escherichia coli K-12 MG1655
<220> tpx b1325
<222> 275-374
<223> tpx b1325 U00096 complement(1386329..1386835)
<313> 11682: 325-344
<400> 11682
CTCGTTTCTG CGGCGCAGAA GGTCTGAACA ACGTTATCAC CCTCTCCACT 50
TTCCGTAACG CTGAATTTCT GCAAGCTTAC GGTGTGGCAA TTGCTGATGG 100
<210> 11683
<211> 100
<212> DNA
<213> Escherichia coli K-12 MG1655

<220> b4140 b4141
<222> 279-378
<223> b4140 b4141 U00096 4366341..4366718
<313> 11683: 340-359
<400> 11683
TAGCGCCGGG ACCGGTGGCG GTAATACTTT TGATGGTGAG TACCAGCGAA  50
AGGATGATGA GCGCGACCGC CTTGATCATA AAGACGATCG CCAGGATTAA  100
<210> 11684
<211> 100
<212> DNA
<213> Escherichia coli K-12 MG1655
<220> b4140 b4141
<222> 208-307
<223> b4140 b4141 U00096 4366341..4366718
<313> 11684: 232-251
<400> 11684
CCGGTGCAAA AGCATCTGAC AGTGAAGTTG ATGCCGCATT TGCGCTTTTC  50
TCGCATGCCT GGCGGCGGTT TTAGCGCCGG GACCGGTGGC GGTAATACTT  100
<210> 11685
<211> 100
<212> DNA
<213> Escherichia coli K-12 MG1655
<220> b4140 b4141
<222> 139-238
<223> b4140 b4141 U00096 4366341..4366718
<313> 11685: 181-200
<400> 11685
ATTGCTGGTT TGCTGCTTTT ATTACCGGGC TTTTTTACCG ACTTCCTCGG  50
TCTTCTACTT TTATTGCCGC CGGTGCAAAA GCATCTGACA GTGAAGTTGA  100
<210> 11686
<211> 100
<212> DNA
<213> Escherichia coli K-12 MG1655
<220> ccha b2458
<222> 237-336
<223> ccha b2458 U00096 complement(2570177..2570512)
<313> 11686: 270-289
<400> 11686
TATCGGCGAG CTGGTATCTG TACACGTTAT CCCGCGTCCG CACGGCGACC  50
TGGAAGAAGT CTTCCCGATC GGCCTGAAAG GCGATAGCAG CAACCTGTAA  100
<210> 11687
<211> 100
<212> DNA
<213> Escherichia coli K-12 MG1655
<220> ccha b2458
<222> 106-205
<223> ccha b2458 U00096 complement(2570177..2570512)
<313> 11687: 135-154
<400> 11687
ATGGTGAAAG CTGCGCGCGT CAAGCTGGTT GGTGTTAAGC AGATTGGCGG  50
CGGCCTGTGT ACTGCCATGG TTCGTGGTGA TGTTGCAGCC TGTAAAGCCG  100
<210> 11688
<211> 100
<212> DNA
<213> Escherichia coli K-12 MG1655
<220> ccha b2458
<222> 19-118
<223> ccha b2458 U00096 complement(2570177..2570512)
<313> 11688: 50-69
<400> 11688
GACCCTGATA AAGAGGAAAG CACAATGGAA GCATTAGGAA TGATCGAAAC  50
CCGGGGCCTG GTTGCACTGA TTGAGGCTTC TGACGCGATG GTGAAAGCTG  100
<210> 11689
<211> 100
<212> DNA
<213> Escherichia coli K-12 MG1655
<220> cchb b2457
<222> 136-235
<223> cchb b2457 U00096 complement(2569783..2570070)
<313> 11689: 199-218
<400> 11689

AATATTGGCG CGGGAACCGG GGAGTGGGTG TTACTGGTGA GTGGCAGTTC 50
CGCCCGCCAG GCGCATAAAA GCGAAACGTC ACCGGTCGAT CTGTGCGTGA 100
<210> 11690
<211> 100
<212> DNA
<213> Escherichia coli K-12 MG1655
<220> cchb b2457
<222> 9-108
<223> cchb b2457 U00096 complement(2569783..2570070)
<313> 11690: 55-74
<400> 11690
GGCAGTCGTC ACTGGACAAA TTGTTTGTAC CGTACGCCAT CACGGACTGG 50
CGCATGACAA ATTGCTGATG GTGGAAATGA TTGATCCACA AGGTAATCCT 100
<210> 11691
<211> 100
<212> DNA
<213> Escherichia coli K-12 MG1655
<220> cchb b2457
<222> 1-100
<223> cchb b2457 U00096 complement(2569783..2570070)
<313> 11691: 5-24
<400> 11691
ATGAAACTGG CAGTCGTCAC TGGACAAATT GTTTGTACCG TACGCCATCA 50
CGGACTGGCG CATGACAAAT TGCTGATGGT GGAAATGATT GATCCACAAG 100
<210> 11692
<211> 100
<212> DNA
<213> Escherichia coli K-12 MG1655
<220> epd b2928
<222> 921-1020
<223> epd b2928 U00096 complement(3070692..3071711)
<313> 11692: 965-984
<400> 11692
ACACCTGATC AAAACGTTGG TCTGGTGCGA TAACGAATGG GGCTTTGCTA 50
ACCGAATGCT CGACACGACG TTAGCTATGG CTACTGTTGC TTTCAGGTAA 100
<210> 11693
<211> 100
<212> DNA
<213> Escherichia coli K-12 MG1655
<220> epd b2928
<222> 873-972
<223> epd b2928 U00096 complement(3070692..3071711)
<313> 11693: 930-949
<400> 11693
TCCGCACAGT GCCATTGTCG ATGGCACCCA AACCCGGGTC AGTGGCGCAC 50
ACCTGATCAA AACGTTGGTC TGGTGCGATA ACGAATGGGG CTTTGCTAAC 100
<210> 11694
<211> 100
<212> DNA
<213> Escherichia coli K-12 MG1655
<220> epd b2928
<222> 726-825
<223> epd b2928 U00096 complement(3070692..3071711)
<313> 11694: 764-783
<400> 11694
GACGGCAATC GATTTAAGCG TGACGGTGAA GAAACCTGTA AAAGCCAATG 50
AAGTCAACCT GTTGCTGCAA AAAGCAGCAC AAGGTGCATT TCATGGTATA 100
<210> 11695
<211> 100
<212> DNA
<213> Escherichia coli K-12 MG1655
<220> ompa b0958
<222> 879-978
<223> ompa b0958 U00096 complement(1018236..1019276)
<313> 11695: 930-949
<400> 11695
CAAGATCTCC GCACGTGGTA TGGGCGAATC CAACCCGGTT ACTGGCAACA 50
CCTGTGACAA CGTGAAACAG CGTGCTGCAC TGATCGACTG CCTGGCTCCG 100
<210> 11696
<211> 100
<212> DNA

<213> Escherichia coli K-12 MG1655
<220> ompa b0958
<222> 813-912
<223> ompa b0958 U00096 complement(1018236..1019276)
<313> 11696: 867-886
<400> 11696
GGGTCTGTCC GAGCGCCGTG CTCAGTCTGT TGTTGATTAC CTGATCTCCA 50
AAGGTATCCC GGCAGACAAG ATCTCCGCAC GTGGTATGGG CGAATCCAAC 100
<210> 11697
<211> 100
<212> DNA
<213> Escherichia coli K-12 MG1655
<220> ompa b0958
<222> 777-876
<223> ompa b0958 U00096 complement(1018236..1019276)
<313> 11697: 797-816
<400> 11697
TTACACCGAC CGCATCGGTT CTGACGCTTA CAACCAGGGT CTGTCCGAGC 50
GCCGTGCTCA GTCTGTTGTT GATTACCTGA TCTCCAAAGG TATCCCGGCA 100
<210> 11698
<211> 100
<212> DNA
<213> Escherichia coli K-12 MG1655
<220> trg trg
<222> 1506-1605
<223> trg trg U00096 1490494..1492134
<313> 11698: 1540-1559
<400> 11698
CTCAGCGGCG GCGGTGTCCC TTGAAGAACA GGCGGCACGA TTAACTGAGG 50
CGGTGGATGT ATTCCGTCTG CACAAACATT CTGTGTCGGC AGAACCTCGC 100
<210> 11699
<211> 100
<212> DNA
<213> Escherichia coli K-12 MG1655
<220> trg trg
<222> 1417-1516
<223> trg trg U00096 1490494..1492134
<313> 11699: 1470-1489
<400> 11699
GAACAAAGTA GAGGCATAAC GCAGGTTAGC CAGGCGATTT CTGAAATGGA 50
TAAGGTGACG CAACAGAATG CTTCTCTGGT AGAAGAGGCC TCAGCGGCGG 100
<210> 11700
<211> 100
<212> DNA
<213> Escherichia coli K-12 MG1655
<220> trg trg
<222> 1375-1474
<223> trg trg U00096 1490494..1492134
<313> 11700: 1412-1431
<400> 11700
AGTGTCACAC ATATCATGCA GGAAATCGCC GCCGCCTCGG ATGAACAAAG 50
TAGAGGCATA ACGCAGGTTA GCCAGGCGAT TTCTGAAATG GATAAGGTGA 100
<210> 11701
<211> 100
<212> DNA
<213> Escherichia coli K-12 MG1655
<220> ompc b2216
<222> 1005-1104
<223> ompc b2216 U00096 complement(2309666..2310769)
<313> 11701: 1058-1077
<400> 11701
TGACTACAAA ATCAACCTGC TGGACGACAA CCAGTTCACT CGTGACGCTG 50
GCATCAACAC TGATAACATC GTAGCTCTGG GTCTGGTTTA CCAGTTCTAA 100
<210> 11702
<211> 100
<212> DNA
<213> Escherichia coli K-12 MG1655
<220> ompc b2216
<222> 996-1095
<223> ompc b2216 U00096 complement(2309666..2310769)
<313> 11702: 1012-1031

<400> 11702
CACCTACGTT GACTACAAAA TCAACCTGCT GGACGACAAC CAGTTCACTC 50
GTGACGCTGG CATCAACACT GATAACATCG TAGCTCTGGG TCTGGTTTAC 100
<210> 11703
<211> 100
<212> DNA
<213> Escherichia coli K-12 MG1655
<220> ompc b2216
<222> 839-938
<223> ompc b2216 U00096 complement(2309666..2310769)
<313> 11703: 872-891
<400> 11703
TCGAAGCTGT TGCTCAGTAC CAGTTCGACT TCGGTCTGCG TCCGTCCCTG 50
GCTTACCTGC AGTCTAAAGG TAAAAACCTG GGTCGTGGCT ACGACGACGA 100
<210> 11704
<211> 100
<212> DNA
<213> Escherichia coli K-12 MG1655
<220> ompf b0930
<222> 990-1089
<223> ompf b0930 U00096 complement(985117..986205)
<313> 11704: 1062-1081
<400> 11704
CACCTATGTT GACTACATCA TCAACCAGAT CGATTCTGAC AACAAACTGG 50
GCGTAGGTTC AGACGACACC GTTGCTGTGG GTATCGTTTA CCAGTTCTAA 100
<210> 11705
<211> 100
<212> DNA
<213> Escherichia coli K-12 MG1655
<220> ompf b0930
<222> 911-1010
<223> ompf b0930 U00096 complement(985117..986205)
<313> 11705: 971-990
<400> 11705
ACGTAGAAGG TATCGGTGAT GTTGATCTGG TGAACTACTT TGAAGTGGGC 50
GCAACCTACT ACTTCAACAA AAACATGTCC ACCTATGTTG ACTACATCAT 100
<210> 11706
<211> 100
<212> DNA
<213> Escherichia coli K-12 MG1655
<220> ompf b0930
<222> 874-973
<223> ompf b0930 U00096 complement(985117..986205)
<313> 11706: 922-941
<400> 11706
CGTCCGTCCA TCGCTTACAC CAAATCTAAA GCGAAAGACG TAGAAGGTAT 50
CGGTGATGTT GATCTGGTGA ACTACTTTGA AGTGGGCGCA ACCTACTACT 100
<210> 11707
<211> 100
<212> DNA
<213> Escherichia coli K-12 MG1655
<220> ompg b1320
<222> 807-906
<223> ompg b1320 U00096 1379971..1380876
<313> 11707: 864-883
<400> 11707
TTCCGTTTCG CTGGAATACG CGTTTGAGTG GCAGGATCAC GACGAAGGCG 50
ACAGTGATAA ATTCCATTAT GCAGGTGTCG GCGTAAATTA CTCGTTCTGA 100
<210> 11708
<211> 100
<212> DNA
<213> Escherichia coli K-12 MG1655
<220> ompg b1320
<222> 704-803
<223> ompg b1320 U00096 1379971..1380876
<313> 11708: 727-746
<400> 11708
TGGATCGCTG GAGTAACTGG GACTGGCAGG ATGATATTGA ACGTGAAGGC 50
CATGATTTTA ACCGTGTAGG TTTATTTTAC GGTTATGATT TCCAGAACGG 100
<210> 11709
<211> 100

<212> DNA
<213> Escherichia coli K-12 MG1655
<220> ompg b1320
<222> 595-694
<223> ompg b1320 U00096 1379971..1380876
<313> 11709: 632-651
<400> 11709
AATATGGACG ACAGCCGCAA TAACGGTGAG TTTTCCACGC AAGAAATTCG 50
CGCCTATTTG CCGCTGACGC TCGGCAACCA CTCGGTGACG CCGTATACGC 100
<210> 11710
<211> 100
<212> DNA
<213> Escherichia coli K-12 MG1655
<220> ompr b3406
<222> 621-720
<223> ompr b3406 U00096 complement(3533503..3534222)
<313> 11710: 701-720
<400> 11710
TCTGCGCCGC ATGGTGGAAG AAGATCCAGC GCATCCGCGT TACATTCAGA 50
CCGTCTGGGG TCTGGGCTAC GTCTTTGTAC CGGACGGCTC TAAAGCATGA 100
<210> 11711
<211> 100
<212> DNA
<213> Escherichia coli K-12 MG1655
<220> ompr b3406
<222> 599-698
<223> ompr b3406 U00096 complement(3533503..3534222)
<313> 11711: 655-674
<400> 11711
CCATCGACGT GCAGATTTCG CGTCTGCGCC GCATGGTGGA AGAAGATCCA 50
GCGCATCCGC GTTACATTCA GACCGTCTGG GGTCTGGGCT ACGTCTTTGT 100
<210> 11712
<211> 100
<212> DNA
<213> Escherichia coli K-12 MG1655
<220> ompr b3406
<222> 543-642
<223> ompr b3406 U00096 complement(3533503..3534222)
<313> 11712: 594-613
<400> 11712
CCGCGATAAG CTGATGAACC TTGCCCGTGG TCGTGAATAT TCCGCAATGG 50
AACGCTCCAT CGACGTGCAG ATTTCGCGTC TGCGCCGCAT GGTGGAAGAA 100
<210> 11713
<211> 100
<212> DNA
<213> Escherichia coli K-12 MG1655
<220> map b0169
<222> 696-795
<223> map b0169 U00096 complement(188712..189506)
<313> 11713: 764-783
<400> 11713
ACAATATGAG CATACTATTG TGGTGACTGA TAACGGCTGC GAAATTCTGA 50
CGCTACGCAA GGATGACACC ATCCCGGCGA TAATCTCGCA CGACGAATAA 100
<210> 11714
<211> 100
<212> DNA
<213> Escherichia coli K-12 MG1655
<220> map b0169
<222> 696-795
<223> map b0169 U00096 complement(188712..189506)
<313> 11714: 719-738
<400> 11714
ACAATATGAG CATACTATTG TGGTGACTGA TAACGGCTGC GAAATTCTGA 50
CGCTACGCAA GGATGACACC ATCCCGGCGA TAATCTCGCA CGACGAATAA 100
<210> 11715
<211> 100
<212> DNA
<213> Escherichia coli K-12 MG1655
<220> map b0169
<222> 626-725
<223> map b0169 U00096 complement(188712..189506)

<313> 11715: 683-702
<400> 11715
CGGGTAAAAA AGAGATCCGC ACCATGAAAG ATGGCTGGAC GGTAAAAACC 50
AAAGATCGCA GCTTGTCTGC ACAATATGAG CATACTATTG TGGTGACTGA 100
<210> 11716
<211> 100
<212> DNA
<213> Escherichia coli K-12 MG1655
<220> ompt b0566
<222> 792-891
<223> ompt b0566 U00096 complement(583903..584856)
<313> 11716: 821-840
<400> 11716
CGCAAAAGTT TATGTTGAAG GCGCATGGAA TCGGGTTACG AATAAAAAAG 50
GTAATACTTC ACTTTATGAT CACAATAATA ACACTTCAGA CTACAGCAAA 100
<210> 11717
<211> 100
<212> DNA
<213> Escherichia coli K-12 MG1655
<220> ompt b0566
<222> 747-846
<223> ompt b0566 U00096 complement(583903..584856)
<313> 11717: 762-781
<400> 11717
TTACTATTCT GTTGCAGTCA ATGCAGGTTA TTACGTCACA CCTAACGCAA 50
AAGTTTATGT TGAAGGCGCA TGGAATCGGG TTACGAATAA AAAAGGTAAT 100
<210> 11718
<211> 100
<212> DNA
<213> Escherichia coli K-12 MG1655
<220> ompt b0566
<222> 625-724
<223> ompt b0566 U00096 complement(583903..584856)
<313> 11718: 664-683
<400> 11718
TATGAAGATT TTGAACTCGG TGGCACATTT AAATACAGCG GCTGGGTGGA 50
ATCATCTGAT AACGATGAAC ACTATGACCC GGGAAAAAGA ATCACTTATC 100
<210> 11719
<211> 100
<212> DNA
<213> Escherichia coli K-12 MG1655
<220> ompx b0815
<222> 410-509
<223> ompx b0815 U00096 849673..850188
<313> 11719: 444-463
<400> 11719
AGTTCAACCC GATGGAAAAC GTTGCTCTGG ACTTCTCTTA CGAGCAGAGC 50
CGTATTCGTA GCGTTGACGT AGGCACCTGG ATTGCCGGTG TTGGTTACCG 100
<210> 11720
<211> 100
<212> DNA
<213> Escherichia coli K-12 MG1655
<220> ompx b0815
<222> 338-437
<223> ompx b0815 U00096 849673..850188
<313> 11720: 378-397
<400> 11720
TCCAGACCAC TGAATACCCG ACCTACAAAC ACGACACCAG CGACTACGGT 50
TTCTCCTACG GTGCGGGTCT GCAGTTCAAC CCGATGGAAA ACGTTGCTCT 100
<210> 11721
<211> 100
<212> DNA
<213> Escherichia coli K-12 MG1655
<220> ompx b0815
<222> 283-382
<223> ompx b0815 U00096 849673..850188
<313> 11721: 338-357
<400> 11721
CGCATTAACG ACTGGGCAAG CATCTACGGT GTAGTGGGTG TGGGTTATGG 50
TAAATTCCAG ACCACTGAAT ACCCGACCTA CAAACACGAC ACCAGCGACT 100
<210> 11722

<211> 100
<212> DNA
<213> Escherichia coli K-12 MG1655
<220> tsf b0171
<222> 740-839
<223> tsf b0171 U00096 190857..191708
<313> 11722: 789-808
<400> 11722
AAGAGCATAA CGCTGAAGTG ACTGGCTTCA TCCGCTTCGA AGTGGGTGAA 50
GGCATCGAGA AAGTTGAGAC TGACTTTGCA GCAGAAGTTG CTGCGATGTC 100
<210> 11723
<211> 100
<212> DNA
<213> Escherichia coli K-12 MG1655
<220> tsf b0171
<222> 719-818
<223> tsf b0171 U00096 190857..191708
<313> 11723: 745-764
<400> 11723
AAACTGTTGG TCAGCTGCTG AAAGAGCATA ACGCTGAAGT GACTGGCTTC 50
ATCCGCTTCG AAGTGGGTGA AGGCATCGAG AAAGTTGAGA CTGACTTTGC 100
<210> 11724
<211> 100
<212> DNA
<213> Escherichia coli K-12 MG1655
<220> tsf b0171
<222> 595-694
<223> tsf b0171 U00096 190857..191708
<313> 11724: 658-677
<400> 11724
CTGGATATCG CGATGCAGTC TGGTAAGCCG AAAGAAATCG CAGAGAAAAT 50
GGTTGAAGGC CGCATGAAGA AATTCACCGG CGAAGTTTCT CTGACCGGTC 100
<210> 11725
<211> 100
<212> DNA
<213> Escherichia coli K-12 MG1655
<220> aidb b4188
<222> 1542-1641
<223> aidb b4188 U00096 4411838..4413478
<313> 11725: 1621-1640
<400> 11725
GTGGTGTCAG GTGATGTTAG ATACGCGCGG CGGCGTACGG TTGTCAGAGC 50
AGATCCAGAA TGATTTATTG CTGCGGGCGA CGGGGGGAGT GTGTGTGTAA 100
<210> 11726
<211> 100
<212> DNA
<213> Escherichia coli K-12 MG1655
<220> aidb b4188
<222> 1502-1601
<223> aidb b4188 U00096 4411838..4413478
<313> 11726: 1529-1548
<400> 11726
CGCAAATGTT GAAATATGCT TCTCCGCCAA TGGCGCAGGC GTGGTGTCAG 50
GTGATGTTAG ATACGCGCGG CGGCGTACGG TTGTCAGAGC AGATCCAGAA 100
<210> 11727
<211> 100
<212> DNA
<213> Escherichia coli K-12 MG1655
<220> aidb b4188
<222> 1435-1534
<223> aidb b4188 U00096 4411838..4413478
<313> 11727: 1460-1479
<400> 11727
CTGCGTAAGC CAGCTGAAGA ACTGGGGCGA GAGATTACTC ATCAGCTATT 50
CCTGCTGGGC TGCGGTGCGC AAATGTTGAA ATATGCTTCT CCGCCAATGG 100
<210> 11728
<211> 100
<212> DNA
<213> Escherichia coli K-12 MG1655
<220> era b2567
<222> 794-893

<223> era b2567 U00096 complement(2700501..2701406)
<313> 11728: 815-834
<400> 11728
TGCAGGAAAT GTTCGAAGCG CCTGTTCACC TTGAACTGTG GGTAAAAGTG 50
AAATCCGGTT GGGCCGACGA CGAACGCGCA CTGCGCAGTC TCGGTTACGT 100
<210> 11729
<211> 100
<212> DNA
<213> Escherichia coli K-12 MG1655
<220> era b2567
<222> 747-846
<223> era b2567 U00096 complement(2700501..2701406)
<313> 11729: 774-793
<400> 11729
CAAAGGGGCC AAGATCAAAA CCATCGGGAT TGAAGCGCGT AAAGACATGC 50
AGGAAATGTT CGAAGCGCCT GTTCACCTTG AACTGTGGGT AAAAGTGAAA 100
<210> 11730
<211> 100
<212> DNA
<213> Escherichia coli K-12 MG1655
<220> era b2567
<222> 687-786
<223> era b2567 U00096 complement(2700501..2701406)
<313> 11730: 718-737
<400> 11730
TGACATCAAC GGTTTGATTC TCGTTGAGCG TGAAGGGCAG AAGAAGATGG 50
TCATTGGCAA CAAAGGGGCC AAGATCAAAA CCATCGGGAT TGAAGCGCGT 100
<210> 11731
<211> 100
<212> DNA
<213> Escherichia coli K-12 MG1655
<220> tsr b4356
<222> 1521-1620
<223> tsr b4356 U00096 4589227..4590882
<313> 11731: 1580-1599
<400> 11731
CCTGACCGAA GCAGTGGCAG TGTTCCGGAT TCAGCAACAG CAGCGTGAAA 50
CATCGGCTGT GGTAAAAACC GTGACGCCAG CTGCGCCGCG TAAAATGGCC 100
<210> 11732
<211> 100
<212> DNA
<213> Escherichia coli K-12 MG1655
<220> tsr b4356
<222> 1492-1591
<223> tsr b4356 U00096 4589227..4590882
<313> 11732: 1510-1529
<400> 11732
GCCGCCGCGC TGGAAGAGCA GGCCAGTCGC CTGACCGAAG CAGTGGCAGT 50
GTTCCGGATT CAGCAACAGC AGCGTGAAAC ATCGGCTGTG GTAAAAACCG 100
<210> 11733
<211> 100
<212> DNA
<213> Escherichia coli K-12 MG1655
<220> tsr b4356
<222> 1419-1518
<223> tsr b4356 U00096 4589227..4590882
<313> 11733: 1473-1492
<400> 11733
TGGCTTAGCG GTTGCTGAGA TGGACCGGGT AACTCAACAG AACGCCGCGC 50
TGGTGGAAGA GTCTGCCGCT GCCGCCGCCG CGCTGGAAGA GCAGGCCAGT 100
<210> 11734
<211> 100
<212> DNA
<213> Escherichia coli K-12 MG1655
<220> tsx b0412
<222> 786-885
<223> tsx b0412 U00096 complement(430353..431237)
<313> 11734: 814-833
<400> 11734
TCAGTGGAAC GACGATGCAG AACTGAACTT CGGCAACGGC AACTTCAACG 50
TTCGCTCTAC CGGCTGGGGT GGTTACCTGG TAGTAGGTTA CAACTTCTGA 100

<210> 11735
<211> 100
<212> DNA
<213> Escherichia coli K-12 MG1655
<220> tsx b0412
<222> 738-837
<223> tsx b0412 U00096 complement(430353..431237)
<313> 11735: 774-793
<400> 11735
CGATCACTGG CACTACTCTG TCGTAGCTCG TTACTGGCAC GACGGTGGTC 50
AGTGGAACGA CGATGCAGAA CTGAACTTCG GCAACGGCAA CTTCAACGTT 100
<210> 11736
<211> 100
<212> DNA
<213> Escherichia coli K-12 MG1655
<220> tsx b0412
<222> 684-783
<223> tsx b0412 U00096 complement(430353..431237)
<313> 11736: 725-744
<400> 11736
TAAGACCCGT ACTAATAACT CTATCGCTTC CAGCCATATT CTGGCTCTGA 50
ACTACGATCA CTGGCACTAC TCTGTCGTAG CTCGTTACTG GCACGACGGT 100
<210> 11737
<211> 100
<212> DNA
<213> Escherichia coli K-12 MG1655
<220> tnaa b3709
<222> 1332-1431
<223> tnaa b3709 U00096 3886344..3887774
<313> 11737: 1402-1421
<400> 11737
TAAACATGTG AAAGAGAACG CGGCGAATAT TAAAGGATTA ACCTTTACGT 50
ACGAACCGAA AGTATTGCGT CACTTCACCG CAAAACTTAA AGAAGTTTAA 100
<210> 11738
<211> 100
<212> DNA
<213> Escherichia coli K-12 MG1655
<220> tnaa b3709
<222> 1227-1326
<223> tnaa b3709 U00096 3886344..3887774
<313> 11738: 1262-1281
<400> 11738
TCCGAAAACC GGTAAACAAC TGCCATGCCC GGCTGAACTG CTGCGTTTAA 50
CCATTCCGCG CGCAACATAT ACTCAAACAC ATATGGACTT CATTATTGAA 100
<210> 11739
<211> 100
<212> DNA
<213> Escherichia coli K-12 MG1655
<220> tnaa b3709
<222> 1165-1264
<223> tnaa b3709 U00096 3886344..3887774
<313> 11739: 1214-1233
<400> 11739
GAGCTGTATA AAGTCGCCGG TATCCGTGCG GTAGAAATTG GCTCTTTCCT 50
GTTAGGCCGC GATCCGAAAA CCGGTAAACA ACTGCCATGC CCGGCTGAAC 100
<210> 11740
<211> 100
<212> DNA
<213> Escherichia coli K-12 MG1655
<220> tnab b3710
<222> 1149-1248
<223> tnab b3710 U00096 3887865..3889112
<313> 11740: 1201-1220
<400> 11740
GGGCGGCAAT CTTATTCCGG CGATTGTCAT TCTCTTTGGT ATAACCGTGA 50
TTTTGTGCTG GTTCGGCAAC GTCTTTAACG TGTTACCTAA ATTTGGCTAA 100
<210> 11741
<211> 100
<212> DNA
<213> Escherichia coli K-12 MG1655
<220> tnab b3710

<222> 1111-1210
<223> tnab b3710 U00096 3887865..3889112
<313> 11741: 1151-1170
<400> 11741
GCTCGCAAGA AGTTTCCCAA TCAGATGTTC ACGGTCTGGG GCGGCAATCT 50
TATTCCGGCG ATTGTCATTC TCTTTGGTAT AACCGTGATT TTGTGCTGGT 100
<210> 11742
<211> 100
<212> DNA
<213> Escherichia coli K-12 MG1655
<220> tnab b3710
<222> 1045-1144
<223> tnab b3710 U00096 3887865..3889112
<313> 11742: 1099-1118
<400> 11742
ATCGGCGGTG CCGGGCTGTG CGCCACCATC TGGGCGGTCA TTATTCCCGC 50
AGTGCTTGCA ATCAAAGCTC GCAAGAAGTT TCCCAATCAG ATGTTCACGG 100
<210> 11743
<211> 100
<212> DNA
<213> Escherichia coli K-12 MG1655
<220> b2710 b2711
<222> 1332-1431
<223> b2710 b2711 U00096 2830499..2831938
<313> 11743: 1378-1397
<400> 11743
GGACGTTGCG CCAGGAACGC CGTGGAGTGA AGTCCCGGAT AACTTCCTCT 50
GCCCGGAATG CTCCCTCGGC AAAGACGTCT TTGAAGAACT GGCATCGGAG 100
<210> 11744
<211> 100
<212> DNA
<213> Escherichia coli K-12 MG1655
<220> b2710 b2711
<222> 1287-1386
<223> b2710 b2711 U00096 2830499..2831938
<313> 11744: 1338-1357
<400> 11744
CGTCTGCCAG TGGATTTACG ATCCGGCAAA AGGCGAGCCA ATGCAGGACG 50
TTGCGCCAGG AACGCCGTGG AGTGAAGTCC CGGATAACTT CCTCTGCCCG 100
<210> 11745
<211> 100
<212> DNA
<213> Escherichia coli K-12 MG1655
<220> b2710 b2711
<222> 1191-1290
<223> b2710 b2711 U00096 2830499..2831938
<313> 11745: 1237-1256
<400> 11745
GCTCGCGCCG CTGCCGCAGA GCACGGTGAA TACGGTAGTT AAAGAAGAAA 50
CCTCTGCCAC CACGACGGCT GACCTCGGCC CACGGATGCA GTGCAGCGTC 100
<210> 11746
<211> 100
<212> DNA
<213> Escherichia coli K-12 MG1655
<220> ttk b3642
<222> 540-639
<223> ttk b3642 U00096 3812079..3812717
<313> 11746: 592-611
<400> 11746
TGAAGGTATG CTGTCACGTT TTGTCCGCAG CGAATTTAAA TACCGCCCGA 50
CGGATGATTT TGACGCCCGC TGGCCGCTAA TTGCGGCACA GTTGCAGTAA 100
<210> 11747
<211> 100
<212> DNA
<213> Escherichia coli K-12 MG1655
<220> ttk b3642
<222> 526-625
<223> ttk b3642 U00096 3812079..3812717
<313> 11747: 543-562
<400> 11747
ATCCTGGCCT TCTGTGAAGG TATGCTGTCA CGTTTTGTCC GCAGCGAATT 50

TAAATACCGC CCGACGGATG ATTTTGACGC CCGCTGGCCG CTAATTGCGG 100
<210> 11748
<211> 100
<212> DNA
<213> Escherichia coli K-12 MG1655
<220> ttk b3642
<222> 450-549
<223> ttk b3642 U00096 3812079..3812717
<313> 11748: 497-516
<400> 11748
GCGCCAGGTA TTGCGTGAAA AGAGAATGCG TGAGGGTGAA GGTTACACCA 50
CCGATGAAAC CCTGCTGGCA AGCCAGATCC TGGCCTTCTG TGAAGGTATG 100
<210> 11749
<211> 100
<212> DNA
<213> Escherichia coli K-12 MG1655
<220> nana b3226
<222> 795-894
<223> nana b3226 U00096 complement(3370318..3371211)
<313> 11749: 863-882
<400> 11749
TTCTGTGCCG CTGTGCCGCA AACCGTTTGG ACCGGTAGAT GAAAAATATC 50
TGCCAGAACT GAAGGCGCTG GCCCAGCAGT TGATGCAAGA GCGCGGGTGA 100
<210> 11750
<211> 100
<212> DNA
<213> Escherichia coli K-12 MG1655
<220> nana b3226
<222> 748-847
<223> nana b3226 U00096 complement(3370318..3371211)
<313> 11750: 791-810
<400> 11750
GGCGTATTCC GCGGCCTGAA AACTGTCCTC CATTATATGG ATGTCGTTTC 50
TGTGCCGCTG TGCCGCAAAC CGTTTGGACC GGTAGATGAA AAATATCTGC 100
<210> 11751
<211> 100
<212> DNA
<213> Escherichia coli K-12 MG1655
<220> nana b3226
<222> 643-742
<223> nana b3226 U00096 complement(3370318..3371211)
<313> 11751: 694-713
<400> 11751
TGGCGCTATC AGGGGATCGT TAAGGCGCTG AAAGAAGGCG ATATCCAGAC 50
CGCGCAGAAA CTGCAAACTG AATGCAATAA AGTCATTGAT TTACTGATCA 100
<210> 11752
<211> 100
<212> DNA
<213> Escherichia coli K-12 MG1655
<220> ygaa ygaA
<222> 1430-1529
<223> ygaa ygaA U00096 complement(2828798..2830387)
<313> 11752: 1487-1506
<400> 11752
TTAAGCAAAA CCTGCGTGAA GCGACAGAAG CGTTCCAGCG TGAAACTATT 50
CGTCAGGCAC TGGCACAAAA TCATCACAAC TGGGCTGCCT GCGCGCGGAT 100
<210> 11753
<211> 100
<212> DNA
<213> Escherichia coli K-12 MG1655
<220> ygaa ygaA
<222> 1418-1517
<223> ygaa ygaA U00096 complement(2828798..2830387)
<313> 11753: 1452-1471
<400> 11753
CGGTGCCCGT TGTTAAGCAA AACCTGCGTG AAGCGACAGA AGCGTTCCAG 50
CGTGAAACTA TTCGTCAGGC ACTGGCACAA AATCATCACA ACTGGGCTGC 100
<210> 11754
<211> 100
<212> DNA
<213> Escherichia coli K-12 MG1655

<220> ygaa ygaA
<222> 1310-1409
<223> ygaa ygaA U00096 complement(2828798..2830387)
<313> 11754: 1370-1389
<400> 11754
ATCGGGCGGT AGTTCTGGCG AGAGCCACCC GCAGCGGCGA TGAAGTGATT 50
CTTGAGGCGC AACATTTTGC TTTTCCTGAG GTGACGTTGC CGACGCCAGA 100
<210> 11755
<211> 100
<212> DNA
<213> Escherichia coli K-12 MG1655
<220> ygac b2672
<222> 246-345
<223> ygac b2672 U00096 complement(2797671..2798015)
<313> 11755: 290-309
<400> 11755
AGCAGGCGAG CGACACGAGC ATTACGGTAT CCCGCATGGC TTTAGTTCGC 50
GTGTTGCGCT TGAACGTTAT TTGAATGGTT TATTTGGCGA AGCCAGTTAA 100
<210> 11756
<211> 100
<212> DNA
<213> Escherichia coli K-12 MG1655
<220> ygac b2672
<222> 225-324
<223> ygac b2672 U00096 complement(2797671..2798015)
<313> 11756: 258-277
<400> 11756
CCAGCAATTT CCGCTCTATT TAGCAGGCGA GCGACACGAG CATTACGGTA 50
TCCCGCATGG CTTTAGTTCG CGTGTTGCGC TTGAACGTTA TTTGAATGGT 100
<210> 11757
<211> 100
<212> DNA
<213> Escherichia coli K-12 MG1655
<220> ygac b2672
<222> 176-275
<223> ygac b2672 U00096 complement(2797671..2798015)
<313> 11757: 196-215
<400> 11757
GTTCGACACT GGCTGAACCC GCTTCCGATA TTAAAACTTG CGATCATTAC 50
CAGCAATTTC CGCTCTATTT AGCAGGCGAG CGACACGAGC ATTACGGTAT 100
<210> 11758
<211> 100
<212> DNA
<213> Escherichia coli K-12 MG1655
<220> ygad b2701
<222> 399-498
<223> ygad b2701 U00096 complement(2821872..2822369)
<313> 11758: 468-487
<400> 11758
CCGGCGGGAA TGCTTCAGCG GCGACCGTGA TGCGGTGCGT CGTCAGGCTA 50
CTGCGTATGC ATTGCAGACC TTGTGGCAAC AATTTCTACA AAACACTTGA 100
<210> 11759
<211> 100
<212> DNA
<213> Escherichia coli K-12 MG1655
<220> ygad b2701
<222> 370-469
<223> ygad b2701 U00096 complement(2821872..2822369)
<313> 11759: 410-429
<400> 11759
TTTGCCACTG CCCGCGGTGA AGGCATTACC CGGCGGGAAT GCTTCAGCGG 50
CGACCGTGAT GCGGTGCGTC GTCAGGCTAC TGCGTATGCA TTGCAGACCT 100
<210> 11760
<211> 100
<212> DNA
<213> Escherichia coli K-12 MG1655
<220> ygad b2701
<222> 238-337
<223> ygad b2701 U00096 complement(2821872..2822369)
<313> 11760: 288-307
<400> 11760

GAACCCGTCG TGGTGGAAAT GGCGATAGGC GCACTGAAAG CGGCTCGTGC 50
TGATTATGCC GTGTCTATTA GTGGTATCGC CGGGCCGGAT GGCGGCAGTG 100
<210> 11761
<211> 100
<212> DNA
<213> Escherichia coli K-12 MG1655
<220> ygae b2665
<222> 582-681
<223> ygae b2665 U00096 2793677..2794357
<313> 11761: 647-666
<400> 11761
GGCGGTACTG GCGCGAGATA CCGCGCGCGC CAGTGAGTTA ATGCGCCAGC 50
ATTTACTGAC GCCAATTCCC ATTATCCAGC AGGCGATGGC TGGCAATTAA 100
<210> 11762
<211> 100
<212> DNA
<213> Escherichia coli K-12 MG1655
<220> ygae b2665
<222> 529-628
<223> ygae b2665 U00096 2793677..2794357
<313> 11762: 550-569
<400> 11762
CTTTCGGTGG AAATGCTGGA GGATAAACAC GATCAGCACC AGACCCTGAC 50
TGCGGCGGTA CTGGCGCGAG ATACCGCGCG CGCCAGTGAG TTAATGCGCC 100
<210> 11763
<211> 100
<212> DNA
<213> Escherichia coli K-12 MG1655
<220> ygae b2665
<222> 374-473
<223> ygae b2665 U00096 2793677..2794357
<313> 11763: 395-414
<400> 11763
CCAGCGAGAA AATGCTTGAT GAGTGGGATC TGCGTCATCA GGCGTTTCAT 50
ACGGCAATTG TGGCGGGCTG TGGTTCTCAC TATTTGCTGC AAATGCGTGA 100
<210> 11764
<211> 100
<212> DNA
<213> Escherichia coli K-12 MG1655
<220> ygaf b2661
<222> 1228-1327
<223> ygaf b2661 U00096 2787937..2789271
<313> 11764: 1279-1298
<400> 11764
CATATTGTCA GCAAGGTACA AACGCTGTTG GCAAGCCAGA GTAACCCCGG 50
ACGCACGCTG CGAGCGGCAC GTAGTGTGGA TGCCTTACAC GCCGCATTTA 100
<210> 11765
<211> 100
<212> DNA
<213> Escherichia coli K-12 MG1655
<220> ygaf b2661
<222> 1143-1242
<223> ygaf b2661 U00096 2787937..2789271
<313> 11765: 1198-1217
<400> 11765
TTTTCTGTTT GTCACCACCC CGCGCACGAT CCACACCTGC AATGCGCCCT 50
CCCCGGCAGC GACATCAGCA ATTCCTATTG GTGCGCATAT TGTCAGCAAG 100
<210> 11766
<211> 100
<212> DNA
<213> Escherichia coli K-12 MG1655
<220> ygaf b2661
<222> 1121-1220
<223> ygaf b2661 U00096 2787937..2789271
<313> 11766: 1162-1181
<400> 11766
CGGACGGCAA GCTGATTGAC GATTTTCTGT TTGTCACCAC CCCGCGCACG 50
ATCCACACCT GCAATGCGCC CTCCCCGGCA GCGACATCAG CAATTCCTAT 100
<210> 11767
<211> 100
<212> DNA

<213> Escherichia coli K-12 MG1655
<220> ygag b2688
<222> 417-516
<223> ygag b2688 U00096 complement(2812239..2812754)
<313> 11767: 495-514
<400> 11767
GCAGGATATT GCGCGTAGCA TTCTGGAACG TGACGTACGC ATCAACAGCA 50
ACGAAGAACT GGCACTGCCG AAAGAGAAGT TGCAGGAACT GCACATCTAG 100
<210> 11768
<211> 100
<212> DNA
<213> Escherichia coli K-12 MG1655
<220> ygag b2688
<222> 365-464
<223> ygag b2688 U00096 complement(2812239..2812754)
<313> 11768: 407-426
<400> 11768
AACTGAACGT CTACCAGTGT GGCACTTACC AGATGCACTC GTTGCAGGAA 50
GCGCAGGATA TTGCGCGTAG CATTCTGGAA CGTGACGTAC GCATCAACAG 100
<210> 11769
<211> 100
<212> DNA
<213> Escherichia coli K-12 MG1655
<220> ygag b2688
<222> 332-431
<223> ygag b2688 U00096 complement(2812239..2812754)
<313> 11769: 364-383
<400> 11769
TGCTGAAAGT GCAGGATCAG AATCAGATCC CGGAACTGAA CGTCTACCAG 50
TGTGGCACTT ACCAGATGCA CTCGTTGCAG GAAGCGCAGG ATATTGCGCG 100
<210> 11770
<211> 100
<212> DNA
<213> Escherichia coli K-12 MG1655
<220> prfa b1212
<222> 984-1083
<223> prfa b1212 U00096 1264235..1265317
<313> 11770: 1033-1052
<400> 11770
CCTGGATGAA GTGATGGAAG GTAAGCTGGA TATGCTGATT GAACCGATTA 50
TCCAGGAACA TCAGGCCGAC CAACTGGCGG CGTTGTCCGA GCAGGAATAA 100
<210> 11771
<211> 100
<212> DNA
<213> Escherichia coli K-12 MG1655
<220> prfa b1212
<222> 948-1047
<223> prfa b1212 U00096 1264235..1265317
<313> 11771: 993-1012
<400> 11771
CGTTACCGAT CACCGCATCA ACCTGACGCT CTACCGCCTG GATGAAGTGA 50
TGGAAGGTAA GCTGGATATG CTGATTGAAC CGATTATCCA GGAACATCAG 100
<210> 11772
<211> 100
<212> DNA
<213> Escherichia coli K-12 MG1655
<220> prfa b1212
<222> 865-964
<223> prfa b1212 U00096 1264235..1265317
<313> 11772: 922-941
<400> 11772
GCCGAAGCGT CTACCCGTCG TAACCTGCTG GGGAGTGGCG ATCGCAGCGA 50
CCGTAACCGT ACTTACAACT TCCCGCAGGG GCGCGTTACC GATCACCGCA 100
<210> 11773
<211> 100
<212> DNA
<213> Escherichia coli K-12 MG1655
<220> ygah b2684
<222> 204-303
<223> ygah b2684 U00096 2808365..2808700
<313> 11773: 258-277

<400> 11773
CCGTTTCGTG CCCACGCTGG TCGGCTTCGC GGTACTGGGT GCCAGTTTCT 50
ATAAAACACG CAGCATTATC ATCCCAACAC TGCTTAGTGC GCTGGCCTAT 100
<210> 11774
<211> 100
<212> DNA
<213> Escherichia coli K-12 MG1655
<220> ygah b2684
<222> 148-247
<223> ygah b2684 U00096 2808365..2808700
<313> 11774: 181-200
<400> 11774
TCGATATGCG CTCTGCTGGT TGTCTCTACC GCACCAGAAG TGATGCACGA 50
TACACGCCGT TTCGTGCCCA CGCTGGTCGG CTTCGCGGTA CTGGGTGCCA 100
<210> 11775
<211> 100
<212> DNA
<213> Escherichia coli K-12 MG1655
<220> ygah b2684
<222> 110-209
<223> ygah b2684 U00096 2808365..2808700
<313> 11775: 150-169
<400> 11775
GCGCGGTAGG TATTTTGCTC GACACCATTG GCATCGCCTC GATATGCGCT 50
CTGCTGGTTG TCTCTACCGC ACCAGAAGTG ATGCACGATA CACGCCGTTT 100
<210> 11776
<211> 100
<212> DNA
<213> Escherichia coli K-12 MG1655
<220> prfb b2892
<222> 994-1093
<223> prfb b2892 U00096 complement(3033204..3034302)
<313> 11776: 1052-1071
<400> 11776
CGCATTAAAG ATCTGCGCAC CGGGGTAGAA ACCCGCAACA CGCAGGCCGT 50
GCTGGACGGC AGCCTGGATC AATTTATCGA AGCAAGTTTG AAAGCAGGGT 100
<210> 11777
<211> 100
<212> DNA
<213> Escherichia coli K-12 MG1655
<220> prfb b2892
<222> 919-1018
<223> prfb b2892 U00096 complement(3033204..3034302)
<313> 11777: 970-989
<400> 11777
AAACAGGCGA TGGAAGATAA CAAATCCGAC ATCGGCTGGG GCAGCCAGAT 50
TCGTTCTTAT GTCCTTGATG ACTCCCGCAT TAAAGATCTG CGCACCGGGG 100
<210> 11778
<211> 100
<212> DNA
<213> Escherichia coli K-12 MG1655
<220> prfb b2892
<222> 873-972
<223> prfb b2892 U00096 complement(3033204..3034302)
<313> 11778: 936-955
<400> 11778
GAAAGCGAAG CTTTATGAAC TGGAGATGCA GAAGAAAAAT GCCGAGAAAC 50
AGGCGATGGA AGATAACAAA TCCGACATCG GCTGGGGCAG CCAGATTCGT 100
<210> 11779
<211> 100
<212> DNA
<213> Escherichia coli K-12 MG1655
<220> prfc b4376
<222> 1491-1590
<223> prfc b4376 U00096 4606983..4608572
<313> 11779: 1531-1550
<400> 11779
CGGCGATAAC CTCGCTTACA TCGCTACCAG CATGGTCAAC CTGCGCCTGG 50
CACAGGAACG TTATCCGGAC GTTCAGTTCC ACCAGACCCG CGAGCATTAA 100
<210> 11780
<211> 100

<212> DNA
<213> Escherichia coli K-12 MG1655
<220> prfc b4376
<222> 1466-1565
<223> prfc b4376 U00096 4606983..4608572
<313> 11780: 1492-1511
<400> 11780
ACGAAAGCCA ACTGGCGCTT GATGGCGGCG ATAACCTCGC TTACATCGCT 50
ACCAGCATGG TCAACCTGCG CCTGGCACAG GAACGTTATC CGGACGTTCA 100
<210> 11781
<211> 100
<212> DNA
<213> Escherichia coli K-12 MG1655
<220> prfc b4376
<222> 1398-1497
<223> prfc b4376 U00096 4606983..4608572
<313> 11781: 1441-1460
<400> 11781
CAACGTTGCC ACTGCCCGCT GGGTAGAATG TGCAGACGCG AAGAAATTCG 50
AAGAGTTCAA GCGTAAGAAC GAAAGCCAAC TGGCGCTTGA TGGCGGCGAT 100
<210> 11782
<211> 100
<212> DNA
<213> Escherichia coli K-12 MG1655
<220> ygam b2673
<222> 138-237
<223> ygam b2673 U00096 2798155..2798496
<313> 11782: 172-191
<400> 11782
AGCTGAAGCC GCACGCAGTA AAGCTCAGGC ATTGCTGAAA GAAACCAGGG 50
CGCGAATGCA TGGTCGTACT CGCGTCCAGC AAGCCGCGCG CGATGCCGTT 100
<210> 11783
<211> 100
<212> DNA
<213> Escherichia coli K-12 MG1655
<220> ygam b2673
<222> 104-203
<223> ygam b2673 U00096 2798155..2798496
<313> 11783: 145-164
<400> 11783
TATTGAAGTC CTGGGGCAGC GACGCCAAAG GGGAAGCTGA AGCCGCACGC 50
AGTAAAGCTC AGGCATTGCT GAAAGAAACC AGGGCGCGAA TGCATGGTCG 100
<210> 11784
<211> 100
<212> DNA
<213> Escherichia coli K-12 MG1655
<220> ygam b2673
<222> 1-100
<223> ygam b2673 U00096 2798155..2798496
<313> 11784: 21-40
<400> 11784
ATGGGGGATC ATATGTTTAA CCGACCGAAC CGCAACGACG TTGATGATGG 50
CGTGCAGGAT ATCCAGAATG ATGTCAATCA ATTAGCTGAC AGCCTCGAAT 100
<210> 11785
<211> 100
<212> DNA
<213> Escherichia coli K-12 MG1655
<220> prfh b0237
<222> 402-501
<223> prfh b0237 U00096 253702..254202
<313> 11785: 467-486
<400> 11785
TAGCGCGGCG CTGAAATCGC AGCGGCGAAT GTTCCATCAC CAGATTGAAC 50
GTGGCAACCC GCGACGGACA TTTACAGGGA TGGCTTTTAT CGAAGGATAA 100
<210> 11786
<211> 100
<212> DNA
<213> Escherichia coli K-12 MG1655
<220> prfh b0237
<222> 321-420
<223> prfh b0237 U00096 253702..254202

<313> 11786: 388-407
<400> 11786
GTCAGAGCGT AGTCAGCATG CTAACAAGCG GCTGGCACGA TTGCTGATTG 50
CCTGGAAGCT GGAGCAACAG CAACAGGAAA ATAGCGCGGC GCTGAAATCG 100
<210> 11787
<211> 100
<212> DNA
<213> Escherichia coli K-12 MG1655
<220> prfh b0237
<222> 269-368
<223> prfh b0237 U00096 253702..254202
<313> 11787: 313-332
<400> 11787
ACTCGGCGGT ACGCGCCACG CATTTGGCAT CCGGTATTAG CGTGAAGGTT 50
CAGTCAGAGC GTAGTCAGCA TGCTAACAAG CGGCTGGCAC GATTGCTGAT 100
<210> 11788
<211> 100
<212> DNA
<213> Escherichia coli K-12 MG1655
<220> nant b3225
<222> 1389-1488
<223> nant b3225 U00096 complement(3368719..3370239)
<313> 11788: 1436-1455
<400> 11788
TATGCCTTCT CGCGTTCAGC GTTGGTTGCG CCCGGAAGCG TTGCGTACTC 50
ATGACGCTAT CGACGGTAAA CCATTCAGCG GTGCCGTGCC GTTTGGCAGC 100
<210> 11789
<211> 100
<212> DNA
<213> Escherichia coli K-12 MG1655
<220> nant b3225
<222> 1332-1431
<223> nant b3225 U00096 complement(3368719..3370239)
<313> 11789: 1369-1388
<400> 11789
GCTGGCATCG CTCTCGTTCA GTCTGACGTT CGTGGTGATC CTGCTGATTG 50
GGCTGGATAT GCCTTCTCGC GTTCAGCGTT GGTTGCGCCC GGAAGCGTTG 100
<210> 11790
<211> 100
<212> DNA
<213> Escherichia coli K-12 MG1655
<220> nant b3225
<222> 1301-1400
<223> nant b3225 U00096 complement(3368719..3370239)
<313> 11790: 1329-1348
<400> 11790
TGATCGCTCA ACGTCTGGAT CTGGGTACTG CGCTGGCATC GCTCTCGTTC 50
AGTCTGACGT TCGTGGTGAT CCTGCTGATT GGGCTGGATA TGCCTTCTCG 100
<210> 11791
<211> 100
<212> DNA
<213> Escherichia coli K-12 MG1655
<220> ygap b2669
<222> 426-525
<223> ygap b2669 U00096 2795541..2796065
<313> 11791: 485-504
<400> 11791
AAGTGGCTTT GTTGGTGCCG GGTTACTGTT TGCAGGAATC AGCGGTTTTT 50
GTGGAATGGC AAGGTTGTTA GATAAGATGC CGTGGAACCA ACGAGCTTGA 100
<210> 11792
<211> 100
<212> DNA
<213> Escherichia coli K-12 MG1655
<220> ygap b2669
<222> 236-335
<223> ygap b2669 U00096 2795541..2796065
<313> 11792: 294-313
<400> 11792
CGATTGCCGC CCCCGCAGAA ATCTTTTTAC TCGAAGATGG GATTGATGGC 50
TGGAAAAAAG CAGGATTGCC AGTAGCGGTA AATAAATCTC AACCCTTGCC 100
<210> 11793

<211> 100
<212> DNA
<213> Escherichia coli K-12 MG1655
<220> ygap b2669
<222> 213-312
<223> ygap b2669 U00096 2795541..2796065
<313> 11793: 255-274
<400> 11793
TAATAATGCC GATAAATTAG CAGCGATTGC CGCCCCCGCA GAAATCTTTT 50
TACTCGAAGA TGGGATTGAT GGCTGGAAAA AAGCAGGATT GCCAGTAGCG 100
<210> 11794
<211> 100
<212> DNA
<213> Escherichia coli K-12 MG1655
<220> ygau ygaU
<222> 304-403
<223> ygau ygaU U00096 complement(2794358..2794807)
<313> 11794: 329-348
<400> 11794
AAGTCTGGCG ACACTCTGAG TGCCATTTCC AAACAGGTCT ACGGTAACGC 50
TAATCTGTAC AATAAAATCT TCGAAGCGAA TAAACCGATG CTAAAAAGCC 100
<210> 11795
<211> 100
<212> DNA
<213> Escherichia coli K-12 MG1655
<220> ygau ygaU
<222> 244-343
<223> ygau ygaU U00096 complement(2794358..2794807)
<313> 11795: 286-305
<400> 11795
AGTGTCGATG ATCAGGTGAA AACGGCGACA CCAGCCACTG CCAGCCAGTT 50
TTATACCGTT AAGTCTGGCG ACACTCTGAG TGCCATTTCC AAACAGGTCT 100
<210> 11796
<211> 100
<212> DNA
<213> Escherichia coli K-12 MG1655
<220> ygau ygaU
<222> 141-240
<223> ygau ygaU U00096 complement(2794358..2794807)
<313> 11796: 189-208
<400> 11796
TATTCAAATT GCCGACGGCA AAGCGACGGT CACTGGTGAC GGCCTGAGTC 50
AGGAGGCGAA GGAGAAAATC CTTGTTGCGG TGGGGAATAT TTCCGGTATT 100
<210> 11797
<211> 100
<212> DNA
<213> Escherichia coli K-12 MG1655
<220> mdh b3237
<222> 840-939
<223> mdh b3237 U00096 complement(3380965..3381903)
<313> 11797: 895-914
<400> 11797
TATCGGTACC CTGAGCGCAT TTGAACAGAA CGCGCTGGAA GGTATGCTGG 50
ATACGCTGAA GAAAGATATC GCCCTGGGCG AAGAGTTCGT TAATAAGTAA 100
<210> 11798
<211> 100
<212> DNA
<213> Escherichia coli K-12 MG1655
<220> mdh b3237
<222> 791-890
<223> mdh b3237 U00096 complement(3380965..3381903)
<313> 11798: 824-843
<400> 11798
TCTCTCAACC GCTGCTGCTG GGTAAAAACG GCGTGGAAGA GCGTAAATCT 50
ATCGGTACCC TGAGCGCATT TGAACAGAAC GCGCTGGAAG GTATGCTGGA 100
<210> 11799
<211> 100
<212> DNA
<213> Escherichia coli K-12 MG1655
<220> mdh b3237
<222> 753-852

<223> mdh b3237 U00096 complement(3380965..3381903)
<313> 11799: 781-800
<400> 11799
TGCCTACGTT GAAGGCGACG GTCAGTACGC CCGTTTCTTC TCTCAACCGC 50
TGCTGCTGGG TAAAAACGGC GTGGAAGAGC GTAAATCTAT CGGTACCCTG 100
<210> 11800
<211> 100
<212> DNA
<213> Escherichia coli K-12 MG1655
<220> tus b1611
<222> 831-930
<223> tus b1611 U00096 1682283..1683212
<313> 11800: 899-918
<400> 11800
GTTAAATTAC GATGCCGACA ATGTGCAGCA CCGTTATAAA CCTCAGGCGC 50
AGCCGCTTCG TTTGATCATT CCACGGCTGC ACCTGTATGT TGCAGATTAA 100
<210> 11801
<211> 100
<212> DNA
<213> Escherichia coli K-12 MG1655
<220> tus b1611
<222> 814-913
<223> tus b1611 U00096 1682283..1683212
<313> 11801: 850-869
<400> 11801
CCGGACGTTG GTGAGTTGTT AAATTACGAT GCCGACAATG TGCAGCACCG 50
TTATAAACCT CAGGCGCAGC CGCTTCGTTT GATCATTCCA CGGCTGCACC 100
<210> 11802
<211> 100
<212> DNA
<213> Escherichia coli K-12 MG1655
<220> tus b1611
<222> 734-833
<223> tus b1611 U00096 1682283..1683212
<313> 11802: 772-791
<400> 11802
AAGGAGATCA AAAACAAGTC CAACACGCCT GCCCTACACC ACTGATTGCA 50
CTGATTAATC GGGATAATGG CGCGGGCGTG CCGGACGTTG GTGAGTTGTT 100
<210> 11803
<211> 100
<212> DNA
<213> Escherichia coli K-12 MG1655
<220> mdla b0449
<222> 1658-1757
<223> mdla b0449 U00096 468095..469867
<313> 11803: 1722-1741
<400> 11803
ATCATGATGT GCTGGCACAA CAAAGCGGCT GGTATCGCGA TATGTATCGC 50
TATCAACAAC TGGAGGCGGC GCTCGACGAC GCTCCGGAAA ATCGCGAGGA 100
<210> 11804
<211> 100
<212> DNA
<213> Escherichia coli K-12 MG1655
<220> mdla b0449
<222> 1602-1701
<223> mdla b0449 U00096 468095..469867
<313> 11804: 1638-1657
<400> 11804
GACGGAAGCC AGTGAAATTA TTGTGATGCA GCACGGACAT ATCGCCCAGC 50
GTGGCAATCA TGATGTGCTG GCACAACAAA GCGGCTGGTA TCGCGATATG 100
<210> 11805
<211> 100
<212> DNA
<213> Escherichia coli K-12 MG1655
<220> mdla b0449
<222> 1536-1635
<223> mdla b0449 U00096 468095..469867
<313> 11805: 1584-1603
<400> 11805
GCATAACCTG CGTCAGTGGG GGCAGGGAAG AACGGTAATC ATCAGTGCCC 50
ATCGCCTTTC TGCACTGACG GAAGCCAGTG AAATTATTGT GATGCAGCAC 100

<210> 11806
<211> 100
<212> DNA
<213> Escherichia coli K-12 MG1655
<220> mdlb b0450
<222> 1632-1731
<223> mdlb b0450 U00096 469860..471641
<313> 11806: 1661-1680
<400> 11806
TCTGGTGCTT CATCGTGGGC AAGCCGTGGA GCAGGGCACT CACCAGCAAC 50
TGCTGGCGGC CCAGGGACGC TACTGGCAGA TGTATCAACT GCAACTTGCG 100
<210> 11807
<211> 100
<212> DNA
<213> Escherichia coli K-12 MG1655
<220> mdlb b0450
<222> 1585-1684
<223> mdlb b0450 U00096 469860..471641
<313> 11807: 1620-1639
<400> 11807
GTAGTGATTG CTCACCGCTT ATCGACCATT GTTGATGCCG ACACCATTCT 50
GGTGCTTCAT CGTGGGCAAG CCGTGGAGCA GGGCACTCAC CAGCAACTGC 100
<210> 11808
<211> 100
<212> DNA
<213> Escherichia coli K-12 MG1655
<220> mdlb b0450
<222> 1536-1635
<223> mdlb b0450 U00096 469860..471641
<313> 11808: 1566-1585
<400> 11808
ACAGGCGATT CAACATGCTC TGGCGGCGGT GCGTGAACAT ACCACGCTGG 50
TAGTGATTGC TCACCGCTTA TCGACCATTG TTGATGCCGA CACCATTCTG 100
<210> 11809
<211> 100
<212> DNA
<213> Escherichia coli K-12 MG1655
<220> ygba b2733
<222> 255-354
<223> ygba b2733 U00096 complement(2854476..2854829)
<313> 11809: 324-343
<400> 11809
GTGGCGTCAT CCGATCTTAA CCGTGCGTCA TCTCATCGAT GACAAGCGTC 50
CGGTGCCAGA ACTGCCGGAA AAATATCGTC CGAAAAAACC TCATGAATAG 100
<210> 11810
<211> 100
<212> DNA
<213> Escherichia coli K-12 MG1655
<220> ygba b2733
<222> 181-280
<223> ygba b2733 U00096 complement(2854476..2854829)
<313> 11810: 221-240
<400> 11810
CCGGTGCACT GTTACCAGCC TGCAAAGCGT GAAGAGATGA AACAGATTAT 50
GCGTTGGGCC GGGCCGAGAA TGCTGTGGCG TCATCCGATC TTAACCGTGC 100
<210> 11811
<211> 100
<212> DNA
<213> Escherichia coli K-12 MG1655
<220> ygba b2733
<222> 132-231
<223> ygba b2733 U00096 complement(2854476..2854829)
<313> 11811: 157-176
<400> 11811
GCTGGATAAA TGTGTGTTCG GCGAAGAGAA ACCAGCCTGT AAGCAGTGTC 50
CGGTGCACTG TTACCAGCCT GCAAAGCGTG AAGAGATGAA ACAGATTATG 100
<210> 11812
<211> 100
<212> DNA
<213> Escherichia coli K-12 MG1655
<220> ygbb b2747

<222> 381-480
<223> ygbb b2747 U00096 complement(2869324..2869803)
<313> 11812: 419-438
<400> 11812
TAACGTGAAA GCCACTACTA CGGAAAAACT GGGATTTACC GGACGTGGGG 50
AAGGGATTGC CTGTGAAGCG GTGGCGCTAC TCATTAAGGC AACAAAATGA 100
<210> 11813
<211> 100
<212> DNA
<213> Escherichia coli K-12 MG1655
<220> ygbb b2747
<222> 288-387
<223> ygbb b2747 U00096 complement(2869324..2869803)
<313> 11813: 303-322
<400> 11813
CACTATCATC GCTCAGGCAC CGAAGATGTT GCCGCACATT CCACAAATGC 50
GCGTGTTTAT TGCCGAAGAT CTCGGCTGCC ATATGGATGA TGTTAACGTG 100
<210> 11814
<211> 100
<212> DNA
<213> Escherichia coli K-12 MG1655
<220> ygbb b2747
<222> 169-268
<223> ygbb b2747 U00096 complement(2869324..2869803)
<313> 11814: 224-243
<400> 11814
ATCGGCAAGC TGTTCCCGGA TACCGATCCG GCATTTAAAG GTGCCGATAG 50
CCGCGAGCTG CTACGCGAAG CCTGGCGTCG TATTCAGGCG AAGGGTTATA 100
<210> 11815
<211> 100
<212> DNA
<213> Escherichia coli K-12 MG1655
<220> ygbd b2712
<222> 1035-1134
<223> ygbd b2712 U00096 2831935..2833068
<313> 11815: 1079-1098
<400> 11815
GGCGCGCGGC GTTGACGATG CTGACCAGCT TCGCGCCTTT GTGGTCAGTG 50
AGGATCGGAT GAAAGAGGCA TTTGGATTGT TGAAAACATT GCCGATGTAG 100
<210> 11816
<211> 100
<212> DNA
<213> Escherichia coli K-12 MG1655
<220> ygbd b2712
<222> 976-1075
<223> ygbd b2712 U00096 2831935..2833068
<313> 11816: 1001-1020
<400> 11816
GGCGAAACCC AGCGTCAGGA TTTACGCTGG CAAATTAATA CCGAACGCCA 50
GGGAATGGTG GCGCGCGGCG TTGACGATGC TGACCAGCTT CGCGCCTTTG 100
<210> 11817
<211> 100
<212> DNA
<213> Escherichia coli K-12 MG1655
<220> ygbd b2712
<222> 912-1011
<223> ygbd b2712 U00096 2831935..2833068
<313> 11817: 968-987
<400> 11817
GCCGCTGAAA CTCCCGGCGA TGCTGGTGAA AATCAAAACG CCGGAATTAC 50
CGCTGCATCT GGCAGGCGAA ACCCAGCGTC AGGATTTACG CTGGCAAATT 100
<210> 11818
<211> 100
<212> DNA
<213> Escherichia coli K-12 MG1655
<220> ygbe b2750
<222> 225-324
<223> ygbe b2750 U00096 complement(2871037..2871360)
<313> 11818: 283-302
<400> 11818
GCATTCATTG ATGGACGGAA AGCTACGCTA CAGTATTGTT TTCACCCTGG 50

TGACAGTAGG CATTATGTTT GGCGCACTGT TTATGTGGCT ACTGGGCTAA 100
<210> 11819
<211> 100
<212> DNA
<213> Escherichia coli K-12 MG1655
<220> ygbe b2750
<222> 131-230
<223> ygbe b2750 U00096 complement(2871037..2871360)
<313> 11819: 166-185
<400> 11819
TGATTTATGG CTCTAACACG CTGTTCTTCT TTATCTACAC CTGGCCTTTC 50
TTTCTGGCGC TGATGCCCGT CGCGGTAGTG GTGGGGATTG CGCTGCATTC 100
<210> 11820
<211> 100
<212> DNA
<213> Escherichia coli K-12 MG1655
<220> ygbe b2750
<222> 19-118
<223> ygbe b2750 U00096 complement(2871037..2871360)
<313> 11820: 54-73
<400> 11820
ATTACACTAA CAAATAACGA CAGCCTTACT GAGGATGAAG AAACCACATG 50
GTCACTGCCT GGTGCCGTGG TCGGTTTTAT CTCCTGGTTA TTTGCGCTGG 100
<210> 11821
<211> 100
<212> DNA
<213> Escherichia coli K-12 MG1655
<220> ygbf ygbF
<222> 252-351
<223> ygbf ygbF U00096 complement(2876592..2876942)
<313> 11821: 306-325
<400> 11821
GAATACGGAA ACGGGATTTG AGTTCCAGAC ATTTGGGTTA AACAGGCGTA 50
CCCCGGTAGA TTTGGATGGT TTAAGGTTGG TGTCTTTTTT ACCTGTTTGA 100
<210> 11822
<211> 100
<212> DNA
<213> Escherichia coli K-12 MG1655
<220> ygbf ygbF
<222> 201-300
<223> ygbf ygbF U00096 complement(2876592..2876942)
<313> 11822: 237-256
<400> 11822
ACAAATAGCT GGACTGGCGG AAGAAGGCAA TGTAGTGATG GCATGGGCAA 50
CGAATACGGA AACGGGATTT GAGTTCCAGA CATTTGGGTT AAACAGGCGT 100
<210> 11823
<211> 100
<212> DNA
<213> Escherichia coli K-12 MG1655
<220> ygbf ygbF
<222> 166-265
<223> ygbf ygbF U00096 complement(2876592..2876942)
<313> 11823: 210-229
<400> 11823
GATGTATCCG CAAAAATTCG TGAAATGATC TGGGAACAAA TAGCTGGACT 50
GGCGGAAGAA GGCAATGTAG TGATGGCATG GGCAACGAAT ACGGAAACGG 100
<210> 11824
<211> 100
<212> DNA
<213> Escherichia coli K-12 MG1655
<220> ygbi ygbI
<222> 669-768
<223> ygbi ygbI U00096 complement(2858490..2859287)
<313> 11824: 722-741
<400> 11824
GGTGGCGACA TGGCTGGCGT TACCATTAAG CGAGTTTGAT CAGATTATTA 50
CAGACGACGG TTTGCCGGAG AGTGCCAGTC GCGCGCTGGT GAAGCAGGAT 100
<210> 11825
<211> 100
<212> DNA
<213> Escherichia coli K-12 MG1655

<220> ygbi ygbI
<222> 634-733
<223> ygbi ygbI U00096 complement(2858490..2859287)
<313> 11825: 657-676
<400> 11825
CGAGTTTTGG TCTGTGATGC GACGAAATAT GGTCAGGTGG CGACATGGCT 50
GGCGTTACCA TTAAGCGAGT TTGATCAGAT TATTACAGAC GACGGTTTGC 100
<210> 11826
<211> 100
<212> DNA
<213> Escherichia coli K-12 MG1655
<220> ygbi ygbI
<222> 547-646
<223> ygbi ygbI U00096 complement(2858490..2859287)
<313> 11826: 578-597
<400> 11826
GCATCGTCAT GGAGTGTGCG GGGGATTTCT ACGCCAGCGG AAGATAAAGT 50
CACGGTGAAA CGGGCGATTG CCAGTGCCAG CCGCCAGCGA GTTTTGGTCT 100
<210> 11827
<211> 100
<212> DNA
<213> Escherichia coli K-12 MG1655
<220> ygbl b2739
<222> 535-634
<223> ygbl b2739 U00096 2861616..2862254
<313> 11827: 586-605
<400> 11827
AACAATATGG AAGAGCTGGA GGAAACGGCA AAGCTGATTT TTATTCTCGG 50
TGACCGCCCG ATCCGTTATC TGACCGCAGG TGAAATTGCG GAATTAAGGA 100
<210> 11828
<211> 100
<212> DNA
<213> Escherichia coli K-12 MG1655
<220> ygbl b2739
<222> 521-620
<223> ygbl b2739 U00096 2861616..2862254
<313> 11828: 554-573
<400> 11828
TGCAAGAAGC CGCCAACAAT ATGGAAGAGC TGGAGGAAAC GGCAAAGCTG 50
ATTTTTATTC TCGGTGACCG CCCGATCCGT TATCTGACCG CAGGTGAAAT 100
<210> 11829
<211> 100
<212> DNA
<213> Escherichia coli K-12 MG1655
<220> ygbl b2739
<222> 452-551
<223> ygbl b2739 U00096 2861616..2862254
<313> 11829: 475-494
<400> 11829
CGGAACTGGC AGCAGACAAT CAGGCTTTTT TACTGGCAAA TCATGGCCCA 50
GTGGTTTGCG GTGAAAGCCT GCAAGAAGCC GCCAACAATA TGGAAGAGCT 100
<210> 11830
<211> 100
<212> DNA
<213> Escherichia coli K-12 MG1655
<220> ygbm ygbM
<222> 678-777
<223> ygbm ygbM U00096 2862259..2863035
<313> 11830: 743-762
<400> 11830
CCTGTTCGAT GAGGTGGGAT ATCAGGGCTG GATCGGTTGT GAATATAAAC 50
CTCGTGGCCT CACCGAAGAA GGGCTTGGCT GGTTTGACGC CTGGCGCTAA 100
<210> 11831
<211> 100
<212> DNA
<213> Escherichia coli K-12 MG1655
<220> ygbm ygbM
<222> 677-776
<223> ygbm ygbM U00096 2862259..2863035
<313> 11831: 698-717
<400> 11831

GCCTGTTCGA TGAGGTGGGA TATCAGGGCT GGATCGGTTG TGAATATAAA 50
CCTCGTGGCC TCACCGAAGA AGGGCTTGGC TGGTTTGACG CCTGGCGCTA 100
<210> 11832
<211> 100
<212> DNA
<213> Escherichia coli K-12 MG1655
<220> ygbm ygbM
<222> 614-713
<223> ygbm ygbM U00096 2862259..2863035
<313> 11832: 668-687
<400> 11832
TTGCCGGACT ACCTGATCGA CATGAACCGG ACGATGGAGA AATCAACTAT 50
CCGTGGCTGT TCCGCCTGTT CGATGAGGTG GGATATCAGG GCTGGATCGG 100
<210> 11833
<211> 100
<212> DNA
<213> Escherichia coli K-12 MG1655
<220> ygbo b2746
<222> 939-1038
<223> ygbo b2746 U00096 complement(2868278..2869327)
<313> 11833: 968-987
<400> 11833
GGATGACGTC ACCGTAGAGA TCCGTTTCTG GCTTCCGGCG GGTAGTTTTG 50
CAACCAGCGT TGTCAGGGAA CTTATCAACA CAACAGGTGA TTATGCGCAT 100
<210> 11834
<211> 100
<212> DNA
<213> Escherichia coli K-12 MG1655
<220> ygbo b2746
<222> 862-961
<223> ygbo b2746 U00096 complement(2868278..2869327)
<313> 11834: 913-932
<400> 11834
TTACTGGTGC GCGAAAAAGT TGAAGCCGCG CGCAGAGCGA TGCTGCTGTA 50
TCCGCAACAA TTAAGCTGGA ATTGGTGGGA TGACGTCACC GTAGAGATCC 100
<210> 11835
<211> 100
<212> DNA
<213> Escherichia coli K-12 MG1655
<220> ygbo b2746
<222> 839-938
<223> ygbo b2746 U00096 complement(2868278..2869327)
<313> 11835: 853-872
<400> 11835
CCGCAGAAAC TGAATTACAA GCTTTACTGG TGCGCGAAAA AGTTGAAGCC 50
GCGCGCAGAG CGATGCTGCT GTATCCGCAA CAATTAAGCT GGAATTGGTG 100
<210> 11836
<211> 100
<212> DNA
<213> Escherichia coli K-12 MG1655
<220> ygbp ygbP
<222> 612-711
<223> ygbp ygbP U00096 complement(2869803..2870513)
<313> 11836: 674-693
<400> 11836
GGTCGAAGGC CGTGCGGATA ACATTAAAGT CACGCGCCCG GAAGATTTGG 50
CACTGGCCGA GTTTTACCTC ACCCGAACCA TCCATCAGGA GAATACATAA 100
<210> 11837
<211> 100
<212> DNA
<213> Escherichia coli K-12 MG1655
<220> ygbp ygbP
<222> 588-687
<223> ygbp ygbP U00096 complement(2869803..2870513)
<313> 11837: 633-652
<400> 11837
ATATTGCGGA TTCCATCCTC AGTTGGTCGA AGGCCGTGCG GATAACATTA 50
AAGTCACGCG CCCGGAAGAT TTGGCACTGG CCGAGTTTTA CCTCACCCGA 100
<210> 11838
<211> 100
<212> DNA

<213> Escherichia coli K-12 MG1655
<220> ygbp ygbP
<222> 545-644
<223> ygbp ygbP U00096 complement(2869803..2870513)
<313> 11838: 606-625
<400> 11838
TAAATGAAGG CGCGACTATT ACCGACGAAG CCTCGGCGCT GGAATATTGC 50
GGATTCCATC CTCAGTTGGT CGAAGGCCGT GCGGATAACA TTAAAGTCAC 100
<210> 11839
<211> 100
<212> DNA
<213> Escherichia coli K-12 MG1655
<220> b2736 b2736
<222> 810-909
<223> b2736 b2736 U00096 2859453..2860361
<313> 11839: 888-907
<400> 11839
TATGTTCACC AGCGCCAGTA ACGCGGGTTA CGGGAAAGAA GACGATAGCG 50
CAGTTATCAA GATTTTCTCT GGCATCACTC TACCGGGAGC GAAATCATGA 100
<210> 11840
<211> 100
<212> DNA
<213> Escherichia coli K-12 MG1655
<220> b2736 b2736
<222> 795-894
<223> b2736 b2736 U00096 2859453..2860361
<313> 11840: 837-856
<400> 11840
CTCAACAGCA TTGAATATGT TCACCAGCGC CAGTAACGCG GGTTACGGGA 50
AAGAAGACGA TAGCGCAGTT ATCAAGATTT TCTCTGGCAT CACTCTACCG 100
<210> 11841
<211> 100
<212> DNA
<213> Escherichia coli K-12 MG1655
<220> b2736 b2736
<222> 736-835
<223> b2736 b2736 U00096 2859453..2860361
<313> 11841: 794-813
<400> 11841
AAGGATCTTG GTCTGGTTGC CGATACAGCC AAAGCCCTGC ACTTCCCGCT 50
GCCATTGGCC TCAACAGCAT TGAATATGTT CACCAGCGCC AGTAACGCGG 100
<210> 11842
<211> 100
<212> DNA
<213> Escherichia coli K-12 MG1655
<220> ccma b2202
<222> 519-618
<223> ccma b2202 U00096 complement(2295041..2295658)
<313> 11842: 592-611
<400> 11842
GGAGCAGGGG GGGATTGTGA TTCTGACTAC CCACCAGCCG CTCAACGTTG 50
CTGAAAGTAA AATTCGCCGC ATTTCACTGA CGCAAACGAG GGCCGCATGA 100
<210> 11843
<211> 100
<212> DNA
<213> Escherichia coli K-12 MG1655
<220> ccma b2202
<222> 375-474
<223> ccma b2202 U00096 complement(2295041..2295658)
<313> 11843: 411-430
<400> 11843
TCAGCTCTCG GCCGGGCAAC AACGCCGCGT CGCTTTAGCG CGTCTGTGGC 50
TGACCCGTGC CACGTTATGG ATCCTCGACG AGCCTTTTAC CGCGATTGAC 100
<210> 11844
<211> 100
<212> DNA
<213> Escherichia coli K-12 MG1655
<220> ccma b2202
<222> 263-362
<223> ccma b2202 U00096 complement(2295041..2295658)
<313> 11844: 317-336

<400> 11844
GGCTGACGGC GTTAGAAAAT CTGCACTTTT ATCATCGCGA TGGCGATACC  50
GCACAATGTC TGGAAGCCCT GGCGCAGGCC GGGCTTGCCG GATTCGAAGA  100
<210> 11845
<211> 100
<212> DNA
<213> Escherichia coli K-12 MG1655
<220> b2737 b2738
<222> 1068-1167
<223> b2737 b2738 U00096 2860358..2861524
<313> 11845: 1121-1140
<400> 11845
TATTGTCGCA GGCGGTGAGA CCTCCGGCGT AGTCACACAG AGCCTGGGAA  50
TAAAAGGGTT TCATATTGGC CCAACCATTT CCCCGGCGTG CCGTGGGTAA  100
<210> 11846
<211> 100
<212> DNA
<213> Escherichia coli K-12 MG1655
<220> b2737 b2738
<222> 1066-1165
<223> b2737 b2738 U00096 2860358..2861524
<313> 11846: 1081-1100
<400> 11846
TTTATTGTCG CAGGCGGTGA GACCTCCGGC GTAGTCACAC AGAGCCTGGG  50
AATAAAAGGG TTTCATATTG GCCCAACCAT TTCCCCGGCG TGCCGTGGGT  100
<210> 11847
<211> 100
<212> DNA
<213> Escherichia coli K-12 MG1655
<220> b2737 b2738
<222> 978-1077
<223> b2737 b2738 U00096 2860358..2861524
<313> 11847: 1035-1054
<400> 11847
GCAATACGGT GCACAAAAAG CCAGTCAGGC AGTAGAAACA CTGTTTTCTC  50
AACTAGCGGC GCGGTTAGCA GCGGAAGGCG TGACACGCTT TATTGTCGCA  100
<210> 11848
<211> 100
<212> DNA
<213> Escherichia coli K-12 MG1655
<220> ccmb b2201
<222> 564-663
<223> ccmb b2201 U00096 complement(2294382..2295044)
<313> 11848: 637-656
<400> 11848
CGTTGACGGG TATCTGGCAA TTTTAGGCGC GTTGCTGGCA GGCACCGCGA  50
CATTAAGTCC TTTTGCGACG GCGGCAGCGT TACGAATCAG CATTCAATAA  100
<210> 11849
<211> 100
<212> DNA
<213> Escherichia coli K-12 MG1655
<220> ccmb b2201
<222> 483-582
<223> ccmb b2201 U00096 complement(2294382..2295044)
<313> 11849: 513-532
<400> 11849
CAGCATACTG GTGTTACCGC TGACTATCCC ATTACTCATC TTTGCCACCG  50
CCGCGATGGA CGCGGCTTCT ATGCATTTGC CCGTTGACGG GTATCTGGCA  100
<210> 11850
<211> 100
<212> DNA
<213> Escherichia coli K-12 MG1655
<220> ccmb b2201
<222> 363-462
<223> ccmb b2201 U00096 complement(2294382..2295044)
<313> 11850: 392-411
<400> 11850
GGATGTTTAT GGCTGGCAAG TGATGGCGCT GACGCTGCTG CTGGGAACGC  50
CTACGCTTGG CTTTCTCGGT GCACCGGGCG TGGCGCTGAC AGTGGGACTT  100
<210> 11851
<211> 100

<212> DNA
<213> Escherichia coli K-12 MG1655
<220> ccmc b2200
<222> 639-738
<223> ccmc b2200 U00096 complement(2293603..2294340)
<313> 11851: 707-726
<400> 11851
TGCCACGCTG ACGCTGATGC GGATGCGTAA TTTGATTTTG CTGATGGAAA 50
AACGCCGTCC GTGGGTGAGT GAACTGATAC TGAAAAGAGG CCGTAAATGA 100
<210> 11852
<211> 100
<212> DNA
<213> Escherichia coli K-12 MG1655
<220> ccmc b2200
<222> 613-712
<223> ccmc b2200 U00096 complement(2293603..2294340)
<313> 11852: 672-691
<400> 11852
TCGATTTTTG GCTTCCTGCT CCTGTCTGCC ACGCTGACGC TGATGCGGAT 50
GCGTAATTTG ATTTTGCTGA TGGAAAAACG CCGTCCGTGG GTGAGTGAAC 100
<210> 11853
<211> 100
<212> DNA
<213> Escherichia coli K-12 MG1655
<220> ccmc b2200
<222> 581-680
<223> ccmc b2200 U00096 complement(2293603..2294340)
<313> 11853: 624-643
<400> 11853
TCGATCCGGC GATGCGTTCG CCGCTGCGCT GGTCGATTTT TGGCTTCCTG 50
CTCCTGTCTG CCACGCTGAC GCTGATGCGG ATGCGTAATT TGATTTTGCT 100
<210> 11854
<211> 100
<212> DNA
<213> Escherichia coli K-12 MG1655
<220> ccmd b2199
<222> 19-118
<223> ccmd b2199 U00096 complement(2293397..2293606)
<313> 11854: 38-57
<400> 11854
TCCTGGAATG AATTTTTCGC AATGGGCGGT TACGCCTTTT TTGTCTGGCT 50
GGCGGTGGTG ATGACCGTTA TTCCGCTGGT GGTTTTGGTC GTGCACTCGG 100
<210> 11855
<211> 100
<212> DNA
<213> Escherichia coli K-12 MG1655
<220> ccmd b2199
<222> 1-100
<223> ccmd b2199 U00096 complement(2293397..2293606)
<313> 11855: 25-44
<400> 11855
ATGACCCCTG CATTTGCTTC CTGGAATGAA TTTTTCGCAA TGGGCGGTTA 50
CGCCTTTTTT GTCTGGCTGG CGGTGGTGAT GACCGTTATT CCGCTGGTGG 100
<210> 11856
<211> 100
<212> DNA
<213> Escherichia coli K-12 MG1655
<220> ccmd b2199
<222> 1-100
<223> ccmd b2199 U00096 complement(2293397..2293606)
<313> 11856: 22-41
<400> 11856
ATGACCCCTG CATTTGCTTC CTGGAATGAA TTTTTCGCAA TGGGCGGTTA 50
CGCCTTTTTT GTCTGGCTGG CGGTGGTGAT GACCGTTATT CCGCTGGTGG 100
<210> 11857
<211> 100
<212> DNA
<213> Escherichia coli K-12 MG1655
<220> ccme b2198
<222> 381-480
<223> ccme b2198 U00096 complement(2292921..2293400)

<313> 11857: 443-462
<400> 11857
GGCGAAACAC GATGAAAACT ATACGCCGCC AGAAGTTGAG AAAGCGATGG 50
AAGCTAACCA CCGTCGCCCG GCGAGTGTTT ATAAGGACCC AGCATCATGA 100
<210> 11858
<211> 100
<212> DNA
<213> Escherichia coli K-12 MG1655
<220> ccme b2198
<222> 368-467
<223> ccme b2198 U00096 complement(2292921..2293400)
<313> 11858: 409-428
<400> 11858
CGAAAGAAGT GCTGGCGAAA CACGATGAAA ACTATACGCC GCCAGAAGTT 50
GAGAAAGCGA TGGAAGCTAA CCACCGTCGC CCGGCGAGTG TTTATAAGGA 100
<210> 11859
<211> 100
<212> DNA
<213> Escherichia coli K-12 MG1655
<220> ccme b2198
<222> 282-381
<223> ccme b2198 U00096 complement(2292921..2293400)
<313> 11859: 331-350
<400> 11859
TTACGAAGGC ATTTTGCCGG ATCTGTTCCG TGAAGGGCAG GGCGTTGTGG 50
TGCAGGGCGA ACTGGAAAAA GGCAATCATA TCCTCGCGAA AGAAGTGCTG 100
<210> 11860
<211> 100
<212> DNA
<213> Escherichia coli K-12 MG1655
<220> ccmf b2197
<222> 1845-1944
<223> ccmf b2197 U00096 complement(2290981..2292924)
<313> 11860: 1924-1943
<400> 11860
GGGCGGGCTG ATGATGGCGT TGGGCGGACT GCTGTGTCTG TTTGATCCTC 50
GCTATCGTAA GCGCGTGAGT CCGCAAAAAA CTGCGCCGGA GGCCGTATGA 100
<210> 11861
<211> 100
<212> DNA
<213> Escherichia coli K-12 MG1655
<220> ccmf b2197
<222> 1794-1893
<223> ccmf b2197 U00096 complement(2290981..2292924)
<313> 11861: 1849-1868
<400> 11861
CGCGTGGGCC GTGCGTCTTT ACTACAAACC ATTTGTTCGC TGGATTTGGG 50
CGGGCGGGCT GATGATGGCG TTGGGCGGAC TGCTGTGTCT GTTTGATCCT 100
<210> 11862
<211> 100
<212> DNA
<213> Escherichia coli K-12 MG1655
<220> ccmf b2197
<222> 1766-1865
<223> ccmf b2197 U00096 complement(2290981..2292924)
<313> 11862: 1816-1835
<400> 11862
CGGCCCTCGG TGAAGAGCTG GAAAACGGCG CGTGGGCCGT GCGTCTTTAC 50
TACAAACCAT TTGTTCGCTG GATTTGGGCG GGCGGGCTGA TGATGGCGTT 100
<210> 11863
<211> 100
<212> DNA
<213> Escherichia coli K-12 MG1655
<220> ccmh b2195
<222> 914-1013
<223> ccmh b2195 U00096 complement(2289378..2290430)
<313> 11863: 963-982
<400> 11863
AGCAGCAGCG ATTTGGCGAA GCCGTTGCCG CGTGGGAGAT GATGTTGAAA 50
CTCTTACCTG CCAACGATAC TCGCCGTGCG GTGATTGAAC GTAGTATCGC 100
<210> 11864

<211> 100
<212> DNA
<213> Escherichia coli K-12 MG1655
<220> ccmh b2195
<222> 886-985
<223> ccmh b2195 U00096 complement(2289378..2290430)
<313> 11864: 901-920
<400> 11864
CTAAGCATGT ATGCGTTTAA TGCCTTTGAG CAGCAGCGAT TTGGCGAAGC 50
CGTTGCCGCG TGGGAGATGA TGTTGAAACT CTTACCTGCC AACGATACTC 100
<210> 11865
<211> 100
<212> DNA
<213> Escherichia coli K-12 MG1655
<220> ccmh b2195
<222> 811-910
<223> ccmh b2195 U00096 complement(2289378..2290430)
<313> 11865: 867-886
<400> 11865
GATCCCAACG ACAACCGCCT CGGCGGTGAA CTGCTACGTC AGCTGGTGAG 50
AACGGACCAT AGCAATATCC GTGTGCTAAG CATGTATGCG TTTAATGCCT 100
<210> 11866
<211> 100
<212> DNA
<213> Escherichia coli K-12 MG1655
<220> napa b2207
<222> 2388-2487
<223> napa b2207 U00096 complement(2298287..2300773)
<313> 11866: 2449-2468
<400> 11866
CGCCGCACAG TTGGTTAACA AACTGACGCT GGATGCGACC GATCCGCTCT 50
CGAAAGAGAC GGACTTCAAG AAGTGCGCGG TCAAACTGGA GAAGGTGTAA 100
<210> 11867
<211> 100
<212> DNA
<213> Escherichia coli K-12 MG1655
<220> napa b2207
<222> 2384-2483
<223> napa b2207 U00096 complement(2298287..2300773)
<313> 11867: 2409-2428
<400> 11867
TCGACGCCGC ACAGTTGGTT AACAAACTGA CGCTGGATGC GACCGATCCG 50
CTCTCGAAAG AGACGGACTT CAAGAAGTGC GCGGTCAAAC TGGAGAAGGT 100
<210> 11868
<211> 100
<212> DNA
<213> Escherichia coli K-12 MG1655
<220> napa b2207
<222> 2186-2285
<223> napa b2207 U00096 complement(2298287..2300773)
<313> 11868: 2243-2262
<400> 11868
GTATGACTCG CCGTGTACCA GAACTGCACC GCGCCTTCCC GGAAGCGGTC 50
CTGTTTATTC ACCCGCTGGA TGCGAAAGCG CGCGATCTGC GCCGTGGCGA 100
<210> 11869
<211> 100
<212> DNA
<213> Escherichia coli K-12 MG1655
<220> napb b2204
<222> 372-471
<223> napb b2204 U00096 complement(2296289..2296759)
<313> 11869: 414-433
<400> 11869
TCCGCGTCGT TATTTCTGTC TGCAATGTCA CGTACCGCAG GCCGATACCG 50
CGCCAATCGT GGGGAATACC TTTACCCCAT CAAAAGGTTA CGGGAAATAA 100
<210> 11870
<211> 100
<212> DNA
<213> Escherichia coli K-12 MG1655
<220> napb b2204
<222> 356-455

<223> napb b2204 U00096 complement(2296289..2296759)
<313> 11870: 373-392
<400> 11870
TGGGCGCGGA AGTGGCTCCG CGTCGTTATT TCTGTCTGCA ATGTCACGTA  50
CCGCAGGCCG ATACCGCGCC AATCGTGGGG AATACCTTTA CCCCATCAAA  100
<210> 11871
<211> 100
<212> DNA
<213> Escherichia coli K-12 MG1655
<220> napb b2204
<222> 271-370
<223> napb b2204 U00096 complement(2296289..2296759)
<313> 11871: 331-350
<400> 11871
TTGCAGTGCC ACGGTGTCGA AAGCTATCGC ACCACTGGCG CGCCGCGTAT  50
CAGTCCTACT CACTTTATGG ACAGCGACGG CAAAGTGGGC GCGGAAGTGG  100
<210> 11872
<211> 100
<212> DNA
<213> Escherichia coli K-12 MG1655
<220> napc b2203
<222> 504-603
<223> napc b2203 U00096 complement(2295677..2296279)
<313> 11872: 571-590
<400> 11872
GCATGACCAG GCGGTGAAAG ATGGGCAAAC CTGTATTGAT TGCCATAAAG  50
GGATAGCGCA CAAGCTGCCC GATATGCGTG AAGTCGAGCC AGGTTTTTAA  100
<210> 11873
<211> 100
<212> DNA
<213> Escherichia coli K-12 MG1655
<220> napc b2203
<222> 488-587
<223> napc b2203 U00096 complement(2295677..2296279)
<313> 11873: 533-552
<400> 11873
CGGTTGCCGC GAAGATGCAT GACCAGGCGG TGAAAGATGG GCAAACCTGT  50
ATTGATTGCC ATAAAGGGAT AGCGCACAAG CTGCCCGATA TGCGTGAAGT  100
<210> 11874
<211> 100
<212> DNA
<213> Escherichia coli K-12 MG1655
<220> napc b2203
<222> 417-516
<223> napc b2203 U00096 complement(2295677..2296279)
<313> 11874: 460-479
<400> 11874
GAAGGACAAT AACTCGCAGG AGTGCCGTAA CTGTCACAAC TTCGAGTATA  50
TGGATACAAC CGCCCAGAAA TCGGTTGCCG CGAAGATGCA TGACCAGGCG  100
<210> 11875
<211> 100
<212> DNA
<213> Escherichia coli K-12 MG1655
<220> napd b2208
<222> 165-264
<223> napd b2208 U00096 complement(2300770..2301033)
<313> 11875: 232-251
<400> 11875
GCTGATCCAA ACCATTGAGT CAGTACGCAA CGTAGAGGGC GTGCTGGCGG  50
TGTCGCTGGT TTATCACCAG CAGGAAGAGC AAGGTGAGGA AACACCATGA  100
<210> 11876
<211> 100
<212> DNA
<213> Escherichia coli K-12 MG1655
<220> napd b2208
<222> 125-224
<223> napd b2208 U00096 complement(2300770..2301033)
<313> 11876: 174-193
<400> 11876
GTCAGTTGAT TGTGGTGGTG GAAGCAGAAG ACAGCGAAAC GCTGATCCAA  50
ACCATTGAGT CAGTACGCAA CGTAGAGGGC GTGCTGGCGG TGTCGCTGGT  100

<210> 11877
<211> 100
<212> DNA
<213> Escherichia coli K-12 MG1655
<220> napd b2208
<222> 58-157
<223> napd b2208 U00096 complement(2300770..2301033)
<313> 11877: 79-98
<400> 11877
TCAGACATCA GCACCCAACT GAACGCCTTT CCCGGCTGTG AAGTTGCTGT  50
CAGCGACGCG CCGAGCGGTC AGTTGATTGT GGTGGTGGAA GCAGAAGACA  100
<210> 11878
<211> 100
<212> DNA
<213> Escherichia coli K-12 MG1655
<220> ygca b2786
<222> 1203-1302
<223> ygca b2786 U00096 complement(2911721..2913022)
<313> 11878: 1263-1282
<400> 11878
ATTAAAAGCA GGATATACCA TTGCGCGACT GGCGATGCTG GATATGTTCC  50
CACACACGGG ACATCTGGAA TCGATGGTAC TTTTCTCGCG CGTTAAATAG  100
<210> 11879
<211> 100
<212> DNA
<213> Escherichia coli K-12 MG1655
<220> ygca b2786
<222> 1160-1259
<223> ygca b2786 U00096 complement(2911721..2913022)
<313> 11879: 1196-1215
<400> 11879
TATCCTGTAA CCCTGCAACG CTGGCTCGGG ATAGCGAAGC GTTATTAAAA  50
GCAGGATATA CCATTGCGCG ACTGGCGATG CTGGATATGT TCCCACACAC  100
<210> 11880
<211> 100
<212> DNA
<213> Escherichia coli K-12 MG1655
<220> ygca b2786
<222> 1055-1154
<223> ygca b2786 U00096 complement(2911721..2913022)
<313> 11880: 1108-1127
<400> 11880
GGGCGAAAAA CGGCTTCGAT AAAGTGTTGC TGGACCCGGC GCGAGCAGGT  50
GCCGCAGGTG TTATGCAGCA AATTATAAAA CTGGAACCTA TTCGTATAGT  100
<210> 11881
<211> 100
<212> DNA
<213> Escherichia coli K-12 MG1655
<220> napf b2209
<222> 396-495
<223> napf b2209 U00096 complement(2301023..2301517)
<313> 11881: 465-484
<400> 11881
CCAGCCGCAA CTTAATAGCC AACTTTGCAA CGGATGTGGA GCATGTGCAG  50
CCAGCTGTCC GGTATCAGCC ATAACTGCGG AGTATCTCCA TGCACACTAA  100
<210> 11882
<211> 100
<212> DNA
<213> Escherichia coli K-12 MG1655
<220> napf b2209
<222> 370-469
<223> napf b2209 U00096 complement(2301023..2301517)
<313> 11882: 420-439
<400> 11882
TTTCGCCCGA CGCTCTCCGG GATCTACCAG CCGCAACTTA ATAGCCAACT  50
TTGCAACGGA TGTGGAGCAT GTGCAGCCAG CTGTCCGGTA TCAGCCATAA  100
<210> 11883
<211> 100
<212> DNA
<213> Escherichia coli K-12 MG1655
<220> napf b2209

<222> 328-427
<223> napf b2209 U00096 complement(2301023..2301517)
<313> 11883: 371-390
<400> 11883
TGCCGCCGCT GTCAGGATAG CTGTGAGCCA ATGGCAATTA TCTTTCGCCC 50
GACGCTCTCC GGGATCTACC AGCCGCAACT TAATAGCCAA CTTTGCAACG 100
<210> 11884
<211> 100
<212> DNA
<213> Escherichia coli K-12 MG1655
<220> ygcb b2762
<222> 2458-2557
<223> ygcb b2762 U00096 complement(2882576..2885242)
<313> 11884: 2508-2527
<400> 11884
GAACAGCAGT ATGAGGCGCT TGCACTTAAT CGCGTCAATG TACCCTTCAC 50
CTGGAAACGT AGTTTTTCTG AAGTAGTAGA TGAAGATGGG TTACTTTGGC 100
<210> 11885
<211> 100
<212> DNA
<213> Escherichia coli K-12 MG1655
<220> ygcb b2762
<222> 2401-2500
<223> ygcb b2762 U00096 complement(2882576..2885242)
<313> 11885: 2454-2473
<400> 11885
CAAACGTCTT CAGGTAAACA ACTGCTCGAT GGCCAGGTCT ACGAGGACCT 50
AAGTCATGAA CAGCAGTATG AGGCGCTTGC ACTTAATCGC GTCAATGTAC 100
<210> 11886
<211> 100
<212> DNA
<213> Escherichia coli K-12 MG1655
<220> ygcb b2762
<222> 2344-2443
<223> ygcb b2762 U00096 complement(2882576..2885242)
<313> 11886: 2372-2391
<400> 11886
ACCATTCTTG CGGTAACGAG GGATGGGGAA ATGAGCCTGC CATTATTGCC 50
TTATGTACAA ACGTCTTCAG GTAAACAACT GCTCGATGGC CAGGTCTACG 100
<210> 11887
<211> 100
<212> DNA
<213> Escherichia coli K-12 MG1655
<220> napg b2206
<222> 585-684
<223> napg b2206 U00096 complement(2297585..2298280)
<313> 11887: 642-661
<400> 11887
ATGCGTGCTG GAACAACCGG CAATCAAGGT GTTACCGCTG TCACTGGCGA 50
AAGGGGAGTT AGGTCACCAT TACCGCTTCG GCTGGCTGGA GGGGAACAAT 100
<210> 11888
<211> 100
<212> DNA
<213> Escherichia coli K-12 MG1655
<220> napg b2206
<222> 487-586
<223> napg b2206 U00096 complement(2297585..2298280)
<313> 11888: 529-548
<400> 11888
CTGGAGCTGG AGCGCAACAC GCGTACCGGT AAGCACGCCC GCTTTCTGCC 50
GACGGTTCAC AGCGACGCCT GTACCGGTTG CGGTAAGTGC GAAAAAGTAT 100
<210> 11889
<211> 100
<212> DNA
<213> Escherichia coli K-12 MG1655
<220> napg b2206
<222> 445-544
<223> napg b2206 U00096 complement(2297585..2298280)
<313> 11889: 483-502
<400> 11889
GTTTGTTATC GCGAATGCCC GAAAATTGAT GAGGCCATCA CCCTGGAGCT 50

GGAGCGCAAC ACGCGTACCG GTAAGCACGC CCGCTTTCTG CCGACGGTTC 100
<210> 11890
<211> 100
<212> DNA
<213> Escherichia coli K-12 MG1655
<220> naph b2205
<222> 727-826
<223> naph b2205 U00096 complement(2296735..2297598)
<313> 11890: 751-770
<400> 11890
CGTGCCCCGG TGCTGGATGA ACAAAGCCCG GTGCAGGTCA CCAGCCGCGA 50
TTGCATGACG TGCGGTCGCT GCGTGGATGT CTGTTCTGAG GATGTATTTA 100
<210> 11891
<211> 100
<212> DNA
<213> Escherichia coli K-12 MG1655
<220> naph b2205
<222> 636-735
<223> naph b2205 U00096 complement(2296735..2297598)
<313> 11891: 682-701
<400> 11891
CAAAGGTGTG ATTACCGTTG CGGCCACCGA TCGGCAGAAA TGTAACCGCT 50
GTATGGATTG TTTTCATGTT TGCCCGGAAC CGCATGTGCT ACGTGCCCCG 100
<210> 11892
<211> 100
<212> DNA
<213> Escherichia coli K-12 MG1655
<220> naph b2205
<222> 529-628
<223> naph b2205 U00096 complement(2296735..2297598)
<313> 11892: 557-576
<400> 11892
GGCGCGCTGC TTATTCTCGC GCTGTTTTTA TTTGATCTAC TGGTCGTTGA 50
ACACGGCTGG TGCGGGCACA TTTGTCCGGT TGGCGCGCTG TATGGCGTGC 100
<210> 11893
<211> 100
<212> DNA
<213> Escherichia coli K-12 MG1655
<220> fixa b0042
<222> 708-807
<223> fixa b0042 U00096 42367..43173
<313> 11893: 772-791
<400> 11893
TGCCGCGCCG AAACAGCGCG AACGTCAGCG CATCGTGATT GAAGGCGACG 50
GCGAAGAACA GATCGCCGCA TTTGCTGAAA ATCTTCGCAA AGTCATTTAA 100
<210> 11894
<211> 100
<212> DNA
<213> Escherichia coli K-12 MG1655
<220> fixa b0042
<222> 621-720
<223> fixa b0042 U00096 42367..43173
<313> 11894: 659-678
<400> 11894
TCTCGGCGCG GCGAAAAAGC CCGTCCAGGT ATGGTCGGCG GCGGATATTG 50
GTTTTAACGC AGAGGCAGCC TGGTCAGAAC AACAGGTTGC CGCGCCGAAA 100
<210> 11895
<211> 100
<212> DNA
<213> Escherichia coli K-12 MG1655
<220> fixa b0042
<222> 602-701
<223> fixa b0042 U00096 42367..43173
<313> 11895: 632-651
<400> 11895
TTCCTTCGAT GAAAGCCATT CTCGGCGCGG CGAAAAAGCC CGTCCAGGTA 50
TGGTCGGCGG CGGATATTGG TTTTAACGCA GAGGCAGCCT GGTCAGAACA 100
<210> 11896
<211> 100
<212> DNA
<213> Escherichia coli K-12 MG1655

<220> ygce b2777
<222> 1380-1479
<223> ygce b2777 U00096 2899918..2901396
<313> 11896: 1430-1449
<400> 11896
CCTAACCAAA CATACTGATG TGATCCTGAA AAAATCGTAT GAAGTGATGC 50
ATGGGGAATT GGGGAATGTG GATTCGATCC AGAGCTGGTC GAATGCGTAA 100
<210> 11897
<211> 100
<212> DNA
<213> Escherichia coli K-12 MG1655
<220> ygce b2777
<222> 1305-1404
<223> ygce b2777 U00096 2899918..2901396
<313> 11897: 1357-1376
<400> 11897
CGTGAAAGAT ATCTTTATAC CGATTGAGAG CAATGCCAAA CGCTACGACG 50
CGATGAATAA AGGCATTTTC AAAGACCTAA CCAAACATAC TGATGTGATC 100
<210> 11898
<211> 100
<212> DNA
<213> Escherichia coli K-12 MG1655
<220> ygce b2777
<222> 1204-1303
<223> ygce b2777 U00096 2899918..2901396
<313> 11898: 1242-1261
<400> 11898
AACGCCATTA ACGGTTGTGC AAGCCTGGGG GCAGCGATTA ATACAGCGGT 50
AGGTCTGGGG CTATACCCGG ATTACGCAAC GGCTGTCGAT AACATGGTTC 100
<210> 11899
<211> 100
<212> DNA
<213> Escherichia coli K-12 MG1655
<220> fixb b0043
<222> 821-920
<223> fixb b0043 U00096 43188..44129
<313> 11899: 862-881
<400> 11899
CGCAAACCAT TTTCGCCATC AATAAAGATA AAAATGCGCC GATCTTCCAG 50
TACGCGGATT ACGGCATTGT TGGCGACGCC GTGAAGATCC TTCCGGCGCT 100
<210> 11900
<211> 100
<212> DNA
<213> Escherichia coli K-12 MG1655
<220> fixb b0043
<222> 707-806
<223> fixb b0043 U00096 43188..44129
<313> 11900: 748-767
<400> 11900
AATGGATGGA GCACGAACGC TATGTCGGTA TCTCCAACCT GATGCTGAAA 50
CCTGAACTGT ACCTGGCGGT GGGGATCTCC GGGCAGATCC AGCACATGGT 100
<210> 11901
<211> 100
<212> DNA
<213> Escherichia coli K-12 MG1655
<220> fixb b0043
<222> 653-752
<223> fixb b0043 U00096 43188..44129
<313> 11901: 711-730
<400> 11901
GCAAGGCGAT AGGTGCGGAG TTGGCCTGTT CTCGTCCGGT GGCGGAAAAC 50
GAAAAATGGA TGGAGCACGA ACGCTATGTC GGTATCTCCA ACCTGATGCT 100
<210> 11902
<211> 100
<212> DNA
<213> Escherichia coli K-12 MG1655
<220> ygcf b2778
<222> 573-672
<223> ygcf b2778 U00096 complement(2902769..2903440)
<313> 11902: 634-653
<400> 11902

TAGCCAAAAG GATGATGCCA CACGTTTGTG CATTGAAACC TGCATTGCGC 50
GTAATTGGCG TTTGTCGATG CAAACACATA AATATCTAAA TATTGCCTGA 100
<210> 11903
<211> 100
<212> DNA
<213> Escherichia coli K-12 MG1655
<220> ygcf b2778
<222> 542-641
<223> ygcf b2778 U00096 complement(2902769..2903440)
<313> 11903: 580-599
<400> 11903
ATAAACCGCG AGTCATTGCA CTGCAGCCGA TTAGCCAAAA GGATGATGCC 50
ACACGTTTGT GCATTGAAAC CTGCATTGCG CGTAATTGGC GTTTGTCGAT 100
<210> 11904
<211> 100
<212> DNA
<213> Escherichia coli K-12 MG1655
<220> ygcf b2778
<222> 505-604
<223> ygcf b2778 U00096 complement(2902769..2903440)
<313> 11904: 545-564
<400> 11904
GAAGCACTGG ATGAACTACT GGCAACGCTG ACCGATGATA AACCGCGAGT 50
CATTGCACTG CAGCCGATTA GCCAAAAGGA TGATGCCACA CGTTTGTGCA 100
<210> 11905
<211> 100
<212> DNA
<213> Escherichia coli K-12 MG1655
<220> fixc b0044
<222> 1188-1287
<223> fixc b0044 U00096 44180..45466
<313> 11905: 1241-1260
<400> 11905
CGCGCCGGAA CTGATGCGCA AGAAAATCCT CCGCCACGGC AAGAAAGTGG 50
GCTTCATCAA TCTAATCAAG GATGGCATGA AAGGAGTGAC CGTTTTATGA 100
<210> 11906
<211> 100
<212> DNA
<213> Escherichia coli K-12 MG1655
<220> fixc b0044
<222> 1081-1180
<223> fixc b0044 U00096 44180..45466
<313> 11906: 1149-1168
<400> 11906
CGTATGTACC AGAAACTACC GGCGTTCCTT GATAACCCAC GCATGTTTAG 50
CGGCTACCCG GAGCTGGCGG TGGGTGTGGC GCGTGACCTG TTCACCATTG 100
<210> 11907
<211> 100
<212> DNA
<213> Escherichia coli K-12 MG1655
<220> fixc b0044
<222> 1072-1171
<223> fixc b0044 U00096 44180..45466
<313> 11907: 1107-1126
<400> 11907
CGCGATATGC GTATGTACCA GAAACTACCG GCGTTCCTTG ATAACCCACG 50
CATGTTTAGC GGCTACCCGG AGCTGGCGGT GGGTGTGGCG CGTGACCTGT 100
<210> 11908
<211> 100
<212> DNA
<213> Escherichia coli K-12 MG1655
<220> ygcg b2779
<222> 843-942
<223> ygcg b2779 U00096 2903664..2904605
<313> 11908: 893-912
<400> 11908
TCATTCAGAC AACAGCGGTT CCGCTGGAGG CTCAGATAGC GGCGGATTTA 50
GCGGCGGGGG CGGTTCTTCT GGAGGTGGCG GCGCATCCGG CCGCTGGTAA 100
<210> 11909
<211> 100
<212> DNA

<213> Escherichia coli K-12 MG1655
<220> ygcg b2779
<222> 658-757
<223> ygcg b2779 U00096 2903664..2904605
<313> 11909: 672-691
<400> 11909
GGCAGTAATT TTTGTCGCGC ATGTAAAAGT GGAGTCTGTA TTTCAGCAAT 50
CTATCTTTTA GATTTATTCC TGTTCTCCGA TAAAATTTTT TCCATTGCTG 100
<210> 11910
<211> 100
<212> DNA
<213> Escherichia coli K-12 MG1655
<220> ygcg b2779
<222> 505-604
<223> ygcg b2779 U00096 2903664..2904605
<313> 11910: 560-579
<400> 11910
GCTATAAACG CTTTGAATAA CCAACTCACT TCACAACACC AATATCCGAC 50
TAATCCTTCA GAAAGTGAAT CAGCGTCTTC CAGTGATCAT TATTACTTTG 100
<210> 11911
<211> 100
<212> DNA
<213> Escherichia coli K-12 MG1655
<220> ygck ygcK
<222> 333-432
<223> ygck ygcK U00096 complement(2880178..2880660)
<313> 11911: 368-387
<400> 11911
AGCCGATATG GTCCAGTTAC GTCGATTACT TACTCACGCC GAACCCGTAC 50
TTGACTGGCC ATTAATGGCC AGGATGTTGA CCTGGTGGGG AAAGCGCGAA 100
<210> 11912
<211> 100
<212> DNA
<213> Escherichia coli K-12 MG1655
<220> ygck ygcK
<222> 219-318
<223> ygck ygcK U00096 complement(2880178..2880660)
<313> 11912: 280-299
<400> 11912
ACATCAGGAC AAAAAATCGG AGCAAACAAC AGGTATCTCG TTGGGAAGAG 50
CTTTAGCCAA TAGTGGAAGA ATTAACGAGC GCCGTATCTT TCAATTAATT 100
<210> 11913
<211> 100
<212> DNA
<213> Escherichia coli K-12 MG1655
<220> ygck ygcK
<222> 200-299
<223> ygck ygcK U00096 complement(2880178..2880660)
<313> 11913: 233-252
<400> 11913
CAGGAAAGAA TGTCATCCGA CATCAGGACA AAAAATCGGA GCAAACAACA 50
GGTATCTCGT TGGGAAGAGC TTTAGCCAAT AGTGGAAGAA TTAACGAGCG 100
<210> 11914
<211> 100
<212> DNA
<213> Escherichia coli K-12 MG1655
<220> b2740 b2741
<222> 1266-1365
<223> b2740 b2741 U00096 2863124..2864488
<313> 11914: 1333-1352
<400> 11914
GTTGTCGGTA GCAGACGGTC TGAAAACCTG GACAGTGTTA ACGACCATTC 50
TCGGTTTTAC CGGATTCTTA ATTACCTGGT GCGTATGGGC GGTAATTTGA 100
<210> 11915
<211> 100
<212> DNA
<213> Escherichia coli K-12 MG1655
<220> b2740 b2741
<222> 1096-1195
<223> b2740 b2741 U00096 2863124..2864488
<313> 11915: 1141-1160

<400> 11915
CAGGGGTCAG CAACCGTAGC AATACTGACA ACCGGCGGGT TACTCTCAGA 50
AGCGGTGATG GGATTGAATC CGATTCAGTG CGTATTGGTG ACGCTGGCAG 100
<210> 11916
<211> 100
<212> DNA
<213> Escherichia coli K-12 MG1655
<220> b2740 b2741
<222> 1062-1161
<223> b2740 b2741 U00096 2863124..2864488
<313> 11916: 1110-1129
<400> 11916
CGCATTTATT ATTTCACTGG CGCTGCGTGC ATCGCAGGGG TCAGCAACCG 50
TAGCAATACT GACAACCGGC GGGTTACTCT CAGAAGCGGT GATGGGATTG 100
<210> 11917
<211> 100
<212> DNA
<213> Escherichia coli K-12 MG1655
<220> ygcm b2766
<222> 252-351
<223> ygcm b2766 U00096 2890237..2890602
<313> 11917: 299-318
<400> 11917
CAGCGAGGTT TTAGCAAAAT GGATTTGGGA TCAGGTTAAA CCCGTTGTGC 50
CGCTGTTAAG TGCGGTGATG GTAAAAGAAA CCTGCACCGC AGGTTGTATC 100
<210> 11918
<211> 100
<212> DNA
<213> Escherichia coli K-12 MG1655
<220> ygcm b2766
<222> 208-307
<223> ygcm b2766 U00096 2890237..2890602
<313> 11918: 257-276
<400> 11918
GATCACCATT ATCTCAATGA TATTCCAGGT CTGGAAAACC CAACCAGCGA 50
GGTTTTAGCA AAATGGATTT GGGATCAGGT TAAACCCGTT GTGCCGCTGT 100
<210> 11919
<211> 100
<212> DNA
<213> Escherichia coli K-12 MG1655
<220> ygcm b2766
<222> 154-253
<223> ygcm b2766 U00096 2890237..2890602
<313> 11919: 211-230
<400> 11919
ATTATCGATT TCGCTGAACT AAAAGCGGCG TTTAAACCAA CCTACGAGCG 50
CCTCGATCAC CATTATCTCA ATGATATTCC AGGTCTGGAA AACCCAACCA 100
<210> 11920
<211> 100
<212> DNA
<213> Escherichia coli K-12 MG1655
<220> ygcn b2767
<222> 1169-1268
<223> ygcn b2767 U00096 2890650..2891951
<313> 11920: 1227-1246
<400> 11920
ATATTTCCCG CGATTTATGG GATCAGGGTG ATAAACCTGT TCCACCGCTG 50
CGCCAGTTAT TCTGGCATCA TTTACGTCGT CACGGCCTGT GGCATCTGGC 100
<210> 11921
<211> 100
<212> DNA
<213> Escherichia coli K-12 MG1655
<220> ygcn b2767
<222> 1146-1245
<223> ygcn b2767 U00096 2890650..2891951
<313> 11921: 1193-1212
<400> 11921
TACGTGGCCT GCGTTAATGC AGGATATTTC CCGCGATTTA TGGGATCAGG 50
GTGATAAACC TGTTCCACCG CTGCGCCAGT TATTCTGGCA TCATTTACGT 100
<210> 11922
<211> 100

<212> DNA
<213> Escherichia coli K-12 MG1655
<220> ygcn b2767
<222> 1006-1105
<223> ygcn b2767 U00096 2890650..2891951
<313> 11922: 1048-1067
<400> 11922
CTGATAAGCG CCTGCCAGCA CCGCGAGCCG CAAAATCTGT TTCCGCTTTA 50
TCATCACAAC GTAGAGCGCA GCCTGCTGTG GGATGTTCTA CAGCGTTATC 100
<210> 11923
<211> 100
<212> DNA
<213> Escherichia coli K-12 MG1655
<220> b3472 b3473
<222> 513-612
<223> b3472 b3473 U00096 3607532..3608143
<313> 11923: 581-600
<400> 11923
TAATCAACTG CTGACCATGC AAATTACGCT GCCCGCTGAC GATCAGCAAA 50
AAGCGCAGAC CACCGCAGAA AACATCATTA ATACGCTGGT TATTCAGTAA 100
<210> 11924
<211> 100
<212> DNA
<213> Escherichia coli K-12 MG1655
<220> b3472 b3473
<222> 513-612
<223> b3472 b3473 U00096 3607532..3608143
<313> 11924: 531-550
<400> 11924
TAATCAACTG CTGACCATGC AAATTACGCT GCCCGCTGAC GATCAGCAAA 50
AAGCGCAGAC CACCGCAGAA AACATCATTA ATACGCTGGT TATTCAGTAA 100
<210> 11925
<211> 100
<212> DNA
<213> Escherichia coli K-12 MG1655
<220> b3472 b3473
<222> 420-519
<223> b3472 b3473 U00096 3607532..3608143
<313> 11925: 481-500
<400> 11925
TGAGCTGAAA GGTCACAAAA TGCAGCAGTT AGACAGTATT ATCTCCGCGA 50
AAGGCCAGAC GGCGTACTCT TCCGTTATTC TGGGTAACGT GGGTAATCAA 100
<210> 11926
<211> 100
<212> DNA
<213> Escherichia coli K-12 MG1655
<220> ygco b2768
<222> 184-283
<223> ygco b2768 U00096 2891906..2892202
<313> 11926: 228-247
<400> 11926
GACTATCGCA GTTGCCTGGA GTGTGGCACC TGCCGTTTGC TGTGCGACGA 50
ATCAACACTA CAACAGTGGC GCTATCCGCC TTCCGGATTC GGCATCACCT 100
<210> 11927
<211> 100
<212> DNA
<213> Escherichia coli K-12 MG1655
<220> ygco b2768
<222> 116-215
<223> ygco b2768 U00096 2891906..2892202
<313> 11927: 166-185
<400> 11927
CAGAACGGTT GATTAACGCC TGTCCGGCAG GTCTTTTTTC GCTCACACCG 50
GAAGGTAACT TACGTATTGA CTATCGCAGT TGCCTGGAGT GTGGCACCTG 100
<210> 11928
<211> 100
<212> DNA
<213> Escherichia coli K-12 MG1655
<220> ygco b2768
<222> 69-168
<223> ygco b2768 U00096 2891906..2892202

<313> 11928: 95-114
<400> 11928
TGATGCGCCG CACATTGTTC CGGCTGACTC CGTTGAGCGC CAGACGGCAG 50
AACGGTTGAT TAACGCCTGT CCGGCAGGTC TTTTTTCGCT CACACCGGAA 100
<210> 11929
<211> 100
<212> DNA
<213> Escherichia coli K-12 MG1655
<220> ygcp b2769
<222> 400-499
<223> ygcp b2769 U00096 2892219..2892794
<313> 11929: 442-461
<400> 11929
CTGCCAGGCT GTATGCCCAA AGTGCTGGGC TGGGTGACAG AGAAAATCCG 50
CCAACCGCTG ATTGCCGGTG GGCTGGTGTG CGATGAAGAA GATGCGCGTA 100
<210> 11930
<211> 100
<212> DNA
<213> Escherichia coli K-12 MG1655
<220> ygcp b2769
<222> 354-453
<223> ygcp b2769 U00096 2892219..2892794
<313> 11930: 404-423
<400> 11930
CATTGATAAG CAAGTTGCGC AATCGAATCC GGATTGTATT GAGATCCTGC 50
CAGGCTGTAT GCCCAAAGTG CTGGGCTGGG TGACAGAGAA AATCCGCCAA 100
<210> 11931
<211> 100
<212> DNA
<213> Escherichia coli K-12 MG1655
<220> ygcp b2769
<222> 197-296
<223> ygcp b2769 U00096 2892219..2892794
<313> 11931: 251-270
<400> 11931
GCGCGTCAAA TAAAGAAGTC GTTATTCAGT TTTTGAAACT GGTGACCGAG 50
GCGGACGGCA TTATCAGTAC CAAAGCCTCA ATGTTGAAAG CGGCAAGAGC 100
<210> 11932
<211> 100
<212> DNA
<213> Escherichia coli K-12 MG1655
<220> ygcq b2770
<222> 795-894
<223> ygcq b2770 U00096 complement(2892942..2893835)
<313> 11932: 830-849
<400> 11932
CAGCGCTGCG GTATTTTCAC AGGCAGATGT TGGCGTGGTT GATGACTGGA 50
AAGTGGTGCT GGAGGCACTG GTGACAAACA TTCACGCAGA TTGCCAATAA 100
<210> 11933
<211> 100
<212> DNA
<213> Escherichia coli K-12 MG1655
<220> ygcq b2770
<222> 686-785
<223> ygcq b2770 U00096 complement(2892942..2893835)
<313> 11933: 713-732
<400> 11933
GACATCTGCT GGCACCTGAG GTGTGTATTG TTGTTGGCGC TTCTGGCGCA 50
GCAGCGTTAA TGGCGGGCGT ACGCAATAGC AAGTTTGTGG TGGCGATTAA 100
<210> 11934
<211> 100
<212> DNA
<213> Escherichia coli K-12 MG1655
<220> ygcq b2770
<222> 594-693
<223> ygcq b2770 U00096 complement(2892942..2893835)
<313> 11934: 622-641
<400> 11934
GCTGGCAGAA AAGCTGGGAG CCGAAGTGGG CTACAGTCGG GCGCGAGTGA 50
TGAACGGTGG CGTTGATGCT GAAAAAGTGA TTGGTATTTC CGGACATCTG 100
<210> 11935

<211> 100
<212> DNA
<213> Escherichia coli K-12 MG1655
<220> ygcr b2771
<222> 669-768
<223> ygcr b2771 U00096 complement(2893799..2894584)
<313> 11935: 725-744
<400> 11935
TCTGCAACTG GCAAGAGCAG AGCAACGACG CGGAGCGACG CTGATTGATG 50
GGCAAACGGT AGCAGAAAAA GCTCAAAAGC TATGGCAGGA CTATTTACGC 100
<210> 11936
<211> 100
<212> DNA
<213> Escherichia coli K-12 MG1655
<220> ygcr b2771
<222> 631-730
<223> ygcr b2771 U00096 complement(2893799..2894584)
<313> 11936: 692-711
<400> 11936
CGTAAAACTG TTGCTGCCGA AATGCCAGCG ATGCAGTGTC TGCAACTGGC 50
AAGAGCAGAG CAACGACGCG GAGCGACGCT GATTGATGGG CAAACGGTAG 100
<210> 11937
<211> 100
<212> DNA
<213> Escherichia coli K-12 MG1655
<220> ygcr b2771
<222> 605-704
<223> ygcr b2771 U00096 complement(2893799..2894584)
<313> 11937: 627-646
<400> 11937
TGGCTGCGGG GAAAGCAGAA ATTATCCGTA AAACTGTTGC TGCCGAAATG 50
CCAGCGATGC AGTGTCTGCA ACTGGCAAGA GCAGAGCAAC GACGCGGAGC 100
<210> 11938
<211> 100
<212> DNA
<213> Escherichia coli K-12 MG1655
<220> ygcs ygcS
<222> 1311-1410
<223> ygcs ygcS U00096 complement(2894556..2895965)
<313> 11938: 1382-1401
<400> 11938
TTTTGTTGTG ACCTGGCTAT GGGCACCAGA AACTAAAGCC CTCCCGCTGG 50
TGGCGGCGGG AAATGTAGGA GGTGCGAATG AACATTCTGT TAGCGTTTAA 100
<210> 11939
<211> 100
<212> DNA
<213> Escherichia coli K-12 MG1655
<220> ygcs ygcS
<222> 1301-1400
<223> ygcs ygcS U00096 complement(2894556..2895965)
<313> 11939: 1326-1345
<400> 11939
TGTTGGTTGG TTTTGTTGTG ACCTGGCTAT GGGCACCAGA AACTAAAGCC 50
CTCCCGCTGG TGGCGGCGGG AAATGTAGGA GGTGCGAATG AACATTCTGT 100
<210> 11940
<211> 100
<212> DNA
<213> Escherichia coli K-12 MG1655
<220> ygcs ygcS
<222> 1232-1331
<223> ygcs ygcS U00096 complement(2894556..2895965)
<313> 11940: 1273-1292
<400> 11940
CTGGCCTGCT GCCGTGGGTG CTGGCGCAGT GGGGAATGCA AGTCACCTTA 50
TTGCTCCTGG CGACAGTGTT GTTGGTTGGT TTTGTTGTGA CCTGGCTATG 100
<210> 11941
<211> 100
<212> DNA
<213> Escherichia coli K-12 MG1655
<220> mfd b1115
<222> 3291-3390

<223> mfd b1115 U00096 complement(1169741..1173187)
<313> 11941: 3338-3357
<400> 11941
GGCCTGGTTG ATTGGTTTGC TGCAAAAACA GCCGCAGCAT TACCGCCTTG 50
ATGGTCCGAC GCGCCTGAAA TTTATTCAGG ATTTGAGTGA GCGGAAAACG 100
<210> 11942
<211> 100
<212> DNA
<213> Escherichia coli K-12 MG1655
<220> mfd b1115
<222> 3181-3280
<223> mfd b1115 U00096 complement(1169741..1173187)
<313> 11942: 3228-3247
<400> 11942
CTGGATATTG CCCGTCTGCG CCAGCAAGCG CAGAAACTGG GGATCAGGAA 50
GCTGGAAGGT AATGAGAAAG GCGGGGTGAT CGAATTTGCC GAGAAGAATC 100
<210> 11943
<211> 100
<212> DNA
<213> Escherichia coli K-12 MG1655
<220> mfd b1115
<222> 3113-3212
<223> mfd b1115 U00096 complement(1169741..1173187)
<313> 11943: 3160-3179
<400> 11943
AACTGGAAGA GATCAAAGTC GAGCTTATCG ATCGCTTCGG CCTGCTGCCA 50
GATCCGGCGC GTACCCTGCT GGATATTGCC CGTCTGCGCC AGCAAGCGCA 100
<210> 11944
<211> 100
<212> DNA
<213> Escherichia coli K-12 MG1655
<220> b2748 b2749
<222> 176-275
<223> b2748 b2749 U00096 complement(2870532..2870843)
<313> 11944: 231-250
<400> 11944
ATCTCAATGG CGGCCAGGAG GCGCTCGAAG AGCGTGCGCG TAATGAACTC 50
AGCATGACCA GGCCGGGCGA AACTTTTTAT CGTCTGGTGC CTGACGCGTC 100
<210> 11945
<211> 100
<212> DNA
<213> Escherichia coli K-12 MG1655
<220> b2748 b2749
<222> 122-221
<223> b2748 b2749 U00096 complement(2870532..2870843)
<313> 11945: 174-193
<400> 11945
CTACAAACGC GAAACTTAAA GCGCGAAACG ATCAACTTTT TGCCGAAATT 50
GACGATCTCA ATGGCGGCCA GGAGGCGCTC GAAGAGCGTG CGCGTAATGA 100
<210> 11946
<211> 100
<212> DNA
<213> Escherichia coli K-12 MG1655
<220> b2748 b2749
<222> 105-204
<223> b2748 b2749 U00096 complement(2870532..2870843)
<313> 11946: 134-153
<400> 11946
TGTGGCGGCA CAGCAAGCTA CAAACGCGAA ACTTAAAGCG CGAAACGATC 50
AACTTTTTGC CGAAATTGAC GATCTCAATG GCGGCCAGGA GGCGCTCGAA 100
<210> 11947
<211> 100
<212> DNA
<213> Escherichia coli K-12 MG1655
<220> ygcu b2774
<222> 558-657
<223> ygcu b2774 U00096 complement(2896784..2897440)
<313> 11947: 626-645
<400> 11947
CGTCACACGC ATTAAAAACG TGCCACGCCG CGCGGCTGGC CCGGACATTC 50
GTCACATCAT CATCGCAACG AAGGTGCATT GTGCTATATC ACTGAAGTAA 100

<210> 11948
<211> 100
<212> DNA
<213> Escherichia coli K-12 MG1655
<220> ygcu b2774
<222> 495-594
<223> ygcu b2774 U00096 complement(2896784..2897440)
<313> 11948: 519-538
<400> 11948
CACACTCTAC GGCGCAATCG AAGATATGGT CGTTGGTCTG GAAGCAGTAT 50
TGGCTGATGG CACCGTCACA CGCATTAAAA ACGTGCCACG CCGCGCGGCT 100
<210> 11949
<211> 100
<212> DNA
<213> Escherichia coli K-12 MG1655
<220> ygcu b2774
<222> 423-522
<223> ygcu b2774 U00096 complement(2896784..2897440)
<313> 11949: 465-484
<400> 11949
GCATTCTCCG CAGTCAAAGC CGCTGGCGCA GATGGGCGGC CTGGTAGCAA 50
CCCGAAGTAT CGGGCAGTTC TCCACACTCT ACGGCGCAAT CGAAGATATG 100
<210> 11950
<211> 100
<212> DNA
<213> Escherichia coli K-12 MG1655
<220> ygcw b2775
<222> 762-861
<223> ygcw b2775 U00096 complement(2897510..2898370)
<313> 11950: 820-839
<400> 11950
TCAGGATTTA ATGGGCGCAG CCGTATTCCT CGCAAGTCCG GCATCGAATT 50
ATGTCAACGG GCATTTATTA GTGGTTGATG GCGGTTATTT AGTGCGCTAA 100
<210> 11951
<211> 100
<212> DNA
<213> Escherichia coli K-12 MG1655
<220> ygcw b2775
<222> 733-832
<223> ygcw b2775 U00096 complement(2897510..2898370)
<313> 11951: 786-805
<400> 11951
CATATTCCGG CAAACCGTTG GGCGATACTC CAGGATTTAA TGGGCGCAGC 50
CGTATTCCTC GCAAGTCCGG CATCGAATTA TGTCAACGGG CATTTATTAG 100
<210> 11952
<211> 100
<212> DNA
<213> Escherichia coli K-12 MG1655
<220> ygcw b2775
<222> 695-794
<223> ygcw b2775 U00096 complement(2897510..2898370)
<313> 11952: 746-765
<400> 11952
CACGCAGTAA TCCAGAAACC AATCAGCGCG TTCTTGATCA TATTCCGGCA 50
AACCGTTGGG GCGATACTCA GGATTTAATG GGCGCAGCCG TATTCCTCGC 100
<210> 11953
<211> 100
<212> DNA
<213> Escherichia coli K-12 MG1655
<220> ygcx b2788
<222> 1242-1341
<223> ygcx b2788 U00096 complement(2916067..2917407)
<313> 11953: 1266-1285
<400> 11953
TCAGAAACAC GGGCTTGGCG CGCGTGACGA TGCGATGGGA ATGCAGTATC 50
TGATTCCTGG CTGGACGTTC GATAACAAGC GCCCGTGCAT GGTGCGTTAA 100
<210> 11954
<211> 100
<212> DNA
<213> Escherichia coli K-12 MG1655
<220> ygcx b2788

<222> 1171-1270
<223> ygcx b2788 U00096 complement(2916067..2917407)
<313> 11954: 1193-1212
<400> 11954
CAGGTGCCAG AAAAACCGGG GCTGGGTGTA GAAATCGATA TGGATCAAGT 50
GATGAAAGCC CATGAGCTGT ATCAGAAACA CGGGCTTGGC GCGCGTGACG 100
<210> 11955
<211> 100
<212> DNA
<213> Escherichia coli K-12 MG1655
<220> ygcx b2788
<222> 1096-1195
<223> ygcx b2788 U00096 complement(2916067..2917407)
<313> 11955: 1147-1166
<400> 11955
GATACGCACT GGATTTGGCA GGAAGGCAAT CAGCGCCTGA CCAAAGAACC 50
GTTTGAGATC AAAGGCGGGC TGGTACAGGT GCCAGAAAAA CCGGGGCTGG 100
<210> 11956
<211> 100
<212> DNA
<213> Escherichia coli K-12 MG1655
<220> ygcy ygcY
<222> 1230-1329
<223> ygcy ygcY U00096 complement(2917428..2918768)
<313> 11956: 1276-1295
<400> 11956
TGAGGCCTAT AAACGTCTGC CTGGCGGTGC GCGTAACGAC GCAGGTCCGA 50
TGCAGTACCT GATCCCCGGC TGGACCTTTG ACCGTAAACG TCCCGTTTTC 100
<210> 11957
<211> 100
<212> DNA
<213> Escherichia coli K-12 MG1655
<220> ygcy ygcY
<222> 1170-1269
<223> ygcy ygcY U00096 complement(2917428..2918768)
<313> 11957: 1212-1231
<400> 11957
CGTTCCTGAT GCGCCCGGTC TGGGCGTGGA ACTGGACTGG GAACAGGTAC 50
AAAAGGCACA TGAGGCCTAT AAACGTCTGC CTGGCGGTGC GCGTAACGAC 100
<210> 11958
<211> 100
<212> DNA
<213> Escherichia coli K-12 MG1655
<220> ygcy ygcY
<222> 1112-1211
<223> ygcy ygcY U00096 complement(2917428..2918768)
<313> 11958: 1166-1185
<400> 11958
AGGAGGGCGA TTGTCGCCTG ACCCAAAATC CGCTGGAGAT TAAAAACGGA 50
AAAATTGCCG TTCCTGATGC GCCCGGTCTG GGCGTGGAAC TGGACTGGGA 100
<210> 11959
<211> 100
<212> DNA
<213> Escherichia coli K-12 MG1655
<220> fixx b0045
<222> 90-189
<223> fixx b0045 U00096 45463..45750
<313> 11959: 118-137
<400> 11959
TGCTGATAAA CAGGCGCTGG AGCTGCTGGT GAAAGCGTGC CCCGCAGGTC 50
TGTACAAGAA GCAGGATGAC GGCAGTGTGC GCTTCGATTA CGCCGGATGT 100
<210> 11960
<211> 100
<212> DNA
<213> Escherichia coli K-12 MG1655
<220> fixx b0045
<222> 48-147
<223> fixx b0045 U00096 45463..45750
<313> 11960: 92-111
<400> 11960
CAATGTCGAT GAAGAGCATC CGCACATTGT TGTGAAGGCC GATGCTGATA 50

AACAGGCGCT GGAGCTGCTG GTGAAAGCGT GCCCCGCAGG TCTGTACAAG 100
<210> 11961
<211> 100
<212> DNA
<213> Escherichia coli K-12 MG1655
<220> fixx b0045
<222> 64-163
<223> fixx b0045 U00096 45463..45750
<313> 11961: 84-103
<400> 11961
CATCCGCACA TTGTTGTGAA GGCCGATGCT GATAAACAGG CGCTGGAGCT 50
GCTGGTGAAA GCGTGCCCCG CAGGTCTGTA CAAGAAGCAG GATGACGGCA 100
<210> 11962
<211> 100
<212> DNA
<213> Escherichia coli K-12 MG1655
<220> ygdb b2825
<222> 267-366
<223> ygdb b2825 U00096 complement(2960771..2961136)
<313> 11962: 328-347
<400> 11962
GCGAACAGGC GAAGTCATTG ATGGAAACAT TGTTTTTTCG CCACGCGGCT 50
GGAGCGATTT TTGTCCGCTG AAAGAGAGGG CGTTATGTCA GCTTCCCTGA 100
<210> 11963
<211> 100
<212> DNA
<213> Escherichia coli K-12 MG1655
<220> ygdb b2825
<222> 240-339
<223> ygdb b2825 U00096 complement(2960771..2961136)
<313> 11963: 289-308
<400> 11963
TGCCGGTTAT GAAGGCGTTT CGTTGTGGCG AACAGGCGAA GTCATTGATG 50
GAAACATTGT TTTTTCGCCA CGCGGCTGGA GCGATTTTTG TCCGCTGAAA 100
<210> 11964
<211> 100
<212> DNA
<213> Escherichia coli K-12 MG1655
<220> ygdb b2825
<222> 190-289
<223> ygdb b2825 U00096 complement(2960771..2961136)
<313> 11964: 233-252
<400> 11964
GATGCCCAGG TTTGTTTGCG TTTACTGGCA GATAATGAAG CCTTATTGAT 50
TGCCGGTTAT GAAGGCGTTT CGTTGTGGCG AACAGGCGAA GTCATTGATG 100
<210> 11965
<211> 100
<212> DNA
<213> Escherichia coli K-12 MG1655
<220> ygdd b2808
<222> 297-396
<223> ygdd b2808 U00096 complement(2939258..2939653)
<313> 11965: 374-393
<400> 11965
GTTTGTCACT CCGGTTGGCG GCGTGAGCTT CCTCGCGGGC TGGGCGTTAA 50
TGTTAGTTGG TGCTATCCGT TTAAAGCGCA AGGGCGTAAG TCATGAATAA 100
<210> 11966
<211> 100
<212> DNA
<213> Escherichia coli K-12 MG1655
<220> ygdd b2808
<222> 297-396
<223> ygdd b2808 U00096 complement(2939258..2939653)
<313> 11966: 350-369
<400> 11966
GTTTGTCACT CCGGTTGGCG GCGTGAGCTT CCTCGCGGGC TGGGCGTTAA 50
TGTTAGTTGG TGCTATCCGT TTAAAGCGCA AGGGCGTAAG TCATGAATAA 100
<210> 11967
<211> 100
<212> DNA
<213> Escherichia coli K-12 MG1655

<220> ygdd b2808
<222> 157-256
<223> ygdd b2808 U00096 complement(2939258..2939653)
<313> 11967: 218-237
<400> 11967
GCGATCTTAG GTCTGGCGGT GGCAATGCAG CGTCGCATCA GTATCTGGTT 50
TTACTGGAGT AGCGTTTTCC TCGCGTTAGG CACGGTGTTG TTCAGCGGCA 100
<210> 11968
<211> 100
<212> DNA
<213> Escherichia coli K-12 MG1655
<220> ygde b2807
<222> 974-1073
<223> ygde b2807 U00096 complement(2938165..2939265)
<313> 11968: 1003-1022
<400> 11968
CACAGCTTGA TGAACATGGC ATAAATGCTC AGATTCAGGC GCGGCAGTTG 50
TATCACGATC GCGAAGAAGT GACGGTGCAC GTCCGCCGCA TCTGGGCTGC 100
<210> 11969
<211> 100
<212> DNA
<213> Escherichia coli K-12 MG1655
<220> ygde b2807
<222> 912-1011
<223> ygde b2807 U00096 complement(2938165..2939265)
<313> 11969: 968-987
<400> 11969
CCTCAAACTG CCGATGAAAA AACGCTACGA AGAAGTGTCA CACAATCTGG 50
CGTATATTCA GGCACAGCTT GATGAACATG GCATAAATGC TCAGATTCAG 100
<210> 11970
<211> 100
<212> DNA
<213> Escherichia coli K-12 MG1655
<220> ygde b2807
<222> 876-975
<223> ygde b2807 U00096 complement(2938165..2939265)
<313> 11970: 925-944
<400> 11970
GCTGGTTAAT GGCTGGTGCC GTGAAACCAT TTTCAACCTC AAACTGCCGA 50
TGAAAAAACG CTACGAAGAA GTGTCACACA ATCTGGCGTA TATTCAGGCA 100
<210> 11971
<211> 100
<212> DNA
<213> Escherichia coli K-12 MG1655
<220> pria b3936
<222> 2100-2199
<223> pria b3936 U00096 complement(4122192..4124390)
<313> 11971: 2166-2185
<400> 11971
CTTGCAACAC ATCATTAACG GTACGCTGGC GCTCATCAAT ACAATACCGG 50
ATTCCCGTAA GGTGAAATGG GTGCTGGATG TTGATCCGAT TGAGGGTTAA 100
<210> 11972
<211> 100
<212> DNA
<213> Escherichia coli K-12 MG1655
<220> pria b3936
<222> 1998-2097
<223> pria b3936 U00096 complement(4122192..4124390)
<313> 11972: 2024-2043
<400> 11972
AGACGAGAAA CTGTGGGTTC TCGGTCCGGT TCCGGCTCTG GCACCTAAAC 50
GTGGCGGTCG CTGGCGCTGG CAGATATTGT TGCAGCACCC TTCCCGCGTG 100
<210> 11973
<211> 100
<212> DNA
<213> Escherichia coli K-12 MG1655
<220> pria b3936
<222> 1930-2029
<223> pria b3936 U00096 complement(4122192..4124390)
<313> 11973: 1985-2004
<400> 11973

CATAACAATC AGCACGCGCC ATTGTTCCTG CAACAACTGC GTAATCTGAT 50
CCTCTCCAGC CCACTGGCAG ACGAGAAACT GTGGGTTCTC GGTCCGGTTC 100
<210> 11974
<211> 100
<212> DNA
<213> Escherichia coli K-12 MG1655
<220> ygdh b2796
<222> 1266-1365
<223> ygdh b2796 U00096 2924330..2925694
<313> 11974: 1335-1354
<400> 11974
TATGCGTCGT ATGGACGACC TGCTACAGGG TTTTGTTGCC CAGCATCGTA 50
TGAAGTTGCC AGGCTCAGCC TACATCCCTT GCTACGAAAT CTGCACGTAA 100
<210> 11975
<211> 100
<212> DNA
<213> Escherichia coli K-12 MG1655
<220> ygdh b2796
<222> 1225-1324
<223> ygdh b2796 U00096 2924330..2925694
<313> 11975: 1277-1296
<400> 11975
GAAGAGTTTG GTCCTTACAA AATCAACGGC GATAAAGAGA TTATGCGTCG 50
TATGGACGAC CTGCTACAGG GTTTTGTTGC CCAGCATCGT ATGAAGTTGC 100
<210> 11976
<211> 100
<212> DNA
<213> Escherichia coli K-12 MG1655
<220> ygdh b2796
<222> 1196-1295
<223> ygdh b2796 U00096 2924330..2925694
<313> 11976: 1237-1256
<400> 11976
ACGTAAAAGA AGTCGGTATT CGCGCCATTG AAGAGTTTGG TCCTTACAAA 50
ATCAACGGCG ATAAAGAGAT TATGCGTCGT ATGGACGACC TGCTACAGGG 100
<210> 11977
<211> 100
<212> DNA
<213> Escherichia coli K-12 MG1655
<220> prib b4202
<222> 185-284
<223> prib b4202 U00096 4423098..4423412
<313> 11977: 251-270
<400> 11977
TTACTCACAG TATAACGGTC GGCAGTCGCA TAACCGTTCA GGGGTTCATT 50
TCATGCCACA AGGCAAAGAA CGGACTGAGC AAAATGGTTT TGCATGCCGA 100
<210> 11978
<211> 100
<212> DNA
<213> Escherichia coli K-12 MG1655
<220> prib b4202
<222> 138-237
<223> prib b4202 U00096 4423098..4423412
<313> 11978: 190-209
<400> 11978
GTGGTGTCAA ATGCCCGTTA TTGTTAGCGG ACACGAAAAC CAGGCCATTA 50
CTCACAGTAT AACGGTCGGC AGTCGCATAA CCGTTCAGGG GTTCATTTCA 100
<210> 11979
<211> 100
<212> DNA
<213> Escherichia coli K-12 MG1655
<220> prib b4202
<222> 82-181
<223> prib b4202 U00096 4423098..4423412
<313> 11979: 140-159
<400> 11979
CAGTTCGTGC TTGAGCATCG TTCTGTGCAG GAGGAAGCCG GCTTTCACCG 50
GCAGGCGTGG TGTCAAATGC CCGTTATTGT TAGCGGACAC GAAAACCAGG 100
<210> 11980
<211> 100
<212> DNA

<213> Escherichia coli K-12 MG1655
<220> pric b0468
<222> 429-528
<223> pric b0468 U00096 complement(489509..490036)
<313> 11980: 478-497
<400> 11980
AACGCTGCAT CGTGAAGTGG AAGCCTATGA AGCGCGCCTG GCACGCTGCC 50
GCCATGCGCT GGAAAAAATC GAAAACAGGT TAGCGCGTTT AACCCGCTAG 100
<210> 11981
<211> 100
<212> DNA
<213> Escherichia coli K-12 MG1655
<220> pric b0468
<222> 397-496
<223> pric b0468 U00096 complement(489509..490036)
<313> 11981: 438-457
<400> 11981
CTGGCGCGGG TGACCGATCT CGTGGAACAG CAAACGCTGC ATCGTGAAGT 50
GGAAGCCTAT GAAGCGCGCC TGGCACGCTG CCGCCATGCG CTGGAAAAAA 100
<210> 11982
<211> 100
<212> DNA
<213> Escherichia coli K-12 MG1655
<220> pric b0468
<222> 349-448
<223> pric b0468 U00096 complement(489509..490036)
<313> 11982: 383-402
<400> 11982
GATTTTGAGC GGCGGCTACG TGAGATGGTT GCCGAACGCA GAGCCCGTCT 50
GGCGCGGGTG ACCGATCTCG TGGAACAGCA AACGCTGCAT CGTGAAGTGG 100
<210> 11983
<211> 100
<212> DNA
<213> Escherichia coli K-12 MG1655
<220> ygdk b2812
<222> 345-444
<223> ygdk b2812 U00096 2942564..2943007
<313> 11983: 413-432
<400> 11983
GTTTGATGAG CTGGGATTAC GTGCGCAGCT TAGCGCCTCA CGCAGCCAGG 50
GGTTAAATGC GTTAAGCGAG GCGATTATCG CTGCGACGAA GCAGGTTTAA 100
<210> 11984
<211> 100
<212> DNA
<213> Escherichia coli K-12 MG1655
<220> ygdk b2812
<222> 312-411
<223> ygdk b2812 U00096 2942564..2943007
<313> 11984: 327-346
<400> 11984
CGCCGAGTTG CAGGCACAGT CACCACTGGC ATTGTTTGAT GAGCTGGGAT 50
TACGTGCGCA GCTTAGCGCC TCACGCAGCC AGGGGTTAAA TGCGTTAAGC 100
<210> 11985
<211> 100
<212> DNA
<213> Escherichia coli K-12 MG1655
<220> ygdk b2812
<222> 256-355
<223> ygdk b2812 U00096 2942564..2943007
<313> 11985: 292-311
<400> 11985
CGCATTGTGC GCGGCCTGCT GGCGGTGTTG TTGACTGCCG TTGAGGGGAA 50
AACCGCCGCC GAGTTGCAGG CACAGTCACC ACTGGCATTG TTTGATGAGC 100
<210> 11986
<211> 100
<212> DNA
<213> Escherichia coli K-12 MG1655
<220> ygdl b2813
<222> 708-807
<223> ygdl b2813 U00096 complement(2943058..2943864)
<313> 11986: 762-781

<400> 11986
TGGATTTGGC GCGGCAACGA TGGTGACCGC CACCTTTGGG TTTGTTGCGG 50
TTTCTCATGC GCTGAAGAAG ATGATGGCGA AAGCGGCGCG TCAGGGTTAA 100
<210> 11987
<211> 100
<212> DNA
<213> Escherichia coli K-12 MG1655
<220> ygdl b2813
<222> 690-789
<223> ygdl b2813 U00096 complement(2943058..2943864)
<313> 11987: 719-738
<400> 11987
GCGGATGGAT TGTGCATCTG GATTTGGCGC GGCAACGATG GTGACCGCCA 50
CCTTTGGGTT TGTTGCGGTT TCTCATGCGC TGAAGAAGAT GATGGCGAAA 100
<210> 11988
<211> 100
<212> DNA
<213> Escherichia coli K-12 MG1655
<220> ygdl b2813
<222> 637-736
<223> ygdl b2813 U00096 complement(2943058..2943864)
<313> 11988: 687-706
<400> 11988
CCGCAGTCAG ACGGTACGGT GTGTGCGATG AAAGCCACGG CAGAAGGGCC 50
GAAGCGGATG GATTGTGCAT CTGGATTTGG CGCGGCAACG ATGGTGACCG 100
<210> 11989
<211> 100
<212> DNA
<213> Escherichia coli K-12 MG1655
<220> ygdp b2831
<222> 432-531
<223> ygdp b2831 U00096 complement(2966469..2966999)
<313> 11989: 491-510
<400> 11989
CCGTAGGGTA ATGAAAGAGT TCGCGAGTGT GGTGATGTCA CTGCAGGAAA 50
ATACGCCAAA ACCACAAAAC GCATCTGCTT ATCGACGTAA AAGAGGTTAA 100
<210> 11990
<211> 100
<212> DNA
<213> Escherichia coli K-12 MG1655
<220> ygdp b2831
<222> 340-439
<223> ygdp b2831 U00096 complement(2966469..2966999)
<313> 11990: 366-385
<400> 11990
CAAACCAGCA GTACACCAGA GTTTGACGGC TGGCGATGGG TAAGTTACTG 50
GTATCCGGTC AGACAGGTGG TGTCATTTAA ACGTGATGTC TACCGTAGGG 100
<210> 11991
<211> 100
<212> DNA
<213> Escherichia coli K-12 MG1655
<220> ygdp b2831
<222> 283-382
<223> ygdp b2831 U00096 complement(2966469..2966999)
<313> 11991: 311-330
<400> 11991
CAAAAACAAA AATGGTTTCT CTTGCAGCTG GTGAGCGGCG ATGCAGAAAT 50
CAATATGCAA ACCAGCAGTA CACCAGAGTT TGACGGCTGG CGATGGGTAA 100
<210> 11992
<211> 100
<212> DNA
<213> Escherichia coli K-12 MG1655
<220> b2755 b2755
<222> 819-918
<223> b2755 b2755 U00096 complement(2876878..2877795)
<313> 11992: 855-874
<400> 11992
CGCTGGAGAA ATACAACCGC CGGCCCCACC TGAAGATGCA CAGCCTGTTG 50
CCATTCCGCT TCCTGTTTCA CTGGGAGATG CAGGCCATCG GAGTAGCTGA 100
<210> 11993
<211> 100

<212> DNA
<213> Escherichia coli K-12 MG1655
<220> b2755 b2755
<222> 768-867
<223> b2755 b2755 U00096 complement(2876878..2877795)
<313> 11993: 820-839
<400> 11993
CAGTAGTAAA ACATTAGCCA AATTGATTCC GCTTATAGAG GACGTGCTTG 50
CCGCTGGAGA AATACAACCG CCGGCCCCAC CTGAAGATGC ACAGCCTGTT 100
<210> 11994
<211> 100
<212> DNA
<213> Escherichia coli K-12 MG1655
<220> b2755 b2755
<222> 676-775
<223> b2755 b2755 U00096 complement(2876878..2877795)
<313> 11994: 698-717
<400> 11994
GACACTGTTG TACCGAAAGC TTTTGAGATA GCGCGTCGTA ACCCTGGTGA 50
GCCGGACCGG GAAGTCCGTT TGGCGTGCAG GGATATTTTT CGCAGTAGTA 100
<210> 11995
<211> 100
<212> DNA
<213> Escherichia coli K-12 MG1655
<220> b2756 b2756
<222> 501-600
<223> b2756 b2756 U00096 complement(2877811..2878410)
<313> 11995: 568-587
<400> 11995
CACCATCAAC GACGCGCCAG CGTTAATAGA TCTTGTACAG CAAGGTATTG 50
GGCCAGCTAA ATCGATGGGA TGTGGCTTGC TATCTTTGGC TCCACTGTGA 100
<210> 11996
<211> 100
<212> DNA
<213> Escherichia coli K-12 MG1655
<220> b2756 b2756
<222> 461-560
<223> b2756 b2756 U00096 complement(2877811..2878410)
<313> 11996: 504-523
<400> 11996
AAAGTGGAAA GATCCAAACG GTTTGCTTTG AAGGTGTGCT CACCATCAAC 50
GACGCGCCAG CGTTAATAGA TCTTGTACAG CAAGGTATTG GGCCAGCTAA 100
<210> 11997
<211> 100
<212> DNA
<213> Escherichia coli K-12 MG1655
<220> b2756 b2756
<222> 395-494
<223> b2756 b2756 U00096 complement(2877811..2878410)
<313> 11997: 430-449
<400> 11997
ATGCGGCGCG CGTTGAAGAT GTGCATCCCA TATCGGAACG GCCACAGTAT 50
TTTTCTGGTG ATGGTAAAAG TGGAAAGATC CAAACGGTTT GCTTTGAAGG 100
<210> 11998
<211> 100
<212> DNA
<213> Escherichia coli K-12 MG1655
<220> b2757 b2757
<222> 575-674
<223> b2757 b2757 U00096 complement(2878397..2879077)
<313> 11998: 630-649
<400> 11998
ATCATTTAAA ATTTACGGCG CGCGACGAAC CGATGATCAC CTTGCCTCGA 50
CAATTTGCTT CCCGAGAATG GTATGTGATT AAAGGAGGTA TGGATGTATC 100
<210> 11999
<211> 100
<212> DNA
<213> Escherichia coli K-12 MG1655
<220> b2757 b2757
<222> 423-522
<223> b2757 b2757 U00096 complement(2878397..2879077)

<313> 11999: 457-476
<400> 11999
GCCTCGGTAT ACACCTTACC TGGGGCGGAG AAGTTGCCCA CTAACACACC 50
CGCTTTTTTT GGGGACATGT CAGGCATCGG ATCCTCAGAA GGCGCTATTA 100
<210> 12000
<211> 100
<212> DNA
<213> Escherichia coli K-12 MG1655
<220> b2757 b2757
<222> 310-409
<223> b2757 b2757 U00096 complement(2878397..2879077)
<313> 12000: 356-375
<400> 12000
GAAACGATTC AAACATGGCG CGAATATTTA TGTGATGCCT CCTTTACCGT 50
CGCTCTCTGG TTAACACCCC ATGCAACGAT GGTTATCTCA GAACTTGAAA 100
<210> 12001
<211> 100
<212> DNA
<213> Escherichia coli K-12 MG1655
<220> b2758 b2759
<222> 993-1092
<223> b2758 b2759 U00096 complement(2879074..2880165)
<313> 12001: 1063-1082
<400> 12001
ATTCAGCTTA TCTGATGTAG ACCCAATTAC TGCTCAAGTT AAACAAATGC 50
CTACTTTAGA ACAGTTAAAA TCCTGGGTTC GTAATAATGG CGAGGCGTGA 100
<210> 12002
<211> 100
<212> DNA
<213> Escherichia coli K-12 MG1655
<220> b2758 b2759
<222> 924-1023
<223> b2758 b2759 U00096 complement(2879074..2880165)
<313> 12002: 953-972
<400> 12002
TATACAGGCG TTTAATCAAT ATTGGGATCG CGTTGCCAAT GGATATGGTC 50
TGAACGGAGC TGCTGCGCAA TTCAGCTTAT CTGATGTAGA CCCAATTACT 100
<210> 12003
<211> 100
<212> DNA
<213> Escherichia coli K-12 MG1655
<220> b2758 b2759
<222> 855-954
<223> b2758 b2759 U00096 complement(2879074..2880165)
<313> 12003: 901-920
<400> 12003
TATGCCACTT TCTATGGCAA ATGCTTTTGA AAAAGCGGTT AAAGCGAAAG 50
ATGGCTTTTT GCAACCGTCT ATACAGGCGT TTAATCAATA TTGGGATCGC 100
<210> 12004
<211> 100
<212> DNA
<213> Escherichia coli K-12 MG1655
<220> mdob b4360
<222> 2154-2253
<223> mdob b4360 U00096 complement(4594719..4596971)
<313> 12004: 2223-2242
<400> 12004
GGAACCTGTC TCAACCAACG AAGGGAATAT CCTCGGACAC TCGCCGCGTA 50
AGCTCGGGAT CGGCATGGTG GAAATTAAAG TGGTAGAACG TGAAGGGTAA 100
<210> 12005
<211> 100
<212> DNA
<213> Escherichia coli K-12 MG1655
<220> mdob b4360
<222> 2125-2224
<223> mdob b4360 U00096 complement(4594719..4596971)
<313> 12005: 2186-2205
<400> 12005
GATGCCGACA CACTGGTAAT TGTGCCGCCG GAACCTGTCT CAACCAACGA 50
AGGGAATATC CTCGGACACT CGCCGCGTAA GCTCGGGATC GGCATGGTGG 100
<210> 12006

<211> 100
<212> DNA
<213> Escherichia coli K-12 MG1655
<220> mdob b4360
<222> 2094-2193
<223> mdob b4360 U00096 complement(4594719..4596971)
<313> 12006: 2145-2164
<400> 12006
CACCACCACA CTGCATTTCG ATAACCCAAC CGATGCCGAC ACACTGGTAA 50
TTGTGCCGCC GGAACCTGTC TCAACCAACG AAGGGAATAT CCTCGGACAC 100
<210> 12007
<211> 100
<212> DNA
<213> Escherichia coli K-12 MG1655
<220> ygea b2841
<222> 594-693
<223> ygea b2841 U00096 complement(2977965..2978657)
<313> 12007: 669-688
<400> 12007
AGAAATTGGT TTACTGGTGC CAGAAGAGCG CAGTGTTTTG CCTGTGTTTG 50
ATACCGCGGC GATCCATGCC GAGGATGCTG TCGCTTTTAT GCTGTCGTAG 100
<210> 12008
<211> 100
<212> DNA
<213> Escherichia coli K-12 MG1655
<220> ygea b2841
<222> 522-621
<223> ygea b2841 U00096 complement(2977965..2978657)
<313> 12008: 573-592
<400> 12008
ACGCGCTTAT TGTGCGCAAG TGATTGCTCG CCTTGCAGAA CAGGGCGCAC 50
AGGGCGTCAT TTTTGGCTGC ACAGAAATTG GTTTACTGGT GCCAGAAGAG 100
<210> 12009
<211> 100
<212> DNA
<213> Escherichia coli K-12 MG1655
<220> ygea b2841
<222> 470-569
<223> ygea b2841 U00096 complement(2977965..2978657)
<313> 12009: 494-513
<400> 12009
TTAATCAGAT TATTTTTGAA GAACTGTGTC TGGGGCAATT TACCGAAGCG 50
TCACGCGCTT ATTGTGCGCA AGTGATTGCT CGCCTTGCAG AACAGGGCGC 100
<210> 12010
<211> 100
<212> DNA
<213> Escherichia coli K-12 MG1655
<220> mdog b1049
<222> 1437-1536
<223> mdog b1049 U00096 1108558..1110093
<313> 12010: 1498-1517
<400> 12010
GAAAGATGCC AAGAAAACCA CTGAAATGCG TGCTGCGCTG GTGAATGCCG 50
ATCAGACGTT GAGTGAAACC TGGAGCTACC AGTTACCTGC CAATGAATAA 100
<210> 12011
<211> 100
<212> DNA
<213> Escherichia coli K-12 MG1655
<220> mdog b1049
<222> 1410-1509
<223> mdog b1049 U00096 1108558..1110093
<313> 12011: 1444-1463
<400> 12011
CTGGCGTCTG GTGATGCGTG TGAAAGTGAA AGATGCCAAG AAAACCACTG 50
AAATGCGTGC TGCGCTGGTG AATGCCGATC AGACGTTGAG TGAAACCTGG 100
<210> 12012
<211> 100
<212> DNA
<213> Escherichia coli K-12 MG1655
<220> mdog b1049
<222> 1383-1482

<223> mdog b1049 U00096 1108558..1110093
<313> 12012: 1407-1426
<400> 12012
GGTGCGCTAT AACCCGGTTA CCAAAGGCTG GCGTCTGGTG ATGCGTGTGA 50
AAGTGAAAGA TGCCAAGAAA ACCACTGAAA TGCGTGCTGC GCTGGTGAAT 100
<210> 12013
<211> 100
<212> DNA
<213> Escherichia coli K-12 MG1655
<220> narg b1225
<222> 3645-3744
<223> narg b1225 U00096 1279087..1282830
<313> 12013: 3710-3729
<400> 12013
AGGTTCTAAC CGCGATGAGT TTGTTGTAGT GCGTAAGATG AAGAACATTG 50
ACTGGTTAGA TGGCGAAGGC AATGACCAGG TACAGGAGAG CGTAAAATGA 100
<210> 12014
<211> 100
<212> DNA
<213> Escherichia coli K-12 MG1655
<220> narg b1225
<222> 3592-3691
<223> narg b1225 U00096 1279087..1282830
<313> 12014: 3626-3645
<400> 12014
ATGATCGGCG GCTATGCCCA TCTGGCATAC GGCTTTAACT ACTATGGCAC 50
CGTAGGTTCT AACCGCGATG AGTTTGTTGT AGTGCGTAAG ATGAAGAACA 100
<210> 12015
<211> 100
<212> DNA
<213> Escherichia coli K-12 MG1655
<220> narg b1225
<222> 3532-3631
<223> narg b1225 U00096 1279087..1282830
<313> 12015: 3580-3599
<400> 12015
ACCCAACAGC GTGGTGGTAT CCATAACTCG GTCACCCGTA TCACGCCGAA 50
ACCGACGCAT ATGATCGGCG GCTATGCCCA TCTGGCATAC GGCTTTAACT 100
<210> 12016
<211> 100
<212> DNA
<213> Escherichia coli K-12 MG1655
<220> mdoh b1050
<222> 2445-2544
<223> mdoh b1050 U00096 1110086..1112629
<313> 12016: 2497-2516
<400> 12016
CTGGAATTCC CCGGAGAGAT ATTCTTCATG GGTGAGTTAT TACGAAGGGA 50
TAAAGCTCAA TCCACTGGCA TTGCGTAAAC CGGATGCGGC TTCGCAATAA 100
<210> 12017
<211> 100
<212> DNA
<213> Escherichia coli K-12 MG1655
<220> mdoh b1050
<222> 2368-2467
<223> mdoh b1050 U00096 1110086..1112629
<313> 12017: 2420-2439
<400> 12017
CCAGAGAAGC TGAATCGCGA TCGTCGCCTG GTGCTGCTAA GCGATCCGGT 50
GACGATGGCC CGTCTGCATT TCCGTGTCTG GAATTCCCCG GAGAGATATT 100
<210> 12018
<211> 100
<212> DNA
<213> Escherichia coli K-12 MG1655
<220> mdoh b1050
<222> 2298-2397
<223> mdoh b1050 U00096 1110086..1112629
<313> 12018: 2347-2366
<400> 12018
GCGTCACCGC GCCAGTAAGG TGCTGGAAAT CGCCCGTGAC CGCCACGTTG 50
AACAGGCGCT GAACGAGACG CCAGAGAAGC TGAATCGCGA TCGTCGCCTG 100

<210> 12019
<211> 100
<212> DNA
<213> Escherichia coli K-12 MG1655
<220> narh b1226
<222> 1432-1531
<223> narh b1226 U00096 1282827..1284365
<313> 12019: 1461-1480
<400> 12019
TTTACCTTTG GTGATGGCTG CCACGGTTCA GATACCAAAT TCAATCTGTT 50
CAACAGCCGT CGTATCGATG CCATCGATGT GACCAGCAAA ACGGAGCCGC 100
<210> 12020
<211> 100
<212> DNA
<213> Escherichia coli K-12 MG1655
<220> narh b1226
<222> 1264-1363
<223> narh b1226 U00096 1282827..1284365
<313> 12020: 1317-1336
<400> 12020
GACGGTAAAG TTGATACCCG TGCGCTGGAA GAGGTCGGTC TGACCGAAGC 50
CCAGGCACAG GAGATGTACC GTTATCTGGC GATTGCTAAC TACGAAGATC 100
<210> 12021
<211> 100
<212> DNA
<213> Escherichia coli K-12 MG1655
<220> narh b1226
<222> 1107-1206
<223> narh b1226 U00096 1282827..1284365
<313> 12021: 1159-1178
<400> 12021
TGAGCTGGGT AGCAACGGCA TTCTGCCAGA CGTCGAAAGC CTGCGTATTC 50
CGGTACAGTA TCTGGCGAAT CTGCTGACCG CCGGTGATAC CAAACCGGTA 100
<210> 12022
<211> 100
<212> DNA
<213> Escherichia coli K-12 MG1655
<220> yged b2836
<222> 1091-1190
<223> yged b2836 U00096 complement(2970691..2971884)
<313> 12022: 1115-1134
<400> 12022
TGGCGGTAAT GATAGGCATC CCGGTCGTGC CCATTGGCAT TGGCTTCGGT 50
GCGCTGTTTG CGCTGGCAAT AACGGCGCTG TGGATCTGGC AGCGCCGTCA 100
<210> 12023
<211> 100
<212> DNA
<213> Escherichia coli K-12 MG1655
<220> yged b2836
<222> 1035-1134
<223> yged b2836 U00096 complement(2970691..2971884)
<313> 12023: 1064-1083
<400> 12023
AGTACAAAAC CTTGGCGAAA ACAGCGCCAT GTTGTTGATG CTGGGCATTT 50
ACTCGCTGGC GGTAATGATA GGCATCCCGG TCGTGCCCAT TGGCATTGGC 100
<210> 12024
<211> 100
<212> DNA
<213> Escherichia coli K-12 MG1655
<220> yged b2836
<222> 974-1073
<223> yged b2836 U00096 complement(2970691..2971884)
<313> 12024: 1009-1028
<400> 12024
CGCTCAATGC GTTGCTACAG GAGCGGGGTA AAAAAAGCGT CGGGGCGGGG 50
AATGCGATTG CAGTACAAAA CCTTGGCGAA AACAGCGCCA TGTTGTTGAT 100
<210> 12025
<211> 100
<212> DNA
<213> Escherichia coli K-12 MG1655
<220> nari narI

<222> 544-643
<223> nari narI U00096 1285072..1285749
<313> 12025: 595-614
<400> 12025
TTTATCTTCC GTCTGCACCT GGTGCTGGGG ATGACGTTAT TCCTGCTGTT 50
CCCGTTCTCG CGTCTGATAC ACATCTGGAG CGTACCGGTG GAGTATCTGA 100
<210> 12026
<211> 100
<212> DNA
<213> Escherichia coli K-12 MG1655
<220> nari narI
<222> 473-572
<223> nari narI U00096 1285072..1285749
<313> 12026: 530-549
<400> 12026
AACTGGTTGG CTGGGCGCAG TCGGTGGTGA CCTTCCACGG TGGCGCTTCT 50
CAACACCTCG ATGGTGTGGC GTTTATCTTC CGTCTGCACC TGGTGCTGGG 100
<210> 12027
<211> 100
<212> DNA
<213> Escherichia coli K-12 MG1655
<220> nari narI
<222> 392-491
<223> nari narI U00096 1285072..1285749
<313> 12027: 455-474
<400> 12027
CGCTGCTCGT TATCCAGTGC GCGCTGGGCC TGTTGACCAT TCCGTTCTCC 50
GCTCAGCATA TGGACGGTAG CGAGATGATG AAACTGGTTG GCTGGGCGCA 100
<210> 12028
<211> 100
<212> DNA
<213> Escherichia coli K-12 MG1655
<220> narj b1227
<222> 612-711
<223> narj b1227 U00096 1284362..1285072
<313> 12028: 689-708
<400> 12028
CAAAGGCTGC GGTGATTCAG CAATCACTGC GCATCAGCGT CGCTTTGCCG 50
GTGCCGTCGC GCCGCAATAT CTGAATATCA CCACCGGAGG ACAGCACTAA 100
<210> 12029
<211> 100
<212> DNA
<213> Escherichia coli K-12 MG1655
<220> narj b1227
<222> 534-633
<223> narj b1227 U00096 1284362..1285072
<313> 12029: 600-619
<400> 12029
TGCCGACGAA GCGCGCGATG ATACGCCGCA GGCGCTGGAT GCTGTCTGGG 50
AAGAAGAGCA GGTTAAATTC TTTGCTGACA AAGGCTGCGG TGATTCAGCA 100
<210> 12030
<211> 100
<212> DNA
<213> Escherichia coli K-12 MG1655
<220> narj b1227
<222> 459-558
<223> narj b1227 U00096 1284362..1285072
<313> 12030: 517-536
<400> 12030
TTATGCCGTG CTGTTTGATC TGCTGCTGAA ACTGGCGAAT ACCGCTATCG 50
ACAGCGACAA AGTGGCGGAA AAAATTGCCG ACGAAGCGCG CGATGATACG 100
<210> 12031
<211> 100
<212> DNA
<213> Escherichia coli K-12 MG1655
<220> ygef b2851
<222> 348-447
<223> ygef b2851 U00096 2988576..2989022
<313> 12031: 423-442
<400> 12031
ATGCACAGAC GTTCTATCCC CCCGCGTCAC CAGACATAAT AAAGAAAAAA 50

ACAAACGACA TTCTACCCTT CTCAAAAATC CCCTTTTTAA TTTTCATTGA 100
<210> 12032
<211> 100
<212> DNA
<213> Escherichia coli K-12 MG1655
<220> ygef b2851
<222> 348-447
<223> ygef b2851 U00096 2988576..2989022
<313> 12032: 403-422
<400> 12032
ATGCACAGAC GTTCTATCCC CCCGCGTCAC CAGACATAAT AAAGAAAAAA 50
ACAAACGACA TTCTACCCTT CTCAAAAATC CCCTTTTTAA TTTTCATTGA 100
<210> 12033
<211> 100
<212> DNA
<213> Escherichia coli K-12 MG1655
<220> ygef b2851
<222> 327-426
<223> ygef b2851 U00096 2988576..2989022
<313> 12033: 372-391
<400> 12033
CCTGAATAAA TTCACTACTA TATGCACAGA CGTTCTATCC CCCCGCGTCA 50
CCAGACATAA TAAAGAAAAA AACAAACGAC ATTCTACCCT TCTCAAAAAT 100
<210> 12034
<211> 100
<212> DNA
<213> Escherichia coli K-12 MG1655
<220> nark b1224
<222> 1209-1308
<223> nark b1224 U00096 1277180..1278571
<313> 12034: 1244-1263
<400> 12034
GGCGGCGGCG CTGGGTTTCA TCTCTGCGAT TGGCGCGATT GGTGGCTTCT 50
TTATCCCGAA AGCGTTTGGT AGCTCGCTGG CATTAACGGG TTCGCCAGTC 100
<210> 12035
<211> 100
<212> DNA
<213> Escherichia coli K-12 MG1655
<220> nark b1224
<222> 1148-1247
<223> nark b1224 U00096 1277180..1278571
<313> 12035: 1184-1203
<400> 12035
ATCGCGTGAA AGCAGAAGGG GGTTCTGACG AACGTGCGAT GCGTGAAGCG 50
GCAACCGACA CGGCGGCGGC GCTGGGTTTC ATCTCTGCGA TTGGCGCGAT 100
<210> 12036
<211> 100
<212> DNA
<213> Escherichia coli K-12 MG1655
<220> nark b1224
<222> 1040-1139
<223> nark b1224 U00096 1277180..1278571
<313> 12036: 1103-1122
<400> 12036
GCTTCATGGC GTTCTTCGCG GTCTTCCTGG CGCTGTTCCT GACAGCTGGG 50
CTGGGTAGTG GTTCCACTTT CCAGATGATT TCAGTGATCT TCCGTAAACT 100
<210> 12037
<211> 100
<212> DNA
<213> Escherichia coli K-12 MG1655
<220> ygeg b2852
<222> 393-492
<223> ygeg b2852 U00096 2989290..2989781
<313> 12037: 462-481
<400> 12037
TTTCTATCAC ATTATTAACA ATTGTGAGGA TGATAGTGTT AAGAGTAAAG 50
CGCAGGCATA TATTGAACTC TTAAACGATA ATTCAGAAGA TAATGGCTAA 100
<210> 12038
<211> 100
<212> DNA
<213> Escherichia coli K-12 MG1655

<220> ygeg b2852
<222> 393-492
<223> ygeg b2852 U00096 2989290..2989781
<313> 12038: 428-447
<400> 12038
TTTCTATCAC ATTATTAACA ATTGTGAGGA TGATAGTGTT AAGAGTAAAG  50
CGCAGGCATA TATTGAACTC TTAAACGATA ATTCAGAAGA TAATGGCTAA  100
<210> 12039
<211> 100
<212> DNA
<213> Escherichia coli K-12 MG1655
<220> ygeg b2852
<222> 388-487
<223> ygeg b2852 U00096 2989290..2989781
<313> 12039: 428-447
<400> 12039
CAATGTTTCT ATCACATTAT TAACAATTGT GAGGATGATA GTGTTAAGAG  50
TAAAGCGCAG GCATATATTG AACTCTTAAA CGATAATTCA GAAGATAATG  100
<210> 12040
<211> 100
<212> DNA
<213> Escherichia coli K-12 MG1655
<220> narl b1222
<222> 552-651
<223> narl b1222 U00096 complement(1274402..1275052)
<313> 12040: 584-603
<400> 12040
AAGCACAGTA AAAGTGCACG TCAAGCACAT GCTGAAGAAA ATGAAGCTCA  50
AGTCTCGCGT GGAAGCAGCG GTATGGGTGC ATCAGGAGCG CATTTTCTGA  100
<210> 12041
<211> 100
<212> DNA
<213> Escherichia coli K-12 MG1655
<220> narl b1222
<222> 503-602
<223> narl b1222 U00096 complement(1274402..1275052)
<313> 12041: 530-549
<400> 12041
CCCAGGGTTT GCCGAACAAG ATGATTGCCC GCCGCCTGGA TATCACCGAA  50
AGCACAGTAA AAGTGCACGT CAAGCACATG CTGAAGAAAA TGAAGCTCAA  100
<210> 12042
<211> 100
<212> DNA
<213> Escherichia coli K-12 MG1655
<220> narl b1222
<222> 474-573
<223> narl b1222 U00096 complement(1274402..1275052)
<313> 12042: 490-509
<400> 12042
ACGCGAGCGC GATATTCTCA AGCTGATTGC CCAGGGTTTG CCGAACAAGA  50
TGATTGCCCG CCGCCTGGAT ATCACCGAAA GCACAGTAAA AGTGCACGTC  100
<210> 12043
<211> 100
<212> DNA
<213> Escherichia coli K-12 MG1655
<220> ygeh ygeH
<222> 850-949
<223> ygeh ygeH U00096 2990116..2991492
<313> 12043: 906-925
<400> 12043
TTACAAAATA AAAGTGATAT ACAGACGCTA AAAACAGAAT GTTATTGCCT  50
TCTAGCGGAA TGCCATATGT CTTTGGCACT TCATGGAAAA AGTGAACTTG  100
<210> 12044
<211> 100
<212> DNA
<213> Escherichia coli K-12 MG1655
<220> ygeh ygeH
<222> 788-887
<223> ygeh ygeH U00096 2990116..2991492
<313> 12044: 808-827
<400> 12044

AGAAAGAACT TTACGACTTC ACCCCTGAAA GCATTTACAG GGCCATGACT 50
ATATTTGATA GATTACAAAA TAAAAGTGAT ATACAGACGC TAAAAACAGA 100
<210> 12045
<211> 100
<212> DNA
<213> Escherichia coli K-12 MG1655
<220> ygeh ygeH
<222> 589-688
<223> ygeh ygeH U00096 2990116..2991492
<313> 12045: 621-640
<400> 12045
TACATTGAGT TGATCGACGC TAAAAACCTT TTCCTCATCG CCAGTAATCA 50
TCTCCCTGTT GATGAACTAC ATAATACATC ACAATTTATT ATAGATAATA 100
<210> 12046
<211> 100
<212> DNA
<213> Escherichia coli K-12 MG1655
<220> rlpa b0634
<222> 990-1089
<223> rlpa b0634 U00096 complement(663325..664413)
<313> 12046: 1055-1074
<400> 12046
CCAGCTTGGC CCATTCGCCA GCAAAGCCGA AGCCAGTACC TTGCAGCAAC 50
GTTTGCAAAC CGAAGCCCAA TTACAGTCAT TTATTACTAC CGCGCAGTAG 100
<210> 12047
<211> 100
<212> DNA
<213> Escherichia coli K-12 MG1655
<220> rlpa b0634
<222> 980-1079
<223> rlpa b0634 U00096 complement(663325..664413)
<313> 12047: 1023-1042
<400> 12047
TCTGGCGGAT CCAGCTTGGC CCATTCGCCA GCAAAGCCGA AGCCAGTACC 50
TTGCAGCAAC GTTTGCAAAC CGAAGCCCAA TTACAGTCAT TTATTACTAC 100
<210> 12048
<211> 100
<212> DNA
<213> Escherichia coli K-12 MG1655
<220> rlpa b0634
<222> 909-1008
<223> rlpa b0634 U00096 complement(663325..664413)
<313> 12048: 946-965
<400> 12048
TGCGCAACAG TACCAACAGC AACTGGGACA GAAGTTCGGC GTCCCCGGTC 50
GCGTAACTCA AAATGGCGCG GTCTGGCGGA TCCAGCTTGG CCCATTCGCC 100
<210> 12049
<211> 100
<212> DNA
<213> Escherichia coli K-12 MG1655
<220> rlpb b0642
<222> 324-423
<223> rlpb b0642 U00096 complement(670828..671409)
<313> 12049: 378-397
<400> 12049
GTTGATCCCC GGCCGTGATA TCTACCCGAT TAGCGCCAAA GTCTTCCGTT 50
CGTTCTTCGA TAACCCGCAA ATGGCGTTAG CGAAAGATAA CGAACAAGAC 100
<210> 12050
<211> 100
<212> DNA
<213> Escherichia coli K-12 MG1655
<220> rlpb b0642
<222> 216-315
<223> rlpb b0642 U00096 complement(670828..671409)
<313> 12050: 279-298
<400> 12050
TCCATCCTTG CGTTTGGGTA AAGTGAGCAT CGCGAAAGAT ACCGCATCGG 50
TATTCCGTAA CGGTCAAACA GCAGAGTATC AGATGATCAT GACGGTTAAT 100
<210> 12051
<211> 100
<212> DNA

<213> Escherichia coli K-12 MG1655
<220> rlpb b0642
<222> 67-166
<223> rlpb b0642 U00096 complement(670828..671409)
<313> 12051: 119-138
<400> 12051
CTGCGTGATA CCACGCAGGT TCCTTCCACT ATGAAGGTCA TGATCCTGGA 50
CTCAGGCGAT CCGAACGGGC CATTAAGCCG TGCGGTGCGT AACCAGTTAC 100
<210> 12052
<211> 100
<212> DNA
<213> Escherichia coli K-12 MG1655
<220> ygek ygeK
<222> 348-447
<223> ygek ygeK U00096 complement(2992482..2992928)
<313> 12052: 412-431
<400> 12052
AGTCGAAACT CATAGACTCA ATATCATGAA GAAATTAGAT GTTCACAGTG 50
GTATCGAGTT GATCAAAACA GCTTTACGTA TGGGTGTTTG CACTATATAA 100
<210> 12053
<211> 100
<212> DNA
<213> Escherichia coli K-12 MG1655
<220> ygek ygeK
<222> 348-447
<223> ygek ygeK U00096 complement(2992482..2992928)
<313> 12053: 382-401
<400> 12053
AGTCGAAACT CATAGACTCA ATATCATGAA GAAATTAGAT GTTCACAGTG 50
GTATCGAGTT GATCAAAACA GCTTTACGTA TGGGTGTTTG CACTATATAA 100
<210> 12054
<211> 100
<212> DNA
<213> Escherichia coli K-12 MG1655
<220> ygek ygeK
<222> 327-426
<223> ygek ygeK U00096 complement(2992482..2992928)
<313> 12054: 353-372
<400> 12054
CTTGCAGTTA AGCAGGAAAA CAGTCGAAAC TCATAGACTC AATATCATGA 50
AGAAATTAGA TGTTCACAGT GGTATCGAGT TGATCAAAAC AGCTTTACGT 100
<210> 12055
<211> 100
<212> DNA
<213> Escherichia coli K-12 MG1655
<220> narp b2194
<222> 549-648
<223> narp b2194 U00096 2288520..2289167
<313> 12055: 615-634
<400> 12055
GACAGTAAAA GTACATATTC GCAATCTGCT GCGTAAACTC AATGTCCGCT 50
CACGCGTGGC GGCCACCATT CTGTTCCTGC AACAACGCGG GGCACAATAA 100
<210> 12056
<211> 100
<212> DNA
<213> Escherichia coli K-12 MG1655
<220> narp b2194
<222> 517-616
<223> narp b2194 U00096 2288520..2289167
<313> 12056: 574-593
<400> 12056
CAGATTGCCT CGGTGTTGAA TATTTCCGAG CAGACAGTAA AAGTACATAT 50
TCGCAATCTG CTGCGTAAAC TCAATGTCCG CTCACGCGTG GCGGCCACCA 100
<210> 12057
<211> 100
<212> DNA
<213> Escherichia coli K-12 MG1655
<220> narp b2194
<222> 446-545
<223> narp b2194 U00096 2288520..2289167
<313> 12057: 511-530

<400> 12057
ATCCCTTCAG CGTGCTGACG GAGCGCGAGC TGGATGTTCT GCACGAGCTG 50
GCACAGGGGC TGTCAAATAA ACAGATTGCC TCGGTGTTGA ATATTTCCGA 100
<210> 12058
<211> 100
<212> DNA
<213> Escherichia coli K-12 MG1655
<220> b2760 b2761
<222> 1410-1509
<223> b2760 b2761 U00096 complement(2880653..2882161)
<313> 12058: 1488-1507
<400> 12058
ACATCATCCT AAATTAATAA GCACATTAGC GCTTGCCCGC GCCACGCTAT 50
ACAAACATTT ACGGGAGTTA AAACCGCAAG GAGGGCCATC AAATGGCTGA 100
<210> 12059
<211> 100
<212> DNA
<213> Escherichia coli K-12 MG1655
<220> b2760 b2761
<222> 1259-1358
<223> b2760 b2761 U00096 complement(2880653..2882161)
<313> 12059: 1317-1336
<400> 12059
AAAGGCATTT CTATCGACAG AGTGAATTAT TAATTCCCGA TGTACTGGCG 50
AATGTTAATT TTTCCCAGGC TGATGAGGTA ATAGCTGATT TACGAGACAA 100
<210> 12060
<211> 100
<212> DNA
<213> Escherichia coli K-12 MG1655
<220> b2760 b2761
<222> 1150-1249
<223> b2760 b2761 U00096 complement(2880653..2882161)
<313> 12060: 1212-1231
<400> 12060
GTTGGTTTGG GATATAAAAC AGCCTTACGC AAGGCGTTAT ATACCTTTGC 50
AGAAGGGTTT AAAAATAAAG ACTTCAAAGG GGCCGGAGTC TCTGTTCATG 100
<210> 12061
<211> 100
<212> DNA
<213> Escherichia coli K-12 MG1655
<220> narq b2470
<222> 1602-1701
<223> narq b2470 U00096 2583751..2585451
<313> 12061: 1642-1661
<400> 12061
GCTAGGTGGG ACGCTGACTT TTTCACAACC TTCCGGCGGC GGCACGTTAG 50
TGAGTATTAG CTTTCGCTCT GCGGAGGGTG AGGAAAGTCA GTTAATGTAA 100
<210> 12062
<211> 100
<212> DNA
<213> Escherichia coli K-12 MG1655
<220> narq b2470
<222> 1549-1648
<223> narq b2470 U00096 2583751..2585451
<313> 12062: 1598-1617
<400> 12062
AAAGAACCCG AAGGTCATTA TGGTCTGAAT ATCATGCGCG AACGCGCGGA 50
ACGGCTAGGT GGGACGCTGA CTTTTTCACA ACCTTCCGGC GGCGGCACGT 100
<210> 12063
<211> 100
<212> DNA
<213> Escherichia coli K-12 MG1655
<220> narq b2470
<222> 1505-1604
<223> narq b2470 U00096 2583751..2585451
<313> 12063: 1556-1575
<400> 12063
ACACGGTTTA TATCCGTGAT AACGGGATTG GTATCGGTGA ACCGAAAGAA 50
CCCGAAGGTC ATTATGGTCT GAATATCATG CGCGAACGCG CGGAACGGCT 100
<210> 12064
<211> 100

<212> DNA
<213> Escherichia coli K-12 MG1655
<220> naru narU
<222> 1282-1381
<223> naru narU U00096 complement(1540696..1542084)
<313> 12064: 1328-1347
<400> 12064
ATGACCGGCT CTCCGGTCGG CGCGATGAAA GTGTTTTTAA TCTTCTACAT 50
CGTTTGTGTG CTGCTGACCT GGCTGGTTTA TGGTCGGCGG AAGTTCAGCC 100
<210> 12065
<211> 100
<212> DNA
<213> Escherichia coli K-12 MG1655
<220> naru narU
<222> 1193-1292
<223> naru narU U00096 complement(1540696..1542084)
<313> 12065: 1238-1257
<400> 12065
CCGAAACGGC GGCGGCTCTG GGCTTTATCT CAGCCATTGG CGCAGTGGGC 50
GGCTTTTTTA TTCCGCAGGC GTTTGGCATG TCGCTCAATA TGACCGGCTC 100
<210> 12066
<211> 100
<212> DNA
<213> Escherichia coli K-12 MG1655
<220> naru narU
<222> 1094-1193
<223> naru narU U00096 complement(1540696..1542084)
<313> 12066: 1149-1168
<400> 12066
CTACTTTCCA GATGATCGCC GTCATCTTTC GCCAGATAAC CATTTATCGG 50
GTAAAGATGA AAGGCGGTAG TGATGAGCAA GCTCATAAAG AAGCCGTCAC 100
<210> 12067
<211> 100
<212> DNA
<213> Escherichia coli K-12 MG1655
<220> narv b1466
<222> 549-648
<223> narv b1466 U00096 complement(1533961..1534641)
<313> 12067: 586-605
<400> 12067
CGTCTTCCGC CTGCATCTGG TGTTGGGAAT GACCATCTTC CTGCTCTTCC 50
CGTTCACCCG ACTGGTGCAT GTGTGGAGCG CGCCGTTTGA GTACTTTACT 100
<210> 12068
<211> 100
<212> DNA
<213> Escherichia coli K-12 MG1655
<220> narv b1466
<222> 519-618
<223> narv b1466 U00096 complement(1533961..1534641)
<313> 12068: 541-560
<400> 12068
ATCGTCAGAG ATGCTCAACG GCGTAGCGTT CGTCTTCCGC CTGCATCTGG 50
TGTTGGGAAT GACCATCTTC CTGCTCTTCC CGTTCACCCG ACTGGTGCAT 100
<210> 12069
<211> 100
<212> DNA
<213> Escherichia coli K-12 MG1655
<220> narv b1466
<222> 453-552
<223> narv b1466 U00096 complement(1533961..1534641)
<313> 12069: 505-524
<400> 12069
TCCTGATGGT AGCGAAATGA TGAAGCTGGT GGGTTGGGCG CAAAGCATTG 50
TCACTTTCCG TGGTGGATCG TCAGAGATGC TCAACGGCGT AGCGTTCGTC 100
<210> 12070
<211> 100
<212> DNA
<213> Escherichia coli K-12 MG1655
<220> narw b1467
<222> 591-690
<223> narw b1467 U00096 complement(1534638..1535333)

<313> 12070: 614-633
<400> 12070
TAACGCCACG GCGTGTGACA GCTCGCCGCT TAATCAATAT CAGCGACGCT 50
TTAGCCAGGA TGTCGCGCCG CAATATGTCG ACATCAGTGC GGGAGGTGGG 100
<210> 12071
<211> 100
<212> DNA
<213> Escherichia coli K-12 MG1655
<220> narw b1467
<222> 551-650
<223> narw b1467 U00096 complement(1534638..1535333)
<313> 12071: 587-606
<400> 12071
ATGCAGTGTG GGAAGAGGAA CAGGTGAAGT TTATTGAAGA TAACGCCACG 50
GCGTGTGACA GCTCGCCGCT TAATCAATAT CAGCGACGCT TTAGCCAGGA 100
<210> 12072
<211> 100
<212> DNA
<213> Escherichia coli K-12 MG1655
<220> narw b1467
<222> 464-563
<223> narw b1467 U00096 complement(1534638..1535333)
<313> 12072: 519-538
<400> 12072
TGCAACTGGC GGGAAGCTCC CTTTCAAGTG ACAGTGTCAC AAAACAAGTA 50
AACAGCGAAG AGCGCGATGA CACCCGCCAG GCGCTTGATG CAGTGTGGGA 100
<210> 12073
<211> 100
<212> DNA
<213> Escherichia coli K-12 MG1655
<220> narx b1223
<222> 1622-1721
<223> narx b1223 U00096 complement(1275045..1276841)
<313> 12073: 1647-1666
<400> 12073
TGCCTGAAAA TGCCATCCGC AGCAATCACT ACGGCATGAT AATAATGCGC 50
GATCGTGCGC AAAGTTTACG AGGCGATTGC CGCGTCCGCC GTCGTGAATC 100
<210> 12074
<211> 100
<212> DNA
<213> Escherichia coli K-12 MG1655
<220> narx b1223
<222> 1540-1639
<223> narx b1223 U00096 complement(1275045..1276841)
<313> 12074: 1583-1602
<400> 12074
TCGCAAGCGA GTGAAGTCGT GGTGACGGTG GCGCAAAACG ATAATCAGGT 50
CAAACTGACC GTCCAGGATA ACGGCTGCGG CGTGCCTGAA AATGCCATCC 100
<210> 12075
<211> 100
<212> DNA
<213> Escherichia coli K-12 MG1655
<220> narx b1223
<222> 1507-1606
<223> narx b1223 U00096 complement(1275045..1276841)
<313> 12075: 1551-1570
<400> 12075
GCCCGTGAGG CATTAAGTAA CGCCCTCAAA CATTCGCAAG CGAGTGAAGT 50
CGTGGTGACG GTGGCGCAAA ACGATAATCA GGTCAAACTG ACCGTCCAGG 100
<210> 12076
<211> 100
<212> DNA
<213> Escherichia coli K-12 MG1655
<220> yget ygeT
<222> 780-879
<223> yget ygeT U00096 3000636..3001514
<313> 12076: 830-849
<400> 12076
ATGGCGGGCC AGTAAAGAGT TTCGTCTGCA TCTCATCCAG ACGATGACCA 50
AAAAAGTGAT TAGCGAAGCC GTCGCCGCGG CGGGGGGAAA ATTGCAATGA 100
<210> 12077

<211> 100
<212> DNA
<213> Escherichia coli K-12 MG1655
<220> yget ygeT
<222> 732-831
<223> yget ygeT U00096 3000636..3001514
<313> 12077: 778-797
<400> 12077
GGAAGCTATC AGCGAATCTG TCCTGCAAGA TGTCGCCCCG CGTTCTTCAT 50
GGCGGGCCAG TAAAGAGTTT CGTCTGCATC TCATCCAGAC GATGACCAAA 100
<210> 12078
<211> 100
<212> DNA
<213> Escherichia coli K-12 MG1655
<220> yget ygeT
<222> 714-813
<223> yget ygeT U00096 3000636..3001514
<313> 12078: 745-764
<400> 12078
ATTAAACCTG CAAACGCTGG AAGCTATCAG CGAATCTGTC CTGCAAGATG 50
TCGCCCCGCG TTCTTCATGG CGGGCCAGTA AAGAGTTTCG TCTGCATCTC 100
<210> 12079
<211> 100
<212> DNA
<213> Escherichia coli K-12 MG1655
<220> nary b1468
<222> 1446-1545
<223> nary b1468 U00096 complement(1535333..1536877)
<313> 12079: 1507-1526
<400> 12079
CGGTTGCCAC GGTTCGGACA GTAAATTCAA CCTGTTCAAC AGTAGCCGTA 50
TCGATGCCAT CAACATCACC GAAGTGCGCG ACAAAGCGGA GGGCGAATAA 100
<210> 12080
<211> 100
<212> DNA
<213> Escherichia coli K-12 MG1655
<220> nary b1468
<222> 1338-1437
<223> nary b1468 U00096 complement(1535333..1536877)
<313> 12080: 1398-1417
<400> 12080
TCTCGCCATT GCCAACTATG AAGATCGTTT TGTTATCCCG ACCAGCCATC 50
GGGAAATGGC GGGCGATGCC TTCGCAGAAC GCAACGGCTG CGGTTTTACC 100
<210> 12081
<211> 100
<212> DNA
<213> Escherichia coli K-12 MG1655
<220> nary b1468
<222> 1328-1427
<223> nary b1468 U00096 complement(1535333..1536877)
<313> 12081: 1355-1374
<400> 12081
TGTATCGCTA TCTCGCCATT GCCAACTATG AAGATCGTTT TGTTATCCCG 50
ACCAGCCATC GGGAAATGGC GGGCGATGCC TTCGCAGAAC GCAACGGCTG 100
<210> 12082
<211> 100
<212> DNA
<213> Escherichia coli K-12 MG1655
<220> narz b1469
<222> 3593-3692
<223> narz b1469 U00096 complement(1536874..1540614)
<313> 12082: 3635-3654
<400> 12082
TTGGCGGTTA CGCGCAGCTG GCCTGGGGCT TTAACTACTA CGGCACCGTC 50
GGATCGAACC GCGATGAGTT CATCATGATC CGCAAGATGA AGAACGTTAA 100
<210> 12083
<211> 100
<212> DNA
<213> Escherichia coli K-12 MG1655
<220> narz b1469
<222> 3545-3644

<223> narz b1469 U00096 complement(1536874..1540614)
<313> 12083: 3591-3610
<400> 12083
GCATTCATAA CTCGGTTACC CGCGTTTGCC CGAAACCAAC GCATATGATT 50
GGCGGTTACG CGCAGCTGGC CTGGGGCTTT AACTACTACG GCACCGTCGG 100
<210> 12084
<211> 100
<212> DNA
<213> Escherichia coli K-12 MG1655
<220> narz b1469
<222> 3323-3422
<223> narz b1469 U00096 complement(1536874..1540614)
<313> 12084: 3355-3374
<400> 12084
TGACGCTCTC TCGCGGTGGA CCGATTGTCT GGATCAGCGA AACAGATGCC 50
CGAGAACTGA CCATTGTCGA TAACGACTGG GTGGAAGTAT TCAACGCCAA 100
<210> 12085
<211> 100
<212> DNA
<213> Escherichia coli K-12 MG1655
<220> ygev b2870
<222> 1680-1779
<223> ygev b2870 U00096 complement(3002030..3003808)
<313> 12085: 1726-1745
<400> 12085
TTCACGTCAT AACAGCAAGA AGCAAGTTGC TGATGAACTG GGCATCGGCA 50
TTGCTACGCT CTATCGCAAG ATTAAGAAAT ATGAGTTGTT AAACACATAA 100
<210> 12086
<211> 100
<212> DNA
<213> Escherichia coli K-12 MG1655
<220> ygev b2870
<222> 1587-1686
<223> ygev b2870 U00096 complement(3002030..3003808)
<313> 12086: 1606-1625
<400> 12086
TGTAACAGAG GTCAGTGAGG CGCACCTGTC ACTCGATGAT GCGGGCGGCA 50
CGGCGCTGGA GGAGATGGAA AAGCAAATGA TCCGCGAGGC GCTTTCACGT 100
<210> 12087
<211> 100
<212> DNA
<213> Escherichia coli K-12 MG1655
<220> ygev b2870
<222> 1510-1609
<223> ygev b2870 U00096 complement(3002030..3003808)
<313> 12087: 1577-1596
<400> 12087
GTTCCTTCAG GTGAAGTTAT CGACAGCACG CTATTGCCGC CAAATCTGCT 50
GAATAATGGC ACAACGGAGC AAAGTGATGT AACAGAGGTC AGTGAGGCGC 100
<210> 12088
<211> 100
<212> DNA
<213> Escherichia coli K-12 MG1655
<220> ygew b2871
<222> 993-1092
<223> ygew b2871 U00096 3004356..3005447
<313> 12088: 1036-1055
<400> 12088
CGCTACCTAC AAAGAAGCCA GCTGGAAGCC TTATATCATC GCCGCGATGA 50
TCTGTCCCGT AAATACGCCA AACCAGGTGC ACTGCTCGAG CAACTGCTGA 100
<210> 12089
<211> 100
<212> DNA
<213> Escherichia coli K-12 MG1655
<220> ygew b2871
<222> 900-999
<223> ygew b2871 U00096 3004356..3005447
<313> 12089: 946-965
<400> 12089
AGCCCTGTAC ATGCACTGCC TGCCAGCTGA TATCAGCGGC GTATCCTGTA 50
AAGAAGGTGA AGTGACTGAA GGCGTATTCG AAAAATACCG TATCGCTACC 100

<210> 12090
<211> 100
<212> DNA
<213> Escherichia coli K-12 MG1655
<220> ygew b2871
<222> 870-969
<223> ygew b2871 U00096 3004356..3005447
<313> 12090: 901-920
<400> 12090
AGAGATGATG GAACTGACCC GTGATGGCGA AGCCCTGTAC ATGCACTGCC 50
TGCCAGCTGA TATCAGCGGC GTATCCTGTA AAGAAGGTGA AGTGACTGAA 100
<210> 12091
<211> 100
<212> DNA
<213> Escherichia coli K-12 MG1655
<220> ygex b2872
<222> 1098-1197
<223> ygex b2872 U00096 3005531..3006727
<313> 12091: 1155-1174
<400> 12091
CAAAGATGCC GTGGTGCTGG TTATCAGCAC TGAAGGCGAC ACCGACGTGA 50
AGCACTACCG CGAAGTTGTC TGGGAAGGCA AACACGCTGT AGCACCTTAA 100
<210> 12092
<211> 100
<212> DNA
<213> Escherichia coli K-12 MG1655
<220> ygex b2872
<222> 1039-1138
<223> ygex b2872 U00096 3005531..3006727
<313> 12092: 1062-1081
<400> 12092
CTCGCAGCGG TTCATTATCA CCCGCAACGT CAAAGCCTGA TGGAAAAACT 50
GGCGCTGAAC AAAGATGCCG TGGTGCTGGT TATCAGCACT GAAGGCGACA 100
<210> 12093
<211> 100
<212> DNA
<213> Escherichia coli K-12 MG1655
<220> ygex b2872
<222> 967-1066
<223> ygex b2872 U00096 3005531..3006727
<313> 12093: 986-1005
<400> 12093
GTGCTGGGTA ATCCGTACGG CAACGACCCG CGCATCATCT CCGGTGAATC 50
CGGCGCTGTC GGTTTGGGCG TTCTCGCAGC GGTTCATTAT CACCCGCAAC 100
<210> 12094
<211> 100
<212> DNA
<213> Escherichia coli K-12 MG1655
<220> ygey ygeY
<222> 1113-1212
<223> ygey ygeY U00096 3006785..3007996
<313> 12094: 1159-1178
<400> 12094
TGAAGCGCAT GCACCTAACG AAAAAACCTG GAAATCTCAC CTGGTGACCT 50
GTGCCGCGAT GTACGCTGCA ATCCCGTTAA GCTGGCTGGC AACAGAGTAA 100
<210> 12095
<211> 100
<212> DNA
<213> Escherichia coli K-12 MG1655
<220> ygey ygeY
<222> 1012-1111
<223> ygey ygeY U00096 3006785..3007996
<313> 12095: 1075-1094
<400> 12095
AAAGCGCCGG TTGTTGATAA GTGGACCTTC TCAACTAACG GCGTATCTAT 50
CATGGGCCGT CACGGCATTC CGGTGATCGG CTTTGGCCCG GGTAAAGAAC 100
<210> 12096
<211> 100
<212> DNA
<213> Escherichia coli K-12 MG1655
<220> ygey ygeY

<222> 943-1042
<223> ygey ygeY U00096 3006785..3007996
<313> 12096: 1007-1026
<400> 12096
ACCTGGAAAG TGGAAGAAGA TCACTTCACC GTTAAAGCAC TGGTGAATGC 50
CTACGAAGGG CTGTTTGGCA AAGCGCCGGT TGTTGATAAG TGGACCTTCT 100
<210> 12097
<211> 100
<212> DNA
<213> Escherichia coli K-12 MG1655
<220> ygfa b2913
<222> 377-476
<223> ygfa b2913 U00096 3054261..3054809
<313> 12097: 411-430
<400> 12097
GCCTGGGAAT GGGCGGTGGT TTTTATGATC GGACCTTACA AAACTGGCAG 50
CACTATAAAA CGCAACCGGT GGGTTATGCG CATGATTGTC AGTTGGTGGA 100
<210> 12098
<211> 100
<212> DNA
<213> Escherichia coli K-12 MG1655
<220> ygfa b2913
<222> 354-453
<223> ygfa b2913 U00096 3054261..3054809
<313> 12098: 378-397
<400> 12098
CGCCTTTGAT GAGTACGGTC AGCGCCTGGG AATGGGCGGT GGTTTTTATG 50
ATCGGACCTT ACAAAACTGG CAGCACTATA AAACGCAACC GGTGGGTTAT 100
<210> 12099
<211> 100
<212> DNA
<213> Escherichia coli K-12 MG1655
<220> ygfa b2913
<222> 285-384
<223> ygfa b2913 U00096 3054261..3054809
<313> 12099: 329-348
<400> 12099
TGAGCCAAAA TTGGATGTGC GTGACGTGCT ACCCCTTTCC CGATTAGACG 50
TGCTGATCAC ACCGCTGGTC GCCTTTGATG AGTACGGTCA GCGCCTGGGA 100
<210> 12100
<211> 100
<212> DNA
<213> Escherichia coli K-12 MG1655
<220> ygfb b2910
<222> 486-585
<223> ygfb b2910 U00096 complement(3052886..3053470)
<313> 12100: 559-578
<400> 12100
GATCATCGAA TACGTTCGTG TTGCCGCGCT GTTATGCCAC GACACCTTTA 50
CTCATCCGCA ACCGACCGCG CCAGAAGTAC AAAAACCGAC TCTACACTAA 100
<210> 12101
<211> 100
<212> DNA
<213> Escherichia coli K-12 MG1655
<220> ygfb b2910
<222> 468-567
<223> ygfb b2910 U00096 complement(3052886..3053470)
<313> 12101: 523-542
<400> 12101
TGAAATGTCG CTTGAAGAGA TCATCGAATA CGTTCGTGTT GCCGCGCTGT 50
TATGCCACGA CACCTTTACT CATCCGCAAC CGACCGCGCC AGAAGTACAA 100
<210> 12102
<211> 100
<212> DNA
<213> Escherichia coli K-12 MG1655
<220> ygfb b2910
<222> 410-509
<223> ygfb b2910 U00096 complement(3052886..3053470)
<313> 12102: 451-470
<400> 12102
ACGATCTGCG TAACATTGCG CAACTGGGTT ACGACGAAGA CGAAGATCAG 50

GAAGAGCTTG AAATGTCGCT TGAAGAGATC ATCGAATACG TTCGTGTTGC 100
<210> 12103
<211> 100
<212> DNA
<213> Escherichia coli K-12 MG1655
<220> ygfd b2919
<222> 894-993
<223> ygfd b2919 U00096 3061007..3062002
<313> 12103: 947-966
<400> 12103
CTATTACCGC CAGACGCTTT TAGCGGTCAA AAACAATACG CTCTCACCGC 50
GCACCGGCCT GCGGCAGCTC AGTGAATTTA TCCAGACGCA ATATTTTGAT 100
<210> 12104
<211> 100
<212> DNA
<213> Escherichia coli K-12 MG1655
<220> ygfd b2919
<222> 863-962
<223> ygfd b2919 U00096 3061007..3062002
<313> 12104: 910-929
<400> 12104
ATCACCTGTT CGCGAATGAA GATTTCGATC GCTATTACCG CCAGACGCTT 50
TTAGCGGTCA AAAACAATAC GCTCTCACCG CGCACCGGCC TGCGGCAGCT 100
<210> 12105
<211> 100
<212> DNA
<213> Escherichia coli K-12 MG1655
<220> ygfd b2919
<222> 793-892
<223> ygfd b2919 U00096 3061007..3062002
<313> 12105: 848-867
<400> 12105
CGTTTACAAC AAGTGCGGCA ACAACAATCG GTGGAATGGC TGCGTAAGCA 50
GACCGAAGAA GAAGTACTGA ATCACCTGTT CGCGAATGAA GATTTCGATC 100
<210> 12106
<211> 100
<212> DNA
<213> Escherichia coli K-12 MG1655
<220> ygfe b2911
<222> 231-330
<223> ygfe b2911 U00096 3053632..3053961
<313> 12106: 285-304
<400> 12106
AAGTATGGAA CAGCGTATTC GGATGCTGCA GCAGACCATA GAACAAGCGT 50
TACTTGAACA AGGTCGCATC ACCGAAAAAA CTAACCAAAA CTTTGAATGA 100
<210> 12107
<211> 100
<212> DNA
<213> Escherichia coli K-12 MG1655
<220> ygfe b2911
<222> 184-283
<223> ygfe b2911 U00096 3053632..3053961
<313> 12107: 229-248
<400> 12107
AGCTATGAGT TAGCGCAAGA AAAAGCAAAG ACTCGTGACT ACGCGGCAAG 50
TATGGAACAG CGTATTCGGA TGCTGCAGCA GACCATAGAA CAAGCGTTAC 100
<210> 12108
<211> 100
<212> DNA
<213> Escherichia coli K-12 MG1655
<220> ygfe b2911
<222> 121-220
<223> ygfe b2911 U00096 3053632..3053961
<313> 12108: 177-196
<400> 12108
CTGAAAGAAC GCACTAGAGT CACAAATACT GAACAGTTGG TCTTCATTGC 50
CGCATTGAAT ATCAGCTATG AGTTAGCGCA AGAAAAAGCA AAGACTCGTG 100
<210> 12109
<211> 100
<212> DNA
<213> Escherichia coli K-12 MG1655

<220> exo b2799
<222> 747-846
<223> exo b2799 U00096 2928987..2929832
<313> 12109: 797-816
<400> 12109
AACCCATAAA GAGATGGCGT TTCTGTGCCG CGATATTGCC CGCTTACAAA  50
CCGATTTGCA TATCGACGGC AATTTACAGC AATTGCGGTT GGTACGGTAA  100
<210> 12110
<211> 100
<212> DNA
<213> Escherichia coli K-12 MG1655
<220> exo b2799
<222> 671-770
<223> exo b2799 U00096 2928987..2929832
<313> 12110: 736-755
<400> 12110
TGGTCGAGTT TCAGAGTCTG GAAGGGATAT ATGAGAATCT GGATGCGGTT  50
GCCGAAAAGT GGCGCAAAAA ATTAGAAACC CATAAAGAGA TGGCGTTTCT  100
<210> 12111
<211> 100
<212> DNA
<213> Escherichia coli K-12 MG1655
<220> exo b2799
<222> 485-584
<223> exo b2799 U00096 2928987..2929832
<313> 12111: 516-535
<400> 12111
AGTTACTTTC ACCGACATTA CGTATTCGTG ATTACTTCCA GAAACGTTGG  50
CTGGATGCGC CATTTATCGA TAAAGAATTT GGCGTTCAAC CGCAGCAGTT  100
<210> 12112
<211> 100
<212> DNA
<213> Escherichia coli K-12 MG1655
<220> ygff b2903
<222> 645-744
<223> ygff b2903 U00096 complement(3043178..3043921)
<313> 12112: 722-741
<400> 12112
TGGACAGGCA GAAGAGGTCG CGCAGGCCAT TGTCTGGCTA CTAAGTGATA  50
AAGCCTCTTA CGTCACGGGA AGTTTTATCG ATTTGGCGGG CGGGAAATAA  100
<210> 12113
<211> 100
<212> DNA
<213> Escherichia coli K-12 MG1655
<220> ygff b2903
<222> 621-720
<223> ygff b2903 U00096 complement(3043178..3043921)
<313> 12113: 683-702
<400> 12113
GTCGAACATC CCCATGCAGC GTGGTGGACA GGCAGAAGAG GTCGCGCAGG  50
CCATTGTCTG GCTACTAAGT GATAAAGCCT CTTACGTCAC GGGAAGTTTT  100
<210> 12114
<211> 100
<212> DNA
<213> Escherichia coli K-12 MG1655
<220> ygff b2903
<222> 520-619
<223> ygff b2903 U00096 complement(3043178..3043921)
<313> 12114: 586-605
<400> 12114
GTCGCCGCGC AGGGGATCCG CGTTAACTGC GTGCGGCCAG GGTTTATTTA  50
TACCGAAATG CACGCCAGCG GCGGCGAGCC TGGACGCGTC GATCGCGTTA  100
<210> 12115
<211> 100
<212> DNA
<213> Escherichia coli K-12 MG1655
<220> ygfg b2920
<222> 729-828
<223> ygfg b2920 U00096 3061971..3062798
<313> 12115: 768-787
<400> 12115

TATTCAGGGG ATGCGCCGCG CGGTGTATGA CAGCGAAGAT TACCAGGAAG 50
GGATGAACGC TTTCCTCGAA AAACGTAAAC CTAATTTCGT TGGTCATTAA 100
<210> 12116
<211> 100
<212> DNA
<213> Escherichia coli K-12 MG1655
<220> ygfg b2920
<222> 672-771
<223> ygfg b2920 U00096 3061971..3062798
<313> 12116: 720-739
<400> 12116
AGAAGAGCTG CGTGTACTGG GCGAAGCACA CACCATGAAC TCCGATGAAT 50
TTGAACGTAT TCAGGGGATG CGCCGCGCGG TGTATGACAG CGAAGATTAC 100
<210> 12117
<211> 100
<212> DNA
<213> Escherichia coli K-12 MG1655
<220> ygfg b2920
<222> 619-718
<223> ygfg b2920 U00096 3061971..3062798
<313> 12117: 668-687
<400> 12117
CAAATGGCGC ACCACATCTC TGAGAAAGCG CCGTTAGCCA TTGCCGTTAT 50
CAAAGAAGAG CTGCGTGTAC TGGGCGAAGC ACACACCATG AACTCCGATG 100
<210> 12118
<211> 100
<212> DNA
<213> Escherichia coli K-12 MG1655
<220> ygfh b2921
<222> 1380-1479
<223> ygfh b2921 U00096 3062822..3064300
<313> 12118: 1450-1469
<400> 12118
CTATCTGGAA AATGCGCCTG GCGGACATAT TCACCACGAT CTTAGCCACG 50
TCTTCGACTT ACACCGTAAT TTAATTGCAA CCGGCTCGAT GCTGGGTTAA 100
<210> 12119
<211> 100
<212> DNA
<213> Escherichia coli K-12 MG1655
<220> ygfh b2921
<222> 1354-1453
<223> ygfh b2921 U00096 3062822..3064300
<313> 12119: 1402-1421
<400> 12119
CCTATGTATC GGGATTATCT GCATCGCTAT CTGGAAAATG CGCCTGGCGG 50
ACATATTCAC CACGATCTTA GCCACGTCTT CGACTTACAC CGTAATTTAA 100
<210> 12120
<211> 100
<212> DNA
<213> Escherichia coli K-12 MG1655
<220> ygfh b2921
<222> 1290-1389
<223> ygfh b2921 U00096 3062822..3064300
<313> 12120: 1322-1341
<400> 12120
CGCCGATCTG CGCGGTCTTT CCCCGCTTCA ACGCGCCCGC ACTATCATTG 50
ATAATTGTGC ACATCCTATG TATCGGGATT ATCTGCATCG CTATCTGGAA 100
<210> 12121
<211> 100
<212> DNA
<213> Escherichia coli K-12 MG1655
<220> prkb b3356
<222> 699-798
<223> prkb b3356 U00096 3482127..3482996
<313> 12121: 759-778
<400> 12121
TTTTCGTAAT CTGGAAGGGA TCGATTTCCC CTGGCTGCTG GCGATGTTGC 50
AAGGCTCATT CATTTCCCAC ATCAATACGT TAGTGGTACC GGGCGGCAAA 100
<210> 12122
<211> 100
<212> DNA

<213> Escherichia coli K-12 MG1655
<220> prkb b3356
<222> 635-734
<223> prkb b3356 U00096 3482127..3482996
<313> 12122: 659-678
<400> 12122
ACACTTCAAA CCCGTTCGCG GCAAAAGGTA TCCCGTCGCT CGATGAAAGC 50
TTTGTGGTGA TCCATTTTCG TAATCTGGAA GGGATCGATT TCCCCTGGCT 100
<210> 12123
<211> 100
<212> DNA
<213> Escherichia coli K-12 MG1655
<220> prkb b3356
<222> 542-641
<223> prkb b3356 U00096 3482127..3482996
<313> 12123: 582-601
<400> 12123
ACTCAGTAGT GCGTTCAATG GAAGACTATA TCAACTACAT CACACCGCAG 50
TTTTCCCGCA CCCATCTTAA CTTCCAGCGC GTTCCCACCG TCGACACTTC 100
<210> 12124
<211> 100
<212> DNA
<213> Escherichia coli K-12 MG1655
<220> ygfi b2922
<222> 721-820
<223> ygfi b2922 U00096 complement(3064297..3065208)
<313> 12124: 755-774
<400> 12124
GAGCGCGGAC ATTTTCTGAC GGTGATCCCG CATTTTGCCT GGCGACTGGT 50
GAATCCGGCA ACGCTGAAAT ATTTTGATGC ACCGCACAGG CCGATGTATA 100
<210> 12125
<211> 100
<212> DNA
<213> Escherichia coli K-12 MG1655
<220> ygfi b2922
<222> 656-755
<223> ygfi b2922 U00096 complement(3064297..3065208)
<313> 12125: 711-730
<400> 12125
CTTGTGGGAT CAATCCAACC ATTATTTATC GTCCCTACAG TTTTATGGCC 50
AAAATCAGCG CCGTGGAGCG CGGACATTTT CTGACGGTGA TCCCGCATTT 100
<210> 12126
<211> 100
<212> DNA
<213> Escherichia coli K-12 MG1655
<220> ygfi b2922
<222> 627-726
<223> ygfi b2922 U00096 complement(3064297..3065208)
<313> 12126: 648-667
<400> 12126
CGAAGCAATG ATTGATGCGA ATATGCGACC TTGTGGGATC AATCCAACCA 50
TTATTTATCG TCCCTACAGT TTTATGGCCA AAATCAGCGC CGTGGAGCGC 100
<210> 12127
<211> 100
<212> DNA
<213> Escherichia coli K-12 MG1655
<220> ygfj ygfJ
<222> 427-526
<223> ygfj ygfJ U00096 3013181..3013759
<313> 12127: 467-486
<400> 12127
CAGGCAATCC AACGACCCAA TGTCACCAAT ATGCGTCAGG CACTCCTTAT 50
GGGAGATCAT TATTCCGTCG AAATAGAAAA TGCAGAAATA ATTTTAGATA 100
<210> 12128
<211> 100
<212> DNA
<213> Escherichia coli K-12 MG1655
<220> ygfj ygfJ
<222> 397-496
<223> ygfj ygfJ U00096 3013181..3013759
<313> 12128: 436-455

<400> 12128
CCGATTTTAG TGTCAAAACC ATGCCTGATG CAGGCAATCC AACGACCCAA 50
TGTCACCAAT ATGCGTCAGG CACTCCTTAT GGGAGATCAT TATTCCGTCG 100
<210> 12129
<211> 100
<212> DNA
<213> Escherichia coli K-12 MG1655
<220> ygfj ygfJ
<222> 350-449
<223> ygfj ygfJ U00096 3013181..3013759
<313> 12129: 380-399
<400> 12129
GAAATGATGG CGCAATACTG CCACTCCATA ATGGCATCCC CGGCCATCCG 50
ATTTTAGTGT CAAAACCATG CCTGATGCAG GCAATCCAAC GACCCAATGT 100
<210> 12130
<211> 100
<212> DNA
<213> Escherichia coli K-12 MG1655
<220> msra b4220
<222> 540-639
<223> msra b4220 U00096 complement(4439115..4439753)
<313> 12130: 586-605
<400> 12130
TTATTATGCC GAAGATGACC ACCAGCAATA TCTGCATAAA AACCCGTATG 50
GTTACTGTGG AATTGGCGGA ATTGGCGTCT GTCTGCCGCC GGAAGCATAG 100
<210> 12131
<211> 100
<212> DNA
<213> Escherichia coli K-12 MG1655
<220> msra b4220
<222> 485-584
<223> msra b4220 U00096 complement(4439115..4439753)
<313> 12131: 549-568
<400> 12131
TTGCCGCCGA TGACGATCGT CACATCACCA CGGAAATCGC TAACGCCACA 50
CCGTTTTATT ATGCCGAAGA TGACCACCAG CAATATCTGC ATAAAAACCC 100
<210> 12132
<211> 100
<212> DNA
<213> Escherichia coli K-12 MG1655
<220> msra b4220
<222> 393-492
<223> msra b4220 U00096 complement(4439115..4439753)
<313> 12132: 436-455
<400> 12132
CGGCACGCAG TATCGTTCAG CGATTTATCC GCTGACCCCA GAACAGGATG 50
CCGCAGCTCG CGCCAGTCTG GAACGTTTTC AGGCGGCGAT GCTTGCCGCC 100
<210> 12133
<211> 100
<212> DNA
<213> Escherichia coli K-12 MG1655
<220> b2772 b2773
<222> 696-795
<223> b2772 b2773 U00096 complement(2895987..2896781)
<313> 12133: 737-756
<400> 12133
GGAACACGGC AGCGCGTGGG CGTTGCTGGA AGGGCTGAAA AAGCAGTTCG 50
ATCCTAATGG CATTATGAAT ACGGGTACTA TCTATCCGAT TGAAAAATAA 100
<210> 12134
<211> 100
<212> DNA
<213> Escherichia coli K-12 MG1655
<220> b2772 b2773
<222> 639-738
<223> b2772 b2773 U00096 complement(2895987..2896781)
<313> 12134: 672-691
<400> 12134
CGGTTCGATG GTGCACCACC ACGGTATCGG TAAACATCGC GTTCACTGGA 50
GCAAACTGGA ACACGGCAGC GCGTGGGCGT TGCTGGAAGG GCTGAAAAAG 100
<210> 12135
<211> 100

<212> DNA
<213> Escherichia coli K-12 MG1655
<220> b2772 b2773
<222> 586-685
<223> b2772 b2773 U00096 complement(2895987..2896781)
<313> 12135: 628-647
<400> 12135
AAGTACCACA ATCCGCTCAA CAAGATCATC TGTGAAGAAA CCATTCGCCT 50
CGGCGGTTCG ATGGTGCACC ACCACGGTAT CGGTAAACAT CGCGTTCACT 100
<210> 12136
<211> 100
<212> DNA
<213> Escherichia coli K-12 MG1655
<220> ygfo b2883
<222> 1359-1458
<223> ygfo b2883 U00096 3022315..3023772
<313> 12136: 1427-1446
<400> 12136
TGGCGGGTTA ACTGCGATTT TATTAAATAT TATCCTCCCT GGTGGCTACC 50
GACAGGAAAA CGTTCTGCCT GGTATTACCT CAGCGGAAGA GATGGATTAA 100
<210> 12137
<211> 100
<212> DNA
<213> Escherichia coli K-12 MG1655
<220> ygfo b2883
<222> 1235-1334
<223> ygfo b2883 U00096 3022315..3023772
<313> 12137: 1286-1305
<400> 12137
AGCGCCGTGA AACACTTATT GTCGCCACTT CTTTAGGTTT AGGGCTTGGC 50
GTCTCCTACG ATCCCGAAAT TTTTAAAATA TTGCCAGCCT CTATTTATGT 100
<210> 12138
<211> 100
<212> DNA
<213> Escherichia coli K-12 MG1655
<220> ygfo b2883
<222> 1196-1295
<223> ygfo b2883 U00096 3022315..3023772
<313> 12138: 1235-1254
<400> 12138
CCATCGCAGG GATTCGCATC ATCATCACCA ACGGTTTAAA GCGCCGTGAA 50
ACACTTATTG TCGCCACTTC TTTAGGTTTA GGGCTTGGCG TCTCCTACGA 100
<210> 12139
<211> 100
<212> DNA
<213> Escherichia coli K-12 MG1655
<220> ygfp b2884
<222> 1215-1314
<223> ygfp b2884 U00096 3023787..3025106
<313> 12139: 1233-1252
<400> 12139
CTCTGTTTCT TTAGTCGACA AATTGTTCGT GATGATGACG TTGGGCGATG 50
ACCGTTCGAT CTACCGCACC TACGTTGATG GTCGTCTGGT GTACGAACGC 100
<210> 12140
<211> 100
<212> DNA
<213> Escherichia coli K-12 MG1655
<220> ygfp b2884
<222> 1131-1230
<223> ygfp b2884 U00096 3023787..3025106
<313> 12140: 1159-1178
<400> 12140
TGGCAACTTT TTACCTGGCA AAGAGGCTGA TTTCGTGGTG ATGGAACCCA 50
CCGCCACTCC GCTACAGCAG CTGCGCTATG ACAACTCTGT TTCTTTAGTC 100
<210> 12141
<211> 100
<212> DNA
<213> Escherichia coli K-12 MG1655
<220> ygfp b2884
<222> 1057-1156
<223> ygfp b2884 U00096 3023787..3025106

<313> 12141: 1116-1135
<400> 12141
CGCCTCTCGG CATATGAAGC GTTTTACCTG GCCACGCTCG GCGGAGCGAA 50
ATCTCTGGGC CTTGACGATT TGATTGGCAA CTTTTTACCT GGCAAAGAGG 100
<210> 12142
<211> 100
<212> DNA
<213> Escherichia coli K-12 MG1655
<220> ygfq b2885
<222> 471-570
<223> ygfq b2885 U00096 3025142..3025711
<313> 12142: 537-556
<400> 12142
GTTTCTGCTG CTGATTGCTG CTAACGGTGT GGGTATGGTT ATCAAAAACC 50
CGATTGAAGG CTTGCAGTGG CGCTCGGTGC GTTTACCTCC TTCCCGGTGA 100
<210> 12143
<211> 100
<212> DNA
<213> Escherichia coli K-12 MG1655
<220> ygfq b2885
<222> 451-550
<223> ygfq b2885 U00096 3025142..3025711
<313> 12143: 495-514
<400> 12143
ACAGGTATCG GTATCGGGCT GTTTCTGCTG CTGATTGCTG CTAACGGTGT 50
GGGTATGGTT ATCAAAAACC CGATTGAAGG CTTGCAGTGG CGCTCGGTGC 100
<210> 12144
<211> 100
<212> DNA
<213> Escherichia coli K-12 MG1655
<220> ygfq b2885
<222> 324-423
<223> ygfq b2885 U00096 3025142..3025711
<313> 12144: 376-395
<400> 12144
GCAACAAATT AGCGTTCCTG TCGCACTGGG CGCGGTATTT CTGATGGGCG 50
TCATCTTCAC CGCCATTTCC GTAACCGGTG TGCGTACCTG GATCTTACGT 100
<210> 12145
<211> 100
<212> DNA
<213> Escherichia coli K-12 MG1655
<220> ygfr b2886
<222> 732-831
<223> ygfr b2886 U00096 3025678..3026508
<313> 12145: 797-816
<400> 12145
AGGCCGCGTC TTTGCACGCG AATGGCAAAA GCTGAATATT GGTACGGTGA 50
TCATTACTGC CGCACTGGTC GCATTTTACG CGGGTGGTTG GGCAATCTAA 100
<210> 12146
<211> 100
<212> DNA
<213> Escherichia coli K-12 MG1655
<220> ygfr b2886
<222> 694-793
<223> ygfr b2886 U00096 3025678..3026508
<313> 12146: 760-779
<400> 12146
GTTACCGGTA TTATGCTGGG CTTTGTGACA CTGGTCGTAG GCCGCGTCTT 50
TGCACGCGAA TGGCAAAAGC TGAATATTGG TACGGTGATC ATTACTGCCG 100
<210> 12147
<211> 100
<212> DNA
<213> Escherichia coli K-12 MG1655
<220> ygfr b2886
<222> 664-763
<223> ygfr b2886 U00096 3025678..3026508
<313> 12147: 707-726
<400> 12147
GCCGTGTTCA TCGTTCTGAC TTGTAATATC GTTACCGGTA TTATGCTGGG 50
CTTTGTGACA CTGGTCGTAG GCCGCGTCTT TGCACGCGAA TGGCAAAAGC 100
<210> 12148

<211> 100
<212> DNA
<213> Escherichia coli K-12 MG1655
<220> ygfs b2887
<222> 393-492
<223> ygfs b2887 U00096 complement(3026544..3027035)
<313> 12148: 466-485
<400> 12148
TGAATCTTGC CCAACACAGG CGCTGCAGTT GCTTACTGAA AGAGAACTCA 50
GGCGAGTCCG CCAGCAGCGT ATTGTTGCCA GCGGTGAGAA TCCTCTCTGA 100
<210> 12149
<211> 100
<212> DNA
<213> Escherichia coli K-12 MG1655
<220> ygfs b2887
<222> 342-441
<223> ygfs b2887 U00096 complement(3026544..3027035)
<313> 12149: 382-401
<400> 12149
AATTGTGAAA TGCGATCTCT GTGAACAGCG GGAAGAGGGG CCAGCCTGCG 50
TTGAATCTTG CCCAACACAG GCGCTGCAGT TGCTTACTGA AAGAGAACTC 100
<210> 12150
<211> 100
<212> DNA
<213> Escherichia coli K-12 MG1655
<220> ygfs b2887
<222> 301-400
<223> ygfs b2887 U00096 complement(3026544..3027035)
<313> 12150: 352-371
<400> 12150
ATGATCACCA TTCAGTCGTT GCCGGGGGAT ACCCGACAAC AAATTGTGAA 50
ATGCGATCTC TGTGAACAGC GGGAAGAGGG GCCAGCCTGC GTTGAATCTT 100
<210> 12151
<211> 100
<212> DNA
<213> Escherichia coli K-12 MG1655
<220> sbcb b2012
<222> 1329-1428
<223> sbcb b2012 U00096 2080778..2082205
<313> 12151: 1392-1411
<400> 12151
TGCTGATGAA TTGCAGATGC TGGTACAACA ATATGCCGAT GACAAAGAGA 50
AAGTGGCGCT GTTAAAAGCA CTTTGGCAGT ACGCGGAAGA GATTGTCTAA 100
<210> 12152
<211> 100
<212> DNA
<213> Escherichia coli K-12 MG1655
<220> sbcb b2012
<222> 1312-1411
<223> sbcb b2012 U00096 2080778..2082205
<313> 12152: 1344-1363
<400> 12152
GAGTTTTTGC AGGGTTATGC TGATGAATTG CAGATGCTGG TACAACAATA 50
TGCCGATGAC AAAGAGAAAG TGGCGCTGTT AAAAGCACTT TGGCAGTACG 100
<210> 12153
<211> 100
<212> DNA
<213> Escherichia coli K-12 MG1655
<220> sbcb b2012
<222> 1238-1337
<223> sbcb b2012 U00096 2080778..2082205
<313> 12153: 1300-1319
<400> 12153
GCAACTTCCC GGGGACGCTG GATTATGCCG AGCAGCAACG CTGGCTGGAG 50
CACCGTCGCC AGGTCTTCAC GCCAGAGTTT TTGCAGGGTT ATGCTGATGA 100
<210> 12154
<211> 100
<212> DNA
<213> Escherichia coli K-12 MG1655
<220> ygft b2888
<222> 1770-1869

<223> ygft b2888 U00096 complement(3027032..3028966)
<313> 12154: 1826-1845
<400> 12154
GATTCAAACC GGTGACGTCG GGTATTTACC TACCCAGACG CATCTGAAAA 50
AAGTCTTTGC TGGTGGTGAT GCAGTTCATG GCGCGGATCT GGTTGTCACT 100
<210> 12155
<211> 100
<212> DNA
<213> Escherichia coli K-12 MG1655
<220> ygft b2888
<222> 1698-1797
<223> ygft b2888 U00096 complement(3027032..3028966)
<313> 12155: 1740-1759
<400> 12155
GGCCTTTGGT TTCCAGGCGC ATGCCATGCC GTGGTTGCAG GGCAGCGGAA 50
TTAAACTCGA TAAATGGGGC CTGATTCAAA CCGGTGACGT CGGGTATTTA 100
<210> 12156
<211> 100
<212> DNA
<213> Escherichia coli K-12 MG1655
<220> ygft b2888
<222> 1666-1765
<223> ygft b2888 U00096 complement(3027032..3028966)
<313> 12156: 1698-1717
<400> 12156
GAGTTTGAAT TGCCCGCCGA TGTTCTGATT ATGGCCTTTG GTTTCCAGGC 50
GCATGCCATG CCGTGGTTGC AGGGCAGCGG AATTAAACTC GATAAATGGG 100
<210> 12157
<211> 100
<212> DNA
<213> Escherichia coli K-12 MG1655
<220> sbcc b0398
<222> 3046-3145
<223> sbcc b0398 U00096 complement(411831..414977)
<313> 12157: 3078-3097
<400> 12157
CACGTAGAAG CGATGAAAGA GCGTATTCCG GTGCAGATCA AAGTGAAAAA 50
GATCAACGGC CTGGGCTACA GCAAACTGGA AAGTACGTTT GCAGTGAAAT 100
<210> 12158
<211> 100
<212> DNA
<213> Escherichia coli K-12 MG1655
<220> sbcc b0398
<222> 2932-3031
<223> sbcc b0398 U00096 complement(411831..414977)
<313> 12158: 2982-3001
<400> 12158
GACTCGCTGT TCCTTGATGA AGGTTTTGGC ACGCTGGATA GCGAAACGCT 50
GGATACCGCC CTTGATGCGC TGGATGCCCT GAACGCCAGT GGCAAAACCA 100
<210> 12159
<211> 100
<212> DNA
<213> Escherichia coli K-12 MG1655
<220> sbcc b0398
<222> 2922-3021
<223> sbcc b0398 U00096 complement(411831..414977)
<313> 12159: 2952-2971
<400> 12159
AACACGTATT GACTCGCTGT TCCTTGATGA AGGTTTTGGC ACGCTGGATA 50
GCGAAACGCT GGATACCGCC CTTGATGCGC TGGATGCCCT GAACGCCAGT 100
<210> 12160
<211> 100
<212> DNA
<213> Escherichia coli K-12 MG1655
<220> ygfu ygfU
<222> 1379-1478
<223> ygfu ygfU U00096 3029318..3030835
<313> 12160: 1422-1441
<400> 12160
TGCTGAACGT CTTCTTTAAT GGCTATCAGC ATCATGCTGA CCTGGTGAAG 50
GAATCCGTCT CTGATAAAGA TTTAAAAGTC AGGACAGTAC GTATGTGGCT 100

<210> 12161
<211> 100
<212> DNA
<213> Escherichia coli K-12 MG1655
<220> ygfu ygfU
<222> 1236-1335
<223> ygfu ygfU U00096 3029318..3030835
<313> 12161: 1296-1315
<400> 12161
CCGTTACAAC CTCTATATTG TGGCGATCAG TCTCGGCGTT GGCATGACTC 50
CGACGCTCTC TCACGATTTC TTTTCTAAGT TACCGGCCGT ACTGCAACCG 100
<210> 12162
<211> 100
<212> DNA
<213> Escherichia coli K-12 MG1655
<220> ygfu ygfU
<222> 1167-1266
<223> ygfu ygfU U00096 3029318..3030835
<313> 12162: 1225-1244
<400> 12162
TCTAGTGATG TTCGGCATGG TACTGGCGAC AGGGATTCGA ATTCTGTCGC 50
GCTGTAACTA CACCACCAAC CGTTACAACC TCTATATTGT GGCGATCAGT 100
<210> 12163
<211> 100
<212> DNA
<213> Escherichia coli K-12 MG1655
<220> sbcd b0399
<222> 1074-1173
<223> sbcd b0399 U00096 complement(414974..416176)
<313> 12163: 1124-1143
<400> 12163
CGAACTCAGC GTCGAAGAGG TGTTCAATCG CCGTCTGGCA CTGGAAGAAC 50
TGGATGAATC GCAGCAGCAA CGTCTGCAGC ATCTTTTCAC CACGACGTTG 100
<210> 12164
<211> 100
<212> DNA
<213> Escherichia coli K-12 MG1655
<220> sbcd b0399
<222> 1044-1143
<223> sbcd b0399 U00096 complement(414974..416176)
<313> 12164: 1076-1095
<400> 12164
GTTAGCCAGC CAACAGCGTG AAACCCTCAG CGAACTCAGC GTCGAAGAGG 50
TGTTCAATCG CCGTCTGGCA CTGGAAGAAC TGGATGAATC GCAGCAGCAA 100
<210> 12165
<211> 100
<212> DNA
<213> Escherichia coli K-12 MG1655
<220> sbcd b0399
<222> 958-1057
<223> sbcd b0399 U00096 complement(414974..416176)
<313> 12165: 1009-1028
<400> 12165
ATTCAGCGCA AAATCCAGGC ATTAACCGAA TCATTGCCTG TCGAAGTATT 50
GCTGGTACGT CGGAGTCGTG AACAGCGCGA GCGTGTGTTA GCCAGCCAAC 100
<210> 12166
<211> 100
<212> DNA
<213> Escherichia coli K-12 MG1655
<220> ygfy b2898
<222> 168-267
<223> ygfy b2898 U00096 complement(3038824..3039090)
<313> 12166: 245-264
<400> 12166
CTGGCTGATG AATCACGGTA AACCGGCCGA TGCAGAACTG GAAATGATGG 50
TCCGACTCAT CCAGACACGG AACCGGGAAC GTGGTCCTGT GGCAATCTGA 100
<210> 12167
<211> 100
<212> DNA
<213> Escherichia coli K-12 MG1655
<220> ygfy b2898

<222> 168-267
<223> ygfy b2898 U00096 complement(3038824..3039090)
<313> 12167: 234-253
<400> 12167
CTGGCTGATG AATCACGGTA AACCGGCCGA TGCAGAACTG GAAATGATGG 50
TCCGACTCAT CCAGACACGG AACCGGGAAC GTGGTCCTGT GGCAATCTGA 100
<210> 12168
<211> 100
<212> DNA
<213> Escherichia coli K-12 MG1655
<220> ygfy b2898
<222> 168-267
<223> ygfy b2898 U00096 complement(3038824..3039090)
<313> 12168: 214-233
<400> 12168
CTGGCTGATG AATCACGGTA AACCGGCCGA TGCAGAACTG GAAATGATGG 50
TCCGACTCAT CCAGACACGG AACCGGGAAC GTGGTCCTGT GGCAATCTGA 100
<210> 12169
<211> 100
<212> DNA
<213> Escherichia coli K-12 MG1655
<220> ygfz ygfZ
<222> 880-979
<223> ygfz ygfZ U00096 3039333..3040313
<313> 12169: 931-950
<400> 12169
GTCGTCATGA ATAACGATAT GGAACCGGAT AGCATCTTCC GCGTACGCGA 50
CGATGCGAAT ACATTGCATA TCGAGCCGCT GCCGTATTCG CTCGAAGAGT 100
<210> 12170
<211> 100
<212> DNA
<213> Escherichia coli K-12 MG1655
<220> ygfz ygfZ
<222> 851-950
<223> ygfz ygfZ U00096 3039333..3040313
<313> 12170: 887-906
<400> 12170
AACTGGAAGA TGGTCAGGTC GTGGTACAGG TCGTCATGAA TAACGATATG 50
GAACCGGATA GCATCTTCCG CGTACGCGAC GATGCGAATA CATTGCATAT 100
<210> 12171
<211> 100
<212> DNA
<213> Escherichia coli K-12 MG1655
<220> ygfz ygfZ
<222> 815-914
<223> ygfz ygfZ U00096 3039333..3040313
<313> 12171: 844-863
<400> 12171
ACTGGCGTCG TACCGGTACG GTACTGGCTG CGGTAAAACT GGAAGATGGT 50
CAGGTCGTGG TACAGGTCGT CATGAATAAC GATATGGAAC CGGATAGCAT 100
<210> 12172
<211> 100
<212> DNA
<213> Escherichia coli K-12 MG1655
<220> ygga b2924
<222> 537-636
<223> ygga b2924 U00096 complement(3066193..3066828)
<313> 12172: 612-631
<400> 12172
ACAGCGCATT ATCAATCTGG TTGTGGGATG TGTTATGTGG TTTATTGCCT 50
TGCAGCTGGC GAGAGACGGT ATTGCTCATG CACAAGCCTT GTTCAGTTAG 100
<210> 12173
<211> 100
<212> DNA
<213> Escherichia coli K-12 MG1655
<220> ygga b2924
<222> 490-589
<223> ygga b2924 U00096 complement(3066193..3066828)
<313> 12173: 532-551
<400> 12173
GCTCTTCTCG CAGCCTGGCT GGCACCGCGT CTGCGCACGG CAAAAGCACA 50

GCGCATTATC AATCTGGTTG TGGGATGTGT TATGTGGTTT ATTGCCTTGC 100
<210> 12174
<211> 100
<212> DNA
<213> Escherichia coli K-12 MG1655
<220> ygga b2924
<222> 421-520
<223> ygga b2924 U00096 complement(3066193..3066828)
<313> 12174: 477-496
<400> 12174
GATGTGGAAC CAAAACGCTG GTTTGCACTC GGGACAATTA GCGCCTCTTT 50
CCTGTGGTTC TTTGGTCTGG CTCTTCTCGC AGCCTGGCTG GCACCGCGTC 100
<210> 12175
<211> 100
<212> DNA
<213> Escherichia coli K-12 MG1655
<220> yggb b2925
<222> 762-861
<223> yggb b2925 U00096 complement(3066967..3067827)
<313> 12175: 826-845
<400> 12175
GGAGCGTATT AAACGTGAAT TTGATGCCGC CGGTATCAGC TTCCCGTACC 50
CGCAAATGGA TGTGAACTTT AAGCGGGTGA AAGAAGACAA AGCTGCGTAA 100
<210> 12176
<211> 100
<212> DNA
<213> Escherichia coli K-12 MG1655
<220> yggb b2925
<222> 703-802
<223> yggb b2925 U00096 complement(3066967..3067827)
<313> 12176: 730-749
<400> 12176
TTCGTGGTCC GCGTCTGGAG CAACAGCGGC GATCTGCAAA ACGTGTACTG 50
GGATGTGCTG GAGCGTATTA AACGTGAATT TGATGCCGCC GGTATCAGCT 100
<210> 12177
<211> 100
<212> DNA
<213> Escherichia coli K-12 MG1655
<220> yggb b2925
<222> 668-767
<223> yggb b2925 U00096 complement(3066967..3067827)
<313> 12177: 705-724
<400> 12177
TGCGCCTGAA CGAACTTGGT GCATCGTCGA TTAATTTCGT GGTCCGCGTC 50
TGGAGCAACA GCGGCGATCT GCAAAACGTG TACTGGGATG TGCTGGAGCG 100
<210> 12178
<211> 100
<212> DNA
<213> Escherichia coli K-12 MG1655
<220> yggc b2929
<222> 615-714
<223> yggc b2929 U00096 complement(3071996..3072709)
<313> 12178: 689-708
<400> 12178
CACCGATGGC CCCAATGTCG AGCGTGTGCT GATGAATAGC CGTCAGGCCA 50
ATCTGATAGT AGAGATGACT GAAGAAGGCC GGTATCACTT CACAAGCTGA 100
<210> 12179
<211> 100
<212> DNA
<213> Escherichia coli K-12 MG1655
<220> yggc b2929
<222> 583-682
<223> yggc b2929 U00096 complement(3071996..3072709)
<313> 12179: 601-620
<400> 12179
ACGCGGCAAG TAGCCGAAGC ATTTTATGCC CGCACCGATG GCCCCAATGT 50
CGAGCGTGTG CTGATGAATA GCCGTCAGGC CAATCTGATA GTAGAGATGA 100
<210> 12180
<211> 100
<212> DNA
<213> Escherichia coli K-12 MG1655

<220> yggc b2929
<222> 527-626
<223> yggc b2929 U00096 complement(3071996..3072709)
<313> 12180: 572-591
<400> 12180
CACCTGCTCA AATTCTGCGG GAGCGCTTGA TTAGTCGTAA AATTGCGGGA 50
GGATTAACGC GGCAAGTAGC CGAAGCATTT TATGCCCGCA CCGATGGCCC 100
<210> 12181
<211> 100
<212> DNA
<213> Escherichia coli K-12 MG1655
<220> gapc_1 b1418
<222> 293-392
<223> gapc_1 b1418 U00096 complement(1487985..1488389)
<313> 12181: 306-325
<400> 12181
CCATTGGTCT GGTGATCCCG GAACTGAGCG GCAAACTGAA AGGTCATGCG 50
CAACGCGTGC CGGTGAAAAC AGGTTCGGTC ACTGAACTGG TATCGATTCT 100
<210> 12182
<211> 100
<212> DNA
<213> Escherichia coli K-12 MG1655
<220> gapc_1 b1418
<222> 238-337
<223> gapc_1 b1418 U00096 complement(1487985..1488389)
<313> 12182: 270-289
<400> 12182
GCTTCACGCG CAGCGGCAGA AAATATCATT CCCCACACTA CGGGGGCGGC 50
AAAAGCCATT GGTCTGGTGA TCCCGGAACT GAGCGGCAAA CTGAAAGGTC 100
<210> 12183
<211> 100
<212> DNA
<213> Escherichia coli K-12 MG1655
<220> gapc_1 b1418
<222> 213-312
<223> gapc_1 b1418 U00096 complement(1487985..1488389)
<313> 12183: 232-251
<400> 12183
TGGCCCGCGT GGTAAAGATT TACGTGCTTC ACGCGCAGCG GCAGAAAATA 50
TCATTCCCCA CACTACGGGG GCGGCAAAAG CCATTGGTCT GGTGATCCCG 100
<210> 12184
<211> 100
<212> DNA
<213> Escherichia coli K-12 MG1655
<220> yggd b2930
<222> 411-510
<223> yggd b2930 U00096 complement(3072706..3073215)
<313> 12184: 486-505
<400> 12184
CGCTGATTTC TCCGTCTGGT CAGAGGCGCG TTTTAGCGGA ATGGTCAAAA 50
CGGCGCTGAC GCTGGCAGTA ACGACCACCT TAAAGGAATT AACGCCGTGA 100
<210> 12185
<211> 100
<212> DNA
<213> Escherichia coli K-12 MG1655
<220> yggd b2930
<222> 376-475
<223> yggd b2930 U00096 complement(3072706..3073215)
<313> 12185: 393-412
<400> 12185
GCGATGCTCT ATGACGCGTT AAAAGCGATG AAGTTCGCTG ATTTCTCCGT 50
CTGGTCAGAG GCGCGTTTTA GCGGAATGGT CAAAACGGCG CTGACGCTGG 100
<210> 12186
<211> 100
<212> DNA
<213> Escherichia coli K-12 MG1655
<220> yggd b2930
<222> 322-421
<223> yggd b2930 U00096 complement(3072706..3073215)
<313> 12186: 360-379
<400> 12186

GCCGATGACA TGACCTGGGA TTTTATCAGC AACGTCAATA GCATAACCCG 50
CAATGCGATG CTCTATGACG CGTTAAAAGC GATGAAGTTC GCTGATTTCT 100
<210> 12187
<211> 100
<212> DNA
<213> Escherichia coli K-12 MG1655
<220> gapc_2 b1417
<222> 153-252
<223> gapc_2 b1417 U00096 complement(1487737..1487988)
<313> 12187: 221-240
<400> 12187
GGGCGATTTA CAACTGGTGA AAACGGTCGC CTGGTACGAT AACGAATATG 50
GCTTCGTCAC ACAGCTTATT CGCACCCTCG AAAAATTCGC TAAACTCTGA 100
<210> 12188
<211> 100
<212> DNA
<213> Escherichia coli K-12 MG1655
<220> gapc_2 b1417
<222> 127-226
<223> gapc_2 b1417 U00096 complement(1487737..1487988)
<313> 12188: 191-210
<400> 12188
GCCACGCAAA CGGAAATTAC CGCCGTGGGC GATTTACAAC TGGTGAAAAC 50
GGTCGCCTGG TACGATAACG AATATGGCTT CGTCACACAG CTTATTCGCA 100
<210> 12189
<211> 100
<212> DNA
<213> Escherichia coli K-12 MG1655
<220> gapc_2 b1417
<222> 95-194
<223> gapc_2 b1417 U00096 complement(1487737..1487988)
<313> 12189: 139-158
<400> 12189
TCATTGGCAG CCATTTCGGT TCGGTGTTTG ATGCCACGCA AACGGAAATT 50
ACCGCCGTGG GCGATTTACA ACTGGTGAAA ACGGTCGCCT GGTACGATAA 100
<210> 12190
<211> 100
<212> DNA
<213> Escherichia coli K-12 MG1655
<220> ygge b2923
<222> 642-741
<223> ygge b2923 U00096 complement(3065360..3066100)
<313> 12190: 712-731
<400> 12190
TTACGAGCAG GCCGCTATTC AGTTTGATGA TCAGGTCGAT GTGGTCTTCC 50
AGTTAGAACC TGTGGATCAA CAACCCGCTA AAACACCTGC AGCACAATAA 100
<210> 12191
<211> 100
<212> DNA
<213> Escherichia coli K-12 MG1655
<220> ygge b2923
<222> 618-717
<223> ygge b2923 U00096 complement(3065360..3066100)
<313> 12191: 672-691
<400> 12191
CGCGCCGGTG TCCGCCCAGG AAACTTACGA GCAGGCCGCT ATTCAGTTTG 50
ATGATCAGGT CGATGTGGTC TTCCAGTTAG AACCTGTGGA TCAACAACCC 100
<210> 12192
<211> 100
<212> DNA
<213> Escherichia coli K-12 MG1655
<220> ygge b2923
<222> 539-638
<223> ygge b2923 U00096 complement(3065360..3066100)
<313> 12192: 598-617
<400> 12192
TGGGGCCGGT ATATAGCGTG CGCTACCATG TTTCCAACTA TCAGCCCAGC 50
CCAATGGTGC GGATGATGAA AGCCGATGCC GCGCCGGTGT CCGCCCAGGA 100
<210> 12193
<211> 100
<212> DNA

<213> Escherichia coli K-12 MG1655
<220> yggf yggF
<222> 867-966
<223> yggf yggF U00096 complement(3073237..3074202)
<313> 12193: 933-952
<400> 12193
GGTGAACGGT ATCCAACAGA CGGCGAATGG GGTGCGGACG CAGACATTAC 50
TGATCGGCGG CGCGGACCAA ACGTGTAATA TAATAGACTC CCTGCATTGA 100
<210> 12194
<211> 100
<212> DNA
<213> Escherichia coli K-12 MG1655
<220> yggf yggF
<222> 862-961
<223> yggf yggF U00096 complement(3073237..3074202)
<313> 12194: 886-905
<400> 12194
GAGCTGGTGA ACGGTATCCA ACAGACGGCG AATGGGGTGC GGACGCAGAC 50
ATTACTGATC GGCGGCGCGG ACCAAACGTG TAATATAATA GACTCCCTGC 100
<210> 12195
<211> 100
<212> DNA
<213> Escherichia coli K-12 MG1655
<220> yggf yggF
<222> 796-895
<223> yggf yggF U00096 complement(3073237..3074202)
<313> 12195: 847-866
<400> 12195
TACTCGCTCG ATGAACTGGT GAGGGGGAAC GATATCCTCT TTAGCGCTAC 50
GGGCGTGACG GGCGGTGAGC TGGTGAACGG TATCCAACAG ACGGCGAATG 100
<210> 12196
<211> 100
<212> DNA
<213> Escherichia coli K-12 MG1655
<220> yggg b2937
<222> 765-864
<223> yggg b2937 U00096 3079806..3080690
<313> 12196: 825-844
<400> 12196
CTTTGAAAAA CTGGCAAAAC TGGAAGAAGG TCGCCAAAGC TCAATGTTTG 50
ACGACCATCC TGCATCCGCC GAACGCGCCC AGCATATTCG CGATCGCATG 100
<210> 12197
<211> 100
<212> DNA
<213> Escherichia coli K-12 MG1655
<220> yggg b2937
<222> 756-855
<223> yggg b2937 U00096 3079806..3080690
<313> 12197: 778-797
<400> 12197
TGCCACCAGC TTTGAAAAAC TGGCAAAACT GGAAGAAGGT CGCCAAAGCT 50
CAATGTTTGA CGACCATCCT GCATCCGCCG AACGCGCCCA GCATATTCGC 100
<210> 12198
<211> 100
<212> DNA
<213> Escherichia coli K-12 MG1655
<220> yggg b2937
<222> 672-771
<223> yggg b2937 U00096 3079806..3080690
<313> 12198: 724-743
<400> 12198
GCAATTCTCC CAGCGCCAGG AAGCAGAAGC CGATGATTAT TCTTACGATC 50
TTCTGCGCCA ACGCGGCATC AGCCCGGCAG GTCTTGCCAC CAGCTTTGAA 100
<210> 12199
<211> 100
<212> DNA
<213> Escherichia coli K-12 MG1655
<220> iada b4329
<222> 1074-1173
<223> iada b4329 U00096 complement(4555923..4557095)
<313> 12199: 1121-1140

<400> 12199
CATGACGCCA GAACTGCGCA TTGAGCAGGT ATACGCTCGC GGCAAACTGA 50
TGGTCAAAGA CGGCAAAGCC TGCGTGAAAG GAACGTTTGA AACGGCTTAA 100
<210> 12200
<211> 100
<212> DNA
<213> Escherichia coli K-12 MG1655
<220> iada b4329
<222> 1011-1110
<223> iada b4329 U00096 complement(4555923..4557095)
<313> 12200: 1044-1063
<400> 12200
CGGTTTCCTT AACCTGACCG GGAAAGGCGA AATTCTGCCA GGCAATGATG 50
CTGACTTGCT GGTCATGACG CCAGAACTGC GCATTGAGCA GGTATACGCT 100
<210> 12201
<211> 100
<212> DNA
<213> Escherichia coli K-12 MG1655
<220> iada b4329
<222> 936-1035
<223> iada b4329 U00096 complement(4555923..4557095)
<313> 12201: 980-999
<400> 12201
AACCGTGCAG GTGCTGGTCA AAGACTATGA TTTCAGTATC AGCGATGCCC 50
TGCGCCCGCT CACCAGTAGC GTAGCCGGTT TCCTTAACCT GACCGGGAAA 100
<210> 12202
<211> 100
<212> DNA
<213> Escherichia coli K-12 MG1655
<220> prla b3301
<222> 1233-1332
<223> prla b3301 U00096 complement(3440403..3441734)
<313> 12202: 1295-1314
<400> 12202
CGTGATTATG GACTTTATGG CTCAAGTGCA AACTCTGATG ATGTCCAGTC 50
AGTATGAGTC TGCATTGAAG AAGGCGAACC TGAAAGGCTA CGGCCGATAA 100
<210> 12203
<211> 100
<212> DNA
<213> Escherichia coli K-12 MG1655
<220> prla b3301
<222> 1195-1294
<223> prla b3301 U00096 complement(3440403..3441734)
<313> 12203: 1252-1271
<400> 12203
TTCTACTTCG GTGGGACCTC ACTGCTTATC GTTGTTGTCG TGATTATGGA 50
CTTTATGGCT CAAGTGCAAA CTCTGATGAT GTCCAGTCAG TATGAGTCTG 100
<210> 12204
<211> 100
<212> DNA
<213> Escherichia coli K-12 MG1655
<220> prla b3301
<222> 1101-1200
<223> prla b3301 U00096 complement(3440403..3441734)
<313> 12204: 1147-1166
<400> 12204
TAAAGTAATG ACCCGCCTGA CCCTGGTTGG TGCGCTGTAT ATTACCTTTA 50
TCTGCCTGAT CCCGGAGTTC ATGCGTGATG CAATGAAAGT ACCGTTCTAC 100
<210> 12205
<211> 100
<212> DNA
<213> Escherichia coli K-12 MG1655
<220> yggh b2961
<222> 621-720
<223> yggh b2961 U00096 complement(3100151..3100870)
<313> 12205: 695-714
<400> 12205
CGTACCGCGT CCGGCATCAC GTCCGGTGAC GAAATTTGAA CAACGTGGTC 50
ATCGTCTTGG TCACGGAGTA TGGGACTTAA TGTTCGAGAG GGTGAAATAA 100
<210> 12206
<211> 100

<212> DNA
<213> Escherichia coli K-12 MG1655
<220> yggh b2961
<222> 604-703
<223> yggh b2961 U00096 complement(3100151..3100870)
<313> 12206: 635-654
<400> 12206
TCAGAGAGCA ATGATTACGT ACCGCGTCCG GCATCACGTC CGGTGACGAA 50
ATTTGAACAA CGTGGTCATC GTCTTGGTCA CGGAGTATGG GACTTAATGT 100
<210> 12207
<211> 100
<212> DNA
<213> Escherichia coli K-12 MG1655
<220> yggh b2961
<222> 527-626
<223> yggh b2961 U00096 complement(3100151..3100870)
<313> 12207: 541-560
<400> 12207
ATATGGCGAC CGACTGGGAA CCTTATGCGG AACATATGCT TGAAGTGATG 50
TCTTCTATTG ACGGTTATAA AAACCTGTCA GAGAGCAATG ATTACGTACC 100
<210> 12208
<211> 100
<212> DNA
<213> Escherichia coli K-12 MG1655
<220> prlc b3499
<222> 1885-1984
<223> prlc b3499 U00096 complement(3640770..3642812)
<313> 12208: 1917-1936
<400> 12208
GAGGAAGAGG GCATTTTCAA CCGTGAAACC GGGCAGTCGT TCCTCGACAA 50
CATTCTGAGC CGTGGCGGTT CAGAAGAGCC GATGGATCTG TTCAAACGCT 100
<210> 12209
<211> 100
<212> DNA
<213> Escherichia coli K-12 MG1655
<220> prlc b3499
<222> 1812-1911
<223> prlc b3499 U00096 complement(3640770..3642812)
<313> 12209: 1864-1883
<400> 12209
CGGTGGTTAT GCCGCAGGTT ACTACAGCTA CCTGTGGGCT GACGTACTGG 50
CGGCAGATGC TTTCTCGCGC TTTGAGGAAG AGGGCATTTT CAACCGTGAA 100
<210> 12210
<211> 100
<212> DNA
<213> Escherichia coli K-12 MG1655
<220> prlc b3499
<222> 1742-1841
<223> prlc b3499 U00096 complement(3640770..3642812)
<313> 12210: 1784-1803
<400> 12210
AGAAACTGGT TGCCGTGGTG CCATCTCCGT CCTGGGGCCG TTTCCCGCAC 50
GCTTTCAGCC ATATTTTCGC CGGTGGTTAT GCCGCAGGTT ACTACAGCTA 100
<210> 12211
<211> 100
<212> DNA
<213> Escherichia coli K-12 MG1655
<220> yggj b2947
<222> 660-759
<223> yggj b2947 U00096 3089126..3089884
<313> 12211: 734-753
<400> 12211
ATTTACTGAT ATCCTGTTGG GACCTCGCGT TTTGCGTACA GAGACAACTG 50
CGCTCACCGC CATTACCGCG CTACAAGTAC GATTTGGCGA TTTGGGCTAA 100
<210> 12212
<211> 100
<212> DNA
<213> Escherichia coli K-12 MG1655
<220> yggj b2947
<222> 660-759
<223> yggj b2947 U00096 3089126..3089884

<313> 12212: 684-703
<400> 12212
ATTTACTGAT ATCCTGTTGG GACCTCGCGT TTTGCGTACA GAGACAACTG 50
CGCTCACCGC CATTACCGCG CTACAAGTAC GATTTGGCGA TTTGGGCTAA 100
<210> 12213
<211> 100
<212> DNA
<213> Escherichia coli K-12 MG1655
<220> yggj b2947
<222> 603-702
<223> yggj b2947 U00096 3089126..3089884
<313> 12213: 640-659
<400> 12213
GATTGGCCCG GAAGGCGGTT TATCGGCAGA TGAAATTGCC ATGACTGCCC 50
GCTATCAATT TACTGATATC CTGTTGGGAC CTCGCGTTTT GCGTACAGAG 100
<210> 12214
<211> 100
<212> DNA
<213> Escherichia coli K-12 MG1655
<220> yggl b2960
<222> 258-357
<223> yggl b2960 U00096 complement(3099825..3100181)
<313> 12214: 324-343
<400> 12214
ATGCACCGAA GAACATCAGG CGATTGTGCG TAAGTGGCTG GAAGAGCGCA 50
AACTGGATGA GGTACGCACC AGCGAACTTT TCGACGTTTG GTGGGACTAA 100
<210> 12215
<211> 100
<212> DNA
<213> Escherichia coli K-12 MG1655
<220> yggl b2960
<222> 227-326
<223> yggl b2960 U00096 complement(3099825..3100181)
<313> 12215: 258-277
<400> 12215
AAGGTCTGAT CTGCATGCAG GAAATCGGCA AATGCACCGA AGAACATCAG 50
GCGATTGTGC GTAAGTGGCT GGAAGAGCGC AAACTGGATG AGGTACGCAC 100
<210> 12216
<211> 100
<212> DNA
<213> Escherichia coli K-12 MG1655
<220> yggl b2960
<222> 193-292
<223> yggl b2960 U00096 complement(3099825..3100181)
<313> 12216: 205-224
<400> 12216
CTGGCCTTTG ACGGCAGCGG TTATCTGGCC TGGGAAGGTC TGATCTGCAT 50
GCAGGAAATC GGCAAATGCA CCGAAGAACA TCAGGCGATT GTGCGTAAGT 100
<210> 12217
<211> 100
<212> DNA
<213> Escherichia coli K-12 MG1655
<220> yggm b2957
<222> 909-1008
<223> yggm b2957 U00096 complement(3096577..3097584)
<313> 12217: 978-997
<400> 12217
AAAAGTCGCG ATTTTGATGA AAATTGACGA TATCAACCAA GCTAATGAAC 50
GCTGGAAAAA AGATACCGAA CAACTGCGGC GCAATGGGGT GATTAAGTAA 100
<210> 12218
<211> 100
<212> DNA
<213> Escherichia coli K-12 MG1655
<220> yggm b2957
<222> 816-915
<223> yggm b2957 U00096 complement(3096577..3097584)
<313> 12218: 849-868
<400> 12218
ACAAAAACTT AGCCGTAAAT TGCAGGATGA CTGGGTAACG CTCGAAAAAG 50
GGCAATATCC GTTAACGATG AAAGTTTCAG AAATAAACAG TAAAAAAGTC 100
<210> 12219

<211> 100
<212> DNA
<213> Escherichia coli K-12 MG1655
<220> yggm b2957
<222> 775-874
<223> yggm b2957 U00096 complement(3096577..3097584)
<313> 12219: 811-830
<400> 12219
TTCGATATCA ACGAACAACG GCGACAGACT TTTCTGCAAT CACAAAAACT 50
TAGCCGTAAA TTGCAGGATG ACTGGGTAAC GCTCGAAAAA GGGCAATATC 100
<210> 12220
<211> 100
<212> DNA
<213> Escherichia coli K-12 MG1655
<220> yggn b2959
<222> 621-720
<223> yggn b2959 U00096 complement(3098923..3099642)
<313> 12220: 675-694
<400> 12220
AAAGCAGGAA AAAGATTTCC AGCAGTTTGG CAAAGATGTT TGTAGCCGCG 50
TTGTGACTCT GGAAGATAGC CGCAAAGCCC TGGTCGGGAA TTTAAAATAA 100
<210> 12221
<211> 100
<212> DNA
<213> Escherichia coli K-12 MG1655
<220> yggn b2959
<222> 581-680
<223> yggn b2959 U00096 complement(3098923..3099642)
<313> 12221: 632-651
<400> 12221
GCCTGGGGGG GCTGCAATCC TCAATCCAAA CCGAGTGGAA AAAGCAGGAA 50
AAAGATTTCC AGCAGTTTGG CAAAGATGTT TGTAGCCGCG TTGTGACTCT 100
<210> 12222
<211> 100
<212> DNA
<213> Escherichia coli K-12 MG1655
<220> yggn b2959
<222> 553-652
<223> yggn b2959 U00096 complement(3098923..3099642)
<313> 12222: 593-612
<400> 12222
GGTAACCCAT TACAGAATGT GCTGGGAAGC CTGGGGGGGC TGCAATCCTC 50
AATCCAAACC GAGTGGAAAA AGCAGGAAAA AGATTTCCAG CAGTTTGGCA 100
<210> 12223
<211> 100
<212> DNA
<213> Escherichia coli K-12 MG1655
<220> yggp b2933
<222> 257-356
<223> yggp b2933 U00096 complement(3075119..3075475)
<313> 12223: 278-297
<400> 12223
AAAAGGCCAG CGTTTTGTAT TGCAACCGGC GATGGGGTTA CCAAGCGGAT 50
ATTCAGCGGG CTACAGCTAC GAATATTTTG GCGGCAATGC CACTTATATG 100
<210> 12224
<211> 100
<212> DNA
<213> Escherichia coli K-12 MG1655
<220> yggp b2933
<222> 172-271
<223> yggp b2933 U00096 complement(3075119..3075475)
<313> 12224: 231-250
<400> 12224
GATTTAGAAA ATCATCCGGT CATTACCGGG CATGAATGTG CCGGGGTTAT 50
TGTCGAAGTG GGTAAAAATC TCACTGGCAA ATATAAAAAG GCCAGCGTTT 100
<210> 12225
<211> 100
<212> DNA
<213> Escherichia coli K-12 MG1655
<220> yggp b2933
<222> 75-174

<223> yggp b2933 U00096 complement(3075119..3075475)
<313> 12225: 138-157
<400> 12225
CGATAATGAA TTACTGGTGA GTGTAATTTC TGACAGCGTC TGTTTATCGA 50
CCTGGAAAGC GGCGTTACTC GGTAGTGAAC ATAAACGCGT ACCCGACGAT 100
<210> 12226
<211> 100
<212> DNA
<213> Escherichia coli K-12 MG1655
<220> yggr b2951
<222> 927-1026
<223> yggr b2951 U00096 complement(3092119..3093144)
<313> 12226: 1007-1026
<400> 12226
AACCCACCAG TTACCGCATG TTATTCAAAC CGGGCAGCAG GTGGGGATGA 50
TAACGTTTCA GCAGAGTTAT CAGCACCGGG TGGGGGAAGG GCGTTTGTGA 100
<210> 12227
<211> 100
<212> DNA
<213> Escherichia coli K-12 MG1655
<220> yggr b2951
<222> 912-1011
<223> yggr b2951 U00096 complement(3092119..3093144)
<313> 12227: 950-969
<400> 12227
GATTCGCGAA GGGAAAACCC ACCAGTTACC GCATGTTATT CAAACCGGGC 50
AGCAGGTGGG GATGATAACG TTTCAGCAGA GTTATCAGCA CCGGGTGGGG 100
<210> 12228
<211> 100
<212> DNA
<213> Escherichia coli K-12 MG1655
<220> yggr b2951
<222> 790-889
<223> yggr b2951 U00096 complement(3092119..3093144)
<313> 12228: 828-847
<400> 12228
GTGCGTAATC AACTGGCAGG TAGTTTACGG GCAGTGCTGT CACAAAAGCT 50
GGAAGTGGAT AAACAGGAAG GACGCGTGGC GCTATTTGAA TTGCTGATTA 100
<210> 12229
<211> 100
<212> DNA
<213> Escherichia coli K-12 MG1655
<220> yggs b2952
<222> 606-705
<223> yggs b2952 U00096 3093117..3093821
<313> 12229: 647-666
<400> 12229
GGGAATGTCG GACGATATGG AAGCCGCCAT TGCGGCAGGT AGCACGATGG 50
TTCGTATCGG CACTGCAATT TTTGGTGCGC GTGATTACTC TAAAAAATAA 100
<210> 12230
<211> 100
<212> DNA
<213> Escherichia coli K-12 MG1655
<220> yggs b2952
<222> 587-686
<223> yggs b2952 U00096 3093117..3093821
<313> 12230: 602-621
<400> 12230
ATATCGACAC GCTCTCTCTG GGAATGTCGG ACGATATGGA AGCCGCCATT 50
GCGGCAGGTA GCACGATGGT TCGTATCGGC ACTGCAATTT TTGGTGCGCG 100
<210> 12231
<211> 100
<212> DNA
<213> Escherichia coli K-12 MG1655
<220> yggs b2952
<222> 537-636
<223> yggs b2952 U00096 3093117..3093821
<313> 12231: 569-588
<400> 12231
TGCACGCCAA ATGGCTGTAG CATTTGCCGG ACTGAAAACG CGCTACCCGC 50
ATATCGACAC GCTCTCTCTG GGAATGTCGG ACGATATGGA AGCCGCCATT 100

<210> 12232
<211> 100
<212> DNA
<213> Escherichia coli K-12 MG1655
<220> yggt b2953
<222> 468-567
<223> yggt b2953 U00096 3093839..3094405
<313> 12232: 538-557
<400> 12232
GATCCTCGTT CTGCTGCTGT ATGTCATCAA TATGGGTGTC GCAGAAGTAT 50
TACAGGCAAC CGGAAATATG CTGCTGCCAG GGCTGTGGAT GGCGTTATGA 100
<210> 12233
<211> 100
<212> DNA
<213> Escherichia coli K-12 MG1655
<220> yggt b2953
<222> 467-566
<223> yggt b2953 U00096 3093839..3094405
<313> 12233: 479-498
<400> 12233
TGATCCTCGT TCTGCTGCTG TATGTCATCA ATATGGGTGT CGCAGAAGTA 50
TTACAGGCAA CCGGAAATAT GCTGCTGCCA GGGCTGTGGA TGGCGTTATG 100
<210> 12234
<211> 100
<212> DNA
<213> Escherichia coli K-12 MG1655
<220> yggt b2953
<222> 394-493
<223> yggt b2953 U00096 3093839..3094405
<313> 12234: 410-429
<400> 12234
CAGCTGGCCG ATCCGCTGCT GCGCCCGATT CGCCGCCTGC TACCGGCAAT 50
GGGTGGGATT GATTTCTCGC CGATGATCCT CGTTCTGCTG CTGTATGTCA 100
<210> 12235
<211> 100
<212> DNA
<213> Escherichia coli K-12 MG1655
<220> b2789 b2789
<222> 1254-1353
<223> b2789 b2789 U00096 complement(2918770..2920122)
<313> 12235: 1334-1353
<400> 12235
GATTTATGTT GGTGTTCATG CCTTAATCGC GGTACTGAGC TACCTGGTGC 50
TGGTGGGCGA TATCAAGCGT ATCGAGTTGA AACCTGTTGC GGGGCAATAA 100
<210> 12236
<211> 100
<212> DNA
<213> Escherichia coli K-12 MG1655
<220> b2789 b2789
<222> 1205-1304
<223> b2789 b2789 U00096 complement(2918770..2920122)
<313> 12236: 1227-1246
<400> 12236
TCGCAATTGG TTATATCGTT GGCACGACTG GCTCGTTTAA TGGGGCGCTG 50
ATTTATGTTG GTGTTCATGC CTTAATCGCG GTACTGAGCT ACCTGGTGCT 100
<210> 12237
<211> 100
<212> DNA
<213> Escherichia coli K-12 MG1655
<220> b2789 b2789
<222> 1158-1257
<223> b2789 b2789 U00096 complement(2918770..2920122)
<313> 12237: 1198-1217
<400> 12237
TGGCCTGTTC AACATGTTCG GTAACATTTC TGGCATCGTC ACGCCAATCG 50
CAATTGGTTA TATCGTTGGC ACGACTGGCT CGTTTAATGG GGCGCTGATT 100
<210> 12238
<211> 100
<212> DNA
<213> Escherichia coli K-12 MG1655
<220> yggu b2954

<222> 204-303
<223> yggu b2954 U00096 3094390..3094692
<313> 12238: 260-279
<400> 12238
CCAGGTGGTG ATTGAAAAAG GCGAACTTGG CCGCCACAAA CAAATTAAAA 50
TCATTAATCC GCAACAAATC CCGCCAGAAA TCGCGGCGTT AATTAATTAG 100
<210> 12239
<211> 100
<212> DNA
<213> Escherichia coli K-12 MG1655
<220> yggu b2954
<222> 170-269
<223> yggu b2954 U00096 3094390..3094692
<313> 12239: 221-240
<400> 12239
AGTTTCTCGG TAAGCAATTC CGGGTTGCCA AAAGCCAGGT GGTGATTGAA 50
AAAGGCGAAC TTGGCCGCCA CAAACAAATT AAAATCATTA ATCCGCAACA 100
<210> 12240
<211> 100
<212> DNA
<213> Escherichia coli K-12 MG1655
<220> yggu b2954
<222> 96-195
<223> yggu b2954 U00096 3094390..3094692
<313> 12240: 143-162
<400> 12240
TTTACATGGC GACGAAGTTA AAGTCGCCAT TACCGCGCCG CCGGTTGACG 50
GCCAGGCCAA CAGTCATCTG GTGAAGTTTC TCGGTAAGCA ATTCCGGGTT 100
<210> 12241
<211> 100
<212> DNA
<213> Escherichia coli K-12 MG1655
<220> yggv b2955
<222> 482-581
<223> yggv b2955 U00096 3094700..3095293
<313> 12241: 521-540
<400> 12241
TCGTACCTTC CGAAGGGAAA ACCGCCGCCG AACTGACCCG CGAAGAAAAG 50
AGCGCCATTT CCCACCGTGG TCAGGCGTTG AAACTGCTGC TGGACGCTTT 100
<210> 12242
<211> 100
<212> DNA
<213> Escherichia coli K-12 MG1655
<220> yggv b2955
<222> 294-393
<223> yggv b2955 U00096 3094700..3095293
<313> 12242: 322-341
<400> 12242
TCTGCAAAAA CTGCTGGAAA CAATGAAAGA CGTACCGGAC GACCAACGTC 50
AGGCGCGTTT CCACTGCGTG CTGGTATATC TGCGTCACGC GGAAGATCCC 100
<210> 12243
<211> 100
<212> DNA
<213> Escherichia coli K-12 MG1655
<220> yggv b2955
<222> 238-337
<223> yggv b2955 U00096 3094700..3095293
<313> 12243: 269-288
<400> 12243
GCGCCGGGCA TTTACTCCGC GCGTTATTCC GGTGAAGACG CGACCGATCA 50
AAAGAATCTG CAAAAACTGC TGGAAACAAT GAAAGACGTA CCGGACGACC 100
<210> 12244
<211> 100
<212> DNA
<213> Escherichia coli K-12 MG1655
<220> yggw b2956
<222> 1038-1137
<223> yggw b2956 U00096 3095286..3096422
<313> 12244: 1114-1133
<400> 12244
TGCCCAGGGT TATCTCACCG AATGTGCGGA TTACTGGCAG ATAACGGAAC 50

ATGGGAAGCT GTTTTTAAAT TCGCTGCTGG AGCTTTTTCT GGCTGAGTAA 100
<210> 12245
<211> 100
<212> DNA
<213> Escherichia coli K-12 MG1655
<220> yggw b2956
<222> 1010-1109
<223> yggw b2956 U00096 3095286..3096422
<313> 12245: 1058-1077
<400> 12245
TGATTCGCCC ACAGTTAGAC GAGGCGATTG CCCAGGGTTA TCTCACCGAA 50
TGTGCGGATT ACTGGCAGAT AACGGAACAT GGGAAGCTGT TTTTAAATTC 100
<210> 12246
<211> 100
<212> DNA
<213> Escherichia coli K-12 MG1655
<220> yggw b2956
<222> 955-1054
<223> yggw b2956 U00096 3095286..3096422
<313> 12246: 1016-1035
<400> 12246
CTGGAGGCCG CGCCGCGCGT GGAGTTTATT GCGTATACCG GGCTTTGCGA 50
AGATGTGATT CGCCCACAGT TAGACGAGGC GATTGCCCAG GGTTATCTCA 100
<210> 12247
<211> 100
<212> DNA
<213> Escherichia coli K-12 MG1655
<220> pcka b3404
<222> 1478-1577
<223> pcka b3404 U00096 3530456..3532078
<313> 12247: 1538-1557
<400> 12247
TTCTCGATCC GCGTAACACC TACGCTTCTC CGGAACAGTG GCAGGAAAAA 50
GCCGAAACCC TGGCGAAACT GTTTATCGAC AACTTCGATA AATACACCGA 100
<210> 12248
<211> 100
<212> DNA
<213> Escherichia coli K-12 MG1655
<220> pcka b3404
<222> 1422-1521
<223> pcka b3404 U00096 3530456..3532078
<313> 12248: 1481-1500
<400> 12248
TCTGCCGATG TTTAACCTGG CGATCCCAAC CGAACTGCCG GGCGTAGACA 50
CGAAGATTCT CGATCCGCGT AACACCTACG CTTCTCCGGA ACAGTGGCAG 100
<210> 12249
<211> 100
<212> DNA
<213> Escherichia coli K-12 MG1655
<220> pcka b3404
<222> 1406-1505
<223> pcka b3404 U00096 3530456..3532078
<313> 12249: 1421-1440
<400> 12249
ATGCAGAAAC CTTCACTCTG CCGATGTTTA ACCTGGCGAT CCCAACCGAA 50
CTGCCGGGCG TAGACACGAA GATTCTCGAT CCGCGTAACA CCTACGCTTC 100
<210> 12250
<211> 100
<212> DNA
<213> Escherichia coli K-12 MG1655
<220> hsca b2527
<222> 1752-1851
<223> hsca b2527 U00096 complement(2655105..2656955)
<313> 12250: 1820-1839
<400> 12250
GATTAAAAAC GTAGACAAAC AAACCCAGGA TTTCGCCGCT CGCCGCATGG 50
ACCAGTCGGT TCGTCGTGCG CTGAAAGGCC ATTCCGTGGA CGAGGTTTAA 100
<210> 12251
<211> 100
<212> DNA
<213> Escherichia coli K-12 MG1655

<220> hsca b2527
<222> 1729-1828
<223> hsca b2527 U00096 complement(2655105..2656955)
<313> 12251: 1772-1791
<400> 12251
GATGTTGACG CCATCGAACA AGCGATTAAA AACGTAGACA AACAAACCCA 50
GGATTTCGCC GCTCGCCGCA TGGACCAGTC GGTTCGTCGT GCGCTGAAAG 100
<210> 12252
<211> 100
<212> DNA
<213> Escherichia coli K-12 MG1655
<220> hsca b2527
<222> 1694-1793
<223> hsca b2527 U00096 complement(2655105..2656955)
<313> 12252: 1733-1752
<400> 12252
CTGCCGCTCA CCTGAGTGAA GTGGCGCAGG GCGATGATGT TGACGCCATC 50
GAACAAGCGA TTAAAAACGT AGACAAACAA ACCCAGGATT TCGCCGCTCG 100
<210> 12253
<211> 100
<212> DNA
<213> Escherichia coli K-12 MG1655
<220> yggx b2963
<222> 177-276
<223> yggx b2963 U00096 3102111..3102386
<313> 12253: 252-271
<400> 12253
TGCCGAGCAC CGCAAGCTGC TTGAGCAGGA GATGGTCAAC TTCCTGTTCG 50
AGGGTAAAGA GGTGCATATC GAGGGCTATA CGCCGGAAGA TAAAAAATAA 100
<210> 12254
<211> 100
<212> DNA
<213> Escherichia coli K-12 MG1655
<220> yggx b2963
<222> 141-240
<223> yggx b2963 U00096 3102111..3102386
<313> 12254: 169-188
<400> 12254
CATGCTGATT AATGAAAAGA AACTCAACAT GATGAATGCC GAGCACCGCA 50
AGCTGCTTGA GCAGGAGATG GTCAACTTCC TGTTCGAGGG TAAAGAGGTG 100
<210> 12255
<211> 100
<212> DNA
<213> Escherichia coli K-12 MG1655
<220> yggx b2963
<222> 94-193
<223> yggx b2963 U00096 3102111..3102386
<313> 12255: 123-142
<400> 12255
AACGAGATCT CCAAAGAAGC CTGGGCGCAG TGGCAGCACA AGCAAACCAT 50
GCTGATTAAT GAAAAGAAAC TCAACATGAT GAATGCCGAG CACCGCAAGC 100
<210> 12256
<211> 100
<212> DNA
<213> Escherichia coli K-12 MG1655
<220> ygha yghA
<222> 771-870
<223> ygha yghA U00096 3147677..3148561
<313> 12256: 816-835
<400> 12256
ACGTGCGGGG CAACCGGCGG AACTGGCCCC TGTATATGTT TATCTGGCAA 50
GTCAGGAGTC GAGCTACGTC ACCGCAGAAG TGCACGGCGT GTGCGGCGGC 100
<210> 12257
<211> 100
<212> DNA
<213> Escherichia coli K-12 MG1655
<220> ygha yghA
<222> 714-813
<223> ygha yghA U00096 3147677..3148561
<313> 12257: 756-775
<400> 12257

TTCCGGCGGA CAAACGCAGG ATAAGATCCC GCAGTTTGGT CAGCAAACGC 50
CGATGAAACG TGCGGGGCAA CCGGCGGAAC TGGCCCCTGT ATATGTTTAT 100
<210> 12258
<211> 100
<212> DNA
<213> Escherichia coli K-12 MG1655
<220> ygha yghA
<222> 674-773
<223> ygha yghA U00096 3147677..3148561
<313> 12258: 723-742
<400> 12258
ATATTGTCGC GCCAGGCCCG ATCTGGACAG CACTGCAAAT TTCCGGCGGA 50
CAAACGCAGG ATAAGATCCC GCAGTTTGGT CAGCAAACGC CGATGAAACG 100
<210> 12259
<211> 100
<212> DNA
<213> Escherichia coli K-12 MG1655
<220> rtca b3421
<222> 342-441
<223> rtca b3421 U00096 complement(3554044..3554484)
<313> 12259: 395-414
<400> 12259
TGGACCTTCG CGTGTTGAAG TGAGCGCGGC GACCGATAAC CCGTCGGCGC 50
CGCCTCGGAT TTTATCCGCC GGGTGCTGGA GCCGCTGCTG GCGAAAATAG 100
<210> 12260
<211> 100
<212> DNA
<213> Escherichia coli K-12 MG1655
<220> rtca b3421
<222> 316-415
<223> rtca b3421 U00096 complement(3554044..3554484)
<313> 12260: 356-375
<400> 12260
GTGCTGCCCG CGCTGTGGTT TGCCGATGGA CCTTCGCGTG TTGAAGTGAG 50
CGCGGCGACC GATAACCCGT CGGCGCCGCC TCGGATTTTA TCCGCCGGGT 100
<210> 12261
<211> 100
<212> DNA
<213> Escherichia coli K-12 MG1655
<220> rtca b3421
<222> 139-238
<223> rtca b3421 U00096 complement(3554044..3554484)
<313> 12261: 198-217
<400> 12261
GGGCTGTTGC GCCAGCATCT GACCGCGGTA AAGGCGGCGA CGGAAATTTG 50
CGGAGCAACC GTGGAAGGCG CGGAGCTGGG GTCGCAGCGT CTGCTCTTCC 100
<210> 12262
<211> 100
<212> DNA
<213> Escherichia coli K-12 MG1655
<220> yghb b3010
<222> 561-660
<223> yghb b3010 U00096 3151578..3152237
<313> 12262: 605-624
<400> 12262
GGTAATGACG TTCCTGATGA TCCTGCCAAT TGCCTTGTTA ACCGCTGGCT 50
TGTTAGGCAC GCTGTTTGTG GTGATTAAAA AAAAATACTG TAACGCCTGA 100
<210> 12263
<211> 100
<212> DNA
<213> Escherichia coli K-12 MG1655
<220> yghb b3010
<222> 535-634
<223> yghb b3010 U00096 3151578..3152237
<313> 12263: 583-602
<400> 12263
CCGTTCGTTA AACGCCATGA AGATCAGGTA ATGACGTTCC TGATGATCCT 50
GCCAATTGCC TTGTTAACCG CTGGCTTGTT AGGCACGCTG TTTGTGGTGA 100
<210> 12264
<211> 100
<212> DNA

<213> Escherichia coli K-12 MG1655
<220> yghb b3010
<222> 479-578
<223> yghb b3010 U00096 3151578..3152237
<313> 12264: 521-540
<400> 12264
GTGGATTGCT GTGGGTCAGC GTGGTAACCA GTTTTGGCTA TGCCTTAAGT 50
ATGATTCCGT TCGTTAAACG CCATGAAGAT CAGGTAATGA CGTTCCTGAT 100
<210> 12265
<211> 100
<212> DNA
<213> Escherichia coli K-12 MG1655
<220> rtcb b3422
<222> 1128-1227
<223> rtcb b3422 U00096 complement(3554485..3555711)
<313> 12265: 1191-1210
<400> 12265
CCCGATGGCG TATAAAGATA TTGATGCGGT GATGGCGGCA CAAAGCGATC 50
TGGTGGAAGT TATCTATACC CTGCGTCAGG TGGTGTGCGT AAAAGGATAA 100
<210> 12266
<211> 100
<212> DNA
<213> Escherichia coli K-12 MG1655
<220> rtcb b3422
<222> 1127-1226
<223> rtcb b3422 U00096 complement(3554485..3555711)
<313> 12266: 1151-1170
<400> 12266
TCCCGATGGC GTATAAAGAT ATTGATGCGG TGATGGCGGC ACAAAGCGAT 50
CTGGTGGAAG TTATCTATAC CCTGCGTCAG GTGGTGTGCG TAAAAGGATA 100
<210> 12267
<211> 100
<212> DNA
<213> Escherichia coli K-12 MG1655
<220> rtcb b3422
<222> 1069-1168
<223> rtcb b3422 U00096 complement(3554485..3555711)
<313> 12267: 1117-1136
<400> 12267
CAAATTCGTG CCACCGCGCA TGTGGAATGC CGTAAAGATG CCGAAGTGAT 50
CGACGAAATC CCGATGGCGT ATAAAGATAT TGATGCGGTG ATGGCGGCAC 100
<210> 12268
<211> 100
<212> DNA
<213> Escherichia coli K-12 MG1655
<220> yghd b2969
<222> 438-537
<223> yghd b2969 U00096 complement(3108607..3109143)
<313> 12268: 512-531
<400> 12268
GGATGAAAAA TATGCGCTGC GGGTGACACA AATTGATGTC AGTGCTGCTG 50
AGAAGCCTGG GATGGTGAAT GTGCAGCGGC TGGAGTTTGG ACGGGGGTAA 100
<210> 12269
<211> 100
<212> DNA
<213> Escherichia coli K-12 MG1655
<220> yghd b2969
<222> 394-493
<223> yghd b2969 U00096 complement(3108607..3109143)
<313> 12269: 451-470
<400> 12269
GAACCTGTGG TGTTTAATGA CCTGCTGAAA TGGTTAAACG CACTGGATGA 50
AAAATATGCG CTGCGGGTGA CACAAATTGA TGTCAGTGCT GCTGAGAAGC 100
<210> 12270
<211> 100
<212> DNA
<213> Escherichia coli K-12 MG1655
<220> yghd b2969
<222> 341-440
<223> yghd b2969 U00096 complement(3108607..3109143)
<313> 12270: 397-416

<400> 12270
CGGTGGTCAT CAGGCGGATA GCCGATCGTG GGGAGAATAT TCAGGTCTGG 50
ATAGAACCTG TGGTGTTTAA TGACCTGCTG AAATGGTTAA ACGCACTGGA 100
<210> 12271
<211> 100
<212> DNA
<213> Escherichia coli K-12 MG1655
<220> yghe yghE
<222> 762-861
<223> yghe yghE U00096 complement(3109145..3110005)
<313> 12271: 817-836
<400> 12271
ACTGCGTAGT GTACTGGCAA CCGATTATGT TGTGCAACAG GACGCGTTAC 50
AGAAAGAGGG TGATGCGGTT TCCGGCGGCG TAACGTTGCG GAGGAAATAA 100
<210> 12272
<211> 100
<212> DNA
<213> Escherichia coli K-12 MG1655
<220> yghe yghE
<222> 684-783
<223> yghe yghE U00096 complement(3109145..3110005)
<313> 12272: 741-760
<400> 12272
GCGTGGTTTT ACCTTTGATC AAAAACGCCA GATACTTCAC CTCCAGCTAC 50
GGGCCGCGAA CTTTGCCAGT TTCGACAAAC TGCGTAGTGT ACTGGCAACC 100
<210> 12273
<211> 100
<212> DNA
<213> Escherichia coli K-12 MG1655
<220> yghe yghE
<222> 622-721
<223> yghe yghE U00096 complement(3109145..3110005)
<313> 12273: 681-700
<400> 12273
CTCGCCGAGT TGTCAGCGAT AGCCAGCACC CTGAAATCAG CGTCACTTTC 50
CGACATCGAA ATGCGTGGTT TTACCTTTGA TCAAAAACGC CAGATACTTC 100
<210> 12274
<211> 100
<212> DNA
<213> Escherichia coli K-12 MG1655
<220> tra8_1 tra8_1
<222> 943-1042
<223> tra8_1 tra8_1 U00096 269827..270978
<313> 12274: 974-993
<400> 12274
TGCGATCCTC AGAGTCCTTG GCAGCGGGGA ACAAATGAGA ATACAAATGG 50
GCTAATTCGG CAGTACTTTC CTAAAAAGAC ATGTCTTGCC CAATATACTC 100
<210> 12275
<211> 100
<212> DNA
<213> Escherichia coli K-12 MG1655
<220> tra8_1 tra8_1
<222> 758-857
<223> tra8_1 tra8_1 U00096 269827..270978
<313> 12275: 778-797
<400> 12275
GAAAATCACG TTATACGATC ATCGTTAGAC TCAGGGGCAA AGATTCTGTC 50
TCAGTAAATC AGGCTCTTAC CGACAAATTC CTGAGTTTAC CGTCAGAACT 100
<210> 12276
<211> 100
<212> DNA
<213> Escherichia coli K-12 MG1655
<220> tra8_1 tra8_1
<222> 681-780
<223> tra8_1 tra8_1 U00096 269827..270978
<313> 12276: 708-727
<400> 12276
CAGACGCTCT CTGGGGCATT GGGAGGGCGA TTTAGTCTCA GGTACAAAAA 50
ACTCTCATAT AGCCACACTT GTAGACCGAA AATCACGTTA TACGATCATC 100
<210> 12277
<211> 100

<212> DNA
<213> Escherichia coli K-12 MG1655
<220> prma b3260
<222> 783-882
<223> prma b3260 U00096 3406707..3407588
<313> 12277: 818-837
<400> 12277
CCAGGCAGAG AGCGTTTGTG AAGCTTATGC CGATAGCTTC GCACTGGACC 50
CGGTCGTGGA AAAAGAAGAG TGGTGCCGTA TTACCGGTCG TAAGAATTAA 100
<210> 12278
<211> 100
<212> DNA
<213> Escherichia coli K-12 MG1655
<220> prma b3260
<222> 737-836
<223> prma b3260 U00096 3406707..3407588
<313> 12278: 787-806
<400> 12278
TGCCGGTTTC AGGCGGTTTG CTGGGCCTTT CCGGTATTCT GGCAAGCCAG 50
GCAGAGAGCG TTTGTGAAGC TTATGCCGAT AGCTTCGCAC TGGACCCGGT 100
<210> 12279
<211> 100
<212> DNA
<213> Escherichia coli K-12 MG1655
<220> prma b3260
<222> 711-810
<223> prma b3260 U00096 3406707..3407588
<313> 12279: 735-754
<400> 12279
TGAACTGGCA CCGTTAATCA GCGTCCTGCC GGTTTCAGGC GGTTTGCTGG 50
GCCTTTCCGG TATTCTGGCA AGCCAGGCAG AGAGCGTTTG TGAAGCTTAT 100
<210> 12280
<211> 100
<212> DNA
<213> Escherichia coli K-12 MG1655
<220> tra8_2 b1405
<222> 931-1030
<223> tra8_2 b1405 U00096 1467382..1468533
<313> 12280: 984-1003
<400> 12280
AAAGTTTACT TCTGCGATCC TCAGAGTCCT TGGCAGCGGG GAACAAATGA 50
GAACACAAAT GGGCTAATTC GGCAGTACTT TCCTAAAAAG ACATGTCTTG 100
<210> 12281
<211> 100
<212> DNA
<213> Escherichia coli K-12 MG1655
<220> tra8_2 b1405
<222> 738-837
<223> tra8_2 b1405 U00096 1467382..1468533
<313> 12281: 775-794
<400> 12281
TATAGCCACA CTTGTAGACC GAAAATCACG TTATACGATC ATCCTTAGAC 50
TCAGGGGCAA AGATTCTGTC TCAGTAAATC AGGCTCTTAC CGACAAATTC 100
<210> 12282
<211> 100
<212> DNA
<213> Escherichia coli K-12 MG1655
<220> tra8_2 b1405
<222> 659-758
<223> tra8_2 b1405 U00096 1467382..1468533
<313> 12282: 704-723
<400> 12282
AACGTTCCCG AAATATCGAT AACAGACGCT CTCTAGGGCA TTGGGAGGGC 50
GATTTAGTCT CAGGTACAAA AAACTCTCAT ATAGCCACAC TTGTAGACCG 100
<210> 12283
<211> 100
<212> DNA
<213> Escherichia coli K-12 MG1655
<220> tra8_3 tra8_3
<222> 944-1043
<223> tra8_3 tra8_3 U00096 complement(4505034..4506185)

<313> 12283: 976-995
<400> 12283
GCGATCCTCA GAGTCCTTGG CAGCGGGGAA CAAATGAGAA CACAAATGGG 50
CTAATTCGGC AGTACTTTCC TAAAAAGACA TGTCTTGCCC AATATACTCA 100
<210> 12284
<211> 100
<212> DNA
<213> Escherichia coli K-12 MG1655
<220> tra8_3 tra8_3
<222> 725-824
<223> tra8_3 tra8_3 U00096 complement(4505034..4506185)
<313> 12284: 790-809
<400> 12284
CAAAAAACTC TCATATAGCC ACACTTGTAG ACCGAAAATC ACGTTATACG 50
ATCATCCTTA GACTCAGGGG CAAAGATTCT GTCTCAGTAA ATCAGGCTCT 100
<210> 12285
<211> 100
<212> DNA
<213> Escherichia coli K-12 MG1655
<220> tra8_3 tra8_3
<222> 645-744
<223> tra8_3 tra8_3 U00096 complement(4505034..4506185)
<313> 12285: 712-731
<400> 12285
AACACCAATT CACGAACGTT CCCGAAATAT CGATAACAGA CGCTCTCTAG 50
GGCATTGGGA GGGCGATTTA GTCTCAGGTA CAAAAAACTC TCATATAGCC 100
<210> 12286
<211> 100
<212> DNA
<213> Escherichia coli K-12 MG1655
<220> yghk b2976
<222> 1560-1659
<223> yghk b2976 U00096 complement(3117613..3119295)
<313> 12286: 1628-1647
<400> 12286
CATGGTGGGC CGAGAATCTG AACTGTTCCG CTACACCGTG AAGCACAGTC 50
TGATTTTTGC CAGCGTTATC GGCATTATCA CCCTGCTGCA GGCGTATGTG 100
<210> 12287
<211> 100
<212> DNA
<213> Escherichia coli K-12 MG1655
<220> yghk b2976
<222> 1528-1627
<223> yghk b2976 U00096 complement(3117613..3119295)
<313> 12287: 1577-1596
<400> 12287
CAATCTATCG CCGTGGCCTG CGCCGCGACG GGCATGGTGG GCCGAGAATC 50
TGAACTGTTC CGCTACACCG TGAAGCACAG TCTGATTTTT GCCAGCGTTA 100
<210> 12288
<211> 100
<212> DNA
<213> Escherichia coli K-12 MG1655
<220> yghk b2976
<222> 1468-1567
<223> yghk b2976 U00096 complement(3117613..3119295)
<313> 12288: 1516-1535
<400> 12288
GACACCCTGC TGGTGGCAGC AAACACCAGC GGCGGCGTAA CTGGCAAGAT 50
GATCTCCCCG CAATCTATCG CCGTGGCCTG CGCCGCGACG GGCATGGTGG 100
<210> 12289
<211> 100
<212> DNA
<213> Escherichia coli K-12 MG1655
<220> b2790 b2791
<222> 336-435
<223> b2790 b2791 U00096 complement(2920557..2921006)
<313> 12289: 399-418
<400> 12289
GGAACAGAGC GCTCAGCGGG TTGGCGAAAT GCTGTTGATT GACGCCAGTG 50
AGAACCCGGA ACCGGAAACG GAATCAAATC CGTGGGTCGA ACAGTGGGGC 100
<210> 12290

<211> 100
<212> DNA
<213> Escherichia coli K-12 MG1655
<220> b2790 b2791
<222> 280-379
<223> b2790 b2791 U00096 complement(2920557..2921006)
<313> 12290: 309-328
<400> 12290
GACAGTAGTT ATGTGAATTT CTGCAATGGC GGCAAACAGT TCGATGCCTT 50
GTTGCAGGAA CAGAGCGCTC AGCGGGTTGG CGAAATGCTG TTGATTGACG 100
<210> 12291
<211> 100
<212> DNA
<213> Escherichia coli K-12 MG1655
<220> b2790 b2791
<222> 152-251
<223> b2790 b2791 U00096 complement(2920557..2921006)
<313> 12291: 185-204
<400> 12291
ATGTTCTGGT GGTTACGTCC ACGACCGGGC AGGGCGACCT TCCTGATAGC 50
ATTGTGCCAC TCTTTCAGGG AATCAAAGAT AGTCTGGGCT TCCAGCCGAA 100
<210> 12292
<211> 100
<212> DNA
<213> Escherichia coli K-12 MG1655
<220> b2792 b2793
<222> 231-330
<223> b2792 b2793 U00096 complement(2921806..2922135)
<313> 12292: 307-326
<400> 12292
CTATTATGAA ATGGCGCTGG CGACGGACCA TCCGCAACGC GCGCTTATTC 50
TGGCTGAGCT GGAAAAACTG GATGCTCTTT TTGCGGATGA TGCGAGCTAA 100
<210> 12293
<211> 100
<212> DNA
<213> Escherichia coli K-12 MG1655
<220> b2792 b2793
<222> 127-226
<223> b2792 b2793 U00096 complement(2921806..2922135)
<313> 12293: 169-188
<400> 12293
GACACCATGG AACCACTGGA GTGGTTGCAG TGGGTGCTGA TCCCGCGAAT 50
GCACGATTTG TTAGATAACA AACAGCCGTT ACCGGGCGCT TTTGCCGTTG 100
<210> 12294
<211> 100
<212> DNA
<213> Escherichia coli K-12 MG1655
<220> b2792 b2793
<222> 68-167
<223> b2792 b2793 U00096 complement(2921806..2922135)
<313> 12294: 95-114
<400> 12294
ACTGGCGAAA CGATGAACCC CAGCCGCATC AATTTAATAG TACCCAACCG 50
TTCTTTATGG ACACCATGGA ACCACTGGAG TGGTTGCAGT GGGTGCTGAT 100
<210> 12295
<211> 100
<212> DNA
<213> Escherichia coli K-12 MG1655
<220> yghq b2984
<222> 879-978
<223> yghq b2984 U00096 complement(3129356..3130333)
<313> 12295: 910-929
<400> 12295
AATCGGTATT CTGGTGGCGC TCGCGGTGTT GATCGTCGGC AAGCCGCTCA 50
TTTCGCTGGT GTTTGGCGTT AAGTATCTCG AAGCGTATGA CCTGATTTAG 100
<210> 12296
<211> 100
<212> DNA
<213> Escherichia coli K-12 MG1655
<220> yghq b2984
<222> 793-892

<223> yghq b2984 U00096 complement(3129356..3130333)
<313> 12296: 857-876
<400> 12296
AGTTTTTACC CAGAGGTGAT GCGTTTAGAT CCGCGCACCA CCAGACCGTG 50
GTTGTTAGGT GTGAAGTCCG GTTTACTGGC GGGCGGAATC GGTATTCTGG 100
<210> 12297
<211> 100
<212> DNA
<213> Escherichia coli K-12 MG1655
<220> yghq b2984
<222> 763-862
<223> yghq b2984 U00096 complement(3129356..3130333)
<313> 12297: 821-840
<400> 12297
GCCGGAACGC CAGCGGGTTT GCTGGGTAAA AGTTTTTACC CAGAGGTGAT 50
GCGTTTAGAT CCGCGCACCA CCAGACCGTG GTTGTTAGGT GTGAAGTCCG 100
<210> 12298
<211> 100
<212> DNA
<213> Escherichia coli K-12 MG1655
<220> yghr b2985
<222> 660-759
<223> yghr b2985 U00096 complement(3130469..3131227)
<313> 12298: 727-746
<400> 12298
TGAGATTGAT TCCAGAGCGC CCTACAGCGA AGTGCTGGAA CAGGCACAGA 50
AGGCGGTTTC TCTGGTTGCC ATCGTTTCTG ACCGCCGAAG TTTAACTTAA 100
<210> 12299
<211> 100
<212> DNA
<213> Escherichia coli K-12 MG1655
<220> yghr b2985
<222> 650-749
<223> yghr b2985 U00096 complement(3130469..3131227)
<313> 12299: 686-705
<400> 12299
CAAAAATTCT TGAGATTGAT TCCAGAGCGC CCTACAGCGA AGTGCTGGAA 50
CAGGCACAGA AGGCGGTTTC TCTGGTTGCC ATCGTTTCTG ACCGCCGAAG 100
<210> 12300
<211> 100
<212> DNA
<213> Escherichia coli K-12 MG1655
<220> yghr b2985
<222> 582-681
<223> yghr b2985 U00096 complement(3130469..3131227)
<313> 12300: 627-646
<400> 12300
GCCTGACCAT GACTATGCCG AGCTGCAGGA CAAAATCGGT GTCATGTCGA 50
AGATTGGCTA TAACGGCACA AAAATTCTTG AGATTGATTC CAGAGCGCCC 100
<210> 12301
<211> 100
<212> DNA
<213> Escherichia coli K-12 MG1655
<220> rtcr b3423
<222> 1500-1599
<223> rtcr b3423 U00096 3555900..3557498
<313> 12301: 1560-1579
<400> 12301
GGGCAAAGCC AGCGTCAATG ACGCGGATCG GCTACGCAAA TACCTGGCGC 50
GTTTTGGTCT GACGTGGGAA GCCGTGCAGG ATCAGCACAG CTCCAGTTGA 100
<210> 12302
<211> 100
<212> DNA
<213> Escherichia coli K-12 MG1655
<220> rtcr b3423
<222> 1429-1528
<223> rtcr b3423 U00096 3555900..3557498
<313> 12302: 1475-1494
<400> 12302
GTTATCGCTA TCTGCCGCCA GGCAAAGTCG CTTTCCGCAG CCGGACGCCA 50
GCTTTTTGAC GTTTCGCGCC AGGGCAAAGC CAGCGTCAAT GACGCGGATC 100

<210> 12303
<211> 100
<212> DNA
<213> Escherichia coli K-12 MG1655
<220> rtcr b3423
<222> 1401-1500
<223> rtcr b3423 U00096 3555900..3557498
<313> 12303: 1450-1469
<400> 12303
TCTCTTCGAC CGTATGCAAC TGGAACACGT TATCGCTATC TGCCGCCAGG 50
CAAAGTCGCT TTCCGCAGCC GGACGCCAGC TTTTTGACGT TTCGCGCCAG 100
<210> 12304
<211> 100
<212> DNA
<213> Escherichia coli K-12 MG1655
<220> yghs b2986
<222> 615-714
<223> yghs b2986 U00096 complement(3131259..3131972)
<313> 12304: 691-710
<400> 12304
GACATTCAAT GGCGCAAAGA TCCTTGAACT GGATGGGCGA CACCCCGCCG 50
ATGAAATTCT GCAAGCGTCA CTACGCGCAA TTCACGCCGC GCTTTCCTGA 100
<210> 12305
<211> 100
<212> DNA
<213> Escherichia coli K-12 MG1655
<220> yghs b2986
<222> 615-714
<223> yghs b2986 U00096 complement(3131259..3131972)
<313> 12305: 650-669
<400> 12305
GACATTCAAT GGCGCAAAGA TCCTTGAACT GGATGGGCGA CACCCCGCCG 50
ATGAAATTCT GCAAGCGTCA CTACGCGCAA TTCACGCCGC GCTTTCCTGA 100
<210> 12306
<211> 100
<212> DNA
<213> Escherichia coli K-12 MG1655
<220> yghs b2986
<222> 553-652
<223> yghs b2986 U00096 complement(3131259..3131972)
<313> 12306: 602-621
<400> 12306
GCGCGTAAAC CTGACCACCA ACTGGCAGCG TTACAGGAAA AAATCGCCGT 50
TACGCCGCAA CTGACATTCA ATGGCGCAAA GATCCTTGAA CTGGATGGGC 100
<210> 12307
<211> 100
<212> DNA
<213> Escherichia coli K-12 MG1655
<220> yght b2987
<222> 537-636
<223> yght b2987 U00096 3132146..3132838
<313> 12307: 553-572
<400> 12307
ACGGGAATCG CTGGCGAGGA AGATTGCCAT AACGCCACAG TTAACCTTTG 50
GTGGTGCACA ACTGGTTGAT ATCGATGCCA ATCAGCCACT GGAACAGGTG 100
<210> 12308
<211> 100
<212> DNA
<213> Escherichia coli K-12 MG1655
<220> yght b2987
<222> 414-513
<223> yght b2987 U00096 3132146..3132838
<313> 12308: 462-481
<400> 12308
TGTCTCATGG CTGGCAAGCC AGGAACGTAA AGCGTTTCAC TGGATGGCGA 50
GCCATAAGCC TGATCTGGTC ATCAAACTCA ATGTTGACCT TGAAGTTGCC 100
<210> 12309
<211> 100
<212> DNA
<213> Escherichia coli K-12 MG1655
<220> yght b2987

<222> 378-477
<223> yght b2987 U00096 3132146..3132838
<313> 12309: 424-443
<400> 12309
TACGGTGTTC CCACCTAACG TTGAAGGTGG TCGTTTTGTC TCATGGCTGG 50
CAAGCCAGGA ACGTAAAGCG TTTCACTGGA TGGCGAGCCA TAAGCCTGAT 100
<210> 12310
<211> 100
<212> DNA
<213> Escherichia coli K-12 MG1655
<220> ygia b3037
<222> 80-179
<223> ygia b3037 U00096 3177612..3177872
<313> 12310: 115-134
<400> 12310
TCTCGCTTCA ATTTCGACCA GCCATCCTCT ATTCTGATGG GTATTTACCA 50
CTGGTCCCGG AAGACAAAAA TGAAACGGAC AAAATCCATA CGCCACGCAT 100
<210> 12311
<211> 100
<212> DNA
<213> Escherichia coli K-12 MG1655
<220> ygia b3037
<222> 23-122
<223> ygia b3037 U00096 3177612..3177872
<313> 12311: 72-91
<400> 12311
CCCGTCTGAA CGTAAGGCAA CGTAAAGATA CGGGTTATCT GCCGCATTCT 50
TCCCCCTTCT CGCTTCAATT TCGACCAGCC ATCCTCTATT CTGATGGGTA 100
<210> 12312
<211> 100
<212> DNA
<213> Escherichia coli K-12 MG1655
<220> ygia b3037
<222> 3-102
<223> ygia b3037 U00096 3177612..3177872
<313> 12312: 28-47
<400> 12312
GACGACGACG GGGCTTCGGC CCCGTCTGAA CGTAAGGCAA CGTAAAGATA 50
CGGGTTATCT GCCGCATTCT TCCCCCTTCT CGCTTCAATT TCGACCAGCC 100
<210> 12313
<211> 100
<212> DNA
<213> Escherichia coli K-12 MG1655
<220> ygib b3038
<222> 562-661
<223> ygib b3038 U00096 3177727..3178431
<313> 12313: 593-612
<400> 12313
ATGACCGTAC CGAAGACGGC AATGGCACCA AAACCGGCGA CCACCACTAC 50
CGTTACCCGT GGCGGTTTTG GTGAATCTGT TGCCAAACAA AGCACTATGC 100
<210> 12314
<211> 100
<212> DNA
<213> Escherichia coli K-12 MG1655
<220> ygib b3038
<222> 525-624
<223> ygib b3038 U00096 3177727..3178431
<313> 12314: 563-582
<400> 12314
GGGTAAAAAC TATGGCGCAG CCCAGCCAGG CCGCACCATG ACCGTACCGA 50
AGACGGCAAT GGCACCAAAA CCGGCGACCA CCACTACCGT TACCCGTGGC 100
<210> 12315
<211> 100
<212> DNA
<213> Escherichia coli K-12 MG1655
<220> ygib b3038
<222> 454-553
<223> ygib b3038 U00096 3177727..3178431
<313> 12315: 508-527
<400> 12315
TTTGCACAGC AGCCGCTGTT CTCCTCGAAA AACCCAGCCA GTCCGGCTTA 50

CGGTAAATAT ACCGACGCGA CGGGTAAAAA CTATGGCGCA GCCCAGCCAG 100
<210> 12316
<211> 100
<212> DNA
<213> Escherichia coli K-12 MG1655
<220> hsdm b4350
<222> 1491-1590
<223> hsdm b4350 U00096 complement(4579029..4580618)
<313> 12316: 1550-1569
<400> 12316
GTCTGAACTG GATGCGCTGA TGCGTGAACT GGGGGCGAGC GATGAGGCCG 50
ATTTGCAGCG TCAGTTGCTG GAAGAAGCGT TTGGTGGGGT GAAGGAATGA 100
<210> 12317
<211> 100
<212> DNA
<213> Escherichia coli K-12 MG1655
<220> hsdm b4350
<222> 1467-1566
<223> hsdm b4350 U00096 complement(4579029..4580618)
<313> 12317: 1495-1514
<400> 12317
GATGGGCGAA CTGGTACAGG CGCTGTCTGA ACTGGATGCG CTGATGCGTG 50
AACTGGGGGC GAGCGATGAG GCCGATTTGC AGCGTCAGTT GCTGGAAGAA 100
<210> 12318
<211> 100
<212> DNA
<213> Escherichia coli K-12 MG1655
<220> hsdm b4350
<222> 1423-1522
<223> hsdm b4350 U00096 complement(4579029..4580618)
<313> 12318: 1450-1469
<400> 12318
GATGCCGACA GCCTGCCGGA GCCGGATGTA TTAGCGGCAG AAGCGATGGG 50
CGAACTGGTA CAGGCGCTGT CTGAACTGGA TGCGCTGATG CGTGAACTGG 100
<210> 12319
<211> 100
<212> DNA
<213> Escherichia coli K-12 MG1655
<220> ygic b3039
<222> 1050-1149
<223> ygic b3039 U00096 3178437..3179597
<313> 12319: 1069-1088
<400> 12319
GATTGGTAGC TGGCTGGTGA ACGATCAACC CGCCGGAATT GGCATTCGTG 50
AAGACCGTGC ATTGATCACC CAGGATATGT CTCGGTTTTA TCCACATATT 100
<210> 12320
<211> 100
<212> DNA
<213> Escherichia coli K-12 MG1655
<220> ygic b3039
<222> 989-1088
<223> ygic b3039 U00096 3178437..3179597
<313> 12320: 1021-1040
<400> 12320
AAGAAGGGAT GATTGTTCAG CAATTCCACC CGTTACCGAA ATTCGGCGAC 50
AGCTATATGC TGATTGGTAG CTGGCTGGTG AACGATCAAC CCGCCGGAAT 100
<210> 12321
<211> 100
<212> DNA
<213> Escherichia coli K-12 MG1655
<220> ygic b3039
<222> 908-1007
<223> ygic b3039 U00096 3178437..3179597
<313> 12321: 954-973
<400> 12321
CGATCTTCTC CCGTGAAGGC GCAAACGTGT CGATCATTGA GAACGGCAAA 50
ACCATTGAAG CAGCGGAAGG TCCGTATGGC GAAGAAGGGA TGATTGTTCA 100
<210> 12322
<211> 100
<212> DNA
<213> Escherichia coli K-12 MG1655

<220> ygid b3040
<222> 717-816
<223> ygid b3040 U00096 complement(3179635..3180450)
<313> 12322: 775-794
<400> 12322
GTATGTGTTA GGTGCGTGGG ATGGGCAGGA GCCAATTACC ATTCCGGTTG 50
AGGGTATAGA AATGGGCAGC CTGAGTATGC TGTCGGTGCA GATAGGCTAA 100
<210> 12323
<211> 100
<212> DNA
<213> Escherichia coli K-12 MG1655
<220> ygid b3040
<222> 681-780
<223> ygid b3040 U00096 complement(3179635..3180450)
<313> 12323: 720-739
<400> 12323
ATCGAACCCA ACGCCAGAGC ATTATTTGCC GTTGTTGTAT GTGTTAGGTG 50
CGTGGGATGG GCAGGAGCCA ATTACCATTC CGGTTGAGGG TATAGAAATG 100
<210> 12324
<211> 100
<212> DNA
<213> Escherichia coli K-12 MG1655
<220> ygid b3040
<222> 648-747
<223> ygid b3040 U00096 complement(3179635..3180450)
<313> 12324: 677-696
<400> 12324
GGTGAATTAC CTCGACCATG AAGGTGGCAC GTTATCGAAC CCAACGCCAG 50
AGCATTATTT GCCGTTGTTG TATGTGTTAG GTGCGTGGGA TGGGCAGGAG 100
<210> 12325
<211> 100
<212> DNA
<213> Escherichia coli K-12 MG1655
<220> ygie b3041
<222> 675-774
<223> ygie b3041 U00096 3180566..3181339
<313> 12325: 745-764
<400> 12325
AGAGATTGAC CCTAATAATA ACCCCAGCTA TGGCGTACTA TGTGGAATGT 50
CAGTGATGGG ATTCAGTTTA GTGCTGCTAC AAACGGCGGG AATTGGTTAA 100
<210> 12326
<211> 100
<212> DNA
<213> Escherichia coli K-12 MG1655
<220> ygie b3041
<222> 622-721
<223> ygie b3041 U00096 3180566..3181339
<313> 12326: 673-692
<400> 12326
GTTGCAGGAA TTATGGTGGC GCTCTCGGTT GATGAATTAA TGCCGCTCGC 50
CAAAGAGATT GACCCTAATA ATAACCCCAG CTATGGCGTA CTATGTGGAA 100
<210> 12327
<211> 100
<212> DNA
<213> Escherichia coli K-12 MG1655
<220> ygie b3041
<222> 578-677
<223> ygie b3041 U00096 3180566..3181339
<313> 12327: 634-653
<400> 12327
GTAGCATGAT TTCCCCGGTG GTCATGGCGG CAATCATGGC GGCGGTTGCA 50
GGAATTATGG TGGCGCTCTC GGTTGATGAA TTAATGCCGC TCGCCAAAGA 100
<210> 12328
<211> 100
<212> DNA
<213> Escherichia coli K-12 MG1655
<220> ygif b3055
<222> 1195-1294
<223> ygif b3055 U00096 complement(3197305..3198606)
<313> 12328: 1241-1260
<400> 12328

CAGGGGCTGC ATCACGCTAT TGCGACCGGG CAACGCATCG AAATTGAACA 50
TTTCCGTAAT GAGGCAAACA ATCAGGAACC GTTCTGGTTG CACAGCGGAA 100
<210> 12329
<211> 100
<212> DNA
<213> Escherichia coli K-12 MG1655
<220> ygif b3055
<222> 1159-1258
<223> ygif b3055 U00096 complement(3197305..3198606)
<313> 12329: 1185-1204
<400> 12329
GATCCTGTCG TCGCGCAAGC CTGGCTGGAG AACTGGCAGG GGCTGCATCA 50
CGCTATTGCG ACCGGGCAAC GCATCGAAAT TGAACATTTC CGTAATGAGG 100
<210> 12330
<211> 100
<212> DNA
<213> Escherichia coli K-12 MG1655
<220> ygif b3055
<222> 999-1098
<223> ygif b3055 U00096 complement(3197305..3198606)
<313> 12330: 1034-1053
<400> 12330
AATCAGCGAC TCCTTCAAAC GCTTTGCCGA TATCCATCTT TCCCGCCATG 50
CCGCTGAACT GAAAAGCGTT TTCTGCCAGC CGTTAGGCGA TCGCTACCGT 100
<210> 12331
<211> 100
<212> DNA
<213> Escherichia coli K-12 MG1655
<220> ygig b3059
<222> 266-365
<223> ygig b3059 U00096 complement(3201862..3202233)
<313> 12331: 299-318
<400> 12331
ACTCGCCGTG GGTGCGTATC AAACTCAGCA AGCCAGGCGC AGTGGCGCGG 50
GCGGCGAATG TTGGCGTAAT CATTGAGCGT GGCAATAATC TGAAAGAAAA 100
<210> 12332
<211> 100
<212> DNA
<213> Escherichia coli K-12 MG1655
<220> ygig b3059
<222> 106-205
<223> ygig b3059 U00096 complement(3201862..3202233)
<313> 12332: 149-168
<400> 12332
GCGTGGGATA ACCGTAAAGC GGCGAAAAGT GATGATGTGG CGGATTGCCT 50
CAGTTACGCT GACATTGCAG AAACGGTGGT CAGCCACGTC GAGGGGGCGC 100
<210> 12333
<211> 100
<212> DNA
<213> Escherichia coli K-12 MG1655
<220> ygig b3059
<222> 60-159
<223> ygig b3059 U00096 complement(3201862..3202233)
<313> 12333: 119-138
<400> 12333
CTGGGAACAG ACCATCGAAC AGAAGTTAGT GTTCGATATC GAAATGGCGT 50
GGGATAACCG TAAAGCGGCG AAAAGTGATG ATGTGGCGGA TTGCCTCAGT 100
<210> 12334
<211> 100
<212> DNA
<213> Escherichia coli K-12 MG1655
<220> hsdr b4351
<222> 3468-3567
<223> hsdr b4351 U00096 complement(4580819..4584385)
<313> 12334: 3531-3550
<400> 12334
CCGTCGCGGC GGGAAGGCGA TGCTGCAAAG AACCTTTGAC GATAATCTCG 50
ATACCCTGCT GGGCAAATTC AGCGATTATA TCTGGGACGA GCTGGCCTGA 100
<210> 12335
<211> 100
<212> DNA

<213> Escherichia coli K-12 MG1655
<220> hsdr b4351
<222> 3467-3566
<223> hsdr b4351 U00096 complement(4580819..4584385)
<313> 12335: 3497-3516
<400> 12335
ACCGTCGCGG CGGGAAGGCG ATGCTGCAAA GAACCTTTGA CGATAATCTC 50
GATACCCTGC TGGGCAAATT CAGCGATTAT ATCTGGGACG AGCTGGCCTG 100
<210> 12336
<211> 100
<212> DNA
<213> Escherichia coli K-12 MG1655
<220> hsdr b4351
<222> 3420-3519
<223> hsdr b4351 U00096 complement(4580819..4584385)
<313> 12336: 3442-3461
<400> 12336
AGAGAAAGTG GTGCTCGACG ACGATGTCTT CAAAACCGGC AACTTCCACC 50
GTCGCGGCGG GAAGGCGATG CTGCAAAGAA CCTTTGACGA TAATCTCGAT 100
<210> 12337
<211> 100
<212> DNA
<213> Escherichia coli K-12 MG1655
<220> ygih b3060
<222> 519-618
<223> ygih b3060 U00096 3202335..3202952
<313> 12337: 568-587
<400> 12337
CCTGCTGCGT CATCATGACA ACATCCAACG TCTGTGGCGT CGTCAGGAGA 50
CAAAAATCTG GACGAAATTC AAAAGAAAGC GCGAAAAGGA TCCCGAGTGA 100
<210> 12338
<211> 100
<212> DNA
<213> Escherichia coli K-12 MG1655
<220> ygih b3060
<222> 484-583
<223> ygih b3060 U00096 3202335..3202952
<313> 12338: 518-537
<400> 12338
TTCACCTTCC CGGTTTCGAT GCTCTCTTGC CTGATCCTGC TGCGTCATCA 50
TGACAACATC CAACGTCTGT GGCGTCGTCA GGAGACAAAA ATCTGGACGA 100
<210> 12339
<211> 100
<212> DNA
<213> Escherichia coli K-12 MG1655
<220> ygih b3060
<222> 433-532
<223> ygih b3060 U00096 3202335..3202952
<313> 12339: 480-499
<400> 12339
ATTGTCAGTG CACTGATTGC TCCGTTTTAT GTCTGGTGGT TTAAGCCACA 50
ATTCACCTTC CCGGTTTCGA TGCTCTCTTG CCTGATCCTG CTGCGTCATC 100
<210> 12340
<211> 100
<212> DNA
<213> Escherichia coli K-12 MG1655
<220> hsds b4349
<222> 1187-1286
<223> hsds b4349 U00096 complement(4577638..4579032)
<313> 12340: 1238-1257
<400> 12340
AAAAACAGGT CAACAACGCC TTAGCCCGCG TCAACAACCT GACGCAATCC 50
ATCCTGGCAA AAGCGTTCCG TGGTGAACTT ACCGCCCAGT GGCGGGCCGA 100
<210> 12341
<211> 100
<212> DNA
<213> Escherichia coli K-12 MG1655
<220> hsds b4349
<222> 1172-1271
<223> hsds b4349 U00096 complement(4577638..4579032)
<313> 12341: 1209-1228

<400> 12341
ACGCCGACAC CATAGAAAAA CAGGTCAACA ACGCCTTAGC CCGCGTCAAC 50
AACCTGACGC AATCCATCCT GGCAAAAGCG TTCCGTGGTG AACTTACCGC 100
<210> 12342
<211> 100
<212> DNA
<213> Escherichia coli K-12 MG1655
<220> hsds b4349
<222> 1117-1216
<223> hsds b4349 U00096 complement(4577638..4579032)
<313> 12342: 1158-1177
<400> 12342
CCTCCAGTAA AAGAACAAGC CGAAATCGTT CGCCGCGTCG AGCAACTCTT 50
CGCCTACGCC GACACCATAG AAAAACAGGT CAACAACGCC TTAGCCCGCG 100
<210> 12343
<211> 100
<212> DNA
<213> Escherichia coli K-12 MG1655
<220> ygil b3044
<222> 453-552
<223> ygil b3044 U00096 3183430..3183981
<313> 12343: 518-537
<400> 12343
TCAGGAACTG AATTTCAAAG CTCGTATGGA ATCCCCAAGC AAAGATGCCA 50
CCCCGGGCAA CGTTTACGCT CAGGCTGACT ACAAGATTGC TTACGAGTAA 100
<210> 12344
<211> 100
<212> DNA
<213> Escherichia coli K-12 MG1655
<220> ygil b3044
<222> 442-541
<223> ygil b3044 U00096 3183430..3183981
<313> 12344: 476-495
<400> 12344
ACCAACAGCT ATCAGGAACT GAATTTCAAA GCTCGTATGG AATCCCCAAG 50
CAAAGATGCC ACCCCGGGCA ACGTTTACGC TCAGGCTGAC TACAAGATTG 100
<210> 12345
<211> 100
<212> DNA
<213> Escherichia coli K-12 MG1655
<220> ygil b3044
<222> 370-469
<223> ygil b3044 U00096 3183430..3183981
<313> 12345: 403-422
<400> 12345
GGCGTACGTA TTCTGGACAG CCAGAGCGGC GAAGTAACTT TAGGTACTCC 50
GGTAGTAATT ACGTTCAACA ATACCAACAG CTATCAGGAA CTGAATTTCA 100
<210> 12346
<211> 100
<212> DNA
<213> Escherichia coli K-12 MG1655
<220> ygim b3056
<222> 522-621
<223> ygim b3056 U00096 3198848..3199468
<313> 12346: 595-614
<400> 12346
TATGTATGGC GGTGGCGTGC TGGGGCTTGG CTTGCTGCTC GGTCTGGTAC 50
TGCCGCACCT GATCCCAAGC CGCAAACGCA AAGATCGCTG GATGAACTAA 100
<210> 12347
<211> 100
<212> DNA
<213> Escherichia coli K-12 MG1655
<220> ygim b3056
<222> 494-593
<223> ygim b3056 U00096 3198848..3199468
<313> 12347: 547-566
<400> 12347
AACAGCGCAC CATCATCATG CAATGGTTTA TGTATGGCGG TGGCGTGCTG 50
GGGCTTGGCT TGCTGCTCGG TCTGGTACTG CCGCACCTGA TCCCAAGCCG 100
<210> 12348
<211> 100

<212> DNA
<213> Escherichia coli K-12 MG1655
<220> ygim b3056
<222> 466-565
<223> ygim b3056 U00096 3198848..3199468
<313> 12348: 508-527
<400> 12348
GATGCCGCCA GCGTACAGCT GGATGACAAA CAGCGCACCA TCATCATGCA 50
ATGGTTTATG TATGGCGGTG GCGTGCTGGG GCTTGGCTTG CTGCTCGGTC 100
<210> 12349
<211> 100
<212> DNA
<213> Escherichia coli K-12 MG1655
<220> ygin b3030
<222> 216-315
<223> ygin b3030 U00096 3171158..3171472
<313> 12349: 259-278
<400> 12349
TGAAGCGCAT CTGCAAACCC CGCACATGAA GGCGTATAGC GAAGCCGTAA 50
AAGGTGACGT GCTGGAGATG AATATCCGTA TTCTGCAGCC AGGGATTTAA 100
<210> 12350
<211> 100
<212> DNA
<213> Escherichia coli K-12 MG1655
<220> ygin b3030
<222> 178-277
<223> ygin b3030 U00096 3171158..3171472
<313> 12350: 222-241
<400> 12350
ATCGTGATGA TTGAGCAGTG GGAAAGCATC GCGCATCTTG AAGCGCATCT 50
GCAAACCCCG CACATGAAGG CGTATAGCGA AGCCGTAAAA GGTGACGTGC 100
<210> 12351
<211> 100
<212> DNA
<213> Escherichia coli K-12 MG1655
<220> ygin b3030
<222> 114-213
<223> ygin b3030 U00096 3171158..3171472
<313> 12351: 163-182
<400> 12351
CGGCTATGCG CCAATGGTGG ATTGCGCAGC TGGCGTGAGT TTCCAGTCTA 50
TGGCACCGGA TTCTATCGTG ATGATTGAGC AGTGGGAAAG CATCGCGCAT 100
<210> 12352
<211> 100
<212> DNA
<213> Escherichia coli K-12 MG1655
<220> ygip b3061
<222> 834-933
<223> ygip b3061 U00096 complement(3202965..3203897)
<313> 12352: 895-914
<400> 12352
GCTCTATCGC AGCATGAAAT TACGTGTCTG CGTGGAATTT CTGGCGGCAT 50
GGTGCCAGCA ACGGCTGGGC AAGCCCGATG AAGGCTATCA GGTCATGTAG 100
<210> 12353
<211> 100
<212> DNA
<213> Escherichia coli K-12 MG1655
<220> ygip b3061
<222> 801-900
<223> ygip b3061 U00096 complement(3202965..3203897)
<313> 12353: 839-858
<400> 12353
AAGCGCCAAT ATCTGGGCTG TTTACCGGGA GCCGCTCTAT CGCAGCATGA 50
AATTACGTGT CTGCGTGGAA TTTCTGGCGG CATGGTGCCA GCAACGGCTG 100
<210> 12354
<211> 100
<212> DNA
<213> Escherichia coli K-12 MG1655
<220> ygip b3061
<222> 753-852
<223> ygip b3061 U00096 complement(3202965..3203897)

<313> 12354: 802-821
<400> 12354
GTTTCTGGAG AGTGGCAAAC TGGTGCAAGT ATTGCCAGAG TATGCACAAA 50
GCGCCAATAT CTGGGCTGTT TACCGGGAGC CGCTCTATCG CAGCATGAAA 100
<210> 12355
<211> 100
<212> DNA
<213> Escherichia coli K-12 MG1655
<220> reca b2700
<222> 943-1042
<223> reca b2700 U00096 complement(2820731..2821792)
<313> 12355: 999-1018
<400> 12355
GAAACCGCGA AAGAGATCGA GAAGAAAGTA CGTGAGTTGC TGCTGAGCAA 50
CCCGAACTCA ACGCCGGATT TCTCTGTAGA TGATAGCGAA GGCGTAGCAG 100
<210> 12356
<211> 100
<212> DNA
<213> Escherichia coli K-12 MG1655
<220> reca b2700
<222> 903-1002
<223> reca b2700 U00096 complement(2820731..2821792)
<313> 12356: 945-964
<400> 12356
GGGTAAAGCG AATGCGACTG CCTGGCTGAA AGATAACCCG GAAACCGCGA 50
AAGAGATCGA GAAGAAAGTA CGTGAGTTGC TGCTGAGCAA CCCGAACTCA 100
<210> 12357
<211> 100
<212> DNA
<213> Escherichia coli K-12 MG1655
<220> reca b2700
<222> 891-990
<223> reca b2700 U00096 complement(2820731..2821792)
<313> 12357: 905-924
<400> 12357
GAAGATCGGT CAGGGTAAAG CGAATGCGAC TGCCTGGCTG AAAGATAACC 50
CGGAAACCGC GAAAGAGATC GAGAAGAAAG TACGTGAGTT GCTGCTGAGC 100
<210> 12358
<211> 100
<212> DNA
<213> Escherichia coli K-12 MG1655
<220> ygir b3017
<222> 879-978
<223> ygir b3017 U00096 complement(3158185..3159162)
<313> 12358: 928-947
<400> 12358
GCTGCCTTCT TTCGAGAAAG TGAAGGGCGA TAAAGTGCTG TACGCCCATG 50
CTTCGCGTAT TCTGCACCAC GAAACCAACC CAGCTGTGCC CGCGCATTGA 100
<210> 12359
<211> 100
<212> DNA
<213> Escherichia coli K-12 MG1655
<220> ygir b3017
<222> 802-901
<223> ygir b3017 U00096 complement(3158185..3159162)
<313> 12359: 844-863
<400> 12359
GCACCGAAAA AGCAGGAAGC CAAAGCCGTA ACCGTGCAGC CACCGCGCCC 50
GAAACCGTGG GAAAAAACCT ACGTGTTGCT GCCTTCTTTC GAGAAAGTGA 100
<210> 12360
<211> 100
<212> DNA
<213> Escherichia coli K-12 MG1655
<220> ygir b3017
<222> 676-775
<223> ygir b3017 U00096 complement(3158185..3159162)
<313> 12360: 730-749
<400> 12360
ATCGTGAAAG AGGCGCTGCC TGGCTGGAGC GGCGTGGATT CCACCCGTCT 50
TGATACCCCT GGAAAAATCG ACCCAATCCC GCATCCGTAT GGTGAAGATT 100
<210> 12361

<211> 100
<212> DNA
<213> Escherichia coli K-12 MG1655
<220> mlc b1595
<222> 1122-1221
<223> mlc b1595 U00096 complement(1665368..1666588)
<313> 12361: 1173-1192
<400> 12361
GAGTACTCAG TTTTCTAACC AGGGCACGAT GGCAGGCGCT GCACTGGTAA 50
AAGACGCGAT GTATAACGGT TCTTTGTTGA TTCGTCTGTT GCAGGGTTAA 100
<210> 12362
<211> 100
<212> DNA
<213> Escherichia coli K-12 MG1655
<220> mlc b1595
<222> 1064-1163
<223> mlc b1595 U00096 complement(1665368..1666588)
<313> 12362: 1086-1105
<400> 12362
TCTCAGACAG CATCCGTCAG CAGGCCCTTC CTGCGTATAG TCAGCACATC 50
AGCGTTGAGA GTACTCAGTT TTCTAACCAG GGCACGATGG CAGGCGCTGC 100
<210> 12363
<211> 100
<212> DNA
<213> Escherichia coli K-12 MG1655
<220> mlc b1595
<222> 1026-1125
<223> mlc b1595 U00096 complement(1665368..1666588)
<313> 12363: 1053-1072
<400> 12363
ACCGTTAAGT AAAGCGGCAG ATATCCTCTT CCCGGTCATC TCAGACAGCA 50
TCCGTCAGCA GGCCCTTCCT GCGTATAGTC AGCACATCAG CGTTGAGAGT 100
<210> 12364
<211> 100
<212> DNA
<213> Escherichia coli K-12 MG1655
<220> recb b2821
<222> 3444-3543
<223> recb b2821 U00096 complement(2950483..2954025)
<313> 12364: 3486-3505
<400> 12364
AGAACATCCG CAACAGGGGA TTTACACAAC CCGACCCAAC GCCGGGTTGA 50
TTGCCCTGAT GGATGAGATG TTTGCCGGTA TGACCCTGGA GGAGGCGTAA 100
<210> 12365
<211> 100
<212> DNA
<213> Escherichia coli K-12 MG1655
<220> recb b2821
<222> 3394-3493
<223> recb b2821 U00096 complement(2950483..2954025)
<313> 12365: 3459-3478
<400> 12365
GAGCACCACT TTGGCGGCGT TATTTATCTG TTCCTGCGTG GCGTTGATAA 50
AGAACATCCG CAACAGGGGA TTTACACAAC CCGACCCAAC GCCGGGTTGA 100
<210> 12366
<211> 100
<212> DNA
<213> Escherichia coli K-12 MG1655
<220> recb b2821
<222> 3382-3481
<223> recb b2821 U00096 complement(2950483..2954025)
<313> 12366: 3410-3429
<400> 12366
GATTACGACT ATGAGCACCA CTTTGGCGGC GTTATTTATC TGTTCCTGCG 50
TGGCGTTGAT AAAGAACATC CGCAACAGGG GATTTACACA ACCCGACCCA 100
<210> 12367
<211> 100
<212> DNA
<213> Escherichia coli K-12 MG1655
<220> recc b2823
<222> 3270-3369

<223> recc b2823 U00096 complement(2957082..2960450)
<313> 12367: 3344-3363
<400> 12367
TCAAAGGCTC TGGCGGCAAT TAACACCAGA GACAATGGAG GCCATCGTTG 50
AACAGTCGCA ACGTTTCCTG TTACCGCTGT TTCGCTTTAA TCAGTCATGA 100
<210> 12368
<211> 100
<212> DNA
<213> Escherichia coli K-12 MG1655
<220> recc b2823
<222> 3249-3348
<223> recc b2823 U00096 complement(2957082..2960450)
<313> 12368: 3291-3310
<400> 12368
CGAAGGTGAT GATATCTGGT ATCAAAGGCT CTGGCGGCAA TTAACACCAG 50
AGACAATGGA GGCCATCGTT GAACAGTCGC AACGTTTCCT GTTACCGCTG 100
<210> 12369
<211> 100
<212> DNA
<213> Escherichia coli K-12 MG1655
<220> recc b2823
<222> 3199-3298
<223> recc b2823 U00096 complement(2957082..2960450)
<313> 12369: 3239-3258
<400> 12369
GCCCGTACGA AATTCCTTCA GGCTTACGAA GGCAACATGA TGGTGCGTGG 50
CGAAGGTGAT GATATCTGGT ATCAAAGGCT CTGGCGGCAA TTAACACCAG 100
<210> 12370
<211> 100
<212> DNA
<213> Escherichia coli K-12 MG1655
<220> recd b2820
<222> 1728-1827
<223> recd b2820 U00096 complement(2948657..2950483)
<313> 12370: 1779-1798
<400> 12370
TCTGTCGCTG TATGCCGATG AGCGCATATT AAGTGCGGCA ATCGCCACTC 50
GTACTGAGCG GCGCAGTGGT CTGGCGGCGT TGTTTAGTTC ACGGGAATAA 100
<210> 12371
<211> 100
<212> DNA
<213> Escherichia coli K-12 MG1655
<220> recd b2820
<222> 1693-1792
<223> recd b2820 U00096 complement(2948657..2950483)
<313> 12371: 1729-1748
<400> 12371
CTGGTTTATA CCGCGGTGAC CCGCGCGCGT CGCCGTCTGT CGCTGTATGC 50
CGATGAGCGC ATATTAAGTG CGGCAATCGC CACTCGTACT GAGCGGCGCA 100
<210> 12372
<211> 100
<212> DNA
<213> Escherichia coli K-12 MG1655
<220> recd b2820
<222> 1608-1707
<223> recd b2820 U00096 complement(2948657..2950483)
<313> 12372: 1676-1695
<400> 12372
GACGGTACAT AAATCGCAGG GATCGGAGTT CGACCATGCG GCGTTGATTT 50
TGCCGAGCCA ACGCACGCCG GTAGTAACGC GAGAGCTGGT TTATACCGCG 100
<210> 12373
<211> 100
<212> DNA
<213> Escherichia coli K-12 MG1655
<220> rece b1351
<222> 2502-2601
<223> rece b1351 U00096 complement(1412810..1415410)
<313> 12373: 2580-2599
<400> 12373
CAATCTGCGA ACCCTGTCTG ACTGCCTGAA TACCGATGAA TGGCCAGCTA 50
TTAAGACATT ATCACTGCCC CGCTGGGCTA AGGAATATGC AAATGACTAA 100

<210> 12374
<211> 100
<212> DNA
<213> Escherichia coli K-12 MG1655
<220> rece b1351
<222> 2471-2570
<223> rece b1351 U00096 complement(1412810..1415410)
<313> 12374: 2517-2536
<400> 12374
CAAAACTGGC AGGTCAACAG GAATATCACC GCAATCTGCG AACCCTGTCT 50
GACTGCCTGA ATACCGATGA ATGGCCAGCT ATTAAGACAT TATCACTGCC 100
<210> 12375
<211> 100
<212> DNA
<213> Escherichia coli K-12 MG1655
<220> rece b1351
<222> 2418-2517
<223> rece b1351 U00096 complement(1412810..1415410)
<313> 12375: 2475-2494
<400> 12375
AACTATTGAA TGCGGACGTT ATCCGGTTGA AATTTTCATG ATGGGCGAAG 50
AAGCAAAACT GGCAGGTCAA CAGGAATATC ACCGCAATCT GCGAACCCTG 100
<210> 12376
<211> 100
<212> DNA
<213> Escherichia coli K-12 MG1655
<220> recf b3701
<222> 975-1074
<223> recf b3701 U00096 complement(3877776..3878849)
<313> 12376: 1034-1053
<400> 12376
ACAGGTCTTT GTCAGCGCGA TCAGTGCTGA ACACGTTATA GACATGTCGG 50
ACGAAAATTC GAAGATGTTT ACCGTGGAAA AGGGTAAAAT AACGGATTAA 100
<210> 12377
<211> 100
<212> DNA
<213> Escherichia coli K-12 MG1655
<220> recf b3701
<222> 942-1041
<223> recf b3701 U00096 complement(3877776..3878849)
<313> 12377: 969-988
<400> 12377
GCTGCTTGCC AGCCGCTTAA AAGCGACGCA ATCACAGGTC TTTGTCAGCG 50
CGATCAGTGC TGAACACGTT ATAGACATGT CGGACGAAAA TTCGAAGATG 100
<210> 12378
<211> 100
<212> DNA
<213> Escherichia coli K-12 MG1655
<220> recf b3701
<222> 873-972
<223> recf b3701 U00096 complement(3877776..3878849)
<313> 12378: 921-940
<400> 12378
TGAAAGCGGG CGGCGGTGTC TCTACCTGAT AGATGATTTT GCCTCTGAGC 50
TTGATGATGA GCGTCGCGGG CTGCTTGCCA GCCGCTTAAA AGCGACGCAA 100
<210> 12379
<211> 100
<212> DNA
<213> Escherichia coli K-12 MG1655
<220> ygiw b3025
<222> 263-362
<223> ygiw b3025 U00096 complement(3167300..3167692)
<313> 12379: 305-324
<400> 12379
ATATCGACCA CAAACGCTGG AACGGCGTGA CGGTGACGCC GAAAGATACG 50
GTTGAGATTC AGGGTGAAGT CGATAAAGAC TGGAATTCTG TTGAAATTGA 100
<210> 12380
<211> 100
<212> DNA
<213> Escherichia coli K-12 MG1655
<220> ygiw b3025

<222> 191-290
<223> ygiw b3025 U00096 complement(3167300..3167692)
<313> 12380: 225-244
<400> 12380
GCGGCAATAT CGTTGAACGC ATCTCTGACG ATCTCTACGT GTTCAAAGAT 50
GCCAGCGGTA CTATCAATGT TGATATCGAC CACAAACGCT GGAACGGCGT 100
<210> 12381
<211> 100
<212> DNA
<213> Escherichia coli K-12 MG1655
<220> ygiw b3025
<222> 116-215
<223> ygiw b3025 U00096 complement(3167300..3167692)
<313> 12381: 156-175
<400> 12381
TCCAGGGGCC GAACGGCAGC GTAACGACTG TAGAAAGCGC AAAATCCCTG 50
CGTGACGACA CCTGGGTAAC CCTGCGCGGC AATATCGTTG AACGCATCTC 100
<210> 12382
<211> 100
<212> DNA
<213> Escherichia coli K-12 MG1655
<220> recg b3653
<222> 1983-2082
<223> recg b3653 U00096 3822837..3824918
<313> 12382: 2057-2076
<400> 12382
TCAGCGCCTG GCACGCCATA TTCACGAACG TTACCCACAA CAGGCAAAAG 50
CCCTGATAGA ACGCTGGATG CCGGAGACGG AACGTTACTC GAATGCGTAA 100
<210> 12383
<211> 100
<212> DNA
<213> Escherichia coli K-12 MG1655
<220> recg b3653
<222> 1954-2053
<223> recg b3653 U00096 3822837..3824918
<313> 12383: 2010-2029
<400> 12383
CTGCGCGATC AGGCGATGAT CCCGGAAGTT CAGCGCCTGG CACGCCATAT 50
TCACGAACGT TACCCACAAC AGGCAAAAGC CCTGATAGAA CGCTGGATGC 100
<210> 12384
<211> 100
<212> DNA
<213> Escherichia coli K-12 MG1655
<220> recg b3653
<222> 1922-2021
<223> recg b3653 U00096 3822837..3824918
<313> 12384: 1960-1979
<400> 12384
CGGGTAATGC TGAATTTAAA GTGGCGGATT TACTGCGCGA TCAGGCGATG 50
ATCCCGGAAG TTCAGCGCCT GGCACGCCAT ATTCACGAAC GTTACCCACA 100
<210> 12385
<211> 100
<212> DNA
<213> Escherichia coli K-12 MG1655
<220> ygix b3026
<222> 555-654
<223> ygix b3026 U00096 3167844..3168503
<313> 12385: 591-610
<400> 12385
CACCAGTAAT GCCGTTGAAG TGCATGTGCA TCATCTGCGA CGCAAACTCG 50
GTAGTGATTT TATTCGTACC GTGCATGGTA TTGGTTACAC ATTAGGTGAG 100
<210> 12386
<211> 100
<212> DNA
<213> Escherichia coli K-12 MG1655
<220> ygix b3026
<222> 490-589
<223> ygix b3026 U00096 3167844..3168503
<313> 12386: 539-558
<400> 12386
AACGCTGGTC GGGTACTGTC GCGCAAACTG ATTGAAGAGA AACTGTATAC 50

CTGGGACGAA GAGGTCACCA GTAATGCCGT TGAAGTGCAT GTGCATCATC 100
<210> 12387
<211> 100
<212> DNA
<213> Escherichia coli K-12 MG1655
<220> ygix b3026
<222> 451-550
<223> ygix b3026 U00096 3167844..3168503
<313> 12387: 500-519
<400> 12387
AAACCAAAAG AATTTGCCCT GCTGGAATTA CTGATGCGTA ACGCTGGTCG 50
GGTACTGTCG CGCAAACTGA TTGAAGAGAA ACTGTATACC TGGGACGAAG 100
<210> 12388
<211> 100
<212> DNA
<213> Escherichia coli K-12 MG1655
<220> ygiy ygiY
<222> 1251-1350
<223> ygiy ygiY U00096 3168500..3169849
<313> 12388: 1321-1340
<400> 12388
TGGGCTATCG ATTGTCCAGC GAATCGCCAA ATTGCATGGC ATGAATGTTG 50
AATTTGGGAA TGCGGAACAA GGTGGATTTG AGGCGAAGGT AAGCTGGTAA 100
<210> 12389
<211> 100
<212> DNA
<213> Escherichia coli K-12 MG1655
<220> ygiy ygiY
<222> 1246-1345
<223> ygiy ygiY U00096 3168500..3169849
<313> 12389: 1268-1287
<400> 12389
GGGCTTGGGC TATCGATTGT CCAGCGAATC GCCAAATTGC ATGGCATGAA 50
TGTTGAATTT GGGAATGCGG AACAAGGTGG ATTTGAGGCG AAGGTAAGCT 100
<210> 12390
<211> 100
<212> DNA
<213> Escherichia coli K-12 MG1655
<220> ygiy ygiY
<222> 1176-1275
<223> ygiy ygiY U00096 3168500..3169849
<313> 12390: 1228-1247
<400> 12390
GACACCAGAG GCACTGGCGC GAATTGGCGA ACGCTTCTAT CGCCCACCCG 50
GACAAACCGC TACCGGCAGC GGGCTTGGGC TATCGATTGT CCAGCGAATC 100
<210> 12391
<211> 100
<212> DNA
<213> Escherichia coli K-12 MG1655
<220> recj b2893
<222> 1635-1734
<223> recj b2893 U00096 complement(3034393..3036126)
<313> 12391: 1702-1721
<400> 12391
CGGCGTGCGC GAAGTGCAAC TGGCTTATAA GCTCGATATC AACGAGTTTC 50
GCGGCAACCG CAGCCTGCAA ATTATCATCG ACAATATCTG GCCAATTTAG 100
<210> 12392
<211> 100
<212> DNA
<213> Escherichia coli K-12 MG1655
<220> recj b2893
<222> 1474-1573
<223> recj b2893 U00096 complement(3034393..3036126)
<313> 12392: 1536-1555
<400> 12392
GGGCAGATGT TCCCGGAGCC GCTGTTTGAC GGTCATTTCC GTCTGCTGCA 50
ACAGCGGCTG GTGGGCGAAC GTCATTTGAA GGTGATGGTC GAACCGGTCG 100
<210> 12393
<211> 100
<212> DNA
<213> Escherichia coli K-12 MG1655

<220> recj b2893
<222> 1449-1548
<223> recj b2893 U00096 complement(3034393..3036126)
<313> 12393: 1482-1501
<400> 12393
GCTGCTGCGC GATGCTGGCC CGTGGGGGCA GATGTTCCCG GAGCCGCTGT 50
TTGACGGTCA TTTCCGTCTG CTGCAACAGC GGCTGGTGGG CGAACGTCAT 100
<210> 12394
<211> 100
<212> DNA
<213> Escherichia coli K-12 MG1655
<220> recn b2617
<222> 1563-1662
<223> recn b2617 U00096 2749816..2751477
<313> 12394: 1641-1660
<400> 12394
GAATAAAAAA GCGCGGTTAC AAGAGCTGGC GCGCCTGCTT GGTGGCAGTG 50
AAGTCACACG TAATACACTG GCGAATGCGA AAGAACTGCT TGCAGCGTAA 100
<210> 12395
<211> 100
<212> DNA
<213> Escherichia coli K-12 MG1655
<220> recn b2617
<222> 1528-1627
<223> recn b2617 U00096 2749816..2751477
<313> 12395: 1564-1583
<400> 12395
GATGGTGCGA TGACAGAAAC GCATATGCAA TCCCTGAATA AAAAAGCGCG 50
GTTACAAGAG CTGGCGCGCC TGCTTGGTGG CAGTGAAGTC ACACGTAATA 100
<210> 12396
<211> 100
<212> DNA
<213> Escherichia coli K-12 MG1655
<220> recn b2617
<222> 1487-1586
<223> recn b2617 U00096 2749816..2751477
<313> 12396: 1503-1522
<400> 12396
CGGGATGTGG TCATCAACAC TATTTTGTCA GCAAAGAAAC CGATGGTGCG 50
ATGACAGAAA CGCATATGCA ATCCCTGAAT AAAAAAGCGC GGTTACAAGA 100
<210> 12397
<211> 100
<212> DNA
<213> Escherichia coli K-12 MG1655
<220> reco b2566
<222> 630-729
<223> reco b2566 U00096 complement(2699761..2700489)
<313> 12397: 695-714
<400> 12397
CATGGCGCTT AAGCCGTATC TTGGCGGTAA ACCTTTAAAG AGCAGGGAAC 50
TGTTCCGGCA GTTTATGCCT AAGCGAACGG TGAAAACACA TTATGAATGA 100
<210> 12398
<211> 100
<212> DNA
<213> Escherichia coli K-12 MG1655
<220> reco b2566
<222> 535-634
<223> reco b2566 U00096 complement(2699761..2700489)
<313> 12398: 554-573
<400> 12398
GACAATAAAA CGTTCACCGG AAGGCAGTTA AAAGCGTTAA ACGCACGGGA 50
ATTTCCTGAC GCAGACACAC TGCGCGCCGC GAAACGCTTT ACCCGCATGG 100
<210> 12399
<211> 100
<212> DNA
<213> Escherichia coli K-12 MG1655
<220> reco b2566
<222> 480-579
<223> reco b2566 U00096 complement(2699761..2700489)
<313> 12399: 504-523
<400> 12399

CACCATGACG TATCGTTATC GCGAAGAAAA AGGGTTTATC GCAAGCGTCG 50
TTATCGACAA TAAAACGTTC ACCGGAAGGC AGTTAAAAGC GTTAAACGCA 100
<210> 12400
<211> 100
<212> DNA
<213> Escherichia coli K-12 MG1655
<220> recq b3823
<222> 1695-1794
<223> recq b3823 U00096 4003469..4005301
<313> 12400: 1720-1739
<400> 12400
TGAGATGGCT GAACAGATGC CGATCACCGC CAGCGAAATG CTCAGCGTTA 50
ACGGCGTTGG GATGCGCAAG CTGGAACGCT TTGGCAAACC GTTTATGGCG 100
<210> 12401
<211> 100
<212> DNA
<213> Escherichia coli K-12 MG1655
<220> recq b3823
<222> 1605-1704
<223> recq b3823 U00096 4003469..4005301
<313> 12401: 1645-1664
<400> 12401
ACTGTTCGCC AAATTACGCA AACTGCGTAA ATCGATAGCC GATGAAAGTA 50
ATGTCCCGCC GTACGTGGTG TTTAACGACG CAACCTTGAT TGAGATGGCT 100
<210> 12402
<211> 100
<212> DNA
<213> Escherichia coli K-12 MG1655
<220> recq b3823
<222> 1571-1670
<223> recq b3823 U00096 4003469..4005301
<313> 12402: 1604-1623
<400> 12402
TGCAGAAATC GTTCGGCGGC AACTATGATC GCAAACTGTT CGCCAAATTA 50
CGCAAACTGC GTAAATCGAT AGCCGATGAA AGTAATGTCC CGCCGTACGT 100
<210> 12403
<211> 100
<212> DNA
<213> Escherichia coli K-12 MG1655
<220> recr b0473
<222> 507-606
<223> recr b0473 U00096 493629..494234
<313> 12403: 581-600
<400> 12403
CCGAATCGCT CATGGCGTTC CGGTTGGCGG CGAGCTGGAA ATGGTCGACG 50
GCACCACGTT GTCACACTCC CTTGCCGGGC GTCATAAGAT TCGTTTTTAA 100
<210> 12404
<211> 100
<212> DNA
<213> Escherichia coli K-12 MG1655
<220> recr b0473
<222> 444-543
<223> recr b0473 U00096 493629..494234
<313> 12404: 507-526
<400> 12404
TGAAGGTGAA GCTACCGCTA ACTACATTGC CGAGCTTTGC GCGCAATATG 50
ACGTGGAAGC CAGCCGAATC GCTCATGGCG TTCCGGTTGG CGGCGAGCTG 100
<210> 12405
<211> 100
<212> DNA
<213> Escherichia coli K-12 MG1655
<220> recr b0473
<222> 415-514
<223> recr b0473 U00096 493629..494234
<313> 12405: 455-474
<400> 12405
GAAGTGATCC TCGCCACCAA CCCCACGGTT GAAGGTGAAG CTACCGCTAA 50
CTACATTGCC GAGCTTTGCG CGCAATATGA CGTGGAAGCC AGCCGAATCG 100
<210> 12406
<211> 100
<212> DNA

<213> Escherichia coli K-12 MG1655
<220> rect b1350
<222> 683-782
<223> rect b1350 U00096 complement(1412008..1412817)
<313> 12406: 718-737
<400> 12406
ATTTGCCCGT ATCAATTGAG ATCCAGCGTG CAGTATCAAT GGATGAAAAG  50
GAACCACTGA CAATCGATCC TGCAGATTCC TCTGTATTAA CCGGGGAATA  100
<210> 12407
<211> 100
<212> DNA
<213> Escherichia coli K-12 MG1655
<220> rect b1350
<222> 622-721
<223> rect b1350 U00096 complement(1412008..1412817)
<313> 12407: 646-665
<400> 12407
CCGTGGGTAA CTCACTGGGA AGAAATGGCA AAGAAAACGG CTATTCGTCG  50
CCTGTTCAAA TATTTGCCCG TATCAATTGA GATCCAGCGT GCAGTATCAA  100
<210> 12408
<211> 100
<212> DNA
<213> Escherichia coli K-12 MG1655
<220> rect b1350
<222> 591-690
<223> rect b1350 U00096 complement(1412008..1412817)
<313> 12408: 621-640
<400> 12408
GCGCAGCCTG AGTAAAGCTG GTAATAACGG GCCGTGGGTA ACTCACTGGG  50
AAGAAATGGC AAAGAAAACG GCTATTCGTC GCCTGTTCAA ATATTTGCCC  100
<210> 12409
<211> 100
<212> DNA
<213> Escherichia coli K-12 MG1655
<220> ygjd b3065
<222> 907-1006
<223> ygjd b3065 U00096 complement(3207171..3208184)
<313> 12409: 934-953
<400> 12409
GCGATGATCG CCTATGCCGG AATGGTGCGG TTTAAAGCAG GCGCGACGGC  50
GGATCTCGGC GTTAGCGTGC GTCCGCGCTG GCCGCTGGCG GAGTTACCGG  100
<210> 12410
<211> 100
<212> DNA
<213> Escherichia coli K-12 MG1655
<220> ygjd b3065
<222> 881-980
<223> ygjd b3065 U00096 complement(3207171..3208184)
<313> 12410: 901-920
<400> 12410
GTCCGGAATT TTGTACTGAT AACGGCGCGA TGATCGCCTA TGCCGGAATG  50
GTGCGGTTTA AAGCAGGCGC GACGGCGGAT CTCGGCGTTA GCGTGCGTCC  100
<210> 12411
<211> 100
<212> DNA
<213> Escherichia coli K-12 MG1655
<220> ygjd b3065
<222> 710-809
<223> ygjd b3065 U00096 complement(3207171..3208184)
<313> 12411: 754-773
<400> 12411
GCGCCTTTGA AGATGCGGTG GTCGATACGC TGATGATTAA GTGCAAGCGG  50
GCGCTGGATC AGACGGGCTT TAAGCGACTG GTCATGGCGG GCGGCGTGAG  100
<210> 12412
<211> 100
<212> DNA
<213> Escherichia coli K-12 MG1655
<220> ygje b3064
<222> 1344-1443
<223> ygje b3064 U00096 3205665..3207128
<313> 12412: 1404-1423

<400> 12412
CTACGGCAGT GGTTATCTGC CAACGGCGGA TTACTGGCGA CTGGGGGCGA 50
TTTTTGGGCT GATATTCCTC GTATTGCTGG TGATTACCGG CTTACTGTGG 100
<210> 12413
<211> 100
<212> DNA
<213> Escherichia coli K-12 MG1655
<220> ygje b3064
<222> 1319-1418
<223> ygje b3064 U00096 3205665..3207128
<313> 12413: 1344-1363
<400> 12413
ACGCCACCGG CCCCAGTCCG ATTTACTACG GCAGTGGTTA TCTGCCAACG 50
GCGGATTACT GGCGACTGGG GGCGATTTTT GGGCTGATAT TCCTCGTATT 100
<210> 12414
<211> 100
<212> DNA
<213> Escherichia coli K-12 MG1655
<220> ygje b3064
<222> 1274-1373
<223> ygje b3064 U00096 3205665..3207128
<313> 12414: 1294-1313
<400> 12414
TGATGGTTGG TGCGGCAATT GGTCTGGGGA GCATTCTTAC ACCATACGCC 50
ACCGGCCCCA GTCCGATTTA CTACGGCAGT GGTTATCTGC CAACGGCGGA 100
<210> 12415
<211> 100
<212> DNA
<213> Escherichia coli K-12 MG1655
<220> ygjf b3069
<222> 334-433
<223> ygjf b3069 U00096 complement(3212608..3213114)
<313> 12415: 367-386
<400> 12415
TATGAACAGG GATTCAGCCA GCGCGGTGCA CAGTGGGGGA AACAAACGCT 50
CACCATTGGT TCGACGCAGA TTTGGGTGCT GCCAAATCCC AGCGGTTTAA 100
<210> 12416
<211> 100
<212> DNA
<213> Escherichia coli K-12 MG1655
<220> ygjf b3069
<222> 289-388
<223> ygjf b3069 U00096 complement(3212608..3213114)
<313> 12416: 323-342
<400> 12416
GAAGATTATC AGCCGCAGGC GTTGGCGATT CTGGGCAAAC AAGCATATGA 50
ACAGGGATTC AGCCAGCGCG GTGCACAGTG GGGGAAACAA ACGCTCACCA 100
<210> 12417
<211> 100
<212> DNA
<213> Escherichia coli K-12 MG1655
<220> ygjf b3069
<222> 217-316
<223> ygjf b3069 U00096 complement(3212608..3213114)
<313> 12417: 264-283
<400> 12417
CCAACGGTGC AAGCCAATGA AGTTTCAAAG CAGGAGCTAC ACGCAGGCGG 50
GCGTAAGCTG ATTGAAAAAA TTGAAGATTA TCAGCCGCAG GCGTTGGCGA 100
<210> 12418
<211> 100
<212> DNA
<213> Escherichia coli K-12 MG1655
<220> ygjg b3074
<222> 1375-1474
<223> ygjg b3074 U00096 3217024..3218514
<313> 12418: 1416-1435
<400> 12418
GCCAAAACGA TCCGCATTGA ACCGCCACTG ACACTGACCA TTGAACAGTG 50
TGAACTGGTG ATCAAAGCGG CGCGTAAGGC GCTGGCGGCC ATGCGAGTAA 100
<210> 12419
<211> 100

<212> DNA
<213> Escherichia coli K-12 MG1655
<220> ygjg b3074
<222> 1356-1455
<223> ygjg b3074 U00096 3217024..3218514
<313> 12419: 1390-1409
<400> 12419
GGCCGGAACG CTCAATAACG CCAAAACGAT CCGCATTGAA CCGCCACTGA 50
CACTGACCAT TGAACAGTGT GAACTGGTGA TCAAAGCGGC GCGTAAGGCG 100
<210> 12420
<211> 100
<212> DNA
<213> Escherichia coli K-12 MG1655
<220> ygjg b3074
<222> 1296-1395
<223> ygjg b3074 U00096 3217024..3218514
<313> 12420: 1330-1349
<400> 12420
TGTTGATAAC GAAATCGGCT ATAACTTTGC CAGCGAGATG TTCCGCCAGC 50
GCGTACTGGT GGCCGGAACG CTCAATAACG CCAAAACGAT CCGCATTGAA 100
<210> 12421
<211> 100
<212> DNA
<213> Escherichia coli K-12 MG1655
<220> proa b0244
<222> 1139-1238
<223> proa b0244 U00096 260727..261980
<313> 12421: 1183-1202
<400> 12421
TGGGTGCGGA AGTGGCGGTA AGCACACAAA AACTCCACGC GCGTGGCCCA 50
ATGGGGCTGG AAGCACTGAC CACTTACAAG TGGATCGGCA TTGGTGATTA 100
<210> 12422
<211> 100
<212> DNA
<213> Escherichia coli K-12 MG1655
<220> proa b0244
<222> 1109-1208
<223> proa b0244 U00096 260727..261980
<313> 12422: 1151-1170
<400> 12422
CGCGTTTTAC CGACGGCGGC CAGTTTGGTC TGGGTGCGGA AGTGGCGGTA 50
AGCACACAAA AACTCCACGC GCGTGGCCCA ATGGGGCTGG AAGCACTGAC 100
<210> 12423
<211> 100
<212> DNA
<213> Escherichia coli K-12 MG1655
<220> proa b0244
<222> 963-1062
<223> proa b0244 U00096 260727..261980
<313> 12423: 1017-1036
<400> 12423
AATCGTCAGC GATCTTGACG ATGCCATCGC CCATATTCGT GAACACGGCA 50
CACAACACTC CGATGCGATC CTGACCCGCG ATATGCGCAA CGCCCAGCGT 100
<210> 12424
<211> 100
<212> DNA
<213> Escherichia coli K-12 MG1655
<220> ygjh b3075
<222> 158-257
<223> ygjh b3075 U00096 complement(3218556..3218888)
<313> 12424: 212-231
<400> 12424
ACAGCGAAGA AGAGCTGATG GGGAAAACGG TGGTGGTCTT ATGCAATCTG 50
CAAAAAGCGA AAATGCGTGG TGAAACGTCG GAATGCATGC TGTTGTGTGC 100
<210> 12425
<211> 100
<212> DNA
<213> Escherichia coli K-12 MG1655
<220> ygjh b3075
<222> 149-248
<223> ygjh b3075 U00096 complement(3218556..3218888)

<313> 12425: 169-188
<400> 12425
TGCCGTACTA CAGCGAAGAA GAGCTGATGG GGAAAACGGT GGTGGTCTTA 50
TGCAATCTGC AAAAAGCGAA AATGCGTGGT GAAACGTCGG AATGCATGCT 100
<210> 12426
<211> 100
<212> DNA
<213> Escherichia coli K-12 MG1655
<220> ygjh b3075
<222> 102-201
<223> ygjh b3075 U00096 complement(3218556..3218888)
<313> 12426: 113-132
<400> 12426
ACAGGTTGAT GTGGGGCAAA AAACGCTGCA AACCGTGACC AGCCTGGTGC 50
CGTACTACAG CGAAGAAGAG CTGATGGGGA AAACGGTGGT GGTCTTATGC 100
<210> 12427
<211> 100
<212> DNA
<213> Escherichia coli K-12 MG1655
<220> prob b0243
<222> 1005-1104
<223> prob b0243 U00096 259612..260715
<313> 12427: 1081-1100
<400> 12427
ACGCCGTATT GCCGGACACC ACTCGCAAGA AATTGATGCA ATACTGGGAT 50
ATGAATACGG CCCGGTTGCC GTTCACCGTG ATGACATGAT TACCCGTTAA 100
<210> 12428
<211> 100
<212> DNA
<213> Escherichia coli K-12 MG1655
<220> prob b0243
<222> 975-1074
<223> prob b0243 U00096 259612..260715
<313> 12428: 1018-1037
<400> 12428
CGGCGTCAGT CGTTACAACA GCGATGCATT ACGCCGTATT GCCGGACACC 50
ACTCGCAAGA AATTGATGCA ATACTGGGAT ATGAATACGG CCCGGTTGCC 100
<210> 12429
<211> 100
<212> DNA
<213> Escherichia coli K-12 MG1655
<220> prob b0243
<222> 891-990
<223> prob b0243 U00096 259612..260715
<313> 12429: 942-961
<400> 12429
GAAAGGCATT AAAAGCGTGA CTGGCAATTT CTCGCGTGGT GAAGTCATCC 50
GCATTTGCAA CCTCGAAGGC CGCGATATCG CCCACGGCGT CAGTCGTTAC 100
<210> 12430
<211> 100
<212> DNA
<213> Escherichia coli K-12 MG1655
<220> ygji b3079
<222> 1335-1434
<223> ygji b3079 U00096 3223875..3225308
<313> 12430: 1398-1417
<400> 12430
CTTCCTTGGC TTTGCGTGGT GGAAATACAG TAAATATATA AAGGGATTAA 50
CGGCTGAAGA GCGCCATATT GAAGCGACGC CAGCCAGCAA TGTTGATTAA 100
<210> 12431
<211> 100
<212> DNA
<213> Escherichia coli K-12 MG1655
<220> ygji b3079
<222> 1229-1328
<223> ygji b3079 U00096 3223875..3225308
<313> 12431: 1265-1284
<400> 12431
TTTCAATGCT GATTGCGATA TTTGCCGTAG GGTTTGTCGC TTCGACATTC 50
CCGACTGGCG CGAATATTCT GACCATCATT TTTTATAACG TCGGCGGTAT 100
<210> 12432

<211> 100
<212> DNA
<213> Escherichia coli K-12 MG1655
<220> ygji b3079
<222> 1146-1245
<223> ygji b3079 U00096 3223875..3225308
<313> 12432: 1175-1194
<400> 12432
GGCTTACCTG AATTTACGCG CCAAATTAGA TCACCTGCCA CGCGATTTCC 50
GTATGGGCTC CCGCCGCACC GGTATTATCG TTGTTTCAAT GCTGATTGCG 100
<210> 12433
<211> 100
<212> DNA
<213> Escherichia coli K-12 MG1655
<220> proc b0387
<222> 658-757
<223> proc b0387 U00096 complement(404059..404868)
<313> 12433: 694-713
<400> 12433
GAACATCCGG GGGCACTGAA AGATATGGTC TGCTCACCGG GAGGCACCAC 50
CATTGAAGCG GTACGCGTAC TGGAAGAGAA AGGCTTCCGT GCTGCAGTGA 100
<210> 12434
<211> 100
<212> DNA
<213> Escherichia coli K-12 MG1655
<220> proc b0387
<222> 597-696
<223> proc b0387 U00096 complement(404059..404868)
<313> 12434: 656-675
<400> 12434
GGCGTATAAA TTTGCCGCTC AGGCGGTAAT GGGTTCCGCA AAAATGGTGC 50
TGGAAACGGG AGAACATCCG GGGGCACTGA AAGATATGGT CTGCTCACCG 100
<210> 12435
<211> 100
<212> DNA
<213> Escherichia coli K-12 MG1655
<220> proc b0387
<222> 546-645
<223> proc b0387 U00096 complement(404059..404868)
<313> 12435: 582-601
<400> 12435
TATCGAAGCG ATGGCCGACG CCGCCGTGCT GGGCGGGATG CCACGCGCCC 50
AGGCGTATAA ATTTGCCGCT CAGGCGGTAA TGGGTTCCGC AAAAATGGTG 100
<210> 12436
<211> 100
<212> DNA
<213> Escherichia coli K-12 MG1655
<220> rlua
<222> 561-660
<223> rlua
<313> 12436: 636-655
<400> 12436
AGCGATGGCA CCACGTTTGT TGCTACATGC AGAGATGTTG ACGATTACCC 50
ATCCGGCGTA TGGCAATAGT ATGACGTTTA AAGCGCCAGC GGATTTTTAA 100
<210> 12437
<211> 100
<212> DNA
<213> Escherichia coli K-12 MG1655
<220> rlua
<222> 546-645
<223> rlua
<313> 12437: 584-603
<400> 12437
ATCACCAGAA GCGAGAGCGA TGGCACCACG TTTGTTGCTA CATGCAGAGA 50
TGTTGACGAT TACCCATCCG GCGTATGGCA ATAGTATGAC GTTTAAAGCG 100
<210> 12438
<211> 100
<212> DNA
<213> Escherichia coli K-12 MG1655
<220> rlua
<222> 519-618

<223> rlua
<313> 12438: 545-564
<400> 12438
TCCGATTCTC GGCGATCGTT TTTATGCATC ACCAGAAGCG AGAGCGATGG 50
CACCACGTTT GTTGCTACAT GCAGAGATGT TGACGATTAC CCATCCGGCG 100
<210> 12439
<211> 100
<212> DNA
<213> Escherichia coli K-12 MG1655
<220> ygjj b3080
<222> 972-1071
<223> ygjj b3080 U00096 3225442..3226512
<313> 12439: 1032-1051
<400> 12439
CGGACGTTTC GCCACGGCTT TTGATAGCCG CTTCTACATG AAAAATGCCT 50
CTCACGATCC CAGCGACCAA ATCTTCCTGA TGCAATATTT CTACTGGTAA 100
<210> 12440
<211> 100
<212> DNA
<213> Escherichia coli K-12 MG1655
<220> ygjj b3080
<222> 961-1060
<223> ygjj b3080 U00096 3225442..3226512
<313> 12440: 988-1007
<400> 12440
GAATACGGCG GCGGACGTTT CGCCACGGCT TTTGATAGCC GCTTCTACAT 50
GAAAAATGCC TCTCACGATC CCAGCGACCA AATCTTCCTG ATGCAATATT 100
<210> 12441
<211> 100
<212> DNA
<213> Escherichia coli K-12 MG1655
<220> ygjj b3080
<222> 915-1014
<223> ygjj b3080 U00096 3225442..3226512
<313> 12441: 955-974
<400> 12441
CGCTGACGAT GAAACCACCT CTTTCATGCA GTTTGGTATC TGGTACGAAT 50
ACGGCGGCGG ACGTTTCGCC ACGGCTTTTG ATAGCCGCTT CTACATGAAA 100
<210> 12442
<211> 100
<212> DNA
<213> Escherichia coli K-12 MG1655
<220> ygjk b3081
<222> 2253-2352
<223> ygjk b3081 U00096 3226529..3228880
<313> 12442: 2304-2323
<400> 12442
TTACAACCCG CTGACAGGCG CACAGCAAGG CGCACCAAAT TTCTCCTGGA 50
GTGCCGCGCA TTTGTATATG TTGTATAACG ATTTTTTCCG TAAGCAATAA 100
<210> 12443
<211> 100
<212> DNA
<213> Escherichia coli K-12 MG1655
<220> ygjk b3081
<222> 2218-2317
<223> ygjk b3081 U00096 3226529..3228880
<313> 12443: 2250-2269
<400> 12443
AAAGGGTTAA CCGCCGATGG ACCAATTCAG GAAAATTACA ACCCGCTGAC 50
AGGCGCACAG CAAGGCGCAC CAAATTTCTC CTGGAGTGCC GCGCATTTGT 100
<210> 12444
<211> 100
<212> DNA
<213> Escherichia coli K-12 MG1655
<220> ygjk b3081
<222> 2150-2249
<223> ygjk b3081 U00096 3226529..3228880
<313> 12444: 2195-2214
<400> 12444
AAGGGATGGA GCGTTACGGT TATCGTGATG ATGCCCTGAA GCTGGCGGAT 50
ACGTTCTTCC GGCACGCCAA AGGGTTAACC GCCGATGGAC CAATTCAGGA 100

<210> 12445
<211> 100
<212> DNA
<213> Escherichia coli K-12 MG1655
<220> ygjl b3082
<222> 1878-1977
<223> ygjl b3082 U00096 3229306..3231324
<313> 12445: 1910-1929
<400> 12445
GCAAGAGCCA AACCGCGCGC TGGCGCAACC GCTGATTGAT AGCGGGAAAA 50
CGGTGCATTT AATTGGCGGC TGCGATGTGG CTATGGAGCT GGACGCACGA 100
<210> 12446
<211> 100
<212> DNA
<213> Escherichia coli K-12 MG1655
<220> ygjl b3082
<222> 1807-1906
<223> ygjl b3082 U00096 3229306..3231324
<313> 12446: 1834-1853
<400> 12446
GACGGGCTGC ATGTGGTGAT CAACGGCGAA ACGCAGGTAT TAGCAGTGGA 50
CAATGTGGTG ATCTGCGCAG GGCAAGAGCC AAACCGCGCG CTGGCGCAAC 100
<210> 12447
<211> 100
<212> DNA
<213> Escherichia coli K-12 MG1655
<220> ygjl b3082
<222> 1759-1858
<223> ygjl b3082 U00096 3229306..3231324
<313> 12447: 1796-1815
<400> 12447
CGGGGTGTGA AAATGATCCC AGGCGTAAGT TATCAGAAGA TTGACGATGA 50
CGGGCTGCAT GTGGTGATCA ACGGCGAAAC GCAGGTATTA GCAGTGGACA 100
<210> 12448
<211> 100
<212> DNA
<213> Escherichia coli K-12 MG1655
<220> ygjm b3083
<222> 318-417
<223> ygjm b3083 U00096 complement(3231369..3231785)
<313> 12448: 396-415
<400> 12448
GGTGTCACGC GTTTTGAGCG GGAAGAGGAA ATTAACGCTG GAACACGCTA 50
AAAAATTGGC AACGCGATTC GGCATTTCTC CCGCCTTGTT TATTGATTAA 100
<210> 12449
<211> 100
<212> DNA
<213> Escherichia coli K-12 MG1655
<220> ygjm b3083
<222> 295-394
<223> ygjm b3083 U00096 complement(3231369..3231785)
<313> 12449: 344-363
<400> 12449
CCGGAAATTG GCAGTAAATC TATGGTGTCA CGCGTTTTGA GCGGGAAGAG 50
GAAATTAACG CTGGAACACG CTAAAAAATT GGCAACGCGA TTCGGCATTT 100
<210> 12450
<211> 100
<212> DNA
<213> Escherichia coli K-12 MG1655
<220> ygjm b3083
<222> 198-297
<223> ygjm b3083 U00096 complement(3231369..3231785)
<313> 12450: 226-245
<400> 12450
ATTTGCGGAA TTTAATGCCA TGGCTCAAGC CATGCCTGGC GGTATAGCCG 50
TGATTCGTAC CCTTATGGAT CAATATGGTT TAACCCTTTC CGATCTGCCG 100
<210> 12451
<211> 100
<212> DNA
<213> Escherichia coli K-12 MG1655
<220> ygjn b3084

<222> 216-315
<223> ygjn b3084 U00096 complement(3231782..3232096)
<313> 12451: 286-305
<400> 12451
GGTCTTTTTT GAATCGCAAA AGTGCTACAT ACGTGAAGTT ATGACGCATA 50
AAGAATACGA TTTCTTTACC GCTGTTCATC GTACTAAGGG GAAAAAATGA 100
<210> 12452
<211> 100
<212> DNA
<213> Escherichia coli K-12 MG1655
<220> ygjn b3084
<222> 185-284
<223> ygjn b3084 U00096 complement(3231782..3232096)
<313> 12452: 226-245
<400> 12452
TTGGGGGCAA TGAATTACGT GTTGTAGCAA TGGTCTTTTT TGAATCGCAA 50
AAGTGCTACA TACGTGAAGT TATGACGCAT AAAGAATACG ATTTCTTTAC 100
<210> 12453
<211> 100
<212> DNA
<213> Escherichia coli K-12 MG1655
<220> ygjn b3084
<222> 69-168
<223> ygjn b3084 U00096 complement(3231782..3232096)
<313> 12453: 135-154
<400> 12453
GGTGGCTCTG GGGAACACGA TTGCTAAGGG ATATTTCAAA AAACCTGAGT 50
CATTAAAAGC AGTATTCCCA TCTCTGGATA ACTTCAAATA TCTGGATAAG 100
<210> 12454
<211> 100
<212> DNA
<213> Escherichia coli K-12 MG1655
<220> ygjo b3085
<222> 1027-1126
<223> ygjo b3085 U00096 complement(3232380..3233546)
<313> 12454: 1056-1075
<400> 12454
AACGGCGAGC TGTATATCGT TGCCAACCGT CACCTGGATT ACTTCCATAA 50
ACTGAAGAAG ATTTTCGGCA ACTGCACCAC TATTGCGACG AATAATAAAT 100
<210> 12455
<211> 100
<212> DNA
<213> Escherichia coli K-12 MG1655
<220> ygjo b3085
<222> 980-1079
<223> ygjo b3085 U00096 complement(3232380..3233546)
<313> 12455: 993-1012
<400> 12455
ACGTCGCCTG GGAGATGTTC CACCACGCCC GCCGCTGCCT GAAAATCAAC 50
GGCGAGCTGT ATATCGTTGC CAACCGTCAC CTGGATTACT TCCATAAACT 100
<210> 12456
<211> 100
<212> DNA
<213> Escherichia coli K-12 MG1655
<220> ygjo b3085
<222> 921-1020
<223> ygjo b3085 U00096 complement(3232380..3233546)
<313> 12456: 966-985
<400> 12456
CCGCTTTAAT GCTGTGCTCT GCAACCCGCC GTTTCACCAA CAACATGCGC 50
TGACCGATAA CGTCGCCTGG GAGATGTTCC ACCACGCCCG CCGCTGCCTG 100
<210> 12457
<211> 100
<212> DNA
<213> Escherichia coli K-12 MG1655
<220> ygjp b3086
<222> 359-458
<223> ygjp b3086 U00096 3233565..3234104
<313> 12457: 400-419
<400> 12457
GCAACGCGCC GGAACCGTTT TTGCGCATGA TCGTGGTGCA CGAGCTGGCG 50

CACCTGAAGG AGAAAGAGCA TAACAAAGCG TTTTACCAGT TGTGTTGTCA 100
<210> 12458
<211> 100
<212> DNA
<213> Escherichia coli K-12 MG1655
<220> ygjp b3086
<222> 292-391
<223> ygjp b3086 U00096 3233565..3234104
<313> 12458: 350-369
<400> 12458
GCTGTTTCTC GTGTGCAGGG CGGCAAGCTG AAAGCAAAGG TGGAGATCCG 50
CGTCGCTACC GTGTTTCGCA ACGCGCCGGA ACCGTTTTTG CGCATGATCG 100
<210> 12459
<211> 100
<212> DNA
<213> Escherichia coli K-12 MG1655
<220> ygjp b3086
<222> 262-361
<223> ygjp b3086 U00096 3233565..3234104
<313> 12459: 305-324
<400> 12459
GTGCTAAAAA ACGCGCTCGG GTTACATACC GCTGTTTCTC GTGTGCAGGG 50
CGGCAAGCTG AAAGCAAAGG TGGAGATCCG CGTCGCTACC GTGTTTCGCA 100
<210> 12460
<211> 100
<212> DNA
<213> Escherichia coli K-12 MG1655
<220> ygjq b3087
<222> 594-693
<223> ygjq b3087 U00096 3234181..3234873
<313> 12460: 669-688
<400> 12460
CGTGATTGAC GCCAAAATCC TCCATCGCCA GCCGAAATAT CTCGGGCCTT 50
CGGTCATGAT TGGGCCGTTT AGTGAGCATG GCTGTCCGGC GCAGAAATAA 100
<210> 12461
<211> 100
<212> DNA
<213> Escherichia coli K-12 MG1655
<220> ygjq b3087
<222> 563-662
<223> ygjq b3087 U00096 3234181..3234873
<313> 12461: 617-636
<400> 12461
AACTGCGGGA GAAACTCGCT CGTGTCAGCG CCGTGATTGA CGCCAAAATC 50
CTCCATCGCC AGCCGAAATA TCTCGGGCCT TCGGTCATGA TTGGGCCGTT 100
<210> 12462
<211> 100
<212> DNA
<213> Escherichia coli K-12 MG1655
<220> ygjq b3087
<222> 549-648
<223> ygjq b3087 U00096 3234181..3234873
<313> 12462: 567-586
<400> 12462
CGGTTTTTAT ACTCAACTGC GGGAGAAACT CGCTCGTGTC AGCGCCGTGA 50
TTGACGCCAA AATCCTCCAT CGCCAGCCGA AATATCTCGG GCCTTCGGTC 100
<210> 12463
<211> 100
<212> DNA
<213> Escherichia coli K-12 MG1655
<220> ygjr b3088
<222> 906-1005
<223> ygjr b3088 U00096 3234934..3235938
<313> 12463: 978-997
<400> 12463
TCATCCAGGG CTGGCGGTCA GTCGCATCAC CGCCAAACTG CTGACCGAGA 50
TCCGCCGCCA GACTGGGGTG ATTTTTCCGG CAGATAGCGT AAAACTATAA 100
<210> 12464
<211> 100
<212> DNA
<213> Escherichia coli K-12 MG1655

<220> ygjr b3088
<222> 897-996
<223> ygjr b3088 U00096 3234934..3235938
<313> 12464: 938-957
<400> 12464
TCTGGTGGAT CATCCAGGGC TGGCGGTCAG TCGCATCACC GCCAAACTGC 50
TGACCGAGAT CCGCCGCCAG ACTGGGGTGA TTTTTCCGGC AGATAGCGTA 100
<210> 12465
<211> 100
<212> DNA
<213> Escherichia coli K-12 MG1655
<220> ygjr b3088
<222> 805-904
<223> ygjr b3088 U00096 3234934..3235938
<313> 12465: 856-875
<400> 12465
CGTGGCAGCC AAATGCAGGA TCTCACCCAG CCGCAGCATA TTAATACCAT 50
GCTCTACGAA GCAGAGCTGT TCGCTACCCT GGTGGATGAG CATCTGGTGG 100
<210> 12466
<211> 100
<212> DNA
<213> Escherichia coli K-12 MG1655
<220> ygjt b3089
<222> 827-926
<223> ygjt b3089 U00096 3236222..3237187
<313> 12466: 858-877
<400> 12466
TCGGTATCAA GATGCTGATT GTCGACTTCT ACCATATTCC AATCGCCGTC 50
TCGCTGGGCG TGGTGTTTGG CATTCTGGTG ATGACGTTTA TTATCAACGC 100
<210> 12467
<211> 100
<212> DNA
<213> Escherichia coli K-12 MG1655
<220> ygjt b3089
<222> 748-847
<223> ygjt b3089 U00096 3236222..3237187
<313> 12467: 808-827
<400> 12467
GCGATGTATT TCCTGCTGGC GGGCGTAGCA GAGCGTTTCT CGATGCTCAA 50
ATATGGCCTG GCGGTGATTC TGGTGTTTAT CGGTATCAAG ATGCTGATTG 100
<210> 12468
<211> 100
<212> DNA
<213> Escherichia coli K-12 MG1655
<220> ygjt b3089
<222> 679-778
<223> ygjt b3089 U00096 3236222..3237187
<313> 12468: 727-746
<400> 12468
ATCTTCGCCG TGACCACTGA CCCGTTCATT GTGCTGACCT CAAACCTGTT 50
TGCGATCCTC GGCCTGCGTG CGATGTATTT CCTGCTGGCG GGCGTAGCAG 100
<210> 12469
<211> 100
<212> DNA
<213> Escherichia coli K-12 MG1655
<220> ygju b3090
<222> 1137-1236
<223> ygju b3090 U00096 3237584..3238828
<313> 12469: 1194-1213
<400> 12469
GCAGGACTCT TGCGAAACCG CGCTGAACTC TTCAACTGAC GTGCTGTTCA 50
CTGCGGCAGC TTGCCAGGCA GAAGACGATC GTCTGGCAAA TAGCGCCCTG 100
<210> 12470
<211> 100
<212> DNA
<213> Escherichia coli K-12 MG1655
<220> ygju b3090
<222> 1064-1163
<223> ygju b3090 U00096 3237584..3238828
<313> 12470: 1128-1147
<400> 12470

CCTGTAATAT GTTCGGTATT TCGAACGATA TCGCCATGCA GGTGGTTGCC 50
GTCGGCTTTA TCATCGGCGT ATTGCAGGAC TCTTGCGAAA CCGCGCTGAA 100
<210> 12471
<211> 100
<212> DNA
<213> Escherichia coli K-12 MG1655
<220> ygju b3090
<222> 1001-1100
<223> ygju b3090 U00096 3237584..3238828
<313> 12471: 1061-1080
<400> 12471
CTTCTCTGTG TGCCTGTGGC GCATCCGGCG TGGCGGGGGG GTCTCTGCTG 50
CTGATCCCAC TGGCCTGTAA TATGTTCGGT ATTTCGAACG ATATCGCCAT 100
<210> 12472
<211> 100
<212> DNA
<213> Escherichia coli K-12 MG1655
<220> ygjv b3091
<222> 453-552
<223> ygjv b3091 U00096 complement(3238833..3239384)
<313> 12472: 514-533
<400> 12472
GAATGGCCTG AATATCATTC GTTTCTGGCG GATGCAGAAA AGGGGAATTG 50
ATCCGTTTAA AGTTGAGAAA ACCCCTTCCG CCGTAGACGA AAGGGGTTAA 100
<210> 12473
<211> 100
<212> DNA
<213> Escherichia coli K-12 MG1655
<220> ygjv b3091
<222> 391-490
<223> ygjv b3091 U00096 complement(3238833..3239384)
<313> 12473: 430-449
<400> 12473
ATTCACAACT TCTGGGCGGG GTCGATAGGC GGCACGATGA TTGAGGGTAG 50
TTTTCTGCTT ATGAATGGCC TGAATATCAT TCGTTTCTGG CGGATGCAGA 100
<210> 12474
<211> 100
<212> DNA
<213> Escherichia coli K-12 MG1655
<220> ygjv b3091
<222> 352-451
<223> ygjv b3091 U00096 complement(3238833..3239384)
<313> 12474: 398-417
<400> 12474
ATGCGCTGCG TAATGTGGTT TTCAACGTGT TGCTGGGTGA TTCACAACTT 50
CTGGGCGGGG TCGATAGGCG GCACGATGAT TGAGGGTAGT TTTCTGCTTA 100
<210> 12475
<211> 100
<212> DNA
<213> Escherichia coli K-12 MG1655
<220> prop b4112
<222> 1404-1503
<223> prop b4112 U00096 4328080..4329582
<313> 12475: 1482-1501
<400> 12475
TAATATCGAG CAGAAAATCG ATGATATTGA CCACGAGATT GCCGATTTGC 50
AGGCGAAACG TACCCGCCTG GTGCAGCAAC ATCCGCGAAT TGATGAATAA 100
<210> 12476
<211> 100
<212> DNA
<213> Escherichia coli K-12 MG1655
<220> prop b4112
<222> 1376-1475
<223> prop b4112 U00096 4328080..4329582
<313> 12476: 1442-1461
<400> 12476
CGAAGGAAAT TCTCGTCGAG CATTACGATA ATATCGAGCA GAAAATCGAT 50
GATATTGACC ACGAGATTGC CGATTTGCAG GCGAAACGTA CCCGCCTGGT 100
<210> 12477
<211> 100
<212> DNA

<213> Escherichia coli K-12 MG1655
<220> prop b4112
<222> 1319-1418
<223> prop b4112 U00096 4328080..4329582
<313> 12477: 1364-1383
<400> 12477
AGACGGCAAA TCGTCCGTTG AAAGGTGCGA CACCGGCGGC GTCAGATATA 50
CAGGAAGCGA AGGAAATTCT CGTCGAGCAT TACGATAATA TCGAGCAGAA 100
<210> 12478
<211> 100
<212> DNA
<213> Escherichia coli K-12 MG1655
<220> pcnb b0144
<222> 1266-1365
<223> pcnb b0144 U00096 complement(157729..159093)
<313> 12478: 1326-1345
<400> 12478
CCAAAAAGGG ATGCTCAACG AGCTGGATGA AGAACCGTCA CCGCGTCGTC 50
GTACTCGTCG TCCACGCAAA CGCGCACCAC GTCGTGAGGG TACCGCATGA 100
<210> 12479
<211> 100
<212> DNA
<213> Escherichia coli K-12 MG1655
<220> pcnb b0144
<222> 1257-1356
<223> pcnb b0144 U00096 complement(157729..159093)
<313> 12479: 1294-1313
<400> 12479
GCCACCAGAC CAAAAAGGGA TGCTCAACGA GCTGGATGAA GAACCGTCAC 50
CGCGTCGTCG TACTCGTCGT CCACGCAAAC GCGCACCACG TCGTGAGGGT 100
<210> 12480
<211> 100
<212> DNA
<213> Escherichia coli K-12 MG1655
<220> pcnb b0144
<222> 1208-1307
<223> pcnb b0144 U00096 complement(157729..159093)
<313> 12480: 1250-1269
<400> 12480
CTGAACTGCA GCGTCTGGTG AAATGGTGGG GTGAGTTCCA GGTTTCCGCG 50
CCACCAGACC AAAAAGGGAT GCTCAACGAG CTGGATGAAG AACCGTCACC 100
<210> 12481
<211> 100
<212> DNA
<213> Escherichia coli K-12 MG1655
<220> pros b0195
<222> 1551-1650
<223> pros b0195 U00096 complement(217057..218775)
<313> 12481: 1600-1619
<400> 12481
CGTGATGTTT GCTGATATGG AACTGATCGG TATTCCGCAC ACTATTGTGC 50
TGGGCGACCG TAACCTCGAC AACGACGATA TCGAATATAA ATATCGTCGC 100
<210> 12482
<211> 100
<212> DNA
<213> Escherichia coli K-12 MG1655
<220> pros b0195
<222> 1458-1557
<223> pros b0195 U00096 complement(217057..218775)
<313> 12482: 1523-1542
<400> 12482
CTTCCGCGTA CAAGAGCTTG CTGAGAAACT GTACAGCGAA CTGCGTGCAC 50
AAGGTATCGA AGTGCTGCTG GATGACCGCA AAGAGCGTCC GGGCGTGATG 100
<210> 12483
<211> 100
<212> DNA
<213> Escherichia coli K-12 MG1655
<220> pros b0195
<222> 1410-1509
<223> pros b0195 U00096 complement(217057..218775)
<313> 12483: 1460-1479

<400> 12483
TATCGCGCCG TTCCAGGTGG CGATTCTGCC GATGAACATG CACAAATCCT 50
TCCGCGTACA AGAGCTTGCT GAGAAACTGT ACAGCGAACT GCGTGCACAA 100
<210> 12484
<211> 100
<212> DNA
<213> Escherichia coli K-12 MG1655
<220> prov b2678
<222> 1047-1146
<223> prov b2678 U00096 2802836..2804038
<313> 12484: 1070-1089
<400> 12484
AGTCGATGCA CAAACGCCTC TTAGCGAGTT GCTCTCTCAT GTCGGACAGG 50
CACCCTGTGC GGTGCCCGTG GTCGACGAGG ACCAACAGTA TGTCGGCATC 100
<210> 12485
<211> 100
<212> DNA
<213> Escherichia coli K-12 MG1655
<220> prov b2678
<222> 988-1087
<223> prov b2678 U00096 2802836..2804038
<313> 12485: 1028-1047
<400> 12485
AAAACCGCGT TAACGCAGCA GCAAGGTCTT GATGCGGCGC TGATTGATGC 50
GCCGTTAGCA GTCGATGCAC AAACGCCTCT TAGCGAGTTG CTCTCTCATG 100
<210> 12486
<211> 100
<212> DNA
<213> Escherichia coli K-12 MG1655
<220> prov b2678
<222> 915-1014
<223> prov b2678 U00096 2802836..2804038
<313> 12486: 981-1000
<400> 12486
TGAAGATCGC GAATATGGCT ACGTTATCGA ACGCGGTAAT AAGTTTGTCG 50
GCGCAGTCTC CATCGATTCG CTTAAAACCG CGTTAACGCA GCAGCAAGGT 100
<210> 12487
<211> 100
<212> DNA
<213> Escherichia coli K-12 MG1655
<220> prow b2679
<222> 966-1065
<223> prow b2679 U00096 2804031..2805095
<313> 12487: 1032-1051
<400> 12487
TCTGACGCAG GCCGTTGGGC GCGACTCACG CAGTCGCGGC AACCGTCGCT 50
GGTACACCAC TGGCCCTGTT GGTCTGCTGA CCCGCCCATT CATTAAGTAA 100
<210> 12488
<211> 100
<212> DNA
<213> Escherichia coli K-12 MG1655
<220> prow b2679
<222> 767-866
<223> prow b2679 U00096 2804031..2805095
<313> 12488: 807-826
<400> 12488
AGTTACCGCT GGCGATGCCG ACCATTATGG CGGGCGTTAA CCAGACGCTG 50
ATGCTGGCCC TTTCTATGGT GGTCATCGCC TCGATGATTG CCGTCGGCGG 100
<210> 12489
<211> 100
<212> DNA
<213> Escherichia coli K-12 MG1655
<220> prow b2679
<222> 698-797
<223> prow b2679 U00096 2804031..2805095
<313> 12489: 753-772
<400> 12489
TTCCGGCGGA TCTGATTGAA GCCTCGCGCT CATTCGGTGC CAGCCCGCGC 50
CAGATGCTGT TCAAAGTTCA GTTACCGCTG GCGATGCCGA CCATTATGGC 100
<210> 12490
<211> 100

<212> DNA
<213> Escherichia coli K-12 MG1655
<220> prox proX
<222> 894-993
<223> prox proX U00096 2805153..2806145
<313> 12490: 947-966
<400> 12490
CTCAGAAGGC GATATTCAGG GACACGTTGA TGGTTGGATC AAAGCCCACC 50
AGCAGCAGTT CGATGGCTGG GTGAATGAGG CGCTGGCAGC GCAGAAGTAA 100
<210> 12491
<211> 100
<212> DNA
<213> Escherichia coli K-12 MG1655
<220> prox proX
<222> 882-981
<223> prox proX U00096 2805153..2806145
<313> 12491: 909-928
<400> 12491
TGACGGCAAA GCCTCAGAAG GCGATATTCA GGGACACGTT GATGGTTGGA 50
TCAAAGCCCA CCAGCAGCAG TTCGATGGCT GGGTGAATGA GGCGCTGGCA 100
<210> 12492
<211> 100
<212> DNA
<213> Escherichia coli K-12 MG1655
<220> prox proX
<222> 817-916
<223> prox proX U00096 2805153..2806145
<313> 12492: 849-868
<400> 12492
GCGAAACTGT TTGCCATTAT GCAGTTGCCA GTGGCAGATA TTAACGCCCA 50
GAACGCCATT ATGCATGACG GCAAAGCCTC AGAAGGCGAT ATTCAGGGAC 100
<210> 12493
<211> 100
<212> DNA
<213> Escherichia coli K-12 MG1655
<220> proy b0403
<222> 1173-1272
<223> proy b0403 U00096 420210..421583
<313> 12493: 1232-1251
<400> 12493
TAAGGCGCTG AAATTTAAAG TGCCGGGTGG GGTAGCAACG ACCATCGGCG 50
GGCTGATTTT CCTGCTCTTT ATTATCGGGT TGATTGGTTA TCACCCGGAT 100
<210> 12494
<211> 100
<212> DNA
<213> Escherichia coli K-12 MG1655
<220> proy b0403
<222> 1167-1266
<223> proy b0403 U00096 420210..421583
<313> 12494: 1192-1211
<400> 12494
AGAAGTTAAG GCGCTGAAAT TTAAAGTGCC GGGTGGGGTA GCAACGACCA 50
TCGGCGGGCT GATTTTCCTG CTCTTTATTA TCGGGTTGAT TGGTTATCAC 100
<210> 12495
<211> 100
<212> DNA
<213> Escherichia coli K-12 MG1655
<220> proy b0403
<222> 1122-1221
<223> proy b0403 U00096 420210..421583
<313> 12495: 1153-1172
<400> 12495
GATCCTGCTG TCGCAAATTG CCTTCCGTCG CCGTTTGCCG CCAGAAGAAG 50
TTAAGGCGCT GAAATTTAAA GTGCCGGGTG GGGTAGCAAC GACCATCGGC 100
<210> 12496
<211> 100
<212> DNA
<213> Escherichia coli K-12 MG1655
<220> hdea b3511
<222> 170-269
<223> hdea b3511 U00096 complement(3654038..3654370)

<313> 12496: 228-247
<400> 12496
CTGAAGCGCT GAACAACAAA GATAAACCAG AAGATGCGGT TTTAGATGTT 50
CAGGGTATTG CAACCGTAAC CCCAGCTATC GTTCAGGCTT GTACTCAGGA 100
<210> 12497
<211> 100
<212> DNA
<213> Escherichia coli K-12 MG1655
<220> hdea b3511
<222> 93-192
<223> hdea b3511 U00096 complement(3654038..3654370)
<313> 12497: 129-148
<400> 12497
AAAACCGGTC AACTCCTGGA CCTGTGAAGA TTTCCTGGCT GTGGACGAAT 50
CCTTCCAGCC AACTGCAGTT GGTTTTGCTG AAGCGCTGAA CAACAAAGAT 100
<210> 12498
<211> 100
<212> DNA
<213> Escherichia coli K-12 MG1655
<220> hdea b3511
<222> 10-109
<223> hdea b3511 U00096 complement(3654038..3654370)
<313> 12498: 41-60
<400> 12498
GTATTAGGCG TTATTCTTGG TGGTCTGCTT CTTCTGCCAG TTGTGAGCAA 50
TGCAGCGGAT GCGCAAAAAG CAGCTGATAA CAAAAAACCG GTCAACTCCT 100
<210> 12499
<211> 100
<212> DNA
<213> Escherichia coli K-12 MG1655
<220> hdeb b3510
<222> 240-339
<223> hdeb b3510 U00096 complement(3653596..3653934)
<313> 12499: 303-322
<400> 12499
TCTCACTCAA ATTCCTAAAG TGATCGAATA CTGTAAGAAA AACCCGCAGA 50
AAAATTTGTA TACCTTCAAA AATCAAGCAT CTAATGACTT GCCGAATTAA 100
<210> 12500
<211> 100
<212> DNA
<213> Escherichia coli K-12 MG1655
<220> hdeb b3510
<222> 239-338
<223> hdeb b3510 U00096 complement(3653596..3653934)
<313> 12500: 294-313
<400> 12500
ATCTCACTCA AATTCCTAAA GTGATCGAAT ACTGTAAGAA AAACCCGCAG 50
AAAAATTTGT ATACCTTCAA AAATCAAGCA TCTAATGACT TGCCGAATTA 100
<210> 12501
<211> 100
<212> DNA
<213> Escherichia coli K-12 MG1655
<220> hdeb b3510
<222> 240-339
<223> hdeb b3510 U00096 complement(3653596..3653934)
<313> 12501: 290-309
<400> 12501
TCTCACTCAA ATTCCTAAAG TGATCGAATA CTGTAAGAAA AACCCGCAGA 50
AAAATTTGTA TACCTTCAAA AATCAAGCAT CTAATGACTT GCCGAATTAA 100
<210> 12502
<211> 100
<212> DNA
<213> Escherichia coli K-12 MG1655
<220> prpb prpB
<222> 792-891
<223> prpb prpB U00096 347906..348796
<313> 12502: 858-877
<400> 12502
CGACACCATG CAGACCCGCA ACGAGCTGTA CGAAAGCATC AACTACTACC 50
AGTACGAAGA GAAGCTCGAC AACCTGTTTG CCCGTAGCCA GGTGAAATAA 100
<210> 12503

<211> 100
<212> DNA
<213> Escherichia coli K-12 MG1655
<220> prpb prpB
<222> 764-863
<223> prpb prpB U00096 347906..348796
<313> 12503: 815-834
<400> 12503
GCCAGGAAGG CACGCAGAAA AGCGTCATCG ACACCATGCA GACCCGCAAC 50
GAGCTGTACG AAAGCATCAA CTACTACCAG TACGAAGAGA AGCTCGACAA 100
<210> 12504
<211> 100
<212> DNA
<213> Escherichia coli K-12 MG1655
<220> prpb prpB
<222> 731-830
<223> prpb prpB U00096 347906..348796
<313> 12504: 786-805
<400> 12504
ACCGCGCCGC TGAACATGTC TACAACGTCC TGCGCCAGGA AGGCACGCAG 50
AAAAGCGTCA TCGACACCAT GCAGACCCGC AACGAGCTGT ACGAAAGCAT 100
<210> 12505
<211> 100
<212> DNA
<213> Escherichia coli K-12 MG1655
<220> hded b3512
<222> 474-573
<223> hded b3512 U00096 3654625..3655197
<313> 12505: 536-555
<400> 12505
GGTGTCTGTA ACGCTGGTTT CCACACTGGT GGGAATTGAA CTGATATTTA 50
GCGCCGCCAG CCTGTTCAGC TTCGCCAGTT TGTTCGTTAA GCAGCAATAA 100
<210> 12506
<211> 100
<212> DNA
<213> Escherichia coli K-12 MG1655
<220> hded b3512
<222> 449-548
<223> hded b3512 U00096 3654625..3655197
<313> 12506: 464-483
<400> 12506
GGATATTCCT TGGTGCAACG CCGATGGTGT CTGTAACGCT GGTTTCCACA 50
CTGGTGGGAA TTGAACTGAT ATTTAGCGCC GCCAGCCTGT TCAGCTTCGC 100
<210> 12507
<211> 100
<212> DNA
<213> Escherichia coli K-12 MG1655
<220> hded b3512
<222> 329-428
<223> hded b3512 U00096 3654625..3655197
<313> 12507: 357-376
<400> 12507
TTATTGCCGG TTTGTTCTGT GTTGCAGGGG TTATTCGCCT GATGAGTTGG 50
TATCGTCAGC GTTCAATGAA AGGCAGCTGG CTACAGCTCG TTATTGGTGT 100
<210> 12508
<211> 100
<212> DNA
<213> Escherichia coli K-12 MG1655
<220> prpc b0334
<222> 1071-1170
<223> prpc b0334 U00096 349236..350405
<313> 12508: 1131-1150
<400> 12508
CATTATCGAA CAACGTCAGG ACAACAAAAT TATCCGTCCT TCCGCCAATT 50
ATGTTGGACC GGAAGACCGC CCGTTTGTCG CGCTGGATAA GCGCCAGTAA 100
<210> 12509
<211> 100
<212> DNA
<213> Escherichia coli K-12 MG1655
<220> prpc b0334
<222> 1037-1136

<223> prpc b0334 U00096 349236..350405
<313> 12509: 1089-1108
<400> 12509
TTGTTATCGC CCGCGTCACC GGCTGGGCGG CGCACATTAT CGAACAACGT 50
CAGGACAACA AAATTATCCG TCCTTCCGCC AATTATGTTG GACCGGAAGA 100
<210> 12510
<211> 100
<212> DNA
<213> Escherichia coli K-12 MG1655
<220> prpc b0334
<222> 978-1077
<223> prpc b0334 U00096 349236..350405
<313> 12510: 1033-1052
<400> 12510
GTTCTCTGCT GTTTCCTACA ACATGATGGG CGTTCCCACC GAGATGTTCA 50
CACCACTGTT TGTTATCGCC CGCGTCACCG GCTGGGCGGC GCACATTATC 100
<210> 12511
<211> 100
<212> DNA
<213> Escherichia coli K-12 MG1655
<220> prpd b0335
<222> 1353-1452
<223> prpd b0335 U00096 350439..351890
<313> 12511: 1421-1440
<400> 12511
CCCGACTCGC CAACAGCAGC GCATTCTGGA GGTTTCTCTC GACAGAGCTC 50
GCCTGGAACA GATGCCGGTC AATGAGTATC TCGACCTGTA CGTCATTTAA 100
<210> 12512
<211> 100
<212> DNA
<213> Escherichia coli K-12 MG1655
<220> prpd b0335
<222> 1333-1432
<223> prpd b0335 U00096 350439..351890
<313> 12512: 1361-1380
<400> 12512
ATCAATCTCG CGCGCCAGTT CCCGACTCGC CAACAGCAGC GCATTCTGGA 50
GGTTTCTCTC GACAGAGCTC GCCTGGAACA GATGCCGGTC AATGAGTATC 100
<210> 12513
<211> 100
<212> DNA
<213> Escherichia coli K-12 MG1655
<220> prpd b0335
<222> 1255-1354
<223> prpd b0335 U00096 350439..351890
<313> 12513: 1299-1318
<400> 12513
GAAGTGGTGG TGGAGTACCC CATTGGTCAT GCTCGCCGCC GTCAGGATGG 50
TATTCCGAAA CTGGTCGATA AATTCAAAAT CAATCTCGCG CGCCAGTTCC 100
<210> 12514
<211> 100
<212> DNA
<213> Escherichia coli K-12 MG1655
<220> prpe b0336
<222> 1788-1887
<223> prpe b0336 U00096 351930..353816
<313> 12514: 1864-1883
<400> 12514
CACGATCCAG GCGATTTGCG AAGGACGCGA TCCTGGAGAT CTGACGACCA 50
TTGATGATCC TGCGTCGTTG GATCAGATCC GCCAGGCGAT GGAAGAGTAG 100
<210> 12515
<211> 100
<212> DNA
<213> Escherichia coli K-12 MG1655
<220> prpe b0336
<222> 1697-1796
<223> prpe b0336 U00096 351930..353816
<313> 12515: 1719-1738
<400> 12515
TGGACAGCCA GATTGGCAAC TTTGGCCGCC CGGCGCACGT CTGGTTTGTC 50
TCGCAATTGC CAAAAACGCG ATCCGGAAAA ATGCTGCGCC GCACGATCCA 100

<210> 12516
<211> 100
<212> DNA
<213> Escherichia coli K-12 MG1655
<220> prpe b0336
<222> 1589-1688
<223> prpe b0336 U00096 351930..353816
<313> 12516: 1626-1645
<400> 12516
ATGCGCTGAA AGGGCAGGTG GCGGTGGCGT TTGTCATTCC GAAAGAGAGC 50
GACAGTCTGG AAGATCGTGA TGTGGCGCAC TCGCAAGAGA AGGCGATTAT 100
<210> 12517
<211> 100
<212> DNA
<213> Escherichia coli K-12 MG1655
<220> prpr b0331
<222> 1488-1587
<223> prpr b0331 U00096 complement(346081..347667)
<313> 12517: 1534-1553
<400> 12517
ACCACAACAG GCACTGGAGA AATTTAATGG CGATAAAACA GCAGCGGCGA 50
ATTATTTAGG CATCAGCCGG ACGACGTTCT GGCGGCGGCT GAAAAGCTGA 100
<210> 12518
<211> 100
<212> DNA
<213> Escherichia coli K-12 MG1655
<220> prpr b0331
<222> 1467-1566
<223> prpr b0331 U00096 complement(346081..347667)
<313> 12518: 1502-1521
<400> 12518
TCCCGCTCCA CGCTTACTGA CACCACAACA GGCACTGGAG AAATTTAATG 50
GCGATAAAAC AGCAGCGGCG AATTATTTAG GCATCAGCCG GACGACGTTC 100
<210> 12519
<211> 100
<212> DNA
<213> Escherichia coli K-12 MG1655
<220> prpr b0331
<222> 1372-1471
<223> prpr b0331 U00096 complement(346081..347667)
<313> 12519: 1402-1421
<400> 12519
CTGGCGCTGT TTTTAAGTGT GGAACCGACG CCGGATTTAA CGCCGCAGTT 50
TATGCAACTG CTACTGCCGG AACTGGCGCG CGAGTCGGCG AAAACTCCCG 100
<210> 12520
<211> 100
<212> DNA
<213> Escherichia coli K-12 MG1655
<220> gntk b3438
<222> 372-471
<223> gntk b3438 U00096 complement(3574697..3575185)
<313> 12520: 404-423
<400> 12520
GTTTGAAACG CTGCAGGAGC CGGGTGCGGA CGAAACCGAT GTACTGGTGG 50
TGGATATCGA TCAACCGCTG GAAGGTGTTG TGGCAAGCAC CATTGAGGTT 100
<210> 12521
<211> 100
<212> DNA
<213> Escherichia coli K-12 MG1655
<220> gntk b3438
<222> 333-432
<223> gntk b3438 U00096 complement(3574697..3575185)
<313> 12521: 364-383
<400> 12521
CAAAGGCCAT TTCTTTAAAA CCCAAATGTT GGTGACGCAG TTTGAAACGC 50
TGCAGGAGCC GGGTGCGGAC GAAACCGATG TACTGGTGGT GGATATCGAT 100
<210> 12522
<211> 100
<212> DNA
<213> Escherichia coli K-12 MG1655
<220> gntk b3438

<222> 253-352
<223> gntk b3438 U00096 complement(3574697..3575185)
<313> 12522: 308-327
<400> 12522
CTGCGTGAAG GTAATCCGAA TCTCTCTTTC ATCTATTTGA AAGGCGATTT 50
TGATGTGATT GAAAGCCGCC TGAAAGCGCG CAAAGGCCAT TTCTTTAAAA 100
<210> 12523
<211> 100
<212> DNA
<213> Escherichia coli K-12 MG1655
<220> gntp b4322
<222> 1245-1344
<223> gntp b4322 U00096 complement(4547522..4548865)
<313> 12523: 1310-1329
<400> 12523
CCTGTCAGTA AAAGACACGT TGAAAACCTG GGTCTGCTGG AGCTGGTCAA 50
ACTCCGTGGT TGGGCTGATT ATTGTGCTGA TTATTAGCAT GGTAGCGTAA 100
<210> 12524
<211> 100
<212> DNA
<213> Escherichia coli K-12 MG1655
<220> gntp b4322
<222> 1217-1316
<223> gntp b4322 U00096 complement(4547522..4548865)
<313> 12524: 1262-1281
<400> 12524
CTTTCTGGTT GTTCAAAGGT TACTTTGACC TGTCAGTAAA AGACACGTTG 50
AAAACCTGGG GTCTGCTGGA GCTGGTCAAC TCCGTGGTTG GGCTGATTAT 100
<210> 12525
<211> 100
<212> DNA
<213> Escherichia coli K-12 MG1655
<220> gntp b4322
<222> 1161-1260
<223> gntp b4322 U00096 complement(4547522..4548865)
<313> 12525: 1186-1205
<400> 12525
GCTGGTACTG GCGACGGCTG CGGGTTCCAA CACCCTCACC CACATTAACG 50
ATGCATCTTT CTGGTTGTTC AAAGGTTACT TTGACCTGTC AGTAAAAGAC 100
<210> 12526
<211> 100
<212> DNA
<213> Escherichia coli K-12 MG1655
<220> gntr b3439
<222> 727-826
<223> gntr b3439 U00096 complement(3575416..3576357)
<313> 12526: 785-804
<400> 12526
GTGTTCTGTA CGAATGATGA CCTGGCGGTC GGCGCGGCGT TTGAATGTCA 50
GCGTCTGGGG TTAAAAGTTC CTGACGATAT GGCGATTGCC GGTTTCCACG 100
<210> 12527
<211> 100
<212> DNA
<213> Escherichia coli K-12 MG1655
<220> gntr b3439
<222> 713-812
<223> gntr b3439 U00096 complement(3575416..3576357)
<313> 12527: 745-764
<400> 12527
CGCAGCTGGA TGGCGTGTTC TGTACGAATG ATGACCTGGC GGTCGGCGCG 50
GCGTTTGAAT GTCAGCGTCT GGGGTTAAAA GTTCCTGACG ATATGGCGAT 100
<210> 12528
<211> 100
<212> DNA
<213> Escherichia coli K-12 MG1655
<220> gntr b3439
<222> 661-760
<223> gntr b3439 U00096 complement(3575416..3576357)
<313> 12528: 688-707
<400> 12528
TCTTCTTACT CTTCCGGTAT TGAACTGATT CGCCAGGCGC GGCGGGAATA 50

TCCGCAGCTG GATGGCGTGT TCTGTACGAA TGATGACCTG GCGGTCGGCG 100
<210> 12529
<211> 100
<212> DNA
<213> Escherichia coli K-12 MG1655
<220> gntt b3416
<222> 1215-1314
<223> gntt b3416 U00096 3544195..3545508
<313> 12529: 1253-1272
<400> 12529
CCTGACTATC GGCGAGACCA TCAAATCCTG GTCGATGCTG GAAACCATTA 50
TCTCGGTGTG CGGTCTGGTA GGCTGTCTGC TGCTGAATAT GGTGATTTGA 100
<210> 12530
<211> 100
<212> DNA
<213> Escherichia coli K-12 MG1655
<220> gntt b3416
<222> 1102-1201
<223> gntt b3416 U00096 3544195..3545508
<313> 12530: 1151-1170
<400> 12530
ATTGCAACGA CGGGTGTTAG CCCGGAGCTG ATGGTTATTG CGGTTGGTTC 50
CGGTAGTGTG ATTTTCTCTC ATGTGAACGA TCCGGGCTTC TGGCTGTTCA 100
<210> 12531
<211> 100
<212> DNA
<213> Escherichia coli K-12 MG1655
<220> gntt b3416
<222> 1056-1155
<223> gntt b3416 U00096 3544195..3545508
<313> 12531: 1108-1127
<400> 12531
CGCAACCGTT GCGGCAATCA CTGCGGGTGG TATCGCGGCA CCGCTGATTG 50
CAACGACGGG TGTTAGCCCG GAGCTGATGG TTATTGCGGT TGGTTCCGGT 100
<210> 12532
<211> 100
<212> DNA
<213> Escherichia coli K-12 MG1655
<220> mog b0010
<222> 489-588
<223> mog b0010 U00096 9306..9893
<313> 12532: 559-578
<400> 12532
GTACTGCATT CAGTTGCTGG AAGGGCCATA CGTTGAAACG GCACCGGAAG 50
TGGTTGCAGC ATTCAGACCG AAGAGTGCAA GACGCGACGT TAGCGAATAA 100
<210> 12533
<211> 100
<212> DNA
<213> Escherichia coli K-12 MG1655
<220> mog b0010
<222> 479-578
<223> mog b0010 U00096 9306..9893
<313> 12533: 515-534
<400> 12533
CCAGCGTACC GTACTGCATT CAGTTGCTGG AAGGGCCATA CGTTGAAACG 50
GCACCGGAAG TGGTTGCAGC ATTCAGACCG AAGAGTGCAA GACGCGACGT 100
<210> 12534
<211> 100
<212> DNA
<213> Escherichia coli K-12 MG1655
<220> mog b0010
<222> 382-481
<223> mog b0010 U00096 9306..9893
<313> 12534: 420-439
<400> 12534
CTGATCCTTA ACTTACCCGG TCAGCCGAAG TCTATTAAAG AGACGCTGGA 50
AGGTGTGAAG GACGCTGAGG GTAACGTTGT GGTACACGGT ATTTTTGCCA 100
<210> 12535
<211> 100
<212> DNA
<213> Escherichia coli K-12 MG1655

<220> msyb msyB
<222> 270-369
<223> msyb msyB U00096 complement(1113030..1113407)
<313> 12535: 320-339
<400> 12535
GCAGGAAGAG AATACATTAC ATGAATGGGA CGAGGGGGAA TTTCAGCTTG 50
AGCCACCGCT GGATACCGAG GAAGGACGCG CAGCAGCTGA TGAGTGGGAT 100
<210> 12536
<211> 100
<212> DNA
<213> Escherichia coli K-12 MG1655
<220> msyb msyB
<222> 233-332
<223> msyb msyB U00096 complement(1113030..1113407)
<313> 12536: 288-307
<400> 12536
ATGGCGACTA TGATGAGATA GAGATACGCC AGGAGTGGCA GGAAGAGAAT 50
ACATTACATG AATGGGACGA GGGGGAATTT CAGCTTGAGC CACCGCTGGA 100
<210> 12537
<211> 100
<212> DNA
<213> Escherichia coli K-12 MG1655
<220> msyb msyB
<222> 211-310
<223> msyb msyB U00096 complement(1113030..1113407)
<313> 12537: 238-257
<400> 12537
GAAGCCGCGC AAAGTGTTTT TGATGGCGAC TATGATGAGA TAGAGATACG 50
CCAGGAGTGG CAGGAAGAGA ATACATTACA TGAATGGGAC GAGGGGGAAT 100
<210> 12538
<211> 100
<212> DNA
<213> Escherichia coli K-12 MG1655
<220> vacb b4180
<222> 2385-2484
<223> vacb b4180 U00096 4404190..4406673
<313> 12538: 2454-2473
<400> 12538
GAAAGACGCG AGAAAAGCGA AAAAGCCATC GGCGAAAACG CAGAAAATAG 50
CTGCAGCGAC CAAAGCGAAG CGTGCGGCGA AGAAAAAAGT GGCAGAGTGA 100
<210> 12539
<211> 100
<212> DNA
<213> Escherichia coli K-12 MG1655
<220> vacb b4180
<222> 2384-2483
<223> vacb b4180 U00096 4404190..4406673
<313> 12539: 2415-2434
<400> 12539
AGAAAGACGC GAGAAAAGCG AAAAAGCCAT CGGCGAAAAC GCAGAAAATA 50
GCTGCAGCGA CCAAAGCGAA GCGTGCGGCG AAGAAAAAAG TGGCAGAGTG 100
<210> 12540
<211> 100
<212> DNA
<213> Escherichia coli K-12 MG1655
<220> vacb b4180
<222> 2323-2422
<223> vacb b4180 U00096 4404190..4406673
<313> 12540: 2371-2390
<400> 12540
GTAAACTTTG AGCCAGACAG CGCCTTCCGC GGTGAGAAAA AAACGAAGCC 50
GAAAGCGGCG AAGAAAGACG CGAGAAAAGC GAAAAAGCCA TCGGCGAAAA 100
<210> 12541
<211> 100
<212> DNA
<213> Escherichia coli K-12 MG1655
<220> xyla b3566
<222> 1188-1287
<223> xyla b3566 U00096 complement(3727072..3728394)
<313> 12541: 1225-1244
<400> 12541

GCAAATCCTG AAAGGCCAAA TGTCACTGGC AGATTTAGCC AAATATGCTC 50
AGGAACATCA TTTGTCTCCG GTGCATCAGA GTGGTCGCCA GGAACAACTG 100
<210> 12542
<211> 100
<212> DNA
<213> Escherichia coli K-12 MG1655
<220> xyla b3566
<222> 1154-1253
<223> xyla b3566 U00096 complement(3727072..3728394)
<313> 12542: 1197-1216
<400> 12542
AGCGTTATTC CGGCTGGAAT AGCGAATTGG GCCAGCAAAT CCTGAAAGGC 50
CAAATGTCAC TGGCAGATTT AGCCAAATAT GCTCAGGAAC ATCATTTGTC 100
<210> 12543
<211> 100
<212> DNA
<213> Escherichia coli K-12 MG1655
<220> xyla b3566
<222> 1083-1182
<223> xyla b3566 U00096 complement(3727072..3728394)
<313> 12543: 1136-1155
<400> 12543
TACGATGGCA CTGGCGCTGA AAATTGCAGC GCGCATGATT GAAGATGGCG 50
AGCTGGATAA ACGCATCGCG CAGCGTTATT CCGGCTGGAA TAGCGAATTG 100
<210> 12544
<211> 100
<212> DNA
<213> Escherichia coli K-12 MG1655
<220> xylb b3565
<222> 1356-1455
<223> xylb b3565 U00096 complement(3725546..3727000)
<313> 12544: 1409-1428
<400> 12544
ACAGTCGCAT CTACCAGATG CGCAGCGTTA TGCCGCTTAT CAGCCACGAC 50
GAGAAACGTT CCGTCGCCTC TATCAGCAAC TTCTGCCATT AATGGCGTAA 100
<210> 12545
<211> 100
<212> DNA
<213> Escherichia coli K-12 MG1655
<220> xylb b3565
<222> 1292-1391
<223> xylb b3565 U00096 complement(3725546..3727000)
<313> 12545: 1336-1355
<400> 12545
TGGCGCAGAT CGCGGCGAAT CCAGAGAAAT CGCTCATTGA ATTGTTGCCG 50
CAACTACCGT TAGAACAGTC GCATCTACCA GATGCGCAGC GTTATGCCGC 100
<210> 12546
<211> 100
<212> DNA
<213> Escherichia coli K-12 MG1655
<220> xylb b3565
<222> 1278-1377
<223> xylb b3565 U00096 complement(3725546..3727000)
<313> 12546: 1303-1322
<400> 12546
GGGCGCAGCA AGGCTGGCGC AGATCGCGGC GAATCCAGAG AAATCGCTCA 50
TTGAATTGTT GCCGCAACTA CCGTTAGAAC AGTCGCATCT ACCAGATGCG 100
<210> 12547
<211> 100
<212> DNA
<213> Escherichia coli K-12 MG1655
<220> xyle b4032
<222> 1377-1476
<223> xyle b4032 U00096 complement(4238358..4239833)
<313> 12547: 1452-1471
<400> 12547
GTGGAAATTT GTCCCGGAAA CCAAAGGTAA AACCCTTGAG GAGCTGGAAG 50
CGCTCTGGGA ACCGGAAACG AAGAAAACAC AACAAACTGC TACGCTGTAA 100
<210> 12548
<211> 100
<212> DNA

<213> Escherichia coli K-12 MG1655
<220> xyle b4032
<222> 1362-1461
<223> xyle b4032 U00096 complement(4238358..4239833)
<313> 12548: 1394-1413
<400> 12548
GGCAGCACTG TTTATGTGGA AATTTGTCCC GGAAACCAAA GGTAAAACCC 50
TTGAGGAGCT GGAAGCGCTC TGGGAACCGG AAACGAAGAA AACACAACAA 100
<210> 12549
<211> 100
<212> DNA
<213> Escherichia coli K-12 MG1655
<220> xyle b4032
<222> 1264-1363
<223> xyle b4032 U00096 complement(4238358..4239833)
<313> 12549: 1316-1335
<400> 12549
GTCTCCTGGA CCTTCCCGAT GATGGACAAA AACTCCTGGC TGGTGGCCCA 50
TTTCCACAAC GGTTTCTCCT ACTGGATTTA CGGTTGTATG GGCGTTCTGG 100
<210> 12550
<211> 100
<212> DNA
<213> Escherichia coli K-12 MG1655
<220> nika b3477
<222> 1476-1575
<223> nika b3477 U00096 3611298..3612872
<313> 12550: 1503-1522
<400> 12550
CTCAATGATG GTGGTATCAA AACCGGAGCT GGGTAACATC CCCTACGCGC 50
CGATCGCCAC CGAAATTCCG TTCGAACAGA TTAAACCGGT GAAACCTTAA 100
<210> 12551
<211> 100
<212> DNA
<213> Escherichia coli K-12 MG1655
<220> nika b3477
<222> 1350-1449
<223> nika b3477 U00096 3611298..3612872
<313> 12551: 1391-1410
<400> 12551
ACCGCTGATT GATAAAGAGA TCGGCGAAGT GCTGGCGACC CATGACGAAA 50
CGCAACGTCA GGCGCTGTAT CGCGACATTC TGACCCGTCT GCATGACGAG 100
<210> 12552
<211> 100
<212> DNA
<213> Escherichia coli K-12 MG1655
<220> nika b3477
<222> 1284-1383
<223> nika b3477 U00096 3611298..3612872
<313> 12552: 1332-1351
<400> 12552
CTTCCTCAGT TCAATGCGCG TACCGTCACA CGCTGACTTC CAGGCACAGC 50
AAGGATTAGC CGACAAACCG CTGATTGATA AAGAGATCGG CGAAGTGCTG 100
<210> 12553
<211> 100
<212> DNA
<213> Escherichia coli K-12 MG1655
<220> xylf b3567
<222> 872-971
<223> xylf b3567 U00096 3728760..3729752
<313> 12553: 902-921
<400> 12553
CCACACTGAA TAATGGCCTG AAAGATGTCC CCTCCCGCCT CCTGACACCG 50
ATCGATGTGA ATAAAAACAA CATCAAAGAT ACGGTAATTA AAGACGGATT 100
<210> 12554
<211> 100
<212> DNA
<213> Escherichia coli K-12 MG1655
<220> xylf b3567
<222> 826-925
<223> xylf b3567 U00096 3728760..3729752
<313> 12554: 856-875

<400> 12554
GAAATTGCCG TTGAGTTGGG CAATGGTCAG GAACCAAAAG CAGATACCAC 50
ACTGAATAAT GGCCTGAAAG ATGTCCCCTC CCGCCTCCTG ACACCGATCG 100
<210> 12555
<211> 100
<212> DNA
<213> Escherichia coli K-12 MG1655
<220> xylf b3567
<222> 702-801
<223> xylf b3567 U00096 3728760..3729752
<313> 12555: 744-763
<400> 12555
TTTATCAGGG AAAGTAGCAA TCTCCGGCCA GGATGCGGAT CTCGCAGGTA 50
TTAAACGTAT TGCTGCCGGT ACGCAAACTA TGACGGTGTA TAAACCTATT 100
<210> 12556
<211> 100
<212> DNA
<213> Escherichia coli K-12 MG1655
<220> nikb b3478
<222> 846-945
<223> nikb b3478 U00096 3612872..3613816
<313> 12556: 917-936
<400> 12556
GATGATGGTG GTGGTTTTTG TGGTCTGTAA TTTGATTGTC GATTTGCTCA 50
ACGCCGCGCT GGACCCGCGC ATTCGTCGTC ATGAAGGAGC GCACGCGTGA 100
<210> 12557
<211> 100
<212> DNA
<213> Escherichia coli K-12 MG1655
<220> nikb b3478
<222> 811-910
<223> nikb b3478 U00096 3612872..3613816
<313> 12557: 847-866
<400> 12557
AACCGTGACT ATCCGGTGAT CCAGTGCTTT ACGCTGATGA TGGTGGTGGT 50
TTTTGTGGTC TGTAATTTGA TTGTCGATTT GCTCAACGCC GCGCTGGACC 100
<210> 12558
<211> 100
<212> DNA
<213> Escherichia coli K-12 MG1655
<220> nikb b3478
<222> 788-887
<223> nikb b3478 U00096 3612872..3613816
<313> 12558: 810-829
<400> 12558
GCTATGCGGT GTCGGCGATT TTTAACCGTG ACTATCCGGT GATCCAGTGC 50
TTTACGCTGA TGATGGTGGT GGTTTTTGTG GTCTGTAATT TGATTGTCGA 100
<210> 12559
<211> 100
<212> DNA
<213> Escherichia coli K-12 MG1655
<220> vacj b2347
<222> 657-756
<223> vacj b2347 U00096 complement(2462272..2463027)
<313> 12559: 698-717
<400> 12559
GCGTCATGAT TTCATCGCTA ATGGCGGCGA ACTCAAACCG CAGGAAAATC 50
CGAACGCACA AGCGATTCAG GATGATTTAA AAGATATTGA TTCTGAATAA 100
<210> 12560
<211> 100
<212> DNA
<213> Escherichia coli K-12 MG1655
<220> vacj b2347
<222> 591-690
<223> vacj b2347 U00096 complement(2462272..2463027)
<313> 12560: 618-637
<400> 12560
GGATTCCGAT GGTCTGCTGC GTCAGTCGTC CGATCCTTAT ATTATGGTGC 50
GCGAAGCGTA CTTCCAGCGT CATGATTTCA TCGCTAATGG CGGCGAACTC 100
<210> 12561
<211> 100

<212> DNA
<213> Escherichia coli K-12 MG1655
<220> vacj b2347
<222> 524-623
<223> vacj b2347 U00096 complement(2462272..2463027)
<313> 12561: 562-581
<400> 12561
GGCTGACCTG GCCGATGTCT GTGGGTAAAT GGACGCTTGA AGGGATCGAA 50
ACCCGCGCTC AGCTGCTGGA TTCCGATGGT CTGCTGCGTC AGTCGTCCGA 100
<210> 12562
<211> 100
<212> DNA
<213> Escherichia coli K-12 MG1655
<220> xylg b3568
<222> 1443-1542
<223> xylg b3568 U00096 3729830..3731371
<313> 12562: 1512-1531
<400> 12562
GAAACTAAAA GCCAACCTGA TAAATCATAA CCTGACTCAG GAGCAGGTGA 50
TGGAAGCCGC ATTGAGGAGC GAACATCATG TCGAAAAGCA ATCCGTCTGA 100
<210> 12563
<211> 100
<212> DNA
<213> Escherichia coli K-12 MG1655
<220> xylg b3568
<222> 1443-1542
<223> xylg b3568 U00096 3729830..3731371
<313> 12563: 1466-1485
<400> 12563
GAAACTAAAA GCCAACCTGA TAAATCATAA CCTGACTCAG GAGCAGGTGA 50
TGGAAGCCGC ATTGAGGAGC GAACATCATG TCGAAAAGCA ATCCGTCTGA 100
<210> 12564
<211> 100
<212> DNA
<213> Escherichia coli K-12 MG1655
<220> xylg b3568
<222> 1394-1493
<223> xylg b3568 U00096 3729830..3731371
<313> 12564: 1431-1450
<400> 12564
TACCTGAAGT GCTCGGCCTT AGCGATCGTG TACTGGTGAT GCATGAAGGG 50
AAACTAAAAG CCAACCTGAT AAATCATAAC CTGACTCAGG AGCAGGTGAT 100
<210> 12565
<211> 100
<212> DNA
<213> Escherichia coli K-12 MG1655
<220> nikc b3479
<222> 735-834
<223> nikc b3479 U00096 3613813..3614646
<313> 12565: 813-832
<400> 12565
GGGGCTGGCG CTGTTTATCA GCGTGATGGC CTTTAACCTG GTGGGTGACG 50
CACTGCGCGA TCATCTGGAC CCTCATCTGG TGACGGAGCA CGCACACTAA 100
<210> 12566
<211> 100
<212> DNA
<213> Escherichia coli K-12 MG1655
<220> nikc b3479
<222> 708-807
<223> nikc b3479 U00096 3613813..3614646
<313> 12566: 766-785
<400> 12566
GACCCAGCCG CTGCAAATGT TCTGGCCGGG GCTGGCGCTG TTTATCAGCG 50
TGATGGCCTT TAACCTGGTG GGTGACGCAC TGCGCGATCA TCTGGACCCT 100
<210> 12567
<211> 100
<212> DNA
<213> Escherichia coli K-12 MG1655
<220> nikc b3479
<222> 666-765
<223> nikc b3479 U00096 3613813..3614646

<313> 12567: 711-730
<400> 12567
CGAATGGGGC GTGATGATTA ACGACGCGCG CCAGTATATC TGGACCCAGC 50
CGCTGCAAAT GTTCTGGCCG GGGCTGGCGC TGTTTATCAG CGTGATGGCC 100
<210> 12568
<211> 100
<212> DNA
<213> Escherichia coli K-12 MG1655
<220> xylh b3569
<222> 1083-1182
<223> xylh b3569 U00096 3731349..3732530
<313> 12568: 1148-1167
<400> 12568
GAGTATGATG GATGTACCGA CCTTCTGGCA GTATATCGTT AAAGGTGCGA 50
TTCTGTTGCT GGCAGTATGG ATGGACTCCG CAACCAAACG CCGTTCTTGA 100
<210> 12569
<211> 100
<212> DNA
<213> Escherichia coli K-12 MG1655
<220> xylh b3569
<222> 1060-1159
<223> xylh b3569 U00096 3731349..3732530
<313> 12569: 1116-1135
<400> 12569
ATGGCTTCAC TGGATAACGG CATGAGTATG ATGGATGTAC CGACCTTCTG 50
GCAGTATATC GTTAAAGGTG CGATTCTGTT GCTGGCAGTA TGGATGGACT 100
<210> 12570
<211> 100
<212> DNA
<213> Escherichia coli K-12 MG1655
<220> xylh b3569
<222> 1010-1109
<223> xylh b3569 U00096 3731349..3732530
<313> 12570: 1065-1084
<400> 12570
TGGCTGGCGG TGTGGGAAGC GTTGCCGGAG CAGTAATGGG GGCATTTATC 50
ATGGCTTCAC TGGATAACGG CATGAGTATG ATGGATGTAC CGACCTTCTG 100
<210> 12571
<211> 100
<212> DNA
<213> Escherichia coli K-12 MG1655
<220> nikd b3480
<222> 666-765
<223> nikd b3480 U00096 3614646..3615410
<313> 12571: 728-747
<400> 12571
GGGCGATGTA GAAACGCTGT TTAACGCCCC CAAACATACA GTGACGCGCA 50
GCCTGGTTTC CGCTCATCTC GCCCTCTACG GTATGGAGCT GGCATCATGA 100
<210> 12572
<211> 100
<212> DNA
<213> Escherichia coli K-12 MG1655
<220> nikd b3480
<222> 642-741
<223> nikd b3480 U00096 3614646..3615410
<313> 12572: 693-712
<400> 12572
GTCTGACGGT AAGATTGTCG AACAGGGCGA TGTAGAAACG CTGTTTAACG 50
CCCCCAAACA TACAGTGACG CGCAGCCTGG TTTCCGCTCA TCTCGCCCTC 100
<210> 12573
<211> 100
<212> DNA
<213> Escherichia coli K-12 MG1655
<220> nikd b3480
<222> 621-720
<223> nikd b3480 U00096 3614646..3615410
<313> 12573: 651-670
<400> 12573
GGCGGATGAC GTGGCGGTGA TGTCTGACGG TAAGATTGTC GAACAGGGCG 50
ATGTAGAAAC GCTGTTTAAC GCCCCCAAAC ATACAGTGAC GCGCAGCCTG 100
<210> 12574

<211> 100
<212> DNA
<213> Escherichia coli K-12 MG1655
<220> nike b3481
<222> 708-807
<223> nike b3481 U00096 3615407..3616213
<313> 12574: 777-796
<400> 12574
GGGAGAGAAA TTAACCTTTT CCTCTGACGC CGGACGTGTG CTACAAAACG 50
CGGTATTACC CGCATTCCCC GTGCGCCGTC GCACCACAGA AAAGGTTTAA 100
<210> 12575
<211> 100
<212> DNA
<213> Escherichia coli K-12 MG1655
<220> nike b3481
<222> 699-798
<223> nike b3481 U00096 3615407..3616213
<313> 12575: 738-757
<400> 12575
CCAGGTGGTG GGAGAGAAAT TAACCTTTTC CTCTGACGCC GGACGTGTGC 50
TACAAAACGC GGTATTACCC GCATTCCCCG TGCGCCGTCG CACCACAGAA 100
<210> 12576
<211> 100
<212> DNA
<213> Escherichia coli K-12 MG1655
<220> nike b3481
<222> 650-749
<223> nike b3481 U00096 3615407..3616213
<313> 12576: 698-717
<400> 12576
GCTTTTGCCA GCGGGTAATG GTTATGGACA ACGGACAAAT CGTCGAAACC 50
CAGGTGGTGG GAGAGAAATT AACCTTTTCC TCTGACGCCG GACGTGTGCT 100
<210> 12577
<211> 100
<212> DNA
<213> Escherichia coli K-12 MG1655
<220> xylr b3570
<222> 1023-1122
<223> xylr b3570 U00096 3732608..3733786
<313> 12577: 1085-1104
<400> 12577
AGCGCGCAGT CTGCTGATTT CAACCACCTT GTCGATCAAT GAGATATCGC 50
AAATGTGCGG TTATCCATCG CTGCAATATT TCTACTCTGT TTTTAAAAAA 100
<210> 12578
<211> 100
<212> DNA
<213> Escherichia coli K-12 MG1655
<220> xylr b3570
<222> 999-1098
<223> xylr b3570 U00096 3732608..3733786
<313> 12578: 1026-1045
<400> 12578
GATTCATGCC GAGAAGCTGG AGAAAGCGCG CAGTCTGCTG ATTTCAACCA 50
CCTTGTCGAT CAATGAGATA TCGCAAATGT GCGGTTATCC ATCGCTGCAA 100
<210> 12579
<211> 100
<212> DNA
<213> Escherichia coli K-12 MG1655
<220> xylr b3570
<222> 947-1046
<223> xylr b3570 U00096 3732608..3733786
<313> 12579: 999-1018
<400> 12579
CCAATCTTGA GAAGCGTTTT AAAGAAGAGG TGGGTGAAAC CATCCATGCC 50
ATGATTCATG CCGAGAAGCT GGAGAAAGCG CGCAGTCTGC TGATTTCAAC 100
<210> 12580
<211> 100
<212> DNA
<213> Escherichia coli K-12 MG1655
<220> hdha b1620
<222> 669-768

<223> hdha b1620 U00096 complement(1695297..1696064)
<313> 12580: 722-741
<400> 12580
TATTGCTAAC GCAGCGCTGT TCCTTTGCTC GCCTGCTGCG AGCTGGGTAA 50
GCGGACAAAT TCTCACCGTC TCCGGTGGTG GGGTACAGGA GCTCAATTAA 100
<210> 12581
<211> 100
<212> DNA
<213> Escherichia coli K-12 MG1655
<220> hdha b1620
<222> 572-671
<223> hdha b1620 U00096 complement(1695297..1696064)
<313> 12581: 618-637
<400> 12581
CAATATTAAC CGATGCCCTG AAATCCGTTA TTACACCAGA AATTGAACAA 50
AAAATGTTAC AGCACACGCC GATCAGACGT CTGGGCCAAC CGCAAGATAT 100
<210> 12582
<211> 100
<212> DNA
<213> Escherichia coli K-12 MG1655
<220> hdha b1620
<222> 529-628
<223> hdha b1620 U00096 complement(1695297..1696064)
<313> 12582: 549-568
<400> 12582
CTGGGTGAAA AAAATATTCG GGTAAATGGC ATTGCGCCGG GGGCAATATT 50
AACCGATGCC CTGAAATCCG TTATTACACC AGAAATTGAA CAAAAAATGT 100
<210> 12583
<211> 100
<212> DNA
<213> Escherichia coli K-12 MG1655
<220> prsa b1208
<222> 849-948
<223> prsa b1208 U00096 complement(1260151..1261098)
<313> 12583: 909-928
<400> 12583
ACTGCCGAAC GTGCGTACTC TGACCCTGTC AGGTATGCTG GCCGAAGCGA 50
TTCGTCGTAT CAGCAACGAA GAATCGATCT CTGCCATGTT CGAACACTAA 100
<210> 12584
<211> 100
<212> DNA
<213> Escherichia coli K-12 MG1655
<220> prsa b1208
<222> 796-895
<223> prsa b1208 U00096 complement(1260151..1261098)
<313> 12584: 837-856
<400> 12584
ATTGATGAAG TCGTTGTCTG CGATACCATT CCGCTGAGCG ATGAAATCAA 50
ATCACTGCCG AACGTGCGTA CTCTGACCCT GTCAGGTATG CTGGCCGAAG 100
<210> 12585
<211> 100
<212> DNA
<213> Escherichia coli K-12 MG1655
<220> prsa b1208
<222> 729-828
<223> prsa b1208 U00096 complement(1260151..1261098)
<313> 12585: 770-789
<400> 12585
TGTATTTGCG TACGCGACTC ACCCGATCTT CTCTGGCAAC GCGGCGAACA 50
ACCTGCGTAA CTCTGTAATT GATGAAGTCG TTGTCTGCGA TACCATTCCG 100
<210> 12586
<211> 100
<212> DNA
<213> Escherichia coli K-12 MG1655
<220> mrp b2114
<222> 1039-1138
<223> mrp b2114 U00096 complement(2191079..2192218)
<313> 12586: 1103-1122
<400> 12586
GAATTTACCG CTATCTACCG CCAGCTGGCT GACCGCGTTG CAGCTCAGCT 50
CTACTGGCAG GGTGAAGTCA TTCCAGGCGA GATTTCCTTC CGCGCGGTCT 100

<210> 12587
<211> 100
<212> DNA
<213> Escherichia coli K-12 MG1655
<220> mrp b2114
<222> 964-1063
<223> mrp b2114 U00096 complement(2191079..2192218)
<313> 12587: 1006-1025
<400> 12587
CAGATGCCGT TACACATCTC CTTACGTGAG GATCTCGATA AAGGTACGCC  50
AACGGTTATC AGCCGTCCGG AGAGCGAATT TACCGCTATC TACCGCCAGC  100
<210> 12588
<211> 100
<212> DNA
<213> Escherichia coli K-12 MG1655
<220> mrp b2114
<222> 890-989
<223> mrp b2114 U00096 complement(2191079..2192218)
<313> 12588: 924-943
<400> 12588
GTCACCATGA ACCGATTTTC GGTACCGGTG GTGCAGAGAA ACTGGCTGAA  50
AAATACCATA CACAGCTGCT GGGGCAGATG CCGTTACACA TCTCCTTACG  100
<210> 12589
<211> 100
<212> DNA
<213> Escherichia coli K-12 MG1655
<220> mrr b4352
<222> 816-915
<223> mrr b4352 U00096 4584519..4585433
<313> 12589: 880-899
<400> 12589
TGATGGGGAA CGCCTGGTGC ACTTAATGAT CGAAAACGAA GTAGGGGTTT  50
CTTCACGTTT GTTGAAGGTG CCGAAACTGG ATATGGACTA TTTTGAGTGA  100
<210> 12590
<211> 100
<212> DNA
<213> Escherichia coli K-12 MG1655
<220> mrr b4352
<222> 760-859
<223> mrr b4352 U00096 4584519..4585433
<313> 12590: 791-810
<400> 12590
GGATTTACTT CTCAGGCGCG TGACTTTGCC CAATCCGTCG AGGGTATGGT  50
GTTGGTTGAT GGGGAACGCC TGGTGCACTT AATGATCGAA AACGAAGTAG  100
<210> 12591
<211> 100
<212> DNA
<213> Escherichia coli K-12 MG1655
<220> mrr b4352
<222> 690-789
<223> mrr b4352 U00096 4584519..4585433
<313> 12591: 726-745
<400> 12591
AGAATTACAG GCATTTTACG GCGCACTGGC TGGGCAAAAA GCGAAACGTG  50
GGGTGTTTAT TACCACTTCT GGATTTACTT CTCAGGCGCG TGACTTTGCC  100
<210> 12592
<211> 100
<212> DNA
<213> Escherichia coli K-12 MG1655
<220> sbma b0378
<222> 1122-1221
<223> sbma b0378 U00096 395863..397083
<313> 12592: 1179-1198
<400> 12592
CACACTGGTT GAGTTGATGT CTATCTACAA ACGTCTGCGC AGCTTTGAAC  50
ATGAGCTGGA TGGTGACAAA ATTCAGGAAG TAACCCATAC CTTGAGCTAA  100
<210> 12593
<211> 100
<212> DNA
<213> Escherichia coli K-12 MG1655
<220> sbma b0378

<222> 1045-1144
<223> sbma b0378 U00096 395863..397083
<313> 12593: 1094-1113
<400> 12593
CTCGGCCTGA TGACGCAGAT TACCAACGTT TTTGGTCAGG TTCGCGGTGC 50
TTTCCAGTAC CTGATTAACT CATGGACCAC ACTGGTTGAG TTGATGTCTA 100
<210> 12594
<211> 100
<212> DNA
<213> Escherichia coli K-12 MG1655
<220> sbma b0378
<222> 1007-1106
<223> sbma b0378 U00096 395863..397083
<313> 12594: 1053-1072
<400> 12594
TCTTGCTGTT TCCGTCAATT GTTGCCGGTA CGATTACGCT CGGCCTGATG 50
ACGCAGATTA CCAACGTTTT TGGTCAGGTT CGCGGTGCTT TCCAGTACCT 100
<210> 12595
<211> 100
<212> DNA
<213> Escherichia coli K-12 MG1655
<220> b2001 b2002
<222> 1440-1539
<223> b2001 b2002 U00096 2072795..2074333
<313> 12595: 1488-1507
<400> 12595
TAATGGCCCC ATGCAACTGG AAGTCCCCCG TGATCAGTAT GCCGGCGCTG 50
TTGAAACCAT GAGGAATAAG ATCCGCGAAG GTAAAGTAGA GGAGAGATGA 100
<210> 12596
<211> 100
<212> DNA
<213> Escherichia coli K-12 MG1655
<220> b2001 b2002
<222> 1422-1521
<223> b2001 b2002 U00096 2072795..2074333
<313> 12596: 1449-1468
<400> 12596
GTATCGTTAC ATGGGAAATA ATGGCCCCAT GCAACTGGAA GTCCCCCGTG 50
ATCAGTATGC CGGCGCTGTT GAAACCATGA GGAATAAGAT CCGCGAAGGT 100
<210> 12597
<211> 100
<212> DNA
<213> Escherichia coli K-12 MG1655
<220> b2001 b2002
<222> 1386-1485
<223> b2001 b2002 U00096 2072795..2074333
<313> 12597: 1414-1433
<400> 12597
TAGCGTCGGT GCGGCATTCG ACGGACAAAA CGGACAGTAT CGTTACATGG 50
GAAATAATGG CCCCATGCAA CTGGAAGTCC CCCGTGATCA GTATGCCGGC 100
<210> 12598
<211> 100
<212> DNA
<213> Escherichia coli K-12 MG1655
<220> sbmc b2010
<222> 375-474
<223> sbmc b2010 U00096 complement(2078811..2079284)
<313> 12598: 414-433
<400> 12598
GTTACCAAAG CCCTGCTTCG AGGTTTATTT GAACAATGGC GCGGAAGATG 50
GGTACTGGGA TATCGAAATG TATGTTGCGG TGCAGCCAAA ACATCACTAA 100
<210> 12599
<211> 100
<212> DNA
<213> Escherichia coli K-12 MG1655
<220> sbmc b2010
<222> 322-421
<223> sbmc b2010 U00096 complement(2078811..2079284)
<313> 12599: 372-391
<400> 12599
AAACCCTGGT ATCAGTTCTT TAATAGTCTC TTGCAGGACA GTGCTTATGA 50

AATGTTACCA AAGCCCTGCT TCGAGGTTTA TTTGAACAAT GGCGCGGAAG 100
<210> 12600
<211> 100
<212> DNA
<213> Escherichia coli K-12 MG1655
<220> sbmc b2010
<222> 266-365
<223> sbmc b2010 U00096 complement(2078811..2079284)
<313> 12600: 304-323
<400> 12600
TTACAGGTGG TCAGTATGCG GTGGCGGTAG CTCGTGTAGT CGGTGATGAT 50
TTTGCTAAAC CCTGGTATCA GTTCTTTAAT AGTCTCTTGC AGGACAGTGC 100
<210> 12601
<211> 100
<212> DNA
<213> Escherichia coli K-12 MG1655
<220> fba b2926
<222> 962-1061
<223> fba b2926 U00096 complement(3068185..3069264)
<313> 12601: 1012-1031
<400> 12601
AAGATCAGCC GAACAAGAAA TACTACGATC CGCGCGTATG GCTGCGTGCC 50
GGTCAGACTT CGATGATCGC TCGTCTGGAG AAAGCATTCC AGGAACTGAA 100
<210> 12602
<211> 100
<212> DNA
<213> Escherichia coli K-12 MG1655
<220> fba b2926
<222> 916-1015
<223> fba b2926 U00096 complement(3068185..3069264)
<313> 12602: 959-978
<400> 12602
GCGAACGAAG CTTATCTGCA GGGTCAGCTG GGTAACCCGA AAGGCGAAGA 50
TCAGCCGAAC AAGAAATACT ACGATCCGCG CGTATGGCTG CGTGCCGGTC 100
<210> 12603
<211> 100
<212> DNA
<213> Escherichia coli K-12 MG1655
<220> fba b2926
<222> 881-980
<223> fba b2926 U00096 complement(3068185..3069264)
<313> 12603: 897-916
<400> 12603
GGGCAACCTG GGAAGGCGTT CTGAACTACT ACAAAGCGAA CGAAGCTTAT 50
CTGCAGGGTC AGCTGGGTAA CCCGAAAGGC GAAGATCAGC CGAACAAGAA 100
<210> 12604
<211> 100
<212> DNA
<213> Escherichia coli K-12 MG1655
<220> nine b0549
<222> 54-153
<223> nine b0549 U00096 572144..572314
<313> 12604: 106-125
<400> 12604
TCGTCGTAAG CGTAAACCTG AGCTGAAGCC ATCCGAAATA CCAACACTGC 50
TCGGATATAC CGCCAGCTTG GTTGATAAAA AATGGTTGCG ACTGGCAGCA 100
<210> 12605
<211> 100
<212> DNA
<213> Escherichia coli K-12 MG1655
<220> nine b0549
<222> 28-127
<223> nine b0549 U00096 572144..572314
<313> 12605: 79-98
<400> 12605
AACGGACACA TCTACAGAGT ACCAAATCGT CGTAAGCGTA AACCTGAGCT 50
GAAGCCATCC GAAATACCAA CACTGCTCGG ATATACCGCC AGCTTGGTTG 100
<210> 12606
<211> 100
<212> DNA
<213> Escherichia coli K-12 MG1655

<220> nine b0549
<222> 1-100
<223> nine b0549 U00096 572144..572314
<313> 12606: 33-52
<400> 12606
ATGGCTACAC CGCTTATTCG TGTCATGAAC GGACACATCT ACAGAGTACC 50
AAATCGTCGT AAGCGTAAAC CTGAGCTGAA GCCATCCGAA ATACCAACAC 100
<210> 12607
<211> 100
<212> DNA
<213> Escherichia coli K-12 MG1655
<220> yoae b1817
<222> 1458-1557
<223> yoae b1817 U00096 complement(1898053..1899609)
<313> 12607: 1525-1544
<400> 12607
TGTAGGGCCA CTGCATATCA CCATCATTGA AGCCAATGAT TATCGTGTTG 50
ATCTGGTTCG CATTGTTAAA GAGCAACCGG CGCACGATGA AGATGAGTAA 100
<210> 12608
<211> 100
<212> DNA
<213> Escherichia coli K-12 MG1655
<220> yoae b1817
<222> 1424-1523
<223> yoae b1817 U00096 complement(1898053..1899609)
<313> 12608: 1458-1477
<400> 12608
ATGGTCACAT TCCCCGTGTG GGCGATGTGA TTGATGTAGG GCCACTGCAT 50
ATCACCATCA TTGAAGCCAA TGATTATCGT GTTGATCTGG TTCGCATTGT 100
<210> 12609
<211> 100
<212> DNA
<213> Escherichia coli K-12 MG1655
<220> yoae b1817
<222> 1377-1476
<223> yoae b1817 U00096 complement(1898053..1899609)
<313> 12609: 1407-1426
<400> 12609
CGATGACGAT GATATCGCGA CGGTCGCGGG CCTCGTGATC TCGGCAAATG 50
GTCACATTCC CCGTGTGGGC GATGTGATTG ATGTAGGGCC ACTGCATATC 100
<210> 12610
<211> 100
<212> DNA
<213> Escherichia coli K-12 MG1655
<220> yoaf b1794
<222> 156-255
<223> yoaf b1794 U00096 complement(1875302..1875556)
<313> 12610: 211-230
<400> 12610
GACAGCGCAA GGGGTCAGCA ACAATTGCAA ATTACCGGGC GGTGAAACGA 50
TTGATGAATG GGCATTGTGG CGGCGTGACC ATCCGGCTGG TGAAAAATAA 100
<210> 12611
<211> 100
<212> DNA
<213> Escherichia coli K-12 MG1655
<220> yoaf b1794
<222> 116-215
<223> yoaf b1794 U00096 complement(1875302..1875556)
<313> 12611: 168-187
<400> 12611
TCTATTGCCA GCAGAAGGGC GGGACGCTCA TTCCAGTGCA GACAGCGCAA 50
GGGGTCAGCA ACAATTGCAA ATTACCGGGC GGTGAAACGA TTGATGAATG 100
<210> 12612
<211> 100
<212> DNA
<213> Escherichia coli K-12 MG1655
<220> yoaf b1794
<222> 101-200
<223> yoaf b1794 U00096 complement(1875302..1875556)
<313> 12612: 132-151
<400> 12612

CAAATCCGGC GTCGGTCTAT TGCCAGCAGA AGGGCGGGAC GCTCATTCCA 50
GTGCAGACAG CGCAAGGGGT CAGCAACAAT TGCAAATTAC CGGGCGGTGA 100
<210> 12613
<211> 100
<212> DNA
<213> Escherichia coli K-12 MG1655
<220> yoag b1797
<222> 78-177
<223> yoag b1797 U00096 complement(1877427..1877609)
<313> 12613: 105-124
<400> 12613
GATGACTTTG CTGGTGCCAG AAGTGGCGGC TGAAGTGATA AAAGATCTGG 50
TGAATACCGT ACGTTCTTAT GACACGGAAA ACGAACATGA TGTTTGTGGT 100
<210> 12614
<211> 100
<212> DNA
<213> Escherichia coli K-12 MG1655
<220> yoag b1797
<222> 22-121
<223> yoag b1797 U00096 complement(1877427..1877609)
<313> 12614: 72-91
<400> 12614
GTGACCGTCA CCAATAACAG CAATGGCGTT TCTGTCGATT ATGAAACAGA 50
GACGCCGATG ACTTTGCTGG TGCCAGAAGT GGCGGCTGAA GTGATAAAAG 100
<210> 12615
<211> 100
<212> DNA
<213> Escherichia coli K-12 MG1655
<220> yoag b1797
<222> 1-100
<223> yoag b1797 U00096 complement(1877427..1877609)
<313> 12615: 35-54
<400> 12615
ATGGGCAAAG CAACGTATAC CGTGACCGTC ACCAATAACA GCAATGGCGT 50
TTCTGTCGAT TATGAAACAG AGACGCCGAT GACTTTGCTG GTGCCAGAAG 100
<210> 12616
<211> 100
<212> DNA
<213> Escherichia coli K-12 MG1655
<220> hslj b1380
<222> 243-342
<223> hslj b1380 U00096 complement(1439345..1439767)
<313> 12616: 299-318
<400> 12616
GACAGCCAAA GGGCTGGCAA TGACCCGTAT GATGTGCGCT AACCCGCAGC 50
TTAATGAACT CGATAACACC ATTAGCGAAA TGCTGAAAGA AGGTGCACAA 100
<210> 12617
<211> 100
<212> DNA
<213> Escherichia coli K-12 MG1655
<220> hslj b1380
<222> 214-313
<223> hslj b1380 U00096 complement(1439345..1439767)
<313> 12617: 260-279
<400> 12617
GGTGAAGGCA AACTGTCTAA TGGTGAACTG ACAGCCAAAG GGCTGGCAAT 50
GACCCGTATG ATGTGCGCTA ACCCGCAGCT TAATGAACTC GATAACACCA 100
<210> 12618
<211> 100
<212> DNA
<213> Escherichia coli K-12 MG1655
<220> hslj b1380
<222> 165-264
<223> hslj b1380 U00096 complement(1439345..1439767)
<313> 12618: 208-227
<400> 12618
CTTTGGTGAA AAAATGATGA TTTCCGGCAG CATGTGTAAC CGCTTTAGCG 50
GTGAAGGCAA ACTGTCTAAT GGTGAACTGA CAGCCAAAGG GCTGGCAATG 100
<210> 12619
<211> 100
<212> DNA

<213> Escherichia coli K-12 MG1655
<220> fbp b4233
<222> 900-999
<223> fbp b4233 U00096 complement(4452185..4453183)
<313> 12619: 959-978
<400> 12619
CATCCCGGAA ACCCTGCACC AGCGCCGTTC ATTCTTTGTC GGCAACGACC 50
ATATGGTTGA AGATGTCGAA CGCTTTATCC GTGAGTTCCC GGACGCGTAA 100
<210> 12620
<211> 100
<212> DNA
<213> Escherichia coli K-12 MG1655
<220> fbp b4233
<222> 877-976
<223> fbp b4233 U00096 complement(4452185..4453183)
<313> 12620: 917-936
<400> 12620
GGCAAAGAGC GTATTCTGGA TATCATCCCG GAAACCCTGC ACCAGCGCCG 50
TTCATTCTTT GTCGGCAACG ACCATATGGT TGAAGATGTC GAACGCTTTA 100
<210> 12621
<211> 100
<212> DNA
<213> Escherichia coli K-12 MG1655
<220> fbp b4233
<222> 827-926
<223> fbp b4233 U00096 complement(4452185..4453183)
<313> 12621: 859-878
<400> 12621
GCAACCCGAT GGCATTCCTG GCGGAACAAG CGGGCGGTAA AGCGAGCGAT 50
GGCAAAGAGC GTATTCTGGA TATCATCCCG GAAACCCTGC ACCAGCGCCG 100
<210> 12622
<211> 100
<212> DNA
<213> Escherichia coli K-12 MG1655
<220> hslu b3932
<222> 1233-1332
<223> hslu b3932 U00096 complement(4117996..4119327)
<313> 12622: 1293-1312
<400> 12622
CGATTTAAGC GGTCAAAATA TCACTATTGA CGCAGATTAT GTGAGCAAAC 50
ATCTGGATGC GTTGGTGGCA GATGAAGATC TGAGCCGTTT TATCCTATAA 100
<210> 12623
<211> 100
<212> DNA
<213> Escherichia coli K-12 MG1655
<220> hslu b3932
<222> 1204-1303
<223> hslu b3932 U00096 complement(4117996..4119327)
<313> 12623: 1240-1259
<400> 12623
TTAATGGAAG AGATTTCCTA CGACGCCAGC GATTTAAGCG GTCAAAATAT 50
CACTATTGAC GCAGATTATG TGAGCAAACA TCTGGATGCG TTGGTGGCAG 100
<210> 12624
<211> 100
<212> DNA
<213> Escherichia coli K-12 MG1655
<220> hslu b3932
<222> 1097-1196
<223> hslu b3932 U00096 complement(4117996..4119327)
<313> 12624: 1146-1165
<400> 12624
TCGAGTTTAC CGACTCCGGT ATTAAACGCA TCGCGGAAGC GGCATGGCAG 50
GTGAACGAAT CTACCGAAAA CATCGGTGCT CGTCGTTTAC ACACTGTTCT 100
<210> 12625
<211> 100
<212> DNA
<213> Escherichia coli K-12 MG1655
<220> hslv b3933
<222> 432-531
<223> hslv b3933 U00096 complement(4119337..4119867)
<313> 12625: 482-501

<400> 12625
CGCCCGTGAA ATTGCTGAAA AGGCGTTGGA TATTGCAGGC GACATTTGCA 50
TCTATACCAA CCATTTCCAC ACCATCGAAG AATTAAGCTA CAAAGCGTAA 100
<210> 12626
<211> 100
<212> DNA
<213> Escherichia coli K-12 MG1655
<220> hslv b3933
<222> 400-499
<223> hslv b3933 U00096 complement(4119337..4119867)
<313> 12626: 417-436
<400> 12626
GCGCGCGCGC TGTTAGAAAA CACTGAACTT AGCGCCCGTG AAATTGCTGA 50
AAAGGCGTTG GATATTGCAG GCGACATTTG CATCTATACC AACCATTTCC 100
<210> 12627
<211> 100
<212> DNA
<213> Escherichia coli K-12 MG1655
<220> hslv b3933
<222> 311-410
<223> hslv b3933 U00096 complement(4119337..4119867)
<313> 12627: 371-390
<400> 12627
CGCTTATCAT CACCGGTAAC GGTGACGTGG TGCAGCCAGA AAACGATCTT 50
ATTGCTATCG GCTCCGGCGG CCCTTACGCC CAGGCTGCGG CGCGCGCGCT 100
<210> 12628
<211> 100
<212> DNA
<213> Escherichia coli K-12 MG1655
<220> udk b2067
<222> 532-631
<223> udk b2067 U00096 complement(2140329..2141024)
<313> 12628: 590-609
<400> 12628
TCAATGGATT CAGTGATGGC GCAATATCAA AAAACCGTGC GCCCGATGTT 50
CCTGCAATTC ATTGAGCCTT CTAAACAATA TGCGGACATT ATCGTGCCGC 100
<210> 12629
<211> 100
<212> DNA
<213> Escherichia coli K-12 MG1655
<220> udk b2067
<222> 415-514
<223> udk b2067 U00096 complement(2140329..2141024)
<313> 12629: 459-478
<400> 12629
TTGTTGCTGA CGGATGCGCG TTTGCGTGAC GAACTTAACT TCTCCATTTT 50
CGTTGATACC CCGCTGGATA TCTGCCTGAT GCGCCGCATC AAGCGTGACG 100
<210> 12630
<211> 100
<212> DNA
<213> Escherichia coli K-12 MG1655
<220> udk b2067
<222> 368-467
<223> udk b2067 U00096 complement(2140329..2141024)
<313> 12630: 401-420
<400> 12630
AAACGGTGAC GGTTGAGCCG AAGAAGGTCA TCATTCTCGA AGGCATTTTG 50
TTGCTGACGG ATGCGCGTTT GCGTGACGAA CTTAACTTCT CCATTTTCGT 100
<210> 12631
<211> 100
<212> DNA
<213> Escherichia coli K-12 MG1655
<220> udp b3832
<222> 663-762
<223> udp b3832 U00096 4014018..4014779
<313> 12631: 727-746
<400> 12631
TAACCGCACC CAGCAAGAGA TCCCGAATGC TGAGACGATG AAACAAACCG 50
AAAGCCATGC GGTGAAAATC GTGGTGGAAG CGGCGCGTCG TCTGCTGTAA 100
<210> 12632
<211> 100

<212> DNA
<213> Escherichia coli K-12 MG1655
<220> udp b3832
<222> 658-757
<223> udp b3832 U00096 4014018..4014779
<313> 12632: 686-705
<400> 12632
ATCGTTAACC GCACCCAGCA AGAGATCCCG AATGCTGAGA CGATGAAACA 50
AACCGAAAGC CATGCGGTGA AAATCGTGGT GGAAGCGGCG CGTCGTCTGC 100
<210> 12633
<211> 100
<212> DNA
<213> Escherichia coli K-12 MG1655
<220> udp b3832
<222> 559-658
<223> udp b3832 U00096 4014018..4014779
<313> 12633: 615-634
<400> 12633
TGGCAGGCGA TGGGCGTAAT GAACTATGAA ATGGAATCTG CAACCCTGCT 50
GACCATGTGT GCAAGTCAGG GCCTGCGTGC CGGTATGGTA GCGGGTGTTA 100
<210> 12634
<211> 100
<212> DNA
<213> Escherichia coli K-12 MG1655
<220> b2016 b2017
<222> 681-780
<223> b2016 b2017 U00096 complement(2086326..2087150)
<313> 12634: 749-768
<400> 12634
GGCCCGTTTA CTGGGGCTGG AACCTCCGCA GTTCAGAAAT AGTCTGGACA 50
GCGGCAAAGG TAAGATTATT GATGGCAGTC GGATTTGTAA TGAACTGGGA 100
<210> 12635
<211> 100
<212> DNA
<213> Escherichia coli K-12 MG1655
<220> b2016 b2017
<222> 667-766
<223> b2016 b2017 U00096 complement(2086326..2087150)
<313> 12635: 699-718
<400> 12635
TTCTATCCGC AGATGGCCCG TTTACTGGGG CTGGAACCTC CGCAGTTCAG 50
AAATAGTCTG GACAGCGGCA AAGGTAAGAT TATTGATGGC AGTCGGATTT 100
<210> 12636
<211> 100
<212> DNA
<213> Escherichia coli K-12 MG1655
<220> b2016 b2017
<222> 592-691
<223> b2016 b2017 U00096 complement(2086326..2087150)
<313> 12636: 629-648
<400> 12636
ACTCTGTTGT TACAGGCACC TAAAGGCGGA CACATCTATA ATATATGTGC 50
GCCAGCTCAC CCTGCGCGTA ATGTTTTCTA TCCGCAGATG GCCCGTTTAC 100
<210> 12637
<211> 100
<212> DNA
<213> Escherichia coli K-12 MG1655
<220> mtr b3162
<222> 1097-1196
<223> mtr b3162 U00096 complement(3302214..3303458)
<313> 12637: 1150-1169
<400> 12637
TAGCCCGTGC ATCGCGTAAA CGCTTTGGCA GCCCGAAATT CCGCGTCTGG 50
GGTGGCAAGC CGATGATTGC GCTGATTCTG GTGTTTGGCG TCGGCAACGC 100
<210> 12638
<211> 100
<212> DNA
<213> Escherichia coli K-12 MG1655
<220> mtr b3162
<222> 1061-1160
<223> mtr b3162 U00096 complement(3302214..3303458)

<313> 12638: 1120-1139
<400> 12638
CGGCTACCAT CTGGGCGGCA ATTGTTCCGG CGCTGTTAGC CCGTGCATCG 50
CGTAAACGCT TTGGCAGCCC GAAATTCCGC GTCTGGGGTG GCAAGCCGAT 100
<210> 12639
<211> 100
<212> DNA
<213> Escherichia coli K-12 MG1655
<220> mtr b3162
<222> 1001-1100
<223> mtr b3162 U00096 complement(3302214..3303458)
<313> 12639: 1051-1070
<400> 12639
TTGTGGGGGG GCTGTTGTTC CCGAACGGAT TCCTGTACGC CATTGGTTAT 50
GCTGGTTTAG CGGCTACCAT CTGGGCGGCA ATTGTTCCGG CGCTGTTAGC 100
<210> 12640
<211> 100
<212> DNA
<213> Escherichia coli K-12 MG1655
<220> rela b2785
<222> 2136-2235
<223> rela b2785 U00096 complement(2909439..2911673)
<313> 12640: 2188-2207
<400> 12640
GACCATTGAG ATTTACAACC TGCAAGTGCT GGGGCGCGTG CTGGGTAAAC 50
TCAACCAGGT GCCGGATGTT ATCGACGCGC GTCGGTTGCA CGGGAGTTAG 100
<210> 12641
<211> 100
<212> DNA
<213> Escherichia coli K-12 MG1655
<220> rela b2785
<222> 2122-2221
<223> rela b2785 U00096 complement(2909439..2911673)
<313> 12641: 2144-2163
<400> 12641
GCGACCATCG ACATGACCAT TGAGATTTAC AACCTGCAAG TGCTGGGGCG 50
CGTGCTGGGT AAACTCAACC AGGTGCCGGA TGTTATCGAC GCGCGTCGGT 100
<210> 12642
<211> 100
<212> DNA
<213> Escherichia coli K-12 MG1655
<220> rela b2785
<222> 2072-2171
<223> rela b2785 U00096 complement(2909439..2911673)
<313> 12642: 2103-2122
<400> 12642
AGGTGAACGT GCTTGGCGTT GCCAGCCGTA GCGACACCAA ACAGCAACTG 50
GCGACCATCG ACATGACCAT TGAGATTTAC AACCTGCAAG TGCTGGGGCG 100
<210> 12643
<211> 100
<212> DNA
<213> Escherichia coli K-12 MG1655
<220> relb relB
<222> 141-240
<223> relb relB U00096 complement(1643657..1643896)
<313> 12643: 217-236
<400> 12643
ACAGACACTC CTGAGTGATG AAGATGCTGA ACTTGTGGAG ATAGTGAAAG 50
AACGGCTTCG TAATCCTAAG CCAGTACGTG TGACGCTGGA TGAACTCTGA 100
<210> 12644
<211> 100
<212> DNA
<213> Escherichia coli K-12 MG1655
<220> relb relB
<222> 141-240
<223> relb relB U00096 complement(1643657..1643896)
<313> 12644: 206-225
<400> 12644
ACAGACACTC CTGAGTGATG AAGATGCTGA ACTTGTGGAG ATAGTGAAAG 50
AACGGCTTCG TAATCCTAAG CCAGTACGTG TGACGCTGGA TGAACTCTGA 100
<210> 12645

<211> 100
<212> DNA
<213> Escherichia coli K-12 MG1655
<220> relb relB
<222> 141-240
<223> relb relB U00096 complement(1643657..1643896)
<313> 12645: 189-208
<400> 12645
ACAGACACTC CTGAGTGATG AAGATGCTGA ACTTGTGGAG ATAGTGAAAG 50
AACGGCTTCG TAATCCTAAG CCAGTACGTG TGACGCTGGA TGAACTCTGA 100
<210> 12646
<211> 100
<212> DNA
<213> Escherichia coli K-12 MG1655
<220> rele b1564
<222> 189-288
<223> rele b1564 U00096 complement(1643370..1643657)
<313> 12646: 260-279
<400> 12646
ATACCAGGTT ATAGACGAGA AAGTTGTCGT TTTCGTGATT TCTGTTGGGA 50
AAAGAGAACG CTCGGAAGTA TATAGCGAGG CGGTCAAACG CATTCTCTGA 100
<210> 12647
<211> 100
<212> DNA
<213> Escherichia coli K-12 MG1655
<220> rele b1564
<222> 152-251
<223> rele b1564 U00096 complement(1643370..1643657)
<313> 12647: 182-201
<400> 12647
ACAAGATTAA GCTCCGGTCT TCAGGCTATC GCCTTGTATA CCAGGTTATA 50
GACGAGAAAG TTGTCGTTTT CGTGATTTCT GTTGGGAAAA GAGAACGCTC 100
<210> 12648
<211> 100
<212> DNA
<213> Escherichia coli K-12 MG1655
<220> rele b1564
<222> 73-172
<223> rele b1564 U00096 complement(1643370..1643657)
<313> 12648: 121-140
<400> 12648
CAGTTGAAAA AGAAGCTGGT TGAAGTACTT GAGTCACCCC GGATTGAAGC 50
AAACAAGCTC CGTGGTATGC CTGATTGTTA CAAGATTAAG CTCCGGTCTT 100
<210> 12649
<211> 100
<212> DNA
<213> Escherichia coli K-12 MG1655
<220> relf b1563
<222> 57-156
<223> relf b1563 U00096 complement(1643143..1643298)
<313> 12649: 122-141
<400> 12649
AGTGACGGCA CTGGTAACGA GGAAAGACCT CTGCGAGGTA CGAATCCGAA 50
CCGGCCAGAC GGAGGTCGCT GTCTTCACAG CTTACGAACC TGAGGAGTAA 100
<210> 12650
<211> 100
<212> DNA
<213> Escherichia coli K-12 MG1655
<220> relf b1563
<222> 40-139
<223> relf b1563 U00096 complement(1643143..1643298)
<313> 12650: 86-105
<400> 12650
ATCTGTTTAA CCGTCATAGT GACGGCACTG GTAACGAGGA AAGACCTCTG 50
CGAGGTACGA ATCCGAACCG GCCAGACGGA GGTCGCTGTC TTCACAGCTT 100
<210> 12651
<211> 100
<212> DNA
<213> Escherichia coli K-12 MG1655
<220> relf b1563
<222> 1-100

<223> relf b1563 U00096 complement(1643143..1643298)
<313> 12651: 37-56
<400> 12651
ATGAAGCAGC AAAAGGCGAT GTTAATCGCC CTGATCGTCA TCTGTTTAAC  50
CGTCATAGTG ACGGCACTGG TAACGAGGAA AGACCTCTGC GAGGTACGAA  100
<210> 12652
<211> 100
<212> DNA
<213> Escherichia coli K-12 MG1655
<220> fdx b2526
<222> 231-330
<223> fdx b2526 U00096 complement(2654768..2655103)
<313> 12652: 285-304
<400> 12652
GGGACTGGAG CCGGAAAGCC GTTTAAGCTG CCAGGCGCGC GTTACCGACG  50
AAGATTTAGT AGTCGAAATC CCGCGTTACA CTATCAACCA TGCGCGTGAG  100
<210> 12653
<211> 100
<212> DNA
<213> Escherichia coli K-12 MG1655
<220> fdx b2526
<222> 182-281
<223> fdx b2526 U00096 complement(2654768..2655103)
<313> 12653: 221-240
<400> 12653
CACTGCCGGA AAGCTCAGAG CAGGAAGACG ACATGCTGGA CAAAGCCTGG  50
GGACTGGAGC CGGAAAGCCG TTTAAGCTGC CAGGCGCGCG TTACCGACGA  100
<210> 12654
<211> 100
<212> DNA
<213> Escherichia coli K-12 MG1655
<220> fdx b2526
<222> 146-245
<223> fdx b2526 U00096 complement(2654768..2655103)
<313> 12654: 194-213
<400> 12654
CCACCTGCCA CTGCATCGTT CGTGAAGGTT TTGACTCACT GCCGGAAAGC  50
TCAGAGCAGG AAGACGACAT GCTGGACAAA GCCTGGGGAC TGGAGCCGGA  100
<210> 12655
<211> 100
<212> DNA
<213> Escherichia coli K-12 MG1655
<220> yodb b1975
<222> 462-561
<223> yodb b1975 U00096 2040360..2040920
<313> 12655: 525-544
<400> 12655
AGGCTACTTT TTAATCGCAG CTCACGCTGG CGCAGCACTC TTTCATCACT  50
ACATTCAGAA AGATAATACT CTGTTACGAA TGATGCCTCG CCGCAAATAA  100
<210> 12656
<211> 100
<212> DNA
<213> Escherichia coli K-12 MG1655
<220> yodb b1975
<222> 418-517
<223> yodb b1975 U00096 2040360..2040920
<313> 12656: 456-475
<400> 12656
ATTAAAGCAC TGATAAAAAA TATTCACGAA ACCTGGGCAA ATATAGGCTA  50
CTTTTTAATC GCAGCTCACG CTGGCGCAGC ACTCTTTCAT CACTACATTC  100
<210> 12657
<211> 100
<212> DNA
<213> Escherichia coli K-12 MG1655
<220> yodb b1975
<222> 367-466
<223> yodb b1975 U00096 2040360..2040920
<313> 12657: 402-421
<400> 12657
TGGAGTTTCC TTGGTTTCAA TGTGTCTCCC TTTGTTACCC CAAACAGCGA  50
AATTAAAGCA CTGATAAAAA ATATTCACGA AACCTGGGCA AATATAGGCT  100

<210> 12658
<211> 100
<212> DNA
<213> Escherichia coli K-12 MG1655
<220> usha b0481
<222> 1535-1634
<223> usha b0481 U00096 504138..505790
<313> 12658: 1581-1600
<400> 12658
AACCGGGCTA TGTGAATACC GGCTTTATTG ATGCCGAAGT GCTGAAAGCG 50
TATATCCAGA AAAGCTCGCC GCTGGATGTG AGTGTTTATG AACCGAAAGG 100
<210> 12659
<211> 100
<212> DNA
<213> Escherichia coli K-12 MG1655
<220> usha b0481
<222> 1476-1575
<223> usha b0481 U00096 504138..505790
<313> 12659: 1530-1549
<400> 12659
TATGGCGACA TTAAACTTCA ATGCCACCGG CGGTGATGGA TATCCGCGCC 50
TTGATAACAA ACCGGGCTAT GTGAATACCG GCTTTATTGA TGCCGAAGTG 100
<210> 12660
<211> 100
<212> DNA
<213> Escherichia coli K-12 MG1655
<220> usha b0481
<222> 1459-1558
<223> usha b0481 U00096 504138..505790
<313> 12660: 1492-1511
<400> 12660
CCGGCGAAAA CTTACCGTAT GGCGACATTA AACTTCAATG CCACCGGCGG 50
TGATGGATAT CCGCGCCTTG ATAACAAACC GGGCTATGTG AATACCGGCT 100
<210> 12661
<211> 100
<212> DNA
<213> Escherichia coli K-12 MG1655
<220> fes b0586
<222> 1026-1125
<223> fes b0586 U00096 612038..613162
<313> 12661: 1088-1107
<400> 12661
GCGTCAGGTT GACGGCGGAC ATGATGCGCT TTGTTGGCGC GGTGGCTTGA 50
TGCAGGGGCT AATCGACCTC TGGCAACCAC TTTTCCATGA CAGGAGTTGA 100
<210> 12662
<211> 100
<212> DNA
<213> Escherichia coli K-12 MG1655
<220> fes b0586
<222> 1005-1104
<223> fes b0586 U00096 612038..613162
<313> 12662: 1057-1076
<400> 12662
CATAAAAGAA TCCATTTTCT GGCGTCAGGT TGACGGCGGA CATGATGCGC 50
TTTGTTGGCG CGGTGGCTTG ATGCAGGGGC TAATCGACCT CTGGCAACCA 100
<210> 12663
<211> 100
<212> DNA
<213> Escherichia coli K-12 MG1655
<220> fes b0586
<222> 871-970
<223> fes b0586 U00096 612038..613162
<313> 12663: 905-924
<400> 12663
CAAGAGGGCG TGTTACTTGA AAAGCTAAAA GCTGGTGAAG TTAGCGCCGA 50
AGGTCTGCGC ATTGTGCTGG AAGCGGGTAT TCGCGAGCCG ATGATCATGC 100
<210> 12664
<211> 100
<212> DNA
<213> Escherichia coli K-12 MG1655
<220> ugd b2029

<222> 1068-1167
<223> ugd b2029 U00096 complement(2096469..2097635)
<313> 12664: 1132-1151
<400> 12664
CAAACAACAA GCCGACGTCA TTATCTCTAA CCGAATGGCA GAAGAGCTTA 50
AGGATGTGGC AGATAAGGTA TACACCCGCG ATCTCTTTGG CAGCGACTAA 100
<210> 12665
<211> 100
<212> DNA
<213> Escherichia coli K-12 MG1655
<220> ugd b2029
<222> 1023-1122
<223> ugd b2029 U00096 complement(2096469..2097635)
<313> 12665: 1050-1069
<400> 12665
AGACTCATTC TTCAACTCTC GCCTGGAACG TGATCTCGCC ACCTTCAAAC 50
AACAAGCCGA CGTCATTATC TCTAACCGAA TGGCAGAAGA GCTTAAGGAT 100
<210> 12666
<211> 100
<212> DNA
<213> Escherichia coli K-12 MG1655
<220> ugd b2029
<222> 887-986
<223> ugd b2029 U00096 complement(2096469..2097635)
<313> 12666: 942-961
<400> 12666
CGCAAGTGGT GGGTATTTAT CGTCTGATTA TGAAGAGCGG TTCAGATAAC 50
TTCCGTGCGT CTTCTATTCA GGGGATTATG AAACGTATCA AGGCGAAAGG 100
<210> 12667
<211> 100
<212> DNA
<213> Escherichia coli K-12 MG1655
<220> b1310 b1311
<222> 1194-1293
<223> b1310 b1311 U00096 1369933..1371225
<313> 12667: 1272-1291
<400> 12667
GAGTTCAATG GTGCATAACG TCACCGTGGG TAAAGCCGAT CTCTCTACTA 50
CGCTGCAAGC GAGCCAGAAA AAACTGGATG AACTGATCGA ACAGCACTAA 100
<210> 12668
<211> 100
<212> DNA
<213> Escherichia coli K-12 MG1655
<220> b1310 b1311
<222> 1180-1279
<223> b1310 b1311 U00096 1369933..1371225
<313> 12668: 1223-1242
<400> 12668
GGTTCTGGCG TGGTGAGTTC AATGGTGCAT AACGTCACCG TGGGTAAAGC 50
CGATCTCTCT ACTACGCTGC AAGCGAGCCA GAAAAAACTG GATGAACTGA 100
<210> 12669
<211> 100
<212> DNA
<213> Escherichia coli K-12 MG1655
<220> b1310 b1311
<222> 1115-1214
<223> b1310 b1311 U00096 1369933..1371225
<313> 12669: 1171-1190
<400> 12669
TGCCAAATAT TCAGGTTTTT GGCGCAGTAG GGGATAAAAA CTTTACCCGC 50
ATGGGTGATG TGACGGGTTC TGGCGTGGTG AGTTCAATGG TGCATAACGT 100
<210> 12670
<211> 100
<212> DNA
<213> Escherichia coli K-12 MG1655
<220> yi41 b4279
<222> 1230-1329
<223> yi41 b4279 U00096 complement(4499671..4500999)
<313> 12670: 1300-1319
<400> 12670
GAAATTACCG ACAAGAAGGG AAAGGGCCTT CCCGAGAGTG GTAAAGGAGA 50

GGCCCTGGAA ATACCCCACA GCCCCGAAAA AGAGCCAGTC AGTTGCTTAA 100
<210> 12671
<211> 100
<212> DNA
<213> Escherichia coli K-12 MG1655
<220> yi41 b4279
<222> 1182-1281
<223> yi41 b4279 U00096 complement(4499671..4500999)
<313> 12671: 1226-1245
<400> 12671
ACGTATACCG GAGCTGATGC GCGATCTTGC AAGTATGGGA CAACTTGTGA 50
AATTACCGAC AAGAAGGGAA AGGGCCTTCC CGAGAGTGGT AAAGGAGAGG 100
<210> 12672
<211> 100
<212> DNA
<213> Escherichia coli K-12 MG1655
<220> yi41 b4279
<222> 1143-1242
<223> yi41 b4279 U00096 complement(4499671..4500999)
<313> 12672: 1173-1192
<400> 12672
GATGAGAATG CTGATGACAT TGCAGGGCGC TTCACCGGGA CGTATACCGG 50
AGCTGATGCG CGATCTTGCA AGTATGGGAC AACTTGTGAA ATTACCGACA 100
<210> 12673
<211> 100
<212> DNA
<213> Escherichia coli K-12 MG1655
<220> lola b0892
<222> 516-615
<223> lola b0892 U00096 936592..937206
<313> 12673: 590-609
<400> 12673
CAGTTATCAA CTGAAATCCC AGCAAAATGG GGCTGTGGAT GCAGCGAAAT 50
TTACCTTCAC CCCGCCGCAA GGCGTCACGG TAGATGATCA ACGTAAGTAG 100
<210> 12674
<211> 100
<212> DNA
<213> Escherichia coli K-12 MG1655
<220> lola b0892
<222> 509-608
<223> lola b0892 U00096 936592..937206
<313> 12674: 543-562
<400> 12674
AGCGCAGCAG TTATCAACTG AAATCCCAGC AAAATGGGGC TGTGGATGCA 50
GCGAAATTTA CCTTCACCCC GCCGCAAGGC GTCACGGTAG ATGATCAACG 100
<210> 12675
<211> 100
<212> DNA
<213> Escherichia coli K-12 MG1655
<220> lola b0892
<222> 479-578
<223> lola b0892 U00096 936592..937206
<313> 12675: 505-524
<400> 12675
ATCAGTTTAG CGCGGTGGAG CAGGACGATC AGCGCAGCAG TTATCAACTG 50
AAATCCCAGC AAAATGGGGC TGTGGATGCA GCGAAATTTA CCTTCACCCC 100
<210> 12676
<211> 100
<212> DNA
<213> Escherichia coli K-12 MG1655
<220> nirb b3366
<222> 2445-2544
<223> nirb b3366 U00096 3491648..3494191
<313> 12676: 2515-2534
<400> 12676
GCGTGACCCG AACGTGCAGA TGGTGCCAGA GCGCGAACAG CACCGTCCGG 50
CAACGCCGTA TGAACGTATC CCAGTAACTC TGGTGGAGGA CAACGCATGA 100
<210> 12677
<211> 100
<212> DNA
<213> Escherichia coli K-12 MG1655

<220> nirb b3366
<222> 2384-2483
<223> nirb b3366 U00096 3491648..3494191
<313> 12677: 2440-2459
<400> 12677
CTGAAACGGT CAATACGCCG TCTGCGCAGA CTCGCTTCAA ACACTTCATC  50
AACAGCGACA AGCGTGACCC GAACGTGCAG ATGGTGCCAG AGCGCGAACA  100
<210> 12678
<211> 100
<212> DNA
<213> Escherichia coli K-12 MG1655
<220> nirb b3366
<222> 2298-2397
<223> nirb b3366 U00096 3491648..3494191
<313> 12678: 2330-2349
<400> 12678
AGTGATCATT GACGACAAAC TGGGGCTGAA CGCACATCTG GAAGAAGAGA  50
TGGCGCGCCT GCGTGAAGCG GTACTGTGTG AGTGGACTGA AACGGTCAAT  100
<210> 12679
<211> 100
<212> DNA
<213> Escherichia coli K-12 MG1655
<220> b1314 b1315
<222> 699-798
<223> b1314 b1315 U00096 1374049..1374846
<313> 12679: 761-780
<400> 12679
GGATAACTAT CAGGGCTATG TAGTGTACGA AGGGCGTATC CGGGCGGAAG  50
ATCCTGCCCA GGCGTACCGT GATTCGTTGG CCTGGTTGCG TACCTGCTAA  100
<210> 12680
<211> 100
<212> DNA
<213> Escherichia coli K-12 MG1655
<220> b1314 b1315
<222> 619-718
<223> b1314 b1315 U00096 1374049..1374846
<313> 12680: 639-658
<400> 12680
GTGCATATTG CGGATAACCA TCGCTACCAG CCGGGCAGCG GCACCCTGGA  50
TTTCCACGCG CTGTTTGAAC AGCTGCGCGC GGATAACTAT CAGGGCTATG  100
<210> 12681
<211> 100
<212> DNA
<213> Escherichia coli K-12 MG1655
<220> b1314 b1315
<222> 534-633
<223> b1314 b1315 U00096 1374049..1374846
<313> 12681: 594-613
<400> 12681
GATTATCGGC GATTTCTATC ACATGAATAT CGAAGAAGAT AACCTGGCGC  50
AGGCGCTGCA TGACAACCGC GACCTGCTCG GTCATGTGCA TATTGCGGAT  100
<210> 12682
<211> 100
<212> DNA
<213> Escherichia coli K-12 MG1655
<220> nirc b3368
<222> 456-555
<223> nirc b3368 U00096 3494892..3495446
<313> 12682: 515-534
<400> 12682
AGGTGCCGTA TTCATGGGAT TGGGTTATTG GTATGCTACG CCGAAAGCGA  50
ATCGTCCGGT TGCGGACAAA TTTAATCAAA CTGAAACGGC TGCCGGTTAA  100
<210> 12683
<211> 100
<212> DNA
<213> Escherichia coli K-12 MG1655
<220> nirc b3368
<222> 406-505
<223> nirc b3368 U00096 3494892..3495446
<313> 12683: 453-472
<400> 12683

GCGGGTATTG GTCATAACCT GCTGTGGGTG ACGCTGGGTA ATACTTTATC 50
AGGTGCCGTA TTCATGGGAT TGGGTTATTG GTATGCTACG CCGAAAGCGA 100
<210> 12684
<211> 100
<212> DNA
<213> Escherichia coli K-12 MG1655
<220> nirc b3368
<222> 330-429
<223> nirc b3368 U00096 3494892..3495446
<313> 12684: 364-383
<400> 12684
CTACGAGCAC TCTATCGCTA ACATGACGCT GTTCGCGCTC TCCTGGTTCG 50
GCAACCACAG CGAAGCCTAC ACGCTGGCGG GTATTGGTCA TAACCTGCTG 100
<210> 12685
<211> 100
<212> DNA
<213> Escherichia coli K-12 MG1655
<220> nird b3367
<222> 218-317
<223> nird b3367 U00096 3494188..3494514
<313> 12685: 274-293
<400> 12685
AAAAACAGCG TTTTCGCTTA AGCGACGGCT TGTGCATGGA AGACGAACAG 50
TTTTCCGTCA AACATTACGA AGCGCGAGTG AAAGACGGCG TGGTGCAGCT 100
<210> 12686
<211> 100
<212> DNA
<213> Escherichia coli K-12 MG1655
<220> nird b3367
<222> 164-263
<223> nird b3367 U00096 3494188..3494514
<313> 12686: 217-236
<400> 12686
CACGCGGACT GATTGCGGAA CACCAGGGCG AGCTGTGGGT CGCCAGCCCG 50
CTGAAAAAAC AGCGTTTTCG CTTAAGCGAC GGCTTGTGCA TGGAAGACGA 100
<210> 12687
<211> 100
<212> DNA
<213> Escherichia coli K-12 MG1655
<220> nird b3367
<222> 132-231
<223> nird b3367 U00096 3494188..3494514
<313> 12687: 166-185
<400> 12687
CATCGACCCG TTCTTCGAGT CCAGCGTGCT GTCACGCGGA CTGATTGCGG 50
AACACCAGGG CGAGCTGTGG GTCGCCAGCC CGCTGAAAAA ACAGCGTTTT 100
<210> 12688
<211> 100
<212> DNA
<213> Escherichia coli K-12 MG1655
<220> ffh ffh
<222> 1263-1362
<223> ffh ffh U00096 complement(2744454..2745815)
<313> 12688: 1329-1348
<400> 12688
CGACATGCAG CGCATGATGA AGAAAATGAA GAAGGGCGGA ATGGCGAAGA 50
TGATGAGAAG CATGAAGGGT ATGATGCCCC CAGGCTTCCC TGGTCGCTAA 100
<210> 12689
<211> 100
<212> DNA
<213> Escherichia coli K-12 MG1655
<220> ffh ffh
<222> 1263-1362
<223> ffh ffh U00096 complement(2744454..2745815)
<313> 12689: 1296-1315
<400> 12689
CGACATGCAG CGCATGATGA AGAAAATGAA GAAGGGCGGA ATGGCGAAGA 50
TGATGAGAAG CATGAAGGGT ATGATGCCCC CAGGCTTCCC TGGTCGCTAA 100
<210> 12690
<211> 100
<212> DNA

<213> Escherichia coli K-12 MG1655
<220> ffh ffh
<222> 1222-1321
<223> ffh ffh U00096 complement(2744454..2745815)
<313> 12690: 1257-1276
<400> 12690
ATGCAGGTGC AGGACGTTAA CCGTCTTCTG AAACAGTTCG ACGACATGCA 50
GCGCATGATG AAGAAAATGA AGAAGGGCGG AATGGCGAAG ATGATGAGAA 100
<210> 12691
<211> 100
<212> DNA
<213> Escherichia coli K-12 MG1655
<220> udha b3963
<222> 1231-1330
<223> udha b3963 U00096 complement(4156969..4158303)
<313> 12691: 1271-1290
<400> 12691
GGCGGCAACA CTATTGAGTA CTTCGTCAAC ACCACCTTTA ACTACCCGAC 50
GATGGCGGAA GCCTATCGGG TAGCTGCGTT AAACGGTTTA AACCGCCTGT 100
<210> 12692
<211> 100
<212> DNA
<213> Escherichia coli K-12 MG1655
<220> udha b3963
<222> 1162-1261
<223> udha b3963 U00096 complement(4156969..4158303)
<313> 12692: 1229-1248
<400> 12692
ATTCACTGCT TTGGCGAGCG CGCTGCCGAA ATTATTCATA TCGGTCAGGC 50
GATTATGGAA CAGAAAGGTG GCGGCAACAC TATTGAGTAC TTCGTCAACA 100
<210> 12693
<211> 100
<212> DNA
<213> Escherichia coli K-12 MG1655
<220> udha b3963
<222> 1068-1167
<223> udha b3963 U00096 complement(4156969..4158303)
<313> 12693: 1106-1125
<400> 12693
CCAGTTTAAA CATCTGGCAC GCGCACAAAT CGTCGGCATG AACGTGGGCA 50
CGCTGAAAAT TTTGTTCCAT CGGGAAACAA AAGAGATTCT GGGTATTCAC 100
<210> 12694
<211> 100
<212> DNA
<213> Escherichia coli K-12 MG1655
<220> b1327 b1328
<222> 834-933
<223> b1327 b1328 U00096 complement(1388957..1389889)
<313> 12694: 881-900
<400> 12694
CATTGTTGAA GGCGCAAACC ATATGGATCT TTATGACGGC AAAGCCTATG 50
TGGCTGAAGC AATTTCAGTA TTAGCTCCGT TCTTTGAGGA AACTCTGTAA 100
<210> 12695
<211> 100
<212> DNA
<213> Escherichia coli K-12 MG1655
<220> b1327 b1328
<222> 788-887
<223> b1327 b1328 U00096 complement(1388957..1389889)
<313> 12695: 823-842
<400> 12695
GTGACGACCT GTACGATCGA GCATCAAGCC AGGATAAACG CTATCACATT 50
GTTGAAGGCG CAAACCATAT GGATCTTTAT GACGGCAAAG CCTATGTGGC 100
<210> 12696
<211> 100
<212> DNA
<213> Escherichia coli K-12 MG1655
<220> b1327 b1328
<222> 731-830
<223> b1327 b1328 U00096 complement(1388957..1389889)
<313> 12696: 779-798

<400> 12696
ACCTGACTCA GCCGACGCAG ATTGTGGCGG GCAGCCAGGC AGGGAGCAAA 50
TGGATGAGTG ACGACCTGTA CGATCGAGCA TCAAGCCAGG ATAAACGCTA 100
<210> 12697
<211> 100
<212> DNA
<213> Escherichia coli K-12 MG1655
<220> b1329 b1329
<222> 1477-1576
<223> b1329 b1329 U00096 1391230..1392864
<313> 12697: 1507-1526
<400> 12697
TACAACGCGG CAGAAAAAAT CCTCATGGAG CAAGCACCGA TTGCACCAAT 50
TTATCAATAT ACCAATGGAC GATTAATCAA GCCGTGGCTG AAAGGTTATC 100
<210> 12698
<211> 100
<212> DNA
<213> Escherichia coli K-12 MG1655
<220> b1329 b1329
<222> 1438-1537
<223> b1329 b1329 U00096 1391230..1392864
<313> 12698: 1474-1493
<400> 12698
GCATCGACGG AAAATACCGT TAAAGCGCGT AATGCCGATT ACAACGCGGC 50
AGAAAAAATC CTCATGGAGC AAGCACCGAT TGCACCAATT TATCAATATA 100
<210> 12699
<211> 100
<212> DNA
<213> Escherichia coli K-12 MG1655
<220> b1329 b1329
<222> 1403-1502
<223> b1329 b1329 U00096 1391230..1392864
<313> 12699: 1434-1453
<400> 12699
TTAACAATCC GGCATATGAC AAAGTTCTGG CCCAGGCATC GACGGAAAAT 50
ACCGTTAAAG CGCGTAATGC CGATTACAAC GCGGCAGAAA AAATCCTCAT 100
<210> 12700
<211> 100
<212> DNA
<213> Escherichia coli K-12 MG1655
<220> b1330 b1331
<222> 933-1032
<223> b1330 b1331 U00096 complement(1392915..1393946)
<313> 12700: 1006-1025
<400> 12700
GAAGATTATC GATATTGTAC AGTCACACGG CGCGGACTTT GCCTTCCCCA 50
GCCAGACGCT GTATATGGAT AACATTACAC CGCCGGAACA GGGTCGCTAA 100
<210> 12701
<211> 100
<212> DNA
<213> Escherichia coli K-12 MG1655
<220> b1330 b1331
<222> 912-1011
<223> b1330 b1331 U00096 complement(1392915..1393946)
<313> 12701: 964-983
<400> 12701
TGCACAGCAA GACGTTTATT TGAAGATTAT CGATATTGTA CAGTCACACG 50
GCGCGGACTT TGCCTTCCCC AGCCAGACGC TGTATATGGA TAACATTACA 100
<210> 12702
<211> 100
<212> DNA
<213> Escherichia coli K-12 MG1655
<220> b1330 b1331
<222> 852-951
<223> b1330 b1331 U00096 complement(1392915..1393946)
<313> 12702: 892-911
<400> 12702
TTCATTGAAT ATTATGGTTT ATTGCTTTAC CAAAACCACG GTATGGGCTG 50
AATGGCTTGC TGCACAGCAA GACGTTTATT TGAAGATTAT CGATATTGTA 100
<210> 12703
<211> 100

<212> DNA
<213> Escherichia coli K-12 MG1655
<220> daca b0633
<222> 1094-1193
<223> daca b0633 U00096 complement(661975..663186)
<313> 12703: 1134-1153
<400> 12703
TTGATGGCAA AACGATCGAG CAACGCCCGC TGGTTGTGTT GCAAGAAATC 50
CCGGAAGGTA ACTTCTTCGG CAAAATCATT GATTACATTA AATTAATGTT 100
<210> 12704
<211> 100
<212> DNA
<213> Escherichia coli K-12 MG1655
<220> daca b0633
<222> 1025-1124
<223> daca b0633 U00096 complement(661975..663186)
<313> 12704: 1060-1079
<400> 12704
TGCTGAACAG CAGTGAATTG CATGCGCCGC TGCAAAAGAA TCAGGTCGTC 50
GGAACTATCA ACTTCCAGCT TGATGGCAAA ACGATCGAGC AACGCCCGCT 100
<210> 12705
<211> 100
<212> DNA
<213> Escherichia coli K-12 MG1655
<220> daca b0633
<222> 979-1078
<223> daca b0633 U00096 complement(661975..663186)
<313> 12705: 1020-1039
<400> 12705
CTGACCATTC CGCGTGGTCG CATGAAAGAT CTGAAAGCCA GCTATGTGCT 50
GAACAGCAGT GAATTGCATG CGCCGCTGCA AAAGAATCAG GTCGTCGGAA 100
<210> 12706
<211> 100
<212> DNA
<213> Escherichia coli K-12 MG1655
<220> b2060 b2061
<222> 2075-2174
<223> b2060 b2061 U00096 complement(2131512..2133710)
<313> 12706: 2110-2129
<400> 12706
TGAGCCGCTT TGAGCAAAAC GGTATTCCGG TGAAAGGGGT GATTCTGAAC 50
TCCATCTTCC GCCGCGCCAG CGCGTATCAG GATTATGGCT ATTACGAATA 100
<210> 12707
<211> 100
<212> DNA
<213> Escherichia coli K-12 MG1655
<220> b2060 b2061
<222> 2042-2141
<223> b2060 b2061 U00096 complement(2131512..2133710)
<313> 12707: 2064-2083
<400> 12707
CGGTCAACAC ATTGAAAGAA GTGGAAACCA GTCTGAGCCG CTTTGAGCAA 50
AACGGTATTC CGGTGAAAGG GGTGATTCTG AACTCCATCT TCCGCCGCGC 100
<210> 12708
<211> 100
<212> DNA
<213> Escherichia coli K-12 MG1655
<220> b2060 b2061
<222> 1901-2000
<223> b2060 b2061 U00096 complement(2131512..2133710)
<313> 12708: 1923-1942
<400> 12708
GCGAACGCTT TGCCGAACTG GTGAACTGGG CGAGTAAAAA CTATGACCTG 50
GTGTTGATTG ATACGCCGCC GATTCTGGCA GTGACCGATG CGGCAATTGT 100
<210> 12709
<211> 100
<212> DNA
<213> Escherichia coli K-12 MG1655
<220> dacb b3183
<222> 1335-1434
<223> dacb b3183 U00096 3326604..3328037

<313> 12709: 1378-1397
<400> 12709
TTCTGGCTAT GCAGTAGAAC CTGCGGATCA GCGTAATCGC CGTATTCCGT 50
TAGTGCGTTT TGAAAGCCGT TTGTATAAAG ATATTTATCA GAACAATTAG 100
<210> 12710
<211> 100
<212> DNA
<213> Escherichia coli K-12 MG1655
<220> dacb b3183
<222> 1286-1385
<223> dacb b3183 U00096 3326604..3328037
<313> 12710: 1343-1362
<400> 12710
GATTCATTAC CACAGCGAGC GGGCAACGAA TGGCGTTTGT GCAATATCTT 50
TCTGGCTATG CAGTAGAACC TGCGGATCAG CGTAATCGCC GTATTCCGTT 100
<210> 12711
<211> 100
<212> DNA
<213> Escherichia coli K-12 MG1655
<220> dacb b3183
<222> 1263-1362
<223> dacb b3183 U00096 3326604..3328037
<313> 12711: 1305-1324
<400> 12711
GCAGGGGGTA TATAACCTGG CGGGATTCAT TACCACAGCG AGCGGGCAAC 50
GAATGGCGTT TGTGCAATAT CTTTCTGGCT ATGCAGTAGA ACCTGCGGAT 100
<210> 12712
<211> 100
<212> DNA
<213> Escherichia coli K-12 MG1655
<220> dacc b0840
<222> 1088-1187
<223> dacc b0840 U00096 879950..881152
<313> 12712: 1117-1136
<400> 12712
TTGAGCAGCG TCCGCTGATC GTGATGGAAA ATGTGGAAGA GGGCGGATTC 50
TTTGGTCGGG TGTGGGATTT CGTGATGATG AAATTCCATC AGTGGTTCGG 100
<210> 12713
<211> 100
<212> DNA
<213> Escherichia coli K-12 MG1655
<220> dacc b0840
<222> 1040-1139
<223> dacc b0840 U00096 879950..881152
<313> 12713: 1081-1100
<400> 12713
AAGGTCAGGT TGTCGGGACC ATTGATTTCC AGCTTAACGG TAAATCCATT 50
GAGCAGCGTC CGCTGATCGT GATGGAAAAT GTGGAAGAGG GCGGATTCTT 100
<210> 12714
<211> 100
<212> DNA
<213> Escherichia coli K-12 MG1655
<220> dacc b0840
<222> 1015-1114
<223> dacc b0840 U00096 879950..881152
<313> 12714: 1039-1058
<400> 12714
CCGCAGCTTA CCGCACCGCT GAAAAAAGGT CAGGTTGTCG GGACCATTGA 50
TTTCCAGCTT AACGGTAAAT CCATTGAGCA GCGTCCGCTG ATCGTGATGG 100
<210> 12715
<211> 100
<212> DNA
<213> Escherichia coli K-12 MG1655
<220> dacd b2011
<222> 1066-1165
<223> dacd b2011 U00096 complement(2079403..2080575)
<313> 12715: 1105-1124
<400> 12715
TACGACCGTG ATAAACAGGT GGCGCACTGG CCGCTGGTTA CCCTGGAATC 50
TGTCGGGGAA GGCAGCATGT TTTCTCGCCT GAGTGATTAT TTCCACCATA 100
<210> 12716

<211> 100
<212> DNA
<213> Escherichia coli K-12 MG1655
<220> dacd b2011
<222> 1036-1135
<223> dacd b2011 U00096 complement(2079403..2080575)
<313> 12716: 1068-1087
<400> 12716
GCCCATCAGC GGGTAGGGGA AATTGAACTT TACGACCGTG ATAAACAGGT 50
GGCGCACTGG CCGCTGGTTA CCCTGGAATC TGTCGGGGAA GGCAGCATGT 100
<210> 12717
<211> 100
<212> DNA
<213> Escherichia coli K-12 MG1655
<220> dacd b2011
<222> 1000-1099
<223> dacd b2011 U00096 complement(2079403..2080575)
<313> 12717: 1021-1040
<400> 12717
ACCCTTGATG GTAAAGAACT CACCGCGCCA ATTAGCGCCC ATCAGCGGGT 50
AGGGGAAATT GAACTTTACG ACCGTGATAA ACAGGTGGCG CACTGGCCGC 100
<210> 12718
<211> 100
<212> DNA
<213> Escherichia coli K-12 MG1655
<220> b1337 b1338
<222> 1347-1446
<223> b1337 b1338 U00096 complement(1399834..1401279)
<313> 12718: 1415-1434
<400> 12718
GCTATTGCAA GAATGCCAAC AAGAGCATCA GCAAGTAACG GACACGCAAC 50
CGTATCACTG CCCTATCCCG AAAAACGTGA CACCGTCACC TTTAAAATAA 100
<210> 12719
<211> 100
<212> DNA
<213> Escherichia coli K-12 MG1655
<220> b1337 b1338
<222> 1346-1445
<223> b1337 b1338 U00096 complement(1399834..1401279)
<313> 12719: 1377-1396
<400> 12719
GGCTATTGCA AGAATGCCAA CAAGAGCATC AGCAAGTAAC GGACACGCAA 50
CCGTATCACT GCCCTATCCC GAAAAACGTG ACACCGTCAC CTTTAAAATA 100
<210> 12720
<211> 100
<212> DNA
<213> Escherichia coli K-12 MG1655
<220> b1337 b1338
<222> 1264-1363
<223> b1337 b1338 U00096 complement(1399834..1401279)
<313> 12720: 1320-1339
<400> 12720
CGAACATCTA TTGCTCATAA AGGAATGCTG CTGGCGGCGA AAACTATGGC 50
AGCAACCACA GTCAATCTCT TCCTTGATTC AGGGCTATTG CAAGAATGCC 100
<210> 12721
<211> 100
<212> DNA
<213> Escherichia coli K-12 MG1655
<220> b0609 b0610
<222> 363-462
<223> b0609 b0610 U00096 642630..643091
<313> 12721: 434-453
<400> 12721
CGCTAAGATT GCGGAATTTA ACCCGGCTGT TCATTGTCAC CACGTCGTGT 50
GGCATCTCTT CTGGCGAACA CATTTGGGCG CGATCCAACT CTGCGTTTAA 100
<210> 12722
<211> 100
<212> DNA
<213> Escherichia coli K-12 MG1655
<220> b0609 b0610
<222> 328-427

<223> b0609 b0610 U00096 642630..643091
<313> 12722: 379-398
<400> 12722
CGGATACACC AGCGTGCGCA CACGCACTTC GCCATCGCTA AGATTGCGGA 50
ATTTAACCCG GCTGTTCATT GTCACCACGT CGTGTGGCAT CTCTTCTGGC 100
<210> 12723
<211> 100
<212> DNA
<213> Escherichia coli K-12 MG1655
<220> b0609 b0610
<222> 258-357
<223> b0609 b0610 U00096 642630..643091
<313> 12723: 308-327
<400> 12723
GCAGCCCCAG CAGTGCGGCA CCTACCGGAG CCATAACGGA AAGCTGAGTA 50
TTGCTATCGG TCATTTTTGC CGGATACACC AGCGTGCGCA CACGCACTTC 100
<210> 12724
<211> 100
<212> DNA
<213> Escherichia coli K-12 MG1655
<220> vals b4259
<222> 2757-2856
<223> vals b4259 U00096 complement(4478550..4481405)
<313> 12724: 2813-2832
<400> 12724
ACCGGAAGCG GTCATCGCGA AAGAGCGTGA GAAGCTGGAA GGCTATGCGG 50
AAGCGAAAGC GAAACTGATT GAACAGCAGG CTGTTATCGC CGCGCTGTAA 100
<210> 12725
<211> 100
<212> DNA
<213> Escherichia coli K-12 MG1655
<220> vals b4259
<222> 2673-2772
<223> vals b4259 U00096 complement(4478550..4481405)
<313> 12725: 2717-2736
<400> 12725
TCTGGCGAAA GAAGTGGCGA AGATTGAAGG TGAAATCAGC CGTATCGAGA 50
ACAAACTGGC GAACGAAGGC TTTGTCGCCC GCGCACCGGA AGCGGTCATC 100
<210> 12726
<211> 100
<212> DNA
<213> Escherichia coli K-12 MG1655
<220> vals b4259
<222> 2582-2681
<223> vals b4259 U00096 complement(4478550..4481405)
<313> 12726: 2616-2635
<400> 12726
AAGGTCCGGT TTCCGTTACG AAGATCATCG ACGGTGCAGA GCTGCTGATC 50
CCGATGGCTG GCCTCATCAA CAAAGAAGAT GAGCTGGCGC GTCTGGCGAA 100
<210> 12727
<211> 100
<212> DNA
<213> Escherichia coli K-12 MG1655
<220> yi5a b3558
<222> 423-522
<223> yi5a b3558 U00096 3718309..3718830
<313> 12727: 443-462
<400> 12727
CCGACGCATT GAGGATCTTG AAAGGCAAGT TCGATTTCTT GAAACGCGGC 50
TTATGTATCT AAAAAAGCTG AAAGCCTTAG CTCATCCCAC GAAAAAGTGA 100
<210> 12728
<211> 100
<212> DNA
<213> Escherichia coli K-12 MG1655
<220> yi5a b3558
<222> 376-475
<223> yi5a b3558 U00096 3718309..3718830
<313> 12728: 401-420
<400> 12728
TCAGTTGATC CAGAAAAAGC GGCATCAGCA TTGGAGCTGT CAAAAGACCG 50
ACGCATTGAG GATCTTGAAA GGCAAGTTCG ATTTCTTGAA ACGCGGCTTA 100

<210> 12729
<211> 100
<212> DNA
<213> Escherichia coli K-12 MG1655
<220> yi5a b3558
<222> 251-350
<223> yi5a b3558 U00096 3718309..3718830
<313> 12729: 310-329
<400> 12729
AGGCTGCTGC TCACTTTATG CTTGCTGGTA GTGGTTCTGT AGCCAGGTGG 50
CTGAAGGTCT ATGAAGAGCG CGGAGAAGCT GGTTTACGCG CGCTCAAGAT 100
<210> 12730
<211> 100
<212> DNA
<213> Escherichia coli K-12 MG1655
<220> dada b1190
<222> 1171-1270
<223> dada b1190 U00096 1236794..1238092
<313> 12730: 1205-1224
<400> 12730
GGTCAGTTGT TAAGCGATCT GCTCTCTGGT CGCACGCCAG CGATCCCATA 50
TGAGGATCTA AGCGTAGCGC GCTACAGCCG TGGATTTACG CCATCACGTC 100
<210> 12731
<211> 100
<212> DNA
<213> Escherichia coli K-12 MG1655
<220> dada b1190
<222> 1117-1216
<223> dada b1190 U00096 1236794..1238092
<313> 12731: 1164-1183
<400> 12731
CTGTGGCTGA ATACCGGTCA CGGCACGCTC GGCTGGACGA TGGCTTGCGG 50
TTCCGGTCAG TTGTTAAGCG ATCTGCTCTC TGGTCGCACG CCAGCGATCC 100
<210> 12732
<211> 100
<212> DNA
<213> Escherichia coli K-12 MG1655
<220> dada b1190
<222> 1053-1152
<223> dada b1190 U00096 1236794..1238092
<313> 12732: 1102-1121
<400> 12732
GACTGGTCTG CGCCCGATGA CGCCAGACGG CACGCCGGTT GTCGGGCGTA 50
CACGCTTTAA AAATCTGTGG CTGAATACCG GTCACGGCAC GCTCGGCTGG 100
<210> 12733
<211> 100
<212> DNA
<213> Escherichia coli K-12 MG1655
<220> b1341 b1341
<222> 1194-1293
<223> b1341 b1341 U00096 complement(1404587..1405879)
<313> 12733: 1247-1266
<400> 12733
TCACGAAGGA AATGAAAGCA TCGATGAACT GTTTAAACGC GTTGATGATG 50
CTTTGTATCG GGCGAAAAAT GATGGACGCA ACCGCGTGCT GGCGGCATAA 100
<210> 12734
<211> 100
<212> DNA
<213> Escherichia coli K-12 MG1655
<220> b1341 b1341
<222> 1115-1214
<223> b1341 b1341 U00096 complement(1404587..1405879)
<313> 12734: 1155-1174
<400> 12734
CTGAAACTAT CCGCGTAAGC GTGGCAAAAG TGAGTATTTC GGGCTTACCA 50
CGCTTTACCG TCAGCATTGG TGTGGCGCAT CACGAAGGAA ATGAAAGCAT 100
<210> 12735
<211> 100
<212> DNA
<213> Escherichia coli K-12 MG1655
<220> b1341 b1341

<222> 1078-1177
<223> b1341 b1341 U00096 complement(1404587..1405879)
<313> 12735: 1124-1143
<400> 12735
CTACCAAGAA CCCCACTGGA TACCGCGCTT TCGCTGGCTG AAACTATCCG 50
CGTAAGCGTG GCAAAAGTGA GTATTTCGGG CTTACCACGC TTTACCGTCA 100
<210> 12736
<211> 100
<212> DNA
<213> Escherichia coli K-12 MG1655
<220> b2070 b2070
<222> 1848-1947
<223> b2070 b2070 U00096 complement(2147061..2149007)
<313> 12736: 1926-1945
<400> 12736
CCGCCTTCAT GCTCCAGCAC GGACCATGCC GTTGATGGAA CCGTTACGTC 50
AGGCAGCAGA AAAACTGGCT CAGGCGCAGG CAGATTTAAG CCGCTGCTGA 100
<210> 12737
<211> 100
<212> DNA
<213> Escherichia coli K-12 MG1655
<220> b2070 b2070
<222> 1821-1920
<223> b2070 b2070 U00096 complement(2147061..2149007)
<313> 12737: 1854-1873
<400> 12737
AGTCGGCGCG ACAGAGTTGC AGCGATTCCG CCTTCATGCT CCAGCACGGA 50
CCATGCCGTT GATGGAACCG TTACGTCAGG CAGCAGAAAA ACTGGCTCAG 100
<210> 12738
<211> 100
<212> DNA
<213> Escherichia coli K-12 MG1655
<220> b2070 b2070
<222> 1729-1828
<223> b2070 b2070 U00096 complement(2147061..2149007)
<313> 12738: 1777-1796
<400> 12738
TTCAGGCCGA ATGAAGCGAT AACGCCAGCA GACAGACAAG CGGTAATGGC 50
GAAAATGACC GCCAAACGAC ATCGGCTGGA ATCGGCGTTG ACAGTCGGCG 100
<210> 12739
<211> 100
<212> DNA
<213> Escherichia coli K-12 MG1655
<220> b1342 b1343
<222> 802-901
<223> b1342 b1343 U00096 1406074..1407057
<313> 12739: 837-856
<400> 12739
TATACAATGT CGTTGATGGC AATGGTCTTT TTACCCAGTA CCTTTCTGAC 50
CGGGTTATTT GGCGTCAACC TTGGTGGGAT CCCTGGCGGC GGGTGGCAAT 100
<210> 12740
<211> 100
<212> DNA
<213> Escherichia coli K-12 MG1655
<220> b1342 b1343
<222> 754-853
<223> b1342 b1343 U00096 1406074..1407057
<313> 12740: 775-794
<400> 12740
GCGGATGAAA TCGCTCAGGT GATGCAGGAA AATTTAGCTC GTCGTACCTA 50
TACAATGTCG TTGATGGCAA TGGTCTTTTT ACCCAGTACC TTTCTGACCG 100
<210> 12741
<211> 100
<212> DNA
<213> Escherichia coli K-12 MG1655
<220> b1342 b1343
<222> 652-751
<223> b1342 b1343 U00096 1406074..1407057
<313> 12741: 699-718
<400> 12741
TGGATGAGCG ATGACCAACG CCGTCGGATG CAGGATATTG CCGATCGCCT 50

TGGGCGCGGC CTTGACGAAA TCGACGCCTG TATAGCACGG ACTGGCGTGA 100
<210> 12742
<211> 100
<212> DNA
<213> Escherichia coli K-12 MG1655
<220> b2071 b2071
<222> 363-462
<223> b2071 b2071 U00096 2149207..2149668
<313> 12742: 414-433
<400> 12742
GCGCGGGGAT AAAATTTACG GCGGTTATAC CATTGATCCT TTATTGGTCA 50
CCTATCCAAA AGAAGAAGCC GACGAGCTAA GAGCAAAATT AGTTCGCTAA 100
<210> 12743
<211> 100
<212> DNA
<213> Escherichia coli K-12 MG1655
<220> b2071 b2071
<222> 236-335
<223> b2071 b2071 U00096 2149207..2149668
<313> 12743: 257-276
<400> 12743
CGGATGTCAC TTATTCTAAC GGCATGTTTA TTGGCGTAAT CAGCAATGAC 50
CCTGGTATTG TTACAAACGT AGAATATGGC CAGGAGTGGA AAATCAAGAA 100
<210> 12744
<211> 100
<212> DNA
<213> Escherichia coli K-12 MG1655
<220> b2071 b2071
<222> 163-262
<223> b2071 b2071 U00096 2149207..2149668
<313> 12744: 220-239
<400> 12744
AAAAAAGATG CCGATACTTT CGCAGTCAAA GCCCCGATAA CCGATCACGG 50
AAGAACCGAG CATTTCTGGC TTACGGATGT CACTTATTCT AACGGCATGT 100
<210> 12745
<211> 100
<212> DNA
<213> Escherichia coli K-12 MG1655
<220> b2072 b2072
<222> 662-761
<223> b2072 b2072 U00096 complement(2149733..2150494)
<313> 12745: 696-715
<400> 12745
ATTTATTACC TGAATTGTTA AAGCAGTTTT TGTCCAGCCC GGCGGTAAAC 50
GAGCGTACGG ATGATGATAA AACGTTGGCA CTGGCGCTGT GGGCAGAGTG 100
<210> 12746
<211> 100
<212> DNA
<213> Escherichia coli K-12 MG1655
<220> b2072 b2072
<222> 601-700
<223> b2072 b2072 U00096 complement(2149733..2150494)
<313> 12746: 645-664
<400> 12746
CATGTGCCTT TCTTTACCCC GTTTTTCAAT GGACTGGCGG CAGCAACGCA 50
GGAACAACTC GATTTATTAC CTGAATTGTT AAAGCAGTTT TTGTCCAGCC 100
<210> 12747
<211> 100
<212> DNA
<213> Escherichia coli K-12 MG1655
<220> b2072 b2072
<222> 584-683
<223> b2072 b2072 U00096 complement(2149733..2150494)
<313> 12747: 608-627
<400> 12747
TGCTGGATAA TTCTCCTCAT GTGCCTTTCT TTACCCCGTT TTTCAATGGA 50
CTGGCGGCAG CAACGCAGGA ACAACTCGAT TTATTACCTG AATTGTTAAA 100
<210> 12748
<211> 100
<212> DNA
<213> Escherichia coli K-12 MG1655

<220> b2073 b2073
<222> 561-660
<223> b2073 b2073 U00096 complement(2150491..2151150)
<313> 12748: 631-650
<400> 12748
ACTGTTTAGC TGGTTATCCA GTTCGCTGCG TTCGGTTTCC CGCTCCACGC 50
CGGGAACGGA AGTTGTGCTG GAAGCGCCAA AAGGCTGGAC GTCAGTGTGA 100
<210> 12749
<211> 100
<212> DNA
<213> Escherichia coli K-12 MG1655
<220> b2073 b2073
<222> 526-625
<223> b2073 b2073 U00096 complement(2150491..2151150)
<313> 12749: 584-603
<400> 12749
CAGCCTTTGC CTCTGCAAGG ATTACAATTC CGTGAACTGT TTAGCTGGTT 50
ATCCAGTTCG CTGCGTTCGG TTTCCCGCTC CACGCCGGGA ACGGAAGTTG 100
<210> 12750
<211> 100
<212> DNA
<213> Escherichia coli K-12 MG1655
<220> b2073 b2073
<222> 503-602
<223> b2073 b2073 U00096 complement(2150491..2151150)
<313> 12750: 533-552
<400> 12750
CGCTGGCACA AATTAGCGTT CGTCAGCCTT TGCCTCTGCA AGGATTACAA 50
TTCCGTGAAC TGTTTAGCTG GTTATCCAGT TCGCTGCGTT CGGTTTCCCG 100
<210> 12751
<211> 100
<212> DNA
<213> Escherichia coli K-12 MG1655
<220> b1345 b1346
<222> 1137-1236
<223> b1345 b1346 U00096 complement(1410024..1411259)
<313> 12751: 1201-1220
<400> 12751
GGGGCATGAA ACTGCGCAGA TGGTGTATGA AATTTACGGT ATGTGGATTG 50
ATGACATGAA CGACGAACAG ATAGCCATGT TGAATGCGCG GTTATCGTAG 100
<210> 12752
<211> 100
<212> DNA
<213> Escherichia coli K-12 MG1655
<220> b1345 b1346
<222> 1070-1169
<223> b1345 b1346 U00096 complement(1410024..1411259)
<313> 12752: 1118-1137
<400> 12752
CGCGGCATAC TTTTGCCTGC TGGCTGTTGA CGGCAGGAGC GAACCCGGCA 50
TTTATAGCCA GCCAAATGGG GCATGAAACT GCGCAGATGG TGTATGAAAT 100
<210> 12753
<211> 100
<212> DNA
<213> Escherichia coli K-12 MG1655
<220> b1345 b1346
<222> 1027-1126
<223> b1345 b1346 U00096 complement(1410024..1411259)
<313> 12753: 1074-1093
<400> 12753
GCAGTAAAAC GTGCTGGTAT TCGCCGCCGT AATCCGTACC ATACGCGGCA 50
TACTTTTGCC TGCTGGCTGT TGACGGCAGG AGCGAACCCG GCATTTATAG 100
<210> 12754
<211> 100
<212> DNA
<213> Escherichia coli K-12 MG1655
<220> b2074 b2075
<222> 1296-1395
<223> b2074 b2075 U00096 2151891..2153285
<313> 12754: 1370-1389
<400> 12754

CGAAGGGGCG AAAGTGGAAG TGGTGGAAGC CCAGAGCGCC ACTACTCCGG 50
AAGAGAAAGC CACCAGCCGC GAATACGCGA AAAAAGGAGC ACGCTCCTGA 100
<210> 12755
<211> 100
<212> DNA
<213> Escherichia coli K-12 MG1655
<220> b2074 b2075
<222> 1264-1363
<223> b2074 b2075 U00096 2151891..2153285
<313> 12755: 1306-1325
<400> 12755
CGCGTGGTGA CAGACGGCAT TGATCGCCTG ACCGAAGGGG CGAAAGTGGA 50
AGTGGTGGAA GCCCAGAGCG CCACTACTCC GGAAGAGAAA GCCACCAGCC 100
<210> 12756
<211> 100
<212> DNA
<213> Escherichia coli K-12 MG1655
<220> b2074 b2075
<222> 1177-1276
<223> b2074 b2075 U00096 2151891..2153285
<313> 12756: 1225-1244
<400> 12756
GAAAACAAGG TCAGCAAACA TCTGGTGACG CCGGGCATTC AGGACAGTCA 50
GAAAGTGGTG ATCCGTGCAG GTATTTCTGC GGGCGATCGC GTGGTGACAG 100
<210> 12757
<211> 100
<212> DNA
<213> Escherichia coli K-12 MG1655
<220> fic b3362
<222> 504-603
<223> fic b3362 U00096 complement(3488498..3489100)
<313> 12757: 576-595
<400> 12757
GGCCTGGAAT CAGGCAAATC AGAGTGGGGC AATGGGGGAT CTCACCGCAC 50
TGCAGATGAT ATTTAGCAAA GTGGTAAGCG AAGCCGGGGA ATCTGAGTAA 100
<210> 12758
<211> 100
<212> DNA
<213> Escherichia coli K-12 MG1655
<220> fic b3362
<222> 431-530
<223> fic b3362 U00096 complement(3488498..3489100)
<313> 12758: 471-490
<400> 12758
CACAGCGGAT CTTCTTCGAG CAACTGGCGA TTCATGCCGG ATATCAACTG 50
AGCTGGCAGG GTATCGAAAA AGAGGCCTGG AATCAGGCAA ATCAGAGTGG 100
<210> 12759
<211> 100
<212> DNA
<213> Escherichia coli K-12 MG1655
<220> fic b3362
<222> 393-492
<223> fic b3362 U00096 complement(3488498..3489100)
<313> 12759: 433-452
<400> 12759
CAACGTGCTG CATCCCTTCC GGGTGGGAAG TGGTCTGGCA CAGCGGATCT 50
TCTTCGAGCA ACTGGCGATT CATGCCGGAT ATCAACTGAG CTGGCAGGGT 100
<210> 12760
<211> 100
<212> DNA
<213> Escherichia coli K-12 MG1655
<220> yohc b2136
<222> 513-612
<223> yohc b2136 U00096 complement(2223064..2223675)
<313> 12760: 561-580
<400> 12760
TAGCTTCTCT AGTTCGACGC TGGCGATTGG CGTGCTGGTA CTGGAAGTGC 50
TGCTCGCCCT AACCGTTATA CTATGGGGCT ACGGTTATCG TTTGTTCTAG 100
<210> 12761
<211> 100
<212> DNA

<213> Escherichia coli K-12 MG1655
<220> yohc b2136
<222> 422-521
<223> yohc b2136 U00096 complement(2223064..2223675)
<313> 12761: 460-479
<400> 12761
CGCTGGTCGG CACGGTGGCG CTGTTTTACA CCGGCTACCT GCTTTACCTT 50
GGTATTCCGA GCTTCCTGAA TATCAACAAA GAGGAAGGCC TTAGCTTCTC 100
<210> 12762
<211> 100
<212> DNA
<213> Escherichia coli K-12 MG1655
<220> yohc b2136
<222> 325-424
<223> yohc b2136 U00096 complement(2223064..2223675)
<313> 12762: 385-404
<400> 12762
CCGTCACTTG CGCACTGCAT GGTCTTCGCG GGCTACGTTG CAACTCCGCT 50
GTTTTTAAGC GGTCTGGTGG CGCTTTATCC ACTGGTCTGG TTGTGTGCGC 100
<210> 12763
<211> 100
<212> DNA
<213> Escherichia coli K-12 MG1655
<220> yohd b2137
<222> 516-615
<223> yohd b2137 U00096 2223785..2224399
<313> 12763: 581-600
<400> 12763
GAAGCACTGG GTCTGGTTGA TTCTGGTCGT GGTTCTGGTG GTGGGCGTGC 50
GCTGGTGGCT GAAACGACGC GGGAAGAAAA AGCCGGATCA TCAGGCGTAA 100
<210> 12764
<211> 100
<212> DNA
<213> Escherichia coli K-12 MG1655
<220> yohd b2137
<222> 494-593
<223> yohd b2137 U00096 2223785..2224399
<313> 12764: 545-564
<400> 12764
TGCACAATCT CGACCAGCAT TTGAAGCACT GGGTCTGGTT GATTCTGGTC 50
GTGGTTCTGG TGGTGGGCGT GCGCTGGTGG CTGAAACGAC GCGGGAAGAA 100
<210> 12765
<211> 100
<212> DNA
<213> Escherichia coli K-12 MG1655
<220> yohd b2137
<222> 284-383
<223> yohd b2137 U00096 2223785..2224399
<313> 12765: 340-359
<400> 12765
GGGCGCAGAA ACTTATCCAA CGCCATCCGT ATCTGTTTGT CATTGGTACG 50
CGCTTTATGT ATGGCTTTCG GGTGATTGGC CCGACGCTGA TTGGTGCCAG 100
<210> 12766
<211> 100
<212> DNA
<213> Escherichia coli K-12 MG1655
<220> yohf b2138
<222> 663-762
<223> yohf b2138 U00096 complement(2224529..2225290)
<313> 12766: 734-753
<400> 12766
GGTGTGGCTT TGTTCGGAGG GCGCAAATTA CACCACCGGG CAGTCGTTGA 50
TAGTGGATGG CGGCTTTATG TTGGCGAATC CACAGTTCAA CCCAGAATAG 100
<210> 12767
<211> 100
<212> DNA
<213> Escherichia coli K-12 MG1655
<220> yohf b2138
<222> 655-754
<223> yohf b2138 U00096 complement(2224529..2225290)
<313> 12767: 697-716

<400> 12767
AGCCTGGTGG TGTGGCTTTG TTCGGAGGGC GCAAATTACA CCACCGGGCA 50
GTCGTTGATA GTGGATGGCG GCTTTATGTT GGCGAATCCA CAGTTCAACC 100
<210> 12768
<211> 100
<212> DNA
<213> Escherichia coli K-12 MG1655
<220> yohf b2138
<222> 586-685
<223> yohf b2138 U00096 complement(2224529..2225290)
<313> 12768: 623-642
<400> 12768
AGCGACGTGA AGCCCGACGC GGAGCCTTCG ATTCCCTTGC GGCGTTTTGG 50
CGCAACGCAT GAGATTGCCA GCCTGGTGGT GTGGCTTTGT TCGGAGGGCG 100
<210> 12769
<211> 100
<212> DNA
<213> Escherichia coli K-12 MG1655
<220> yohg b2139
<222> 1094-1193
<223> yohg b2139 U00096 complement(2225343..2226539)
<313> 12769: 1133-1152
<400> 12769
TCCCCGCGCT GCGTGAGCGG GCCAATGGCC TGTTATTGCA AGGGCAGTGG 50
CTGGATGCCT CCATTCAACT CACTGGTGCG TTGGGCGGGG GGTACAAACG 100
<210> 12770
<211> 100
<212> DNA
<213> Escherichia coli K-12 MG1655
<220> yohg b2139
<222> 1048-1147
<223> yohg b2139 U00096 complement(2225343..2226539)
<313> 12770: 1087-1106
<400> 12770
TTTAACGCGG GCATCATTGC TGGTTCCCGC GTCAGCGAAG CCAGAATCCC 50
CGCGCTGCGT GAGCGGGCCA ATGGCCTGTT ATTGCAAGGG CAGTGGCTGG 100
<210> 12771
<211> 100
<212> DNA
<213> Escherichia coli K-12 MG1655
<220> yohg b2139
<222> 953-1052
<223> yohg b2139 U00096 complement(2225343..2226539)
<313> 12771: 1003-1022
<400> 12771
CAGCCAGTCA GGTTCAGACA CTGGCGGAGA AAAACCAGCA TCAGGCGCAA 50
ATTGAGCGCG ATGCCTTGCG TGTGGTAGGT CTTGCGCAGG CGCGCTTTAA 100
<210> 12772
<211> 100
<212> DNA
<213> Escherichia coli K-12 MG1655
<220> yohh b2140
<222> 192-291
<223> yohh b2140 U00096 complement(2226569..2226859)
<313> 12772: 263-282
<400> 12772
CGCTGCACAA GTTGACACCG CATTACCCAC GGCGCTGAAA ATGGTTGGCC 50
AGACAGCCAA TGGTGGCTGG AGTATCACGA TAATCAACTC ACTTCCTTAA 100
<210> 12773
<211> 100
<212> DNA
<213> Escherichia coli K-12 MG1655
<220> yohh b2140
<222> 153-252
<223> yohh b2140 U00096 complement(2226569..2226859)
<313> 12773: 191-210
<400> 12773
TATGCATGAA ACCCGCCAGG CGTTAAGCCA GCAAACGCCC GCTGCACAAG 50
TTGACACCGC ATTACCCACG GCGCTGAAAA TGGTTGGCCA GACAGCCAAT 100
<210> 12774
<211> 100

<212> DNA
<213> Escherichia coli K-12 MG1655
<220> yohh b2140
<222> 55-154
<223> yohh b2140 U00096 complement(2226569..2226859)
<313> 12774: 106-125
<400> 12774
CACCTTTTCT TACCTGATTA TTCCCCCATG AATCGTGATT CCTTTTATCC 50
AGCCATCGCC TGTTTTCCGC TGTTACTGAT GCTGGCCGGG TGTGCGCCTA 100
<210> 12775
<211> 100
<212> DNA
<213> Escherichia coli K-12 MG1655
<220> yohi b2141
<222> 848-947
<223> yohi b2141 U00096 complement(2227458..2228405)
<313> 12775: 895-914
<400> 12775
AATACGATGA AGCAACGGAA TTATTTCAGC ATGTTCGGGT GTTGAATAAT 50
TCCCCTGATA TTGCAAGGGC TATTCAGGCA ATTGATATCG AGAAACTCTA 100
<210> 12776
<211> 100
<212> DNA
<213> Escherichia coli K-12 MG1655
<220> yohi b2141
<222> 758-857
<223> yohi b2141 U00096 complement(2227458..2228405)
<313> 12776: 777-796
<400> 12776
TGCAAAAATA TACCCGTCTG GAAAAGCAGG GCGATACCGG GTTATATCAC 50
GTTGCGCGGA TTAAACAGTG GTTGAGTTAT TTGCGTAAAG AATACGATGA 100
<210> 12777
<211> 100
<212> DNA
<213> Escherichia coli K-12 MG1655
<220> yohi b2141
<222> 659-758
<223> yohi b2141 U00096 complement(2227458..2228405)
<313> 12777: 707-726
<400> 12777
CAGTGATGAT TGGTCGCGGG GCGCTCAATA TTCCCAACCT GAGCCGGGTG 50
GTAAAATATA ACGAACCGCG AATGCCGTGG CCGGAGGTGG TTGCTTTGCT 100
<210> 12778
<211> 100
<212> DNA
<213> Escherichia coli K-12 MG1655
<220> yohj b2142
<222> 300-399
<223> yohj b2142 U00096 2228644..2229042
<313> 12778: 361-380
<400> 12778
TGCAGTCAGT ACGCTGGTGG TTTTTCTGGT GGTGAGCTGG AGTTCGCAAC 50
TGGTACACGG TGAACGTAAA GTCGTAGGTC AGAAAGGATC AGAAGAATGA 100
<210> 12779
<211> 100
<212> DNA
<213> Escherichia coli K-12 MG1655
<220> yohj b2142
<222> 219-318
<223> yohj b2142 U00096 2228644..2229042
<313> 12779: 280-299
<400> 12779
ATTGTTTGTG CCGATTGGCG TAGGCGTCAT GCAATATTTT GATTTGCTCC 50
GCGCACAGTT TGGCCCGGTA GTGGTTTCCT GTGCAGTCAG TACGCTGGTG 100
<210> 12780
<211> 100
<212> DNA
<213> Escherichia coli K-12 MG1655
<220> yohj b2142
<222> 126-225
<223> yohj b2142 U00096 2228644..2229042

<313> 12780: 181-200
<400> 12780
GATGCTGATC CTGTTTGTCC TGCTGGCCTT GCAAATTCTT CCGGCAAAAT 50
GGGTCAATCC GGGGTGCTAC GTACTGATTC GCTATATGGC GCTATTGTTT 100
<210> 12781
<211> 100
<212> DNA
<213> Escherichia coli K-12 MG1655
<220> yohk b2143
<222> 597-696
<223> yohk b2143 U00096 2229039..2229734
<313> 12781: 673-692
<400> 12781
GGAAGGTGCA TTTAGTTCGC TAGCGCTGGT GTTATGCGGG ATAATTACTT 50
CGCTGATCGC ACCGTTCCTT TTCCCGATTA TTCTGGCAGT AATGGGCTAA 100
<210> 12782
<211> 100
<212> DNA
<213> Escherichia coli K-12 MG1655
<220> yohk b2143
<222> 485-584
<223> yohk b2143 U00096 2229039..2229734
<313> 12782: 545-564
<400> 12782
GCCATACATT GCTTAATGCG ATGCGTATTC GTACCAAAGC TGCGCGCGGT 50
CTGGCAATGG GGACTGCCTC GCACGCCCTC GGTACGGCGC GCTGCGCCGA 100
<210> 12783
<211> 100
<212> DNA
<213> Escherichia coli K-12 MG1655
<220> yohk b2143
<222> 430-529
<223> yohk b2143 U00096 2229039..2229734
<313> 12783: 460-479
<400> 12783
CCGGCAATCA GCGCAGTTTG CGTGATTTTC GTCGGCATCC TCGGCGCGGT 50
ATTTGGCCAT ACATTGCTTA ATGCGATGCG TATTCGTACC AAAGCTGCGC 100
<210> 12784
<211> 100
<212> DNA
<213> Escherichia coli K-12 MG1655
<220> yohl b2106
<222> 170-269
<223> yohl b2106 U00096 complement(2183544..2183816)
<313> 12784: 199-218
<400> 12784
TTAAAGGTCA TCTGACGGAA CACATCGTTC ACCAGGGGGA TGAGCTAAAA 50
CGTGAAGAAG ATCTGGATGT CGTTCTGAAG GTGCTGGATT CATATATCAA 100
<210> 12785
<211> 100
<212> DNA
<213> Escherichia coli K-12 MG1655
<220> yohl b2106
<222> 140-239
<223> yohl b2106 U00096 complement(2183544..2183816)
<313> 12785: 155-174
<400> 12785
GCGCGGTAAA CGGTCTGATG CGGGAAGTGA TTAAAGGTCA TCTGACGGAA 50
CACATCGTTC ACCAGGGGGA TGAGCTAAAA CGTGAAGAAG ATCTGGATGT 100
<210> 12786
<211> 100
<212> DNA
<213> Escherichia coli K-12 MG1655
<220> yohl b2106
<222> 41-140
<223> yohl b2106 U00096 complement(2183544..2183816)
<313> 12786: 83-102
<400> 12786
GTGCCAGTAA GATTCAGGGC CAGGTCGTGG CGCTCAAGAA AATGCTCGAC 50
GAGCCGCACG AATGCGCTGC AGTTTTACAA CAGATTGCTG CTATCCGTGG 100
<210> 12787

<211> 100
<212> DNA
<213> Escherichia coli K-12 MG1655
<220> yohm yohM
<222> 726-825
<223> yohm yohM U00096 2183937..2184761
<313> 12787: 793-812
<400> 12787
TAACACTCTC GCTAAACGCG CCCCCTATTT TTCCAGTCTG TTGATTGGCT 50
TAGTCGGTGT GTATATGGGC GTACATGGCT TCATGGGCAT AATGCGATAA 100
<210> 12788
<211> 100
<212> DNA
<213> Escherichia coli K-12 MG1655
<220> yohm yohM
<222> 715-814
<223> yohm yohM U00096 2183937..2184761
<313> 12788: 748-767
<400> 12788
TGGAGCGGAT TTAACACTCT CGCTAAACGC GCCCCCTATT TTTCCAGTCT 50
GTTGATTGGC TTAGTCGGTG TGTATATGGG CGTACATGGC TTCATGGGCA 100
<210> 12789
<211> 100
<212> DNA
<213> Escherichia coli K-12 MG1655
<220> yohm yohM
<222> 680-779
<223> yohm yohM U00096 2183937..2184761
<313> 12789: 705-724
<400> 12789
GCGCAGCAAT CAGCGTTCAG CAGGTCGCAA AACGCTGGAG CGGATTTAAC 50
ACTCTCGCTA AACGCGCCCC CTATTTTTCC AGTCTGTTGA TTGGCTTAGT 100
<210> 12790
<211> 100
<212> DNA
<213> Escherichia coli K-12 MG1655
<220> dadx dadX
<222> 937-1036
<223> dadx dadX U00096 1238102..1239172
<313> 12790: 985-1004
<400> 12790
CAGGCGGGTA TTGGTACGCC GGTTGAGCTG TGGGGCAAGG AGATCAAAAT 50
TGATGATGTC GCCGCCGCTG CCGGAACGGT GGGCTATGAG TTGATGTGCG 100
<210> 12791
<211> 100
<212> DNA
<213> Escherichia coli K-12 MG1655
<220> dadx dadX
<222> 784-883
<223> dadx dadX U00096 1238102..1239172
<313> 12791: 812-831
<400> 12791
GAACAGCGAA TCGGCATTGT CGCCGCAGGG TACGCCGACG GTTATCCGCG 50
CCACGCGCCT ACCGGTACCC CTGTTTTAGT GGACGGCGTG CGCACCATGA 100
<210> 12792
<211> 100
<212> DNA
<213> Escherichia coli K-12 MG1655
<220> dadx dadX
<222> 709-808
<223> dadx dadX U00096 1238102..1239172
<313> 12792: 731-750
<400> 12792
AGTGAGATTA TTGGTGTCCA GACGCTAAAA GCGGGCGAGC GTGTGGGCTA 50
CGGCGGTCGC TATACTGCGC GCGATGAACA GCGAATCGGC ATTGTCGCCG 100
<210> 12793
<211> 100
<212> DNA
<213> Escherichia coli K-12 MG1655
<220> fis b3262
<222> 178-277

<223> fis b3262 U00096 3408908..3409204
<313> 12793: 220-239
<400> 12793
CAGCCCCTGT TGGACATGGT GATGCAATAC ACCCGTGGTA ACCAGACCCG 50
TGCTGCGCTG ATGATGGGCA TCAACCGTGG TACGCTGCGT AAAAAATTGA 100
<210> 12794
<211> 100
<212> DNA
<213> Escherichia coli K-12 MG1655
<220> fis b3262
<222> 162-261
<223> fis b3262 U00096 3408908..3409204
<313> 12794: 181-200
<400> 12794
ACTGGCTGAA GTAGAACAGC CCCTGTTGGA CATGGTGATG CAATACACCC 50
GTGGTAACCA GACCCGTGCT GCGCTGATGA TGGGCATCAA CCGTGGTACG 100
<210> 12795
<211> 100
<212> DNA
<213> Escherichia coli K-12 MG1655
<220> fis b3262
<222> 31-130
<223> fis b3262 U00096 3408908..3409204
<313> 12795: 92-111
<400> 12795
CTGACCGTTT CTACCGTTAA CTCTCAGGAT CAGGTAACCC AAAAACCCCT 50
GCGTGACTCG GTTAAACAGG CACTGAAGAA CTATTTTGCT CAACTGAATG 100
<210> 12796
<211> 100
<212> DNA
<213> Escherichia coli K-12 MG1655
<220> b2080 b2081
<222> 273-372
<223> b2080 b2081 U00096 2163172..2163543
<313> 12796: 346-365
<400> 12796
CGGCTCCAGC CAGATGTACG CCACCGCGCA ATCTCGTGAA ACCGGAATTG 50
CCTCCGTTAA AGCCAATGGC ACAAGCCAGA CGGTGAAAGA CAATACGTAA 100
<210> 12797
<211> 100
<212> DNA
<213> Escherichia coli K-12 MG1655
<220> b2080 b2081
<222> 247-346
<223> b2080 b2081 U00096 2163172..2163543
<313> 12797: 303-322
<400> 12797
CTGAAAGCCG CTAATCATCA AATTATCGGC TCCAGCCAGA TGTACGCCAC 50
CGCGCAATCT CGTGAAACCG GAATTGCCTC CGTTAAAGCC AATGGCACAA 100
<210> 12798
<211> 100
<212> DNA
<213> Escherichia coli K-12 MG1655
<220> b2080 b2081
<222> 227-326
<223> b2080 b2081 U00096 2163172..2163543
<313> 12798: 270-289
<400> 12798
ACGGCAAATT CTATTTCAAT CTGAAAGCCG CTAATCATCA AATTATCGGC 50
TCCAGCCAGA TGTACGCCAC CGCGCAATCT CGTGAAACCG GAATTGCCTC 100
<210> 12799
<211> 100
<212> DNA
<213> Escherichia coli K-12 MG1655
<220> b1354 b1354
<222> 78-177
<223> b1354 b1354 U00096 1417192..1417368
<313> 12799: 96-115
<400> 12799
TTCCATCCTG GTTGGGGAAC ACGCCGCACA CCAGGGATTC GTTGTTGCCG 50
AGGTCGATTT TTTGCATTTT GCGAATCTCA CATCTTGTTG CTACGTATAG 100

<210> 12800
<211> 100
<212> DNA
<213> Escherichia coli K-12 MG1655
<220> b1354 b1354
<222> 1-100
<223> b1354 b1354 U00096 1417192..1417368
<313> 12800: 41-60
<400> 12800
GTGCTTCTCC AACCATCGGC GCGCACCAGT TTCGGTTTTA AATGTTTTGC 50
TTTTGGTATA CGTCATGGCA GTGAACGTTC CATCCTGGTT GGGGAACACG 100
<210> 12801
<211> 100
<212> DNA
<213> Escherichia coli K-12 MG1655
<220> b2083 b2083
<222> 48-147
<223> b2083 b2083 U00096 complement(2165624..2165770)
<313> 12801: 118-137
<400> 12801
TAAGGTGACA CATTTTCTGA ATAATAGCGG CTTCACGACG TCATTAGAGC 50
TTGAGGTCAG GCTTTCTGAT GTGGGGTACG AAACAGAAGA TAATGAGTGA 100
<210> 12802
<211> 100
<212> DNA
<213> Escherichia coli K-12 MG1655
<220> b2083 b2083
<222> 48-147
<223> b2083 b2083 U00096 complement(2165624..2165770)
<313> 12802: 107-126
<400> 12802
TAAGGTGACA CATTTTCTGA ATAATAGCGG CTTCACGACG TCATTAGAGC 50
TTGAGGTCAG GCTTTCTGAT GTGGGGTACG AAACAGAAGA TAATGAGTGA 100
<210> 12803
<211> 100
<212> DNA
<213> Escherichia coli K-12 MG1655
<220> b2083 b2083
<222> 19-118
<223> b2083 b2083 U00096 complement(2165624..2165770)
<313> 12803: 61-80
<400> 12803
GTCATAGACG AGCAGGACTG GACAATCACT AAGGTGACAC ATTTTCTGAA 50
TAATAGCGGC TTCACGACGT CATTAGAGCT TGAGGTCAGG CTTTCTGATG 100
<210> 12804
<211> 100
<212> DNA
<213> Escherichia coli K-12 MG1655
<220> b1355 b1356
<222> 81-180
<223> b1355 b1356 U00096 complement(1417346..1417525)
<313> 12804: 159-178
<400> 12804
TTGTTGCGGT ATCACTTACA ACCAGGCTGT GCAGGCTTTA CGCAGCAACG 50
ACTGGGATCT CTGGCAGGCA GAAGTCGCTA TACGTAGCAA CAAGATGTGA 100
<210> 12805
<211> 100
<212> DNA
<213> Escherichia coli K-12 MG1655
<220> b1355 b1356
<222> 0-99
<223> b1355 b1356 U00096 complement(1417346..1417525)
<313> 12805: 29-48
<400> 12805
GTGCAGCAGT TAGTTCCGCC ACCCGGCGTT AAGGGGAGAG ATAAGATGGT 50
GCATTACGAA GTAGTTCAGT ATTTGATGGA TTGTTGCGGT ATCACTTAC 100
<210> 12806
<211> 100
<212> DNA
<213> Escherichia coli K-12 MG1655
<220> b2084 b2084

<222> 93-192
<223> b2084 b2084 U00096 complement(2165757..2166023)
<313> 12806: 146-165
<400> 12806
TGAAAGGCAT CGTGCTATGA AGGAGGATTC TATCGATGTG GTCAATGGAA 50
GACGGTTACC AGAGATAGGG CTTATGCATA AAAAAATAAG CCCGTGTAGC 100
<210> 12807
<211> 100
<212> DNA
<213> Escherichia coli K-12 MG1655
<220> b2084 b2084
<222> 40-139
<223> b2084 b2084 U00096 complement(2165757..2166023)
<313> 12807: 102-121
<400> 12807
TGCGCGTTCT GGCTGTGCGT AGCCGGAACC TGTGGGAGCA CGATGCCGAT 50
AAGTGAAAGG CATCGTGCTA TGAAGGAGGA TTCTATCGAT GTGGTCAATG 100
<210> 12808
<211> 100
<212> DNA
<213> Escherichia coli K-12 MG1655
<220> b2085 b2085
<222> 279-378
<223> b2085 b2085 U00096 complement(2166011..2166388)
<313> 12808: 351-370
<400> 12808
CTGTGTTACT CAGGGCAAAA AAATGCAAAT AGTTGAGCTG AAGACAGAAG 50
GTGTAGGATT ACATGGTGTC GCTCGTCTGA CATTCAAATG TGGAGAGTGA 100
<210> 12809
<211> 100
<212> DNA
<213> Escherichia coli K-12 MG1655
<220> b2085 b2085
<222> 279-378
<223> b2085 b2085 U00096 complement(2166011..2166388)
<313> 12809: 327-346
<400> 12809
CTGTGTTACT CAGGGCAAAA AAATGCAAAT AGTTGAGCTG AAGACAGAAG 50
GTGTAGGATT ACATGGTGTC GCTCGTCTGA CATTCAAATG TGGAGAGTGA 100
<210> 12810
<211> 100
<212> DNA
<213> Escherichia coli K-12 MG1655
<220> b2085 b2085
<222> 279-378
<223> b2085 b2085 U00096 complement(2166011..2166388)
<313> 12810: 334-353
<400> 12810
CTGTGTTACT CAGGGCAAAA AAATGCAAAT AGTTGAGCTG AAGACAGAAG 50
GTGTAGGATT ACATGGTGTC GCTCGTCTGA CATTCAAATG TGGAGAGTGA 100
<210> 12811
<211> 100
<212> DNA
<213> Escherichia coli K-12 MG1655
<220> b2086 b2087
<222> 801-900
<223> b2086 b2087 U00096 2166734..2167633
<313> 12811: 864-883
<400> 12811
TCTTGATGGC GAACCGTTGA GTGGGCAAAA TTTTCATATT GAAATACTTC 50
CGGCAGCGTT GCGTTGTCGA TTACCACCAG ATTGTCCACT GTTGCGTTAA 100
<210> 12812
<211> 100
<212> DNA
<213> Escherichia coli K-12 MG1655
<220> b2086 b2087
<222> 749-848
<223> b2086 b2087 U00096 2166734..2167633
<313> 12812: 781-800
<400> 12812
AAGGCGCTTC GTCGTGGTTT GATATTCAGG CACCACACGA CATCACCTTT 50

AATCTTGATG GCGAACCGTT GAGTGGGCAA AATTTTCATA TTGAAATACT 100
<210> 12813
<211> 100
<212> DNA
<213> Escherichia coli K-12 MG1655
<220> b2086 b2087
<222> 660-759
<223> b2086 b2087 U00096 2166734..2167633
<313> 12813: 686-705
<400> 12813
GCTGCAACTG CGCATTTTTA CCGGCGATGA AATACTTCCG GCTCTCGTAT 50
CAACCTTAAA ATCTGACGAA GATAACCCGA ATATTATCGA AGGCGCTTCG 100
<210> 12814
<211> 100
<212> DNA
<213> Escherichia coli K-12 MG1655
<220> b2088 b2089
<222> 121-220
<223> b2088 b2089 U00096 2168249..2168557
<313> 12814: 152-171
<400> 12814
GAACTCAGCC TGTATGAATC ACAACTCTAC AACTGGCGCA GTAAACAGCA 50
AAATCAGCAG ACGTCTTCTG AACGTGAACT GGAGATGTCT ACCGAGATTG 100
<210> 12815
<211> 100
<212> DNA
<213> Escherichia coli K-12 MG1655
<220> b2088 b2089
<222> 63-162
<223> b2088 b2089 U00096 2168249..2168557
<313> 12815: 114-133
<400> 12815
ATTTCGCAGT GAAGCCCTGA AGCTTGCTGA ACGCATCGGT GTTACTGCCG 50
CAGCCCGTGA ACTCAGCCTG TATGAATCAC AACTCTACAA CTGGCGCAGT 100
<210> 12816
<211> 100
<212> DNA
<213> Escherichia coli K-12 MG1655
<220> b2088 b2089
<222> 37-136
<223> b2088 b2089 U00096 2168249..2168557
<313> 12816: 73-92
<400> 12816
AAACCCCGTA AACAGCATTC GCCTGAATTT CGCAGTGAAG CCCTGAAGCT 50
TGCTGAACGC ATCGGTGTTA CTGCCGCAGC CCGTGAACTC AGCCTGTATG 100

## Beschreibung

### Die Erfindung betrifft einen Biochip.

Es ist bekannt, mit Biochips Nukleinsäuren zu analysieren, um z.B. zelluläre Funktionen und deren Regulation zu verstehen. Auf Biochips sind Sensormoleküle in geordneten Rastern auf Oberflächen angeordnet. Die Art wie diese Raster erstellt werden, und die verschiedenen Anwendungsmöglichkeiten variieren jedoch so stark, dass von einer Vielzahl verschiedener Techniken gesprochen werden kann. Die zu untersuchenden Nukleinsäuren, meist Ribonukleinsäuren (RNA), werden aus Gewebe oder Zellen isoliert. Untersucht man RNA, kann anschließend eine Anreicherung der mRNA erfolgen. Das Umschreiben der RNA bzw. mRNA in cDNA erfolgt mittels einer reversen Transkriptase. Dieser Schritt beinhaltet herkömmlicherweise eine Markierung der späteren Hybridisierungsprobe, so dass nach der Hybridisierung detektiert werden kann. Bei begrenzter Probenmenge kann ein Amplifikatiosschritt des Ausgangsmaterials über PCR (Polymerase Chain Reaction ) oder Invitrotranskription eingefügt werden. Daran schließt sich die Datenanalyse an, die bei Mikroarray-Hybridisierungen in Abhängigkeit von der lntegrationsdichte häufig den zeitaufwendigsten Schritt darstellt. Diese Biochip-Technologien haben den Vorteil, dass sie die Verfahren der Probenaufbereitung über die Hybridisierung und Detektion bis hin zur Datenanalyse integrieren. Durch die weitest gehende Parallelisierung von Analyseschritten kann der Probendurchsatz erhöht werden (Medizinische Genetik Nr.1 März 1999 Jahrgang 11 S.1-32).

Ein Oligonukleotid-Chip besteht aus einem Träger und den für das jeweilige Experiment notwendigen, darauf fixierten Molekülen. Bei der Herstellung werden diese Moleküle entweder in situ mittels photolithographischer Techniken unter Verwendung physikalischer Masken auf der Matrix synthetisiert, oder durch verschiedene Verfahren aufgedruckt, wie beispielsweise das Kontaktverfahren mit Hilfe von Kapillarnadeln oder die Nichtkontaktverfahren auf Basis von piezoelektronischen Tintenstrahldüsen. Die Herstellung von aufgedruckten DNA-Mikro-Arrays gliedert sich in die Aktivierung bzw. die Beschichtung der festen Chipmatrix, an die Biomoleküle über eine geeignete Kopplungschemie fixiert werden. Grundlegende Patentanmeldungen zu DNA-Chips sind z. B. EP 0 619 321 A, EP 0 373 203 A, EP 0 476 014 A und EP 0 386 229 A.

Im allgemeinen werden für Nukleinsäureanalytik verschiedene Fluoreszenzfarbstoffe als Markierungsagenzien verwendet. Andere Detektionsmöglichkeiten ergeben sich durch die Verwendung von radioaktiven Markierungen, chemilumineszenten oder anderweitigen Markierungen, durch Messung der anhand dieser Markeirungen jeweils detektierbaren Signale. Ferner sind auch Detektionsmöglichkeiten mittels Impedanzmessungen oder anderer physikalischer Messmethoden gegeben. Es kann prinzipiell auch eine Detektion mit der MALDI-TOF-Analyse (Mass Absorption Laser Desorption lonization-Time of Flight Spektrometrie) auf Basis der Massenspektrometrie erfolgen, bei der auf Markierungszusätze verzichtet werden kann.

Aus der EP 0 923 050 A ist ein Computer-System zur Auswertung von DNA-Chips bekannt.

Eine wesentliche Grundlage der Biochip Technologie ist die Hybridisierung. Einzelstränge der Desoxyribo- (DNA) oder Ribo- (RNA) nukleinsäure sind aus Basen wie Adenin (A), Thymin (T), Cytosin (C), Guanin (G), Uracil (U) oder Inosin (I) zusammengesetzt. Sie sind in der Lage, zu Doppelsträngen zu hybridisieren. Dabei werden A mit T, C mit G über Wasserstoffbrücken verknüpft. Es bilden sich sogenannte Basenpaare, z.B. AT oder CG. Darüber hinaus können nicht komplementäre Basenpaare vorliegen, z. B. AG, GU.

Bringt man unter geeigneten Bedingungen zwei verschiedene Einzelstränge zusammen, die genügend benachbarte komplementäre Basenpaare enthalten, hybridisieren sie zu einer doppelsträngigen Nukleinsäure. Unter geeigneten Bedingungen erhält man so DNA/DNA-, DNA/RNA- und RNA/RNA-Hybride.

Die Hybridisierung von Nukleinsäuren findet Anwendung in der Detektion bestimmter Nukleinsäuresequenzen aus Probenmaterial, das zuvor aufbereitet werden muss. Dabei werden zur gesuchten Nukleinsäuresequenz komplementäre Nukleotidsequenzen bereitgestellt, die mit der gesuchten Sequenz hybridisieren. Die Bildung eines solchen Doppelstrangs muss dann mittels geeigneter Methoden nachgewiesen werden. Eine solche Nukleotidsequenz, die an der Oberfläche eines Trägers angebracht ist, kann aus mittels PCR-Techniken selektiv amplifizierten DNA- oder cDNA-Abschnitten bestehen, die für ein bestimmtes Transkript eines Organismus spezifisch sind.

Ein cDNA-Chip oder eine Nukleinsäuresequenzen aufweisender Biochip, bei dem mittels PCR-Techniken gewonnenes Sondenmaterial auf dem Träger aufgebracht ist, weist folgende Nachteile auf:
- die Sequenz ist in der Regel nur über einen geringen Teil ihrer Länge selektiv,
- durch die PCR-Techniken kommt es immer wieder vor, dass die Sequenzen stellenweise nicht vorgesehene Nukleotide beinhalten,
- die physikalischen Parameter der verschiedenen Sequenzen auf einem solchen Biochip, wie z.B. die Schmelztemperatur lassen sich nur in einem geringen Rahmen vereinheitlichen, da die Längen der jeweiligen Amplifikate nicht immer frei gewählt werden können,
- wegen fehleingebauter Nukleotide und nicht optimal vereinheitlichter physikalischer Eigenschaften der Sonden wird unterschiedlich viel Probenmaterial gebraucht, um detektierbare Signale zu erhalten,
- erhaltene Signale sind wegen der erwähnten Fehlerquellen schlecht miteinander zu vergleichen, da die Fehler von Sonde zu Sonde unterschiedliche Ausmasse annehmen können,
- Experimente, bei denen solche Biochips für die Erstellung von Genexpressionsmustern verwendet werden sollen, führen wegen der aufgezählten Nachteile zu wenig exakten Ergebnissen.

Biochips, auf denen (synthetische) Oligonukleotide aufgebracht sind, weisen demgegenüber folgende Vorteile auf:
- die Länge der Sequenzen ist frei variierbar,
- die Sequenz kann daher über eine maximale Länge selektiv sein,
- solche Oligonukleotidsequenzen sind in der Regel kürzer als z.B. aus biologischem Material stammende, mittels PCR amplifizierte Sequenzen. Da eine Sonde viele identische Sondenmoleküle aufweist, und kurze Sequenzen gleichbedeutend sind mit kurzen Sondenmolekülen, reagieren Sonden mit Oligonukleotidsequenzen zuverlässiger mit Probenmaterial als längere Sondenmoleküle, die eher dazu neigen mit sich selbst oder dem Nachbarmolekül zu hybridisieren oder sich aufgrund ihrer Länge ungleichmässig auszurichten. Hybridisierungsexperimente mit Oligonukleotiden sind daher sicherer, einheitlicher und unkomplizierter.

Oiigonukleotidchips existieren mit in situ synthetisierten Oligonukleotiden. Ein solches Verfahren ist sehr aufwendig. Dabei werden die Oligonukleotide Schritt für Schritt aufgebaut, wobei mittels Schablonen ein Nukleotid nach dem anderen an das entstehende Oligonukleotid angereiht wird. Je länger das Molekül ist, desto flexibler wird es, und desto unzuverlässiger scheint im Gegenzug die Schablone zu funktionieren, so dass nach dieser Methode hergestellte Biochips nur mit Oligonukleotiden mit vergleichsweise wenigen Basen zuverlässig hergestellt werden.

Längere Oligonukleotide lassen sich jedoch auch ex situ synthetisieren, auf einen Träger aufspotten und kovalent oder in anderer Weise an diesem befestigen. So hergestellte Biochips weisen die oben genannten Nachteile nicht auf und sind für einzelne Anwendungen im Handel erhältlich. Eine ex situ oder ex vivo Synthese zeichnet sich durch die Möglichkeit einer Qualitätskontrolle aus, die z.B. mittels MALDI-TOF (Matrixassistierte Laser Desorption Ionisation Flugzeit Massenspektrometrie) erfolgen kann. Hierdurch kann im Gegensatz zu einer direkten Synthese auf dem Träger (z.B. mittels photolithographischer Techniken) eine hohe Reinheit der Oligonukleotide garantiert werden.

In Hybridisierungsexperimenten ist entweder die zu untersuchende Nukleinsäure-Probe oder die zur gesuchten Nukleinsäuresequenz komplementäre Oligonukleotid- oder Nukleinsäuresequenz an einer festen Phase immobilisiert. Nach erfolgreicher Hybridisierung kann das Produkt gewaschen werden. Der Nachweis erfolgt i.d.R. über bei der Hybridisierung oder später eingebaute Radioisotope, Färbemittel oder andere Markierungsagenzien, die letztendlich die spätere Detektion eines Signals auf physikalischem Wege ermöglichen (C. R. Cantor, C. L. Smith, Genomics, John Wiley and Sons, New York 1999, S. 67). Auf diese Weise können viele Einzel-Untersuchungen auf sogenannten Chips parallel ausgeführt werden, was den möglichen Probendurchsatz pro Zeiteinheit erhöht.

Die Analyse von biologischem Probenmaterial durch Reaktion mit Biochips führt zu an der Chip-Oberfläche immobilisierten Molekülen, deren Topologie und Quantitäten auf dem Biochip Aussagen über die Menge einer bestimmten Nukleinsäuresequenz, eines Gens oder eines Genprodukts in diesem Probenmaterial ermöglichen. Daraus lassen sich biologische Informationen z.B. über Genexpressionen und Mutationen in einem Genom ableiten. Diese haben einen potentiellen, beschleunigenden Nutzen bei der Entwicklung neuer Medikamente, in der Diagnose und anderen Bereichen der biomedizinischen und biochemischen Forschung (M. Schena, R. W. Davis, Genes, genomes and chips in DNA Microarrays, Hrsg. M. Schena, Oxford University Press 1999).

Um eine Hybridisierung fehlerfrei detektieren zu können, muss diese unter den jeweils üblichen Bedingungen hinreichend stabil und spezifisch sein.

Die Stabilität eines Nukleinsäuredoppelstrangs drückt sich in seiner Schmelztemperatur aus. Die Schmelztemperatur ist die Temperatur, bei der ein potentielles Hybrid zu gleichen Teilen doppelsträngig und einzelsträngig vorliegt. Sie hängt von der Art und Zusammensetzung der Basenpaare ab, aus denen ein Doppelstrang zusammengesetzt ist, sowie der Art und Zusammensetzung des Mediums, in dem der Doppelstrang vorliegt.

Die Spezifität einer Hybridisierung, also der Grad an Hybriden, die keine Fehlbindung im Sinne von nicht komplementären Basenpaaren enthalten, hängt eng mit der Stabilität der gebildeten Hybride zusammen. Benachbarte komplementäre Basenpaare leisten in Abhängigkeit von ihrer jeweiligen Art und Zusammensetzung einen Beitrag zur Gesamtstabilität eines Hybrids. Liegt innerhalb eines Hybrids eine Fehlbindung im Sinne eines nicht komplementären Basenpaares vor, so fehlt im Vergleich zu einem fehlerfrei ausgebildeten Hybrid der Beitrag eines Basenpaares sowie zweier kooperativer Wechselwirkungen zweier benachbarter gebundener Basenpaare zur Bildungsenthalpie jener fehlgebildeten Hybride. Dies drückt sich in der niedrigeren Schmelztemperatur der fehlgebildeten Hybride gegenüber den Hybriden ohne Fehlbindung aus. So weist z.B. ein vollständig komplementäres DNA/DNA-14mer gegenüber einem 14mer mit einer Fehlbindung eine um ca. 7°C erhöhte Schmelztemperatur auf (C. R. Cantor, C. L. Smith, Genomics, John Wiley and Sons, New York 1999, Kap.3). Ein Anteil von 1% Fehlbindung in einem gegebenen Hybrid erniedrigen dessen Schmelztemperatur um ca. 1°C. Deshalb ist die für die Durchführung einer Hybridisierung gewählte Temperatur neben der Art und Zusammensetzung des Reaktionsmediums ein Kontrollparameter für die Spezifität einer Hybridisierung (R.J. Britten, E. H. Davidson, Hybridization strategy in Nucleic acid hybridization: a practical approach, Hrsg. B. D. James, S. J. Higgins, IRL Press 1985).

Ferner hängt die Spezifität einer Hybridisierung von der Länge der in das Hybrid eingehenden Basenfragmente ab. Je mehr benachbarte Basenpaare hybridisieren, desto schneller verläuft die eigentliche Hybridisierung. Eine Fehlbindung hingegen verlangsamt die Bildung eines Hybrids. Dieser Geschwindigkeitsunterschied wächst mit der Anzahl der ein Hybrid bildenden Basenpaare. 10% Fehlbindung in einem gegebenen Hybrid verlangsamen die Reaktionsgeschwindigkeit i.d.R. um die Hälfte (R.J. Britten, E. H. Davidson, Hybridization strategy in Nucleic acid hybridization: a practical approach, Hrsg. B. D. James, S. J. Higgins, IRL Press 1985).

Können zwei DNA-Einzelstränge acht oder mehr Basenpaare bilden, gilt wegen der realisierbaren Spezifität und der Bindungsstärke der einzelnen Basenpaare die Bildung fehlerfreier Doppelstränge in der überwiegenden Zahl der Hybridisierungen als sicher (C. R. Cantor, C. L. Smith, Genomics, John Wiley and Sons, New York 1999, S. 11).

Anwendungsbeispiele für Hybridisierungsexperimente sind z.B. der Nachweis von *Mycoplasma Pneumoniae* (EP 0 173 920 A2), die Detektion des Proteins *human telomerase reverse transkriptase (hTRT)* (EP 0 841 396 A1), und die Detektion von bestimmten Polymorphismen (z.B. EP 0812 922 A2).

Ein weiterer wichtiger Anwendungsbereich von Biochips ist die Analyse von Genexpressionsmustern. Genexpressionsmuster erlauben eine Aussage über die Expression bestimmter Gene eines Organismus in Abhängigkeit von Parametern, die je nach Experiment variiert werden. Es ist zu diesem Zweck grundsätzlich erstrebenswert, den Grad der Expression möglichst vieler, unterschiedlicher Gene zugleich auf einem Biochip analysieren zu können, weil hierdurch der Zeitaufwand für die Untersuchung der Genexpression von möglichst vielen Genen eines Genoms minimiert werden kann. Qualitative Aussagen über den Grad der Expression verschiedener Gene eines Genoms können so mit hoher Sicherheit getroffen werden können, da Aussagen über die Expressionsgrade vieler verschiedener Gene eines Genoms anhand eines Analyseexperimentes unter möglichst identischen Bedingungen getroffen werden können. Im Gegensatz hierzu wäre Aussage über die Expression der gleichen Gene eines Genoms, die man anhand von Analyseexperimenten treffen würde, die unterschiedlichen Bedingungen ausgesetzt sein können, weniger aussagekräftig. Dies wäre der Fall, wenn man die selbe Anzahl von Genexpressionen auf unterschiedlichen Biochips analysieren würde oder wenn man dieselbe Anzahl von Genexpressionen mit unterschiedlich ausgebildeten Sonden, bzw. Methoden ermitteln würde.

Um aussagekräftige Genexpressionsmuster möglichst vieler unterschiedlicher Gene anhand eines einzelnen Biochips treffen zu können, muss sichergestellt sein, dass die Oligonukleotidsequenzen, die an der Oberfläche des Biochips angebracht sind, geeignet sind, spezifisch mit einem Exprimierungsprodukt zu hybridisieren, das einem bestimmten Gen zugeordnet werden kann.

Wie oben erläutert wurde, hängt die Spezifität von Hybridisierungen neben der Komplementarität der Basensequenzen, die das Hybrid bilden, von der Länge der Hybride, ihrer individuellen Zusammensetzung und ihrer Schmelztemperatur ab.

Wenn man die Expression vieler Gene eines Genoms auf einmal untersucht, müssen demzufolge viele Sequenzen ähnliche Eigenschaften, wie Länge und Schmelztemperatur aufweisen, um die Ergebnisse miteinander vergleichen zu können.

Wenn man die Expression weniger Gene eines Genoms untersucht, müssen entsprechend wenige Sequenzen ähnliche Eigenschaften, wie Länge und Schmelztemperatur aufweisen.

Gleichwohl ist es wichtig, dass eine Sondensequenz auf einem Biochip spezifisch die Expression eines Genes aus der Gesamtmenge der exprimierten Gene eines Organismus detektiert.

Um solche Sondensequenzen zur Verfügung stellen zu können, ist es notwendig, dass das Genom des zu untersuchenden Organismus weitgehend bekannt ist.

In dem Genom eines Organismus lassen sich sogenannte Open Reading Frames (ORFs) identifizieren. Ein ORF ist ein Bereich innerhalb eines DNA-Moleküls, von dem man aufgrund einer bestimmten Anfangssequenz und einer bestimmten Endsequenz annimmt, dass er auf natürliche Weise grundsätzlich exprimierbar ist.

Ein Organismus dessen Genom bekannt ist, ist *Escherichia Coli,* ein Bakterium der *Enterobacteriaceae* Familie. Die Anwesenheit von *Escherichia Coli* und anderen Bakterien in der Darmflora ist notwendig für die physiologischen Funktionen des Verdauungssystems. Neben den nicht pathogenen *Escherichia Coli*-Stämmen existieren auch *Escherichia Coli*-Stämme, die für den Menschen infektiös sind. *Escherichia Coli* dient ferner seit langem als Modellorganismus zum Studium von grundlegenden zellulären und molekularen Prozessen, sowie zur Analyse von Reaktionen, mit denen Baktereien auf Umwelteinflüsse, physiologische Veränderungen oder Differenzierungsfaktoren reagieren. Ausserdem sind Populationen von *Escherichia Coli* von grosser Bedeutung für die klinische Herstellung von Aminosäuren und komplexen Enzymen und Proteinen wie Insulin, sowie eine wichtige Quelle für die Produktion von K- und B-Komplexvitaminen.

Unter den *Escherichia Coli*-Stämmen ist der *Escherichia Coli-*Stamm K12 als weit verbreiteter Modellorganismus etabliert, der als Referenzstamm für gram negative Bakterien verwendet wird. Um das Verhalten des Modellorganismus unter verschiedenen Bedingungen (Nährmedium, Temperatur, etc.) untersuchen zu können und/oder andere Organismen mit diesem vergleichen zu können, sollte die Untersuchung der Expression möglichst vieler Gene eines Organismus zugleich möglich sein.

Aufgabe der Erfindung ist es daher, eine Vorrichtung zum möglichst umfassenden Nachweis des Genoms von *Escherichia Coli* K12 zur Verfügung zu stellen.

Die Aufgabe wird durch einen Biochip gemäss Anspruch 1 gelöst, bei dem auf einem Träger Sonden vorgesehen sind, welche zum spezifischen Nachweis von Genen bzw. der Aktivität von Genen von *Escherichia Coli* K12 geeignet sind.

Bei dem erfindungsgemässen Biochip sind die Sondensequenzen Nukleotidsequenzen, und zumindest eine der Sonden weist Sondensequenzen auf, die identisch mit oder komplementär zu einem Abschnitt eines Open Reading Frames von *Escherichia Coli* K12 ist.

Nach einer bevorzugten Ausführungsform sind die Sondensequenzen auf einem erfindungsgemässen Biochip aus synthetischen Oligonukleotiden ausgebildet. Ein so ausgebildeter Biochip hat den Vorteil, dass die Länge der Sonden nach bestimmten Gesichtspunkten genau variiert werden kann, dass eine hohe Reinheit und damit Selektivität und Reaktivität der Sonden sowie Reproduzierbarkeit von mit einem solchen Biochip durchgeführten Experimenten besonders gut gewährleistet werden kann.

Nach einer bevorzugten Ausführungsform weist der Biochip 4.289 spezifische Sonden für 4.289 verschiedene Gene des Bakteriums *Escherichia Coli* K12 auf. Eine solche Ausführungsform hat den Vorteil, dass ein kompletter Satz der Gene des Genoms des Modellorganismus *Escherichia Coli* K12 zur Verfügung gestellt wird, und damit eine einzige Vorrichtung dazu verwendet wird, die Expression völlig unterschiedlicher Gene zugleich zu analysieren.

Eine weitere bevorzugte Ausführungsform eines erfindungsgemässen Biochips ist ein Biochip, der außer Sonden für Gene des Bakteriums *Escherichia Coli* K12 auch Sonden für Mutanten von *Escherichia Coli* K12 und/oder für andere *Escherichia Coli*-Stämme aufweist.

Solche Biochips erbringen den Vorteil, dass mit ihnen ein Vergleich der Genexpression im Modellorganismus *Escherichia Coli* K12 und in den entsprechenden Mutanten, bzw. anderen *Escherichia Coli*-Stämmen in ein und demselben Hybridisierungsexperiment durchgeführt werden kann. Darüber hinaus werden sich mit einem solchen Biochip neben qualitativen und quantitativen Aussagen über die jeweilige Genexpression auch solche über die Populationsdichte der gemessenen *Escherichia Coli-*Stämme oder Mutanten machen lassen. Ein solcher Biochip kann ausserdem für diagnostische Verfahren eingesetzt werden, z.B. wenn es darum geht, zu bestimmen, welche *Escherichia Coli*-Stämme in der Darmflora eines Patienten vorhanden sind.

Eine weitere bevorzugte Ausführungsform eines erfindungsgemässen Biochips ist ein Biochip, der ausser Sonden für Gene des Bakteriums *Escherichia Coli* K12 auch Sonden für andere Bakterien als *Escherichia Coli* K12 beinhaltet. Ein solcher Biochip weist den Vorteil auf, dass er für diagnostische Zwecke verwendbar ist, indem neben dem Vorhandensein des *Escherichia Coli* K12-Stammes in einer Probe auch das Vorhandensein anderer Bakterien nachgewiesen werden kann.

Weitere vorteilhafte Ausführungen gehen aus den Unteransprüchen hervor.

Durch ein Auswahlverfahren ist sichergestellt, dass die Sondensequenzen spezifisch mit Probenmaterial hybridisieren, das einem bestimmten Gen oder ORF der Art *Escherichia Coli* K12 zugeordnet werden kann.

Je nachdem, anhand welcher Exprimierungsprodukte das Expressionsmuster zu erstellen ist, sind die Sondensequenzen identisch mit oder komplementär zu Sequenzabschnitten aus dem Genom von *Escherichia Coli* K12.

### Die Erfindung wird im folgenden anhand der Figuren 1-5 beschrieben. In diesen zeigen

- Fig. 1: die Generierung von cDNA,
- Fig. 2: ein ORF bzw. ein cDNA-Molekül und dazu komplementäre Sondensequenzen,
- Fig. 3: das erfindungsgemässe Auswahlverfahren in einem Flussdiagramm,
- Fig. 4: einen schematischen Ausschnitt des erfindungsgemässen Biochips, und
- Fig. 5a) - c): jeweils schematisch die Abfolge der auf dem erfindungsgemässen Biochip ausgeführten Reaktionsschritte.

Ein erfindungsgemässer Biochip ermöglicht die Analyse von Genexpressionen von *Escherichia Coli* K12. Dabei wird nachgewiesen, welche Gene von *Escherichia Coli* K12 in welchem relativen Ausmass exprimiert worden sind.

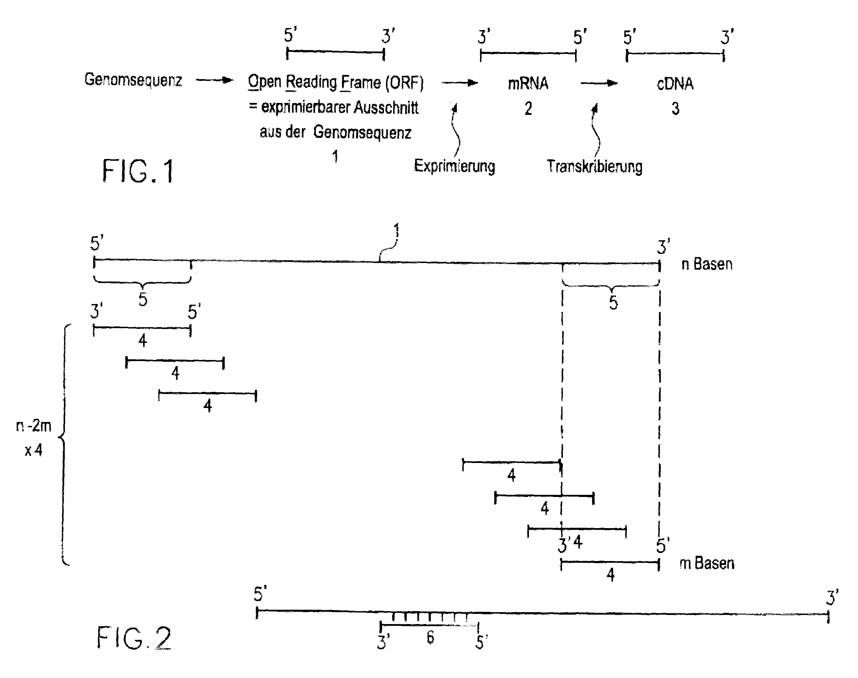

Ein Sequenzabschnitt aus dem Genom eines Organismus, der exprimiert wird, stellt ein Gen dar. Es handelt sich dabei in der Regel um ein ORF 1 (Open Reading Frame). Wenn ein ORF 1 in einem Schritt exprimiert wird, so wird aus diesem Gen oder ORF 1 eine bestimmte Menge mRNA 2 erzeugt, wobei diese Menge von verschiedenen Parametern abhängig ist (Fig. 1).

Bei der Erstellung eines Genexpressionsmusters wird festgestellt, ob mRNA 2 erzeugt worden ist und in welchem Ausmass dies geschehen ist. Zu diesem Zweck wird die erzeugte mRNA 2 aufbereitet, indem die erzeugte mRNA 2 revers in cDNA 3 transkribiert wird, wobei z.B. ein fluoreszierender cy-3- oder cy-5-Marker in die cDNA 3 eingebaut wird (Fig. 1).

Mit einer derart markierten cDNA 3 ist es in Verbindung mit einem erfindungsgemässen Biochip möglich, eine Aussage über die Menge an exprimierter mRNA 2 zu treffen. Dies wird nachfolgend erläutert.

Zu diesem Zweck wird die cDNA 3 mit einem erfindungsgemässen Biochip in Verbindung gebracht, auf dem Nukleotidsequenzen angebracht sind, die mit den cDNA-Molekülen 3 hybridisieren können.

Solche spezifischen Sequenzen lassen sich identifizieren, aus biologischem Material mittels PCR amplifizieren und auf einen Biochip aufbringen.

Eine bevorzugte Ausführungsform der Erfindung ist eine solche, bei der synthetische Oligonukleotide für die Herstellung der Sonden eines Biochips verwendet werden.

Die Oligonukleotidsequenzen, die dabei Verwendung finden, werden in einer durch Computerberechnungen gestützten Auswahl ausgewählt.

### 1. Computer-gestütztes Auswahlverfahren für die Auswahl von Sonden

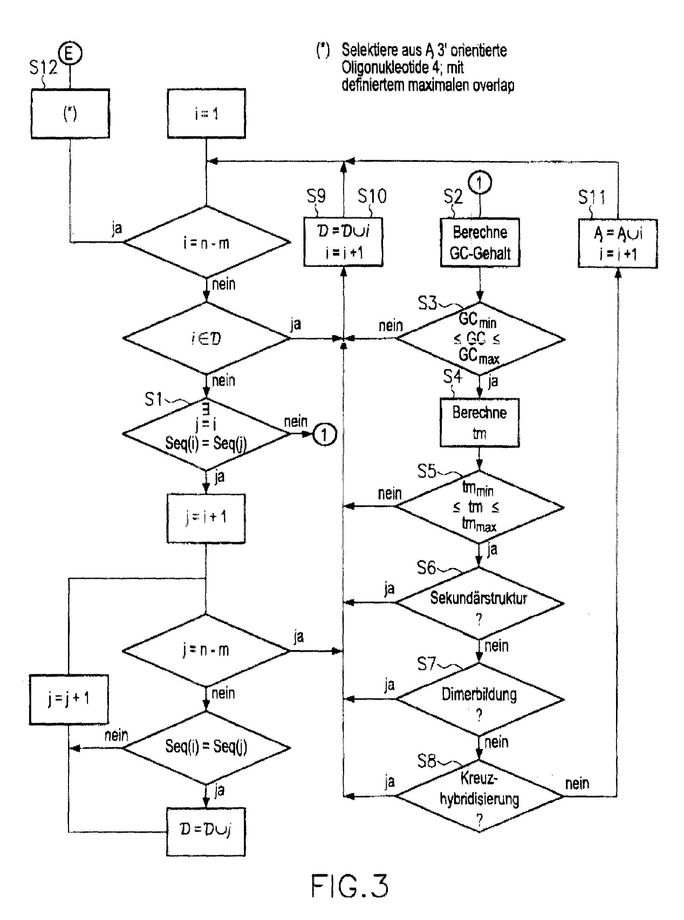

Dieses Auswahlverfahren wird im folgenden anhand der Figuren 2 und 3 erläutert.

Zunächst wird eine bestimmte Länge von m Basen für die Oligonukleotidsequenzen vorgegeben, die den wesentlichen Bestandteil von Sonden eines erfindungsgemässen Oligonukleotidchips bilden sollen.

Ein ORF 1, das n Basen lang ist, kann einen Sequenzabschnitt 5 enthalten, der m Basen lang ist. In einem n Basen langen ORF 1 gibt es n-m unterschiedliche Sequenzabschnitte 5 mit einer Länge von m Basen. Dementsprechend lassen sich n-m unterschiedliche Oligonukleotidsequenzen 4 identifizieren, die zu n-m Sequenzabschnitten 5 eines ORF 1 komplementär sind und eine Länge von m Basen aufweisen.

Für jedes ORF 1 aus dem Genom von *Escherichia Coli* mit einer Länge von n Basen, wobei n bei jedem ORF 1 unterschiedlich sein kann, aber n grösser ist als m, werden die n-m Oligonukleotidsequenzen 4 identifiziert, die komplementär zu n-m möglichen Sequenzabschnitten 5 eines ORF 1 von *Escherichia Coli* sind (Fig. 2). Zugleich werden Mengen 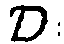 =Ø und 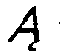 = Ø definiert.

In Schritt S1 wird für eine *i*-te Oligonukleotidsequenz 4_{*i*} (*i* = 1, 2, 3,... n-m) und *i* ∉ 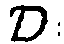 geprüft, ob Oligonukleotidsequenzen 4_{*j*} existieren mit Sequenz (4_{*i*}) = Sequenz (4_{*j*})_{*i*}, wobei *j* ≠ *i* und *j* = (i + 1, 2, ..., n-m).

Ist die Antwort ja, wird auf den Schritt S9 übergegangen, in dem das Oligonukleotid 4i verworfen wird. Alle *j* ≠ *i* werden der Menge 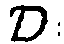 zugeordnet, das heisst 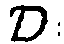 = 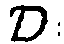 ∪ {/} für alle *j*≠*i*.

Ist das Ergebnis nein, wird mit Schritt S2 fortgefahren.

Im Schritt S2 wird für eine *i*-te Oligonukleotidsequenz 4_{*i*} (*i* = 1, 2, 3, ..., n-m) und *i* ∉ 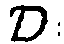 der Anteil von Guanin und Cytosin (GC-Gehalt) bestimmt.

Im Schritt S3 wird die Oligonukleotidsequenz 4_{*i*} dahingehend überprüft, ob sich der GC-Gehalt (GC) in einem vorbestimmten Bereich (zwischen GCₘᵢₙ und GCₘₐₓ) befindet. Dieser Bereich kann zum Beispiel zwischen 40 und 60% betragen.

Ist das Ergebnis nein, wird auf Schritt S9 übergegangen, in dem das Oligonukleotid 4_{*i*} verworfen wird. Hierauf folgt Schritt S10, mit dem das Prüfverfahren für das Oligonukleotid beendet wird.

Ist das Ergebnis ja, wird mit Schritt S4 fortgefahren.

Im Schritt S4 wird für eine *i*-te Oligonukleotidsequenz 4_{*i*} (*i* = 1, 2, 3, ..., n-m) und *i* ∉ 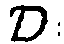 eine Schmelztemperatur tm berechnet.

In Schritt S5 wird das Oligonukleotid 4_{*i*} dahingehend überprüft, ob sich die berechnete Schmelztemperatur tm in einem vorbestimmten Bereich befindet. Dieser Bereich kann zum Beispiel zwischen tmₘᵢₙ = 65 °C und tmₘₐₓ = 75°C betragen.

Ist das Ergebnis nein, wird auf den Schritt S9 übergegangen, in dem das Oligonukleotid 4_{*i*} verworfen wird. Hierauf folgt Schritt S10, mit dem das Prüfverfahren für das Oligonukleotid 4_{*i*} beendet wird.

Ist das Ergebnis hingegen ja, wird auf Schritt S6 übergegangen, in dem überprüft wird, ob das Oligonukleotid 4_{*i*} Sekundärstrukturen ausbilden kann, die eine Hybridisierung des Oligonukleotides 4_{*i*} mit einem dazu komplementären Sequenzabschnitt 5_{*i*} erschweren oder unwahrscheinlich machen. Ist dies der Fall, wird auf die Schritte S9, S10 verzweigt, womit das Oligonukleotid 4; verworfen wird.

Ist die Antwort hingegen nein, wird mit Schritt S7 fortgefahren, in dem überprüft wird, ob das Oligonukleotid 4_{*i*} Dimere ausbilden kann, die eine Hybridisierung des Oligonukleotides 4_{*i*} mit einem dazu komplementären Sequenzabschnitt 5_{*i*} erschweren oder unwahrscheinlich machen. Ist dies der Fall, wird auf die Schritte S9, S10 verzweigt, womit das Oligonukleotid 4_{*i*} verworfen wird.

Ist die Antwort hingegen ja, wird in Schritt S8 überprüft, ob eine Kreuzhybridisierung zwischen dem Oligonukleotid 4_{*i*} und einem Sequenzabschnitt 5_{*j*} aus einem anderen ORF 1 als dem, anhand dessen Sequenzabschnitt 5_{*i*} das Oligonukleotid 4_{*i*} ursprünglich identifiziert wurde, möglich ist. Ist die Antwort ja, wird auf die Schritte S9 und S10 verzweigt.

Ist die Antwort nein, wird mit Schritt S11 fortgefahren, in dem Oligonukleotide 4_{*i*} in eine Liste übernommen werden ( 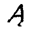 = 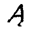 ∪ {4_{*i*}}).

In Schritt S12 werden aus der Menge der Oligonukleotide 4ᵢ ∈ 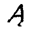 beispielsweise 3 Oligonukleotide so selektiert, dass sie am weitesten im 3'-Bereich des ORF 1 hybridisieren und einander maximal mit einem vorgegebenen Prozentsatz überlappen.

Die Schritte S1 bis S12 werden für jedes *i*-te mögliche Oligonukleotid 4_{*i*} (mit *i* = 1, 2, 3, ..., n-m) eines ORF 1 vorgenommen. Dieses Auswahlverfahren wird wiederum für alle *i* möglichen Oligonukleotide der anderen identifizierten ORFs von *Escherichia Coli* durchgeführt

Im Rahmen der Erfindung kann es auch zweckmässig sein, Sequenzabschnitte 5_{*i*} anstelle der Oligonukleotidsequenzen 4_{*i*} auszuwählen. Dies kann der Fall sein, wenn man anstelle von cDNA 3 mRNA 2 mit einem erfindungsgemässen Oligonukleotidchip nachweisen möchte. Die Schritte S1 bis S12 lassen sich daher analog für die Auswahl von Sequenzabschnitten 5_{*i*} durchführen.

### 2. Aufbau eines erfindungsgemässen Biochips

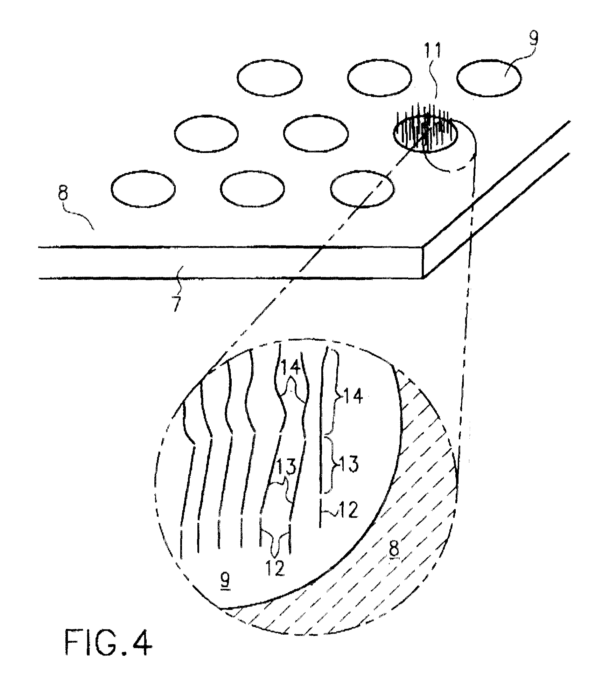

Ein Ausschnitt aus einem erfindungsgemässen Biochips ist in Figur 4 dargestellt. Ein erfindungsgemässer Biochip ist ein Träger 7 mit einer Trägeroberfläche 8, auf der in einem geordneten Raster in Zeilen und Reihen Sondenpunkte 9 angebracht sind, wobei jeder Sondenpunkt 9 eine Vielzahl identischer Sondenmoleküle 10 aufweist. Die Sondenmoleküle 10 eines Sondenpunktes 9 bilden eine Sonde 11. Jedes Sondenmolekül 10 setzt sich zusammen aus einem Linker 12, der eine kovalente Bindung des Sondenmoleküls an die Trägeroberfläche 8 ermöglicht, einem Spacer 13, und einer Sondensequenz 14.

Ein 5'-Amino-modifiziertes Oligonukleotid am 5'-Ende des Spacers 13 ist ein Linker 12.

Der Spacer 13 ermöglicht die Einhaltung eines Abstandes zwischen der Trägeroberfläche 8 und der Sondensequenz 14. Ein geeigneter Spacer ist zum Beispiel ein C₆-Oligonukleotid, das aus sechs dCTP-Monomeren aufgebaut ist.

Es sind 5'-Amino-modifizierte Oligonukleotide vorgesehen, die zwischen der Amino-Modifikation, die den Linker 12 darstellt und der Sondensequenz 14 einen C₆-spacer 13 aufweisen, der aus sechs dCTP-Monomeren aufgebaut ist. Bei der Herstellung des Biochips werden die Oligonukleotide zu einer Konzentration von 100µM in 50µM Na-Borat (pH 8,5) und 250 mM NaCl gelöst, auf silylierte Objektträger des Typs CSS der Fa. CEL Associates, Houston USA, mittels eines Affymetrix 417 Arrayers aufgespottet, in einer Feuchtekammer rehydratisiert und bei Raumtemperatur getrocknet. Anschliessend wird die Bildung der kovalenten Bindung zwischen Linker und Trägeroberfläche durch eine Reduktion mit NaBH₄ abgeschlossen.

Eine Oligonukleotidsequenz 4, die mit Hilfe des oben erläuterten Verfahrens ausgewählt wird, kann eine Sondensequenz sein. Die Sondensequenz ist mit dem 5'-Ende an den Spacer 13 gebunden. Die Sondensequenzen 14 einer Sonde 11 unterscheiden sich von den Sondensequenzen 14 anderer Sonden 11. Innerhalb eines Sondenpunktes 9 sind nur identische Sonden 11 vorgesehen. Im Rahmen der Erfindung ist es jedoch auch mögliche, unterschiedliche Sondensequenzen 14 innerhalb einer Sonde 11 vorzusehen.

Im Rahmen der Erfindung ist es ferner möglich, andere Spacer, Linker, Trägeroberflächen und Arten der kovalenten Bindung vorzusehen, ebenso wie es möglich ist, Sonden auf andere Arten als durch Aufspotten auf einem Träger anzuordnen.

### 3. Ausführungsbeispiel

Auf die unter 1. beschriebene Weise wird für jedes von 4.289 ORFs im Genom von *Escherichia Coli* K12 eine Oligonukleotidsequenz 4 ermittelt, die als Sondensequenz 14 eingesetzt wird.

Die ermittelten Sondensequenzen 14 sind komplementär zu Sequenzabschnitten 5 aus dem Genom von *Escherichia Coli* K12, die im beiliegenden Sequenzprotokoll angegeben sind.

Das Sequenzprotokoll liegt sowohl in Papierform als auch in Form einer auf einem maschinenlesbaren Datenträger gespeicherten elektronischen Datei als Anlage anbei. Die im Protokoll verwendeten Kennziffern in Brackett-Klammern < > entsprechen den Kennziffern des WIPO-Standards Nr. 25. Dabei definieren die Kennziffern
- <160>: die Gesamtzahl der Sequenzbereiche, innerhalb derer ermittelte Sequenzabschnitte 5 liegen, deren Komplement als Sondensequenz 14 auf einem erfindungsgemässen Oligonukleotidchip aufgebracht sein kann,
- <210>: jeweils einen Sequenzbereich SB innerhalb dessen ein Sequenzabschnitt 5 liegen kann; weist jedem Sequenzbereich eine Sequenzidentitätsnummer zu,
- <220>: den Abschnitt des Genoms von *Escherichia Coli* K12 dem der Sequenzbereich <210> zuzuordnen ist; dieser Abschnitt entspricht zugleich einem von 4.289 ORFs 1 aus dem Genom von *Escherichia Coli* K12,

- <222>: die Position des Sequenzbereiches <210> innerhalb des Abschnittes <220>
- <223>: die Position des Abschnittes <220> innerhalb des Genoms von *Escherichia Coli* K12, und
- <313>: einen Kernbereich des jeweiligen Sequenzbereichs <210>, dessen Komplement Bestandteil einer Sondensequenz 14 ist.

In dem Sequenzprotokoll sind für 4.289 unterschiedliche ORFs 1 jeweils bis zu drei Sequenzbereiche <210> angegeben, wobei die bis zu drei für ein ORF 1 (<220>) angegebenen Sequenzbereiche <210> sich jeweils von allen anderen angegebenen Sequenzbereichen <210> der übrigen ORFs 1 (<220>) des Genoms von *Escherichia Coli* K12 unterscheiden. Die zu einem ORF 1 gehörenden Sequenzbereiche <210> sind im Sequenzprotokoll unmittelbar nacheinander angegeben.

Ein Abschnitt einer vorbestimmten Länge eines der Sequenzbereiche <210> ist für das jeweilige ORF 1 spezifisch. Im vorliegenden Beispiel beträgt die Mindestlänge 20 Basen.

Als Sondensequenzen werden Abschnitte bevorzugt, die die 20 Basen der Kernbereiche gemäss der Kenziffer <313> der jeweiligen Sequenzbereiche <210> umfassen. Diese Kernbereiche oder deren Komplemente bilden besonders bevorzugte Oligonukleotidsequenzen 4 bzw. Sondensequenzen 14 (Fig. 4), da sie spezifisch sind.

Grundsätzlich ist es jedoch auch möglich, insbesondere auch längere Bereiche, die nur einen Teil der Kernbereiche <313> oder keinen Teil der Kernbereiche <313> umfassen, als Sondensequenzen zu verwenden. In der Regel sind derartige Sequenzen auch spezifisch, wenn die Anzahl der Basen zumindest 40 beträgt.

Zur vereinfachten Darstellung werden nachfolgend die Sequenzbereiche <210> durch SB (Sequenzbereich) gefolgt von ihrer im Sequenzprotokoll nach der Kennziffer <210> angegebenen Sequenzidentitätsnummer und die Kernbereiche <313> durch KB (Kernbereich) gefolgt von der Sequenzidentitätsnummer des zugehörigen Sequenzbereiches angegeben.

Beim erfindungsgemässen Ausführungsbeispiel sind anhand des Sequenzprotokolls 4.289, 40 Basen lange Oligonukleotidsequenzen 4 ausgewählt, die das in Fig. 3 dargestellte Prüfungsverfahren durchlaufen haben und die komplementär zu 4.289 Sequenzabschnitten 5 der ersten im Sequenzprotokoll angegebenen Sequenzbereiche der jeweiligen ORFs 1 (<220>) sind, wobei jede Oligonukleotidsequenz 4 komplementär zu einem Sequenzabschnitt 5 des jeweiligen ORFs 1 (<220>) ist, der 9 Basen vor dem jeweiligen Kernbereich KB beginnt und 11 Basen nach diesem Teilabschnitt endet.

Auf einem Träger sind insgesamt 4.416 Sonden aufgebracht. 4.289 dieser Sonden besitzen 40 Basen lange Sondensequenzen 14, die den oben beschriebenen 4.289 Oligonukleotidsequenzen 4 entsprechen. Die übrigen 127 Sonden sind Kontrollsonden. 79 davon sind Replikate von Komplementen von Abschnitten des Genoms von *Escherichia Coli* K12, die immer hybridisieren (Positivkontrolle), die übrigen 48 Sonden solche, die Sondensequenzen aufweisen, die ungeeignet sind, mit einem Transkribierungsprodukt aus dem exprimierten Genom von *Escherichia Coli* K12 zu hybridisieren *(Arabidopsis*-Negativkontrollen).

Jede der 4.289 Sonden hybridisiert mit einem unterschiedlichen cDNA-Molekül 3, das aus der jeweils aus einem ORF 1 (<220>) exprimierten mRNA 2 revers transkribiert wird.

Die Sonden sind auf dem Träger auf einer Fläche von 2×2 cm² wie oben beschrieben aufgebracht.

Im Rahmen der Erfindung können auch andere Sondensequenzen 14 als die im Ausführungsbeispiel angegebenen Oligonukleotidsequenzen 4 auf dem Biochip aufgebracht sein, solange sie Teil eines für die jeweiligen ORFs 1 (<220>) angegebenen Sequenzbereiches <210> sind.

Eine vorteilhafte Ausführungsform eines erfindungsgemässen Biochips ist ein Biochip, auf dem Sondensequenzen für den Nachweis von Probenmaterial (z.B. cDNA 3 oder mRNA 2) aus *Escherichia Coli* K12 Kulturen vorgesehen sind und auf dem zusätzlich Sonden zum Nachweis von Mutationen von *Escherichia Coli* K12 innerhalb dieses Probenmaterial vorgesehen sind.

Eine vorteilhafte Ausführungsform eines erfindungsgemässen Biochips ist ein Biochip, auf dem Sondensequenzen für den Nachweis von Probenmaterial (z.B. cDNA 3 oder mRNA 2) aus *Escherichia Coli* K12 Kulturen vorgesehen sind und auf dem zusätzlich Sonden zum Nachweis anderer Bakterienstämme innerhalb des Probenmaterials vorgesehen sind.

Dies eröffnet weitergehende Auswahlmöglichkeiten für spezielle Ausbildungen eines erfindungsgemässen Biochips. Will man zum Beispiel die Expression einer bestimmten Gengruppe bevorzugt untersuchen, so lassen sich die jeweiligen Sondensequenzen wie oben beschrieben von den für diese Gengruppe angegebenen Sequenzbereichen aus dem Sequenzprotokoll ableiten und im Experiment oder in einer Berechnung gesondert optimieren.

Zum Beispiel erlaubt die freie Wahl der Länge der Sondensequenzen 14, der bevorzugten Schmelztemperatur der berechneten Hybride 6 und der für die jeweiligen Berechnungen eingesetzten Berechnungsprogramme ein genaueres Angleichen.

Demnach kann es auch zweckmässig sein, Oligonukleotidsequenzen mit unterschiedlichen Längen in einzelnen Sondenpunkten vorzusehen.

Im Rahmen der Erfindung können aber auch andere Arten von Zuordnungen zu Gengruppen vorgenommen werden.

Eine weitere Möglichkeit der Optimierung besteht in einem experimentellen Vergleich von verschiedenen für ein ORF1 durch das oben beschriebene Verfahren ausgewählten Sondensequenzen 14.

### 4. Beispiel für die Anwendung eines erfindungsgemässen Biochips

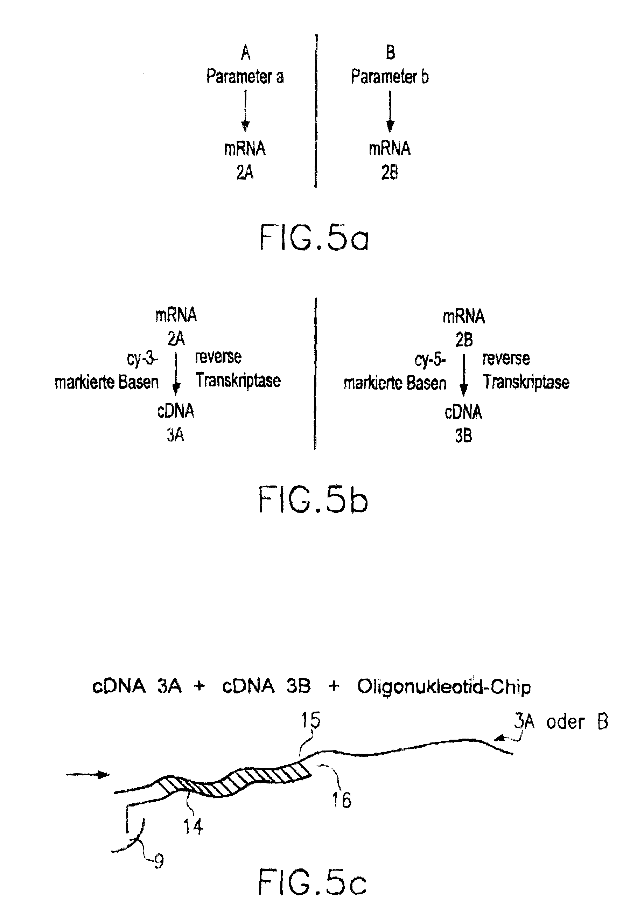

Die Anwendung eines erfindungsgemässen Biochips in einem Verfahren zur Analyse eines Genexpressionsmusters von *Escherichia Coli* K12 wird im folgenden anhand der Figuren 5a)-c) erläutert.

Es werden zwei Nährlösungen A und B mit *Escherichia Coli* K12 angelegt, wobei Nährlösung A anderen Bedingungen ausgesetzt ist als Nährlösung B. Bei den unterschiedlichen Bedingungen handelt es sich um die Parameter, deren Auswirkung auf die Genexpression untersucht wird. Solche Parameter können zum Beispiel Temperatur, Konzentration und /oder besondere Inhaltstoffe der Nährlösungen sein (Fig. 5a)).

In einem ersten Schritt wird die jeweilige mRNA 2 der Nährlösungen A und B in cDNA 3 transkribiert, wobei in das eine cDNA-Transkript cy-5-markierte Basen mit eingebaut werden und in das andere cDNA-Transkript cy-3-markierte Basen eingebaut werden (Fig. 5b)).

Die beiden cDNA-Proben 3A und 3B werden mit einem erfindungsgemäßen Biochip in Kontakt gebracht (Fig. 5c)).

Diejenigen cDNA-Moküle 3, welche einen Sequenzabschnitt 15 aufweisen, der komplementär zu einer der auf dem Biochip vorhandenen Sondensequenzen 14 ist, bilden ein Hybrid 16 (Fig. 5c)).

Diejenigen cDNA-Moleküle 3, die keinen Sequenzabschnitt 15 aufweisen, der zu einer der auf dem Biochip vorhandenen Sondensequenzen 14 komplementär ist, oder die sich auf dem Biochip nicht in räumlicher Nähe zu einer Sondensequenz 14 befinden, zu der ein solcher Sequenzabschnitt 15 komplementär ist, werden durch Waschen von der Oberfläche des Biochips entfernt.

Die Menge an gebildetem Hybrid 16 in jedem einzelnen Sondenpunkt 8 lässt sich nun mit Hilfe eines Fluoreszenzspektrometers nachweisen. Dabei wird die Intensität der Fluoreszenz gemessen. Dies geschieht bei einer für den jeweiligen Marker spezifischen Wellenlänge. Wird ein ORF 1 von *Escherichia Coli* K12 nur in einem der beiden Nährmedien A oder B exprimiert, wird Fluoreszenz nur bei der Wellenlänge detektiert, die für den Marker spezifisch ist, der bei der Synthese der entsprechenden cDNA 3 eingebaut wird. Wird ein ORF 1 von *Escherichia Coli* K12 in beiden Nährmedien exprimiert, wird Fluoreszenz bei Wellenlängen detektiert, die für die Marker spezifisch ist, die bei der Synthese von cDNA 3 verwendet werden. Die im Ausführungsbeispiel verwendeten Marker sind cy-3 und cy-5. Diese beiden Marker fluoreszieren bei unterschiedlichen Wellenlängen. Es ist deshalb möglich, in jedem einzelnen Sondenpunkt, der eine detektierbare Mindestmenge an Hybrid 16 aufweist, einen Intensitätswert für die Fluoreszenz getrennt nach Wellenlänge zu ermitteln. Die Messung der Fluoreszenz erfolgt daher in zwei sogenannten Kanälen:
- einem cy-3-Kanal, mit dem die Fluoreszenzintensität cy-3 markierter cDNA 3A gemessen wird, die mit Sondensequenzen 14 auf dem Biochip Hybride 16A gebildet haben, und
- einem cy-5-Kanal, mit dem die Fluoreszenzintensität cy-5-markierter cDNA 3B gemessen wird, die mit Sonden auf dem Biochip Hybride 16B gebildet haben.

Gemessen wird im jeweiligen Kanal die Fluoreszenzintensität jedes einzelnen Sondenpunktes 8. Gemessen wird ausserdem das sogenannte Hintergrundrauschen, dass heisst die Fluoreszenzintensität der Biochipoberfläche in Bereichen zwischen den Sondenpunkten 8. Die Differenz dieser beiden Messwerte ergibt einen korrigierten Wert für die Fluoreszenzintensität des cDNA-Moleküls 3, das in dem gemessenen Sondenpunkt 8 ein Hybrid 16 gebildet hat und mit dem entsprechenden Marker versehen ist. Dieser korrigierte Intensitätswert ist proportional zur Menge an von *Escherichia Coli* K12 in einem der beiden Nährmedien exprimiertem ORF 1, für das die gemessene Sonde 11 spezifisch ist.

Im Messkanal, in dem die Werte ermittelt werden, die zu diesem korrigierten Intensitätswert führen, wird spezifisch die Intensität der Fluoreszenz eines dem Messkanal entsprechenden Markers gemessen, und damit dessen relative Konzentration. Liegt der korrigierte Intensitätswert über 0, beziehungsweise hebt sich die Intensität der in einem Sondenpunkt gemessenen Fluoreszenz genügend vom Hintergrund ab, so liegt ein qualitativer Nachweis über das Vorhandensein von mit einem bestimmten Marker versehener cDNA 3 in dem entsprechenden Sondenpunkt 8 vor.

Die Gesamtheit der in einem Kanal ermittelten Intensitätswerte entspricht einem Genexpressionsmuster, das Auskunft über die Expressionen derjenigen ORFs 1 aus *Escherichia Coli* K12 gibt, für die spezifische Sonden 9 auf dem Biochip vorgesehen sind. Das Genexpressionsmuster ist abhängig von den Bedingungen, denen die jeweilige Probe *Escherichia Coli* K12 vor der Transkribierung der mRNA 2 ausgesetzt ist. Man geht davon aus, dass der überwiegende Teil der Gene, bzw. ORFs 1 eines Organismus, das heisst ca. 85%, unter verschiedenen Bedingungen gleich exprimiert wird. Würde man also mehrere Expressionsmuster des vollständigen Genoms eines Organismus ermitteln, würden zwei unterschiedliche Expressionsmuster jeweils nur einen Teil von Genen aufweisen, die in unterschiedlichen Konzentrationen vorliegen.

Die hohe Zahl an unterschiedlichen Sonden auf einem Biochip gestattet es, die in den verschiedenen Kanälen ermittelten Genexpressionsmuster direkt miteinander zu vergleichen. Zwischen den beiden Kanälen können Unterschiede in der jeweils gemessenen Intensität zurückgehen auf:
a) unterschiedliche Marker,
b) Schwankungen der Konzentrationen von Gesamt-mRNA-Populationen A und B aus zwei Experimenten A und B, die nicht auf Expressionsunterschiede zurückzuführen sind,
c) unterschiedliche Expressionen eines ORFs 1 unter verschiedenen Bedingungen. Weil die überwiegende Zahl der ORFs 1 von *Escherichia Coli*K12 unter verschiedenen Bedingungen gleich exprimiert wird, lassen sich die Intensitätsunterschiede, die durch die Punkte a) und b) der eben aufgeführten Auflistung entstehen können, mit Hilfe einer Normalisierung der ermittelten Intensitätswerte ausgleichen. Nach diesem Ausgleich werden die Unterschiede zwischen zwei Genexpressionsmustern deutlich. Würde man dasselbe Experiment auf einem Biochip mit wenigen Sonden durchführen, dann wäre aus statistischen Gründen ein solcher Ausgleich hingegen kaum möglich. Es ist deshalb zweckmässig, mindestens 1000, vorzugsweise mehr als 3000 Sonden für jeweils unterschiedliche Gene auf einem Biochip vorzusehen.

Eine solche Normalisierung lässt sich zum Beispiel wie folgt vornehmen:
- es wird jeweils die Summe über alle in einem Messkanal ermittelten Intensitätswerte gebildet,
- die grössere der ermittelten Summen wird durch die kleinere der ermittelten Summen dividiert, woraus ein Faktor resultiert, und
- der ermittelten Intensitätswerte, aus denen die kleinere Summe zustande gekommen ist, werden mit dem Faktor multipliziert.

Die Auswertung erfolgt, indem die Differenzen zwischen den mit dem Faktor multiplizierten ermittelten Intensitätswerten des insgesamt intensitätsschwächeren Kanals und den ermittelten Intensitätswerten des anderen Kanals gebildet werden, und zwar für jeden einzelnen Sondepunkt.

Die Differenzen zwischen den normalisierten, ermittelten Intensitätswerten gestatten es, eine qualitative Aussage über die unterschiedliche Expression eines Gens unter verschiedenen Bedingungen zu machen. Die ermittelten Intensitätswerte erlauben ausserdem eine Aussage darüber, wie viel mehr ein bestimmtes Gen unter Bedingungen A exprimiert wurde als unter Bedingungen B. Somit kann mit einem erfindungsgemässen Biochip eine quantitative Aussage mittels Skalierung getroffen werden.

Im Rahmen der Erfindung ist es auch möglich, ein Genexpressionsmuster von *Escherichia Coli* K12 zu erstellen, in dem lediglich eine *Escherichia Coli* K12 Kultur untersucht wird. Man kann die in den Sondenpunkten auf einem erfindungsgemässen Biochip gemessenen Intensitäten dann qualitativ zueinander in Bezug setzen und/oder sie mit Expressionsmustern in einer Bibliothek vergleichen.

Im Rahmen der Erfindung ist es ferner möglich, für *Escherichia Coli* K12 optimierte Sonden mit für weitere *Escherichia Coli* -Stämme oder *Escherichia Coli* K12-Mutanten optimierten Sonden auf einen Chip aufzubringen, bzw. die hierfür vorgesehenen Sonden nach dem obigen Verfahren gemeinsam zu optimieren.

## Patentansprüche

1. Biochip mit einer Vielzahl von Sondenpunkten, wobei in jedem Sondenpunkt eine Sonde aufgebracht ist, jede Sonde eine Vielzahl von Sondenmolekülen aufweist, und alle Sondenmoleküle einer Sonde eine identische Sondensequenz besitzen, wobei
- die Sondensequenzen Nukleotidsequenzen sind, und
- zumindest eine der Sonden Sondensequenzen mit einer Länge von mindestens 20 Basen aufweist, die identisch mit oder komplementär zu einem Abschnitt eines Open Reading Frames von *Escherichia Coli* K12 ist.

2. Biochip gemäss Anspruch 1,
dadurch gekenzeichnet,
dass die Nukleotidsequenzen aus synthetischen Oligonukleotiden ausgebildet sind.

3. Biochip gemäss Anspruch 1 oder 2,
**dadurch gekennzeichnet,**
**dass** mehrere Sondensequenzen einen Bereich aufweisen, der jeweils identisch mit oder komplementär zu einem Abschnitt der Sequenzbereiche SB 1 - SB 12812 ist.

4. Biochip gemäss einem der Ansprüche 1 - 3,
**dadurch gekennzeichnet,**
**dass** mehrere Sondensequenzen einen Bereich aufweisen, der jeweils identisch mit oder komplementär zu einem der Kernbereiche KB 1 - KB 12812 ist.

5. Biochip nach einem der Ansprüche 1 - 4,
**dadurch gekennzeichnet,**
**dass** zumindest eine Sondensequenz mit einer Basenlänge von zumindest 20 Basen komplementär zu oder identisch mit einem Sequenzabschnitt der Kernbereiche KB 1-KB 12812 ist.

6. Biochip nach einem der Ansprüche 1 - 5,
**dadurch gekennzeichnet,**
**dass** die Sondensequenzen eine Basenlänge von mehr als 30 Basen aufweisen.

7. Biochip nach einem der Ansprüche 1 - 6,
**dadurch gekennzeichnet,**
**dass** die Sondensequenzen eine Basenlänge von mehr als 40 Basen aufweisen.

8. Biochip nach einem der Ansprüche 1 - 7,
**dadurch gekennzeichnet,**
**dass** die Sondensequenzen eine Basenlänge von 30-80 Basen aufweisen.

9. Biochip gemäss einem der Ansprüche 1 - 8,
wobei der Biochip für mehr als zehn unterschiedliche Open Reading Frames von *Escherichia Coli* K12 Sonden aufweist, deren Sondensequenz identisch mit oder komplementär zu einem Abschnitt jeweils eines Open Reading Frames sind.

10. Biochip gemäss einem der Ansprüche 1 - 9,
wobei der Oligonukleotidchip für mehr als 100 unterschiedliche Open Reading Frames von *Escherichia Coli* K12 Sonden aufweist, deren Sondensequenz identisch mit oder komplementär zu einem Abschnitt jeweils eines Open Reading Frames sind.

11. Biochip gemäss einem der Ansprüche 1 - 9,
wobei der Biochip für mehr als 1000 unterschiedliche Open Reading Frames von E*scherichia Coli* K12 Sonden aufweist, deren Sondensequenz identisch mit oder komplementär zu einem Abschnitt jeweils eines Open Reading Frames sind.

12. Biochip gemäss einem der Ansprüche 1 - 9,
wobei der Biochip für mehr als 1000-4000 unterschiedliche Open Reading Frames von *Escherichia Coli* K12 Sonden aufweist, deren Sondensequenz identisch mit oder komplementär zu einem Abschnitt jeweils eines Open Reading Frames sind.

13. Biochip gemäss einem der Ansprüche 1 - 9,
wobei der Biochip für mehr als 4000 unterschiedliche Open Reading Frames von E*scherichia Coli* K12 Sonden aufweist, deren Sondensequenz identisch mit oder komplementär zu einem Abschnitt jeweils eines Open Reading Frames sind.

## Geänderte Patentansprüche

### Geänderte Patentansprüche gemäss Regel 86(2) EPÜ.

**1.** Biochip mit einer Vielzahl von Sondenpunkten, wobei in jedem Sondenpunkt eine Sonde aufgebracht ist, jede Sonde eine Vielzahl von Sondenmolekülen aufweist, und alle Sondenmoleküle einer Sonde eine identische Sondensequenz besitzen, wobei
- die Sondensequenzen Nukleotidsequenzen sind,
- die Sondensequenzen eine Basenlänge von 30-80 Basen aufweisen,
- die Nukleotidsequenzen aus ex situ synthetisierten Oligonukleotiden ausgebildet sind, und
- zumindest eine der Sonden Sondensequenzen aufweist, die identisch mit order Komplementär zu einem Abschnitt eines Open Reading Frames von *Escherichia Coli* K12 ist.

**2.** Biochip gemäss Anspruch 1
**dadurch gekennzeichnet,**
**dass** mehrere Sondensequenzen einen Bereich aufweisen, der jeweils identisch mit oder komplementär zu einem Abschnitt der Sequenzbereiche SB 1 - SB 12812 ist.

**3.** Biochip gemäss einem der Ansprüche 1 oder 2,
**dadurch gekennzeichnet,**
**dass** mehrere Sondensequenzen einen Bereich aufweisen, der jeweils identisch mit oder komplementär zu einem der Kernbereiche KB 1 - KB 12812 ist.

**4.** Biochip nach einem der Ansprüche 1 - 3,
**dadurch gekennzeichnet,**
**dass** zumindest eine Sondensequenz mit einer Basenlänge von zumindest 20 Basen komplementär zu oder identisch mit einem Sequenzabschnitt der Kernbereiche KB 1 - KB 12812 ist.

**5.** Biochip nach einem der Ansprüche 1 - 4,
**dadurch gekennzeichnet,**
**dass** die Sondensequenzen eine Basenlänge von mehr als 30 Basen aufweisen.

**6.** Biochip nach einem der Ansprüche 1 - 5,
**dadurch gekennzeichnet,**
**dass** die Sondensequenzen eine Basenlänge von mehr als 40 Basen aufweisen.

**7.** Biochip gemäss einem der Ansprüche 1 - 6,
wobei der Biochip für mehr als zehn unterschiedliche Open Reading Frames von *Escherichia Coli* K12 Sonden aufweist, deren Sondensequenz identisch mit oder komplementär zu einem Abschnitt jeweils eines Open Reading Frames sind.

**8.** Biochip gemäss einem der Ansprüche 1 - 6,
wobei der Oligonukleotidchip für mehr als 100 unterschiedliche Open Reading Frames von *Escherichia Coli* K12 Sonden aufweist, deren Sondensequenz identisch mit oder komplementär zu einem Abschnitt jeweils eines Open Reading Frames sind.

**9.** Biochip gemäss einem der Ansprüche 1 - 6,
wobei der Biochip für mehr als 1000 unterschiedliche Open Reading Frames von *Escherichia Coli* K12 Sonden aufweist, deren Sondensequenz identisch mit oder komplementär zu einem Abschnitt jeweils eines Open Reading Frames sind.

**10.** Biochip gemäss einem der Ansprüche 1 - 6,
wobei der Biochip für mehr als 1000-4000 unterschiedliche Open Reading Frames von *Escherichia Coli* K12 Sonden aufweist, deren Sondensequenz identisch mit oder komplementär zu einem Abschnitt jeweils eines Open Reading Frames sind.

**11.** Biochip gemäss einem der Ansprüche 1 - 6,
wobei der Biochip für mehr als 4000 unterschiedliche Open Reading Frames von *Escherichia Coli* K12 Sonden aufweist, deren Sondensequenz identisch mit oder komplementär zu einem Abschnitt jeweils eines Open Reading Frames sind.
